# EUROPEAN PATENT APPLICATION

(11) **EP 1 130 094 A2**
(43) Date of publication of application: **05.09.2001**
(21) Application number: 00114089.6
(22) Date of filing: 07.07.2000
(51) Int. Cl.: C12N 15/12, C12N 15/11, C12N 15/10, C12N 15/70, C12N 15/85, C12N 5/10, C12N 1/21, C07K 14/47, C07K 16/18, C12Q 1/68

(54) **Primers for synthesizing full length cDNA clones and their use**

(30) Priority: 08.07.1999 JP 19448699; 11.01.2000 JP 2000118774; 02.05.2000 JP 2000183765
(71) Applicant: Helix Research Institute, Kisarazu-shi, Chiba 292-0812 (JP)
(72) Inventor: Ota, Toshio, Fujisawa-shi, Kanagawa 251-0042 (JP); Nishikawa, Tetsuo, Tokyo 173-0013 (JP); Isogai, Takao, Inashiki-gun, Ibaraki 300-0303 (JP); Hayashi, Koji, Ichihara-shi, Chiba 299-0125 (JP); Ishii, Shizuko, Kisarazu-shi, Chiba 292-0812 (JP); Kawai, Yuri, Kisarazu-shi, Chiba 292-0812 (JP); Wakamatsu, Ai, Kisarazu-shi, Chiba 292-0014 (JP); Sugiyama, Tomoyasu, Kisarazu-shi, Chiba 292-0045 (JP); Nagai, Keiichi, Higashiyamato-shi, Tokyo 207-0022 (JP); Kojima, Shinichi, Kisarazu-shi, Chiba 292-0052 (JP); Otsuki, Tetsuji, Kisarazu-shi, Chiba 292-0055 (JP); Koga, Hisashi, Kisarazu-shi, Chiba 292-0055 (JP)
(74) Representative: VOSSIUS & PARTNER

(57) **Abstract**

Primers for synthesizing full length cDNAs and their use are provided.

830 cDNA encoding a human protein has been isolated and nucleotide sequences of 5'-, and 3'-ends of the cDNA have been determined. Furthermore, primers for synthesizing the full length cDNA have been provided to clarify the function of the protein encoded by the cDNA. The full length cDNA of the present invention containing the translation start site provides information useful for analyzing the functions of the protein.

## Description

### FIELD OF THE INVENTION

The present invention relates to a polynucleotide encoding a novel protein, a protein encoded by the polynucleotide, and new uses of these.

### BACKGROUND OF THE INVENTION

Currently, the sequencing projects, the determination and analysis of the genomic DNA of various living organisms have been in progress all over the world. The whole genomic sequences of more than 10 species of prokaryotes, a lower eukaryote, yeast, and a multicellular eukaryote, C. elegans are already determined. As to human genome, which is supposed to be composed of three thousand million base pairs, the world wide cooperative projects have been under way to analyze it, and the whole structure is predicted to be determined by the years 2002-2003. The aim of the determination of genomic sequence is to reveal the functions of all genes and their regulation and to understand living organisms as a network of interactions between genes, proteins, cells or individuals through deducing the information in a genome, which is a blueprint of the highly complicated living organisms. To understand living organisms by utilizing the genomic information from various species is not only important as an academic subject, but also socially significant from the viewpoint of industrial application.

However, determination of genomic sequences itself cannnot identify the functions of all genes. For example, as for yeast, only the function of approximately half of the 6000 genes, which is predicted based on the genomic sequence, was able to be deduced. As for human, the number of the genes is predicted to be approximately one hundred thousand. Therefore, it is desirable to establish "a high throughput analysis system of the gene functions" which allows us to identify rapidly and efficiently the functions of vast amounts of the genes obtained by the genomic sequencing.

Many genes in the eukaryotic genome are split by introns into multiple exons. Thus, it is difficult to predict correctly the structure of encoded protein solely based on genomic information. In contrast, cDNA, which is produced from mRNA that lacks introns, encodes a protein as a single continuous amino acid sequence and allows us to identify the primary structure of the protein easily. In human cDNA research, to date, more than one million ESTs (Expression Sequence Tags) are publicly available, and the ESTs presumably cover not less than 80% of all human genes.

The information of ESTs is utilized for analyzing the structure of human genome, or for predicting the exon-regions of genomic sequences or their expression profile. However, many human ESTs have been derived from proximal regions to the 3'-end of cDNA, and information around the 5'-end of mRNA is extremely little. Among these human cDNAs, the number of the corresponding mRNAs whose encoding protein sequences are deduced is approximately 7000, and further, the number of full-length therein is only 5500. Thus, even including cDNA registered as EST, the percentage of human cDNA obtained so far is estimated to be 10-15% of all the genes.

It is possible to identify the transcription start site of mRNA on the genomic sequence based on the 5'-end sequence of a full-length cDNA, and to analyze factors involved in the stability of mRNA that is contained in the cDNA, or in its regulation of expression at the translation stage. Also, since a full-length cDNA contains ATG, the translation start site, in the 5'-region, it can be translated into a protein in a correct frame. Therefore, it is possible to produce a large amount of the protein encoded by the cDNA or to analyze biological activity of the expressed protein by utilizing an appropriate expression system. Thus, analysis of a full-length cDNA provides valuable information which complements the information from genome sequencing. Also, full-length cDNA clones that can be expressed are extremely valuable in empirical analysis of gene function and in industrial application.

In particular, human secretory proteins or membrane proteins are would be useful by itself as a medicine like tissue plasminogen activator (TPA), or as a target of medicines like membrane receptors. In addition, genes for signal transduction-associated proteins (protein kinases, etc.), glycoprotein-associated proteins, transcription-associated proteins, and disease-associated proteins form a gene group rich in genes whose relationships to human diseases have been elucidated.

Therefore, it has great significance to isolate novel full-length cDNA clones of human, only few of which has been isolated. Especially, isolation of a novel cDNA clone encoding a secretory protein or membrane protein is desired since the protein itself would be useful as a medicine, and also the clones potentially include a gene associated with diseases. In addition, genes encoding proteins that are associated with signal transduction, glycoprotein, transcription, or diseases are expected to be useful as target molecules for therapy, or as medicines themselves. These genes form a gene group predicted to be strongly associated with diseases. Thus, identification of the full-length cDNA clones encoding those proteins has great significance.

### SUMMARY OF THE INVENTION

An objective of the present invention is to provide a primer that enables synthesizing polynucleotide from human, the resulting polynucleotide or its clone, and a protein encoded by the polynucleotide.

The inventors have developed a method for efficiently cloning a human full-length cDNA that is predicted by the ATGpr etc. to be a full-length cDNA clone, from a full-length-enriched cDNA library that is synthesized by the oligo-capping method. Then, the inventors determined the nucleotide sequence of the obtained cDNA clones from both 5'- and 3'- ends. By utilizing the sequences, the inventors selected clones that were expected to contain a signal by the PSORT (Nakai K. and Kanehisa M. (1992) Genomics 14: 897-911), and obtained clones that contain a cDNA encoding a secretory protein or membrane protein. Moreover, the inventors specifically selected full-length cDNA clones that encode secretory or membrane proteins, signal transduction-associated proteins, glycoprotein-associated proteins, transcription-associated proteins, or disease-associated proteins from clones homologous to the clones in the SwissProt (http://www.ebi.ac.uk/ebi_docsSwissProt_db/swisshome.html) according to the keywords of SwissProt.

The full-length cDNA clones of the present invention have high fullness ratio since these were obtained by the combination of (1) construction of a full-length-enriched cDNA library that is synthesized by the oligo-capping method, and (2) a system in which fullness ratio is evaluated from the nucleotide sequence of the 5'-end (in this system, clones are selected based on the estimation by the ATGpr, following the removal of sequences judged not to be full-length when compared with ESTs). However, the primers of the present invention enable obtaining full-length cDNA easily without any special methods mentioned above.

Homology analysis in which the analysis is carried out against a non-full-length cDNA fragment to postulate the function of a protein encoded by said fragment, is being commonly performed. However, since such analysis is based on the information of the fragment, it is not clear as to whether this fragment corresponds to a part that is functionally important in the protein. In other words, the reliability of the homology analysis based on the information of a fragment is doubtful, as information relating to the structure of the whole protein is not available. However, the homology analysis of the present invention is conducted based on the information of a full-length cDNA comprising the whole coding region of the cDNA, and therefore, the homology of various portions of the protein can be analyzed. Hence, the reliability of the homology analysis has been dramatically improved in the present invention.

The inventors completed the invention by finding that it is possible to synthesize a novel full-length cDNA by using the combination of a primer that is designed based on the nucleotide sequence of the 5'-ends of the selected full-length cDNA clones and any of an oligo-dT primer or a 3'-primer that is designed based on the nucleotide sequence of the 3'-ends of the selected clones.

Thus, the present invention relates to primers described below, a method for synthesizing a polynucleotide using the primers, and polynucleotides obtained by the method.

First, the present invention relates to
(1) use of an oligonucleotide as a primer for synthesizing the polynucleotide comprising the nucleotide sequence set forth in any one of SEQ ID NOs: 1-829 and 2545, or the complementary strand thereof, wherein said oligonucleotide is complementary to said polynucleotide or the complementary strand thereof and comprises at least 15 nucleotides;
(2) a primer set for synthesizing polynucleotides, the primer set comprising an oligo-dT primer and an oligonucleotide complementary to the complementary strand of the polynucleotide comprising the nucleotide sequence set forth in any one of SEQ ID NOs: 1-829 and 2545, wherein said oligonucleotide comprises at least 15 nucleotides; and
(3) A primer set for synthesizing polynucleotides, the primer set comprising a combination of an oligonucleotide comprising a nuclectide sequence complementary to the complementary strand of the polynucleotide comprising a 5'-end nucleotide sequence and an oligonucleotide comprising a nucleotide sequence complementary to the polynucleotide comprising a 3'-end nucleotide sequence, wherein said oligonucleotides comprise at least 15 nucleotides and wherein said combination of 5'-end nucleotide sequence / 3'-end nucleotide sequence is selected from the combinations of 5'-end nucleotide sequence / 3'-end nucleotide sequence set forth in the SEQ ID NOs in Table 1.

Table 1 shows names of clones obtained in the examples described later, comprising the polynucleotide of the present invention (830 clones), names of nucleotide sequences at the 5'-end and 3'-end of the full-length cDNA, and their corresponding SEQ ID NOs. A blank indicates that the of the 3'-end sequence corresponding to the 5'-end sequence has not been determined the same clone.

The SEQ ID NO of a 5'-end sequence is shown on the right side of the name of the 5'-end sequence, and the SEQ ID NO of a 3'-end sequence is shown on the right side of the name of the 3'-end sequence.

**Table 1**

| Correspondence between names of clone and the sequence name, and the SEQ ID. | | | | |
|---|---|---|---|---|
| Name of clone | Name of 5'-sequence | SEQ ID | 3 Name of 3'-sequence | SEQ ID |
| BNGH41000020 | F-BNGH41000020 | 1 | | |
| BNGH41000087 | F-BNGH41000087 | 2 | | |
| BNGH41000091 | F-BNGH41000091 | 3 | | |
| HEMBA1000006 | F-HEMBA1000006 | 4 | R-HEMBA1000006 | 830 |
| HEMBA1000121 | F-HEMBA1000121 | 5 | R-HEMBA1000121 | 831 |
| HEMBA1000128 | F-HEMBA1000128 | 6 | R-HEMBA1000128 | 832 |
| HEMBA1000275 | F-HEMBA1000275 | 7 | R-HEMBA1000275 | 833 |
| HEMBA1000300 | F-HEMBA1000300 | 8 | R-HEMBA1000300 | 834 |
| HEMBA1000349 | F-HEMBA1000349 | 9 | R-nnnnnnnnnnnn | 835 |
| HEMBA1000443 | F-HEMBA1000443 | 10 | | |
| HEMBA1000462 | F-HEMBA1000462 | 11 | R-HEMBA1000462 | 836 |
| HEMBA1000477 | F-HEMBA1000477 | 12 | R-HEMBA1000477 | 837 |
| HEMBA1000590 | F-HEMBA1000590 | 13 | R-HEMBA1000590 | 838 |
| HEMBA1000634 | F-HEMBA1000634 | 14 | R-HEMBA1000634 | 839 |
| HEMBA1000671 | F-HEMBA1000671 | 15 | R-HEMBA1000671 | 840 |
| HEMBA1000713 | F-HEMBA1000713 | 16 | R-HEMBA1000713 | 841 |
| HEMBA1000732 | F-HEMBA1000732 | 17 | R-HEMBA1000732 | 842 |
| HEMBA1000745 | F-HEMBA1000745 | 18 | R-nnnnnnnnnnnn | 843 |
| HEMBA1000835 | F-HEMBA1000835 | 19 | | |
| HEMBA1000875 | F-HEMBA1000875 | 20 | R-HEMBA1000875 | 844 |
| HEMBA1000907 | F-HEMBA1000907 | 21 | | |
| HEMBA1000940 | F-HEMBA1000940 | 22 | R-HEMBA1000940 | 845 |
| HEMBA1000962 | F-HEMBA1000962 | 23 | R-HEMBA1000962 | 846 |
| HEMBA1001184 | F-HEMBA1001184 | 24 | R-HEMBA1001184 | 847 |
| HEMBA1001221 | F-HEMBA1001221 | 25 | R-HEMBA1001221 | 848 |
| HEMBA1001228 | F-HEMBA1001228 | 26 | R-HEMBA1001228 | 849 |
| HEMBA1001272 | F-HEMBA1001272 | 27 | R-HEMBA1001272 | 850 |
| HEMBA1001296 | F-HEMBA1001296 | 28 | R-HEMBA1001296 | 851 |
| HEMBA1001297 | F-HEMBA1001297 | 29 | R-HEMBA1001297 | 852 |
| HEMBA1001390 | F-HEMBA1001390 | 30 | R-HEMBA1001390 | 853 |
| HEMBA1001563 | F-HEMBA1001563 | 31 | R-HEMBA1001563 | 854 |
| HEMBA1001621 | F-HEMBA1001621 | 32 | R-HEMBA1001621 | 855 |
| HEMBA1001878 | F-HEMBA1001878 | 33 | R-HEMBA1001878 | 856 |
| HEMBA1001886 | F-HEMBA1001886 | 34 | R-HEMBA1001886 | 857 |
| HEMBA1002048 | F-HEMBA1002048 | 35 | R-HEMBA1002048 | 858 |
| HEMBA1002131 | F-HEMBA1002131 | 36 | R-HEMBA1002131 | 859 |
| HEMBA1002163 | F-HEMBA1002163 | 37 | R-HEMBA1002163 | 860 |
| HEMBA1002164 | F-HEMBA1002164 | 38 | | |
| HEMBA1002167 | F-HEMBA1002167 | 39 | R-HEMBA1002167 | 861 |
| HEMBA1002178 | F-HEMBA1002178 | 40 | R-HEMBA1002178 | 862 |
| HEMBA1002195 | F-HEMBA1002195 | 41 | R-HEMBA1002195 | 863 |
| HEMBA1002227 | F-HEMBA1002227 | 42 | R-HEMBA1002227 | 864 |
| HEMBA1002239 | F-HEMBA1002239 | 43 | | |
| HEMBA1002316 | F-HEMBA1002316 | 44 | R-HEMBA1002316 | 865 |
| HEMBA1002420 | F-HEMBA1002420 | 45 | R-HEMBA1002420 | 866 |
| HEMBA1002421 | F-HEMBA1002421 | 46 | R-HEMBA1002421 | 867 |
| HEMBA1002524 | F-HEMBA1002524 | 47 | R-HEMBA1002524 | 868 |
| HEMBA1002551 | F-HEMBA1002551 | 48 | R-HEMBA1002551 | 869 |
| HEMBA1002767 | F-HEMBA1002767 | 49 | R-HEMBA1002767 | 870 |
| HEMBA1002985 | F-HEMBA1002985 | 50 | R-HEMBA1002985 | 871 |
| HEMBA1002992 | F-HEMBA1002992 | 51 | | |
| HEMBA1003047 | F-HEMBA1003047 | 52 | R-HEMBA1003047 | 872 |
| HEMBA1003072 | F-HEMBA1003072 | 53 | R-HEMBA1003072 | 873 |
| HEMBA1003101 | F-HEMBA1003101 | 54 | R-HEMBA1003101 | 874 |
| HEMBA1003120 | F-HEMBA1003120 | 55 | R-HEMBA1003120 | 875 |
| HEMBA1003230 | F-HEMBA1003230 | 56 | R-HEMBA1003230 | 876 |
| HEMBA1003294 | F-HEMBA1003294 | 57 | R-HEMBA1003294 | 877 |
| HEMBA1003315 | F-HEMBA1003315 | 58 | R-HEMBA1003315 | 878 |
| HEMBA1003392 | F-HEMBA1003392 | 59 | R-HEMBA1003392 | 879 |
| HEMBA1003399 | F-HEMBA1003399 | 60 | R-HEMBA1003399 | 880 |
| HEMBA1003487 | F-HEMBA1003487 | 61 | R-HEMBA1003487 | 881 |
| HEMBA1003497 | F-HEMBA1003497 | 62 | R-HEMBA1003497 | 882 |
| HEMBA1003530 | F-HEMBA1003530 | 63 | R-HEMBA1003530 | 883 |
| HEMBA1003602 | F-HEMBA1003602 | 64 | R-HEMBA1003602 | 884 |
| HEMBA1003732 | F-HEMBA1003732 | 65 | R-HEMBA1003732 | 885 |
| HEMBA1003945 | F-HEMBA1003945 | 66 | R-HEMBA1003945 | 886 |
| HEMBA1004007 | F-HEMBA1004007 | 67 | R-HEMBA1004007 | 887 |
| HEMBA1004067 | F-HEMBA1004067 | 68 | | |
| HEMBA1004085 | F-HEMBA1004085 | 69 | R-HEMBA1004085 | 888 |
| HEMBA1004110 | F-HEMBA1004110 | 70 | R-nnnnnnnnnnnn | 889 |
| HEMBA1004250 | F-HEMBA1004250 | 71 | R-HEMBA1004250 | 890 |
| HEMBA1004391 | F-HEMBA1004391 | 72 | R-HEMBA1004391 | 891 |
| HEMBA1004444 | F-HEMBA1004444 | 73 | R-HEMBA1004444 | 892 |
| HEMBA1004454 | F-HEMBA1004454 | 74 | R-HEMBA1004454 | 893 |
| HEMBA1004505 | F-HEMBA1004505 | 75 | R-HEMBA1004505 | 894 |
| HEMBA1004785 | F-HEMBA1004785 | 76 | R-HEMBA1004785 | 895 |
| HEMBA1004797 | F-HEMBA1004797 | 77 | R-HEMBA1004797 | 896 |
| HEMBA1004952 | F-HEMBA1004952 | 78 | R-HEMBA1004952 | 897 |
| HEMBA1004971 | F-HEMBA1004971 | 79 | R-HEMBA1004971 | 898 |
| HEMBA1004982 | F-HEMBA1004982 | 80 | R-HEMBA1004982 | 899 |
| HEMBA1005070 | F-HEMBA1005070 | 81 | R-HEMBA1005070 | 900 |
| HEMBA1005084 | F-HEMBA1005084 | 82 | R-HEMBA1005084 | 901 |
| HEMBA1005145 | F-HEMBA1005145 | 83 | R-HEMBA1005145 | 902 |
| HEMBA1005230 | F-HEMBA1005230 | 84 | R-HEMBA1005230 | 903 |
| HEMBA1005246 | F-HEMBA1005246 | 85 | R-HEMBA1005246 | 904 |
| HEMBA1005267 | F-HEMBA1005267 | 86 | R-HEMBA1005267 | 905 |
| HEMBA1005337 | F-HEMBA1005337 | 87 | R-HEMBA1005337 | 906 |
| HEMBA1005430 | F-HEMBA1005430 | 88 | R-HEMBA1005430 | 907 |
| HEMBA1005449 | F-HEMBA1005449 | 89 | R-HEMBA1005449 | 908 |
| HEMBA1005489 | F-HEMBA1005489 | 90 | R-HEMBA1005489 | 909 |
| HEMBA1005522 | F-HEMBA1005522 | 91 | R-HEMBA1005522 | 910 |
| HEMBA1005545 | F-HEMBA1005545 | 92 | R-HEMBA1005545 | 911 |
| HEMBA1005698 | F-HEMBA1005698 | 93 | R-HEMBA1005698 | 912 |
| HEMBA1005913 | F-HEMBA1005913 | 94 | R-HEMBA1005913 | 913 |
| HEMBA1005929 | F-HEMBA1005929 | 95 | R-HEMBA1005929 | 914 |
| HEMBA1005945 | F-HEMBA1005945 | 96 | R-HEMBA1005945 | 915 |
| HEMBA1006016 | F-HEMBA1006016 | 97 | R-HEMBA1006016 | 916 |
| HEMBA1006171 | F-HEMBA1006171 | 98 | R-HEMBA1006171 | 917 |
| HEMBA1006276 | F-HEMBA1006276 | 99 | R-HEMBA1006276 | 918 |
| HEMBA1006299 | F-HEMBA1006299 | 100 | R-HEMBA1006299 | 919 |
| HEMBA1006311 | F-HEMBA1006311 | 101 | R-HEMBA1006311 | 920 |
| HEMBA1006335 | F-HEMBA1006335 | 102 | R-HEMBA1006335 | 921 |
| HEMBA1006357 | F-HEMBA1006357 | 103 | R-HEMBA1006357 | 922 |
| HEMBA1006430 | F-HEMBA1006430 | 104 | R-HEMBA1006430 | 923 |
| HEMBA1006482 | F-HEMBA1006482 | 105 | R-HEMBA1006482 | 924 |
| HEMBA1006517 | F-HEMBA1006517 | 106 | R-HEMBA1006517 | 925 |
| HEMBA1006544 | F-HEMBA1006544 | 107 | R-HEMBA1006544 | 926 |
| HEMBA1006572 | F-HEMBA1006572 | 108 | R-HEMBA1006572 | 927 |
| HEMBA1006658 | F-HEMBA1006658 | 109 | R-HEMBA1006658 | 928 |
| HEMBA1006707 | F-HEMBA1006707 | 110 | R-HEMBA1006707 | 929 |
| HEMBA1006724 | F-HEMBA1006724 | 111 | R-HEMBA1006724 | 930 |
| HEMBA1006749 | F-HEMBA1006749 | 112 | R-HEMBA1006749 | 931 |
| HEMBA1006770 | F-HEMBA1006770 | 113 | R-HEMBA1006770 | 932 |
| HEMBA1006902 | F-HEMBA1006902 | 114 | R-HEMBA1006902 | 933 |
| HEMBA1006912 | F-HEMBA1006912 | 115 | R-HEMBA1006912 | 934 |
| HEMBA1006916 | F-HEMBA1006916 | 116 | R-HEMBA1006916 | 935 |
| HEMBA1006960 | F-HEMBA1006960 | 117 | R-HEMBA1006960 | 936 |
| HEMBA1007013 | F-HEMBA1007013 | 118 | R-HEMBA1007013 | 937 |
| HEMBA1007057 | F-HEMBA1007057 | 119 | R-HEMBA1007057 | 938 |
| HEMBA1007063 | F-HEMBA1007063 | 120 | R-HEMBA1007063 | 939 |
| HEMBA1007226 | F-HEMBA1007226 | 121 | | |
| HEMBA1007241 | F-HEMBA1007241 | 122 | R-HEMBA1007241 | 940 |
| HEMBA1007291 | F-HEMBA1007291 | 123 | R-HEMBA1007291 | 941 |
| HEMBA1007332 | F-HEMBA1007332 | 124 | R-HEMBA1007332 | 942 |
| HEMBB1000106 | F-HEMBB1000106 | 125 | R-HEMBB1000106 | 943 |
| HEMBB1000276 | F-HEMBB1000276 | 126 | R-HEMBB1000276 | 944 |
| HEMBB1000309 | F-HEMBB1000309 | 127 | R-HEMBB1000309 | 945 |
| HEMBB1000407 | F-HEMBB1000407 | 128 | R-HEMBB1000407 | 946 |
| HEMBB1000447 | F-HEMBB1000447 | 129 | R-HEMBB1000447 | 947 |
| HEMBB1000542 | F-HEMBB1000542 | 130 | R-HEMBB1000542 | 948 |
| HEMBB1000567 | F-HEMBB1000567 | 131 | R-HEMBB1000567 | 949 |
| HEMBB1000642 | F-HEMBB1000642 | 132 | R-HEMBB1000642 | 950 |
| HEMBB1000668 | F-HEMBB1000668 | 133 | R-HEMBB1000668 | 951 |
| HEMBB1000679 | F-HEMBB1000679 | 134 | R-HEMBB1000679 | 952 |
| HEMBB1000881 | F-HEMBB1000881 | 135 | R-HEMBB1000881 | 953 |
| HEMBB1000905 | F-HEMBB1000905 | 136 | R-HEMBB1000905 | 954 |
| HEMBB1001026 | F-HEMBB1001026 | 137 | R-HEMBB1001026 | 955 |
| HEMBB1001048 | F-HEMBB1001048 | 138 | R-HEMBB1001048 | 956 |
| HEMBB1001200 | F-HEMBB1001200 | 139 | R-HEMBB1001200 | 957 |
| HEMBB1001407 | F-HEMBB1001407 | 140 | R-HEMBB1001407 | 958 |
| HEMBB1001530 | F-HEMBB1001530 | 141 | R-HEMBB1001530 | 959 |
| HEMBB1001547 | F-HEMBB1001547 | 142 | R-HEMBB1001547 | 960 |
| HEMBB1001573 | F-HEMBB1001573 | 143 | R-HEMBB1001573 | 961 |
| HEMBB1001847 | F-HEMBB1001847 | 144 | R-HEMBB1001847 | 962 |
| HEMBB1001959 | F-HEMBB1001959 | 145 | R-HEMBB1001959 | 963 |
| HEMBB1001978 | F-HEMBB1001978 | 146 | R-HEMBB1001978 | 964 |
| HEMBB1002039 | F-HEMBB1002039 | 147 | R-HEMBB1002039 | 965 |
| HEMBB1002041 | F-HEMBB1002041 | 148 | R-HEMBB1002041 | 966 |
| HEMBB1002051 | F-HEMBB1002051 | 149 | R-HEMBB1002051 | 967 |
| HEMBB1002120 | F-HEMBB1002120 | 150 | R-HEMBB1002120 | 968 |
| HEMBB1002162 | F-HEMBB1002162 | 151 | R-HEMBB1002162 | 969 |
| HEMBB1002228 | F-HEMBB1002228 | 152 | R-HEMBB1002228 | 970 |
| HEMBB1002245 | F-HEMBB1002245 | 153 | R-HEMBB1002245 | 971 |
| HEMBB1002302 | F-HEMBB1002302 | 154 | R-HEMBB1002302 | 972 |
| HEMBB1002427 | F-HEMBB1002427 | 155 | R-HEMBB1002427 | 973 |
| HEMBB1002465 | F-HEMBB1002465 | 156 | R-HEMBB1002465 | 974 |
| HEMBB1002661 | F-HEMBB1002661 | 157 | R-HEMBB1002661 | 975 |
| HEMBB1002663 | F-HEMBB1002663 | 158 | R-HEMBB1002663 | 976 |
| HEMBB1002693 | F-HEMBB1002693 | 159 | R-HEMBB1002693 | 977 |
| MAMMA 1000046 | F-MAMMA1000046 | 160 | R-MAMMA1000046 | 978 |
| MAMMA1000102 | F-MAMMA1000102 | 161 | R-MAMMA1000102 | 979 |
| MAMMA 1000106 | F-MAMMA1000106 | 162 | R-MAMMA1000106 | 980 |
| MAMMA1000118 | F-MAMMA1000118 | 163 | R-MAMMA1000118 | 981 |
| MAMMA1000141 | F-MAMMA1000141 | 164 | R-MAMMA1000141 | 982 |
| MAMMA1000204 | F-MAMMA1000204 | 165 | R-MAMMA1000204 | 983 |
| MAMMA1000226 | F-MAMMA1000226 | 166 | R-MAMMA1000226 | 984 |
| MAMMA1000403 | F-MAMMA1000403 | 167 | R-MAMMA1000403 | 985 |
| MAMMA1000449 | F-MAMMA1000449 | 168 | R-MAMMA1000449 | 986 |
| MAMMA1000457 | F-MAMMA1000457 | 169 | R-MAMMA1000457 | 987 |
| MAMMA1000473 | F-MAMMA1000473 | 170 | R-MAMMA1000473 | 988 |
| MAMMA1000496 | F-MAMMA1000496 | 171 | R-MAMMA1000496 | 989 |
| MAMMA1000528 | F-MAMMA1000528 | 172 | R-MAMMA1000528 | 990 |
| MAMMA1000591 | F-MAMMA1000591 | 173 | R-MAMMA1000591 | 991 |
| MAMMA 1000614 | F-MAMMA1000614 | 174 | R-MAMMA1000614 | 992 |
| MAMMA1000652 | F-MAMMA1000652 | 175 | R-MAMMA1000652 | 993 |
| MAMMA1000681 | F-MAMMA1000681 | 176 | R-MAMMA1000681 | 994 |
| MAMMA 1000706 | F-MAMMA1000706 | 177 | R-MAMMA1000706 | 995 |
| MAMMA 1000788 | F-MAMMA1000788 | 178 | R-MAMMA1000788 | 996 |
| MAMMA 1000810 | F-MAMMA1000810 | 179 | R-MAMMA1000810 | 997 |
| MAMMA1000814 | F-MAMMA1000814 | 180 | R-MAMMA1000814 | 998 |
| MAMMA1000881 | F-MAMMA1000881 | 181 | R-MAMMA1000881 | 999 |
| MAMMA1000986 | F-MAMMA1000986 | 182 | R-MAMMA1000986 | 1000 |
| MAMMA 1000994 | F-MAMMA1000994 | 183 | R-MAMMA1000994 | 1001 |
| MAMMA1001043 | F-MAMMA1001043 | 184 | R-MAMMA1001043 | 1002 |
| MAMMA1001066 | F-MAMMA1001066 | 185 | R-MAMMA1001066 | 1003 |
| MAMMA1001094 | F-MAMMA1001094 | 186 | R-MAMMA1001094 | 1004 |
| MAMMA1001141 | F-MAMMA1001141 | 187 | R-MAMMA1001141 | 1005 |
| MAMMA1001150 | F-MAMMA1001150 | 188 | R-MAMMA1001150 | 1006 |
| MAMMA1001237 | F-MAMMA1001237 | 189 | R-MAMMA1001237 | 1007 |
| MAMMA1001284 | F-MAMMA1001284 | 190 | R-MAMMA1001284 | 1008 |
| MAMMA1001310 | F-MAMMA1001310 | 191 | R-MAMMA1001310 | 1009 |
| MAMMA1001344 | F-MAMMA1001344 | 192 | | |
| MAMMA1001418 | F-MAMMA1001418 | 193 | R-MAMMA1001418 | 1010 |
| MAMMA1001532 | F-MAMMA1001532 | 194 | R-MAMMA1001532 | 1011 |
| MAMMA1001609 | F-MAMMA1001609 | 195 | R-MAMMA1001609 | 1012 |
| MAMMA 1001615 | F-MAMMA1001615 | 196 | R-MAMMA1001615 | 1013 |
| MAMMA 1001623 | F-MAMMA1001623 | 197 | R-MAMMA1001623 | 1014 |
| MAMMA1001634 | F-MAMMA1001634 | 198 | R-MAMMA1001634 | 1015 |
| MAMMA 1001893 | F-MAMMA1001893 | 199 | R-MAMMA1001893 | 1016 |
| MAMMA1001901 | F-MAMMA1001901 | 200 | R-MAMMA1001901 | 1017 |
| MAMMA 1001957 | F-MAMMA1001957 | 201 | R-MAMMA1001957 | 1018 |
| MAMMA1001978 | F-MAMMA1001978 | 202 | R-MAMMA1001978 | 1019 |
| MAMMA1002070 | F-MAMMA1002070 | 203 | R-MAMMA1002070 | 1020 |
| MAMMA1002080 | F-MAMMA1002080 | 204 | R-MAMMA1002080 | 1021 |
| MAMMA 1002087 | F-MAMMA1002087 | 205 | R-MAMMA1002087 | 1022 |
| MAMMA1002091 | F-MAMMA1002091 | 206 | | |
| MAMMA 1002095 | F-MAMMA1002095 | 207 | R-MAMMA1002095 | 1023 |
| MAMMA 1002128 | F-MAMMA1002128 | 208 | R-MAMMA1002128 | 1024 |
| MAMMA1002142 | F-MAMMA1002142 | 209 | R-MAMMA1002142 | 1025 |
| MAMMA1002165 | F-MAMMA1002165 | 210 | R-MAMMA1002165 | 1026 |
| MAMMA 1002205 | F-MAMMA1002205 | 211 | R-MAMMA1002205 | 1027 |
| MAMMA 1002224 | F-MAMMA1002224 | 212 | R-MAMMA1002224 | 1028 |
| MAMMA1002234 | F-MAMMA1002234 | 213 | R-MAMMA1002234 | 1029 |
| MAMMA1002586 | F-MAMMA1002586 | 214 | R-MAMMA1002586 | 1030 |
| MAMMA1002633 | F-MAMMA1002633 | 215 | R-MAMMA1002633 | 1031 |
| MAMMA1003126 | F-MAMMA1003126 | 216 | R-MAMMA1003126 | 1032 |
| NT2RM1000407 | F-NT2RM1000407 | 217 | | |
| NT2RM1000462 | F-NT2RM1000462 | 218 | | |
| NT2RM1000542 | F-NT2RM1000542 | 219 | | |
| NT2RM1000580 | F-NT2RM1000580 | 220 | | |
| NT2RM1000789 | F-NT2RM1000789 | 221 | | |
| NT2RM1000855 | F-NT2RM1000855 | 222 | | |
| NT2RM1000858 | F-NT2RM1000858 | 223 | | |
| NT2RM1000899 | F-NT2RM1000899 | 224 | | |
| NT2RM2000241 | F-NT2RM2000241 | 225 | | |
| NT2RM2000306 | F-NT2RM2000306 | 226 | | |
| NT2RM2000410 | F-NT2RM2000410 | 227 | | |
| NT2RM2000423 | F-NT2RM2000423 | 228 | | |
| NT2RM2000497 | F-NT2RM2000497 | 229 | | |
| NT2RM2000514 | F-NT2RM2000514 | 230 | | |
| NT2RM2000565 | F-NT2RM2000565 | 231 | | |
| NT2RM2000582 | F-NT2RM2000582 | 232 | | |
| NT2RM2000589 | F-NT2RM2000589 | 233 | | |
| NT2RM2000622 | F-NT2RM2000622 | 234 | | |
| NT2RM2000632 | F-NT2RM2000632 | 235 | | |
| NT2RM2000773 | F-NT2RM2000773 | 236 | | |
| NT2RM2001126 | F-NT2RM2001126 | 237 | | |
| NT2RM2001558 | F-NT2RM2001558 | 238 | | |
| NT2RM2001626 | F-NT2RM2001626 | 239 | | |
| NT2RM2001643 | F-NT2RM2001643 | 240 | | |
| NT2RM2001738 | F-NT2RM2001738 | 241 | | |
| NT2RM2001767 | F-NT2RM2001767 | 242 | | |
| NT2RM2001792 | F-NT2RM2001792 | 243 | | |
| NT2RM2001818 | F-NT2RM2001818 | 244 | | |
| NT2RM2001902 | F-NT2RM2001902 | 245 | | |
| NT2RM2001939 | F-NT2RM2001939 | 246 | | |
| NT2RM2001941 | F-NT2RM2001941 | 247 | | |
| NT2RM4000100 | F-NT2RM4000100 | 248 | R-NT2RM4000100 | 1033 |
| NT2RM4000115 | F-NT2RM4000115 | 249 | R-NT2RM4000115 | 1034 |
| NT2RM4000198 | F-NT2RM4000198 | 250 | R-NT2RM4000198 | 1035 |
| NT2RM4000284 | F-NT2RM4000284 | 251 | R-NT2RM4000284 | 1036 |
| NT2RM4000295 | F-NT2RM4000295 | 252 | R-NT2RM4000295 | 1037 |
| NT2RM4000326 | F-NT2RM4000326 | 253 | R-NT2RM4000326 | 1038 |
| NT2RM4000417 | F-NT2RM4000417 | 254 | R-NT2RM4000417 | 1039 |
| NT2RM4000444 | F-NT2RM4000444 | 255 | R-NT2RM4000444 | 1040 |
| NT2RM4000587 | F-NT2RM4000587 | 256 | R-NT2RM4000587 | 1041 |
| NT2RM4000593 | F-NT2RM4000593 | 257 | R-NT2RM4000593 | 1042 |
| NT2RM4000648 | F-NT2RM4000648 | 258 | R-NT2RM4000648 | 1043 |
| NT2RM4000761 | F-NT2RM4000761 | 259 | R-NT2RM4000761 | 1044 |
| NT2RM4000965 | F-NT2RM4000965 | 260 | R-NT2RM4000965 | 1045 |
| NT2RM4000997 | F-NT2RM4000997 | 261 | R-NT2RM4000997 | 1046 |
| NT2RM4001321 | F-NT2RM4001321 | 262 | R-NT2RM4001321 | 1047 |
| NT2RM4001325 | F-NT2RM4001325 | 263 | R-NT2RM4001325 | 1048 |
| NT2RM4001377 | F-NT2RM4001377 | 264 | R-NT2RM4001377 | 1049 |
| NT2RM4001735 | F-NT2RM4001735 | 265 | R-NT2RM4001735 | 1050 |
| NT2RM4001768 | F-NT2RM4001768 | 266 | R-NT2RM4001768 | 1051 |
| NT2RM4001843 | F-NT2RM4001843 | 267 | R-NT2RM4001843 | 1052 |
| NT2RM4002352 | F-NT2RM4002352 | 268 | R-NT2RM4002352 | 1053 |
| NT2RP1000002 | F-NT2RP1000002 | 269 | | |
| NT2RP1000050 | F-NT2RP1000050 | 270 | | |
| NT2RP1000181 | F-NT2RP1000181 | 271 | | |
| NT2RP1000239 | F-NT2RP1000239 | 272 | | |
| NT2RP1000261 | F-NT2RP1000261 | 273 | | |
| NT2RP1000271 | F-NT2RP1000271 | 274 | | |
| NT2RP1000300 | F-NT2RP1000300 | 275 | | |
| NT2RP1000325 | F-NT2RP1000325 | 276 | | |
| NT2RP1000448 | F-NT2RP1000448 | 277 | | |
| NT2RP1000465 | F-NT2RP1000465 | 278 | | |
| NT2RP1000468 | F-NT2RP1000468 | 279 | | |
| NT2RP1000551 | F-NT2RP1000551 | 280 | | |
| NT2RP1000579 | F-NT2RP1000579 | 281 | | |
| NT2RP1000613 | F-NT2RP1000613 | 282 | | |
| NT2RP1000679 | F-NT2RP1000679 | 283 | | |
| NT2RP1000740 | F-NT2RP1000740 | 284 | | |
| NT2RP1000903 | F-NT2RP1000903 | 285 | | |
| NT2RP1000981 | F-NT2RP1000981 | 286 | | |
| NT2RP1001004 | F-NT2RP1001004 | 287 | | |
| NT2RP1001020 | F-NT2RP1001020 | 288 | | |
| NT2RP1001031 | F-NT2RP1001031 | 289 | | |
| NT2RP1001563 | F-NT2RP1001563 | 290 | | |
| NT2RP2000092 | F-NT2RP2000092 | 291 | R-NT2RP2000092 | 1054 |
| NT2RP2000178 | F-NT2RP2000178 | 292 | R-NT2RP2000178 | 1055 |
| NT2RP2000240 | F-NT2RP2000240 | 293 | R-NT2RP2000240 | 1056 |
| NT2RP2000394 | F-NT2RP2000394 | 294 | R-NT2RP2000394 | 1057 |
| NT2RP2000447 | F-NT2RP2000447 | 295 | R-NT2RP2000447 | 1058 |
| NT2RP2000479 | F-NT2RP2000479 | 296 | R-NT2RP2000479 | 1059 |
| NT2RP2000514 | F-NT2RP2000514 | 297 | R-NT2RP2000514 | 1060 |
| NT2RP2000533 | F-NT2RP2000533 | 298 | R-NT2RP2000533 | 1061 |
| NT2RP2000610 | F-NT2RP2000610 | 299 | | |
| NT2RP2000616 | F-NT2RP2000616 | 300 | R-NT2RP2000616 | 1062 |
| NT2RP2000649 | F-NT2RP2000649 | 301 | R-NT2RP2000649 | 1063 |
| NT2RP2000663 | F-NT2RP2000663 | 302 | R-NT2RP2000663 | 1064 |
| NT2RP2000694 | F-NT2RP2000694 | 303 | | |
| NT2RP2000712 | F-NT2RP2000712 | 304 | R-NT2RP2000712 | 1065 |
| NT2RP2000739 | F-NT2RP2000739 | 305 | R-NT2RP2000739 | 1066 |
| NT2RP2000818 | F-NT2RP2000818 | 306 | R-NT2RP2000818 | 1067 |
| NT2RP2000903 | F-NT2RP2000903 | 307 | R-NT2RP2000903 | 1068 |
| NT2RP2001200 | F-NT2RP2001200 | 308 | R-NT2RP2001200 | 1069 |
| NT2RP2001223 | F-NT2RP2001223 | 309 | R-NT2RP2001223 | 1070 |
| NT2RP2001276 | F-NT2RP2001276 | 310 | R-NT2RP2001276 | 1071 |
| NT2RP2001388 | F-NT2RP2001388 | 311 | R-NT2RP2001388 | 1072 |
| NT2RP2001469 | F-NT2RP2001469 | 312 | R-NT2RP2001469 | 1073 |
| NT2RP2001480 | F-NT2RP2001480 | 313 | R-NT2RP2001480 | 1074 |
| NT2RP2001495 | F-NT2RP2001495 | 314 | R-NT2RP2001495 | 1075 |
| NT2RP2001514 | F-NT2RP2001514 | 315 | R-NT2RP2001514 | 1076 |
| NT2RP2001529 | F-NT2RP2001529 | 316 | | |
| NT2RP2001538 | F-NT2RP2001538 | 317 | R-NT2RP2001538 | 1077 |
| NT2RP2001562 | F-NT2RP2001562 | 318 | R-NT2RP2001562 | 1078 |
| NT2RP2001662 | F-NT2RP2001662 | 319 | R-NT2RP2001662 | 1079 |
| NT2RP2001755 | F-NT2RP2001755 | 320 | R-NT2RP2001755 | 1080 |
| NT2RP2001769 | F-NT2RP2001769 | 321 | R-NT2RP2001769 | 1081 |
| NT2RP2001817 | F-NT2RP2001817 | 322 | R-NT2RP2001817 | 1082 |
| NT2RP2001878 | F-NT2RP2001878 | 323 | R-NT2RP2001878 | 1083 |
| NT2RP2001903 | F-NT2RP2001903 | 324 | R-NT2RP2001903 | 1084 |
| NT2RP2001915 | F-NT2RP2001915 | 325 | R-NT2RP2001915 | 1085 |
| NT2RP2001921 | F-NT2RP2001921 | 326 | R-NT2RP2001921 | 1086 |
| NT2RP2001948 | F-NT2RP2001948 | 327 | R-NT2RP2001948 | 1087 |
| NT2RP2001956 | F-NT2RP2001956 | 328 | R-NT2RP2001956 | 1088 |
| NT2RP2002015 | F-NT2RP2002015 | 329 | R-NT2RP2002015 | 1089 |
| NT2RP2002063 | F-NT2RP2002063 | 330 | R-NT2RP2002063 | 1090 |
| NT2RP2002188 | F-NT2RP2002188 | 331 | R-NT2RP2002188 | 1091 |
| NT2RP2002232 | F-NT2RP2002232 | 332 | R-NT2RP2002232 | 1092 |
| NT2RP2002304 | F-NT2RP2002304 | 333 | R-nnnnnnnnnnnn | 1093 |
| NT2RP2002409 | F-NT2RP2002409 | 334 | R-NT2RP2002409 | 1094 |
| NT2RP2002510 | F-NT2RP2002510 | 335 | R-NT2RP2002510 | 1095 |
| NT2RP2002527 | F-NT2RP2002527 | 336 | R-NT2RP2002527 | 1096 |
| NT2RP2002533 | F-NT2RP2002533 | 337 | R-NT2RP2002533 | 1097 |
| NT2RP2002564 | F-NT2RP2002564 | 338 | R-NT2RP2002564 | 1098 |
| NT2RP2002674 | F-NT2RP2002674 | 339 | R-NT2RP2002674 | 1099 |
| NT2RP2002721 | F-NT2RP2002721 | 340 | R-NT2RP2002721 | 1100 |
| NT2RP2002824 | F-NT2RP2002824 | 341 | R-NT2RP2002824 | 1101 |
| NT2RP2002942 | F-NT2RP2002942 | 342 | R-NT2RP2002942 | 1102 |
| NT2RP2002974 | F-NT2RP2002974 | 343 | R-NT2RP2002974 | 1103 |
| NT2RP2002976 | F-NT2RP2002976 | 344 | R-NT2RP2002976 | 1104 |
| NT2RP2003042 | F-NT2RP2003042 | 345 | R-NT2RP2003042 | 1105 |
| NT2RP2003138 | F-NT2RP2003138 | 346 | | |
| NT2RP2003179 | F-NT2RP2003179 | 347 | R-NT2RP2003179 | 1106 |
| NT2RP2003210 | F-NT2RP2003210 | 348 | R-NT2RP2003210 | 1107 |
| NT2RP2003302 | F-NT2RP2003302 | 349 | R-NT2RP2003302 | 1108 |
| NT2RP2003369 | F-NT2RP2003369 | 350 | R-NT2RP2003369 | 1109 |
| NT2RP2003383 | F-NT2RP2003383 | 351 | R-NT2RP2003383 | 1110 |
| NT2RP2003390 | F-NT2RP2003390 | 352 | R-NT2RP2003390 | 1111 |
| NT2RP2003469 | F-NT2RP2003469 | 353 | R-NT2RP2003469 | 1112 |
| NT2RP2003545 | F-NT2RP2003545 | 354 | R-NT2RP2003545 | 1113 |
| NT2RP2003593 | F-NT2RP2003593 | 355 | R-NT2RP2003593 | 1114 |
| NT2RP2003599 | F-NT2RP2003599 | 356 | R-NT2RP2003599 | 1115 |
| NT2RP2003655 | F-NT2RP2003655 | 357 | R-NT2RP2003655 | 1116 |
| NT2RP2003664 | F-NT2RP2003664 | 358 | R-NT2RP2003664 | 1117 |
| NT2RP2003931 | F-NT2RP2003931 | 359 | R-NT2RP2003931 | 1118 |
| NT2RP2003940 | F-NT2RP2003940 | 360 | R-NT2RP2003940 | 1119 |
| NT2RP2003950 | F-NT2RP2003950 | 361 | R-NT2RP2003950 | 1120 |
| NT2RP2004069 | F-NT2RP2004069 | 362 | R-NT2RP2004069 | 1121 |
| NT2RP2004108 | F-NT2RP2004108 | 363 | R-NT2RP2004108 | 1122 |
| NT2RP2004141 | F-NT2RP2004141 | 364 | R-NT2RP2004141 | 1123 |
| NT2RP2004179 | F-NT2RP2004179 | 365 | R-NT2RP2004179 | 1124 |
| NT2RP2004205 | F-NT2RP2004205 | 366 | R-NT2RP2004205 | 1125 |
| NT2RP2004447 | F-NT2RP2004447 | 367 | R-NT2RP2004447 | 1126 |
| NT2RP2004495 | F-NT2RP2004495 | 368 | R-NT2RP2004495 | 1127 |
| NT2RP2004524 | F-NT2RP2004524 | 369 | R-NT2RP2004524 | 1128 |
| NT2RP2004556 | F-NT2RP2004556 | 370 | R-NT2RP2004556 | 1129 |
| NT2RP2004606 | F-NT2RP2004606 | 371 | R-NT2RP2004606 | 1130 |
| NT2RP2004648 | F-NT2RP2004648 | 372 | R-NT2RP2004648 | 1131 |
| NT2RP2004670 | F-NT2RP200467() | 373 | R-NT2RP2004670 | 1132 |
| NT2RP2004794 | F-NT2RP2004794 | 374 | R-NT2RP2004794 | 1133 |
| NT2RP2004837 | F-NT2RP2004837 | 375 | R-NT2RP2004837 | 1134 |
| NT2RP2004847 | F-NT2RP2004847 | 376 | R-NT2RP2004847 | 1135 |
| NT2RP2005027 | F-NT2RP2005027 | 377 | R-NT2RP2005027 | 1136 |
| NT2RP2005069 | F-NT2RP2005069 | 378 | R-NT2RP2005069 | 1137 |
| NT2RP2005163 | F-NT2RP2005163 | 379 | R-NT2RP2005163 | 1138 |
| NT2RP2005181 | F-NT2RP2005181 | 380 | R-NT2RP2005181 | 1139 |
| NT2RP2005247 | F-NT2RP2005247 | 381 | R-NT2RP2005247 | 1140 |
| NT2RP2005378 | F-NT2RP2005378 | 382 | R-NT2RP2005378 | 1141 |
| NT2RP2005391 | F-NT2RP2005391 | 383 | R-NT2RP2005391 | 1142 |
| NT2RP2005425 | F-NT2RP2005425 | 384 | R-NT2RP2005425 | 1143 |
| NT2RP2005463 | F-NT2RP2005463 | 385 | R-NT2RP2005463 | 1144 |
| NT2RP2005514 | F-NT2RP2005514 | 386 | R-NT2RP2005514 | 1145 |
| NT2RP2005535 | F-NT2RP2005535 | 387 | R-NT2RP2005535 | 1146 |
| NT2RP2005541 | F-NT2RP2005541 | 388 | R-NT2RP2005541 | 1147 |
| NT2RP2005597 | F-NT2RP2005597 | 389 | R-NT2RP2005597 | 1148 |
| NT2RP2005632 | F-NT2RP2005632 | 390 | R-nnnnnnnnnnnn | 1149 |
| NT2RP2005666 | F-NT2RP2005666 | 391 | R-NT2RP2005666 | 1150 |
| NT2RP2005774 | F-NT2RP2005774 | 392 | R-NT2RP2005774 | 1151 |
| NT2RP2005878 | F-NT2RP2005878 | 393 | R-NT2RP2005878 | 1152 |
| NT2RP2005883 | F-NT2RP2005883 | 394 | R-NT2RP2005883 | 1153 |
| NT2RP2005887 | F-NT2RP2005887 | 395 | R-NT2RP2005887 | 1154 |
| NT2RP2005941 | F-NT2RP2005941 | 396 | R-nnnnnnnnnnnn | 1155 |
| NT2RP2005994 | F-NT2RP2005994 | 397 | R-NT2RP2005994 | 1156 |
| NT2RP2006004 | F-NT2RP2006004 | 398 | R-NT2RP2006004 | 1157 |
| NT2RP2006042 | F-NT2RP2006042 | 399 | R-NT2RP2006042 | 1158 |
| NT2RP2006092 | F-NT2RP2006092 | 400 | R-NT2RP2006092 | 1159 |
| NT2RP2006099 | F-NT2RP2006099 | 401 | R-NT2RP2006099 | 1160 |
| NT2RP2006134 | F-NT2RP2006134 | 402 | R-NT2RP2006134 | 1161 |
| NT2RP2006269 | F-NT2RP2006269 | 403 | R-NT2RP2006269 | 1162 |
| NT2RP2006512 | F-NT2RP2006512 | 404 | R-NT2RP2006512 | 1163 |
| NT2RP3000011 | F-NT2RP3000011 | 405 | R-NT2RP3000011 | 1164 |
| NT2RP3000022 | F-NT2RP3000022 | 406 | R-NT2RP3000022 | 1165 |
| NT2RP3000059 | F-NT2RP3000059 | 407 | R-NT2RP3000059 | 1166 |
| NT2RP3000063 | F-NT2RP3000063 | 408 | R-NT2RP3000063 | 1167 |
| NT2RP3000125 | F-NT2RP3000125 | 409 | R-nnnnnnnnnnnn | 1168 |
| NT2RP3000148 | F-NT2RP3000148 | 410 | R-NT2RP3000148 | 1169 |
| NT2RP3000169 | F-NT2RP3000169 | 411 | R-NT2RP3000169 | 1170 |
| NT2RP3000171 | F-NT2RP3000171 | 412 | R-NT2RP3000171 | 1171 |
| NT2RP3000172 | F-NT2RP3000172 | 413 | R-NT2RP3000172 | 1172 |
| NT2RP3000201 | F-NT2RP3000201 | 414 | R-NT2RP3000201 | 1173 |
| NT2RP3000232 | F-NT2RP3000232 | 415 | R-NT2RP3000232 | 1174 |
| NT2RP3000304 | F-NT2RP3000304 | 416 | R-NT2RP3000304 | 1175 |
| NT2RP3000378 | F-NT2RP3000378 | 417 | R-NT2RP3000378 | 1176 |
| NT2RP3000427 | F-NT2RP3000427 | 418 | | |
| NT2RP3000436 | F-NT2RP3000436 | 419 | R-NT2RP3000436 | 1177 |
| NT2RP3000444 | F-NT2RP3000444 | 420 | R-NT2RP3000444 | 1178 |
| NT2RP3000460 | F-NT2RP3000460 | 421 | R-NT2RP3000460 | 1179 |
| NT2RP3000481 | F-NT2RP3000481 | 422 | R-NT2RP3000481 | 1180 |
| NT2RP3000616 | F-NT2RP3000616 | 423 | R-NT2RP3000616 | 1181 |
| NT2RP3000645 | F-NT2RP3000645 | 424 | R-NT2RP3000645 | 1182 |
| NT2RP3000652 | F-NT2RP3000652 | 425 | R-NT2RP3000652 | 1183 |
| NT2RP3000676 | F-NT2RP3000676 | 426 | R-NT2RP3000676 | 1184 |
| NT2RP3000677 | F-NT2RP3000677 | 427 | R-NT2RP3000677 | 1185 |
| NT2RP3000721 | F-NT2RP3000721 | 428 | R-NT2RP3000721 | 1186 |
| NT2RP3000789 | F-NT2RP3000789 | 429 | R-NT2RP3000789 | 1187 |
| NT2RP3000818 | F-NT2RP3000818 | 430 | R-NT2RP3000818 | 1188 |
| NT2RP3000820 | F-NT2RP3000820 | 431 | R-NT2RP3000820 | 1189 |
| NT2RP3000838 | F-NT2RP3000838 | 432 | R-NT2RP3000838 | 1190 |
| NT2RP3000871 | F-NT2RP3000871 | 433 | R-NT2RP3000871 | 1191 |
| NT2RP3000907 | F-NT2RP3000907 | 434 | R-NT2RP3000907 | 1192 |
| NT2RP3000921 | F-NT2RP3000921 | 435 | R-NT2RP3000921 | 1193 |
| NT2RP3001012 | F-NT2RP3001012 | 436 | R-NT2RP3001012 | 1194 |
| NT2RP3001044 | F-NT2RP3001044 | 437 | R-NT2RP3001044 | 1195 |
| NT2RP3001061 | F-NT2RP3001061 | 438 | R-NT2RP3001061 | 1196 |
| NT2RP3001159 | F-NT2RP3001159 | 439 | R-NT2RP3001159 | 1197 |
| NT2RP3001170 | F-NT2RP3001170 | 440 | R-NT2RP3001170 | 1198 |
| NT2RP3001195 | F-NT2RP3001195 | 441 | R-NT2RP3001195 | 1199 |
| NT2RP3001240 | F-NT2RP3001240 | 442 | R-NT2RP3001240 | 1200 |
| NT2RP3001271 | F-NT2RP3001271 | 443 | R-NT2RP3001271 | 1201 |
| NT2RP3001322 | F-NT2RP3001322 | 444 | R-NT2RP3001322 | 1202 |
| NT2RP3001388 | F-NT2RP3001388 | 445 | | |
| NT2RP3001542 | F-NT2RP3001542 | 446 | R-NT2RP3001542 | 1203 |
| NT2RP3001560 | F-NT2RP3001560 | 447 | R-NT2RP3001560 | 1204 |
| NT2RP3001592 | F-NT2RP3001592 | 448 | R-NT2RP3001592 | 1205 |
| NT2RP3001650 | F-NT2RP3001650 | 449 | | |
| NT2RP3001685 | F-NT2RP3001685 | 450 | R-NT2RP3001685 | 1206 |
| NT2RP3001738 | F-NT2RP3001738 | 451 | R-NT2RP3001738 | 1207 |
| NT2RP3001754 | F-NT2RP3001754 | 452 | R-NT2RP3001754 | 1208 |
| NT2RP3001858 | F-NT2RP3001858 | 453 | R-NT2RP3001858 | 1209 |
| NT2RP3001976 | F-NT2RP3001976 | 454 | R-NT2RP3001976 | 1210 |
| NT2RP3002015 | F-NT2RP3002015 | 455 | R-NT2RP3002015 | 1211 |
| NT2RP3002160 | F-NT2RP3002160 | 456 | R-NT2RP3002160 | 1212 |
| NT2RP3002281 | F-NT2RP3002281 | 457 | R-NT2RP3002281 | 1213 |
| NT2RP3002286 | F-NT2RP3002286 | 458 | R-NT2RP3002286 | 1214 |
| NT2RP3002311 | F-NT2RP3002311 | 459 | R-NT2RP3002311 | 1215 |
| NT2RP3002324 | F-NT2RP3002324 | 460 | R-NT2RP3002324 | 1216 |
| NT2RP3002342 | F-NT2RP3002342 | 461 | R-NT2RP3002342 | 1217 |
| NT2RP3002353 | F-NT2RP3002353 | 462 | R-NT2RP3002353 | 1218 |
| NT2RP3002409 | F-NT2RP3002409 | 463 | R-NT2RP3002409 | 1219 |
| NT2RP3002411 | F-NT2RP3002411 | 464 | R-NT2RP3002411 | 1220 |
| NT2RP3002448 | F-NT2RP3002448 | 465 | R-NT2RP3002448 | 1221 |
| NT2RP3002571 | F-NT2RP3002571 | 466 | R-NT2RP3002571 | 1222 |
| NT2RP3002664 | F-NT2RP3002664 | 467 | R-NT2RP3002664 | 1223 |
| NT2RP3002721 | F-NT2RP3002721 | 468 | R-NT2RP3002721 | 1224 |
| NT2RP3002737 | F-NT2RP3002737 | 469 | R-NT2RP3002737 | 1225 |
| NT2RP3002738 | F-NT2RP3002738 | 470 | R-NT2RP3002738 | 1226 |
| NT2RP3002790 | F-NT2RP3002790 | 471 | R-NT2RP3002790 | 1227 |
| NT2RP3002836 | F-NT2RP3002836 | 472 | R-NT2RP3002836 | 1228 |
| NT2RP3002887 | F-NT2RP3002887 | 473 | R-NT2RP3002887 | 1229 |
| NT2RP3002900 | F-NT2RP3002900 | 474 | R-NT2RP3002900 | 1230 |
| NT2RP3002958 | F-NT2RP3002958 | 475 | R-NT2RP3002958 | 1231 |
| NT2RP3002983 | F-NT2RP3002983 | 476 | R-NT2RP3002983 | 1232 |
| NT2RP3003000 | F-NT2RP3003000 | 477 | R-NT2RP3003000 | 1233 |
| NT2RP3003076 | F-NT2RP3003076 | 478 | R-NT2RP3003076 | 1234 |
| NT2RP3003354 | F-NT2RP3003354 | 479 | R-NT2RP3003354 | 1235 |
| NT2RP3003448 | F-NT2RP3003448 | 480 | R-NT2RP3003448 | 1236 |
| NT2RP3003469 | F-NT2RP3003469 | 481 | R-NT2RP3003469 | 1237 |
| NT2RP3003473 | F-NT2RP3003473 | 482 | R-NT2RP3003473 | 1238 |
| NT2RP3003527 | F-NT2RP3003527 | 483 | R-NT2RP3003527 | 1239 |
| NT2RP3003532 | F-NT2RP3003532 | 484 | R-NT2RP3003532 | 1240 |
| NT2RP3003535 | F-NT2RP3003535 | 485 | R-nnnnnnnnnnnn | 1241 |
| NT2RP3003559 | F-NT2RP3003559 | 486 | R-NT2RP3003559 | 1242 |
| NT2RP3003614 | F-NT2RP3003614 | 487 | R-NT2RP3003614 | 1243 |
| NT2RP3003729 | F-NT2RP3003729 | 488 | R-NT2RP3003729 | 1244 |
| NT2RP3003849 | F-NT2RP3003849 | 489 | R-NT2RP3003849 | 1245 |
| NT2RP3003874 | F-NT2RP3003874 | 490 | R-NT2RP3003874 | 1246 |
| NT2RP3003939 | F-NT2RP3003939 | 491 | | |
| NT2RP3003963 | F-NT2RP3003963 | 492 | R-NT2RP3003963 | 1247 |
| NT2RP3004000 | F-NT2RP3004000 | 493 | R-NT2RP3004000 | 1248 |
| NT2RP3004025 | F-NT2RP3004025 | 494 | R-NT2RP3004025 | 1249 |
| NT2RP3004067 | F-NT2RP3004067 | 495 | | |
| NT2RP3004075 | F-NT2RP3004075 | 496 | R-NT2RP3004075 | 1250 |
| NT2RP3004083 | F-NT2RP3004083 | 497 | R-NT2RP3004083 | 1251 |
| NT2RP3004090 | F-NT2RP3004090 | 498 | R-NT2RP3004090 | 1252 |
| NT2RP3004119 | F-NT2RP3004119 | 499 | R-NT2RP3004119 | 1253 |
| NT2RP3004130 | F-NT2RP3004130 | 500 | R-NT2RP3004130 | 1254 |
| NT2RP3004133 | F-NT2RP3004133 | 501 | R-NT2RP3004133 | 1255 |
| NT2RP3004202 | F-NT2RP3004202 | 502 | R-NT2RP3004202 | 1256 |
| NT2RP3004294 | F-NT2RP3004294 | 503 | R-NT2RP3004294 | 1257 |
| NT2RP3004309 | F-NT2RP3004309 | 504 | R-NT2RP3004309 | 1258 |
| NT2RP3004321 | F-NT2RP3004321 | 505 | R-NT2RP3004321 | 1259 |
| NT2RP3004345 | F-NT2RP3004345 | 506 | R-NT2RP3004345 | 1260 |
| NT2RP3004355 | F-NT2RP3004355 | 507 | R-NT2RP3004355 | 1261 |
| NT2RP3004374 | F-NT2RP3004374 | 508 | R-NT2RP3004374 | 1262 |
| NT2RP3004406 | F-NT2RP3004406 | 509 | R-NT2RP3004406 | 1263 |
| NT2RP3004481 | F-NT2RP3004481 | 510 | R-NT2RP3004481 | 1264 |
| NT2RP3004552 | F-NT2RP3004552 | 511 | R-NT2RP3004552 | 1265 |
| NT2RP3004557 | F-NT2RP3004557 | 512 | | |
| NT2RP3004625 | F-NT2RP3004625 | 513 | R-NT2RP3004625 | 1266 |
| NT2RP3004640 | F-NT2RP3004640 | 514 | R-NT2RP3004640 | 1267 |
| NT2RP3004647 | F-NT2RP3004647 | 515 | R-NT2RP3004647 | 1268 |
| NT2RP4000108 | F-NT2RP4000108 | 516 | R-NT2RP4000108 | 1269 |
| NT2RP4000634 | F-NT2RP4000634 | 517 | R-NT2RP4000634 | 1270 |
| NT2RP4000962 | F-NT2RP4000962 | 518 | R-NT2RP4000962 | 1271 |
| NT2RP4001001 | F-NT2RP4001001 | 519 | R-NT2RP4001001 | 1272 |
| NT2RP4001009 | F-NT2RP4001009 | 520 | R-NT2RP4001009 | 1273 |
| NT2RP4001467 | F-NT2RP4001467 | 521 | R-NT2RP4001467 | 1274 |
| NT2RP4001877 | F-NT2RP4001877 | 522 | R-NT2RP4001877 | 1275 |
| NT2RP4001879 | F-NT2RP4001879 | 523 | R-NT2RP4001879 | 1276 |
| NT2RP4002187 | F-NT2RP4002187 | 524 | R-NT2RP4002187 | 1277 |
| NT2RP4002451 | F-NT2RP4002451 | 525 | R-NT2RP4002451 | 1278 |
| NT2RP4002715 | F-NT2RP4002715 | 526 | R-NT2RP4002715 | 1279 |
| NT2RP4002750 | F-NT2RP4002750 | 527 | R-NT2RP4002750 | 1280 |
| OVARC1000003 | F-OVARC1000003 | 528 | R-OVARC1000003 | 1281 |
| OVARC1000090 | F-OVARC1000090 | 529 | R-OVARC1000090 | 1282 |
| OVARC1000105 | F-OVARC1000105 | 530 | R-OVARC1000105 | 1283 |
| OVARC1000137 | F-OVARC1000137 | 531 | R-OVARC1000137 | 1284 |
| OVARC1000208 | F-OVARC1000208 | 532 | R-OVARC1000208 | 1285 |
| OVARC1000255 | F-OVARC1000255 | 533 | R-OVARC1000255 | 1286 |
| OVARC1000275 | F-OVARC1000275 | 534 | R-OVARC1000275 | 1287 |
| OVARC1000298 | F-OVARC1000298 | 535 | R-OVARC1000298 | 1288 |
| OVARC1000307 | F-OVARC1000307 | 536 | R-OVARC1000307 | 1289 |
| OVARC1000313 | F-OVARC1000313 | 537 | R-OVARC1000313 | 1290 |
| OVARC1000331 | F-OVARC1000331 | 538 | R-OVARC1000331 | 1291 |
| OVARC1000410 | F-OVARC1000410 | 539 | R-OVARC1000410 | 1292 |
| OVARC1000439 | F-OVARC1000439 | 540 | R-OVARC1000439 | 1293 |
| OVARC1000467 | F-OVARC1000467 | 541 | R-OVARC1000467 | 1294 |
| OVARC1000529 | F-OVARC1000529 | 542 | R-OVARC1000529 | 1295 |
| OVARC1000553 | F-OVARC1000553 | 543 | R-OVARC1000553 | 1296 |
| OVARC1000775 | F-OVARC1000775 | 544 | R-OVARC1000775 | 1297 |
| OVARC1000811 | F-OVARC1000811 | 545 | R-OVARC1000811 | 1298 |
| OVARC1000853 | F-OVARC1000853 | 546 | R-OVARC1000853 | 1299 |
| OVARC1000873 | F-OVARC1000873 | 547 | R-OVARC1000873 | 1300 |
| OVARC1000916 | F-OVARC1000916 | 548 | R-OVARC1000916 | 1301 |
| OVARC1000956 | F-OVARC1000956 | 549 | R-OVARC1000956 | 1302 |
| OVARC1000995 | F-OVARC1000995 | 550 | R-OVARC1000995 | 1303 |
| OVARC1001030 | F-OVARC1001030 | 551 | R-OVARC1001030 | 1304 |
| OVARC1001049 | F-OVARC1001049 | 552 | R-OVARC1001049 | 1305 |
| OVARC1001086 | F-OVARC1001086 | 553 | R-OVARC1001086 | 1306 |
| OVARC1001132 | F-OVARC1001132 | 554 | R-OVARC1001132 | 1307 |
| OVARC1001163 | F-OVARC1001163 | 555 | R-OVARC1001163 | 1308 |
| OVARC1001222 | F-OVARC1001222 | 556 | R-OVARC1001222 | 1309 |
| OVARC1001260 | F-OVARC1001260 | 557 | R-OVARC1001260 | 1310 |
| OVARC1001336 | F-OVARC1001336 | 558 | R-OVARC1001336 | 1311 |
| OVARC1001338 | F-OVARC1001338 | 559 | R-OVARC1001338 | 1312 |
| OVARC1001569 | F-OVARC1001569 | 560 | R-OVARC1001569 | 1313 |
| OVARC1001570 | F-OVARC1001570 | 561 | R-OVARC1001570 | 1314 |
| OVARC1001596 | F-OVARC1001596 | 562 | R-OVARC1001596 | 1315 |
| OVARC1001607 | F-OVARC1001607 | 563 | R-OVARC1001607 | 1316 |
| OVARC1001725 | F-OVARC1001725 | 564 | R-OVARC1001725 | 1317 |
| OVARC1001727 | F-OVARC1001727 | 565 | R-OVARC1001727 | 1318 |
| OVARC1001807 | F-OVARC1001807 | 566 | R-OVARC1001807 | 1319 |
| OVARC1001833 | F-OVARC1001833 | 567 | R-OVARC1001833 | 1320 |
| OVARC1001952 | F-OVARC1001952 | 568 | | |
| OVARC1001991 | F-OVARC1001991 | 569 | R-OVARC1001991 | 1321 |
| OVARC1002058 | F-OVARC1002058 | 570 | R-OVARC1002058 | 1322 |
| OVARC1002178 | F-OVARC1002178 | 571 | R-OVARC1002178 | 1323 |
| PLACE 1000033 | F-PLACE1000033 | 572 | R-PLACE1000033 | 1324 |
| PLACE 1000231 | F-PLACE1000231 | 573 | R-PLACE1000231 | 1325 |
| PLACE 1000258 | F-PLACE1000258 | 574 | R-PLACE1000258 | 1326 |
| PLACE 1000442 | F-PLACE1000442 | 575 | R-PLACE1000442 | 1327 |
| PLACE 1000560 | F-PLACE1000560 | 576 | R-PLACE1000560 | 1328 |
| PLACE 1000740 | F-PLACE1000740 | 577 | R-PLACE1000740 | 1329 |
| PLACE 1000907 | F-PLACE1000907 | 578 | | |
| PLACE 1000912 | F-PLACE1000912 | 579 | R-PLACE1000912 | 1330 |
| PLACE 1000914 | F-PLACE1000914 | 580 | R-PLACE1000914 | 1331 |
| PLACE 1000927 | F-PLACE1000927 | 581 | R-PLACE1000927 | 1332 |
| PLACE 1000986 | F-PLACE1000986 | 582 | R-PLACE1000986 | 1333 |
| PLACE1001016 | F-PLACE1001016 | 583 | R-PLACE1001016 | 1334 |
| PLACE 1001100 | F-PLACE1001100 | 584 | R-PLACE1001100 | 1335 |
| PLACE 1001114 | F-PLACE1001114 | 585 | R-PLACE1001114 | 1336 |
| PLACE 1001123 | F-PLACE1001123 | 586 | R-PLACE1001123 | 1337 |
| PLACE 1001183 | F-PLACE1001183 | 587 | R-PLACE1001183 | 1338 |
| PLACE 1001229 | F-PLACE1001229 | 588 | R-PLACE1001229 | 1339 |
| PLACE 1001231 | F-PLACE1001231 | 589 | R-PLACE1001231 | 1340 |
| PLACE 1001340 | F-PLACE1001340 | 590 | R-PLACE1001340 | 1341 |
| PLACE1001401 | F-PLACE1001401 | 591 | R-PLACE1001401 | 1342 |
| PLACE 1001407 | F-PLACE1001407 | 592 | R-PLACE1001407 | 1343 |
| PLACE 1001464 | F-PLACE1001464 | 593 | R-PLACE1001464 | 1344 |
| PLACE1001500 | F-PLACE1001500 | 594 | R-PLACE1001500 | 1345 |
| PLACE1001516 | F-PLACE1001516 | 595 | R-PLACE1001516 | 1346 |
| PLACE1001536 | F-PLACE1001536 | 596 | R-PLACE1001536 | 1347 |
| PLACE 1001564 | F-PLACE1001564 | 597 | R-PLACE1001564 | 1348 |
| PLACE 1001655 | F-PLACE1001655 | 598 | R-PLACE1001655 | 1349 |
| PLACE 1001788 | F-PLACE1001788 | 599 | R-PLACE1001788 | 1350 |
| PLACE1001795 | F-PLACE1001795 | 600 | R-PLACE1001795 | 1351 |
| PLACE1001836 | F-PLACE1001836 | 601 | R-PLACE1001836 | 1352 |
| PLACE 1001918 | F-PLACE1001918 | 602 | R-PLACE1001918 | 1353 |
| PLACE 1001949 | F-PLACE1001949 | 603 | R-PLACE1001949 | 1354 |
| PLACE 1002080 | F-PLACE1002080 | 604 | R-PLACE1002080 | 1355 |
| PLACE 1002095 | F-PLACE1002095 | 605 | R-PLACE1002095 | 1356 |
| PLACE 1002153 | F-PLACE1002153 | 606 | R-PLACE1002153 | 1357 |
| PLACE 1002329 | F-PLACE1002329 | 607 | R-PLACE1002329 | 1358 |
| PLACE 1002355 | F-PLACE1002355 | 608 | R-PLACE1002355 | 1359 |
| PLACE1002374 | F-PLACE1002374 | 609 | R-PLACE1002374 | 1360 |
| PLACE 1002518 | F-PLACE1002518 | 610 | R-PLACE1002518 | 1361 |
| PLACE 1002547 | F-PLACE1002547 | 611 | R-PLACE1002547 | 1362 |
| PLACE 1002726 | F-PLACE1002726 | 612 | R-PLACE1002726 | 1363 |
| PLACE 1002905 | F-PLACE1002905 | 613 | R-PLACE1002905 | 1364 |
| PLACE 1002911 | F-PLACE1002911 | 614 | R-PLACE1002911 | 1365 |
| PLACE 1002967 | F-PLACE1002967 | 615 | R-PLACE1002967 | 1366 |
| PLACE 1003135 | F-PLACE1003135 | 616 | R-PLACE1003135 | 1367 |
| PLACE1003163 | F-PLACE1003163 | 617 | R-PLACE1003163 | 1368 |
| PLACE 1003407 | F-PLACE1003407 | 618 | R-PLACE1003407 | 1369 |
| PLACE 1003428 | F-PLACE1003428 | 619 | R-PLACE1003428 | 1370 |
| PLACE1003438 | F-PLACE1003438 | 620 | R-PLACE1003438 | 1371 |
| PLACE 1003460 | F-PLACE1003460 | 621 | R-PLACE1003460 | 1372 |
| PLACE 1003529 | F-PLACE1003529 | 622 | R-nnnnnnnnnnnn | 1373 |
| PLACE 1003573 | F-PLACE1003573 | 623 | R-PLACE1003573 | 1374 |
| PLACE 1003598 | F-PLACE1003598 | 624 | R-PLACE1003598 | 1375 |
| PLACE1003644 | F-PLACE1003644 | 625 | R-PLACE1003644 | 1376 |
| PLACE1003737 | F-PLACE1003737 | 626 | R-PLACE1003737 | 1377 |
| PLACE 1003772 | F-PLACE1003772 | 627 | R-PLACE1003772 | 1378 |
| PLACE 1003839 | F-PLACE1003839 | 628 | R-PLACE1003839 | 1379 |
| PLACE 1003845 | F-PLACE1003845 | 629 | R-PLACE1003845 | 1380 |
| PLACE 1003 852 | F-PLACE1003852 | 630 | R-PLACE1003852 | 1381 |
| PLACE 1004028 | F-PLACE1004028 | 631 | R-PLACE1004028 | 1382 |
| PLACE 1004078 | F-PLACE1004078 | 632 | R-PLACE1004078 | 1383 |
| PLACE1004166 | F-PLACE1004166 | 633 | R-PLACE1004166 | 1384 |
| PLACE 1004168 | F-PLACE1004168 | 634 | R-nnnnnnnnnnnn | 1385 |
| PLACE1004199 | F-PLACE1004199 | 635 | R-PLACE1004199 | 1386 |
| PLACE 1004279 | F-PLACE1004279 | 636 | R-PLACE1004279 | 1387 |
| PLACE 1004282 | F-PLACE1004282 | 637 | R-PLACE1004282 | 1388 |
| PLACE 1004305 | F-PLACE1004305 | 638 | R-PLACE1004305 | 1389 |
| PLACE 1004441 | F-PLACE1004441 | 639 | R-PLACE1004441 | 1390 |
| PLACE 1004450 | F-PLACE1004450 | 640 | R-PLACE1004450 | 1391 |
| PLACE 1004482 | F-PLACE1004482 | 641 | R-PLACE1004482 | 1392 |
| PLACE 1004492 | F-PLACE1004492 | 642 | R-PLACE1004492 | 1393 |
| PLACE 1004519 | F-PLACE1004519 | 643 | R-PLACE1004519 | 1394 |
| PLACE 1004520 | F-PLACE1004520 | 644 | R-PLACE1004520 | 1395 |
| PLACE 1004630 | F-PLACE1004630 | 645 | R-PLACE1004630 | 1396 |
| PLACE 1004637 | F-PLACE1004637 | 646 | R-PLACE1004637 | 1397 |
| PLACE 1004648 | F-PLACE1004648 | 647 | R-PLACE1004648 | 1398 |
| PLACE 1004816 | F-PLACE1004816 | 648 | R-PLACE1004816 | 1399 |
| PLACE 1004887 | F-PLACE1004887 | 649 | R-PLACE1004887 | 1400 |
| PLACE 1005003 | F-PLACE1005003 | 650 | R-PLACE1005003 | 1401 |
| PLACE 1005005 | F-PLACE1005005 | 651 | R-PLACE1005005 | 1402 |
| PLACE 1005031 | F-PLACE1005031 | 652 | R-PLACE1005031 | 1403 |
| PLACE 1005239 | F-PLACE1005239 | 653 | R-PLACE1005239 | 1404 |
| PLACE 1005250 | F-PLACE1005250 | 654 | R-PLACE1005250 | 1405 |
| PLACE 1005383 | F-PLACE1005383 | 655 | R-PLACE1005383 | 1406 |
| PLACE 1005410 | F-PLACE1005410 | 656 | R-PLACE1005410 | 1407 |
| PLACE 1005426 | F-PLACE1005426 | 657 | R-PLACE1005426 | 1408 |
| PLACE 1005519 | F-PLACE1005519 | 658 | R-PLACE1005519 | 1409 |
| PLACE 1005539 | F-PLACE1005539 | 659 | R-PLACE1005539 | 1410 |
| PLACE 1005544 | F-PLACE1005544 | 660 | R-PLACE1005544 | 1411 |
| PLACE 1005569 | F-PLACE1005569 | 661 | R-PLACE1005569 | 1412 |
| PLACE 1005601 | F-PLACE1005601 | 662 | R-PLACE1005601 | 1413 |
| PLACE 1005660 | F-PLACE1005660 | 663 | R-PLACE1005660 | 1414 |
| PLACE 1005669 | F-PLACE1005669 | 664 | R-PLACE1005669 | 1415 |
| PLACE 1005682 | F-PLACE1005682 | 665 | R-PLACE1005682 | 1416 |
| PLACE 1005725 | F-PLACE1005725 | 666 | R-PLACE1005725 | 1417 |
| PLACE 1005736 | F-PLACE1005736 | 667 | R-PLACE1005736 | 1418 |
| PLACE 1005745 | F-PLACE1005745 | 668 | R-PLACE1005745 | 1419 |
| PLACE 1005768 | F-PLACE1005768 | 669 | R-PLACE1005768 | 1420 |
| PLACE1005815 | F-PLACE1005815 | 670 | R-PLACE1005815 | 1421 |
| PLACE 1005878 | F-PLACE1005878 | 671 | R-PLACE1005878 | 1422 |
| PLACE 1005927 | F-PLACE1005927 | 672 | R-PLACE1005927 | 1423 |
| PLACE 1006071 | F-PLACE1006071 | 673 | R-PLACE1006071 | 1424 |
| PLACE 1006073 | F-PLACE1006073 | 674 | R-PLACE1006073 | 1425 |
| PLACE 1006079 | F-PLACE1006079 | 675 | R-PLACE1006079 | 1426 |
| PLACE 1006093 | F-PLACE1006093 | 676 | R-PLACE1006093 | 1427 |
| PLACE 1006208 | F-PLACE1006208 | 677 | R-nnnnnnnnnnnn | 1428 |
| PLACE 1006219 | F-PLACE1006219 | 678 | R-PLACE1006219 | 1429 |
| PLACE 1006277 | F-PLACE1006277 | 679 | R-PLACE1006277 | 1430 |
| PLACE 1006290 | F-PLACE1006290 | 680 | R-PLACE1006290 | 1431 |
| PLACE 1006443 | F-PLACE1006443 | 681 | R-PLACE1006443 | 1432 |
| PLACE 1006515 | F-PLACE1006515 | 682 | R-PLACE1006515 | 1433 |
| PLACE 1006716 | F-PLACE1006716 | 683 | R-PLACE1006716 | 1434 |
| PLACE 1006786 | F-PLACE1006786 | 684 | R-PLACE1006786 | 1435 |
| PLACE 1006809 | F-PLACE1006809 | 685 | R-PLACE1006809 | 1436 |
| PLACE 1006959 | F-PLACE1006959 | 686 | R-PLACE1006959 | 1437 |
| PLACE 1007028 | F-PLACE1007028 | 687 | R-PLACE1007028 | 1438 |
| PLACE 1007040 | F-PLACE1007040 | 688 | R-PLACE1007040 | 1439 |
| PLACE 1007077 | F-PLACE1007077 | 689 | R-PLACE1007077 | 1440 |
| PLACE 1007081 | F-PLACE1007081 | 690 | R-PLACE1007081 | 1441 |
| PLACE 1007096 | F-PLACE1007096 | 691 | R-PLACE1007096 | 1442 |
| PLACE 1007296 | F-PLACE1007296 | 692 | R-PLACE1007296 | 1443 |
| PLACE 1007591 | F-PLACE1007591 | 693 | R-PLACE1007591 | 1444 |
| PLACE1007626 | F-PLACE1007626 | 694 | R-PLACE1007626 | 1445 |
| PLACE 1007702 | F-PLACE1007702 | 695 | R-PLACE1007702 | 1446 |
| PLACE1007845 | F-PLACE1007845 | 696 | R-PLACE1007845 | 1447 |
| PLACE1007881 | F-PLACE1007881 | 697 | R-PLACE1007881 | 1448 |
| PLACE 1007971 | F-PLACE1007971 | 698 | R-PLACE1007971 | 1449 |
| PLACE 1008282 | F-PLACE1008282 | 699 | R-PLACE1008282 | 1450 |
| PLACE 1008297 | F-PLACE1008297 | 700 | R-PLACE1008297 | 1451 |
| PLACE 1008359 | F-PLACE1008359 | 701 | R-PLACE1008359 | 1452 |
| PLACE 1008469 | F-PLACE1008469 | 702 | R-PLACE1008469 | 1453 |
| PLACE 1008549 | F-PLACE1008549 | 703 | R-PLACE1008549 | 1454 |
| PLACE 1008657 | F-PLACE1008657 | 704 | R-PLACE1008657 | 1455 |
| PLACE 1008716 | F-PLACE1008716 | 705 | R-PLACE1008716 | 1456 |
| PLACE 1008744 | F-PLACE1008744 | 706 | R-PLACE1008744 | 1457 |
| PLACE 1008984 | F-PLACE1008984 | 707 | R-PLACE1008984 | 1458 |
| PLACE 1008985 | F-PLACE1008985 | 708 | R-PLACE1008985 | 1459 |
| PLACE 1009067 | F-PLACE1009067 | 709 | R-PLACE1009067 | 1460 |
| PLACE 1009196 | F-PLACE1009196 | 710 | R-PLACE1009196 | 1461 |
| PLACE 1009279 | F-PLACE1009279 | 711 | R-PLACE1009279 | 1462 |
| PLACE 1009527 | F-PLACE1009527 | 712 | R-PLACE1009527 | 1463 |
| PLACE 1009546 | F-PLACE1009546 | 713 | R-PLACE1009546 | 1464 |
| PLACE 1009600 | F-PLACE1009600 | 714 | R-PLACE1009600 | 1465 |
| PLACE 1009735 | F-PLACE1009735 | 715 | R-PLACE1009735 | 1466 |
| PLACE 1009982 | F-PLACE1009982 | 716 | R-nnnnnnnnnnnn | 1467 |
| PLACE1010011 | F-PLACE1010011 | 717 | R-PLACE1010011 | 1468 |
| PLACE1010078 | F-PLACE1010078 | 718 | R-PLACE1010078 | 1469 |
| PLACE1010081 | F-PLACE1010081 | 719 | R-PLACE1010081 | 1470 |
| PLACE 1010251 | F-PLACE1010251 | 720 | R-PLACE1010251 | 1471 |
| PLACE1010445 | F-PLACE1010445 | 721 | R-PLACE1010445 | 1472 |
| PLACE 1010713 | F-PLACE1010713 | 722 | R-PLACE1010713 | 1473 |
| PLACE 1010784 | F-PLACE1010784 | 723 | R-PLACE1010784 | 1474 |
| PLACE 1010827 | F-PLACE1010827 | 724 | R-PLACE1010827 | 1475 |
| PLACE 1010968 | F-PLACE1010968 | 725 | R-PLACE1010968 | 1476 |
| PLACE 1011045 | F-PLACE1011045 | 726 | R-PLACE1011045 | 1477 |
| PLACE1011116 | F-PLACE1011116 | 727 | R-PLACE1011116 | 1478 |
| PLACE1011181 | F-PLACE1011181 | 728 | | |
| PLACE1011236 | F-PLACE1011236 | 729 | R-PLACE1011236 | 1479 |
| PLACE 1011364 | F-PLACE1011364 | 730 | R-PLACE1011364 | 1480 |
| PLACE 1 011407 | F-PLACE1011407 | 731 | R-PLACE1011407 | 1481 |
| PLACE1011516 | F-PLACE1011516 | 732 | R-PLACE1011516 | 1482 |
| PLACE 1011708 | F-PLACE1011708 | 733 | R-PLACE1011708 | 1483 |
| PLACE 1011824 | F-PLACE1011824 | 734 | R-PLACE1011824 | 1484 |
| PLACE 1011978 | F-PLACE1011978 | 735 | R-PLACE1011978 | 1485 |
| PLACE2000118 | F-PLACE2000118 | 736 | R-PLACE2000118 | 1486 |
| PLACE2000219 | F-PLACE2000219 | 737 | R-PLACE2000219 | 1487 |
| PLACE3000181 | F-PLACE3000181 | 738 | R-PLACE3000181 | 1488 |
| PLACE3000213 | F-PLACE3000213 | 739 | R-PLACE3000213 | 1489 |
| PLACE4000354 | F-PLACE4000354 | 740 | R-PLACE4000354 | 1490 |
| PLACE4000455 | F-PLACE4000455 | 741 | R-PLACE4000455 | 1491 |
| SKNMC1000004 | F-SKNMC1000004 | 742 | | |
| SKNMC1000014 | F-SKNMC1000014 | 743 | | |
| SKNMC1000082 | F-SKNMC1000082 | 744 | | |
| THYRO1000036 | F-THYRO1000036 | 745 | R-THYRO1000036 | 1492 |
| THYRO1000061 | F-THYRO1000061 | 746 | R-THYRO1000061 | 1493 |
| THYRO1000099 | F-THYRO1000099 | 747 | R-THYRO1000099 | 1494 |
| THYRO1000196 | F-THYRO1000196 | 748 | R-THYRO1000196 | 1495 |
| THYRO1000400 | F-THYRO1000400 | 749 | R-THYRO1000400 | 1496 |
| THYRO1000580 | F-THYRO1000580 | 750 | R-THYRO1000580 | 1497 |
| THYRO1000584 | F-THYRO1000584 | 751 | R-THYRO1000584 | 1498 |
| THYRO1000678 | F-THYRO1000678 | 752 | R-THYRO1000678 | 1499 |
| THYRO1000776 | F-THYRO1000776 | 753 | R-THYRO1000776 | 1500 |
| THYRO1000795 | F-THYRO1000795 | 754 | R-THYRO1000795 | 1501 |
| THYRO1000846 | F-THYRO1000846 | 755 | R-THYRO1000846 | 1502 |
| THYRO1000866 | F-THYRO1000866 | 756 | R-THYRO1000866 | 1503 |
| THYRO1000956 | F-THYRO1000956 | 757 | R-THYRO1000956 | 1504 |
| THYRO1000964 | F-THYRO1000964 | 758 | R-THYRO1000964 | 1505 |
| THYRO1000999 | F-THYRO1000999 | 759 | R-THYRO1000999 | 1506 |
| THYRO1001063 | F-THYRO1001063 | 760 | R-THYRO1001063 | 1507 |
| THYRO1001071 | F-THYRO1001071 | 761 | R-THYRO1001071 | 1508 |
| THYR01001102 | F-THYRO1001102 | 762 | R-THYRO1001102 | 1509 |
| THYRO1001113 | F-THYRO1001113 | 763 | R-THYRO1001113 | 1510 |
| THYRO1001128 | F-THYRO1001128 | 764 | R-THYRO1001128 | 1511 |
| THYRO1001205 | F-THYRO1001205 | 765 | R-THYRO1001205 | 1512 |
| THYRO1001237 | F-THYRO1001237 | 766 | R-THYRO1001237 | 1513 |
| THYRO1001242 | F-THYRO1001242 | 767 | R-THYRO1001242 | 1514 |
| THYRO1001266 | F-THYRO1001266 | 768 | R-THYRO1001266 | 1515 |
| THYRO1001327 | F-THYRO1001327 | 769 | R-THYRO1001327 | 1516 |
| THYRO1001456 | F-THYRO1001456 | 770 | R-THYRO1001456 | 1517 |
| THYRO1001457 | F-THYRO1001457 | 771 | R-THYRO1001457 | 1518 |
| THYRO1001471 | F-THYRO1001471 | 772 | R-THYRO1001471 | 1519 |
| THYRO1001478 | F-THYRO1001478 | 773 | R-THYRO1001478 | 1520 |
| THYRO1001495 | F-THYRO1001495 | 774 | R-THYRO1001495 | 1521 |
| THYRO1001523 | F-THYRO1001523 | 775 | R-THYRO1001523 | 1522 |
| THYRO1001529 | F-THYRO1001529 | 776 | R-THYRO1001529 | 1523 |
| THYRO1001593 | F-THYRO1001593 | 777 | R-THYRO1001593 | 1524 |
| THYRO1001608 | F-THYRO1001608 | 778 | R-THYRO1001608 | 1525 |
| THYRO1001641 | F-THYRO1001641 | 779 | R-THYRO1001641 | 1526 |
| THYRO1001700 | F-THYRO1001700 | 780 | R-THYRO1001700 | 1527 |
| THYRO1001702 | F-THYRO1001702 | 781 | R-THYRO1001702 | 1528 |
| THYRO1001725 | F-THYRO1001725 | 782 | R-THYRO1001725 | 1529 |
| THYRO1001770 | F-THYRO1001770 | 783 | R-THYRO1001770 | 1530 |
| THYRO1001803 | F-THYRO1001803 | 784 | R-THYRO1001803 | 1531 |
| Y79AA1000030 | F-Y79AA1000030 | 785 | R-Y79AA1000030 | 1532 |
| Y79AA1000127 | F-Y79AA1000127 | 786 | R-Y79AA1000127 | 1533 |
| Y79AA1000207 | F-Y79AA1000207 | 787 | R-Y79AA1000207 | 1534 |
| Y79AA1000226 | F-Y79AA1000226 | 788 | R-Y79AA1000226 | 1535 |
| Y79AA1000270 | F-Y79AA1000270 | 789 | R-Y79AA1000270 | 1536 |
| Y79AA1000426 | F-Y79AA1000426 | 790 | R-Y79AA1000426 | 1537 |
| Y79AA1000521 | F-Y79AA1000521 | 791 | R-Y79AA1000521 | 1538 |
| Y79AA1000750 | F-Y79AA1000750 | 792 | R-Y79AA1000750 | 1539 |
| Y79AA1000776 | F-Y79AA1000776 | 793 | R-Y79AA1000776 | 1540 |
| Y79AA1000777 | F-Y79AA1000777 | 794 | R-Y79AA1000777 | 1541 |
| Y79AA1000876 | F-Y79AA1000876 | 795 | R-Y79AA1000876 | 1542 |
| Y79AA1000888 | F-Y79AA1000888 | 796 | | |
| Y79AA1000959 | F-Y79AA1000959 | 797 | R-Y79AA1000959 | 1543 |
| Y79AA1000967 | F-Y79AA1000967 | 798 | R-Y79AA1000967 | 1544 |
| Y79AA1001013 | F-Y79AA1001013 | 799 | R-Y79AA1001013 | 1545 |
| Y79AA1001056 | F-Y79AA1001056 | 800 | R-Y79AA1001056 | 1546 |
| Y79AA1001062 | F-Y79AA1001062 | 801 | R-Y79AA1001062 | 1547 |
| Y79AA1001090 | F-Y79AA1001090 | 802 | R-Y79AA1001090 | 1548 |
| Y79AA1001212 | F-Y79AA1001212 | 803 | R-Y79AA1001212 | 1549 |
| Y79AA1001264 | F-Y79AA1001264 | 804 | R-Y79AA1001264 | 1550 |
| Y79AA1001272 | F-Y79AA1001272 | 805 | R-Y79AA1001272 | 1551 |
| Y79AA1001328 | F-Y79AA1001328 | 806 | R-Y79AA1001328 | 1552 |
| Y79AA1001426 | F-Y79AA1001426 | 807 | R-Y79AA1001426 | 1553 |
| Y79AA1001427 | F-Y79AA1001427 | 808 | | |
| Y79AA1001430 | F-Y79AA1001430 | 809 | R-Y79AA1001430 | 1554 |
| Y79AA1001523 | F-Y79AA1001523 | 810 | R-Y79AA1001523 | 1555 |
| Y79AA1001530 | F-Y79AA1001530 | 811 | R-Y79AA1001530 | 1556 |
| Y79AA1001592 | F-Y79AA1001592 | 812 | R-Y79AA1001592 | 1557 |
| Y79AA1001727 | F-Y79AA1001727 | 813 | R-Y79AA1001727 | 1558 |
| Y79AA1001787 | F-Y79AA1001787 | 814 | R-Y79AA1001787 | 1559 |
| Y79AA1001793 | F-Y79AA1001793 | 815 | | |
| Y79AA1001795 | F-Y79AA1001795 | 816 | R-Y79AA1001795 | 1560 |
| Y79AA1001799 | F-Y79AA1001799 | 817 | R-Y79AA1001799 | 1561 |
| Y79AA1001803 | F-Y79AA1001803 | 818 | R-Y79AA1001803 | 1562 |
| Y79AA1001863 | F-Y79AA1001863 | 819 | R-Y79AA1001863 | 1563 |
| Y79AA1002022 | F-Y79AA1002022 | 820 | R-Y79AA1002022 | 1564 |
| Y79AA1002058 | F-Y79AA1002058 | 821 | | |
| Y79AA1002121 | F-Y79AA1002121 | 822 | R-nnnnnnnnnnnn | 1565 |
| Y79AA1002129 | F-Y79AA1002129 | 823 | R-nnnnnnnnnnnn | 1566 |
| Y79AA1002213 | F-Y79AA1002213 | 824 | R-Y79AA1002213 | 1567 |
| Y79AA1002334 | F-Y79AA1002334 | 825 | R-Y79AA1002334 | 1568 |
| Y79AA1002373 | F-Y79AA1002373 | 826 | R-Y79AA1002373 | 1569 |
| Y79AA1002376 | F-Y79AA1002376 | 827 | R-Y79AA1002376 | 1570 |
| Y79AA1002378 | F-Y79AA1002378 | 828 | R-Y79AA1002378 | 1571 |
| Y79AA1002381 | F-Y79AA1002381 | 829 | R-Y79AA1002381 | 1572 |
| NT2RP2006580 | F-NT2RP2006580 | 2545 | R-NT2RP2006580 | 2546 |

The sequence name starting from "F" means the name of 5'-end sequence, and the sequence name starting from "R" means the name of 3'-end sequence. A blank indicates that the 3'-end sequence corresponding to the 5'-end sequence has not been determined in the clone.

Furthermore, the present invention relates to the use of the above primers, as described below.
(4) A polynucleotide which can be synthesized with the primer set of (2) or (3).
(5) A polynucleotide comprising a coding region in the polynucleotide of (4).
(6) A substantially pure protein encoded by polynucleotide of (4).
(7) A partial peptide of the protein of (6).

In addition, the present invention comprises a polynucleotide described below and a protein encoded by the polynucleotide.
(8) An isolated polynucleotide selected from the group consisting of
   (a) a polynucleotide comprising a coding region of the nucleotide sequence set forth in any one of the SEQ ID NOs in Table 370;
   (b) a polynucleotide comprising a nucleotide sequence encoding a protein comprising the amino acid sequence set forth in any one of the SEQ ID NOs in Table 370;
   (c) a polynucleotide comprising a nucleotide sequence encoding a protein comprising an amino acid sequence selected from the amino acid sequences set forth in the SEQ ID NOs in Table 370, in which one or more amino acids are substituted, deleted, inserted, and/or added, wherein said protein is functionally equivalent to the protein comprising said amino acid sequence selected from the amino acid sequences set forth in the SEQ ID NOs in Table 370;
   (d) a polynucleotide that hybridizes with a polynucleotide comprising a nucleotide sequence selected from the nucleotide sequences set forth in the SEQ ID NOs in Table 370, and that comprises a nucleotide sequence encoding a protein functionally equivalent to the protein encoded by the nucleotide sequence selected from the nucleotide sequences set forth in the SEQ ID NOs in Table 370;
   (e) a polynucleotide comprising a nucleotide sequence encoding a partial amino acid sequence of a protein encoded by the polynucleotide of (a) to (d);
   (f) a polynucleotide comprising a nucleotide sequence with at least 70% identity to the nucleotide sequence set forth in any one of the SEQ ID NOs in Table 370.
(9). A substantially pure protein encoded by the polynucleotide of (8).
(10) An antibody against the protein or peptide of any one of (6), (7), and (9).
(11) A vector comprising the polynucleotide of (5) or (8).
(12) A transformant carrying the polynucleotide of (5) or (8), or the vector of (11).
(13) A transformant expressively carrying the polynucleotide of (5) or (8), or the vector of (11).
(14) A method for producing the protein or peptide of any one of (6), (7), and (9), comprising culturing the transformant of (13) and recovering the expression product.
(15) An oligonucleotide comprising; the nucleotide sequence set forth in any one of the SEQ ID NOs in Table 370 or the nucleotide sequence complementary to the complementary strand thereof, wherein said oligonucleotide comprises 15 nucleotides or more.
(16) Use of the oligonucleotide of (15) as a primer for synthesizing a polynucleotide.
(17) Use of the oligonucleotide of (15) as a probe for detecting a gene.
(18) An antisense polynucleotide against the polynucleotide of (8), or the portion thereof.
(19) A method for synthesizing a polynucleotide, the method comprising:
   a) synthesizing a complementary strand using a cDNA library as a template, and using the primer set of (2) or (3), or the primer of (16); and
   b) recovering the synthesized product.
(20) The method of (19), wherein the cDNA library is obtainable by oligo-capping method.
(21) The method of (19), wherein the complementary strand is obtainable by PCR.
(22) A method for detecting the polynucleotide of (8), the method comprising:
   a) incubating a target polynucleotide with the oligonucleotide of (15) under the conditions where hybridization occurs, and
   b) detecting the hybridization of the target polynucleotide with the oligonucleotide of (15).
(23) A database of polynucleotides and/or proteins, the database comprising information on at least one sequence selected from the nucleotide sequences set forth in the SEQ ID NOs in Table 370 and/or the amino acid sequences set forth in the SEQ ID NOs in Table 370, or a medium on which the database is stored.

Any patents, patent applications, and publications cited herein are incorporated by reference.

### BRIEF DESCRIPTION OF THE DRAWINGS

Figure 1 shows the restriction maps of vectors pME18SFL3 and pUC19FL3.
Figure 2 shows the reproducibility of gene expression analysis. The ordinate and the abscissa show the intensities of gene expression obtained in experiments different from each other.
Figure 3 shows the detection limit in gene expression analysis. The intensity of expression is shown in the ordinate, and the concentration (µg/ml) of the probe used is shown in the abscissa.

### DETAILED DESCRIPTION OF THE INVENTION

Herein, "polynucleotide" is defined as a molecule in which multiple nucleotides are polymerized. There are no limitations in the number of the polymerized nucleotides. In case that the polymer contains relatively low number of nucleotides, it is also described as an "oligonucleotide". The polynucleotide or the oligonucleotide of the present invention can be a natural or chemically synthesized product. Alternatively, it can be synthesized using a template DNA by an enzymatic reaction such as PCR.

All the cDNA provided by the invention are full-length cDNA. Herein, a "full-length cDNA" is defined as a cDNA which contains both ATG codon (the translation start site) and the stop codon. Accordingly, the untranslated regions, which are originally found in the upstream or downstream of the protein coding region in natural mRNA, may or may not be contained.
An "isolated polynucleotide" is a polynucleotide the structure of which is not identical to that of any naturally occurring nucleic acid or to that of any fragment of a naturally occurring genomic nucleic acid spanning more than three separate genes. The term therefore covers, for example,
(a) a DNA which has the sequence of part of a naturally occurring genomic DNA molecule but is not flanked by both of the coding sequences that flank that part of the molecule in the genome of the organism in which it naturally occurs;
(b) a nucleic acid incorporated into a vector or into the genomic DNA of a prokaryote or eukaryote in a manner such that the resulting molecule is not identical to any naturally occurring vector or genomic DNA;
(c) a separate molecule such as a cDNA, a genomic fragment, a fragment produced by polymerase chain reaction (PCR), or a restriction fragment; and
(d) a recombinant nucleotide sequence that is part of a hybrid gene, i.e., a gene encoding a fusion protein. Specifically excluded from this definition are nucleic acids present in mixtures of different (i) DNA molecules, (ii) transfected cells, or (iii) cell clones: e.g., as these occur in a DNA library such as a cDNA or genomic DNA library.

The term "substantially pure" as used herein in reference to a given polypeptide means that the protein or polypeptide is substantially free from other biological macromolecules. The substantially pure protein or polypeptide is at least 75% (e.g., at least 80, 85, 95, or 99%) pure by dry weight. Purity can be measured by any appropriate standard method, for example, by column chromatography, polyacrylamide gel electrophoresis, or HPLC analysis.

All the clones of the present invention (830 clones) are novel and covering full-length, and also predicted to encode any of the following functional protein:
secretory proteins,
membrane proteins,
proteins associated to signal transduction (signal transduction-associated proteins; e.g. protein kinases, etc.),
proteins associated to a glycoprotein (glycoprotein-associated proteins),
proteins associated with transcription (transcription-associated proteins),
proteins associated with diseases (disease-associated proteins),
or, enzymes and/or metabolism-associated proteins, cell division- and/or cell proliferation-associated proteins, cytoskeleton-associated proteins, nuclear proteins, DNA-and/or RNA-binding proteins, ATP- and/or GTP-binding proteins, protein synthesis- and/or protein transport-associated proteins, and cellular defense-associated proteins.

Furthermore, all the cDNA clones of the present invention can be characterized as follows:
(1) a cDNA that is obtained by the oligo-capping method, which provides cDNA with high fullness ratio. The cDNA was selected by the score in the ATGpr (described as ATGpr1, as well), which is a program for prediction of the fullness of the 5'-end of cDNA based on the features of the 5'-end sequence. In addition, the PSORT, which is a program for prediction of the existence of the signal sequence selected, cDNA that contains a signal sequence in the 5'-end, or transmembrane region in the protein coding region. Furthermore, the homology search with the 5'-end sequences confirmed that, the selected clones were not identical to any of the known human mRNA (namely novel);
   or,
(2) a cDNA that is obtained by the oligo-capping method, which provides cDNA with high fullness ratio. The cDNA was selected by the score in the ATGpr, which is a program for prediction of the fullness of the 5'-end based on the features of the 5'-end sequence. Furthermore, the a cDNA that has relative homology with an amino acid sequence of a protein with known functions was selected by the BLAST search (Altschul S.F., Gish W., Miller W., Myers E.W., and Lipman D.J. (1990) J. Mol. Biol. 215: 403-410 ; Gish W., and States D.J. (1993) Nature Genet. 3: 266-272) on the SwissProt database using the 5'-end sequence. In addition, the homology search using the 5'-end sequence confirmed that the selected clones were not identical to any of the known human mRNA (namely novel).

All clones are obtainable as a full-length clone by such a method as PCR (Current Protocols in Molecular Biology, Ausubel et al. edit, (1987) John Wiley & Sons, Section 6.1-6.4) using both the 5'- and 3'-end sequences, or using the 5'-end sequence and an oligo-dT primer that corresponds to the polyA sequence.

Specifically, PCR can be performed using an oligonucleotide that has 15 nucleotides longer, and specifically hybridizes with the complementary strand of the polynucleotide that contains the nucleotide sequence selected from the 5'-end sequences shown in Table 1 (SEQ ID NO: 1-829, and SEQ ID NO: 2545), and an oligo-dT primer as a 5'-, and 3'-primer, respectively. The length of the primers is usually 15-100 bp, and favorably between 15-35 bp. In case of LA PCR, which is described below, the primer length of 25-35 bp may provide a good result.

A method to design a primer that enables a specific amplification based on the given nucleotide sequence is known to those skilled in the art (Current Protocols in Molecular Biology, Ausubel et al. edit, (1987) John Wiley & Sons, Section 6.1-6.4). In designing a primer based on the 5'-end sequence, the primer is designed so as that, in principle, the amplification products will include the translation start site. Accordingly, in case that a given 5'-end nucleotide sequence is the 5'-untranslated region (5'UTR), any part of the sequence can be used as a 5'-primer as far as the specificity toward the target cDNA is insured. The translation start site can be predicted using a known method such as the ATGpr as described below.

When synthesizing a polynucleotide, the target nucleotide sequence to be amplified can extend to several thousand bp in some cDNA. However, it is possible to amplify such a long nucleotides by using such as LA PCR (Long and Accurate PCR). It is advantageous to use LA PCR when synthesizing long DNA In LA PCR, in which a special DNA polymerase having 3' □5' exonuclease activity is used, misincorporated nucleotides can be removed. Accordingly, accurate synthesis of the complementary strand can be achieved even with a long nucleotide sequence. By using LA PCR, it is reported that amplification of a nucleotide with 20 kb longer can be achieved under desirable condition (Takeshi Hayashi (1996) Jikken-Igaku Bessatsu, "Advanced Technologies in PCR" Youdo-sha).

A template DNA for synthesizing the cDNA of the present invention can be obtained by using cDNA libraries that are prepared by various methods. The full-length cDNA clones obtained here are those with high fullness ratio, which were obtained using a combination of (1) a method to prepare a full-length-enriched cDNA library using the oligo-capping method, and (2) an estimation system for fullness using the 5'-end sequence (selection based on the estimation by the ATGpr after removing clones that are non-full-length compared to the ESTs). However, it is possible to easily obtain a full-length cDNA by using the primers that are provided by the present invention, not by the above described specialized method.

The problem with the cDNA libraries prepared by the known methods or commercially available is that mRNA contained in the libraries has very low fullness ratio. Thus, it is difficult to screen full-length cDNA clone directly from the library using ordinary cloning methods. The present invention has revealed a primer that is capable of synthesizing a full-length cDNA. If provided with primers, it is possible to synthesize a target full-length cDNA by using enzymatic reactions such as PCR. In particular, a full-length-enriched cDNA library, synthesized by methods such as oligo-capping, is desirable to synthesize a full-length cDNA with more reliability.

Once the nucleotide sequences of the full-length cDNAs obtained in the present invention is determined, it is possible to predict the functions of the proteins encoded by the cDNA clones, for example, by searching the databases such as GenBank (http://www.ncbi.nlm.nih.gov/web/GenBank/), Swiss-Prot (http://www.ebi.ac.uk/ebi_docsSwiss-Prot_db/swisshome.html), UniGene (http://www.ncbi.nlm.nih.gov/UniGene) for homologies of the cDNAs, or by searching the amino acid sequences deduced from the full-length nucleotide sequences for signal sequence by using software such as PSORT (K. Nakai & M. Kanehisa, Genomics, 14: 897-991 (1992), for transmembrane region by using software such as SOSUI (T. Hirokawa et al., Bioinformatics, 14:378-379 (1998); Mitsui Knowledge Industry Co., Ltd.) or for motif by using software such as Pfam (http://www.sanger.ac.uk/Software/Pfam/index.shtml) or PROSITE (http://www.expasy.ch/prosite). As a matter of course, the functions are often predictable by using partial sequence information (preferably 300 nucleotides or more) instead of the full-length nucleotide sequences. However, the result of the prediction obtained by using partial sequence information does not always agree with the result obtained by using full-length nucleotide sequence, and thus it is needless to say that the prediction of function is preferably performed based on the full-length nucleotide sequences.

Homology search using each of GenBank, Swiss-Prot and UniGene was performed for the 826 clones whose full-length nucleotide sequences had been determined (HEMBA1005337, NT2RM1000407, NT2RM2001767, and NT2RP3003939 are not full-length). The amino acid sequences deduced from the full-length nucleotide sequences were searched for functional domains by using analytical software programs, PSORT, SOSUI and Pfam. Based on the results, proteins encoded by the cDNA clones were grouped into some categories and their functions were predicted.

The following 437 clones were categorized into secretory and/or membrane proteins. The clones categorized into secretory and/or membrane proteins are those which matched the full-length sequences of Swiss-Prot database with the keywords "growth factor", "cytokine", "hormone", "signal", "transmembrane", "membrane", "extracellular matrix", "receptor", "G-protein coupled receptor", "ionic channel", "voltage-gated channel", "calcium channel", "cell adhesion", "collagen" or "connective tissue"; those which matched the data, suggesting that the proteins are secretory and/or membrane proteins; or those which matched with the full-length sequences of GenBank or UniGene database similar description; and, further, those predicted to have an N-terminal signal sequence or a transmembrane region as a result of domain search for the amino acid sequences deduced from the full-length nucleotide sequences.
BNGH41000020, BNGH41000087, BNGH41000091, HEMBA1000121, HEMBA1000128, HEMBA1000349, HEMBA1000477, HEMBA1000590, HEMBA1000713, HEMBA1000732, HEMBA1000745, HEMBA1000835, HEMBA1000940, HEMBA1000962, HEMBA1001221, HEMBA1001228, HEMBA1001621, HEMBA1002131, HEMBA1002163, HEMBA1002167, HEMBA1002178, HEMBA1002195, HEMBA1002227, HEMBA1002420, HEMBA1002421, HEMBA1002767, HEMBA1003047, HEMBA1003101, HEMBA1003230, HEMBA1003392, HEMBA1003530, HEMBA1003602, HEMBA1003732, HEMBA1003945, HEMBA1004110, HEMBA1004250, HEMBA1004391, HEMBA1004444, HEMBA1004454, HEMBA1004505, HEMBA1004797, HEMBA1004982, HEMBA1005070, HEMBA1005449, HEMBA1005522, HEMBA1005545, HEMBA1005698, HEMBA1005945, HEMBA1006171, HEMBA1006299, HEMBA1006311, HEMBA1006335, HEMBA1006357, HEMBA1006430, HEMBA1006482, HEMBA1006707, HEMBA1006724, HEMBA1006749, HEMBA1006902, HEMBA1006960, HEMBA1007241, HEMBB1000407, HEMBB1000447, HEMBB1000567, HEMBB1000679, HEMBB1000881, HEMBB1001026, HEMBB1001048, HEMBB1001407, HEMBB1001530, HEMBB1001573, HEMBB1001847, HEMBB1001978, HEMBB1002041, HEMBB1002162, HEMBB1002245, HEMBB1002427, HEMBB1002693, MAMMA1000102, MAMMA1000106, MAMMA1000118, MAMMA1000141, MAMMA1000204, MAMMA1000226, MAMMA1000457, MAMMA1000473, MAMMA1000496, MAMMA1000591, MAMMA1000681, MAMMA1000810, MAMMA1000986, MAMMA1000994, MAMMA1001043, MAMMA1001141, MAMMA1001237, MAMMA1001344,
MAMMA1001418, MAMMA1001893, MAMMA1001957, MAMMA1001978,
MAMMA1002070, MAMMA1002091, MAMMA1002095, MAMMA1002165, MAMMA1002234, MAMMA1002586, MAMMA1002633, MAMMA1003126, NT2RM1000462, NT2RM1000542, NT2RM1000580, NT2RM1000855, NT2RM1000858, NT2RM1000899, NT2RM2000241, NT2RM2000410, NT2RM2000423, NT2RM2000565, NT2RM2001626, NT2RM2001792, NT2RM2001939, NT2RM2001941, NT2RM4000198, NT2RM4000284, NT2RM4000417, NT2RM4000444, NT2RM4000587, NT2RM4000593, NT2RM4000648, NT2RM4000761, NT2RM4000997, NT2RM4001325, NT2RM4001735, NT2RM4001768, NT2RM4001843, NT2RM4002352, NT2RP1000050, NT2RP1000181, NT2RP1000261, NT2RP1000300, NT2RP1000325, NT2RP1000448, NT2RP1000551, NT2RP1000613, NT2RP1000981, NT2RP1001004, NT2RP1001563, NT2RP2000479, NT2RP2000533, NT2RP2000616, NT2RP2000649, NT2RP2000663, NT2RP2000694, NT2RP2000818, NT2RP2000903, NT2RP2001200, NT2RP2001276, NT2RP2001480, NT2RP2001495, NT2RP2001514, NT2RP2001755, NT2RP2001915, NT2RP2001956, NT2RP2002063, NT2RP2002188, NT2RP2002232, NT2RP2002527, NT2RP2002533, NT2RP2002721, NT2RP2002824, NT2RP2002942, NT2RP2002976, NT2RP2003042, NT2RP2003210, NT2RP2003383, NT2RP2003390, NT2RP2003469, NT2RP2003593, NT2RP2003655, NT2RP2003664, NT2RP2003950, NT2RP2004179, NT2RP2004205, NT2RP2004495, NT2RP2004524, NT2RP2004556, NT2RP2004606, NT2RP2004648, NT2RP2004794, NT2RP2005027, NT2RP2005163, NT2RP2005181, NT2RP2005378, NT2RP2005463, NT2RP2005541, NT2RP2005597, NT2RP2005666, NT2RP2005883, NT2RP2005994, NT2RP2006004,
NT2RP2006042, NT2RP2006269, NT2RP1006512, NT2RP2006580, NT2RP3000169, NT2RP3000171, NT2RP3000304, NT2RP3000436, NT2RP3000460, NT2RP3000616, NT2RP3000676, NT2RP3000721, NT2RP3000818, NT2RP3000907, NT2RP3000921, NT2RP3001012, NT2RP3001159, NT2RP3001195, NT2RP3001240, NT2RP3001271, NT2RP3001322, NT2RP3001388, NT2RP3001560, NT2RP3001592, NT2RP3001650, NT2RP3001738, NT2RP3001858, NT2RP3002015, NT2RP3002160, NT2RP3002311, NT2RP3002342, NT2RP3002411, NT2RP3002737, NT2RP3002790, NT2RP3002836, NT2RP3002900, NT2RP3002958, NT2RP3003000, NT2RP3003076, NT2RP3003354, NT2RP3003532, NT2RP3003535, NT2RP3003614, NT2RP3004025, NT2RP3004075, NT2RP3004083, NT2RP3004130, NT2RP3004133, NT2RP3004309, NT2RP3004345, NT2RP3004406, NT2RP3004481, NT2RP3004552, NT2RP3004625, NT2RP3004647, NT2RP4001001, NT2RP4001009, NT2RP4001467, NT2RP4001879, NT2RP4002187, NT2RP4002451, NT2RP4002750, OVARC1000003, OVARC1000105, OVARC1000298, OVARC1000307, OVARC1000313, OVARC1000410, OVARC1000439, OVARC1000553, OVARC1000811, OVARC1000873, OVARC1000956, OVARC1001030, OVARC1001163, OVARC1001336, OVARC1001570, OVARC1001607, OVARC1001725, OVARC1001991, PLACE1000033, PLACE1000231, PLACE1000560, PLACE1000740, PLACE1000912, PLACE1000914, PLACE1000927, PLACE1001016, PLACE1001123, PLACE1001183, PLACE1001231, PLACE1001340, PLACE1001401, PLACE1001407, PLACE1001464, PLACE1001516, PLACE1001536, PLACE1001564, PLACE1001655, PLACE1001795,
PLACE1001836, PLACE1001918, PLACE1001949, PLACE1002080, PLACE1002095, PLACE1002355, PLACE1002374, PLACE1002518, PLACE1002547, PLACE1002726, PLACE1002905, PLACE1002911, PLACE1002967, PLACE1003407, PLACE1003573, PLACE1003737, PLACE1003772, PLACE1003839, PLACE1003845, PLACE1003852, PLACE1004279, PLACE1004282, PLACE1004441, PLACE1004450, PLACE1004482, PLACE1004520, PLACE1004630, PLACE1004657, PLACE1004648, PLACE1004816, PLACE1005003, PLACE1005005, PLACE1005031, PLACE1005383, PLACE1005410, PLACE1005426, PLACE1005544, PLACE1005569, PLACE1005660, PLACE1005725, PLACE1005745, PLACE1005878, PLACE1005927, PLACE1006071, PLACE1006093, PLACE1006208, PLACE1006277, PLACE1006290, PLACE1006443, PLACE1006716, PLACE1006809, PLACE1006959, PLACE1007081, PLACE1007096, PLACE1007296, PLACE1007626, PLACE1007845, PLACE1007881, PLACE1008359, PLACE1008469, PLACE1008716, PLACE1008744, PLACE1008985, PLACE1009067, PLACE1009196, PLACE1009279, PLACE1009527, PLACE1009546, PLACE1009600, PLACE1009982, PLACE1010011, PLACE1010078, PLACE1010251, PLACE1010445, PLACE1010713, PLACE1010784, PLACE1010827, PLACE1010968, PLACE1011116, PLACE1011181, PLACE1011236, PLACE1011516, PLACE1011708, PLACE3000181, PLACE3000213, PLACE4000354, SKNMC1000004, SKNMC1000014, SKNMC1000082, THYRO1000036, THYRO1000099, THYRO1000196, THYRO1000400, THYRO1000584, THYRO1000678, THYPO1000776, THYRO1000795, THYRO1000956, THYRO1001102, THYRO1001113,
THYRO1001205, THYRO1001237, THYRO1001242, THYRO1001266, THYRO1001327, THYRO1001456, THYRO1001478, THYRO1001523, THYRO1001529, THYRO1001641, THYRO1001702, THYRO1001725, Y79AA1000207, Y79AA1000226, Y79AA1000270, Y79AA1000426, Y79AA1000521, Y79AA1000876, Y79AA1000888, Y79AA1000959, Y79AA1001013, Y79AA1001212, Y79AA1001264, Y79AA1001328, Y79AA1001426, Y79AA1001427, Y79AA1001430, Y79AA1001727, Y79AA1001787, Y79AA1001795, Y79AA1001799, Y79AA1001803, Y79AA1002022, Y79AA1002058, Y79AA1002129, Y79AA1002213, Y79AA1002373,

The following 146 clones were categorized into glycoprotein-associated proteins. The clones categorized into glycoprotein-associated proteins are those which matched the full-length sequences of Swiss-Prot database with the keyword "glycoprotein"; those which matched the data suggesting that the proteins are glycoprotein; or those which matched the full-length sequences of GenBank or UniGene database with similar description.
BNGH41000087, BNGH41000091, HEMBA1000349, HEMBA1000590, HEMBA1000745, HEMBA1000835, HEMBA1001221, HEMBA1001228, HEMBA1001621, HEMBA1002131, HEMBA1002178, HEMBA1002421, HEMBA1002767, HEMBA1003230, HEMBA1003392, HEMBA1004250, HEMBA1004391, HEMBA1004444, HEMBA1004505, HEMBA1005449, HEMBA1005522, HEMBA1005545, HEMBA1006707, HEMBA1006749, HEMBA1006902, HEMBB1000679, HEMBB1000881, HEMBB1001048, HEMBB1002120, HEMBB1002245, HEMBB1002427, MAMMA1000102, MAMMA1000591, MAMMA1000681, MAMMA1001043, MAMMA1001237, MAMMA1002070, MAMMA1002586, MAMMA1003126, NT2RM1000462, NT2RM1000580, NT2RM2001792, NT2RM2001818, NT2RM2001939, NT2RM2001941, NT2RM4000198, NT2RM4000284, NT2RM4000417, NT2RM4000648, NT2RM4000997, NT2RM4001325, NT2RM4002352, NT2RP1000613, NT2RP1000981, NT2RP1001004, NT2RP2000616, NT2RP2000694, NT2RP2000903, NT2RP2001480, NT2RP2001755, NT2RP2002533, NT2RP2003042, NT2RP2003210, NT2RP2004205, NT2RP2004606, NT2RP2005027, NT2RP2005181, NT2RP2005541, NT2RP2005597, NT2RP2005883, NT2RP2006004, NT2RP2006042, NT2RP2006269, NT2RP3000304, NT2RP3000616, NT2RP3000921, NT2RP3001650, NT2RP3002160, NT2RP3002737, NT2RP3002958, NT2RP3003000, NT2RP3003532, NT2RP3004130, NT2RP3004133, NT2RP3004481, NT2RP3004552, NT2RP3004640, NT2RP4000108, NT2RP4001467, NT2RP4002750, OVARC1000003, OVARC1000553, OVARC1000811, OVARC1000873, OVARC1001336, OVARC1001607, OVARC1001991, PLACE1000033, PLACE1000740, PLACE1001016,
PLACE1001123, PLACE1001231, PLACE1001464, PLACE1001655, PLACE1001836, PLACE1002355, PLACE1002374, PLACE1002905, PLACE1002911, PLACE1003573, PLACE1003737, PLACE1003772, PLACE1003839, PLACE1004282, PLACE1004441, PLACE1004450, PLACE1004520, PLACE1004648, PLACE1005003, PLACE1005426, PLACE1006071, PLACE1006073, PLACE1006290, PLACE1007081, PLACE1007845, PLACE1008716, PLACE1008744, PLACE1008985, PLACE1010251, PLACE1010784, PLACE1010968, PLACE1011116, PLACE3000181, PLACE3000213, PLACE4000354, THYRO1000036, THYRO1000196, THYRO1000584, THYRO1000956, THYRO1001266, Y79AA1000270, Y79AA1000426, Y79AA1001727, Y79AA1001795, Y79AA1002022, Y79AA1002213,

The following 57 clones were categorized into signal transduction-associated proteins. The clones categorized into signal transduction-associated proteins are those which matched the full-length sequences of Swiss-Prot database with the keywords "serine/threonine-protein kinase", "tyrosine-protein kinase" or "SH3 domain"; those which matched the data suggesting that the proteins are signal transduction-associated proteins (for example, "ADP-ribosylation factor"); or those which matched the full-length sequences of GenBank or UniGene database with similar description; and, further, those which was similarly predicted to be signal transduction-associated proteins based on the matching data of Pfam.
HEMBA1000006, HEMBA1002195, HEMBA1002227, HEMBA1002551, HEMBA1005084, HEMBA1005929, HEMBA1006658, HEMBA1006916, MAMMA1000881, MAMMA1001150, MAMMA1001310, MAMMA1002142, NT2RM2001902, NT2RP1001020, NT2RP1001031, NT2RP2001469, NT2RP2001529, NT2RP2001769, NT2RP2003179, NT2RP2003545, NT2RP2004670, NT2RP3000011, NT2RP3000022, NT2RP3000172, NT2RP3000201, NT2RP3000820, NT2RP3003527, NT2RP3003849, NT2RP3003874, NT2RP3004067, NT2RP4000634, NT2RP4000962, OVARC1000255, OVARC1000529, OVARC1000916, OVARC1001338, OVARC1001569, PLACE1002329, PLACE1003135, PLACE1003598, PLACE1005519, PLACE1006208, PLACE1008282, PLACE1008297, PLACE1010081, PLACE1011364, PLACE1011824, THYRO1001457, THYRO1001593, THYRO1001700, THYRO1001770, Y79AA1000777, Y79AA1000967, Y79AA1002376, Y79AA1002381, HEMBB1000668, NT2RM4001377

The following 81 clones were categorized into transcription-associated proteins. The clones categorized into transcription-associated proteins are those which keywords "transcription regulation", "zinc finger" or "homeobox" matched the full-length sequences of Swiss-Prot database; those which the matched the data suggesting that the proteins were transcription-associated proteins; or those which matched the full-length sequences of GenBank or UniGene database with similar description; and, further, those which was similarly predicted to be transcription-associated proteins based on the matching data of Pfam.
HEMBA1000462, HEMBA1000671, HEMBA1001297, HEMBA1001390, HEMBA1001886, HEMBA1002048, HEMBA1003120, HEMBA1003497, HEMBA1004785, HEMBA1005230, HEMBA1005246, HEMBA1006276, HEMBA1006572, HEMBA1007226, HEMBB1000106, HEMBB1000905, HEMBB1001959, HEMBB1002051, HEMBB1002661, MAMMA1001094, MAMMA1001532, MAMMA1001615, NT2RM1000789, NT2RM2000632, NT2RM2000773, NT2RM4000326, NT2RP1000271, NT2RP1000468, NT2RP2000092, NT2RP2000610, NT2RP2000712, NT2RP2000739, NT2RP2001538, NT2RP2001662, NT2RP2001817, NT2RP2001948, NT2RP2002564, NT2RP2002974, NT2RP2003138, NT2RP2003302, NT2RP2003940, NT2RP2004108, NT2RP2004847, NT2RP2005247, NT2RP2005391, NT2RP2005535, NT2RP2005774, NT2RP2005941, NT2RP2006092, NT2RP3000148, NT2RP3000232, NT2RP3000378, NT2RP3000652, NT2RP3001976, NT2RP3004090, NT2RP3004119, NT2RP3004294, OVARC1001049, OVARC1001086, OVARC1001132, OVARC1001807, PLACE1000258, PLACE1000442, PLACE1000907, PLACE1003529, PLACE1004166, PLACE1004168, PLACE1004887, PLACE1005250, PLACE1005682, PLACE1006079, PLACE1008549, PLACE1011407, PLACE1011978, THYRO1000580, Y79AA1000030, Y79AA1001090, Y79AA1001523, Y79AA1002334, Y79AA1002378, HEMBB1002302,

The following 85 clones were categorized into disease-associated proteins. The clones categorized into disease-associated proteins are those which matched the full-length sequences of Swiss-Prot database with the keywords "disease mutation" or "syndrome"; those which matched the data suggesting that the proteins are disease-associated proteins; or those which matched the full-length sequences of Swiss-Prot database and GenBank or UniGene database where the matched sequences are those of genes or proteins which had been deposited in the database of Online Mendelian Inheritance in Man (OMIM) (http://www.ncbi.nlm.nih.gov/Omim/), which is a database of human genes and diseases.
BNGH41000020, HEMBA1000349, HEMBA1000590, HEMBA1000671, HEMBA1000835, HEMBA1001184, HEMBA1001228, HEMBA1001886, HEMBA1003120, HEMBA1004250, HEMBA1005246, HEMBA1005267, HEMBA1006707, HEMBA1006749, HEMBA1006902, HEMBA1006916, HEMBA1007013, HEMBB1002120, MAMMA1000204, MAMMA1002080, NT2RM2000632, NT2RM2001126, NT2RM2001558, NT2RP1000271, NT2RP1000465, NT2RP1000579, NT2RP2000447, NT2RP2000514, NT2RP2000739, NT2RP2001223, NT2RP2001529, NT2RP2001562, NT2RP2002674, NT2RP2003369, NT2RP2004108, NT2RP2004205, NT2RP2005535, NT2RP2005941, NT2RP2006004, NT2RP3000059, NT2RP3000125, NT2RP3000201, NT2RP3000232, NT2RP3000616, NT2RP3000677, NT2RP3000838, NT2RP3000921, NT2RP3001542, NT2RP3002286, NT2RP3002721, NT2RP3002737, NT2RP3002738, NT2RP3004481, OVARC1000208, OVARC1000275, OVARC1000331, OVARC1000410, OVARC1001086, OVARC1001132, OVARC1001607, OVARC1001725, OVARC1001952, PLACE1000258, PLACE1000442, PLACE1000907, PLACE1001100, PLACE1001500, PLACE1002905, PLACE1002967, PLACE1003407, PLACE1003428, PLACE1005005, PLACE1005239, PLACE1005815, PLACE1007028, PLACE1008716, PLACE1011407, PLACE1011978, PLACE2000118, THYPO1000580, THYRO1000866, THYRO1001071, THYRO1001478, Y79AA1001062, Y79AA1001530,

It is unclear, by the analyses so far, whether or not the remaining 212 clones encode proteins belonging to any of the categories of secretory and/or membrane proteins, glycoprotein-associated proteins, signal transduction -associated proteins, transcription-associated proteins or disease-associated proteins. Nonetheless, it is still possible for these clones to encode secretory and/or membrane proteins, glycoprotein-associated proteins, signal transduction-associated proteins, transcription-associated proteins, or disease-associated proteins. On the other hand, some of these clones can be presumed to have functions other than those as secretory and/or membrane proteins, glycoprotein-associated proteins, signal transduction-associated proteins, transcription-associated proteins and disease-associated proteins.

Among the 212 clones, the following clones presumably belong to the categories of enzymes and/or metabolism-associated proteins, cell division- and/or cell proliferation-associated proteins, cytoskeleton-associated proteins, nuclear proteins, DNA- and/or RNA-binding proteins, ATP-and/or GTP-binding proteins, protein synthesis- and/or protein transport-associated proteins, or cellular defense-associated proteins, although it is unclear whether or not the clones belong to any of the categories of secretory and/or transmembrane proteins, glycoprotein-associated proteins, signal transduction-associated proteins, transcription-associated proteins, and disease-associated proteins.

The following 10 clones presumably belong to the category of enzymes and/or metabolism-associated proteins. The clones herein defined as clones presumably belonging to the category of enzymes and/or metabolism-associated proteins matched data containing keywords such as "metabolism", "oxidoreductase" and "E.C. No. (Enzyme commission number)".
HEMBA1003315, HEMBB1002465, MAMMA1000614, NT2RP2000178, NT2RP2001388, NT2RP2001903, NT2RP2002304, NT2RP2005878, NT2RP3001685, PLACE1006219

The following 4 clones presumably belong to the category of cell division- and/or cell proliferation-associated proteins. The cDNA clones were herein defined as clones presumably belonging to the category of cell division- and/or cell proliferation-associated proteins matched data containing keywords such as "cell division", "cell cycle", "mitosis", "chromosomal protein", "cell growth" and "apoptosis".
MAMMA1000403, NT2RM2000497, NT2RP2000394, Y79AA1002121

The following 6 clones presumably belong to the category of cytoskeleton-associated proteins. The cDNA clones were herein defined as clones presumably belonging to the category of cytoskeleton-associated proteins matched data containing keywords such as "structural protein", "cytoskeleton", "actin-binding" and "microtubules".
MAMMA 1001609, NT2RM2000589, NT2RP3000063, PLACE 1004078, PLACE 1004492, PLACE 1008657

The following 7 clones presumably belong to the category of nuclear proteins. The cDNA clones were herein defined as clones presumably belonging to the category of nuclear proteins matched data containing keywords such as "nuclear protein".
HEMBA1001878, HEMBA1002992, MAMMA1000614, NT2RM4000965, NT2RM2001738, NT2RP2001388, Y79AA1002121

The following 5 clones presumably belong to the category of DNA- and/or RNA-binding proteins. The cDNA clones were herein defined as clones presumably belonging to the category of DNA- and/or RNA-binding proteins matched data containing keywords such as "DNA-binding" and "RNA-binding".
HEMBA1003072, HEMBA1006770, HEMBA1007332, NT2RM2000497, Y79AA1002121

The following 7 clones presumably belong to the category of ATP- and/or GTP-binding proteins. The cDNA clones were herein defined as clones presumably belonging to the category of ATP- and/or GTP-binding proteins matched data containing keywords such as "ATP-binding" and "GTP-binding".
HEMBA1002316, MAMMA1001609, NT2RM2000306, NT2RM2000497, NT2RP2000178, NT2RP3003729, PLACE1004305

The following 7 clones presumably belong to the category of protein synthesis- and/or protein transport-associated proteins. The cDNA clones were herein defined as clones presumably belonging to the category of protein synthesis-associated and/or protein transport-associated proteins matched data containing keywords such as "translation regulation", "protein biosynthesis", "amino-acid biosynthesis", "ribosomal protein", "protein transport" and "signal recognition particle".
NT2RM4000965, NT2RP2005069, NT2RP3000481, NT2RP3000789, NT2RP4001877, OVARC1001833, OVARC1002058,

The following 1 clone presumably belongs to the category of cellular defense-associated proteins. The cDNA clones were herein defined as clones presumably belonging to the category of cellular defense-associated proteins matched data containing keywords such as "heat shock", "DNA repair" and "DNA damage".
PLACE1005539

Although it is unclear whether or not 26 out of 174 clones other than the above-mentioned clones belong to any of the above-described categories, these clones are predicted to have some functions, based on the homology search using their full-length sequences thereof. The clone names and the gene definitions found in the result of homology search are shown below, separated with a double-slash mark, //.
HEMBA1000634//Homo sapiens T-cell activation protein (PGR1) gene, complete cds.
HEMBA1002524//Human MHC Class I region proline rich protein mRNA, complete cds.
HEMBA1003399//MVP1 PROTEIN.
HEMBA1005489//Mus musculus semaphorin cytoplasmic domain-associated protein 3A (Semcap3) mRNA, complete cds.
HEMBB1000542//Mus musculus bromodomain-containing protein BP75 mRNA, complete cds.
MAMMA1000788//Bos taurus P14 (p14) mRNA, complete cds.
MAMMA1002128//ABC1 PROTEIN HOMOLOG PRECURSOR.
NT2RM2000514//Homo Sapiens F-box protein Fbx21 (FBX21) mRNA, complete cds.
NT2RM2000622//Mus musculus F-box protein FBL10 mRNA, partial cds.
NT2RM4000100//Homo sapiens Leman coiled-coil protein (LCCP) mRNA, complete cds.
NT2RP2005425//Homo sapiens mRNA for AKAP450 protein.
NT2RP3001170//Mus musculus activity-dependent neuroprotective protein (Adnp) mRNA, complete cds.
NT2RP3002571//Bos taurus mRNA for lyncein.
NT2RP3004557//Human Ki nuclear autoantigen mRNA, complete cds.
OVARC1001596//Homo sapiens Arf-like 2 binding protein BART1 mRNA, complete cds.
PLACE1002153//Homo sapiens TACC2 protein (TACC2) mRNA, partial cds.
PLACE1003163//Homo sapiens DBI-related protein mRNA, complete cds.
PLACE1005736//Human mRNA for BAS-GRIP protein.
PLACE1007702//Mus musculus TRA1 mRNA, complete cds.
PLACE1011045//Homo sapiens E1-like protein mRNA, complete cds.
THYRO1000061//Mus musculus mRNA for UBE-1c1, UBE-1c2, UBE-1c3, complete cds.
THYRO1000964//Drosophila melanogaster Pelle associated protein Pellino (Pli) mRNA, complete cds.
Y79AA1000776//Mus musculus mRNA for GSG1, complete cds.
Y79AA1001056//Homo sapiens MAID protein mRNA, complete cds.
Y79AA1001272//Homo sapiens retinoic acid repressible protein (RARG-1) mRNA, complete cds.
Y79AA1001793//Mus musculus mRNA for GSG1, complete cds.

So far, useful information for presuming the functions are unavailable for the remaining 148 clones, whose names are listed below.
HEMBA1000275, HEMBA1000300, HEMBA1000443, HEMBA1000875, HEMBA1000907, HEMBA1001272, HEMBA1001296, HEMBA1001563, HEMBA1002164, HEMBA1002239, HEMBA1002985, HEMBA1003294, HEMBA1003487, HEMBA1004007, HEMBA1004067, HEMBA1004085, HEMBA1004952, HEMBA1004971, HEMBA1005145, HEMBA1005430, HEMBA1005913, HEMBA1006016, HEMBA1006517, HEMBA1006544, HEMBA1006912, HEMBA1007057, HEMBA1007063, HEMBA1007291, HEMBB1000276, HEMBB1000309, HEMBB1000642, HEMBB1001200, HEMBB1001547, HEMBB1002039, HEMBB1002228, HEMBB1002663, MAMMA1000046, MAMMA1000449, MAMMA1000528, MAMMA1000652, MAMMA1000706, MAMMA1000814, MAMMA1001066, MAMMA1001284, MAMMA1001623, MAMMA1001634, MAMMA1001901, MAMMA1002087, MAMMA1002205, MAMMA1002224, NT2RM2000582, NT2RM2001643, NT2RM4000115, NT2RM4000295, NT2RM4001321, NT2RP1000002, NT2RP1000239, NT2RP1000679, NT2RP1000740, NT2RP1000903, NT2RP2000240, NT2RP2001878, NT2RP2001921, NT2RP2002015, NT2RP2002409, NT2RP2002510, NT2RP2003599, NT2RP2003931, NT2RP2004069, NT2RP2004141, NT2RP2004447, NT2RP2004837, NT2RP2005514, NT2RP2005632, NT2RP2005887, NT2RP2006099, NT2RP2006134, NT2RP3000427, NT2RP3000444, NT2RP3000645, NT2RP3000871, NT2RP3001044, NT2RP3001061, NT2RP3001754, NT2RP3002281, NT2RP3002324, NT2RP3002353, NT2RP3002409, NT2RP3002448, NT2RP3002664, NT2RP3002887, NT2RP3002983, NT2RP3003448, NT2RP3003469, NT2RP3003473, NT2RP3003559, NT2RP3003963, NT2RP3004000, NT2RP3004202, NT2RP3004321,
NT2RP3004355, NT2RP3004374, NT2RP4002715, OVARC1000090, OVARC1000137, OVARC1000467, OVARC1000775, OVARC1000853, OVARC1000995, OVARC1001222, OVARC1001260, OVARC1001727, OVARC1002178, PLACE1000986, PLACE1001114, PLACE1001229, PLACE1001788, PLACE1003438, PLACE1003460, PLACE1003644, PLACE1004028, PLACE1004199, PLACE1004519, PLACE1005601, PLACE1005669, PLACE1005768, PLACE1006515, PLACE1006786, PLACE1007040, PLACE1007077, PLACE1007591, PLACE1007971, PLACE1008984, PLACE1009735, PLACE2000219, PLACE4000455, THYRO1000846, THYRO1000999, THYRO1001063, THYRO1001128, THYRO1001471, THYRO1001495, THYRO1001608, THYRO1001803, Y79AA1000127, Y79AA1000750, Y79AA1001592, Y79AA1001863,

In the 437 clones categorized into secretory and/or membrane proteins by using their full-length sequences, 410 clones were also predicted to encode proteins having functions of secretory and/or membrane proteins by using their partial nucleotide sequences. In the 146 clones categorized into glycoprotein-associated proteins by using their full-length sequences, 124 clones were also predicted to encode proteins having functions of glycoprotein-associated proteins by using their partial nucleotide sequences. In the 57 clones categorized into signal transduction-associated proteins by using their full-length sequences, 46 clones were also predicted to encode proteins having functions of signal transduction-associated proteins by using their partial nucleotide sequences. In the 81 clones categorized into transcription-associated proteins by using their full-length sequences, 57 clones were also predicted to encode proteins having functions of transcription-associated proteins by using their partial nucleotide sequences. In the 85 clones categorized into disease-associated proteins by using their full-length sequences, 6 clones were also predicted to encode proteins having functions of disease-associated proteins by using their partial nucleotide sequences. The number of clones, which were predicted to encode disease-associated proteins based on the full-length nucleotide sequences, is much greater than that predicted based on the partial sequences. The reason is that the full-length sequences were categorized by using the data found in the OMIM database into the category of disease-associated proteins.

In some cases, the predicted functions based on the partial sequences are different from those based on the full-length sequences. The reason is that a protein does not always belong solely to a single category of the above-described functional categories, and therefore, it is possible for the protein to belong to both of the predicted functional categories. Besides, additional functions can be found for the clones classified into these functional categories by further analyses.

The following list shows the cDNA clones predicted and selected on the basis of the partial sequences (5' sequences) as cDNAs encoding secretory and/or membrane proteins, glycoprotein-associated proteins, signal transduction-associated proteins, transcription-associated proteins, or disease-associated proteins.

The clones that are selected by the score in the ATGpr and by the PSORT for the existence of a signal sequence can be expected to encode a secretory or membrane protein since they are predicted to possess the secretion signal or a transmembrane region. The clones that are selected by the score in the ATGpr and by the PSORT for the existence of a signal sequence are listed below (254 clones).
HEMBA1000300 HEMBA1000713 HEMBA1000907
HEMBA1000962 HEMBA1001272 HEMBA1001297
HEMBA1002164 HEMBA1002239 HEMBA1002420
HEMBA1002421 HEMBA1003101 HEMBA1003294
HEMBA1003399 HEMBA1003602 HEMBA1003732
HEMBA1004110 HEMBA1004797 HEMBA1005430
HEMBA1006016 HEMBA1006171 HEMBA1006311
HEMBA1006335 HEMBA1006357 HEMBA1006572
HEMBA1006658 HEMBA1006707 HEMBA1006902
HEMBA1006960 HEMBA1007013 HEMBB1000276
HEMBB1000447 HEMBB1000567 HEMBB1000642
HEMBB1000905 HEMBB1001200 HEMBB1001407
HEMBB1001530 HEMBB1001547 HEMBB1001978
HEMBB1002162 HEMBB1002228 HEMBB1002245
HEMBB1002427 HEMBB1002465 HEMBB1002663
HEMBB1002693 MAMMA1000046 MAMMA1000102
MAMMA1000118 MAMMA1000141 MAMMA1000449
MAMMA1000457 MAMMA1000591 MAMMA1000652
MAMMA1000681 MAMMA1000986 MAMMA1000994
MAMMA1001043 MAMMA1001141 MAMMA1001284
MAMMA1001310 MAMMA1001344 MAMMA1001893
MAMMA1001901 MAMMA1001957 MAMMA1002070
MAMMA1002087 MAMMA1002165 MAMMA1002205
MAMMA1002224 MAMMA1002633 NT2RM2000241
NT2RM2000306 NT2RM2000410 NT2RM2000514
NT2RM2001643 NT2RM2001941 NT2RM4000115
NT2RM4000997 NT2RM4001321 NT2RM4001325
NT2RM4001768 NT2RP1000050 NT2RP1000448
NT2RP1000903 NT2RP1001563 NT2RP2000479
NT2RP2001495 NT2RP2001915 NT2RP2001948
NT2RP2002015 NT2RP2002063 NT2RP2002304
NT2RP2002674 NT2RP2002721 NT2RP2003383
NT2RP2003469 NT2RP2003593 NT2RP2003599
NT2RP2003655 NT2RP2003664 NT2RP2004179
NT2RP2004447 NT2RP2004495 NT2RP2004524
NT2RP2004556 NT2RP2004837 NT2RP2005027
NT2RP2005463 NT2RP2005514 NT2RP2005887
NT2RP2006042 NT2RP2006269 NT2RP3000169
NT2RP3000460 NT2RP3000481 NT2RP3000645
NT2RP3000789 NT2RP3000818 NT2RP3001012
NT2RP3001044 NT2RP3001195 NT2RP3001560
NT2RP3001685 NT2RP3001858 NT2RP3002160
NT2RP3002281 NT2RP3002721 N12RP3002836
NT2RP3002958 NT2RP3003076 NT2RP3003354
NT2RP3003469 NT2RP3003535 NT2RP3003559
NT2RP3003963 NT2RP3004000 NT2RP3004083
NT2RP3004133 NT2RP3004309 NT2RP3004321
NT2RP3004355 NT2RP3004374 NT2RP4001001
NT2RP4001879 NT2RP4002451 NT2RP4002715
OVARC1000208 OVARC1000298 OVARC1000439
OVARC1000775 OVARC1000811 OVARC1000853
OVARC1001222 OVARC1001727 OVARC1001807
OVARC1001833 PLACE1000231 PLACE1000560
PLACE1000740 PLACE1000912 PLACE1000914
PLACE1000927 PLACE1000986 PLACE1001100
PLACE1001183 PLACE1001229 PLACE1001407
PLACE1001536 PLACE1001788 PLACE1002080
PLACE1002095 PLACE1002374 PLACE1002518
PLACE1003407 PLACE1003428 PLACE1003460
PLACE1003839 PLACE1003845 PLACE1004028
PLACE1004199 PLACE1004282 PLACE1004305
PLACE1004482 PLACE1004637 PLACE1005005
PLACE1005250 PLACE1005383 PLACE1005410
PLACE1005544 PLACE1005569 PLACE1005601
PLACE1005660 PLACE1005669 PLACE1005725
PLACE1005768 PLACE1005927 PLACE1006079
PLACE1006093 PLACE1006219 PLACE1006277
PLACE1006443 PLACE1006786 PLACE1006809
PLACE1007040 PLACE1007096 PLACE1007296
PLACE1007626 PLACE1007971 PLACE1008469
PLACE1008984 PLACE1008985 PLACE1009067
PLACE1009196 PLACE1009527 PLACE1009982
PLACE1010251 PLACE1011236 PLACE2000219
PLACE4000455 SKNMC1000004 SKNMC1000014
THYRO1000036 THYRO1000099 THYRO1000196
THYRO1000795 THYRO1000999 THYRO1001237
THYRO1001327 THYRO1001478 THYRO1001495
THYRO1001523 THYRO1001702 THYRO1001725
Y79AA1000226 Y79AA1000270 Y79AA1000426
Y79AA1000521 Y79AA1000776 Y79AA1000959
Y79AA1001013 Y79AA1001056 Y79AA1001264
Y79AA1001328 Y79AA1001427 Y79AA1001430
Y79AA1001530 Y79AA1001592 Y79AA1001793
Y79AA1001795 Y79M1001803 Y79AA1001863
Y79AA1002022 Y79AA1002373

In the example mentioned below, the 254 clones as described above were categorized into three groups according to their maximal value in the ATGpr and the result in the PSORT, which are shown in Table 7-10, 11, 12 (246 clones), and Table 13, 14, 15 (8 clones). In the tables, the name of clone, indicate the name of the clone that was selected by the ATGpr and the PSORT; the name of sequence indicates the name of the 5'-end sequence of the clone on the left; the maximal ATGpr score indicates the maximal ATGpr1 score of the 5'-end sequence shown on the left; and signal indicates the presence of the signal sequence according to the prediction by the PSORT. In addition, the representative sequence is the sequence that has the longest sequence among the cluster in which the 5'-end sequence on the left was included. The maximal ATGpr score and signal on the right indicate the maximal ATGpr1 score of the representative sequence, and the presence of a signal sequence in the representative sequence according to the prediction by the PSORT, respectively. The 170 clones shown in Table 7-10, having the maximal score in the ATGpr1 higher than 0.5, and predicted to possess a signal sequence by the PSORT, are very likely to be full-length and encode a secretory or membrane protein. The 35 clones in Table 11, which have the maximal score in the ATGpr1 0.3 or higher and less than 0.5, and predicted to have a signal sequence, are also as well. And, the 41 clones in Table 12, having the maximal score in the ATGpr1 0 or higher and less than 0.3, and predicted to have a signal sequence, are likely to be full-length and encode a secretory or membrane protein.

The 8 clones in Table 13 (4 clones), Table 14 (2 clones), and Table 15 (2 clones) have the maximal score in the ATGpr1 0.5 or higher, 0.3 or higher and less than 0.5, and 0 or higher and less than 0.3, respectively, and are predicted to have no signal sequence by the PSORT. However, these clones contain a region that is recognized by the PSORT to be a signal sequence within the representative sequence composing the same cluster. Thus, the clones were judged as a full-length clone which encodes a membrane protein, especially.

The clones selected by the score in the ATGpr and by the keywords in the top hit data in the SwissProt are likely to encode a secretory or membrane protein, or proteins with functions associated to signal transduction, glycoprotein, transcription, and diseases according to the respective keywords. These 659 clones are shown below. Here, top hit data is defined to be data of known amino acid sequence which is identified to be the most homologous sequence in homology search using the SwissProt.
BNGH41000020 BNGH41000087 BNGH41000091
HEMBA1000006 HEMBA1000121 HEMBN1000128
HEMBA1000275 HEMBA1000349 HEMBA1000443
HEMBA1000462 HEMBA1000477 HEMBA1000590
HEMBA1000634 HEMBA1000671 HEMBA1000732
HEMBA1000745 HEMBA1000835 HEMBA1000875
HEMBA1000907 HEMBA1000940 HEMBA1001184
HEMBA1001221 HEMBA1001228 HEMBA1001296
HEMBA1001390 HEMBA1001563 HEMBA1001621
HEMBA1001878 HEMBA1001886 HEMBA1002048
HEMBA1002131 HEMBA1002163 HEMBA1002164
HEMBA1002167 HEMBA1002178 HEMBA1002195
HEMBA1002227 HEMBA1002316 HEMBA1002421
HEMBA1002524 HEMBA1002551 HEMBA1002767
HEMBA1002985 HEMBA1002992 HEMBA1003047
HEMBA1003072 HEMBA1003101 HEMBA1003120
HEMBA1003230 HEMBA1003315 HEMBA1003392
HEMBA1003487 HEMBA1003497 HEMBA1003530
HEMBA1003945 HEMBA1004007 HEMBA1004067
HEMBA1004085 HEMBA1004250 HEMBA1004391
HEMBA1004444 HEMBA1004454 HEMBA1004505
HEMBA1004785 HEMBA1004797 HEMBA1004952
HEMBA1004971 HEMBA1004982 HEMBA1005070
HEMBA1005084 HEMBA1005145 HEMBA1005230
HEMBA1005246 HEMBA1005267 HEMBA1005337
HEMBA1005449 HEMBA1005489 HEMBA1005522
HEMBA1005545 HEMBA1005698 HEMBA1005913
HEMBA1005929 HEMBA1005945 HEMBA1006276
HEMBA1006299 HEMBA1006335 HEMBA1006430
HEMBA1006482 HEMBA1006517 HEMBA1006544
HEMBA1006572 HEMBA1006707 HEMBA1006724
HEMBA1006749 HEMBA1006770 HEMBA1006902
HEMBA1006912 HEMBA1006916 HEMBA1007013
HEMBA1007057 HEMBA1007063 HEMBA1007226
HEMBA1007241 HEMBA1007291 HEMBA1007332
HEMBB1000106 HEMBB1000309 HEMBB1000407
HEMBB1000447 HEMBB1000542 HEMBB1000567
HEMBB1000668 HEMBB1000679 HEMBB1000881
HEMBB1001026 HEMBB1001048 HEMBB1001200
HEMBB1001573 HEMBB1001847 HEMBB1001959
HEMBB1002039 HEMBB1002041 HEMBB1002051
HEMBB1002120 HEMBB1002302 HEMBB1002427
HEMBB1002661 MAMMA1000106 MAMMA1000204
MAMMA1000226 MAMMA1000403 MAMMA1000473
MAMMA1000496 MAMMA1000528 MAMMA1000591
MAMMA1000614 MAMMA1000681 MAMMA1000706
MAMMA1000788 MAMMA1000810 MAMMA1000814
MAMMA1000881 MAMMA1001043 MAMMA1001066
MAMMA1001094 MAMMA1001150 MAMMA1001237
MAMMA1001418 MAMMA1001532 MAMMA1001609
MAMMA1001615 MAMMA1001623 MAMMA1001634
MAMMA1001893 MAMMA1001957 MAMMA1001978
MAMMA1002070 MAMMA1002080 MAMMA1002091
MAMMA1002095 MAMMA1002128 MAMMA1002142
MAMMA1002165 MAMMA1002234 MAMMA1002586
MAMMA1002633 MAMMA1003126 NT2RM1000407
NT2RM1000462 NT2RM1000542 NT2RM1000580
NT2RM1000789 NT2RM1000855 NT2RM1000858
NT2RM1000899 NT2RM2000410 NT2RM2000423
NT2RM2000497 NT2RM2000565 NT2RM2000582
NT2RM2000589 NT2RM2000622 NT2RM2000632
NT2RM2000773 NT2RM2001126 NT2RM2001558
NT2RM2001626 NT2RM2001738 NT2RM2001767
NT2RM2001792 NT2RM2001818 NT2RM2001902
NT2RM2001939 NT2RM2001941 NT2RM4000100
NT2RM4000198 NT2RM4000284 NT2RM4000295
NT2RM4000326 NT2RM4000417 NT2RM4000444
NT2RM4000587 NT2RM4000593 NT2RM4000648
NT2RM4000761 NT2RM4000965 NT2RM4001377
NT2RM4001735 NT2RM4001843 NT2RM4002352
NT2RP1000002 NT2RP1000050 NT2RP1000181
NT2RP1000239 NT2RP1000261 NT2RP1000271
NT2RP1000300 NT2RP1000325 NT2RP1000465
NT2RP1000468 NT2RP1000551 NT2RP1000579
NT2RP1000613 NT2RP1000679 NT2RP1000740
NT2RP1000981 NT2RP1001004 NT2RP1001020
NT2RP1001031 NT2RP2000092 NT2RP2000178
NT2RP2000240 NT2RP2000394 NT2RP2000447
NT2RP2000514 NT2RP2000533 NT2RP2000610
NT2RP2000616 NT2RP2000649 NT2RP2000663
NT2RP2000694 NT2RP2000712 NT2RP2000739
NT2RP2000818 NT2RP2000903 NT2RP2001200
NT2RP2001223 NT2RP2001276 NT2RP2001388
NT2RP2001469 NT2RP2001480 NT2RP2001495
NT2RP2001514 NT2RP2001529 NT2RP2001538
NT2RP2001562 NT2RP2001662 NT2RP2001755
NT2RP2001769 NT2RP2001817 NT2RP2001878
NT2RP2001903 NT2RP2001921 NT2RP2001948
NT2RP2001956 NT2RP2002063 NT2RP2002188
NT2RP2002232 NT2RP2002304 NT2RP2002409
NT2RP2002510 NT2RP2002527 NT2RP2002533
NT2RP2002564 NT2RP2002824 NT2RP2002942
NT2RP2002974 NT2RP2002976 NT2RP2003042
NT2RP2003138 NT2RP2003179 NT2RP2003210
NT2RP2003302 NT2RP2003369 NT2RP2003390
NT2RP2003469 NT2RP2003545 NT2RP2003593
NT2RP2003655 NT2RP2003664 NT2RP2003931
NT2RP2003940 NT2RP2003950 NT2RP2004069
NT2RP2004108 NT2RP2004141 NT2RP2004205
NT2RP2004447 NT2RP2004606 NT2RP2004648
NT2RP2004670 NT2RP2004794 NT2RP2004847
NT2RP2005069 NT2RP2005163 NT2RP2005181
NT2RP2005247 NT2RP2005378 NT2RP2005391
NT2RP2005425 NT2RP2005535 NT2RP2005541
NT2RP2005597 NT2RP2005632 NT2RP2005666
NT2RP2005774 NT2RP2005878 NT2RP2005883
NT2RP2005941 NT2RP2005994 NT2RP2006004
NT2RP2006042 NT2RP2006092 NT2RP2006099
NT2RP2006134 NT2RP2006269 NT2RP2006512
NT2RP3000011 NT2RP3000022 NT2RP3000059
NT2RP3000063 NT2RP3000125 NT2RP3000148
NT2RP3000171 NT2RP3000172 NT2RP3000201
NT2RP3000232 NT2RP3000304 NT2RP3000378
NT2RP3000427 NT2RP3000436 NT2RP3000444
NT2RP3000481 NT2RP3000616 NT2RP3000645
NT2RP3000652 NT2RP3000676 NT2RP3000677
NT2RP3000721 NT2RP3000820 NT2RP3000838
NT2RP3000871 NT2RP3000907 NT2RP3000921
NT2RP3001012 NT2RP3001061 NT2RP3001159
NT2RP3001170 NT2RP3001195 NT2RP3001240
NT2RP3001271 NT2RP3001322 NT2RP3001388
NT2RP3001542 NT2RP3001560 NT2RP3001592
NT2RP3001650 NT2RP3001738 NT2RP3001754
NT2RP3001976 NT2RP3002015 NT2RP3002160
NT2RP3002286 NT2RP3002311 NT2RP3002324
NT2RP3002342 NT2RP3002353 NT2RP3002409
NT2RP3002411 NT2RP3002448 NT2RP3002571
NT2RP3002664 NT2RP3002737 NT2RP3002738
NT2RP3002790 NT2RP3002836 NT2RP3002887
NT2RP3002900 NT2RP3002958 NT2RP3002983
NT2RP3003000 NT2RP3003076 NT2RP3003354
NT2RP3003448 NT2RP3003473 NT2RP3003527
NT2RP3003532 NT2RP3003614 NT2RP3003729
NT2RP3003849 NT2RP3003874 NT2RP3003939
NT2RP3004025 NT2RP3004067 NT2RP3004075
NT2RP3004090 NT2RP3004119 NT2RP3004130
NT2RP3004133 NT2RP3004202 NT2RP3004294
NT2RP3004309 NT2RP3004345 NT2RP3004406
NT2RP3004481 NT2RP3004552 NT2RP3004557
NT2RP3004625 NT2RP3004640 NT2RP3004647
NT2RP4000108 NT2RP4000634 NT2RP4000962
NT2RP4001009 NT2RP4001467 NT2RP4001877
NT2RP4001879 NT2RP4002187 NT2RP4002451
NT2RP4002750 OVARC1000003 OVARC1000090
OVARC1000105 OVARC1000137 OVARC1000255
OVARC1000275 OVARC1000307 OVARC1000313
OVARC1000331 OVARC1000410 OVARC1000439
OVARC1000467 OVARC1000529 OVARC1000553
OVARC1000873 OVARC1000916 OVARC1000956
OVARC1000995 OVARC1001030 OVARC1001049
OVARC1001086 0VAPC1001132 OVARC1001163
OVARC1001222 OVARC1001260 OVARC1001336
OVARC1001338 OVARC1001569 OVARC1001570
OVARC1001596 OVARC1001607 OVARC1001725
OVARC1001952 OVARC1001991 OVARC1002058
OVARC1002178 PLACE1000033 PLACE1000258
PLACE1000442 PLACE1000740 PLACE1000907
PLACE1001016 PLACE1001114 PLACE1001123
PLACE1001231 PLACE1001340 PLACE1001401
PLACE1001407 PLACE1001464 PLACE1001500
PLACE1001516 PLACE1001564 PLACE1001655
PLACE1001795 PLACE1001836 PLACE1001918
PLACE1001949 PLACE1002080 PLACE1002095
PLACE1002153 PLACE1002329 PLACE1002355
PLACE1002374 PLACE1002547 PLACE1002726
PLACE1002905 PLACE1002911 PLACE1002967
PLACE1003135 PLACE1003163 PLACE1003428
PLACE1003438 PLACE1003460 PLACE1003529
PLACE1003573 PLACE1003598 PLACE1003644
PLACE1003737 PLACE1003772 PLACE1003852
PLACE1004078 PLACE1004166 PLACE1004168
PLACE1004279 PLACE1004441 PLACE1004450
PLACE1004482 PLACE1004492 PLACE1004519
PLACE1004520 PLACE1004630 PLACE1004648
PLACE1004816 PLACE1004887 PLACE1005003
PLACE1005031 PLACE1005239 PLACE1005383
PLACE1005426 PLACE1005519 PLACE1005539
PLACE1005544 PLACE1005569 PLACE1005682
PLACE1005736 PLACE1005745 PLACE1005815
PLACE1005878 PLACE1005927 PLACE1006071
PLACE1006073 PLACE1006208 PLACE1006277
PLACE1006290 PLACE1006443 PLACE1006515
PLACE1006716 PLACE1006959 PLACE1007028
PLACE1007077 PLACE1007081 PLACE1007096
PLACE1007296 PLACE1007591 PLACE1007702
PLACE1007845 PLACE1007881 PLACE1008282
PLACE1008297 PLACE1008359 PLACE1008469
PLACE1008549 PLACE1008657 PLACE1008716
PLACE1008744 PLACE1008984 PLACE1008985
PLACE1009279 PLACE1009527 PLACE1009546
PLACE1009600 PLACE1009735 PLACE1010011
PLACE1010078 PLACE1010081 PLACE1010251
PLACE1010445 PLACE1010713 PLACE1010784
PLACE1010827 PLACE1010968 PLACE1011045
PLACE1011116 PLACE1011181 PLACE1011236
PLACE1011364 PLACE1011407 PLACE1011516
PLACE1011708 PLACE1011824 PLACE1011978
PLACE2000118 PLACE3000181 PLACE3000213
PLACE4000354 SKNMC1000014 SKNMC1000082
THYRO1000061 THYRO1000196 THYRO1000400
THYRO1000580 THYRO1000584 THYRO1000678
THYRO1000776 THYRO1000795 THYRO1000846
THYRO1000866 THYRO1000956 THYRO1000964
THYRO1001063 THYRO1001071 THYRO1001102
THYRO1001113 THYRO1001128 THYRO1001205
THYRO1001242 THYRO1001266 THYRO1001456
THYRO1001457 THYRO1001471 THYRO1001478
THYRO1001529 THYRO1001593 THYRO1001608
THYRO1001641 THYRO1001700 THYRO1001702
THYRO1001770 THYRO1001803 Y79AA1000030
Y79AA1000127 Y79AA1000207 Y79AA1000270
Y79AA1000426 Y79AA1000750 Y79AA1000777
Y79AA1000876 Y79AA1000888 Y79AA1000967
Y79AA1001062 Y79AA1001090 Y79AA1001212
Y79AA1001272 Y79AA1001426 Y79AA1001523
Y79AA1001727 Y79AA1001787 Y79AA1001799
Y79AA1001803 Y79AA1001863 Y79AA1002058
Y79AA1002121 Y79AA1002129 Y79AA1002213
Y79AA1002334 Y79AA1002376 Y79AA1002378
Y79AA1002381 NT2RP2006580

Among the clones, the following 83 clones are identical to the clones selected by the score in the ATGpr and the prediction by the PSORT for the existence of a signal sequence.
HEMBA1000907 NT2RM2000410 PLACE1000740
HEMBA1002164 NT2RM2001941 PLACE1001407
HEMBA1002421 NT2RP1000050 PLACE1002080
HEMBA1003101 NT2RP2001495 PLACE1002095
HEMBA1004797 NT2RP2001948 PLACE1002374
HEMBA1006335 NT2RP2002063 PLACE1003428
HEMBA1006572 NT2RP2002304 PLACE1003460
HEMBA1006707 NT2RP2003469 PLACE1004482
HEMBA1006902 NT2RP2003593 PLACE1005383
HEMBA1007013 NT2RP2003655 PLACE1005544
HEMBB1000447 NT2RP2003664 PLACE1005569
HEMBB1000567 NT2RP2004447 PLACE1005927
HEMBB1001200 NT2RP2006042 PLACE1006277
HEMBB1002427 NT2RP2006269 PLACE1006443
MAMMA1000591 NT2RP3000481 PLACE1007096
MAMMA1000681 NT2RP3000645 PLACE1007296
MAMMA1001043 NT2RP3001012 PLACE1008469
MAMMA1001893 NT2RP3001195 PLACE1008984
MAMMA1001957 NT2RP3001560 PLACE1008985
MAMMA1002070 NT2RP3002160 PLACE1009527
MAMMA1002165 NT2RP3002836 PLACE1010251
MAMMA1002633 NT2RP3002958 PLACE1011236
NT2RP3003076 SKNMC1000014
NT2RP3003354 THYPO1000196
NT2RP3004133 THYRO1000795
NT2RP3004309 THYRO1001478
NT2RP4001879 THYRO1001702
NT2RP4002451 Y79AA1000270
OVARC1000439 Y79AA1000426
OVARC1001222 Y79AA1001803
Y79AA1001863

The 446 clones in Table 16, 17, 18, 19, and 20, and NT2RP2006580 are predicted to encode a secretory or membrane protein. Among them, 77 clones were identical to the clones selected by the score in the ATGpr and the prediction by the PSORT for the existence of a signal sequence (overlapping with any of the 254 clones listed in Table 7-15). Besides, many clones were turned out to be identical to the clones selected as a protein associated with a glycoprotein. Also, there were clones identical to those selected as a protein associated with a disease.

The 243 clones in Table 21 are predicted to encode a glycoprotein. Among them, 53 clones were identical to those selected by the score in the ATGpr and the prediction by the PSORT for the existence of a signal sequence. And, many clones were turned out to be identical to the clones selected as a secretory or membrane protein. Moreover, there were clones identical to those selected as a protein associated with a disease.

The 51 clones in Table 22 are predicted to encode a protein associated to signal transduction.

The 130 clones in Table 23 are predicted to encode a protein associated to transcription.

The 17 clones in Table 24 are predicted to encode a protein associated with diseases.

In these clones, 532 clones have the maximal ATGpr1 score of 0.5 or higher (Table 25). 60 clones have the maximal ATGpr1 score of 0.3 or higher and less than 0.5 (Table 26 and NT2RP2006580). And 67 clones were with the maximal ATGpr1score of 0 or higher and less than 0.3 (Table 27).

532 clones shown in Table 25, each having the maximal score in the ATGpr1 0.5 or higher, are very likely to be full-length and encode a secretory or membrane protein, or proteins associated to signal transduction, glycoprotein, transcription, or diseases. 59 clones in Table 26 and NT2RP2006580, which have the maximal score in the ATGpr1 0.3 or higher and less than 0.5, are likely to be full-length and encode a secretory or membrane protein, or proteins associated to signal transduction, glycoprotein, transcription, or diseases. 67 clones in Table 27, having the maximal score in the ATGpr1 0 or higher and less than 0.3, are still likely to be full-length and encode a secretory or membrane protein, or proteins associated to signal transduction, glycoprotein, transcription, or diseases.

This is the method for selecting the cDNA clones predicted to encode secretory and/or transmembrane proteins, glycoprotein-associated proteins, signal transduction-associated proteins, transcription-associated proteins, or disease-associated proteins on the basis of the partial sequences (5' sequences).

The polynucleotide of the present invention encodes an amino acid sequence of a functional protein such as a secretory or membrane protein, or a protein associated to signal transduction, glycoprotein, transcription, or diseases. Since the protein has the complete amino acid sequence, it is possible to analyze its biological activity by expressing the protein as a recombinant protein using an appropriate expression system, or by raising and using an antibody which specifically recognizes it.

It is possible to analyze the biological activity of a secretory protein or a membrane protein, or proteins associated to signal transduction, glycoprotein, or transcription, based on the methods in "Gene Transcription" (Hames B.D., and Higgins S.J. edit, (1993)), "Glycobiology" (Fukuda M., and Kobata A. edit, (1993)), "Growth Factors" (McKay I., and Leigh I. edit, (1993)), "Extracellular Matrix" (Haralson M.A., and Hassell J.R. edit, (1995), "Transcription Factors" (Latchman D.S. edit, (1993)), "Signal Transduction" (Milligans G. edit, (1992)), featured in "The Practical Approach Series" (IRL PRESS), or "Signal Transduction Protocols" (Kendall D.A., and Hill S.J. edit, (1995), "Glycoprotein Analysis in Biomedicine" (Hounsell E.F. edit, (1993)), featured in "Method in Molecular Biology" (Humana Press).

As to a protein associated with a disease, it is possible to perform a functional analysis as described above, but also possible to analyze correlation between the expression or the activity of the protein and a certain disease by using a specific antibody that recognizes the protein. Alternatively, it is possible to utilize the database Online Mendelian Inheritance in Man (OMIM) (http://www.ncbi.nlm.nih.gov/Omim/), which is a database of human genes and diseases, to analyze the protein. New information is constantly being deposited in the OMIM database. Therefore, it is possible for one skilled in the art to find a new relationship between a particular disease and a gene of the present invention in the updated database.

Proteins associated with diseases are useful in drug development as they can be utilized as a diagnostic marker, a drug that regulates the level of their expression and activities, or a target of gene therapy. Also, as for a secretory protein, membrane protein, or proteins associated with signal transduction, glycoprotein, or transcription, search of the OMIM with the keywords mentioned below revealed that the proteins are associated with many diseases. Also, relationship between a proteins associated to signal transduction or transcription and diseases is reported in "Transcription Factor Research-1999" (Fujii, Tamura, Morohashi, Kageyama, and Satake edit, (1999) Jikken-Igaku Zoukan, Vol.17, No.3), and "Gene Medicine" ((1999) Vol.3, No.2). Thus, not only a protein associated with diseases, but also a secretory protein, membrane protein, or protein associated with signal transduction, glycoprotein, or transcription is involved in diseases, suggesting these proteins also are very important as a target in medical industry.

Keywords used in the search of the OMIM
(1) secretion protein
(2) membrane protein
(3) channel
(4) extracellular matrix
(5) receptor
(6) glycoprotein
(7) protein kinase
(8) calmodulin kinase
(9) transcription factor

Shown in the search result are only the accession numbers in the OMIM. Using the number, data showing the relationship between a disease and a gene or protein can be seen. The OMIM data has been renewed everyday.
1) Secretion protein
   268 entries found, searching for "secretion protein"
   104760, 176860, 160900, 107400, 118910, 139320, 603850, 147572, 176880, 600946, 603215, 157147, 600174, 151675, 170280, 179512, 179513, 138120, 179509, 246700, 179510, 600626, 179511, 600998, 109270, 601489, 154545, 179490, 185860, 603216, 122559, 601746, 147290, 602672, 146770, 603062, 179508, 131230, 601591, 602421, 139250, 167805, 167770, 600041, 600564, 118825, 601146, 300090, 600753, 601652, 600759, 600768, 602434, 182590, 603166, 308230, 602534, 603489, 107470, 150390, 104610, 173120, 158106, 143890, 306900, 308700, 134797, 137350, 227500, 176300, 107730, 600760, 138079, 120180, 120160, 120150, 124092, 138160, 101000, 227600, 600509, 601199, 142410, 104311, 193400, 201910, 107300, 122560, 272800, 217000, 590050, 147670, 133170, 176730, 300300, 134370, 274600, 120140, 162151, 158070,
   152790, 120120, 106100, 300200, 192340, 190160, 138040, 147470, 147620, 173350, 147380, 152200, 152760, 157145, 153450, 264080, 113811, 600937, 600840, 188545, 202110, 600514, 186590, 603372, 136435, 137241, 252800, 214500, 207750, 138850, 139191, 142640, 138130, 189907, 603692, 600633, 603355, 107270, 600377, 147892, 232200, 600281, 232800, 602358, 137035, 601771, 601769, 253200, 601933, 118444, 600270, 120700, 600945, 603732, 147660, 600761, 172400, 600823, 600877, 130080, 171060, 107740, 307800, 602843, 130660, 152780, 124020, 601124, 601340, 601604, 601610, 171050, 312060, 232700, 300159, 142703, 600734, 125255, 168450, 123812, 188540, 147940, 188450, 600839, 182452, 188400, 182280, 176760, 263200, 600264, 188826, 252650, 601185, 162641, 137216, 601398, 601538, 118888, 118445, 601745,
   190180, 601922, 182098, 602008, 147440, 602384, 600031, 109160, 602663, 151670, 602682, 602730, 602779, 146880, 603061, 142704, 603140, 106150, 600732, 153620, 603318, 139392, 600042, 102200, 603493, 182100, 264300, 603795, 184600
2) Membrane protein
   1017 entries found, searching for "membrane protein"
   130500, 305360, 153330, 173610, 170995, 109270, 170993, 309060, 120920, 602333, 133740, 133710, 602690, 133730, 159430, 600897, 133090, 601178, 602413, 602003, 109280, 603237, 602173, 107776, 602334, 125305, 602335, 182879, 154045, 309845, 600594, 603718, 603241, 603214, 603657, 603177, 600182, 601476, 602879, 136950, 600723, 601114, 185880, 185881, 300096, 602257, 160900, 177070, 603062, 603344, 602977, 310200, 600959, 300100, 186945, 600039, 600267, 128240, 182900, 601097, 136430, 600946, 602534, 601047, 143450, 603141, 603700, 600579, 256540, 159440, 602414, 600403, 602048, 188860, 137290, 158343, 184756, 602910, 603179, 600279, 108733, 107770, 173335, 602625, 154050, 219800, 603850, 601028, 600447, 104225, 186946, 601767, 603143, 121015, 603215, 227400, 603735, 600179, 602421, 180721,
   176801, 176860, 600753, 603142, 176790, 600266, 601239, 115501, 143890, 121014, 121011, 125950, 603534, 304040, 601134, 600754, 601510, 601595, 190315, 300172, 602216, 602261, 602262, 602461, 131560, 179514, 179512, 176981, 142461, 139310, 312080, 176640, 128239, 185470, 310300, 601403, 601757, 273800, 151460, 176943, 104311, 168468, 120130, 602887, 600164, 601531, 601832, 104775, 600040, 603583, 176894, 602631, 166945, 182180, 120620, 141180, 601014, 139150, 182860, 177061, 600174, 180069, 191275, 104760, 601693, 300017, 603518, 601009, 134651, 601107, 103868, 600168, 136425, 603531, 603291, 600917, 603216, 102720, 300118, 179590, 135630, 602285, 107450, 602296, 303630, 176878, 120090, 600322, 138160, 601212, 603293, 131230, 112205, 600763, 600718, 300187, 170715, 601966, 300051, 602474,
   120070, 600691, 600855, 182309, 602101, 602857, 194355, 162230, 600874, 113730, 155550, 602701, 306400, 601789, 231200, 107271, 175100, 182870, 305100, 301000, 601313, 157147, 147670, 139200, 603593, 157655, 600934, 155970, 602049, 155960, 155760, 118990, 135620, 308230, 602694, 162060, 300023, 160993, 153619, 153432, 120131, 603823, 603167, 601023, 600816, 165040, 601681, 166490, 300112, 120190, 300145, 163970, 600772, 602926, 602933, 602202, 400015, 151510, 600759, 602672, 602654, 603821, 116952, 151430, 602632, 155975, 602217, 150370, 600752, 601179, 600932, 603048, 603234, 601805, 603822, 603869, 601717, 601181, 313440, 139130, 107777, 109190, 603452, 191163, 191164, 602370, 176877, 103195, 600523, 191328, 601275, 204200, 602426, 603820, 600551, 600695, 600552, 600553, 602306, 601523,
   602507, 602299, 600583, 114070, 600632, 603498, 185430, 600587, 235200, 173470, 603199, 601633, 602500, 208900, 180297, 156225, 516020, 190195, 141900, 102680, 193300, 101000, 193400, 300011, 107400, 257220, 107741, 180380, 203200, 111700, 600024, 304800, 600065, 110750, 179605, 113705, 601638, 222900, 120120, 602509, 602469, 600930, 601383, 176261, 602574, 602997, 311770, 131550, 603616, 308700, 603372, 256100, 224100, 276903, 305900, 516000, 131195, 314555, 601567, 603866, 306900, 103390, 186720, 173850, 601050, 602505, 186590, 246530, 602689, 194380, 300041, 162643, 152790, 120150, 600682, 600106, 272750, 188040, 602382, 601497, 113811, 182138, 212138, 601309, 109690, 114760, 176805, 601253, 123900, 602581, 189980, 191190, 110700, 600163, 137167, 600580, 601610, 190000, 123825, 603491,
   600135, 186591, 173910, 138140, 107266, 120950, 601081, 603690, 244400, 312700, 171060, 601199, 601758, 170500, 277900, 601997, 314850, 601880, 603009, 120220, 603126, 164920, 602934, 164730, 163890, 603434, 107269, 602909, 600877, 256550, 164761, 602872, 120110, 126150, 158070, 266200, 223360, 250800, 269920, 252650, 603355, 154582, 138190, 300035, 602640, 227650, 158120, 153700, 182380, 155740, 204500, 603401, 601975, 300135, 136350, 602924, 300167, 185050, 176100, 300189, 151525, 300200, 165180, 230800, 602158, 602676, 603411, 193245, 120325, 601848, 192500, 603102, 147795, 245900, 137060, 147557, 120650, 602377, 307800, 120930, 308100, 142800, 191092, 232300, 173510, 602225, 180470, 190930, 186357, 134638, 600544, 601373, 600509, 600359, 603784, 600395, 600653, 603754, 601597, 601066,
   600185, 601295, 600978, 205400, 603274, 600418, 600839, 516050, 601691, 601007, 600650, 600308, 603261, 601193, 600004, 600017, 516040, 253800, 276901, 600019, 257200, 108780, 300037, 300104, 300126, 255125, 203300, 300191, 426000, 302060, 304700, 201475, 252010, 193210, 311030, 306250, 248600, 191740, 108360, 131244, 600423, 232200, 191305, 231680, 103320, 190180, 600493, 111200, 226200, 312600, 600170, 111680, 186910, 203100, 600536, 600238, 186830, 186760, 186745, 186711, 106180, 111203, 103180, 182530, 182160, 600644, 307030, 192321, 600667, 125647, 179080, 114207, 114860, 176000, 116930, 600748, 173515, 173325, 600377, 171760, 171050, 118425, 170260, 191315, 600798, 600821, 600823, 600444, 600840, 159465, 600857, 158380, 600867, 154360, 152427, 150330, 110900, 147840, 147360, 147280,
   146880, 312610, 120940, 142871, 142790, 600937, 142600, 134390, 111250, 600979, 600997, 142460, 186845, 134635, 601017, 139191, 139090, 138850, 601040, 138720, 122561, 131100, 123610, 217070, 100500, 603377, 602354, 603302, 603207, 603086, 602188, 602095, 603867, 603842, 603798, 602602, 601194, 602607, 603713, 603681, 601252, 603648, 603646, 603644, 601282, 601284, 603667, 603712, 603594, 601872, 603425, 601843, 603263, 603208, 601411, 603201, 603189, 601463, 603164, 603152, 603087, 602874, 601492, 602893, 602057, 602859, 602746, 603879, 603510, 602458, 603380, 601581, 603765, 603283, 601599, 601733, 601852, 602316, 601615, 601617, 602184, 602894, 603005, 603030, 603861, 602835, 602136, 600153, 600074, 600046, 600023, 601625, 516006, 600018, 600016, 516002, 601590, 313475, 313470, 600244,
   600528, 601611, 600282, 600327, 601568, 600368, 601730, 601535, 601745, 601929, 300169, 300150, 300132, 601533, 600385, 600464, 600424, 600429, 601756, 601488, 516005, 251100, 516004, 600918, 516003, 602192, 516001, 240500, 600465, 602241, 602243, 230200, 601485, 601478, 601416, 602297, 601459, 601839, 602314, 193065, 193001, 191306, 600504, 601020, 191191, 602372, 190181, 600534, 188380, 186854, 186360, 600530, 185250, 182331, 600535, 182305, 601296, 600582, 600732, 600734, 600742, 600782, 176802, 176266, 600769, 601883, 600864, 601901, 176260, 173490, 600910, 601905, 171890, 600916, 601987, 602679, 162651, 161555, 160994, 602714, 602715, 602724, 602736, 300007, 602783, 275630, 602836, 270200, 602871, 159460, 602876, 154540, 153900, 602890, 601153, 602190, 602905, 153634, 153337, 602914,
   152310, 151690, 151625, 602935, 602974, 150325, 602992, 150320, 250790, 603006, 603007, 603008, 150292, 233690, 603046, 150210, 603061, 147940, 603063, 221770, 223100, 603097, 147880, 603118, 147730, 146928, 146630, 142622, 603149, 603150, 603151, 600923, 138981, 138590, 138330, 216950, 603192, 138297, 603202, 601002, 602343, 138230, 136131, 603217, 603220, 134660, 131390, 131235, 603242, 603243, 130130, 602345, 126455, 601123, 126064, 125240, 602359, 603312, 602380, 603318, 123890, 123836, 603356, 603361, 603366, 123830, 179610, 188060, 123620, 120980, 186355, 118510, 114835, 114217, 113810, 603499, 182310, 111740, 109610, 603548, 603564, 108740, 603598, 603613, 107273, 603626, 602518, 179410, 603647, 602515, 603652, 106195, 602573, 178990, 105210, 104615, 167055, 603717, 104614, 603728,
   104210, 603749, 603750, 103850, 602608, 603787, 603788, 603796, 173445, 103220, 102910, 102681, 102670, 102642, 603833, 173391, 102576, 102575, 171833, 102573, 101800, 603875, 601108
3) Channel
   272 entries found, searching for "channel"
   176266, 600724, 170500, 182390, 123825, 114208, 114205, 601784, 114206, 600937, 114204, 603415, 600053, 114209, 114207, 600760, 118425, 601011, 192500, 176261, 600761, 176260, 600359, 600228, 600877, 602235, 300008, 182389, 182391, 601328, 601534, 600504, 602323, 601958, 602780, 602781, 601327, 601012, 600734, 603208, 182392, 603220, 603219, 603888, 600054, 602232, 601745, 603537, 602604, 603796, 302910, 602866, 601013, 602905, 602906, 600163, 152427, 180901, 600702, 600308, 602754, 107776, 602024, 314555, 601949, 600235, 602023, 176263, 600681, 176265, 193245, 603305, 176258, 602983, 601219, 601141, 176267, 602343, 602726, 138253, 176262, 600003, 600397, 602872, 138249, 600843, 600935, 600580, 600845, 602158, 602106, 176264, 300110, 176257, 602717, 603493, 176268, 600932, 602727, 138254,
   603652, 300138, 602420, 600570, 600150, 603583, 602345, 603749, 601142, 176256, 600846, 138252, 602982, 603787, 602836, 603788, 602566, 603651, 602421, 100690, 107777, 100725, 100710, 600509, 603061, 154275, 304040, 154276, 180902, 121014, 602368, 139311, 601383, 108745, 601313, 601042, 600131, 186360, 600109, 600229, 600170, 603319, 601485, 118503, 180903, 602076, 124030, 601059, 601212, 601218, 147450, 600855, 600919, 601154, 601157, 171060, 600968, 182139, 131230, 121015, 600421, 113730, 249210, 310500, 600637, 125950, 118800, 156490, 602974, 104610, 121011, 602522, 118504, 300041, 160900, 601382, 602103, 600465, 602014, 600442, 601109, 602481, 277900, 254210, 138247, 164920, 170280, 171050, 128100, 173910, 600884, 123885, 602887, 600232, 180297, 137192, 600304, 138251, 603053, 300103,
   603152, 603199, 118511, 118508, 138079, 600983, 182307, 603324, 305990, 603418, 114080, 232200, 600046, 600040, 602403, 603750, 603785, 104210, 600019, 600300, 182860, 603852, 603853, 603855, 516060
4) Extracellular matrix
   167 entries found, searching for "extracellular matrix"
   603479, 602201, 601418, 601548, 154870, 115437, 602285, 602262, 602261, 134797, 600754, 120361, 116935, 602263, 603320, 601807, 603321, 185250, 185261, 253700, 128239, 120324, 193300, 276901, 308700, 600514, 600261, 602109, 120140, 120150, 147557, 193400, 600536, 188826, 120180, 118661, 120320, 152200, 135821, 112260, 230740, 602090, 155760, 192975, 190182, 602108, 601463, 186745, 600900, 600985, 600758, 602369, 179590, 601211, 600065, 602178, 600262, 182888, 182889, 151510, 182120, 150325, 190181, 150370, 186355, 193065, 165070, 154705, 147559, 146650, 146640, 153619, 175100, 187380, 231050, 188060, 135820, 156790, 130660, 301870, 128240, 600076, 600119, 193210, 600215, 600245, 121010, 150240, 600309, 600491, 222600, 120328, 600564, 600596, 600616, 600700, 600742, 120325, 138297, 600930,
   156225, 601028, 601050, 601105, 253800, 601284, 601313, 120280, 310200, 601492, 120250, 601587, 601636, 601652, 601692, 601728, 120220, 601915, 602048, 155120, 310300, 120210, 120165, 120120, 118940, 116930, 602264, 116806, 602366, 120470, 602415, 602428, 602453, 602505, 602574, 603005, 603196, 603221, 603319, 107269, 216550, 103320, 603489, 603551, 603767, 603799, 603842
5) Receptor (including membrane proteins, and also including transcription factors, since nuclear proteins were not excluded in the search)
   1606 entries found, searching for "receptor"
   600408, 176943, 107770, 601531, 143890, 313700, 162643, 202200, 147670, 191306, 182131, 192321, 138249, 190120, 600264, 162321, 603613, 603614, 602164, 138251, 182134, 138040, 152790, 602393, 133430, 601769, 304800, 147280, 168468, 147730, 155555, 191190, 109760, 122561, 136537, 602034, 147138, 187930, 312861, 107450, 138981, 182135, 603612, 191191, 600933, 601267, 600168, 120650, 182133, 601919, 108962, 162323, 102576, 600022, 603366, 600052, 136430, 601835, 600825, 600979, 102981, 192974, 602779, 601828, 603499, 601972, 191315, 602601, 603611, 602943, 601300, 191316, 108960, 601299, 147810, 602909, 601834, 108961, 602392, 602910, 603372, 106165, 601199, 180901, 131244, 107470, 136435, 146740, 164920, 139191, 138971, 109690, 100690, 138850, 118504, 147781, 146928, 601373, 126449,
   104225, 100725, 164342, 104220, 118503, 136538, 137241, 601663, 601805, 306250, 600377, 600337, 600509, 600983, 126110, 118507, 602548, 162010, 601268, 147671, 601284, 113503, 109190, 603826, 154540, 147569, 600956, 138245, 139139, 155541, 137192, 118445, 104250, 300119, 118511, 601159, 100710, 305660, 104210, 114610, 138253, 146929, 602778, 135630, 147140, 146790, 147045, 601998, 158378, 131243, 600041, 118508, 137142, 113995, 138247, 182137, 147811, 182132, 138248, 600414, 600563, 137160, 138032, 601496, 138252, 138254, 601600, 173800, 146760, 179590, 180903, 109635, 300038, 138244, 182139, 600849, 600644, 601296, 602282, 179611, 143090, 100710, 601076, 600380, 170998, 600845, 118505, 118510, 190160, 104260, 137164, 137140, 600456, 147880, 137190, 601156, 601956, 100730, 601194, 602717,
   173880, 600042, 180902, 300093, 602423, 602469, 603028, 601437, 146933, 179610, 173510, 600843, 305915, 137143, 603361, 603031, 137141, 136539, 162332, 118509, 155540, 600233, 601109, 601743, 600409, 147139, 131235, 603506, 603455, 151445, 602408, 601681, 603540, 602454, 602490, 600214, 600798, 600232, 602729, 600730, 601122, 120577, 160994, 603248, 602621, 601305, 176946, 118502, 176888, 602167, 138246, 158050, 601470, 603453, 603065, 137163, 137166, 600846, 118495, 138243, 192975, 138060, 602757, 191311, 603652, 120340, 600315, 136425, 118493, 600731, 601502, 601503, 601751, 603651, 602345, 118496, 601598, 602343, 118494, 186880, 134934, 601007, 600946, 186930, 186970, 133171, 308380, 120620, 126452, 190010, 126450, 186810, 180220, 173410, 188070, 131550, 109691, 147679, 180240, 173490,
   300034, 264080, 114131, 146661, 147370, 147545, 136352, 190182, 186780, 126453, 110700, 147851, 308385, 126451, 128239, 600018, 601487, 601040, 305990, 601604, 190181, 188545, 162641, 600445, 231200, 136350, 601586, 164770, 600073, 602376, 138491, 118946, 176761, 176806, 601770, 180245, 164870, 602691, 600939, 109610, 192977, 159530, 602997, 164860, 300007, 146710, 601757, 150370, 118444, 600239, 138033, 601167, 108360, 162642, 173850, 603881, 180246, 193210, 173470, 601469, 134935, 182452, 138492, 135620, 601523, 601790, 600221, 109630, 602451, 147265, 602626, 602001, 602069, 601642, 600848, 102776, 180190, 182098, 176886, 602250, 182454, 600222, 600821, 180247, 602730, 134637, 167055, 165196, 165195, 173391, 601426, 600527, 600167, 153618, 601330, 601970, 600785, 600004, 601043, 109135,
   109636, 601613, 600804, 601529, 102581, 600936, 126150, 182451, 300107, 190151, 602746, 165070, 170650, 600421, 100997, 602648, 603243, 300035, 305670, 182455, 162651, 109535, 601320, 120920, 603411, 603691, 601535, 102775, 601166, 180381, 176804, 602188, 600051, 601993, 601773, 603755, 600241, 601050, 603659, 600253, 300130, 137026, 602368, 154570, 176945, 602005, 120980, 602583, 102777, 600524, 602848, 173393, 601711, 104221, 176884, 600441, 108361, 600665, 600446, 600799, 600742, 104219, 603819, 600867, 602351, 601978, 600578, 602566, 600551, 601898, 151443, 602642, 300182, 191164, 602356, 603071, 602190, 601522, 182889, 142703, 601576, 100735, 176882, 602969, 182453, 601895, 602886, 602927, 602697, 601070, 120830, 601108, 176891, 176802, 600024, 600552, 601908, 176981, 603510, 151460,
   602237, 602545, 162341, 603748, 161565, 600894, 186711, 137161, 601839, 601592, 603507, 602885, 430000, 104222, 601116, 602643, 601896, 601404, 601381, 600180, 600870, 123889, 600206, 600242, 602992, 601380, 602950, 602174, 603159, 602042, 602297, 602836, 602355, 602304, 603822, 147267, 603030, 603821, 602043, 603820, 603317, 601965, 601524, 601974, 603823, 602728, 601741, 137570, 603500, 602592, 153622, 601204, 186990, 601115, 600283, 182180, 600600, 600066, 600282, 176944, 600926, 600895, 300188, 602657, 602756, 600925, 182888, 603808, 603029, 602702, 602464, 603751, 425000, 601910, 151020, 600553, 603195, 602646, 601909, 603692, 136950, 600543, 164320, 300086, 602933, 602993, 137162, 603281, 153243, 161555, 600011, 600144, 600869, 600493, 602346, 600342, 602336, 113955, 602893, 601577,
   603749, 602892, 603810, 602853, 600579, 601044, 603809, 186690, 600752, 603304, 600240, 603232, 600868, 602894, 602337, 603143, 109545, 602891, 602890, 602716, 603142, 602510, 603144, 603141, 300090, 602550, 603145, 107741, 107730, 164761, 208900, 107269, 114208, 235200, 158120, 147440, 300200, 161561, 186830, 151520, 131240, 139250, 601667, 153240, 218030, 147680, 186740, 600044, 107940, 153390, 102582, 600978, 601218, 142409, 601156, 601884, 601789, 125950, 171833, 602221, 139320, 147760, 190000, 184757, 601512, 165180, 166490, 168470, 601366, 176000, 191160, 102578, 103320, 112260, 192240, 109091, 603233, 134370, 254210, 173610, 125480, 600957, 118945, 120470, 136351, 600725, 176797, 602887, 173511, 123910, 601459, 252500, 104615, 601528, 600345, 601890, 151390, 602170, 186790, 186960,
   600542, 186973, 600423, 600058, 176730, 186945, 104760, 601309, 601500, 300091, 113705, 214500, 602731, 600726, 176885, 164875, 139330, 104640, 147840, 600065, 131242, 164160, 600565, 601211, 151530, 603599, 180860, 190040, 147720, 222100, 223900, 600555, 600994, 186770, 120420, 602004, 601099, 603776, 601212, 132890, 600007, 158105, 603886, 603884, 176830, 162200, 602458, 601603, 107910, 193400, 133100, 160900, 114855, 601498, 601511, 116831, 131390, 107773, 137800, 155750, 147795, 264600, 114207, 244400, 187040, 126455, 164040, 147683, 263200, 188230, 184756, 147574, 179615, 176960, 176947, 147561, 131320, 107265, 147470, 264700, 173335, 124010, 147050, 132810, 230350, 276000, 114130, 155730, 134638, 254150, 122560, 186940, 208150, 188040, 188400, 100600, 601564, 603875, 603849, 601497,
   603254, 603180, 602952, 602775, 602584, 602570, 601045, 600632, 602559, 601883, 600618, 601848, 601766, 601753, 600554, 600937, 600694, 311360, 302910, 600173, 300139, 300065, 274000, 305100, 300116, 300022, 163800, 187410, 187020, 603818, 105850, 107941, 307800, 601897, 212895, 603598, 603584, 603583, 603454, 603412, 601872, 114204, 266300, 114841, 116897, 152690, 603261, 603130, 603109, 603061, 603055, 603043, 601771, 154275, 600910, 187011, 188060, 600039, 600464, 186355, 121360, 122559, 123000, 253800, 602901, 125255, 131100, 600837, 602772, 600835, 131530, 602695, 137580, 602623, 139130, 249210, 142408, 602457, 600179, 142445, 154276, 245480, 142800, 600998, 602354, 601698, 602272, 601053, 602265, 231550, 229300, 147780, 601985, 147796, 226200, 600276, 147830, 150200, 601937, 189906,
   601922, 600341, 100650, 601662, 189965, 159440, 190020, 601054, 600764, 600410, 153440, 205400, 601055, 203800, 600576, 600749, 190090, 601767, 600475, 190080, 154550, 184745, 601721, 601712, 600594, 600739, 600738, 180069, 601020, 601679, 162662, 601266, 601676, 601203, 172400, 601463, 600685, 601599, 164009, 600611, 601570, 600626, 600201, 600514, 191100, 195002, 600567, 600633, 191030, 190700, 600014, 303700, 308210, 308230, 191170, 269700, 191092, 192130, 600319, 238600, 195000, 600125, 600577, 261680, 600566, 266100, 309900, 266600, 306900, 600536, 194070, 201910, 188860, 188595, 257220, 600038, 202110, 261000, 600363, 600850, 306100, 602229, 603883, 102645, 186921, 186910, 603785, 186852, 104311, 107266, 107270, 603746, 603732, 109270, 602060, 602041, 148180, 151385, 151625, 123830,
   182530, 123842, 124030, 134660, 153370, 134850, 601023, 182280, 134797, 182125, 134390, 601047, 135820, 601804, 153420, 601801, 139312, 139392, 181031, 601622, 180700, 147570, 603157, 162150, 601589, 121013, 151440, 602919, 151510, 123810, 123890, 131210, 179730, 602663, 179020, 602630, 601231, 601252, 137295, 138190, 138550, 277700, 601295, 139311, 153455, 176311, 176310, 601313, 133170, 141800, 142995, 602358, 143100, 145505, 300079, 168461, 145981, 158070, 106700, 107400, 107720, 601485, 103950, 108330, 165360, 300300, 156490, 603492, 602018, 118485, 164951, 164730, 164720, 147620, 603406, 164343, 119530, 161560, 142910, 602414, 601933, 120290, 164013, 164012, 600667, 601534, 601513, 601520, 164015, 164340, 173515, 164975, 302020, 160782, 601501, 165230, 302060, 165120, 601492, 601483,
   167040, 167415, 600714, 192968, 600696, 601458, 601447, 173325, 168820, 176820, 601413, 190198, 600489, 173445, 601754, 600481, 300155, 600453, 154230, 173430, 154030, 173360, 181590, 176876, 176970, 178600, 164005, 182160, 274500, 179616, 275120, 151990, 300147, 601863, 211750, 275355, 151675, 190170, 179838, 600750, 180200, 180231, 151430, 600753, 601582, 150240, 601584, 601153, 224500, 601154, 601969, 180440, 600596, 600281, 600262, 227400, 601997, 147684, 147678, 600761, 147581, 602014, 602024, 147573, 601150, 159555, 147559, 600945, 191163, 602065, 147558, 601641, 147450, 602096, 602113, 600583, 602154, 600556, 159350, 147245, 186855, 192020, 191510, 146631, 146630, 180950, 601693, 146255, 602260, 238300, 600231, 602293, 194540, 602299, 145680, 602311, 145260, 600230, 602350, 239100,
   600508, 143055, 601723, 143010, 155960, 601377, 602382, 275350, 600220, 602396, 304050, 600424, 602421, 142461, 602430, 602431, 142460, 246530, 273300, 249100, 602461, 602465, 141850, 601788, 602498, 140210, 181460, 600422, 300127, 600164, 600765, 602582, 138800, 300101, 138720, 138420, 602609, 600157, 138079, 138030, 600156, 601017, 137240, 602671, 602680, 602682, 136530, 182090, 600807, 601826, 602724, 153245, 182331, 210250, 600151, 602738, 131340, 131241, 300008, 131195, 600119, 602809, 602839, 602840, 128240, 126850, 126065, 252650, 253200, 188380, 600810, 600396, 123841, 182810, 123835, 600053, 217030, 602921, 602931, 602932, 601900, 600050, 185430, 185520, 121800, 254770, 259700, 222300, 120940, 120700, 120436, 120360, 603033, 600295, 603035, 262600, 603046, 600009, 263400, 120150,
   263520, 603126, 590085, 185605, 118960, 118940, 118455, 603149, 603177, 300019, 603181, 118425, 603207, 426000, 116955, 116940, 116930, 300088, 603264, 603272, 603274, 116899, 603302, 313650, 266265, 603331, 114835, 603356, 114830, 312760, 603378, 602061, 310400, 603414, 603437, 113730, 310200, 113710, 147557, 113000, 112205, 111400, 111100, 110750, 603518, 603526, 602136, 603576, 309845, 603594, 308310, 108728, 108355, 600250, 307200, 603628, 107777, 602165, 603653, 600899, 107273, 107271, 603695, 603705, 603717, 186590, 186720, 186760, 186820, 187700, 600916, 147183, 105590, 104610, 146770, 306400, 602227, 103390, 103280, 103070, 102670, 301000, 603851, 600874, 600871, 102200, 158380, 603889
6) Glycoprotein
   438 entries found, searching for "glycoprotein"
   231200, 273800, 138700, 138600, 173510, 173515, 192974, 109770, 138610, 603578, 195002, 182889, 601275, 602977, 300051, 173470, 171050, 138680, 142640, 194460, 176390, 138720, 600119, 185430, 138670, 180297, 171060, 600073, 173335, 128239, 118850, 602616, 110700, 164345, 159465, 159460, 191845, 173511, 600564, 159440, 256550, 153310, 168810, 600738, 151460, 138470, 190197, 603586, 601325, 601193, 162820, 158120, 155970, 190920, 603201, 603594, 601411, 155735, 186730, 601028, 158030, 176391, 115501, 137290, 601103, 600315, 176870, 601525, 601418, 137061, 137207, 137060, 176397, 176393, 176394, 182888, 160995, 176398, 600718, 176396, 176392, 176399, 176401, 176395, 125645, 601774, 104175, 195000, 601785, 193400, 600065, 111200, 253700, 152200, 154550, 310200, 188230, 256540, 120980, 130660,
   107269, 158070, 156790, 131550, 135630, 138297, 155760, 231050, 603381, 143890, 176801, 118860, 103600, 193210, 186820, 114890, 188060, 118910, 107271, 153340, 313470, 600074, 603062, 109270, 602724, 603356, 120324, 192340, 266265, 110900, 306400, 601327, 118457, 151250, 136836, 253000, 104640, 190010, 190000, 135620, 135600, 601852, 134797, 134370, 111250, 156225, 600596, 187430, 164870, 151510, 186720, 601962, 600900, 151530, 180990, 180290, 182120, 603759, 179605, 158381, 158373, 158343, 155740, 153420, 151675, 142800, 176300, 153240, 140100, 134820, 134660, 134390, 125240, 120520, 112205, 111740, 106100, 104225, 103000, 139090, 176860, 102720, 176895, 180621, 602421, 602257, 252600, 252650, 180620, 227600, 228960, 208900, 182145, 208400, 602643, 602593, 602461, 602171, 182900, 601269,
   600270, 600033, 602894, 247100, 186910, 600239, 230500, 217800, 600867, 253220, 601581, 305600, 162060, 158378, 188450, 186880, 186990, 188040, 154582, 164022, 188540, 153634, 189965, 193190, 165095, 165220, 158375, 152790, 158374, 151627, 182205, 155750, 191316, 151523, 151020, 165330, 166490, 192225, 224050, 224100, 185490, 192240, 173445, 167770, 185050, 153619, 230800, 147740, 147730, 147670, 153618, 236200, 193300, 249000, 176385, 153440, 147557, 152310, 253270, 184900, 182331, 146760, 146661, 272300, 272800, 142930, 169615, 171640, 182520, 182310, 300027, 147780, 305370, 151525, 142858, 142840, 308230, 308385, 309060, 142445, 142290, 216950, 139130, 600026, 600044, 600046, 138971, 138960, 138140, 600133, 600182, 600215, 173350, 173360, 135820, 600403, 600410, 600491, 134934, 147563,
   600535, 133700, 600587, 133170, 131390, 600616, 600713, 131195, 600722, 130160, 600742, 600751, 600764, 600769, 600798, 180540, 600978, 129010, 601040, 601050, 125505, 125490, 124010, 601194, 601211, 601253, 147380, 120930, 601313, 120830, 120620, 601365, 601368, 120220, 601456, 601484, 117700, 147045, 601652, 274500, 116930, 116880, 601788, 116805, 114250, 113810, 601880, 601914, 601932, 111700, 602046, 602053, 602108, 602243, 176820, 178630, 178640, 111680, 300017, 109535, 142860, 602692, 109530, 602746, 308840, 107740, 107400, 603104, 603125, 603126, 603130, 107273, 603197, 107266, 603241, 603243, 104614, 312080, 104610, 603492, 103390, 102670, 102480, 100735, 603665, 603756, 100730, 603777
7) Protein kinase (a member of signal transduction)
   729 entries found, searching for "protein kinase"
   600289, 160900, 601032, 176948, 600899, 176947, 176894, 601939, 602980, 601314, 601955, 601335, 600447, 602130, 603606, 601158, 602006, 602549, 602678, 176872, 603014, 602520, 186945, 601254, 602315, 601263, 177061, 176873, 600497, 603607, 602449, 602740, 602898, 602741, 300083, 602899, 114080, 603412, 601184, 602739, 176910, 600050, 176949, 602741, 602743, 600341, 601951, 601212, 600038, 156490, 600855, 600758, 603455, 156491, 603424, 300075, 180381, 151510, 600168, 600085, 601890, 602896, 602123, 600221, 602904, 602687, 176945, 602897, 600222, 602098, 601685, 176944, 109635, 601795, 601679, 603722, 602521, 601436, 191164, 602004, 176942, 601684, 300101, 601299, 601434, 602615, 601435, 176960, 153390, 603166, 179090, 300300, 176871, 602216, 176912, 176893, 603453, 300185, 177060, 176943,
   176981, 603441, 176970, 116899, 603442, 311550, 603495, 169190, 600448, 602399, 176946, 176977, 188830, 602350, 601591, 601782, 137026, 400008, 300094, 600058, 600831, 176892, 601639, 602919, 176911, 176975, 300172, 603072, 600583, 177015, 176982, 176980, 603248, 176941, 602757, 600870, 603440, 148750, 601959, 600869, 602448, 600408, 306000, 266200, 602539, 602425, 600160, 310200, 208900, 600997, 186973, 305360, 179611, 307030, 603262, 601050, 602758, 147795, 311800, 600664, 600441, 603251, 600527, 313650, 123831, 191315, 602958, 123832, 602989, 602614, 123828, 136350, 601284, 602940, 601329, 600173, 171834, 124095, 603464, 603584, 116953, 300035, 600600, 109135, 601232, 601988, 603850, 603384, 603184, 602990, 125855, 601047, 603460, 603258, 602748, 603723, 164870, 603368, 139310, 601818,
   602913, 603005, 602221, 173570, 147670, 602610, 600524, 113508, 251170, 142370, 142408, 191306, 147796, 118423, 600286, 601074, 600765, 600922, 179610, 602747, 601953, 601132, 600136, 601288, 601289, 602297, 191316, 601535, 602925, 125450, 602839, 602168, 602461, 105590, 114085, 600714, 154235, 603261, 109270, 191305, 602625, 602366, 602749, 600927, 300134, 603496, 136352, 176790, 600073, 601523, 115441, 191170, 114210, 116900, 188250, 102576, 603764, 603497, 603304, 601934, 602255, 600004, 600526, 601589, 600856, 601269, 601465, 601207, 601935, 600863, 602394, 601983, 602048, 300200, 179050, 164761, 189980, 602626, 137025, 602694, 602731, 125305, 123810, 300139, 601792, 275350, 171833, 193525, 601540, 162200, 601826, 116898, 601108, 601528, 600954, 602609, 602838, 603167, 601522, 600505,
   600431, 600267, 188555, 600140, 308240, 600137, 600011, 151410, 601530, 134934, 116952, 300142, 191311, 601603, 603889, 601728, 165160, 165070, 136351, 601836, 601839, 133090, 601595, 602745, 602516, 154950, 603601, 602052, 154045, 603583, 600695, 602933, 602756, 601014, 602474, 602887, 172270, 601366, 180220, 602189, 114105, 600963, 603369, 601296, 603289, 602265, 602337, 600066, 600456, 602387, 600917, 139270, 603271, 188345, 601231, 115440, 115442, 600778, 164920, 175100, 310300, 601767, 603168, 603140, 603113, 116940, 162060, 603048, 603275, 601146, 600434, 602336, 602310, 602308, 147522, 603311, 227645, 114110, 601114, 300022, 164785, 167414, 123280, 600007, 600560, 229300, 151430, 176740, 602426, 109636, 602775, 108355, 601999, 601524, 190151, 202500, 601592, 600723, 102750, 600753,
   603618, 603814, 147521, 602514, 603015, 602525, 602524, 172471, 602647, 602590, 600864, 172470, 602538, 602527, 602526, 143890, 164730, 232300, 131550, 116951, 601713, 154550, 603434, 138160, 601702, 126335, 272800, 601497, 602038, 107940, 191190, 273800, 193300, 120150, 603068, 603259, 102582, 601099, 253700, 308000, 602338, 602689, 185605, 164040, 600119, 152690, 601496, 602188, 123900, 602860, 164960, 600618, 601045, 300189, 164860, 602505, 230800, 141850, 147545, 110300, 600051, 138600, 233700, 109700, 238600, 109690, 253800, 311770, 156225, 603719, 191030, 309000, 601893, 190030, 189972, 601247, 190120, 600179, 258501, 190040, 300121, 180380, 314850, 600438, 306400, 179715, 190450, 600636, 180901, 173335, 601290, 154500, 131195, 115460, 603078, 603775, 603760, 158900, 602354, 102680,
   122560, 602382, 103320, 603597, 603510, 232600, 133170, 603353, 603345, 603318, 603309, 230400, 139250, 603296, 603272, 114240, 147557, 232400, 139259, 602122, 230200, 164014, 248600, 601703, 194364, 150000, 602102, 601683, 158070, 602170, 602007, 601306, 248610, 601387, 161770, 132800, 164760, 601902, 601253, 173470, 602930, 603691, 165360, 123835, 603544, 147265, 107777, 602377, 600605, 602869, 601121, 602388, 301300, 590060, 601059, 168461, 142600, 314555, 142410, 170290, 602498, 190090, 311030, 142409, 190181, 600966, 308100, 600936, 171900, 190182, 172405, 102600, 603754, 600281, 172430, 138249, 300039, 603154, 602642, 300041, 602659, 192240, 603473, 136537, 603134, 182280, 603084, 125660, 189990, 133430, 603755, 603302, 600737, 300144, 305400, 188545, 600652, 189940, 600633, 603009,
   181031, 182160, 148500, 602384, 600650, 602190, 171150, 173490, 164765, 147780, 600910, 601272, 147880, 125255, 120090, 173390, 602668, 600483, 602995, 602996, 180860, 142445, 116949, 602681, 603027, 182125, 600327, 600799, 164757, 230450, 602730, 600782, 164880, 232700, 116935, 602303, 603203, 164710, 600244, 600206, 263200, 300034, 601487, 600980, 600956, 601985, 600884, 601956, 191041, 601568, 601586, 155960, 602288, 176761, 113900, 600581, 516050, 190070, 164940, 600544, 107941, 312861, 602373, 123836, 106165, 103850, 274270, 601922, 602355, 147420, 311010, 163800, 600536, 602018, 307200, 603619, 603652, 193001, 193245, 603729, 603752, 118888, 603796, 305600, 603852, 603853, 603886
8) Calmodulin kinase (a member of signal transduction)
   35 entries found, searching for "calmodulin binding"
   300172, 114180, 302020, 139312, 602584, 602293, 600310, 603379, 114106, 602350, 114105, 114213, 138249, 153430, 177061, 182520, 102680, 600489, 601478, 306000, 600490, 600922, 601302, 601568, 168890, 164795, 163730, 602698, 602699, 602987, 102681, 603384
9) Transcription factor
   717 entries found, searching for "transcription factor"
   305371, 189963, 164177, 600297, 600298, 163260, 189907, 173110, 600733, 189903, 189972, 600438, 600281, 189962, 157670, 602272, 600609, 189968, 189971, 189889, 107580, 189908, 601748, 601542, 601632, 600610, 189969, 600480, 601760, 176310, 603022, 600519, 601750, 123803, 600520, 600494, 164176, 602460, 603246, 113725, 107773, 189904, 602228, 147141, 132890, 189964, 164175, 142410, 313650, 164011, 164014, 164005, 601714, 600492, 164012, 601878, 600673, 600635, 191523, 602191, 600490, 602149, 189967, 140580, 164343, 603256, 600727, 600729, 600728, 140581, 600571, 601511, 102582, 189906, 600660, 124097, 314310, 600860, 600662, 184756, 600777, 600013, 600172, 600489, 601425, 600786, 600502, 602406, 600743, 189965, 603257, 602751, 602542, 603148, 603107, 603789, 602318, 156845, 123811, 600425,
   600540, 600695, 601622, 147574, 601601, 602407, 602150, 601538, 600663, 603306, 165170, 187040, 602444, 189902, 189973, 600659, 600661, 601010, 600788, 602617, 601602, 602053, 601742, 300039, 602438, 602976, 600744, 602543, 602479, 600481, 600473, 603739, 600426, 603738, 189901, 603677, 600427, 603255, 600607, 600379, 189909, 601679, 600787, 602160, 601043, 601397, 601044, 600366, 300025, 602575, 602669, 601804, 601801, 176312, 176311, 600746, 602480, 602944, 600967, 600912, 306700, 306900, 193400, 601206, 480000, 191160, 601861, 164008, 600475, 600773, 600772, 600774, 142409, 156490, 600589, 601490, 151385, 600599, 184757, 602955, 234000, 603433, 603349, 603198, 602294, 600390, 603628, 147620, 600211, 601787, 601863, 147470, 603795, 603734, 152760, 104155, 128990, 601729, 600197, 147370,
   173490, 603423, 600822, 188595, 603243, 600573, 601689, 142765, 603181, 600879, 603731, 600288, 602295, 121360, 164874, 300019, 162095, 602355, 603258, 126090, 159540, 300070, 600555, 600664, 601874, 153245, 191191, 601126, 601512, 146733, 131550, 142385, 601796, 603406, 602959, 601734, 601732, 139191, 139139, 600633, 138971, 600006, 603170, 601488, 147576, 147680, 601498, 602630, 602643, 603364, 600914, 154040, 602746, 128992, 143089, 160900, 600140, 134934, 133510, 176860, 190180, 601150, 601175, 170993, 601361, 122560, 602778, 308230, 602903, 309550, 601788, 602946, 159970, 124092, 180200, 173410, 602356, 603015, 600779, 603111, 187930, 602614, 600951, 603200, 602369, 164770, 147569, 603300, 603301, 159980, 134638, 603431, 147730, 603366, 603348, 600556, 602136, 164160, 310200, 152390,
   601241, 116897, 137295, 600576, 194070, 601487, 600698, 164810, 601769, 141900, 602225, 275350, 131100, 179755, 600075, 162200, 165160, 116806, 600899, 123810, 133450, 216400, 278700, 190080, 164730, 191170, 193300, 600618, 600999, 601090, 106150, 601843, 133530, 110700, 602550, 138040, 133430, 300133, 163731, 602302, 126337, 309548, 180245, 126110, 602291, 109565, 107400, 314670, 601444, 143100, 104760, 106180, 601953, 600584, 125852, 602419, 600401, 142200, 107680, 167414, 600020, 188400, 208900, 175100, 602700, 601828, 139320, 602777, 600185, 602681, 603023, 314997, 602848, 600284, 102578, 114290, 165095, 137070, 602991, 602421, 600005, 602996, 314995, 152200, 151900, 112260, 129010, 600892, 273800, 176760, 602341, 490000, 136533, 400003, 601007, 602229, 603620, 602218, 602116, 602020,
   142000, 601955, 126340, 120150, 193067, 182452, 142461, 194558, 180660, 600756, 160745, 107741, 106210, 157640, 186770, 146738, 603759, 213700, 147880, 152391, 277900, 151626, 107730, 600711, 600246, 107470, 600237, 223100, 107748, 600065, 600349, 426000, 194500, 154030, 227650, 600247, 314980, 109560, 305370, 600800, 301000, 277700, 600838, 312173, 600439, 600440, 191315, 601595, 190450, 190070, 190020, 162360, 131320, 133540, 600993, 601993, 159530, 601902, 602868, 181590, 601724, 602260, 601093, 187270, 164761, 602102, 603245, 136950, 106100, 601182, 167410, 601897, 602896, 170100, 602506, 104150, 176730, 601600, 187011, 102600, 180380, 162080, 603450, 142967, 602301, 126375, 603372, 603355, 164720, 603250, 167409, 167415, 602897, 601565, 185250, 182138, 601851, 600749, 601575, 194548,
   154500, 601365, 194541, 601621, 601623, 601531, 600790, 194355, 123830, 123812, 154540, 601415, 143055, 601386, 194550, 186930, 131290, 601320, 601620, 601754, 601313, 184430, 182900, 182500, 600725, 147870, 154365, 116953, 601297, 601296, 601265, 600796, 120436, 601644, 601930, 601643, 230200, 601645, 601972, 600861, 602009, 601172, 601158, 601646, 180630, 600821, 118440, 601656, 601647, 150200, 601125, 601671, 141850, 116899, 600697, 109270, 202110, 150570, 601108, 191339, 601063, 109691, 180240, 203100, 151430, 179710, 111000, 176797, 238600, 104311, 240300, 125255, 600423, 158070, 602439, 600324, 112261, 243305, 602474, 174762, 600613, 602539, 138890, 138720, 114550, 173865, 602582, 602584, 173510, 600250, 602627, 173325, 602635, 246530, 172425, 600193, 602691, 600188, 170998, 152790,
   168468, 256540, 225250, 600848, 143400, 168461, 262600, 168360, 601912, 602951, 600017, 230000, 266600, 602981, 272800, 109150, 102200, 603025, 603026, 603109, 167050, 603127, 603128, 165240, 230400, 313700, 164975, 164875, 602017, 115500, 235800, 164873, 602110, 164785, 164772, 312865, 603296, 600542, 164740, 602125, 309801, 602148, 300007, 306955, 603368, 116940, 602181, 603416, 126650, 163920, 300024, 603437, 602209, 603576, 603607, 305435, 600944, 180410, 303630, 159557, 301870, 132810, 100790, 603849, 603862, 603881,

There are several methods for analyzing the expression levels of genes associated with diseases. Differences in gene expression levels between diseased and normal tissues are studied by the analytical methods, for example, Northern hybridization and differential display. Other examples include a method with high-density cDNA filter, a method with DNA microarray and methods with PCR amplification (Experimental Medicine, Vol.17, No. 8, 980-1056 (1999); Cell Engineering (additional volume) DNA Microarray and Advanced PCR Methods, Muramatsu & Naba (eds.), Shujunsya). The levels of gene expression between diseased tissues and normal tissues can be studied by any of these analytical methods. When explicit difference in expression level is observed for a gene, it can be concluded that the gene is closely associated with a disease or disorder. Instead of diseased tissues, cultured cells can be used for the assessment. Similarly, when gene expression is explicitly different between normal cells and cells reproducing disease-associated specific features, it can be concluded that the gene is closely associated with a disease or disorder. When the expression levels of genes are evidently varied during major cellular events (such as differentiation and apoptosis), the genes are involved in the cellular events and accordingly are candidates for disease- and/or disorder-associated genes. Further, genes exhibiting tissue-specific expression are genes playing important parts in the tissue functions and, therefore, can be candidates for genes associated with diseases and/or disorders affecting the tissues.

For example, non-enzymic protein glycation reaction is believed to be a cause for a variety of chronic diabetic complications. Accordingly, in endothelial cells, genes, of which expression levels are elevated or decreased in a glycated protein-dependent manner, are associated with diabetic complications caused by glycated proteins (Diabetes 1996, 45 (Suppl. 3), S67-S72; Diabetes 1997, 46 (Suppl. 2), S19-S25). The onset of rheumatoid arthritis is thought to be involved in the proliferation of synovial cells covering inner surfaces of joint cavity and in inflammatory reaction resulted from the action of cytokines produced by leukocytes infiltrating into the joint synovial tissues (Rheumatism Information Center, http://www.rheuma-net.or.jp/). Recent studies have also revealed that tissue necrosis factor (TNF)-α participates in the onset (Current opinion in immunology 1959, 11, 657-662). When the expression of a gene exhibits responsiveness to the action of TNF on synovial cells, the gene is considered to be involved in rheumatoid arthritis. Many genes acting at the downstream of TNF-. and IL-1. among inflammation-associated cytokines have been previously identified. The respective stimulations are transduced through independent pathways of signaling cascade. There exists another signaling cascade for both stimulations, wherein NF-.B is a common transducing molecule shared by the two stimulations (J. Leukoc. Biol., 1994, 56(5): 542-547). It has also been revealed that many inflammation-associated genes, including IL-2, IL-6 and G-CSF, are varied in the expression levels in response to the signal through the common pathway (Trends Genet. 1999, 15(6): 229-235). It is assumed that genes of which expression levels are varied in response to the stimulation of TNF-. or IL-1. also participate in inflammation. Genes associated with neural differentiation can be candidates for causative genes for neurological diseases as well as candidates for genes usable for treating the diseases.

Clones exhibiting differences in the expression levels thereof can be selected by using gene expression analysis. The selection comprises, for example; analyzing cDNA clones by using high-density cDNA filter; and statistically treating the multiple signal values (signal values of radioisotope in the radiolabeled probes or values obtained by measuring fluorescence intensities emitted from the fluorescent labels) for the respective clones by two-sample t-test, where the signal values are determined by multiple experiments of hybridization. The clones of interest are selectable based on the statistically significant differences in the signal distribution at p<0.05. However, selectable clones with significant difference in the expression levels thereof may be changed depending on the partial modification of statistical treatment. For example, the clones may be selected by conducting statistical treatment with two-sample t-test at p<0.01; or genes exhibiting more explicit differences in the expression levels thereof can be selected by performing statistical treatment with a pre-determined cut-off value for the significant signal difference. An alternative method is that the expression levels are simply compared with each other, and then, the clones of interest are selected based on the ratio of the expression levels thereof.

Clones exhibiting differences in the expression levels can also be selected by comparing the expression levels by PCR analysis, for example, by using the method of determining the band intensities representing the amounts of PCR products with ethidium bromide staining; the method of determining the radioisotopic signal values or fluorescence intensities of the PCR products when radio-labeled or fluorescence-labeled primers; or the method of determining the values of radioisotope signals or fluorescence intensities of the probes hybridized to the PCR products when radio-labeled or fluorescence-labeled probes, respectively, are used in the hybridization. If the expression level ratios obtained in multiple PCR experiments are constantly at least 2-fold, such a clone can be judged to exhibit the difference in the expression level. When the ratios are several-fold or not less than 10-fold, the clone can be selected as a gene exhibiting the explicit difference in the expression level.

A survey of genes of which expression levels are varied specifically to the glycated protein in the endothelial cells revealed three genes with elevated expression levels, NT2RP2001538, NT2RP4001001 and Y79AA1000967. These clones are genes associated with diabetes.

A survey of genes of which expression levels are varied in response to TNF. (Tumor Necrosis Factor-alpha) in the primary cell culture of synovial tissue detected the following clones with elevated expression levels in the presence of TNF.:
BNGH41000020, HEMBA1000349, HEMBA1000634, HEMBA1000671, HEMBA1000835, HEMBA1000962, HEMBA1002178, HEMBA1002195, HEMBA1002239, HEMBA1002420, HEMBA1002524, HEMBA1002992, HEMBA1003315, HEMBA1003392, HEMBA1003487, HEMBA1003602, HEMBA1004067, HEMBA1004797, HEMBA1005337, HEMBA1005489, HEM3A1006916, HEMBB1000668, HEMBB1000905, HEMBB1001547, HEMBB1001573, HEMBB1002041, HEMBB1002663, MAMMA1000652, MAMMA1000810, MAMMA1001634, MAMMA1002091, MAMMA1002234, NT2RM2000306, NT2RM4000417, NT2RP1000002, NT2RP1000181, NT2RP1000740, NT2RP2000694, NT2RP2001921, NT2RP2002527, NT2RP2004495, NT2RP2004606, NT2RP2005163, NT2RP2005463, NT2RP2006134, NT2RP3000171, NT2RP3000652, NT2RP3001195, NT2RP3001976, NT2RP3003473, NT2RP3003874, NT2RP3004090, NT2RP3004294, NT2RP3004557, NT2RP3004647, NT2RP4000108, NT2RP4001001, NT2RP4001877, OVARC1000090, OVARC1000105, OVARC1000275, OVARC1000439, OVARC1001607, PLACE1000740, PLACE1000927, PLACE1001016, PLACE1001100, PLACE1001464, PLACE1001500, PLACE1001918, PLACE1002095, PLACE1002547, PLACE1003644, PLACE1004519, PLACE1005031, PLACE1005410, PLACE1005736, PLACE1006219, PLACE1006809, PLACE1008716, PLACE1010081, THYRO1001770, Y79AA1000127, Y79M1000207, Y79AA1000270, Y79AA1000876, Y794A1001013, Y79AA1001264, Y79AA1001272, Y79AA1001328, Y79AA1001430, Y79AA1001530, Y79AA1001799

Clones with decreased expression levels in the presence of TNF□ are NT2RM4000326, NT2RP1000300, NT2RP2000514, NT2RP2001755, NT2RP2006042, NT2RP3000481, NT2RP3002790. These clones are candidates for rheumatoid arthritis-associated genes.

A survey of genes of which expression levels are varied in response to TNF. (Tumor Necrosis Factor-alpha) or IL-1. (Interleukin-1 beta) in a human T cell strain, Jurkat cell, revealed the following clones with elevated expression levels in the presence of TNF.:
MAMMA1000141, MAMMA1000788, MAMMA1001237, MAMMA1002070,NT2RM2000582, NT2RM2002109, NT2RP1000679, NT2RP2003664, NT2RP2004108, NT2RP2005597, NT2RP3001592, NT2RP3002738, NT2RP3004133, NT2RP3004294, NT2RP3004321, NT2RP3004557, PLACE1002547, PLACE1003573, PLACE1004305, PLACE1008744, PLACE1010011, PLACE1010713, PLACE1011181, Y79AA1000776, Y79AA1002129

The survey also revealed a clone of which expression level was decreased in the presence of TNF. The clone is PLACE1002070. The same survey further revealed the clones of which expression levels were elevated in the presence of IL-1.. The clones are MAMMA1000614, MAMMA1001237, NT2RM2000514 and NT2RP3001159. These clones are genes associated with inflammation.

A survey of genes of which expression levels are varied in response to the stimulation for inducing cell differentiation (stimulation using retinoic acid (RA) or using RA/inhibitor (inhibitor for cell division)) in cultured cells of neural strain, NT2, revealed the following clones with elevated expression levels in the presence of RA:
HEMBA1000121, HEMBA1000275, HEMBA1000300, HEMBA1000634, HEMBA1000671, HEMBA1000875, HEMBA1001184, HEMBA1001390, HEMBA1001886, HEMBA1002163, HEMBA1002227, HEMBA1002420, HEMBA1002421, HEMBA1003072, HEMBA1003120, HEMBA1003294, HEMBA1003497, HEMBA1004007, HEMBA1004110, HEMBA1004391, HEMBA1004444, HEMBA1005230, HEMBA1005246, HEMBA1005267, HEMBA1005489, HEMBA1005913, HEMBA1006299, HEMBA1006357, HEMBA1006517, HEMBA1006544, HEMBA1006658, HEMBA1006749, HEMBA1007063, HEMBA1007241, HEMBB1000447, HEMBB1000542, HEMBB1000567, HEMBB1000642, HEMBB1000668, HEMBB1001026, HEMBB1001847, HEMBB1002051, HEMBB1002120, HEMBB1002228, HEMBB1002693, MAMMA1000106, MAMMA1000141, MAMMA1000473, MAMMA1000528, MAMMA1000810, MAMMA1000881, MAMMA1001634, MAMMA1001957, MAMMA1002205, MAMMA1002224, NT2RM2000423, NT2RM2000497, NT2RM2000582, NT2RM2001126, NT2RM2001902, NT2RM4000198, NT2RM4000284, NT2RM4000593, NT2RM4001321, NT2RP1000002, NT2RP1000050, NT2RP1000181, NT2RP1000261, NT2RP1000465, NT2RP1000468, NT2RP1000579, NT2RP1000679, NT2RP2000092, NT2RP2000479, NT2RP2000610, NT2RP2000663, NT2RP2000694, NT2RP2000903, NT2RP2001388, NT2RP2001538, NT2RP2001878, NT2RP2002015, NT2RP2002304, NT2RP2002721, NT2RP2002824, NT2RP2002942, NT2RP2002974, NT2RP2002976, NT2RP2003179, NT2RP2003302, NT2RP2003383, NT2RP2003469, NT2RP2003664, NT2RP2003940, NT2RP2004069, NT2RP2004108, NT2RP2004524, NT2RP2004556, NT2RP2004670, NT2RP2005069,
NT2RP2005247, NT2RP2005425, NT2RP2005463, NT2RP2005514, NT2RP2005535, NT2RP2005541, NT2RP2005774, NT2RP2005878, NT2RP2005883, NT2RP2005887, NT2RP2006099, NT2RP2006134, NT2RP3000011, NT2RP3000125, NT2RP3000171, NT2RP3000232, NT2RP3000460, NT2RP3000481, NT2RP3000652, NT2RP3000677, NT2RP3000818, NT2RP3000820, NT2RP3001044, NT2RP3001061, NT2RP3001170, NT2RP3001240, NT2RP3001322, NT2RP3001388, NT2RP3001542, NT2RP3001592, NT2RP3001976, NT2RP3002790, NT2RP3002900, NT2RP3002983, NT2RP3003000, NT2RP3003354, NT2RP3003532, NT2RP3003729, NT2RP3003874, NT2RP3003939, NT2RP3004025, NT2RP3004083, NT2RP3004090, NT2RP3004130, NT2RP3004202, NT2RP3004294, NT2RP3004640, NT2RP4000108, NT2RP4000634, NT2RP4002451, NT2RP4002715, OVARC1000090, OVARC1000208, OVARC1000275, OVARC1000553, OVARC1000775, OVARC1000853, OVARC1000873, OVARC1000916, OVARC1000995, OVARC1001030, OVARC1001049, OVARC1001132, OVARC1001596, OVARC1002178, PLACE1000258, PLACE1000442, PLACE1000927, PLACE1000986, PLACE1001100, PLACE1001123, PLACE1001795, PLACE1002518, PLACE1002547, PLACE1002967, PLACE1003407, PLACE1003428, PLACE1003644, PLACE1003839, PLACE1004078, PLACE1004441, PLACE1004450, PLACE1005669, PLACE1005682, PLACE1005736, PLACE1005768, PLACE1005815, PLACE1006073, PLACE1006208, PLACE1007296, PLACE1007626, PLACE1008282, PLACE1008984, PLACE1008985, PLACE1010445, PLACE1011708, PLACE1011978, PLACE4000455, SKNMC1000004, THYRO1000036,
THYRO1000580, THYRO1000776, THYRO1000999, THYRO1001063, THYRO1001128, THYRO1001205, THYRO1001327, THYRO1001523, THYRO1001725, THYRO1001770, Y79AA1000207, Y79AA1000226, Y79AA1000270, Y79AA1001056, Y79AA1001062, Y79AA1001090, Y79AA1001727, Y79AA1002213, Y79AA1002381

The survey also revealed the clones of which expression levels were decreased in the presence of RA. The clones are BNGH41000020, HEMBA1005070, NT2RP2005027, NT2RP3003473 and Y79AA1002376.

The same survey further revealed the following clones with elevated expression levels in the presence of RA/inhibitor:
HEMBA1000128, HEMBA1000875, HEMBA1001390, HEMBA1002163, HEMBA1002227, HEMBA1002421, HEMBA1004391, HEMBA1004454, HEMBA1004785, HEMBA1005913, HEMBA1006171, HEMBA1006299, HEMBA1006335, HEMBA1006544, HEMBA1007241, HEMBB1000447, HEMBB1000668, MAMMA1000994, MAMMA1001344, NT2RM2000582, NT2RP1001004, NT2RP2000663, NT2RP2000694, NT2RP2000903, NT2RP2001388, NT2RP2002674, NT2RP2002974, NT2RP2003383, NT2RP2004069, NT2RP2004606, NT2RP2004837, NT2RP2005069, NT2RP2005425, NT2RP2005463, NT2RP2005541, NT2RP2005883, NT2RP2005887, NT2RP3000460, NT2RP3000838, NT2RP3001044, NT2RP3001240, NT2RP3001388, NT2RP3002721, NT2RP3002738, NT2RP3003469, NT2RP3004083, NT2RP3004130, NT2RP3004202, NT2RP3004294, NT2RP3004640, NT2RP4000108, NT2RP4002451, NT2RP4002715, OVARC1000275, OVARC1000467, OVARC1000553, OVARC1000853, OVARC1000873, OVARC1000916, OVARC1000995, OVARC1001030, OVARC1001222, OVARC1001596, OVARC1002058, OVARC1002178, PLACE1000927, PLACE1001123, PLACE1001407, PLACE1001464, PLACE1001564, PLACE1001795, PLACE1002547, PLACE1003407, PLACE1003644, PLACE1003845, PLACE1004441, PLACE1004482, PLACE1005410, PLACE1005601, PLACE1005725, PLACE1005736, PLACE1006093, PLACE1006219, PLACE1006290, PLACE1006716, PLACE1007296, PLACE1007626, PLACE1008359, PLACE1010968, PLACE1011364, PLACE1011824, THYRO1000678, THYRO1000776, THYRO1000999, THYRO1001113, THYRO1001237, THYRO1001523, Y79AA1000226, Y79AA1000888, Y79AA1001430

The same survey further revealed the following clones with elevated expression levels in the presence of RA/inhibitor:
HEMBA1000349, HEMBA1001297, HEMBA1001878, HEMBA1005070, HEMBA1006482, HEMBB1001959, NT2RM2001939, NT2RP1000981, NT2RP2001469, NT2RP3003473, OVARC1001132, PLACE1001655, Y79AA1000127, Y79AA1002381. These clones are associated with neural differentiation and, therefore, are candidates for genes associated with neurological diseases.

Based on the functional analyses using a secretory protein, membrane protein, or proteins associated with signal transduction, glycoprotein, transcription, or diseases, it is possible to develop a medicine.

In case of a membrane protein, it is most likely to be a protein that functions as a receptor or ligand on the cell surface. Therefore, it is possible to reveal a new relationship between a ligand and receptor by screening the membrane protein of the invention based on the binding activity with the known ligand or receptor. Screening can be performed according to the known methods.

For example, a ligand against the protein of the invention can be screened in the following manner. Namely, a ligand that binds to a specific protein can be screened by a method comprising the steps of: (a) contacting a test sample with the protein of the invention or a partial peptide thereof, or cells expressing these, and (b) selecting a test sample that binds to said protein, said partial peptide, or said cells.

On the other hand, for example, screening using cells expressing the protein of the present invention that is a receptor protein can also be performed as follows. It is possible to screen receptors that is capable of binding to a specific protein by using procedures (a) attaching the sample cells to the protein of the invention or its partial peptide, and (b) selecting cells that can bind to the said protein or its partial peptide.

In a following screening as an example, first the protein of the invention is expressed, and the recombinant protein is purified. Next, the purified protein is labeled, binding assay is performed using a various cell lines or primary cultured cells, and cells that are expressing a receptor are selected (Growth and differentiation factors and their receptors, Shin-Seikagaku Jikken Kouza Vol.7 (1991) Honjyo, Arai, Taniguchi, and Muramatsu edit, p203-236, Tokyo-Kagaku-Doujin). A protein of the invention can be labeled with RI such as ¹²⁵I, and enzyme (alkaline phosphatase etc.). Alternatively, a protein of the invention may be used without labeling and then detected by using a labeled antibody against the protein. The cells that are selected by the above screening methods, which express a receptor of the protein of the invention, can be used for the further screening of an agonists or antagonists of the said receptor.

Once the ligand binding to the protein of the invention, the receptor of the protein of the invention or the cells expressing the receptor are obtained by screening, it is possible to screen a compound that binds to the ligand and receptor. Also it is possible to screen a compound that can inhibit both bindings (agonists or antagonists of the receptor, for example) by utilizing the binding activities.

When the protein of the invention is a receptor, the screening method comprises the steps of (a) contacting the protein of the invention or cells expressing the protein of the invention with the ligand, in the presence of a test sample, (b) detecting the binding activity between said protein or cells expressing said protein and the ligand, and (c) selecting a compound that reduces said binding activity when compared to the activity in the absence of the test sample. Furthermore, when the protein of the invention is a ligand, the screening method comprises the steps of (a) contacting the protein of the invention with its receptor or cells expressing the receptor in the presence of samples, (b) detecting the binding activity between the protein and its receptor or the cells expressing the receptor, and (c) selecting a compound that can potentially reduce the binding activity compared to the activity in the absence of the sample.

Samples to screen include cell extracts, expressed products from a gene library, synthesized low molecular compound, synthesized peptide, and natural compounds, for example, but are not construed to be listed here. A compound that is isolated by the above screening using a binding activity of the protein of the invention can also be used as a sample.

A compound isolated by the screening may be a candidate to be an agonist or an antagonist of the receptor of the protein. By utilizing an assay that monitors a change in the intracellular signaling such as phosphorylation which results from reduction of the binding between the protein and its receptor, it is possible to identify whether the obtained compound is an agonist or antagonist of the receptor. Also, the compound may be a candidate of a molecule that can inhibit the interaction between the protein and its associated proteins (including a receptor) in vivo. Such compounds can be used for developing drugs for precaution or cures of a disease with which the protein is associated.

Secretory proteins may regulate cellular conditions such as growth and differentiation. It is possible to find out a novel factor that regulates cellular conditions by adding the secretory protein of the invention to a certain kind of cell, and performing a screening by utilizing the cellular changes in growth or differentiation, or activation of a particular gene.

The screening can be performed, for example, as follows. First, the protein of the invention is expressed and purified in a recombinant form. Then, the purified protein is microinjectedadded into a various kind of cell lines or primary cultured cells, and the change in the cell growth and differentiation is monitored. The induction of a particular gene that is known to be involved in a certain cellular change is detected with the amounts of mRNA and protein. Alternatively, the amount of an intracellular molecule (low molecular compounds, etc.) that is changed by the function of a gene product (protein) that is known to be functioning in a certain cellular change is used for the detection.

Once the screening reveals that the protein of the invention can regulate cellular conditions or the functions, it is possible to apply the protein as a pharmaceutical and diagnostic medicine for associated diseases by itself or by altering a part of it into an appropriate composition.

As is above described for membrane proteins, the secretory protein provided by the invention may be used to explore a novel ligand-receptor interaction using a screening based on the binding activity to a known ligand or receptor. A similar method can be used to identify an agonist or antagonist. The resulting compounds obtained by the methods can be a candidate of a compound that can inhibit the interaction between the protein of the invention and an interacting molecule (including a receptor). The compounds may be able to use as a preventive, therapeutic, and diagnostic medicine for the diseases, in which the protein may play a certain role.

Proteins associated with signal transduction or transcription may be a factor that affects a certain protein or gene in response to intracellular/extracellular stimuli. It is possible to find out a novel factor that can affect a protein or gene by expressing the protein provided by the invention in a certain types of cells, and performing a screening utilizing the activation of a certain intracellular protein or gene.

The screening may be performed as follows. First, a transformed cell expressing the protein is obtained. Then, the transformed cell line and the untransformed original cell are compared for the changes in the expression of a certain gene by detecting the amount of its mRNA or protein. Alternatively, the amount of an intracellular molecule (low molecular compounds), which is changed by the function of a gene product (protein) that is known to function in a certain cellular change, may be used for the detection. Furthermore, the change of the expression of a certain gene can be detected by introducing a fusion gene that comprises a regulatory region of the gene and a marker gene (luciferase, beta-galactosidase, etc.) into a cell, expressing the protein provided by the invention into the cell, and estimating the activity of a marker gene product (protein).

If the protein or gene of the invention is associated with diseases, it is possible to screen a gene or compound that can regulate its expression and/or activity either directly or indirectly by utilizing the protein of the present invention.

For example, the protein of the invention is expressed and the recombinant protein is purified. Then, the protein and gene whose expression was affected in the presence of the protein of the invention is also purified, and the binding activity between the two proteins or genes is examined. The examination may be performed with pretreatment with a compound that is candidate of an inhibitor. In an alternative method, a transcription regulatory region locating in the 5'-upstream of the gene encoding the protein of the invention that is capable of regulating the expression of other genes is obtained, and fused with a marker gene. The fusion is introduced into a cell, and the cell is added with compounds to explore a regulatory factor of the expression of the said gene.

The compound obtained by the screening can be used for developing pharmaceutical and diagnostic medicines for the diseases with which the protein of the present invention is associated. Similarly, if the regulatory factor obtained by the screening is a protein, the protein itself can be used as a pharmaceutical, and if there is a compound that affects the original expression level and/or activity of the protein, it also can be used for the same purpose.

If the protein of the invention has an enzymatic activity, regardless of whether it is a secretory protein, membrane protein, or proteins associated with signal transduction, glycoprotein, transcription, or diseases, a screening may be performed by adding a compound to the protein of the invention under the suitable condition and monitoring the change of the compound. The enzymatic activity may also be utilized to screen for a compound that can inhibit the activity of the protein.

In a screening given as an example, the protein of the invention is expressed and the recombinant protein is purified. Then, compounds are contacted with the purified protein, and the amount of the compound and the reaction products is examined. Alternatively, compounds that are candidates of an inhibitor are pretreated, then a compound (substrate) that can react with the purified protein is added, and the amount of the substrate and the reaction products is examined.

The compounds obtained in the screening may be used as a medicine for diseases with which the protein of the invention is associated. Also they can be applied for tests that examine whether the protein of the invention functions normally *in vivo.*

Whether the secretory or membrane protein of the present invention is a novel protein associated with diseases or not is determined in another method than described above, by obtaining a specific antibody against the protein of the invention, and examining the relationship between the expression or activity of the protein and a certain disease. In an alternative way, it may be analyzed referred to the methods in "Molecular Diagnosis of Genetic Diseases" (Elles R. edit, (1996) in the series of "Method in Molecular Biology" (Humana Press).

Disease-associated proteins are a target of the above described screenings and very useful for developing a drug that is capable of regulating the expression and activity of the protein. Also, they are useful in medicinal industry as a diagnostic marker of the related disease and as a target for gene therapy.

Compounds isolated as mentioned above can be administered patients as it is, or after formulated into a pharmaceutical composition according to the known methods. For example, a pharmaceutically acceptable carrier or vehicle, specifically sterilized water, saline, plant oil, emulsifier, or suspending agent can be mixed with the compounds appropriately. The pharmaceutical compositions can be administered to patients by a method known to those skilled in the art, such as intraarterial intravenous, or subcutaneous injections. The dosage may vary depending on the weight or age of a patient, or the method of administration, but those skilled in the art can choose an appropriate dosage properly. If the compound is encoded by DNA, the DNA can be cloned into a vector for gene therapy, and used for gene therapy. The dosage of the DNA and the method of its administration may vary depending on the weight or age of a patient, or the symptoms, but those skilled in the art can choose properly.

The protein encoded by the polynucleotide of the invention can be prepared as a recombinant protein or as a natural protein. For example, the recombinant protein can be prepared by inserting the polynucleotide encoding the protein of the invention into a vector, introducing the vector into an appropriate host cell and purifying the protein expressed within the transformed host cell, as described below. In contrast, the natural protein can be prepared, for example, by utilizing an affinity column to which an antibody against the protein of the invention (Current Protocols in Molecular Biology (1987) Ausubel et al. edit, John Wily & Sons, Section 16.1-16.19) is attached. The antibody used for the affinity purification may be either a polyclonal antibody, or a monoclonal antibody. Alternatively, in vitro translation (See, for example, "On the fidelity of mRNA translation in the nuclease-treated rabbit reticulocyte lysate system." Dasso M.C., and Jackson R.J. (1989) Nucleic Acids Res. 17: 3129-3144) may be used for preparing the protein of the invention.

Proteins functionally equivalent to the proteins of the present invention can be prepared based on the activities, which were clarified in the above-mentioned manner, of the proteins of the present invention. Using the biological activity possessed by the protein of the invention as an index, it is possible to verify whether or not a particular protein is functionally equivalent to the protein of the invention by examining whether or not the protein has said activity.

Proteins functionally equivalent to the proteins of the present invention can be prepared by those skilled in the art, for example, by using a method for introducing mutations into an amino acid sequence of a protein (for example, site-directed mutagenesis (Current Protocols in Molecular Biology, edit, Ausubel et al., (1987) John Wiley & Sons, Section 8.1-8.5). Besides, such proteins can be generated by spontaneous mutations. The present invention comprises the proteins having one or more amino acid substitutions, deletions, insertions and/or additions in the amino acid sequences of the proteins of the present invention (Table 370), as far as the proteins have the equivalent functions to those of the proteins identified in the present Examples described later.

There are no limitations in the number and sites of amino acid mutations, as far as the proteins maintain the functions thereof. The number of mutations is typically 30% or less, or 20% or less, or 10% or less, preferably within 5% or less, or 3% or less of the total amino acids, more preferably within 2% or less or 1% or less of the total amino acids. From the viewpoint of maintaining the protein function, it is preferable that a substituted amino has a similar property to that of the original amino acid. For example, Ala, Val, Leu, Ile, Pro, Met, Phe and Trp are assumed to have similar properties to one another because they are all classified into a group of non-polar amino acids. Similarly, substitution can be performed among non-charged amino acid such as Gly, Ser, Thr, Cys, Tyr, Asn, and Gln, acidic amino acids such as Asp and Glu, and basic amino acids such as Lys, Arg, and His.

In addition, proteins functionally equivalent to the proteins of the present invention can be isolated by using techniques of hybridization or gene amplification known to those skilled in the art. Specifically, using the hybridization technique (Current Protocols in Molecular Biology, edit, Ausubel et al., (1987) John Wiley & Sons, Section 6.3-6.4)), those skilled in the art can usually isolate a DNA highly homologous to the DNA encoding the protein identified in the present Example based on the identified nucleotide sequence (Table 370) or a portion thereof and obtain the functionally equivalent protein from the isolated DNA. The present invention includes proteins encoded by the DNAs hybridizing with the DNAs encoding the proteins identified in the present Example, as far as the proteins are functionally equivalent to the proteins identified in the present Example. Organisms from which the functionally equivalent proteins are isolated are illustrated by vertebrates such as human, mouse, rat, rabbit, pig and bovine, but are not limited to these animals.

Washing conditions of hybridization for the isolation of DNAs encoding the functionally equivalent proteins are usually "1xSSC, 0.1% SDS, 37"; more stringent conditions are "0.5xSSC, 0.1% SDS, 42."; and still more stringent conditions are "0.1 x SSC, 0.1% SDS, 65.". Alternatively, the following conditions can be given as hybridization conditions of the present invention. Namely, conditions in which the hybridization is done at "6xSSC, 40% Formamide, 25.", and the washing at "1xSSC, 55." can be given. More preferable conditions are those in which the hybridization is done at "6xSSC, 40% Formamide, 37.", and the washing at "0.2xSSC, 55,". Even more preferable are those in which the hybridization is done at "6xSSC, 50% Formamide, 37.", and the washing at "0.1xSSC, 62.". The more stringent the conditions of hybridization are, the more frequently the DNAs highly homologous to the probe sequence are isolated. Therefore, it is preferable to conduct hybridization under stringent conditions. Examples of stringent conditions in the present invention are, washing conditions of "0.5xSSC, 0.1% SDS, 42.", or alternatively, hybridization conditions of "6xSSC, 40% Formamide, 37.", and the washing at "0.2xSSC, 55.". However, the above-mentioned combinations of SSC, SDS and temperature conditions are indicated just as examples. Those skilled in the art can select the hybridization conditions with similar stringency to those mentioned above by properly combining the above-mentioned or other factors (for example, probe concentration, probe length and duration of hybridization reaction) that determines the stringency of hybridization.

The amino acid sequences of proteins isolated by using the hybridization techniques usually exhibit high homology to those of the proteins of the present invention, which are shown in Table 370. The present invention encompasses a polynucleotide comprising a nucleotide sequence that has a high identity to the nucleotide sequence of claim 8 (a). Furthermore, the present invention encompasses a peptide, or protein comprising an amino acid sequence that has a high identity to the amino acid sequence encoded by the polynucleotide of claim 8 (b). The term "high identity" indicates sequence identity of at least 40% or more; preferably 60% or more; and more preferably 70% or more. Alternatively, more preferable is identity of 90% or more, or 93% or more, or 95% or more, furthermore, 97% or more, or 99% or more. The identity can be determined by using the BLAST search algorithm.

With the gene amplification technique (PCR) (Current Protocols in Molecular Biology, edit, Ausubel et al., (1987) John Wiley & Sons, Section 6.3-6.4)) using primers designed based on the nucleotide sequence (Table 370) or a portion thereof identified in the present Example, it is possible to isolate a DNA fragment highly homologous to the nucleotide sequence or a portion thereof and to obtain functionally equivalent protein to a particular protein identified in the present Example based on the isolated DNA fragment.

The "percent identity" of two amino acid sequences or of two nucleic acids is determined using the algorithm of Karlin and Altschul (Proc. Natl. Acad. Sei. USA 87:2264-2268, 1990), modified as in Karlin and Altschul (Proc. Natl. Acad. Sei. USA 90:5873-5877, 1993). Such an algorithm is incorporated into the BLASTN and BLASTX programs of Altschul et al. (J. Mol. Biol.215:403-410, 1990). BLAST nucleotide searches are performed with the BLASTN program, score = 100, wordlength = 12. BLAST protein searches are performed with the BLASTX program, score = 50, wordlength = 3. When gaps exist between two sequences, Gapped BLAST is utilized as described in Altschul et al. (Nucleic Acids Res.25:3389-3402,1997). When utilizing BLAST and Gapped BLAST programs, the default parameters of the respective programs (e.g., BLASTX and BLASTN) are used. See http://www.ncbi.nlm.nih.gov.

The present invention also includes a partial peptide of the proteins of the invention. The partial peptide comprises a protein generated as a result that a signal peptide has been removed from a secretory protein. If the protein of the present invention has an activity as a receptor or a ligand, the partial peptide may function as a competitive inhibitor of the protein and may bind to the receptor (or ligand). In addition, the present invention comprises an antigen peptide for raising antibodies. For the peptides to be specific for the protein of the invention, the peptides comprise at least 7 amino acids, preferably 8 amino acids or more, more preferably 9 amino acids or more, and even more preferably 10 amino acids or more. The peptide can be used for preparing antibodies against the protein of the invention, or competitive inhibitors of them, and also screening for a receptor that binds to the protein of the invention. The partial peptides of the invention can be produced, for example, by genetic engineering methods, known methods for synthesizing peptides, or digesting the protein of the invention with an appropriate peptidase.

The present invention also relates to a vector into which the DNA of the invention is inserted. The vector of the invention is not limited as long as it contains the inserted DNA stably. For example, if E. coli is used as a host, vectors such as pBluescript vector (Stratagene) are preferable as a cloning vector. To produce the protein of the invention, expression vectors are especially useful. Any expression vector can be used as far as it is capable of expressing the protein in vitro, in E. coli, in cultured cells, or in vivo. For example, pBEST vector (Promega) is preferable for in vitro expression, pET vector (Invitrogen) for E. coli, pME18S-FL3 vector (GenBank Accession No. AB009864) for cultured cells, and pME18S vector (Mol. Cell. Biol. (1988) 8: 466-472) for in vivo expression. To insert the DNA of the invention, ligation utilizing restriction sites can be performed according to the standard method (Current Protocols in Molecular Biology (1987) Ausubel et al. edit, John Wily & Sons, Section 11.4-11.11).

The present invention also relates to a transformant carrying the vector of the invention. Any cell can be used as a host into which the vector of the invention is inserted, and various kinds of host cells can be used depending on the purposes. For strong expression of the protein in eukaryotic cells, COS cells or CHO cells can be used, for example.

Introduction of the vector into host cells can be performed, for example, by calcium phosphate precipitation method, electroporation method (Current Protocols in Molecular Biology (1987) Ausubel et al. edit, John Wily & Sons, Section 9.1-9.9), lipofectamine method (GIBCO-BRL), or microinjection method, etc.

The primer of the present invention can be used for synthesizing full-length cDNA, and also for the detection and/or diagnosis of the abnormality of the protein of the invention encoded by the full-length cDNA. For example, by utilizing polymerase chain reaction (genomic DNA-PCR, or RT-PCR) using the primer of the invention, DNA encoding the protein of the invention can be amplified. It is also possible to obtain the regulatory region of expression in the 5'-upstream by using PCR or hybridization since the transcription start site within the genomic sequence can be easily specified based on the 5'-end sequence of the full-length cDNA. The obtained genomic region can be used for detection and/or diagnosis of the abnormality of the sequence by RFLP analysis, SSCP, or direct sequencing.

Furthermore, the "polynucleotide having a length of at least 15 nucleotides, comprising a nucleotide sequence that is complementary to a polynucleotide comprising the nucleotide sequence set forth in any one of SEQ ID NOs in Table 370, or its complementary strand" includes an antisense polynucleotide for suppressing the expression of the protein of the invention. To exert the antisense effect, the antisense polynucleotide has a length of at least 15 bp or more, for example, 50 bp or more, preferably 100 bp or more, and more preferably 500 bp or more, and has a length of usually 3000 bp or less and preferably 2000 bp or less. The antisense DNA can be used in the gene therapy of the diseases that are caused by the abnormality of the protein of the invention (abnormal function or abnormal expression). Said antisense DNA can be prepared, for example, by the phosphorothioate method ("Physicochemical properties of phosphorothioate oligodeoxynucleotides." Stein (1988) Nucleic Acids Res. 16: 3209-3221) based on the nucleotide sequence of the DNA encoding the protein (for example, the DNA set forth in any one of SEQ ID NOs in Table 370).

The polynucleotide or antisense DNA of the present invention can be used in gene therapy, for example, by administrating it into a patient by the in vivo or ex vivo method with virus vectors such as retrovirus vectors, adenovirus vectors, and adeno-associated virus vectors, or non-virus vectors such as liposome.

The present invention also relates to antibodies that bind to the protein of the invention. There are no limitations in the form of the antibodies of the invention. They include polyclonal antibodies, monoclonal antibodies, or their portions that can bind to the protein of the invention. They also include antibodies of all classes. Furthermore, special antibodies such as humanized antibodies are also included.

The polyclonal antibody of the invention can be obtained according to the standard method by synthesizing an oligopeptide corresponding to the amino acid sequence and immunizing rabbits with the peptide (Current Protocols in Molecular Biology (1987) Ausubel et al. edit, John Wily & Sons, Section 11.12-11.13). The monoclonal antibody of the invention can be obtained according to the standard method by purifying the protein expressed in E. coli, immunizing mice with the protein, and producing a hybridoma cell by fusing the spleen cells and myeloma cells (Current Protocols in Molecular Biology (1987) Ausubel et al. edit, John Wily & Sons, Section 11.4-11.11).

The antibody binding to the protein of the present invention can be used for purification of the protein of the invention, and also for detection and/or diagnosis of the abnormalities of the expression and structure of the protein. Specifically, proteins can be extracted, for example, from tissues, blood, or cells, and the protein of the invention is detected by Western blotting, immunoprecipitation, or ELISA, etc. for the above purpose.

Furthermore, the antibody binding to the protein of the present invention can be utilized for treating the diseases that associates with the protein of the invention. If the antibodies are used for treating patients, human antibodies or humanized antibodies are preferable in terms of their low antigenicity. The human antibodies can be prepared by immunizing a mouse whose immune system is replaced with that of human ("Functional transplant of megabase human immunoglobulin loci recapitulates human antibody response in mice" Mendez M.J. et al. (1997) Nat. Genet. 15: 146-156). The humanized antibodies can be prepared by recombination of the hypervariable region of a monoclonal antibody (Methods in Enzymology (1991) 203: 99-121).

The present invention further relates to databases comprising at least a sequence of polynucleotide and/or protein, or a medium recorded in such databases, selected from the sequence data of the nucleotide and/or the amino acids indicated in Table 370. The term "database" means a set of accumulated information as machine-searchable and readable information of nucleotide sequence. The databases of the present invention comprise at least one of the novel nucleotide sequences of polynucleotide provided by the present invention. The databases of the present invention can consist of only the sequence data of the polynucleotide provided by the present invention or can comprise other information on nucleotide sequences of known full-length cDNAs or ESTs. The databases of the present invention can be comprised of not only the information on the nucleotide sequences but also the information on the gene functions revealed by the present invention. Additional information such as names of DNA clones carrying the full-length cDNAs can be recorded or linked together with the sequence data in the databases.

The database of the present invention is useful for gaining complete gene sequence information from partial sequence information of a gene of interest. The database of the present invention comprises nucleotide sequence information of full-length cDNAs. Consequently, by comparing the information in this database with the nucleotide sequence of a partial gene fragment yielded by differential display method or subtraction method, the information on the full-length nucleotide sequence of interest can be gained from the sequence of the partial fragment as a starting clue.

The sequence information of the full-length cDNAs constituting the database of the present invention contains not only the information on the complete sequences but also extra information on expression frequency of the genes as well as homology of the genes to known genes and known proteins. Thus the extra information facilitates rapid functional analyses of partial gene fragments. Further, the information on human genes is accumulated in the database of the present invention, and therefore, the database is useful for isolating a human homologue of a gene originating from other species. The human homologue can be isolated based on the nucleotide sequence of the gene from the original species.

At present, information on a wide variety of gene fragments can be obtained by differential display method and subtraction method. In general, these gene fragments are utilized as tools for isolating the full-length sequences thereof. When the gene fragment corresponds to an already-known gene, the full-length sequence is easily obtained by comparing the partial sequence with the information in known databases. However, when there exists no information corresponding to the partial sequence of interest in the known databases, cDNA cloning should be carried out for the full-length cDNA. It is often difficult to obtain the full-length nucleotide sequence using the partial sequence information as an initial clue. If the full-length of the gene is not available, the amino acid sequence of the protein encoded by the gene remains unidentified. Thus the database of the present invention can contribute to the identification of full-length cDNAs corresponding to gene fragments, which cannot be revealed by using databases of known genes.

The invention is illustrated more specifically with reference to the following examples, but is not to be construed as being limited thereto.

### EXAMPLE 1

### Construction of a cDNA library by the oligo-capping method.

The NT-2 neuron progenitor cells (Stratagene), a teratocarcinoma cell line from human embryo testis, which can differentiate into neurons by treatment with retinoic acid were used. The NT-2 cells were cultured according to the rnanufacturer's instructions as follows.
(1) NT-2 cells were cultured without induction by retinoic acid treatment ((NT2RM1, NT2RM2, NT2RM4)).
(2) After cultured, NT-2 cells were induced by adding retinoic acid, and then were cultured for 48 hours (NT2RP1).
(3) After cultured, NT-2 cells were induced by adding retinoic acid, and then were cultured for 2 weeks (NT2RP2, NT2RP3, NT2RP4).

Also, the human brain neuroglioma cell line H4 (ATCC HTG-148) (BNGH41), human neuroblastoma cell line SK-N-MC (ATCC HTB-10) (SKNMC1), and human retinoblastoma cell line Y79 (ATCC HTB-18) (Y79AA1) were cultured according to the culture conditions described in the ATCC catalogue. The cells were harvested separately, and mRNA was extracted from each cell by the method described in the literature (Molecular Cloning 2nd edition. Sambrook J., Fritsch, E.F., and Maniatis T. (1989) Cold Spring Harbor Laboratory Press). Furthermore, poly(A)⁺RNA was purified from the mRNA using oligo-dT cellulose.

Similarly, human placenta (PLACE1, PLACE2, PLACE3), human ovary cancer tissue (OVARC1), tissues from human embryo at 10 weeks, which is enriched with head (HEMBA1), or body (HEMBB1), human mammary gland (MAMMA1), human thyroid gland (THYRO1) were used to extract mRNA by the method described in the literature (Molecular Cloning 2nd edition. Sambrook J., Fritsch, E.F., and Maniatis T. (1989) Cold Spring Harbor Laboratory Press). Furthermore, poly(A)⁺RNA was purified from the mRNA using oligo-dT cellulose.

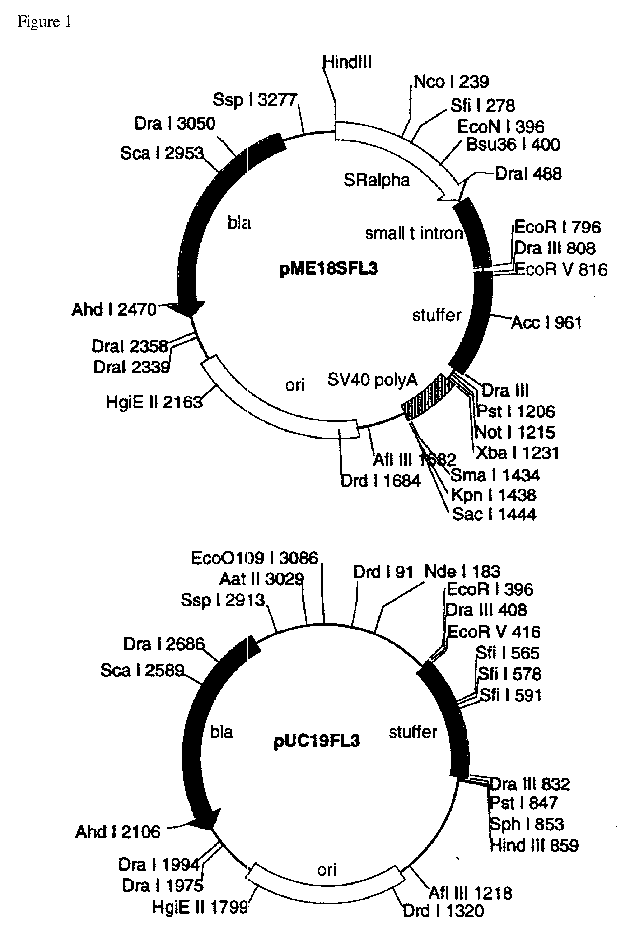

Each poly(A)⁺RNA was used to construct a cDNA library by the oligo-capping method (Maruyama M. and Sugano S. (1994) Gene 138: 171-174). Using the Oligo-cap linker (SEQ ID NO: 2541) and the Oligo-dT primer (SEQ ID NO: 2542), bacterial alkaline phosphatase (BAP) treatment, tobacco acid phosphatase (TAP) treatment, RNA ligation, the first strand cDNA synthesis, and removal of RNA were performed as described in the reference (Suzuki and Kanno (1996) Protein Nucleic acid and Enzyme. 41: 197-201; Suzuki Y. et al. (1997) Gene 200: 149-156). Next, 5'- and 3'-PCR primers (SEQ ID NO: 2543, and 2544, respectively) were used for performing PCR to convert the cDNA into double stranded cDNA, which was then digested with SfiI. Then, the DraIII-cleaved pUC19FL3 vector (Figure 1; for NT2RM1, and NT2RP1), or the DraIII-cleaved pME18SFL3 (Figure 1) (GenBank AB009864, expression vector; for NT2RM2, NT2RM4, NT2RP2, NT2RP3, NT2RP4, BNGH41, SKNMC1, Y79AA1, PLACE1, PLACE2, PLACE3, OVARC1, HEMBA1, HEMBB1, MAMMA1, and THYRO1) was used for cloning the cDNA in an unidirectional manner, and cDNA libraries were obtained. Then, the nucleotide sequence of the 5'- and 3'- ends of the cDNA clones was analyzed with a DNA sequencer (ABI PRISM 377, PE Biosystems) after sequencing reactions were performed with the DNA sequencing reagents (Dye Terminator Cycle Sequencing FS Ready Reaction Kit, dRhodamine Terminator Cycle Sequencing FS Ready Reaction Kit, or BigDye Terminator Cycle Sequencing FS Ready Reaction Kit, from by PE Biosystems) according to the instructions. The data were compiled into a database.

The full-length-enriched cDNA libraries except those for NT2RM1 and NT2RP1 were constructed using eukaryotic expression vector pME18SFL3. The vector contains SRα promoter and SV40 small t intron in the upstream of the cloning site, and SV40 polyA added signal sequence site in the downstream. As the cloning site of pME18SFL3 has asymmetrical DraIII sites, and the ends of cDNA fragments contain SfiI sites complementary to the DraIII sites, the cloned cDNA fragments can be inserted into the downstream of the SRα promoter unidirectionally. Therefore, clones containing full-length cDNA can be expressed transiently by introducing the obtained plasmid directly into COS cells. Thus, the clones can be analyzed very easily in terms of the proteins that are the gene products of the clones, or in terms of the biological

Herein, the cDNA libraries and the name of each clone are related as shown in Table 2. Therein, "xxxxxx" represents the clone number of six digits. Thus, the sequences are named by the library name, the clone number plus F- for the 5'-end, or R- for the 3'-end.

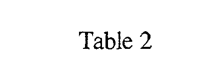

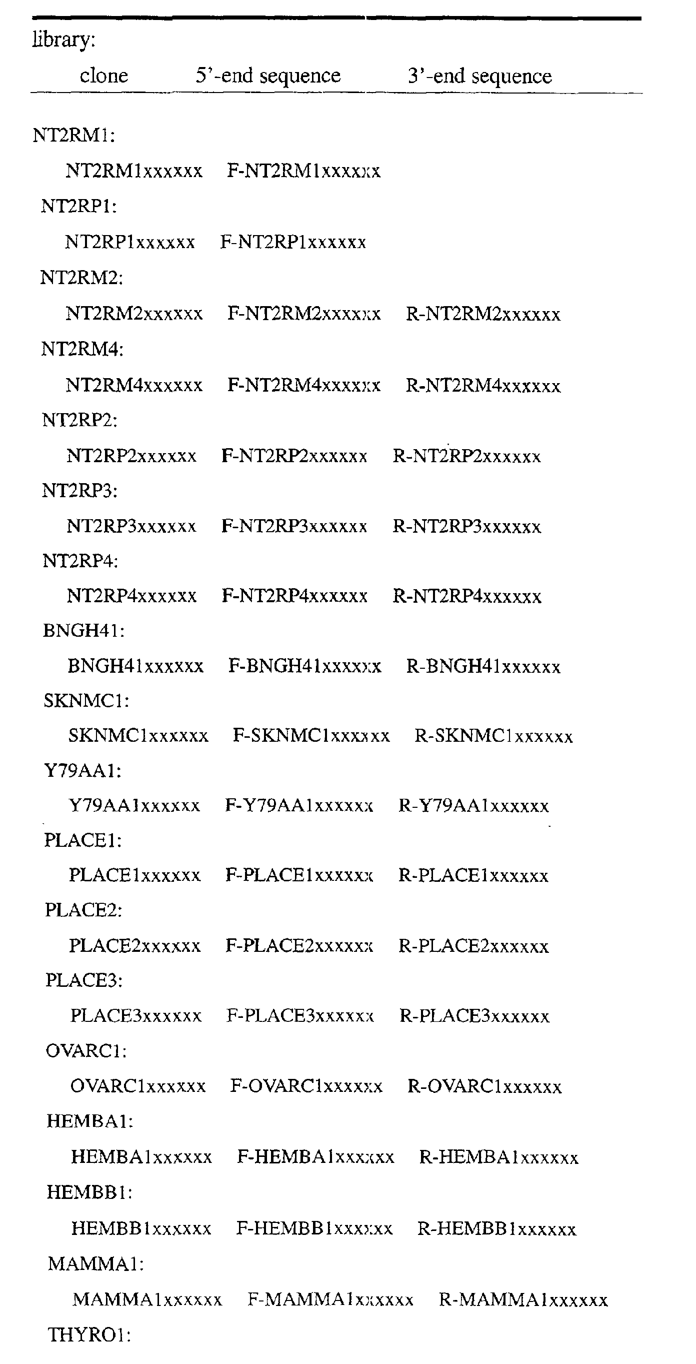

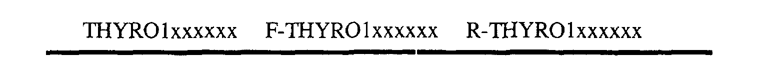

### EXAMPLE 2

### Estimation of the fullness ratio of the 5'-ends of the clones contained in the cDNA libraries constructed by the oligo-capping method.

The fullness ratio at the 5'-end sequences of the 59,823 clones in the human cDNA libraries constructed by the oligo-capping method was determined as follows. Of all the clones whose 5'-end sequences were found in those of known human mRNA in the public database, a clone was judged to be "full-length", if it had a longer 5'-end sequence than that of the known human mRNA, or, even though the 5'-end sequence was shorter, if it contained the translation initiation codon. A clone which did not contain the translation initiation codon was judged to be "non-full-length". The fullness ratio ((the number of full-length clones)/(the number of full-length and non-full-length clones)) at the 5'-end of the cDNA clones from each library was determined by comparing with the known human mRNA. As a result, the fullness ratio of the 5'-ends was 63.5%. It suggests that the human cDNA clones obtained by the described method have complete 5'-ends with high probability.

### EXAMPLE 3

### Assessment of the fullness ratio of the 5'-end of the cDNA by the ATGpr and the ESTiMateFL.

The ATGpr, developed by Salamov A.A., Nishikawa T., and Swindells M.B. in the Helix Research Institute, is a program for prediction of the translation start codon based on the characteristics of the sequences in the vicinity of the ATG codon. The results are shown with expectations that an ATG is a true start codon (0.05-0.94). When this program is applied to general cDNAs without considering whether or not the ATG codons in the cDNAs are the true initiation codons of the cDNAs, both the sensitivity and the specificity of the results are estimated at 66%. Here, the sensitivity means the ratio of the number of codons judged to be initiation codons by the program to the total number of true initiation codons, and the specificity means the ratio of the number of true initiation codons to the number of codons judged to be initiation codons by the program. In contrast, when the program was applied to the 5'-end sequences of the clones from the cDNA library that was obtained by the oligo-capping method and that had 65% fullness ratio, the sensitivity and specificity of evaluation of a full-length clone (clone containing the N-terminal end of ORF) were improved to 82-83% by selecting only clones having the ATGpr1 score 0.6 or higher.

Furthermore, the program was used to assess the fullness of 18,959 clones in the human cDNA libraries obtained here, which have 5'-ends matched to a known human mRNA. Briefly, the maximal ATGpr1 score of the clones was determined, and then their 5'-end sequence was compared with the known human mRNA to estimate whether the clone is full-length or not. The result was summarized in Table 3. Based on the knowledge that known mRNAs, in general, are highly expressed in the cell, the expression levels of genes having a low number in the EST hit, which represent mRNAs whose expression levels are relatively low were examined, and the result is shown in Table 4.

In the table, the number of full-length clones indicate that of clones containing the N-terminal end of ORF, and so does the number of non-full-length clones that of clones without the N-terminal end of ORF. The fullness ratio represents (the number of full-length clones)/(the number of full-length clones plus the number of non-full-length clones).

**Table 3**

| The maximal ATGpr1 score and the fullness ratio of the 5'-end sequences of clones obtained from human cDNA libraries constructed by the oligo-capping method; clones having a matched 5'-end with that of a known human mRNA. | | | |
|---|---|---|---|
| maximal ATGpr1 score | number of (full-length clones plus non-full-length clones) | number of full-length clones | fullness ratio |
| >=0.70 | 11,193 | 9,346 | 83.5% |
| >=0.50 | 13,369 | 10,549 | 78.9% |
| >=0.30 | 15,489 | 11,340 | 73.2% |
| >=0.15 | 17,394 | 11,811 | 67.9% |
| >=0.00 | 18,959 | 12,046 | 63.5% |

**Table 4**

| The maximal ATGpr1 score and the fullness ratio of the 5'-end sequences of the clones obtained from human cDNA libraries constructed by the oligo-capping method; clones having 5 EST hits or less among the clones having a matched 5'-end with that of a known human mRNA. | | | |
|---|---|---|---|
| maximal ATGpr1 score | number of (full-length clones plus non-full-length clones) | number of full-length clones | fullness ratio |
| >=0.70 | 2,801 | 1,934 | 69.0% |
| >=0.50 | 3,683 | 2,393 | 65.0% |
| >=0.30 | 4,683 | 2,707 | 57.8% |
| >=0.15 | 5,559 | 2,890 | 52.0% |
| >=0.00 | 6,113 | 3,013 | 49.8% |

The ESTiMateFL, developed by Nishikawa and Ota in the Helix Research Institute, is a method for the selection of a clone with high fullness ratio by comparing with the 5'-end or 3'-end sequences of ESTs in the public database.

By the method, a cDNA clone is judged presumably not to be full-length if there exist any ESTs which have longer 5'-end or 3'-end sequences than the clone. The method is systematized for high throughput analysis. A clone is judged to be full-length if the clone has a longer 5'-end sequence than ESTs in the public database. Even if a clone has a shorter 5'-end, the clone is judged to be full-length if the difference in length is within 50 bases, and otherwise judged not to be full-length, for convenience. In case of the 5'-end sequence of the clones which matches a known mRNA, about 80% of the sequences that were judged to be full-length by comparing with ESTs was judged to be full-length by estimating the 5'-end sequence, as well; about 80% of the sequences that were judged to be not full-length by comparing with ESTs was judged to be not full-length by estimating the 5'-end sequence, as well. The accuracy of the prediction by comparing cDNA clones with ESTs is improved with increasing number of ESTs to be compared. However, when only a limited number of ESTs are available, the reliability becomes low. Thus, the method is effective in excluding clones with high probability of being non-full-length, from the cDNA clones that is synthesized by the oligo-capping method and that have the 5'-end sequences with about 60 % fullness ratio. In particular, the ESTiMateFL is efficiently used to estimate the fullness ratio at the 3'-end sequence of cDNA of a human unknown mRNA which has a significant number of ESTs in the public database.

The 18,959 clones isolated from human cDNA libraries constructed by the oligo-capping method, which have the 5'-end sequence that matches a known human mRNA, were estimated by using the ATGpr and ESTiMateFL. Briefly, the 5'-end sequence that matches a known human mRNA of the respective clone was analyzed to obtain the maximal ATGpr1 score, and compared with the ORF of the known human mRNA that matches it to determine whether the clone is full-length or not. Then, the 5'-end sequence of the respective clone was analyzed by the ESTiMateFL to judge whether the clone is full-length or not. Specifically, the 5'-end sequences that match a known human mRNA of the 18,959 clones constructed by the oligo-capping method were compared with those of ESTs by the ESTiMateFL and the clones other than those that are not full-length were selected. Then, the selected clones were used to analyze the relationship between the ATGpr and the fullness ratio. The result was summarized in Table 5. Also, among the selected, the clones in which the number of the EST hit is not more than 5 were selected and analyzed. The result was summarized in Table 6, which represents the result of the analysis of mRNA with relatively low abundance.

In the Tables, the number of full-length clones, the number of non-full-length clones, and the fullness ratio indicate the number of the clones that contain the N-terminus of the ORF, the number of the clones that do not contain the N-terminus of the ORF, and (the number of full-length clones)/(the number of full-length clones) plus (the number of non-full-length clones), respectively.

**Table 5**

| The maximal ATGpr1 score and the fullness ratio of the 5'-end sequence in the clones isolated from human cDNA libraries constructed by the oligo-capping method, which have the 5'-end sequence that matches a known human mRNA, and also other than those being not full-length according to the comparison with ESTs. | | | |
|---|---|---|---|
| maximal ATGpr1 score | number of (full-length clones plus non-full-length clones) | number of full-length clones | fullness ratio |
| >=0.70 | 9,068 | 8,349 | 92.1% |
| >=0.50 | 10,345 | 9,318 | 90.1% |
| >=0.30 | 11,425 | 9,964 | 87.2% |
| >=0.15 | 12,254 | 10,335 | 84.3% |
| >=0.00 | 12,785 | 10,484 | 82.0% |

**Table 6**

| The maximal ATGpr1 score and the fullness ratio of the 5'-end sequence in the clones isolated from human cDNA libraries constructed by the oligo-capping method, which have the 5'-end sequence that matches a known human mRNA, and also other than those being not full-length according to the comparison with ESTs, in which the number of the EST hit is not more than 5. | | | |
|---|---|---|---|
| maximal ATGpr1 score | number of (full-length clones plus non-full-length clones) | number of full-length clones | fullness ratio |
| >=0.70 | 1,959 | 1,510 | 77.1% |
| >=0.50 | 2,469 | 1,821 | 73.8% |
| >=0.30 | 2,975 | 2,046 | 68.8% |
| >=0.15 | 3,368 | 2,164 | 64.3% |
| >=0.00 | 3,661 | 2,226 | 60.8% |

According to the above results, it was found that, in case of using clones isolated from human cDNA libraries constructed by the oligo-capping method, the fullness ratio of the clones that have low score in the ATGpr can be improved by assessing their 5'-end sequence using the combination of the ATGpr and the ESTiMateFL. Therefore, the method was applied to select a cDNA clone with high fullness ratio.

### EXAMPLE 4

### Clustering of the 5'-end and 3'-end sequences of cDNA clones.

The 5'-end and 3'-end sequences of cDNA clones were obtained, and clustered separately. Briefly, data of the single pass sequencing of the determined 5'-end and 3'-end of cDNA clones was subjected to the BLAST search between the sequence data of all the clones synthesized in Example 1, and clones that are supposed to be originating from the same gene were clustered into a group. For the 5'-end sequence, those having the consensus sequence of 95% identity 300 base pairs or more are clustered into the same group. For the 3'-end sequence, those having the consensus sequence of 90% identity 200 base pairs or more are clustered into the same group. Among the clusters of the 5'-end and 3'-end sequences, the sequence having the longest lead was chosen as the representative sequence of the cluster (group).

### EXAMPLE 5

### Characterization of the representative sequences and the sequences of clones

Data of the 5'-end sequences of the representative sequences and clones was characterized by the following methods:
(1) judging whether it is identical to the sequence of mRNA or ESTs from human by the BLAST search of the GenBank or SwissProt, and examining whether it is full-length by comparing with the sequences of known mRNA and ESTs from human.
(2) determining the ATGpr1 score using all the initiation codons contained within the 5'-end sequence by the ATGpr which predict fullness ratio.
(3) predicting the existence of the signal sequence using all the initiation codons contained within the 5'-end sequence by the PSORT which predict signal.
   and,
(4) only with the 5'-end sequences of the representative sequences of the clusters, examining the keywords in the top hit data of the homology search of the SwissProt.

Data of the characterized representative sequences and clones was used for the final selection of the clones.

### EXAMPLE 6

### Identity to the human mRNA and human EST, and comparison of the 5'-end length.

The clones and the representative sequences of the clusters were judged to be identical to any human mRNA, if their 5'-end sequence has a region of 200 nucleotides or longer with 94% or more identity to the mRNA. The clones and the representative sequences of the clusters were judged to be identical to any human EST, if their 5'-end sequence has a region of 200 nucleotides or longer with 90% or more identity to the EST.

The clones and the representative sequences of the clusters were judged to be full-length in comparison with human mRNA, if their 5'-end sequence is longer than those of the mRNA, or it contains the translation initiation site. The clones and the representative sequences of the clusters were judged to be full-length in comparison with human EST in the database, if their 5'-end sequence is longer than those of the EST, or even though it is shorter, the difference in length between the two sequences is 50 nucleotides or less, for convenience. Otherwise, the clones and the representative sequences of the clusters were judged to be not full-length.

### EXAMPLE 7

### Prediction of the fullness ratio by the ATGpr.

The score in the ATGpr1 is the expectation to be full-length based on calculations, and the higher score reflects the higher fullness ratio as shown in Example 3. Further, the maximal ATGpr1 score represents the score obtained with all the initiation codons contained in the 5'-end sequence of the clones and the representative sequences, and are used for the characterization.

### EXAMPLE 8

### Prediction of the existence of a signal sequence by the PSORT.

Prediction of the existence of a signal sequence by the PSORT was performed on all of the amino acid sequences predicted from all the initiation codons in the 5'-end sequence of the clones and the representative sequences of the clusters. By analyzing the presence or absence of the sequence which is predicted to be a signal sequence, which is characteristics of the N-terminus of many secretory proteins, cDNA clones encoding a secretory protein or membrane protein were selected.

### EXAMPLE 9

### Prediction of the protein function by the BLAST search.

The 5'-end sequence of the representative sequences of the cluster was analyzed by the BLAST homology search of the SwissProt. The obtained top hit data was classified into those identical to the 5'-end representative sequence (identity was 90% or higher), those not identical to the 5'-end representative sequence (identity was 60% or lower, and compared sequence was not more than 25 nucleotides), and those similar to the 5'-end representative sequence (the rest of the data).

All the keywords in the SwissProt data corresponding to the top hit data were selected, and the 5'-end representative sequences were classified by the keywords relating with functions.
The keywords relating with a secretory protein or membrane protein are the followings:
growth factor,
cytokine,
hormone,
receptor,
G-protein coupled receptor,
ionic channel,
voltage-gated channel,
calcium channel,
extracellular matrix,
transmembrane, and
signal.

The keywords relating to glycoprotein is glycoprotein.

The keywords relating to signal transduction are the followings:
serine/threonine-protein kinase,
tyrosine-protein kinase, and
calmodulin-binding.

The keywords relating to transcription are the followings:
transcription regulation and activator,
transcription regulation and repressor, and
nuclear protein and repressor.

The keywords relating to diseases are disease mutation, and syndrome.

Many keywords overlapped in the respective group (receptor and transmembrane, for example), and some keywords overlapped in different groups (secretory or membrane, and diseases, etc.).

### EXAMPLE 10

### Selection of clones by characterization.

From the data obtained by the above characterization, clones encoding a novel secretory protein or membrane protein, or proteins with other predicted functions were selected by the combination of the ATGpr1 score and the prediction of the signal sequence by the PSORT, or according to the top hit data in the homology search of the SwissProt.

In selecting the clones, the 5'-end sequences that are identical to a human mRNA were ignored, whereas those that are identical to a human mRNA in part but obviously not identical in the other part were included. Because there were clones selected that are identical to a human mRNA in part but obviously not identical in the other part.

Also, if the finally selected clones were found to be not full-length compared with the sequences of human mRNA and ESTs, these clones were discarded.

### EXAMPLE 11

### A method for selection of clones by the combination of the ATGpr1 score and the prediction of the signal sequence by the PSORT (a method for selection of secretory proteins and membrane proteins that are novel and full-length).

The sequences of clones and the representative sequences of their clusters were used to obtain the maximal ATGpr1 score and predict the presence of the signal sequence. First, clones were selected based on the representative sequences of the clusters. The correspondence between the name and SEQ ID of the representative sequences used for selection (Table 368), and the correspondence between the name and SEQ ID of the introns (including the representative sequences of the 5'-end and 3'-end, and ESTs) used for selection of clones from the representative sequences of the groups (Table 369) were shown in the last part of the present specification. Therein, HRIFA and HRIRA indicate the representative sequence of the 5'-end group, and that of the 3'-end group, respectively.

In the clusters in which a single clone is contained (the sequence of the 5'-end clone = the representative sequence of the 5'-end), selected were the clones that were judged to be full-length in comparison with human mRNA and ESTs, having the maximal ATGpr1 score 0.5 or higher, and predicted to contain the signal sequence, in principle. However, in the following cases, a clone having a longer 5'-end was selected: the maximal ATGpr1 score was less than 0.5, the sequence of the 5'-end was not full-length, the clone was obviously shorter although the clone was not classified into the same cluster according to the BLAST search of the other clones, or the 5'-end sequence corresponding to the 3'-end of the other clones in the same cluster in which the 3'-end sequence of the clone was contained was found to be longer by assembling. Furthermore, if there were multiple full-length clones in the same cluster and it was not successful to determine by assembling which has the longer 5'-end, all the clones were selected. For assembling, the Sequencher^{(™)} (Hitachi Soft Engineering ) was used. As a result, the signal sequence predicted to be present in the representative sequence was not present in some of the selected clones. In some cases, the ATGpr1 score became smaller than 0.5 or 0.3. The fullness ratio in these clones was low, yet still it is possible that the clones are full-length. The clones in which the signal sequence predicted to be present in the representative sequence was not present after selection were likely to be without the signal sequence, but still it is possible that the clones encode a membrane protein.

In the clusters comprising multiple clones, in which the representative sequence of the 5'-end was predicted to contain the signal sequence, selected were the clones having the longest 5'-end sequence among the clones which were judged to be full-length compared with human mRNA and ESTs, having the maximal ATGpr1 score for the 5'-end sequence 0.5 or higher, and predicted to contain the signal sequence. However, in the following cases, a clone having a longer 5'-end was selected: the maximal ATGpr1 score was less than 0.5, the sequence of the 5'-end was not full-length, the clone was obviously shorter although the clone was not classified into the same cluster according to the BLAST search of the other clones, or the 5'-end sequence corresponding to the 3'-end of the other clones in the same cluster in which the 3'-end sequence of the clone was contained was found to be longer. Furthermore, if there were multiple full-length clones in the same cluster and it was not successful to determine by assembling which has the longer 5'-end, all the clones were selected. As a result, the signal sequence predicted to be present in the representative sequence was not present in some of the selected clones. In some cases, the ATGpr1 score became smaller than 0.5 or 0.3. The fullness ratio in these clones was low, yet still it is possible that the clones are full-length. The clones in which the signal sequence predicted to be present in the representative sequence was not present after selection were likely to be without the signal sequence at the 5'-end, but still it is possible that the clones encode a membrane protein.

Next, in the clusters comprising multiple clones, in which the representative sequence of the 5'-end was predicted to have no signal sequence, selected were the clones which were judged to be full-length compared with human mRNA and ESTs, having the maximal ATGpr1 score for the 5'-end sequence 0.5 or higher, and predicted to contain the signal sequence.

The number of the clones selected by the combination of the ATGpr1 score and the prediction of a signal sequence by the PSORT were 254. The number of the clones having the maximal ATGpr1 score 0.5 or higher, and predicted to contain a signal sequence were 170 (Table 7-10). Among the clones, 164 clones were found to have the representative sequence of the original cluster that fulfills the same conditions. On the other hand, 5 clones were selected from the representative sequences of the 5'-end of the clusters which was predicted to contain a signal sequence while the maximal ATGpr1 score was lower than 0.5. A clone was selected from the representative sequence of the 5'-end of the cluster which was predicted to have no signal sequence.

The clones that have the maximal ATGpr1 score 0.3 or higher and less than 0.5 and predicted to contain the signal sequence were 35 clones (Table 11), in which 8 clones were found to have the representative sequence of the original cluster that fulfills the same conditions. Twenty-seven clones were selected from the representative sequences of the clusters which have the maximal ATGpr1 score 0.5 or higher and were predicted to have no signal sequence.

The clones that have the maximal ATGpr1 score less than 0.3 and were predicted to contain a signal sequence were 41 clones (Table 12). The clones that have the maximal ATGpr1 score 0.5 or higher and were predicted to have no signal sequence were 4 clones (Table 13). The clones that have the maximal ATGpr1 score 0.3 or higher and less than 0.5 and were predicted to have no signal sequence were 2 clones (Table 14). The clones that have the maximal ATGpr1 score less than 0.3 and were predicted to contain a signal sequence were 2 clones (Table 15). The representative sequences of the original clusters of all the clones had the maximal ATGpr1 score 0.3 or higher, and were predicted to contain a signal sequence.

The fullness ratio of the clones having the maximal ATGpr1 score 0.5 or higher, 0.3 or higher, and 0 or higher is expected to be as shown in Table 3, 4, 5, and 6.

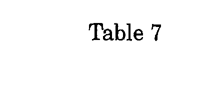

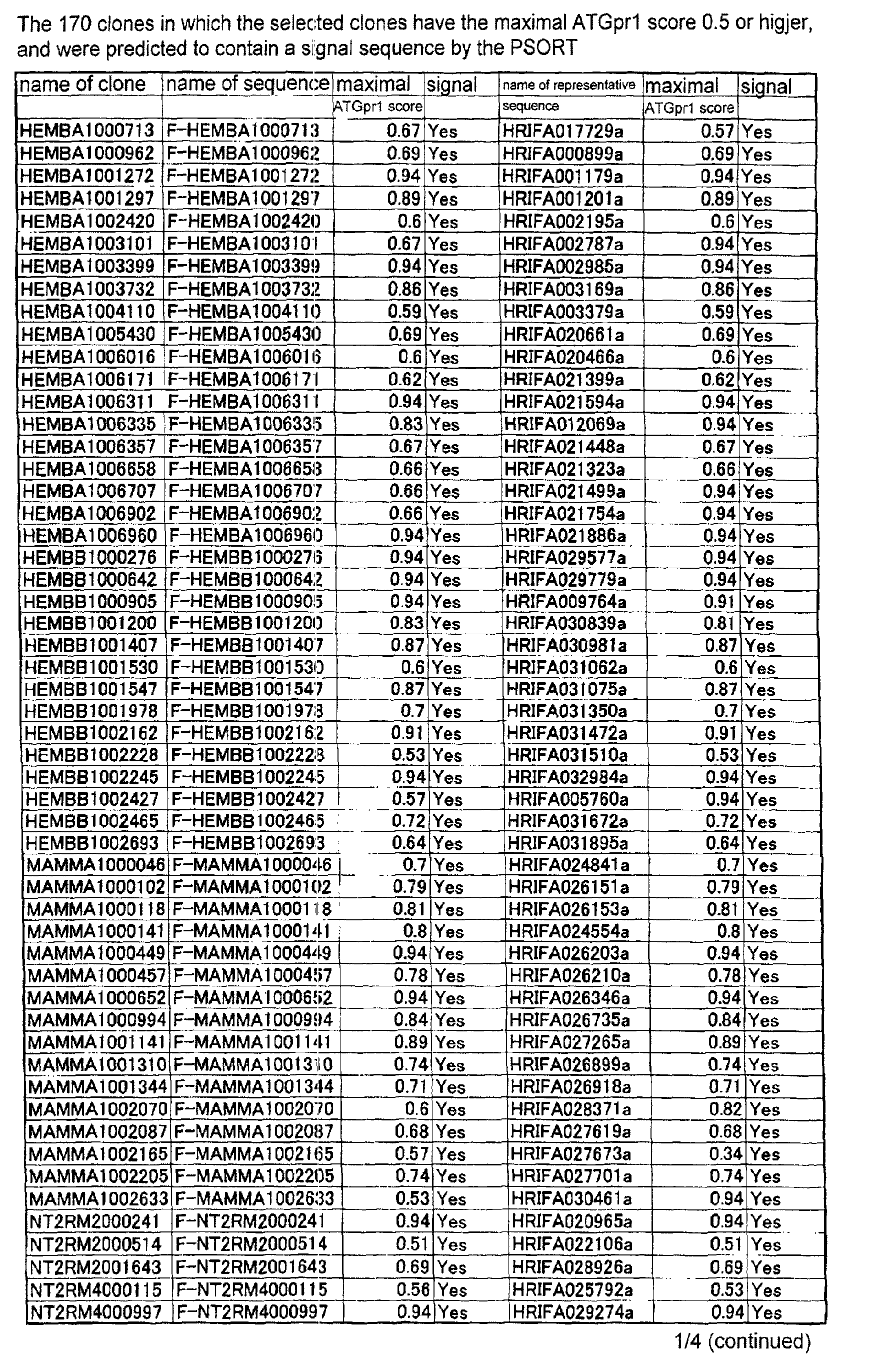

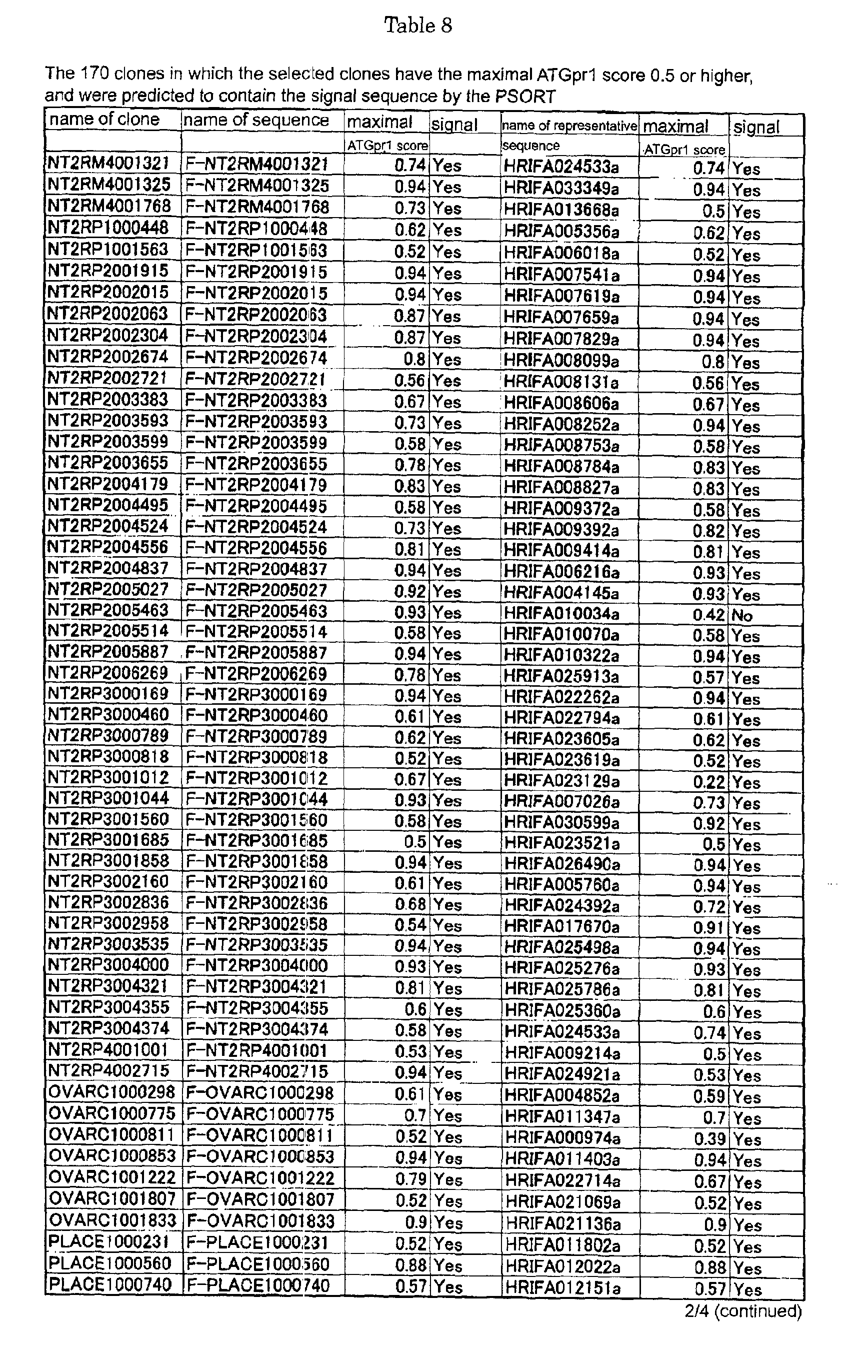

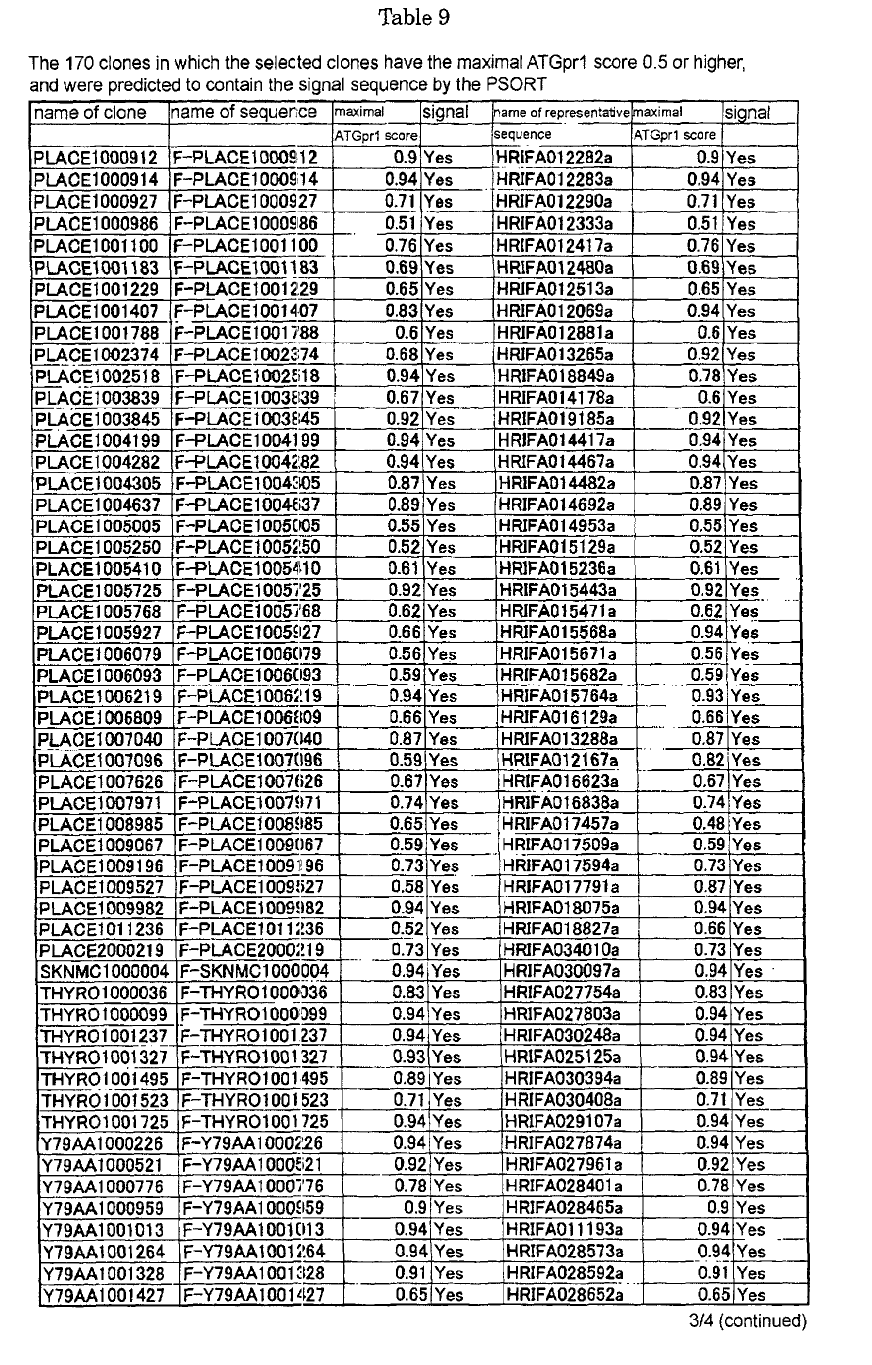

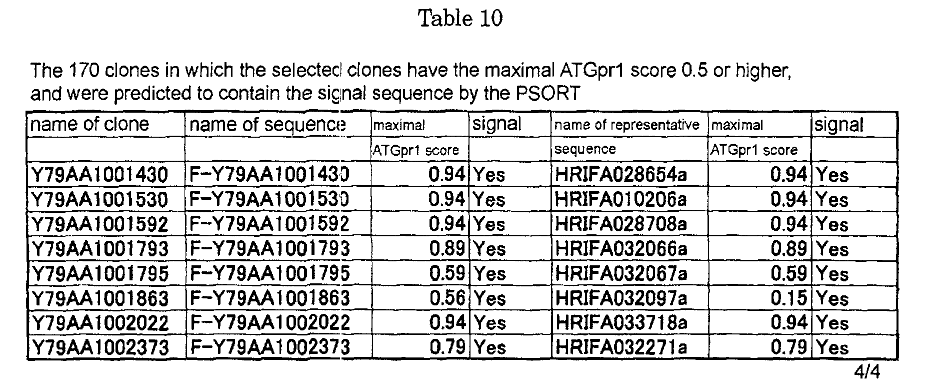

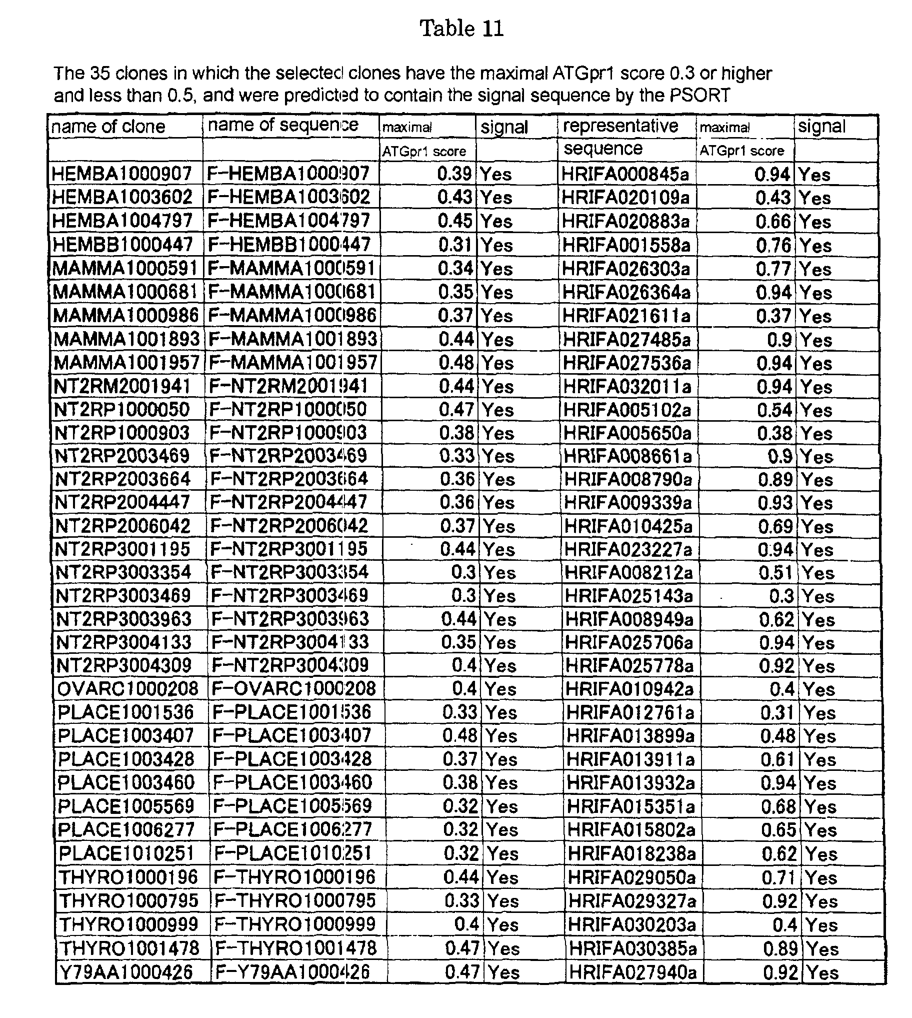

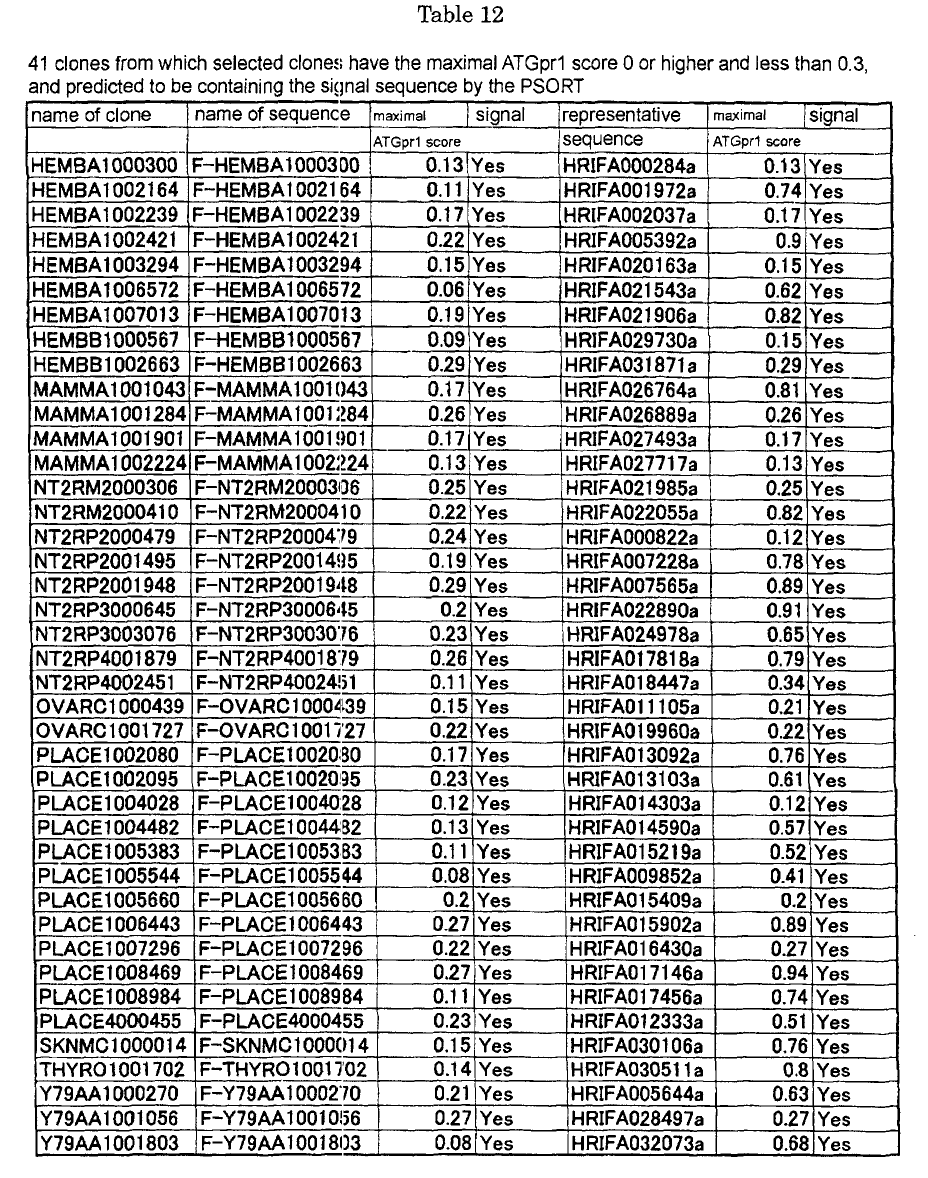

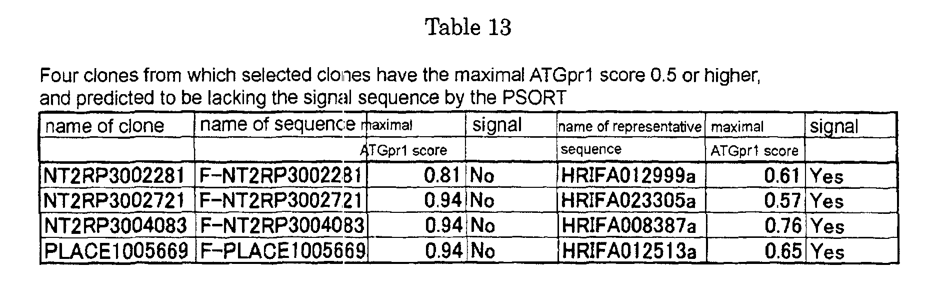

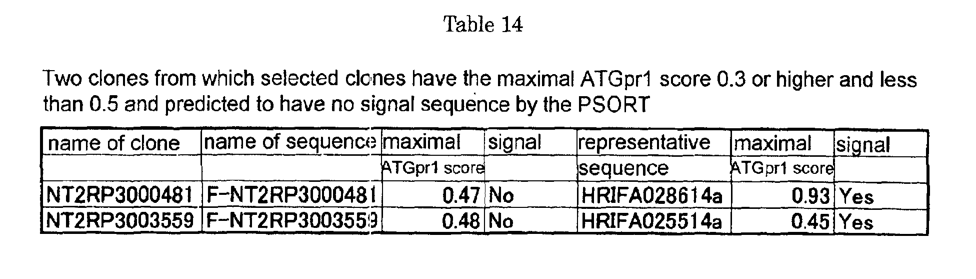

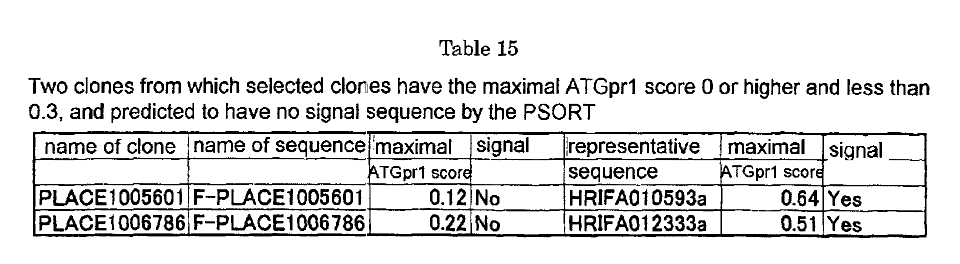

### EXAMPLE 12

### A method for the selection of clones based on the top hit data in the homology search against the SwissProt (a method for the selection of a novel full-length protein that is predicted to have a function based on the top hit data).

The representative sequences of the clusters were discarded in which the 5'-end sequence is identical (90% or more matching), or not similar (the compared part contains a sequence of 25 nucleotides or shorter and the similarity is lower than 60%) to the top hit data in the SwissProt. Then, the remaining representative sequences which has similarity to the representative sequences of the 5'-ends were classified by a group of the above keywords (some representative sequences belong to a group by multiple keywords), and then clones were selected from the clusters. The names and the corresponding SEQ IDs of the representative sequences, and also the names of the introns (including the representative sequence of the 5'-end or the 3'-end, or ESTs) used for selecting the clones from the representative sequences and the corresponding SEQ IDs are shown in the last part of the present specification (Table 368 and 369, respectively). HRIFA indicates the representative sequence of the 5'-end group, and HRIRA indicates the representative sequence of the 3'-end group.

In principle, from the clusters containing only a single clone (the 5'-end sequence is the representative sequence of the cluster), the clone was selected. However, in the following cases, the clone containing a longer 5'-end was selected: where the maximal ATGpr1 score was less than 0.5, the 5'-end sequence of the clone to be selected was not complete, or the 5'-end of the clone was found to be obviously short nevertheless the clone should not be included in the same cluster based on the BLAST analysis between the other clones, or further, the 5'-end sequence of the said clone, which corresponds to the 3'-ends of the other clones belonging to the same cluster in which the 3'-end of the said clone was included, was turn out to be longer than those of the other clones by assembling them. When there were two clones in the same cluster, judged to be full-length, and it was difficult to determine which clone has the longer 5'-end even by assembling them, all the clones were selected. As a result, the ATGpr1 score in some clones became less than 0.5 or less than 0.3. The fullness ratio of these clones became lower, but there is still a possibility that the clones are full-length.

In the case in which multiple clones were contained in a cluster, selected was the clone having the longest 5'-end in the clones judged to be full-length compared to the human mRNA or human EST. However, in the following cases, the clone containing a longer 5'-end was selected: where the maximal ATGpr1 score was less than 0.5, the 5'-end sequence of the clone to be selected was not complete, or the 5'-end of the clone was found to be obviously short nevertheless the clone should not be included in the same cluster based on the BLAST analysis between the other clones, or further, the 5'-end sequence of the said clone, which corresponds to the 3'-ends of the other clones belonging to the same cluster in which the 3'-end of the said clone was included, was turn out to be longer than those of the other clones by assembling them. When there were two clones in the same cluster, judged to be full-length, and it was difficult to determine which clone has the longer 5'-end even by assembling them, all the clones were selected. As a result, the ATGpr1 score in some clones became less than 0.5 or less than 0.3. These clones can still be full-length.

Based on the top hit data in the SwissProt homology search, 658 clones were selected. Among them, 446 clones were selected by the keywords, secretion or membrane. Using the keyword, glycoprotein, 243 clones were selected. 51 clones were selected by the keywords for signal transduction. With the keywords for transcription, 130 clones were selected. 17 clones were selected by the keywords for disease.

Among the 446 clones selected by the keywords, secretion or membrane, 77 clones were overlapped with those selected by combining the ATGpr1 score and prediction by the PSORT for the existence of a signal sequence. Also, many clones were overlapped with those selected by the keyword, glycoprotein. Moreover, some clones were overlapped with the clones selected by the keywords for diseases.

Among the 243 clones selected by the keyword, glycoprotein, 53 clones were overlapped with those selected by combining the ATGpr1 score and prediction by the PSORT for the existence of a signal sequence. Also, many clones were overlapped with those selected by the keywords, secretion or membrane. Moreover, some clones were overlapped with the clones selected by the keywords in diseases.

Among the clones selected by the top hit data in the homology search on the SwissProt, 532 clones were having the maximal ATGpr1 score 0.5 or higher. 59 clones were having the maximal score 0.3 or higher and less than 0.5. 67 clones were with the maximal score less than 0.3.

When the maximal ATGpr1 score is 0.5 or higher, 0.3 or higher, no less than 0, the expected fullness ratio is as shown in Table 3, 4, 5, and 6, respectively.

**Table 16**

| The representative sequences of the most homologous sequences in the SwissProt with the keyword(s) "growth factor", "cytokine", or "hormone", and the selected clones. | |
|---|---|
| name of clone | name of representative sequence |
| HEMBA1001563 | HRIFA001439a |
| HEMBA1003047 | HRIFA002743a |
| HEMBA1005070 | HRIFA020144a |
| HEMBA1006724 | HRIFA021620a |
| HEMBA1006916 | HRIFA021855a |
| MAMMA1001066 | HRIFA027355a |
| MAMMA1001634 | HRIFA027187a |
| MAMMA1002165 | HRIFA027673a |
| NT2RM4000326 | HRIFA032530a |
| NT2RM4001377 | HRIFA005300a |
| NT2RP2000447 | HRIFA006448a |
| NT2RP2000663 | HRIFA006609a |
| NT2RP2000903 | HRIFA006798a |
| NT2RP2002974 | HRIFA027860a |
| NT2RP2003369 | HRIFA008596a |
| NT2RP2004141 | HRIFA009123a |
| NT2RP2005941 | HRIFA010361a |
| NT2RP2006099 | HRIFA010466a |
| NT2RP3000645 | HRIFA022890a |
| NT2RP3000838 | HRIFA005300a |
| NT2RP4002451 | HRIFA018447a |
| OVARC1000275 | HRIFA010988a |
| OVARC1001030 | HRIFA021061a |
| PLACE1004492 | HRIFA014598a |
| PLACE1009279 | HRIFA017643a |
| THYRO1001071 | HRIFA029440a |
| Y79AA1000207 | HRIFA027867a |
| Y79AA1000426 | HRIFA027940a |

**Table 17**

| The representative sequences of the most homologous sequences in the SwissProt with the keyword(s) "receptor", "G-protein coupled receptor", "ionic channel", "voltage-gated channel", or "calcium channel", and the selected clones. | |
|---|---|
| name of clone | name of representative sequence |
| BNGH41000091 | HRIFA029511a |
| HEMBA1001621 | HRIFA001489a |
| HEMBA1003392 | HRIFA002980a |
| HEMBA1005545 | HRIFA020272a |
| HEMBA1007291 | HRIFA022462a |
| HEMBA1007332 | HRIFA022493a |
| MAMMA1000681 | HRIFA026364a |
| MAMMA1000706 | HRIFA026382a |
| MAMMA 1001978 | HRIFA027549a |
| NT2RM2001939 | HRIFA032009a |
| NT2RM2001941 | HRIFA032011a |
| NT2RM4002352 | HRIFA001337a |
| NT2RP2002510 | HRIFA007985a |
| NT2RP2002533 | HRIFA008000a |
| NT2RP2005181 | HRIFA005409a |
| NT2RP3000304 | HRIFA022616a |
| NT2RP3001542 | HRIFA028501a |
| NT2RP3002409 | HRIFA024197a |
| NT2RP3002836 | HRIFA024392a |
| NT2RP3003000 | HRIFA024767a |
| NT2RP3004552 | HRIFA025904a |
| NT2RP4001877 | HRIFA032433a |
| NT2RP4002750 | HRIFA028157a |
| OVARC1000090 | HRIFA010859a |
| OVARC1000956 | HRIFA011484a |
| OVARC1001991 | HRIFA019498a |
| PLACE1001016 | HRIFA012354a |
| PLACE1001340 | HRIFA012584a |
| PLACE1001401 | HRIFA012625a |
| PLACE 1001564 | HRIFA012737a |
| PLACE1001655 | HRIFA012795a |
| PLACE 1002547 | HRIFA013376a |
| PLACE 1002967 | HRIFA013620a |
| PLACE 1003573 | HRIFA014006a |
| PLACE1003852 | HRIFA014185a |
| PLACE1004441 | HRIFA014561a |
| PLACE1005031 | HRIFA014967a |
| PLACE1005878 | HRIFA015536a |
| PLACE1007296 | HRIFA016430a |
| PLACE 1008469 | HRIFA017146a |
| PLACE1010784 | HRIFA031126a |
| PLACE1010968 | HRIFA018666a |
| THYRO1000956 | HRIFA029393a |
| Y79AA1001062 | HRIFA023434a |

**Table 18**

| The representative sequences of the most homologous sequenses in the SwissProt with the keyword(s) "extracellular matrix", and the selected clones. | |
|---|---|
| name of clone | name of representative sequence |
| HEMBA1000006 | HRIFA027327a |
| HEMBA1000275 | HRIFA000264a |
| HEMBA1000835 | HRIFA000776a |
| HEMBA1000907 | HRIFA000845a |
| HEMBA1002164 | HRIFA001972a |
| HEMBA1003101 | HRIFA002787a |
| HEMBA1003230 | HRIFA002891a |
| MAMMA1000403 | HRIFA026465a |
| MAMMA1001615 | HRIFA022865a |
| MAMMA1001893 | HRIFA027485a |
| MAMMA1002128 | HRIFA027644a |
| NT2RM4000284 | HRIFA032506a |
| NT2RM4000295 | HRIFA032511a |
| NT2RM4000587 | HRIFA032696a |
| NT2RP2000616 | HRIFA006572a |
| NT2RP2000694 | HRIFA006633a |
| NT2RP2001562 | HRIFA010799a |
| NT2RP2001948 | HRIFA007565a |
| NT2RP2002409 | HRIFA007909a |
| NT2RP2004447 | HRIFA009339a |
| NT2RP2004847 | HRIFA005944a |
| NT2RP2006004 | HRIFA002766a |
| NT2RP3000059 | HRIFA022203a |
| NT2RP3000616 | HRIFA022875a |
| NT2RP3000871 | HRIFA023048a |
| NT2RP3000921 | HRIFA023069a |
| NT2RP3002015 | HRIFA015995a |
| NT2RP3002448 | HRIFA024218a |
| NT2RP3002983 | HRIFA024473a |
| NT2RP3003729 | HRIFA025488a |
| NT2RP3004067 | HRIFA027327a |
| OVARC1001049 | HRIFA022702a |
| OVARC1001222 | HRIFA022714a |
| OVARC1002058 | HRIFA022737a |
| PLACE1001114 | HRIFA012427a |
| PLACE1002329 | HRIFA013235a |
| PLACE1004816 | HRIFA014819a |
| PLACE1005383 | HRIFA015219a |
| PLACE1005569 | HRIFA015351a |
| PLACE1006073 | HRIFA003402a |
| PLACE1006277 | HRIFA015802a |
| PLACE1008984 | HRIFA017456a |
| THYR01001102 | HRIFA008174a |
| THYRO1001471 | HRIFA030381a |
| THYRO1001478 | HRIFA030385a |
| Y79AA1000888 | HRIFA028440a |

**Table 19**

| The representative sequences of the most homologous sequences in the SwissProt with the keyword(s) "transmembrane", and the selected clones (except overlapped with Table 16, 17, and 18) | |
|---|---|
| name of clone | name of representative sequence |
| BNGH41000020 | HRIFA030662a |
| HEMBA1000121 | HRIFA000116a |
| HEMBA1000349 | HRIFA000327a |
| HEMBA1000477 | HRIFA000446a |
| HEMBA1000940 | HRIFA002384a |
| HEMBA1002163 | HRIFA001971a |
| HEMBA1002421 | HRIFA005392a |
| HEMBA1002767 | HRIFA002503a |
| HEMBA1003945 | HRIFA009220a |
| HEMBA1004250 | HRIFA003504a |
| HEMBA1004391 | HRIFA020693a |
| HEMBA1004444 | HRIFA029285a |
| HEMBA1004454 | HRIFA003592a |
| HEMBA1004505 | HRIFA003635a |
| HEMBA1004797 | HRIFA020883a |
| HEMBA1004982 | HRIFA003892a |
| HEMBA1005489 | HRIFA024543a |
| HEMBA1005698 | HRIFA020453a |
| HEMBA1005945 | HRIFA020349a |
| HEMBA1006299 | HRIFA025771a |
| HEMBA1006430 | HRIFA021213a |
| HEMBA1006482 | HRIFA022328a |
| HEMBA1007241 | HRIFA022423a |
| HEMBB1000679 | HRIFA029802a |
| HEMBB1001200 | HRIFA030839a |
| HEMBB1001573 | HRIFA031091a |
| HEMBB1002427 | HRIFA005760a |
| MAMMA1000204 | HRIFA025966a |
| MAMMA1000473 | HRIFA018870a |
| MAMMA1000196 | HRIFA026242a |
| MAMMA1000788 | HRIFA018287a |
| MAMMA1000814 | HRIFA026618a |
| MAMMA1001237 | HRIFA026860a |
| MAMMA1001418 | HRIFA027045a |
| MAMMA1002091 | HRIFA027622a |
| MAMMA1002095 | HRIFA027625a |
| MAMMA1002586 | HRIFA027012a |
| NT2RM1000580 | HRIFA004523a |
| NT2RM1000855 | HRIFA004696a |
| NT2RM1000858 | HRIFA004714a |
| NT2RM1000899 | HRIFA004745a |
| NT2RM2000565 | HRIFA022139a |
| NT2RM2000582 | HRIFA021787a |
| NT2RM2001126 | HRIFA024088a |
| NT2RM4000198 | HRIFA032453a |
| NT2RM4000417 | HRIFA032587a |
| NT2RM4000444 | HRIFA032605a |
| NT2RM4000593 | HRIFA023489a |
| NT2RM4000761 | HRIFA023923a |
| NT2RM4000965 | HRIFA030103a |
| NT2RM4001735 | HRIFA002063a |
| NT2RP1000181 | HRIFA005184a |
| NT2RP1000261 | HRIFA005231a |
| NT2RP1000300 | HRIFA005255a |
| NT2RP1000325 | HRIFA005271a |
| NT2RP1000551 | HRIFA005420a |
| NT2RP1000981 | HRIFA005702a |
| NT2RP2000533 | HRIFA006510a |
| NT2RP2000649 | HRIFA006596a |
| NT2RP2000818 | HRIFA006730a |
| NT2RP2001200 | HRIFA007013a |
| NT2RP2001495 | HRIFA007228a |
| NT2RP2001514 | HRIFA007243a |
| NT2RP2001956 | HRIFA007571a |
| NT2RP2002063 | HRIFA007659a |
| NT2RP2002232 | HRIFA009783a |
| NT2RP2002527 | HRIFA023767a |
| NT2RP2002942 | HRIFA008284a |
| NT2RP2002976 | HRIFA008314a |
| NT2RP2003210 | HRIFA008483a |
| NT2RP2003390 | HRIFA008611a |
| NT2RP2003469 | HRIFA008661a |
| NT2RP2003655 | HRIFA008784a |
| NT2RP2003664 | HRIFA008790a |
| NT2RP2004205 | HRIFA009171a |
| NT2RP2004794 | HRIFA009578a |
| NT2RP2005425 | HRIFA010005a |
| NT2RP2005597 | HRIFA010130a |
| NT2RP2005632 | HRIFA010152a |
| NT2RP2005994 | HRIFA010394a |
| NT2RP2006269 | HRIFA025913a |
| NT2RP2006512 | HRIFA024937a |
| NT2RP3000125 | HRIFA022234a |
| NT2RP3000171 | HRIFA007722a |
| NT2RP3000676 | HRIFA026576a |
| NT2RF3000907 | HRIFA025046a |
| NT2RP3001061 | HRIFA023154a |
| NT2RP3001170 | HRIFA010078a |
| NT2RP3001195 | HRIFA023227a |
| NT2RP3001240 | HRIFA023257a |
| NT2RP3001322 | HRIFA023304a |
| NT2RP3001388 | HRIFA006926a |
| NT2RP3001560 | HRIFA030599a |
| NT2RP3001738 | HRIFA025766a |
| NT2RP3002160 | HRIFA005760a |
| NT2RP3002324 | HRIFA024884a |
| NT2RP3002342 | HRIFA006586a |
| NT2RP3002571 | HRIFA024255a |
| NT2RP3002900 | HRIFA024423a |
| NT2RP3002958 | HRIFA017670a |
| NT2RP3003532 | HRIFA013477a |
| NT2RP3003939 | HRIFA025636a |
| NT2RP3004130 | HRIFA025703a |
| NT2RP3004133 | HRIFA025706a |
| NT2RP3004294 | HRIFA025771a |
| NT2RP3004406 | HRIFA025800a |
| NT2RP3004481 | HRIFA026089a |
| NT2RP3004625 | HRIFA026564a |
| NT2RP3004647 | HRIFA026576a |
| NT2RP4001009 | HRIFA022227a |
| NT2RP4001879 | HRIFA017818a |
| OVARC1000003 | HRIFA010790a |
| OVARC1000105 | HRIFA007493a |
| OVARC1000137 | HRIFA010891a |
| OVARC1000307 | HRIFA002919a |
| OVARC1000331 | HRIFA004162a |
| OVARC1000553 | HRIFA011197a |
| OVARC1000873 | HRIFA032478a |
| OVARC1001163 | HRIFA032079a |
| OVARC1001260 | HRIFA019867a |
| OVARC1001336 | HRIFA019867a |
| OVARC1001607 | HRIFA022729a |
| PLACE1000740 | HRIFA012151a |
| PLACE1001123 | HRIFA012436a |
| PLACE1001231 | HRIFA012515a |
| PLACE1001836 | HRIFA012914a |
| PLACE1001949 | HRIFA012990a |
| PLACE1002095 | HRIFA013103a |
| PLACE1002905 | HRIFA013586a |
| PLACE1002911 | HRIFA013589a |
| PLACE1003163 | HRIFA013744a |
| PLACE1003644 | HRIFA014056a |
| PLACE1003737 | HRIFA014111a |
| PLACE1004279 | HRIFA014465a |
| PLACE1004450 | HRIFA014568a |
| PLACE1004482 | HRIFA014590a |
| PLACE1004630 | HRIFA014688a |
| PLACE1005544 | HRIFA009852a |
| PLACE1005745 | HRIFA017855a |
| PLACE1005927 | HRIFA015568a |
| PLACE1006290 | HRIFA015811a |
| PLACE1007096 | HRIFA012167a |
| PLACE1007845 | HRIFA016758a |
| PLACE1007881 | HRIFA016240a |
| PLACE1008359 | HRIFA015547a |
| PLACE1008716 | HRIFA017295a |
| PLACE1008985 | HRIFA017457a |
| PLACE1009600 | HRIFA017836a |
| PLACE1010011 | HRIFA018092a |
| PLACE1010078 | HRIFA018131a |
| PLACE1010251 | HRIFA018238a |
| PLACE1010445 | HRIFA010736a |
| PLACE1010827 | HRIFA018580a |
| PLACE1011045 | HRIFA014500a |
| PLACE1011181 | HRIFA018794a |
| PLACE1011236 | HRIFA018827a |
| PLACE1011516 | HRIFA018993a |
| PLACE3000181 | HRIFA004112a |
| SKNMC1000082 | HRIFA030147a |
| THYRO1000196 | HRIFA029050a |
| THYRO1000400 | HRIFA000564a |
| THYRO1000584 | HRIFA029209a |
| THYRO1000678 | HRIFA029256a |
| THYRO1000776 | HRIFA029317a |
| THYRO1000795 | HRIFA029327a |
| THYRO1000866 | HRIFA027714a |
| THYRO1001113 | HRIFA029460a |
| THYRO1001128 | HRIFA029467a |
| THYRO1001242 | HRIFA032360a |
| THYRO1001266 | HRIFA030264a |
| THYRO1001456 | HRIFA030370a |
| THYRO1001529 | HRIFA030411a |
| THYRO1001702 | HRIFA030511a |
| Y79AA1000127 | HRIFA026121a |
| Y79AA1000270 | HRIFA005644a |
| Y79AA1001426 | HRIFA028651a |
| Y79AA1001787 | HRIFA028790a |
| Y79AA1001799 | HRIFA032070a |
| Y79AA1002213 | HRIFA032224a |

**Table 20**

| The representative sequences of the most homologous sequences in the SwissProt with the keyword(s) "signal", and the selected clones (except overlapped with Table 16, 17, 18 and 19) | |
|---|---|
| name of clone | name of representative sequence |
| BNGH41000087 | HRIFA029508a |
| HEMBA1000128 | HRIFA000123a |
| HEMBA1000443 | HRIFA000415a |
| HEMBA1000590 | HRIFA000553a |
| HEMBA1000634 | HRIFA004780a |
| HEMBA1000745 | HRIFA000695a |
| HEMBA1001221 | HRIFA001132a |
| HEMBA1001228 | HRIFA001138a |
| HEMBA1001390 | HRIFA000071a |
| HEMBA1002131 | HRIFA001942a |
| HEMBA1002167 | HRIFA001975a |
| HEMBA1002178 | HRIFA001984a |
| HEMBA1002524 | HRIFA002284a |
| HEMBA1002992 | HRIFA002694a |
| HEMBA1003072 | HRIFA002762a |
| HEMBA1003315 | HRIFA000016a |
| HEMBA1003487 | HRIFA003055a |
| HEMBA1003530 | HRIFA003093a |
| HEMBA1005145 | HRIFA003946a |
| HEMBA1005337 | HRIFA019651a |
| HEMBA1005449 | HRIFA020707a |
| HEMBA1005522 | HRIFA021398a |
| HEMBA1006335 | HRIFA012069a |
| HEMBA1006572 | HRIFA021543a |
| HEMBA1006707 | HRIFA021499a |
| HEMBA1006749 | HRIFA021637a |
| HEMBA1006902 | HRIFA021754a |
| HEMBA1007013 | HRIFA021906a |
| HEMBA1007057 | HRIFA022985a |
| HEMBB1000447 | HRIFA001558a |
| HEMBB1000567 | HRIFA029730a |
| HEMBB1000881 | HRIFA029932a |
| HEMBB1001026 | HRIFA030025a |
| HEMBB1001048 | HRIFA030045a |
| HEMBB1001847 | HRIFA031249a |
| MAMMA1000106 | HRIFA024482a |
| MAMMA1000226 | HRIFA025978a |
| MAMMA1000591 | HRIFA026303a |
| MAMMA1001043 | HRIFA026764a |
| MAMMA1001957 | HRIFA027536a |
| MAMMA1002080 | HRIFA016963a |
| MAMMA1002234 | HRIFA027722a |
| MAMMA1002633 | HRIFA030461a |
| MAMMA1003126 | HRIFA029263a |
| NT2RM1000462 | HRIFA004426a |
| NT2RM1000542 | HRIFA004490a |
| NT2RM2000410 | HRIFA022055a |
| NT2RM2000423 | HRIFA022065a |
| NT2RM2000622 | HRIFA022156a |
| NT2RM2000773 | HRIFA023894a |
| NT2RM2001626 | HRIFA028911a |
| NT2RM2001818 | HRIFA031935a |
| NT2RM4000648 | HRIFA032730a |
| NT2RM4001843 | HRIFA024718a |
| NT2RP1000050 | HRIFA005102a |
| NT2RP1001004 | HRIFA005720a |
| NT2RP2000394 | HRIFA003640a |
| NT2RP2000514 | HRIFA006494a |
| NT2RP2001480 | HRIFA007219a |
| NT2RP2001755 | HRIFA007424a |
| NT2RP2001878 | HRIFA007512a |
| NT2RP2002188 | HRIFA007745a |
| NT2RP2002564 | HRIFA007244a |
| NT2RP2002824 | HRIFA008200a |
| NT2RP2003042 | HRIFA008362a |
| NT2RP2003593 | HRIFA008252a |
| NT2RP2003931 | HRIFA008976a |
| NT2RP2004606 | HRIFA009451a |
| NT2RP2004648 | HRIFA009482a |
| NT2RP2005163 | HRIFA009825a |
| NT2RP2005247 | HRIFA009881a |
| NT2RP2005378 | HRIFA004919a |
| NT2RP2005541 | HRIFA010090a |
| NT2RP2005883 | HRIFA010319a |
| NT2RP2006042 | HRIFA010425a |
| NT2RP3000063 | HRIFA022528a |
| NT2RP3000436 | HRIFA022776a |
| NT2RP3000444 | HRIFA022782a |
| NT2RP3000481 | HRIFA028614a |
| NT2RP3000721 | HRIFA009825a |
| NT2RP3001012 | HRIFA023129a |
| NT2RP3001159 | HRIFA023212a |
| NT2RP3001592 | HRIFA023464a |
| NT2RP3001754 | HRIFA007728a |
| NT2RP3002311 | HRIFA024718a |
| NT2RP3002738 | HRIFA020748a |
| NT2RP3002790 | HRIFA026519a |
| NT2RP3002887 | HRIFA029278a |
| NT2RP3003354 | HRIFA008212a |
| NT2RP3003448 | HRIFA025479a |
| NT2RP3003473 | HRIFA001413a |
| NT2RP3003614 | HRIFA032642a |
| NT2RP3004075 | HRIFA010301a |
| NT2RP3004090 | HRIFA027329a |
| NT2RP3004202 | HRIFA025327a |
| NT2RP3004309 | HRIFA025778a |
| NT2RP3004345 | HRIFA025353a |
| NT2RP3004557 | HRIFA025907a |
| NT2RP4001467 | HRIFA013276a |
| OVARC1000313 | HRIFA011016a |
| OVARC1000410 | HRIFA022691a |
| OVARC1000439 | HRIFA011105a |
| OVARC1001086 | HRIFA011580a |
| OVARC1001569 | HRIFA022728a |
| OVARC1001570 | HRIFA019412a |
| PLACE1001407 | HRIFA012069a |
| PLACE1001464 | HRIFA013276a |
| PLACE1001516 | HRIFA012702a |
| PLACE1001795 | HRIFA012885a |
| PLACE1001918 | HRIFA012969a |
| PLACE1002080 | HRIFA0130921a |
| PLACE1002153 | HRIFA013135a |
| PLACE1002355 | HRIFA013254a |
| PLACE1002374 | HRIFA013265a |
| PLACE1002726 | HRIFA018688a |
| PLACE1003428 | HRIFA013911a |
| PLACE1003460 | HRIFA013932a |
| PLACE1003772 | HRIFA014133a |
| PLACE1004078 | HRIFA014336a |
| PLACE1004520 | HRIFA014621a |
| PLACE1004648 | HRIFA014702a |
| PLACE1004887 | HRIFA014868a |
| PLACE1005426 | HRIFA015246a |
| PLACE1006071 | HRIFA016639a |
| PLACE1006443 | HRIFA015902a |
| PLACE1006716 | HRIFA016070a |
| PLACE1006959 | HRIFA016214a |
| PLACE1007077 | HRIFA016639a |
| PLACE1007081 | HRIFA016290a |
| PLACE1007702 | HRIFA016669a |
| PLACE1008657 | HRIFA017257a |
| PLACE1008744 | HRIFA017312a |
| PLACE1009546 | HRIFA017801a |
| PLACE1011116 | HRIFA018754a |
| PLACE1011708 | HRIFA019105a |
| PLACE2000118 | HRIFA024994a |
| PLACE3000213 | HRIFA015486a |
| PLACE4000354 | HRIFA015486a |
| THYRO1000061 | HRIFA013279a |
| THYRO1000846 | HRIFA029349a |
| THYRO1001063 | HRIFA029434a |
| THYRO1001608 | HRIFA050456a |
| THYRO1001803 | HRIFA030566a |
| Y79AA1000876 | HRIFA030629a |
| Y79AA1001090 | HRIFA028511a |
| Y79AA1001272 | HRIFA028576a |
| Y79AA1001727 | HRIFA006642a |
| Y79AA1001803 | HRIFA032073a |
| Y79AA1002376 | HRIFA032820a |

**Table 21**

| The representative sequences of the most homologous sequences in the SwissProt with the keyword(s) "glycoprotein", and the selected clones | |
|---|---|
| name of clone | name of representative sequence |
| BNGH41000087 | HRIFA029508a |
| BNGH41000091 | HRIFA029511a |
| HEMBA1000275 | HRIFA000264a |
| HEMBA1000349 | HRIFA000327a |
| HEMBA1000590 | HRIFA000553a |
| HEMBA1000634 | HRIFA004780a |
| HEMBA1000835 | HRIFA000776a |
| HEMBA1000907 | HRIFA000845a |
| HEMBA1001221 | HRIFA001132a |
| HEMBA1001228 | HRIFA001138a |
| HEMBA1001621 | HRIFA001489a |
| HEMBA1002131 | HRIFA001942a |
| HEMBA1002164 | HRIFA001972a |
| HEMBA1002167 | HRIFA001975a |
| HEMBA1002178 | HRIFA001984a |
| HEMBA1002316 | HRIFA002102a |
| HEMBA1002421 | HRIFA005392a |
| HEMBA1002767 | HRIFA002503a |
| HEMBA1003047 | HRIFA002743a |
| HEMBA1003101 | HRIFA002787a |
| HEMBA1003230 | HRIFA002891a |
| HEMBA1003392 | HRIFA002980a |
| HEMBA1004250 | HRIFA003504a |
| HEMBA1004391 | HRIFA020693a |
| HEMBA1004444 | HRIFA029285a |
| HEMBA1004454 | HRIFA003592a |
| HEMBA1004505 | HRIFA003635a |
| HEMBA1005449 | HRIFA020707a |
| HEMBA1005489 | HRIFA024543a |
| HEMBA1005522 | HRIFA021398a |
| HEMBA1005545 | HRIFA020272a |
| HEMBA1006335 | HRIFA012069a |
| HEMBA1006572 | HRIFA021543a |
| HEMBA1006707 | HRIFA021499a |
| HEMBA1006724 | HRIFA021620a |
| HEMBA1006749 | HRIFA021637a |
| HEMBA1006902 | HRIFA021754a |
| HEMBA1007057 | HRIFA022985a |
| HEMBA1007332 | HRIFA022493a |
| HEMBB1000447 | HRIFA001558a |
| HEMBB1000567 | HRIFA029730a |
| HEMBB1000679 | HRIFA029802a |
| HEMBB1000881 | HRIFA029932a |
| HEMBB1001048 | HRIFA030045a |
| HEMBB1002427 | HRIFA005760a |
| MAMMA1000106 | HRIFA024482a |
| MAMMA1000403 | HRIFA026465a |
| MAMMA1000591 | HRIFA026303a |
| MAMMA1000681 | HRIFA026364a |
| MAMMA1000706 | HRIFA026382a |
| MAMMA1001043 | HRIFA026764a |
| MAMMA1001237 | HRIFA026860a |
| MAMMA1001615 | HRIFA022865a |
| MAMMA1001893 | HRIFA027485a |
| MAMMA1001978 | HRIFA027549a |
| MAMMA1002070 | HRIFA028371a |
| MAMMA1001091 | HRIFA027622a |
| MAMMA1002128 | HRIFA027644a |
| MAMMA1002586 | HRIFA027012a |
| MAMMA1003126 | HRIFA029263a |
| NT2RM1000462 | HRIFA004426a |
| NT2RM1000542 | HRIFA004490a |
| NT2RM1000580 | HRIFA004523a |
| NT2RM2000423 | HRIFA022065a |
| NT2RM2001626 | HRIFA028911a |
| NT2RM2001792 | HRIFA029002a |
| NT2RM2001818 | HRIFA031935a |
| NT2RM2001939 | HRIFA032009a |
| NT2RM2001941 | HRIFA032011a |
| NT2RM4000198 | HRIFA032453a |
| NT2RM4000284 | HRIFA032506a |
| NT2RM4000417 | HRIFA032587a |
| NT2RM4000587 | HRIFA032696a |
| NT2RM4000648 | HRIFA032730a |
| NT2RM4001843 | HRIFA024718a |
| NT2RM4002352 | HRIFA001337a |
| NT2RP1000002 | HRIFA005072a |
| NT2RP1000050 | HRIFA005102a |
| NT2RP1000613 | HRIFA005462a |
| NT2RP1000981 | HRIFA005702a |
| NT2RP1001004 | HRIFA005720a |
| NT2RP2000394 | HRIFA003640a |
| NT2RP2000514 | HRIFA006494a |
| NT2RP2000616 | HRIFA006572a |
| NT2RP2001480 | HRIFA007219a |
| NT2RP2001562 | HRIFA010799a |
| NT2RP2001755 | HRIFA007424a |
| NT2RP2001878 | HRIFA007512a |
| NT2RP2002188 | HRIFA007745a |
| NT2RP2002304 | HRIFA007829a |
| NT2RP2002409 | HRIFA007909a |
| NT2RP2002510 | HRIFA007985a |
| NT2RP2002533 | HRIFA008000a |
| NT2RP2002564 | HRIFA007244a |
| NT2RP2002942 | HRIFA008284a |
| NT2RP2003042 | HRIFA008362a |
| NT2RP2003469 | HRIFA008661a |
| NT2RP2003931 | HRIFA008976a |
| NT2RP2004205 | HRIFA009171a |
| NT2RP2004447 | HRIFA009339a |
| NT2RP2004606 | HRIFA009451a |
| NT2RP2004648 | HRIFA009482a |
| NT2RP2004847 | HRIFA005944a |
| NT2RP2005181 | HRIFA005409a |
| NT2RP2005247 | HRIFA009881a |
| NT2RP2005541 | HRIFA010090a |
| NT2RP2005597 | HRIFA010130a |
| NT2RP2005632 | HRIFA010152a |
| NT2RP2005883 | HRIFA010319a |
| NT2RP2006004 | HRIFA002766a |
| NT2RP2006042 | HRIFA010425a |
| NT2RP2006269 | HRIFA025913a |
| NT2RP3000059 | HRIFA022203a |
| NT2RP3000063 | HRIFA022528a |
| NT2RP3000125 | HRIFA022234a |
| NT2RP3000304 | HRIFA022616a |
| NT2RP3000481 | HRIFA028614a |
| NT2RP3000616 | HRIFA022875a |
| NT2RP3000871 | HRIFA023048a |
| NT2RP3000921 | HRIFA023069a |
| NT2RP3001012 | HRIFA023129a |
| NT2RP3001061 | HRIFA023154a |
| NT2RP3001159 | HRIFA023212a |
| NT2RP3001542 | HRIFA028501a |
| NT2RP3001560 | HRIFA030599a |
| NT2RP3001754 | HRIFA007728a |
| NT2RP3002015 | HRIFA015995a |
| NT2RP3002160 | HRIFA005760a |
| NT2RP3002311 | HRIFA024718a |
| NT2RP3002448 | HRIFA024218a |
| NT2RP3002738 | HRIFA020748a |
| NT2RP3002836 | HRIFA024392a |
| NT2RP3002958 | HRIFA017670a |
| NT2RP3002983 | HRIFA024473a |
| NT2RP3003000 | HRIFA024767a |
| NT2RP3003076 | HRIFA024978a |
| NT2RP3003354 | HRIFA008212a |
| NT2RP3003532 | HRIFA013477a |
| NT2RP3003729 | HRIFA025488a |
| NT2RP3004130 | HRIFA025703a |
| NT2RP3004133 | HRIFA025706a |
| NT2RP3004309 | HRIFA025778a |
| NT2RP3004481 | HRIFA026089a |
| NT2RP3004552 | HRIFA025904a |
| NT2RP3004625 | HRIFA026564a |
| NT2RP3004640 | HRIFA030250a |
| NT2RP4000108 | HRIFA001341a |
| NT2RP4001467 | HRIFA013276a |
| NT2RP4001877 | HRIFA032433a |
| NT2RP4002750 | HRIFA028157a |
| OVARC1000003 | HRIFA010790a |
| OVARC1000090 | HRIFA010859a |
| OVARC1000313 | HRIFA011016a |
| OVARC1000467 | HRIFA011128a |
| OVARC1000553 | HRIFA011197a |
| OVARC1000873 | HRIFA032478a |
| OVARC1000956 | HRIFA011484a |
| OVARC1001049 | HRIFA022702a |
| OVARC1001086 | HRIFA011580a |
| OVARC1001260 | HRIFA019867a |
| OVARC1001336 | HRIFA019867a |
| OVARC1001569 | HRIFA022728a |
| OVARC1001570 | HRIFA019412a |
| OVARC1001607 | HRIFA022729a |
| OVARC1001991 | HRIFA019498a |
| OVARC1002058 | HRIFA022737a |
| PLACE1000740 | HRIFA012151a |
| PLACE1001016 | HRIFA012354a |
| PLACE1001114 | HRIFA012427a |
| PLACE1001123 | HRIFA012436a |
| PLACE1001231 | HRIFA012515a |
| PLACE1001407 | HRIFA012069a |
| PLACE1001464 | HRIFA013276a |
| PLACE1001516 | HRIFA012702a |
| PLACE1001564 | HRIFA012737a |
| PLACE1001655 | HRIFA012795a |
| PLACE1001836 | HRIFA012914a |
| PLACE1002095 | HRIFA013103a |
| PLACE1002329 | HRIFA013235a |
| PLACE1002355 | HRIFA013254a |
| PLACE1002374 | HRIFA013265a |
| PLACE1002905 | HRIFA013586a |
| PLACE1002911 | HRIFA013589a |
| PLACE1003163 | HRIFA013744a |
| PLACE1003428 | HRIFA013911a |
| PLACE1003438 | HRIFA013919a |
| PLACE1003573 | HRIFA014006a |
| PLACE1003737 | HRIFA014111a |
| PLACE1003852 | HRIFA014185a |
| PLACE1004441 | HRIFA014561a |
| PLACE1004450 | HRIFA014568a |
| PLACE1004520 | HRIFA014621a |
| PLACE1004630 | HRIFA014688a |
| PLACE1004816 | HRIFA014819a |
| PLACE1005003 | HRIFA014951a |
| PLACE1005383 | HRIFA015219a |
| PLACE1005426 | HRIFA015246a |
| PLACE1005539 | HRIFA029425a |
| PLACE1005544 | HRIFA009852a |
| PLACE1005569 | HRIFA015351a |
| PLACE1006071 | HRIFA016639a |
| PLACE1006073 | HRIFA003402a |
| PLACE1006277 | HRIFA015802a |
| PLACE1006290 | HRIFA015811a |
| PLACE1006443 | HRIFA015902a |
| PLACE1006716 | HRIFA016070a |
| PLACE1007077 | HRIFA016639a |
| PLACE1007081 | HRIFA016290a |
| PLACE1007845 | HRIFA016758a |
| PLACE1008469 | HRIFA017146a |
| PLACE1008716 | HRIFA017295a |
| PLACE1008744 | HRIFA017312a |
| PLACE1008984 | HRIFA017456a |
| PLACE1008985 | HRIFA017457a |
| PLACE1009527 | HRIFA017791a |
| PLACE1010251 | HRIFA018238a |
| PLACE1010784 | HRIFA031126a |
| PLACE1010968 | HRIFA018666a |
| PLACE1011116 | HRIFA018754a |
| PLACE1011708 | HRIFA019105a |
| PLACE2000118 | HRIFA024994a |
| PLACE3000181 | HRIFA004112a |
| PLACE3000213 | HRIFA015486a |
| PLACE4000354 | HRIFA015486a |
| SKNMC1000014 | HRIFA030106a |
| THYRO1000196 | HRIFA029050a |
| THYRO1000584 | HRIFA029209a |
| THYRO1000956 | HRIFA029393a |
| THYRO1001102 | HRIFA008174a |
| THYRO1001128 | HRIFA029467a |
| THYRO1001266 | HRIFA030264a |
| THYRO1001803 | HRIFA030566a |
| Y79AA1000127 | HRIFA026121a |
| Y79AA1000207 | HRIFA027867a |
| Y79AA1000270 | HRIFA005644a |
| Y79AA1000426 | HRIFA027940a |
| Y79AA1000888 | HRIFA028440a |
| Y79AA1001062 | HRIFA023434a |
| Y79AA1001272 | HRIFA028576a |
| Y79AA1001426 | HRIFA028651a |
| Y79AA1001523 | HRIFA030642a |
| Y79AA1001727 | HRIFA006642a |
| Y79AA1001863 | HRIFA032097a |

**Table 22**

| The representative sequences of the most homologous sequences in the SwissProt with the keyword(s) "serine/threonine-protein kinase", "tyrosine-protein kinase", or "calmodulin-binding", and the selected clones | |
|---|---|
| name of clone | name of representative sequence |
| HEMBA1001878 | HRIFA001712a |
| HEMBA1002195 | HRIFA017703a |
| HEMBA1002227 | HRIFA019136a |
| HEMBA1002551 | HRIFA002309a |
| HEMBA1005084 | HRIFA020184a |
| HEMBA1005913 | HRIFA029866a |
| HEMBA1005929 | HRIFA020335a |
| HEMBB1000668 | HRIFA029792a |
| MAMMA1000881 | HRIFA026659a |
| MAMMA1001150 | HRIFA026813a |
| MAMMA1002142 | HRIFA027656a |
| NT2RM2000589 | HRIFA021794a |
| NT2RM2001902 | HRIFA031986a |
| NT2PP1001020 | HRIFA005728a |
| NT2RP1001031 | HRIFA005732a |
| NT2RP2001469 | HRIFA028061a |
| NT2RP2001529 | HRIFA007256a |
| NT2RP2001769 | HRIFA007435a |
| NT2RP2003179 | HRIFA008459a |
| NT2RP2003545 | HRIFA008717a |
| NT2RP2004670 | HRIFA028468a |
| NT2RP3000011 | HRIFA022177a |
| NT2RP3000022 | HRIFA022182a |
| NT2RP3000172 | HRIFA022265a |
| NT2RP3000201 | HRIFA022546a |
| NT2RP3000820 | HRIFA018262a |
| NT2RP3003527 | HRIFA025492a |
| NT2RP3003849 | HRIFA025250a |
| NT2RP3003874 | HRIFA025261a |
| NT2RP4000634 | HRIFA029866a |
| NT2RP4000962 | HRIFA027681a |
| OVARC1000255 | HRIFA010975a |
| OVARC1000529 | HRIFA011179a |
| OVARC1000916 | HRIFA011449a |
| OVARC1001338 | HRIFA019869a |
| PLACE1003135 | HRIFA013726a |
| PLACE1005519 | HRIFA015070a |
| PLACE1005736 | HRIFA015453a |
| PLACE1008282 | HRIFA016654a |
| PLACE1008297 | HRIFA017031a |
| PLACE1010081 | HRIFA018134a |
| PLACE1011364 | HRIFA018904a |
| PLACE1011824 | HRIFA019175a |
| THYRO1001205 | HRIFA030237a |
| THYRO1001457 | HRIFA030371a |
| THYRO1001593 | HRIFA030448a |
| THYRO1001700 | HRIFA030509a |
| THYRO1001770 | HRIFA030545a |
| Y79AA1000777 | HRIFA028402a |
| Y79AA1000967 | HRIFA028468a |
| Y79AA1002381 | HRIFA032275a |

**Table 23**

| The representative sequences of the most homologous sequences in the SwissProt with the keyword(s) "transcription regulation" and "activator", "transcription regulation" and "repressor", or "nuclear protein" and "repressor", and the selected clones | |
|---|---|
| name of clone | name of representative sequence |
| HEMBA1000462 | HRIFA000432a |
| HEMBA1000671 | HRIFA000631a |
| HEMBA1000875 | HRIFA000814a |
| HEMBA1001184 | HRIFA001099a |
| HEMBA1001296 | HRIFA001200a |
| HEMBA1001886 | HRIFA001720a |
| HEMBA1002048 | HRIFA001866a |
| HEMBA1002985 | HRIFA002689a |
| HEMBA1003120 | HRIFA002805a |
| HEMBA1003497 | HRIFA003063a |
| HEMBA1004007 | HRIFA021040a |
| HEMBA1004067 | HRIFA003340a |
| HEMBA1004085 | HRIFA003357a |
| HEMBA1004785 | HRIFA020862a |
| HEMBA1004952 | HRIFA019532a |
| HEMBA1004971 | HRIFA003883a |
| HEMBA1005230 | HRIFA004006a |
| HEMBA1005246 | HRIFA019490a |
| HEMBA1005267 | HRIFA004034a |
| HEMBA1006276 | HRIFA021224a |
| HEMBA1006517 | HRIFA021445a |
| HEMBA1006544 | HRIFA021494a |
| HEMBA1006770 | HRIFA021651a |
| HEMBA1006912 | HRIFA022335a |
| HEMBA1007063 | HRIFA022348a |
| HEMBA1007226 | HRIFA022411a |
| HEMBB1000106 | HRIFA028262a |
| HEMBB1000309 | HRIFA029602a |
| HEMBB1000407 | HRIFA029649a |
| HEMBB1000542 | HRIFA029715a |
| HEMBB1001959 | HRIFA031336a |
| HEMBB1002039 | HRIFA031395a |
| HEMBB1002041 | HRIFA031397a |
| HEMBB1002051 | HRIFA026351a |
| HEMBB1002120 | HRIFA031438a |
| HEMBB1002302 | HRIFA009136a |
| HEMBB1002661 | HRIFA031869a |
| MAMMA1000528 | HRIFA026265a |
| MAMMA1000614 | HRIFA026316a |
| MAMMA1000810 | HRIFA026615a |
| MAMMA1001094 | HRIFA026789a |
| MAMMA1001532 | HRIFA027125a |
| MAMMA1001609 | HRIFA027173a |
| NT2RM1000407 | HRIFA004401a |
| NT2RM1000789 | HRIFA004663a |
| NT2RM2001558 | HRIFA028804a |
| NT2RM2001738 | HRIFA028867a |
| NT2RM2001767 | HRIFA028983a |
| NT2RM4000100 | HRIFA032389a |
| NT2RP1000239 | HRIFA005214a |
| NT2RP1000271 | HRIFA005240a |
| NT2RP1000465 | HRIFA005369a |
| NT2RP1000468 | HRIFA005372a |
| NT2RP1000679 | HRIFA005500a |
| NT2RP1000740 | HRIFA005540a |
| NT2RP2000092 | HRIFA006183a |
| NT2RP2000178 | HRIFA006250a |
| NT2RP2000240 | HRIFA006298a |
| NT2RP2000610 | HRIFA006566a |
| NT2RP2000712 | HRIFA006649a |
| NT2RP2000739 | HRIFA006667a |
| NT2RP2001223 | HRIFA007032a |
| NT2RP2001276 | HRIFA007068a |
| NT2RP2001388 | HRIFA007152a |
| NT2RP2001538 | HRIFA007262a |
| NT2RP2001662 | HRIFA007352a |
| NT2RP2001817 | HRIFA007463a |
| NT2RP2001921 | HRIFA007547a |
| NT2RP2003138 | HRIFA008426a |
| NT2RP2003302 | HRIFA008547a |
| NT2RP2003940 | HRIFA008981a |
| NT2RP2003950 | HRIFA008989a |
| NT2RP2004069 | HRIFA009071a |
| NT2RP2004108 | HRIFA009101a |
| NT2RP2005069 | HRIFA009762a |
| NT2RP2005391 | HRIFA009983a |
| NT2RP2005535 | HRIFA010085a |
| NT2RP2005666 | HRIFA010176a |
| NT2RP2005774 | HRIFA015063a |
| NT2RP2006092 | HRIFA010460a |
| NT2RP2006134 | HRIFA010490a |
| NT2RP3000148 | HRIFA022249a |
| NT2RP3000232 | HRIFA022564a |
| NT2RP3000378 | HRIFA022671a |
| NT2RP3000427 | HRIFA025033a |
| NT2RP3000652 | HRIFA022895a |
| NT2RP3000677 | HRIFA023007a |
| NT2RP3001271 | HRIFA023634a |
| NT2RP3001650 | HRIFA026923a |
| NT2RP3001976 | HRIFA026496a |
| NT2RP3002286 | HRIFA024185a |
| NT2RP3002353 | HRIFA024893a |
| NT2RP3002664 | HRIFA024305a |
| NT2RP3004025 | HRIFA025290a |
| NT2RP3004119 | HRIFA025695a |
| OVARC1000995 | HRIFA011512a |
| OVARC1001132 | HRIFA022707a |
| OVARC1001596 | HRIFA019437a |
| OVARC1001725 | HRIFA019958a |
| OVARC1001952 | HRIFA019466a |
| OVARC1002178 | HRIFA021007a |
| PLACE1000258 | HRIFA011820a |
| PLACE1000442 | HRIFA011947a |
| PLACE1000907 | HRIFA012278a |
| PLACE1003529 | HRIFA013980a |
| PLACE1003598 | HRIFA014024a |
| PLACE1004166 | HRIFA014396a |
| PLACE1004168 | HRIFA014397a |
| PLACE1004519 | HRIFA014620a |
| PLACE1005239 | HRIFA015122a |
| PLACE1005682 | HRIFA015423a |
| PLACE1006208 | HRIFA015756a |
| PLACE1006515 | HRIFA015947a |
| PLACE1007028 | HRIFA016255a |
| PLACE1007591 | HRIFA016599a |
| PLACE1008549 | HRIFA017190a |
| PLACE1009735 | HRIFA017921a |
| PLACE1011407 | HRIFA018931a |
| PLACE1011978 | HRIFA019262a |
| THYRO1000580 | HRIFA029208a |
| THYRO1000964 | HRIFA029398a |
| THYRO1001641 | HRIFA030472a |
| Y79AA1000030 | HRIFA025936a |
| 179AA1000750 | HRIFA028187a |
| Y79AA1001212 | HRIFA005296a |
| Y79AA1002058 | HRIFA032161a |
| Y79AA1002121 | HRIFA032186a |
| Y79AA1002129 | HRIFA011926a |
| Y79AA1002334 | HRIFA032257a |
| Y79AA1002378 | HRIFA032274a |

**Table 24**

| The representative sequences of the most homologous sequences in the SwissProt with the keyword(s) "disease mutation", or "syndrome", and the selected clones | |
|---|---|
| name of clone | name of representative sequence |
| HEMBA1000732 | HRIFA000683a |
| HEMBA1000835 | HRIFA000776a |
| HEMBA1004391 | HRIFA020693a |
| MAMMA1001623 | HRIFA027179c |
| NT2RM2000497 | HRIFA021781a |
| NT2RM2000632 | HRIFA022166a |
| NT2RP1000579 | HRIFA005438a |
| NT2RP2001903 | HRIFA007532a |
| NT2RP2005878 | HRIFA008186a |
| NT2RP3002411 | HRIFA005781a |
| NT2RP3002737 | HRIFA024132a |
| NT2RP4002187 | HRIFA030342a |
| PLACE1000033 | HRIFA011659a |
| PLACE1001500 | HRIFA012692a |
| PLACE1005815 | HRIFA015506a |
| PLACE1008657 | HRIFA017257a |
| PLACE1010713 | HRIFA024504a |

**Table 25**

| The clones selected by the keyword(s) of the top hit data in the SwissProt, and having the maximal score in the ATGpr1 0.5 or higher. | | |
|---|---|---|
| name of clone | name of sequence | maximal ATGpr1 score |
| BNGH41000020 | F-BNGH41000020 | 0.94 |
| BNGH41000087 | F-BNGH41000087 | 0.57 |
| BNGH41000091 | F-BNGH41000091 | 0.81 |
| HEMBA1000121 | F-HEMBA1000121 | 0.94 |
| HEMBA1000128 | F-HEMBA1000128 | 0.81 |
| HEMBA1000275 | F-HEMBA1000275 | 0.83 |
| HEMBA1000349 | F-HEMBA1000349 | 0.52 |
| HEMBA1000443 | F-HEMBA1000443 | 0.56 |
| HEMBA1000462 | F-HEMBA1000462 | 0.75 |
| HEMBA1000477 | F-HEMBA1000477 | 0.55 |
| HEMBA1000590 | F-HEMBA1000590 | 0.75 |
| HEMBA1000634 | F-HEMBA1000634 | 0.94 |
| HEMBA1000671 | F-HEMBA1000671 | 0.65 |
| HEMBA1000745 | F-HEMBA1000745 | 0.78 |
| HEMBA1000835 | F-HEMBA1000835 | 0.64 |
| HEMBA1000907 | F-HEMBA1000907 | 0.94 |
| HEMBA1000940 | F-HEMBA1000940 | 0.86 |
| HEMBA1001184 | F-HEMBA1001184 | 0.54 |
| HEMBA1001221 | F-HEMBA1001221 | 0.89 |
| HEMBA1001228 | F-HEMBA1001228 | 0.94 |
| HEMBA1001390 | F-HEMBA1001390 | 0.90 |
| HEMBA1001621 | F-HEMBA1001621 | 0.94 |
| HEMBA1001878 | F-HEMBA1001878 | 0.92 |
| HEMBA1002048 | F-HEMBA1002048 | 0.71 |
| HEMBA1002131 | F-HEMBA1002131 | 0.94 |
| HEMBA1002164 | F-HEMBA1002164 | 0.74 |
| HEMBA1002167 | F-HEMBA1002167 | 0.94 |
| HEMBA1002178 | F-HEMBA1002178 | 0.91 |
| HEMBA1002316 | F-HEMBA1002316 | 0.73 |
| HEMBA1002421 | F-HEMBA1002421 | 0.90 |
| HEMBA1002524 | F-HEMBA1002524 | 0.80 |
| HEMBA1002551 | F-HEMBA1002551 | 0.60 |
| HEMBA1002767 | F-HEMBA1002767 | 0.94 |
| HEMBA1002985 | F-HEMBA1002985 | 0.90 |
| HEMBA1002992 | F-HEMBA1002992 | 0.76 |
| HEMBA1003047 | F-HEMBA1003047 | 0.70 |
| HEMBA1003072 | F-HEMBA1003072 | 0.94 |
| HEMBA1003101 | F-HEMBA1003101 | 0.94 |
| HEMBA1003230 | F-HEMBA1003230 | 0.77 |
| HEMBA1003315 | F-HEMBA1003315 | 0.76 |
| HEMBA1003392 | F-HEMBA1003392 | 0.94 |
| HEMBA1003487 | F-HEMBA1003487 | 0.90 |
| HEMBA1003497 | F-HEMBA1003497 | 0.61 |
| HEMBA1003530 | F-HEMBA1003530 | 0.94 |
| HEMBA1003945 | F-HEMBA1003945 | 0.94 |
| HEMBA1004085 | F-HEMBA1004085 | 0.59 |
| HEMBA1004250 | F-HEMBA1004250 | 0.83 |
| HEMBA1004391 | F-HEMBA1004391 | 0.94 |
| HEMBA1004444 | F-HEMBA1004444 | 0.94 |
| HEMBA1004454 | F-HEMBA1004454 | 0.50 |
| HEMBA1004505 | F-HEMBA1004505 | 0.94 |
| HEMBA1004785 | F-HEMBA1004785 | 0.89 |
| HEMBA1004797 | F-HEMBA1004797 | 0.66 |
| HEMBA1004982 | F-HEMBA1004982 | 0.55 |
| HEMBA1005070 | F-HEMBA1005070 | 0.94 |
| HEMBA1005084 | F-HEMBA1005084 | 0.89 |
| HEMBA1005145 | F-HEMBA1005145 | 0.85 |
| HEMBA1005337 | F-HEMBA1005337 | 0.62 |
| HEMBA1005449 | F-HEMBA1005449 | 0.91 |
| HEMBA1005489 | F-HEMBA1005489 | 0.70 |
| HEMBA1005522 | F-HEMBA1005522 | 0.73 |
| HEMBA1005545 | F-HEMBA1005545 | 0.94 |
| HEMBA1005698 | F-HEMBA1005698 | 0.68 |
| HEMBA1005929 | F-HEMBA1005929 | 0.72 |
| HEMBA1005945 | F-HEMBA1005945 | 0.80 |
| HEMBA1006276 | F-HEMBA1006276 | 0.50 |
| HEMBA1006299 | F-HEMBA1006299 | 0.94 |
| HEMBA1006335 | F-HEMBA1006335 | 0.94 |
| HEMBA1006430 | F-HEMBA1006430 | 0.93 |
| HEMBA1006482 | F-HEMBA1006482 | 0.59 |
| HEMBA1006517 | F-HEMBA1006517 | 0.68 |
| HEMBA1006544 | F-HEMBA1006544 | 0.94 |
| HEMBA1006572 | F-HEMBA1006572 | 0.62 |
| HEMBA1006707 | F-HEMBA1006707 | 0.94 |
| HEMBA1006724 | F-HEMBA1006724 | 0.80 |
| HEMBA1006749 | F-HEMBA1006749 | 0.94 |
| HEMBA1006902 | F-HEMBA1006902 | 0.94 |
| HEMBA1006916 | F-HEMBA1006916 | 0.80 |
| HEMBA1007013 | F-HEMBA1007013 | 0.82 |
| HEMBA1007057 | F-HEMBA1007057 | 0.94 |
| HEMBA1007226 | F-HEMBA1007226 | 0.50 |
| HEMBA1007241 | F-HEMBA1007241 | 0.94 |
| HEMBB1000106 | F-HEMBB1000106 | 0.94 |
| HEMBB1000447 | F-HEMBB1000447 | 0.73 |
| HEMBB1000668 | F-HEMBB1000668 | 0.50 |
| HEMBB1000679 | F-HEMBB1000679 | 0.91 |
| HEMBB1000881 | F-HEMBB1000881 | 0.77 |
| HEMBB1001026 | F-HEMBB1001026 | 0.94 |
| HEMBB1001048 | F-HEMBB1001048 | 0.88 |
| HEMBB1001200 | F-HEMBB1001200 | 0.81 |
| HEMBB1001573 | F-HEMBB1001573 | 0.80 |
| HEMBB1001847 | F-HEMBB1001847 | 0.81 |
| HEMBB1001959 | F-HEMBB1001959 | 0.94 |
| HEMBB1002041 | F-HEMBB1002041 | 0.79 |
| HEMBB1002051 | F-HEMBB1002051 | 0.60 |
| HEMBB1002302 | F-HEMBB1002302 | 0.89 |
| HEMBB1002427 | F-HEMBB1002427 | 0.94 |
| HEMBB1002661 | F-HEMBB1002661 | 0.94 |
| MAMMA1000106 | F-MAMMA1000106 | 0.78 |
| MAMMA1000204 | F-MAMMA1000204 | 0.94 |
| MAMMA1000226 | F-MAMMA1000226 | 0.94 |
| MAMMA1000403 | F-MAMMA1000403 | 0.59 |
| MAMMA1000473 | F-MAMMA1000473 | 0.86 |
| MAMMA1000496 | F-MAMMA1000496 | 0.70 |
| MAMMA1000591 | F-MAMMA1000591 | 0.77 |
| MAMMA1000681 | F-MAMMA1000681 | 0.94 |
| MAMMA1000788 | F-MAMMA1000788 | 0.83 |
| MAMMA1000814 | F-MAMMA1000814 | 0.94 |
| MAMMA1000881 | F-MAMMA1000881 | 0.51 |
| MAMMA1001043 | F-MAMMA1001043 | 0.81 |
| MAMMA1001094 | F-MAMMA1001094 | 0.80 |
| MAMMA1001150 | F-MAMMA1001150 | 0.91 |
| MAMMA1001237 | F-MAMMA1001237 | 0.52 |
| MAMMA1001532 | F-MAMMA1001532 | 0.52 |
| MAMMA1001615 | F-MAMMA1001615 | 0.94 |
| MAMMA1001634 | F-MAMMA1001634 | 0.94 |
| MAMMA1001893 | F-MAMMA1001893 | 0.90 |
| MAMMA1001957 | F-MAMMA1001957 | 0.94 |
| MAMMA1002070 | F-MAMMA1002070 | 0.82 |
| MAMMA1002080 | F-MAMMA1002080 | 0.72 |
| MAMMA1002091 | F-MAMMA1002091 | 0.70 |
| MAMMA1002095 | F-MAMMA1002095 | 0.85 |
| MAMMA1002128 | F-MAMMA1002128 | 0.79 |
| MAMMA1002234 | F-MAMMA1002234 | 0.94 |
| MAMMA1002586 | F-MAMMA1002586 | 0.67 |
| MAMMA1002633 | F-MAMMA1002633 | 0.94 |
| MAMMA1003126 | F-MAMMA1003126 | 0.94 |
| NT2RM1000407 | F-NT2RM1000407 | 0.94 |
| NT2RM1000462 | F-NT2RM1000462 | 0.94 |
| NT2RM1000542 | F-NT2RM1000542 | 0.94 |
| NT2RM1000580 | F-NT2RM1000580 | 0.94 |
| NT2RM1000789 | F-NT2RM1000789 | 0.82 |
| NT2RM1000855 | F-NT2RM1000855 | 0.94 |
| NT2RM1000858 | F-NT2RM1000858 | 0.50 |
| NT2RM1000899 | F-NT2RM1000899 | 0.78 |
| NT2RM2000410 | F-NT2RM2000410 | 0.82 |
| NT2RM2000423 | F-NT2RM2000423 | 0.66 |
| NT2RM2000565 | F-NT2RM2000565 | 0.56 |
| NT2RM2000582 | F-NT2RM2000582 | 0.78 |
| NT2RM2000589 | F-NT2RM2000589 | 0.86 |
| NT2RM2000622 | F-NT2RM2000622 | 0.94 |
| NT2RM2000632 | F-NT2RM2000632 | 0.73 |
| NT2RM2000773 | F-NT2RM2000773 | 0.64 |
| NT2RM2001126 | F-NT2RM2001126 | 0.87 |
| NT2RM2001558 | F-NT2RM2001558 | 0.94 |
| NT2RM2001626 | F-NT2RM2001626 | 0.68 |
| NT2RM2001738 | F-NT2RM2001738 | 0.94 |
| NT2RM2001767 | F-NT2RM2001767 | 0.82 |
| NT2RM2001818 | F-NT2RM2001818 | 0.70 |
| NT2RM2001902 | F-NT2RM2001902 | 0.69 |
| NT2RM2001939 | F-NT2RM2001939 | 0.55 |
| NT2RM2001941 | F-NT2RM2001941 | 0.94 |
| NT2RM4000100 | F-NT2RM4000100 | 0.74 |
| NT2RM4000198 | F-NT2RM4000198 | 0.85 |
| NT2RM4000284 | F-NT2RM4000284 | 0.89 |
| NT2RM4000295 | F-NT2RM4000295 | 0.74 |
| NT2RM4000417 | F-NT2RM4000417 | 0.82 |
| NT2RM4000444 | F-NT2RM4000444 | 0.94 |
| NT2RM4000587 | F-NT2RM4000587 | 0.94 |
| NT2RM4000648 | F-NT2RM4000648 | 0.88 |
| NT2RM4000965 | F-NT2RM4000965 | 0.60 |
| NT2RM4001377 | F-NT2RM4001377 | 0.82 |
| NT2RM4001735 | F-NT2RM4001735 | 0.94 |
| NT2RM4002352 | F-NT2RM4002352 | 0.83 |
| NT2RP1000002 | F-NT2RP1000002 | 0.76 |
| NT2RP1000050 | F-NT2RP1000050 | 0.54 |
| NT2RP1000181 | F-NT2RP1000181 | 0.73 |
| NT2RP1000239 | F-NT2RP1000239 | 0.94 |
| NT2RP1000261 | F-NT2RP1000261 | 0.57 |
| NT2RP1000300 | F-NT2RP1000300 | 0.94 |
| NT2RP1000465 | F-NT2RP1000465 | 0.94 |
| NT2RP1000468 | F-NT2RP1000468 | 0.94 |
| NT2RP1000551 | F-NT2RP1000551 | 0.94 |
| NT2RP1000579 | F-NT2RP1000579 | 0.94 |
| NT2RP1000613 | F-NT2RP1000613 | 0.90 |
| NT2RP1000679 | F-NT2RP1000679 | 0.68 |
| NT2RP1000740 | F-NT2RP1000740 | 0.80 |
| NT2RP1000981 | F-NT2RP1000981 | 0.94 |
| NT2RP1001004 | F-NT2RP1001004 | 0.74 |
| NT2RP1001020 | F-NT2RP1001020 | 0.70 |
| NT2RP1001031 | F-NT2RP1001031 | 0.59 |
| NT2RP2000092 | F-NT2RP2000092 | 0.88 |
| NT2RP2000394 | F-NT2RP2000394 | 0.94 |
| NT2RP2000447 | F-NT2RP2000447 | 0.50 |
| NT2RP2000514 | F-NT2RP2000514 | 0.82 |
| NT2RP2000533 | F-NT2RP2000533 | 0.94 |
| NT2RP2000610 | F-NT2RP2000610 | 0.94 |
| NT2RP2000616 | F-NT2RP2000616 | 0.80 |
| NT2RP2000649 | F-NT2RP2000649 | 0.53 |
| NT2RP2000663 | F-NT2RP2000663 | 0.88 |
| NT2RP2000694 | F-NT2RP2000694 | 0.74 |
| NT2RP2000818 | F-NT2RP2000818 | 0.54 |
| NT2RP2000903 | F-NT2RP2000903 | 0.59 |
| NT2RP2001200 | F-NT2RP2001200 | 0.88 |
| NT2RP2001223 | F-NT2RP2001223 | 0.92 |
| NT2RP2001276 | F-NT2RP2001276 | 0.59 |
| NT2RP2001480 | F-NT2RP2001480 | 0.85 |
| NT2RP2001495 | F-NT2RP2001495 | 0.78 |
| NT2RP2001514 | F-NT2RP2001514 | 0.78 |
| NT2RP2001529 | F-NT2RP2001529 | 0.94 |
| NT2RP2001538 | F-NT2RP2001538 | 0.89 |
| NT2RP2001662 | F-NT2RP2001662 | 0.94 |
| NT2RP2001755 | F-NT2RP2001755 | 0.94 |
| NT2RP2001769 | F-NT2RP2001769 | 0.66 |
| NT2RP2001878 | F-NT2RP2001878 | 0.68 |
| NT2RP2001921 | F-NT2RP2001921 | 0.61 |
| NT2RP2001948 | F-NT2RP2001948 | 0.89 |
| NT2RP2001956 | F-NT2RP2001956 | 0.74 |
| NT2RP2002063 | F-NT2RP2002063 | 0.94 |
| NT2RP2002188 | F-NT2RP2002188 | 0.78 |
| NT2RP2002232 | F-NT2RP2002232 | 0.90 |
| NT2RP2002304 | F-NT2RP2002304 | 0.94 |
| NT2RP2002409 | F-NT2RP2002409 | 0.94 |
| NT2RP2002527 | F-NT2RP2002527 | 0.58 |
| NT2RP2002533 | F-NT2RP2002533 | 0.87 |
| NT2RP2002564 | F-NT2RP2002564 | 0.94 |
| NT2RP2002942 | F-NT2RP2002942 | 0.66 |
| NT2RP2002976 | F-NT2RP2002976 | 0.94 |
| NT2RP2003042 | F-NT2RP2003042 | 0.93 |
| NT2RP2003179 | F-NT2RP2003179 | 0.94 |
| NT2RP2003210 | F-NT2RP2003210 | 0.61 |
| NT2RP2003302 | F-NT2RP2003302 | 0.79 |
| NT2RP2003369 | F-NT2RP2003369 | 0.93 |
| NT2RP2003390 | F-NT2RP2003390 | 0.79 |
| NT2RP2003469 | F-NT2RP2003469 | 0.90 |
| NT2RP2003545 | F-NT2RP2003545 | 0.55 |
| NT2RP2003593 | F-NT2RP2003593 | 0.94 |
| NT2RP2003655 | F-NT2RP2003655 | 0.83 |
| NT2RP2003664 | F-NT2RP2003664 | 0.89 |
| NT2RP2004069 | F-NT2RP2004069 | 0.76 |
| NT2RP2004108 | F-NT2RP2004108 | 0.91 |
| NT2RP2004141 | F-NT2RP2004141 | 0.53 |
| NT2RP2004447 | F-NT2RP2004447 | 0.93 |
| NT2RP2004606 | F-NT2RP2004606 | 0.94 |
| NT2RP2004648 | F-NT2RP2004648 | 0.94 |
| NT2RP2004670 | F-NT2RP2004670 | 0.94 |
| NT2RP2004794 | F-NT2RP2004794 | 0.65 |
| NT2RP2004847 | F-NT2RP2004847 | 0.94 |
| NT2RP2005069 | F-NT2RP2005069 | 0.89 |
| NT2RP2005163 | F-NT2RP2005163 | 0.79 |
| NT2RP2005181 | F-NT2RP2005181 | 0.87 |
| NT2RP2005247 | F-NT2RP2005247 | 0.77 |
| NT2RP2005425 | F-NT2RP2005425 | 0.77 |
| NT2RP2005535 | F-NT2RP2005535 | 0.51 |
| NT2RP2005597 | F-NT2RP2005597 | 0.74 |
| NT2RP2005632 | F-NT2RP2005632 | 0.87 |
| NT2RP2005666 | F-NT2RP2005666 | 0.77 |
| NT2RP2005774 | F-NT2RP2005774 | 0.87 |
| NT2RP2005878 | F-NT2RP2005878 | 0.70 |
| NT2RP2005883 | F-NT2RP2005883 | 0.94 |
| NT2RP2005941 | F-NT2RP2005941 | 0.81 |
| NT2RP2005994 | F-NT2RP2005994 | 0.62 |
| NT2RP2006004 | F-NT2RP2006004 | 0.61 |
| NT2RP2006042 | F-NT2RP2006042 | 0.69 |
| NT2RP2006099 | F-NT2RP2006099 | 0.65 |
| NT2RP2006512 | F-NT2RP2006512 | 0.94 |
| NT2RP3000011 | F-NT2RP3000011 | 0.93 |
| NT2RP3000022 | F-NT2RP3000022 | 0.55 |
| NT2RP3000059 | F-NT2RP3000059 | 0.74 |
| NT2RP3000063 | F-NT2RP3000063 | 0.78 |
| NT2RP3000171 | F-NT2RP3000171 | 0.72 |
| NT2RP3000172 | F-NT2RP3000172 | 0.93 |
| NT2RP3000201 | F-NT2RP3000201 | 0.50 |
| NT2RP3000232 | F-NT2RP3000232 | 0.61 |
| NT2RP3000304 | F-NT2RP3000304 | 0.94 |
| NT2RP3000378 | F-NT2RP3000378 | 0.56 |
| NT2RP3000436 | F-NT2RP3000436 | 0.65 |
| NT2RP3000444 | F-NT2RP3000444 | 0.94 |
| NT2RP3000616 | F-NT2RP3000616 | 0.77 |
| NT2RP3000645 | F-NT2RP3000645 | 0.91 |
| NT2RP3000676 | F-NT2RP3000676 | 0.94 |
| NT2RP3000721 | F-NT2RP3000721 | 0.82 |
| NT2RP3000838 | F-NT2RP3000838 | 0.94 |
| NT2RP3000871 | F-NT2RP3000871 | 0.94 |
| NT2RP3000907 | F-NT2RP3000907 | 0.59 |
| NT2RP3000921 | F-NT2RP3000921 | 0.64 |
| NT2RP3001061 | F-NT2RP3001061 | 0.72 |
| NT2RP3001159 | F-NT2RP3001159 | 0.94 |
| NT2RP3001170 | F-NT2RP3001170 | 0.70 |
| NT2RP3001195 | F-NT2RP3001195 | 0.94 |
| NT2RP3001240 | F-NT2RP3001240 | 0.83 |
| NT2RP3001322 | F-NT2RP3001322 | 0.62 |
| NT2RP3001388 | F-NT2RP3001388 | 0.94 |
| NT2RP3001560 | F-N12RP3001560 | 0.94 |
| NT2RP3001592 | F-NT2RP3001592 | 0.74 |
| NT2RP3001650 | F-NT2RP3001650 | 0.73 |
| NT2RP3001738 | F-N12RP3001738 | 0.94 |
| NT2RP3002015 | F-NT2RP3002015 | 0.93 |
| NT2RP3002160 | F-NT2RP3002160 | 0.65 |
| NT2RP3002286 | F-NT2RP3002286 | 0.82 |
| NT2RP3002311 | F-NT2RP3002311 | 0.94 |
| NT2RP3002324 | F-NT2RP3002324 | 0.92 |
| NT2RP3002342 | F-NT2RP3002342 | 0.94 |
| NT2RP3002353 | F-NT2RP3002353 | 0.76 |
| NT2RP3002411 | F-NT2RP3002411 | 0.74 |
| NT2RP3002448 | F-NT2RP3002448 | 0.87 |
| NT2RP3002571 | F-NT2RP3002571 | 0.61 |
| NT2RP3002664 | F-NT2RP3002664 | 0.82 |
| NT2RP3002738 | F-NT2RP3002738 | 0.81 |
| NT2RP3002790 | F-NT2RP3002790 | 0.94 |
| NT2RP3002836 | F-NT2RP3002836 | 0.72 |
| NT2RP3002887 | F-NT2RP3002887 | 0.94 |
| NT2RP3002900 | F-NT2RP3002900 | 0.88 |
| NT2RP3002958 | F-NT2RP3002958 | 0.91 |
| NT2RP3002983 | F-NT2RP3002983 | 0.92 |
| NT2RP3003000 | F-NT2RP3003000 | 0.80 |
| NT2RP3003076 | F-NT2RP3003076 | 0.65 |
| NT2RP3003354 | F-NT2RP3003354 | 0.66 |
| NT2RP3003448 | F-NT2RP3003448 | 0.61 |
| NT2RP3003473 | F-NT2RP3003473 | 0.94 |
| NT2RP3003527 | F-NT2RP3003527 | 0.94 |
| NT2RP3003532 | F-NT2RP3003532 | 0.93 |
| NT2RP3003614 | F-NT2RP3003614 | 0.81 |
| NT2RP3003729 | F-NT2RP3003729 | 0.90 |
| NT2RP3003849 | F-NT2RP3003849 | 0.93 |
| NT2RP3003939 | F-NT2RP3003939 | 0.67 |
| NT2RP3004025 | F-NT2RP3004025 | 0.83 |
| NT2RP3004067 | F-NT2RP3004067 | 0.74 |
| NT2RP3004075 | F-NT2RP3004075 | 0.77 |
| NT2RP3004090 | F-NT2RP3004090 | 0.94 |
| NT2RP3004119 | F-NT2RP3004119 | 0.57 |
| NT2RP3004130 | F-NT2RP3004130 | 0.66 |
| NT2RP3004133 | F-NT2RP3004133 | 0.94 |
| NT2RP3004202 | F-NT2RP3004202 | 0.53 |
| NT2RP3004294 | F-NT2RP3004294 | 0.71 |
| NT2RP3004309 | F-NT2RP3004309 | 0.92 |
| NT2RP3004345 | F-NT2RP3004345 | 0.73 |
| NT2RP3004406 | F-NT2RP3004406 | 0.62 |
| NT2RP3004481 | F-NT2RP3004481 | 0.76 |
| NT2RP3004552 | F-NT2RP3004552 | 0.78 |
| NT2RP3004557 | F-NT2RP3004557 | 0.78 |
| NT2RP3004625 | F-NT2RP3004625 | 0.87 |
| NT2RP3004640 | F-NT2RP3004640 | 0.82 |
| NT2RP3004647 | F-NT2RP3004647 | 0.94 |
| NT2RP4000108 | F-NT2RP4000108 | 0.94 |
| NT2RP4000634 | F-NT2RP4000634 | 0.69 |
| NT2RP4000962 | F-NT2RP4000962 | 0.67 |
| NT2RP4001009 | F-NT2RP4001009 | 0.56 |
| NT2RP4001467 | F-NT2RP4001467 | 0.94 |
| NT2RP4001877 | F-NT2RP4001877 | 0.94 |
| NT2RP4001879 | F-NT2RP4001879 | 0.82 |
| NT2RP4002187 | F-NT2RP4002187 | 0.7 |
| NT2RP4002451 | F-NT2RP4002451 | 0.53 |
| NT2RP4002750 | F-NT2RP4002750 | 0.67 |
| OVARC1000003 | F-OVARC1000003 | 0.62 |
| OVARC1000105 | F-OVARC1000105 | 0.62 |
| OVARC1000137 | F-OVARC1000137 | 0.72 |
| OVARC1000255 | F-OVARC1000255 | 0.78 |
| OVARC1000307 | F-OVARC1000307 | 0.94 |
| OVARC1000313 | F-OVARC1000313 | 0.94 |
| OVARC1000331 | F-OVARC1000331 | 0.57 |
| OVARC1000410 | F-OVARC1000410 | 0.79 |
| OVARC1000467 | F-OVARC1000467 | 0.83 |
| OVARC1000529 | F-OVARC1000529 | 0.94 |
| OVARC1000553 | F-OVARC1000553 | 0.94 |
| OVARC1000873 | F-OVARC1000873 | 0.88 |
| OVARC1000916 | F-OVARC1000916 | 0.94 |
| OVARC1000956 | F-OVARC1000956 | 0.92 |
| OVARC1001030 | F-OVARC1001030 | 0.94 |
| OVARC1001049 | F-OVARC1001049 | 0.94 |
| OVARC1001086 | F-OVARC1001086 | 0.73 |
| OVARC1001132 | F-OVARC1001132 | 0.94 |
| OVARC1001163 | F-OVARC1001163 | 0.75 |
| OVARC1001222 | F-OVARC1001222 | 0.67 |
| OVARC1001336 | F-OVARC1001336 | 0.92 |
| OVARC1001338 | F-OVARC1001338 | 0.89 |
| OVARC1001570 | F-OVARC1001570 | 0.94 |
| OVARC1001607 | F-OVARC1001607 | 0.86 |
| OVARC1001725 | F-OVARC1001725 | 0.81 |
| OVARC1001952 | F-OVARC1001952 | 0.66 |
| OVARC1001991 | F-OVARC1001991 | 0.94 |
| OVARC1002058 | F-OVARC1002058 | 0.79 |
| PLACE1000442 | F-PLACE1000442 | 0.93 |
| PLACE1000740 | F-PLACE1000740 | 0.57 |
| PLACE1001016 | F-PLACE1001016 | 0.92 |
| PLACE1001114 | F-PLACE1001114 | 0.94 |
| PLACE1001123 | F-PLACE1001123 | 0.89 |
| PLACE1001231 | F-PLACE1001231 | 0.81 |
| PLACE1001340 | F-PLACE1001340 | 0.77 |
| PLACE1001401 | F-PLACE1001401 | 0.87 |
| PLACE1001407 | F-PLACE1001407 | 0.94 |
| PLACE1001464 | F-PLACE1001464 | 0.91 |
| PLACE1001500 | F-PLACE1001500 | 0.77 |
| PLACE1001516 | F-PLACE1001516 | 0.89 |
| PLACE1001564 | F-PLACE1001564 | 0.52 |
| PLACE1001655 | F-PLACE1001655 | 0.56 |
| PLACE1001795 | F-PLACE1001795 | 0.91 |
| PLACE1001836 | F-PLACE1001836 | 0.81 |
| PLACE1001918 | F-PLACE1001918 | 0.76 |
| PLACE1001949 | F-PLACE1001949 | 0.94 |
| PLACE1002080 | F-PLACE1002080 | 0.76 |
| PLACE1002095 | F-PLACE1002095 | 0.61 |
| PLACE1002153 | F-PLACE1002153 | 0.94 |
| PLACE1002329 | F-PLACE1002329 | 0.94 |
| PLACE1002355 | F-PLACE1002355 | 0.57 |
| PLACE1002374 | F-PLACE1002374 | 0.92 |
| PLACE1002547 | F-PLACE1002547 | 0.87 |
| PLACE1002726 | F-PLACE1002726 | 0.83 |
| PLACE1002905 | F-PLACE1002905 | 0.94 |
| PLACE1002911 | F-PLACE1002911 | 0.73 |
| PLACE1003135 | F-PLACE1003135 | 0.69 |
| PLACE1003163 | F-PLACE1003163 | 0.61 |
| PLACE1003428 | F-PLACE1003428 | 0.61 |
| PLACE1003438 | F-PLACE1003438 | 0.93 |
| PLACE1003460 | F-PLACE1003460 | 0.94 |
| PLACE1003573 | F-PLACE1003573 | 0.78 |
| PLACE1003598 | F-PLACE1003598 | 0.57 |
| PLACE1003644 | F-PLACE1003644 | 0.93 |
| PLACE1003737 | F-PLACE1003737 | 0.88 |
| PLACE1003772 | F-PLACE1003772 | 0.80 |
| PLACE1003852 | F-PLACE1003852 | 0.82 |
| PLACE1004078 | F-PLACE1004078 | 0.94 |
| PLACE1004166 | F-PLACE1004166 | 0.92 |
| PLACE1004168 | F-PLACE1004168 | 0.58 |
| PLACE1004279 | F-PLACE1004279 | 0.64 |
| PLACE1004441 | F-PLACE1004441 | 0.90 |
| PLACE1004450 | F-PLACE1004450 | 0.85 |
| PLACE1004482 | F-PLACE1004482 | 0.57 |
| PLACE1004492 | F-PLACE1004492 | 0.85 |
| PLACE1004519 | F-PLACE1004519 | 0.74 |
| PLACE1004520 | F-PLACE1004520 | 0.94 |
| PLACE1004630 | F-PLACE1004630 | 0.76 |
| PLACE1004648 | F-PLACE1004648 | 0.59 |
| PLACE1004816 | F-PLACE1004816 | 0.94 |
| PLACE1004887 | F-PLACE1004887 | 0.94 |
| PLACE1005003 | F-PLACE1005003 | 0.80 |
| PLACE1005031 | F-PLACE1005031 | 0.88 |
| PLACE1005239 | F-PLACE1005239 | 0.94 |
| PLACE1005383 | F-PLACE1005383 | 0.52 |
| PLACE1005426 | F-PLACE1005426 | 0.52 |
| PLACE1005519 | F-PLACE1005519 | 0.69 |
| PLACE1005544 | F-PLACE1005544 | 0.59 |
| PLACE1005569 | F-PLACE1005569 | 0.68 |
| PLACE1005682 | F-PLACE1005682 | 0.64 |
| PLACE1005736 | F-PLACE1005736 | 0.67 |
| PLACE1005815 | F-PLACE1005815 | 0.94 |
| PLACE1005878 | F-PLACE1005878 | 0.92 |
| PLACE1005927 | F-PLACE1005927 | 0.94 |
| PLACE1006073 | F-FLACE1006073 | 0.94 |
| PLACE1006208 | F-PLACE1006208 | 0.75 |
| PLACE1006277 | F-PLACE1006277 | 0.65 |
| PLACE1006290 | F-PLACE1006290 | 0.94 |
| PLACE1006443 | F-PLACE1006443 | 0.89 |
| PLACE1006716 | F-PLACE1006716 | 0.63 |
| PLACE1006959 | F-PLACE1006959 | 0.89 |
| PLACE1007028 | F-PLACE1007028 | 0.94 |
| PLACE1007081 | F-PLACE1007081 | 0.77 |
| PLACE1007096 | F-PLACE1007096 | 0.84 |
| PLACE1007702 | F-PLACE1007702 | 0.51 |
| PLACE1006282 | F-PLACE1008282 | 0.70 |
| PLACE1008297 | F-PLACE1008297 | 0.64 |
| PLACE1008469 | F-PLACE1008469 | 0.94 |
| PLACE1008549 | F-PLACE1008549 | 0.52 |
| PLACE1008657 | F-PLACE1008657 | 0.94 |
| PLACE1008716 | F-PLACE1008716 | 0.79 |
| PLACE1008744 | F-PLACE1008744 | 0.94 |
| PLACE1008984 | F-PLACE1008984 | 0.74 |
| PLACE1009279 | F-PLACE1009279 | 0.60 |
| PLACE1009527 | F-PLACE1009527 | 0.87 |
| PLACE1009546 | F-PLACE1009546 | 0.80 |
| PLACE1009600 | F-PLACE1009600 | 0.76 |
| PLACE1010011 | F-PLACE1010011 | 0.94 |
| PLACE1010078 | F-PLACE1010078 | 0.86 |
| PLACE1010081 | F-PLACE1010081 | 0.92 |
| PLACE1010251 | F-PLACE1010251 | 0.62 |
| PLACE1010445 | F-PLACE1010445 | 0.94 |
| PLACE1010713 | F-PLACE1010713 | 0.62 |
| PLACE1010827 | F-PLACE1010827 | 0.88 |
| PLACE1010968 | F-PLACE1010968 | 0.51 |
| PLACE1011045 | F-PLACE1011045 | 0.64 |
| PLACE1011116 | F-PLACE1011116 | 0.69 |
| PLACE1011181 | F-PLACE1011181 | 0.78 |
| PLACE1011236 | F-PLACE1011236 | 0.66 |
| PLACE1011364 | F-PLACE1011364 | 0.94 |
| PLACE1011407 | F-PLACE1011407 | 0.64 |
| PLACE1011516 | F-PLACE1011516 | 0.83 |
| PLACE1011708 | F-PLACE1011708 | 0.94 |
| PLACE1011824 | F-PLACE1011824 | 0.94 |
| PLACE3000181 | F-PLACE3000181 | 0.74 |
| SKNMC1000014 | F-SKNMC1000014 | 0.76 |
| SKNMC1000082 | F-SKNMC1000082 | 0.74 |
| THYRO1000196 | F-THYRO1000196 | 0.71 |
| THYRO1000400 | F-THYRO1000400 | 0.73 |
| THYRO1000584 | F-THYRO1000584 | 0.73 |
| THYRO1000678 | F-THYRO1000678 | 0.86 |
| THYRO1000776 | F-THYRO1000776 | 0.91 |
| THYRO1000795 | F-THYRO1000795 | 0.92 |
| THYRO1000846 | F-THYRO1000846 | 0.78 |
| THYRO1000866 | F-THYRO1000866 | 0.56 |
| THYRO1000956 | F-THYRO1000956 | 0.94 |
| THYRO1000964 | F-THYRO1000964 | 0.80 |
| THYRO1001063 | F-THYRO1001063 | 0.87 |
| THYRO1001071 | F-THYRO1001071 | 0.94 |
| THYRO1001102 | F-THYRO1001102 | 0.90 |
| THYRO1001113 | F-THYRO1001113 | 0.74 |
| THYRO1001128 | F-THYRO1001128 | 0.79 |
| THYRO1001205 | F-THYRO1001205 | 0.94 |
| THYRO1001242 | F-THYRO1001242 | 0.94 |
| THYRO1001266 | F-THYRO1001266 | 0.94 |
| THYRO1001456 | F-THYRO1001456 | 0.69 |
| THYRO1001457 | F-THYRO1001457 | 0.88 |
| THYRO1001471 | F-THYRO1001471 | 0.91 |
| THYRO1001478 | F-THYRO1001478 | 0.89 |
| THYRO1001529 | F-THYRO1001529 | 0.55 |
| THYRO1001593 | F-THYRO1001593 | 0.94 |
| THYRO1001608 | F-THYRO1001608 | 0.94 |
| THYRO1001641 | F-THYRO1001641 | 0.94 |
| THYRO1001700 | F-THYRO1001700 | 0.76 |
| THYRO1001702 | F-THYRO1001702 | 0.80 |
| THYRO1001770 | F-THYRO1001770 | 0.73 |
| THYRO1001803 | F-THYRO1001803 | 0.94 |
| Y79AA1000030 | F-Y79AA1000030 | 0.88 |
| Y79AA1000270 | F-Y79AA1000270 | 0.63 |
| Y79AA1000426 | F-Y79AA1000426 | 0.92 |
| Y79AA1000750 | F-Y79AA1000750 | 0.94 |
| Y79AA1000777 | F-Y79AA1000777 | 0.94 |
| Y79AA1000876 | F-Y79AA1000876 | 0.94 |
| Y79AA1000888 | F-Y79AA1000888 | 0.85 |
| Y79AA1000967 | F-Y79AA1000967 | 0.92 |
| Y79AA1001090 | F-Y79AA1001090 | 0.74 |
| Y79AA1001212 | F-Y79AA1001212 | 0.93 |
| Y79AA1001426 | F-Y79AA1001426 | 0.82 |
| Y79AA1001523 | F-Y79AA1001523 | 0.94 |
| Y79AA1001727 | F-Y79AA1001727 | 0.94 |
| Y79AA1001787 | F-Y79AA1001787 | 0.75 |
| Y79AA1001799 | F-Y79AA1001799 | 0.60 |
| Y79AA1001803 | F-Y79AA1001803 | 0.68 |
| Y79AA1002058 | F-Y79AA1002058 | 0.89 |
| Y79AA1002121 | F-Y79AA1002121 | 0.66 |
| Y79AA1002213 | F-Y79AA1002213 | 0.86 |
| Y79AA1002378 | F-Y79AA1002378 | 0.82 |
| Y79AA1002381 | F-Y79AA1002381 | 0.89 |

**Table 26**

| The clones selected by the keyword(s) of the top hit data in the SwissProt, and having the maximal score in the ATGpr1 0.3 or higher and less than 0.5. | | |
|---|---|---|
| name of clone | name of sequence | maximal ATGpr1 score |
| HEMBA1000732 | F-HEMBA1000732 | 0.32 |
| HEMBA1001886 | F-HEMBA1001886 | 0.46 |
| HEMBA1002163 | F-HEMBA1002163 | 0.44 |
| HEMBA1002195 | F-HEMBA1002195 | 0.41 |
| HEMBA1003120 | F-HEMBA1003120 | 0.44 |
| HEMBA1004007 | F-HEMBA1004007 | 0.31 |
| HEMBA1004067 | F-HEMBA1004067 | 0.49 |
| HEMBA1005267 | F-HEMBA1005267 | 0.37 |
| HEMBA1006770 | F-HEMBA1006770 | 0.40 |
| HEMBB1000407 | F-HEMBB1000407 | 0.41 |
| HEMBB1000542 | F-HEMBB1000542 | 0.47 |
| HEMBB1002120 | F-HEMBB1002120 | 0.33 |
| MAMMA1000810 | F-MAMMA1000810 | 0.43 |
| MAMMA1001609 | F-MAMMA1001609 | 0.32 |
| MAMMA1001978 | F-MAMMA1001978 | 0.32 |
| MAMMA1002142 | F-MAMMA1002142 | 0.34 |
| MAMMA1002165 | F-MAMMA1002165 | 0.34 |
| NT2RM2001792 | F-NT2RM2001792 | 0.37 |
| NT2RM4001843 | F-NT2RM4001843 | 0.44 |
| NT2RP1000271 | F-NT2RP1000271 | 0.41 |
| NT2RP2000739 | F-NT2RP2000739 | 0.39 |
| NT2RP2001388 | F-NT2RP2001388 | 0.47 |
| NT2RP2001562 | F-NT2RP2001562 | 0.41 |
| NT2RP2001903 | F-NT2RP2001903 | 0.46 |
| NT2RP2003138 | F-NT2RP2003138 | 0.41 |
| NT2RP2003931 | F-NT2RP2003931 | 0.44 |
| NT2RP2004205 | F-NT2RP2004205 | 0.37 |
| NT2RP2005378 | F-NT2RP2005378 | 0.43 |
| NT2RP2005541 | F-NT2RP2005541 | 0.31 |
| NT2RP2006092 | F-NT2RP2006092 | 0.47 |
| NT2RP2006269 | F-NT2RP2006269 | 0.41 |
| NT2RP3000148 | F-NT2RP3000148 | 0.44 |
| NT2RP3000427 | F-NT2RP3000427 | 0.38 |
| NT2RP3000820 | F-NT2RP3000820 | 0.44 |
| NT2RP3001754 | F-NT2RP3001754 | 0.30 |
| NT2RP3001976 | F-NT2RP3001976 | 0.46 |
| NT2RP3002409 | F-NT2RP3002409 | 0.31 |
| NT2RP3002737 | F-NT2RP3002737 | 0.33 |
| NT2RP3003874 | F-NT2RP3003874 | 0.34 |
| OVARC1000275 | F-0VARC1000275 | 0.44 |
| OVARC1000995 | F-OVARC1000995 | 0.34 |
| OVARC1001569 | F-OVARC1001569 | 0.30 |
| OVARC1001596 | F-OVARC1001596 | 0.36 |
| PLACE1000907 | F-PLACE1000907 | 0.34 |
| PLACE1002967 | F-PLACE1002967 | 0.38 |
| PLACE1003529 | F-PLACE1003529 | 0.31 |
| PLACE1006071 | F-PLACE1006071 | 0.38 |
| PLACE1006515 | F-PLACE1006515 | 0.48 |
| PLACE1007881 | F-PLACE1007881 | 0.43 |
| PLACE1008359 | F-PLACE1008359 | 0.47 |
| PLACE1008985 | F-PLACE1008985 | 0.48 |
| PLACE1009735 | F-PLACE1009735 | 0.37 |
| PLACE1010784 | F-PLACE1010784 | 0.44 |
| PLACE1011978 | F-PLACE1011978 | 0.39 |
| THYR01000061 | F-THYR01000061 | 0.38 |
| THYR01000580 | F-THYR01000580 | 0.48 |
| Y79AA1000127 | F-Y79AA1000127 | 0.32 |
| Y79AA1001272 | F-Y79AA1001272 | 0.47 |
| Y79AA1002129 | F-Y79AA1002129 | 0.36 |

**Table 27**

| The clones selected by the keyword(s) of the top hit data in the SwissProt, and having the maximal score in the ATGpr1 0 or higher and less than 0.3. | | |
|---|---|---|
| name of clone | name of sequence | maximal ATGpr1 score |
| HEMBA1000006 | F-HEMBA1000006 | 0.14 |
| HEMBA1000875 | F-HEMBA1000875 | 0.12 |
| HEMBA1001296 | F-HEMBA1001296 | 0.08 |
| HEMBA1001563 | F-HEMBA1001563 | 0.17 |
| HEMBA1002227 | F-HEMBA1002227 | 0.05 |
| HEMBA1004952 | F-HFMBA1004952 | 0.05 |
| HEMBA1004971 | F-HEMBA1004971 | 0.24 |
| HEMBA1005230 | F-HEMBA1005230 | 0.14 |
| HEMBA1005246 | F-HEMBA1005246 | 0.17 |
| HEMBA1005913 | F-HEMBA1005913 | 0.12 |
| HEMBA1006912 | F-HEMBA1006912 | 0.11 |
| HEMBA1007063 | F-HEMBA1007063 | 0.14 |
| HEMBA1007291 | F-HEMBA1007291 | 0.14 |
| HEMBA1007332 | F-HEMBA1007332 | 0.23 |
| HEMBB1000309 | F-HEMBB1000309 | 0.15 |
| HEMBB1000567 | F-HEMBB1000567 | 0.15 |
| HEMBB1002039 | F-HEMBB1002039 | 0.15 |
| MAMMA1000528 | F-MAMMA1000528 | 0.11 |
| MAMMA1000614 | F-MAMMA1000614 | 0.23 |
| MAMMA1000706 | F-MAMMA1000706 | 0.26 |
| MAMMA1001066 | F-MAMMA1001066 | 0.28 |
| MAMMA1001418 | F-MAMMA1001418 | 0.12 |
| MAMMA1001623 | F-MAMMA1001623 | 0.23 |
| NT2RM2000497 | F-NT2RM2000497 | 0.17 |
| NT2RM4000326 | F-NT2RM4000326 | 0.23 |
| NT2RM4000593 | F-NT2RM4000593 | 0.16 |
| NT2RM4000761 | F-NT2RM4000761 | 0.13 |
| NT2RP1000325 | F-NT2RP1000325 | 0.29 |
| NT2RP2000178 | F-NT2RP2000178 | 0.28 |
| NT2RP2000240 | F-NT2RP2000240 | 0.16 |
| NT2RP2000712 | F-NT2RP2000712 | 0.15 |
| NT2RP2001469 | F-NT2RP2001469 | 0.28 |
| NT2RP2001817 | F-NT2RP2001817 | 0.15 |
| NT2RP2002510 | F-NT2RP2002510 | 0.26 |
| NT2RP2002824 | F-NT2RP2002824 | 0.14 |
| NT2RP2002974 | F-NT2RP2002974 | 0.05 |
| NT2RP2003940 | F-NT2RP2003940 | 0.25 |
| NT2RP2003950 | F-NT2RP2003950 | 0.07 |
| NT2RP2005391 | F-NT2RP2005391 | 0.19 |
| NT2RP2006134 | F-NT2RP2006134 | 0.10 |
| NT2RP3000125 | F-NT2RP3000125 | 0.26 |
| NT2RP3000481 | F-NT2RP3000481 | 0.11 |
| NT2RP3000652 | F-NT2RP3000652 | 0.24 |
| NT2RP3000677 | F-NT2RP3000677 | 0.11 |
| NT2RP3001012 | F-NT2RP3001012 | 0.22 |
| NT2RP3001271 | F-NT2RP3001271 | 0.29 |
| NT2RP3001542 | F-NT2RP3001542 | 0.28 |
| OVARC1000090 | F-OVARC1000090 | 0.21 |
| OVARC1000439 | F-OVARC1000439 | 0.21 |
| OVARC1001260 | F-OVARC1001260 | 0.10 |
| OVARC1002178 | F-OVARC1002178 | 0.12 |
| PLACE1000033 | F-PLACE1000033 | 0.20 |
| PLACE1000258 | F-PLACE1000258 | 0.21 |
| PLACE1005539 | F-PLACE1005539 | 0.27 |
| PLACE1005745 | F-PLACE1005745 | 0.29 |
| PLACE1007077 | F-PLACE1007077 | 0.21 |
| PLACE1007296 | F-PLACE1007296 | 0.27 |
| PLACE1007591 | F-PLACE1007591 | 0.20 |
| PLACE1007845 | F-PLACE1007845 | 0.21 |
| PLACE2000118 | F-PLACE2000118 | 0.23 |
| PLACE3000213 | F-PLACE3000213 | 0.21 |
| PLACE4000354 | F-PLACE4000354 | 0.20 |
| Y79AA1000207 | F-Y79AA1000207 | 0.21 |
| Y79AA1001062 | F-Y79AA1001062 | 0.28 |
| Y79AA1001863 | F-Y79AA1001863 | 0.15 |
| Y79AA1002334 | F-Y79AA1002334 | 0.23 |
| Y79AA1002376 | F-Y79AA1002376 | 0.29 |

### EXAMPLE 13

### Selection of cDNA clone NT2RP2036580

Clone NT2RP2006580 as well as clone HEMBA1000121 was selected from the representative sequences belonging to HRIFA000116a cluster of the most homologous sequence in the SwissProt with the keywoods "transmembrane". Although each of the clones, HEMBA1000121 and NT2RP2006580, was assembled with other clones for 5' extension, any other clones did not extend the clones toward the 5' direction. Accordingly, it is possible that both clones are full-length cDNA clones. The maximal ATGpr1 score of F-NT2RP2006580 is 0.37, and therefore, the fullness ratio is low. However, it is still possible for the sequence to cover the full-length.

Thus, the total number of selected clones is 830. Based on the top matching data resulted from Swiss-Prot homology search, 659 clones were selected. From them, 447 clones were selected by the keywords of "secretion" and "membrane". Among the clones selected based on the top matching data, 60 clones exhibited the maximal ATGpr1 score of 0.3 or higher and less than 0.5.

The sequences of F-NT2RP2006580 and R-NT2RP2006580 are shown in SEQ ID NO: 2545 and SEQ ID NO: 2546, respectively.

### EXAMPLE 14

### Full-length sequence analysis and homology search

Full-length sequence was determined for each selected cDNA clones. The nucleotide sequence determination was performed mainly by the dye-terminator method using custom synthesized DNA primers according to the primer walking procedure (custom synthesized DNA primers were used for sequencing; sequencing reaction was performed with DNA sequencing reagent supplied by PE Biosystems according to the supplier's manual; and the samples were analyzed in an automatic sequencer made by the same supplier). Sequence determination of some clones was carried out in the same manner but using a Licor DNA sequencer. Overlapping partial nucleotide sequences, which were obtained by the above-described method, were assembled together to determine a full-length nucleotide sequence. Amino acid sequences were then deduced from the determined full-length nucleotide sequences. However, amino acid sequence is not shown for a clone of which coding region was hard to be deduced or of which amino acid sequence has less than 100 amino acid residues. SEQ ID NOs corresponding to the respective clones are indicated in Table 370.

GenBank, Swiss-Prot and UniGene were searched for the determined nucleotide sequences by BLAST analysis. Matching data of cDNA clone which exhibits higher homology and of which functions are easily predicted based on the nucleotide sequences and the deduced amino acid sequences are selected from the BLAST analysis matching data with P value of 10⁻⁴ or less. The matching data selected are listed herein. However, there are some clones that did not match the criteria for judgment and such matching data of BLAST analysis are not shown herein. The results of homology search indicated in the last part of this specification are as follows.

Homology search result 1: data obtained by the homology search of Swiss-Prot database for representative sequences of the 5'-end cluster

Homology search result 2: homology of representative sequences of the 5'-end cluster to the data in Swiss-Prot database; the P value is 10⁻¹⁰ or less

Homology search result 3: homology of representative sequences of the 5'-end cluster to the data in Swiss-Prot database; the P value is higher than 10⁻¹⁰ and 10⁻⁴ or less

Homology search result 4: homology of representative sequences of the 5'-end cluster to the data in Swiss-Prot database; the P value is higher than 10⁻⁴ and 1 or less

Homology search result 5: data obtained by the homology search of Swiss-Prot database for 5'-end sequences of cDNA clone

Homology search result 6: data obtained by the homology search of GenBank database (http:/lwww.ncbi.nlm.nih.gov/web/GenBank/) except for EST and STS sequence data for 5'-end sequences of cDNA clone

Homology search result 7: data obtained by the homology search of GenBank database (http://www.ncbi.nlm.nih.gov/web/GenBank/) except for EST and STS sequence data for 3'-end sequences of cDNA clone

Homology search result 8: data obtained by the homology search of Human UniGene database (http://www.ncbi.nlm.nih.gov/Unigene) for 5'-end sequences of cDNA clone

Homology search result 9: data obtained by the homology search of Human UniGene database (http://www.ncbi.nlm.nih.gov/Unigene) for 3'-end sequences of cDNA clone

Homology search result 10: result obtained by the homology search for full-length nucleotide sequences and deduced amino acid sequences

The P value indicates similarity between two sequences as a score by considering the probability that the two sequences are accidentally similar. In general, as the value is lower, the similarity is higher. In general, as the value is lower, the homology is higher (Altschul, S.F., Gish, W., Miller, W., Myers, E.W. & Lipman, D.L. (1990) "Basic local alignment search tool." J. Mol. Biol. 215:403-410; Gish, W. & States, D.J. (1993) "Identification of protein coding regions by database similarity search." Nature Genet. 3:266-272).

### Example 15. Gene expression analysis with hybridization using high density DNA filter

Nylon membrane for DNA spotting was prepared according to the following procedure. E. coli was cultured in each well of a 96-well plate (in a LB medium at 37. for 16 hours). A sample of each culture was suspended in 10 .1 of sterile water in a well of a 96-well plate. The plate was heated at 100. for 10 minutes. Then, the boiled samples were analyzed by PCR. PCR was performed in a 20 .1 solution by using TaKaRa PCR Amplification Kit (Takara) according to the supplier's protocol. Primers used for the amplification of an insert cDNA in a plasmid were a pair of sequencing primers, ME761FW (5' tacggaagtgttacttctgc 3'/ SEQ ID NO: 3591) and ME1250RV (5' tgtgggaggttttttctcta 3'/ SEQ ID NO: 3592), or a pair of primers, M13M4 (5' gttttcccagtcacgac 3'/ SEQ ID NO: 3593) and M13RV (5' caggaaacagctatgac 3' / SEQ ID NO: 3594). PCR was performed using a thermal cycler, GeneAmp System 9600 (PE Biosystems) at 95. for 5 minutes; at 95. for 10 seconds and at 68. for 1 minute for 10 cycles; at 98. for 20 seconds and at 60. for 3 minutes for 20 cycles; and at 72. for 10 minutes. After the PCR, the 20 .1 reaction solution was loaded onto a 1% agarose gel and fractionated by electrophoresis. DNA on the gel was stained with ethidium bromide to confirm the amplification of cDNA. When cDNAs were not amplified by PCR, plasmids containing the corresponding insert cDNAs were prepared by the alkali-extraction method (J. Sambrook, E.F., Fritsh, & T. Maniatis, "Molecular Cloning, A laboratory manual/ 2nd edition, Cold Spring Harbor Laboratory Press, 1989).

Preparation of DNA array was carried out by the following procedure. A sample of a DNA solution was added in each well of a 384-well plate. DNA was spotted onto a nylon membrane (Boehringer) by using a 384-pin tool of Biomek 2000 Laboratory Automation System (Beckman-Coulter). Specifically, the 384-well plate containing the DNA was placed under the 384-pin tool. The independent 384 needles were simultaneously dipped into the DNA solution for DNA deposition. The needles were gently pressed onto a nylon membrane and the DNA deposited at the tips of needles was spotted onto the membrane. Denaturation of the spotted DNA and immobilization of the DNA on the nylon membrane were carried out according to standard methods (J. Sambrook, E.F., Fritsh, & T. Maniatis, "Molecular Cloning, A laboratory manual/ 2nd edition, Cold Spring Harbor Laboratory Press, 1989).

A probe for hybridization was radioisotope-labeled first strand cDNA. Synthesis of the first strand cDNA was performed by using Thermoscript^{(TM)} RT-PCR System (GIBCO). Specifically, the first strand cDNA was synthesized by using 1.5 .g of mRNAs from various human tissues (Clontech), 1 .1 of 50.M Oligo(dT)20 and 50.Ci [.³³P]dATP according to an attached protocol. Purification of a probe was carried out by using ProbeQuant^{(TM)} G-50 micro column (Amersham-Pharmacia Biotech) according to an attached protocol. In the next step, 2 units of E. coli RNase H were added to the reaction mixture. The mixture was incubated at room temperature for 10 minutes, and then, 100.g of human COT-1 DNA (GIBCO) was added thereto. The mixture was incubated at 97. for 10 minutes and then was allowed to stand on ice to give hybridization probe.

Hybridization of the radioisotope-labeled probe to the DNA array was performed according to standard methods (J. Sambrook, E.F., Fritsh, & T. Maniatis, Molecular Cloning, A laboratory manual/ 2nd edition, Cold Spring Harbor Laboratory Press, 1989). The membrane was washed as follows: the nylon membrane was washed 3 times by incubating it in Washing solution 1 (2xSSC, 1% SDS) at room temperature (about 26.) for 20 minutes; then the membrane was washed 3 times by incubating it in Washing solution 2 (0.1xSSC, 1% SDS) at 65. for 20 minutes.

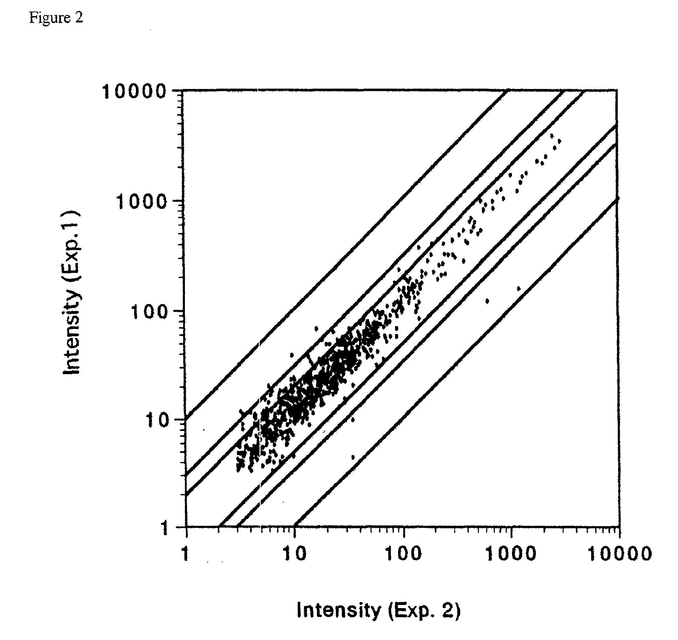

Autoradiography was performed by using an image plate for BAS2000 (Fuji Photo Film Co., Ltd.). Specifically, the nylon membrane with probe hybridized thereon was wrapped with a piece of Saran Wrap and brought into tight contact with the image plate on the light-sensitive surface. The membrane with the image plate was placed in an imaging cassette for radioisotope and allowed to stand in dark place for 4 hours. The radioactivity recorded on the image plate was analyzed by using BAS2000 (Fuji Photo Film Co., Ltd.). The activity was subjected to electronic conversion and recorded as an image file of autoradiogram. The signal intensity of each DNA spot was analyzed by using Visage High Density Grid Analysis Systems (Genomic Solutions Inc.). The signal intensity was converted into numerical data. The data were taken in duplicate. The reproducibility was assessed by comparing the signal intensities of the corresponding spots on the duplicated DNA filters that were hybridized to a single DNA probe (Figure 2). In 95% of entire spots, the ratio between the corresponding spots falls within a range of 2 or less, and the correlation coefficient is r=1.97. Thus, the reproducibility is satisfactory.

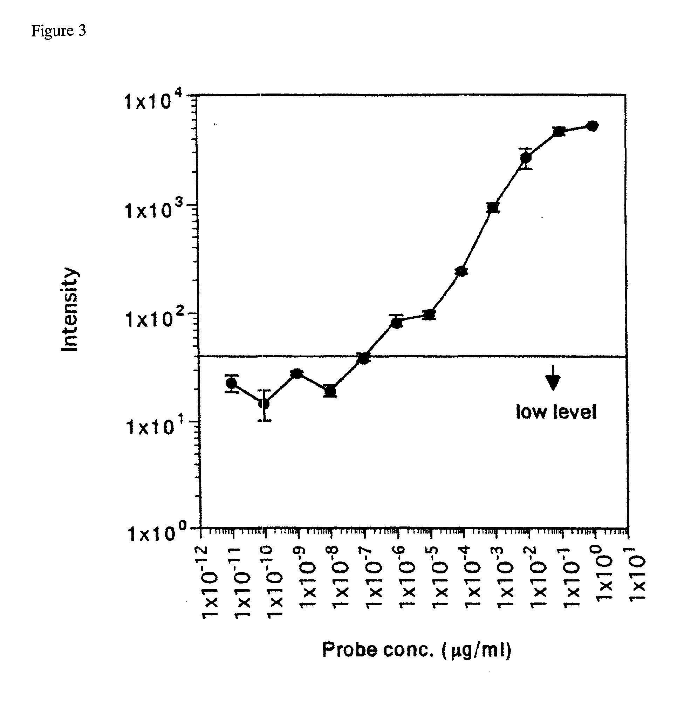

The detection sensitivity in gene expression analysis was estimated by examining increases in the signal intensity of probe concentration-dependent spot in hybridization using a probe complementary to the DNA spotted on the nylon membrane. DNA used was PLACE 1008092 (the same as DNA deposited in GenBank under an Accession No. AF107253). The DNA array with DNA of PLACE1008092 was prepared according to the above-mentioned method. The probe used was prepared as follows: mRNA was synthesized in vitro from the clone, PLACE1008092. By using this mRNA as a template, radioisotope-labeled first strand cDNA was synthesized in the same manner as described above, and the cDNA was used as the probe. In order to synthesize mRNA from PLACE1008092 in vitro, a plasmid in which the 5' end of the cDNA PLACE1008092 was ligated to the T7 promoter of pBluescript SK(-) was constructed. Specifically, the PLACE1008092 insert was cut out from pME18SFL3 carrying the cDNA at a DraIII site thereof by XhoI digestion. The resulting PLACE1008092 fragment was ligated to XhoI-predigested pBluescript SK(-) by using DNA ligation kit ver.2 (Takara). The in vitro mRNA synthesis from PLACE1008092 inserted into pBluescript SK(-) was carried out by using Ampliscribe^{(TM)} T7 high yield transcription kit (Epicentre technologies). Hybridization and the analysis of signal intensity of each DNA spot were performed by the same methods as described above. When the probe concentration is 1x10⁷.g/ml or less, there was no increase of signal intensity proportional to the probe concentration. Therefore, it was assumed to be difficult to compare the signals with one another in this concentration range. Thus, the spots with the intensity of 40 or less were uniformly taken as low level signals (Figure 3). Within a concentration of the probe ranging from 1x10⁷.g/ml to 0.1.g/ml, the signal was found to increase in a probe concentration-dependent manner. The detection limit represented as the ratio of the expression level of test mRNA to that of total mRNA in a sample was 1:100,000.

Tables 28-184 (also containing clones without description in Examples) show the expression of each cDNA in human normal tissues (heart, lung, pituitary gland, thymus, brain, kidney, liver and spleen). The expression levels are indicated with numerical values of 0-10,000. Genes that were expressed in at least a single tissue are indicated below by the corresponding clone names:
clone: BNGH41000020, BNGH41000087, BNGH41000091, HEMBA1000121, HEMBA1000275, HEMBA1000300, HEMBA1000443, HEMBA1000462, HEMBA1000477, HEMBA1000634, HEMBA1000713, HEMBA1000835, HEMBA1000875, HEMBA1000940, HEMBA1000962, HEMBA1001228, HEMBA1001296, HEMBA1001390, HEMBA1001563, HEMBA1001621, HEMBA1002048, HEMBA1002131, HEMBA1002163, HEMBA1002164, HEMBA1002167, HEMBA1002178, HEMBA1002195, HEMBA1002227, HEMBA1002239, HEMBA1002316, HEMBA1002421, HEMBA1002524, HEMBA1002551, HEMBA1002767, HEMBA1002985, HEMBA1002992, HEMBA1003047, HEMBA1003072, HEMBA1003101, HEMBA1003120, HEMBA1003230, HEMBA1003294, HEMBA1003315, HEMBA1003392, HEMBA1003399, HEMBA1003487, HEMBA1003530, HEMBA1003945, HEMBA1004007, HEMBA1004067, HEMBA1001085, HEMBA1004110, HEMBA1004391, HEMBA1004444, HEMBA1004454, HEMBA1004505, HEMBA1004797, HEMBA1004952, HEMBA1005070, HEMBA1005084, HEMBA1005145, HEMBA1005230, HEMBA1005246, HEMBA1005337, HEMBA1005430, HEMBA1005449, HEMBA1005489, HEMBA1005545, HEMBA1005698, HEMBA1005929, HEMBA1005945, HEMBA1005016, HEMBA1006171, HEMBA1006276, HEMBA1006311, HEMBA1006335, HEMBA1006357, HEMBA1006430, HEMBA1006482, HEMBA1006517, HEMBA1006544, HEMBA1006658, HEMBA1006707, HEMBA1006749, HEMBA1006770, HEMBA1006902, HEMBA1006912, HEMBA1006916, HEMBA1006960, HEMBA1007013, HEMBA1007057, HEMBA1007063, HEMBA1007291, HEMBA1007332, HEMBB1000106, HEMBB1000309, HEMBB1000447, HEMBB1000542,
HEMBB1000567, HEMBB1000642, HEMBB1000905, HEMBB1001026, HEMBB1001048, HEMBB1001407, HEMBB1001530, HEMBB1001573, HEMBB1001847, HEMBB1001959, HEMBB1001978, HEMBB1002039, HEMBB1002041, HEMBB1002051, HEMBB1002162, HEMBB1002228, HEMBB1002302, HEMBB1002427, HEMBB1002465, HEMBB1002661, HEMBB1002663, HEMBB1002693, MAMMA1000046, MAMMA1000102, MAMMA1000106, MAMMA1000118, MAMMA1000204, MAMMA1000226, MAMMA1000403, MAMMA1000449, MAMMA1000457, MAMMA1000473, MAMMA1000528, MAMMA1000591, MAMMA1000614, MAMMA1000652, MAMMA1000681, MAMMA1000706, MAMMA1000788, MAMMA1000810, MAMMA1000814, MAMMA1000881, MAMMA1000986, MAMMA1000994, MAMMA1001043, MAMMA1001066, MAMMA1001094, MAMMA1001141, MAMMA1001150, MAMMA1001284, MAMMA1001310, MAMMA1001344, MAMMA1001418, MAMMA1001532, MAMMA1001609, MAMMA1001615, MAMMA1001634, MAMMA1001893, MAMMA1001901, MAMMA1001957, MAMMA1002070, MAMMA1002091, MAMMA1002095, MAMMA1002128, MAMMA1002142, MAMMA1002165, MAMMA1002205, MAMMA1002224, MAMMA1002586, MAMMA1003126, NT2RM1000407, NT2RM1000462, NT2RM1000542, NT2RM1000789, NT2RM1000855, NT2RM1000858, NT2RM2000241, NT2RM2000306, NT2RM2000410, NT2RM2000423, NT2RM2000497, NT2RM2000514, NT2RM2000565, NT2RM2000582, NT2RM2000589, NT2RM2000622, NT2RM2000773, NT2RM2001126, NT2RM2001626, NT2RM2001792, NT2RM2001941, NT2RM4000198, NT2RM4000295, NT2RM4000444, NT2RM4000593, NT2RM4000761, NT2RM4000965, NT2RM4000997, NT2RM4001321, NT2RM4001325,
NT2RM4001377, NT2RM4001735, NT2RM4001768, NT2RM4001843, NT2RP1000002, NT2RP1000181, NT2RP1000271, NT2RP1000300, NT2RP1000325, NT2RP1000465, NT2RP1000468, NT2RP1000740, NT2RP1000903, NT2RF1000981, NT2RP2000092, NT2RP2000178, NT2RP2000240, NT2RP2000447, NT2RP2000479, NT2RP2000533, NT2RP2000610, NT2RP2000616, NT2RP2000694, NT2RP2000739, NT2RP2001200, NT2RP2001223, NT2RP2001388, NT2RP2001469, NT2RP2001480, NT2RP2001514, NT2RP2001529, NT2RP2001538, NT2RP2001562, NT2RP2001662, NT2RP2001878, NT2RP2001903, NT2RP2001921, NT2RP2001956, NT2RP2002015, NT2RP2002063, NT2RP2002188, NT2RP2002232, NT2RP2002409, NT2RP2002510, NT2RP2002527, NT2RP2002533, NT2RP2002564, NT2RP2002721, NT2RP2002824, NT2RP2002942, NT2RP2002974, NT2RP2003138, NT2RP2003179, NT2RP2003210, NT2RP2003302, NT2RP2003369, NT2RP2003383, NT2RP2003390, NT2RP2003469, NT2RP2003593, NT2RP2003599, NT2RP2003655, NT2RP2003940, NT2RP2003950, NT2RP2004069, NT2RP2004108, NT2RP2004141, NT2RP2004179, NT2RP2004205, NT2RP2004447, NT2RP2004524, NT2RP2004556, NT2RP2004606, NT2RP2004648, NT2RP2004794, NT2RP2004837, NT2RP2004847, NT2RP2005027, NT2RP2005069, NT2RP2005163, NT2RP2005181, NT2RP2005247, NT2RP2005378, NT2RP2005391, NT2RP2005425, NT2RP2005463, NT2RP2005535, NT2RP2005541, NT2RP2005597, NT2RP2005632, NT2RP2005666, NT2RP2005774, NT2RP2005878, NT2RP2005887, NT2RP2005941, NT2RP2006004, NT2RP2006042, NT2RP2006092, NT2RP2006099, NT2RP2006269, NT2RP3000011, NT2RP3000022, NT2RP3000059, NT2RP3000063, NT2RP3000125, NT2RP3000148, NT2RP3000171, NT2RP3000172, NT2RP3000201, NT2RP3000232, NT2RP3000304, NT2RP3000378, NT2RP3000436, NT2RP3000460, NT2RP3000645, NT2RP3000652, NT2RP3000676, NT2RP3000677, NT2RP3000721, NT2RP3000789, NT2RP3000818, NT2RP3000820, NT2RP3000838, NT2RP3000907, NT2RP3000921, NT2RP3001044, NT2RP3001159, NT2RP3001170, NT2RP3001195, NT2RP3001271, NT2RP3001388, NT2RP3001560, NT2RP3001592, NT2RP3001685, NT2RP3001738, NT2RP3001754, NT2RP3001858, NT2RP3001976, NT2RP3002015, NT2RP3002160, NT2RP3002281, NT2RP3002311, NT2RP3002324, NT2RP3002353, NT2RP3002409, NT2RP3002411, NT2RP3002721, NT2RP3002737, NT2RP3002738, NT2RP3002836, NT2RP3002900, NT2RP3002958, NT2RP3003000, NT2RP3003076, NT2RP3003354, NT2RP3003448, NT2RP3003469, NT2RP3003473, NT2RP3003532, NT2RP3003614, NT2RP3003729, NT2RP3003849, NT2RP3003874, NT2RP3003939, NT2RP3003963, NT2RP3004025, NT2RP3004067, NT2RP3004083, NT2RP3004090, NT2RP3004119, NT2RP3004130, NT2RP3004133, NT2RP3004202, NT2RP3004294, NT2RP3004309, NT2RP3004321, NT2RP3004355, NT2RP3004374, NT2RP3004406, NT2RP3004481, NT2RP3004552, NT2RP3004557, NT2RP3004625, NT2RP3004640, NT2RP3004647, NT2RP4000108, NT2RP4000634, NT2RP4001877, NT2RP4001879, NT2RP4002187, NT2RP4002715, NT2RP4002750, OVARC1000090, OVARC1000105, OVARC1000137, OVARC1000208, OVARC1000255, OVARC1000313, OVARC1000331, OVARC1000410, OVARC1000439, OVARC1000467, OVARC1000529, OVARC1000553, OVARC1000775, OVARC1000853, OVARC1000873, OVARC1000916, OVARC1000956, OVARC1000995, OVARC1001030, OVARC1001049, OVARC1001086, OVARC1001163, OVARC1001260, OVARC1001336, OVARC1001569, OVARC1001570, OVARC1001596, OVARC1001807, OVARC1001833, OVARC1001991, PLACE1000231, PLACE1000258, PLACE1000442, PLACE1000560, PLACE1000912, PLACE1000927, PLACE1001016, PLACE1001100, PLACE1001114, PLACE1001183, PLACE1001229, PLACE1001340, PLACE1001407, PLACE1001500, PLACE1001516, PLACE1001655, PLACE1001836, PLACE1001918, PLACE1002080, PLACE1002095, PLACE1002153, PLACE1002329, PLACE1002374, PLACE1002518, PLACE1002547, PLACE1002726, PLACE1002905, PLACE1002911, PLACE1002967, PLACE1003163, PLACE1003407, PLACE1003428, PLACE1003438, PLACE1003460, PLACE1003529, FLACE1003598, PLACE1003644, PLACE1003772, PLACE1003839, PLACE1003845, PLACE1003852, PLACE1004078, PLACE1004166, PLACE1004168, PLACE1004199, PLACE1004279, PLACE1004282, PLACE1004305, PLACE1004441, PLACE1004482, PLACE1004492, PLACE1004520, PLACE1004630, PLACE1004637, PLACE1004648, PLACE1004816, PLACE1004887, PLACE1005005, PLACE1005031, PLACE1005383, PLACE1005410, PLACE1005426, PLACE1005539, PLACE1005544, PLACE1005569, PLACE1005725, PLACE1005736, PLACE1005768, PLACE1005815, PLACE1005878, PLACE1005927, PLACE1006071, PLACE1006073, PLACE1006079, PLACE1006277, PLACE1006443, PLACE1006716, PLACE1006809, PLACE1007077, PLACE1007096, PLACE1007626, PLACE1007702, PLACE1008469, PLACE1008985, PLACE1009067, PLACE1009527, PLACE1009982, PLACE1010078, PLACE1010251, PLACE1010445, PLACE1011045, PLACE1011116, PLACE1011181, PLACE1011236, PLACE1011364, PLACE1011516, PLACE1011708, PLACE1011978, PLACE2000118, PLACE2000219, PLACE3000181, PLACE4000354, PLACE4000455, SKNMC1000014, THYRO1000061, THYRO1000099, THYRO1000584, THYRO1000795, THYRO1000866, THYRO1000999, THYRO1001063, THYRO1001113, THYRO1001128, THYRO1001205, THYRO1001237, THYRO1001242, THYRO1001456, THYRO1001457, THYRO1001478, THYRO1001495, THYRO1001523, THYRO1001529, THYRO1001593, THYRO1001608, THYRO1001700, THYRO1001702, THYRO1001725, THYRO1001770, THYRO1001803, Y79AA1000127, Y79AA1000207, Y79AA1000226, Y79AA1000270, Y79AA1000426, Y79AA1000521, Y79AA1000776, Y79AA1000777, Y79AA1000888, Y79AA1000967, Y79AA1001013, Y79AA1001090, Y79AA1001272, Y79AA1001328, Y79AA1001426, Y79AA1001427, Y79AA1001430, Y79AA1001523, Y79AA1001530, Y79AA1001592, Y79AA1001727, Y79AA1001787, Y79AA1001793, Y79AA1001799, Y79AA1001803, Y79AA1001863, Y79AA1002022, Y79AA1002213, Y79AA1002373, Y79AA1002376, Y79AA1002381.

Genes that were expressed in all the tissues tested are indicated below by the corresponding clone names: clone: BNGH41000020, HEMBA1000300, HEMBA1001390, HEMBA1002239, HEMBA1002316, HEMBA1004007, HEMBA1004067, HEMBA1005145, HEMBA1005230, HEMBA1005929, HEMBA1006357, HEMBA1006482, HEMBB1000567, HEMBB1001847, NEMBB1001978, MAMMA1000614, MAMMA1000652, MAMMA1000810, MAMMA1000814, MAMMA1001066, MAMMA1001094, MAMMA1001284, MAMMA1001310, MAMMA1001634, MAMMA1002165, MAMMA1002205, MAMMA1002224, NT2RM1000462, NT2RM1000855, NT2RM1000858, NT2RM2000423, NT2RM4000761, NT2RM4000997, NT2RP1000271, NT2RP1000325, NT2RP1000465, NT2RP2001538, NT2RP2001662, NT2RP2001903, NT2RP2002015, NT2RP2002188, NT2RP2002409, NT2RP2002510, NT2RP2002533, NT2RP2004556, NT2RP2004794, NT2RP2004847, NT2RP2005069, NT2RP2005163, NT2RP2005535, NT2RP2006269, NT2RP3000171, NT2RP3000645, NT2RP3000838, NT2RP3001271, NT2RP3001754, NT2RP3003076, NT2RP3003354, NT2RP3003614, NT2RP3004640, NT2RP3004647, OVARC1000090, OVARC1000208, OVARC1000553, OVARC1000995, OVARC1001030, OVARC1001049, PLACE1000231, PLACE1000258, PLACE1001516, PLACE1002080, PLACE1002911, PLACE1003598, PLACE1004648, PLACE1006443, PLACE1008469, PLACE1011708, PLACE2000118, THYRO1001128, THYRO1001205, THYRO1001242, THYRO1001803, Y79AA1000207, Y79AA1001013, Y79AA1001272, Y79AA1001328, Y79AA1001793, Y79AA1001863, Y79AA1002022, Y79AA1002376.

Genes that were expressed at low levels in any of the tissues tested are indicated below by the corresponding clone names: clone: HEMBA1000006, HEMBA1000128, HEMBA1000349, HEMBA1000590, HEMBA1000671, HEMBA1000732, HEMBA1000745, HEMBA1000907, HEMBA1001184, HEMBA1001221, HEMBA1001272, HEMBA1001297, HEMBA1001878, HEMBA1001886, HEMBA1002420, HEMBA1003497, HEMBA1003602, HEMBA1003732, HEMBA1004250, HEMBA1004785, HEMBA1004971, HEMBA1004982, HEMBA1005267, HEMBA1005522, HEMBA1005913, HEMBA1006299, HEMBA1006572, HEMBA1006724, HEMBA1007241, HEMBB1000276, HEMBB1000407, HEMBB1000668, HEMBB1000679, HEMBB1000881, HEMBB1001200, HEMBB1001547, HEMBB1002120, HEMBB1002245, MAMMA1000141, MAMMA1000496, MAMMA1001237, MAMMA1001623, MAMMA1001978, MAMMA1002080, MAMMA1002087, MAMMA1002234, MAMMA1002633, NT2RM1000580, NT2RM1000899, NT2RM2000632, NT2RM2001643, NT2PM2001818, NT2RM2001902, NT2RM2001939, NT2RM4000100, NT2RM4000115, NT2RM4000284, NT2RM4000326, NT2RM4000417, NT2RM4000587, NT2RM4000648, NT2RM4002352, NT2RP1000050, NT2RP1000239, NT2RP1000261, NT2RP1000448, NT2RP1000551, NT2RP1000579, NT2RP1000613, NT2RP1000679, NT2RP1001004, NT2RP1001020, NT2RP1001031, NT2RP1001563, NT2RP2000394, NT2RP2000514, NT2RP2000649, NT2RP2000663, NT2RP2000712, NT2RP2000818, NT2RP2000903, NT2RP2001276, NT2RP2001495, NT2RP2001755, NT2RP2001769, NT2RP2001817, NT2RP2001915, NT2RP2001948, NT2RP2002304, NT2RP2002674, NT2RP2002976, NT2RP2003042, NT2RP2003545, NT2RP2003664, NT2RP2003931, NT2RP2004495, NT2RP2004670, NT2RP2005514, NT2RP2005883, NT2RP2005994,
NT2RP2006134, NT2RP2006512, NT2RP3000169, NT2RP3000444, NT2RP3000481, NT2RP3000616, NT2RP3000871, NT2RP3001012, NT2RP3001061, NT2RP3001240, NT2RP3001322, NT2RP3001542, NT2RP3002286, NT2RP3002342, NT2RP3002448, NT2RP3002571, NT2RP3002664, NT2RP3002790, NT2RP3002887, NT2RP3002983, NT2RP3003527, NT2RP3003535, NT2RP3003559, NT2RP3004000, NT2RP3004075, NT2RP3004345, NT2RP4000962, NT2RP4001001, NT2RP4001009, NT2RP4001467, NT2RP4002451, OVARC1000003, OVARC1000275, OVARC1000298, OVARC1000307, OVARC1000811, OVARC1001132, OVARC1001222, OVARC1001338, OVARC1001607, OVARC1001725, OVARC1001727, OVARC1002058, OVARC1002178, PLACE1000033, PLACE1000740, PLACE1000914, PLACE1000986, PLACE1001123, PLACE1001231, PLACE1001401, PLACE1001464, PLACE1001536, PLACE1001564, PLACE1001788, PLACE1001795, PLACE1001949, PLACE1002355, PLACE1003135, PLACE1003573, PLACE1003737, PLACE1004028, PLACE1004450, PLACE1004519, PLACE1005003, PLACE1005239, PLACE1005250, PLACE1005519, PLACE1005601, PLACE1005660, PLACE1005669, PLACE1005682, PLACE1005745, PLACE1006093, PLACE1006208, PLACE1006219, PLACE1006290, PLACE1006515, PLACE1006786, PLACE1006959, PLACE1007028, PLACE1007040, PLACE1007081, PLACE1007296, PLACE1007591, PLACE1007845, PLACE1007881, PLACE1007971, PLACE1008282, PLACE1008297, PLACE1008359, PLACE1008549, PLACE1008657, PLACE1008716, PLACE1008744, PLACE1008984, PLACE1009196,
PLACE1009279, PLACE1009546, PLACE1009600, PLACE1009735, PLACE1010011, PLACE1010081, PLACE1010713, PLACE1010784, PLACE1010827, PLACE1010968, PLACE1011407, PLACE1011824, PLACE3000213, SKNMC1000004, SKNMC1000082, THYRO1000036, THYRO1000196, THYRO1000400, THYRO1000580, THYRO1000678, THYRO1000776, THYRO1000846, THYRO1000956, THYRO1001071, THYRO1001102, THYRO1001266, THYRO1001327, THYRO1001471, Y79AA1000876, Y79AA1000959, Y79AA1001056, Y79AA1001062, Y79AA1001264, Y79AA1001795.

Genes exhibiting characteristic features in the expression thereof were selected by statistical analysis of these data. Two examples are shown below to describe the selection of genes of which expression is varied greatly among tissues. The .-actin gene is used frequently as a control in gene expression analysis. Genes of which expression is varied greatly among tissues as compared that of the .-actin gene were determined as follows. Specifically, sum of squared deviation was calculated in the signal intensity of .-actin observed in each tissue, which was divided by 7 degrees of freedom to determine a variance Sₐ². Next, sum of squared deviation was calculated in the signal intensity of a compared gene in each tissue, which was divided by 7 degrees of freedom to determine a variance S_{b}². By taking variance ratio F as F=S_{b}²/Sₐ², genes with a significance level of 5% or more were extracted in the F distribution. Genes extracted are indicated below by the corresponding clone names: clone: BNGH41000020, NT2RM4000761, Y79AA1002376.

Gene of OVARC1000037{heterogeneous nuclear ribonucleoprotein (hnRNP)} which expression is varied little. Genes of which expression is varied greatly among tissues as compared that of the OVARC1000037 gene were determined as follows. Specifically, sum of squared deviation was calculated in the signal intensity of .-actin observed in each tissue, which was divided by 7 degrees of freedom to determine a variance Sₐ². Next, sum of squared deviation was calculated in the signal intensity of a gene to be compared observed in each tissue, which was divided by 7 degrees of freedom to determine a variance S_{b}². By taking variance ratio F as F=S_{b}²/Sₐ², genes with a significance level of 5% or more were extracted in the F distribution. Genes extracted are indicated below by the corresponding clone names:
clone: BNGH41000020, HEMBA1000300, OVARC1001030, NT2RM4000761, PLACE1000231, HEMBA1002316, NT2RP1000325, NT2RP1000271, PLACE1004648, HEMBA1005145, HEMBA1005929, NT2RP2002510, NT2RP2001538, NT2RP2002409, NT2RP2002188, NT2RP2001903, NT2RP2002533, NT2RP2002015, NT2RP2006269, NT2RP2004837, NT2RP2004205, NT2RP2005378, HEMBA1006357, HEMBB1000567, NT2RP2003940, NT2RP2004794, HEMBA1006912, NT2RP2004556, NT2RP2005163, NT2RP3000838, NT2RP3001271, PLACE2000118, NT2RP3000645, NT2RP3003076, HEMBB1002693, MAMMA1000046, NT2RP3003354, THYR01001205, MAMMA1000614, MAMMA1000652, MAMMA1000810, THYRO1001242, MAMMA1001066, MAMMA1002224, MAMMA1001634, MAMMA1001094, MAMMA1002205, NT2RM1000855, NT2RM1000858, Y79AA1002376, NT2RM2000423.

Thus, characteristic features in the expression of a gene are illustrated by comparing and statistically analyzing the expression of many genes.

### Analysis of disease-associated genes

Non-enzymic protein glycation reaction is believed to be a cause of a variety of chronic diabetic complications. Accordingly, genes of which expression is elevated or decreased in a glycated protein-specific manner in the endothelial cells are associated with diabetic complications caused by glycated proteins. Vascular endothelial cells are affected with glycated proteins present in blood. Reaction products of non-enzymic protein glycation include amadori compound (glycated protein) as a mildly glycated protein and advanced glycation endproduct as a heavily glycated protein. Hence, a survey was carried out for genes of which expression levels are varied depending on the presence of these glycated proteins in endothelial cells. The mRNAs were extracted from endothelial cells that were cultured in the presence or absence of glycated protein. The mRNAs were converted into radiolabeled first strand cDNAs for preparing probes. The probes were hybridized to the above-mentioned DNA array. Signal of each DNA spot was detected by BAS2000 and analyzed by ArrayGauge (Fuji Photo Film Co., Ltd.).

Advanced glycation endproduct of bovine serum albumin was prepared as follows: bovine serum albumin (BSA; Sigma) was incubated in a phosphate buffer solution containing 50 mM glucose at 37 for 8 weeks; and the resulting brownish BSA was dialyzed against a phosphate buffer solution.

Human normal pulmonary arterial endothelial cells (Cell Applications) were cultured in an Endothelial Cell Growth Medium (Cell Applications). The culture dish (Farcon) with the cells were incubated in a CO₂ incubator (37., 5% CO₂, in a humid atmosphere). When the cells were grown to be confluent in the dish, 250.g/ml of bovine serum albumin (sigma), glycated bovine serum albumin (Sigma) or advanced glycation endproduct of bovine serum albumin was added thereto and the cells were incubated for 33 hours. The mRNA was extracted from the cells by using a FastTack^{(TM)} 2.0 kit (Invitrogen). The labeling of hybridization probe was carried out by using the mRNA according to the same procedure as described above.

Table 185 shows the expression level of each cDNA in human pulmonary arterial endothelial cells cultured in a medium containing bovine serum albumin (sigma), glycated bovine serum albumin (Sigma) or advanced glycation endproduct of bovine serum albumin. Genes of which expression was detected in the endothelial cell are as follows:
BNGH41000020, BNGH41000087, HEMBA1000275, HEMBA1000300, HEMBA1000477, HEMBA1000634, HEMBA1000671, HEMBA1000713, HEMBA1000745, HEMBA1000835, HEMBA1000875, HEMBA1000940, HEMBA1001390, HEMBA1002131, HEMBA1002163, HEMBA1002164, HEMBA1002195, HEMBA1002227, HEMBA1002239, HEMBA1002420, HEMBA1002767, HEMBA1002992, HEMBA1003047, HEMBA1003120, HEMBA1003294, HEMBA1003315, HEMBA1003602, HEMBA1003945, HEMBA1004007, HEMBA1004067, HEMBA1004971, HEMBA1005145, HEMBA1005267, HEMBA1005337, HEMBA1005698, HEMBA1005929, HEMBA1005945, HEMBA1006171, HEMBA1006299, HEMBA1006335, HEMBA1006357, HEMBA1006430, HEMBA1006482, HEMBA1006658, HEMBA1006724, HEMBA1006770, HEMBA1006912, HEMBA1006960, HEMBA1007063, HEMBB1000447, HEMBB1000642, HEMBB1000905, HEMBB1001026, HEMBB1001048, HEMBB1001573, HEMBB1001847, HEMBB1001978, HEMBB1002041, HEMBB1002427, HEMBB1002663, HEMBB1002693, MAMMA1000102, MAMMA1000106, MAMMA1000204, MAMMA1000403, MAMMA1000449, MAMMA1000614, MAMMA1000652, MAMMA1000810, MAMMA1000814, MAMMA1000881, MAMMA1000986, MAMMA1001066, MAMMA1001237, MAMMA1001284, MAMMA1001344, MAMMA1001615, MAMMA1001634, MAMMA1001893, MAMMA1001901, MAMMA1001957, MAMMA1002087, MAMMA1002095, MAMMA1002165, MAMMA1002205, MAMMA1002224, MAMMA1002633, MAMMA1003126, NT2RM1000462, NT2RM1000580, NT2RM1000789, NT2RM1000855, NT2RM1000858,
NT2RM2000241, NT2RM2000306, NT2RM2000410, NT2RM2000423, NT2RM2000582, NT2RM2000589, NT2RM2000622, NT2RM2000773, NT2RM4000100, NT2RM4000198, NT2RM4000284, NT2RM4000444, NT2RM4000587, NT2RM4000593, NT2RM4000761, NT2RM4000997, NT2RM4001321, NT2RM4001325, NT2RM4001377, NT2RM4001735, NT2RM4001768, NT2RM4001843, NT2RP1000002, NT2RP1000181, NT2RP1000271, NT2RP1000300, NT2RP1000325, NT2RP1000465, NT2RP1000740, NT2RP1000981, NT2RP2000092, NT2RP2000240, NT2RP2000479, NT2RP2000533, NT2RP2000610, NT2RP2000616, NT2RP2000649, NT2RP2000663, NT2RP2000712, NT2RP2000903, NT2RP2001276, NT2RP2001388, NT2RP2001480, NT2RP2001495, NT2RP2001529, NT2RP2001538, NT2RP2001662, NT2RP2001878, NT2RP2001903, NT2RP2001948, NT2RP2001956, NT2RP2002015, NT2RP2002188, NT2RP2002232, NT2RP2002409, NT2RP2002510, NT2RP2002527, NT2RP2002533, NT2RP2002564, NT2RP2002721, NT2RP2002824, NT2RP2002942, NT2RP2002976, NT2RP2003138, NT2RP2003210, NT2RP2003390, NT2RP2003593, NT2RP2003599, NT2RP2003664, NT2RP2003931, NT2RP2003940, NT2RP2004069, NT2RP2004108, NT2RP2004179, NT2RP2004205, NT2RP2004495, NT2RP2004524, NT2RP2004556, NT2RP2004606, NT2RP2004648, NT2RP2004794, NT2RP2004837, NT2RP2004847, NT2RP2005027, NT2RP2005069, NT2RP2005163, NT2RP2005247, NT2RP2005378, NT2RP2005425, NT2RP2005535, NT2RP2005541, NT2RP2005632, NT2RP2005774,
NT2RP2005878, NT2RP2006099, NT2RP2006134, NT2RP2006269, NT2RP2006512, NT2RP3000011, NT2RP3000171, NT2RP3000201, NT2RP3000232, NT2RP3000436, NT2RP3000460, NT2RP3000645, NT2RP3000652, NT2RP3000676, NT2RP3000721, NT2RP3000818, NT2RP3000820, NT2RP3000838, NT2RP3000907, NT2RP3001159, NT2RP3001195, NT2RP3001240, NT2RP3001271, NT2RP3001388, NT2RP3001592, NT2RP3001738, NT2RP3001754, NT2RP3002015, NT2RP3002324, NT2RP3002342, NT2RP3002353, NT2RP3002409, NT2RP3002448, NT2RP3002721, NT2RP3002737, NT2RP3002738, NT2RP3002836, NT2RP3002900, NT2RP3003076, NT2RP3003354, NT2RP3003448, NT2RP3003473, NT2RP3003532, NT2RP3003614, NT2RP3003939, NT2RP3003963, NT2RP3004025, NT2RP3004067, NT2RP3004075, NT2RP3004083, NT2RP3004090, NT2RP3004119, NT2RP3004130, NT2RP3004133, NT2RP3004294, NT2RP3004309, NT2RP3004345, NT2RP3004374, NT2RP3004557, NT2RP3004625, NT2RP3004640, NT2RP3004647, NT2RP4000108, NT2RP4000634, NT2RP4001001, NT2RP4001009, NT2RP4001467, NT2RP4001877, NT2RP4001879, NT2RP4002187, NT2RP4002451, NT2RP4002715, OVARC1000003, OVARC1000090, OVARC1000105, OVARC1000137, OVARC1000208, OVARC1000298, OVARC1000313, OVARC1000331, OVARC1000410, OVARC1000439, OVARC1000553, OVARC1000775, OVARC1000853, OVARC1000873, OVARC1000916, OVARC1000956, OVARC1000995, OVARC1001030, OVARC1001049, OVARC1001086, OVARC1001132,
OVARC1001222, OVARC1001260, OVARC1001336, OVARC1001569, OVARC1001570, OVARC1001596, OVARC1001607, OVARC1001807, OVARC1001991, PLACE1000231, PLACE1000258, PLACE1000442, PLACE1000740, PLACE1000927, PLACE1001016, PLACE1001100, PLACE1001114, PLACE1001123, PLACE1001229, PLACE1001340, PLACE1001407, PLACE1001464, PLACE1001788, PLACE1001795, PLACE1001918, PLACE1002080, PLACE1002095, PLACE1002329, PLACE1002374, PLACE1002518, PLACE1002547, PLACE1002726, PLACE1002905, PLACE1002911, PLACE1002967, PLACE1003163, PLACE1003407, PLACE1003460, PLACE1003573, PLACE1003598, PLACE1003644, PLACE1003772, PLACE1003839, PLACE1003845, PLACE1004078, PLACE1004166, PLACE1004168, PLACE1004199, PLACE1004279, PLACE1004282, PLACE1004441, PLACE1004482, PLACE1004492, PLACE1004637, PLACE1004887, PLACE1005003, PLACE1005005, PLACE1005031, PLACE1005250, PLACE1005410, PLACE1005519, PLACE1005544, PLACE1005660, PLACE1005669, PLACE1005725, PLACE1005736, PLACE1005745, PLACE1005768, PLACE1005815, PLACE1006073, PLACE1006208, PLACE1006219, PLACE1006290, PLACE1006443, PLACE1006809, PLACE1006959, PLACE1007028, PLACE1007296, PLACE1007626, PLACE1007702, PLACE1007845, PLACE1008282, PLACE1008469, PLACE1008657, PLACE1009196, PLACE1009600, PLACE1003735, PLACE1010081, PLACE1010251, PLACE1010713, PLACE1011116, PLACE1011181,
PLACE1011236, PLACE1011516, PLACE1011708, PLACE1011824, PLACE1011978, PLACE2000118, PLACE3000181, SKNMC1000004, SKNMC1000014, THYRO1000584, THYRO1000866, THYRO1001113, THYRO1001128, THYRO1001205, THYRO1001242, THYRO1001495, THYRO1001523, THYRO1001529, THYRO1001593, THYRO1001608, THYRO1001702, THYRO1001725, THYRO1001770, THYRO1001803, Y79AA1000117, Y79AA1000207, Y79AA1000226, Y79AA1000270, Y79AA1000426, Y79AA1000777, Y79AA1000876, Y79AA1000888, Y79AA1000959, Y79AA1001013, Y79AA1001056, Y79AA1001090, Y79AA1001264, Y79AA1001272, Y79AA1001328, Y79AA1001427, Y79AA1001430, Y79AA1001530, Y79AA1001592, Y79AA1001727, Y79AA1001793, Y79AA1001799, Y79AA1001863, Y79AA1002022, Y79AA1002213, Y79AA1002373, Y79AA1002376, Y79AA1002381.

Signal ratios of EC_AGE_BSA to EC_BSA and of EC_glycated_BSA to EC_BSA were calculated for each gene. Genes with high signal ratios were selected. In the case of calculating the ratio of signal value of 40 or less to that of more than 40, such signal values were, for convenience, taken as 40 instead of the real values. When the ratio EC_AGE_BSA/EC_BSA is 2 or more, expression of the genes exhibiting such ratio is expected to be elevated due to advanced glycation endproduct of bovine serum albumin. The higher the value is, the higher the gene expression level is. When the ratio EC_AGE_BSA/EC_BSA ranges from 0.5 to 2, expression of the genes exhibiting such ratio is expected to be unaffected due to advanced glycation endproduct of bovine serum albumin. When the ratio EC_AGE_BSA/EC_BSA is less than 0.5, expression of the genes exhibiting such ratio value is expected to be decreased due to advanced glycation endproduct of bovine serum albumin. The lower the value is, the lower the gene expression level is.

Clone with EC_AGE_BSA/BC_BSA ratio of 2 or higher are as follows: NT2RP2001538, NT2RP4001001 and Y79AA1000967.

These cDNAs are associated with diabetes.

### Analysis of genes associated with neural cell differentiation

Genes involved in neural cell differentiation are useful for treating neurological diseases. It is possible that genes with varying expression levels in response to induction of cellular differentiation in neural cells are associated with neurological diseases.

A survey was performed for genes of which expression levels are varied in response to induction of differentiation (stimulation by retinoic acid (RA)) in cultured cells of a neural strain, NT2.

The NT2 cells were treated basically according to supplier's instruction manual. "Undifferentiated NT2 cells" means NT2 cells successively cultured in an Opti-MEM I (GIBCO-BRL; catalog No. 31985) containing 10%(v/v) fetal bovine serum and 1%(v/v) penicillin-streptomycin (GIBCO BRL). "NT2 cells cultured in the presence of retinoic acid" means the cells resulted from transferring undifferentiated NT2 cells into a retinoic acid-containing medium, which consists of D-MEM (GIBCO BRL; catalog No. 11965), 10%(v/v) fetal bovine serum, 1%(v/v) penicillin-streptomycin and 10.M retinoic acid (GIBCO-BRL), and the subsequent successive culture therein for 5 weeks. "NT2 cells that were cultured in the presence of retinoic acid and then further cultured in the presence of cell-division inhibitor added" means NT2 cells resulted from transferring NT2 cells cultured in the presence of retinoic acid for 5 weeks into a cell-division inhibitor-containing medium, which consisted of D-MEM(GIBCO BRL; catalog No.11965), 10%(v/v) fetal bovine serum, 1%(v/v) penicillin-streptomycin, 10. M retinoic acid, 10.M FudR (5-fluoro-2'-deoxyuridine: GIBCO BRL), 10. M Urd (Uridine: GIBCO BRL) and 1.M araC (Cytosine.-D-Arabinofuranoside: GIBCO BRL), and the subsequence successive culture for 2 weeks. Each of the cells were treated with trypsin and then harvested. Total RNAs were extracted from the cells by using S.N.A.P.^{(TM)} Total RNA Isolation kit (Invitrogen). The labeling of probe used for hybridization was carried out by using 10.g of the total RNA according to the same methods as described above. The data were obtained in triplicate (n=3). The data of signal value representing gene expression level in the cells in the presence of stimulation for inducing differentiation were compared with those in the absence of the stimulation. The comparison was performed by statistical treatment of two-sample t-test. Clones with significant difference in the signal distribution were selected under the condition of p<0.05. In this analysis, clones with the difference can be statistically detected even when the signals were low. Accordingly, clones with signal value of 40 or less were also assessed for the selection.

Tables 186-365 show the expression level of each cDNA in undifferentiated NT2 cells, NT2 cells cultured in the presence of RA, and NT2 cells that were cultured in the presence of RA and that were further cultured in the presence of cell-division inhibitor added.

Averaged signal values (M₁, M₂) and sample variances (s₁², s₂²) were calculated for each gene in each of the cells, and then, the pooled sample variances s² were obtained from the sample variances of the two types of cells to be compared. The t values were determined according to the following formula: t=(M1-M2)/s/(1/3+1/3)^{1/2}. When the determined t-value was greater than a t-value at P, which means the probability of significance level, of 0.05 or 0.01 in the t-distribution table with 4 degrees of freedom, the difference was judged to be found in the expression level of the gene between the two types of cells at p<0.05 or p<0.01, respectively. The tables also include the information on an increase (+) or decrease (-) in the expression level of a gene in the treated cells when the level is compared with that of untreated undifferentiated cells.

Clones of which expression levels increased by RA are as follows: HEMBA1000121, HEMBA1000275, HEMBA1000300, HEMBA1000634, HEMBA1000671, HEMBA1000875, HEMBA1001184, HEMBA1001390, HEMBA1001886, HEMBA1002163, HEMBA1002227, HEMBA1002420, HEMBA1002421, HEMBA1003072, HEMBA1003120, HEMBA1003294, HEMBA1003497, HEMBA1004007, HEMBA1004110, HEMBA1004391, HEMBA1004444, HEMBA1005230, HEMBA1005246, HEMBA1005267, HEMBA1005489, HEMBA1005913, HEMBA1006299, HEMBA1006357, HEMBA1006517, HEMBA1006544, HEMBA1006658, HEMBA1006749, HEMBA1007063, HEMBA1007241, HEMBB1000447, HEMBB1000542, HEMBB1000567, HEMBB1000642, HEMBB1000668, HEMBB1001026, HEMBB1001847, HEMBB1002051, HEMBB1002120, HEMBB1002228, HEMBB1002693, MAMMA1000106, MAMMA1000141, MAMMA1000473, MAMMA1000528, MAMMA1000810, MAMMA1000881, MAMMA1001634, MAMMA1001957, MAMMA1002205, MAMMA1002224, NT2RM2000423, NT2RM2000497, NT2RM2000582, NT2RM2001126, NT2RM2001902, NT2RM4000198, NT2RM4000284, NT2RM4000593, NT2RM4001321, NT2RP1000002, NT2RP1000050, NT2RP1000181, NT2RP1000261, NT2RP1000465, NT2RP1000468, NT2RP1000579, NT2RP1000679, NT2RP2000092, NT2RP2000479, NT2RP2000610, NT2RP2000663, NT2RP2000694, NT2RP2000903, NT2RP2001388, NT2RP2001538, NT2RP2001878, NT2RP2001015, NT2RP2002304, NT2RP2002721, NT2RP2002824, NT2RP2002942, NT2RP2002974, NT2RP2002976, NT2RP2003179, NT2RP2003302, NT2RP2003383, NT2RP2003469, NT2RP2003664, NT2RP2003940, NT2RP2004069, NT2RP2004108, NT2RP2004524, NT2RP2004556, NT2RP2004670, NT2RP2005069, NT2RP2005247, NT2RP2005425, NT2RP2005463, NT2RP2005514, NT2RP2005535, NT2RP2005541, NT2RP2005774, NT2RP2005878, NT2RP2005883, NT2RP2005887, NT2RP2006099, NT2RP2006134, NT2RP3000011, NT2RP3000125, NT2RP3000171, NT2RP3000232, NT2RP3000460, NT2RP3000481, NT2RP3000652, NT2RP3000677, NT2RP3000818, NT2RP3000820, NT2RP3001044, NT2RP3001061, NT2RP3001170, NT2RP3001240, NT2RP3001322, NT2RP3001388, NT2RP3001542, NT2RP3001592, NT2RP3001976, NT2RP3002790, NT2RP3002900, NT2RP3002983, NT2RP3003000, NT2RP3003354, NT2RP3003532, NT2RP3003729, NT2RP3003874, NT2RP3003939, NT2RP3004025, NT2RP3004083, NT2RP3004090, NT2RP3004130, NT2RP3004202, NT2RP3004294, NT2RP3004640, NT2RP4000108, NT2RP4000634, NT2RP4002451, NT2RP4002715, OVARC1000090, OVARC1000208, OVARC1000275, OVARC1000553, OVARC1000775, OVARC1000853, OVARC1000873, OVARC1000916, OVARC1000995, OVARC1001030, OVARC1001049, OVARC1001132, OVARC1001596, OVARC1002178, PLACE1000258, PLACE1000442, PLACE1000927, FLACE1000986, PLACE1001100, PLACE1001123, PLACE1001795, PLACE1002518, PLACE1002547, PLACE1002967, PLACE1003407, PLACE1003428, PLACE1003644, PLACE1003839, PLACE1004078, PLACE1004441, PLACE1004450, PLACE1005669, PLACE1005682, PLACE1005736, PLACE1005768, PLACE1005815, PLACE1006073, PLACE1006208, PLACE1007296, PLACE1007626, PLACE1008282, PLACE1008984, PLACE1008985, PLACE1010445, PLACE1011708, PLACE1011978, PLACE4000455, SKNMC1000004, THYRO1000036, THYRO1000580, THYRO1000776, THYRO1000999, THYRO1001063, THYRO1001128, THYRO1001205, THYRO1001327, THYRO1001523, THYRO1001725, THYRO1001770, Y79AA1000207, Y79AA1000226, Y79AA1000270, Y79AA1001056, Y79AA1001062, Y79AA1001090, Y79AA1001727, Y79AA1002213, Y79AA1002381.

Clones of which expression levels decreased by RA are as follows: BNGH41000020, HEMBA1005070, NT2RP2005027, NT2RP3003473, Y79AA1002376.

Clones of which expression levels increase by RA/inhibitor are as follows:
HEMBA1000128, HEMBA1000875, HEMBA1001390, HEMBA1002163, HEMBA1002227, HEMBA1002421, HEMBA1004391, HEMBA1004454, HEMBA1004785, HEMBA1005913, HEMBA1006171, HEMBA1006299, HEMBA1006335, HEMBA1006544, HEMBA1007241, HEMBB1000447, HEMBB1000668, MAMMA1000994, MAMMA1001344, NT2RM2000582, NT2RP1001004, NT2RP2000663, NT2RP2000694, NT2RP2000903, NT2RP2001388, NT2RP2002674, NT2RP2002974, NT2RP2003383, NT2RP2004069, NT2RP2004606, NT2RP2004837, NT2RP2005069, NT2RP2005425, NT2RP2005463, NT2RP2005541, NT2RP2005883, NT2RP2005887, NT2RP3000460, NT2RP3000838, NT2RP3001044, NT2RP3001240, NT2RP3001388, NT2RP3002721, NT2RP3002738, NT2RP3003469, NT2RP3004083, NT2RP3004130, NT2RP3004202, NT2RP3004294, NT2RP3004640, NT2RP4000108, NT2RP4002451, NT2RP4002715, OVARC1000275, OVARC1000467, OVARC1000553, OVARC1000853, OVARC1000873, OVARC1000916, OVARC1000995, OVARC1001030, OVARC1001222, OVARC1001596, OVARC1002058, OVARC1002178, PLACE1000927, PLACE1001123, PLACE1001407, PLACE1001464, PLACE1001564, PLACE1001795, PLACE1002547, PLACE1003407, PLACE1003644, PLACE1003845, PLACE1004441, PLACE1004482, PLACE1005410, PLACE1005601, PLACE1005725, PLACE1005736, PLACE1006093, PLACE1006219, PLACE1006290, PLACE1006716, PLACE1007296, PLACE1007626, PLACE1008359, PLACE1010968, PLACE1011364, PLACE1011824, THYRO1000678, THYRO1000776, THYRO1000999, THYRO1001113, THYRO1001237, THYRO1001523, Y79AA1000226, Y79AA1000888, Y79AA1001430.

Clones of which expression levels decrease by RA/inhibitor are as follows: HEMBA1000349, HEMBA1001297, HEMBA1001878, HEMBA1005070, HEMBA1006482, HEMBB1001959, NT2RM2001939, NT2RP1000981, NT2RP2001469, NT2RP3003473, OVARC1001132, PLACE1001655, Y79AA1000127, Y79AA1002381.

Clones of which expression levels increase in the presence of both RA and RA/inhibitor are as follows: HEMBA1000875, HEMBA1001390, HEMBA1002163, HEMBA1002227, HEMBA1002421, HEMBA1004391, HEMBA1005913, HEMBA1006299, HEMBA1006544, HEMBA1007241, HEMBB1000447, HEMBB1000668, NT2RM2000582, NT2RP2000663, NT2RP2000694, NT2RP2000903, NT2RP2001388, NT2RP2002974, NT2RP2003383, NT2RP2004069, NT2RP2005069, NT2RP2005425, NT2RP2005463, NT2RP2005541, NT2RP2005883, NT2RP2005887, NT2RP3000460, NT2RP3001044, NT2RP3001240, NT2RP3001388, N12RP3004083, NT2RP3004130, NT2RP3004202, NT2RP3004294, NT2RP3004640, NT2RP4000108, NT2RP4002451, NT2RP4002715, OVARC1000275, OVARC1000553, OVARC1000853, OVARC1000873, OVARC1000916, OVARC1000995, OVARC1001030, OVARC1001596, OVARC1002178, PLACE1000927, PLACE1001123, PLACE1001795, PLACE1002547, PLACE1003407, PLACE1003644, PLACE1004441, PLACE1005736, PLACE1007296, PLACE1007626, THYRO1000776, THYRO1000999, THYRO1001523, Y79AA1000226.

Clones of which expression levels decrease in the presence of both RA and RA/inhibitor are as follows:HEMBA1005070 and NT2RP3003473.

These are neurological disease-associated clones.

### Analysis of rheumatoid arthritis-associated genes

The onset of rheumatoid arthritis is thought to be involved in the proliferation of synovial cells covering inner surfaces of joint cavity and in inflammatory reaction resulted from the action of cytokines produced by leukocytes infiltrating into the joint synovial tissues (Rheumatism Information Center.http://www.rheuma-net.or.jp/). Recent studies have also revealed that tissue necrosis factor (TNF)-. participates in the onset (Current opinion in immunology 1999, 11, 657-662). When the expression of a gene exhibits responsiveness to the action of TNF on synovial cells, the gene is considered to be involved in rheumatoid arthritis.

A survey was performed for genes of which expression levels are varied in response to TNF-. in the primary cell culture of synovial tissue. The primary cultured cells of the smooth muscle (Cell Applications) were grown to be confluent in a culture dish, and then, human TNF-. (Boehringer-Mannheim) was added at a final concentration of 10 ng/ml thereto. The culture was further continued for 24 hours.

Total RNA was extracted from the cells by using S.N.A.P.^{(TM)} Total RNA Isolation kit (Invitrogen). The labeling of probe used for hybridization was carried out by using 10.g of the total RNA according to the same methods as described above. The data were obtained in tripricate (n=3). The data of signal value representing gene expression level in the cells in the presence of TNF stimulation were compared with those in the absence of the stimulation. The comparison was performed by statistical treatment of two-sample t-test. Clones with significant difference in the signal distribution were selected under the condition of p<0.05. In this analysis, clones with the difference can be statistically detected even when the signals were low. Accordingly, clones with signal value of 40 or less were also assessed for the selection.

Table 366 shows the expression level of each cDNA in synovial cells cultured in the absence or presence of TNF.

Averaged signal values (M₁, M₂) and sample variances (s₁², s₂²) for each gene were calculated in each of the cells, and then, the pooled sample variances s² were obtained from the sample variances of the two types of cells to be compared. The t-values were determined according to the following formula: t=(M₁-M₂)/s/(1/3+1/3)^{1/2}. When the determined t-value was greater than a t-value at P, which means the probability of significance level, of 0.05 or 0.01 in the t-distribution table with 4 degrees of freedom, the difference was judged to be found in the expression level of the gene between the two types of cells at p<0.05 or p<0.01, respectively. The tables also include the information of an increase (+) or decrease (-) in the expression level of a gene in the stimulated cells when the level is compared with that of unstimulated cells.

Clones of which expression levels are elevated by TNF-. are as follows:
BNGH41000020, HEMBA1000349, HEMBA1000634, HEMBA1000671, HEMBA1000835, HEMBA1000962, HEMBA1002178, HEMBA1002195, HEMBA1002239, HEMBA1002420, HEMBA1002524, HEMBA1002992, HEMBA1003315, HEMBA1003392, HEMBA1003487, HEMBA1003602, HEMBA1004067, HEMBA1004797, HEMBA1005337, HEMBA1005489, HEMBA1006916, HEMBB1000668, HEMBB1000905, HEMBB1001547, HEMBB1001573, HEMBB1002041, HEMBB1002663, MAMMA1000652, MAMMA1000810, MAMMA1001634, MAMMA1002091, MAMMA1002234, NT2RM2000306, NT2RM4000417, NT2RP1000002, NT2RP1000181, NT2RP1000740, NT2RP2000694, NT2RP2001921, NT2RP2002527, NT2RP2004495, NT2RP2004606, NT2RP2005163, NT2RP2005463, NT2RP2006134, NT2RP3000171, NT2RP3000652, NT2RP3001195, NT2RP3001976, NT2RP3003473, NT2RP3003874, NT2RP3004090, NT2RP3004294, NT2RP3004557, NT2RP3004647, NT2RP4000108, NT2RP4001001, NT2RP4001877, OVARC1000090, OVARC1000105, OVARC1000275, OVARC1000439, OVARC1001607, PLACE1000740, PLACE1000927, PLACE1001016, PLACE1001100, PLACE1001464, PLACE1001500, PLACE1001918, PLACE1002095, PLACE1002547, PLACE1003644, PLACE1004519, PLACE1005031, PLACE1005410, PLACE1005736, PLACE1006219, PLACE1006809, PLACE1008716, PLACE1010081, THYRO1001770, Y79AA1000127, Y79AA1000207, Y79AA1000270, Y79AA1000876, Y79AA1001013, Y79AA1001264, Y79AA1001272, Y79AA1001328, Y79AA1001430, Y79AA1001530, Y79AA1001799.

Clones of which expression levels decrease by TNF-. are as follows:
NT2RM4000326, NT2RP1000300, NT2RP2000514, NT2RP2001755, NT2RP2006042, NT2RP3000481, NT2RP3002790. These are rheumatoid arthritis-associated clones.

### EXAMPLE 16

### Search for a signal sequence, transmembrane region and functional domain in deduced amino acid sequences

The deduced amino acid sequences from the full-length nucleotide sequences were examined to predict the presence of a signal sequence in their amino-termini as well as the presence of a transmembrane region. The amino acid sequences were also searched for a protein functional domain (motif). The examinations for a signal sequence in the amino-terminus, for a transmembrane region and for a functional domain were performed by using PSORT [K. Nakai & M. Kanehisa, Genomics, 14:897-911 (1992)], SOSUI [T. Hirokawa et al., Bioinformatics, 14:378-379 (1998)] (Mitsui Knowledge Industry Co., Ltd.) and Pfam (http://www.sanger.ac.uk/Software/Pfam/index.shtml), respectively. When the presence of a signal sequence or a transmembrane region in the amino-terminus was predicted in the amino acid sequence by PSORT or SOSUI, the protein was predicted to be a secretory protein or a transmembrane protein. When the amino acid sequence matched a functional domain in the Pfam search for a functional domain, the function of the protein is predictable based on the matching data, for example, by referring to the functional categories in PROSITE (http://www.expasy.ch/cgi-bin/prosite-list.pl). The functional domain search can be performed by using PROSITE instead of Pfam.

Search results obtained by using the respective software programs are indicated below.

Clones whose deduced amino acid sequences were predicted to have signal sequences by PSORT search are as follows:
HEMBA1000713, HEMBA1002420, HEMBA1002421, HEMBA1003101, HEMBA1004110, HEMBA1006707, HEMBA1006902, HEMBB1001530, HEMBB1001573, HEMBB1001978, HEMBB1002162, HEMBB1002245, HEMBB1002427, MAMMA1000102, MAMMA1000118, MAMMA1000457, MAMMA1001043, MAMMA1001344, MAMMA1001893, MAMMA1002070, MAMMA1002165, MAMMA1002633, NT2RM2000241, NT2RM2000410, NT2RM2001941, NT2RM4001325, NT2RP1001563, NT2RP2001495, NT2RP2002063, NT2RP2002721, NT2RP2003383, NT2RP2003593, NT2RP2003655, NT2RP2003664, NT2RP2004179, NT2RP2004205, NT2RP2004524, NT2RP2005463, NT2RP3000460, NT2RP3001012, NT2RP3001858, NT2RP3002836, NT2RP3003076, NT2RP3003532, NT2RP3004133, NT2RP3004309, NT2RP4001467, NT2RP4002451, OVARC1000298, OVARC1000811, PLACE1000231, PLACE1000740, PLACE1001183, PLACE1001536, PLACE1001564, PLACE1002095, PLACE1002374, PLACE1003839, PLACE1001482, PLACE1005005, PLACE1005250, PLACE1005383, PLACE1005410, PLACE1005544, PLACE1005569, PLACE1006093, PLACE1006277, PLACE1006809, PLACE1007626, PLACE1008359, PLACE1009067, PLACE1010251, PLACE1011236, SKNMC1000004, SKNMC1000014, THYRO1000099, THYRO1000196, THYRO1001237, THYRO1001327, THYRO1001523, THYRO1001702, THYRO1001725, Y79AA1000426, Y79AA1000521, Y79AA1000959, Y79AA1001013, Y79AA1001264, Y79AA1001328, Y79AA1001427, Y79AA1001430, Y79AA1001795, Y79AA1001803, Y79AA1002022,

Clones whose deduced amino acid sequences were predicted to have transmembrane regions by SOSUI search are as follows: BNGH41000091, HEMBA1000121, HEMBA1000349, HEMBA1000477, HEMBA1000713, HEMBA1000940, HEMBA1000962, HEMBA1001221, HEMBA1001228, HEMBA1001621, HEMBA1002167, HEMBA1002195, HEMBA1002227, HEMBA1002421, HEMBA1003101, HEMBA1003392, HEMBA1003530, HEMBA1003732, HEMBA1003945, HEMBA1004391, HEMBA1004454, HEMBA1004797, HEMBA1004982, HEMBA1005449, HEMBA1005522, HEMBA1005545, HEMBA1005698, HEMBA1006171, HEMBA1006299, HEMBA1006311, HEMBA1006335, HEMBA1006357, HEMBA1006430, HEMBA1006724, HEMBA1006960, HEMBB1000407, HEMBB1000447, HEMBB1000567, HEMBB1000679, HEMBB1000905, HEMBB1001026, HEMBB1001407, HEMBB1001573, HEMBB1001978, HEMBB1002041, HEMBB1002162, HEMBB1002245, HEMBB1002427, HEMBB1002693, MAMMA1000102, MAMMA1000106, MAMMA1000118, MAMMA1000141, MAMMA1000204, MAMMA1000226, MAMMA1000457, MAMMA1000473, MAMMA1000591, MAMMA1000681, MAMMA1000810, MAMMA1000986, MAMMA1001043, MAMMA1001141, MAMMA1001237, MAMMA1001344, MAMMA1001893, MAMMA1001957, MAMMA1001978, MAMMA1002070, MAMMA1002091, MAMMA1002095, MAMMA1002633, NT2RM1000580, NT2RM1000855, NT2RM1000858, NT2RM2000410, NT2RM2000565, NT2RM2001626, NT2RM2001939, NT2RM2001941, NT2RM4000444, NT2RM4000587, NT2RM4000648, NT2RM4000997, NT2RM4001325, NT2RM4001735, NT2RM4001768, NT2RM4002352, NT2RP1000050, NT2RP1000181, NT2RP1000261, NT2RP1000300, NT2RP1000448, NT2RP1000551, NT2RP1000613, NT2RP1000981, NT2RP1001563, NT2RP2000479, NT2RP2000533, NT2RP2000649,
NT2RP2000663, NT2RP2000694, NT2RP2000818, NT2RP2000903, NT2RP2001200, NT2RP2001276, NT2RP2001495, NT2RP2001915, NT2RP2001956, NT2RP2002188, NT2RP2002232, NT2RP2002527, NT2RP2002533, NT2RP2002721, NT2RP2002824, NT2RP2002942, NT2RP2002976, NT2RP2003042, NT2RP2003390, NT2RP2003469, NT2RP2003593, NT2RP2003655, NT2RP2003664, NT2RP2003950, NT2RP2004179, NT2RP2004205, NT2RP2004495, NT2RP2004524, NT2RP2004556, NT2RP2004606, NT2RP2004648, NT2RP2004794, NT2RP2005163, NT2RP2005181, NT2RP2005463, NT2RP2005597, NT2RP2005666, NT2RP2005883, NT2RP2005994, NT2RP2006004, NT2RP2006269, NT2RP2006512, NT2RP2006580, NT2RP3000169, NT2RP3000171, NT2RP3000304, NT2RP3000460, NT2RP3000616, NT2RP3000721, NT2RP3000818, NT2RP3000907, NT2RP3000921, NT2RP3001159, NT2RP3001195, NT2RP3001240, NT2RP3001271, NT2RP3001322, NT2RP3001388, NT2RP3001560, NT2RP3001592, NT2RP3001650, NT2RP3001738, NT2RP3002015, NT2RP3002311, NT2RP3002342, NT2RP3002411, NT2RP3002790, NT2RP3002836, NT2RP3002900, NT2RP3002958, NT2RP3003000, NT2RP3003354, NT2FP3003532, NT2RP3003535, NT2RP3003614, NT2RP3004025, NT2RP3004075, NT2RP3004083, NT2RP3004090, NT2RP3004130, NT2RP3004294, NT2RP3004309, NT2RP3004345, NT2RP3004406, NT2RP3004481, NT2RP3004552, NT2RP4001001, NT2RP4001009, NT2RP4001467, NT2RP4001879, NT2RP4002187, NT2RP4002451, NT2RP4002750, OVARC1000003, OVARC1000105, OVARC1000307, OVARC1000439, OVARC1000553, OVARC1001030, OVARC1001336,
OVARC1001570, PLACE1000231, PLACE1000560, PLACE1000740, PLACE1000912, PLACE1000914, PLACE1000927, PLACE1001016, PLACE1001183, PLACE1001231, PLACE1001401, PLACE1001407, PLACE1001464, PLACE1001536, PLACE1001564, PLACE1001655, PLACE1001836, PLACE1001918, PLACE1001949, PLACE1002518, PLACE1002726, PLACE1002967, PLACE1003573, PLACE1003737, PLACE1003839, PLACE1003845, PLACE1003852, PLACE1004279, PLACE1004282, PLACE1004441, PLACE1004637, PLACE1004648, PLACE1004816, PLACE1004887, PLACE1005003, PLACE1005005, PLACE1005410, PLACE1005544, PLACE1005569, PLACE1005660, PLACE1005725, PLACE1005745, PLACE1005927, PLACE1006290, PLACE1006443, PLACE1006959, PLACE1007096, PLACE1007296, PLACE1007626, PLACE1007881, PLACE1008359, PLACE1008469, PLACE1008716, PLACE1008985, PLACE1009196, PLACE1009279, PLACE1009527, PLACE1009546, PLACE1009600, PLACE1010011, PLACE1010078, PLACE1010445, PLACE1010713, PLACE1010784, PLACE1010968, PLACE1011236, PLACE1011516, PLACE3000181, THYRO1000400, THYRO1000678, THYRO1000776, THYRO1000956, THYRO1001102, THYRO1001113, THYRO1001205, THYRO1001237, THYRO1001242, THYRO1001266, THYRO1001327, THYRO1001478, THYRO1001523, THYRO1001641, THYRO1001702, THYRO1001725, Y79AA1000207, Y79AA1000226, Y79AA1000270, Y79AA1000521, Y79AA1000888, Y79AA1001013, Y79AA1001212, Y79AA1001264, Y79AA1001328, Y79AA1001426, Y79AA1001427, Y79AA1001727, Y79AA1001787, Y79AA1001795, Y79AA1001803, Y79AA1002058,
Y79AA1002129, Y79AA1002213, Y79AA1002373,

Names of clones whose deduced amino acid sequences were predicted to have functional domains by Pfam search, and names of the matched functional domains are shown below. When multiple functional domains matched a clone, each domain name was indicated, separated by a double-slash mark,//.
HEMBA1000006//Src homology domain 3
HEMBA1000128//SCP-like extracellular Proteins
HEMBA1000349//ABC transporters
HEMBA1000462//RNA recognition motif. (aka RRM, RBD, or RNP domain)
HEMBA1000590//EGF-like domain//von Willebrand factor type A domain
HEMBA1000671//Zinc finger, C2H2 type
HEMBA1000732//EGF-like domain
HEMBA1000940//Connexin
HEMBA1001221//EGF-like domain//Kazal-type serine protease inhibitor domain
HEMBA1001621//7 transmembrane receptor (rhodopsin family)
HEMBA1001878//WD domain, G-beta repeats
HEMBA1002048//Zinc finger, C2H2 type
HEMBA1002167//Carboxylesterases
HEMBA1002551//WD domain, G-beta repeats
HEMBA1002992//Ubiquitin family
HEMBA1003047//CUB domain
HEMBA1003120//Zinc finger, C2H2 type
HEMBA1003230//EGF-like domain
HEMBA1003392//Low-density lipoprotein receptor domain class A
HEMBA1003497//Zinc finger, C2H2 type
HEMBA1004250//Cadherin
HEMBA1004391//Fibronectin type III domain//IG superfamily
HEMBA1004454//4 transmembrane segments integral membrane proteins
HEMBA1004785!/'chromo' (CHRromatin Organization MOdifier) domain
HEMBA1005246//Zinc finger, C2H2 type
HEMBA1005267//Ank repeat
HEMBA1005545//7 transmembrane receptor (rhodopsin family)
HEMBA1005929//Eukaryotic protein kinase domain
HEMBA1005945//Mitochondrial carrier proteins
HEMBA1006572//Zinc finger, C2H2 type
HEMBA1006707//EGF-like domain//von Willebrand factor type A domain
HEMBA1006749//EGF-like domain//von Willebrand factor type A domain
HEMBA1006770//RNA recognition motif. (aka RRM, RBD, or RNP domain)
HEMBA1006902//EGF-like domain//von Willebrand factor type A domain
HEMBB1000106//Zinc finger, CCHC class
HEMBB1000668//WD domain, G-beta repeats
HEMBB1000881//Thrombospondin type 1 domain
HEMBB1000905//WD domain, G-beta repeats
HEMBB1002041//EGF-like domain//Kazal-type serine protease inhibitor domain
HEMBB1002245//IG superfamily
HEMBB1002302//Zinc finger, CCHC class
HEMBB1002465//Acyl-CoA dehydrogenases
HEMBB1002661//Helix-loop-helix DNA-binding domain
MAMMA1000204//C2 domain
MAMMA1000457//FAD/NAD-binding domain in oxidoreductases
MAMMA1000681//7 transmembrane receptor (rhodopsin family)
MAMMA1000881//Eukaryotic protein kinase domain//Protein kinase C terminal domain
MAMMA1001150//Phorbol esters / diacylglycerol binding domain//Eukaryotic protein kinase domain
MAMMA1001310//WD domain, G-beta repeats
MAMMA1001532//Zinc finger, C2H2 type
MAMMA1001615//Helix-loop-helix DNA-binding domain
MAMMA1002070//Kringle domain
MAMMA1002080//Ras family (contains ATP/GTP binding P-loop)
MAMMA1002095//E1-E2 ATPases
MAMMA1002165//Insulin-like growth factor binding proteins
NT2RM1000789//HMG (high mobility group) box
NT2RM1000855//eubacterial secY protein
NT2RM1000899//Mitochondrial carrier proteins
NT2RM2000589//PH (pleckstrin homology) domain
NT2RM2000632//Helicases conserved C-terminal domain
NT2RM2001792//Fibrinogen beta and gamma chains, C-terminal globular domain
NT2RM2001902//Eukaryotic protein kinase domain
NT2RM2001939//7 transmembrane receptor (rhodopsin family)
NT2RM2001941//7 transmembrane receptor (rhodopsin family)
NT2RM4000284//Class I Histocompatibility antigen, domains alpha 1 and 2
NT2RM4000326//Zinc finger, C2H2 type
NT2RM4000417//C2 domain
NT2RM4000444//ABC transporters
NT2RM4001377//PH (pleckstrin homology) domain
NT2RM4001768//Alcohol/other dehydrogenases, short chain type
NT2RM4002352//Low-density lipoprotein receptor domain class A
NT2RP1000181//Heme-binding domain in cytochrome b5 and oxidoreductases
NT2RP1000271//Zinc finger, C2H2 type
NT2RP1000325//Mitochondrial carrier proteins
NT2RP1000613//Eukaryotic-type carbonic anhydrases
NT2RP1000981//IG superfamily
NT2RP1001004//Thrombospondin type 1 domain
NT2RP1001020//Eukaryotic protein kinase domain
NT2RP1001031//WD domain, G-beta repeats
NT2RP1001563//EGF-like domain//Lectin C-type domain short and long forms//SCP-like extracellular Proteins
NT2RP2000092//Zinc finger, C2H2 type
NT2RP2000514//Fibronectin type III domain//IG superfamily
NT2RP2000649//Zinc-binding metalloprotease domain
NT2RP2000712//Zinc finger, C2H2 type
NT2RP2000739//Zinc finger, C2H2 type
NT2RP2001514//E1-E2 ATPases
NT2RP2001529//Eukaryotic protein kinase domain
NT2RP2001755//Thrombospondin type 1 domain
NT2RP2001769//Eukaryotic protein kinase domain
NT2RP2002188//Carboxylesterases
NT2RP2002527//Heme-binding domain in cytochrome b5 and oxidoreductases
NT2RP2002564//Zinc finger, C2H2 type
NT2RP2002942//IG superfamily
NT2RP2003179//Eukaryotic protein kinase domain
NT2RP2003302//Zinc finger, C2H2 type
NT2RP2003390//DnaJ, prokaryotic heat shock protein
NT2RP2003469//Sugar (and other) transporters
NT2RP2003545//Eukaryotic protein kinase domain
NT2RP2003593//Thioredoxins
NT2RP2003940//Zinc finger, C2H2 type
NT2RP2004108//Zinc finger, C2H2 type
NT2RP2004205//IG superfamily
NT2RP2004670//Eukaryotic protein kinase domain
NT2RP2004847//Zinc finger, C2H2 type
NT2RP2005181//Amino acid permeases
NT2RP2005247//Zinc finger, C3HC4 type (RING finger)
NT2RP2005391//Fibronectin type III domain
NT2RP2005535//Zinc finger, C2H2 type
NT2RP2005774//Zinc finger, C2H2 type
NT2RP2005878//Alcohol/other dehydrogenases, short chain type
NT2RP2005941//Homeobox domain//'Paired box' domain
NT2RP2006004//Fibronectin type III domain
NT2RP3000011//WD domain, G-beta repeats
NT2RP3000022//Eukaryotic protein kinase domain
NT2RP3000063//Zinc finger, C2H2 type
NT2RP3000148//Zinc finger, C2H2 type
NT2RP3000172//Eukaryotic protein kinase domain
NT2RP3000201//Eukaryotic protein kinase domain
NT2RP3000232//Zinc finger, C2H2 type
NT2RP3000304//Low-density lipoprotein receptor domain class A//Low-density lipoprotein receptor domain class B
NT2RP3000436//Thioredoxins
NT2RP3000460//eubacterial secY protein
NT2RP3000616//Fibronectin type III domain
NT2RP3000652//Zinc finger, C2H2 type
NT2RP3000676//Mitochondrial carrier proteins
NT2RP3000789//KH domain family of RNA binding proteins
NT2RP3000820//WD domain, G-beta repeats
NT2RP3000838//PH (pleckstrin homology) domain
NT2RP3000907//E1-E2 ATPases
NT2RP3000921//IG superfamily
NT2RP3001195//Sugar (and other) transporters
NT2RP3001240//eubacterial secY protein
NT2RP3001388//C2 domain
NT2RP3001650//CUB domain//Low-density lipoprotein receptor domain class A
NT2RP3001738//Heme-binding domain in cytochrome b5 and oxidoreductases
NT2RP3001976//Zinc finger, C2H2 type
NT2RP3002281//RNA recognition motif. (aka RRM, RBD, or RNP domain)
NT2RP3002411//Alcohol/other dehydrogenases, short chain type
NT2RP3002721//Citrate synthase
NT2RP3003000//Ion transport proteins
NT2RP3003527//Eukaryotic protein kinase domain
NT2RP3003535//TPR Domain
NT2RP3003849//C2 domain
NT2RP3004067//Src homology domain 3
NT2RP3004090//Zinc finger, C3HC4 type (RING finger)
NT2RP3004481//IG superfamily
NT2RP3004552//CUB domain//Sushi domain
NT2RP3004647//Mitochondrial carrier proteins
NT2RP4000108//Intermediate filament proteins
NT2RP4000634//Eukaryotic protein kinase domain
NT2RP4000962//Eukaryotic protein kinase domain
NT2RP4001009//Zinc-binding metalloprotease domain
NT2RP4001877//RNA recognition motif. (aka RRM, RBD, or RNP domain)
NT2RP4002187//Alcohol/other dehydrogenases, short chain type
NT2RP4002750//Amino acid permeases
OVARC1000105//Ubiquitin-conjugating enzymes
OVARC1000255//Eukaryotic protein kinase domain
OVARC1000313//Thioredoxins
OVARC1000410//Fibrinogen beta and gamma chains, C-terminal globular domain
OVARC1000529//Eukaryotic protein kinase domain
OVARC1000811//CUB domain//Kringle domain
OVARC1000916//Eukaryotic protein kinase domain
OVARC1001049//Helix-loop-helix DNA-binding domain
OVARC1001338//Eukaryotic protein kinase domain
OVARC1001569//Eukaryotic protein kinase domain
OVARC1001570//Eukaryotic aspartyl proteases
PLACE1000231//WAP-type (Whey Acidic Protein) 'four-disulfide core'
PLACE1000258//Zinc finger, C2H2 type
PLACE1000740//EGF-like domain
PLACE1000907//Zinc finger, C2H2 type
PLACE1001016//Ion transport proteins
PLACE1001500//Helicases conserved C-terminal domain
PLACE1001655//Ion transport proteins
PLACE1001795//SCP-like extracellular Proteins
PLACE1001949//E1-E2 ATPases
PLACE1002329//Src homology domain 3
PLACE1002355//Alpha-2-macroglobulin family//Kazal-type serine protease inhibitor domain
PLACE1002374//Cysteine proteases
PLACE1002518//Zinc finger, C3HC4 type (RING finger)
PLACE1002911//IG superfamily
PLACE1003135//Eukaryotic protein kinase domain
PLACE1003163//Enoyl-CoA hydratase/isomerase
PLACE1003573//Lectin C-type domain short and long forms
PLACE1004166//Bromodomain
PLACE1004305//Ras family (contains ATP/GTP binding P-loop)
PLACE1004441//7 transmembrane receptor (rhodopsin family)
PLACE1004520//IG superfamily
PLACE1004816//Fibrinogen beta and gamma chains, C-terminal globular domain
PLACE1004887//Zinc finger, C3HC4 type (RING finger)
PLACE1005003//Trypsin
PLACE1005383//EGF-like domain
PLACE1005410//eubacterial secY protein
PLACE1005426//IG superfamily
PLACE1005519//Eukaryotic protein kinase domain
PLACE1005539//Heat shock hsp20 proteins
PLACE1005544//IG superfamily
PLACE1005569//IG superfamily
PLACE1005682//Zinc finger, C3HC4 type (RING finger)
PLACE1005736//PH (pleckstrin homology) domain
PLACE1006079//Homeobox domain
PLACE1006716//C1q domain
PLACE1008282//Eukaryotic protein kinase domain
PLACE1008549//Ets-domain
PLACE1008744//EGF-like domain//Sushi domain
PLACE1009067//Src homology domain 3
PLACE1010081//Eukaryotic protein kinase domain
PLACE1010251//EGF-like domain
PLACE1010713//Alcohol/other dehydrogenases, short chain type
PLACE1010784//7 transmembrane receptor (rhodopsin family)
PLACE1010968//Fibronectin type III domain
PLACE1011181//ATPases associated with various cellular activities (AAA)
PLACE1011364//Eukaryotic protein kinase domain
PLACE1011407//Zinc finger, C2H2 type
PLACE1011708//CUB domain
PLACE1011824//Eukaryotic protein kinase domain
PLACE1011978//Zinc finger, C2H2 type
PLACE3000181//Cadherin
PLACE3000213//Sushi domain
PLACB4000354//BGF-like domain//Sushi domain
SKNMC1000082//Mitochondrial carrier proteins
THYRO1000196//Cadherin
THYRO1000580//Zinc finger, C2H2 type
THYRO1000678//Connexin
THYRO1000795//Mitochondrial carrier proteins
THYRO1000956//7 transmembrane receptor (rhodopsin family)
THYRO1001113//C2 domain
THYRO1001266//Sodium:solute symporter family
THYRO1001457//Phorbol esters / diacylglycerol binding domain//Bukaryotic protein kinase domain
THYRO1001478//EF hand
THYRO1001593//Eukaryotic protein kinase domain
THYRO1001700//Eukaryotic protein kinase domain
THYRO1001770//Eukaryotic protein kinase domain
Y79AA1000030//WW/rsp5/WWP domain containing proteins
Y79AA1000426//Transforming growth factor beta like domain
Y79AA1000777//WD domain, G-beta repeats
Y79AA1000876//Thioredoxins
Y79AA1000967//Eukaryotic protein kinase domain
Y79AA1001090//Ank repeat
Y79AA1001264//DnaJ, prokaryotic heat shock protein
Y79AA1001328//EGF-like domain
Y79AA1001427//FAD/NAD-binding domain in oxidoreductases
Y79AA1001523//Bromodomain//Zinc finger, C3HC4 type (RING finger)
Y79AA1001530//Tubulin
Y79AA1001727//IG superfamily
Y79AA1001787//E1-E2 ATPases
Y79AA1001799//Mitochondrial carrier proteins
Y79AA1002022//IG superfamily
Y79AA1002381//Eukaryotic protein kinase domain

### EXAMPLE 17

### Functional categories based on the full-length nucleotide sequences

Prediction of functions of proteins encoded by the clones and the categorization thereof were performed based on the results of homology search (see homology search result 10) of the databases, GenBank, Swiss-Prot and UniGene for the full-length nucleotide sequences of 826 clones as well as based on the results of domain search (see Example 16) of the deduced amino acid sequences encoded by the full-length nucleotide sequences. (HEMBA1005337, NT2RM1000407, NT2RM2001767, and NT2RP3003939 were excluded because of the absence of full-length sequence.)

There are 611 clones that presumably encode proteins belonging to any of categories of secretory and/or membrane proteins, glycoprotein-associated proteins, signal transduction-associated proteins, transcription-associated proteins and disease-associated proteins.

The clones presumably encoding proteins categorized into secretory and/or membrane proteins are those which matched the full-length sequences of Swiss-Prot database with keywords "growth factor", "cytokine", "hormone", "signal", "transmembrane", "membrane", "extracellular matrix", "receptor", "G-protein coupled receptor", "ionic channel", "voltage-gated channel", "calcium channel", "cell adhesion", "collagen" or "connective tissue"; those which matched the data, suggesting that the proteins are secretory and/or membrane proteins; or those which matched the full-length sequences of GenBank or UniGene database with similar description; and, further, those predicted to have an N-terminal signal sequence or a transmembrane region as a result of domain search for the amino acid sequences deduced from the full-length nucleotide sequences.

The clones presumably encoding proteins categorized into glycoprotein-associated proteins are those which matched the full-length sequences of Swiss-Prot database with the keywords "glycoprotein"; those which matched the data, suggesting that the proteins are glycoprotein; or those which matched the full-length sequences of GenBank or UniGene database.

The clones presumably encoding proteins categorized into signal transduction-associated proteins are those which matched the full-length sequences of Swiss-Prot database with the keywords "serine/threonine-protein kinase", "tyrosine-protein kinase" or "SH3 domain"; those which matched the data, suggesting that the proteins are signal transduction-associated proteins (for example, "ADP-ribosylation factor"); or those which matched the the full-length sequences of GenBank or UniGene database with similar description.

The clones presumably encoding proteins categorized into transcription-associated proteins are those which matched the full-length sequences of Swiss-Prot database with the keywords "transcription regulation", "zinc finger" or "homeobox"; those which matched the data, suggesting that the proteins are transcription-associated proteins; or those which matched the full-length sequences of GenBank or UniGene database with similar description.

The clones presumably encoding proteins categorized into disease-associated proteins are those which matched the full-length sequences of Swiss-Prot database with the keywords "disease mutation" or "syndrome"; those which matched the data, suggesting that the proteins are disease-associated proteins; or those which matched the full-length sequences of Swiss-Prot database and GenBank or UniGene database where the matched sequences are those of genes or proteins which had been deposited in the database of Online Mendelian Inheritance in Man (OMIM) (http:I/www.ncbi.nlm.nih.gov/Omim/), which is a database of human genes and diseases.

The following 437 clones were categorized into secretory and/or membrane proteins.
BNGH41000020, BNGH41000087, BNGH41000091, HEMBA1000121, HEMBA1000128, HEMBA1000349, HEMBA1000477, HEMBA1000590, HEMBA1000713, HEMBA1000732, HEMBA1000745, HEMBA1000835, HEMBA1000940, HEMBA1000962, HEM8A1001221, HEMBA1001228, HEMBA1001621, HEMBA1002131, HEMBA1002163, HEMBA1002167, HEMBA1002178, HEMBA1002195, HEMBA1002227, HEMBA1002420, HEMBA1002421, HEMBA1002767, HEMBA1003047, HEMBA1003101, HEMBA1003230, HEMBA1003392, HEMBA1003530, HEMBA1003602, HEMBA1003732, HEMBA1003945, HEMBA1004110, HEMBA1004250, HEMBA1004391, HEMBA1004444, HEMBA1004454, HEMBA1004505, HEMBA1004797, HEMBA1004982, HEMBA1005070, HEMBA1005449, HEMBA1005522, HEMBA1005545, HEMBA1005698, HEMBA1005945, HEMBA1006171, HEMBA1006299, HEMBA1006311, HEMBA1006335, HEMBA1006357, HEMBA1006430, HEMBA1006482, HEMBA1006707, HEMBA1006724, HEMBA1006749, HEMBA1006902, HEMBA1006960, HEMBA1007241, HEMBB1000407, HEMBB1000447, HEMBB1000567, HEMBB1000679, HEMBB1000881, HEMBB1001026, HEMBB1001048, HEMBB1001407, HEMBB1001530, HEMBB1001573, HEMBB1001847, HEMBB1001978, HEMBB1002041, HEMBB1002162, HEMBB1002245, HEMBB1002427, HEMBB1002693, MAMMA1000102, MAMMA1000106, MAMMA1000118, MAMMA1000141, MAMMA1000204, MAMMA1000226, MAMMA1000457, MAMMA1000473, MAMMA1000496, MAMMA1000591, MAMMA1000681, MAMMA1000810, MAMMA1000986, MAMMA1000994, MAMMA1001043, MAMMA1001141, MAMMA1001237, MAMMA1001344, MAMMA1001418, MAMMA1001893, MAMMA1001957, MAMMA1001978,
MAMMA1002070, MAMMA1002091, MAMMA1002095, MAMMA1002165, MAMMA1002234, MAMMA1002586, MAMMA1002633, MAMMA1003126, NT2RM1000462, NT2RM1000542, NT2RM1000580, NT2RM1000855, NT2RM1000858, NT2RM1000899, NT2RM2000241, NT2RM2000410, NT2RM2000423, NT2RM2000565, NT2RM2001626, NT2RM2001792, NT2RM2001939, NT2RM2001941, NT2RM4000198, NT2RM4000284, NT2RM4000417, NT2RM4000444, NT2RM4000587, NT2RM4000593, NT2RM4000648, NT2RM4000761, NT2RM4000997, NT2RM4001325, NT2RM4001735, NT2RM4001768, NT2RM4001843, NT2RM4002352, NT2RP1000050, NT2RP1000181, NT2RP1000261, NT2RP1000300, NT2RP1000325, NT2RP1000448, NT2RP1000551, NT2RP1000613, NT2RP1000981, NT2RP1001004, NT2RP1001563, NT2RP2000479, NT2RP2000533, NT2RP2000616, NT2RP2000649, NT2RP2000663, NT2RP2000694, NT2RP2000818, NT2RP2000903, NT2RP2001200, NT2RP2001276, NT2RP2001480, NT2RP2001495, NT2RP2001514, NT2RP2001755, NT2RP2001915, NT2RP2001956, NT2RP2002063, NT2RP2002188, NT2RP2002232, NT2RP2002527, NT2RP2002533, NT2RP2002721, NT2RP2002824, NT2RP2002942, NT2RP2002976, NT2RP2003042, NT2RP2003210, NT2RP2003383, NT2RP2003390, NT2RP2003469, NT2RP2003593, NT2RP2003655, NT2RP2003664, NT2RP2003950, NT2RP2004179, NT2RP2004205, NT2RP2004495, NT2RP2004524, NT2RP2004556, NT2RP2004606, NT2RP2004648, NT2RP2004794, NT2RP2005027, NT2RP2005163, NT2RP2005181, NT2RP2005378, NT2RP2005463, NT2RP2005541, NT2RP2005597, NT2RP2005666, NT2RP2005883, NT2RP2005994, NT2RP2006004,
NT2RP2006042, NT2RP2006269, NT2RP2006512, NT2RP2006580, NT2RP3000169, NT2RP3000171, NT2RP3000304, NT2RP3000436, NT2RP3000460, NT2RP3000616, NT2RP3000676, NT2RP3000721, NT2RP3000818, NT2RP3000907, NT2RP3000921, NT2RP3001012, NT2RP3001159, NT2RP3001195, NT2RP3001240, NT2RP3001271, NT2RP3001322, NT2RP3001388, NT2RP3001560, NT2RP3001592, NT2RP3001650, NT2RP3001738, NT2RP3001858, NT2RP3002015, NT2RP3002160, NT2RP3002311, NT2RP3002342, NT2RP3002411, NT2RP3002737, NT2RP3002790, NT2RP3002836, NT2RP3002900, NT2RP3002958, NT2RP3003000, NT2RP3003076, NT2RP3003354, NT2RP3003532, NT2RP3003535, NT2RP3003614, NT2RP3004025, NT2RP3004075, NT2RP3004083, NT2RP3004130, NT2RP3004133, NT2RP3004309, NT2RP3004345, NT2RP3004406, NT2RP3004481, NT2RP3004552, NT2RP3004625, NT2RP3004647, NT2RP4001001, NT2RP4001009, NT2RP4001467, NT2RP4001879, NT2RP4002187, NT2RP4002451, NT2RP4002750, OVARC1000003, OVARC1000105, OVARC1000298, OVARC1000307, OVARC1000313, OVARC1000410, OVARC1000439, OVARC1000553, OVARC1000811, OVARC1000873, OVARC1000956, OVARC1001030, OVARC1001163, OVARC1001336, OVARC1001570, OVARC1001607, OVARC1001725, OVARC1001991, PLACE1000033, PLACE1000231, PLACE1000560, PLACE1000740, PLACE1000912, PLACE1000914, PLACE1000927, PLACE1001016, PLACE1001123, PLACE1001183, PLACE1001231, PLACE1001340, PLACE1001401, PLACE1001407, PLACE1001464, PLACE1001516, PLACE1001536, PLACE1001564, PLACE1001655, PLACE1001795,
PLACE1001836, PLACE1001918, PLACE1001949, PLACE1002080, PLACE1002095, PLACE1002355, PLACE1002374, PLACE1002518, PLACE1002547, PLACE1002726, PLACE1002905, PLACE1002911, PLACE1002967, PLACE1003407, PLACE1003573, PLACE1003737, PLACE1003772, PLACE1003839, PLACE1003845, PLACE1003852, PLACE1004279, PLACE1004282, PLACE1004441, PLACE1004450, PLACE1004482, PLACE1004520, PLACE1004630, PLACE1004637, PLACE1004648, PLACE1004816, PLACE1005003, PLACE1005005, PLACE1005031, PLACE1005383, PLACE1005410, PLACE1005426, PLACE1005544, PLACE1005569, PLACE1005660, PLACE1005725, PLACE1005745, PLACE1005878, PLACE1005927, PLACE1006071, PLACE1006093, PLACE1006208, PLACE1006277, PLACE1006290, PLACE1006443, PLACE1006716, PLACE1006809, PLACE1006959, PLACE1007081, PLACE1007096, PLACE1007296, PLACE1007626, PLACE1007845, PLACE1007881, PLACE1008359, PLACE1008469, PLACE1008716, PLACE1008744, PLACE1008985, PLACE1009067, PLACE1009196, PLACE1009279, PLACE1009527, PLACE1009546, PLACE1009600, PLACE1009982, PLACE1010011, PLACE1010078, PLACE1010251, PLACE1010445, PLACE1010713, PLACE1010784, PLACE1010827, PLACE1010968, PLACE1011116, PLACE1011181, PLACE1011236, PLACE1011516, PLACE1011708, PLACE3000181, PLACE3000213, PLACE4000354, SKNMC1000004, SKNMC1000014, SKNMC1000082, THYRO1000036, THYRO1000099, THYRO1000196, THYRO1000400, THYRO1000584, THYRO1000678, THYRO1000776, THYRO1000795, THYRO1000956, THYRO1001102, THYRO1001113,
THYRO1001205, THYRO1001237, THYRO1001242, THYRO1001266, THYRO1001327, THYRO1001456, THYRO1001478, THYRO1001523, THYRO1001529, THYRO1001641, THYRO1001702, THYRO1001725, Y79AA1000207, Y79AA1000226, Y79AA1000270, Y79AA1000426, Y79AA1000521, Y79AA1000876, Y79AA1000888, Y79AA1000959, Y79AA1001013, Y79AA1001212, Y79AA1001264, Y79AA1001328, Y79AA1001426, Y79AA1001427, Y79AA1001430, Y79AA1001727, Y79AA1001787, Y79AA1001795, Y79AA1001799, Y79AA1001803, Y79AA1002022, Y79AA1002058, Y79AA1002129, Y79AA1002213, Y79AA1002373,

The following 146 clones were categorized into glycoprotein-associated proteins.
BNGH41000087, BNGH41000091, HEMBA1000349, HEMBA1000590, HEMBA1000745, HEMBA1000835, HEMBA1001221, HEMBA1001228, HEMBA1001621, HEMBA1002131, HEMBA1002178, HEMBA1002421, HEMBA1002767, HEMBA1003230, HEMBA1003392, HEMBA1004250, HEMBA1004391, HEMBA1004444, HEMBA1004505, HEMBA1005449, HEMBA1005522, HEMBA1005545, HEMBA1006707, HEMBA1006749, HEMBA1006902, HEMBB1000679, HEMBB1000881, HEMBB1001048, HEMBB1002120, HEMBB1002245, HEMBB1002427, MAMMA1000102, MAMMA1000591, MAMMA1000681, MAMMA1001043, MAMMA1001237, MAMMA1002070, MAMMA1002586, MAMMA1003126, NT2RM1000462, NT2RM1000580, NT2RM2001792, NT2RM2001818, NT2RM2001939, NT2RM2001941, NT2RM4000198, NT2RM4000284, NT2RM4000417, NT2RM4000648, NT2RM4000997, NT2RM4001325, NT2RM4002352, NT2RP1000613, NT2RP1000981, NT2RP1001004, NT2RP2000616, NT2RP2000694, NT2RP2000903, NT2RP2001480, NT2RP2001755, NT2RP2002533, NT2RP2003042, NT2RP2003210, NT2RP2004205, NT2RP2004606, NT2RP2005027, NT2RP2005181, NT2RP2005541, NT2RP2005597, NT2RP2005883, NT2RP2006004, NT2RP2006042, NT2RP2006269, NT2RP3000304, NT2RP3000616, NT2RP3000921, NT2RP3001650, NT2RP3002160, NT2RP3002737, NT2RP3002958, NT2RP3003000, NT2RP3003532, NT2RP3004130, NT2RP3004133, NT2RP3004481, NT2RP3004552, NT2RP3004640, NT2RP4000108, NT2RP4001467, NT2RP4002750, OVARC1000003, OVARC1000553, OVARC1000811, OVARC1000873, OVARC1001336, OVARC1001607, OVARC1001991, PLACE1000033, PLACE1000740, PLACE1001016,
PLACE1001123, PLACE1001231, PLACE1001464, PLACE1001655, PLACE1001836, PLACE1002355, PLACE1002374, PLACE1002905, PLACE1002911, PLACE1003573, PLACE1003737, PLACE1003772, PLACE1003839, PLACE1004282, PLACE1004441, PLACE1004450, PLACE1004520, PLACE1004648, PLACE1005003, PLACE1005426, PLACE1006071, PLACE1006073, PLACE1006290, PLACE1007081, PLACE1007845, PLACE1008716, PLACE1008744, PLACE1008985, PLACE1010251, PLACE1010784, PLACE1010968, PLACE1011116, PLACE3000181, PLACE3000213, PLACE4000354, THYRO1000036, THYRO1000196, THYRO1000584, THYRO1000956, THYRO1001266, Y79AA1000270, Y79AA1000426, Y79AA1001727, Y79AA1001795, Y79AA1002022, Y79AA1002213,

The following 55 clones were categorized into signal transduction-associated proteins.
HEMBA1000006, HEMBA1002195, HEMBA1002227, HEMBA1002551, HEMBA1005084, HEMBA1005929, HEMBA1006658, HEMBA1006916, MAMMA1000881, MAMMA1001150, MAMMA1001310, MAMMA1002142, NT2RM2001902, NT2RP1001020, NT2RP1001031, NT2RP2001469, NT2RP2001529, NT2RP2001769, NT2RP2003179, NT2RP2003545, NT2RP2004670, NT2RP3000011, NT2RP3000022, NT2RP3000172, NT2RP3000201, NT2RP3000820, NT2RP3003527, NT2RP3003849, NT2RP3003874, NT2RP3004067, NT2RP4000634, NT2RP4000962, OVARC1000255, OVARC1000529, OVARC1000916, OVARC1001338, OVARC1001569, PLACE1002329, PLACE1003135, PLACE1003598, PLACE1005519, PLACE1006208, PLACE1008282, PLACE1008297, PLACE1010081, PLACE1011364, PLACE1011824, THYRO1001457, THYRO1001593, THYRO1001700, THYRO1001770, Y79AA1000777, Y79AA1000967, Y79AA1002376, Y79AA1002381,

The following 80 clones were categorized into transcription -associated proteins.
HEMBA1000462, HEMBA1000671, HEMBA1001297, HEMBA1001390, HEMBA1001886, HEMBA1002048, HEMBA1003120, HEMBA1003497, HEMBA1004785, HEMBA1005230, HEMBA1005246, HEMBA1006276, HEMBA1006572, HEMBA1007226, HEMBB1000106, HEMBB1000905, HEMBB1001959, HEMBB1002051, HEMBB1002661, MAMMA1001094, MAMMA1001532, MAMMA1001615, NT2RM1000789, NT2RM2000632, NT2RM2000773, NT2RM4000326, NT2RP1000271, NT2RP1000468, NT2RP2000092, NT2RP2000610, NT2RP2000712, NT2RP2000739, NT2RP2001538, NT2RP2001662, NT2RP2001817, NT2RP2001948, NT2RP2002564, NT2RP2002974, NT2RP2003138, NT2RP2003302, NT2RP2003940, NT2RP2004108, NT2RP2004847, NT2RP2005247, NT2RP2005391, NT2RP2005535, NT2RP2005774, NT2RP2005941, NT2RP2006092, NT2RP3000148, NT2RP3000232, NT2RP3000378, NT2RP3000652, NT2RP3001976, NT2RP3004090, NT2RP3004119, NT2RP3004294, OVARC1001049, OVARC1001086, OVARC1001132, OVARC1001807, PLACE1000258, PLACE1000442, PLACE1000907, PLACE1003529, PLACE1004166, PLACE1004168, PLACE1004887, PLACE1005250, PLACE1005682, PLACE1006079, PLACE1008549, PLACE1011407, PLACE1011978, THYRO1000580, Y79AA1000030, Y79AA1001090, Y79AA1001523, Y79AA1002334, Y79AA1002378,

The following 85 clones were categorized into disease-associated proteins.
BNGH41000020, HEMBA1000349, HEMBA1000590, HEMBA1000671, HEMBA1000835, HEMBA1001184, HEMBA1001228, HEMBA1001886, HEMBA1003120, HEMBA1004250, HEMBA1005246, HEMBA1005267, HEMBA1006707, HEMBA1006749, HEMBA1006902, HEMBA1006916, HEMBA1007013, HEMBB1002120, MAMMA1000204, MAMMA1002080, NT2RM2000632, NT2RM2001126, NT2RM2001558, NT2RP1000271, NT2RP1000465, NT2RP1000579, NT2RP2000447, NT2RP2000514, NT2RP2000739, NT2RP2001223, NT2RP2001529, NT2RP2001562, NT2RP2002674, NT2RP2003369, NT2RP2004108, NT2RP2004205, NT2RP2005535, NT2RP2005941, NT2RP2006004, NT2RP3000059, NT2RP3000125, NT2RP3000201, NT2RP3000232, NT2RP3000616, NT2RP3000677, NT2RP3000838, NT2RP3000921, NT2RP3001542, NT2RP3002286, NT2RP3002721, NT2RP3002737, NT2RP3002738, NT2RP3004481, OVARC1000208, OVARC1000275, OVARC1000331, OVARC1000410, OVARC1001086, OVARC1001132, OVARC1001607, OVARC1001725, OVARC1001952, PLACE1000258, PLACE1000442, PLACE1000907, PLACE1001100, PLACE1001500, PLACE1002905, PLACE1002967, PLACE1003407, PLACE1003428, PLACE1005005, PLACE1005239, PLACE1005815, PLACE1007028, PLACE1008716, PLACE1011407, PLACE1011978, PLACE2000118, THYRO1000580, THYRO1000866, THYRO1001071, THYRO1001478, Y79AA1001062, Y79AA1001530,

Out of them, the following 67 clones are those which matched the data of Swiss-Prot database and GenBank or UniGene database, genes or proteins which had been deposited in the database of Online Mendelian Inheritance in Man (OMIM) (http://www.ncbi.nim.nih.gov/Omim/), which is a database of human genes and diseases. (The corresponding OMIM numbers are indicated after the clone names.)
HEMBA1000349(*600046), HEMBA1000590(*603897), HEMBA1000671(*602277), HEMBA1001886(*603899), HEMBA1003120(*602277), HEMBA1004250(*600976), HEMBA1005246(*602291), HEMBA1005267(*106410), HEMBA1006707(*603897), HEMBA1006749(*603897), HEMBA1006902(*603897), HEMBA1006916(*601524), HEMBA1007013(*603730), HEMBB1002120(*603367), MAMMA1002080(*602672), NT2RM2001126(*603785), NT2RM2001558(*604689), NT2RP1000271(*603899), NT2RP1000465(*602231), NT2RP2000447(*602580), NT2RP2000514(*602431), NT2RP2000739(*194558), NT2RP2001223(*603558), NT2RP2001529(*603289), NT2RP2001562(*603371), NT2RP2002674(*132811), NT2RP2003369(*179555), NT2RP2004108(*601260), NT2RP2004205(*601610), NT2RP2005535(*603899), NT2RP2006004(*600245), NT2RP3000059(*106410), NT2RP3000125(*180202), NT2RP3000201(*604666), NT2RP3000232(*602277), NT2RP3000616(*600245), NT2RP3000677(*142765), NT2RP3000838(*190370), NT2RP3001542(*191161), NT2RP3002286(*604331), NT2RP3002721(*118950), NT2RP3002738(*602265), NT2RP3004481(*601610), OVARC1000208(*603603), OVARC1000275(*125647), OVARC1000331(*139265), OVARC1000410(*603874), OVARC1001086(*603862), OVARC1001725(*603046), OVARC1001952(*190370), PLACE1000258(*603971), PLACE1000442(*601260), PLACE1000907(*194558), PLACE1001500(*603781), PLACE1002905(*125950), PLACE1003428(*603570), PLACE1005005(*603124), PLACE1005239(*603365), PLACE1007028(*602131), PLACE1011407(*602277), PLACE1011978(*603971), PLACE2000118(*301000), THYRO1000580(*602277), THYRO1000866(*604045), THYRO1001071(*603533), Y79AA1001062(*191161), Y79AA1001530(*602662),

Out of 215 clones excluding the above-mentioned clones, HEMBB1000668 and NT2RM4001377 presumably belong to a group of signal transduction-associated proteins, based on the results of domain search by Pfam.

HEMBB1002302 presumably belong to a group of transcription-associated proteins, based on the results of domain search by Pfam.

In the 437 clones categorized into secretory and/or transmembrane proteins on the basis of their full-length sequences, 410 clones were also predicted to encode proteins having functions of secretory and/or membrane proteins on the basis of their partial nucleotide sequences (5' sequences). In the 146 clones categorized into glycoprotein-associated proteins on the basis of their full-length sequences, 124 clones were also predicted to encode proteins having functions of glycoprotein-associated proteins on the basis of their partial nucleotide sequences. In the 57 clones categorized into signal transduction-associated proteins on the basis of their full-length sequences, 46 clones were also predicted to encode proteins having functions of signal transduction-associated proteins on the basis of their partial nucleotide sequences. In the 81 clones categorized into transcription-associated proteins on the basis of their full-length sequences, 57 clones were also predicted to encode proteins having functions of transcription-associated proteins on the basis of their partial nucleotide sequences. In the 85 clones categorized into disease-associated proteins on the basis of their full-length sequences, 6 clones were also predicted to encode proteins having functions of disease-associated proteins on the basis of their partial nucleotide sequences. The number of clones which were predicted to encode disease-associated proteins based on the full-length nucleotide sequences is much greater than that predicted based on the partial sequences. The reason is that the full-length sequences were categorized by using the data found in the OMIM database into the category of disease-associated proteins.

When the predicted functions based on the partial sequences were different from those based on the full-length sequences, several reasons were presumed; the ORF is too short in the partial sequence as compared with that of the full-length sequence; alternatively, P value for the partial sequence was greater than that for the full-length, that is, as compared with the probability of occurrence of the predicted function found in the full-length sequence, the probability was lower in the partial sequence. A protein does not always belong solely to a single category of the above-described functional categories, and therefore, additional functions can be found for the cDNA clones by further analyses.

It is unclear, by the analyses for the full-length sequences so far, whether or not the remaining 212 clones encode proteins belonging to any of the categories of secretory and/or membrane proteins, glycoprotein-associated proteins, signal transduction-associated proteins, transcription-associated proteins or disease-associated proteins. Nonetheless, the functions which were predicted based on the partial sequences can be verified by further analyses.

Among the 212 clones, there are 38 clones that presumably belong to the category of enzymes and/or metabolism-associated proteins, cell division- and/or cell proliferation-associated proteins, cytoskeleton-associated proteins, nuclear proteins, DNA-and/or RNA-binding proteins, ASP- and/or GTP-binding proteins, protein synthesis- and/or protein transport-associated proteins, or cellular defense-associated proteins. The clones containing results of homology search of Swiss-Prot database were categorized by considering the keywords and mentioned items in the matching data. The clones containing results of homology search of GenBank or UniGene database were categorized by considering the definitions and mentioned items in the matching data.
When the matching data contained keywords such as "metabolism", "oxidoreductase" and "E.C. No. (Enzyme commission number)", the clones were herein defined as clones presumably belonging to the category of enzymes and/or metabolism-associated proteins. When the matching data contained keywords such as "cell division", "cell cycle", "mitosis", "chromosomal protein", "cell growth" and "apoptosis", the clones were herein defined as clones presumably belonging to the category of cell division- or cell proliferation-associated proteins. When the matching data contained keywords such as "structural protein", "cytoskeleton", "actin-binding" and "microtubules", the clones were herein defined as clones presumably belonging to the category of cytoskeleton-associated proteins. When the matching data contained keywords such as "nuclear protein", the clones were herein defined as clones presumably belonging to the category of nuclear proteins. When the matching data contained keywords such as "DNA-binding" and "RNA-binding", the clones were herein defined as clones presumably belonging to the category of DNA- or RNA-binding proteins. When the matching data contained keywords such as "ATP-binding" and "GTP-binding", the clones were herein defined as clones presumably belonging to the category of ATP- and/or GTP-binding proteins. When the matching data contained keywords such as "translation regulation", "protein biosynthesis", "amino-acid biosynthesis", "ribosomal protein", "protein transport" and "signal recognition particle", the clones were herein defined as clones presumably belonging to the category of protein synthesis- and/or protein transport-associated proteins. When the matching data contained keywords such as "heat shock", "DNA repair" and "DNA damage", the clones were herein defined as clones presumably belonging to the category of cellular defense-associated proteins.

The following 10 clones presumably belong to enzymes and/or metabolism-associated proteins.
HEMBA1003315, HEMBB1002465, MAMMA1000614, NT2RP2000178, NT2RP2001388, NT2RP2001903, NT2RP2002304, NT2RP2005878, NT2RP3001685, PLACE1006219

The following 4 clones presumably belong to cell division-associated and/or cell proliferation-associated proteins.
MAMMA1000403, NT2RM2000497, NT2RP2000394, Y79AA1002121

The following 6 clones presumably belong to cytoskeleton-associated proteins.
MAMMA1001609, NT2RM2000589, NT2RP3000063, PLACE1004078, PLACE 1004492, PLACE 1008657

The following 7 clones presumably belong to nuclear proteins.
HEMBA1001878, HEMBA1002992, MAMMA1000614, NT2RM4000965, NT2RM2001738, NT2RP2001388, Y79AA1002121

The following 5 clones presumably belong to DNA- and/or RNA-binding proteins.
HEMBA1003072, HEMBA1006770, HEMBA1007332, NT2RM2000497, Y79AA1002121

The following 7 clones presumably belong to ATP- and/or GTP-binding proteins.
HEMBA1002316, MAMMA1001609, NT2RM2000306, NT2RM2000497, NT2RP2000178, NT2RP3003729, PLACE1004305

The following 7 clones presumably belong to protein synthesis- and/or protein transport-associated proteins.
NT2RM4000965, NT2RP2005069, NT2RP3000481, NT2RP3000789, NT2RP4001877, OVARC1001833, OVARC1002058,

The following clone presumably belongs to cellular defense-associated proteins.
PLACE 1005539

Although it is unclear whether or not 26 out of 174 clones other than the above-mentioned clones belong to any of the above-described categories, these clones are predicted to have some functions, based on the homology search using their full-length sequences. The clone names and the gene definitions found in the result of homology search are shown below, separated by a double-slash,//
HEMBA1000634//Homo sapiens T-cell activation protein (PGR1) gene, complete cds.
HEMBA1002524//Human MHC Class I region proline rich protein mRNA, complete cds.
HEMBA1003399//MVP1 PROTEIN,
HEMBA1005489//Mus musculus semaphorin cytoplasmic domain-associated protein 3A (Semcap3) mRNA, complete cds.
HEMBB1000542//Mus musculus bromodamain-containing protein BP75 mRNA, complete cds.
MAMMA1000788//Bos taurus P14 (p14) mRNA, complete cds.
MAMMA1002128//ABC1 PROTEIN HOMOLOG PRECURSOR.
NT2RM2000514//Homo sapiens F-box protein Fbx21 (FBX21) mRNA, complete cds.
NT2RM2000622//Mus musculus F-box protein FBL10 mRNA, partial cds.
NT2RM4000100//Homo Sapiens Leman coiled-coil protein (LCCP) mRNA, complete cds.
NT2RP2005425//Homo sapiens mRNA for AKAP450 protein.
NT2RP3001170//Mus musculus activity-dependent neuroprotective protein (Adnp) mRNA, complete cds.
NT2RP3002571//Bos taurus mRNA for lyncein.
NT2RP3004557//Human Ki nuclear autoantigen mRNA, complete cds.
OVARC1001596//Homo sapiens Arf-like 2 binding protein BART1 mRNA, complete cds.
PLACE1002153//Homo sapiens TACC2 protein (TACC2) mRNA, partial cds.
PLACE1003163//Homo sapiens DBI-related protein mRNA, complete cds.
PLACE1005736//Human mRNA for BAS-GRIP protein.
PLACE1007702//Mus musculus TRA1 mRNA, complete cds.
PLACE1011045//Homo sapiens E1-like protein mRNA, complete cds.
THYRO1000061//Mus musculus mRNA for UBE-1c1, UBE-1c2, UBE-1c3, complete cds.
THYRO1000964//Drosophila melanogaster Felle associated protein Pellino (Pli) mRNA, complete cds.
Y79AA1000776//Mus musculus mRNA for GSG1, complete cds.
Y79AA1001056//Homo sapiens MAID protein mRNA, complete cds.
Y79AA1001272//Homo sapiens retinoic acid repressible protein (RARG-1) mRNA, complete cds.
Y79AA1001793//Mus musculus mRNA for GSG1, complete cds.

So far, useful information for presuming the functions are unavailable for the remaining 148 clones, of which names are listed below.
HEMBA1000275, HEMBA1000300, HEMBA1000443, HEMBA1000875, HEMBA1000907, HEMBA1001272, HEMBA1001296, HEMBA1001563, HEMBA1002164, HEMEA1002239, HEMBA1002985, HEMBA1003294, HEMBA1003487, HEMBA1004007, HEMBA1004067, HEMBA1004085, HEMBA1004952, HEMBA1004971, HEMBA1005145, HEMBA1005430, HEMBA1005913, HEMBA1006016, HEMBA1006517, HEMBA1006544, HEMBA1006912, HEMBA1007057, HEMBA1007063, HEMBA1007291, HEMBB1000276, HEMBB1000309, HEMBB1000642, HEMBB1001200, HEMBB1001547, HEMBB1002039, HEMBB1002228, HEMBB1002663, MAMMA1000046, MAMMA1000449, MAMMA1000528, MAMMA1000652, MAMMA1000706, MAMMA1000814, MAMMA1001066, MAMMA1001284, MAMMA1001623, MAMMA1001634, MAMMA1001901, MAMMA1002087, MAMMA1002205, MAMMA1002224, NT2RM2000582, NT2RM2001643, NT2RM4000115, NT2RM4000295, NT2RM4001321, NT2RP1000002, NT2RP1000239, NT2RP1000679, NT2RP1000740, NT2RP1000903, NT2RP2000240, NT2RP2001878, NT2RP2001921, NT2RP2002015, NT2RP2002409, NT2RP2002510, NT2RP2003599, NT2RP2003931, NT2RP2004069, NT2RP2004141, NT2RP2004447, NT2RP2004837, NT2RP2005514, NT2RP2005632, NT2RP2005887, NT2RP2006099, NT2RP2006134, NT2RP3000427, NT2RP3000444, NT2RP3000645, NT2RP3000871, NT2RP3001044, NT2RP3001061, NT2RP3001754, NT2RP3002281, NT2RP3002324, NT2RP3002353, NT2RP3002409, NT2RP3002448, NT2RP3002664, NT2RP3002887, NT2RP3002983, NT2RP3003448, NT2RP3003469, NT2RP3003473, NT2RP3003559, NT2RP3003963, NT2RP3004000, NT2RP3004202, NT2RP3004321,
NT2RP3004355, NT2RP3004374, NT2RP4002715, OVARC1000090, OVARC1000137, OVARC1000467, OVARC1000775, OVARC1000853, OVARC1000995, OVARC1001222, OVARC1001260, OVARC1001727, OVARC1002178, PLACE1000986, PLACE1001114, PLACE1001229, PLACE1001788, PLACE1003438, PLACE1003460, PLACE1003644, PLACE1004028, PLACE1004199, PLACE1004519, PLACE1005601, PLACE1005669, PLACE1005768, PLACE1006515, PLACE1006786, PLACE1007040, PLACE1007077, PLACE1007591, PLACE1007971, PLACE1008984, PLACE1009735, PLACE2000219, PLACE4000455, THYRO1000846, THYRO1000999, THYRO1001063, THYRO1001128, THYRO1001471, THYRO1001495, THYRO1001608, THYRO1001803, Y79AA1000127, Y79AA1000750, Y79AA1001592, Y79AA1001863,

### EXAMPLE 18

### Expression frequency analysis using PCR

Many genes acting at the downstream of TNF-. and IL-1. among inflammation-associated cytokines have been previously identified. The respective stimulations are transduced through independent pathways of signaling cascade. There exists another signaling cascade for both stimulations, wherein NF-.B is a common transducing molecule shared by the two stimulations (J. Leukoc. Biol., 1994, 56(5): 542-547). It has also been revealed that many inflammation-associated genes, including IL-2, IL-6 and G-CSF, are varied in their expression levels in response to the signal through the common pathway (Trends Genet. 1999, 15(6): 229-235). A survey was performed by using ATAC-PCR method (adaptor-competitive PCR method: Nucleic Acids Res. 1997, Nov 15; 25(22): 4694-6) for genes of which expression levels were varied depending on stimulation of inflammatory cytokines, TNF-. and IL-1.. It is possible that genes of which expression is varied in response to this stimulation also participate in inflammation.

Jurkat cells (Dainippon Pharmaceutical Co., Ltd.: catalog No. 06-152) were cultured in a PRMI1640 medium (Nikken Biological and Medical Institute: catalog No. 14-501F) containing 10% fetal calf serum until the cell count reached 10⁷ cells. The cells were transferred into a fresh medium containing 10 ng/ml TNF-. (recombinant Tumor Necrosis Factor; Wako pure chemical Industries Inc.: catalog No. 201-13461) or IL-1. (recombinant Interleukin-1.; PeprotechEC: catalog No. 200-01B) and, further, cultured at 37. under an atmosphere of 5% CO₂. The cells cultured in the presence of TNF-. were harvested 1, 3 and 7 hours after addition of TNF-.. The cells cultured in the presence of IL-1. were harvested 1 and 7 hours after addition of IL-1.. Total RNA was extracted from each of the cells by AGPC method (Acid-Guanidinium-Phenol-Chloroform method: Ana Biochem. 1987, Apr; 162(1):156-9). Total RNA was also extracted form the cells in the absence of any stimulation of TNF-. and IL-1

ATAC-PCR analysis is performed basically according to the same procedure as described in "DNA Microarray and Advanced PCR Methods" (Cell Engineering, p. 104-112, (additional volume, Genome Science Series 1), Muramatsu & Naba (eds.), Shujunnsya). Adaptor ligation reaction was performed for an internal standard sample (which was used for preparing a calibration curve for the assessment of the test samples) and test samples in the following independent two reaction systems. Combinations of each type of the 6 adaptors (AD-1, AD-2, AD-3, AD-4, AD-5, and AD-6: see the sequences shown below) with each sample are as follows:
Reaction system A
   AD1: internal standard sample (x10 concentration)
   AD2: sample before stimulation
   AD3: internal standard sample (x3 concentration)
   AD4: sample with IL-1 stimulation for 1 hour
   AD5: sample with IL-1 stimulation for 7 hours
   AD6: internal standard sample (x1 concentration)
Reaction system B
   AD1: internal standard sample (x1 concentration)
   AD2: sample with TNF stimulation for 1 hour
   AD3: sample with TNF stimulation for 3 hours
   AD4: internal standard sample (x3 concentration)
   AD5: sample with TNF stimulation for 7 hours
   AD6: internal standard sample (x10 concentration)

Adaptor sequence
AD1;
SEQ ID NO:4180//5'-GTACATATTGTCGTTAGAACGCG-3'

SEQ ID NO:4181//3'-CATGTATAACAGCAATCTTGCGCCTAG-5'
AD2;
SEQ ID NO:4182//5'-GTACATATTGTCGTTAGAACGCGACT-3'
SEQ ID NO:4183//3'-CATGTATAACAGCAATCTTGCGCTGACTAG-5'
AD3;
SEQ ID NO:4184//5'-GTACATATTGTCGTTAGAACGCGCATACT-3'
SEQ ID NO:4185//3'-CATGTATAACAGCAATCTTGCGCGTATCACTAG-5'
AD4;
SEQ ID NO:4186//5'-GTACATATTGTCGTTAGAACGCGATCCATACT-3'
SEQ ID NO:4187//3'-CATGTATAACAGCAATCTTGCGCTAGGTATGACTAG-5'
AD5;
SEQ ID NO:4188//5'-GTACATATTGTCGTTAGAACGCGTCAATCCATACT-3'
SEQ ID NO:4189//3'-CATGTATAACAGCAATCTTGCGCAGTTAGGTATGACTAG-5'
AD6;
SEQ ID NO:4190//5'-GTACATATTGTCGTTAGAACGCGTACTCAATCCATACT-3'
SEQ ID NO:4191//3'-CATGTATAACAGCAATCTTGCGCATGAGTTAGGTATGACTAG-5'

In this assay, the internal standard samples used were total RNA from cultured cells or human tissues from which the cDNA libraries originated. The cultured cells and the total RNAs from tissues are indicated below. Culture of the cells was performed according to the method as described in the supplier's instruction manual. RNA preparation was carried out by standard methods.
Human teratocarcinoma cell NT-2 (Stratagene, catalog No. 204101)
Human neuroblastoma cell SK-N-MC (Dainippon Pharmaceutical Co., Ltd., catalog No. 04-010)
Human neuroblastoma cell Y79 (Dainippon Pharmaceutical Co., Ltd., catalog No. 04-018)
Human placenta tissues total RNA (BioChin, catalog No. 064008)
Human breast tissue total RNA (Clontech, catalog No. 64037-1)

PCR primers used for amplification of specific genes, and names of the corresponding cDNA clones are shown below. The assay was not carried out for clones of which corresponding internal standard sample could not be prepared for the assay. The gene-specific primers were designed so that the PCR products derived from the cDNAs with adaptor were 70-200 bp in size. Sequence of the adaptor-specific primer (labeled with fluorescent dye (FAM)) used for the competitive PCR was GTACATATTGTCGTTAGAACGC (22 nucleotides, SEQ ID NO: 4192). PCR was performed basically at 94. for 5 minutes; and at 94. for 30 seconds, at 50. for 60 seconds, and at 72. for 60 seconds for 30 cycles. The annealing temperature was, however, changed in some PCR experiments.

Nucleotide sequence of clone-specific primer (all the primers consist of 20 nucleotides) used in this experiment

Clone names, primer sequences, and SEQ ID NOs were shown in this order, separated with a double-slash mark, //

MAMMA1000046//GTTACATCCAAGCATACAGG//SEQ ID NO:4193
MAMMA1000102//ACGGGGTCTCATTCTGACAC//SEQ ID NO:4194
MAMMA1000141//CAAGGTAACACGAGTCTATC//SEQ ID NO:4195
MAMMA1000226//ACTGAGGGGCAAAGGAGAGA//SEQ ID NO:4196
MAMMA1000403//ATTTTCCTGGAGAGCCGACT//SEQ ID NO:4197
MAMMA1000473//TGGAAAGTGTACCGGAATTG//SEQ ID NO:4198
MAMMA1000496//TCAATCTGGCGCTCTGTCAC//SEQ ID NO:4199
MAMMA1000614//AGTTCTTTACATGCTGAGGT//SEQ ID NO:4200
MAMMA1000652//TGGTGGAAGACTGGGTTTGC//SEQ ID NO:4201
MAMMA1000706//ATGGTCTTGTGGTGCCAGGT//SEQ ID NO:4202
MAMMA1000788//TGTCCAAAAGCCACACAGAG//SEQ ID NO:4203
MAMMA1000810//ATACTTCCCGCACCCCAAAA//SEQ ID NO:4204
MAMMA1000814//CAGGGTTTCTGCATGTTGGC//SEQ ID NO:4205
MAMMA1000881//ATGGAGTTTCACTCTTGTTG//SEQ ID NO:4206
MAMMA1000986//TGCTGCTTCTTTACATGGGA//SEQ ID NO:4207
MAMMA1000994//CAGGATAGAGGTTGCAGGCT//SEQ ID NO:4208
MAMMA1001066//GATGGGGTCTCACTCTGTCA//SEQ ID NO:4209
MAMMA1001094//ACGTCCAGAAACTACAGGGT//SEQ ID NO:4210
MAMMA1001141//ACTGTACTTAGGATGCTTCA//SEQ ID NO:4211
MAMMA1001237//GGGCAACCCTATGTAGATGA//SEQ ID NO:4212
MAMMA1001284//GTCTGCTCTGTTACATAGGG//SEQ ID NO:4213
MAMMA1001310//ACGCCTGTAATCCCAACCCA//SEQ ID NO:4214
MAMMA1001344//GCCAGTTTGTTCTAGGATGC//SEQ ID NO:4215
MAMMA1001532//ACATCTATAAGGCTGTTTGC//SEQ ID NO:4216
MAMMA1001609//GGGTCTCACTCTGTTACCCA//SEQ ID NO:4217
MAMMA1001615//CAAGGGACACTGAGAACTGG//SEQ ID NO:4218
MAMMA1001623//GGATTGATGCCCGATACTTA//SEQ ID NO:4219
MAMMA1001901//GATAGGGTCTCATTCTGTTA//SEQ ID NO:4220
MAMMA1001957//TAGTAGAGACGGGGTTTCAC//SEQ ID NO:4221
MAMMA1001978//CTCCCTCAGACGCTTTATTG//SEQ ID NO:4222
MAMMA1002070//GAAGAGAAACTGGGGCATCC//SEQ ID NO:4223
MAMMA1002080//ACTCTCCCTCACTACCACTG//SEQ ID NO:4224
MAMMA1002087//AGCTCGTCTCATGGGCAACT//SEQ ID NO:4225
MAMMA1002091//CTGACGTAGGTGAGGTCCAT//SEQ ID NO:4226

MAMMA1002095//CATGTTTATGGTCAGGTAGT//SEQ ID NO:4227
MAMMA1002128//TGGAGCAGATGAGGAAGGAG//SEQ ID NO:4228
MAMMA1002142//TCTCTTAAACATGCAACAGG//SEQ ID NO:4229
MAMMA1002165//CTCAAACACCCAGGCTCAAG//SEQ ID NO:4230
MAMMA1002234//TTTGTCTTCCTCTAAACCAG//SEQ ID NO:4231
MAMMA1002586//CTCCACCGAAAAGACCCATT//SEQ ID NO:4232
MAMMA1002633//CACAGCAGCATCTCCAAGCA//SEQ ID NO:4233
MAMMA1003126//ACAGTATCAGAGGAGCAGGA//SEQ ID NO:4234
NT2RM1000462//AGAGCCGAGGACATTTGAGG//SEQ ID NO:4235
NT2RM1000542//CAAGGGCACGTTTCAGCACT//SEQ ID NO:4236
NT2RM1000789//TTCGCTTGCTCTTCTCTGGA//SEQ ID NO:4237
NT2RM1000855//AGCAAGGTCTCCAGAGTGTG//SEQ ID NO:4238
NT2RM1000858//GAACAGGAAAATGCTCTCAG//SEQ ID NO:4239
NT2RM2000241//CTGCTTGCCTGCCTTGTAGT//SEQ ID NO:4240
NT2RM2000306//TAGTCCCTTTCCTGATGGTC//SEQ ID NO:4241
NT2RM2000497//TTAGTAGAGACGGTGTTTCA//SEQ ID NO:4242
NT2RM2000514//TGTTCTCTTTCTTTGCACTG//SEQ ID NO:4243
NT2RM2000582//GGGAATAACATCTACAACCT//SEQ ID NO:4244
NT2RM2000588//CCCCAGAAACAGAGAAGGCT//SEQ ID NO:4245
NT2RM2000589//TAAGGCATGTGTCCTCTAAG//SEQ ID NO:4246
NT2RM2000622//ATCTCGGCTATGAACTGCTC//SEQ ID NO:4247
NT2RM2000773//TTCTGCCCCTCCTCAAACCT//SEQ ID NO:4248
NT2RM2001126//GCAACAGCTTCTTCATGGGT//SEQ ID NO:4249
NT2RM2001558//TCGCCCACACTGCATCCTTT//SEQ ID NO:4250
NT2RM2001626//TCGGGTGGCAGTGTGTTGAA//SEQ ID NO:4251
NT2RM2001643//GTAGTTCTCTCTGAAGAATC//SEQ ID NO:4252
NT2RM2001738//CCCAGCACTTTTATTTGTAG//SEQ ID NO:4253
NT2RM2001792//AGTGTAGTTTGGAGATGAGA//SEQ ID NO:4254
NT2RM2001902//TCCCCATCCAGCCACAGAAA//SEQ ID NO:4255
NT2RM2002109//ATTGCCTATAGAAAGTCAGC//SEQ ID NO:4256
NT2RM4000100//GCATTTATAGGGCTCAAGAT//SEQ ID NO:4257
NT2RM4000115//TAGTTTCGACTTCTGGTTCA//SEQ ID NO:4258
NT2RM4000284//ACATCCTTCAAATCAGCATC//SEQ ID NO:4259
NT2RM4000295//GGTCTCACGCTGCTCACAAA//SEQ ID NO:4260
NT2RM4000417//TGCTGTGTAGTATTCCTTAG//SEQ ID NO:4261
NT2RM4000761//CTAATACAATGCCAGTCAGG//SEQ ID NO:4262
NT2RM4001377//CTAGCTTCCTCTCCCAACTG//SEQ ID NO:4263
NT2RM4001735//TCATCACGACTGCTGTAGAG//SEQ ID NO:4264
NT2RM4001768//TTAGTAGAGGTGGGGTTTCA//SEQ ID NO:4265

NT2RM4001843//CAAGGGCACGTTTCAGCACT//SEQ ID NO:4266
NT2RP1000239//TTAGCAGTGTATCCTTTCCG//SEQ ID NO:4267
NT2RP1000465//TTGCCCAGGCTAGTCTCGAA//SEQ ID NO:4268
NT2RP1000468//TTGCCCAGGCTAGTCTCGAA//SEQ ID NO:4269
NT2RP1000679//GGCCTCAGTTCCTTGCATTT//SEQ ID NO:4270
NT2RP1000740//CGCTATATTTCATGGGCTTT//SEQ ID NO:4271
NT2RP1001031//CATGTTACCTATACACACCA//SEQ ID NO:4272
NT2RP2000092//CACACACACATGAACTCATT//SEQ ID NO:4273
NT2RP2000178//ATCTTTTGTGACAGCTCCAG//SEQ ID NO:4274
NT2RP2000240//CCATTCCACTCATACCAAGA//SEQ ID NO:4275
NT2RP2000447//CTCTTGGCATGATAGGTCTT//SEQ ID NO:4276
NT2RP2000610//GACATGAGACAAAATTAGCC//SEQ ID NO:4277
NT2RP2000616//AAAATAACTGCCTGGGAGGT//SEQ ID NO:4278
NT2RP2000712//GAAGGTAGAGCTTGAGAGAG//SEQ ID NO:4279
NT2RP2000739//AGCAACAGGGAATGGAGGTG//SEQ ID NO:4280
NT2RP2000818//GGGAAGAAATGAGACAAAGA//SEQ ID NO:4281
NT2RP2001200//ACCACATAGCTGCAGGAAAG//SEQ ID NO:4282
NT2RP2001276//TTGCCGTTGGTGCTGGTAGT//SEQ ID NO:4283
NT2RP2001388//TGGCACAATCTCCGCTCACT//SEQ ID NO:4284
NT2RP2001469//TAATGGGTGGTGGGAGCTGA//SEQ ID NO:4285
NT2RP2001538//GAAAAGGGTAGGCAGCAAGG//SEQ ID NO:4286
NT2RP2001562//CACCCTGCCACAAGACATTT//SEQ ID NO:4287
NT2RP2001662//AAGAGGCTGGGCAAATGGCA//SEQ ID NO:4288
NT2RP2001755//CAAGCAAATAATCCAGCCAT//SEQ ID NO:4289
NT2RP2001817//CTAAAGCAACAGAGGAATAC//SEQ ID NO:4290
NT2RP2001921//TGTGGGGTTGGTCTTTGGAA//SEQ ID NO:4291
NT2RP2001948//GCATTGAGGACTTTTCCAGA//SEQ ID NO:4292
NT2RP2002015//GTAGTTCTCTCTGAAGAATC//SEQ ID NO:4293
NT2RP2003138//ATGGAAGAGGCTGAGGCAAA//SEQ ID NO:4294
NT2RP2003194//CACCTCTCATGTTTCTGCAC//SEQ ID NO:4295
NT2RP2003302//AGAGGACAGTTGGAAGATTT//SEQ ID NO:4296
NT2RP2003390//CTGTGGGTGATTTTCTGGCA//SEQ ID NO:4297
NT2RP2003593//CTGACCCCTAAGTAAATGAA//SEQ ID NO:4298
NT2RP2003664//GGGTGTGATGTTTACTTCTC//SEQ ID NO:4299
NT2RP2003950//CTGAAGGAGGTACTGACACT//SEQ ID NO:4300
NT2RP2004069//CATCACCAAGGTATTAAGCC//SEQ ID NO:4301
NT2RP2004108//GATAGGTTACTGGTGAATTG//SEQ ID NO:4302
NT2RP2005069//GAAAATGAACCTGTAGCCTT//SEQ ID NO:4303
NT2RP2005378//ATTCTGGCTCCCTCTTCCTC//SEQ ID NO:4304

NT2RP2005391//CAAGGTGGATTTTACATGGG//SEQ ID NO:4305
NT2RP2005535//GAATTATCGCCATGTCTCTT//SEQ ID NO:4306
NT2RP2005597//GACCAAGACCCTTCAACCTC//SEQ ID NO:4307
NT2RP2006092//TCCTACGTTGTGGTGCAGTG//SEQ ID NO:4308
NT2RP2006134//TGACTGCCAATTAGAAACCT//SEQ ID NO:4309
NT2RP2006208//AAAATTGGCTTCTGCCTAGT//SEQ ID NO:4310
NT2RP2006476//GTTCACCTTCACATCCAAAA//SEQ ID NO:4311
NT2RP3000011//TGGCACAATCTCAGCTCACT//SEQ ID NO:4312
NT2RP3000031//TTCTGGGCTGGAGTAGTGGT//SEQ ID NO:4313
NT2RP3000063//AGATAGTCACATAGACAGAG//SEQ ID NO:4314
NT2RP3000125//ACACATCCAACCCTTCACTT//SEQ ID NO:4315
NT2RP3000148//AACAAGTCCAGCCCCAGAAG//SEQ ID NO:4316
NT2RP3000169//CCAAGCACGCCATATGAAGC//SEQ ID NO:4317
NT2RP3000171//CAGAATTTTGCCACGAGGAT//SEQ ID NO:4318
NT2RP3000172//GGCACAGACACCATCCTTGA//SEQ ID NO:4319
NT2RP3000201//AATGGGGTTTTGCCAGGTTG//SEQ ID NO:4320
NT2RP3000232//ACCTTCATACAACTTTTCCC//SEQ ID NO:4321
NT2RP3000304//TGGTCTGCCTCATCCTTCTC//SEQ ID NO:4322
NT2RP3000378//GAAAAGGGTAGGCAGCAAGG//SEQ ID NO:4323
NT2RP3000427//ATGTAAAGTGTGGAGTACCC//SEQ ID NO:4324
NT2RP3000444//TTCTTCTCAGTCACCTCCAC//SEQ ID NO:4325
NT2RP3000616//GTGATAGTAACACAATCCTG//SEQ ID NO:4326
NT2RP3000645//TGGCCAGTGTATGAGAGGTC//SEQ ID NO:4327
NT2RP3000676//CAAGACAACAAAACAGAAGG//SEQ ID NO:4328
NT2RP3000677//TCCATTTTAGCTGTAGACAC//SEQ ID NO:4329
NT2RP3000721//GAGACTGTGATGCCTTGGTG//SEQ ID NO:4330
NT2RP3000789//AACTGATGGCTTCTGTCTCC//SEQ ID NO:4331
NT2RP3000818//GGGAGAAGCCTAGAAAAGAA//SEQ ID NO:4332
NT2RP3000820//TCACATTCAAGCTCTACGTC//SEQ ID NO:4333
NT2RP3000838//CTAGCTTCCTCTCCCAACTG//SEQ ID NO:4334
NT2RP3000871//GATTGTGCTTGGAGCTTGGC//SEQ ID NO:4335
NT2RP3000907//GGGAGATGAAGAGGAAGCAG//SEQ ID NO:4336
NT2RP3000921//ACATGGGTAGCACTCCTTTT//SEQ ID NO:4337
NT2RP3001012//ACCCATTTCTACCTCTCTTA//SEQ ID NO:4338
NT2RP3001044//AGCAAGGCAATGAGGATACT//SEQ ID NO:4339
NT2RP3001159//AAATGTAGGGGTGGGGAAGT//SEQ ID NO:4340
NT2RP3001240//AGCAAGGTCTCCAGAGTGTG//SEQ ID NO:4341
NT2RP3001271//GTGCTTCTACCATTGAGGAT//SEQ ID NO:4342
NT2RP3001542//TGGTCCTCACTGCCTTCAAA//SEQ ID NO:4343

NT2RP3001560//CGCCTCCACAAACAAACCCT//SEQ ID NO:4344
NT2RP3001592//TAAAAGGGAGGATGTTCAGT//SEQ ID NO:4345
NT2RP3001650//GCATCTTCTGGTAGTTGTCC//SEQ ID NO:4346
NT2RP3001685//GATTCTCCTTGCCTCAGCCT//SEQ ID NO:4347
NT2RP3001754//AGTTAGTGGTGCCTGCTTCC//SEQ ID NO:4348
NT2RP3001976//CTCACTGGCATTTAGCTGGT//SEQ ID NO:4349
NT2RP3002015//CAACAACCTTCCTCCTACCC//SEQ ID NO:4350
NT2RP3002281//TAAACAGTCAACCAATGCTC//SEQ ID NO:4351
NT2RP3002286//AAGCAAGAGATTTGGAGGAA//SEQ ID NO:4352
NT2RP3002353//GGCTGGAACTCAATTTCTGC//SEQ ID NO:4353
NT2RP3002409//GTACCCCTAGTGAAGACCTG//SEQ ID NO:4354
NT2RP3002411//TCAATAGCTACCTGTAGAGT//SEQ ID NO:4355
NT2RP3002448//TCTTCCTACGACATAACCAT//SEQ ID NO:4356
NT2RP3002571//GCCCAAAATCCAACAGTAAA//SEQ ID NO:4357
NT2RP3002721//AGTGGCTTGTATTCTGTGGA//SEQ ID NO:4358
NT2RP3002737//GTGAGGTACTTGCATATCCG//SEQ ID NO:4359
NT2RP3002738//TCCCCGATGAACACCAGCTT//SEQ ID NO:4360
NT2RP3002790//CTGGAGCCTGATTATGAGAA//SEQ ID NO:4361
NT2RP3002836//CTTTAGCAACATAACCTCCA//SEQ ID NO:4362
NT2RP3002900//TTTCTTCCTCCCTAACACAT//SEQ ID NO:4363
NT2RP3002958//GCCTTCTTCCAGCTCTACAT//SEQ ID NO:4364
NT2RP3002983//AATTTCCTTGCTAGAAGGCT//SEQ ID NO:4365
NT2RP3003354//GGCTGACACCTATTATCCCA//SEQ ID NO:4366
NT2RP3003448//TGTAGTCCCAGCTATTCAGG//SEQ ID NO:4367
NT2RP3003473//AATTAATCTCTGGGTAGCAC//SEQ ID NO:4368
NT2RP3003527//TGTCAATGGCCAGGTCGTAG//SEQ ID NO:4369
NT2RP3003532//TAAACTGACTTCCTCTTGGG//SEQ ID NO:4370
NT2RP3003535//CTAATGCCAGTGTTTCAAGA//SEQ ID NO:4371
NT2RP3003559//AATTCTTGTCCTGCTTTTGC//SEQ ID NO:4372
NT2RP3003614//GGGTCTTTGGATTGAGTGTC//SEQ ID NO:4373
NT2RP3003963//CACGATTATCTCTGCCAAAA//SEQ ID NO:4374
NT2RP3004000//TGGCCAGTGTATGAGAGGTC//SEQ ID NO:4375
NT2RP3004025//CACATTCTGGTGGAAAAGCA//SEQ ID NO:4376
NT2RP3004067//TGATGACACGCAGCACTTCC//SEQ ID NO:4377
NT2RP3004075//TTTCAAGTCAACACCTGCAC//SEQ ID NO:4378
NT2RP3004090//TACACTACAGATGGGCAAAA//SEQ ID NO:4379
NT2RP3004119//TCTAAGGCTGGGTACAGTGG//SEQ ID NO:4380
NT2RP3004130//ACATTTCTCCTCCATACGCA//SEQ ID NO:4381
NT2RP3004133//TAACCGCACTATGAGGAAAG//SEQ ID NO:4382

NT2RP3004202//CAGTGGCCTGGTAATAGAGT//SEQ ID NO:4383
NT2RP3004294//ATATTCCACTCCCCATTCGG//SEQ ID NO:4384
NT2RP3004321//GACCAACATGCTAGAAGTGC//SEQ ID NO:4385
NT2RP3004345//AACTCTCATTCCATAAGGTG//SEQ ID NO:4386
NT2RP3004406//CACCTAAAAGACTAATCCCT//SEQ ID NO:4387
NT2RP3004552//GAATCCAAAAGCCGGTAGGG//SEQ ID NO:4388
NT2RP3004557//GAAAGAGGTCAAAGTACCTG//SEQ ID NO:4389
NT2RP3004625//ATACAGGCAGCAGGAATCAC//SEQ ID NO:4390
NT2RP3004647//CAAGACAACAAAACAGAAGG//SEQ ID NO:4391
NT2RP4000634//ATGAAGAGTTACCTATGTGG//SEQ ID NO:4392
NT2RP4000962//AACCCTGGGCTTGGAATTCA//SEQ ID NO:4393
NT2RP4001001//TTTCACAATGCTACAGAGGT//SEQ ID NO:4394
NT2RP4001009//AATGTCAGCGGAGCAAAAGA//SEQ ID NO:4395
NT2RP4001467//GTTGAGAATGCTGGACTTGA//SEQ ID NO:4396
NT2RP4001877//AATCATATAGTCCCAGGTTG//SEQ ID NO:4397
NT2RP4001879//GTGTGAGAGTAGTTGGGGAA//SEQ ID NO:4398
NT2RP4002187//TCAATAGCTACCTGTAGAGT//SEQ ID NO:4399
NT2RP4002451//CATAAACAGTGACAGCAGAA//SEQ ID NO:4400
NT2RP4002715//AGCAAGGCAATGAGGATACT//SEQ ID NO:4401
NT2RP4002750//CAGCATTTAGGTGTGACGAT//SEQ ID NO:4402
PLACE1000040//ACATTGCTTGAGTCTTGCCA//SEQ ID NO:4403
PLACE1000986//CAAGGAGGTAATAGGGAGAT//SEQ ID NO:4404
PLACE1002080//CTCACTCTGTCATCGAGGCT//SEQ ID NO:4405
PLACE1002547//GTACCCCTAGTGAAGACCTG//SEQ ID NO:4406
PLACE1002911//CAAAGGCAAGTGTTCAGGCT//SEQ ID NO:4407
PLACE1003407//GACTACAAAACCCTGGAAAG//SEQ ID NO:4408
PLACE1003573//GGTGGCATGTAAATAAGACT//SEQ ID NO:4409
PLACE1004078//CTCAGCCTTCCAAGTAGCAG//SEQ ID NO:4410
PLACE1004199//GGAATCTGGGAGTCAAAATC//SEQ ID NO:4411
PLACE1004305//GCACCACTTTCGTCTTGAGC//SEQ ID NO:4412
PLACE1004450//GGCACTTAGCTTCTTGTTTT//SEQ ID NO:4413
PLACE1004492//TGGGCATCAATAAACACCTC//SEQ ID NO:4414
PLACE1004630//TGCTTTTGTATGGCCTGGGA//SEQ ID NO:4415
PLACE1004816//ATGTGGACAGGCAAGCAGAG//SEQ ID NO:4416
PLACE1005031//GGTGTTCCCCTTCTTGTTTG//SEQ ID NO:4417
PLACE1005539//AGAGGTAGAATGAGGGGAAA//SEQ ID NO:4418
PLACE1005569//CTGAACCCTGGCCTGTGAAA//SEQ ID NO:4419
PLACE1005601//AGATGGGGACTATGAAGAGG//SEQ ID NO:4420
PLACE1005736//CCTGTAATCCCAGCACTTTG//SEQ ID NO:4421

PLACE1005815//AGAGACAGAGTTTTGCTCTT//SEQ ID NO:4422
PLACE1005927//GGTGTAGCCCATTGTAGAGG//SEQ ID NO:4423
PLACE1006071//TTTGCTCTTTAACTCTGCCT//SEQ ID NO:4424
PLACE1006073//TAAAGGGCAGGGTGGAGGAA//SEQ ID NO:4425
PLACE1006079//TGATGTCCACTGTCTTATTG//SEQ ID NO:4426
PLACE1006786//CAAGGAGGTAATAGGGAGAT//SEQ ID NO:4427
PLACE1007077//ATGTGCTGGTAAGTGAAATC//SEQ ID NO:4428
PLACE1007081//GCCGGCCAAGTTTACACGAA//SEQ ID NO:4429
PLACE1007845//TAACCGCACTATGAGGAAAG//SEQ ID NO:4430
PLACE1007971//GACAATTCAACTGGAAGACC//SEQ ID NO:4431
PLACE1008282//CGTGATGACTGCCAACTCCA//SEQ ID NO:4432
PLACE1008359//AACAGGGTCCCATTTATTTG//SEQ ID NO:4433
PLACE1008469//TTGTCATCCTCCTGCCTCTG//SEQ ID NO:4434
PLACE1008657//CTCAGCCTTCCAAGTAGCAG//SEQ ID NO:4435
PLACE1008744//TAAACAAGACCCAGCACCAT//SEQ ID NO:4436
PLACE1008984//TCGAGACCGCTTCCCATAGA//SEQ ID NO:4437
PLACE1009546//AGGTAGCCGATGACAAAGCC//SEQ ID NO:4438
PLACE1010011//GGATATAAAGACAAGGATGC//SEQ ID NO:4439
PLACE1010713//TCAATAGCTACCTGTAGAGT//SEQ ID NO:4440
PLACE1011019//AAGGAGTGAAGCTCCATGCC//SEQ ID NO:4441
PLACE1011116//CCATTACAAACCCTTTAACC//SEQ ID NO:4442
PLACE1011181//TGTTATCCATTGTTCACCTG//SEQ ID NO:4443
PLACE1011364//TGTTGACGCTGATTTAGGCA//SEQ ID NO:4444
PLACE3000213//TAAACAAGACCCAGCACCAT//SEQ ID NO:4445
PLACE4000354//TAAACAAGACCCAGCACCAT//SEQ ID NO:4446
PLACE4000455//CAAGGAGGTAATAGGGAGAT//SEQ ID NO:4447
SKNMC1000014//CGAGACAGGGTCTTGGTTTG//SEQ ID NO:4448
SKNMC1000082//TTTCCTTCGGCTGGTATGGC//SEQ ID NO:4449
Y79AA1000030//TCCTACGTTGTGGTGCAGTG//SEQ ID NO:4450
Y79AA1000037//AGCACCCCTTATTGGAAGAT//SEQ ID NO:4451
Y79AA1000127//GATGGTTACTCCTCTTTGGT//SEQ ID NO:4452
Y79AA1000226//TGCTGATGCCTCTGTTCCCT//SEQ ID NO:4453
Y79AA1000270//GAAACAACCAAGCACCCCAT//SEQ ID NO:4454
Y79AA1000750//ATAAGGGCAGCTGGGAAGTG//SEQ ID NO:4455
Y79AA1000776//TGTTAGTAGCAGGAGGAAGC//SEQ ID NO:4456
Y79AA1000777//ACAGAGTTCAGTCCCCTTTA//SEQ ID NO:4457
Y79AA1000876//GTTAGAGGACACTGGCATCA//SEQ ID NO:4458
Y79AA1000888//ACTGACTTGAGGAATAAGCC//SEQ ID NO:4459
Y79AA1000959//AGAGTGAGAGCATGGGAGGT//SEQ ID NO:4460

Y79AA1000967//GGCACAGACACCATCCTTGA//SEQ ID NO:4461
Y79AA1001056//ACAAATGAGCCTGAAAAGTC//SEQ ID NO:4462
Y79AA1001062//TGGTCCTCACTGCCTTCAAA//SEQ ID NO:4463
Y79AA1001090//AGTGCCCTCAAAGCTCCAGT//SEQ ID NO:4464
Y79AA1001212//ACGAAAGCACTCAAATGTCA//SEQ ID NO:4465
Y79AA1001272//GAATGAAATGTGGTTGAGCA//SEQ ID NO:4466
Y79AA1001426//AATGATTCGGGGCAGCAGGA//SEQ ID NO:4467
Y79AA1001427//GAGAGAGACACACACAGAAA//SEQ ID NO:4468
Y79AA1001523//AGTTTTATACCAGCATTGGC//SEQ ID NO:4469
Y79AA1001530//GGTGTAGAAGTAAAATGGGA//SEQ ID NO:4470
Y79AA1001592//GATTGTGTTCTCTTACTCCT//SEQ ID NO:4471
Y79AA1001727//GCTCCACCTGACGTTCTTTA//SEQ ID NO:4472
Y79AA1001795//GTCTCCCATATCGCTGTCTT//SEQ ID NO:4473
Y79AA1001803//CACTTTCTAATAACCCTTGG//SEQ ID NO:4474
Y79AA1001863//TTGGGATTGGAAACCGCATT//SEQ ID NO:4475
Y79AA1001874//AGAAACCACTGAGGCCCAAG//SEQ ID NO:4476
Y79AA1002058//CAGAAGCAGAAGCAGGAGCA//SEQ ID NO:4477
Y79AA1002121//ATTTACTGCGATTTCTCCTG//SEQ ID NO:4478
Y79AA1002129//GAGTTTCTTTGCTAGTTCCA//SEQ ID NO:4479
Y79AA1002334//ATATTTGTGTTTGCCTTGGG//SEQ ID NO:4480
Y79AA1002373//GGATGGCTGGGTCAAATGGT//SEQ ID NO:4481
Y79AA1002376//AATGATGGCTAGGGTGACTT//SEQ ID NO:4482
Y79AA1002378//TCTTCCCACATTCGTTACAC//SEQ ID NO:4483
Y79AA1002381//AGGGAGTAGATGTTGGTAAA//SEQ ID NO:4484

The result of expression frequency analysis is shown in Table 367. Only clones with correlation coefficient of 0.9 or higher are indicated in this Table. Clones that are not presented in the Table include clones for which the assay could not performed because of low expression levels thereof in internal standard samples or because of unexpectedly smaller or larger sizes of the PCR products.

Among the clones that could be analyzed, clones of which expression levels increased by two fold in response to the IL-1. stimulation 1 or 7 hours after the stimulation are: NT2RM2000514, NT2RP3001159, MAMMA1001237 and MAMMA1000614.

Clones of which expression levels increased by two fold in response to the TNF-stimulation 1, 3 or 7 hours after the stimulation are:
NT2RM2000582, NT2RM2002109, NT2RP1000679, NT2RP2003664, NT2RP2005597, NT2RP2004108, NT2RP3001592, NT2RP3002738, NT2RP3004133, NT2RP3004321, NT2RP3004557, NT2RP3004294, MAMMA1001237, MAMMA1000141, MAMMA1000788, MAMMA1002070, PLACE1002547, PLACE1003573, PLACE1004305, PLACE1008744, PLACE1011181, PLACE1010713, PLACE1010011, Y79AA1000776, Y79AA1002129,

Among the clones of which expression levels increased in response to IL-1. stimulation, MAMMA1001237 was a clone of which expression level was varied in response to TNF-. stimulation. Among clones showing higher expression levels (with relative value of 5 or higher) prior to the stimulation, PLACE1002080 is an example of clones of which expression was suppressed by the stimulation. The expression of the clone decreased by three or more fold in response to the stimulation. These genes were found to be associated with inflammatory reaction induced by IL-1. or TNF-..

In Example 15, the genes of which expression levels were varied by culturing in the presence of TNF-. were analyzed by hybridization with high-density DNA filter. As for 3 clones (NT2RP3004557, NT2RP3004294 and PLACE 1002547), the results obtained by ATAC-PCR method were similar to those obtained by hybridization method. However, the results obtained by ATAC-PCR method were not necessarily consistent with those obtained by the hybridization method. Possible reasons for the inconsistency are the difference in cells used between the two experiments, unavailability of some data in the ATAC-PCR experiment, and the difference in the method of data treatment.

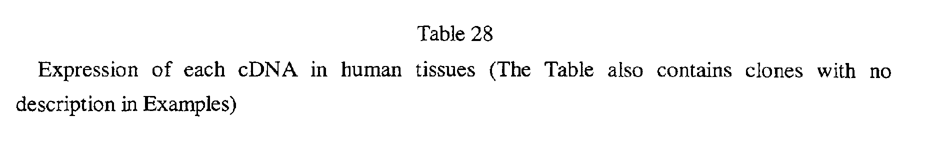

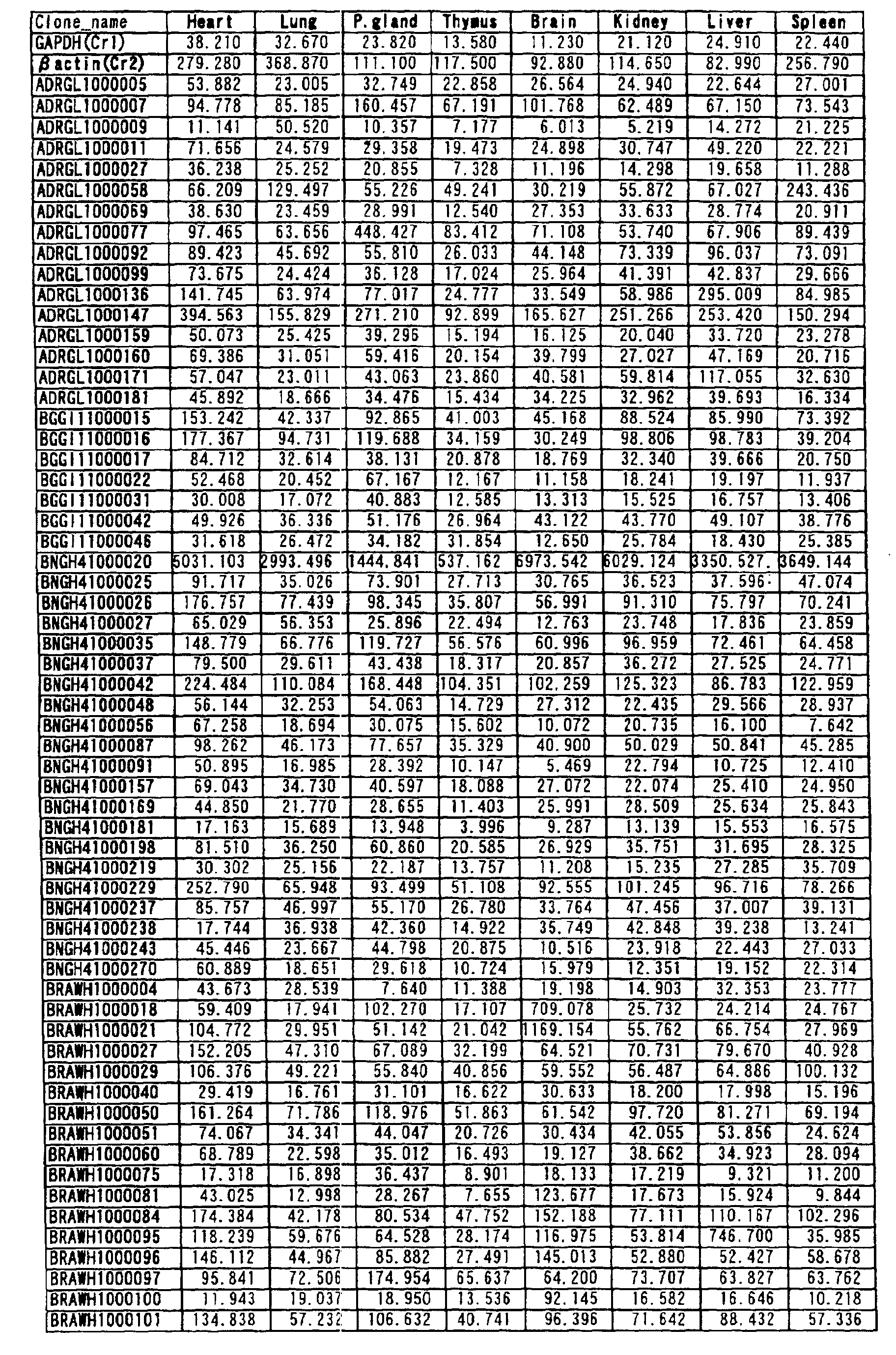

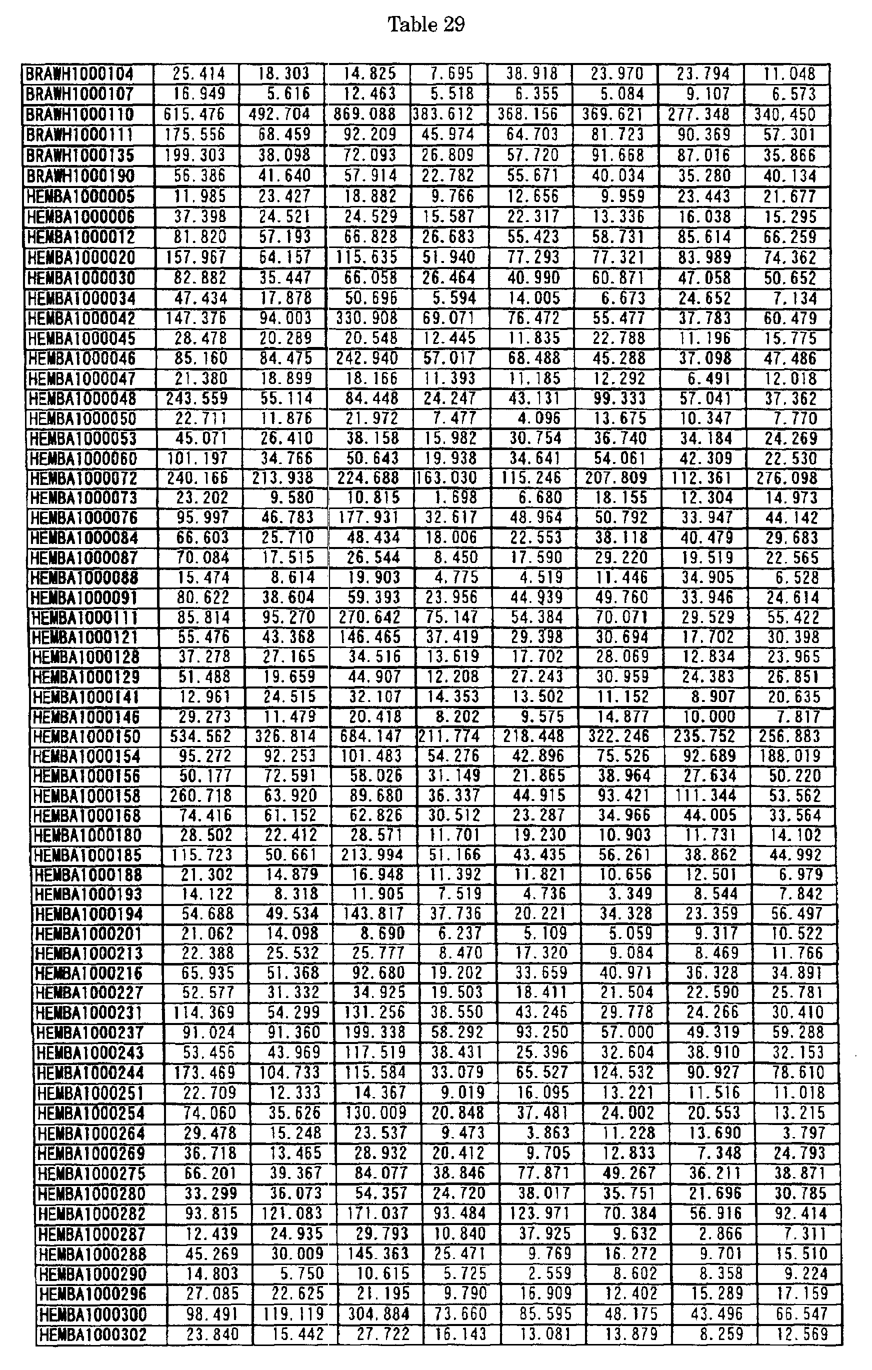

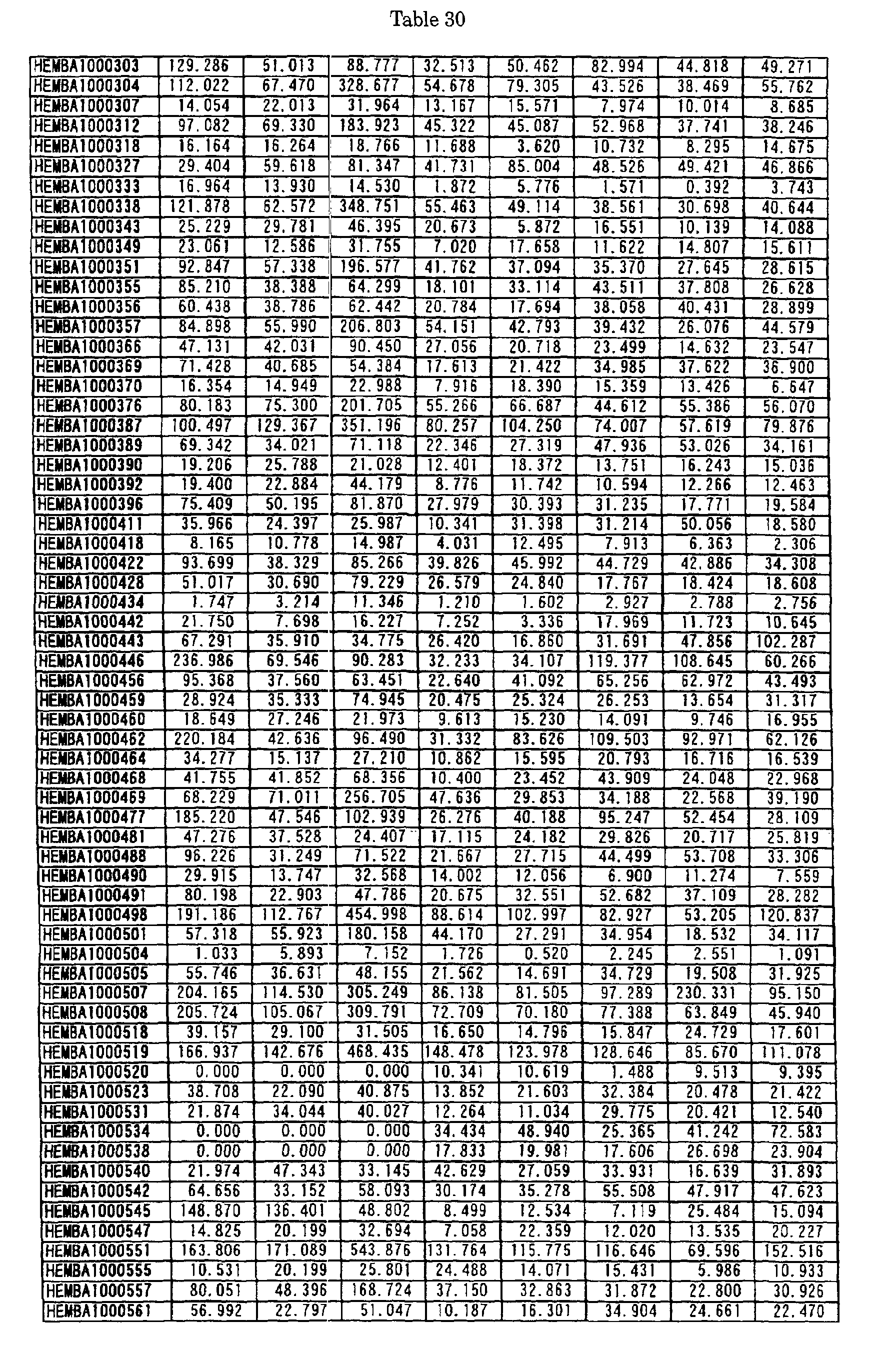

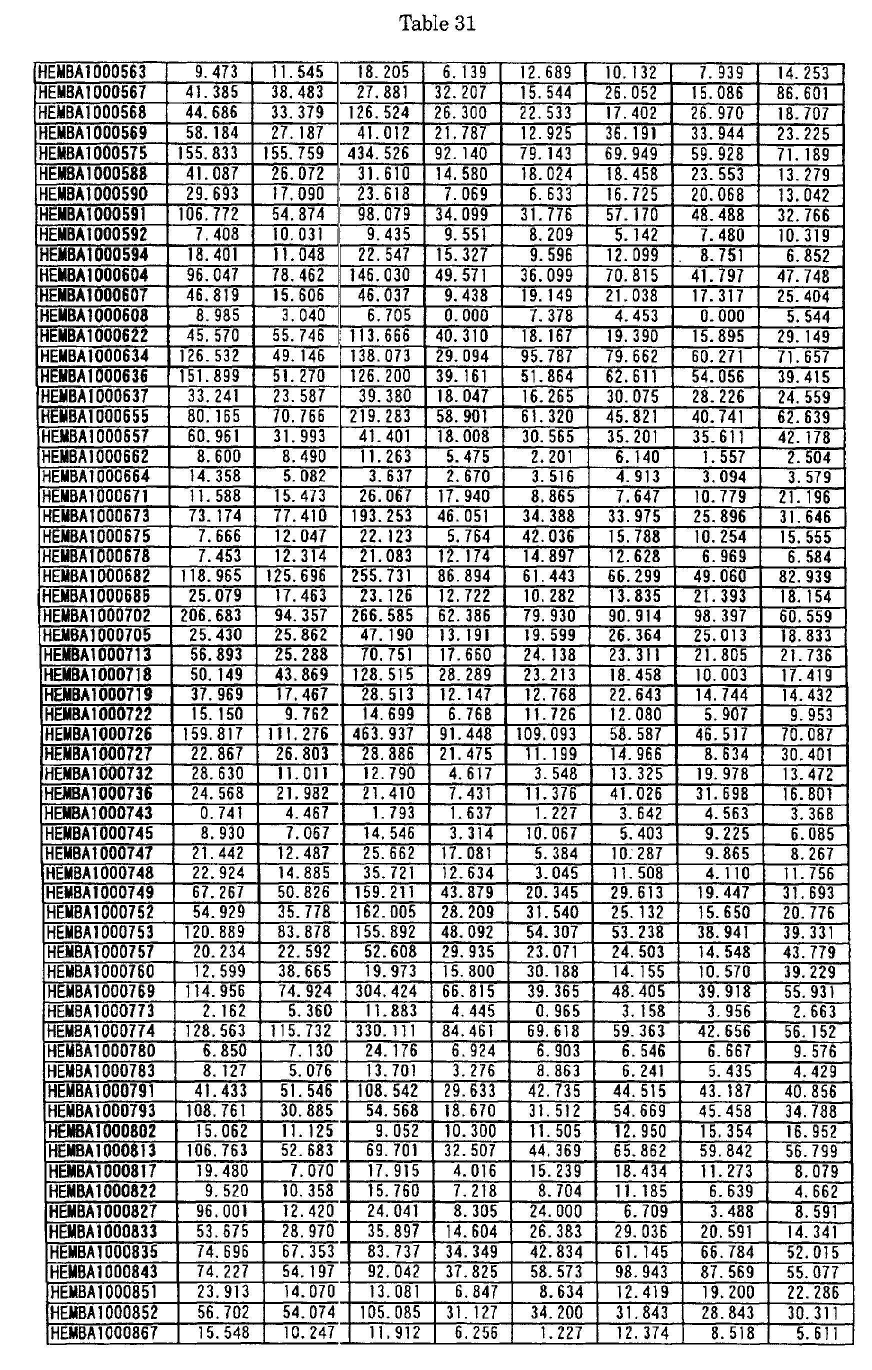

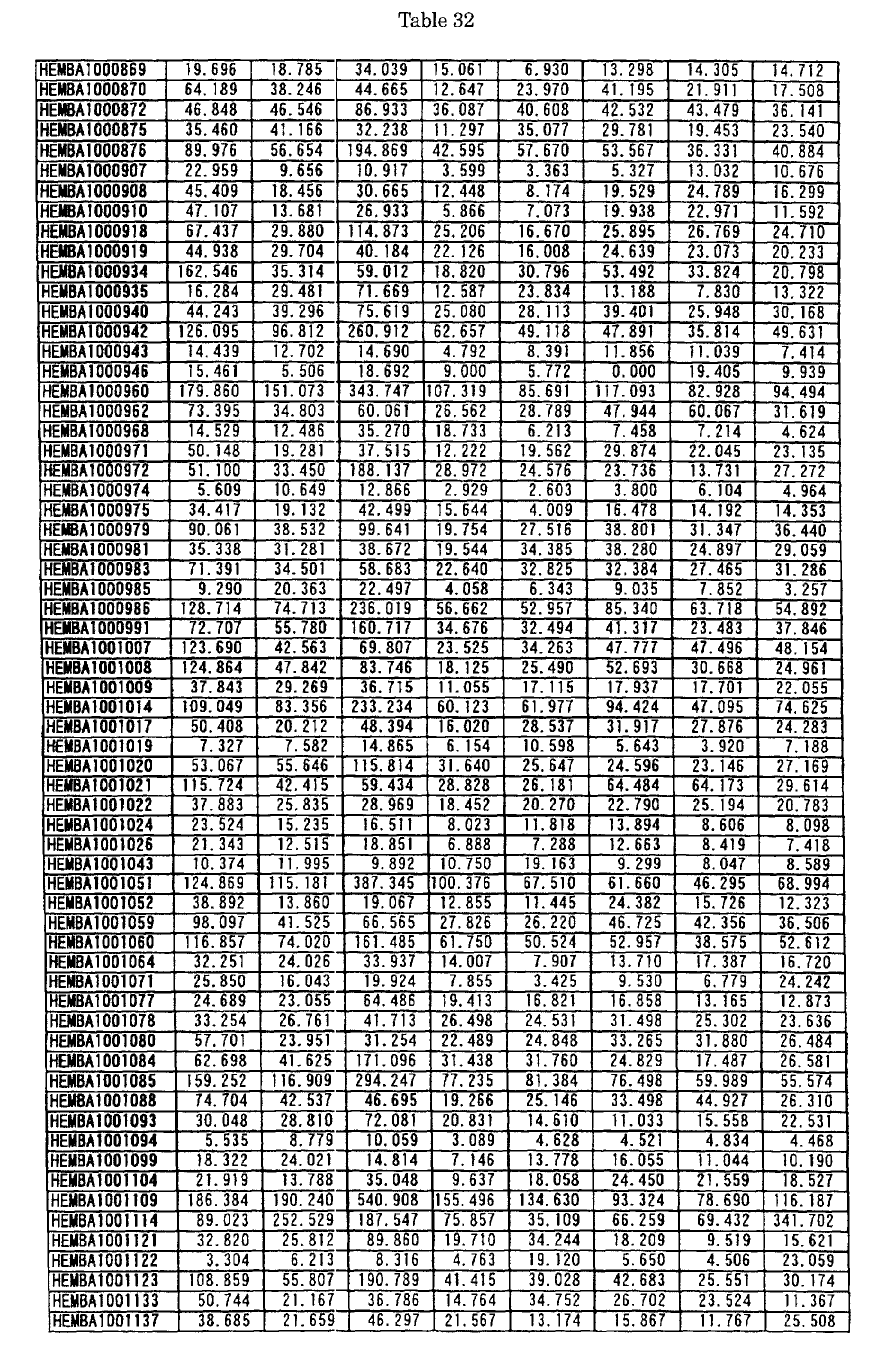

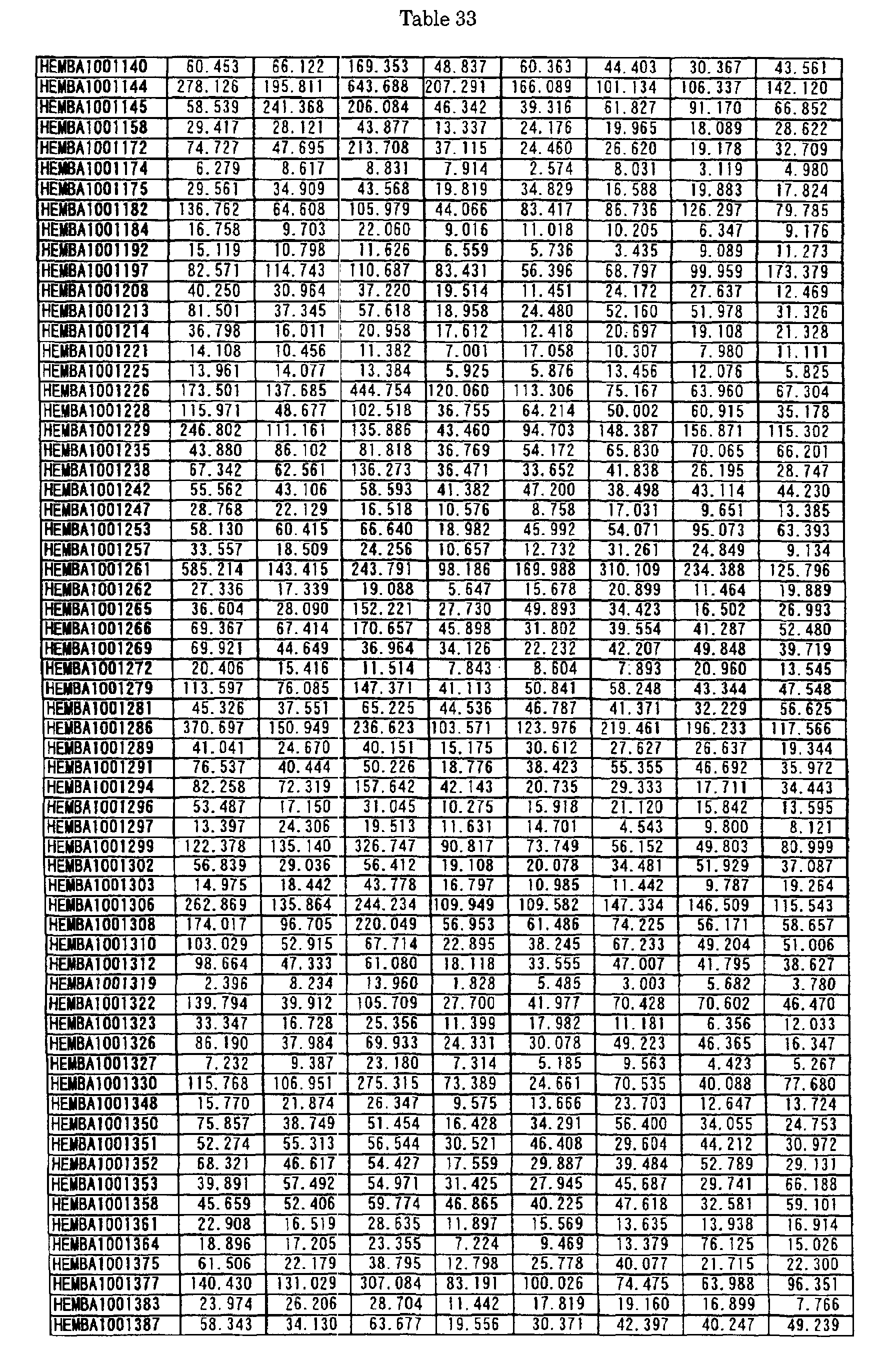

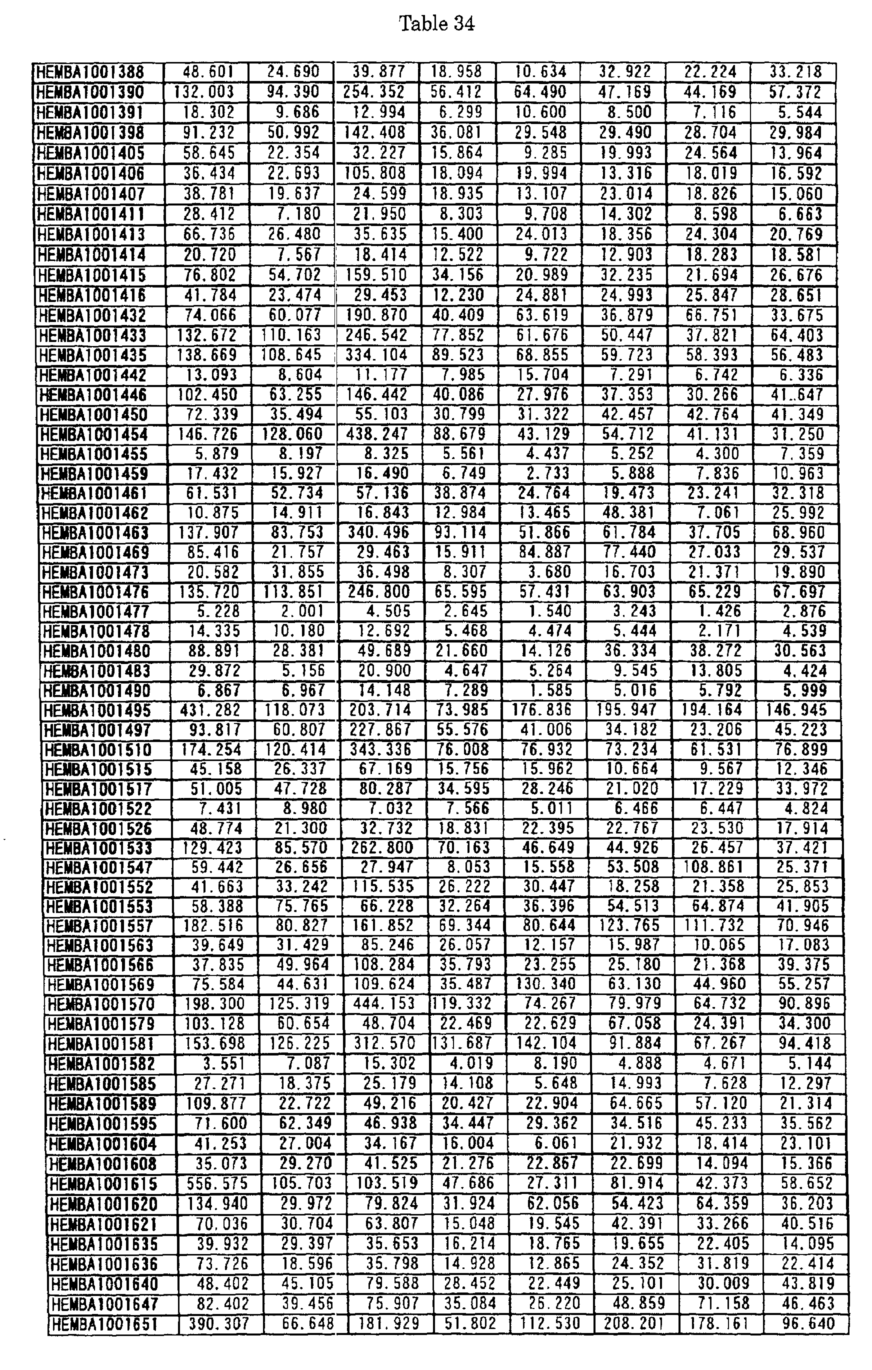

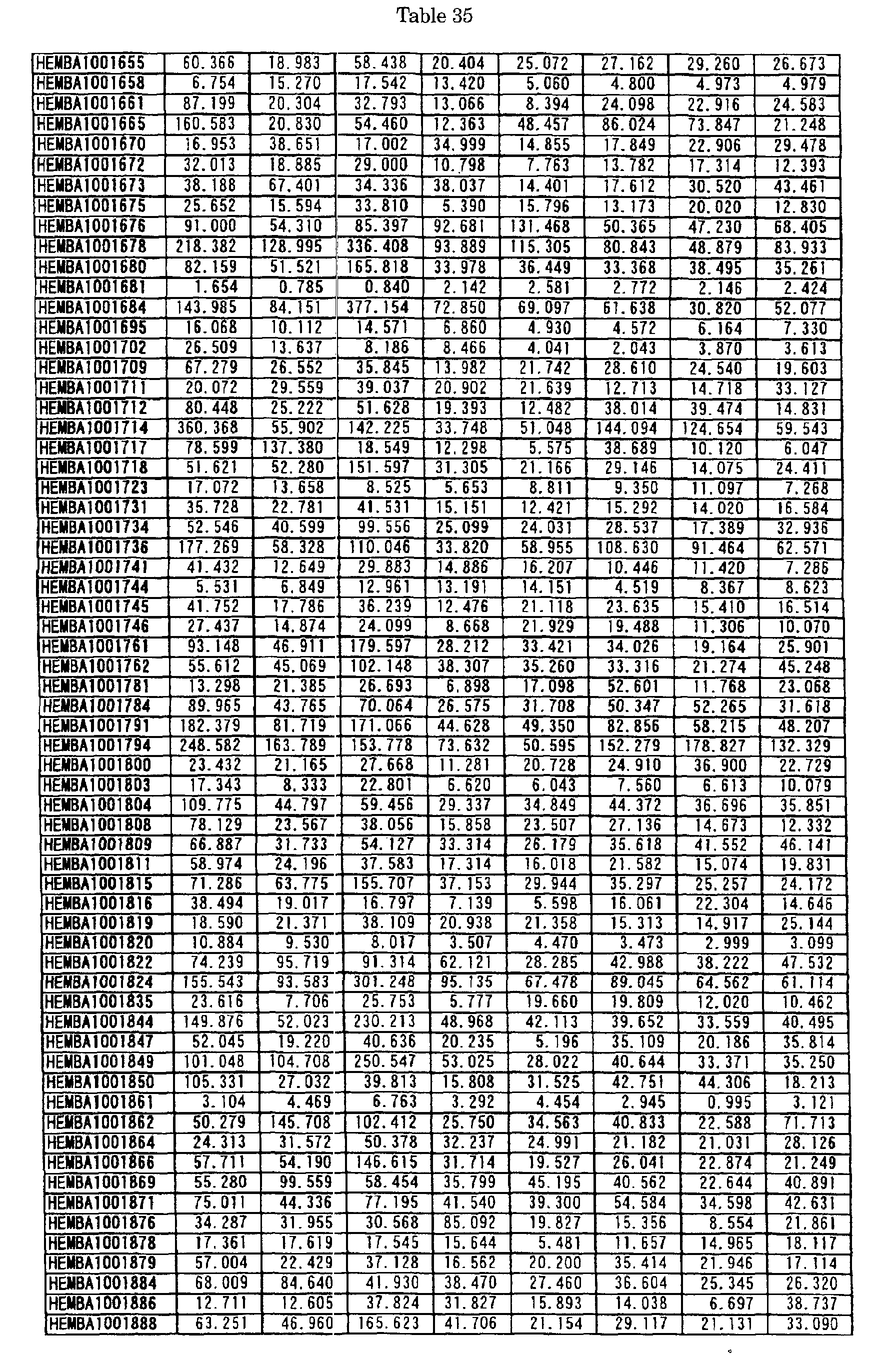

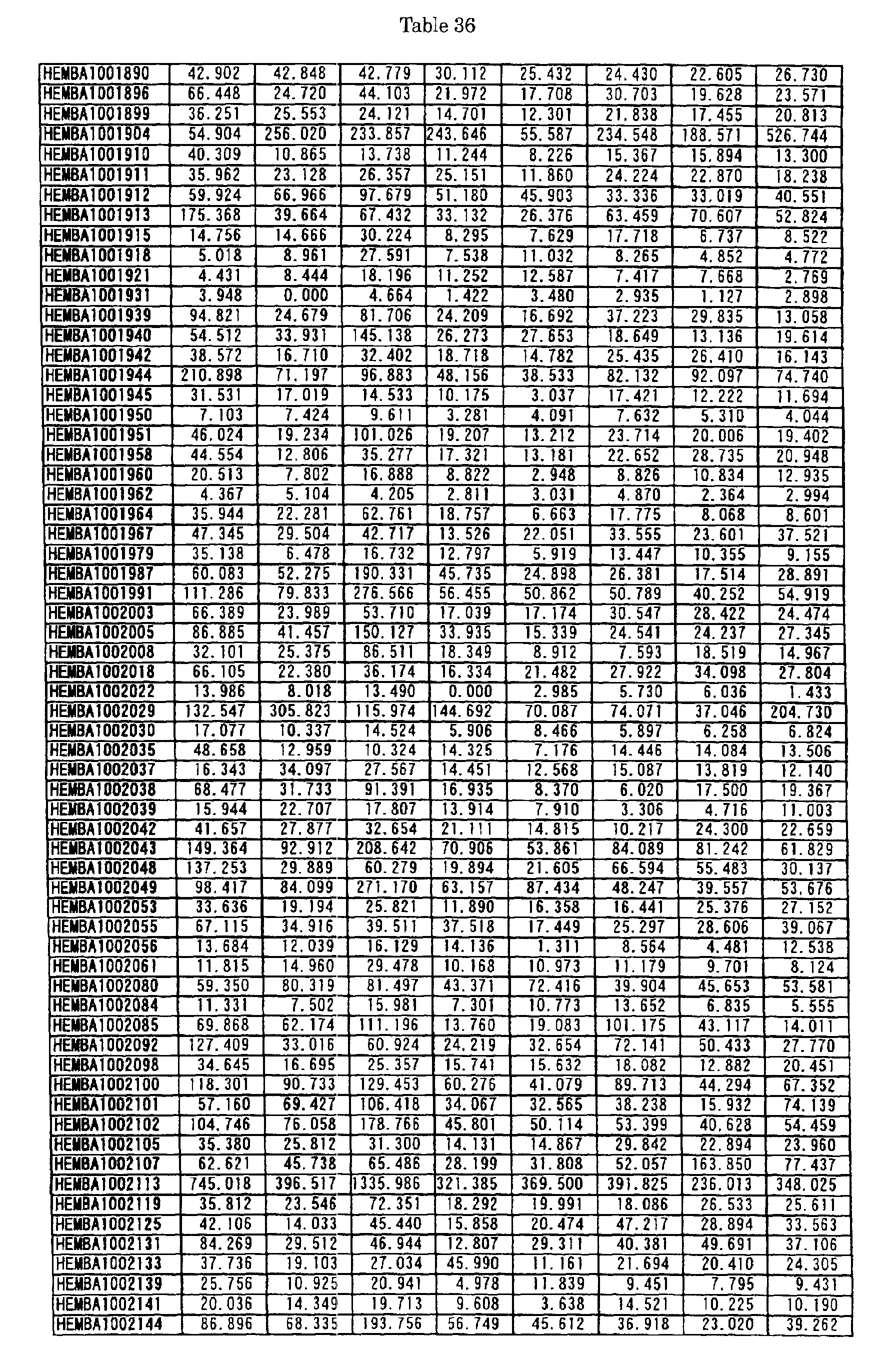

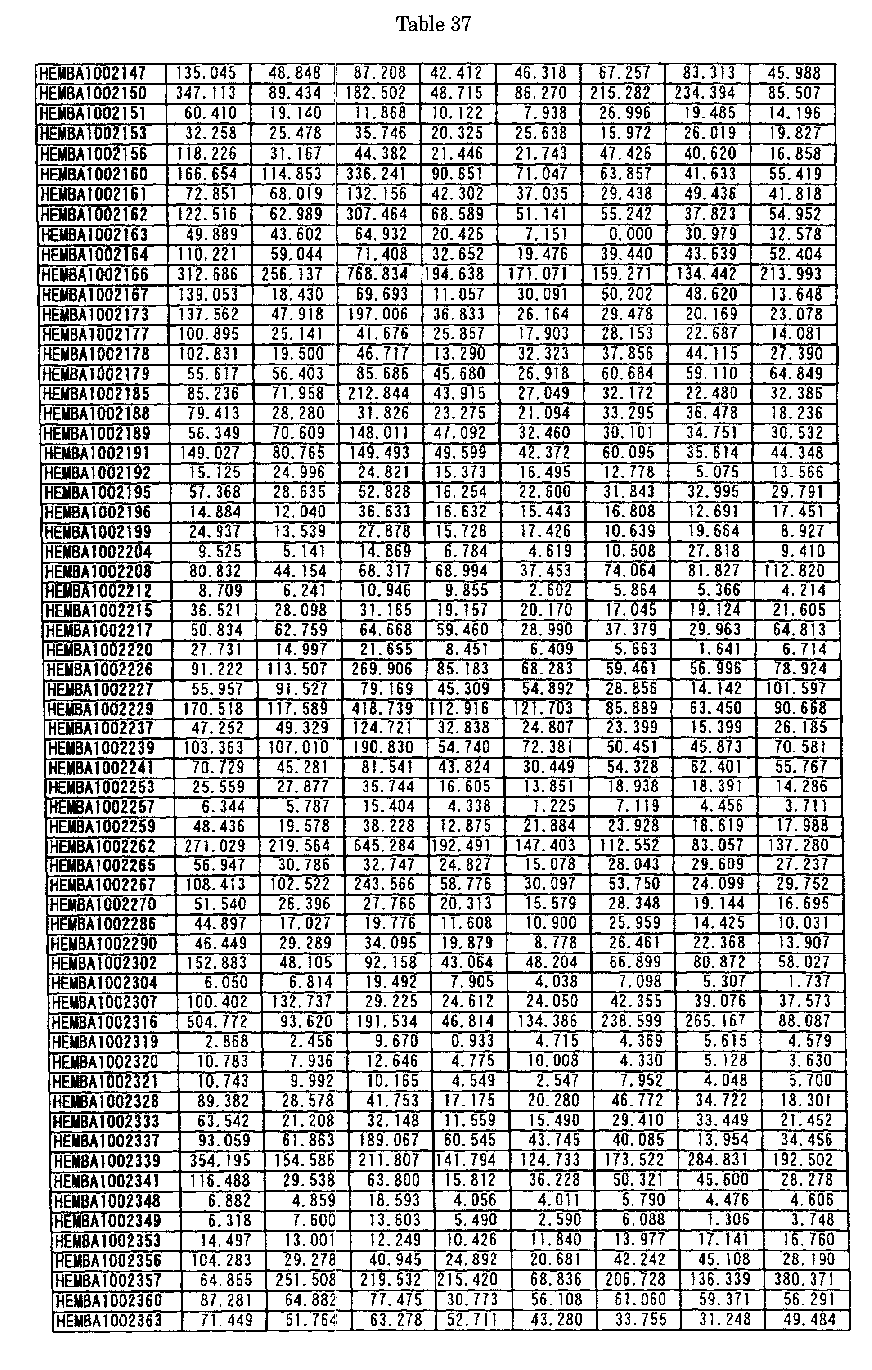

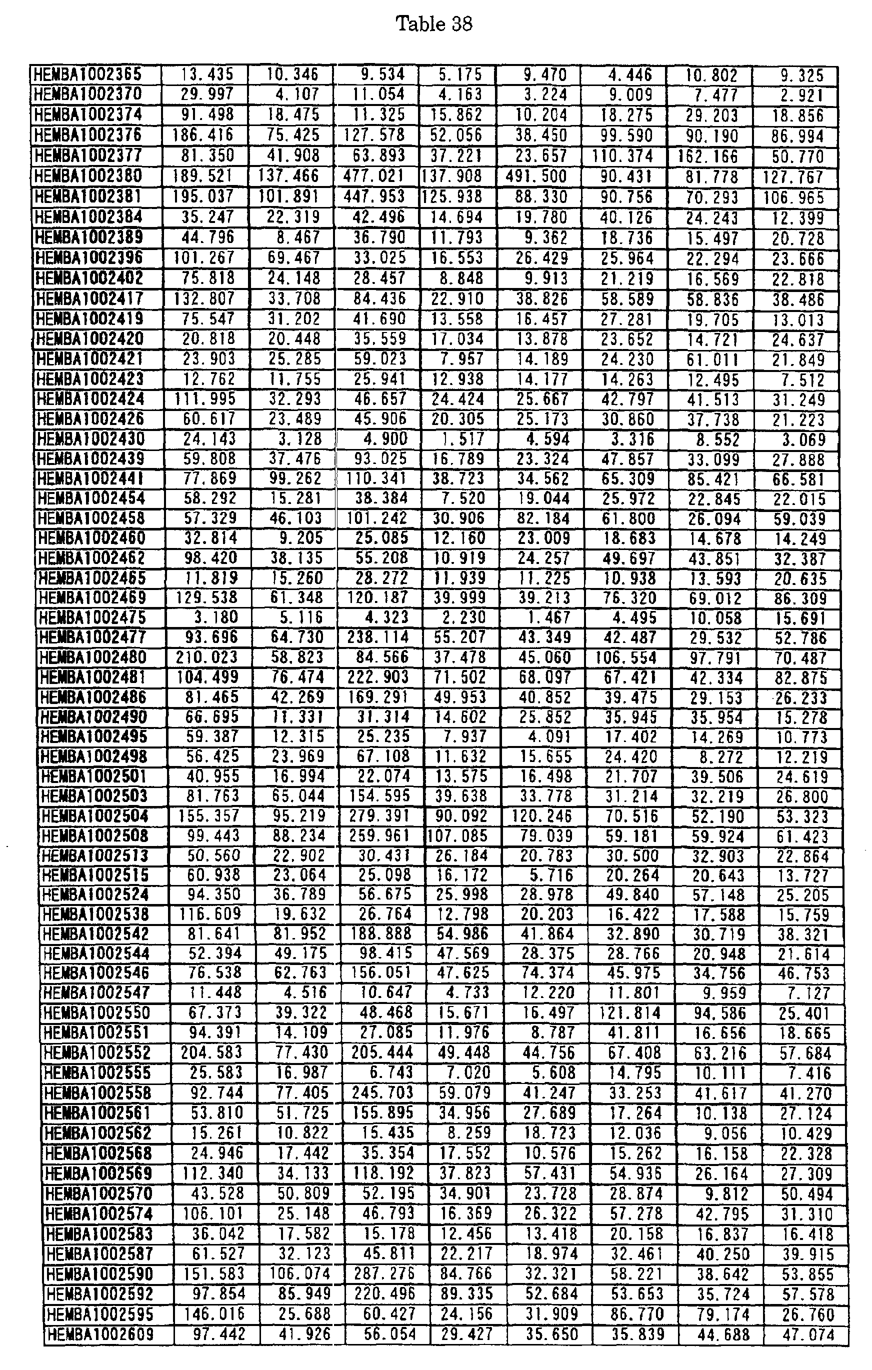

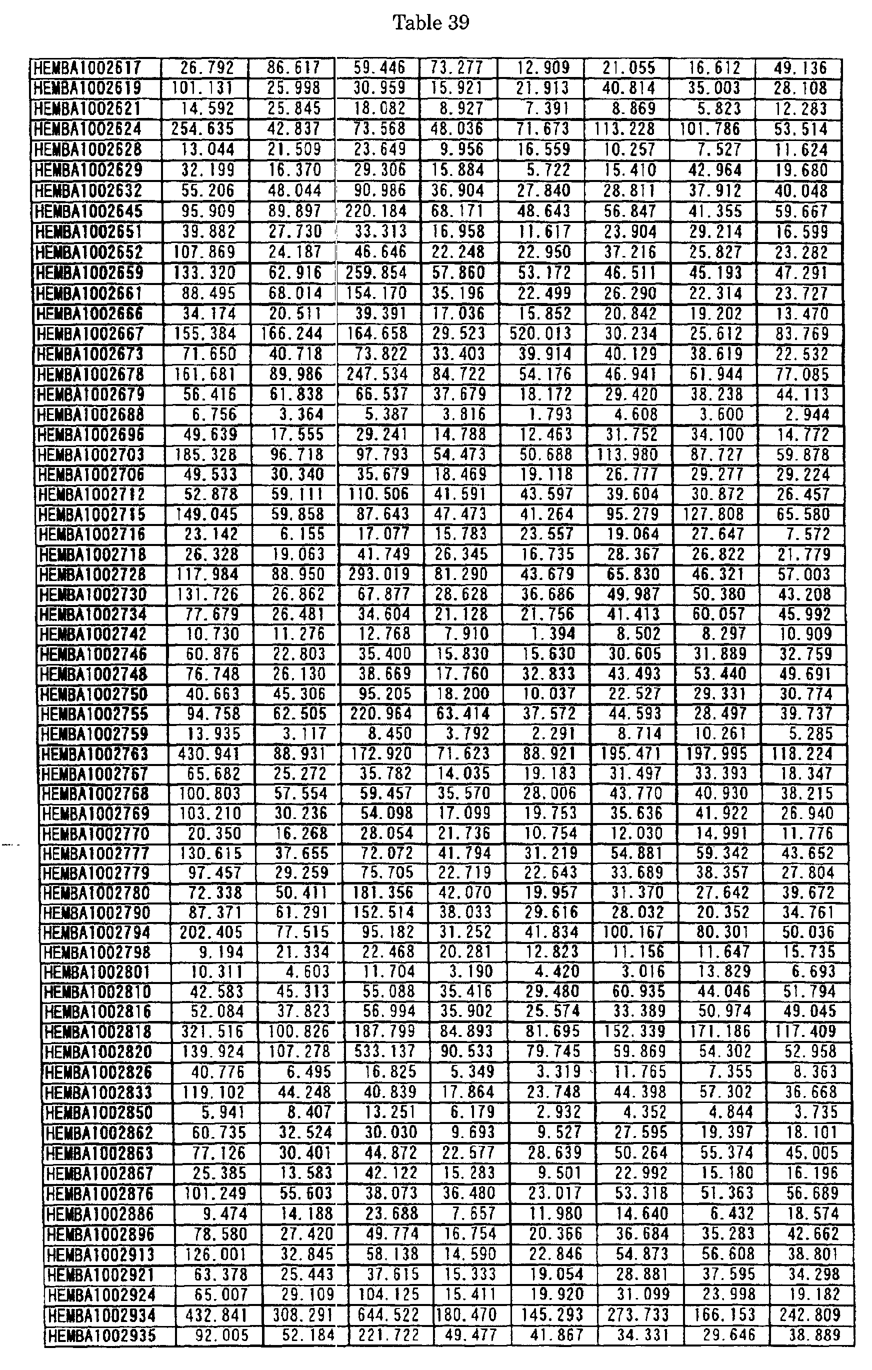

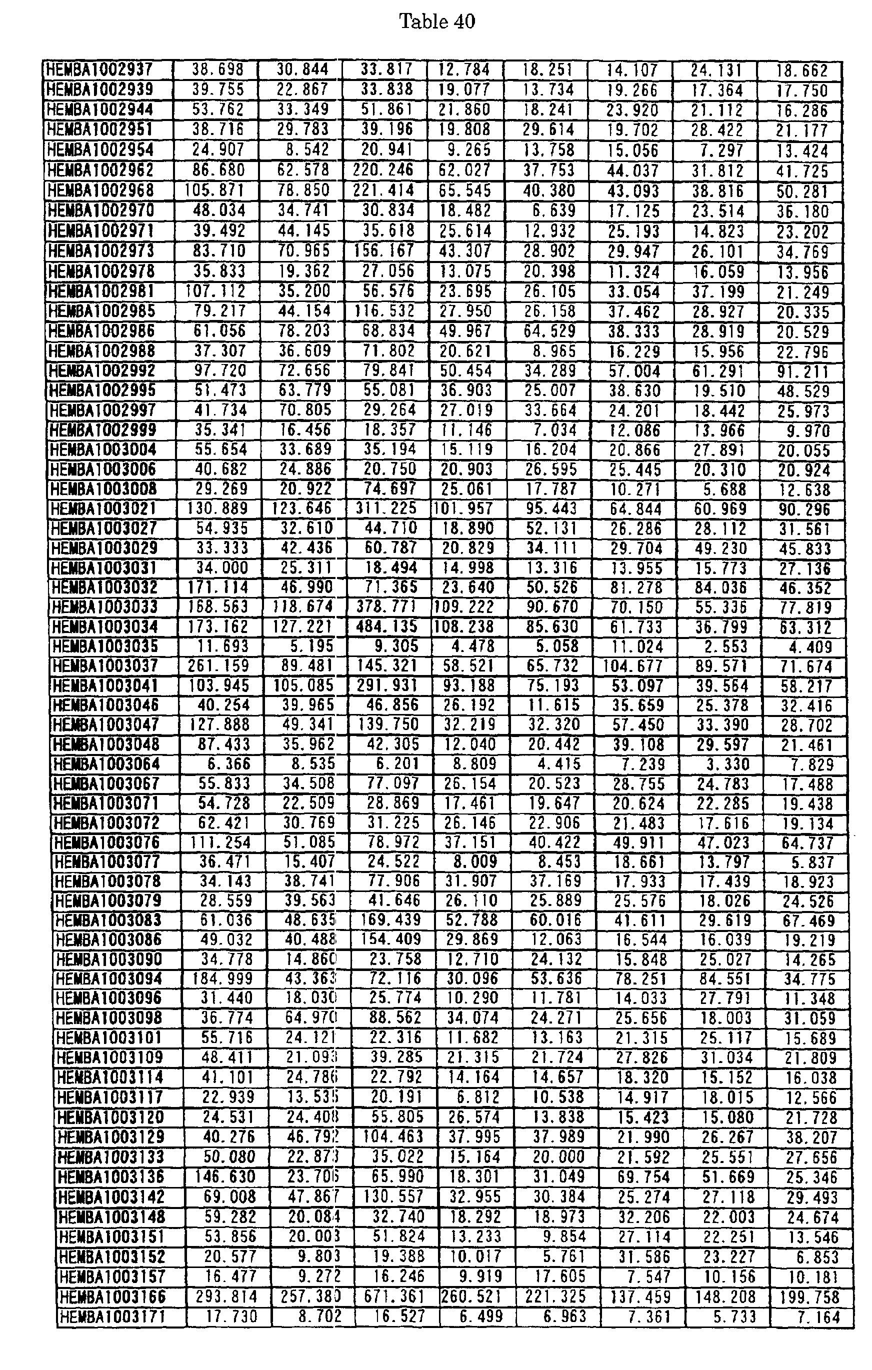

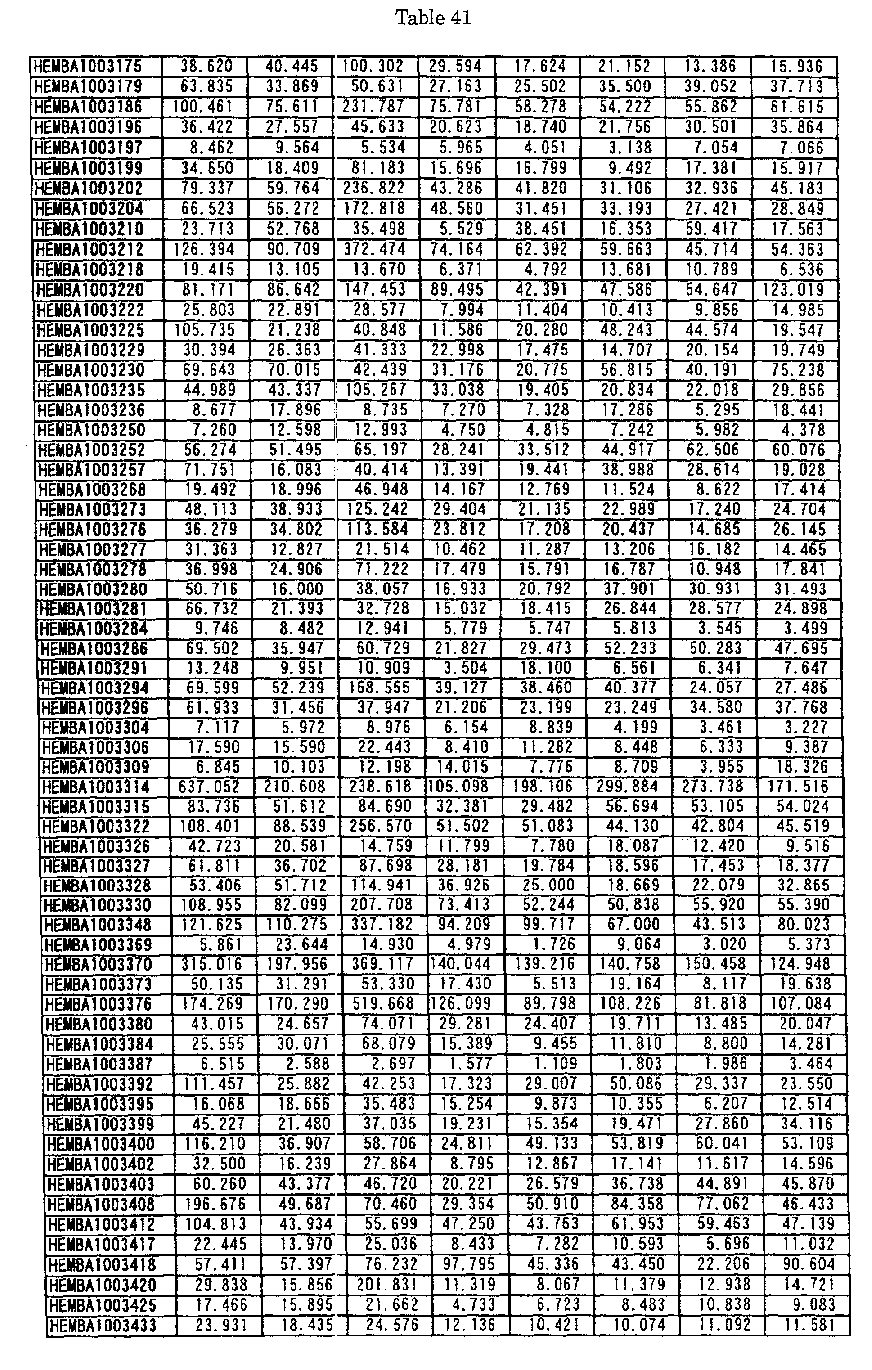

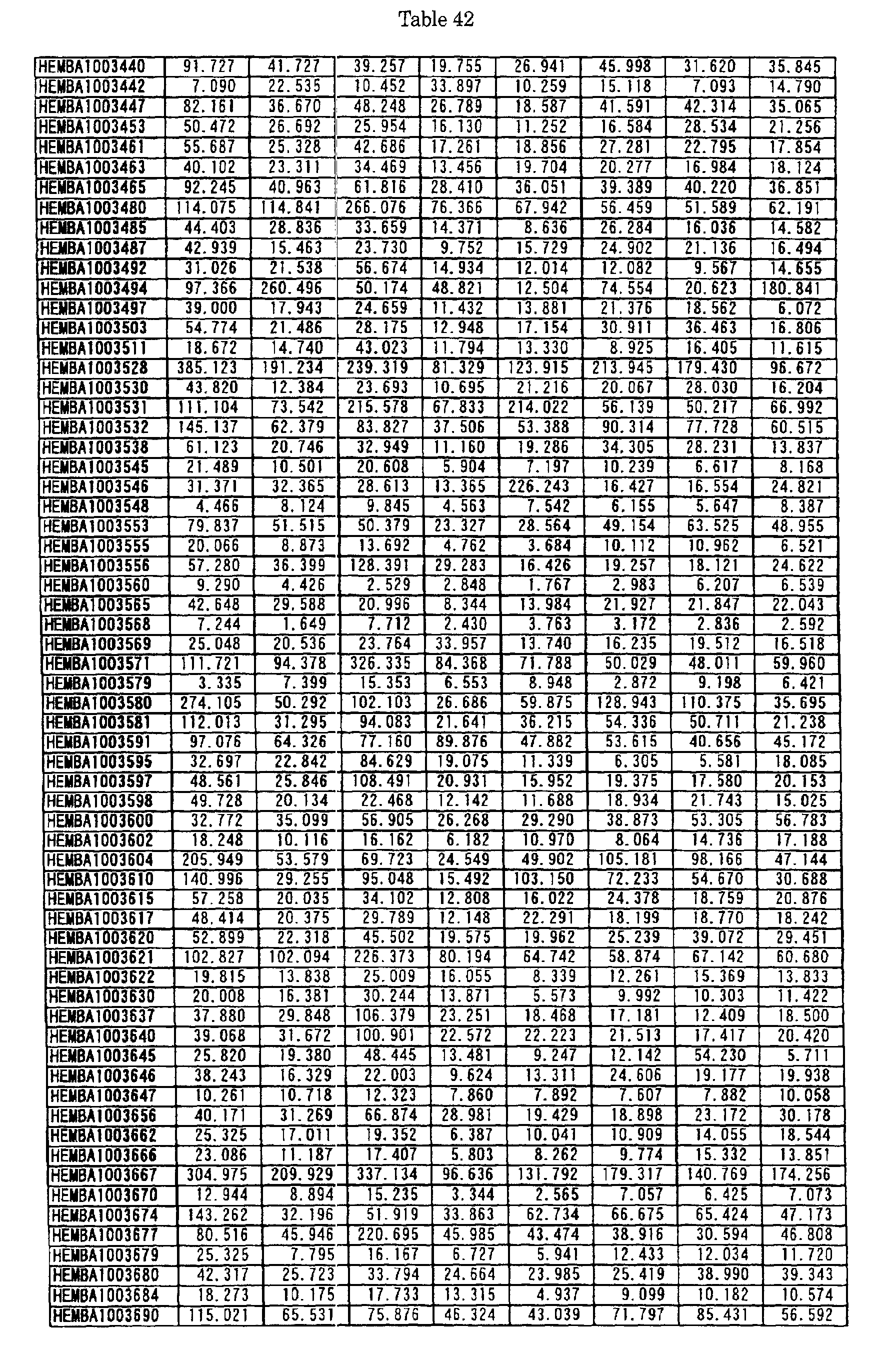

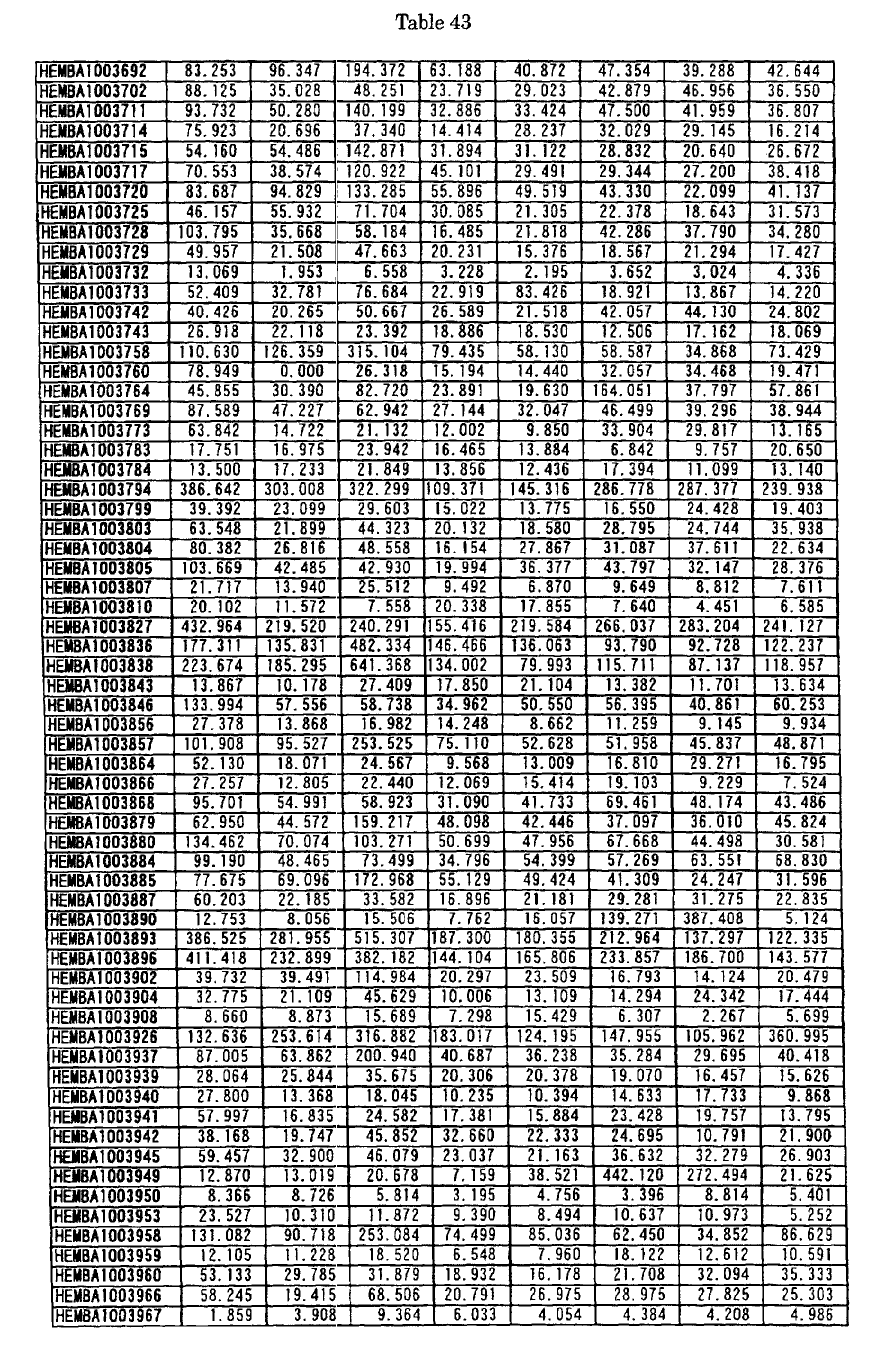

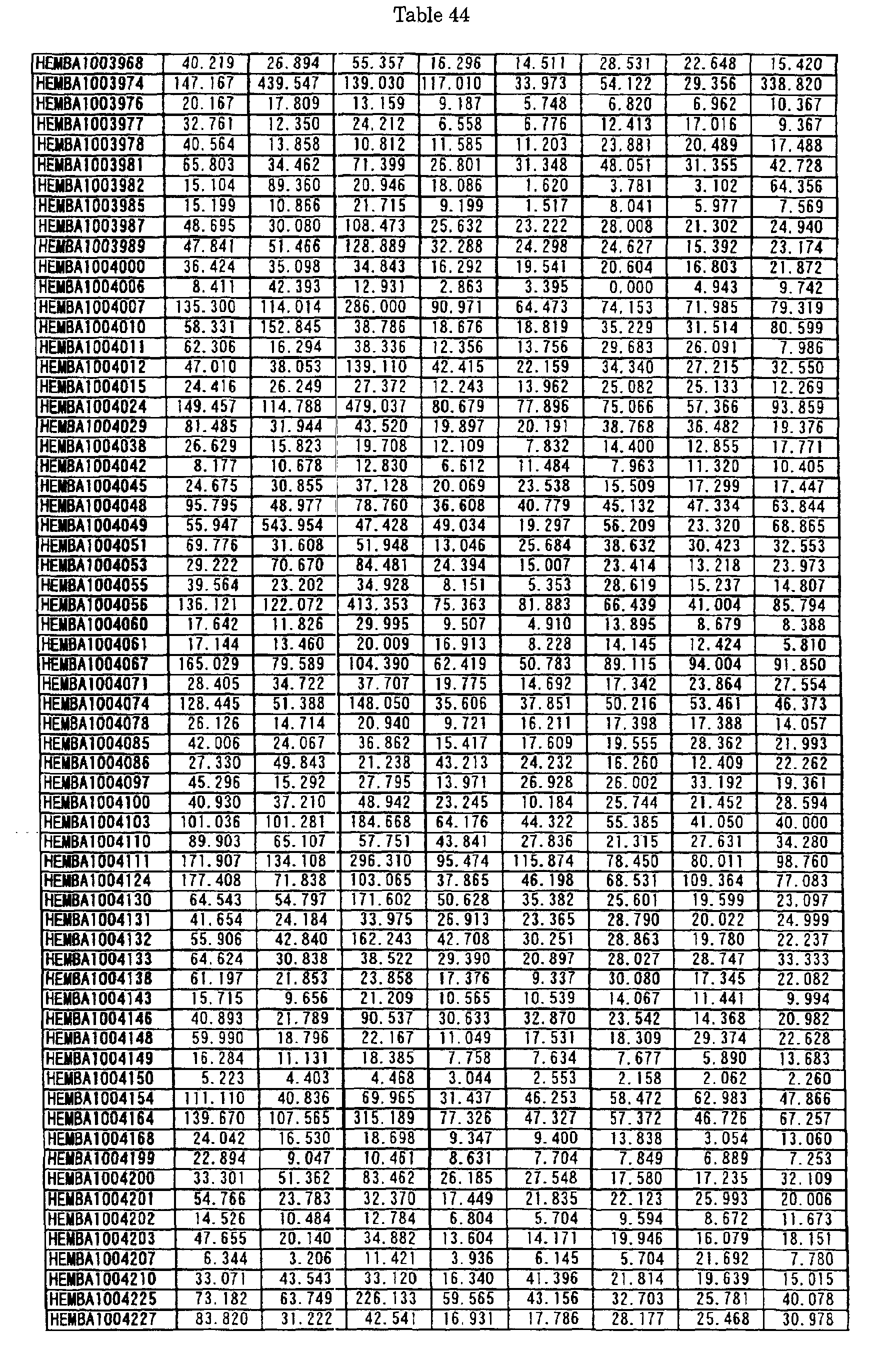

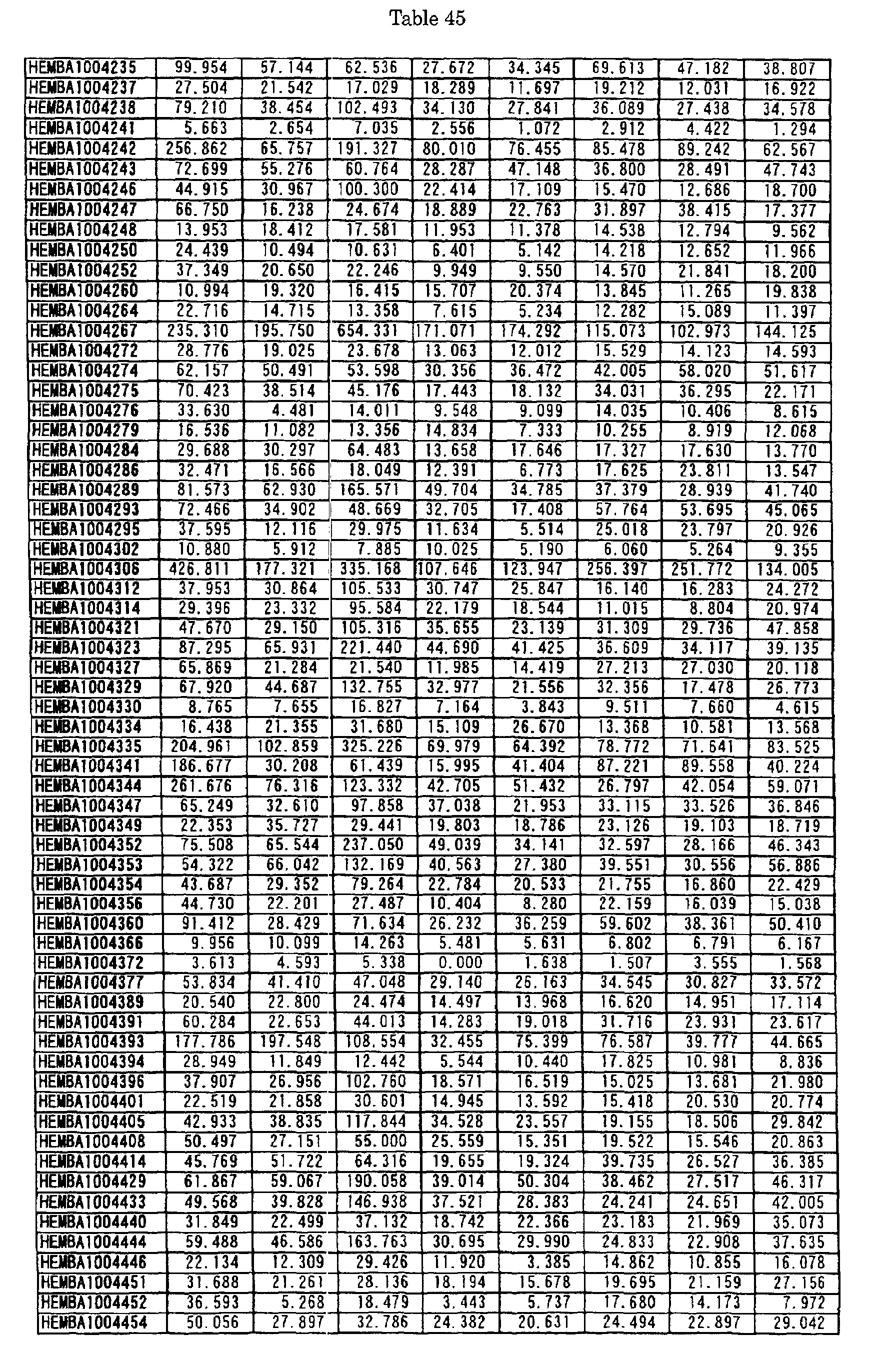

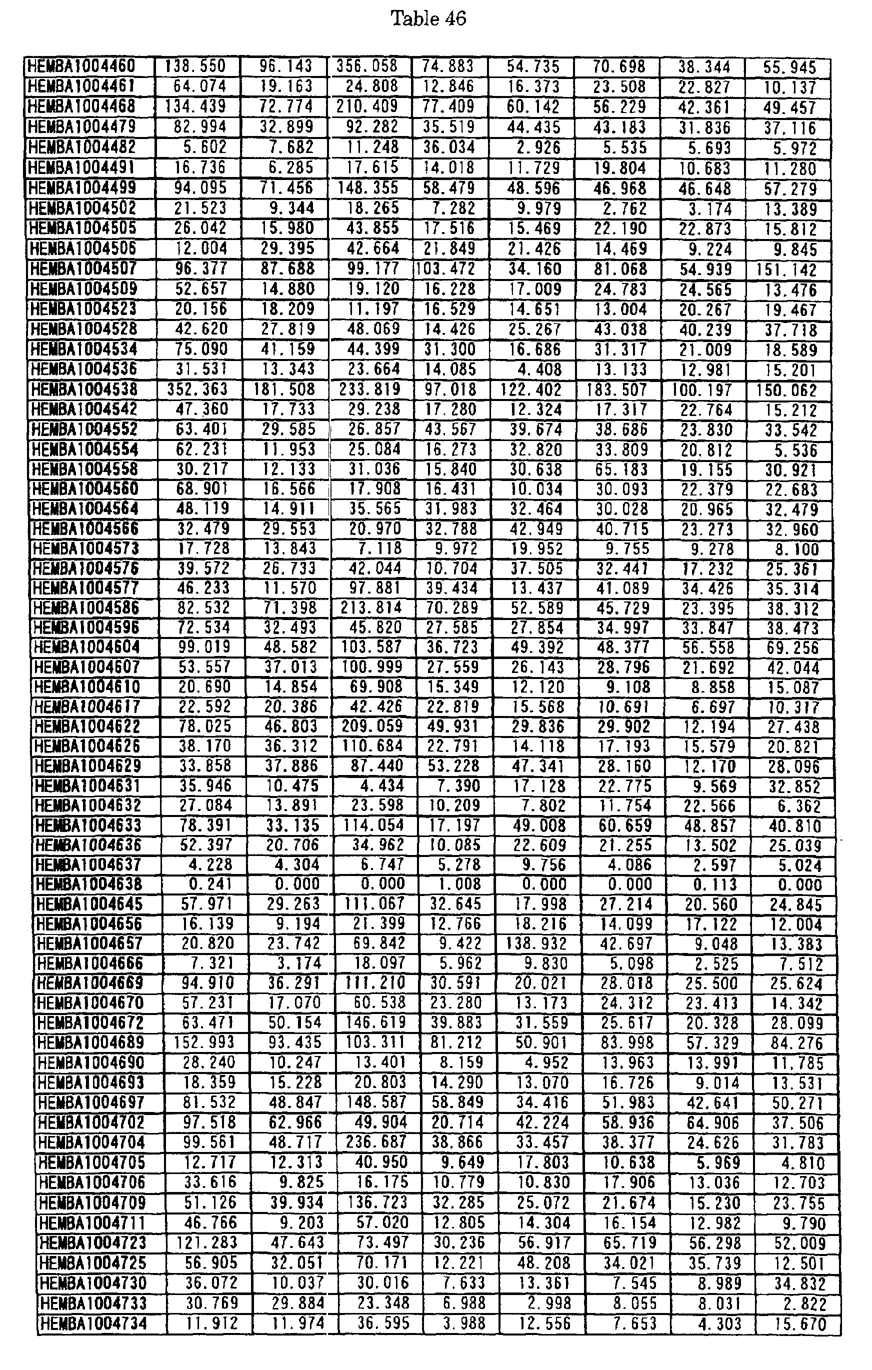

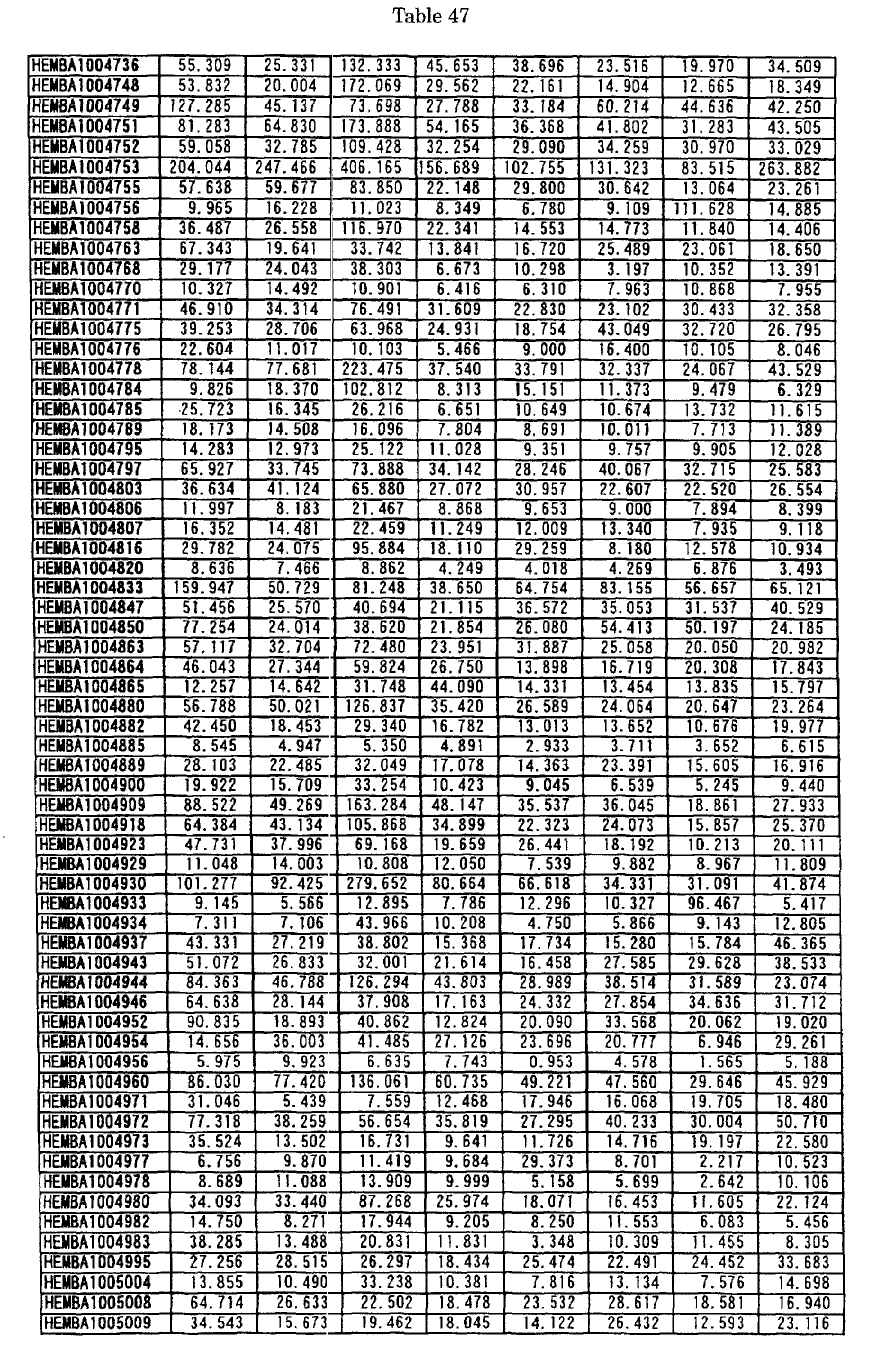

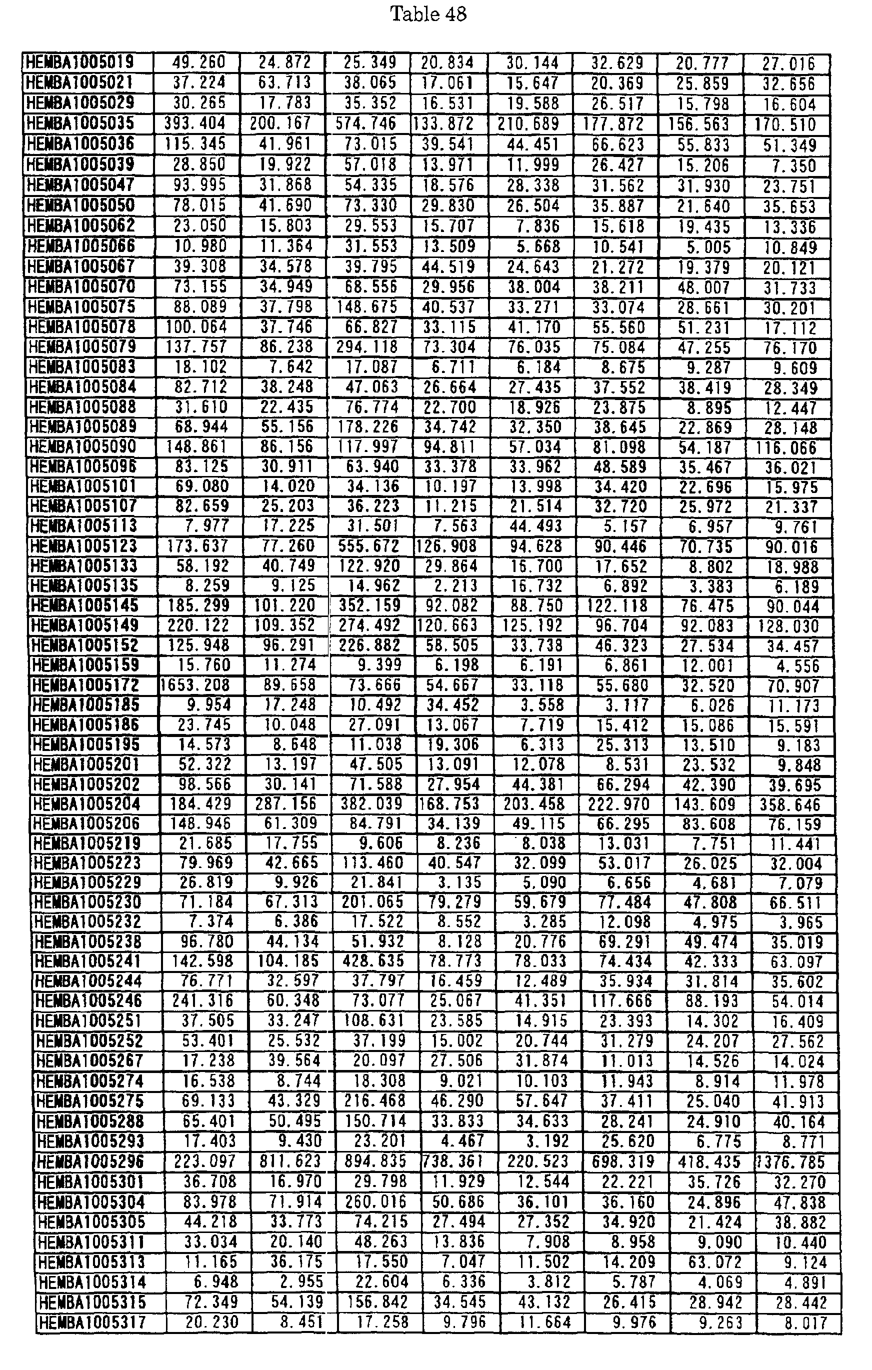

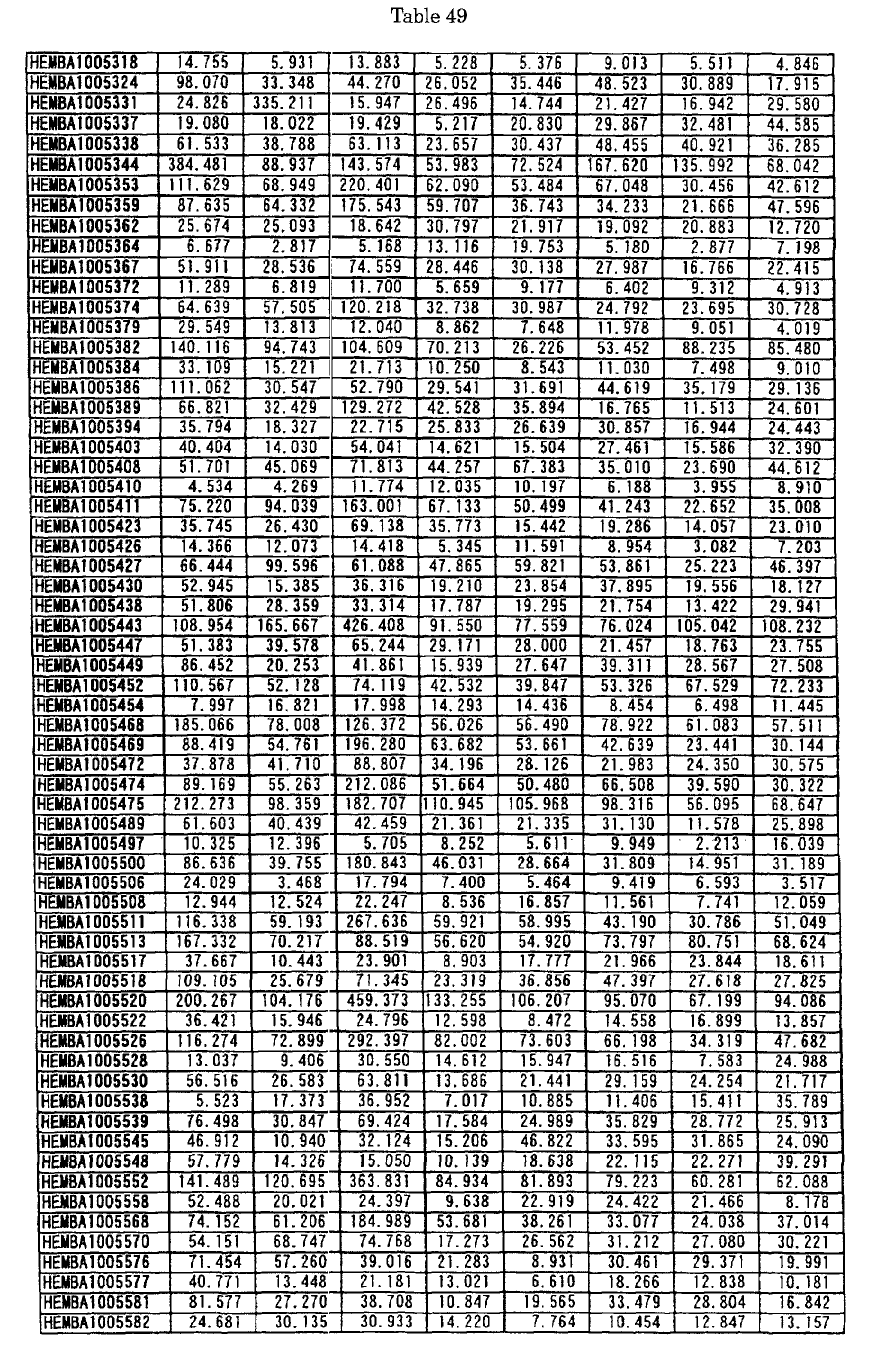

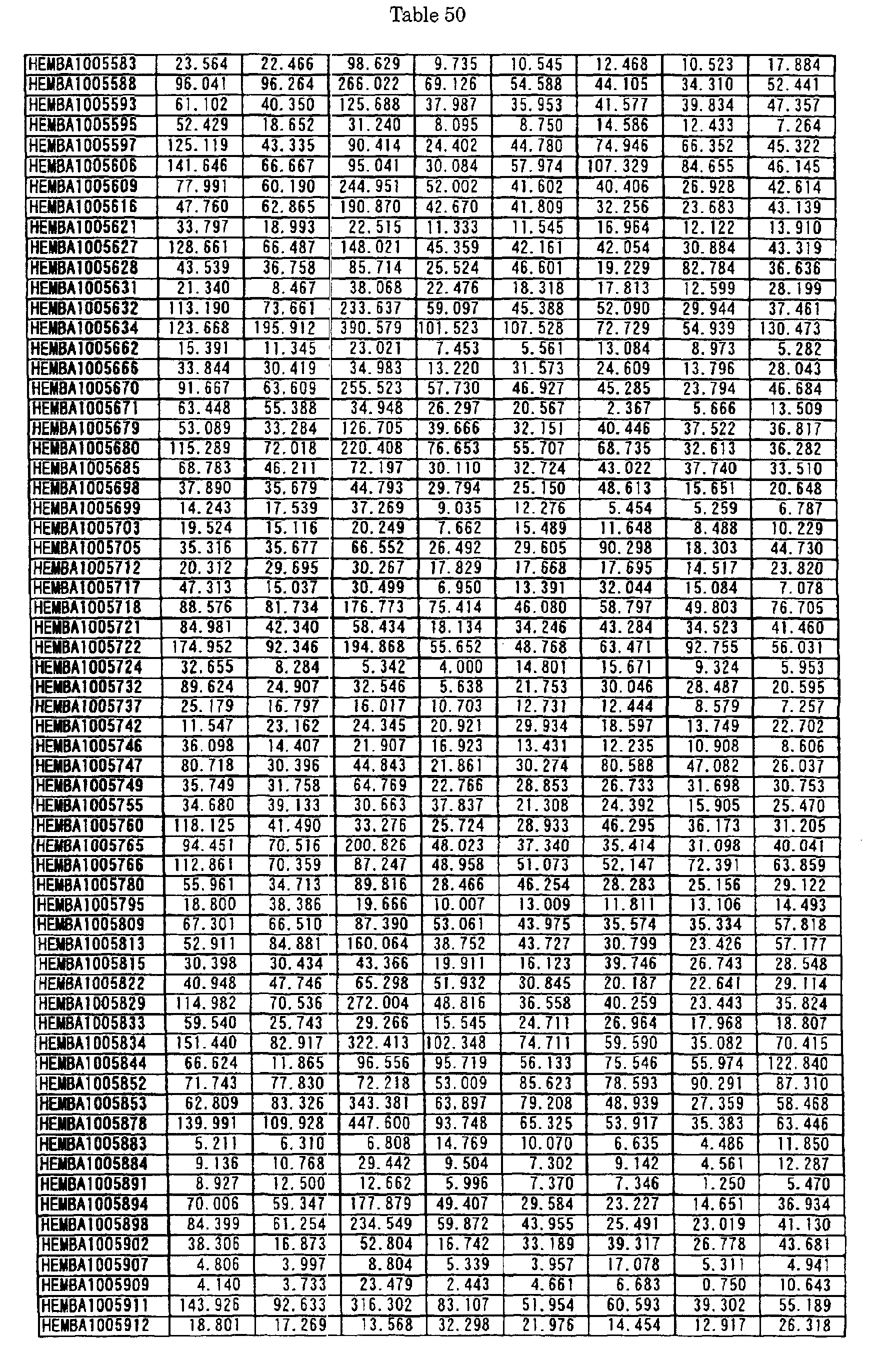

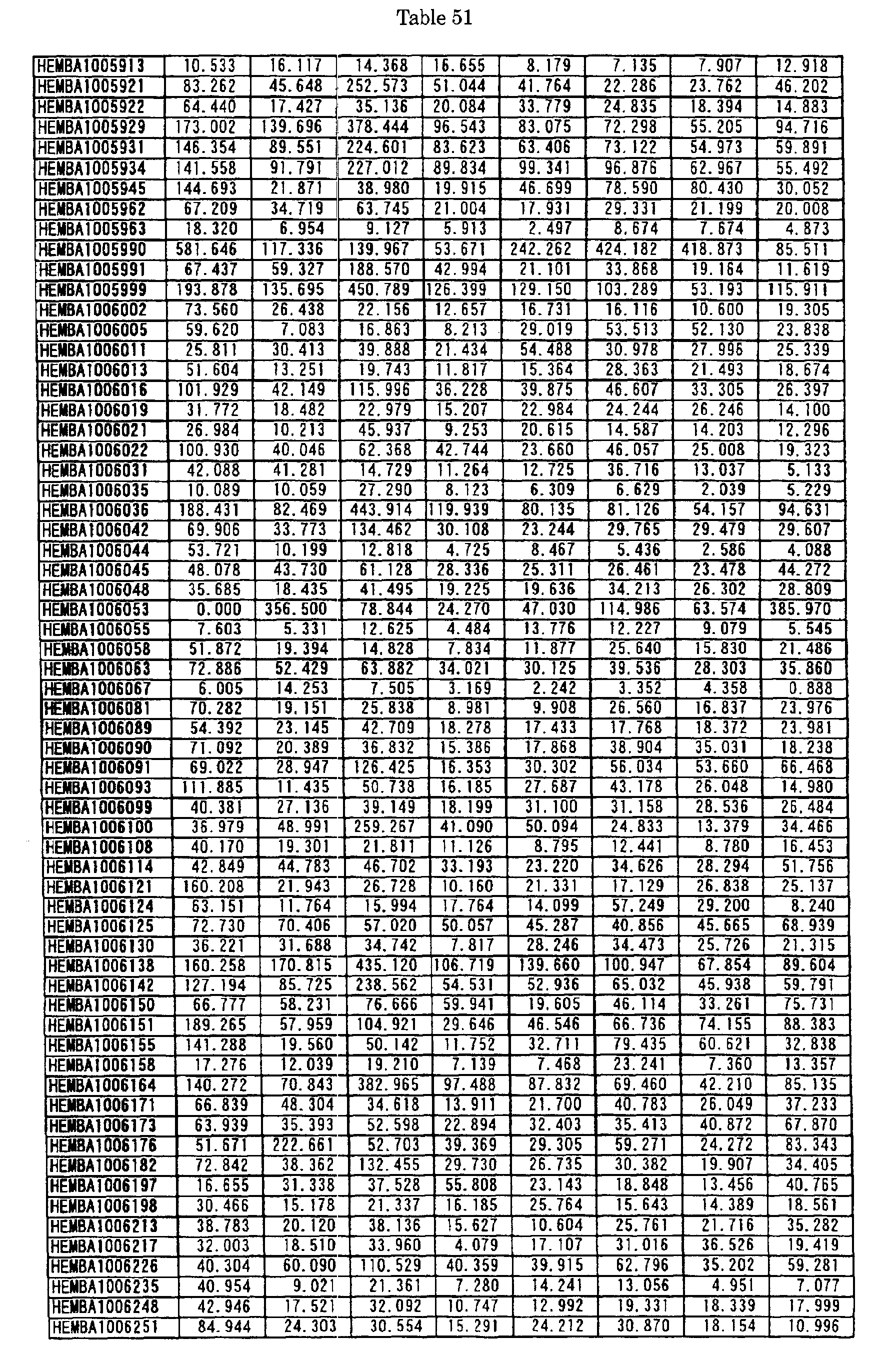

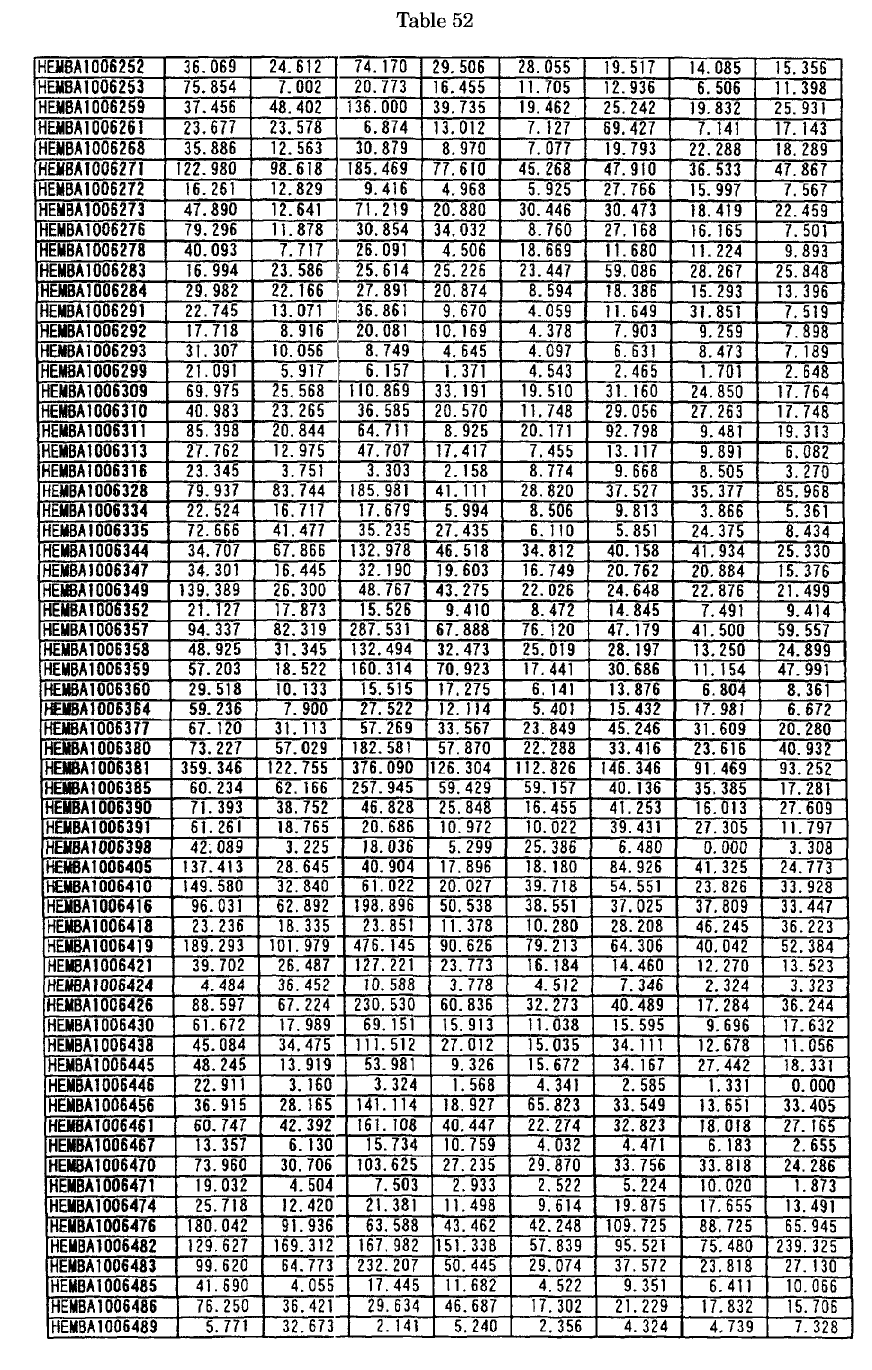

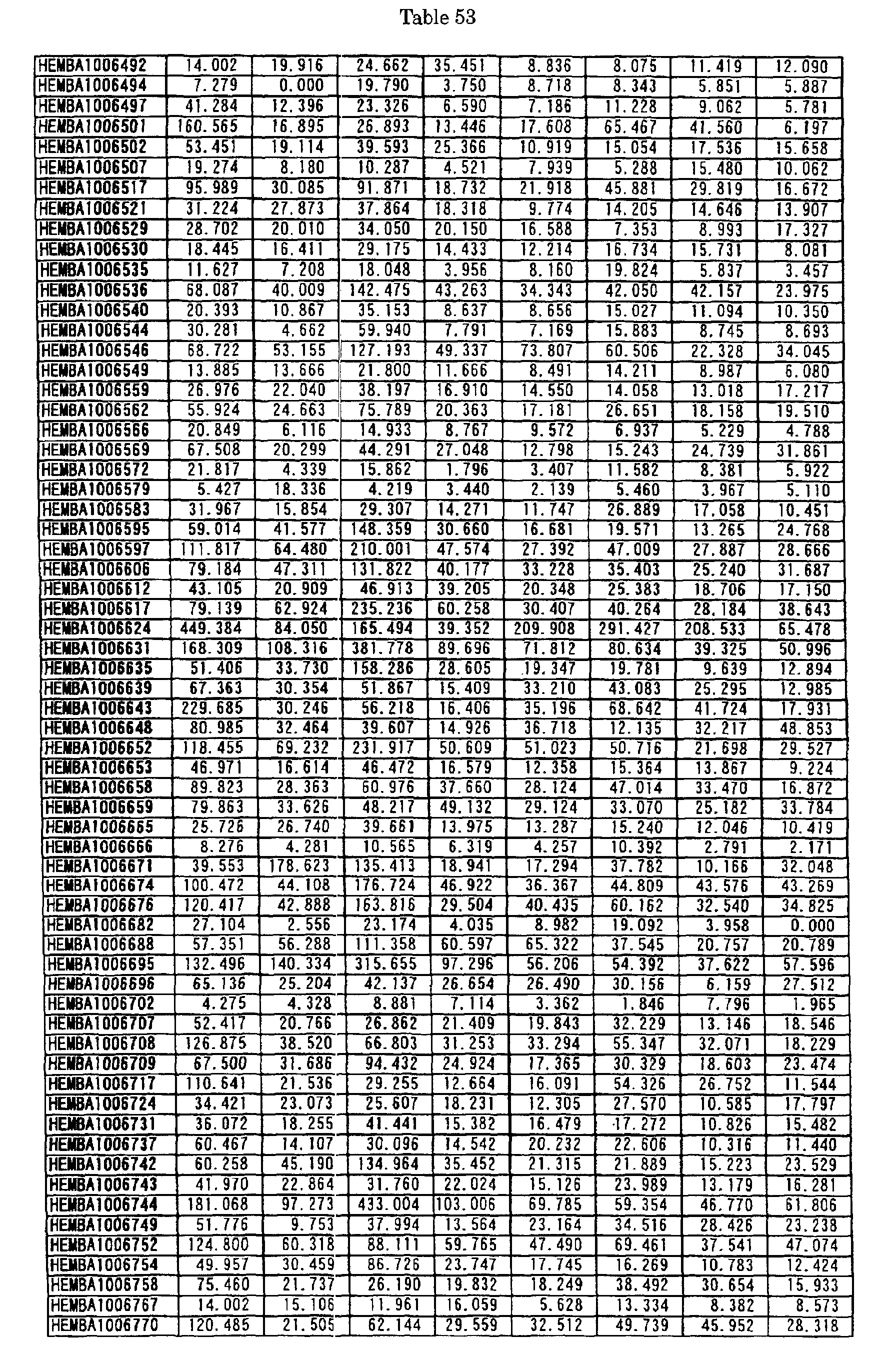

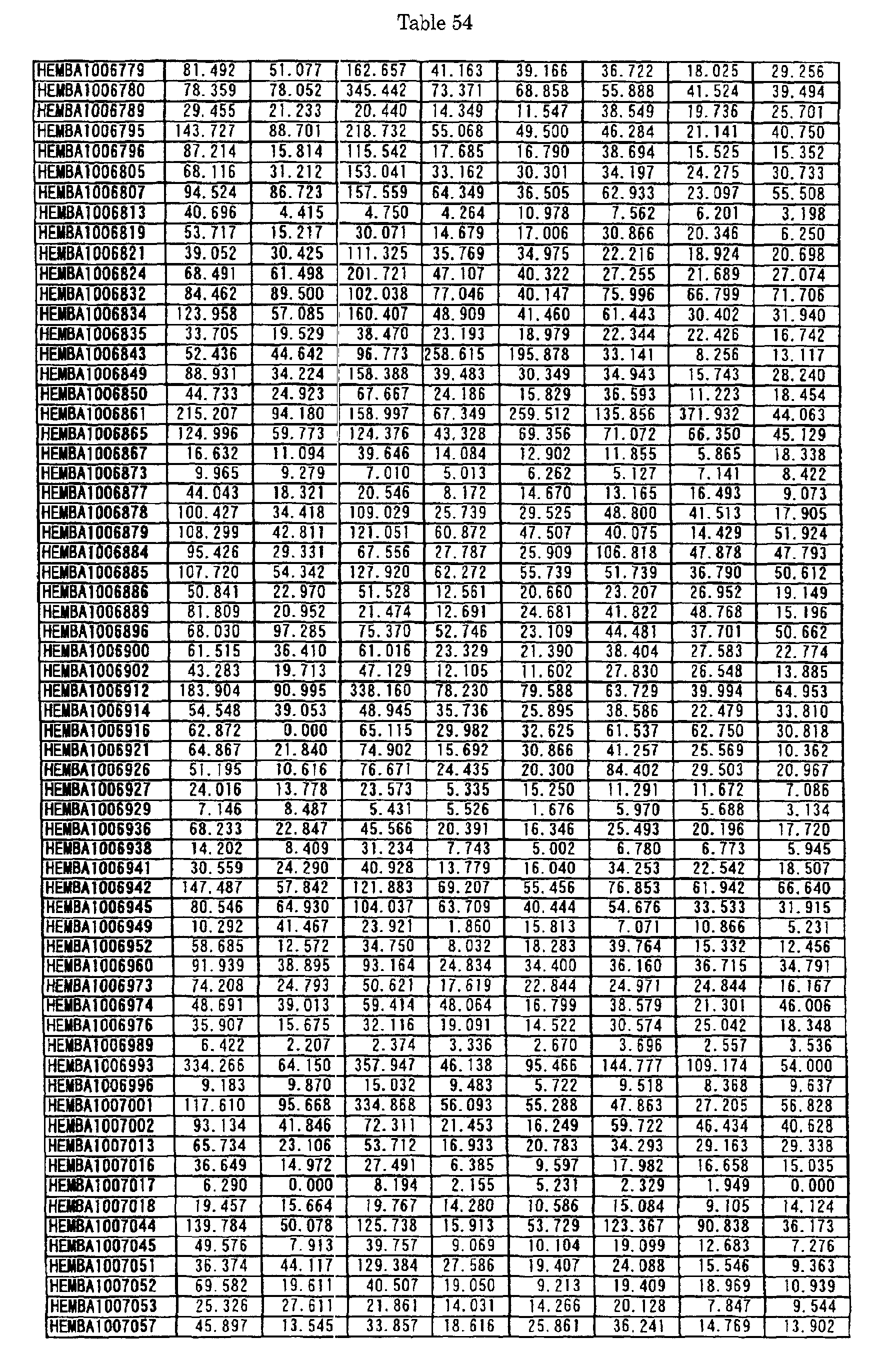

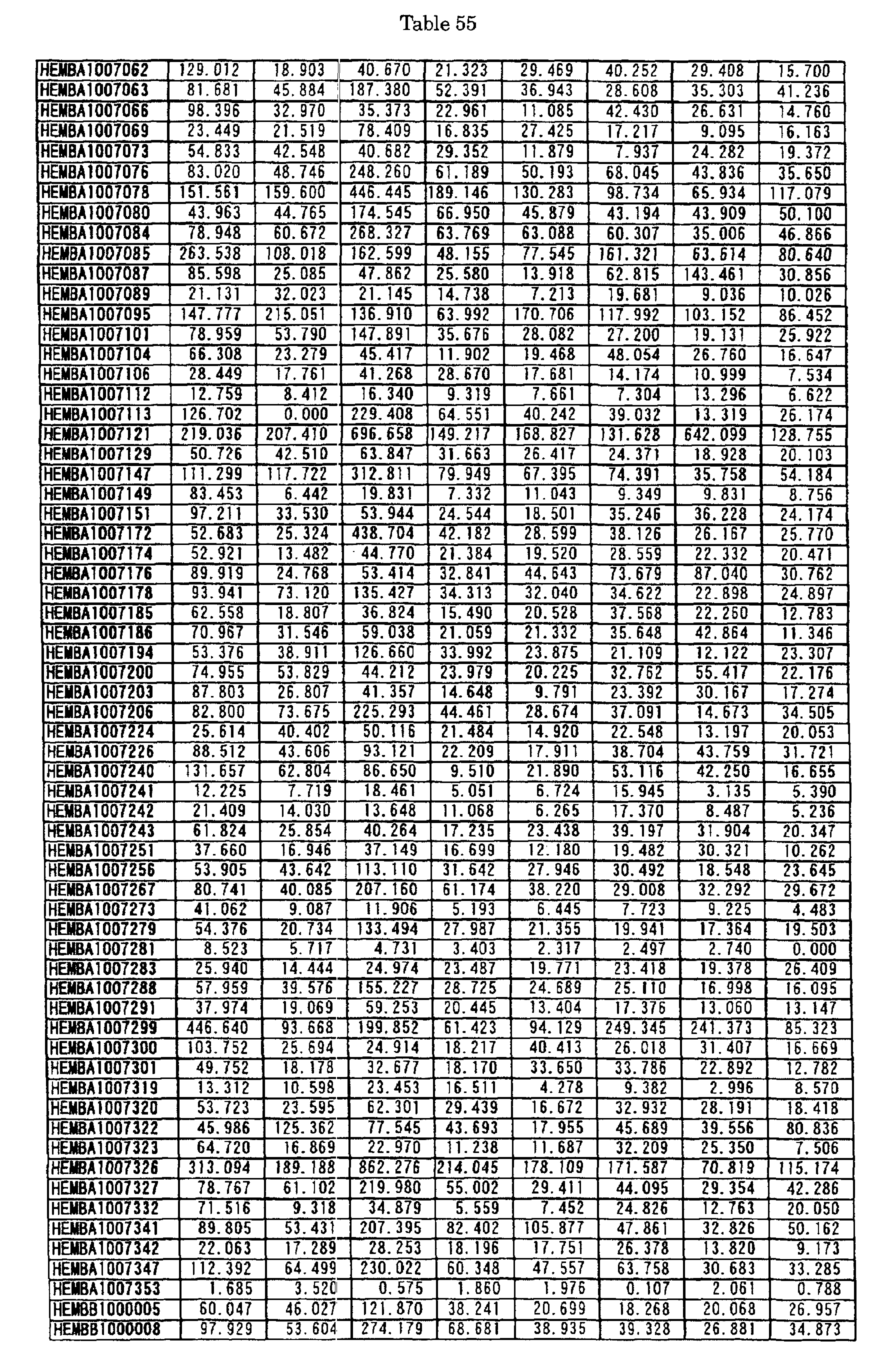

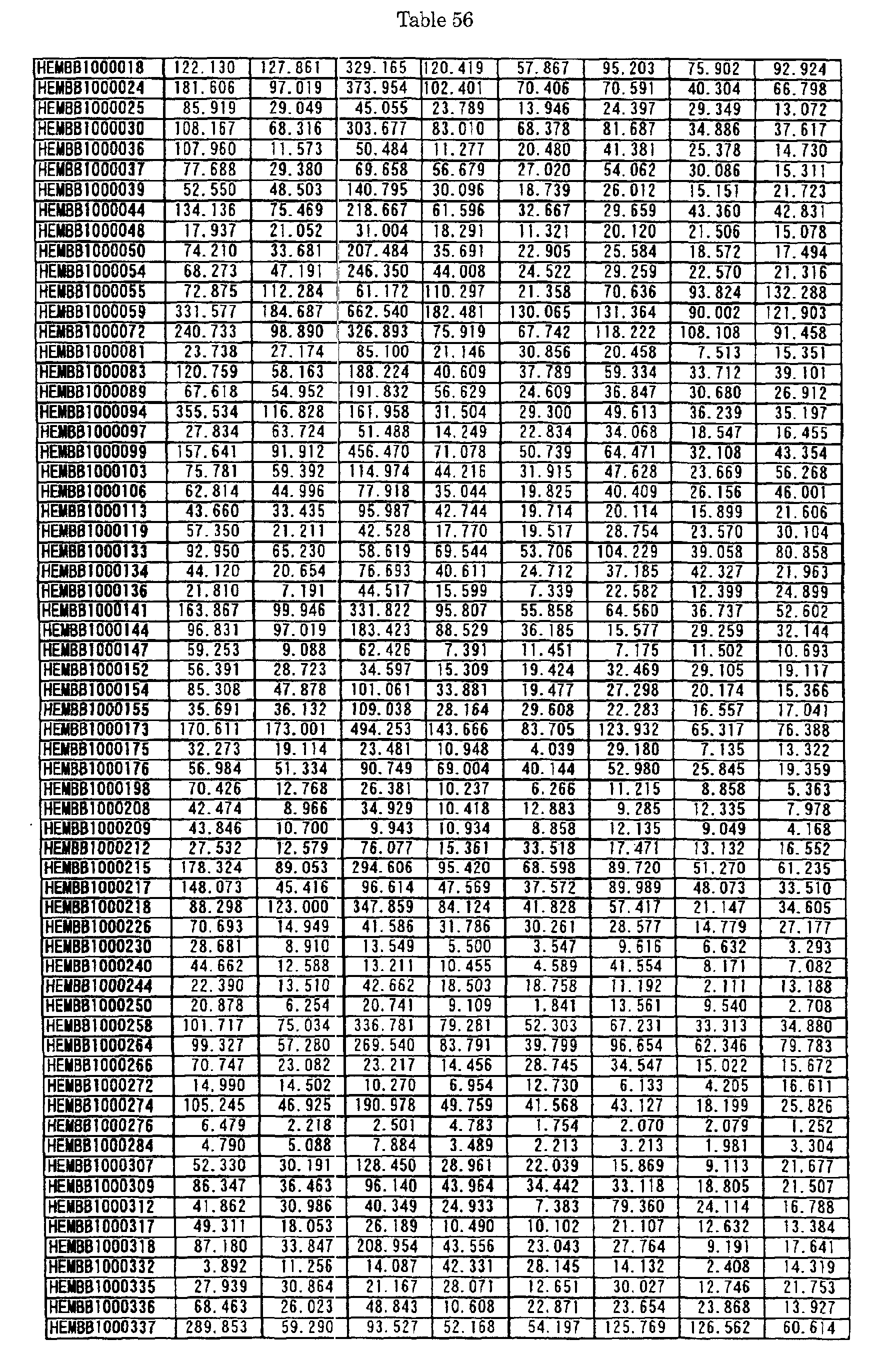

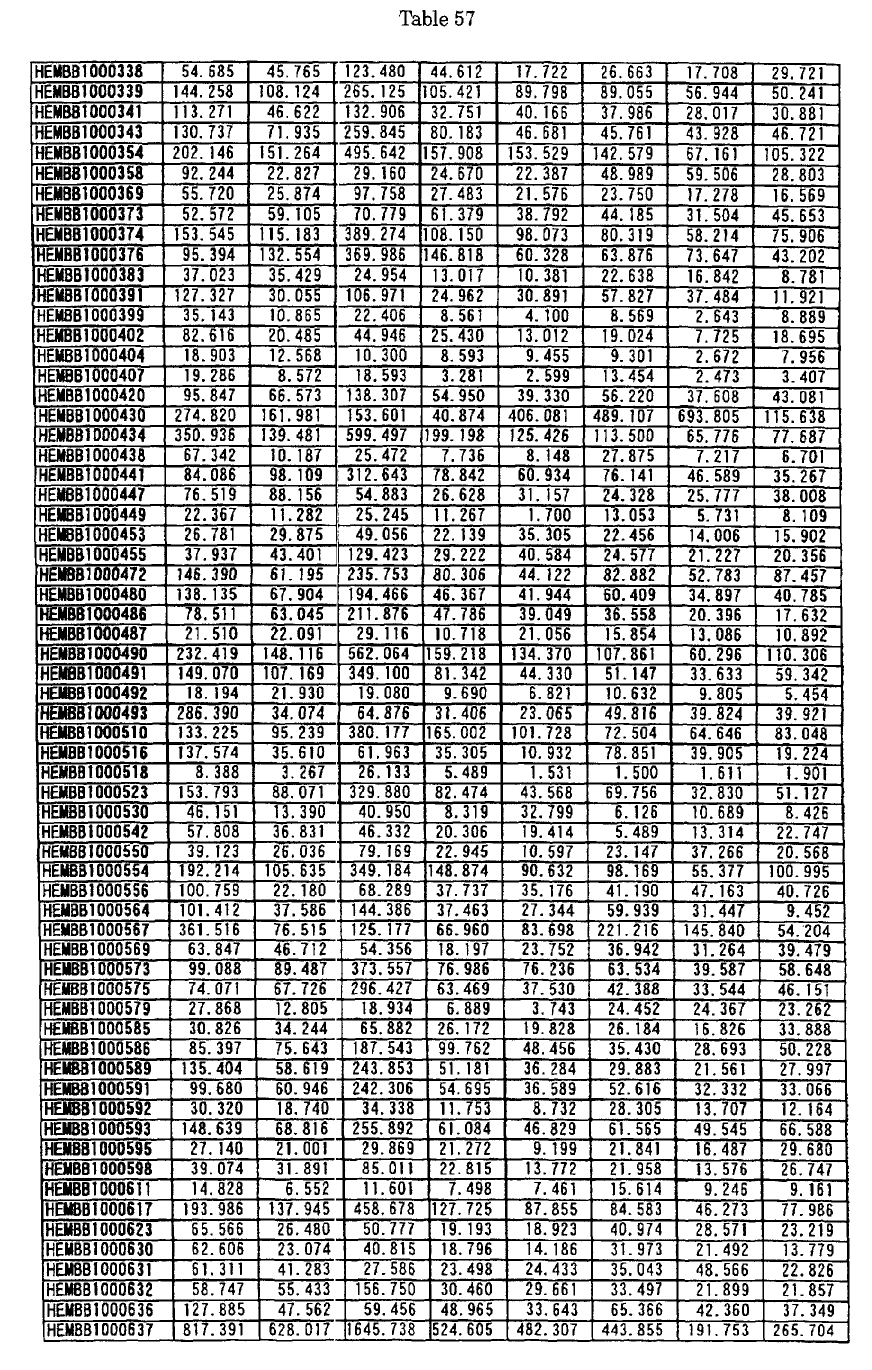

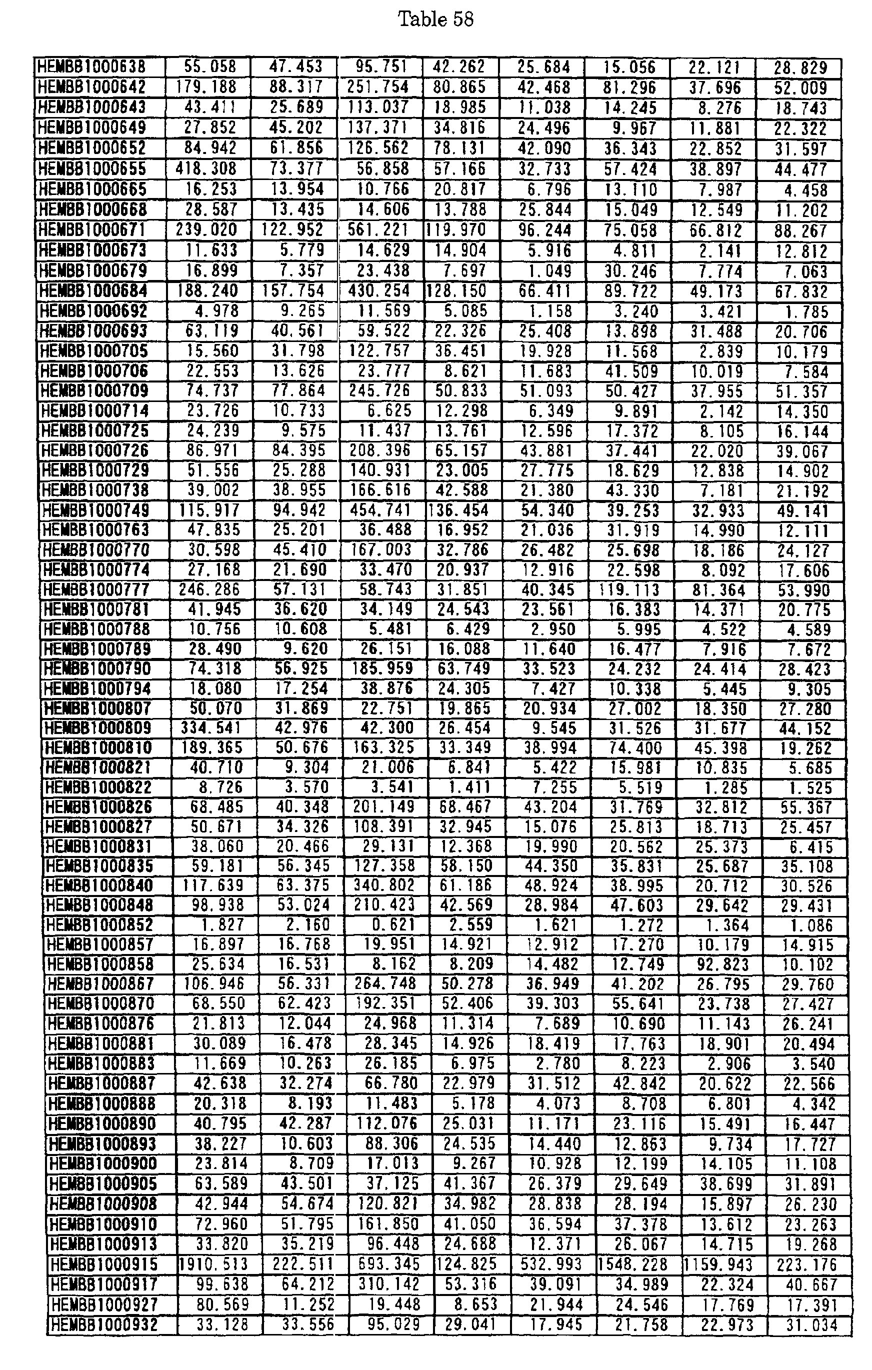

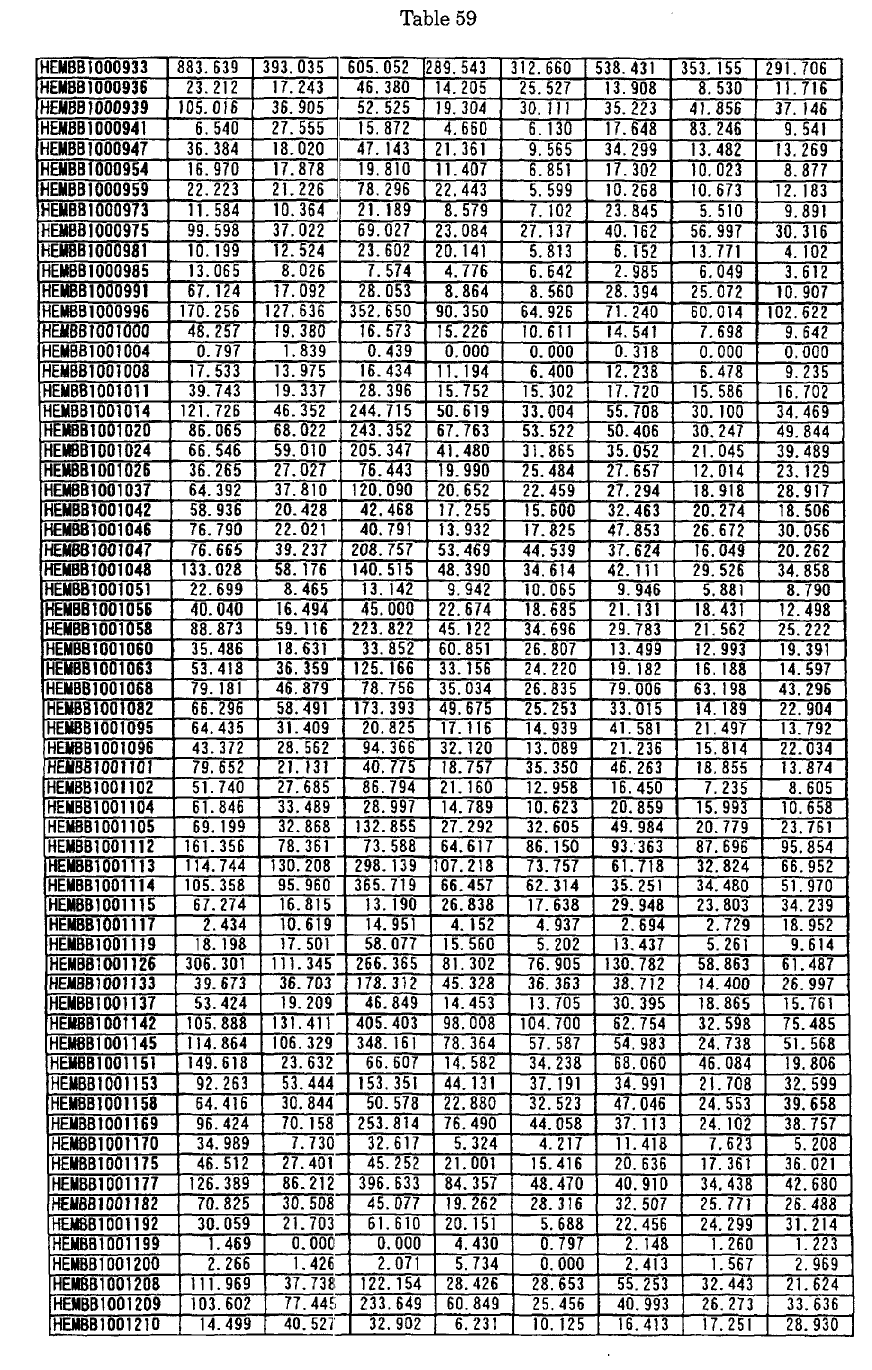

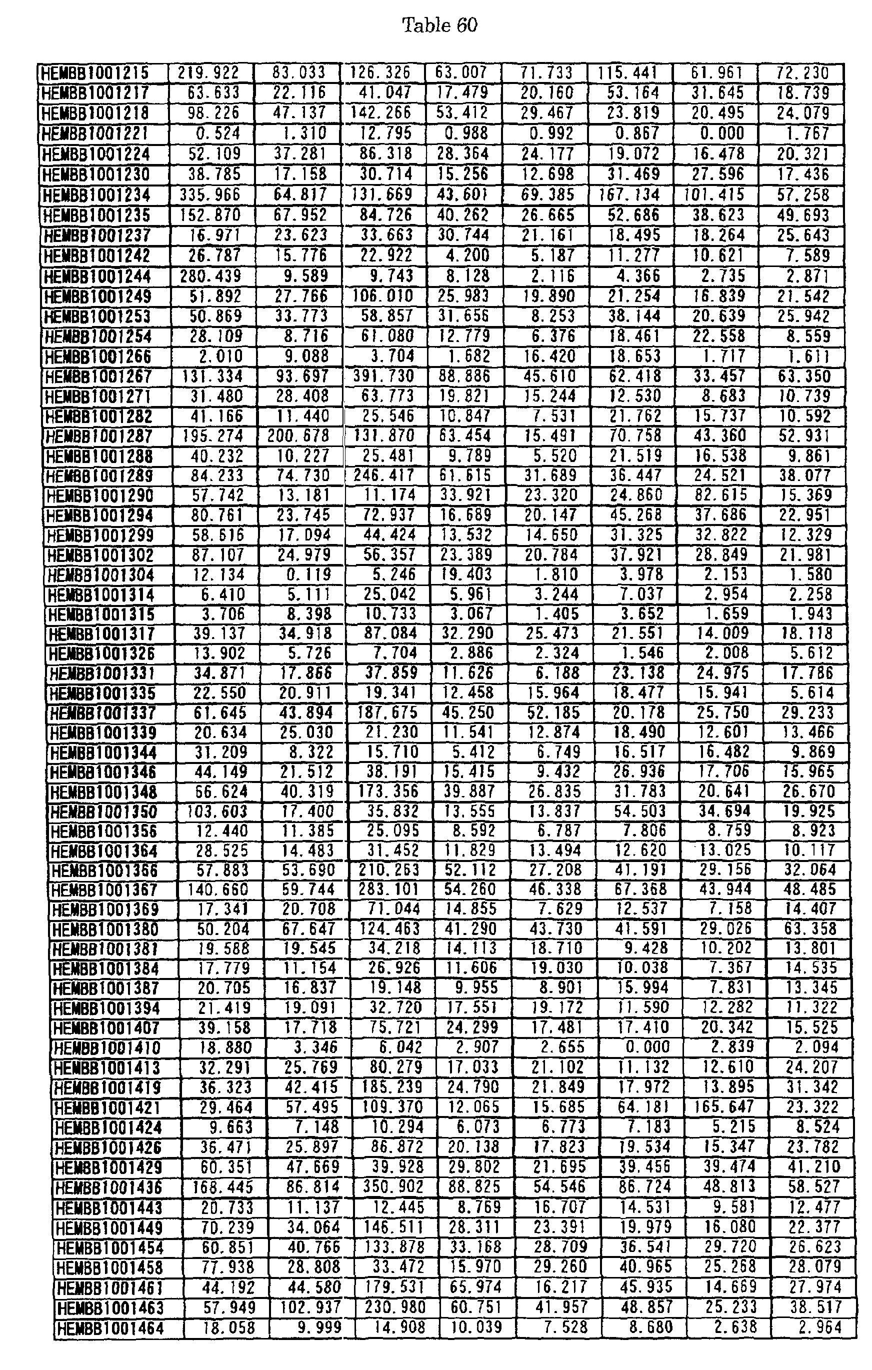

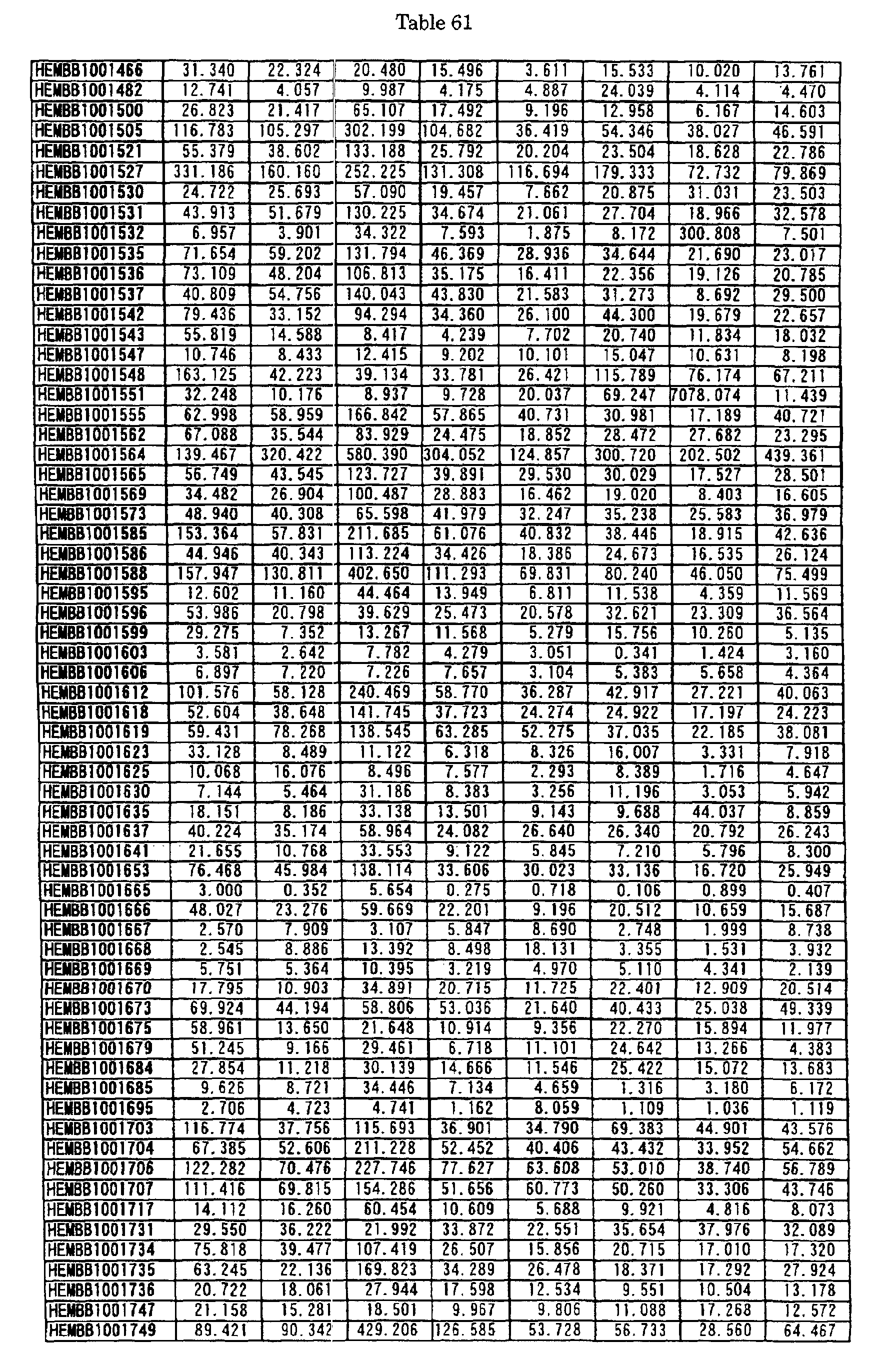

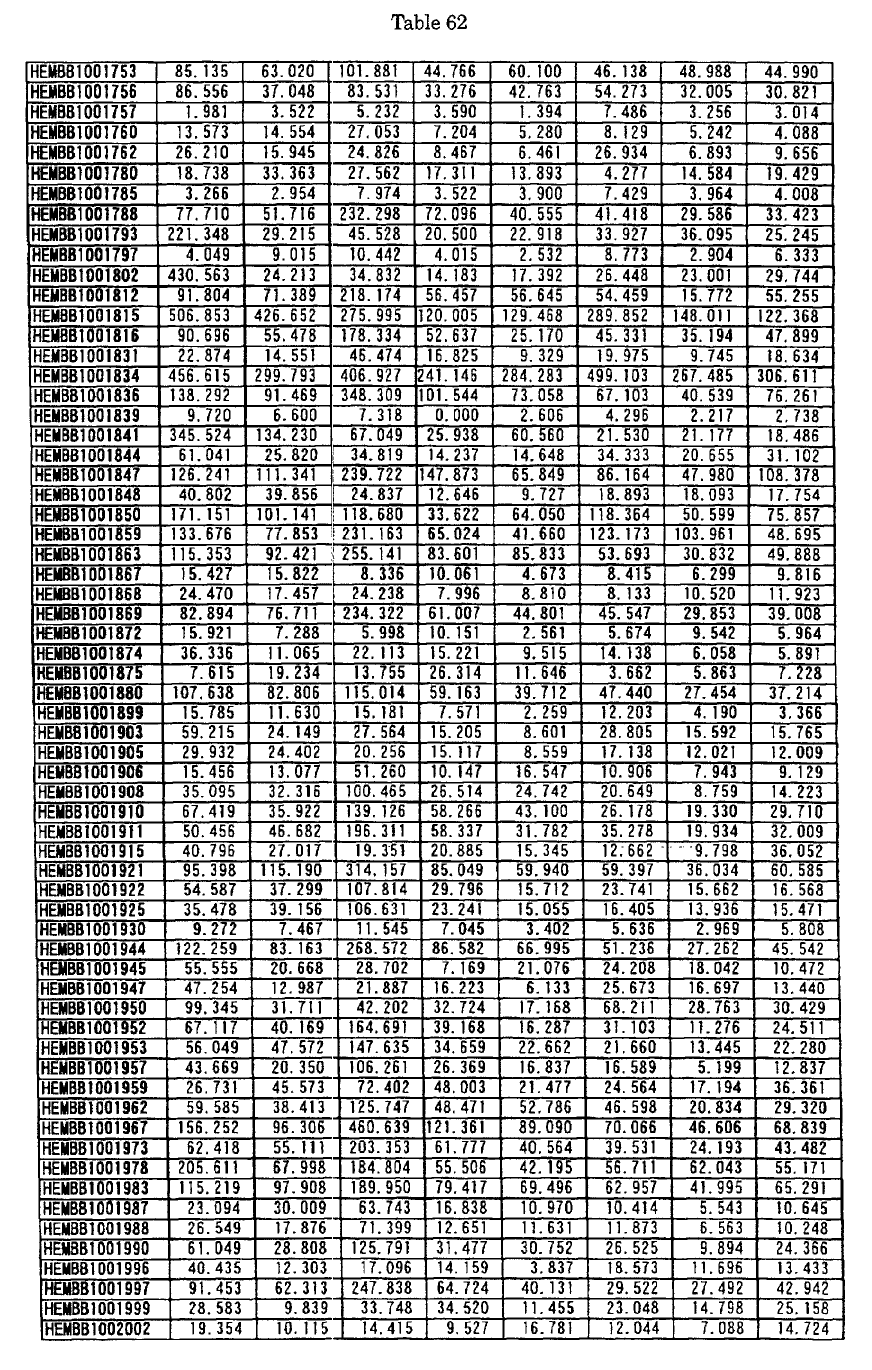

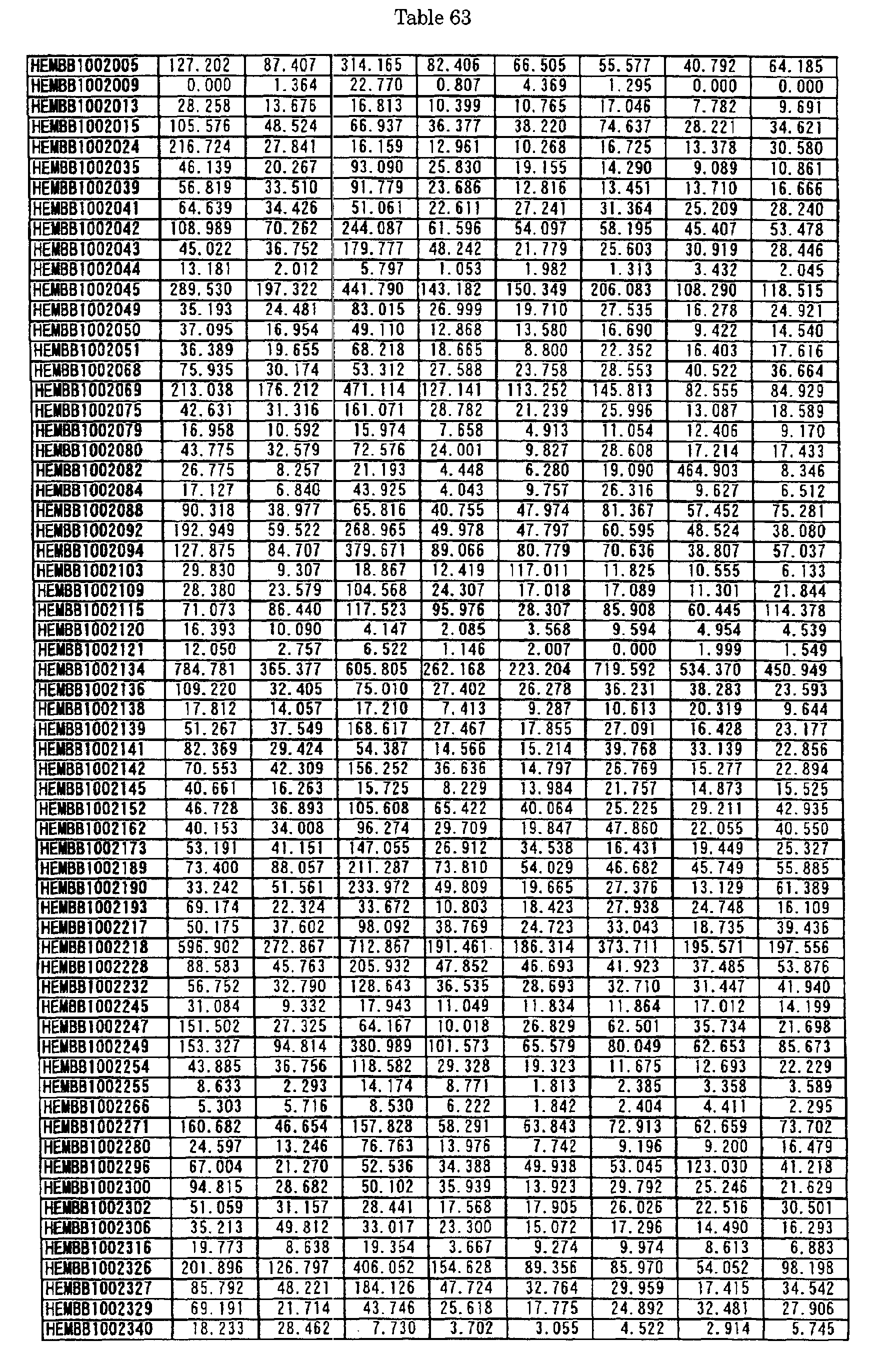

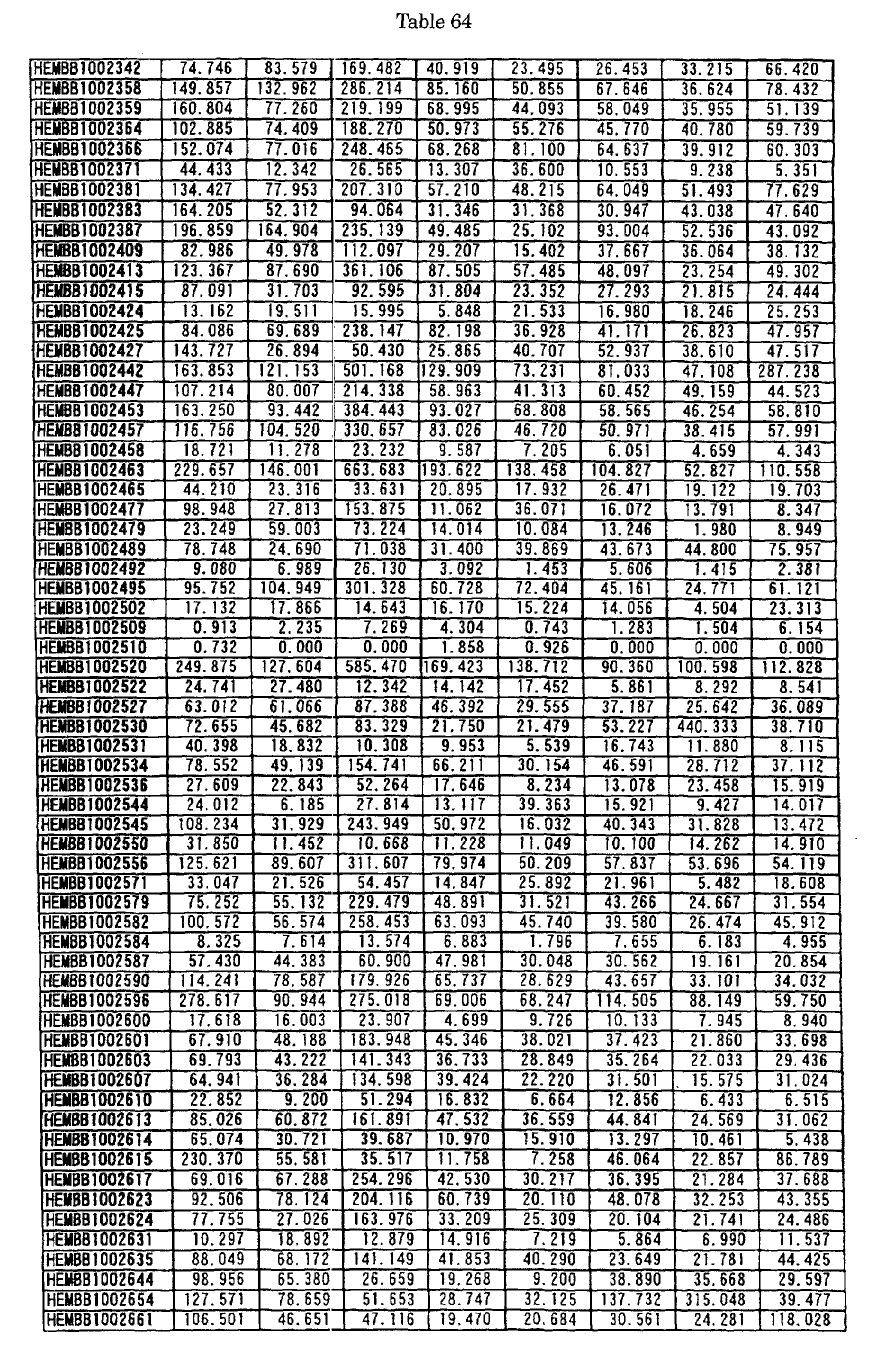

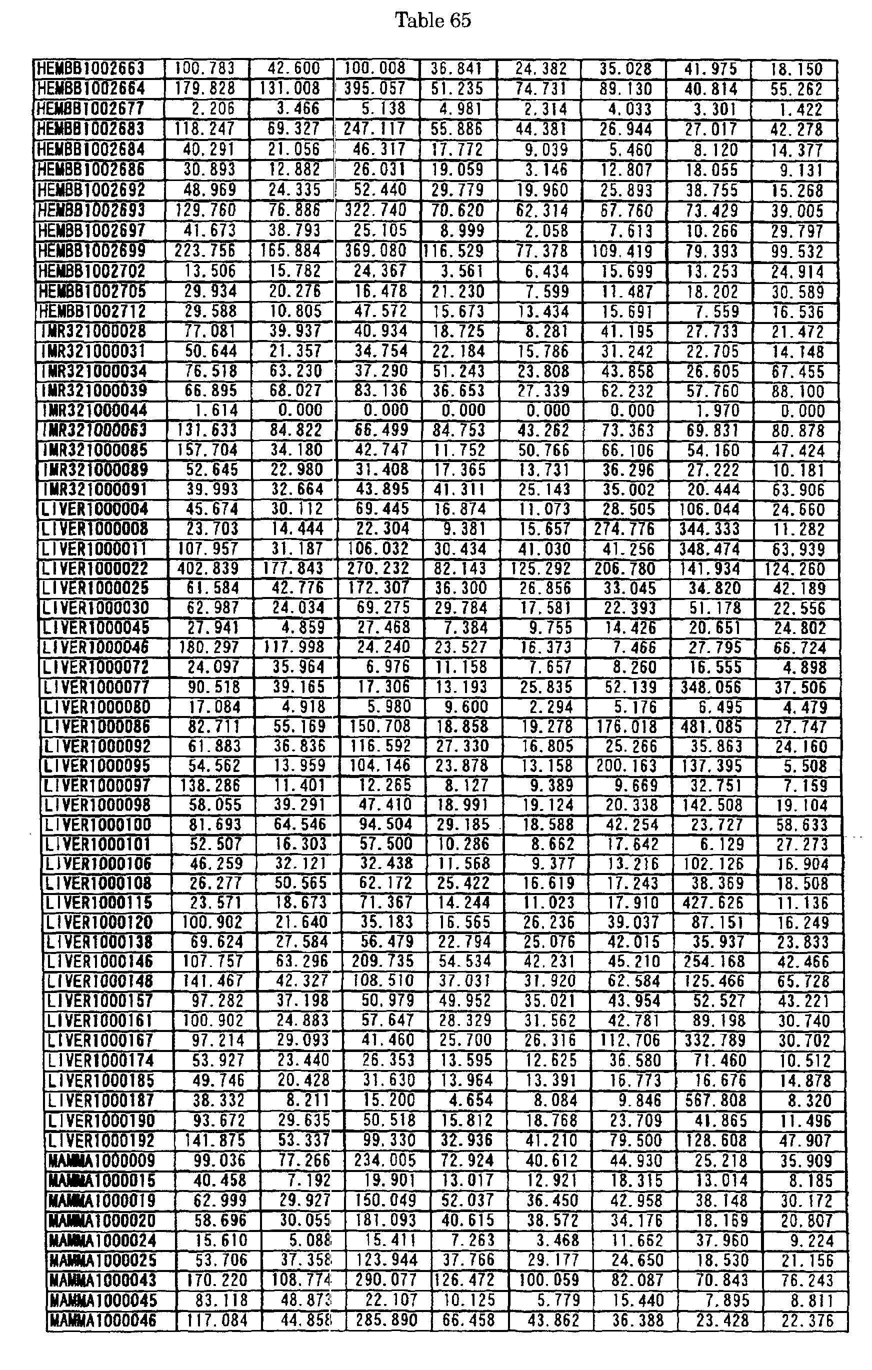

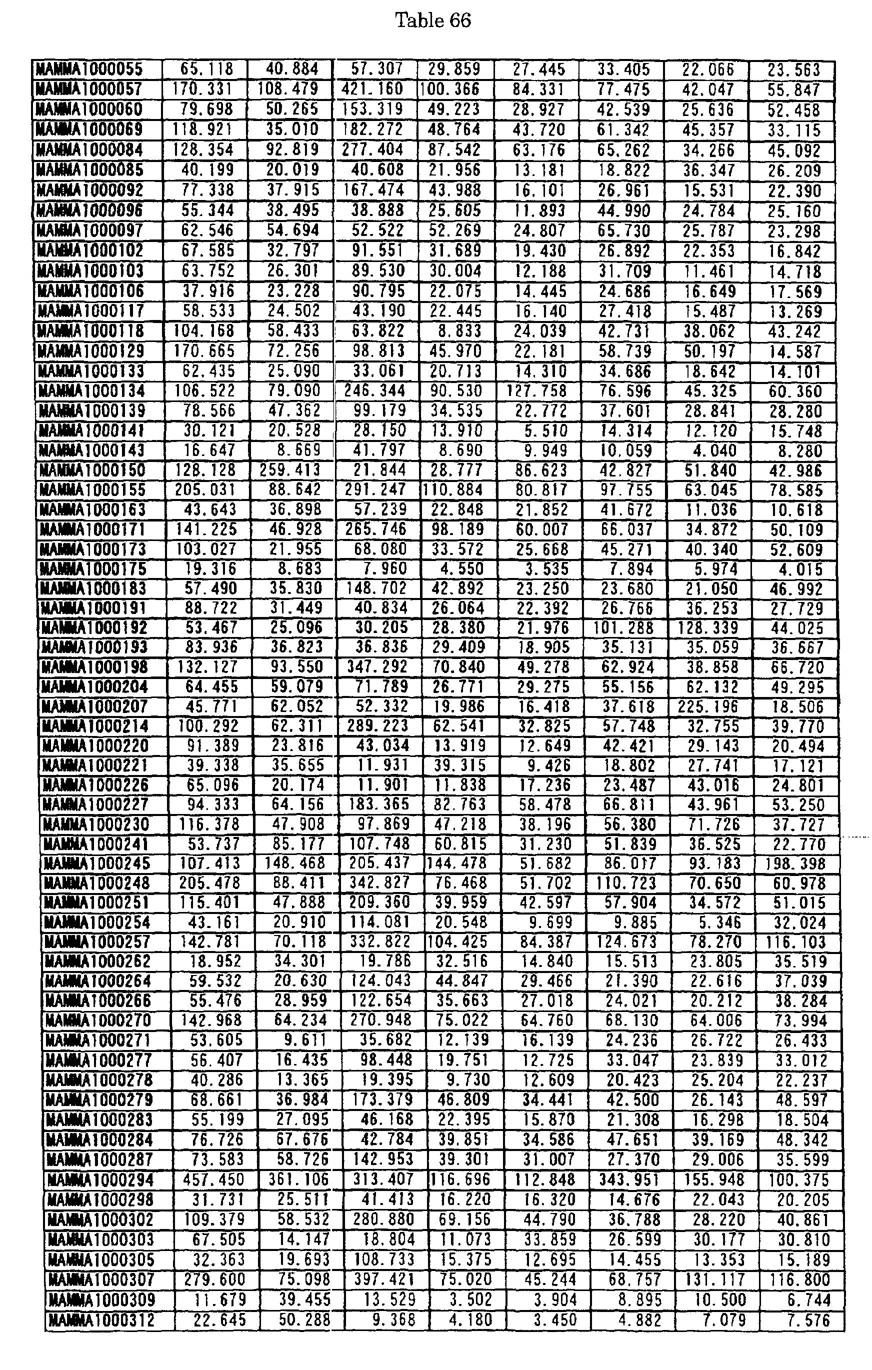

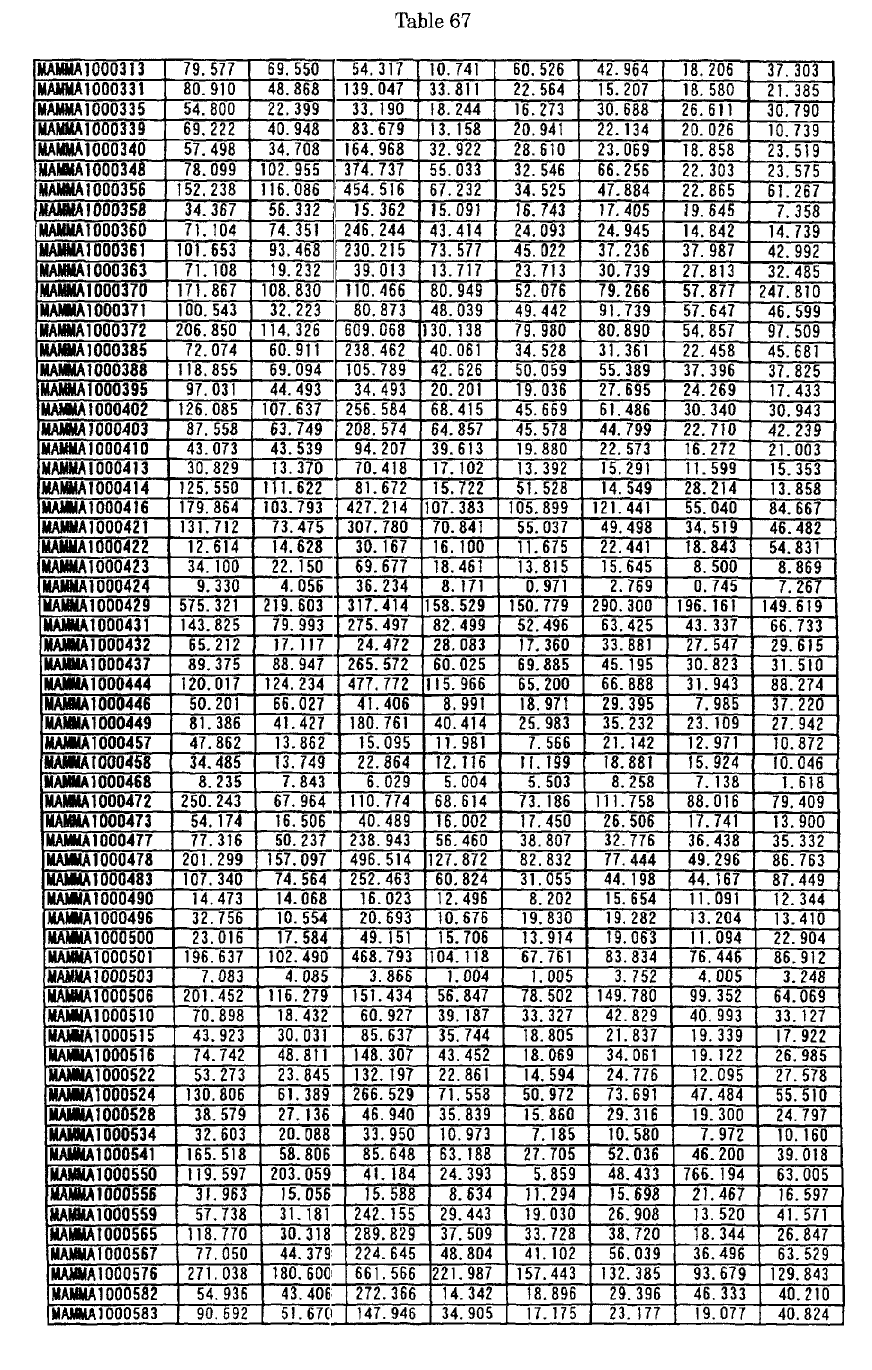

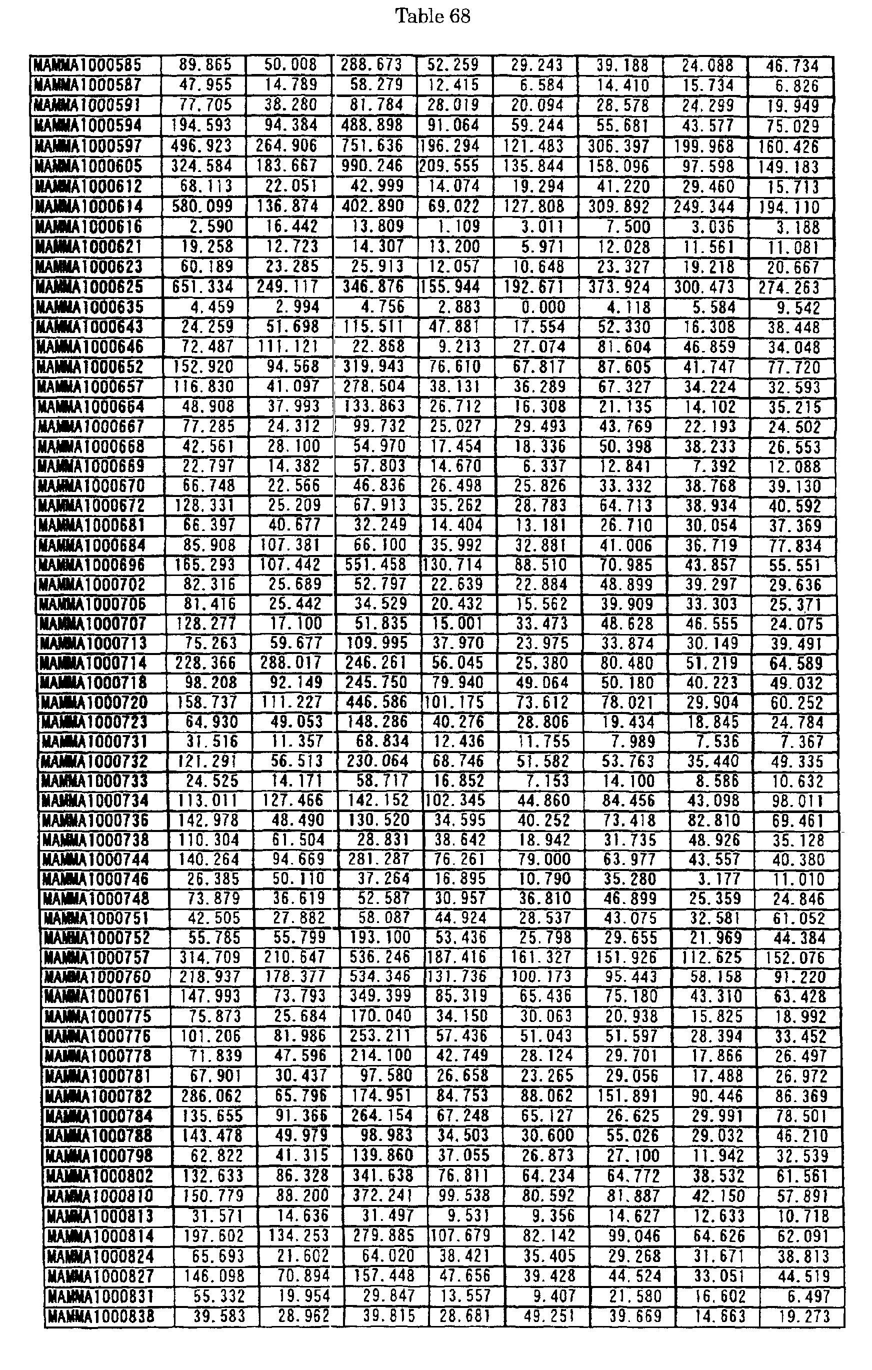

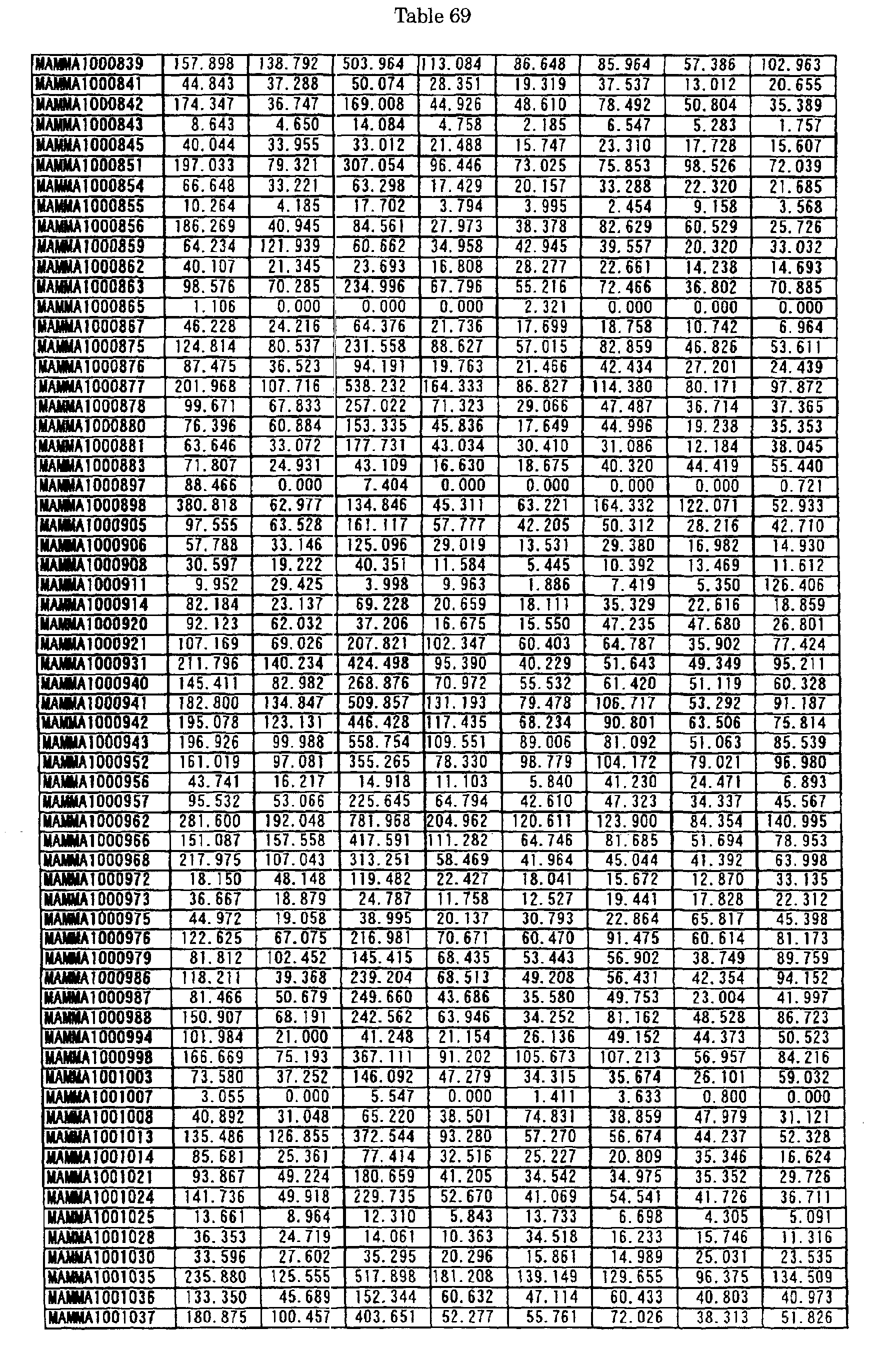

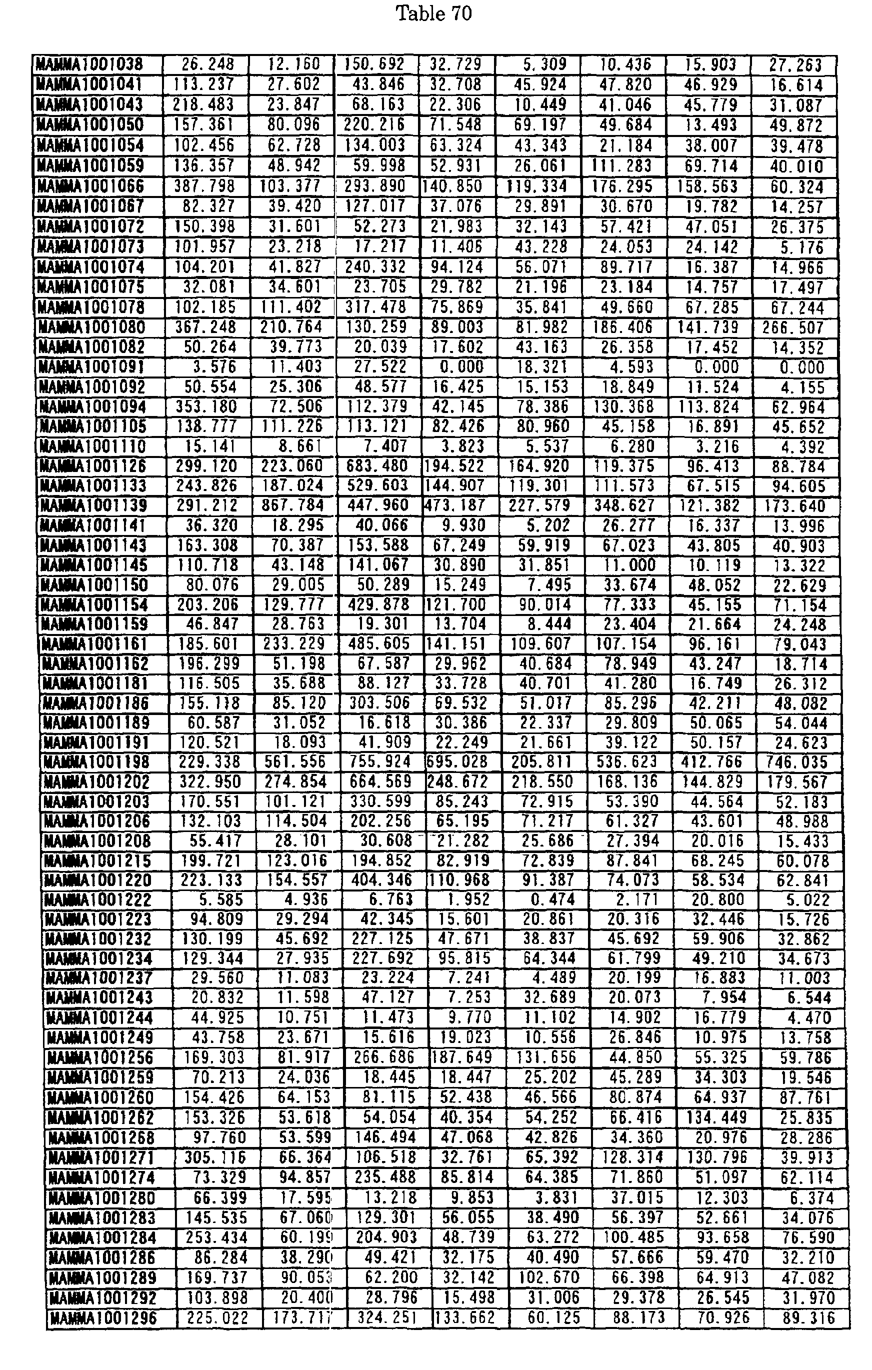

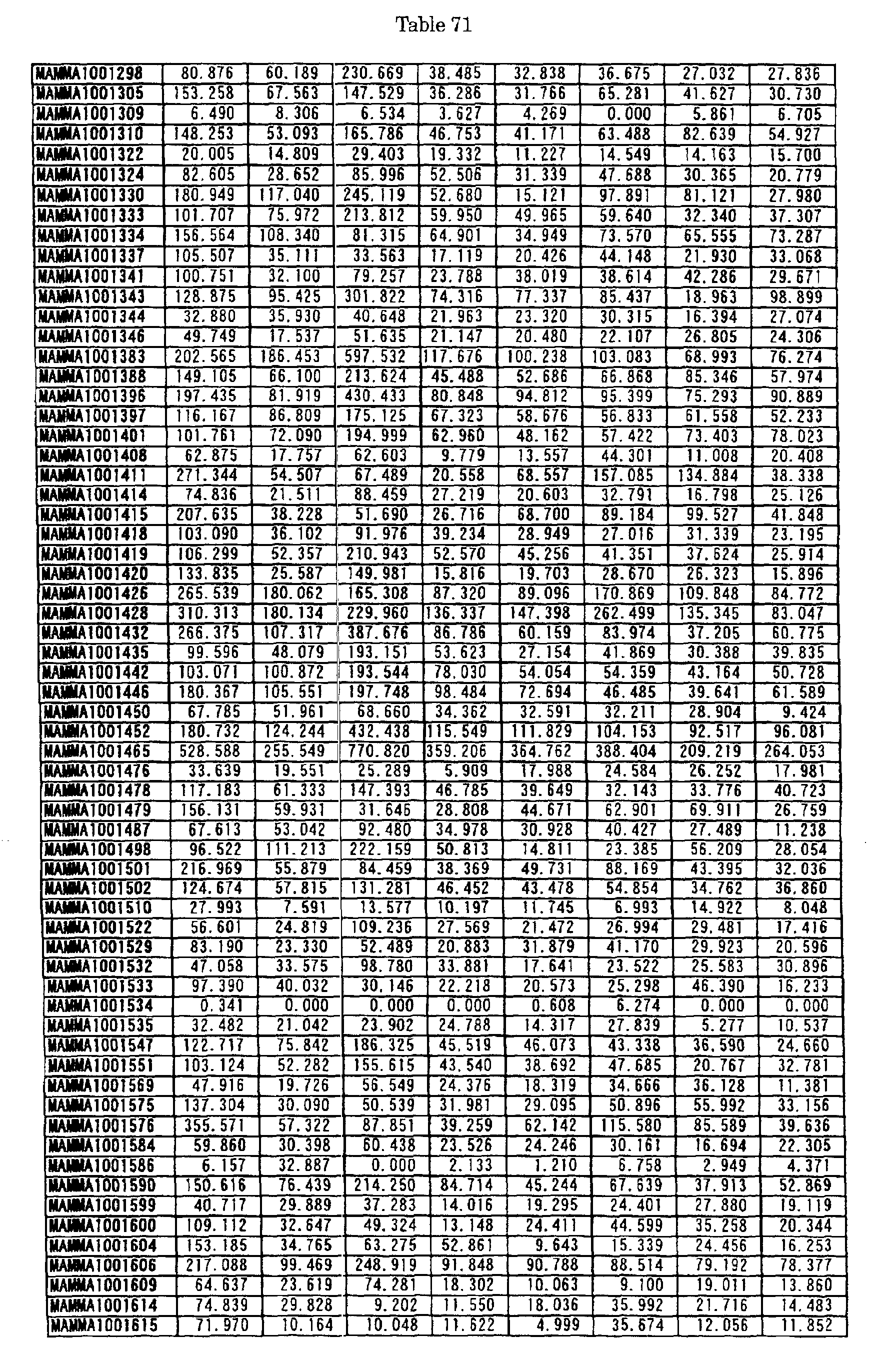

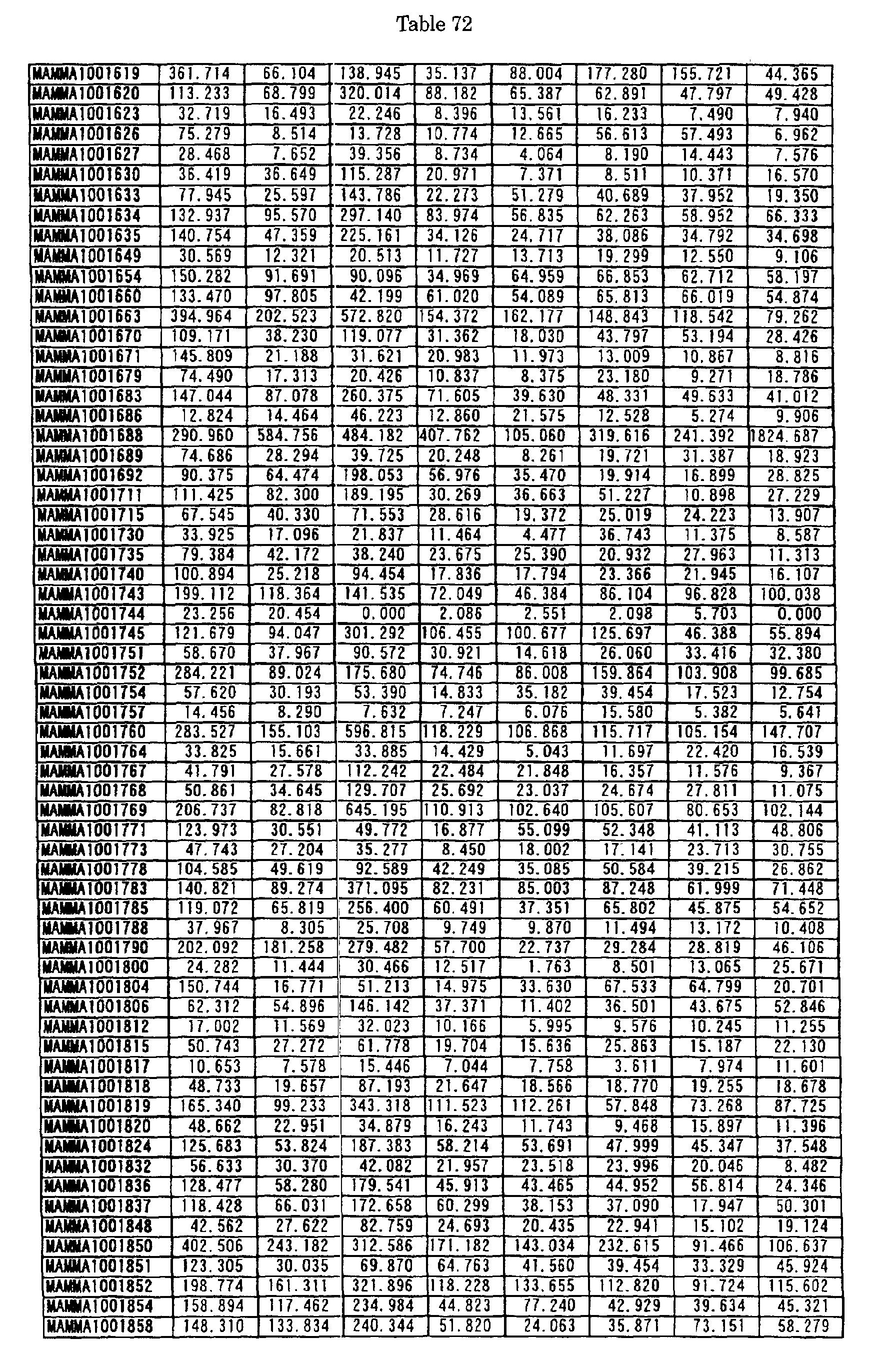

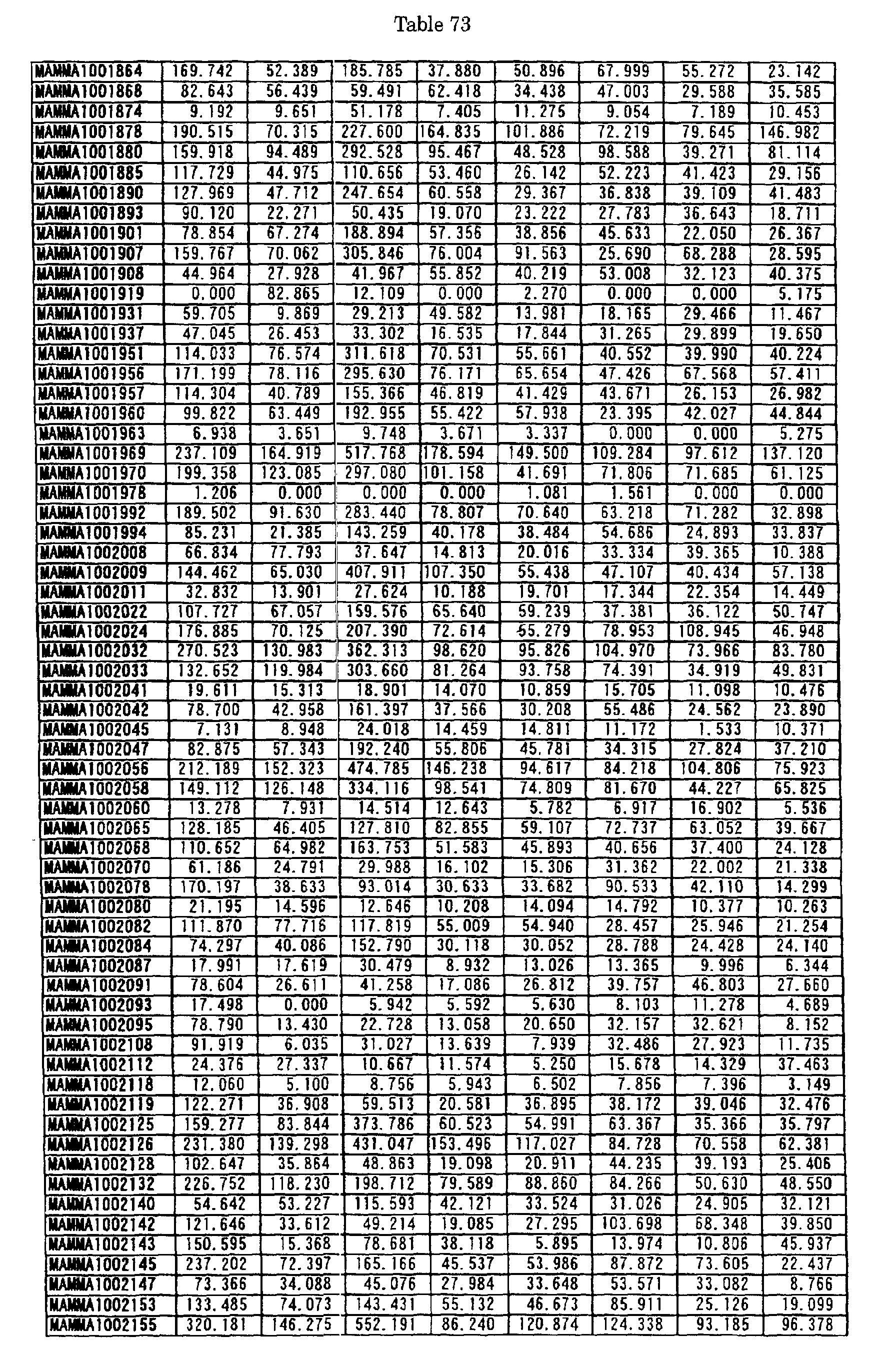

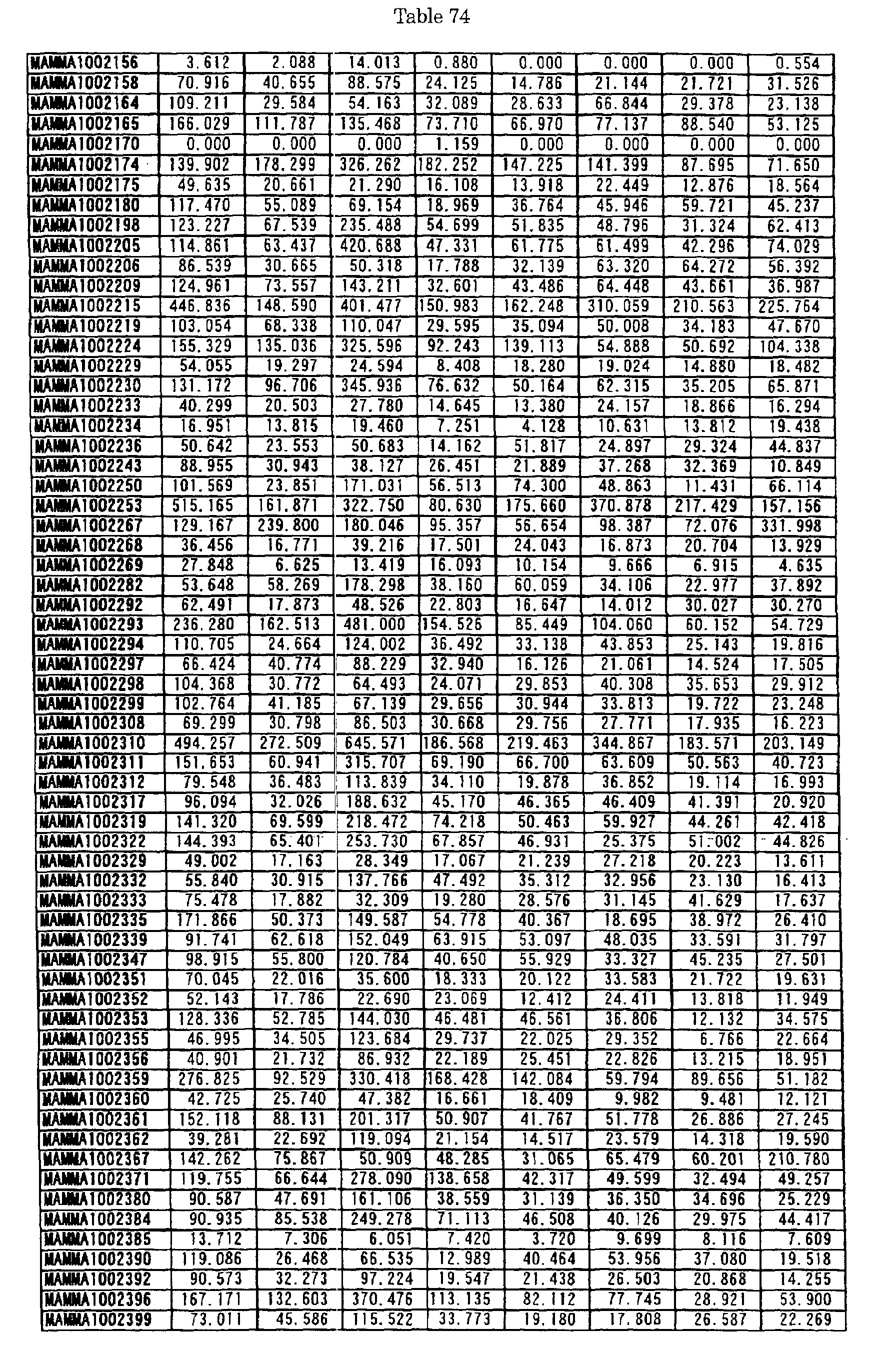

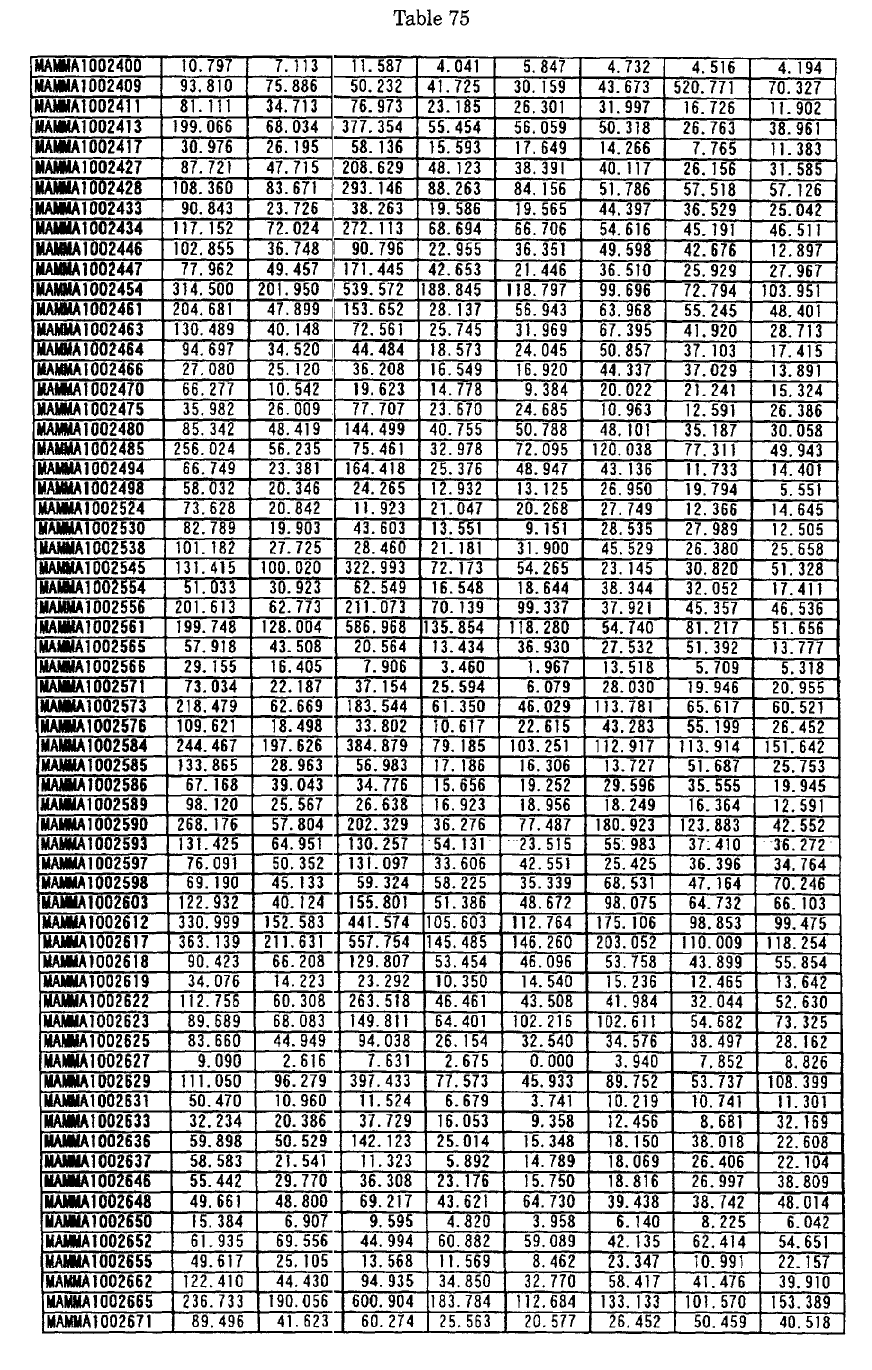

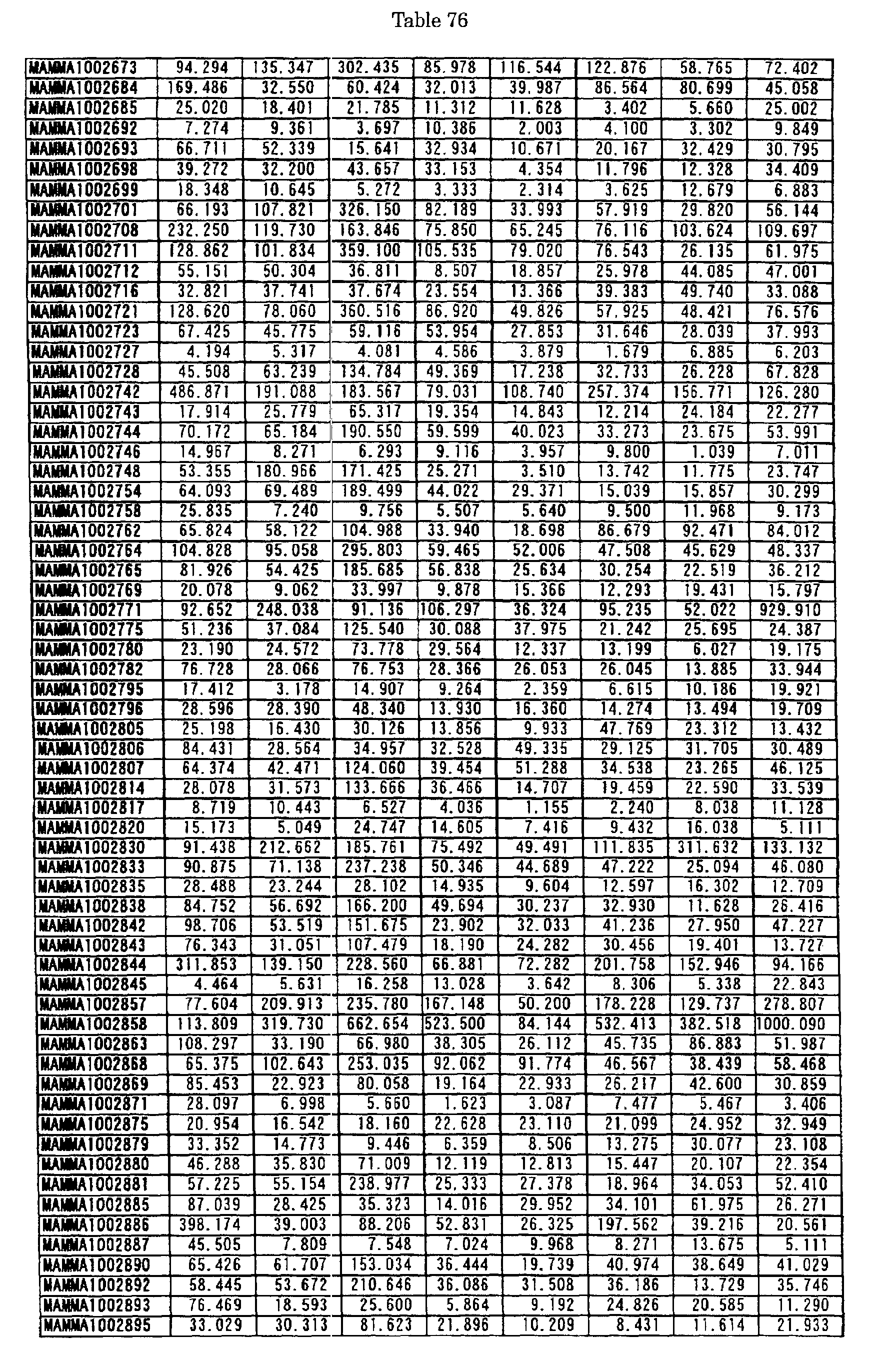

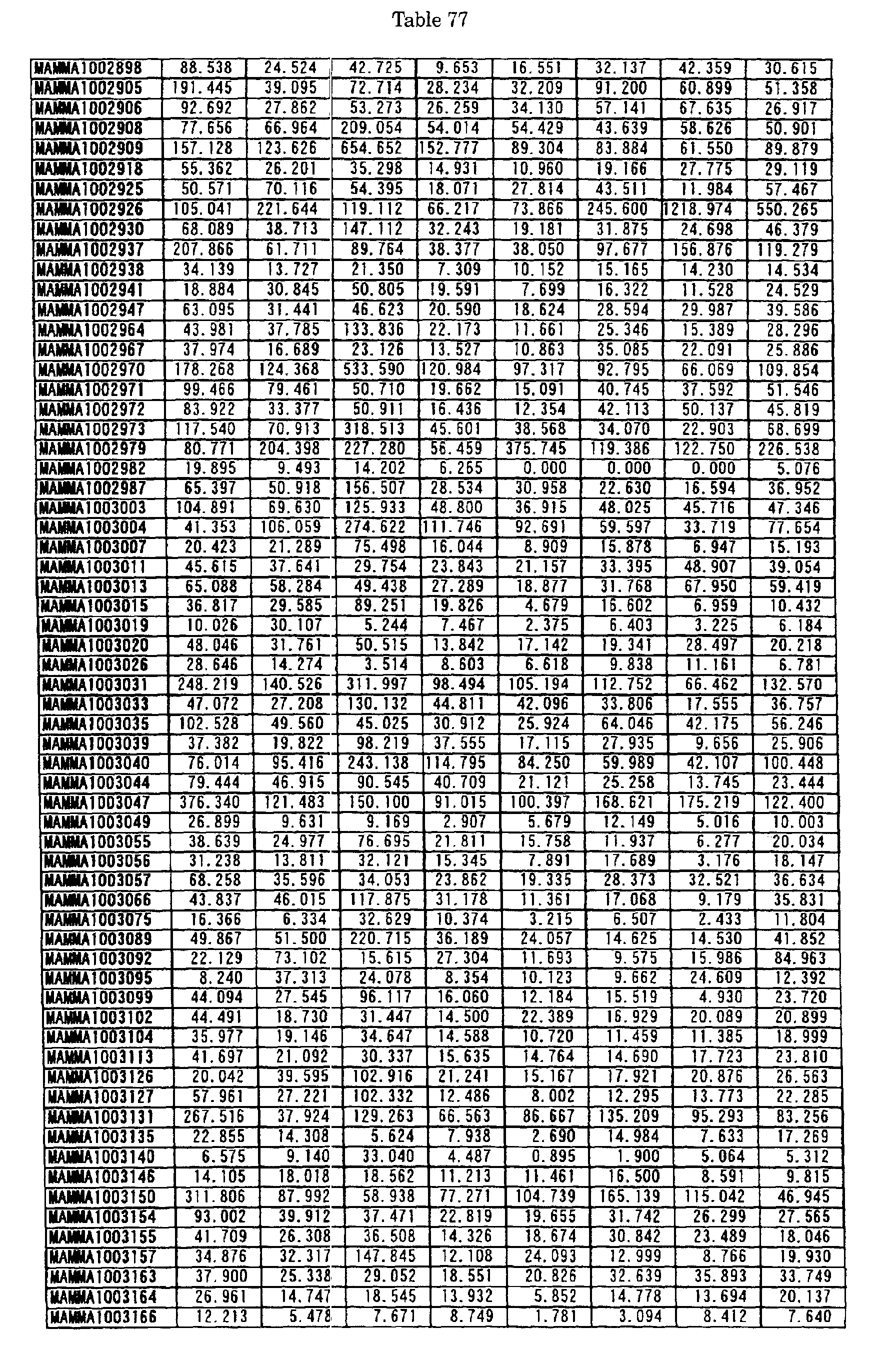

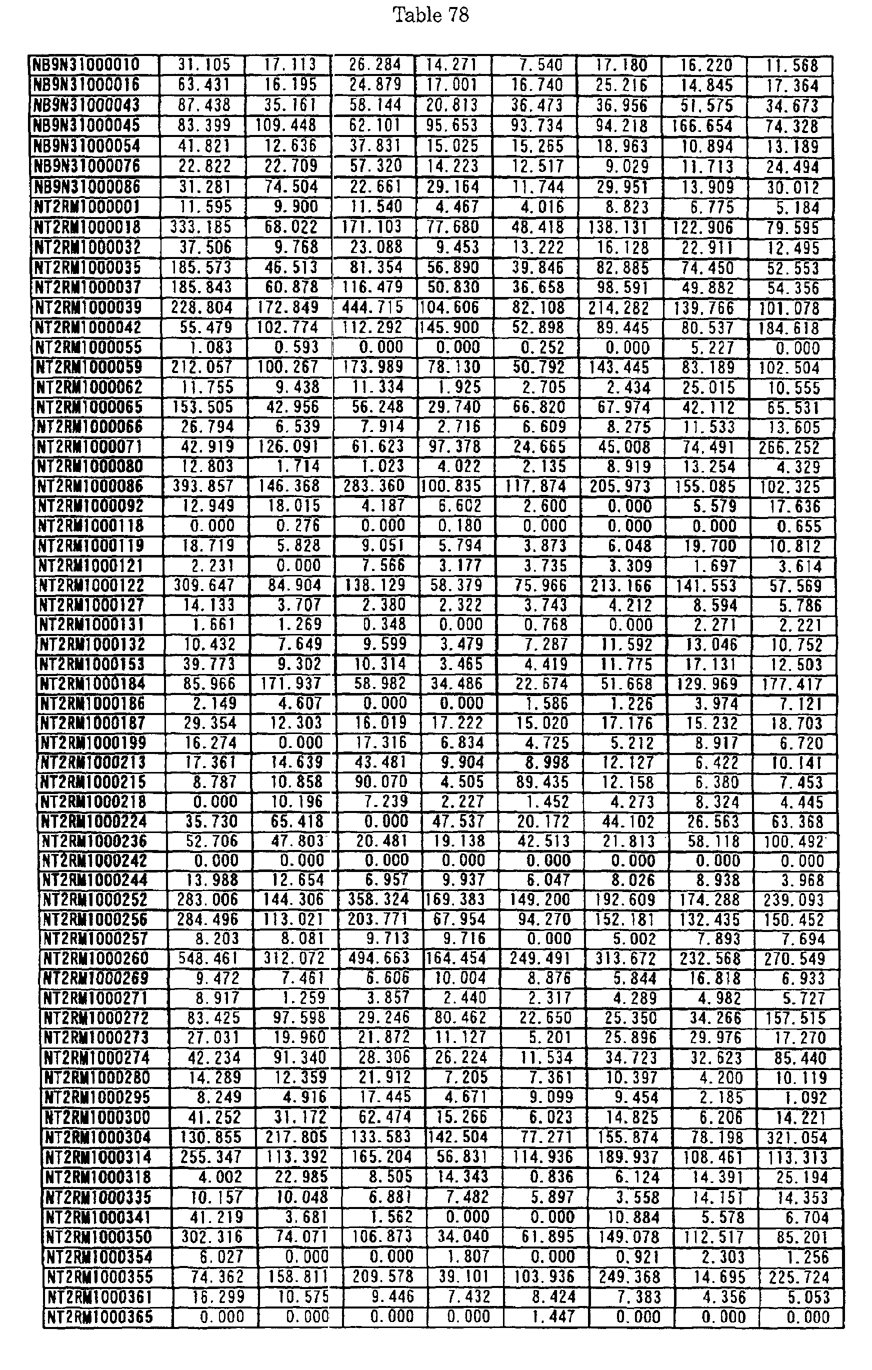

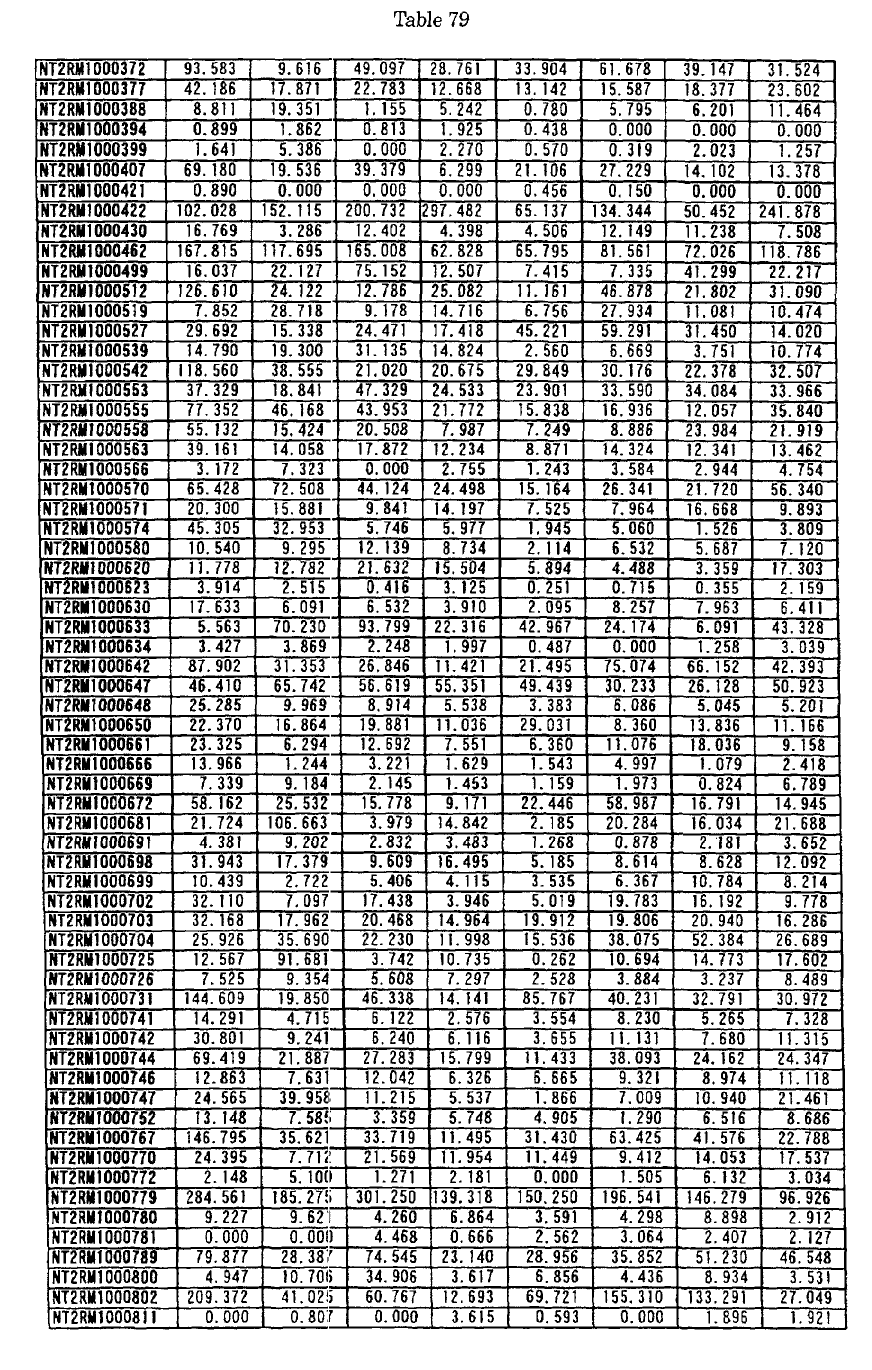

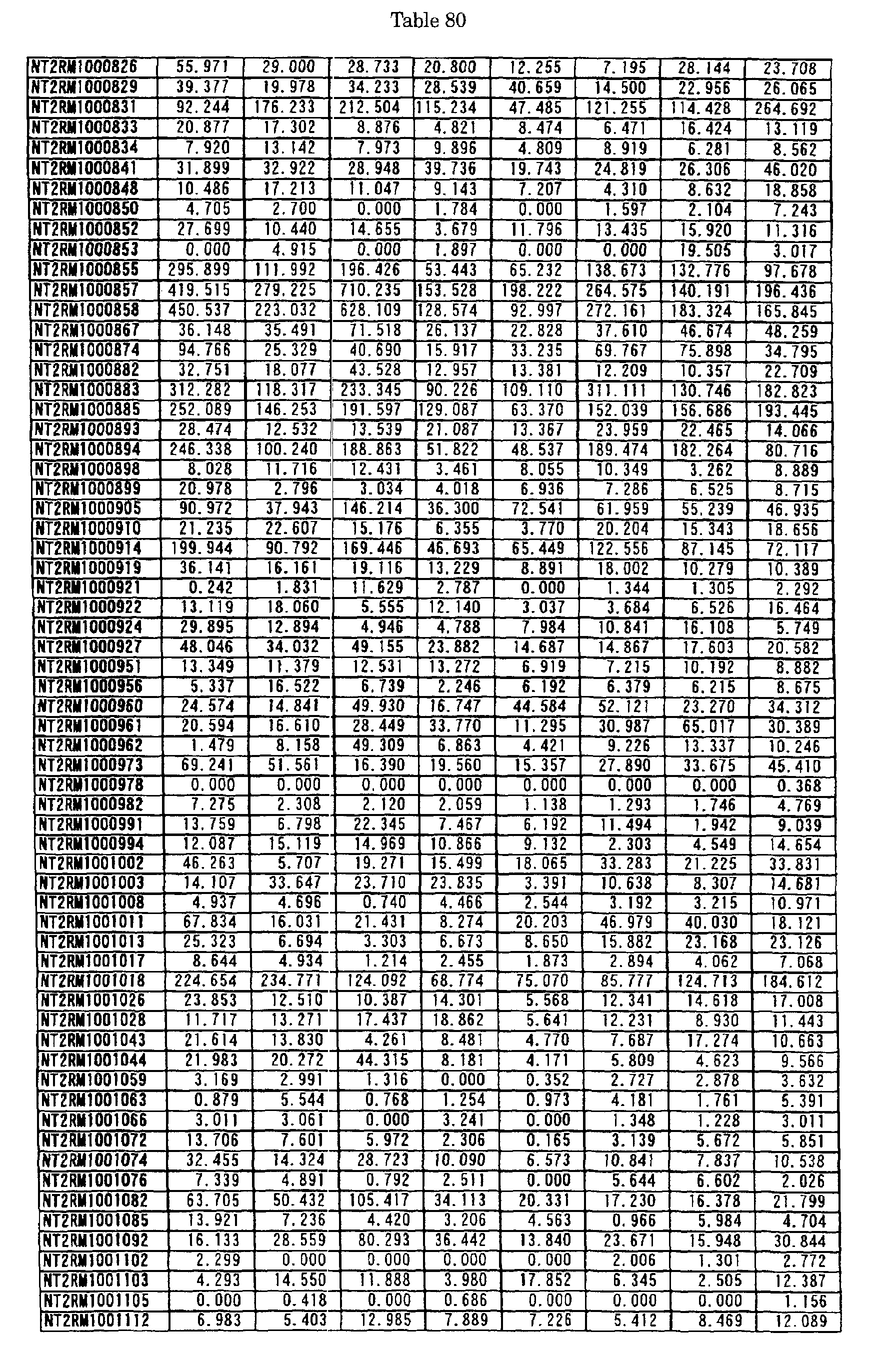

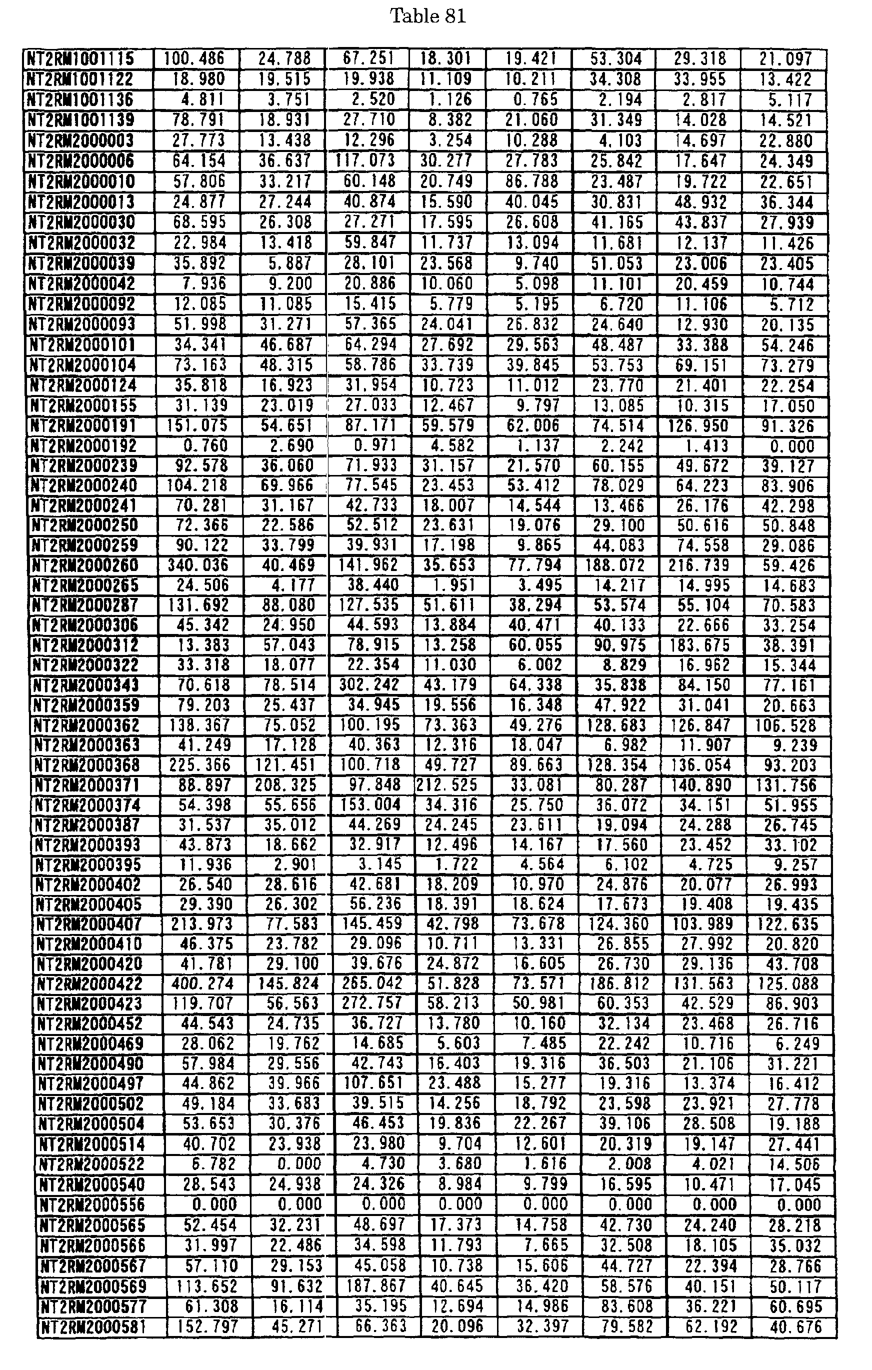

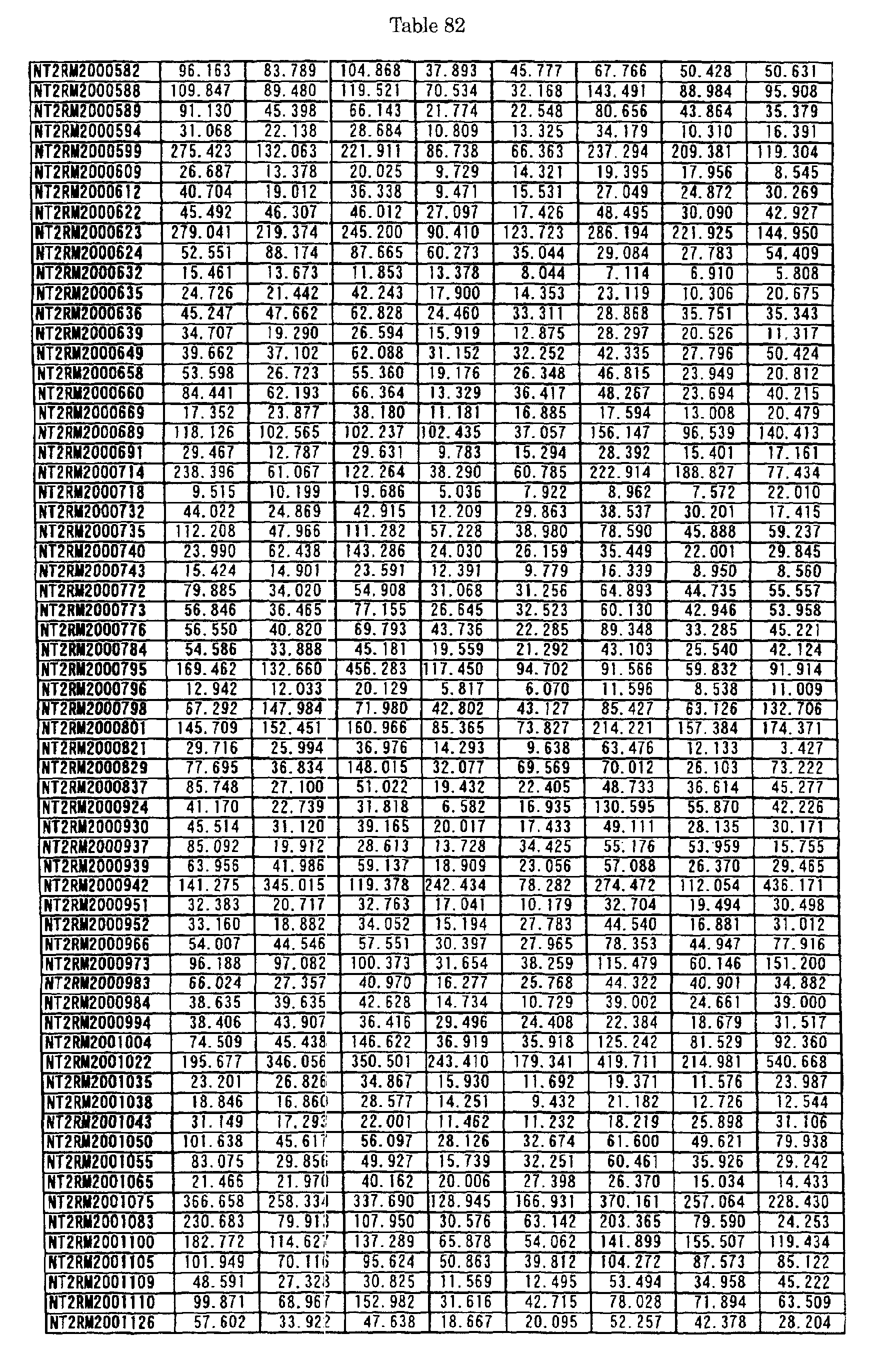

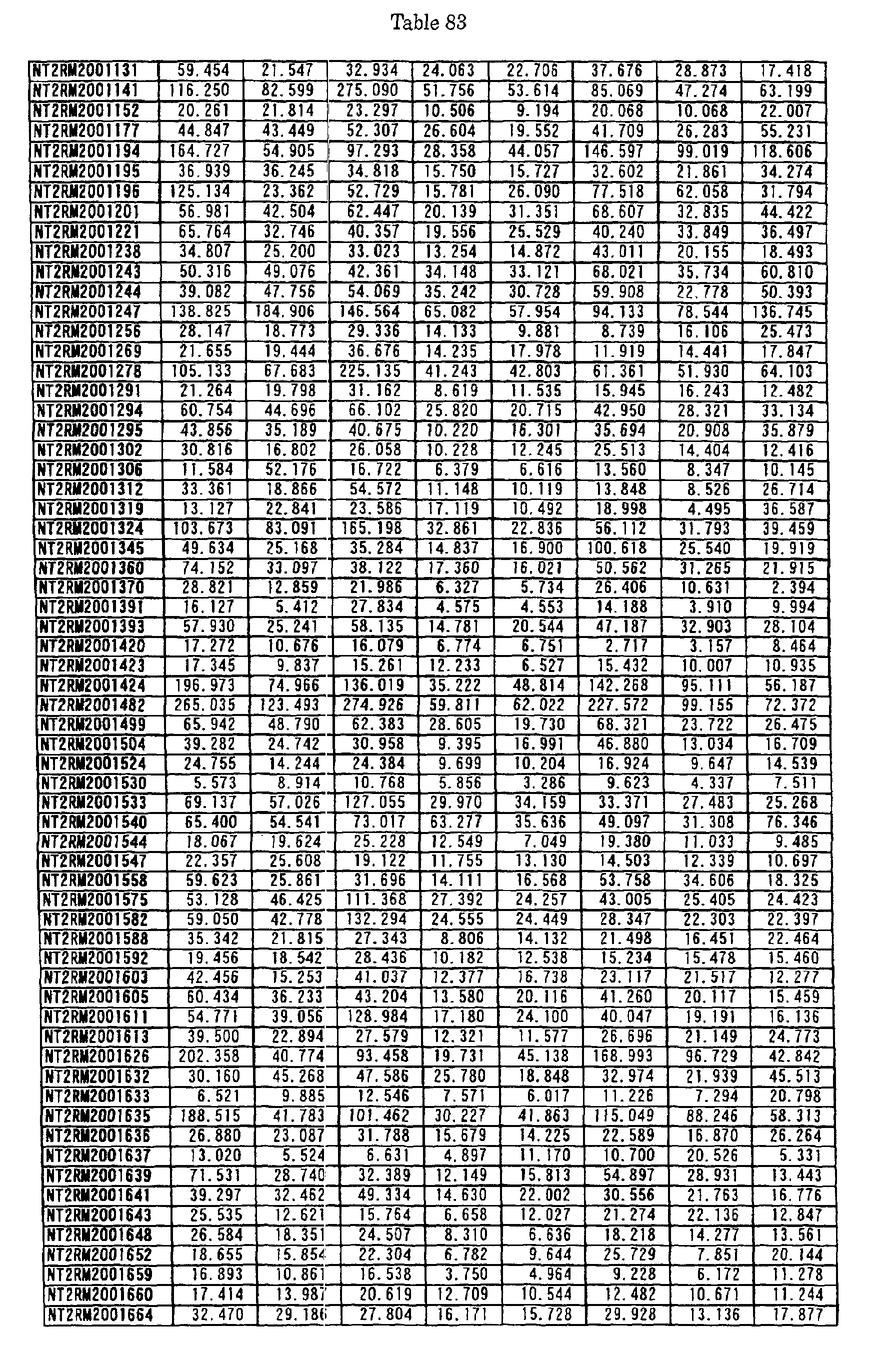

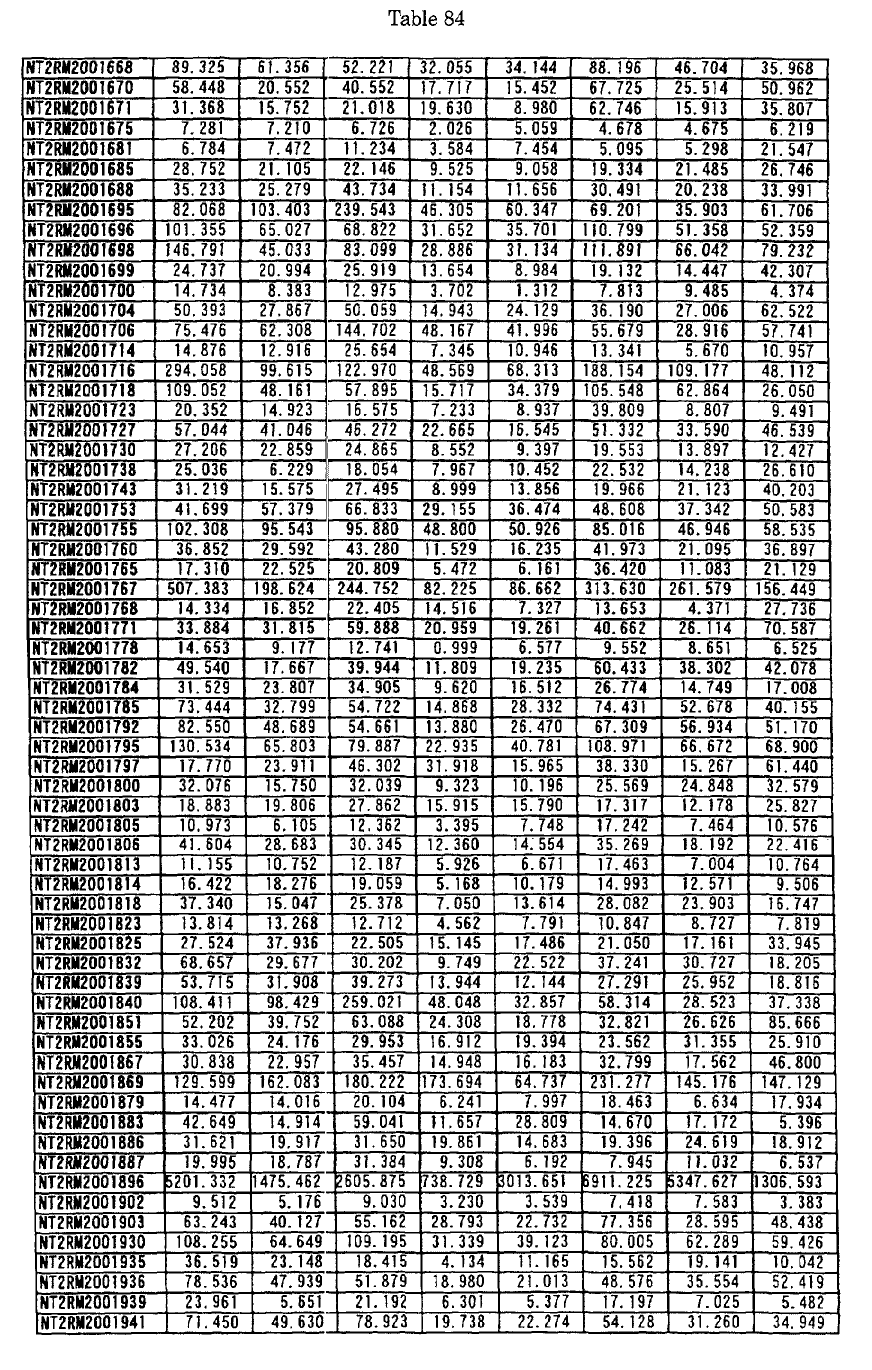

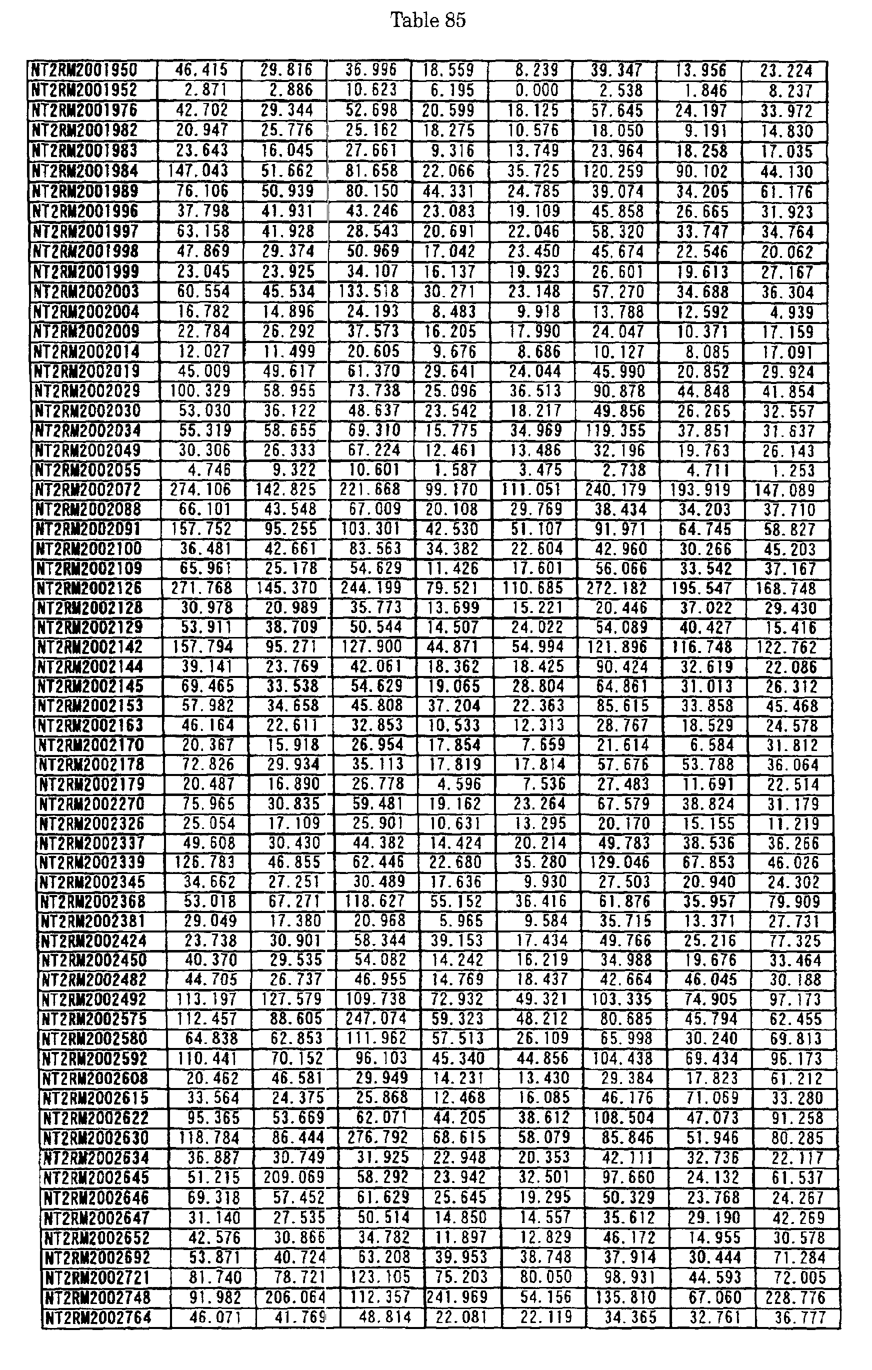

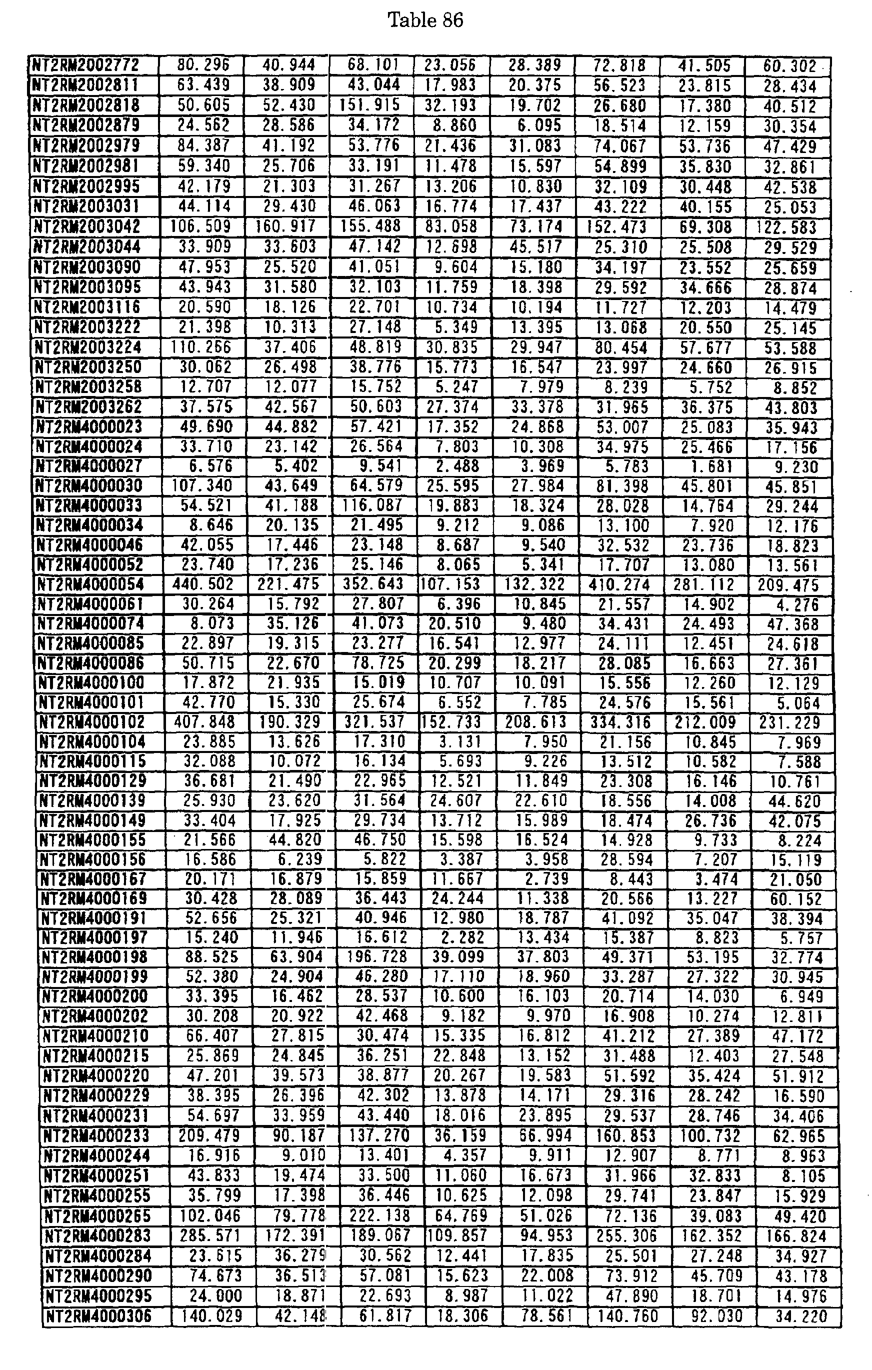

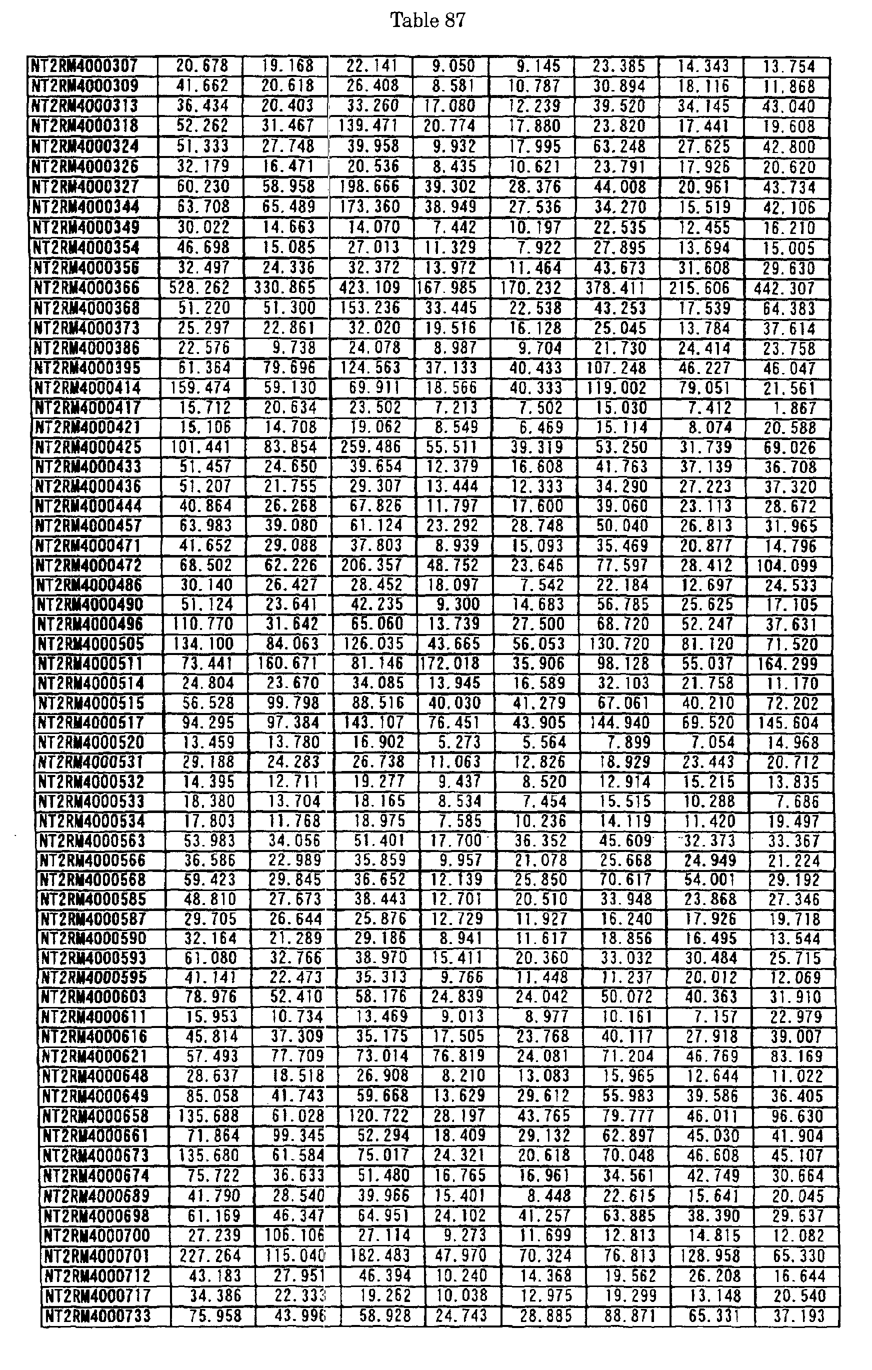

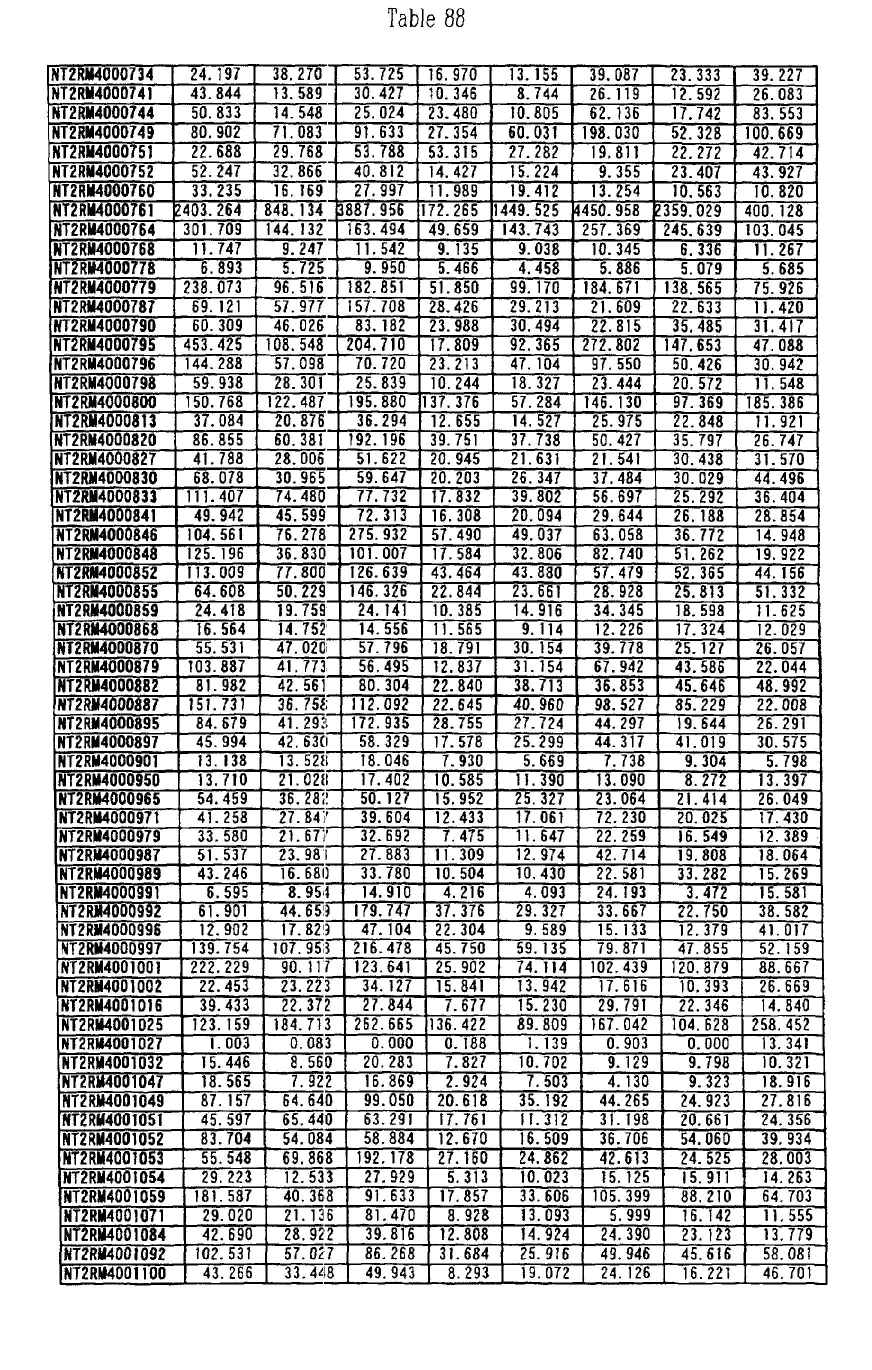

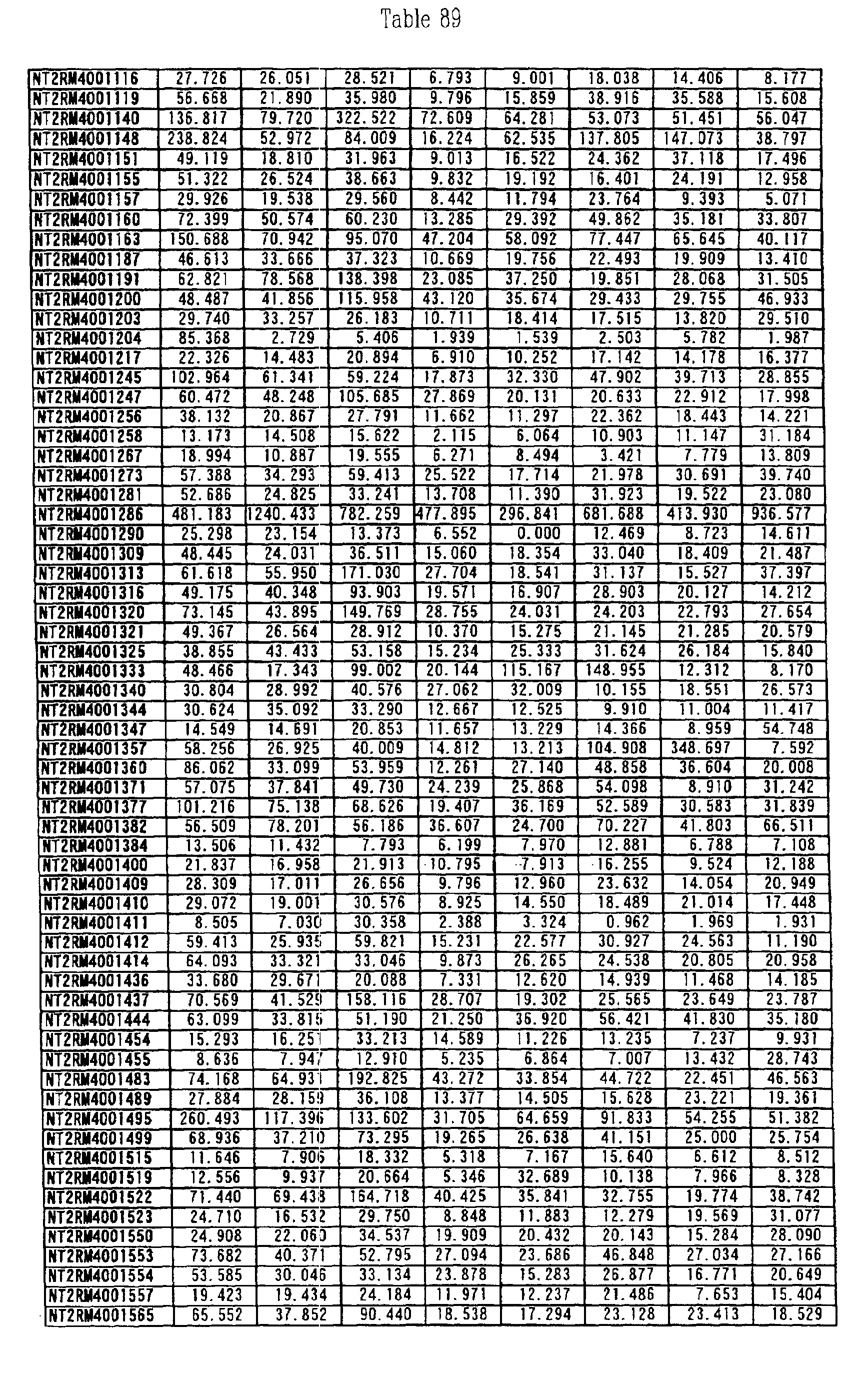

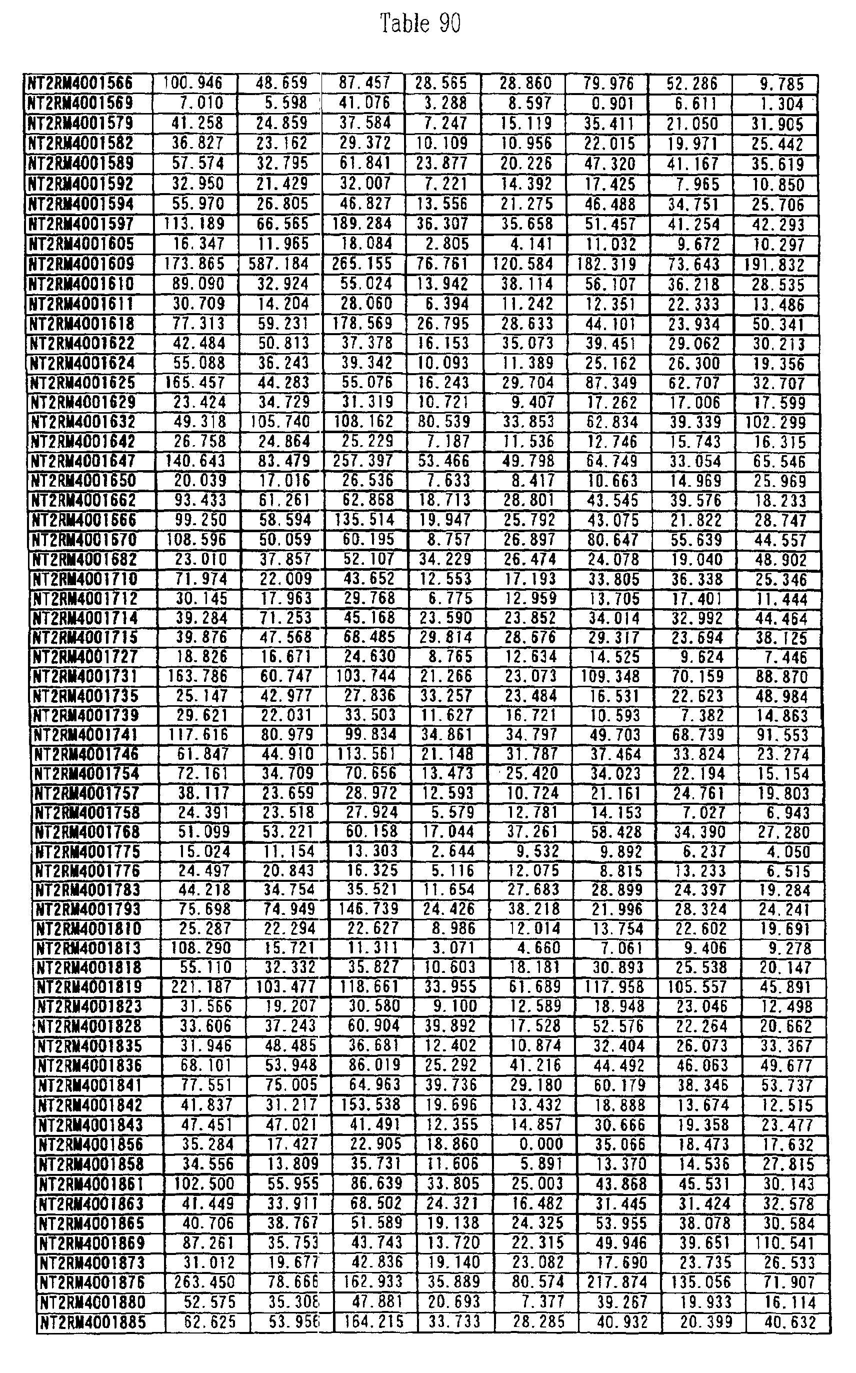

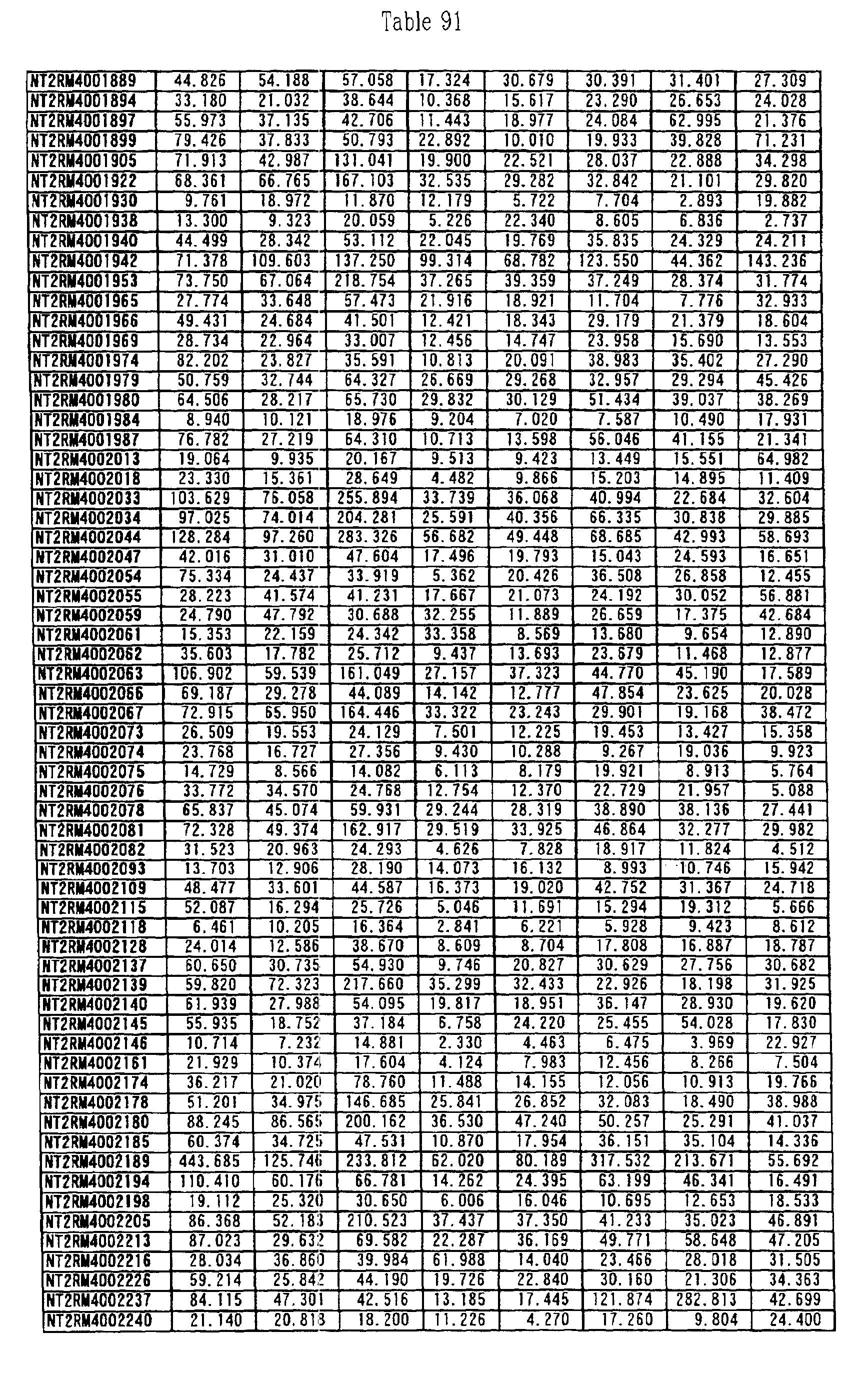

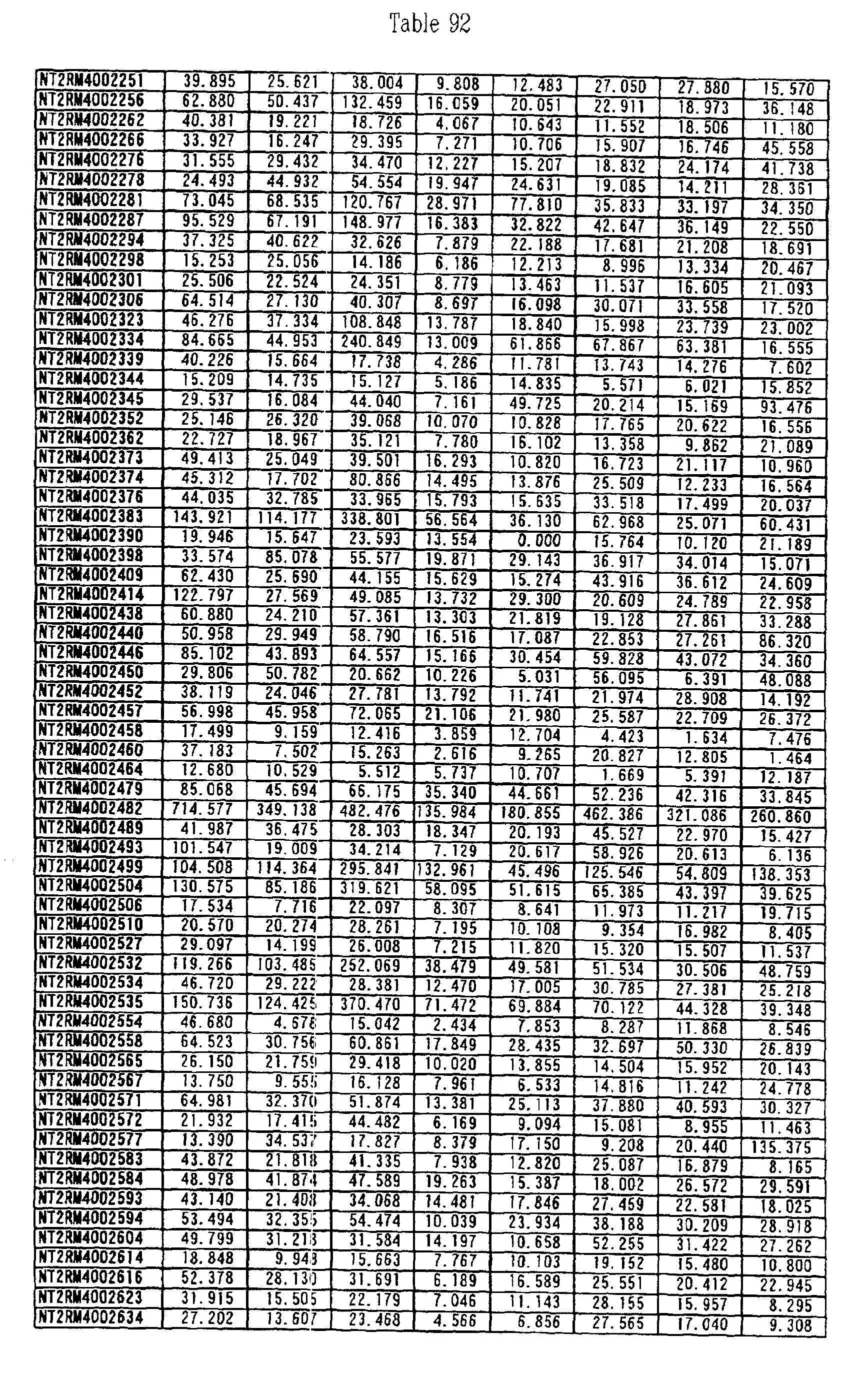

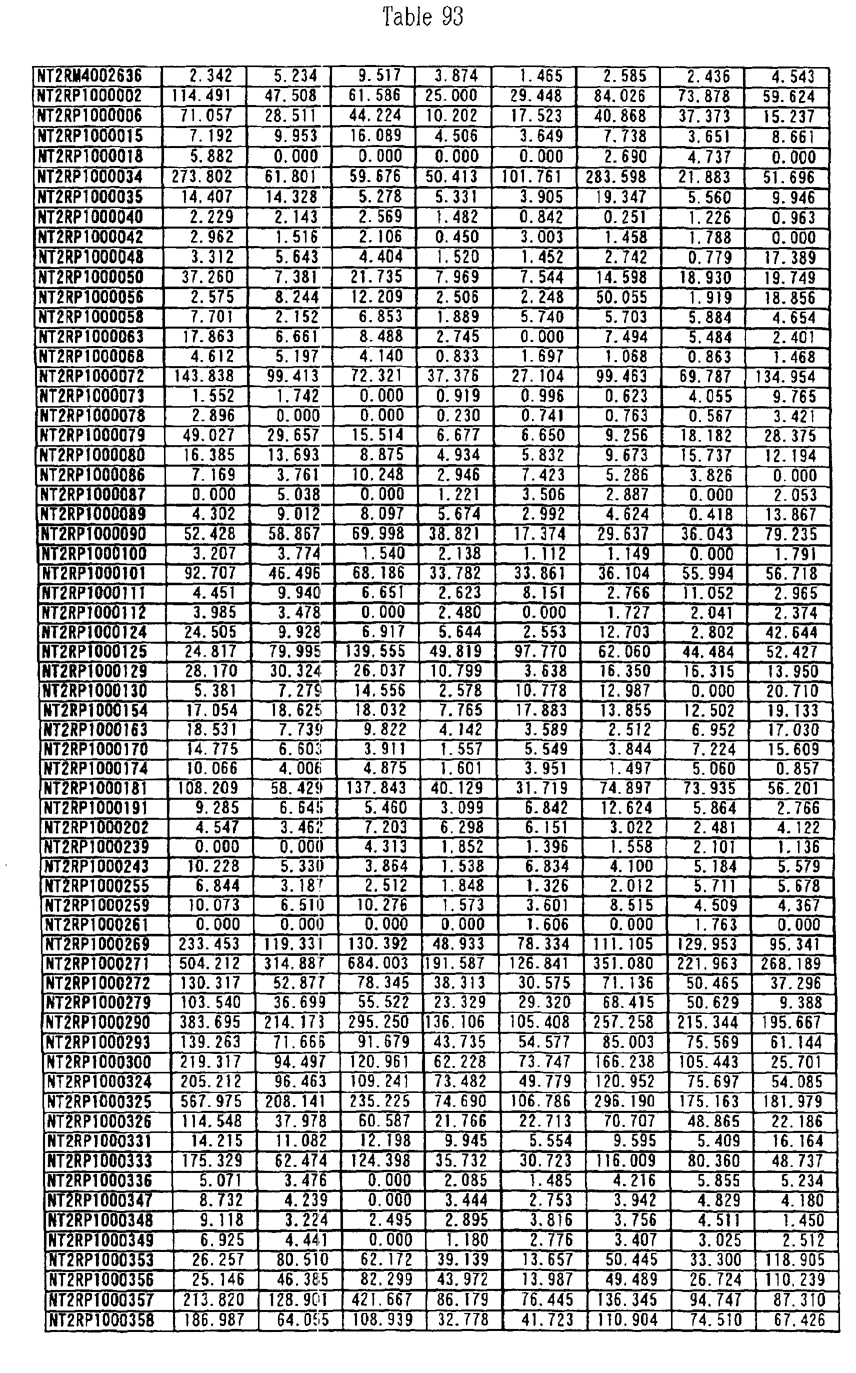

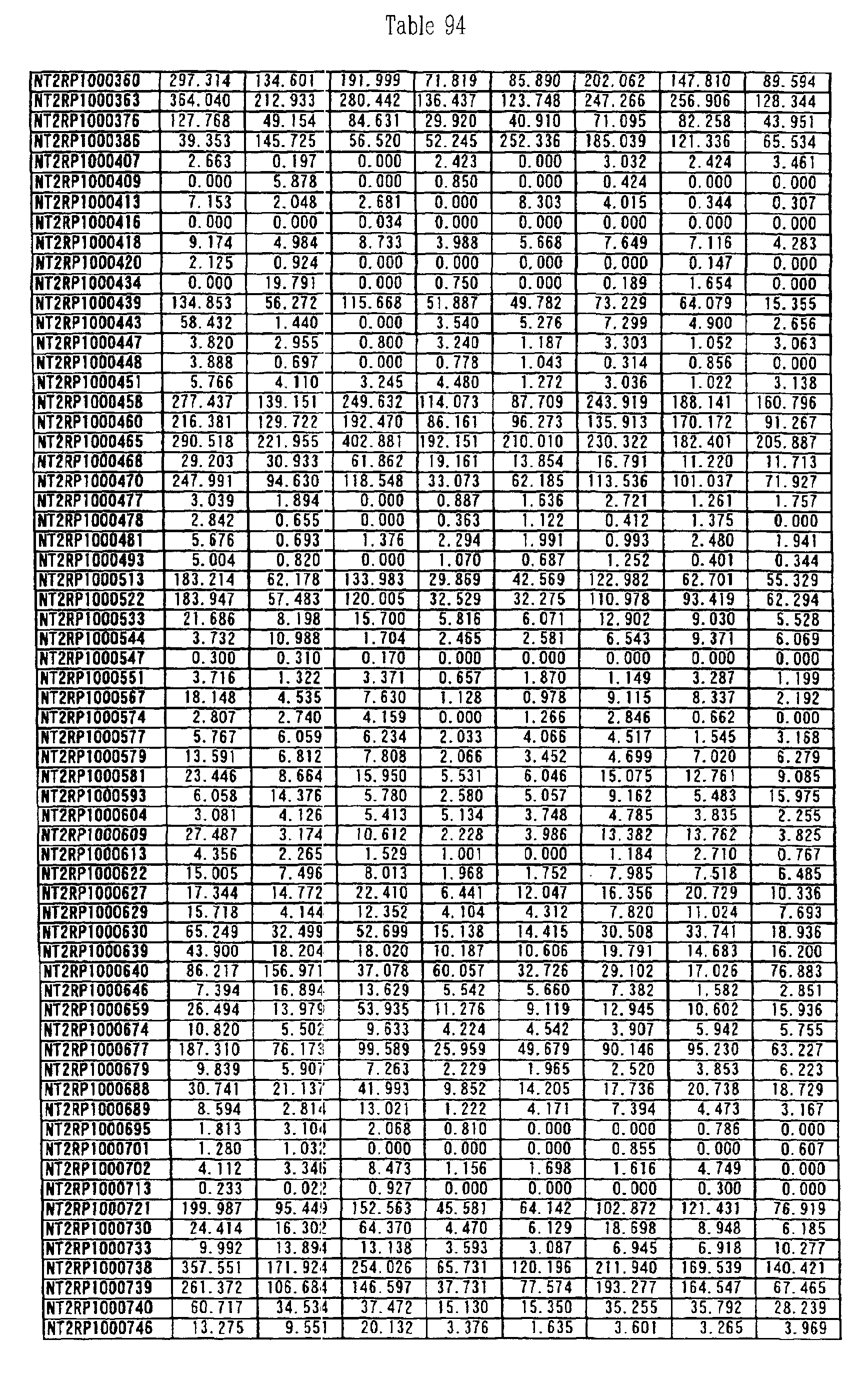

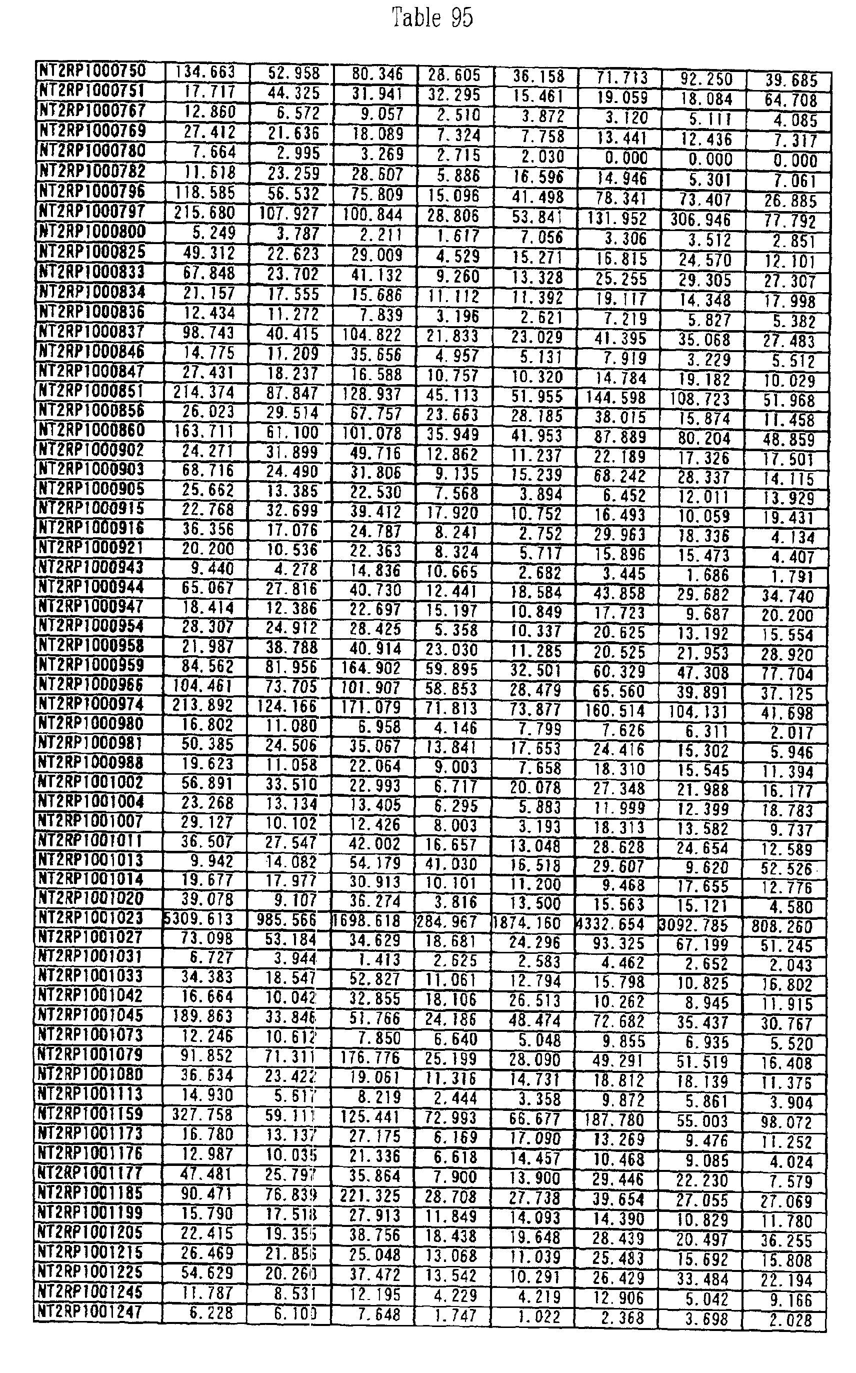

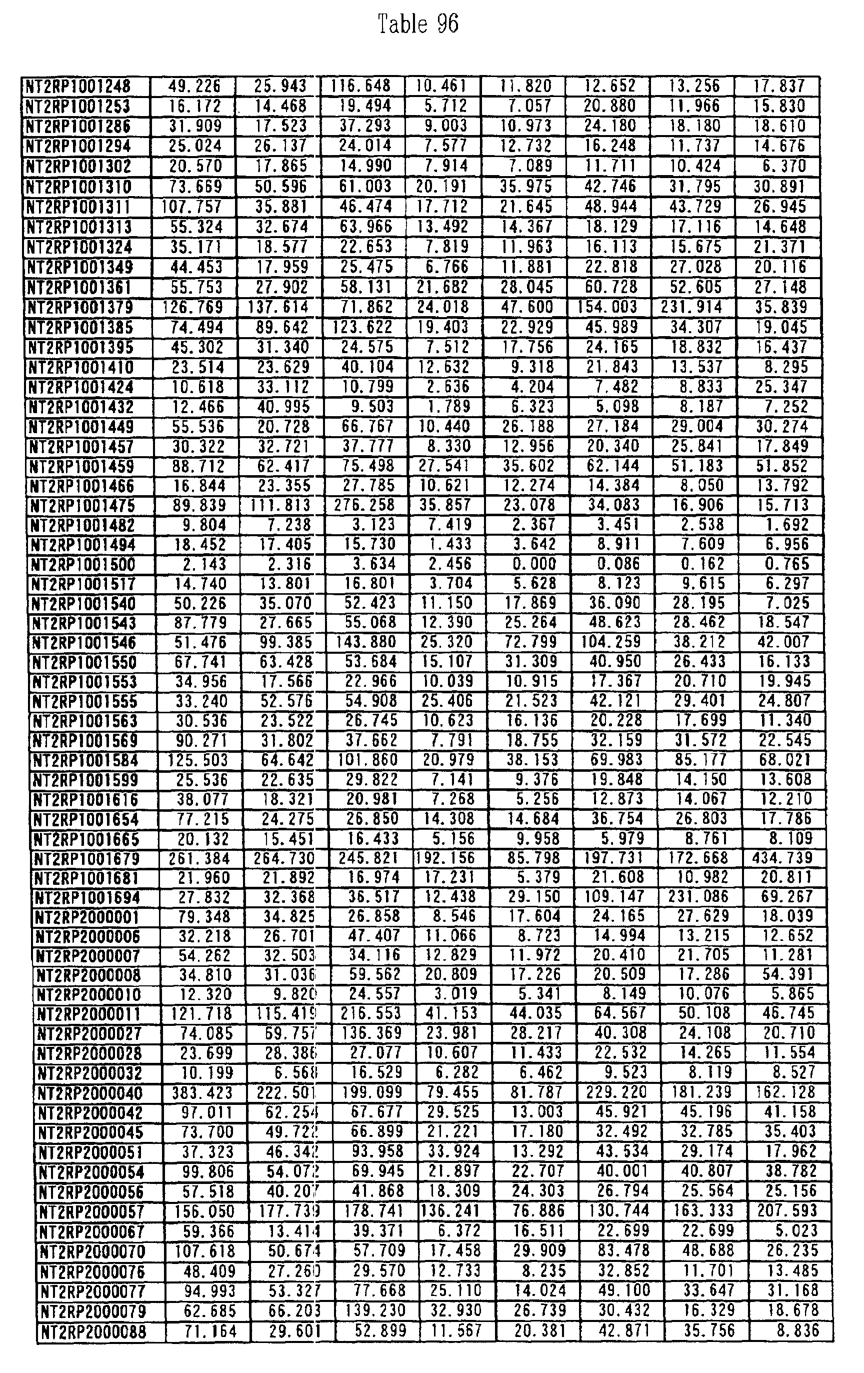

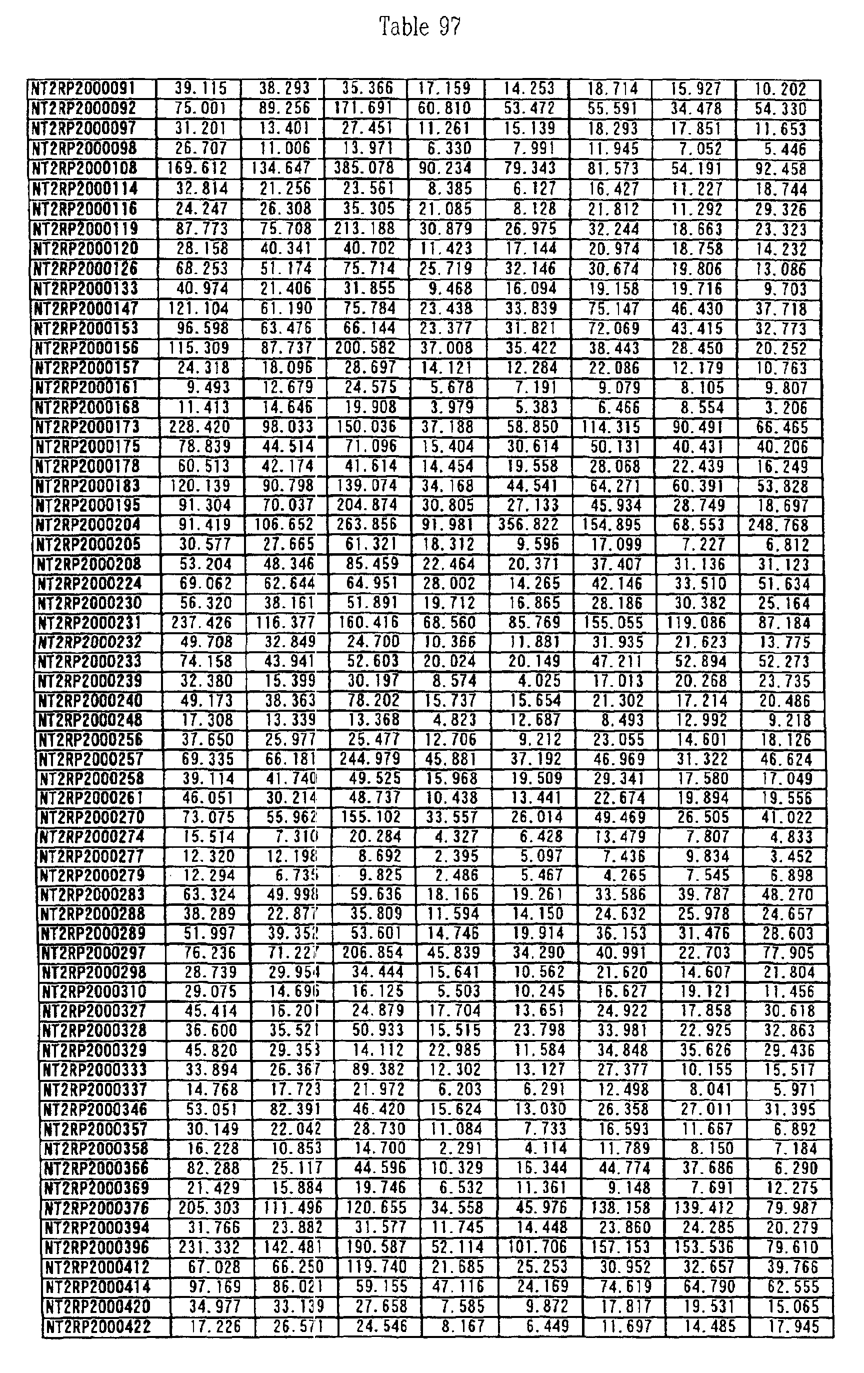

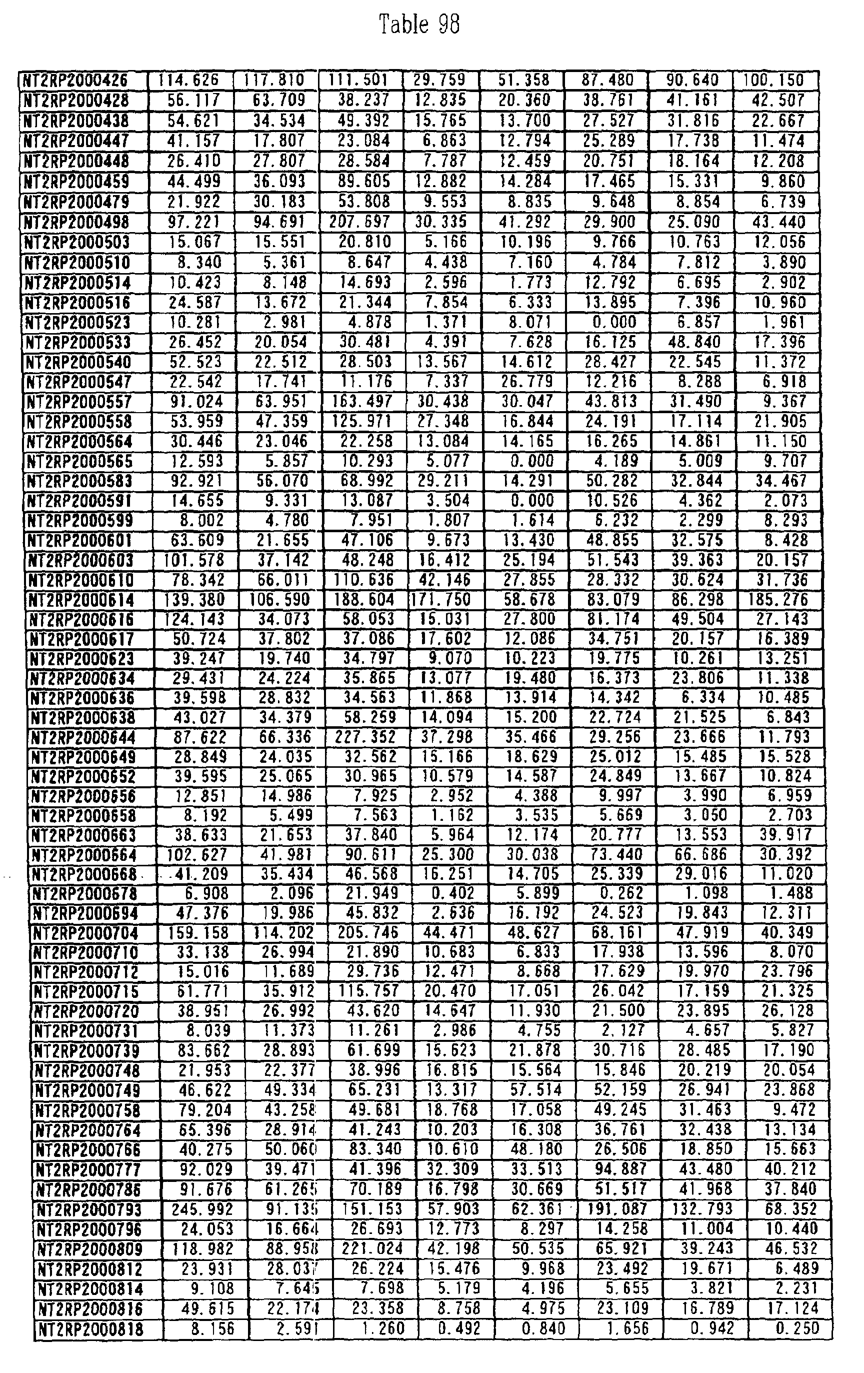

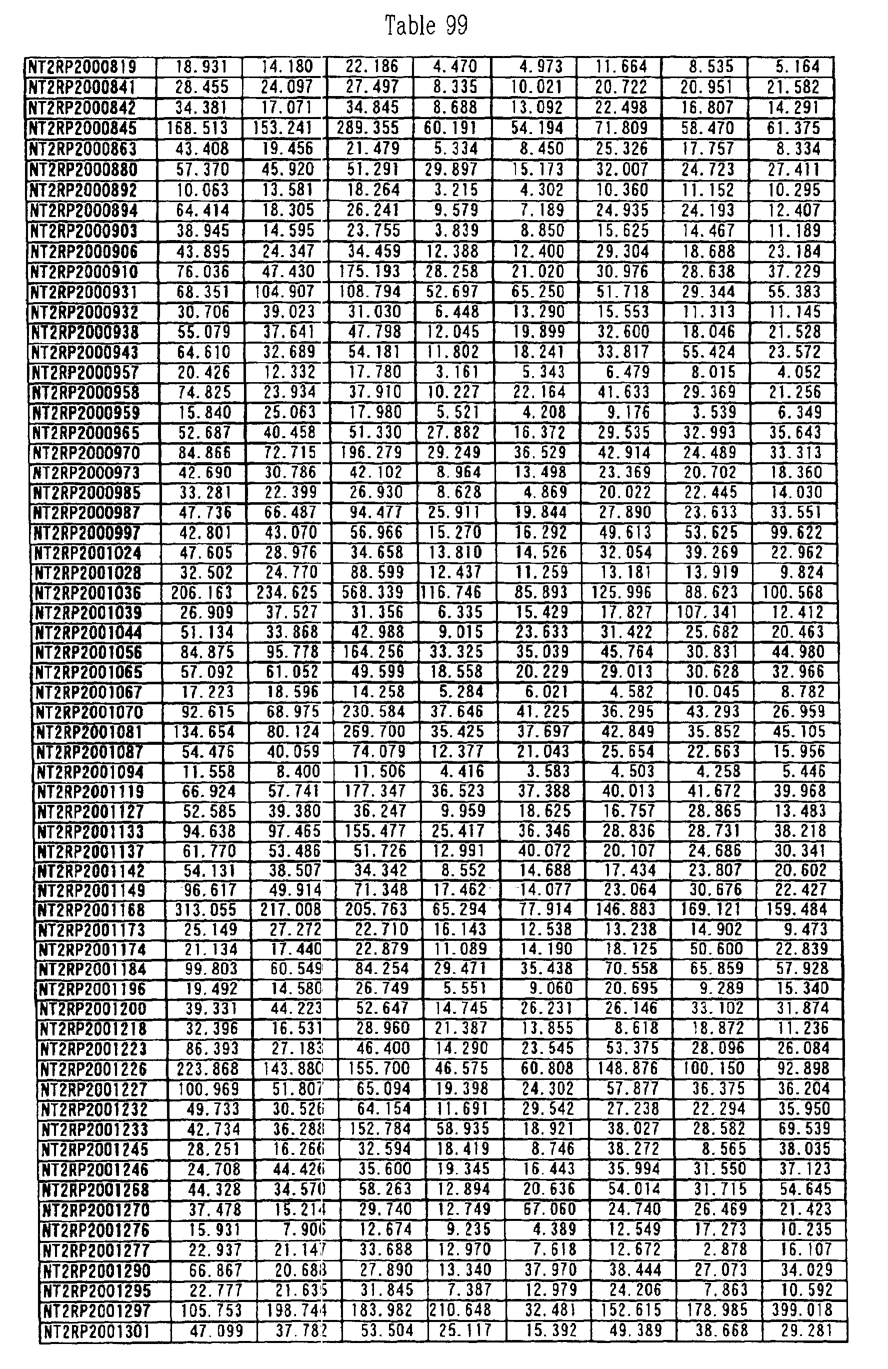

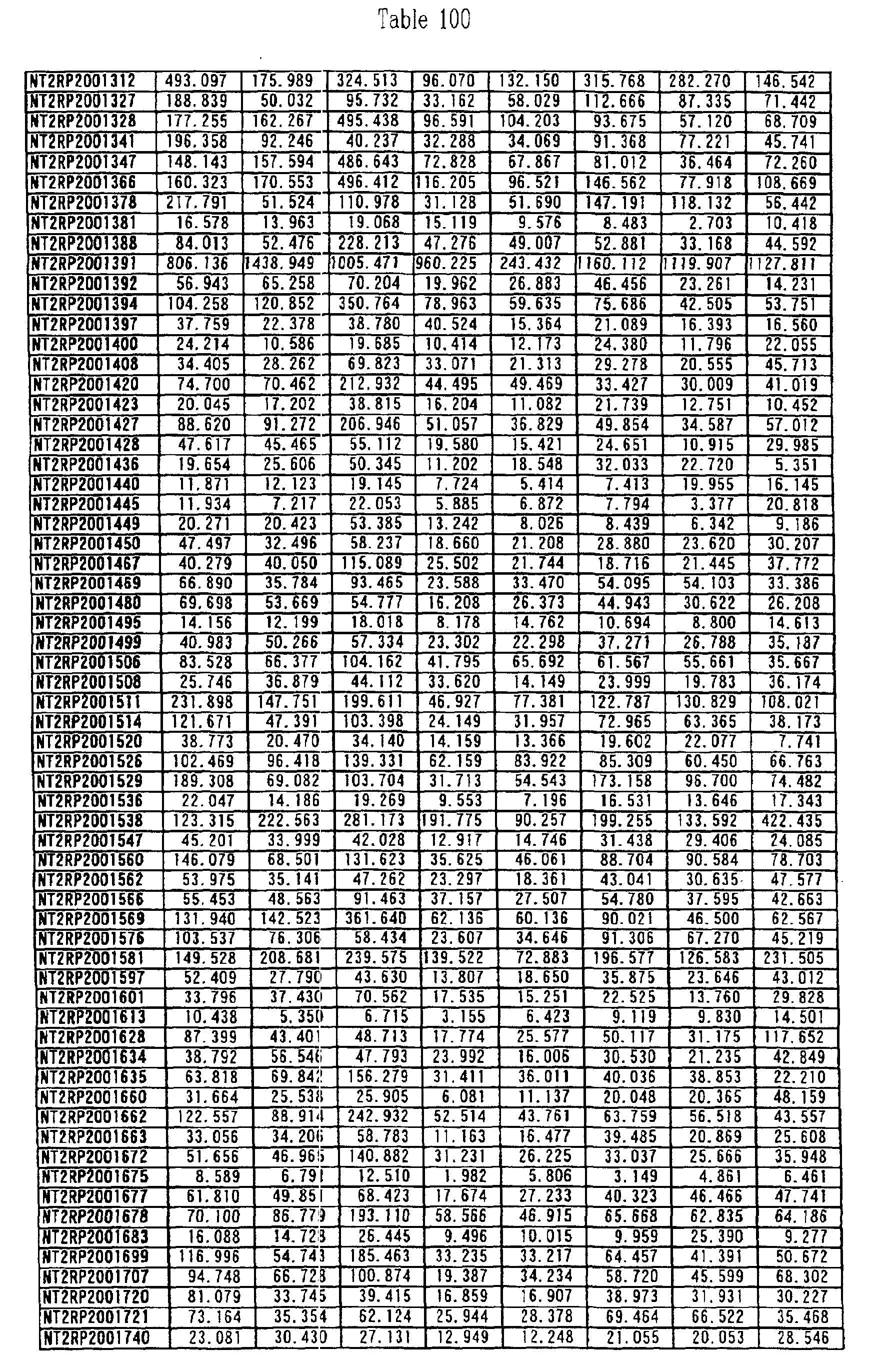

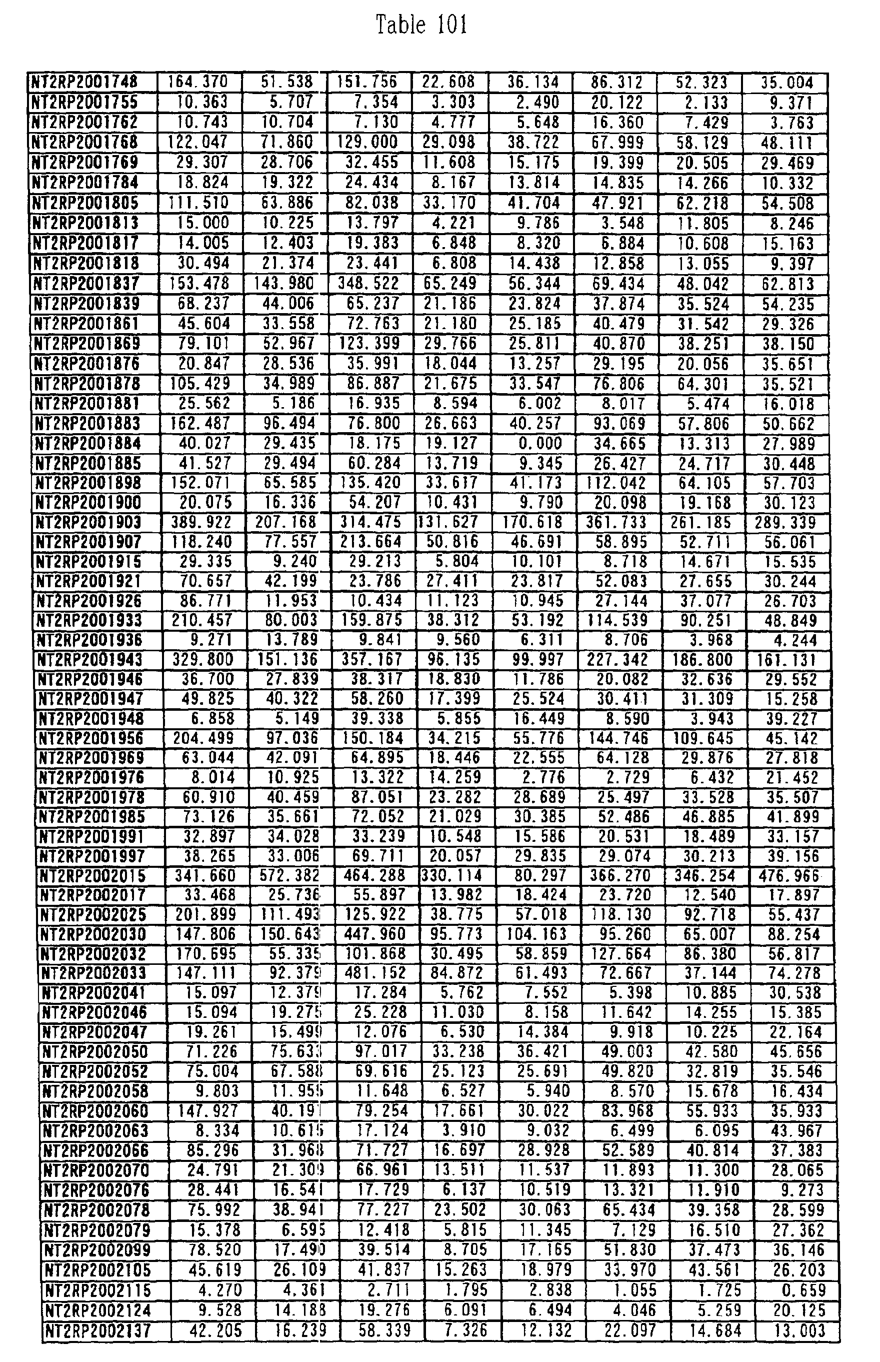

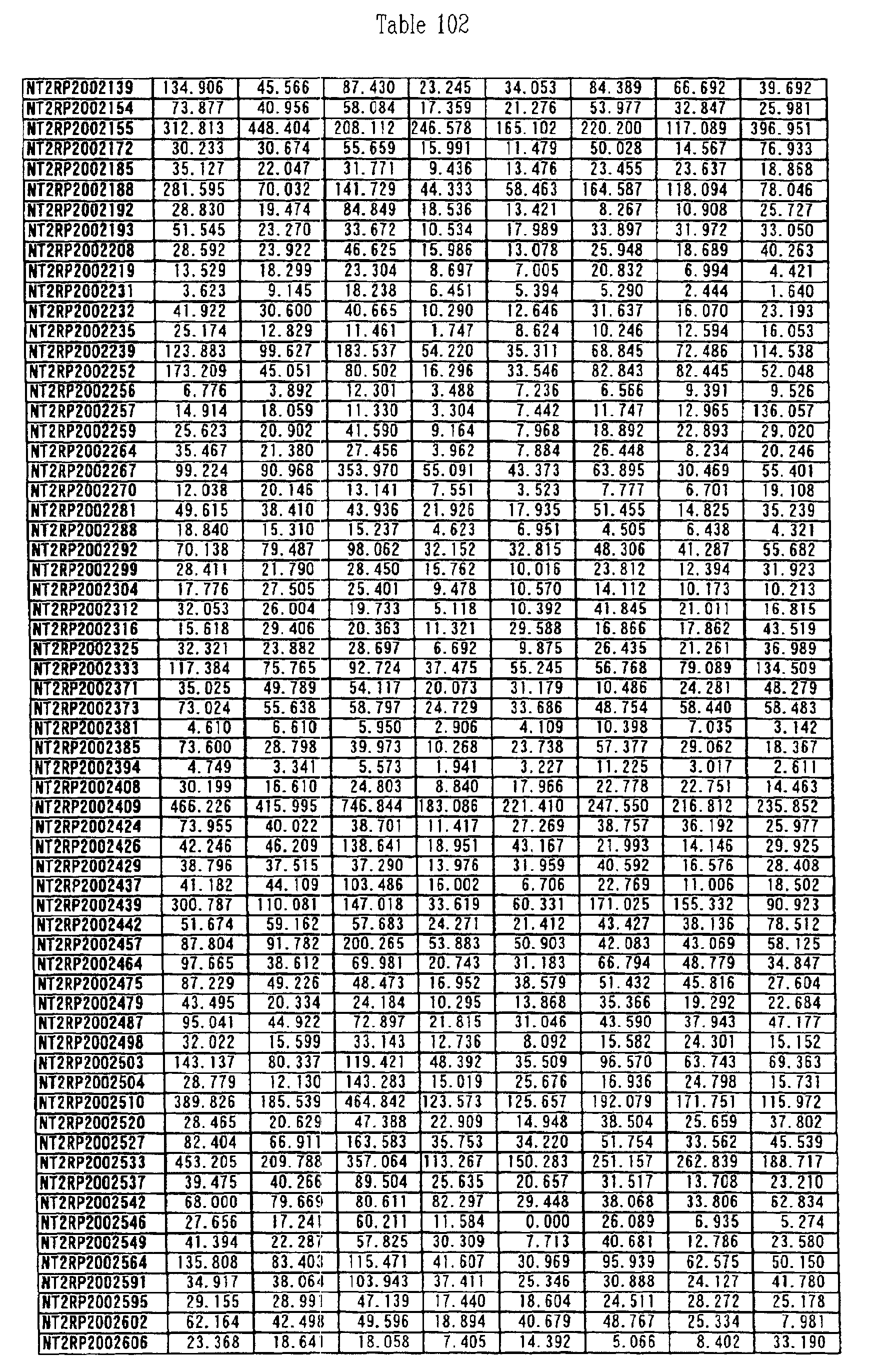

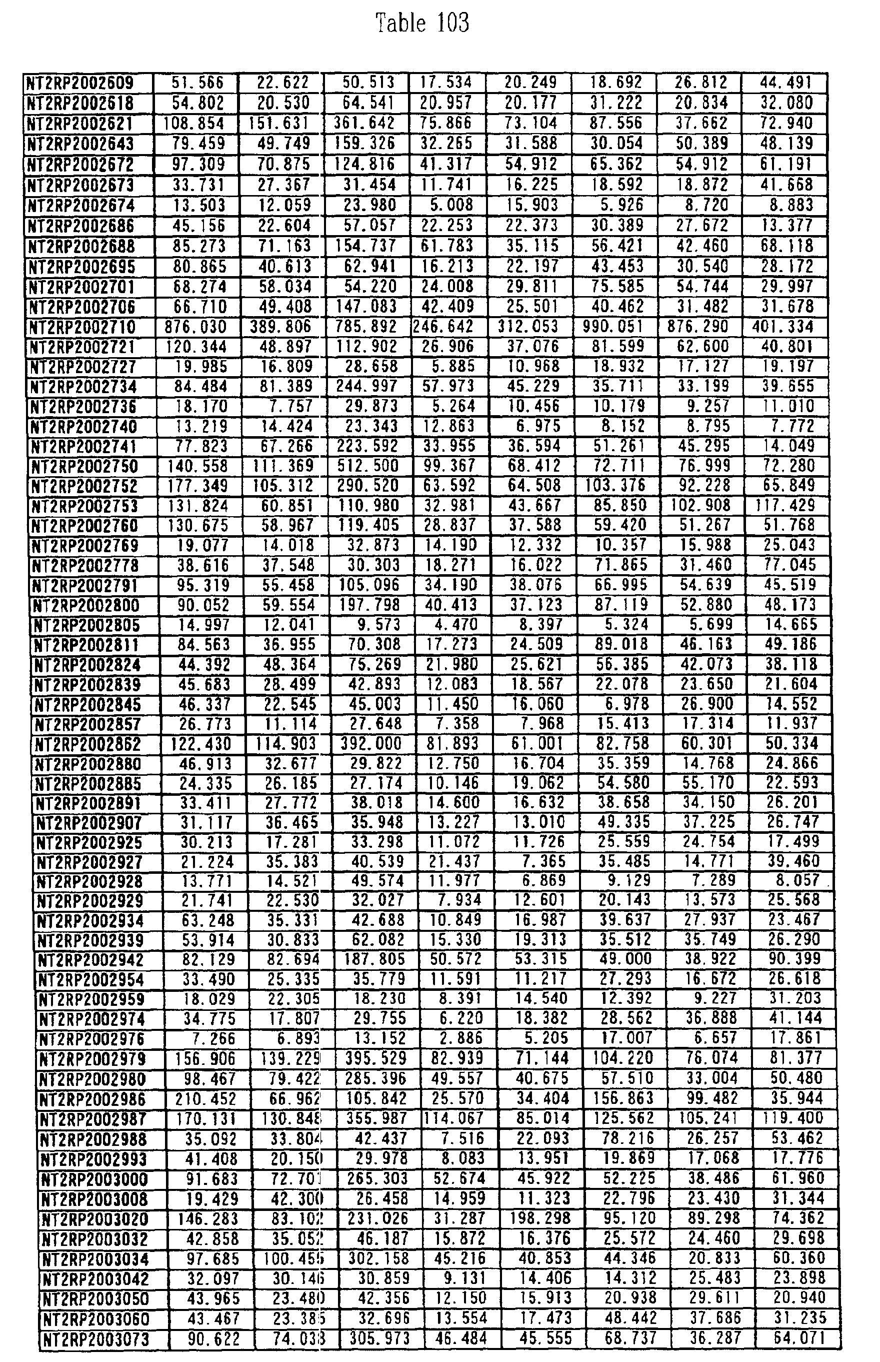

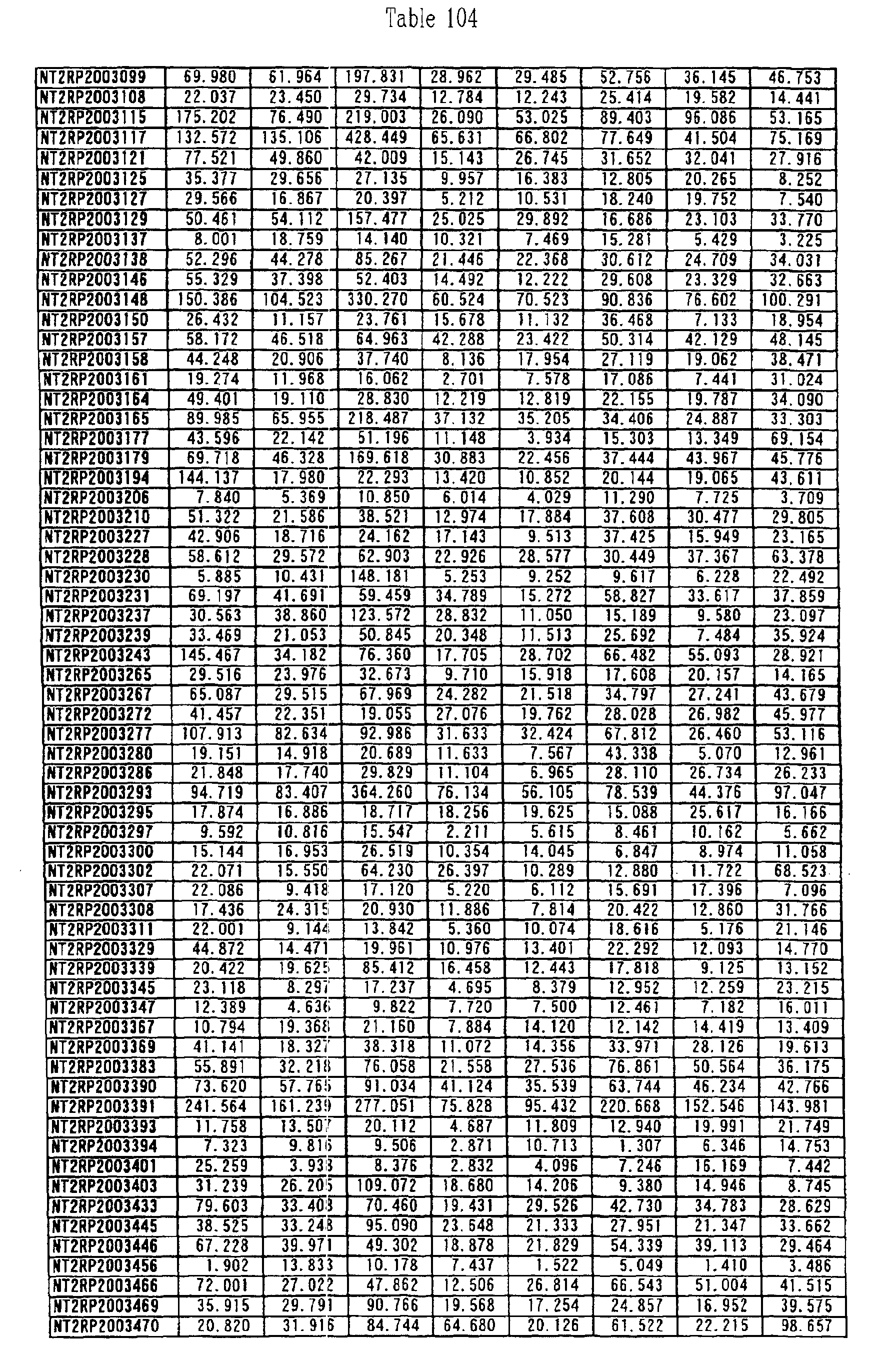

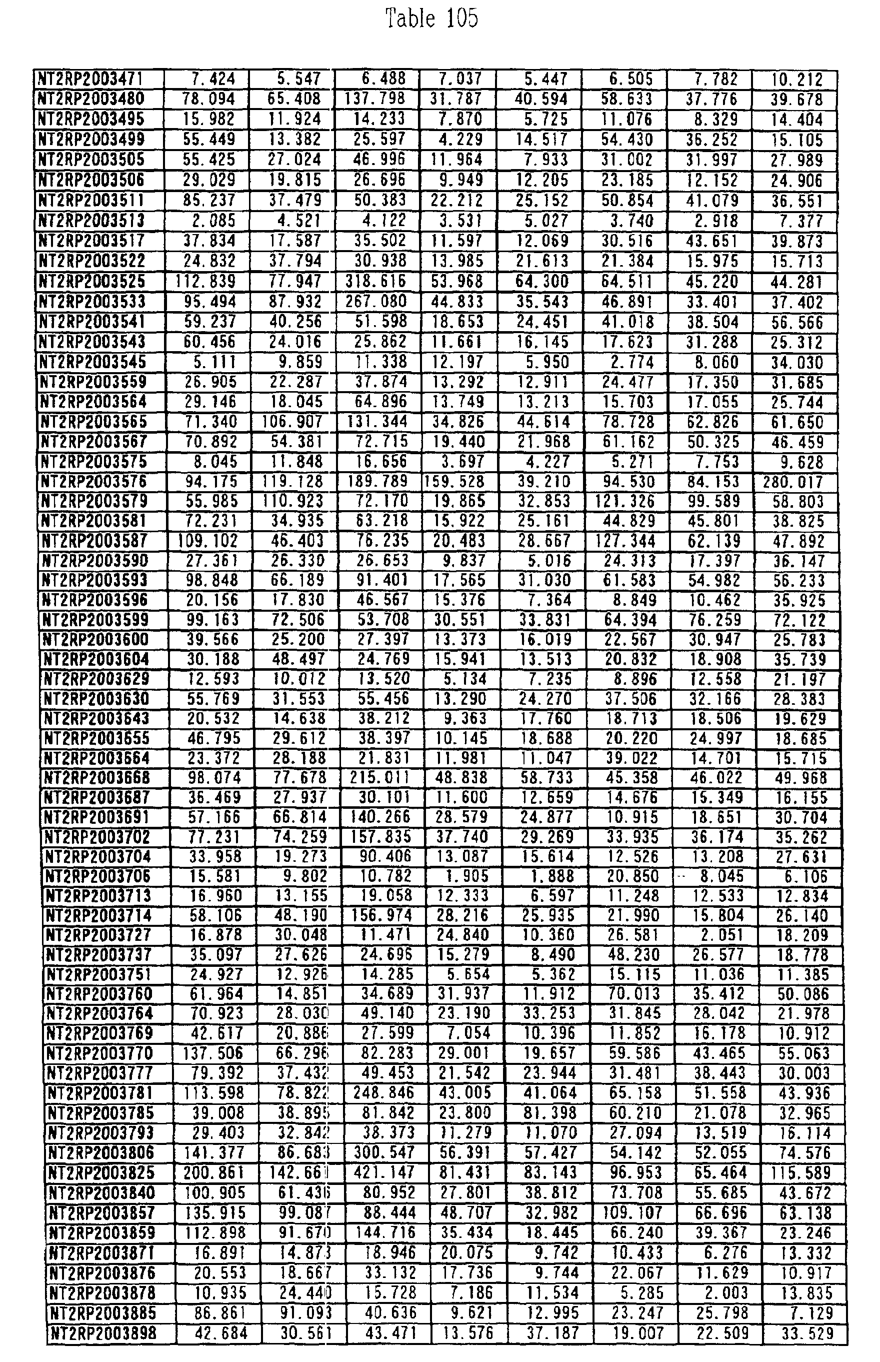

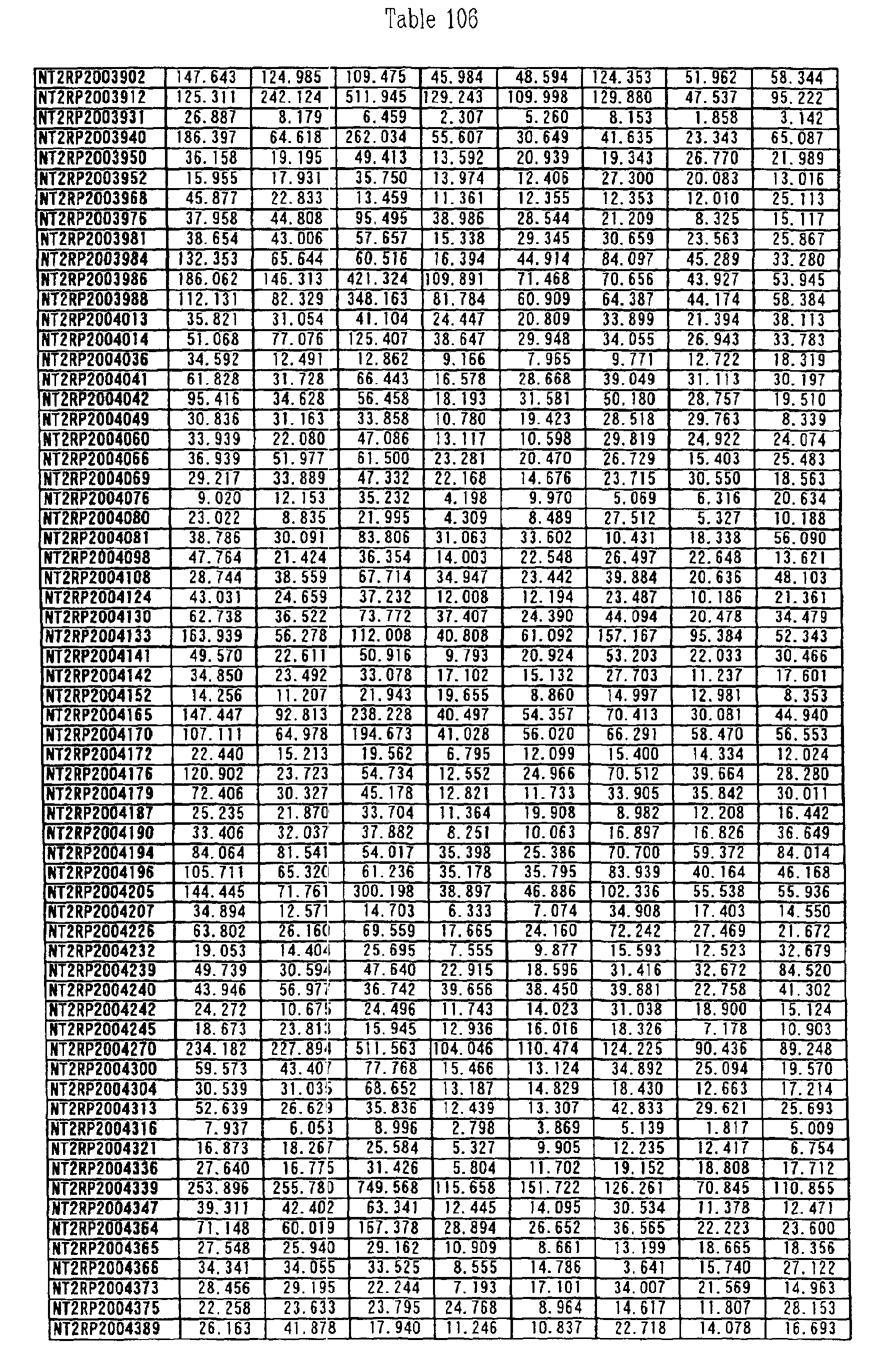

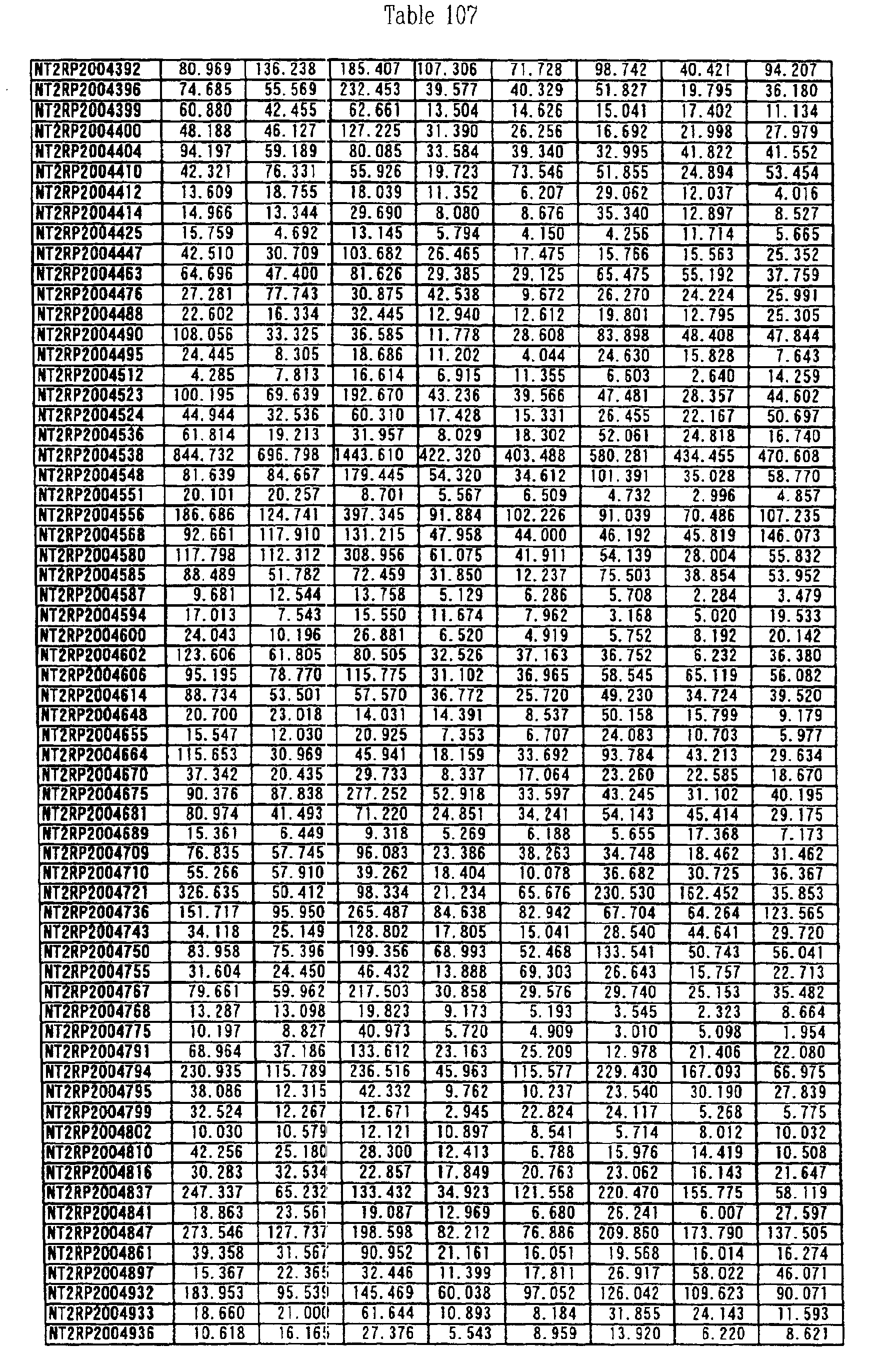

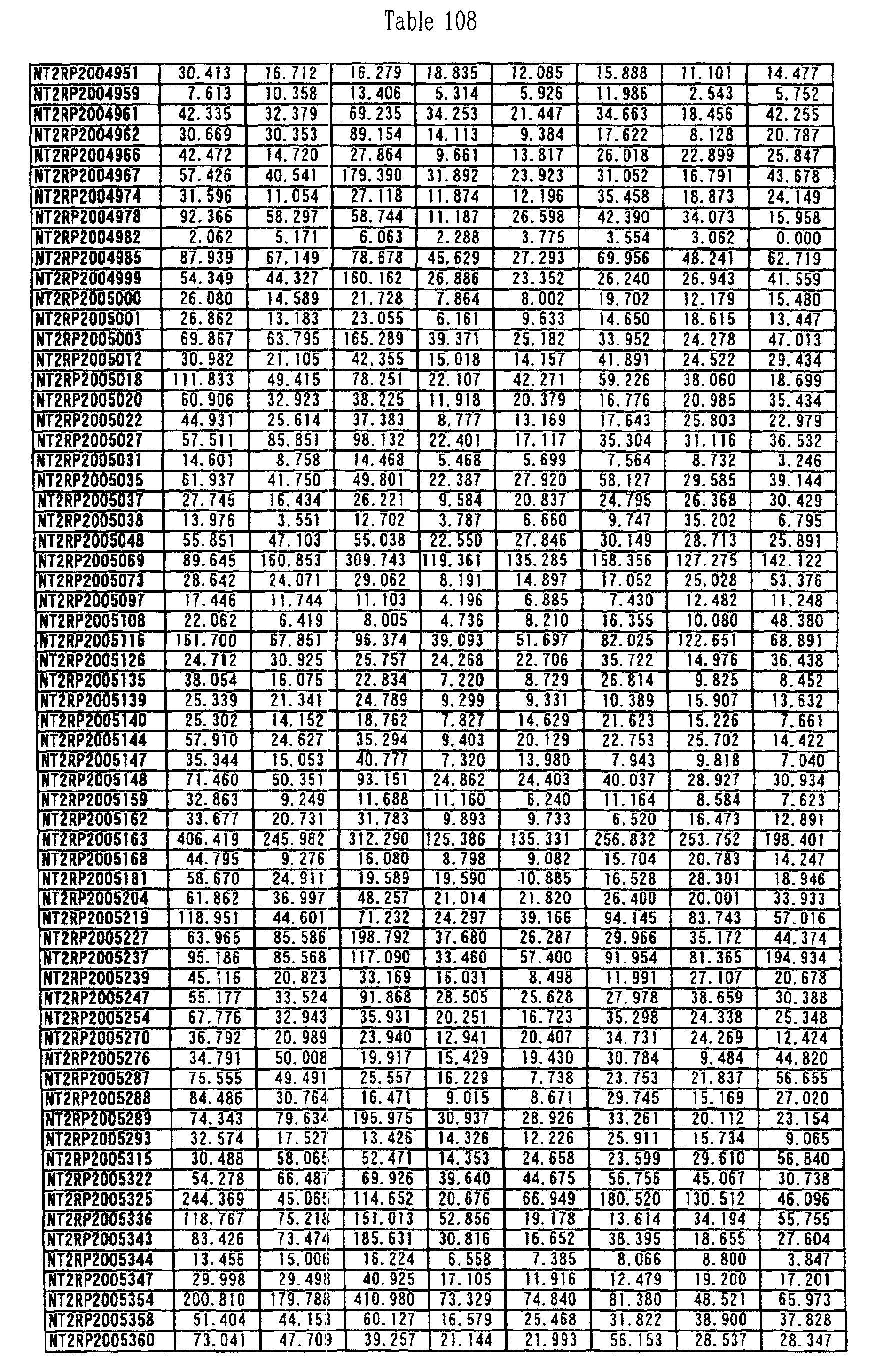

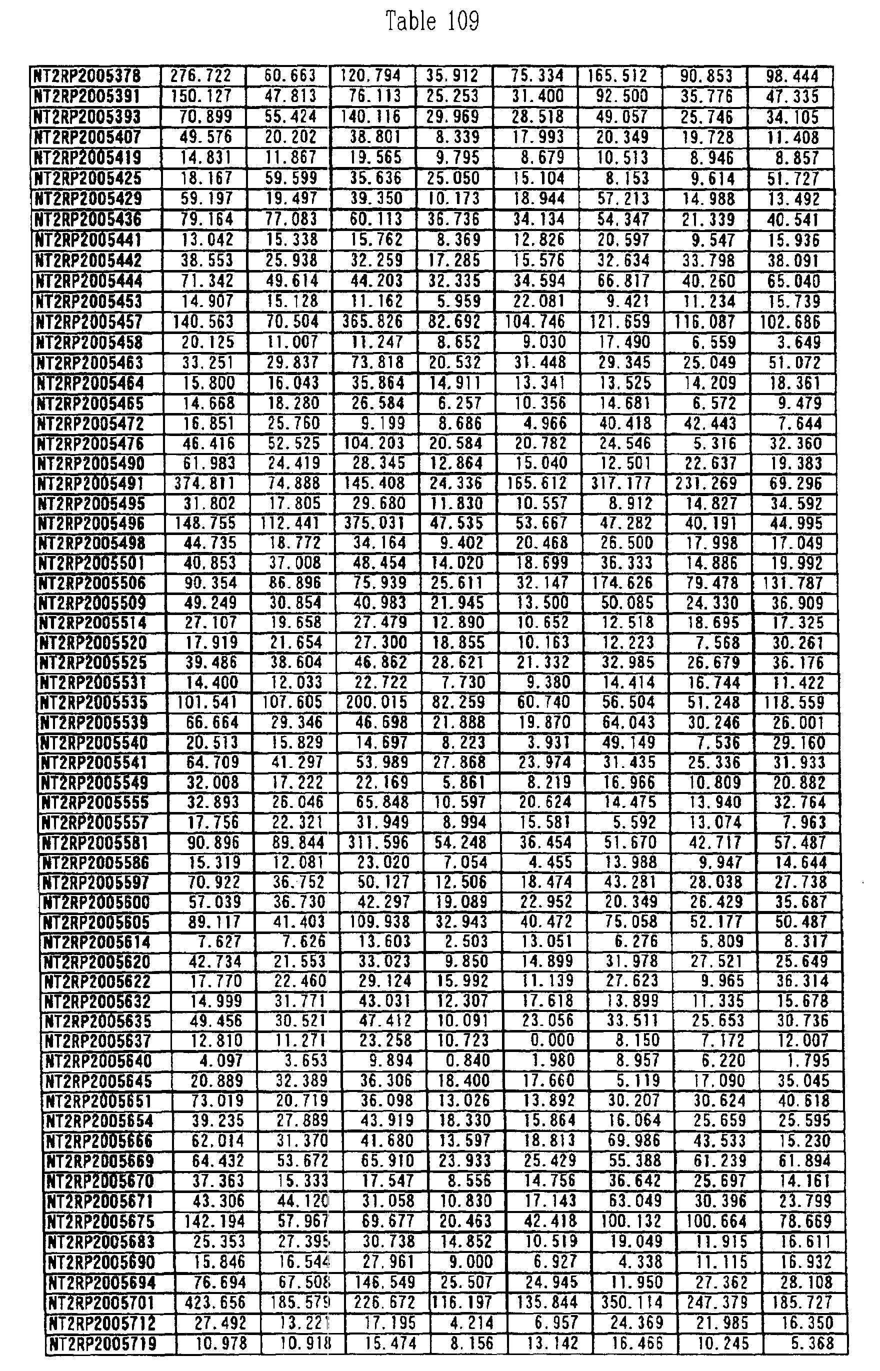

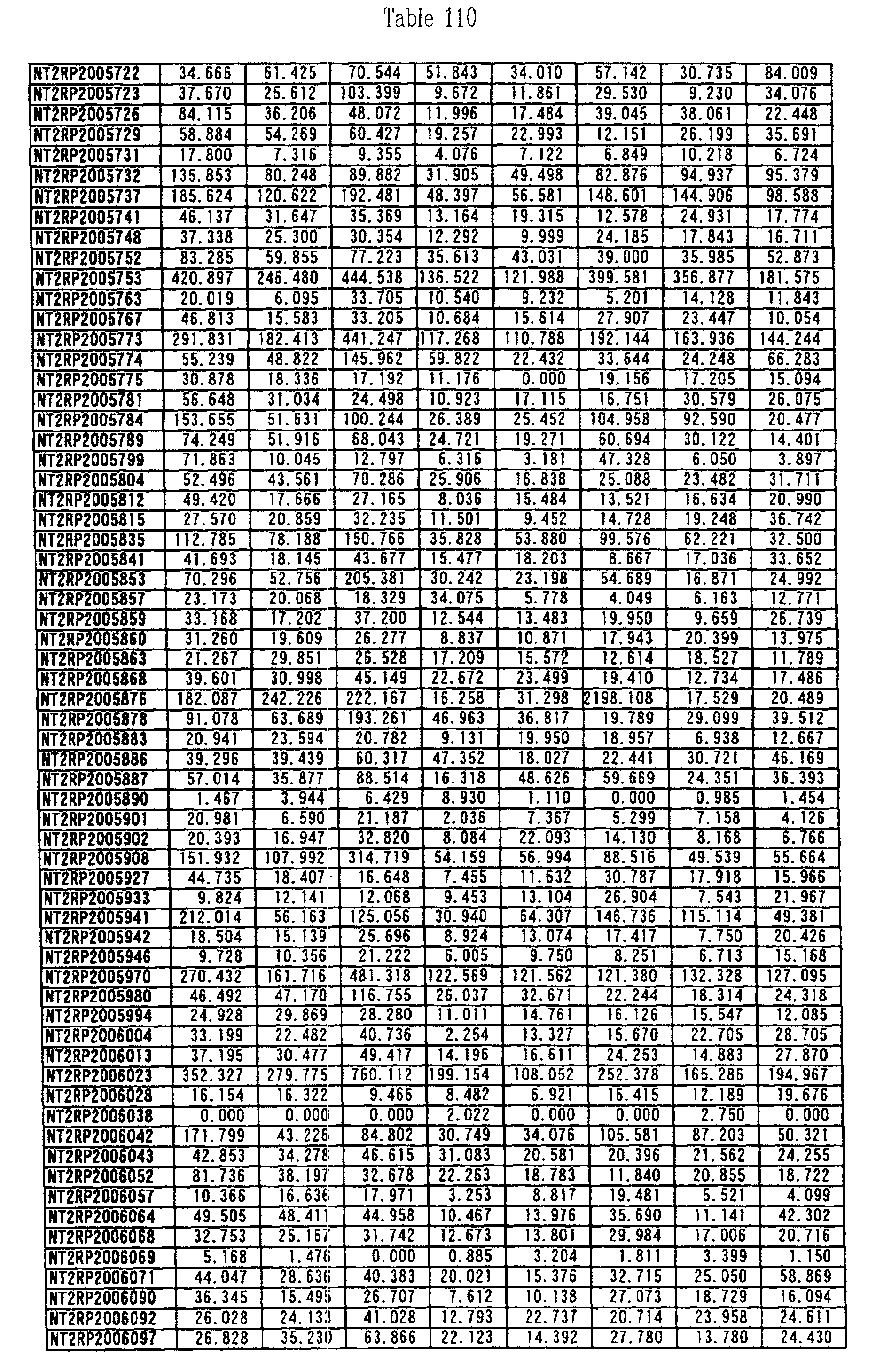

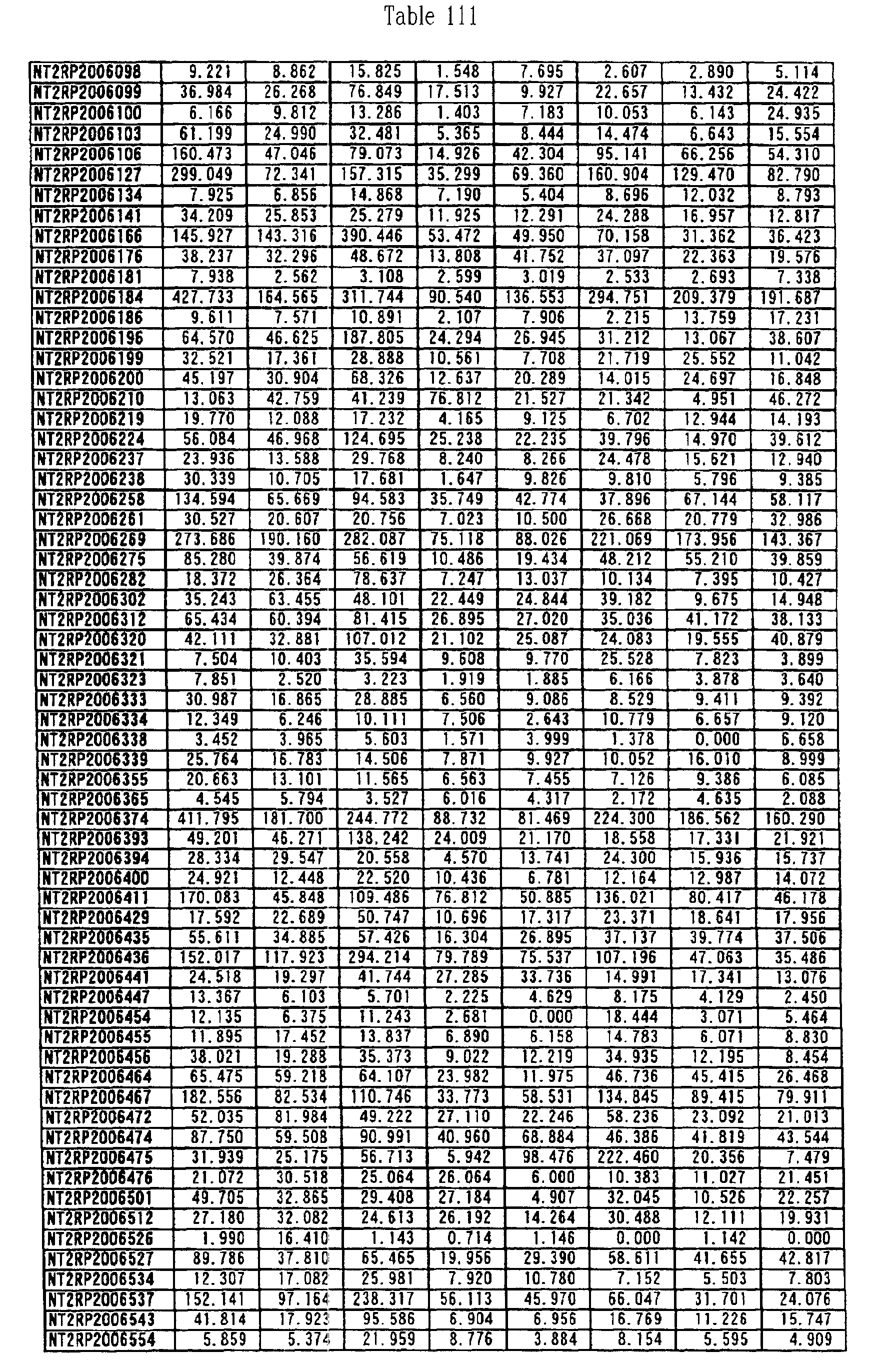

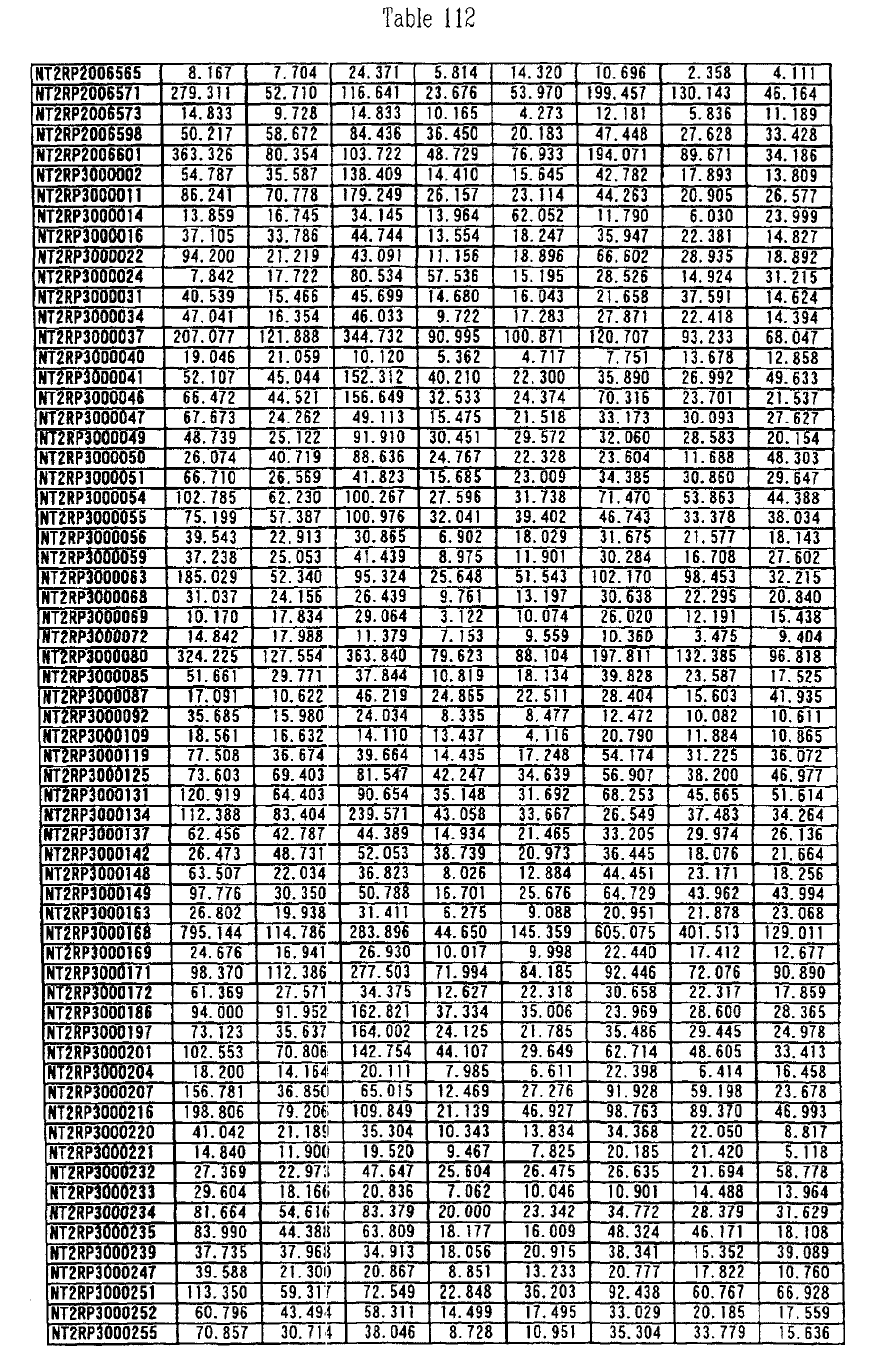

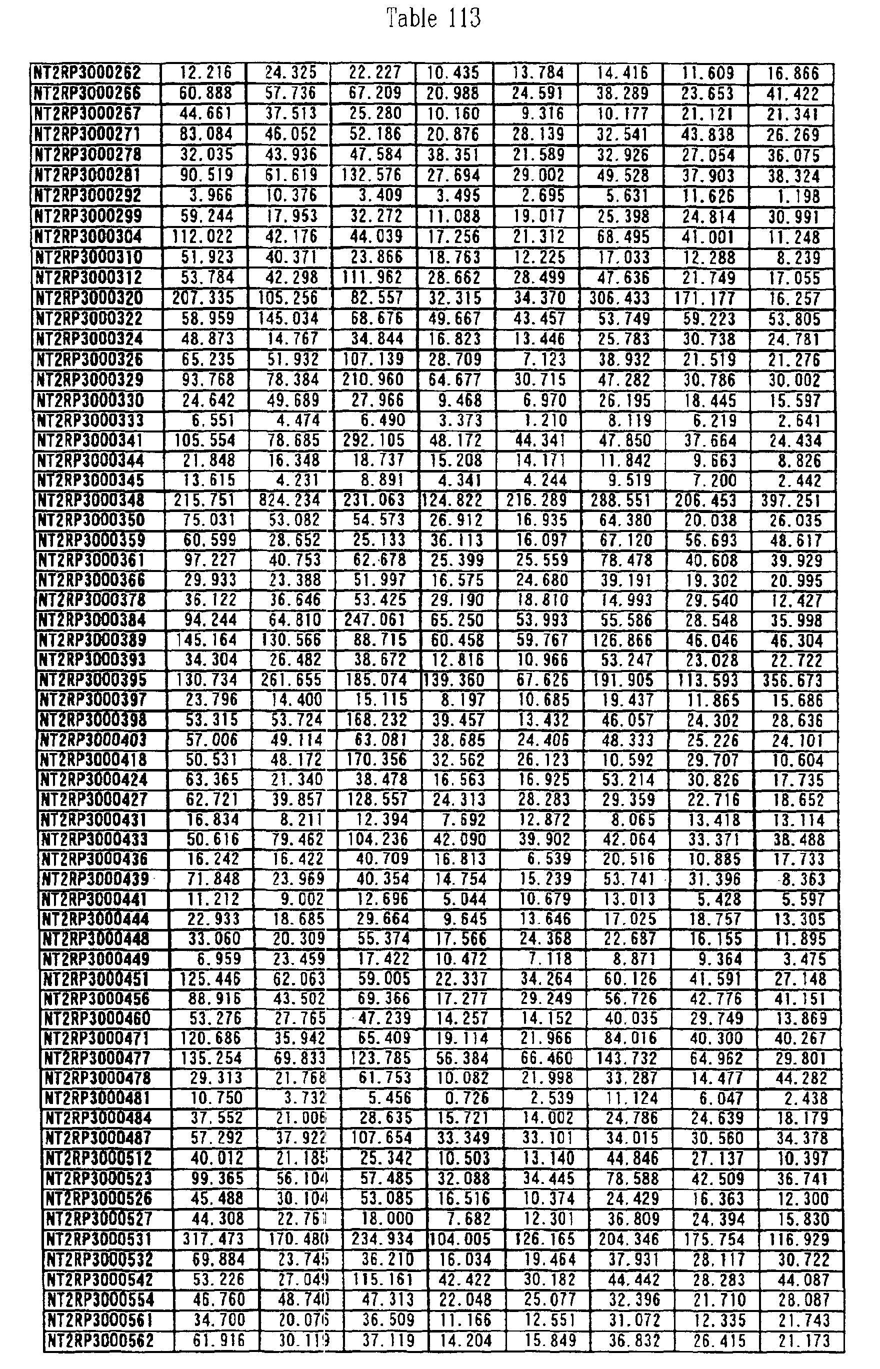

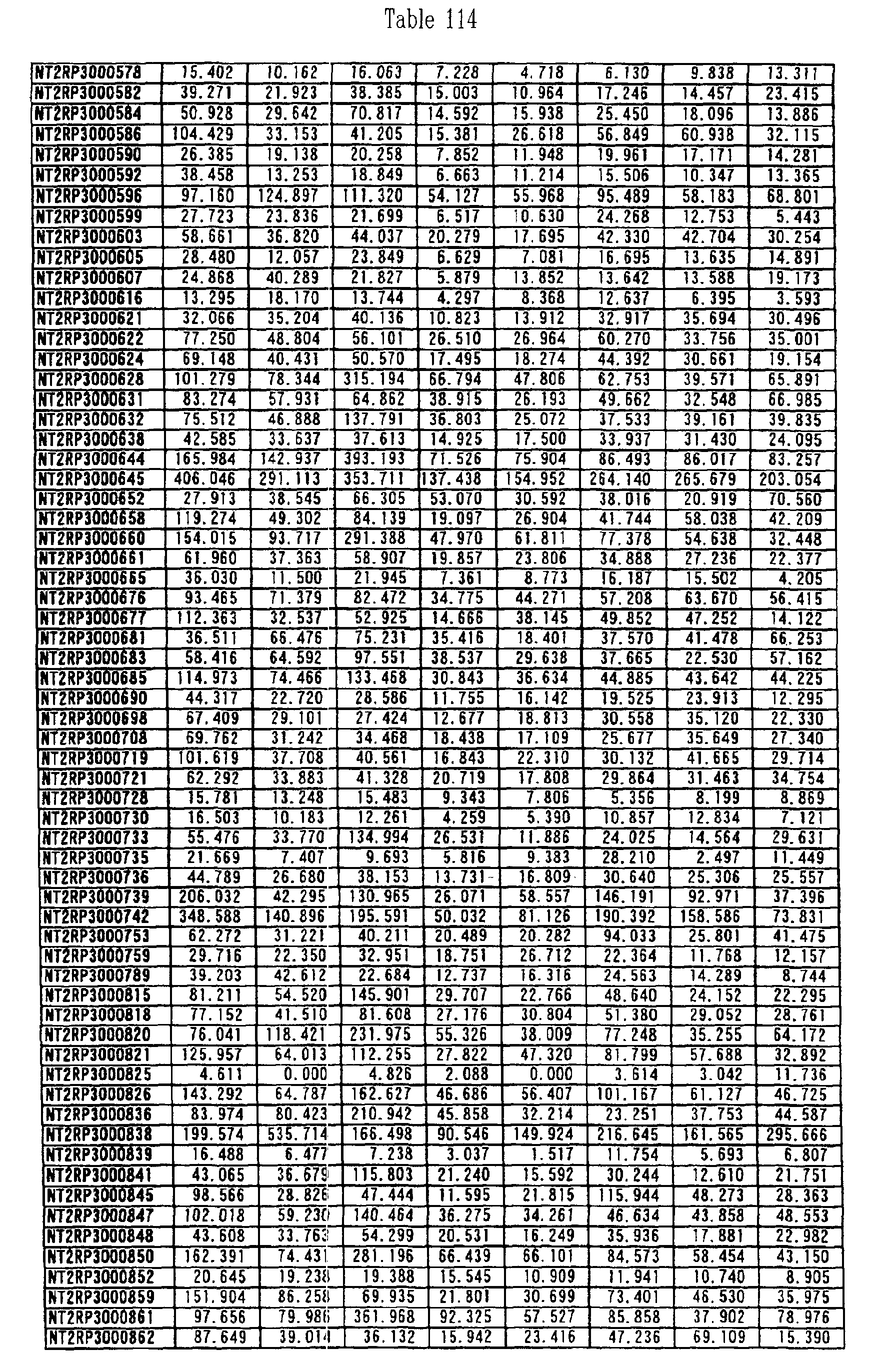

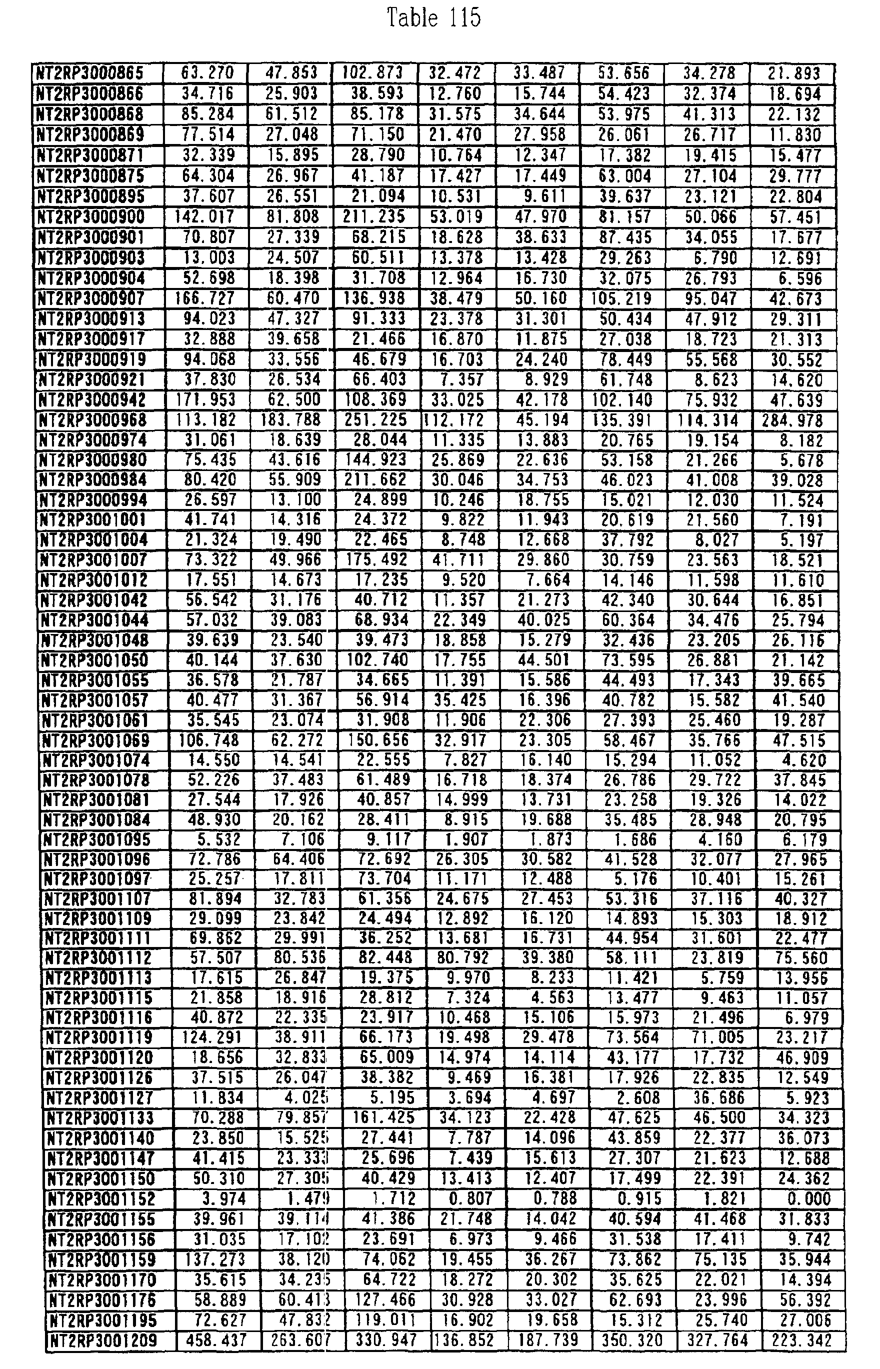

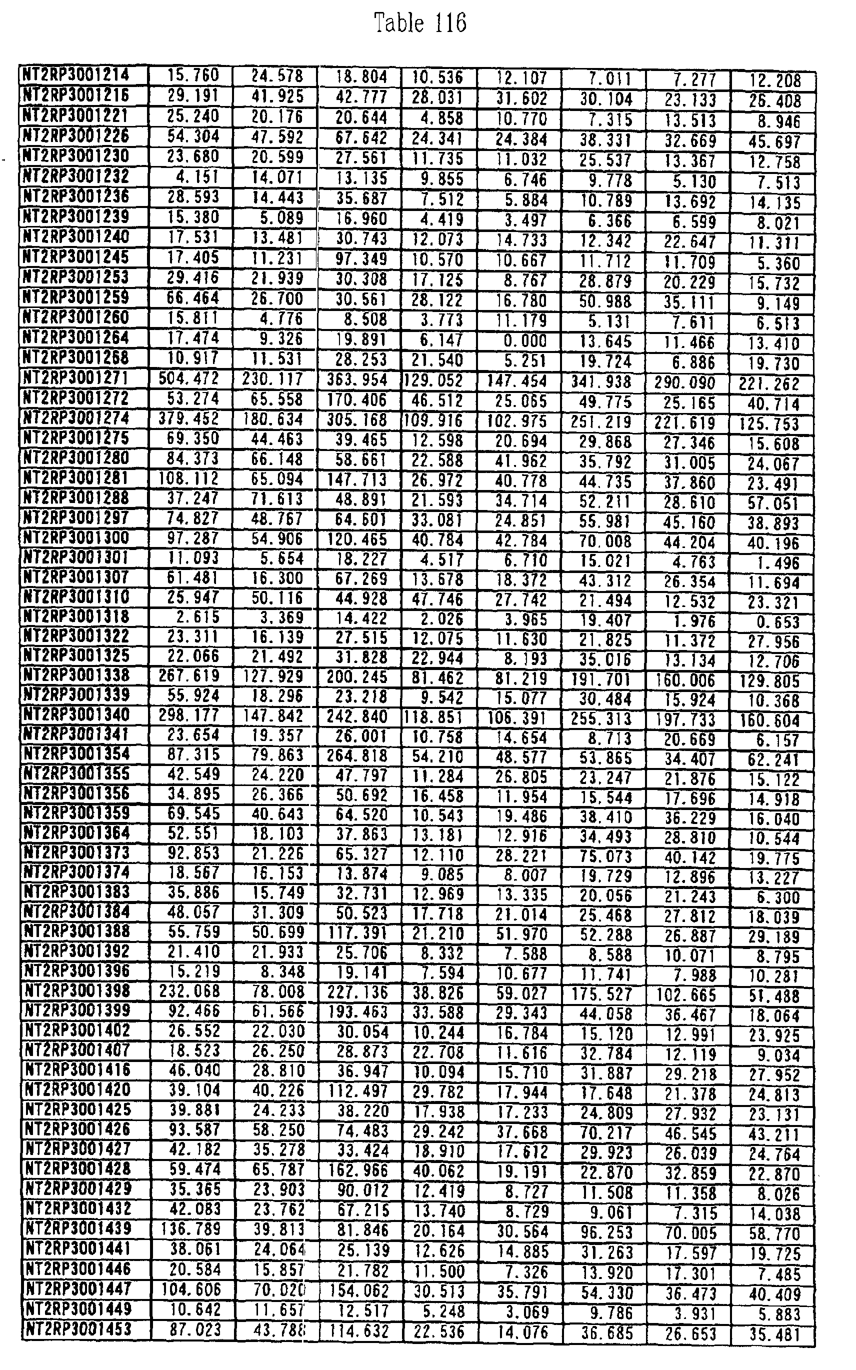

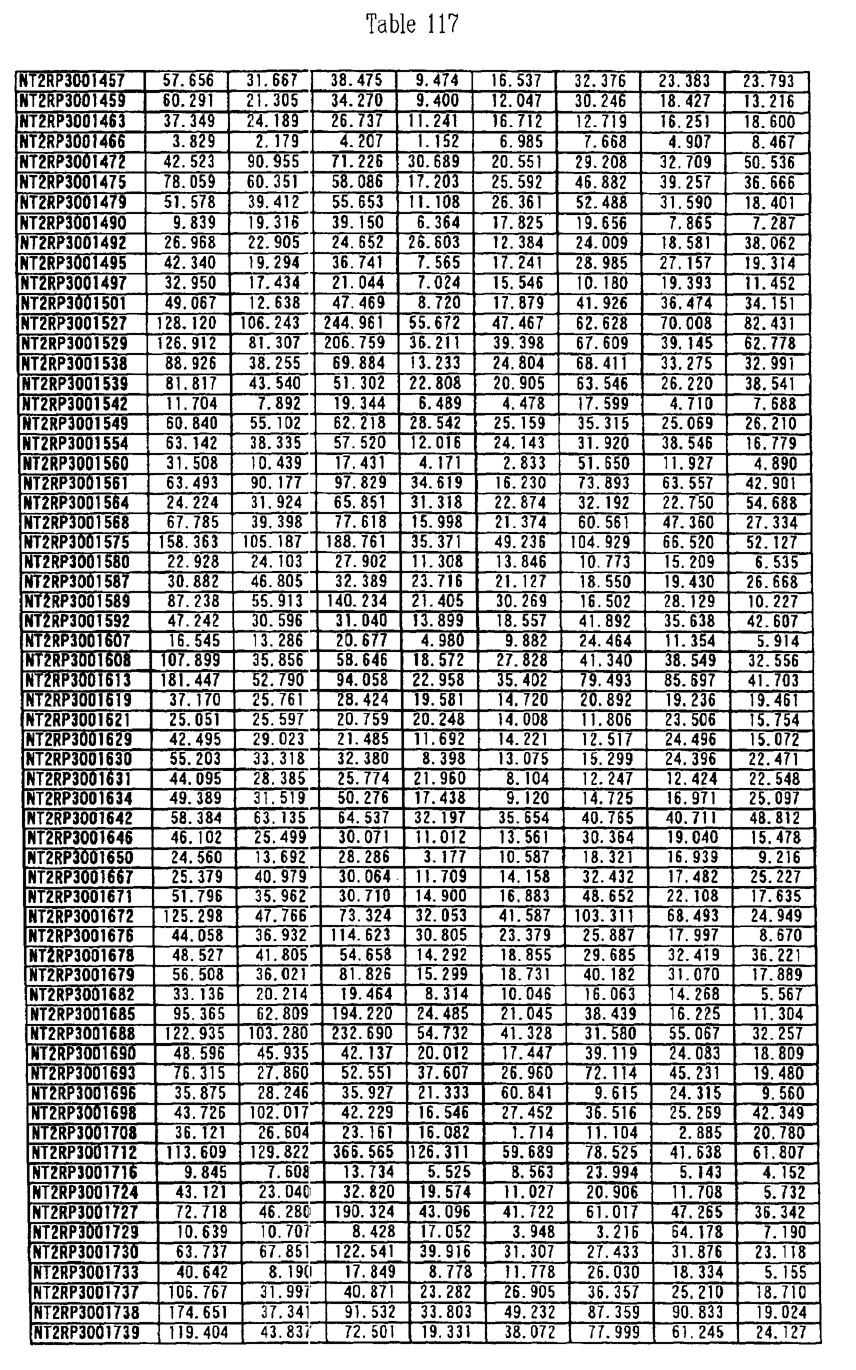

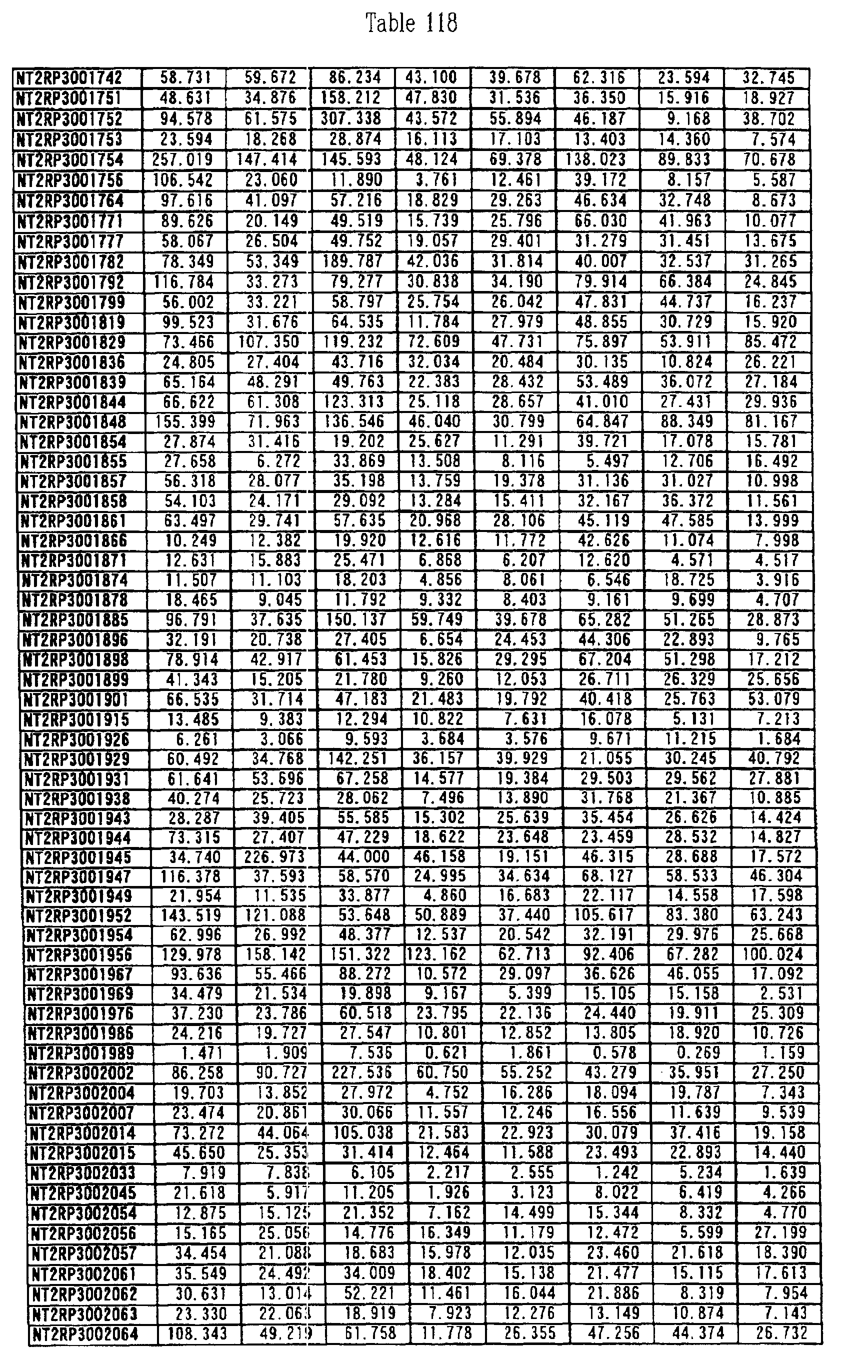

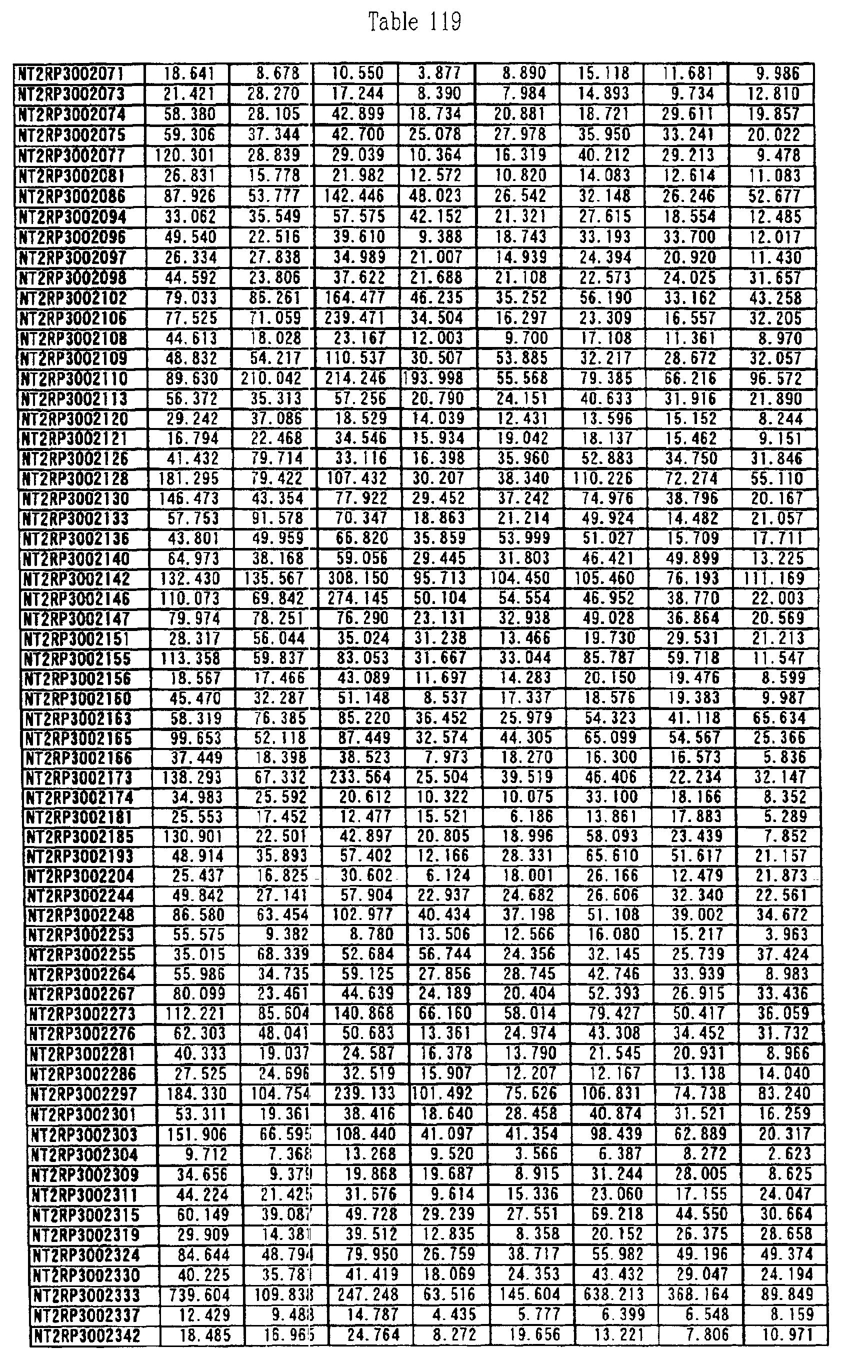

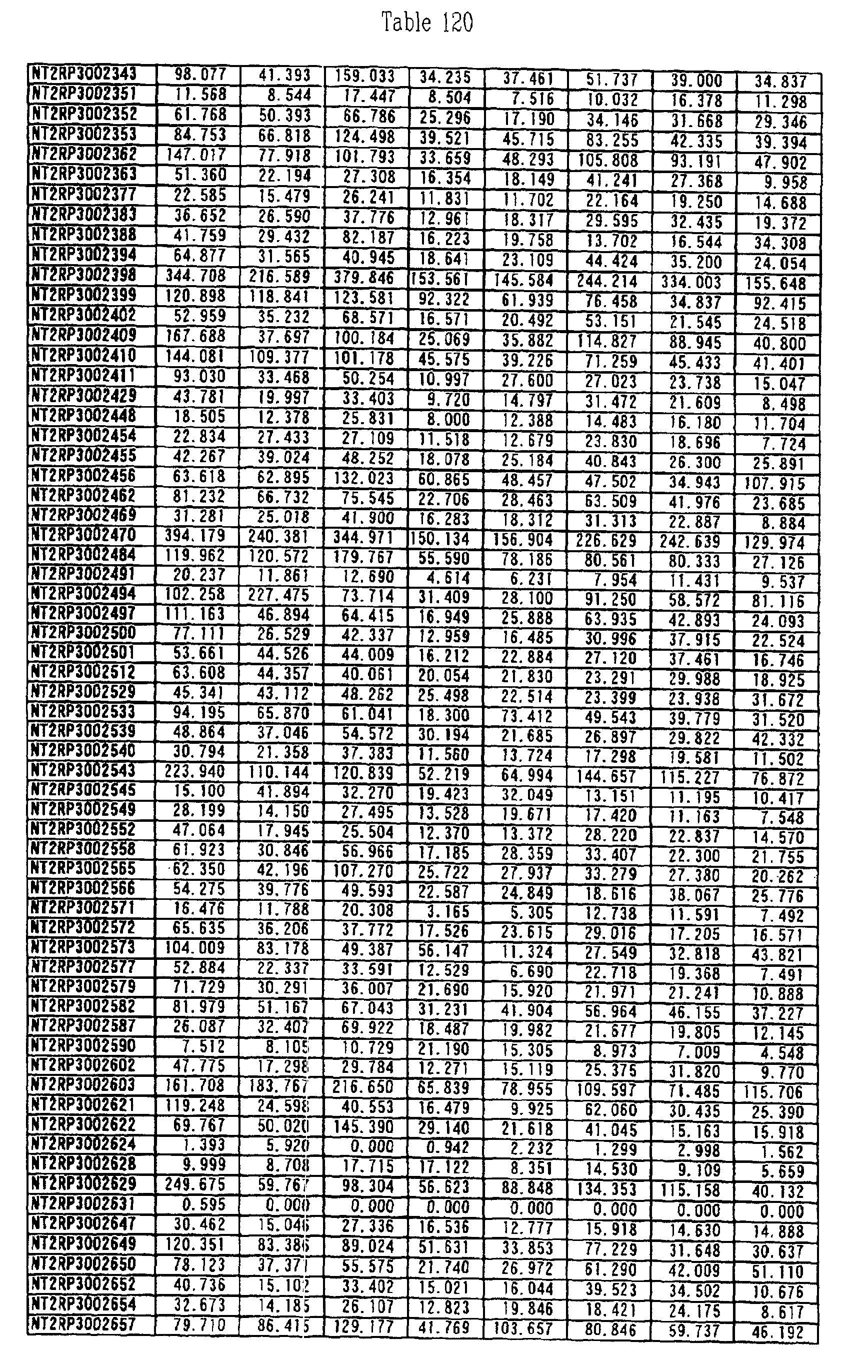

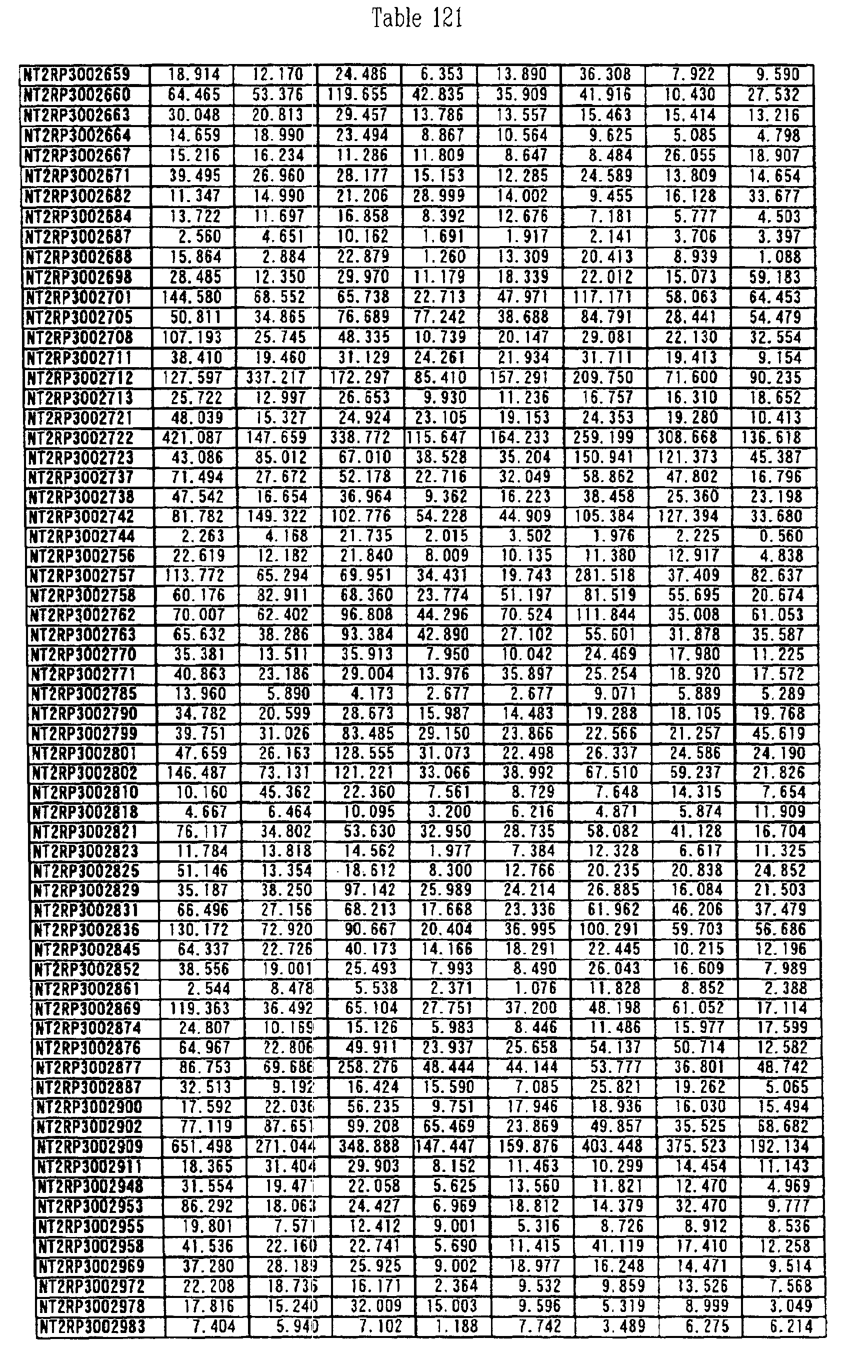

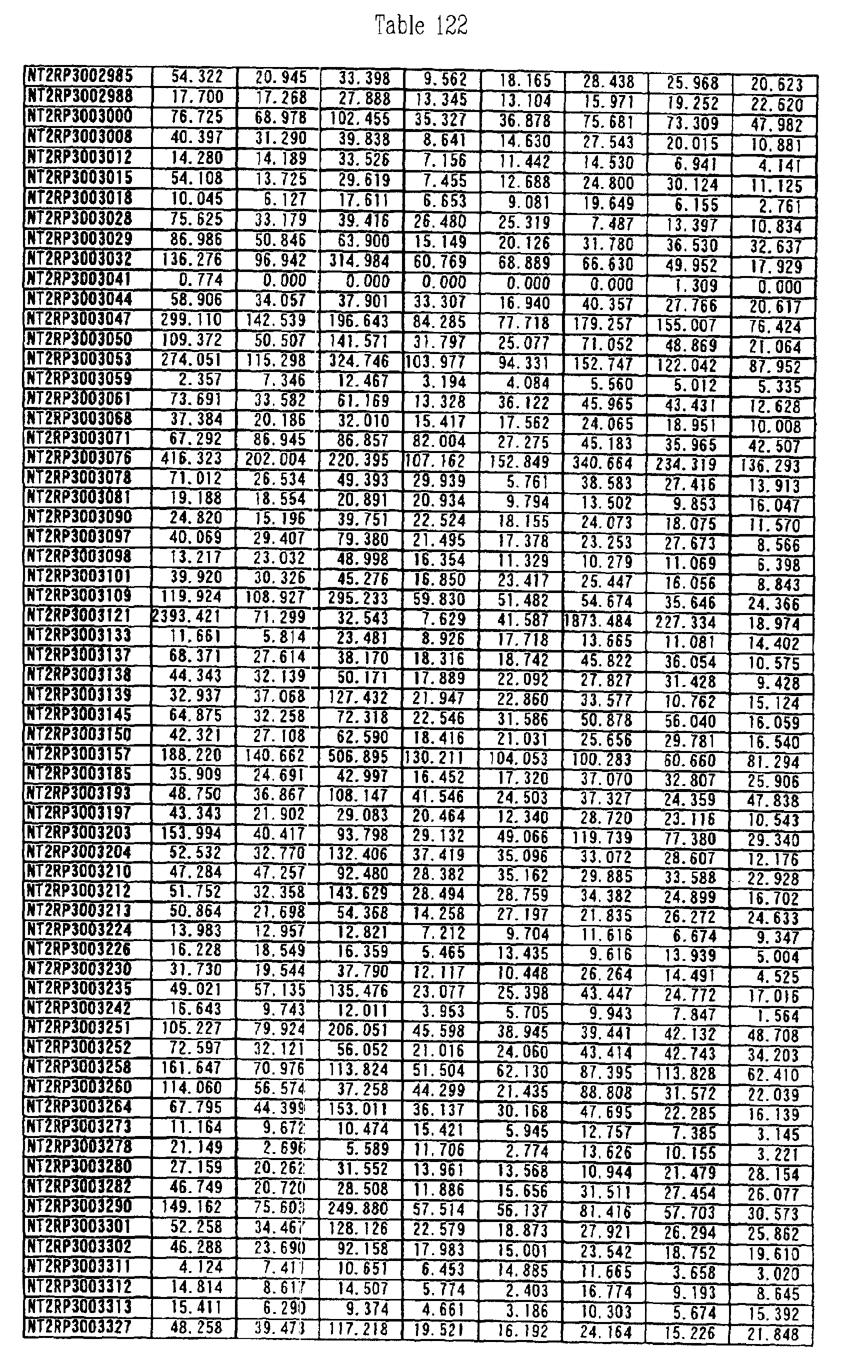

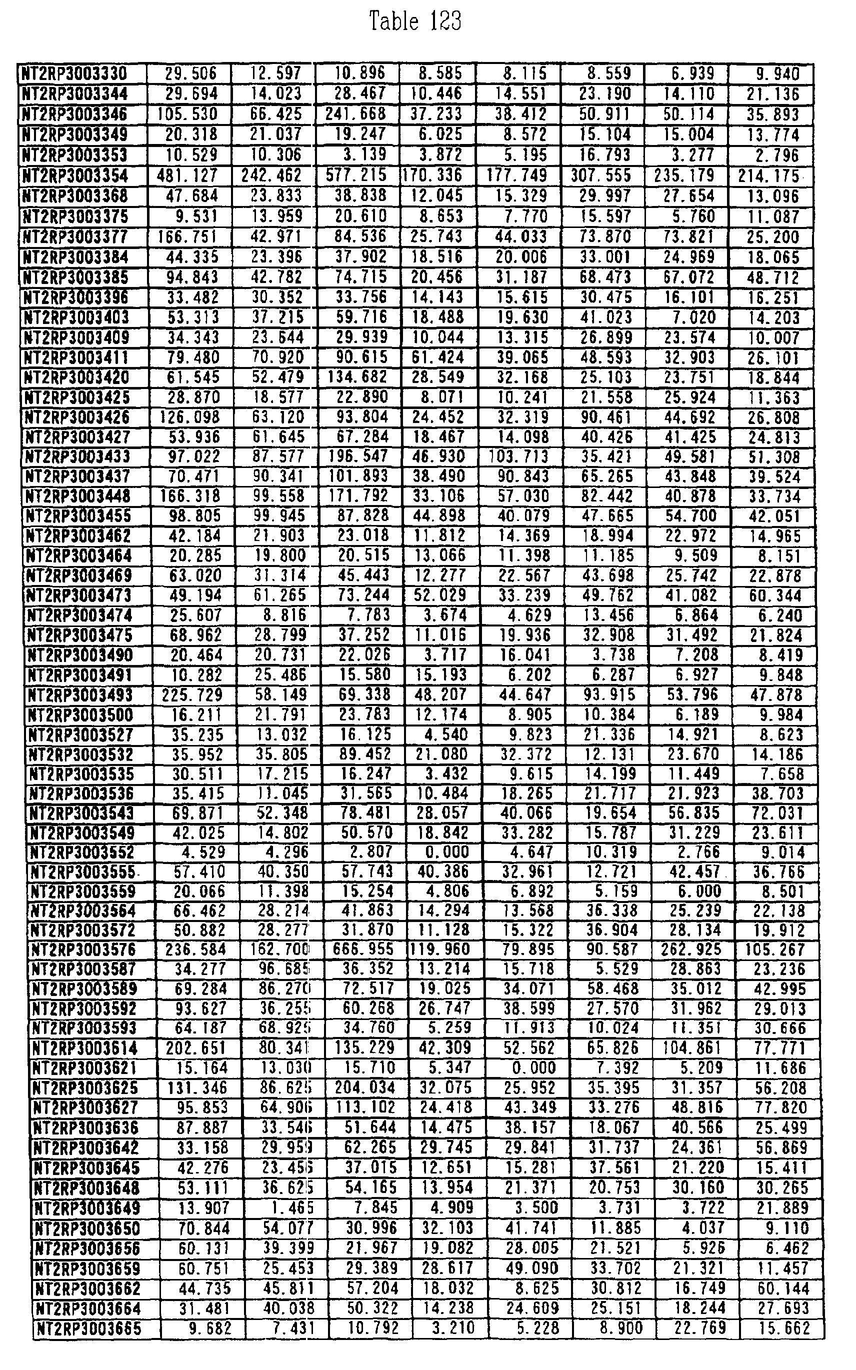

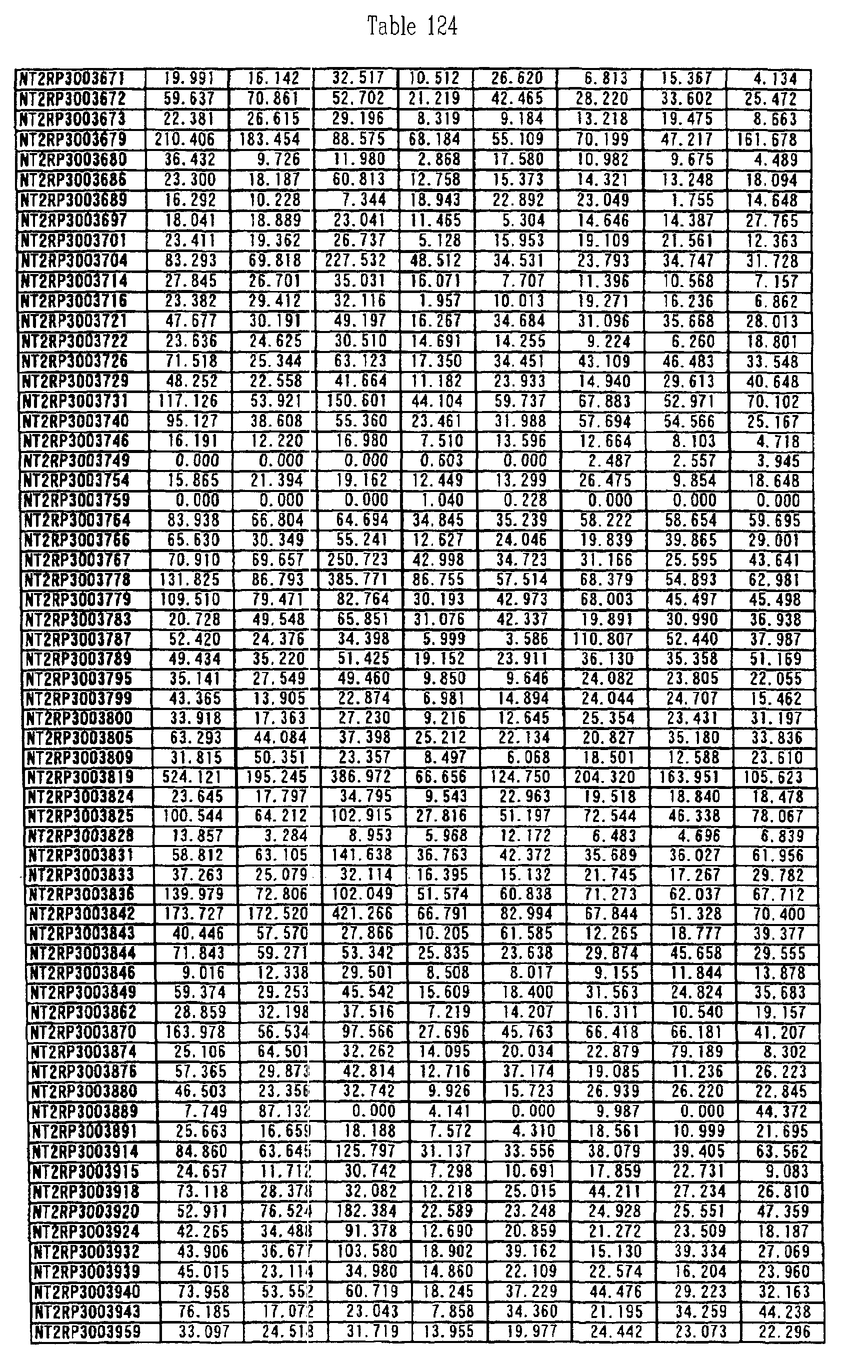

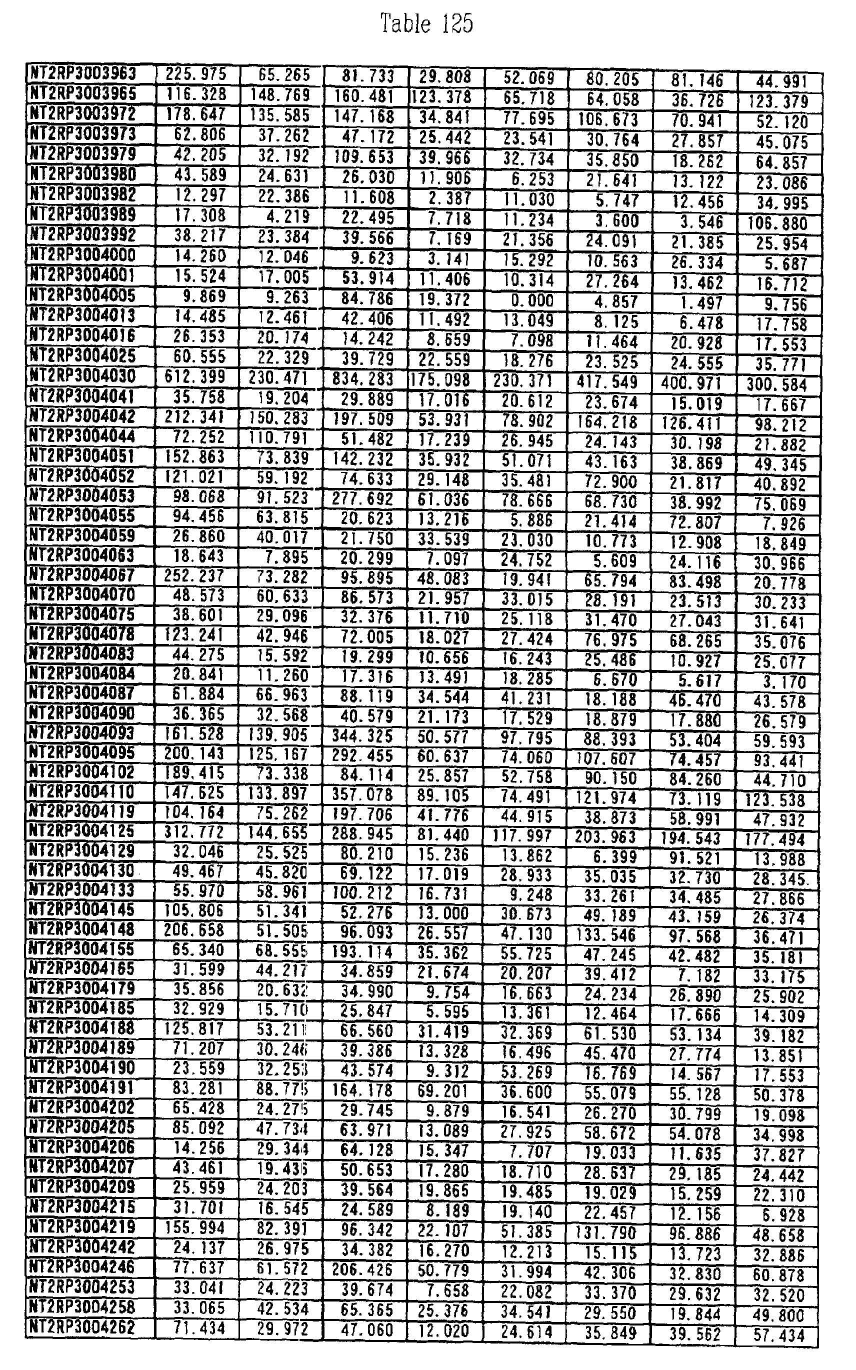

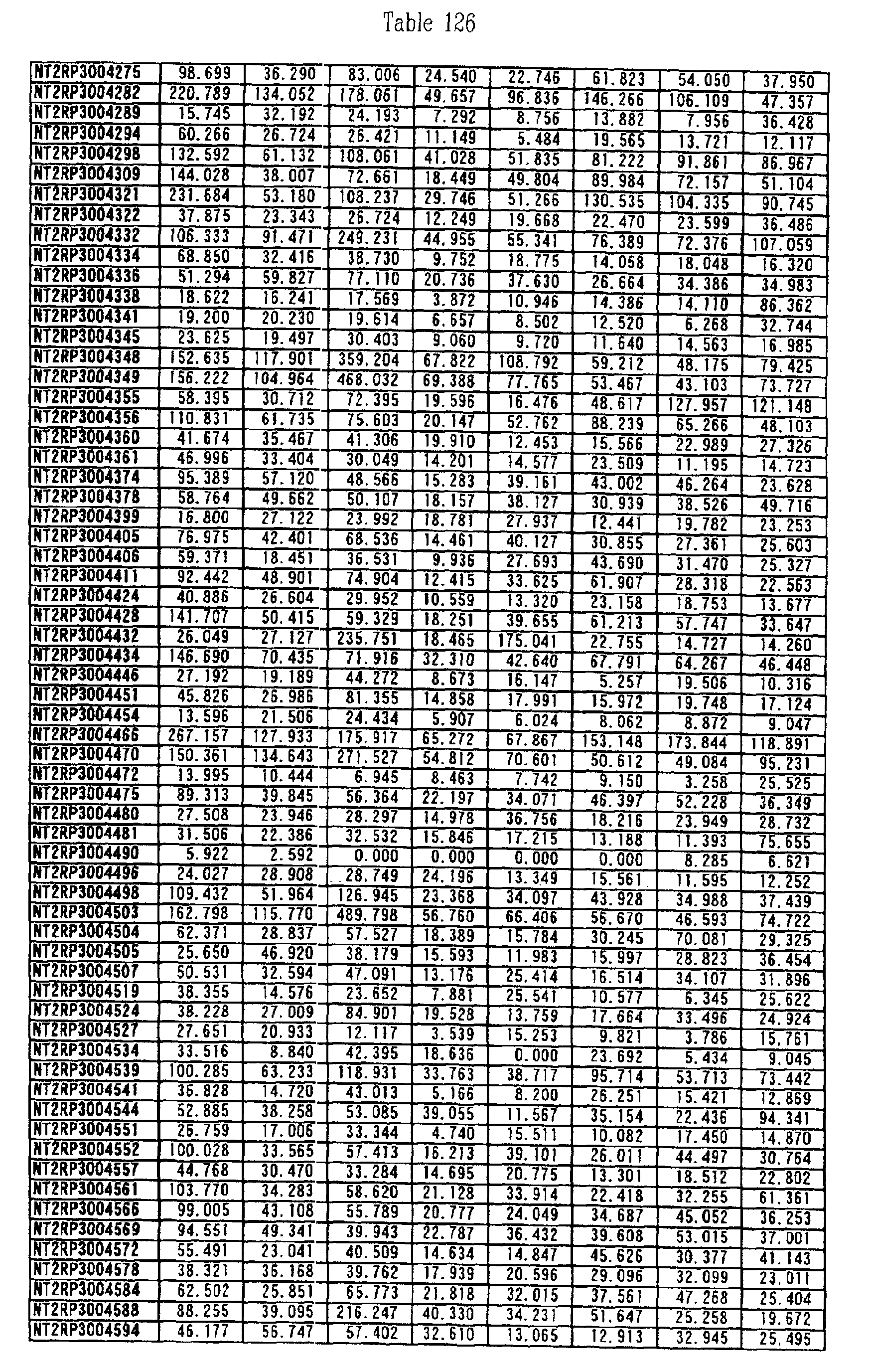

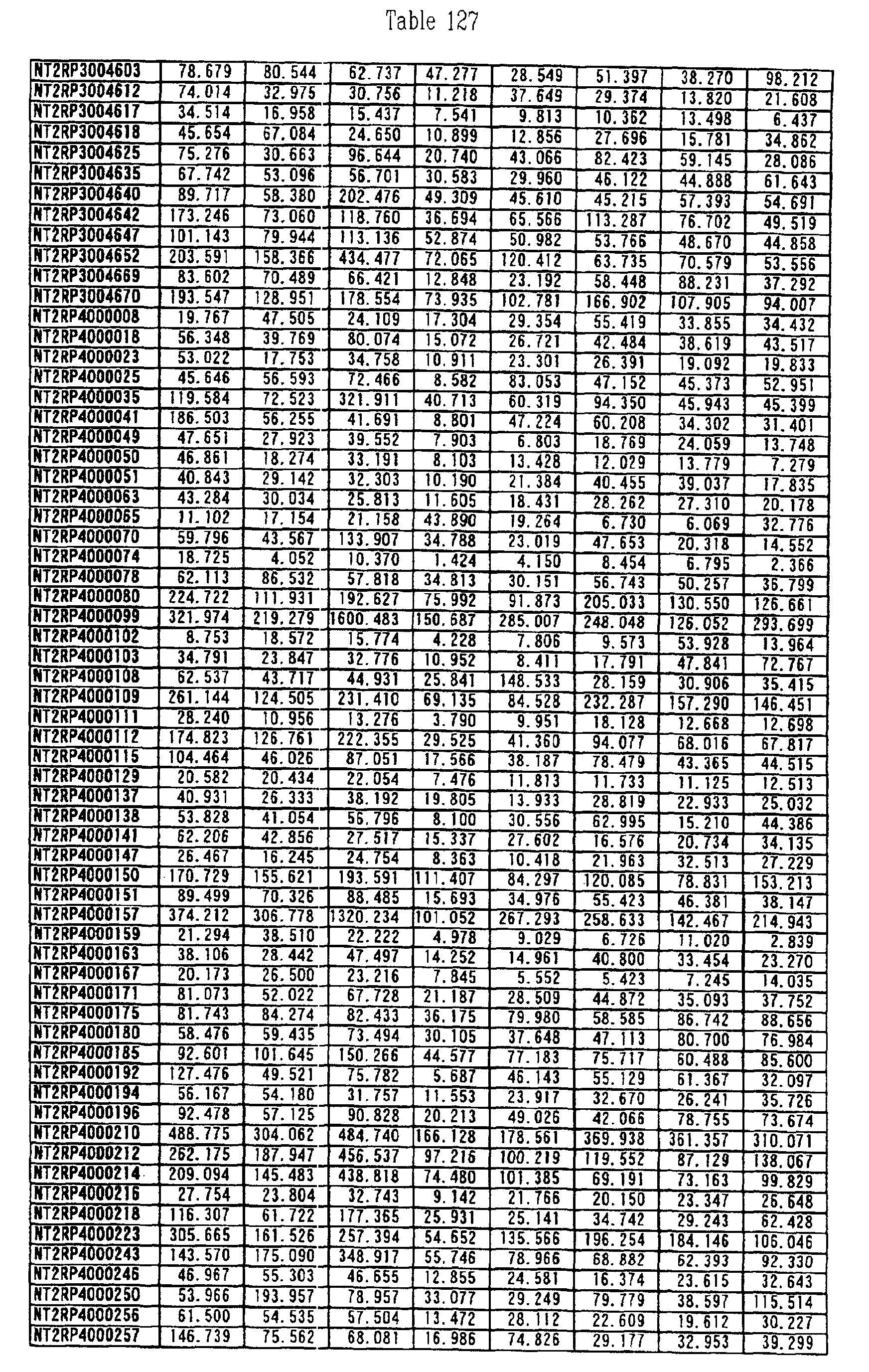

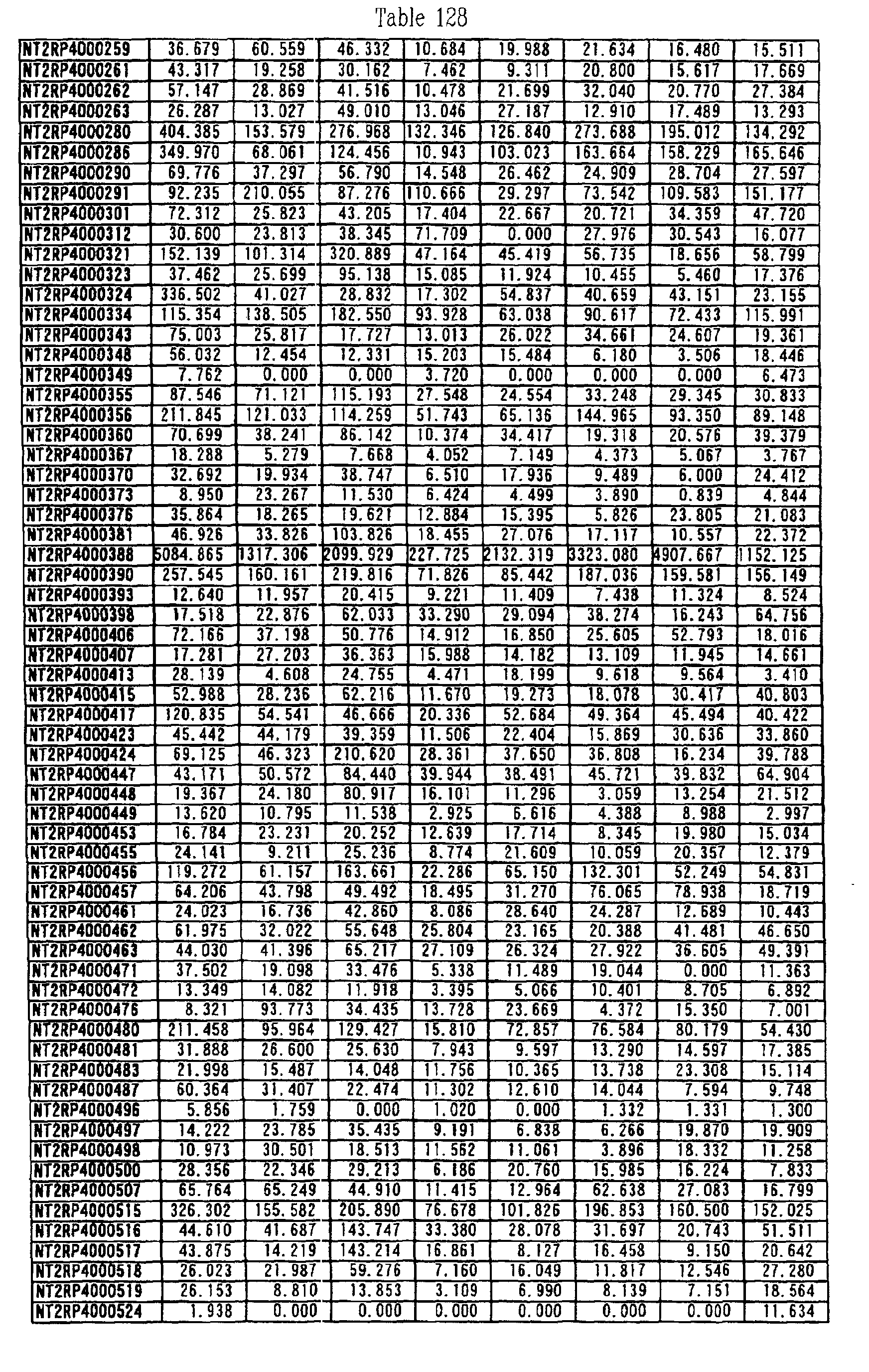

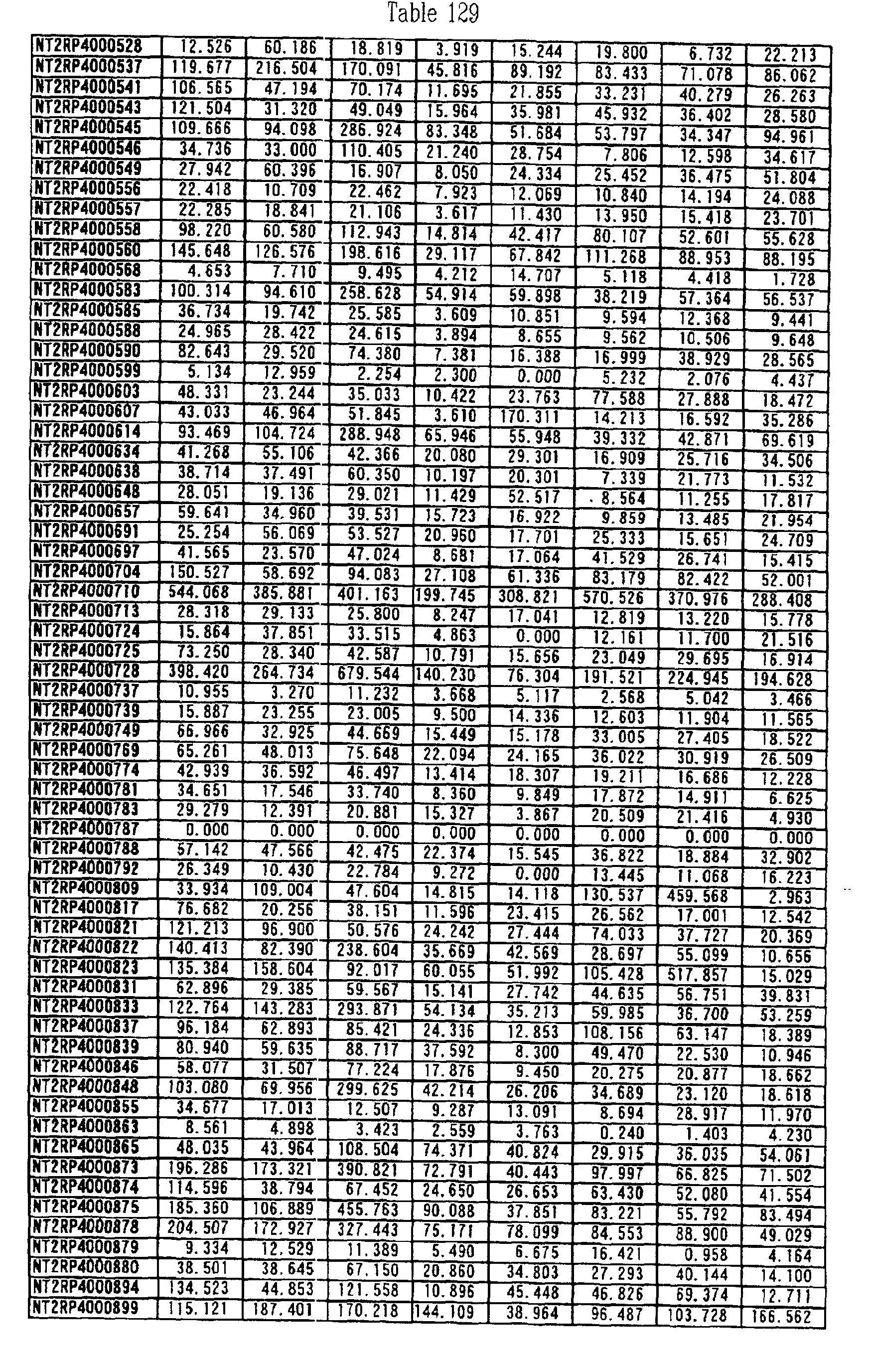

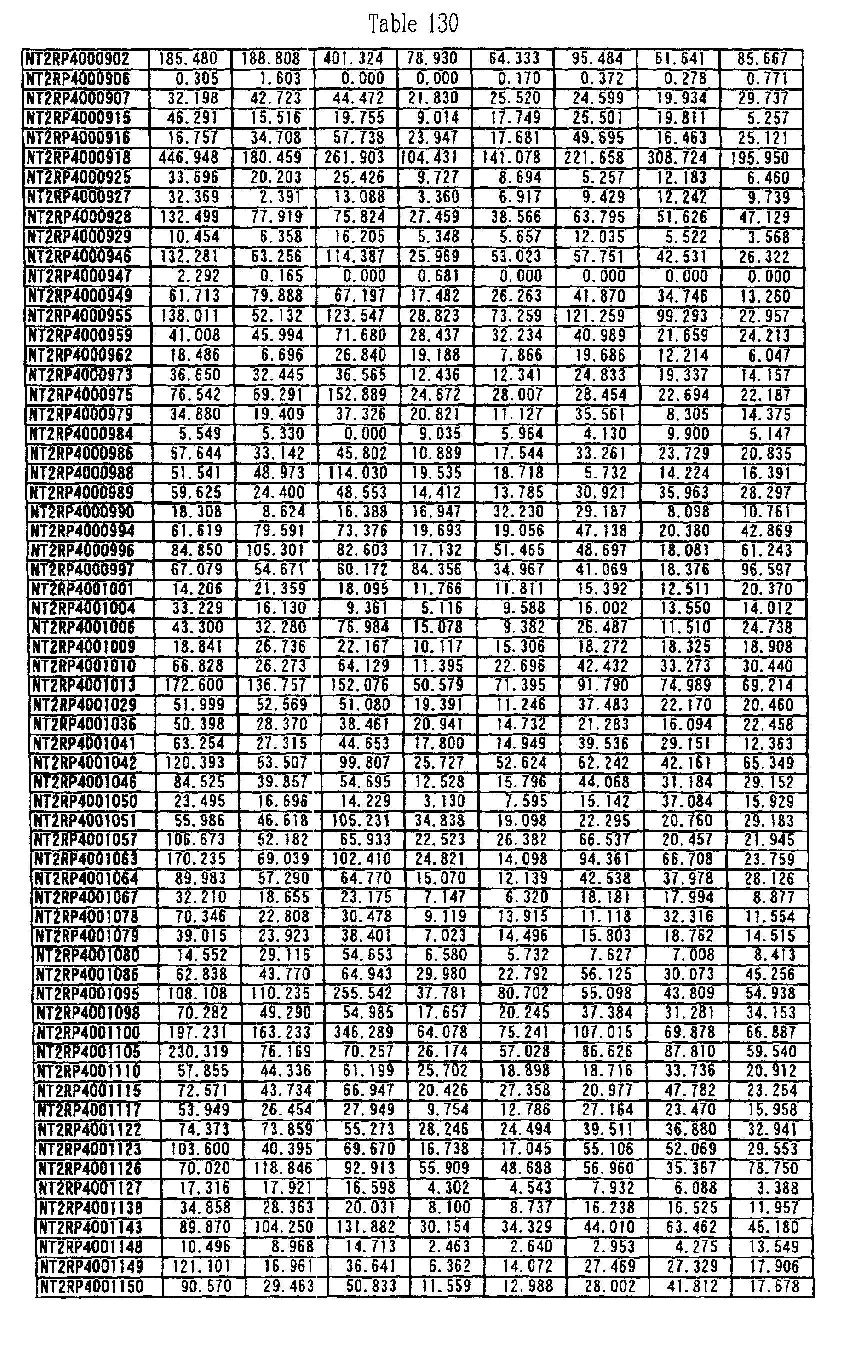

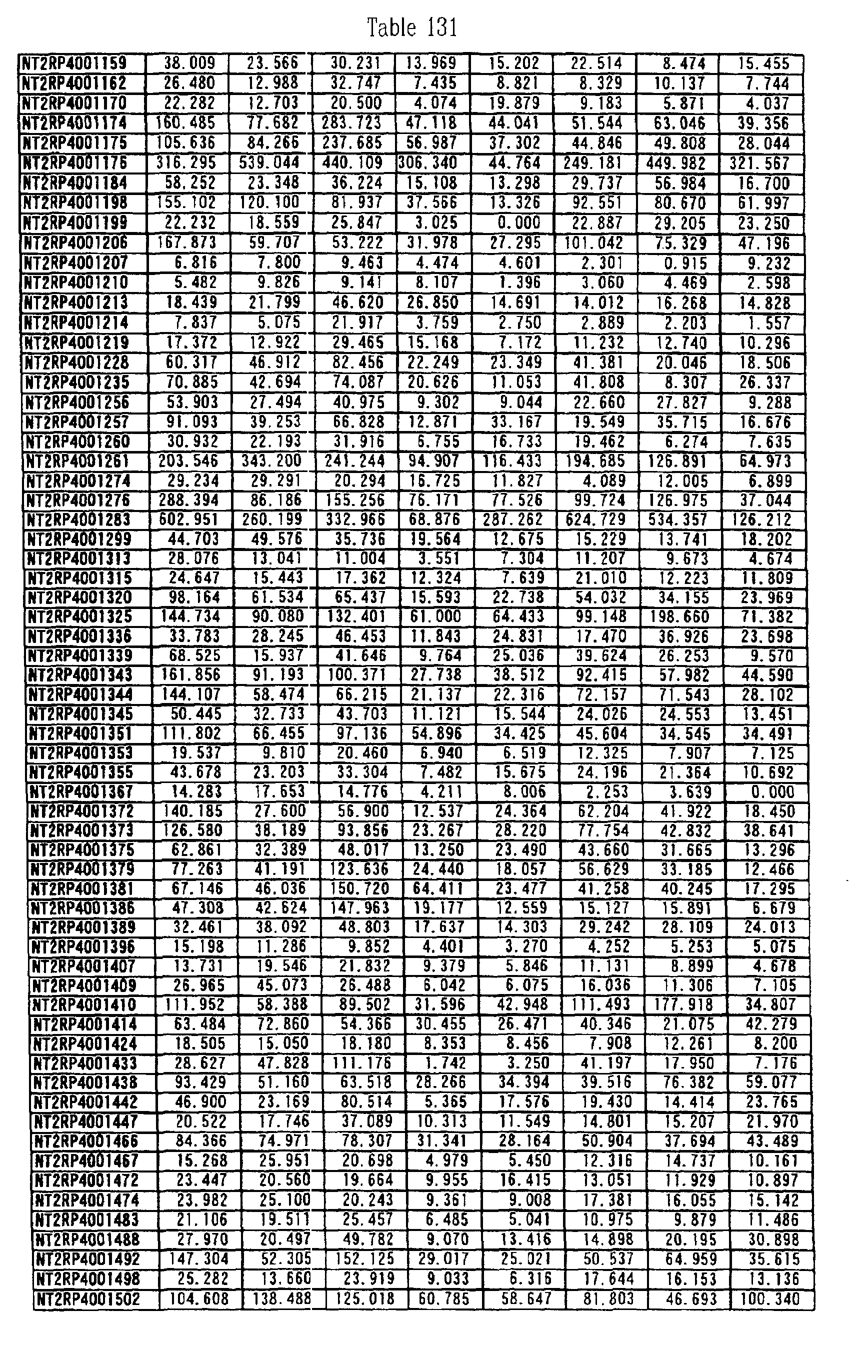

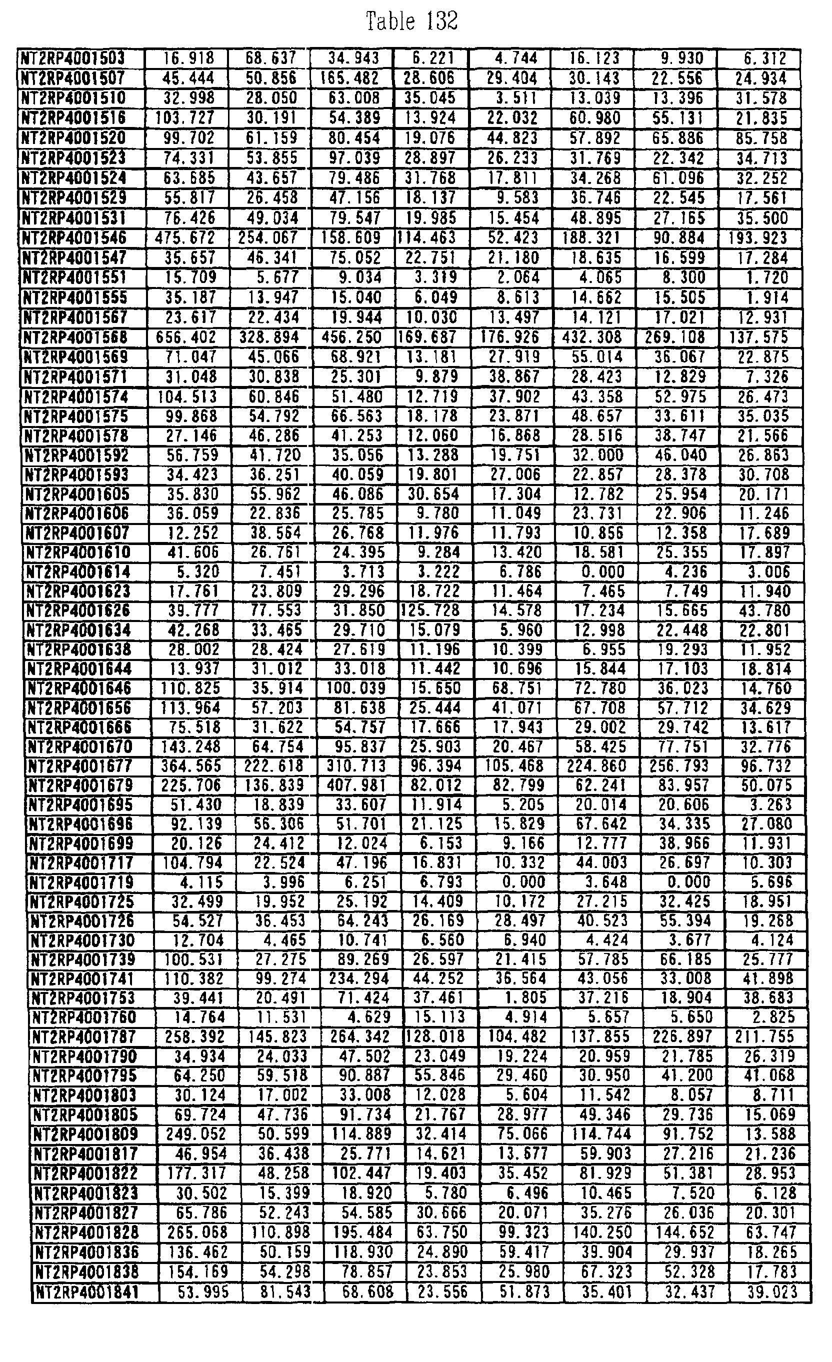

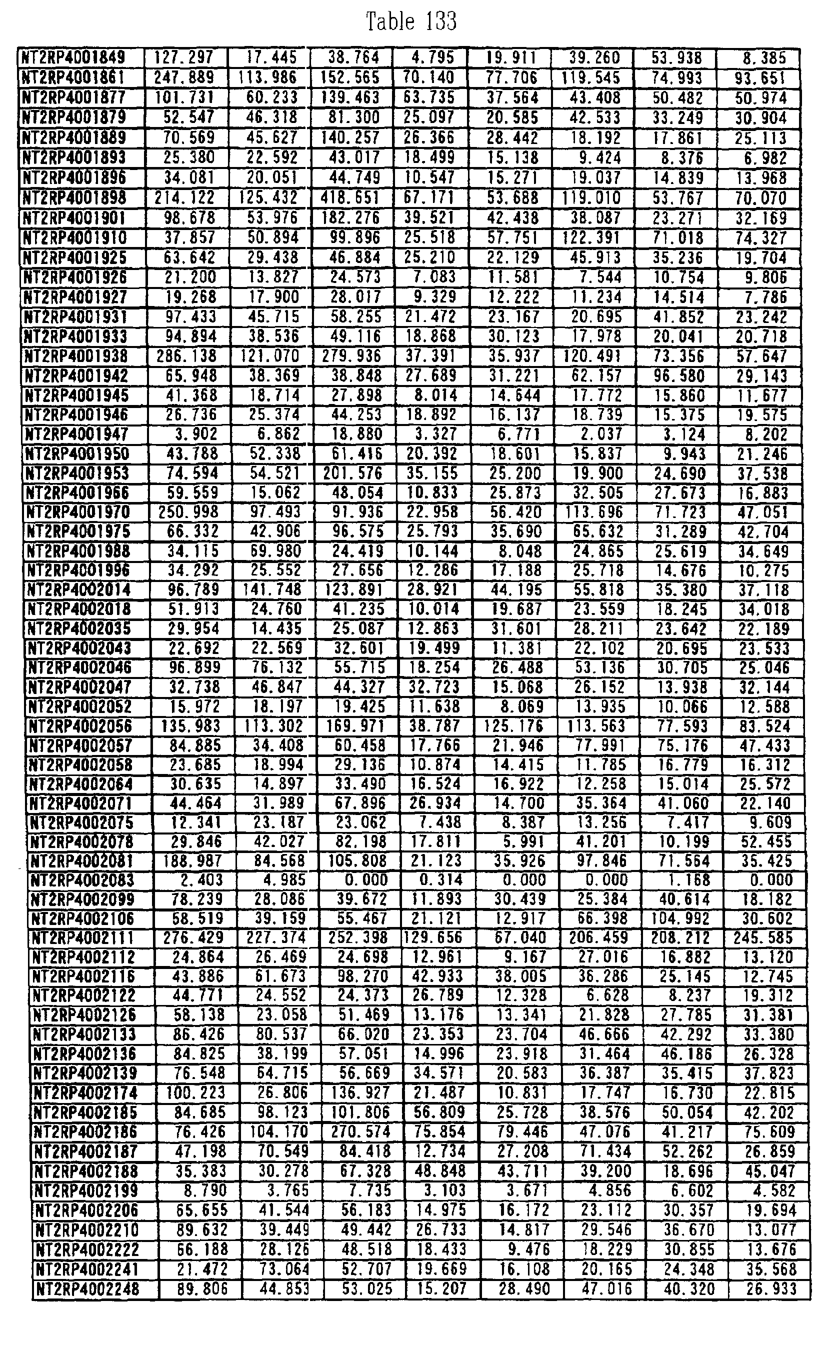

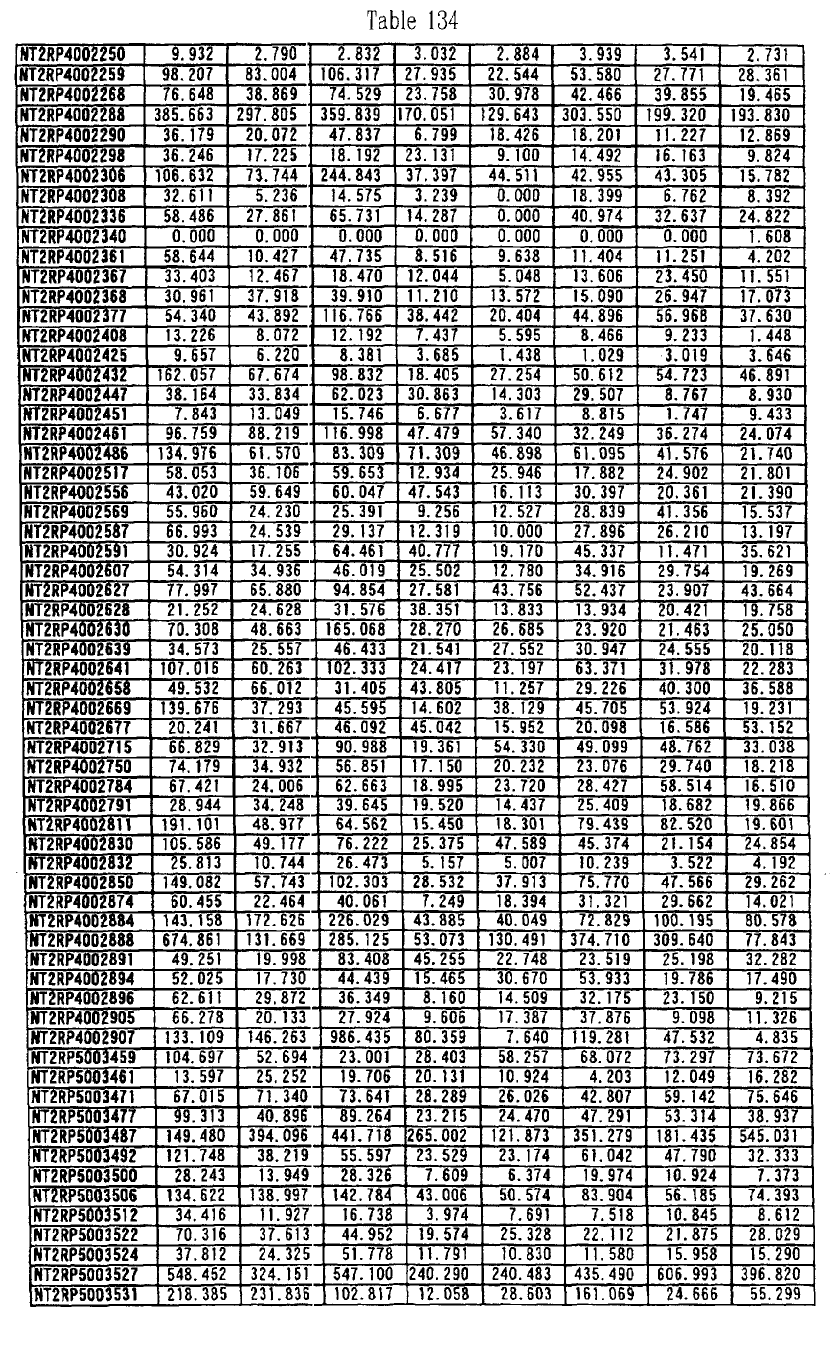

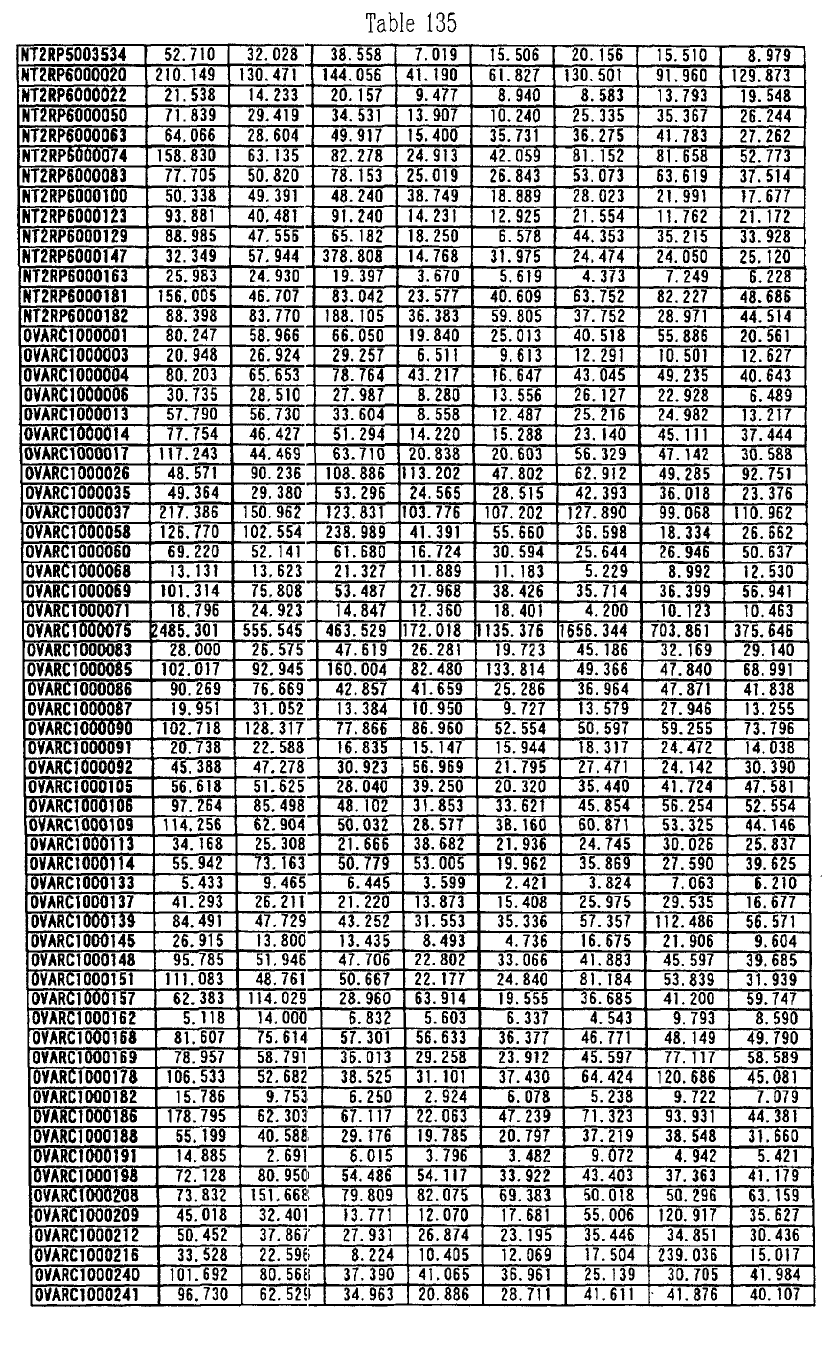

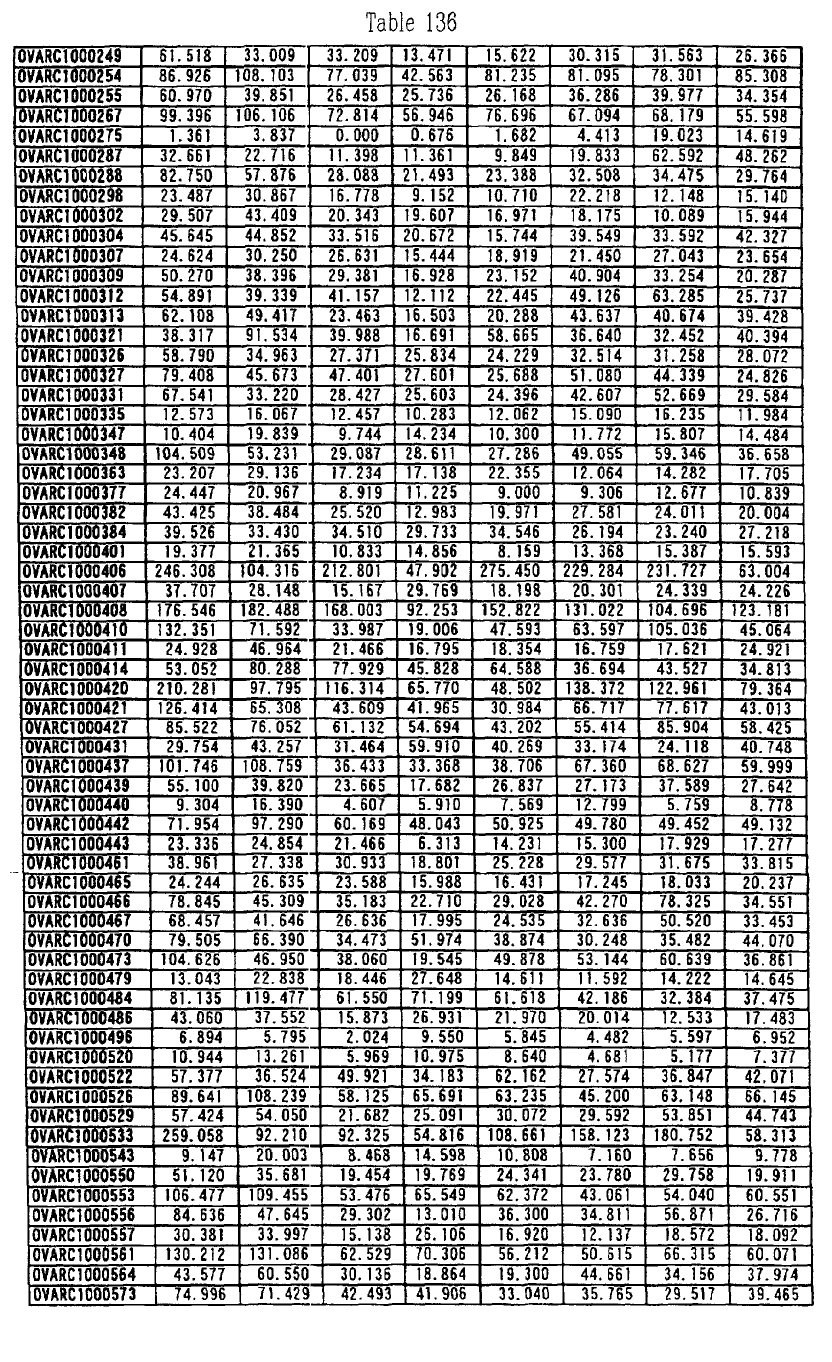

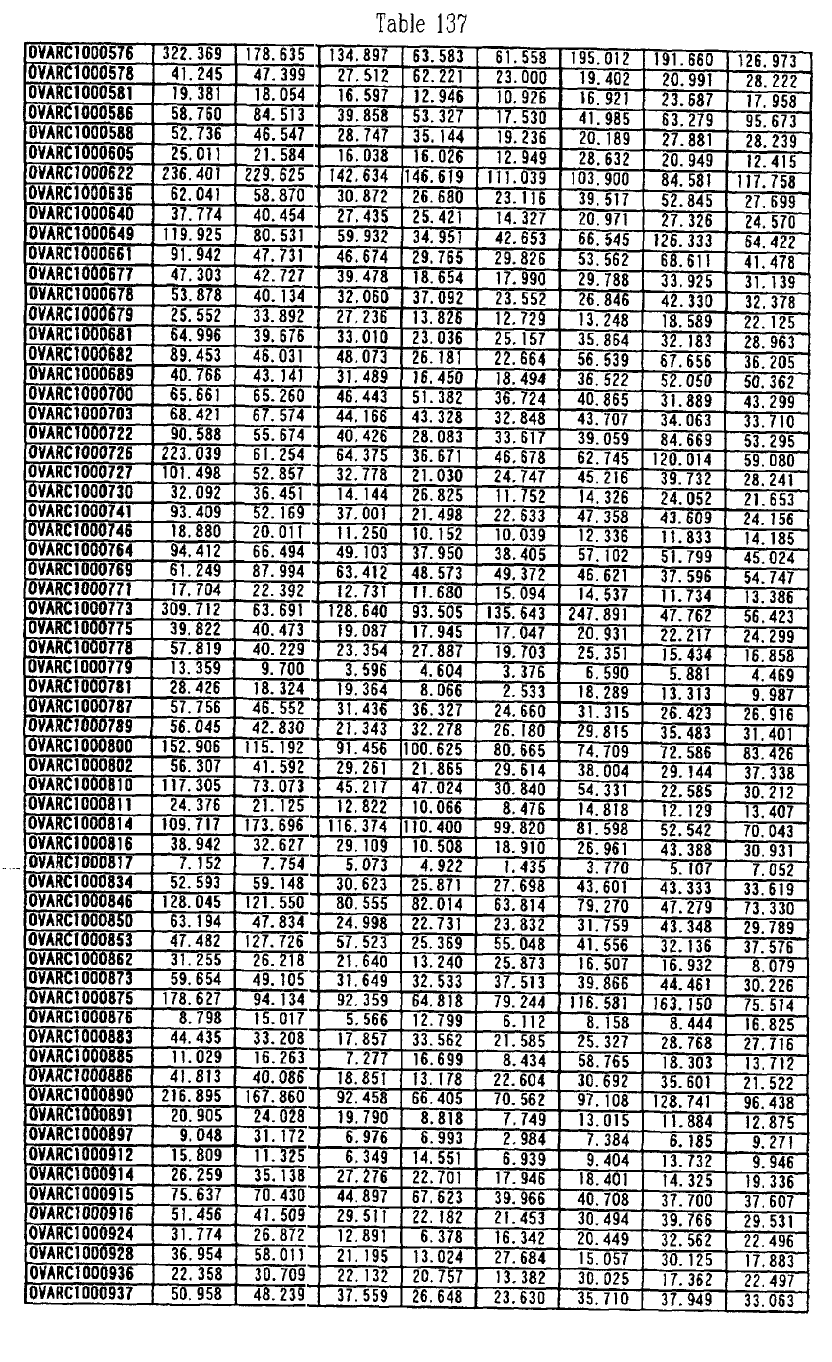

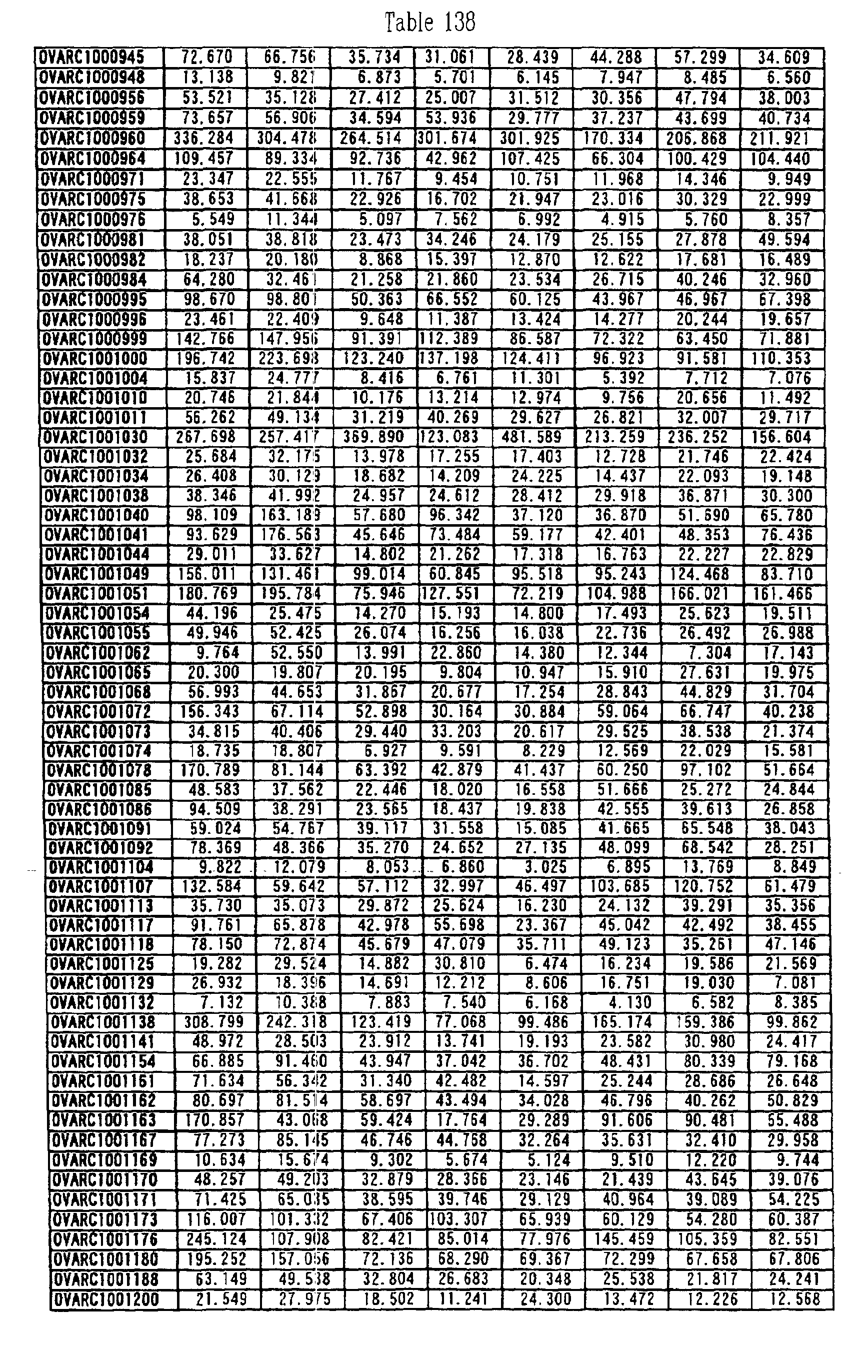

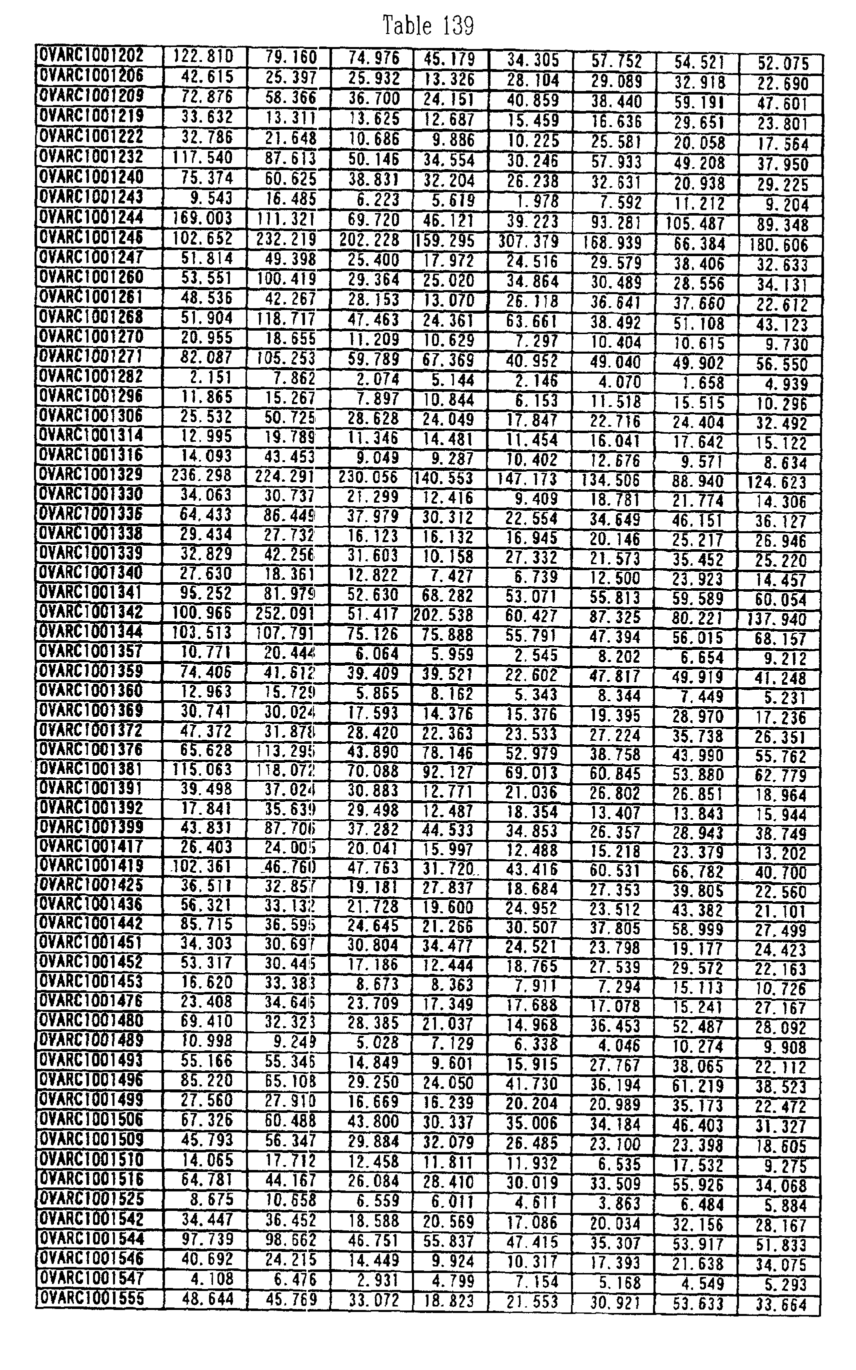

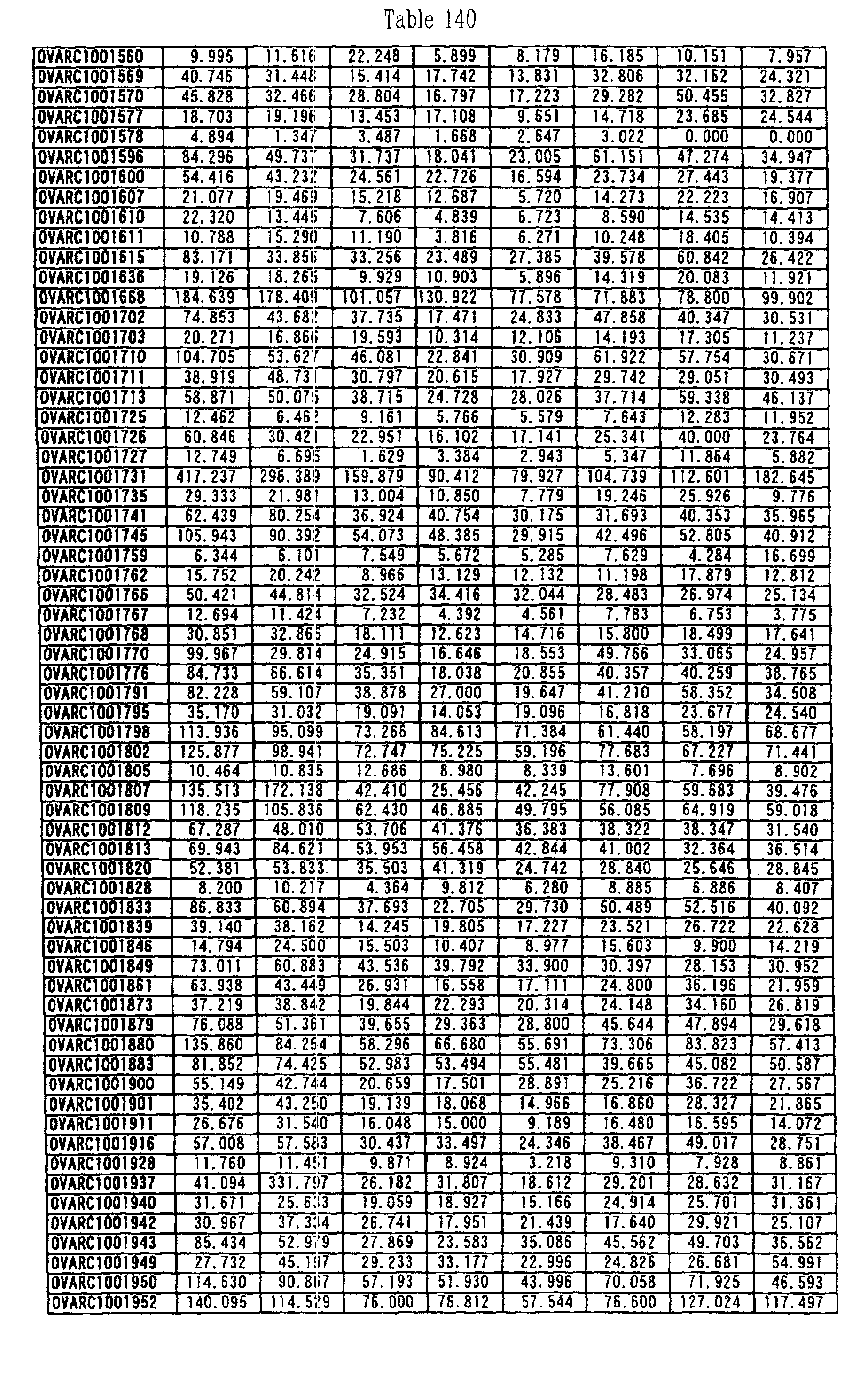

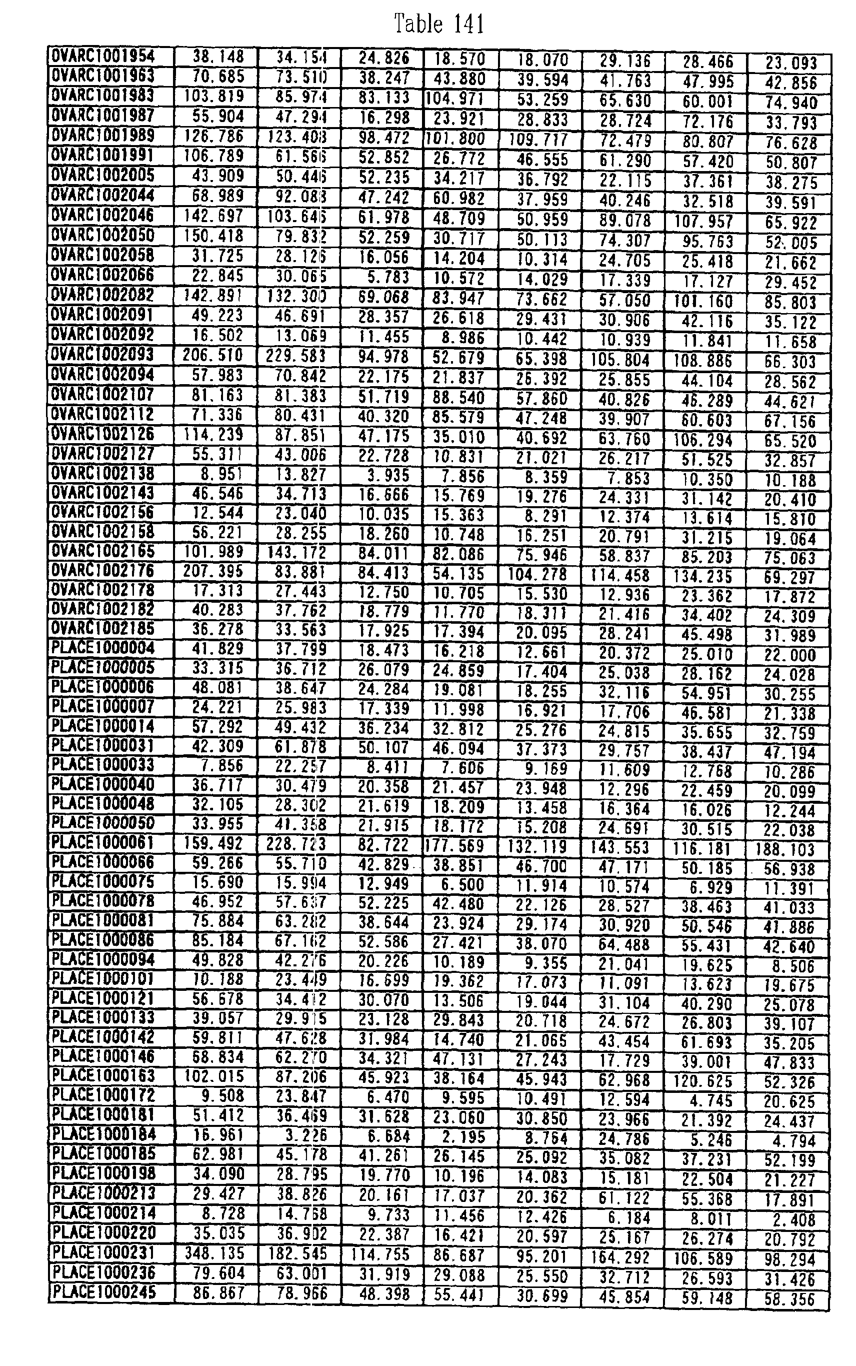

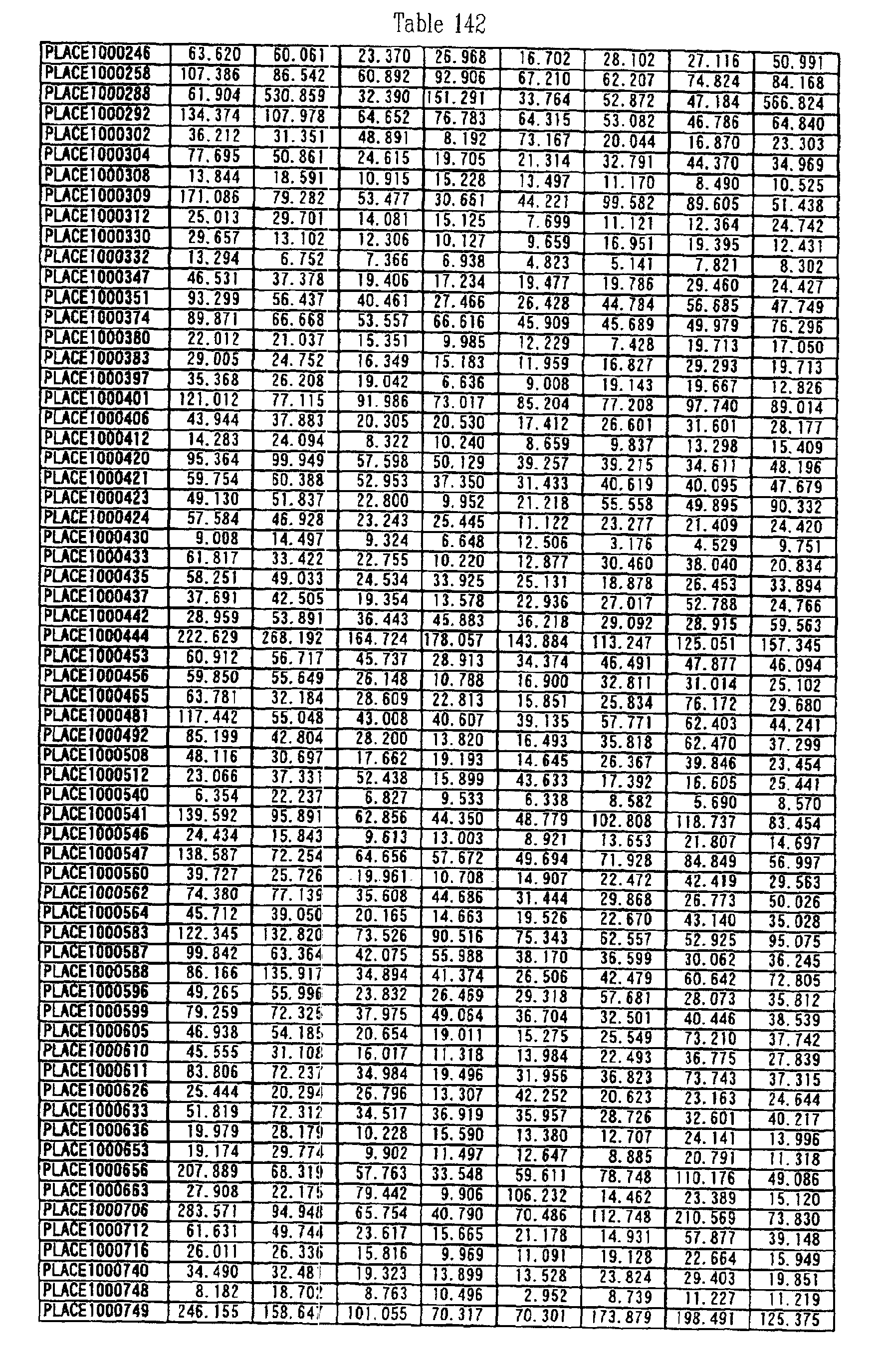

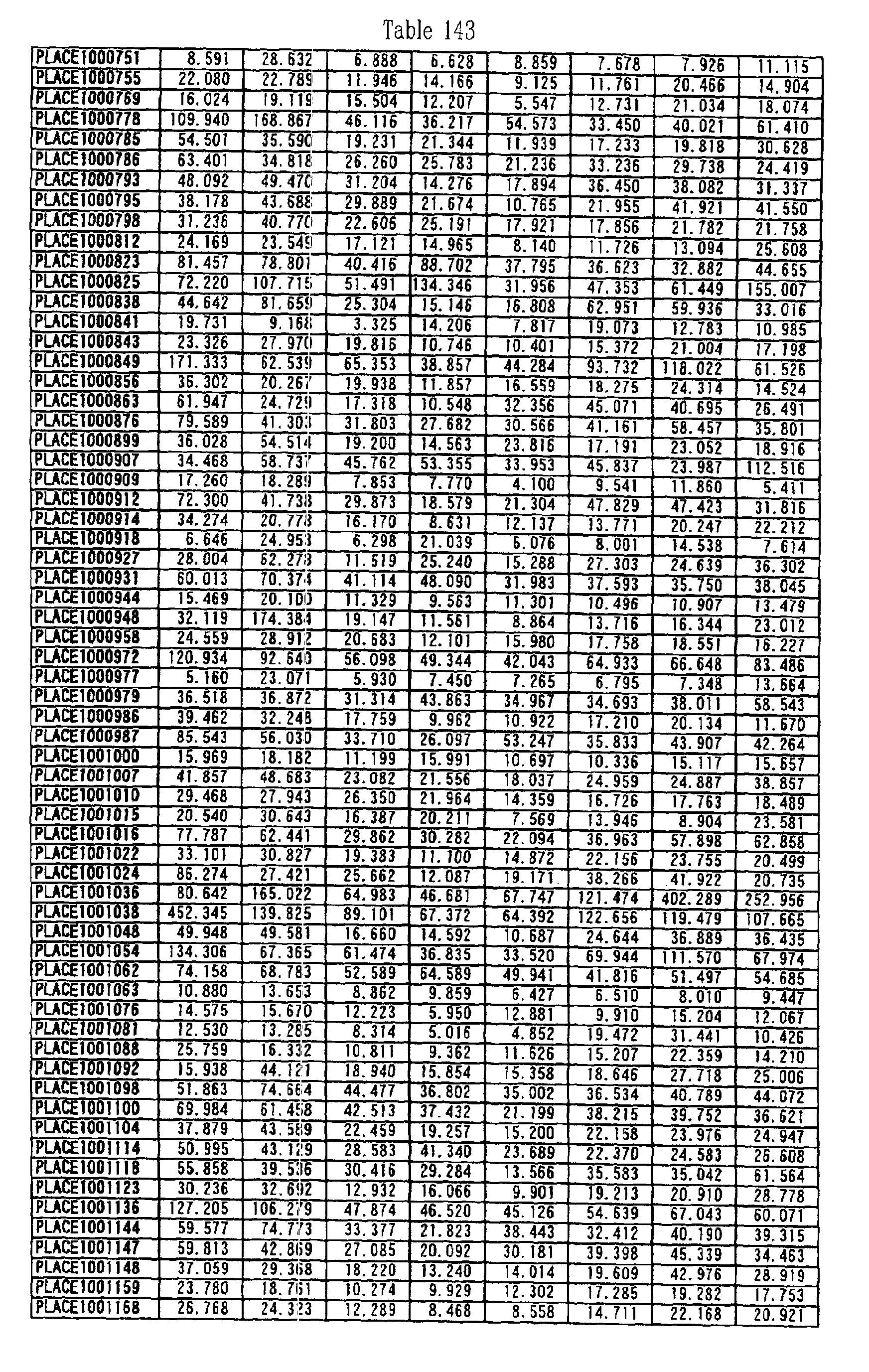

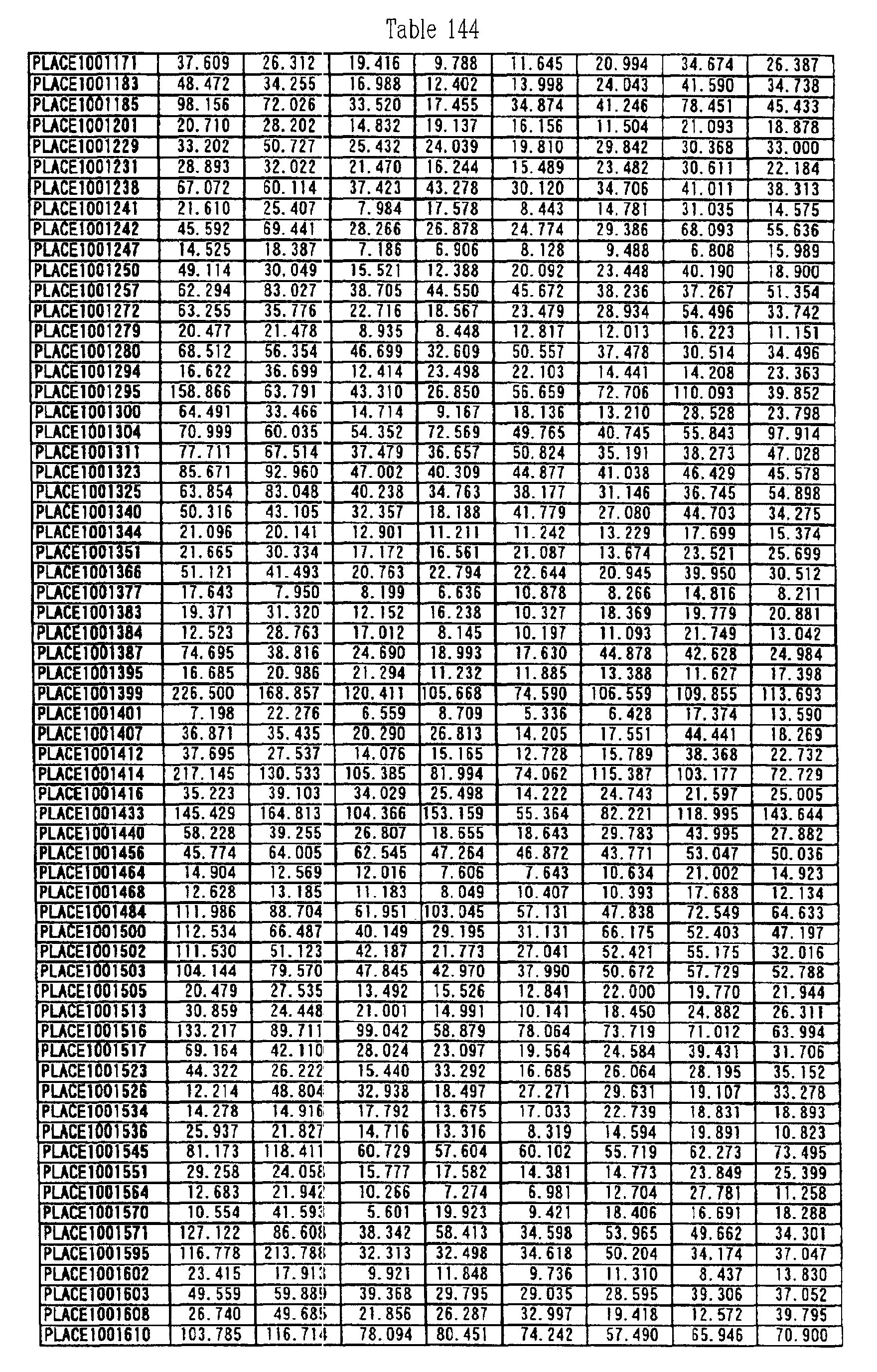

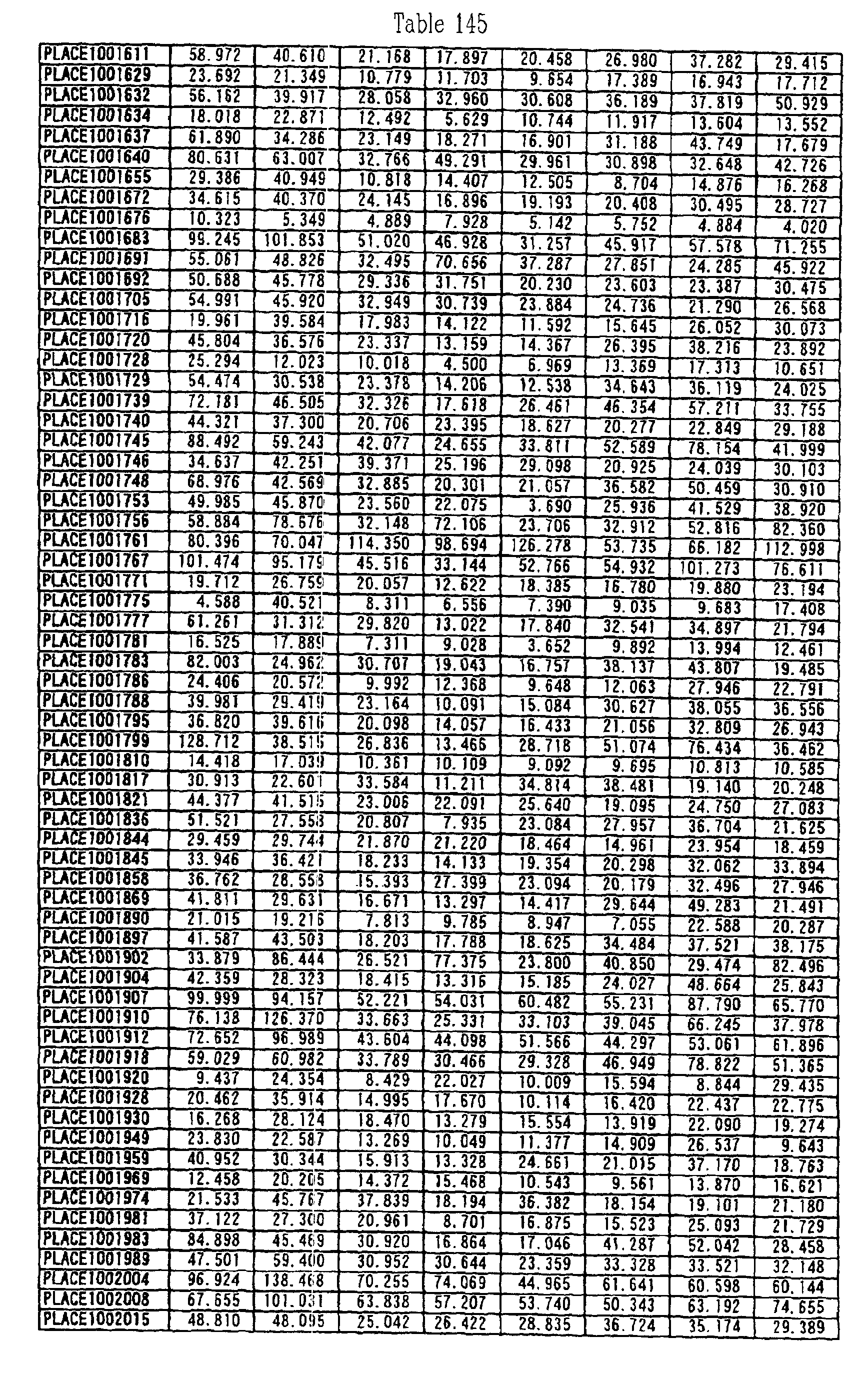

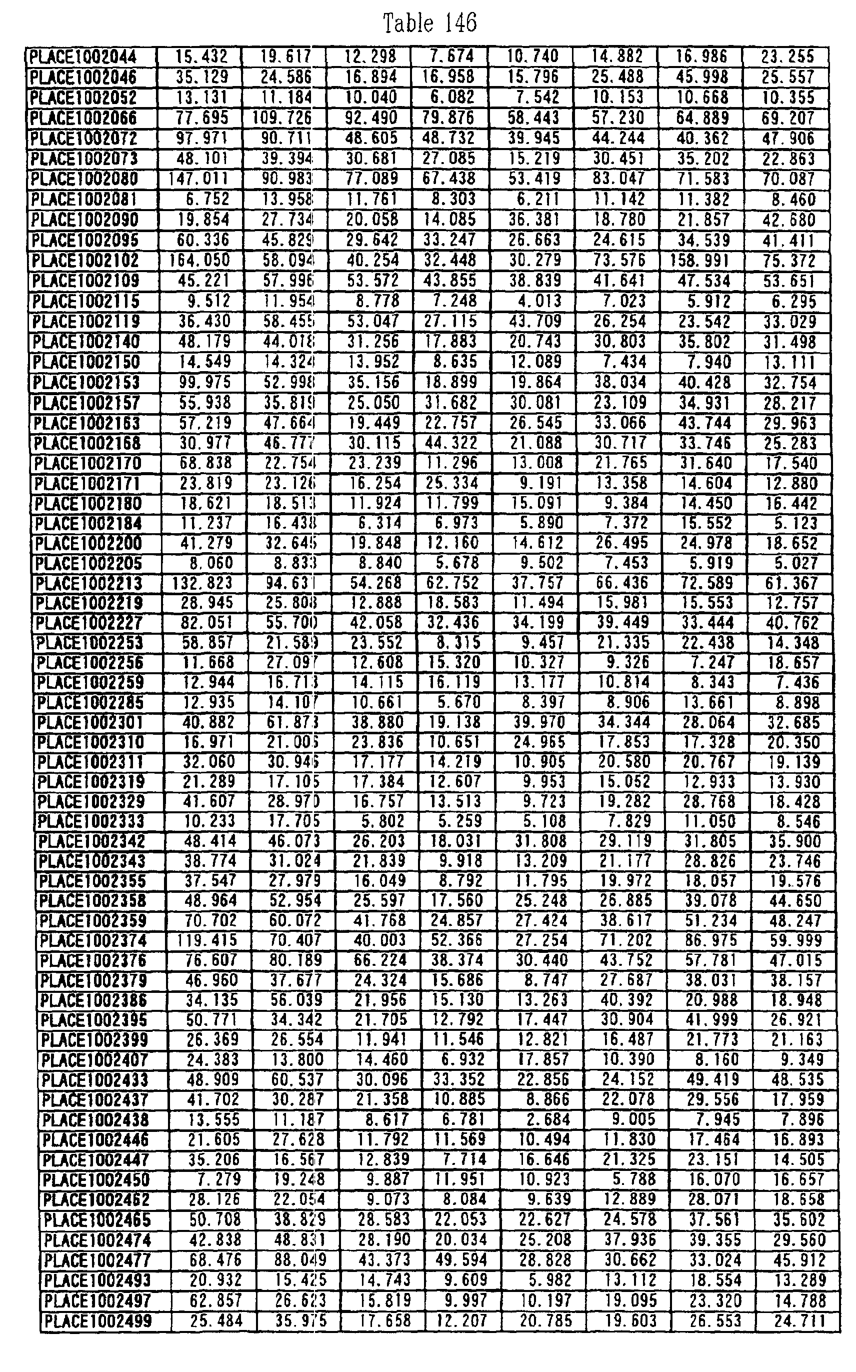

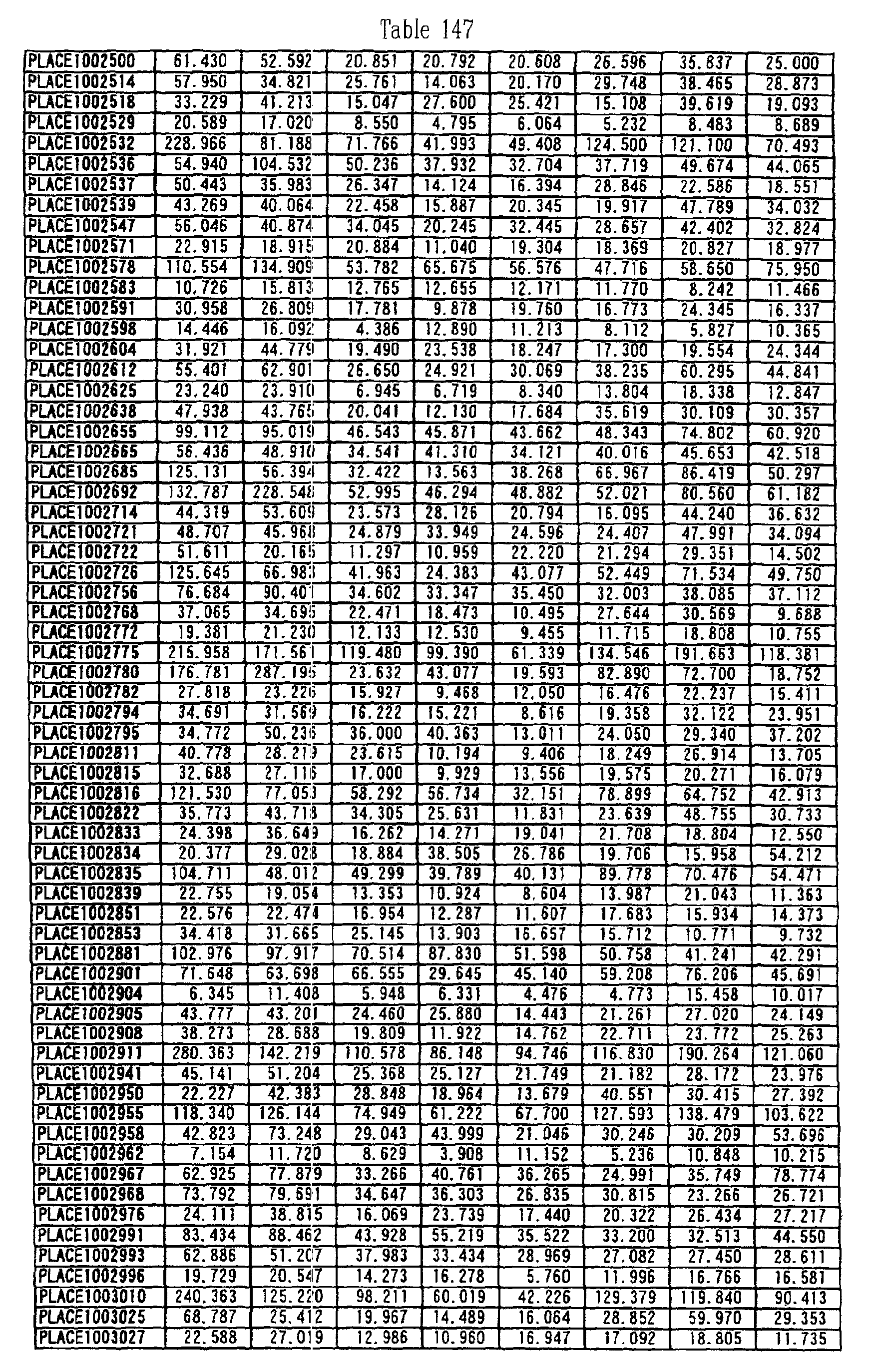

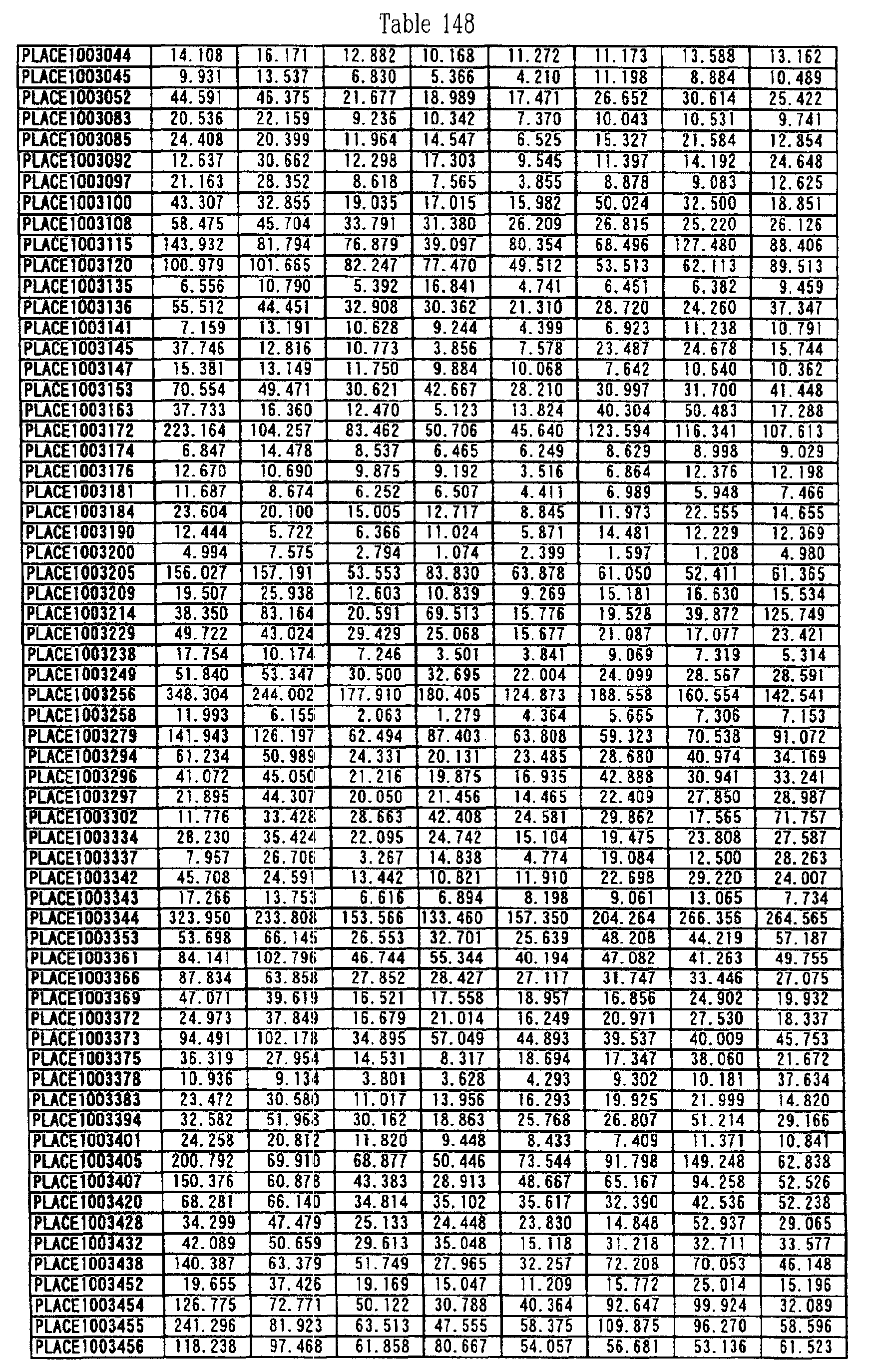

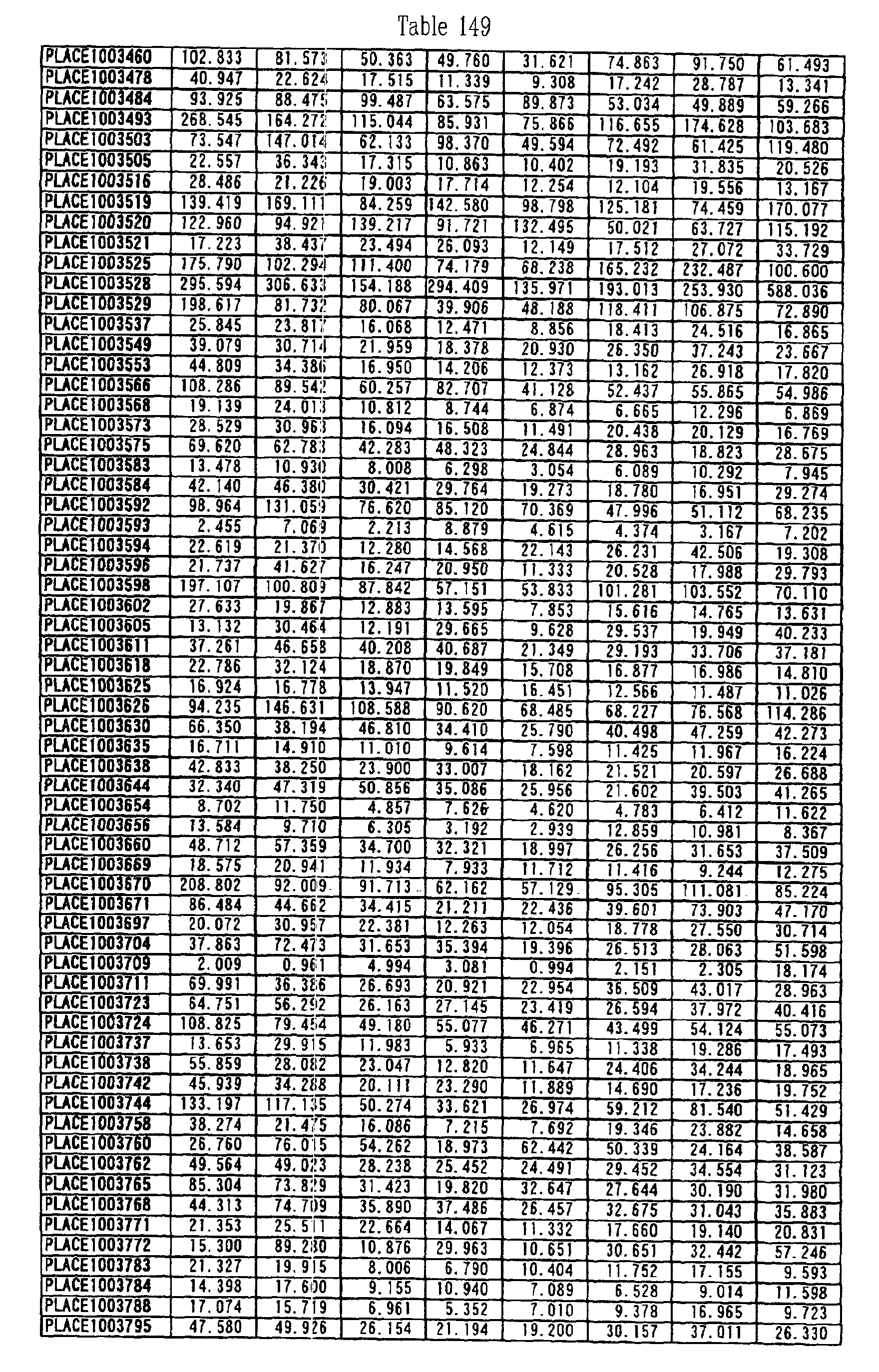

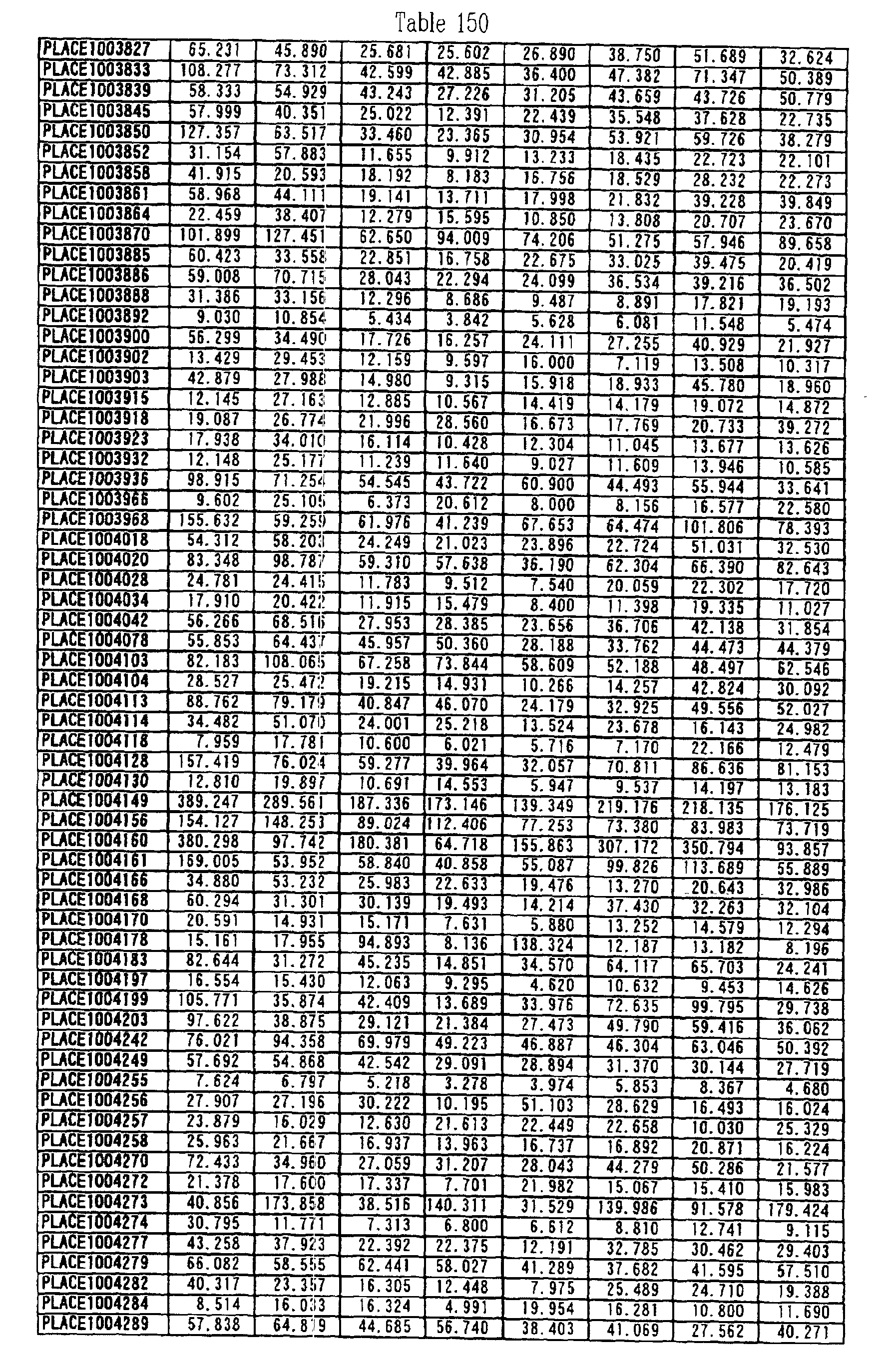

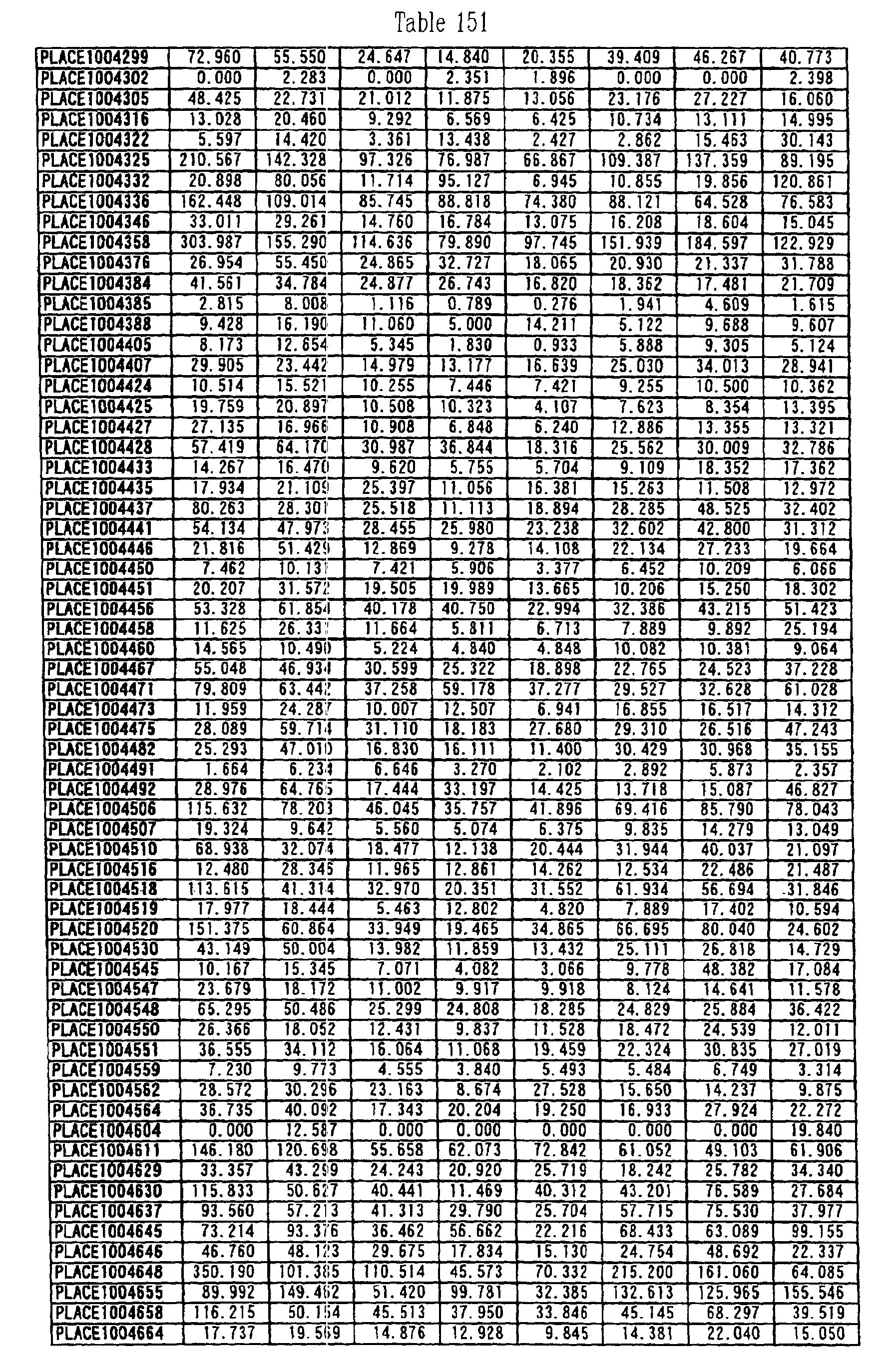

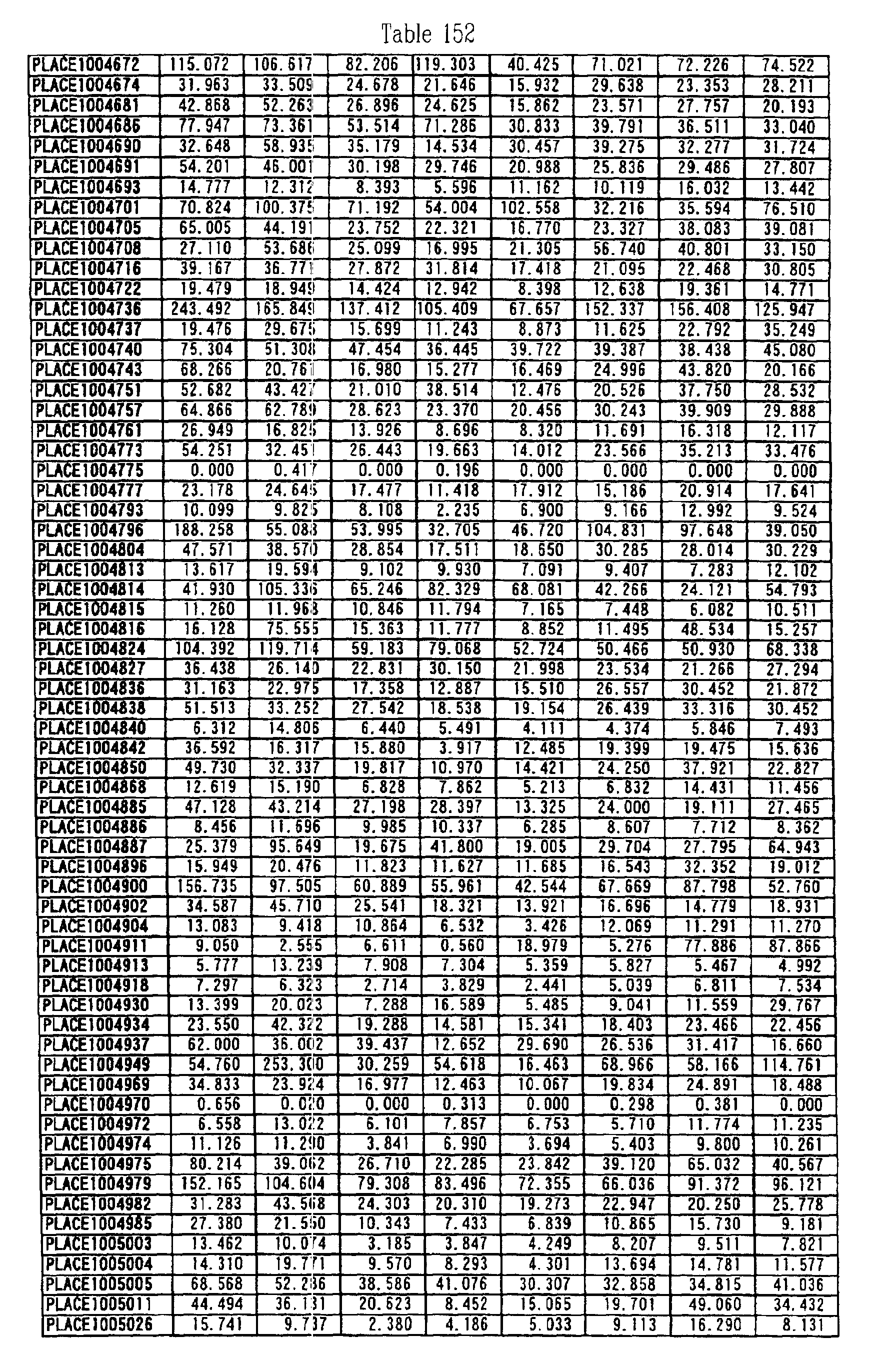

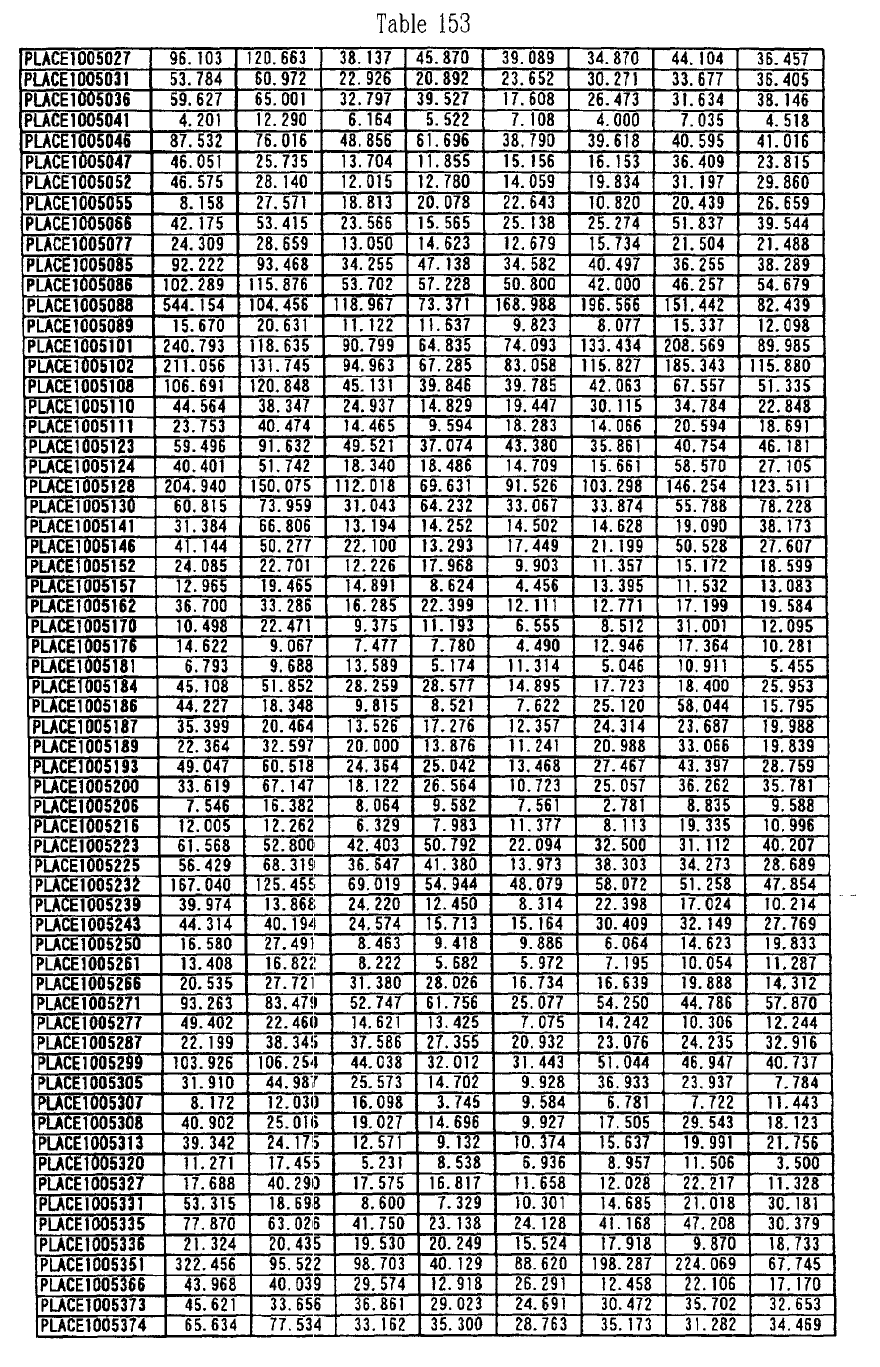

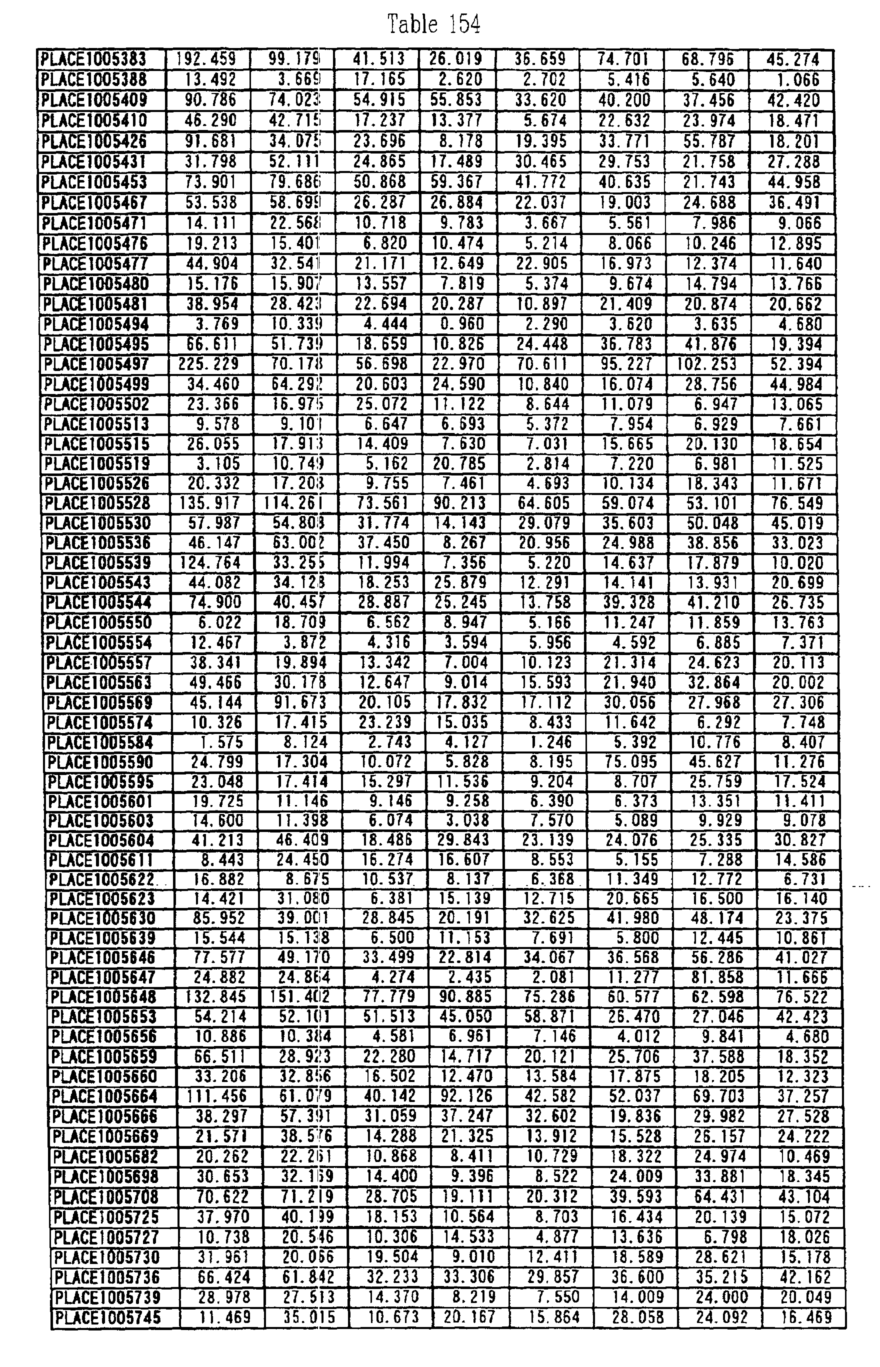

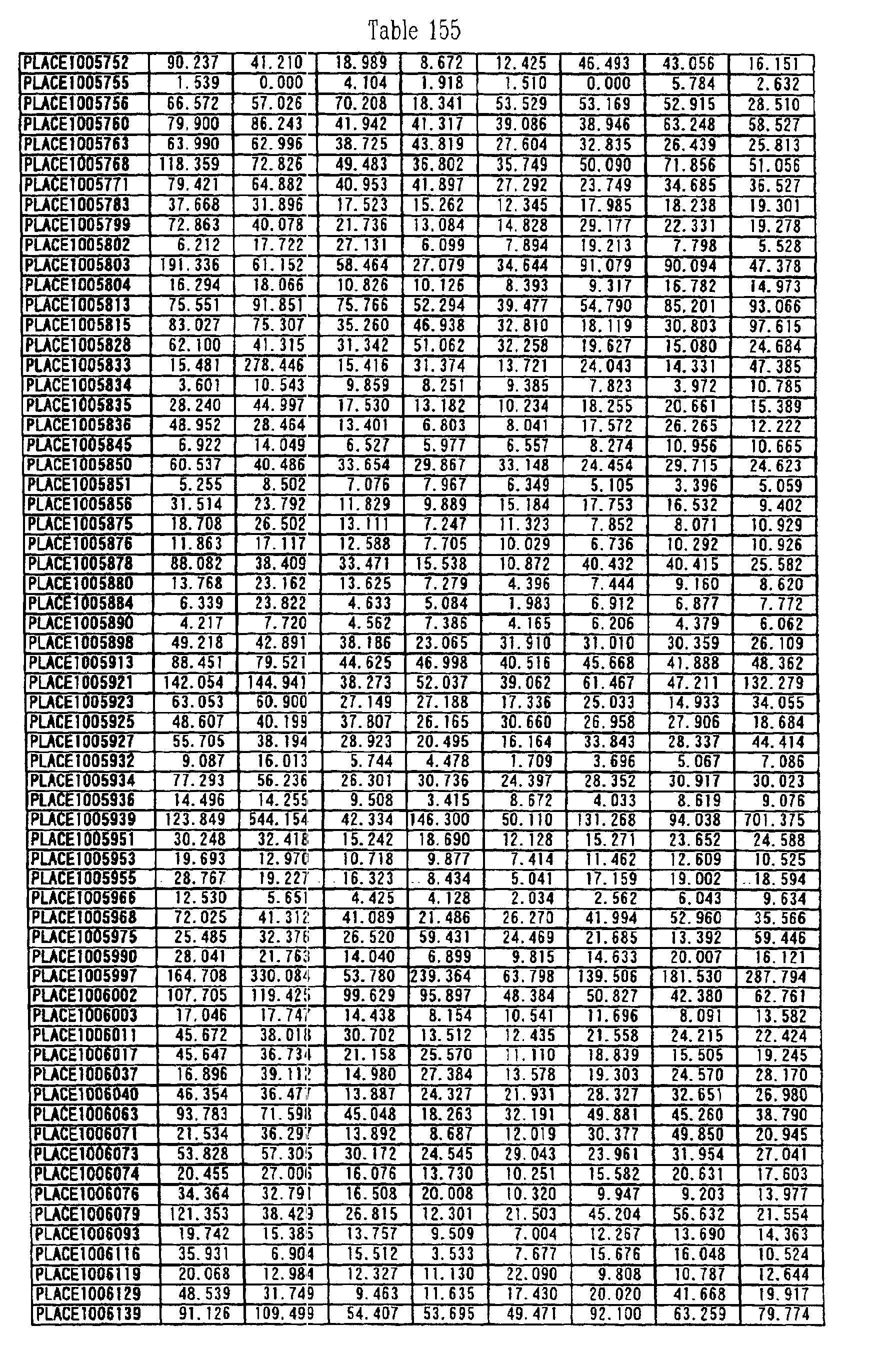

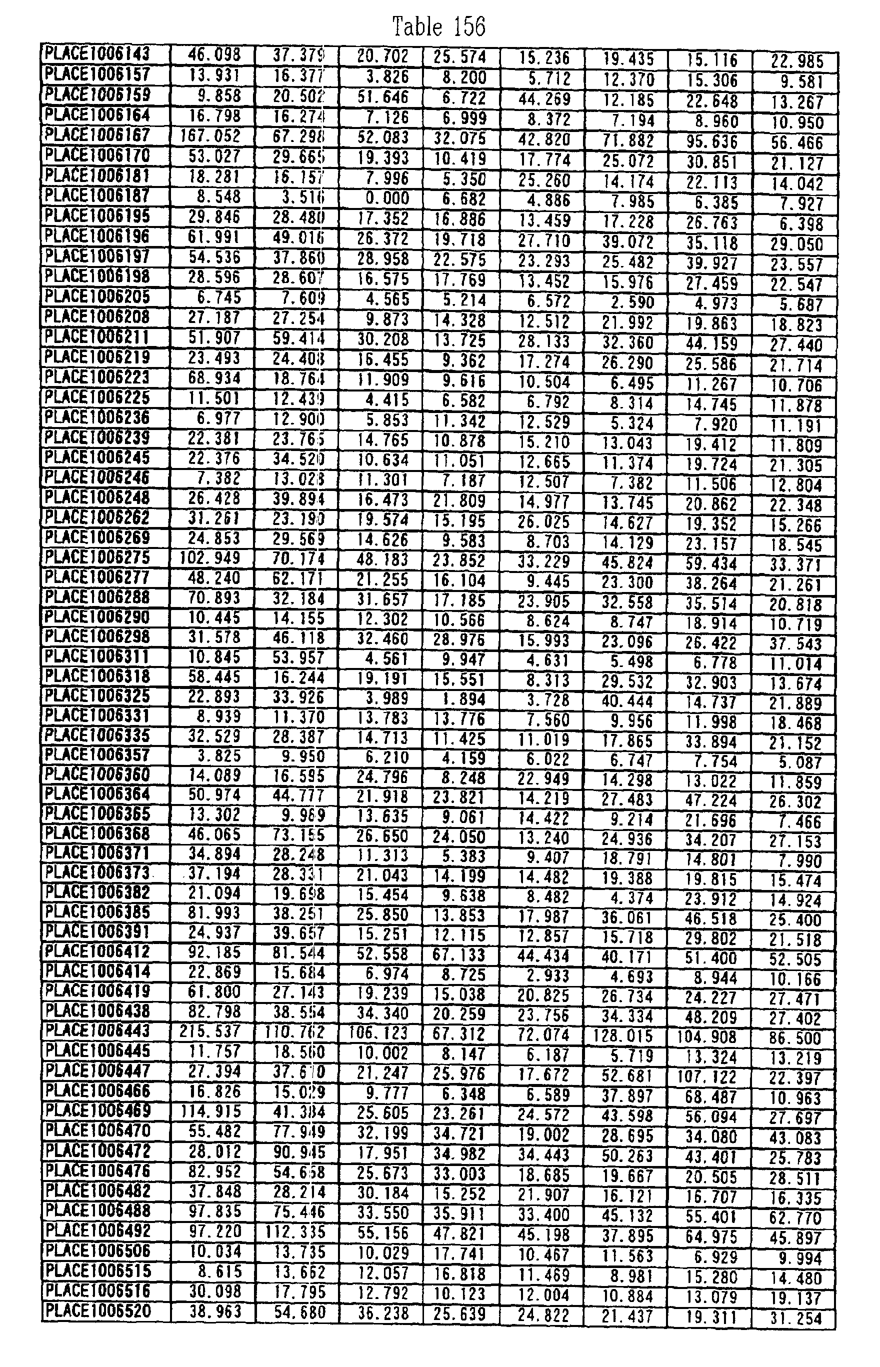

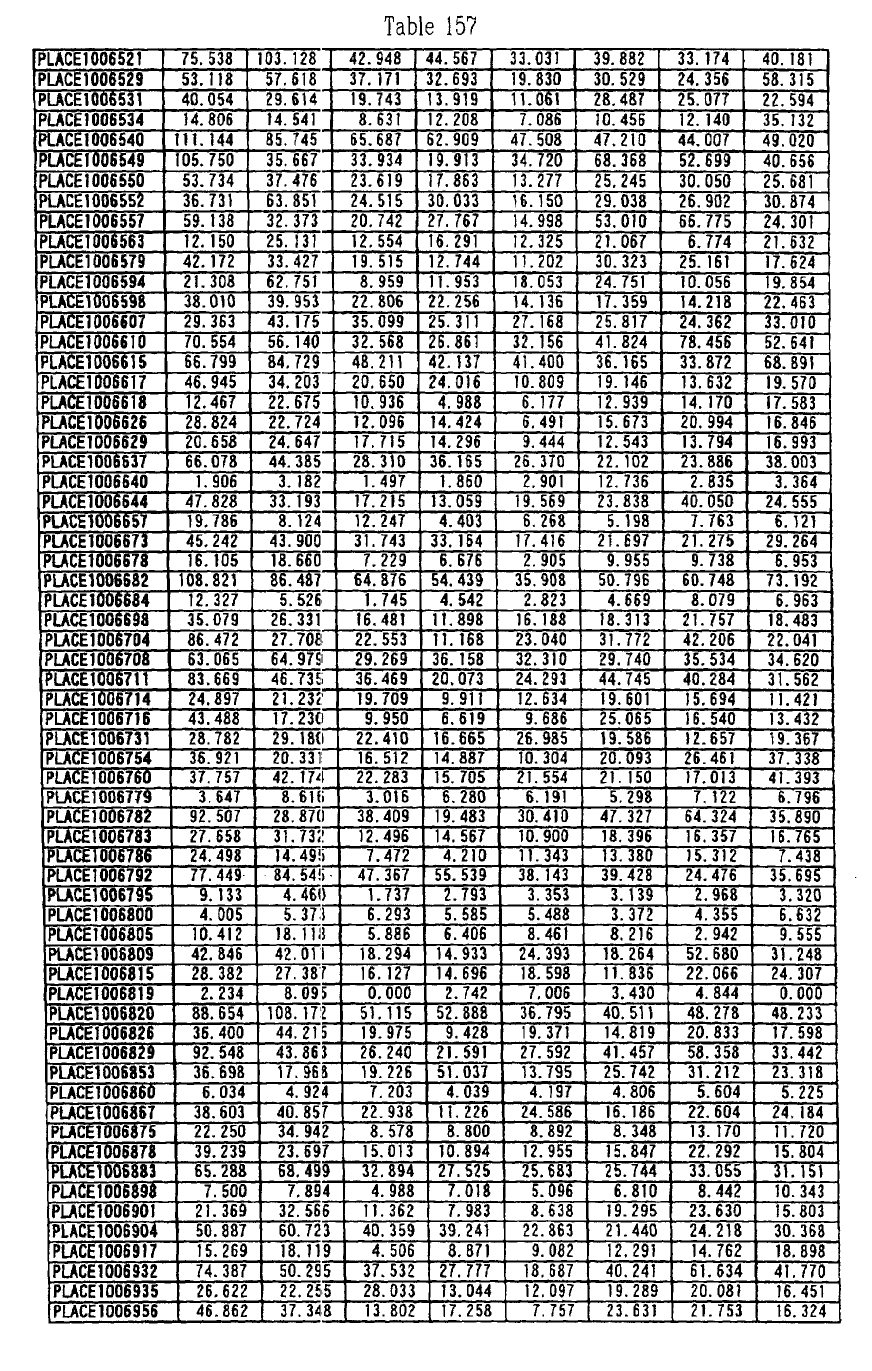

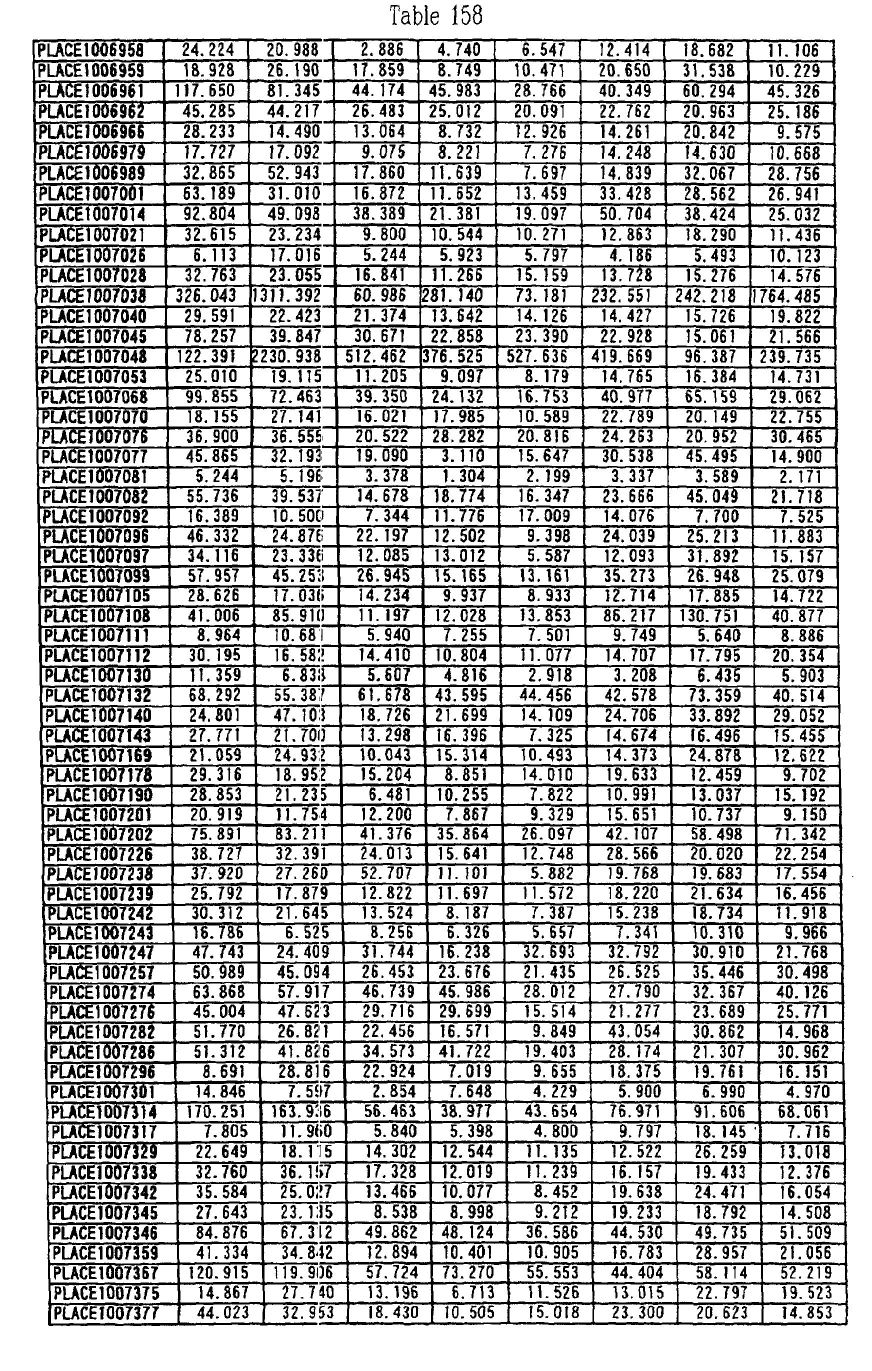

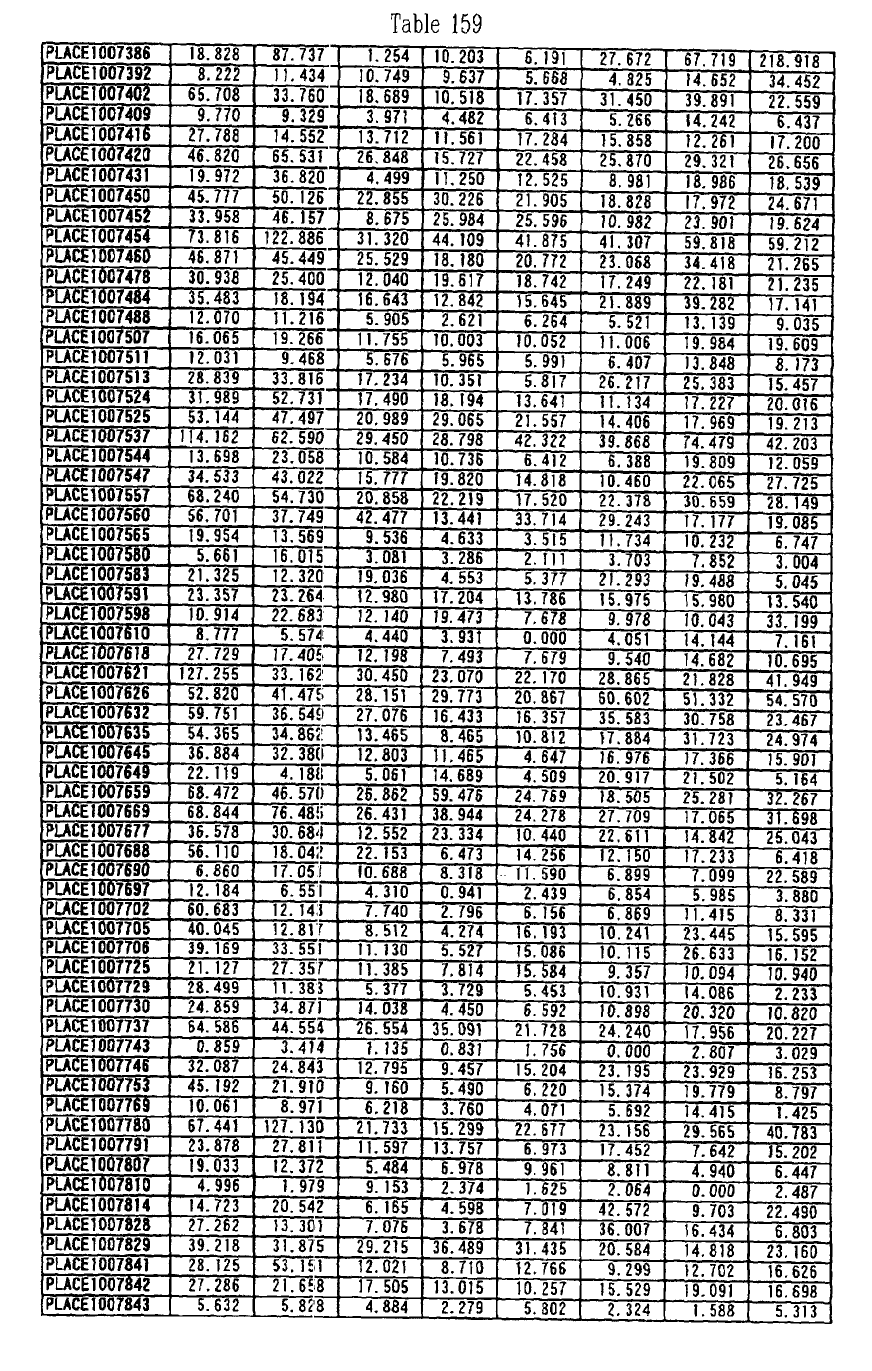

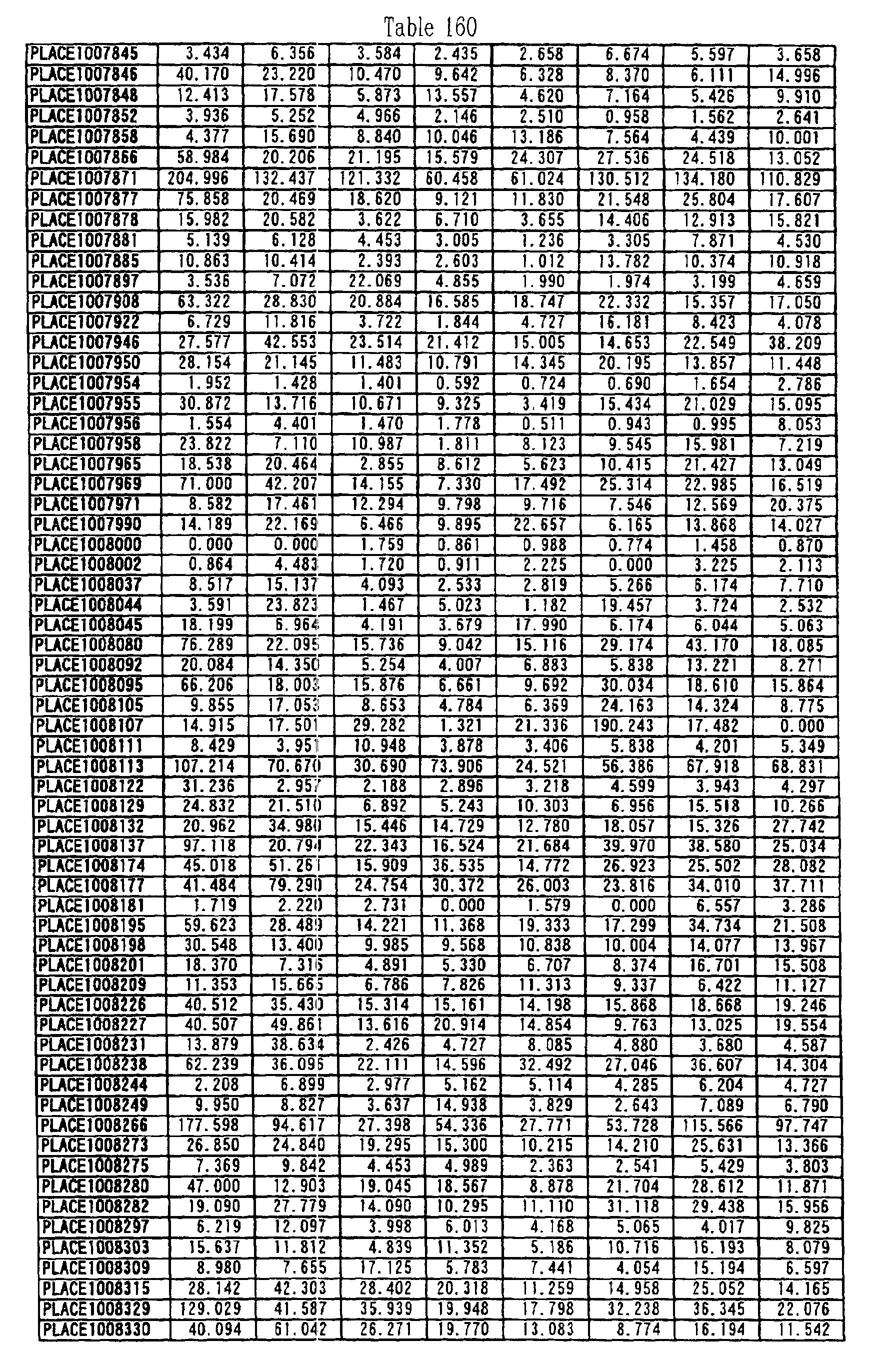

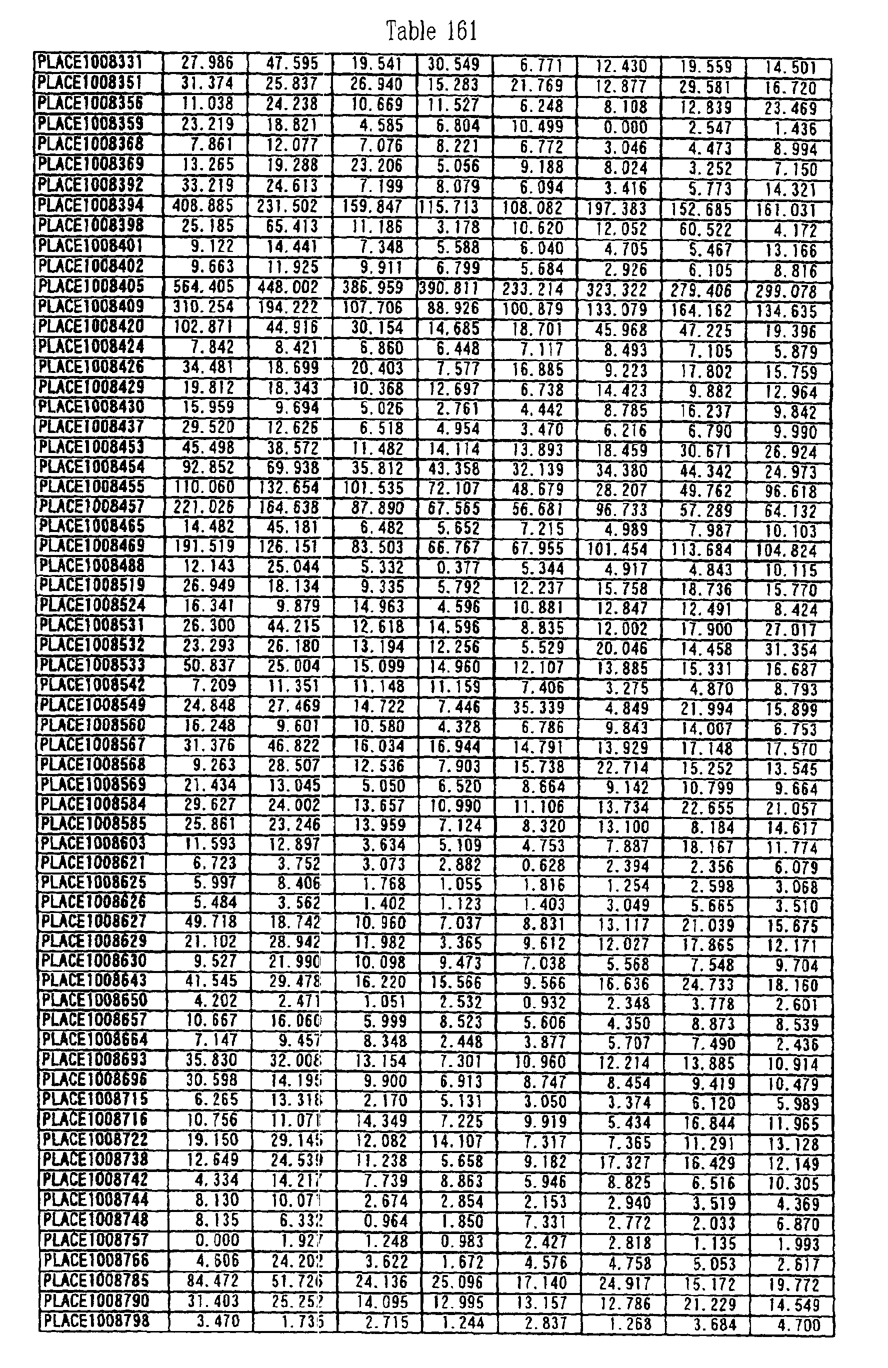

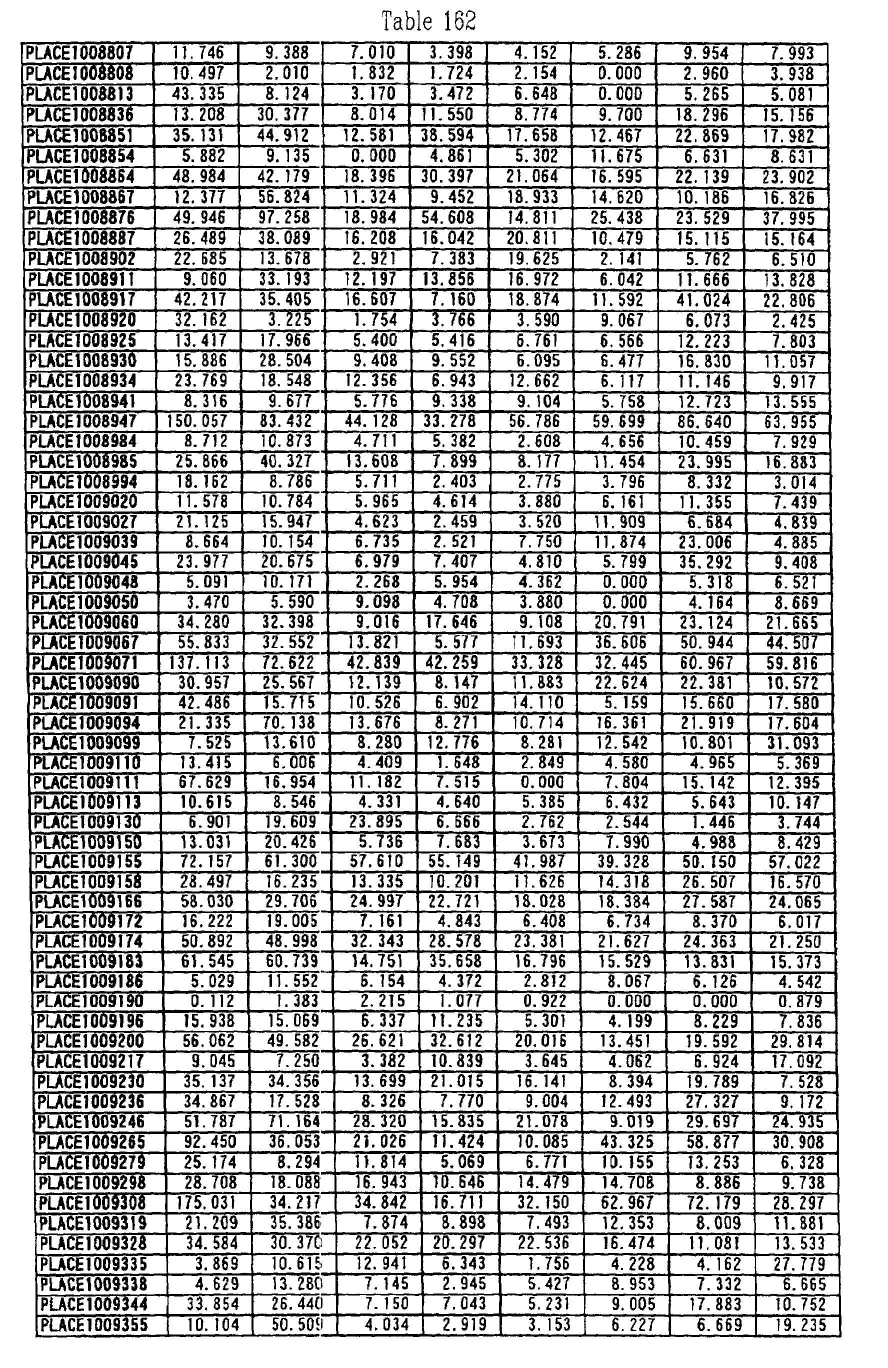

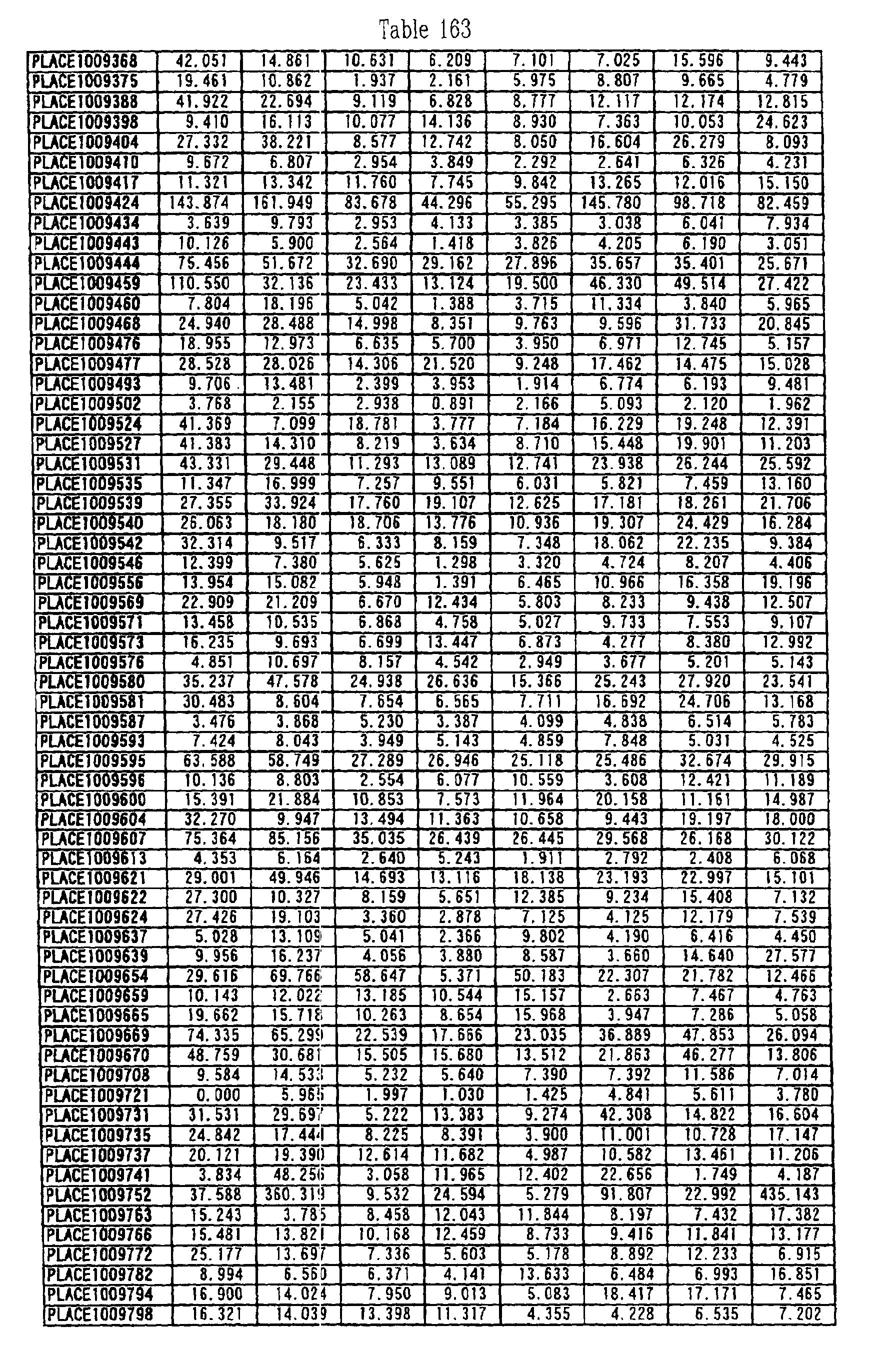

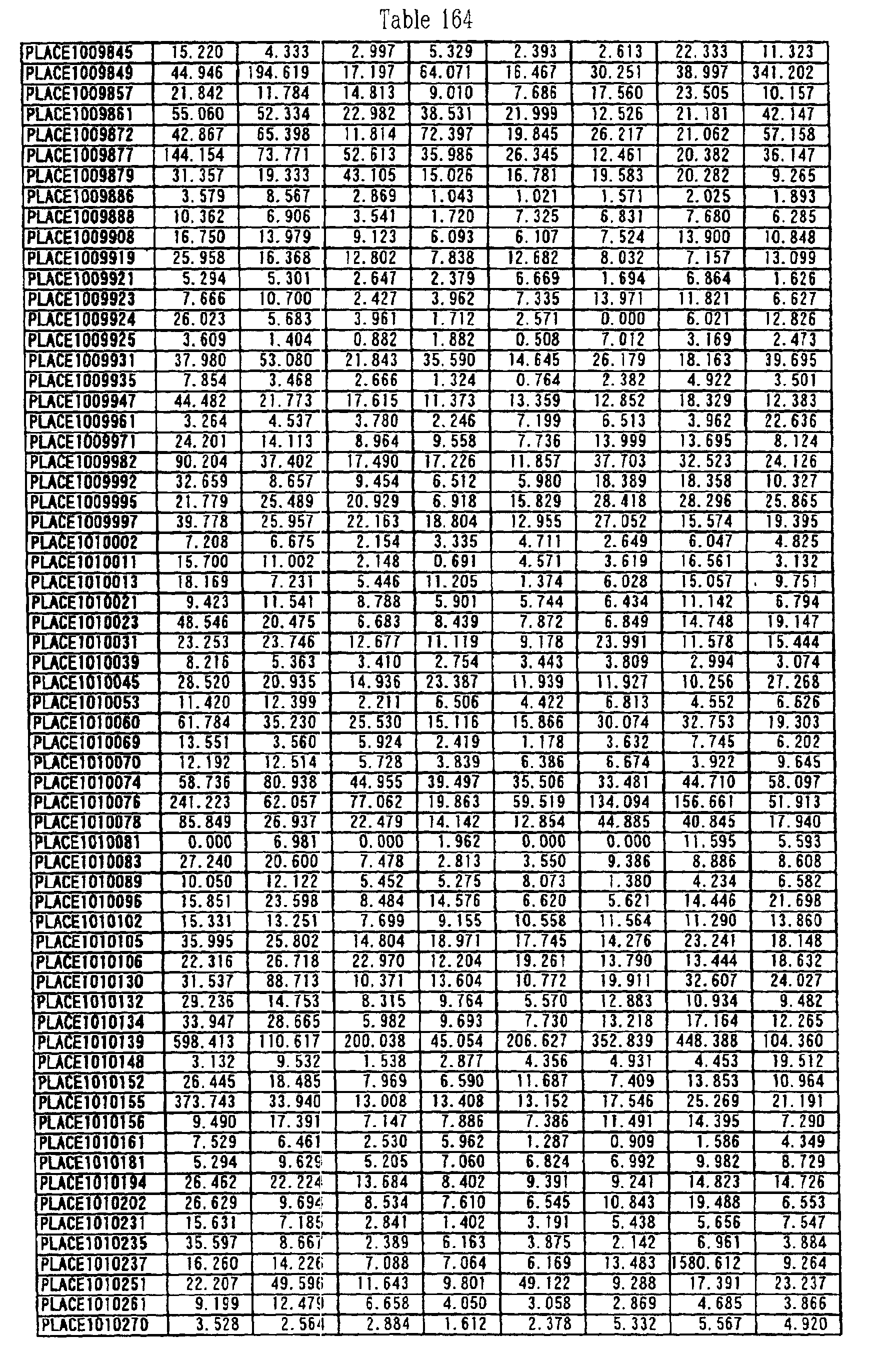

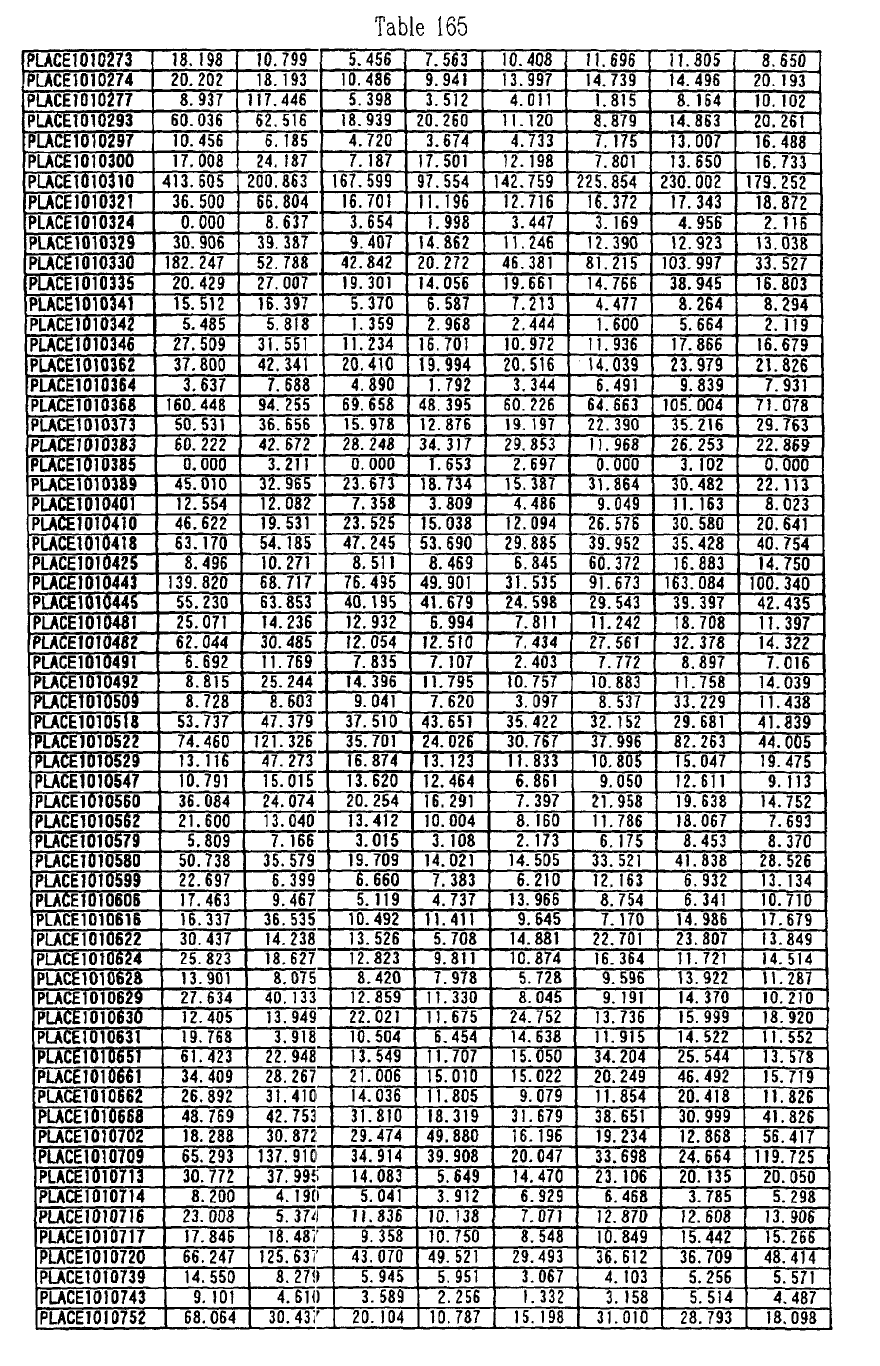

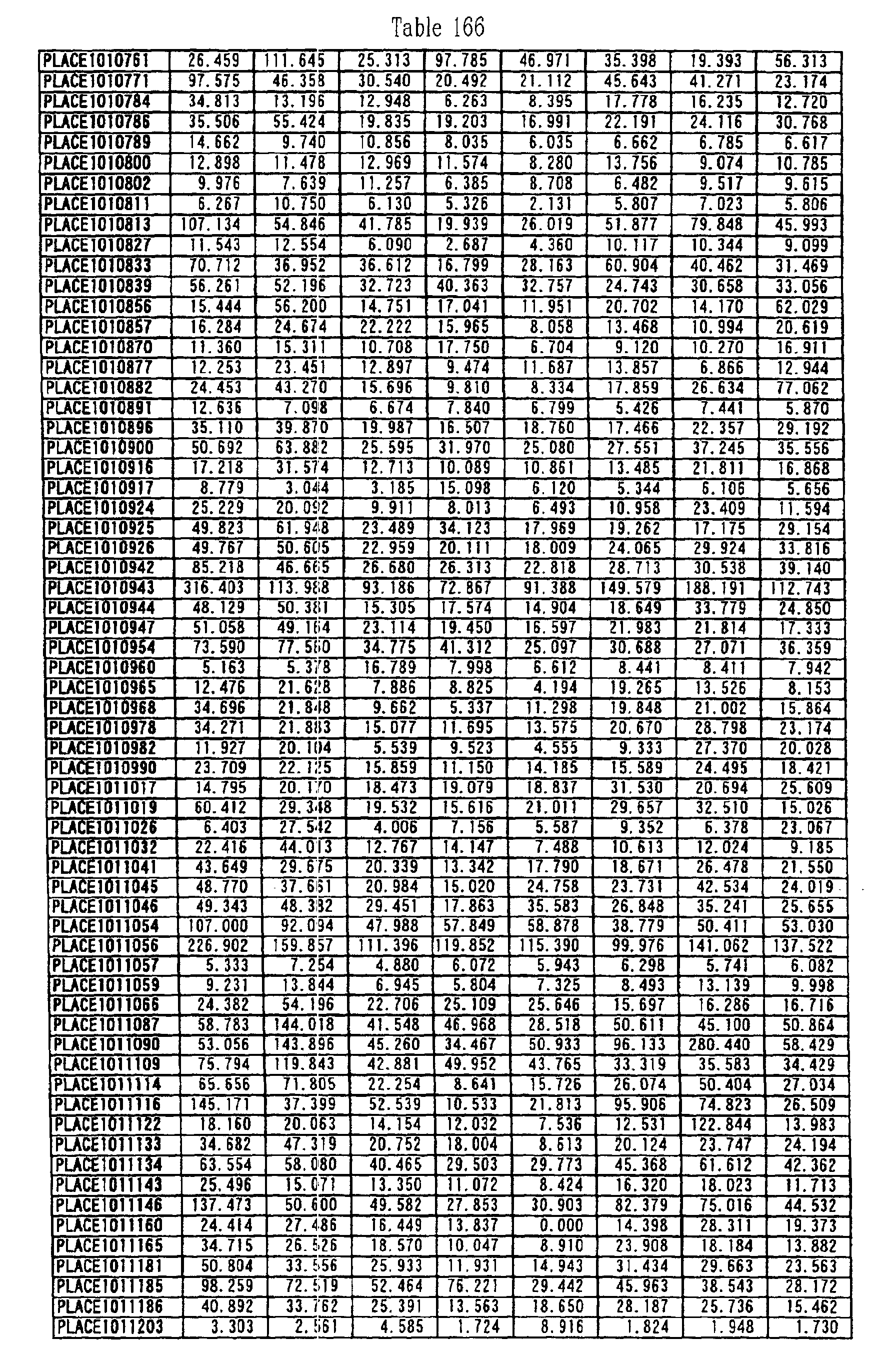

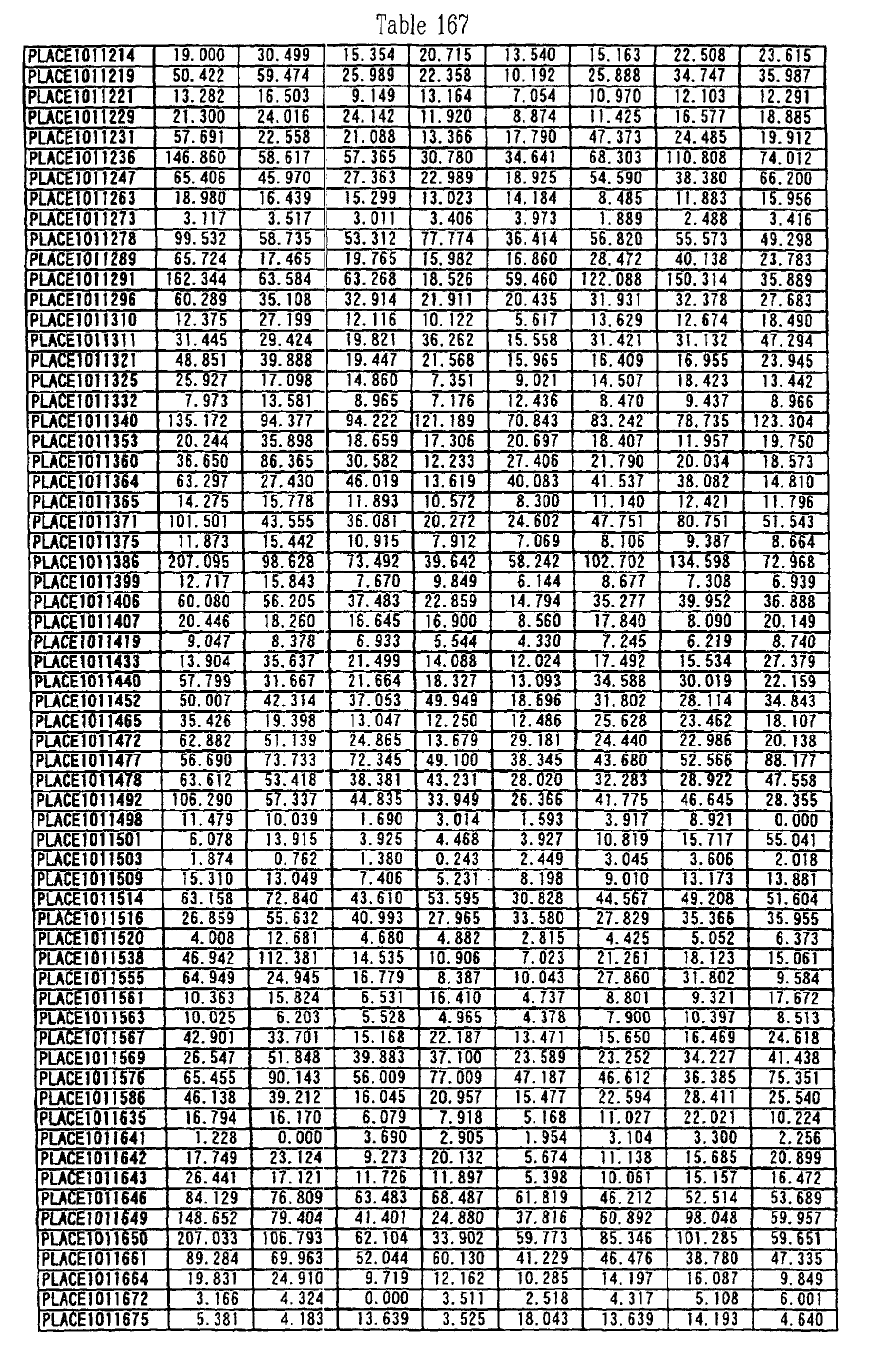

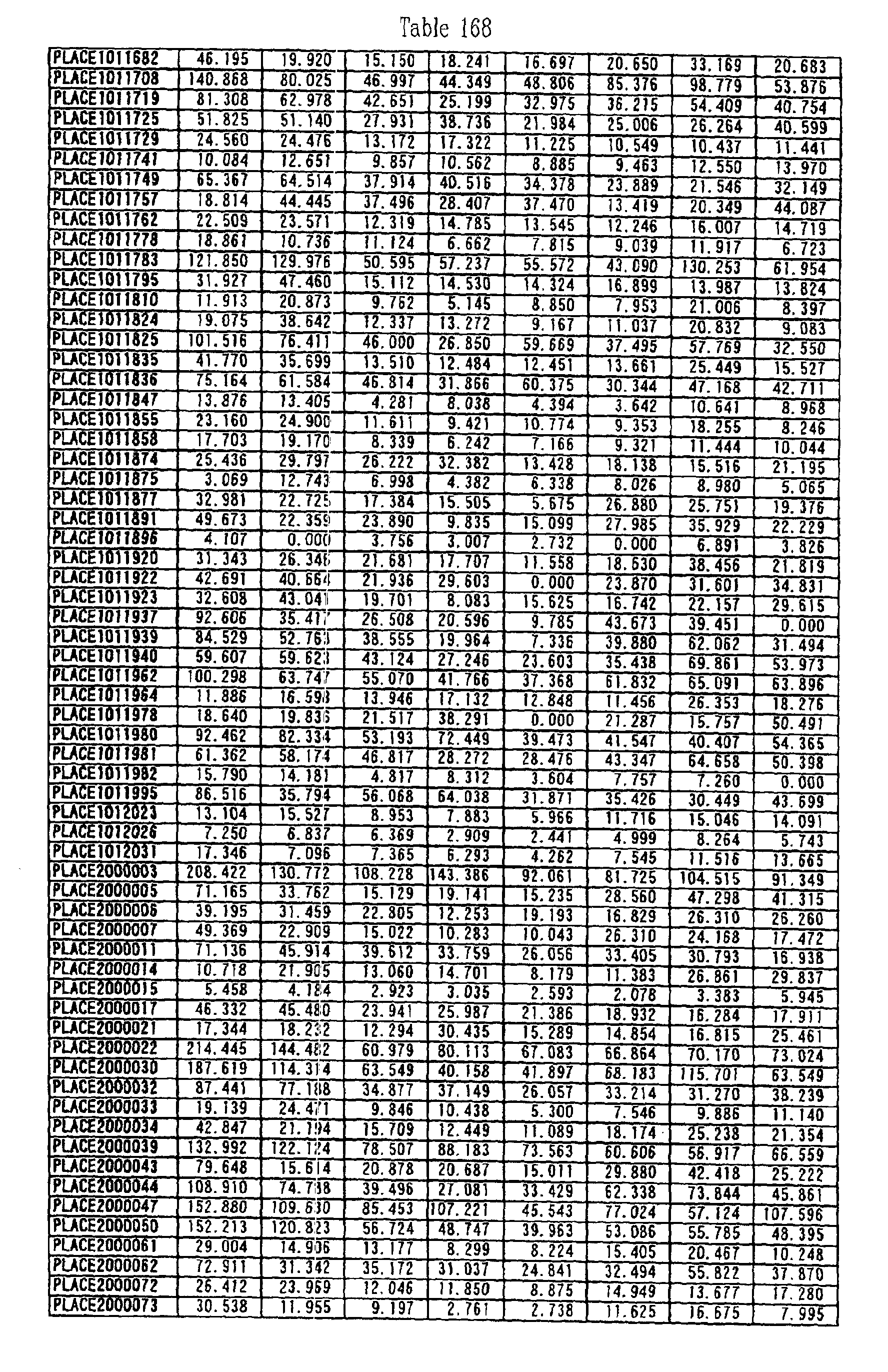

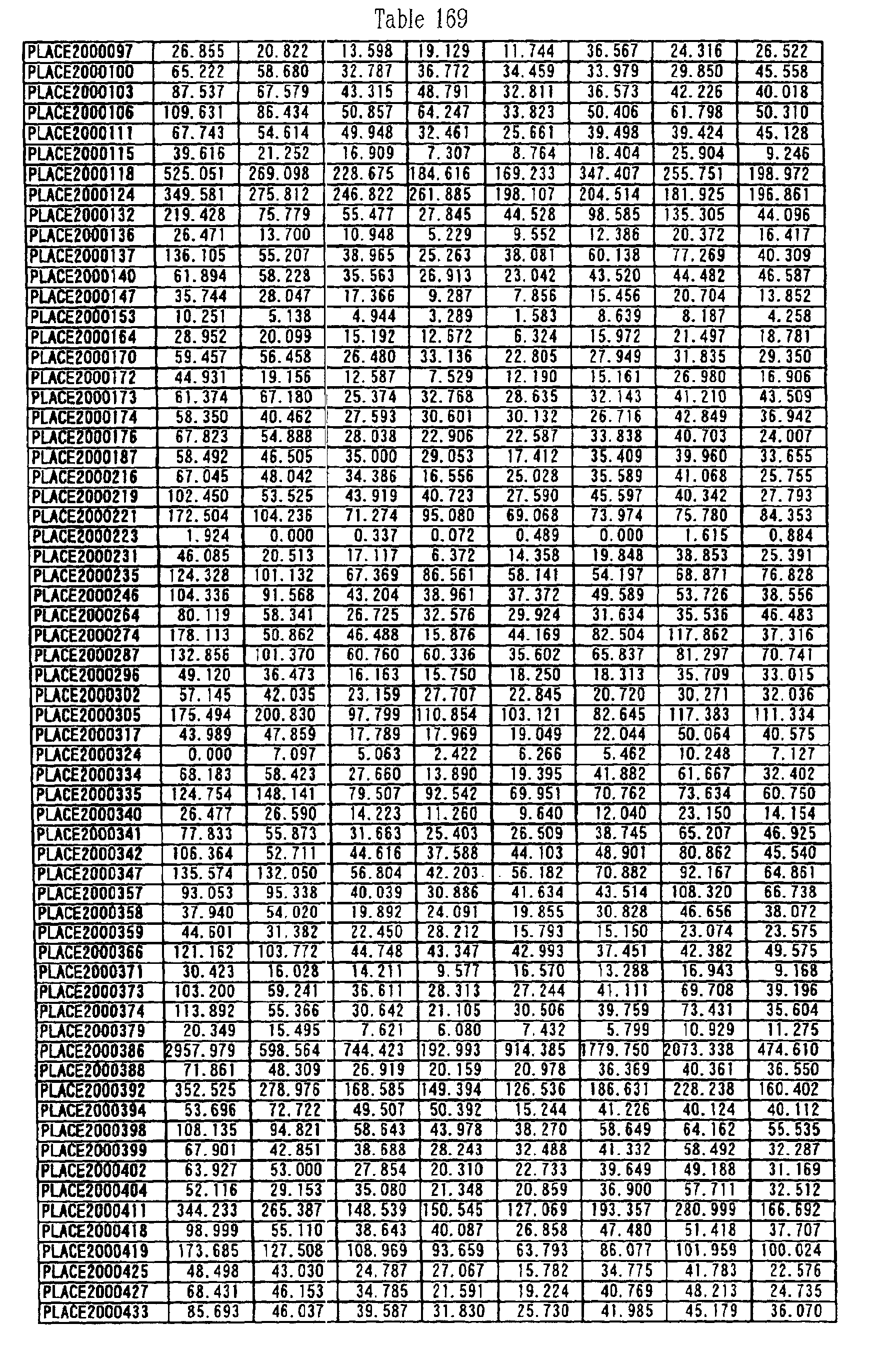

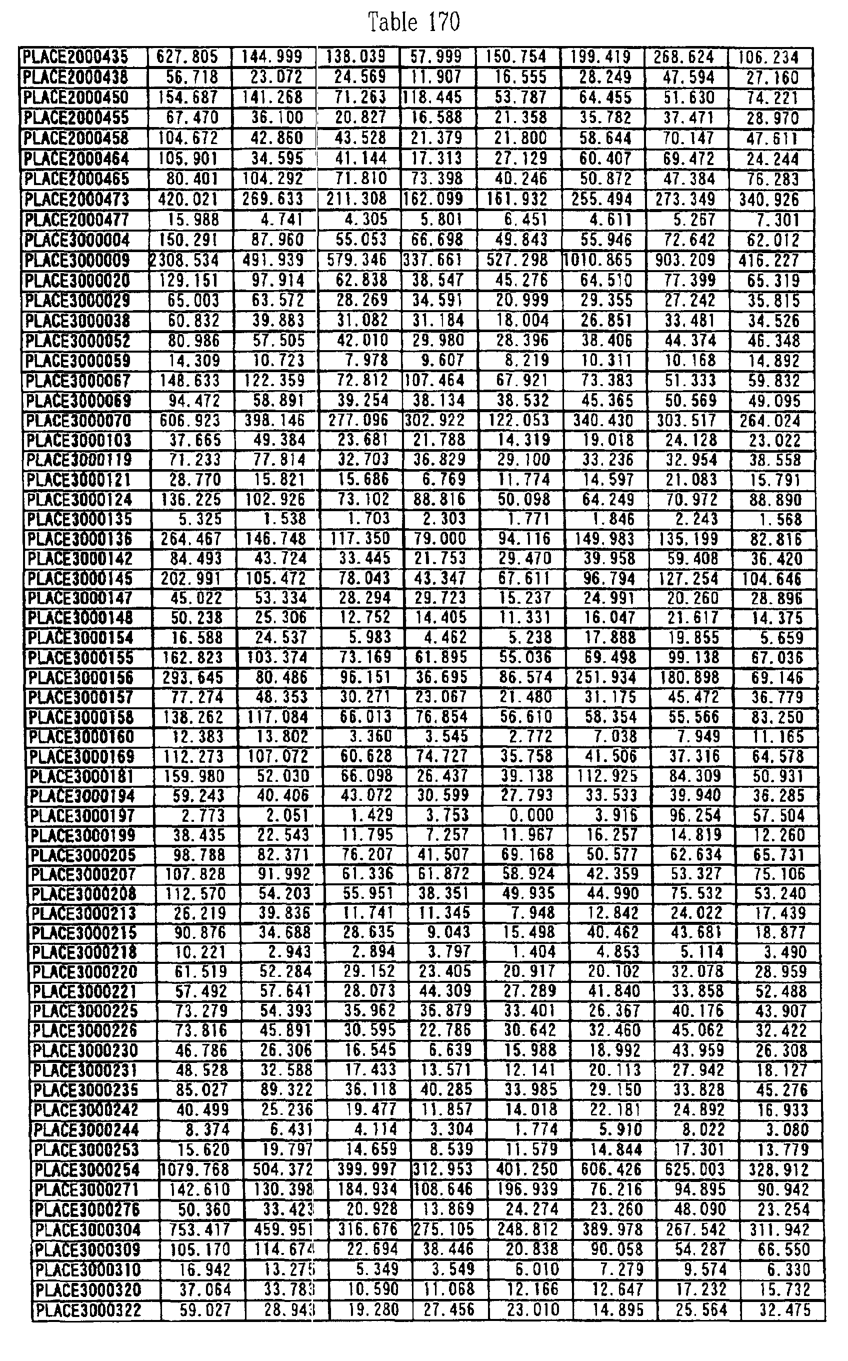

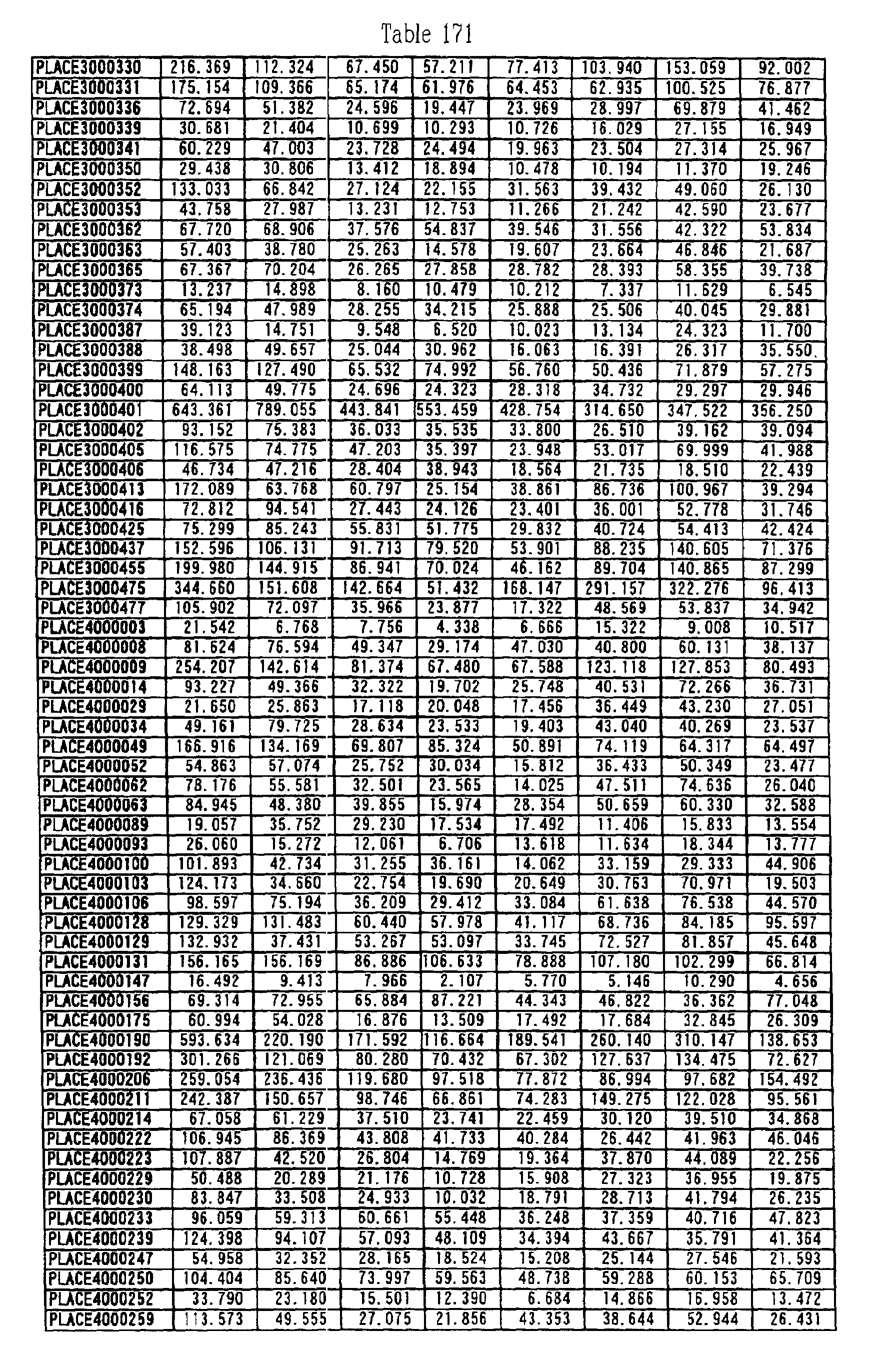

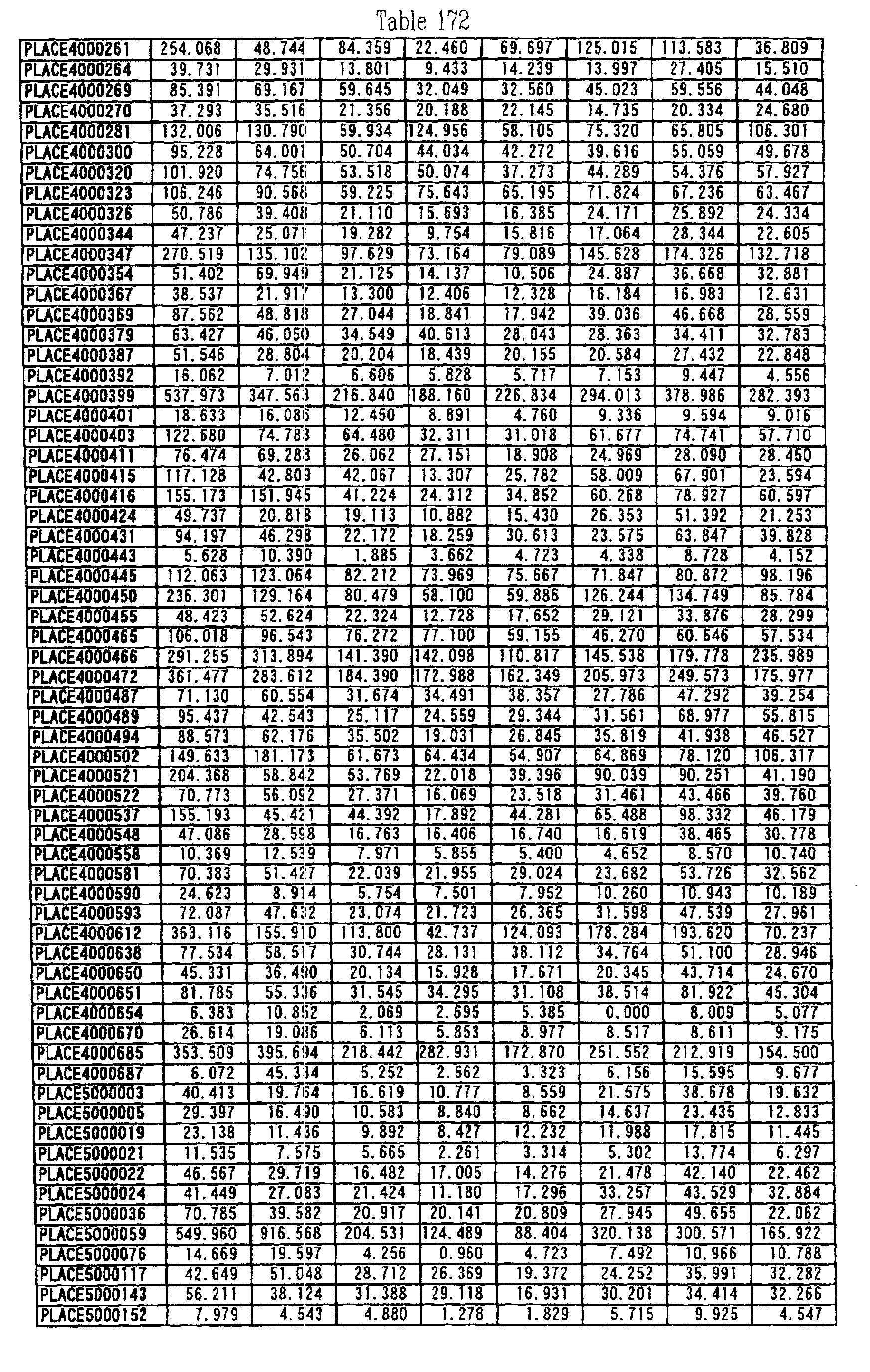

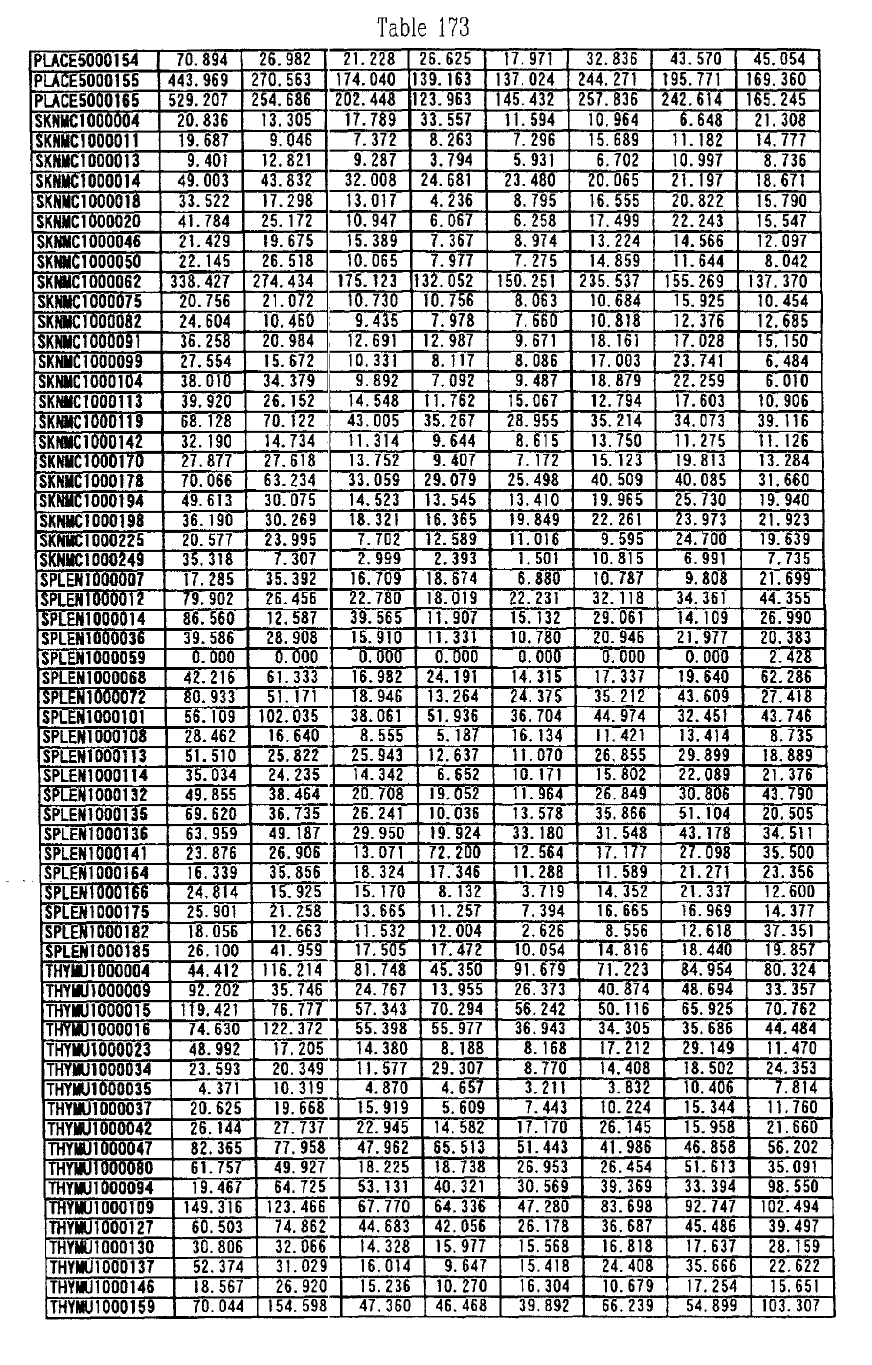

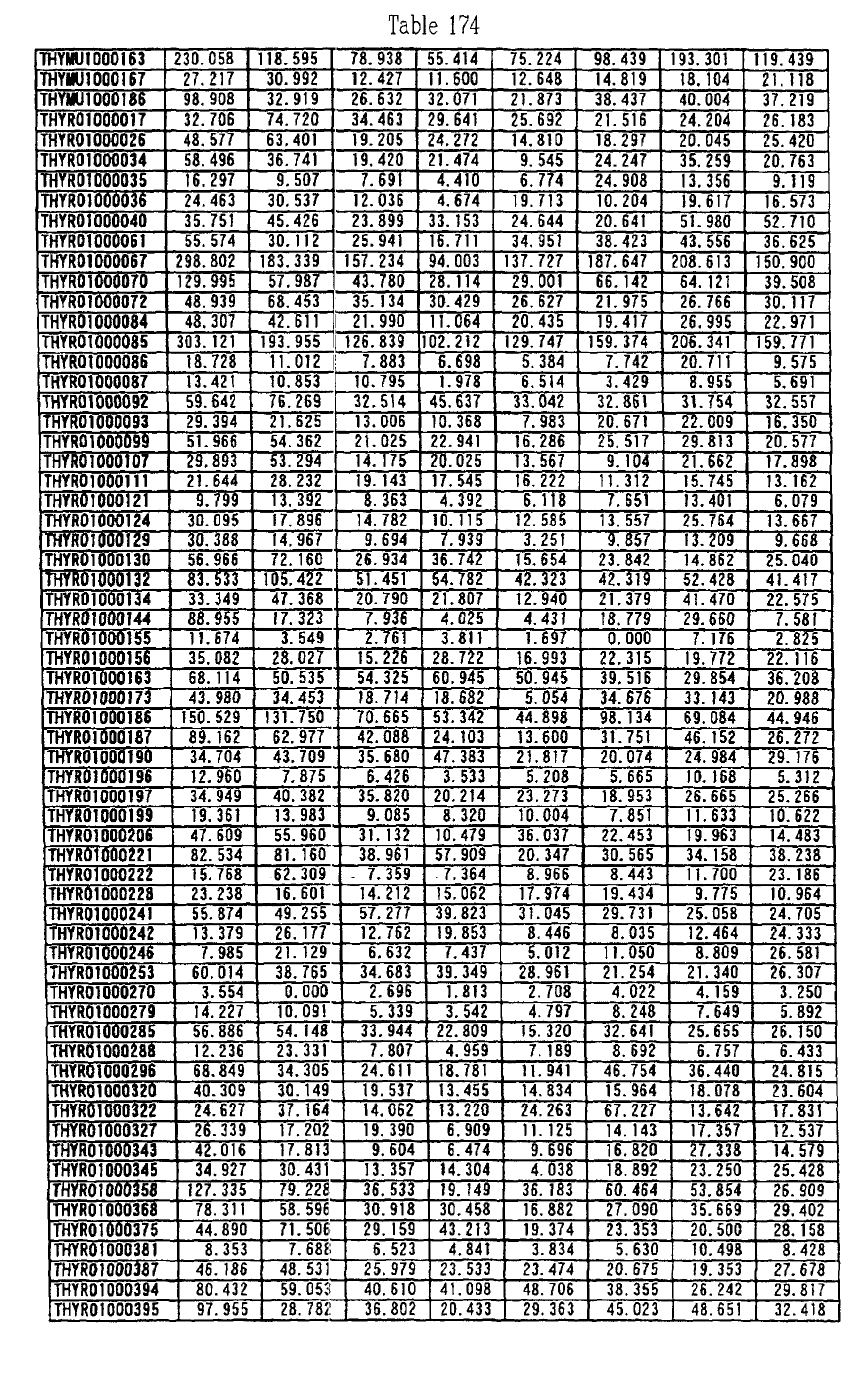

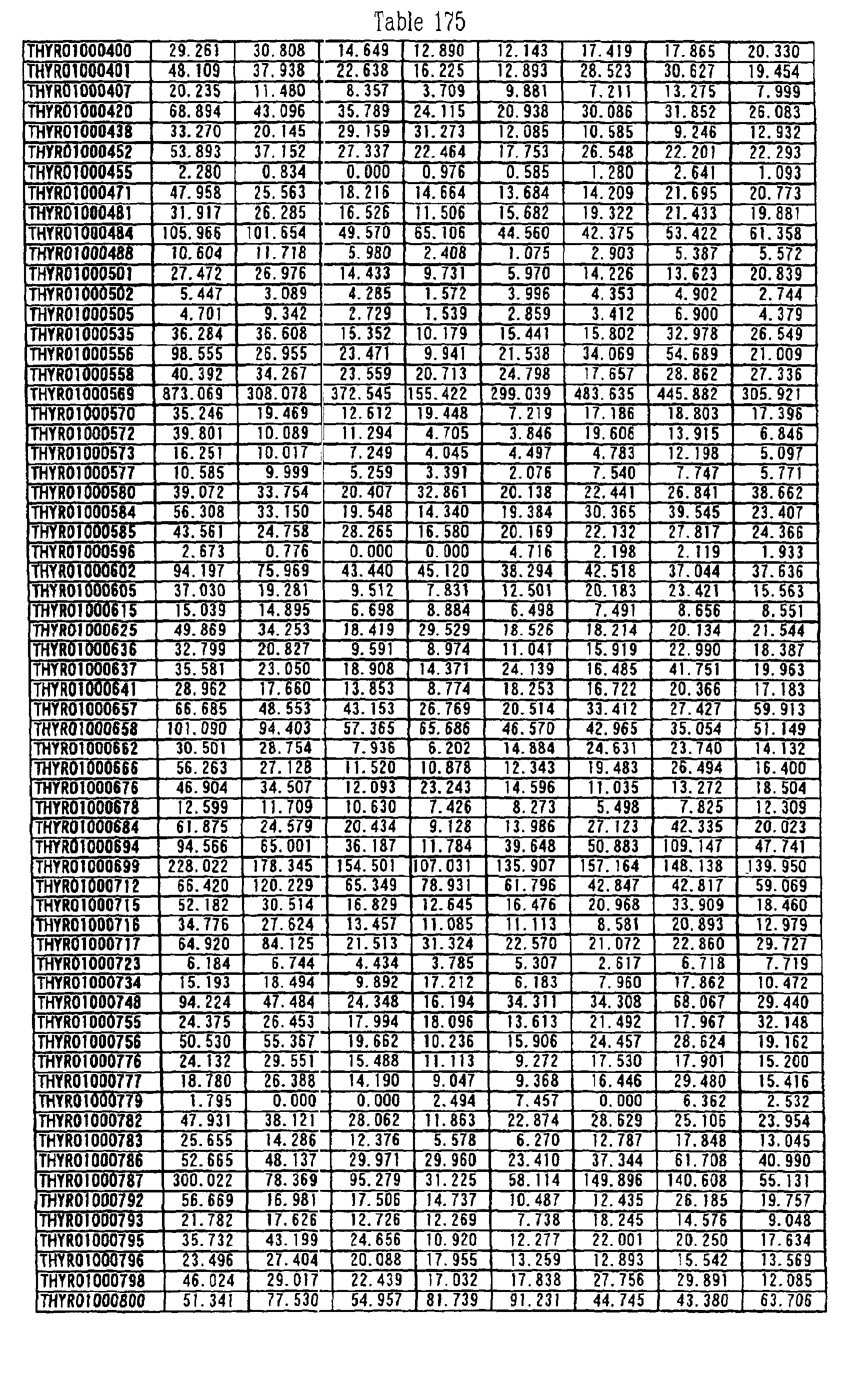

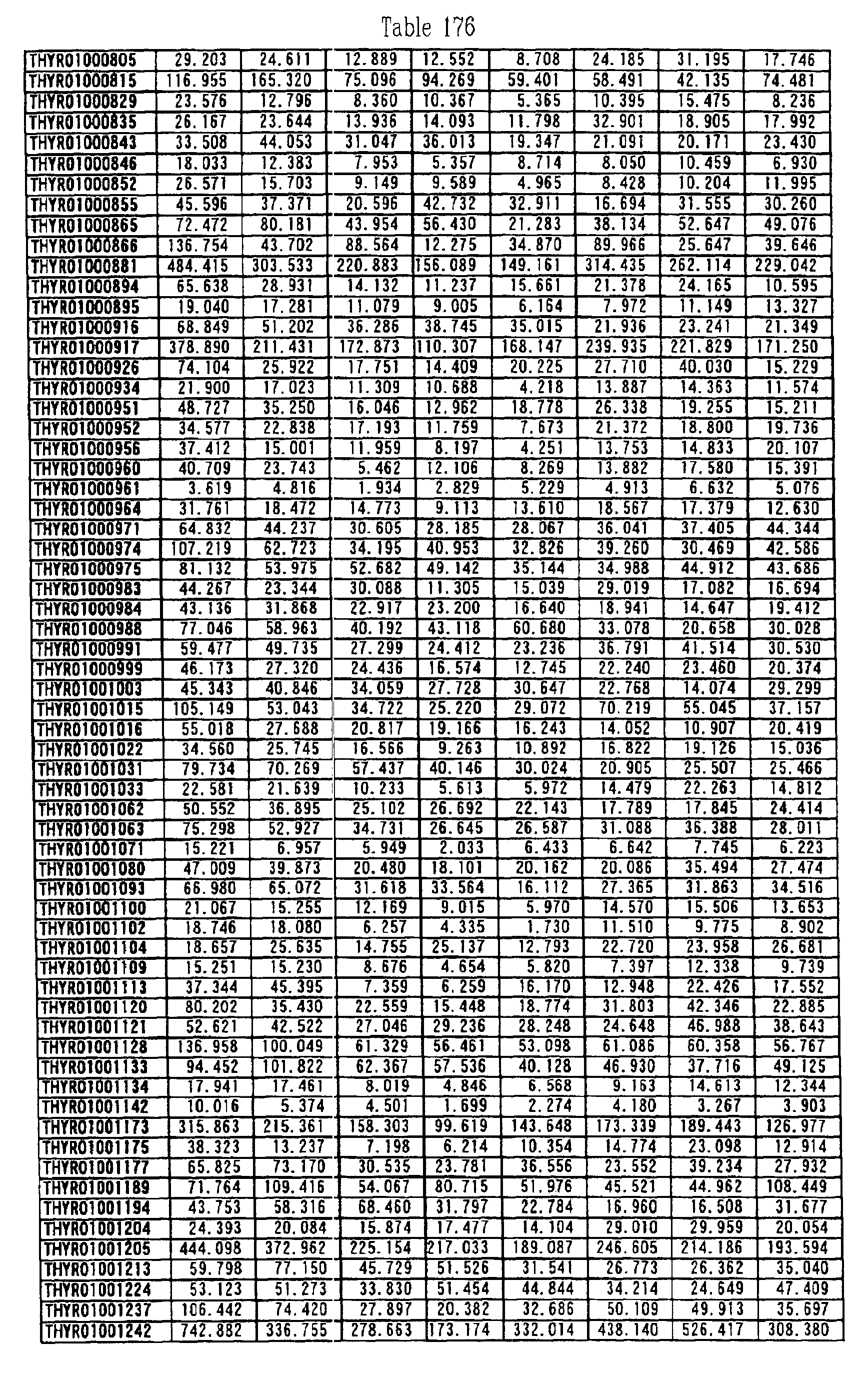

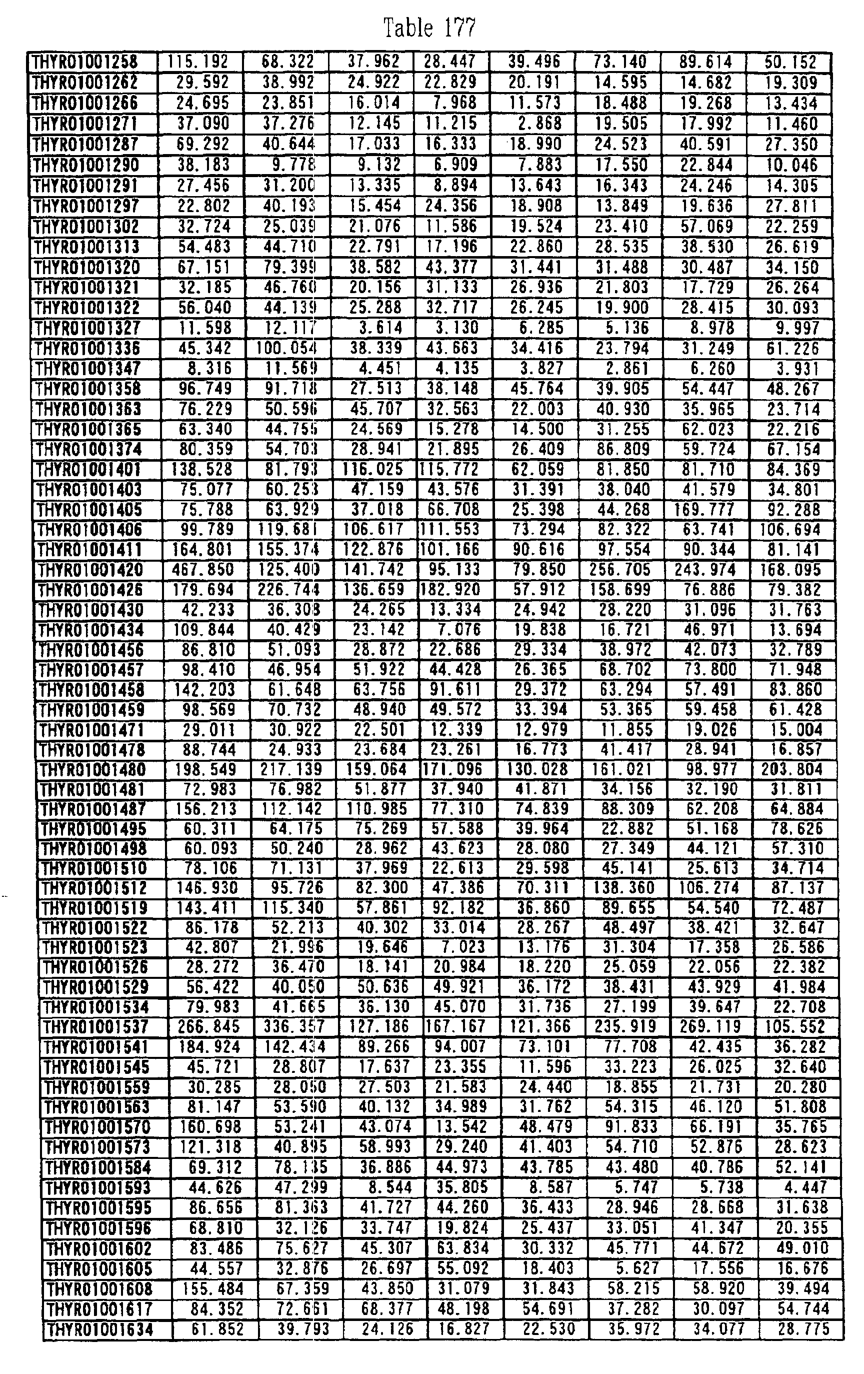

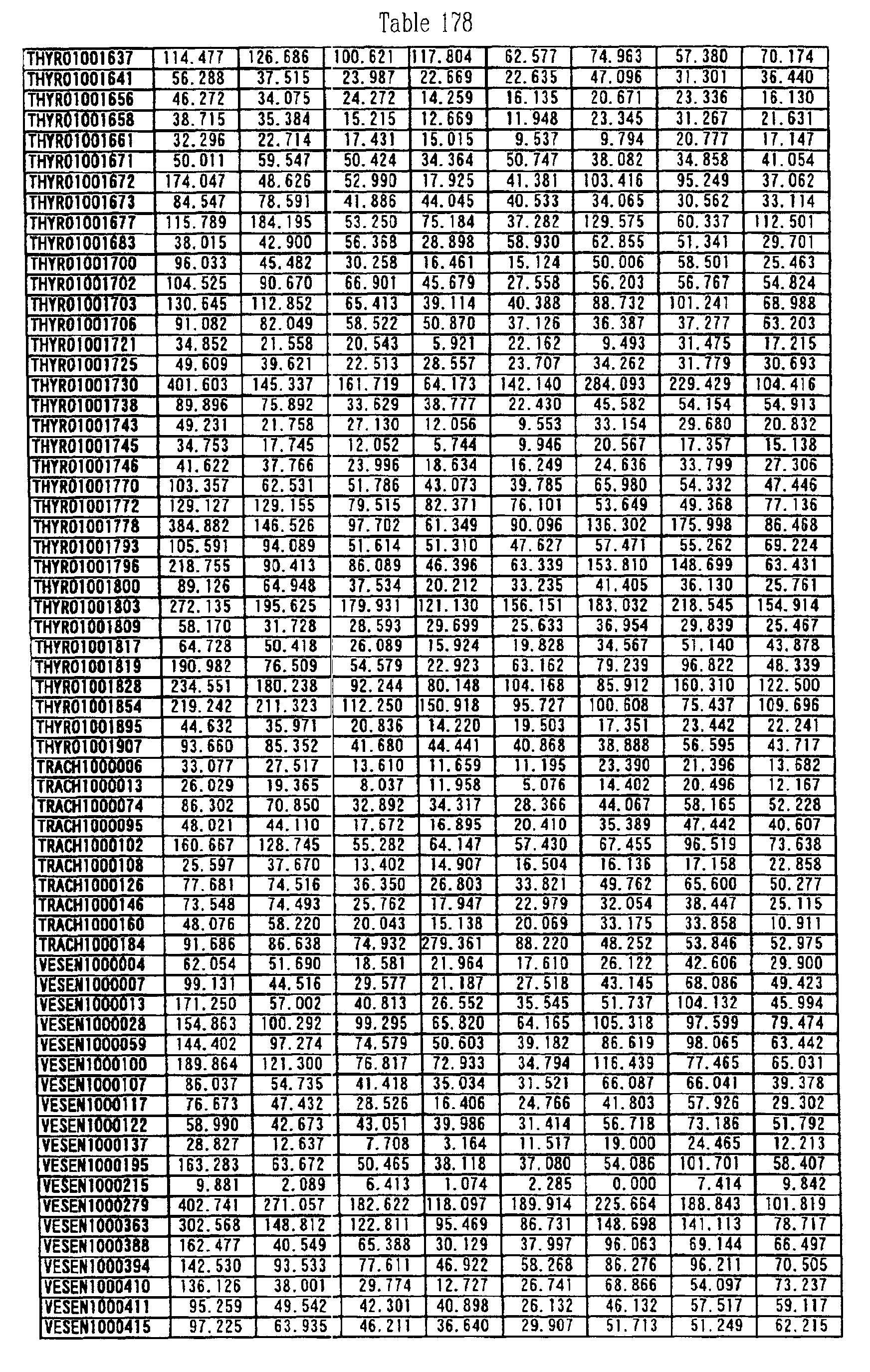

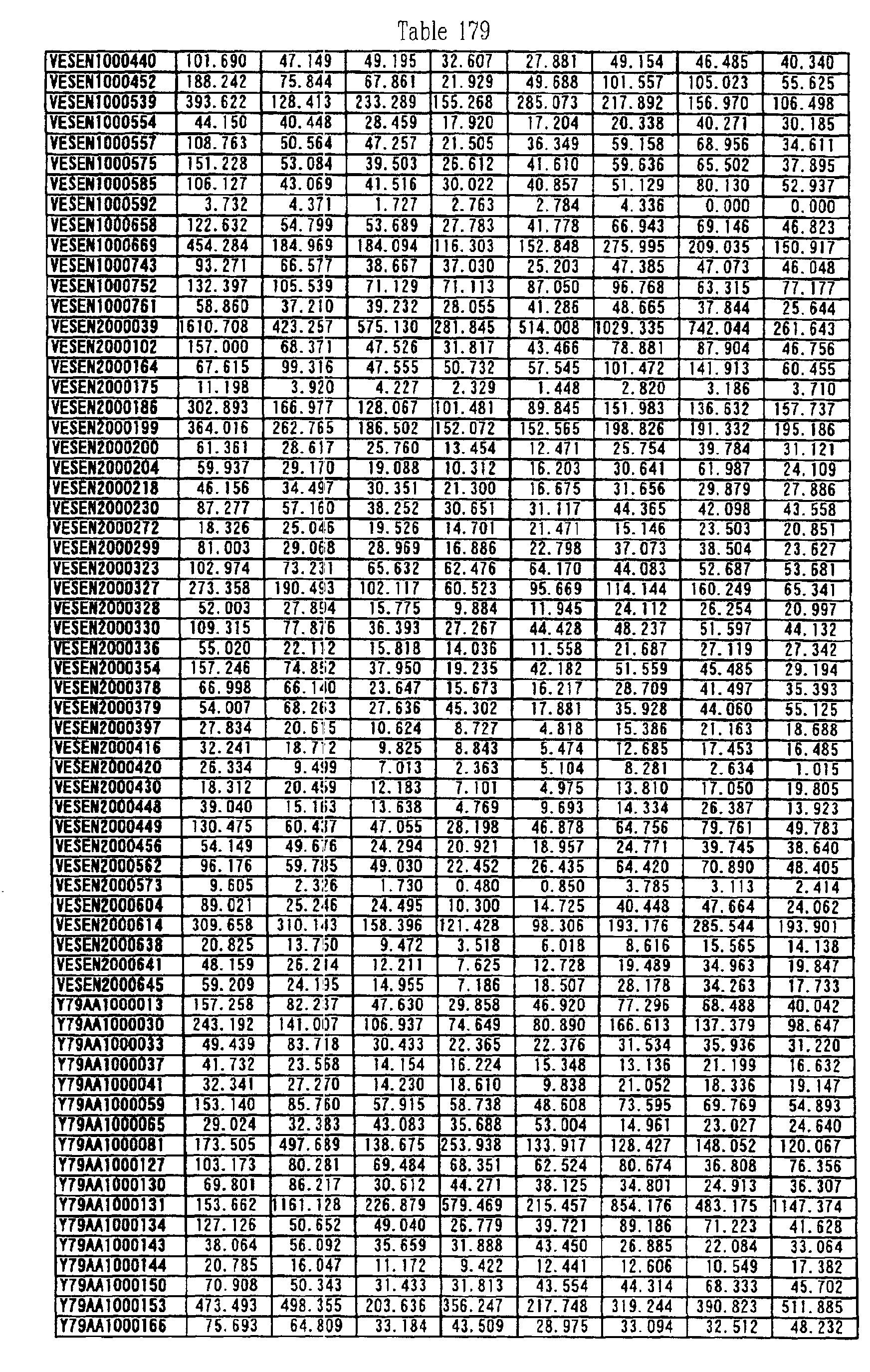

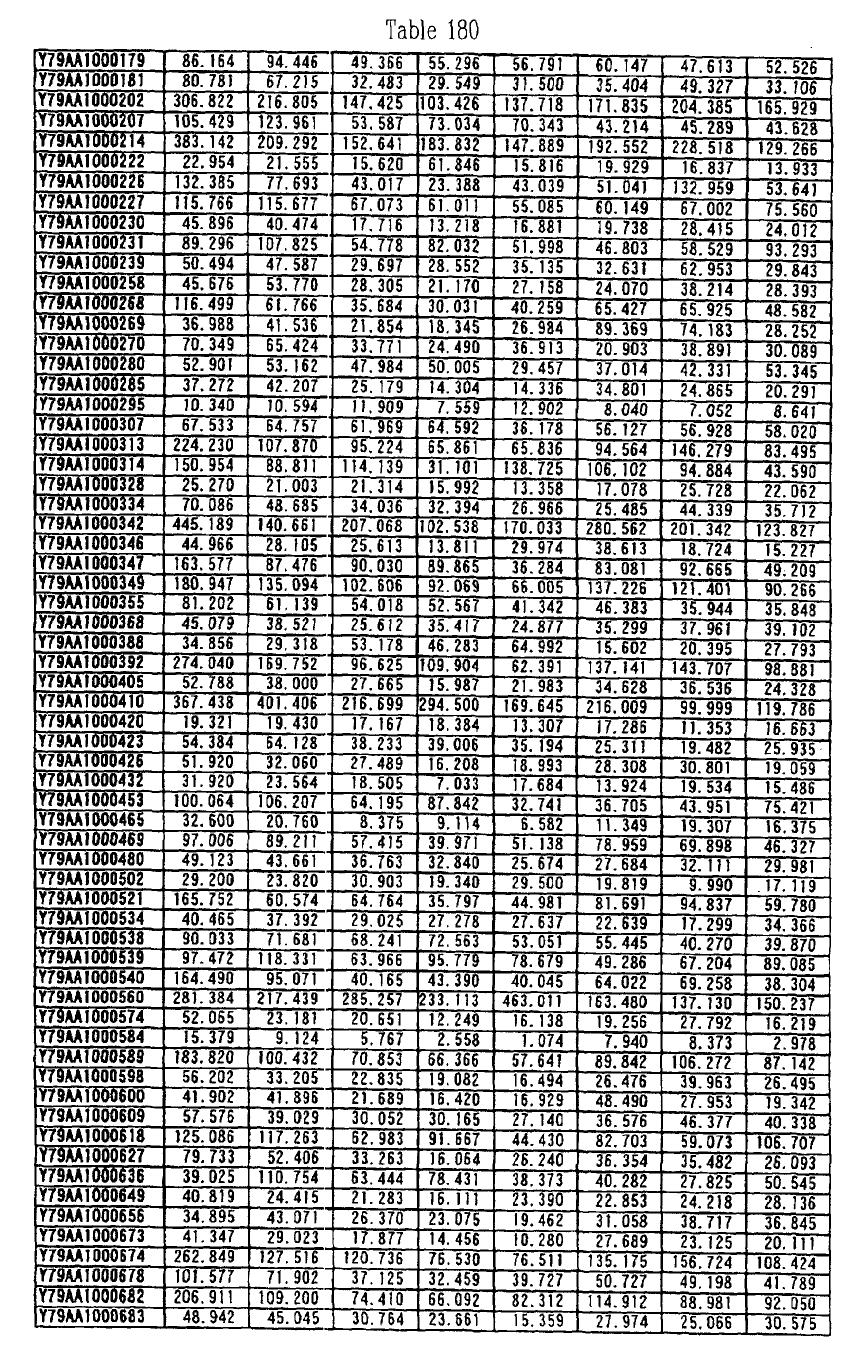

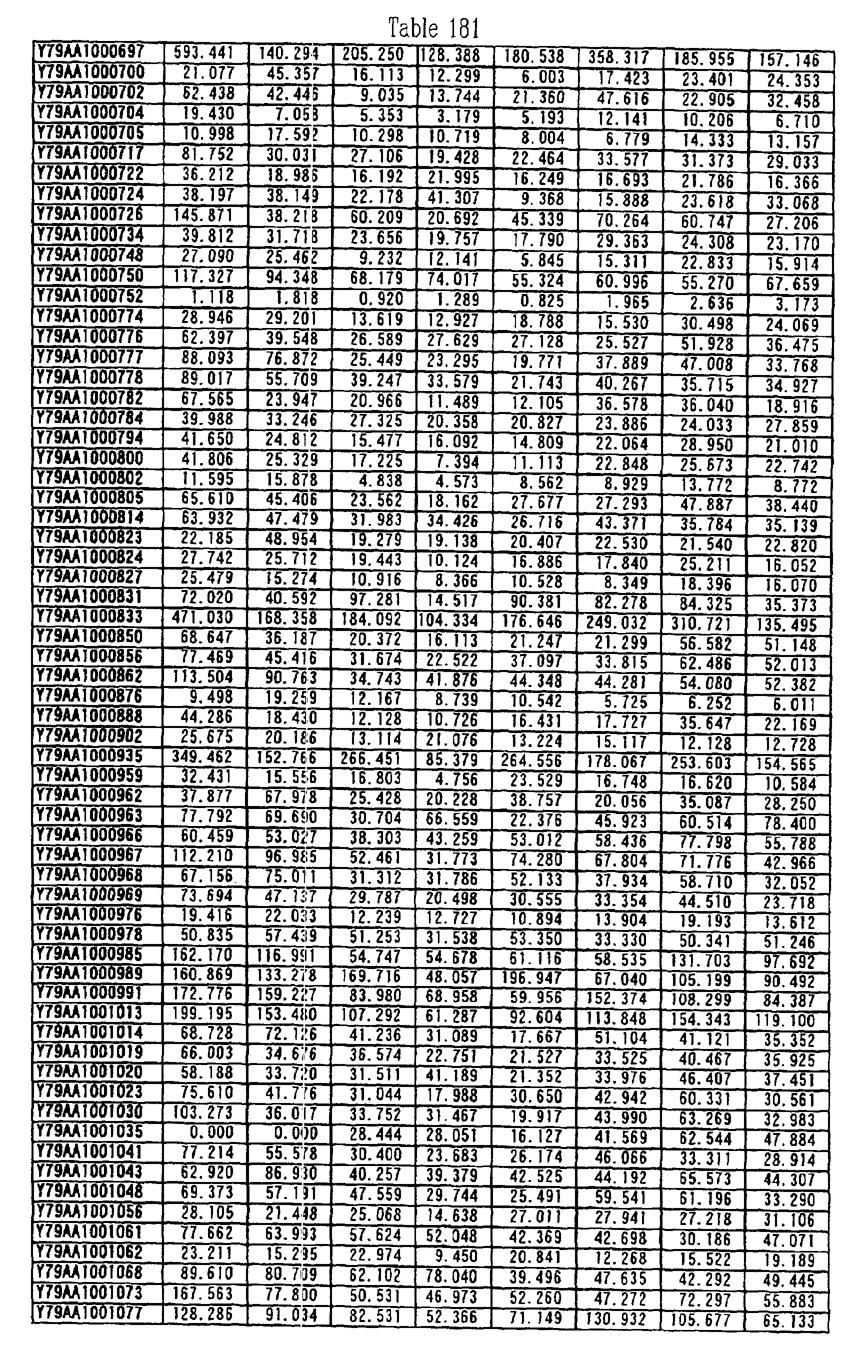

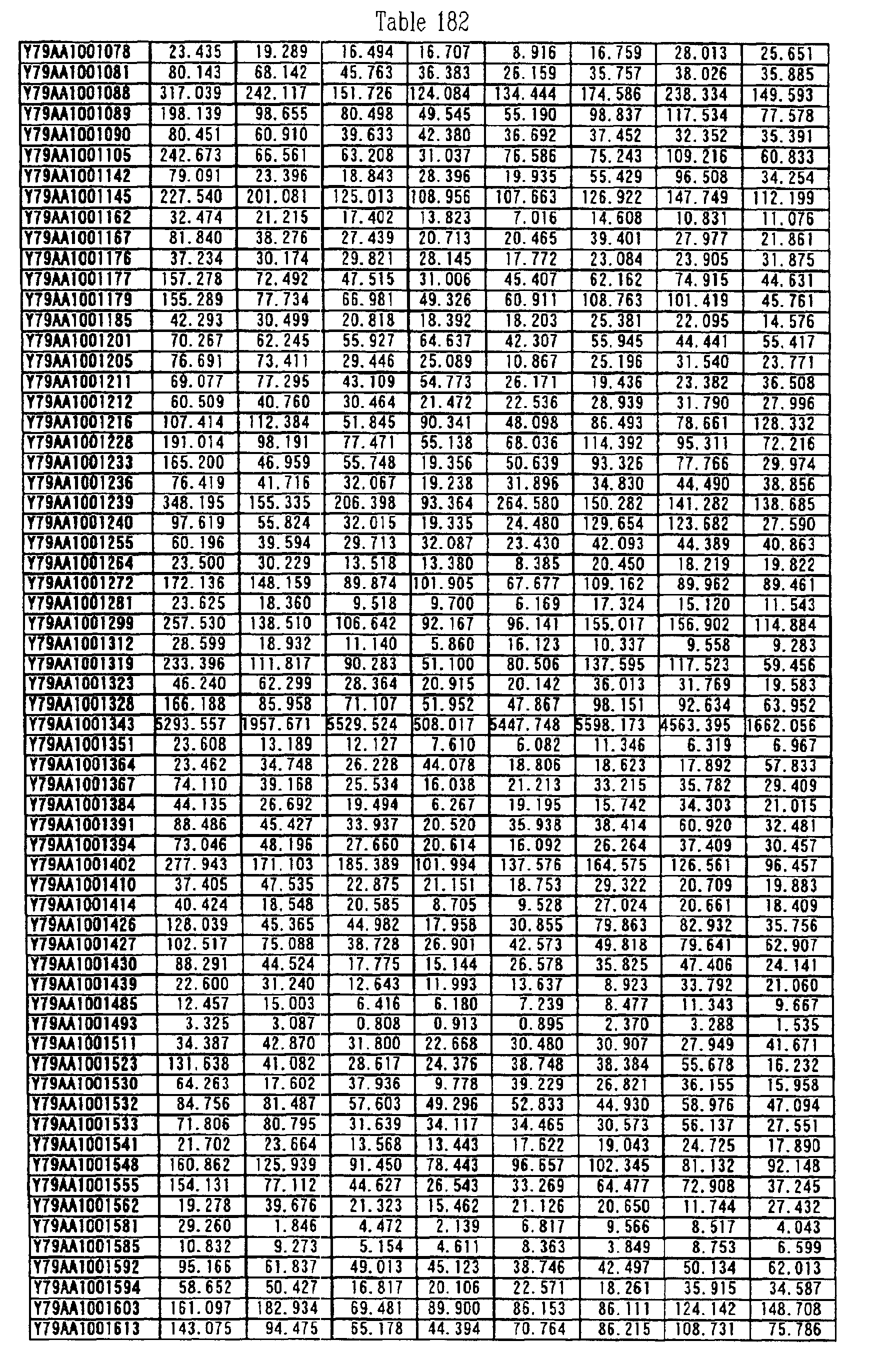

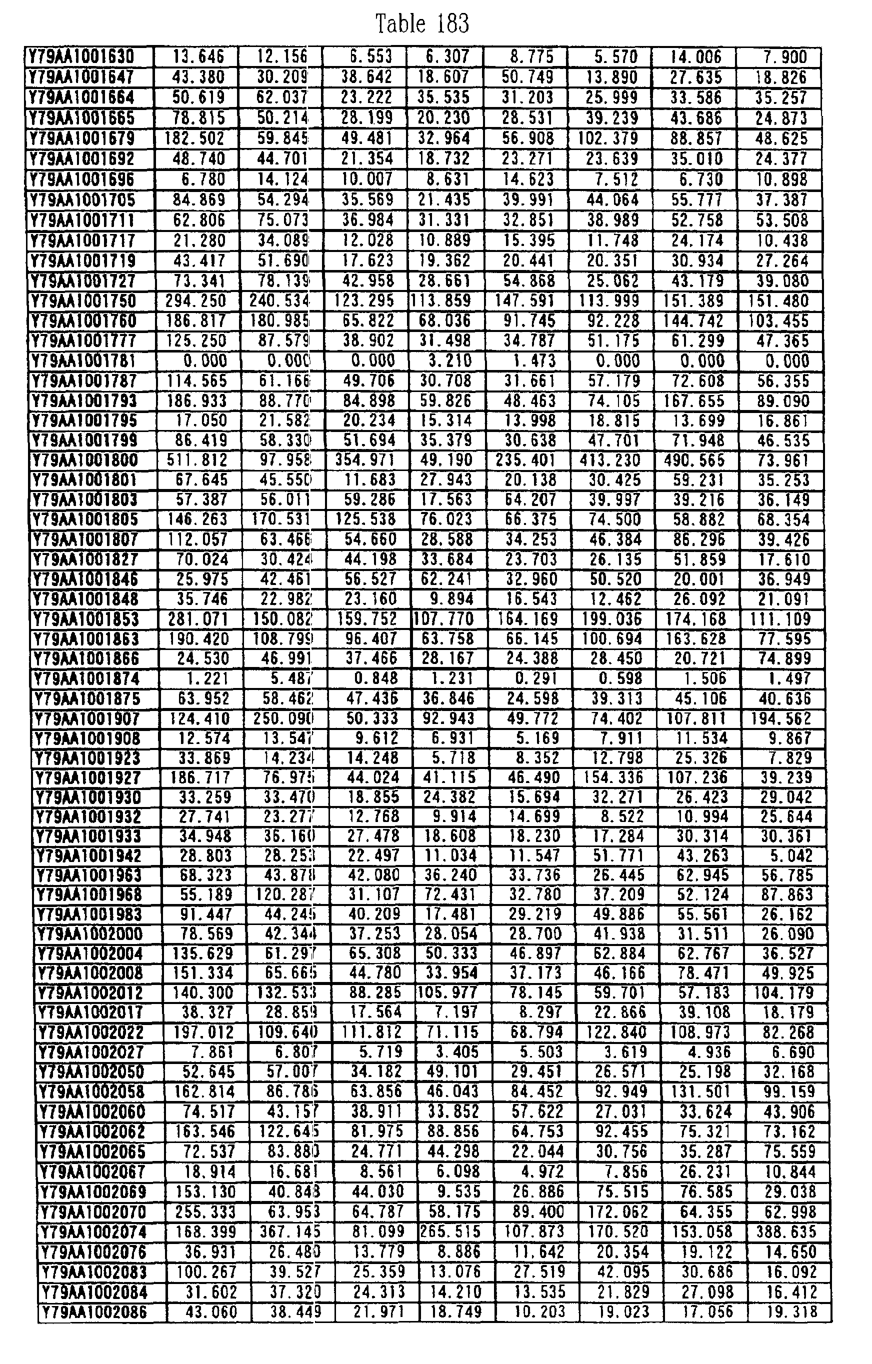

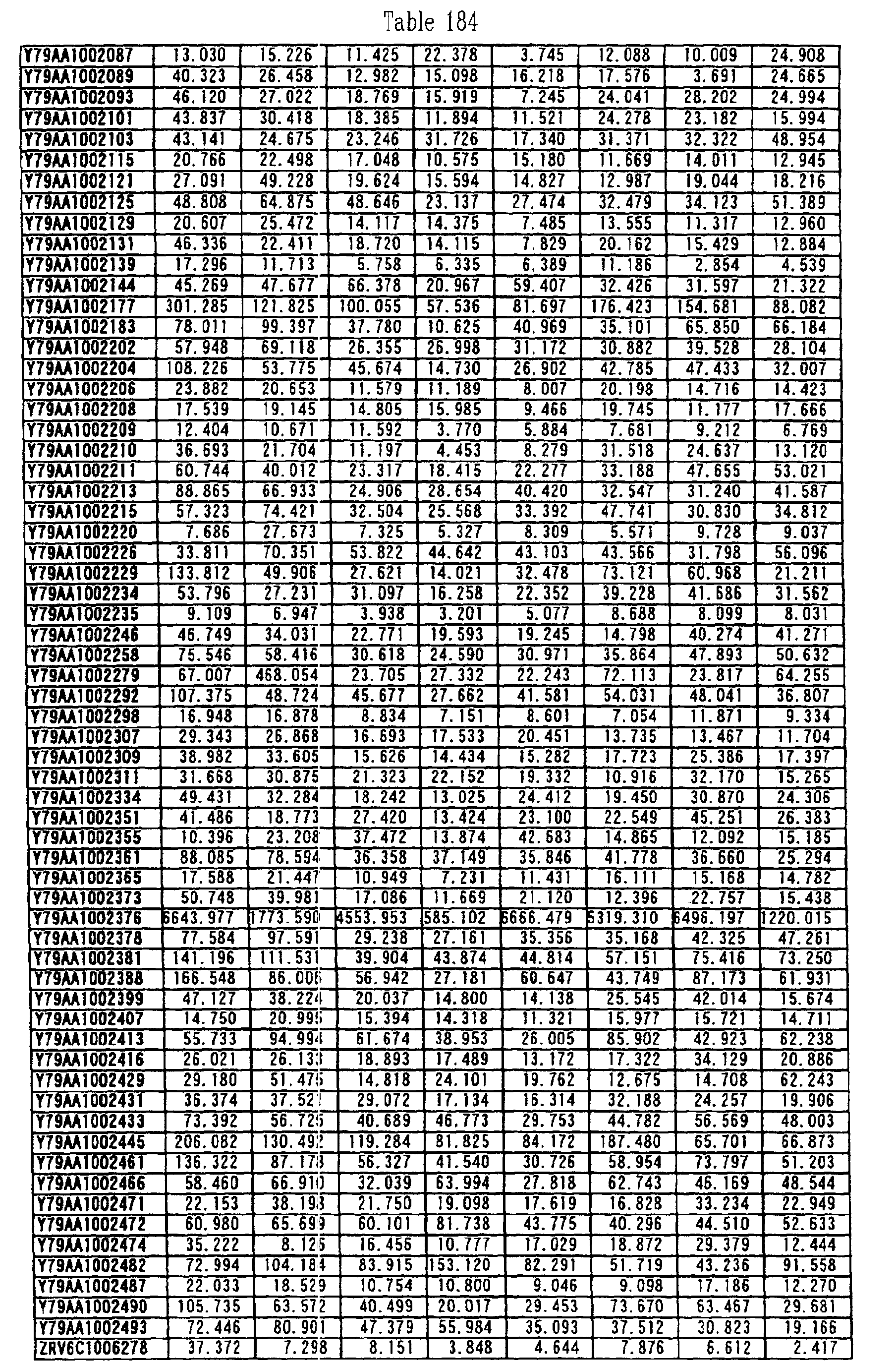

Expression of each cDNA in human tissues (The Table also contains clones without description in Examples)

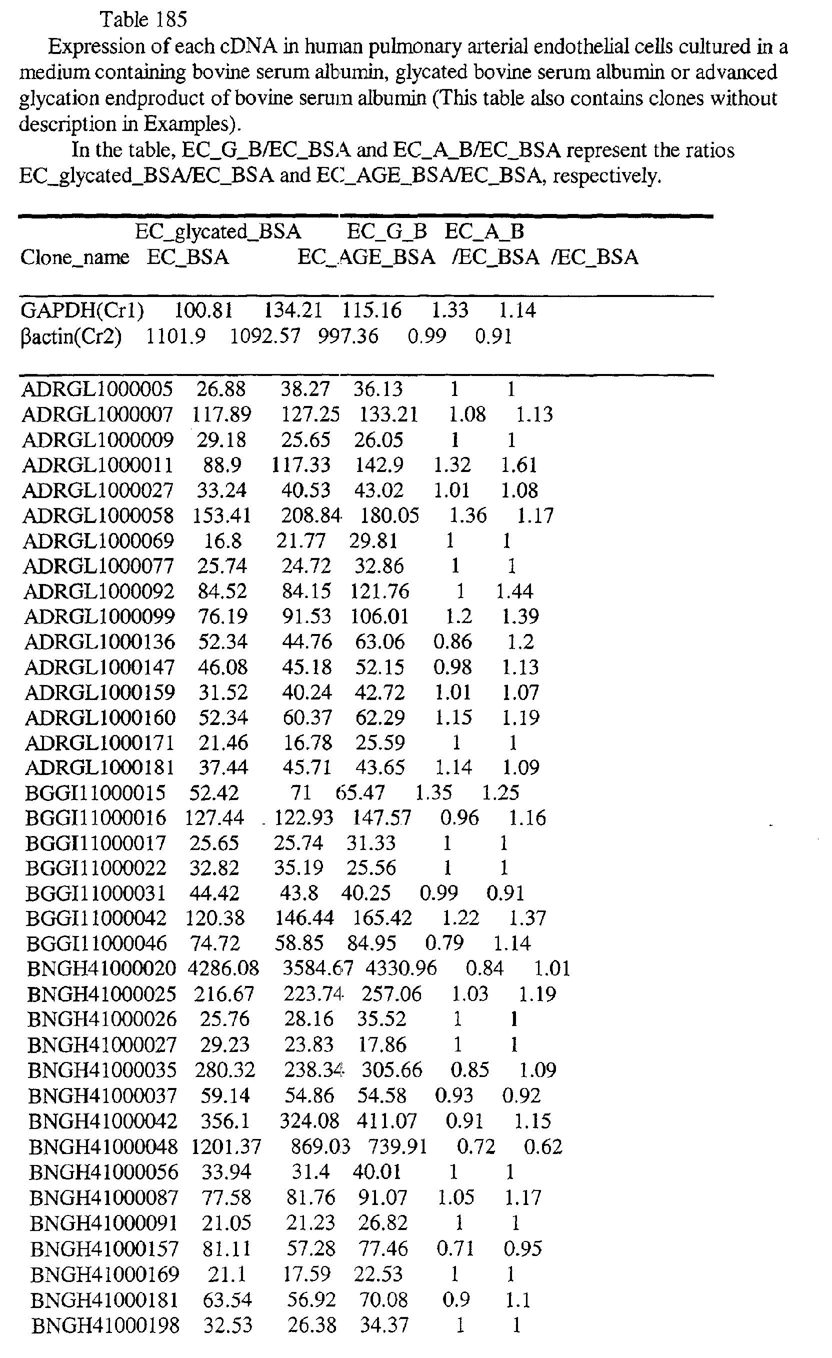

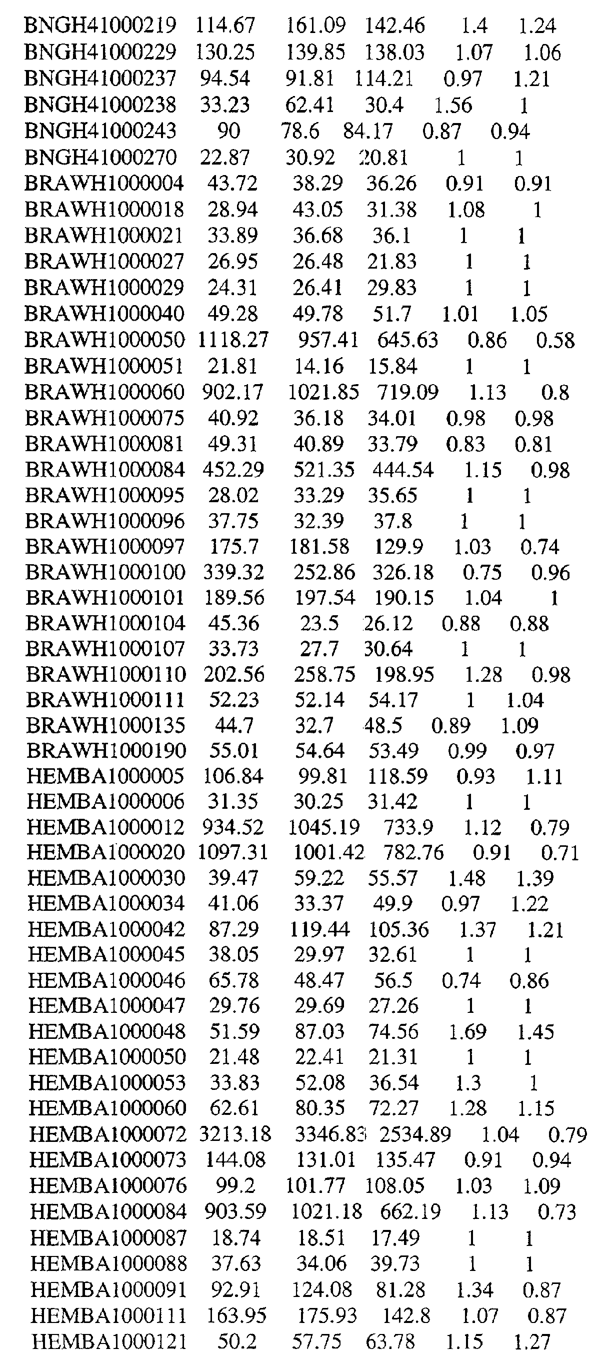

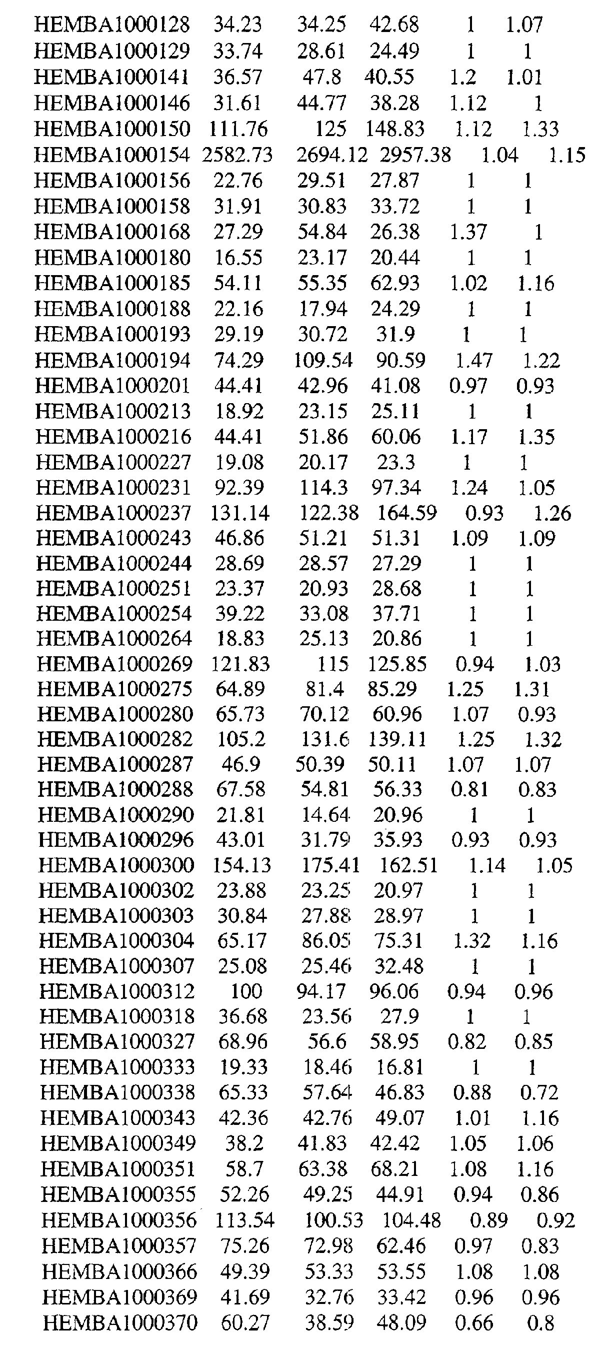

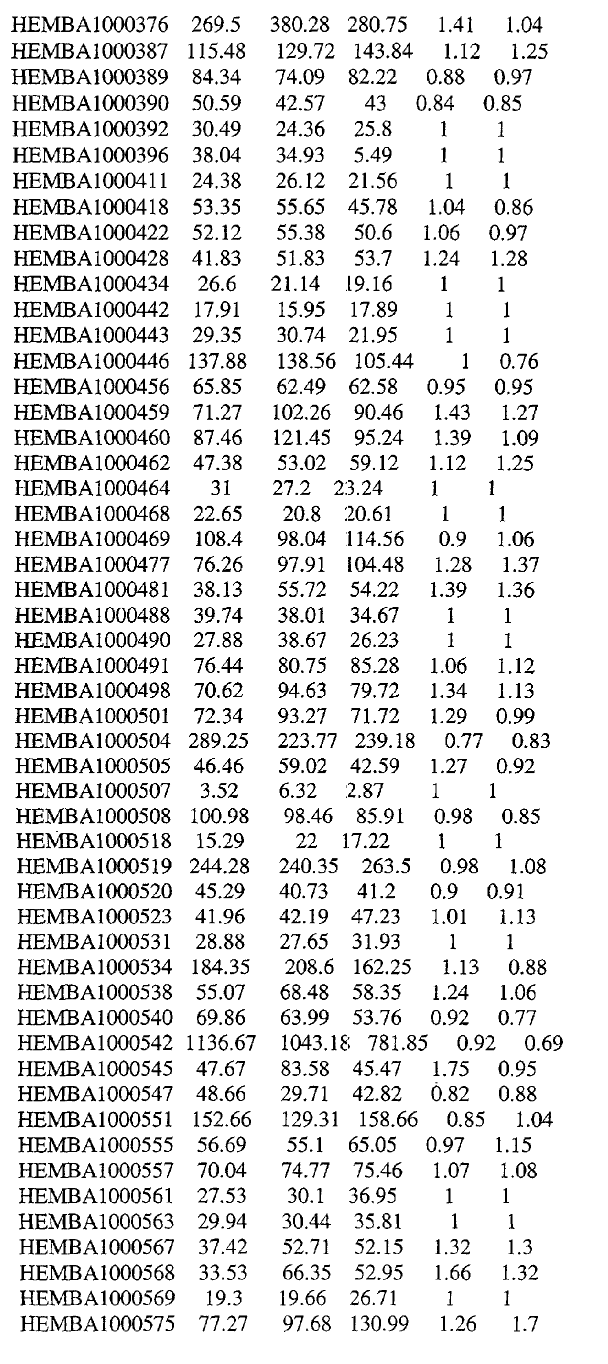

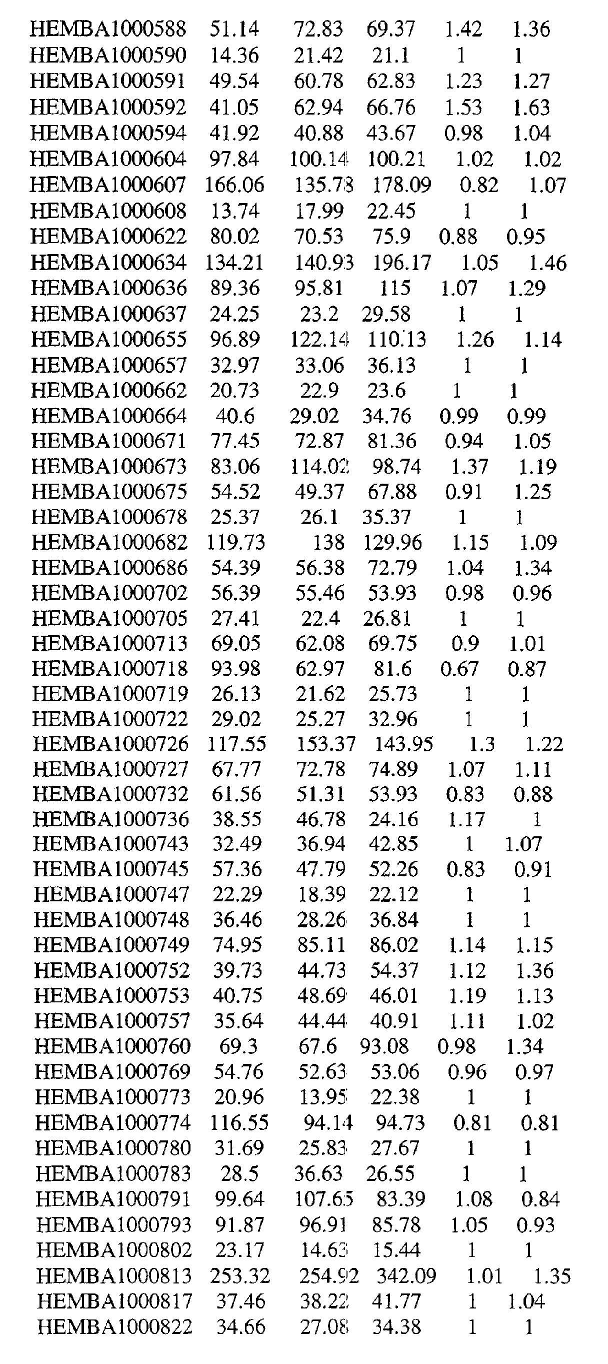

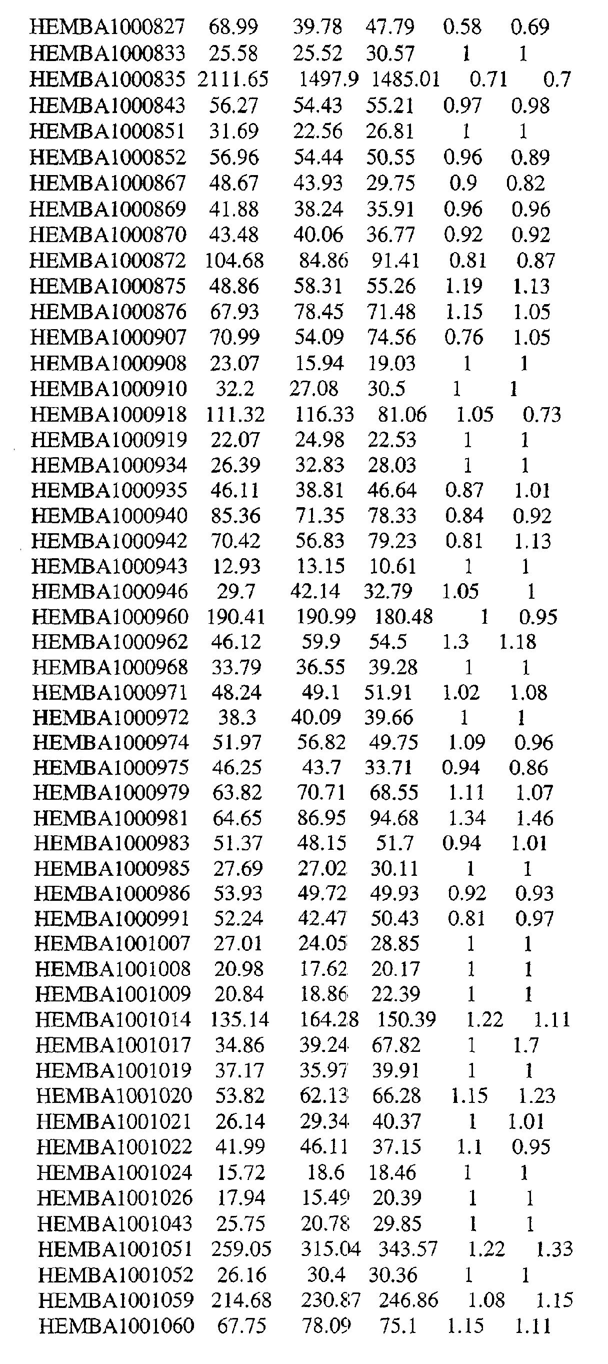

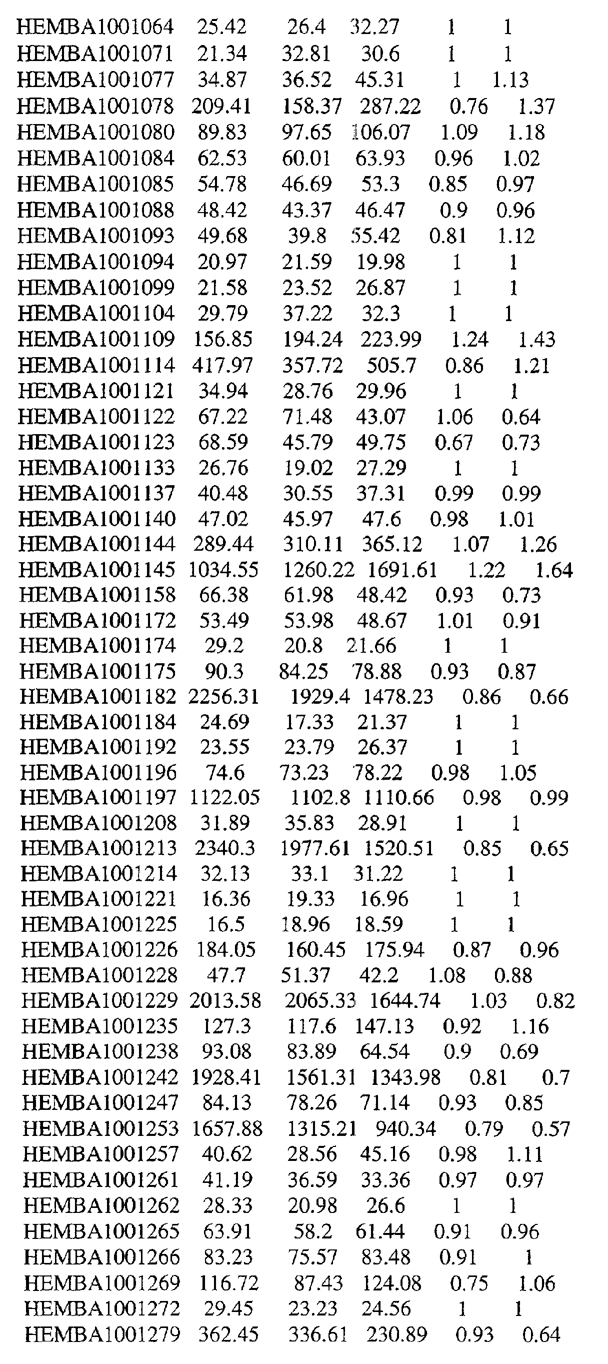

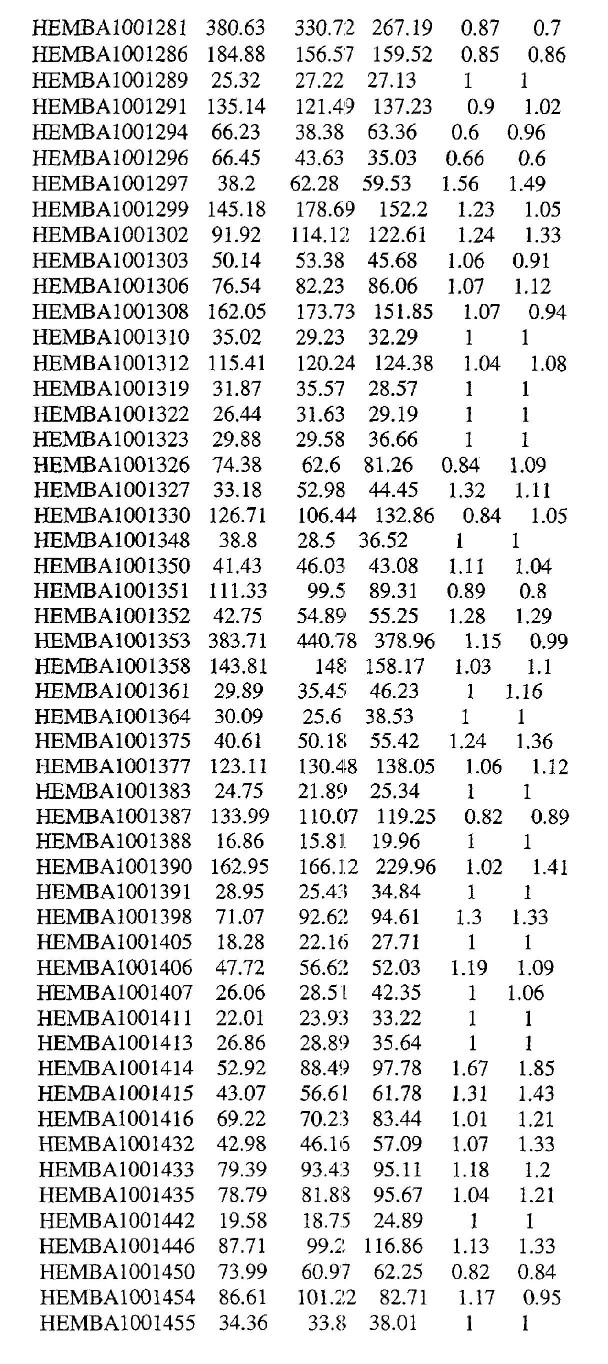

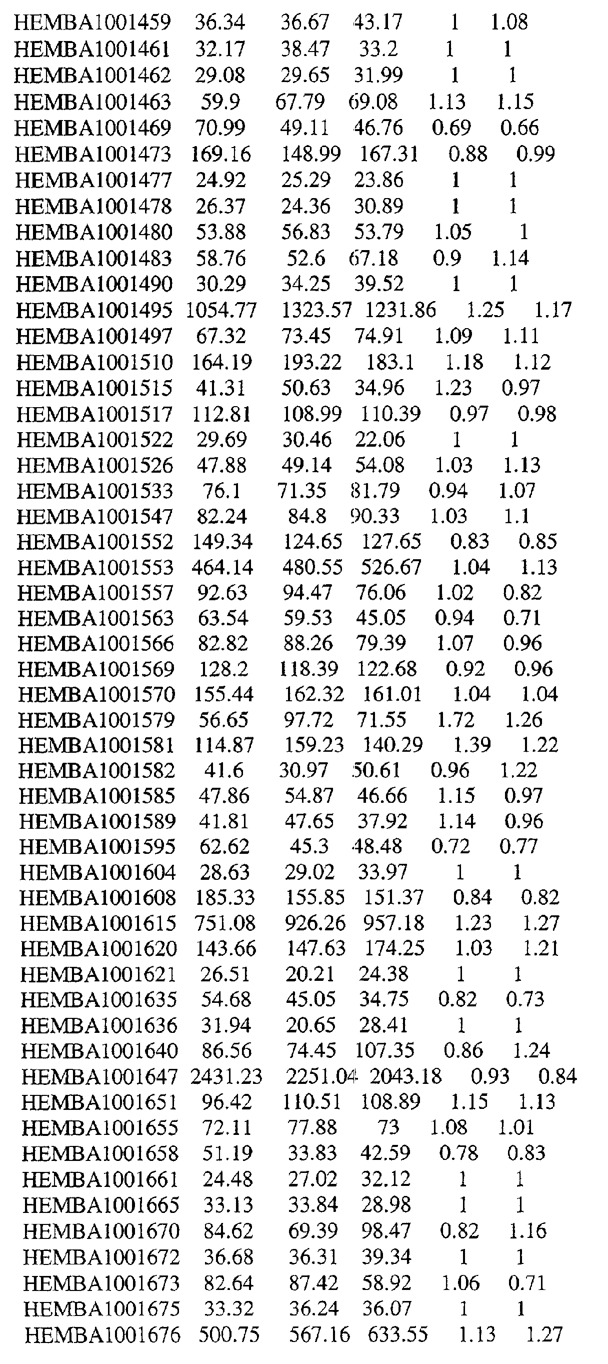

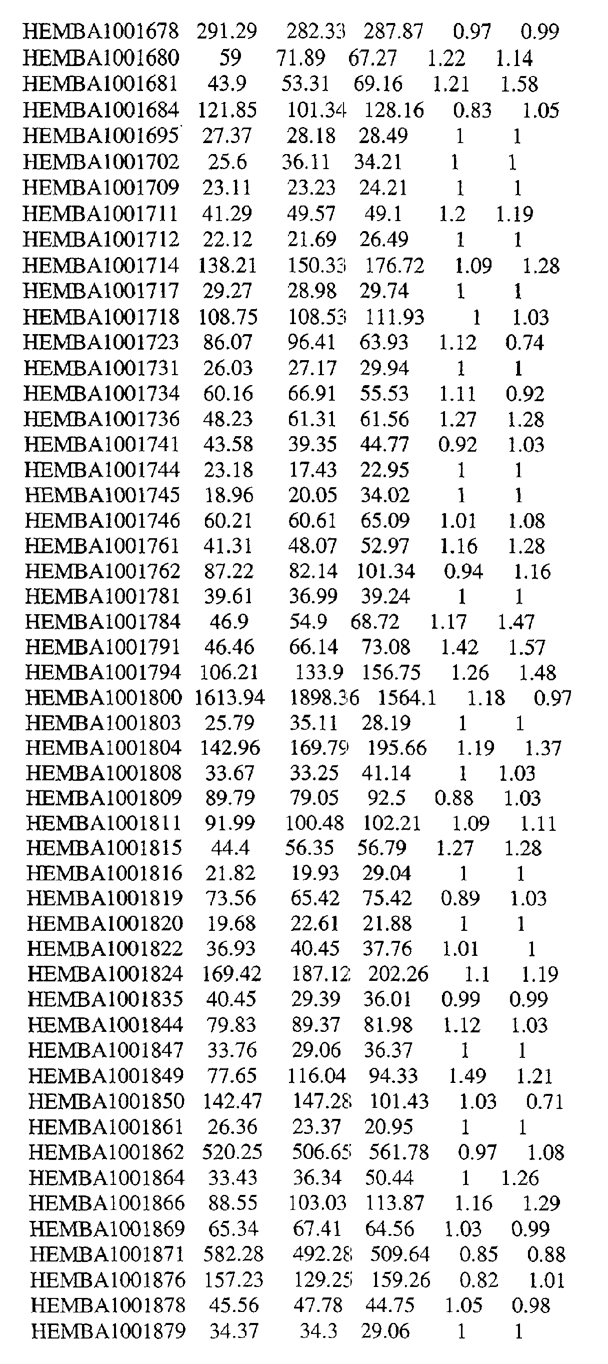

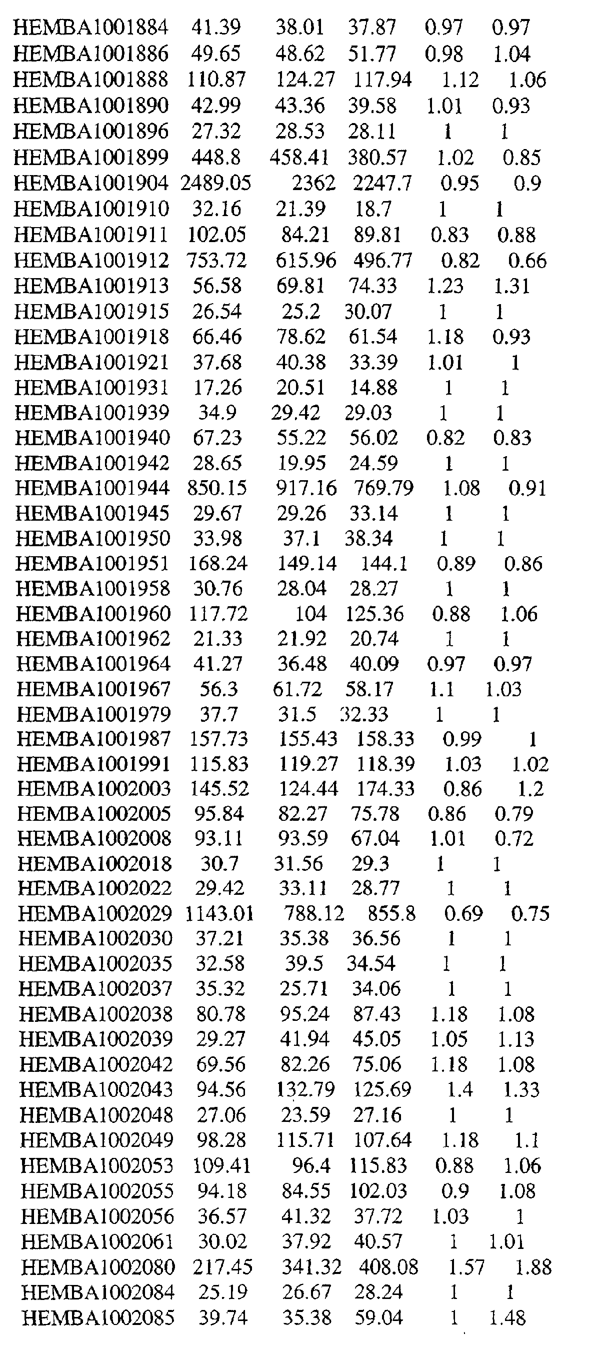

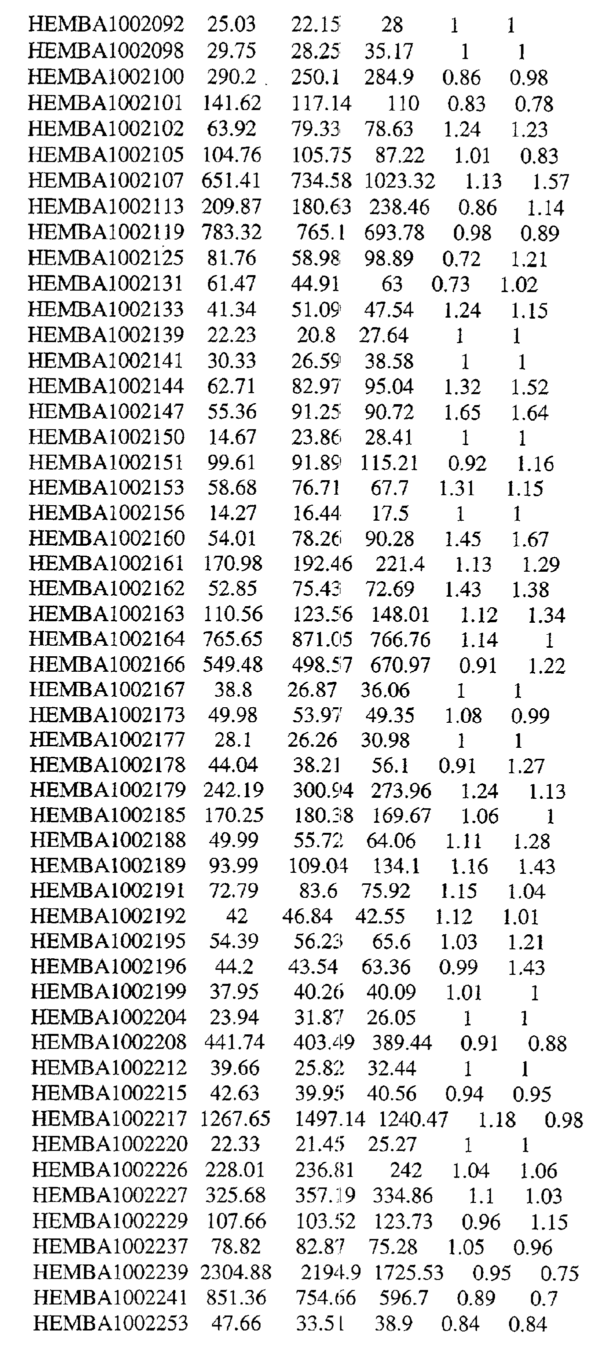

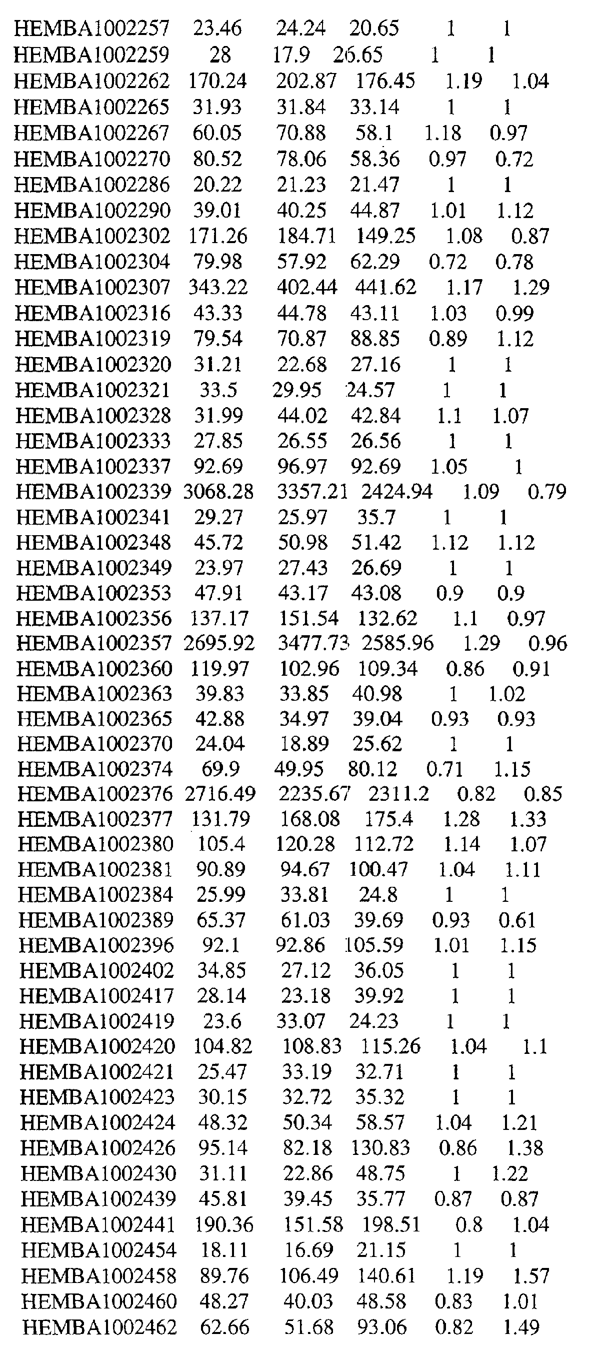

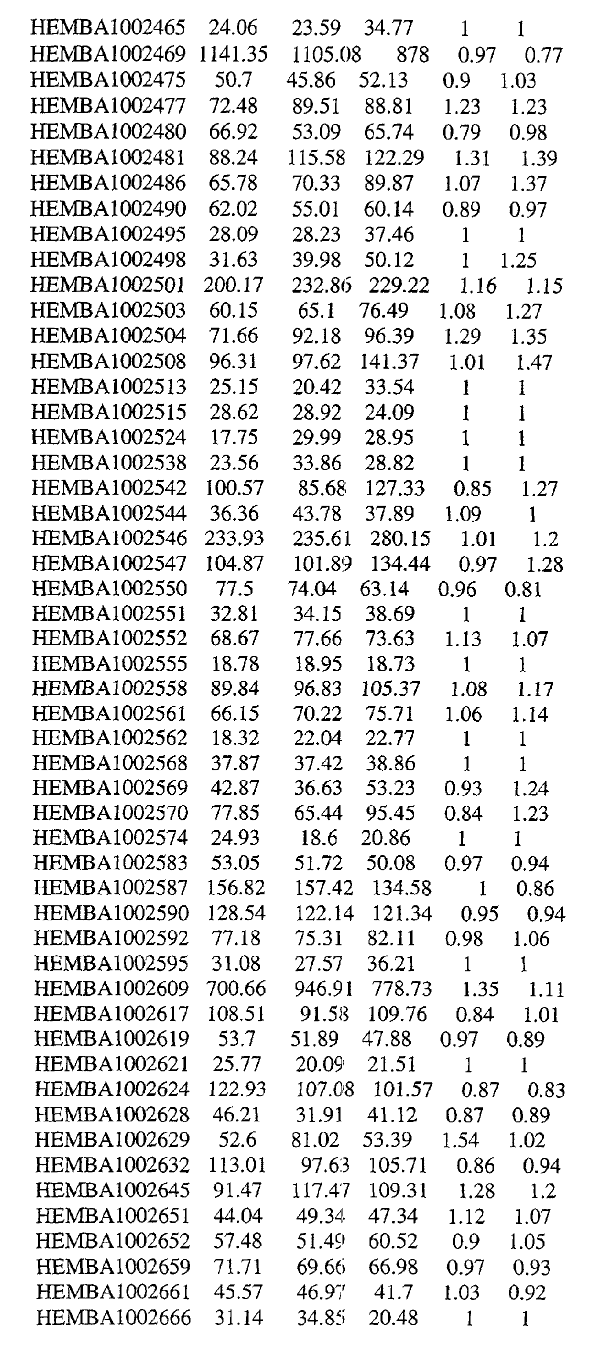

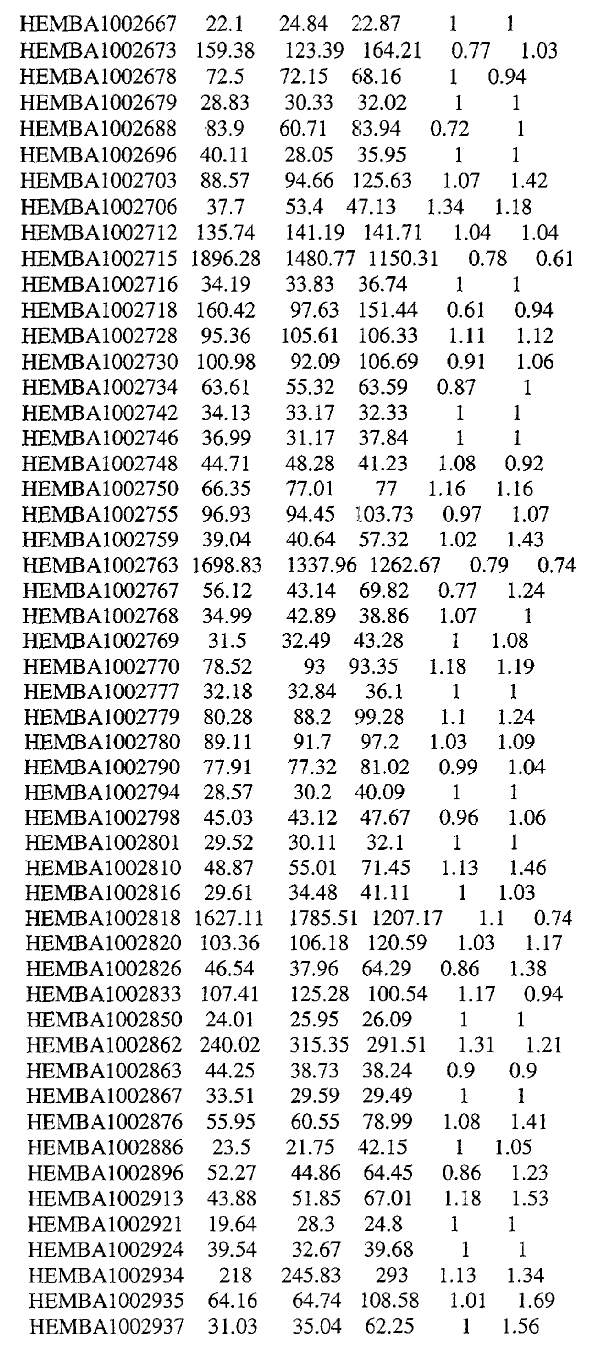

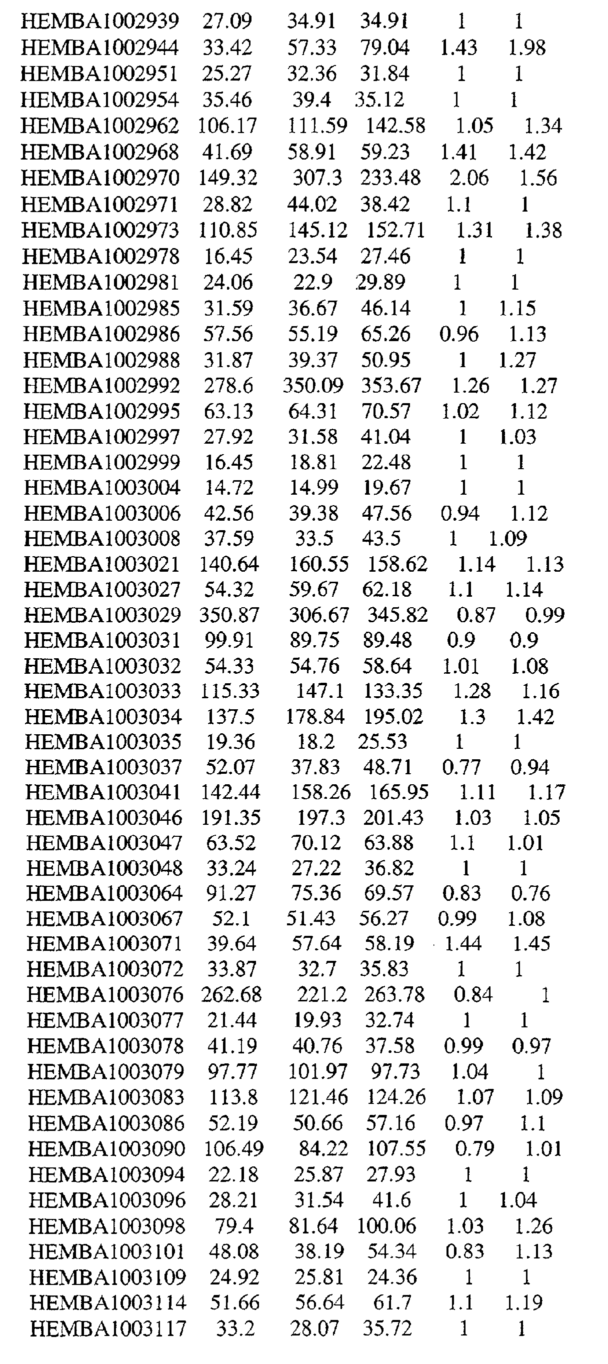

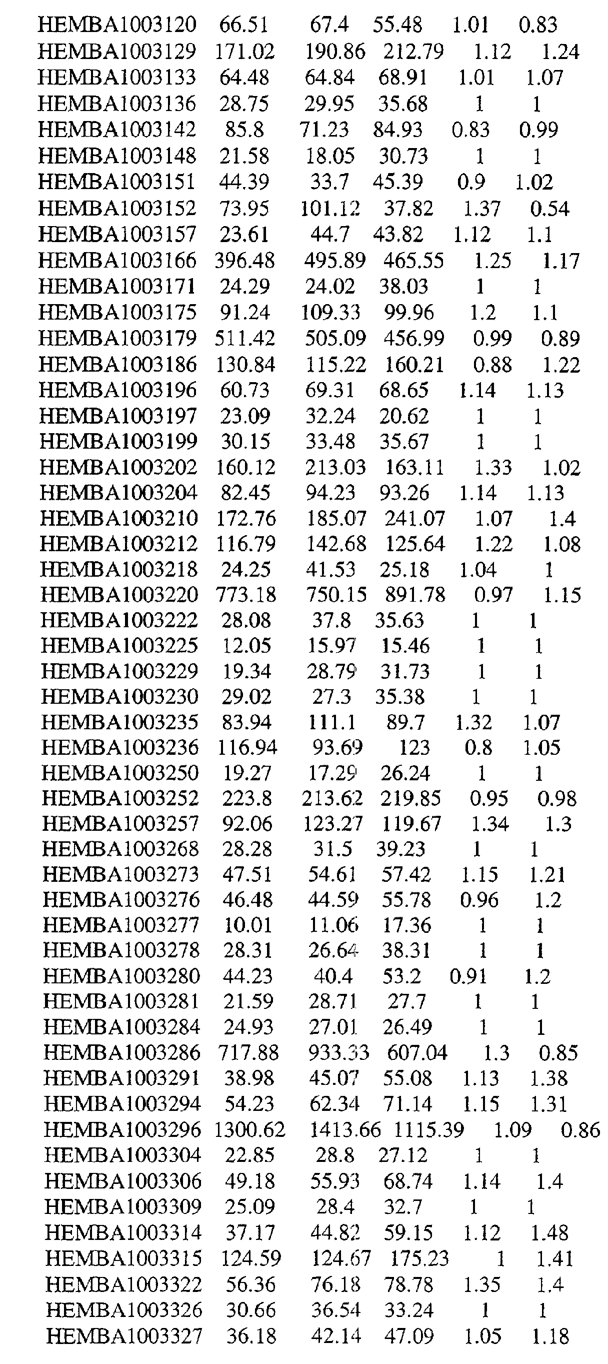

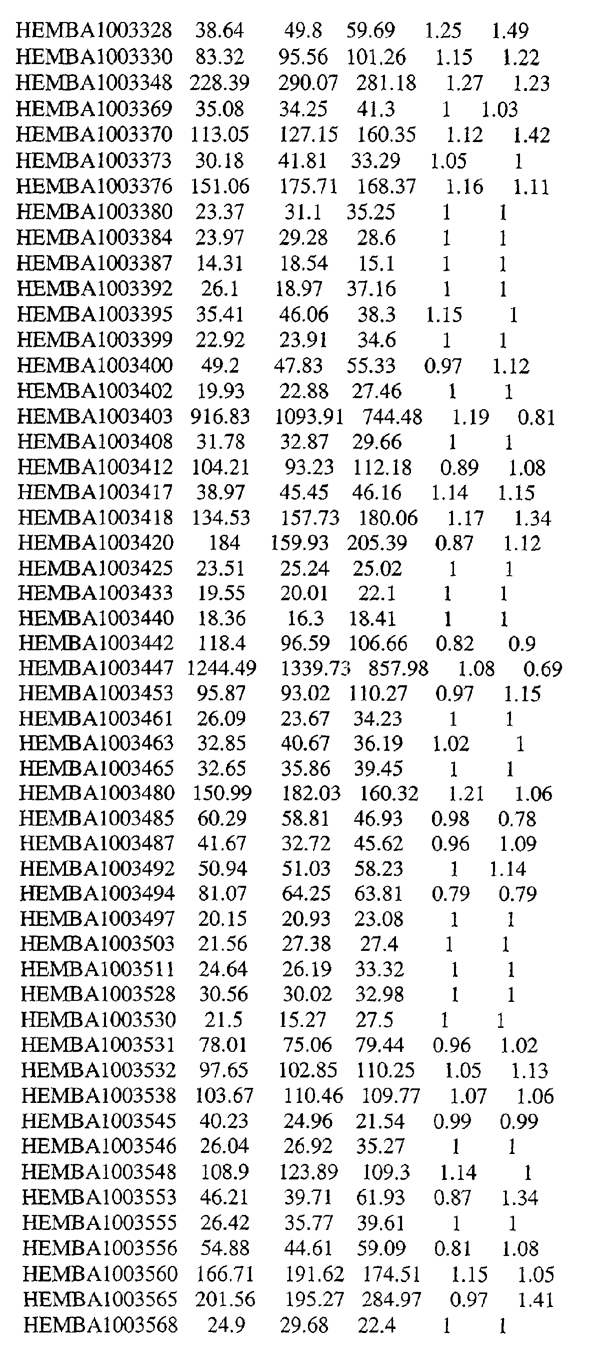

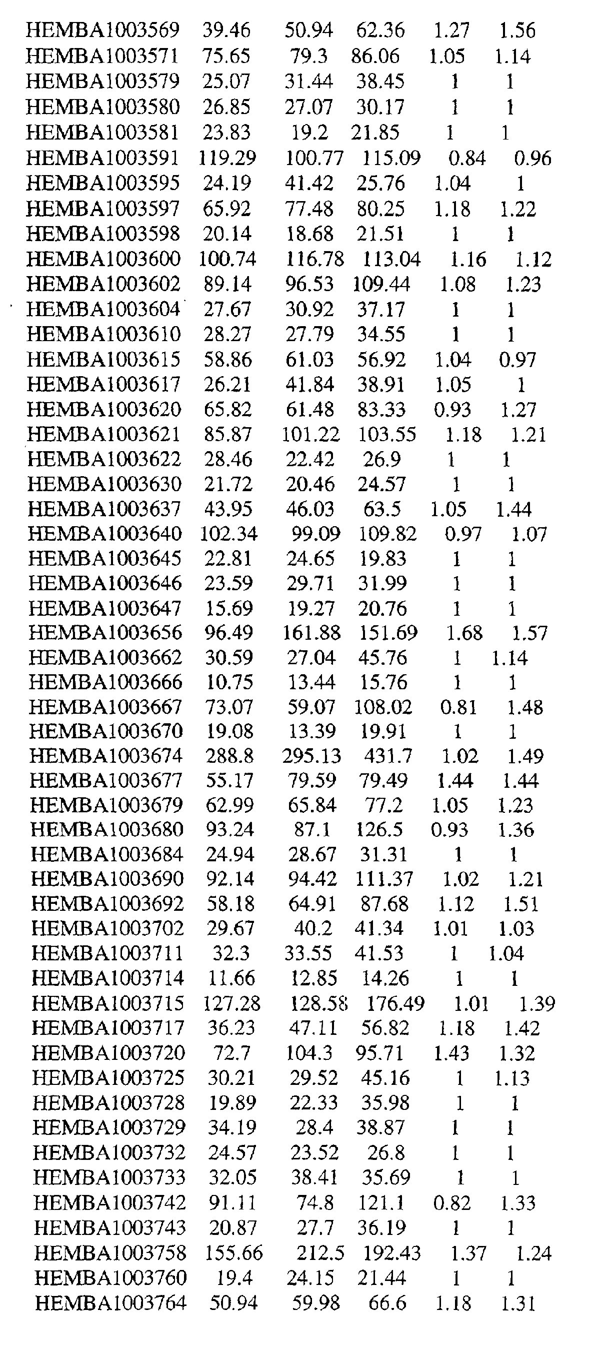

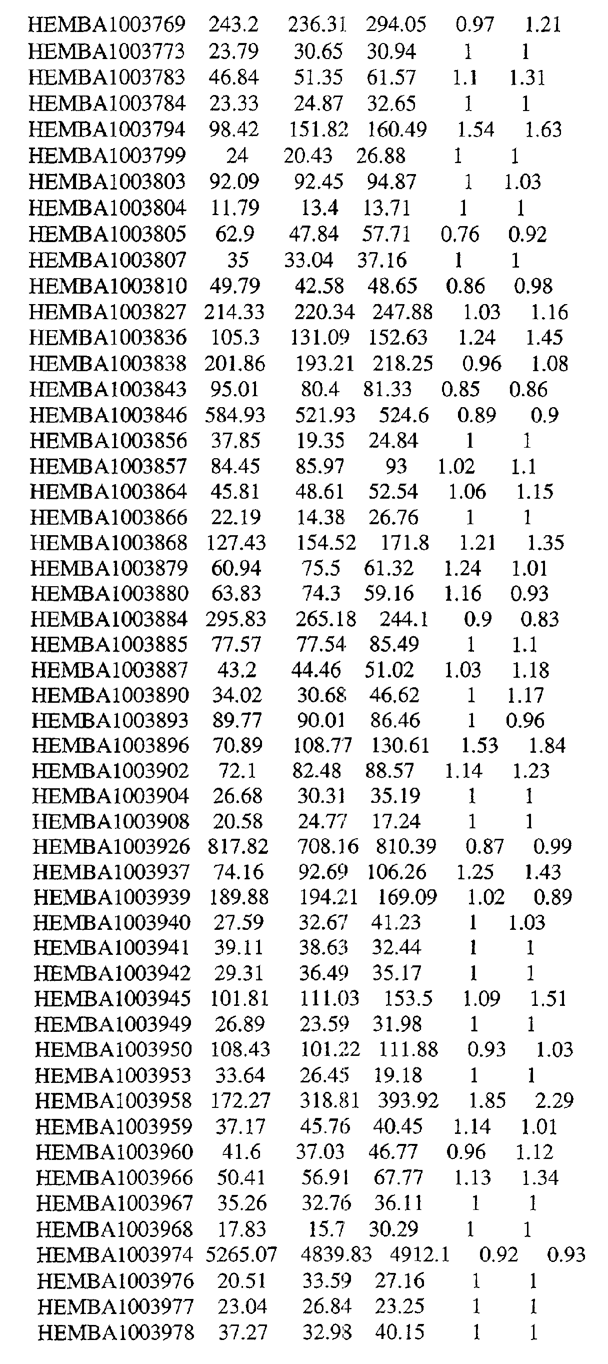

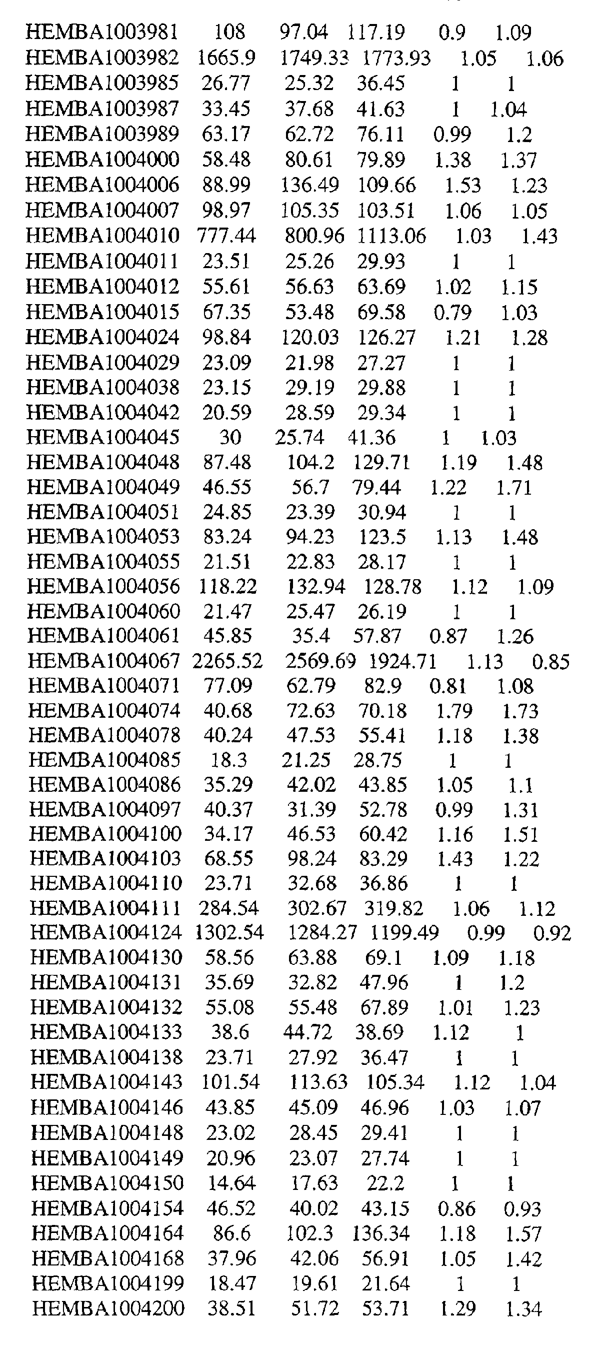

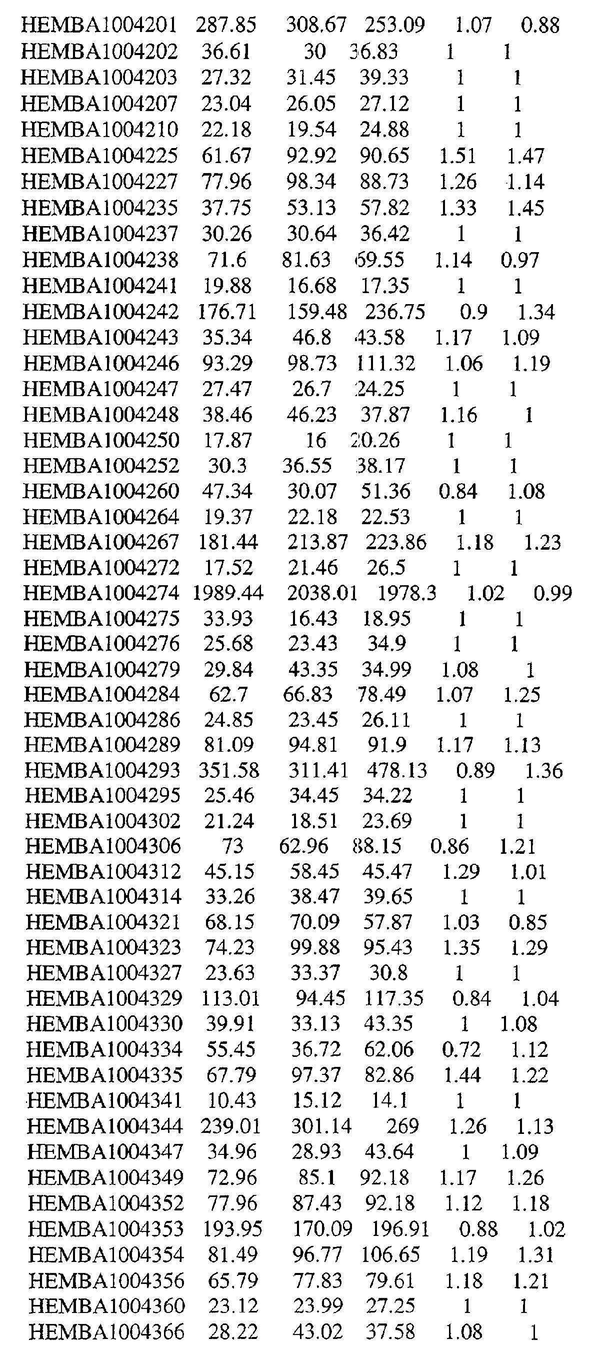

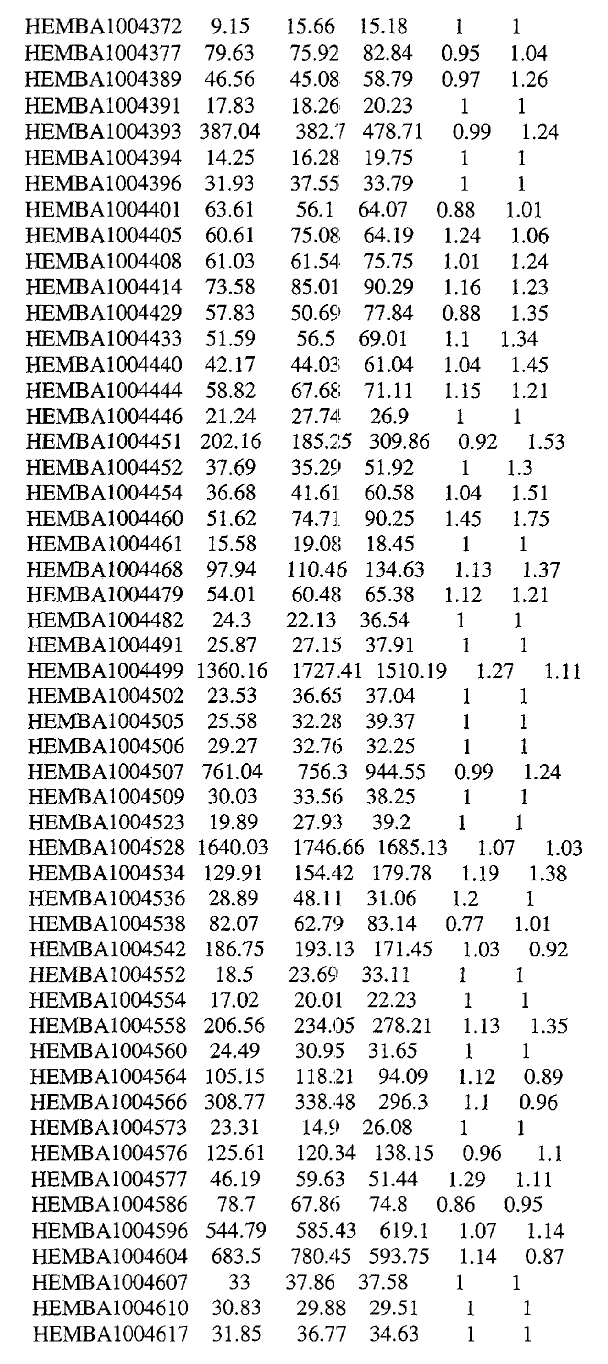

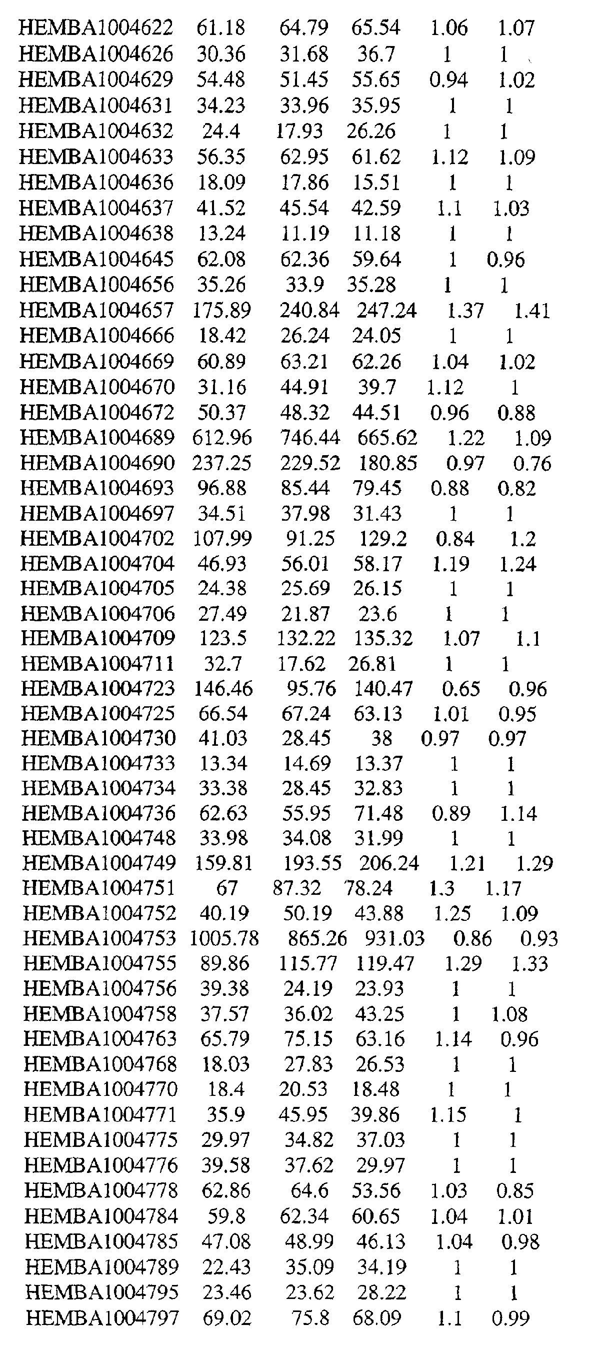

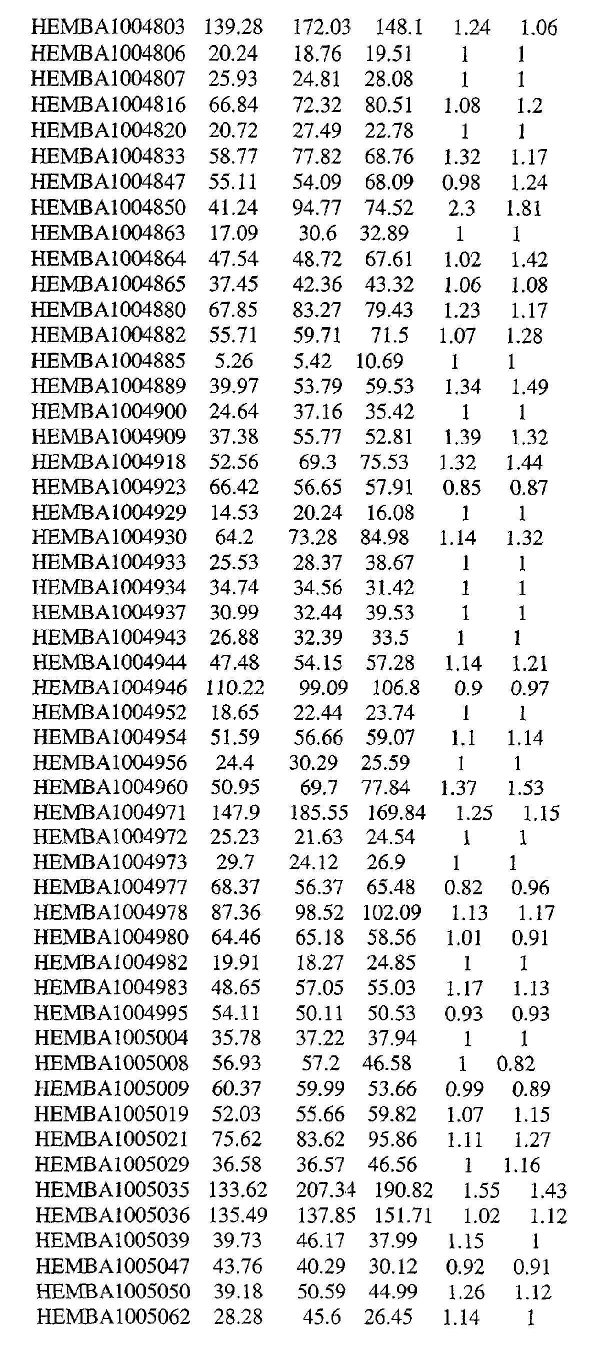

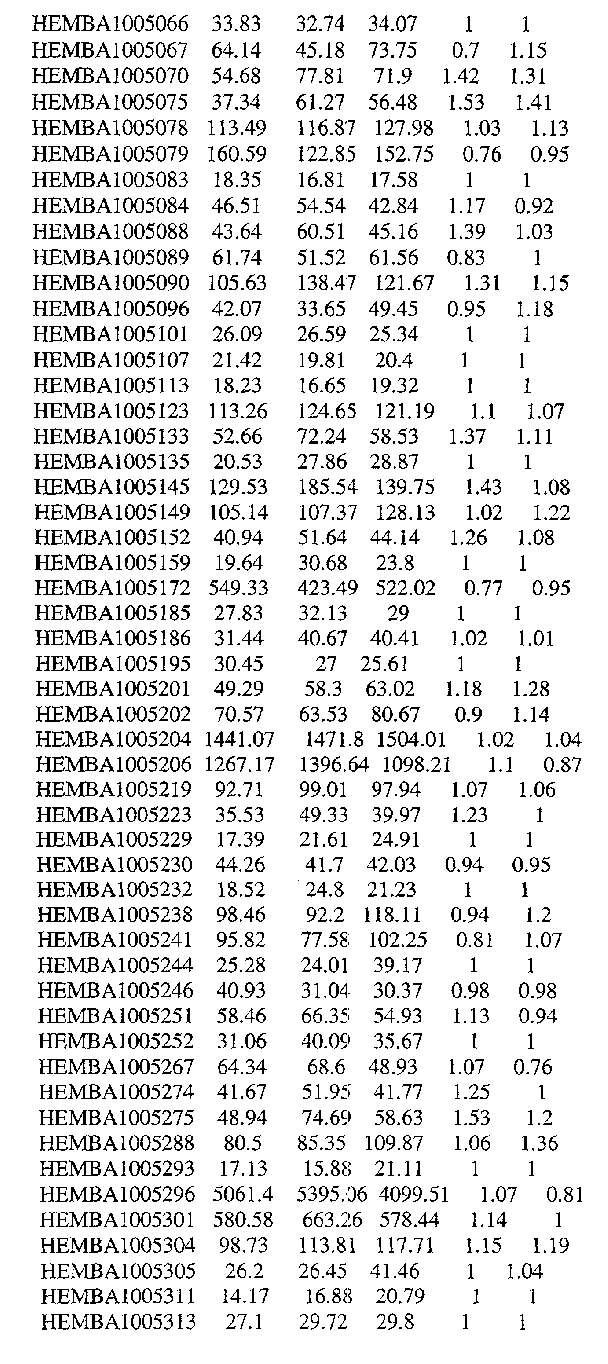

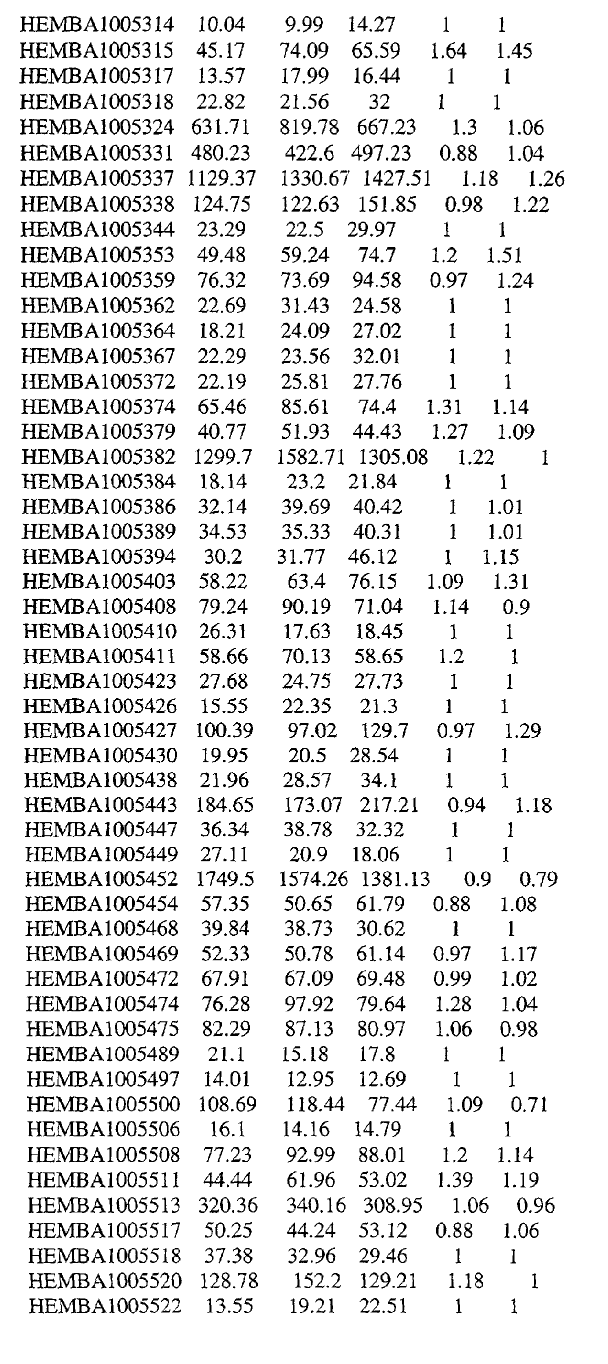

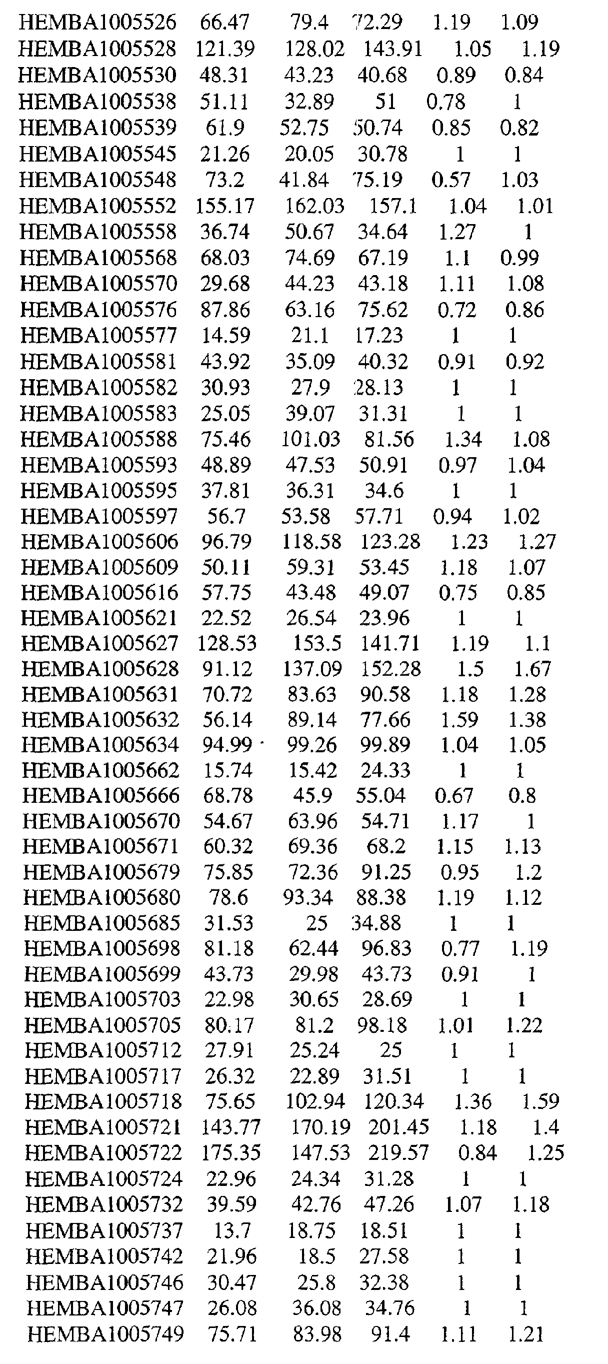

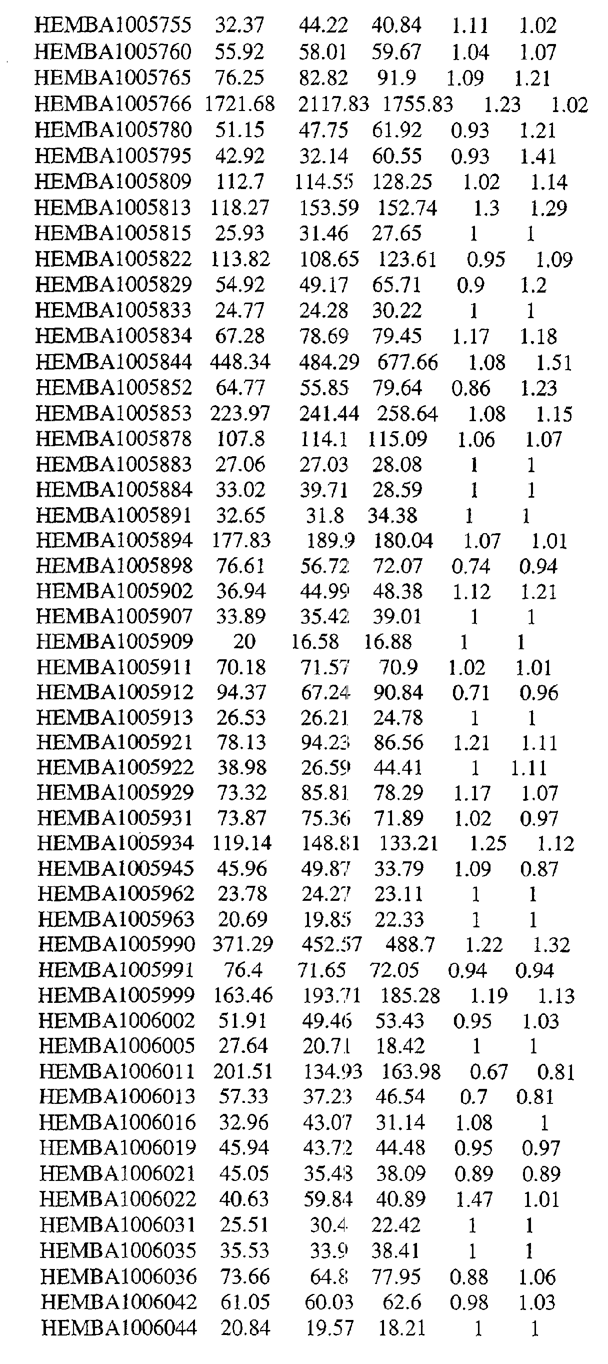

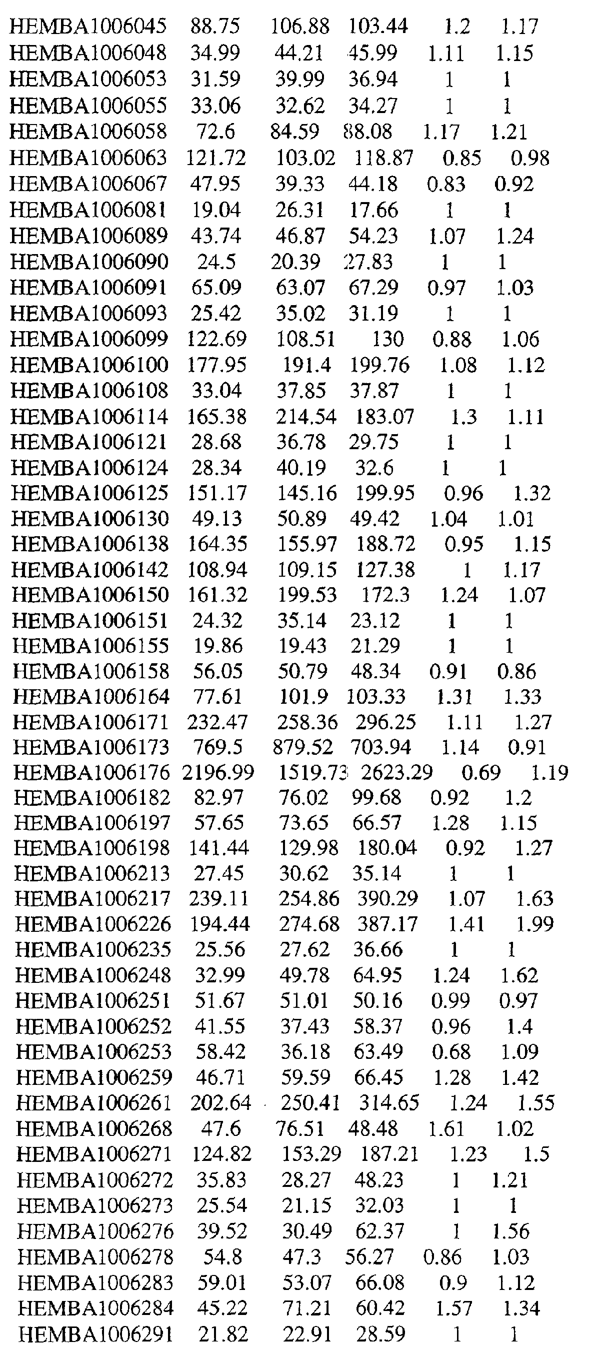

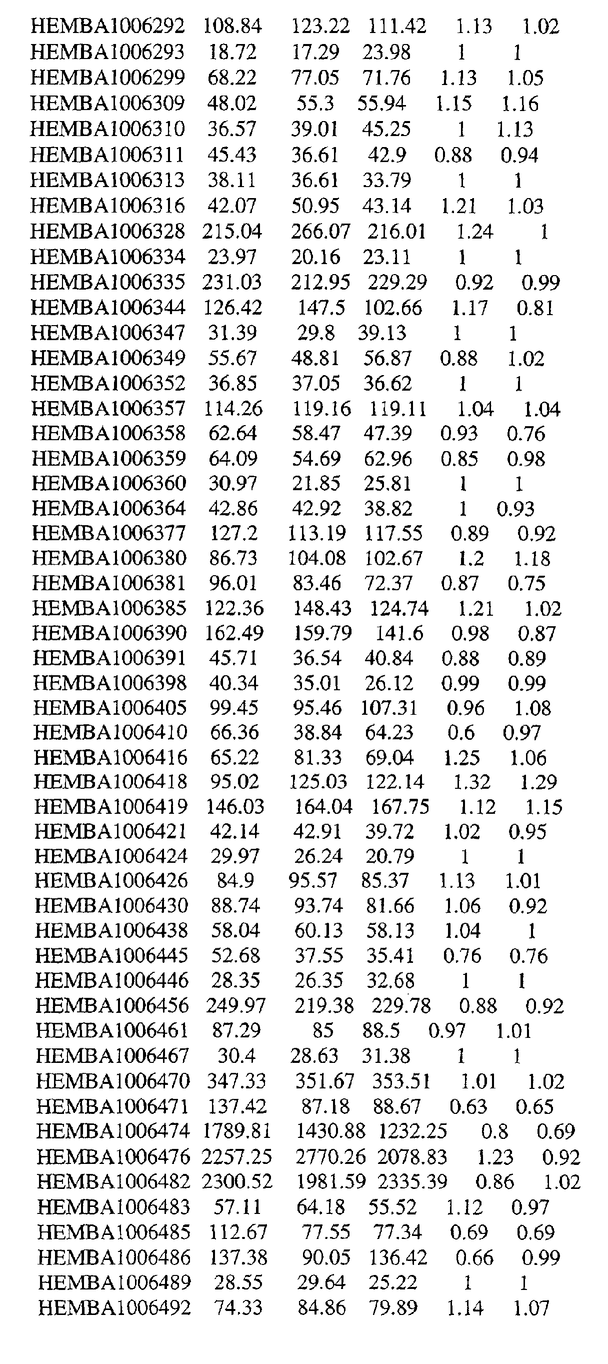

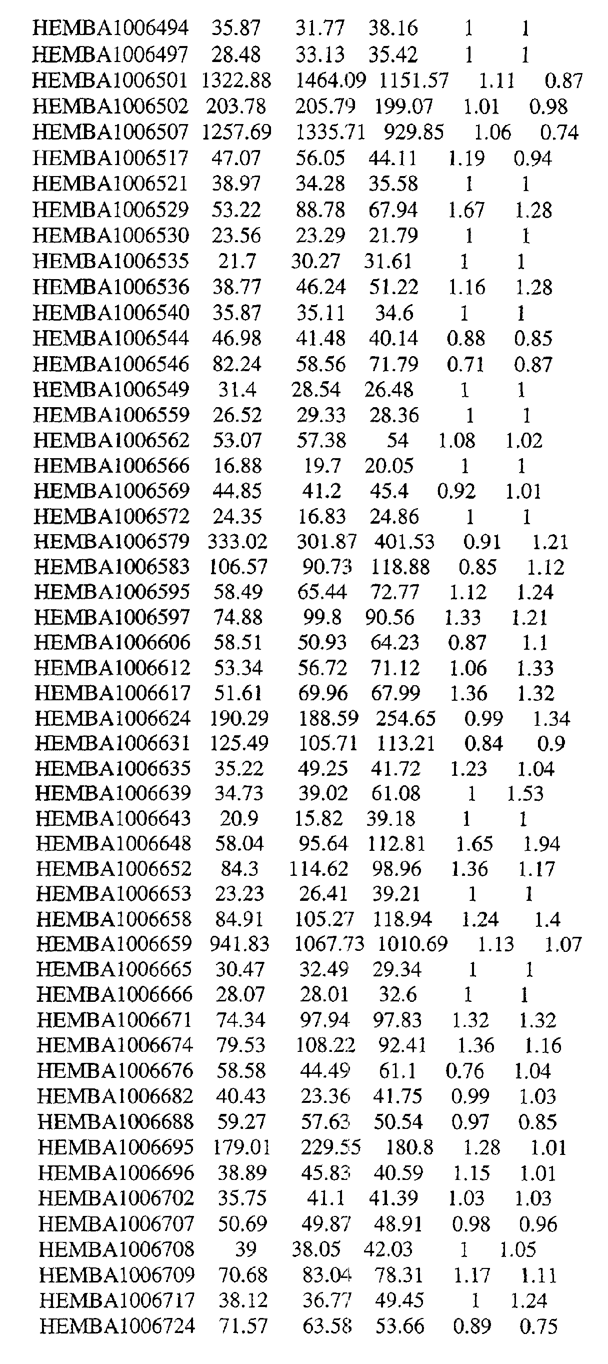

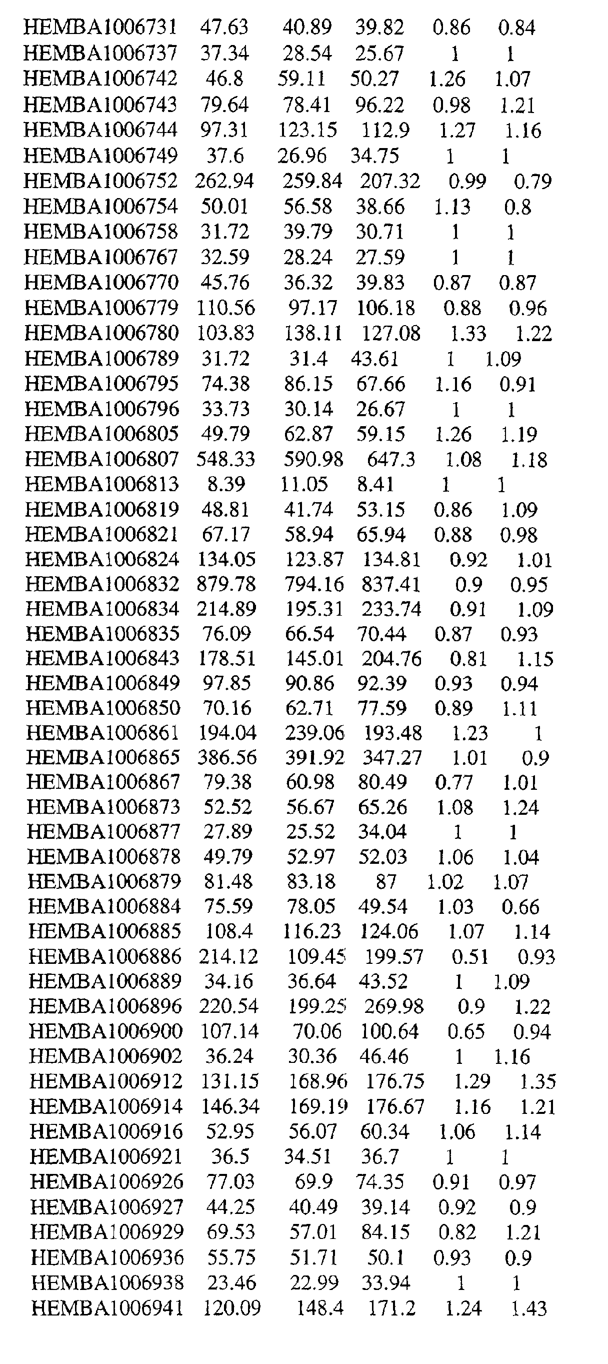

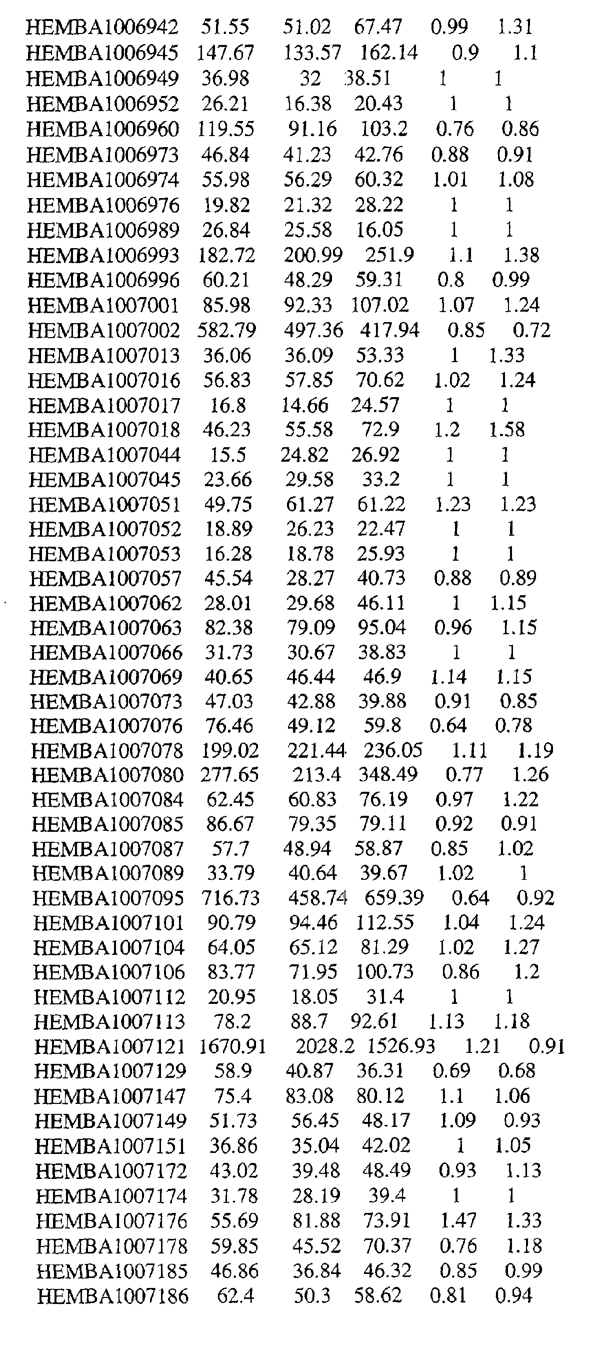

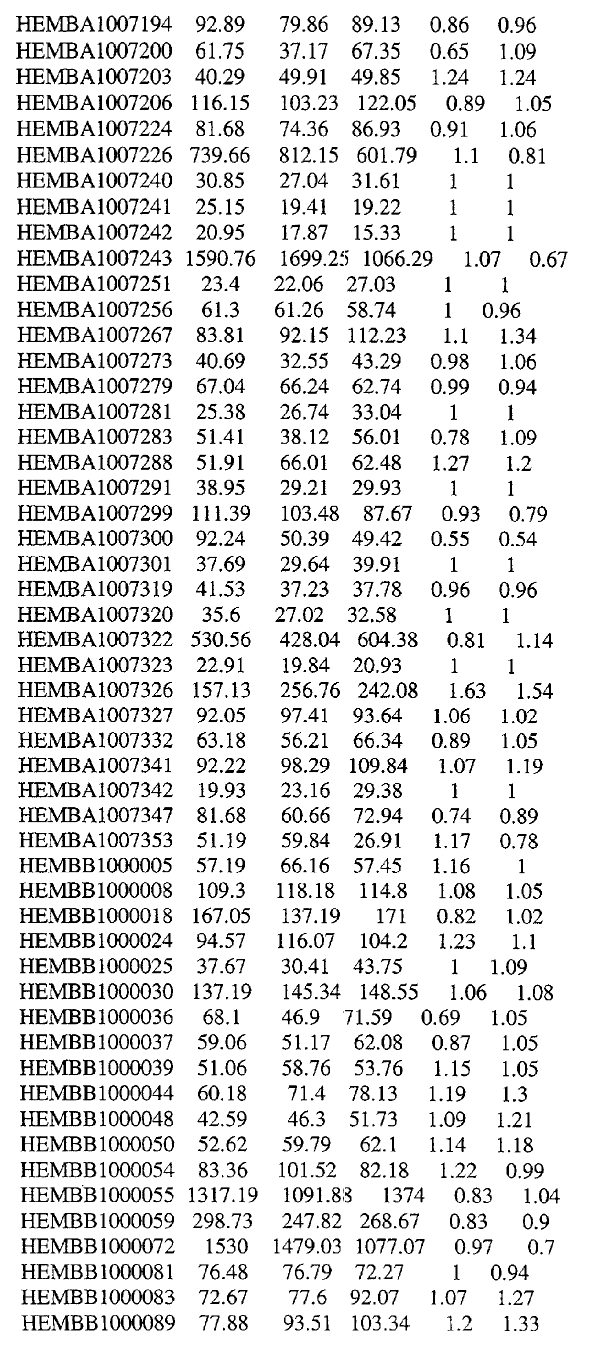

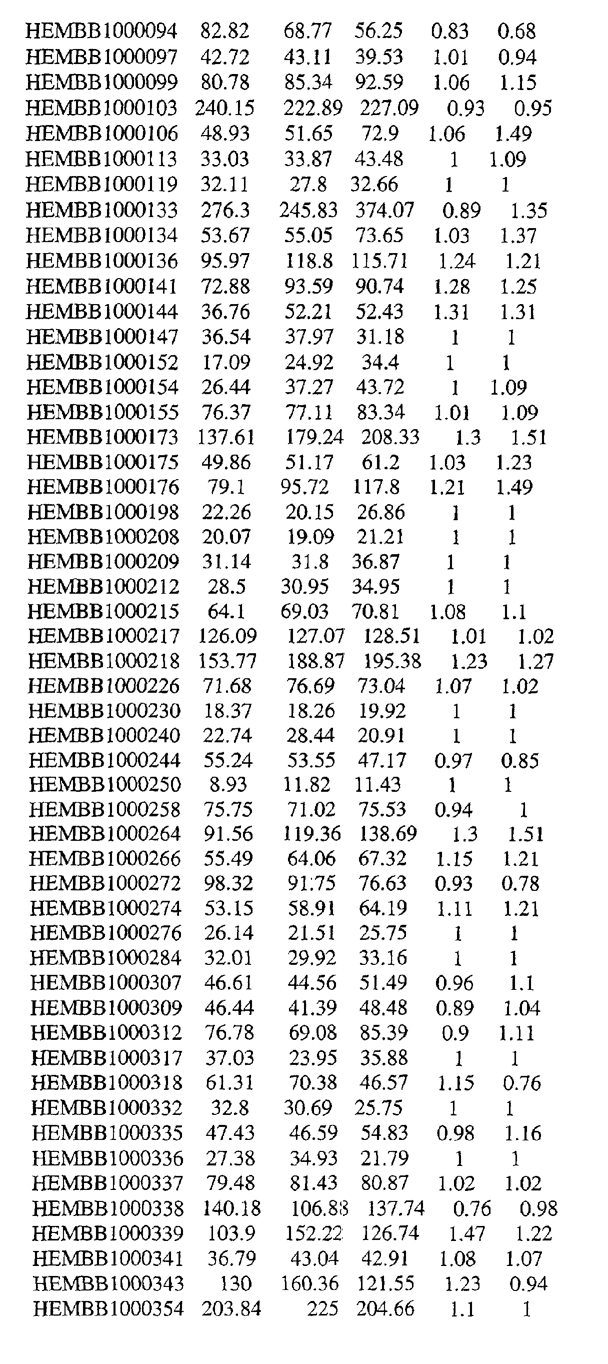

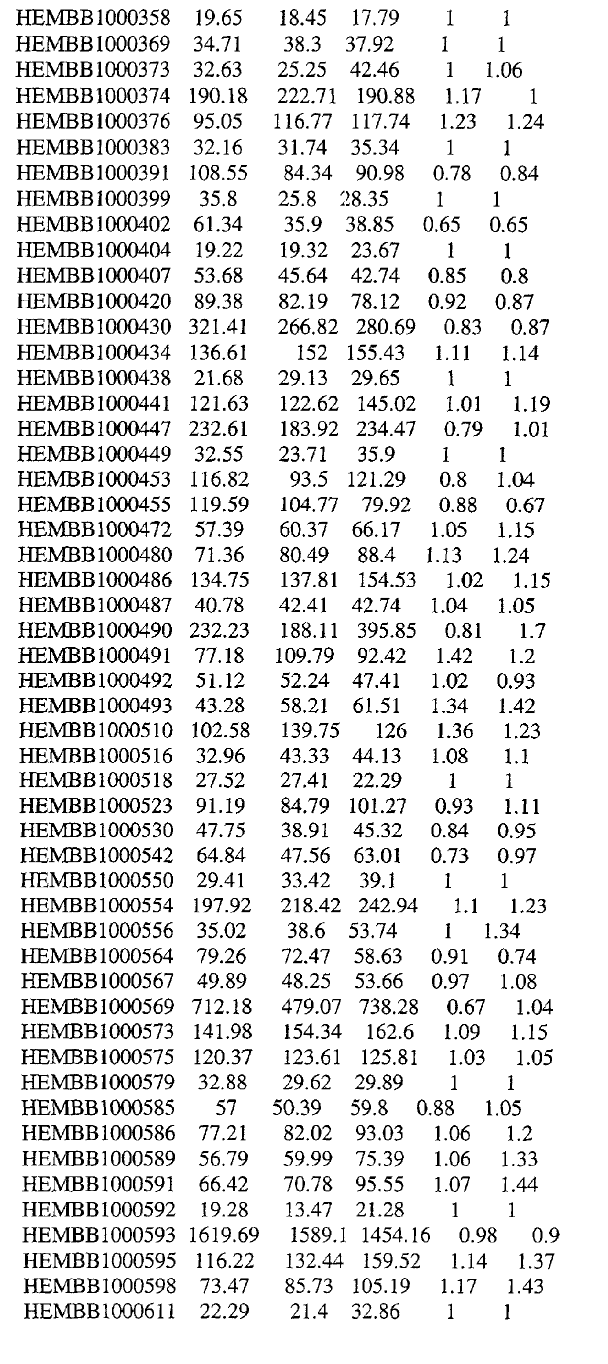

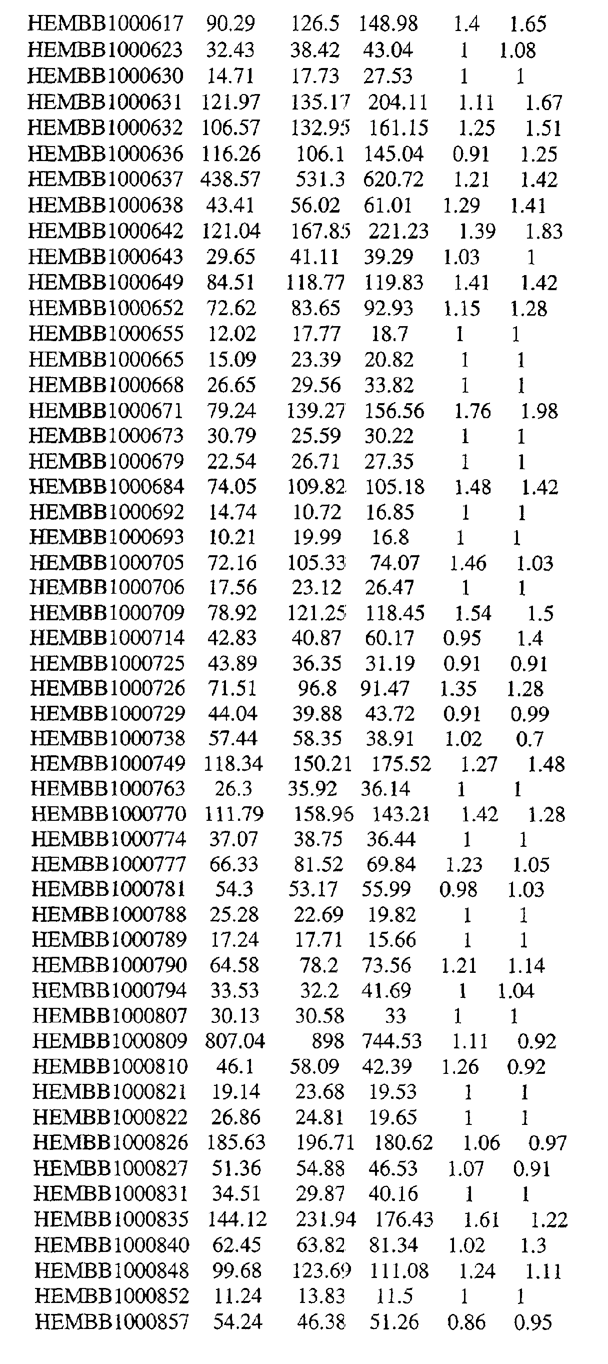

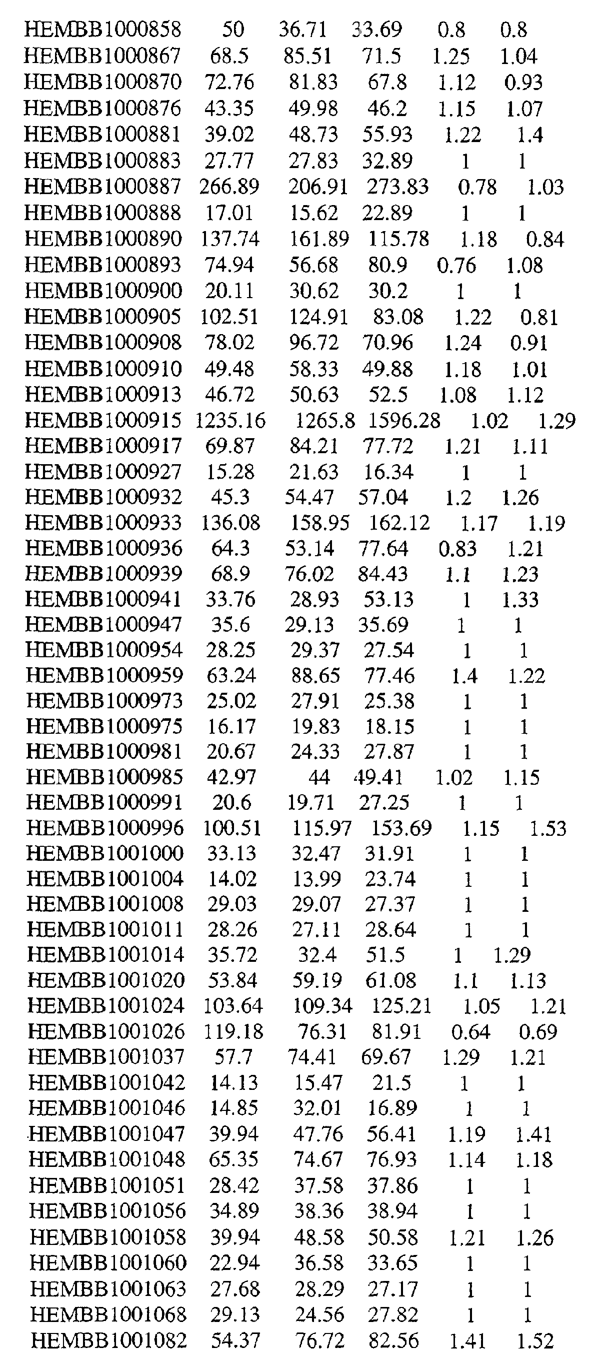

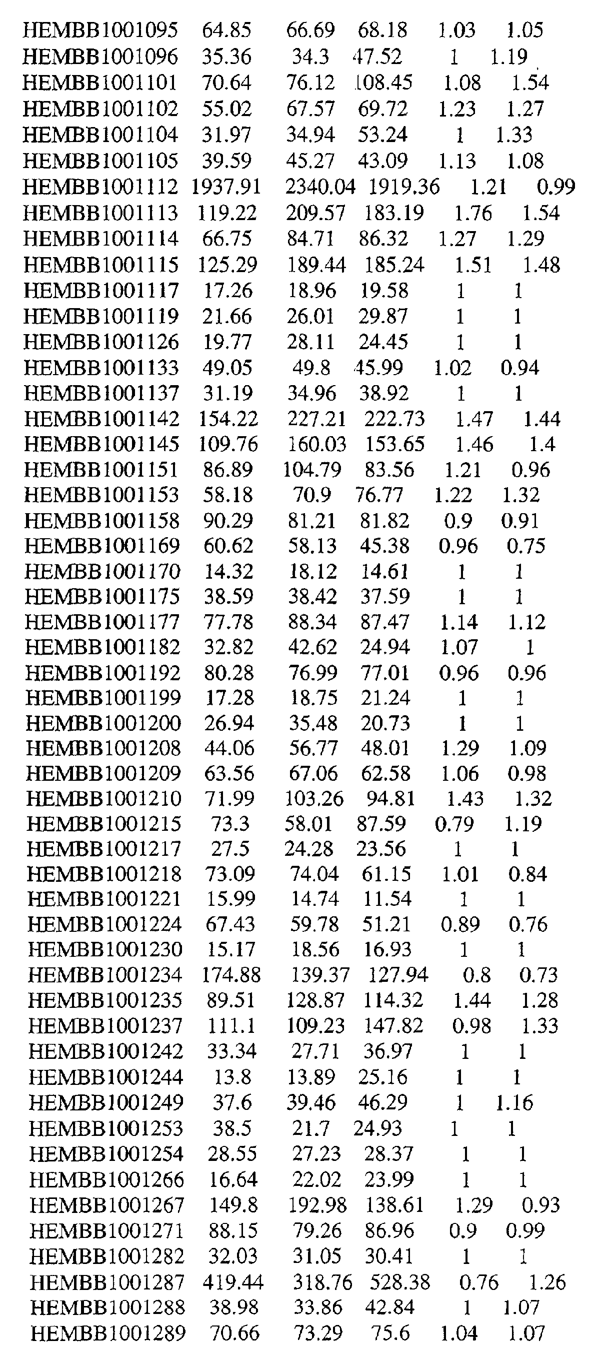

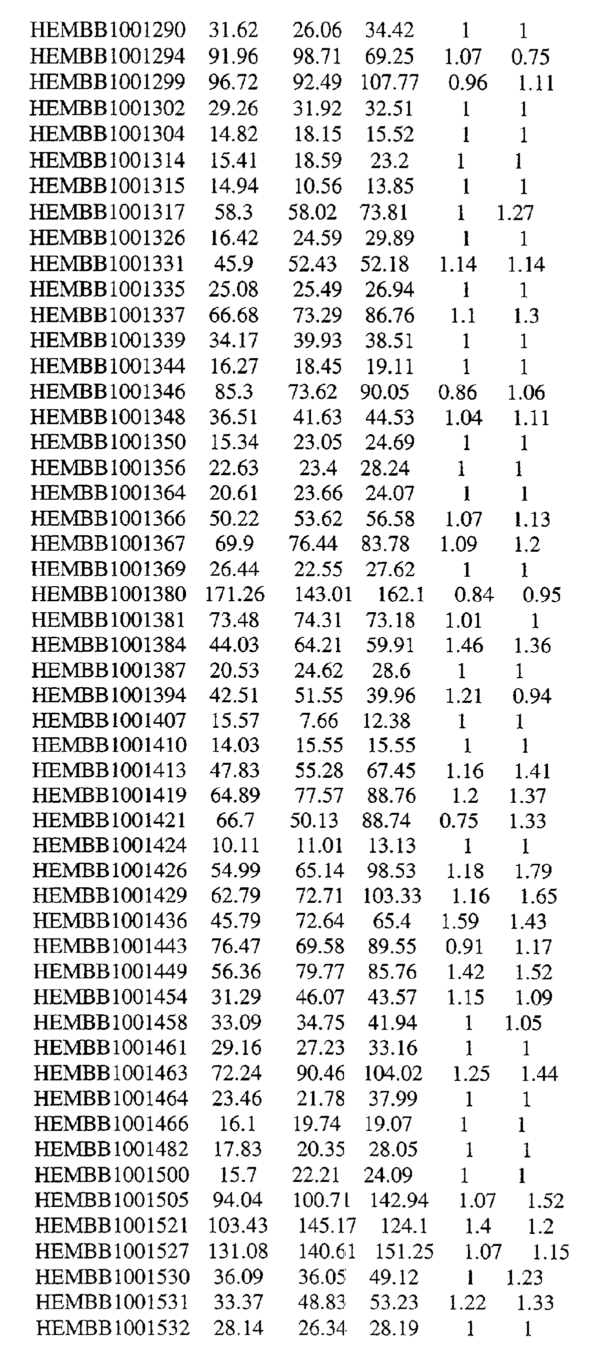

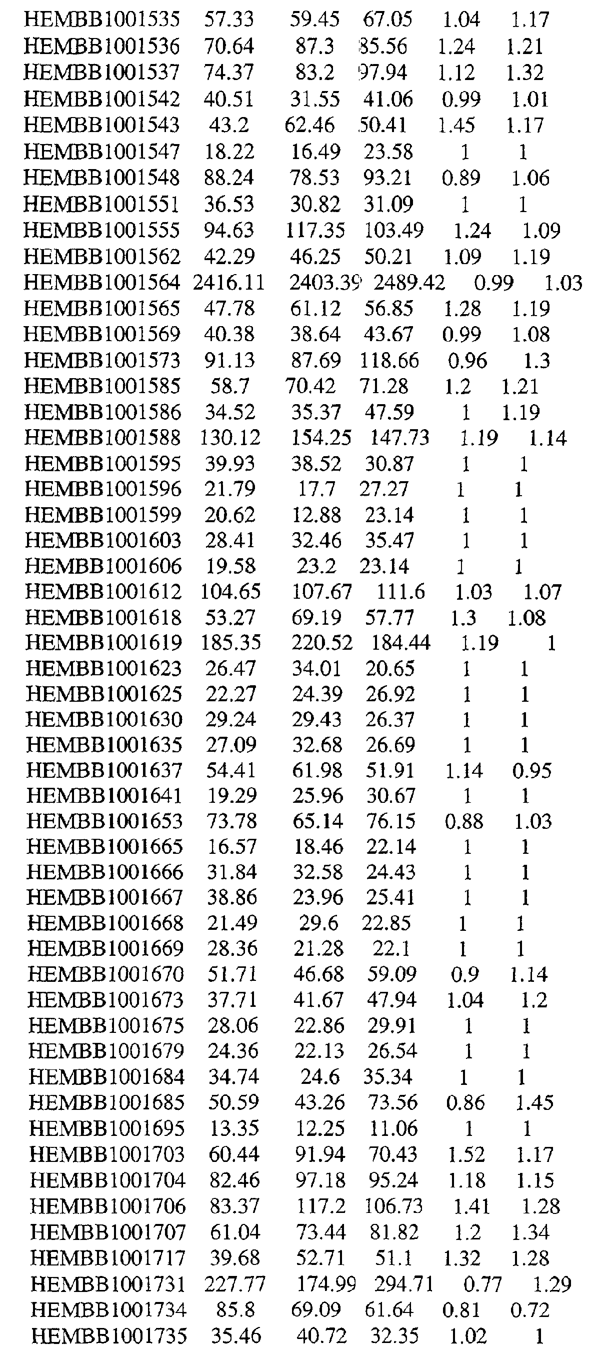

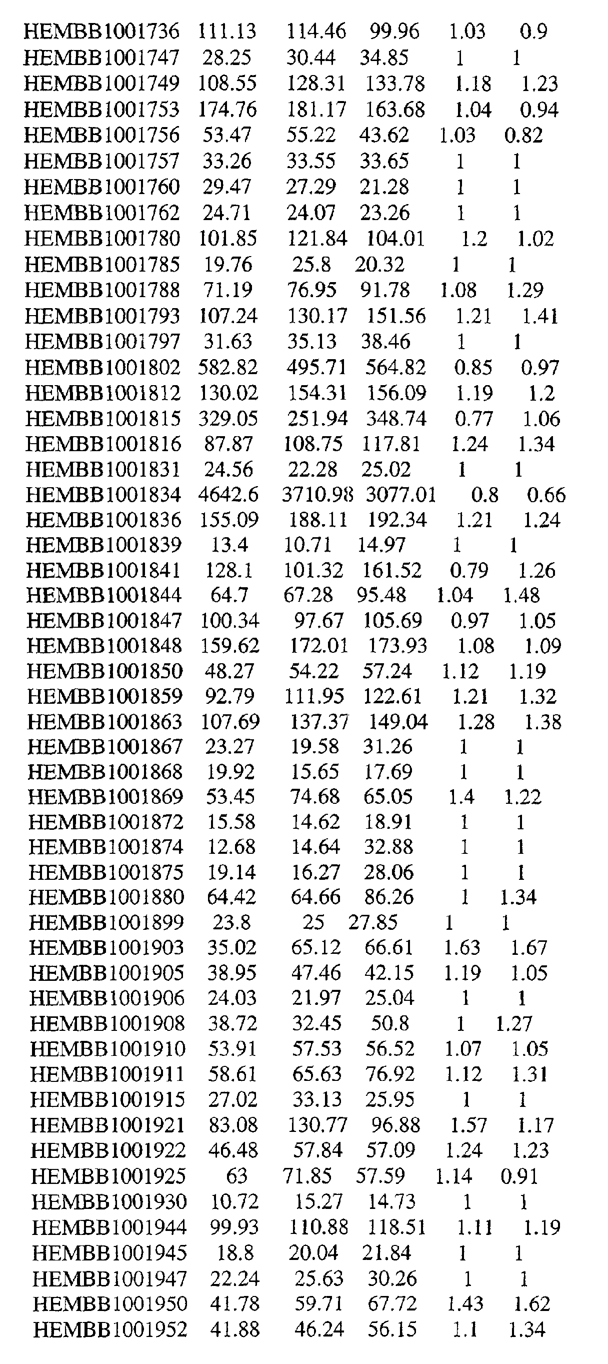

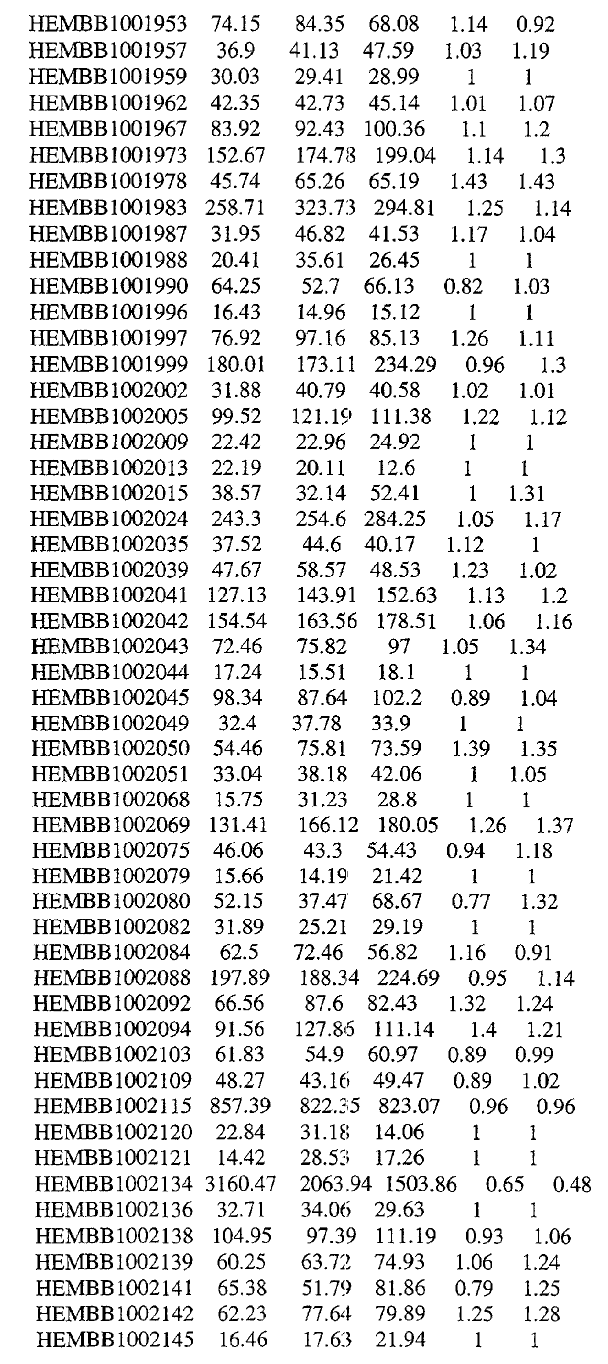

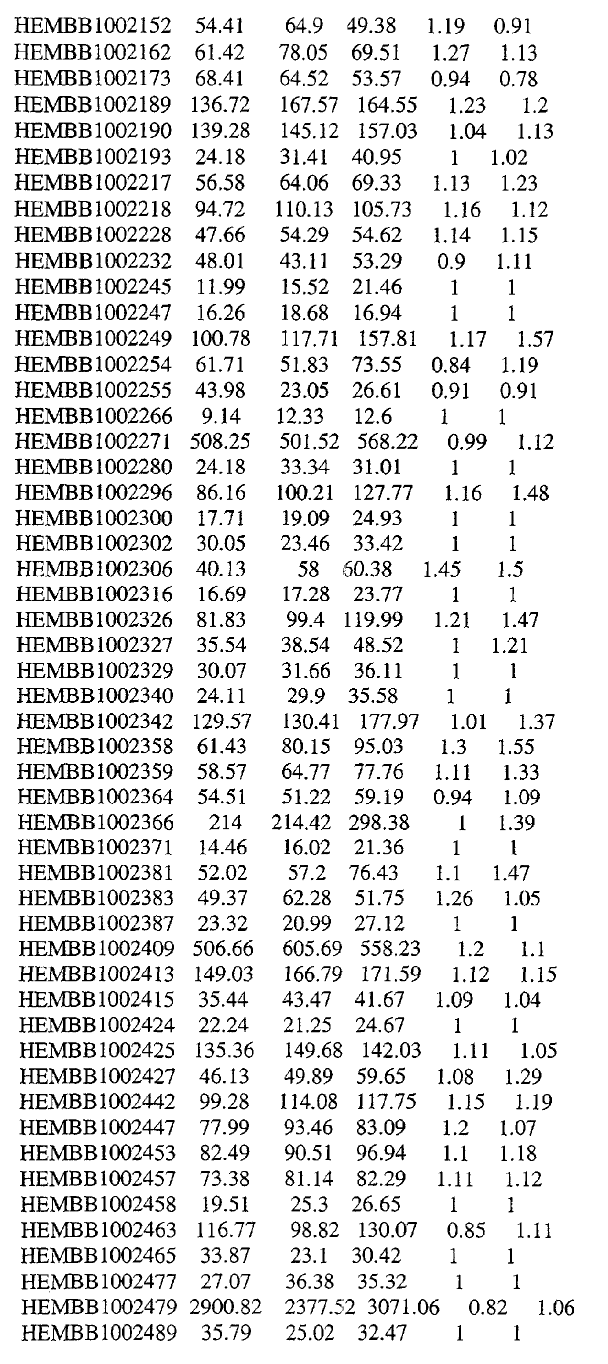

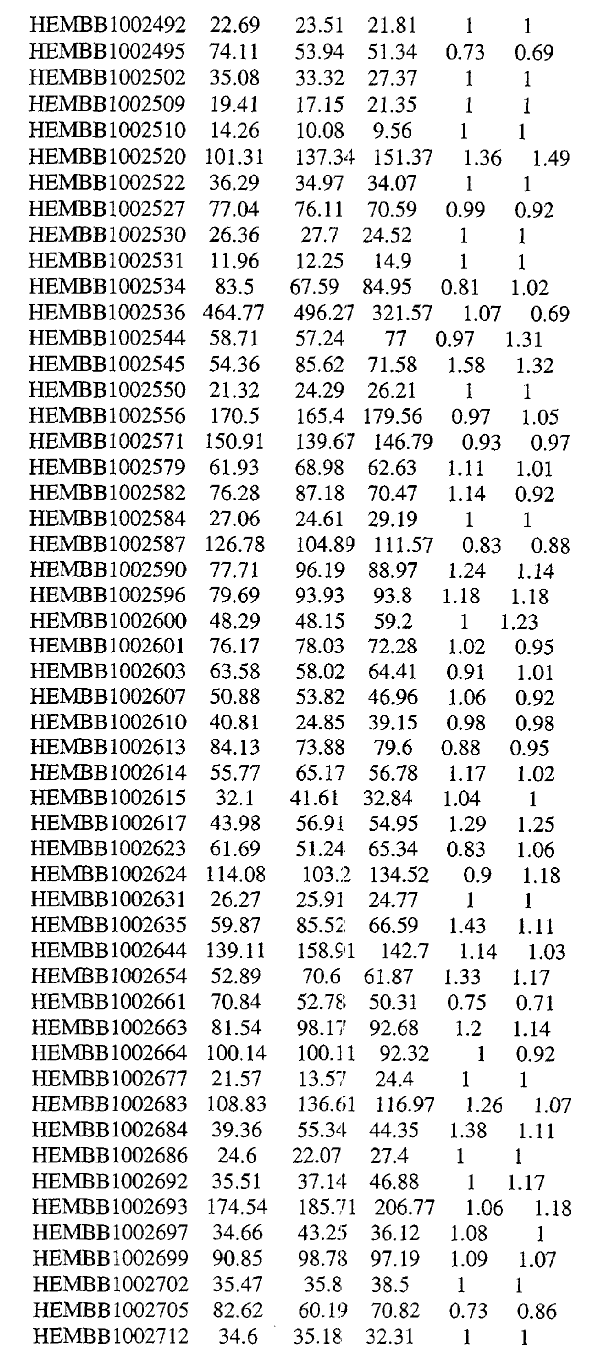

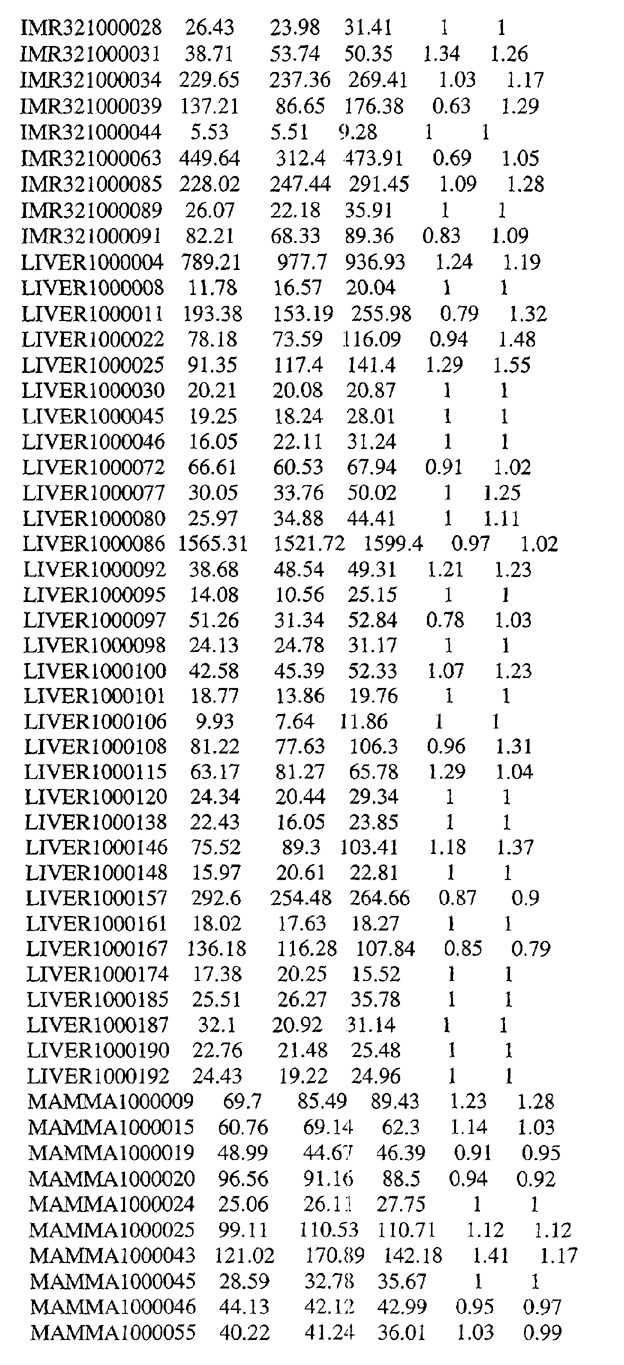

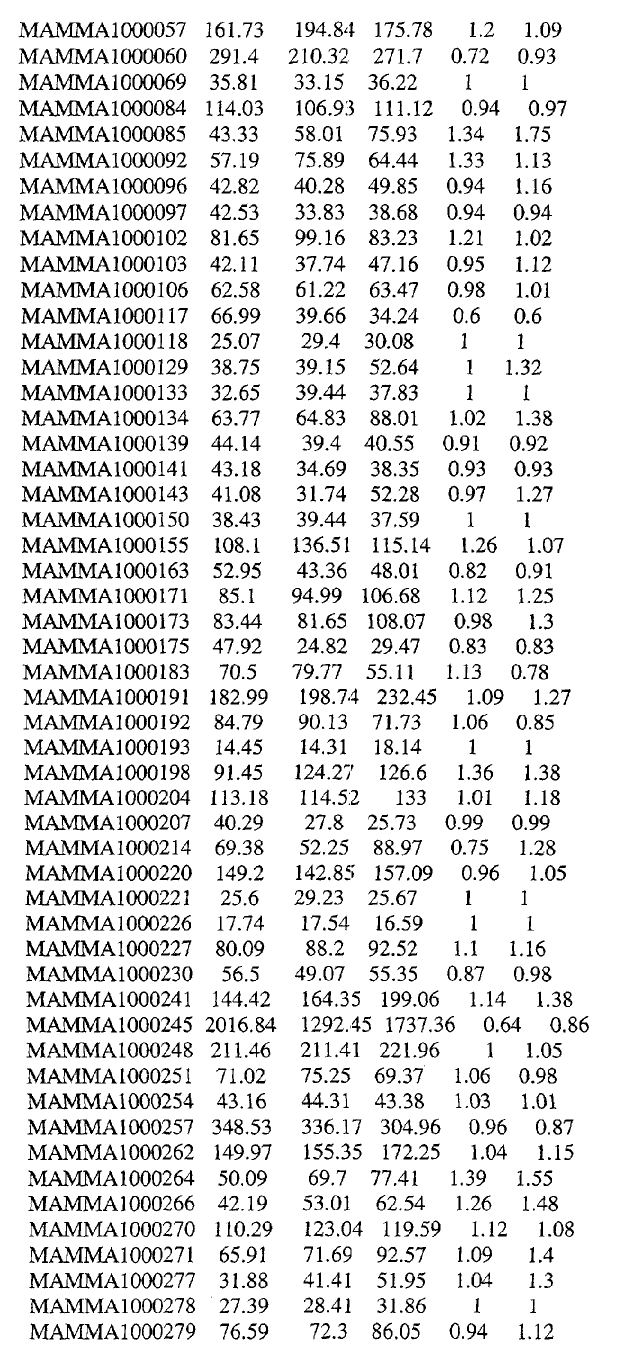

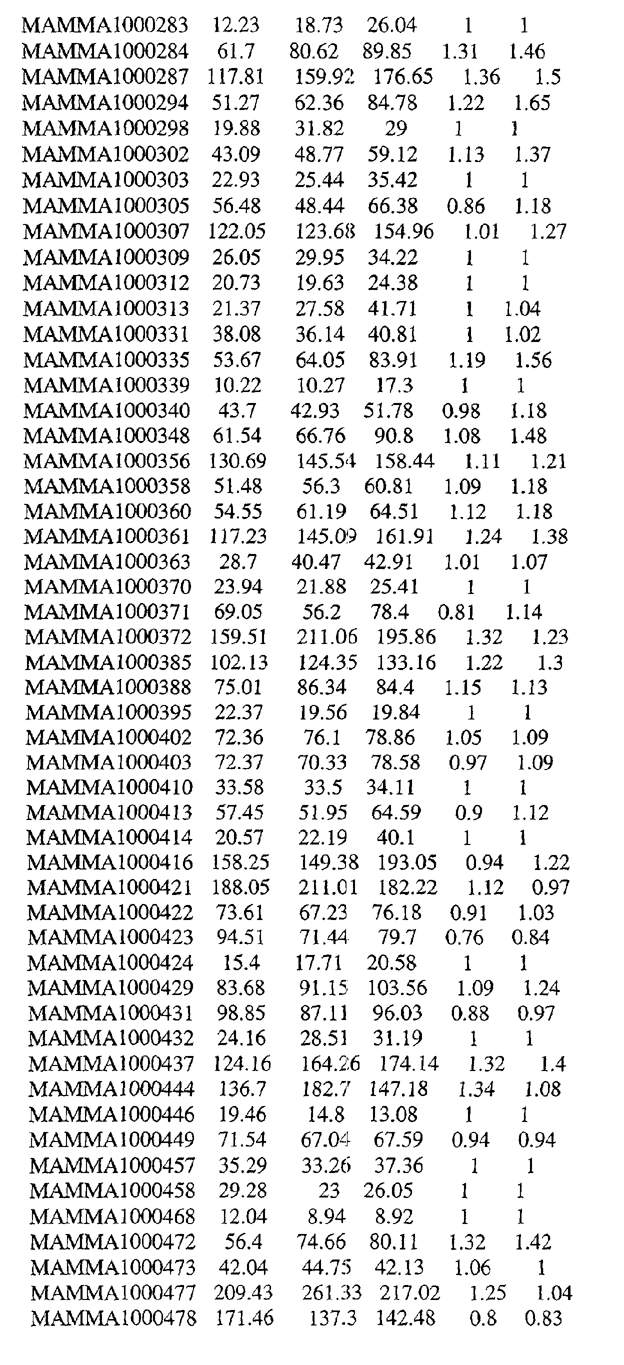

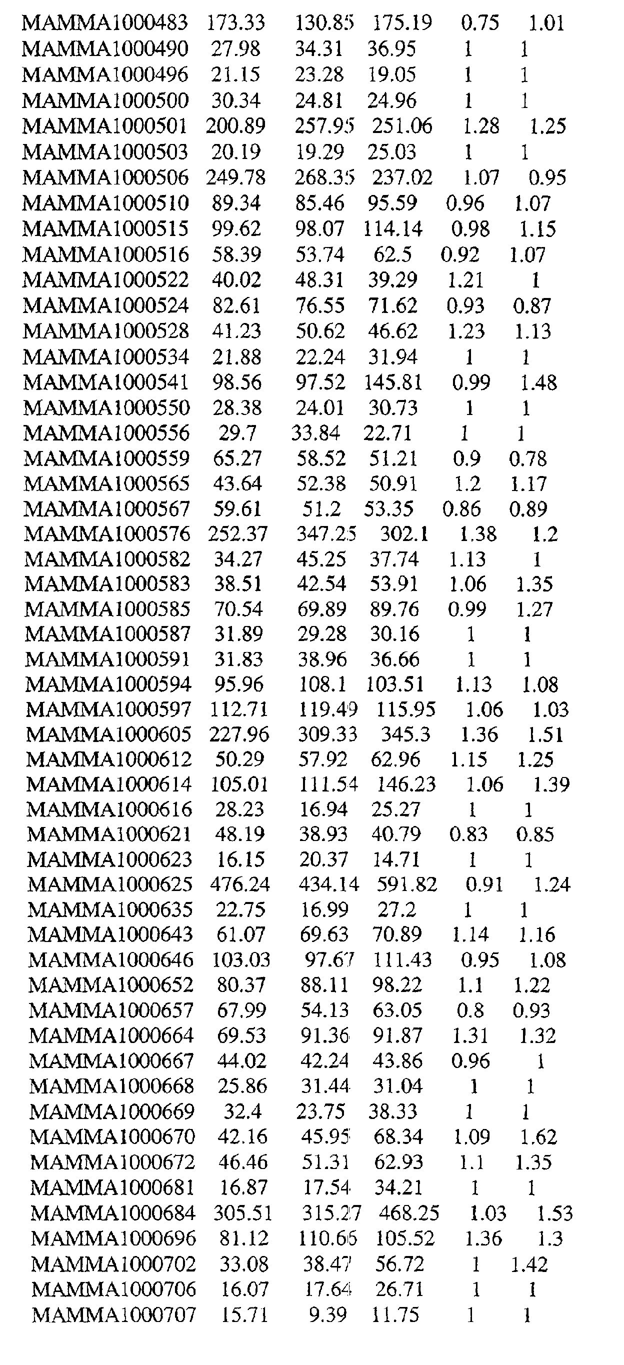

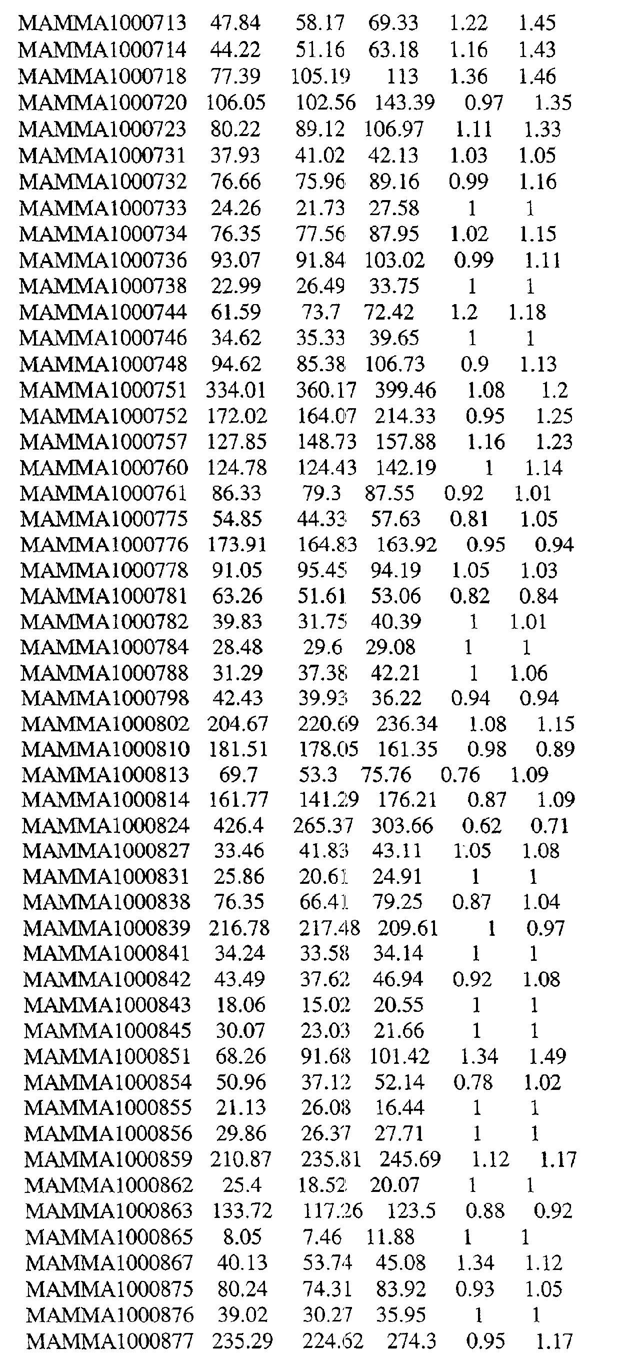

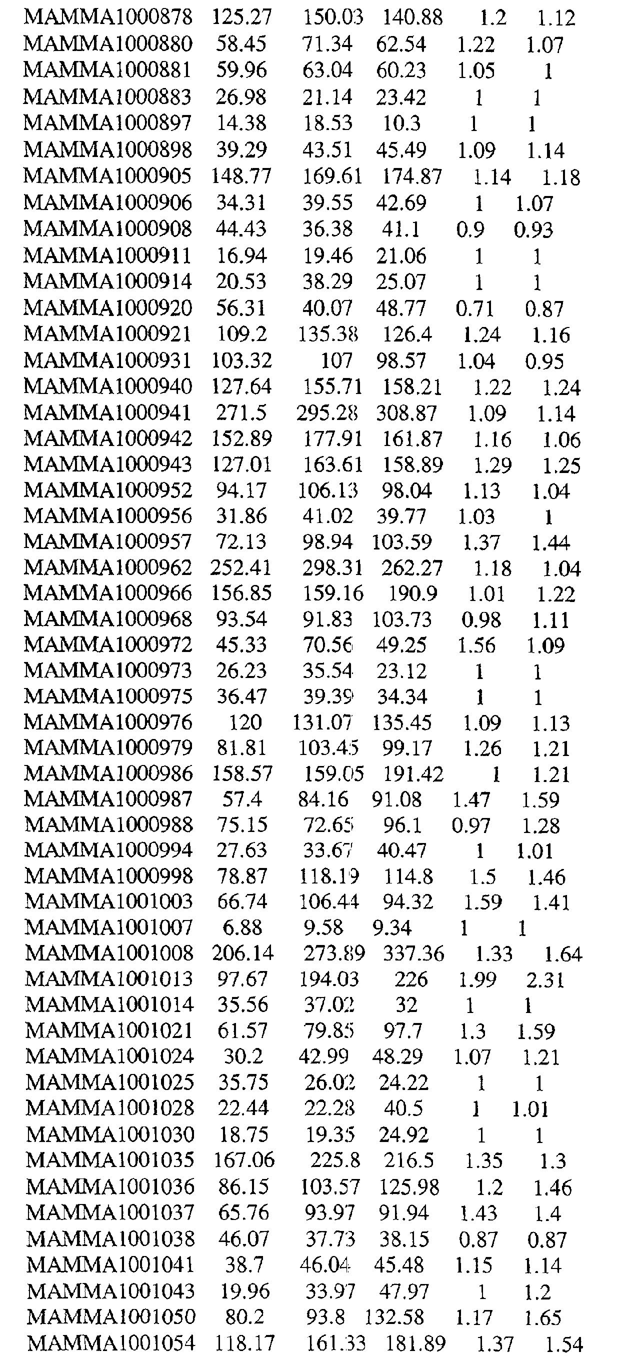

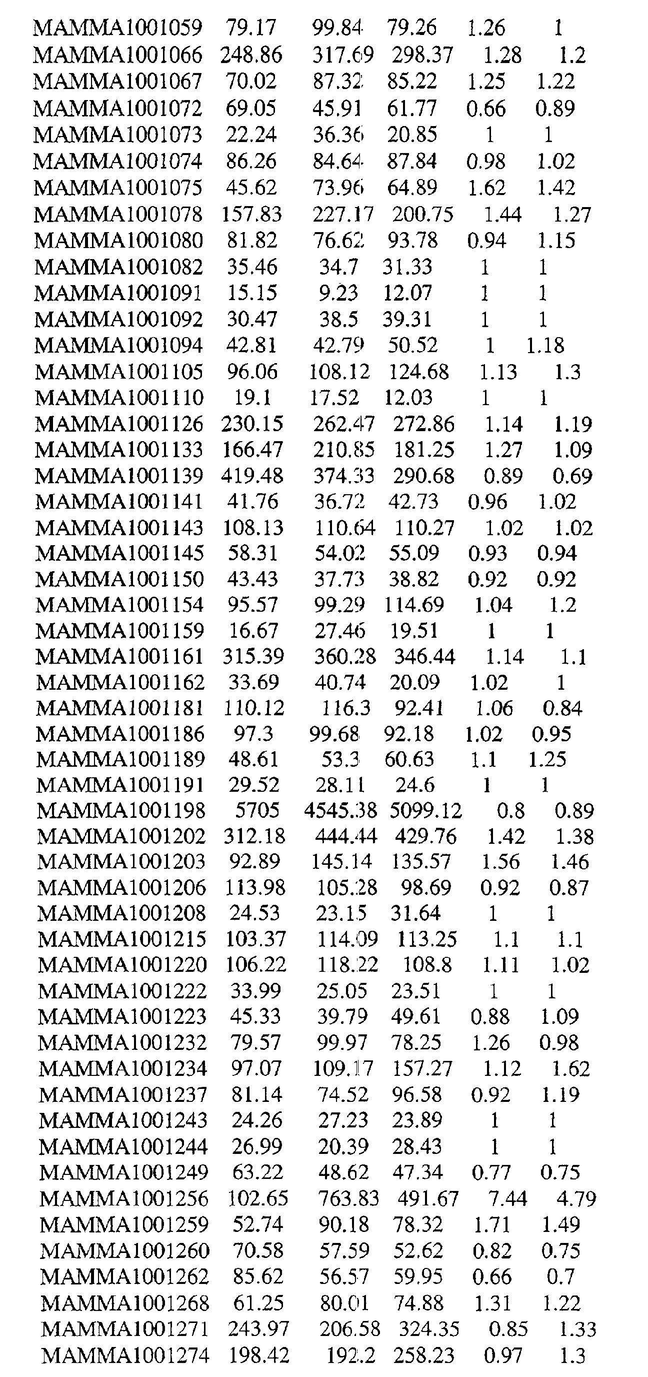

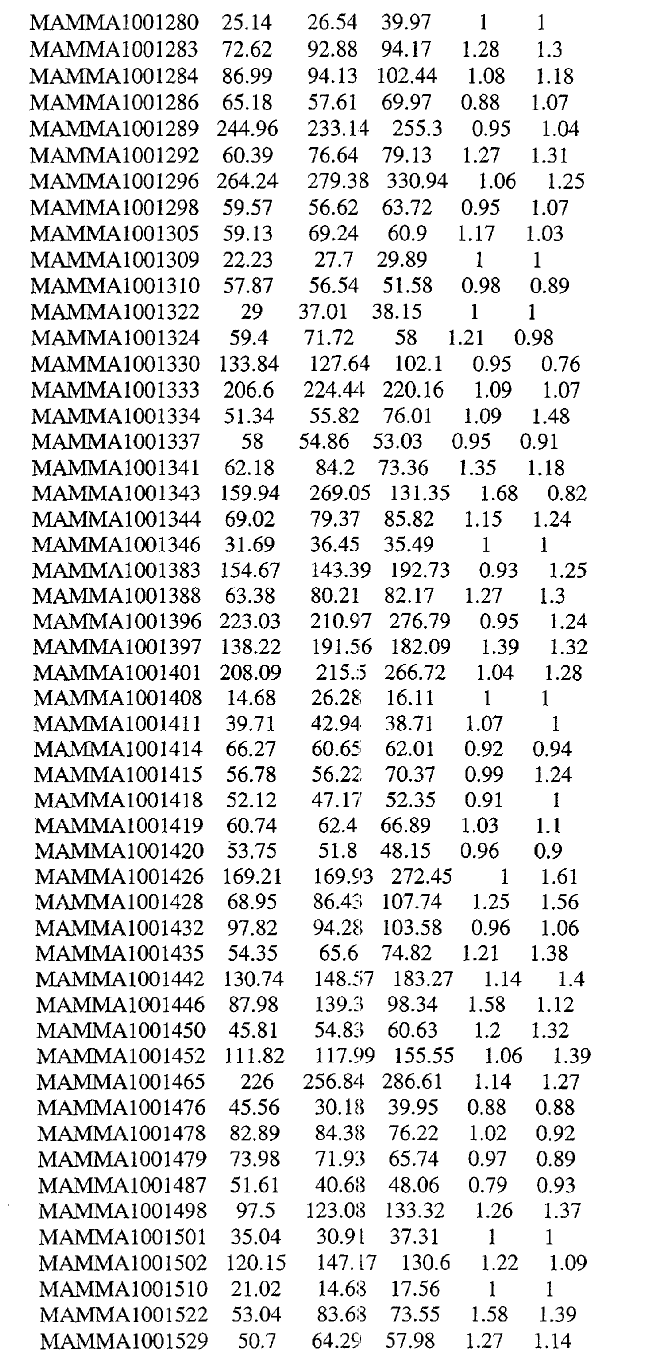

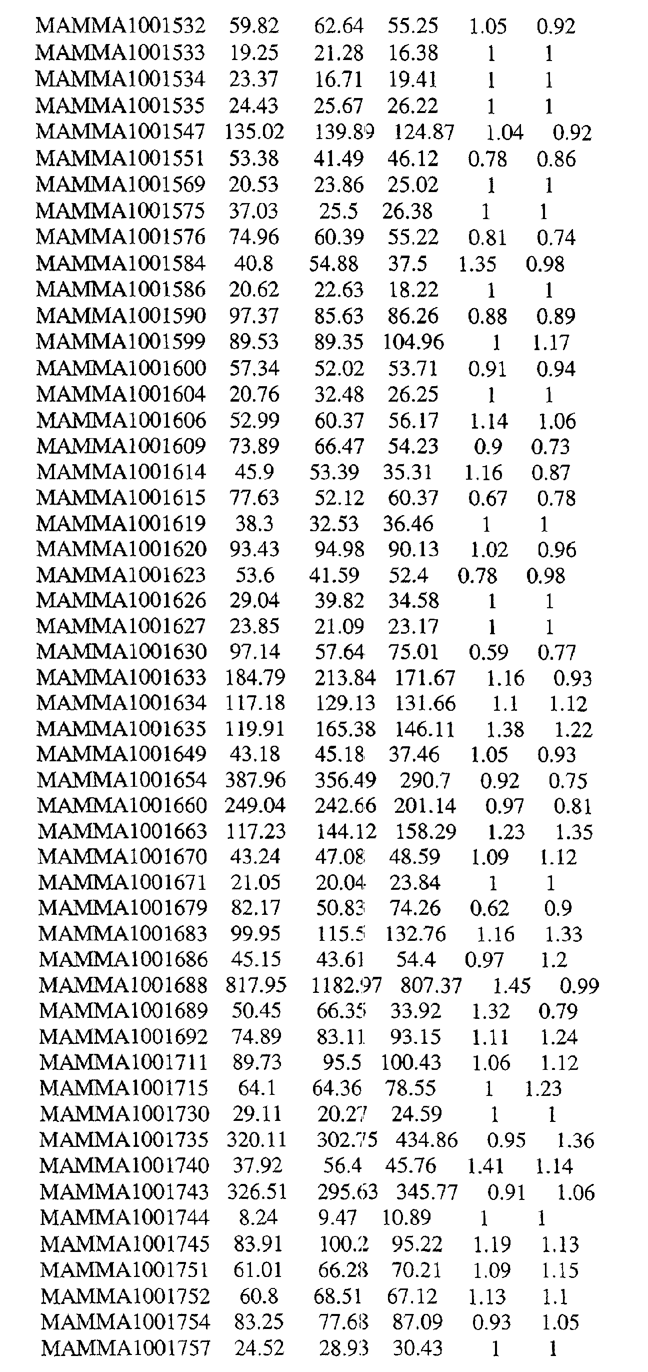

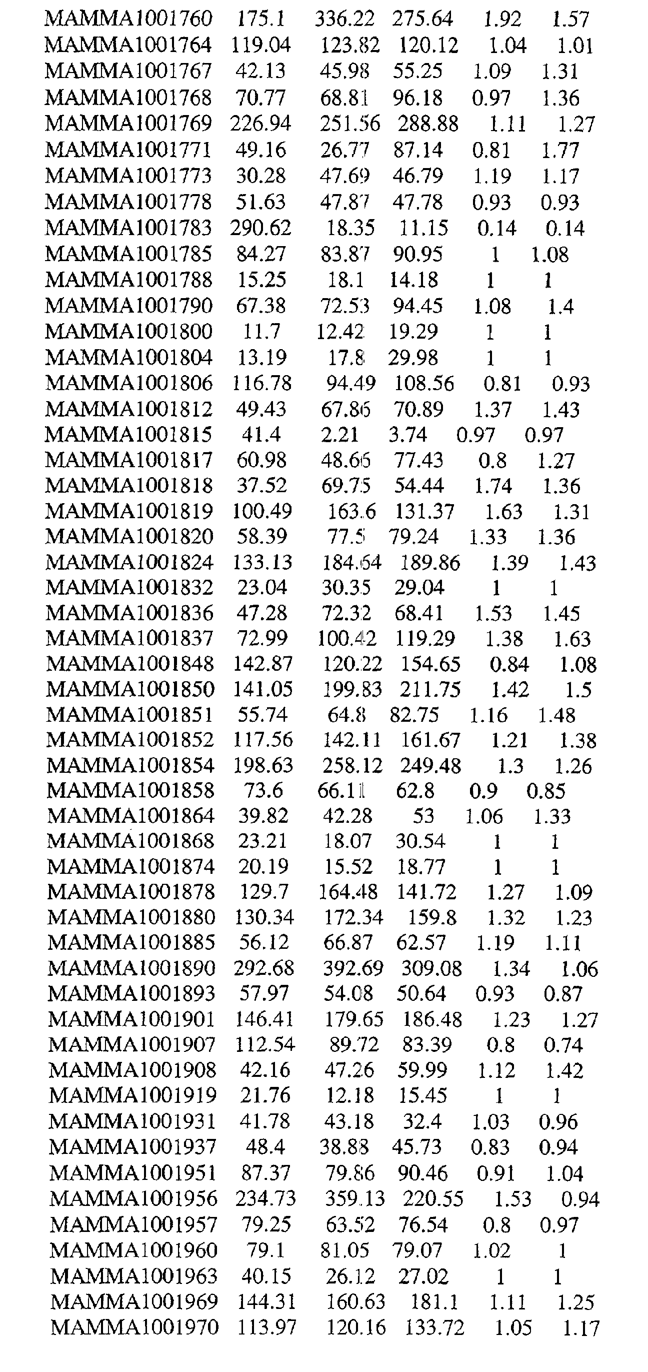

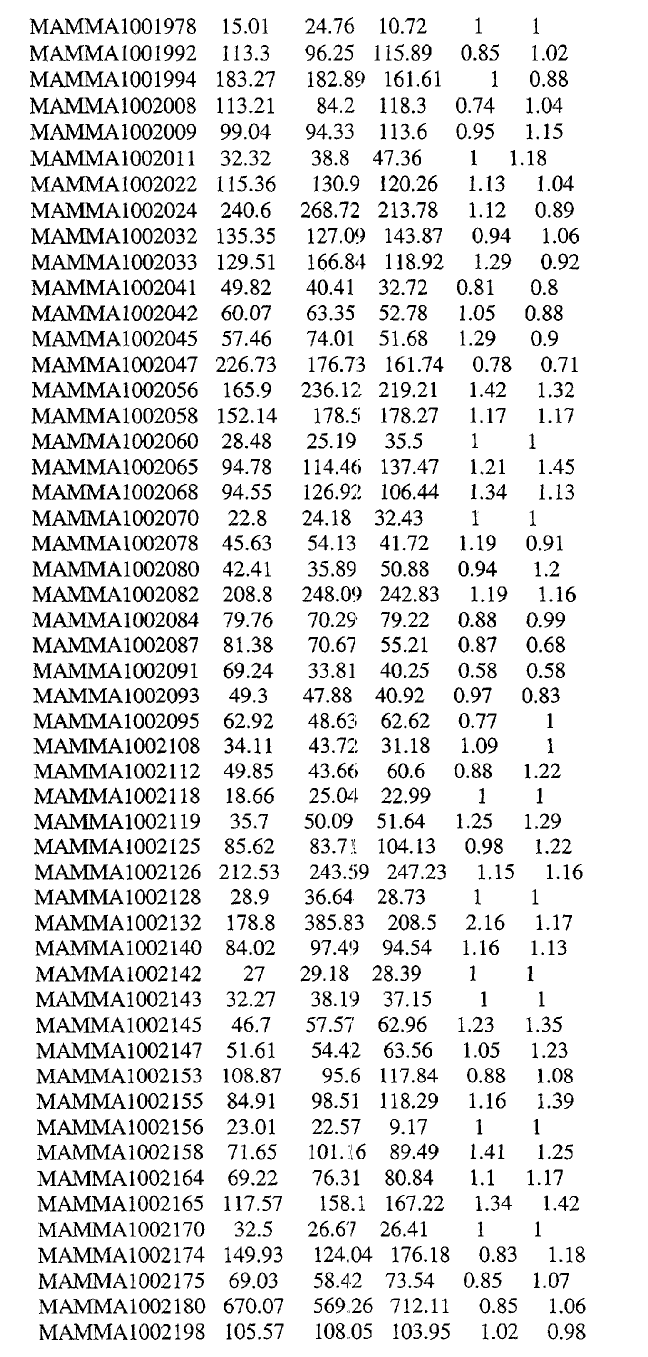

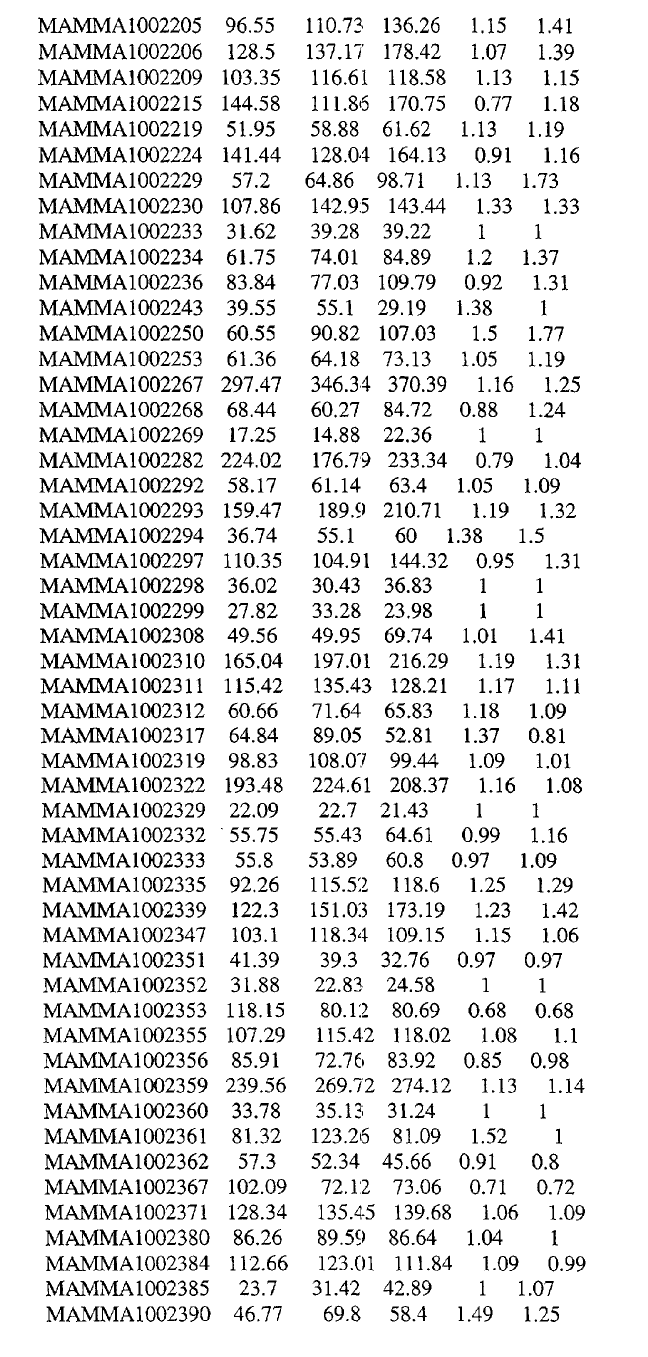

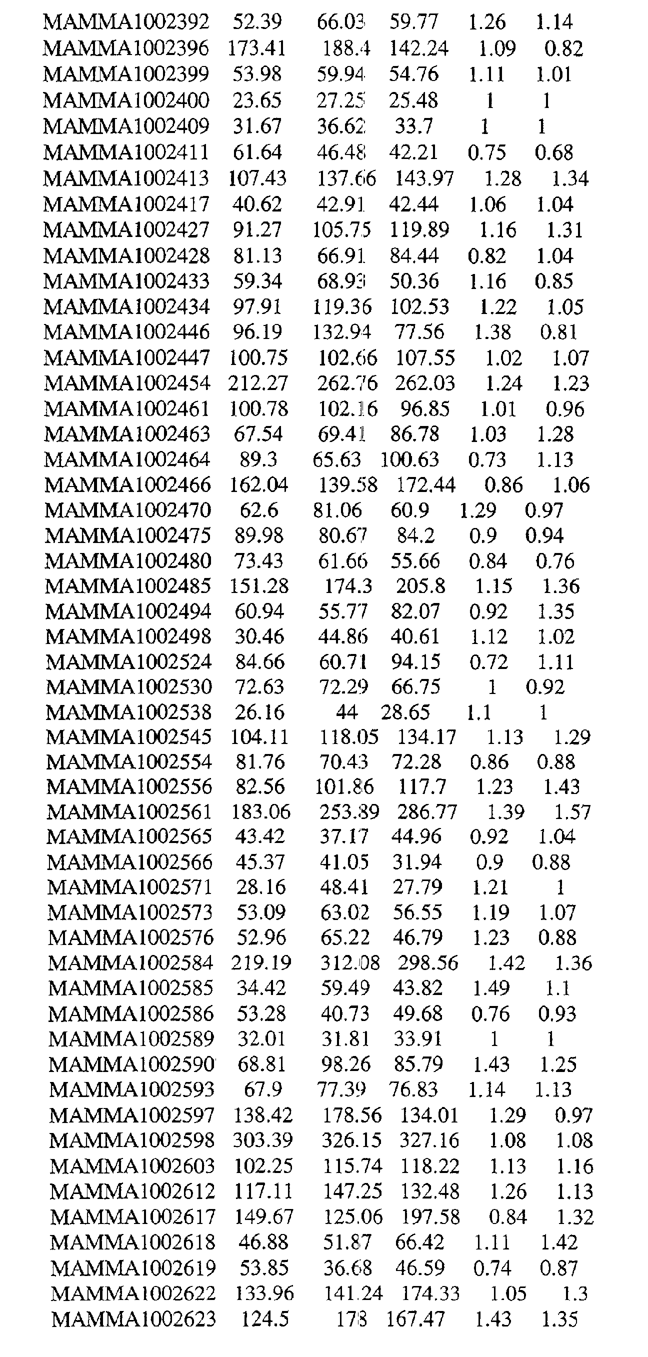

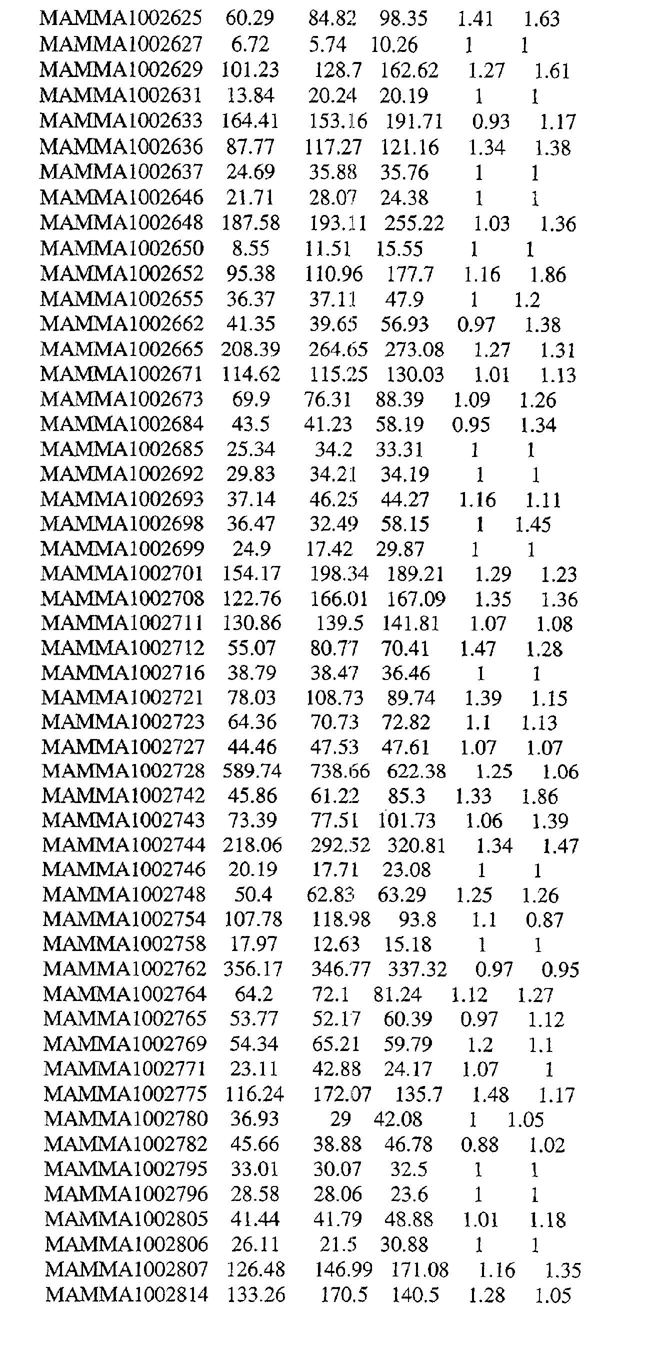

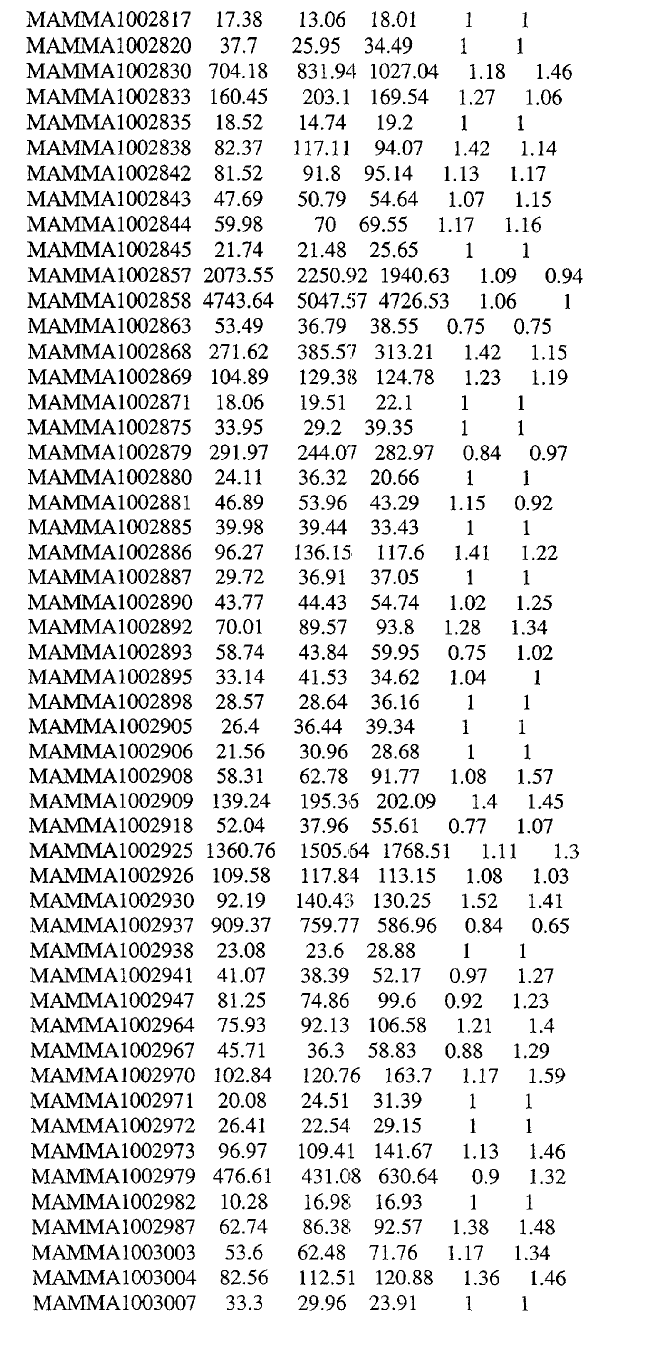

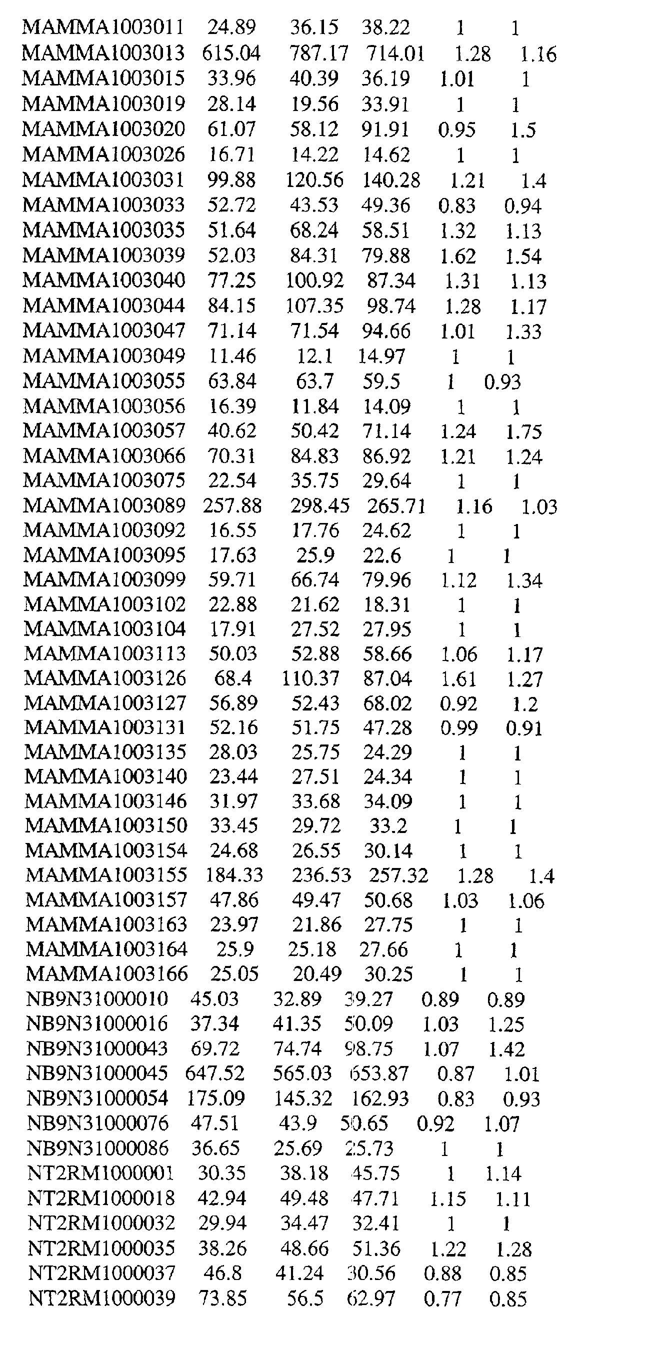

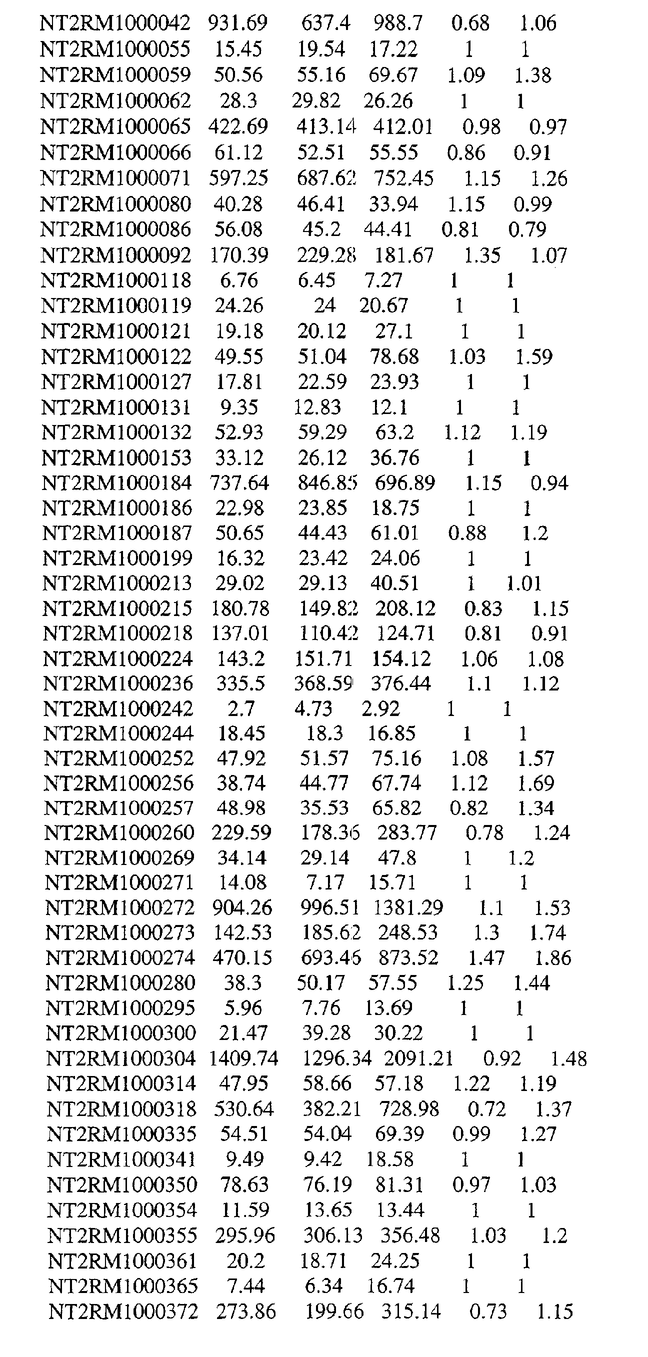

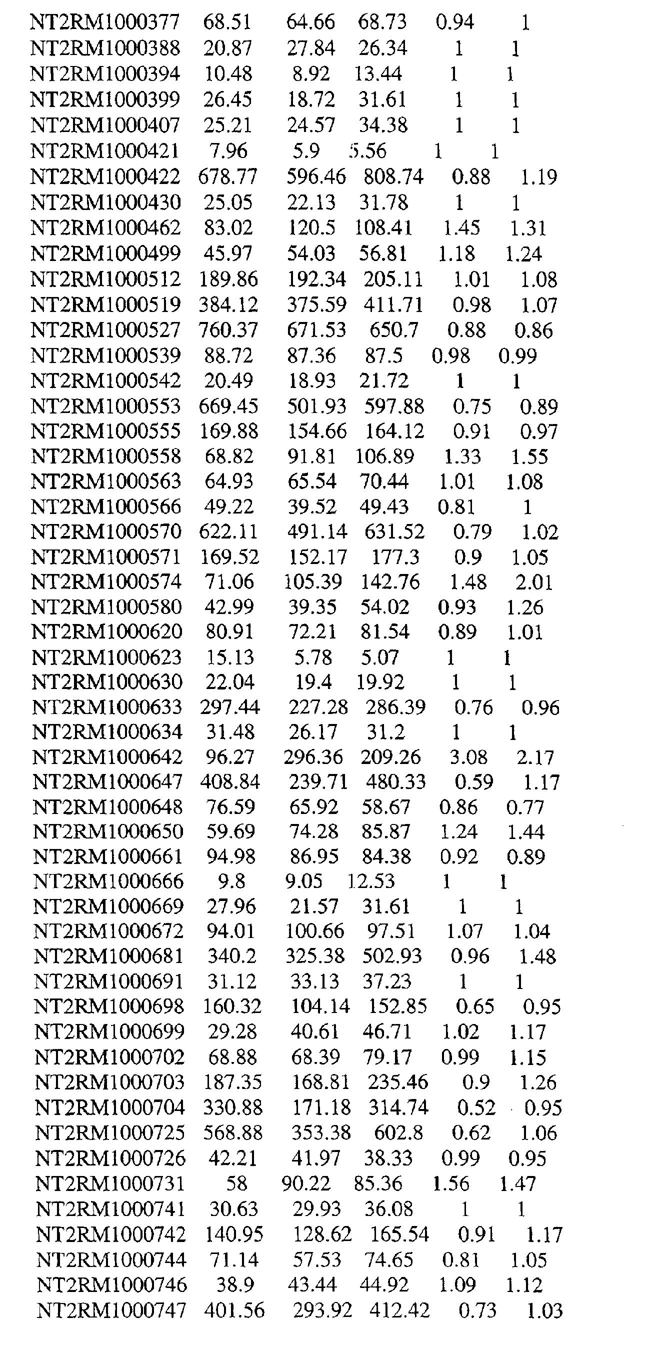

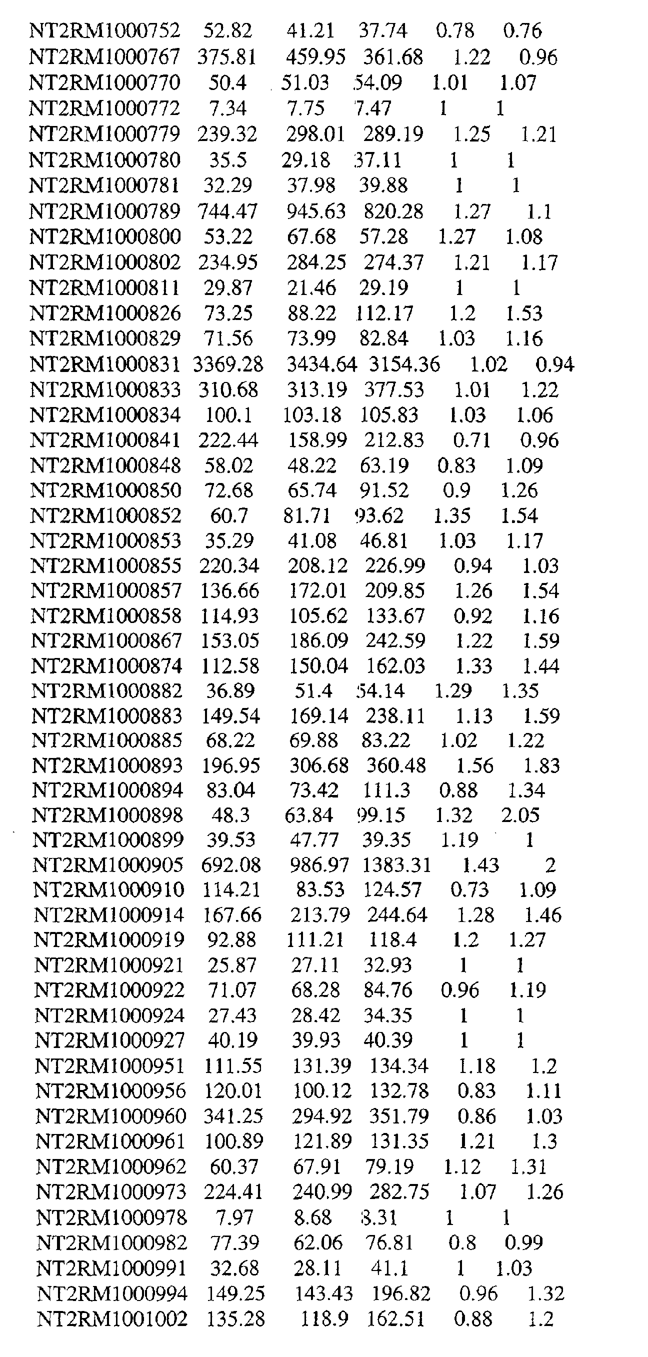

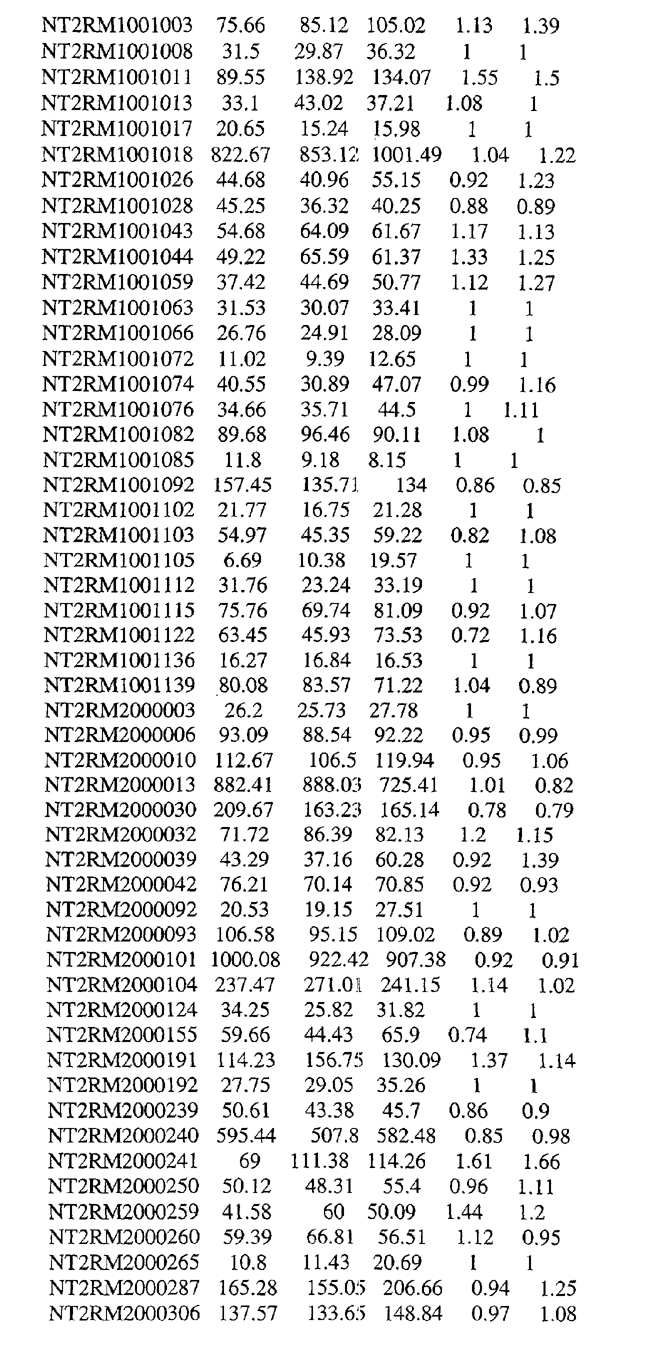

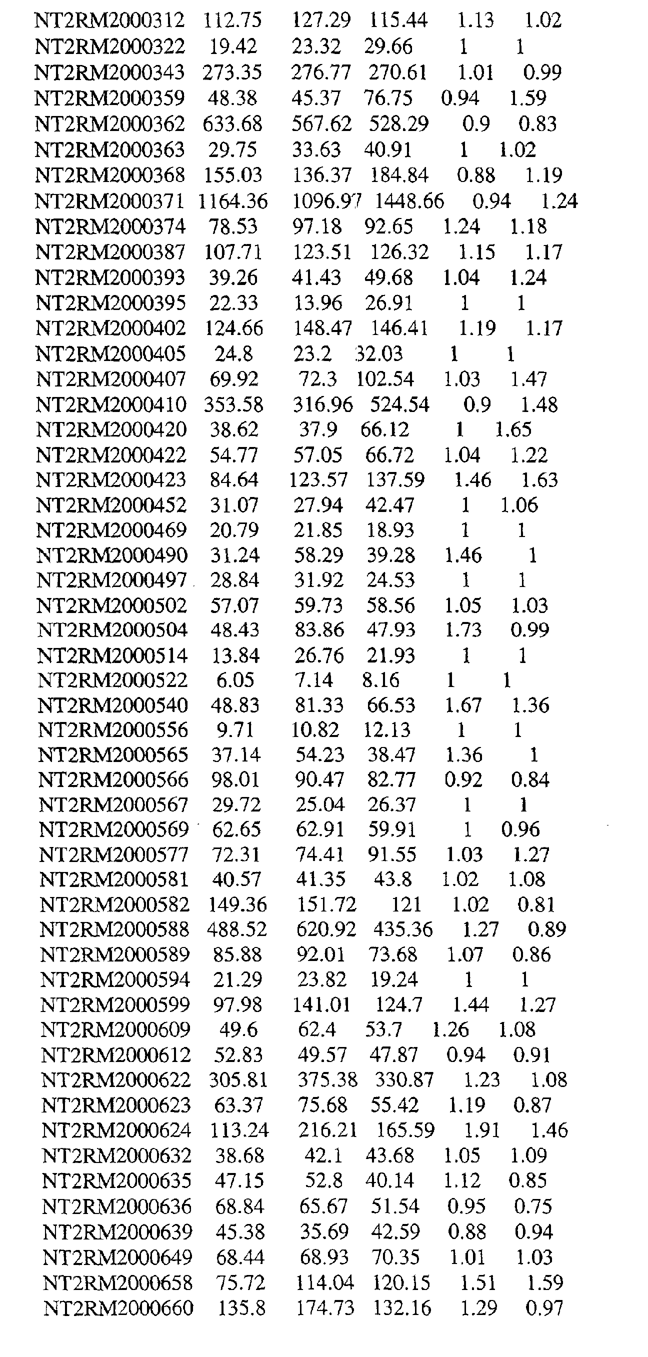

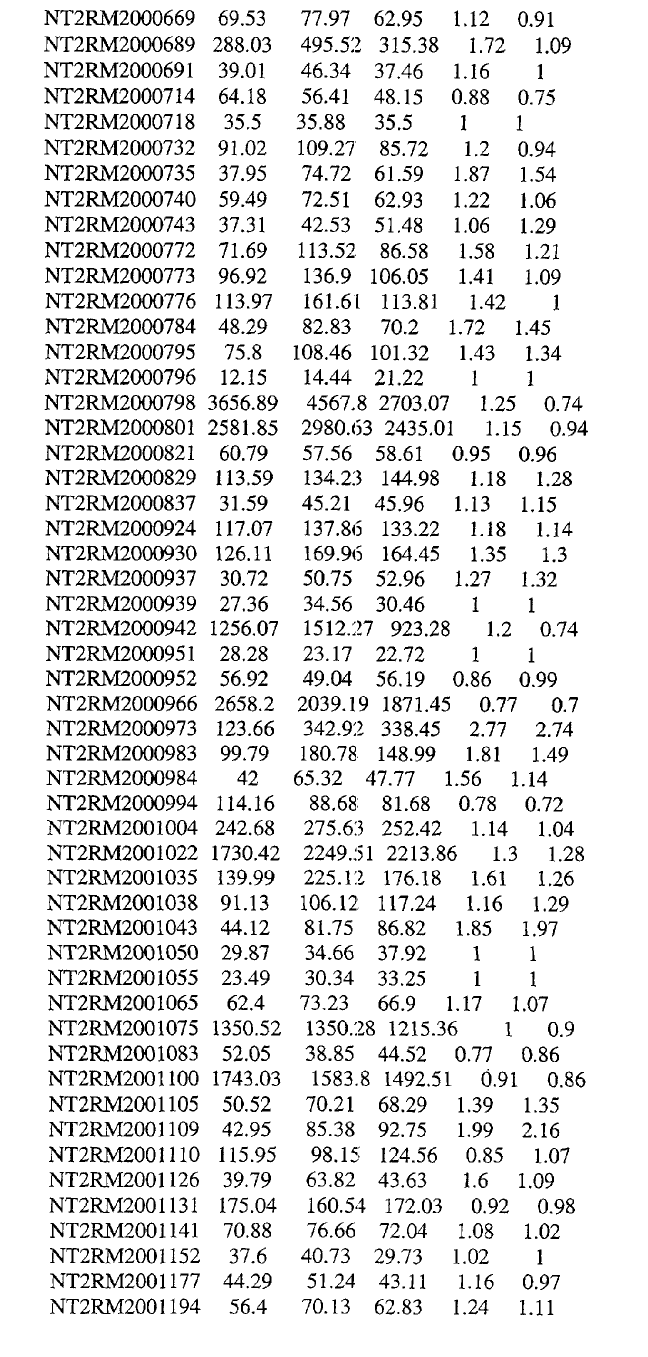

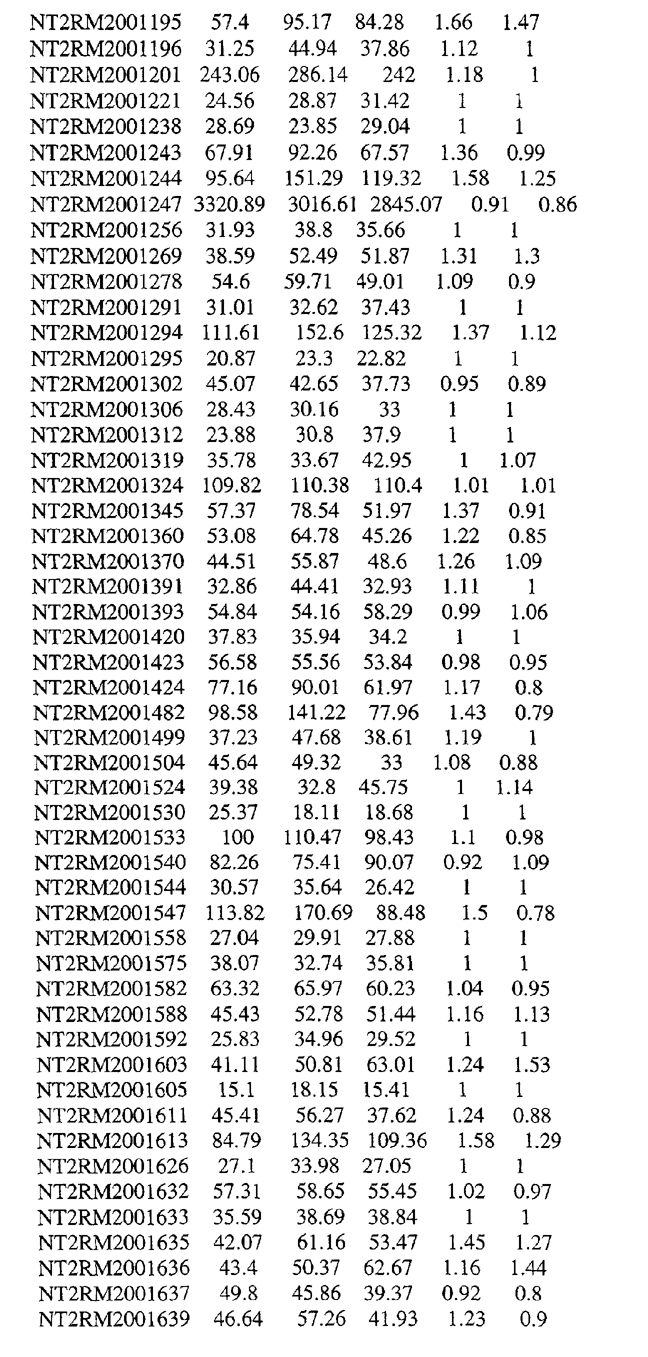

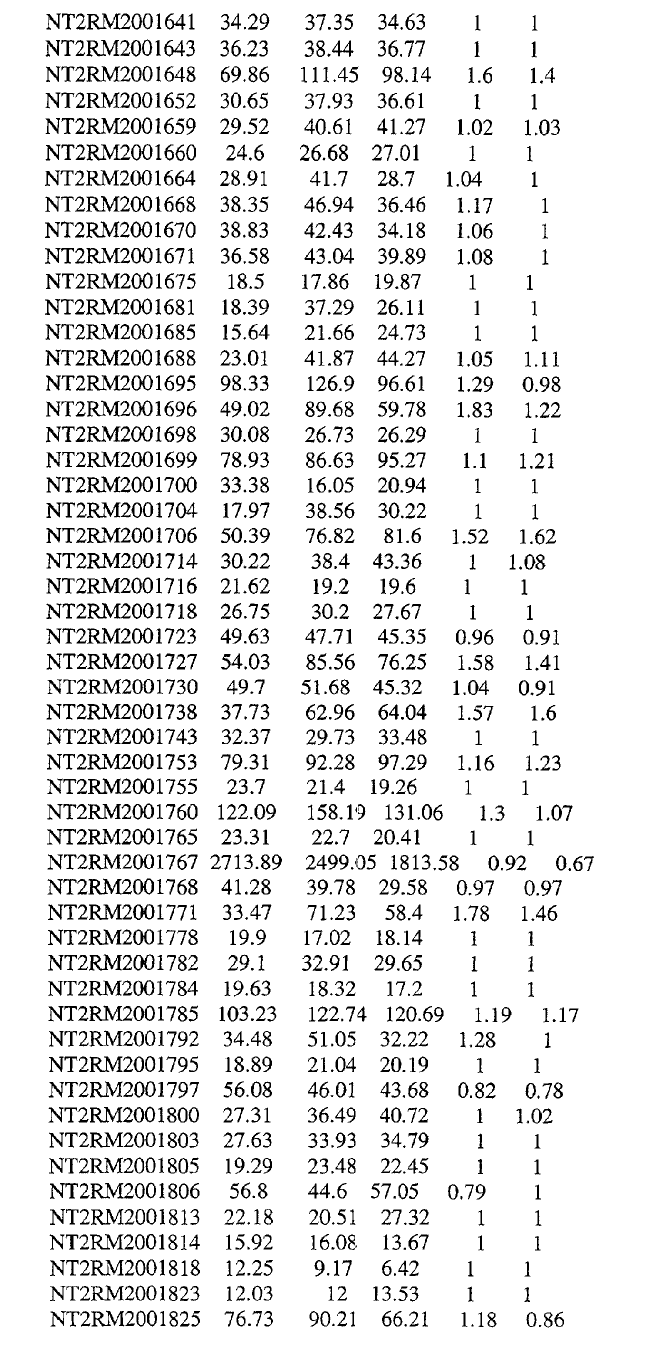

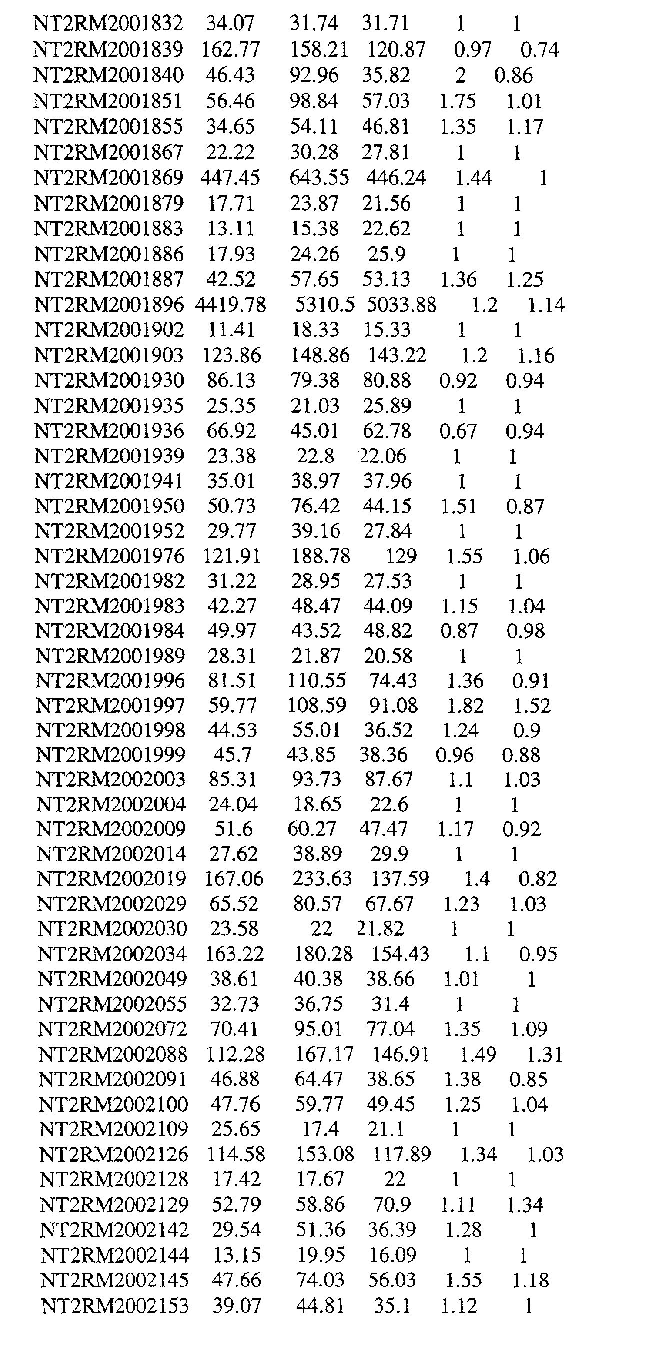

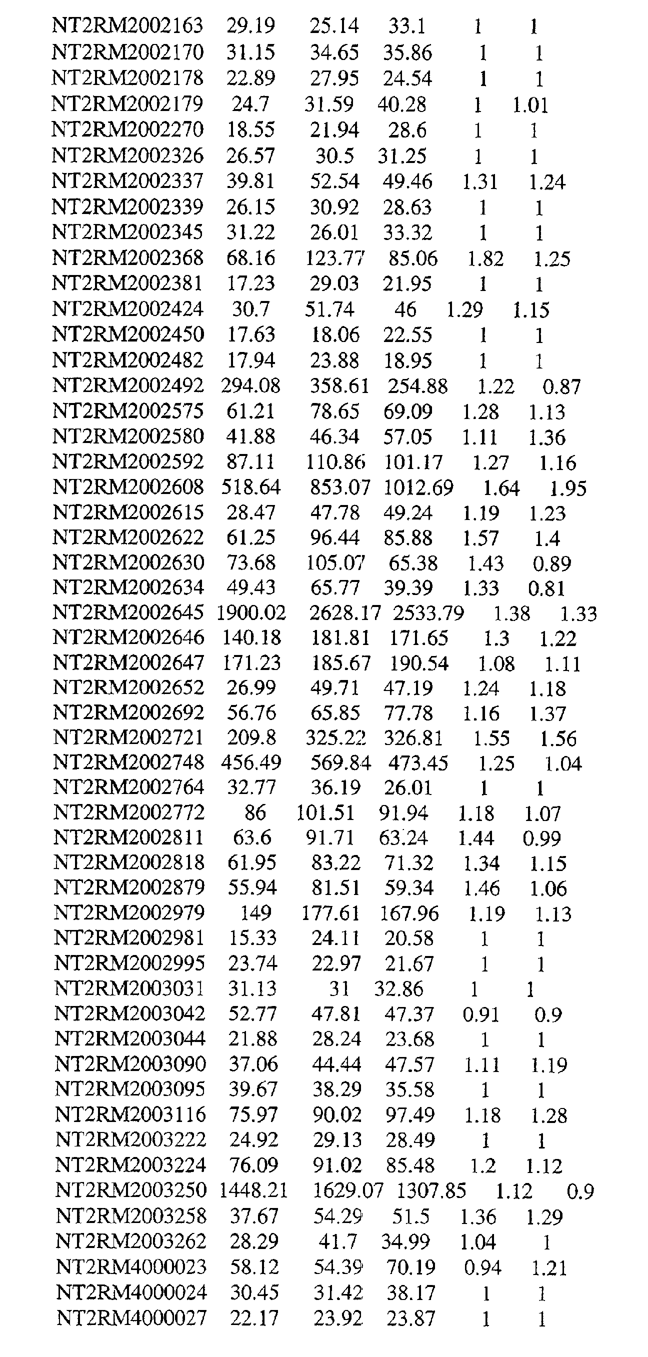

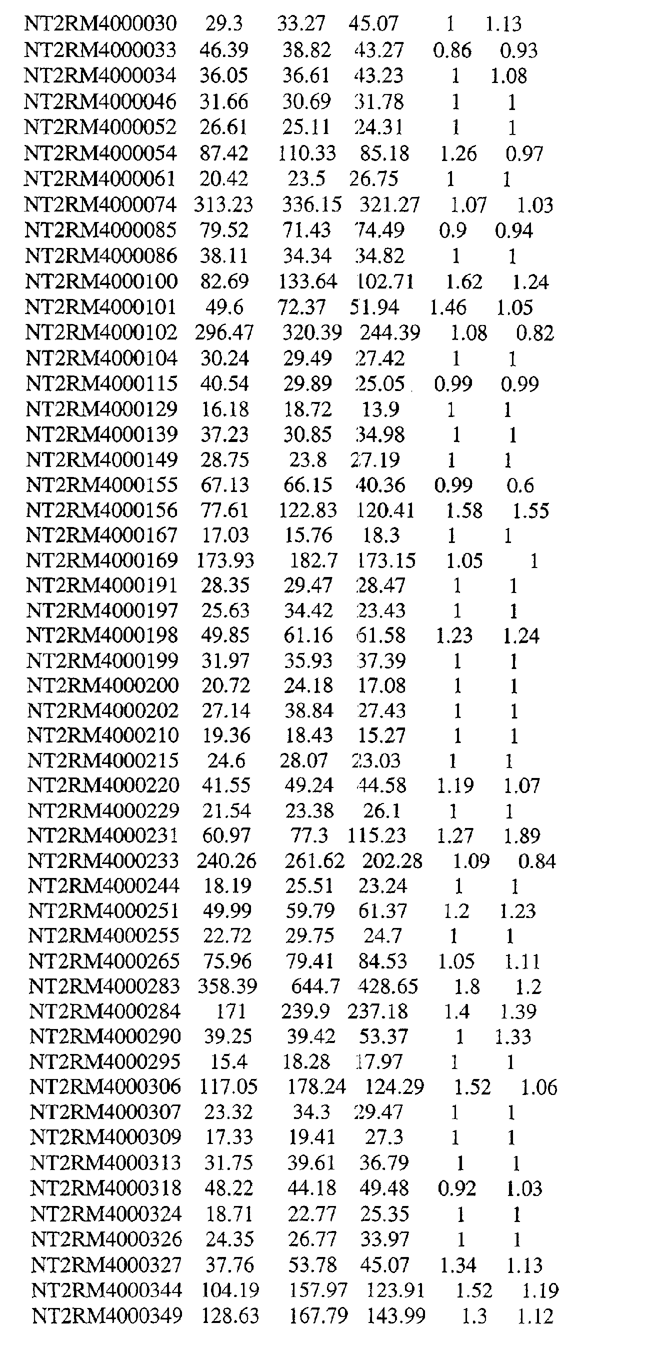

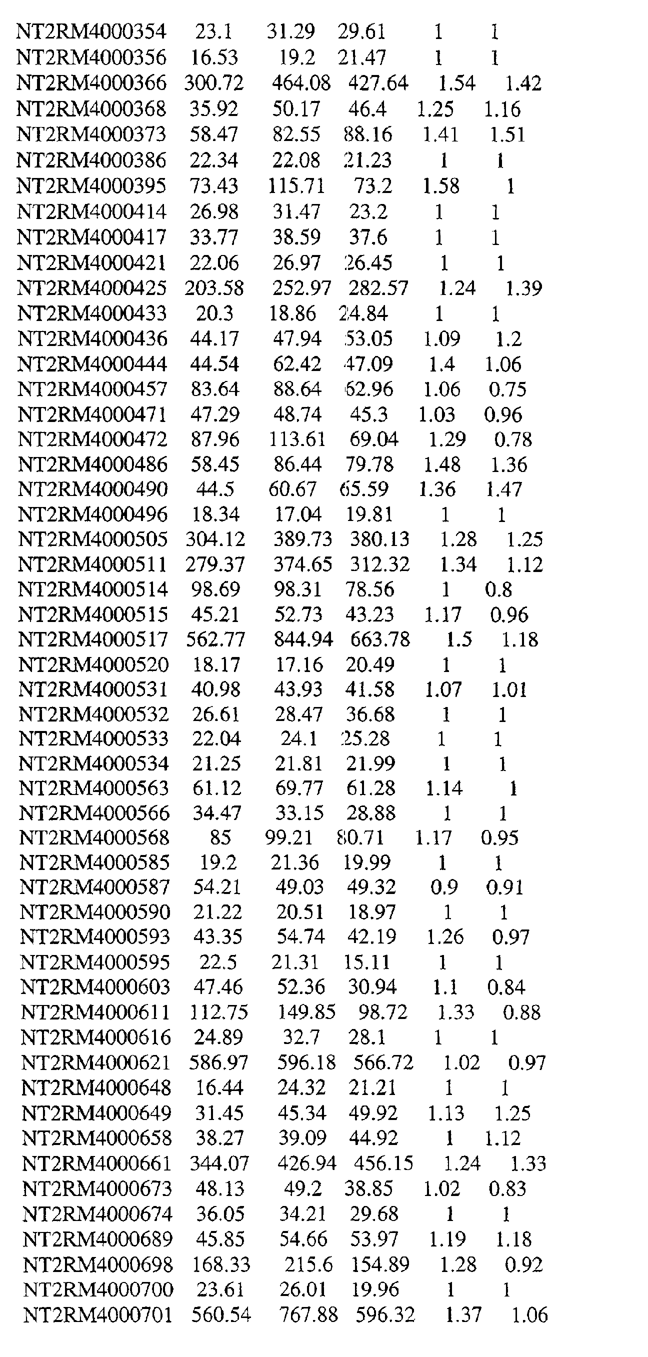

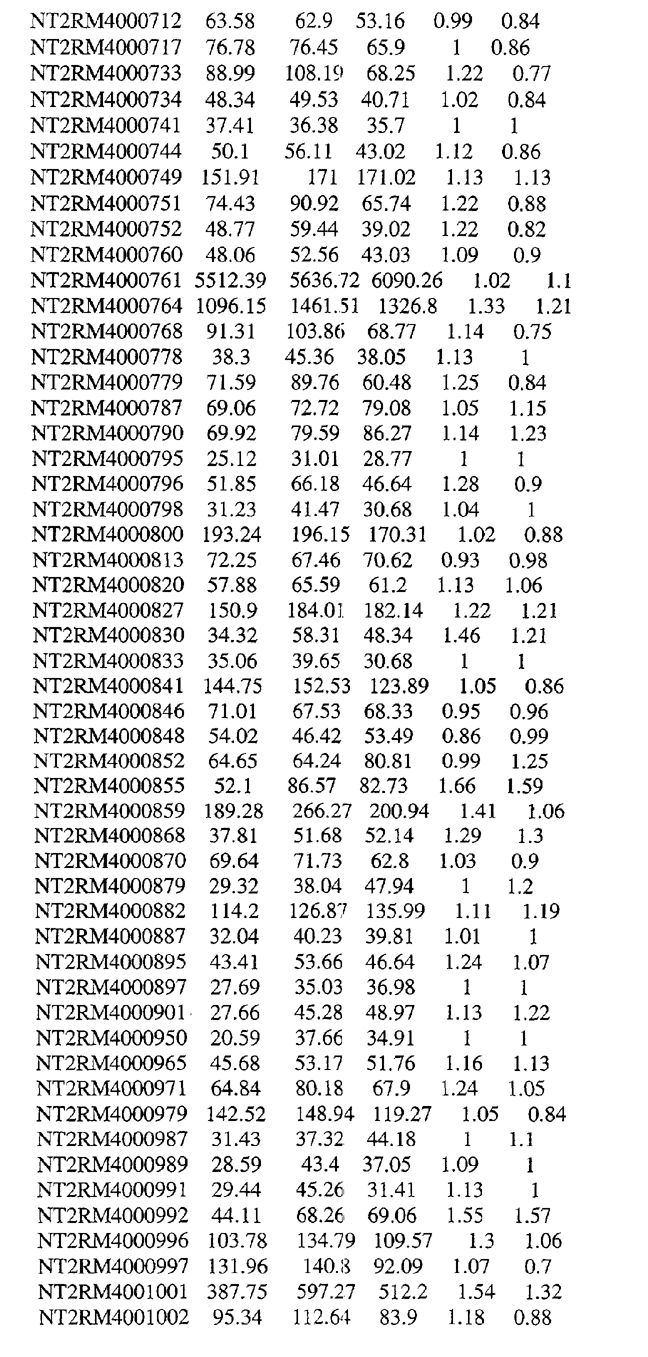

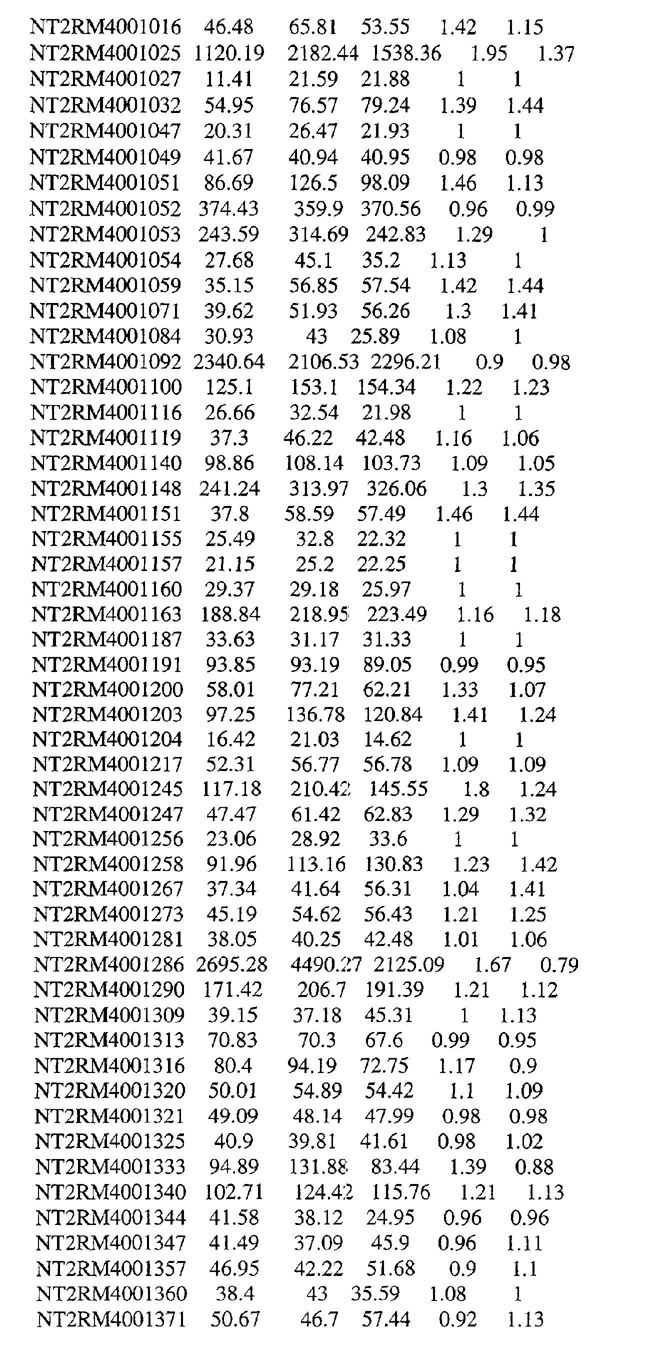

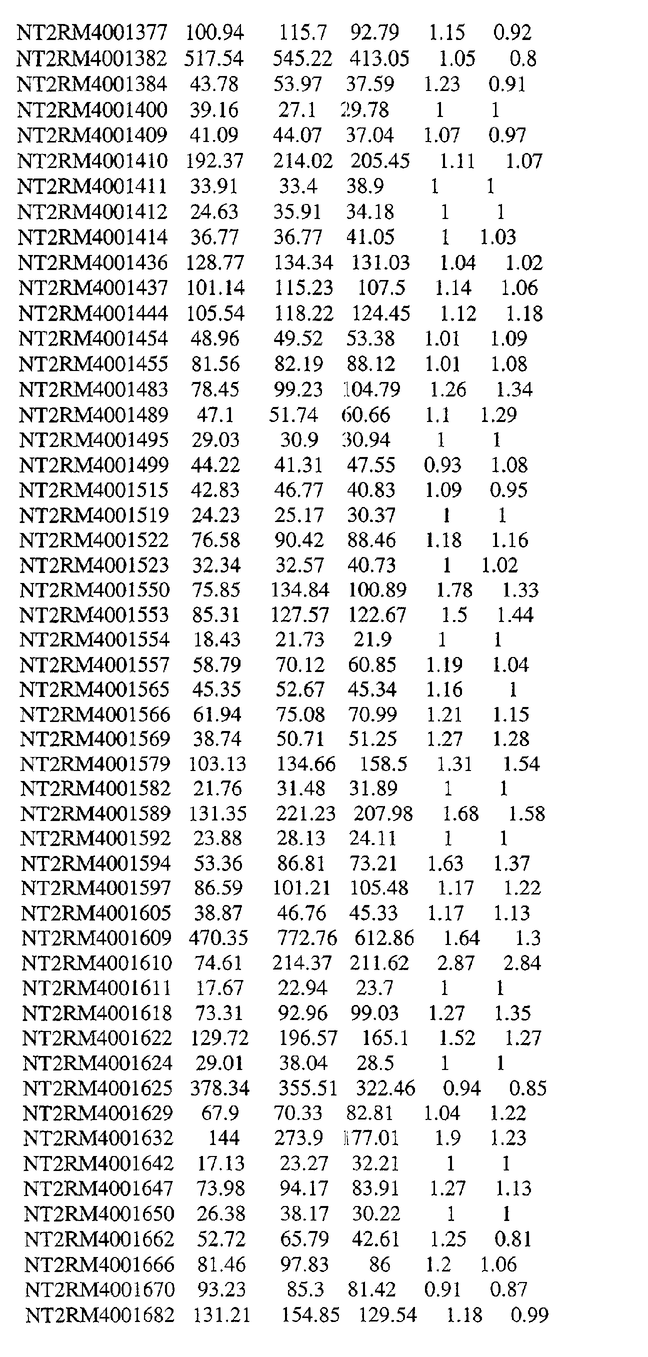

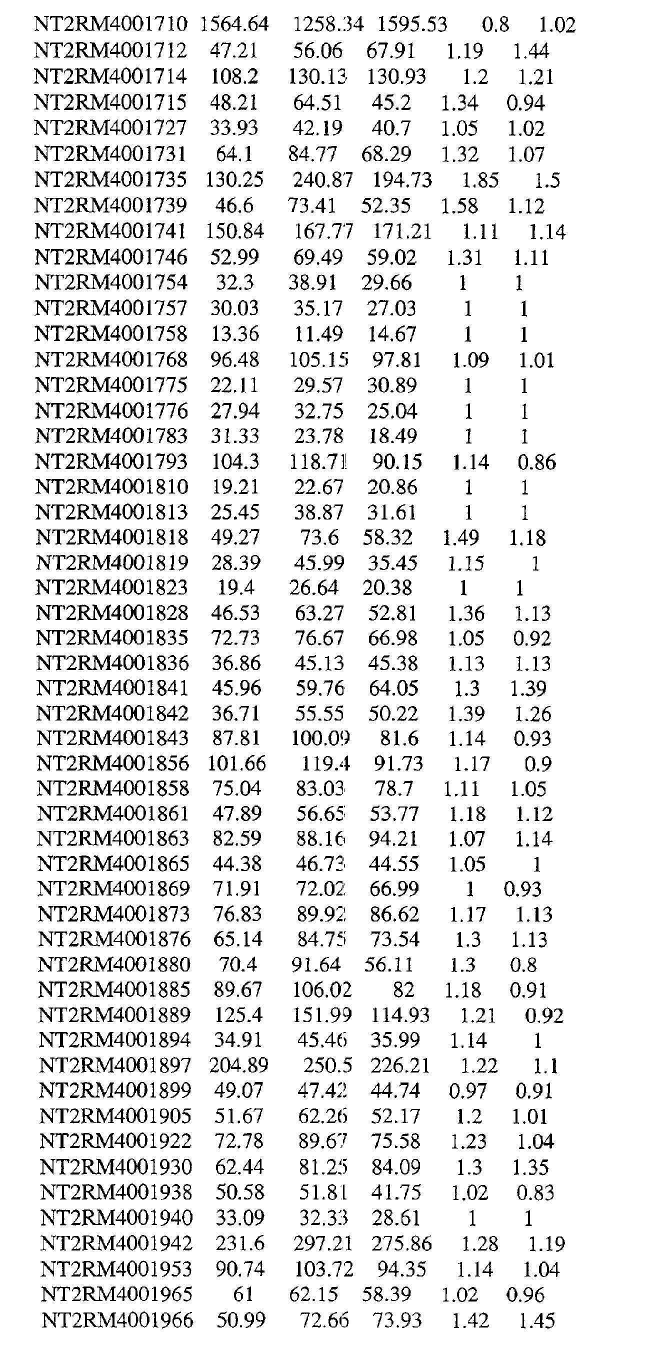

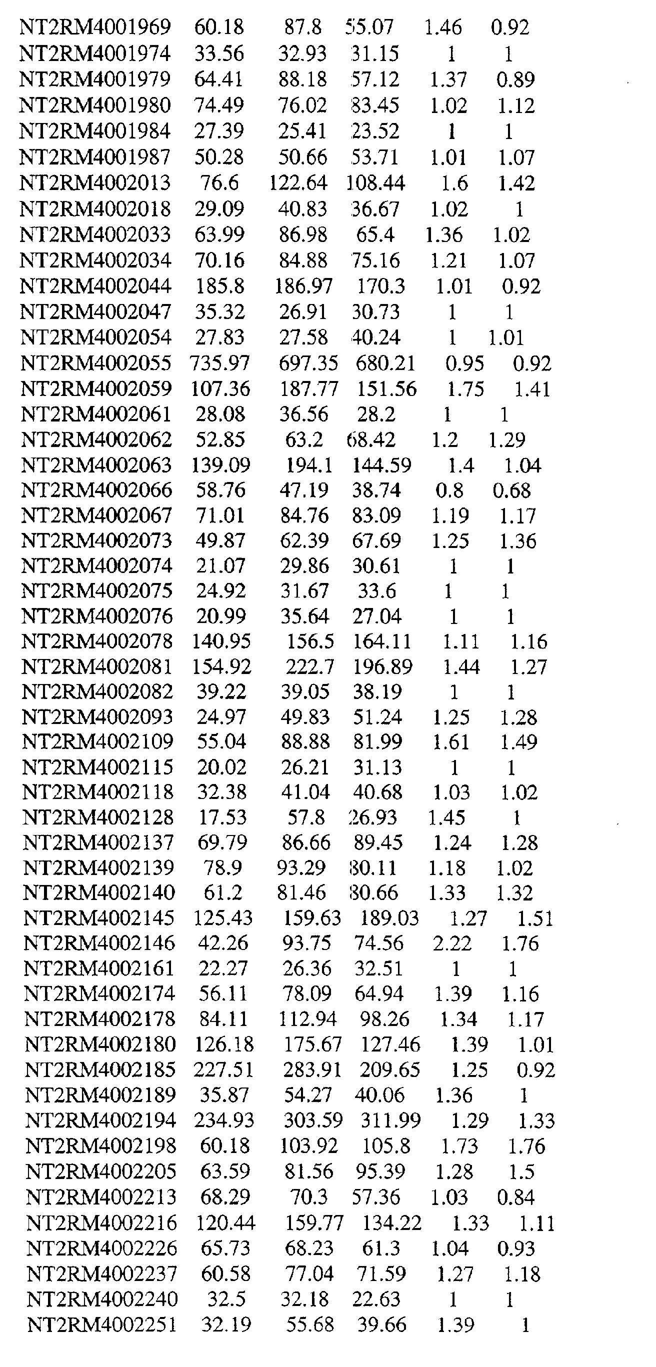

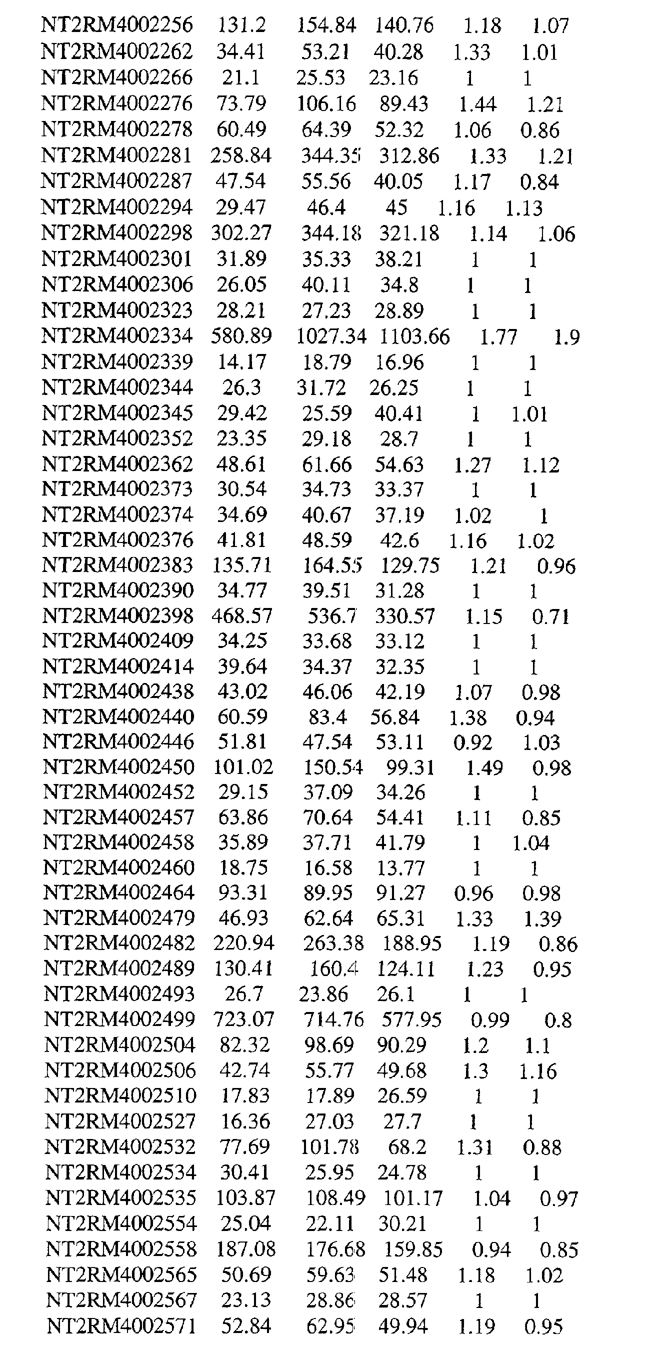

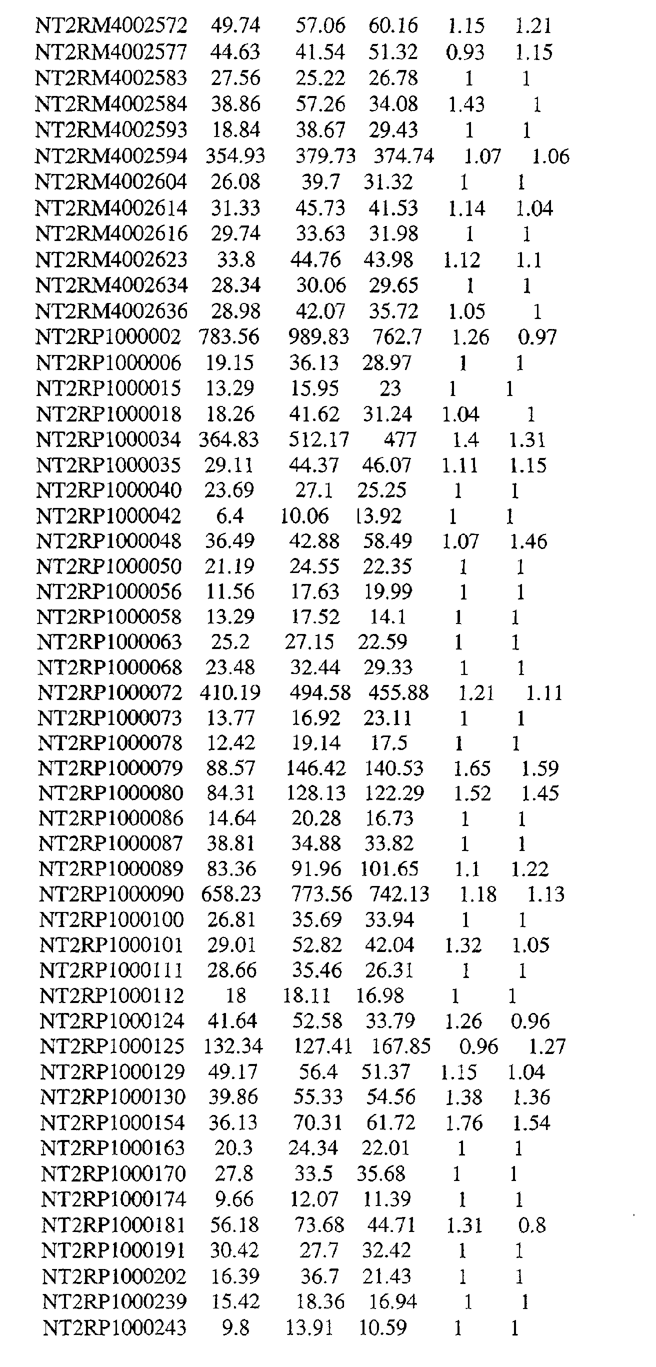

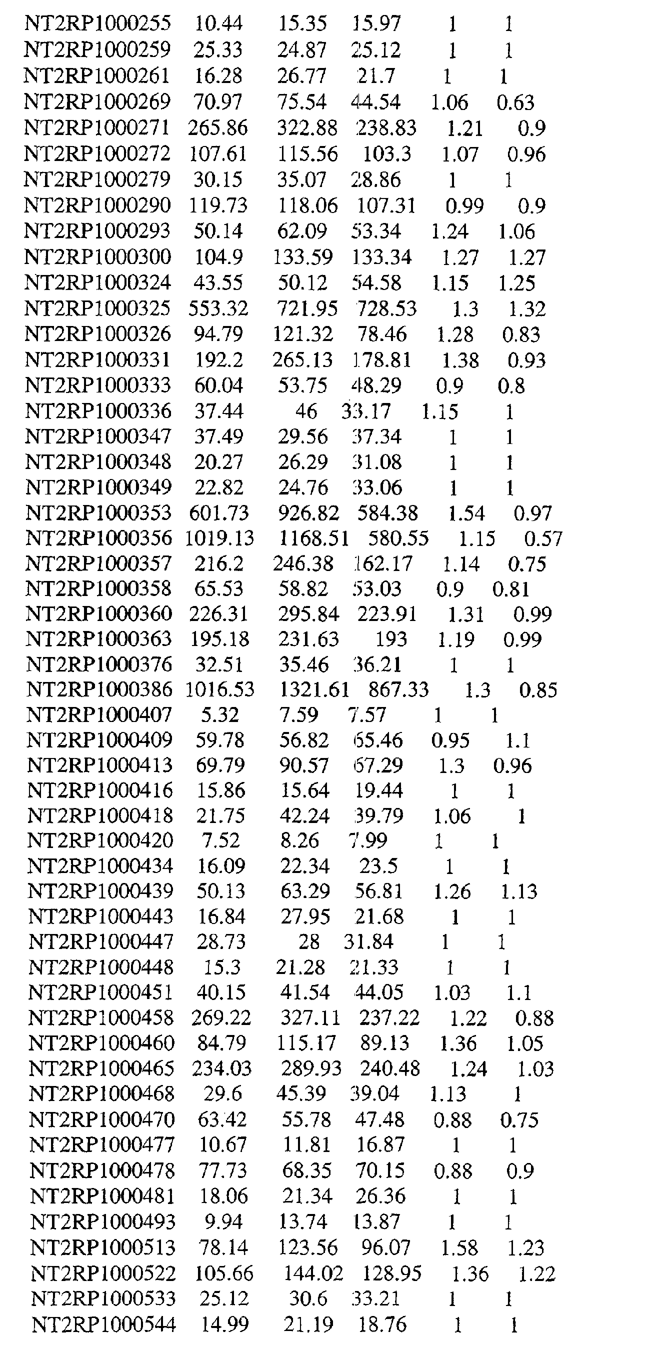

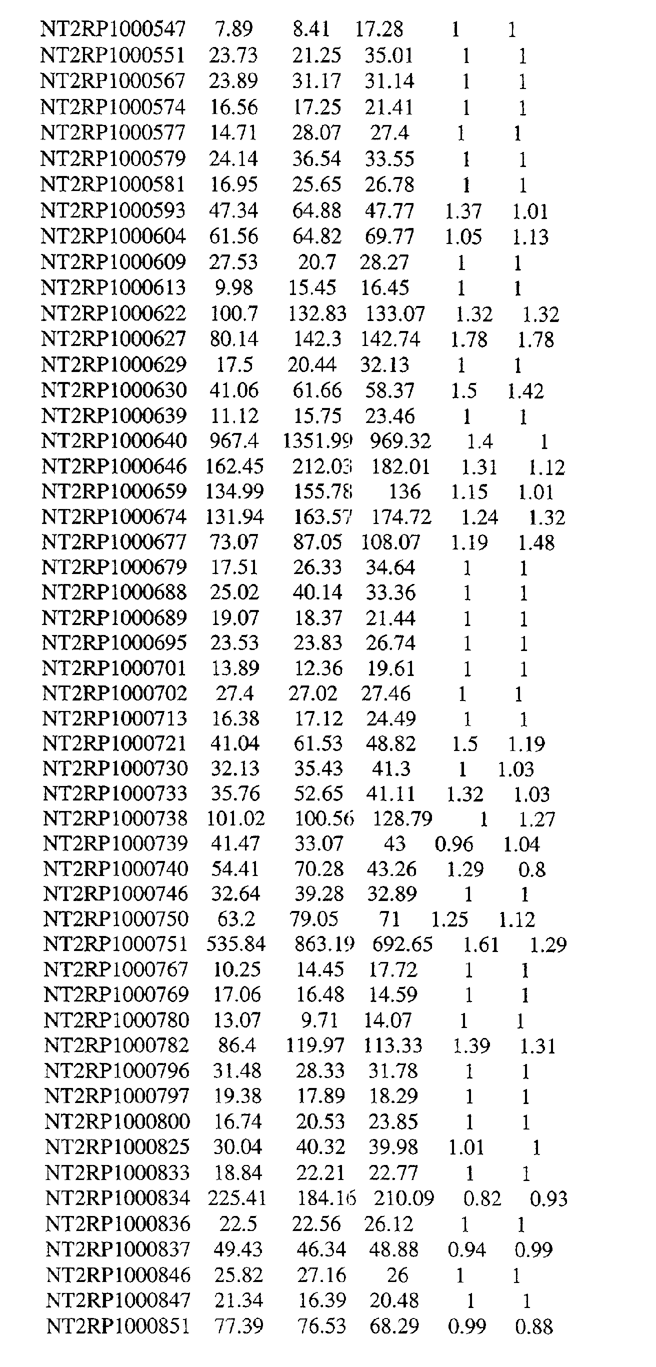

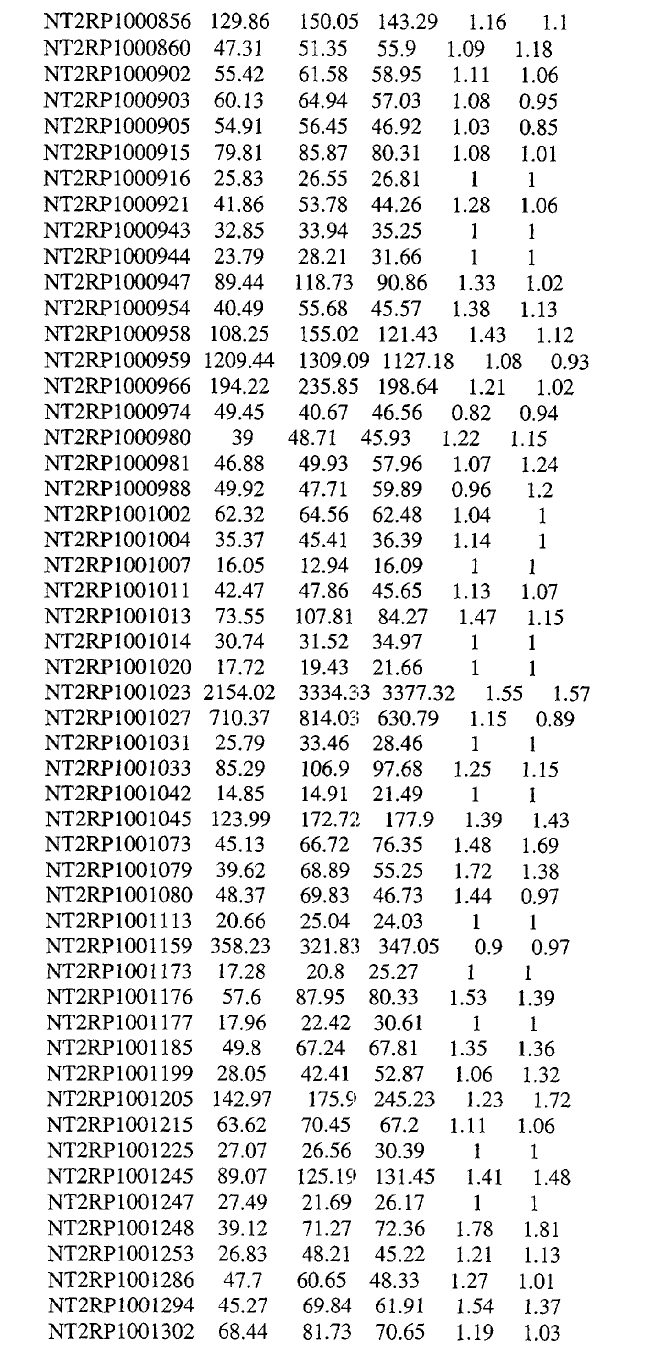

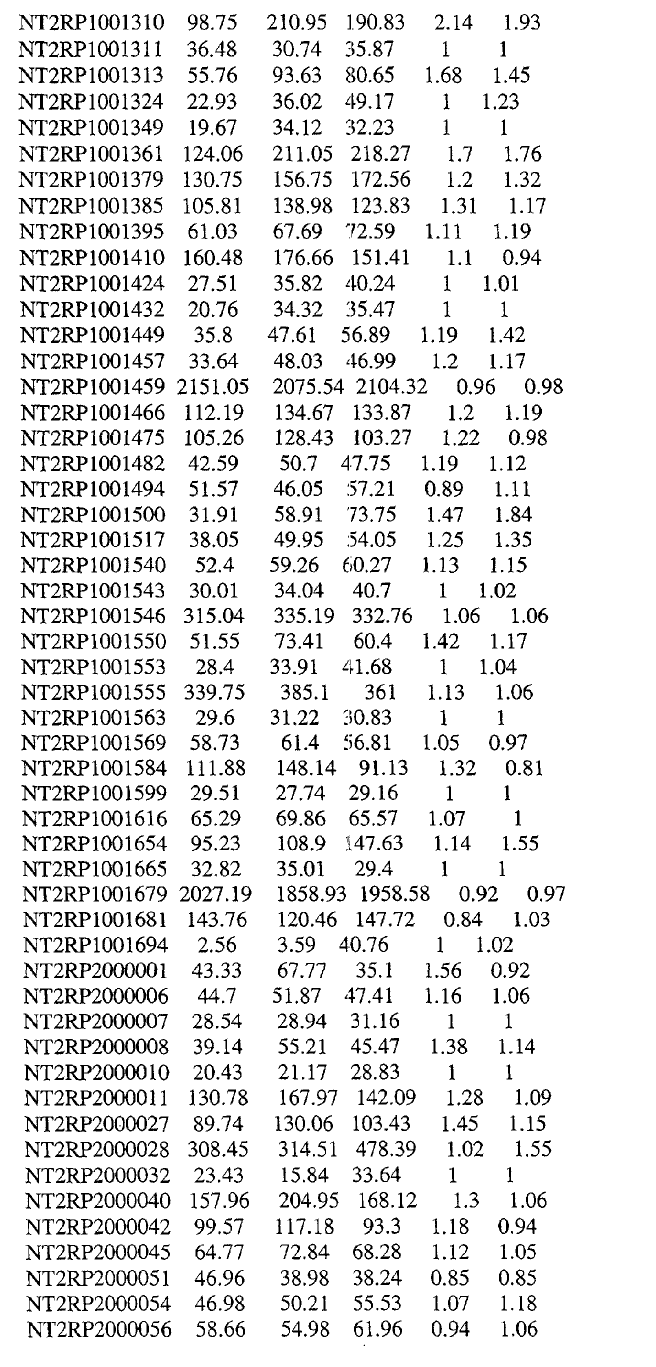

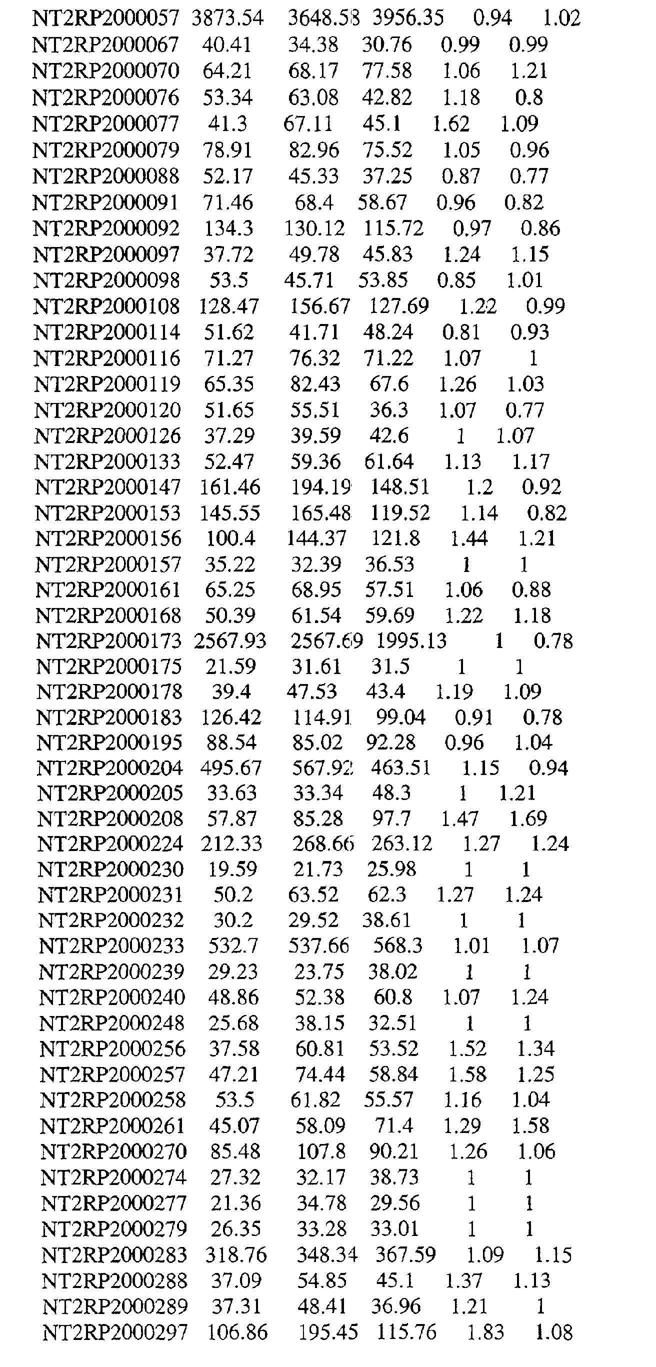

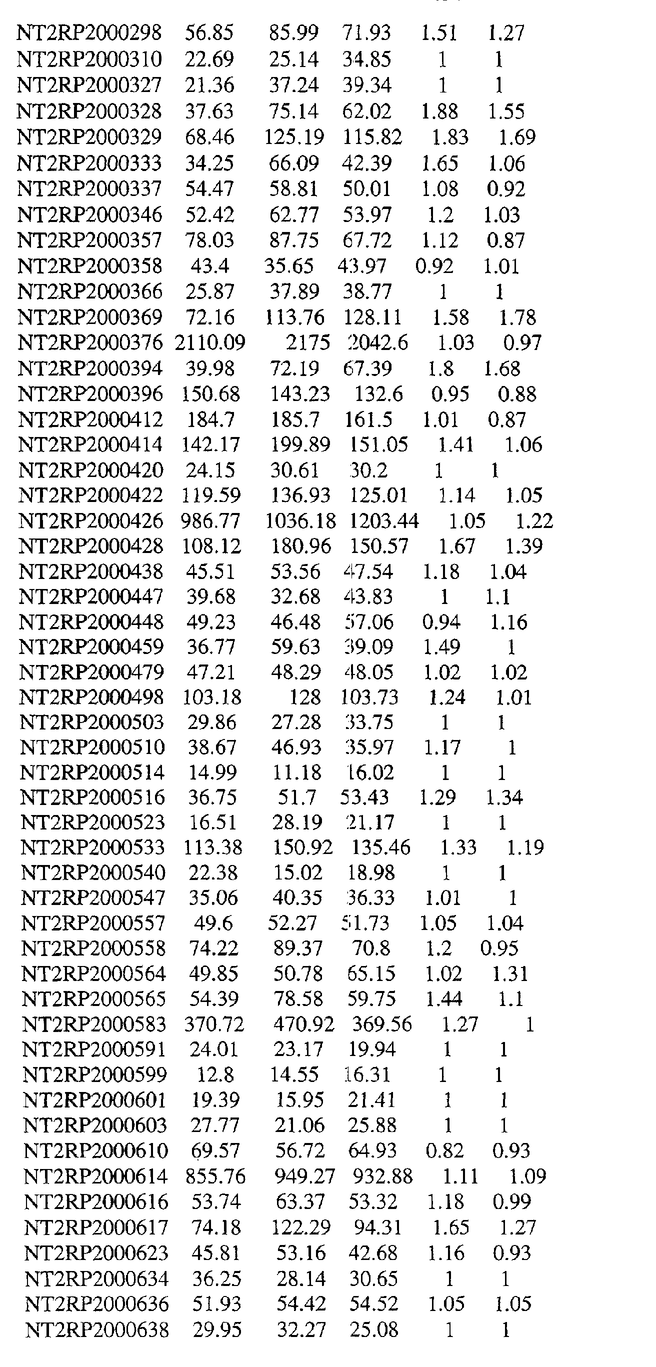

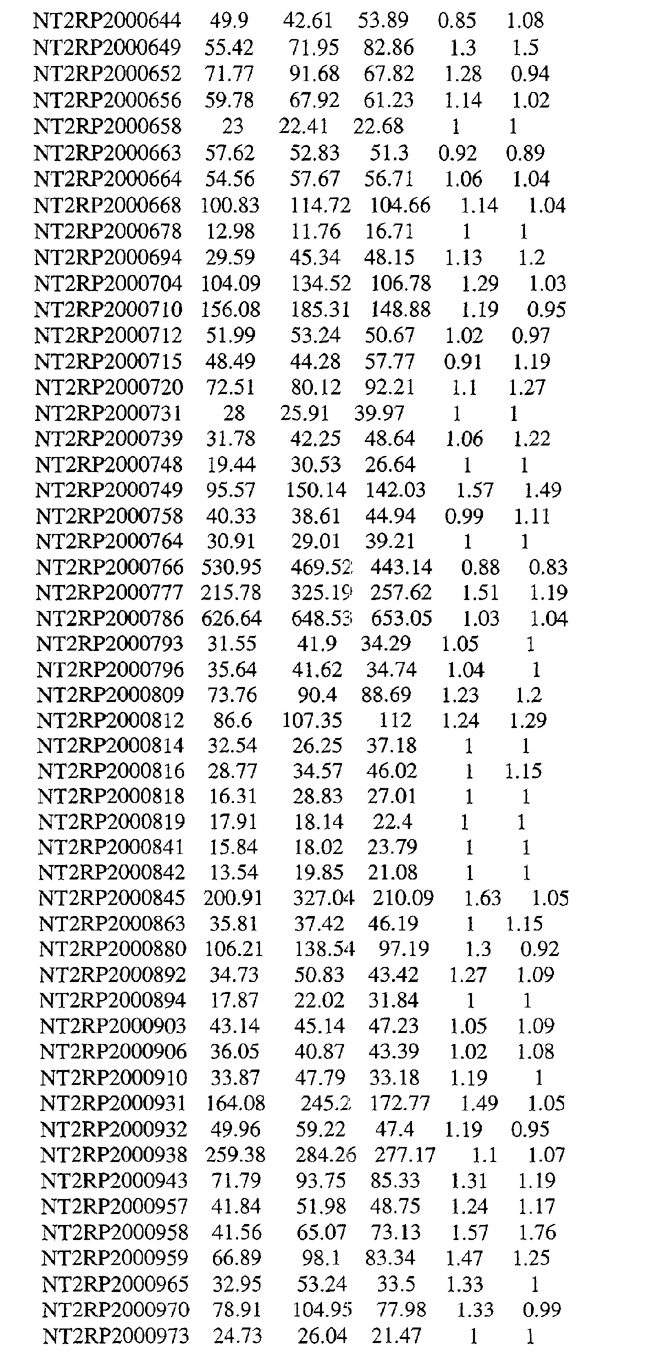

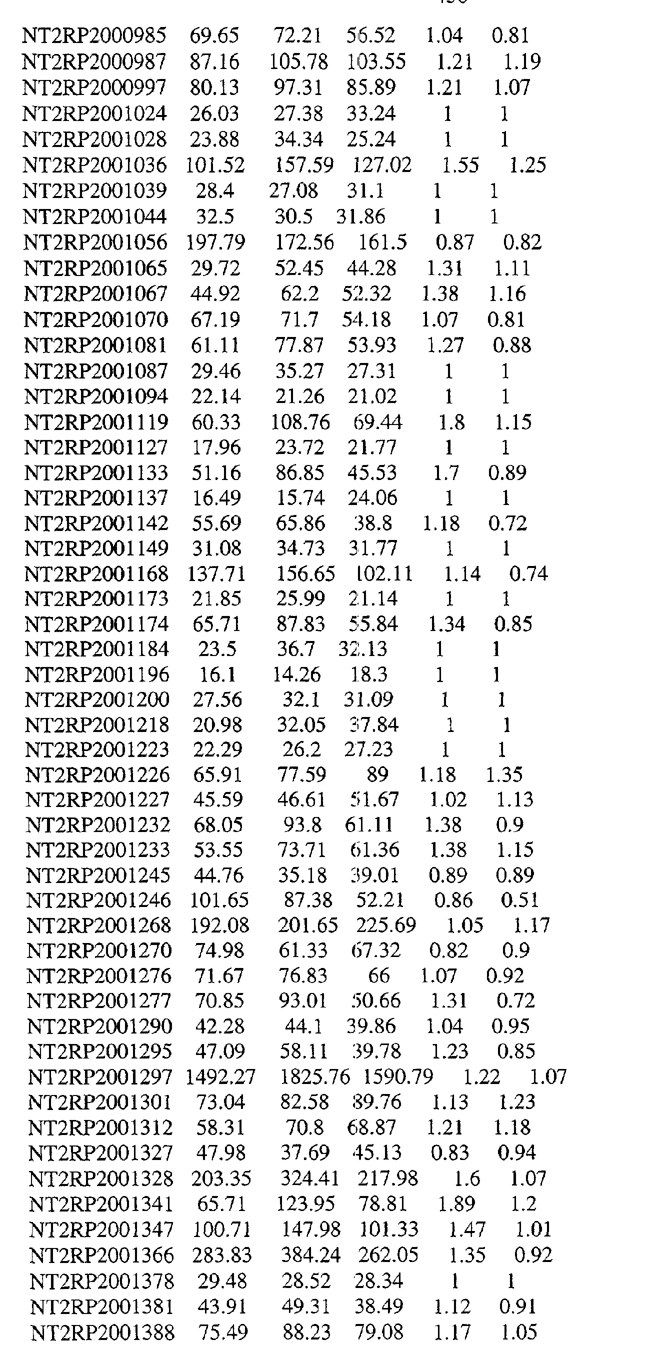

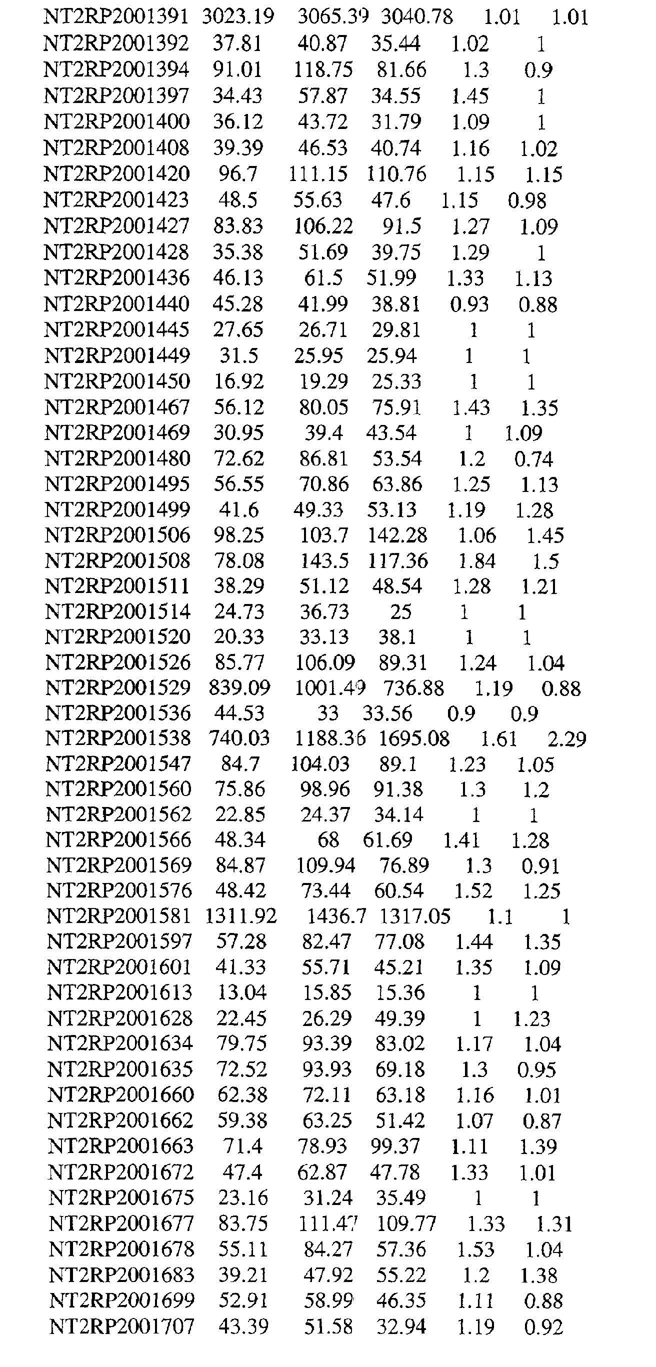

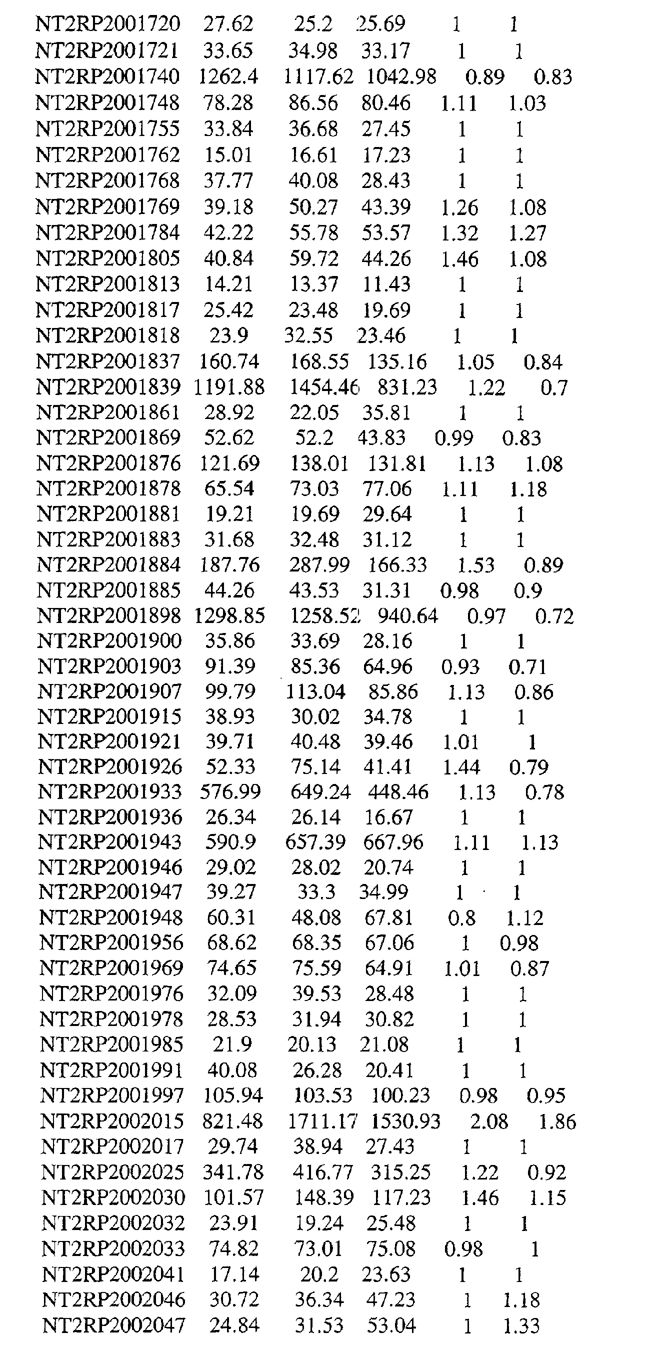

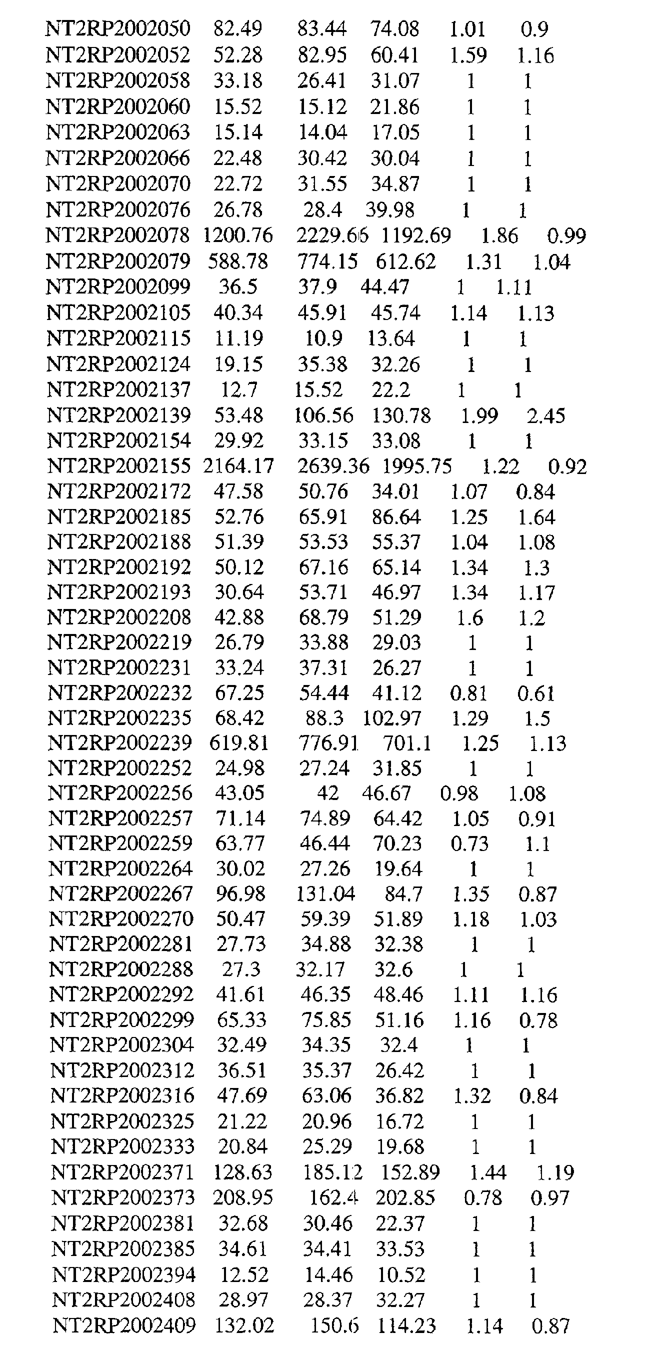

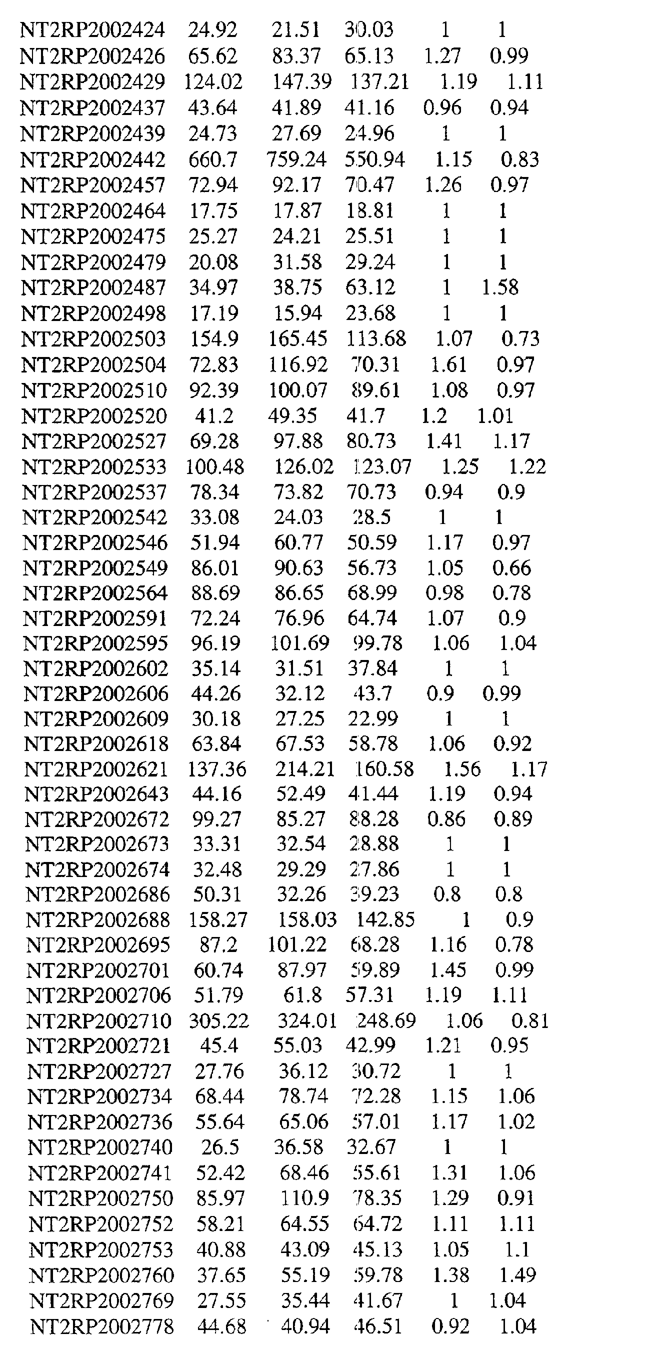

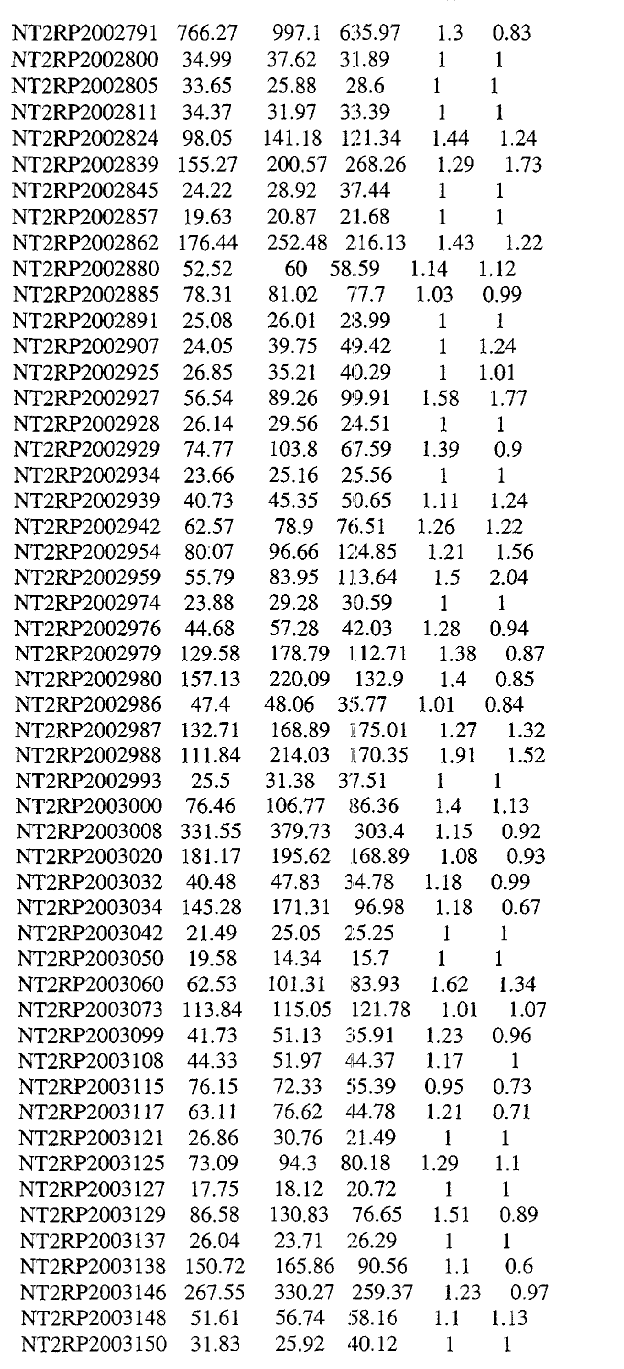

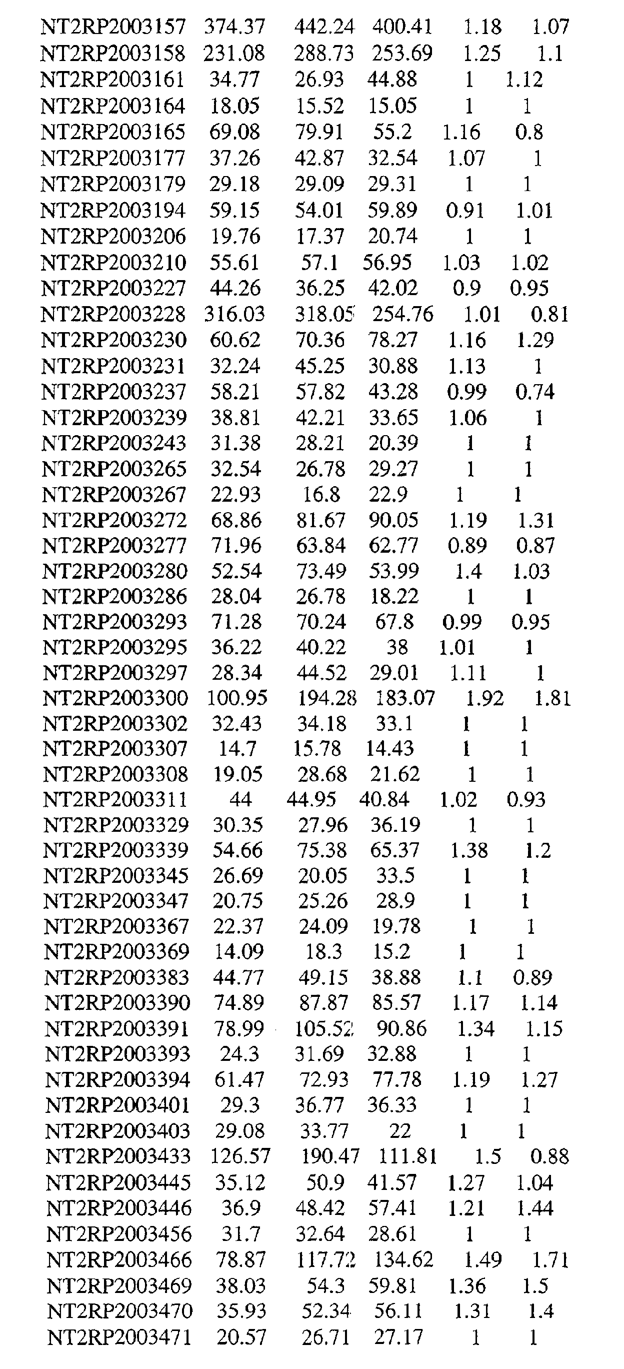

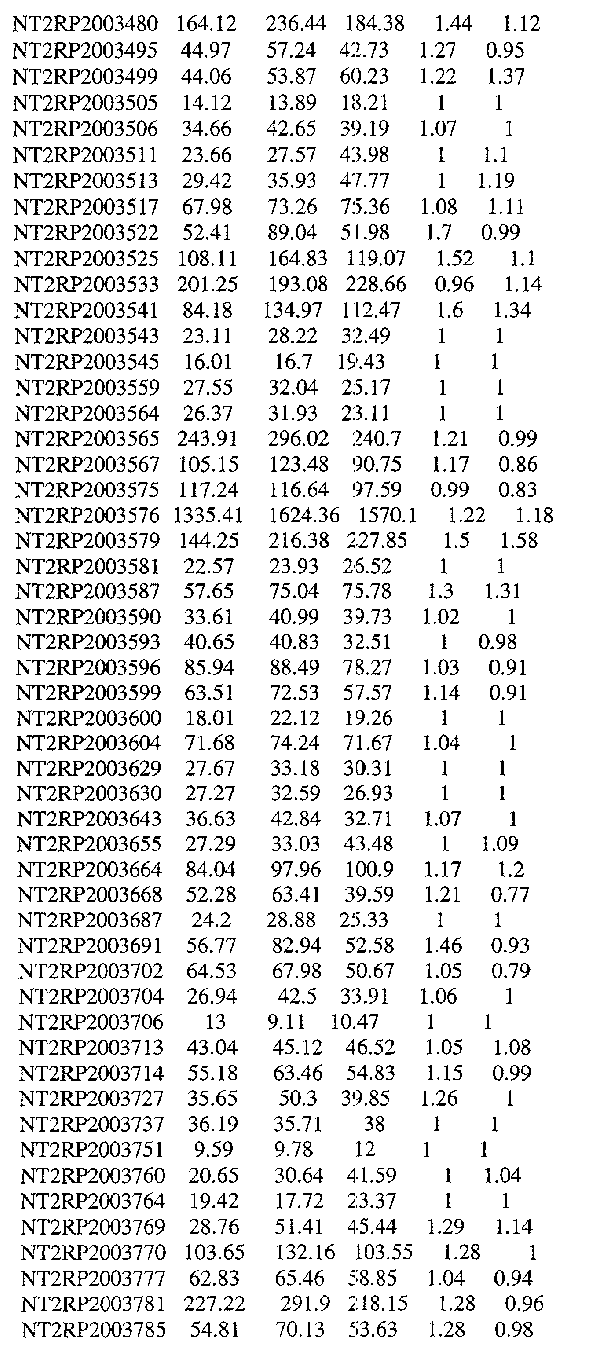

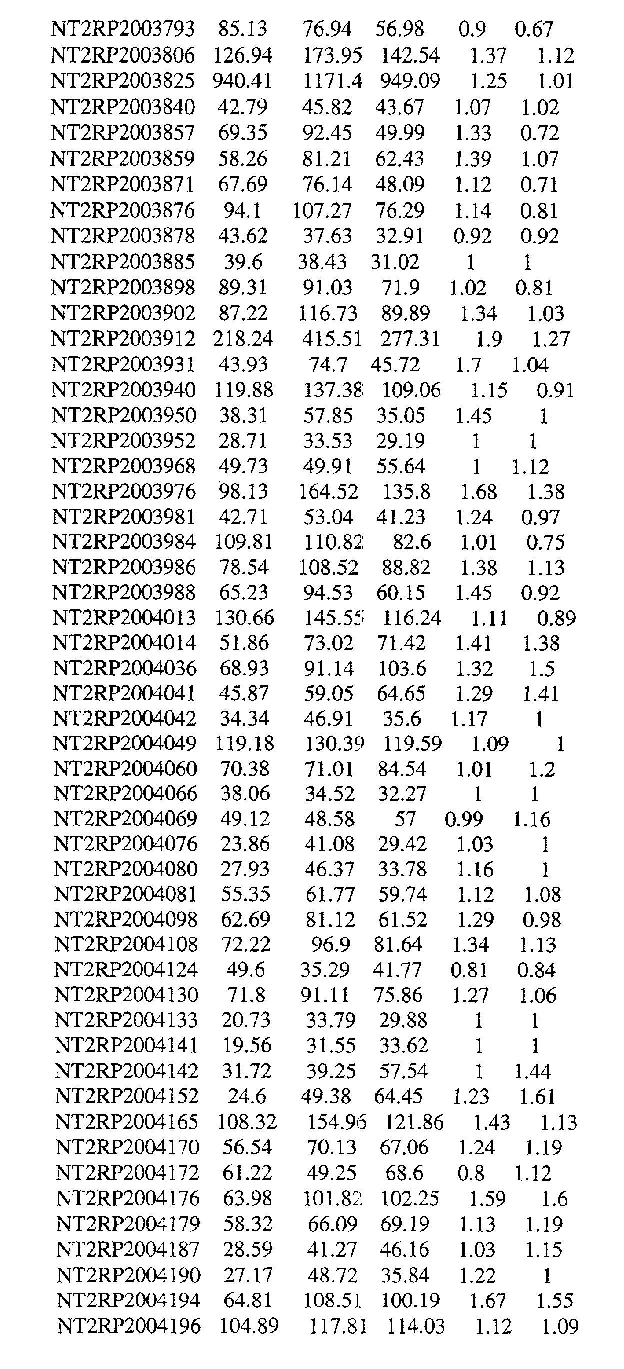

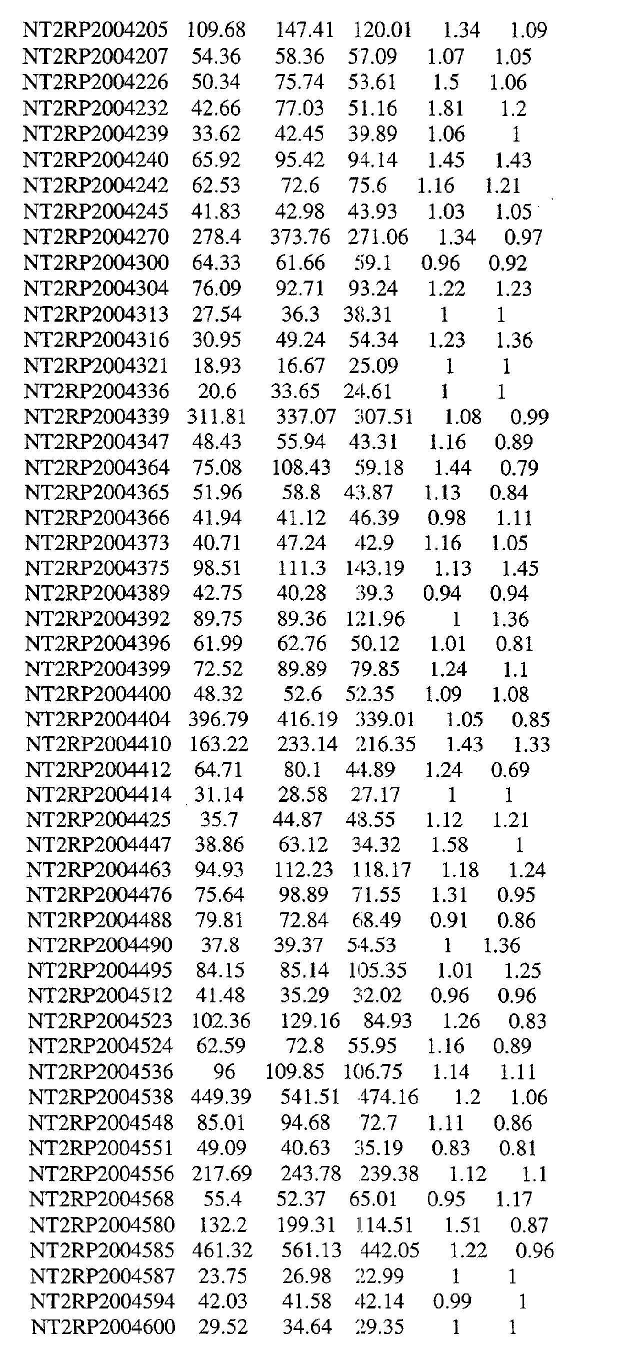

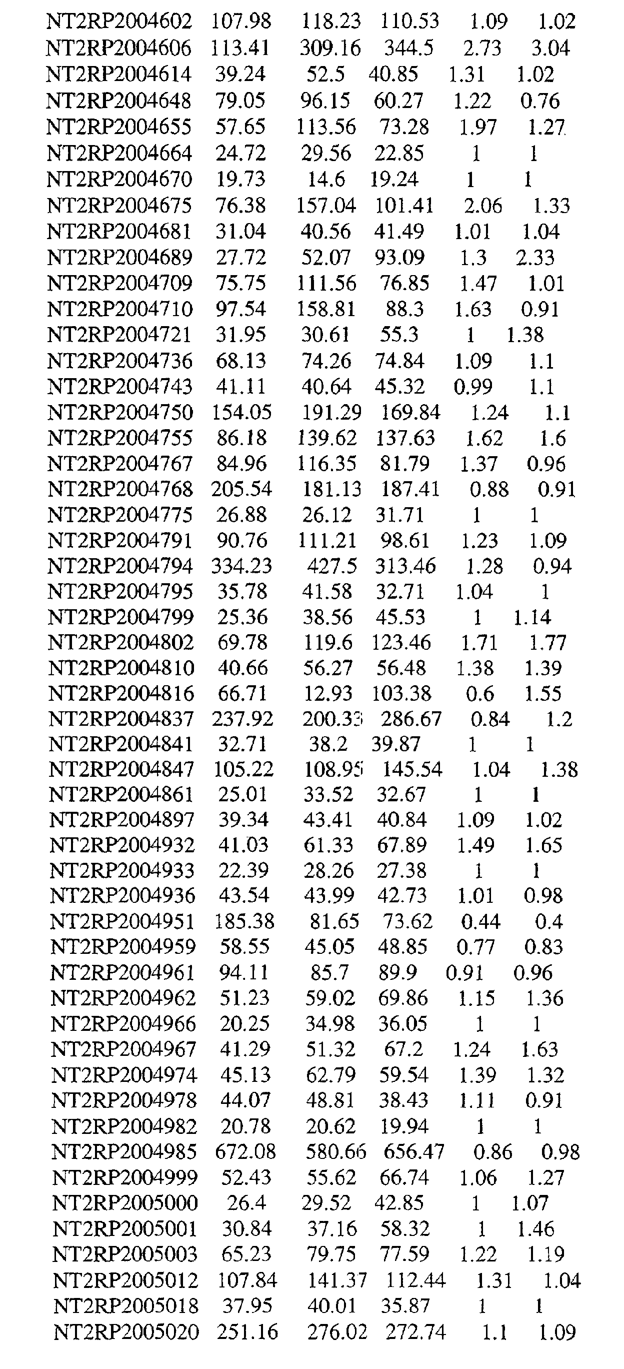

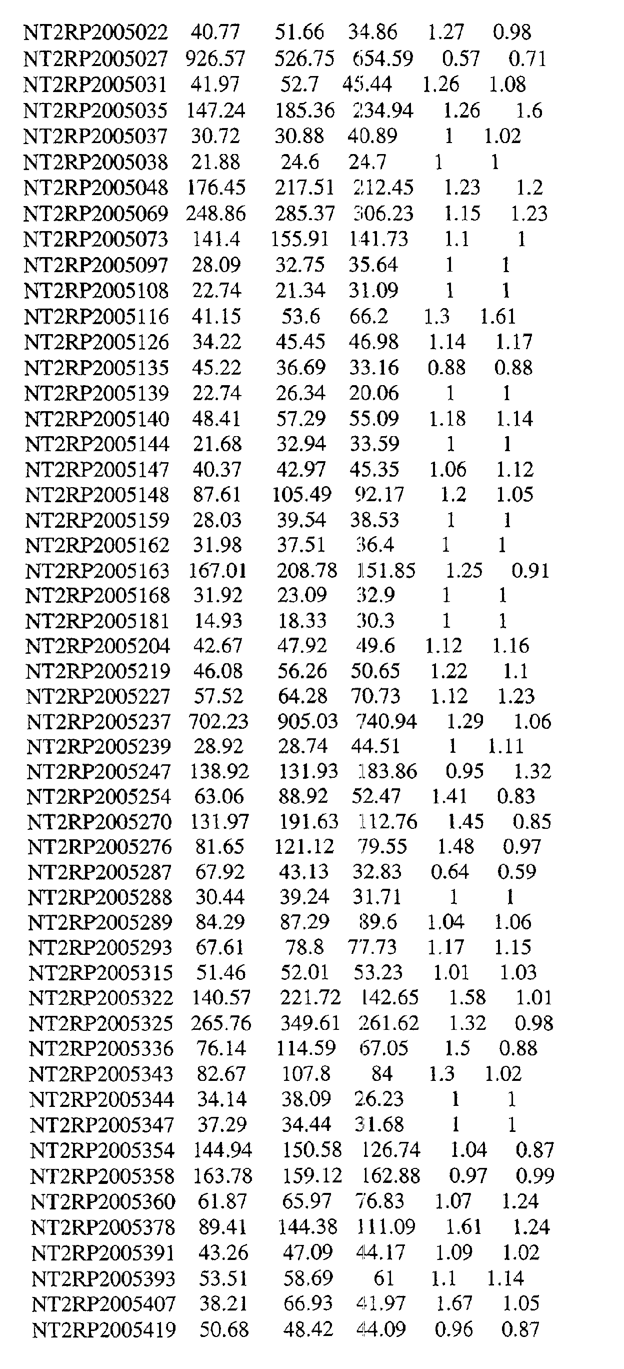

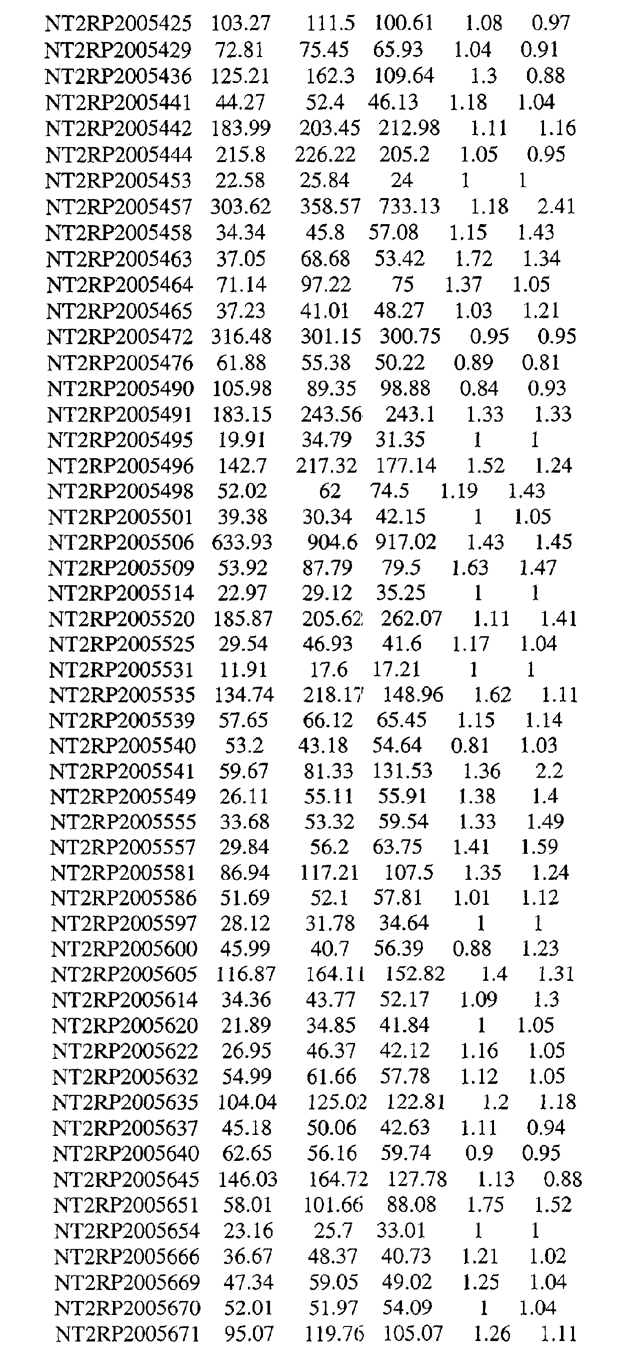

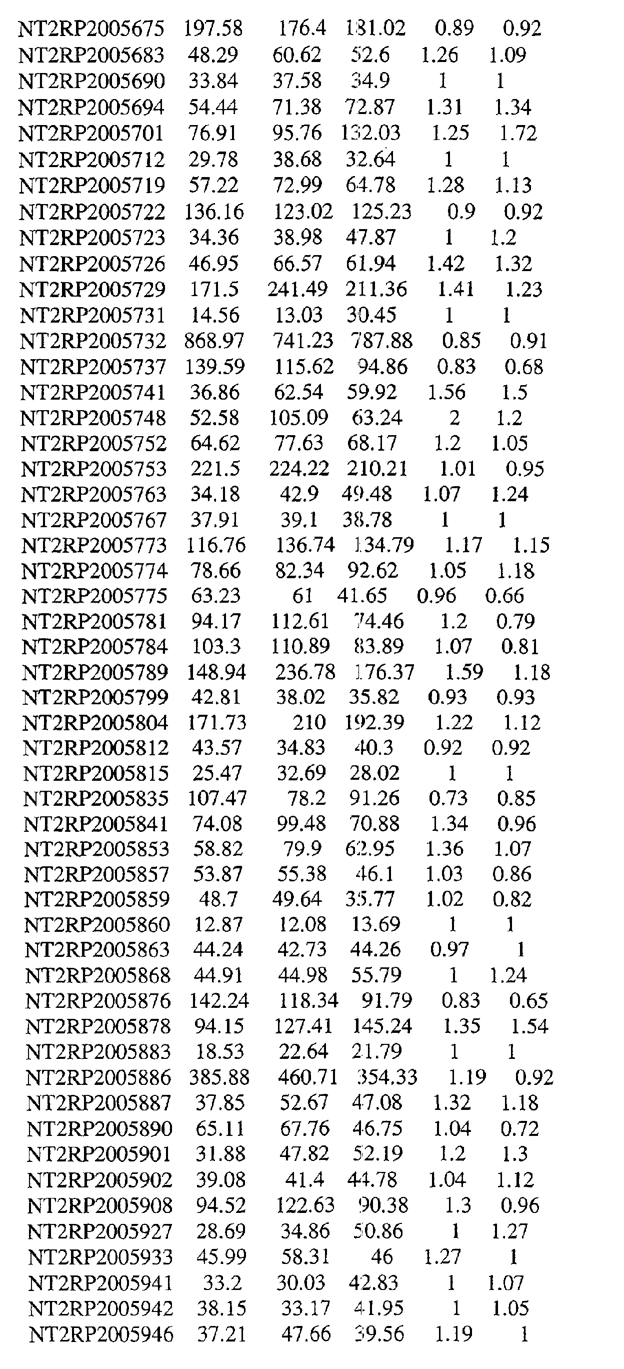

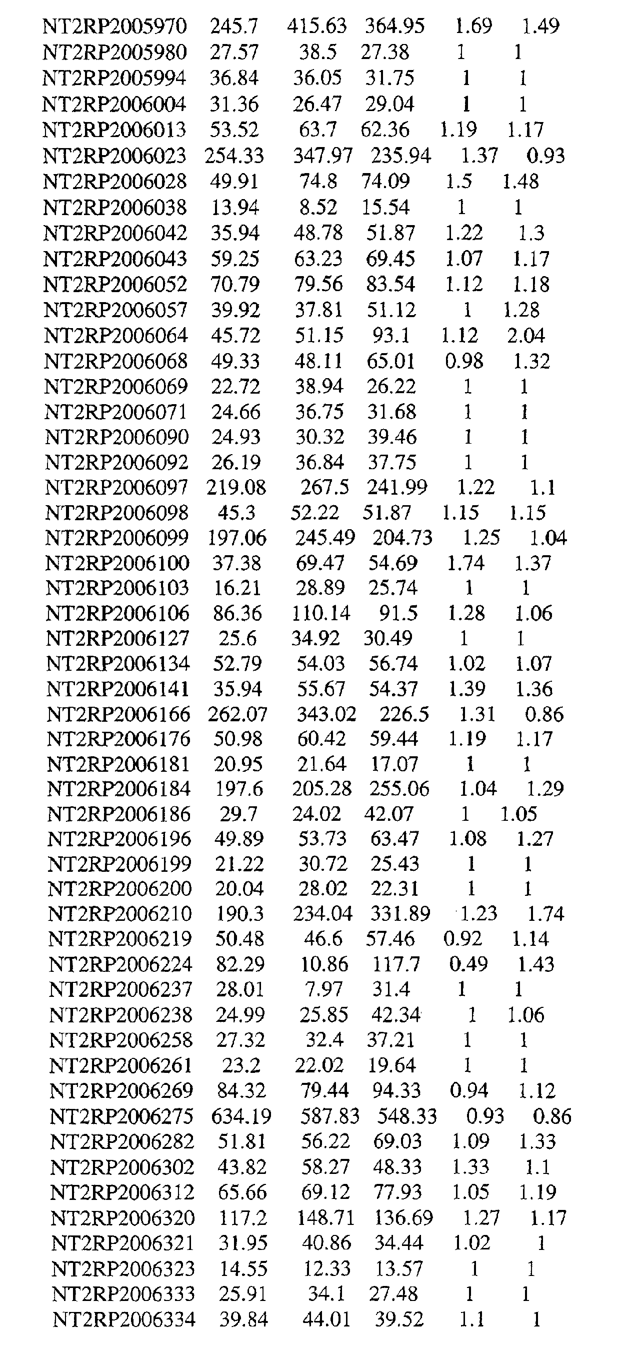

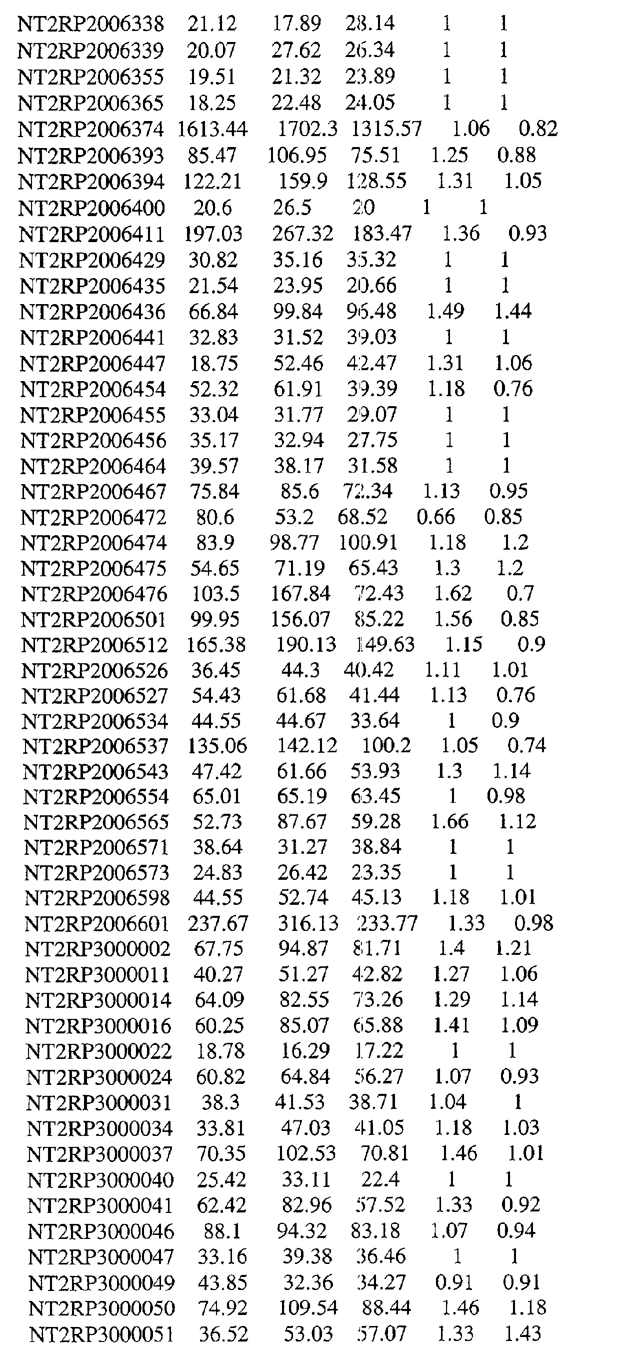

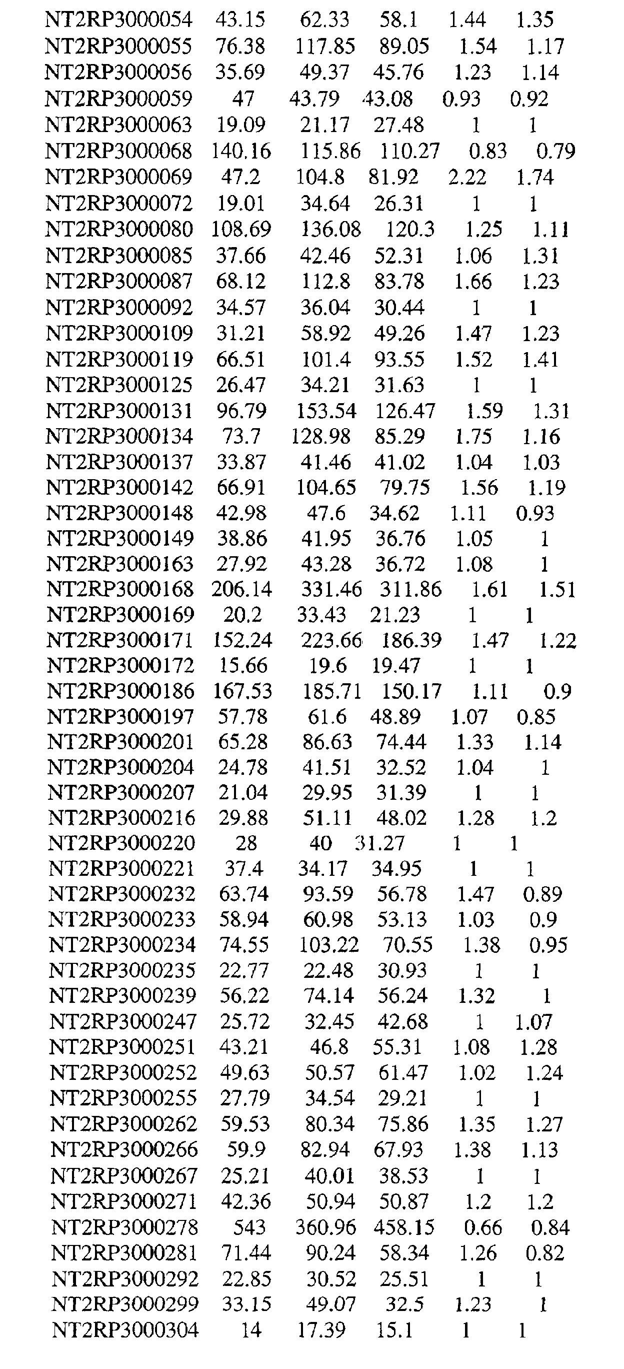

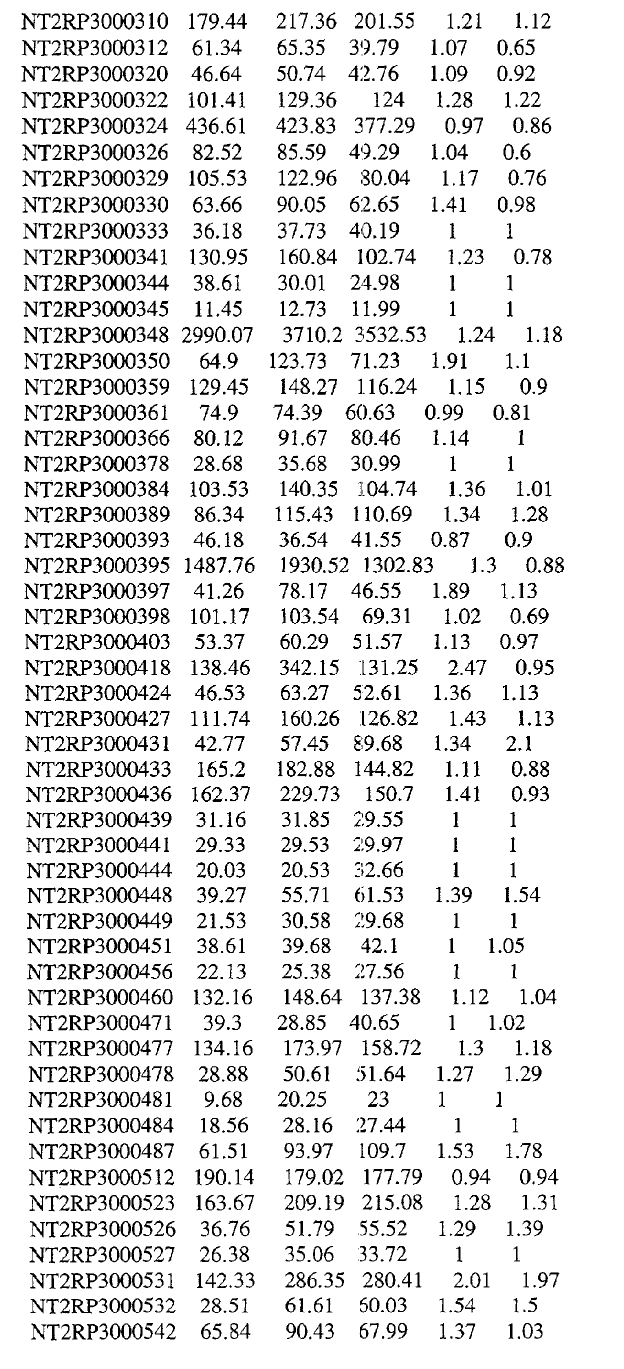

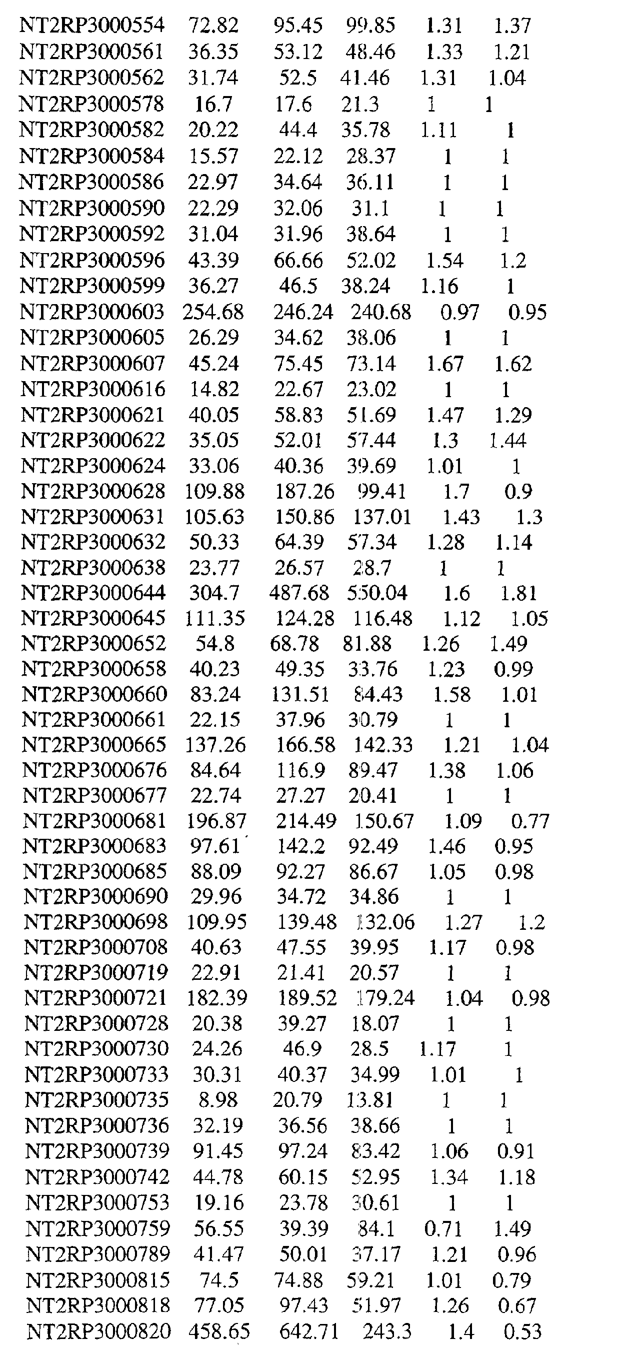

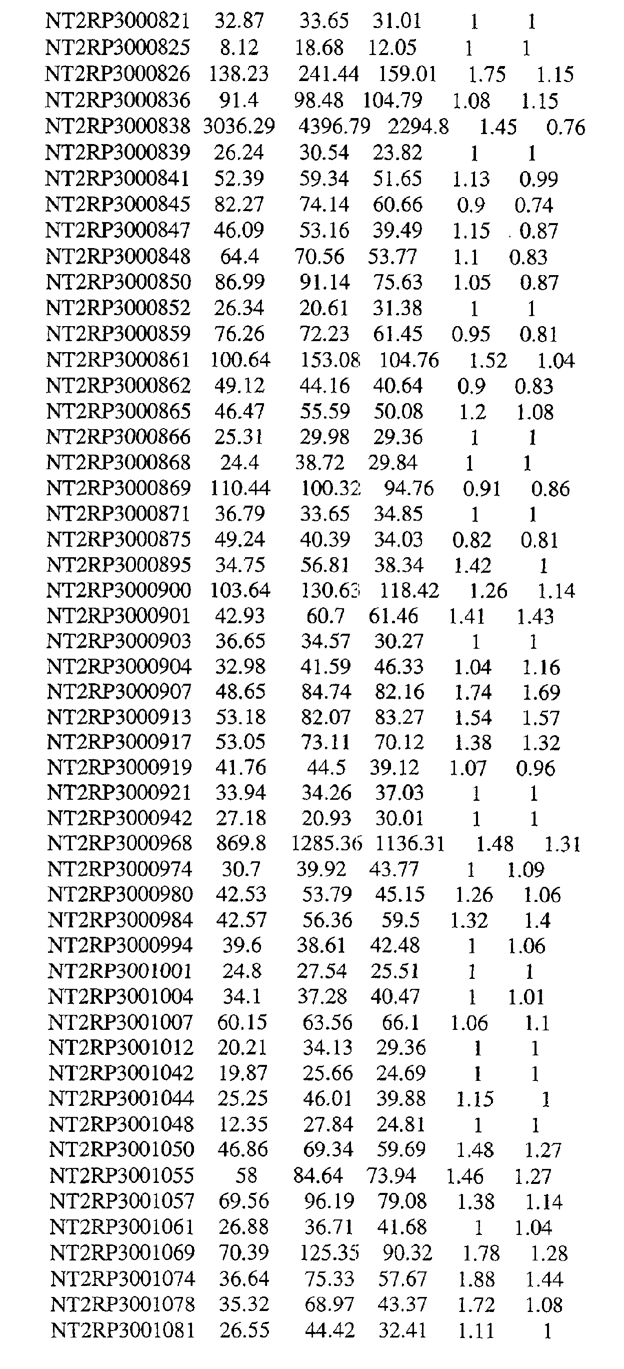

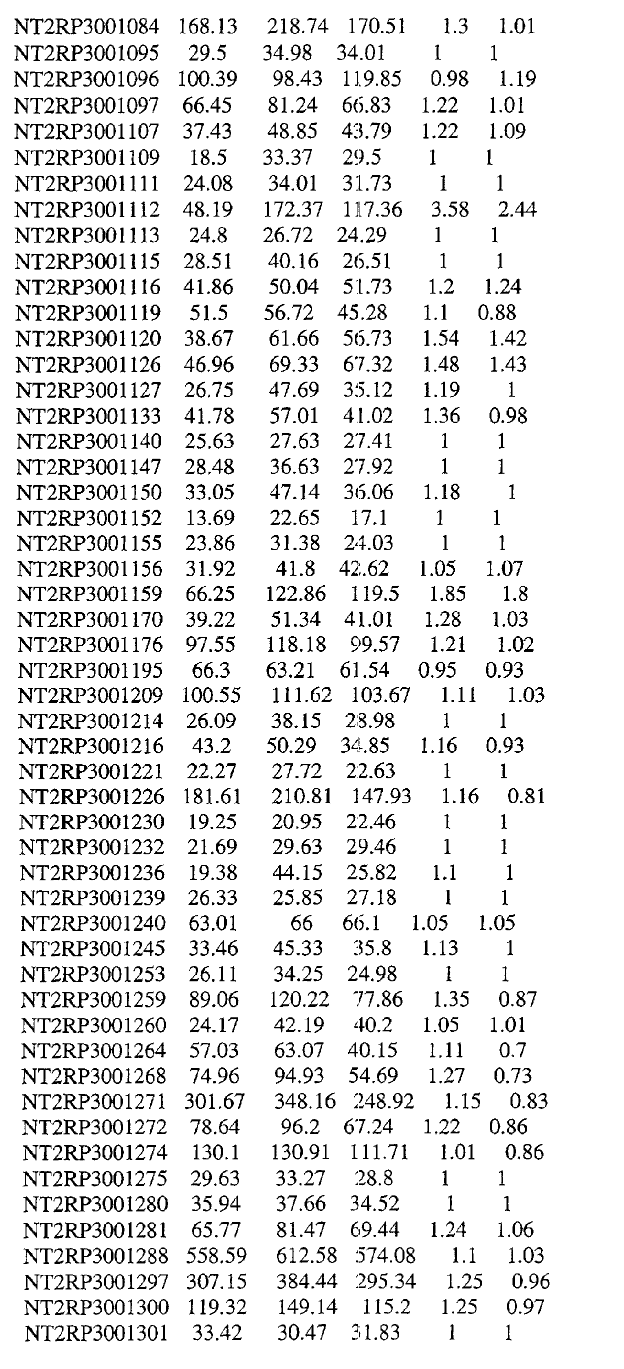

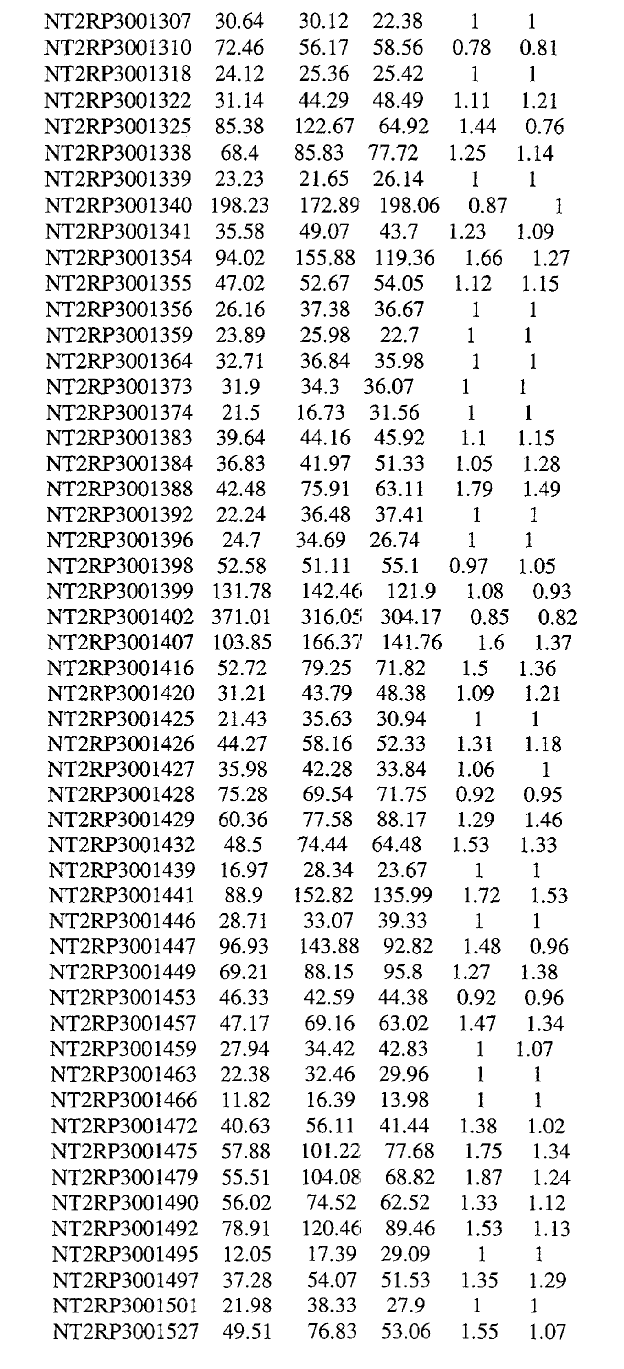

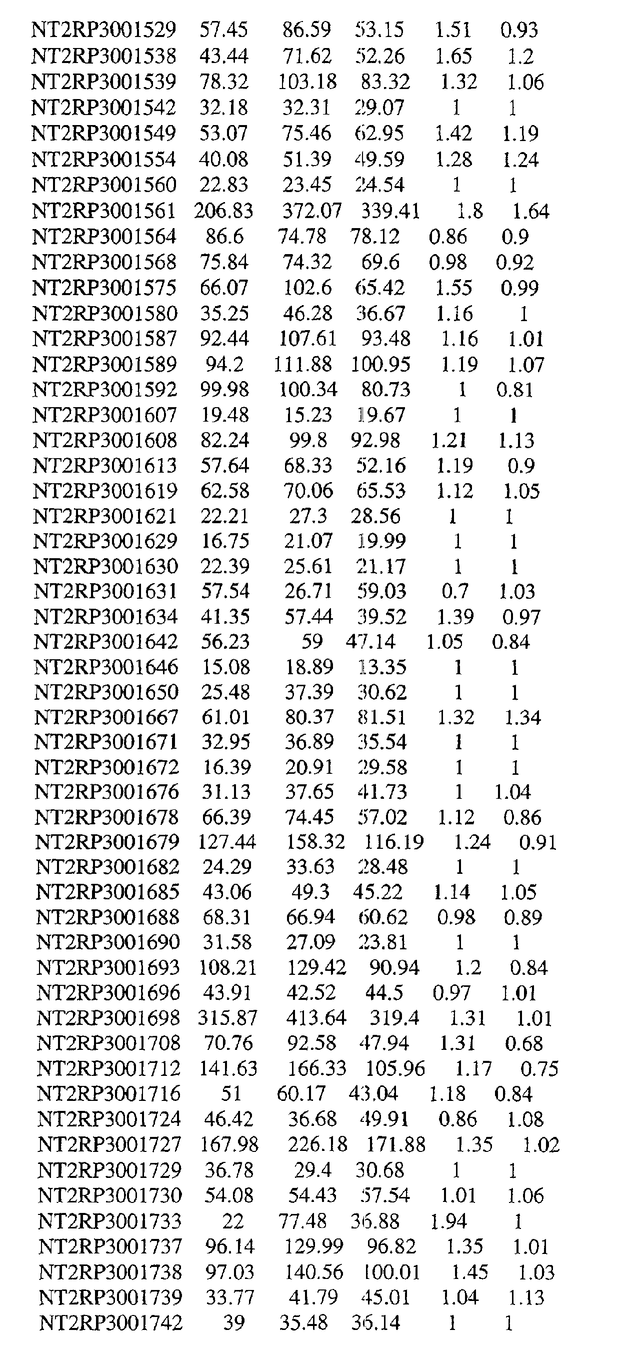

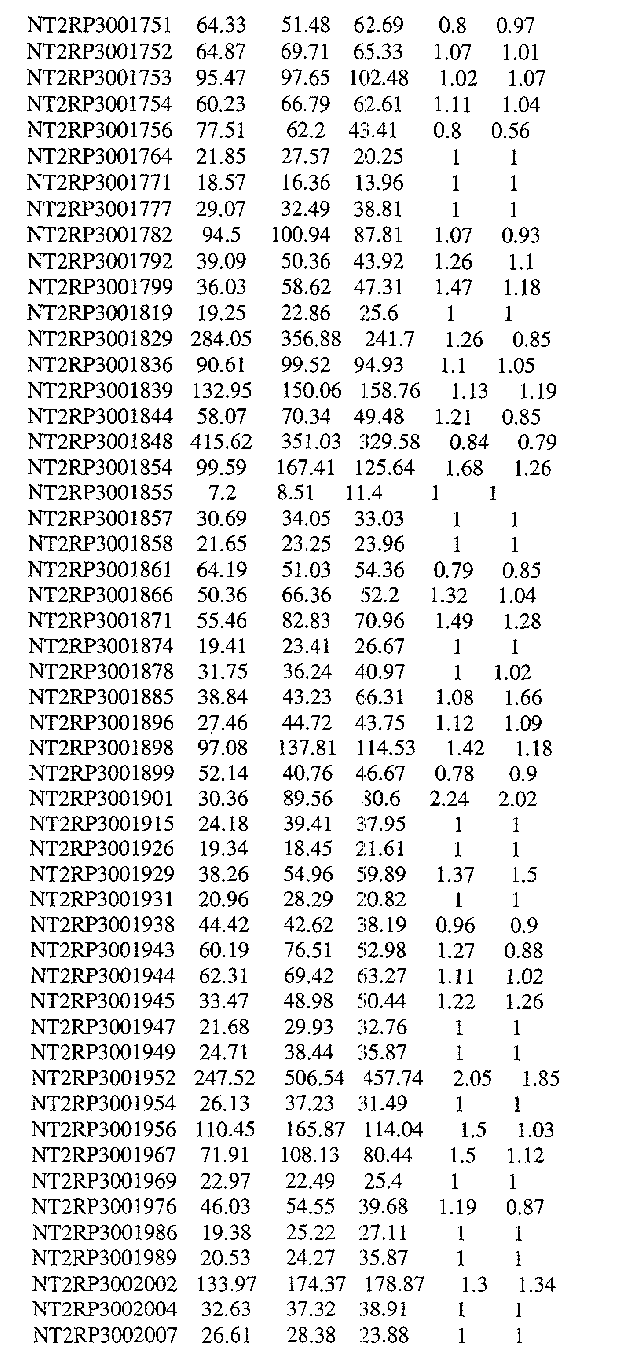

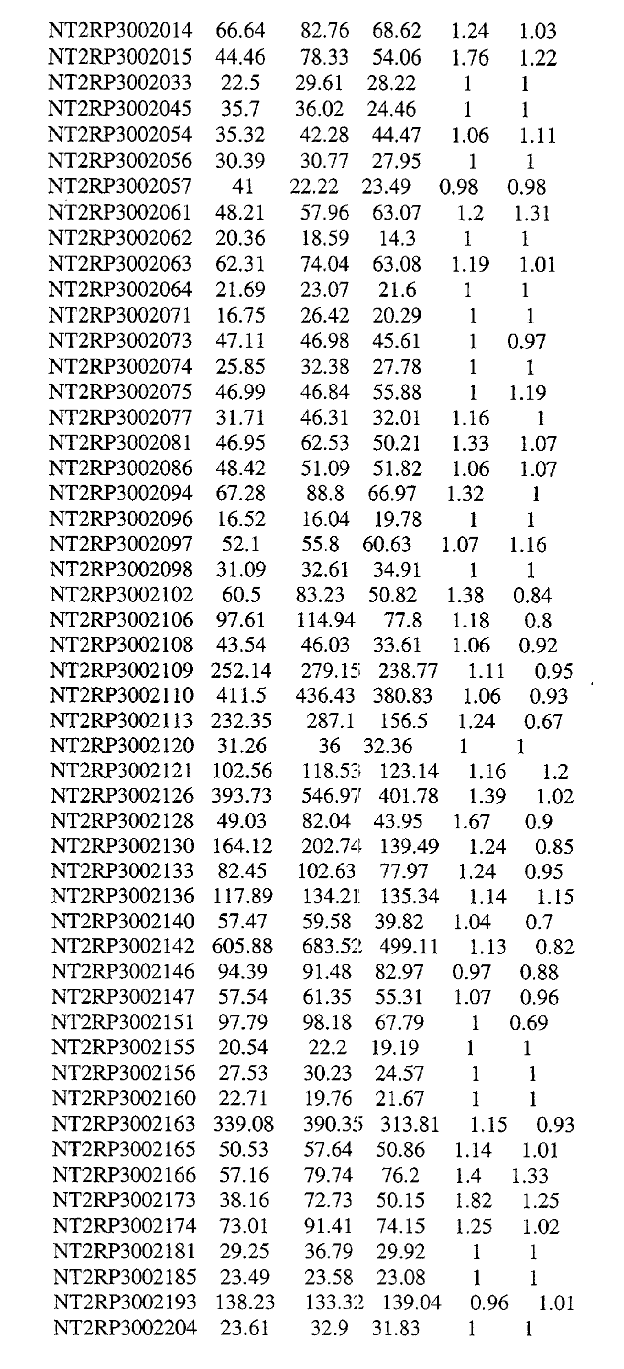

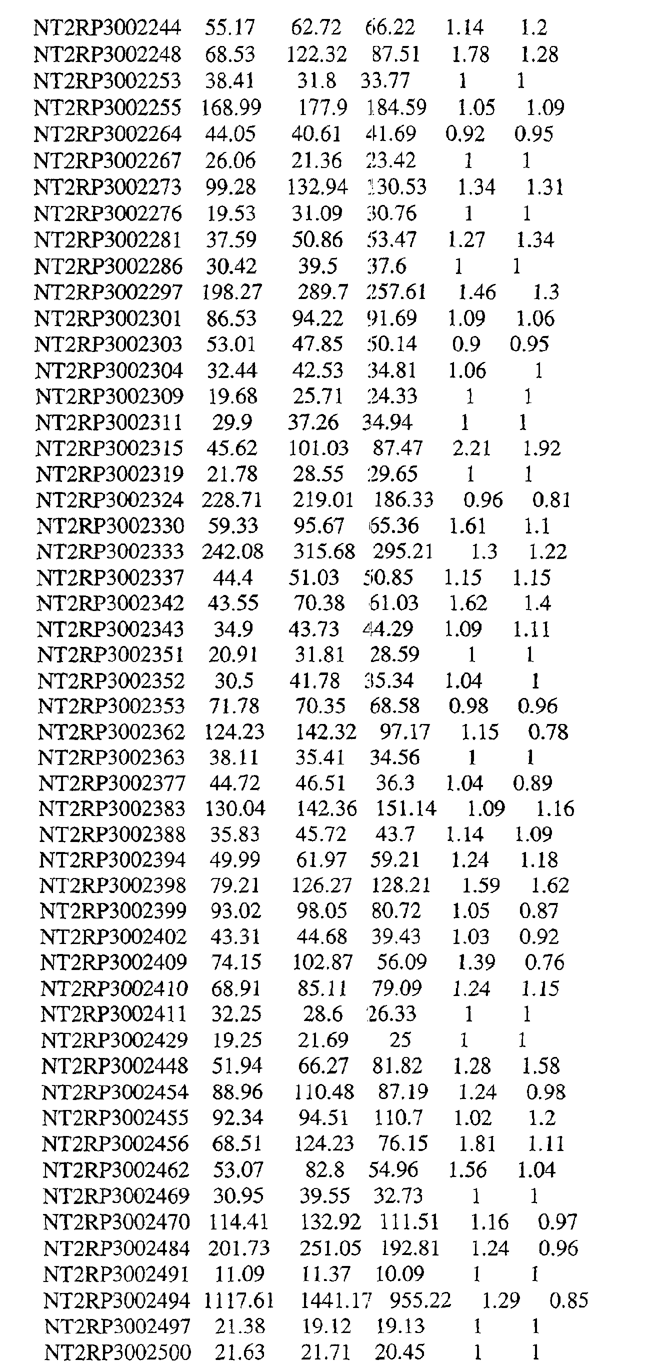

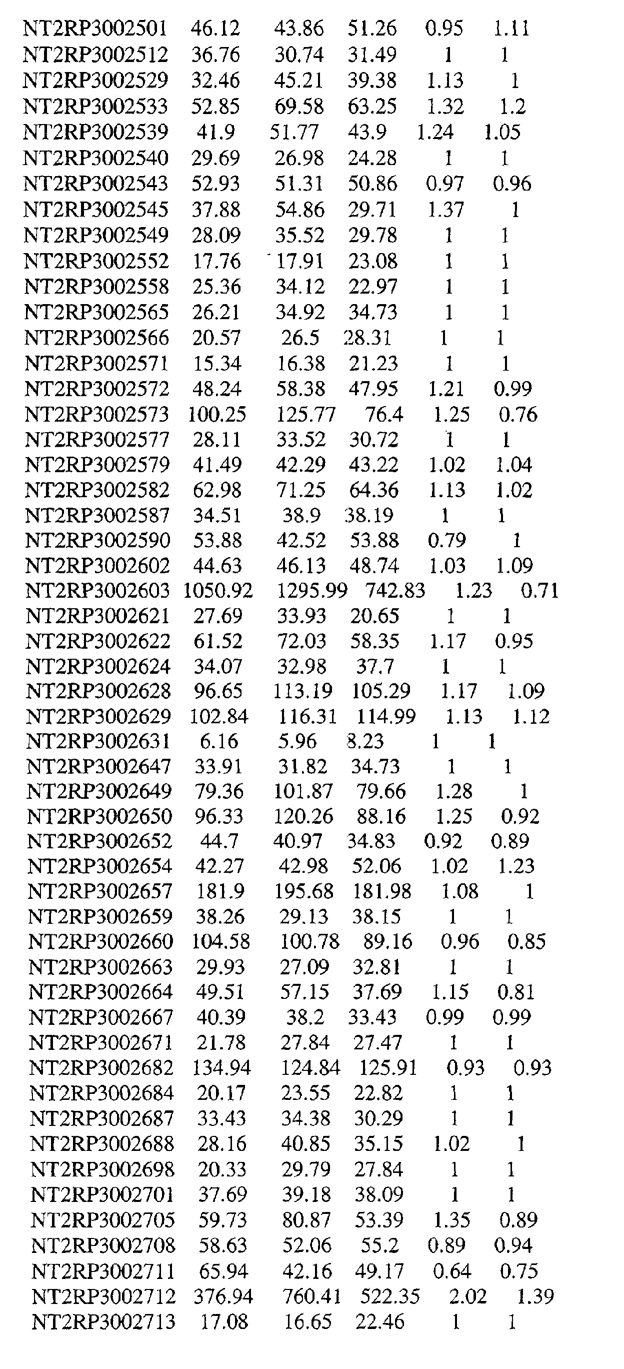

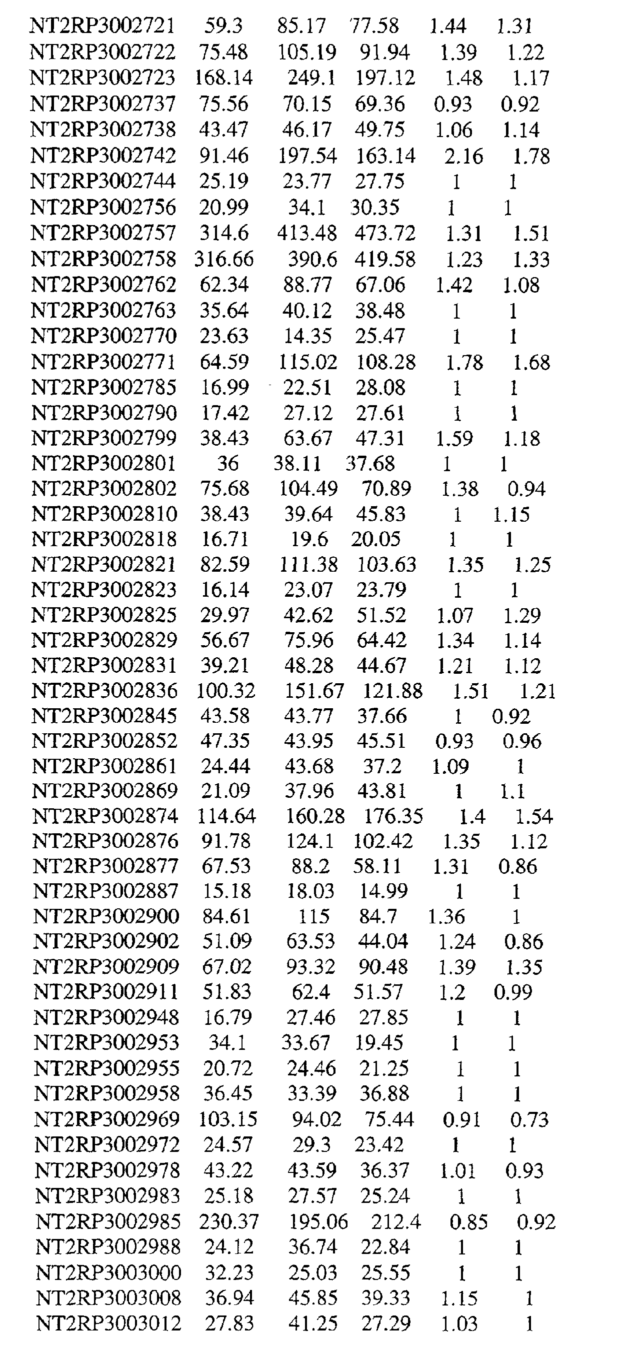

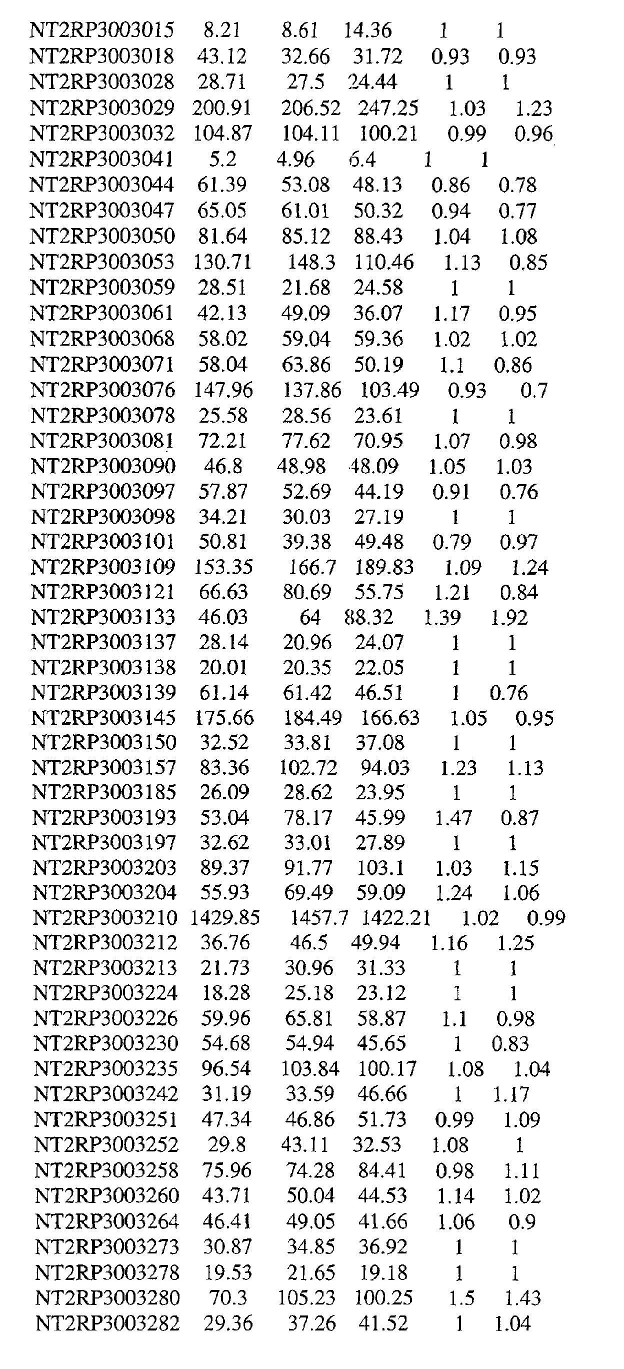

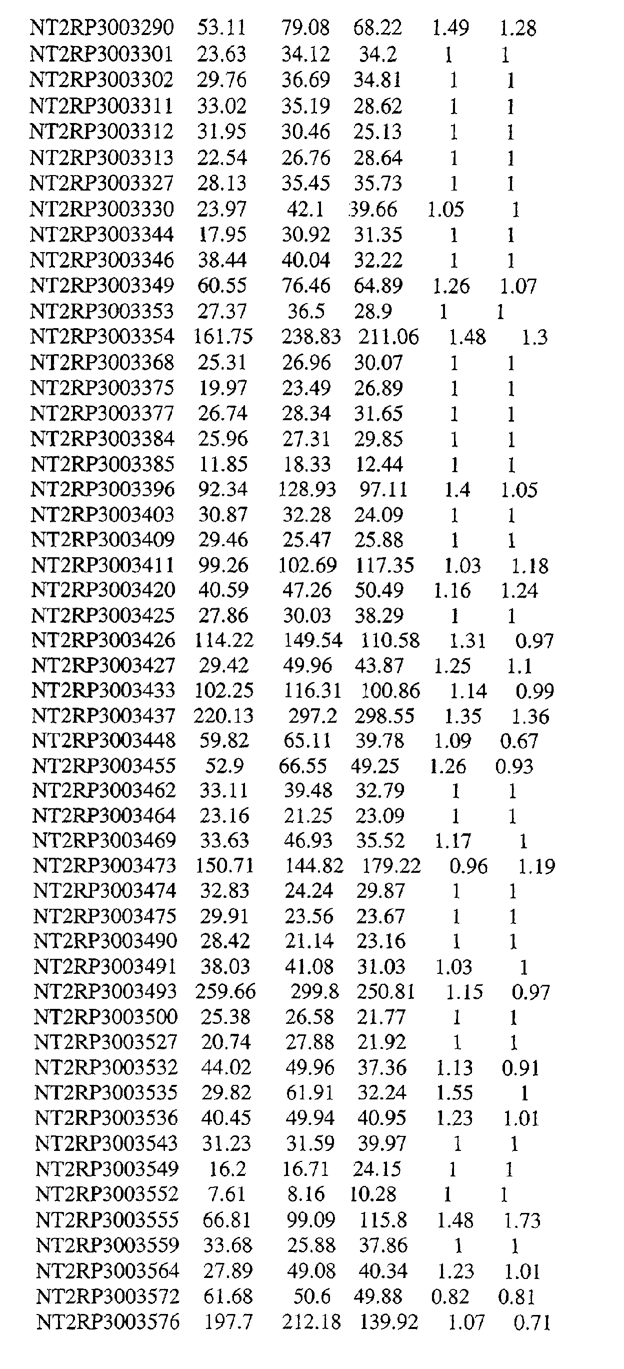

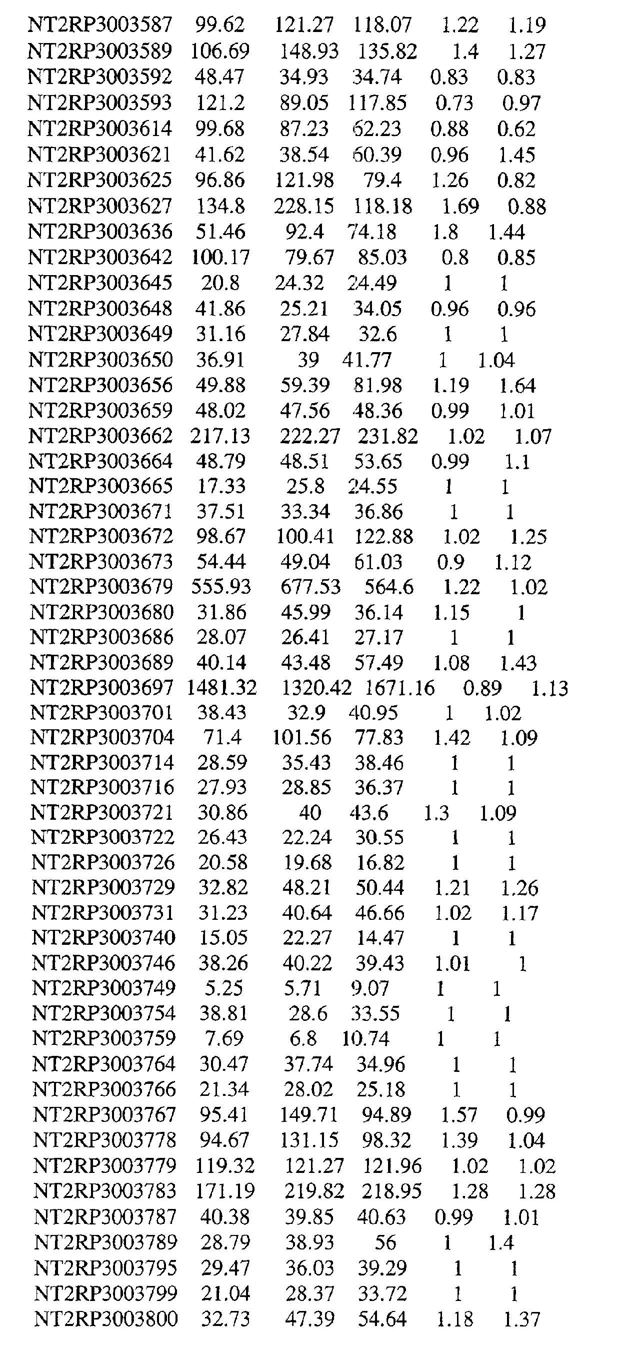

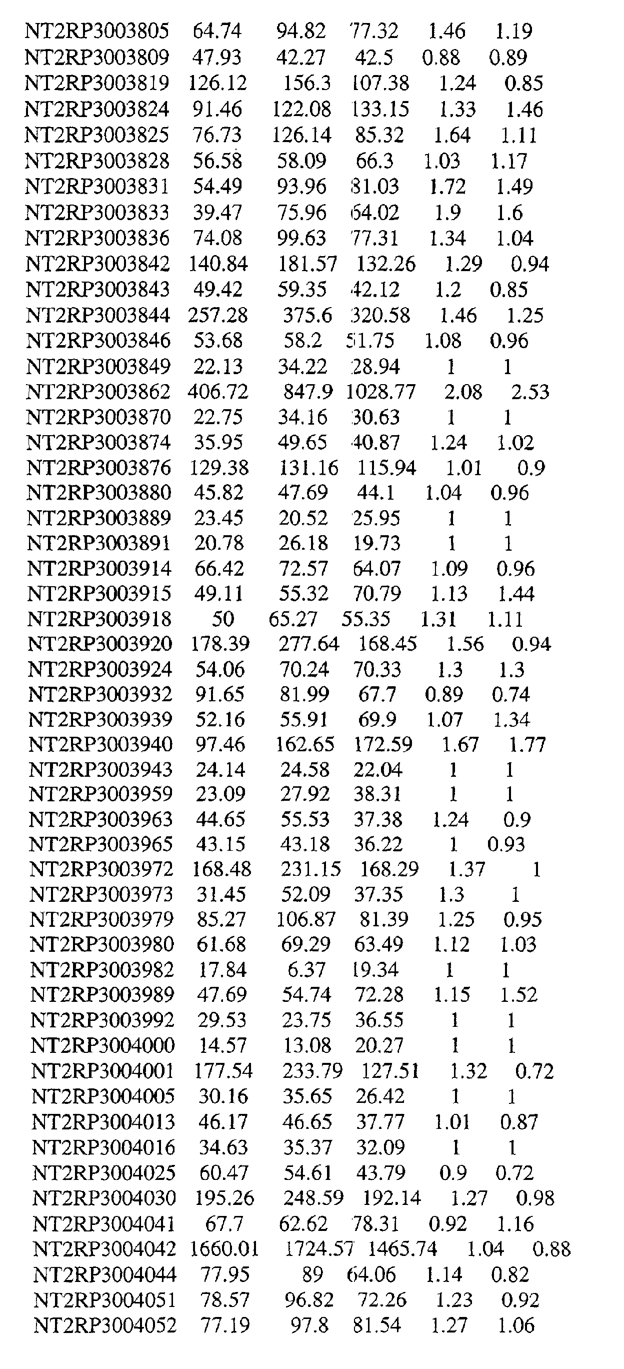

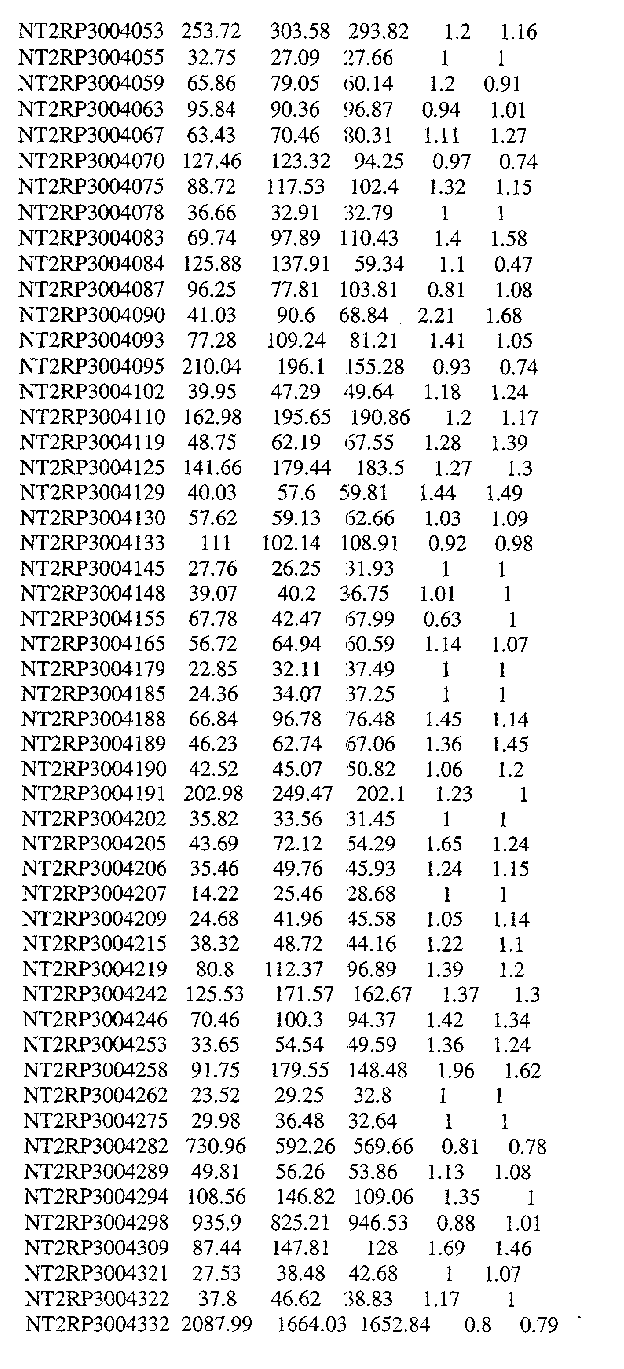

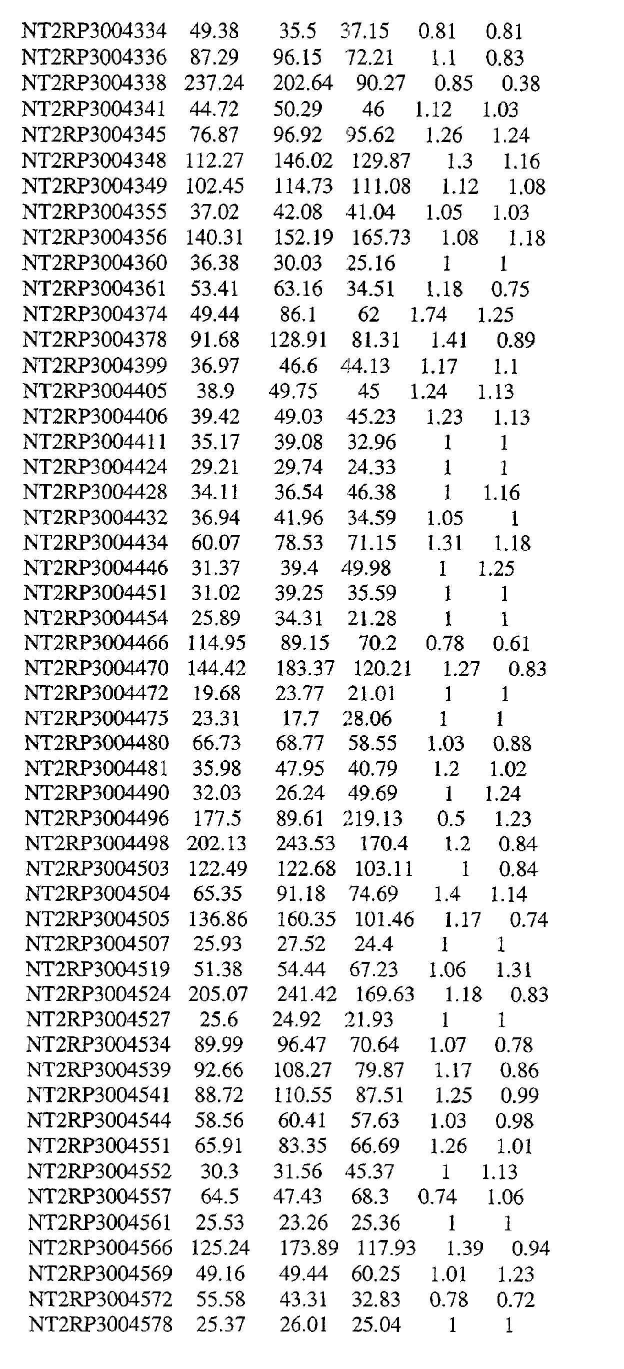

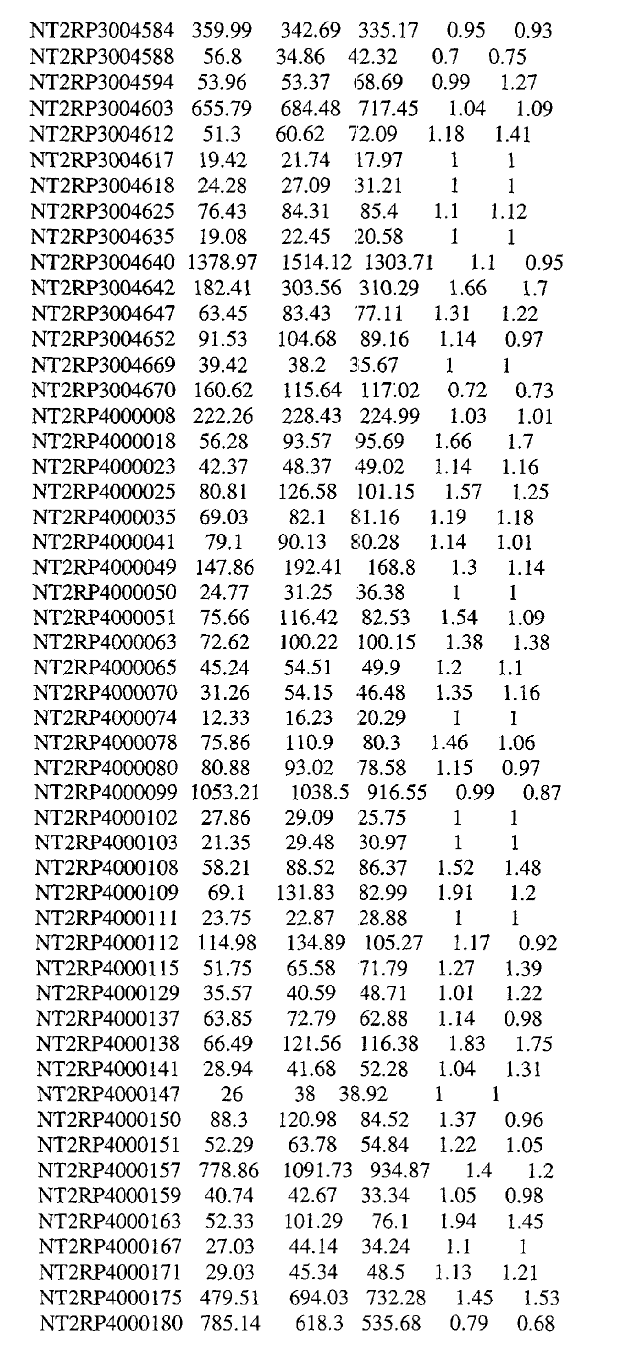

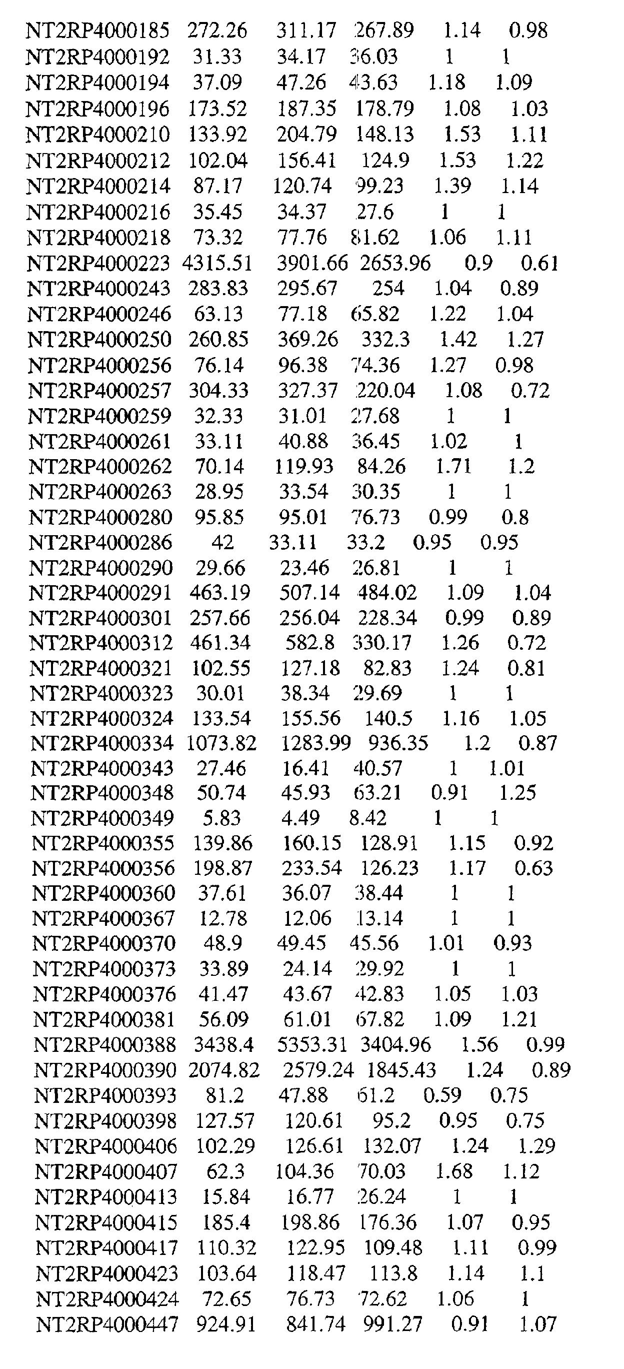

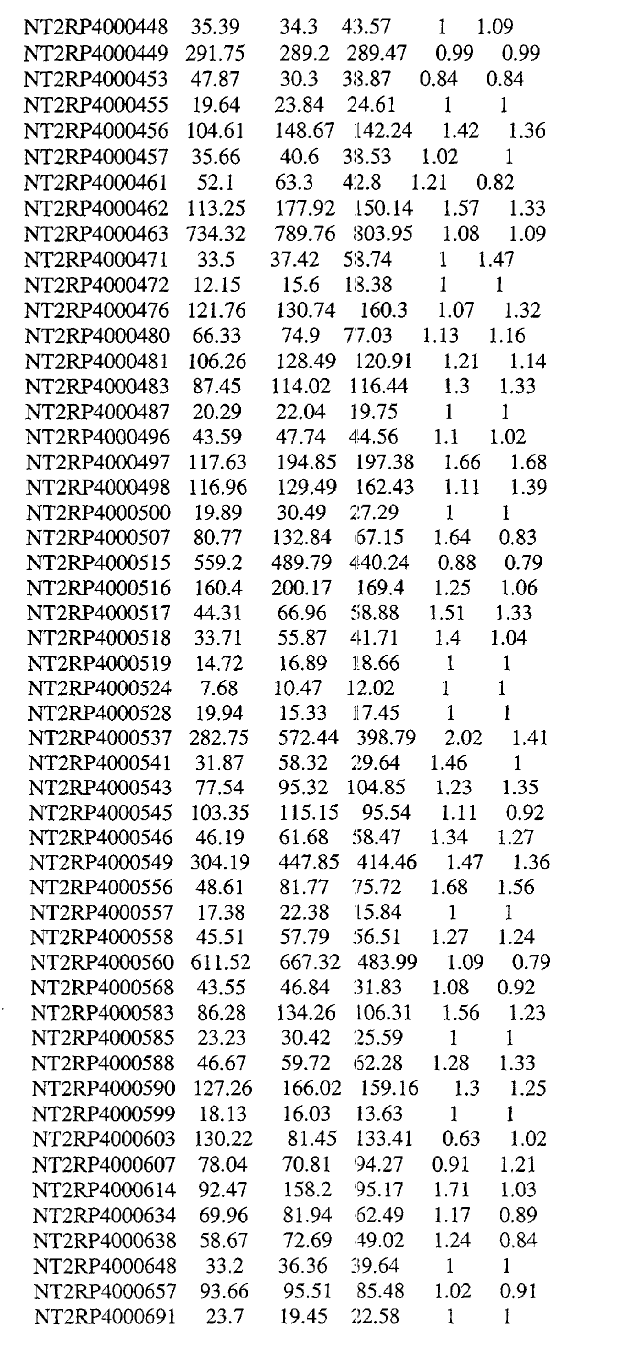

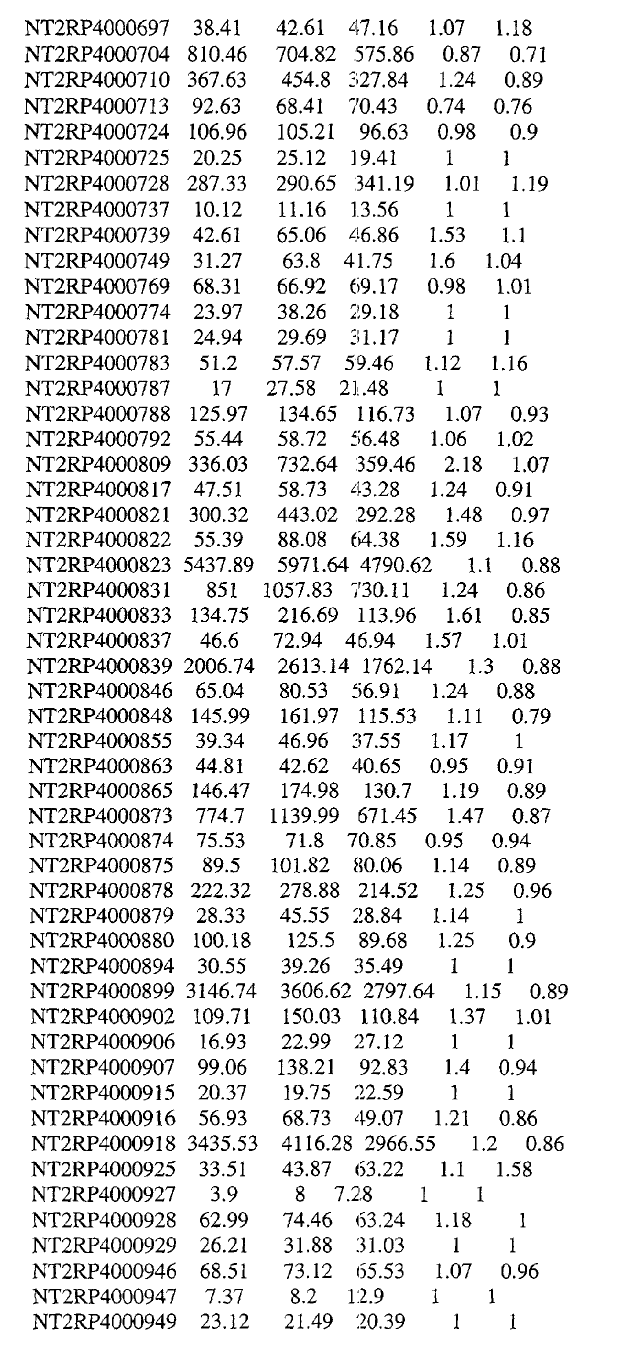

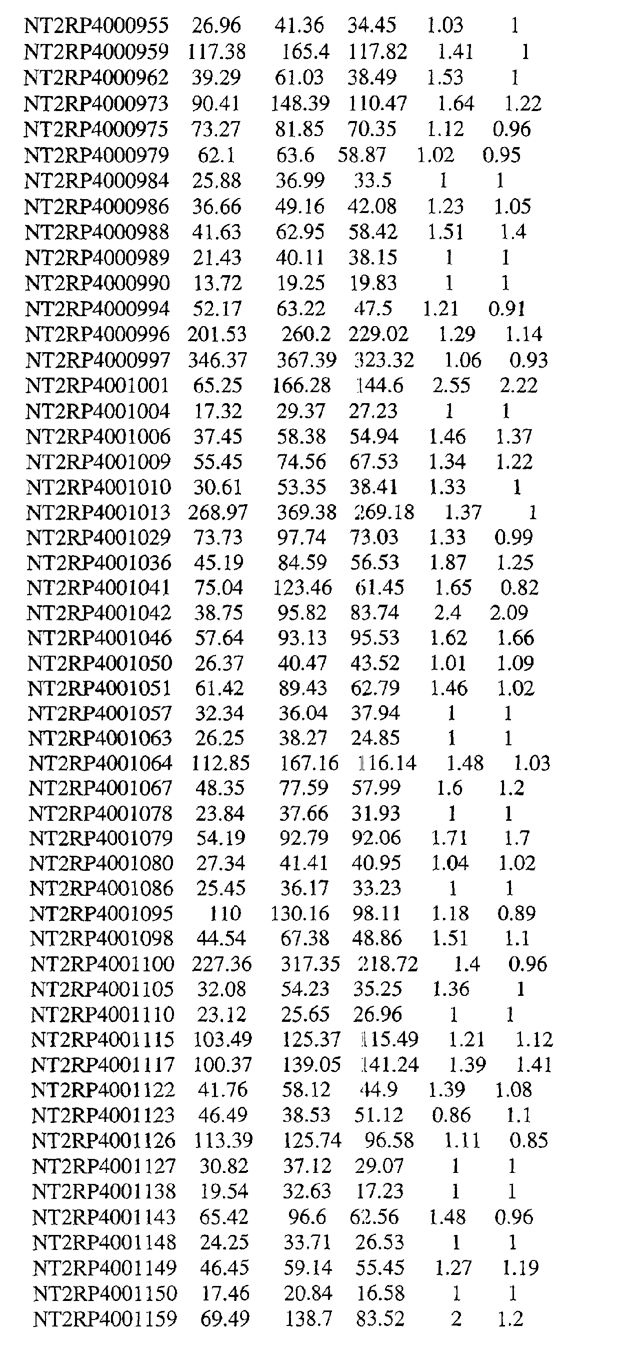

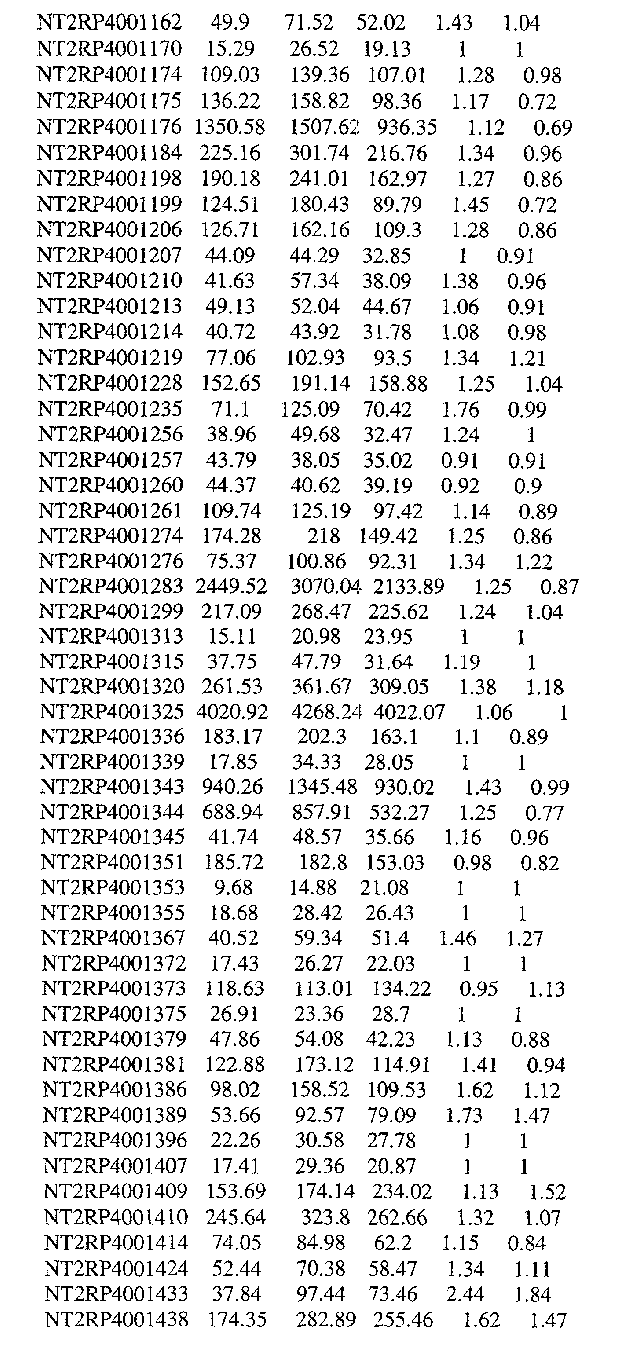

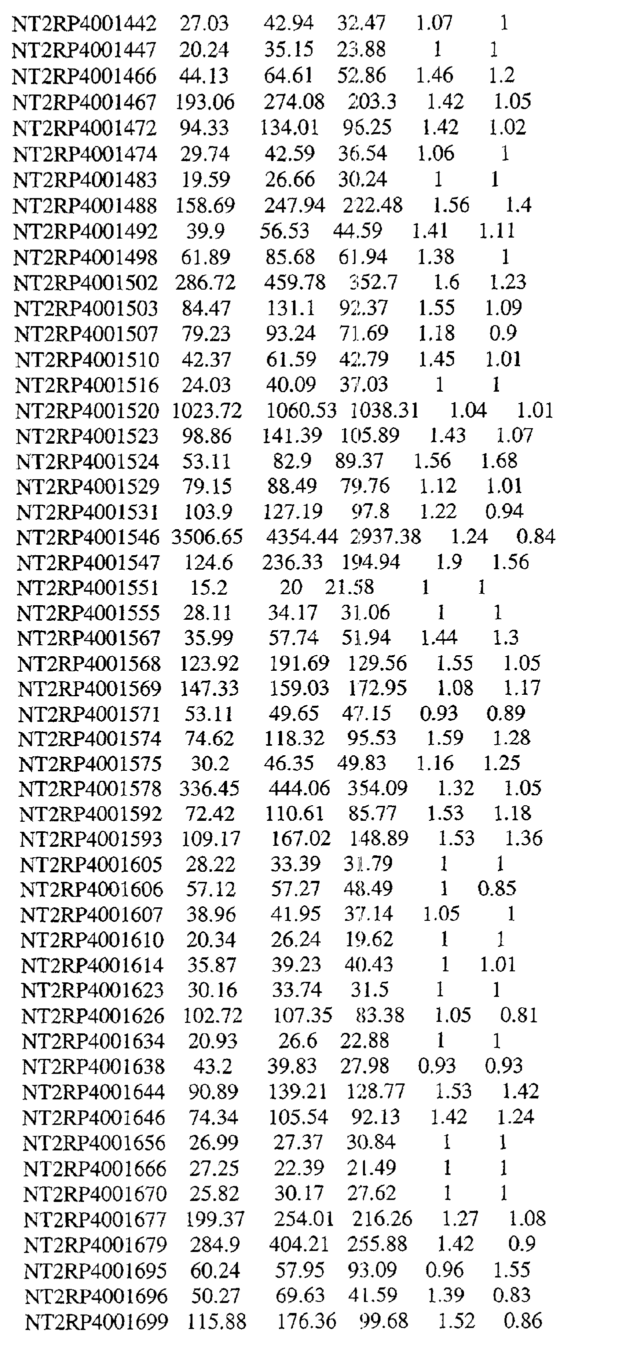

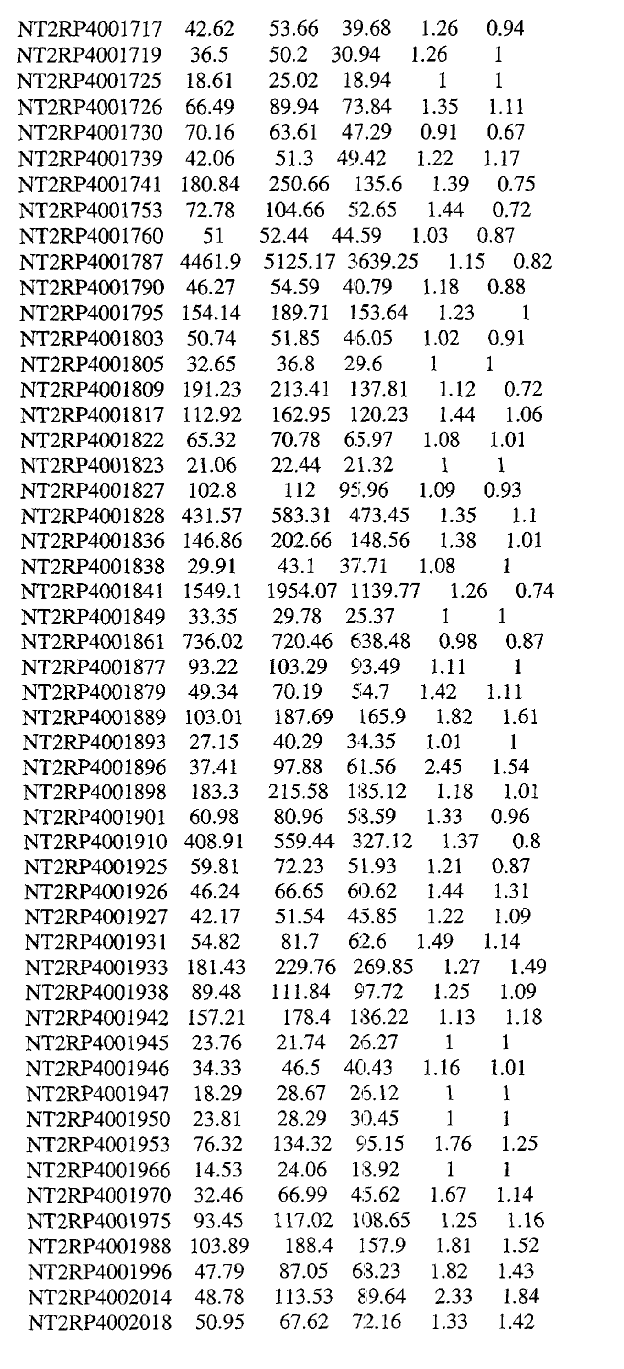

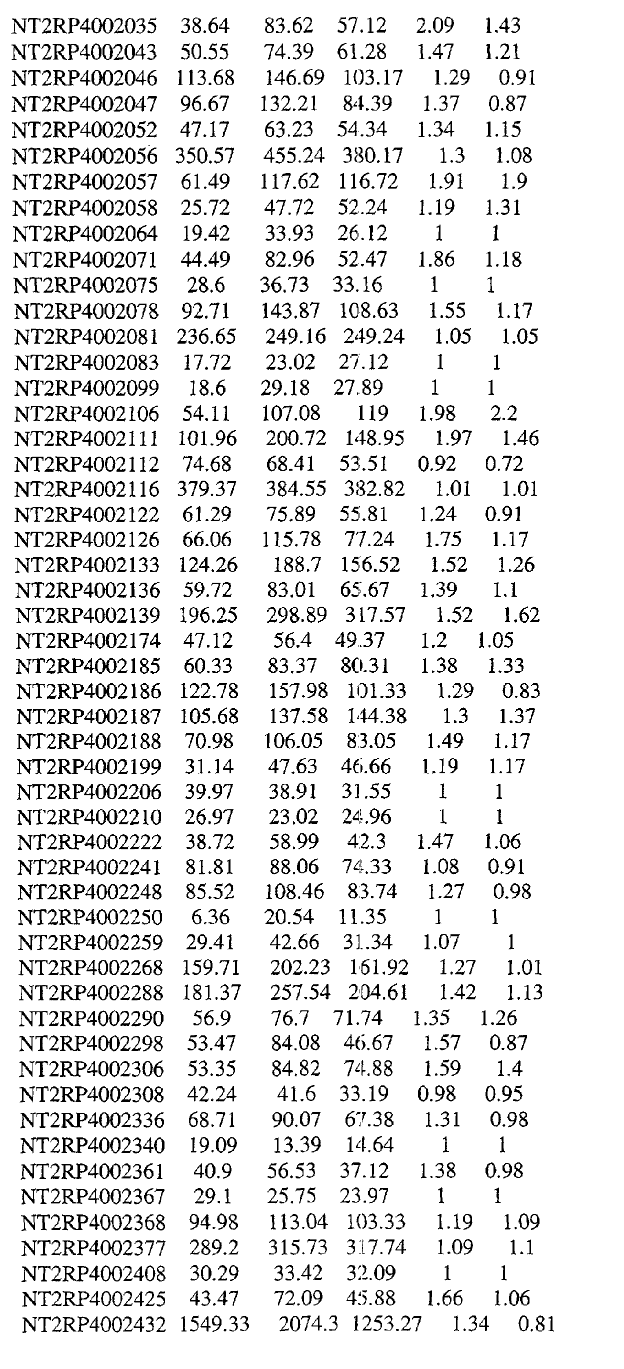

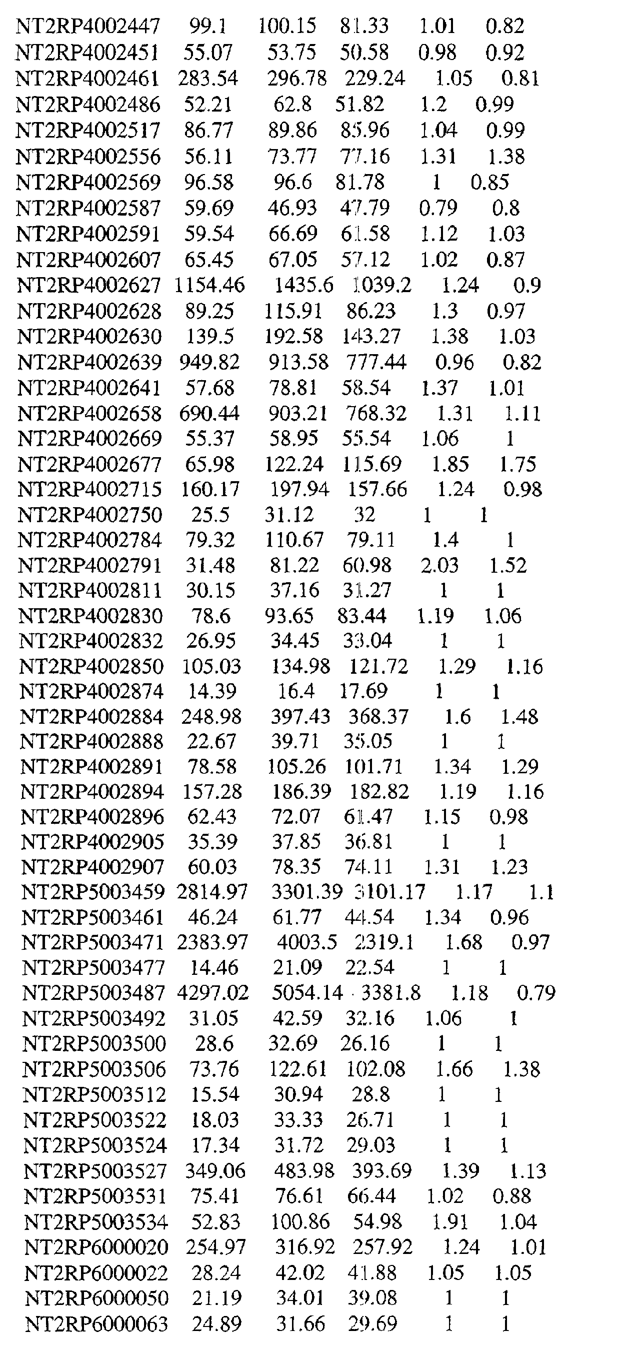

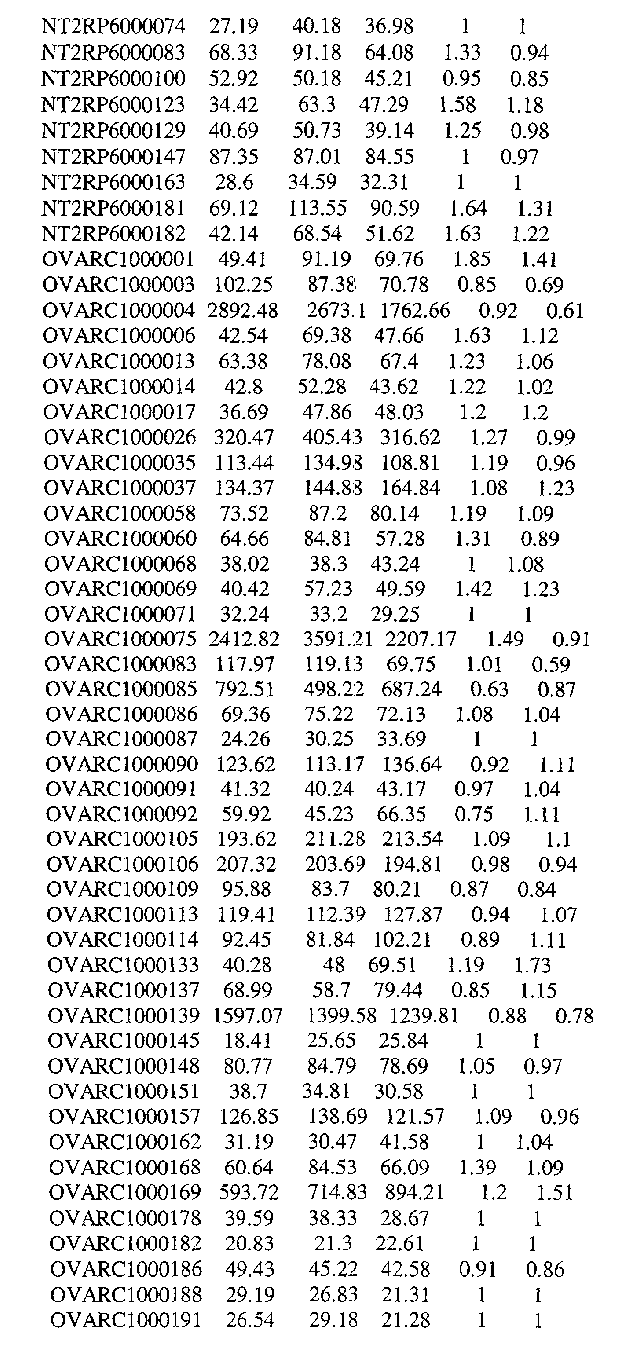

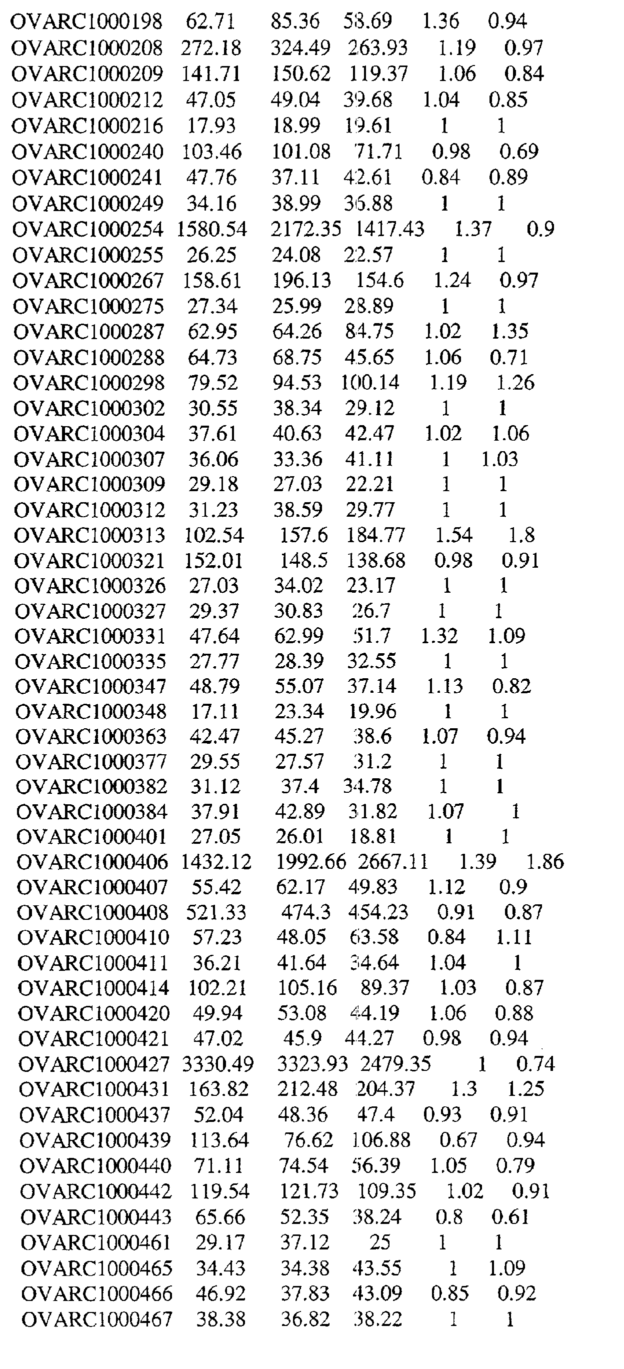

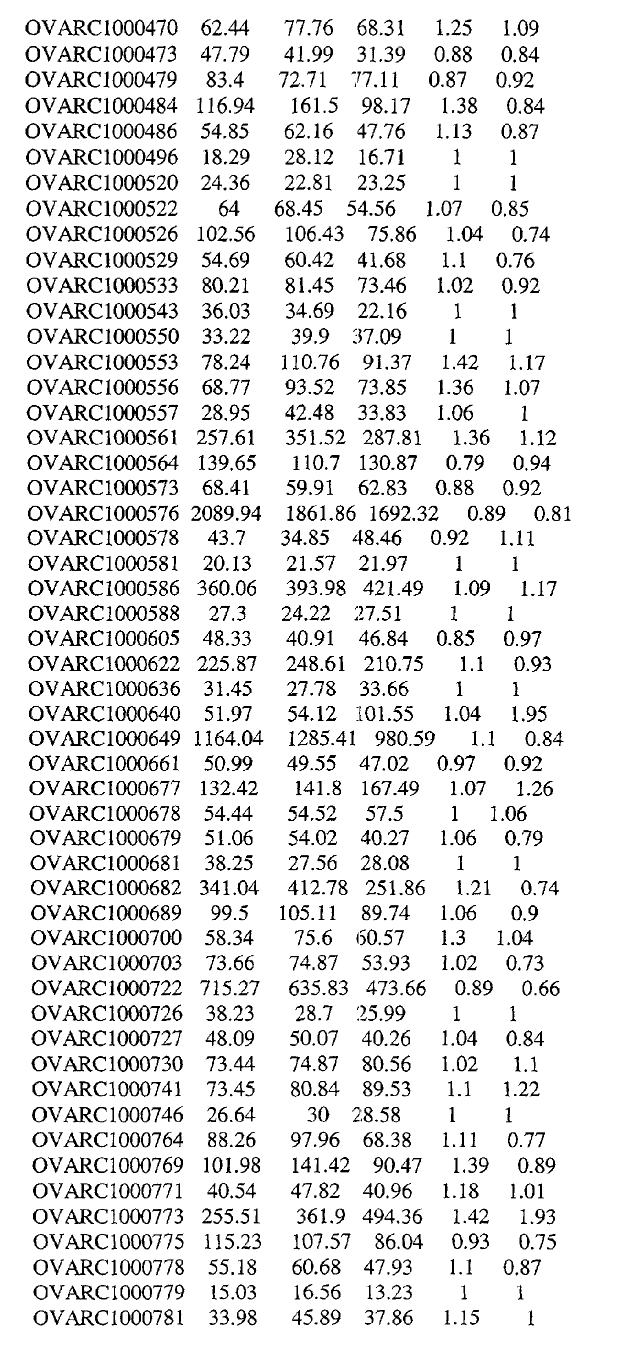

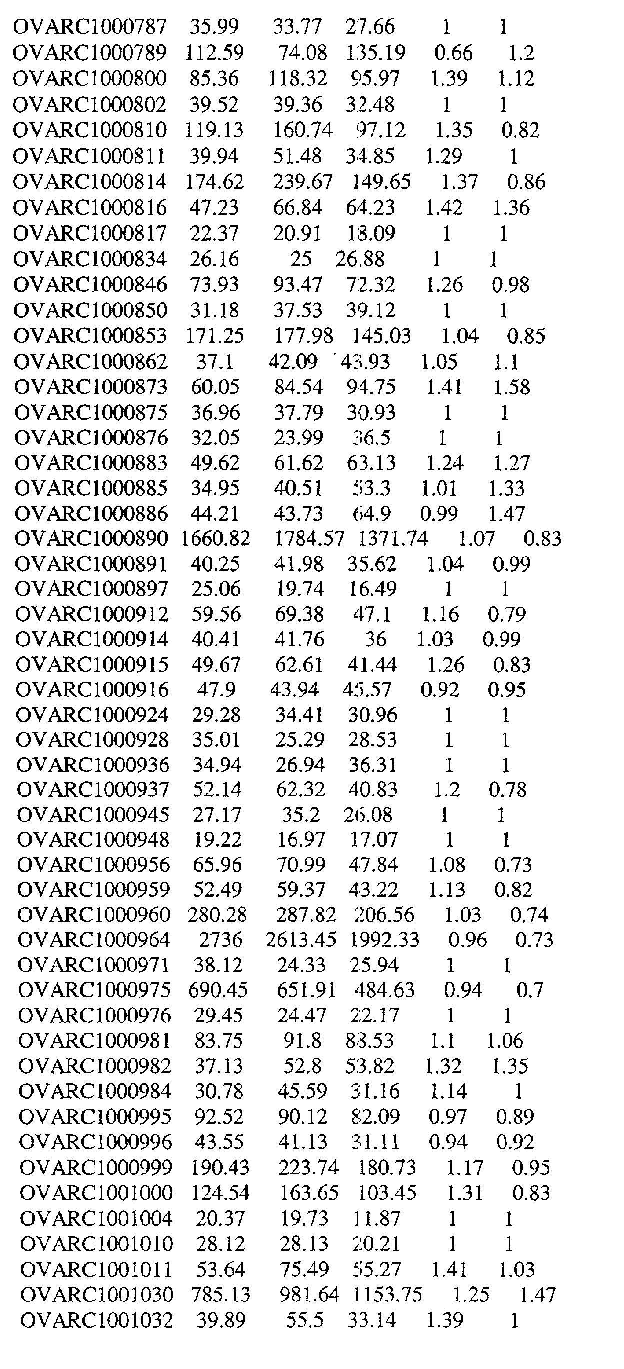

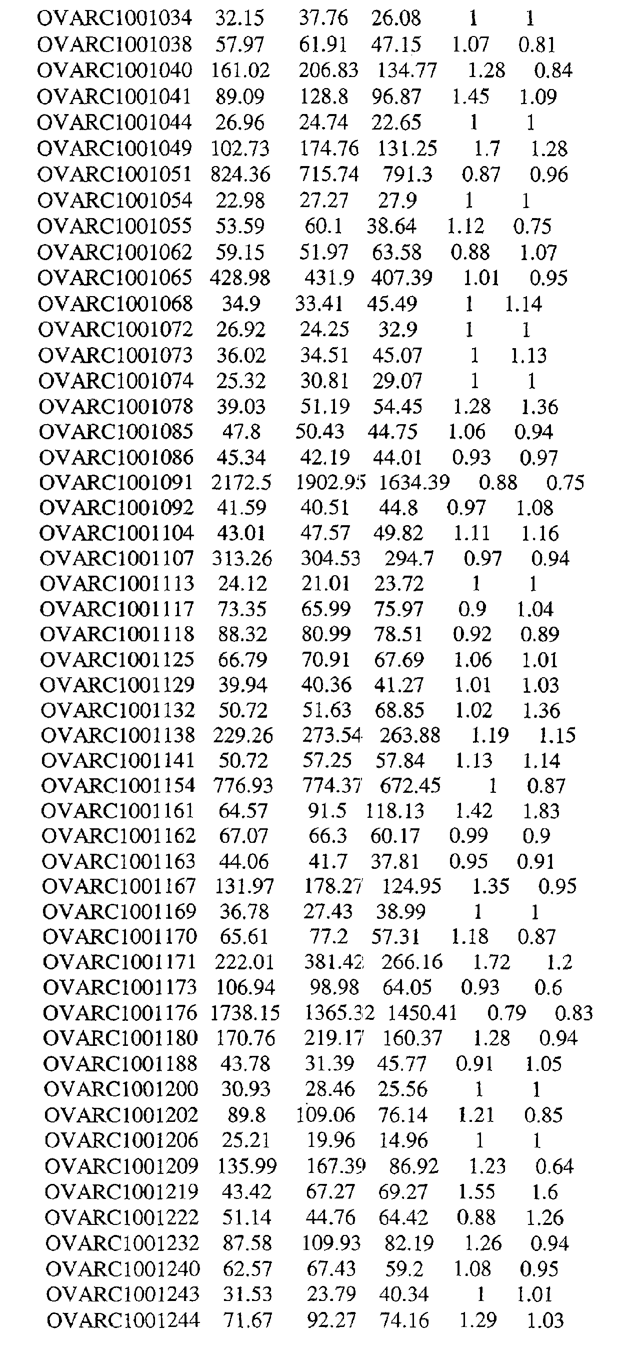

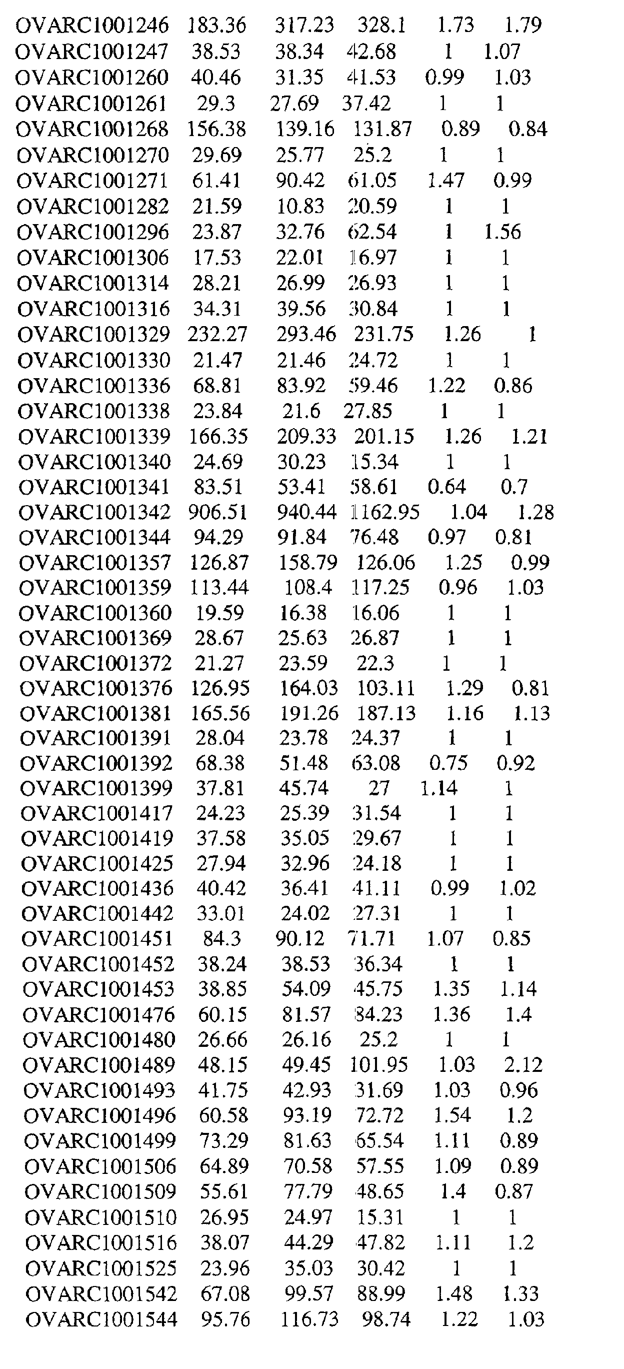

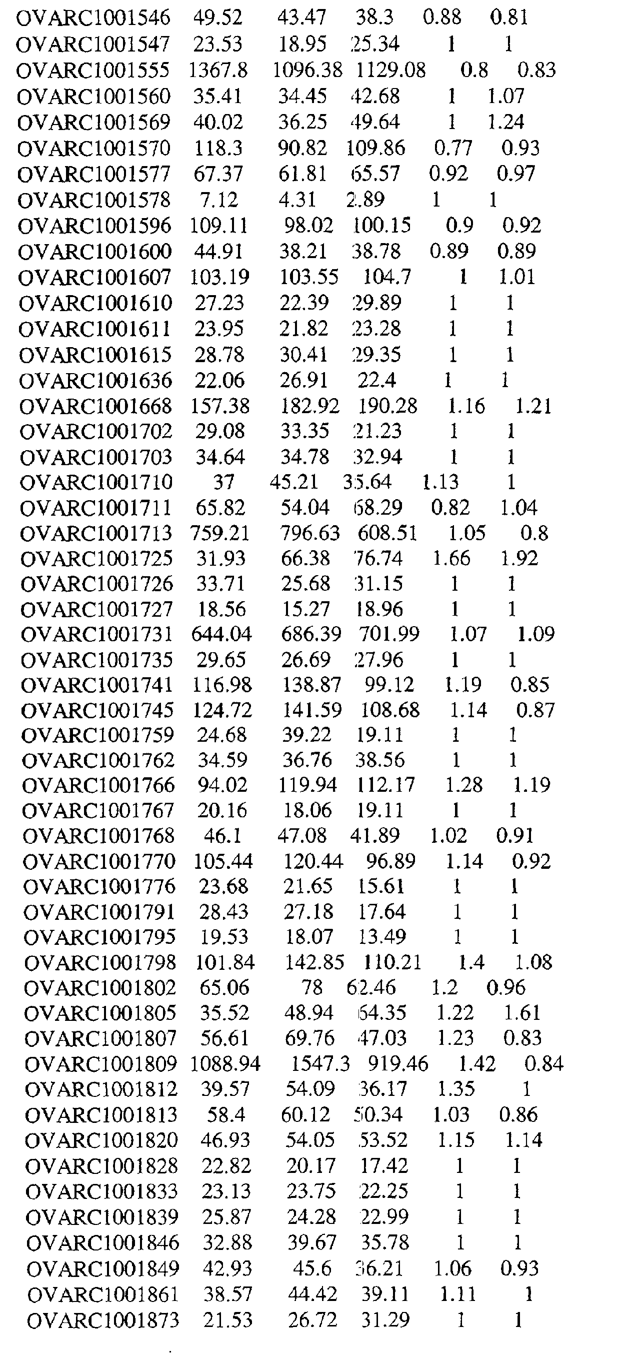

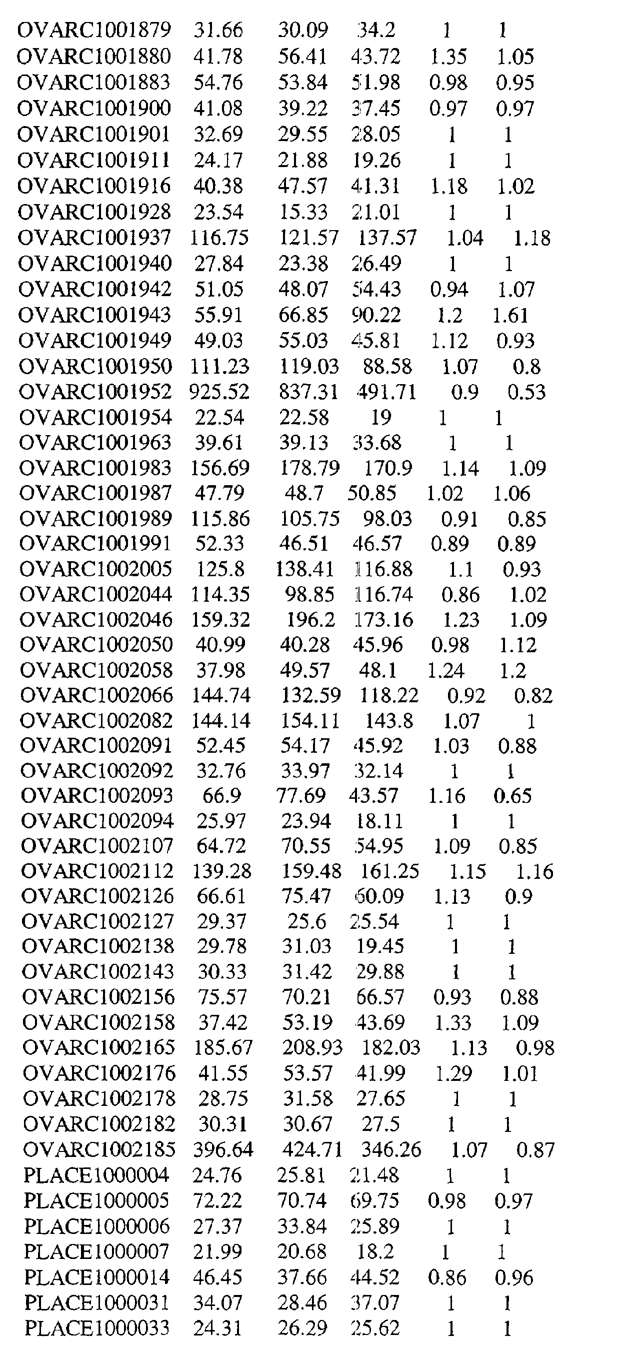

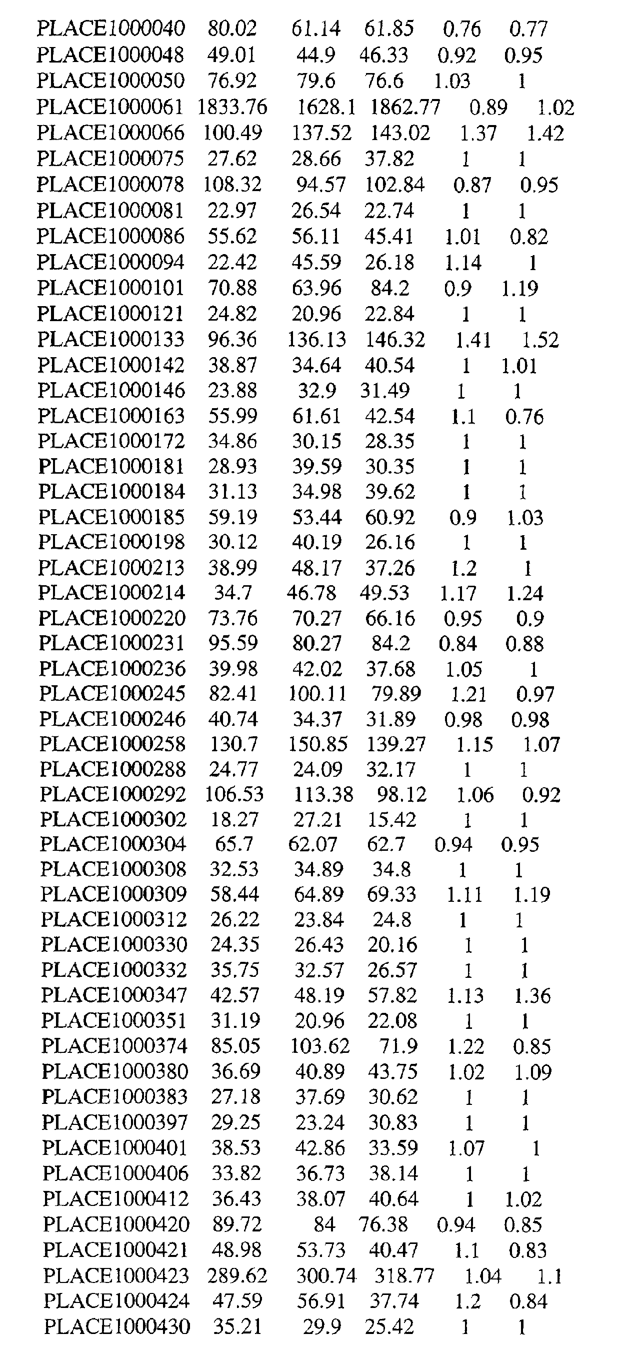

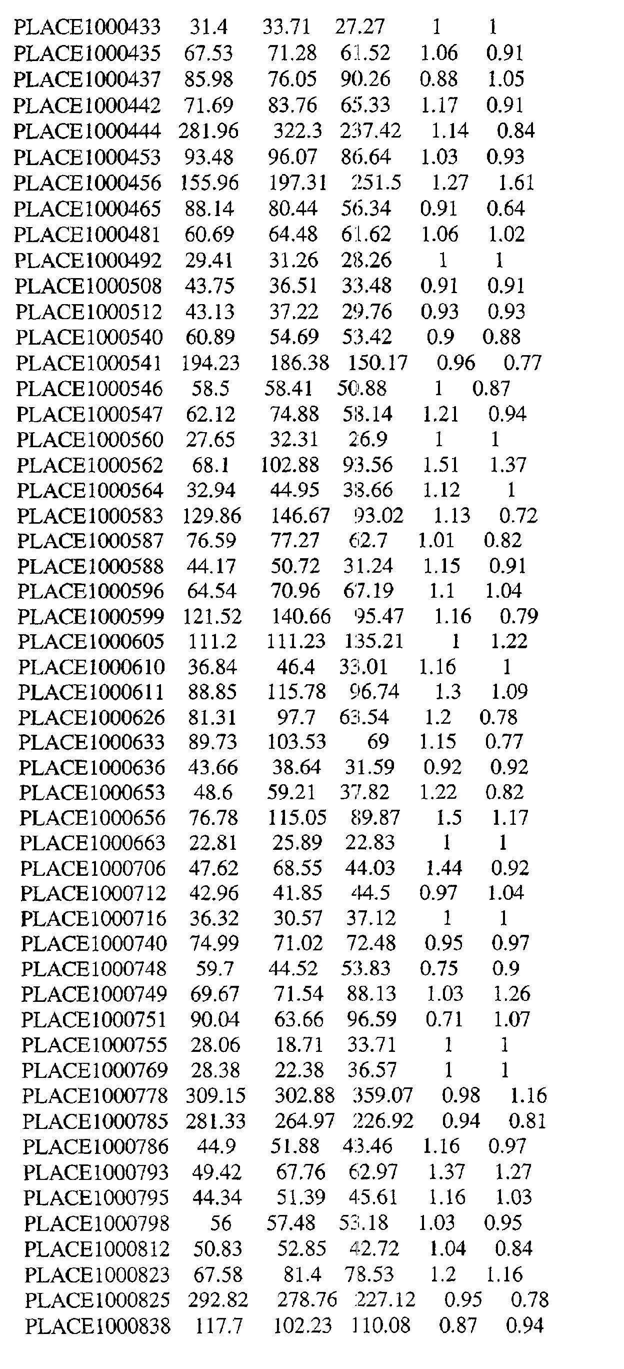

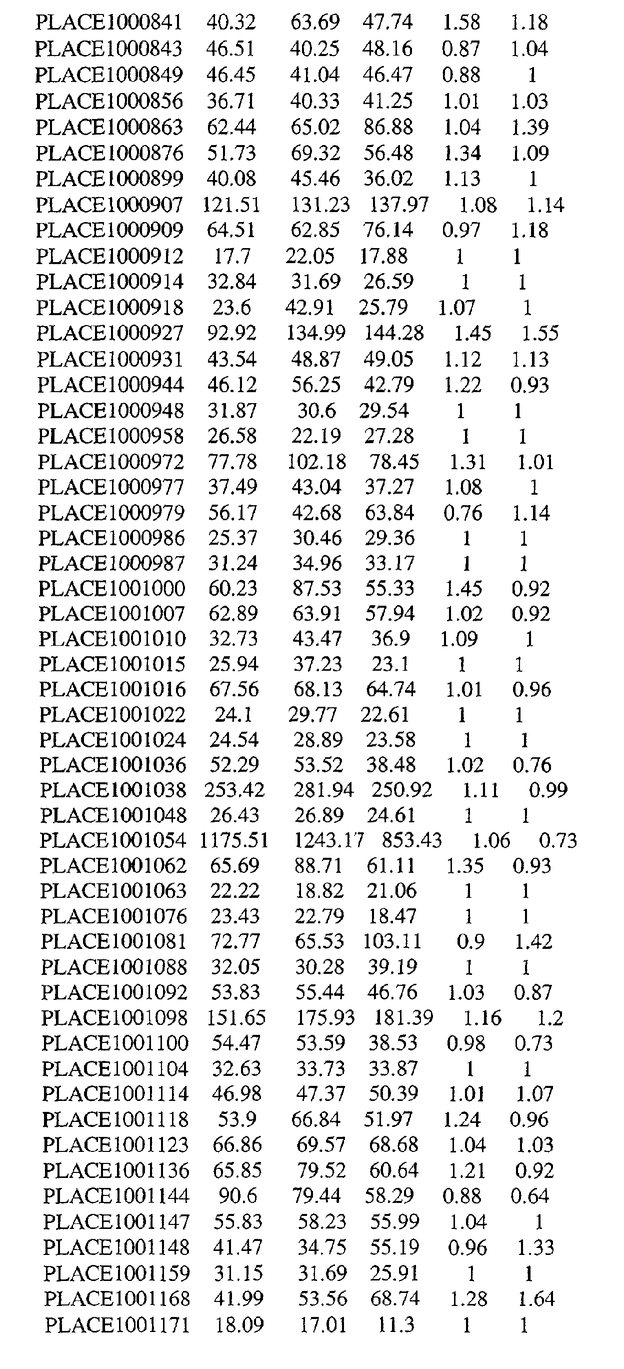

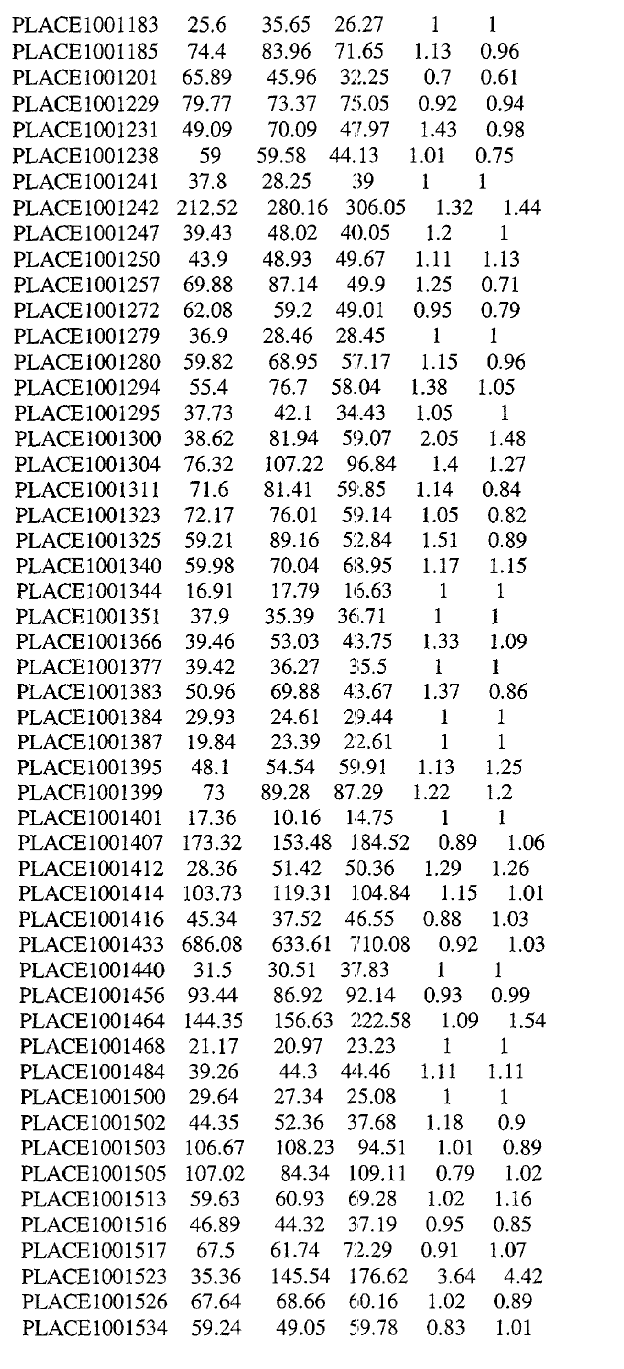

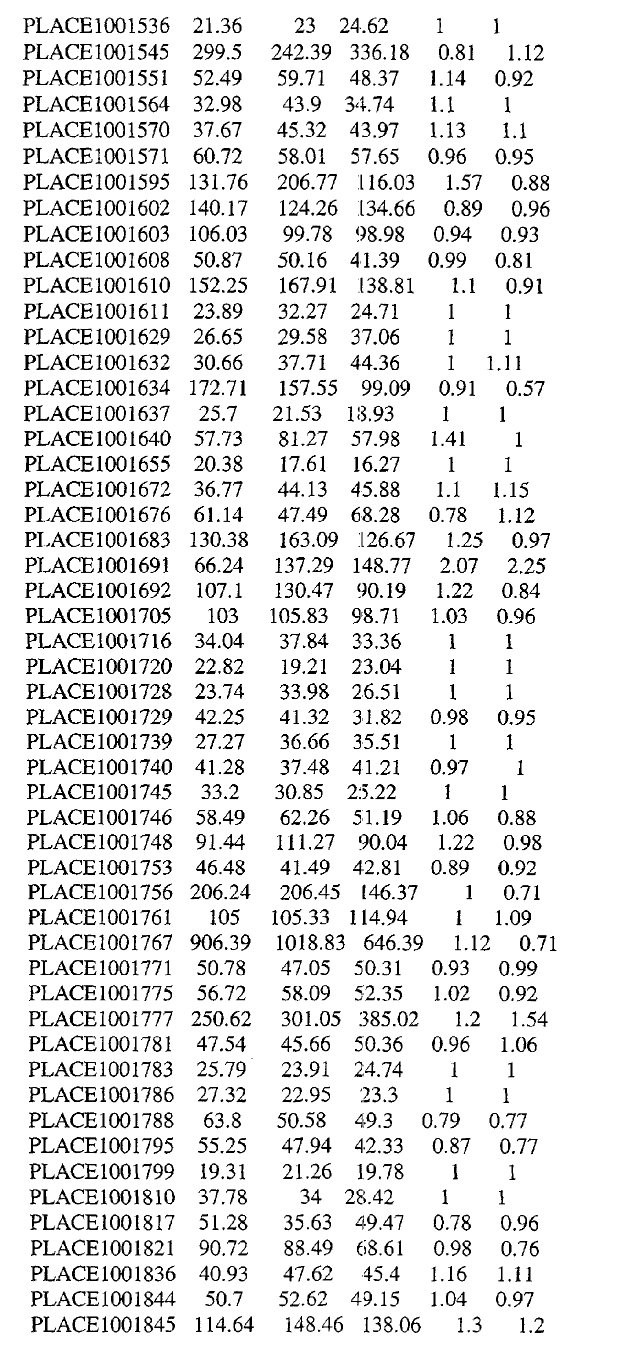

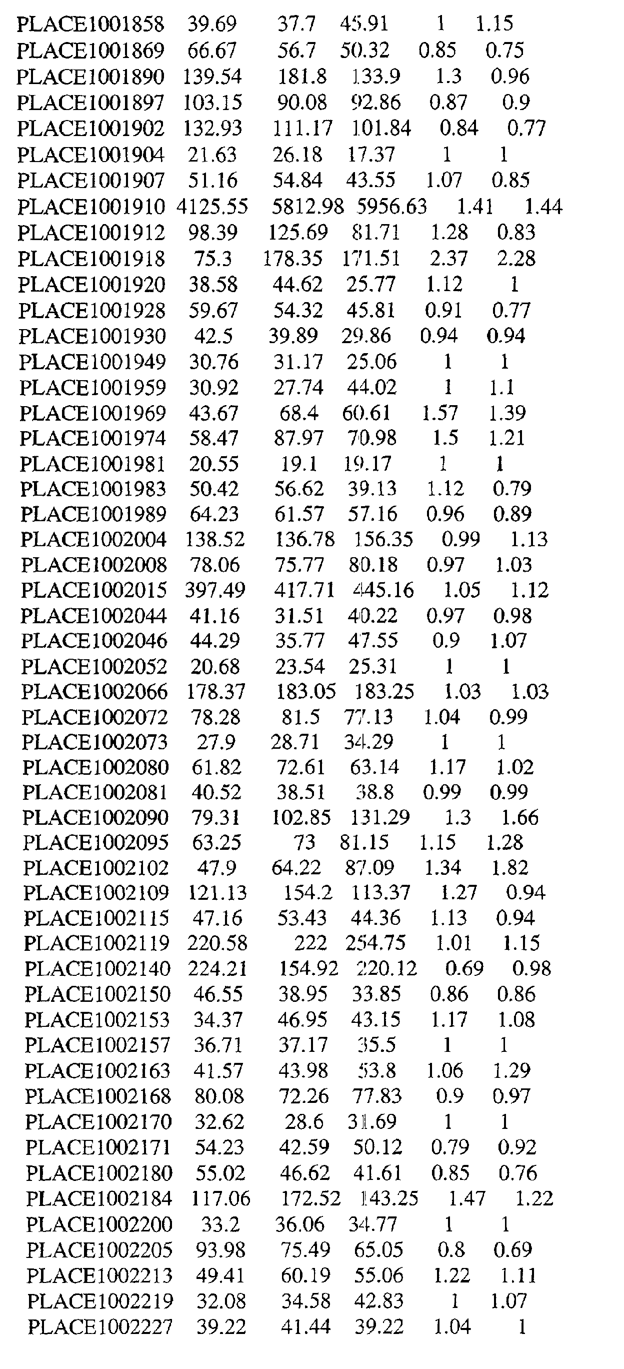

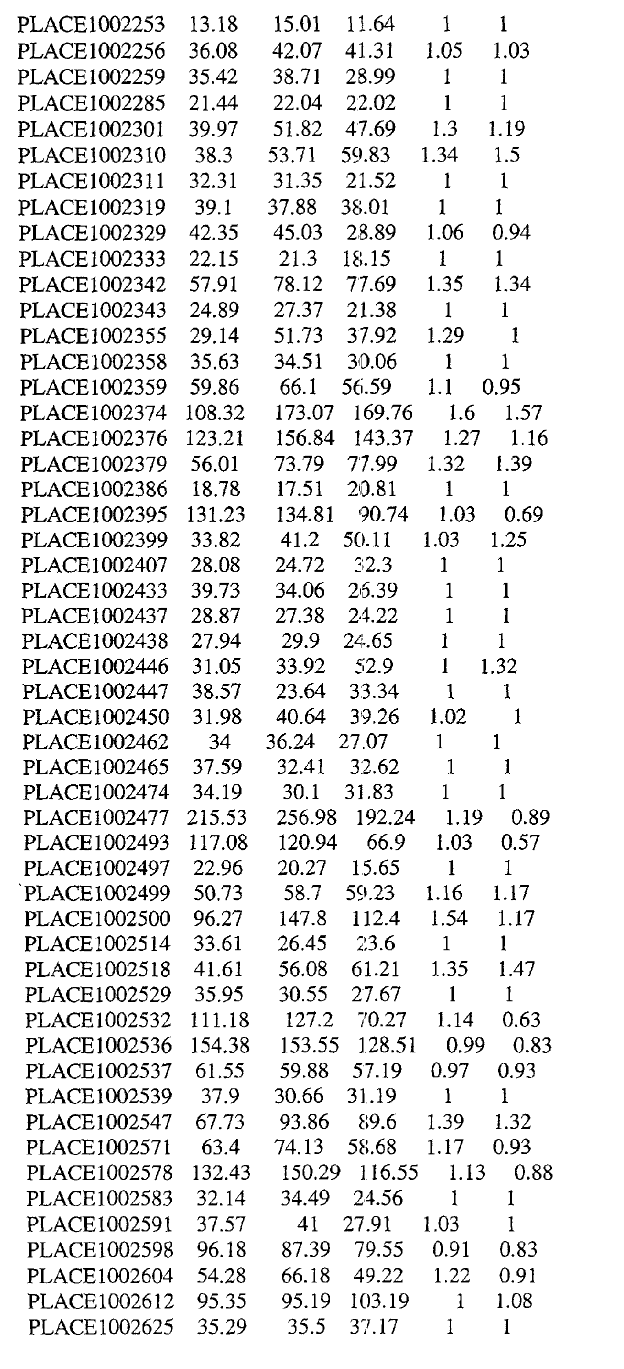

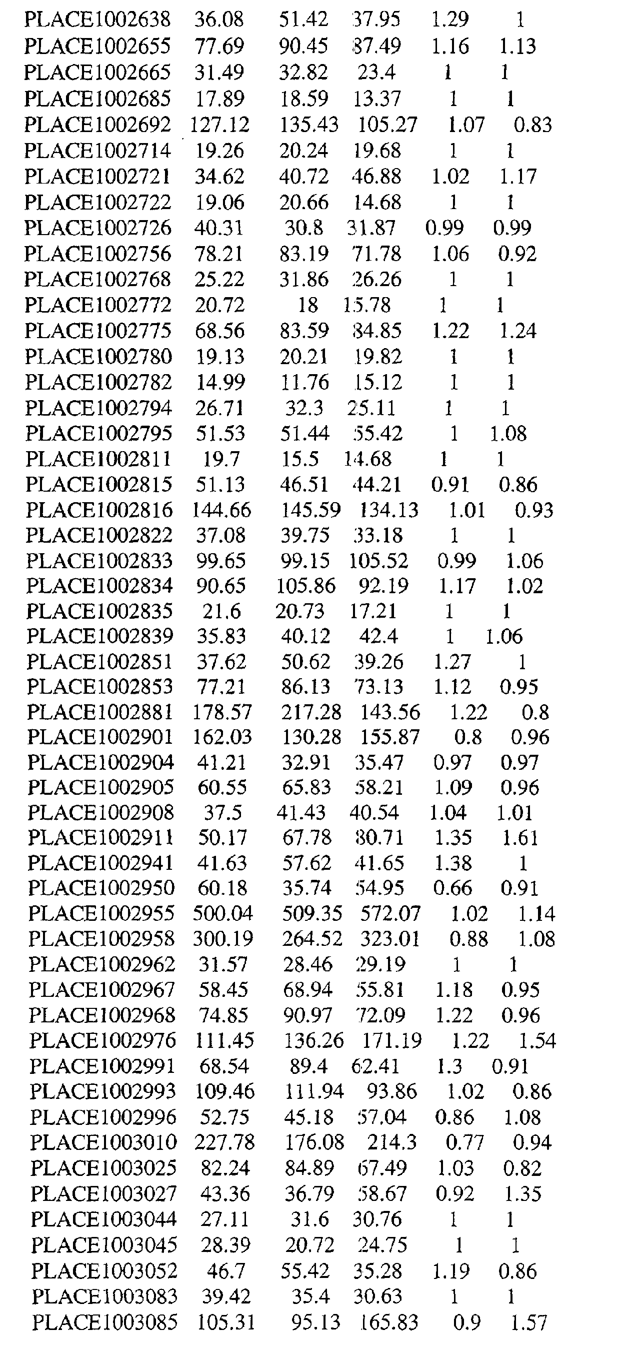

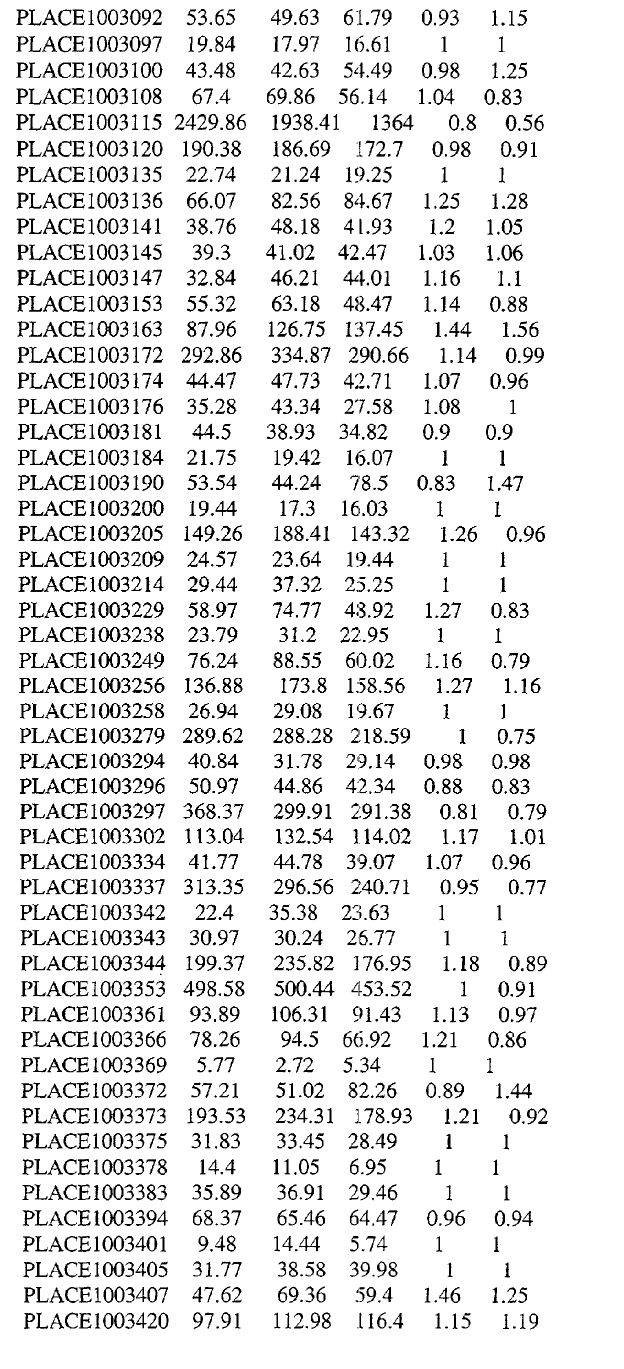

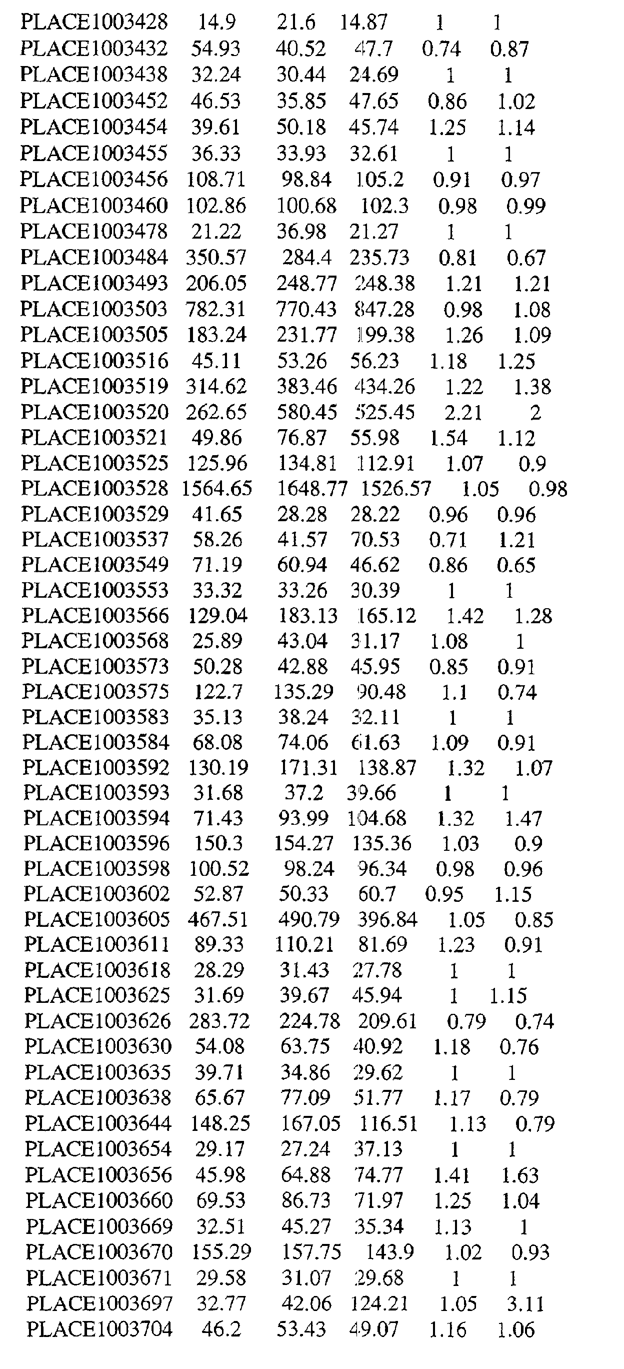

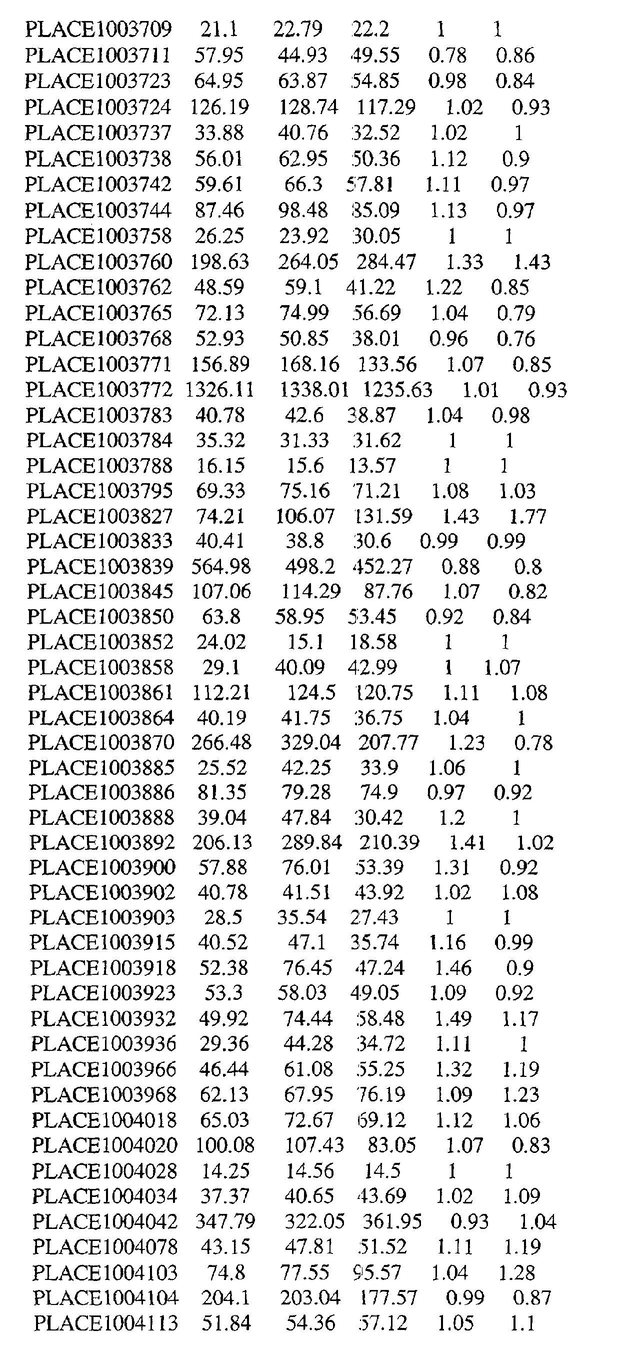

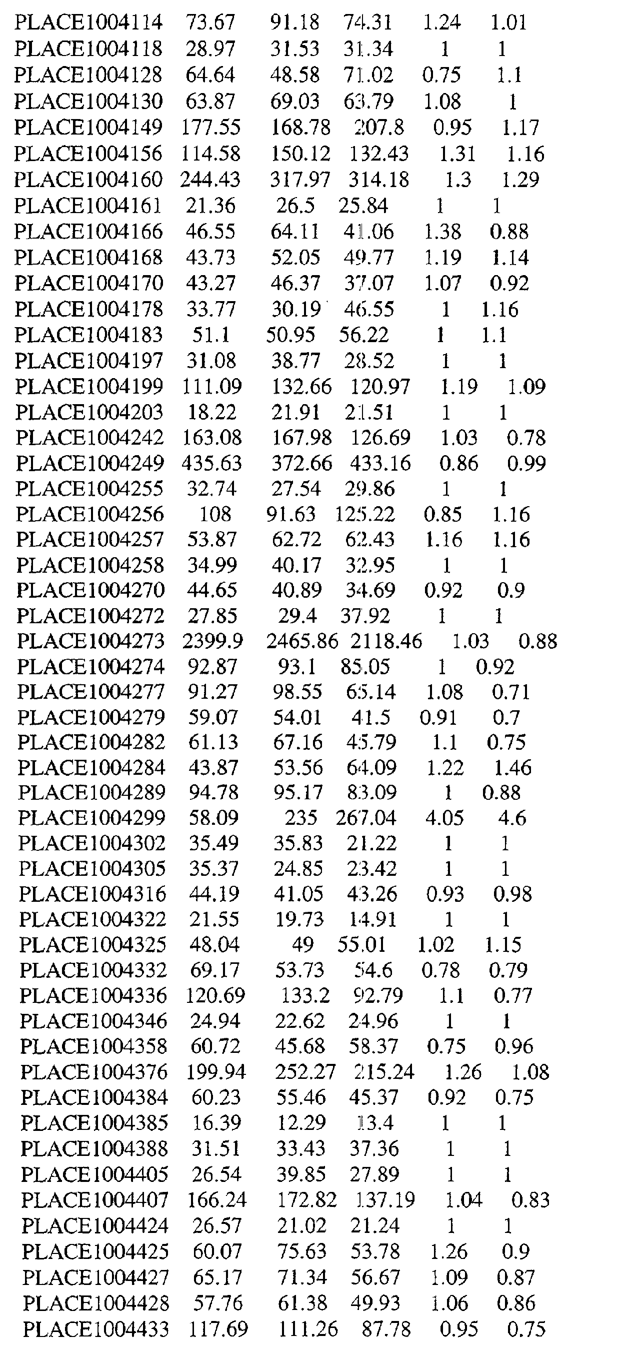

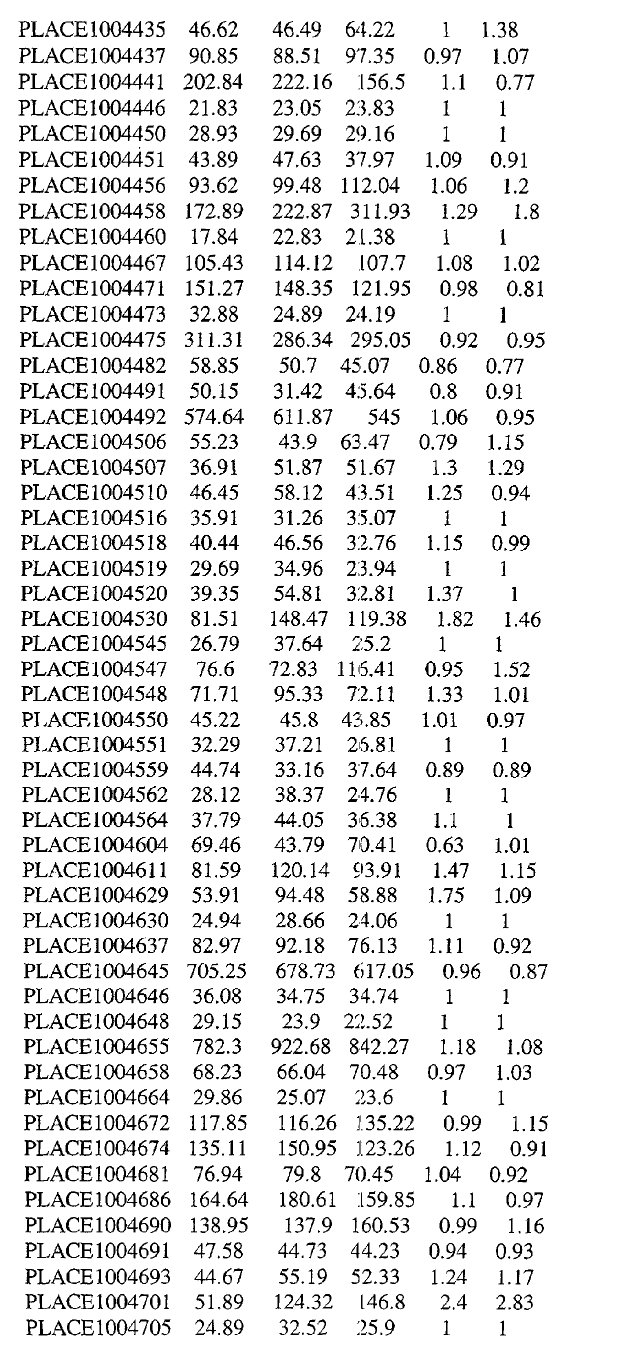

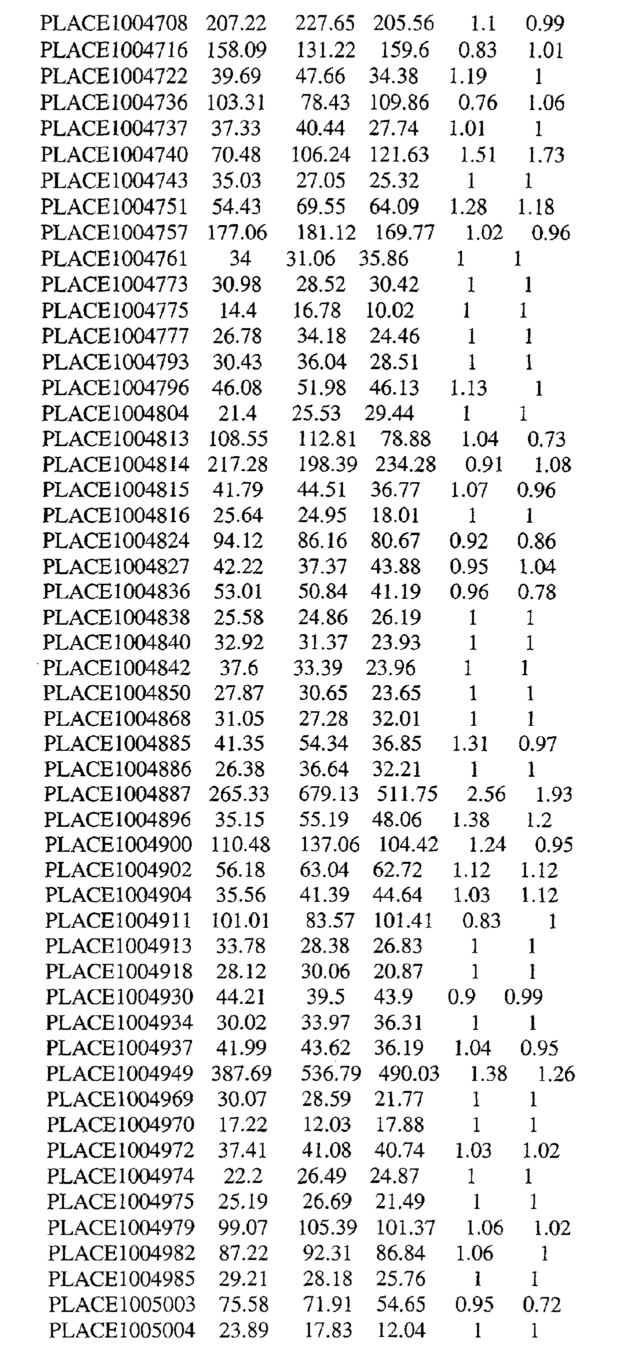

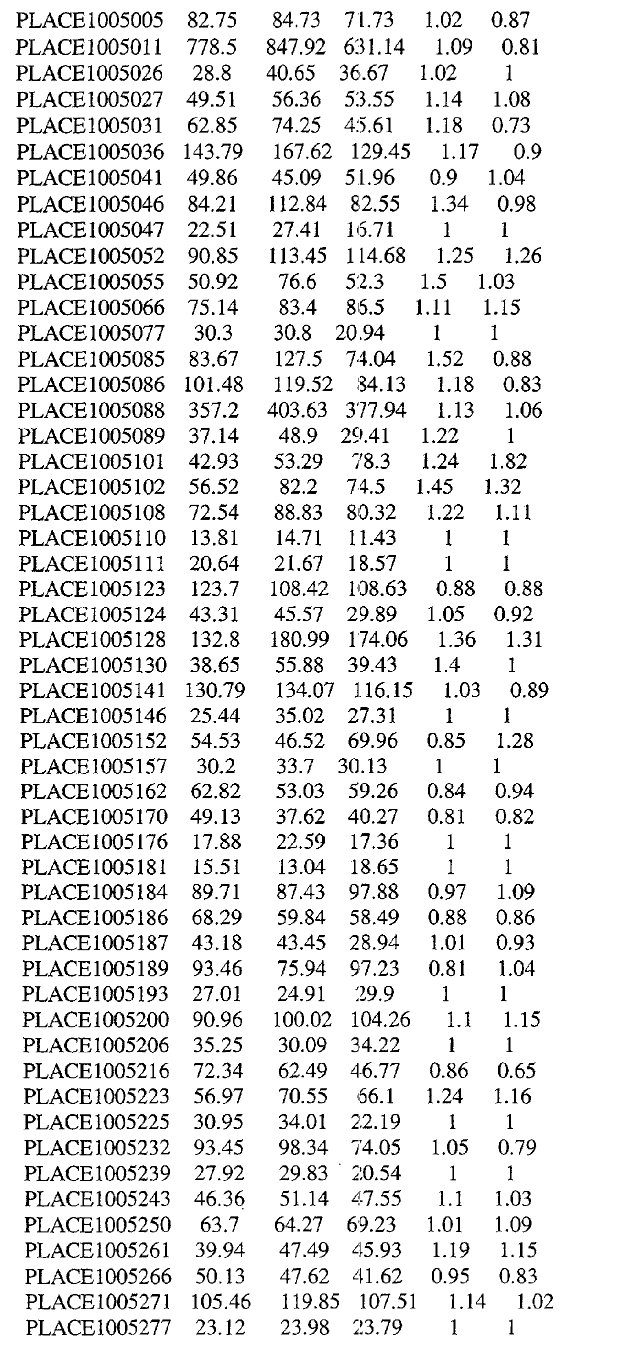

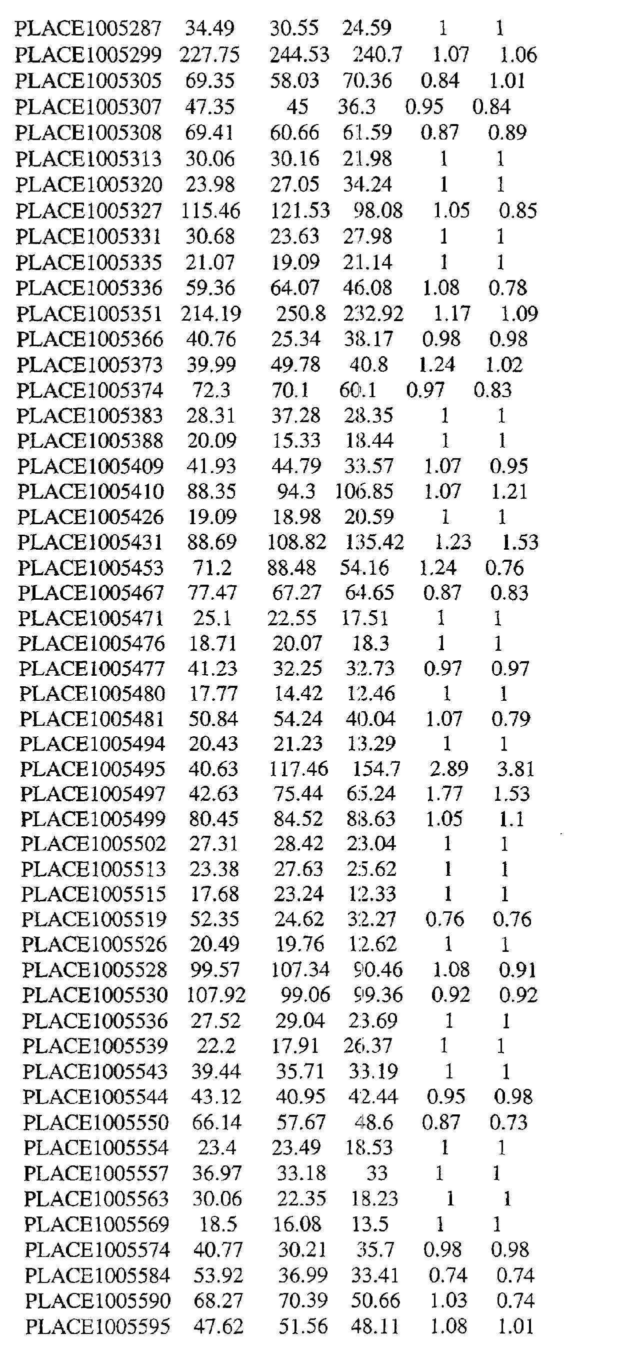

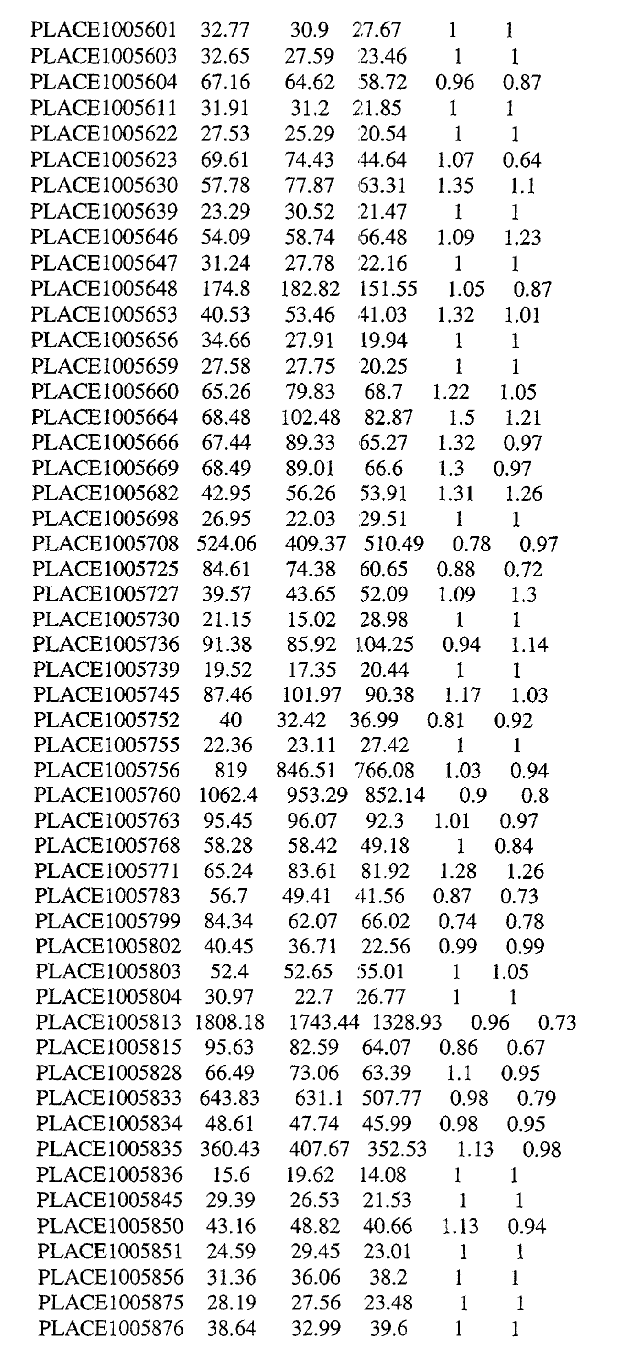

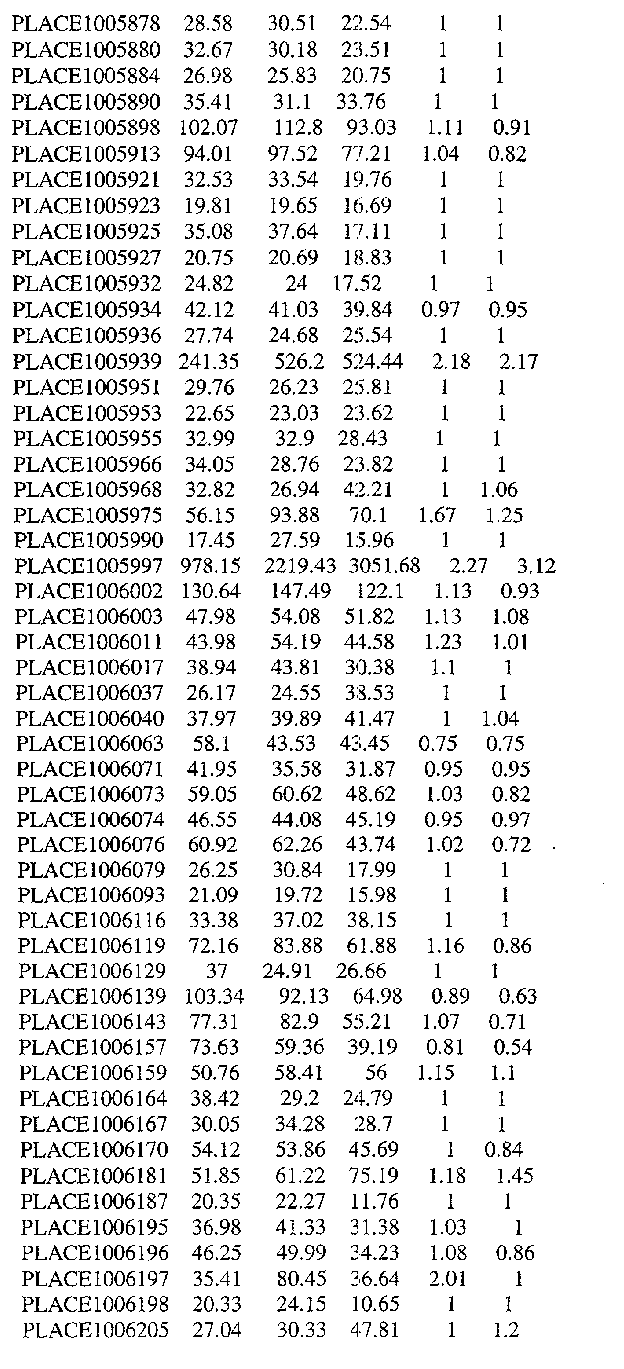

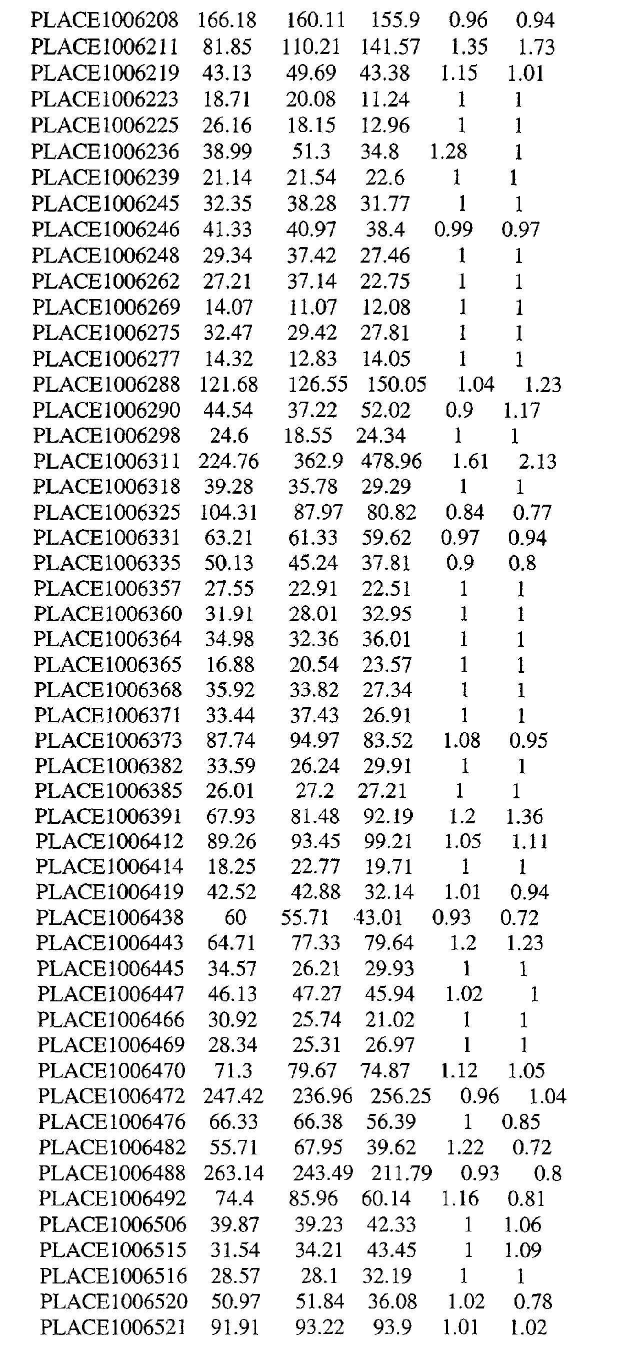

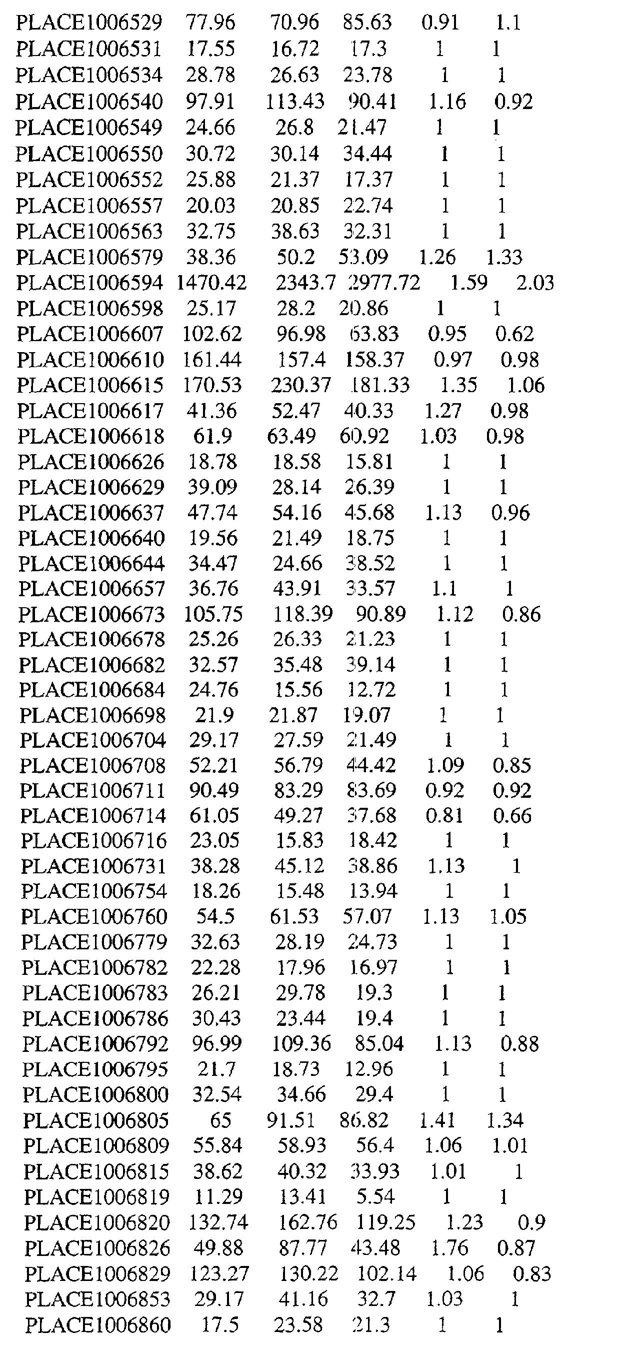

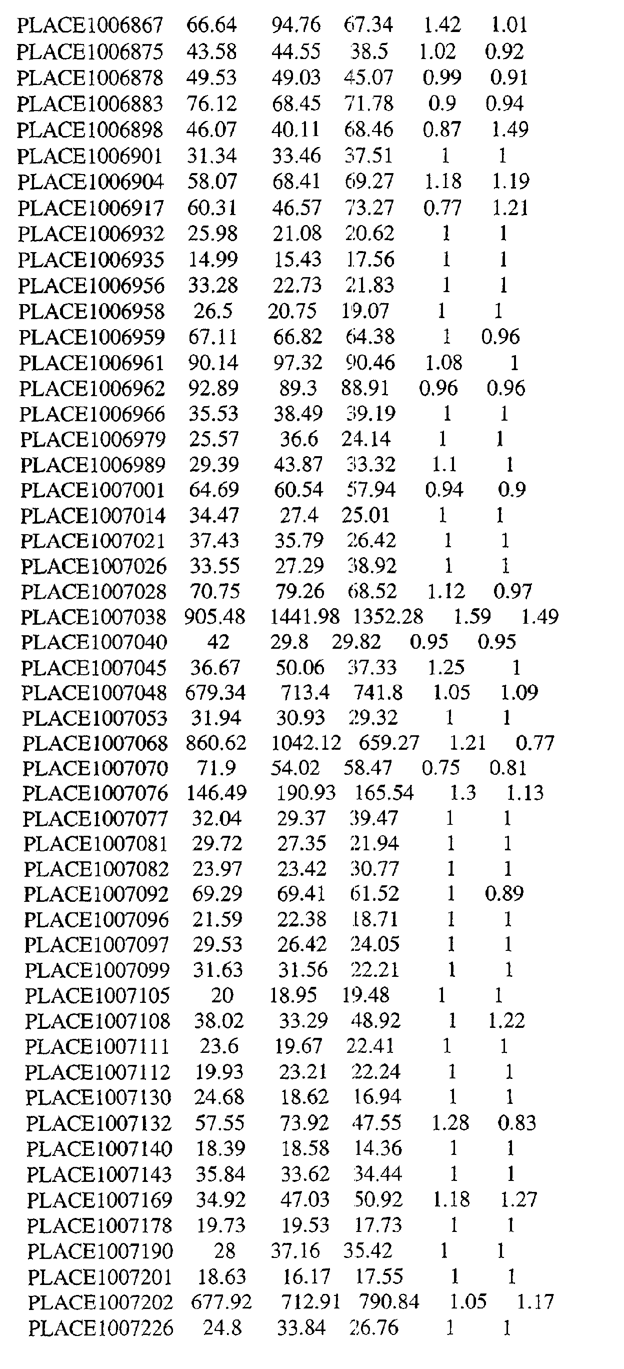

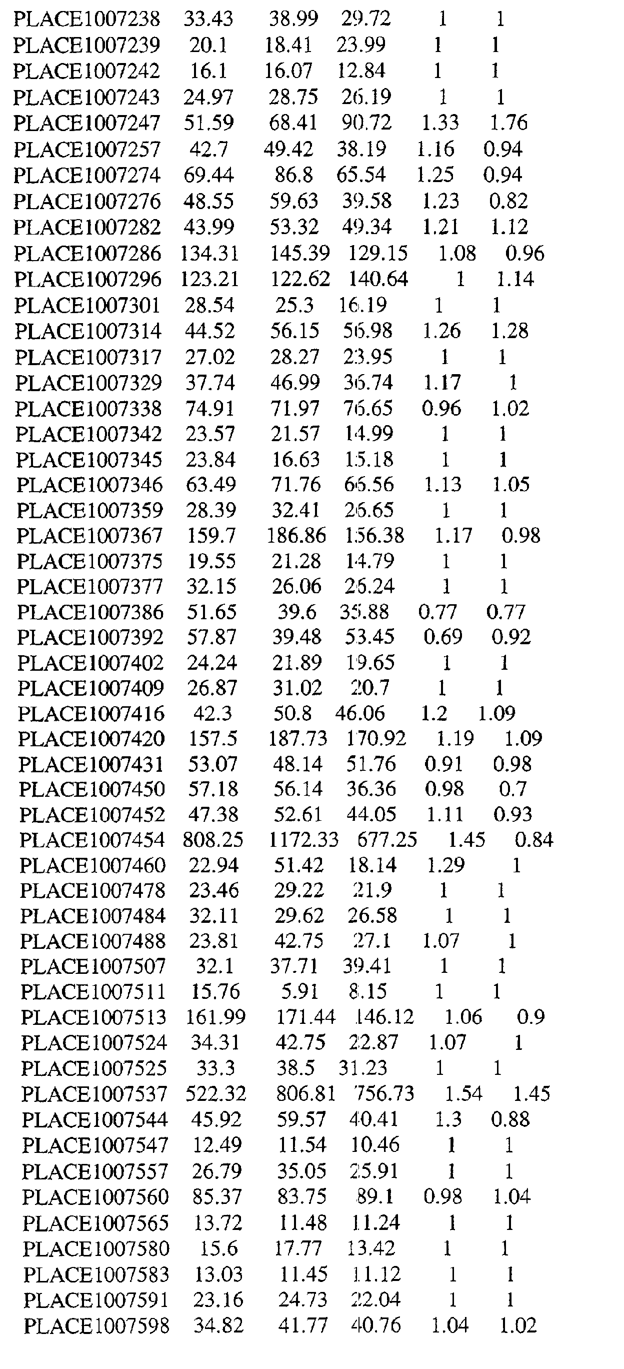

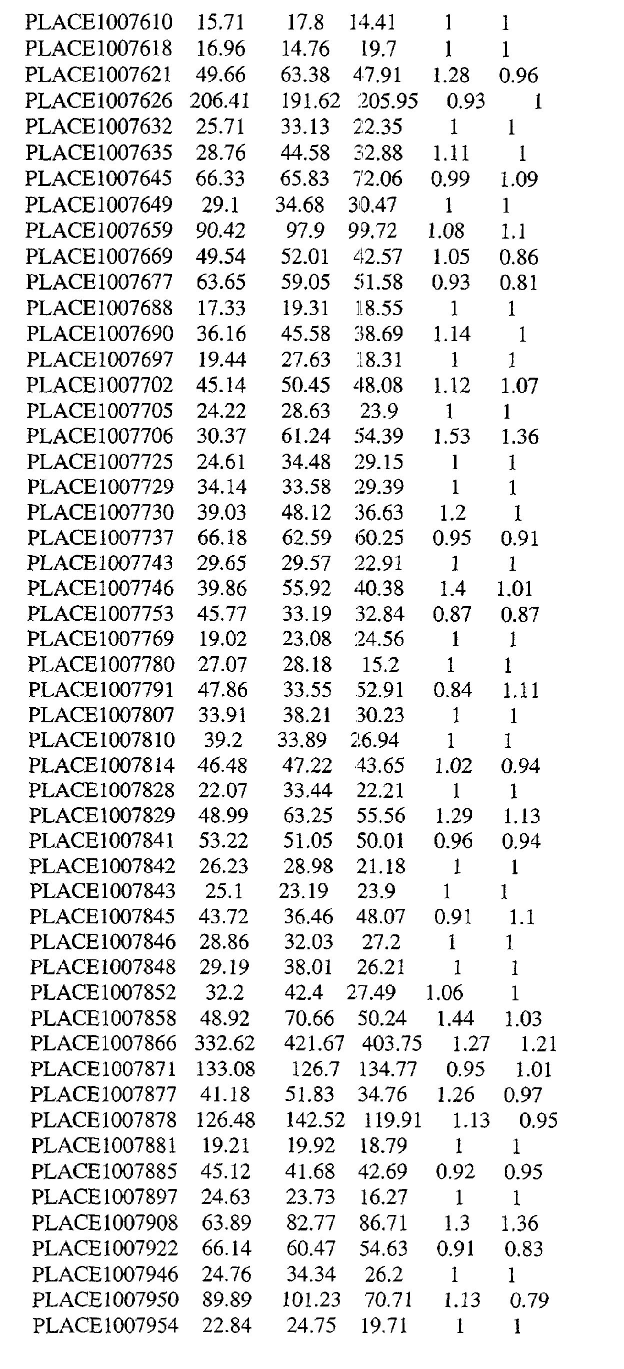

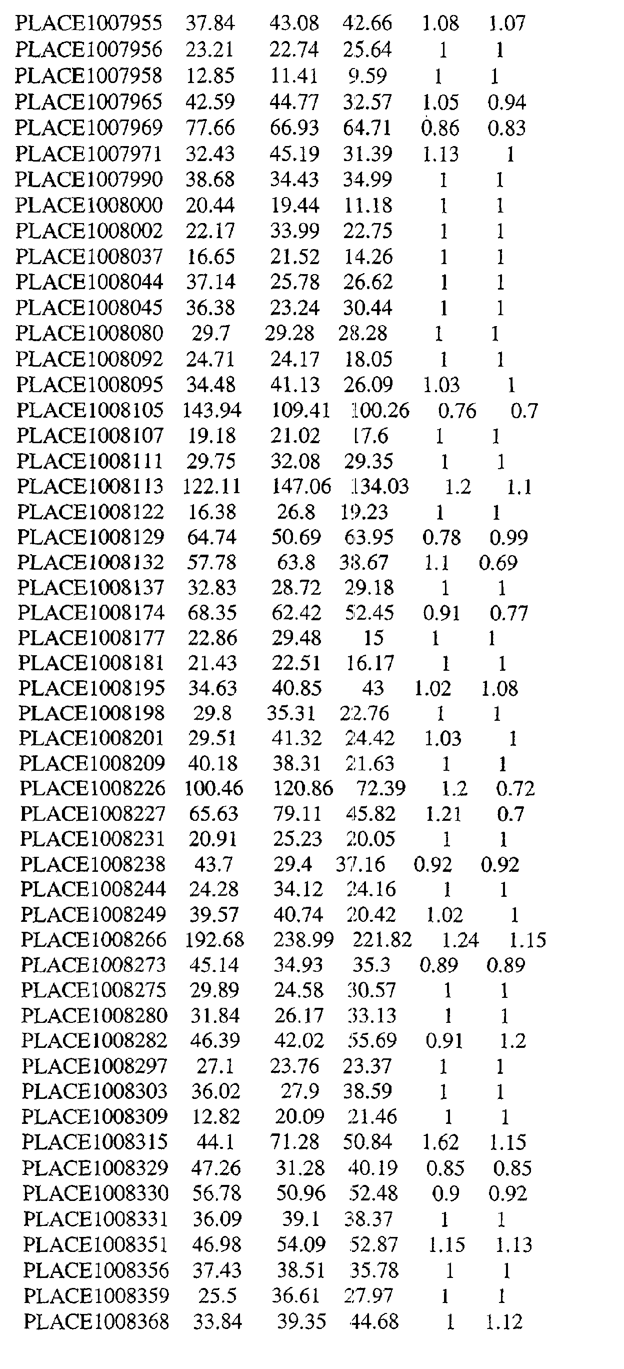

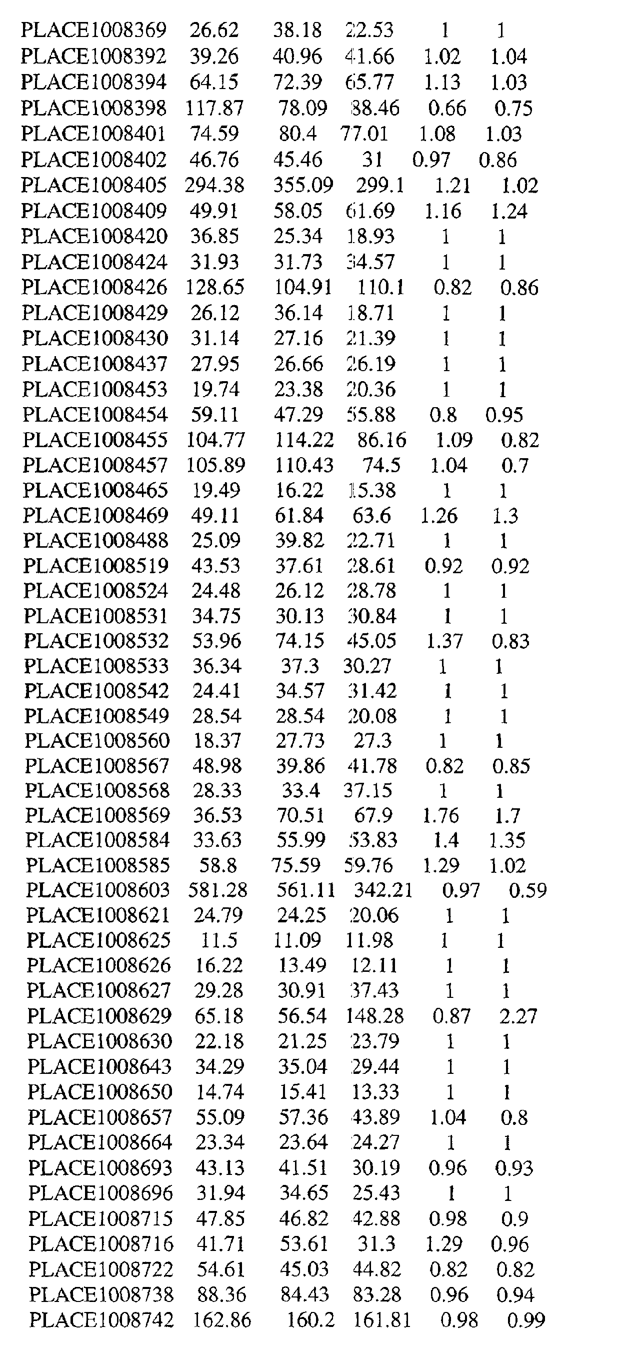

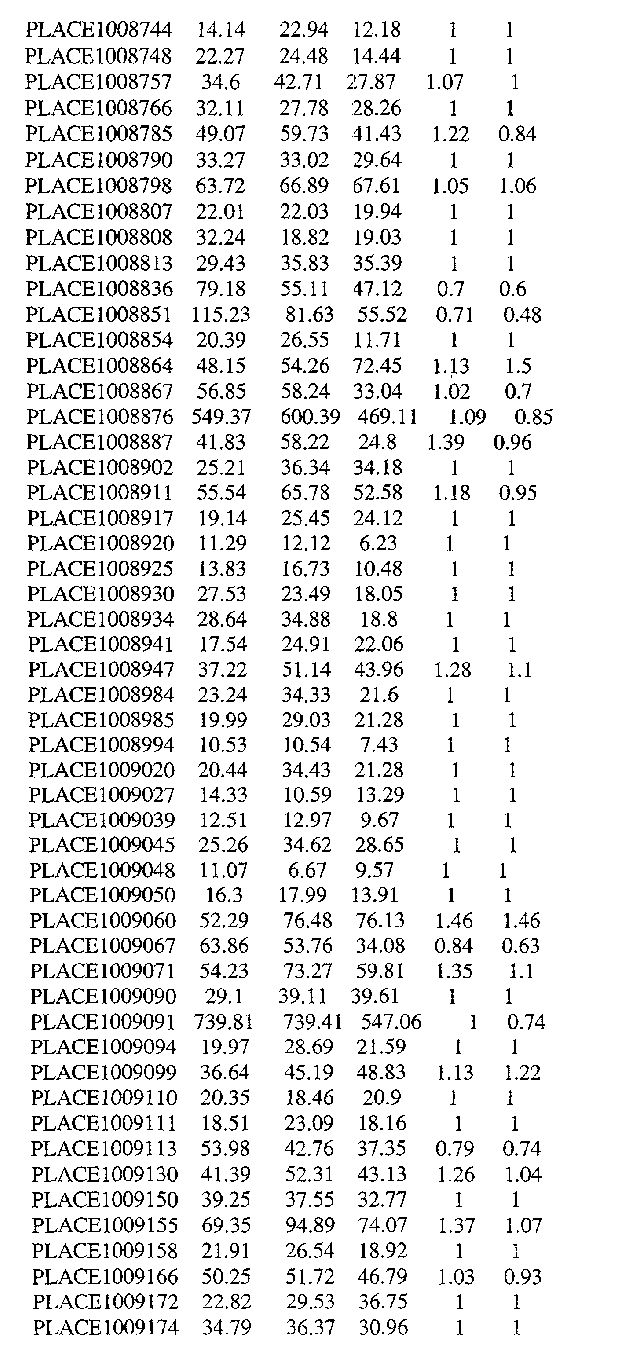

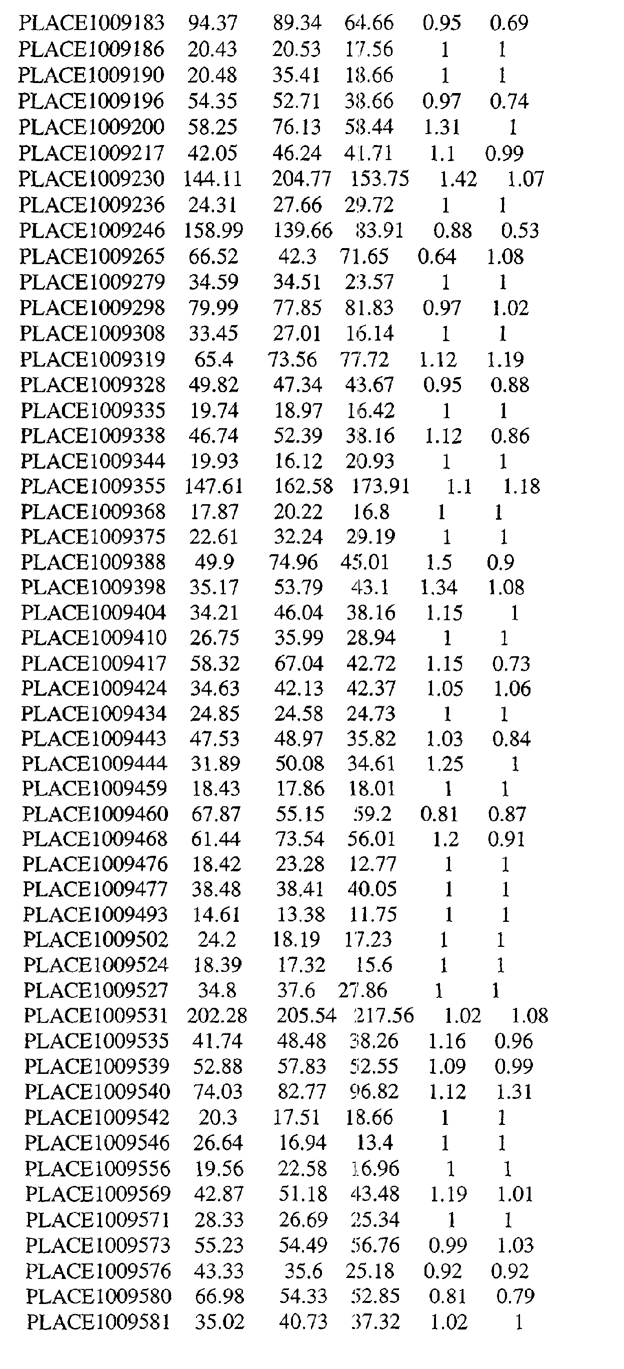

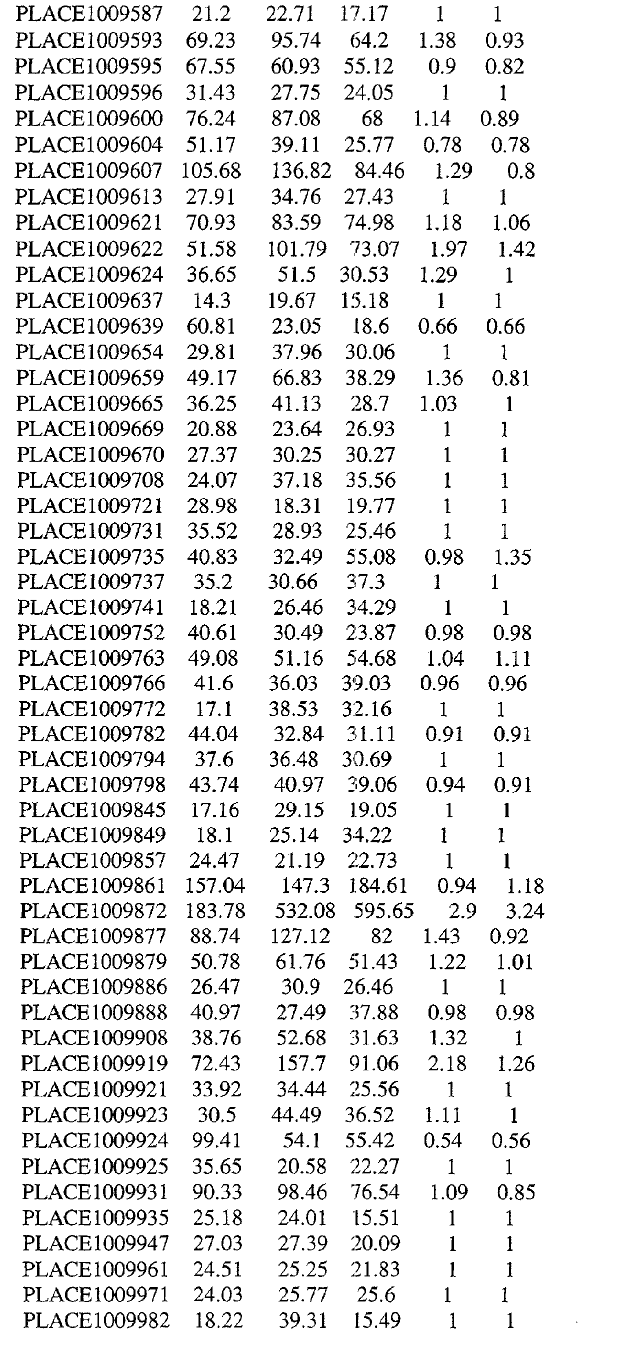

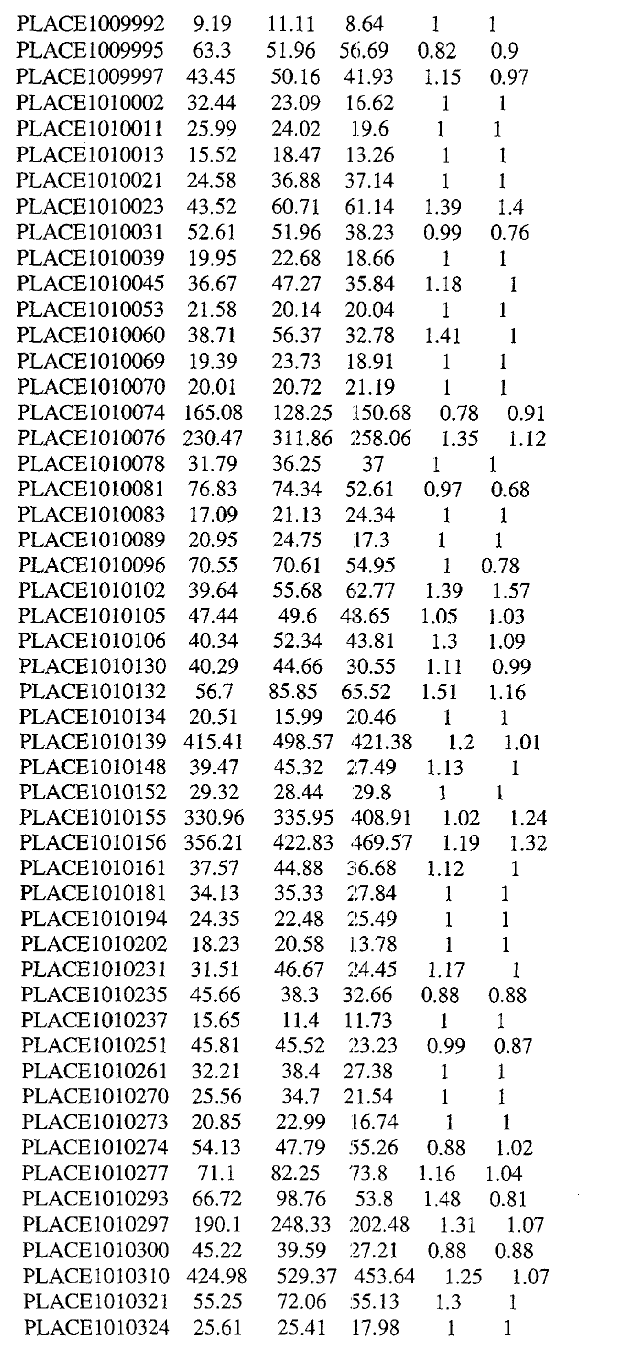

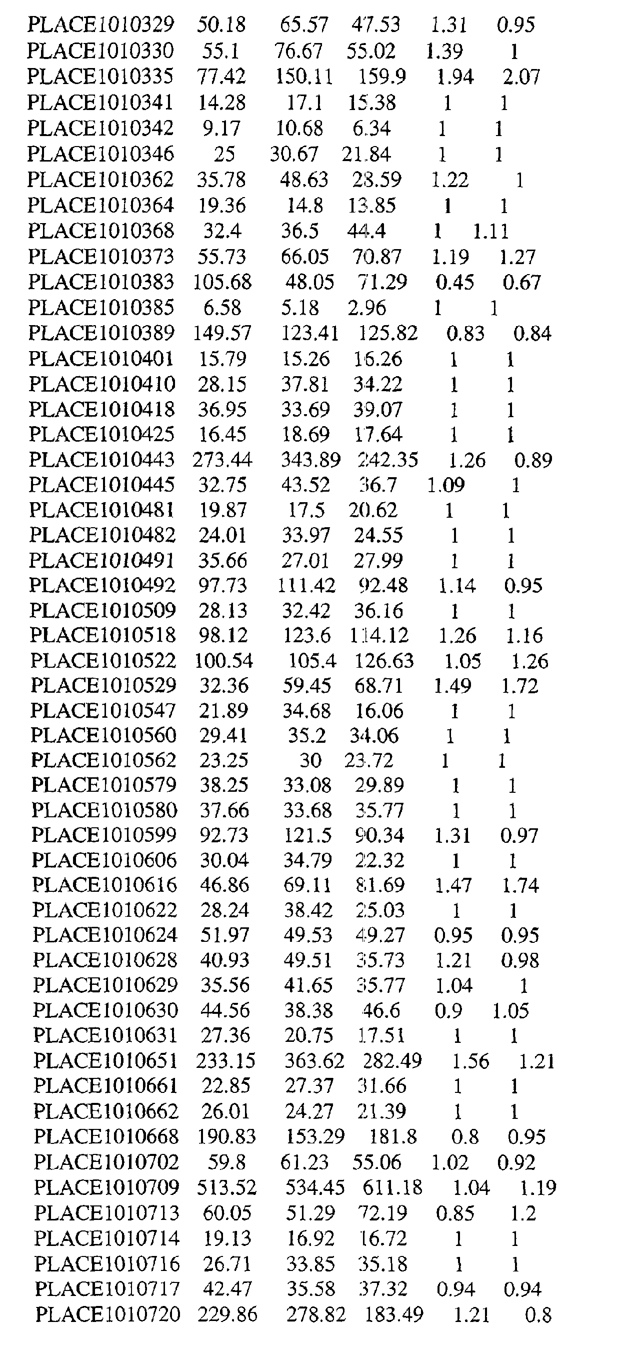

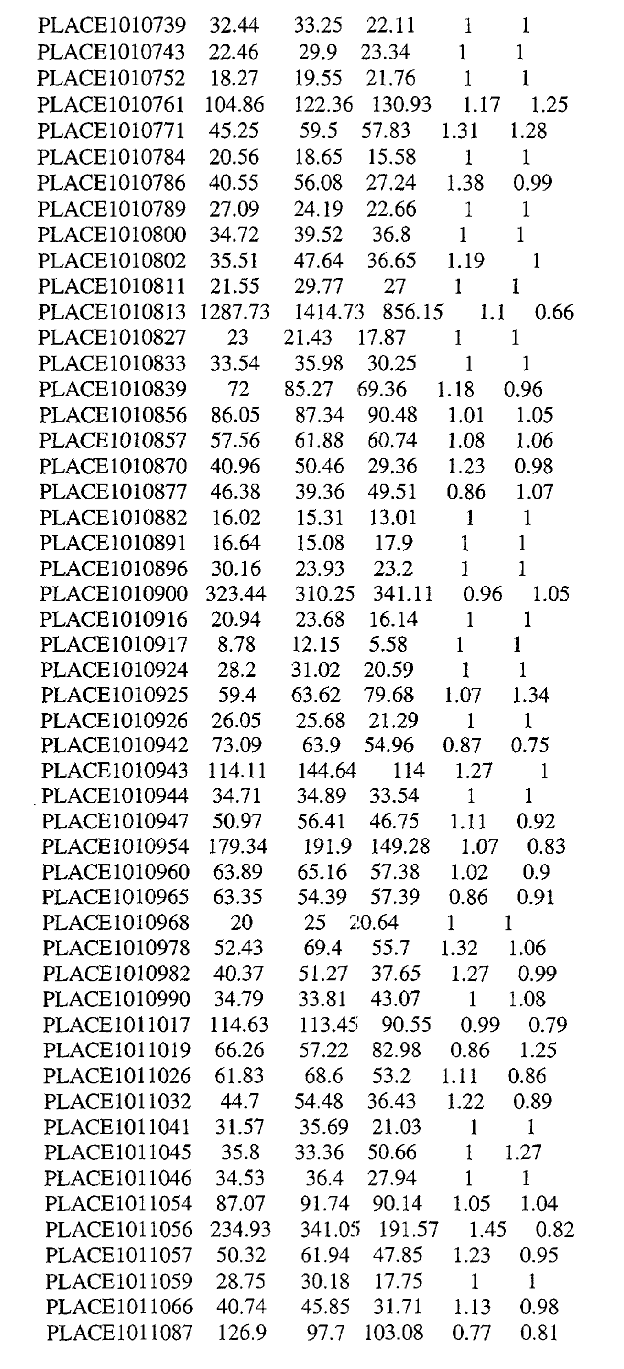

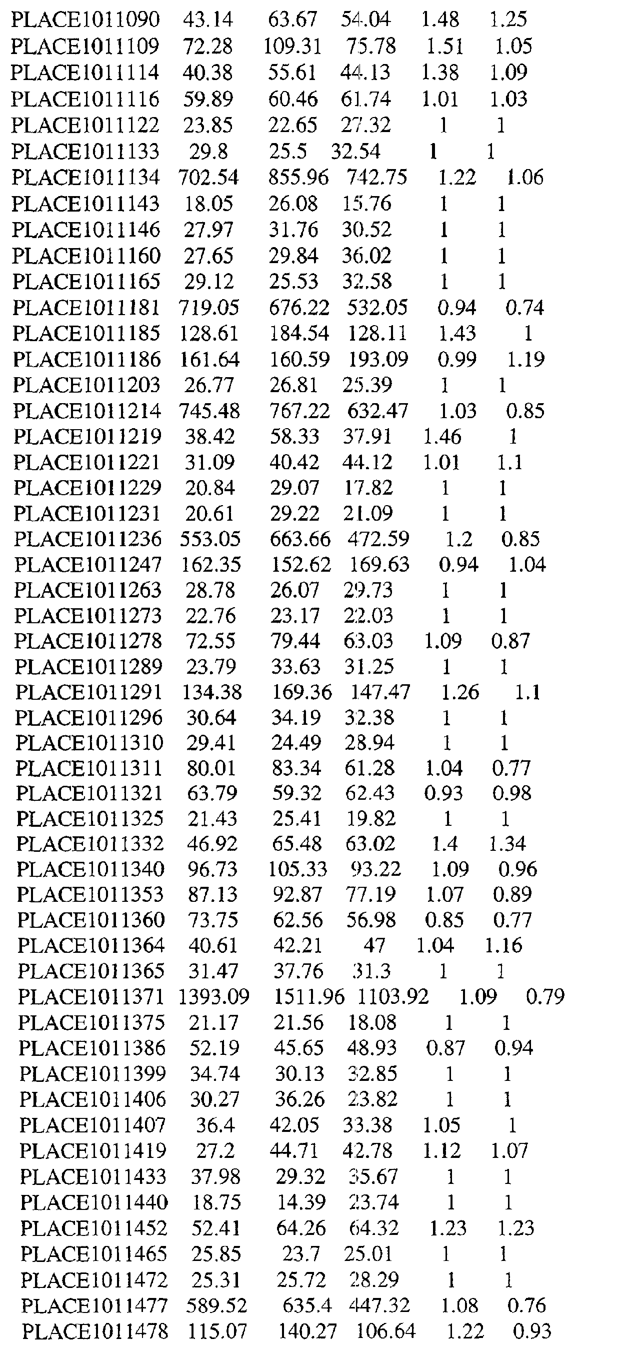

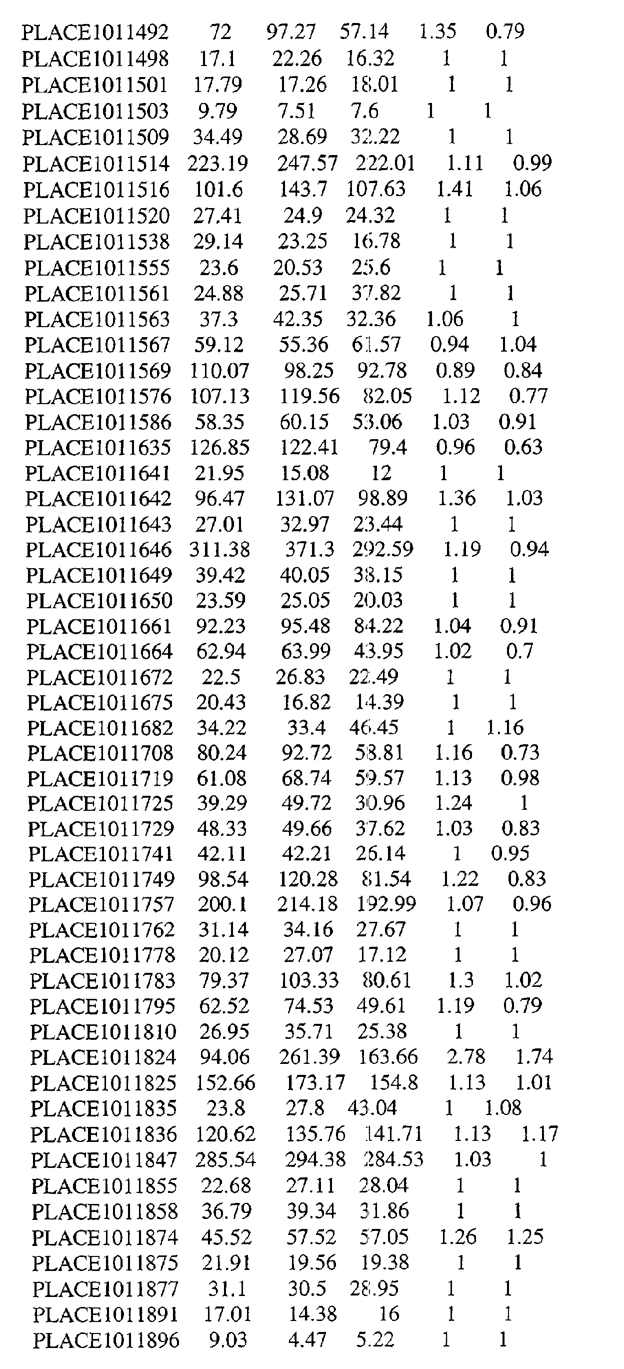

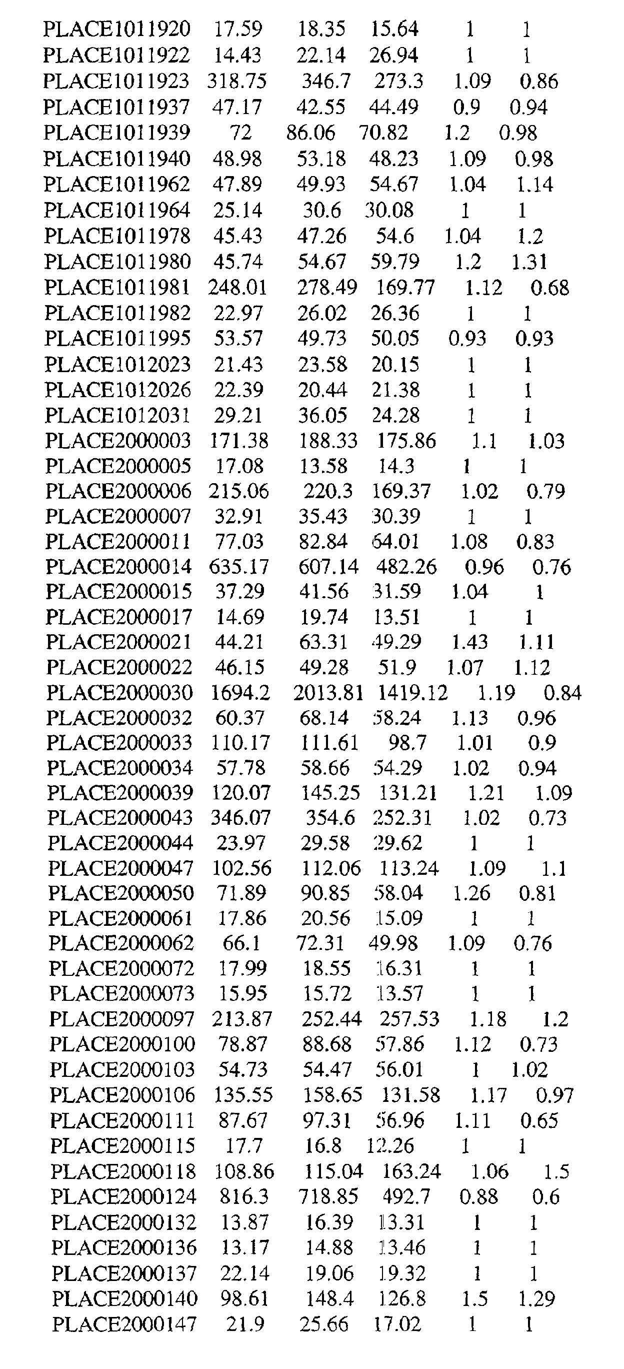

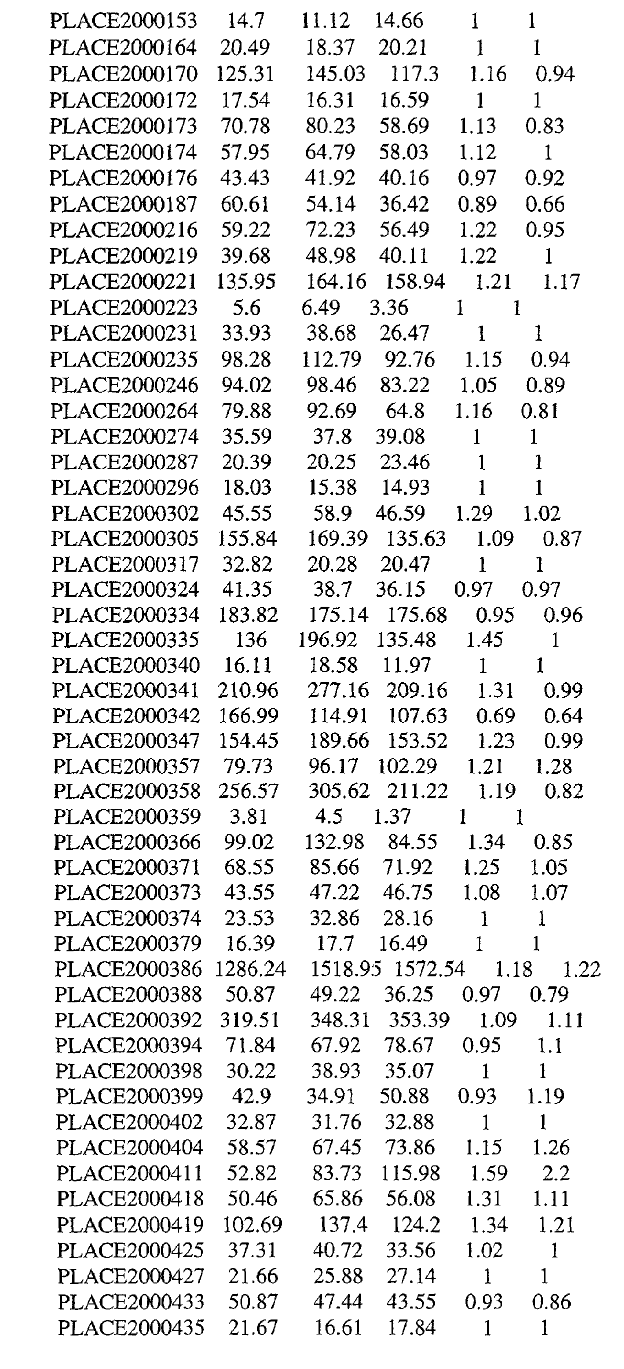

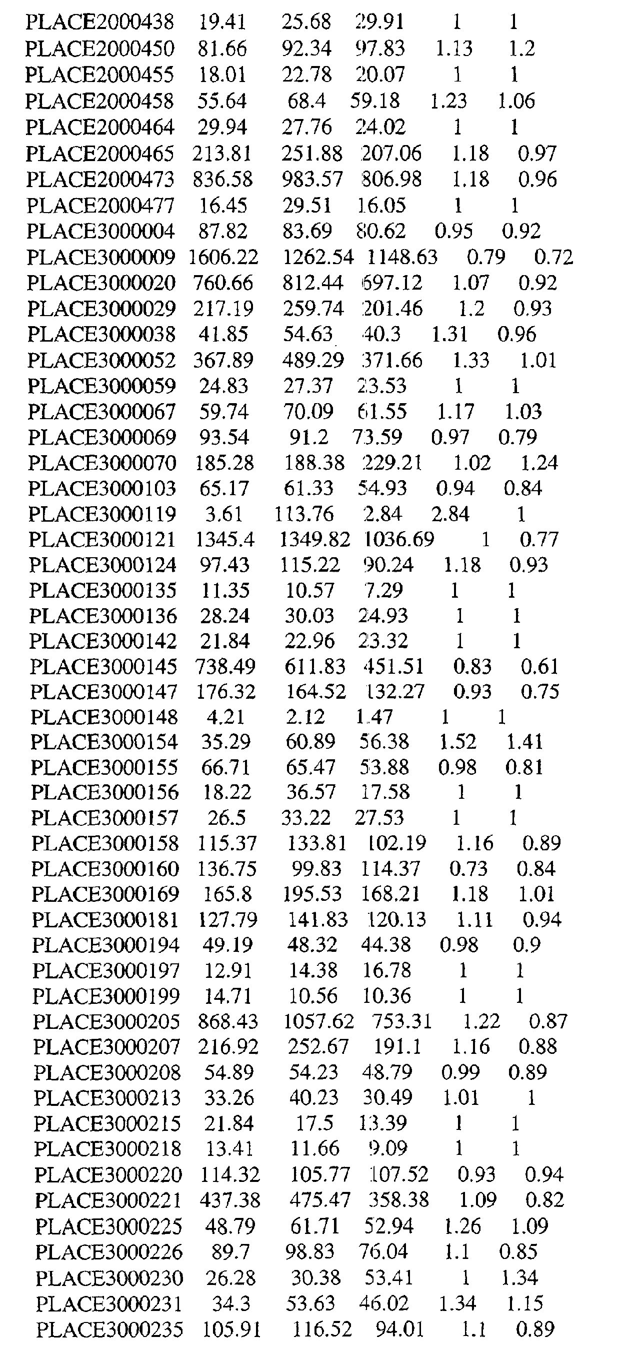

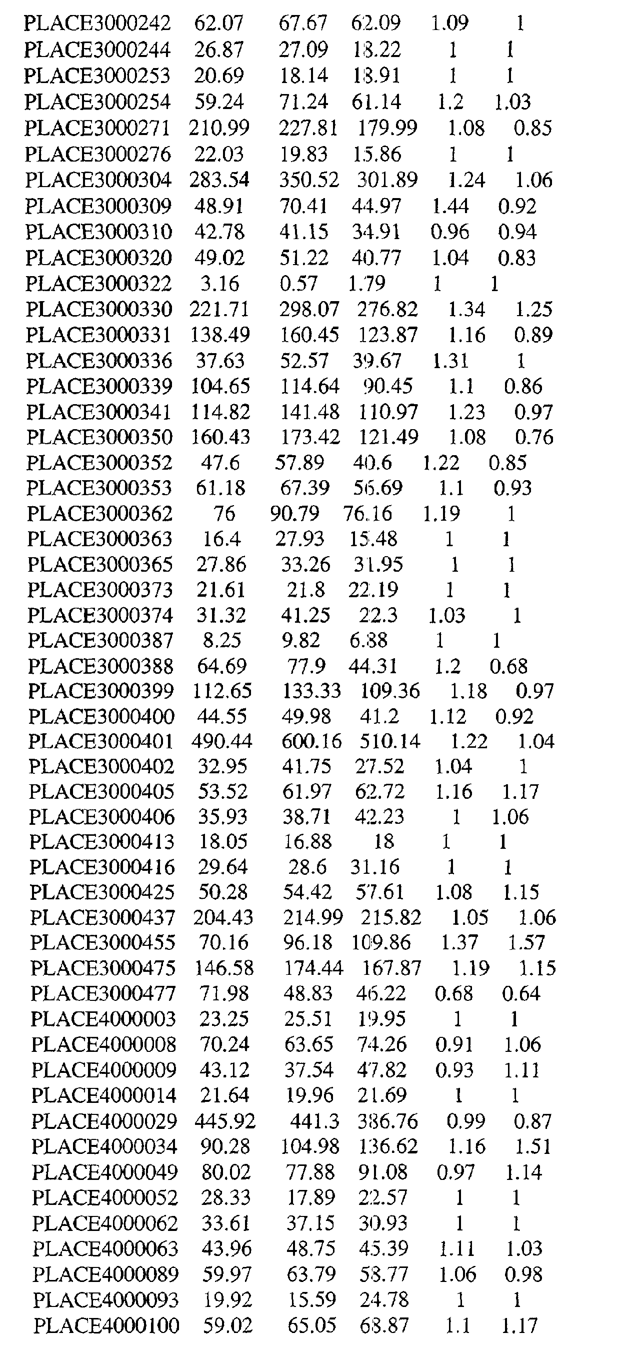

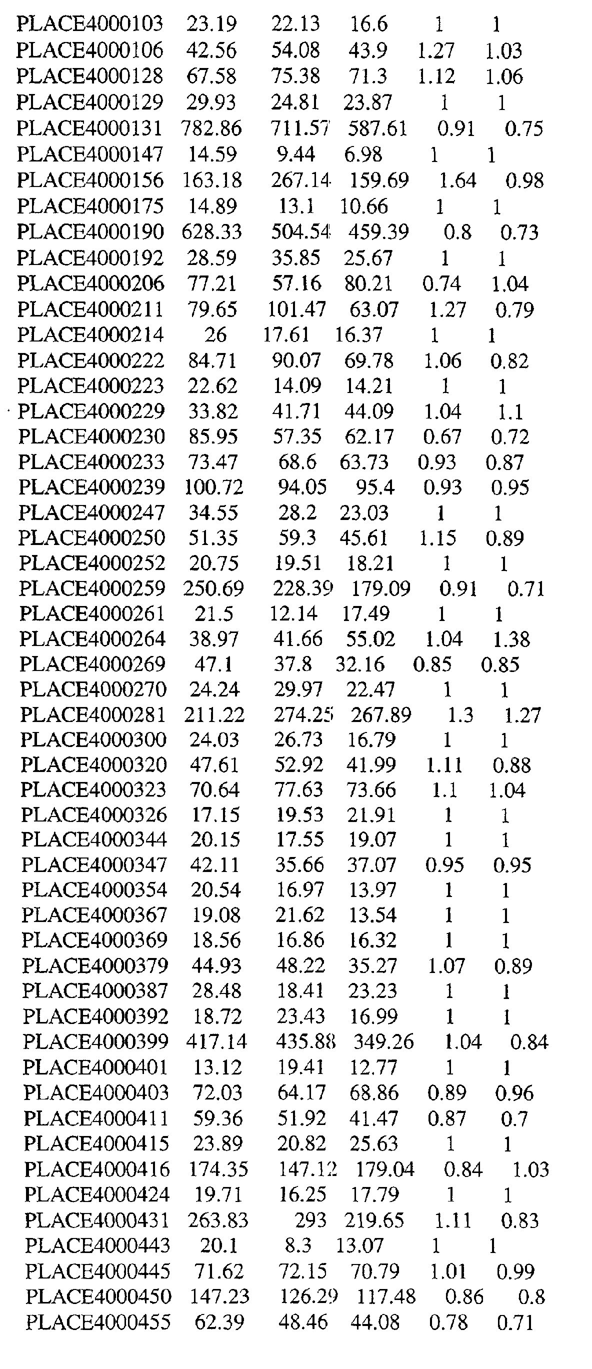

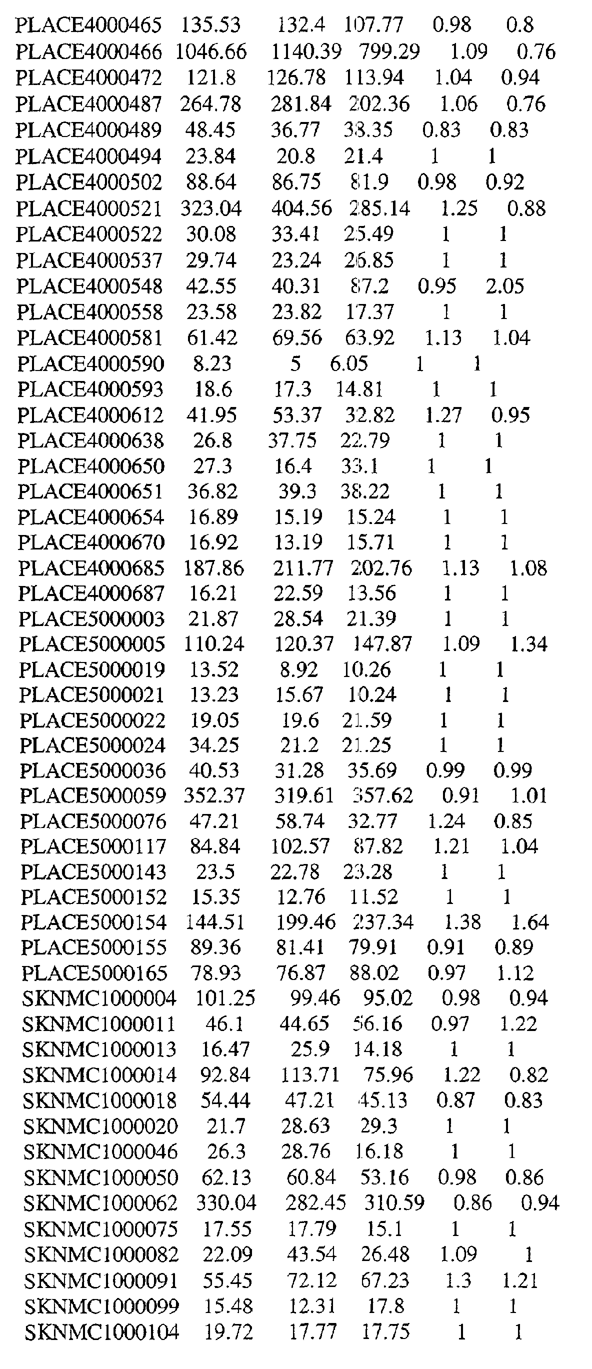

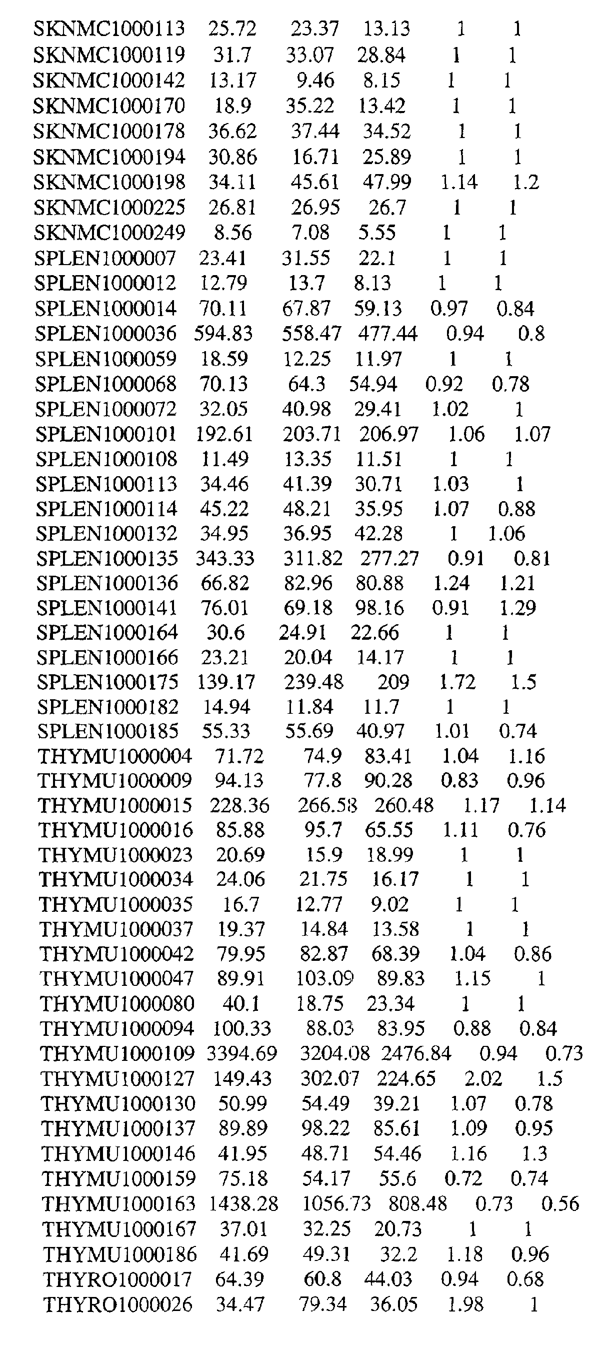

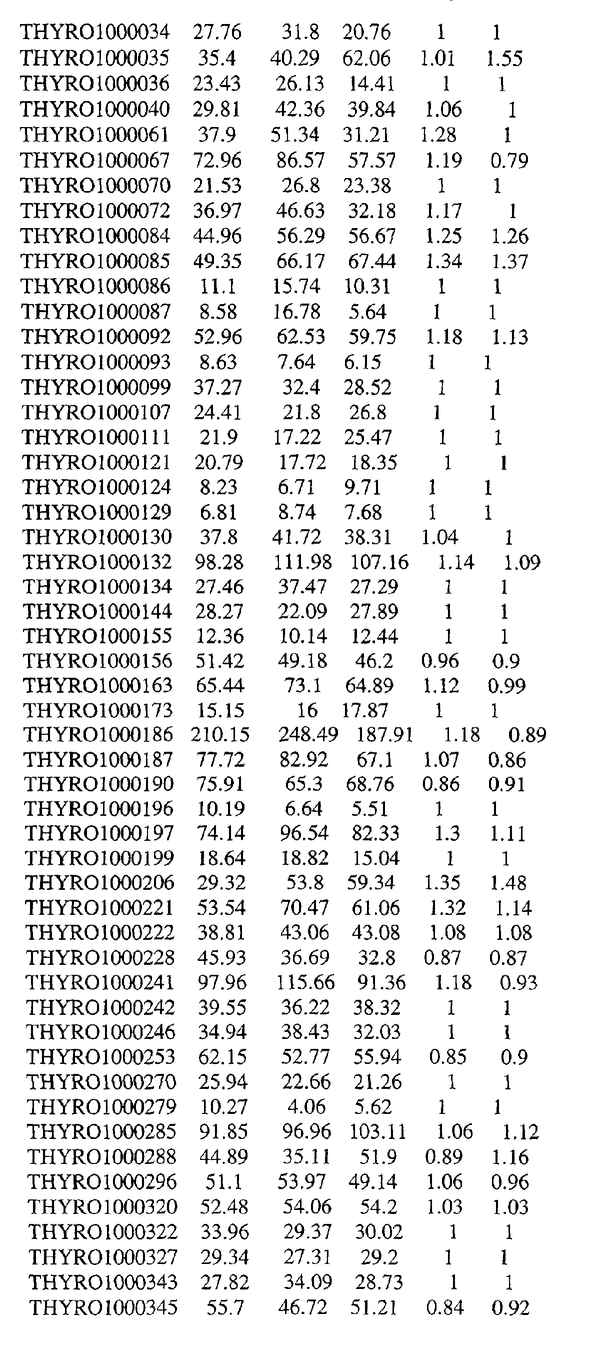

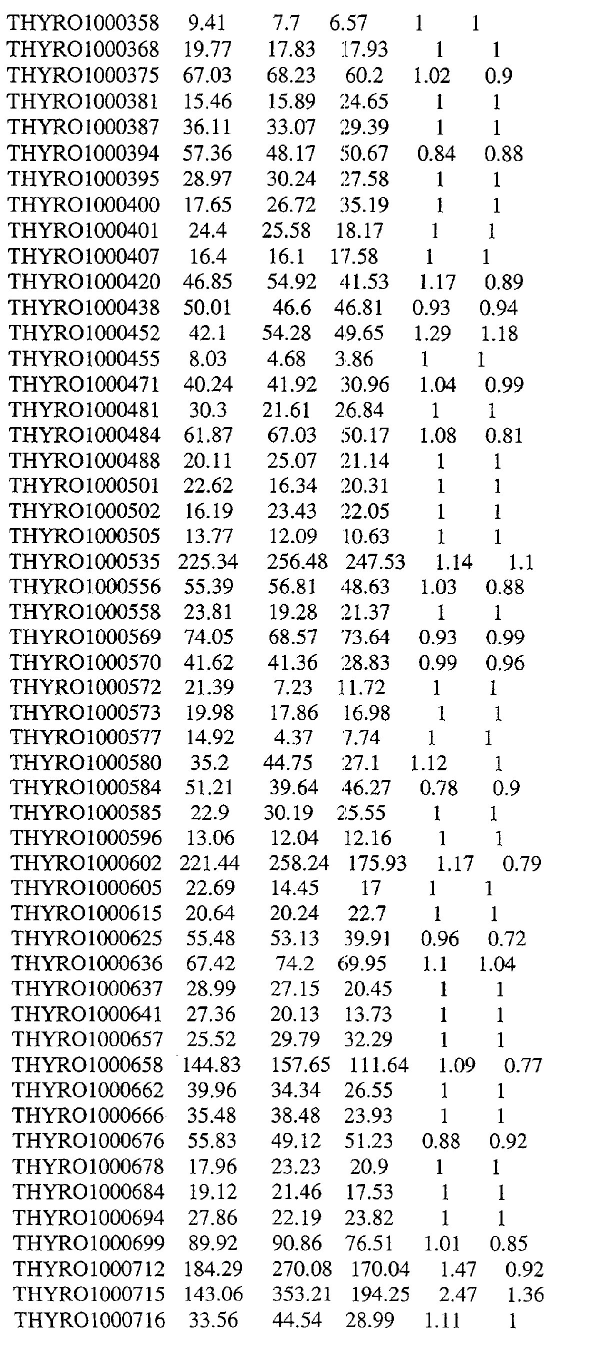

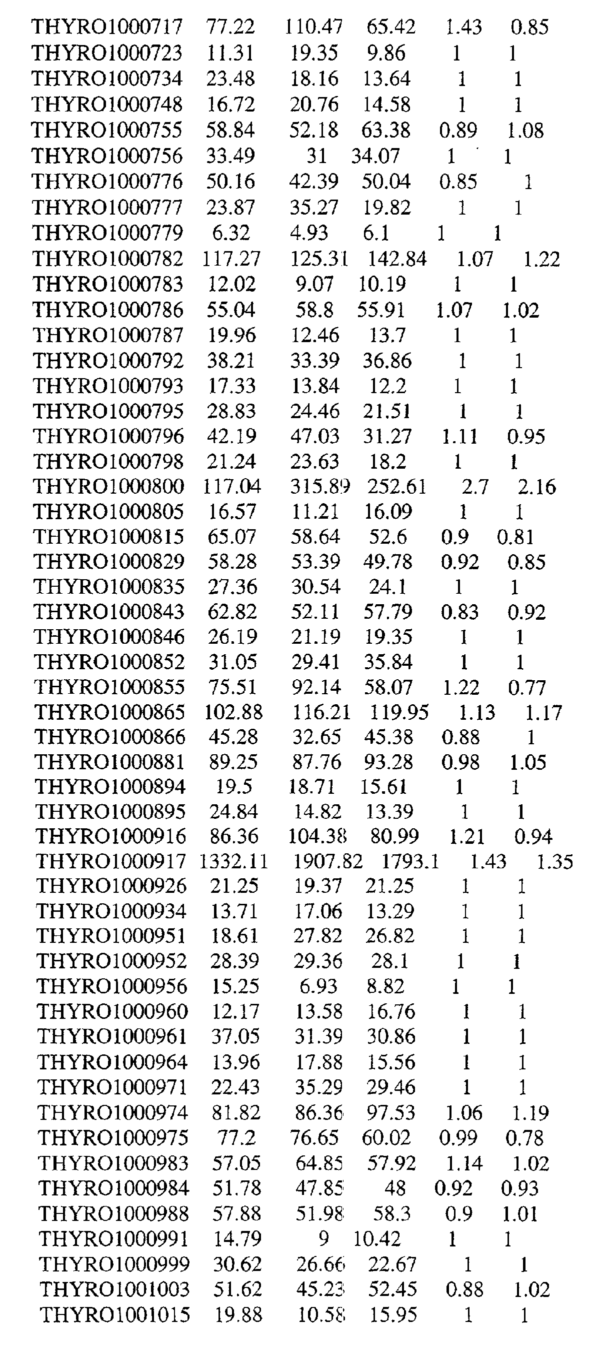

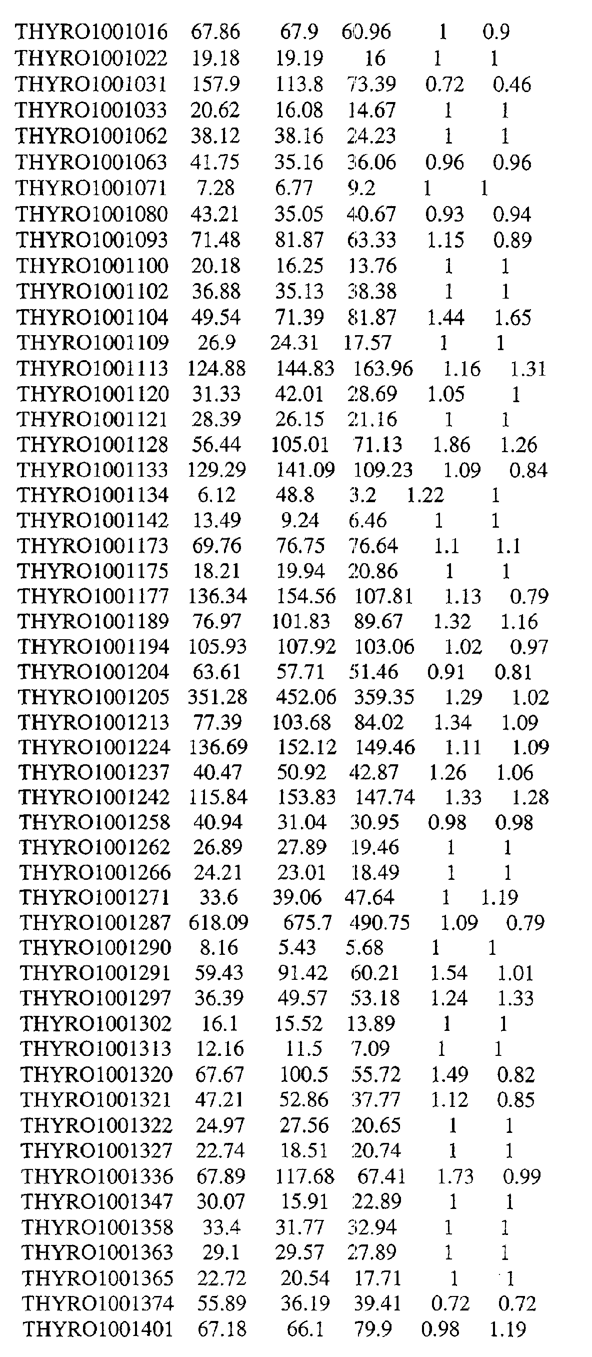

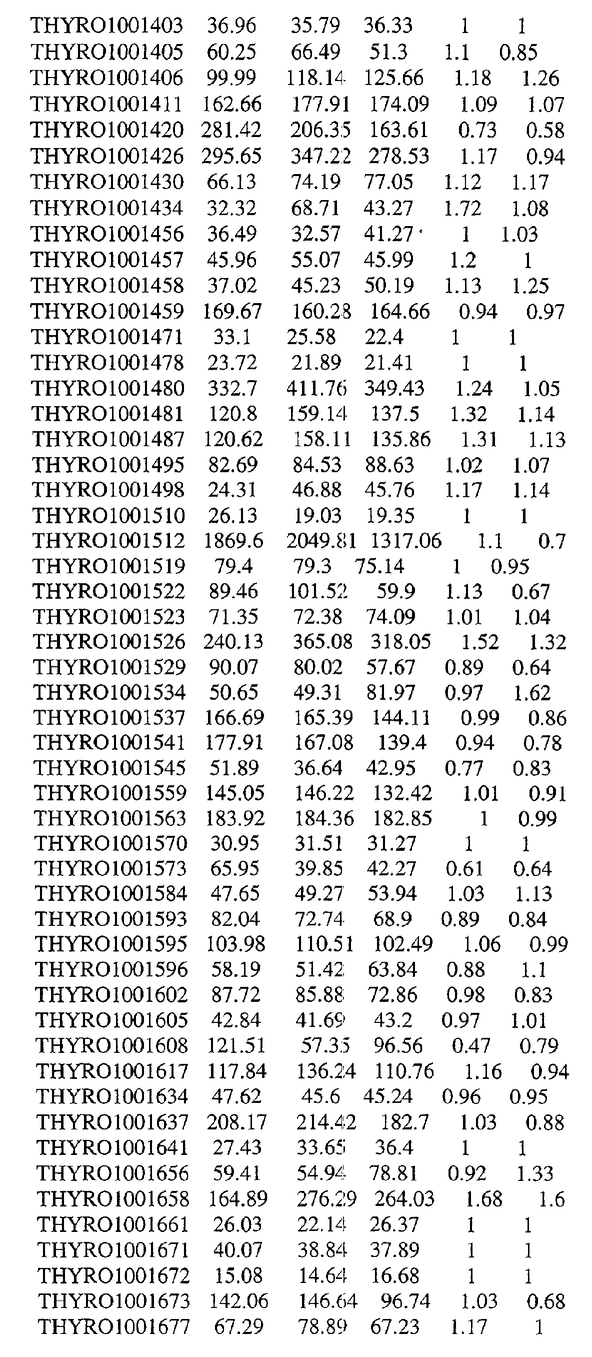

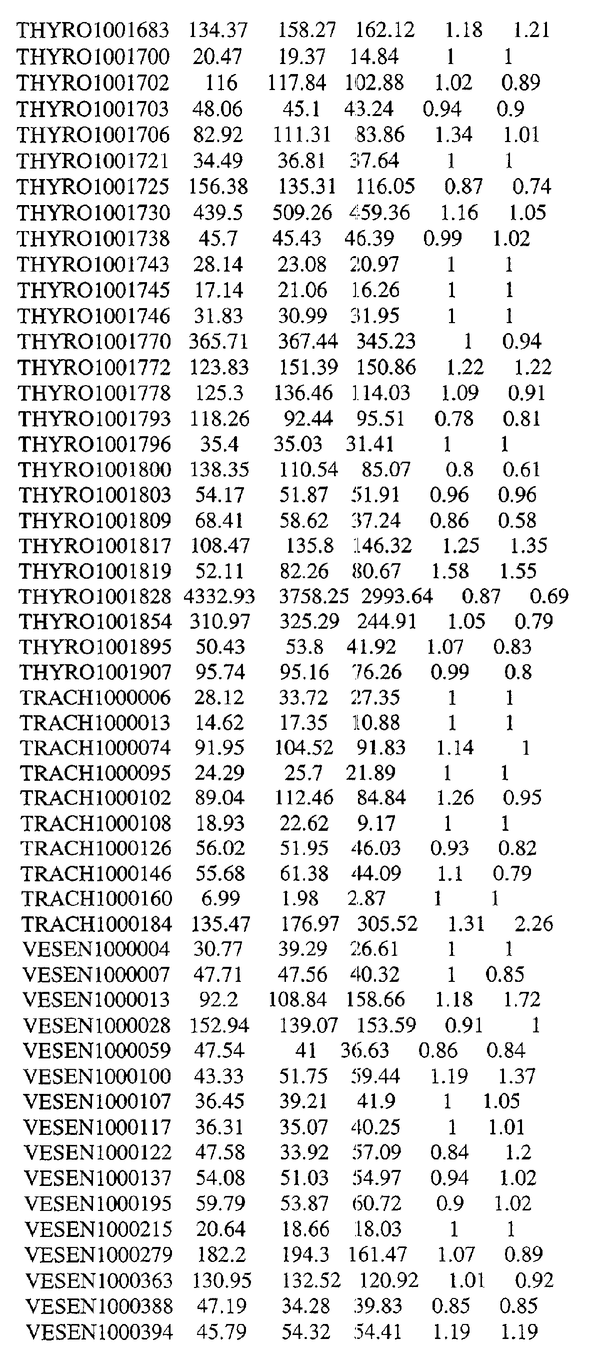

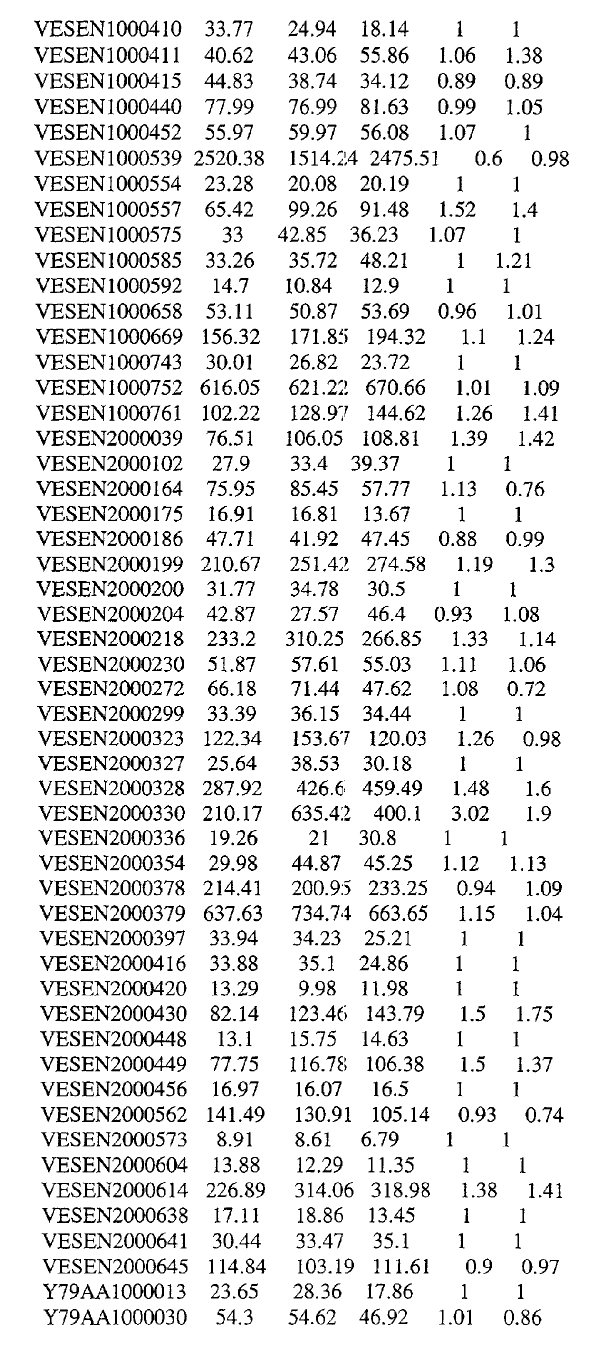

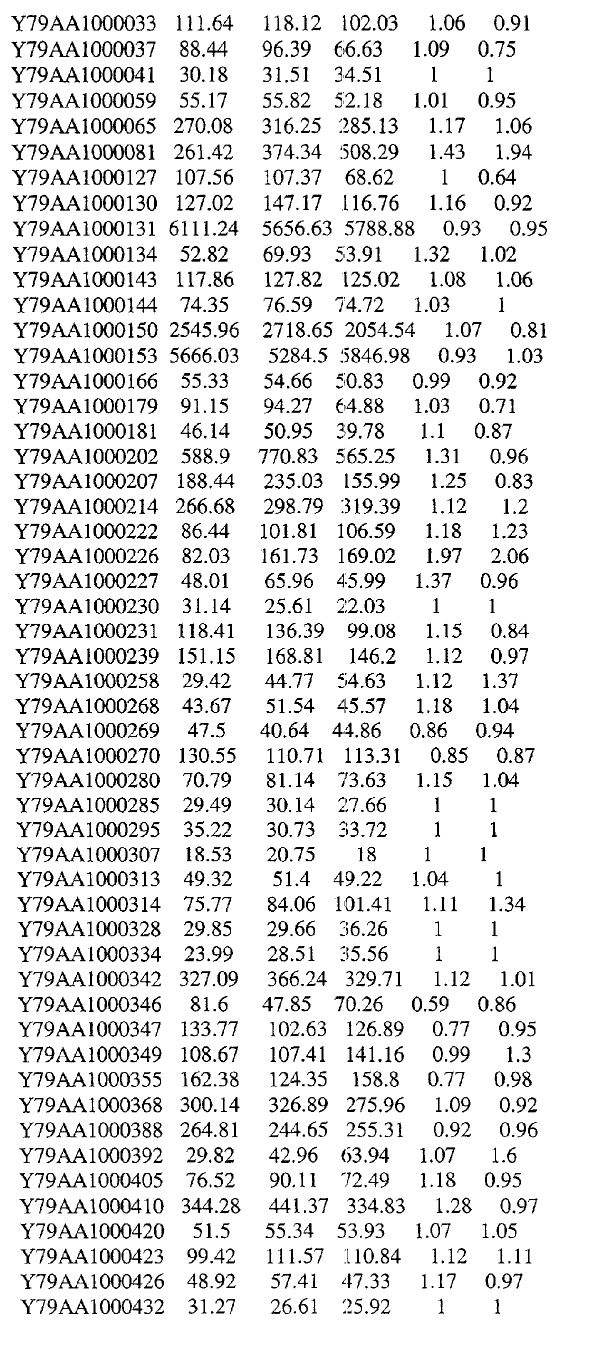

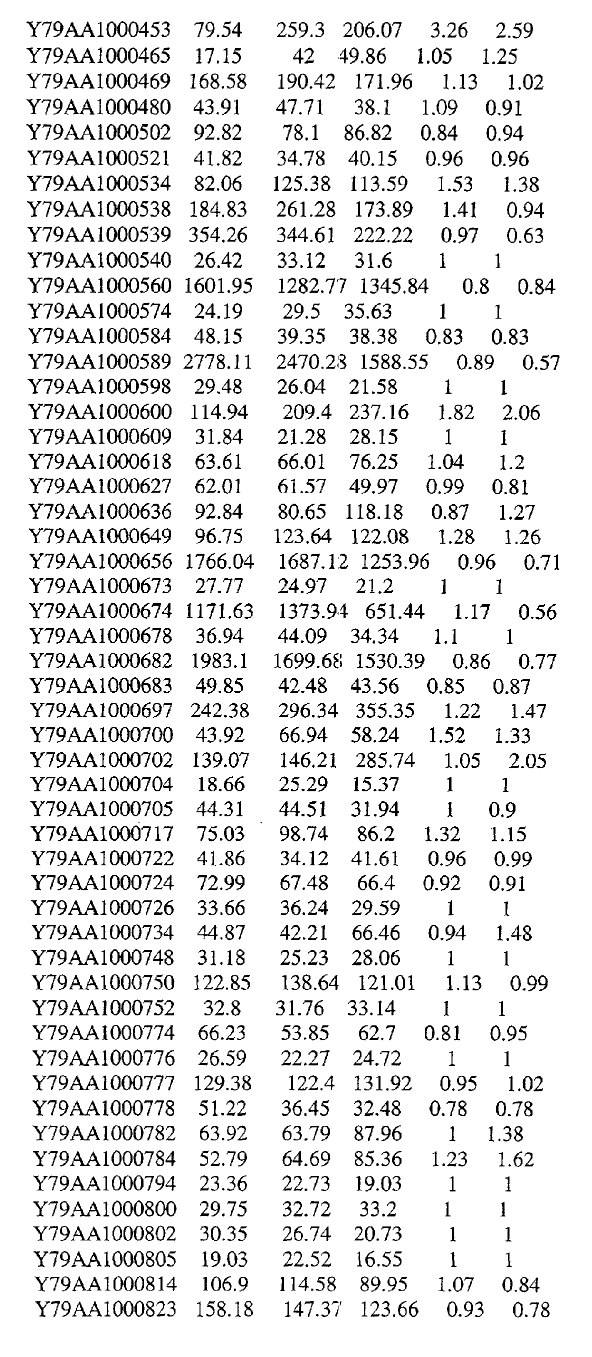

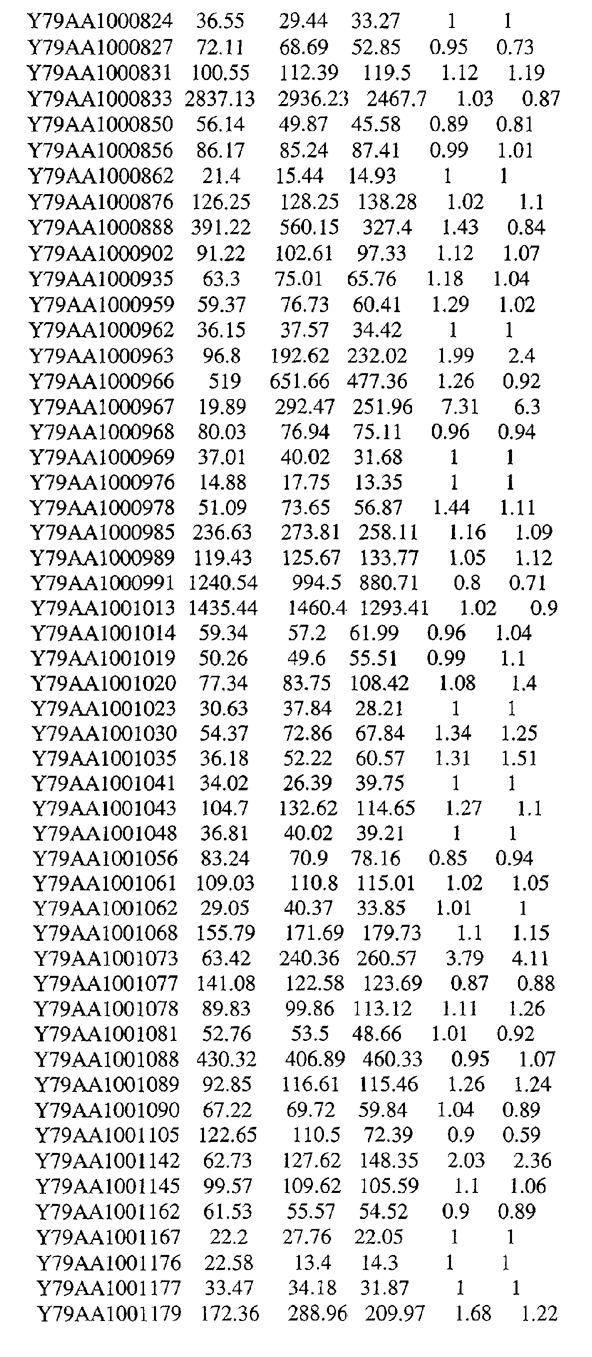

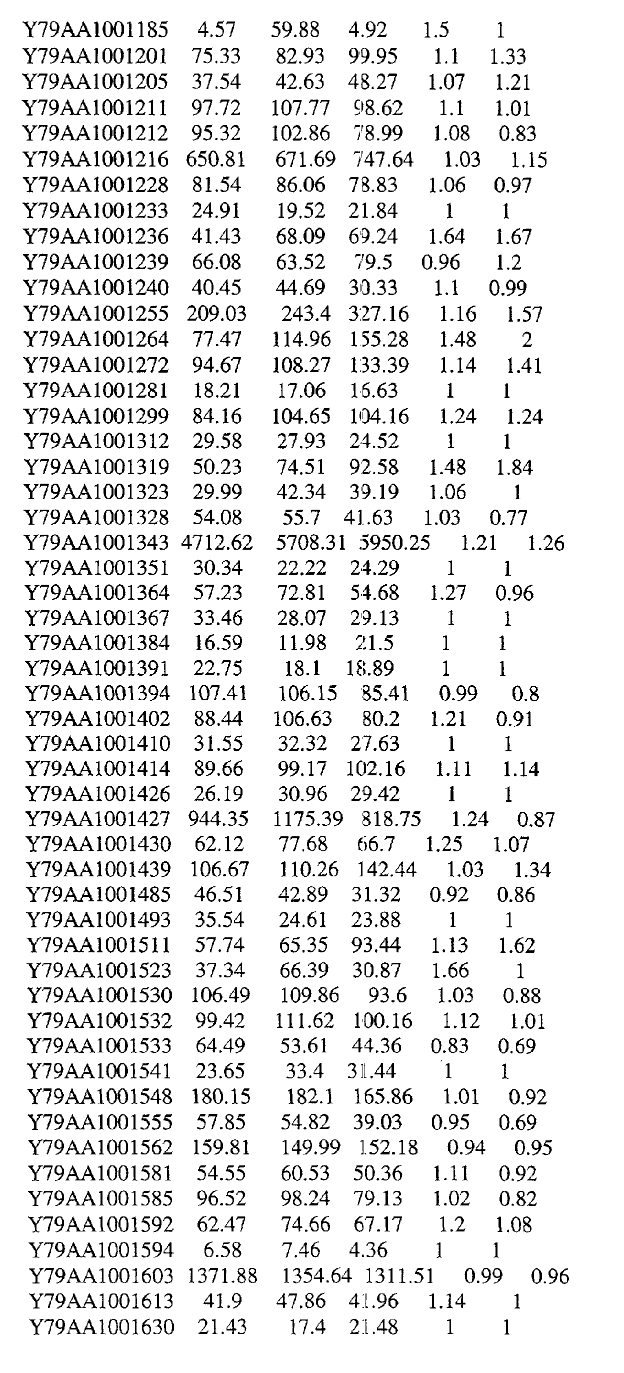

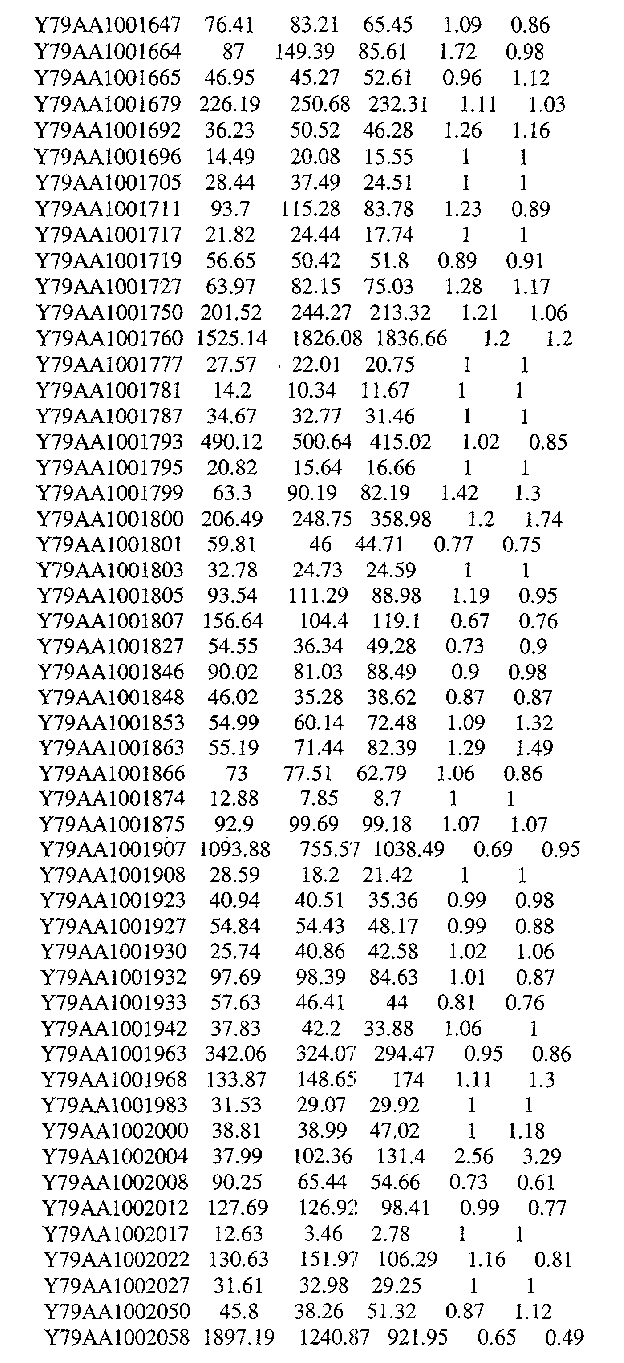

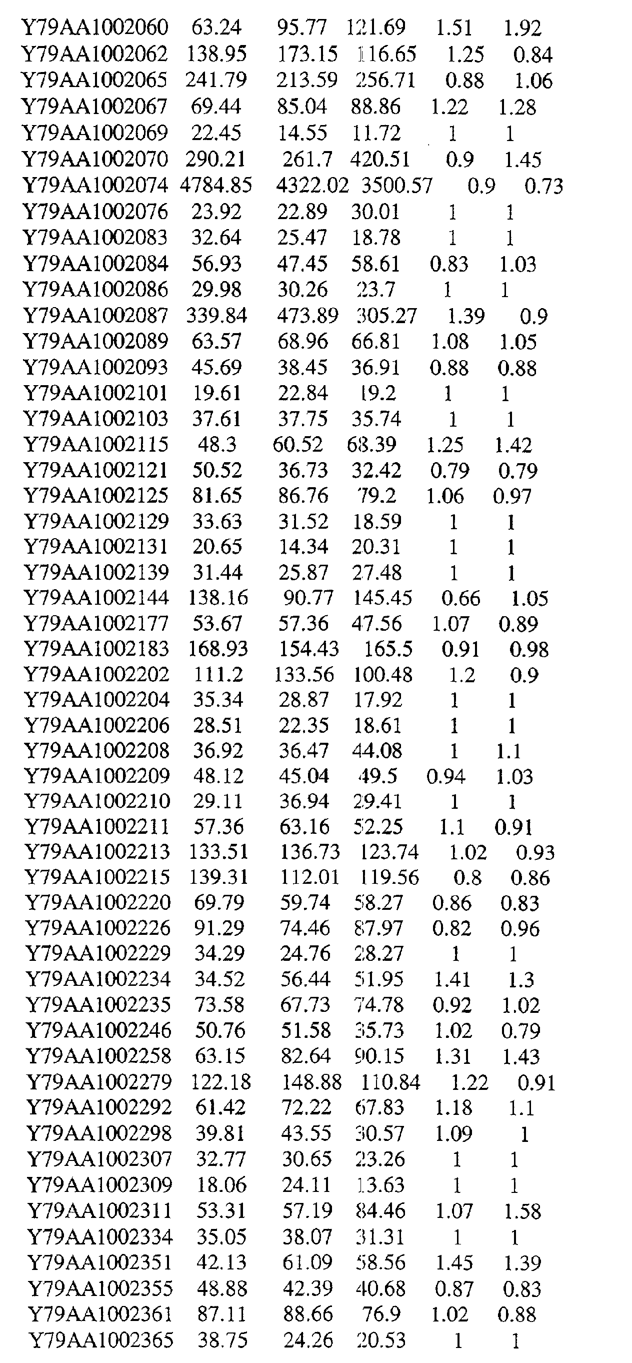

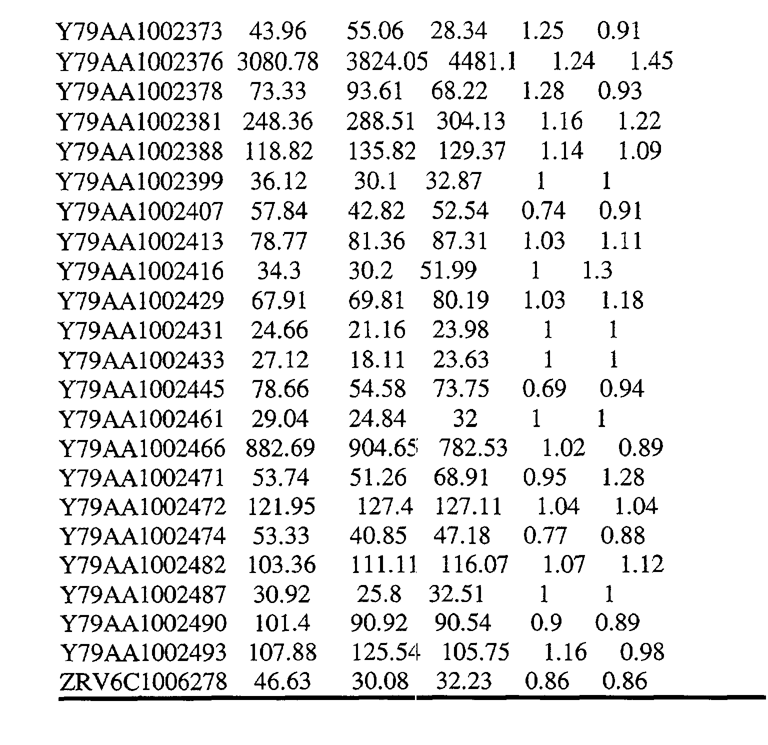

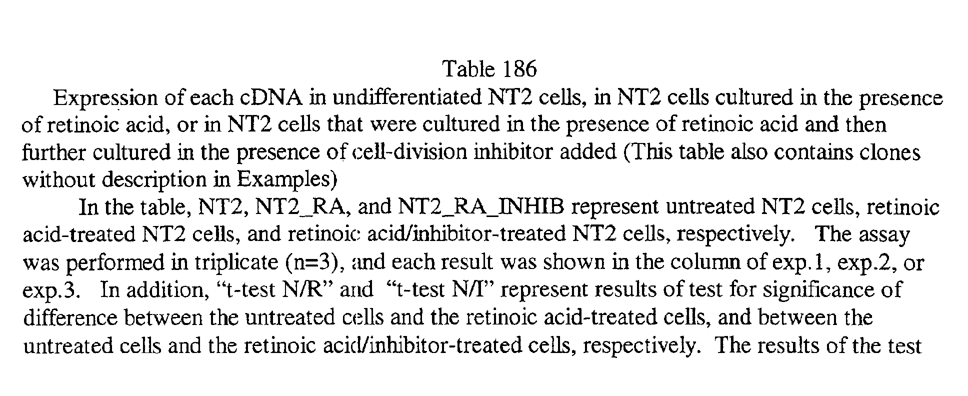

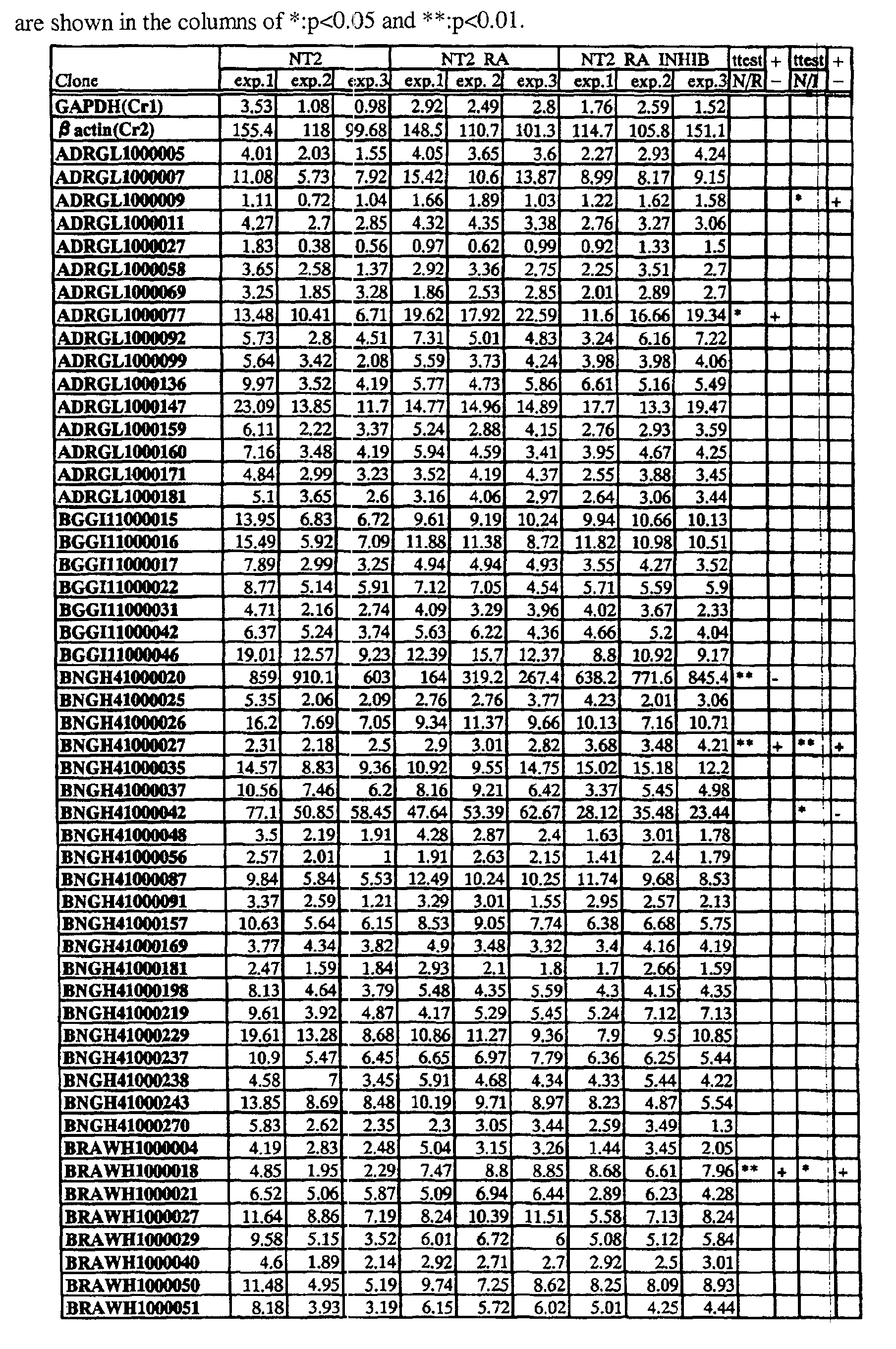

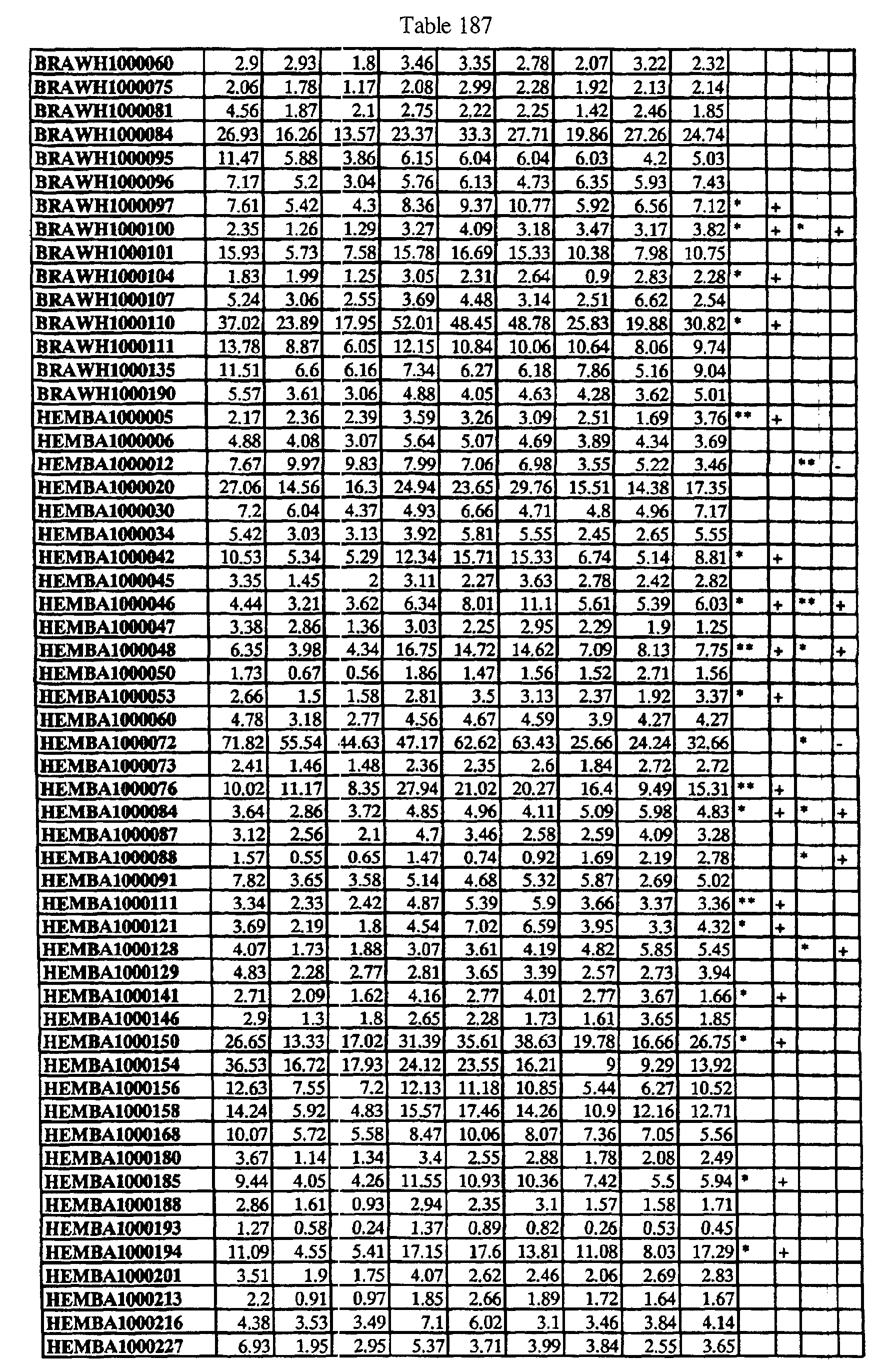

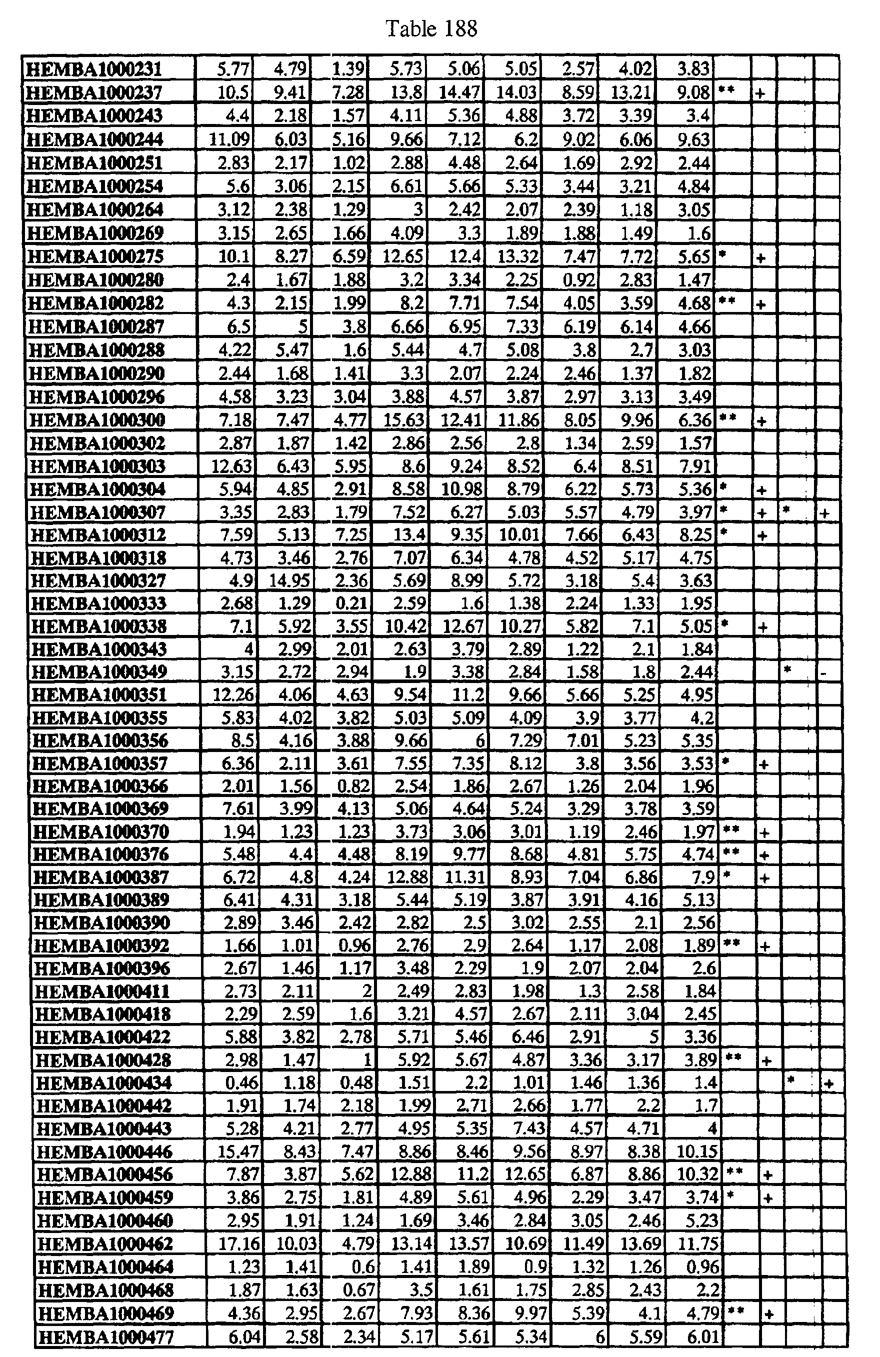

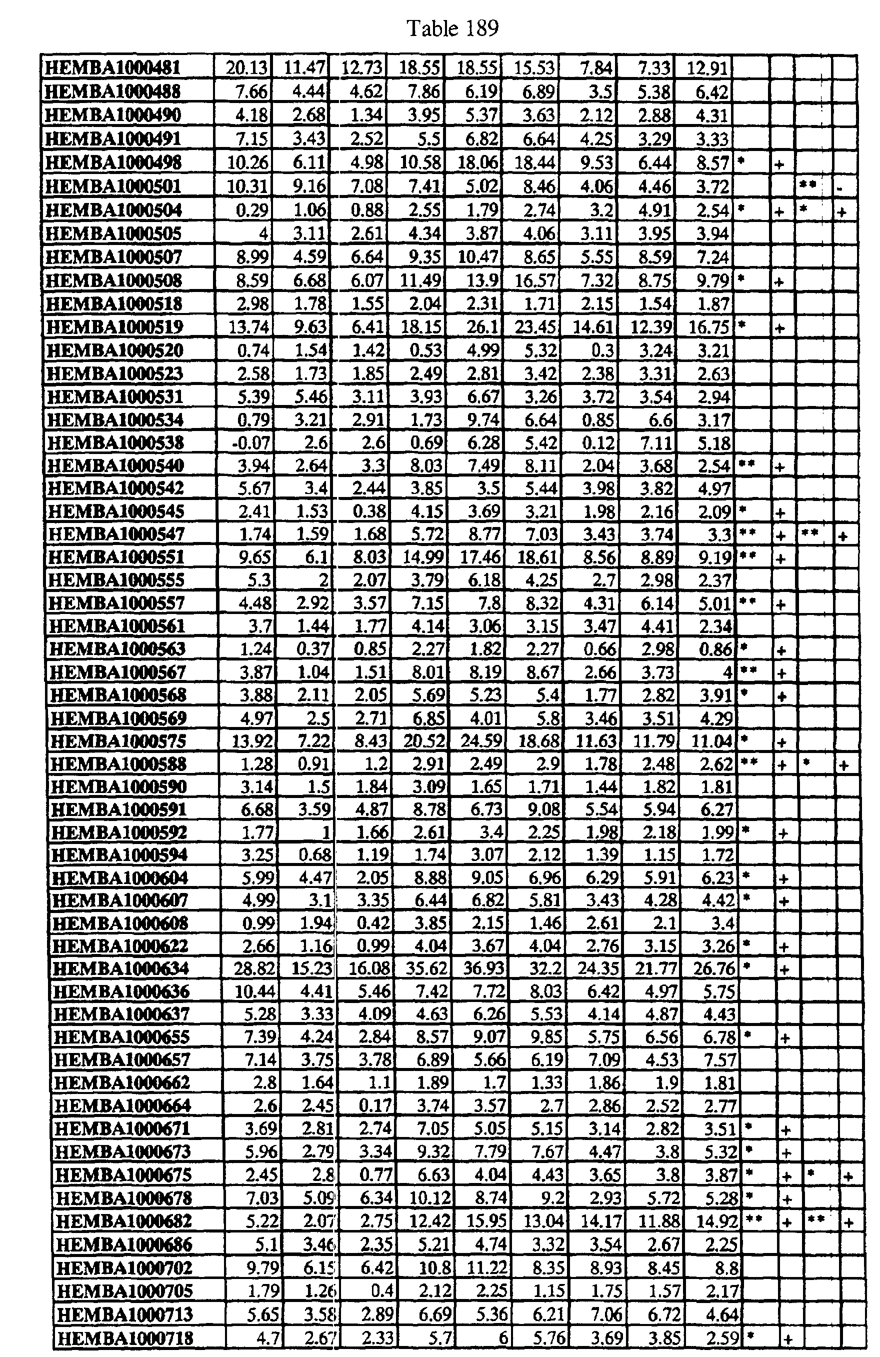

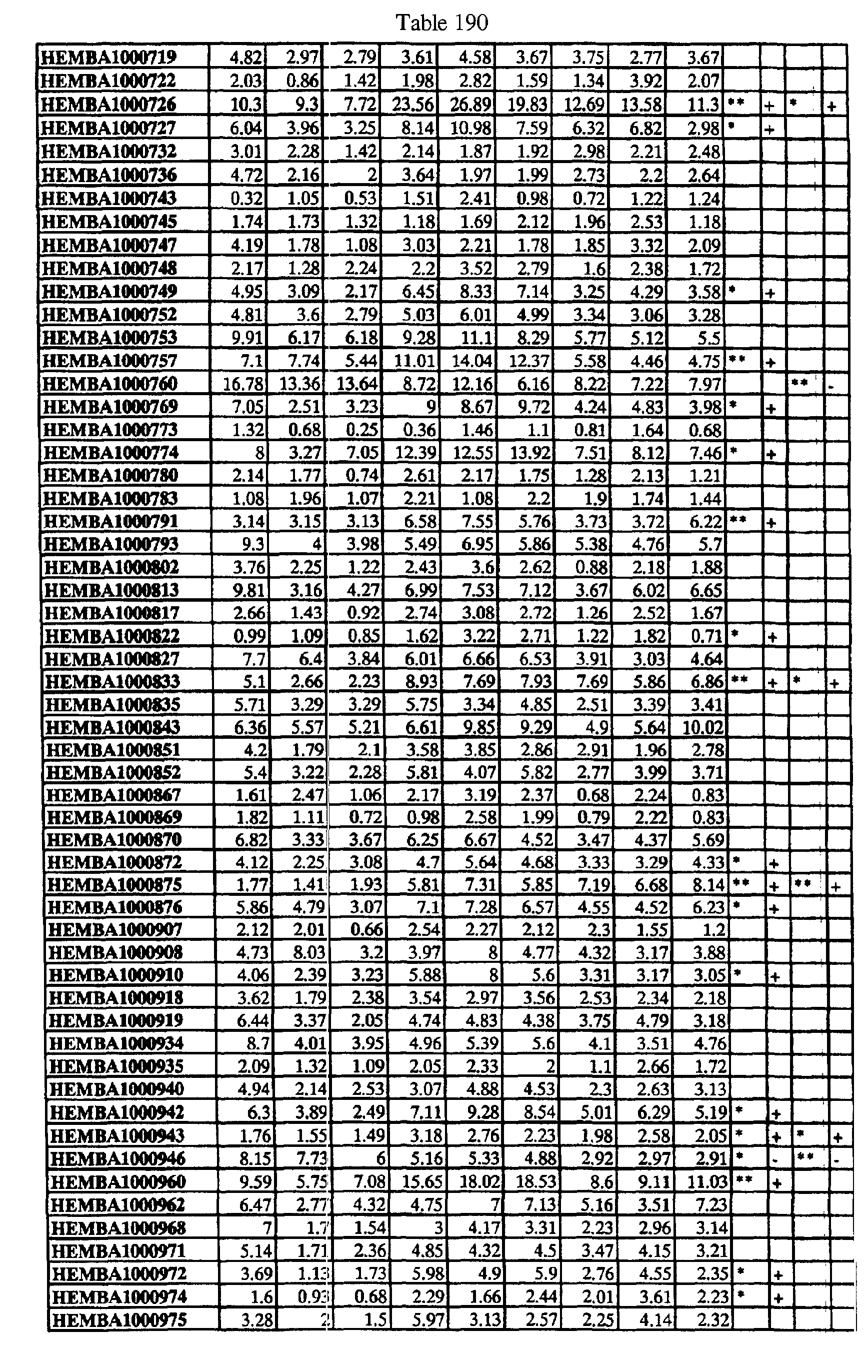

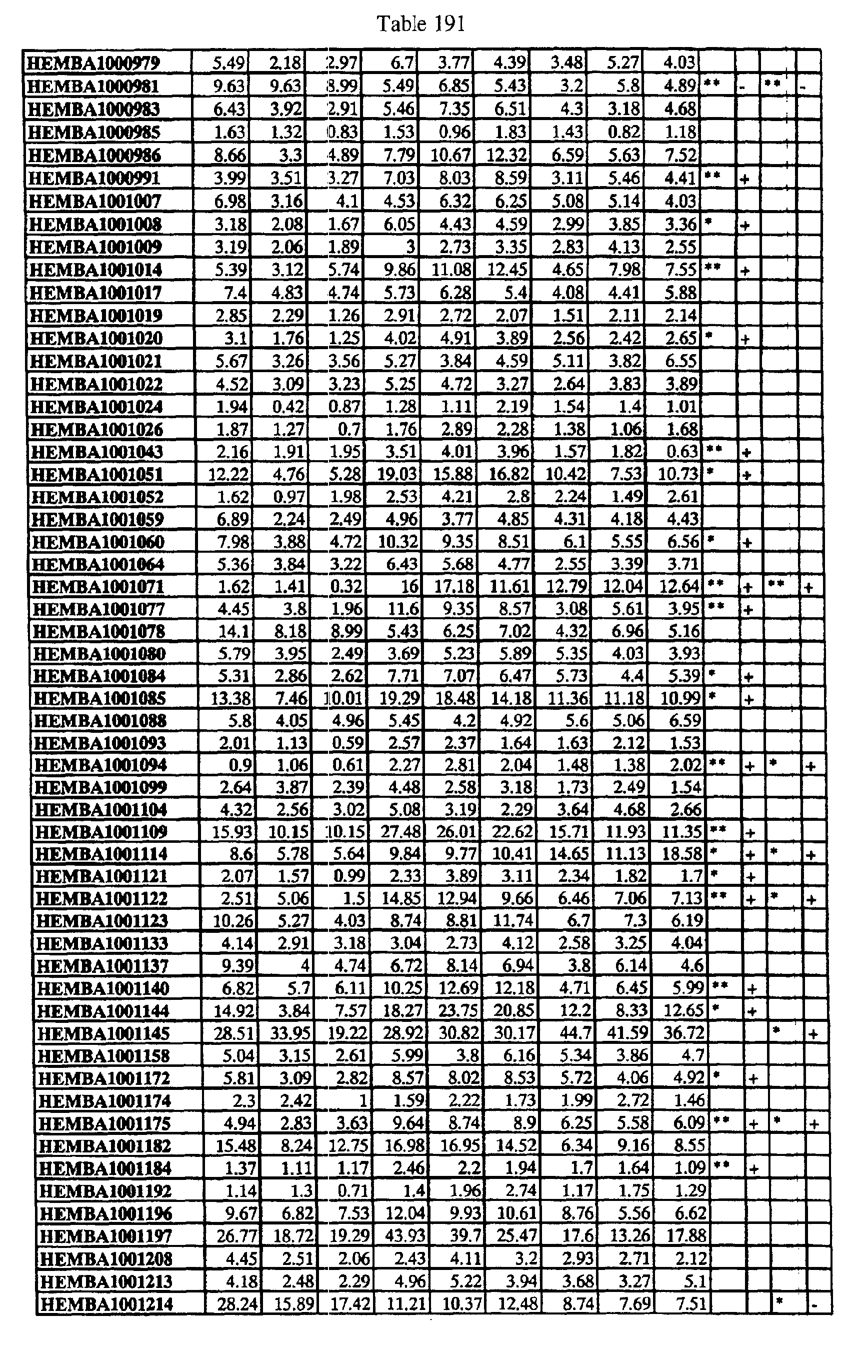

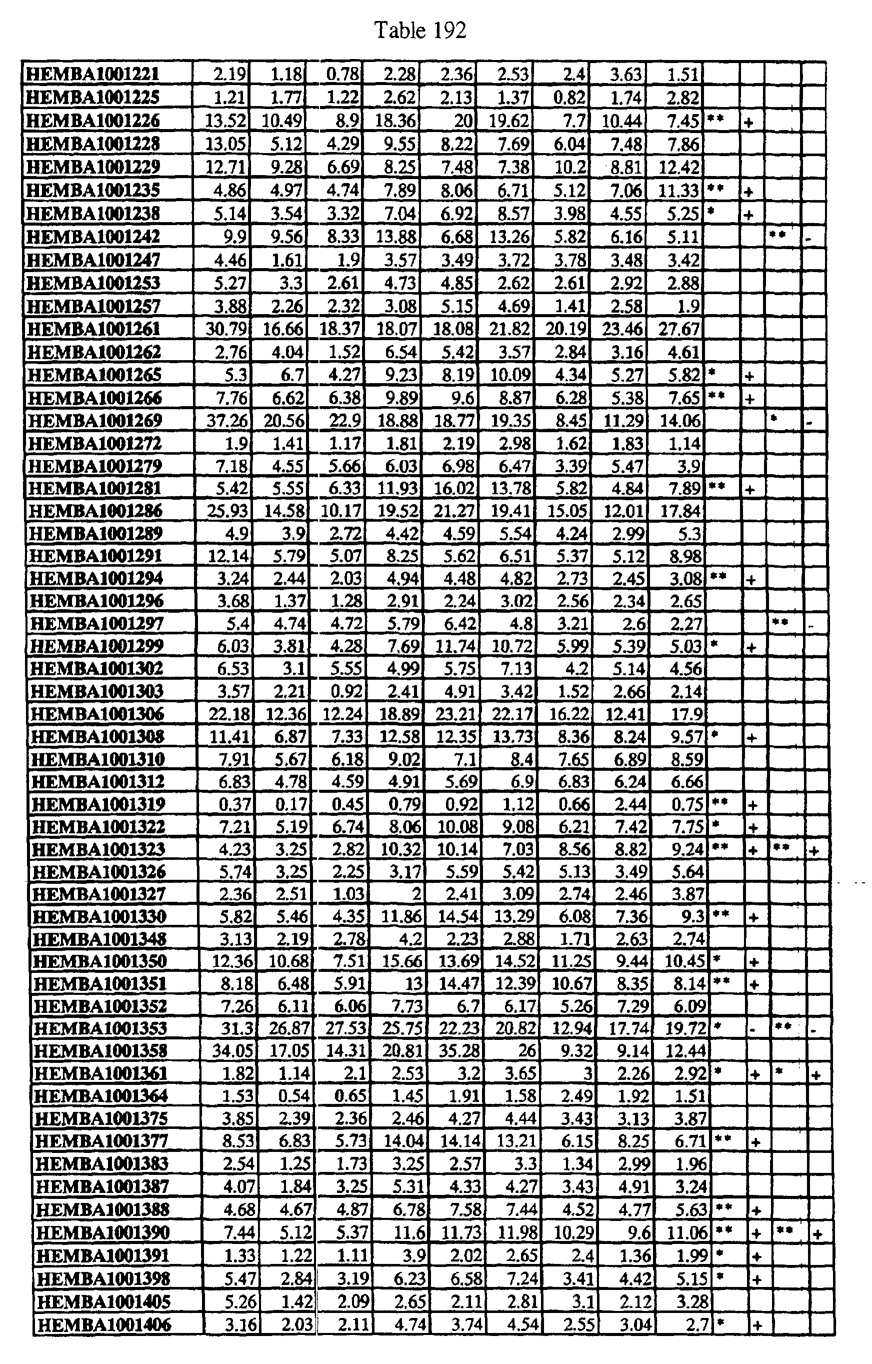

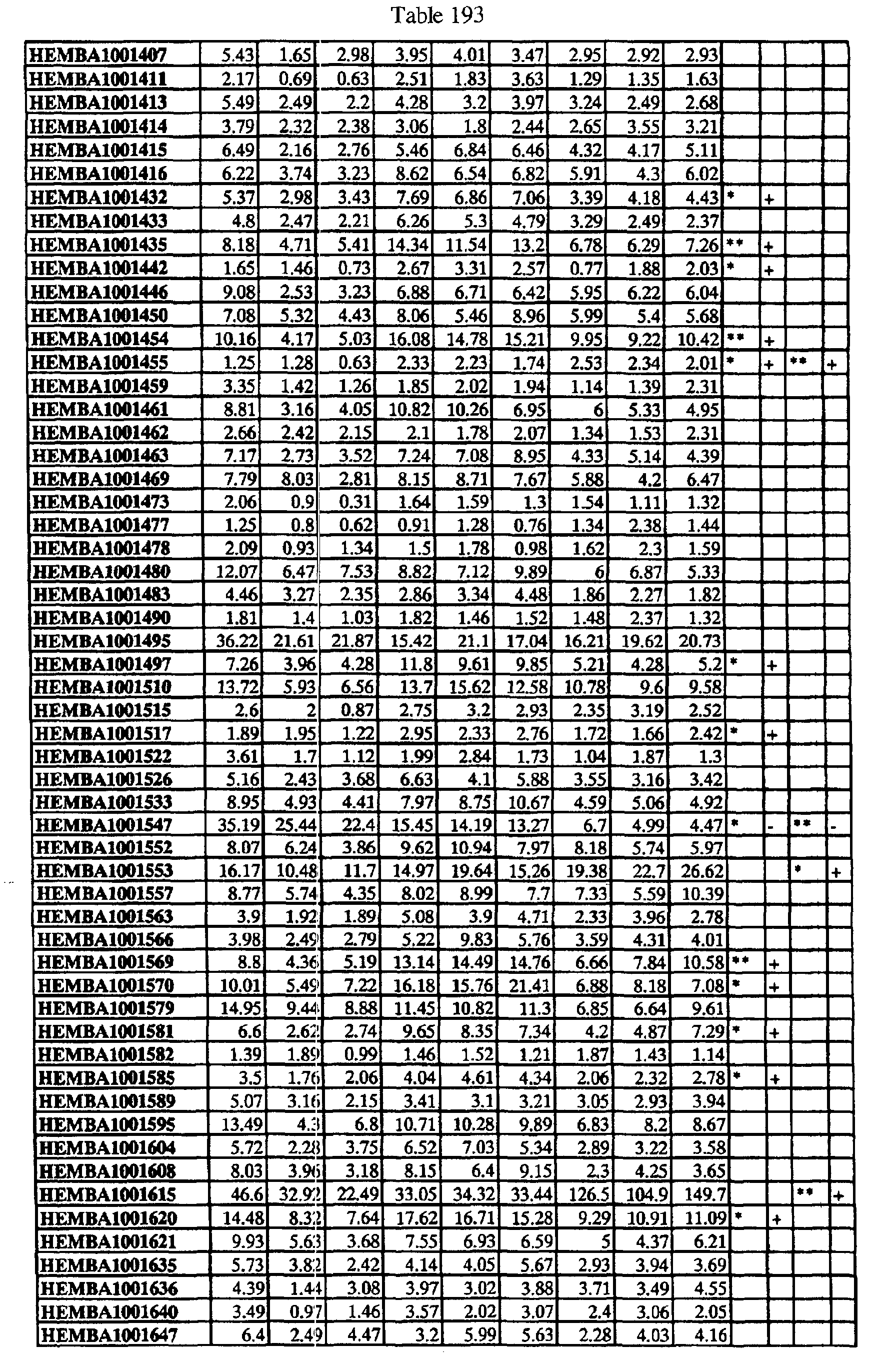

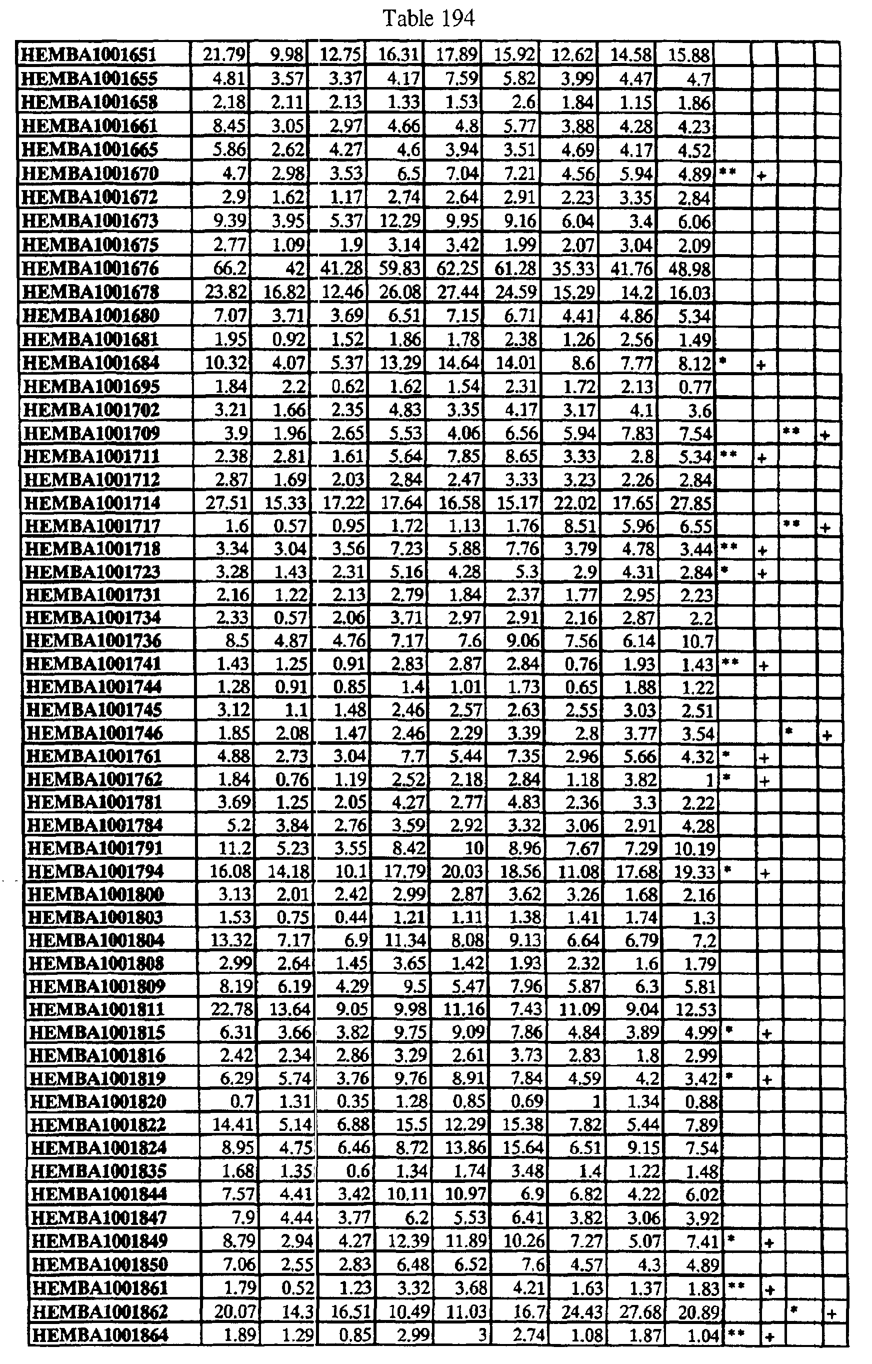

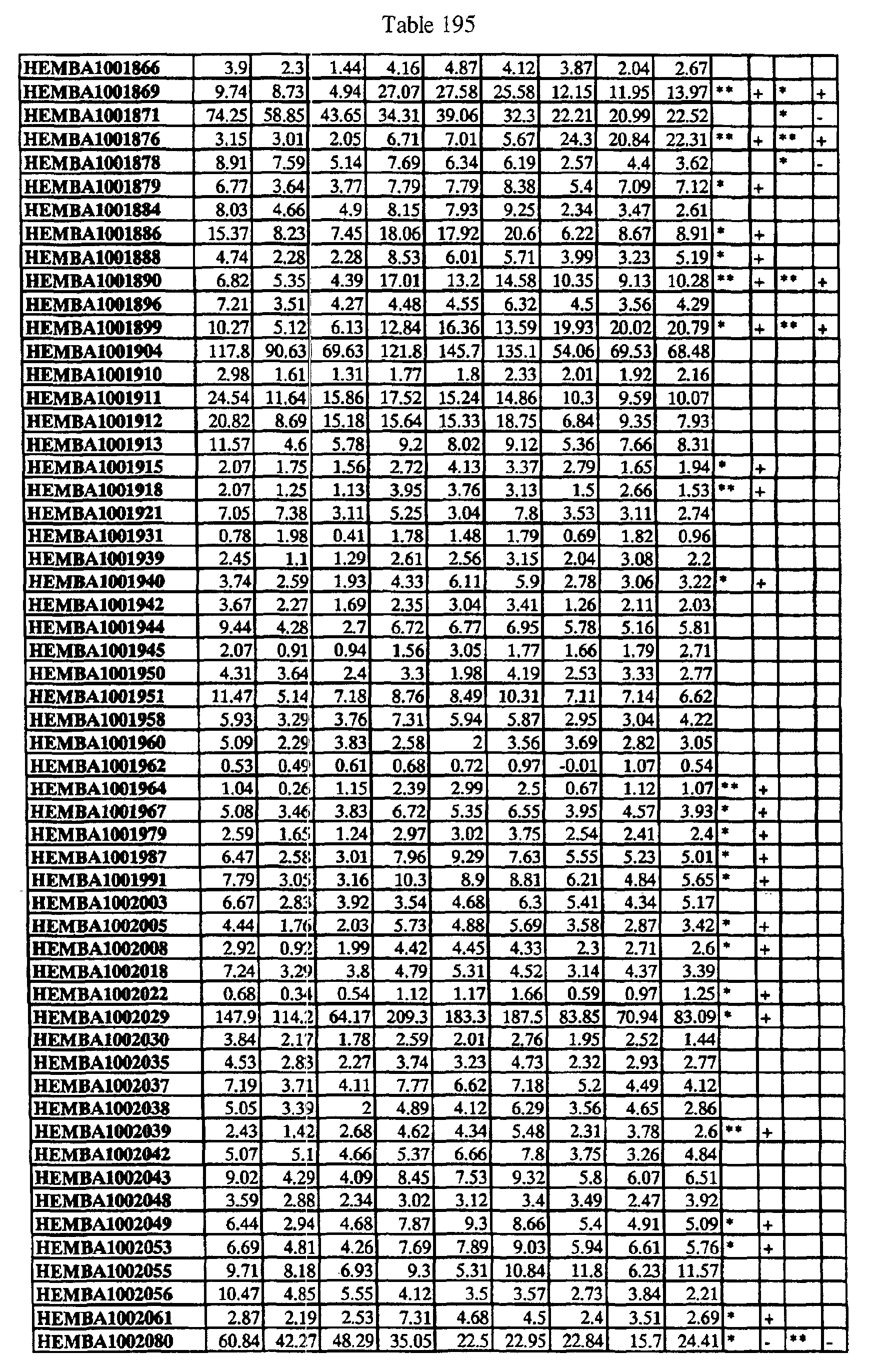

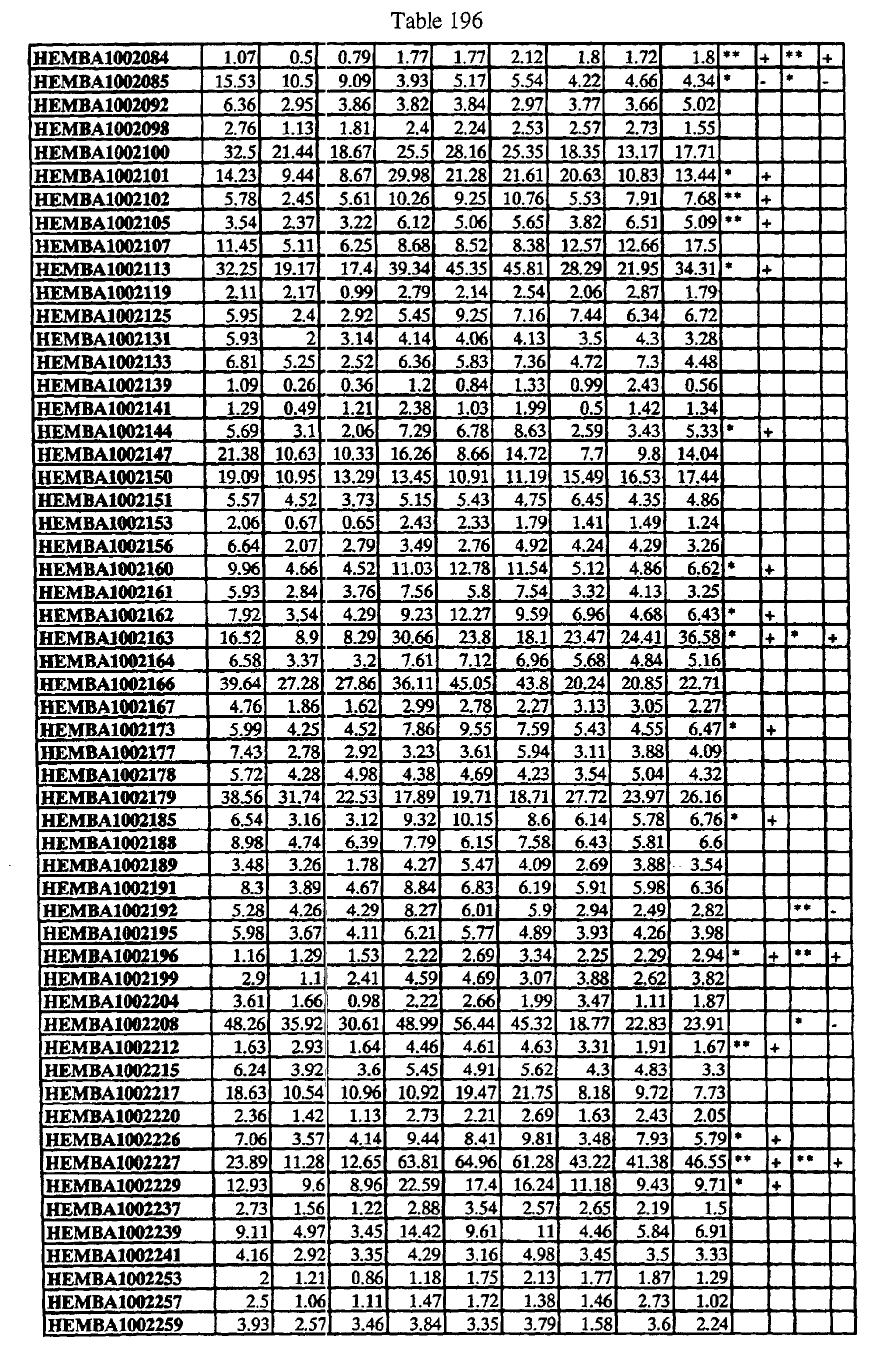

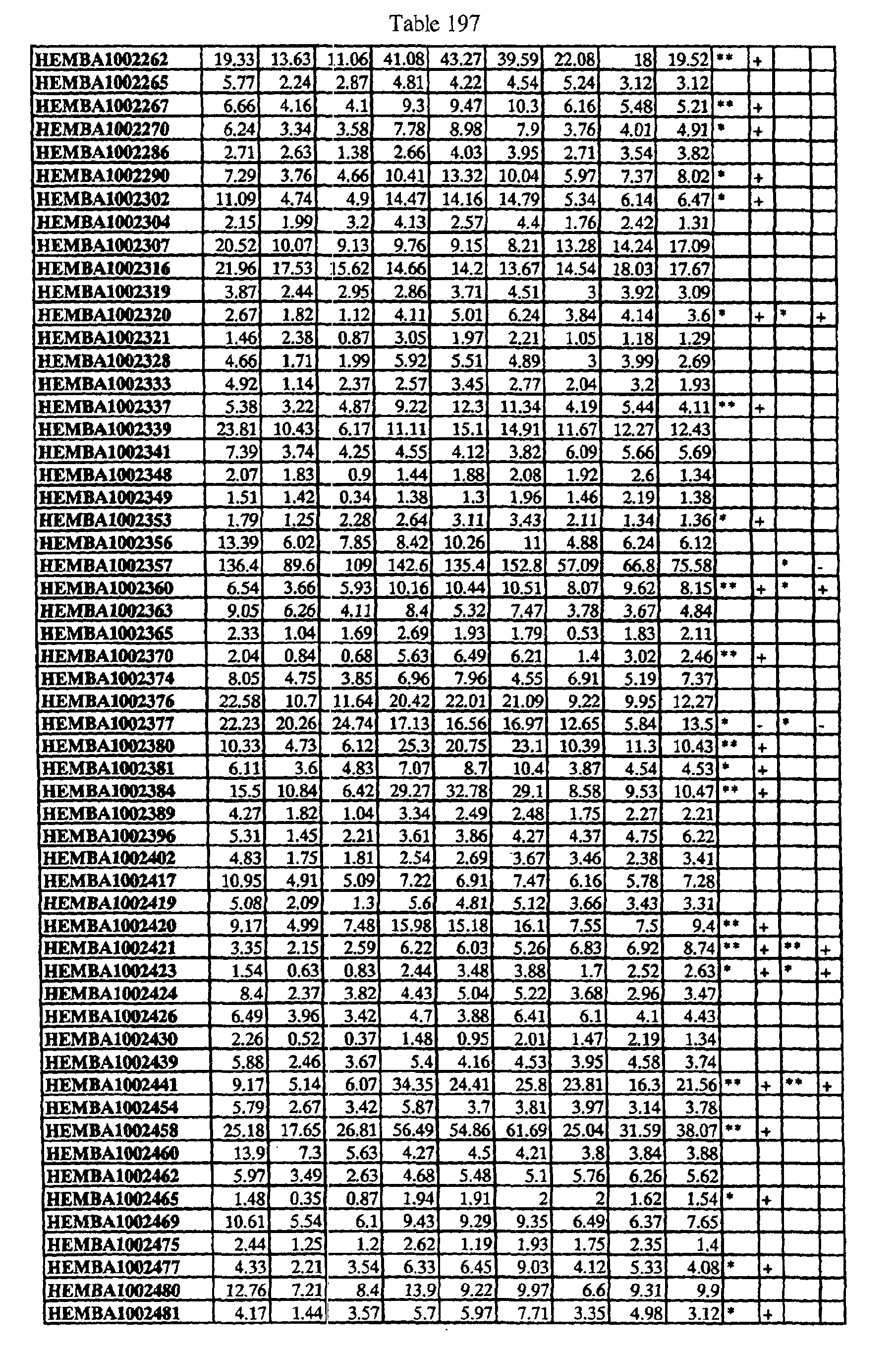

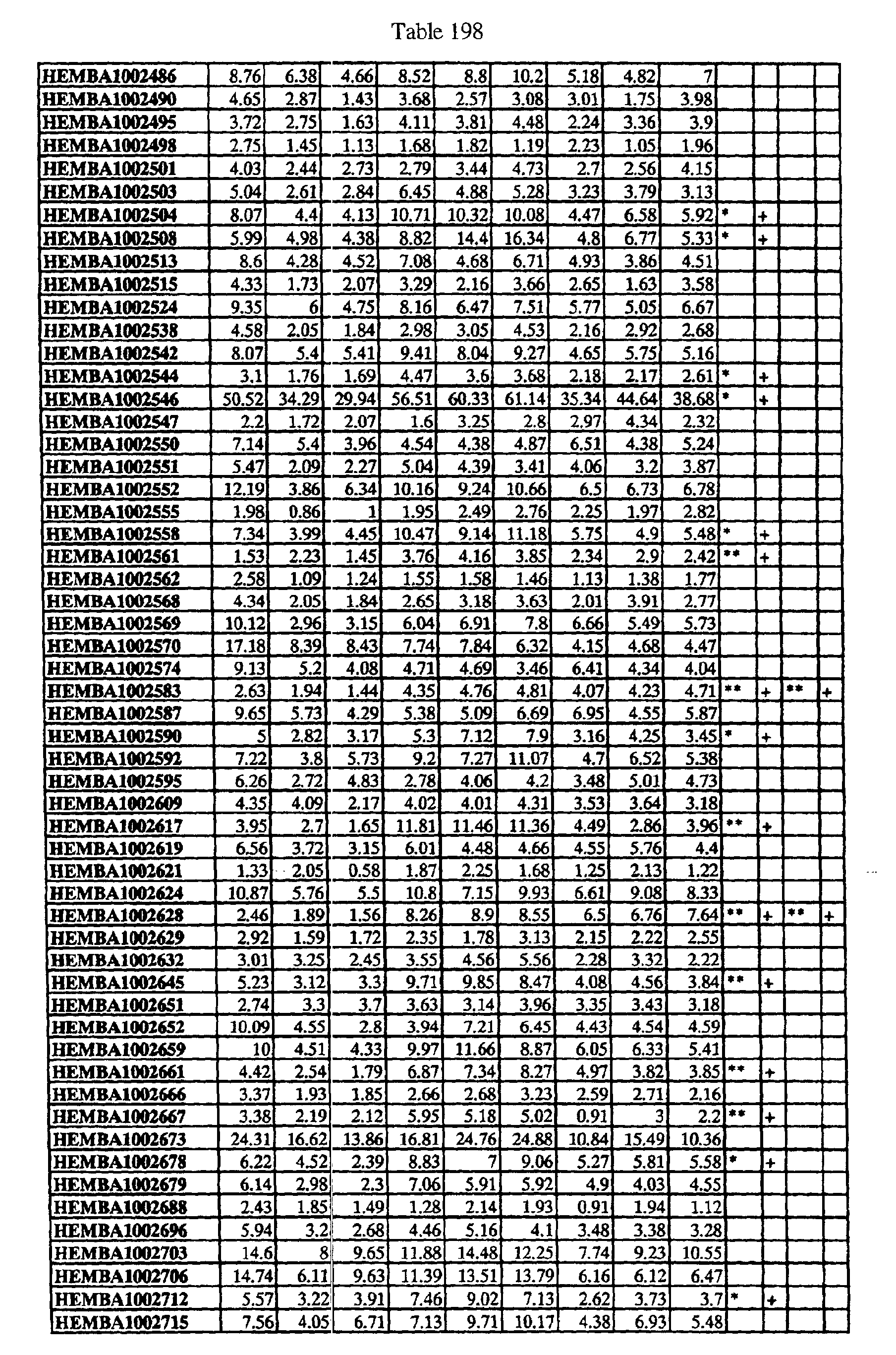

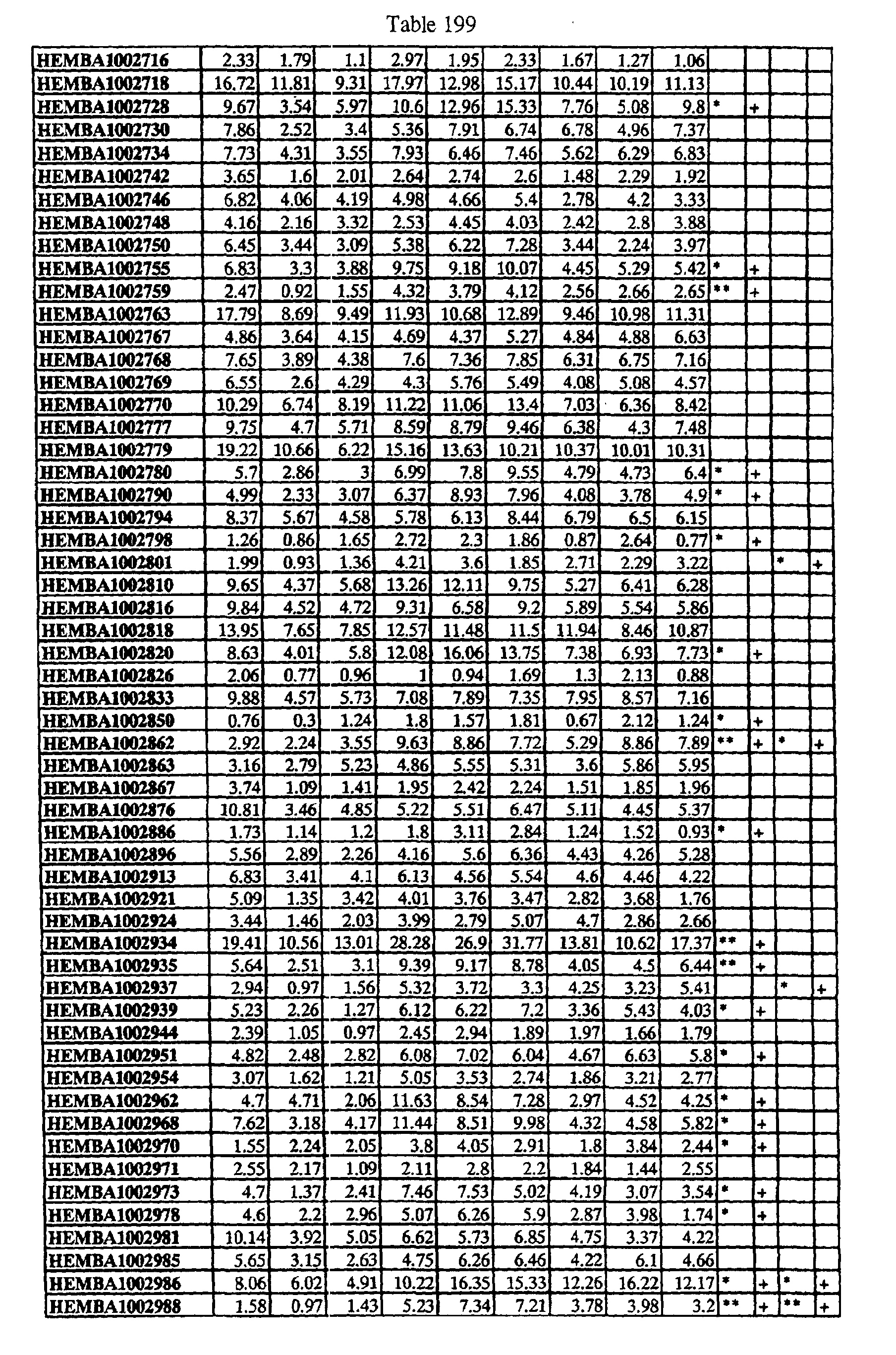

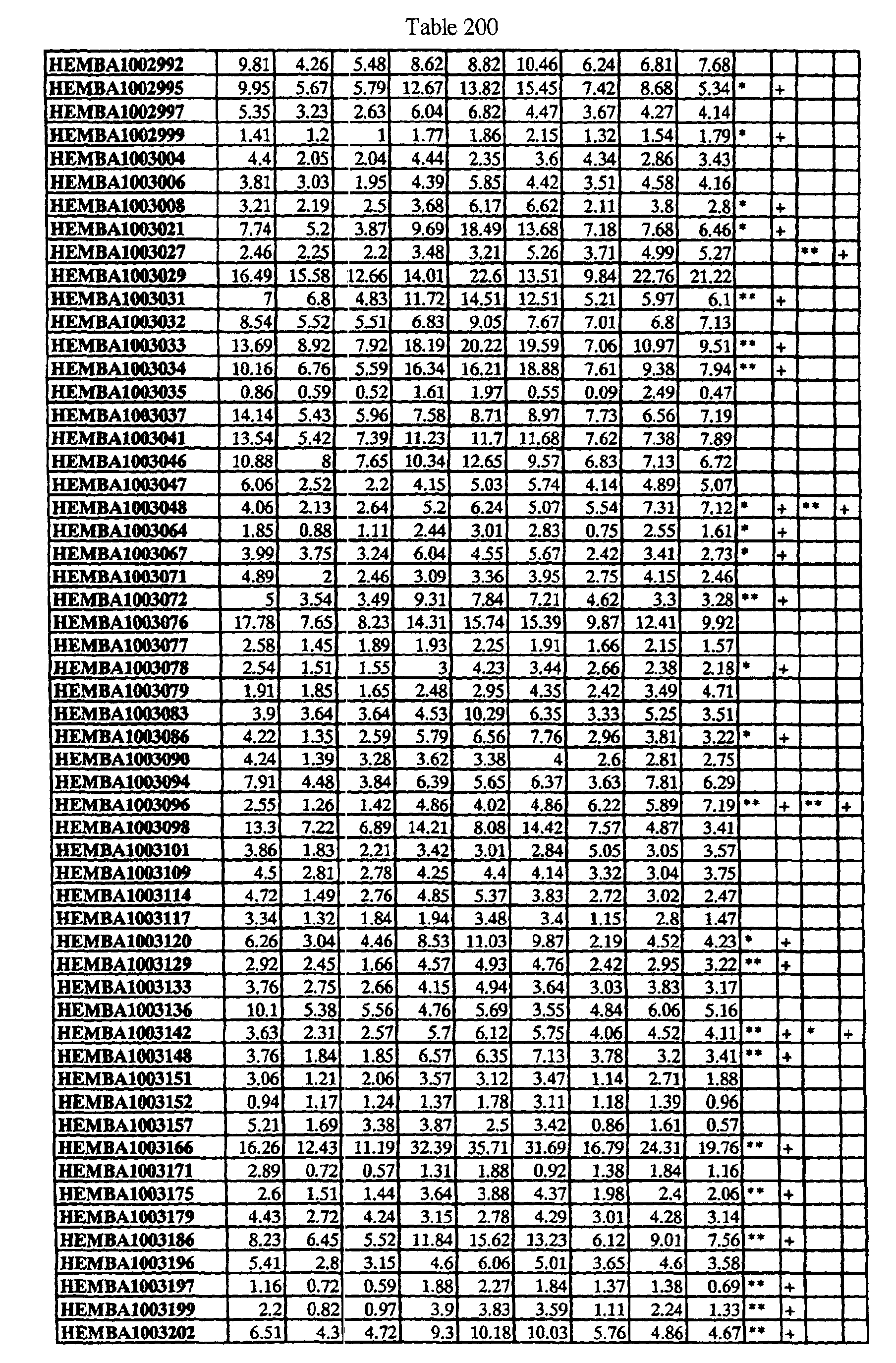

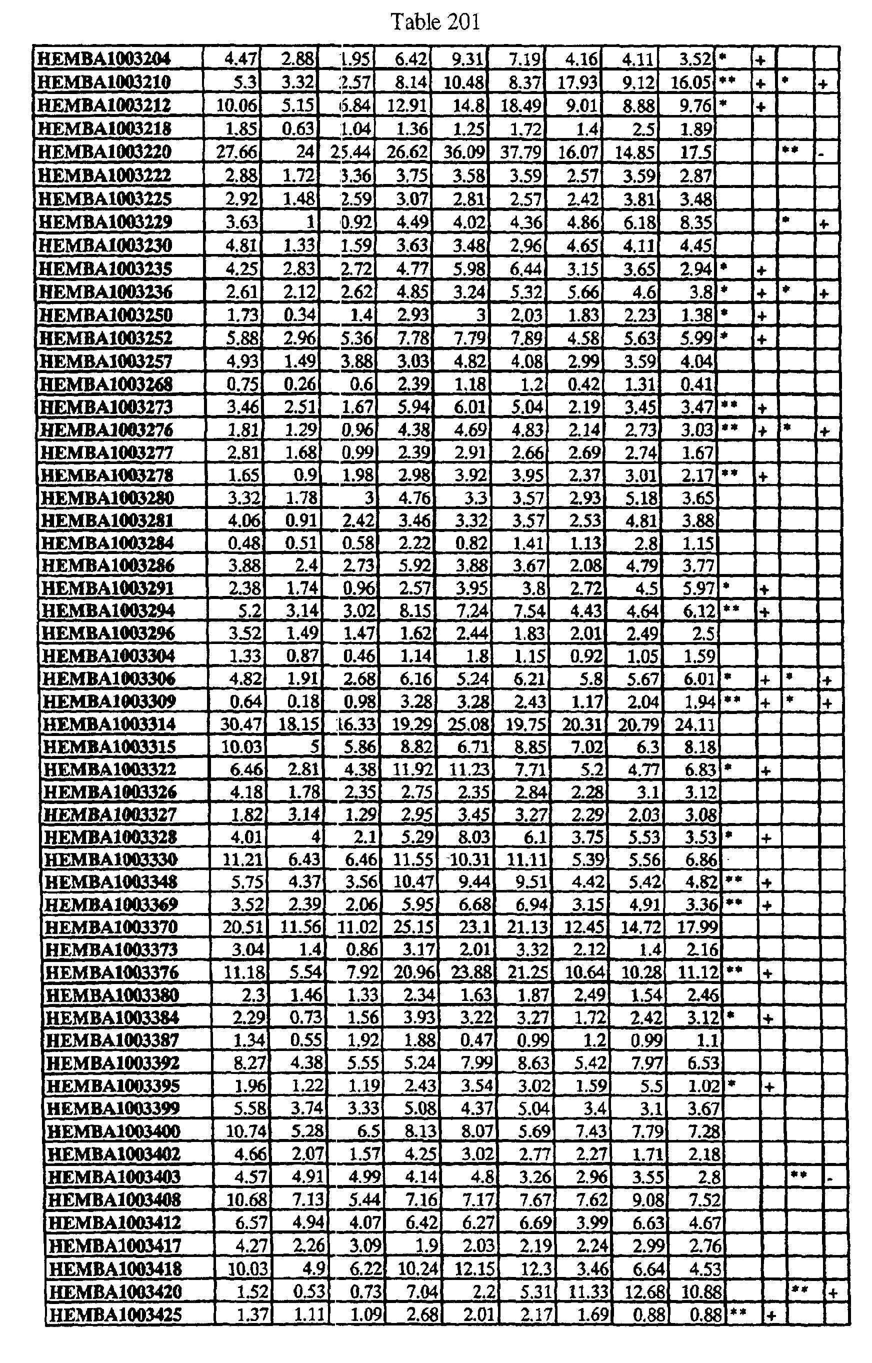

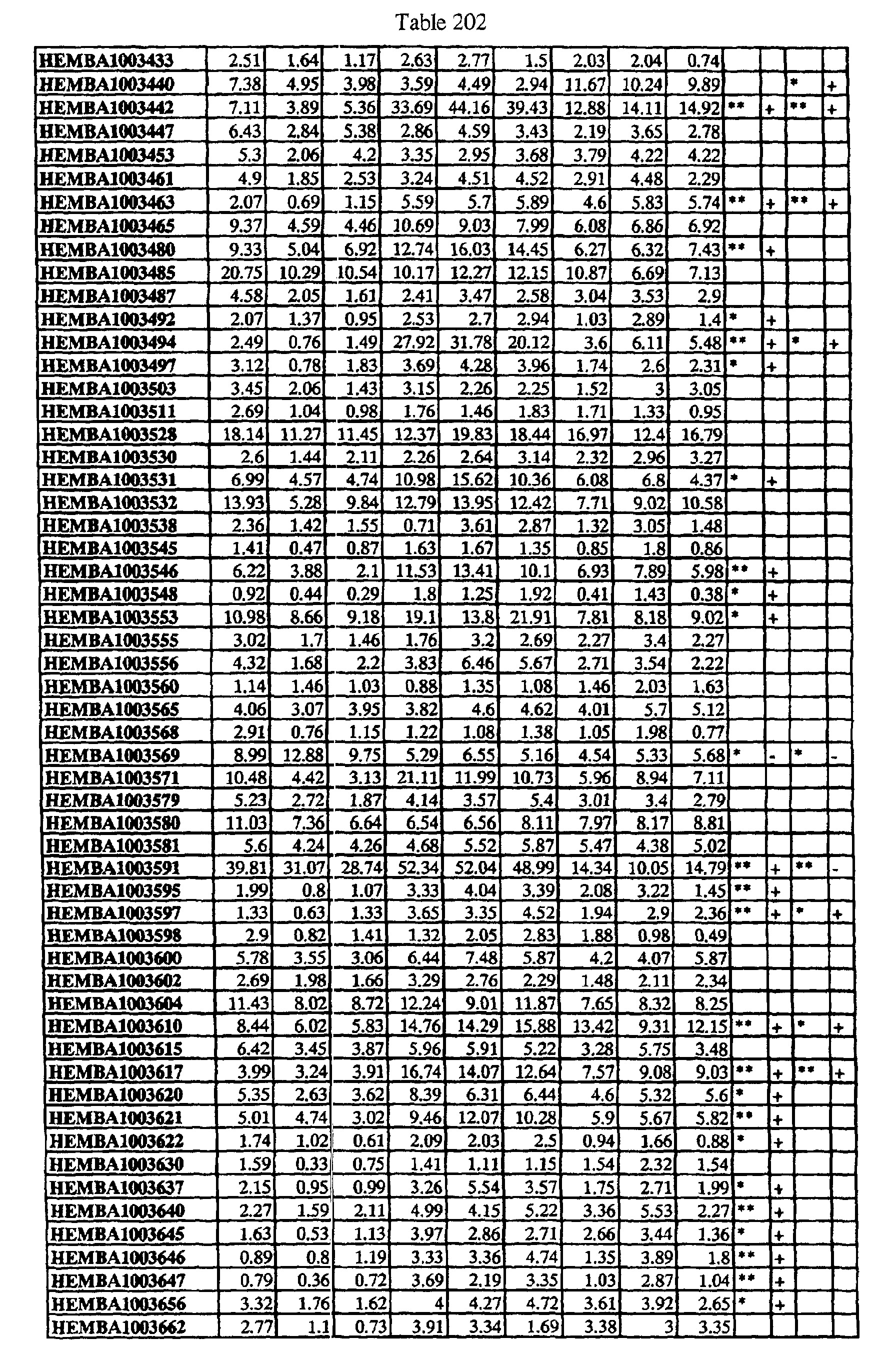

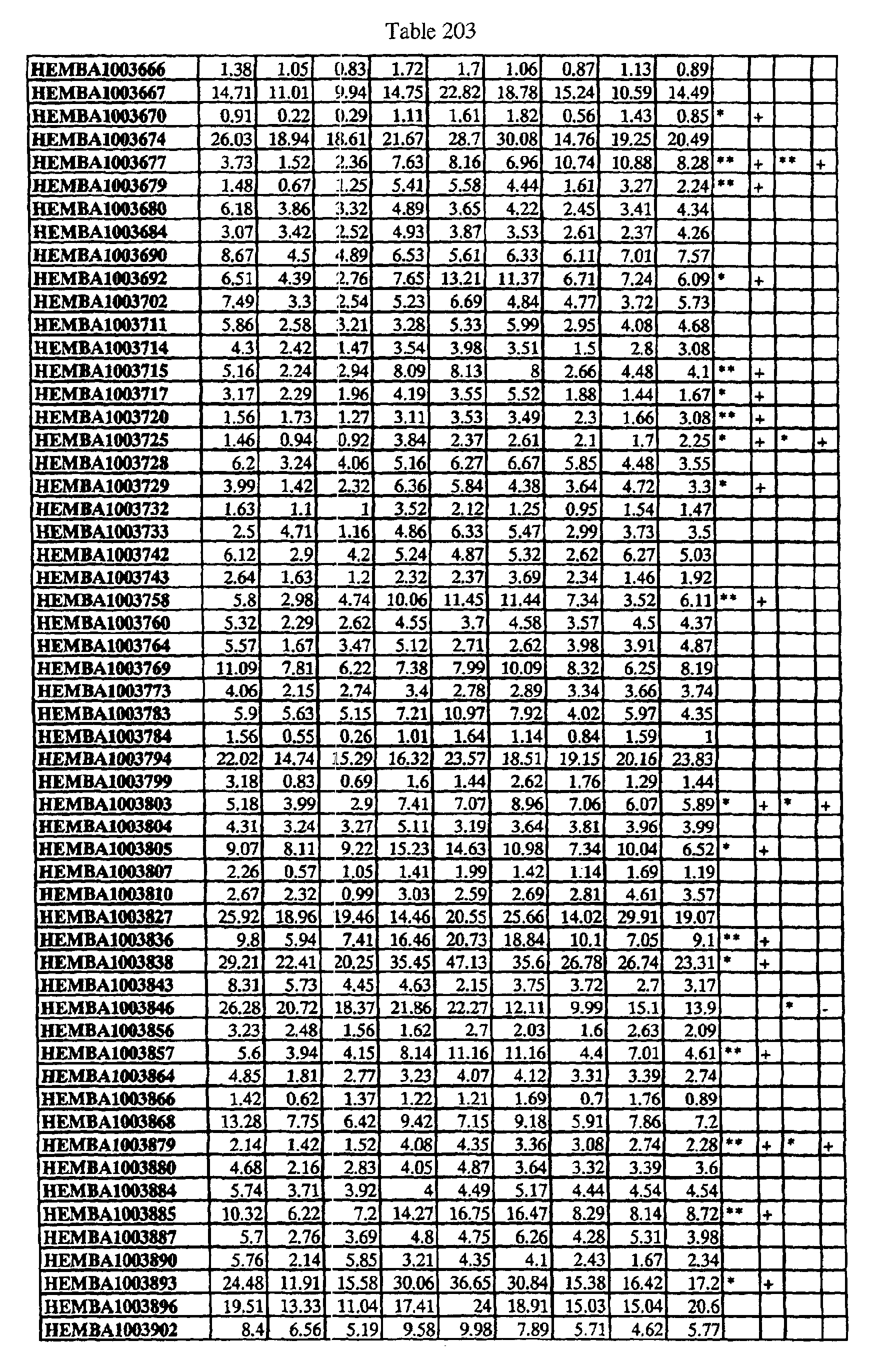

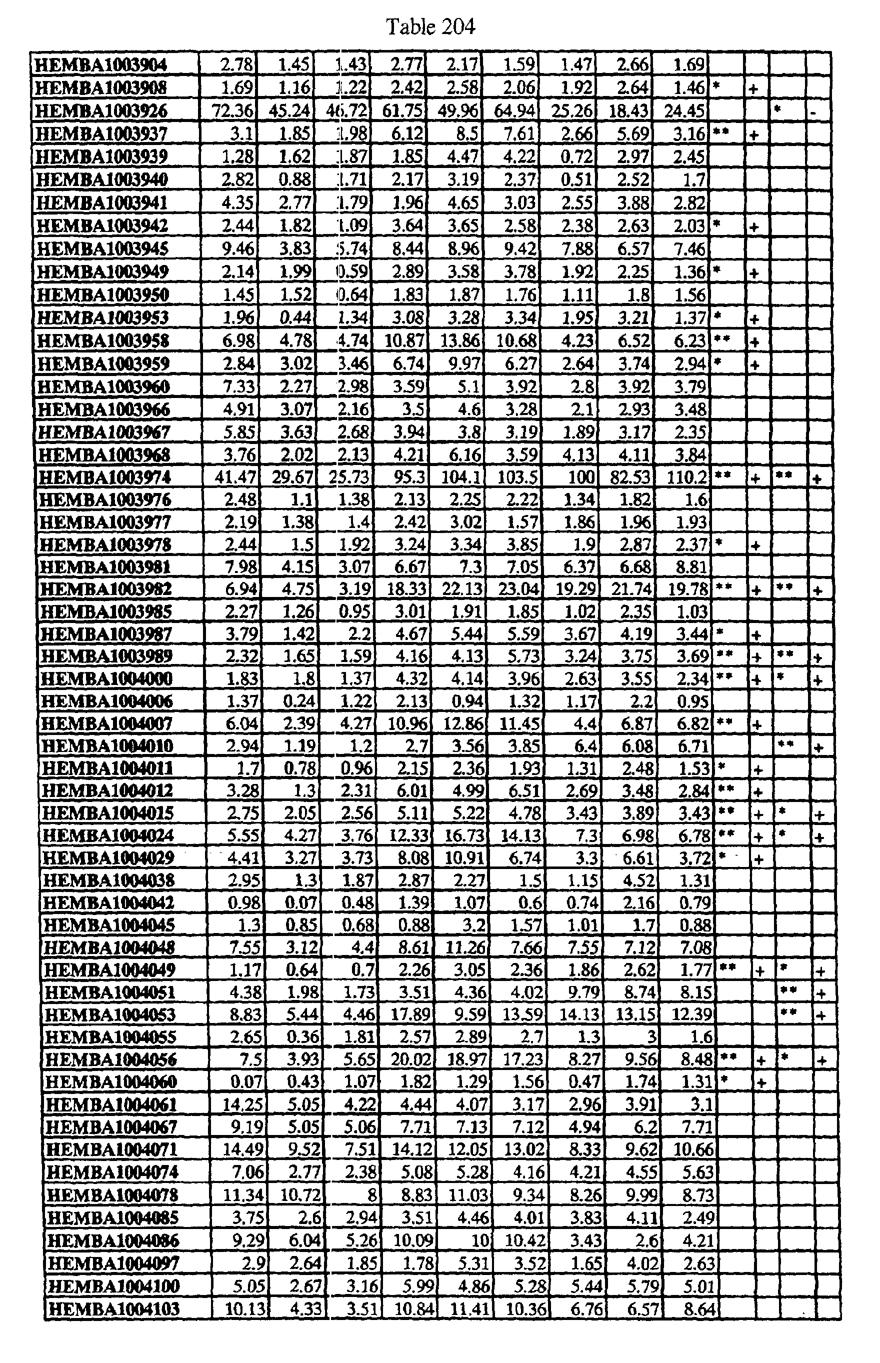

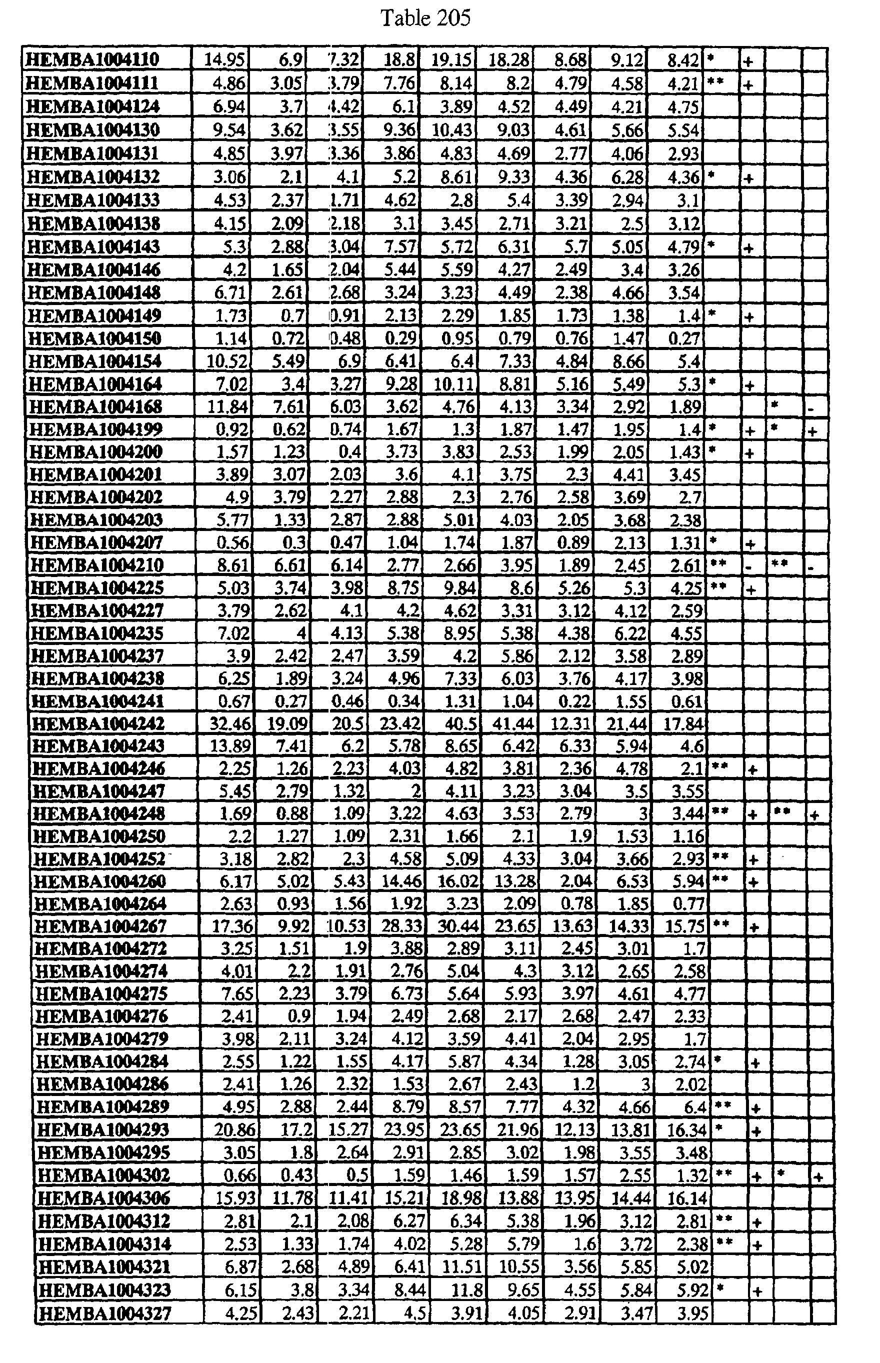

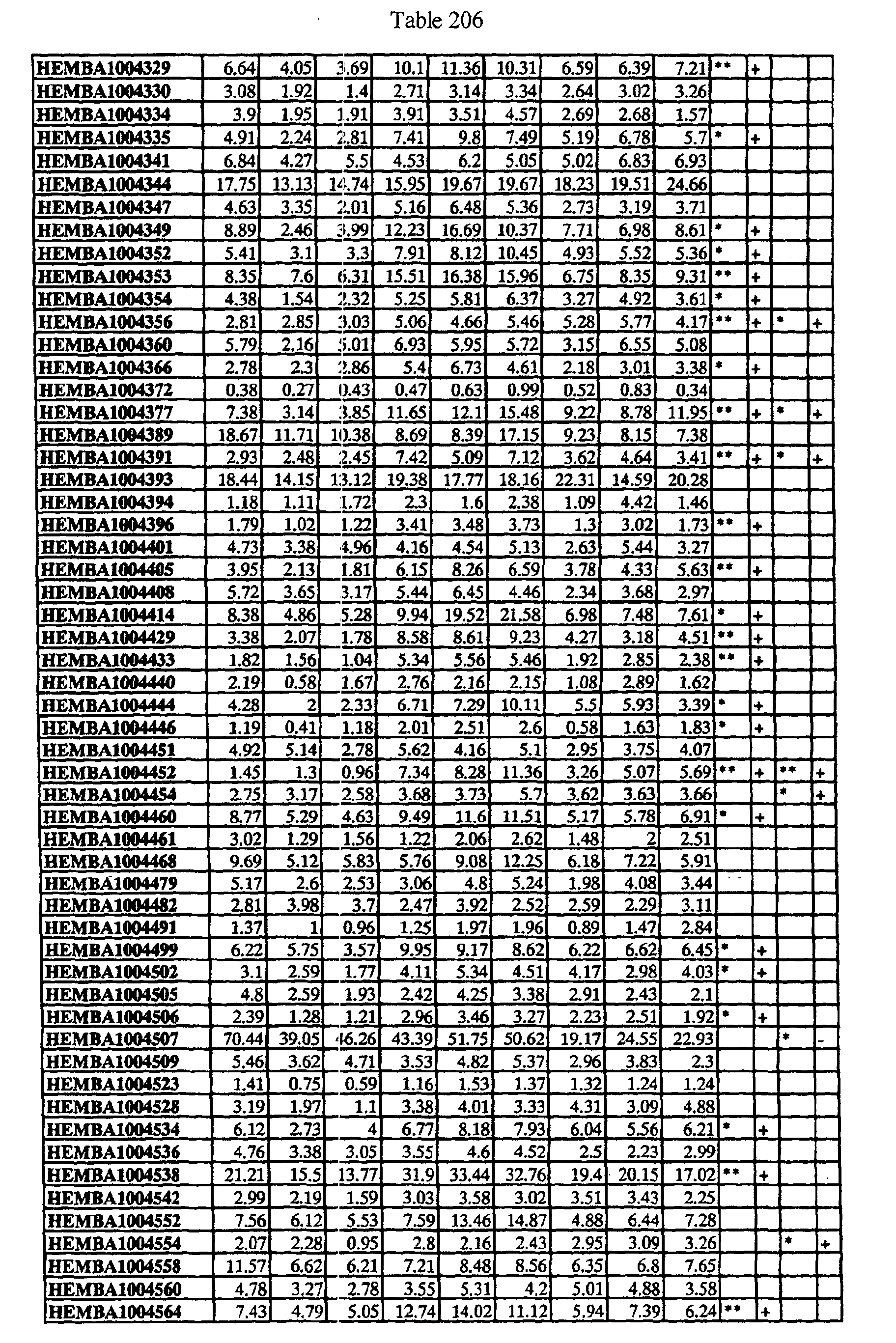

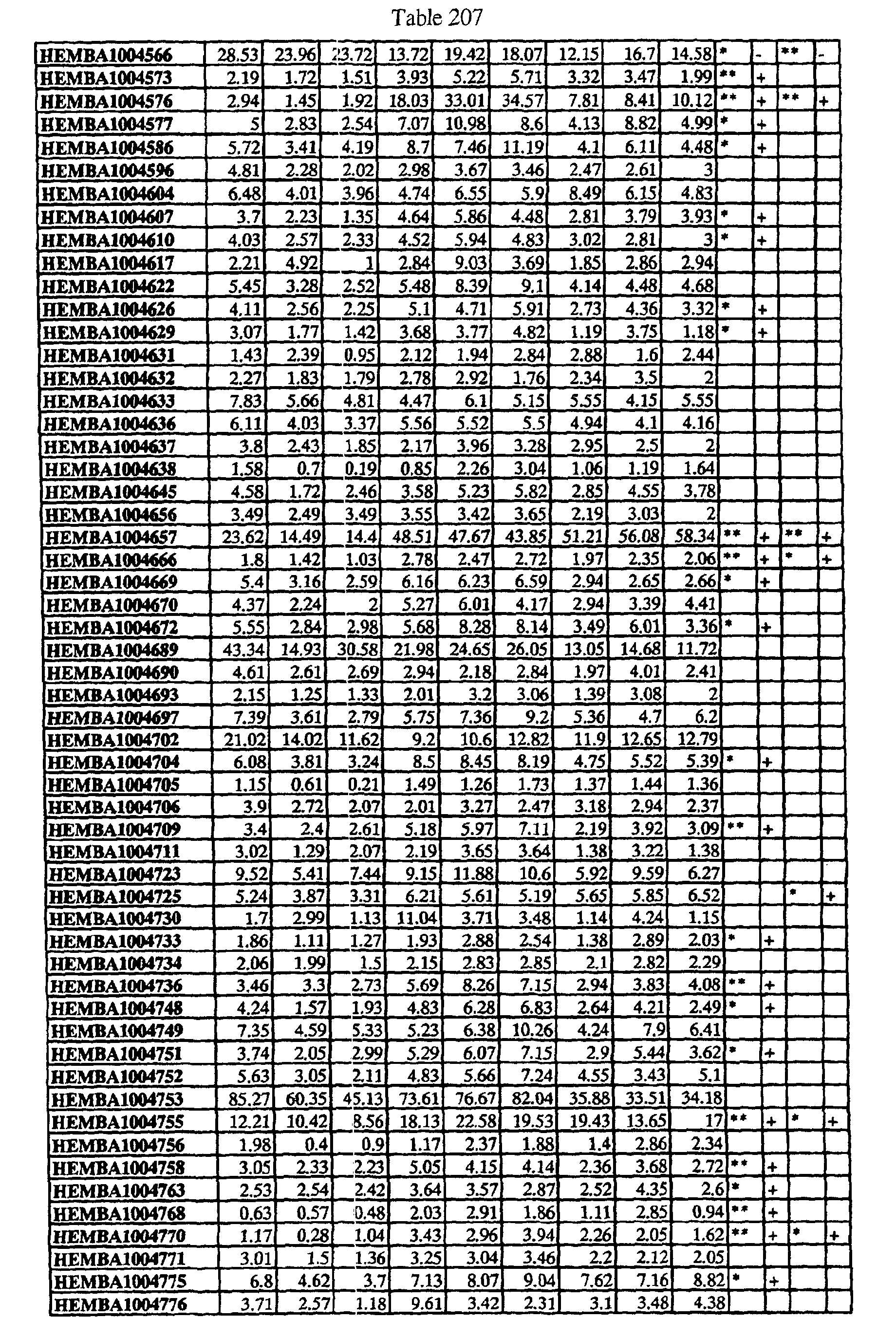

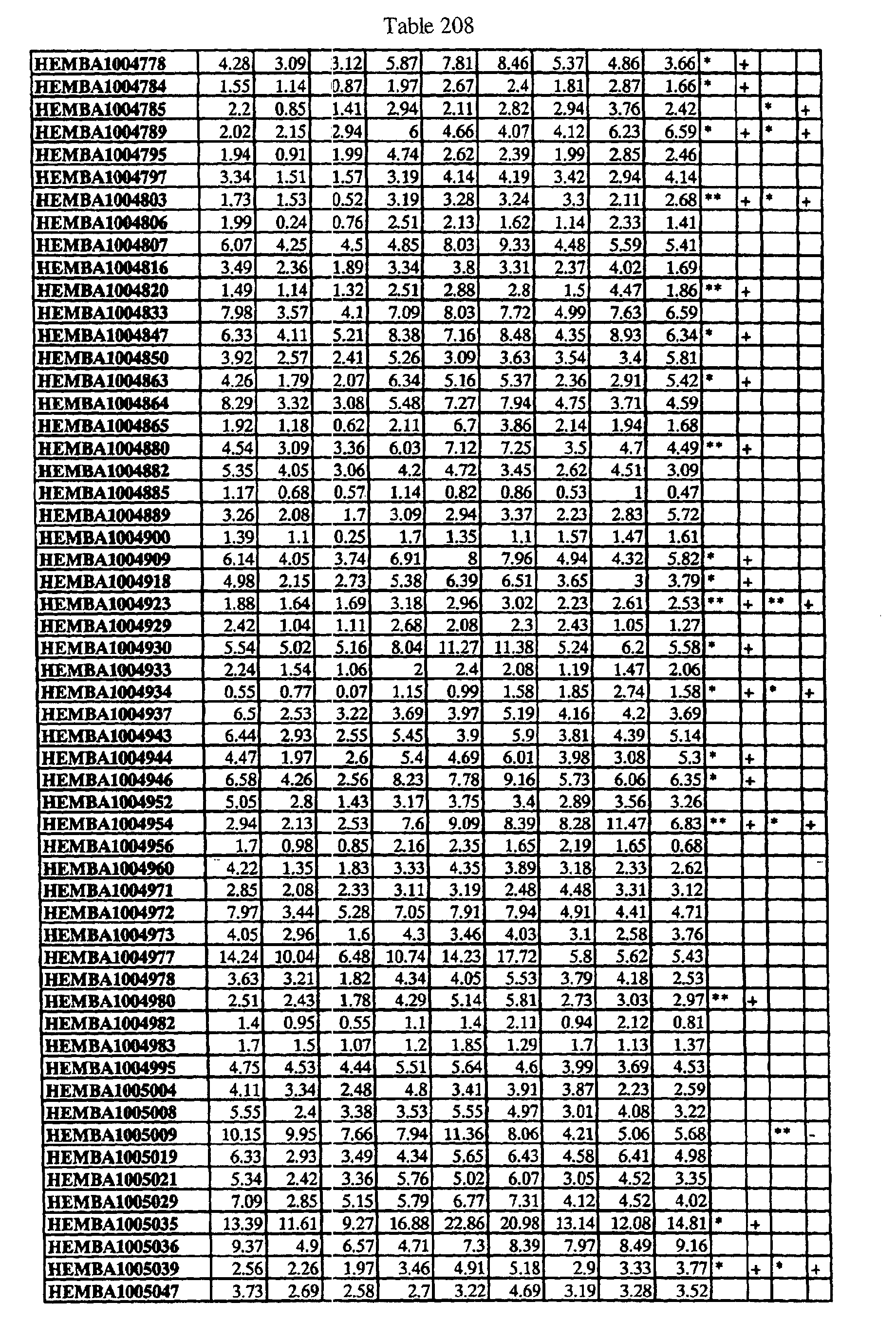

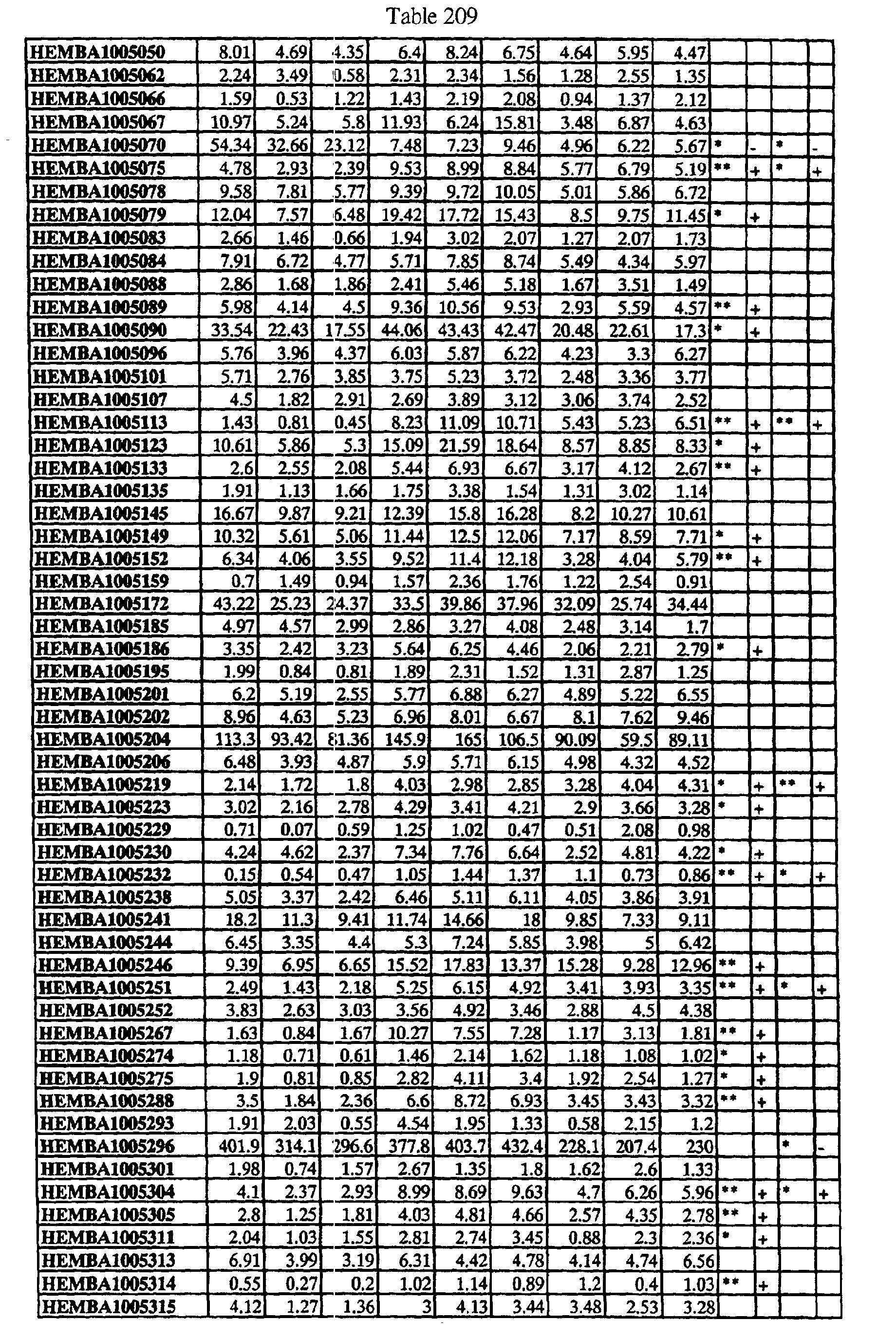

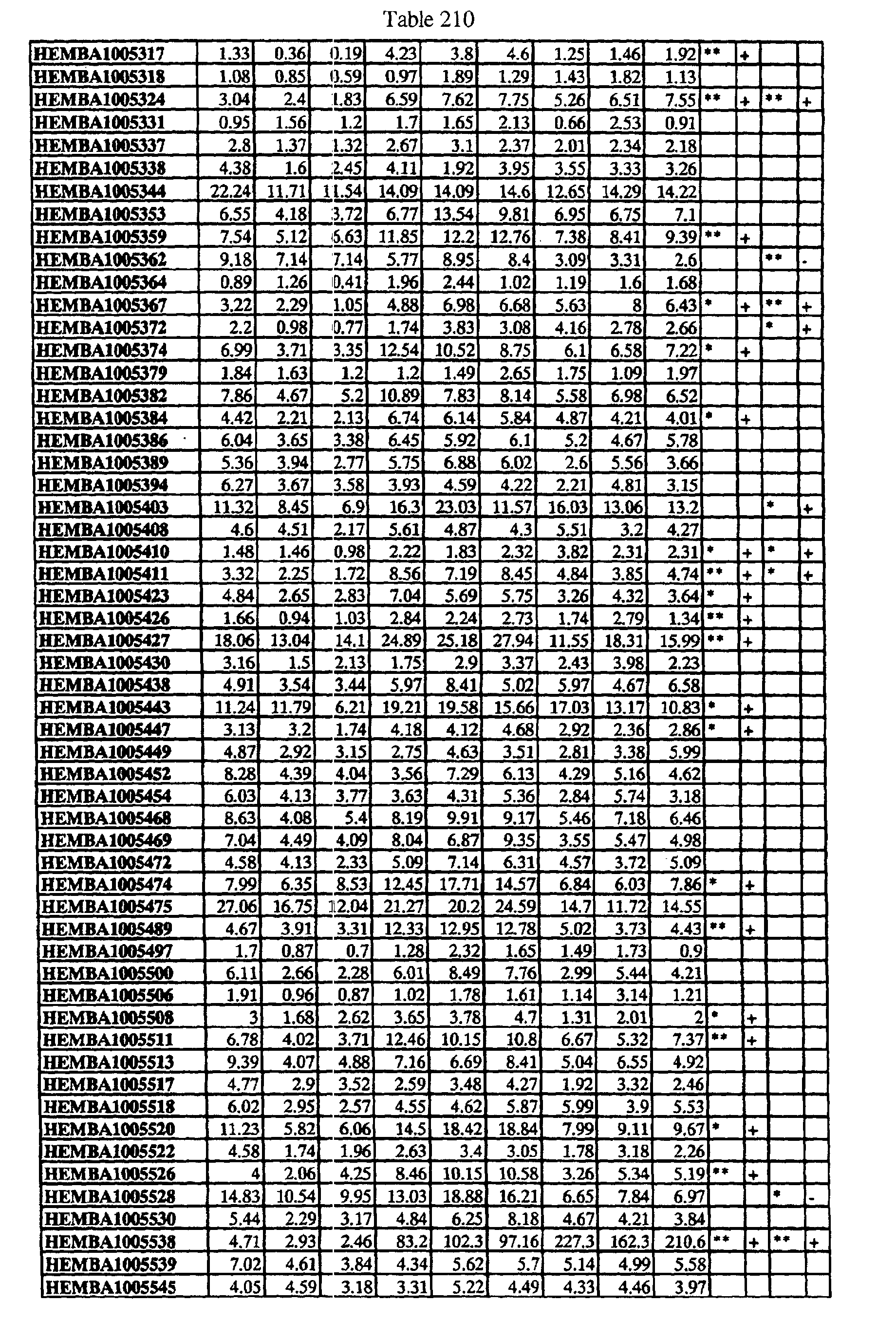

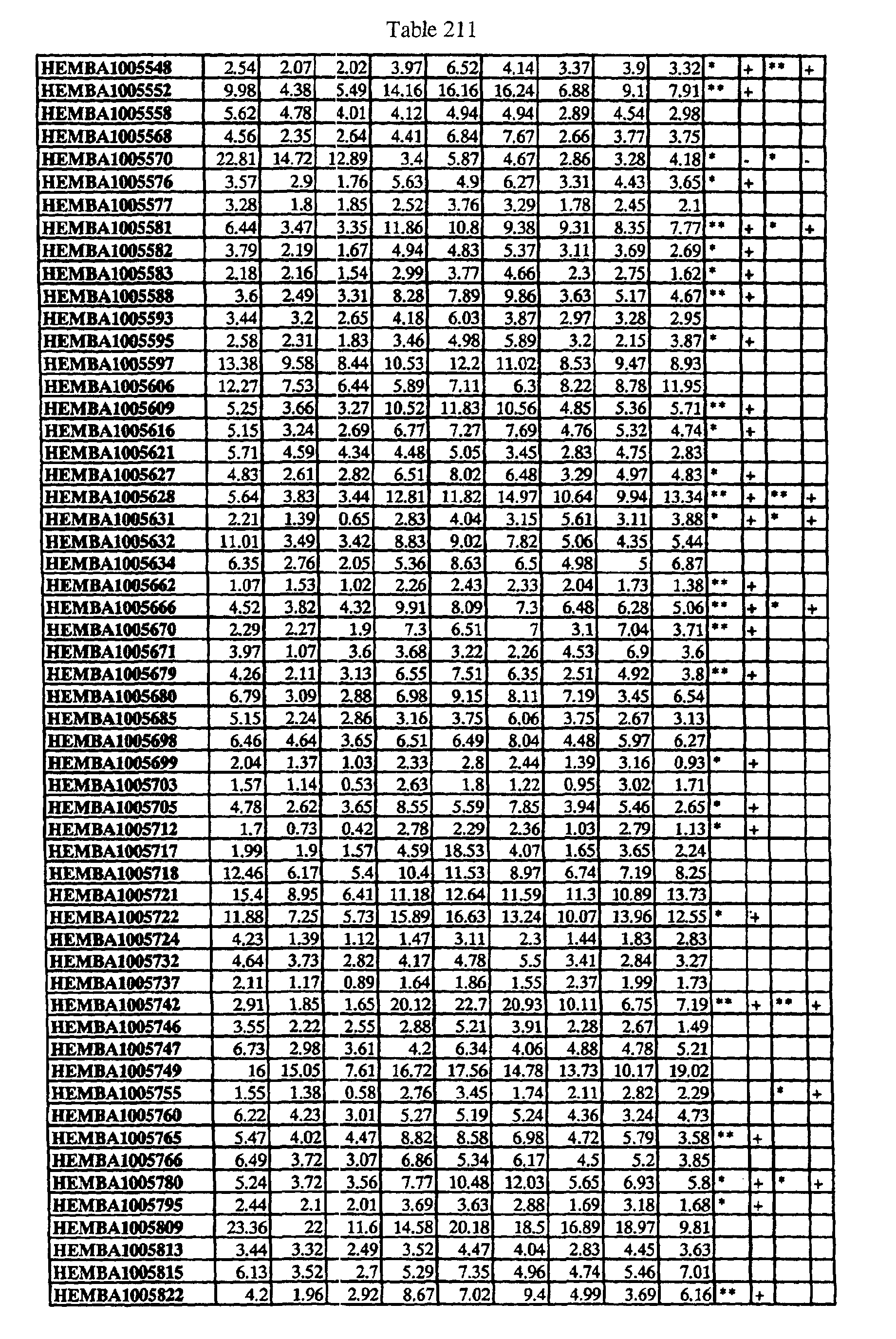

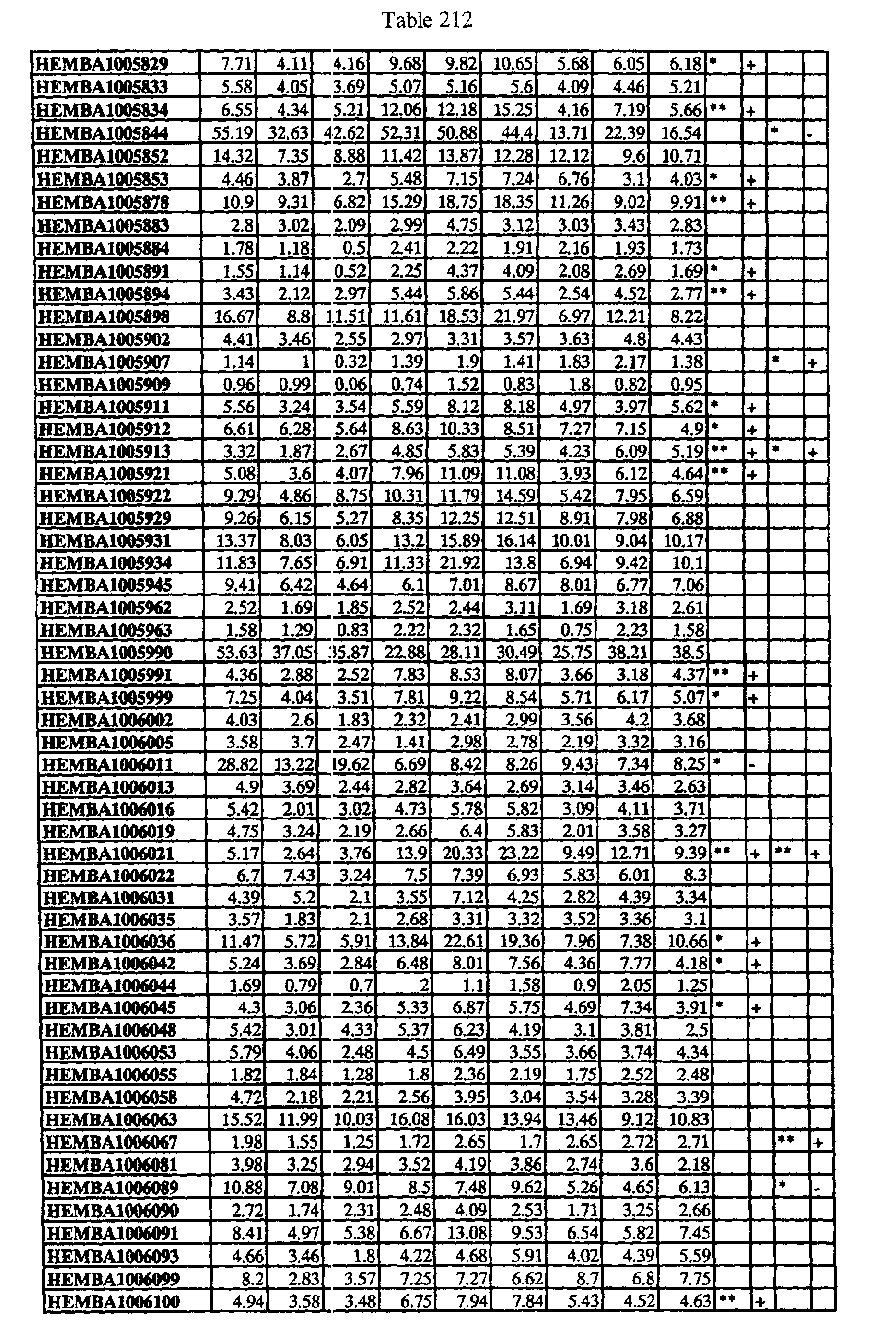

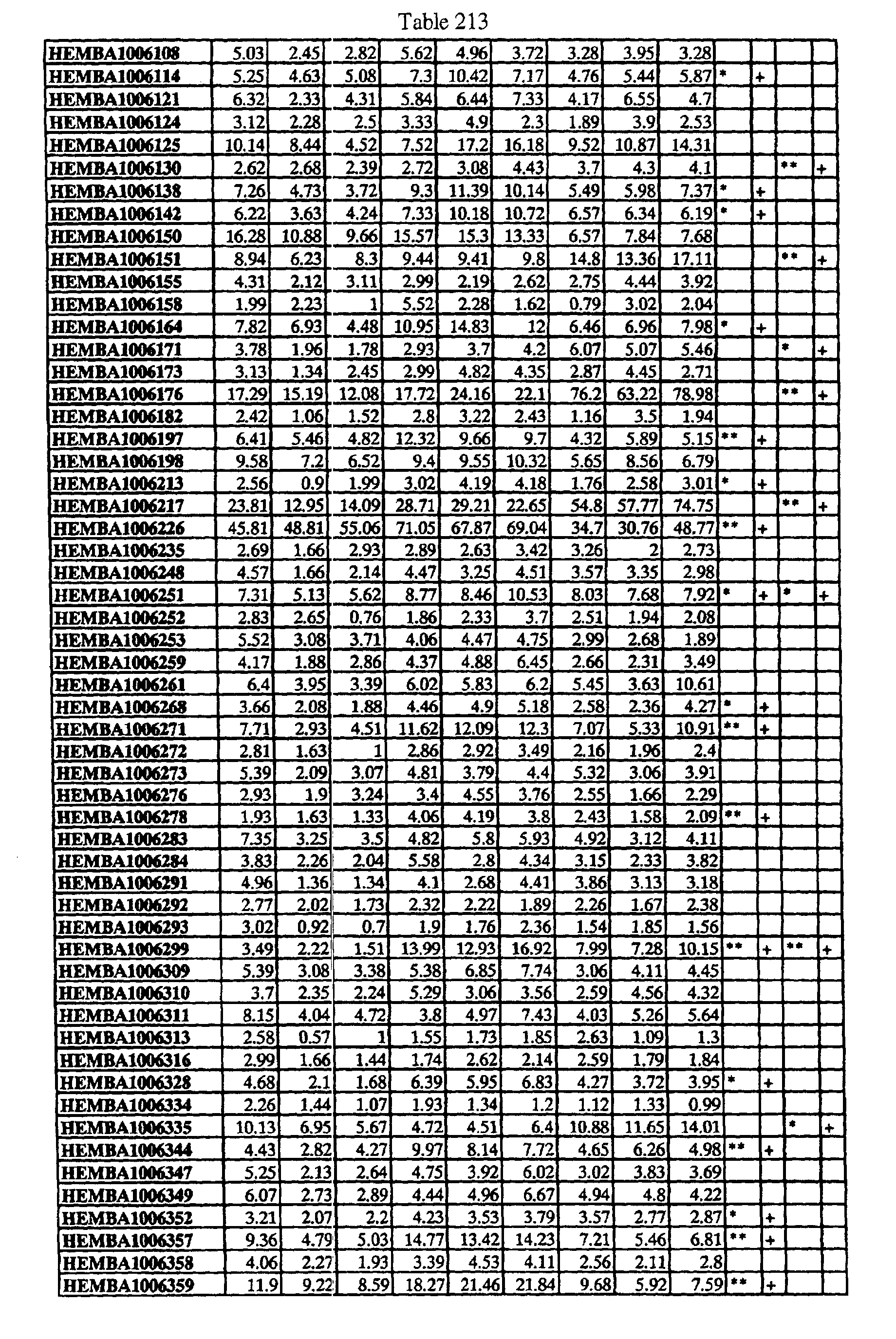

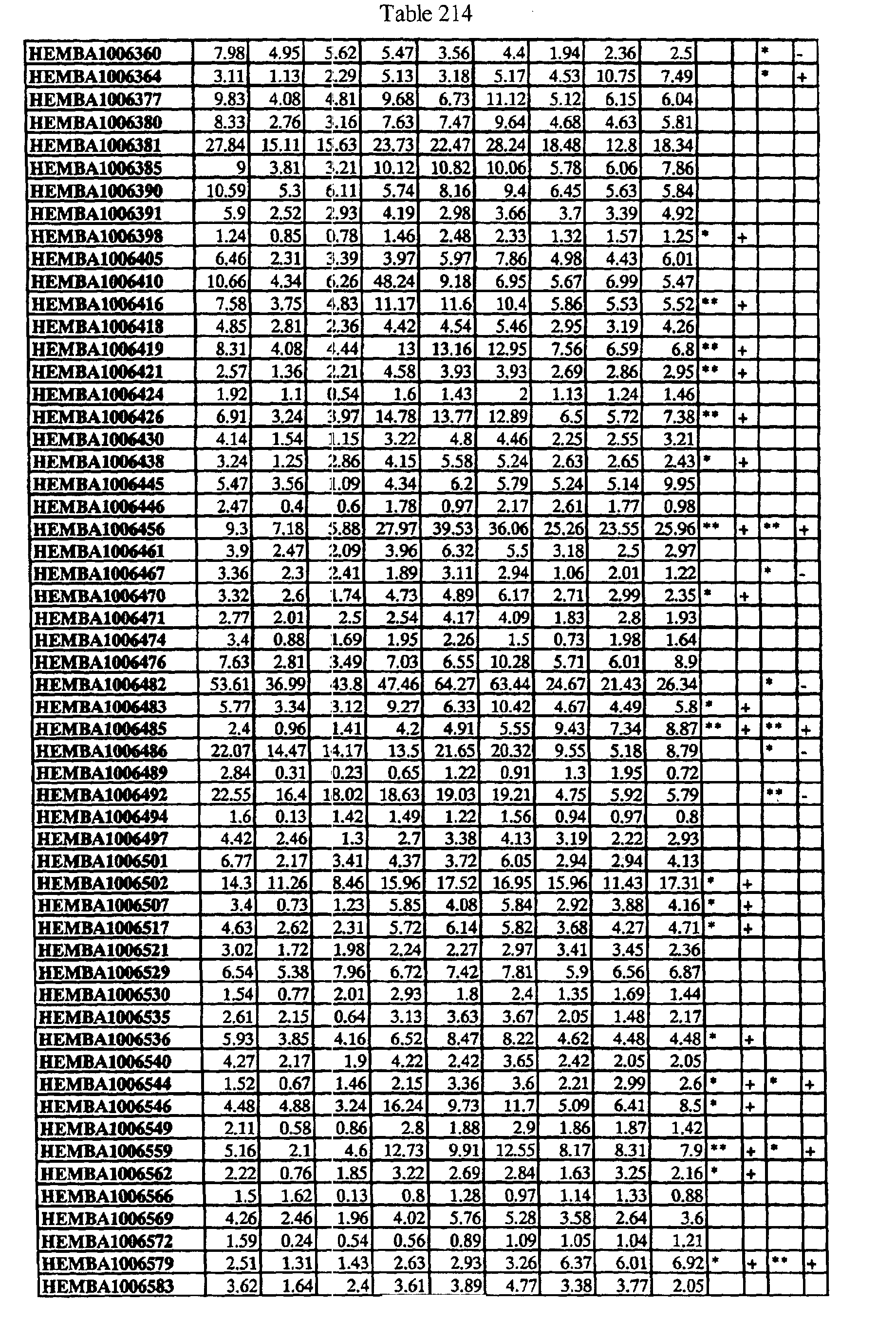

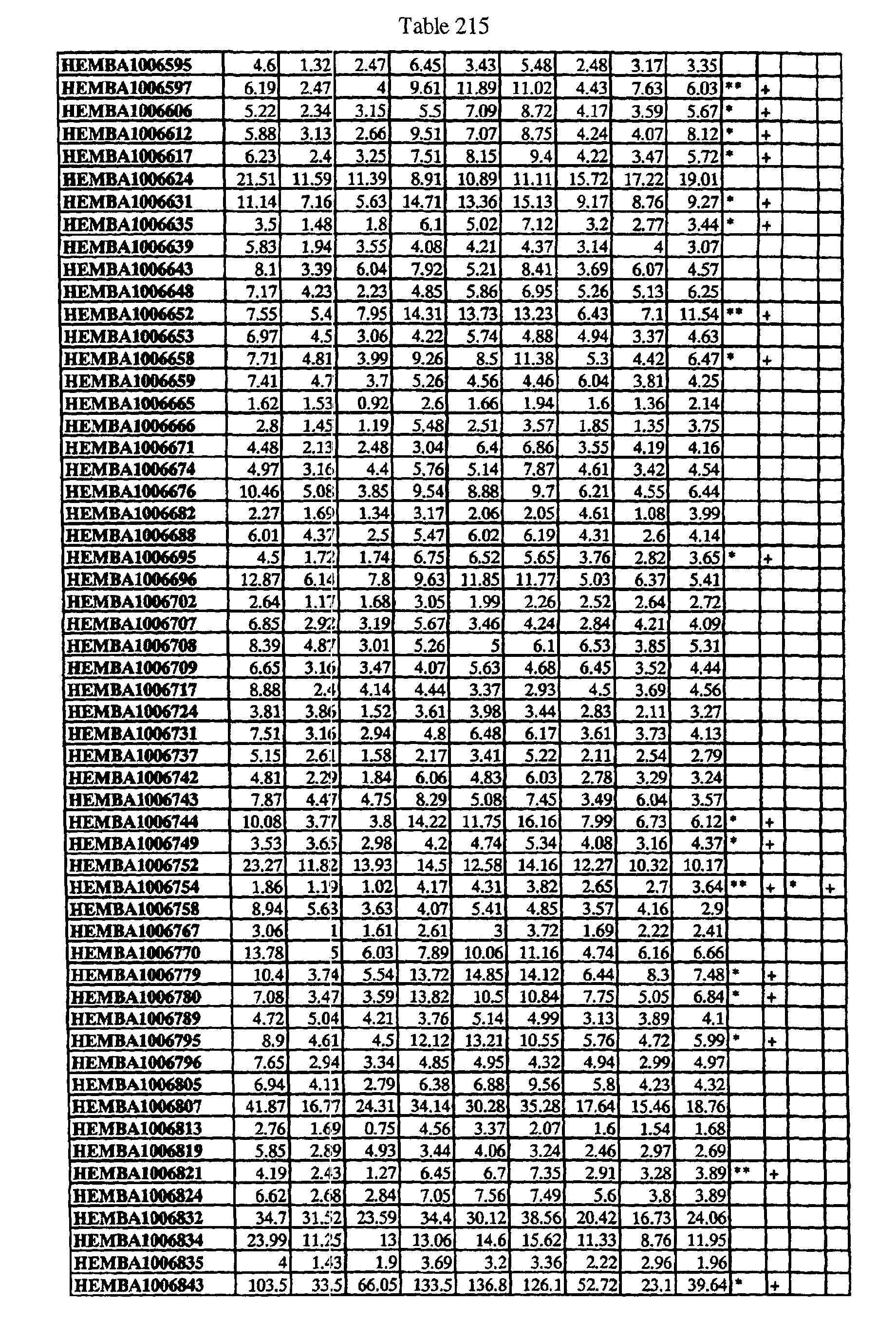

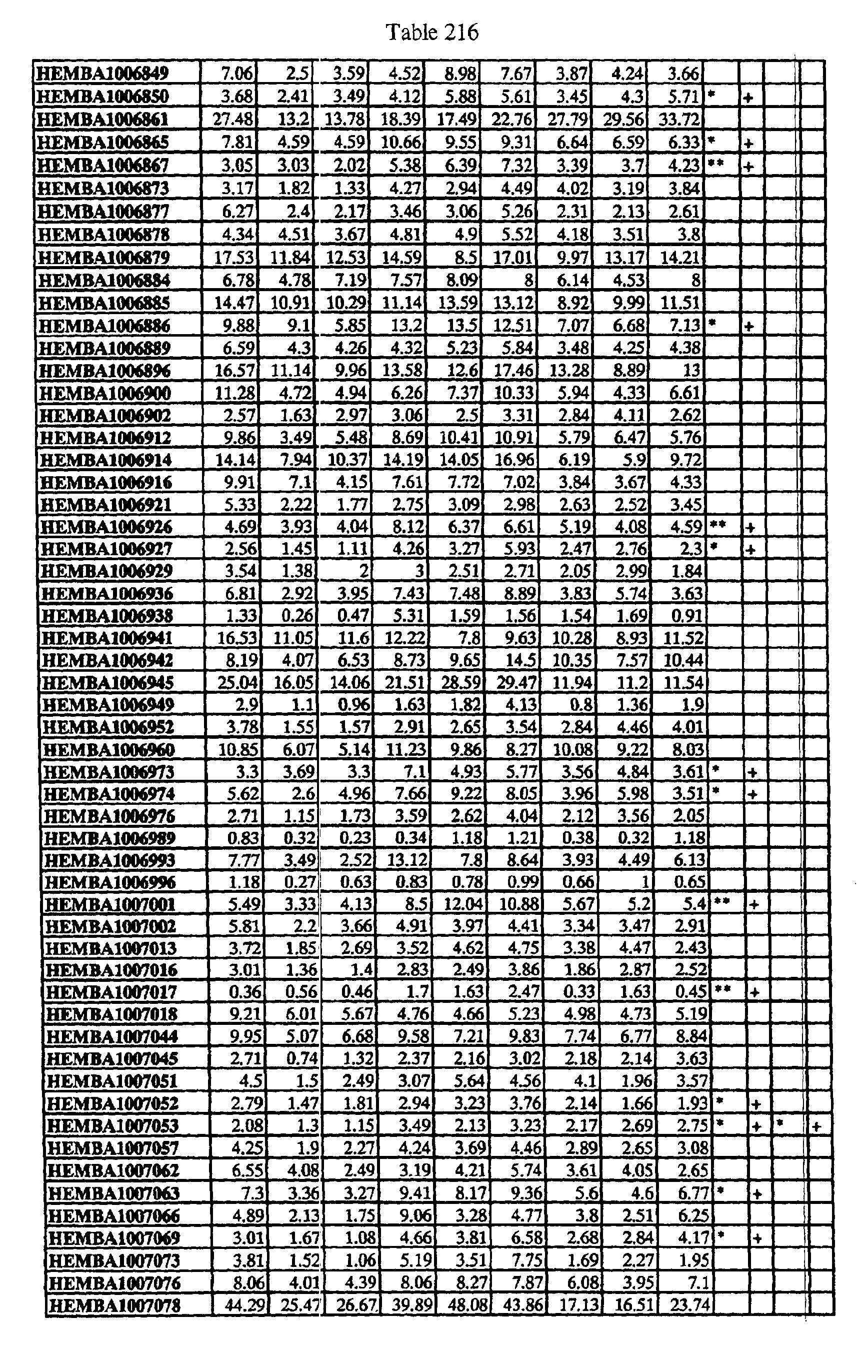

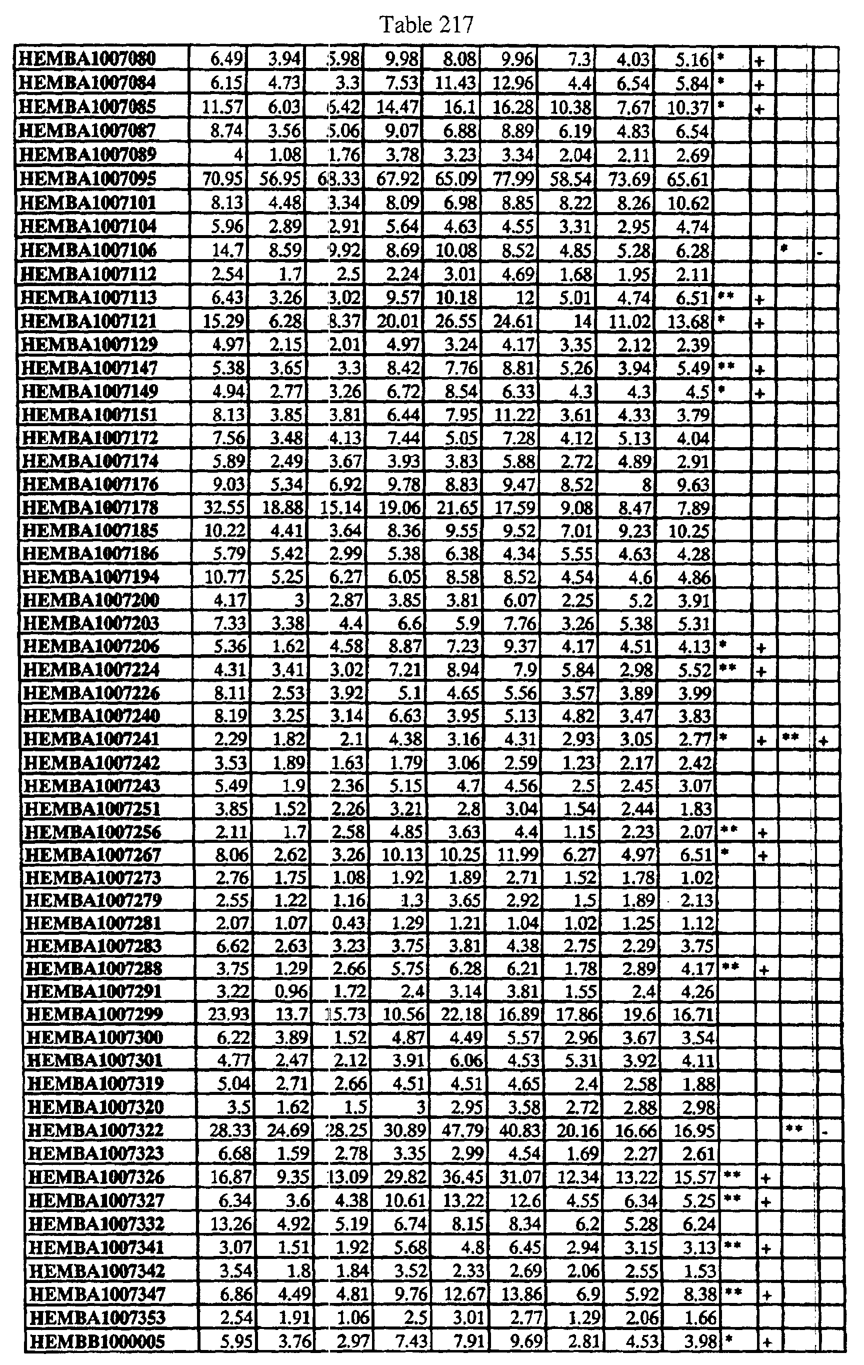

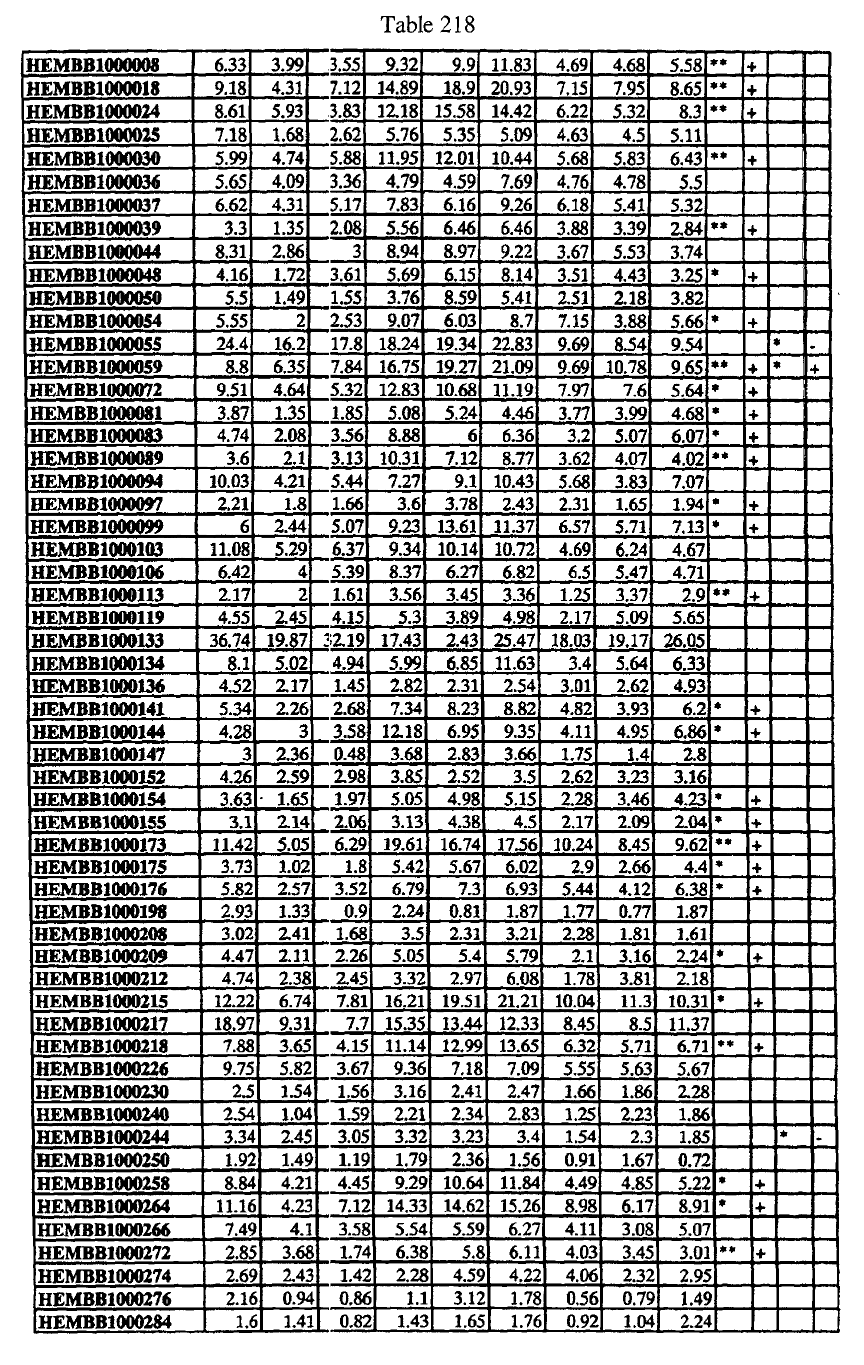

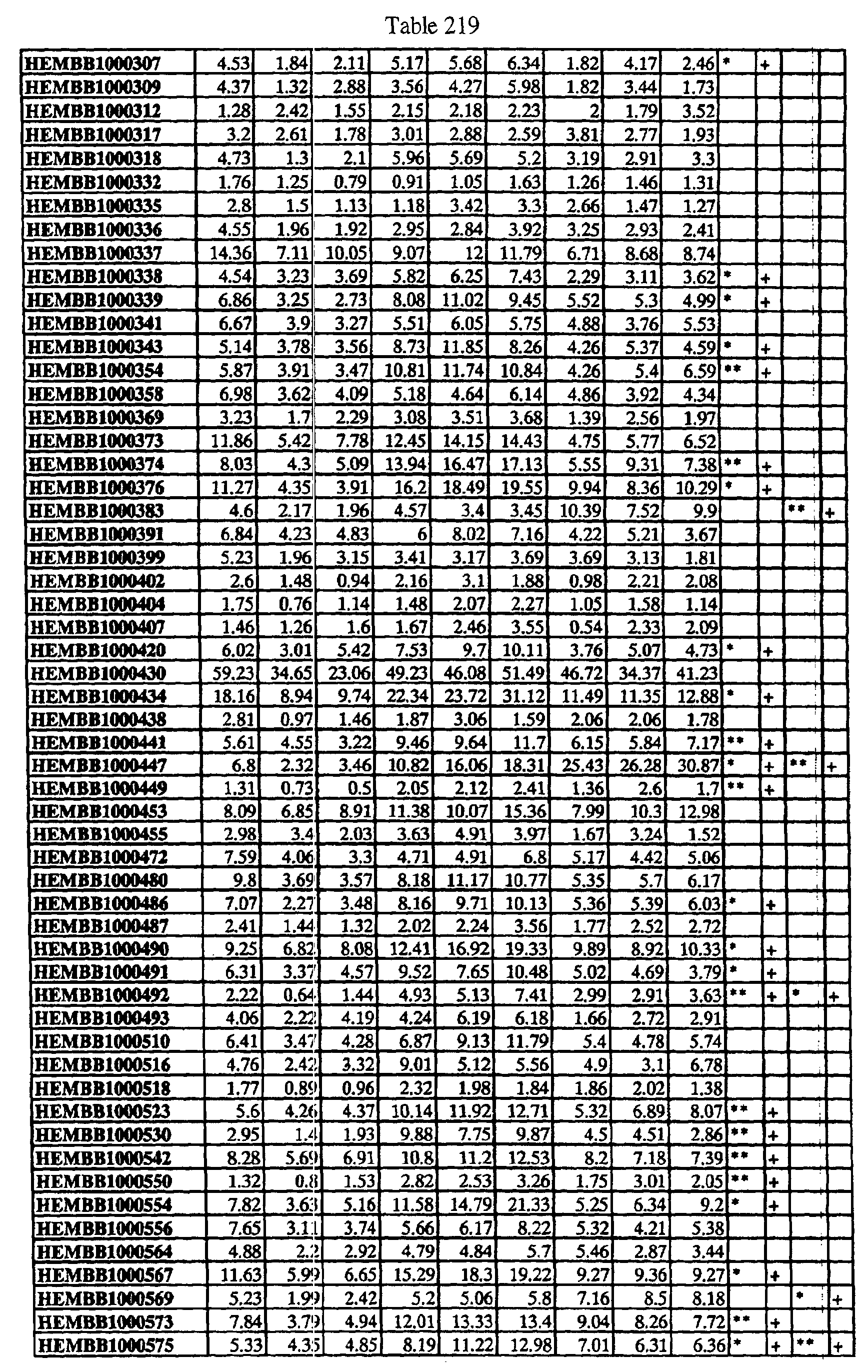

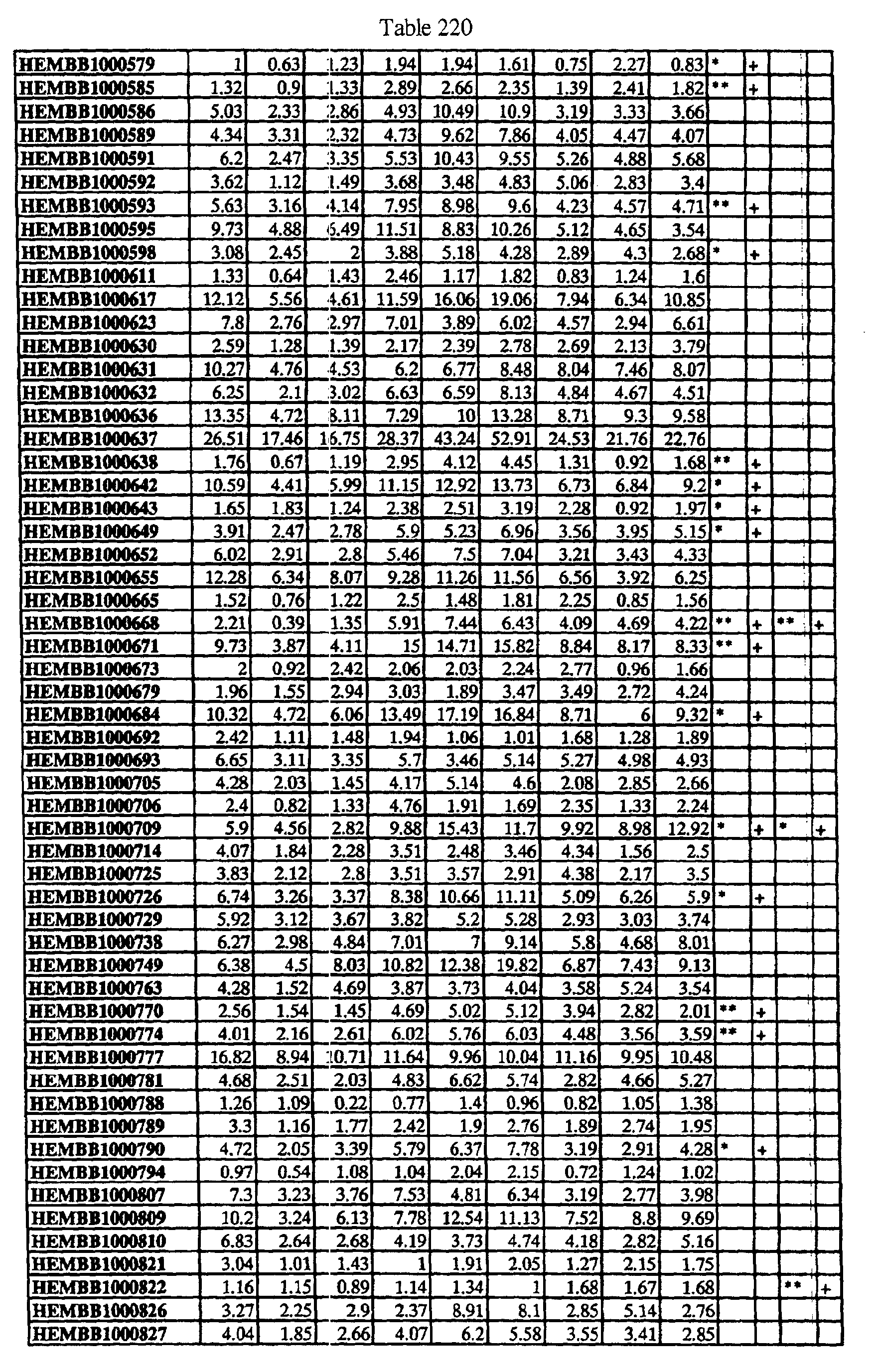

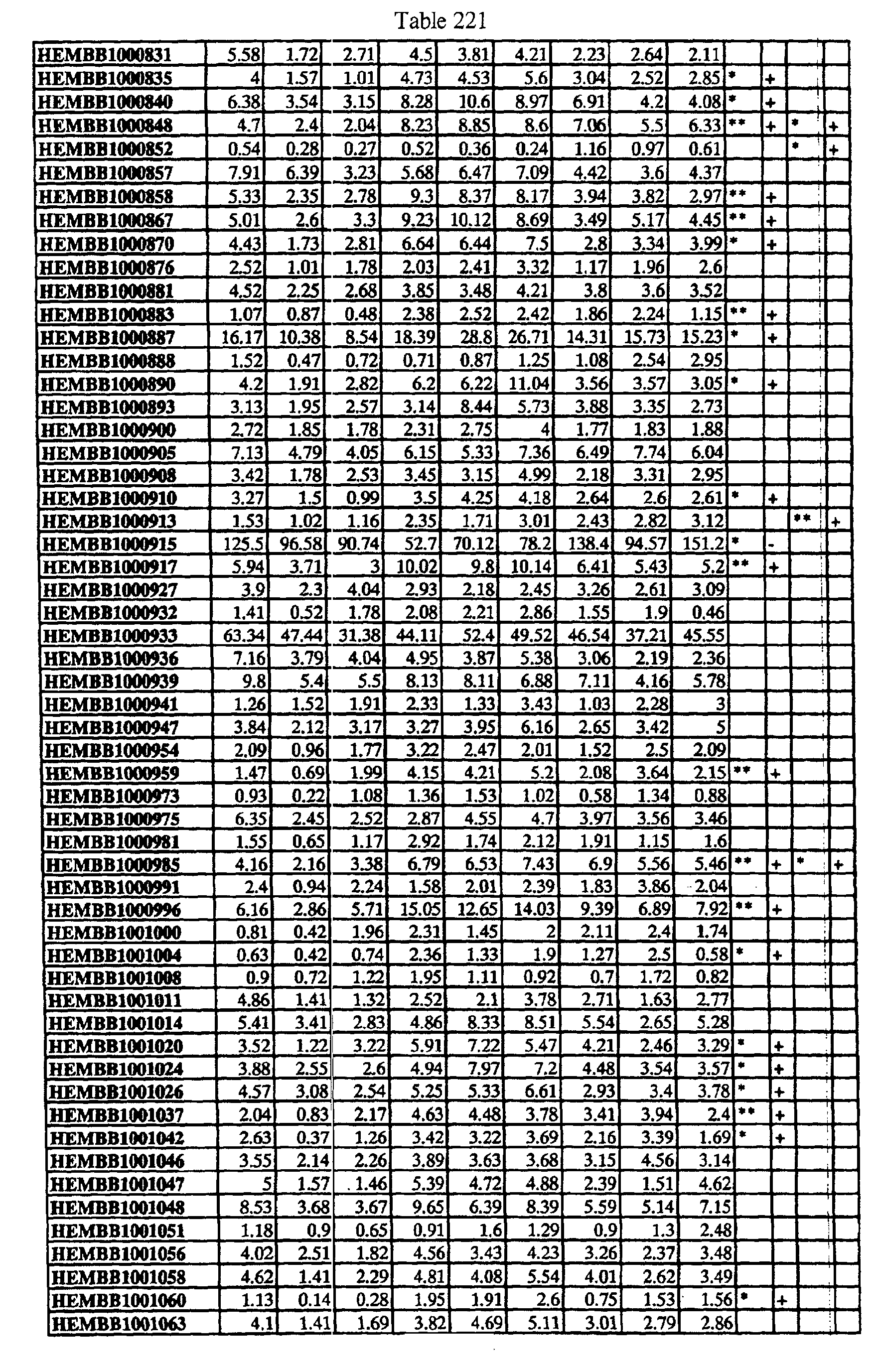

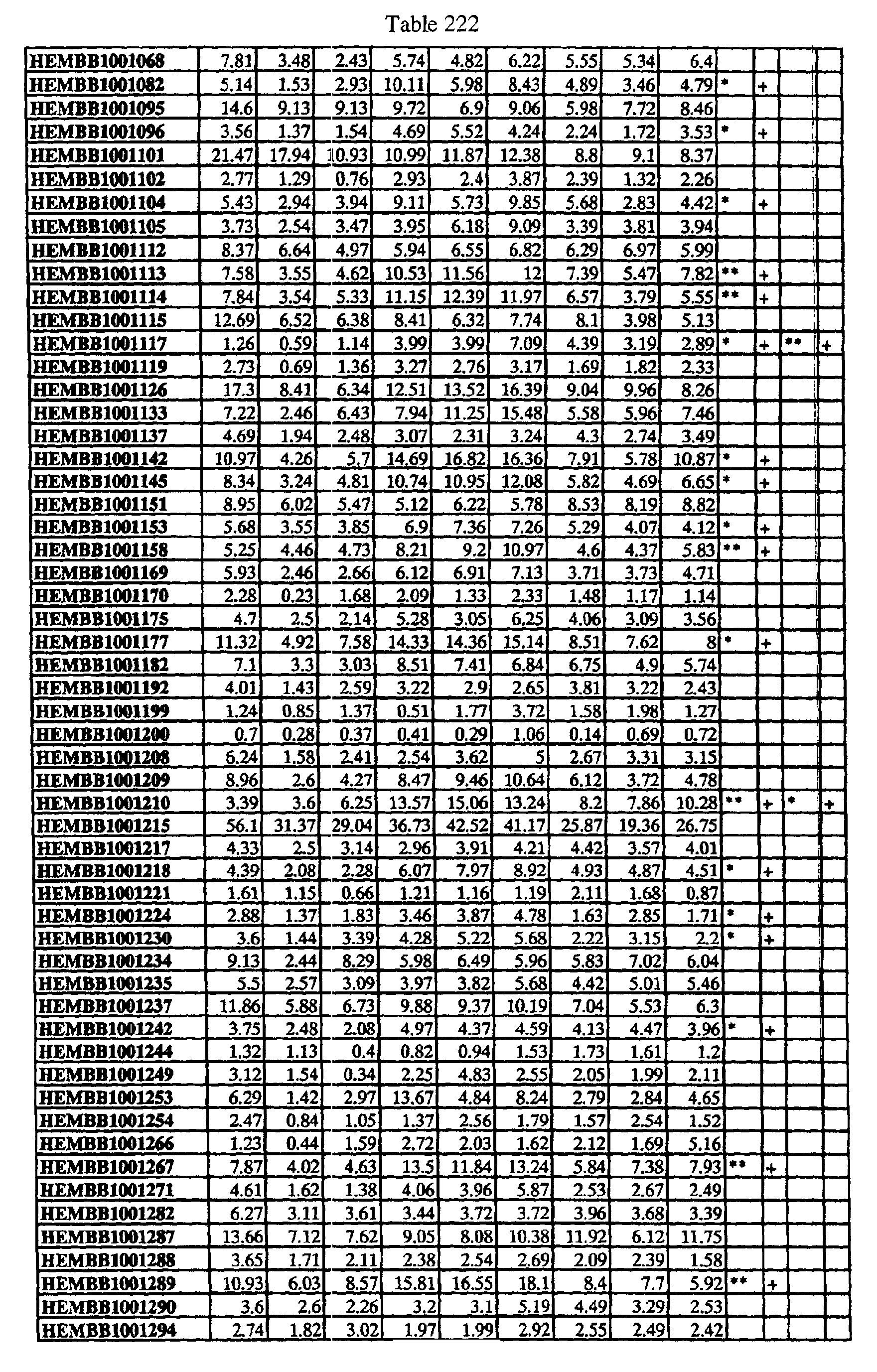

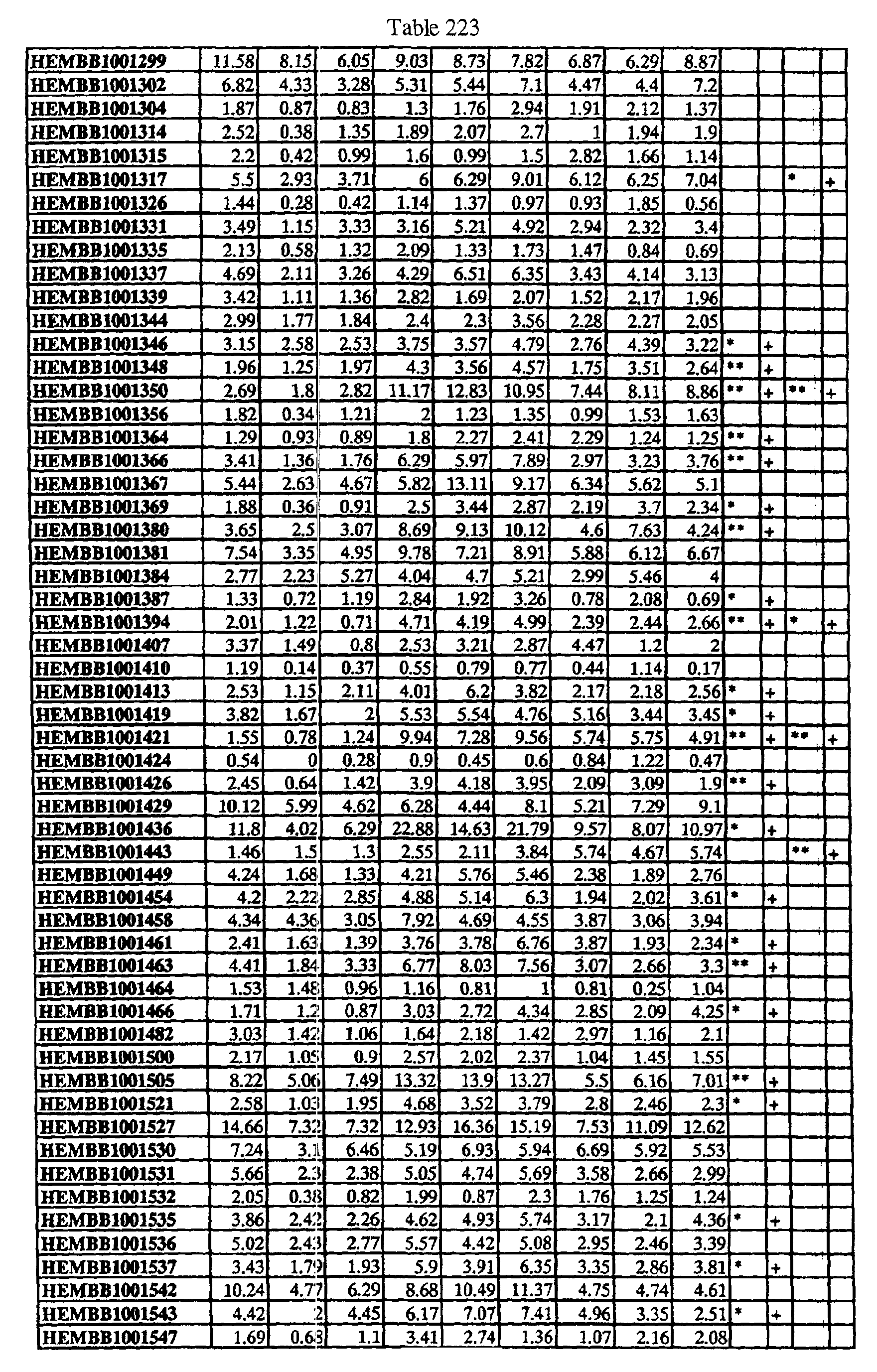

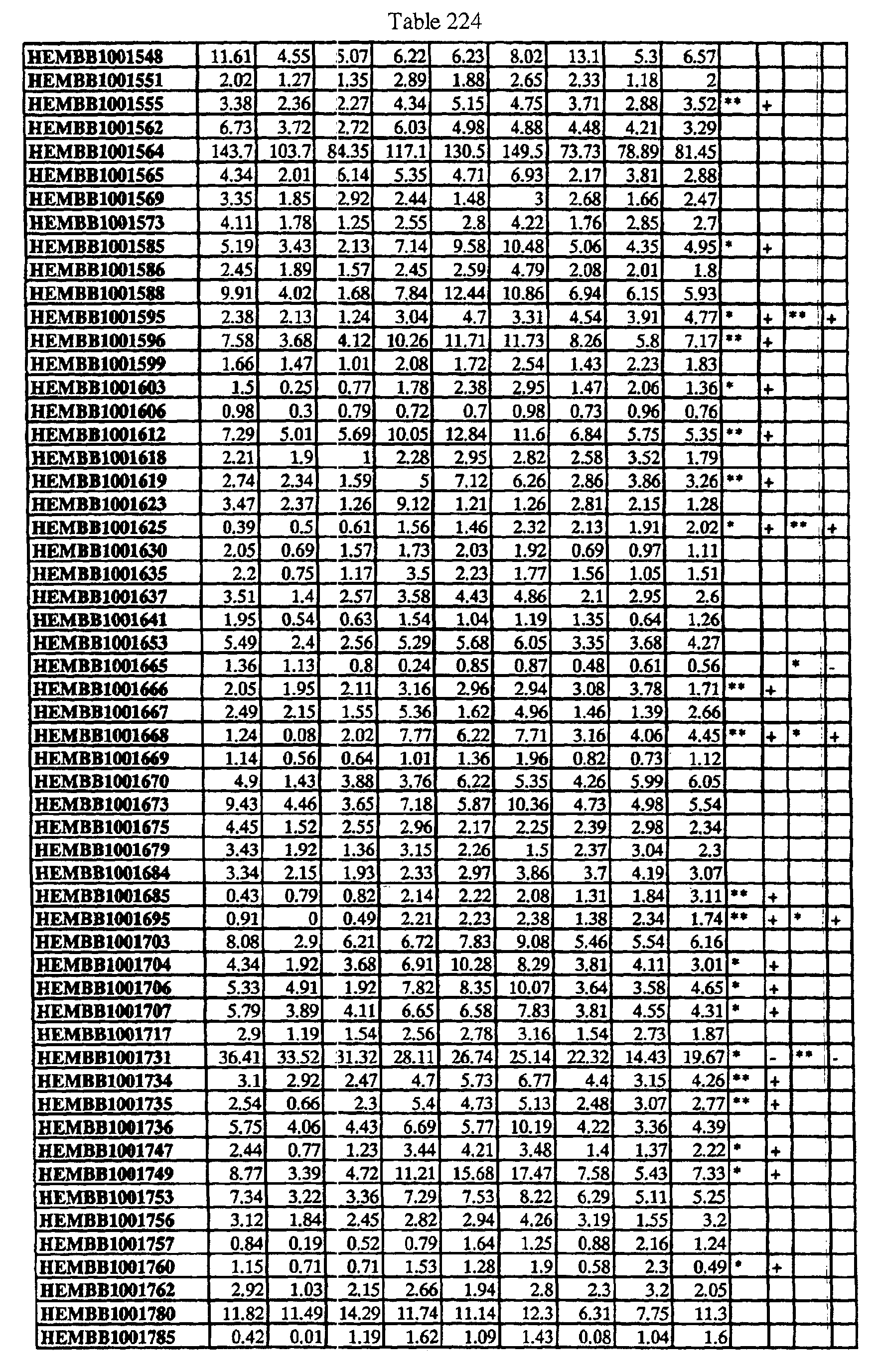

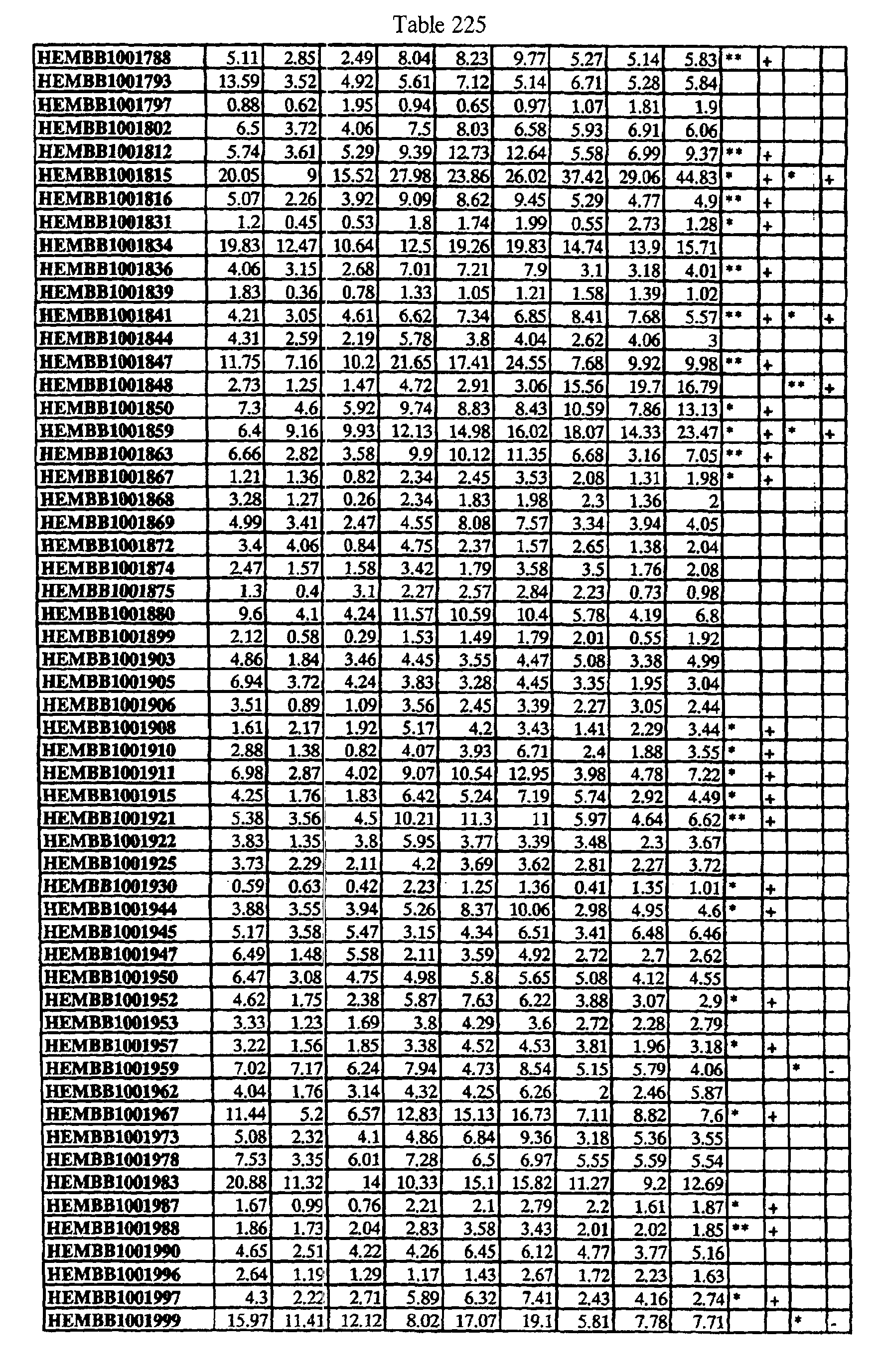

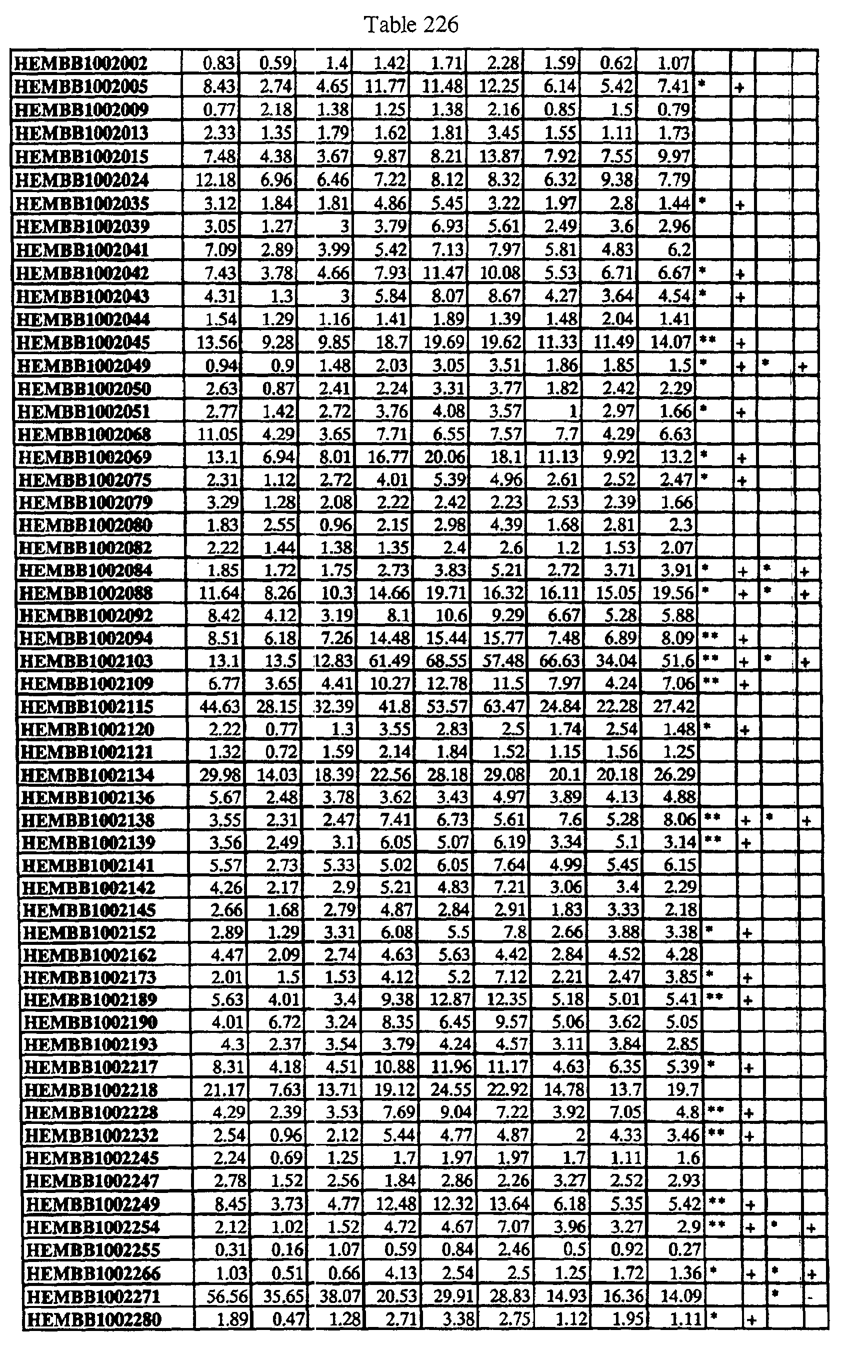

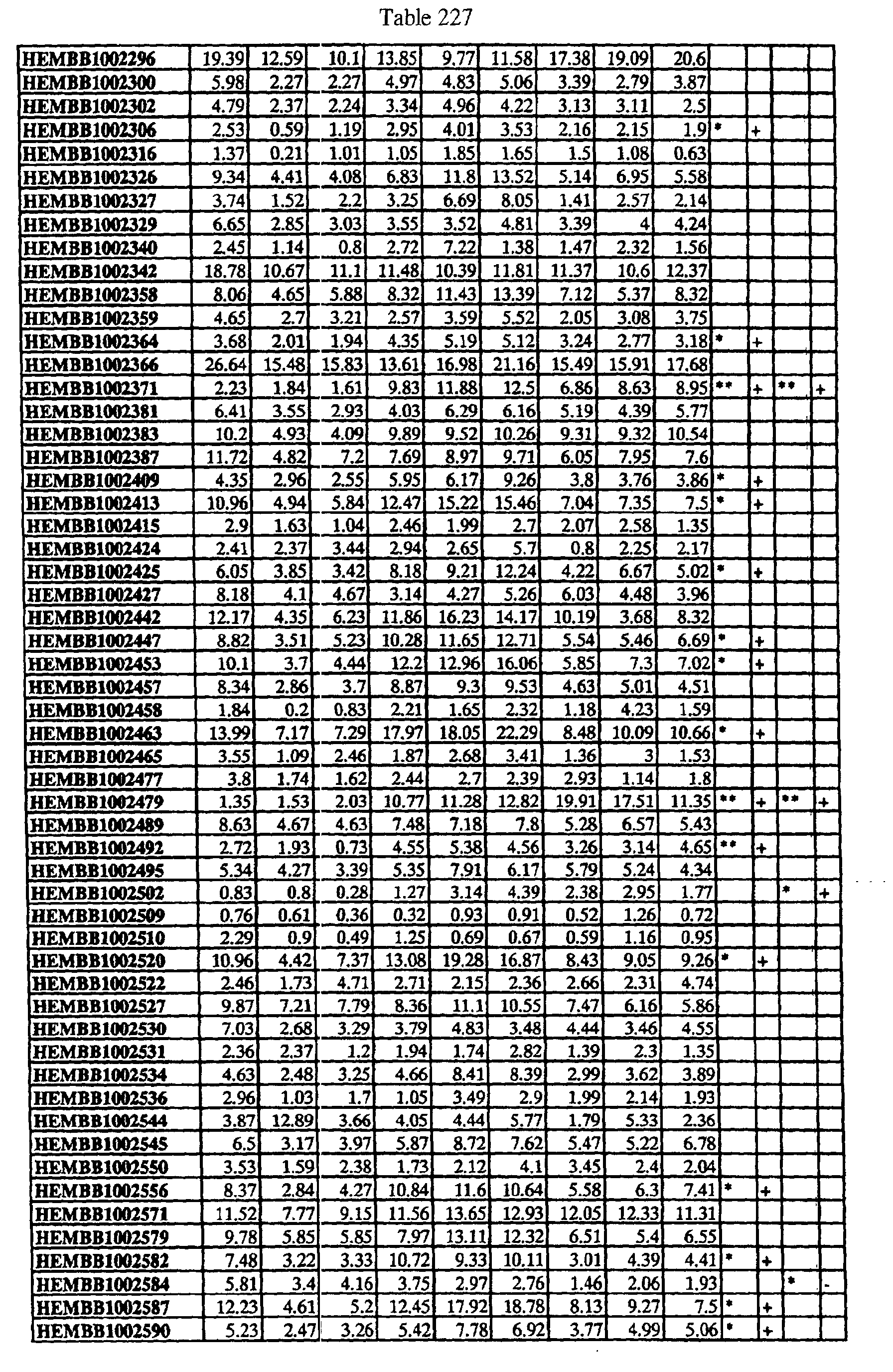

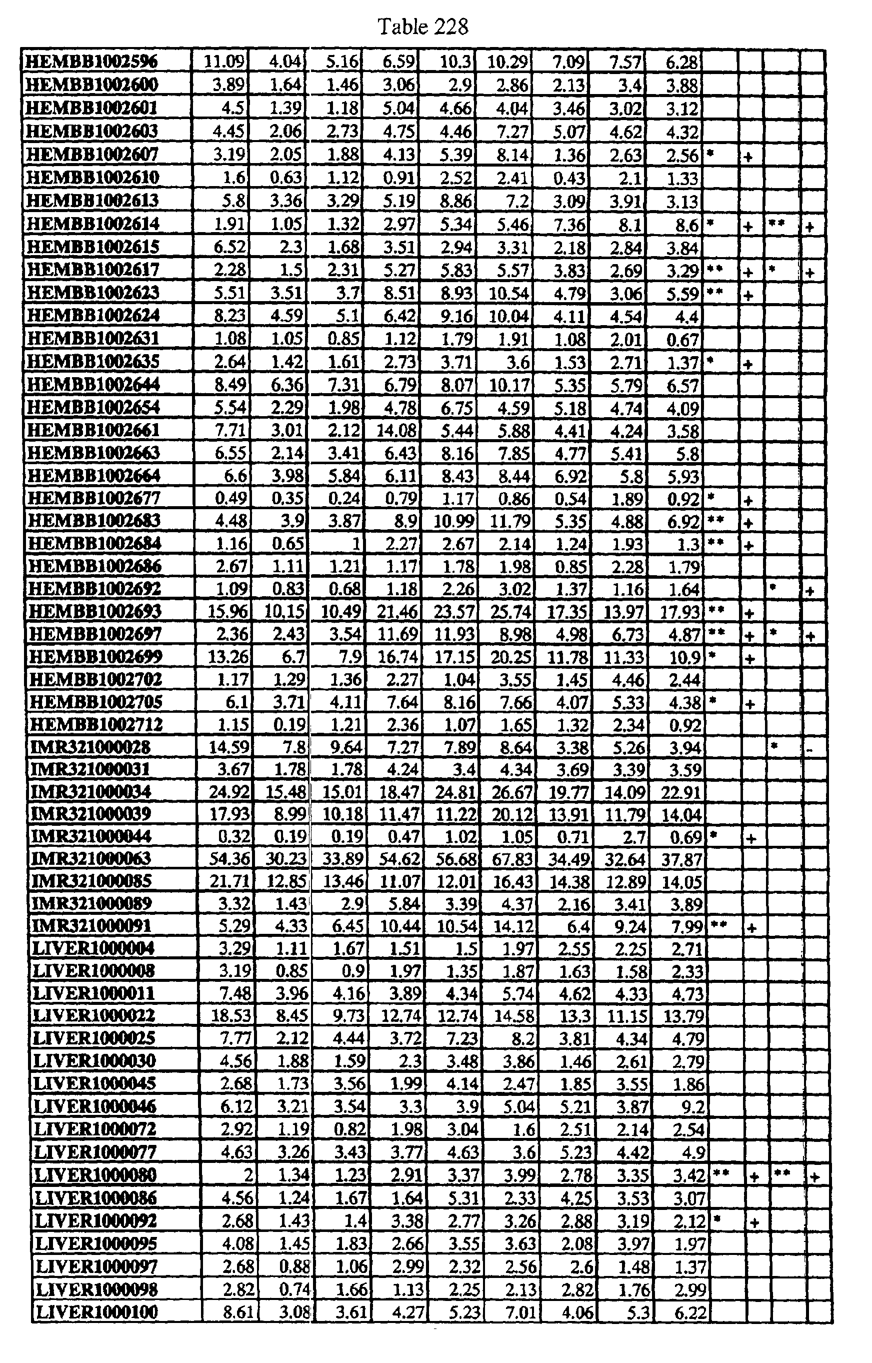

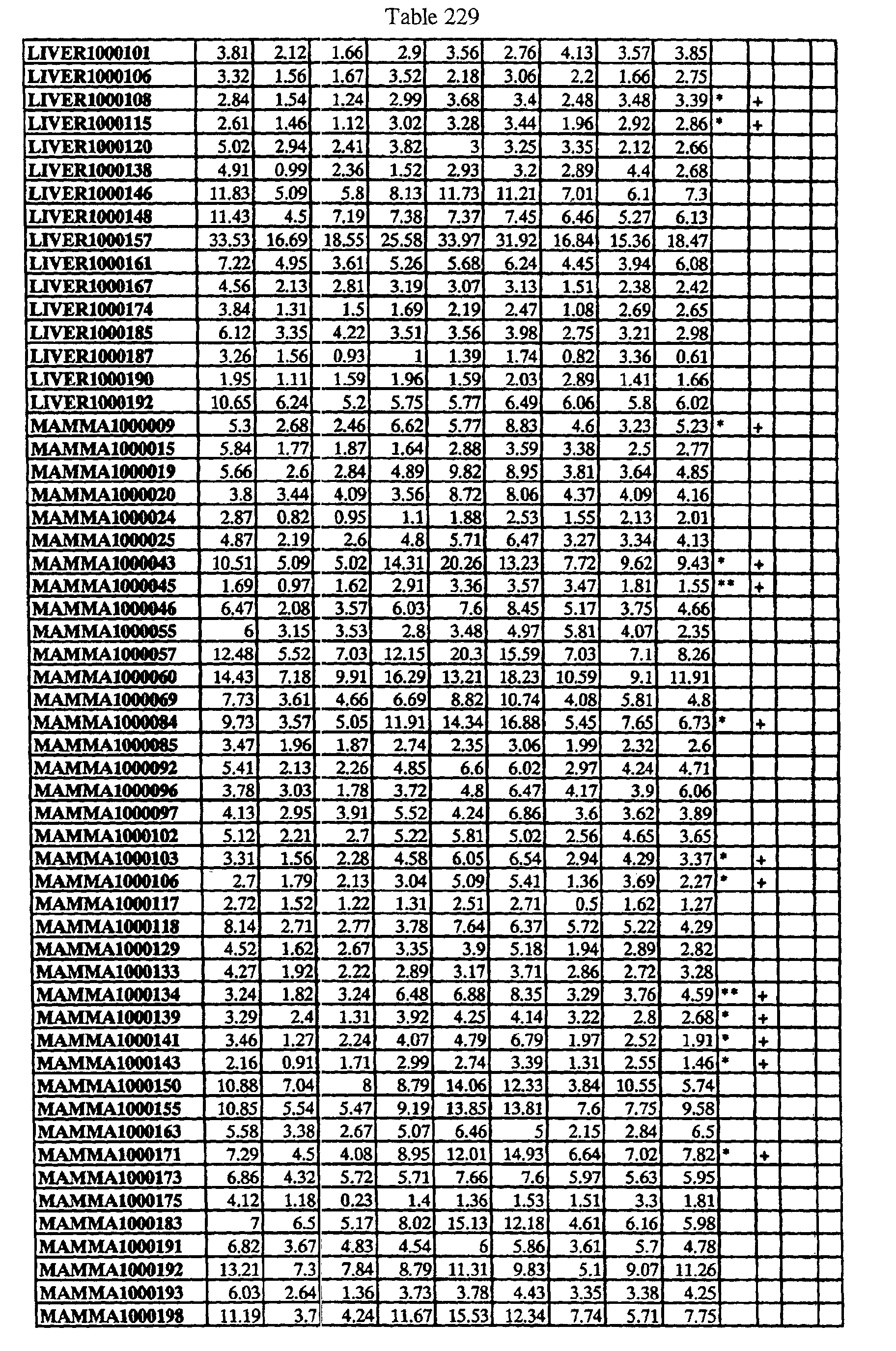

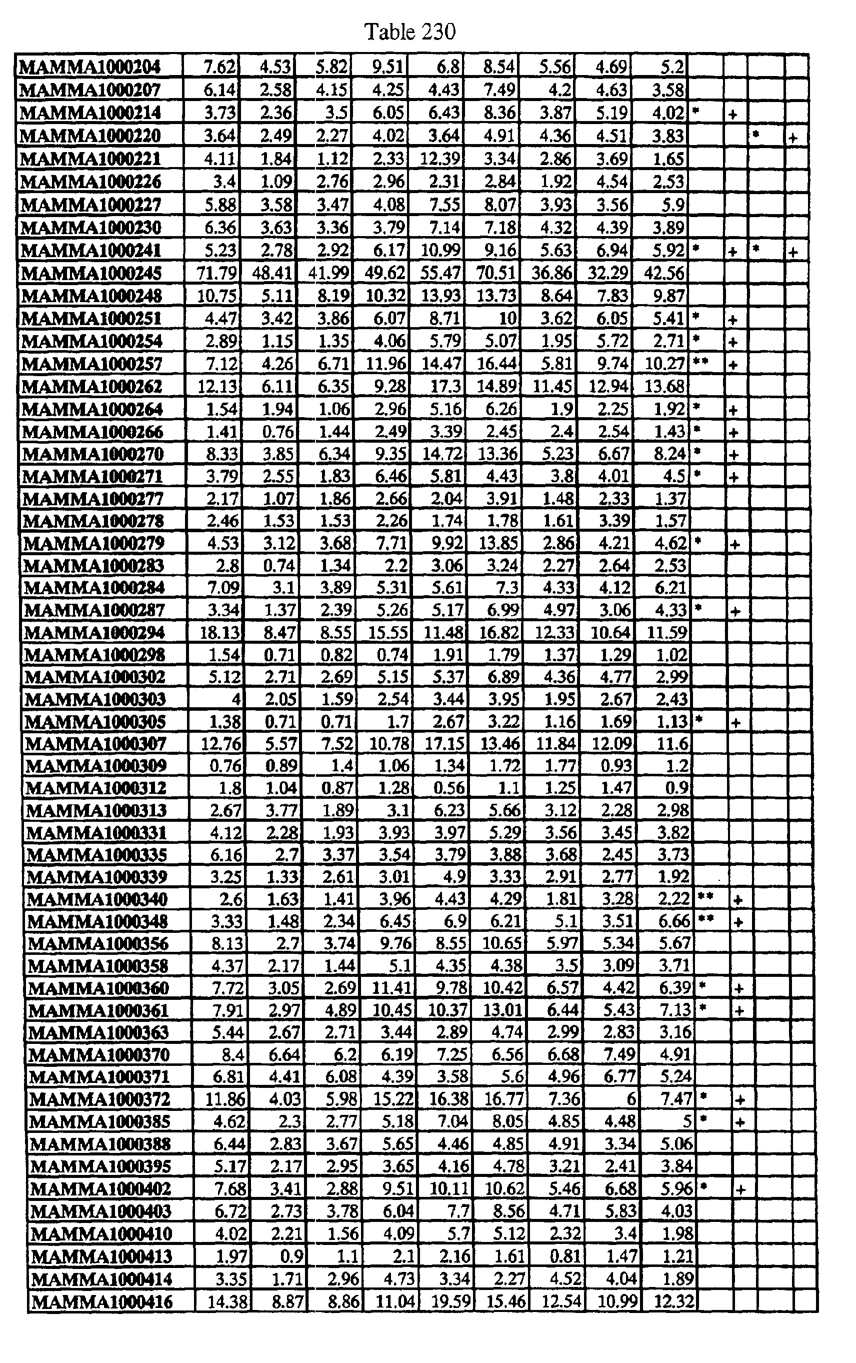

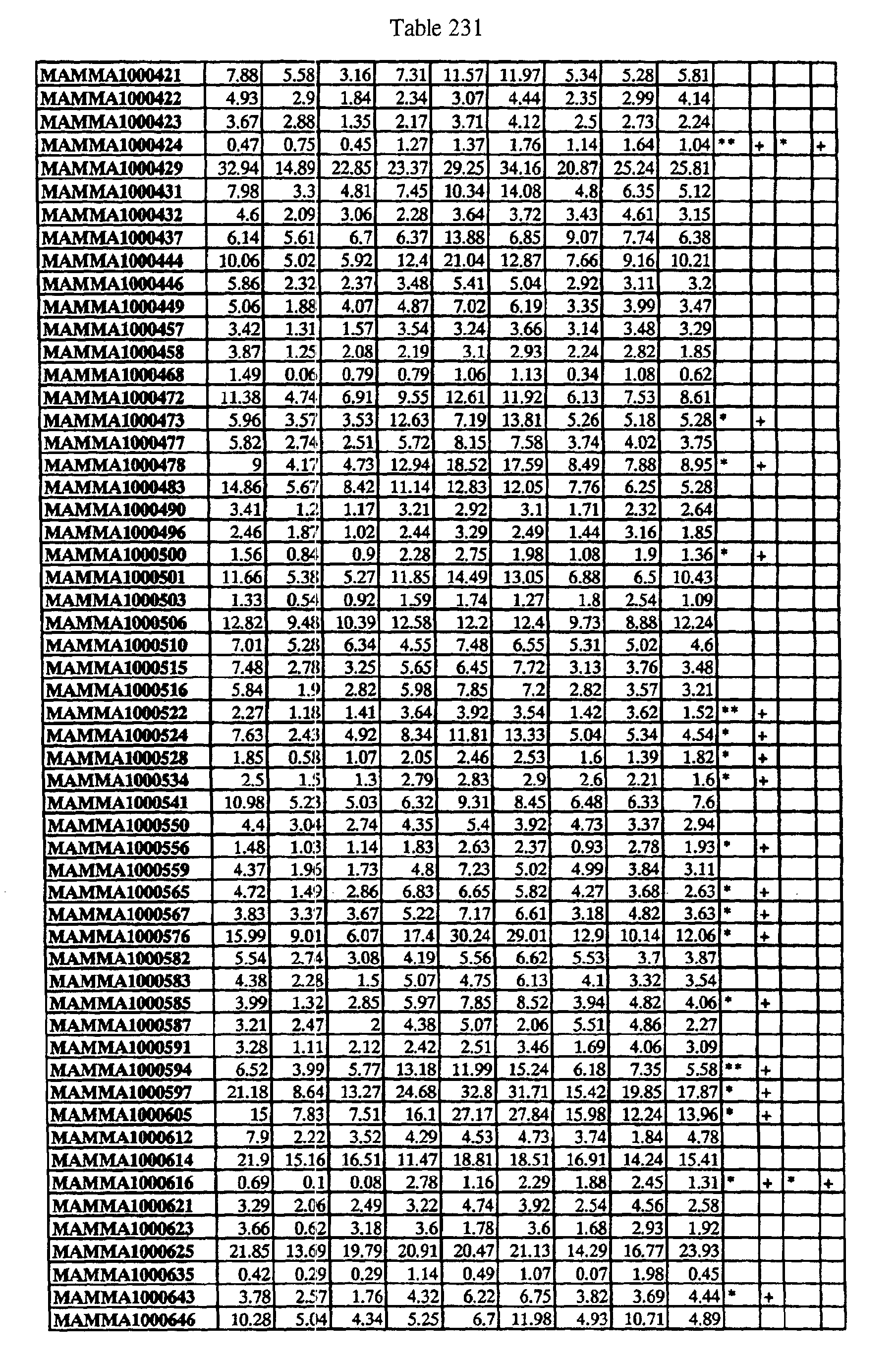

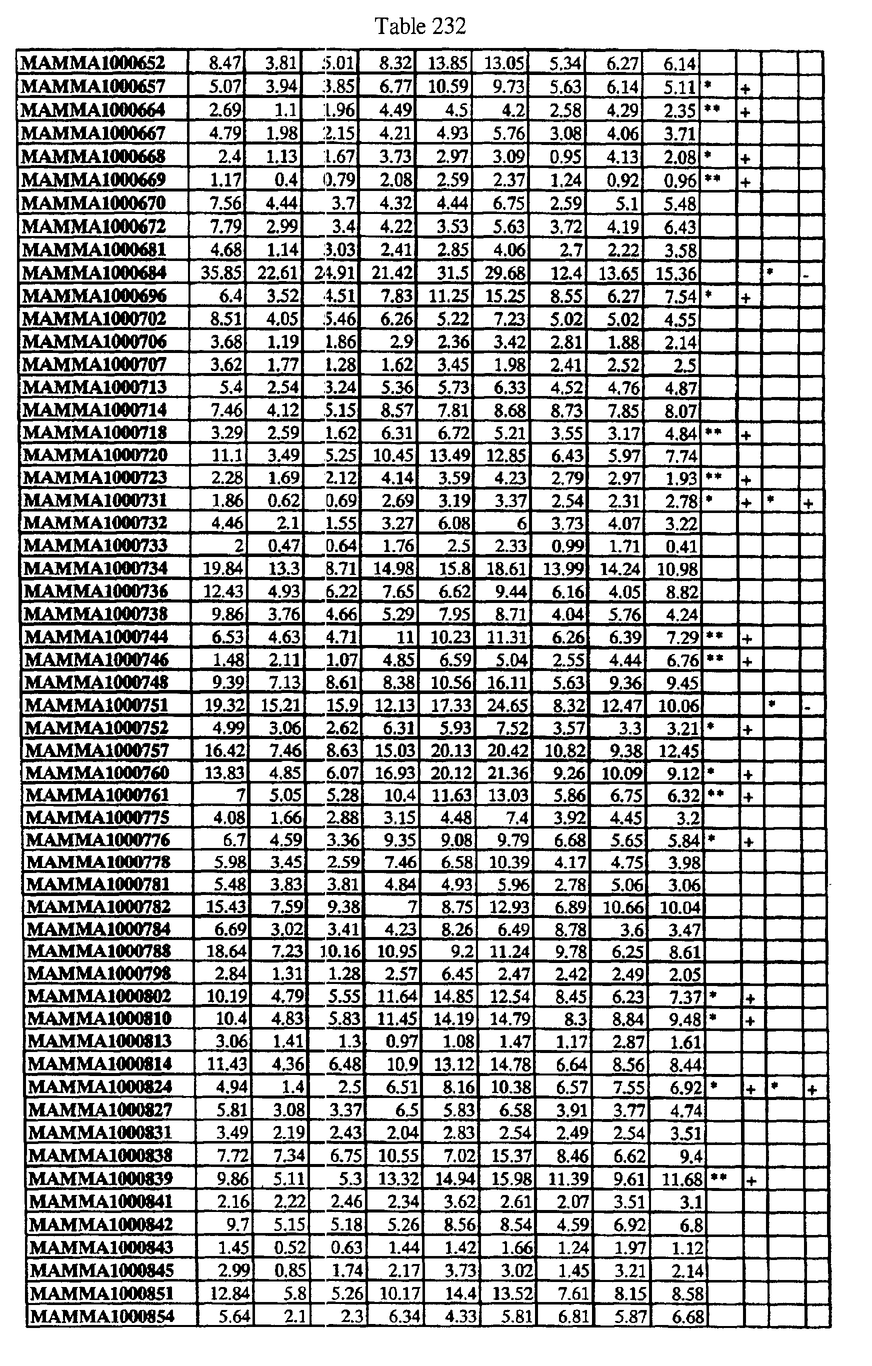

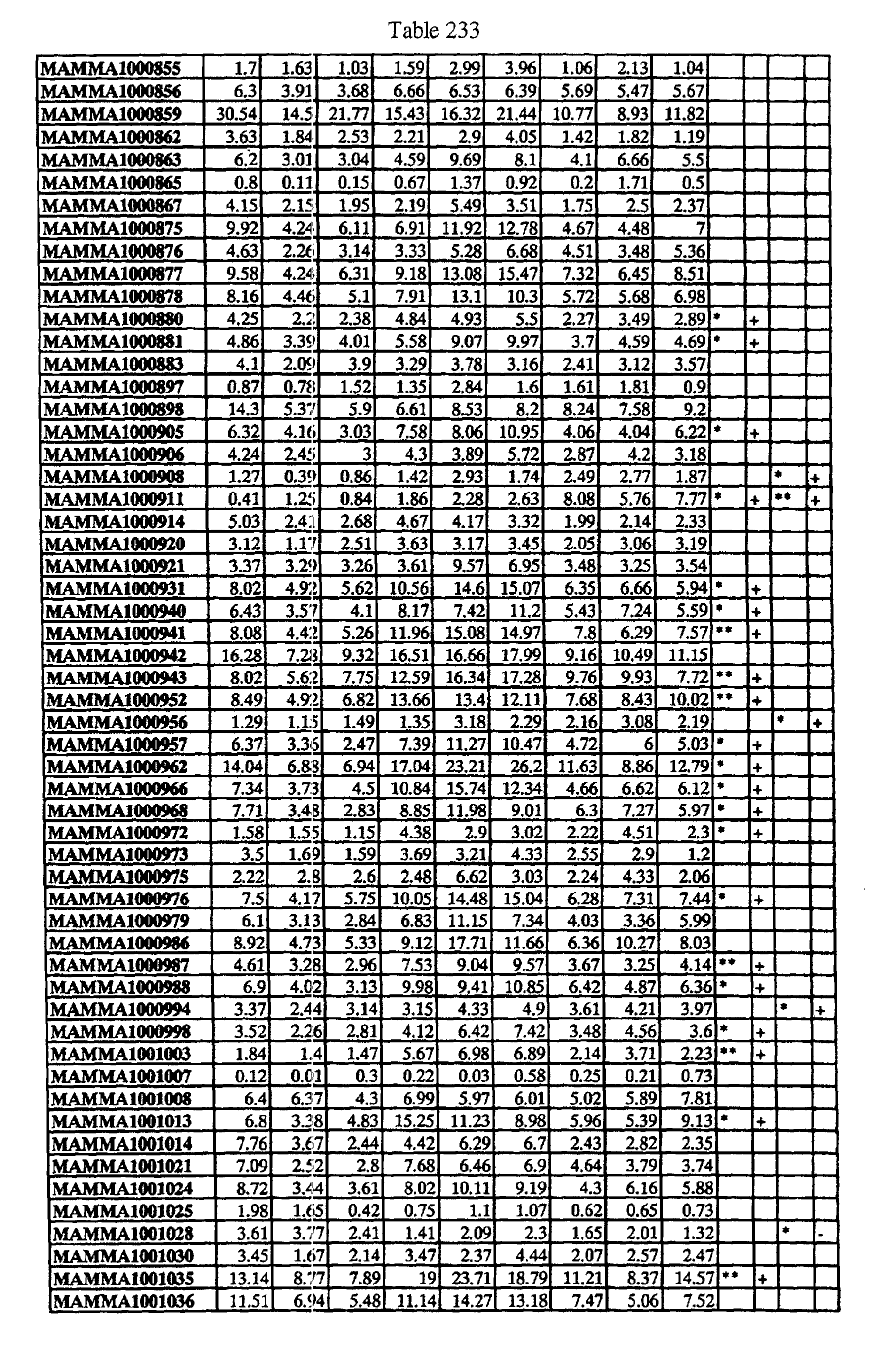

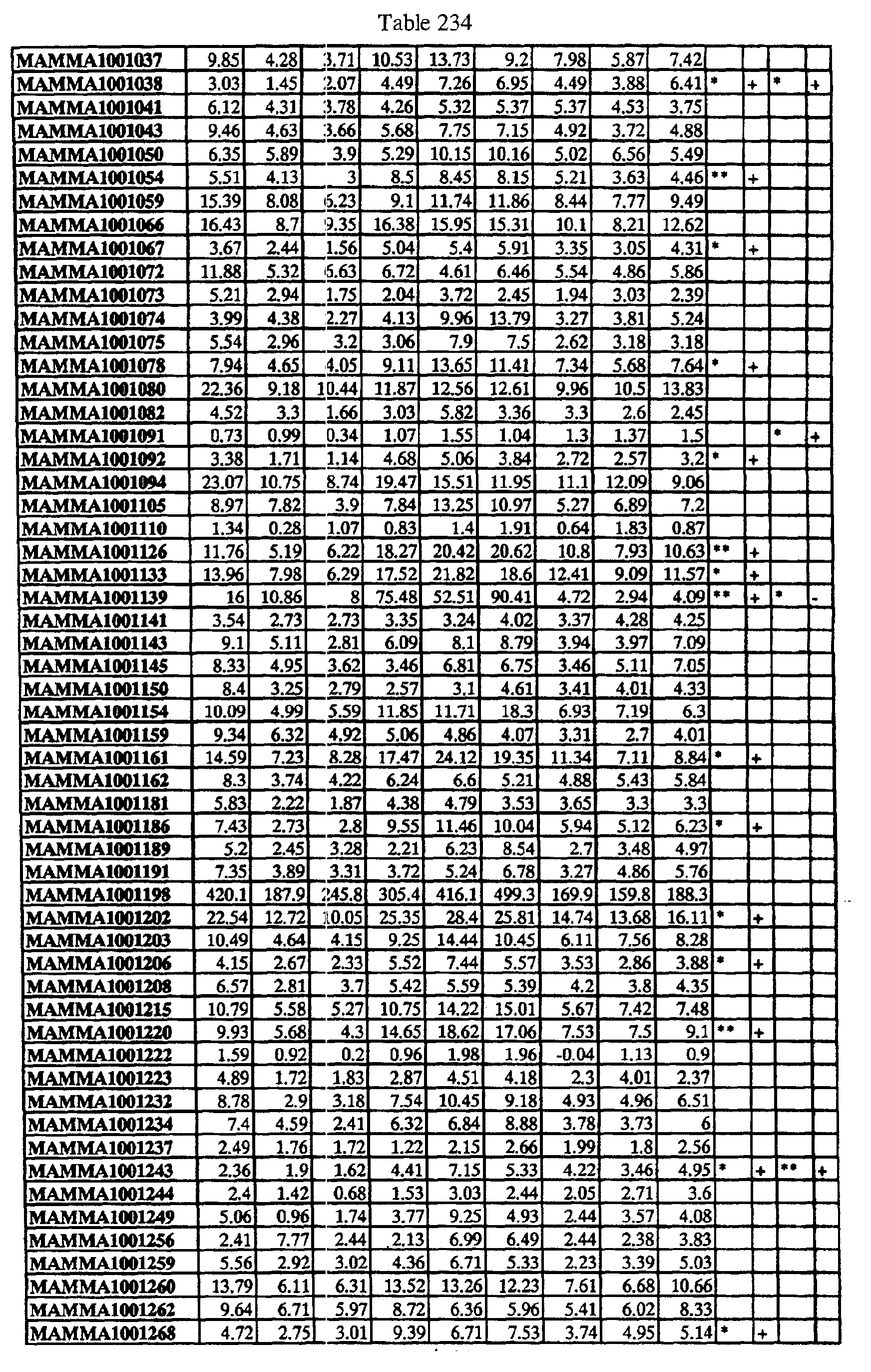

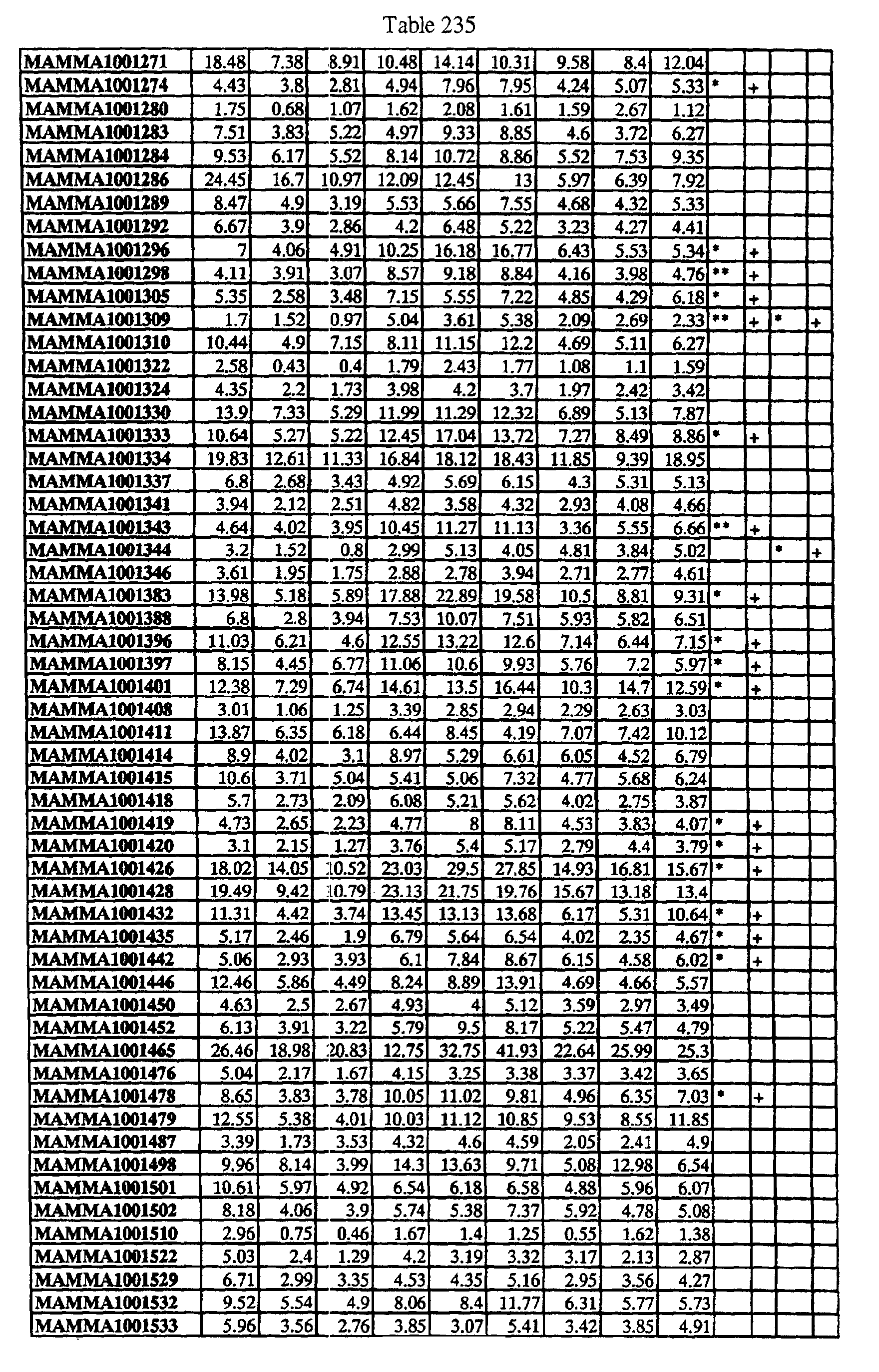

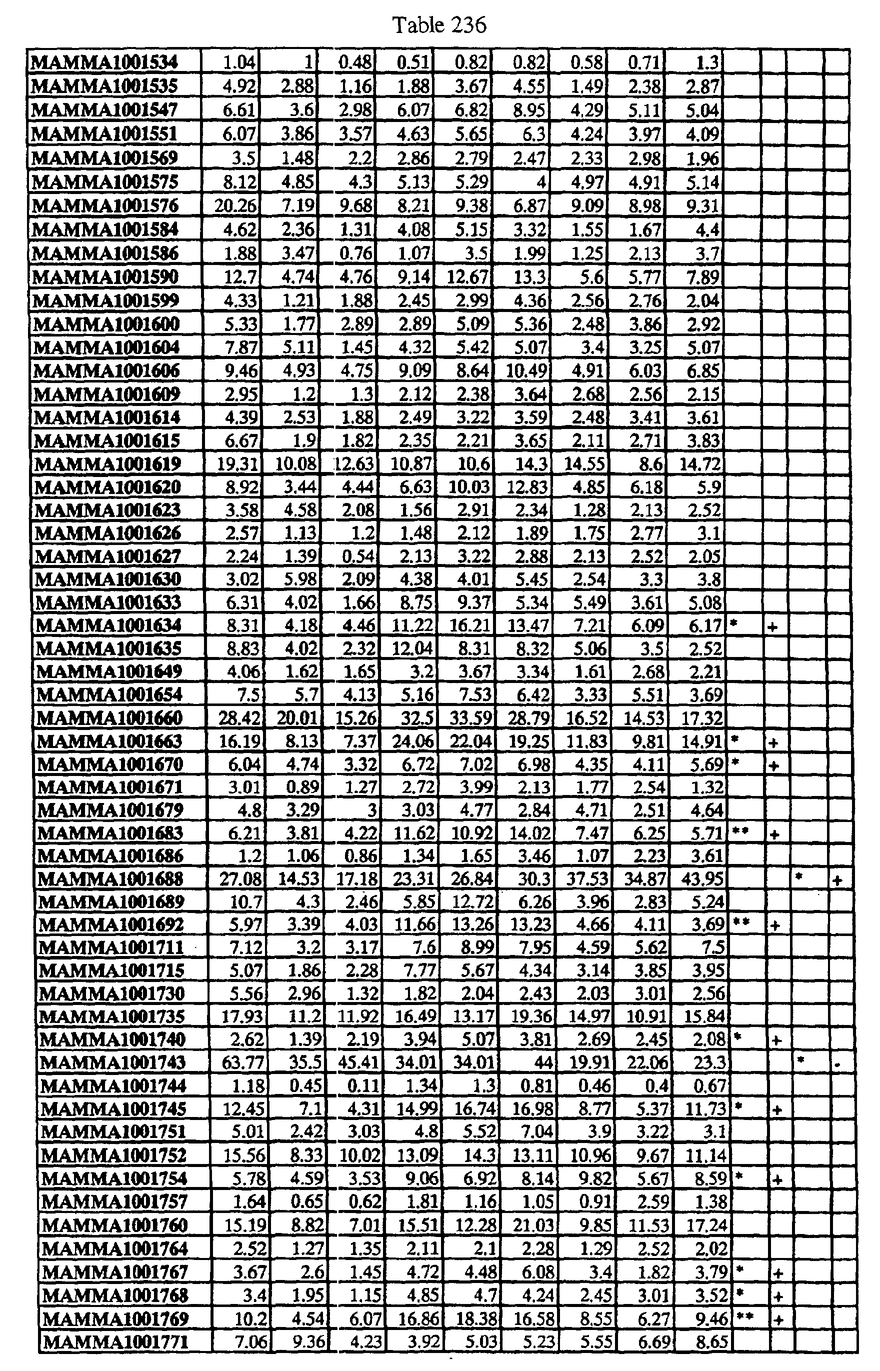

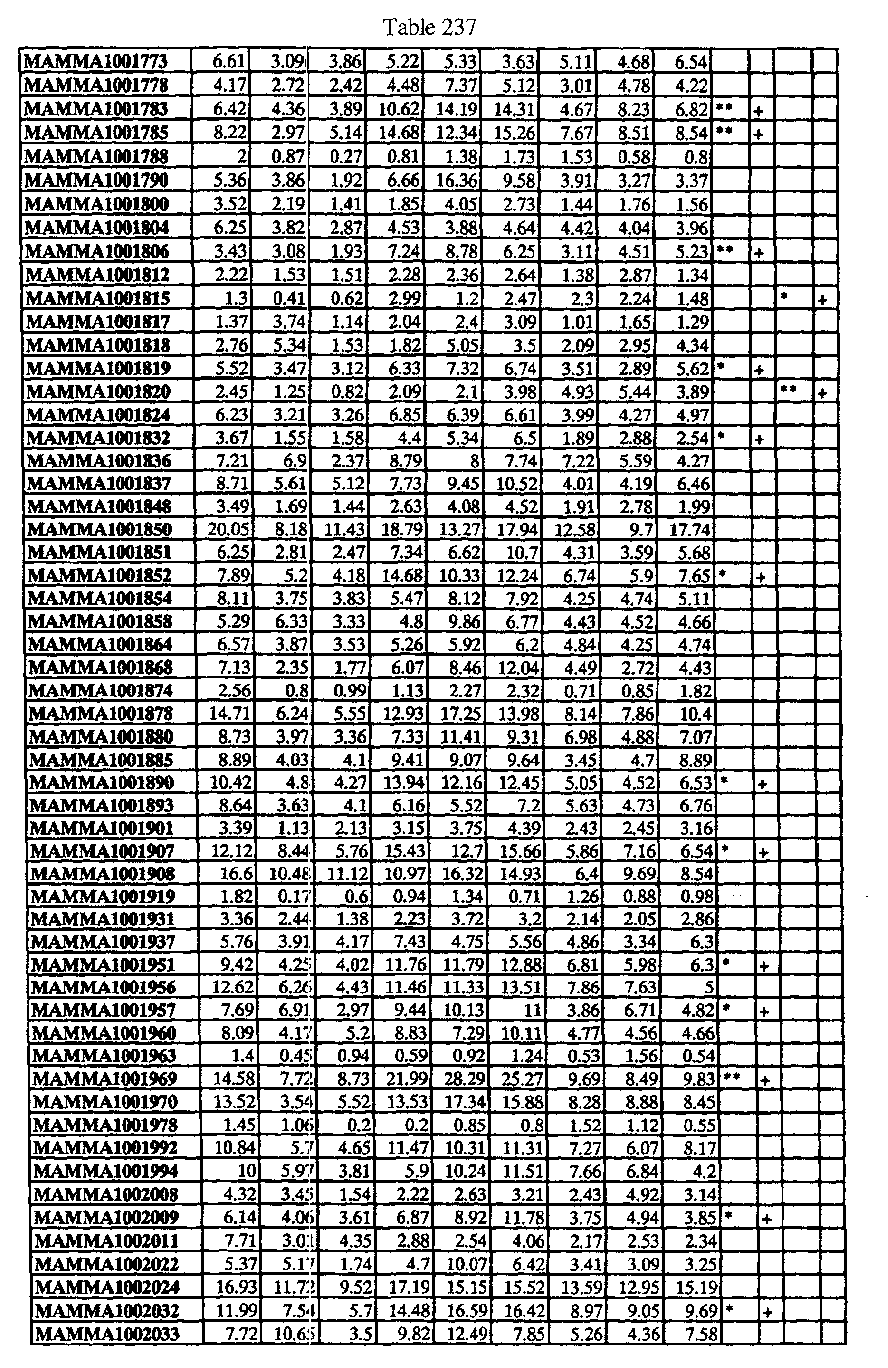

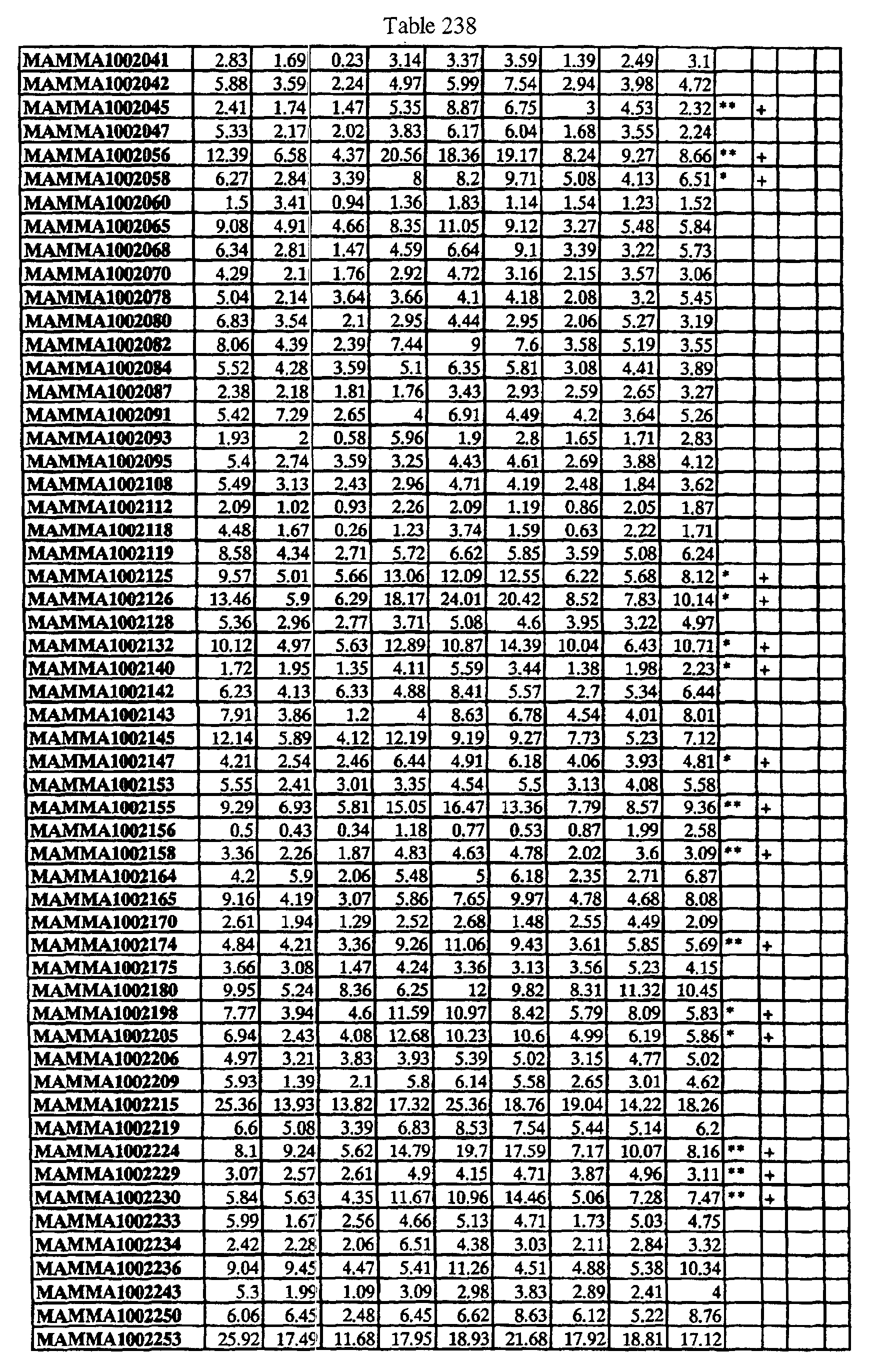

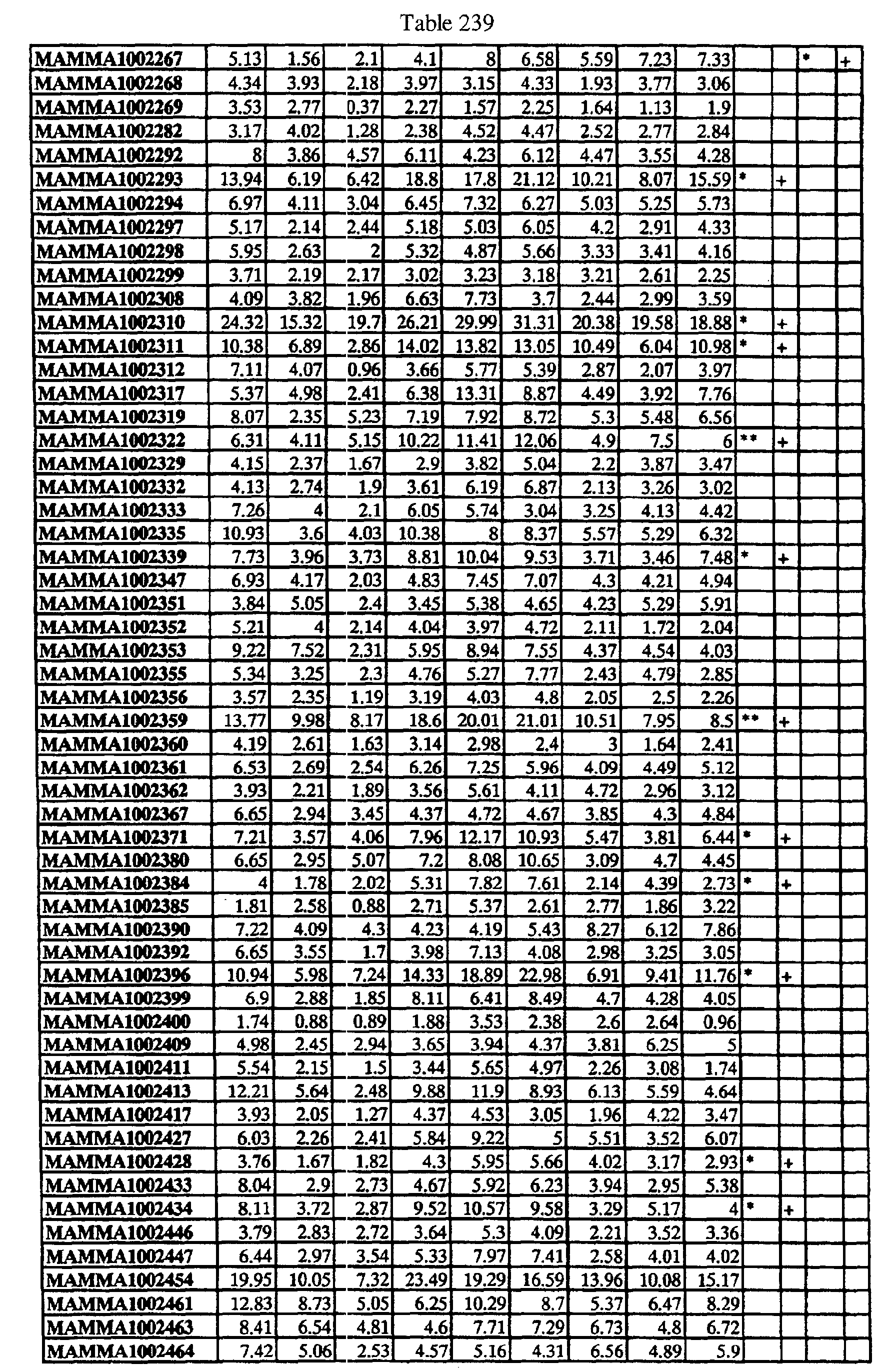

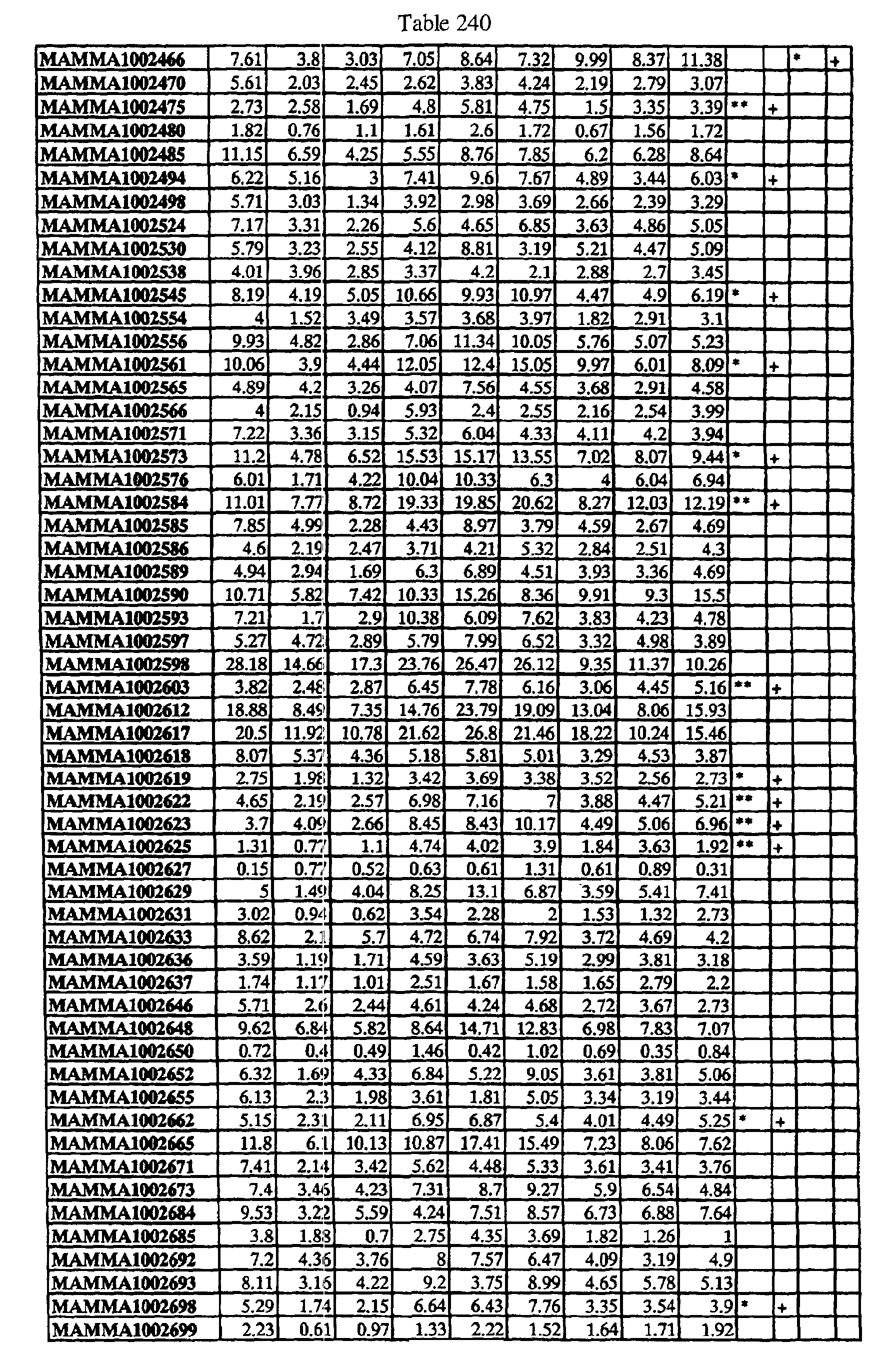

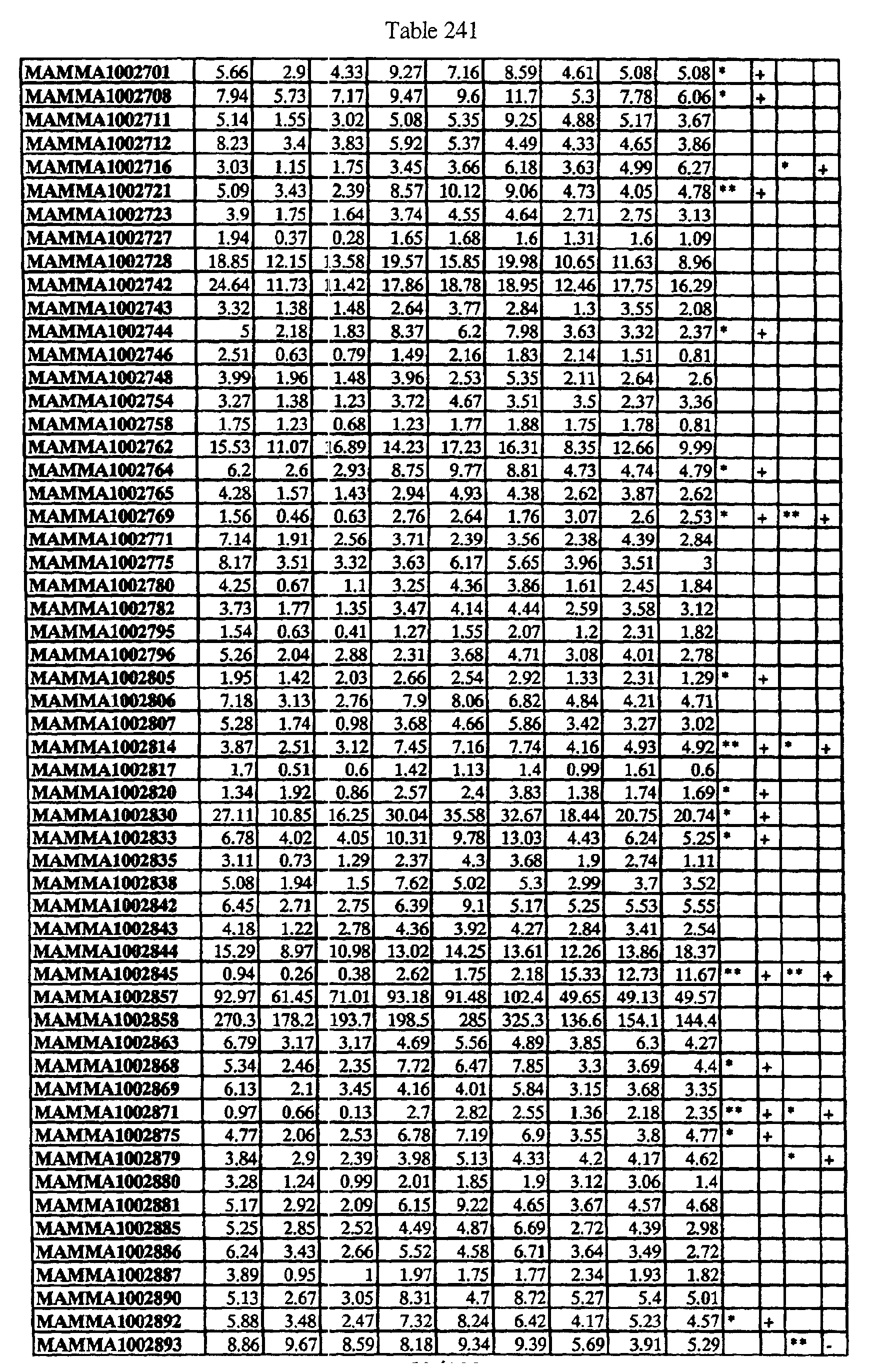

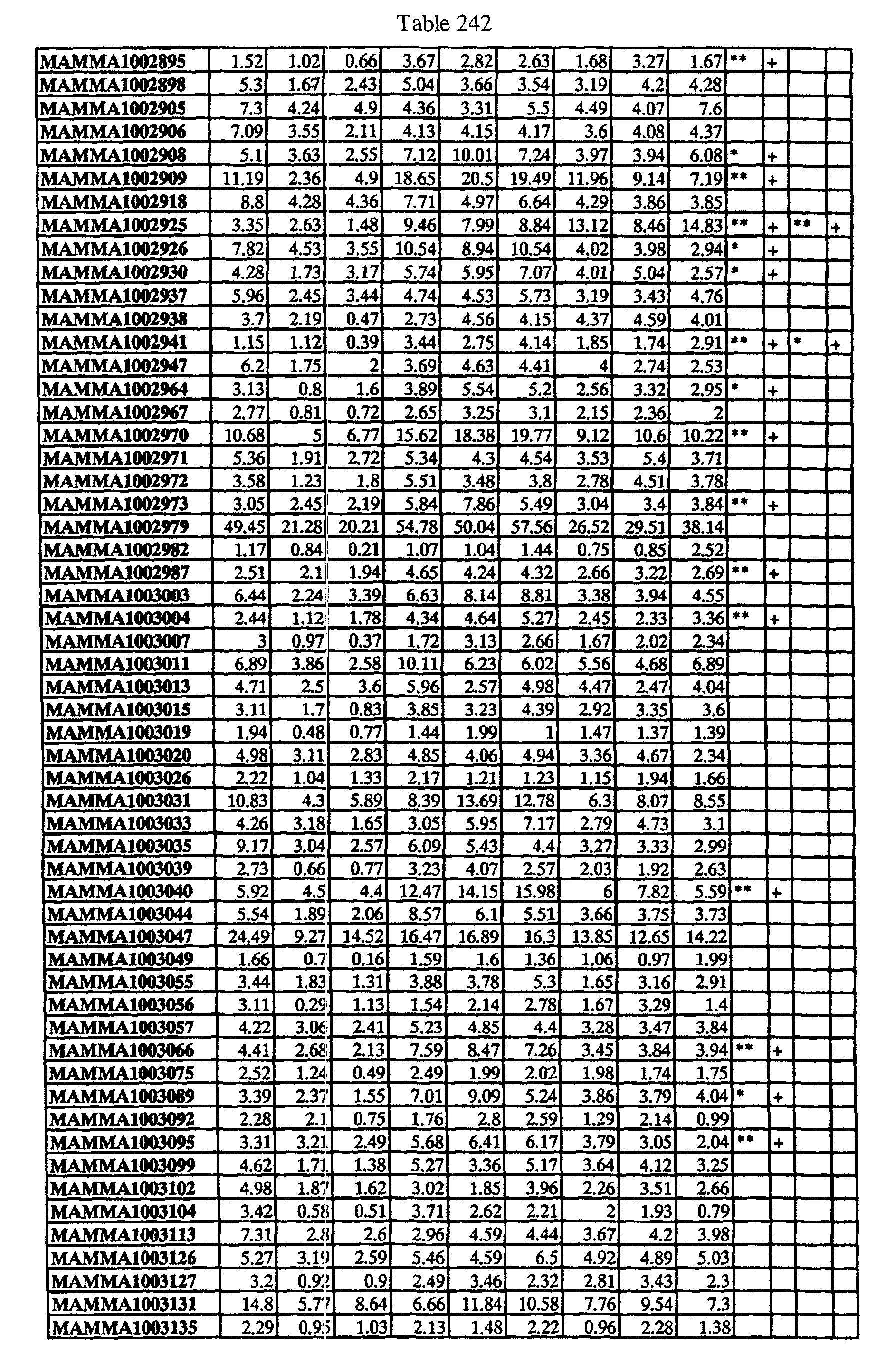

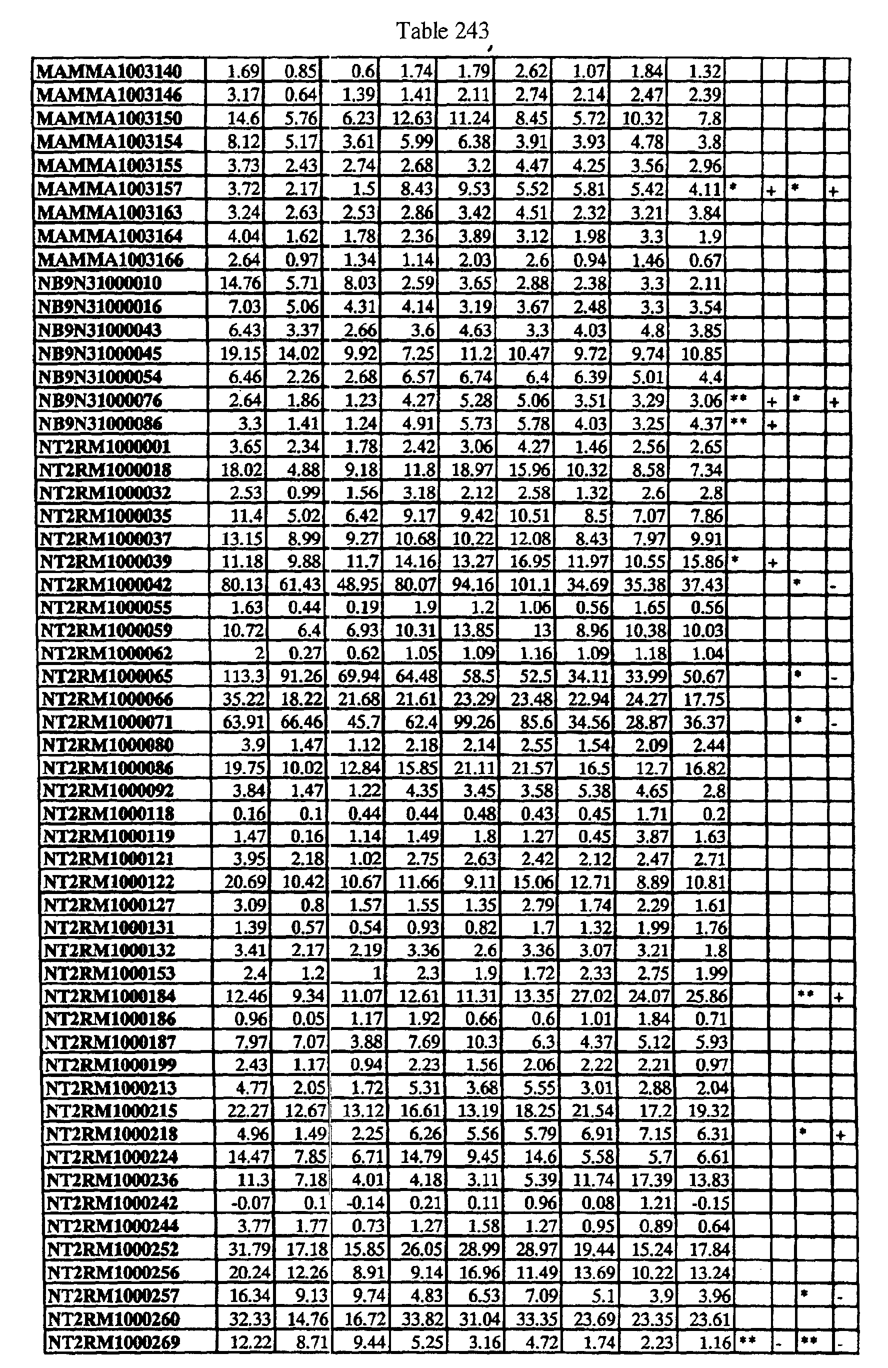

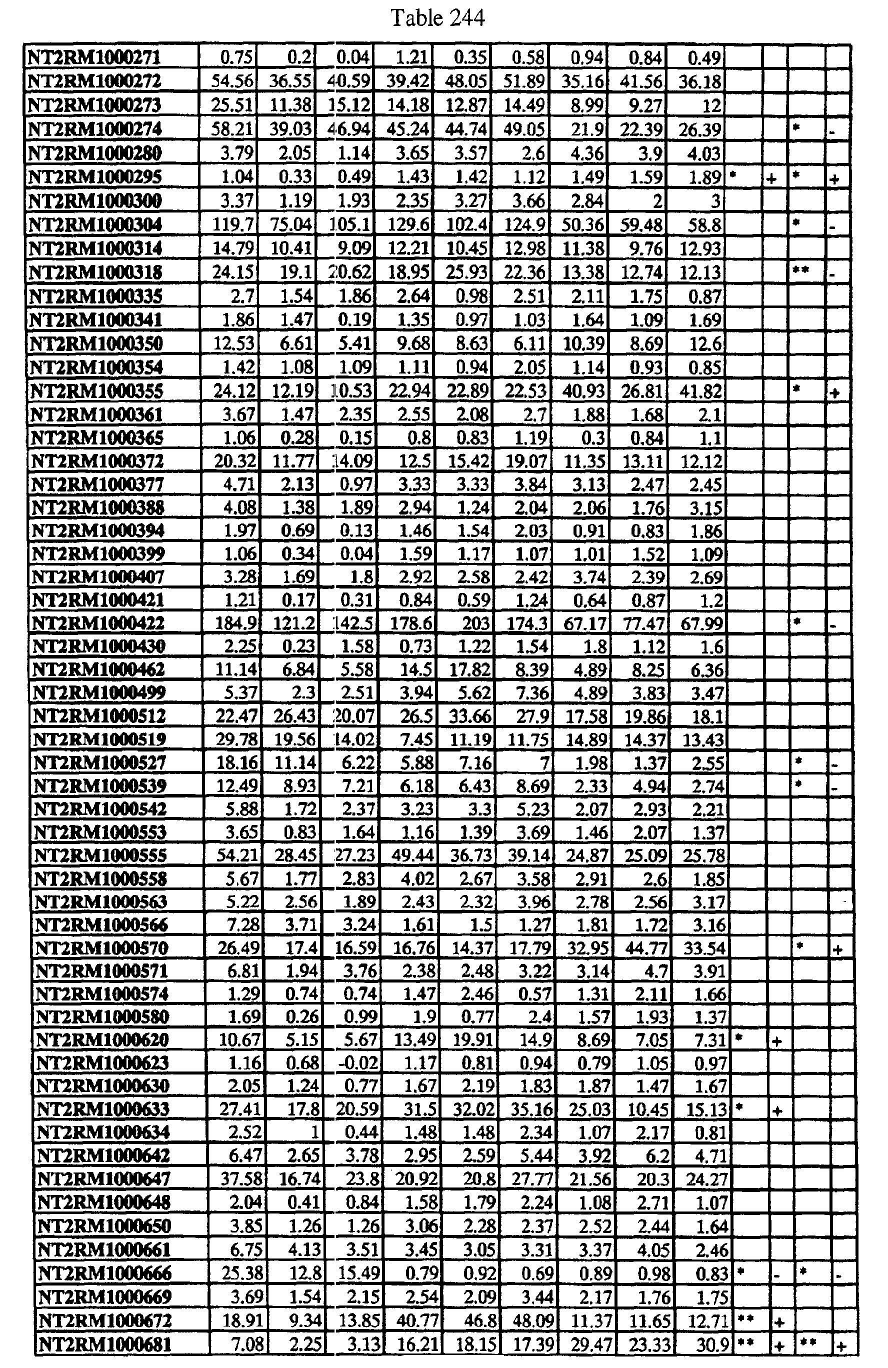

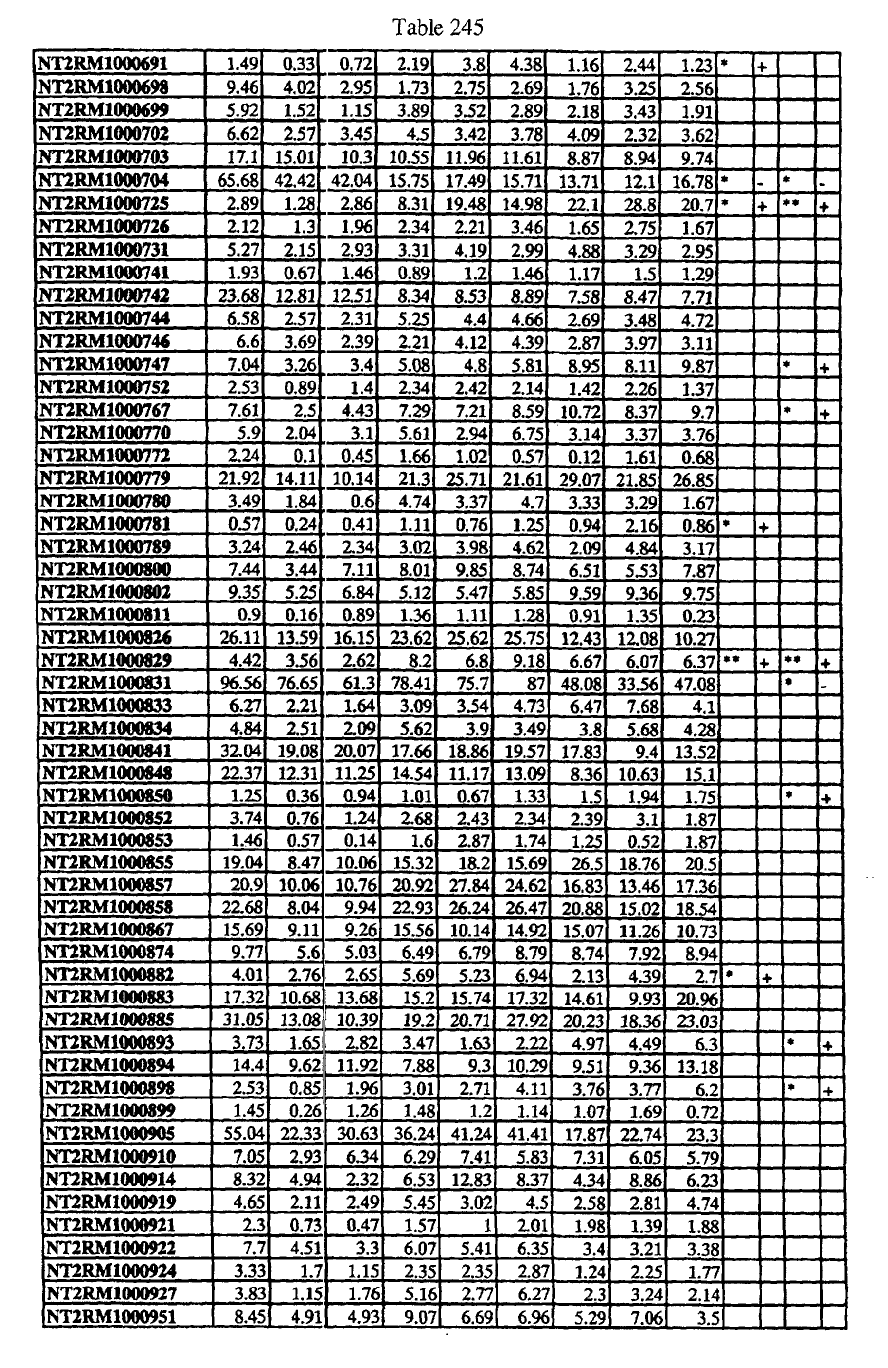

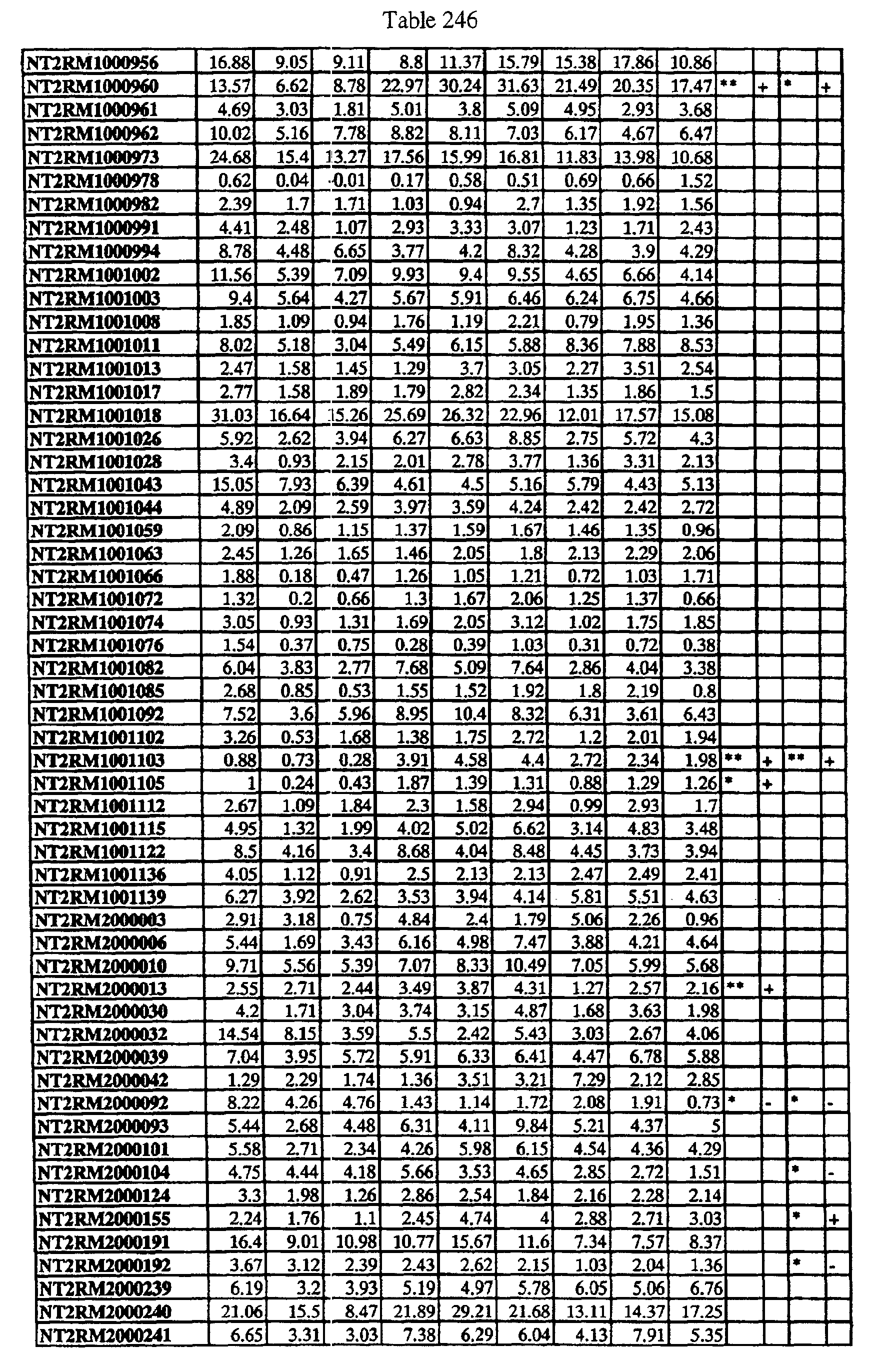

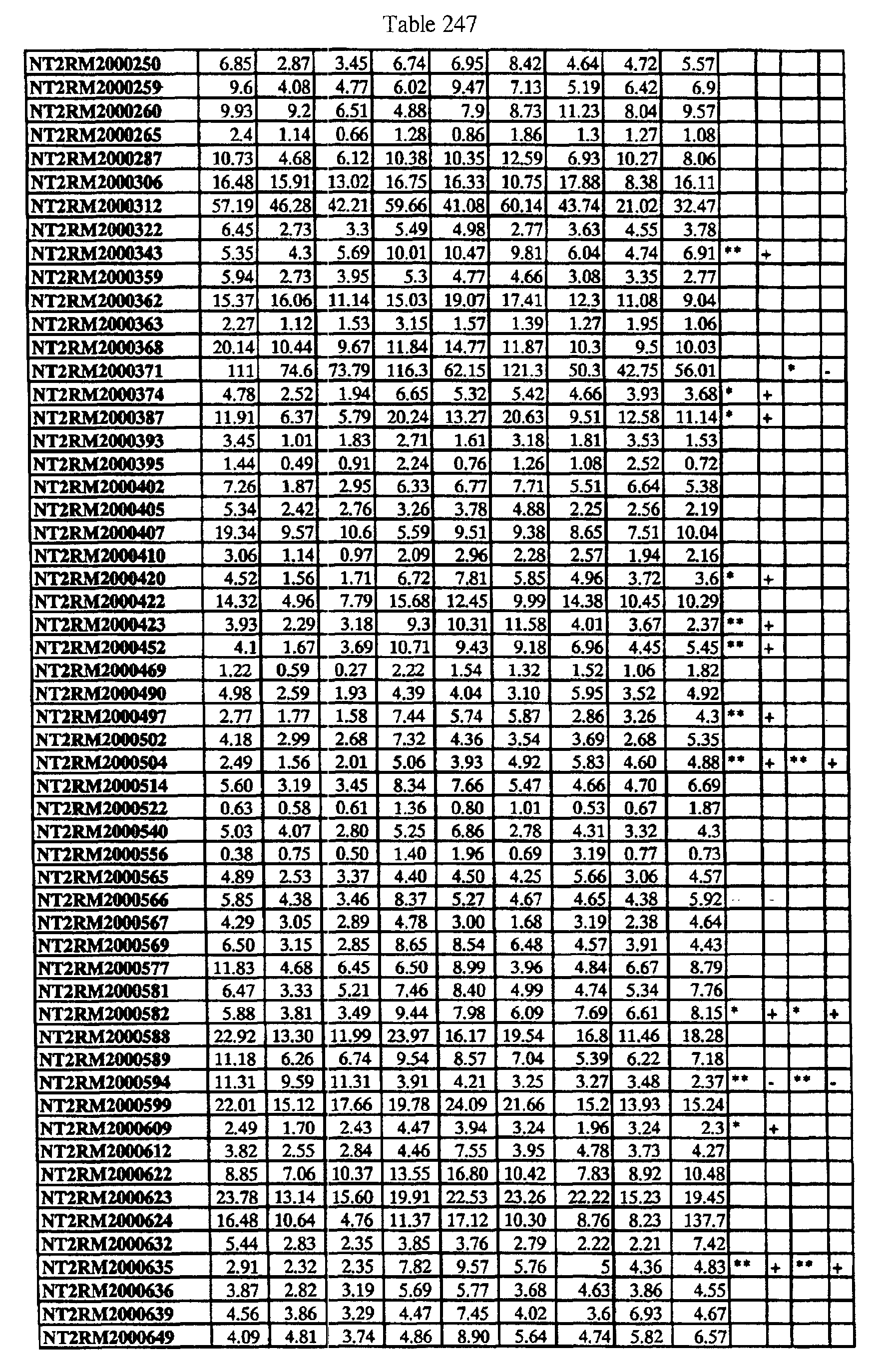

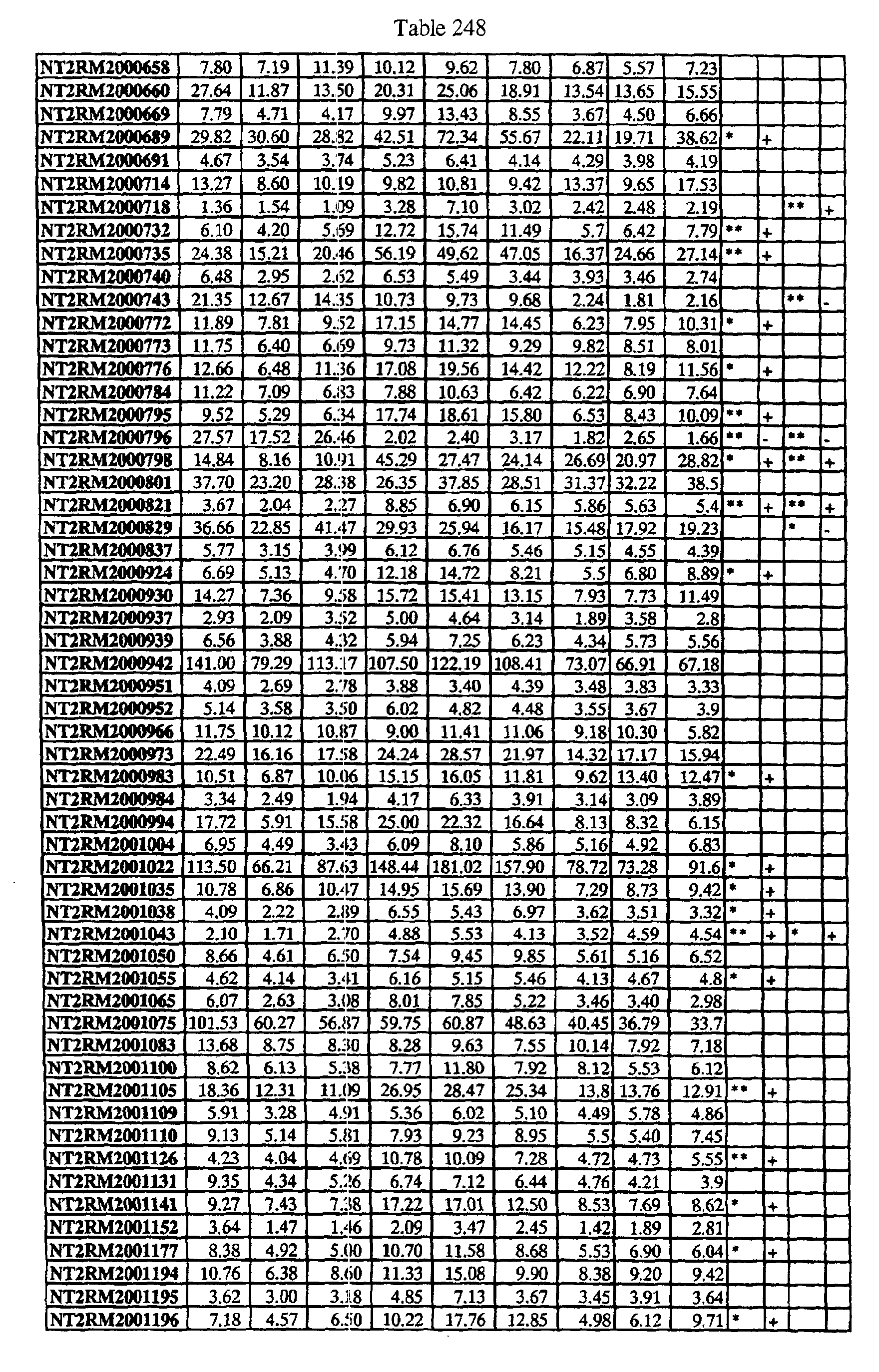

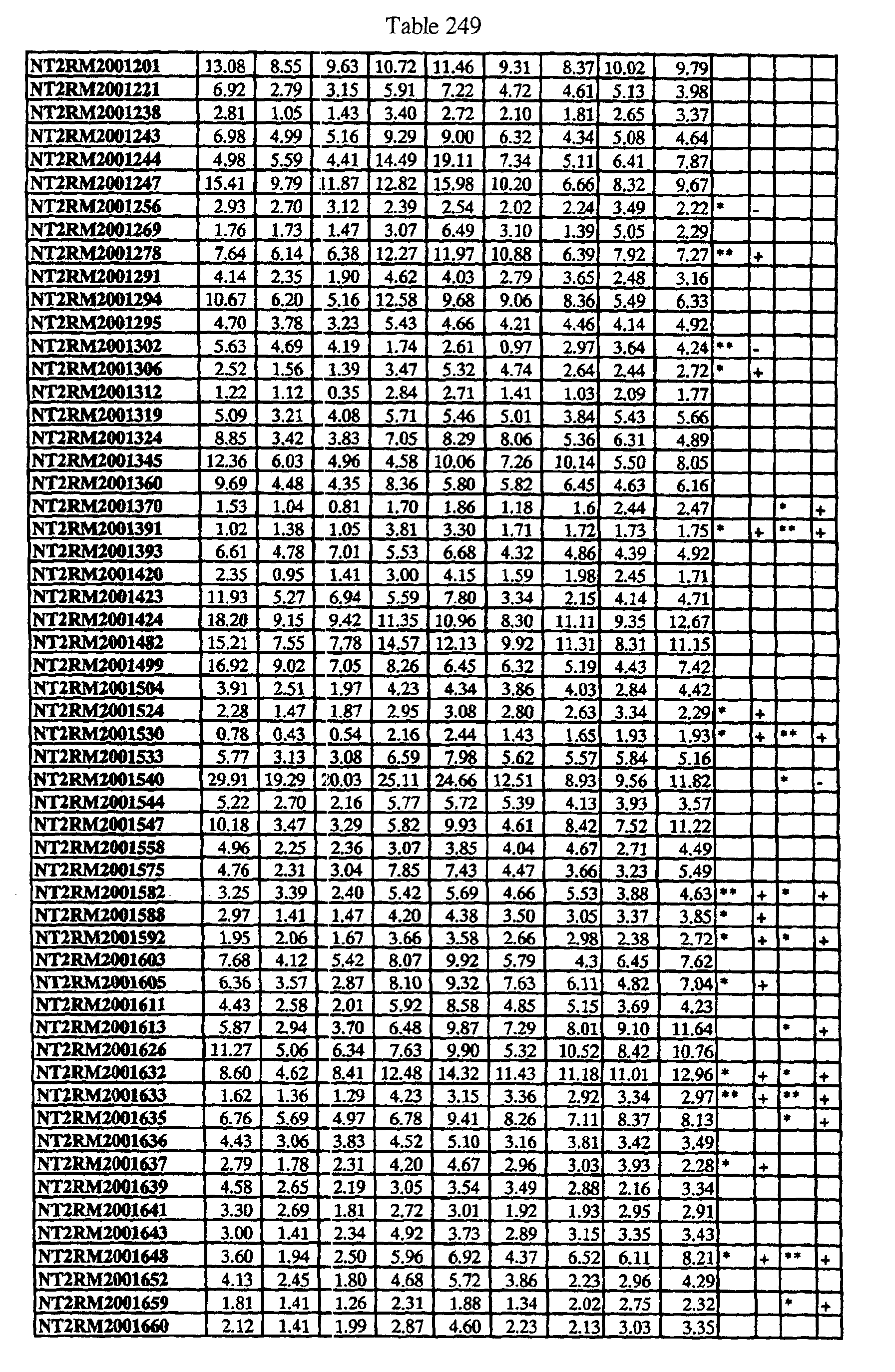

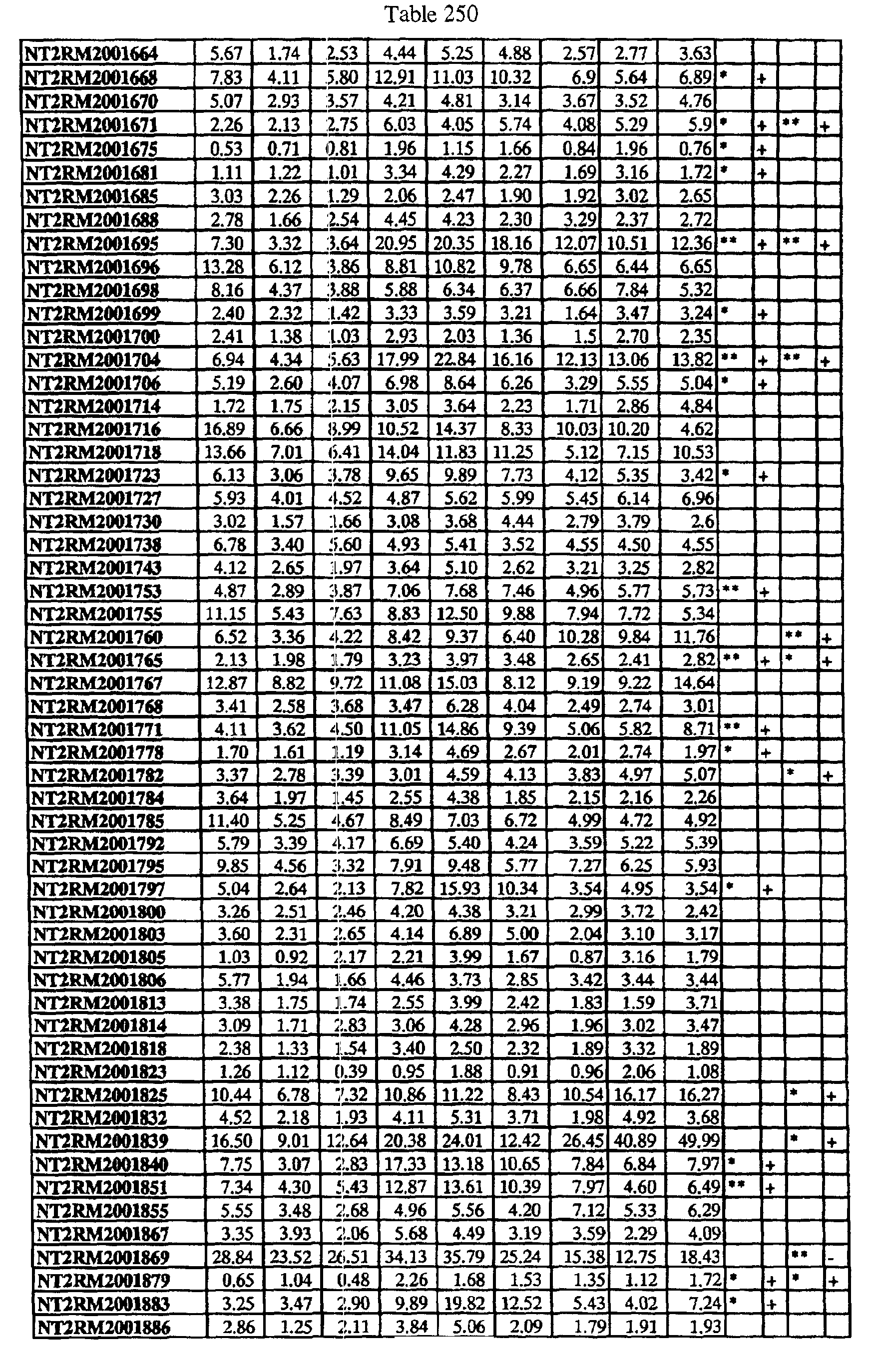

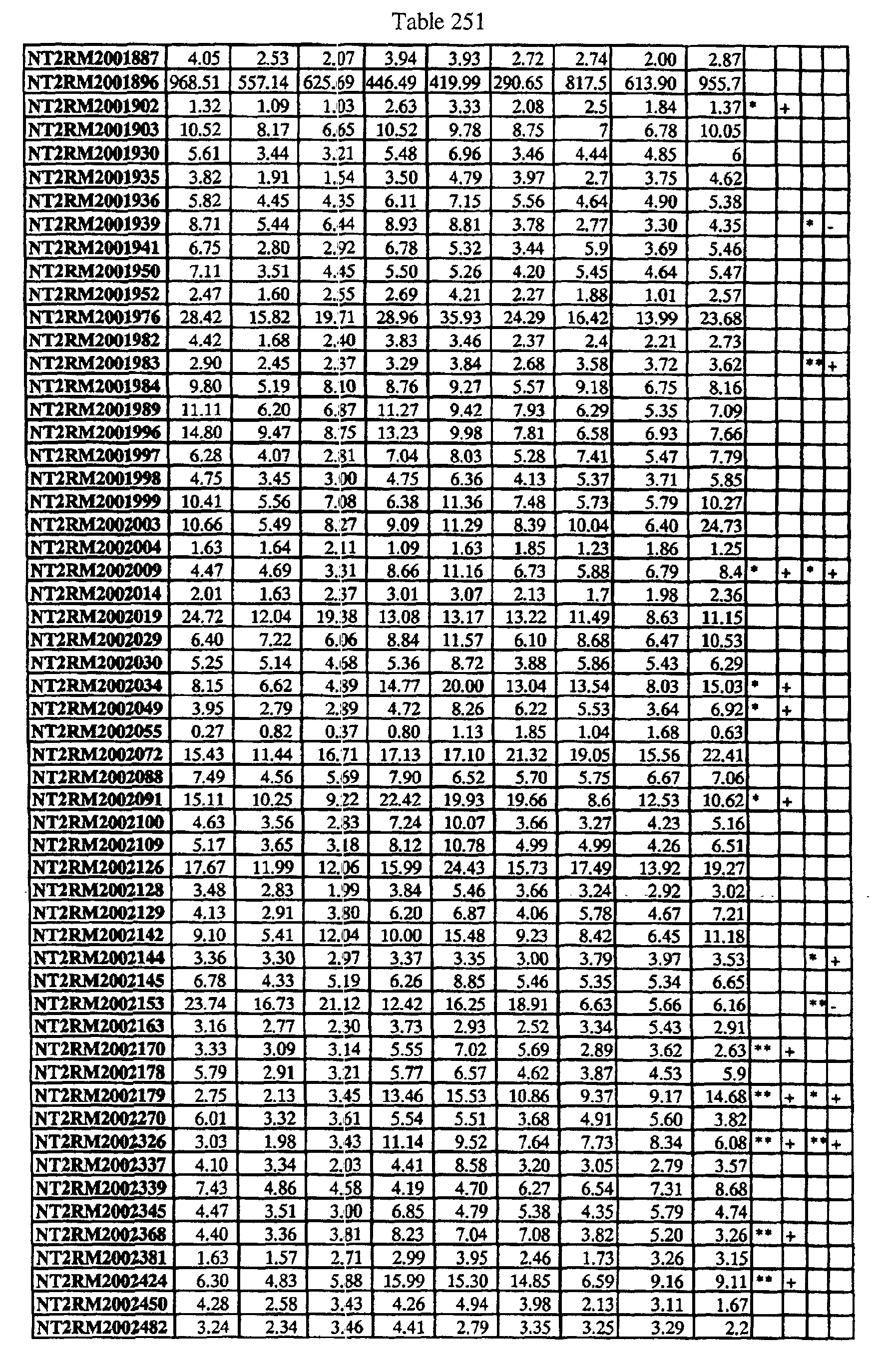

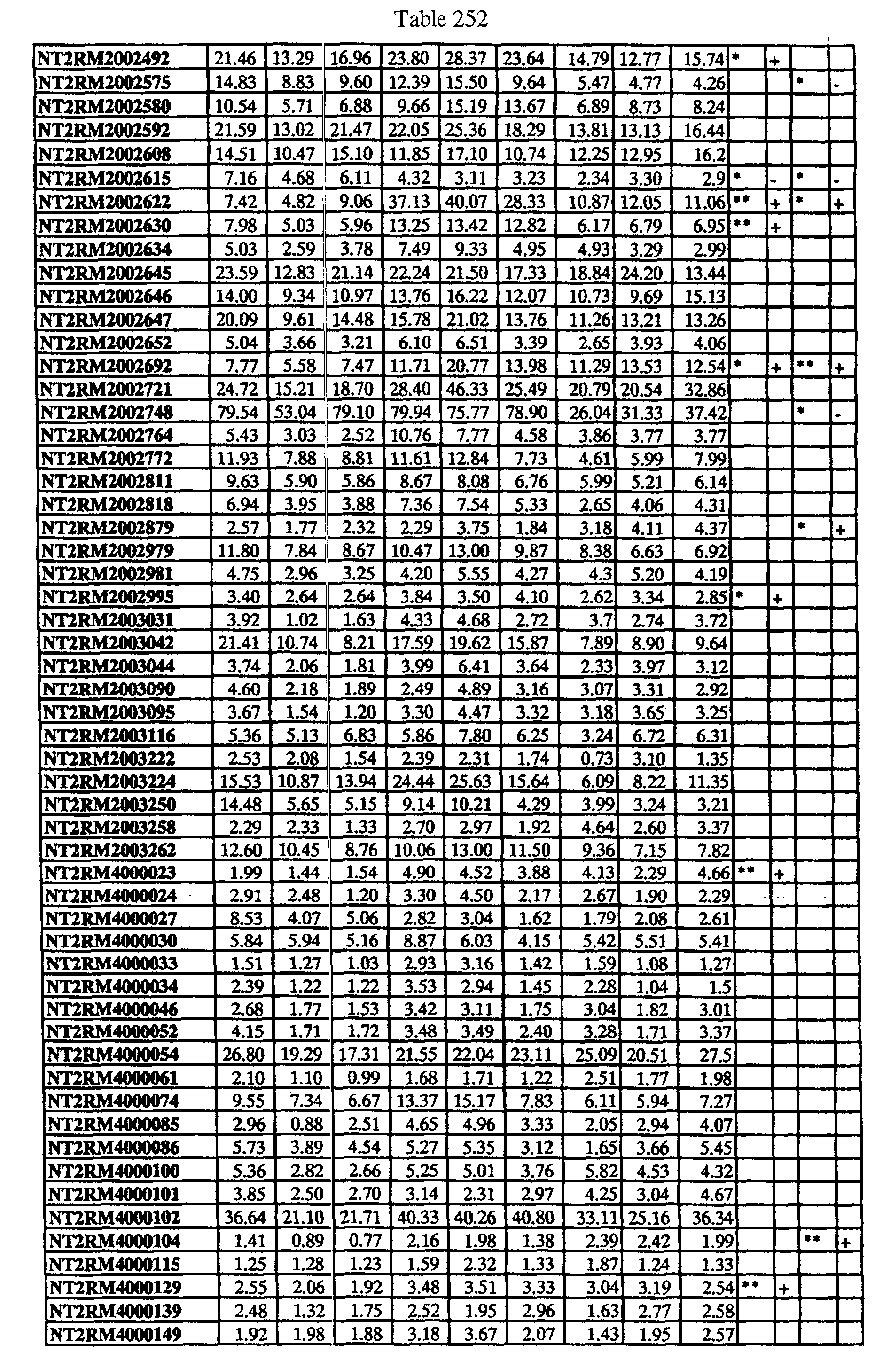

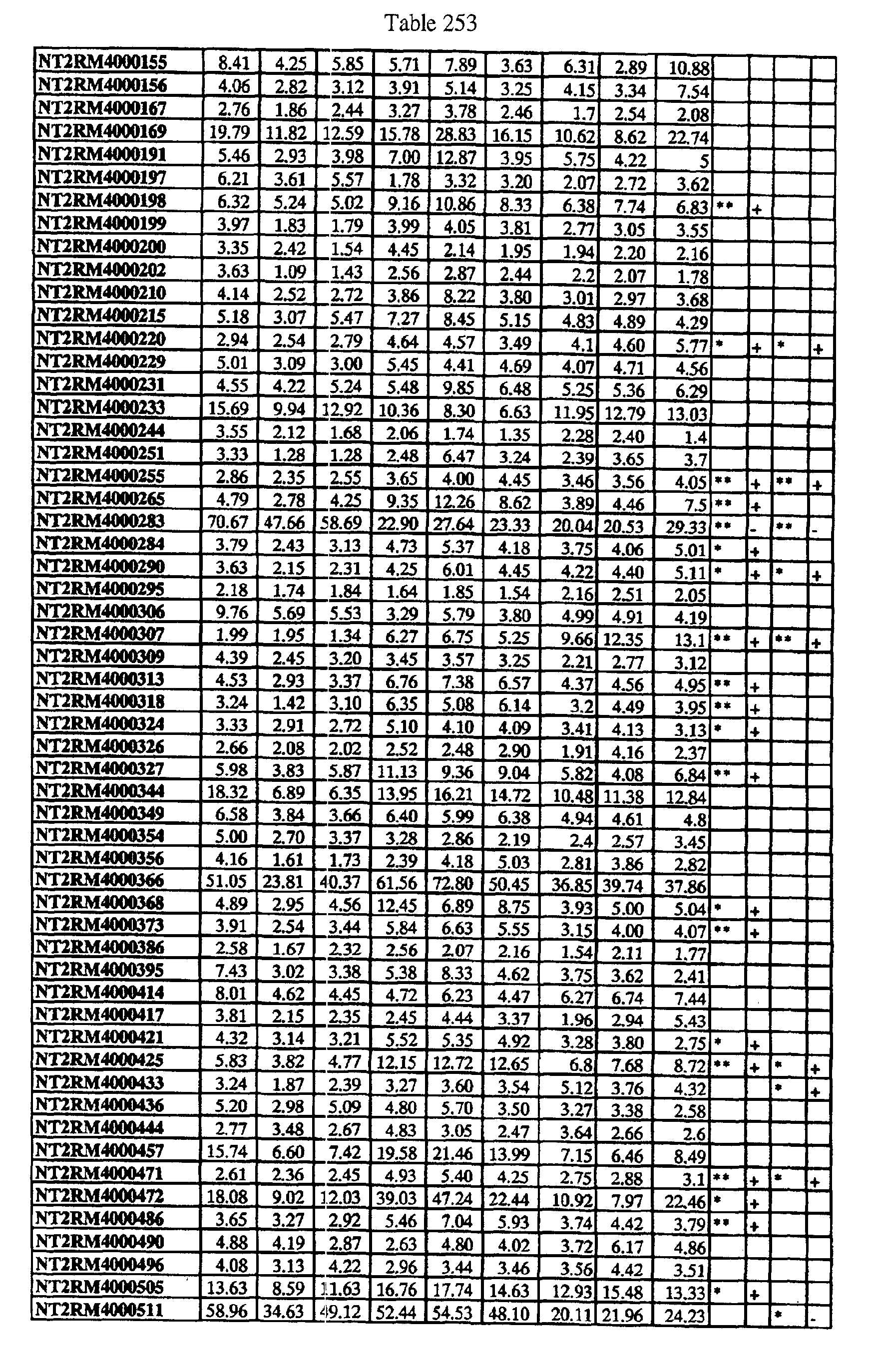

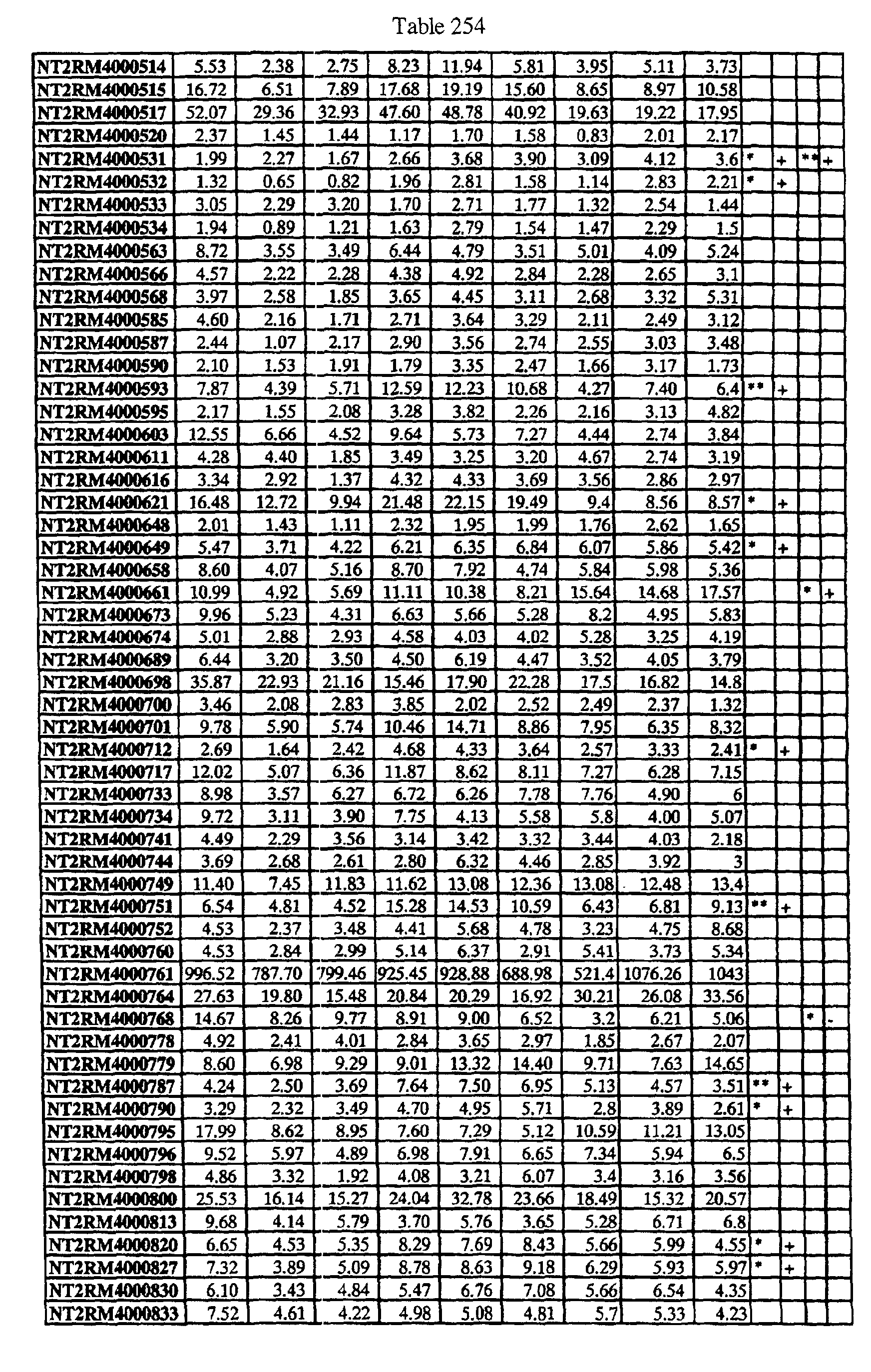

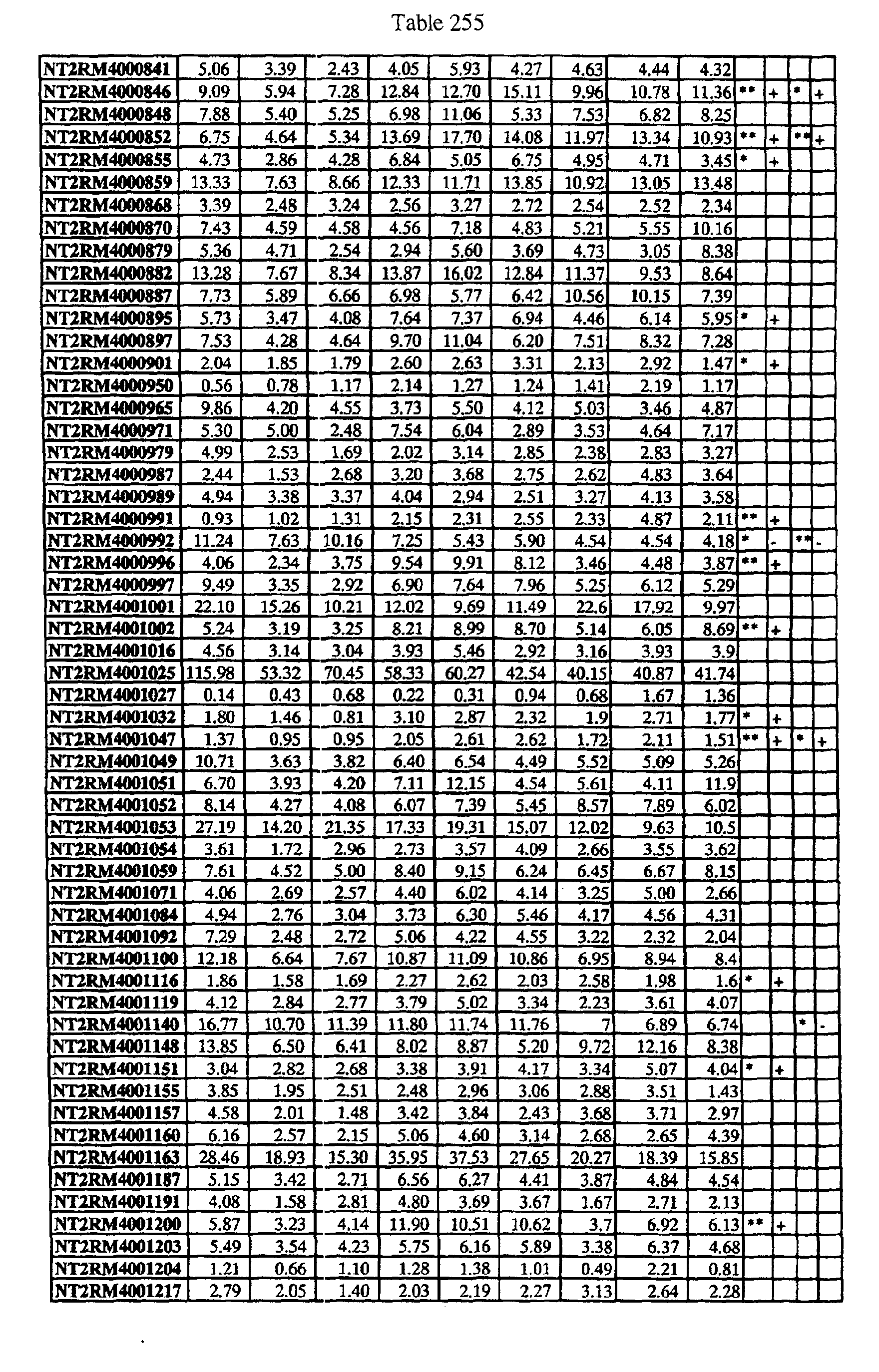

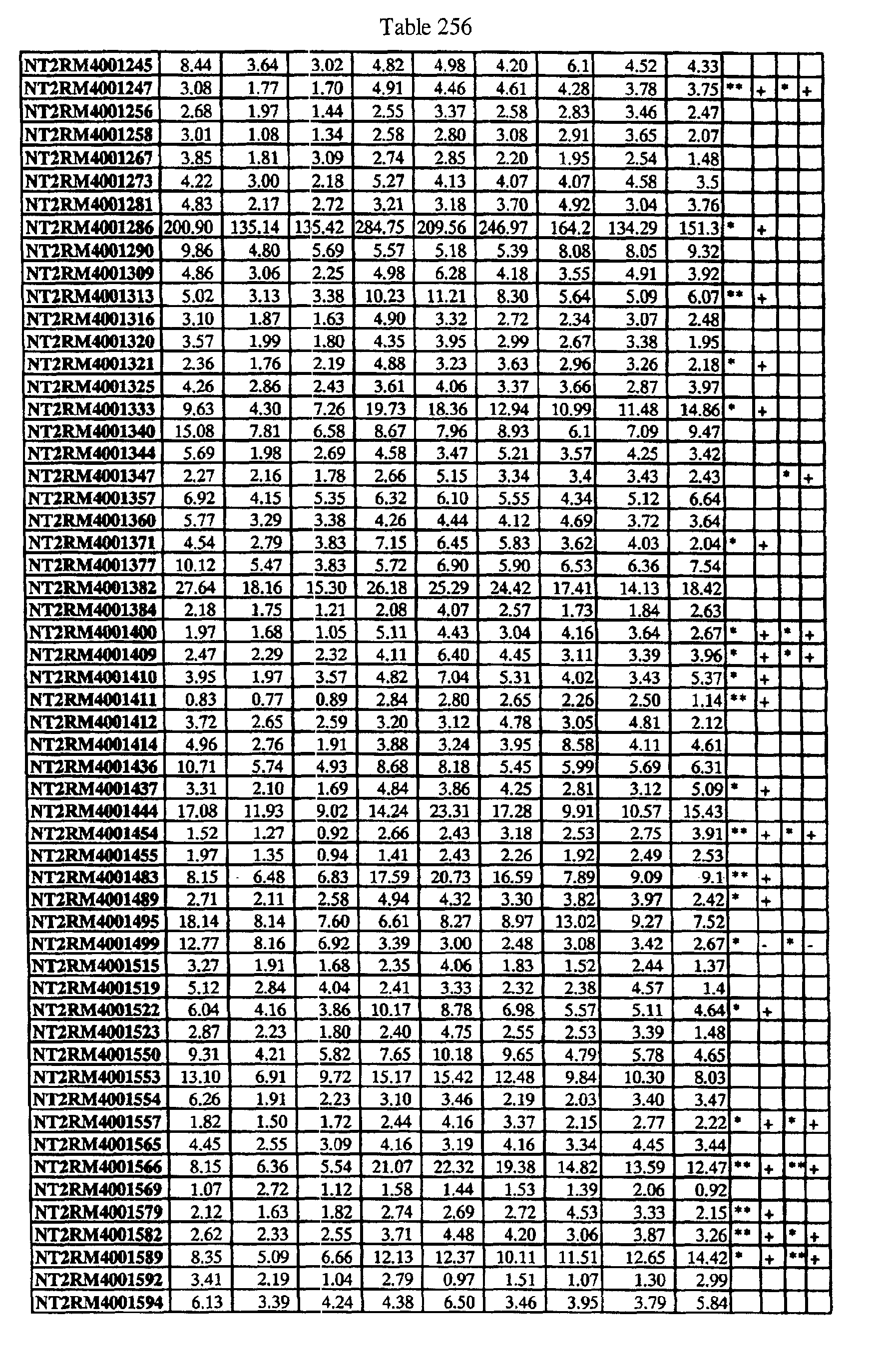

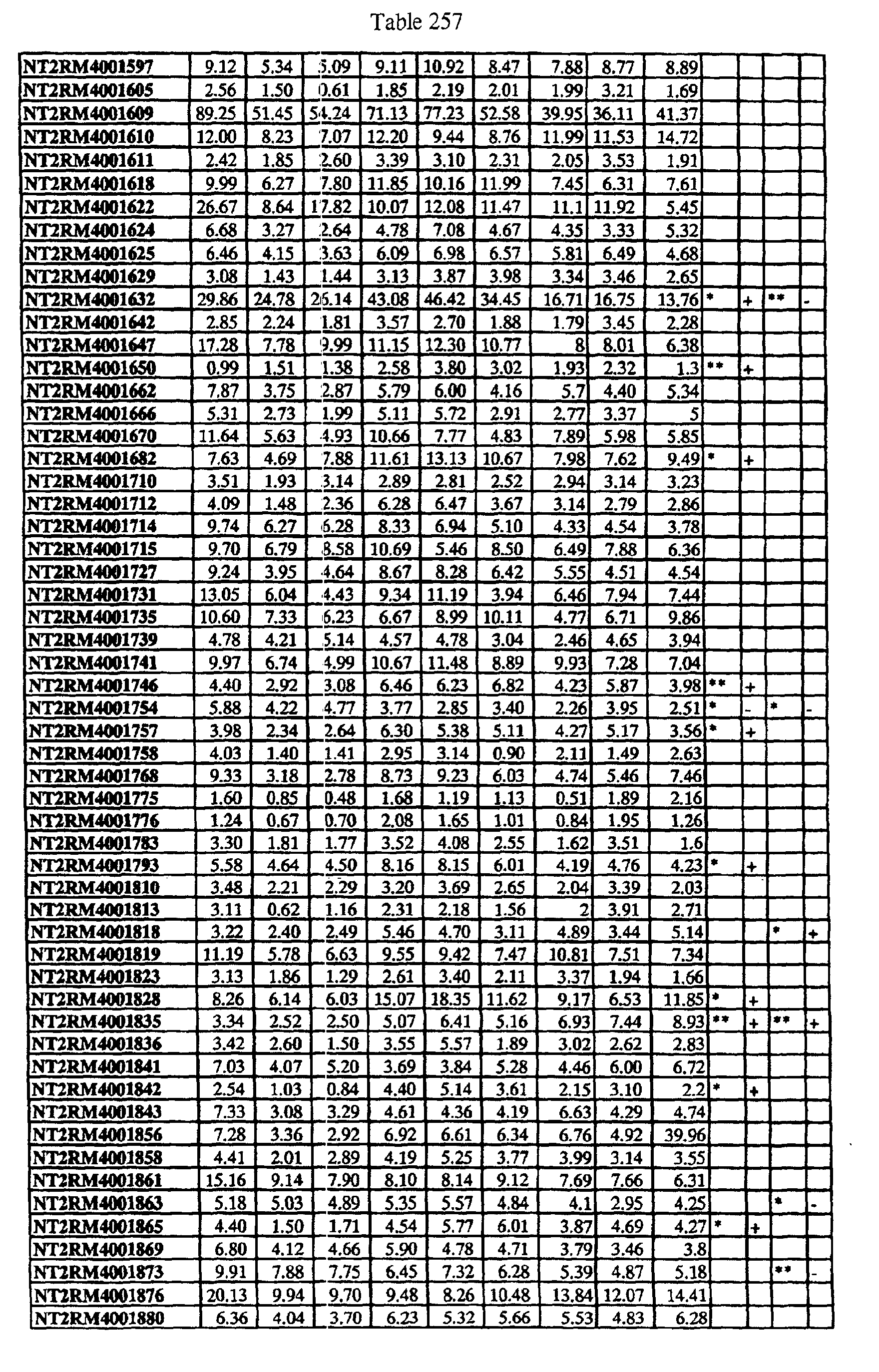

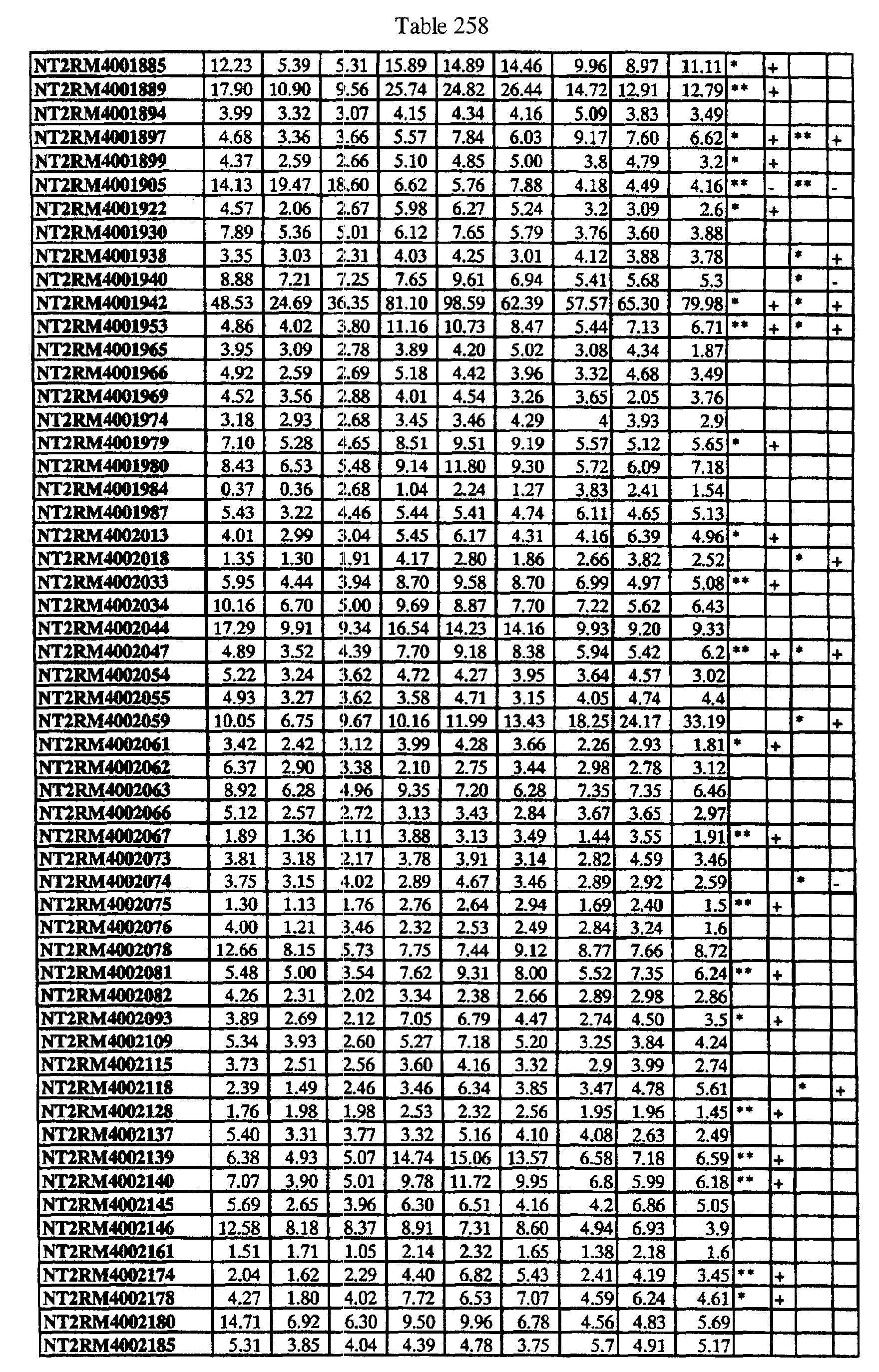

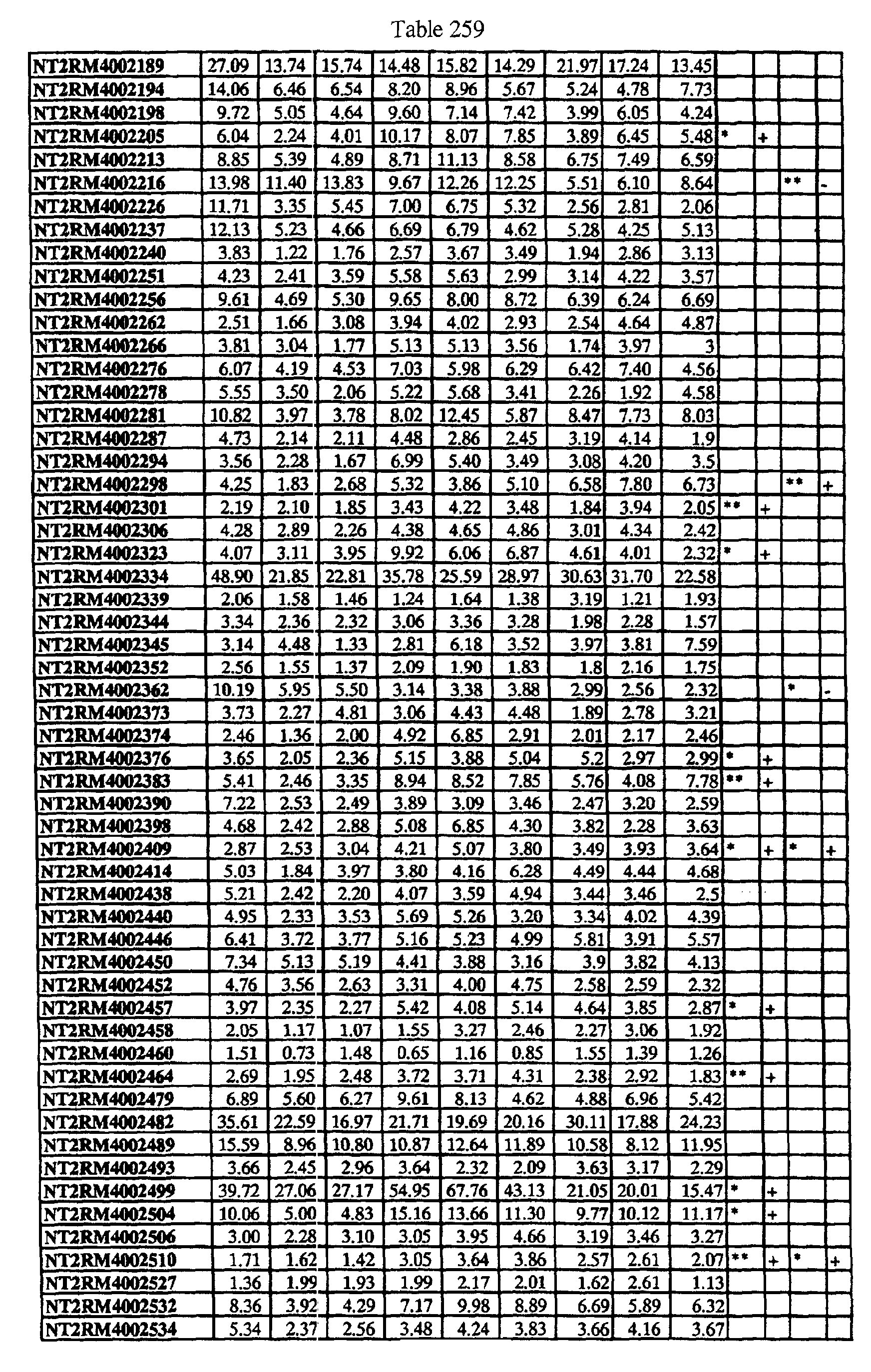

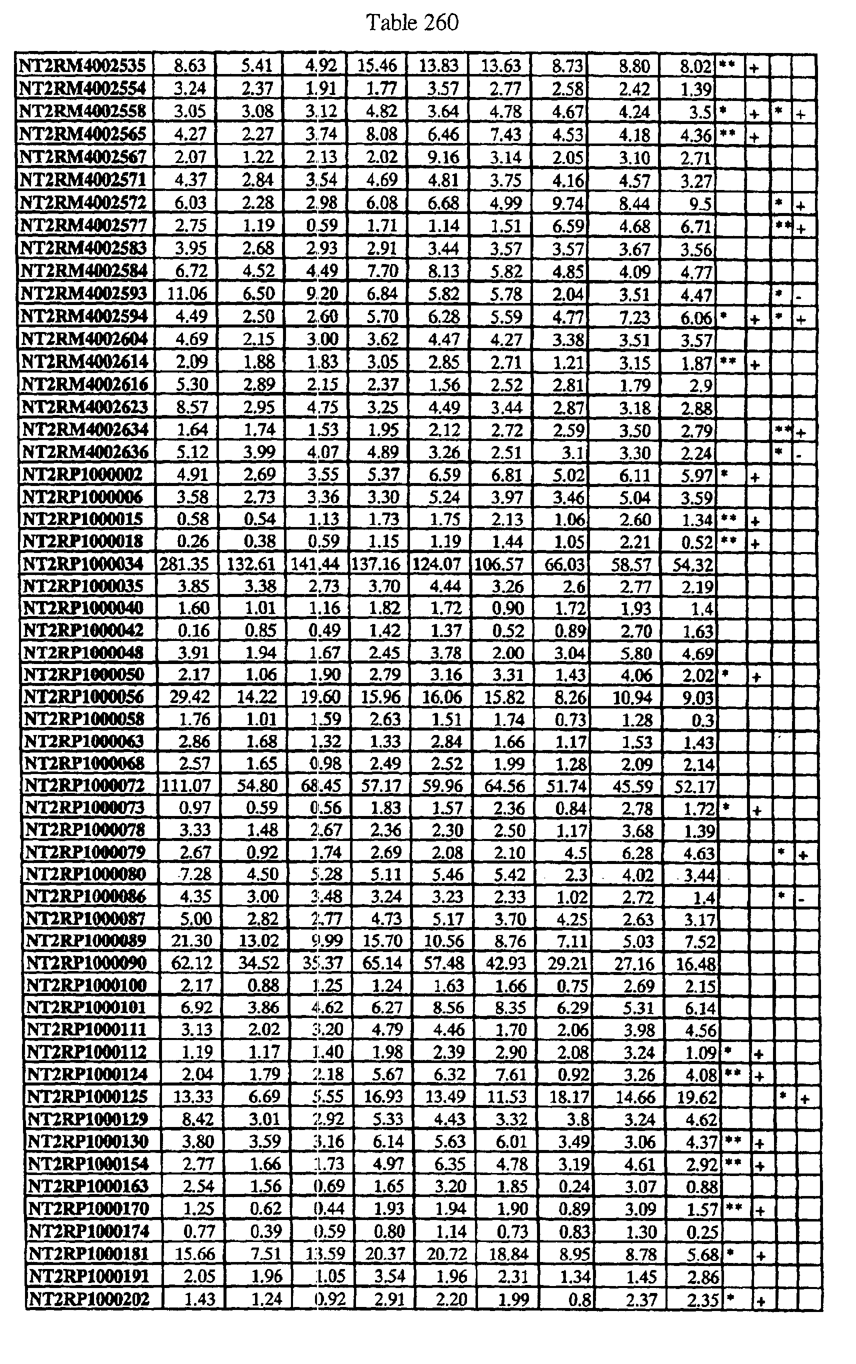

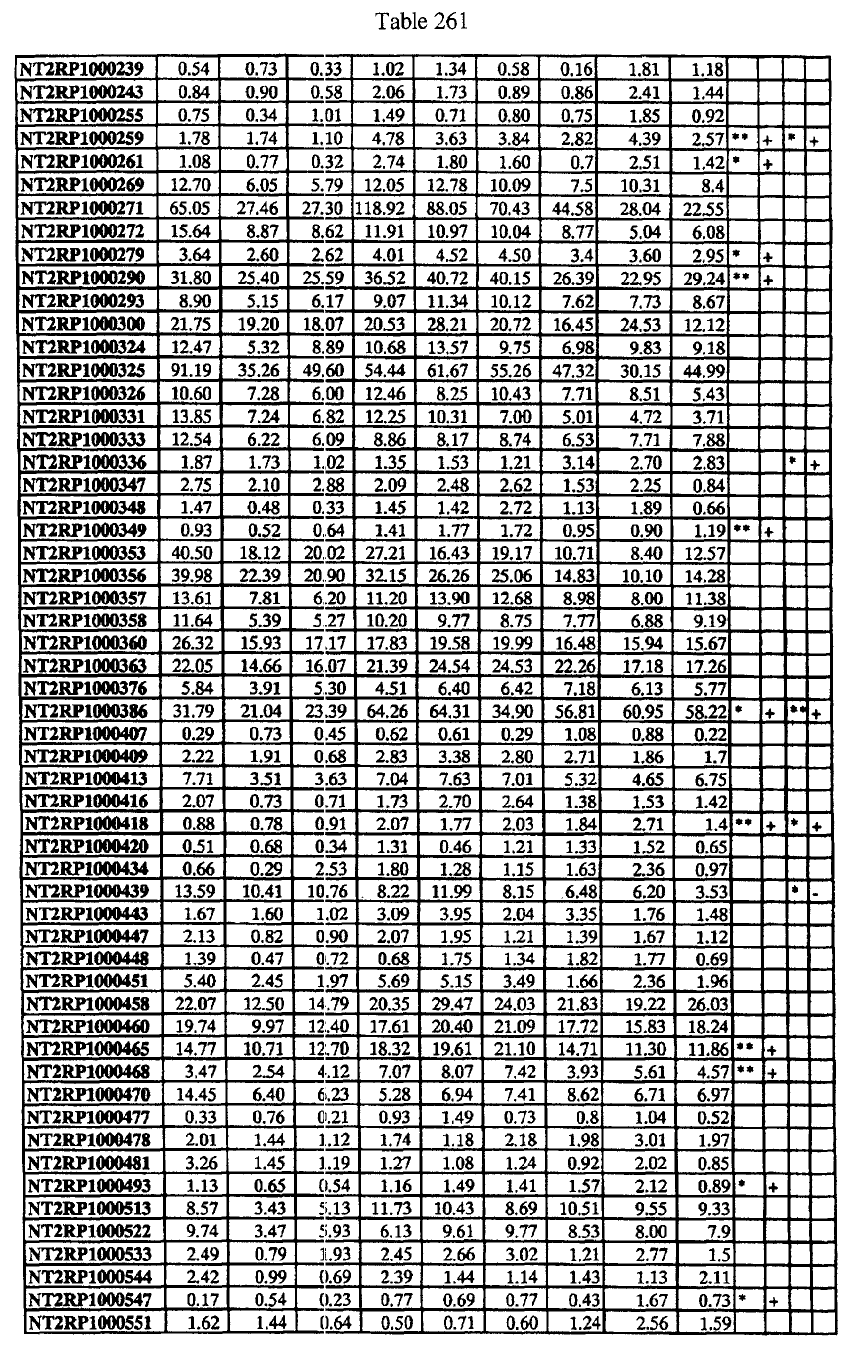

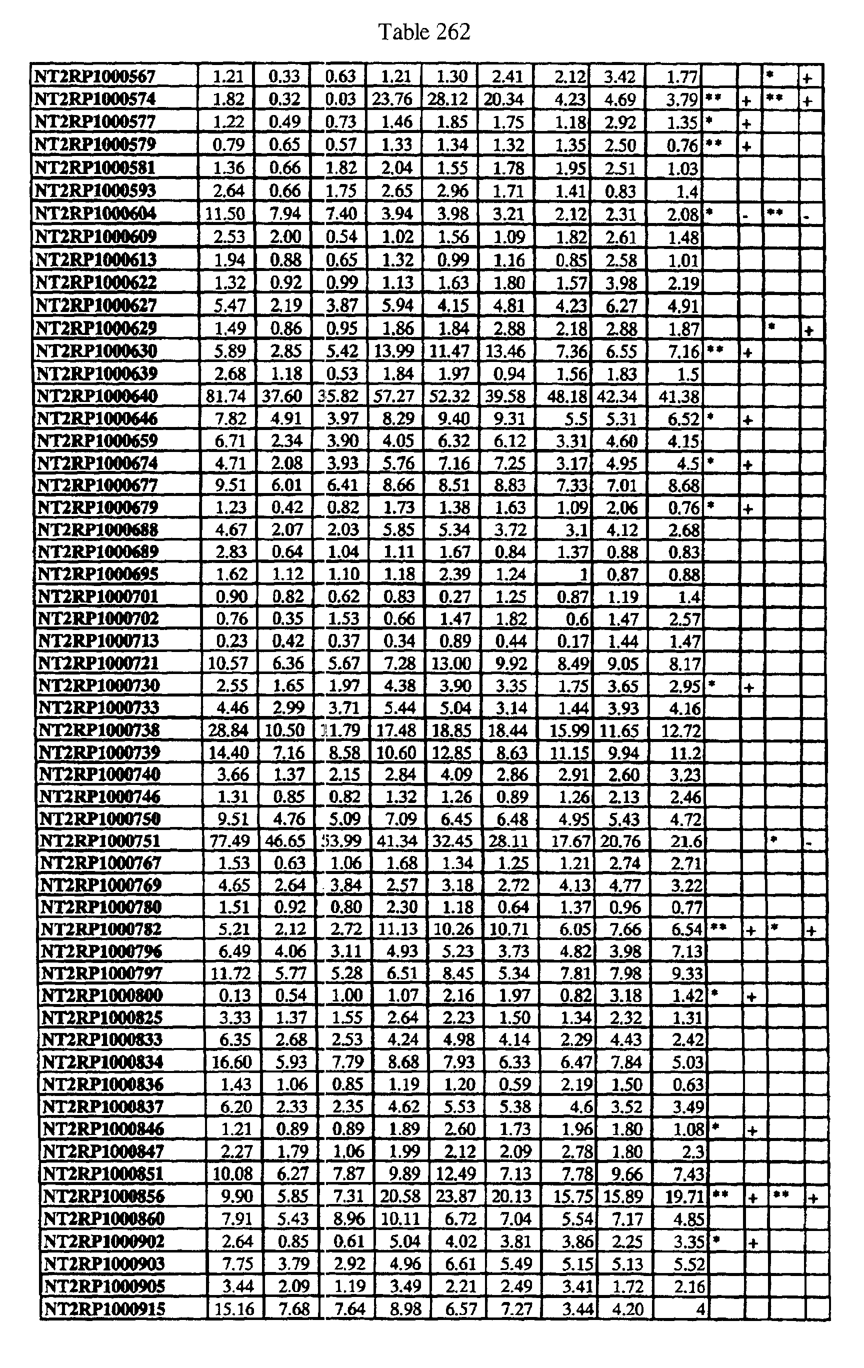

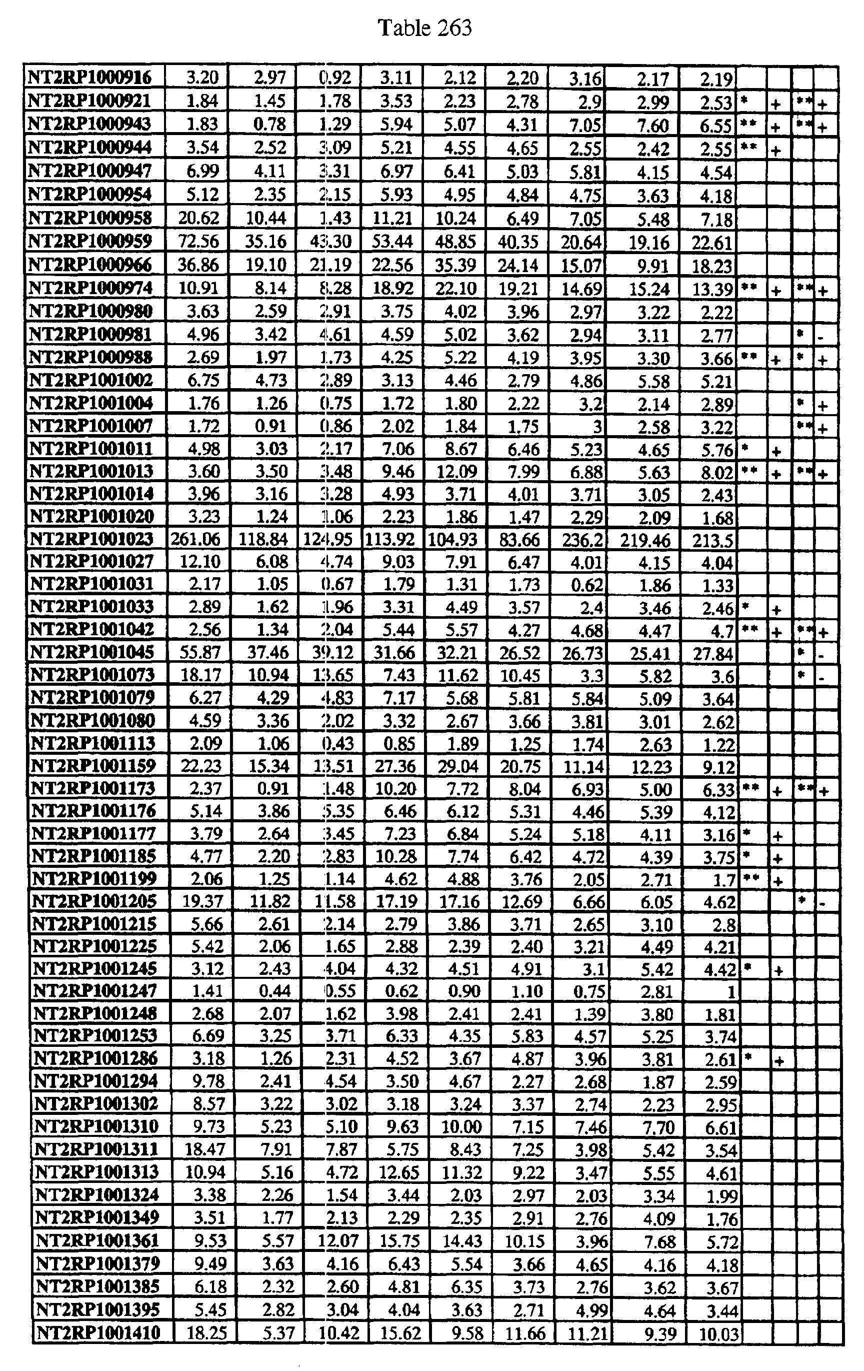

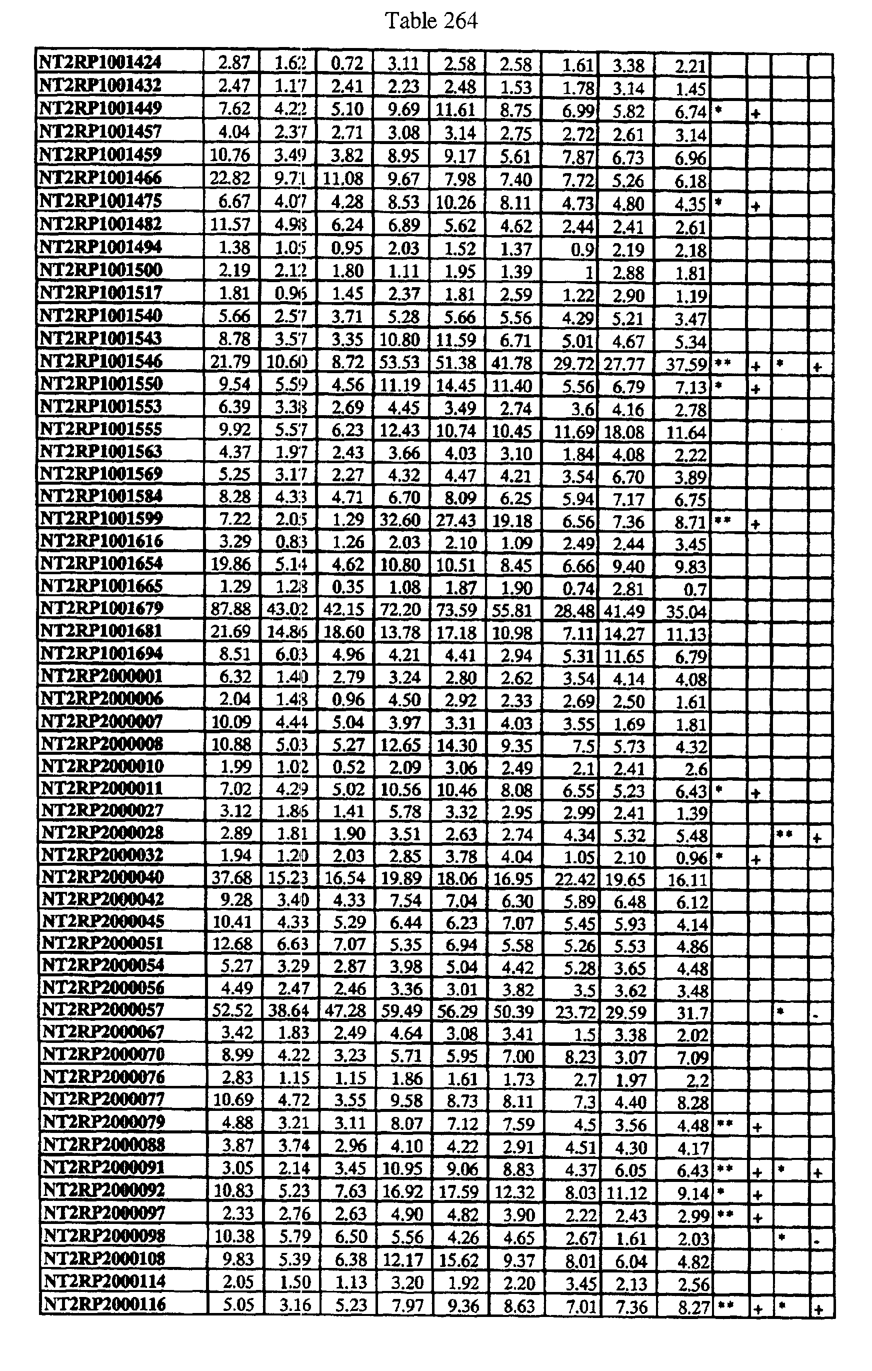

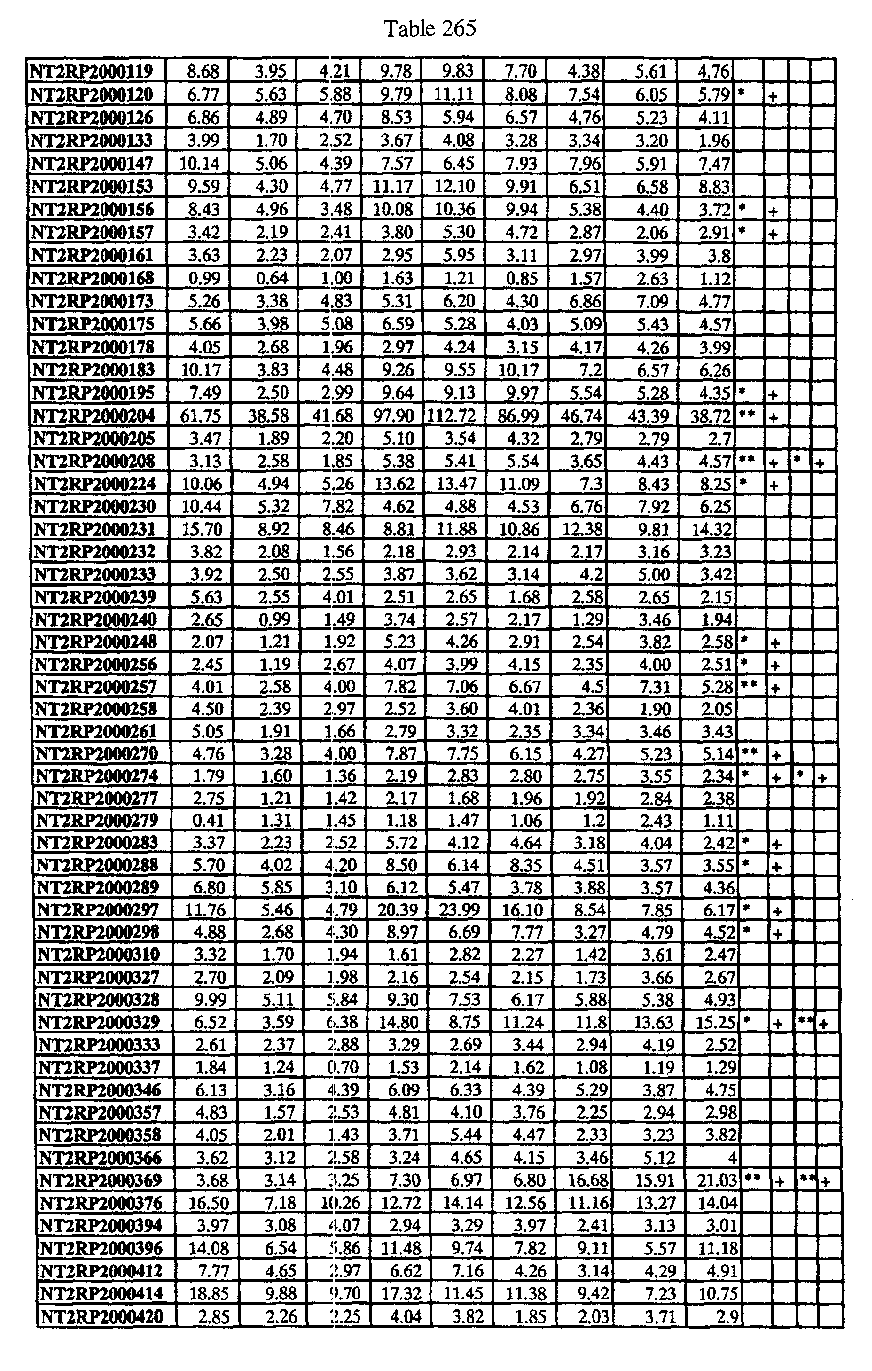

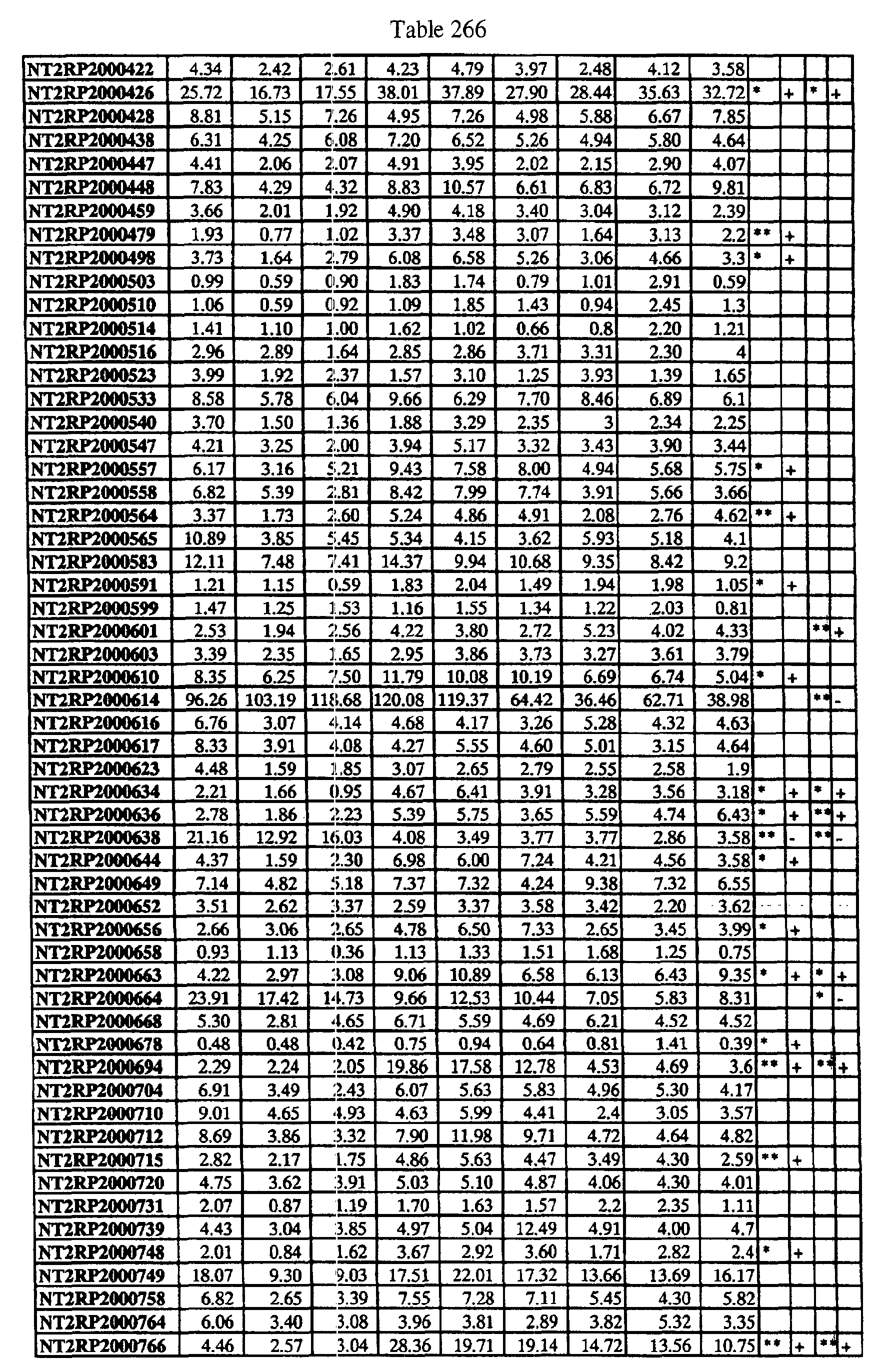

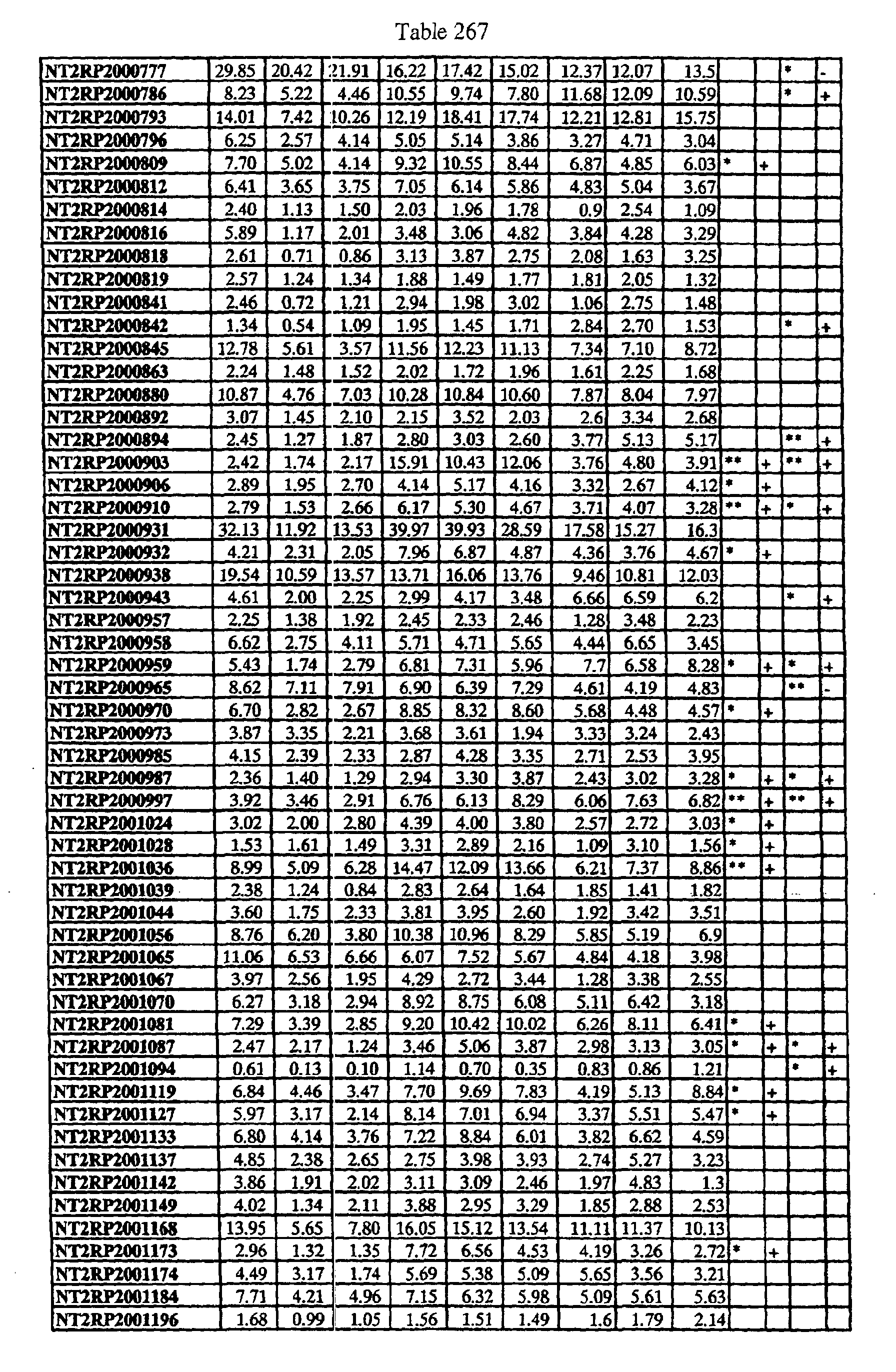

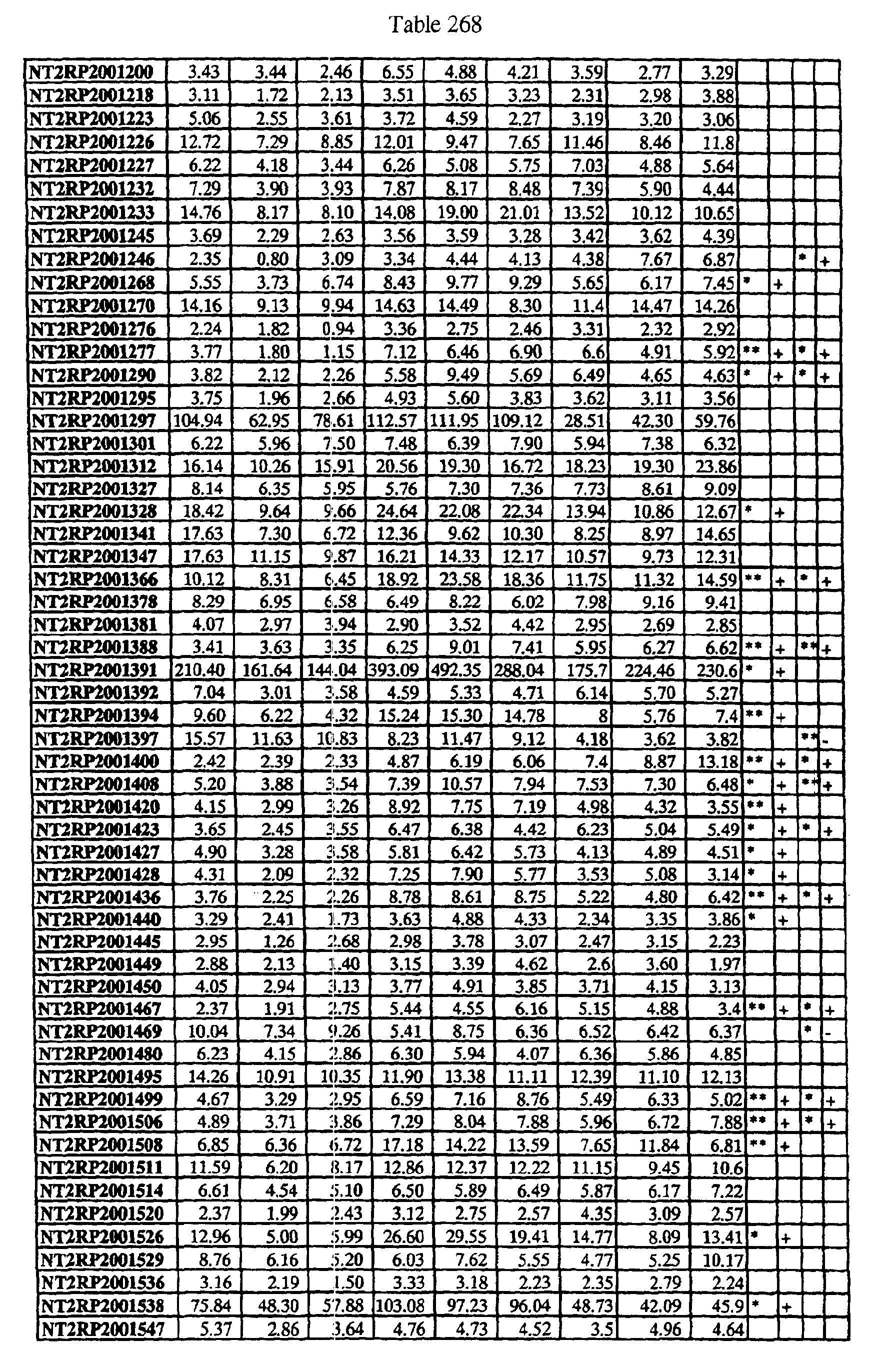

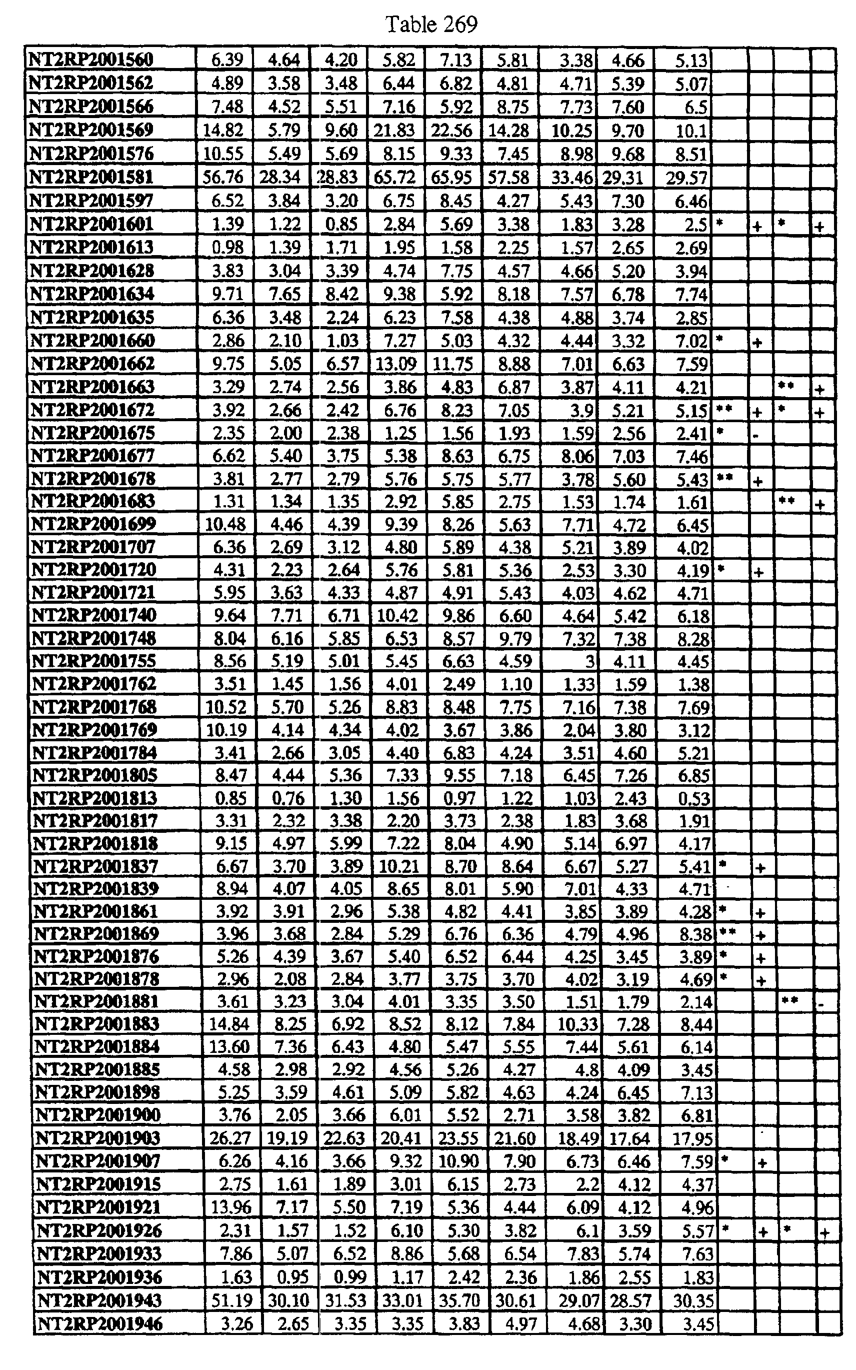

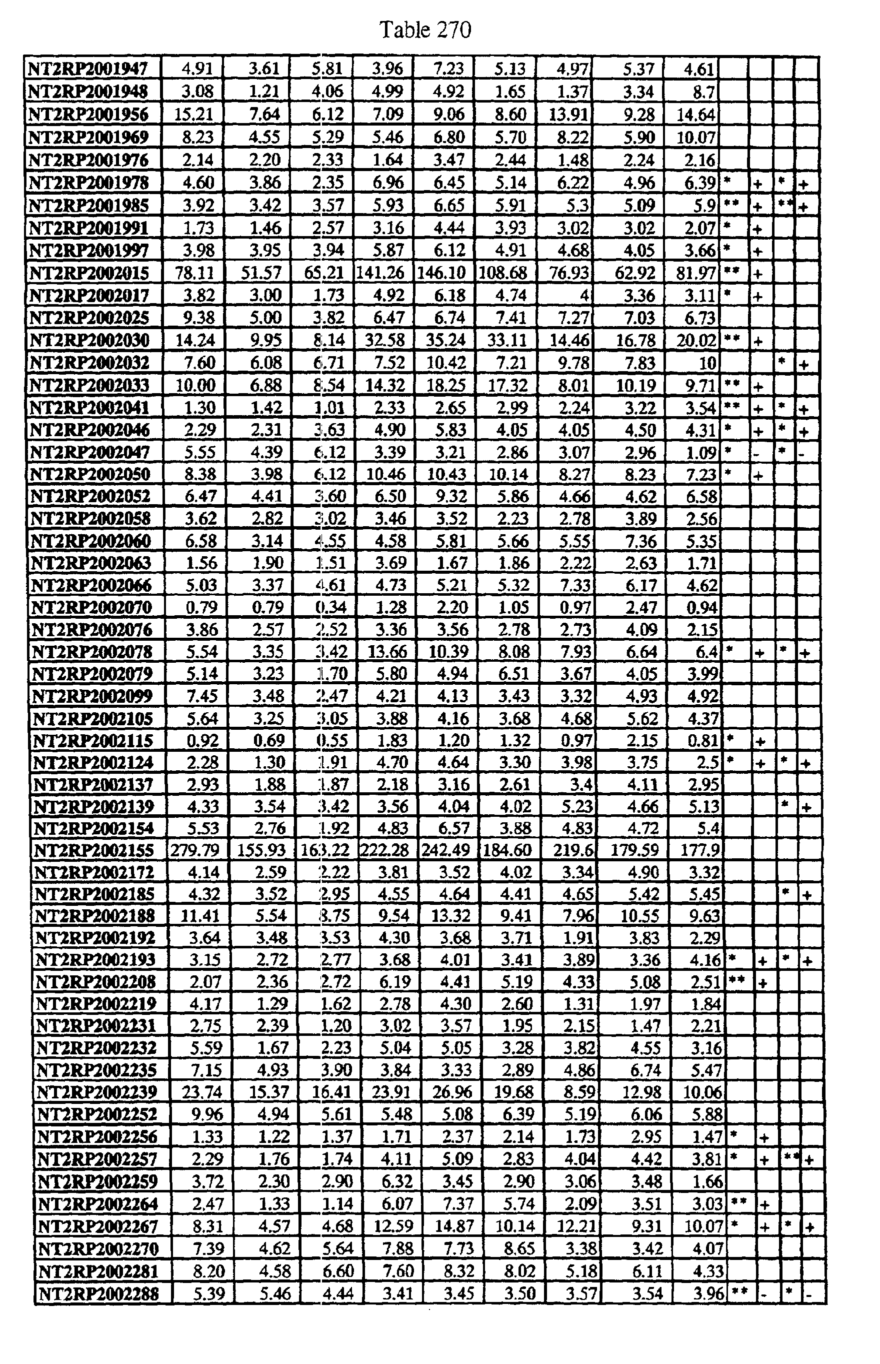

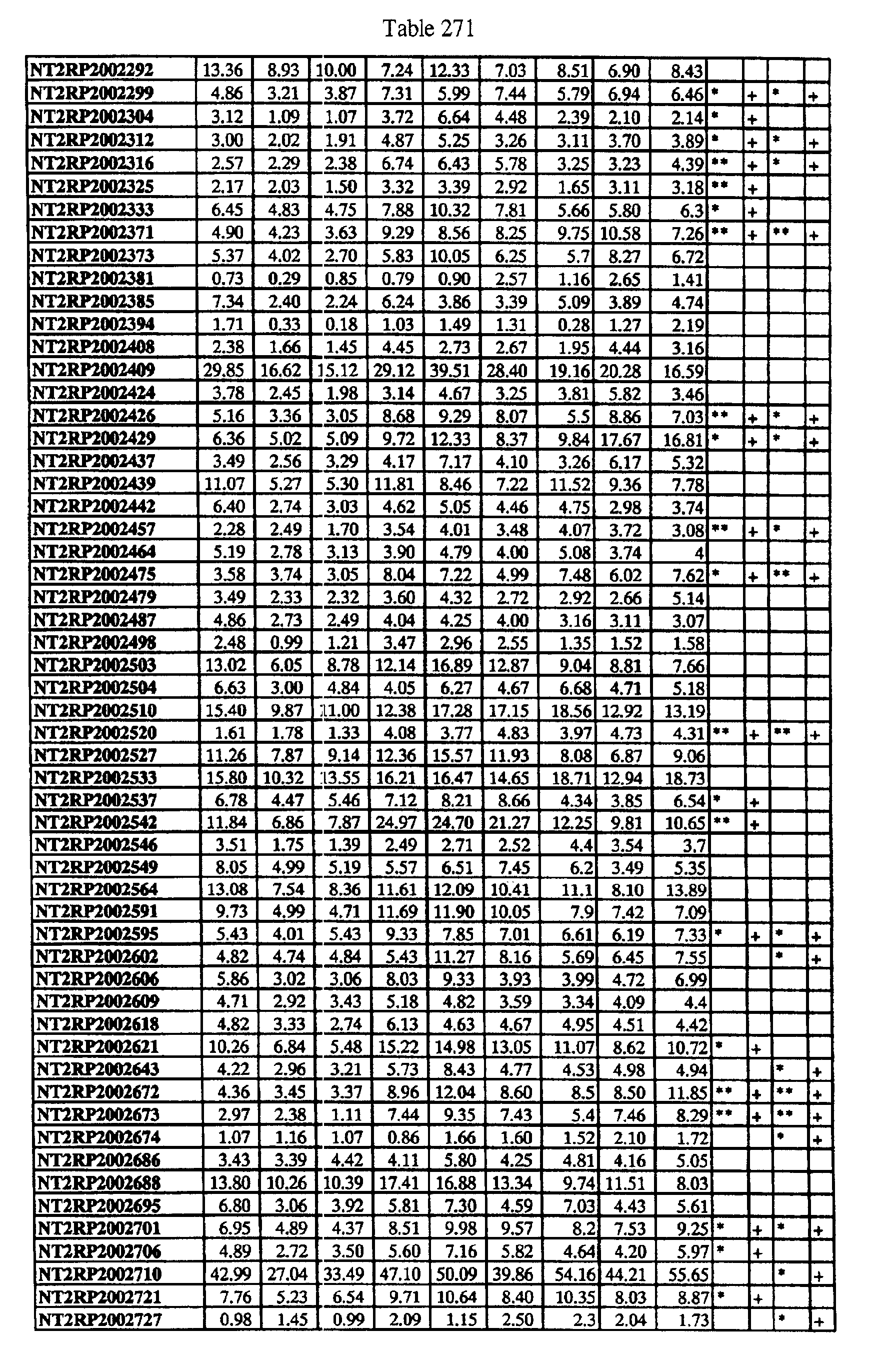

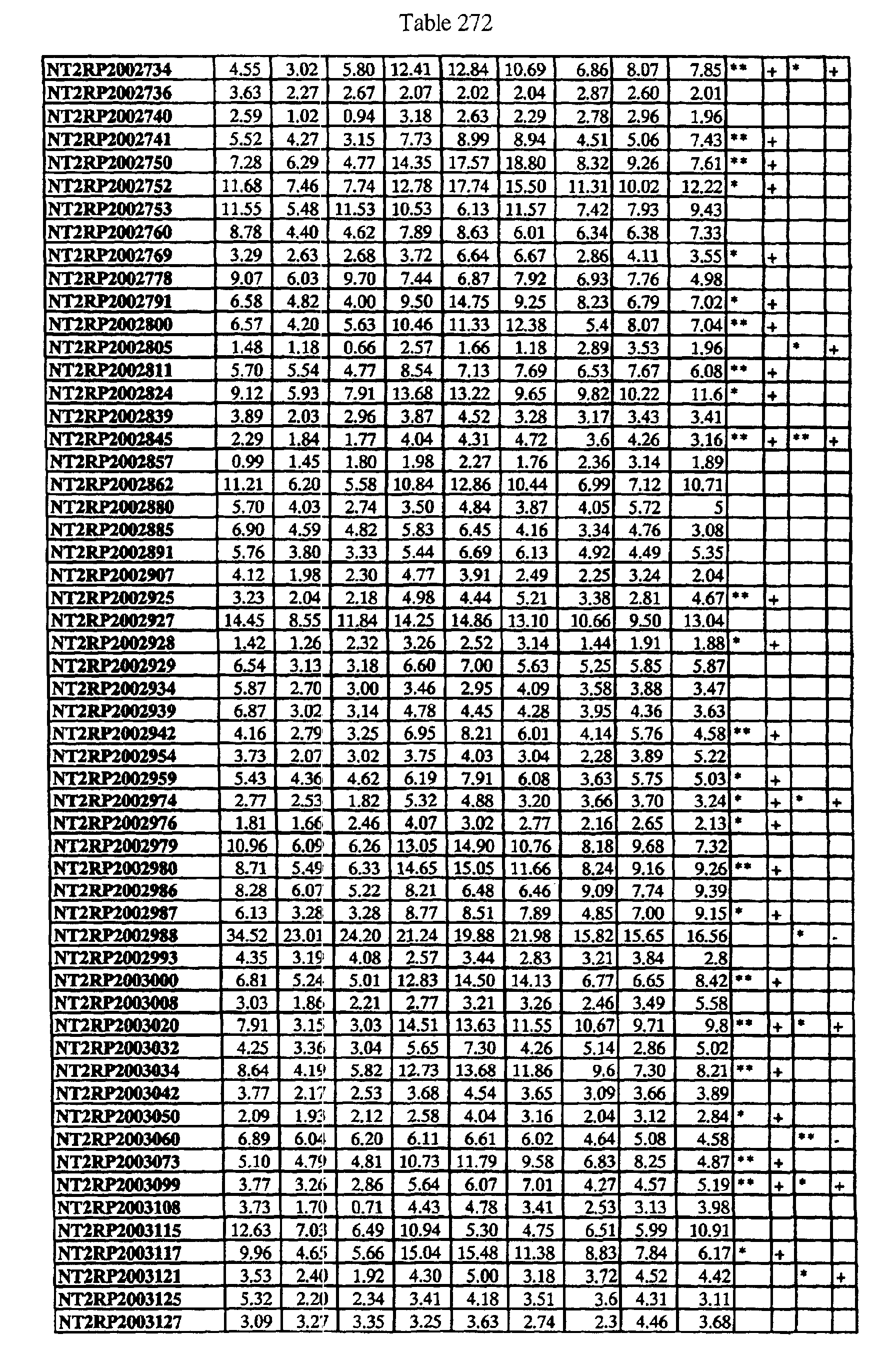

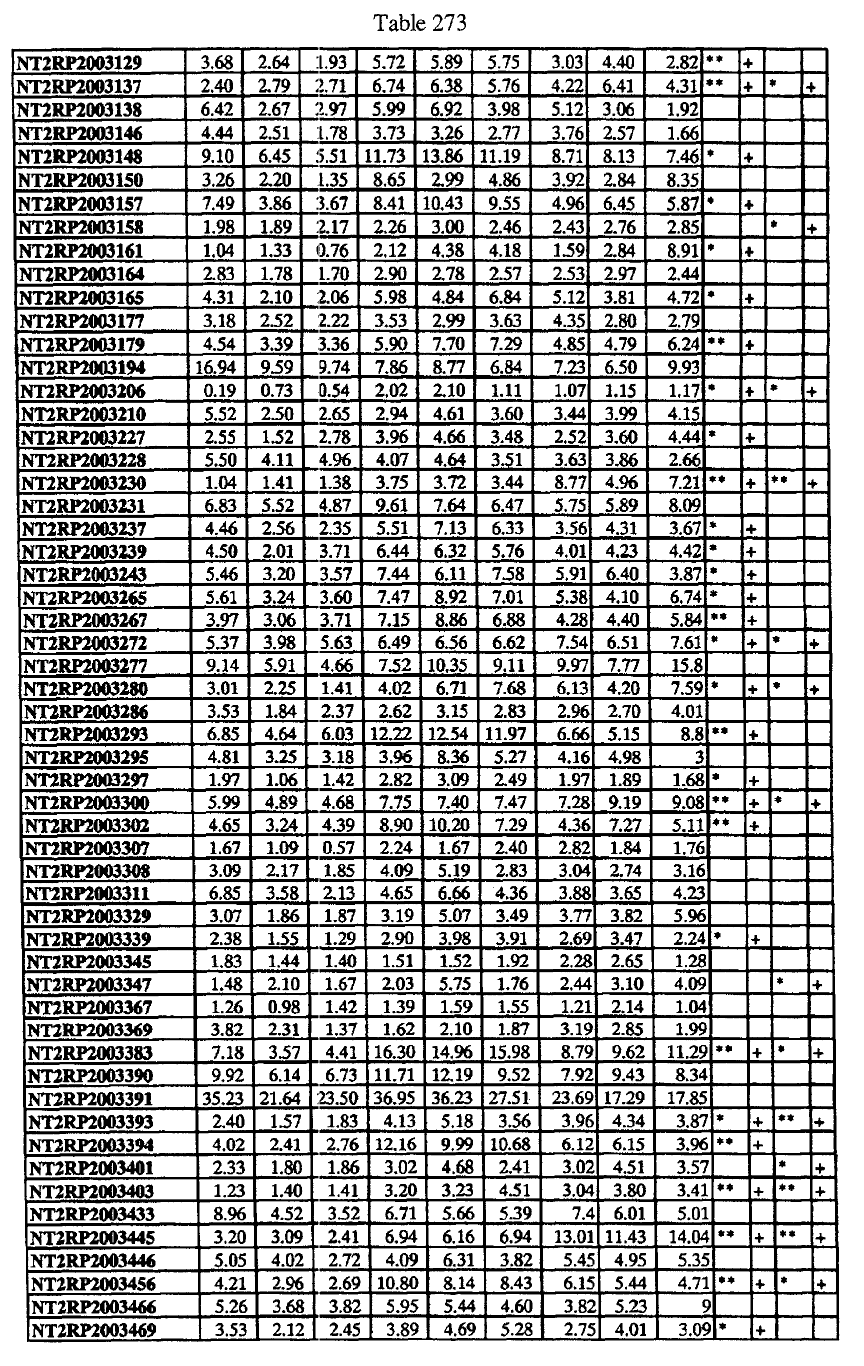

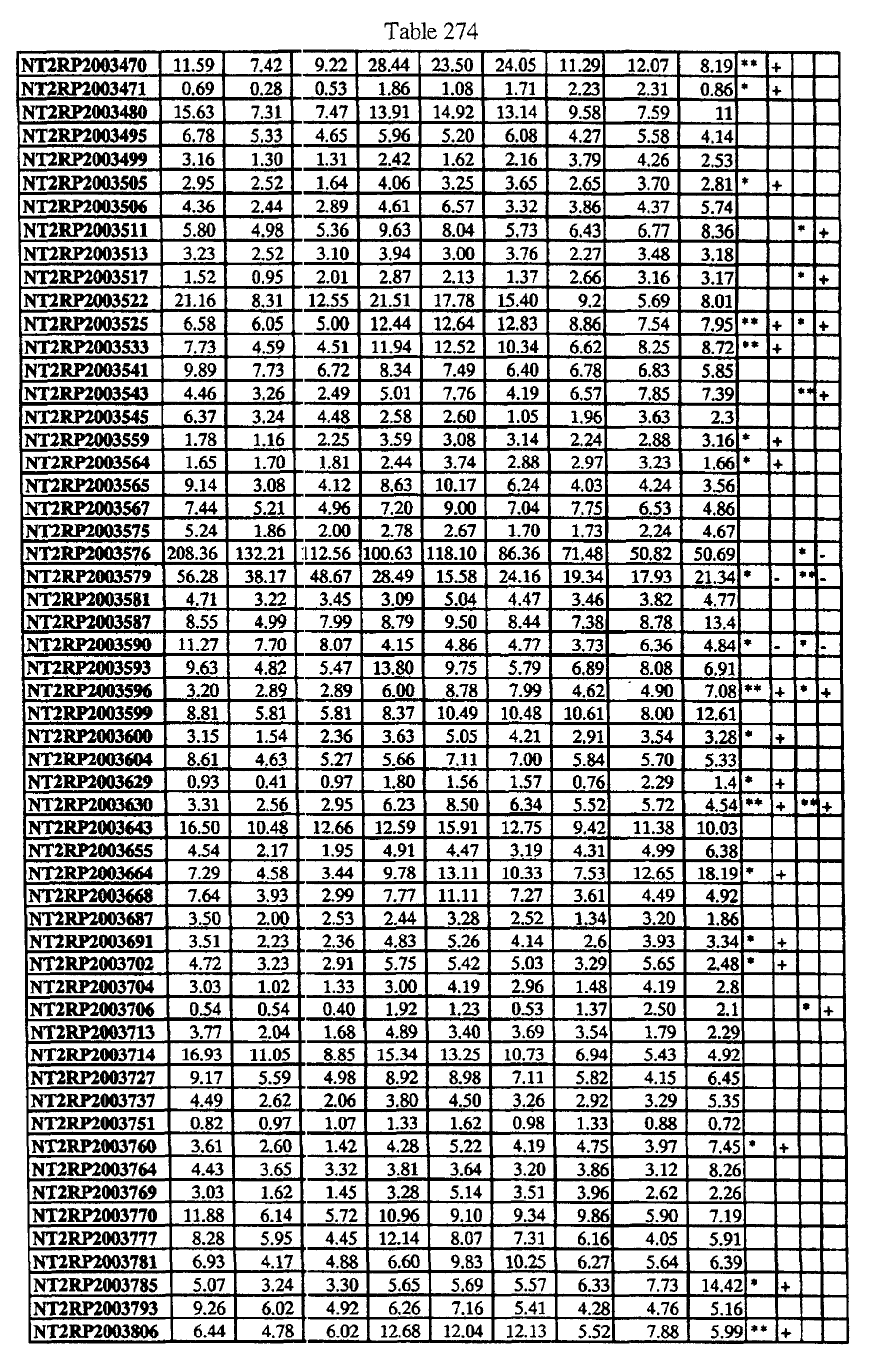

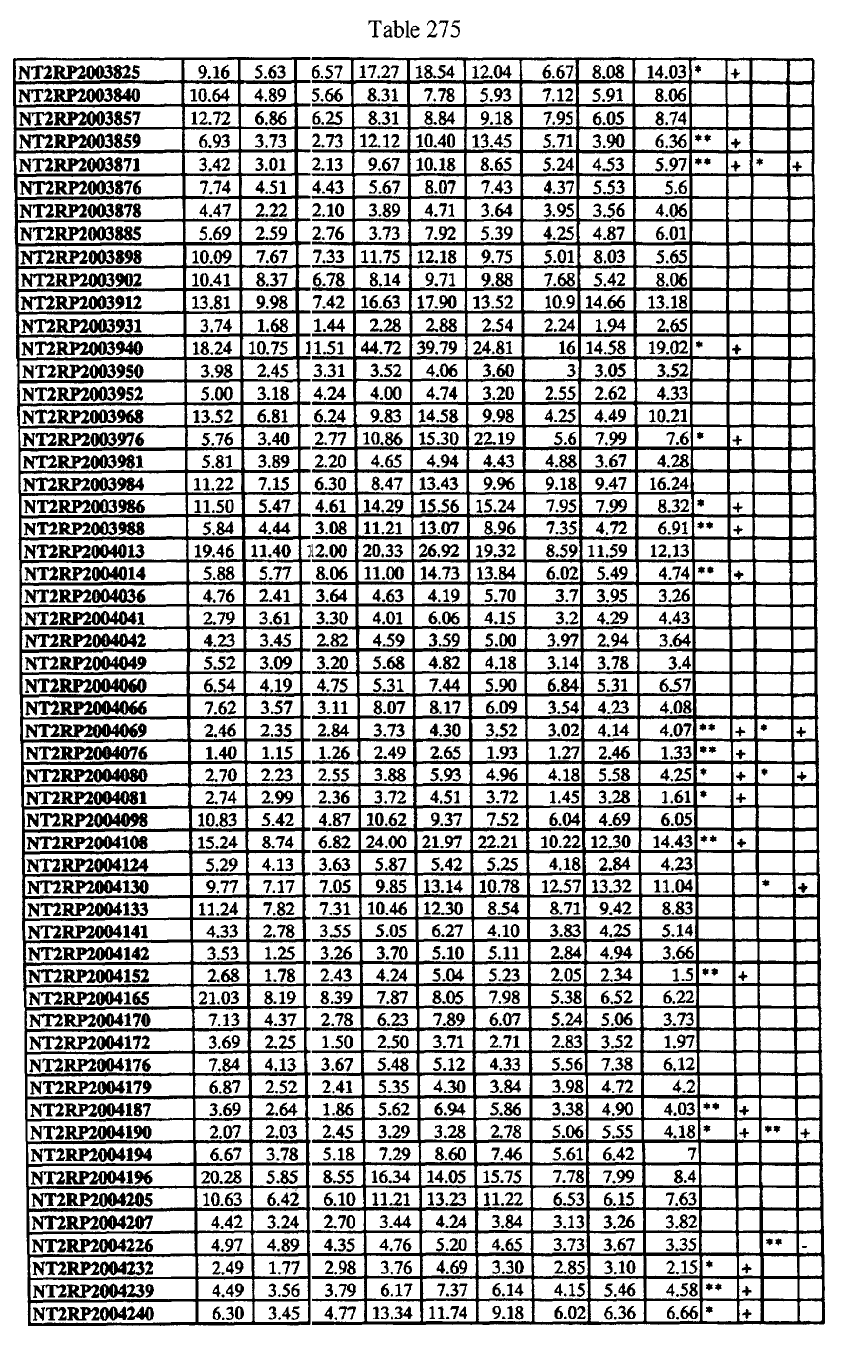

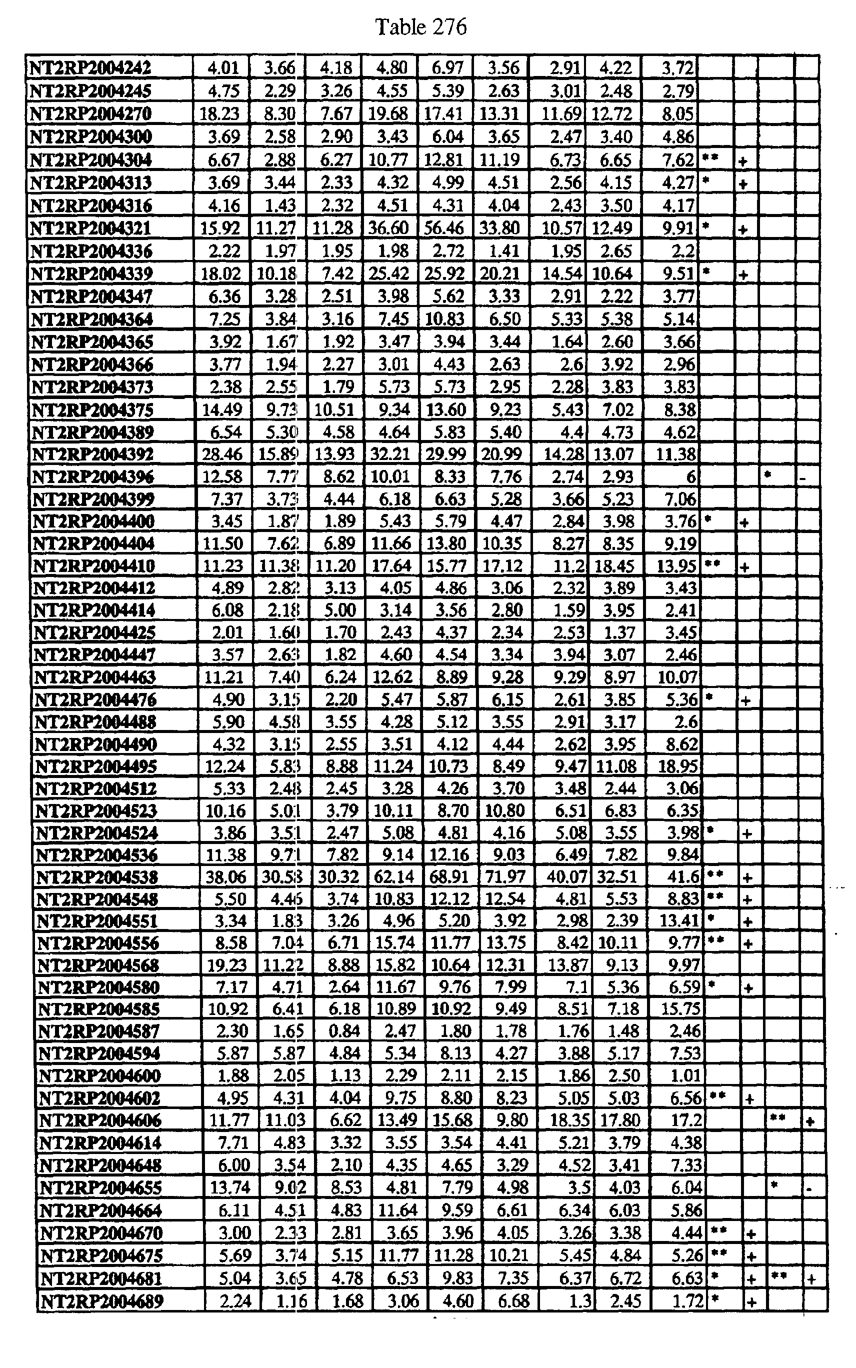

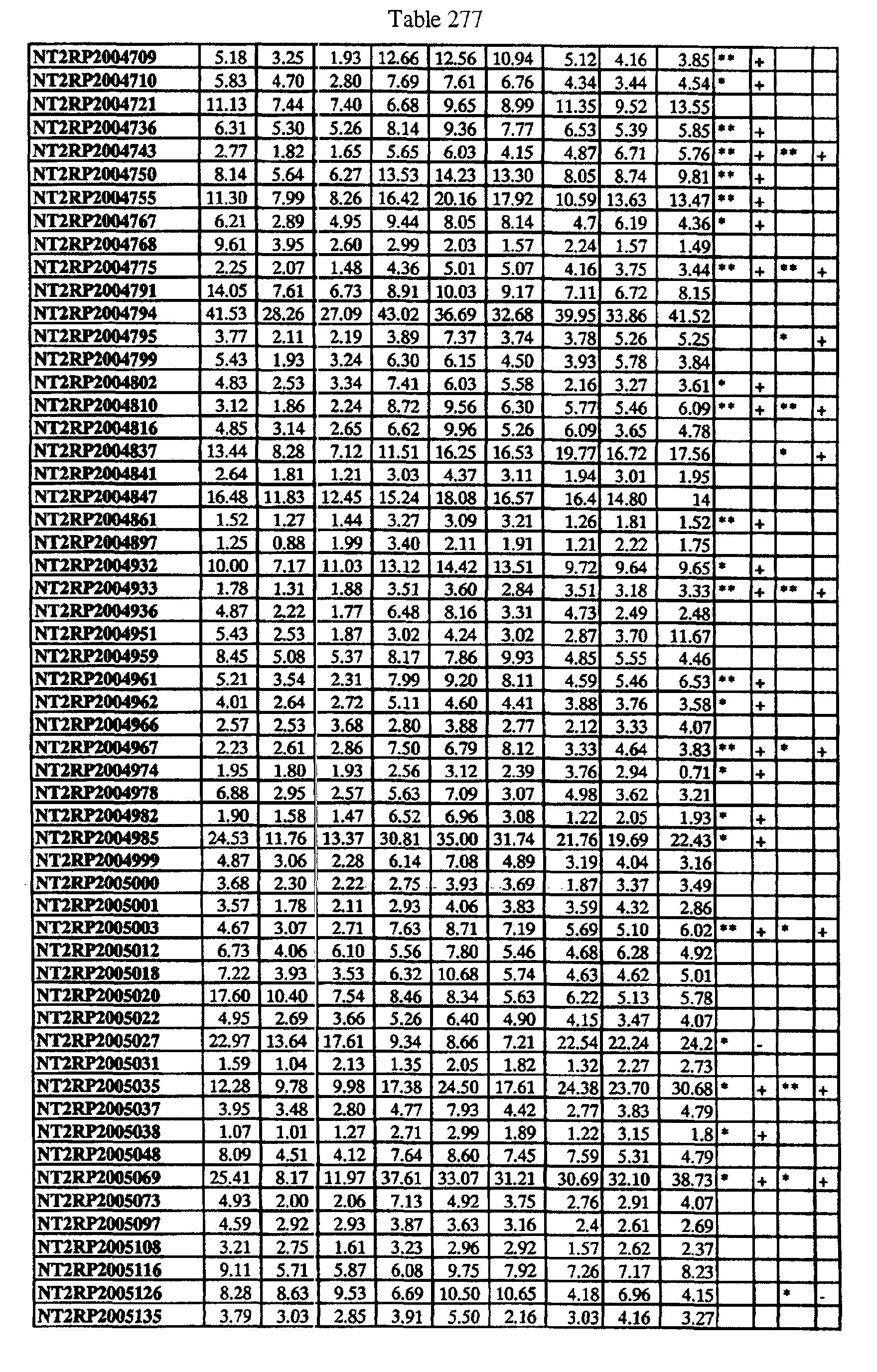

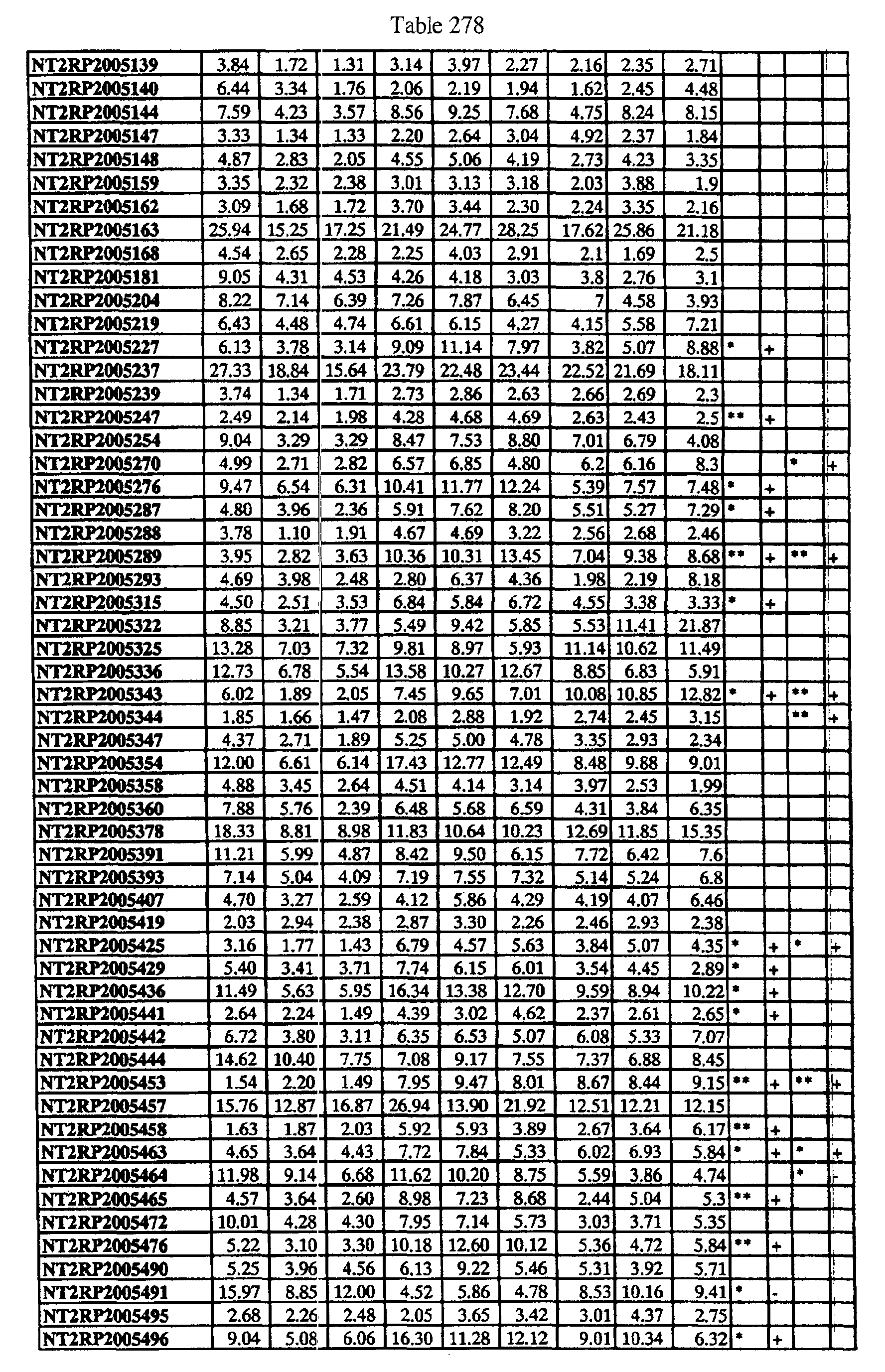

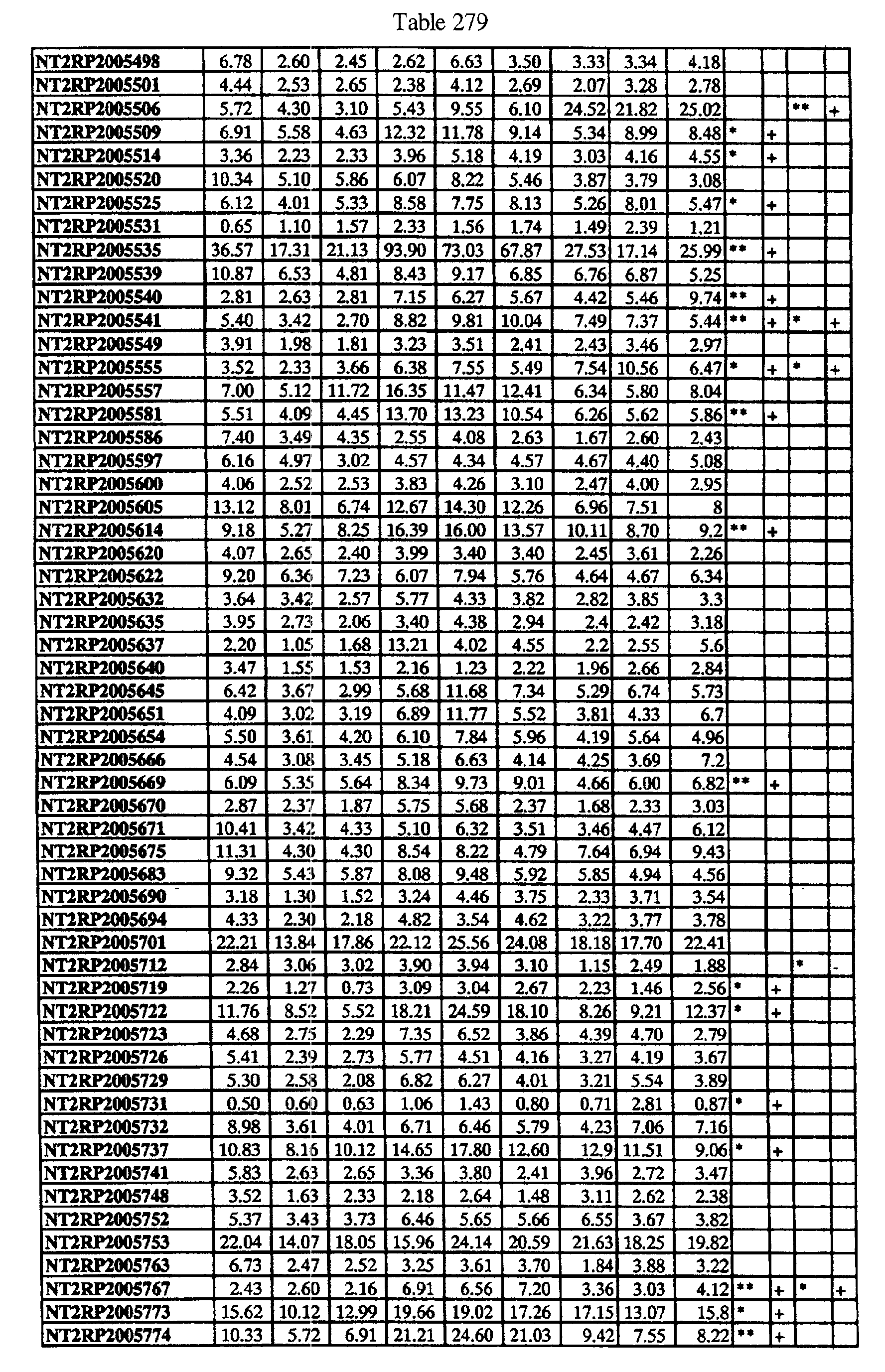

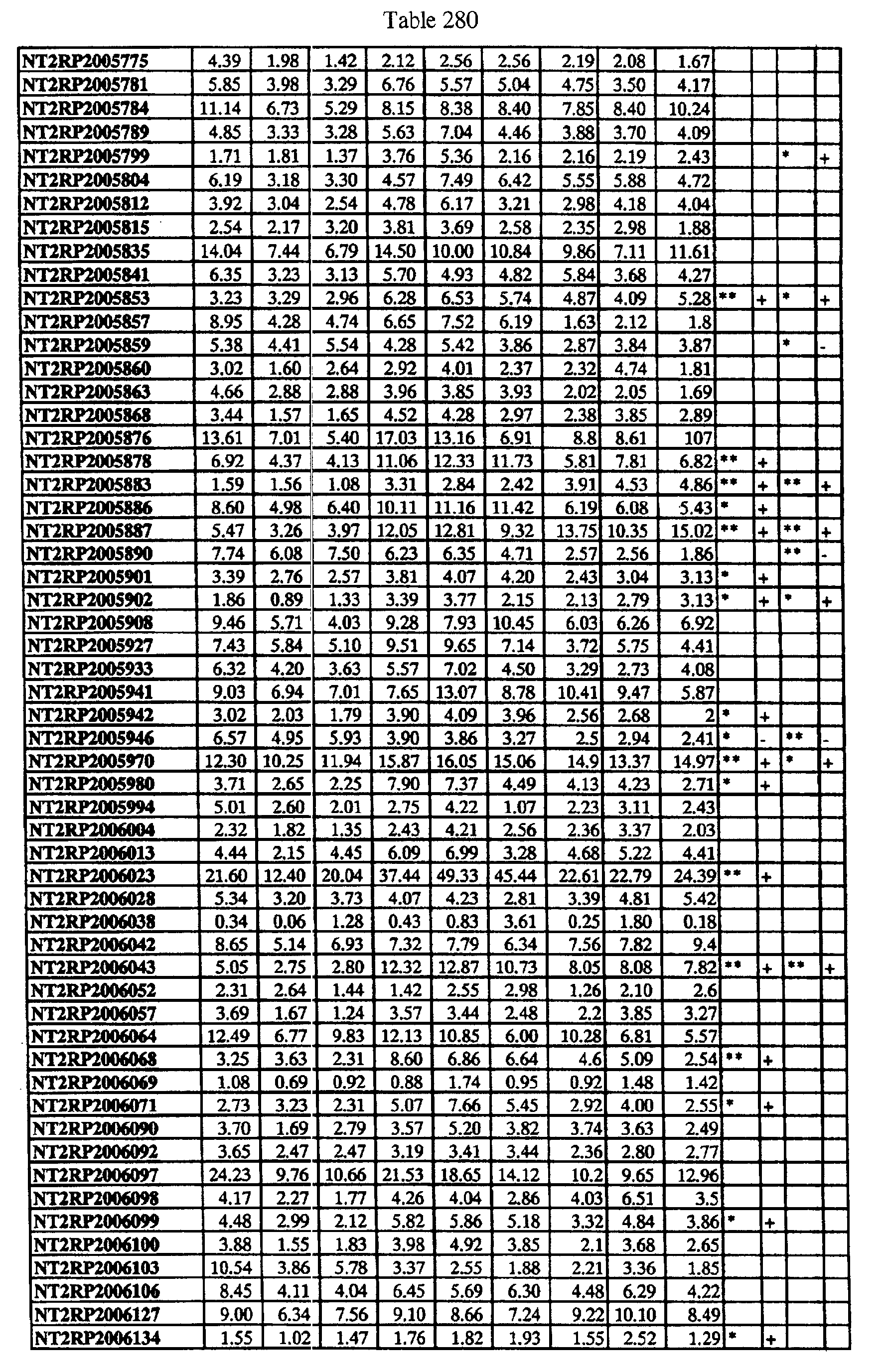

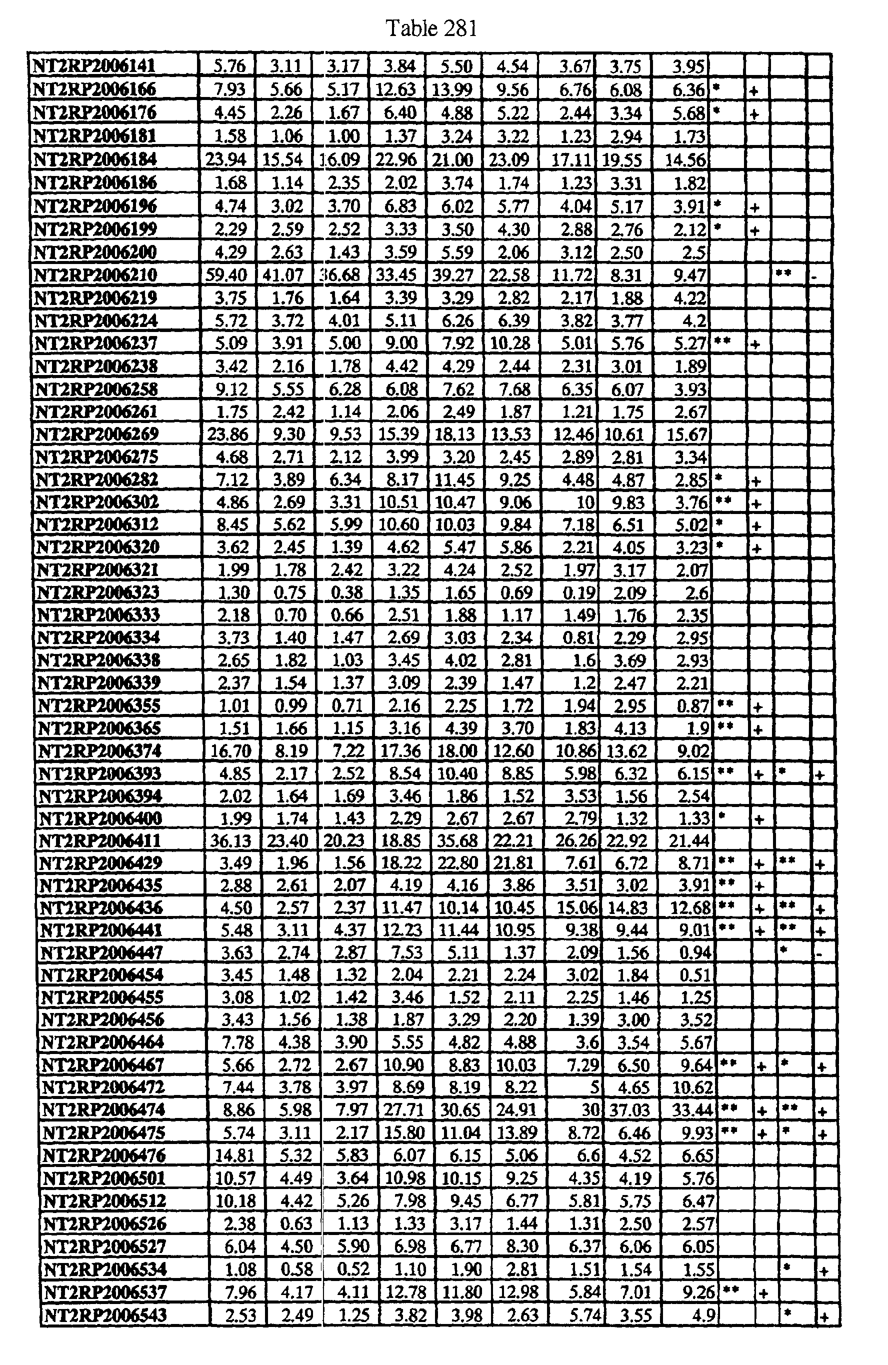

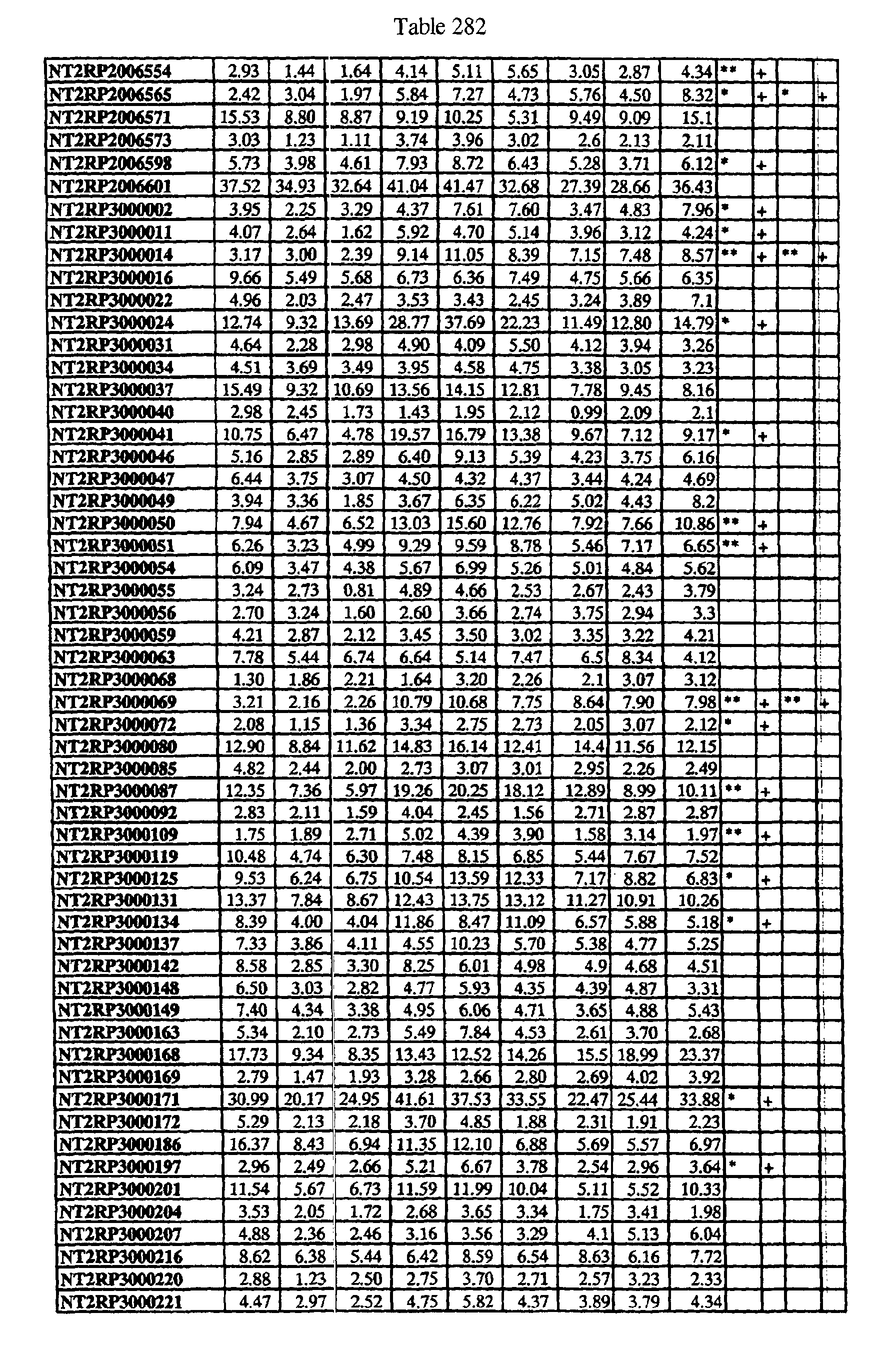

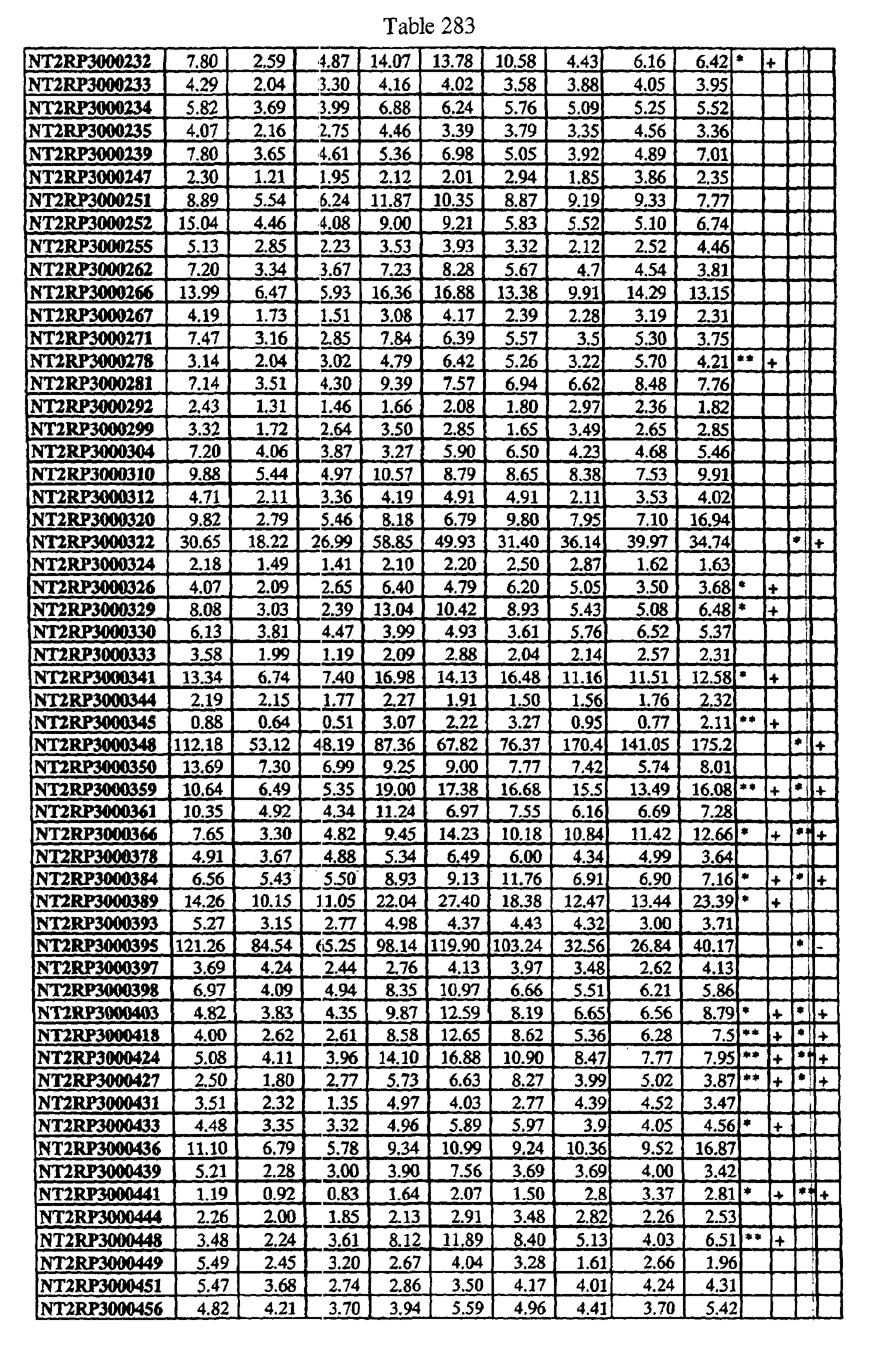

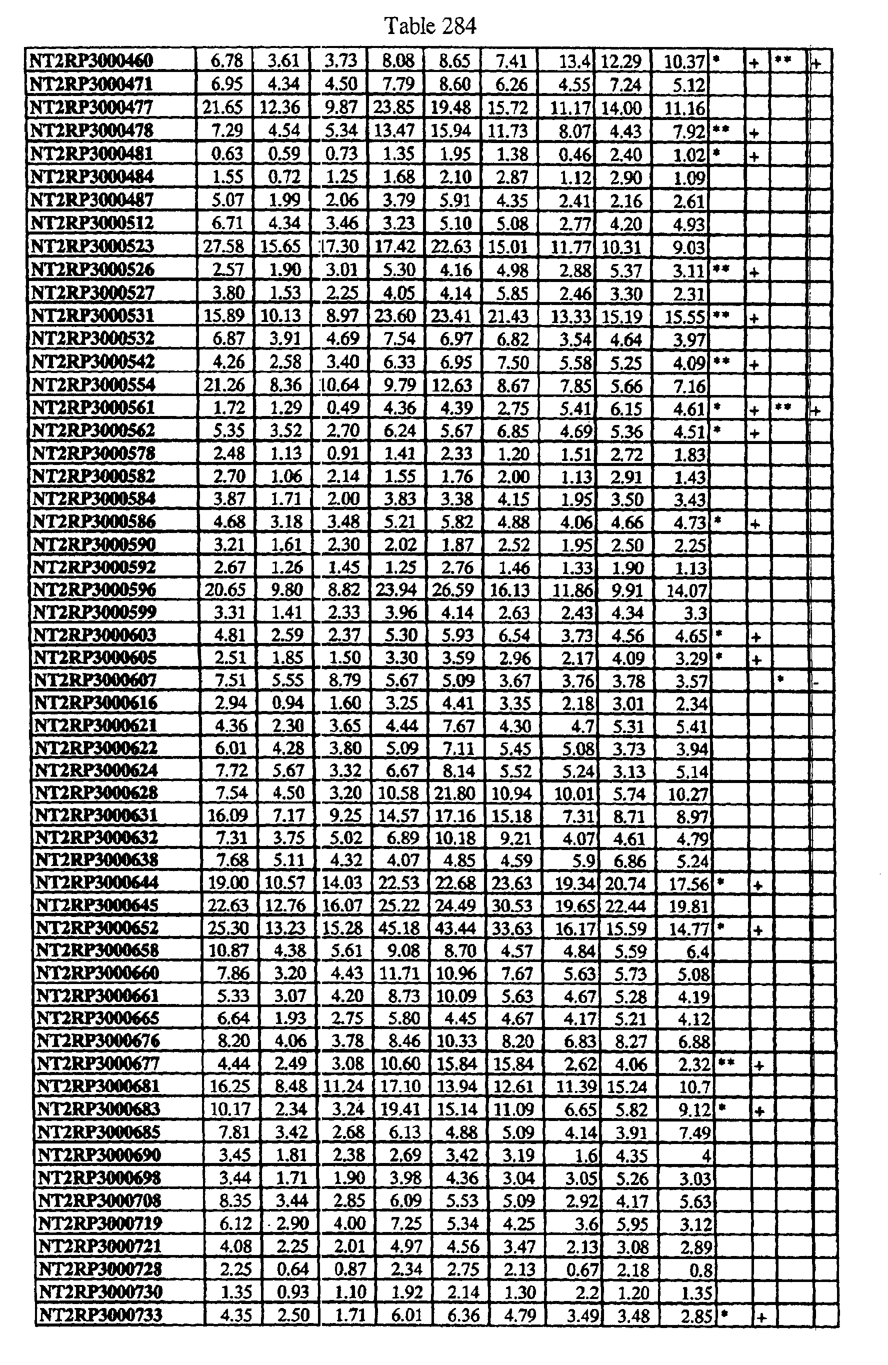

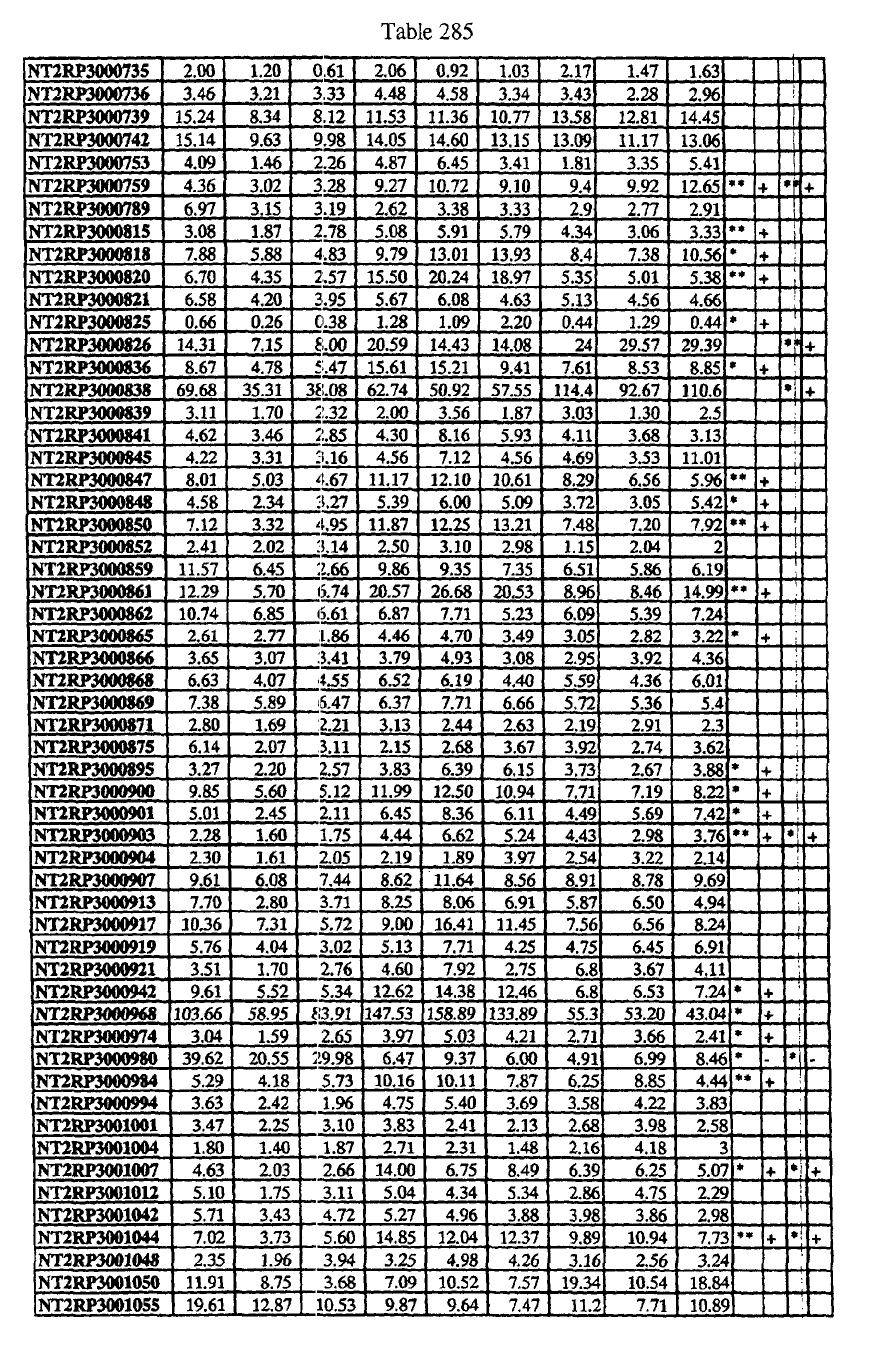

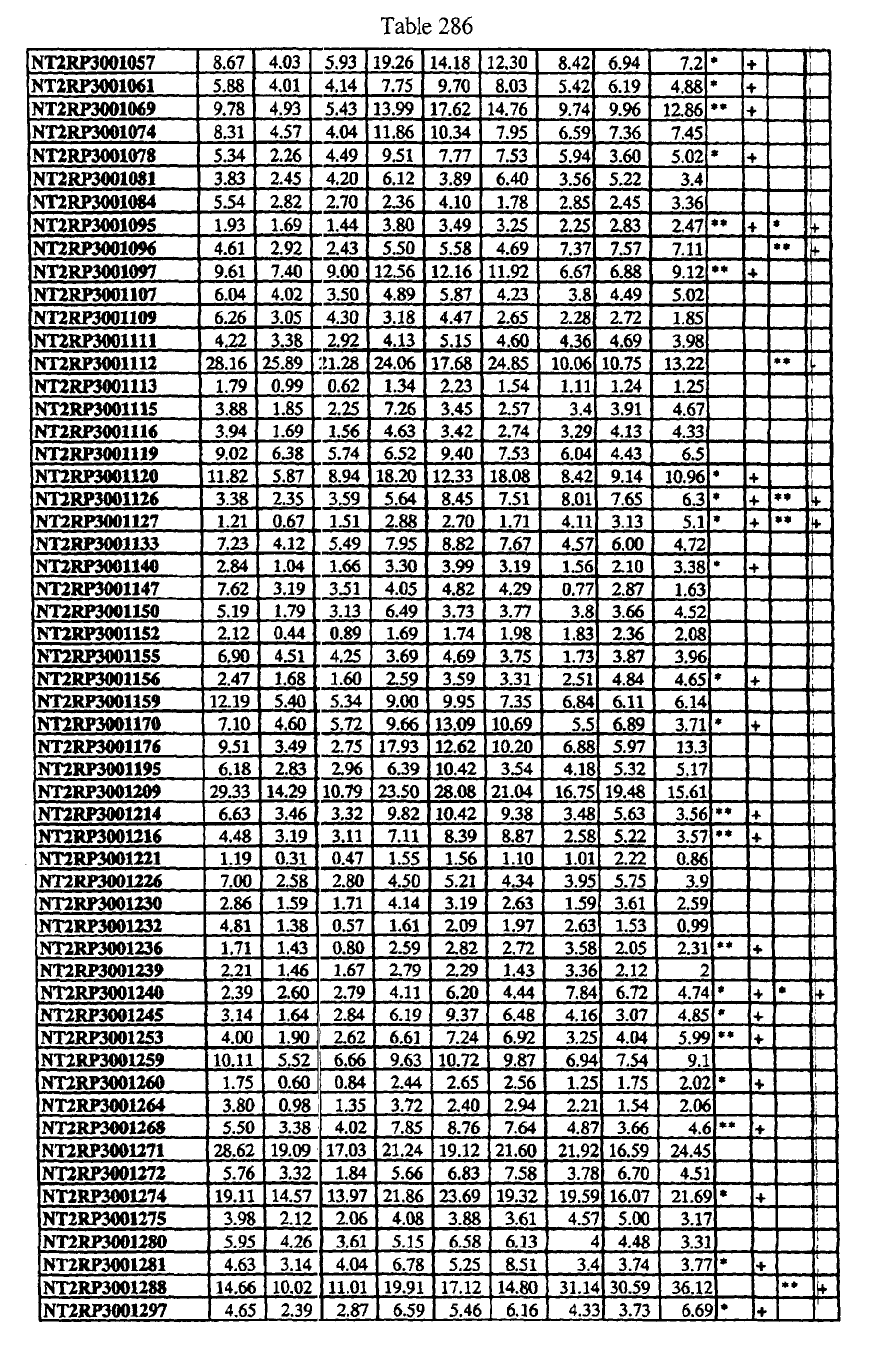

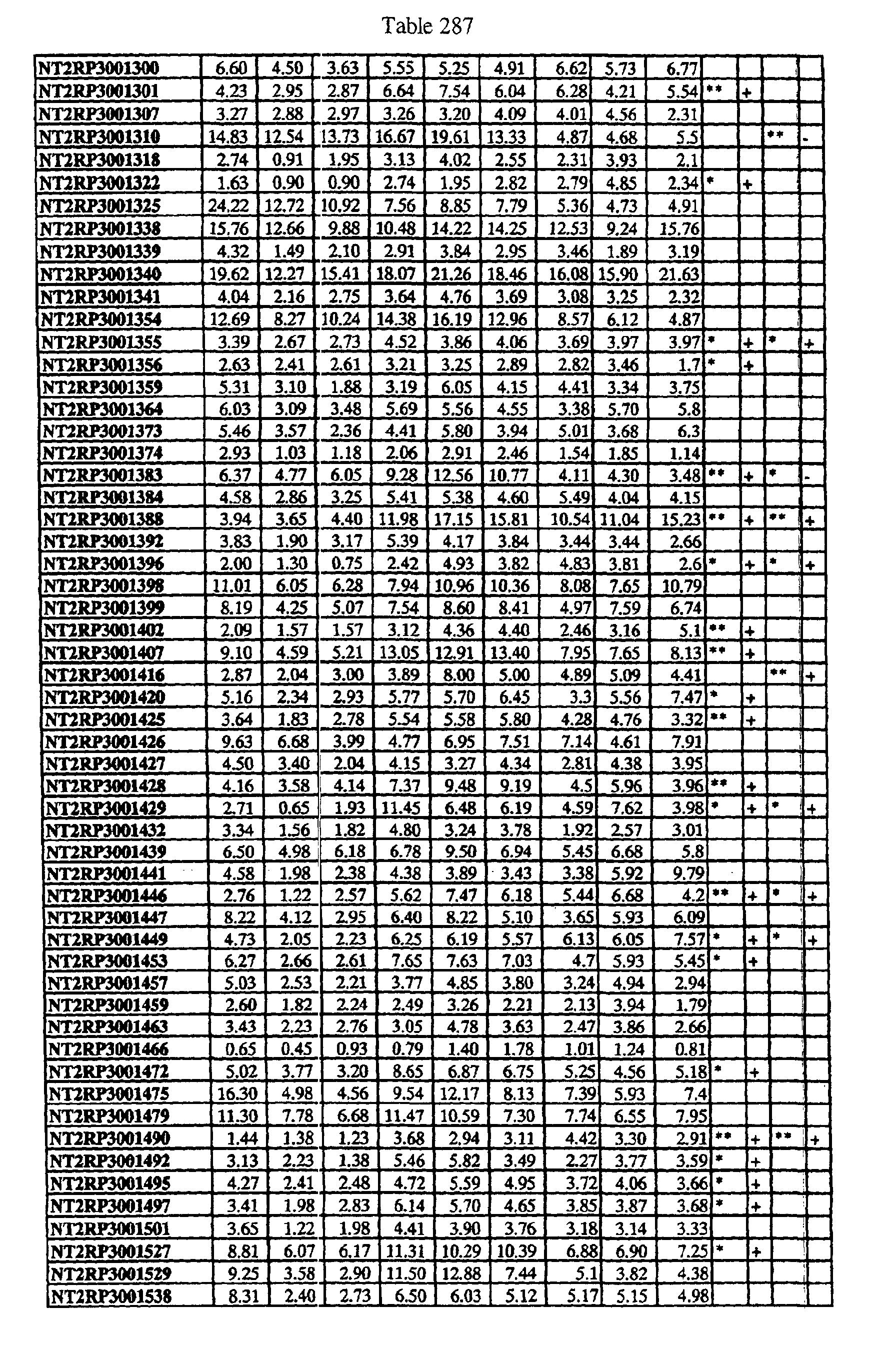

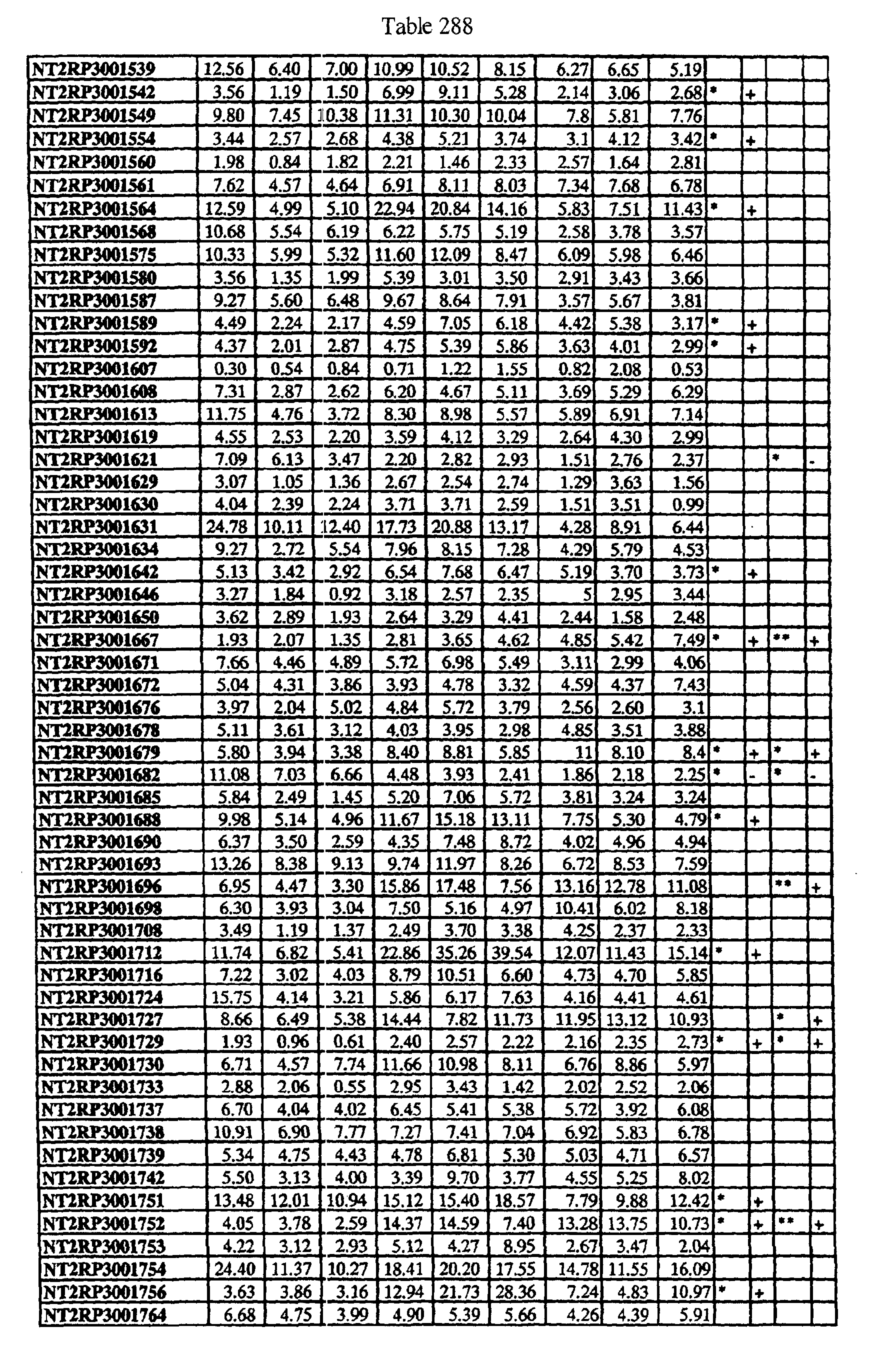

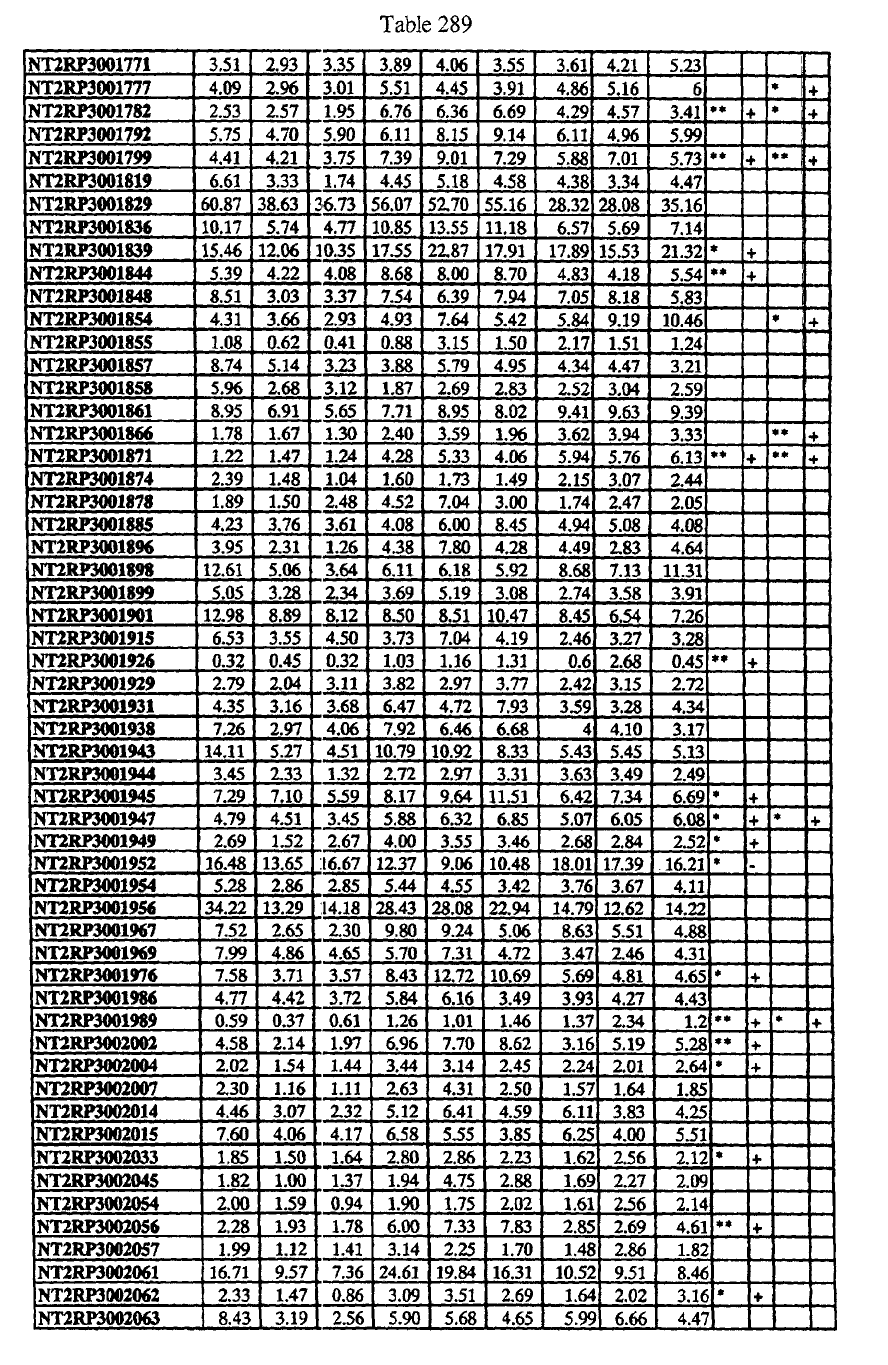

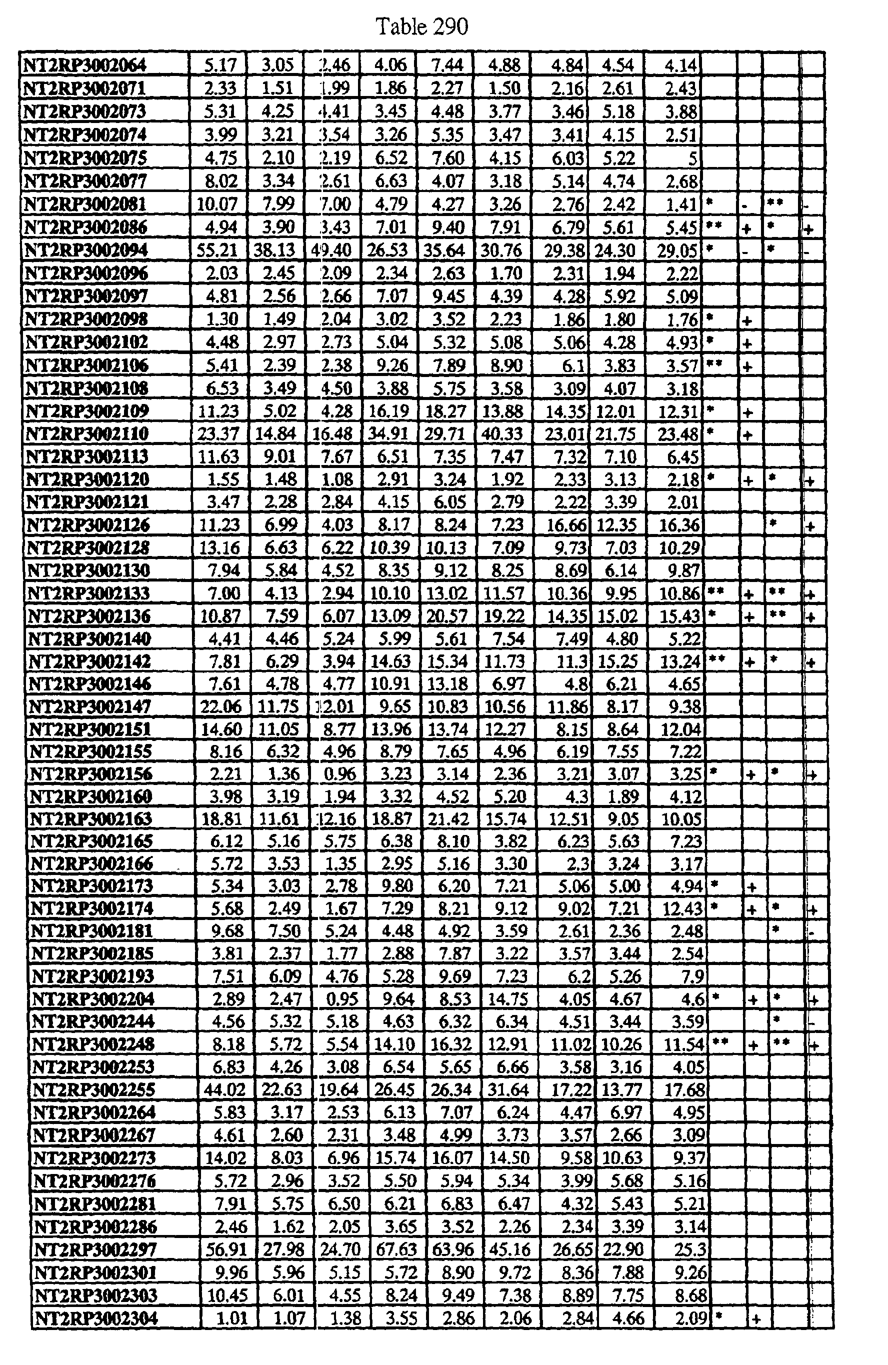

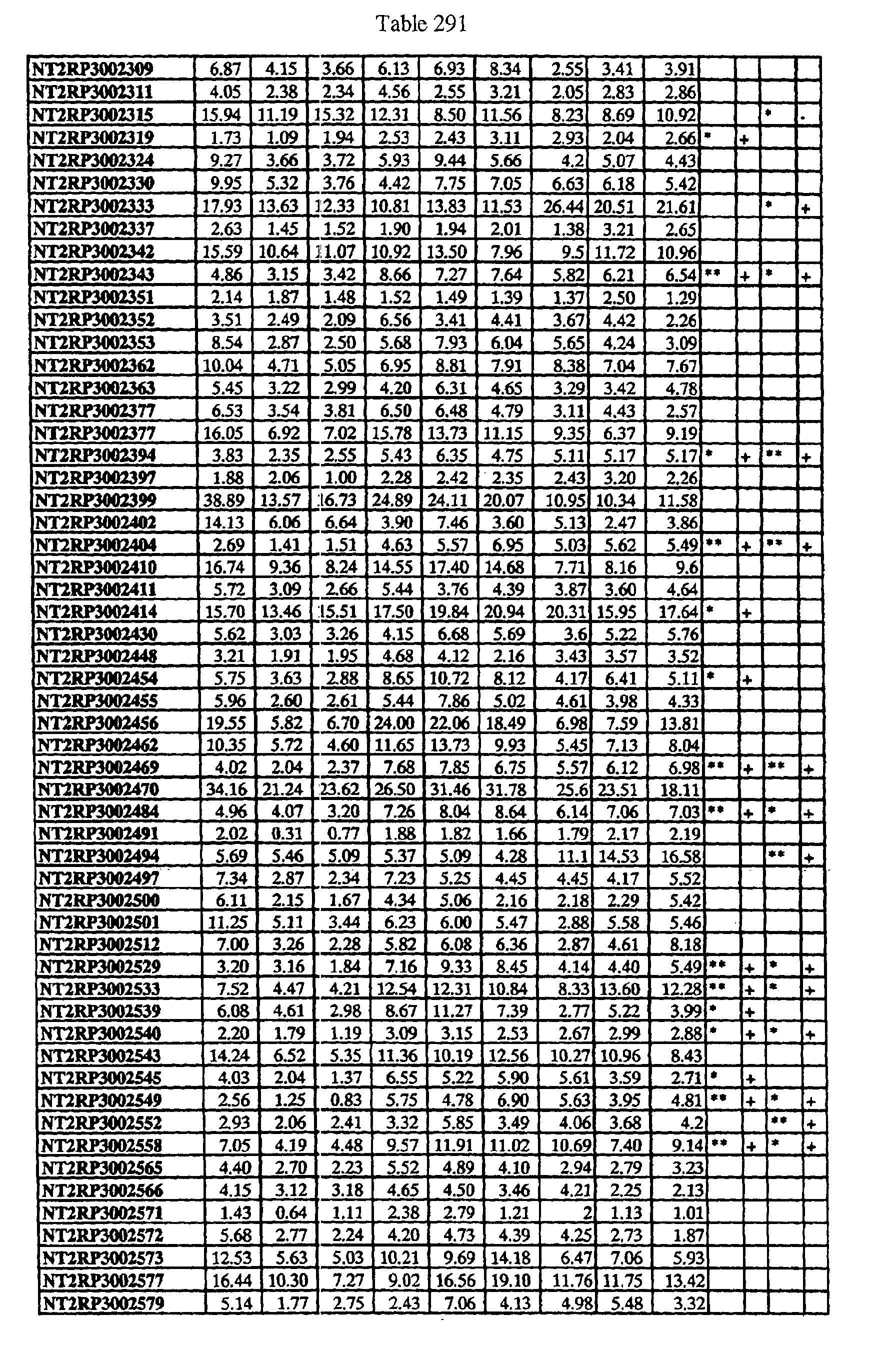

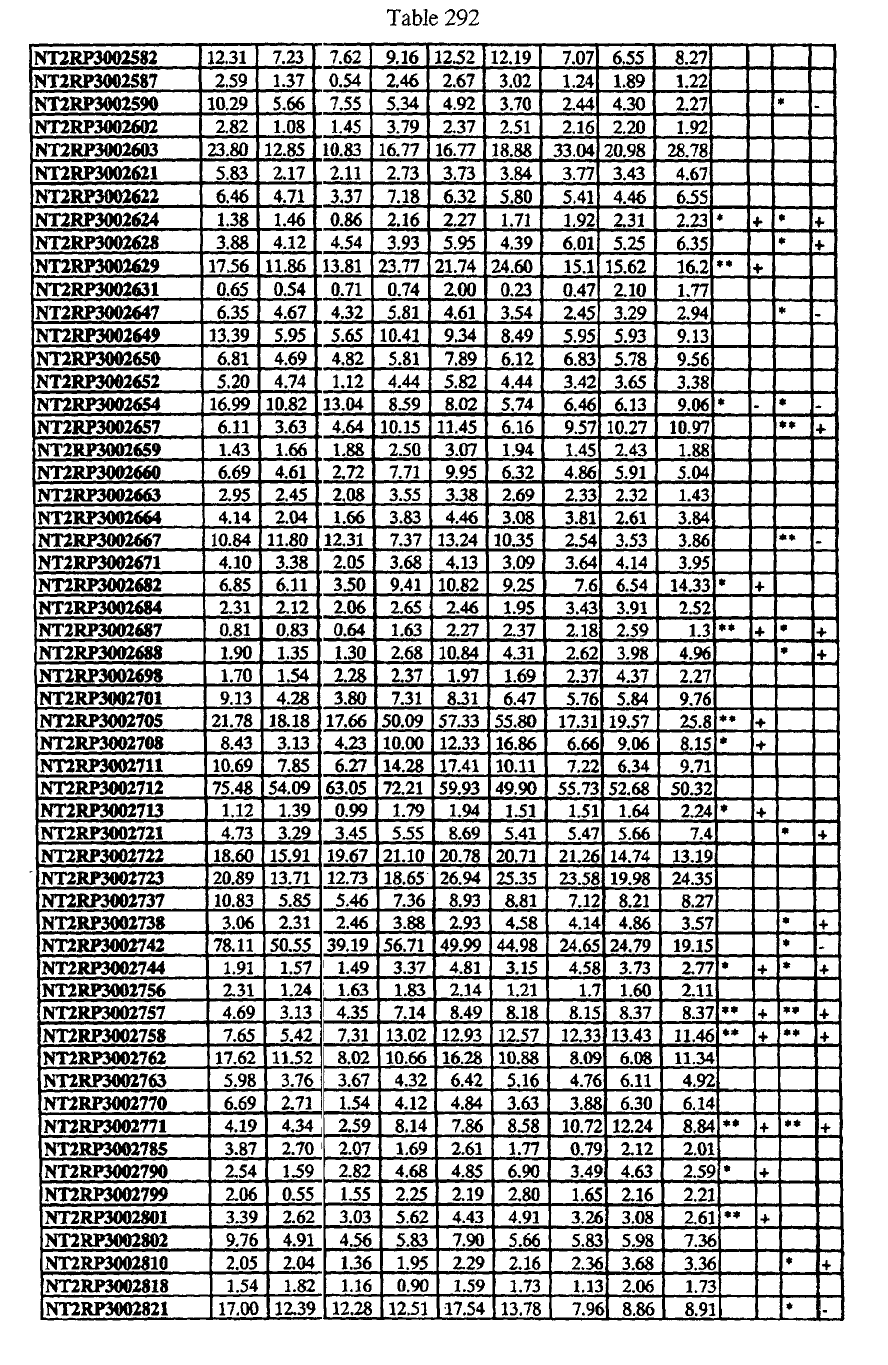

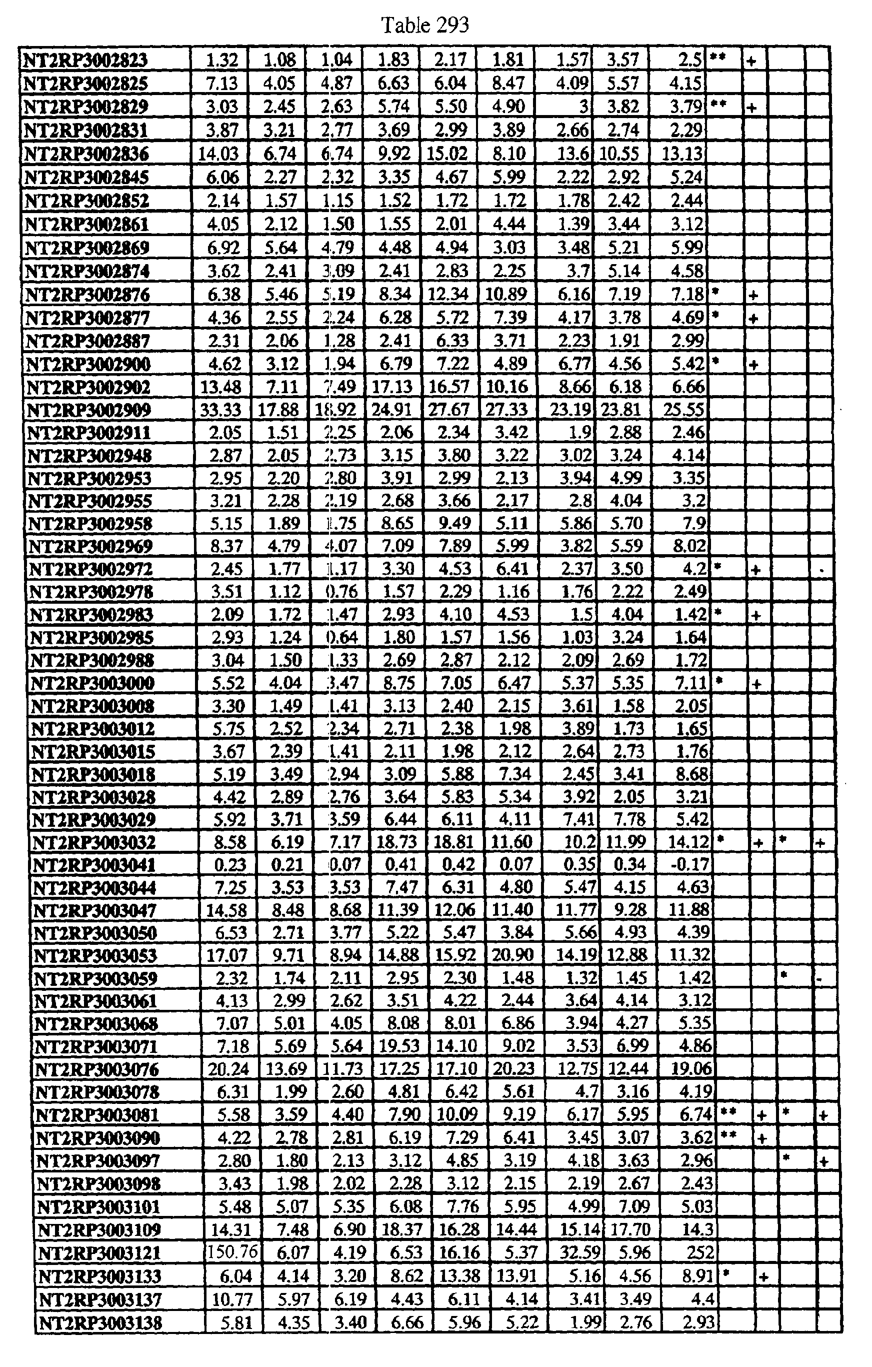

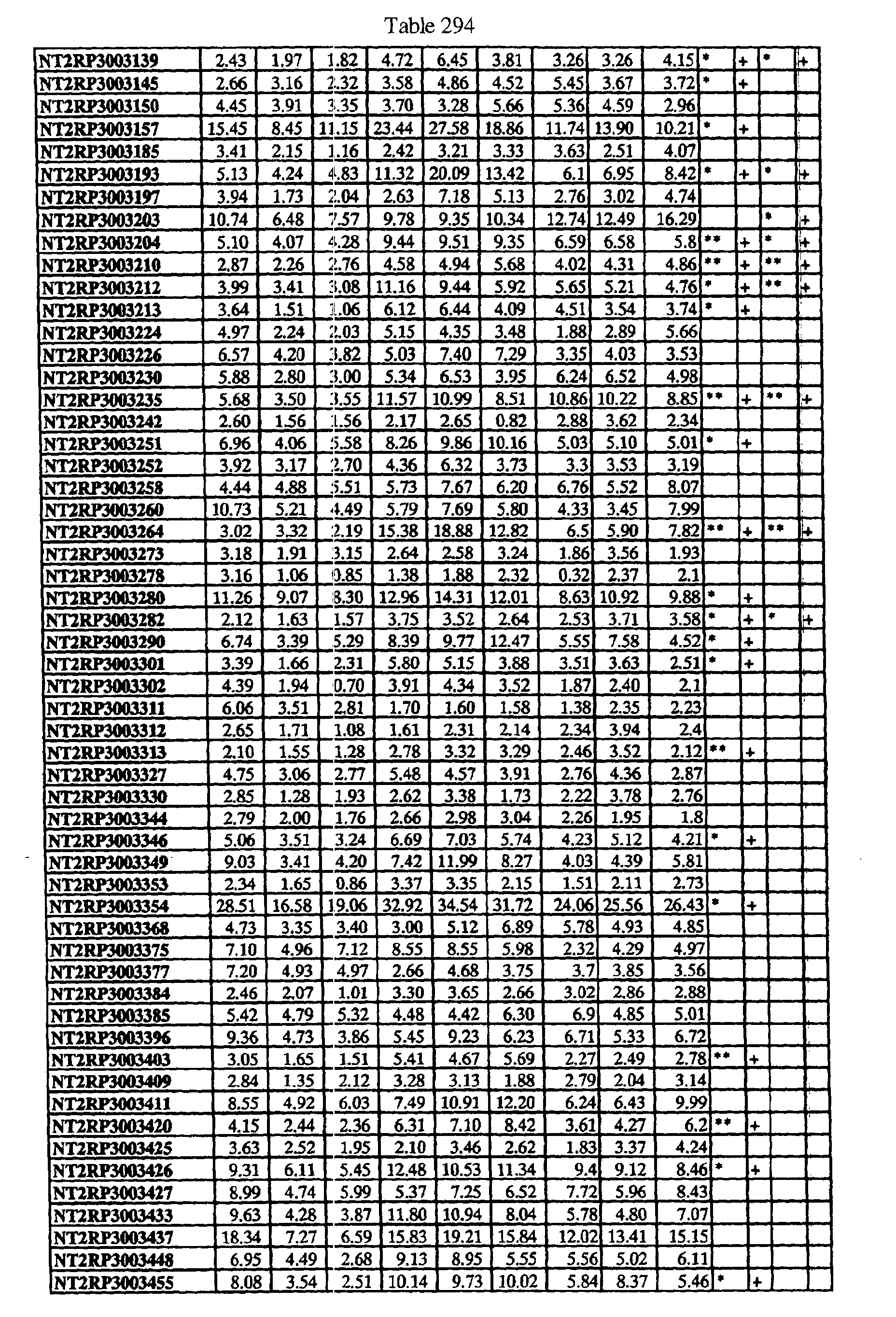

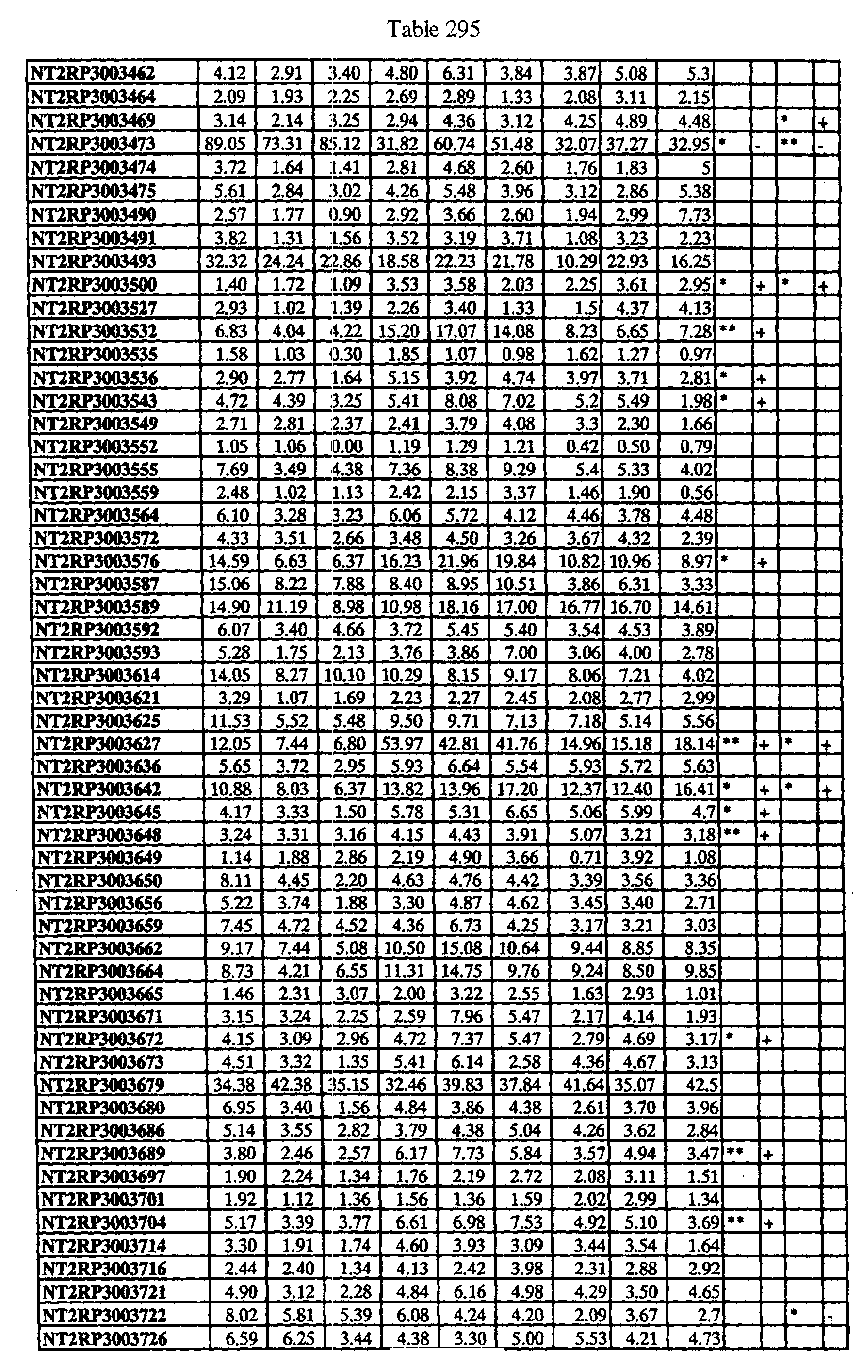

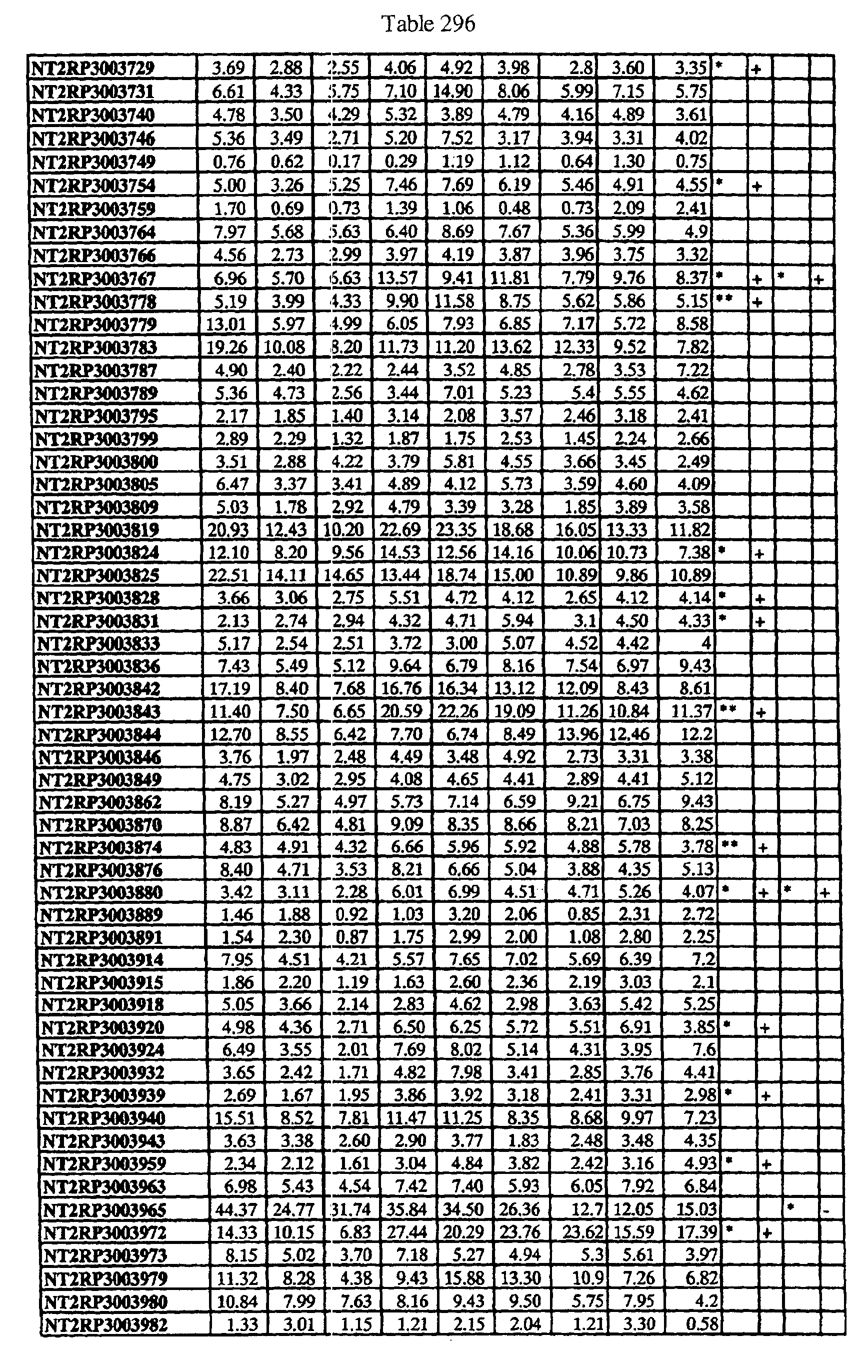

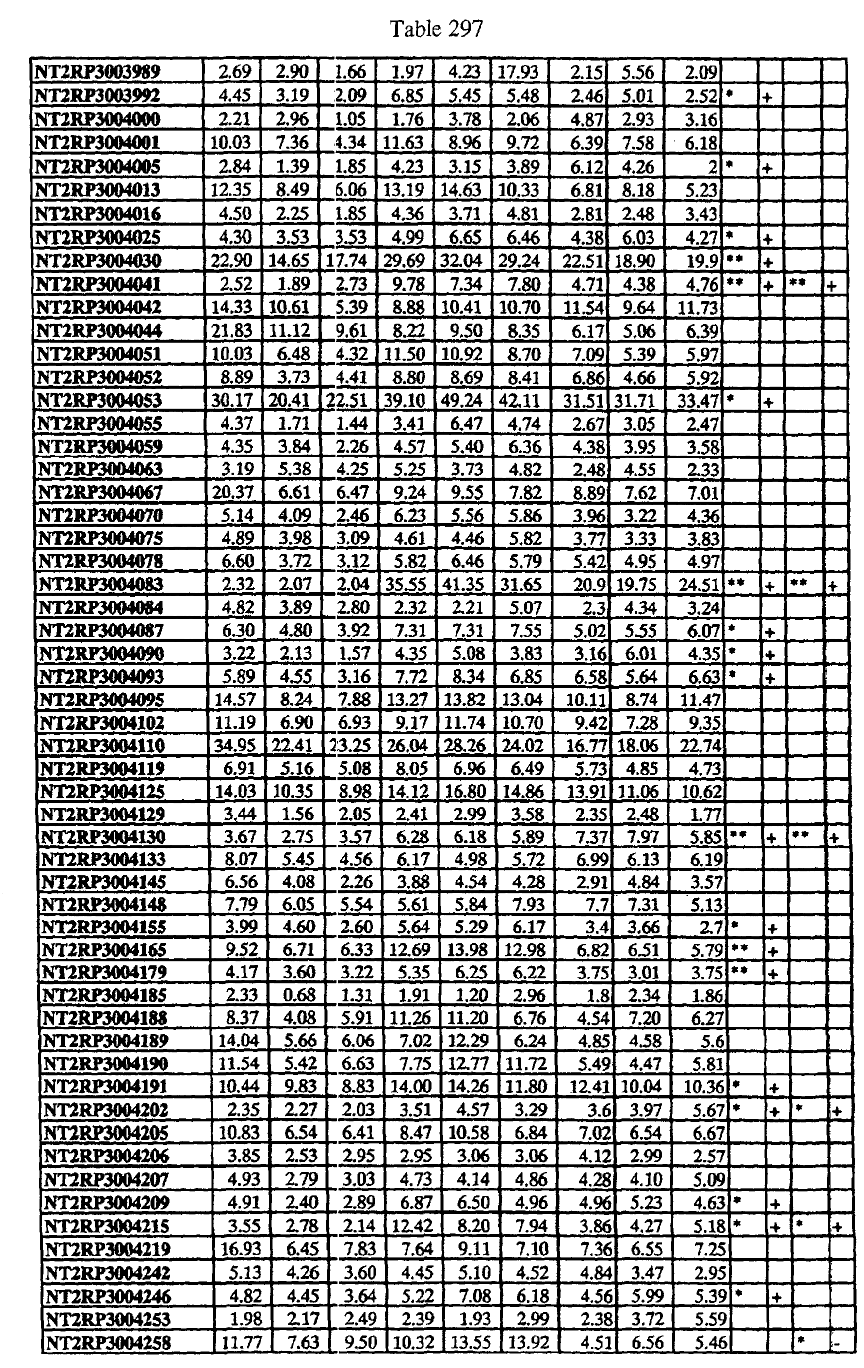

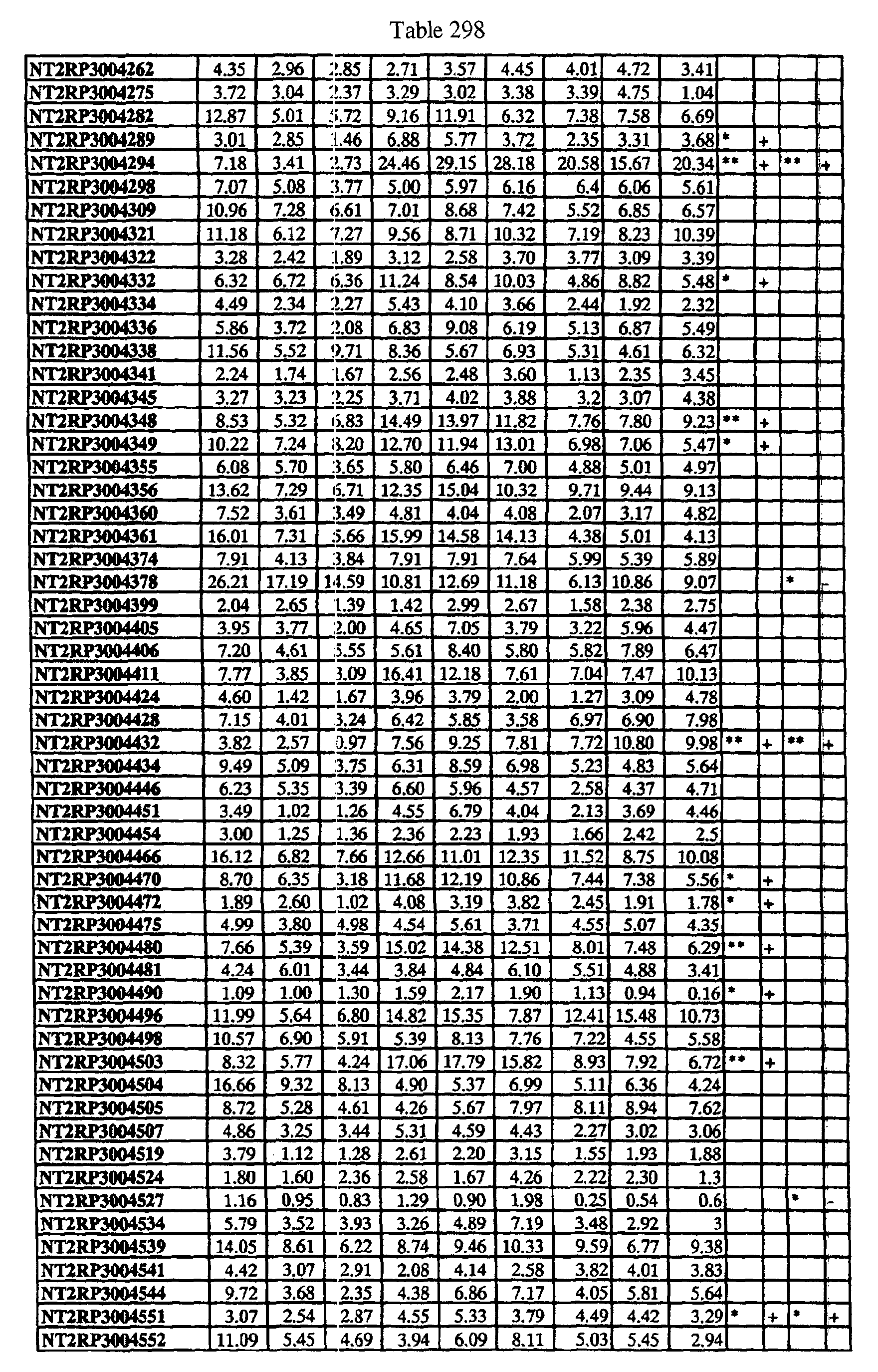

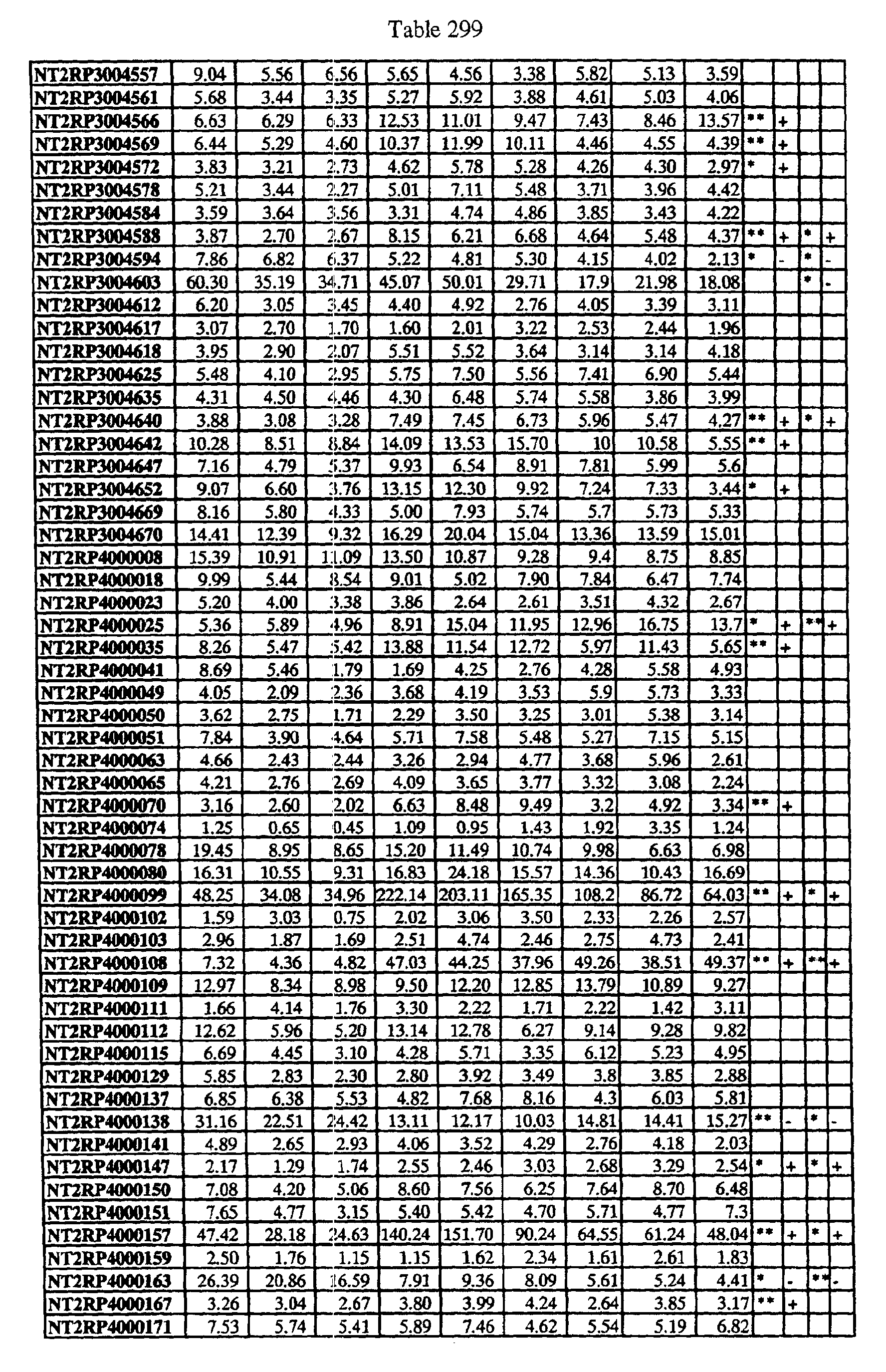

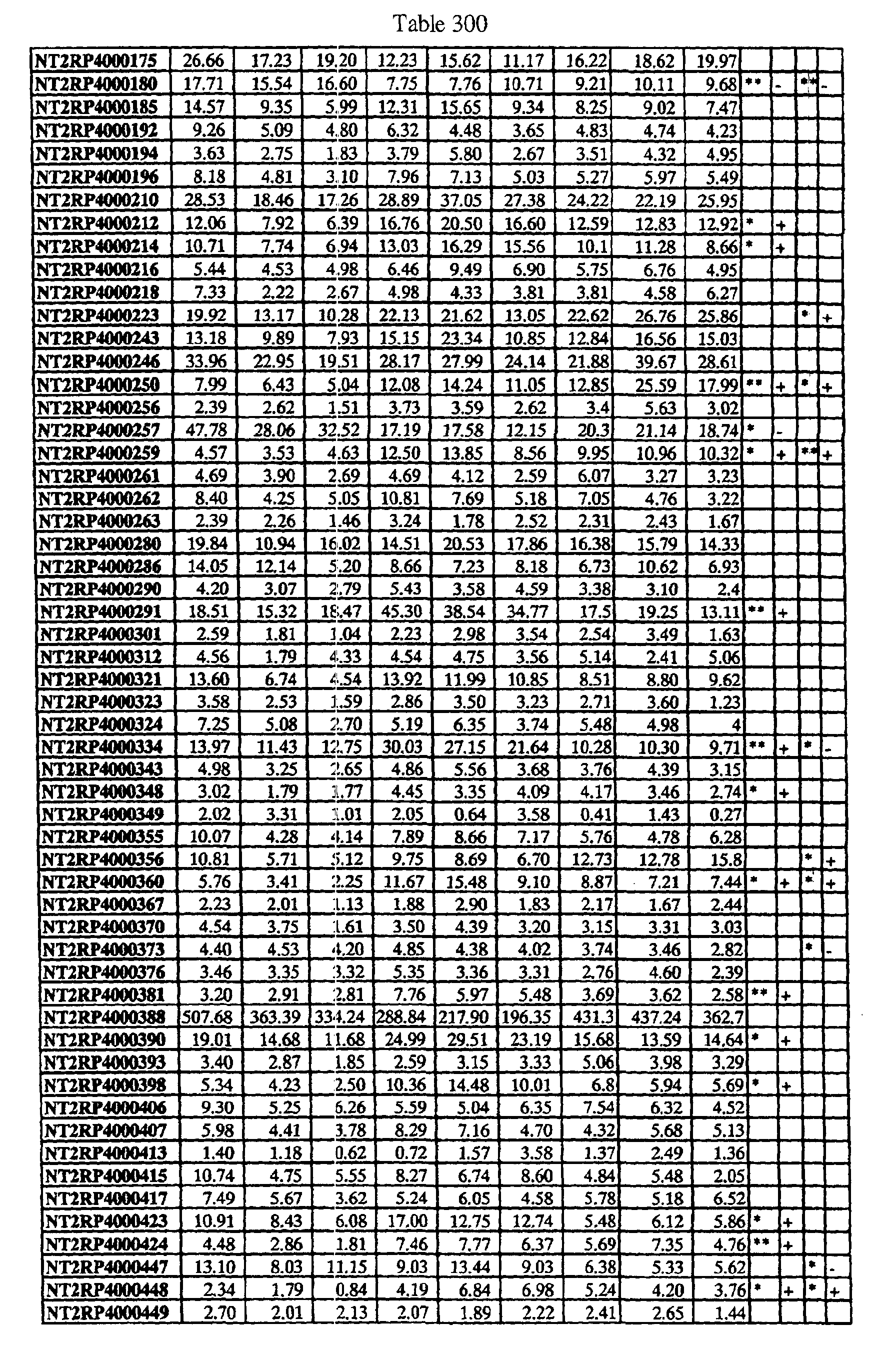

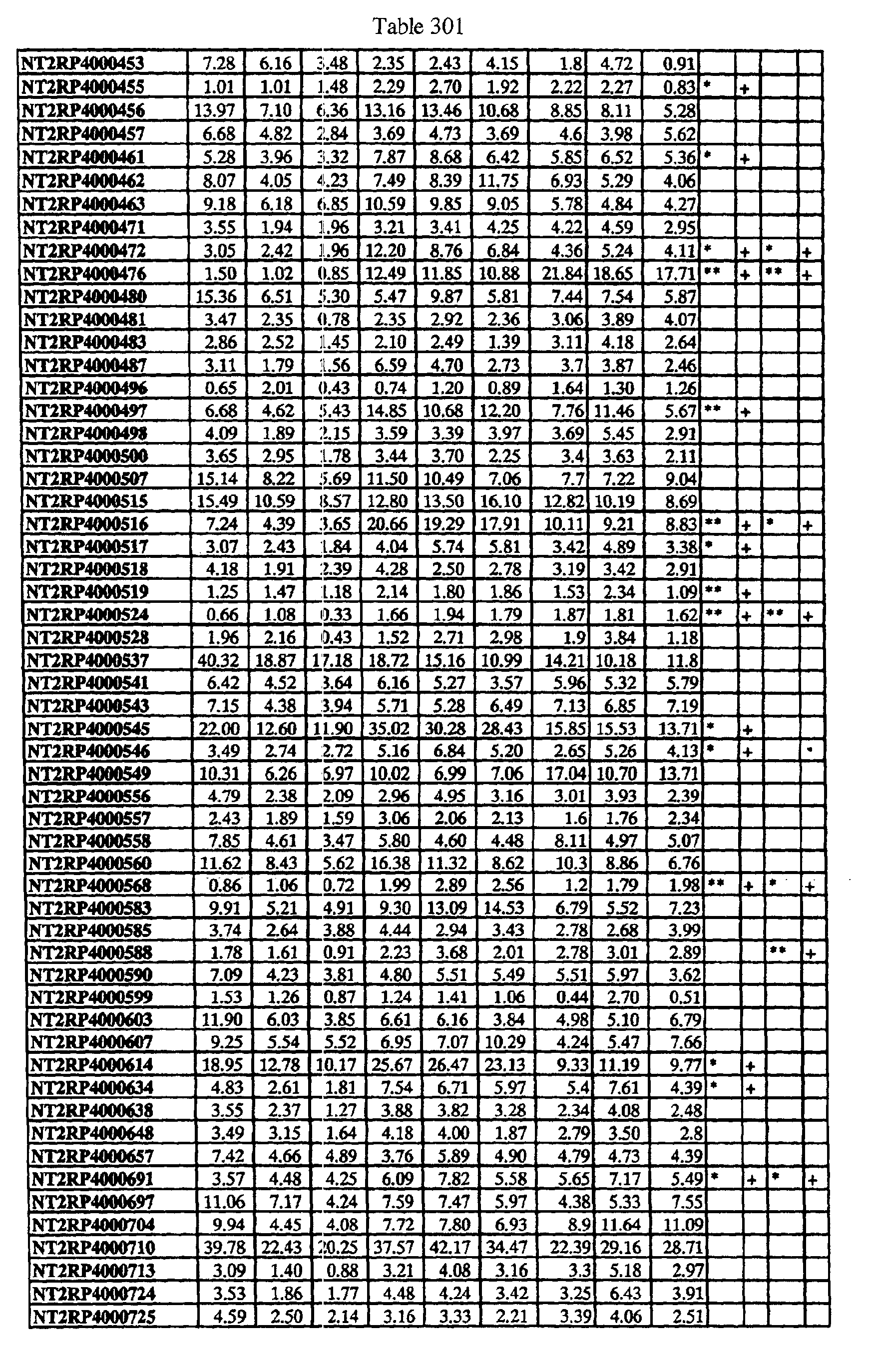

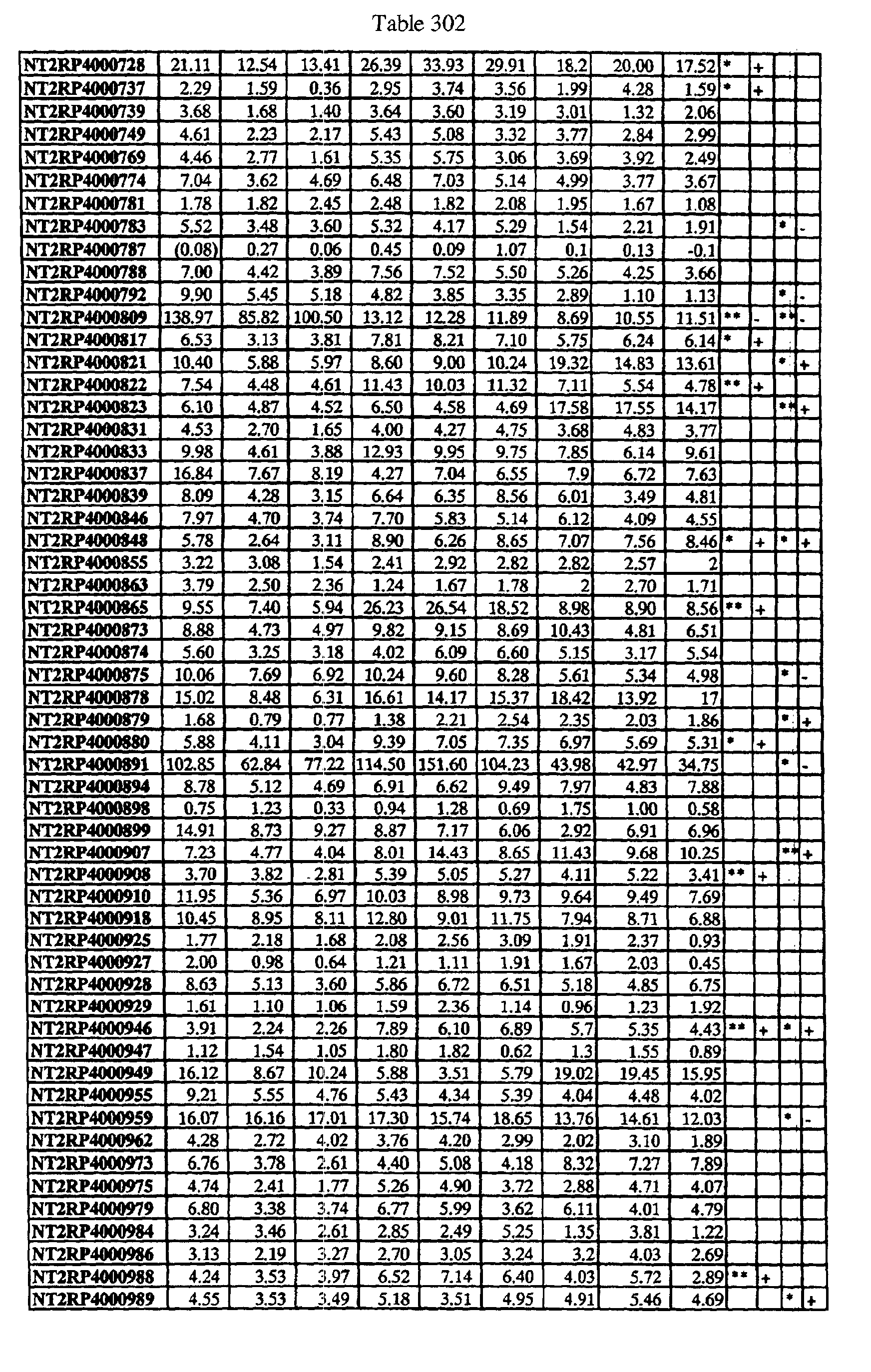

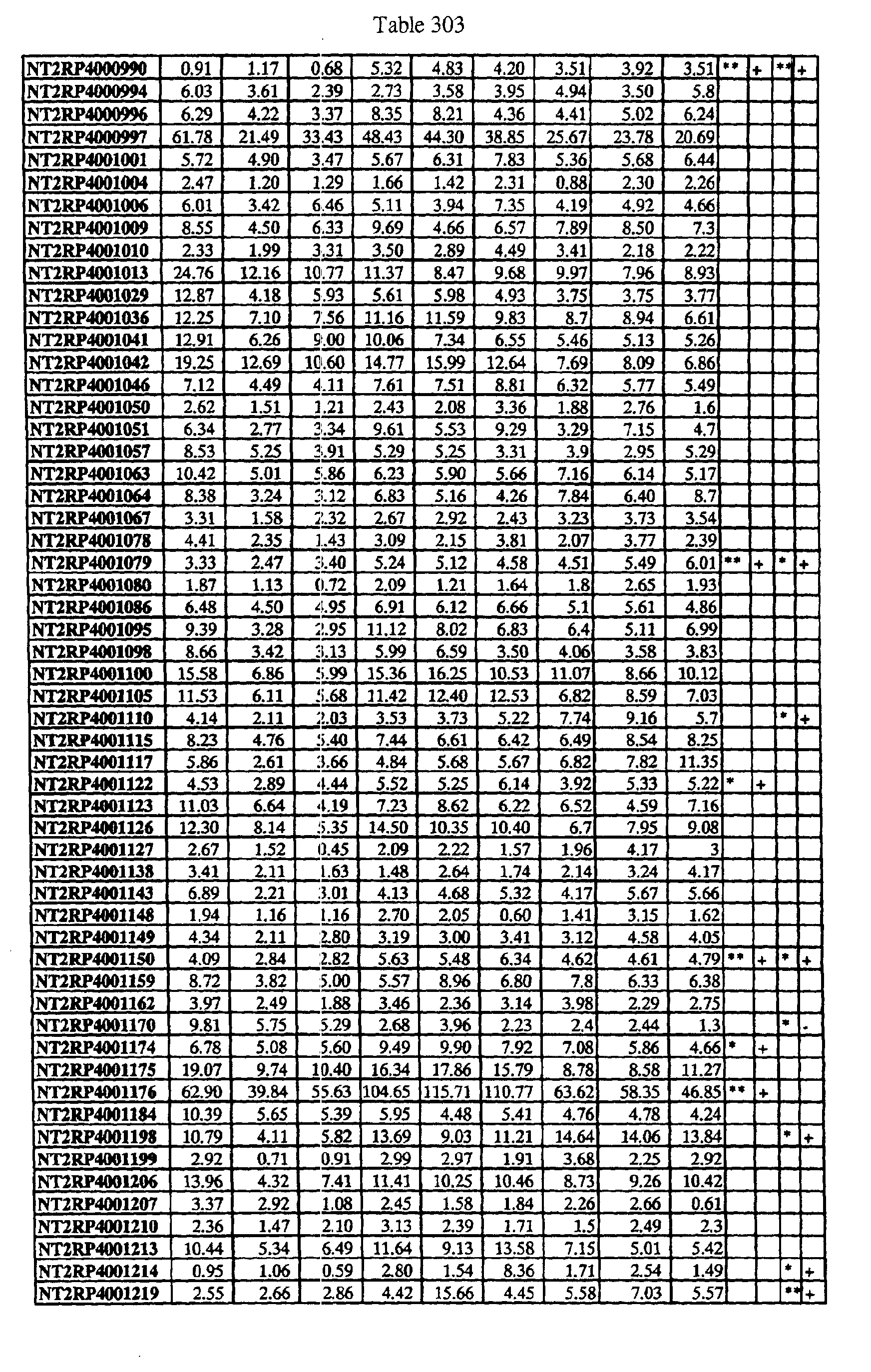

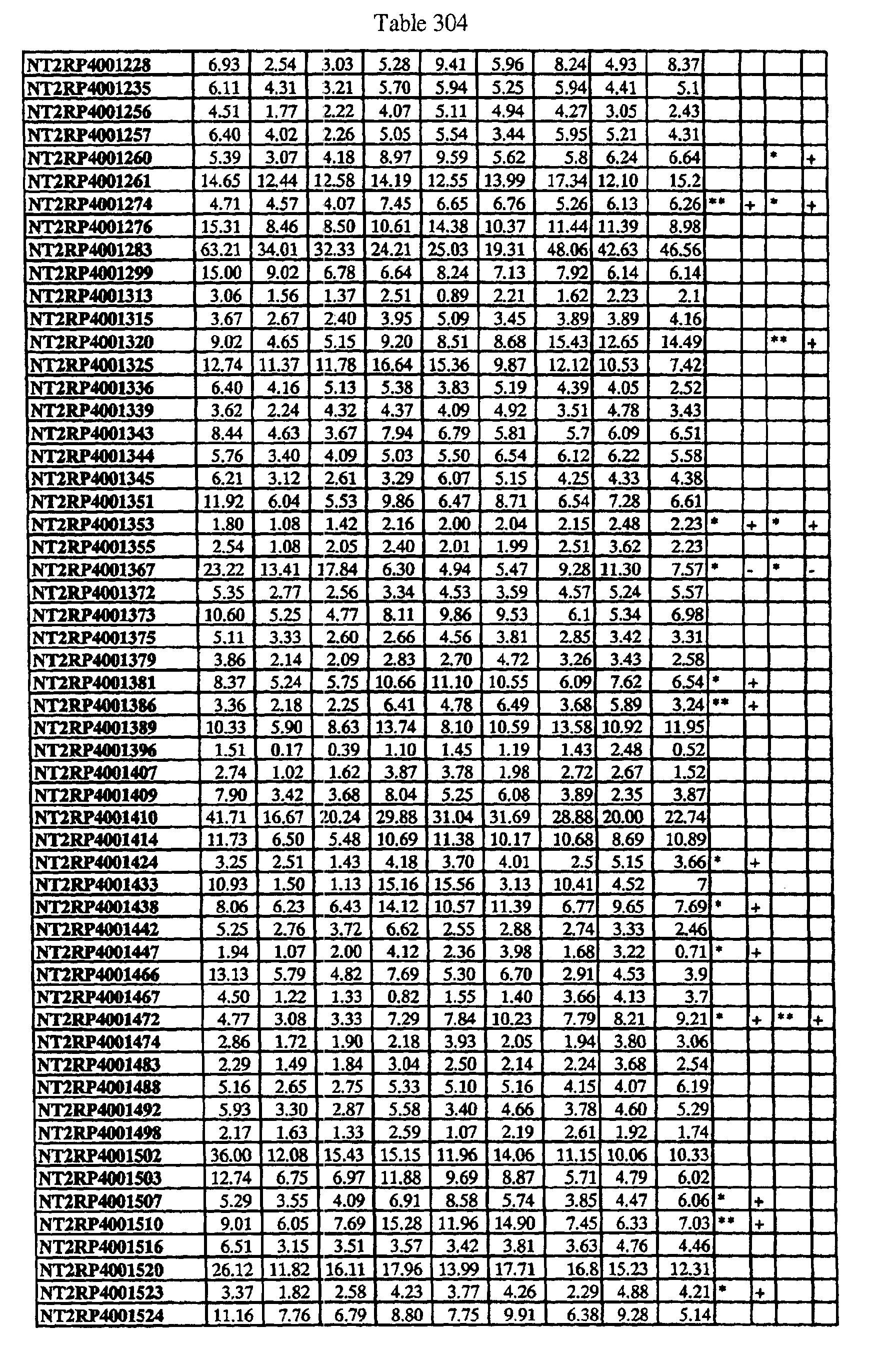

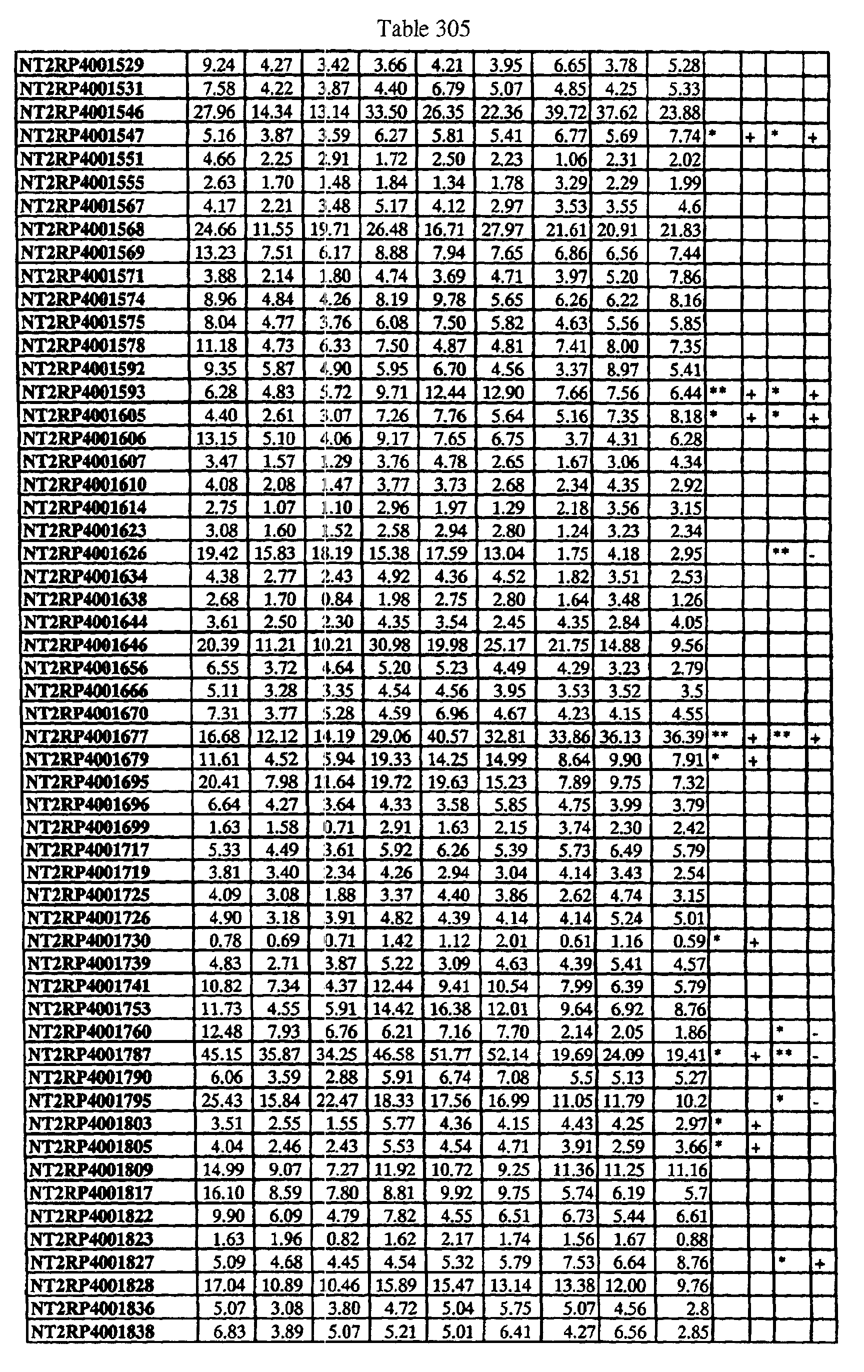

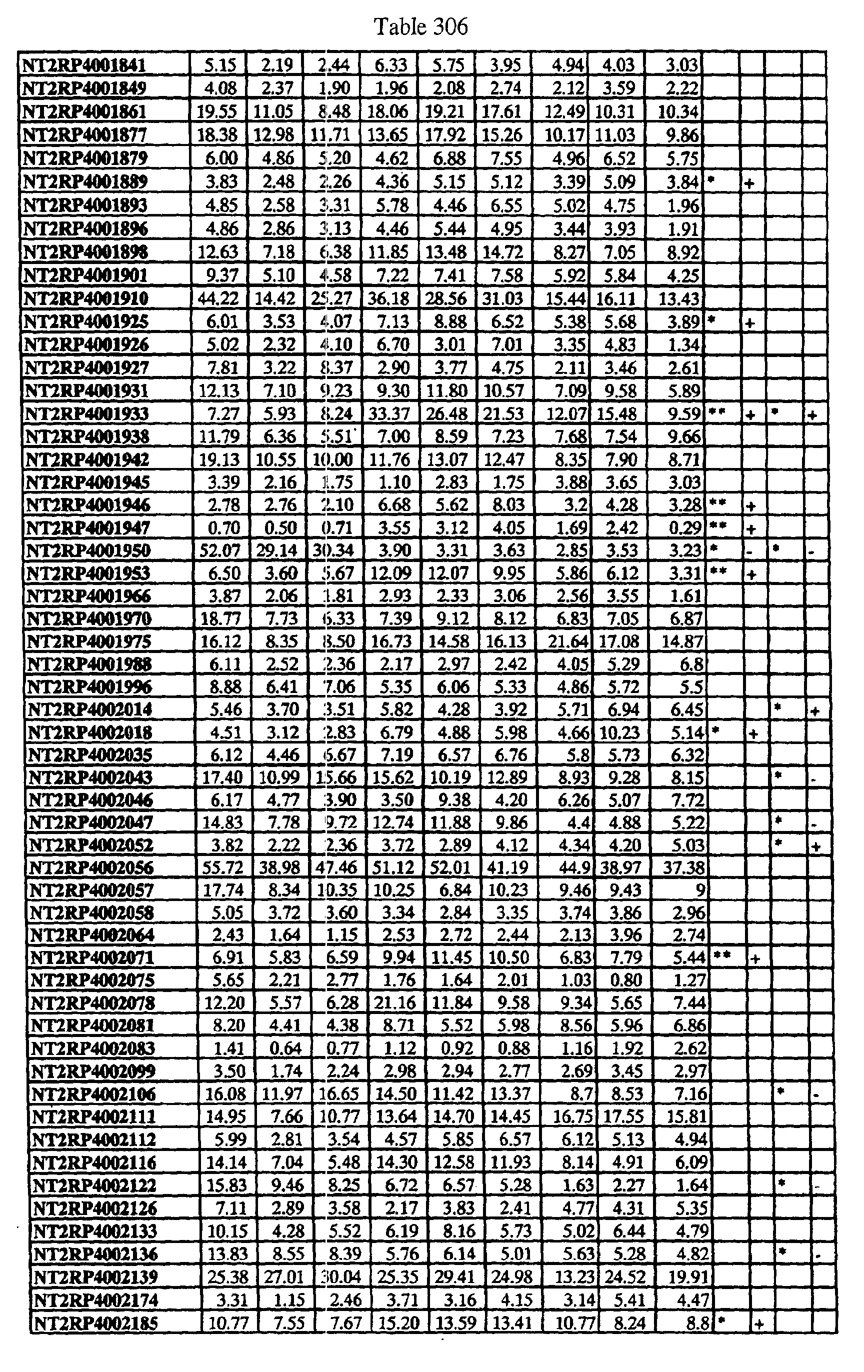

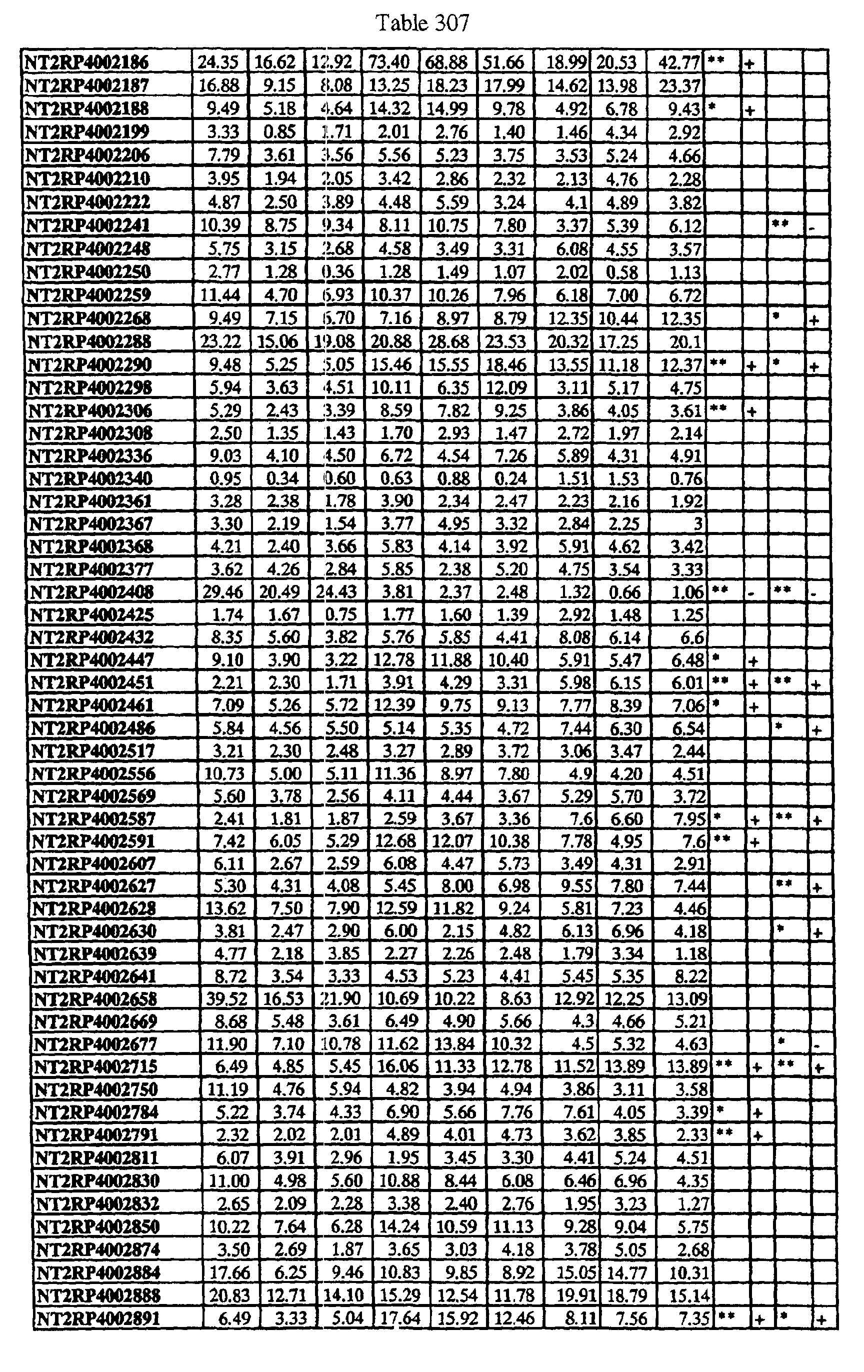

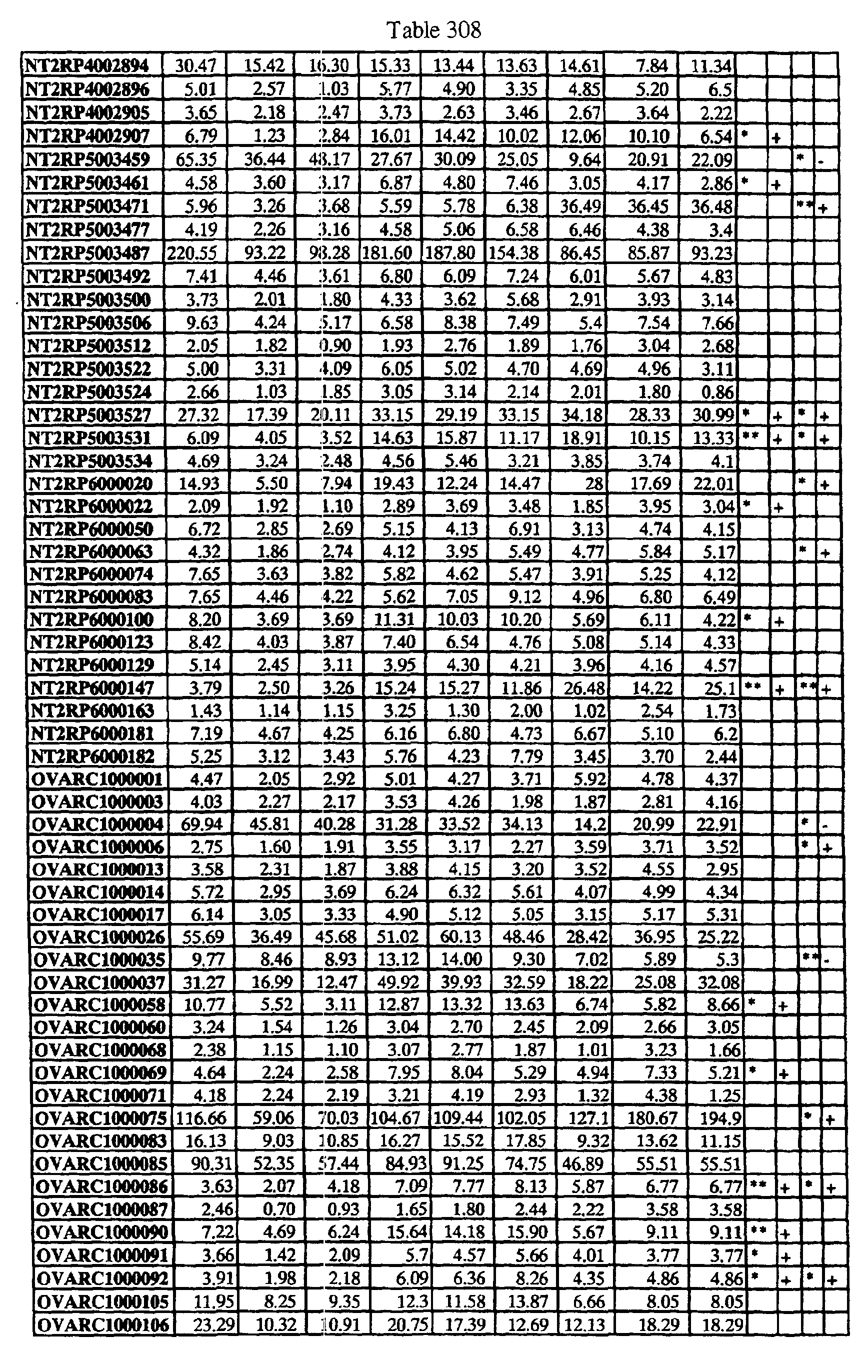

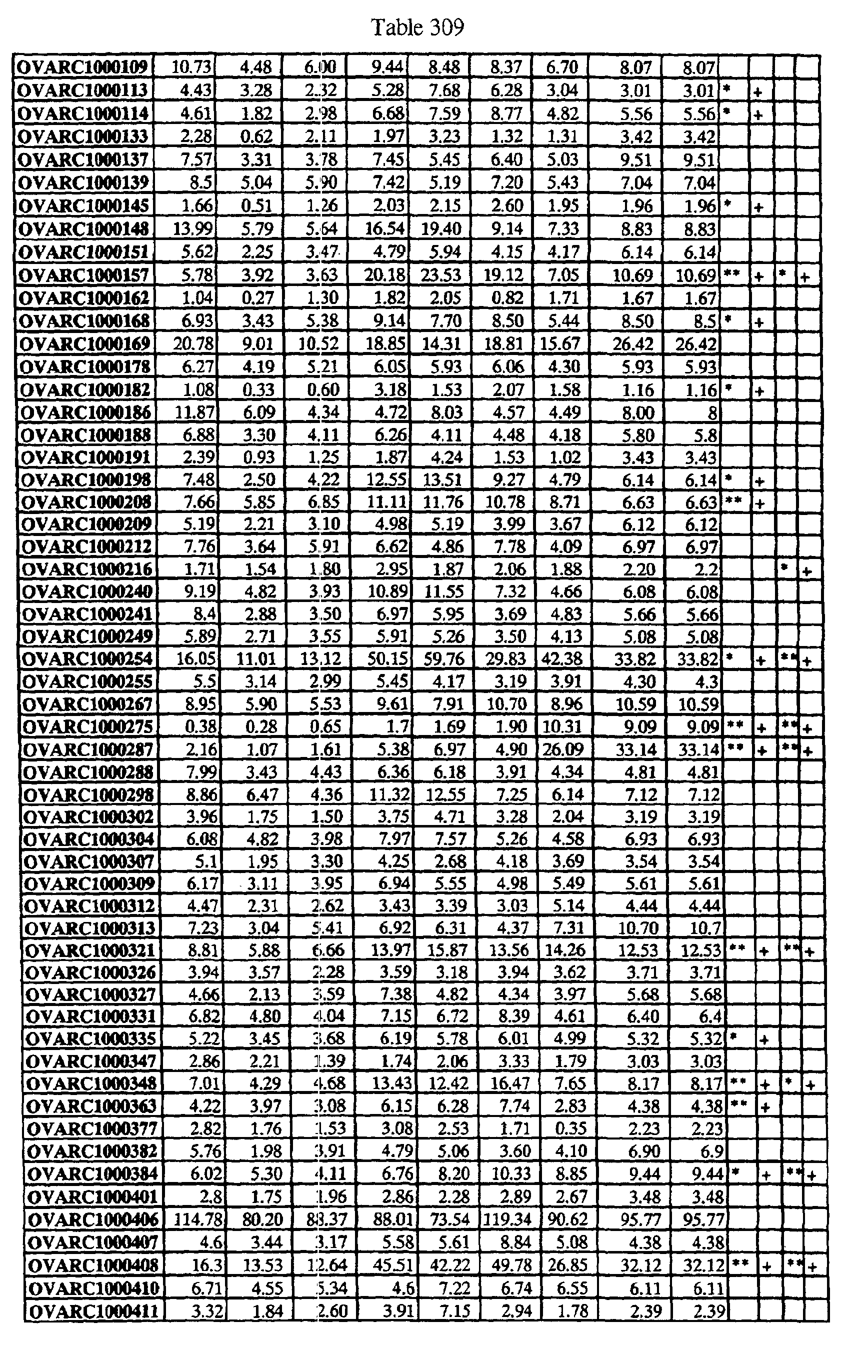

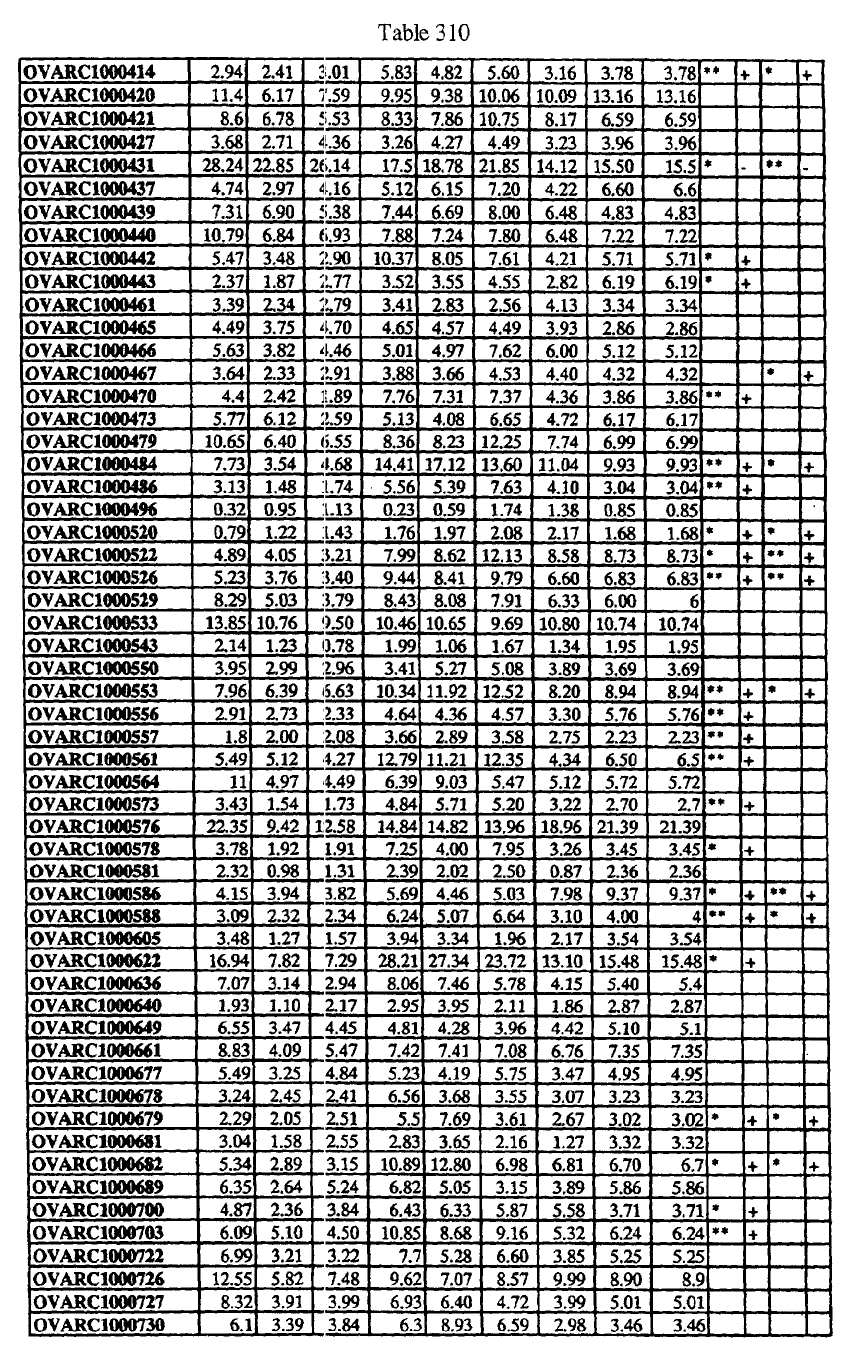

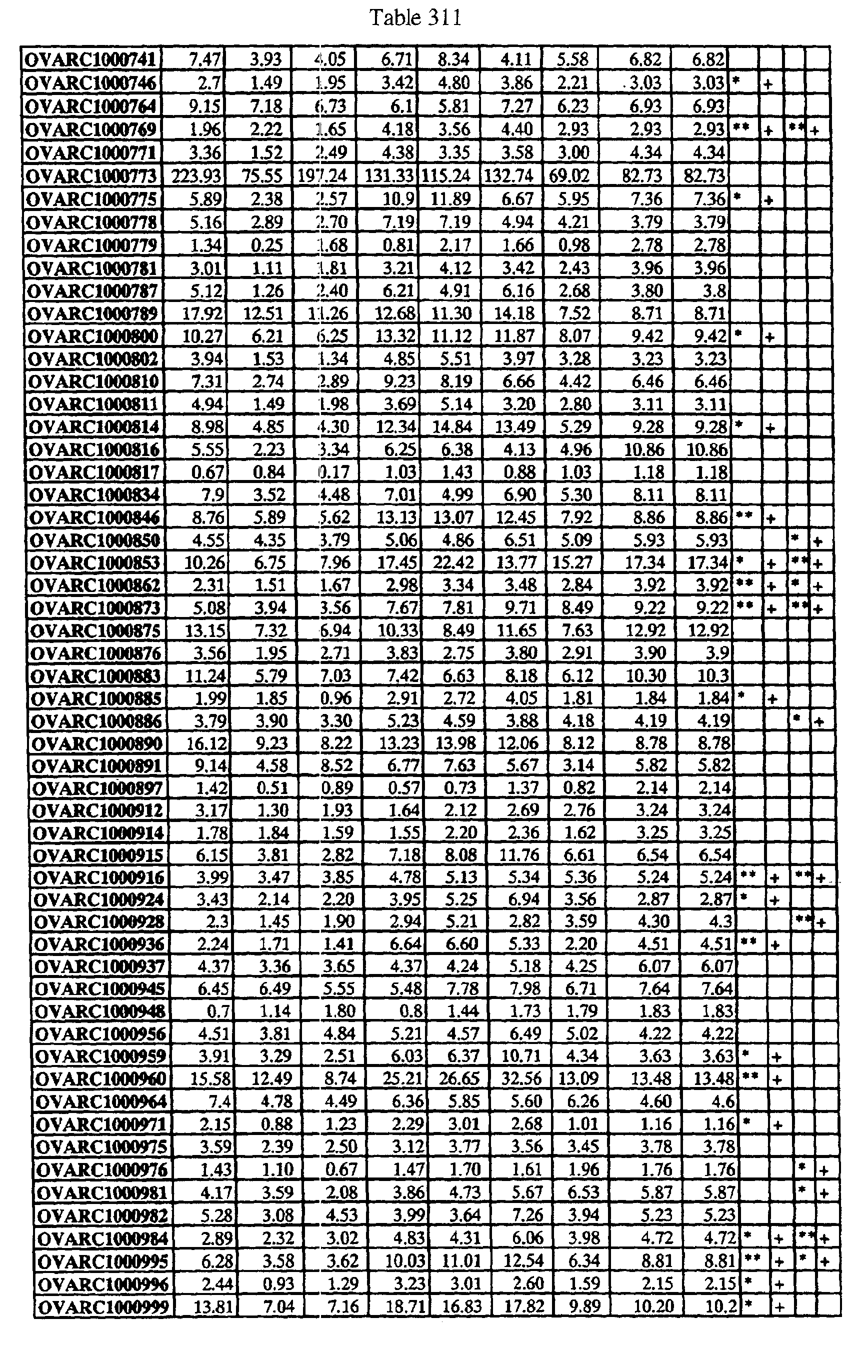

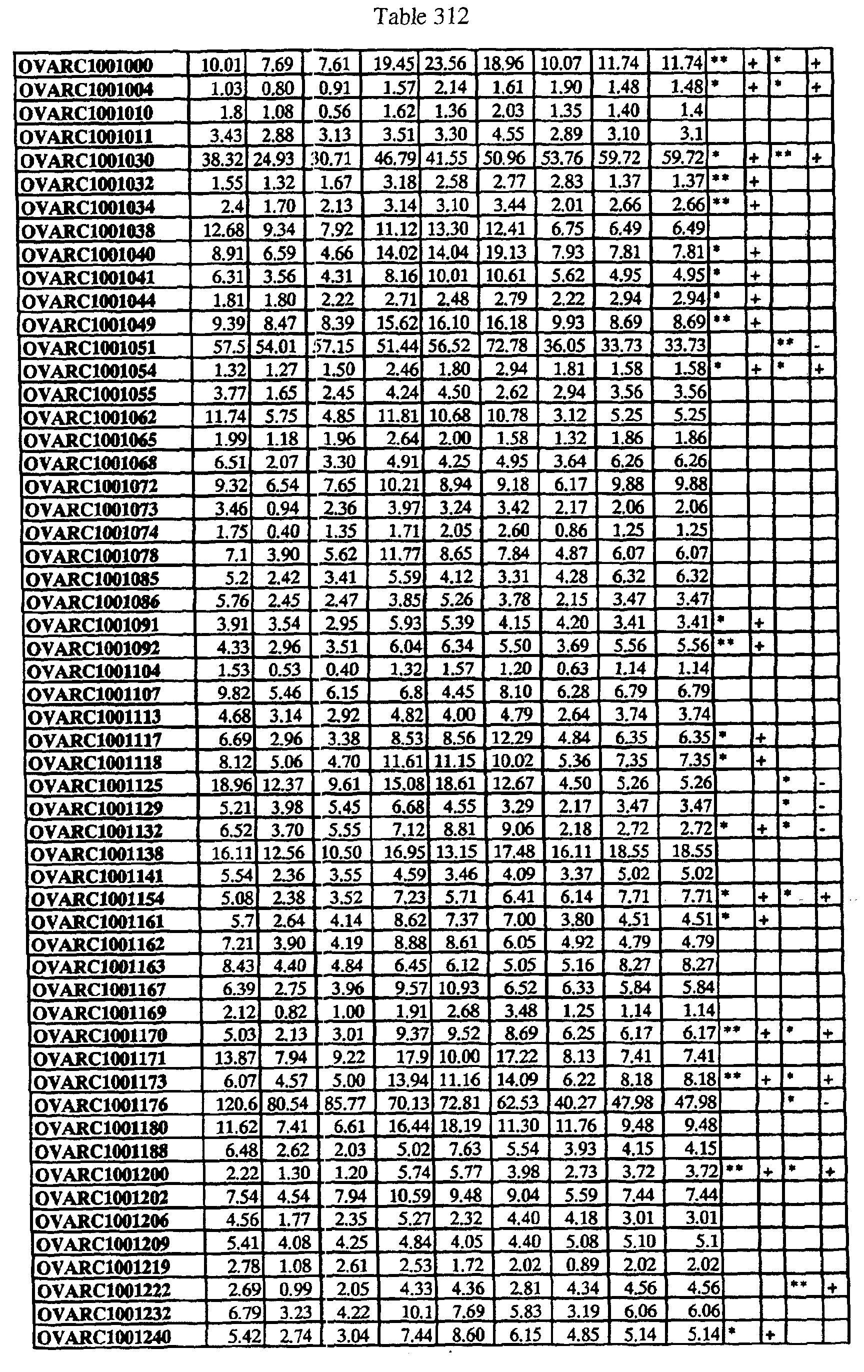

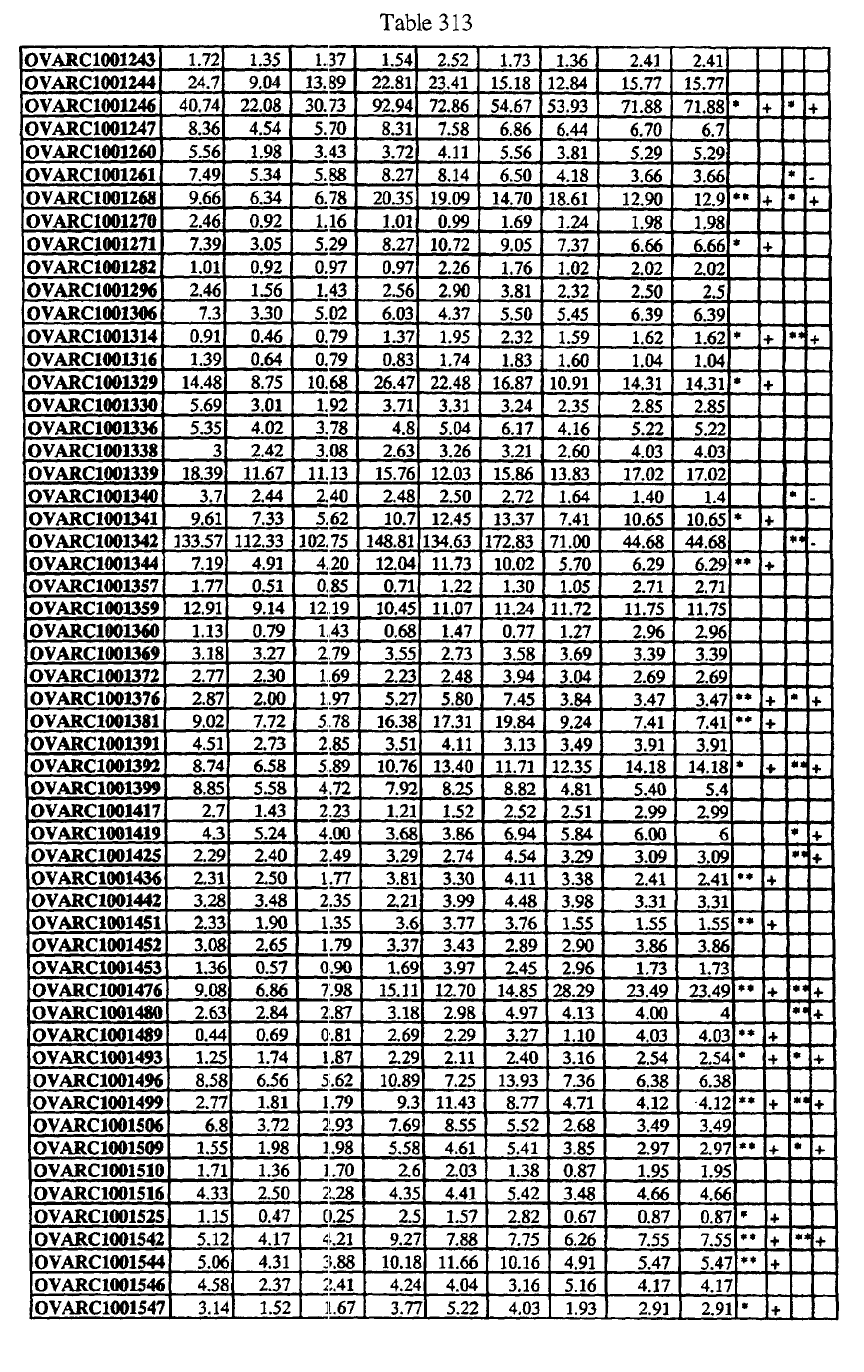

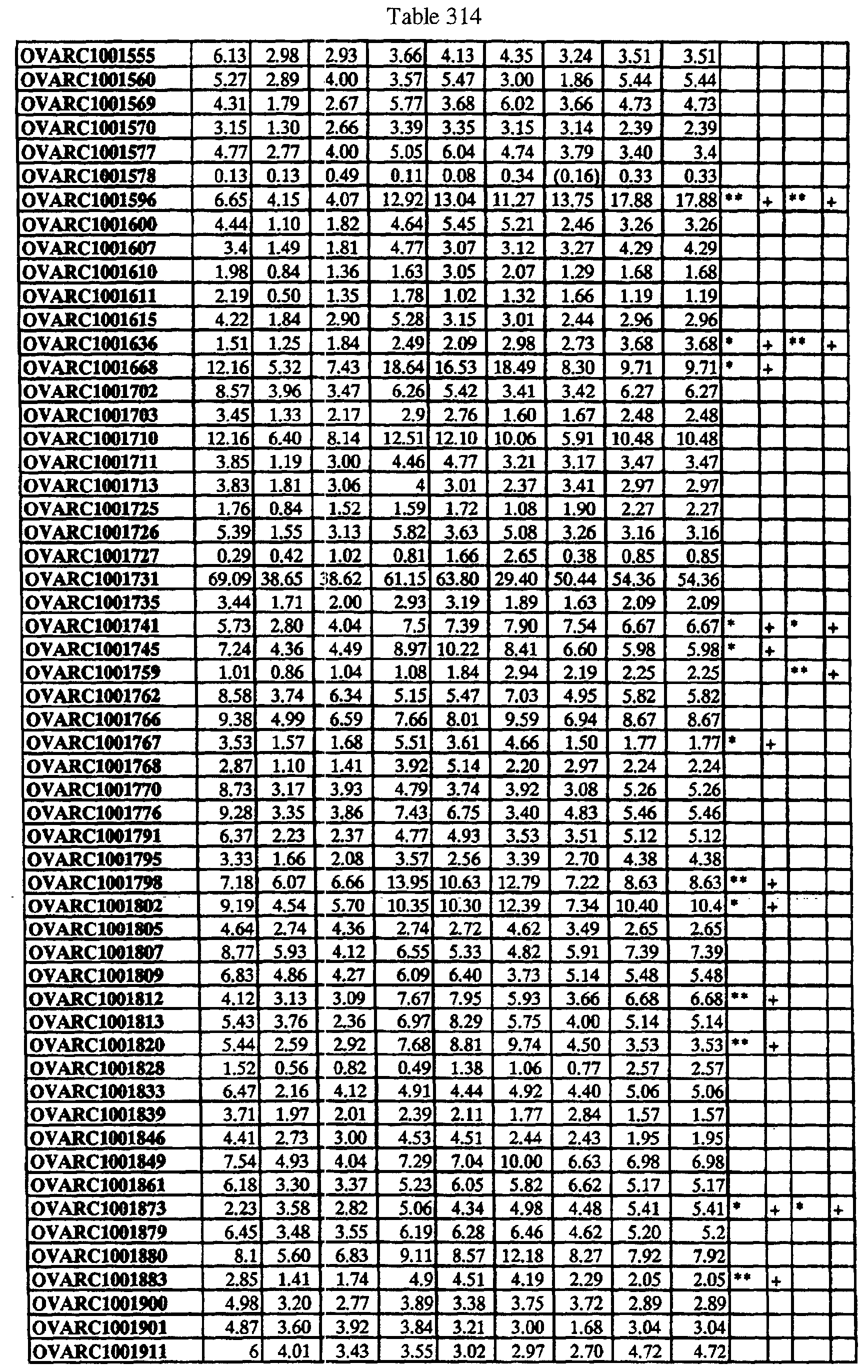

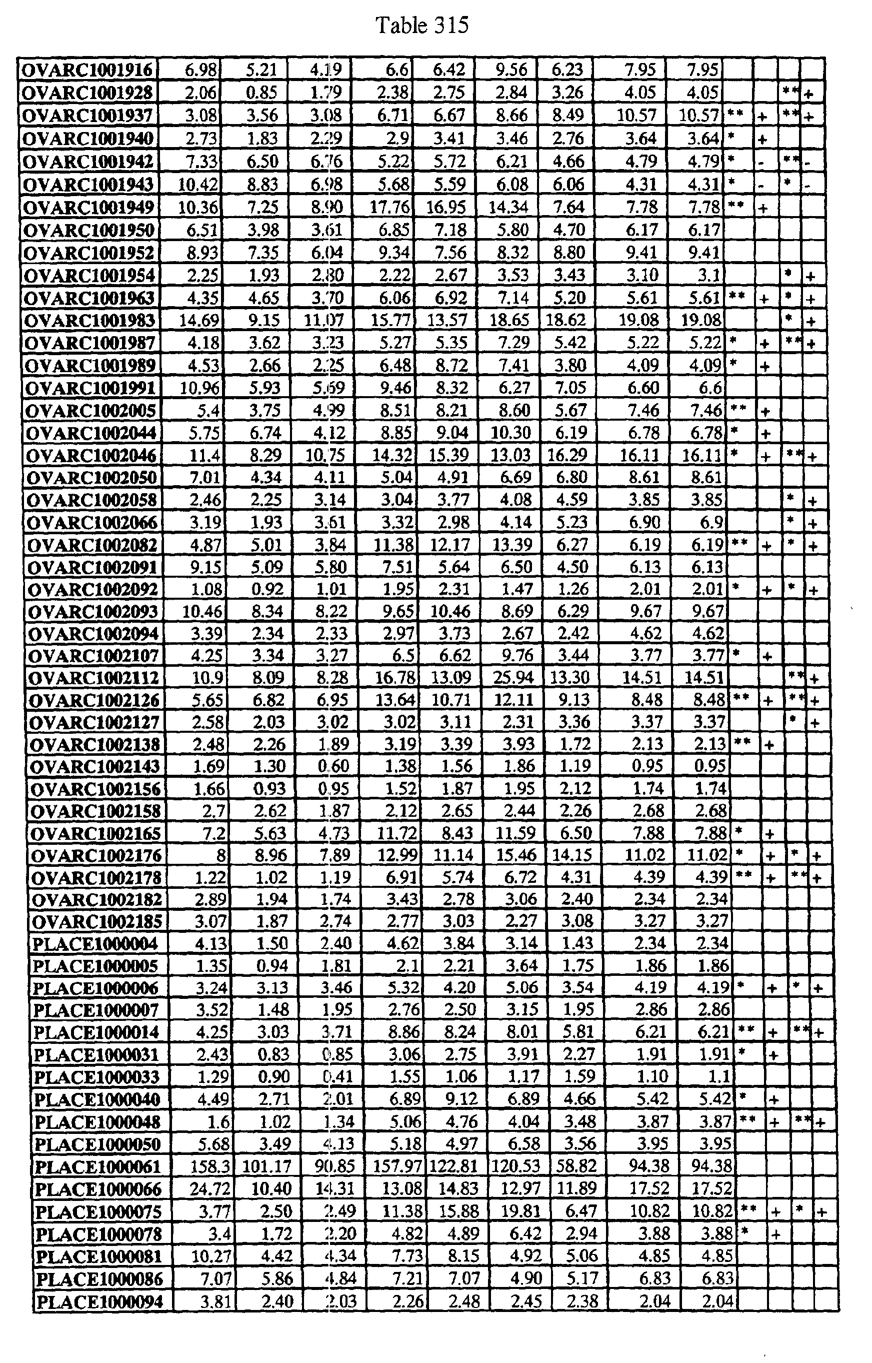

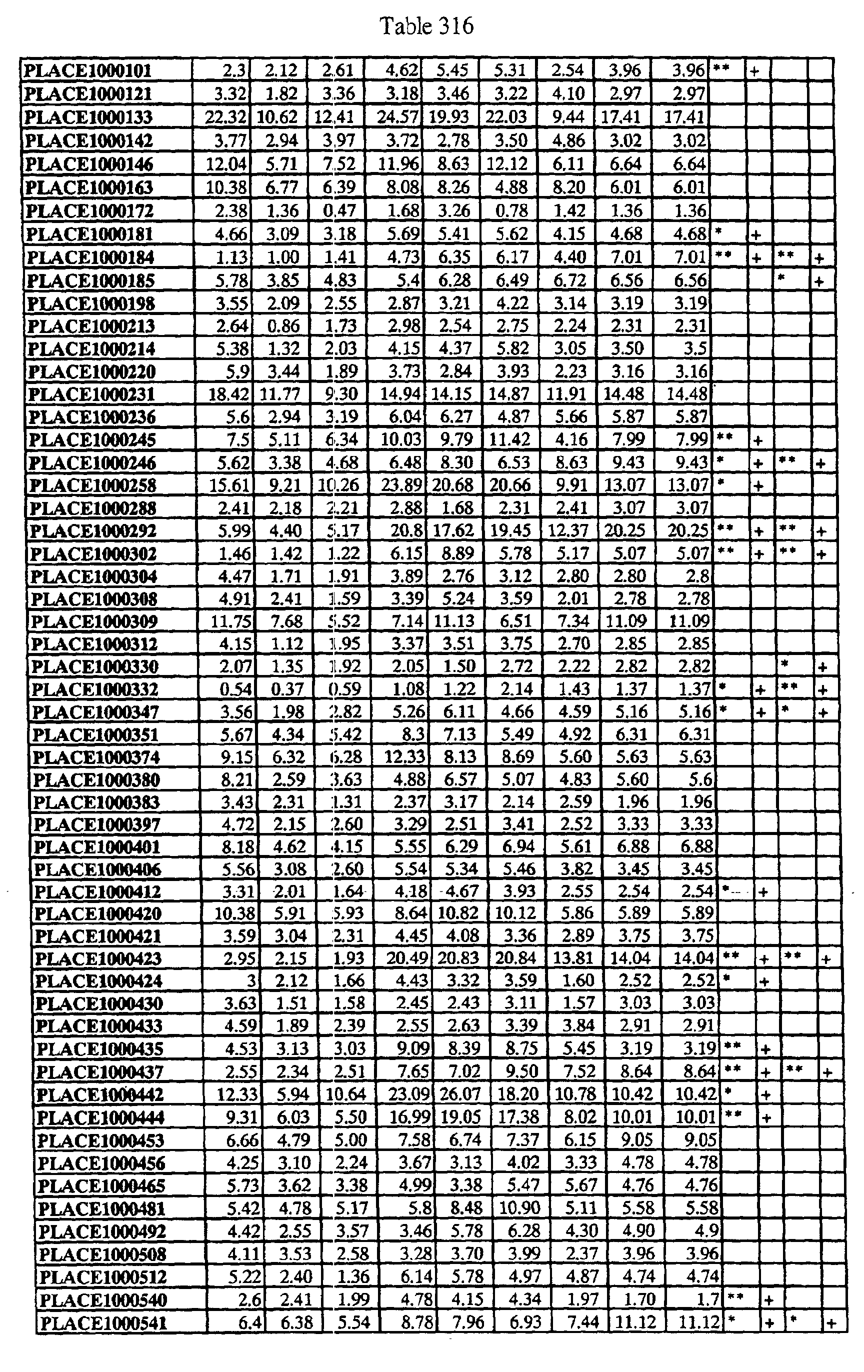

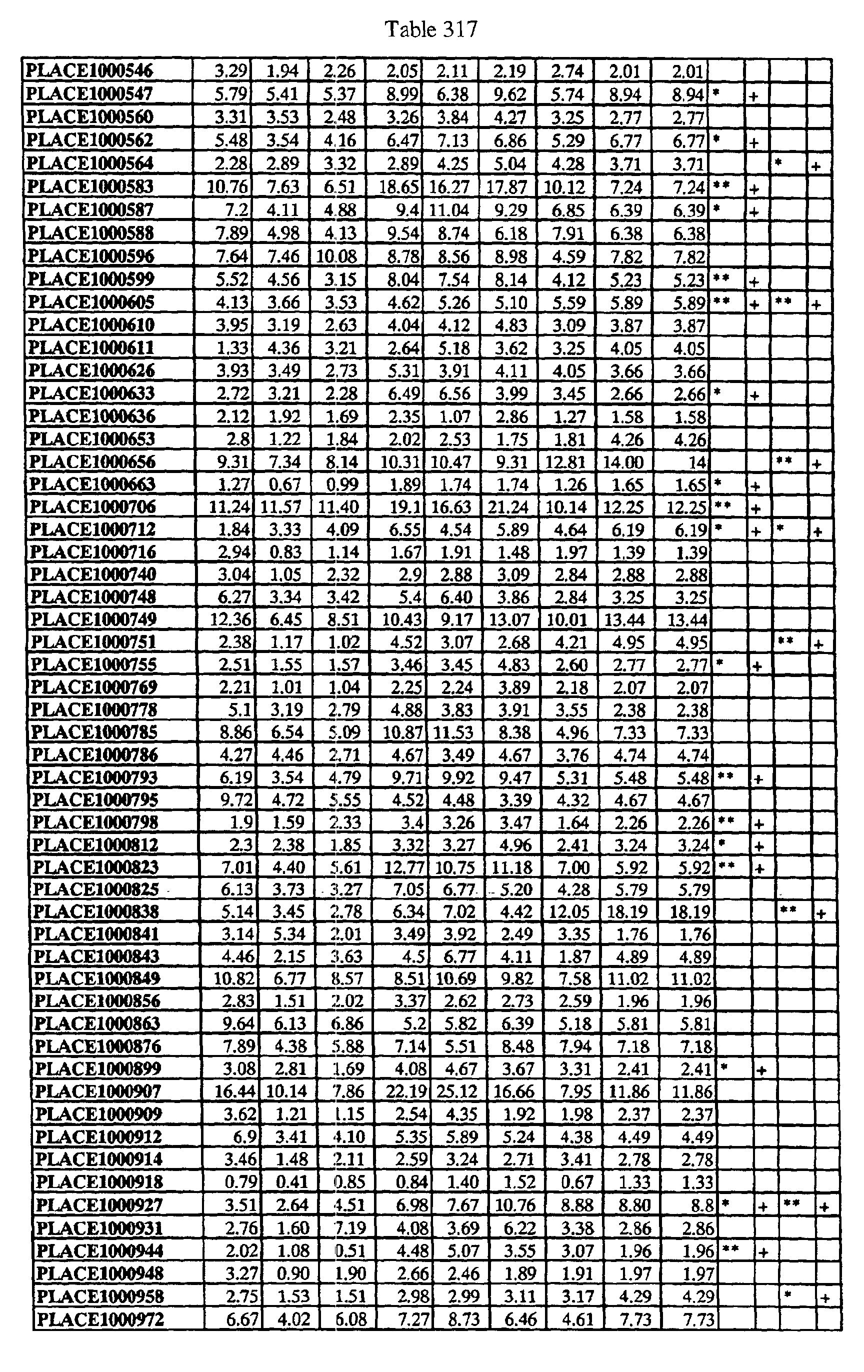

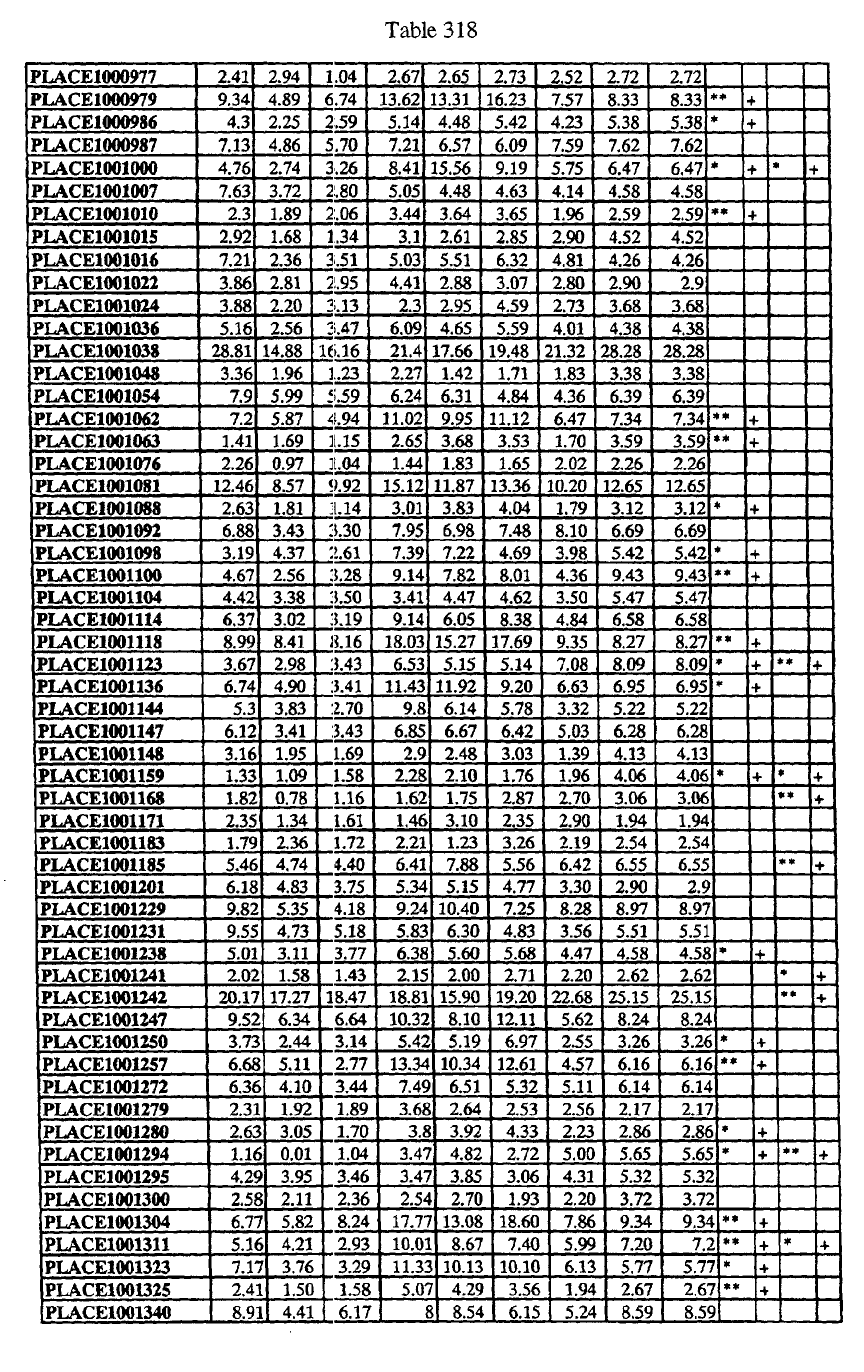

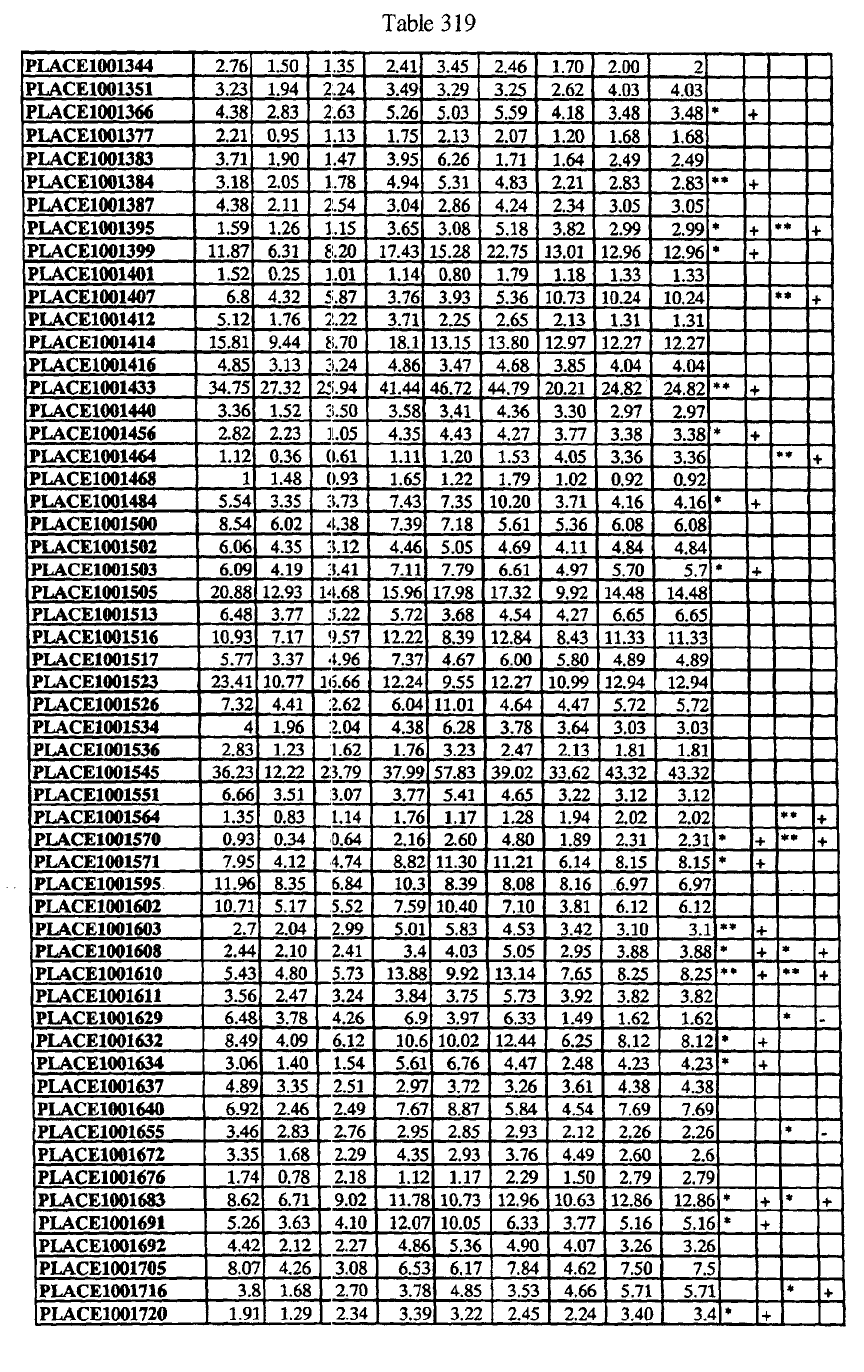

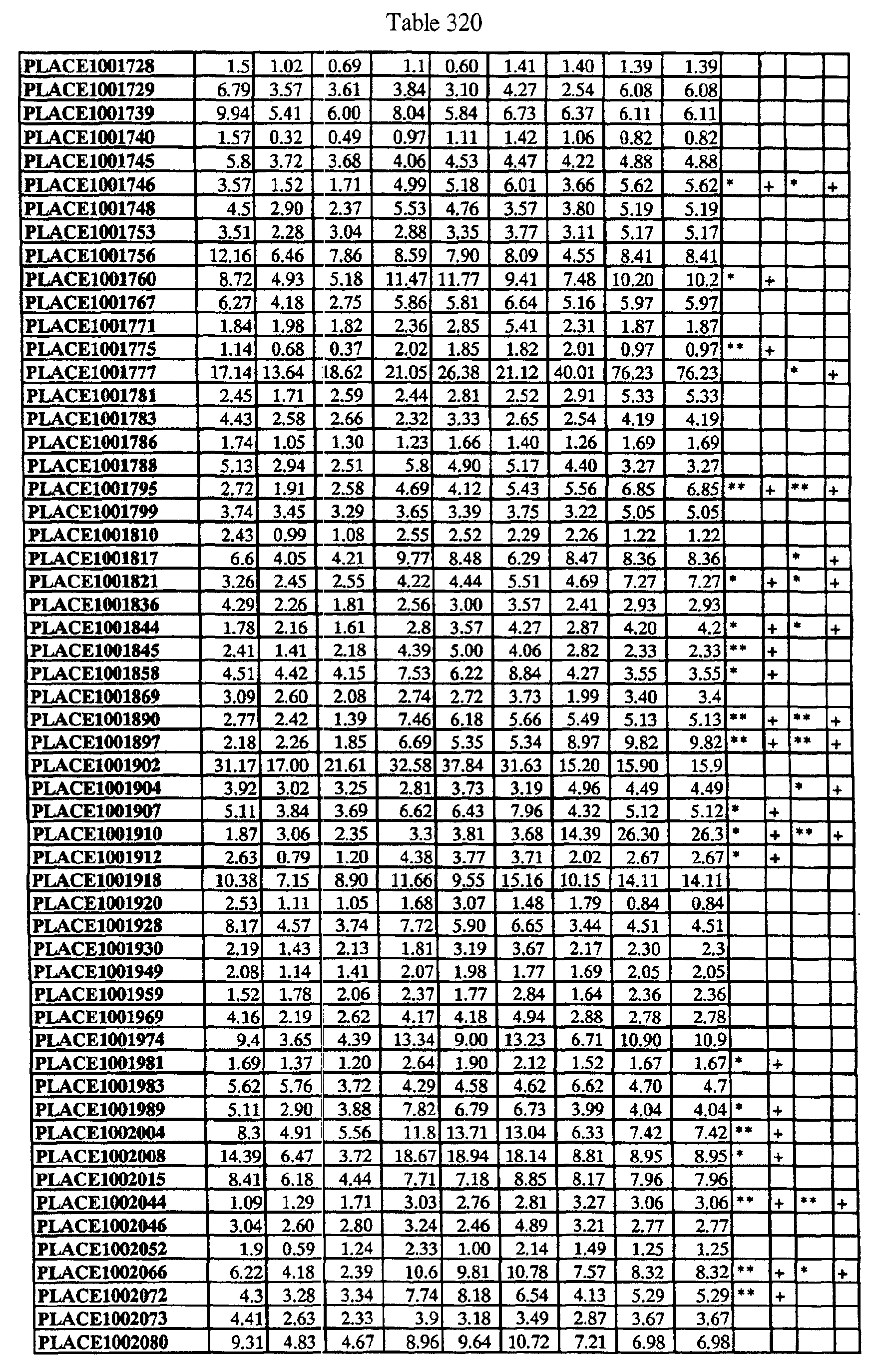

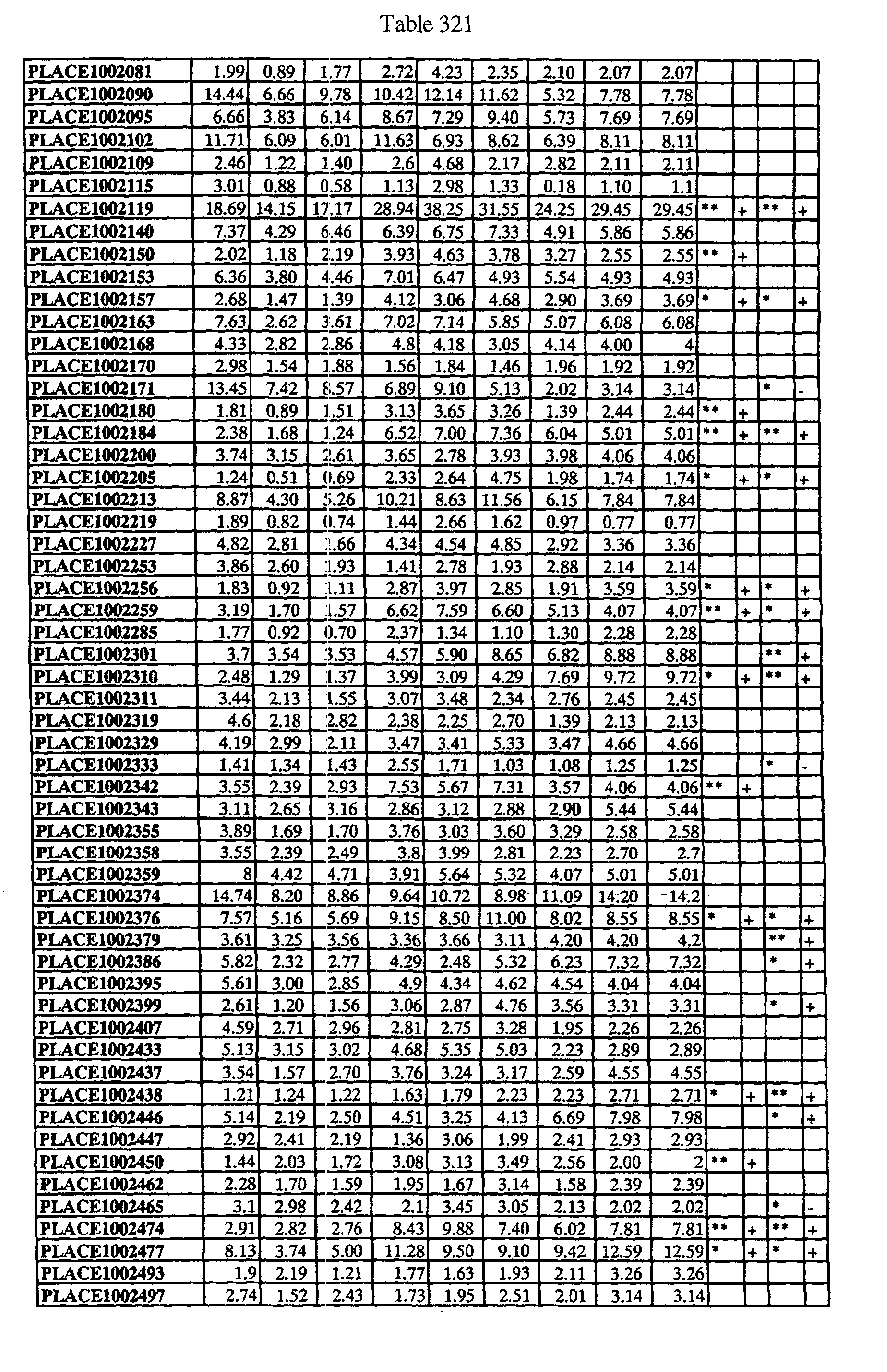

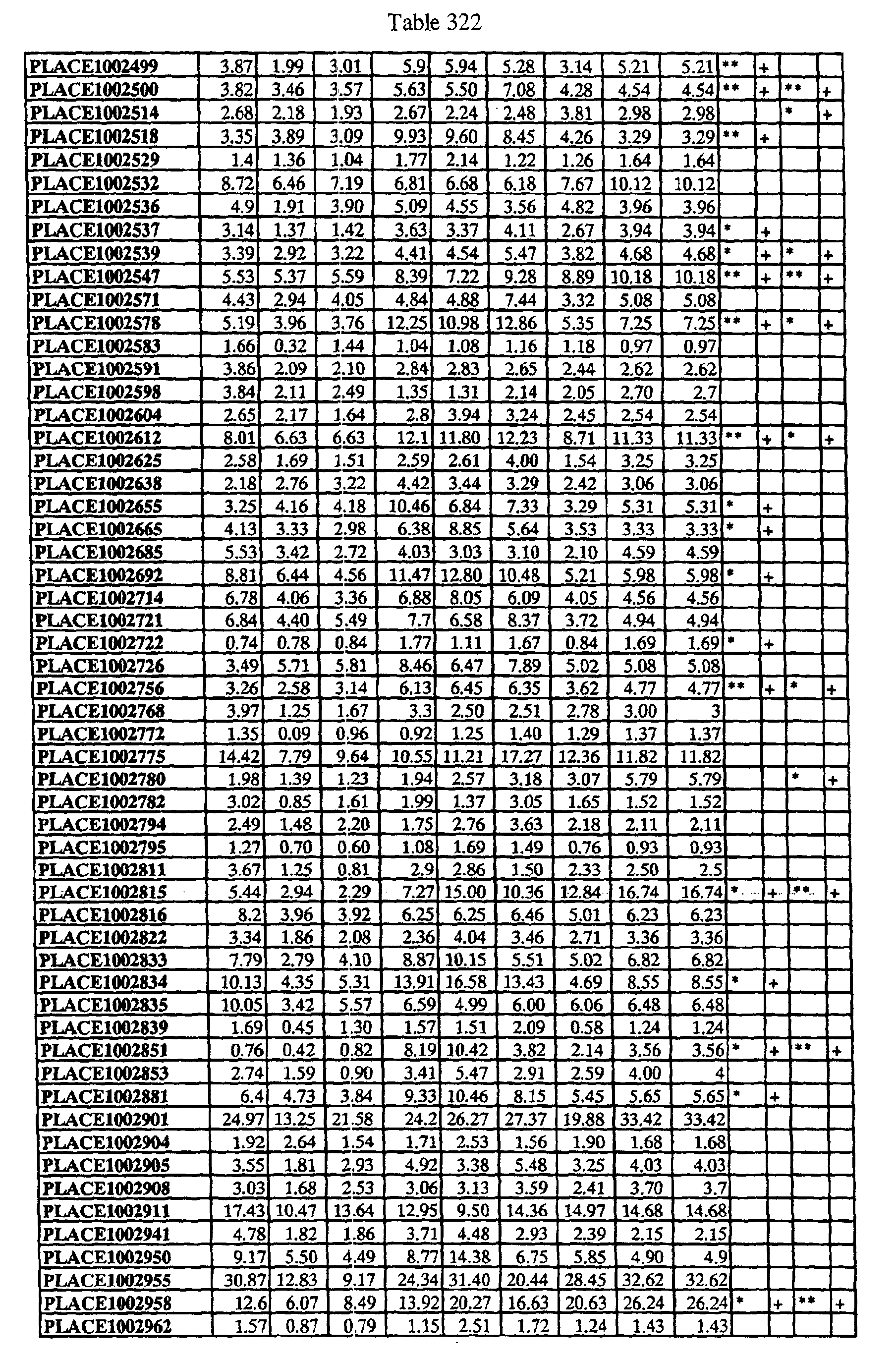

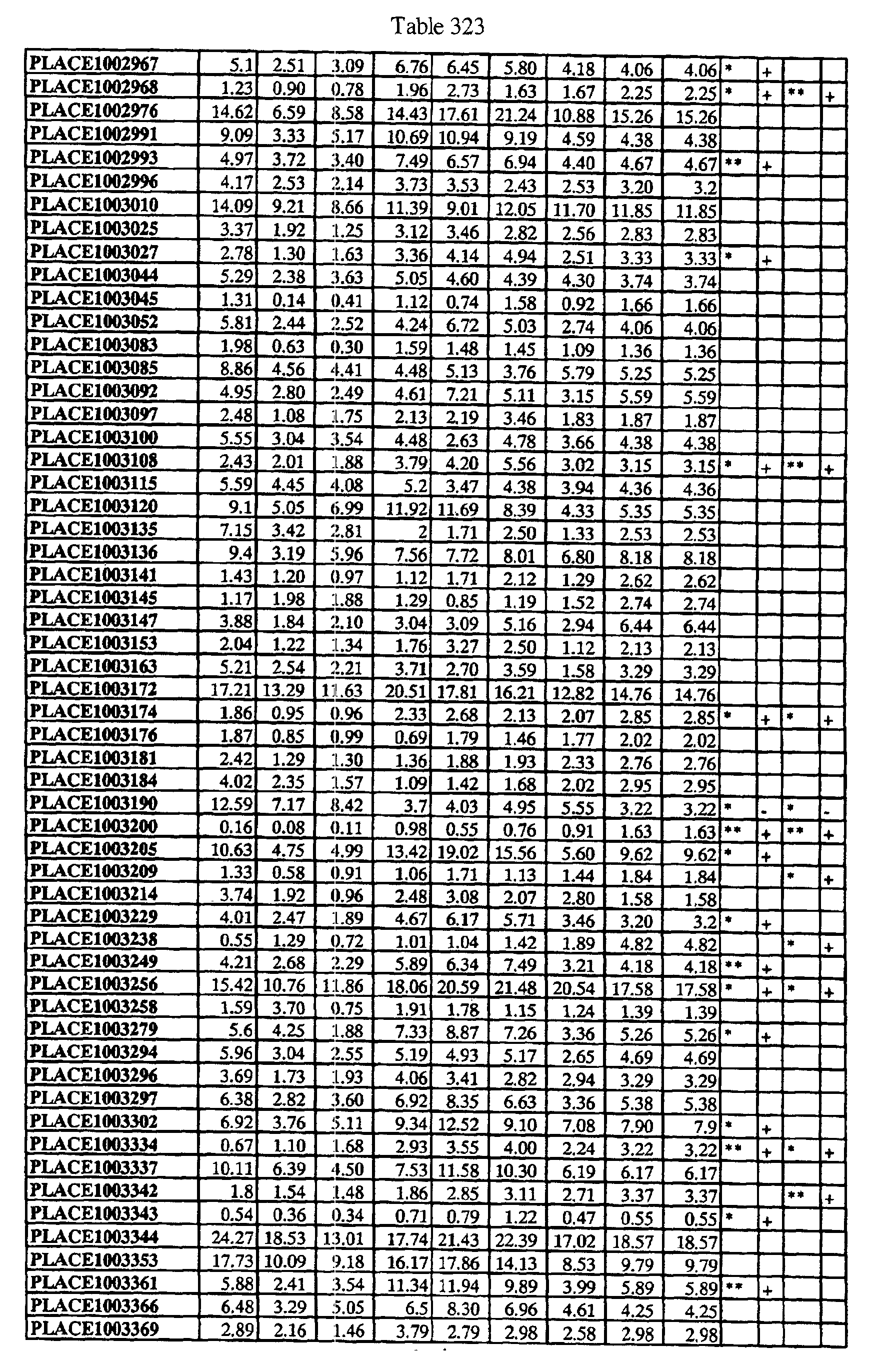

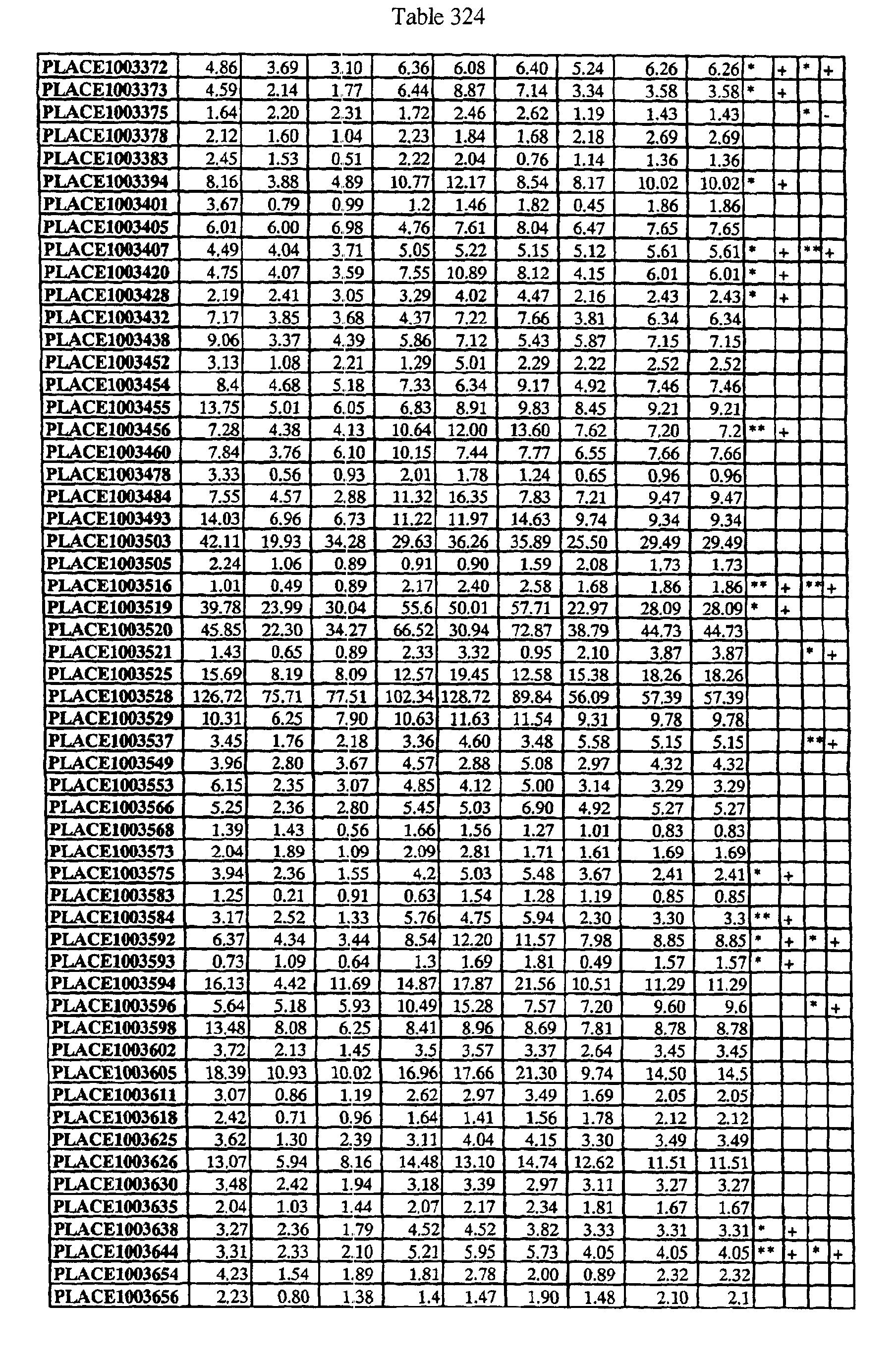

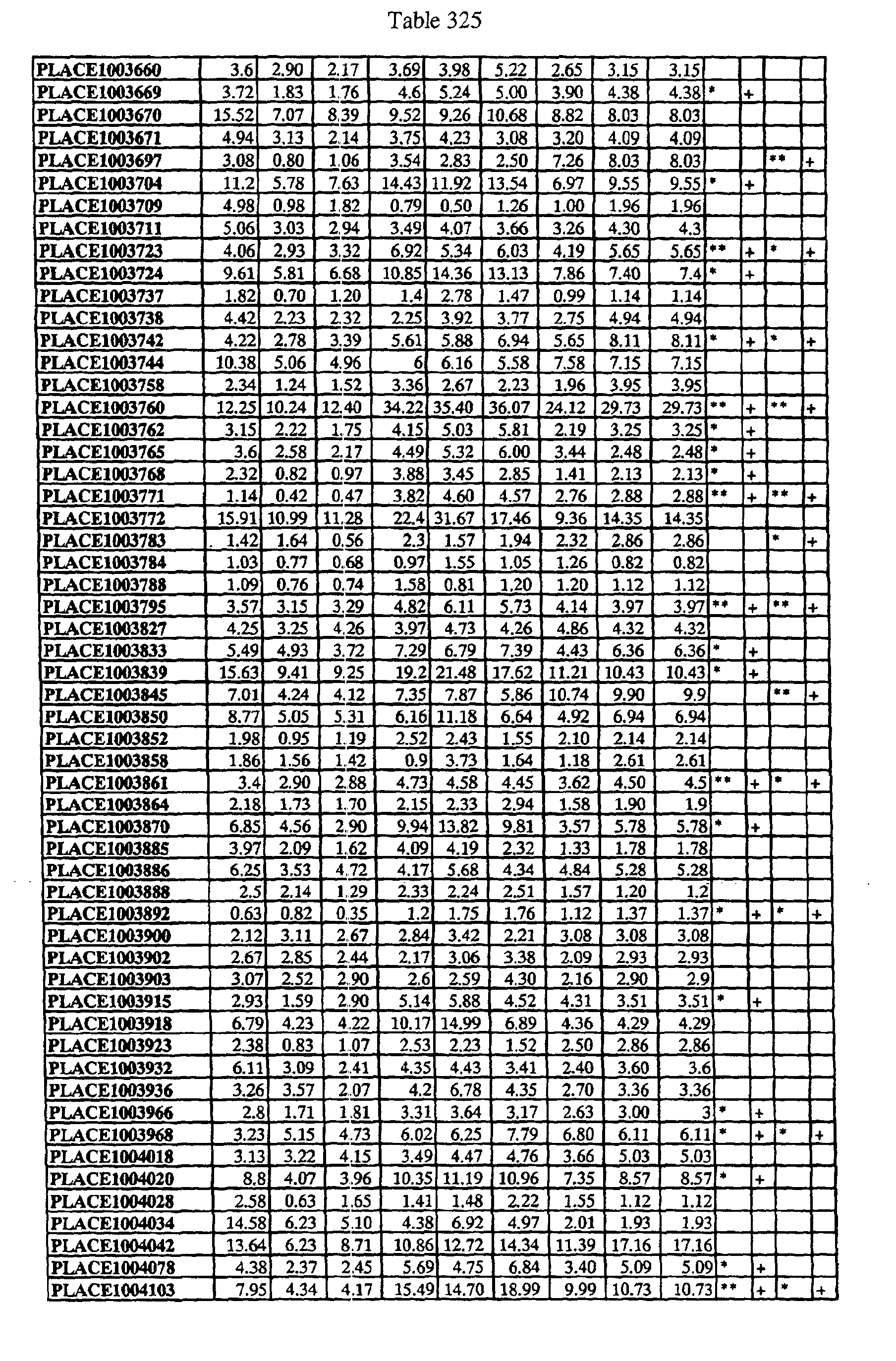

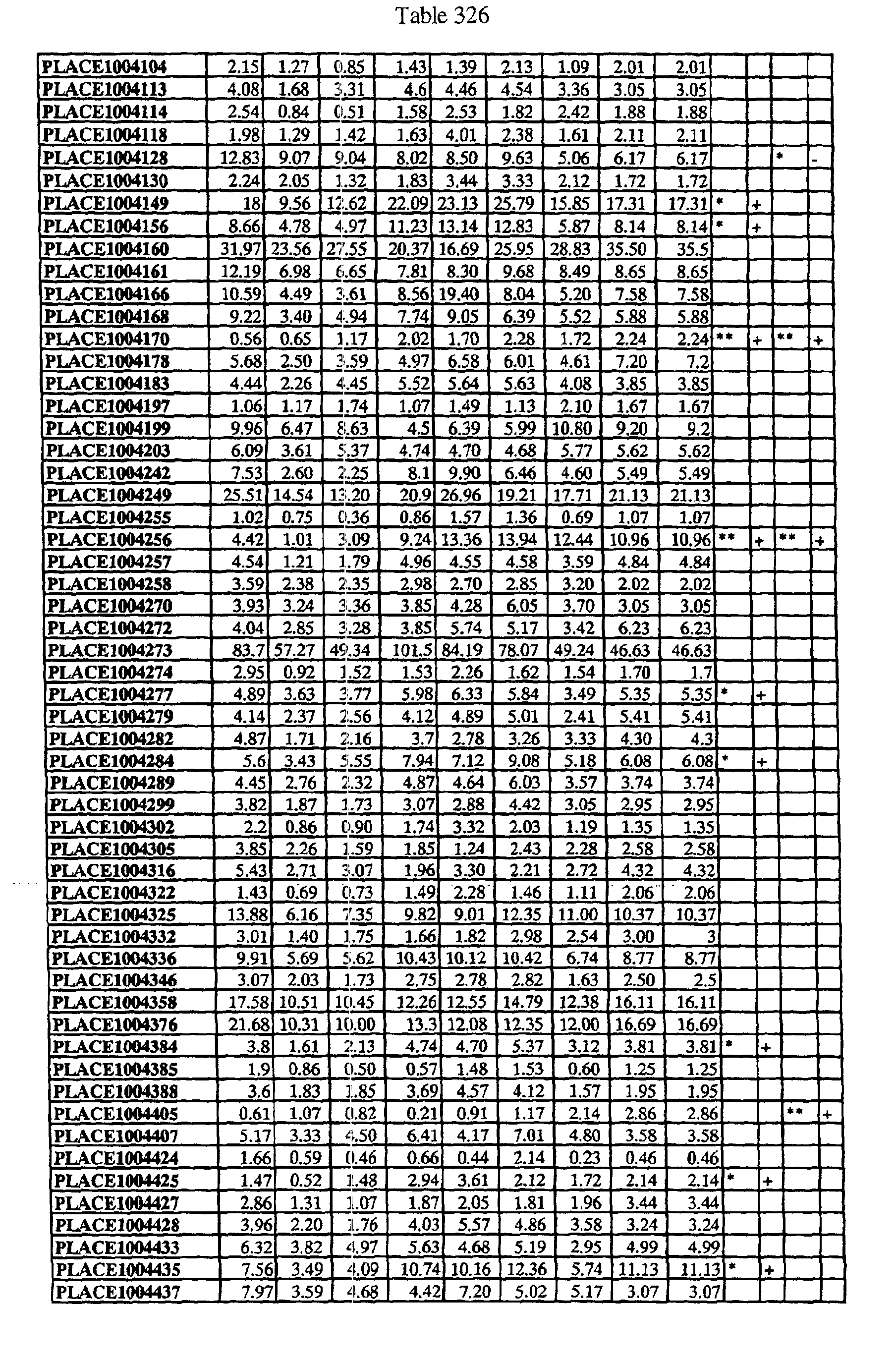

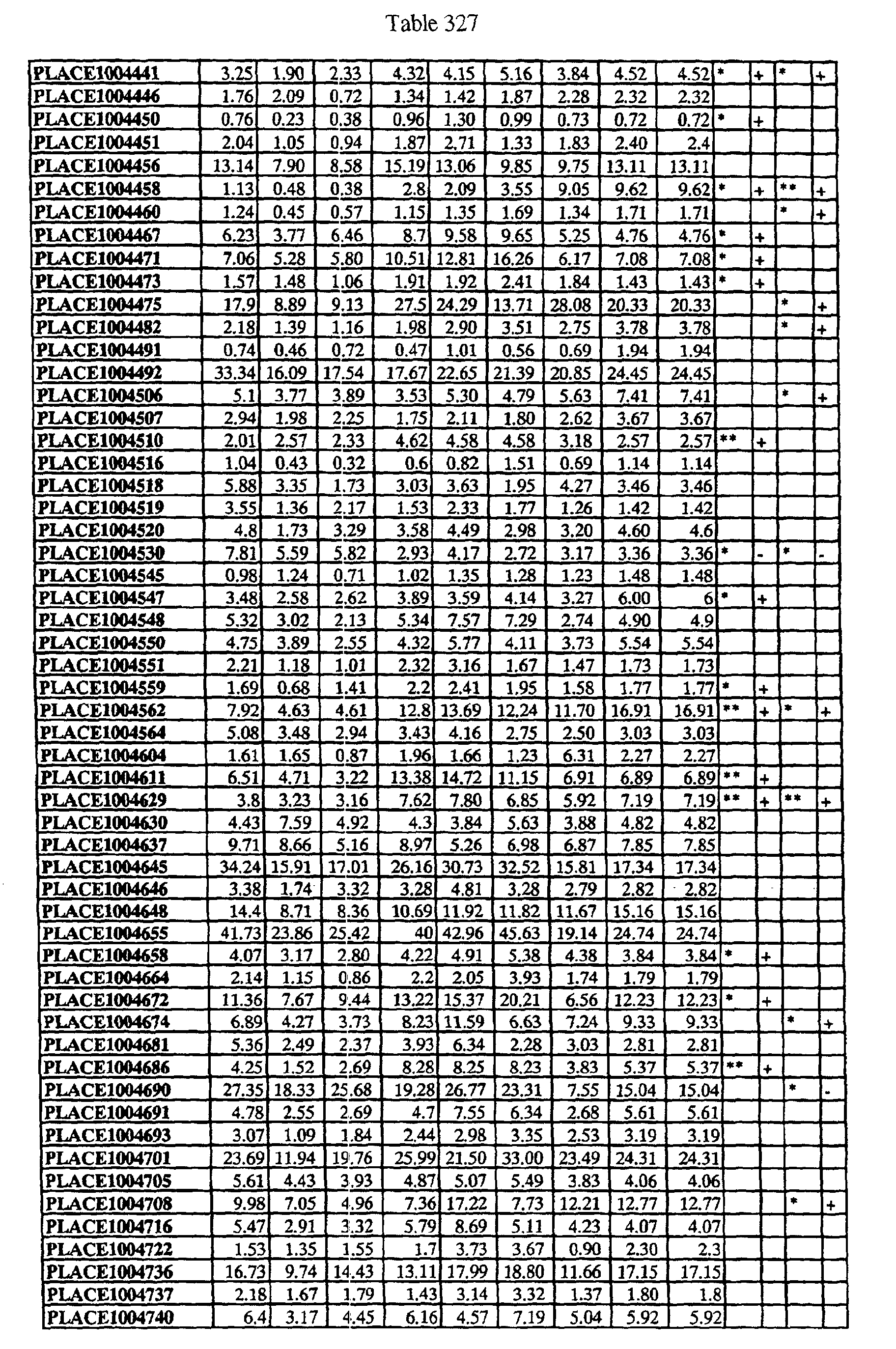

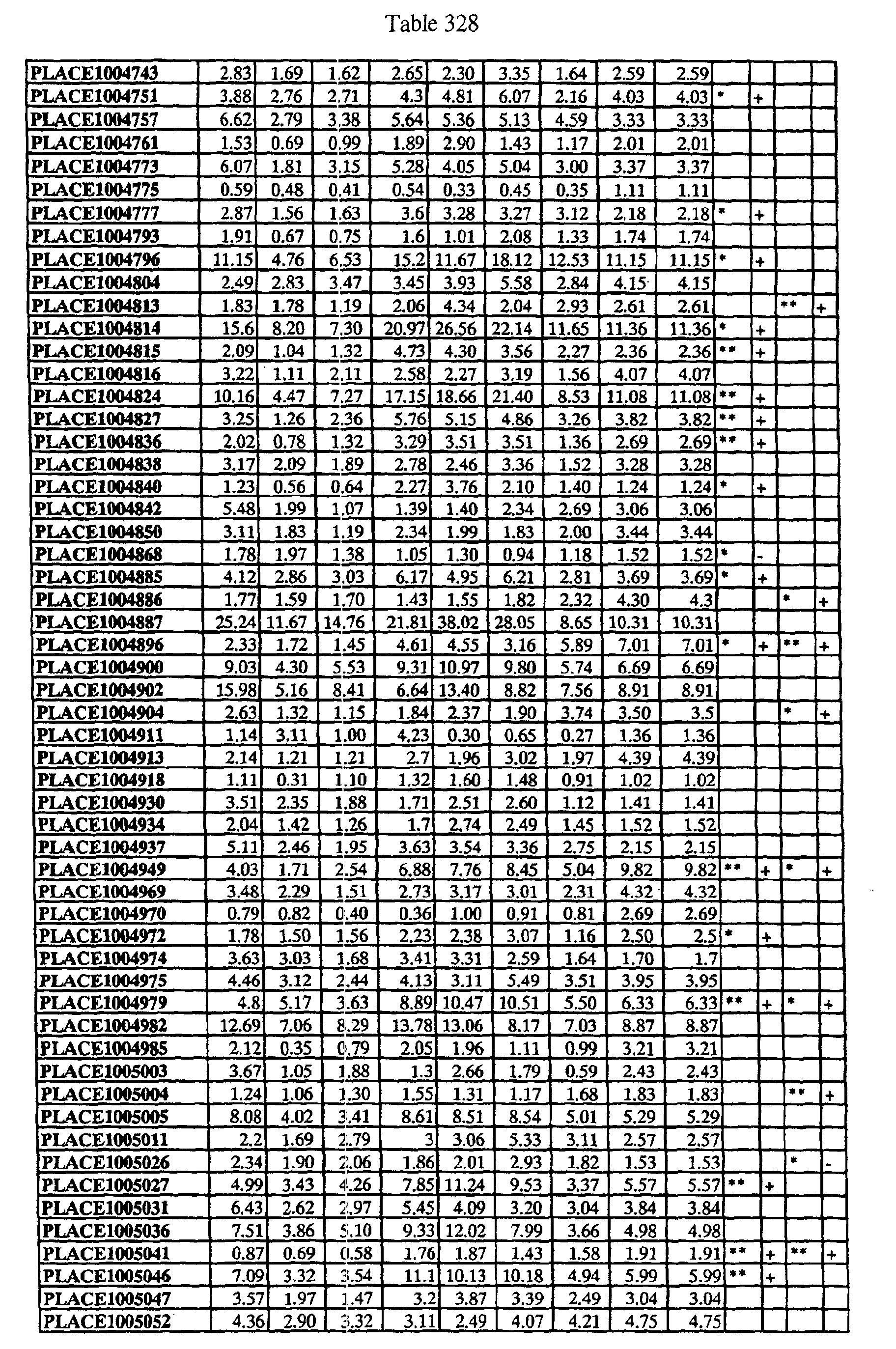

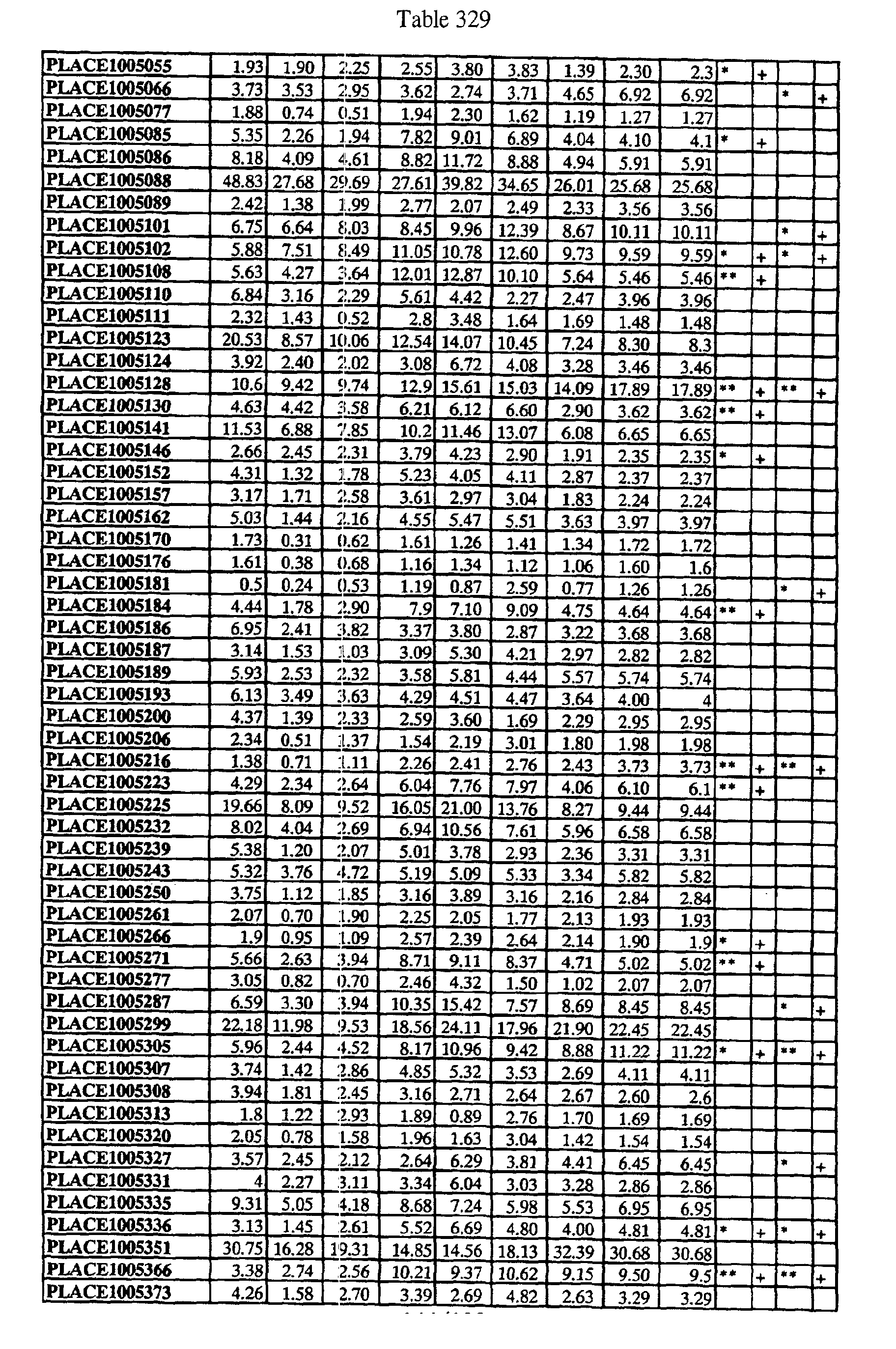

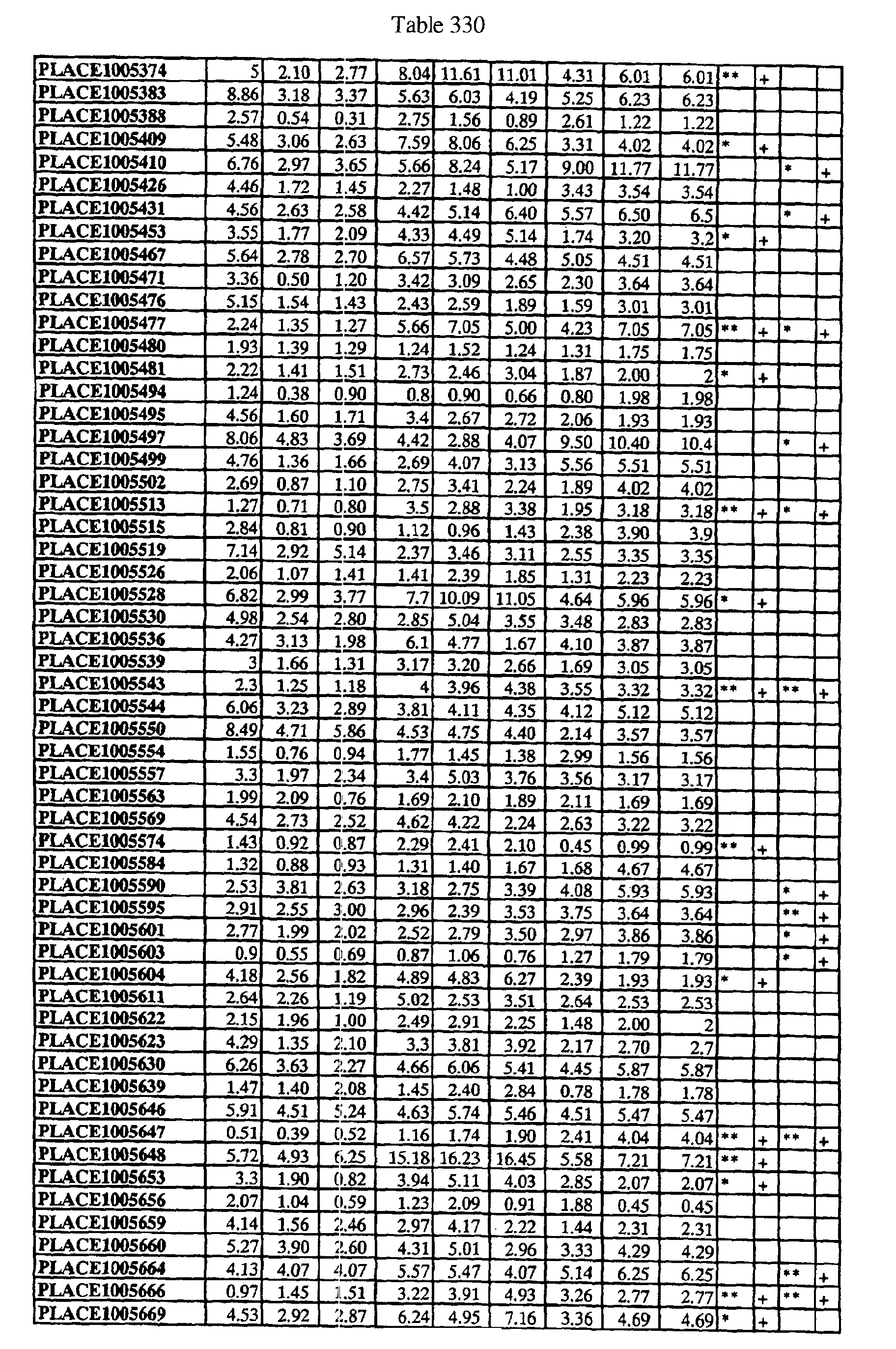

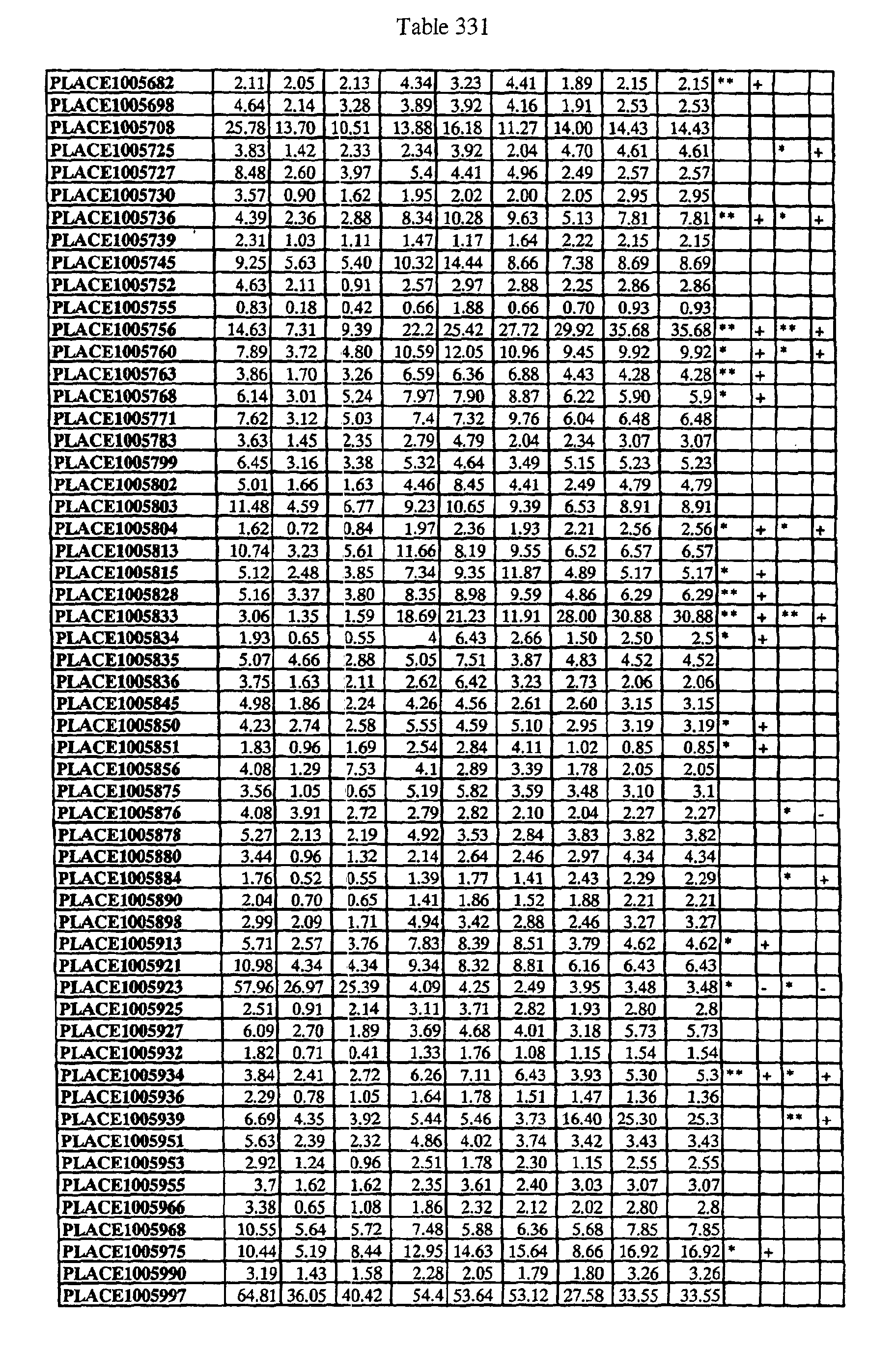

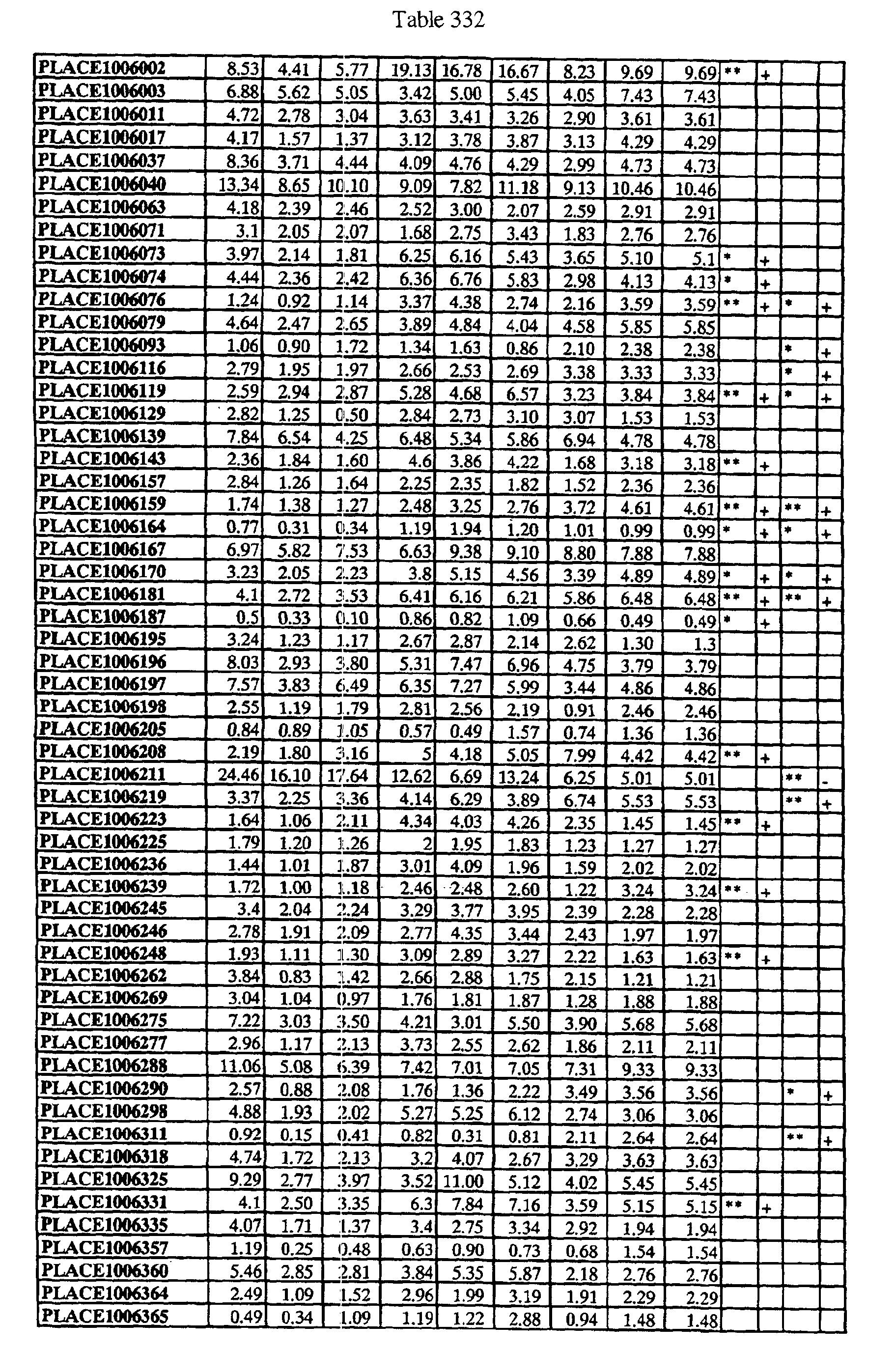

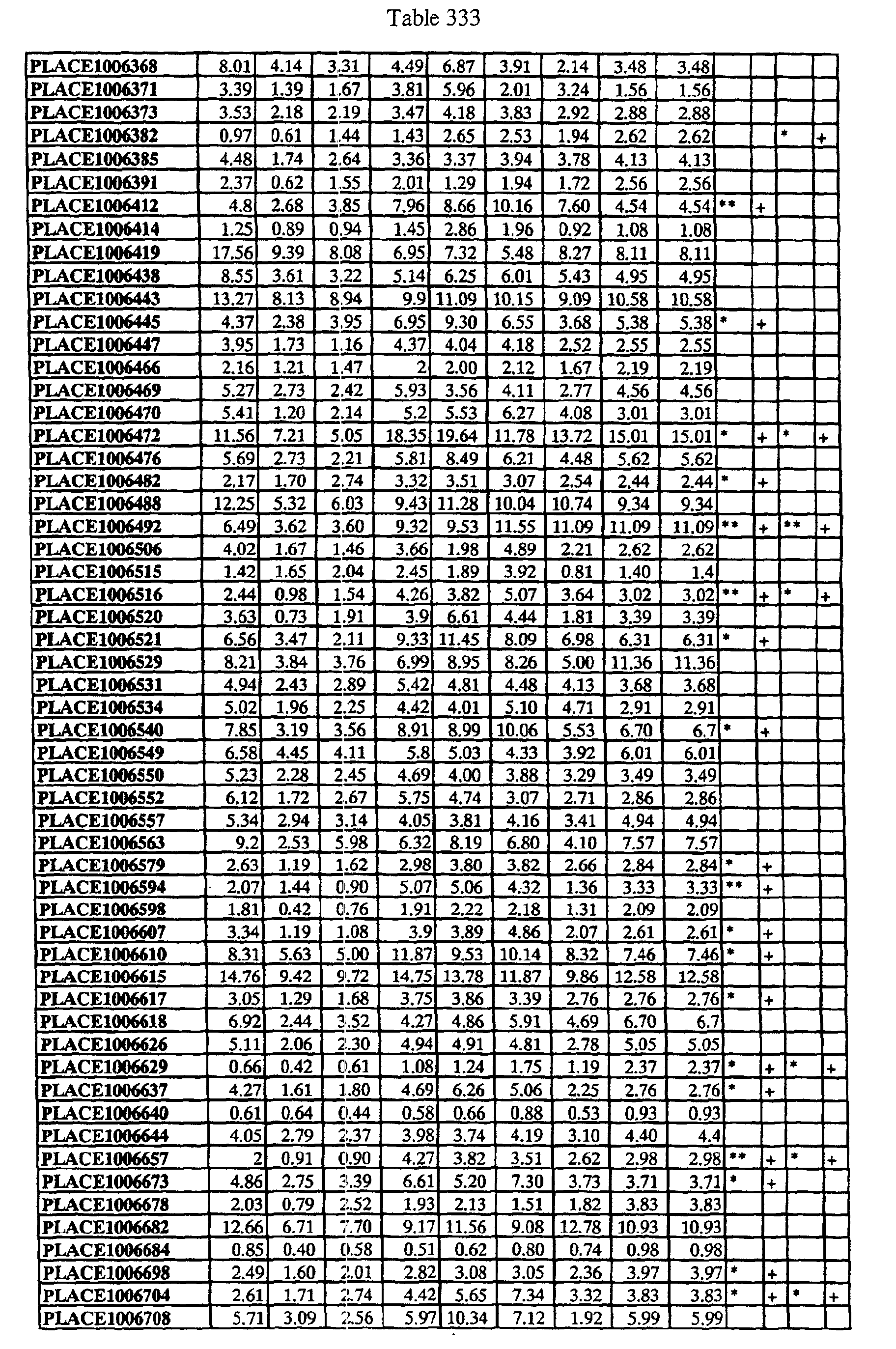

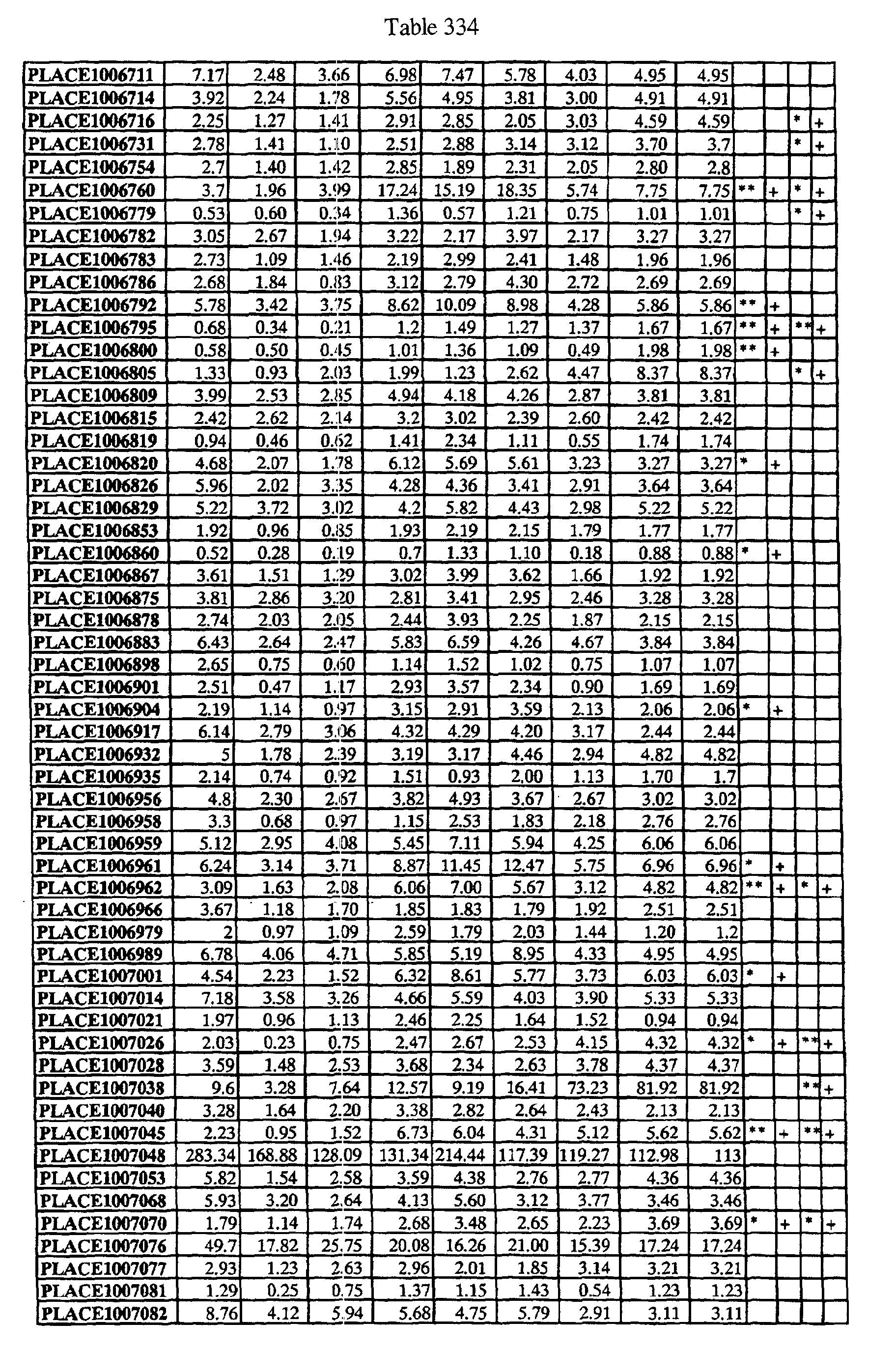

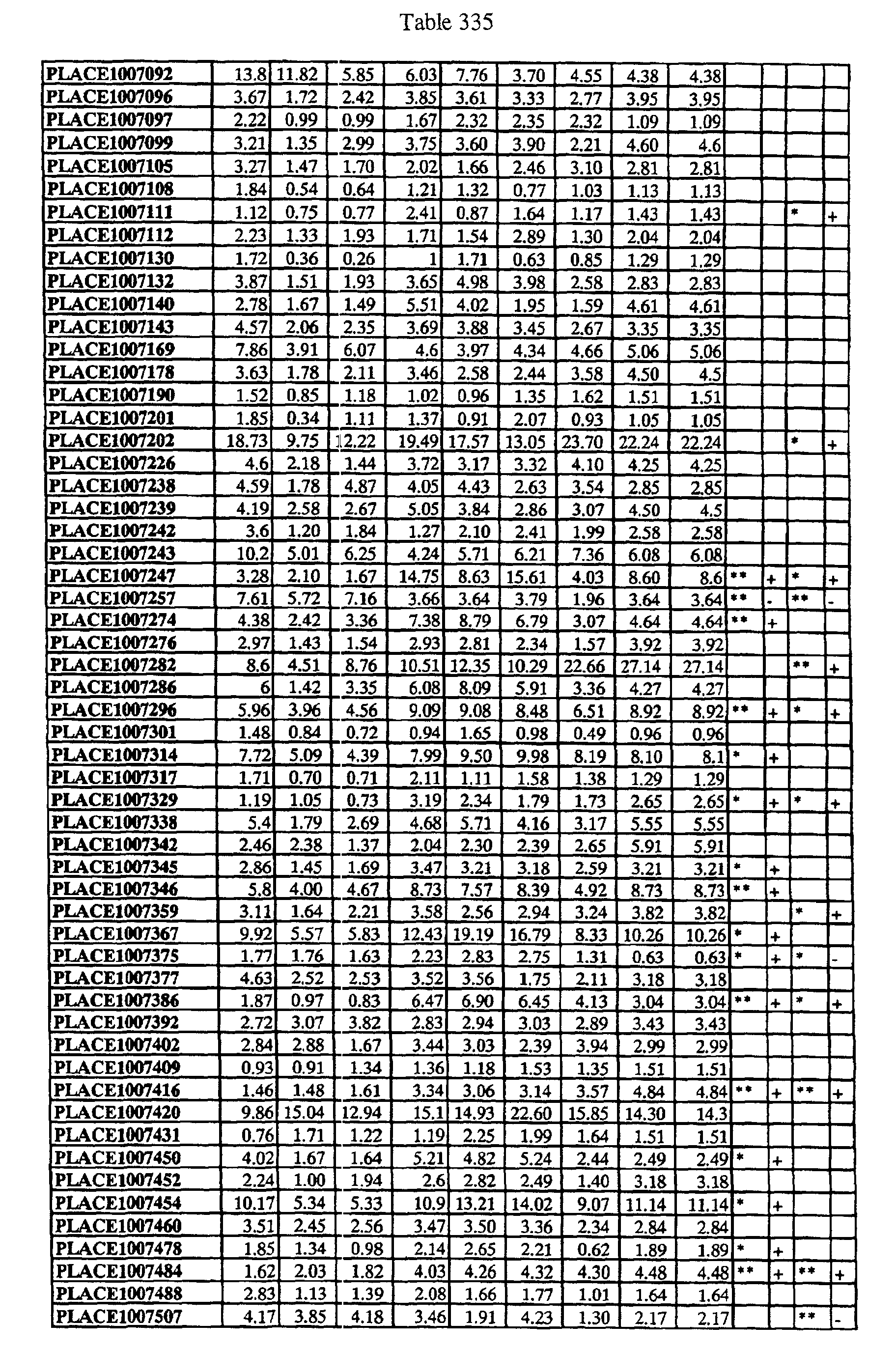

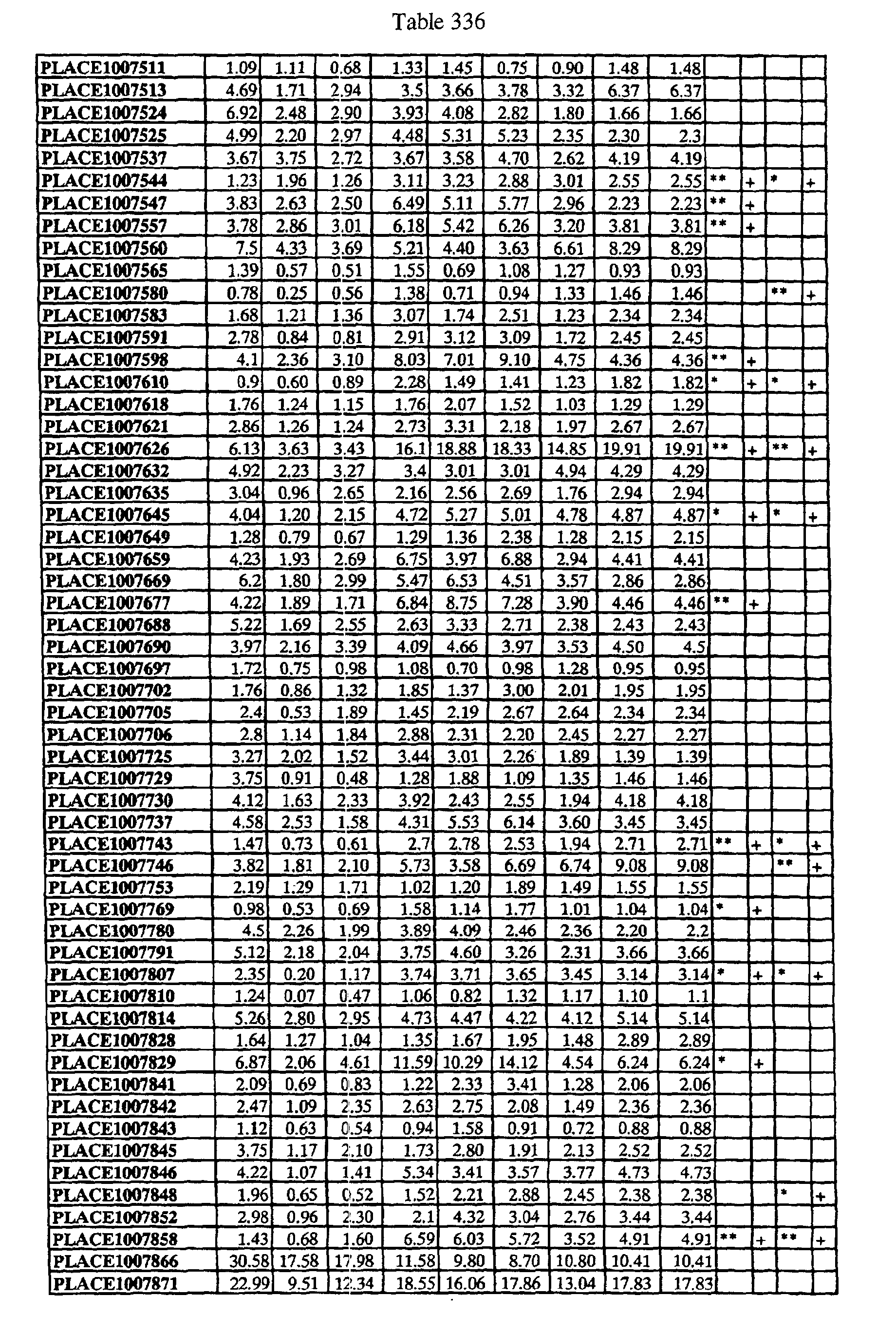

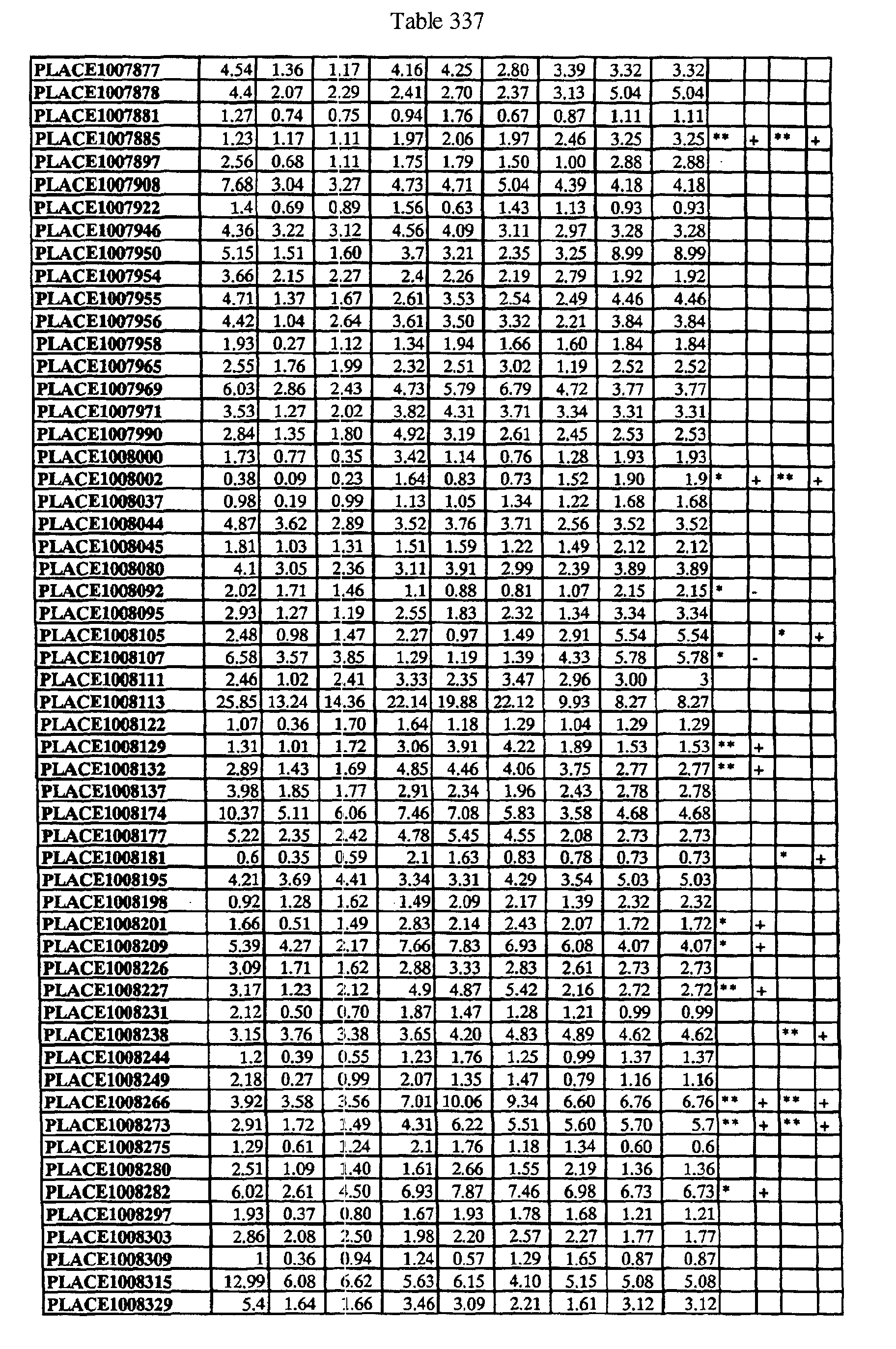

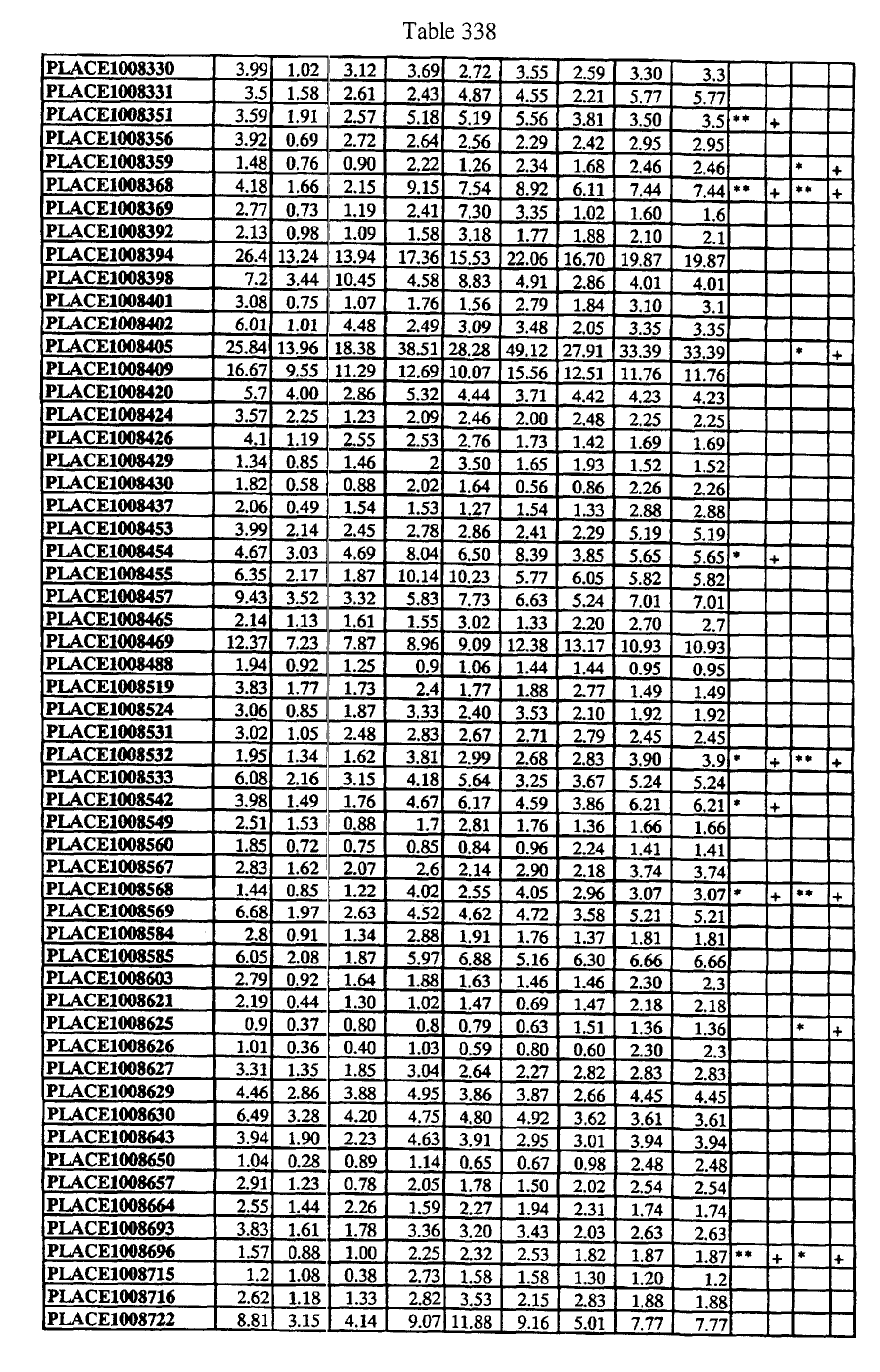

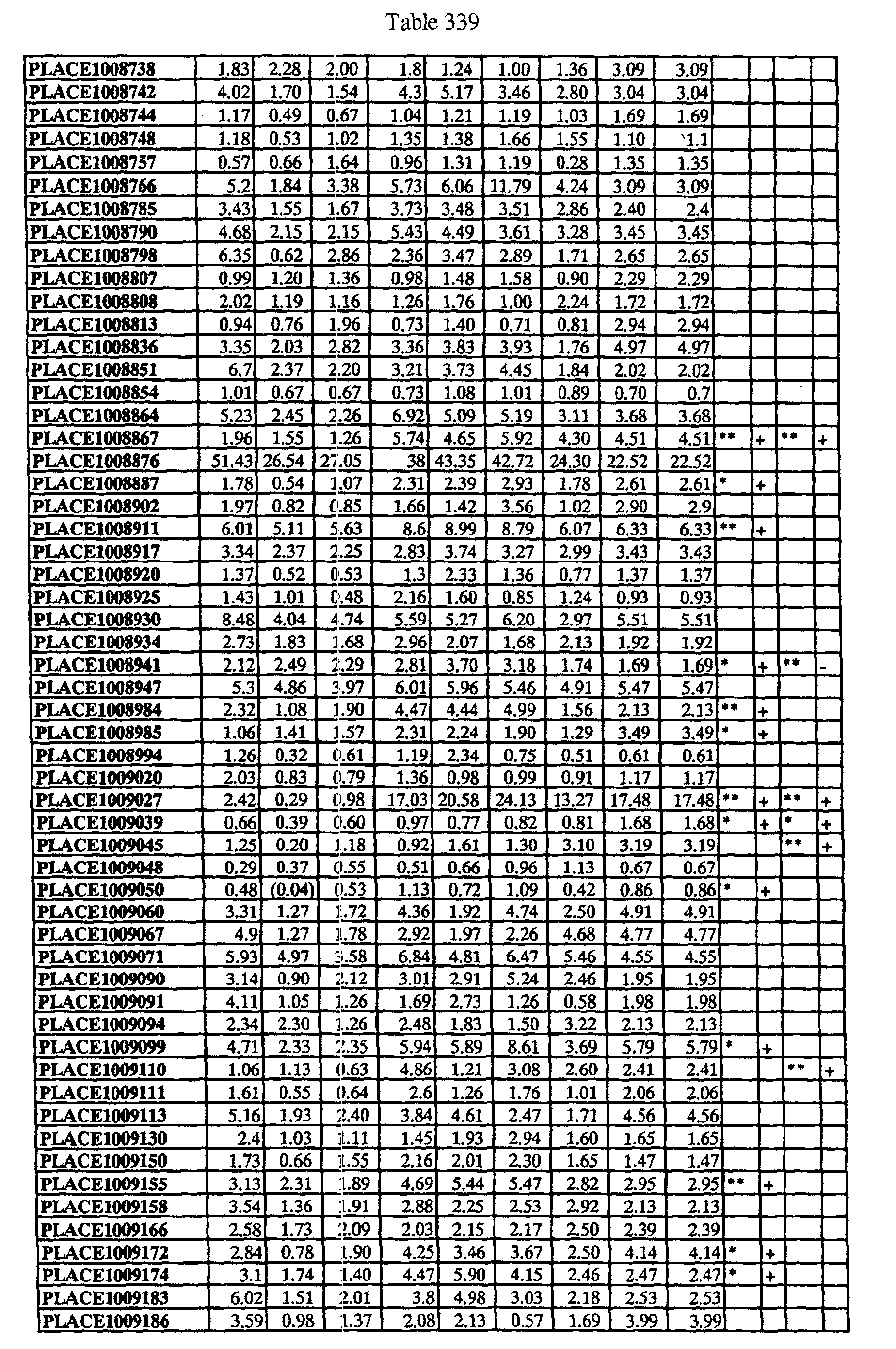

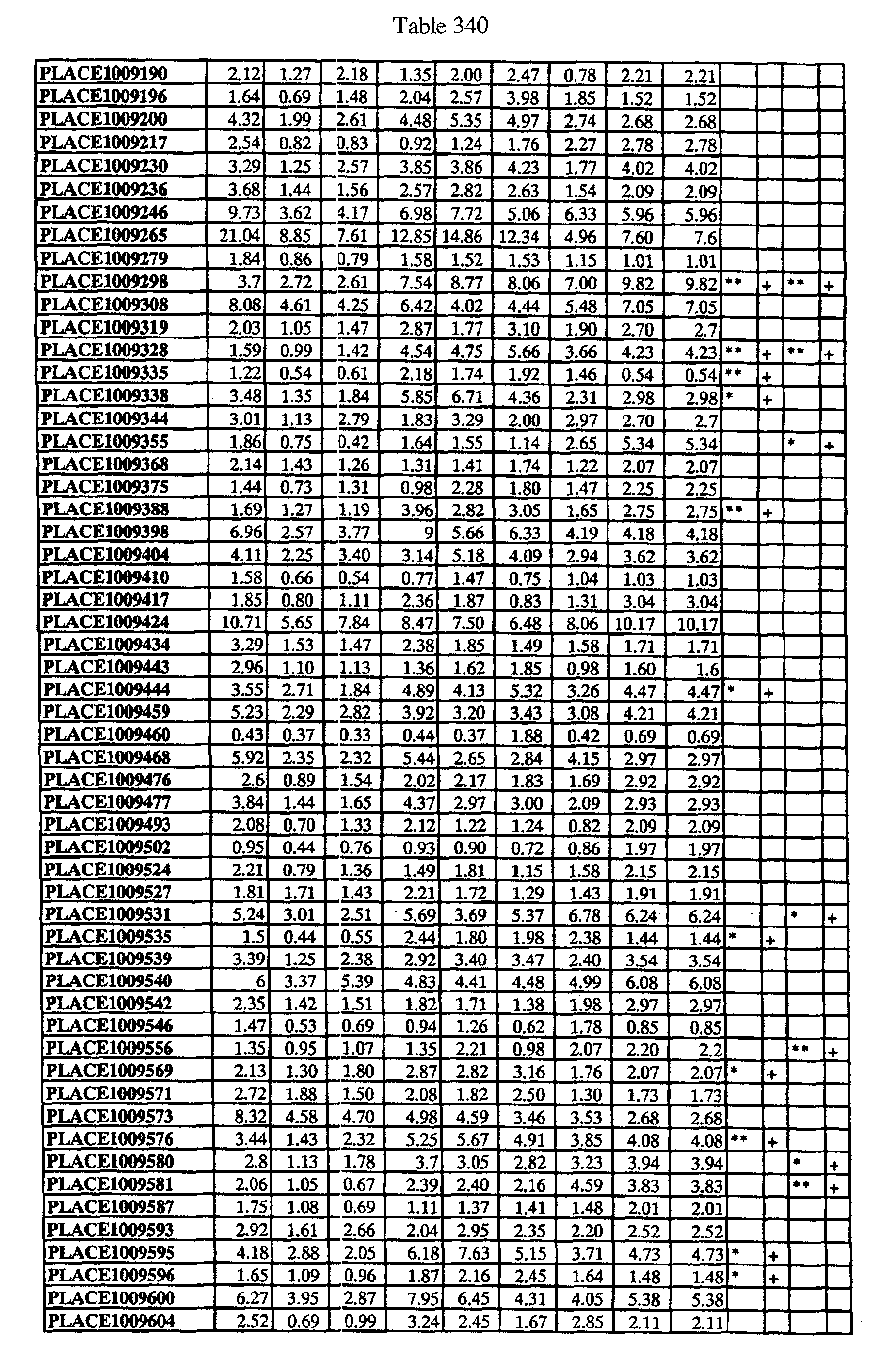

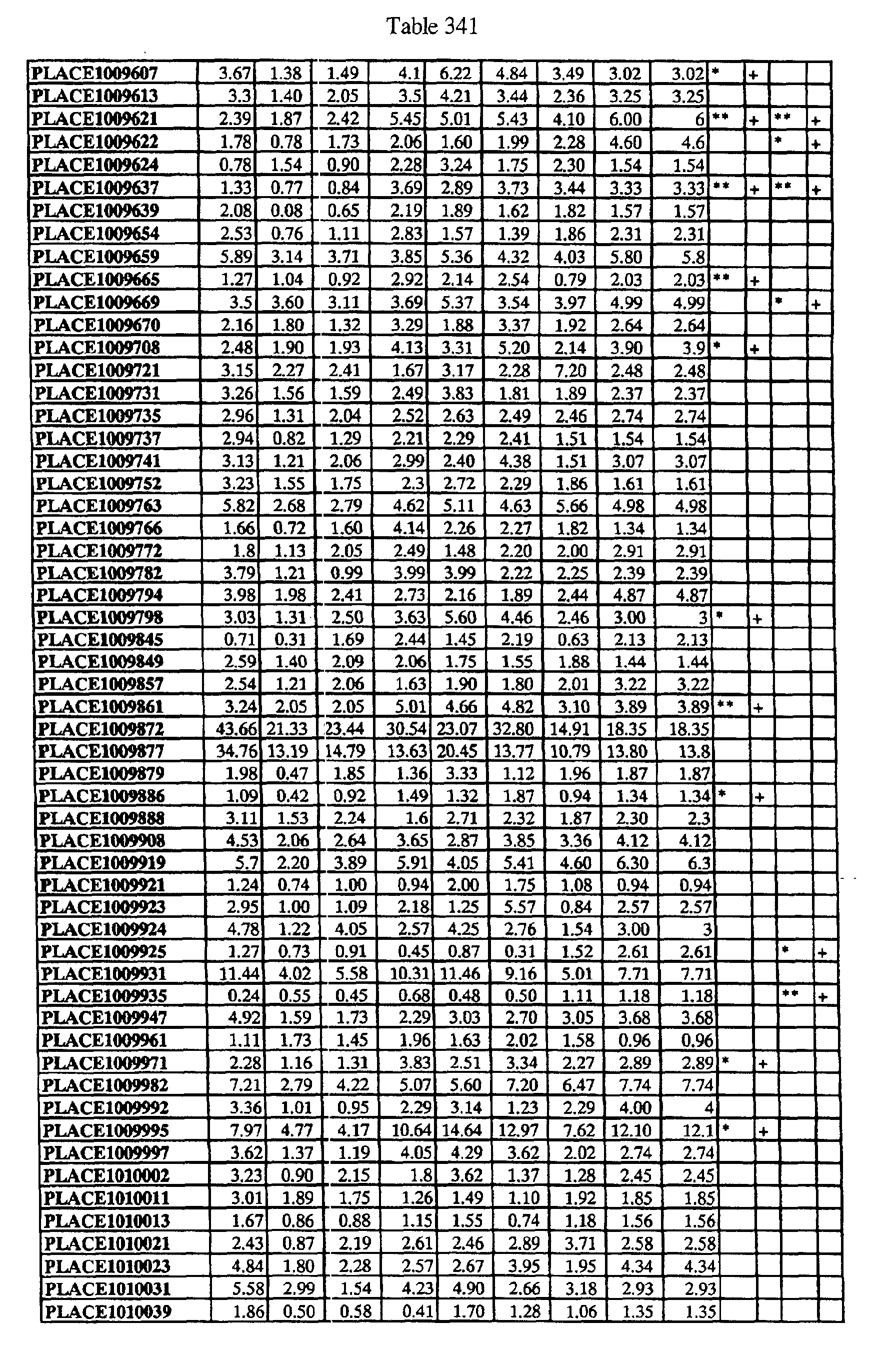

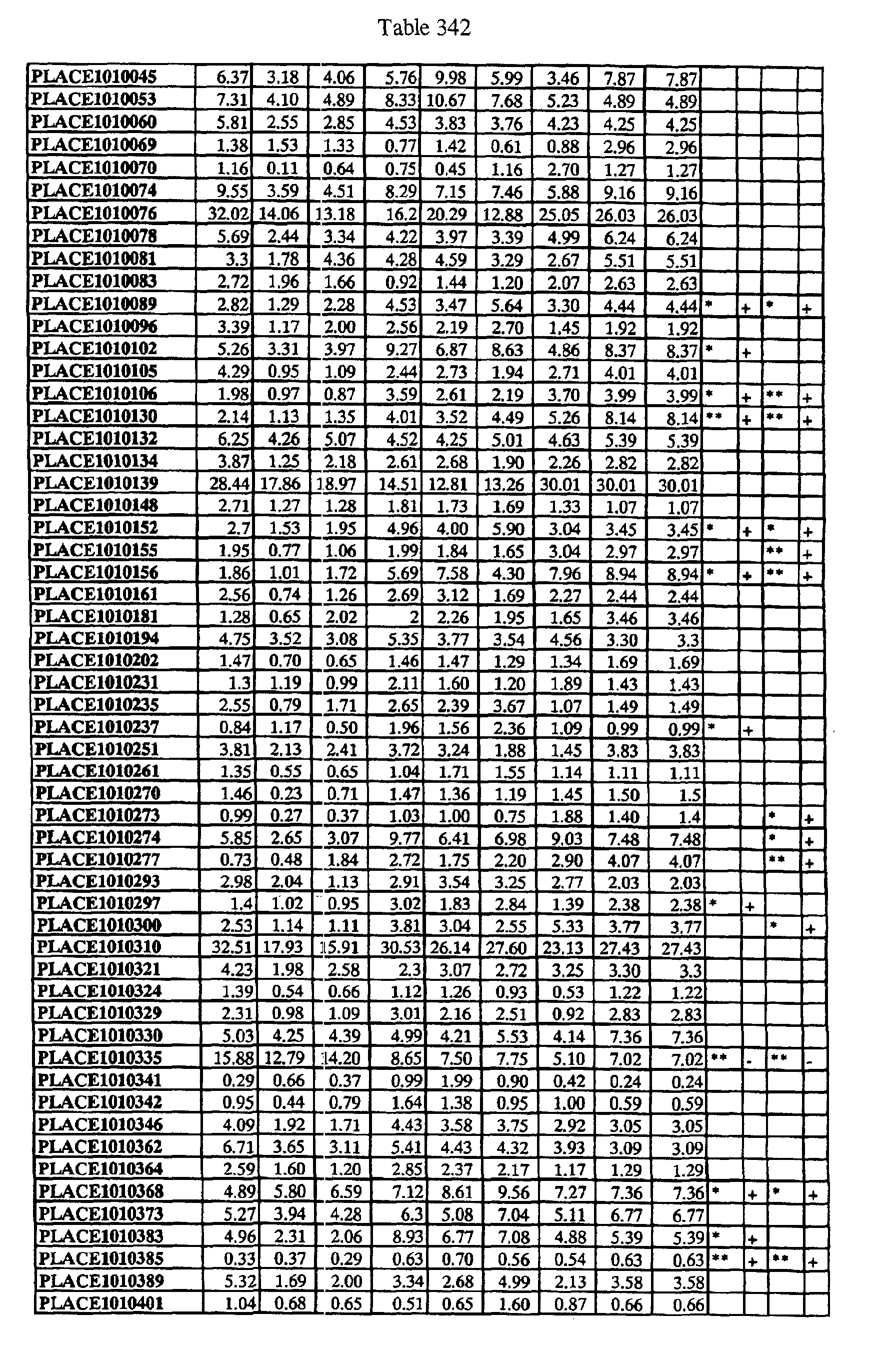

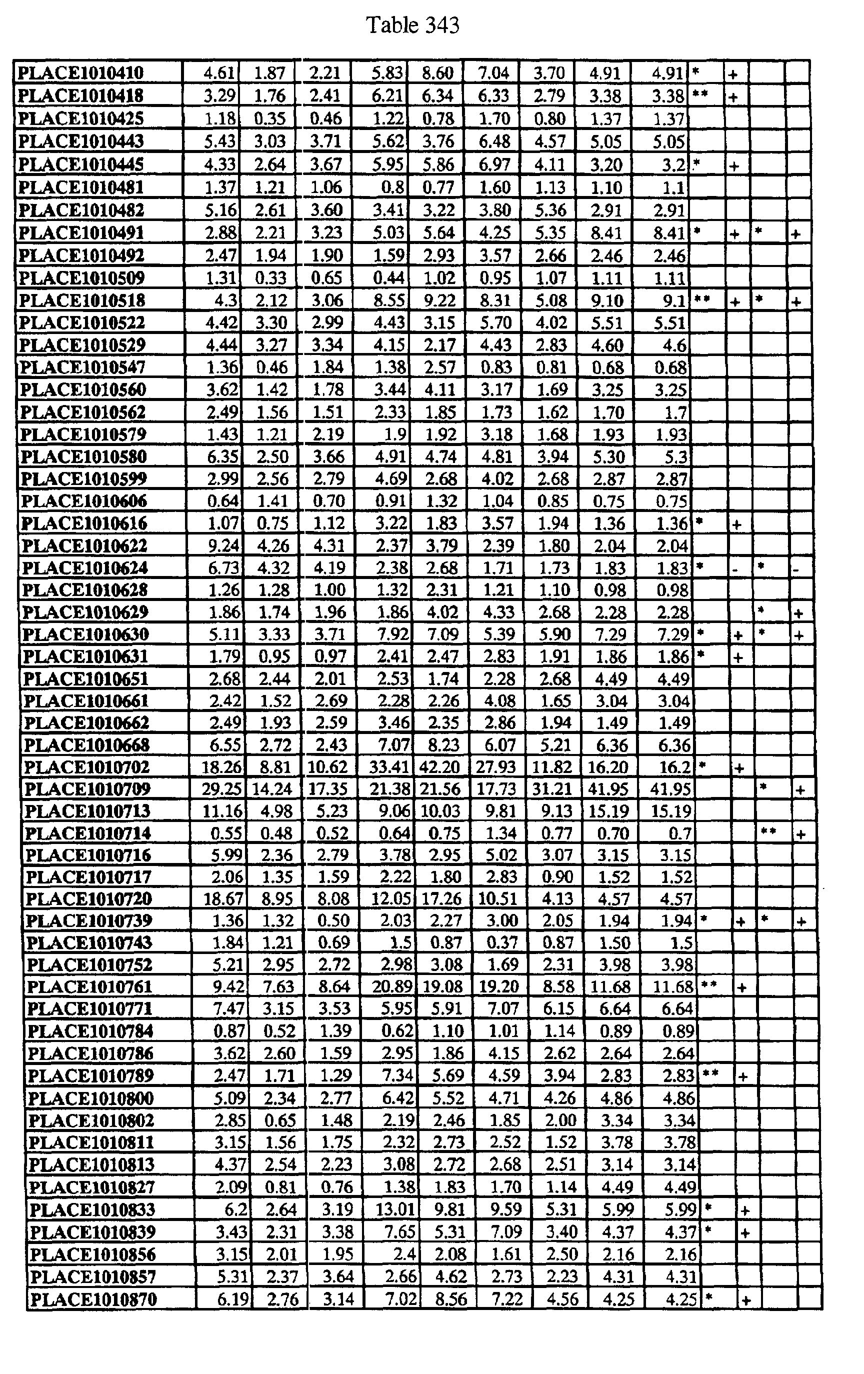

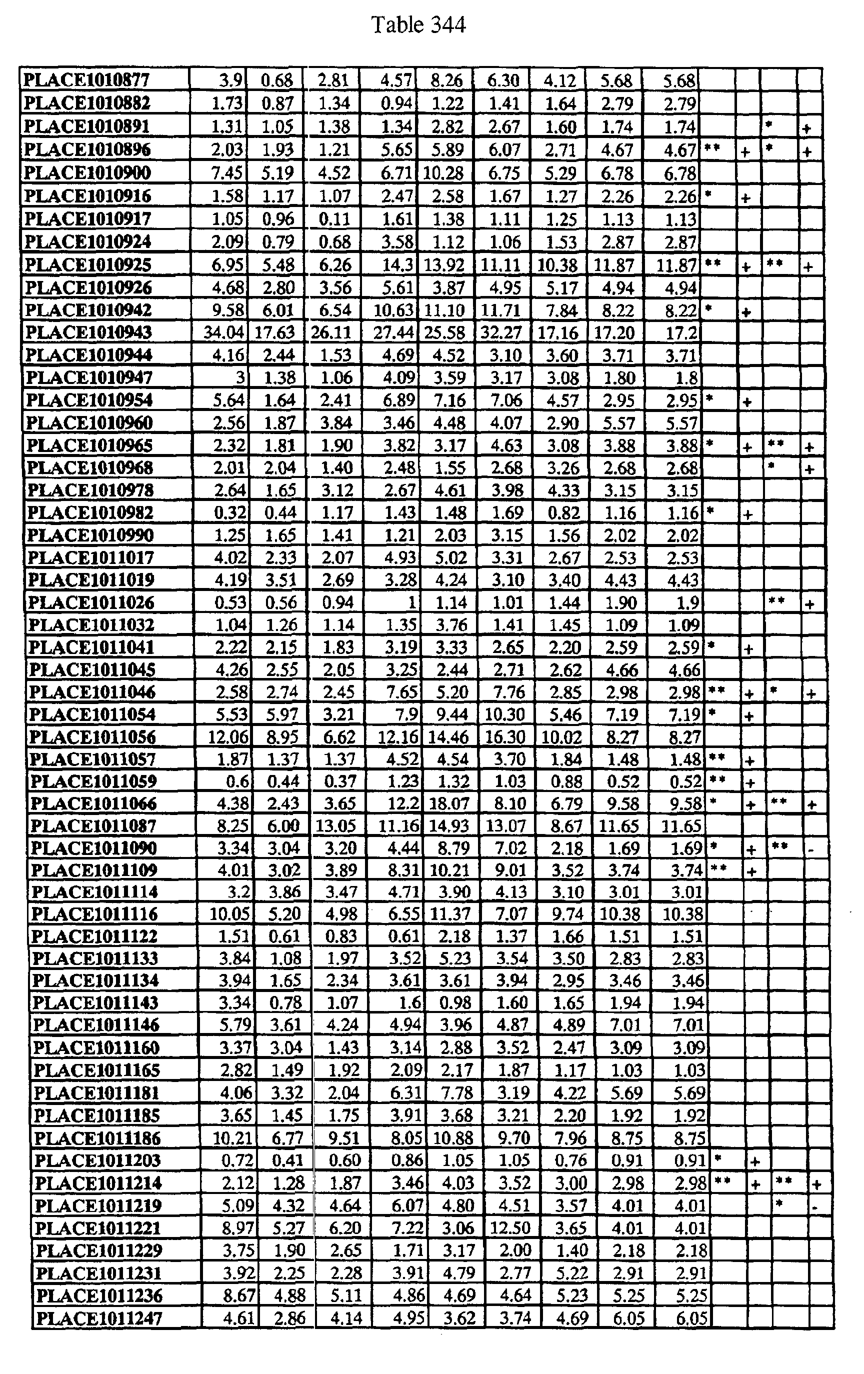

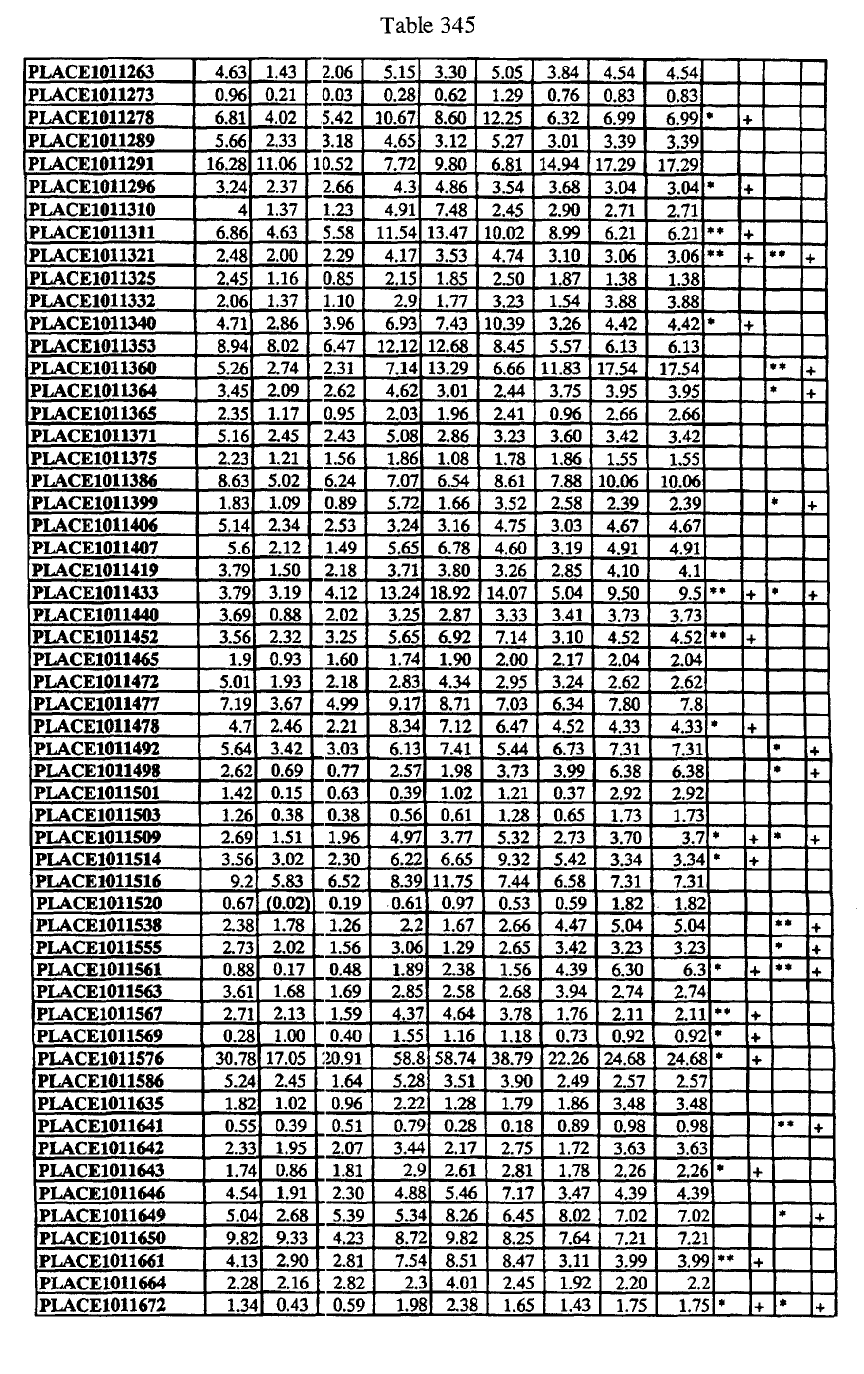

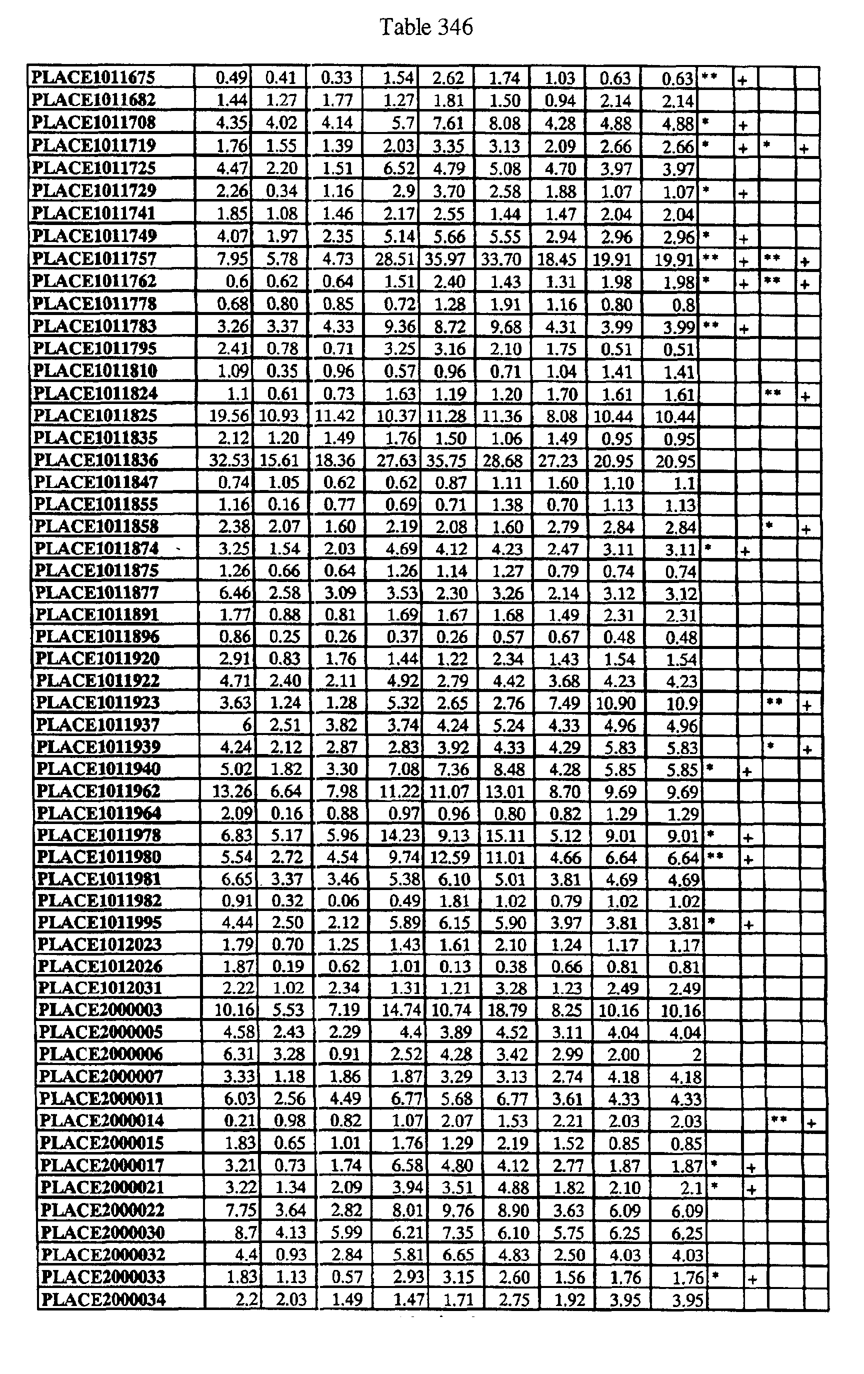

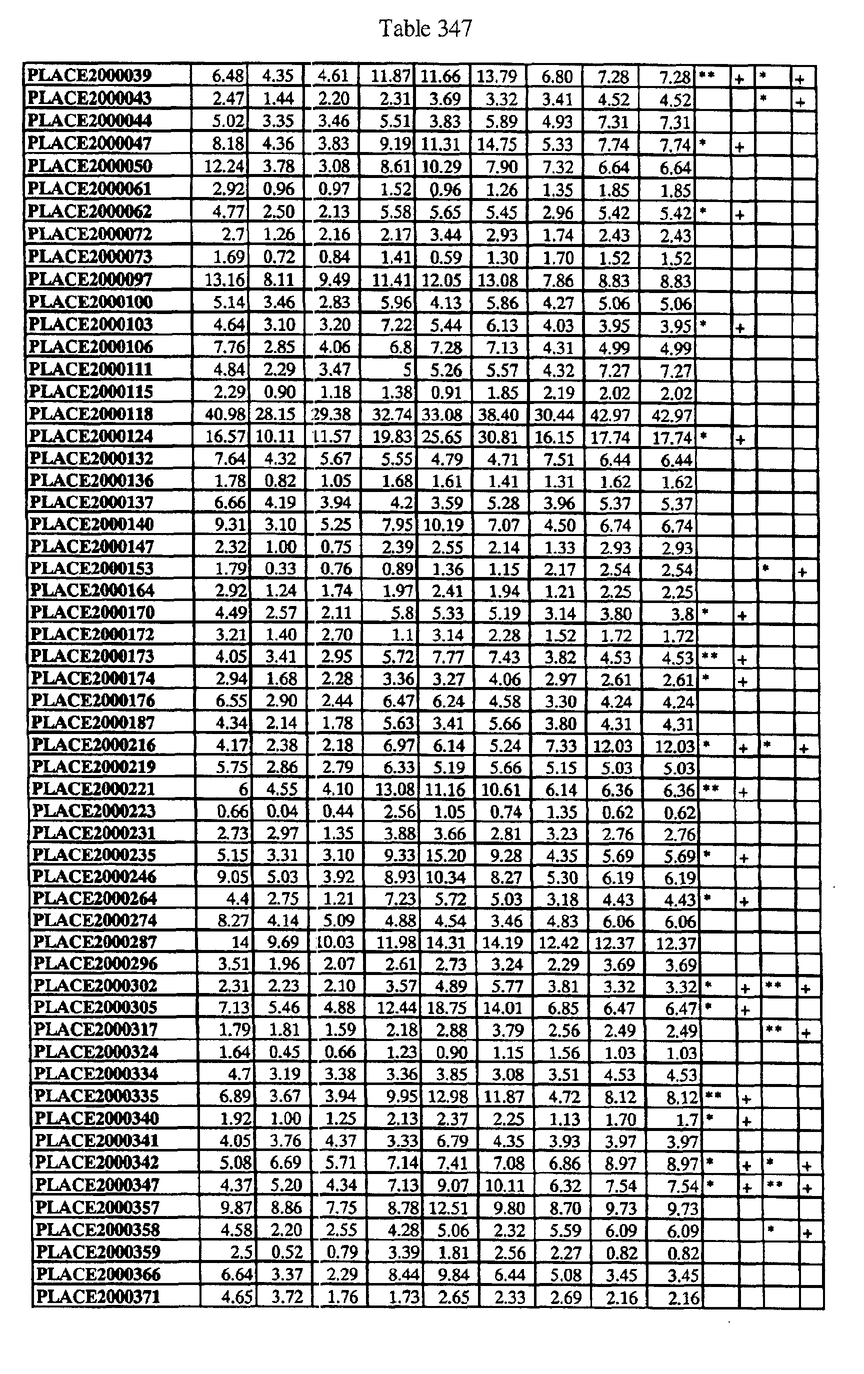

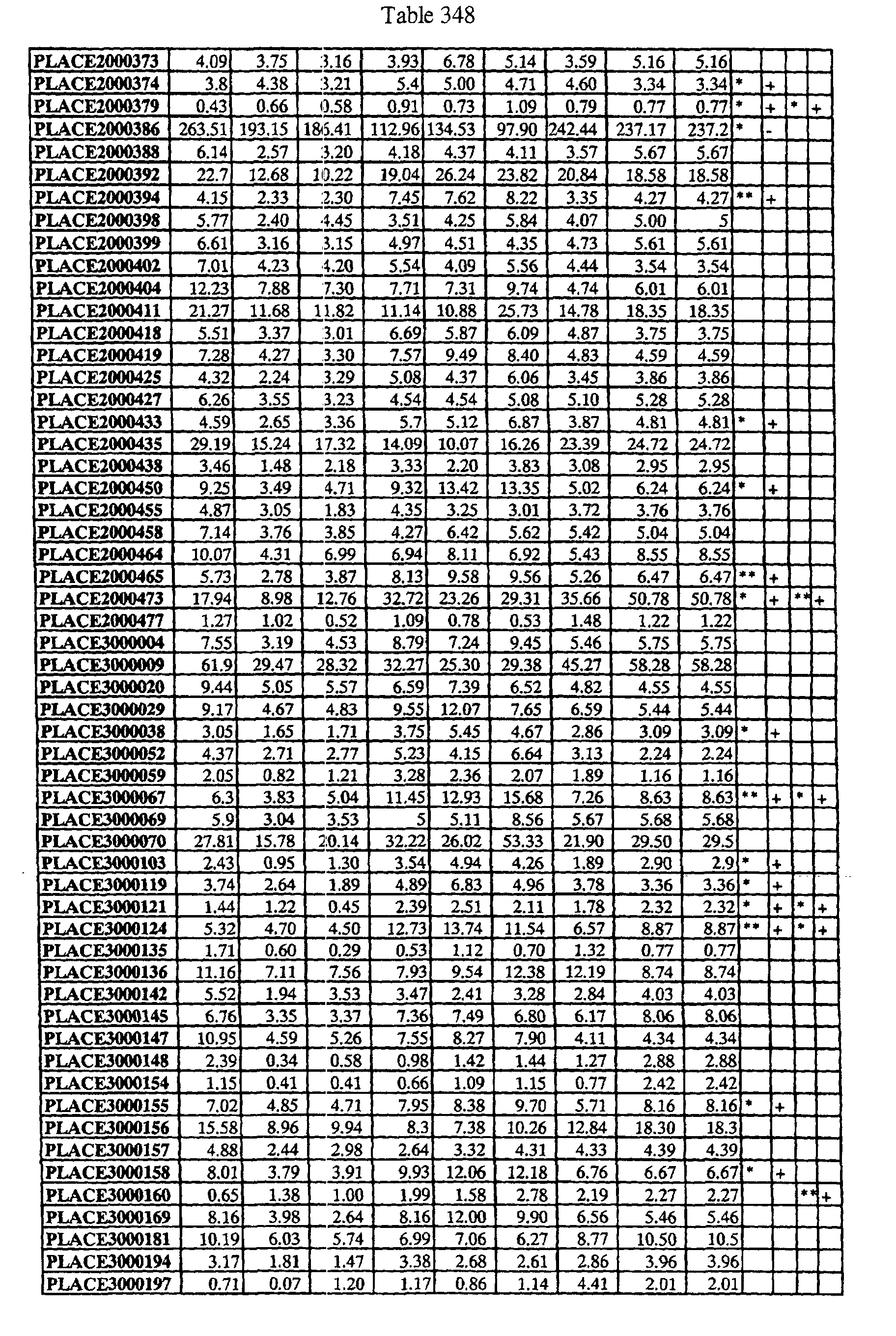

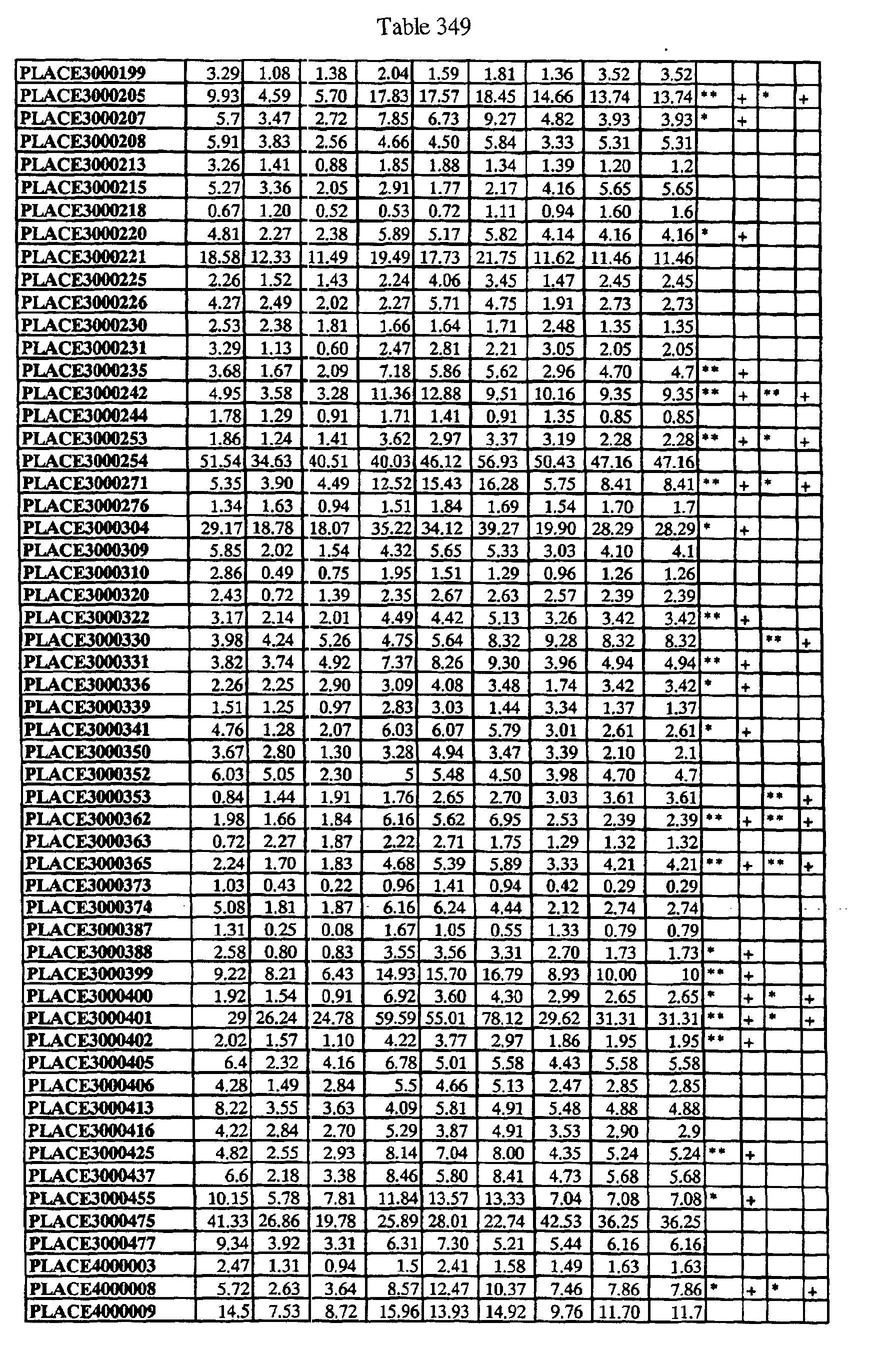

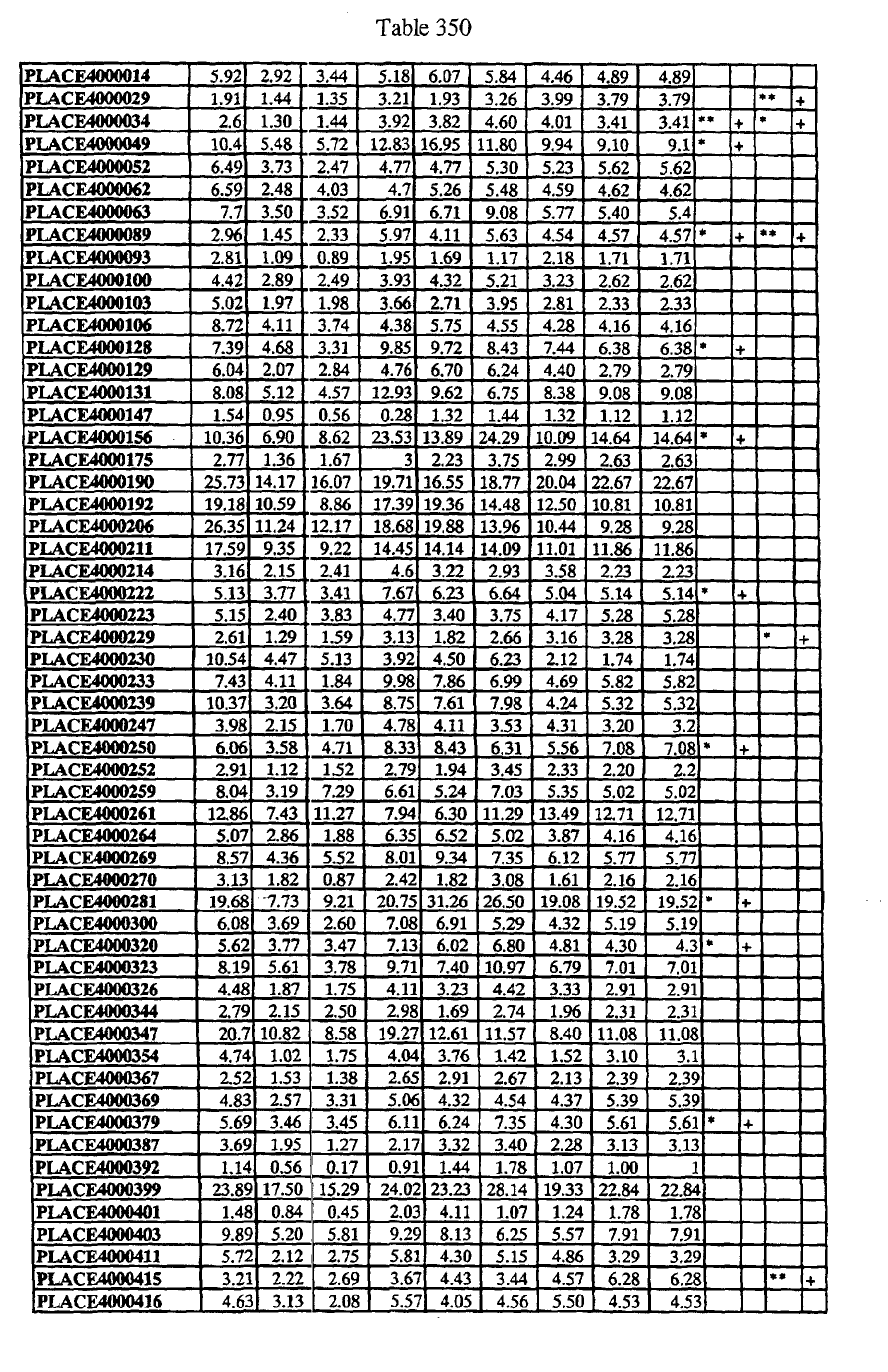

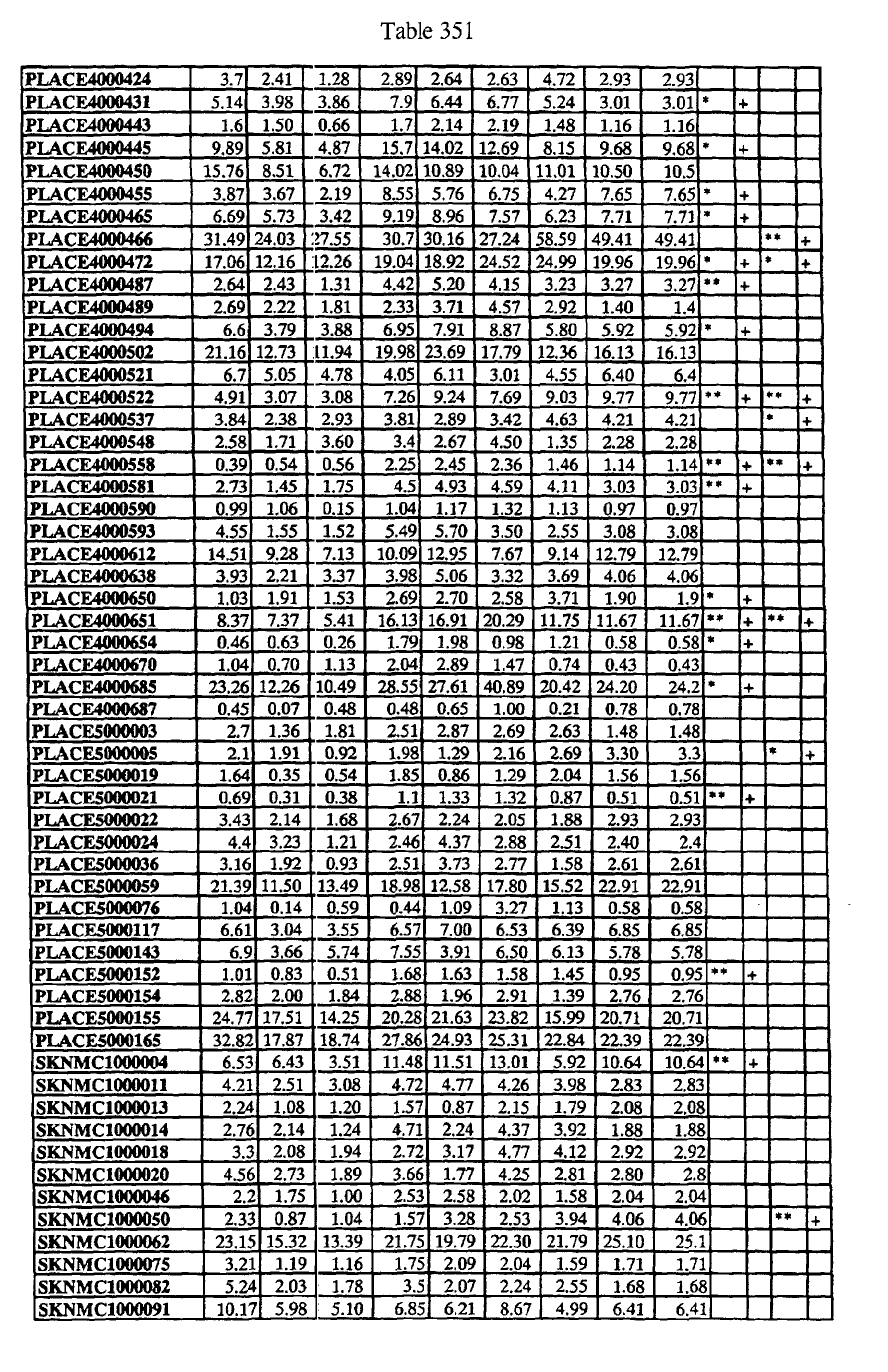

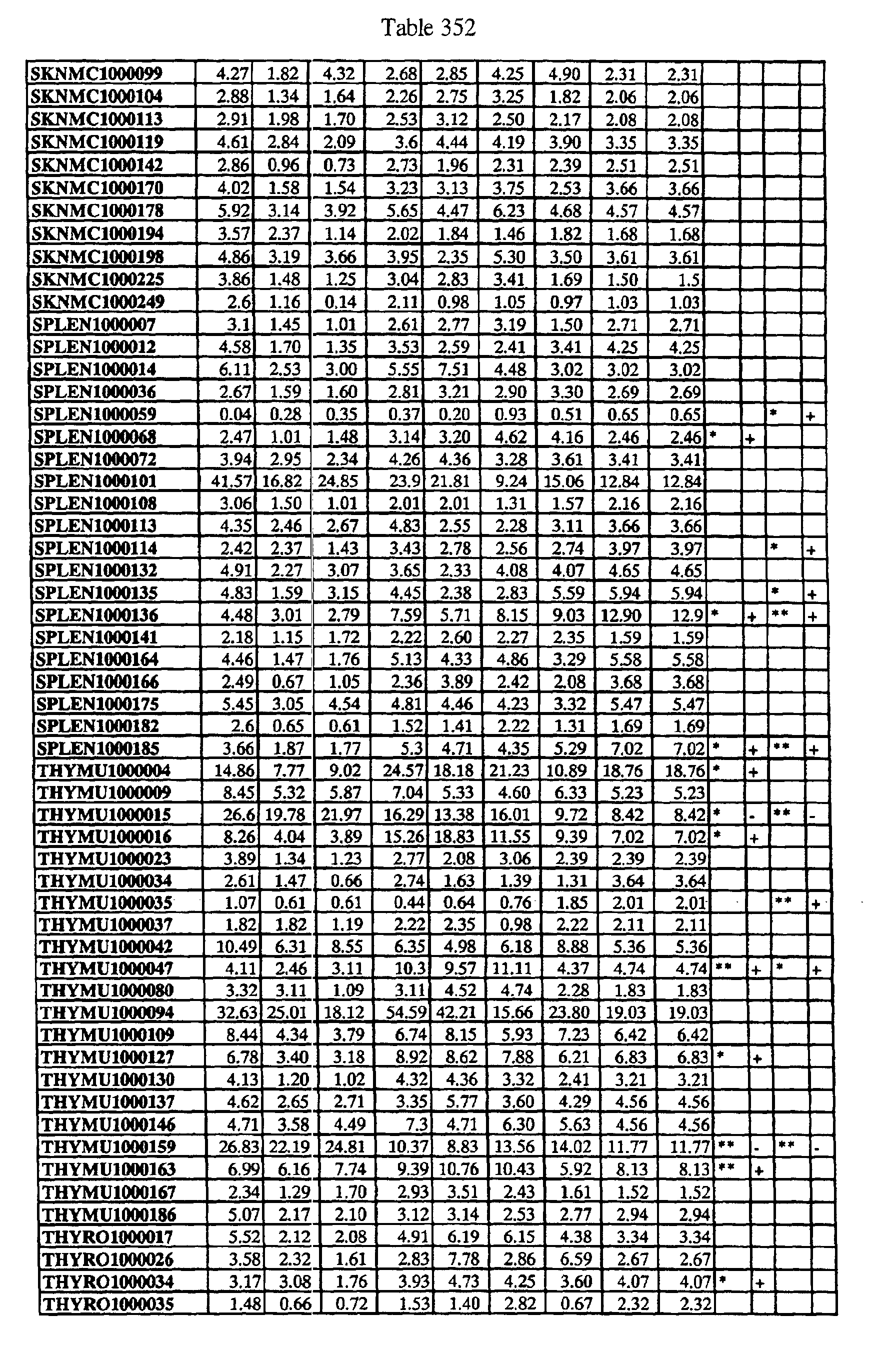

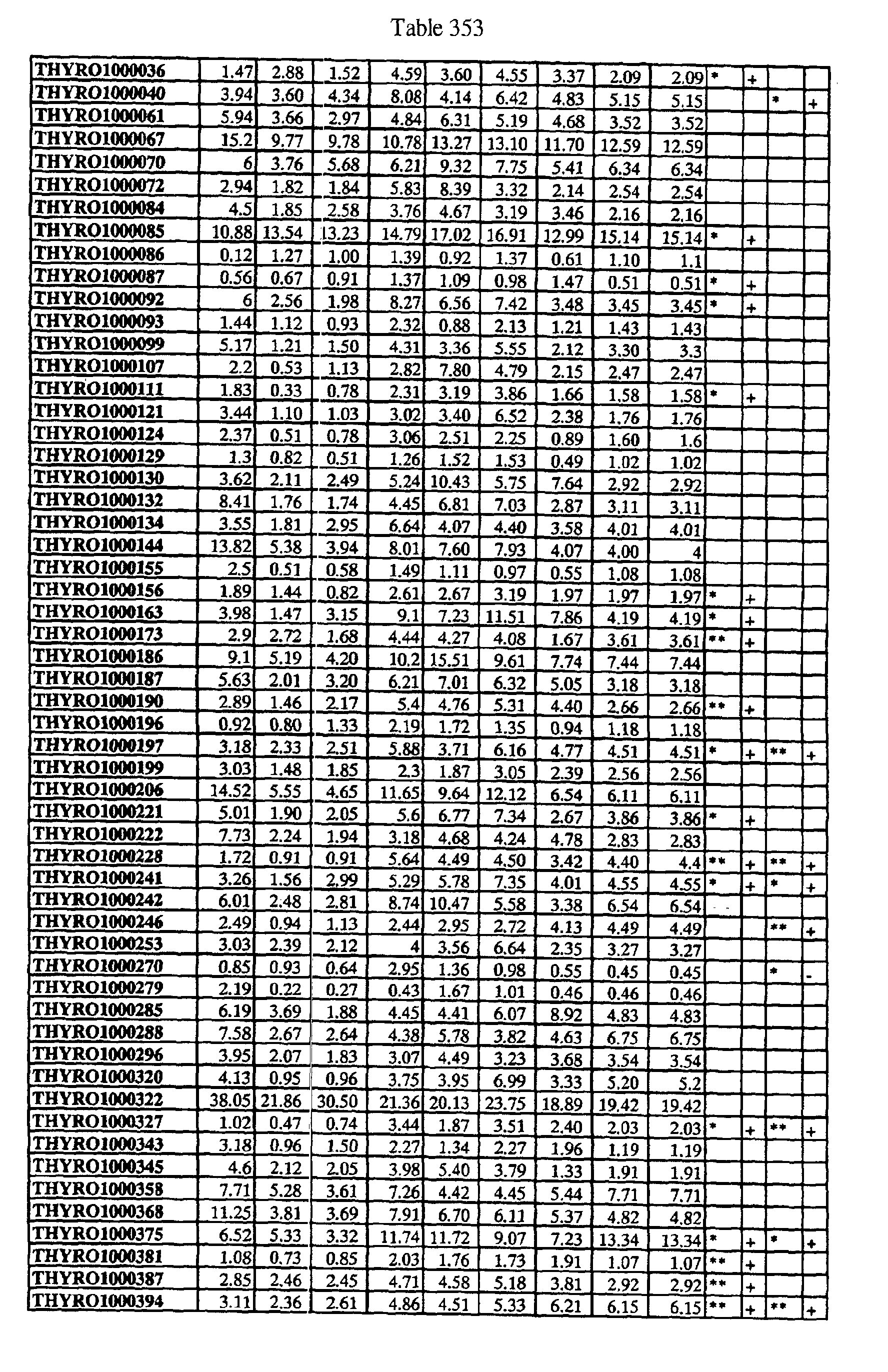

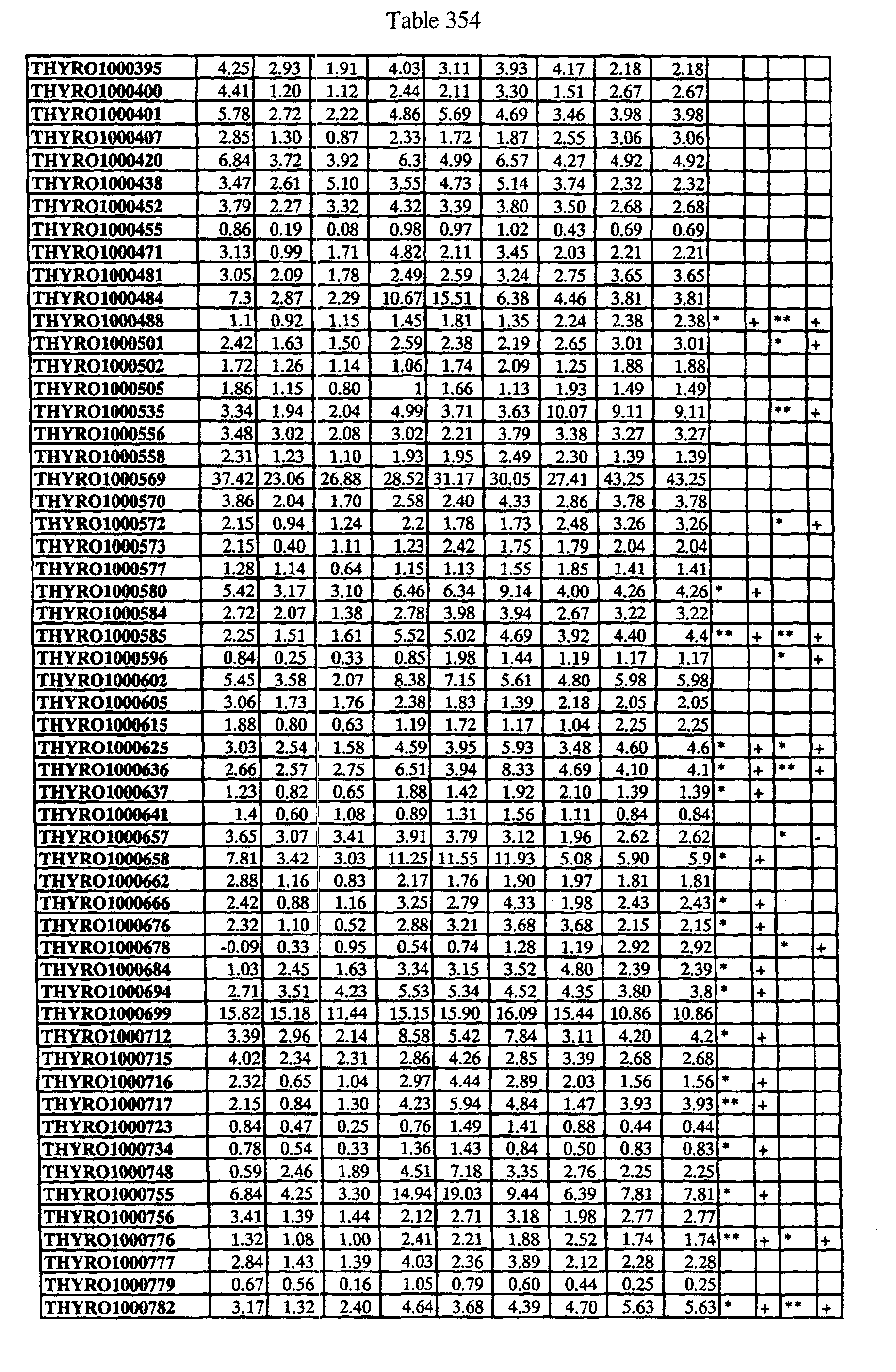

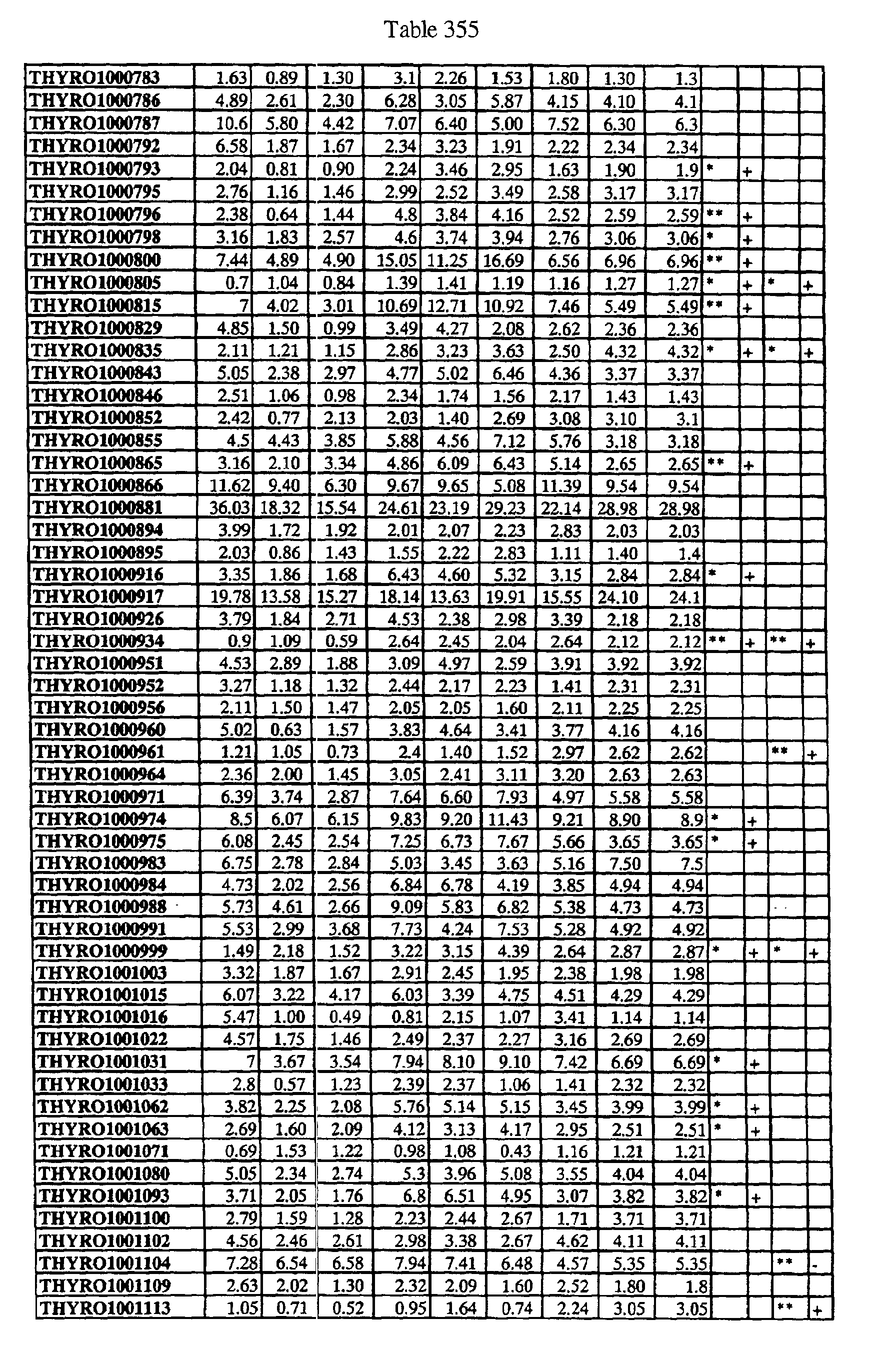

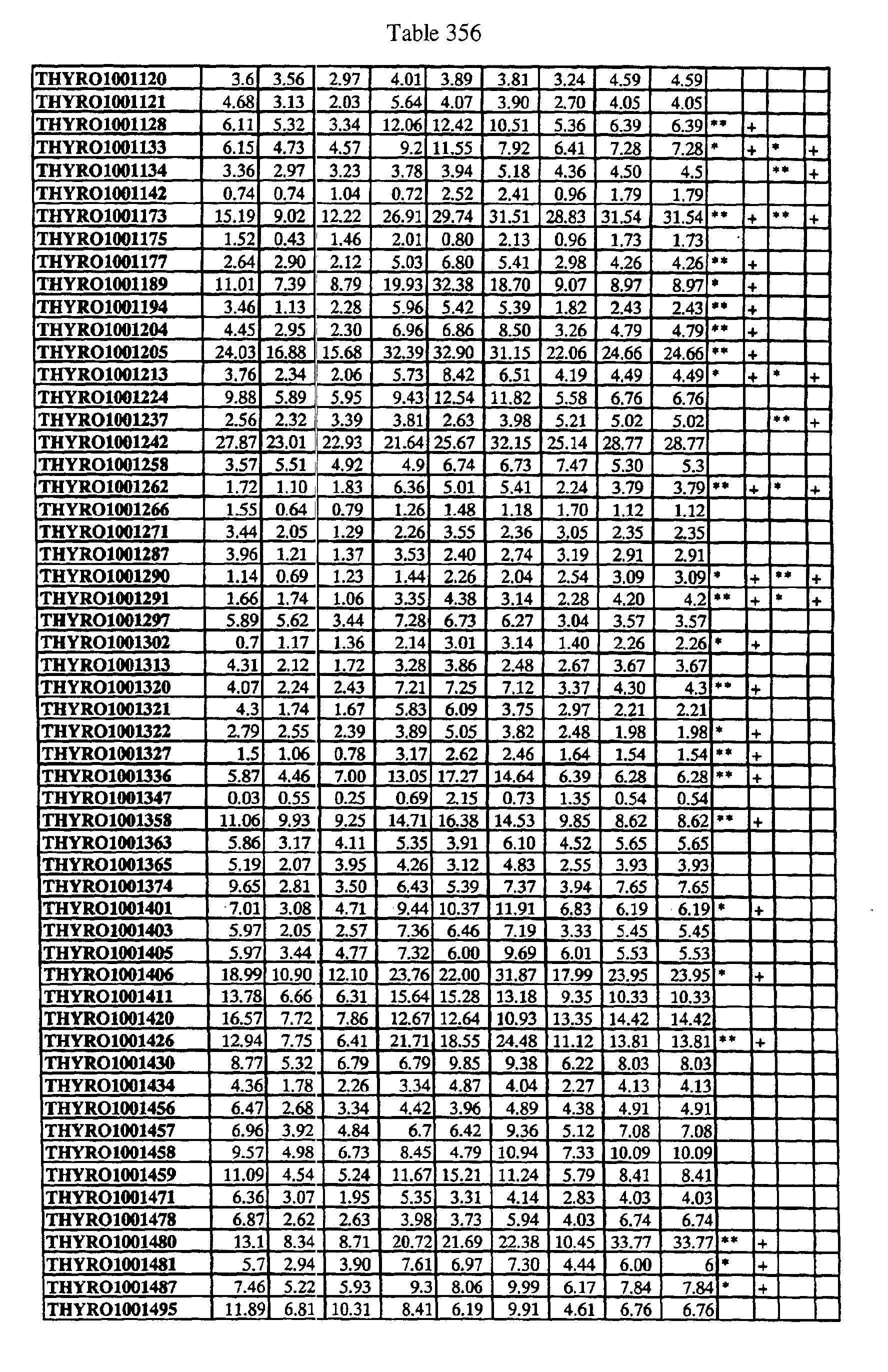

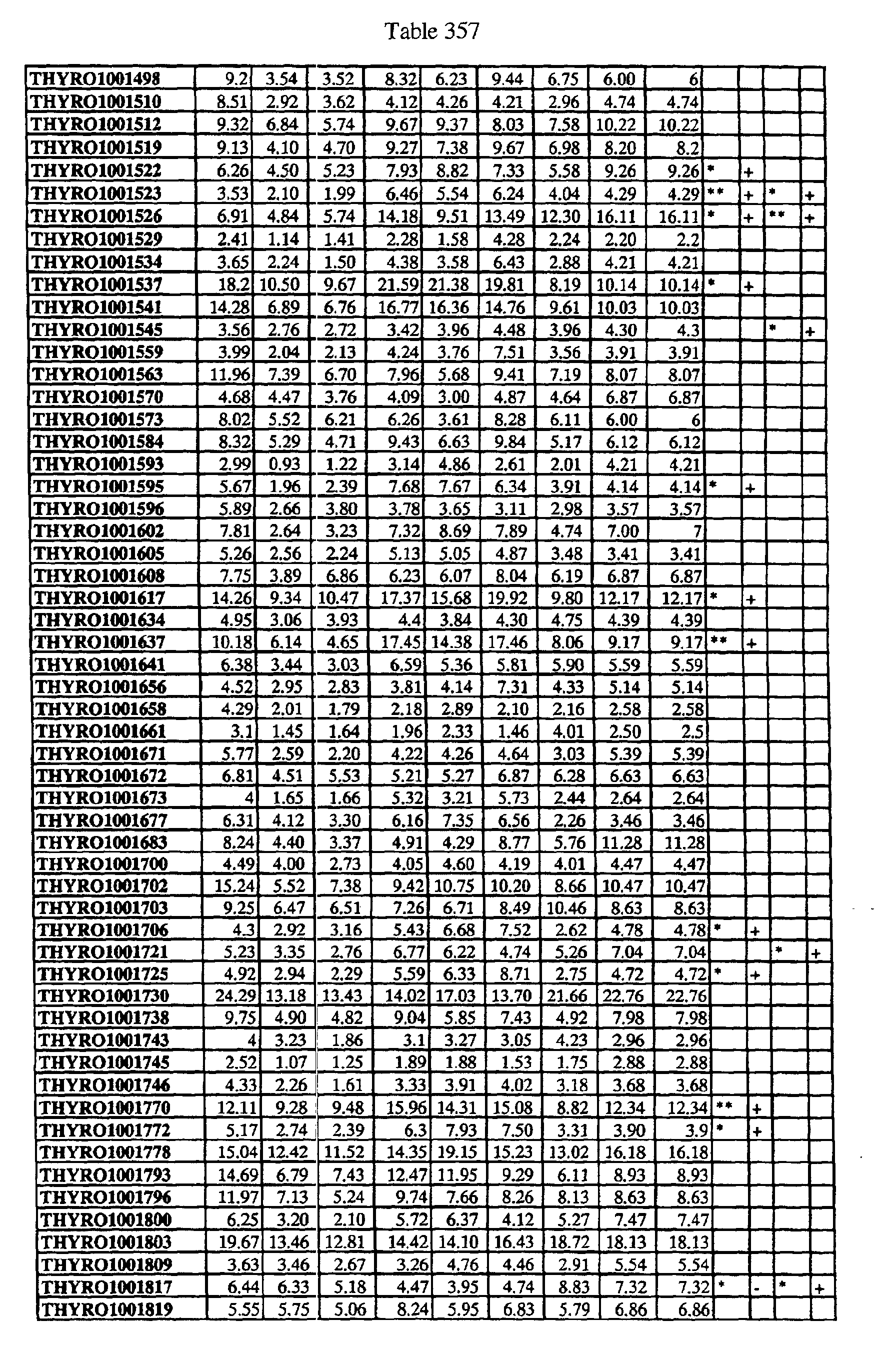

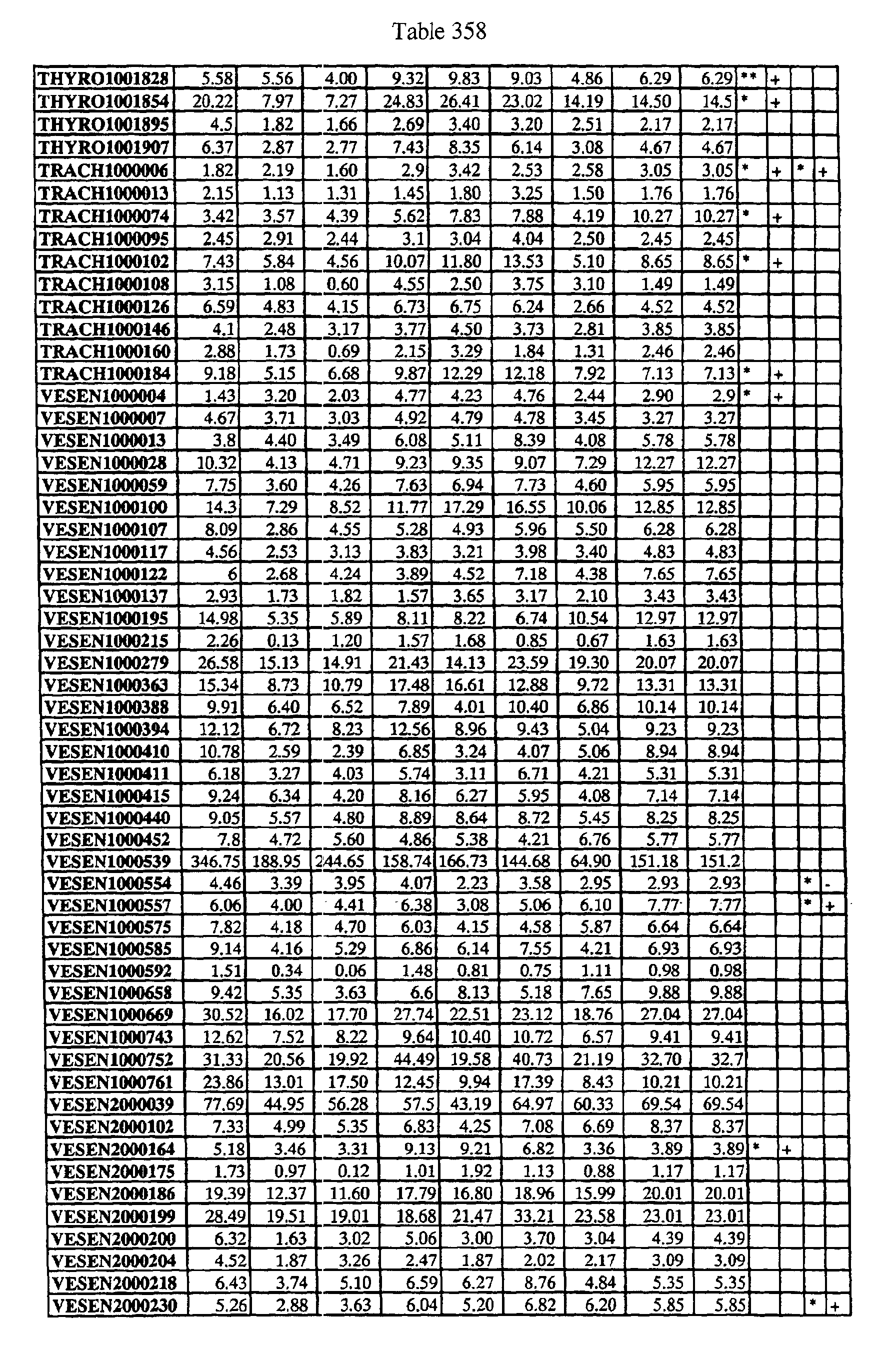

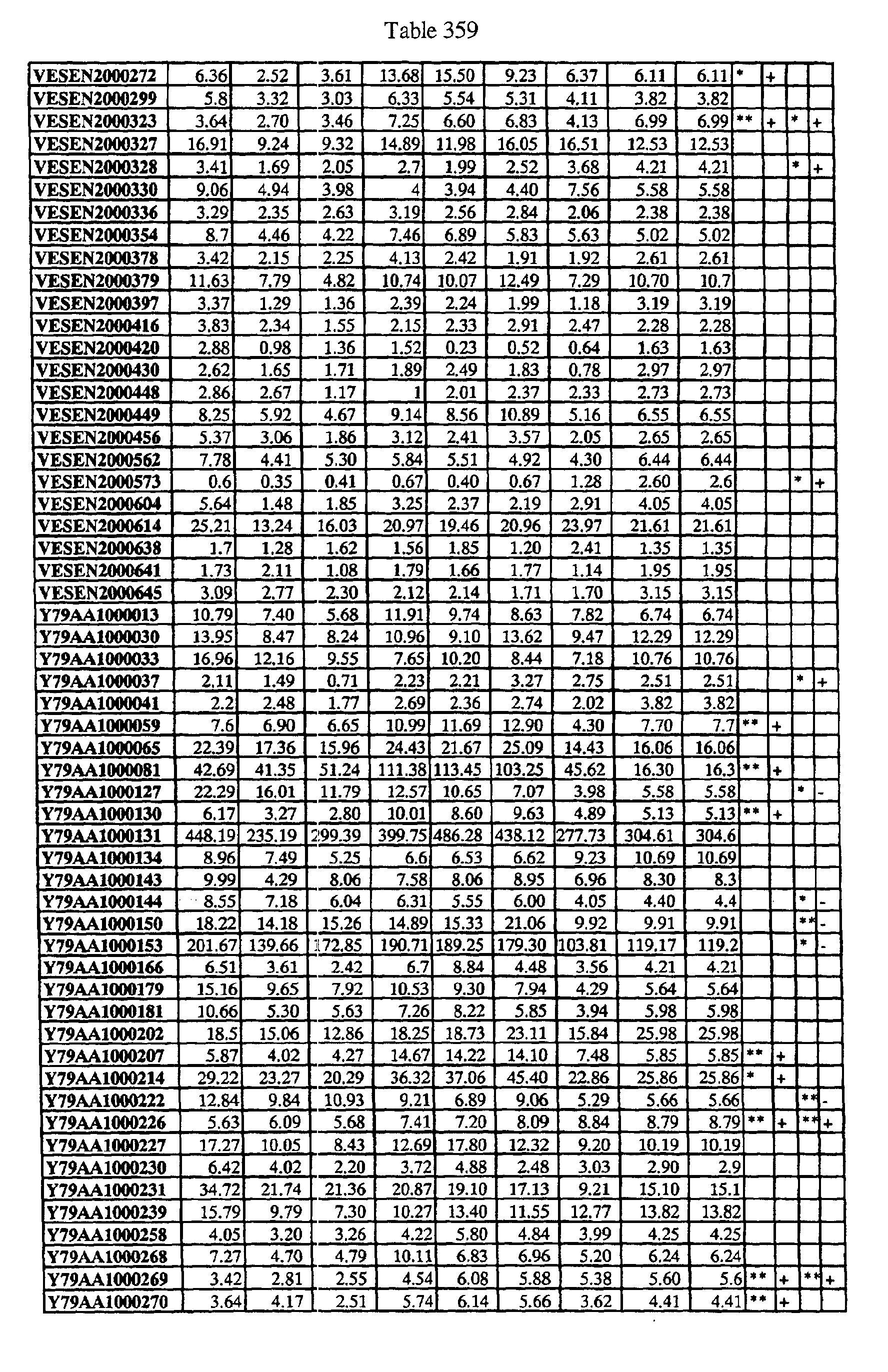

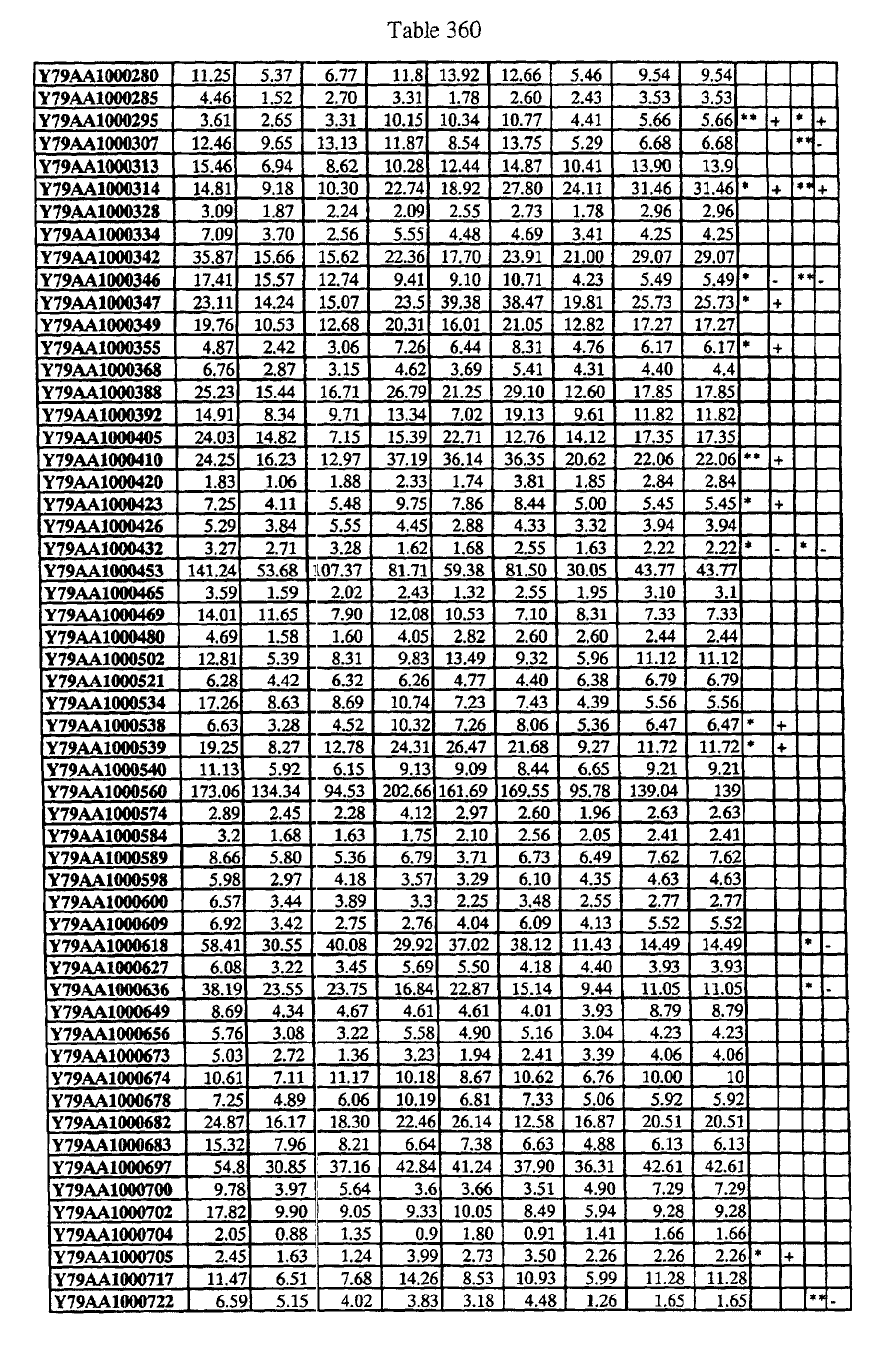

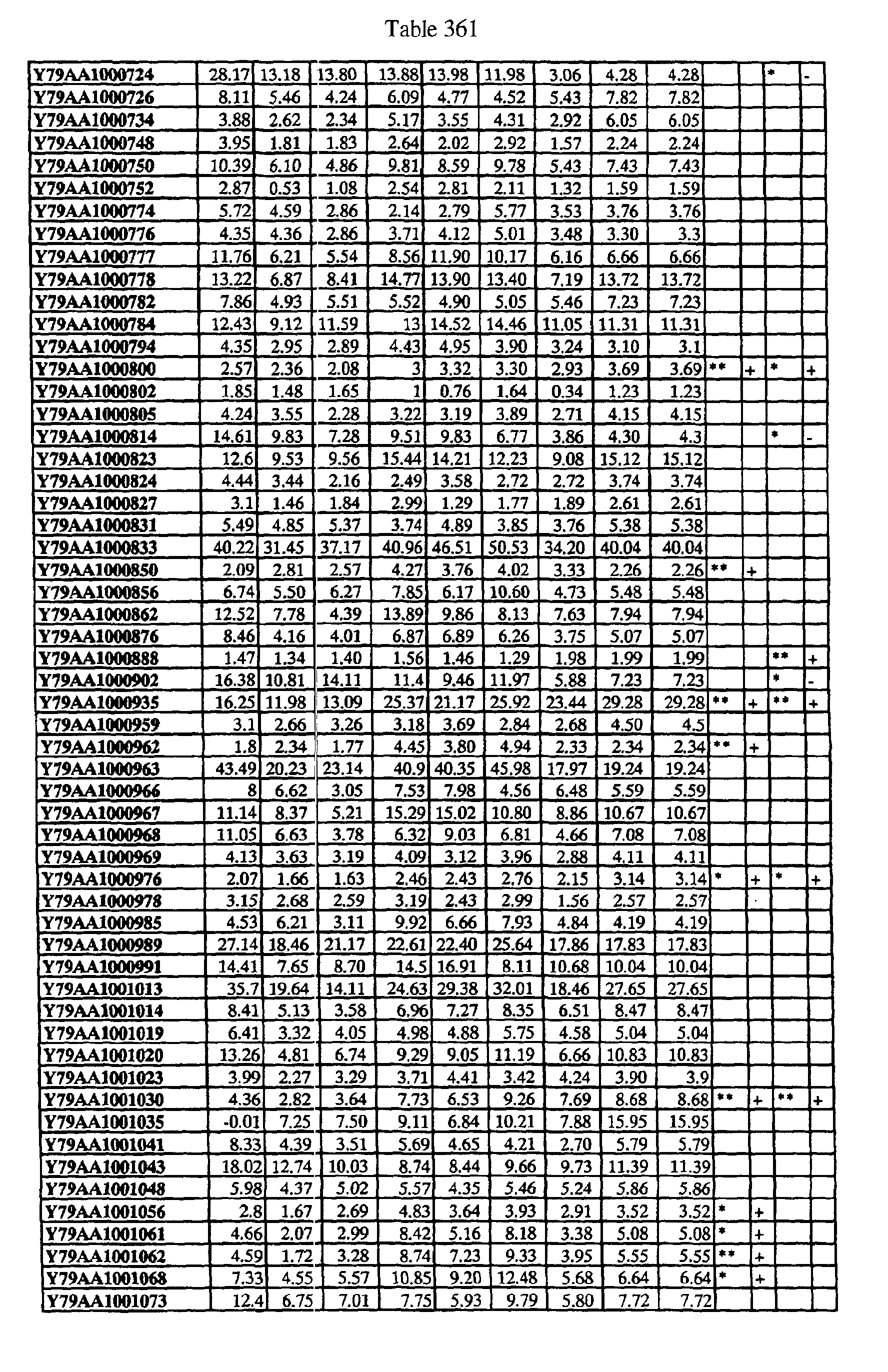

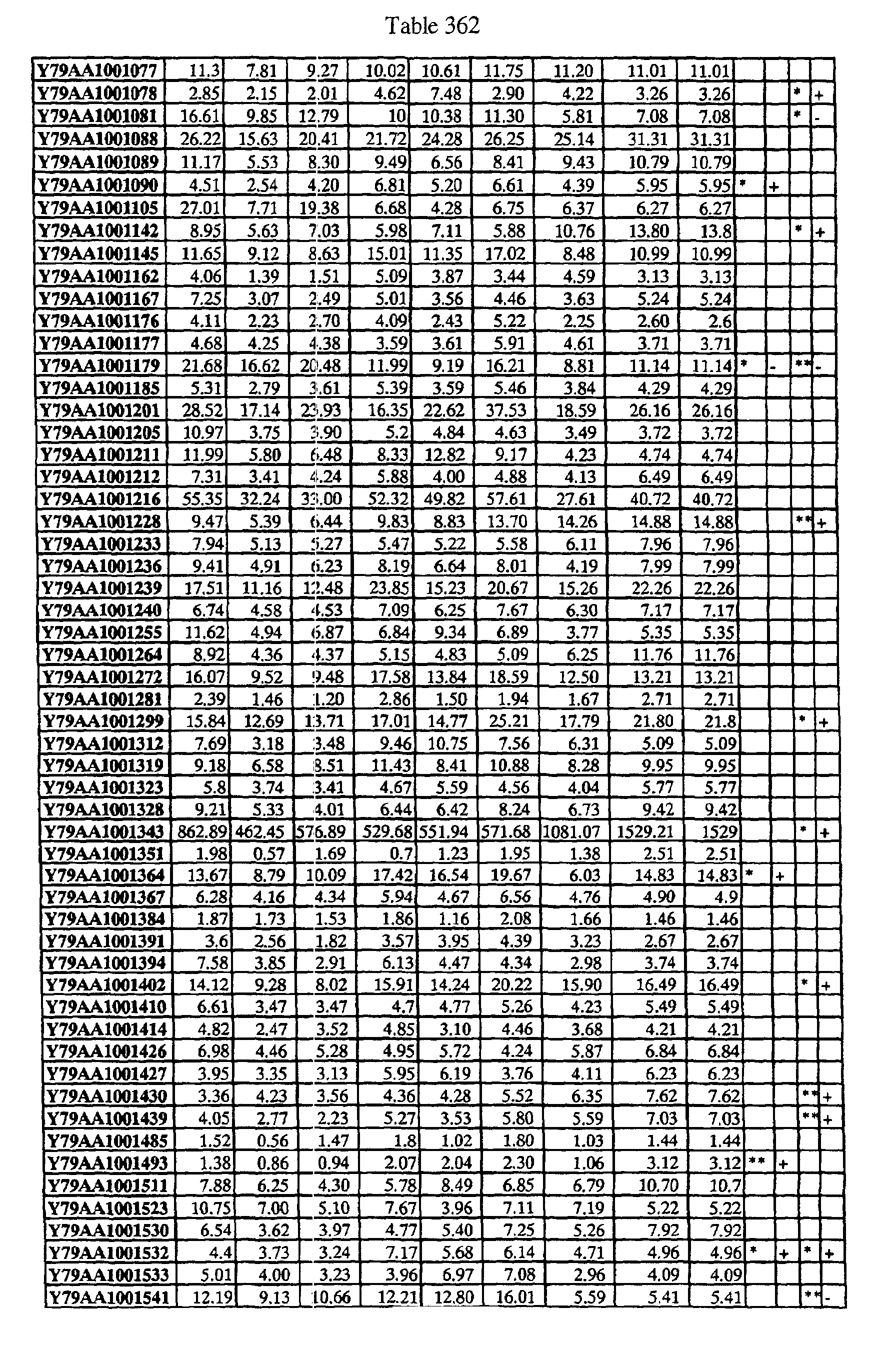

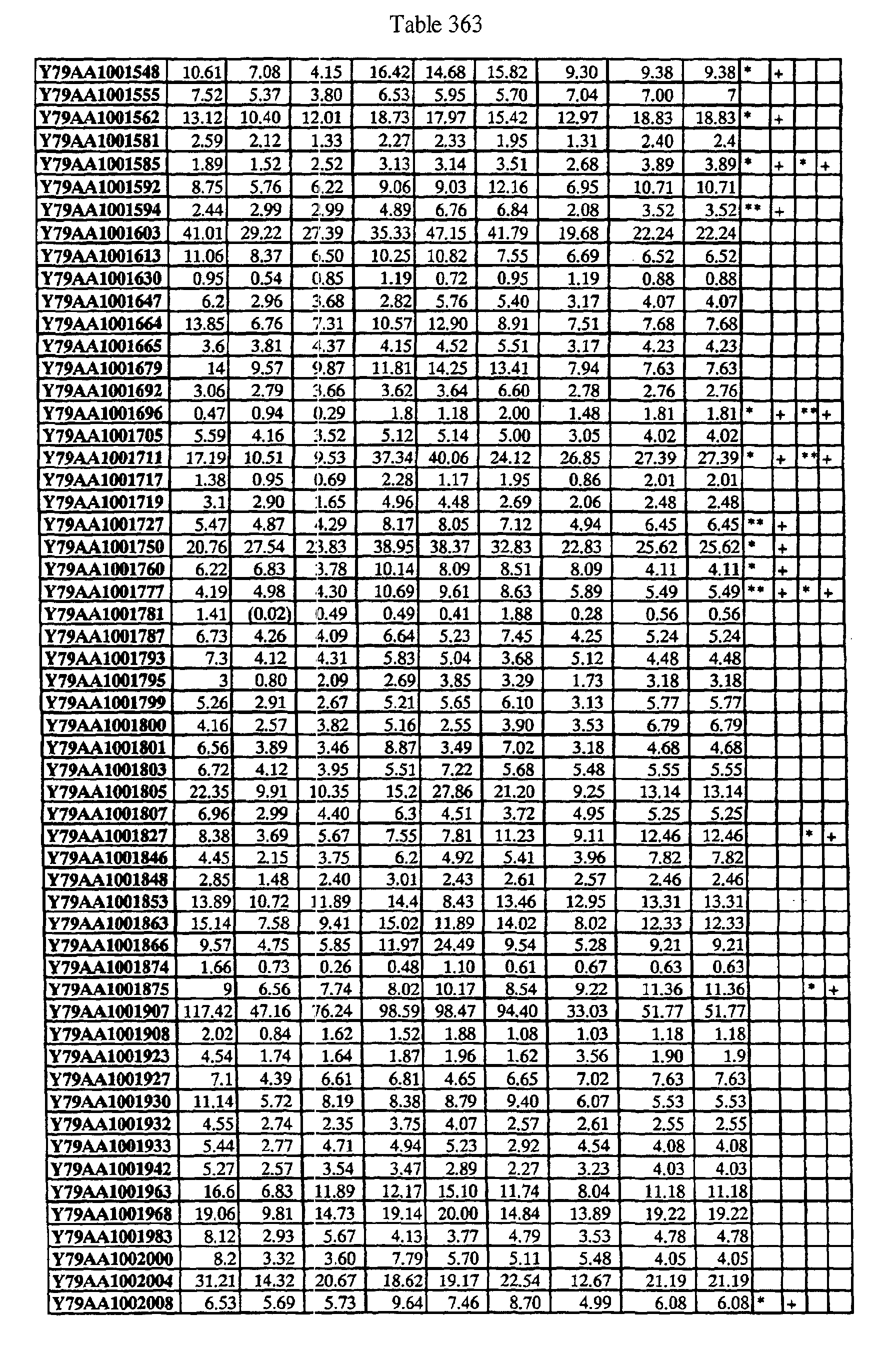

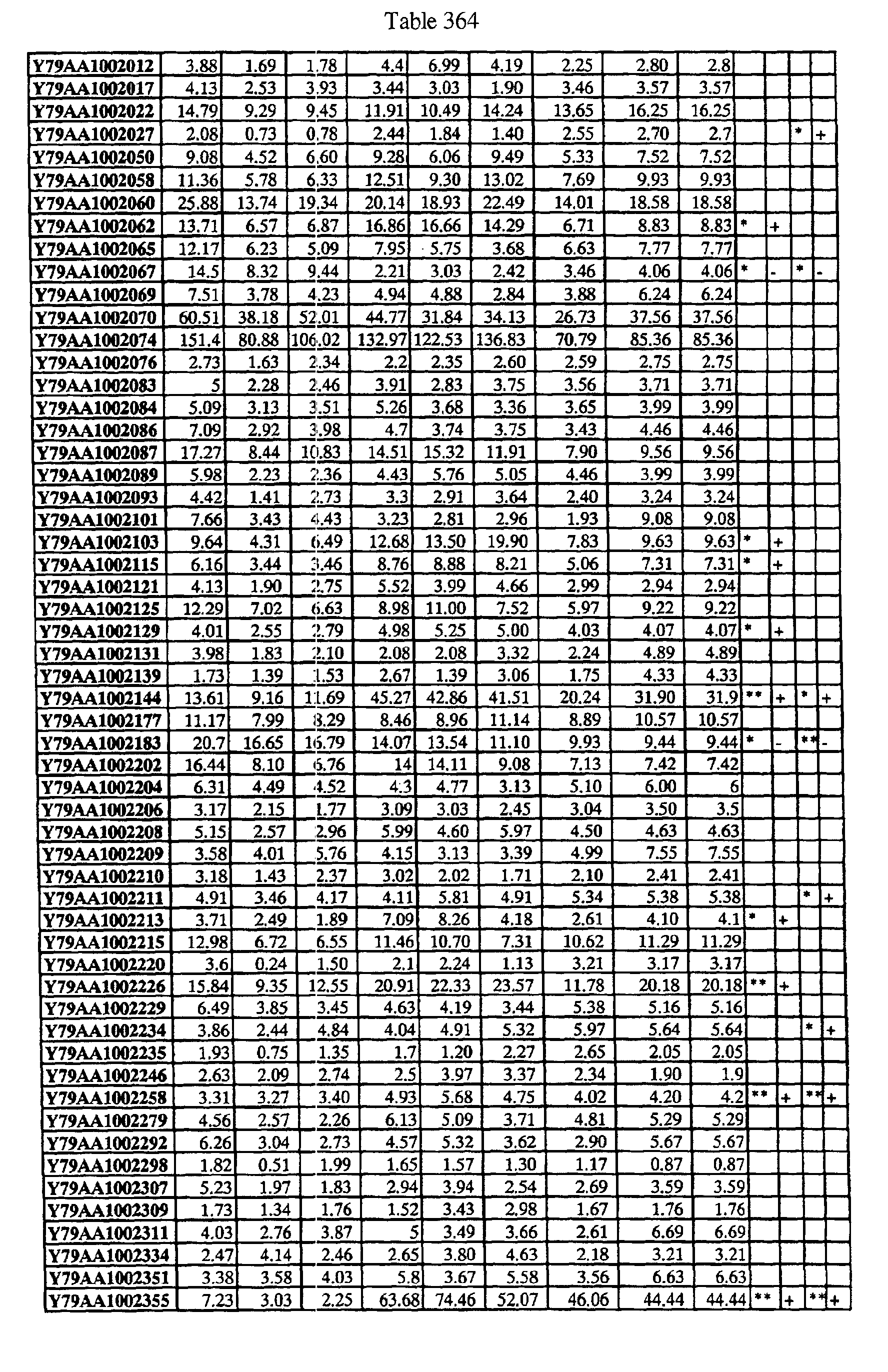

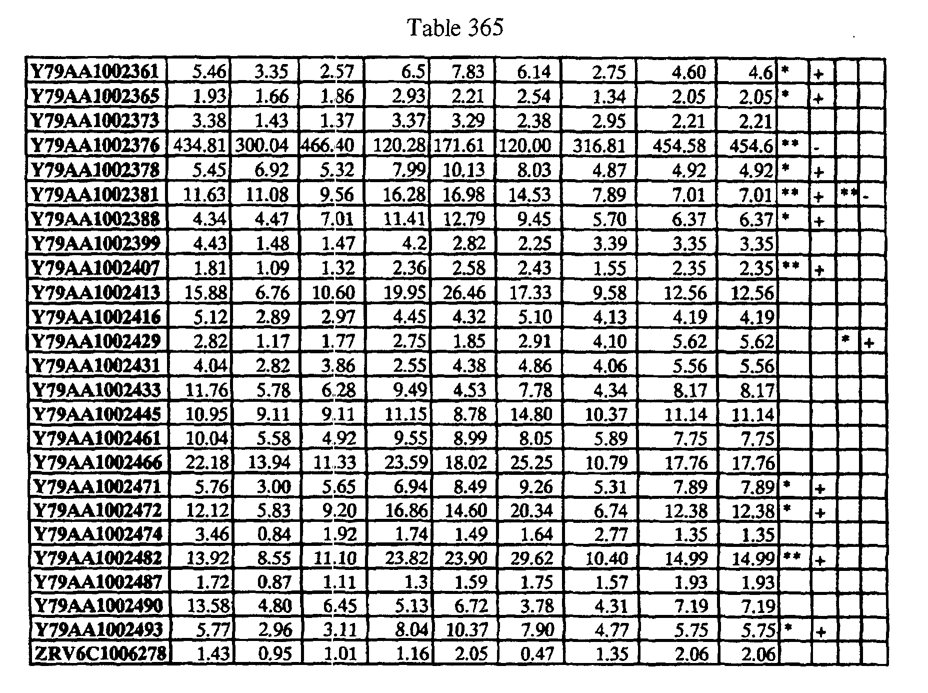

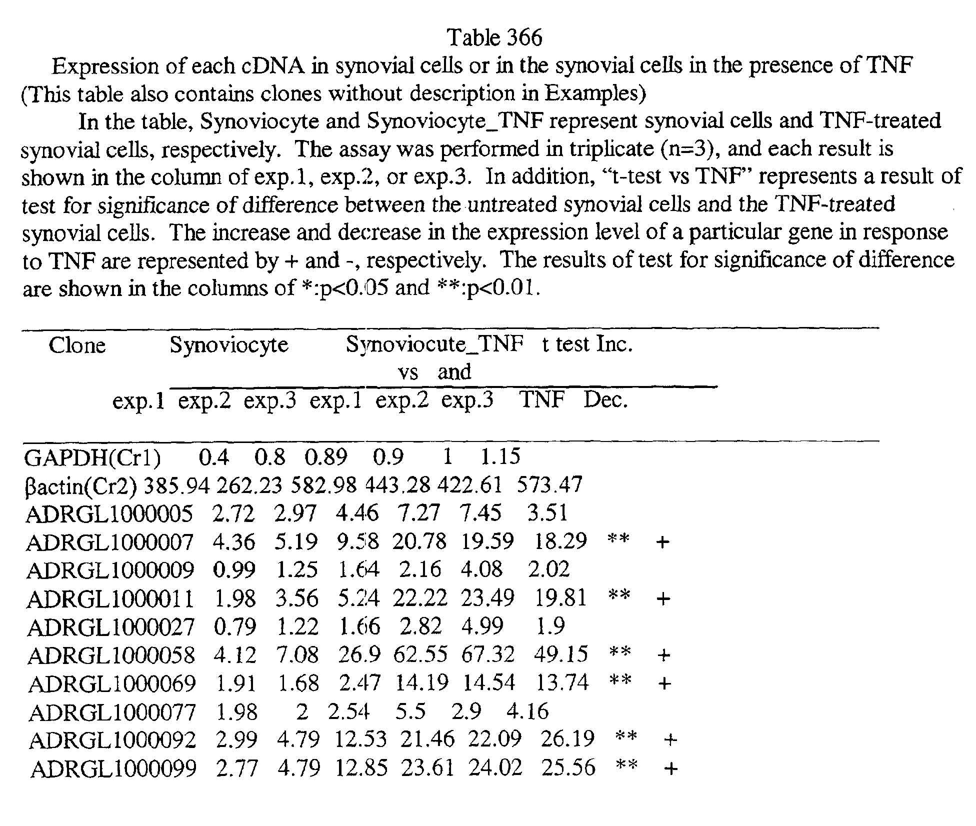

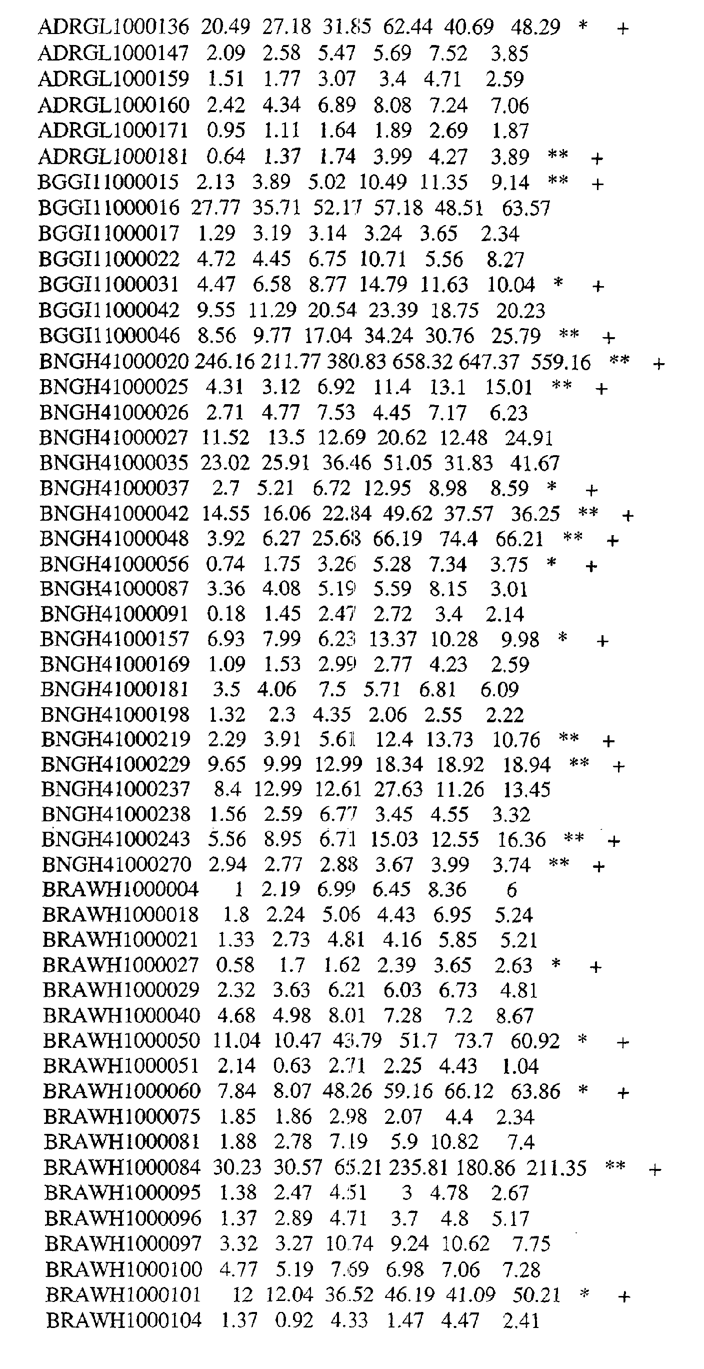

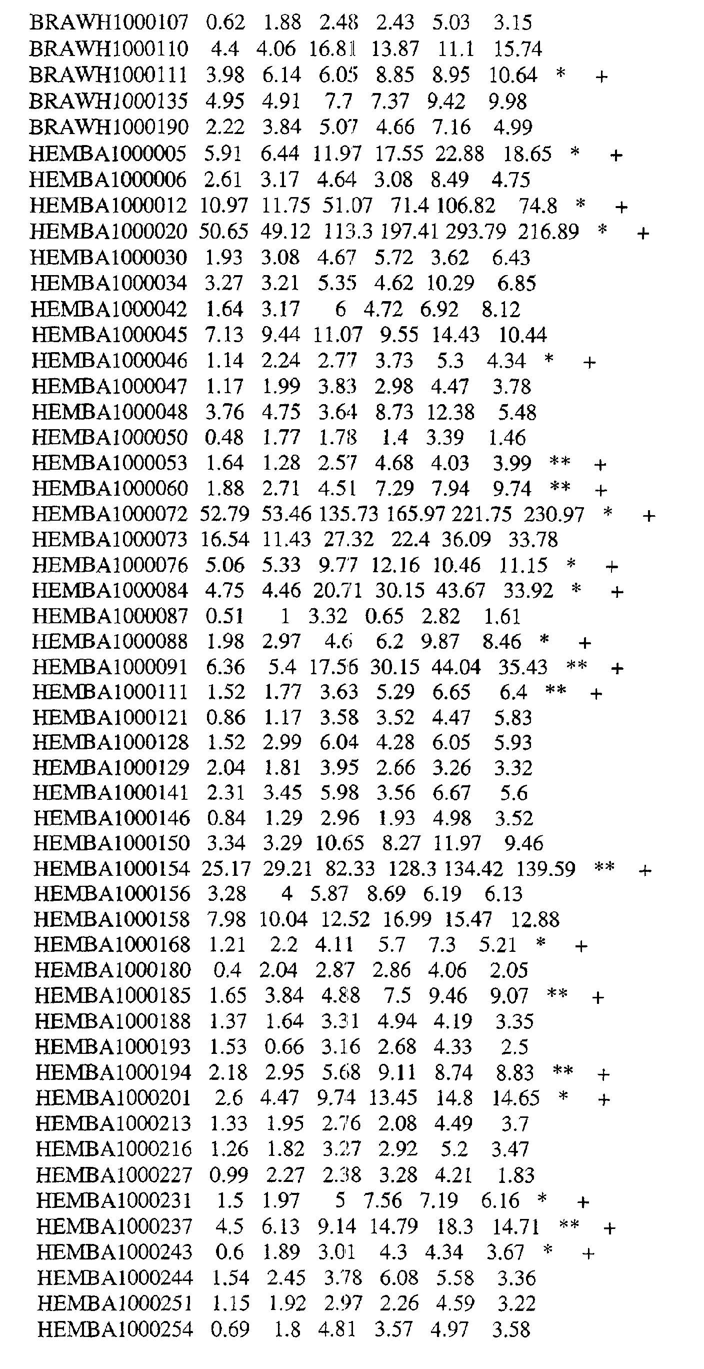

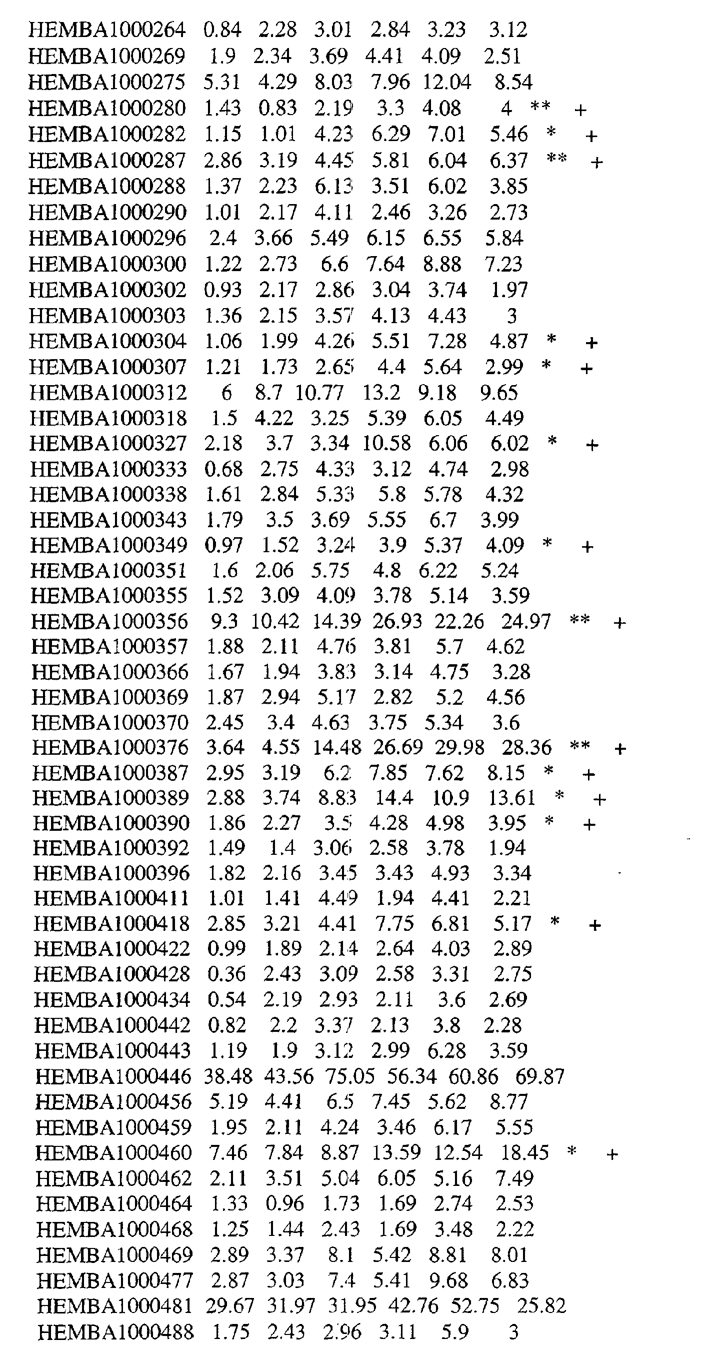

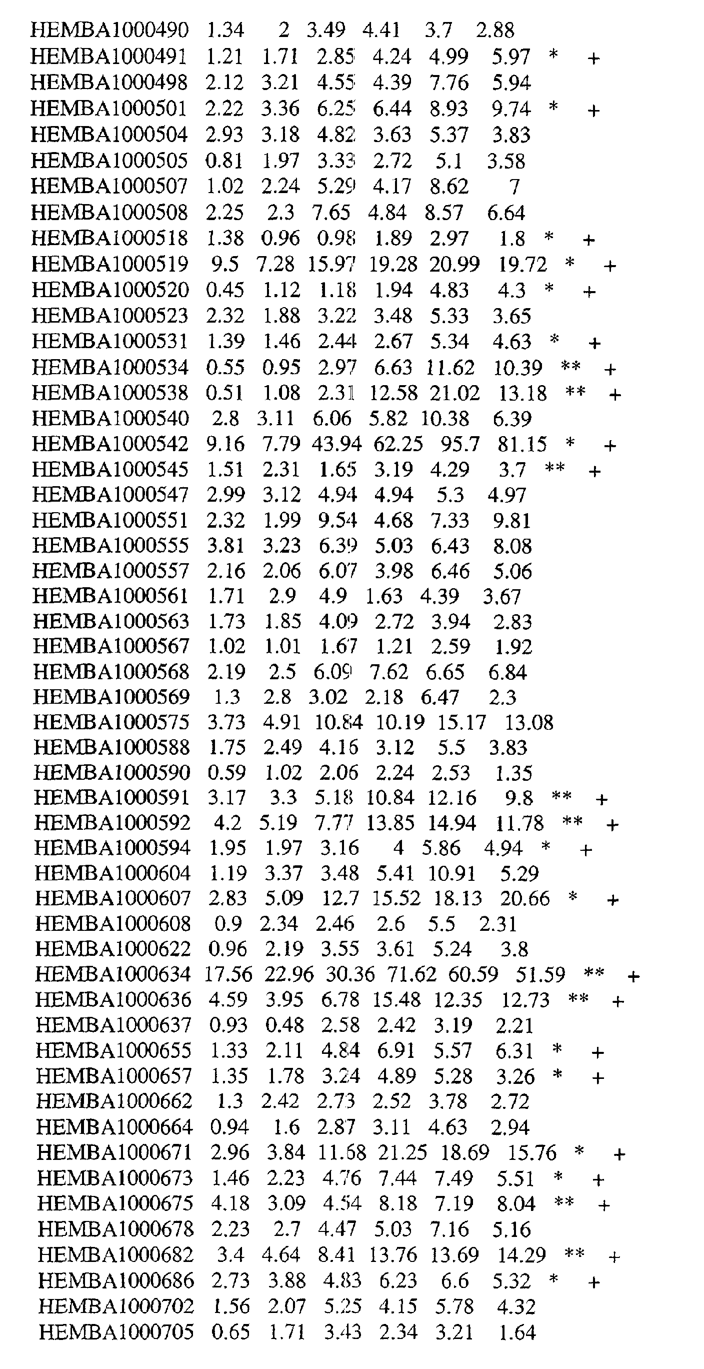

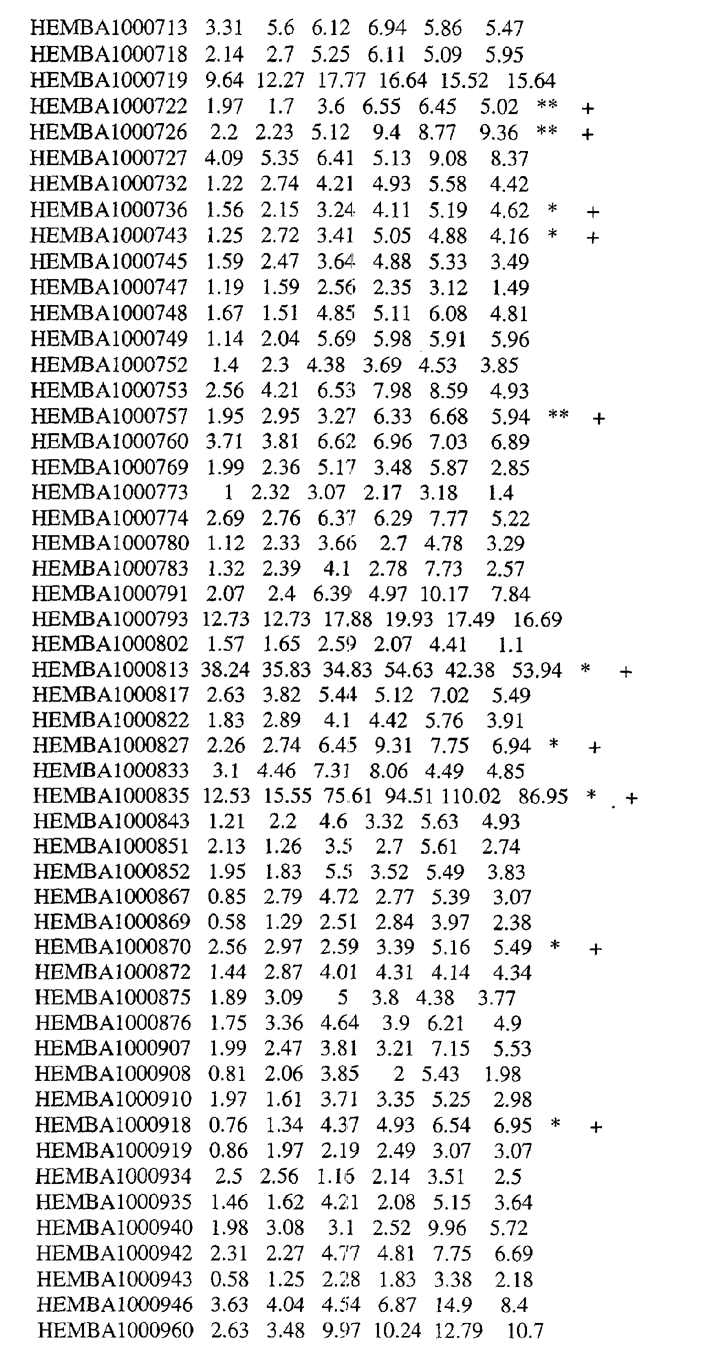

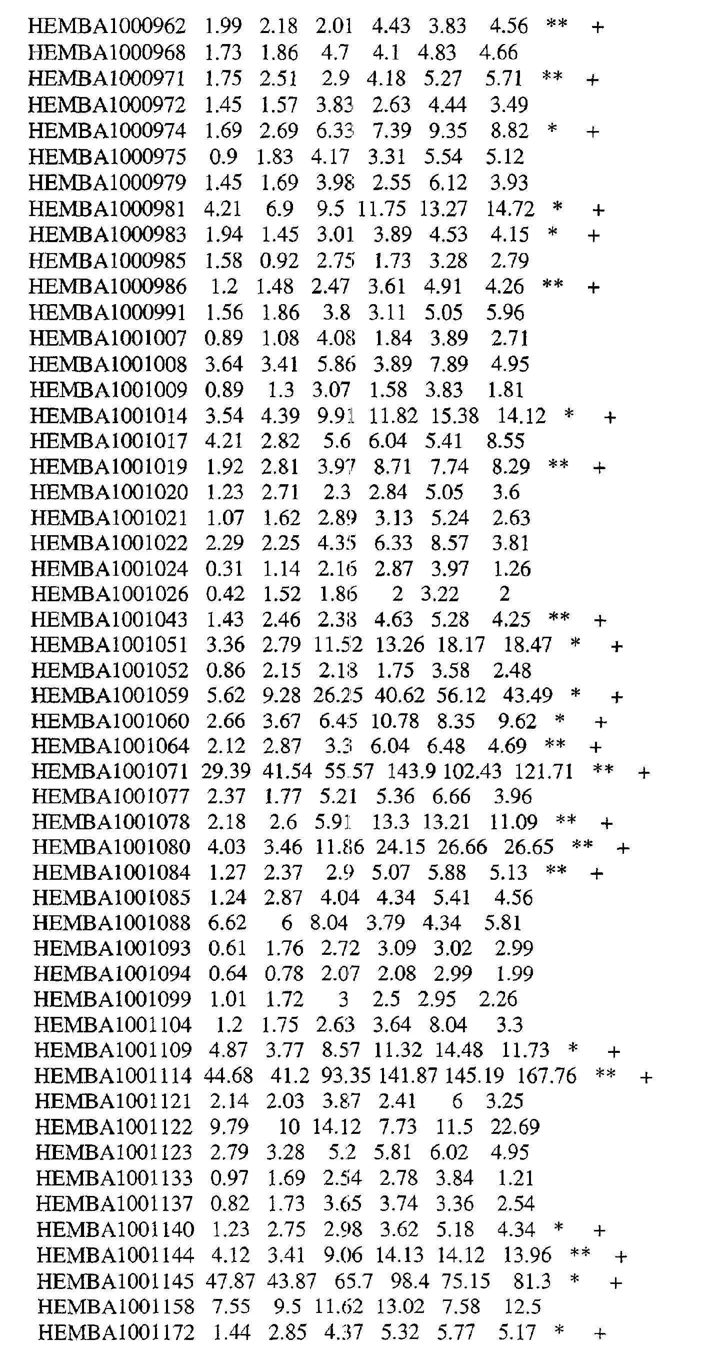

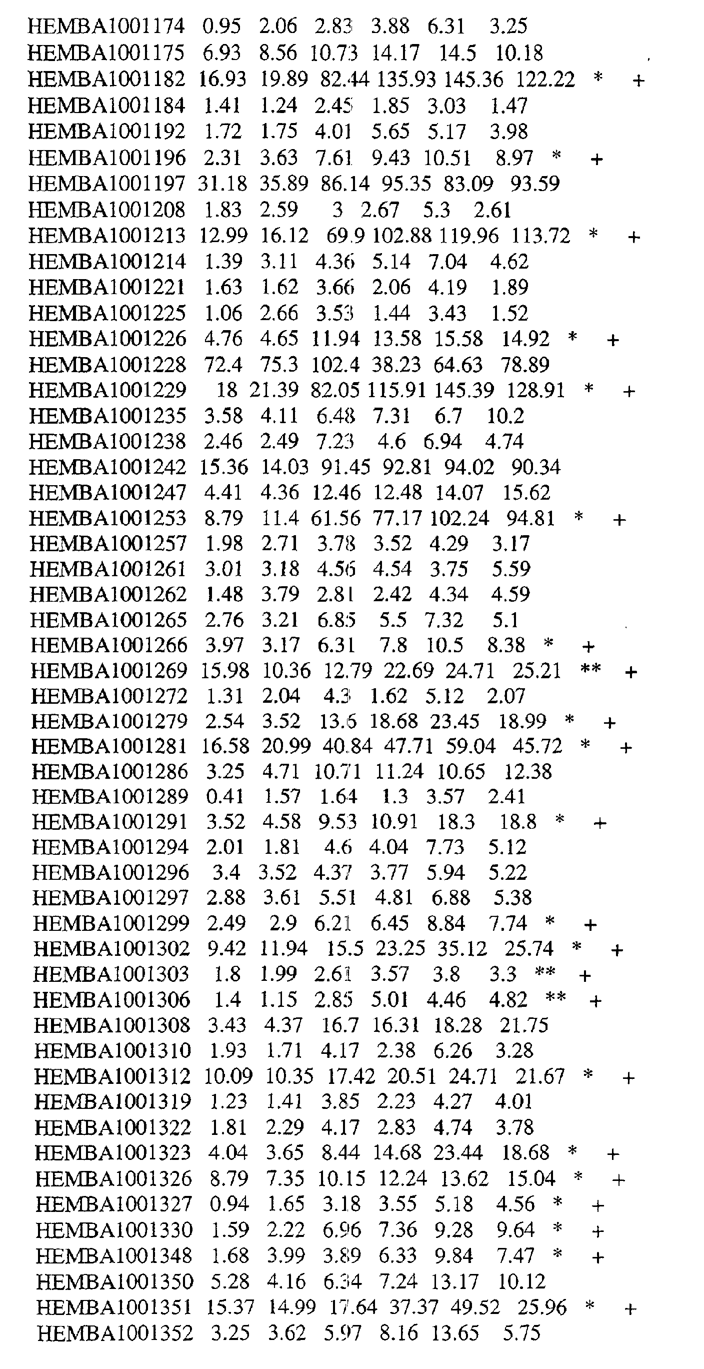

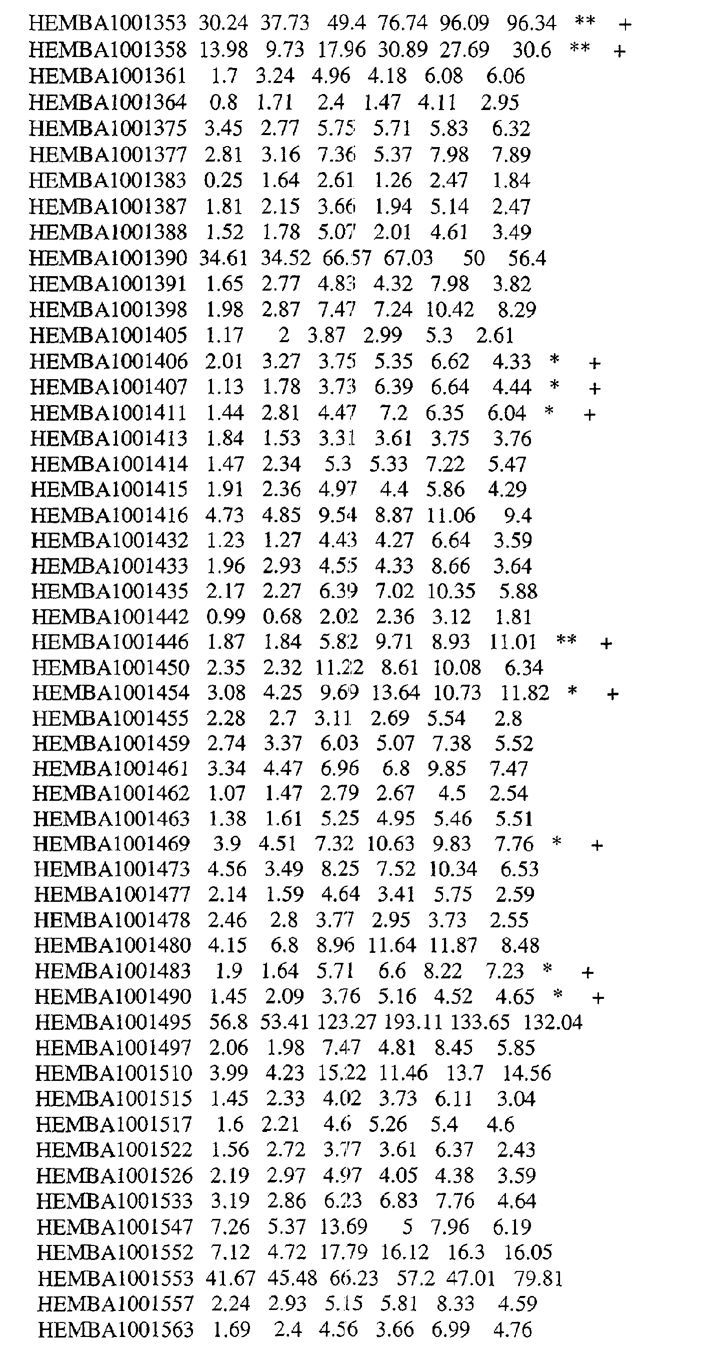

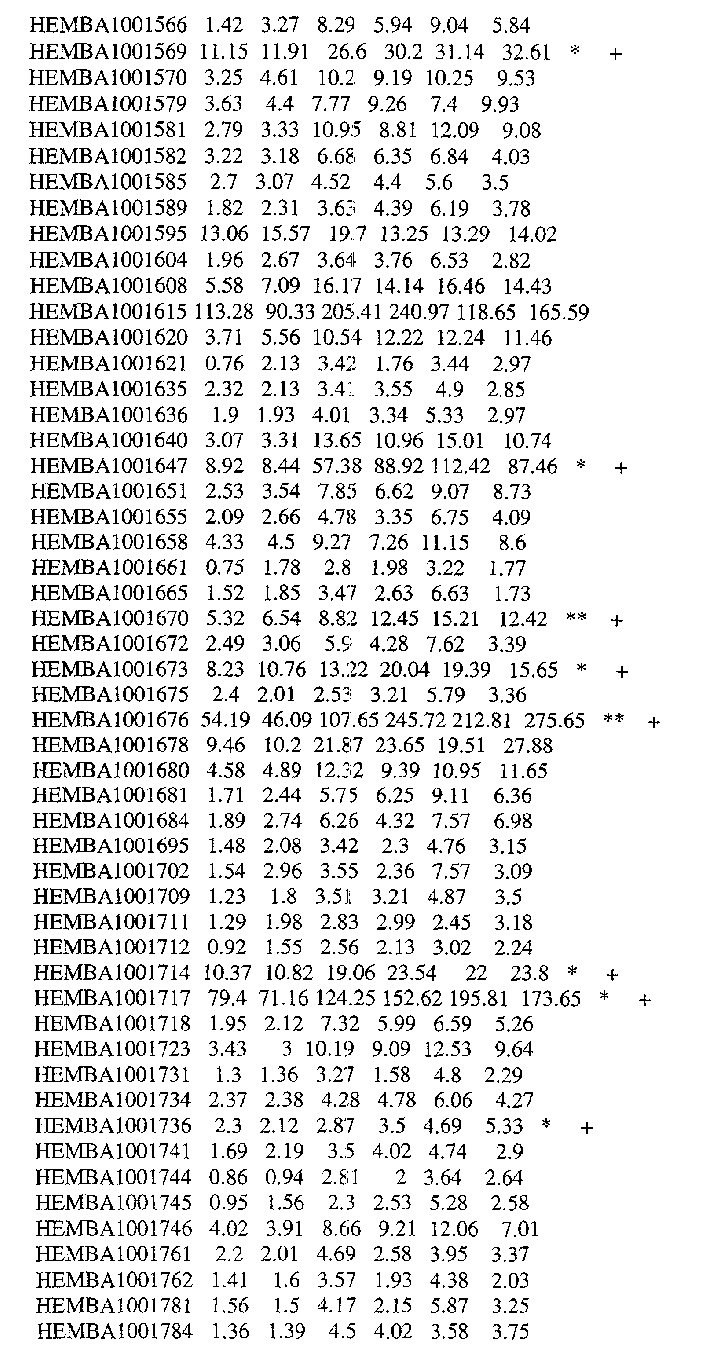

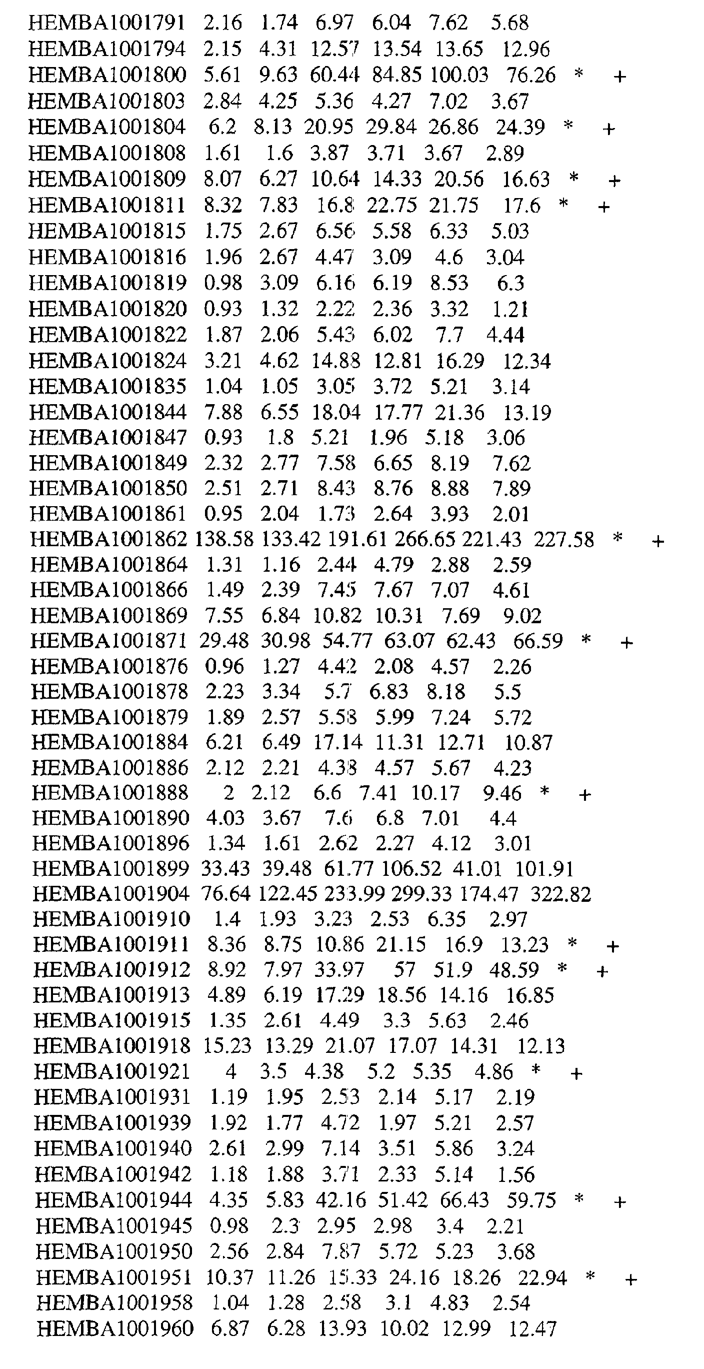

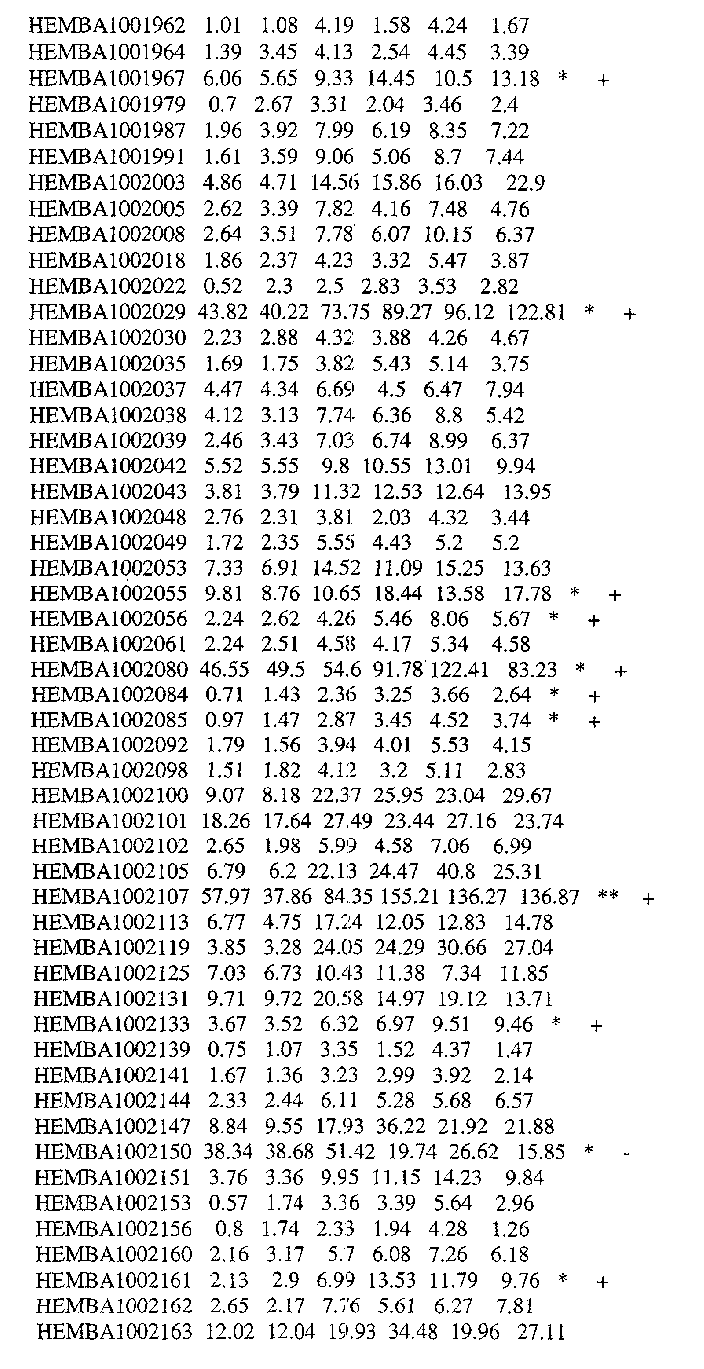

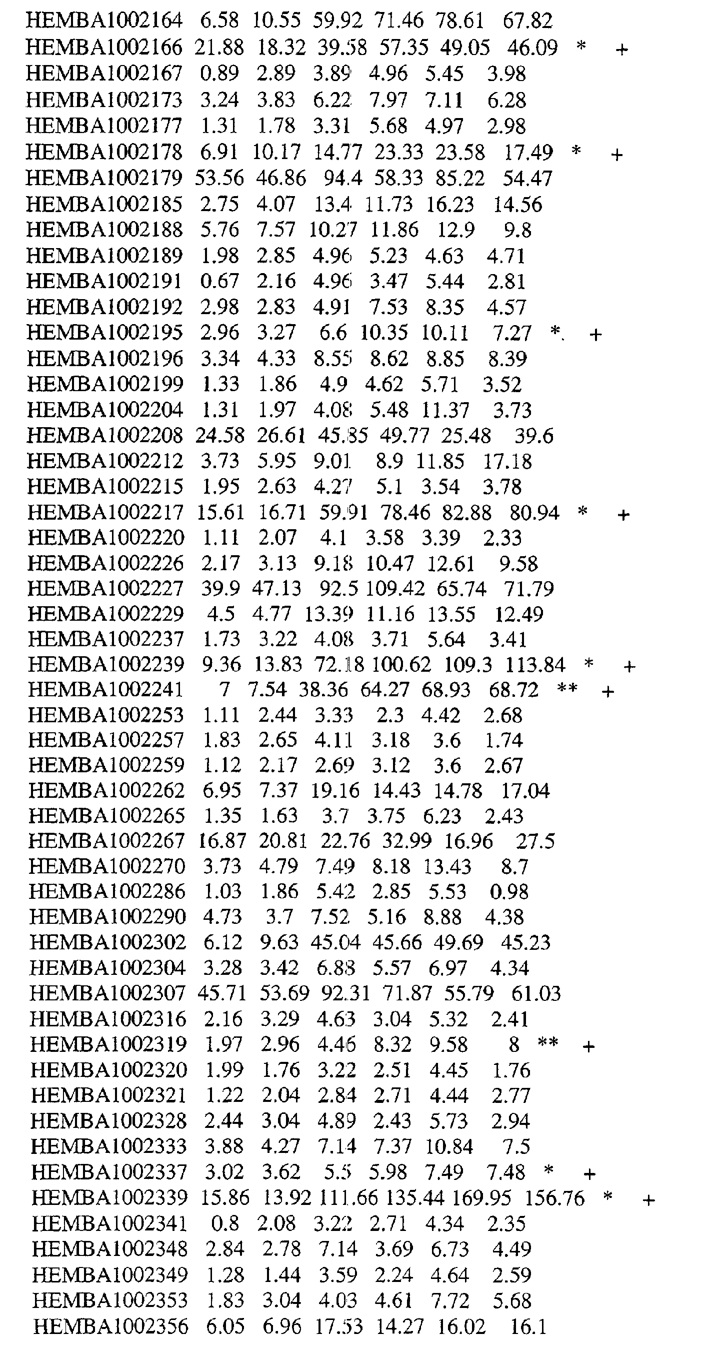

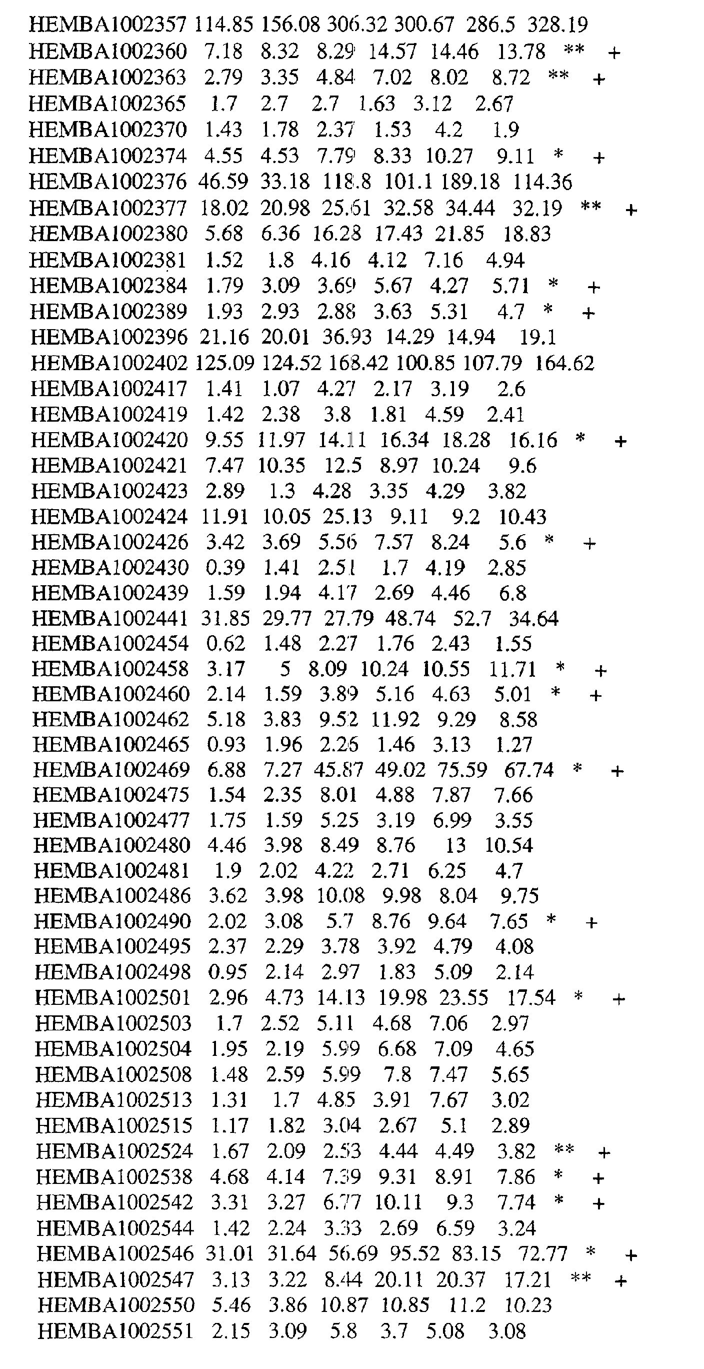

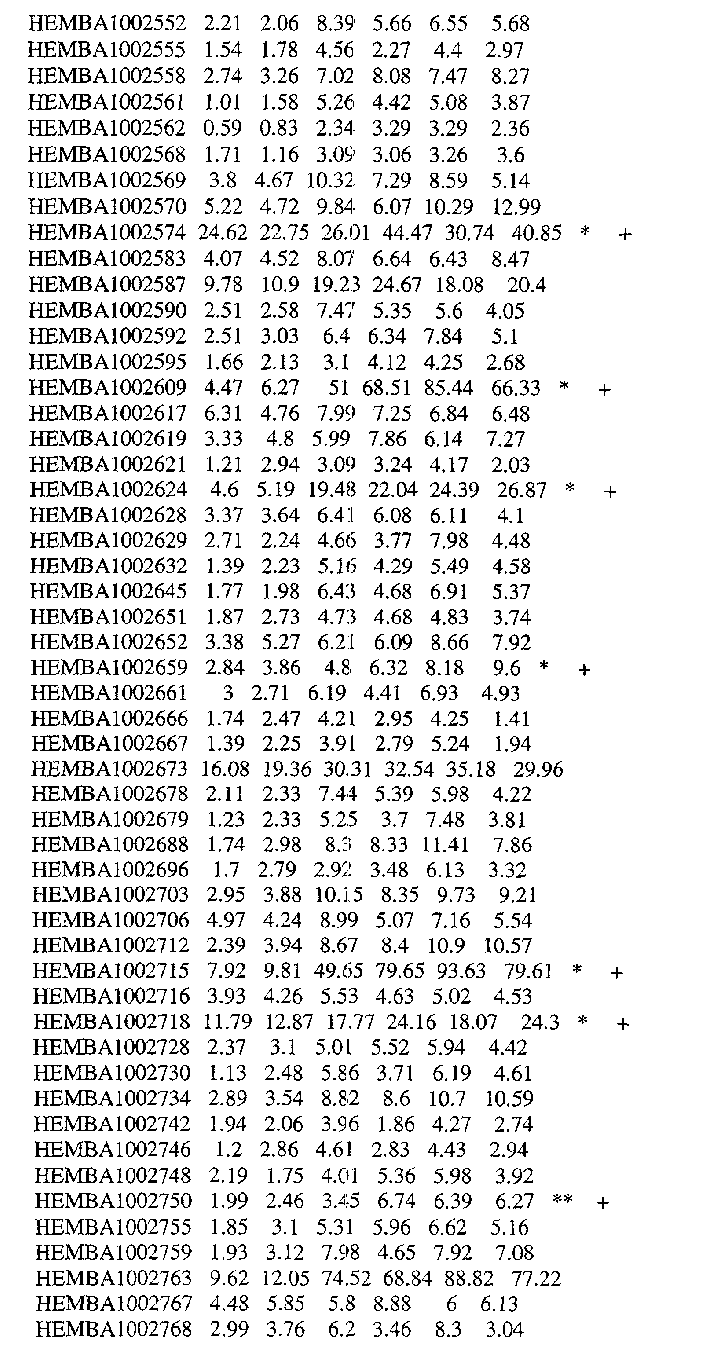

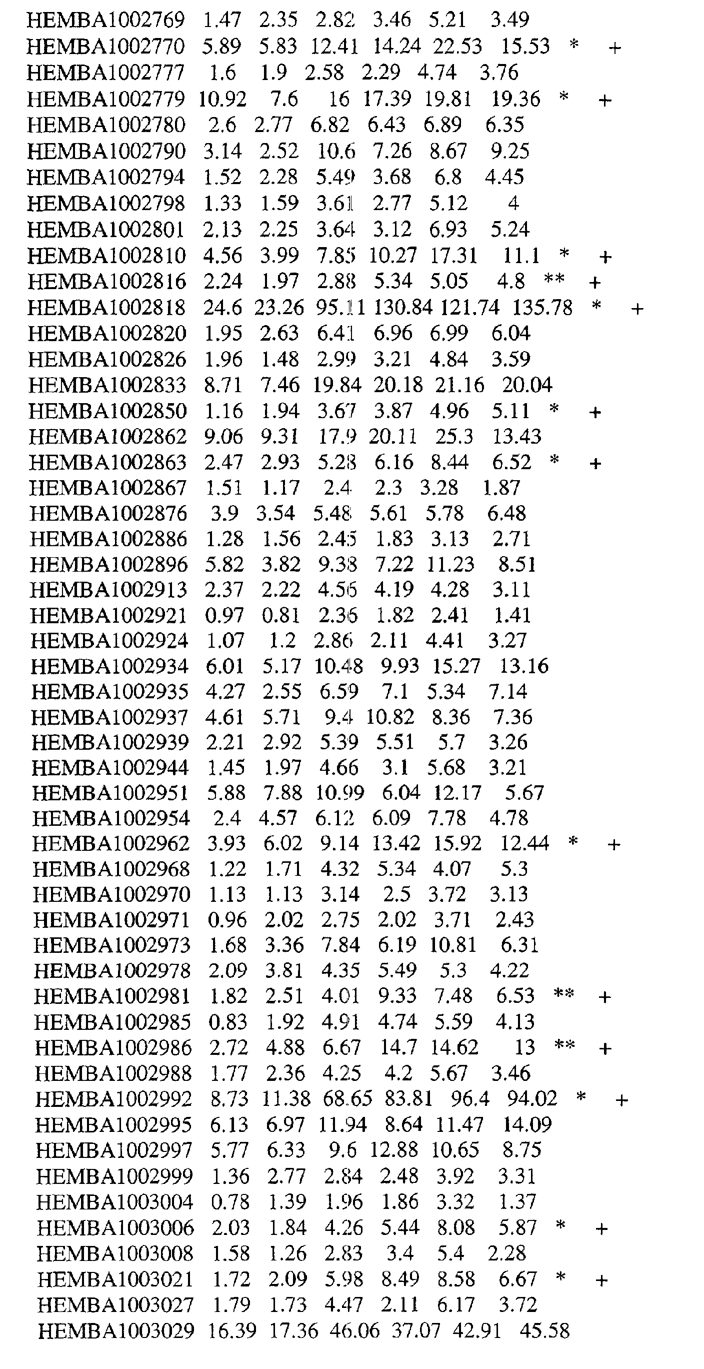

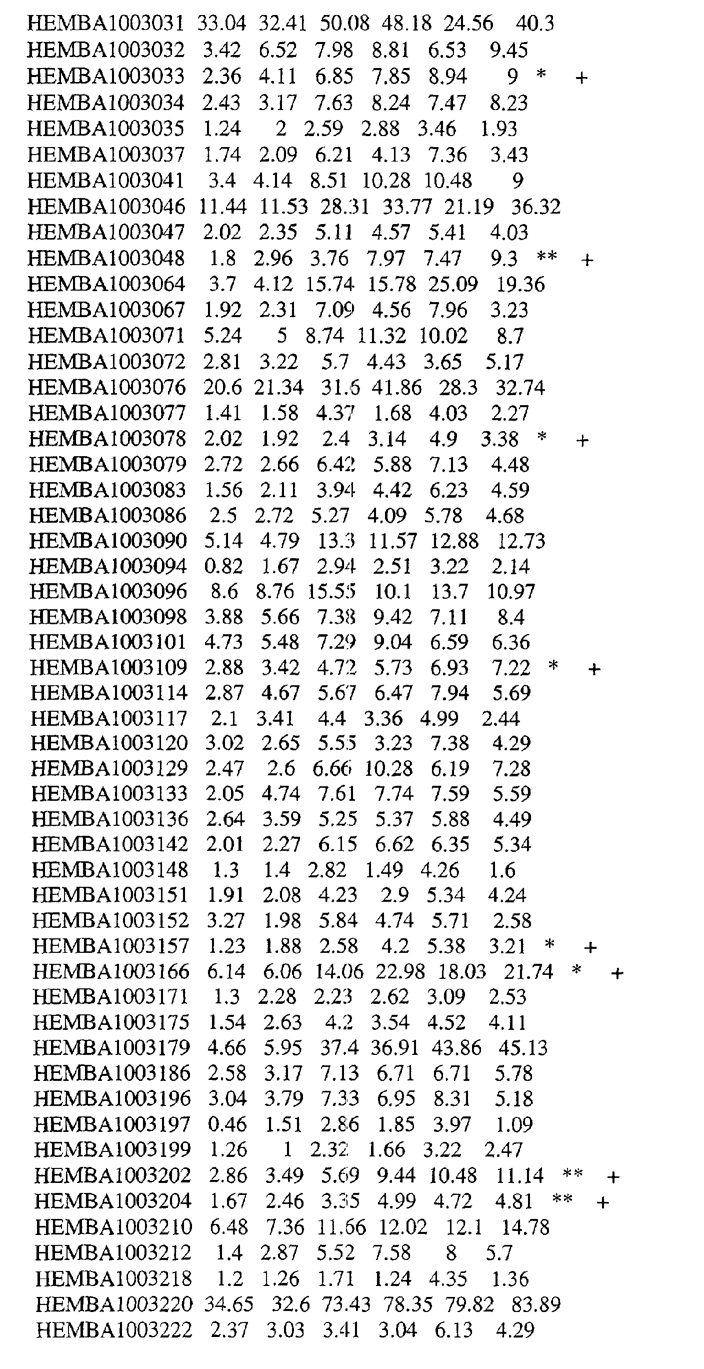

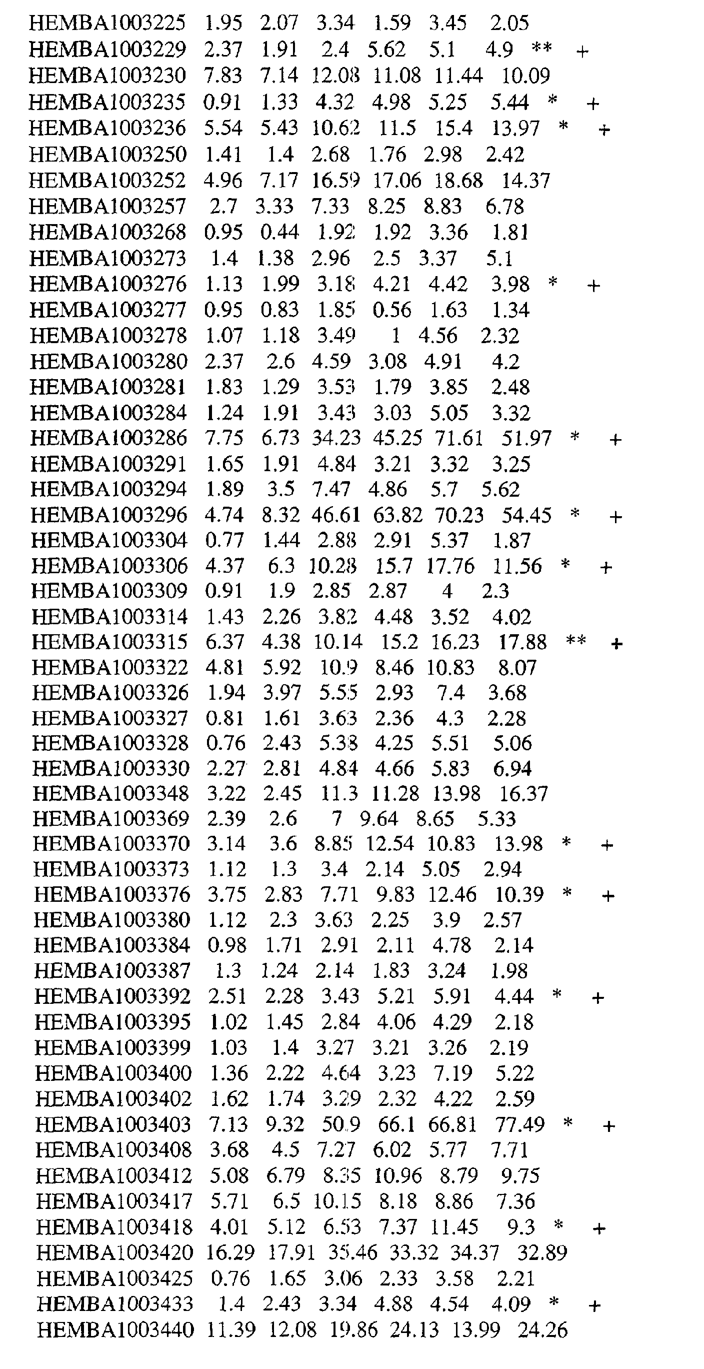

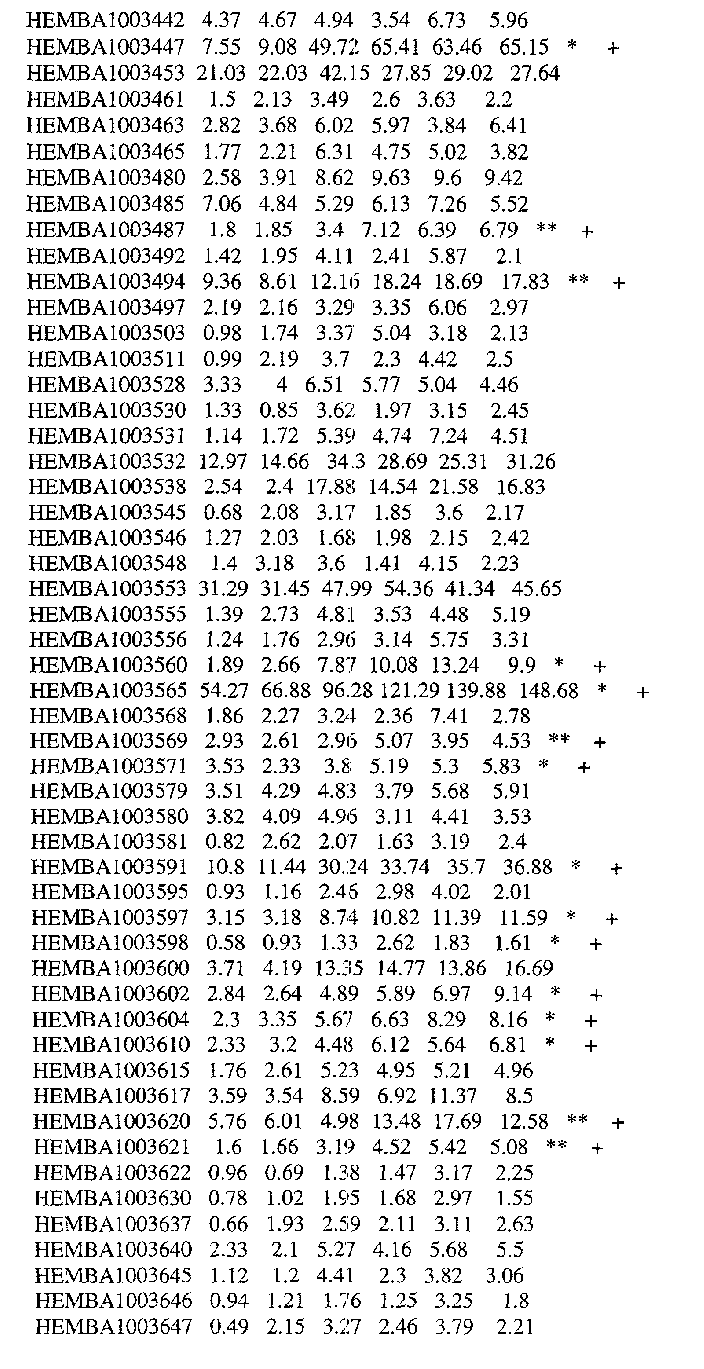

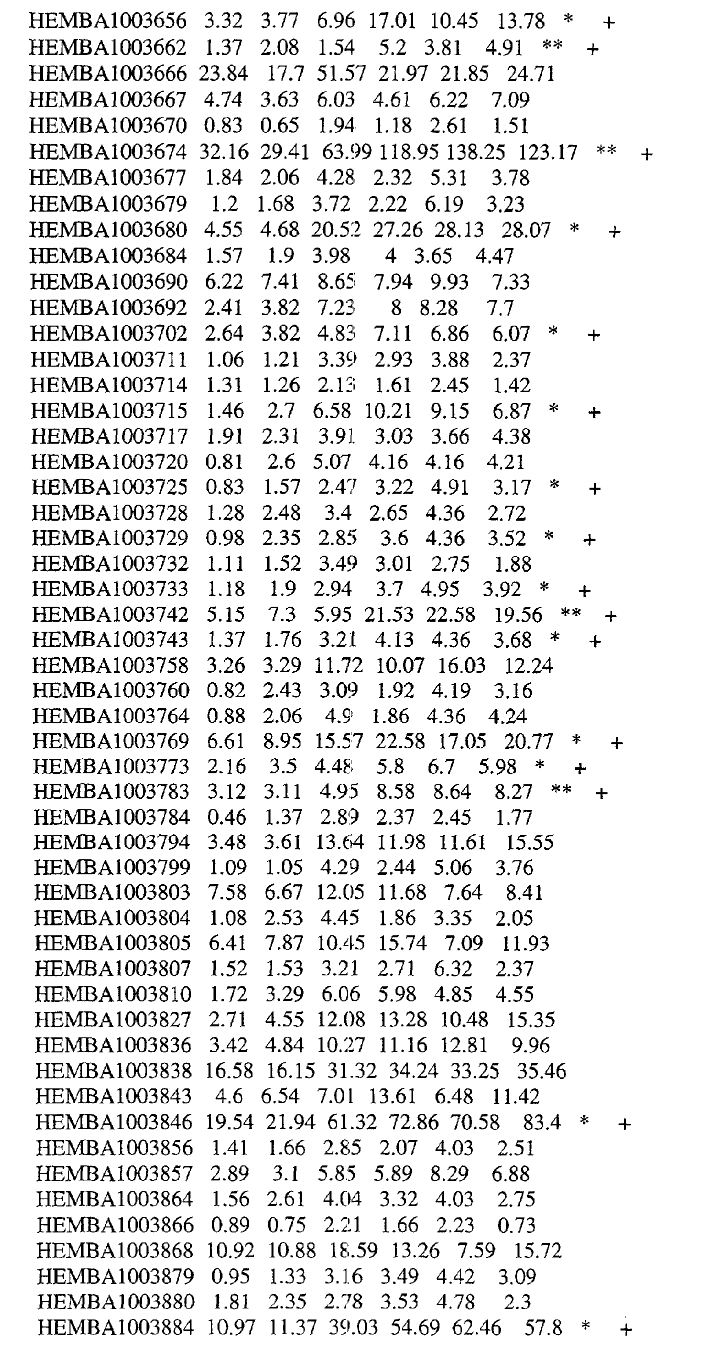

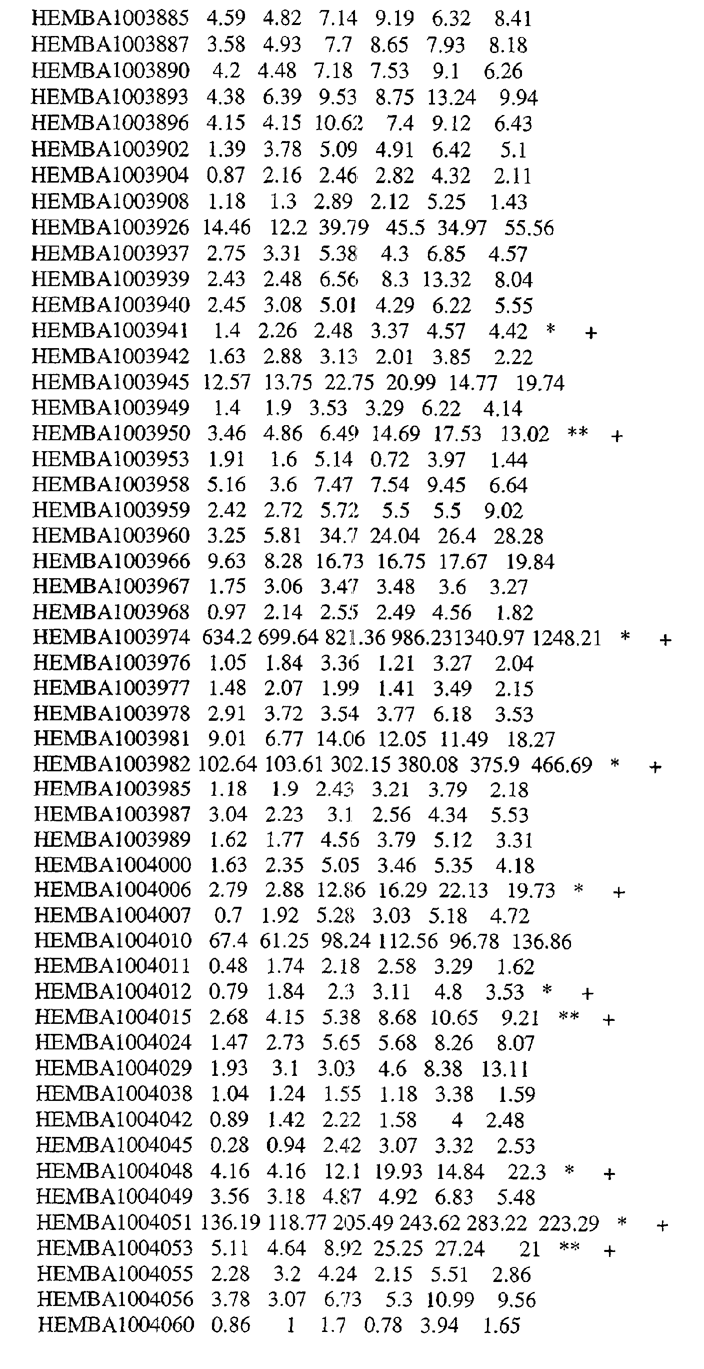

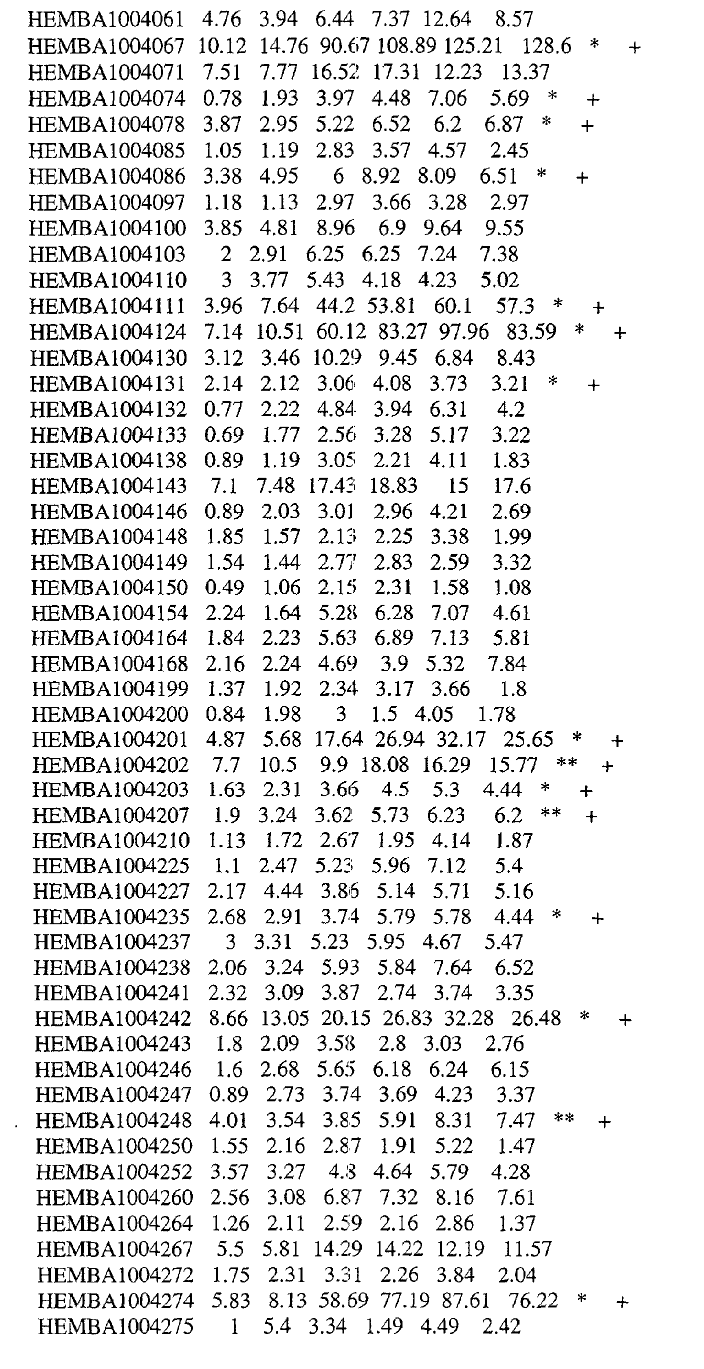

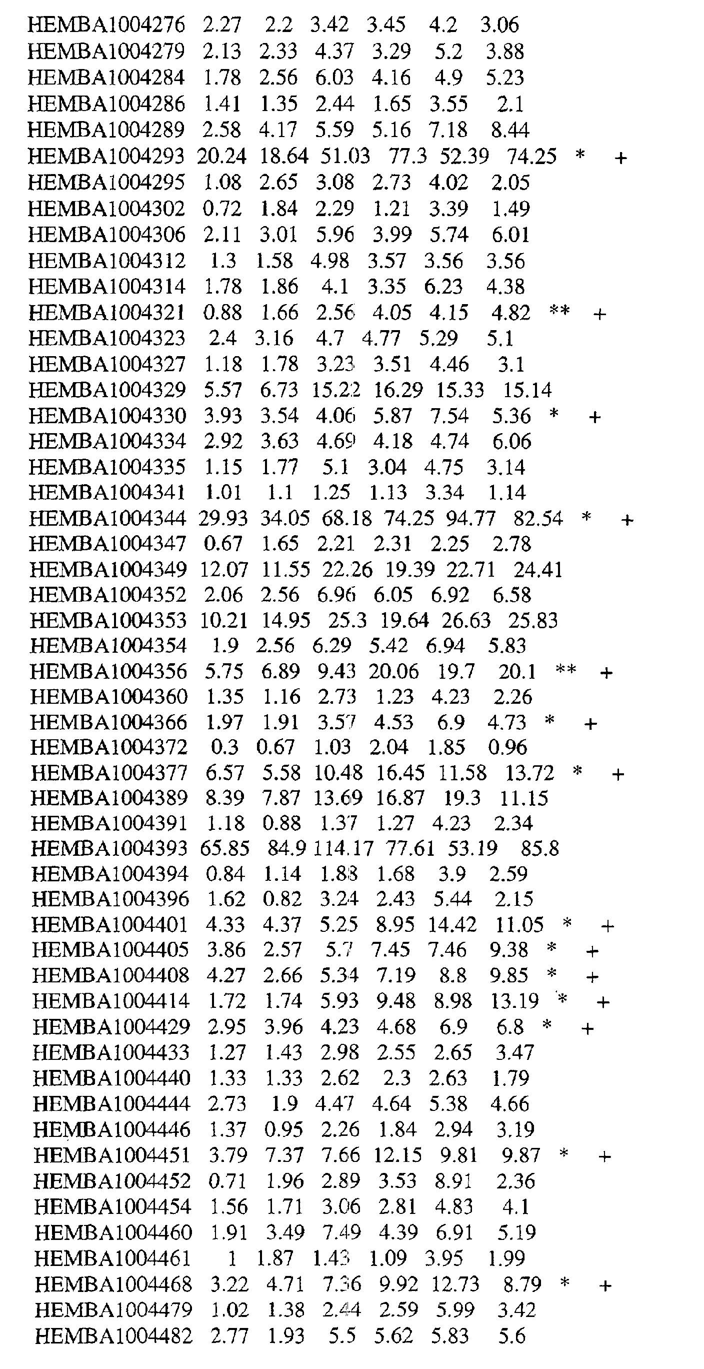

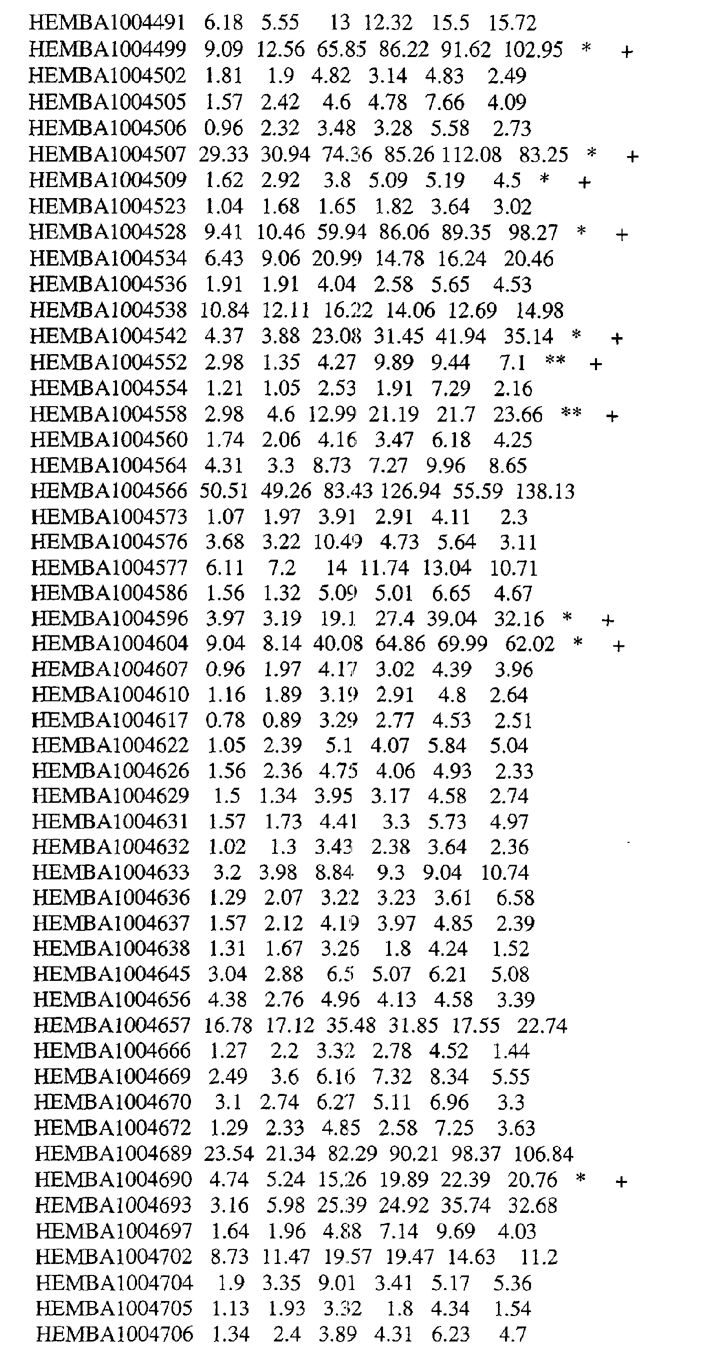

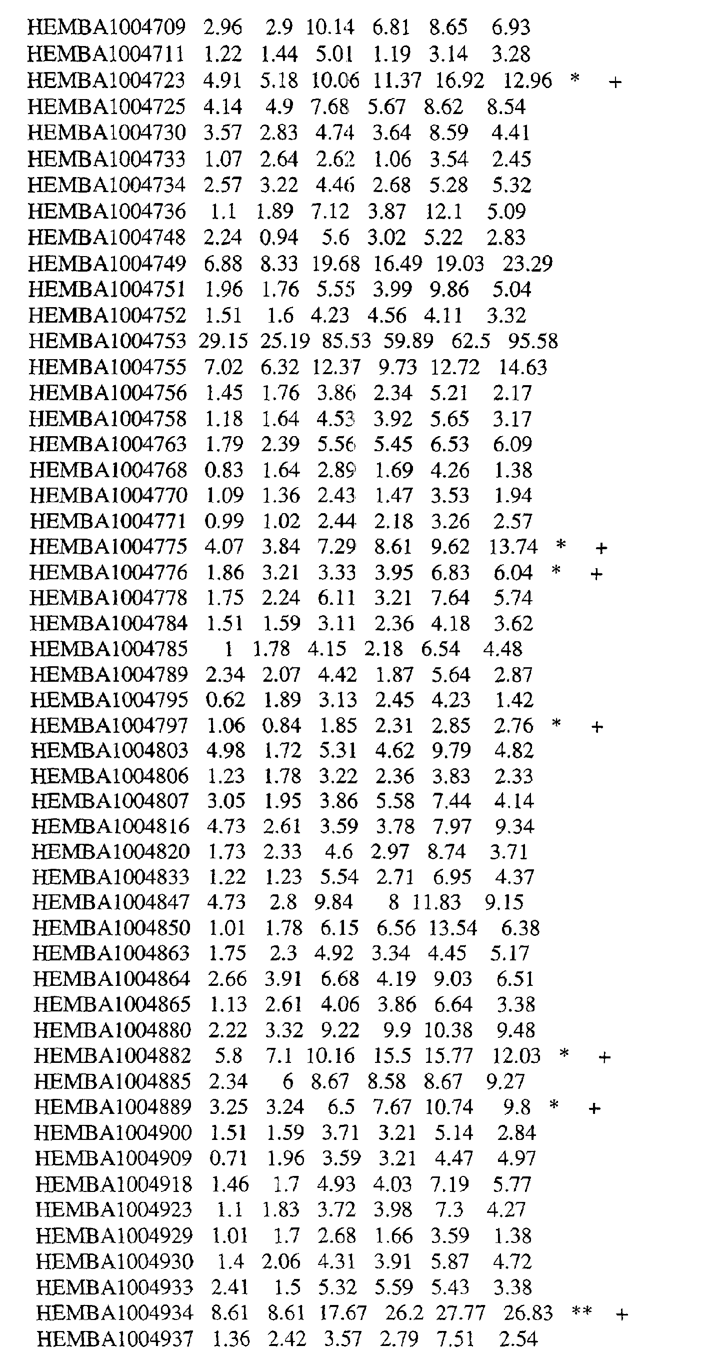

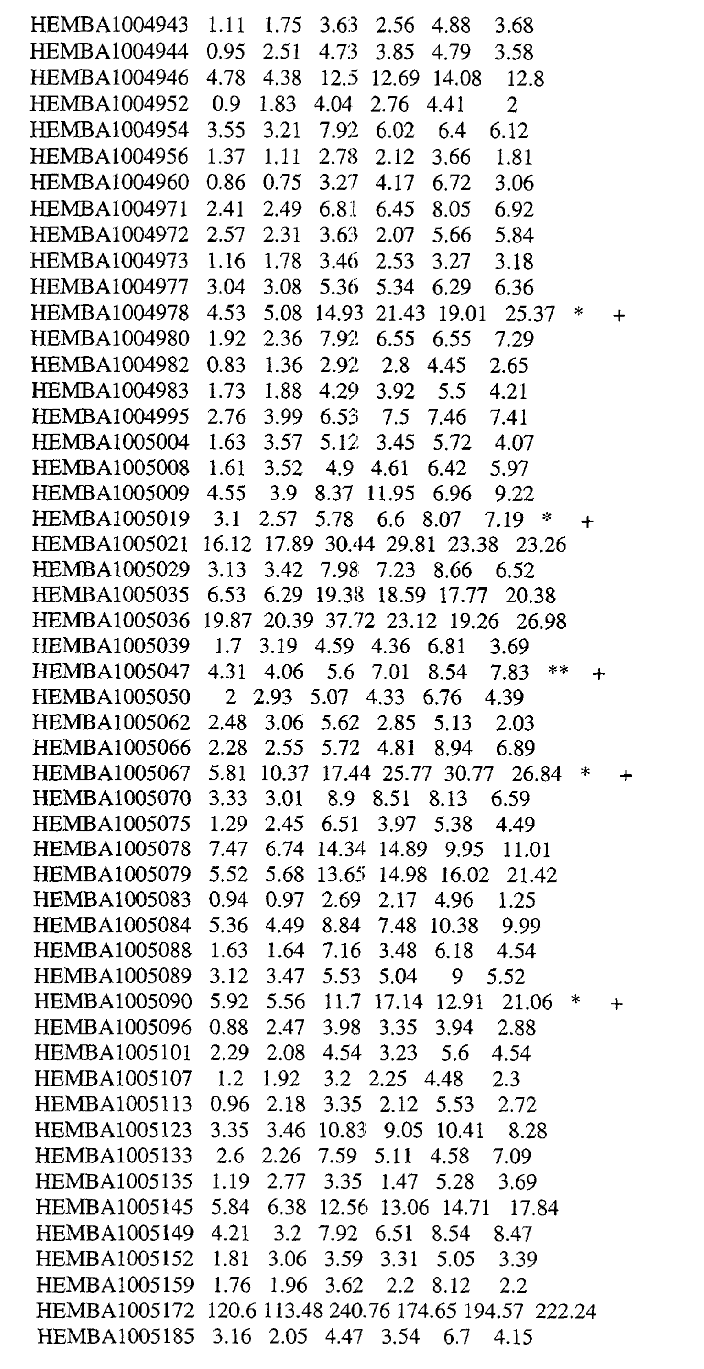

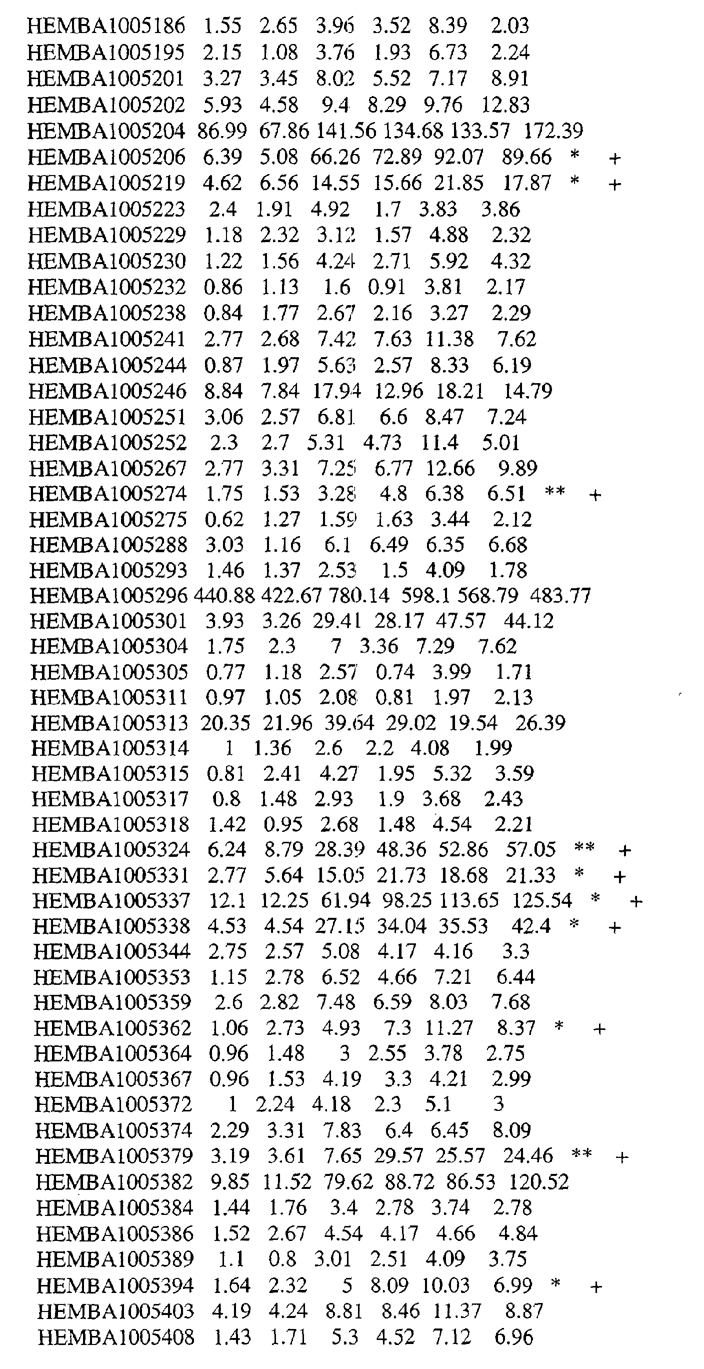

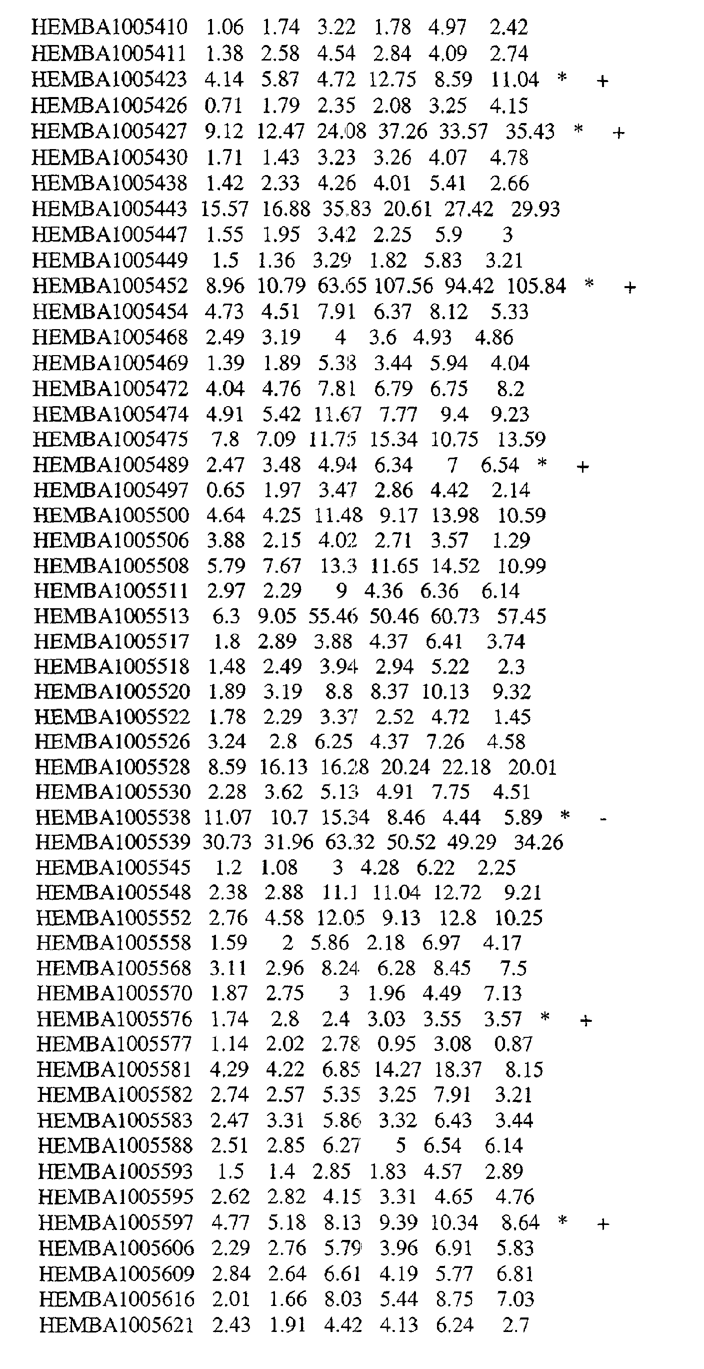

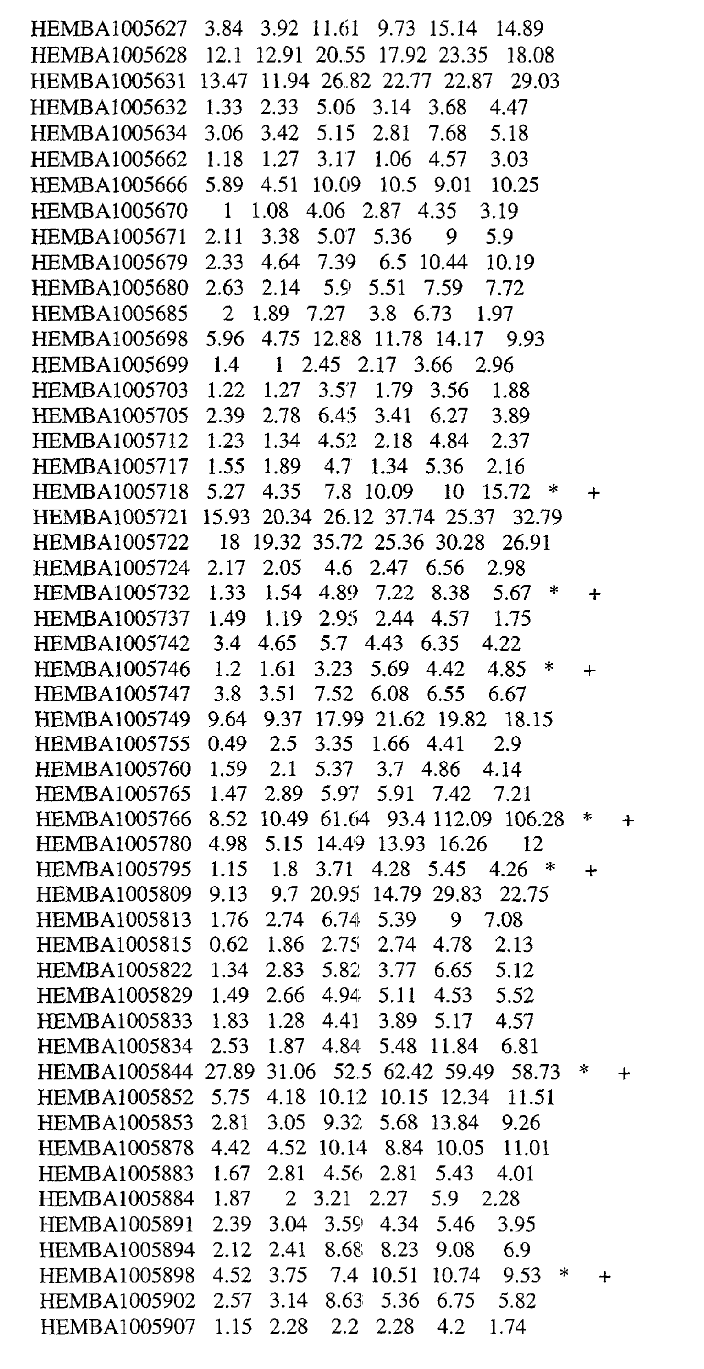

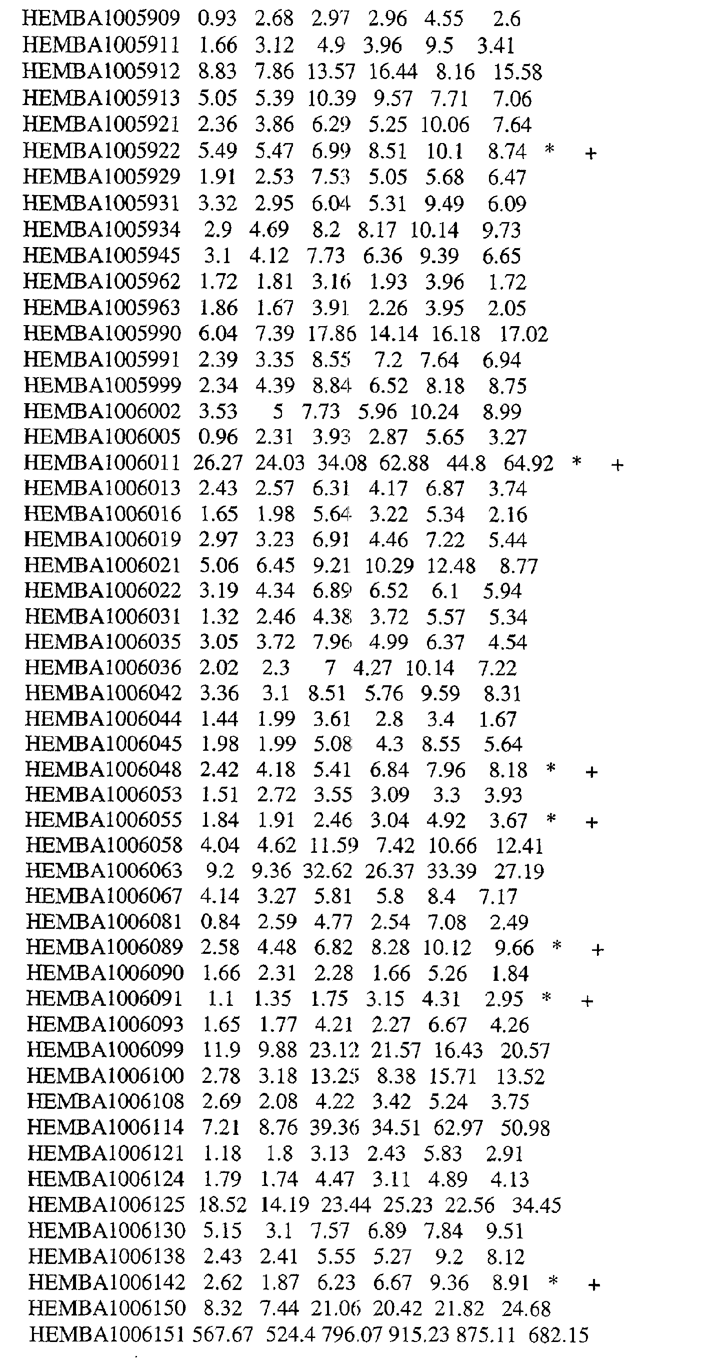

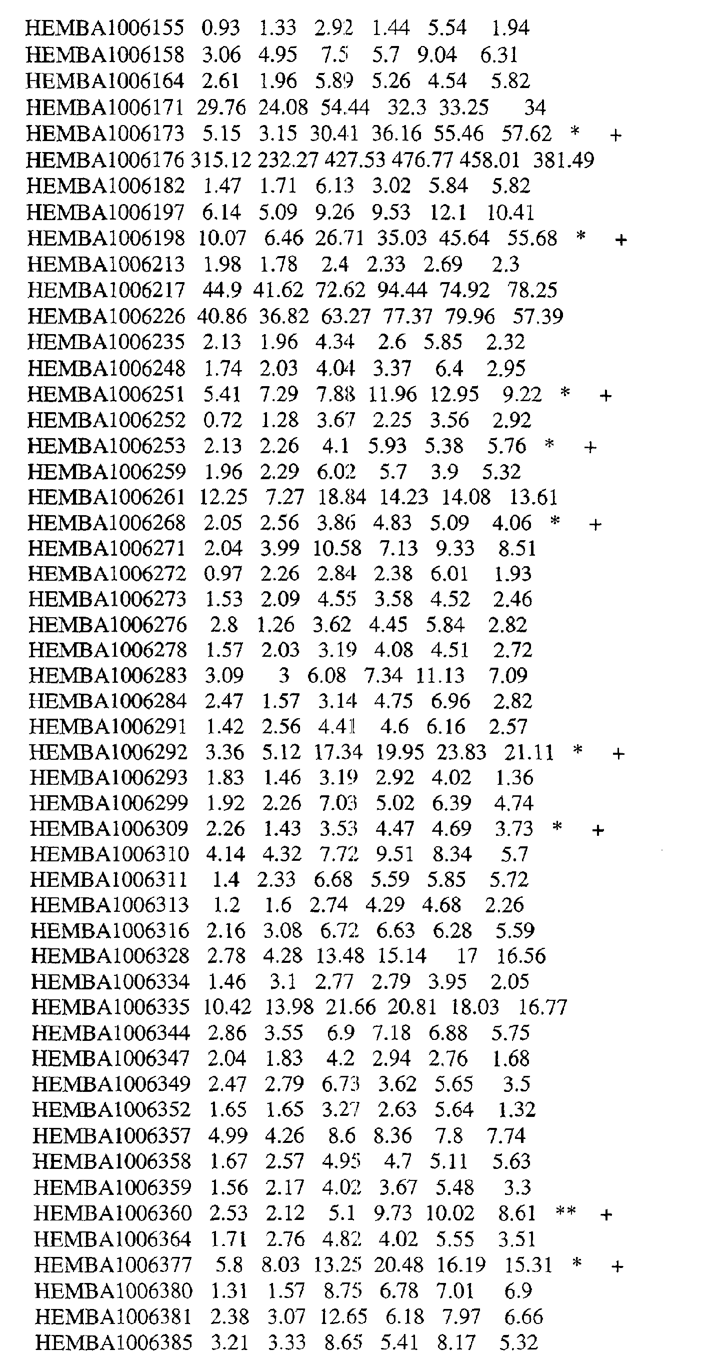

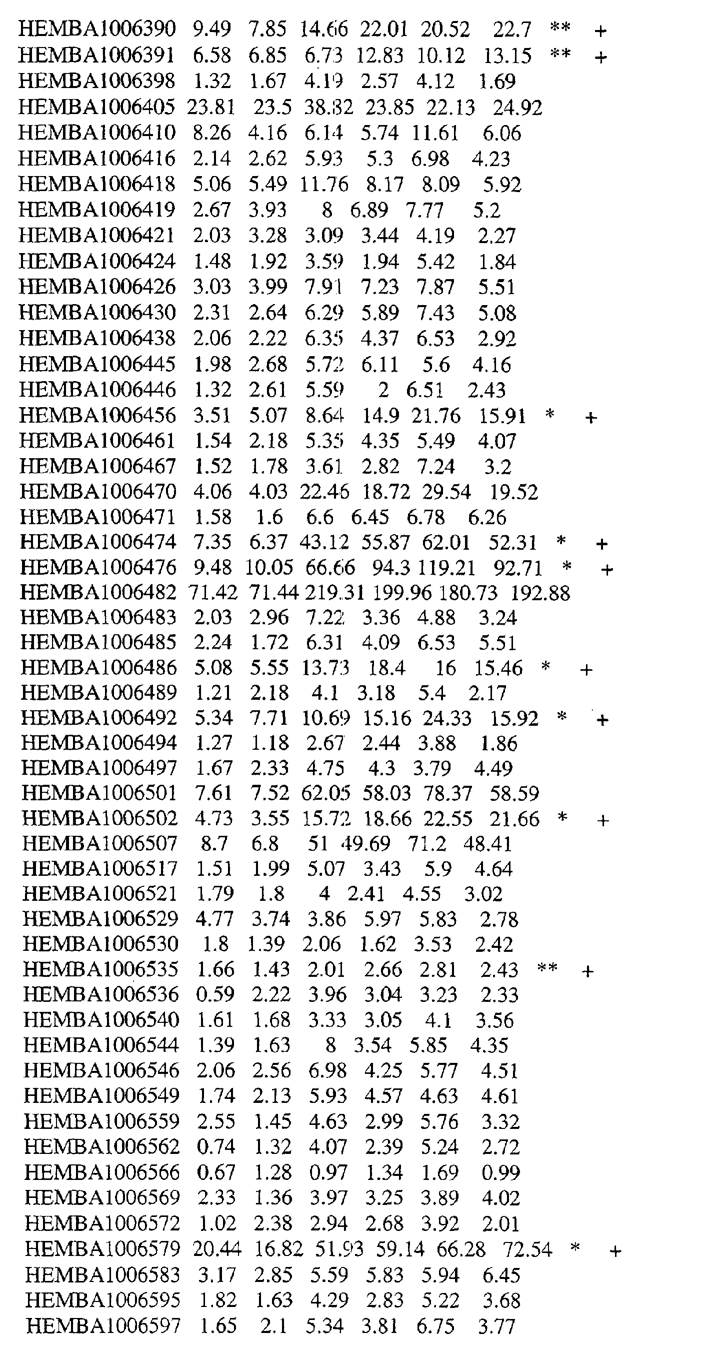

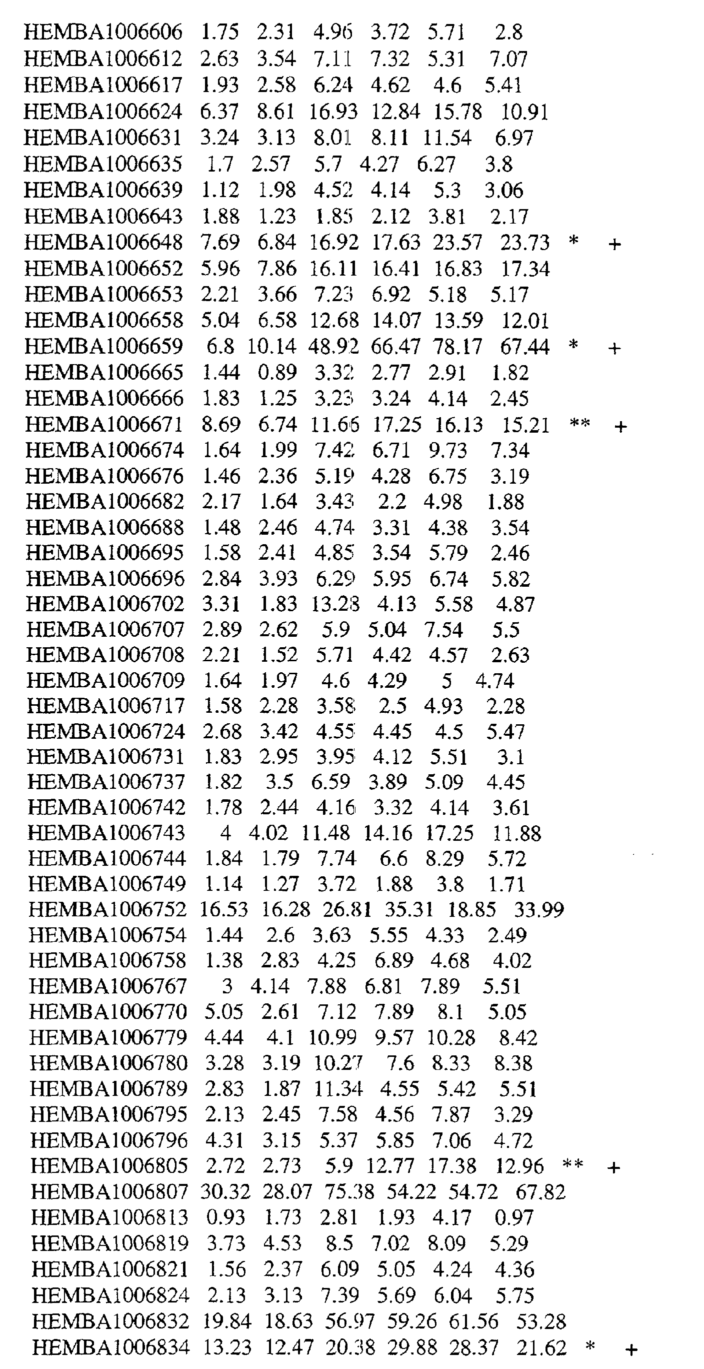

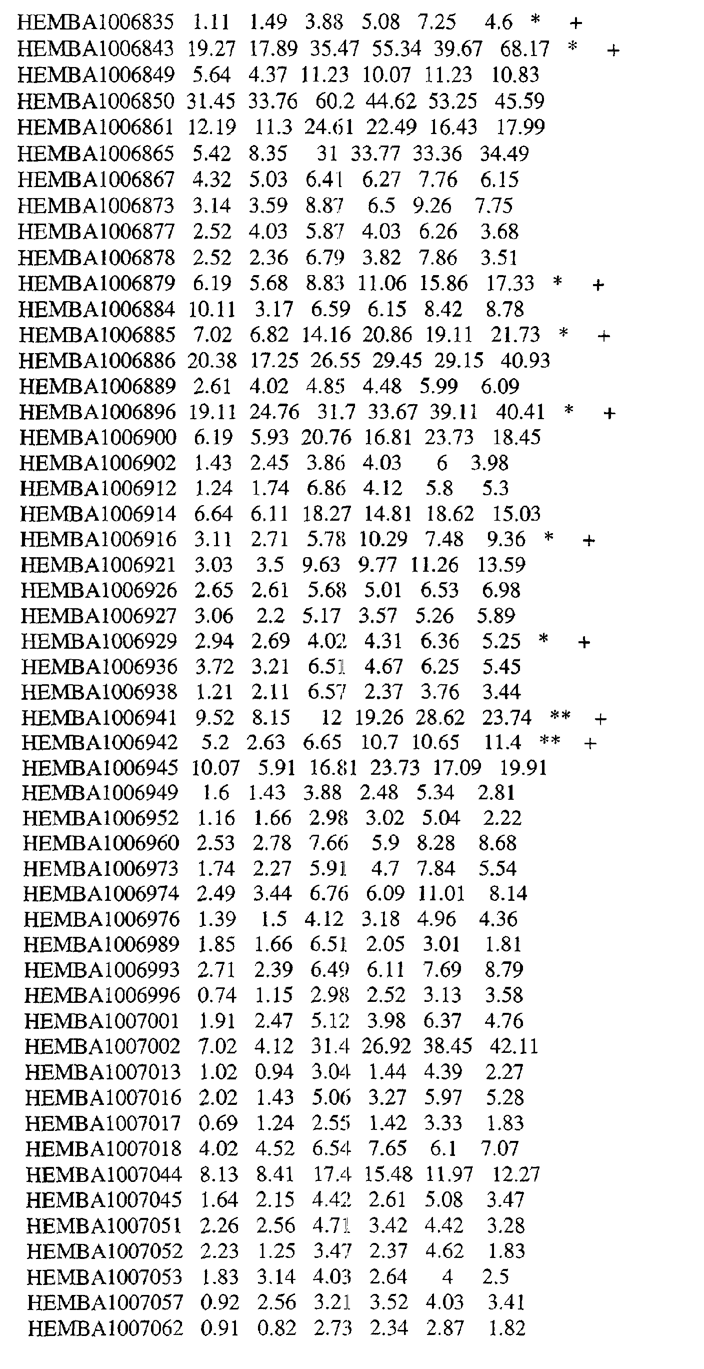

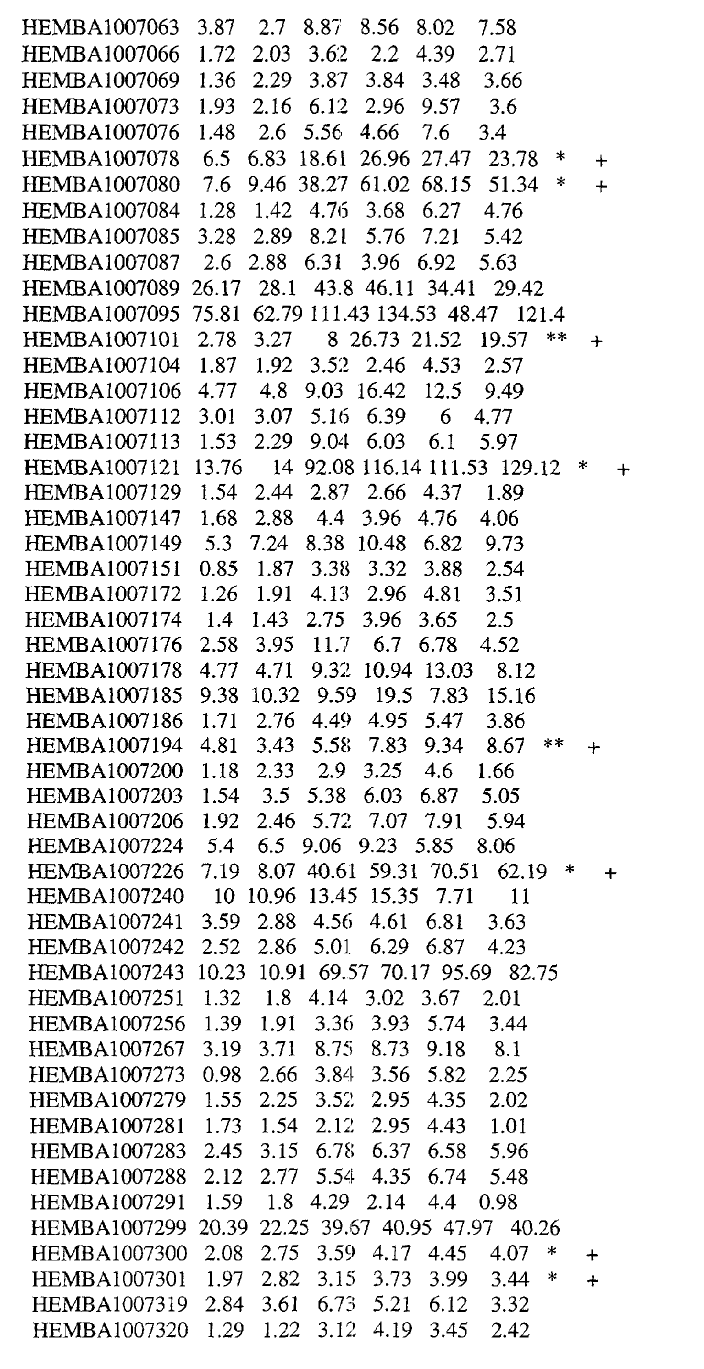

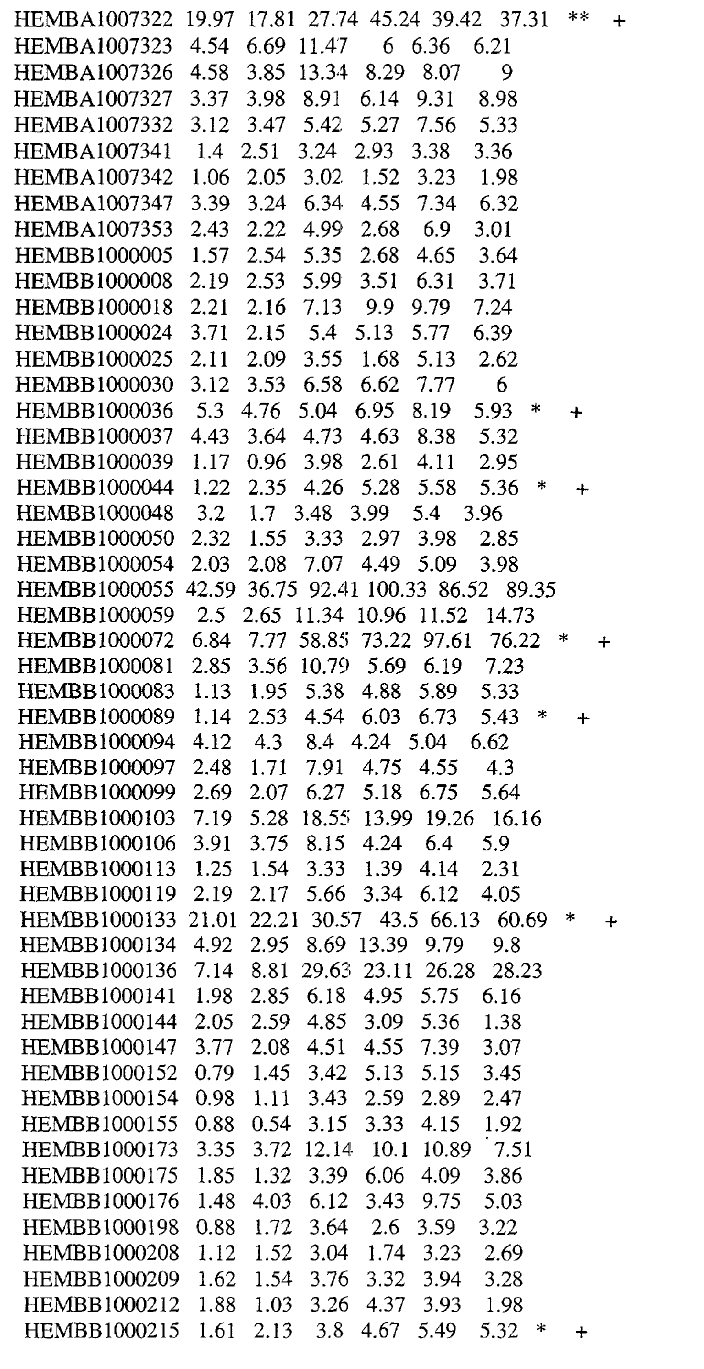

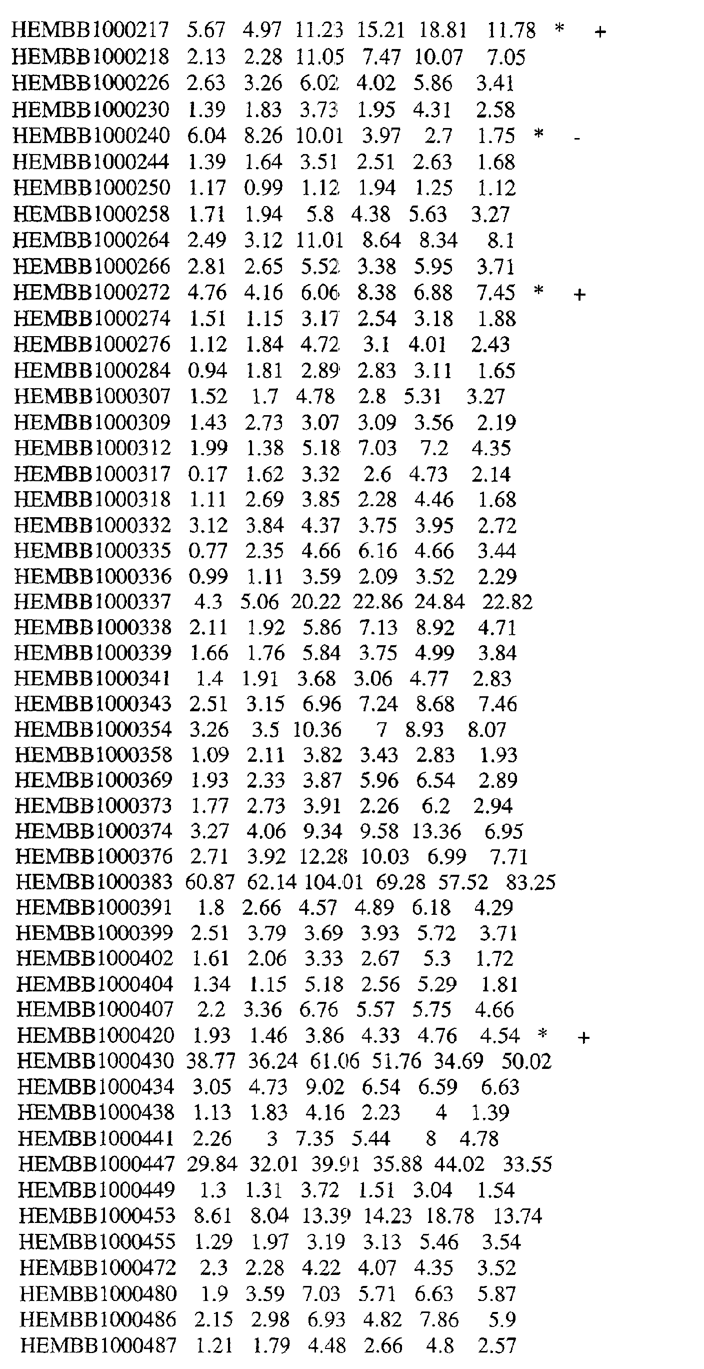

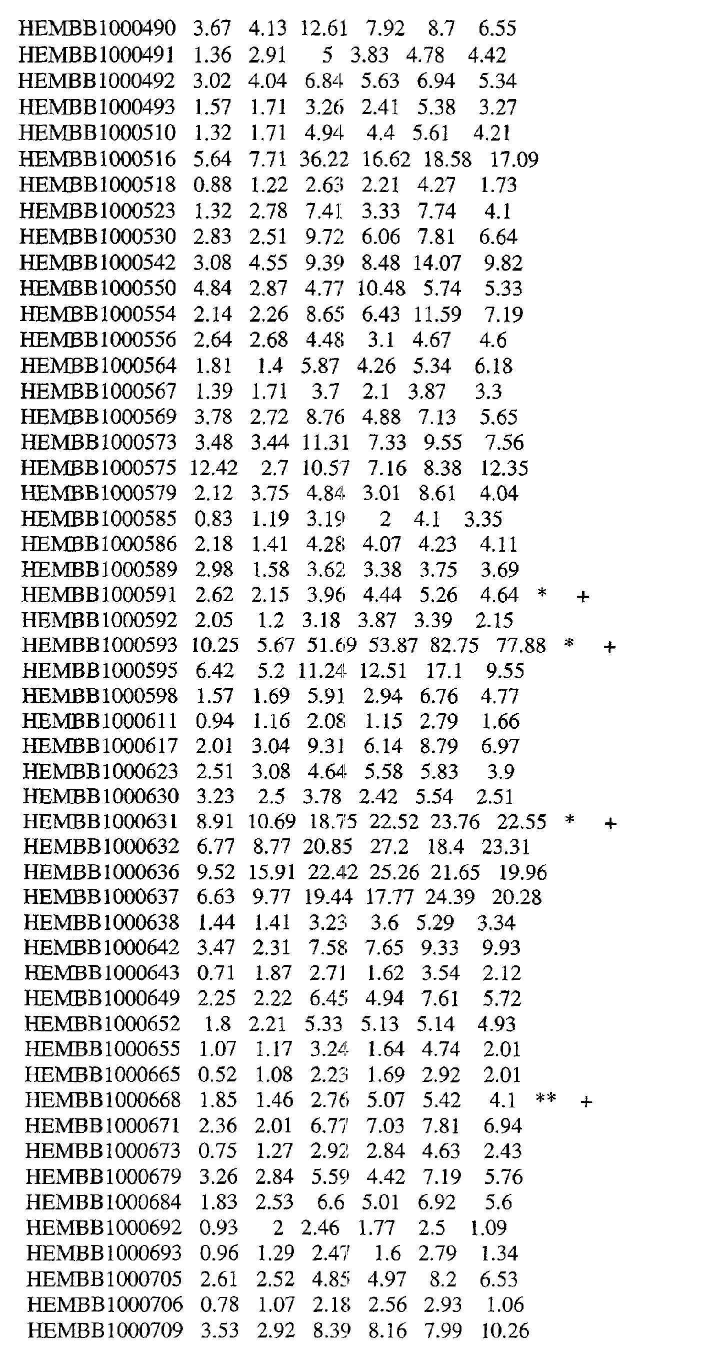

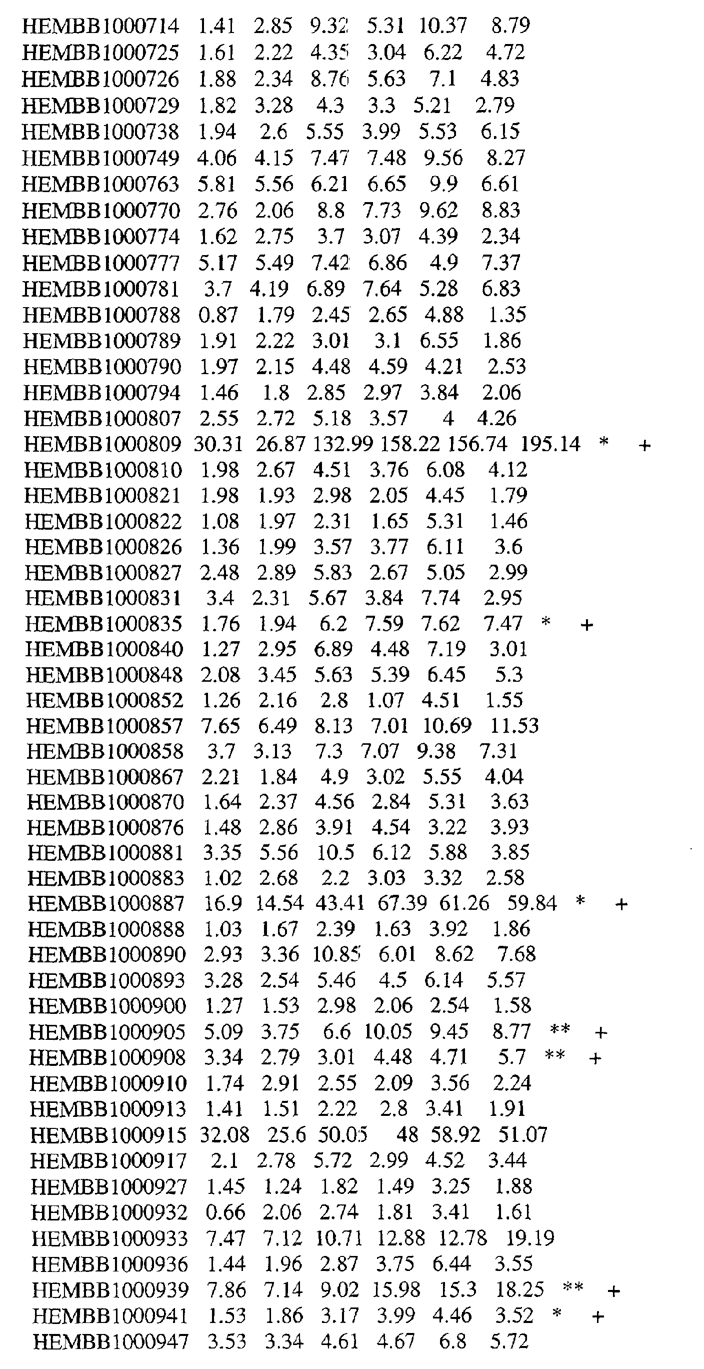

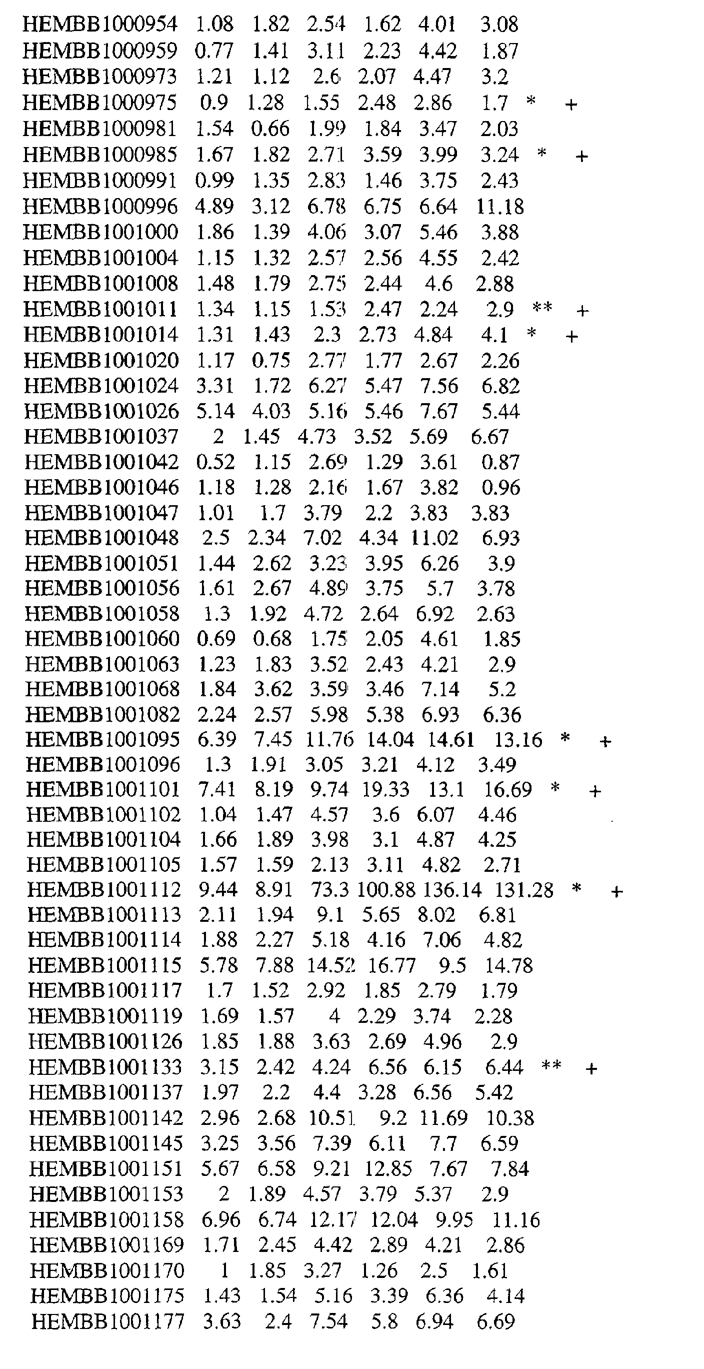

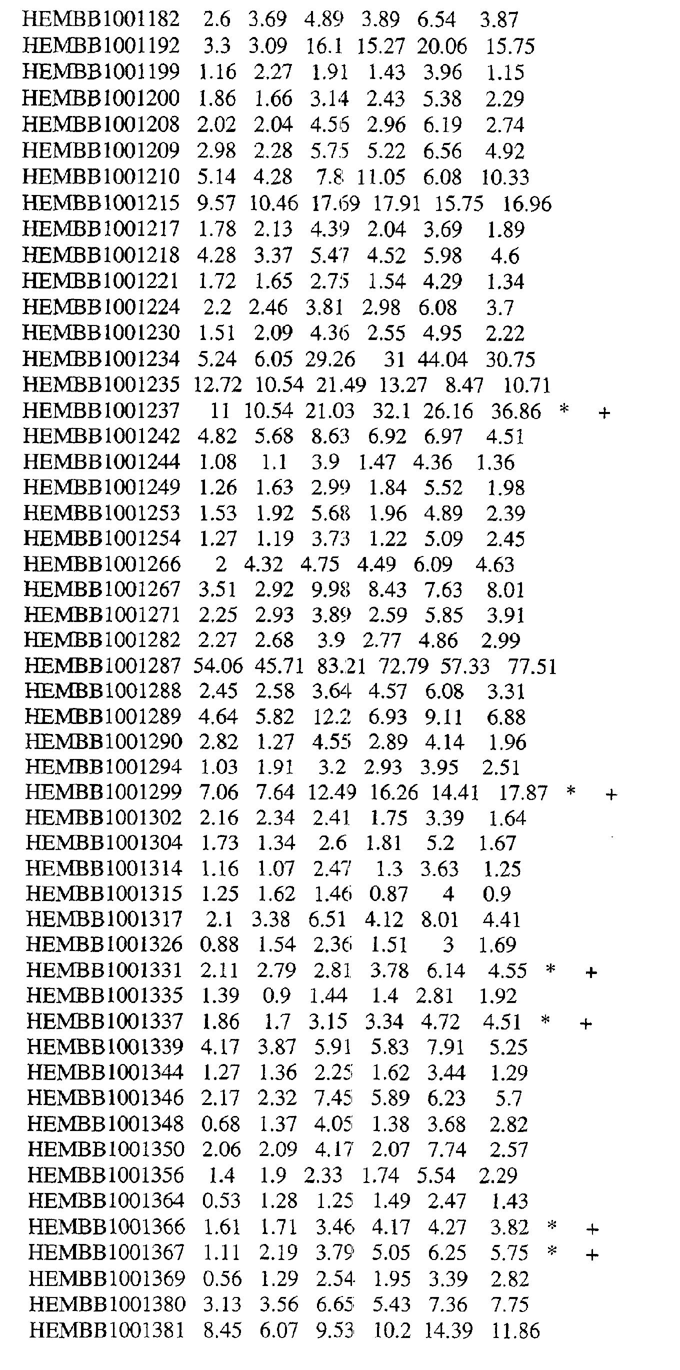

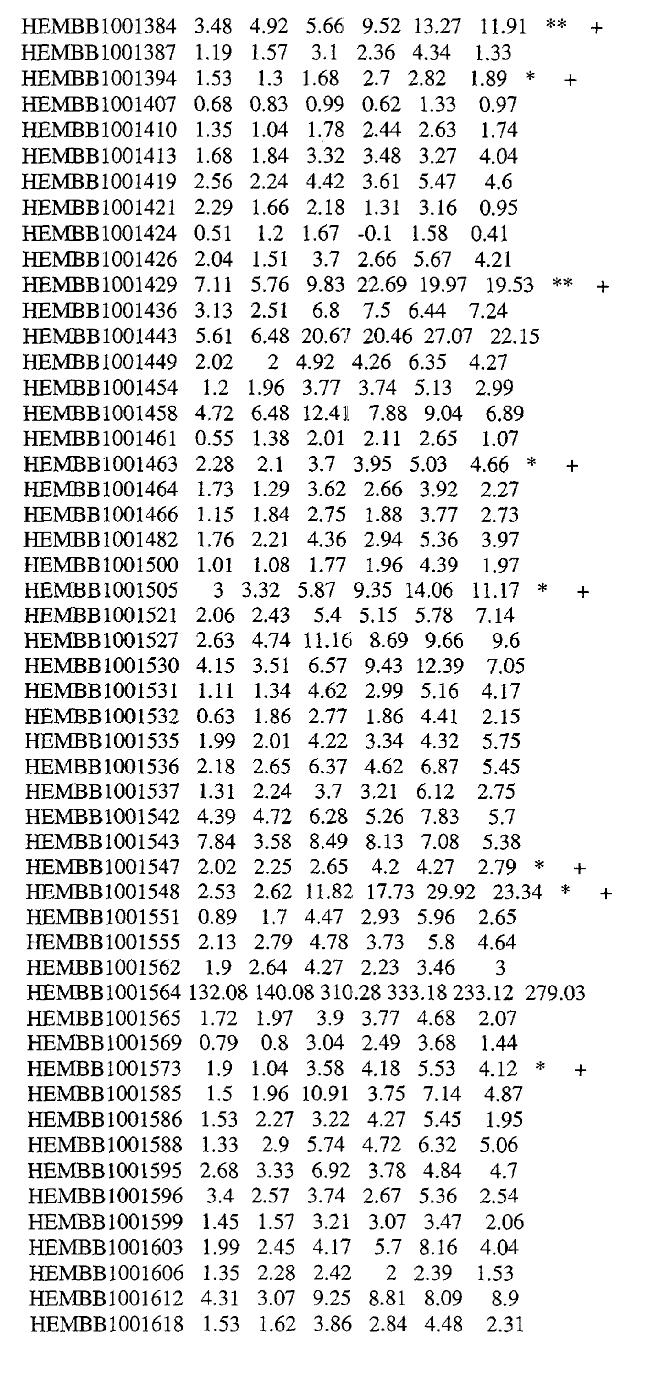

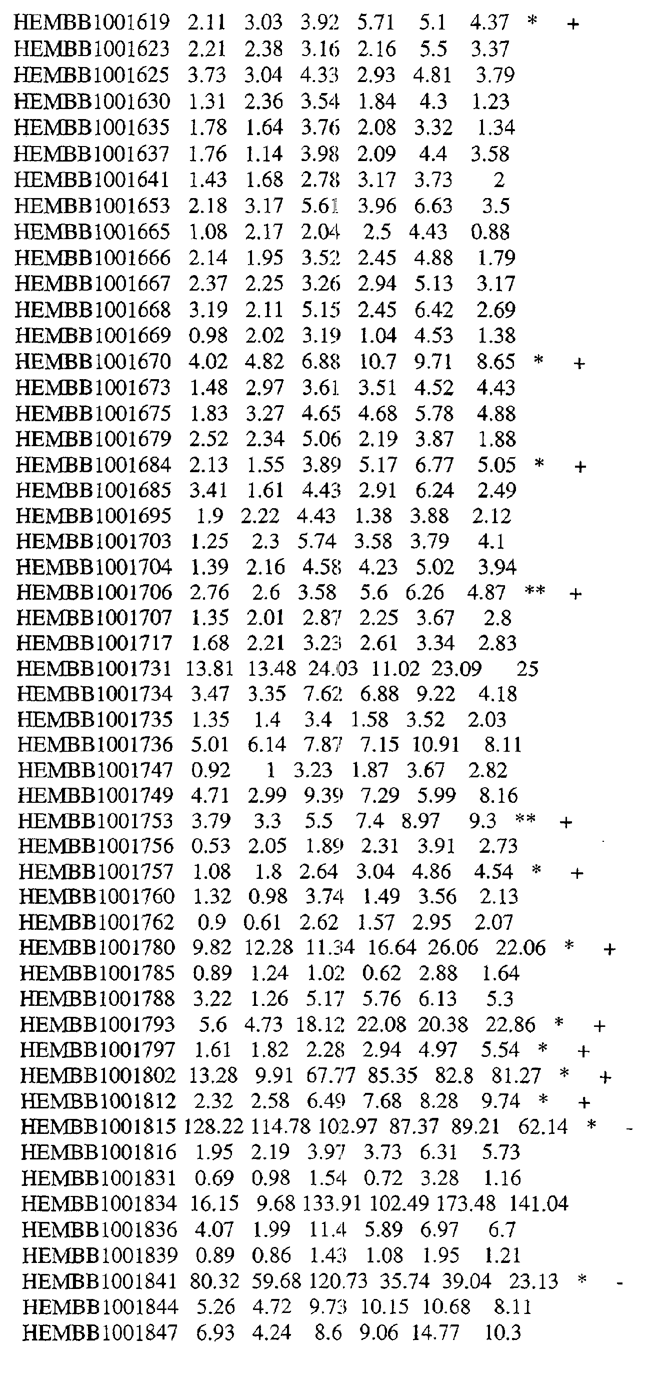

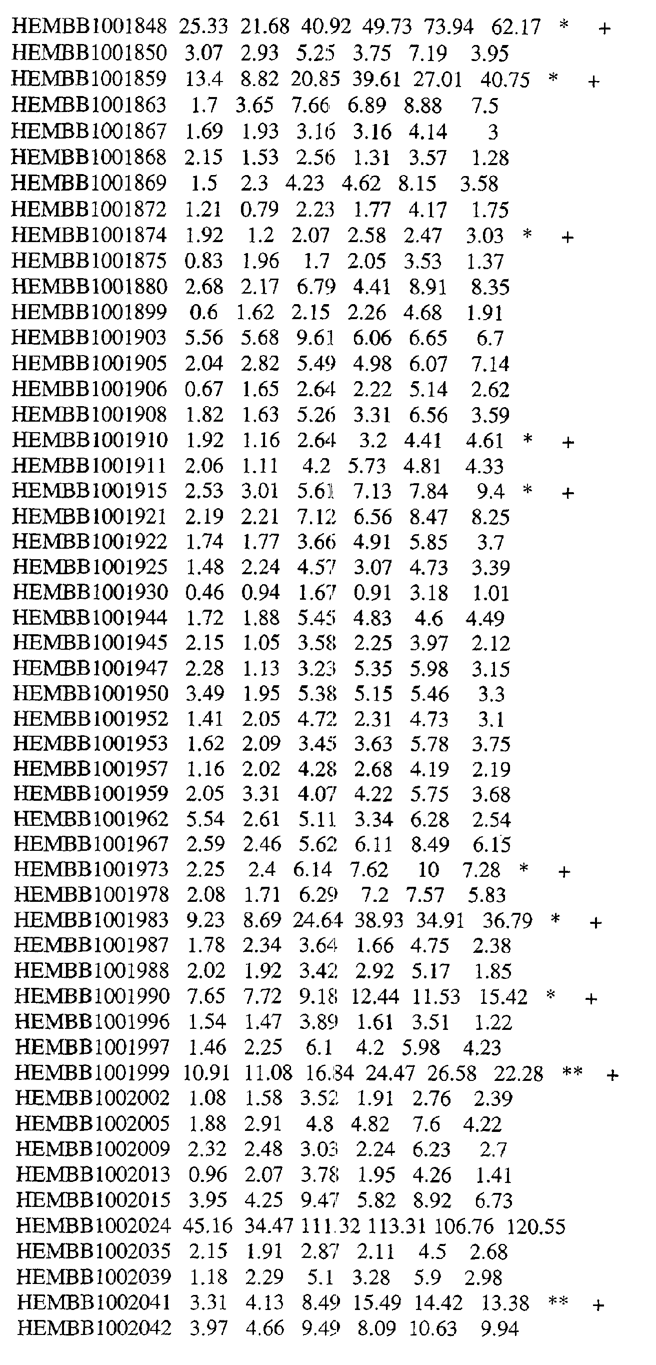

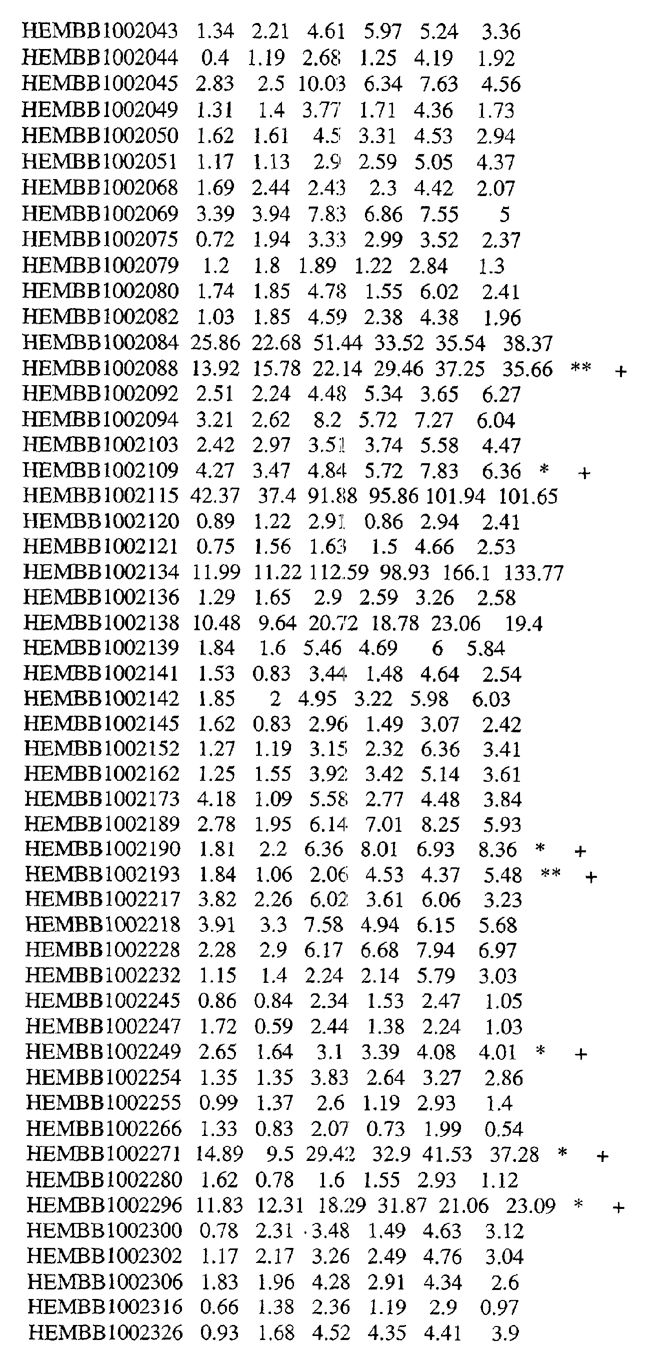

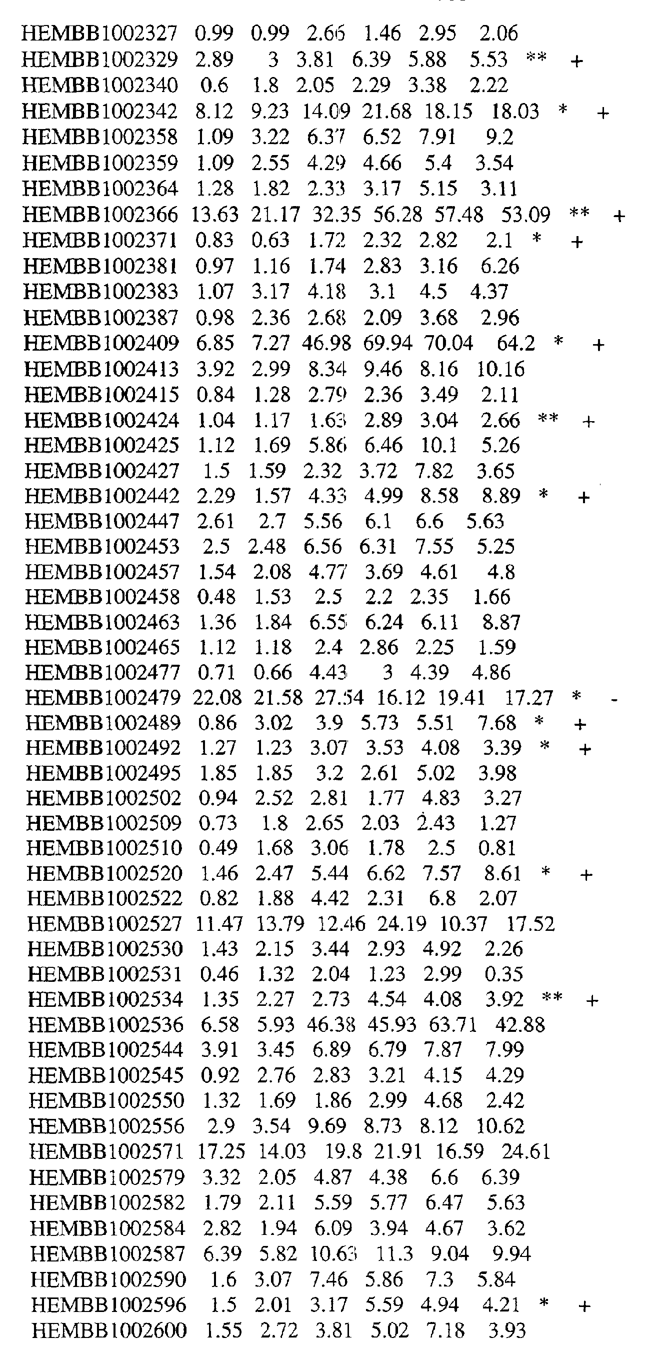

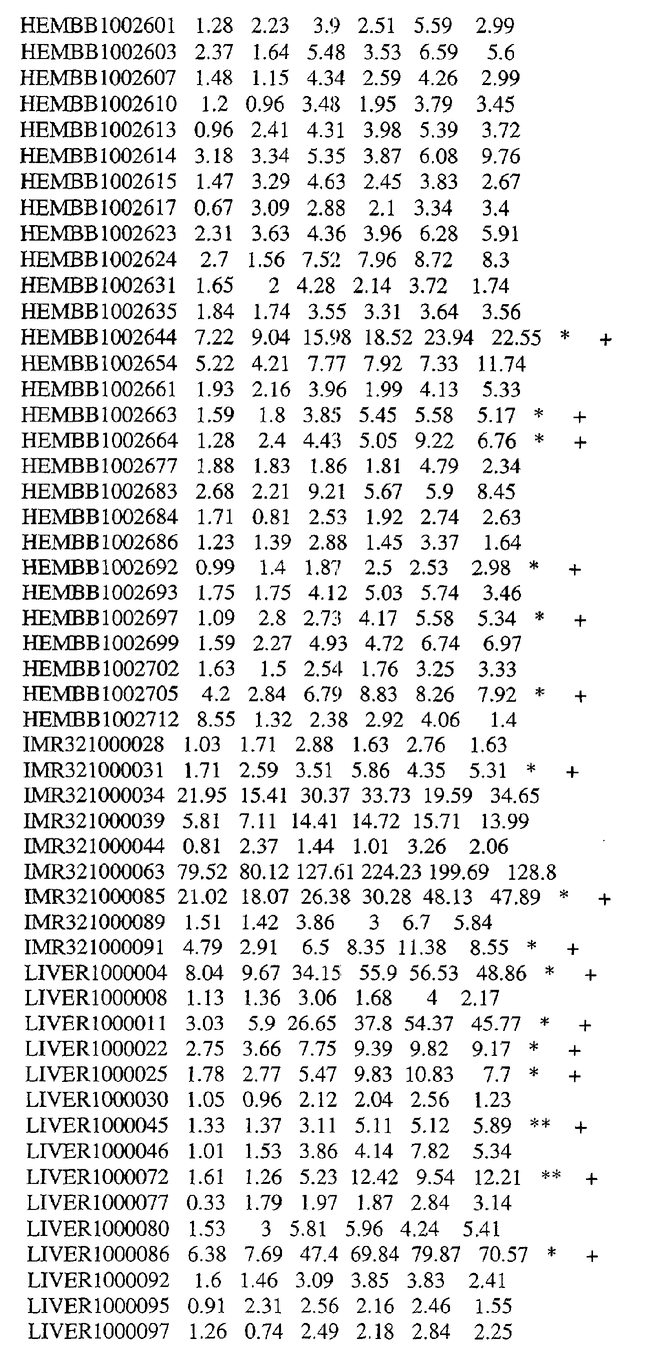

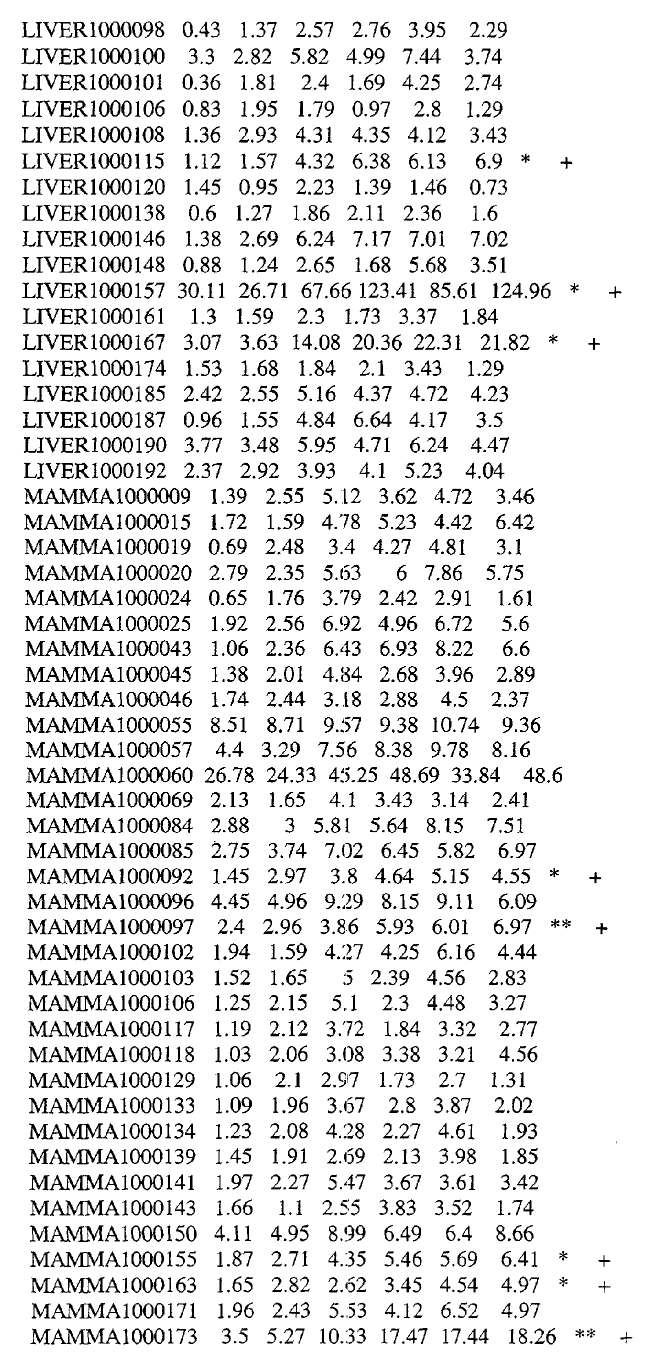

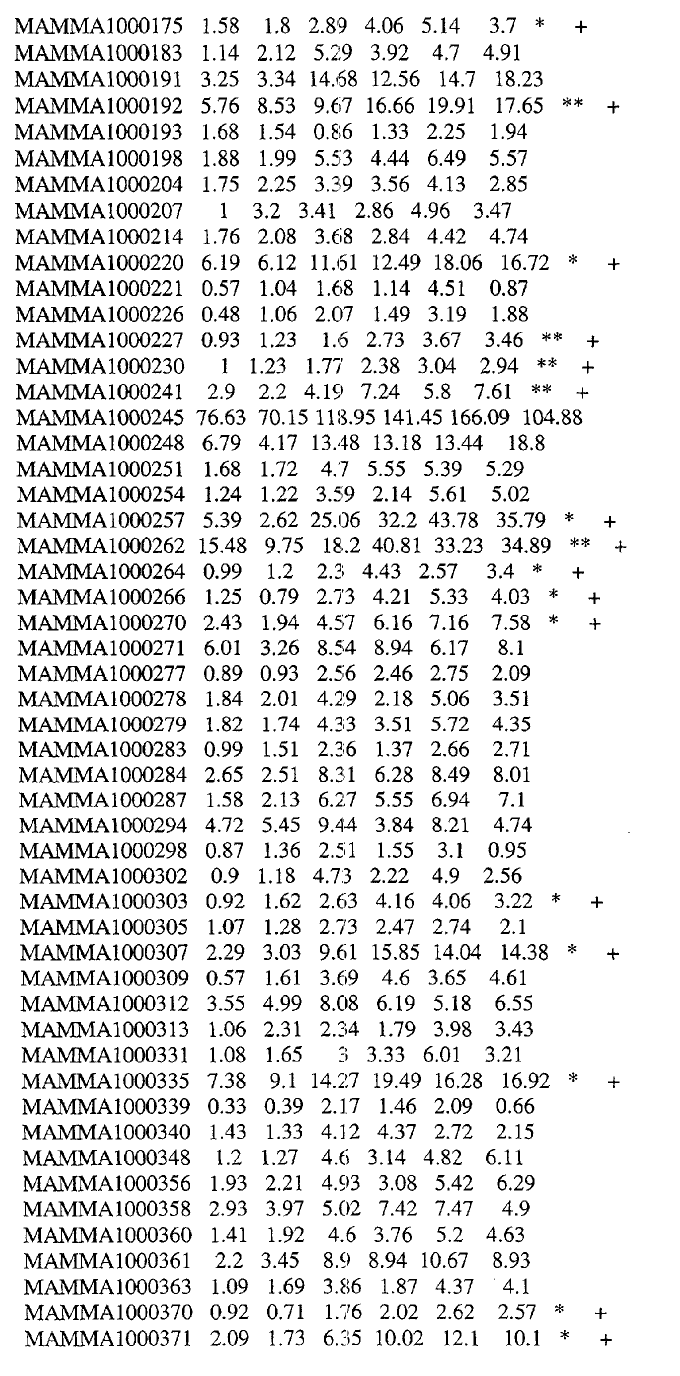

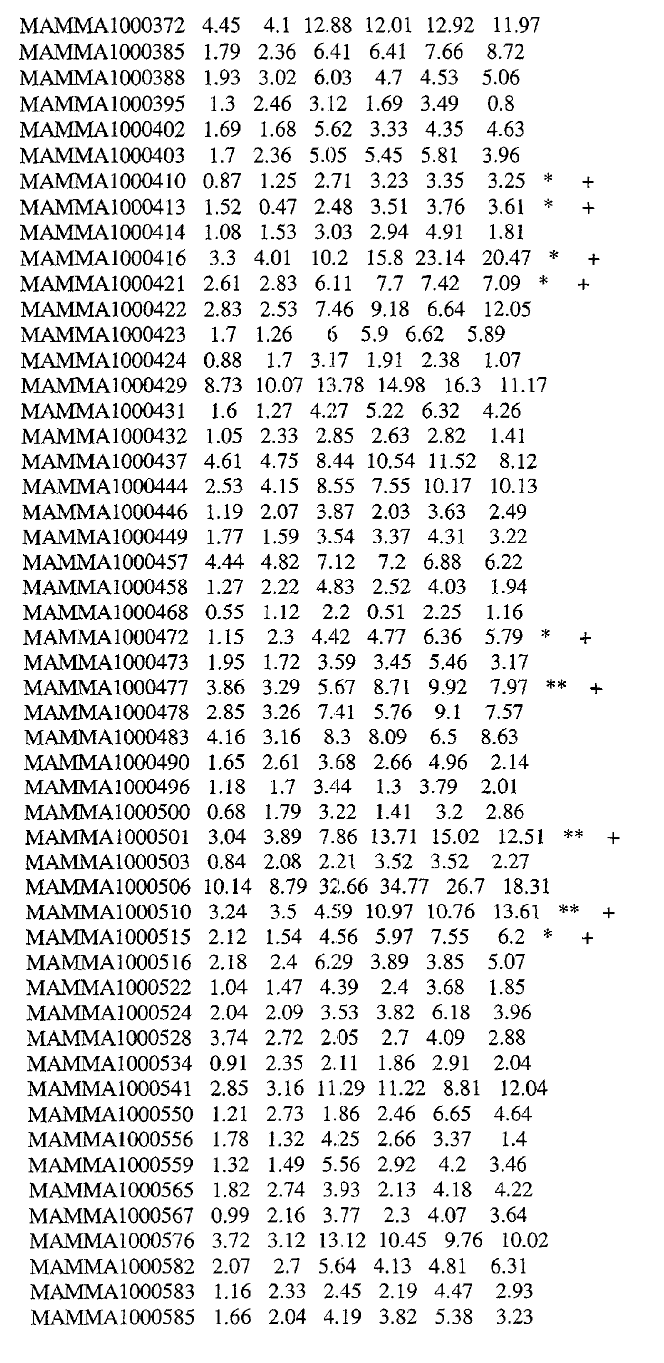

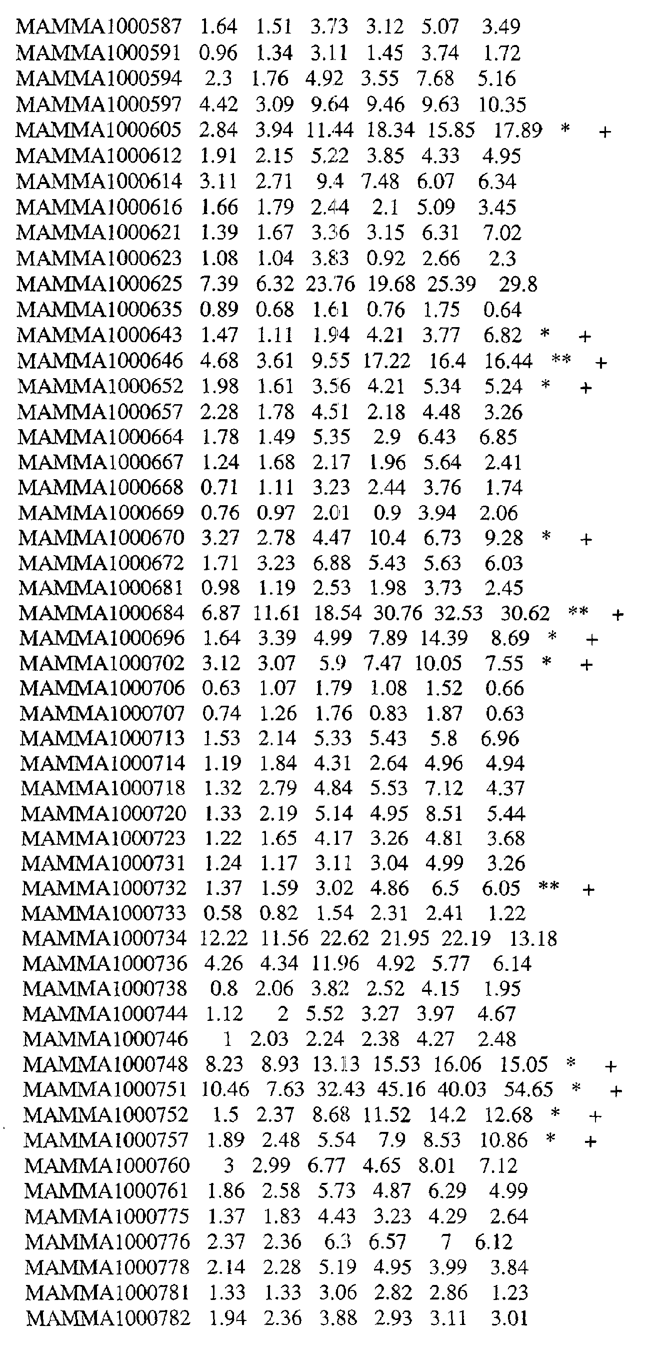

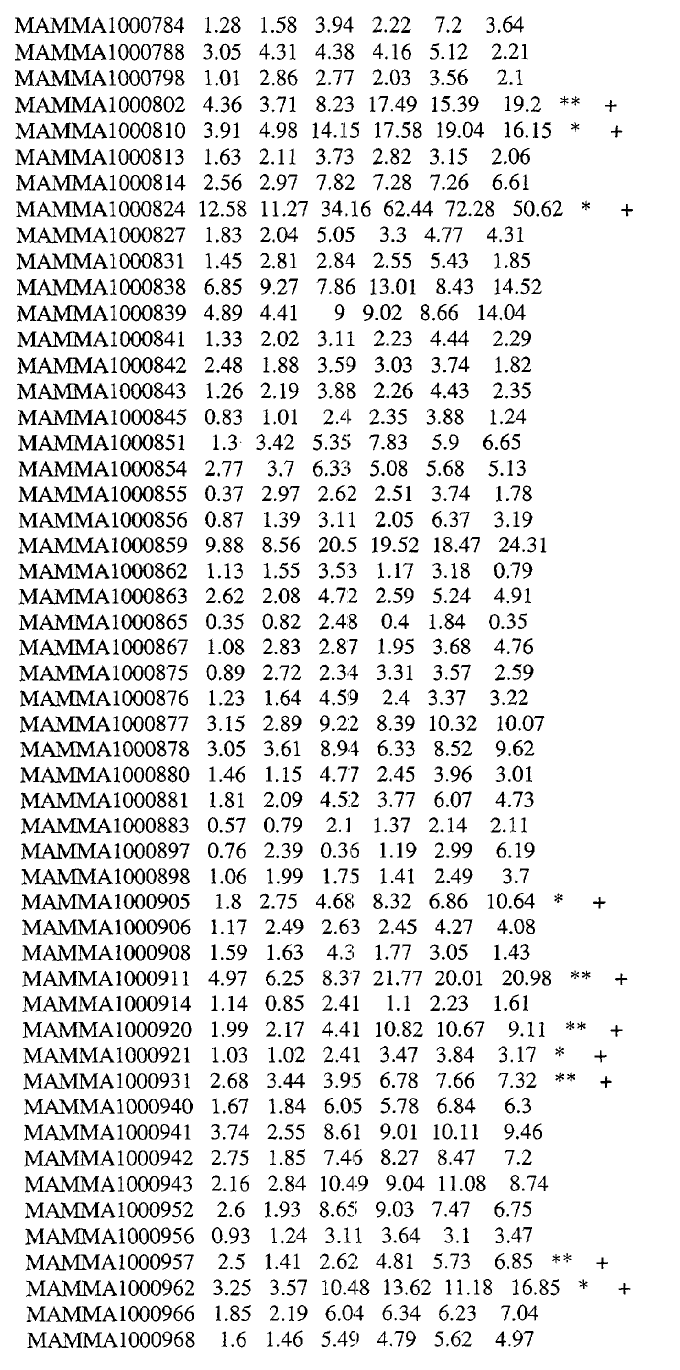

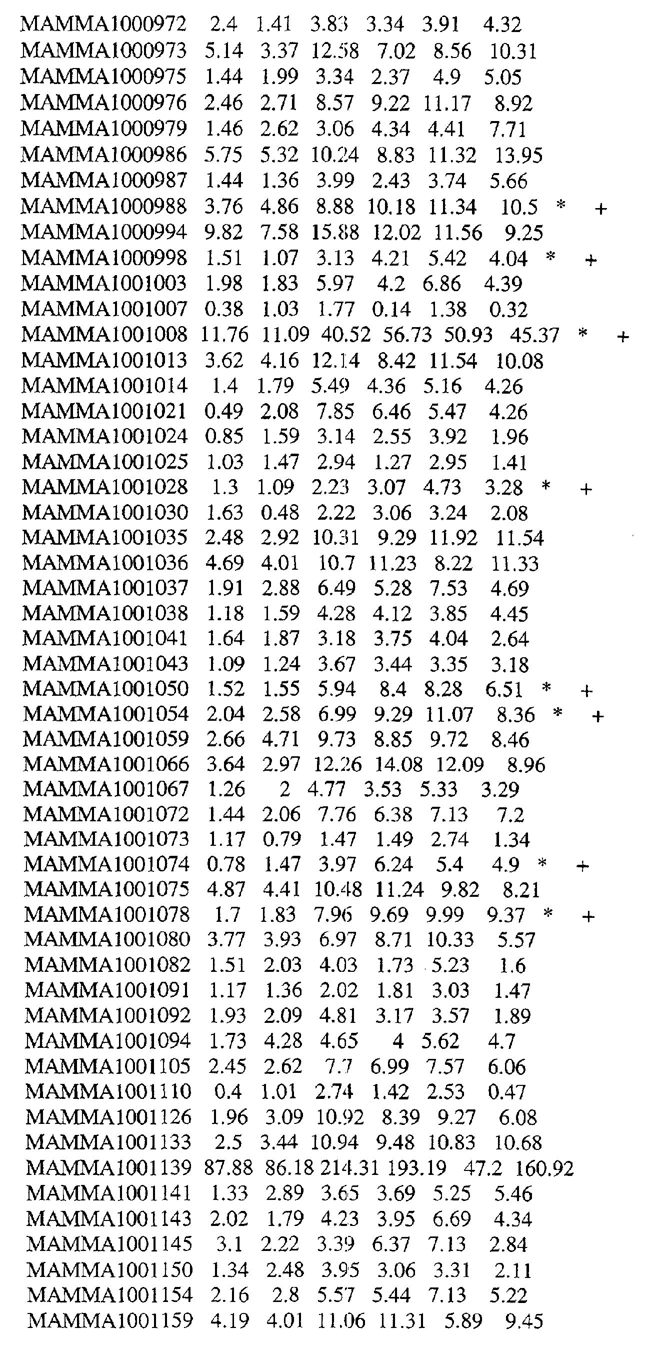

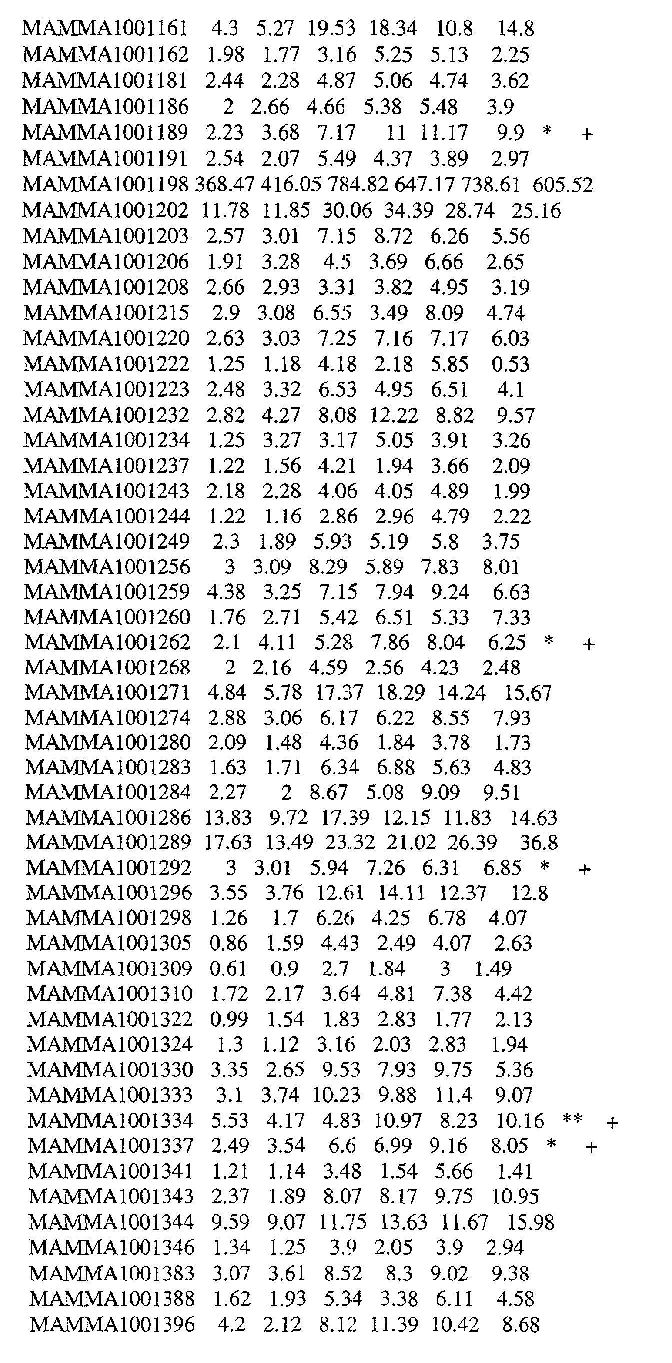

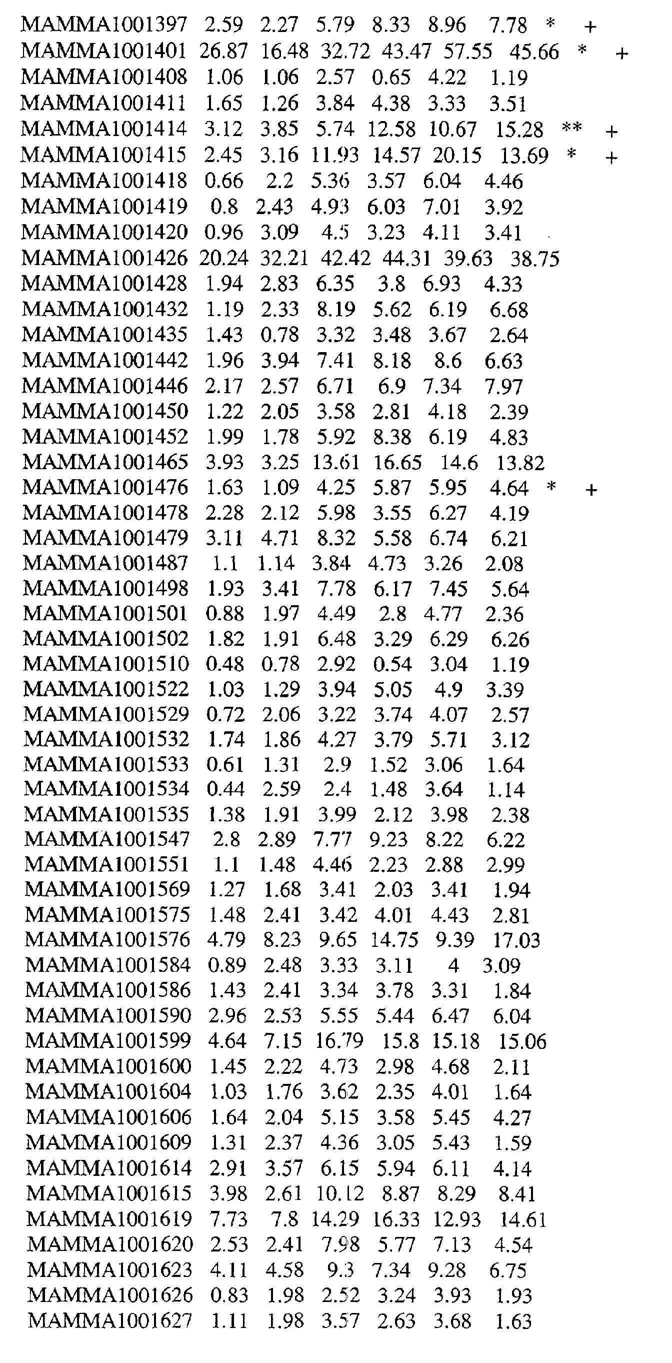

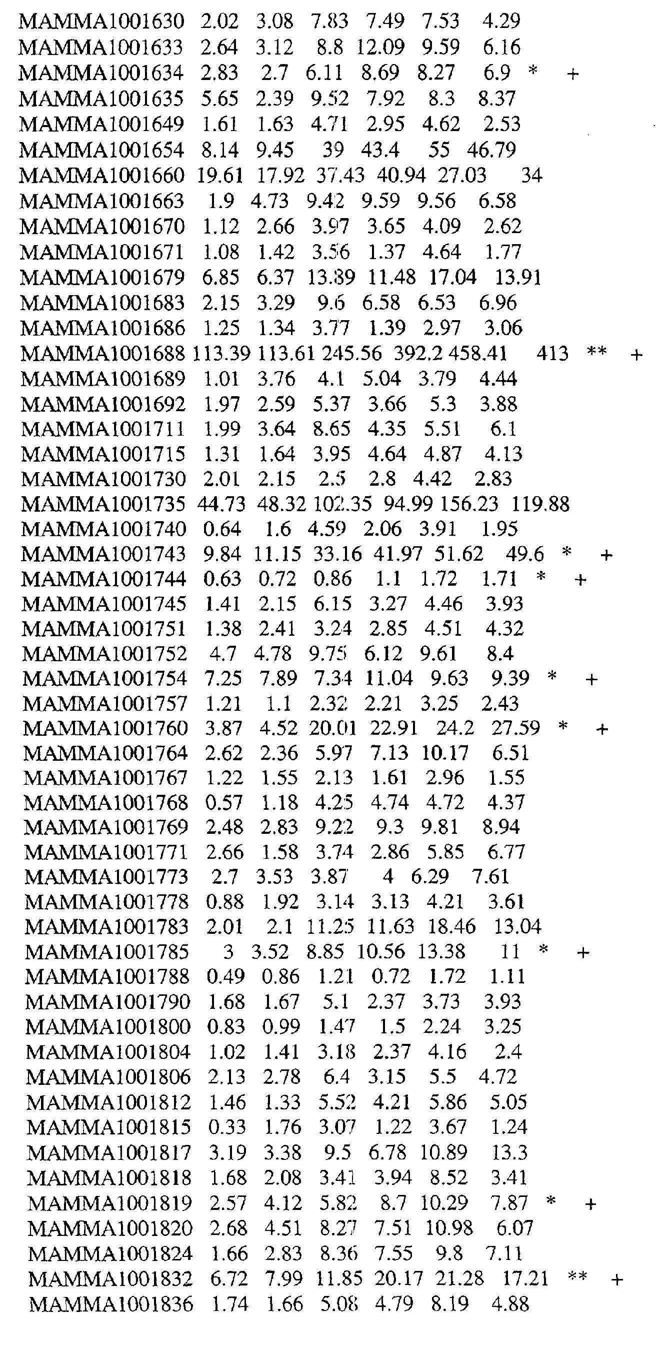

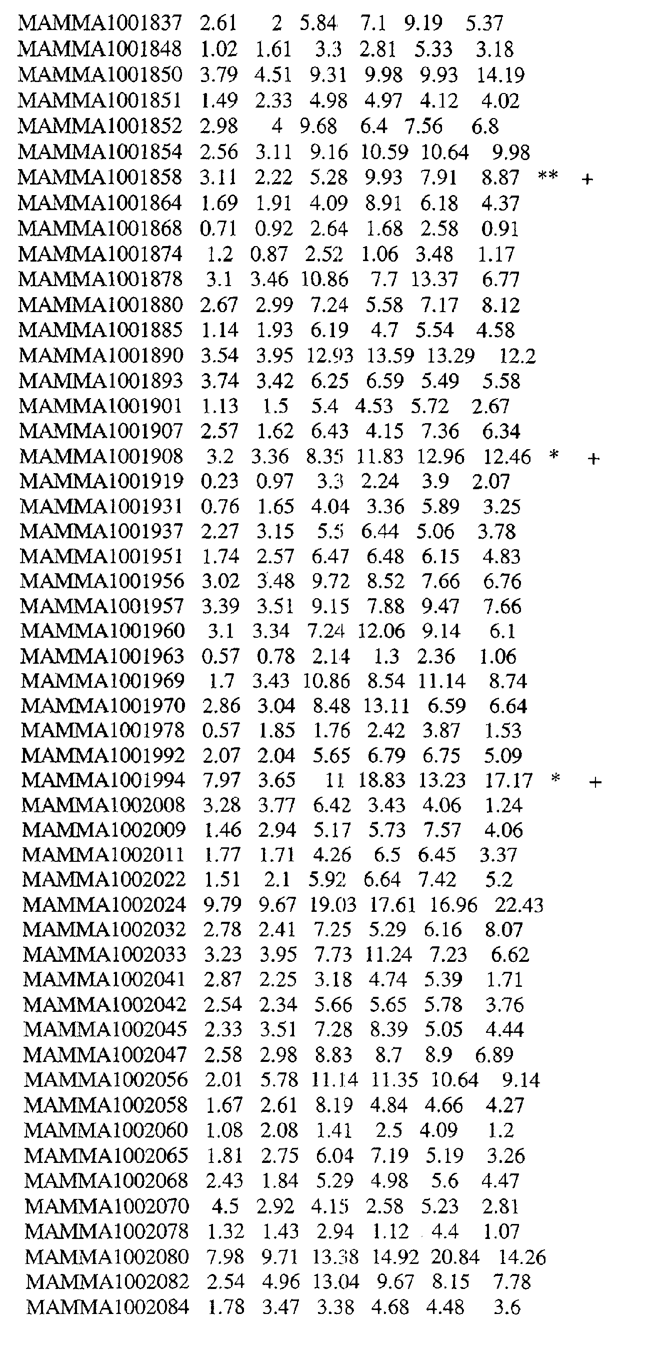

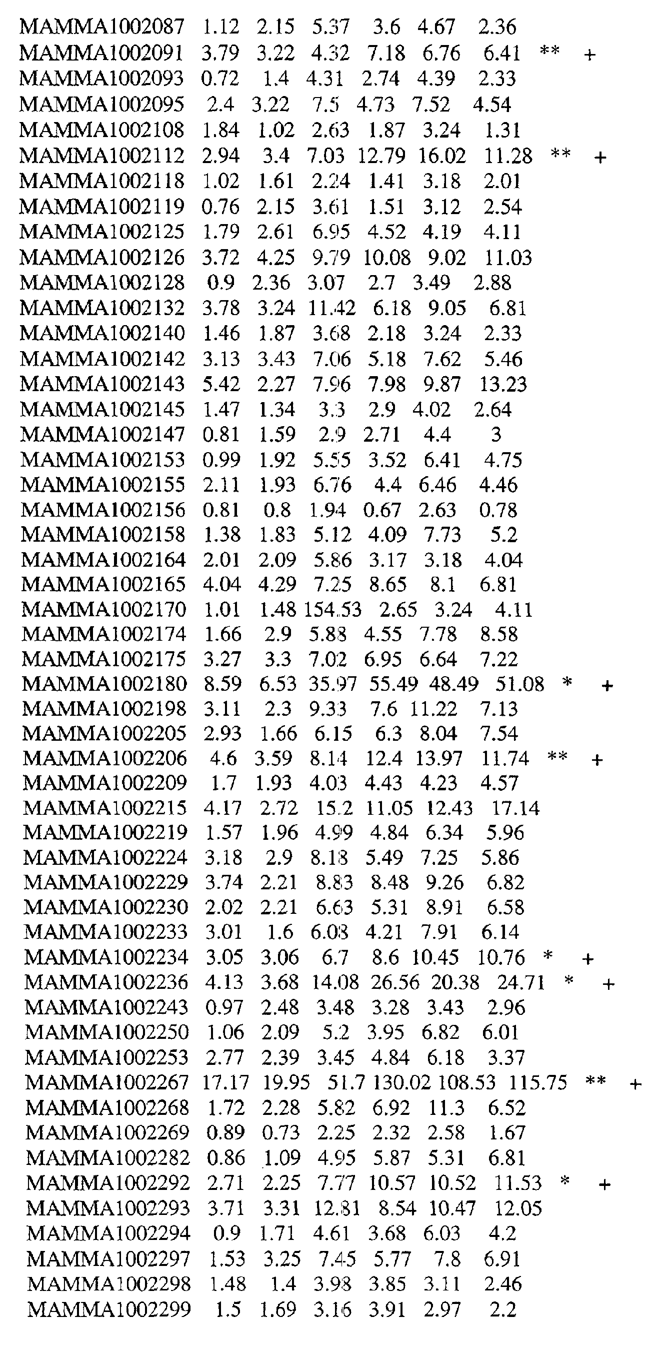

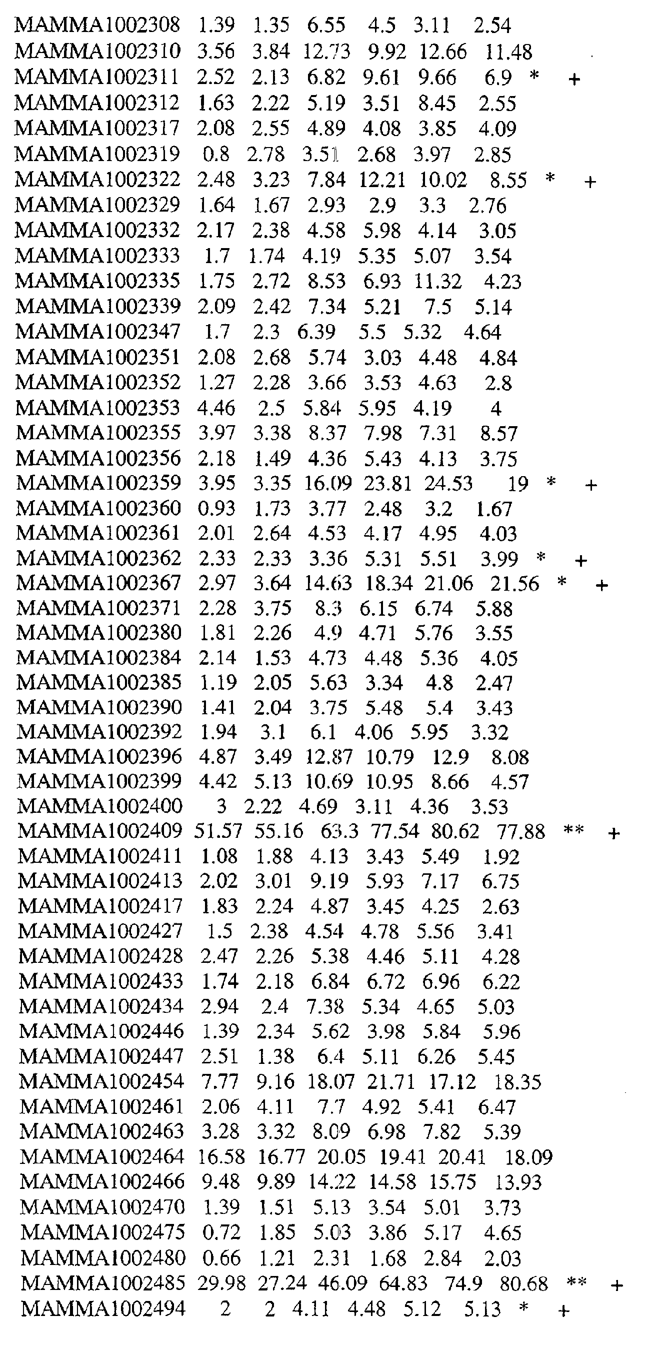

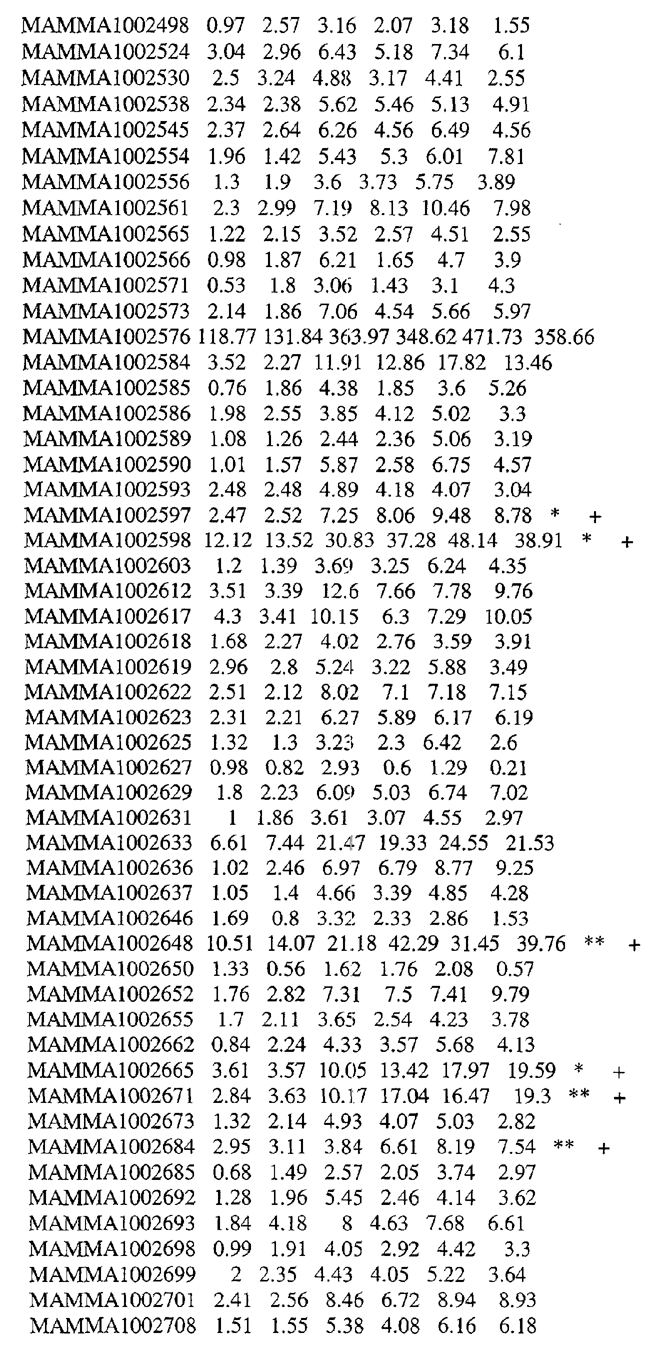

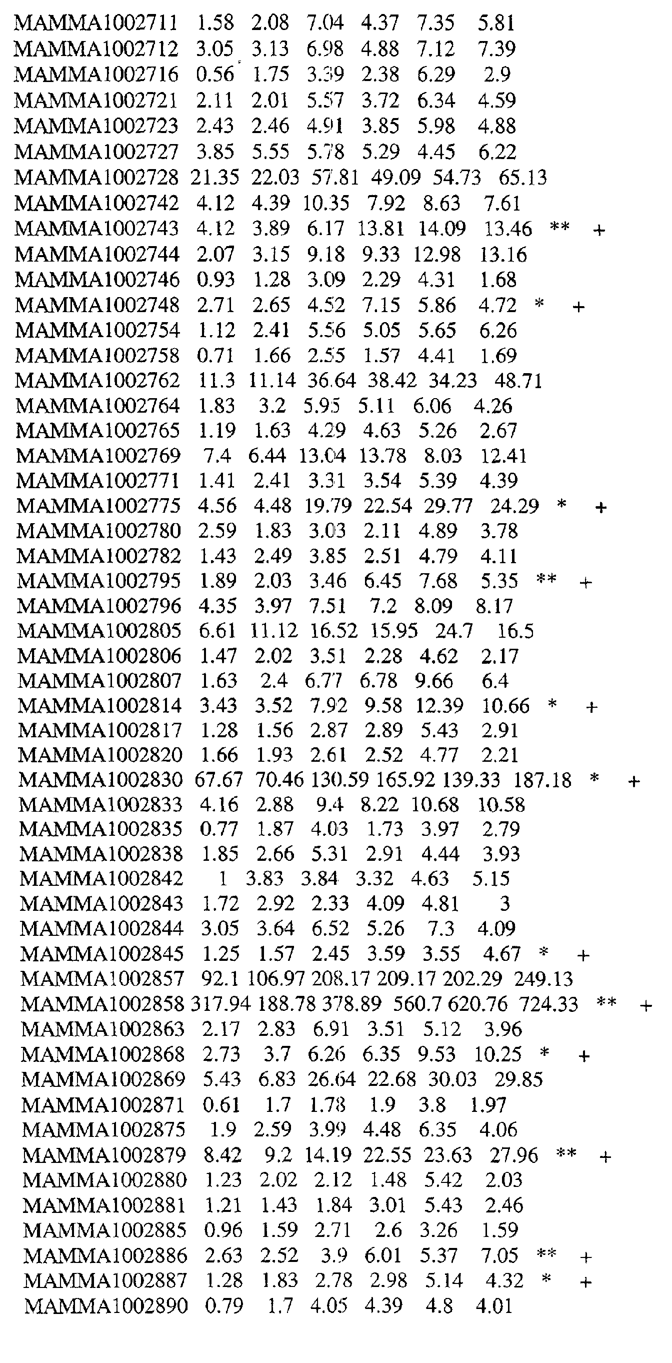

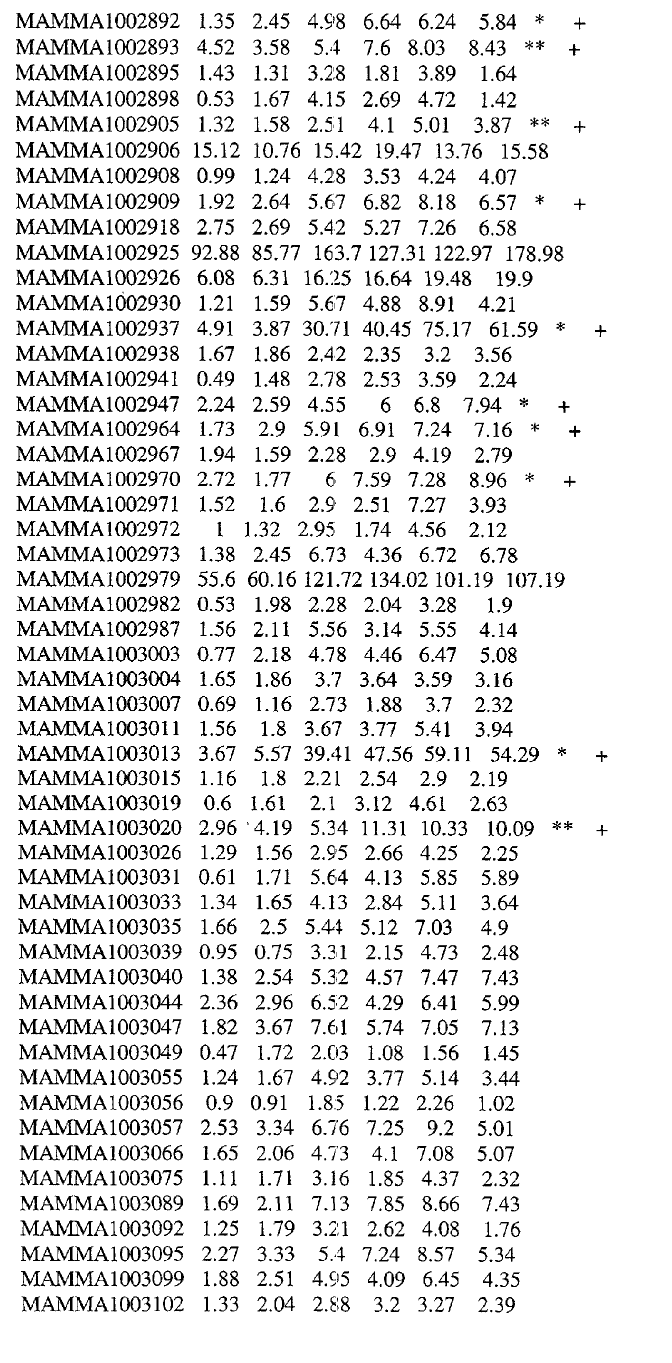

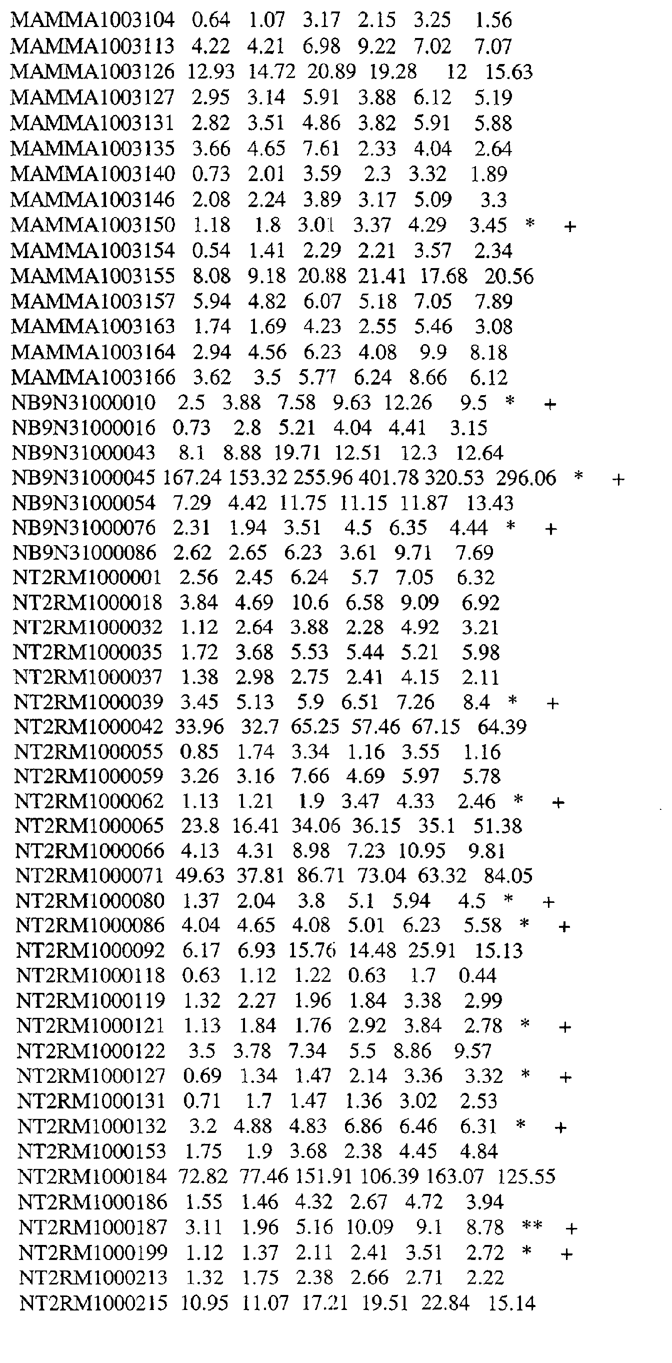

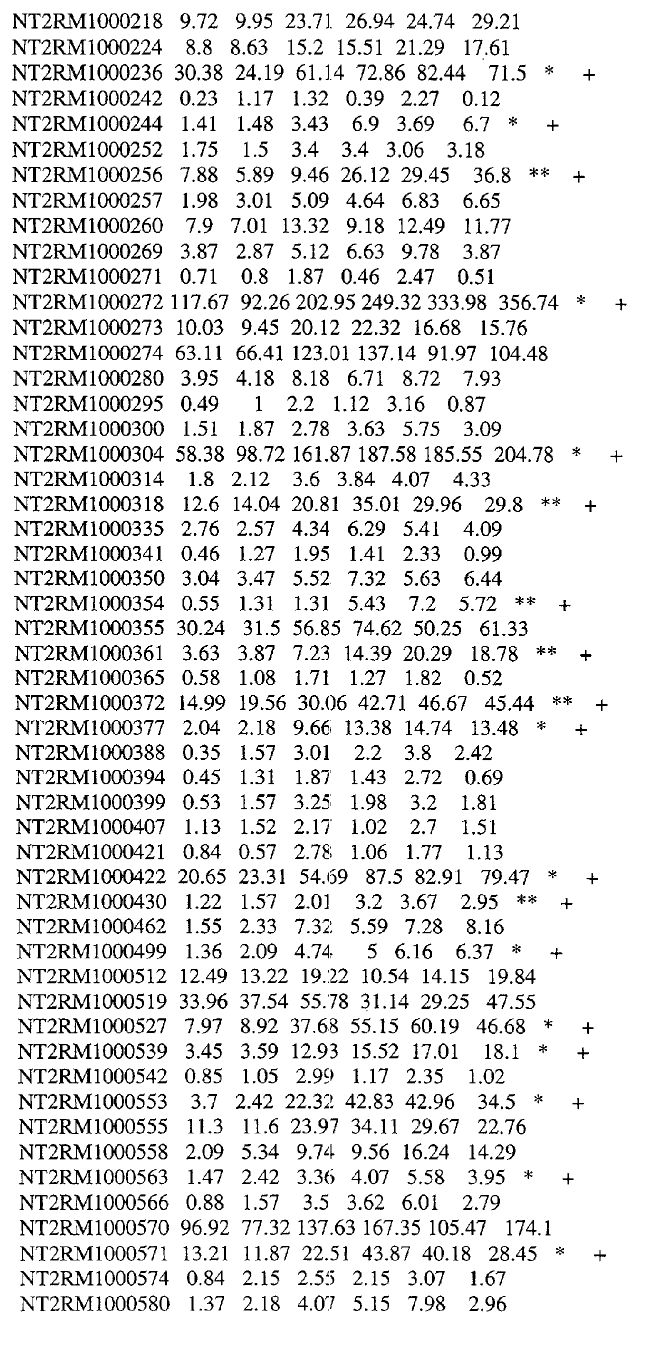

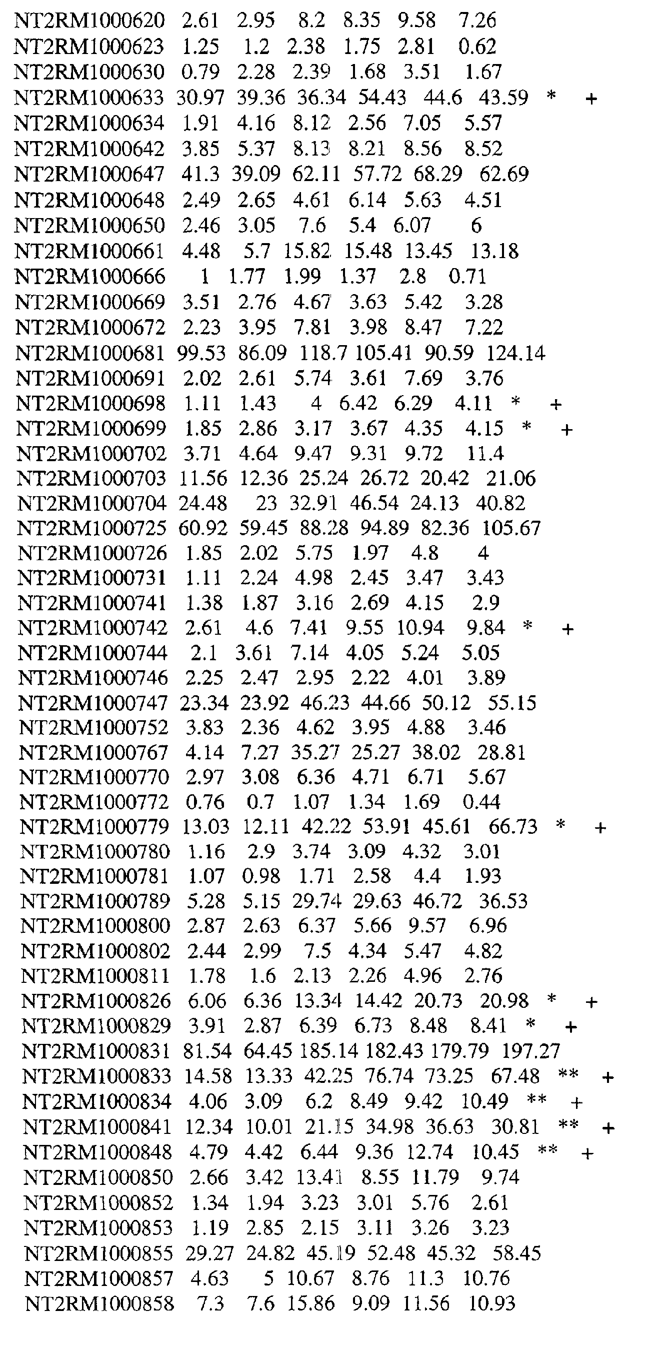

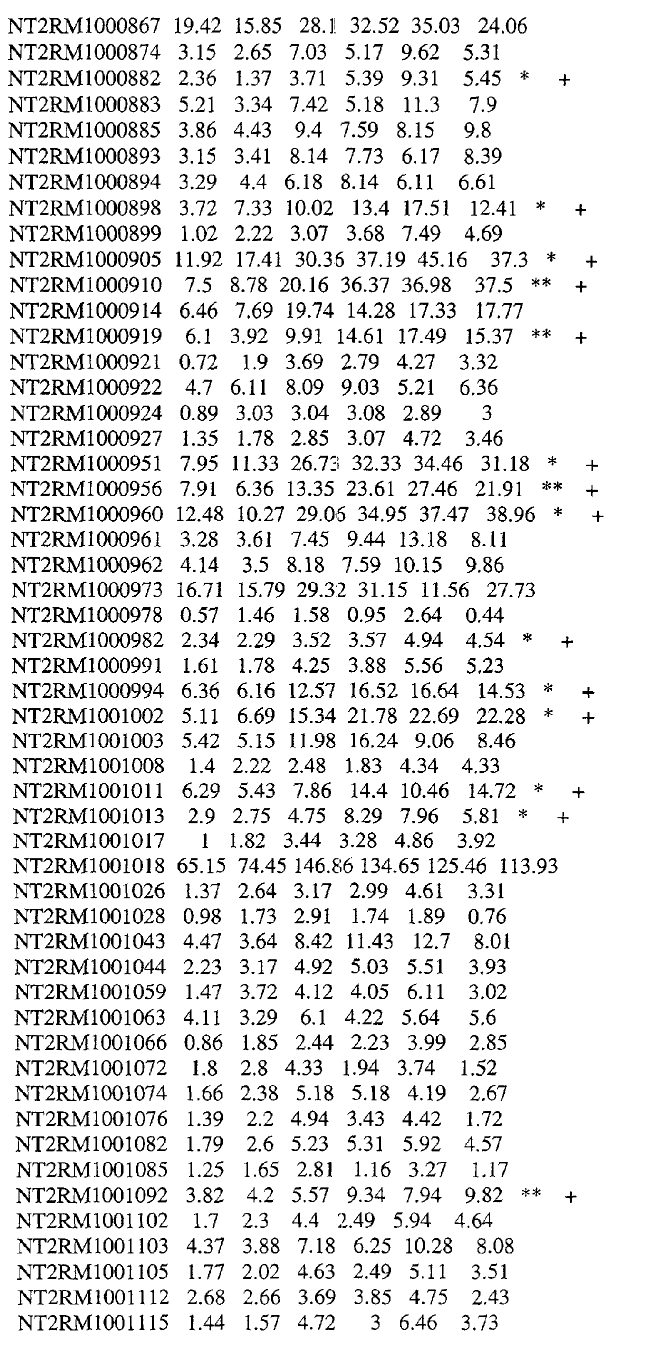

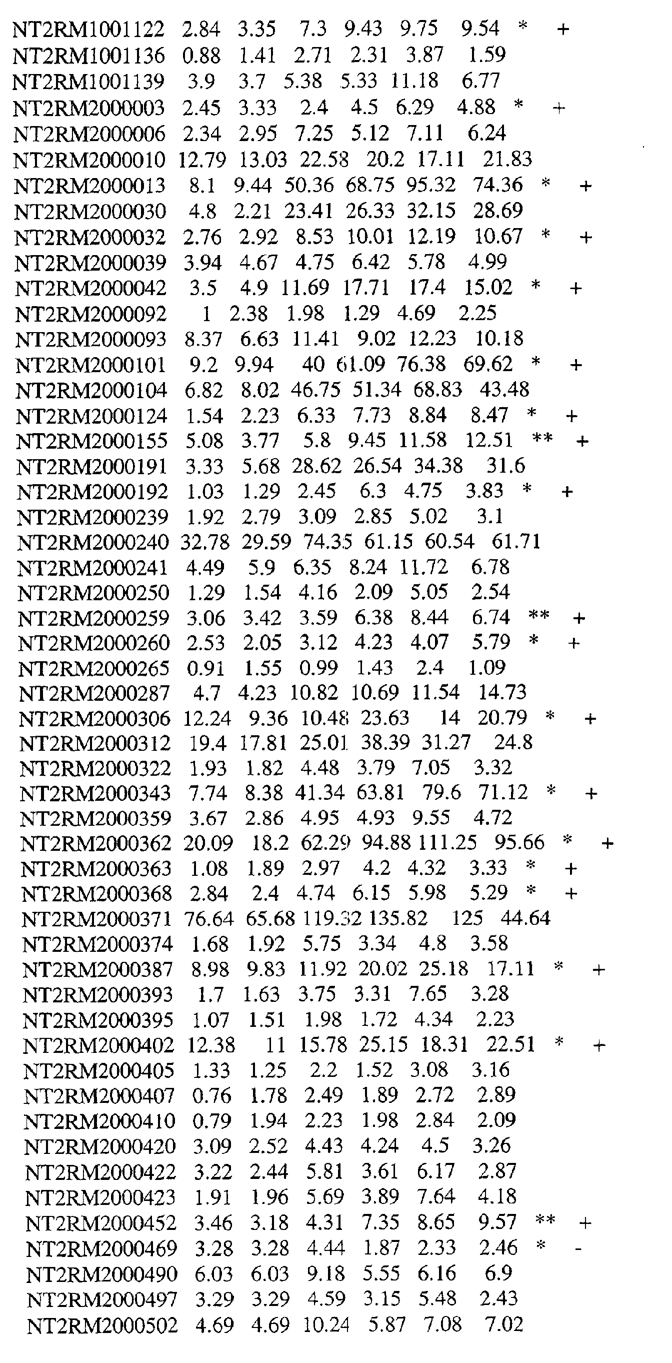

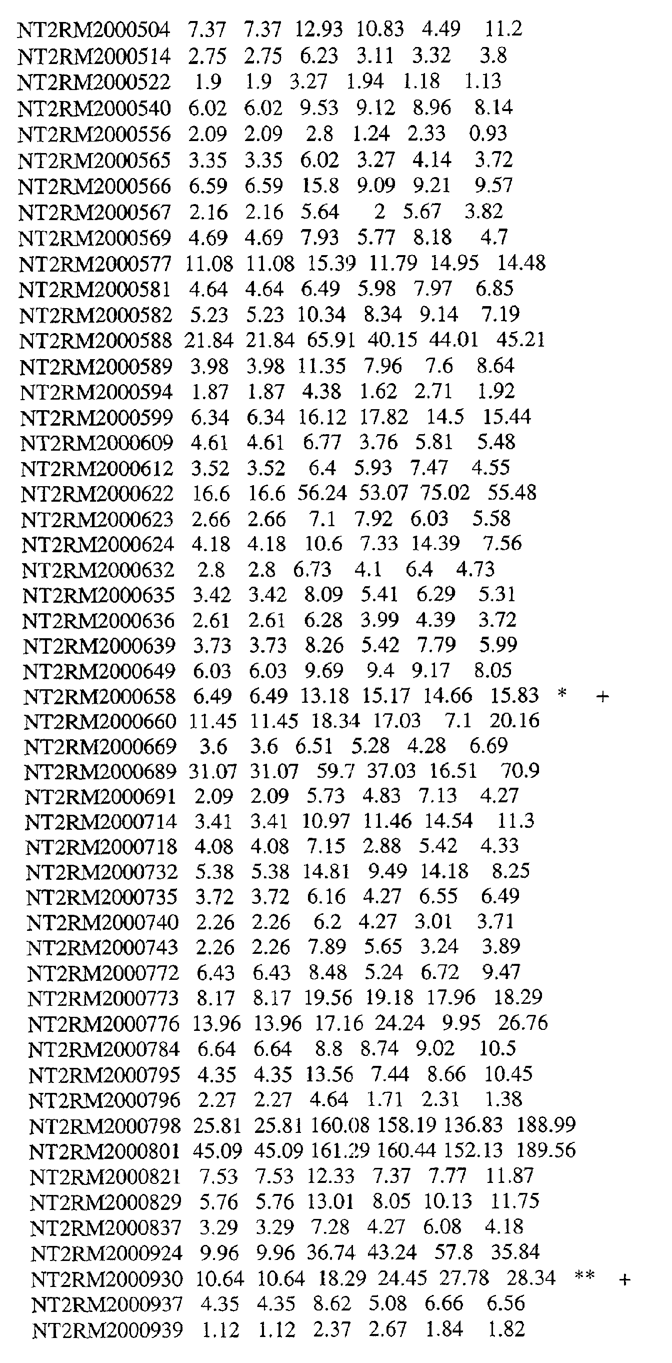

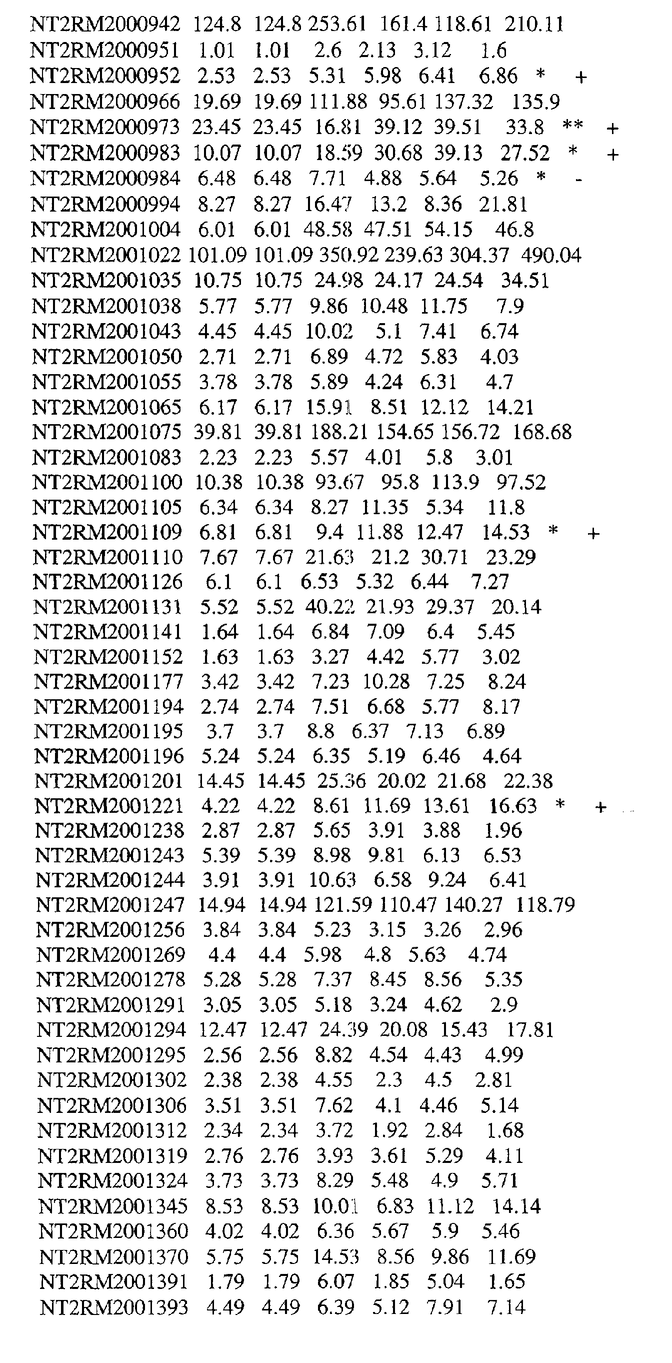

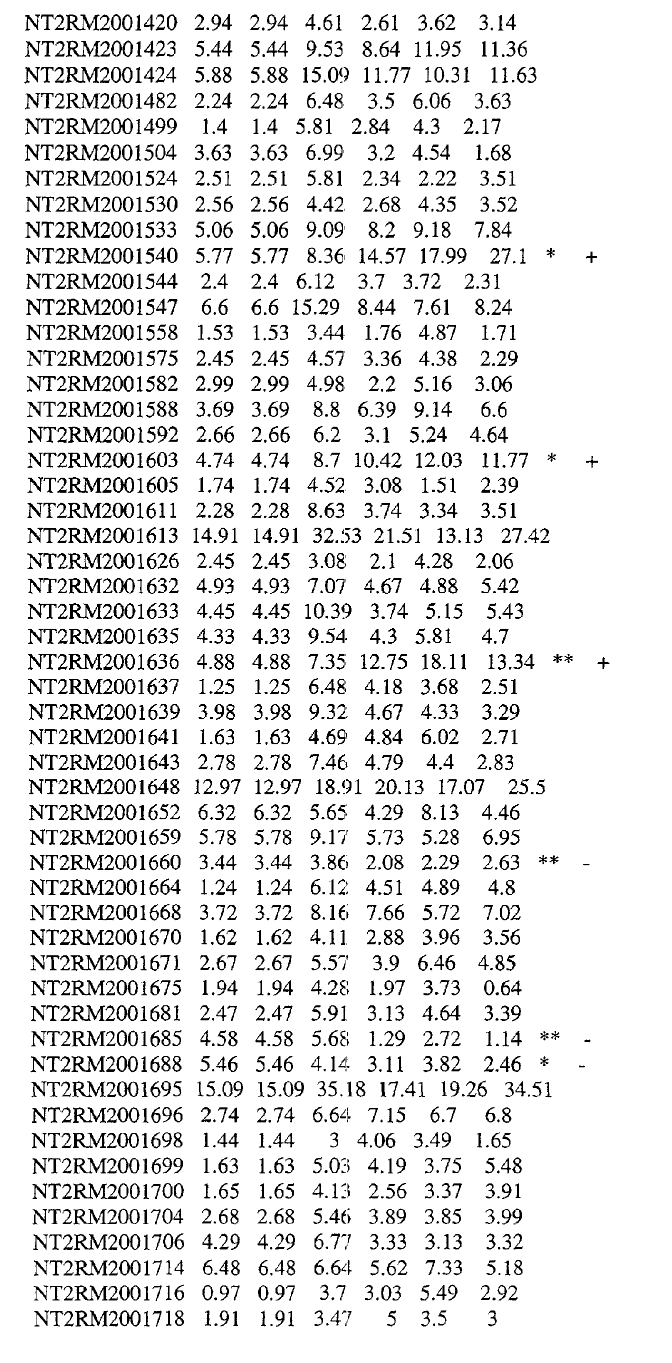

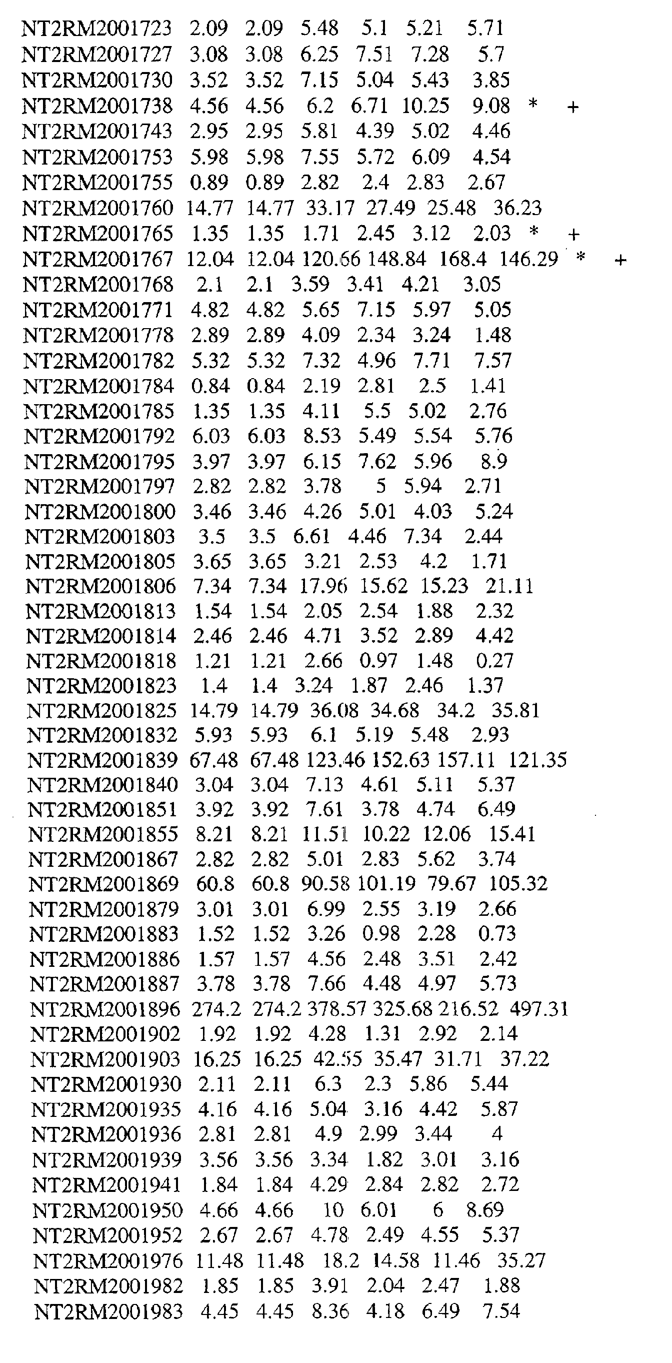

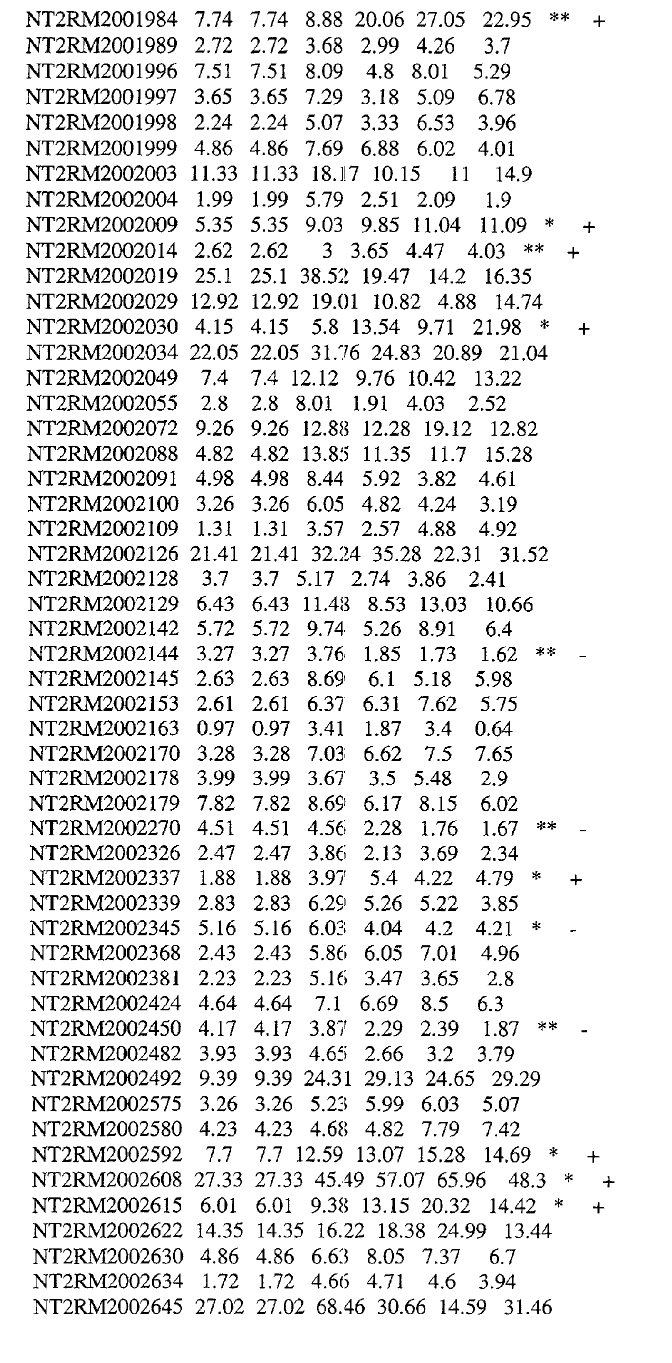

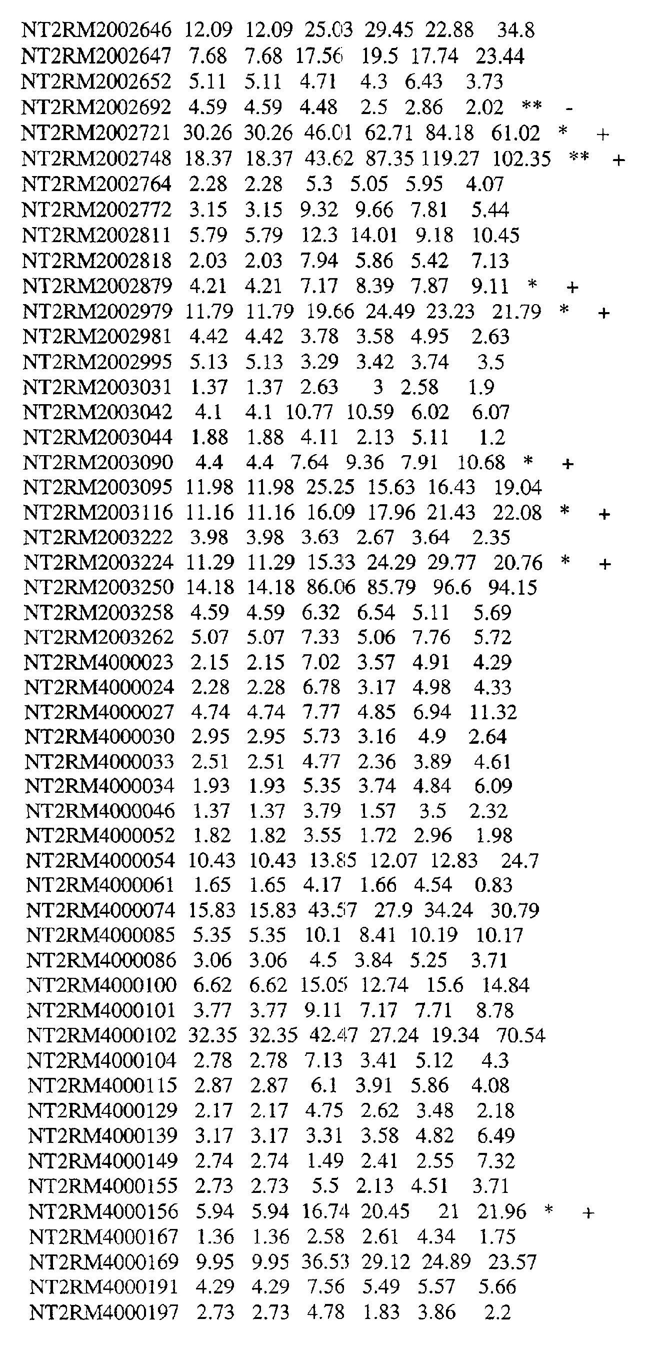

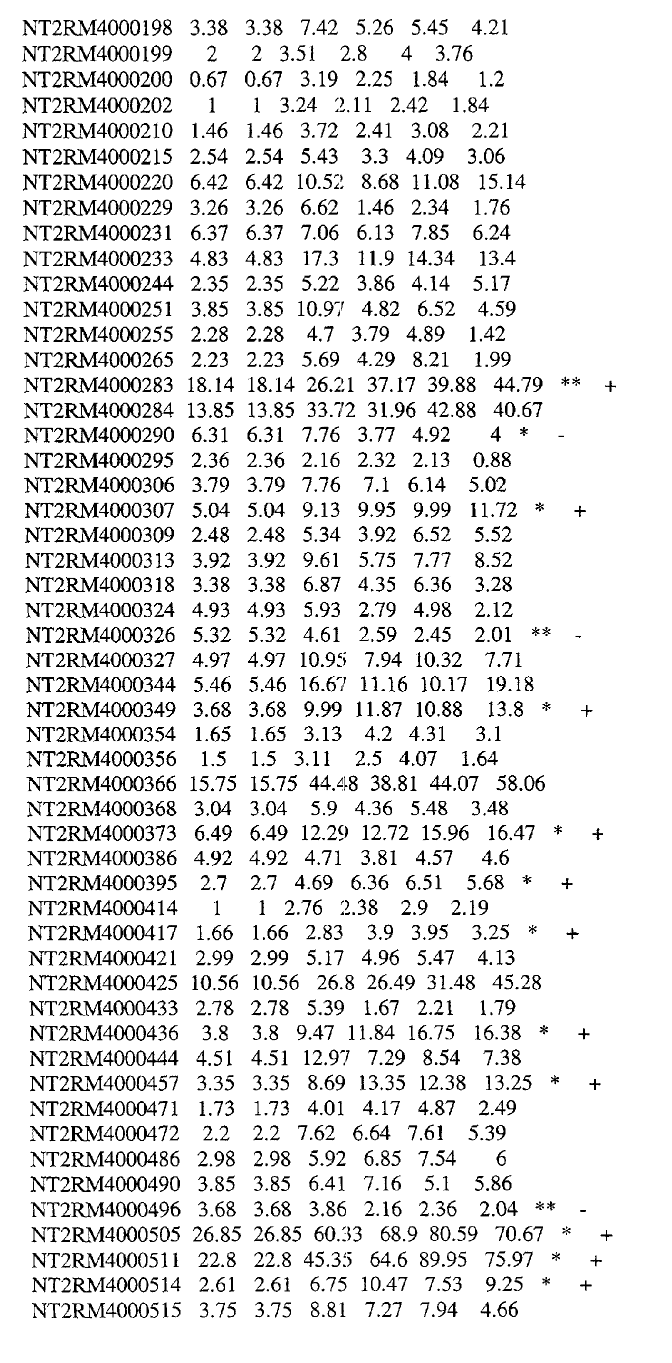

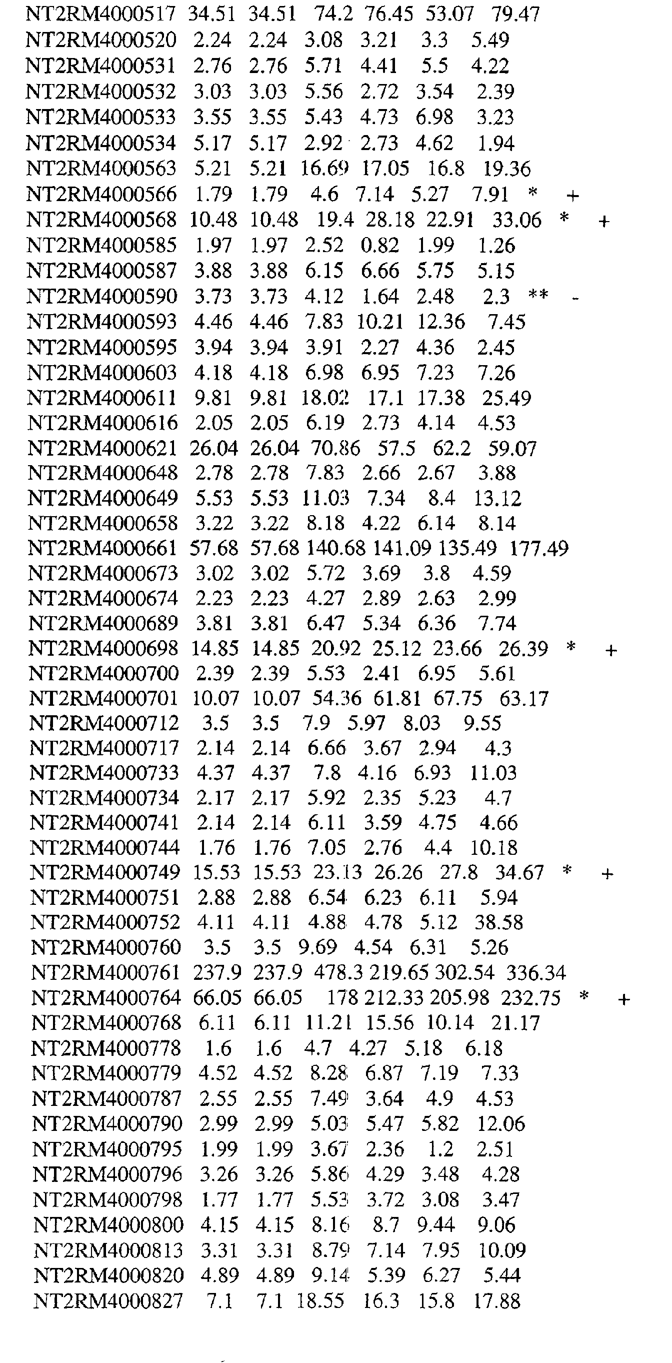

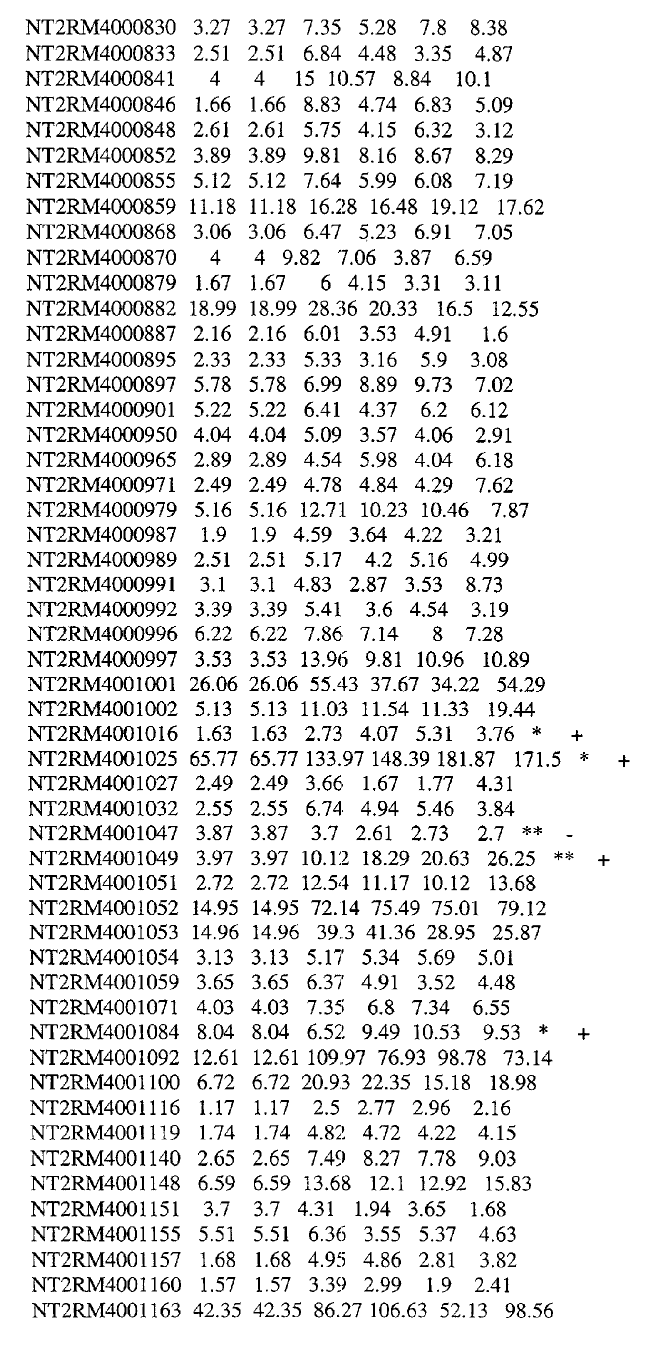

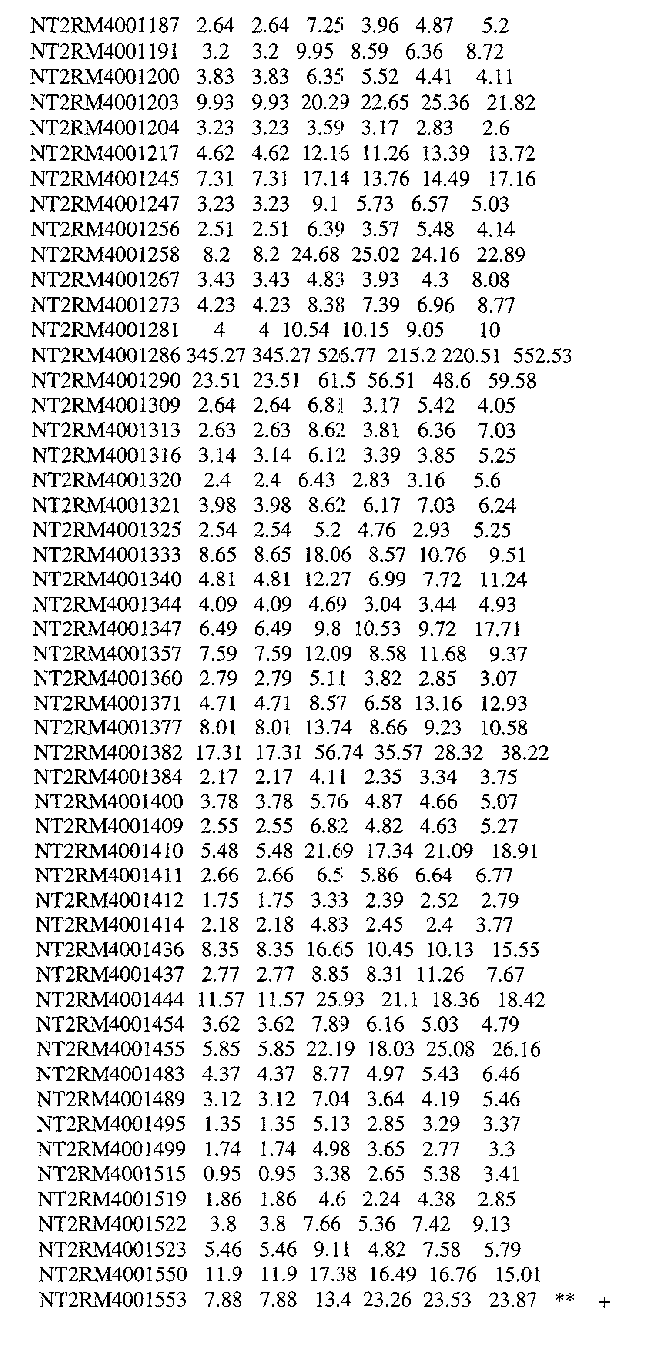

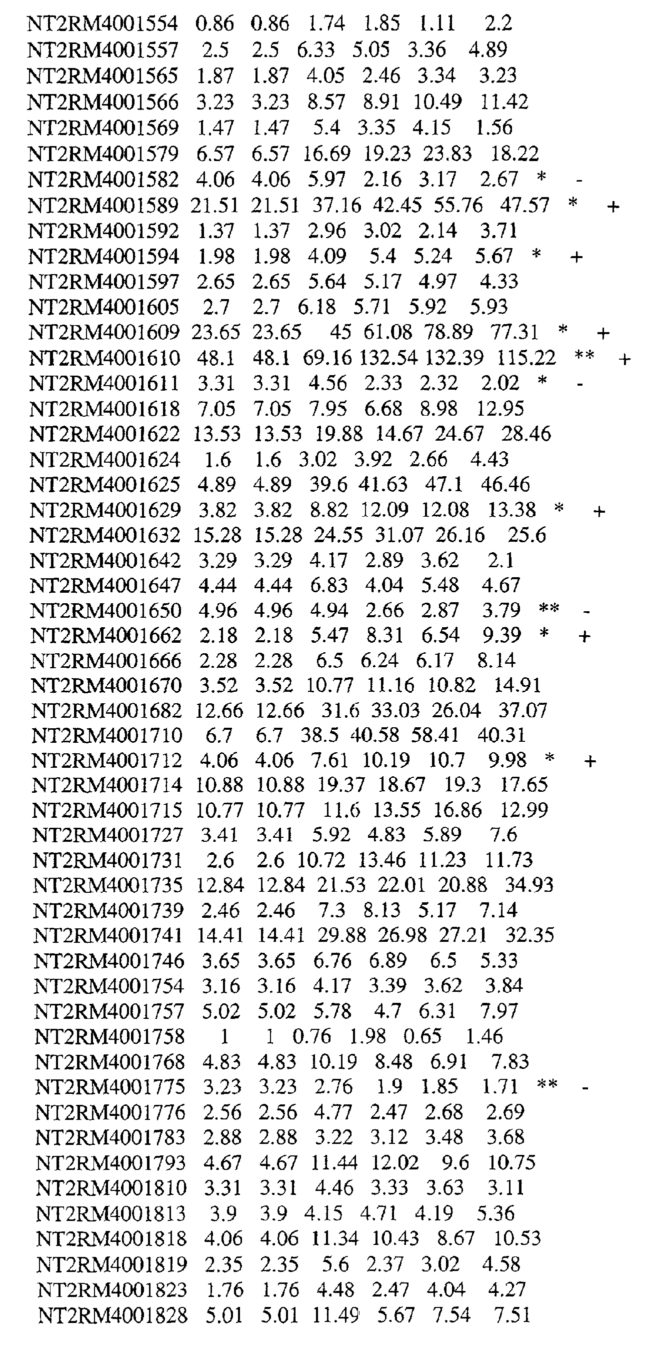

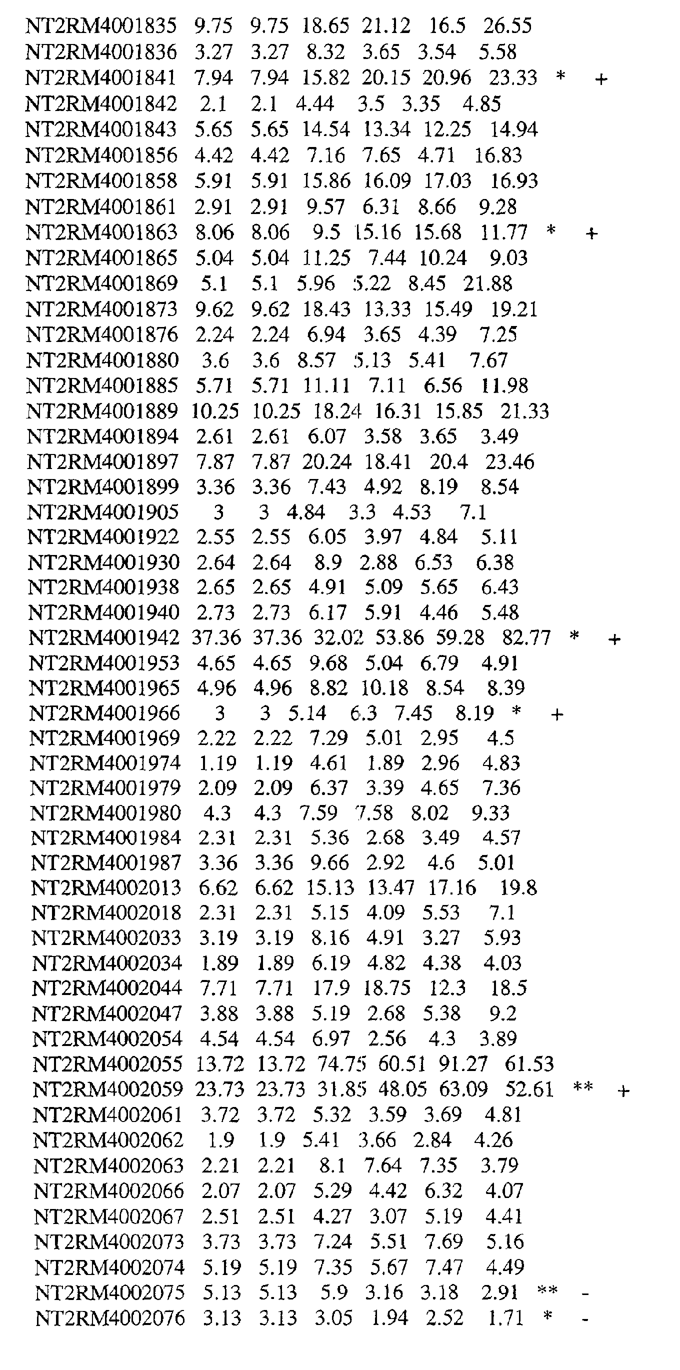

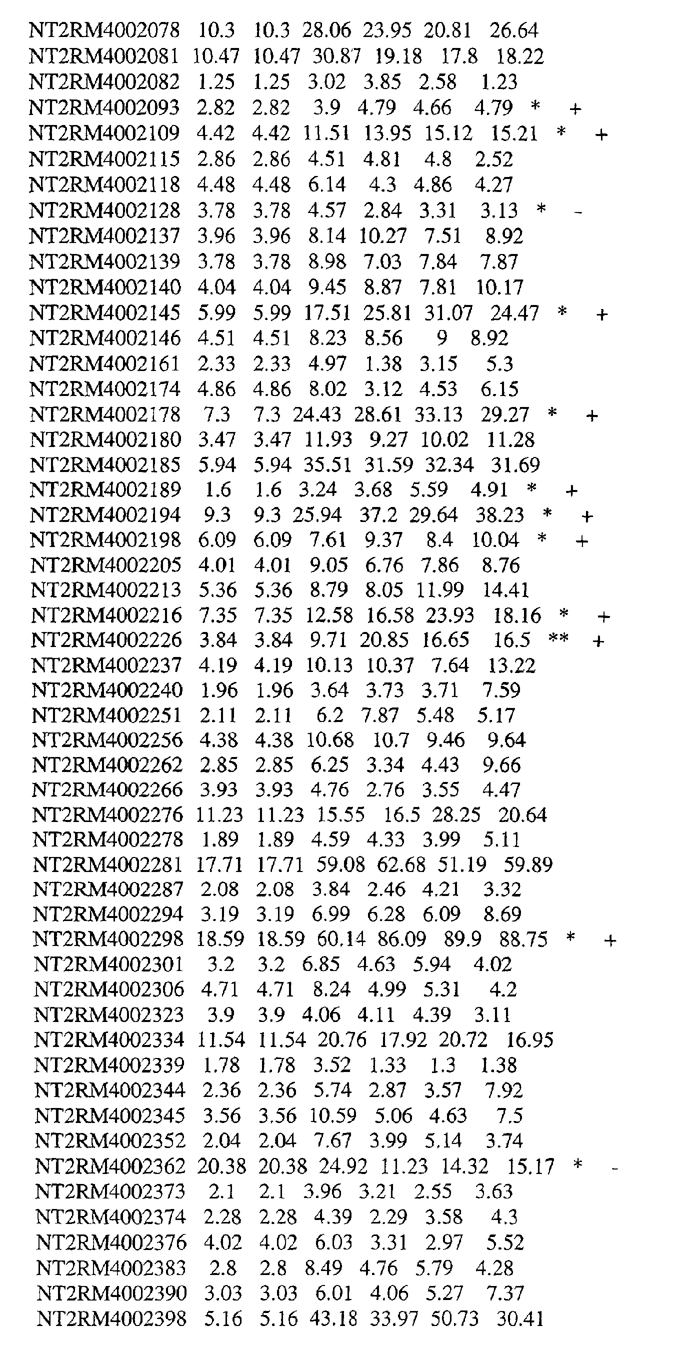

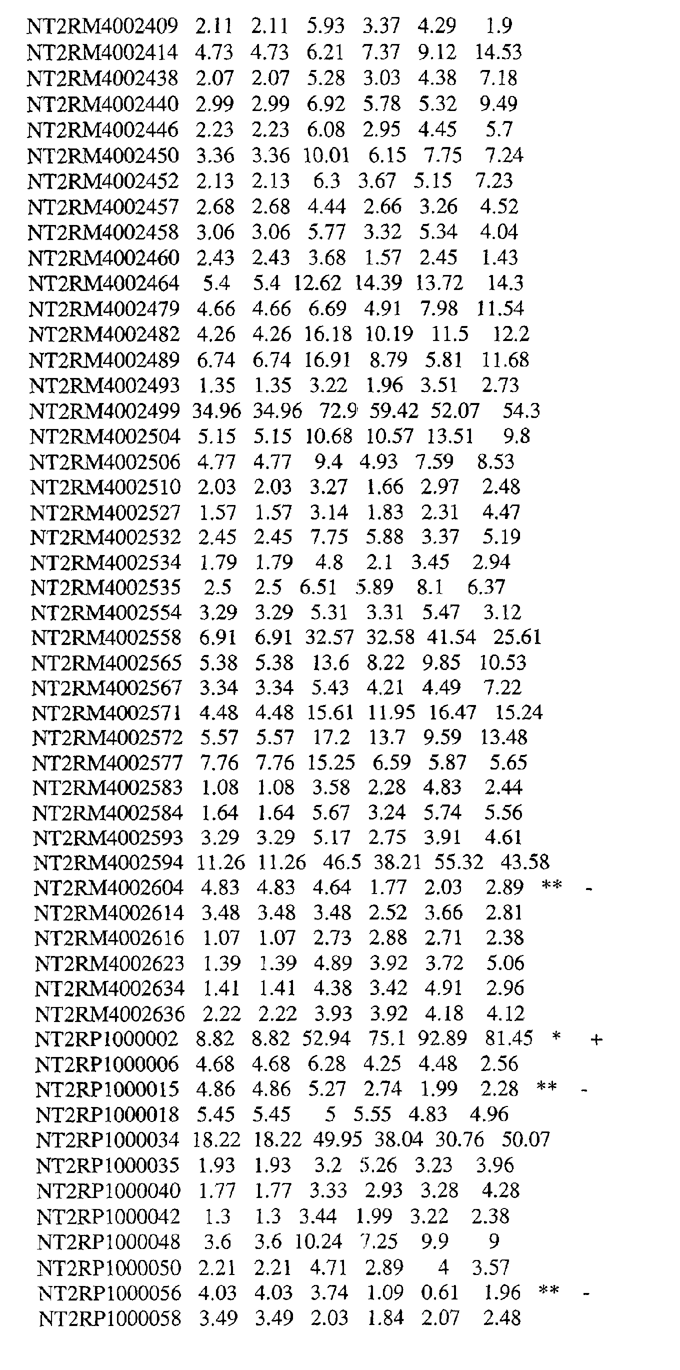

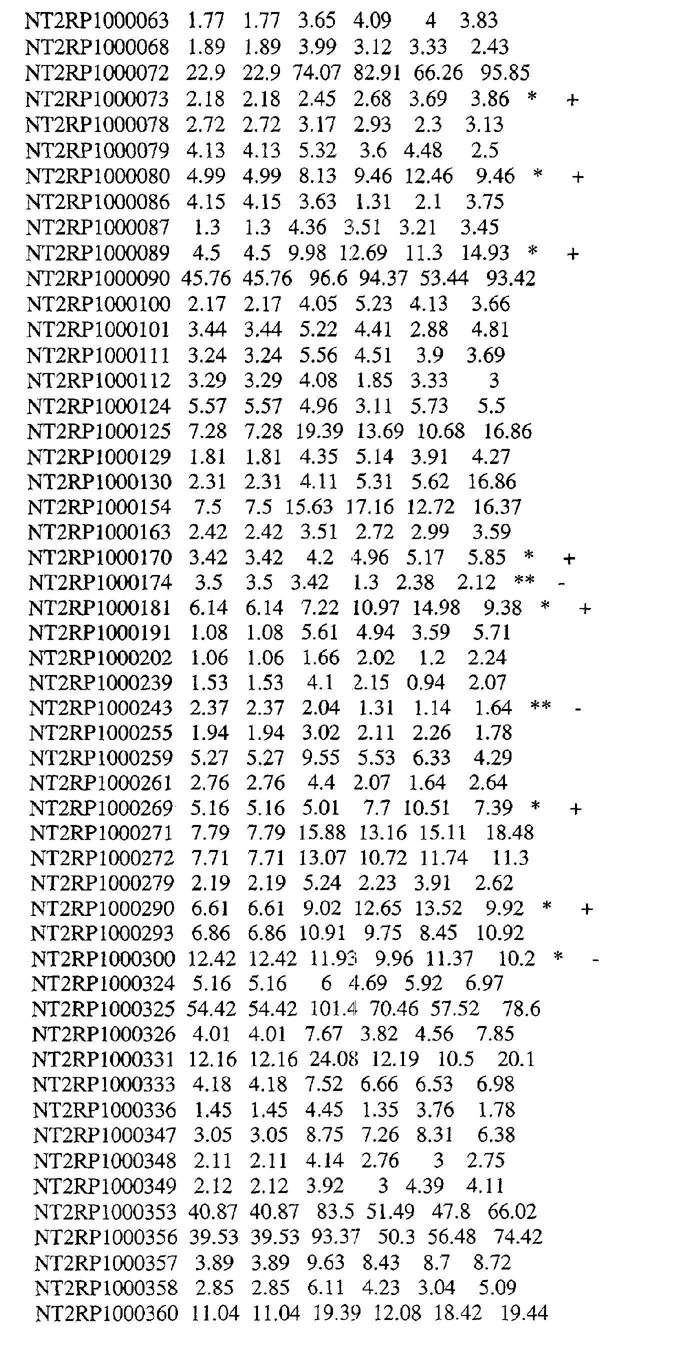

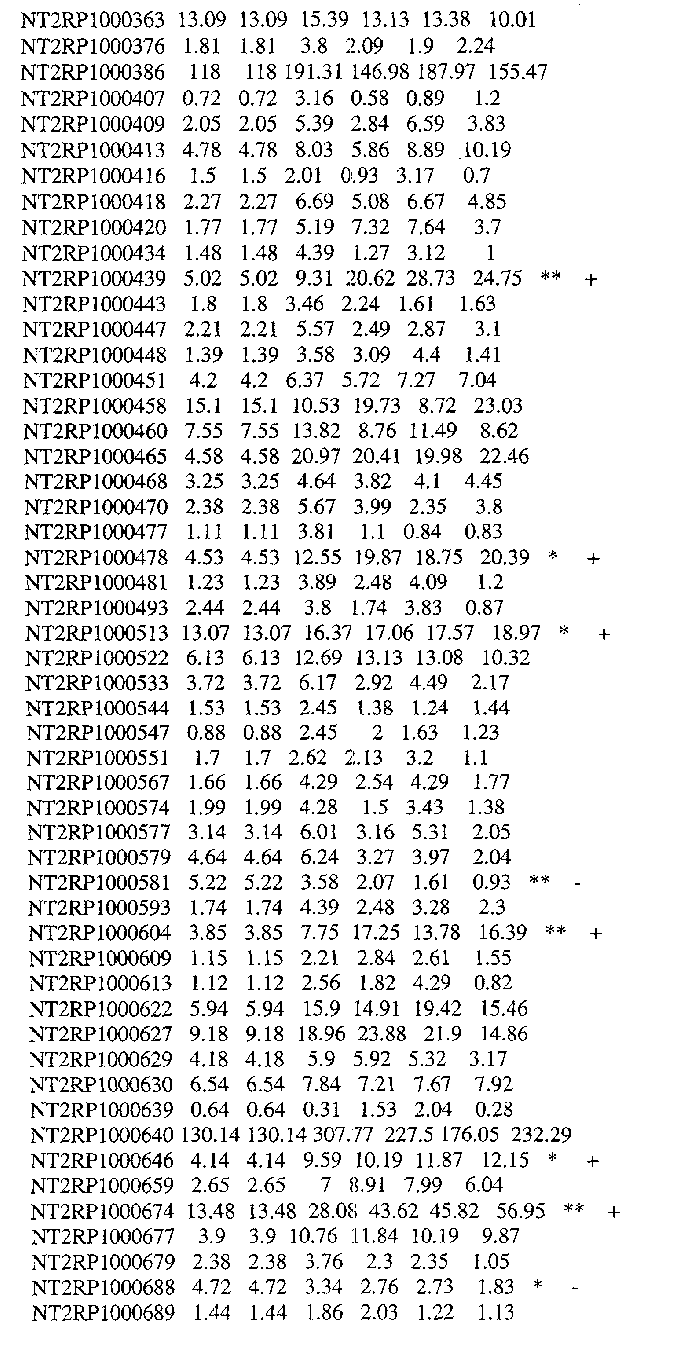

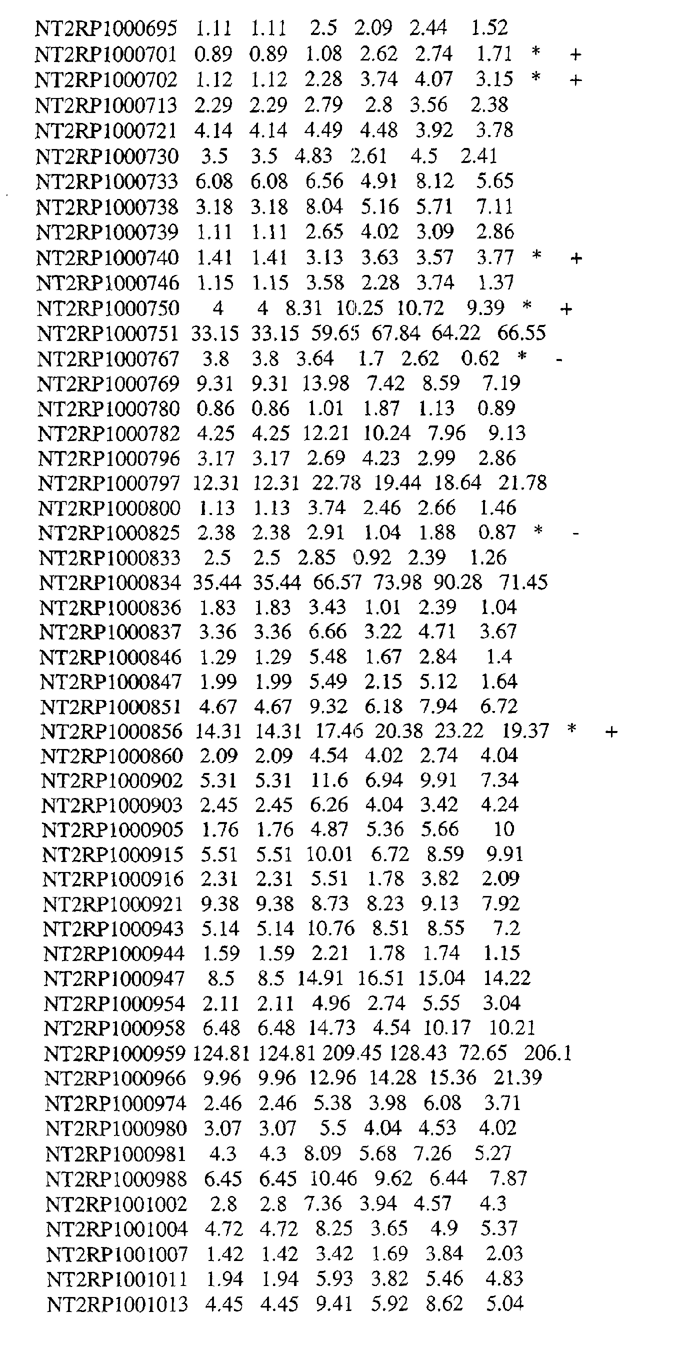

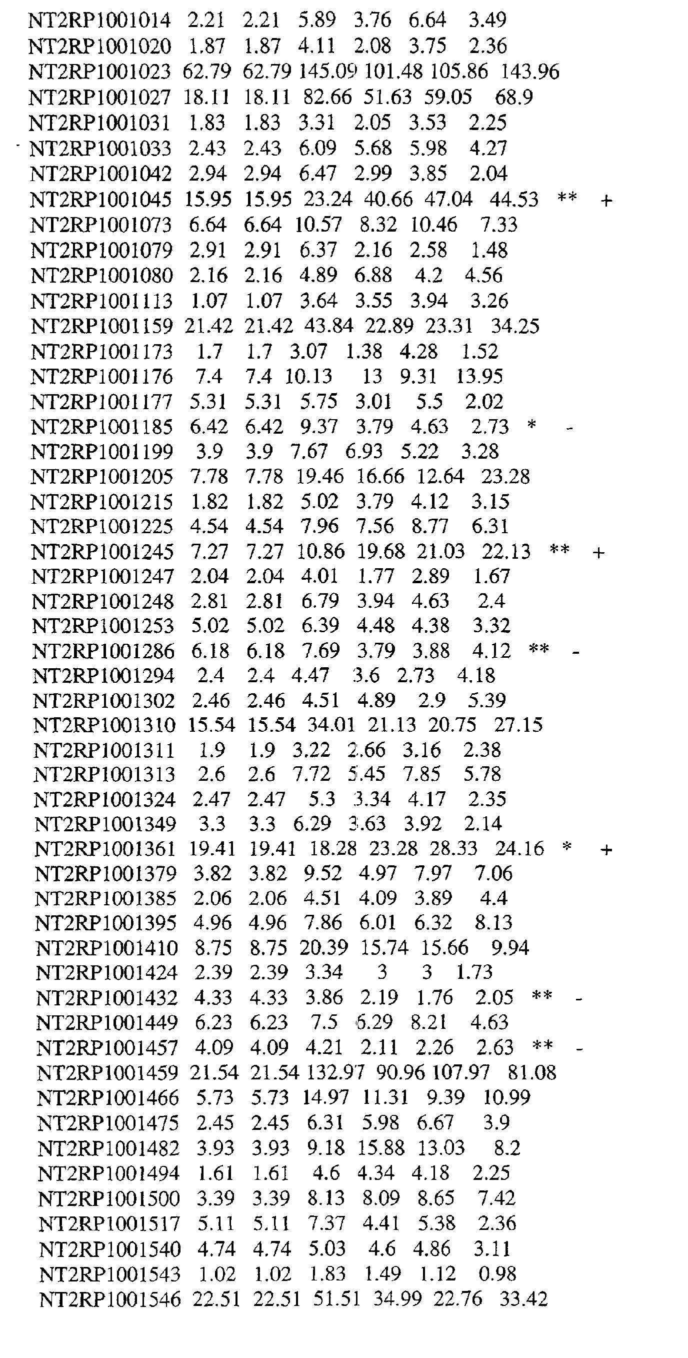

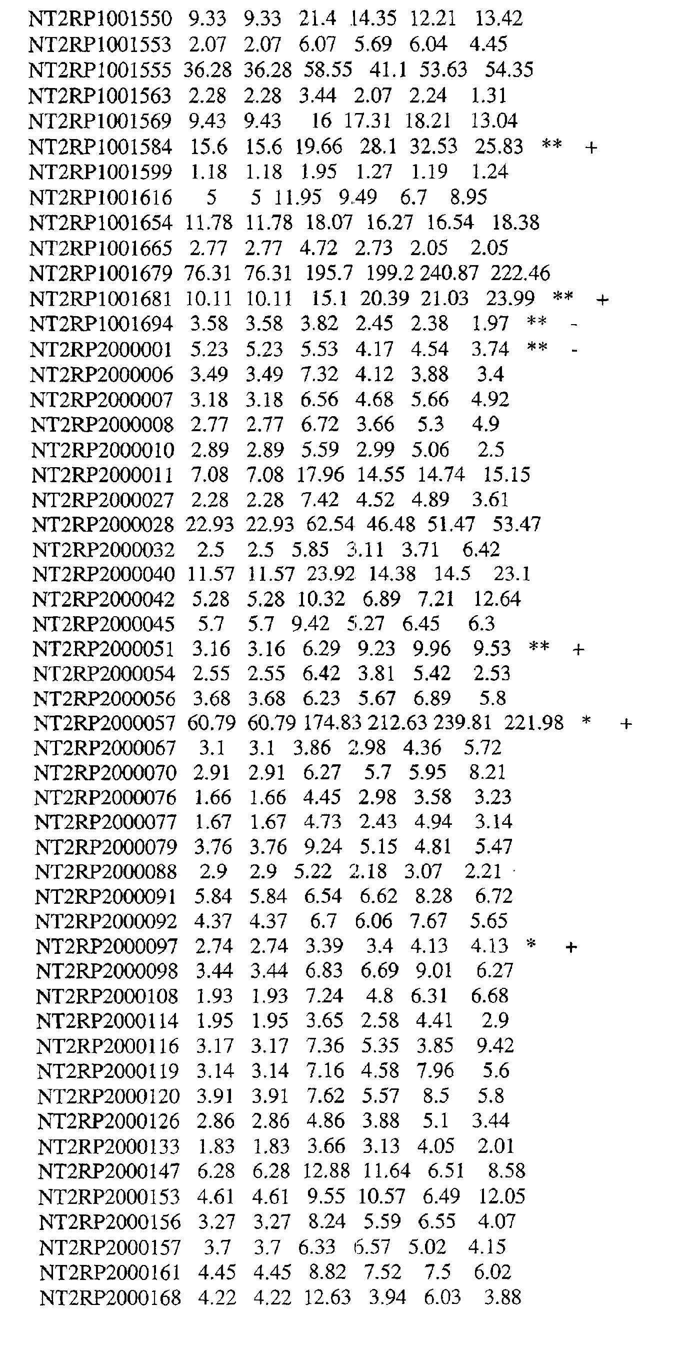

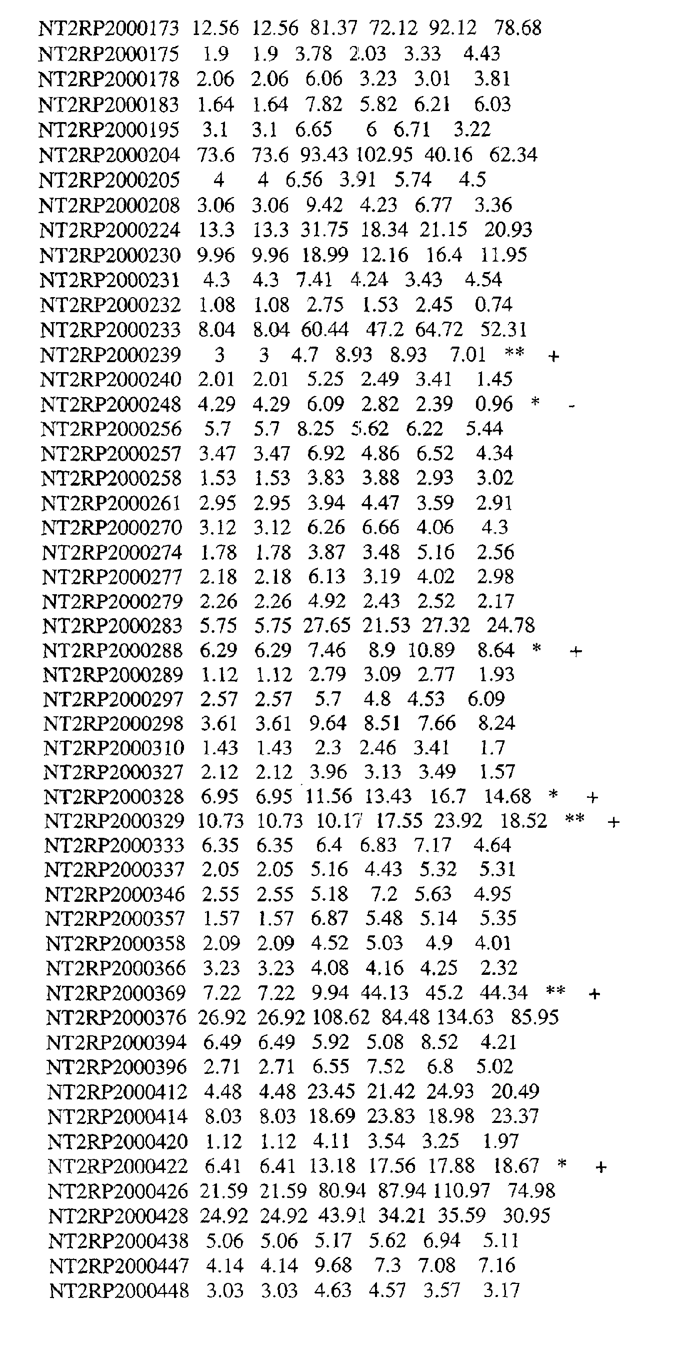

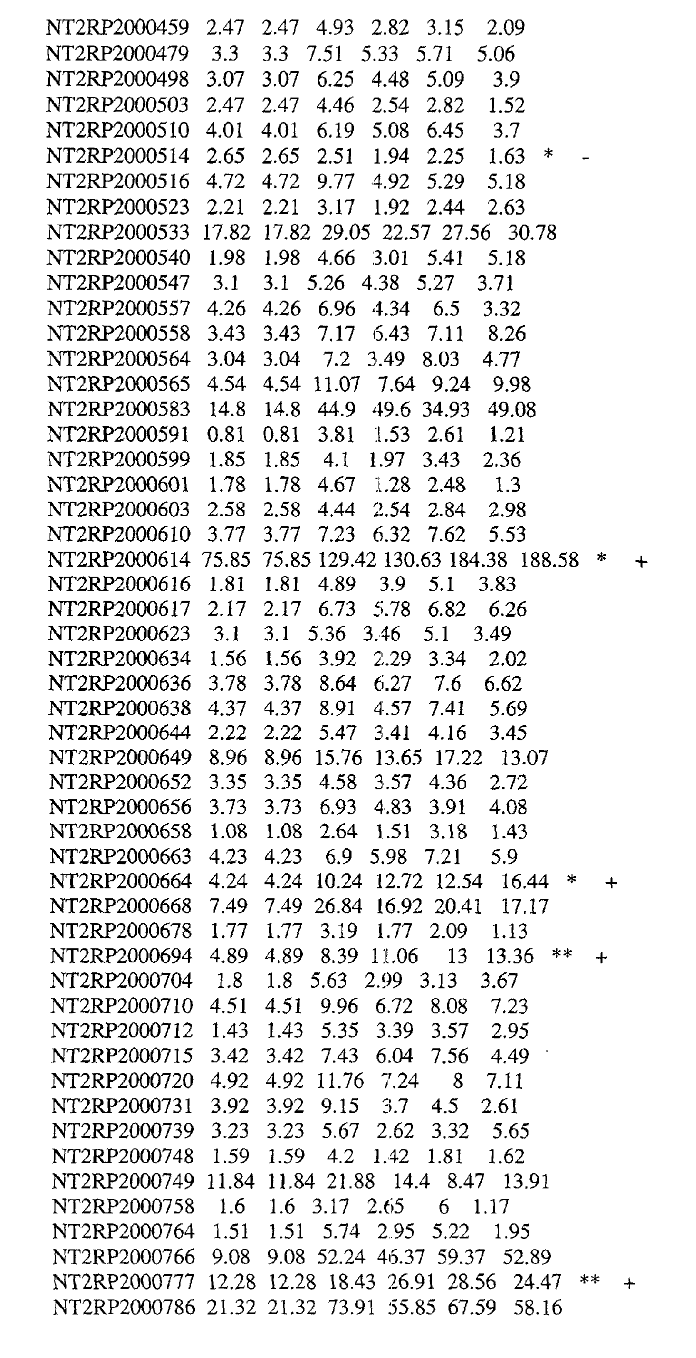

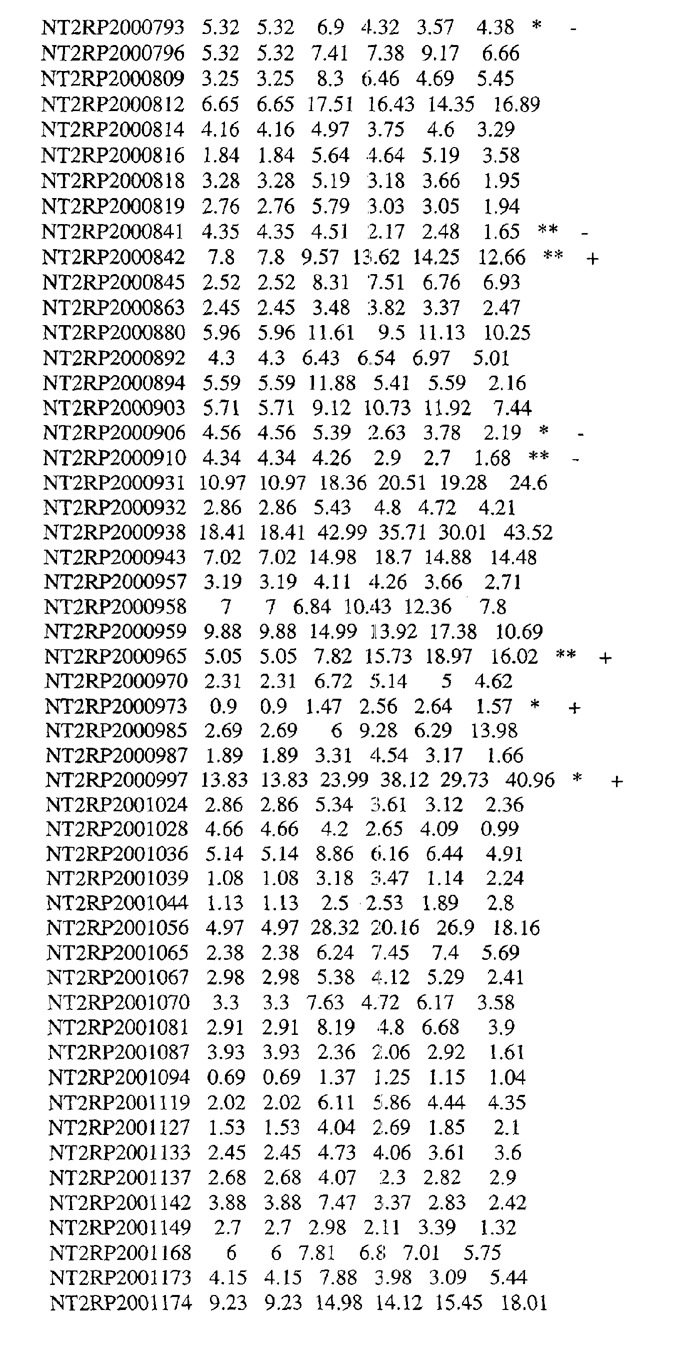

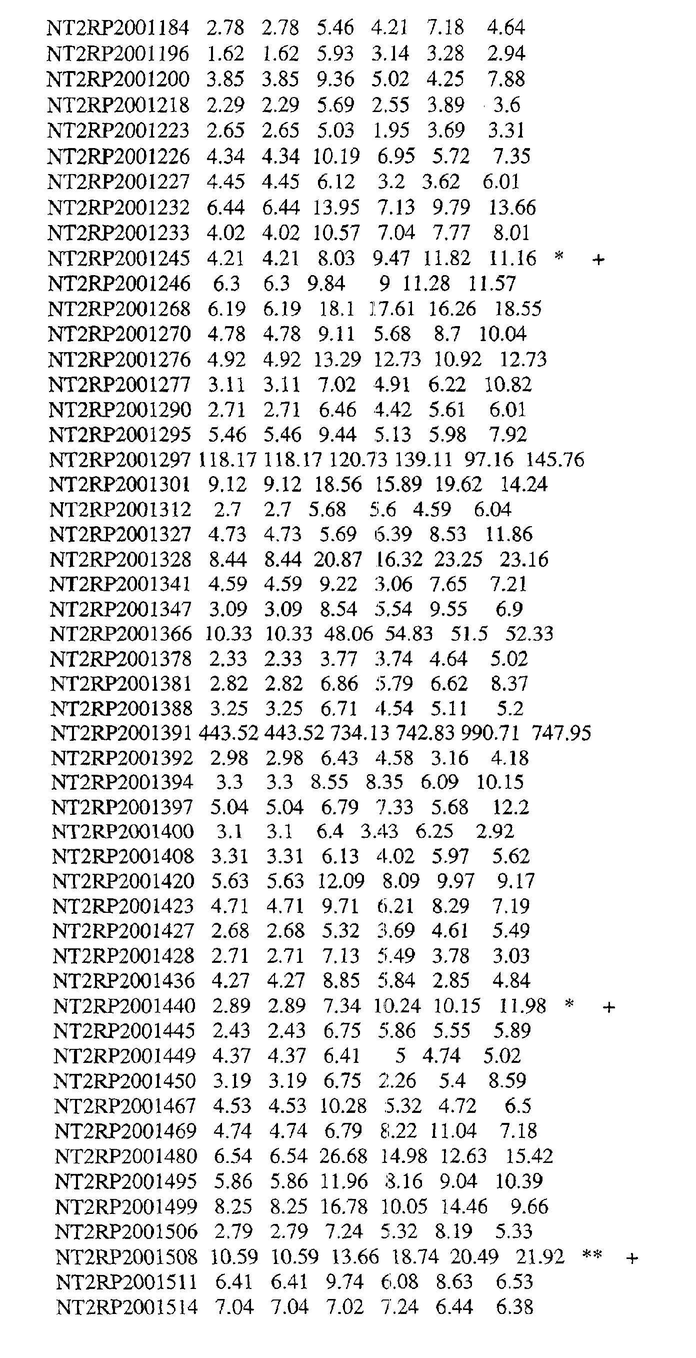

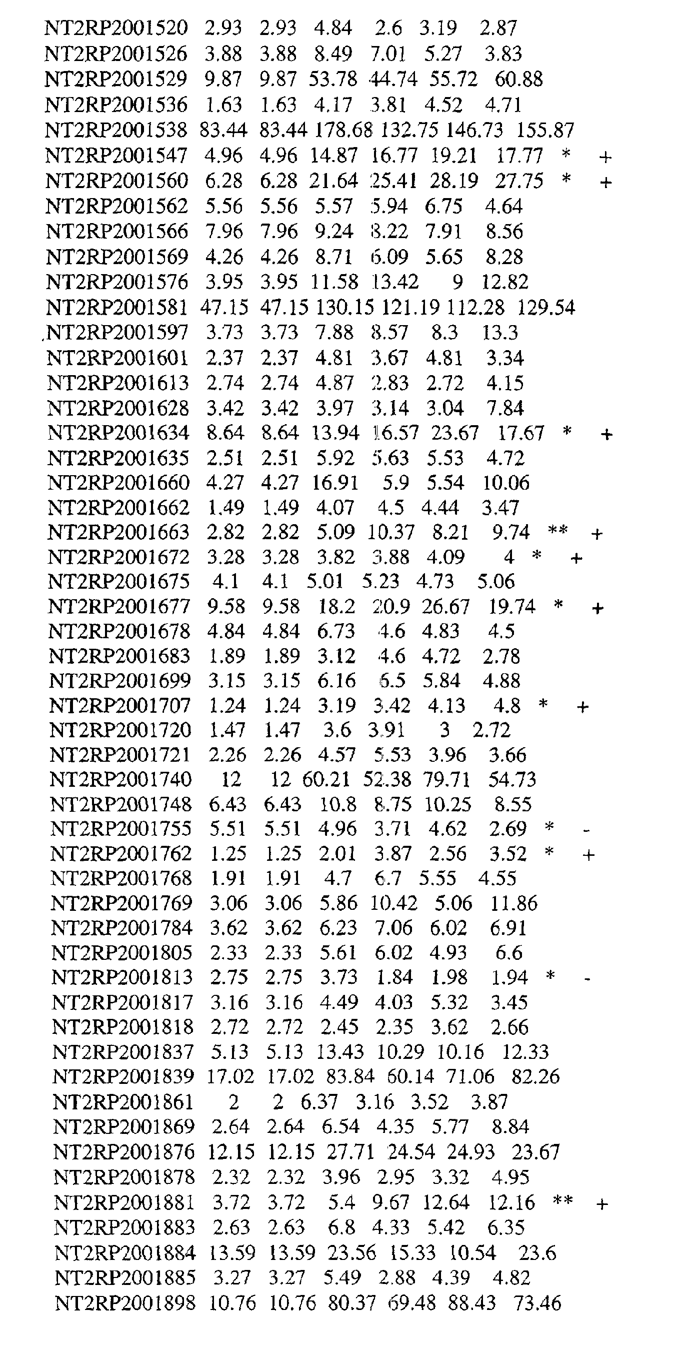

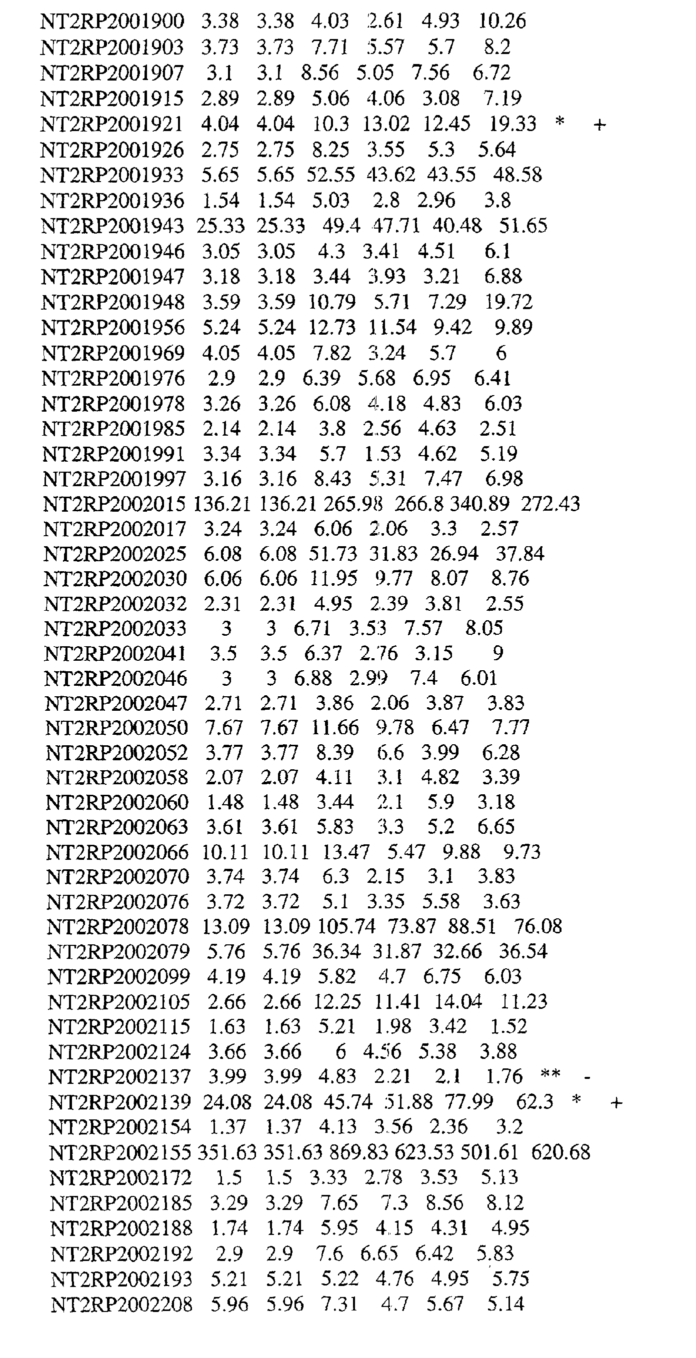

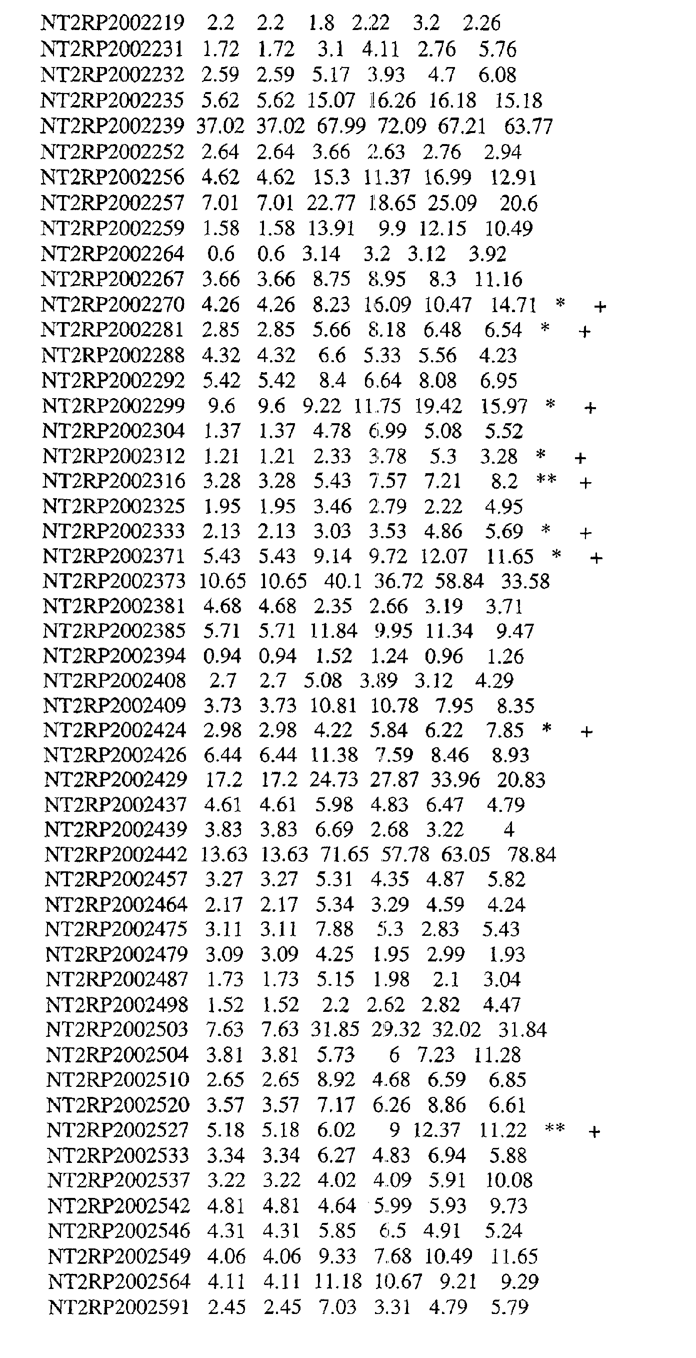

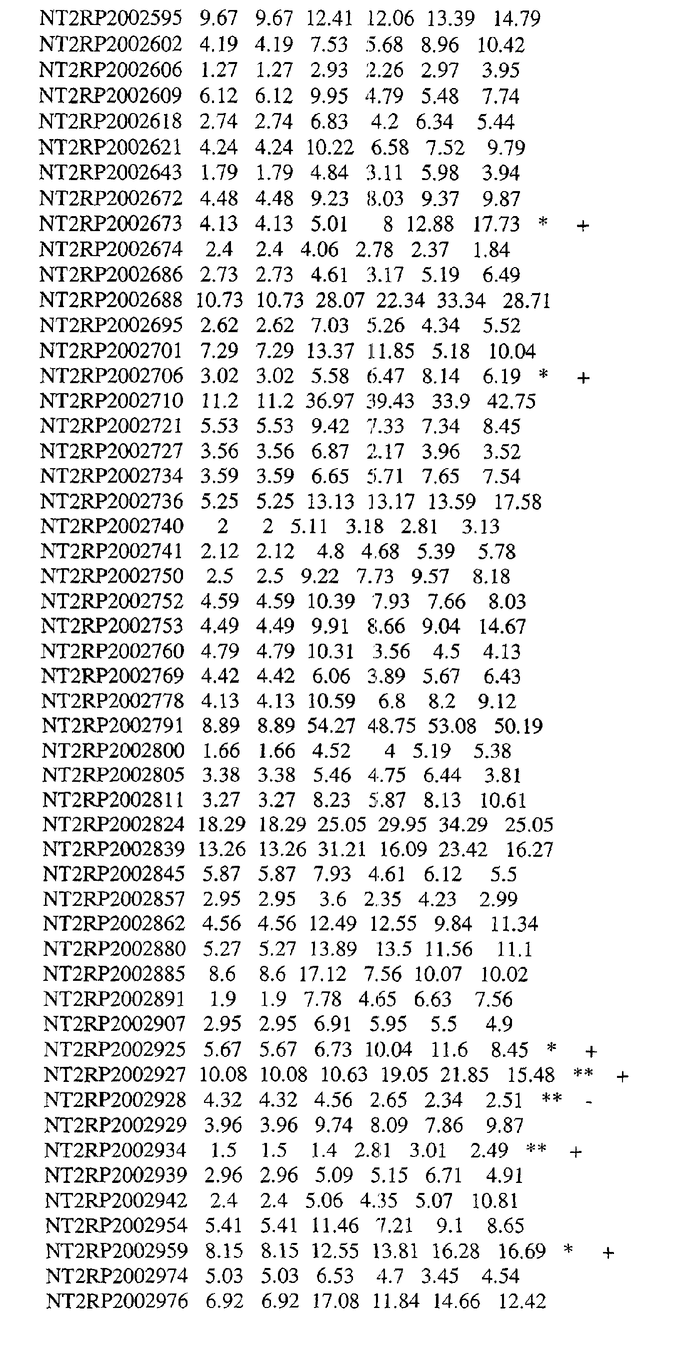

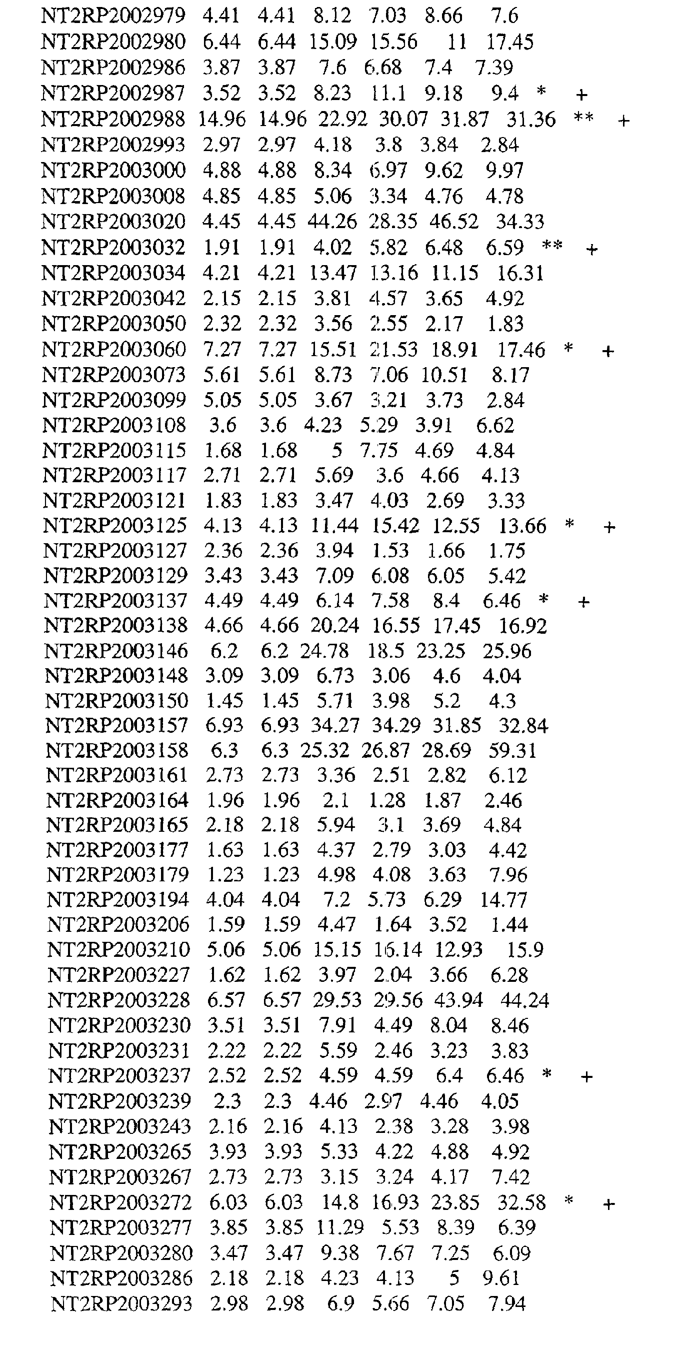

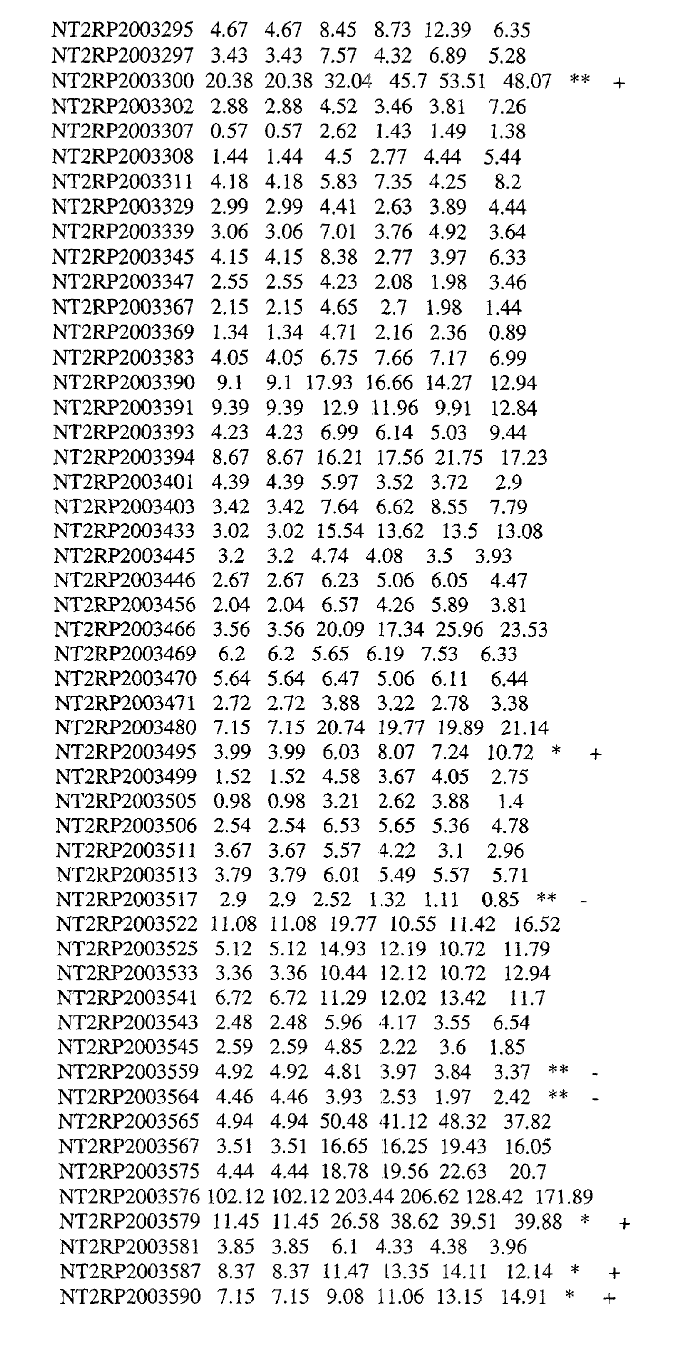

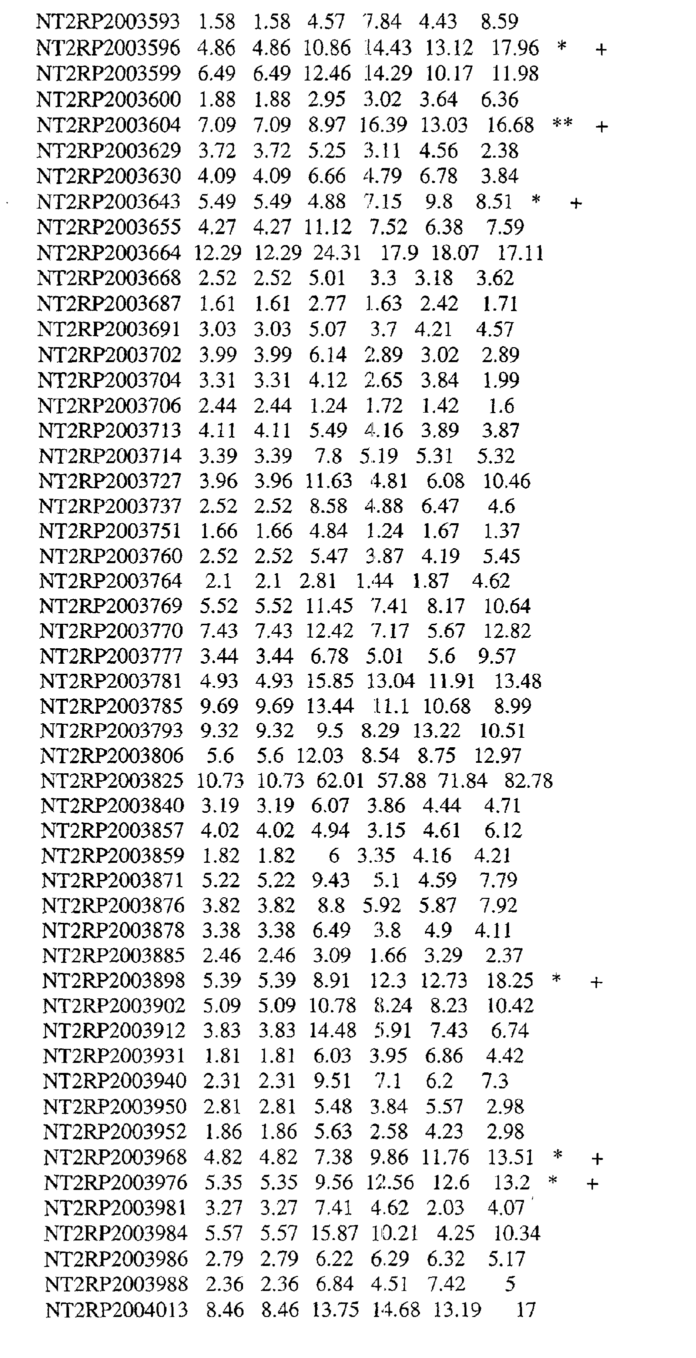

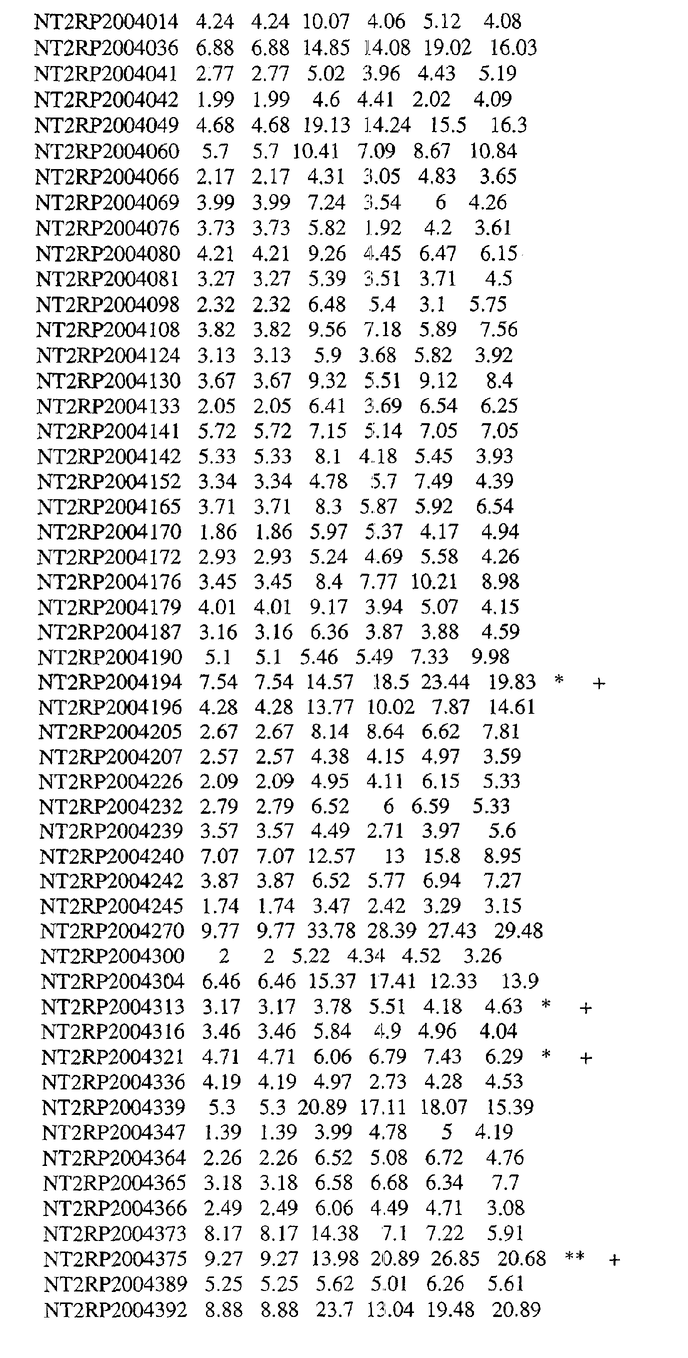

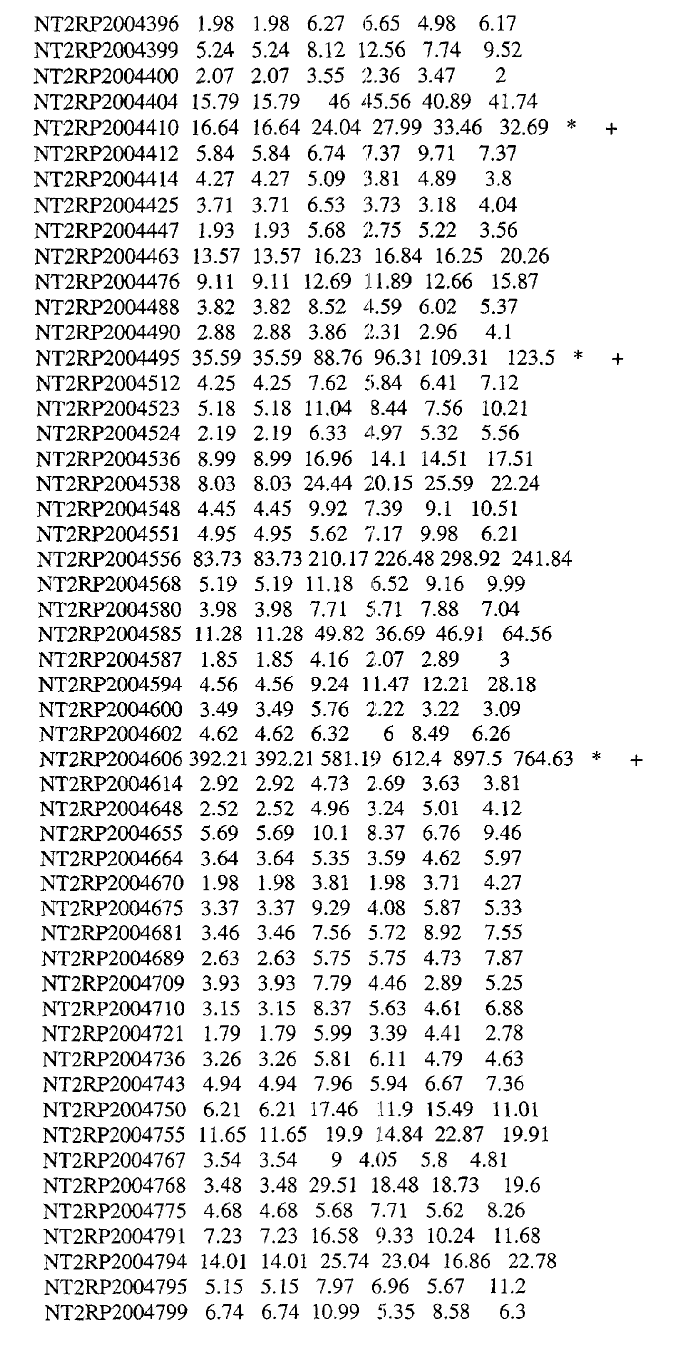

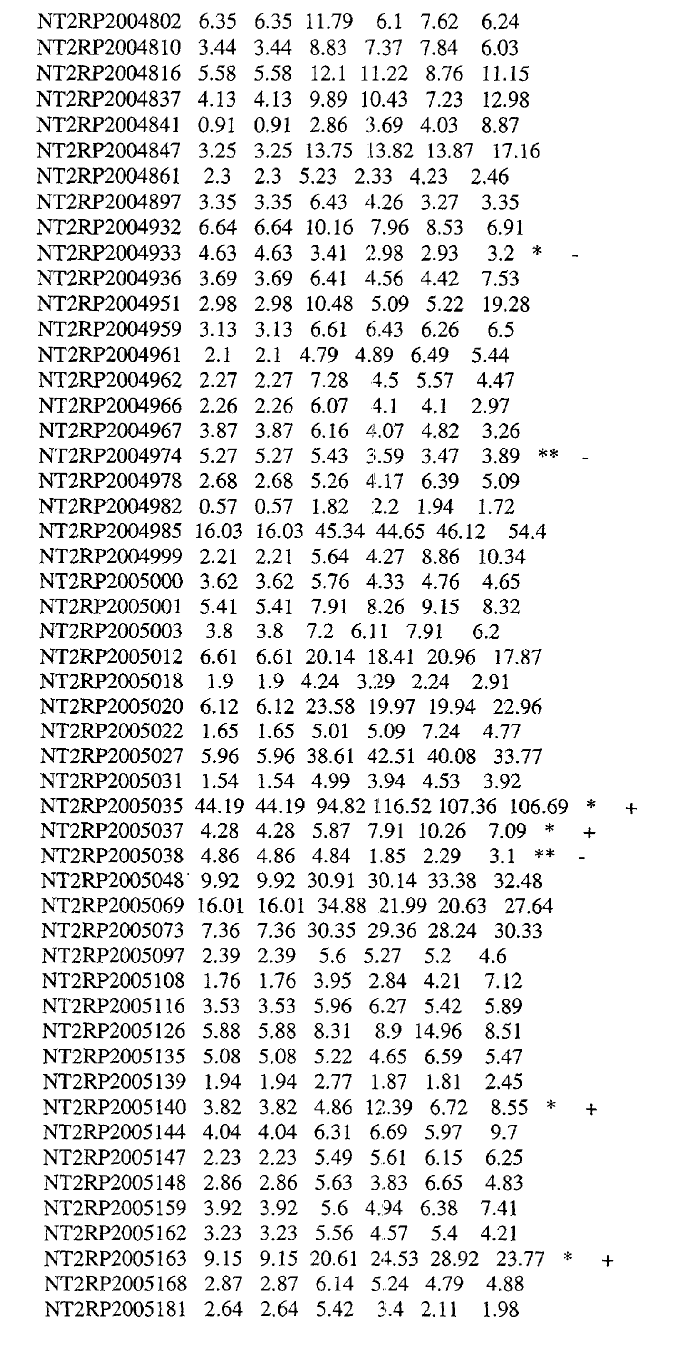

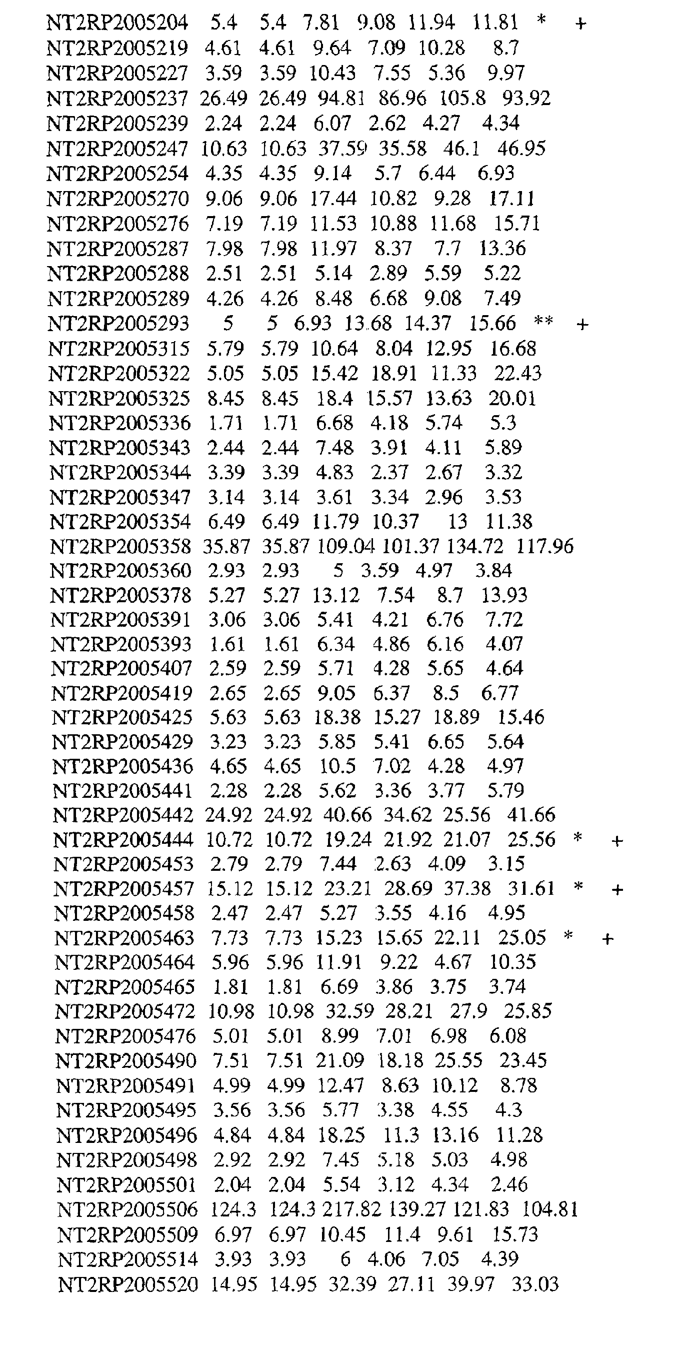

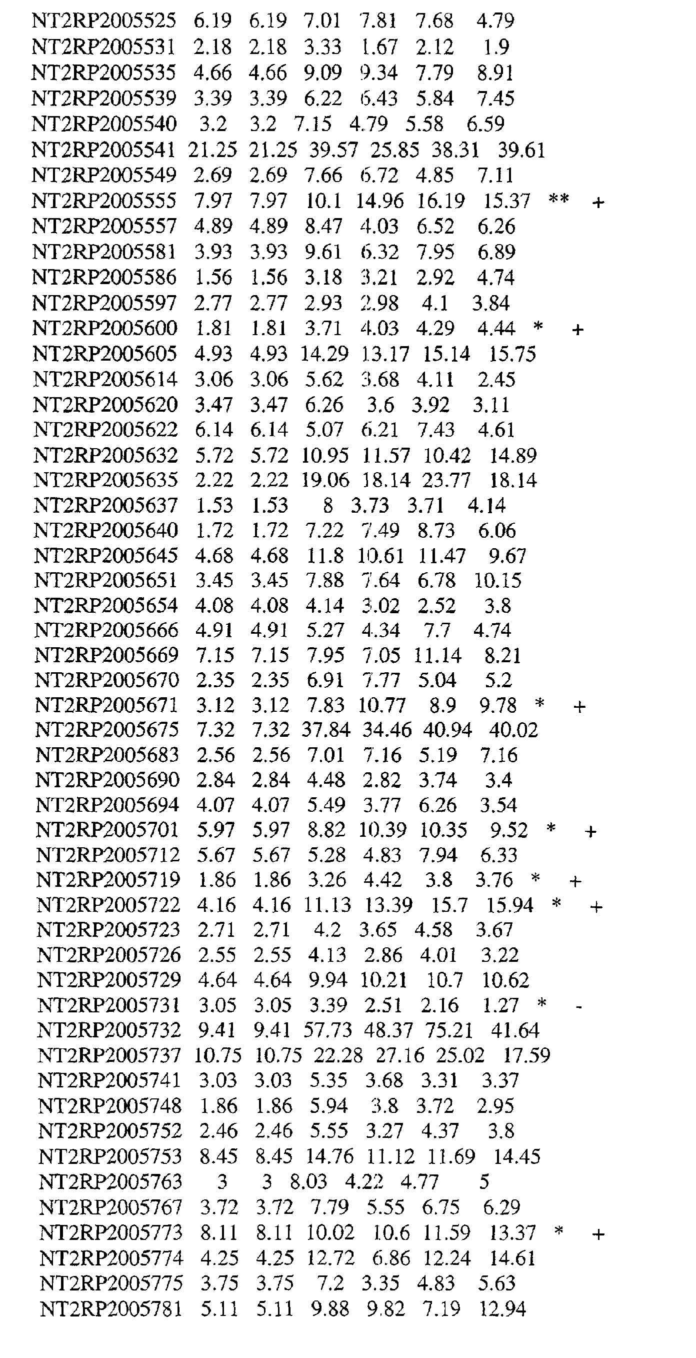

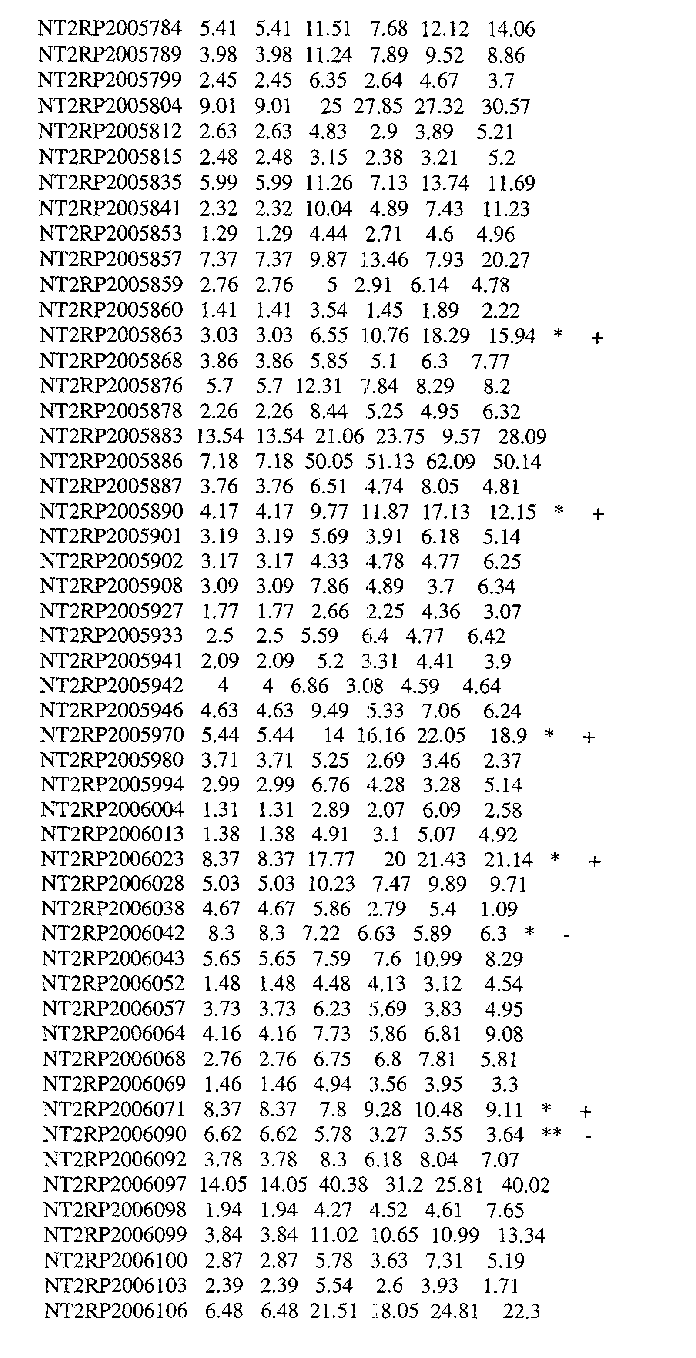

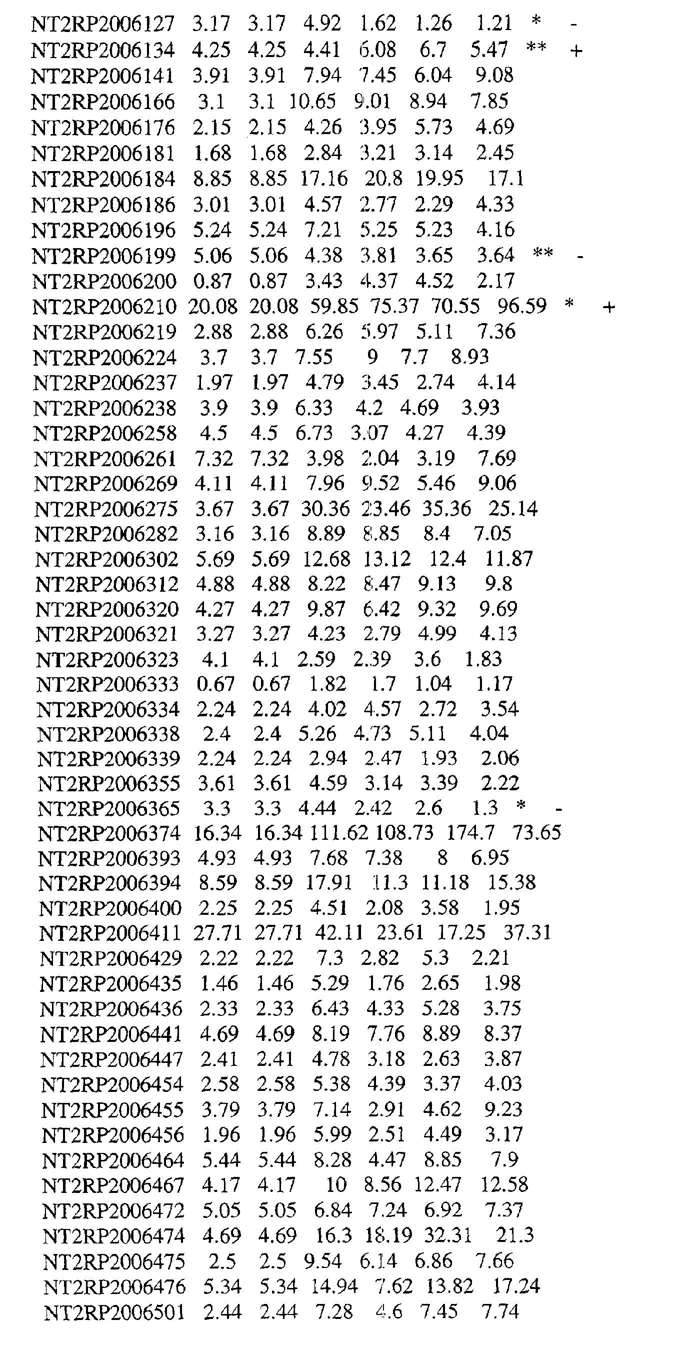

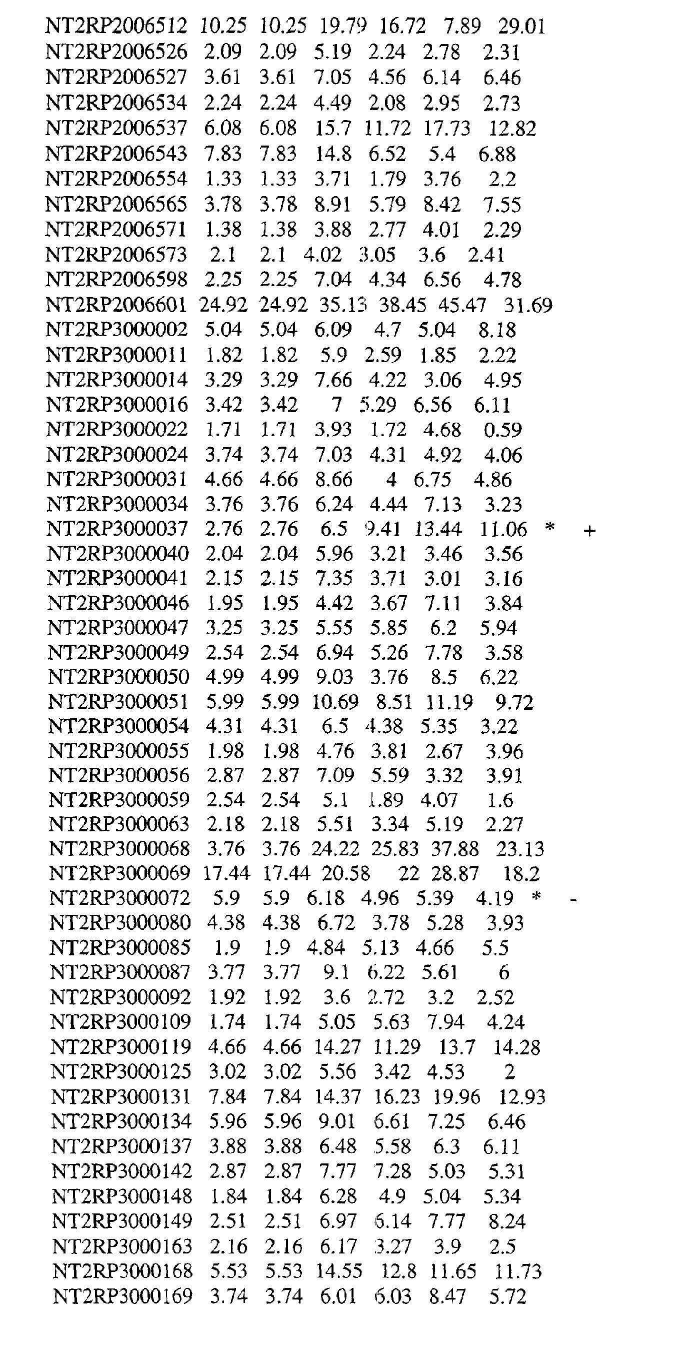

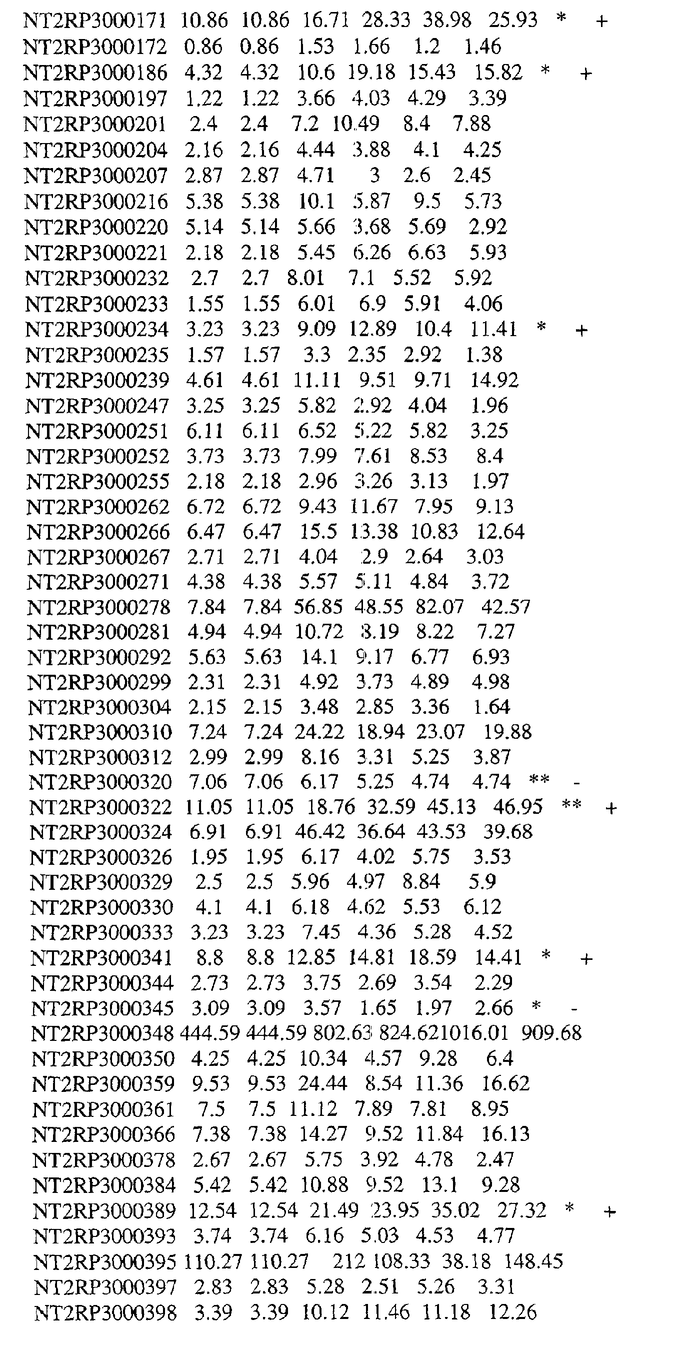

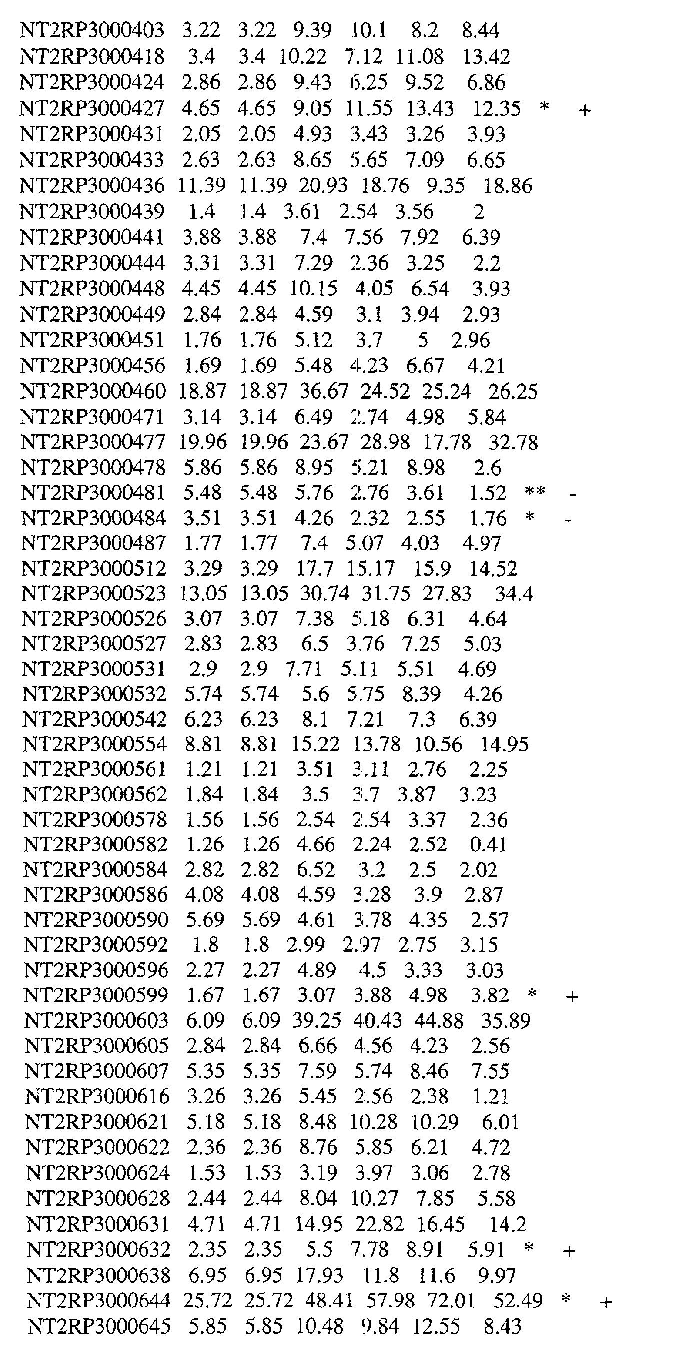

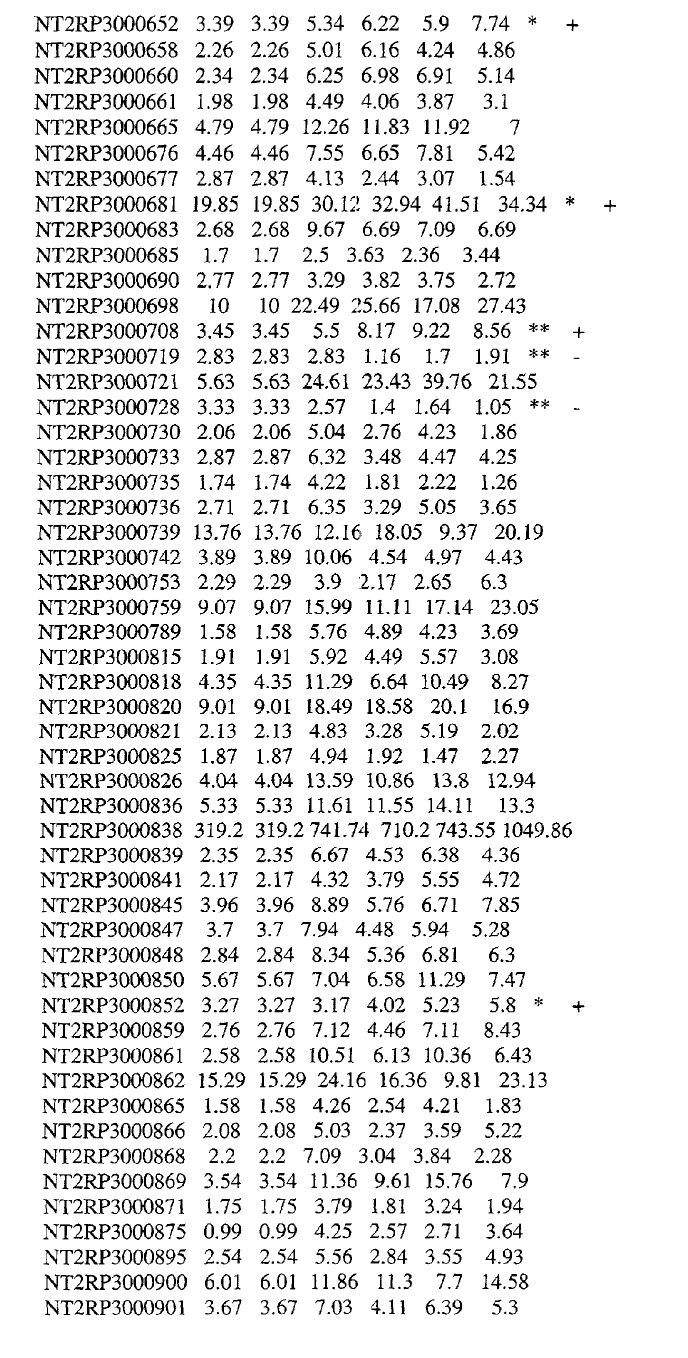

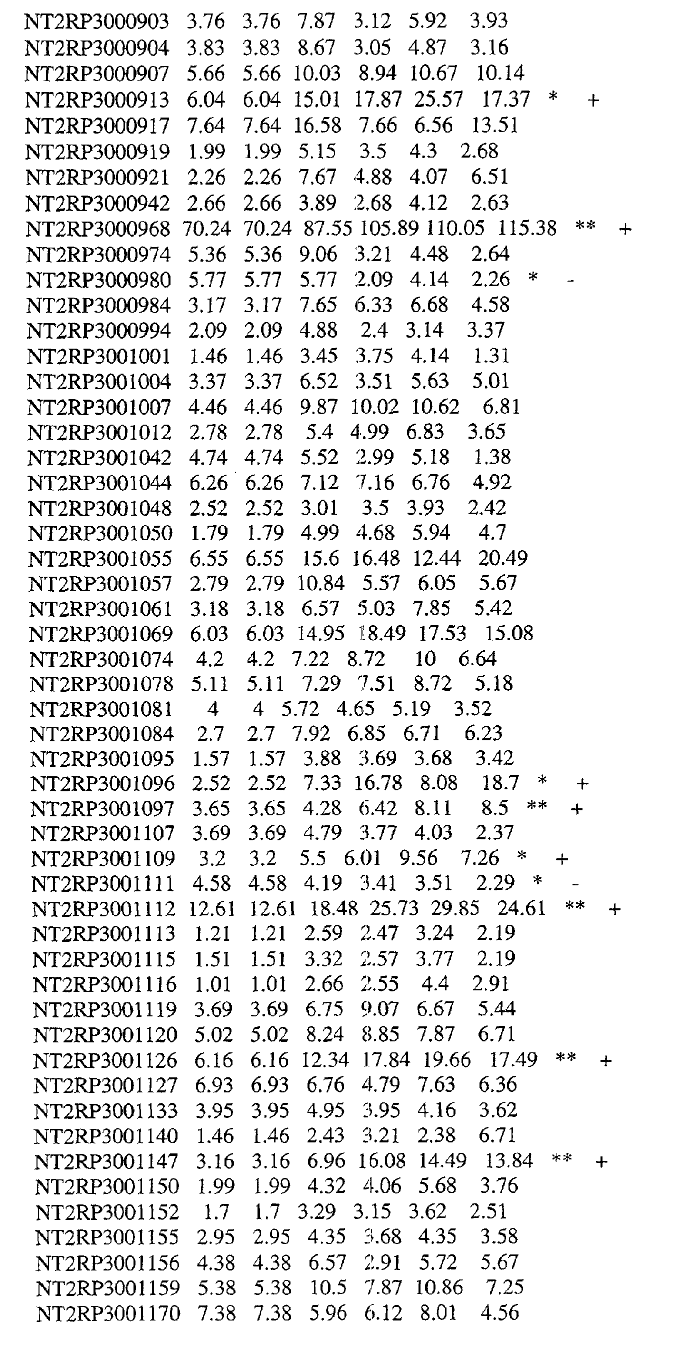

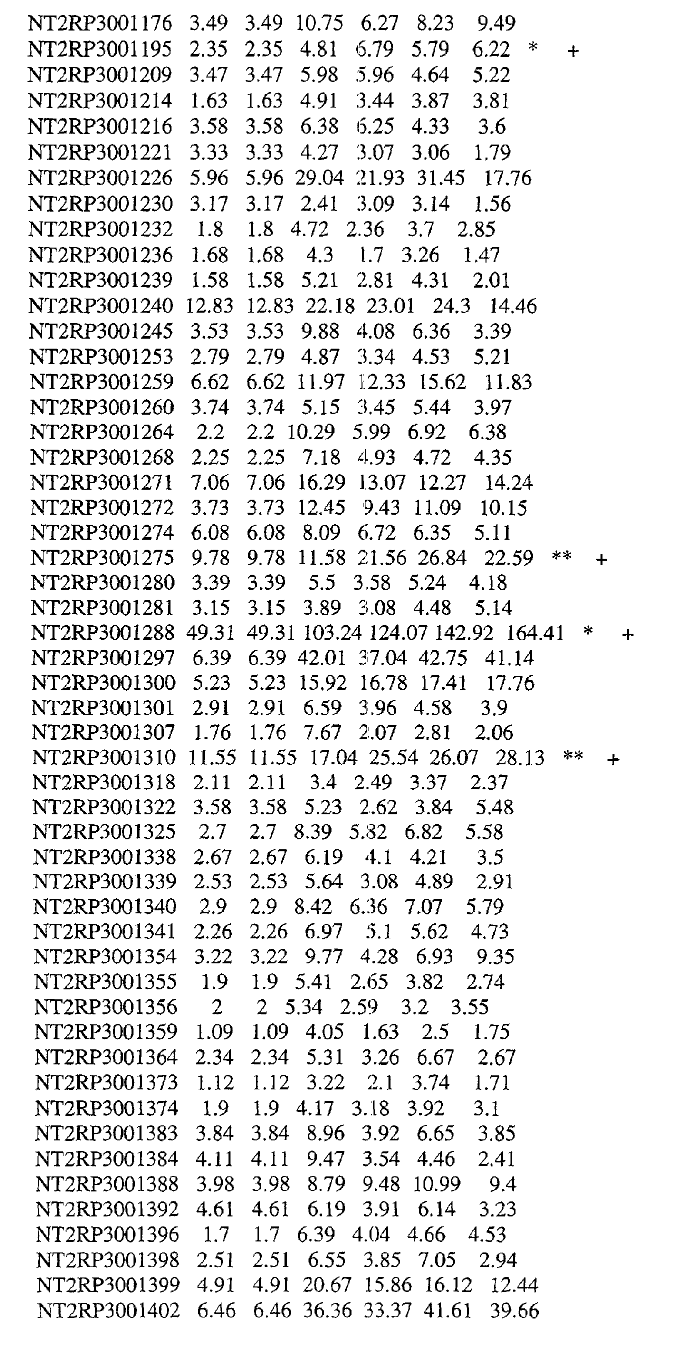

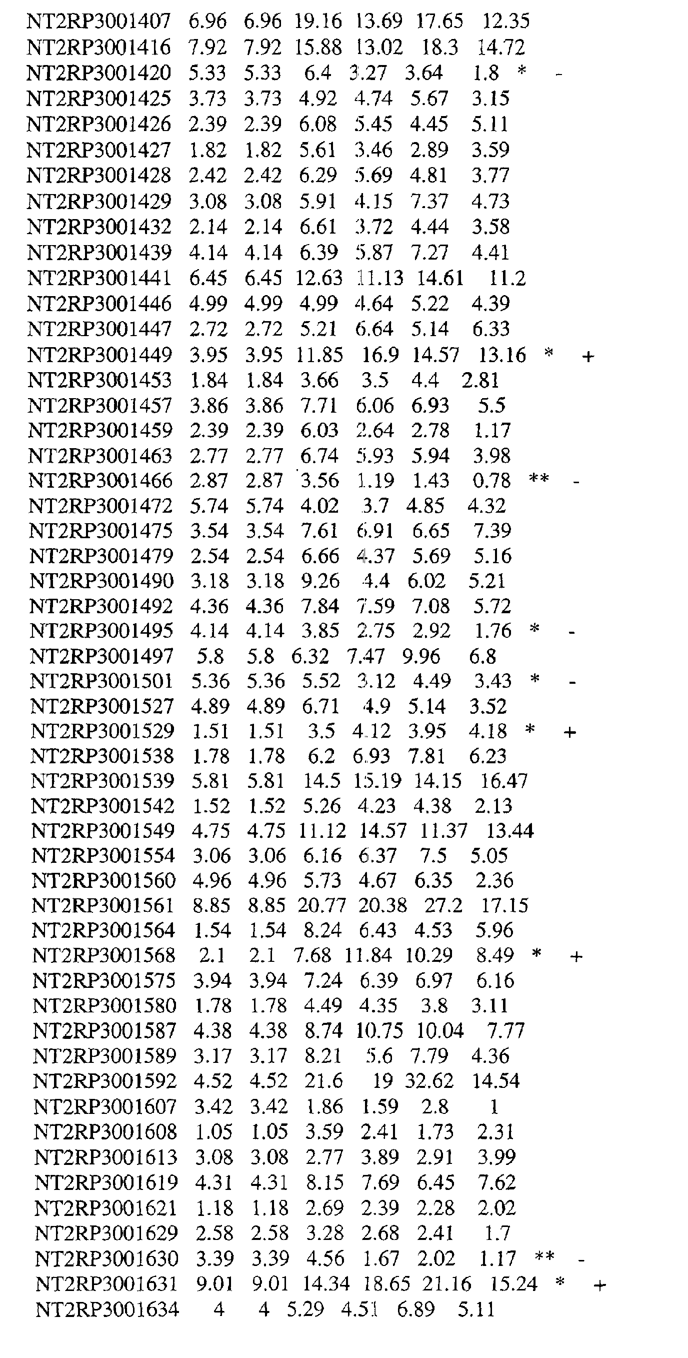

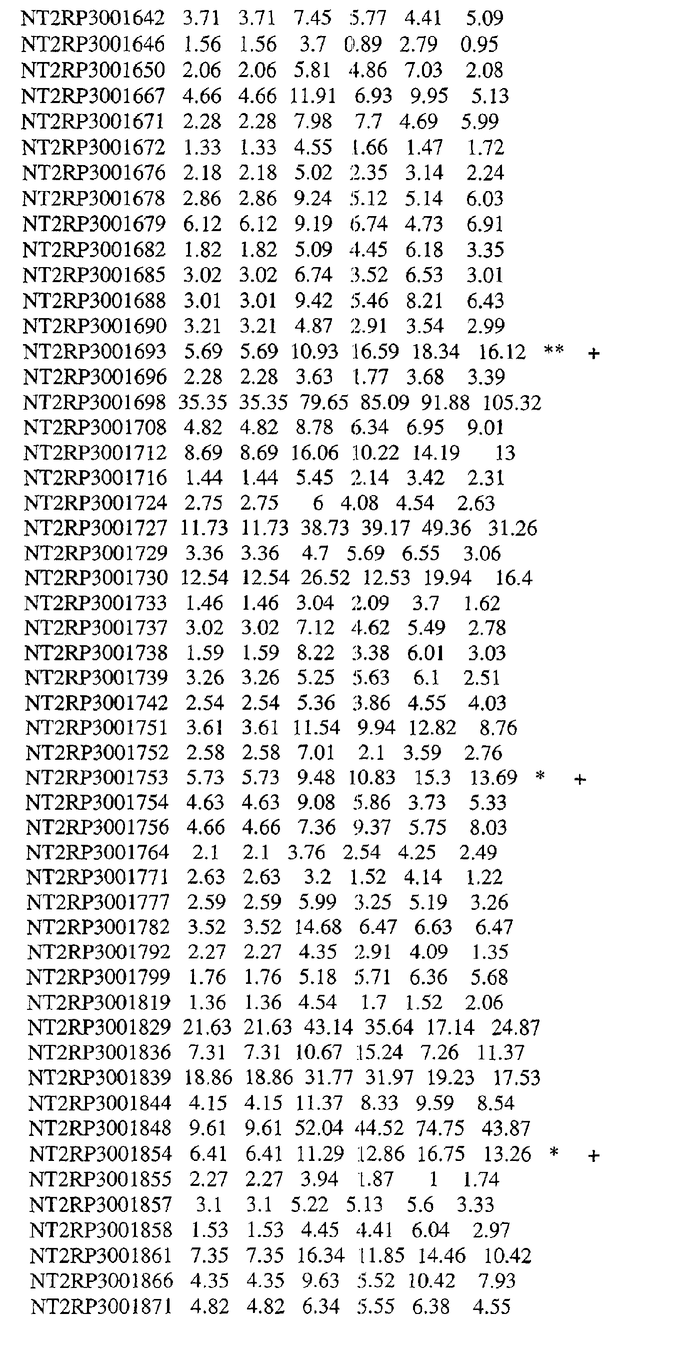

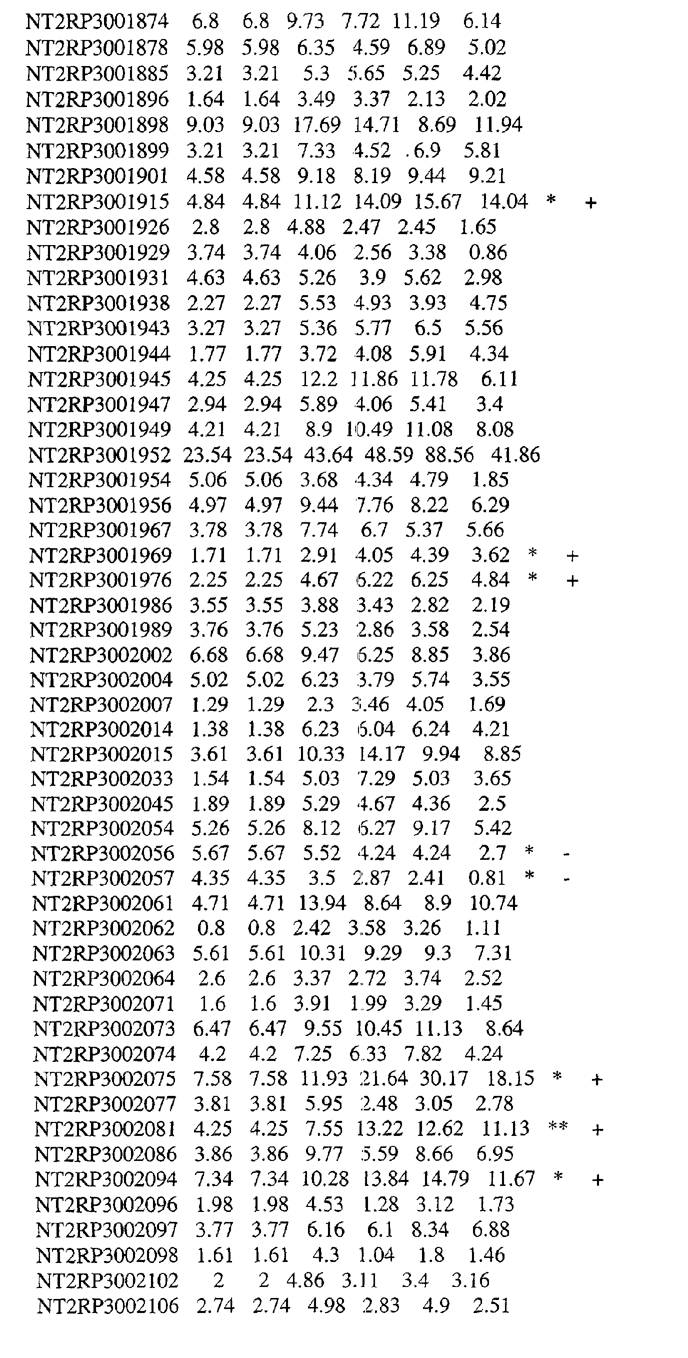

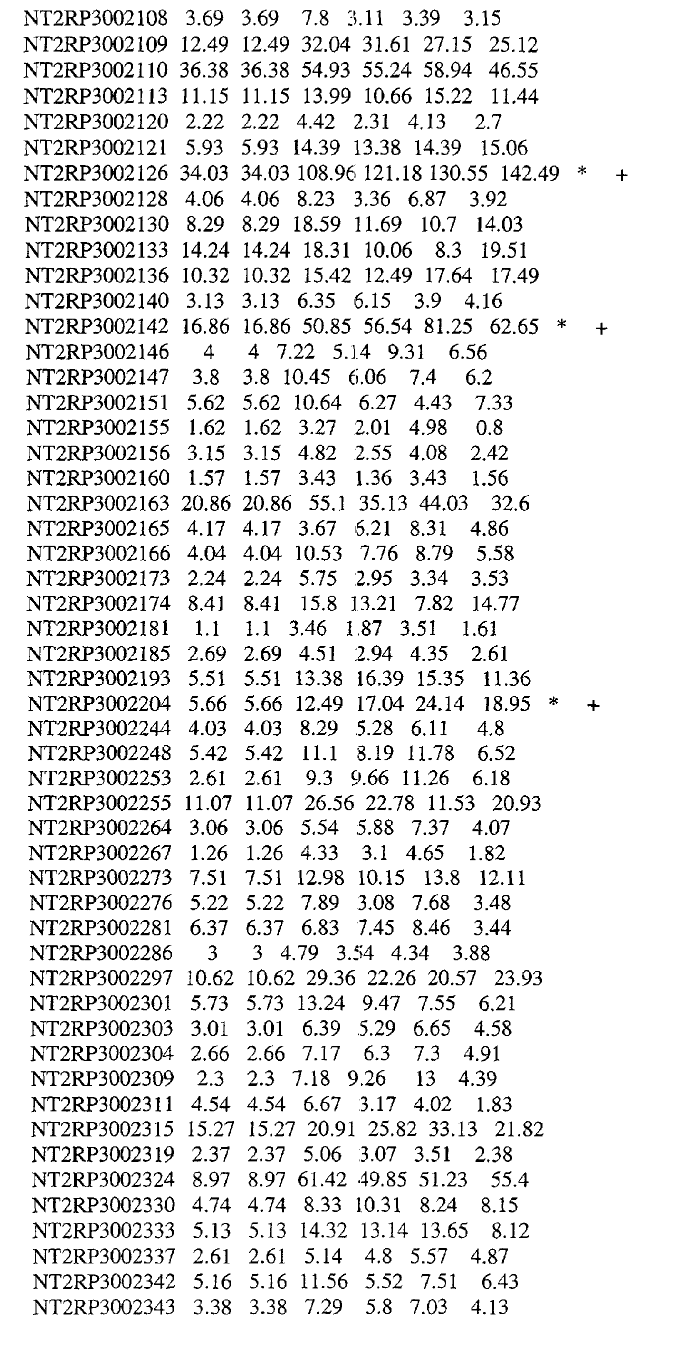

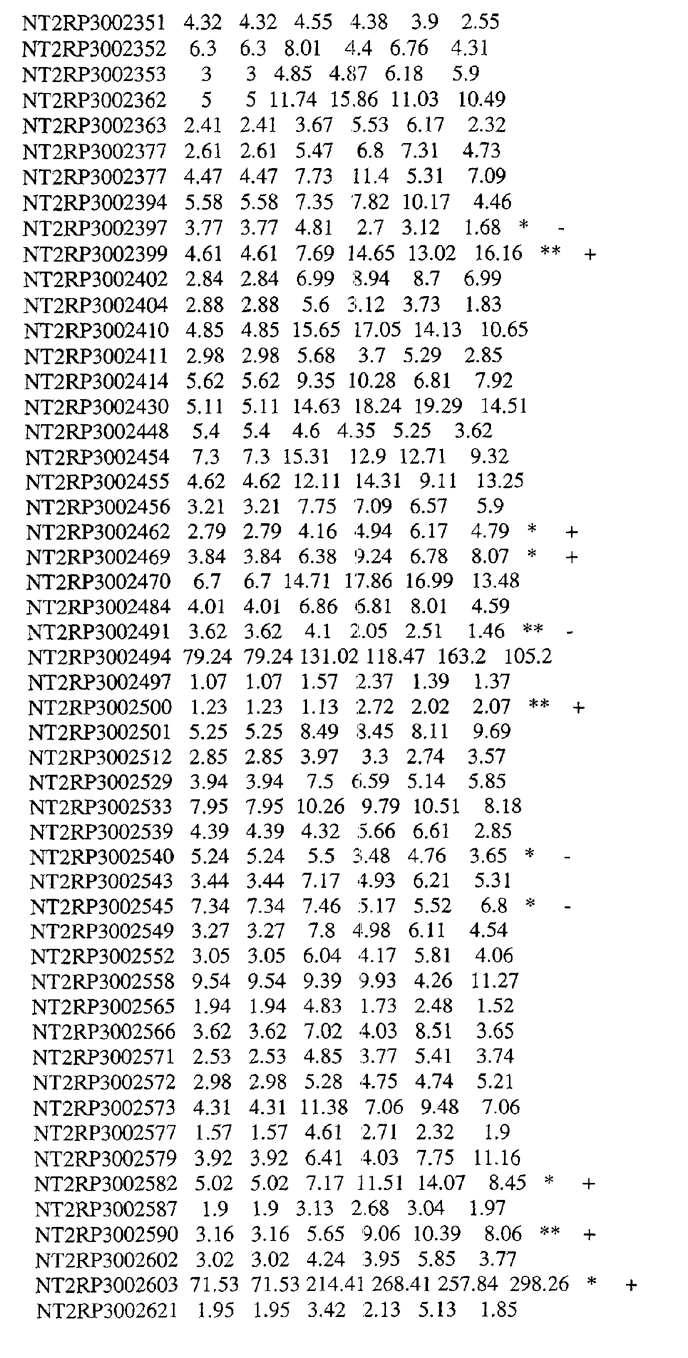

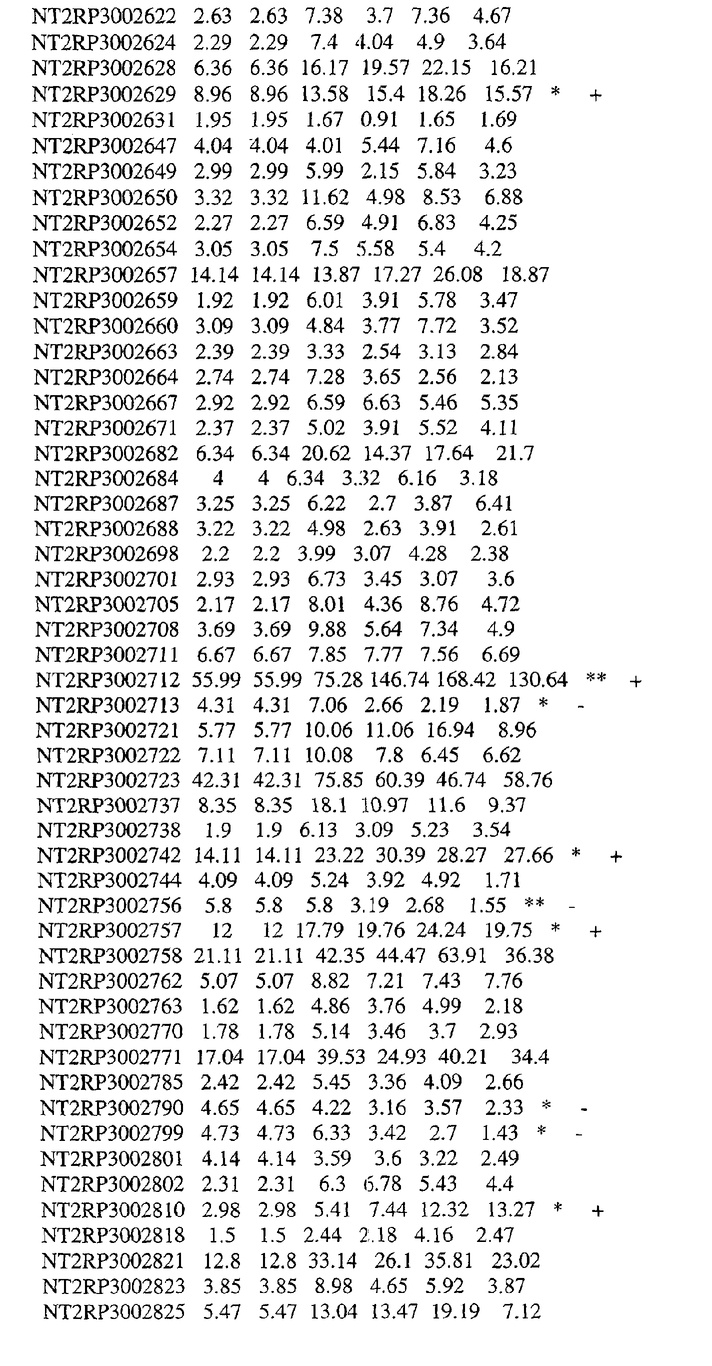

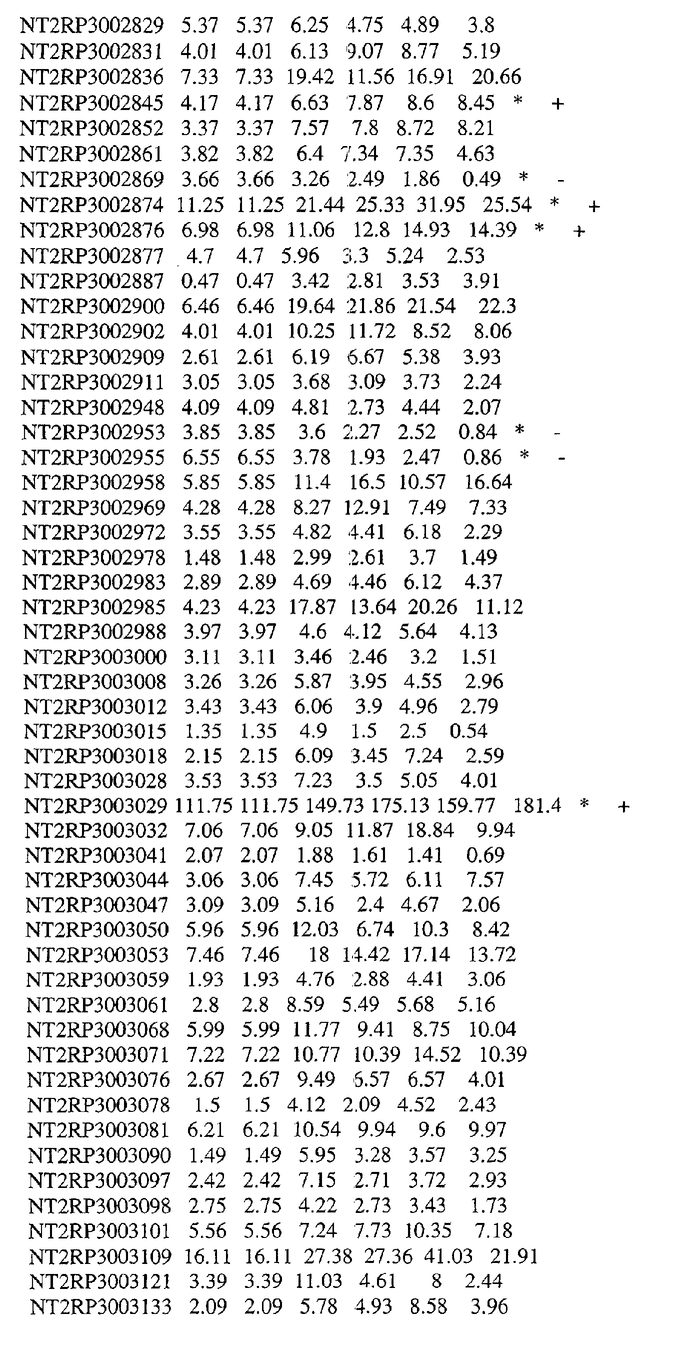

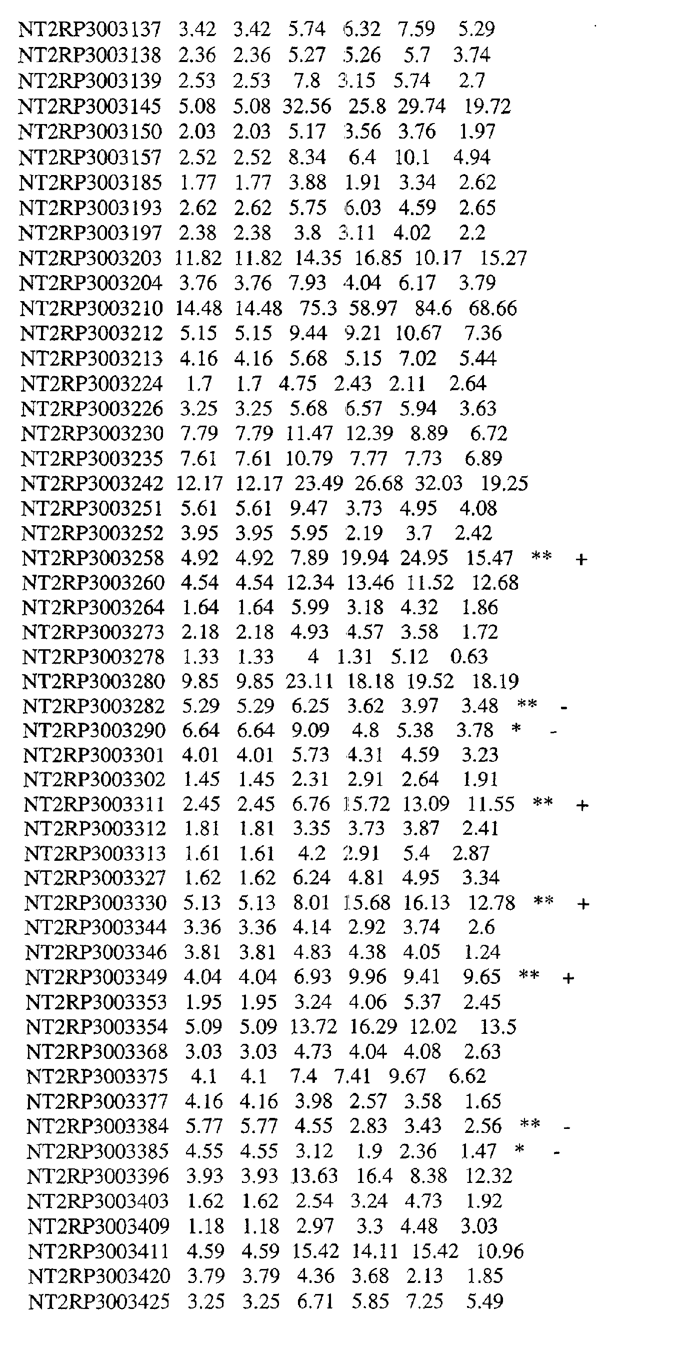

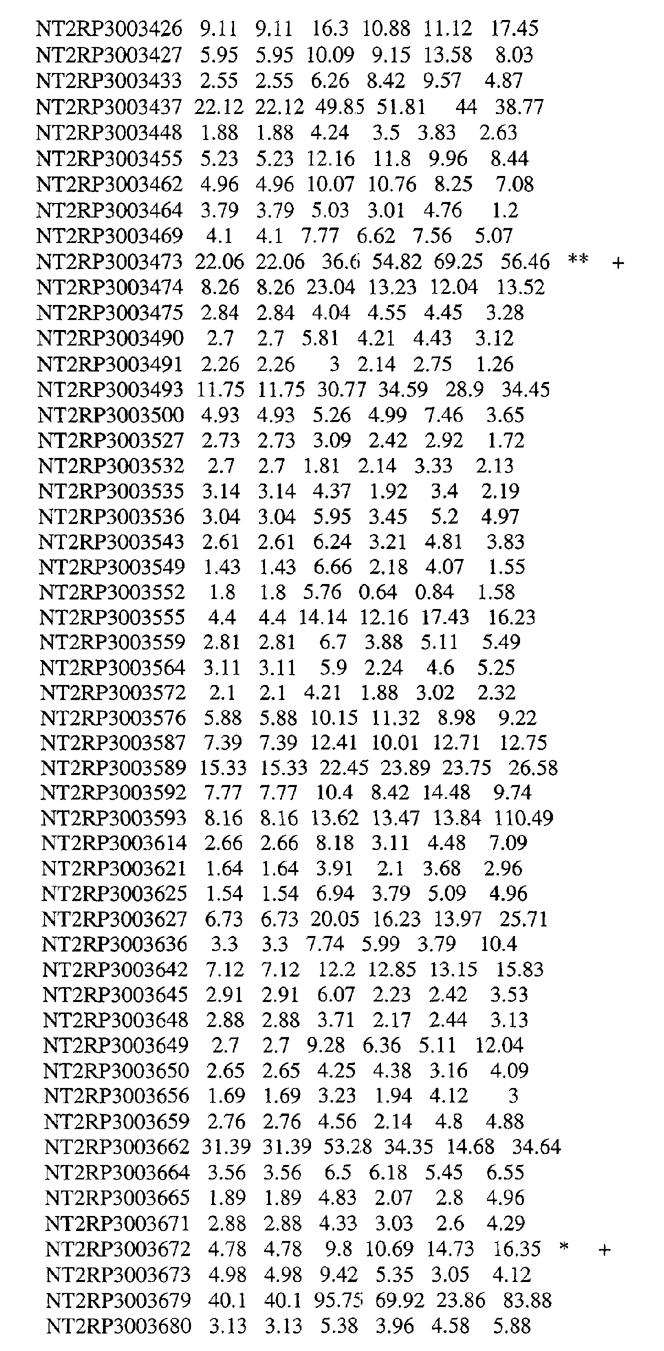

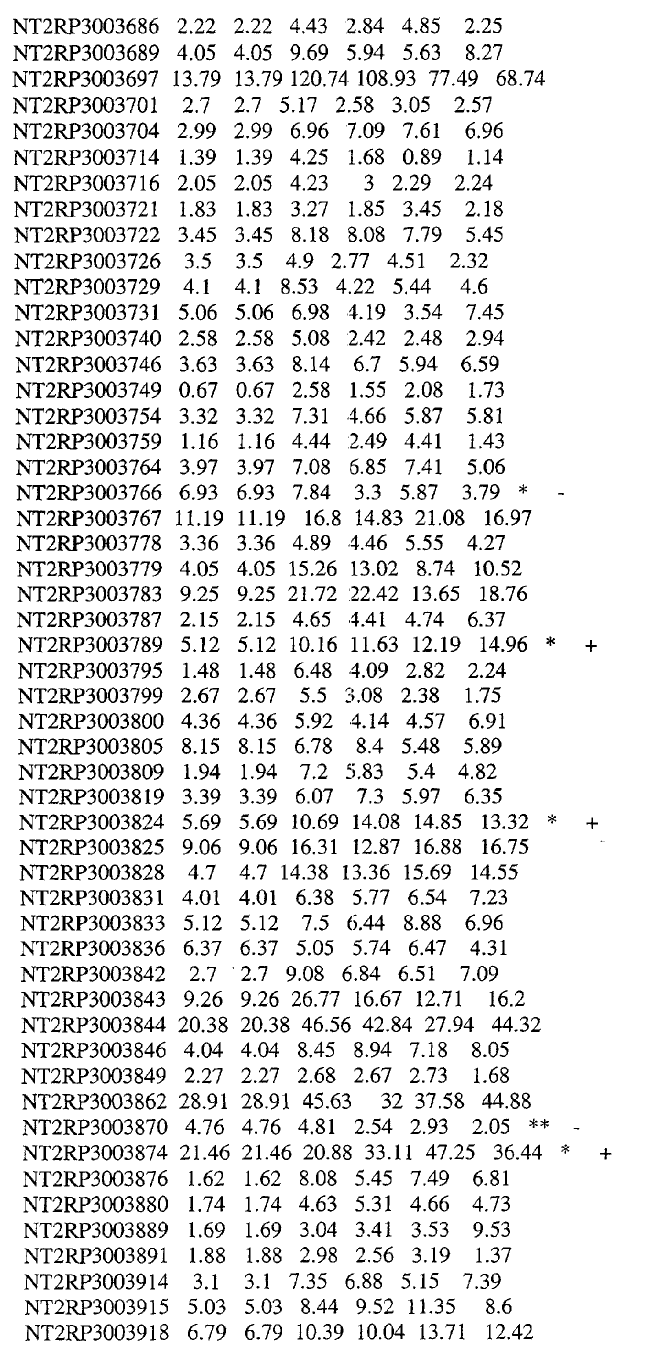

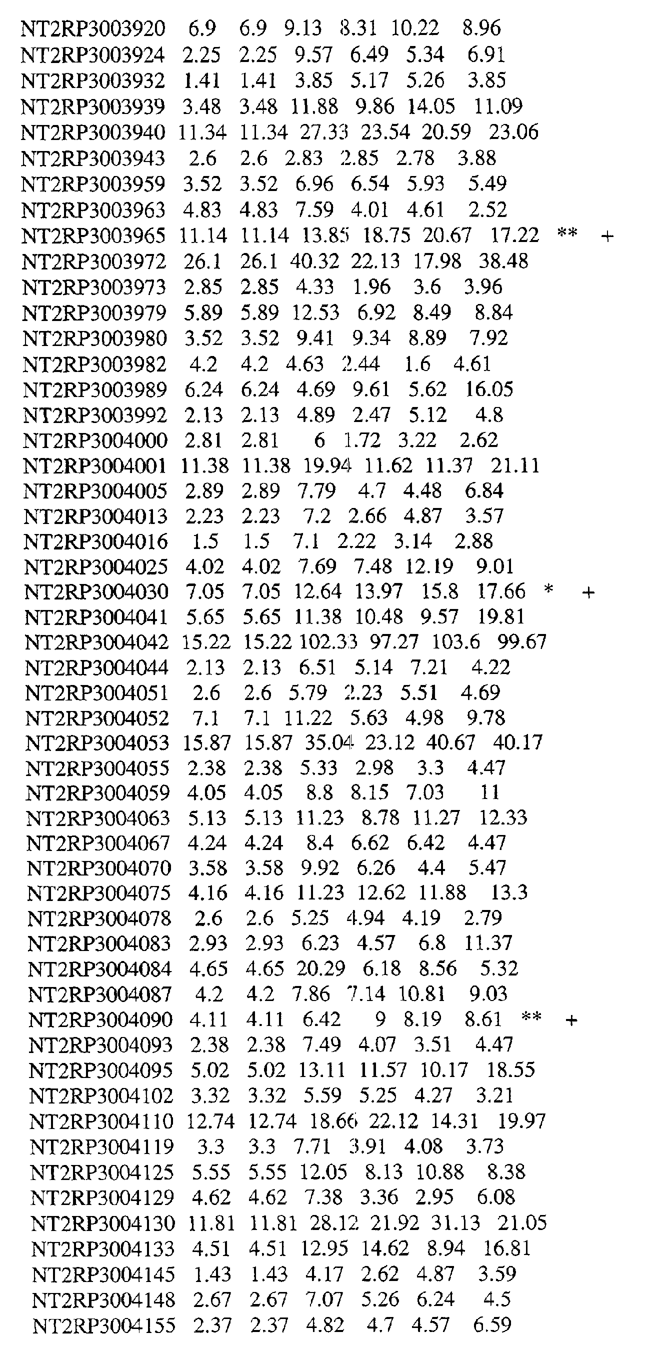

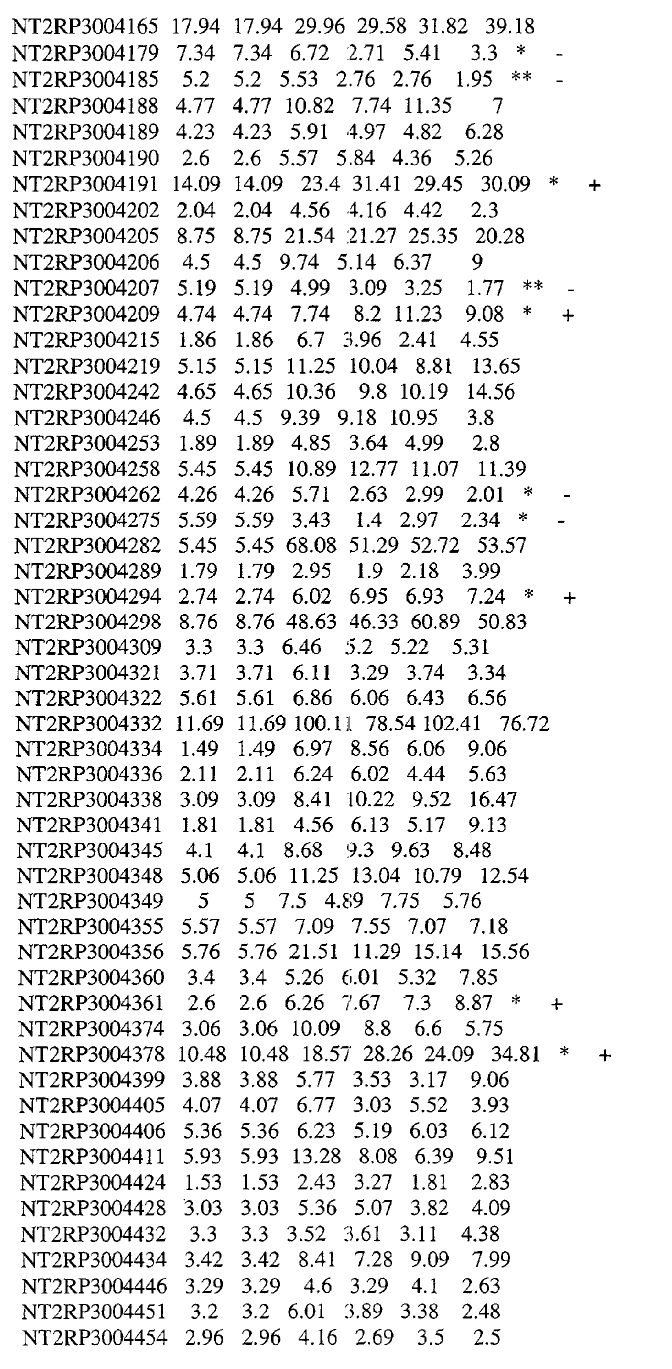

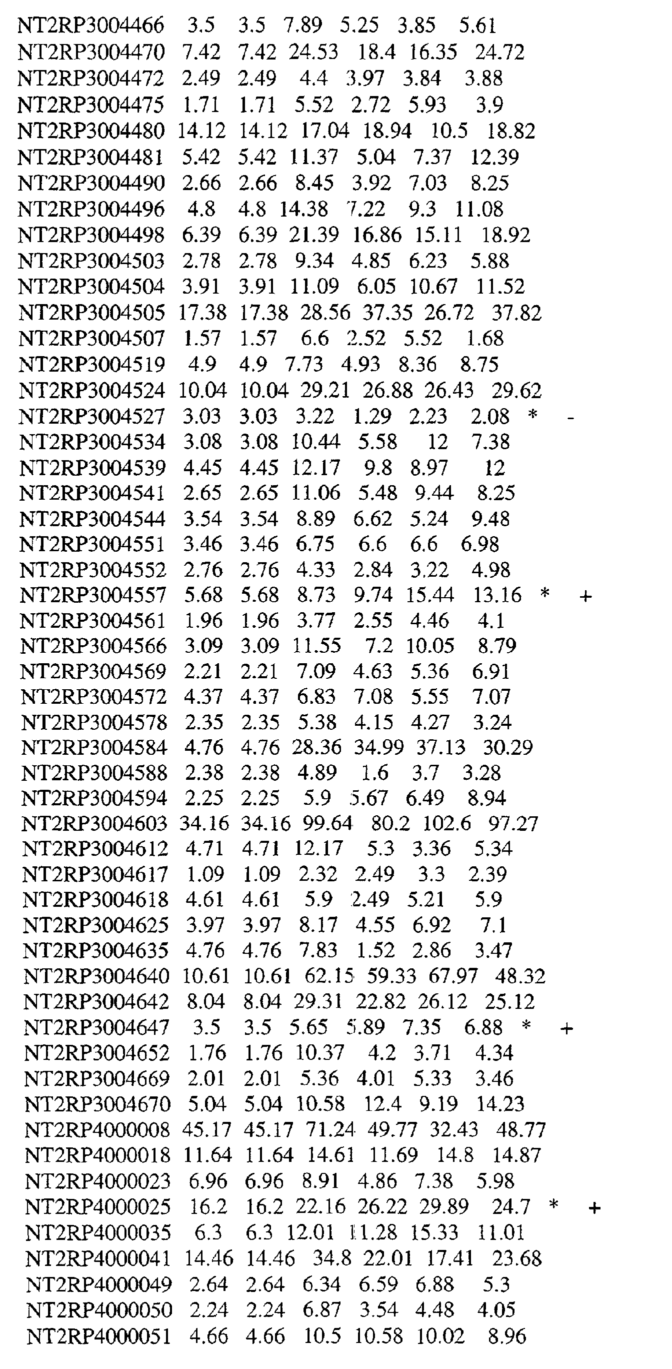

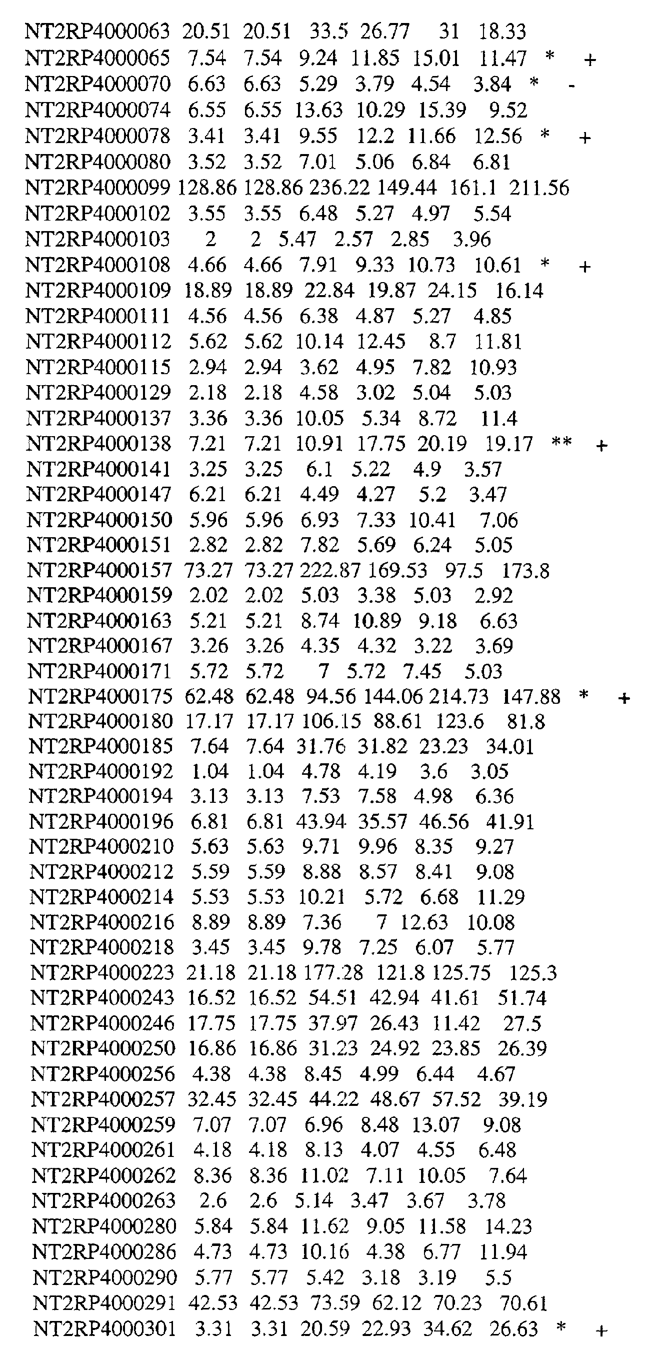

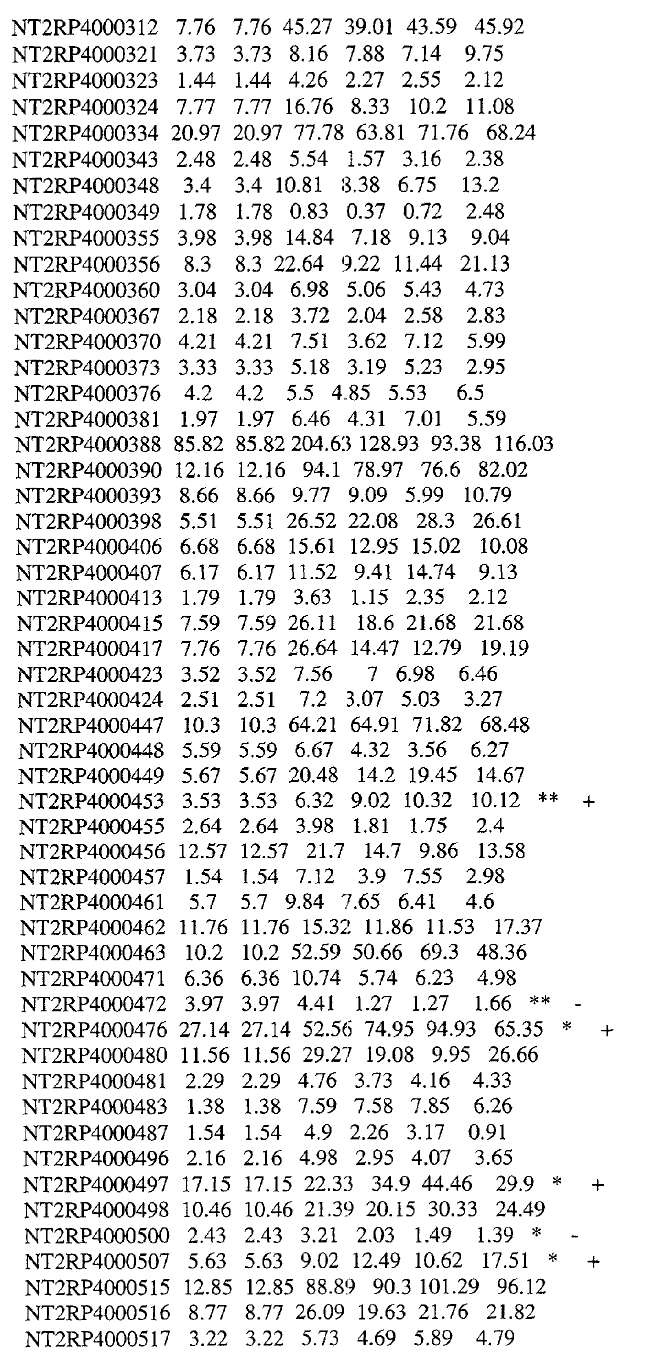

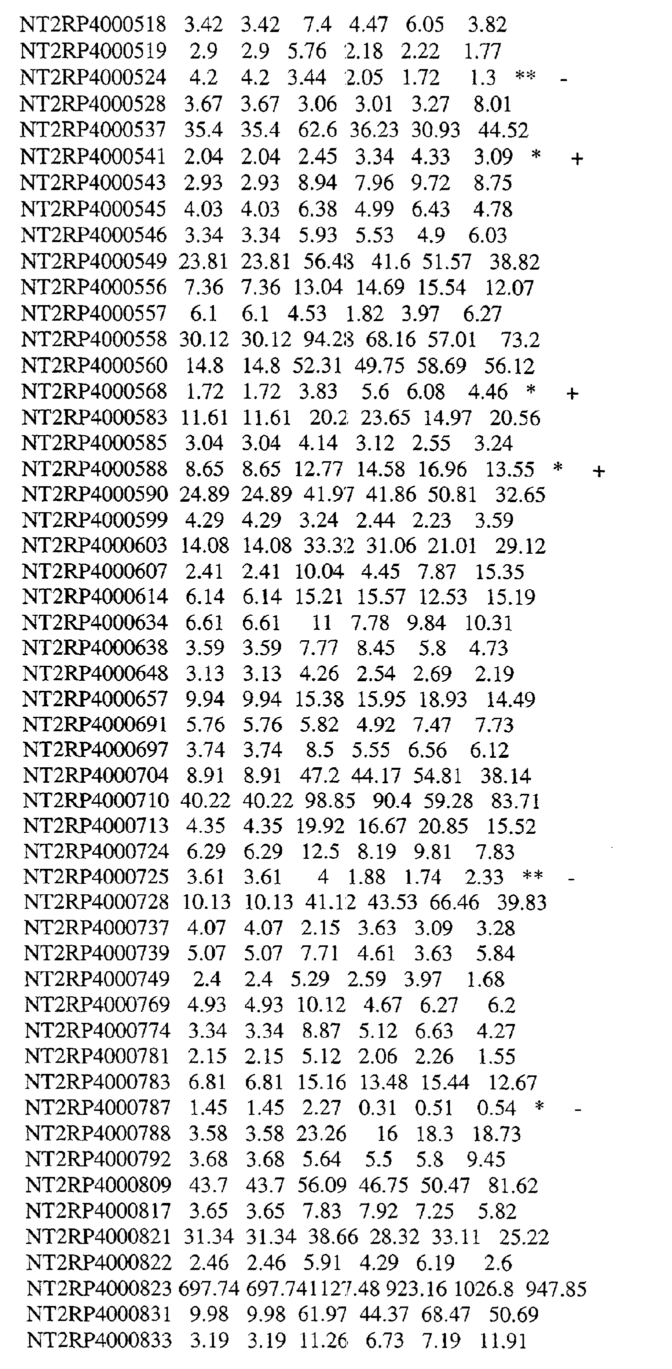

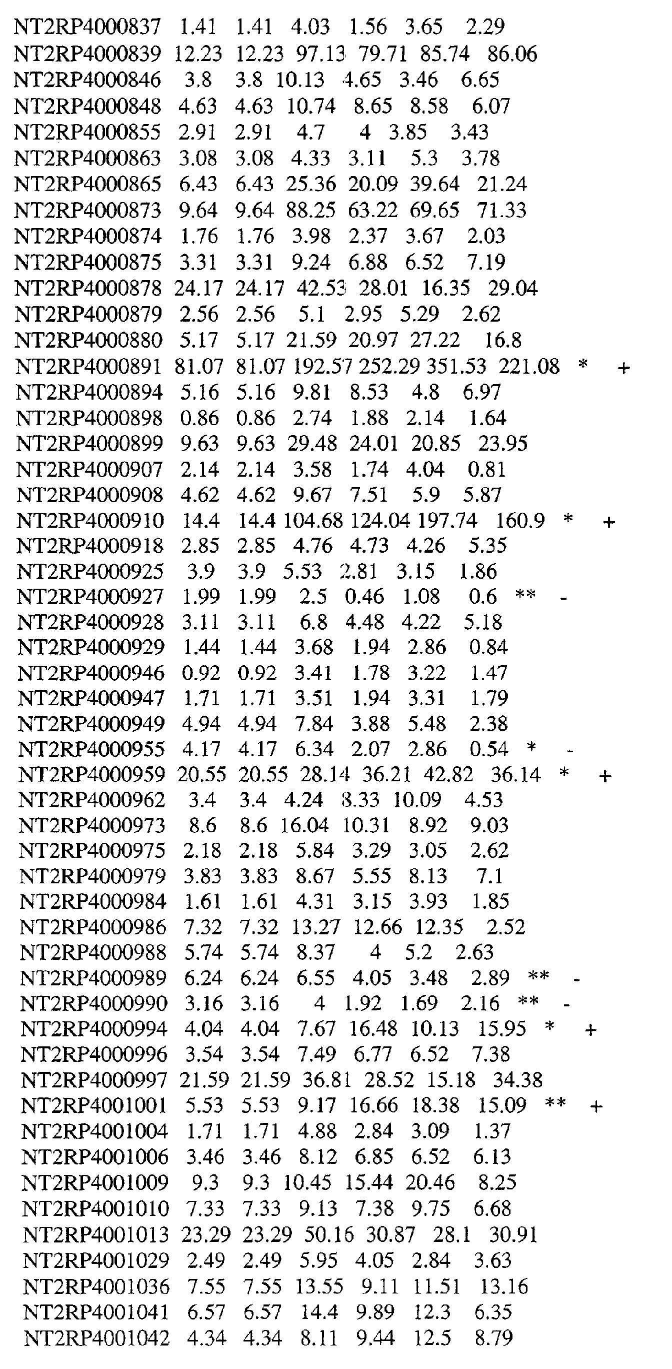

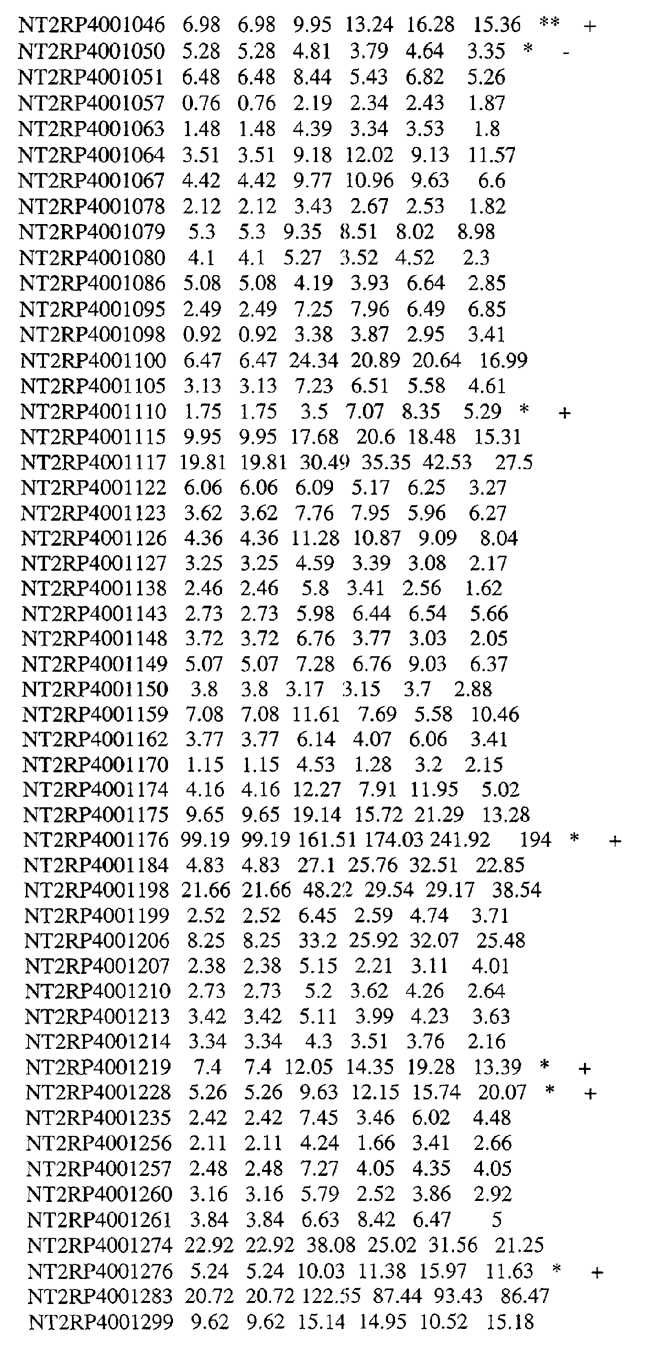

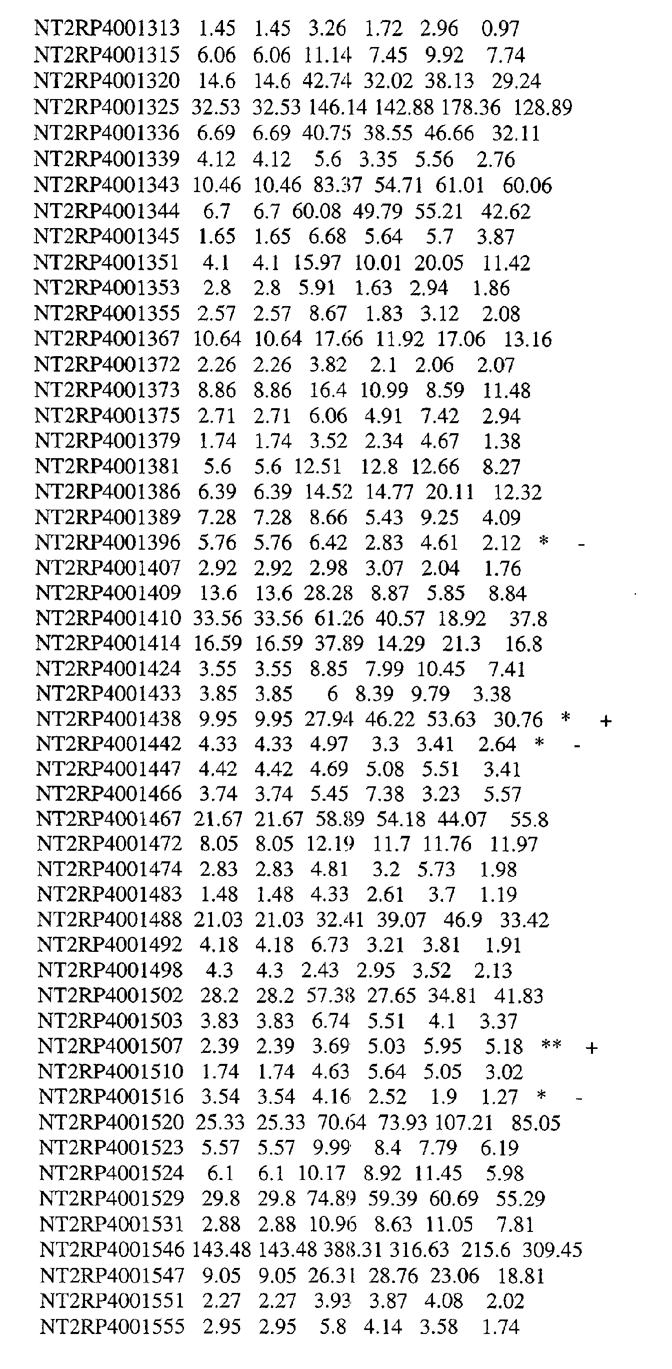

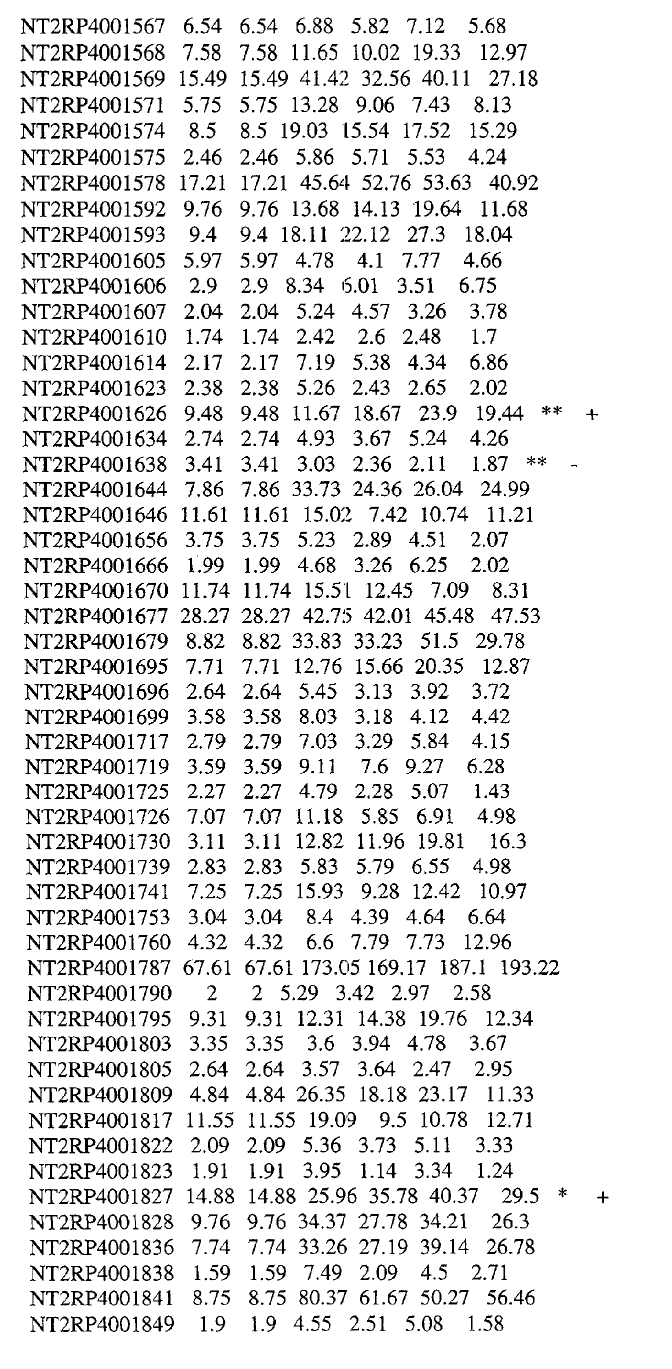

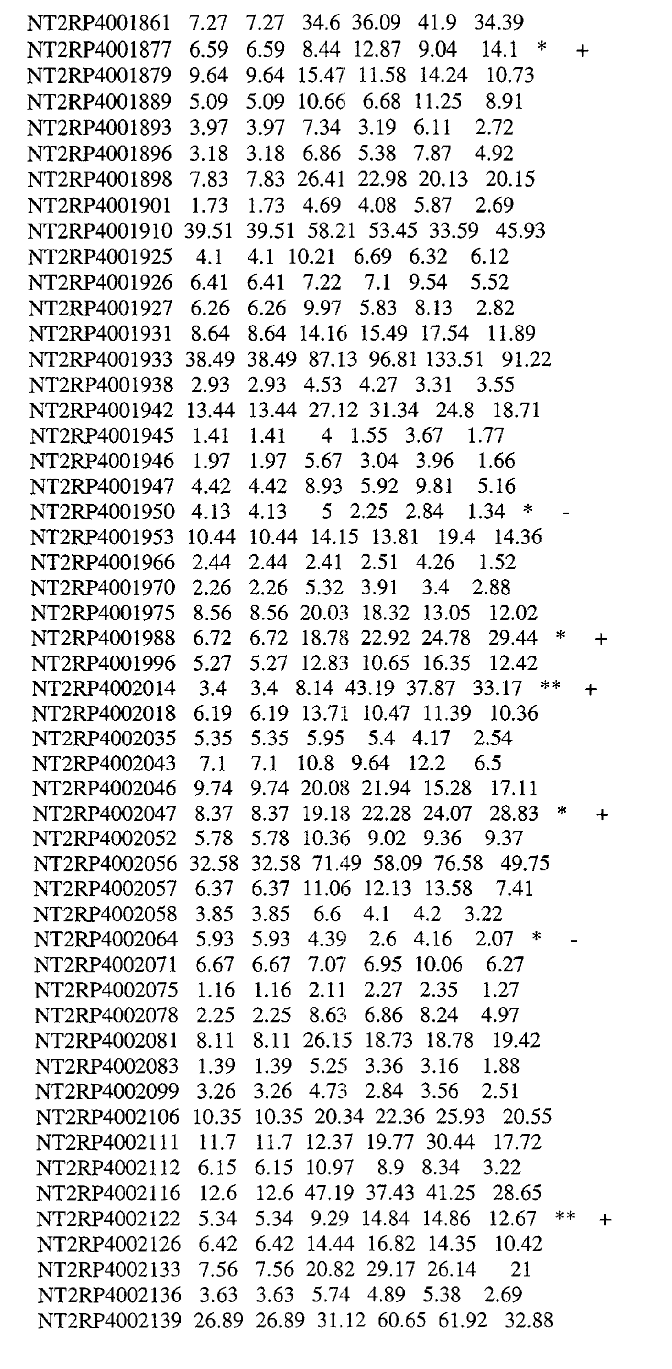

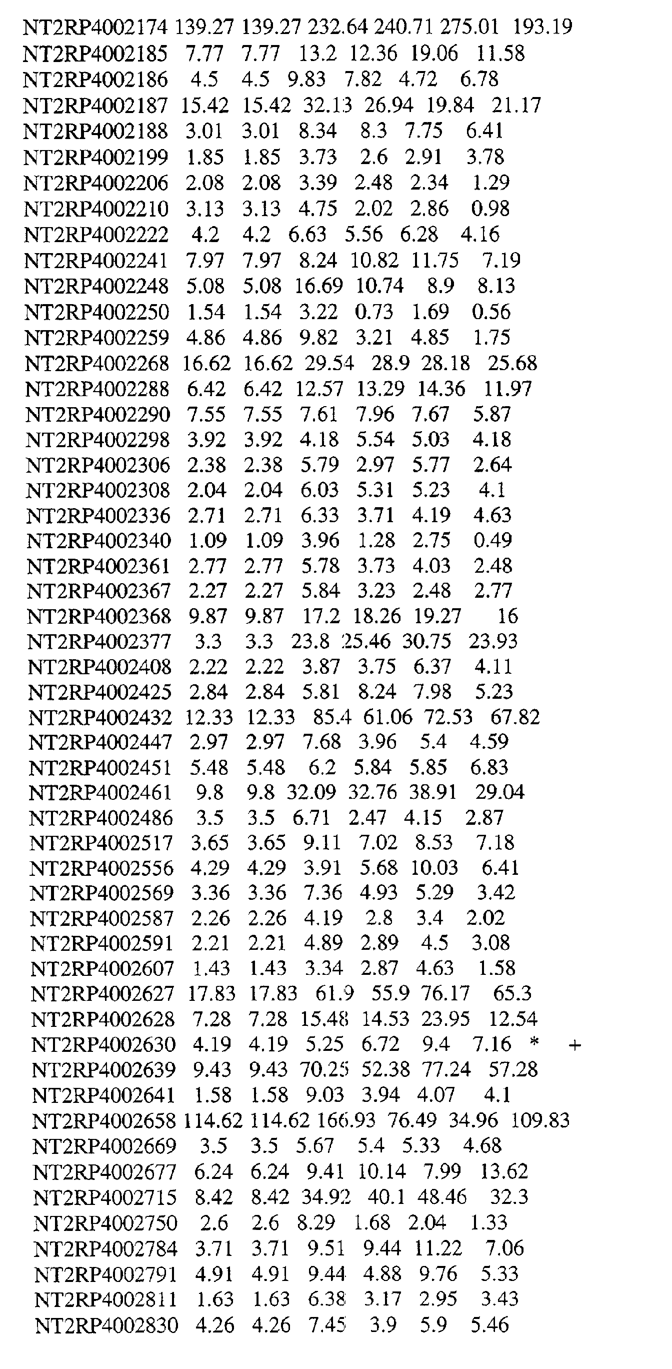

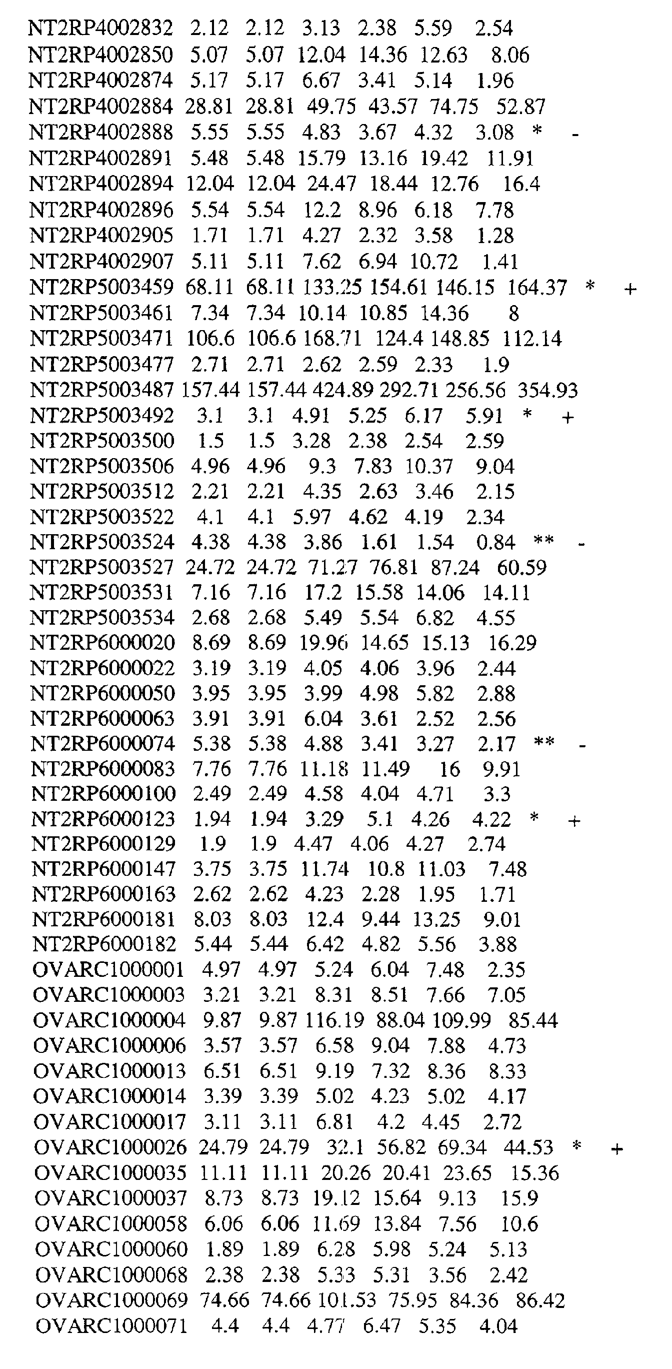

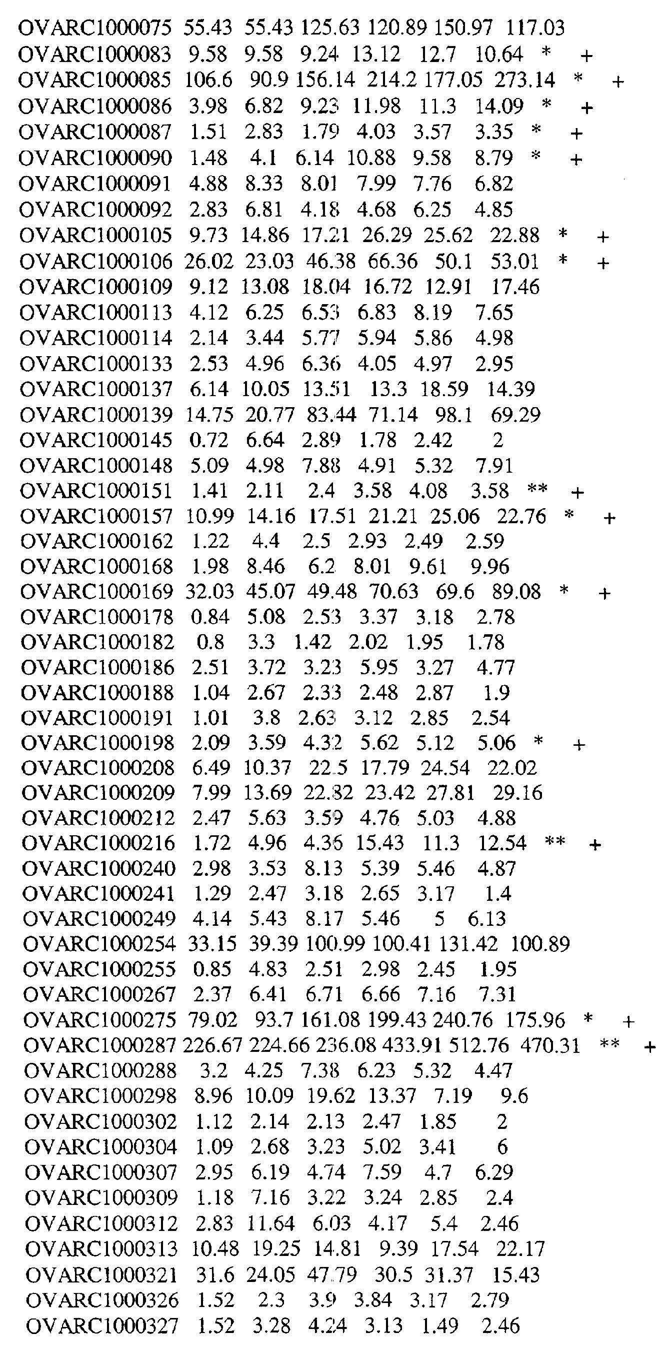

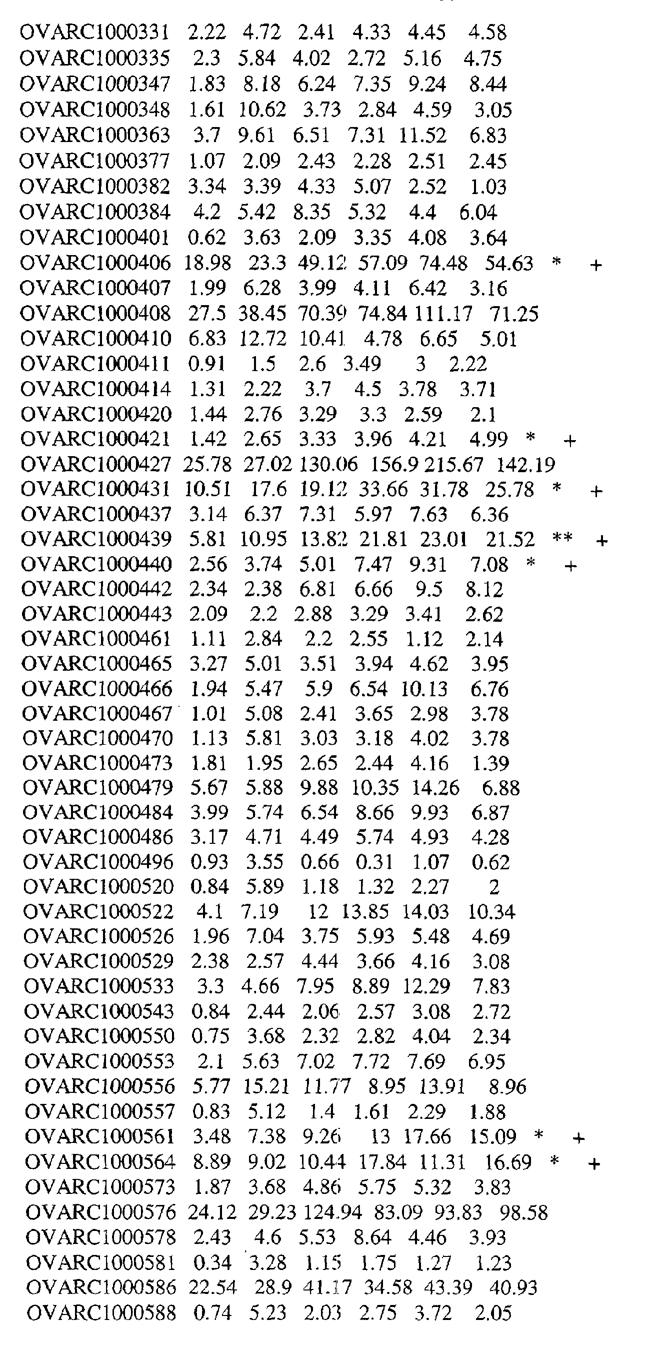

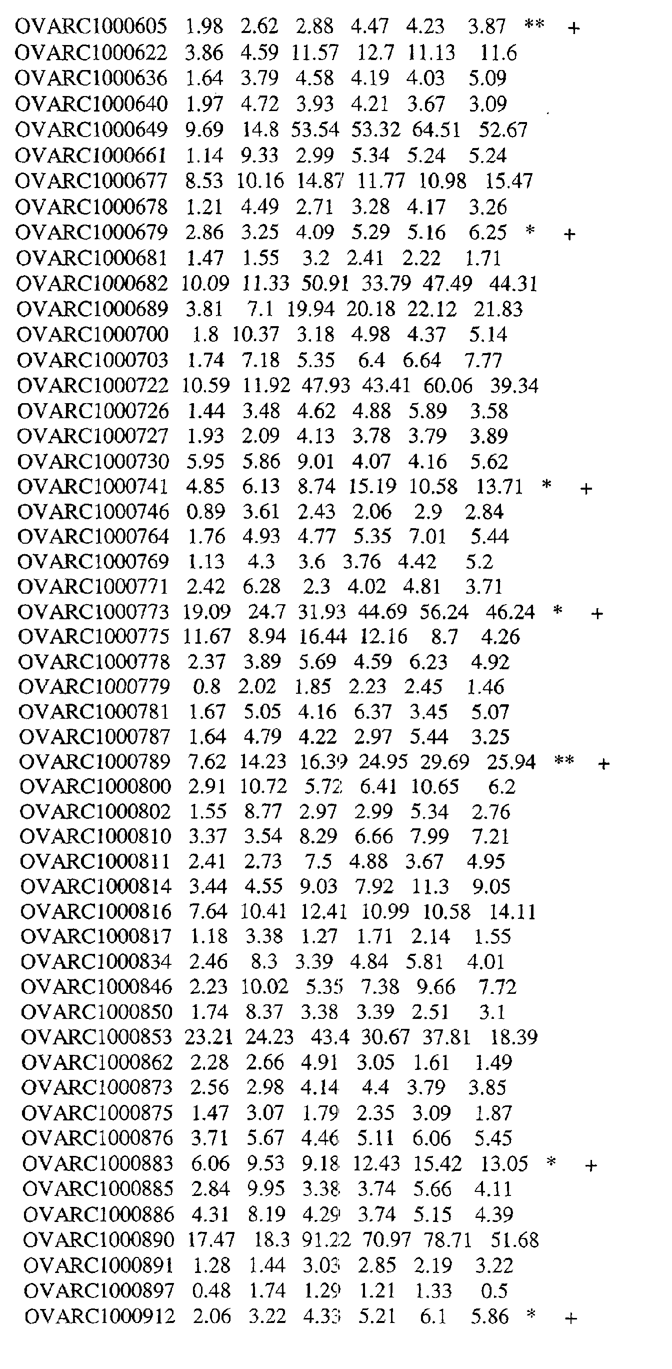

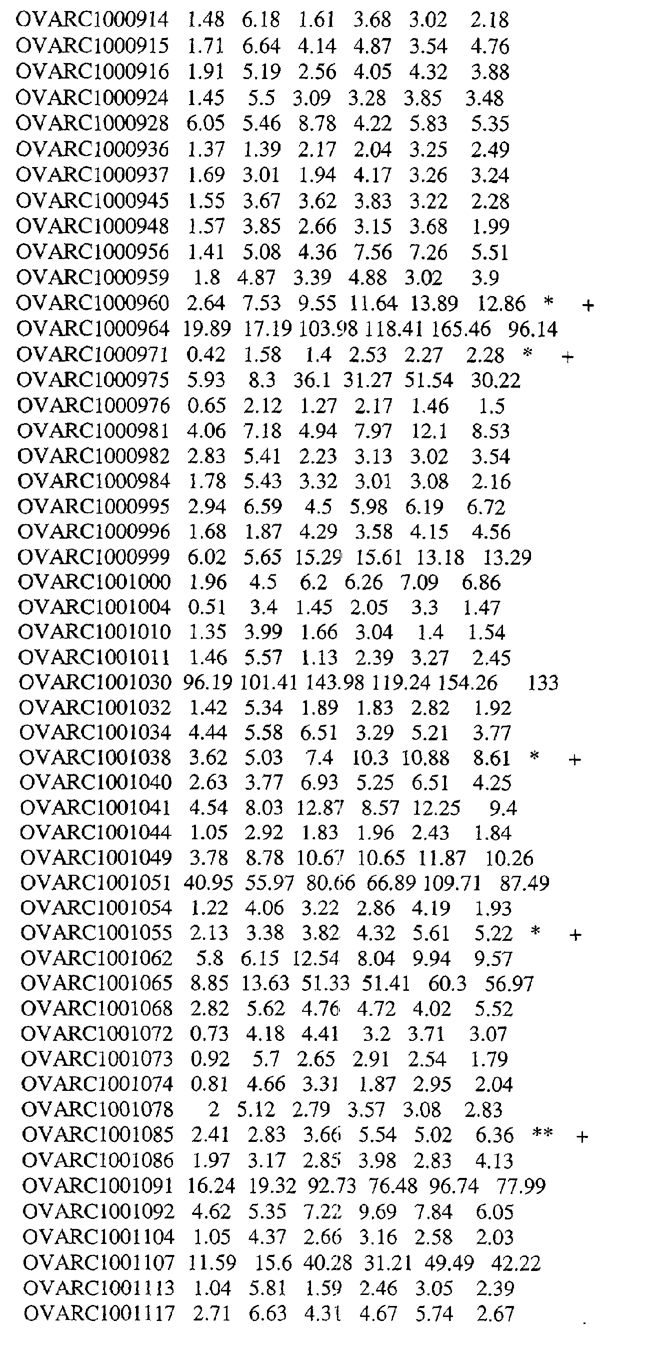

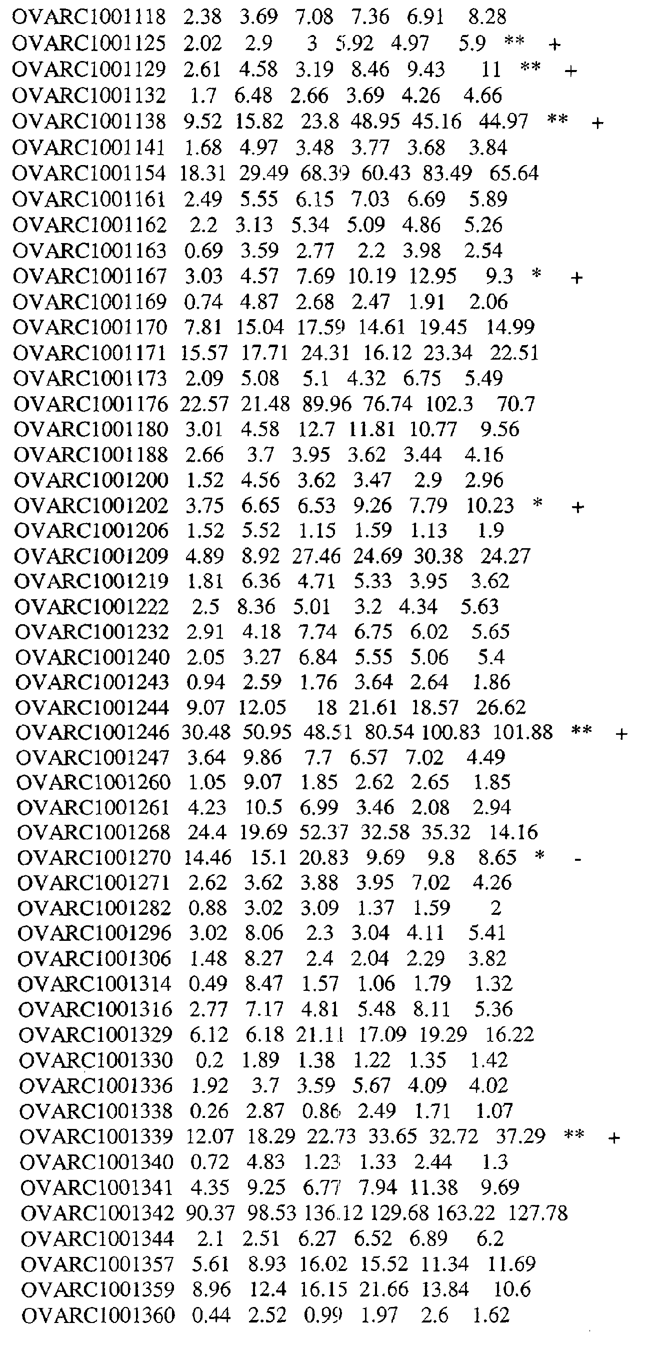

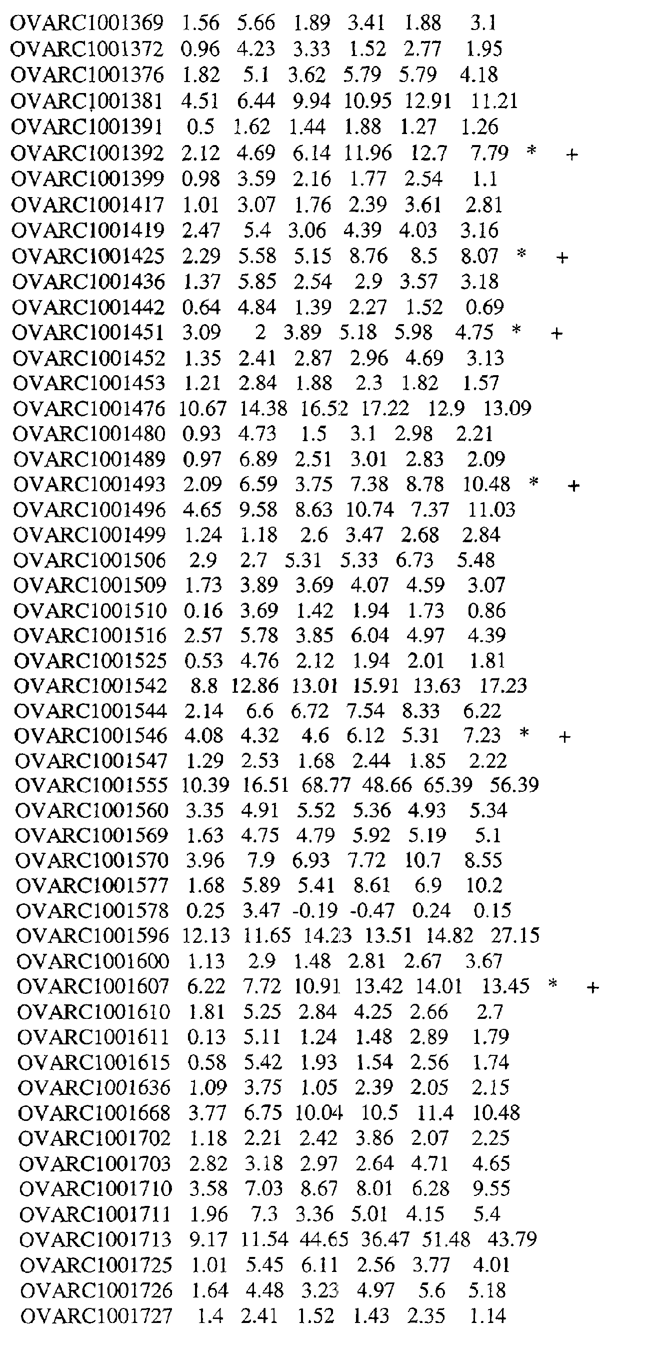

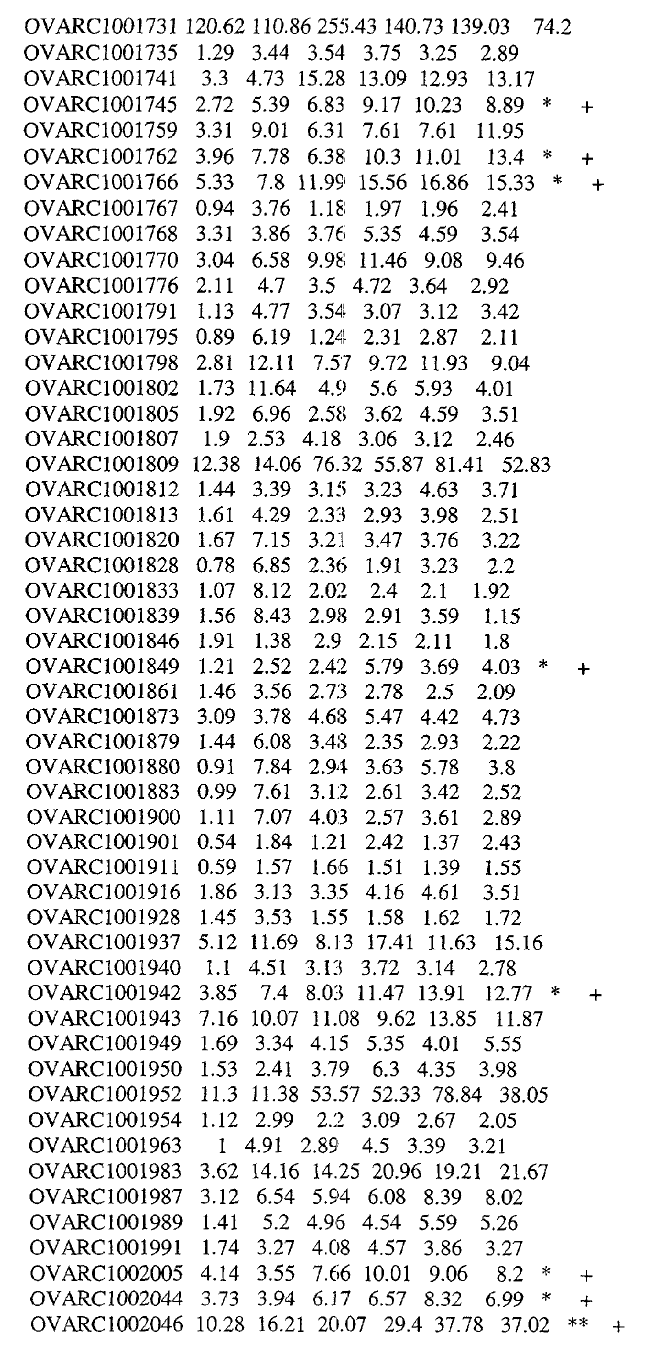

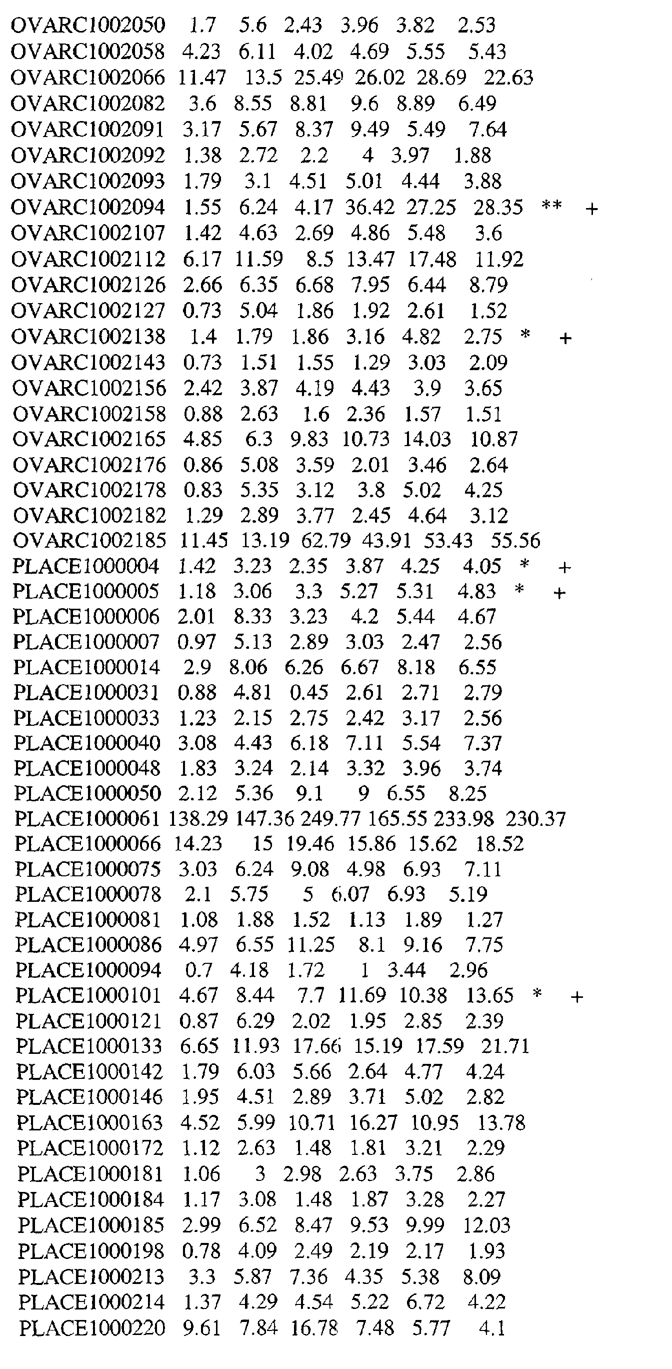

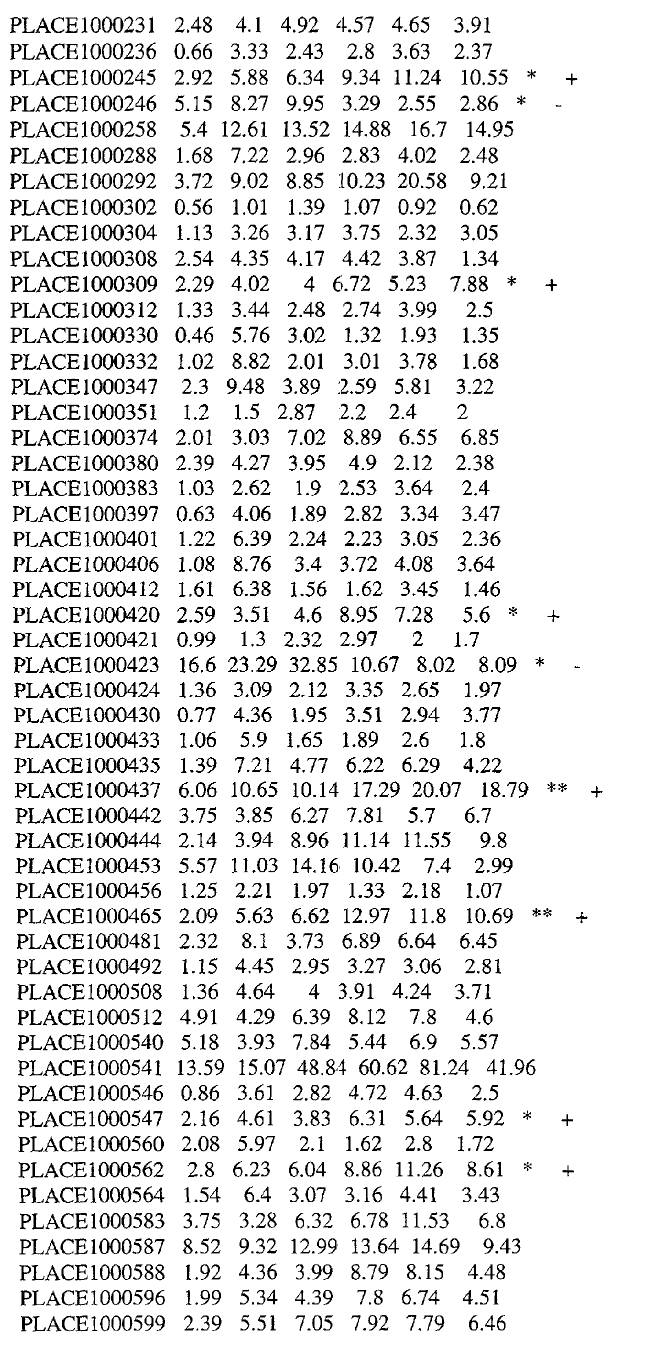

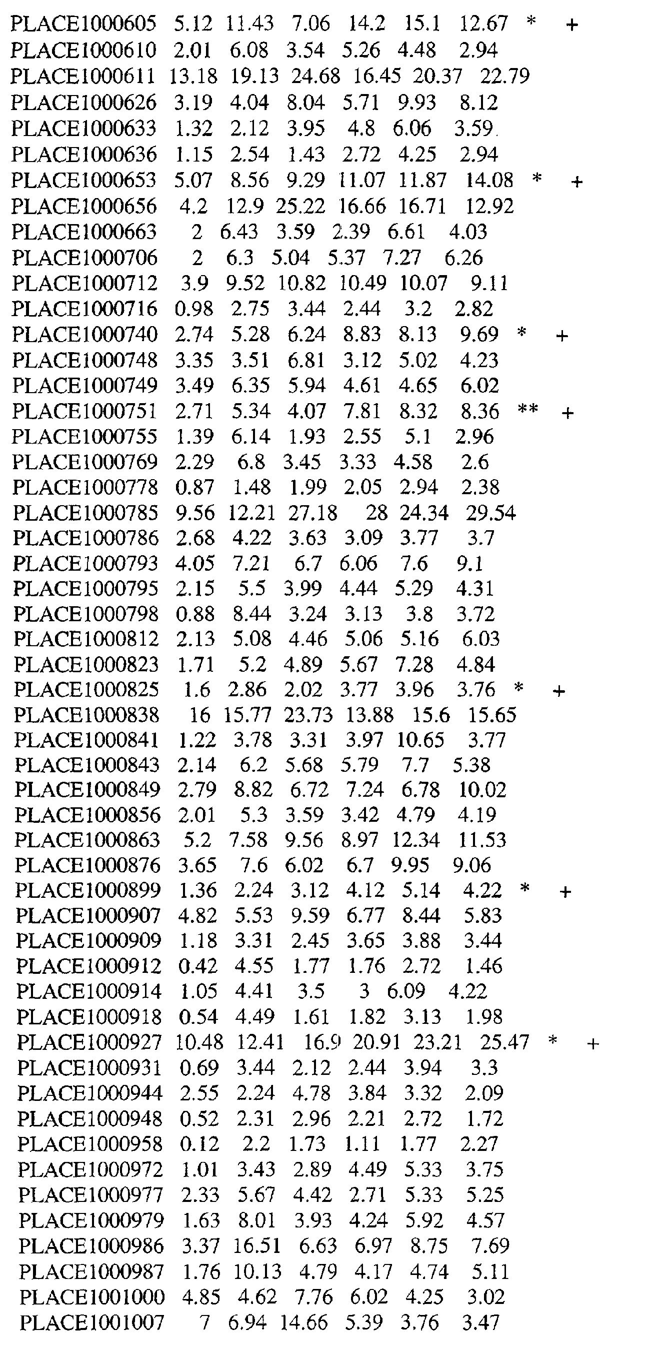

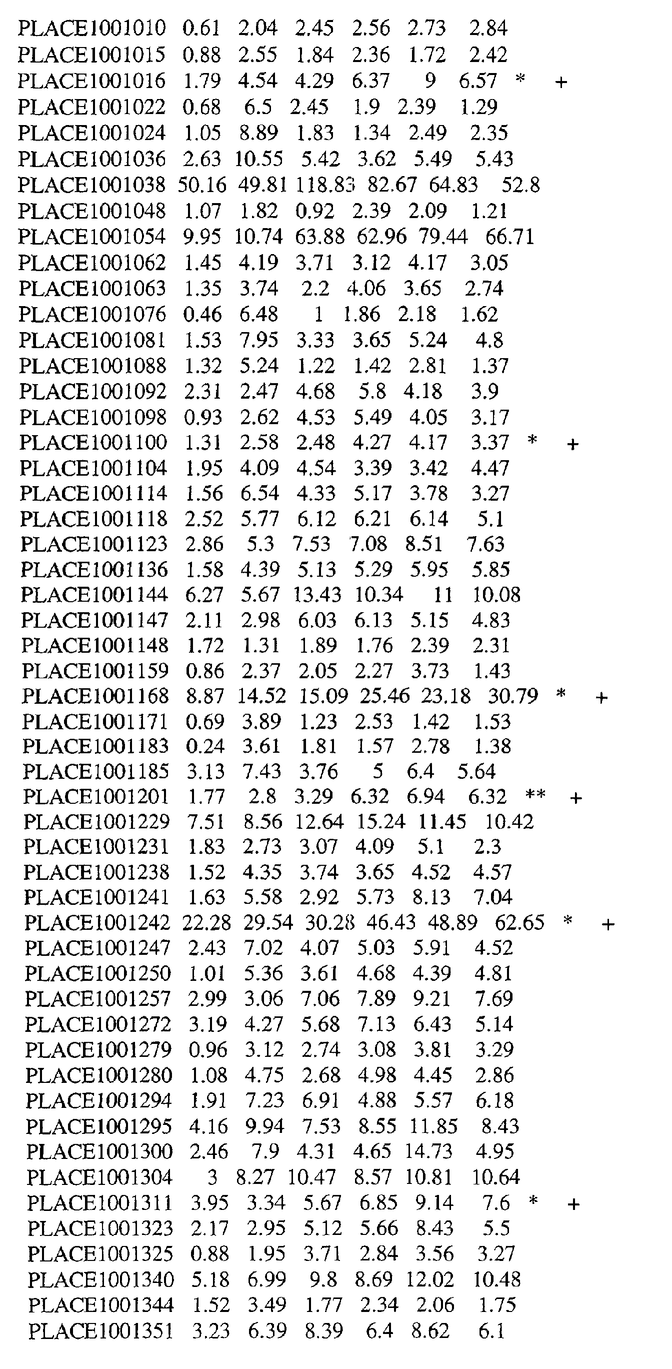

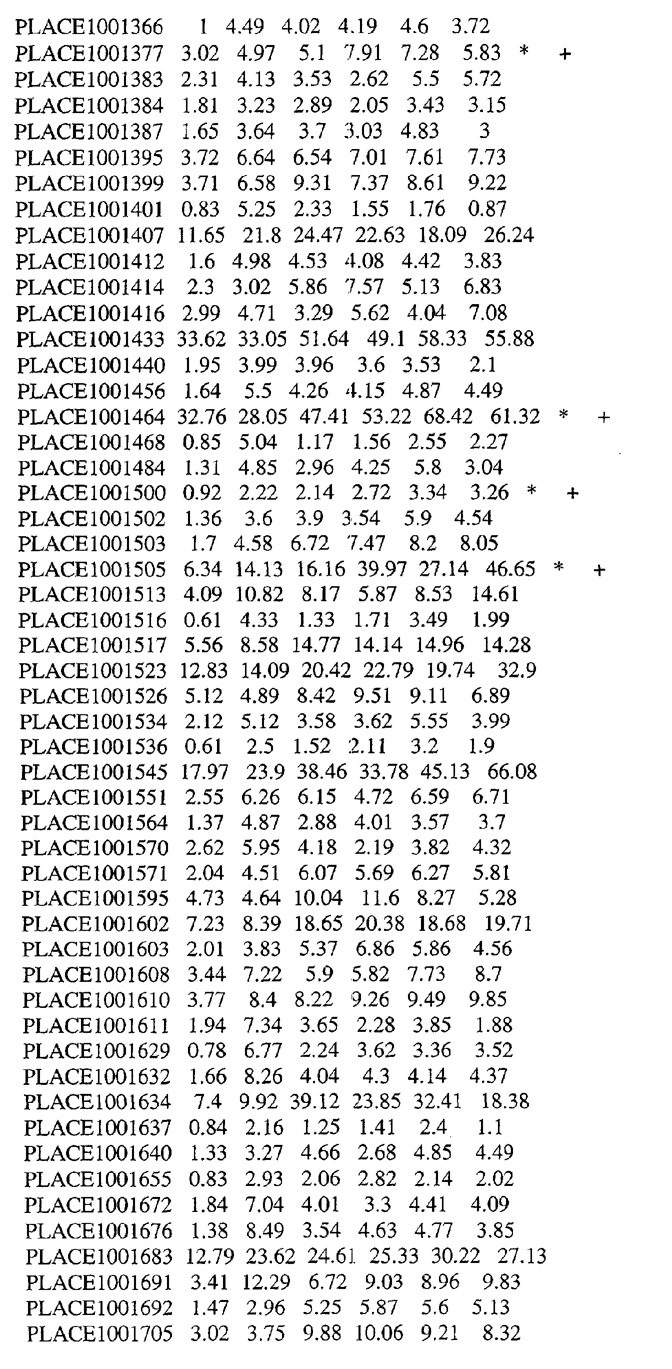

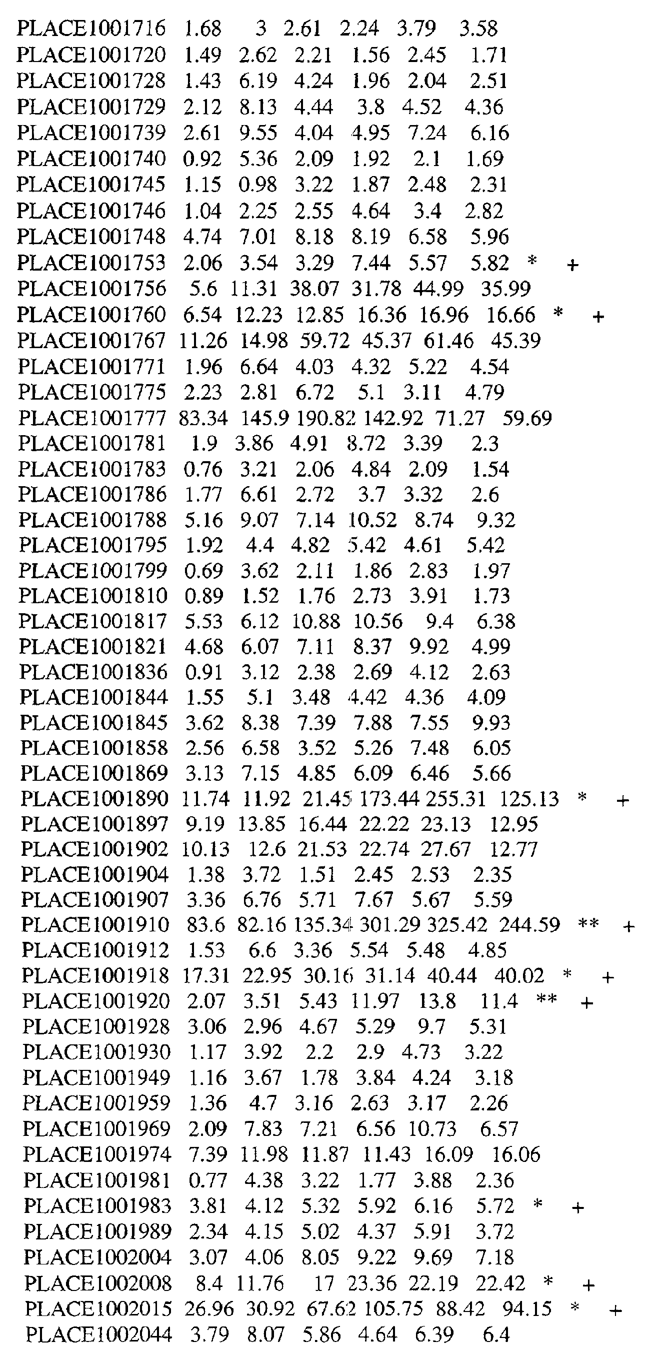

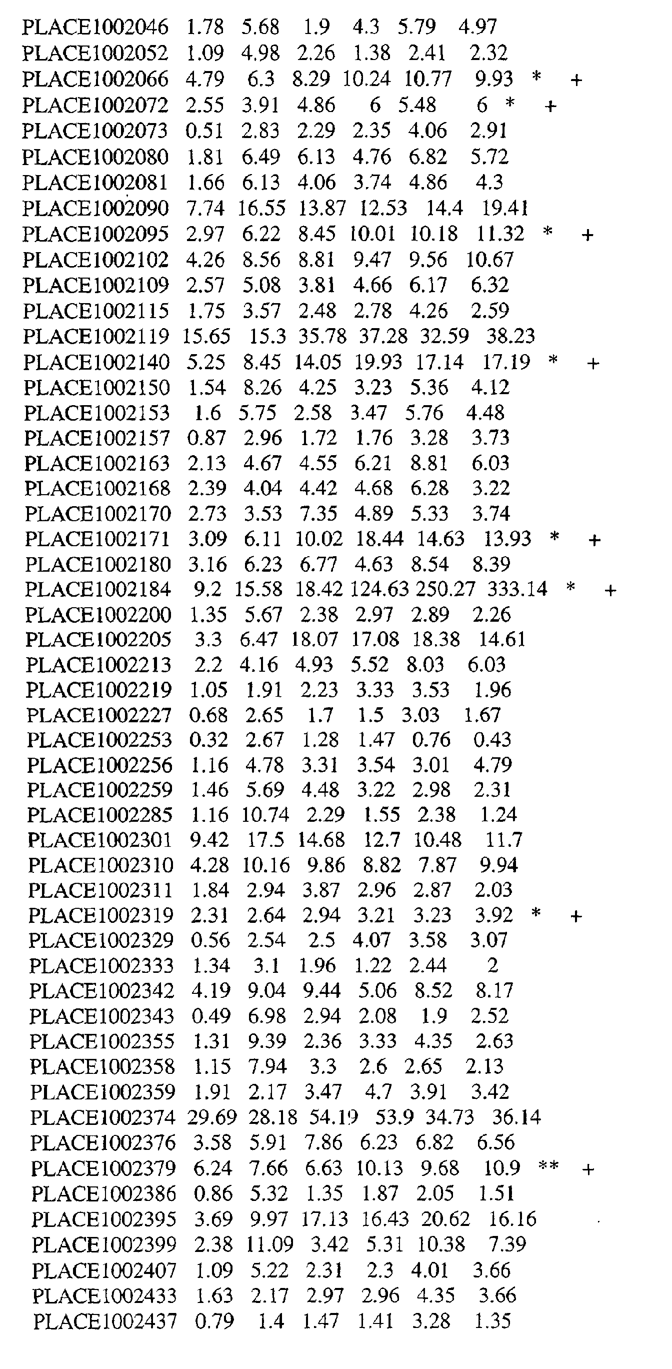

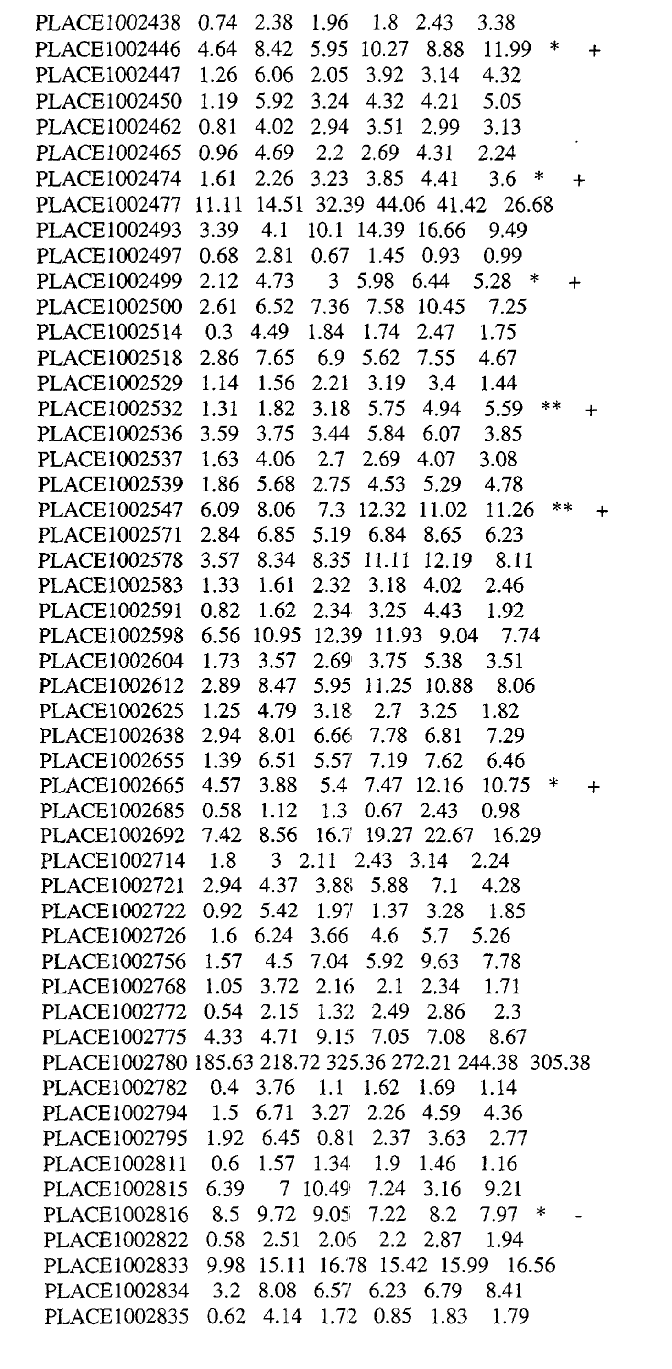

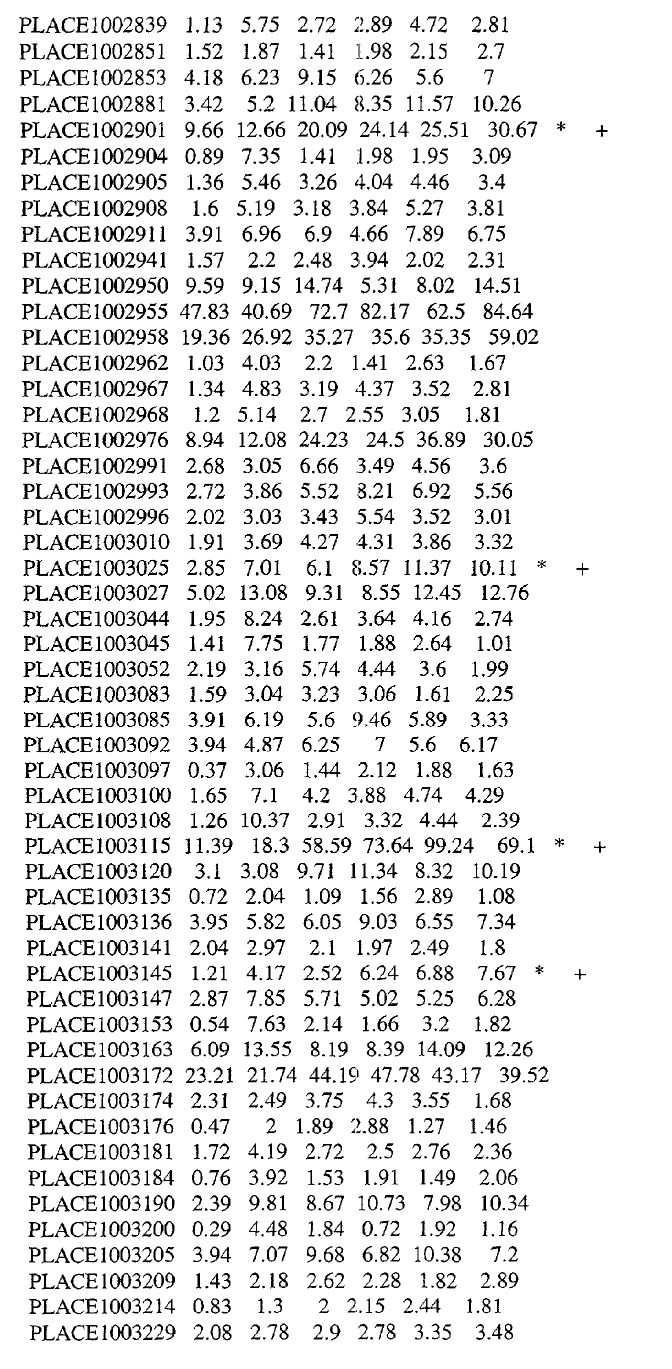

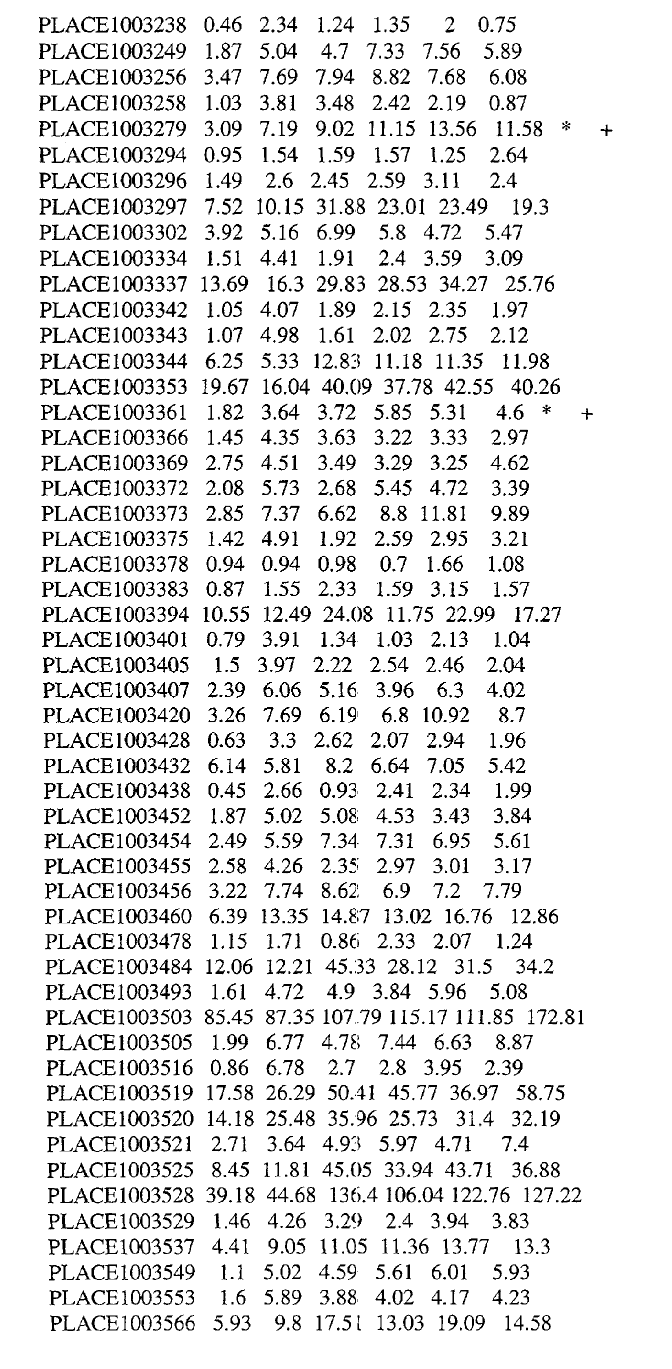

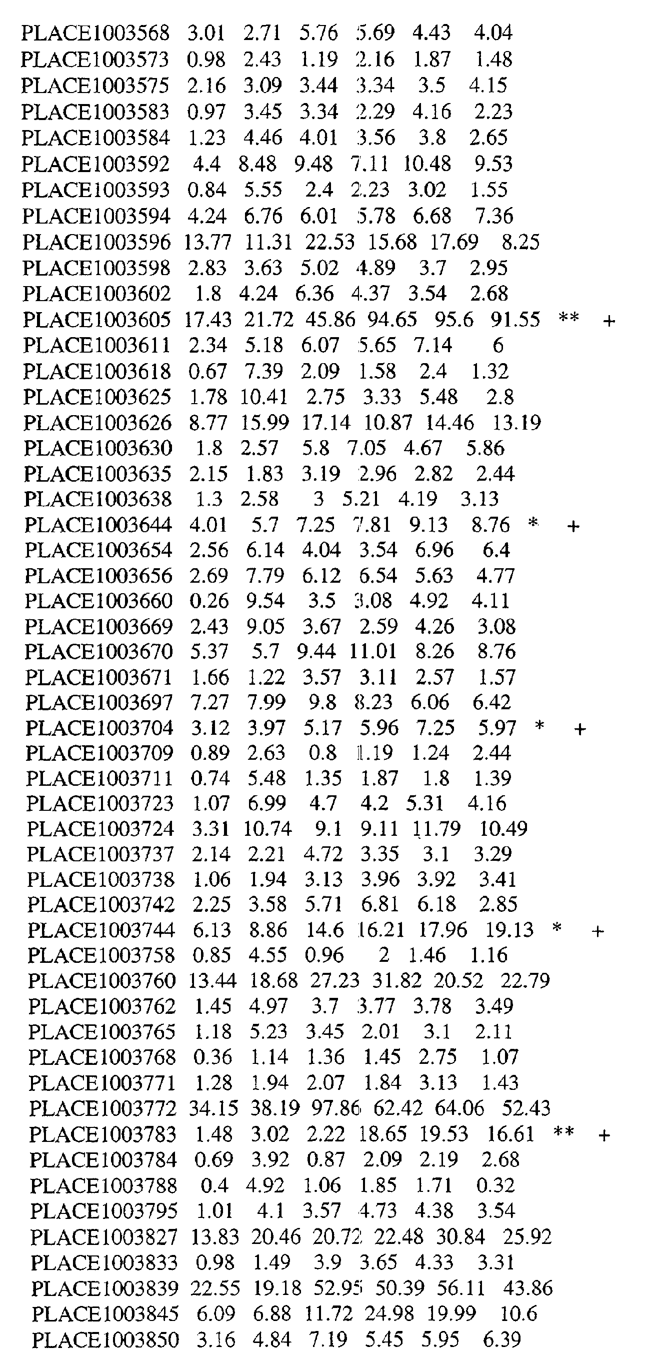

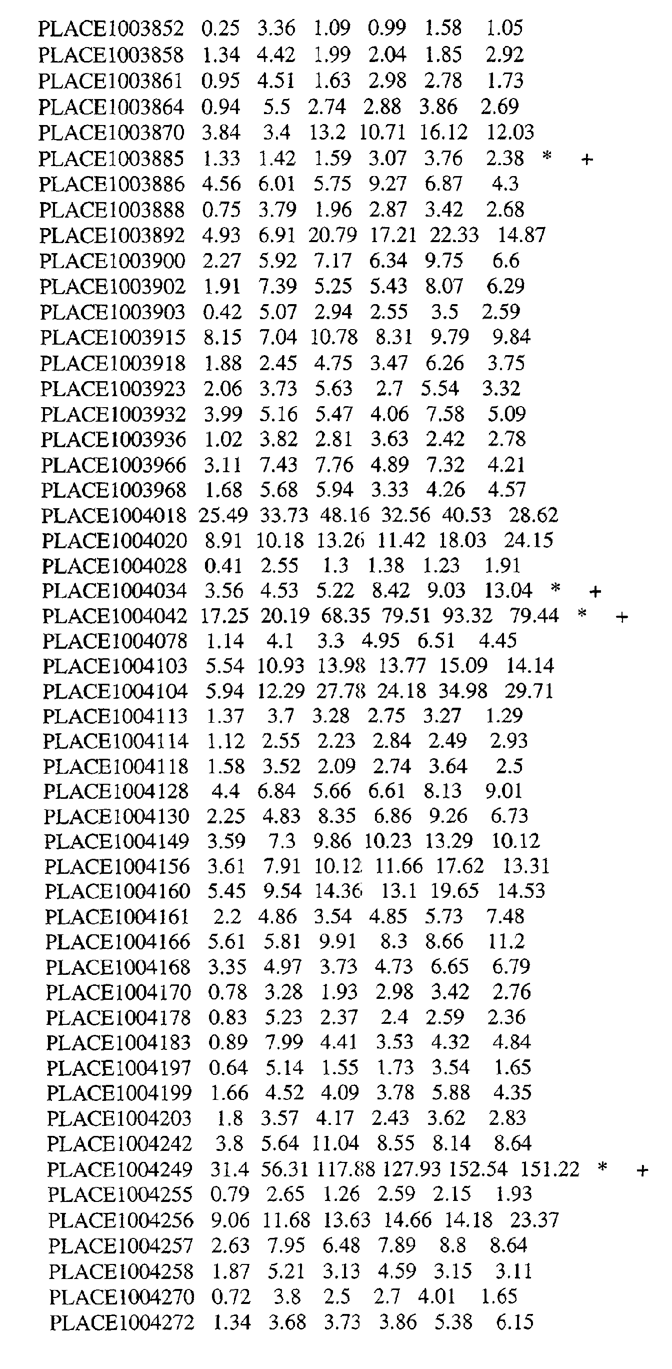

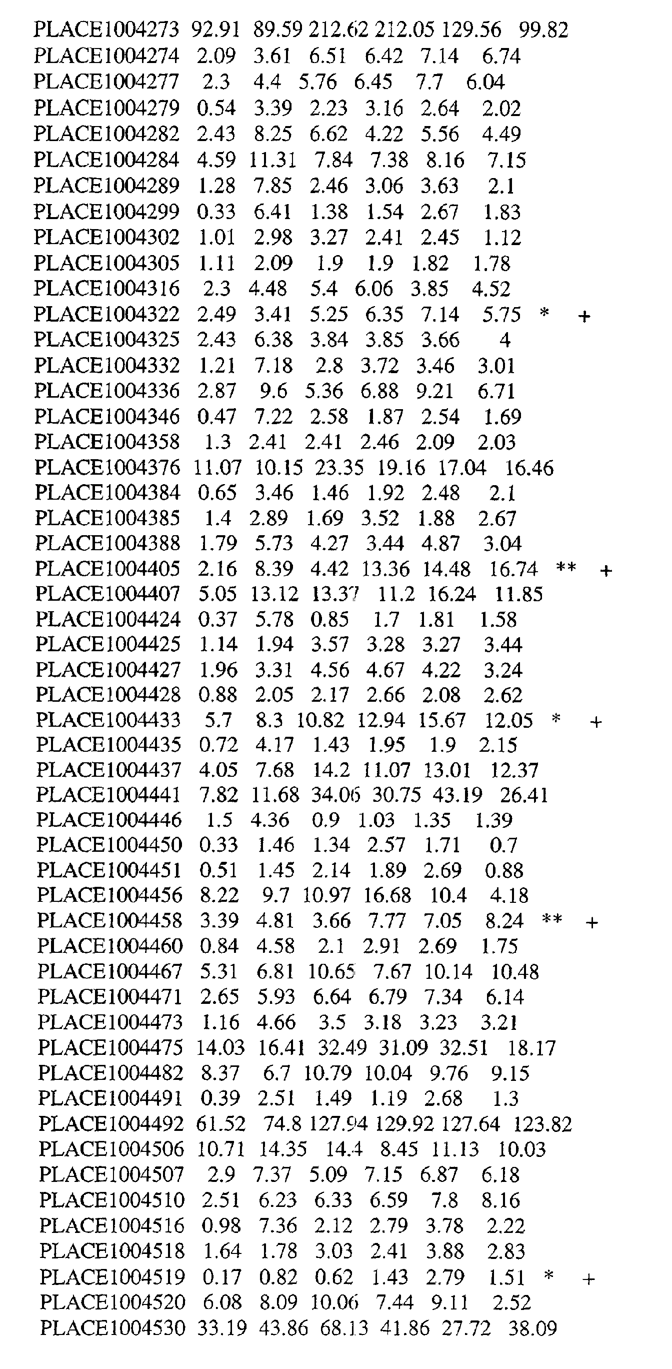

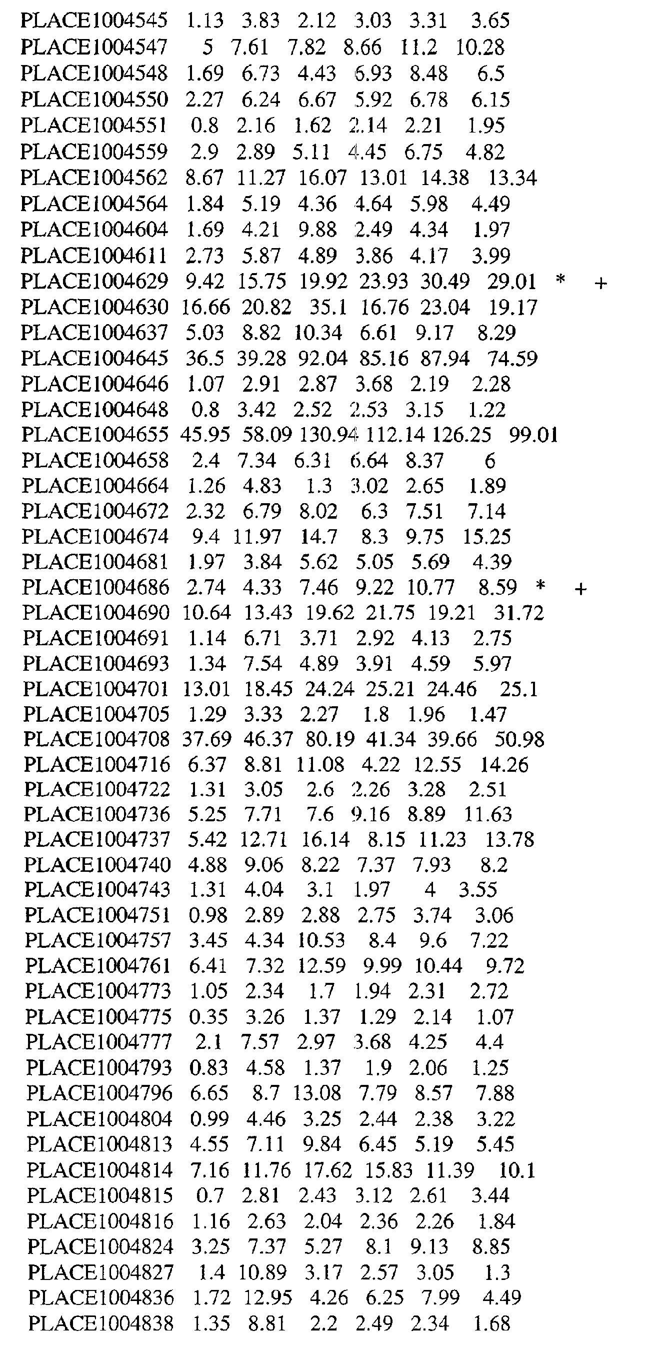

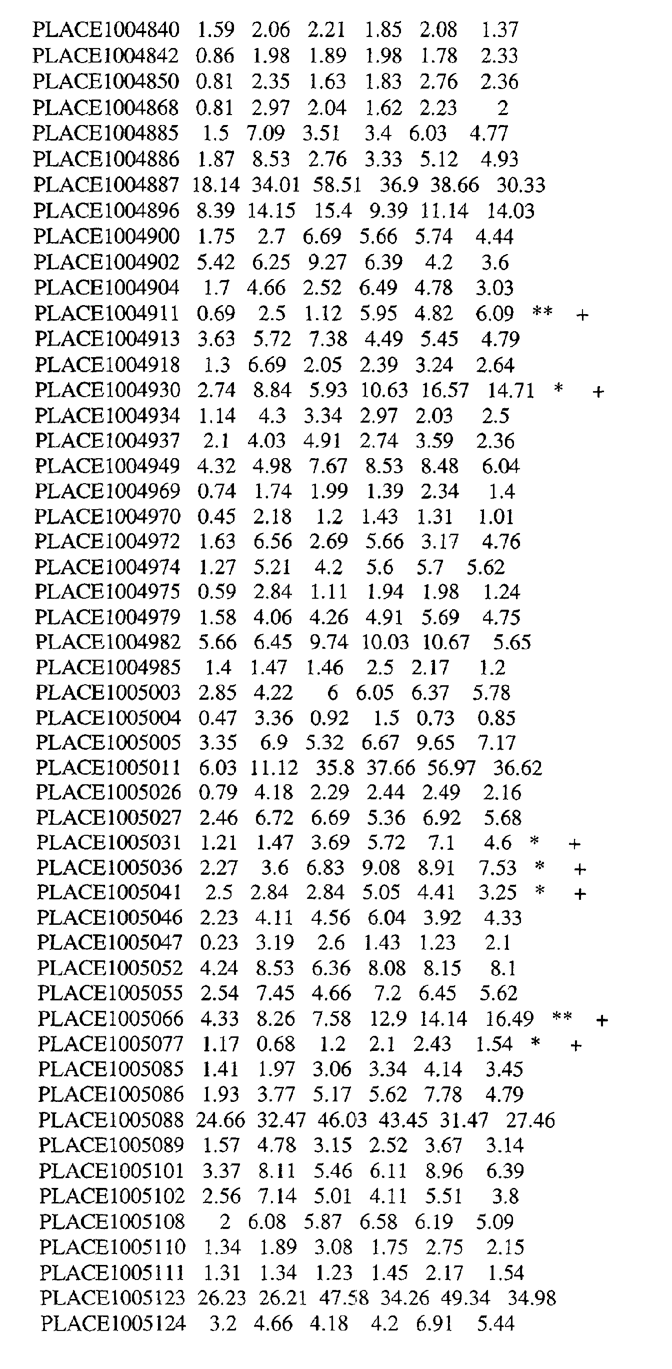

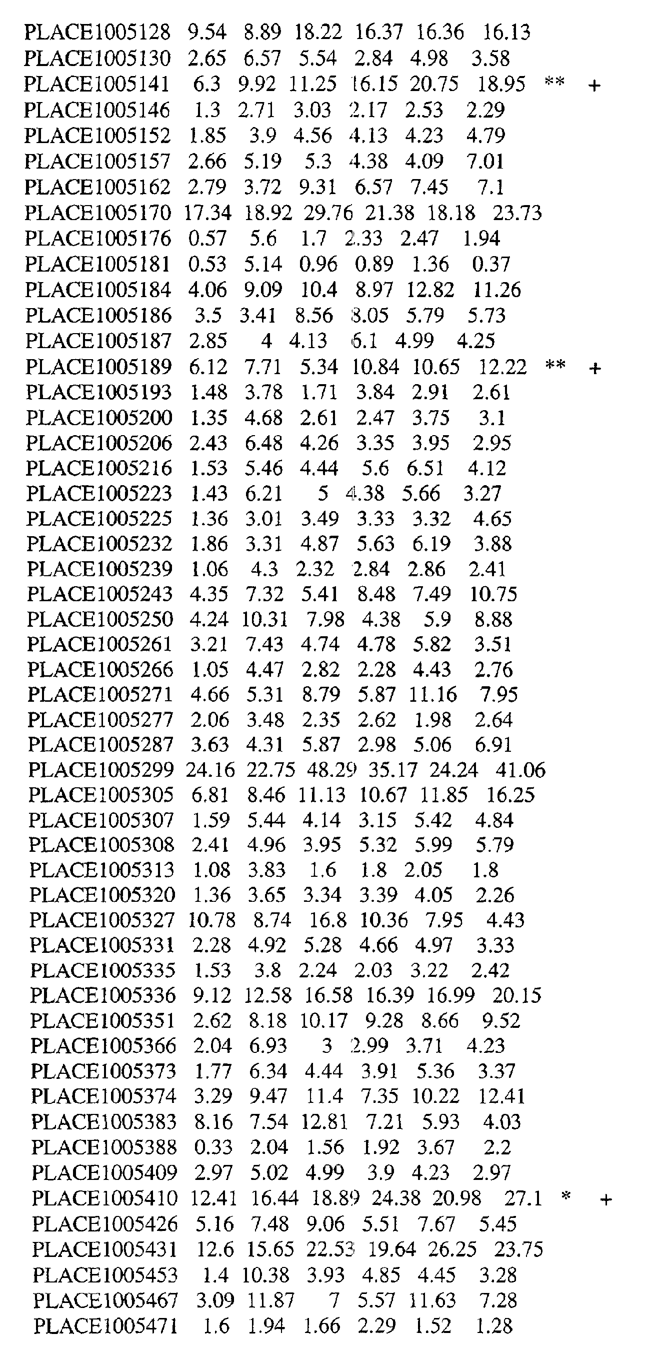

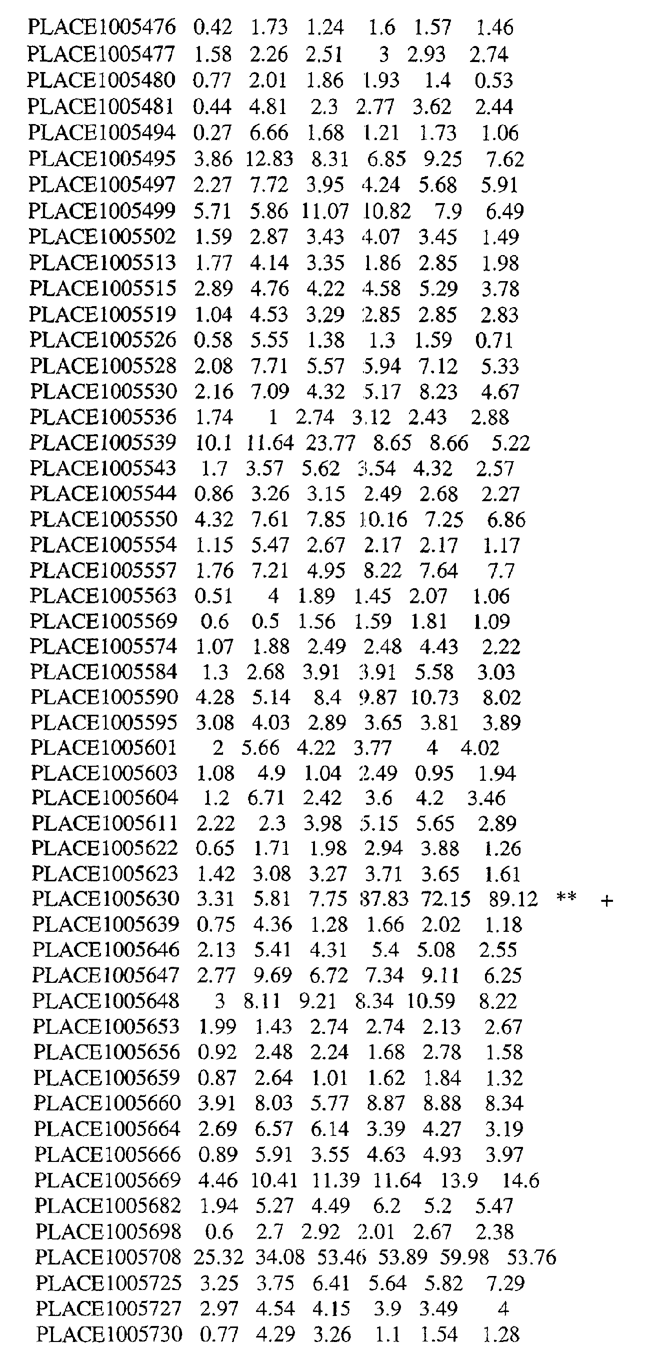

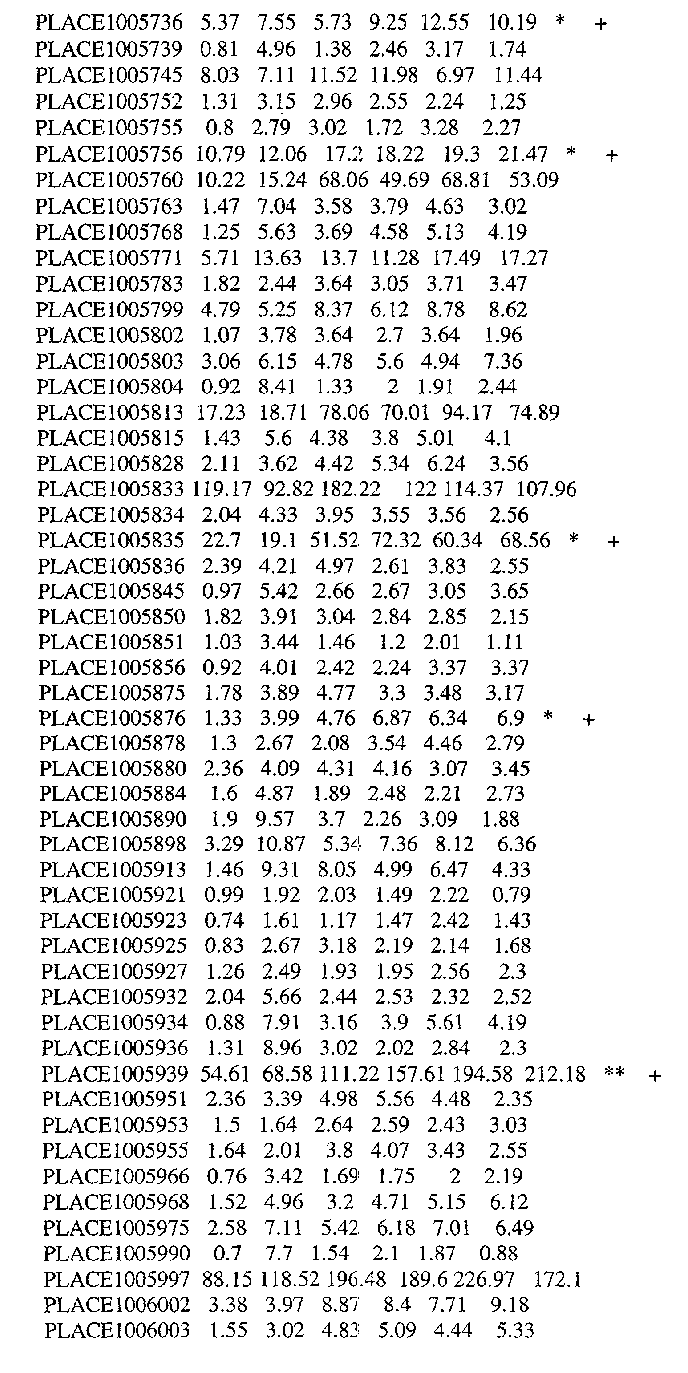

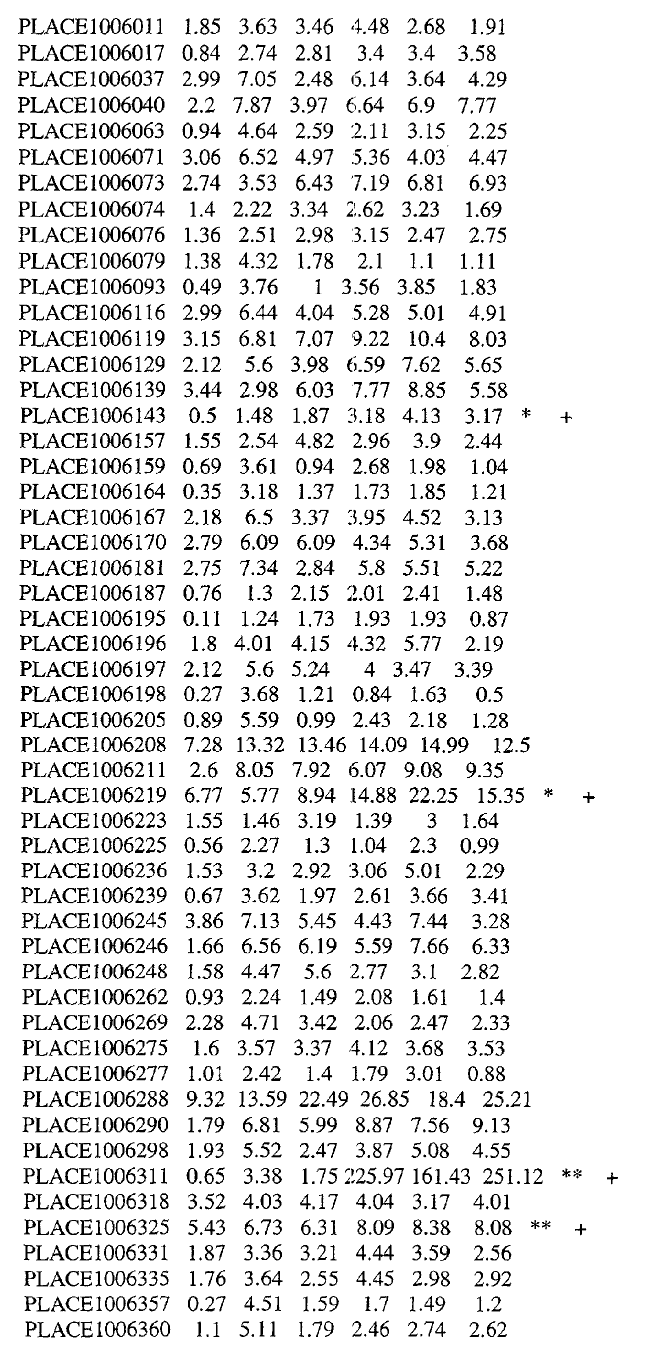

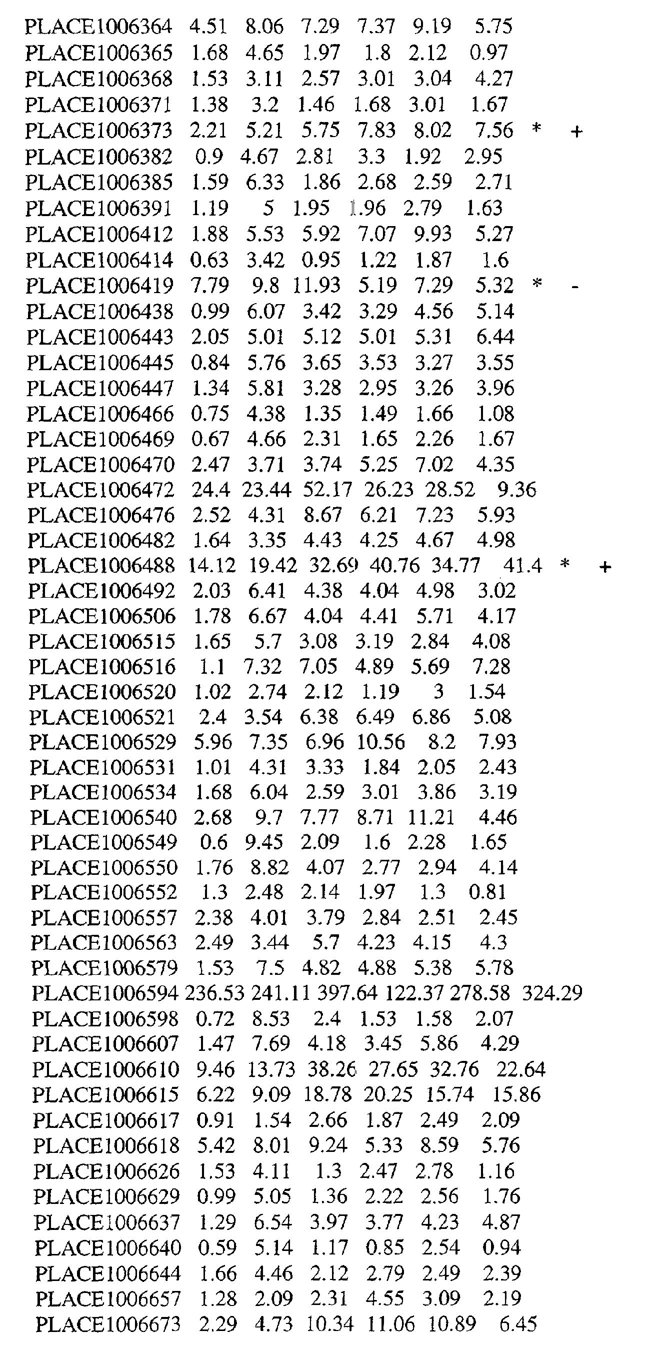

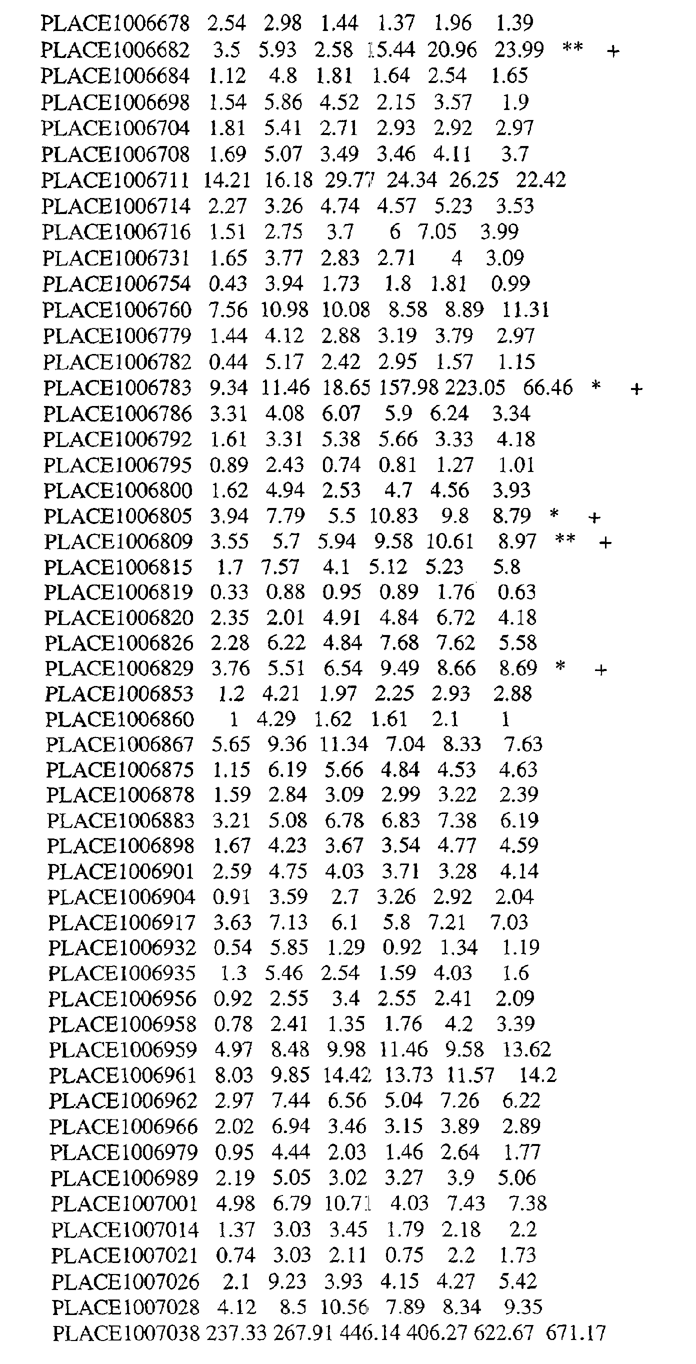

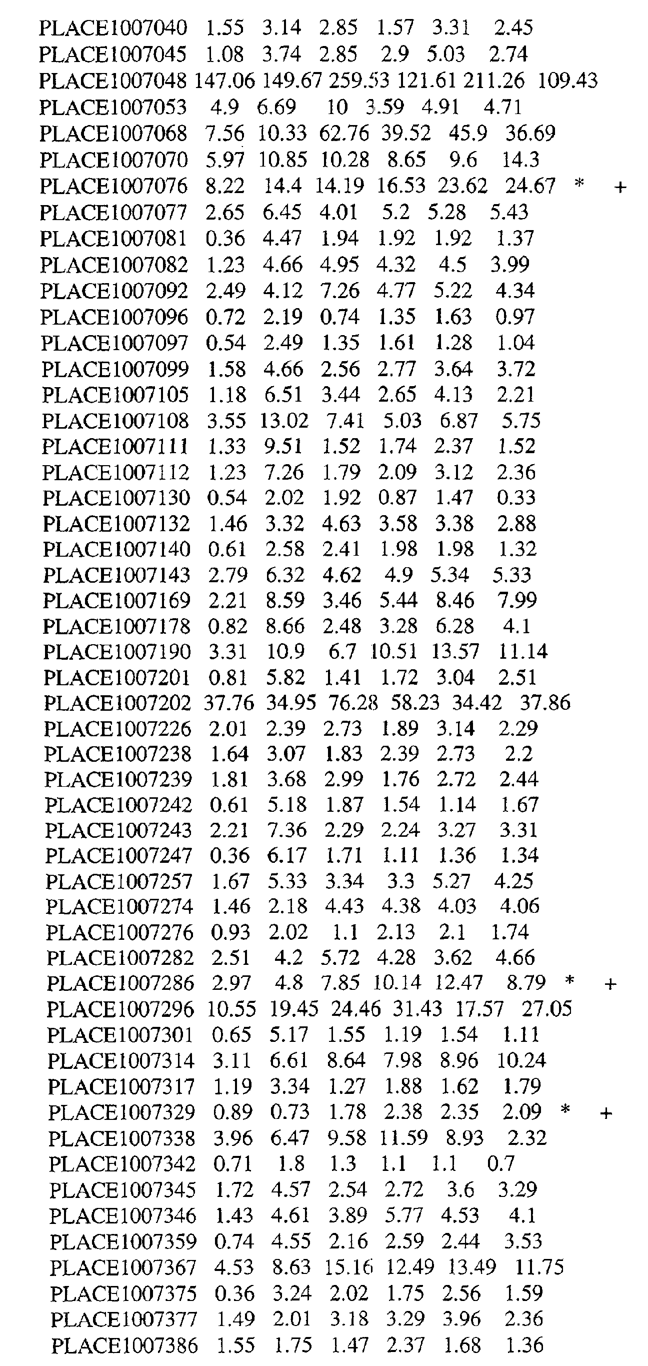

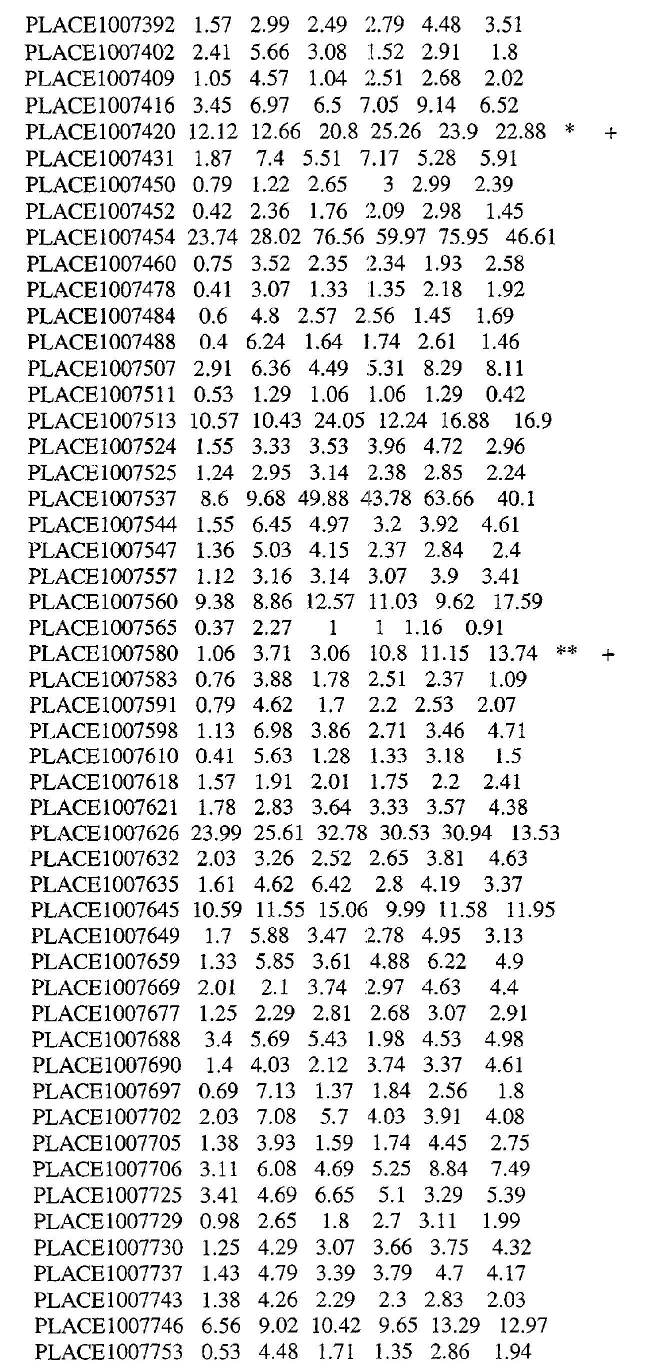

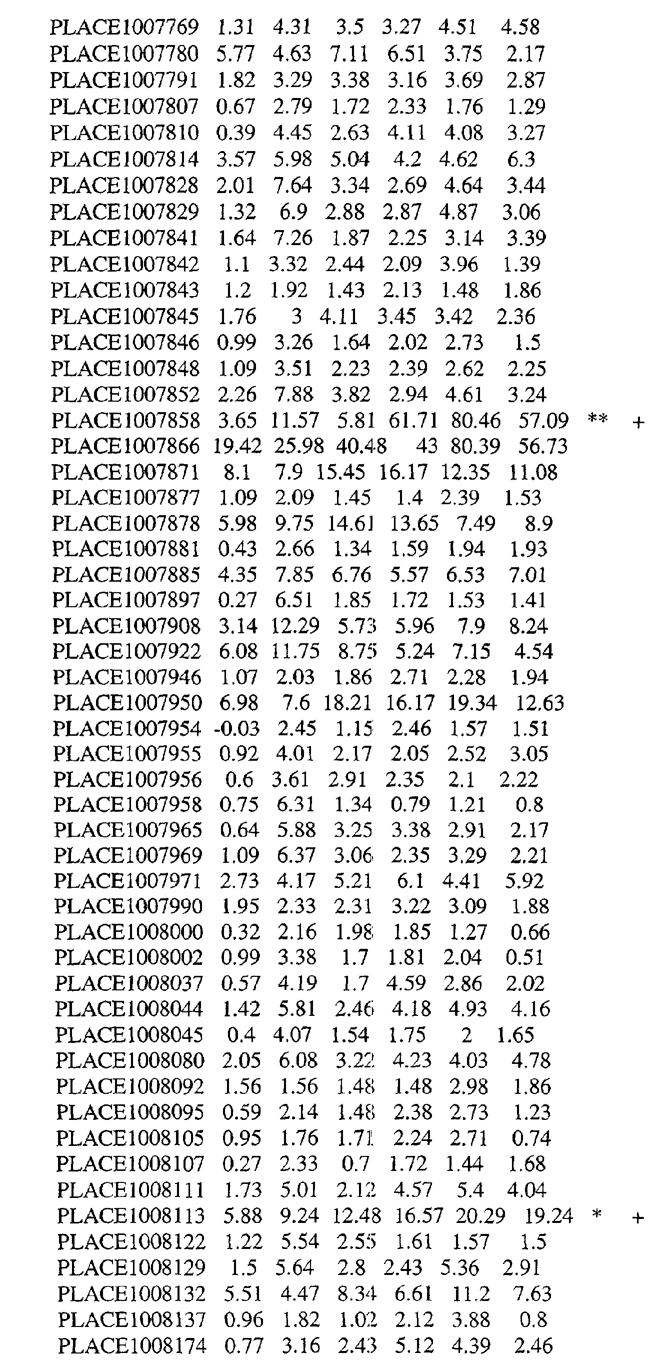

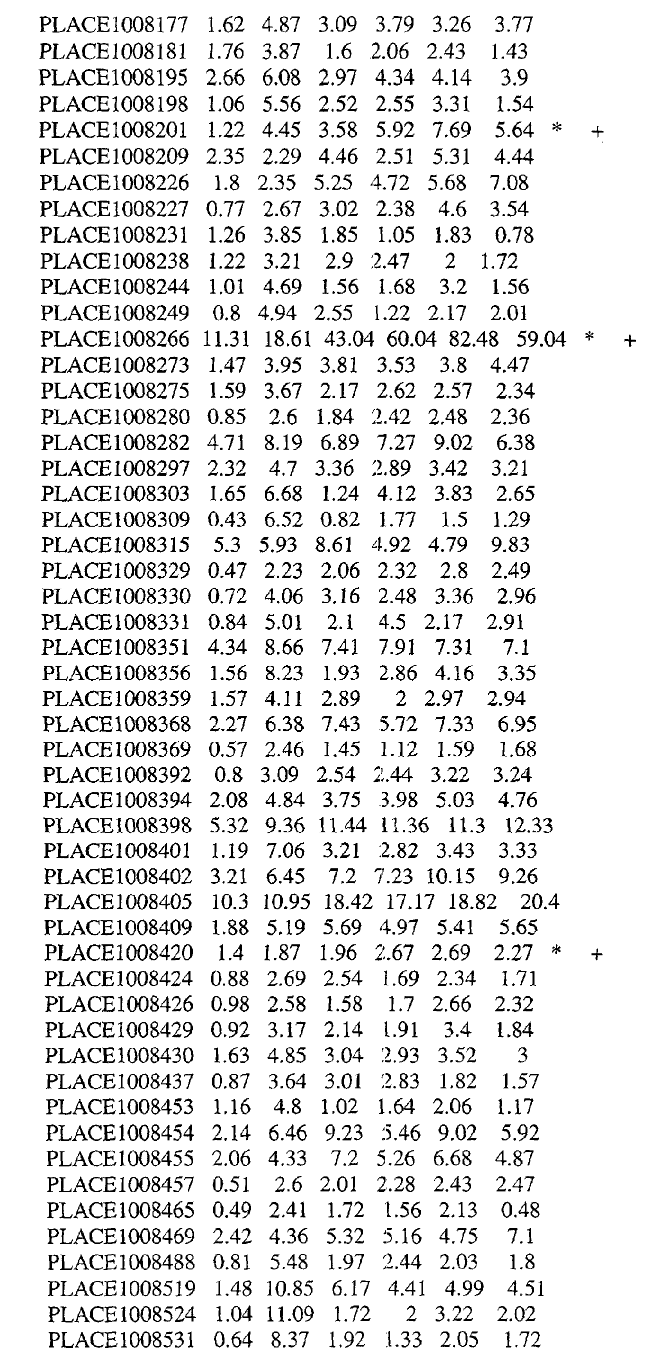

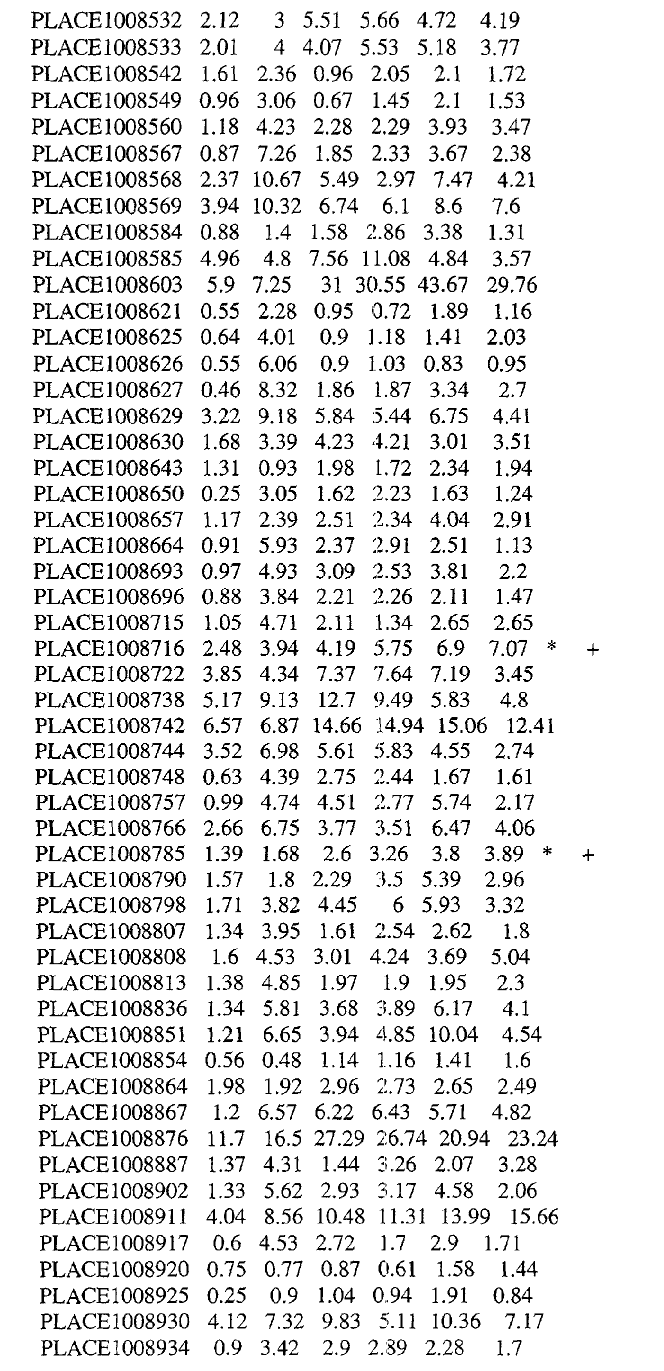

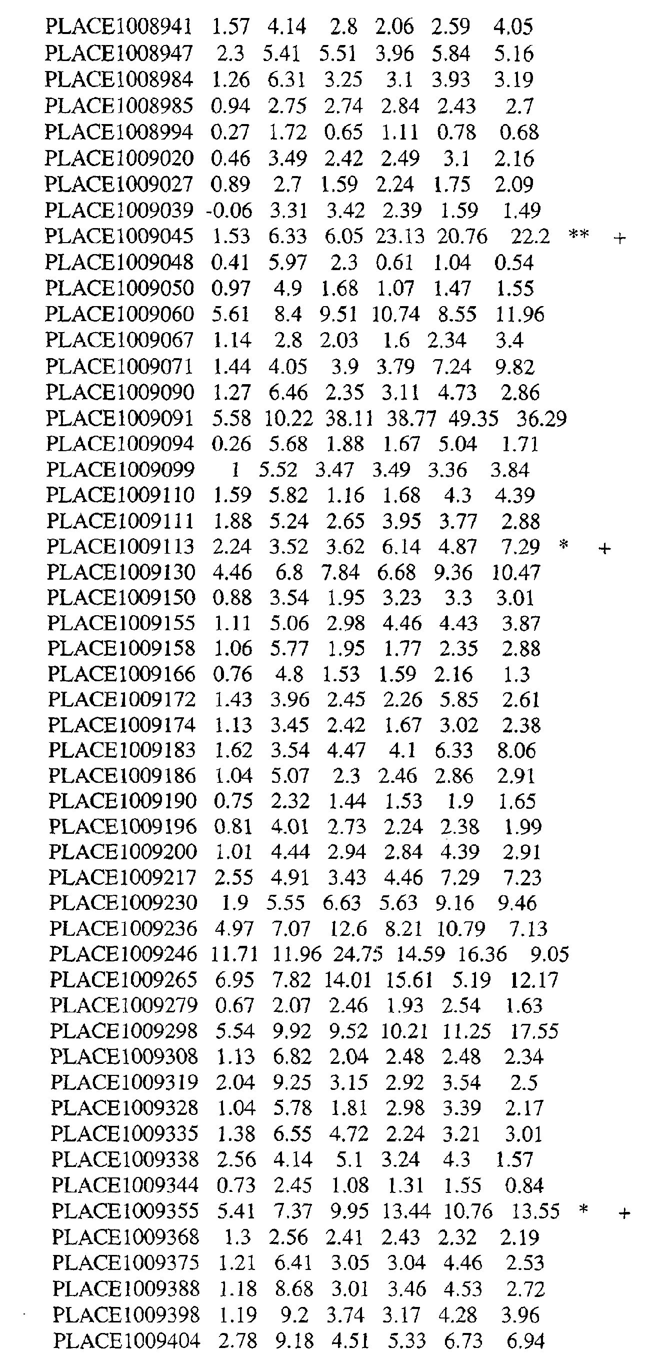

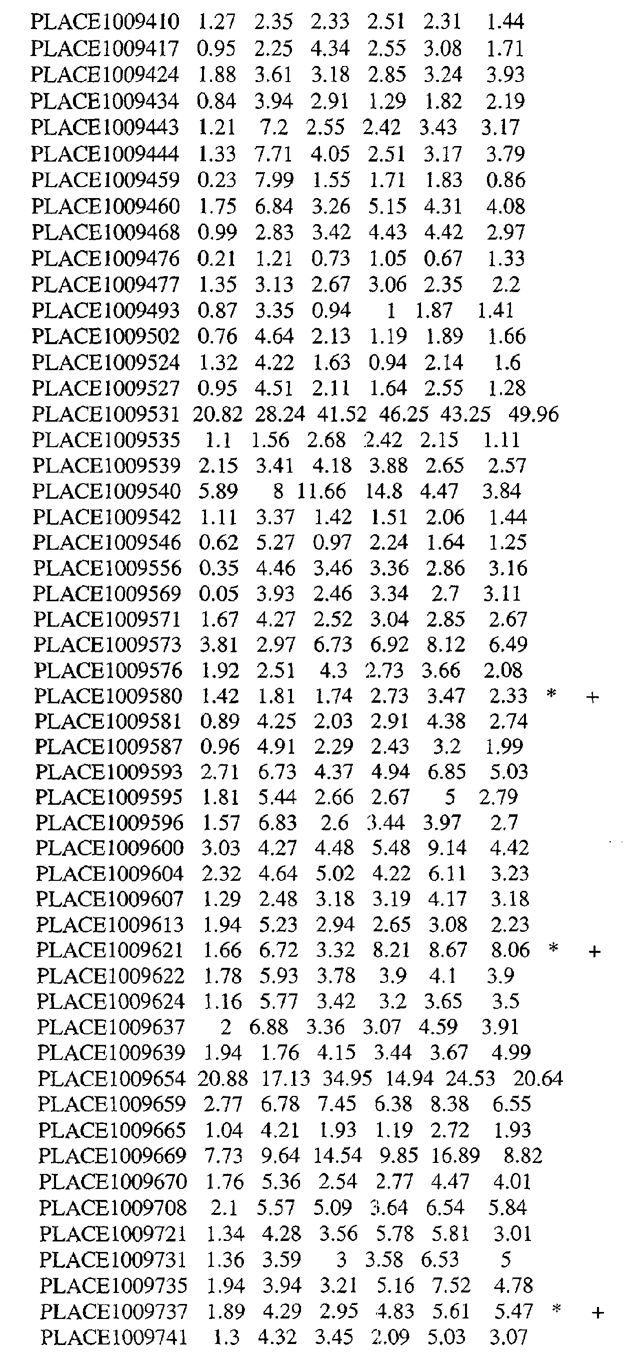

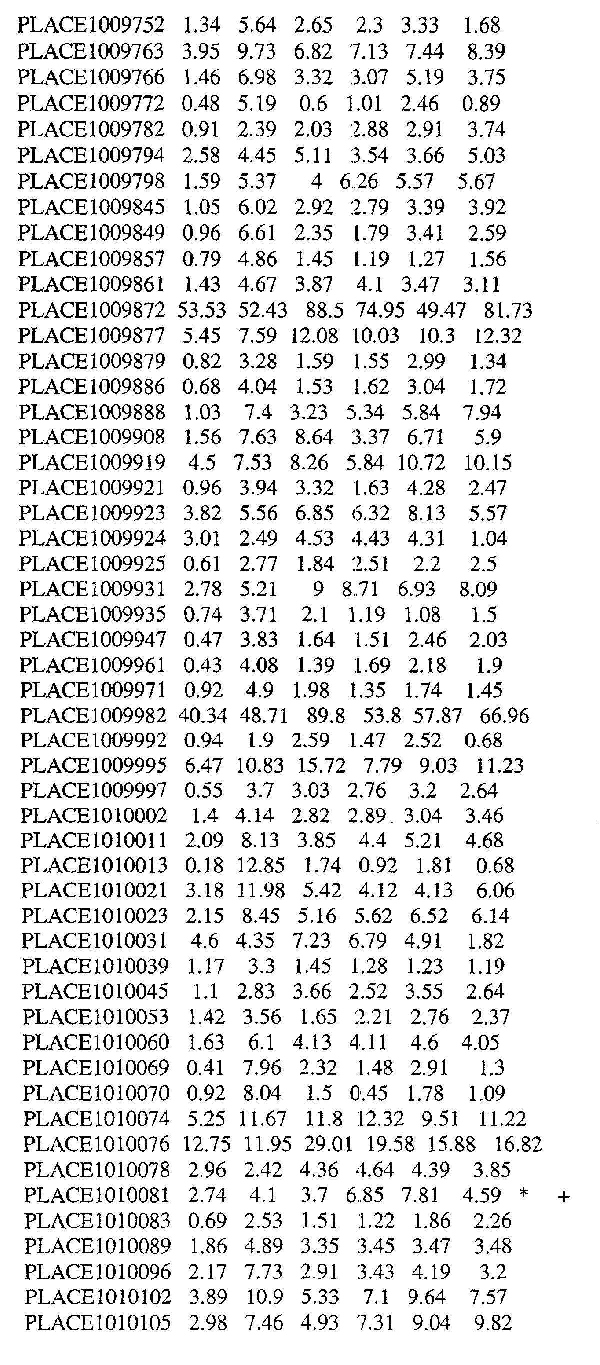

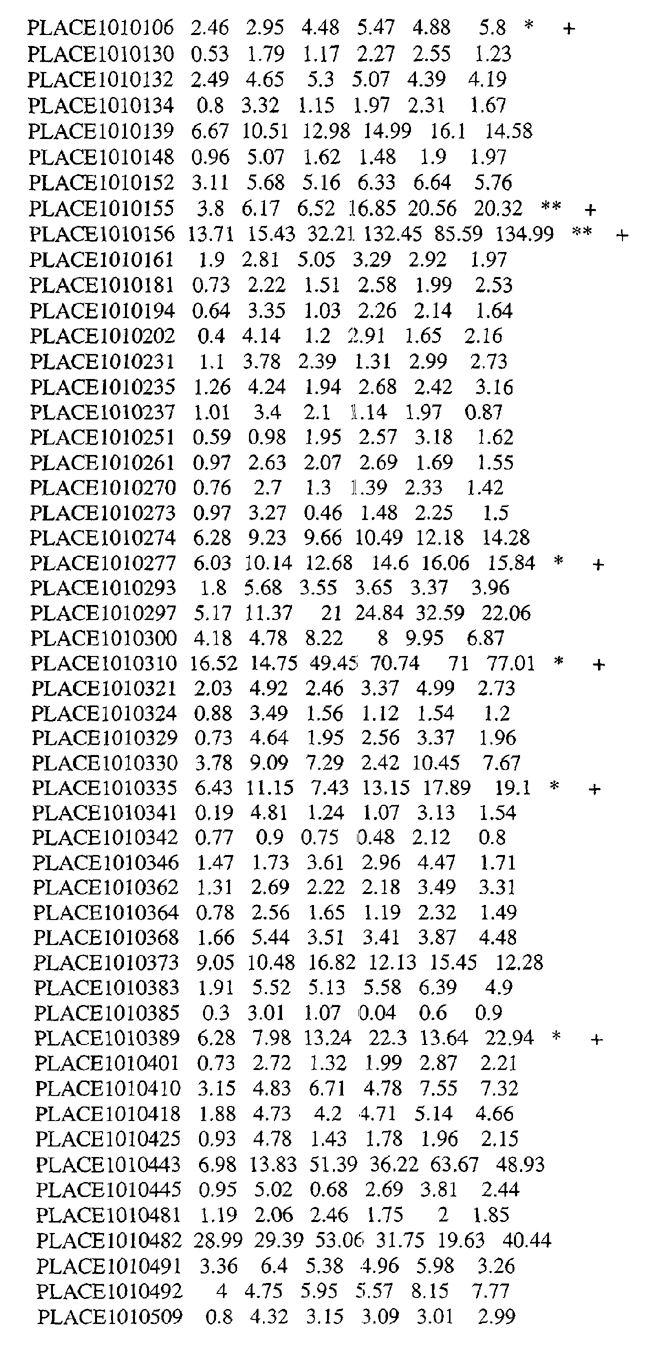

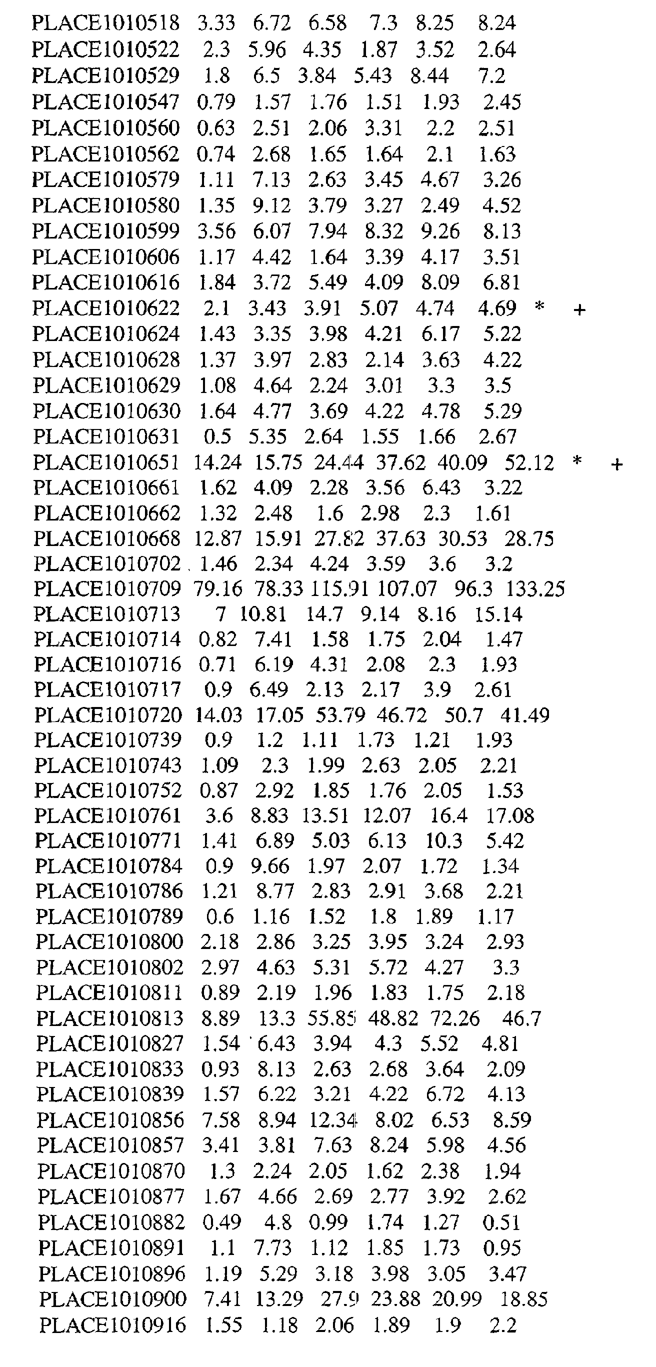

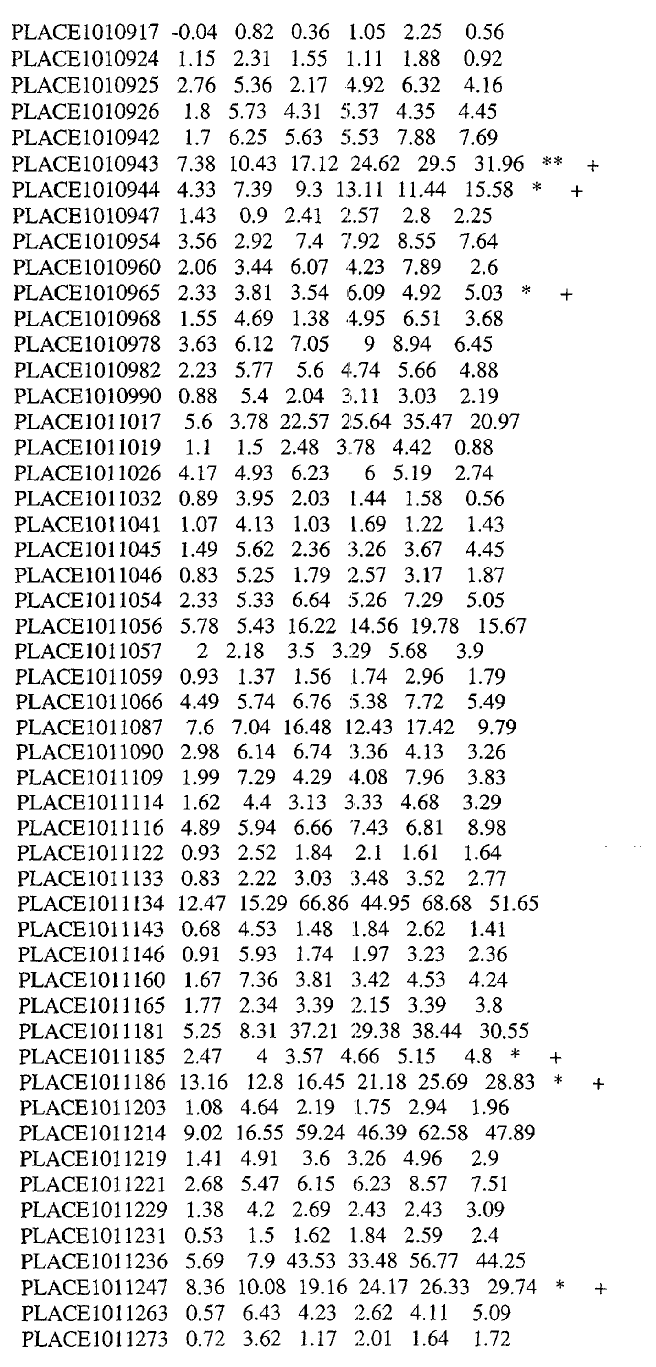

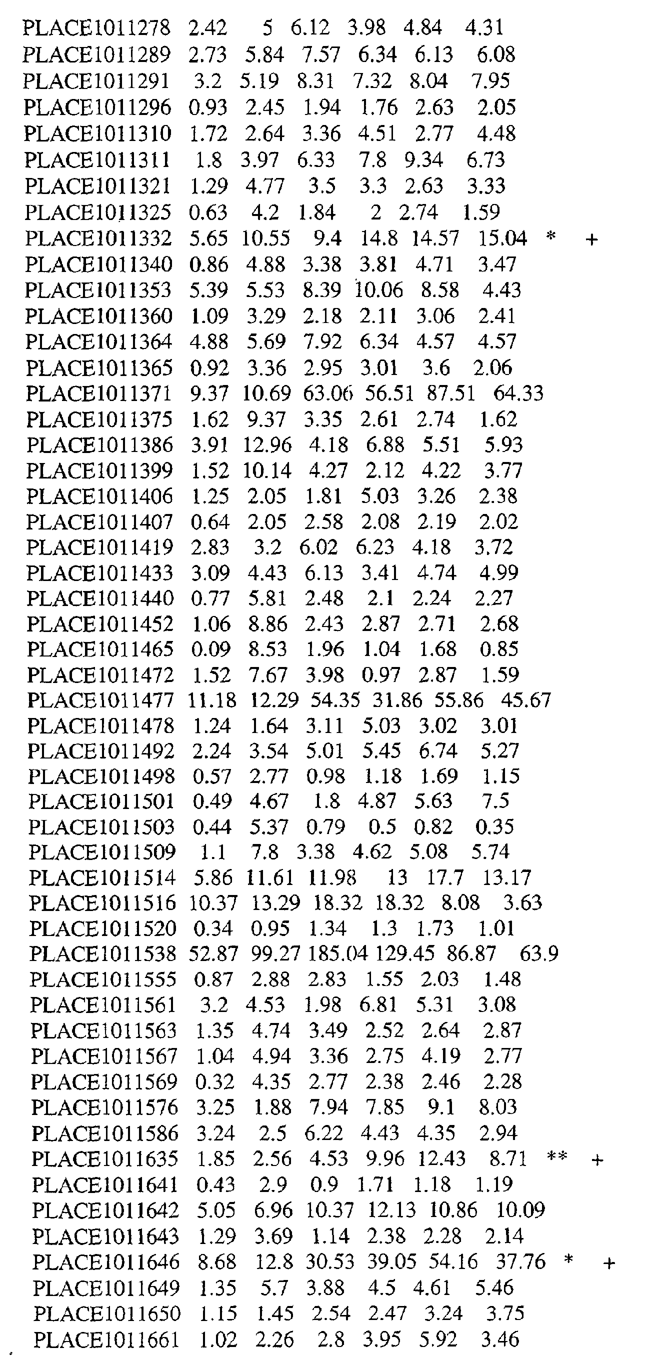

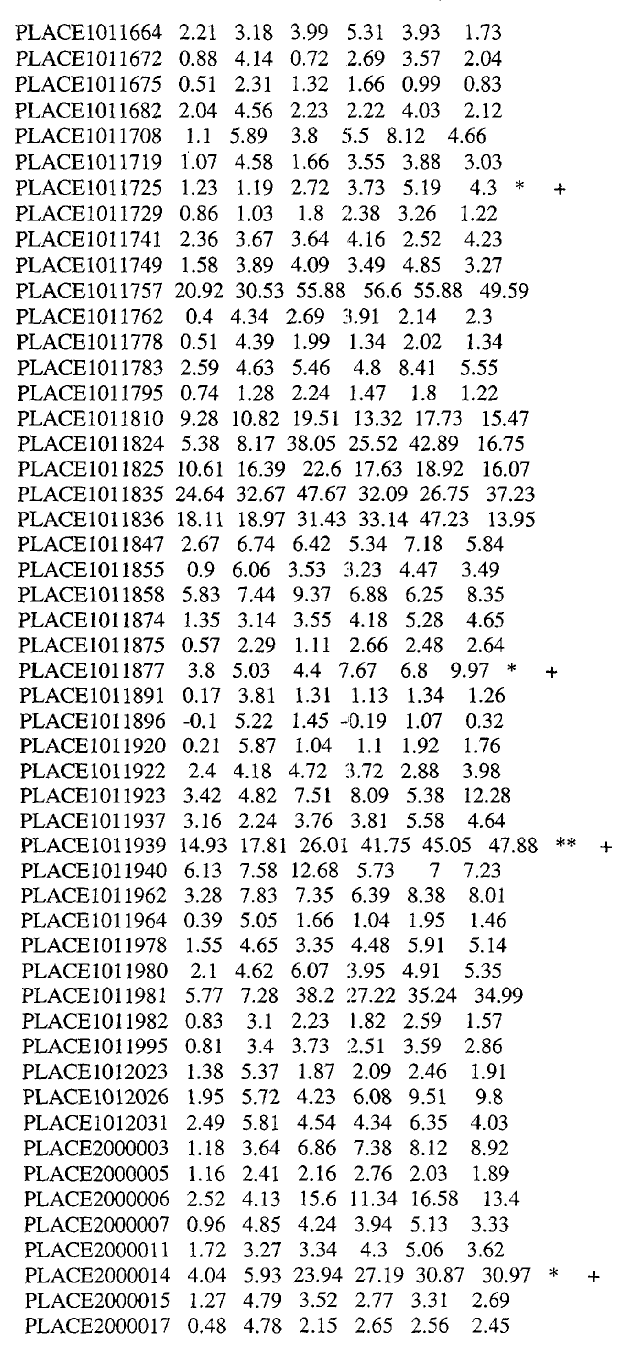

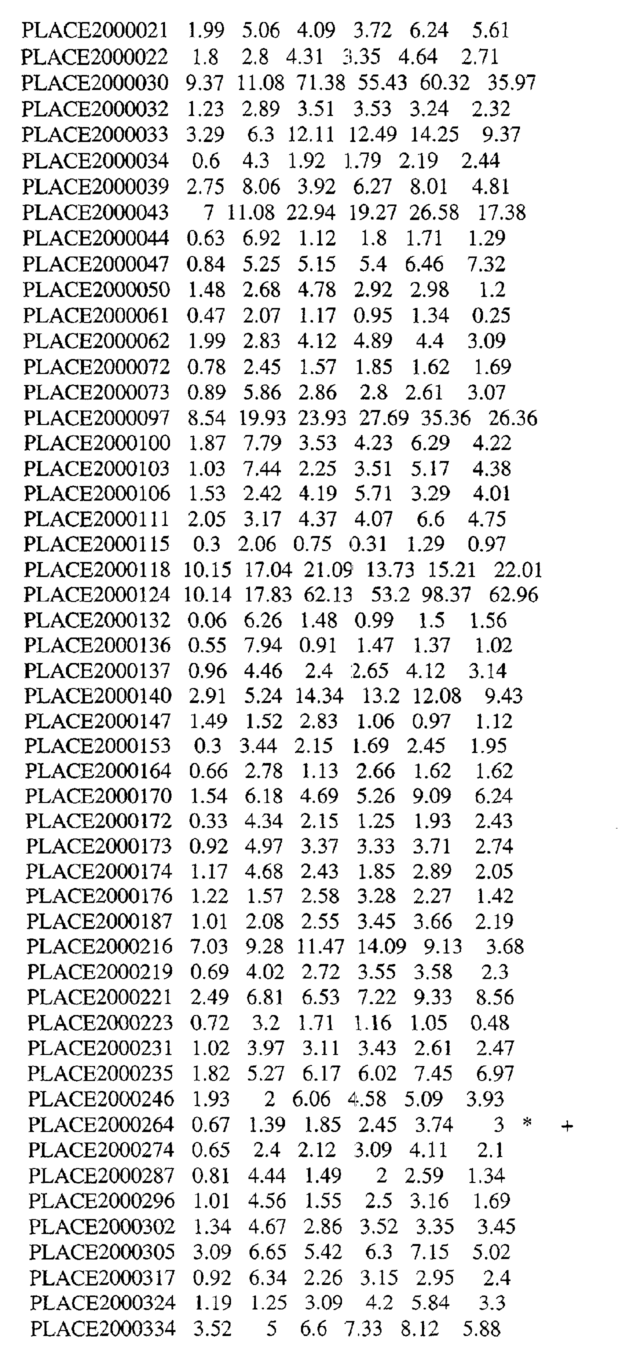

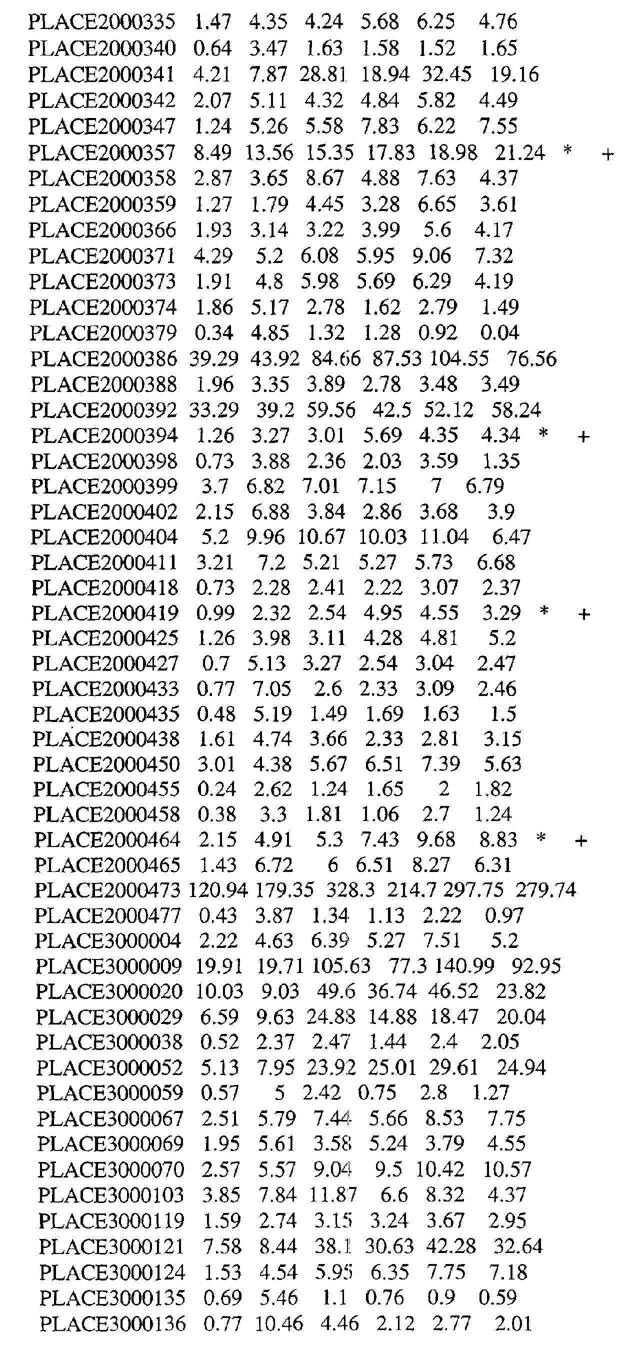

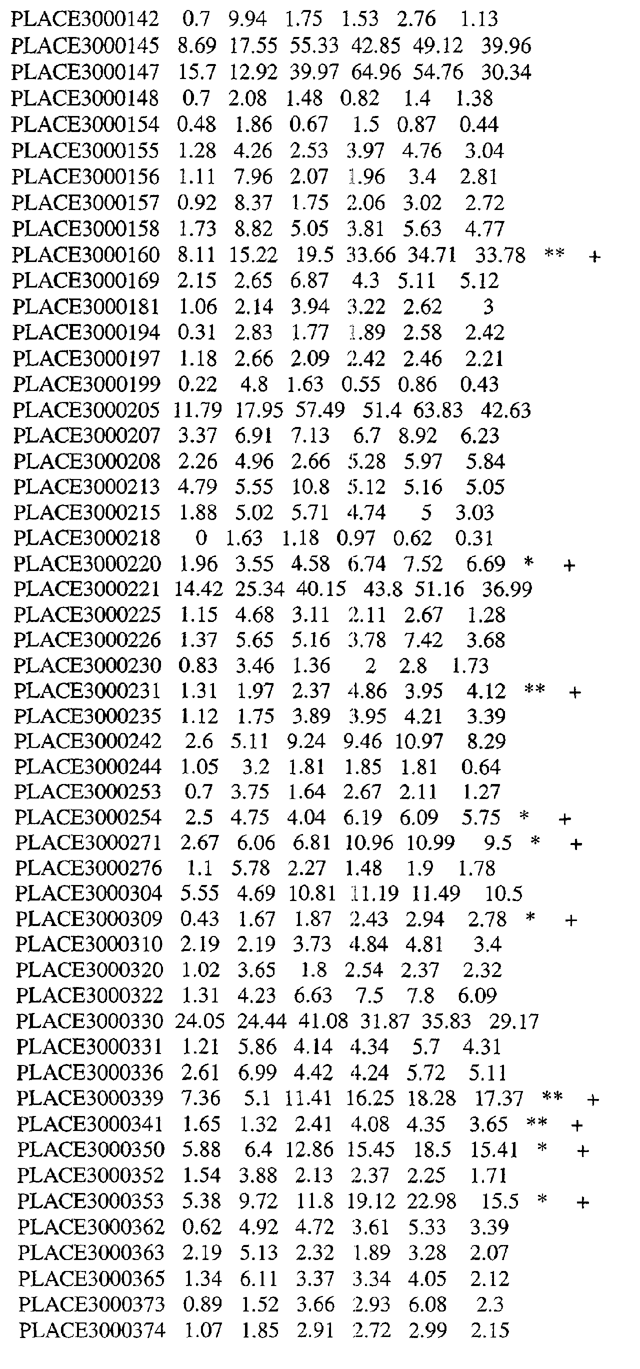

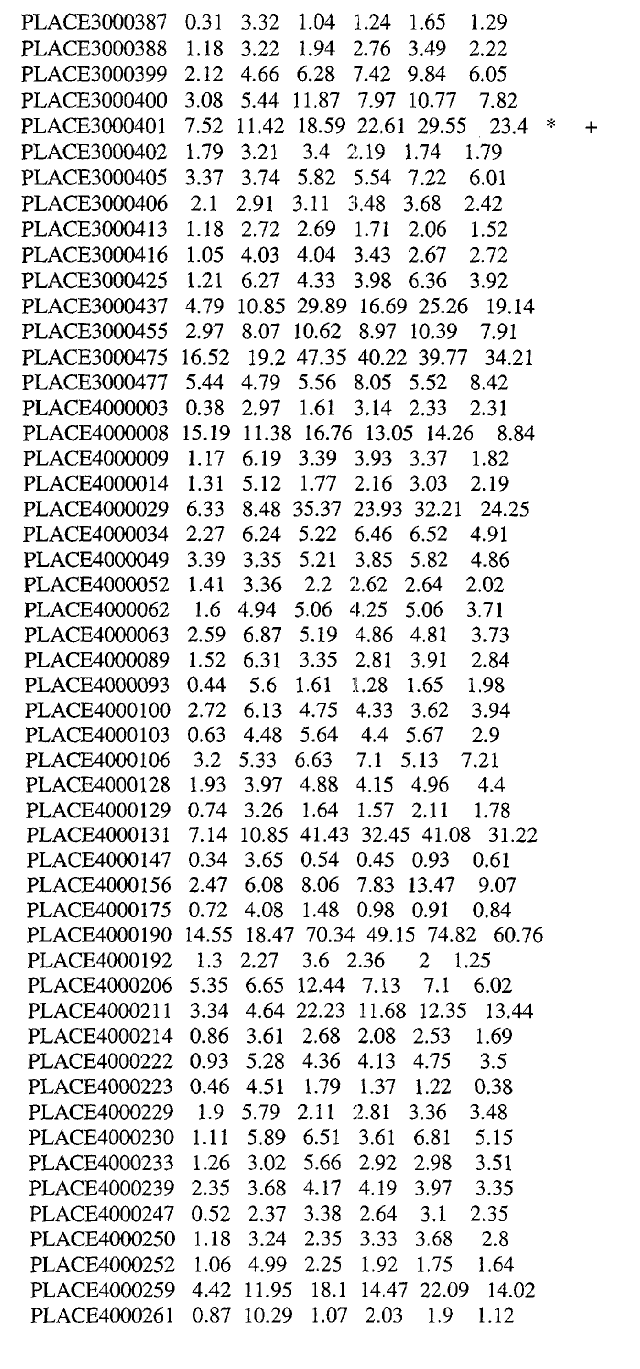

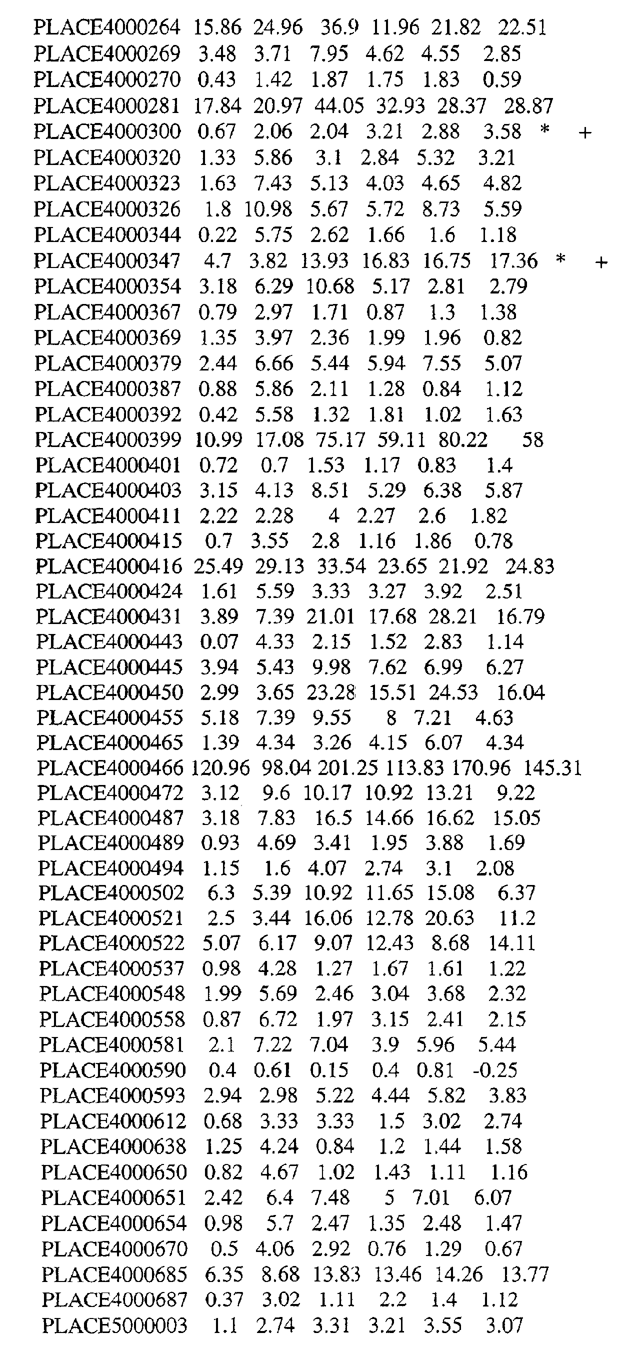

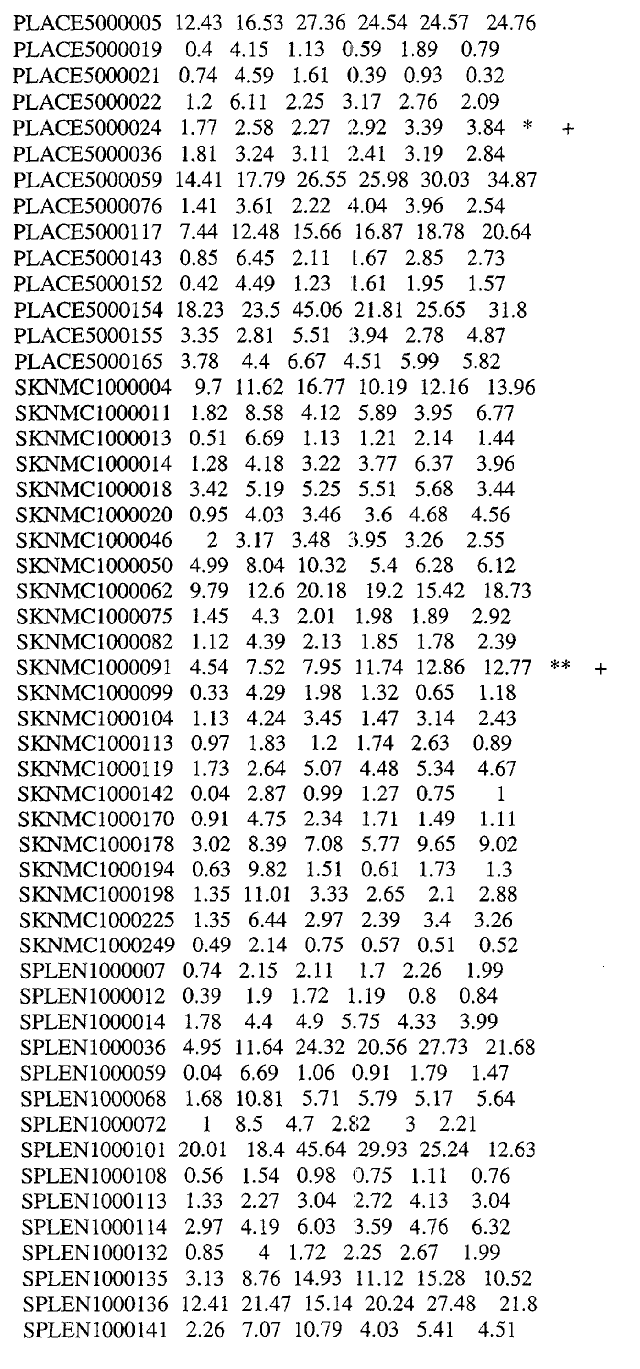

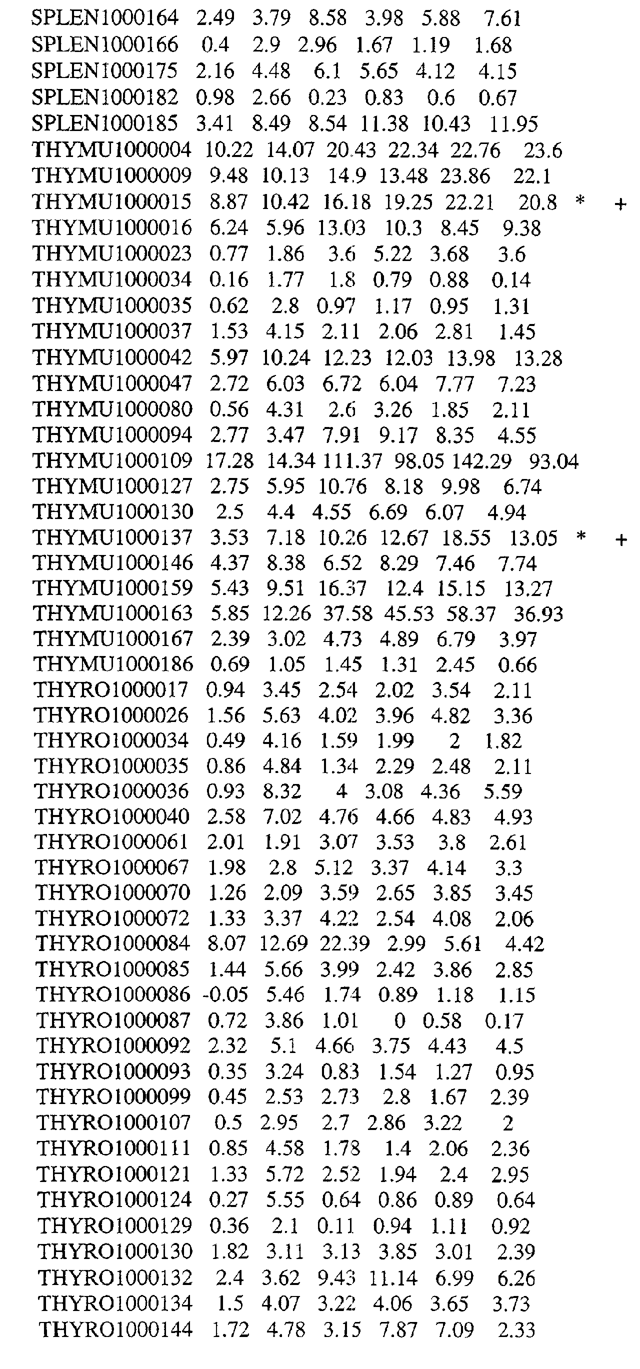

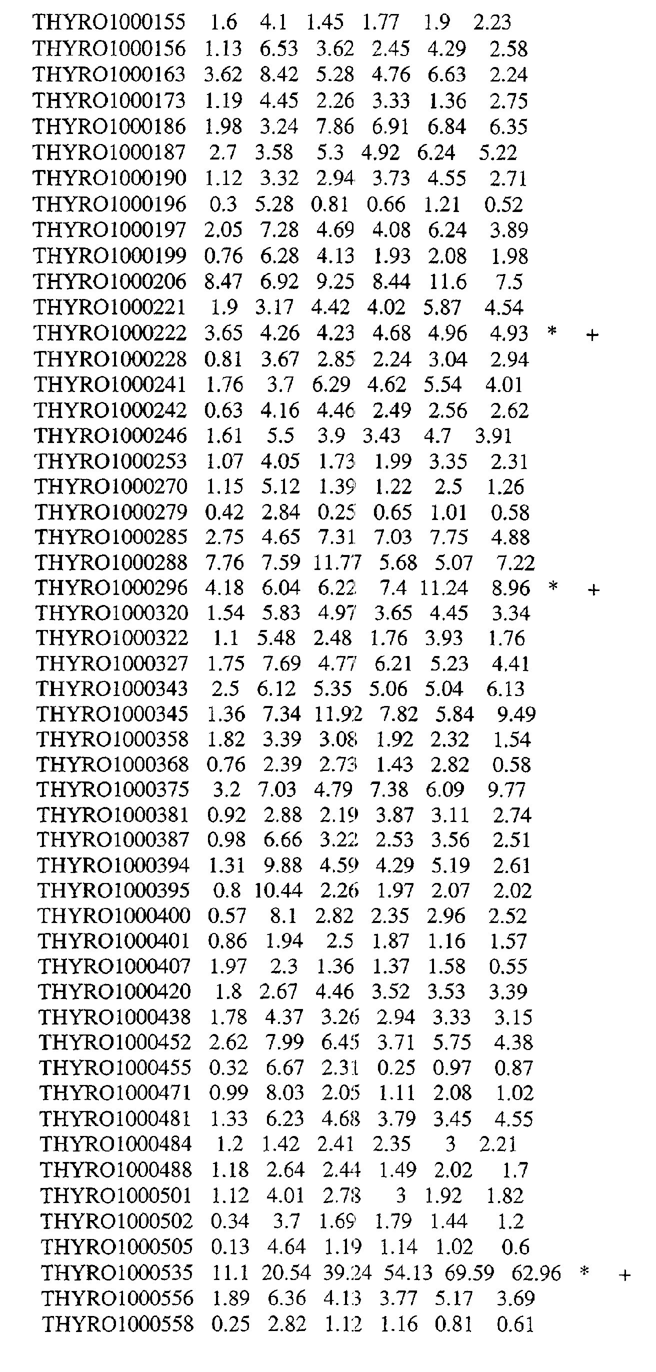

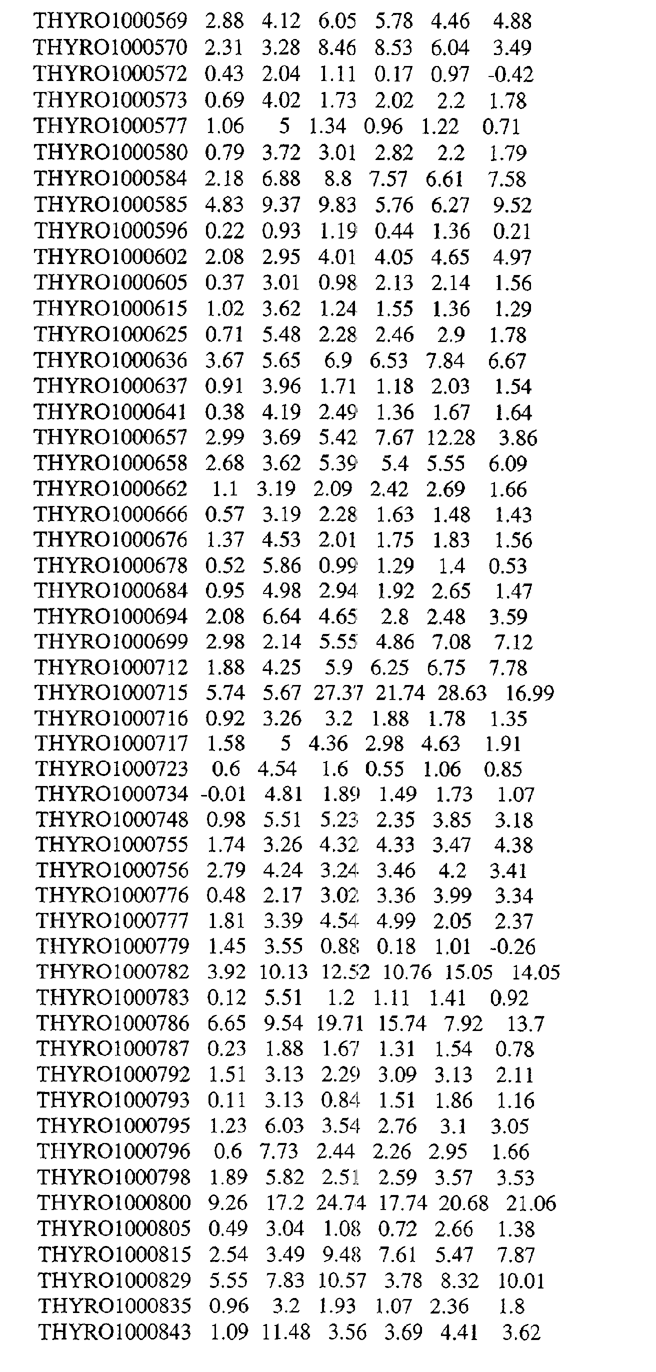

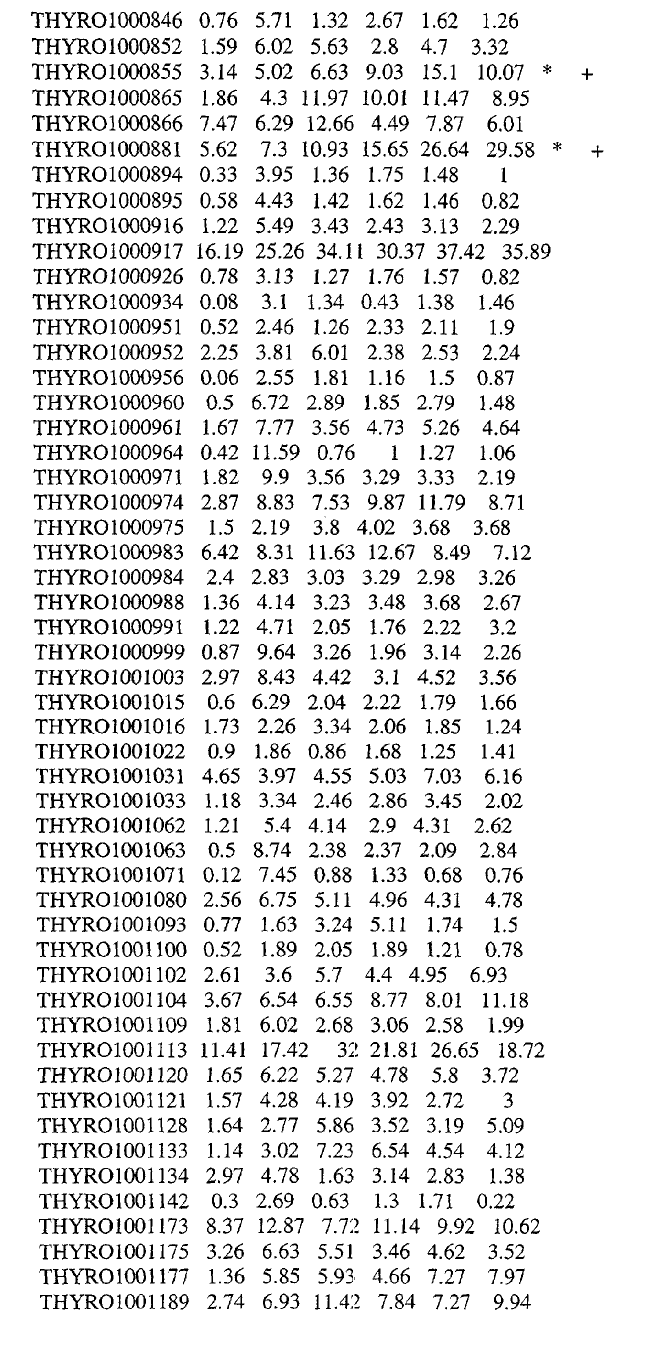

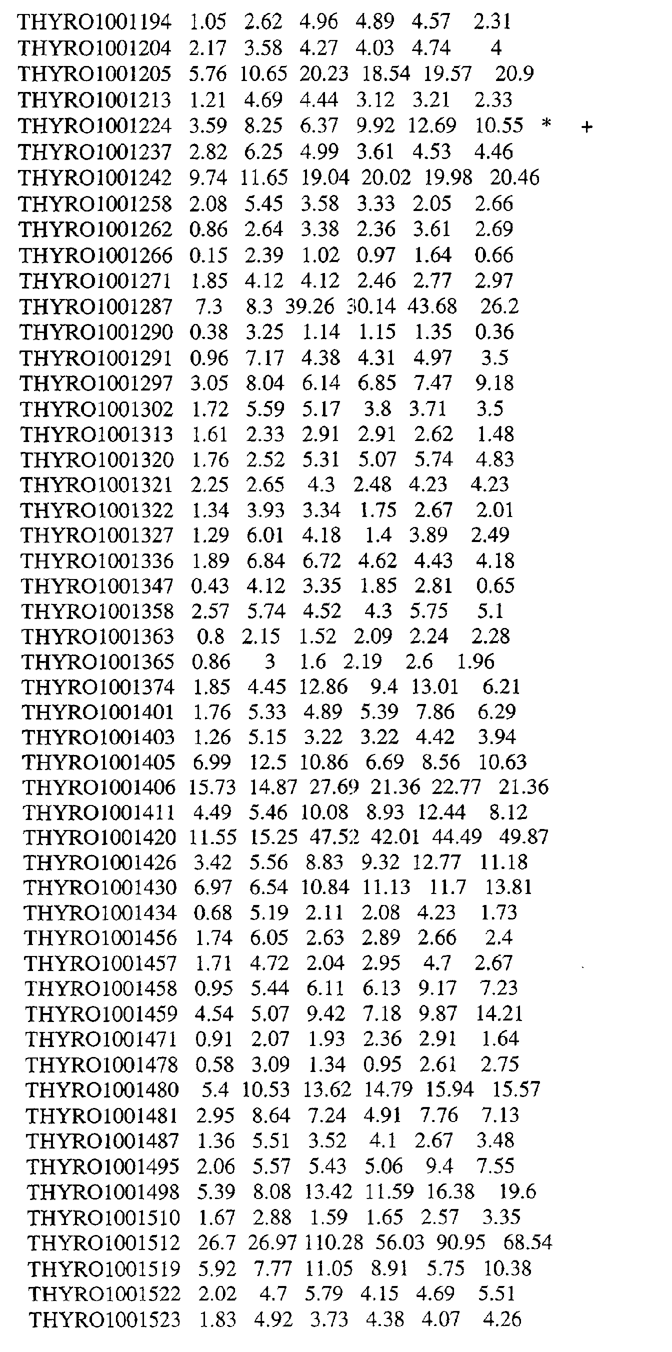

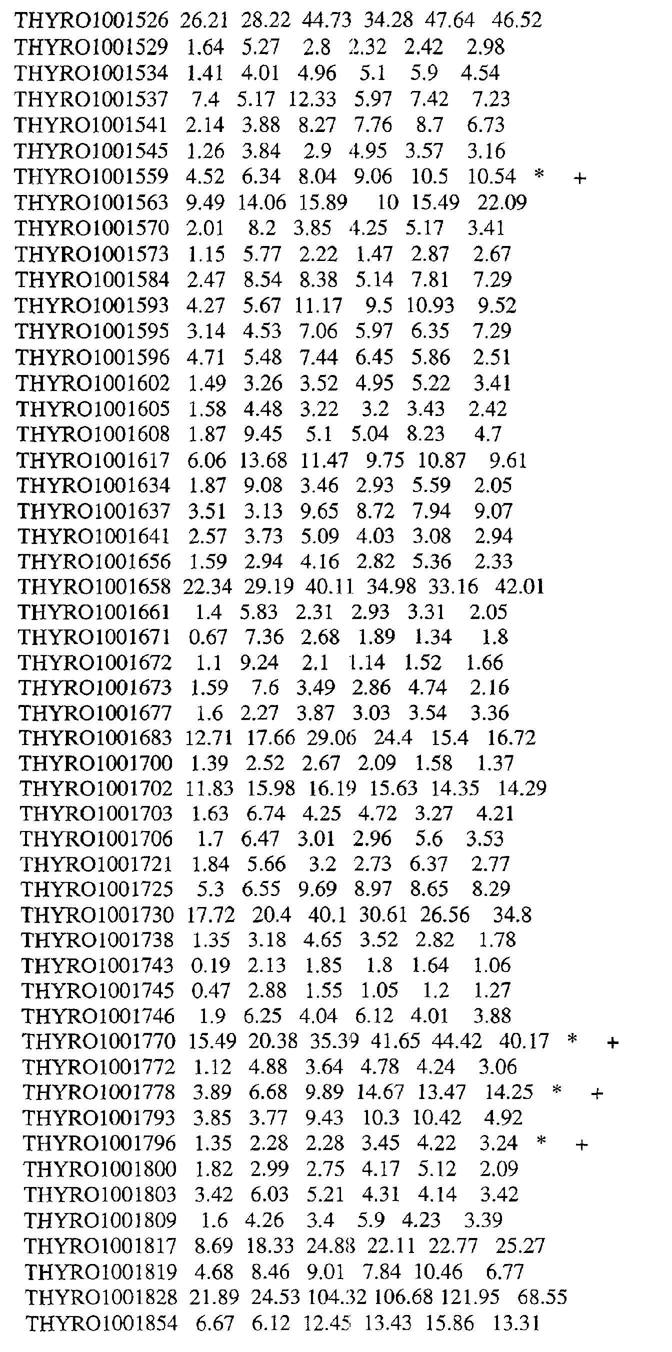

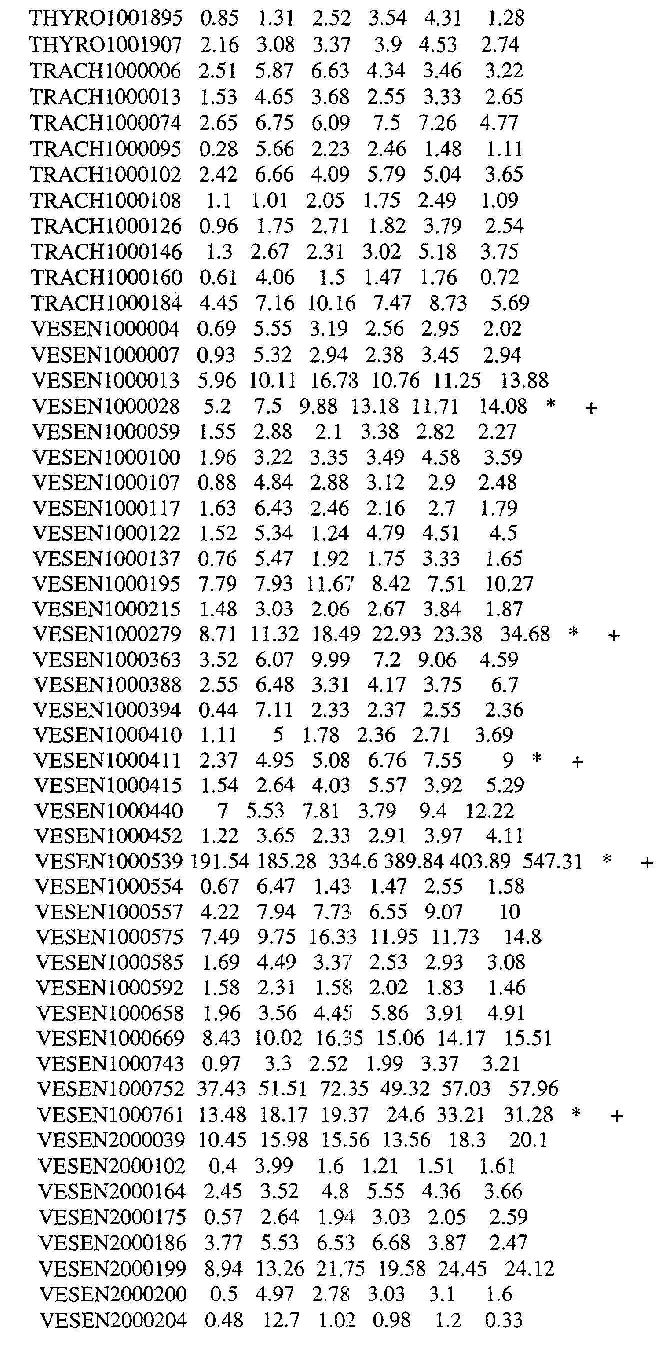

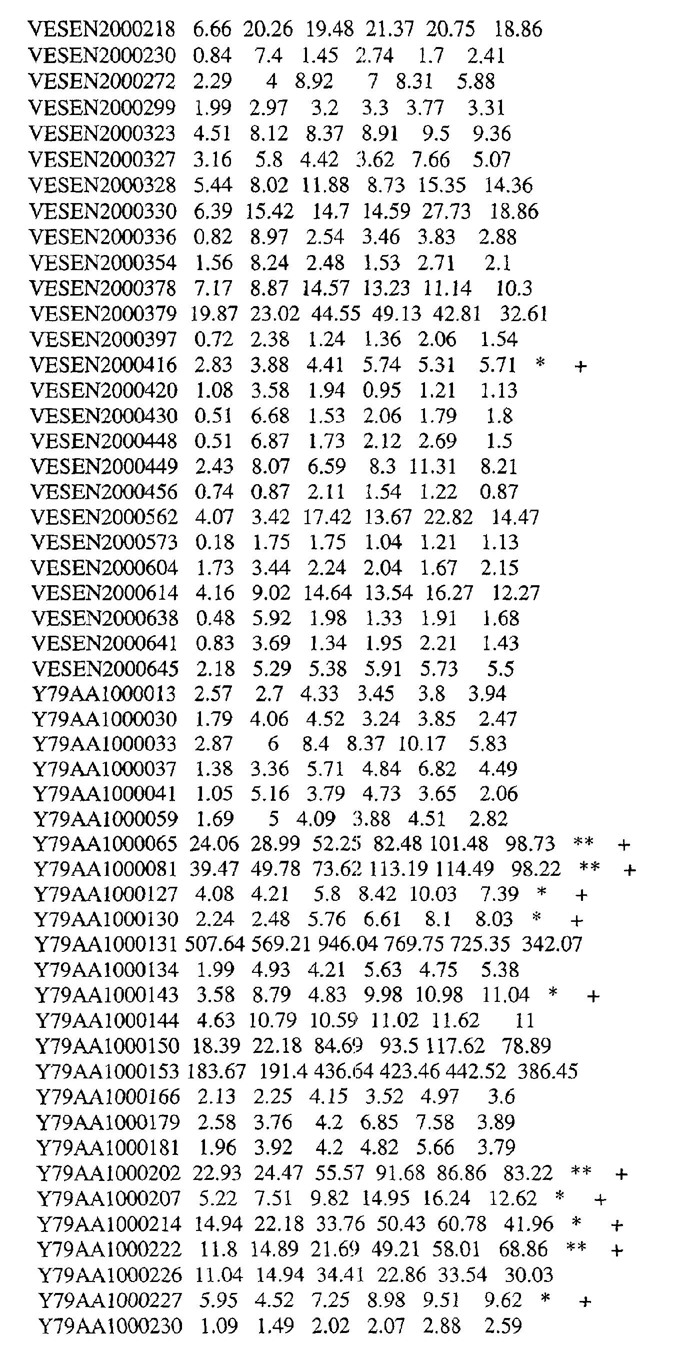

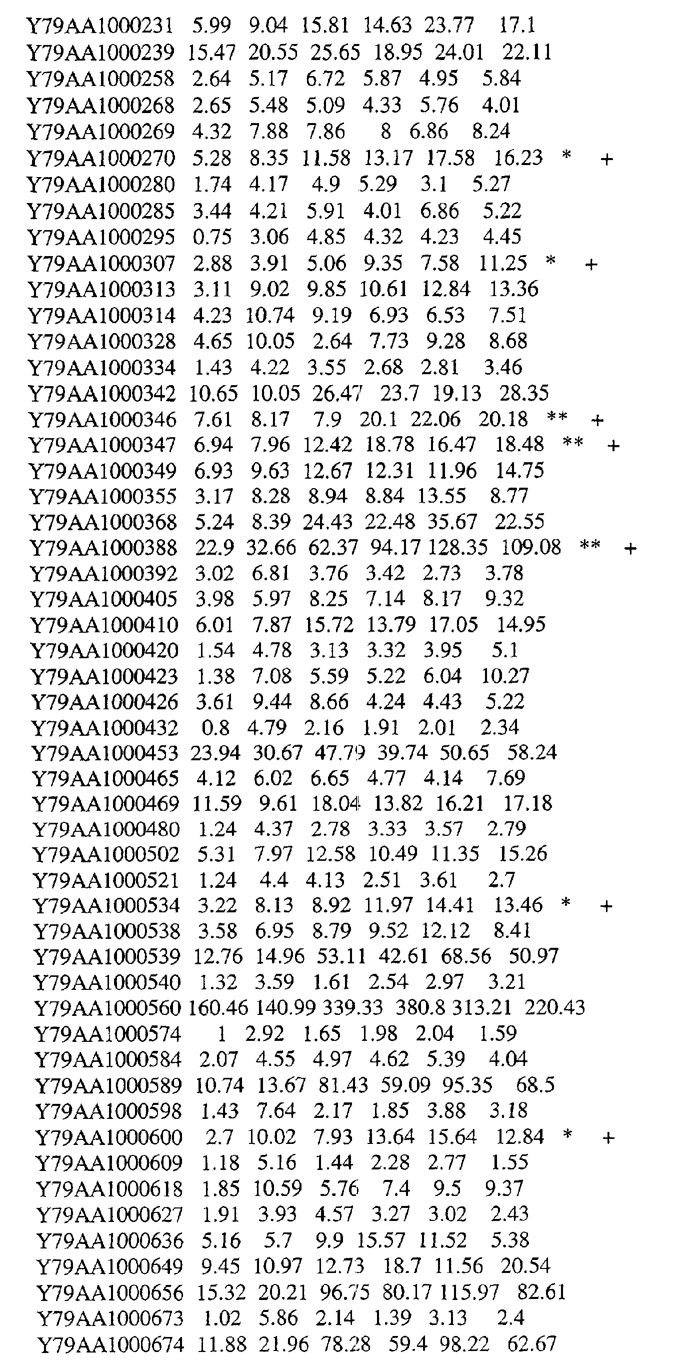

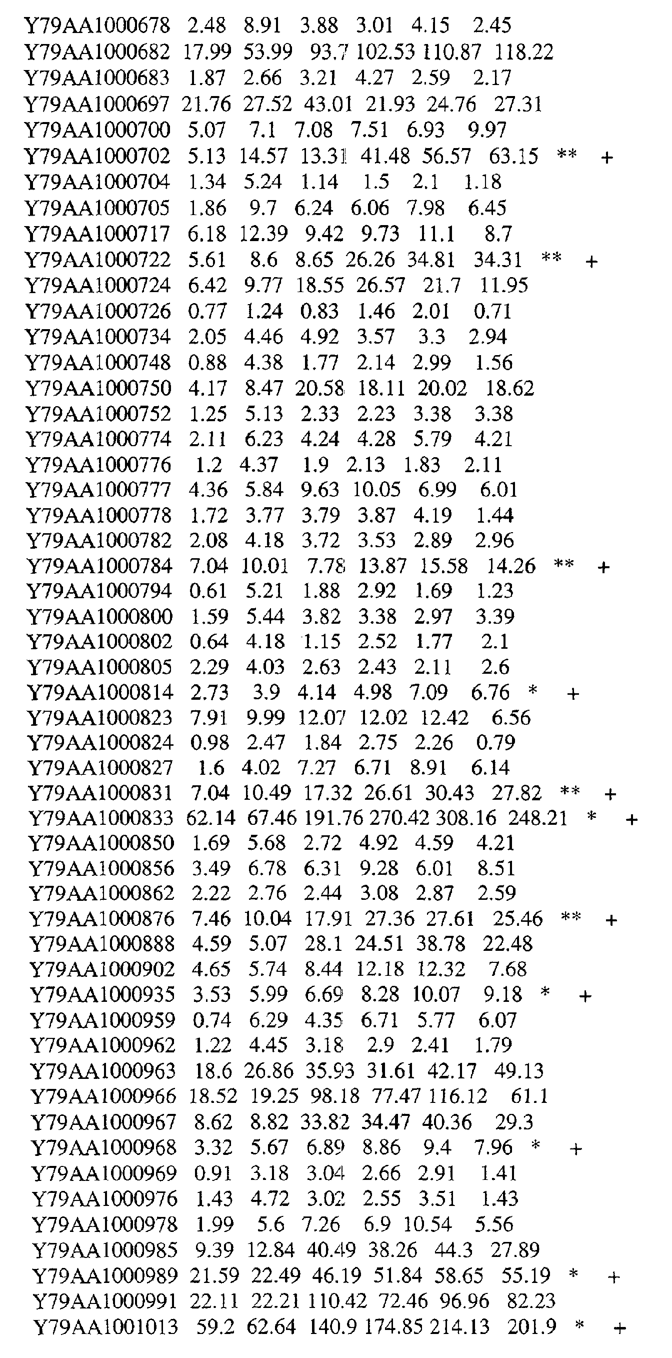

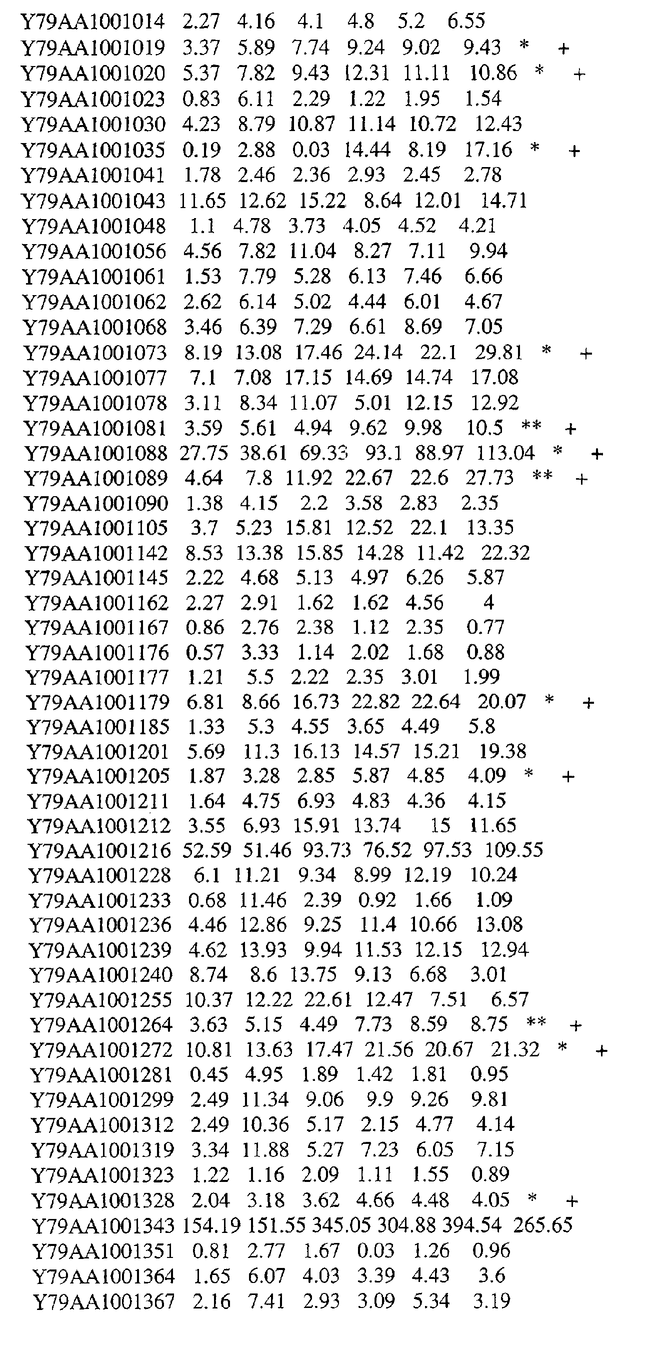

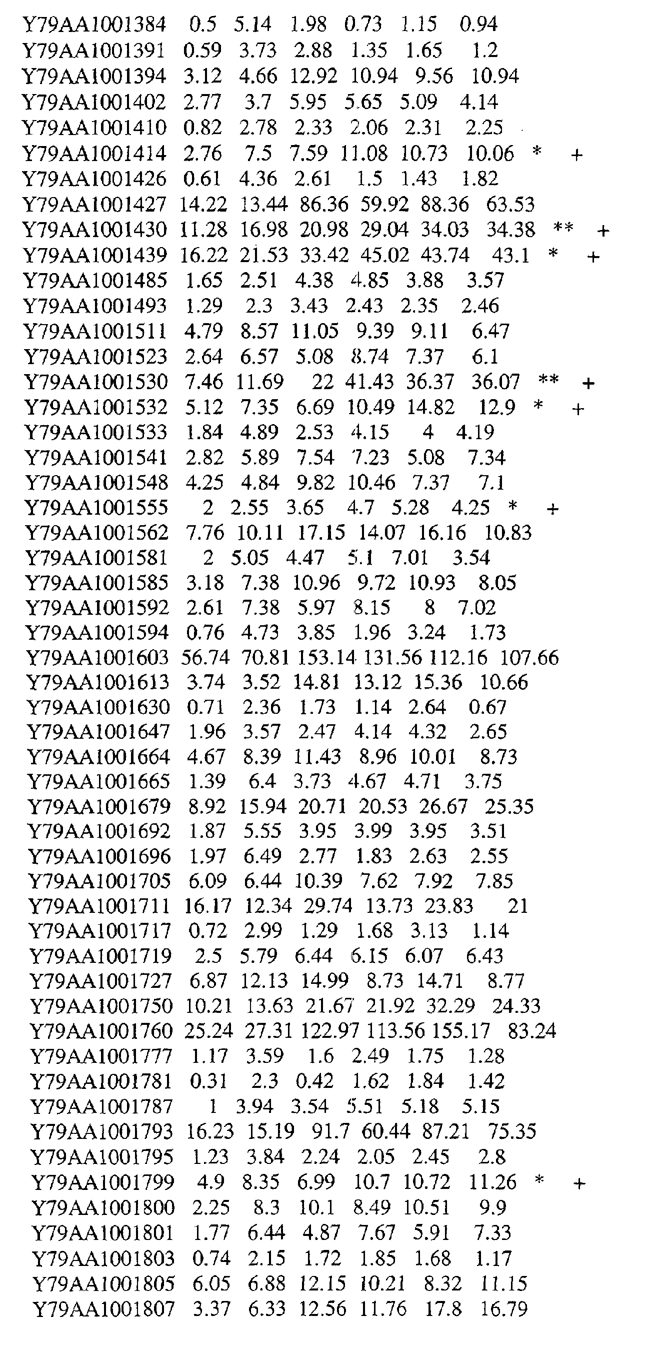

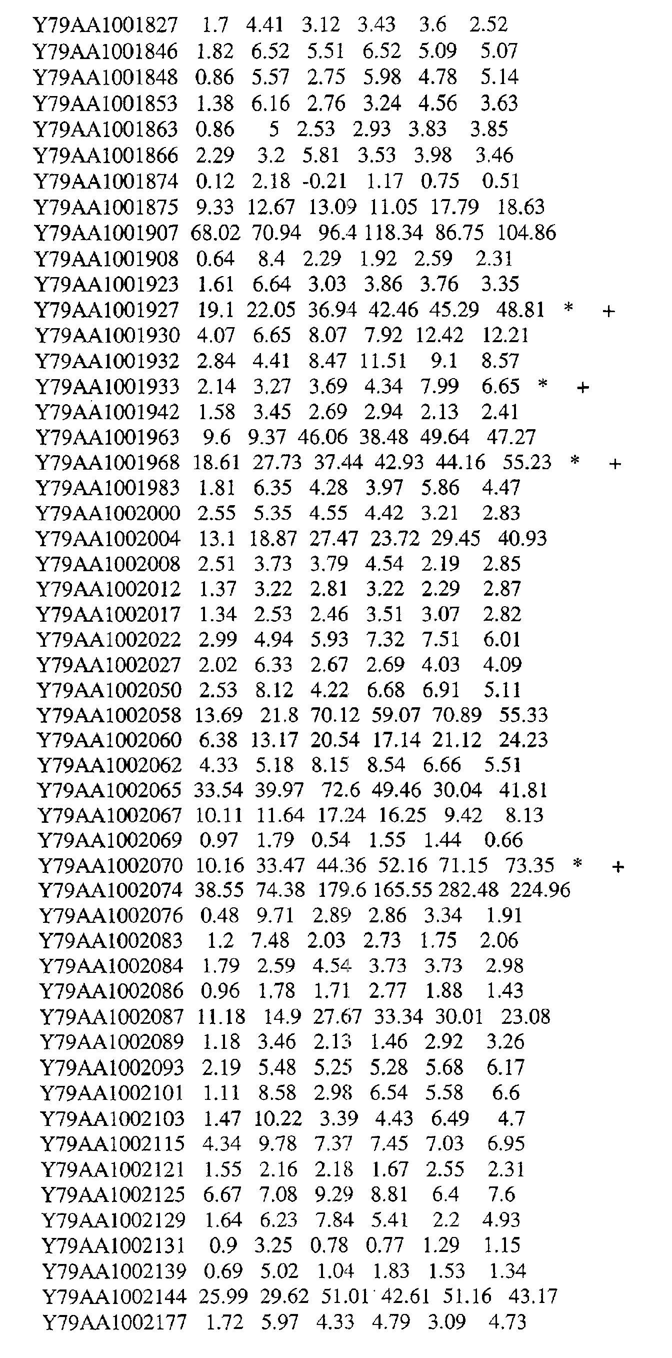

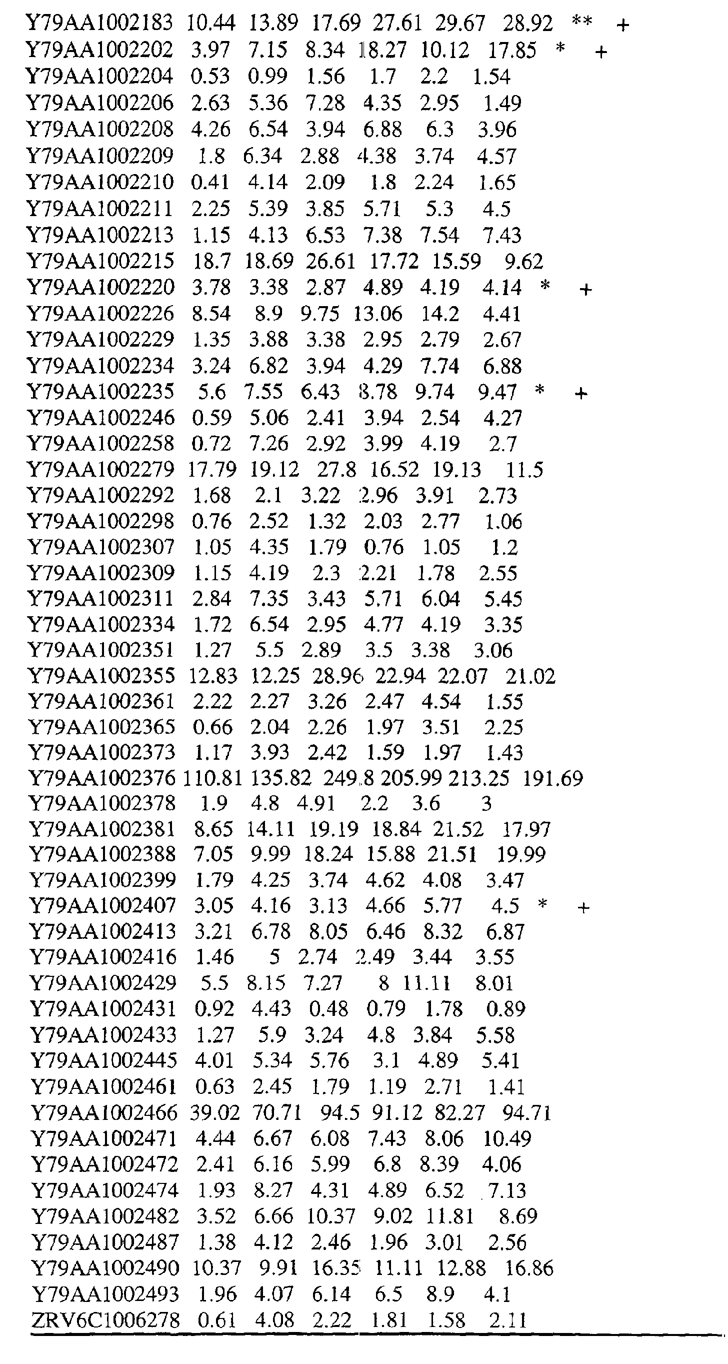

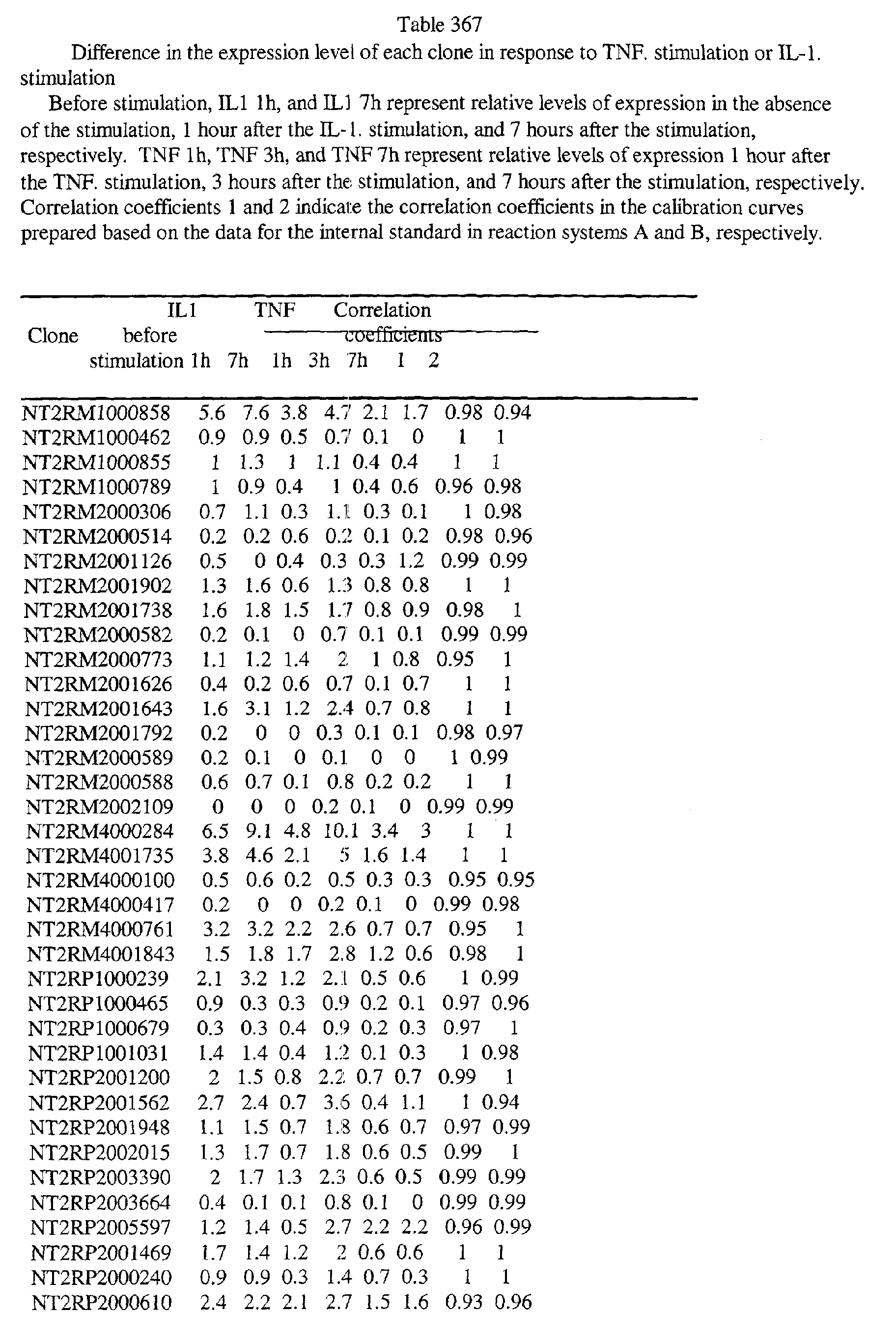

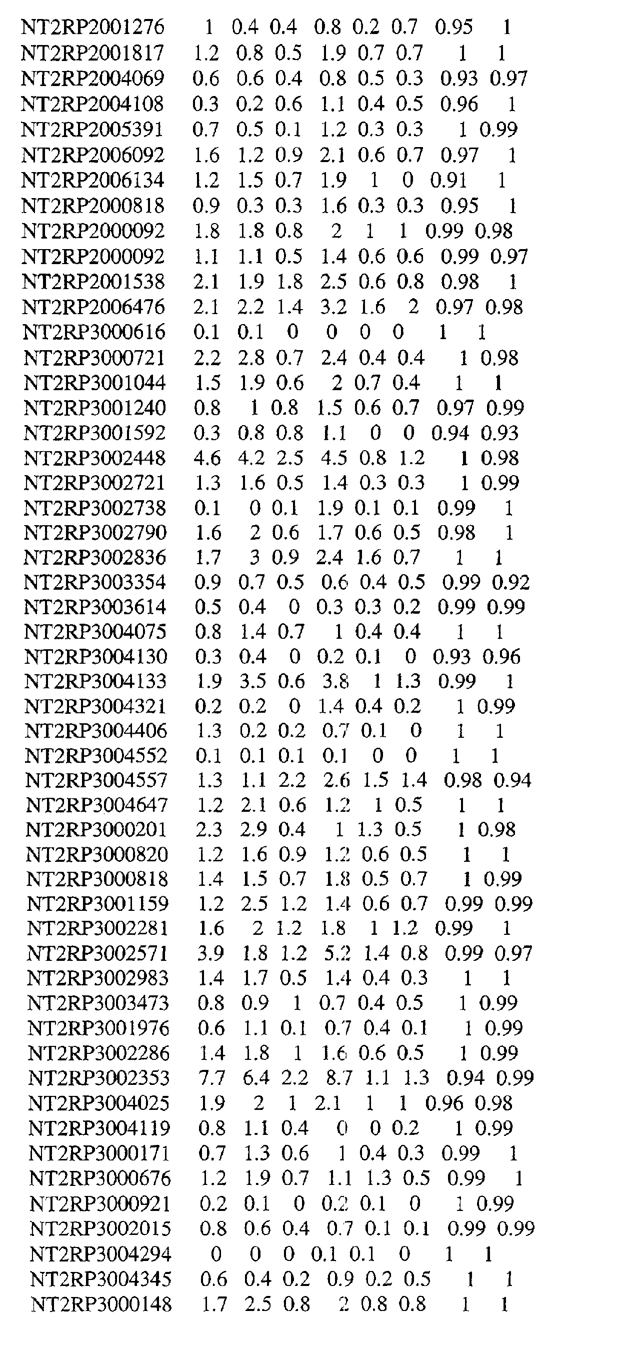

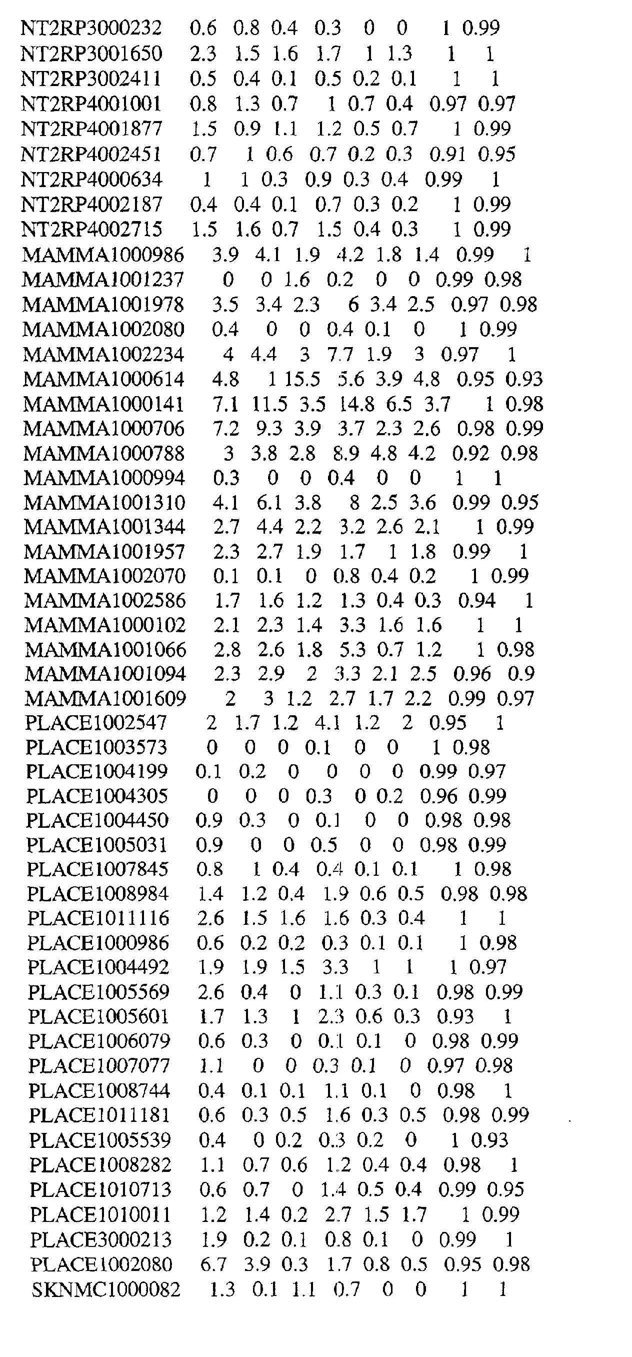

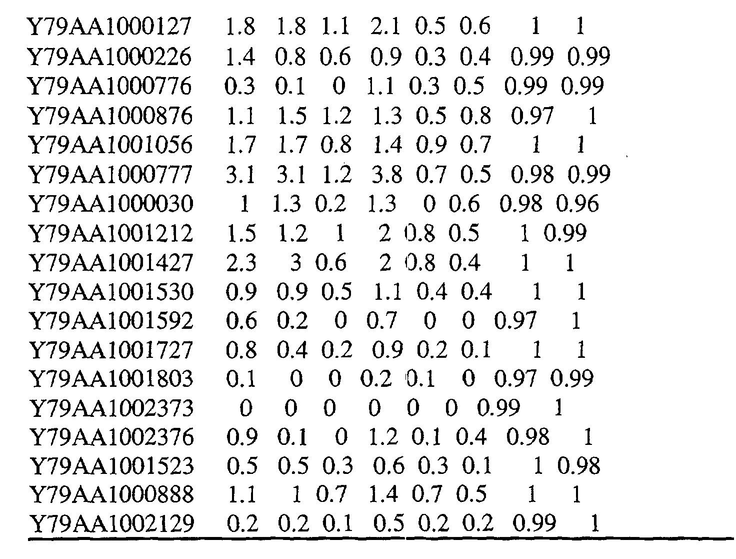

The present invention has provided a total of 830 novel full length cDNA clones. As has not yet proceeded the isolation of full length cDNA within the human, the invention has a large significance. Those proteins such as secretory proteins, membrane proteins, and proteins associated with signal transduction, glycoprotein, and transcription are known to be associated with many diseases. Those genes and proteins associating with diseases are useful for developing medicines as they can be used as a diagnostic marker, or a target for gene therapy or developing medicines that is capable of regulating their expression and activity. Especially, the cDNA clones encoding a secretion protein are extremely important for medicinal industry since the protein itself is expected to be effective as a medicine, and also the gene may have potential to be associating with many diseases. Moreover, those proteins such as membrane proteins, and proteins associated with signal transduction, glycoprotein, transcription, and diseases, and the genes encoding the proteins may be used as a disease marker. These cDNA clones are also important for medicinal industry as they may be effective for treating diseases through the regulation of the expression and activity of their encoded proteins.

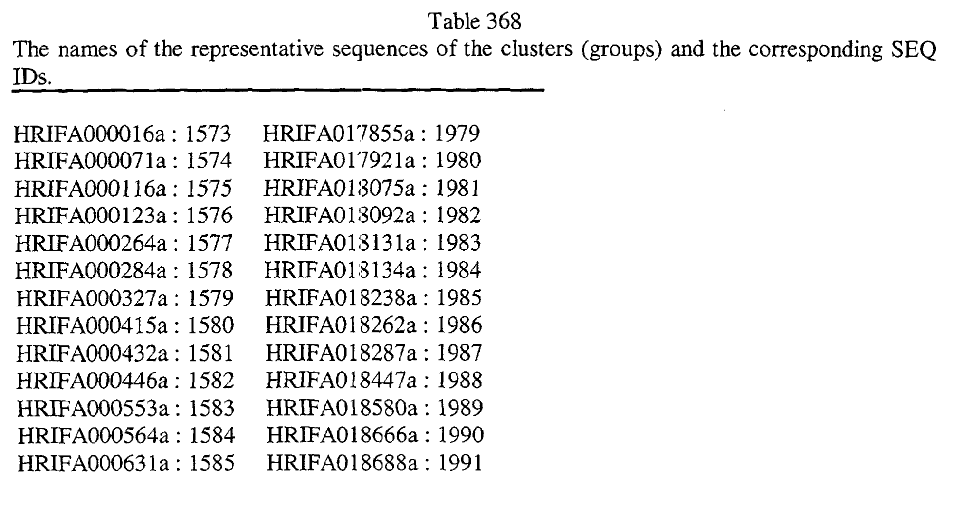

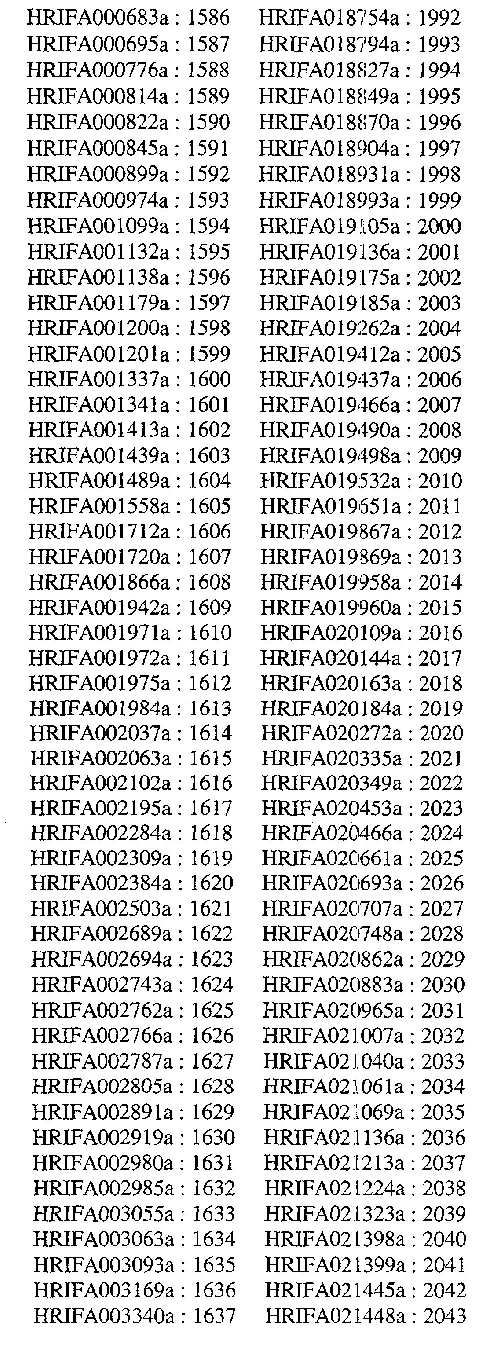

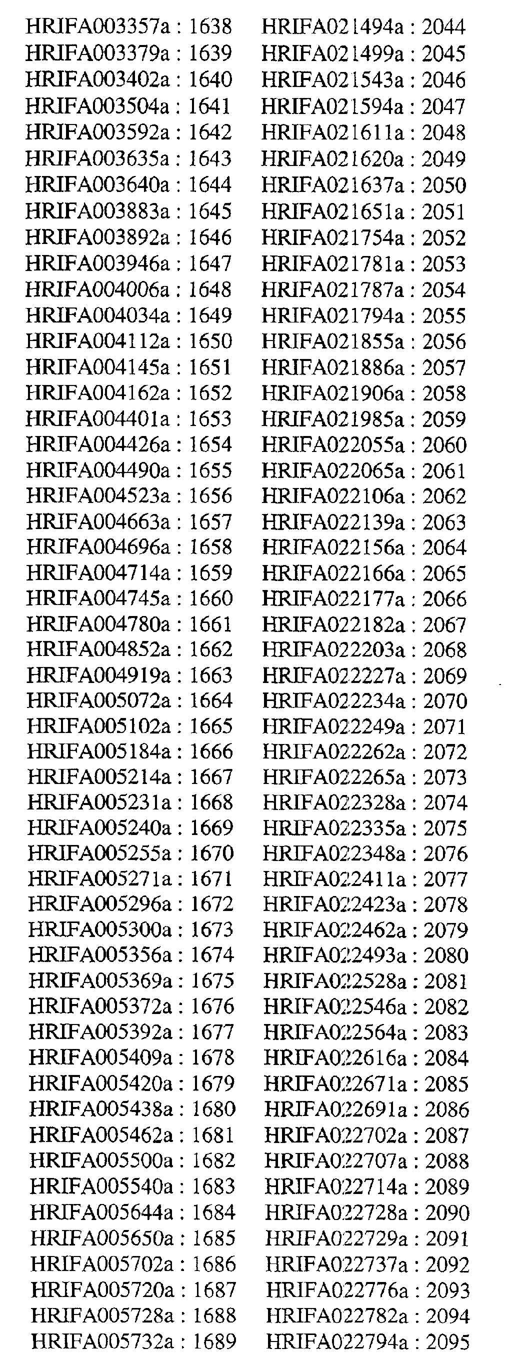

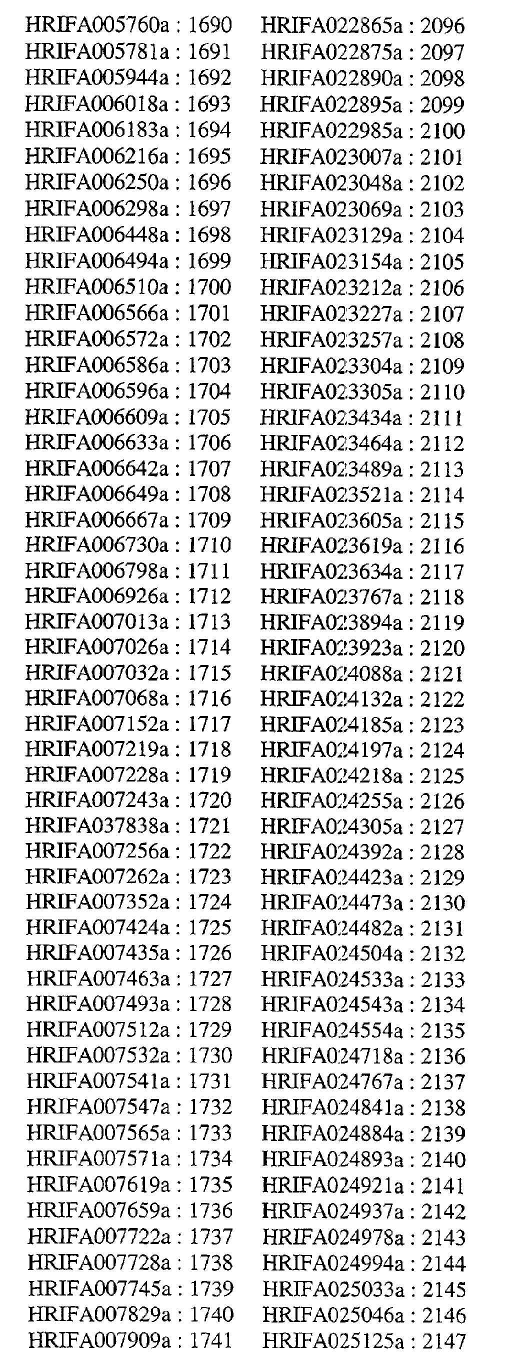

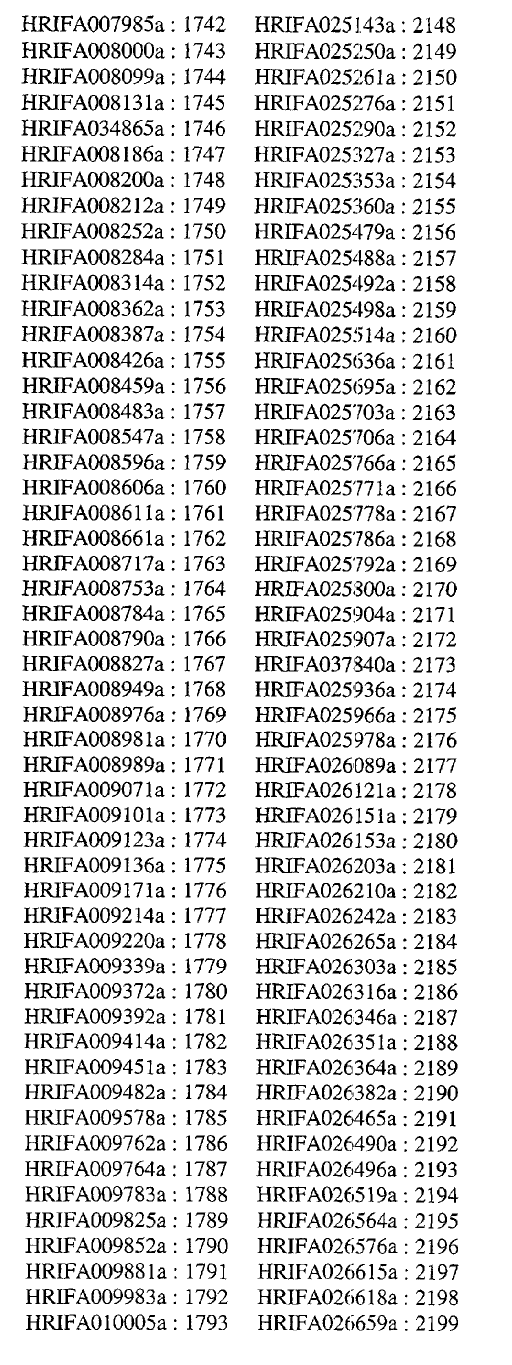

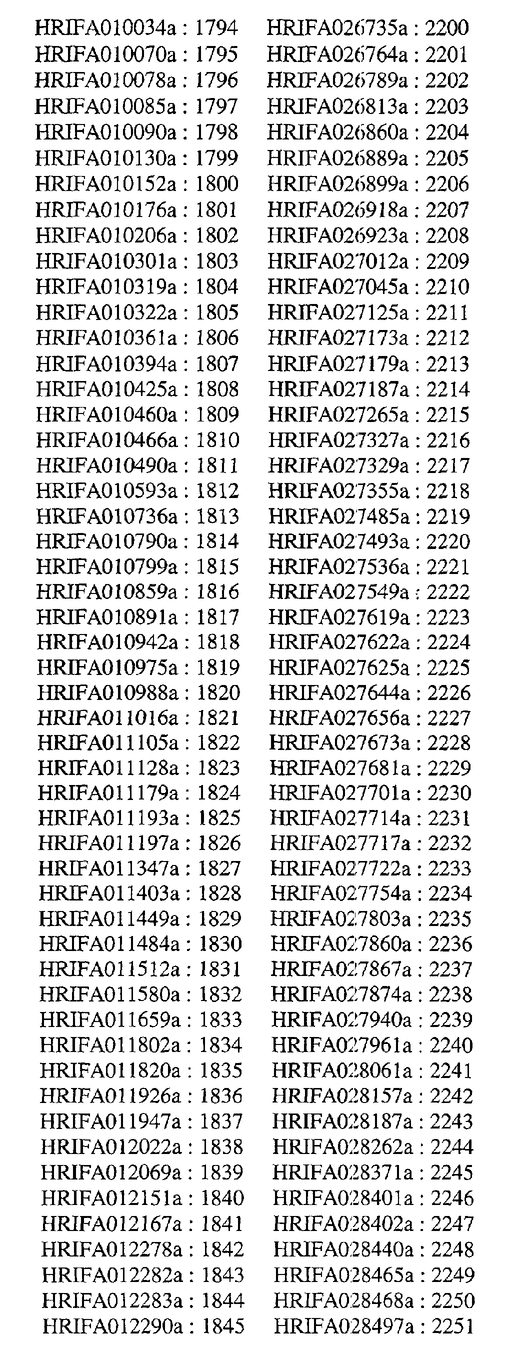

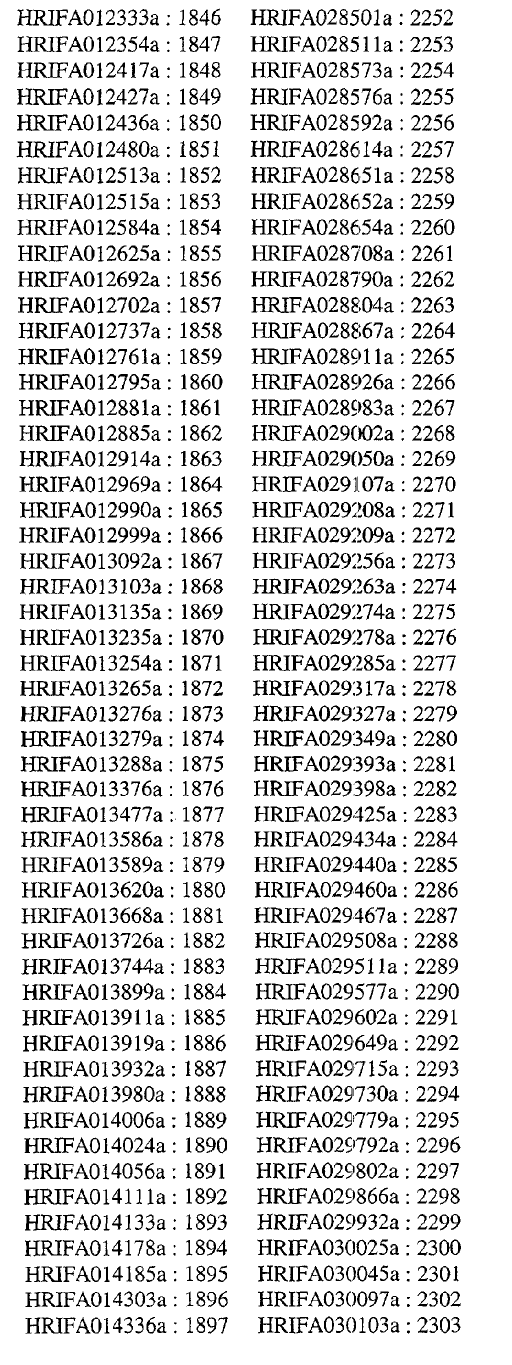

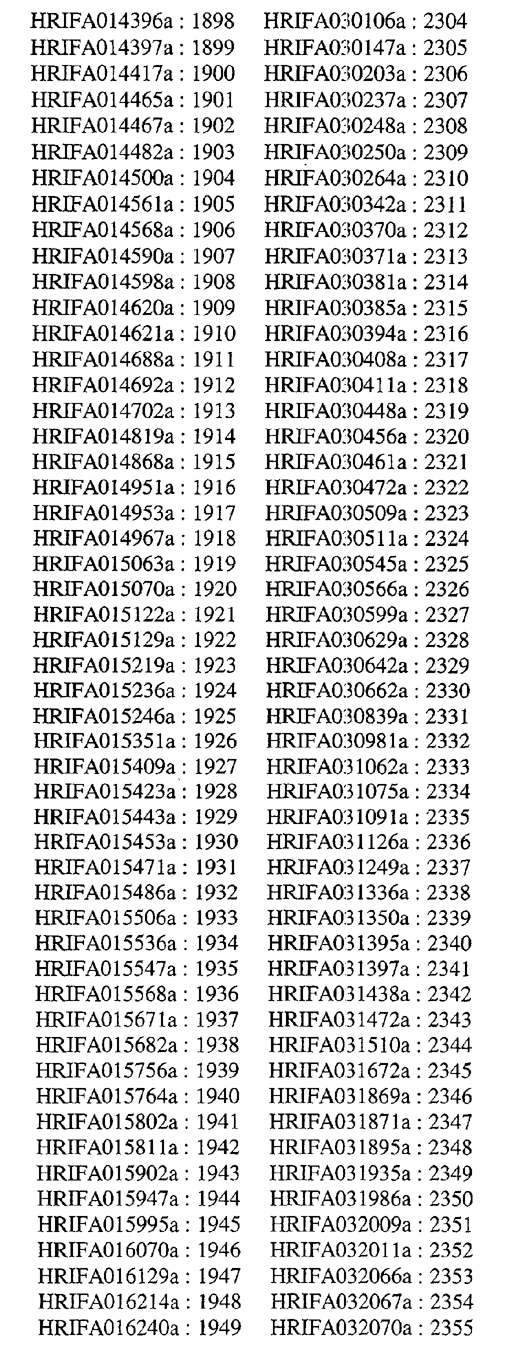

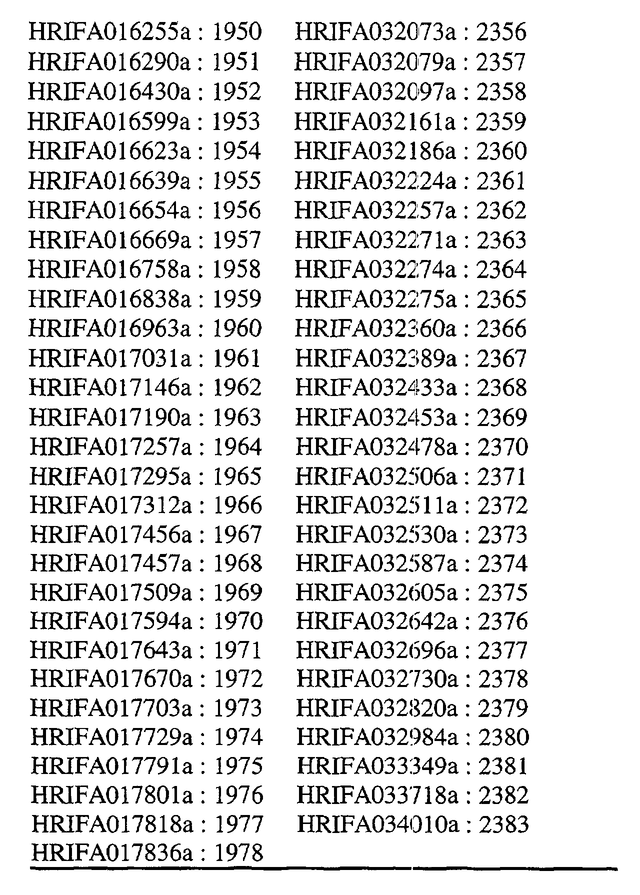

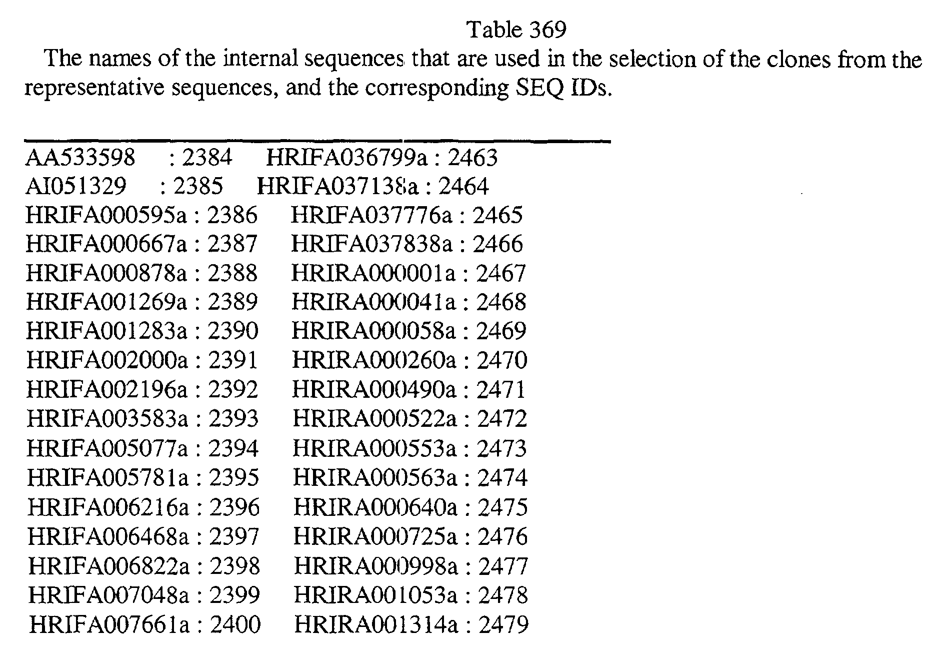

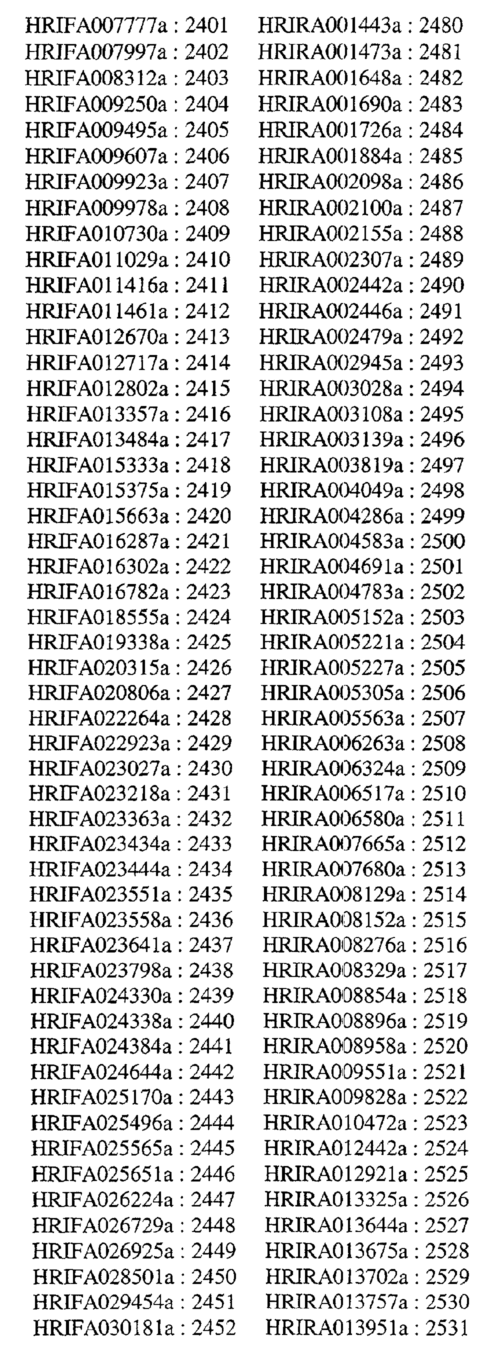

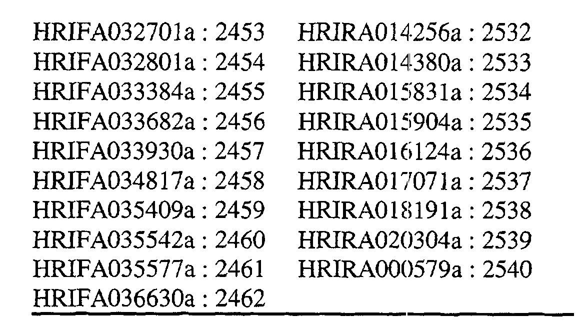

The internal sequences include EST, HRIFA(the representative sequence of the 5'-end), and HRIRA (the representative sequence of the 3'-end).

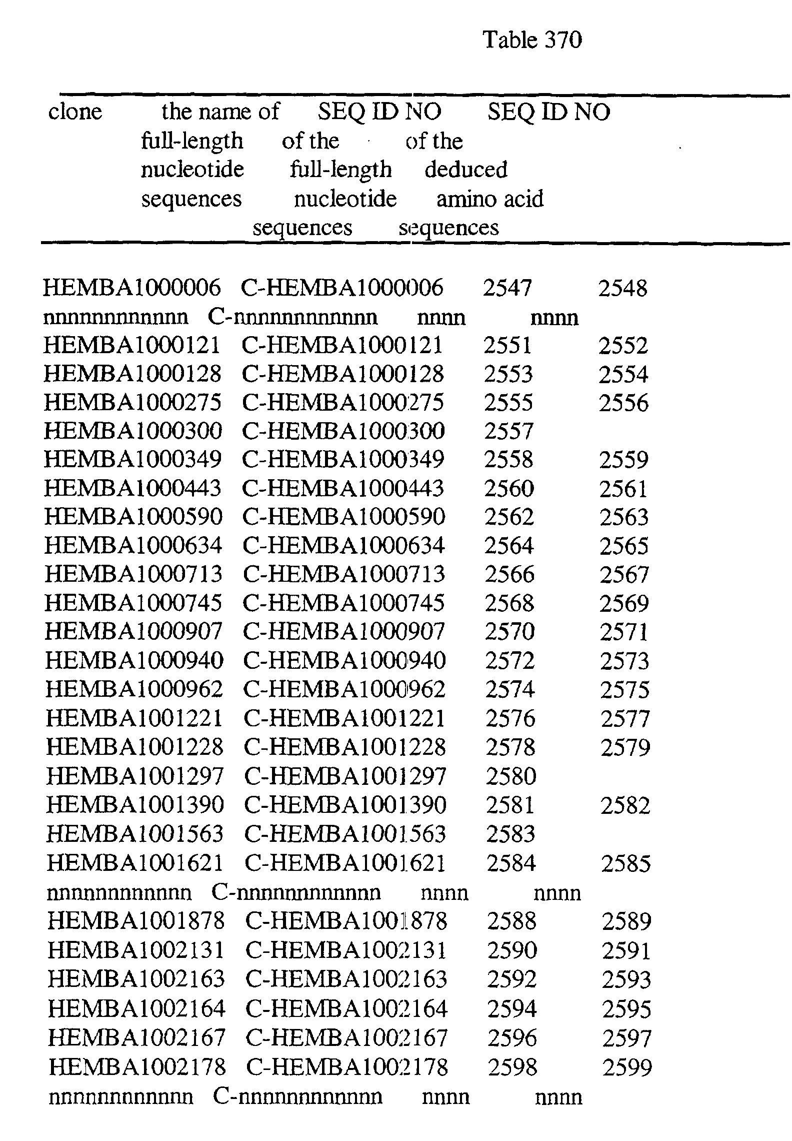

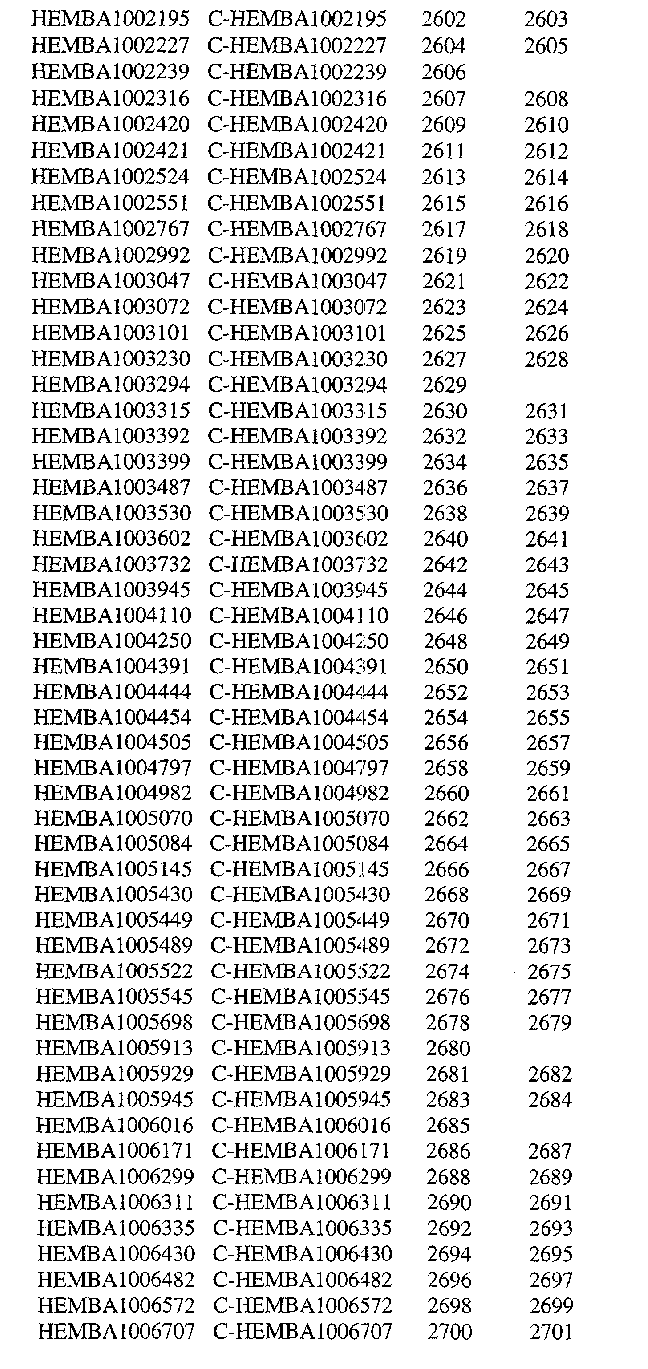

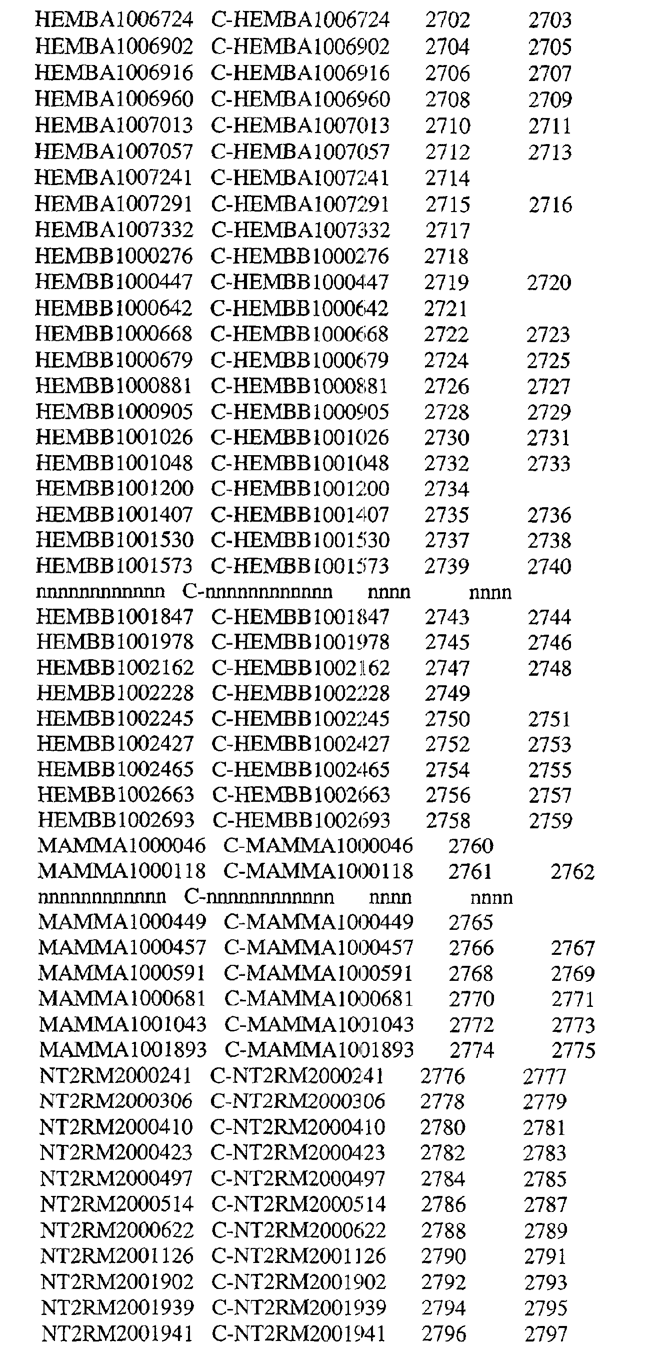

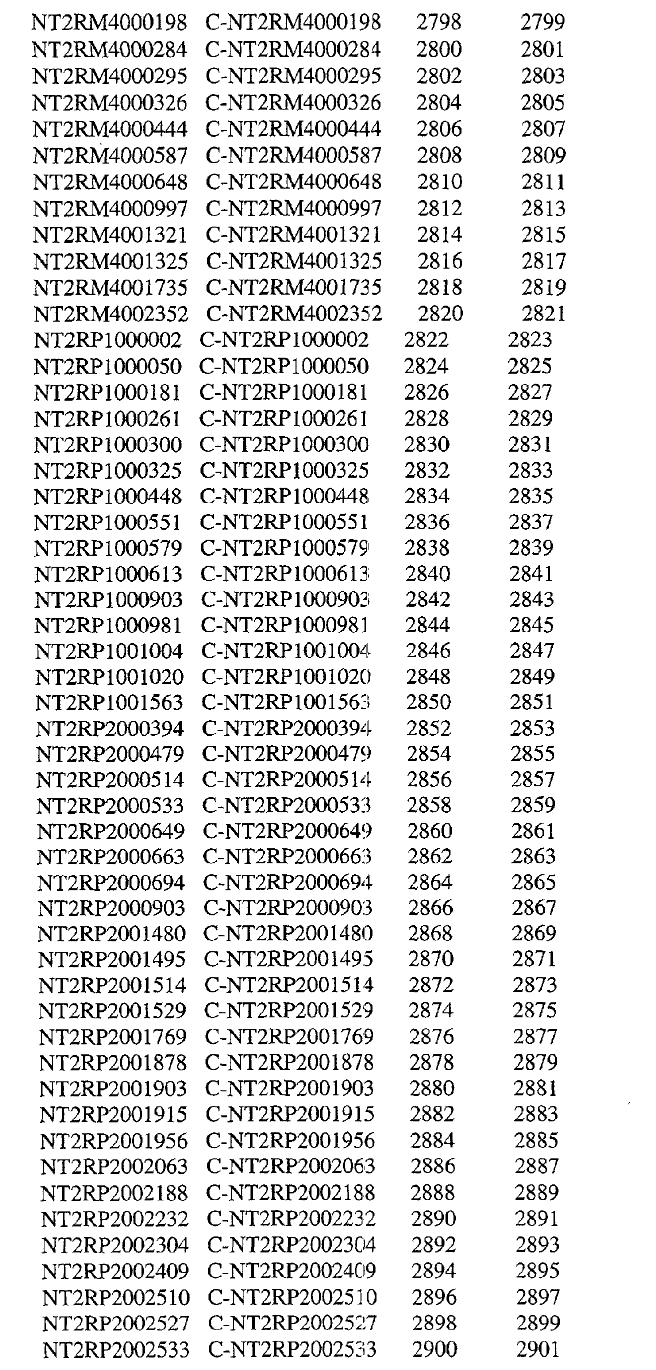

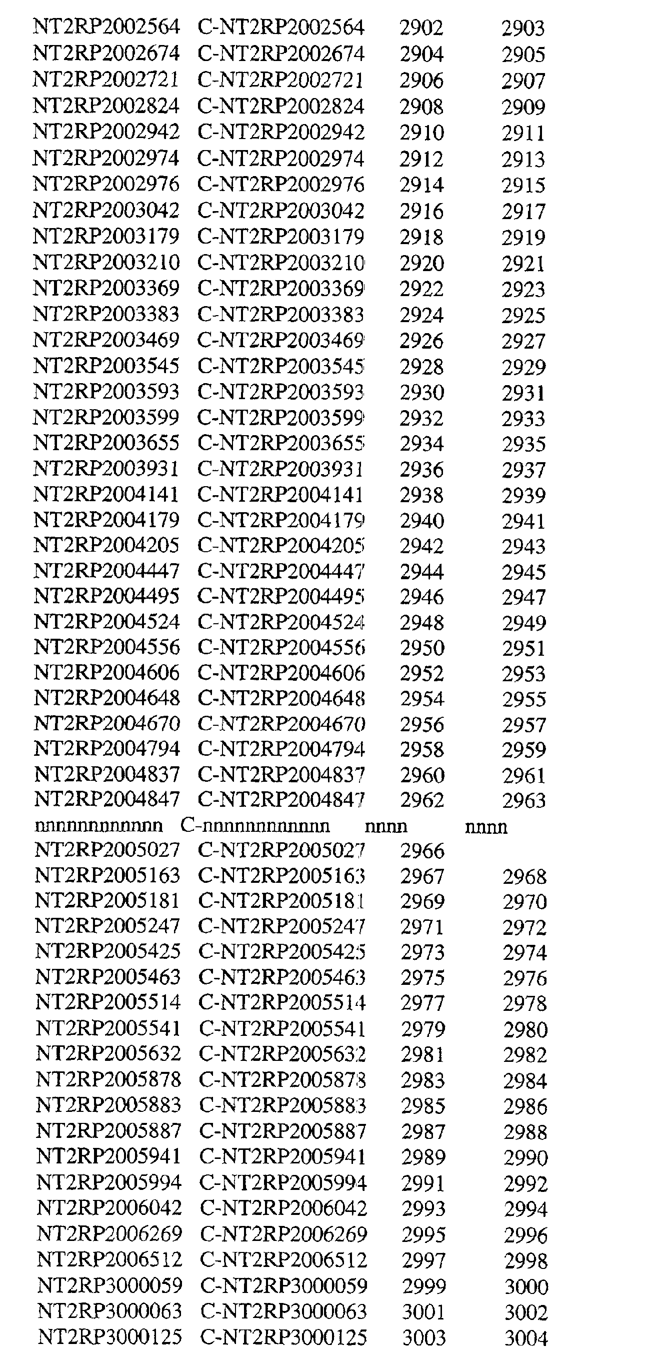

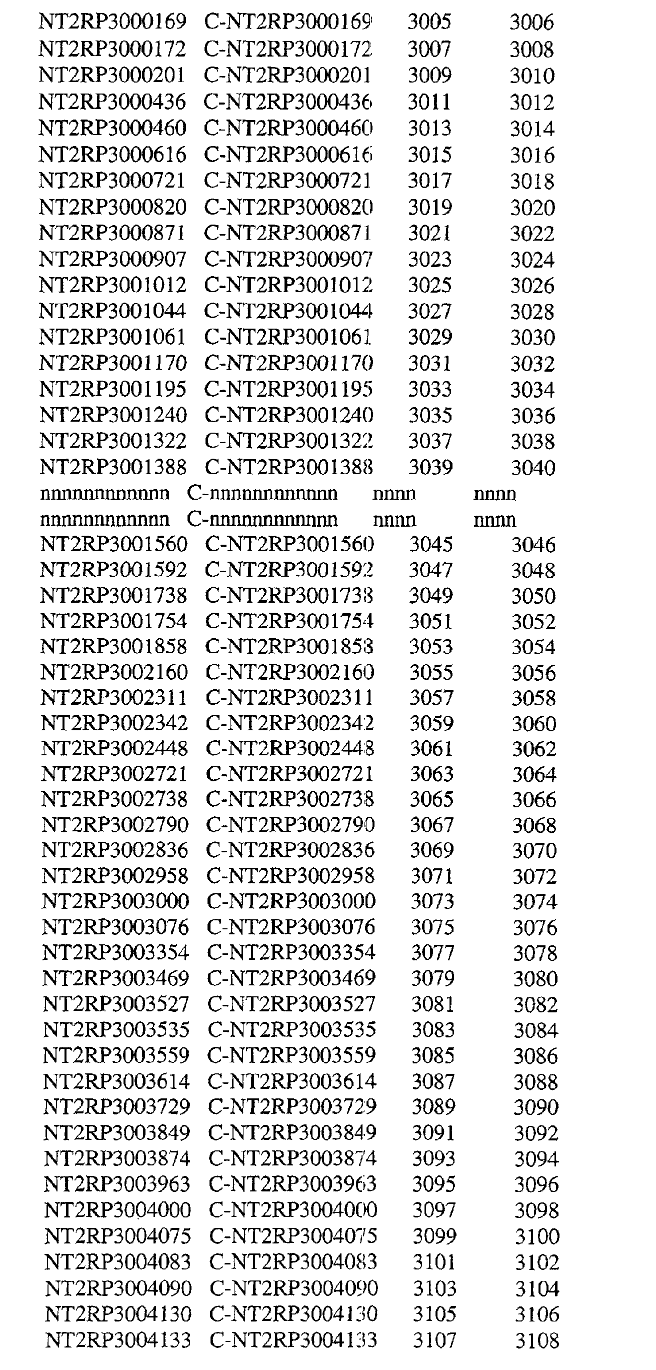

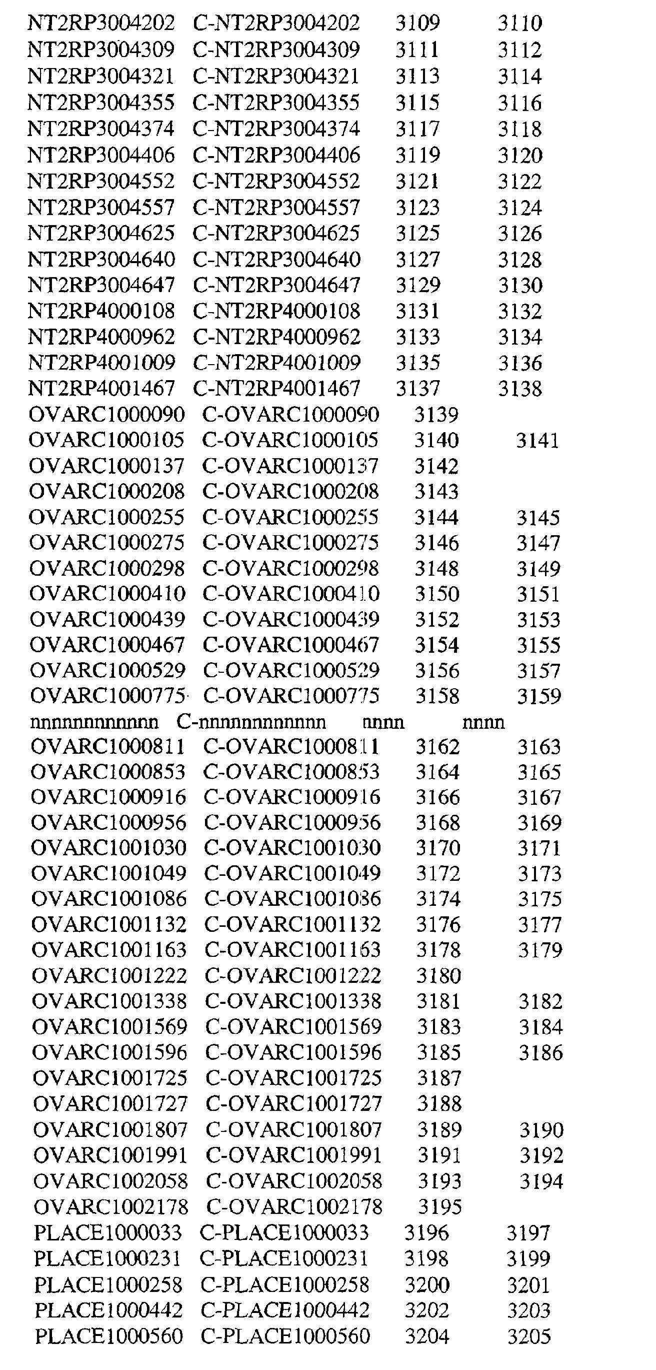

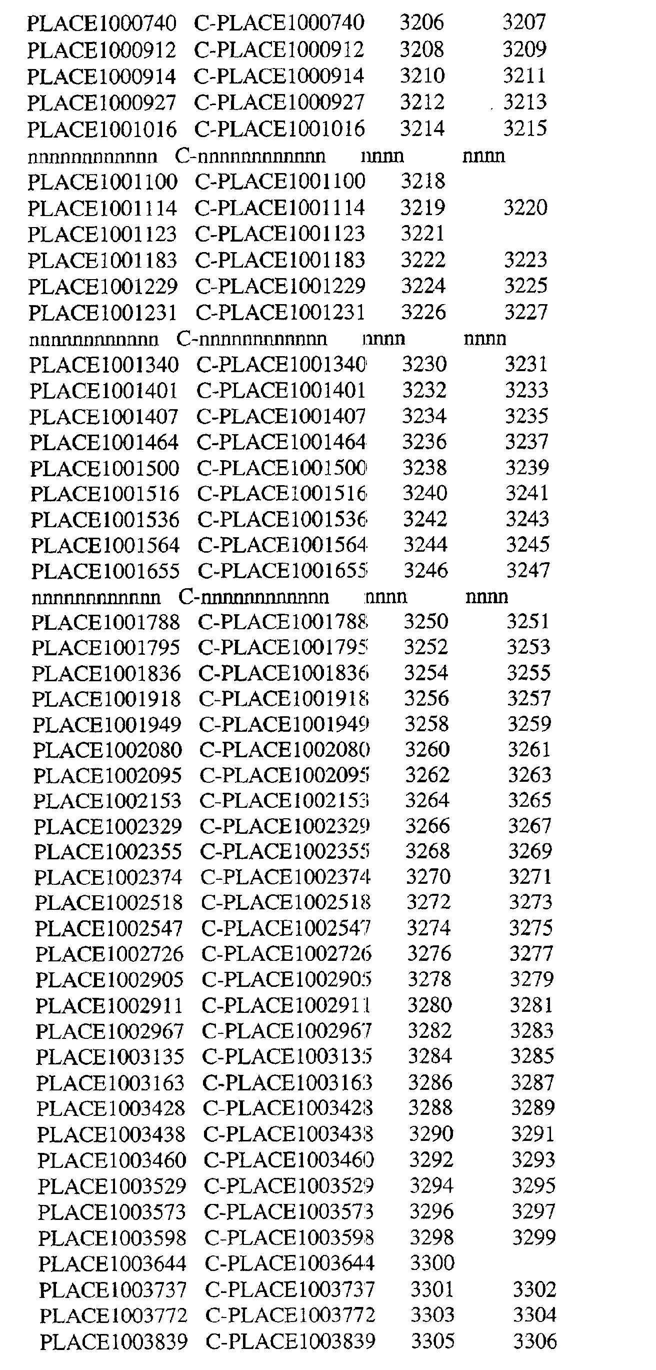

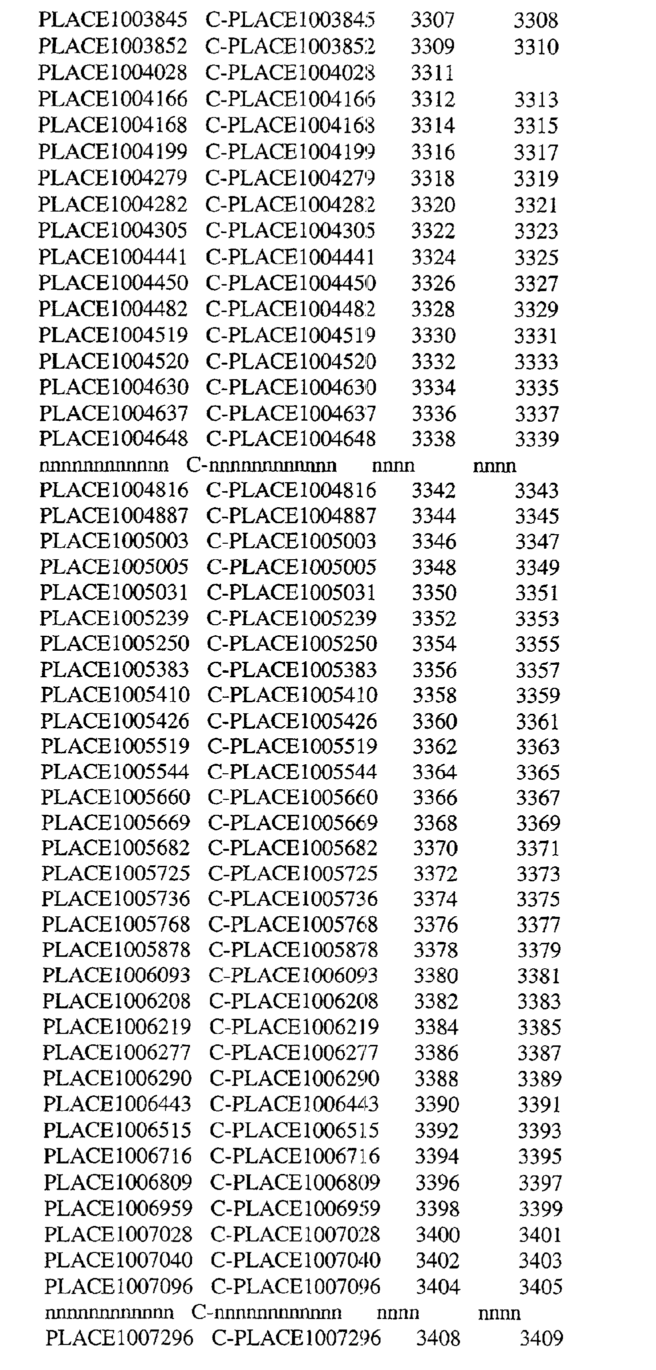

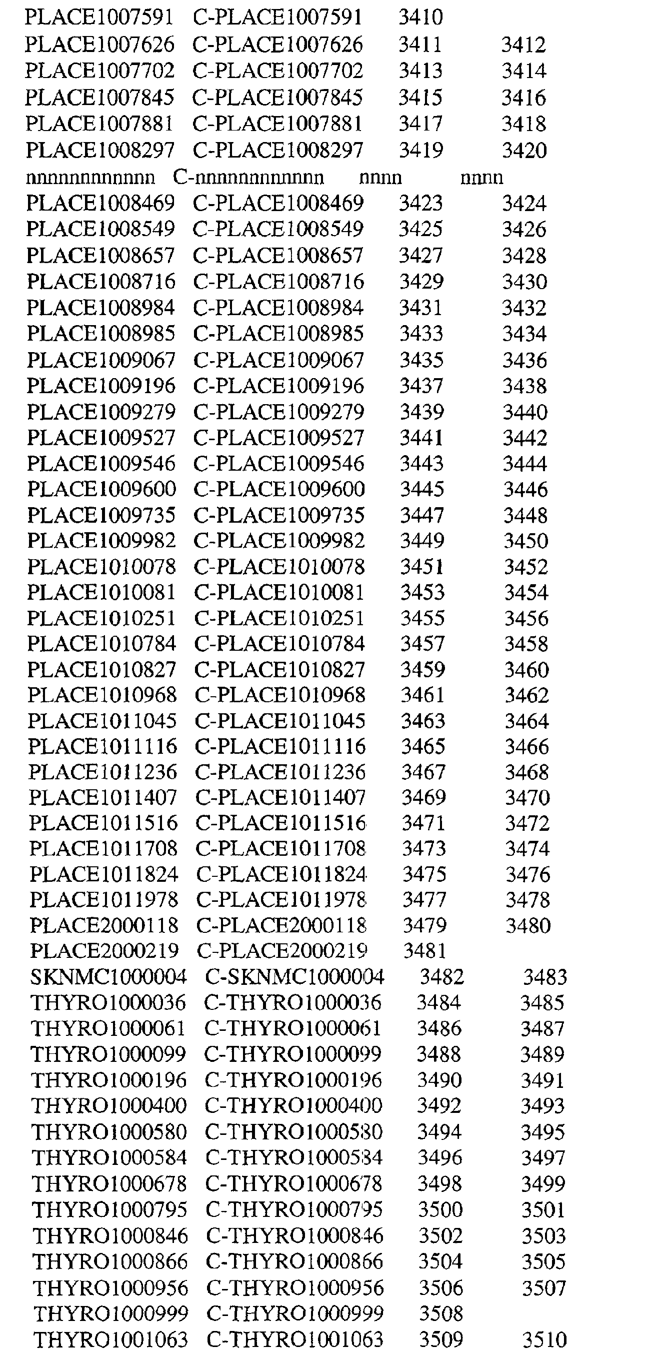

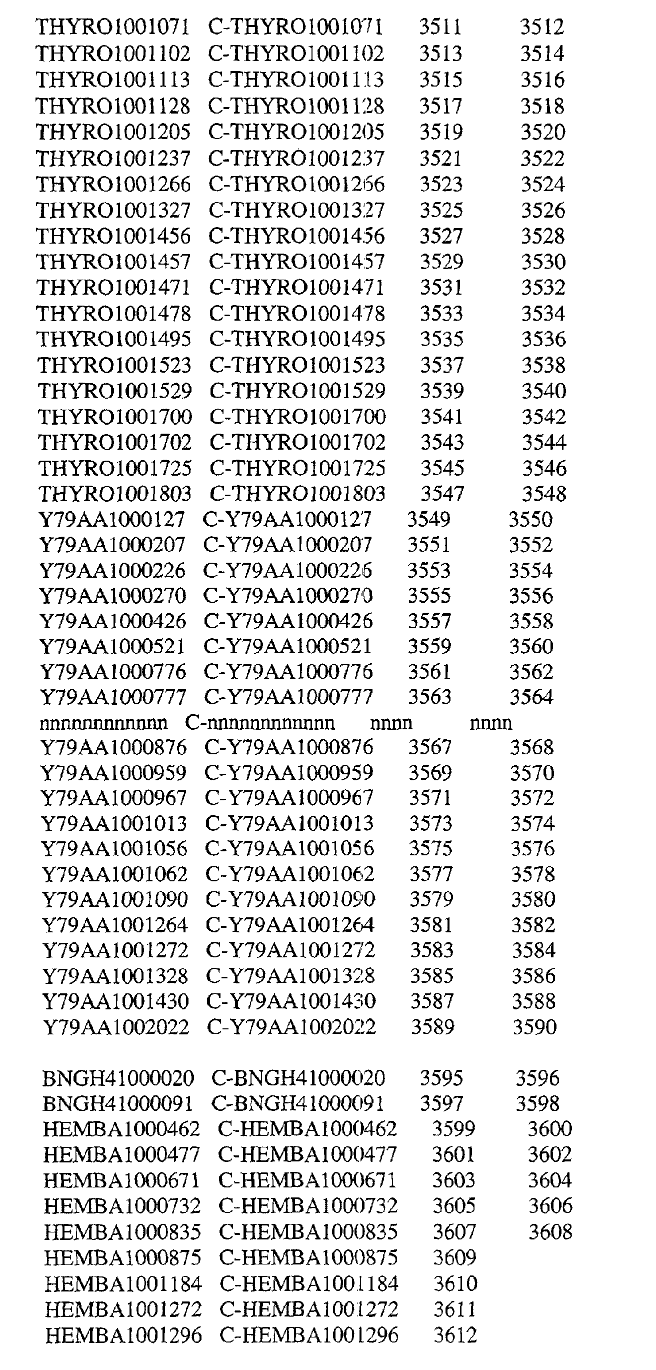

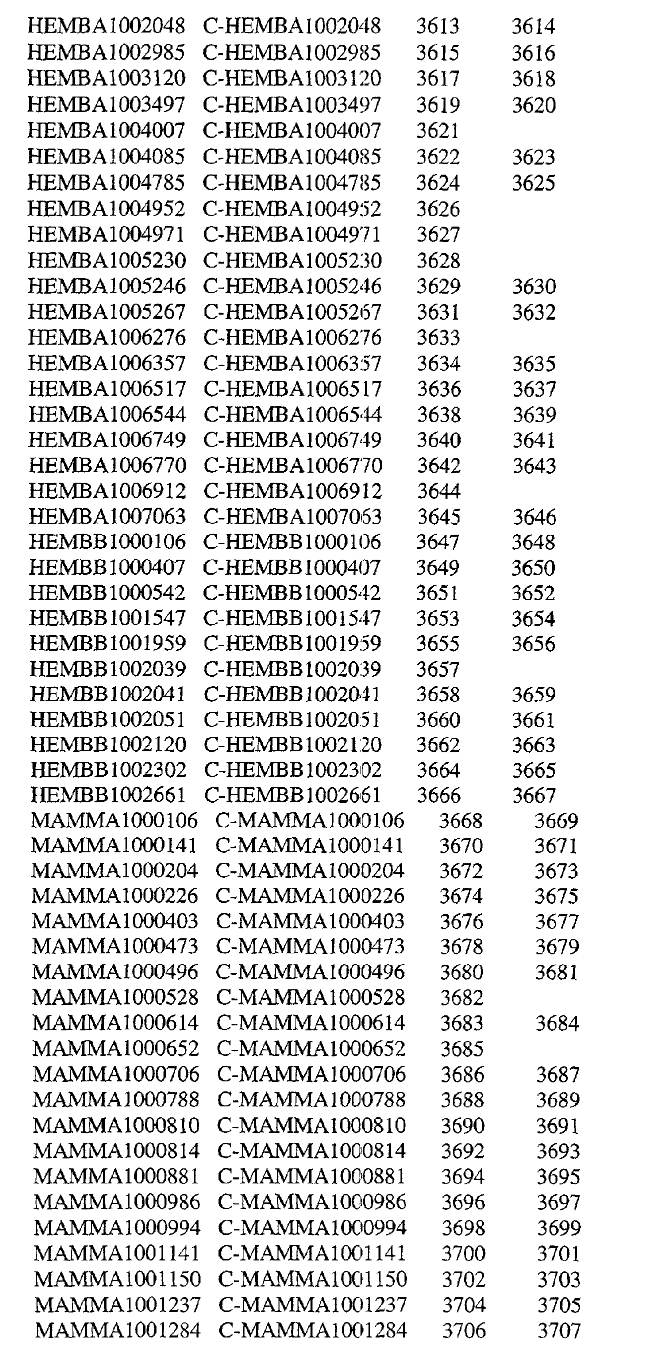

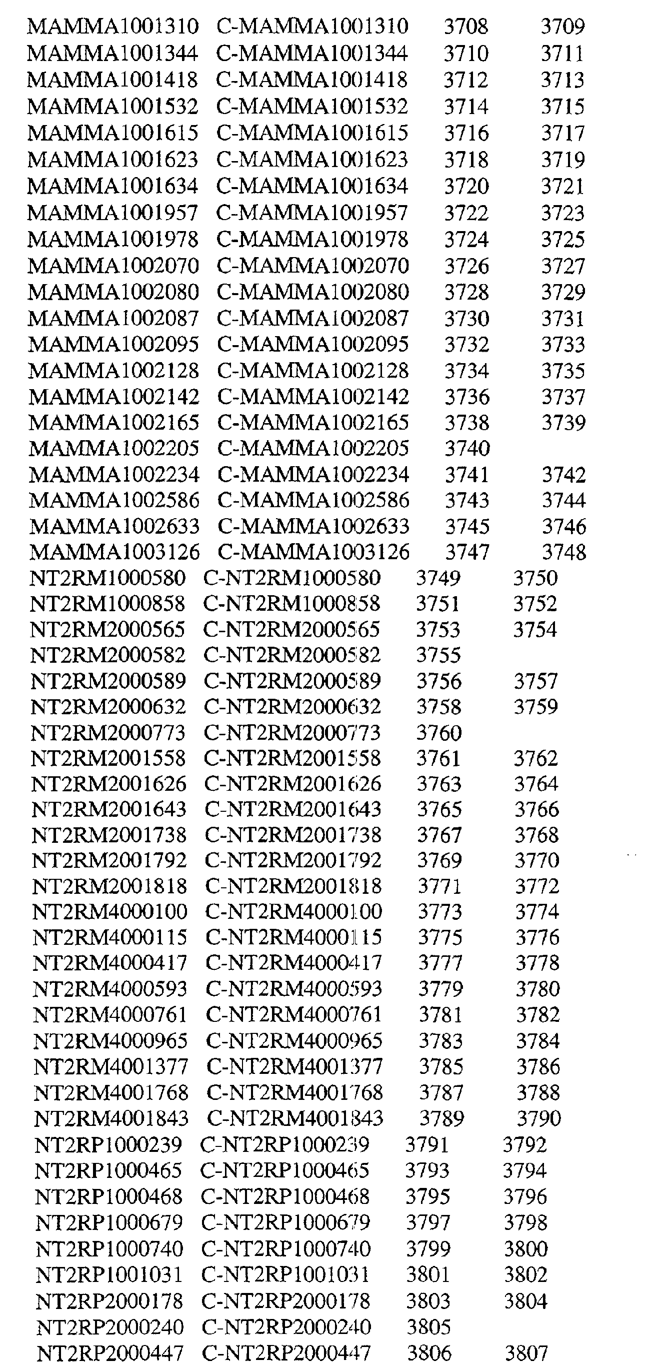

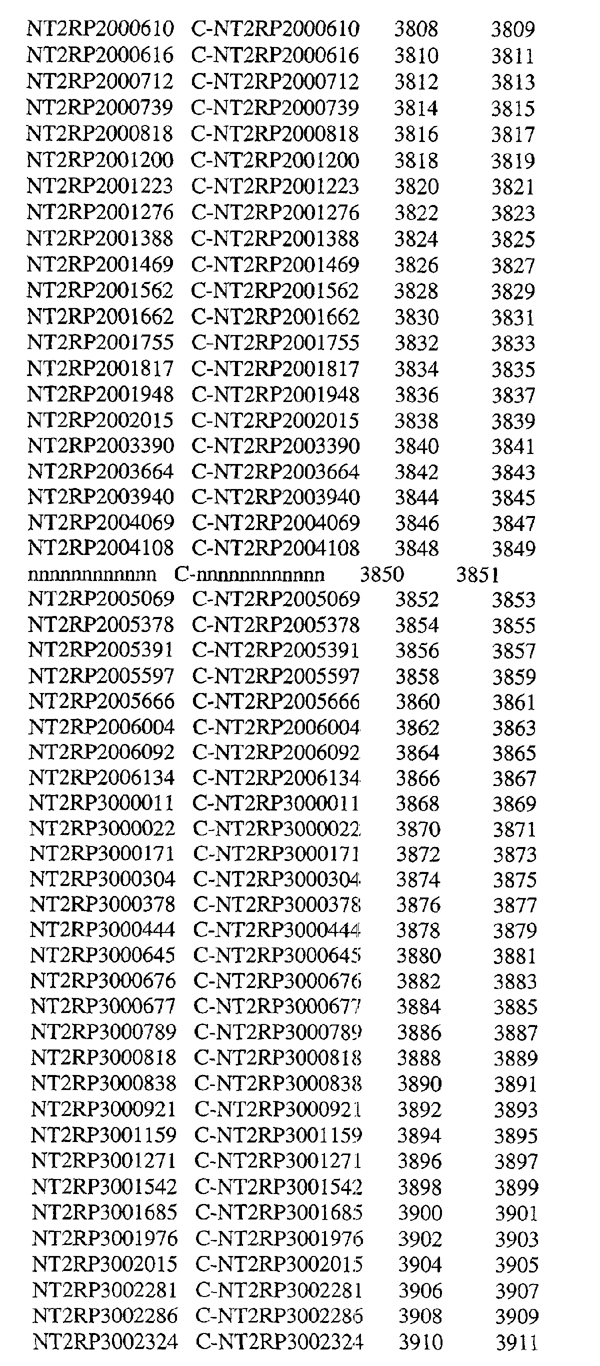

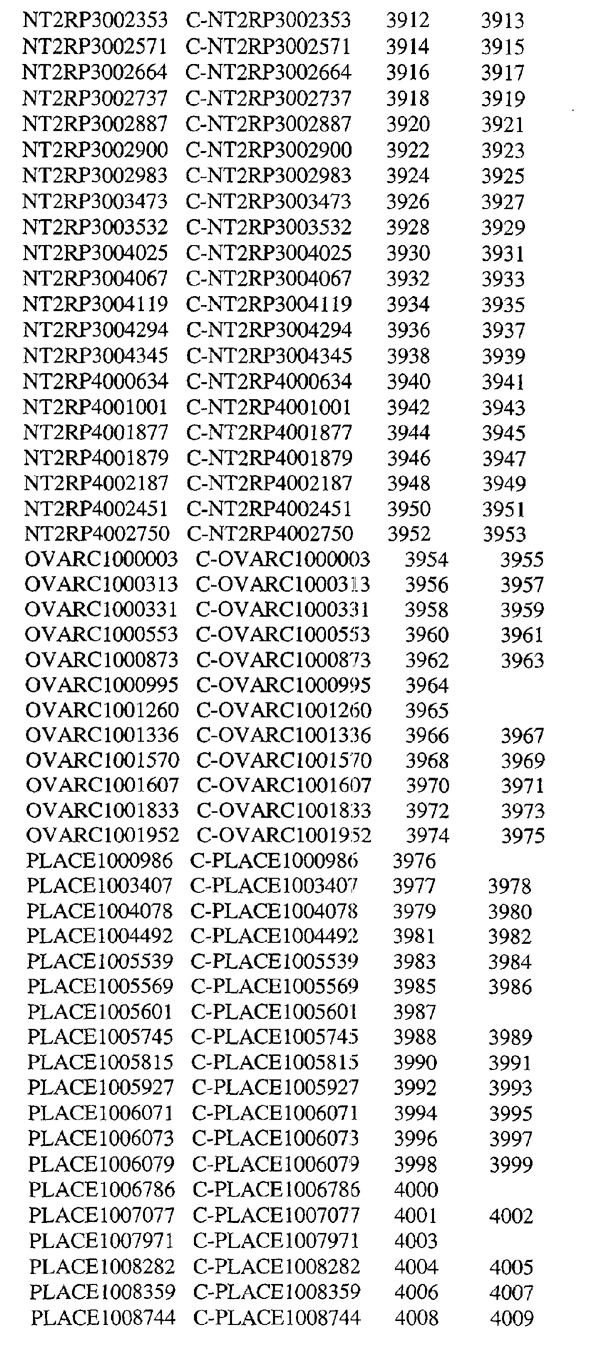

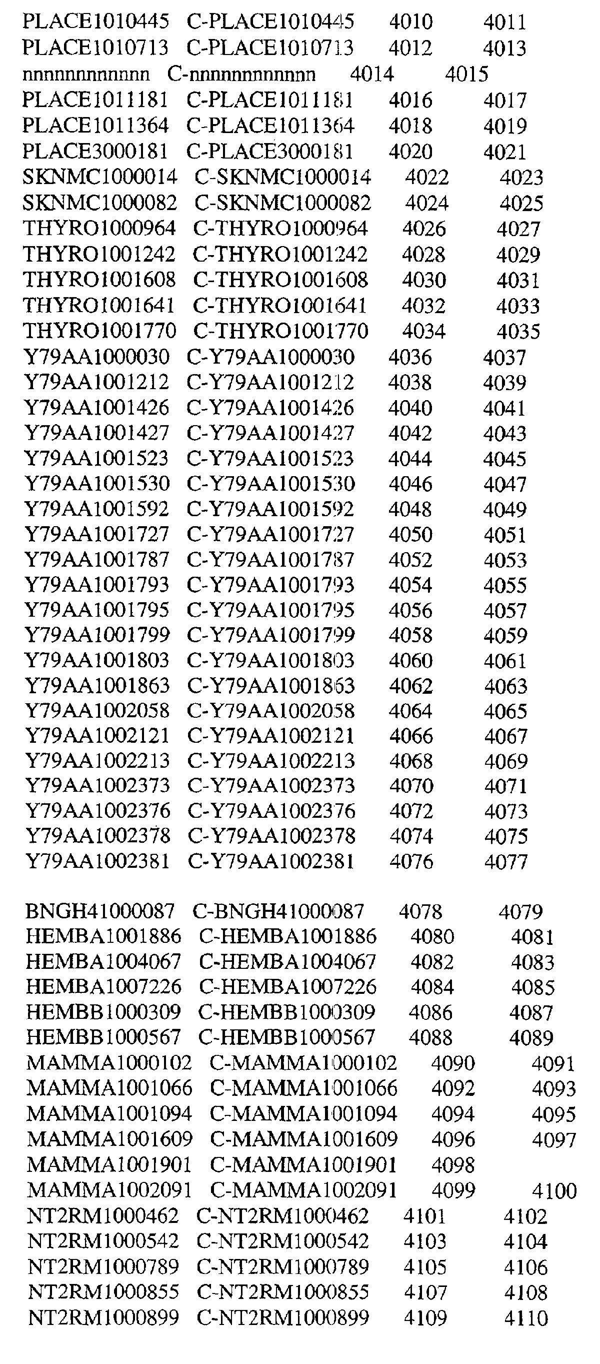

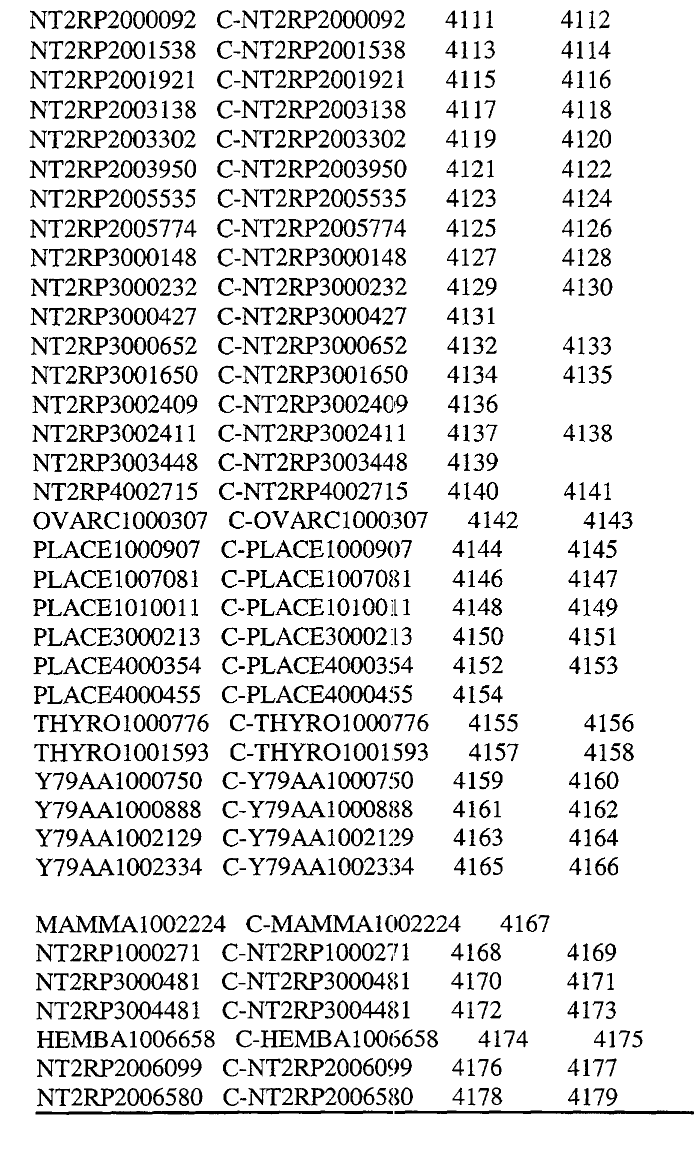

### Homology search result 1

The result of the homology search in the SwissProt using the representative sequences of the 5'-ends.
Indicated are from the top,
the name of the representative sequence of the cluster,
definition of the top hit data,
the P-value: the length of the sequence used for comparison (nucleotide):similarity (%),
the organism of which the top hit data is obtained,
the Accession No. of the top hit data.

Homology search results of the representative sequences of the 5'-end cluster to the data in SwissProt database are shown only for the representative sequences of the cluster from which clones were selected based on the homology search results.

The P-value is the score which is determined by taking into account the statistic probability of occurrence between the two sequences, and generally low score reflects high similarity. (Altschul, S.F., Gish, W., Miller, W., Myers, E.W. & Lipman, D.J. (1990) "Basic local alignment search tool." J. Mol. Biol. 215:403-410; Gish, W. & States, D.J. (1993) "Identification of protein coding regions by database similarity search." Nature Genet. 3:266-272).
HRIFA000016a
   GLYCINE-RICH CELL WALL STRUCTURAL PROTEIN 1.8 PRECURSOR (GRP 1.8).
   9.2e-05:178:32
   PHASEOLUS VULGARIS (KIDNEY BEAN) (FRENCH BEAN).
   P10496
HRIFA000071a
   CIRCUMSPOROZOITE PROTEB PRECURSOR (CS).
   5.8e-05:194:29
   PLASMODIUM SIMIUM.
   Q03110
HRIFA000116a
   HYPOTHETICAL 68.7 KD PROTEIN ZK757.1 IN CHROMOSOME III.
   6.2e-06:83:27
   CAENORHABDITIS ELEGANS.
   P34679
HRIFA000123a
   PATHOGENESIS-RELATED PROTEIN 1 PRECURSOR (PR-1).
   6.2e-08:89:34
   ARABIDOPSIS THALIANA (MOUSE-EAR CRESS).
   P33154
HRIFA000264a
   PROCOLLAGEN ALPHA 1(I) CHAIN PRECURSOR.
   1.4e-06:231:34
   GALLUS GALLUS (CHICKEN).
   P02457
HRIFA000327a
   ATP-BINDING CASSETTE TRANSPORTER 1.
   2.0e-16:238:31
   MUS MUSCULUS (MOUSE).
   P41233
HRIFA000415a
   PROLINE-RICH PROTEIN MP-2 PRECURSOR.
   3.6e-06:120:35
   MUS MUSCULUS (MOUSE).
   P05142
HRIFA000432a
   PUTATIVE GENERAL NEGATIVE REGULATOR OF TRANSCRIPTION C16C9.04C.
   2.2e-21:86:52
   SCHIZOSACCHAROMYCES POMBE (FISSION YEAST).
   Q09818
HRIFA000446a
   HYPOTHETICAL 64.8 KD PROTEIN IN GDI1-COX15 INTERGENIC REGION.
   2.5e-09:138:34
   SACCHAROMYCES CEREVISIAE (BAKER'S YEAST).
   P40085
HRIFA000553a
   CARTILAGE MATRIX PROTEIN PRECURSOR (MATRILIN-1).
   1.7e-27:117:48
   GALLUS GALLUS (CHICKEN).
   P05099
HRIFA000564a
   ERYTHROCYTE BAND 7 INTEGRAL MEMBRANE PROTEIN (STOMATIN) (PROTEIN 7.2B).
   2.9e-28:163:38
   MUS MUSCULUS (MOUSE).
   P54116
HRIFA000631a
   ZINC FINGER PROTEIN 140.
   8.2e-45:155:47
   HOMO SAPIENS (HUMAN).
   P52738
HRIFA000683a
   FIBRILLIN 1 PRECURSOR.
   4.8e-18:77:46
   HOMO SAPIENS (HUMAN).
   P35555
HRIFA000695a
   "SALIVARY PROLINE-RICH PROTEIN PRECURSOR (CLONES CP3, CP4 AND CP5) [CONTAINS: BASIC PEPTIDE IB-6" PEPTIDE P-H].
   4.0e-06:105:33
   HOMO SAPIENS (HUMAN).
   P04280
HRIFA000776a
   FIBRILLIN 2 PRECURSOR.
   1.6e-42:214:44
   HOMO SAPIENS (HUMAN).
   P35556
HRIFA000814a
   ZINC FINGER PROTEIN 133.
   4.4e-16:49:87
   HOMO SAPIENS (HUMAN).
   P52736
HRIFA000845a
   PROCOLLAGEN ALPHA 1(I) CHAIN PRECURSOR.
   6.0e-06:172:34
   MUS MUSCULUS (MOUSE).
   P11087
HRIFA001099a
   CYCLIC-AMP-DEPENDENT TRANSCRIPTION FACTOR ATF-5 (FRAGMENT).
   0.92:38:34
   HOMO SAPIENS (HUMAN).
   P18849
HRIFA001132a
   AGRIN PRECURSOR.
   1.3e-26:239:32
   GALLUS GALLUS (CHICKEN).
   P31696
HRIFA001138a
   CARTILAGE OLIGOMERIC MATRIX PROTEIN PRECURSOR (COMP).
   5.9e-114:147:83
   HOMO SAPIENS (HUMAN).
   P49747
HRIFA001200a
   TRANS-ACTING TRANSCRIPTIONAL PROTEIN ICP0 (P135 PROTEIN) (IER 2.9/ER2.6).
   0.035:119:34
   BOVINE HERPESVIRUS TYPE 1 (STRAIN JURA).
   P29128
HRIFA001337a
   LOW-DENSITY LIPOPROTEIN RECEPTOR PRECURSOR (LDL RECEPTOR).
   2.4e-17:98:42
   CRICETULUS GRISEUS (CHINESE HAMSTER).
   P35950
HRIFA001341a
   NEUROFILAMENT TRIPLET L PROTEIN (68 KD NEUROFILAMENT PROTEIN) (NF-L)
   (NF68).
   1.2e-102:248:87
   RATTUS NORVEGICUS (RAT).
   P19527
HRIFA001413a
   BACTENECIN 7 PRECURSOR (BAC7) (PR-59).
   0.0032:33:63
   BOS TAURUS (BOVINE).
   P19661
HRIFA001439a
   B-CELL GROWTH FACTOR PRECURSOR (BCGF-12 KD).
   0.00031:34:61
   HOMO SAPIENS (HUMAN).
   P20931
HRIFA001489a
   PROBABLE G PROTEIN-COUPLED RECEPTOR APJ.
   8.4e-65:105:72
   HOMO SAPIENS (HUMAN).
   P35414
HRIFA001558a
   HISTIDINE-RICH GLYCOPROTEIN PRECURSOR.
   0.0048:80:31
   PLASMODIUM LOPHURAE.
   P04929
HRIFA001712a
   PUTATIVE SERINE/THREONINE-PROTEIN KINASE PKWA (EC 2.7.1.-).
   2.5e-19:169:31
   THERMOMONOSPORA CURVATA.
   P49695
HRIFA001720a
   ZINC FINGER PROTEIN 85 (ZINC FINGER PROTEIN HPF4) (HTF1).
   1.4e-94:273:64
   HOMO SAPIENS (HUMAN).
   Q03923
HRIFA001866a
   EARLY ANTIGEN PROTEIN D (EA-D).
   0.10:93:34
   EPSTEIN-BARR VIRUS (STRAIN B95-8) (HUMAN HERPESVIRUS 4).
   P03191
HRIFA001942a
   "PROCOLLAGEN-LYSINE,2-OXOGLUTARATE 5-DIOXYGENASE 1 PRECURSOR (EC 1.14.11.4) (LYSYL HYDROXYLASE 1) (LH1)."
   4.7e-12:140:30
   GALLUS GALLUS (CHICKEN).
   P24802
HRIFA001971a
   HYPOTHETICAL 46.3 KD PROTEIN IN PTA1-CDC24 INTERGENIC REGION.
   2.5e-10:86:30
   SACCHAROMYCES CEREVISIAE (BAKER'S YEAST).
   P39727
HRIFA001972a
   LAMININ ALPHA-1 CHAIN PRECURSOR (LAMININ A CHAIN).
   0.10:100:34
   MUS MUSCULUS (MOUSE).
   P19137
HRIFA001975a
   ACETYLCHOLINESTERASE PRECURSOR (EC 3.1.1.7).
   6.5e-30:243:33
   MUS MUSCULUS (MOUSE).
   P21836
HRIFA001984a
   "PROCOLLAGEN-LYSINE,2-OXOGLUTARATE 5-DIOXYGENASE 1 PRECURSOR (EC 1.14.11.4) (LYSYL HYDROXYLASE 1) (LH1)."
   1.2e-11:140:30
   GALLUS GALLUS (CHICKEN).
   P24802
HRIFA002063a
   GNS1 PROTEIN.
   1.3e-05:127:30
   SACCHAROMYCES CEREVISIAE (BAKERS YEAST).
   P25358
HRIFA002102a
   MUCIN 2 PRECURSOR (INTESTINAL MUCIN 2).
   2.9e-07:241:30
   HOMO SAPIENS (HUMAN).
   Q02817
HRIFA002284a
   ACIDIC PROLINE-RICH PROTEIN PRECURSOR (CLONE PRP33).
   3.8e-05:104:34
   RATTUS NORVEGICUS (RAT).
   P04474
HRIFA002309a
   PUTATIVE SERINE/THREONINE-PROTEIN KINASE PKWA (EC 2.7.1.-).
   1.5e-08:110:37
   THERMOMONOSPORA CURVATA.
   P49695
HRIFA002384a
   GAP JUNCTION ALPHA-6 PROTEIN (CONNEXIN 45) (CX45).
   1.8e-31:94:42
   HOMO SAPIENS (HUMAN).
   P36383
HRIFA002503a
   N-ACETYLLACTOSAMINE SYNTHASE (EC 2.4.1.90) (N-ACETYLGLUCOSAMINE (BETA 1→4)GALACTOSYLTRANSFERASE) (EC 2.4.1.38) (LACTOSE SYNTHASE A PROTEIN (EC 2.4.1.22)) (GALACTOSYLTRANSFERASE) (GT).
   6.1e-92:246:67
   MUS MUSCULUS (MOUSE).
   P15535
HRIFA002689a
   TRANSCRIPTION FACTOR GATA-6 (GATA BINDING FACTOR-6) (DNA BINDING PROTEIN GATA-GT2).
   0.38:49:34
   RATTUS NORVEGICUS (RAT).
   P46153
HRIFA002694a
   ANTER-SPECIFIC PROLINE-RICH PROTEIN APG PRECURSOR.
   4.7e-05:93:37
   ARABIDOPSIS THALIANA (MOUSE-EAR CRESS).
   P40602
HRIFA002743a
   BONE MORPHOGENETIC PROTEIN 1 PRECURSOR (EC 3.4.24.-) (BMP-1).
   1.2e-23:216:31
   HOMO SAPIENS (HUMAN).
   P13497
HRIFA002762a
   PROLINE-RICH PROTEIN MP-2 PRECURSOR.
   5.1e-09:129:41
   MUS MUSCULUS (MOUSE).
   P05142
HRIFA002766a
   FIBROMODULIN PRECURSOR (FM) (COLLAGEN-BINDING 59 KD PROTEIN).
   1.8e-12:139:34
   HOMO SAPIENS (HUMAN).
   Q06828
HRIFA002787a
   PROCOLLAGEN ALPHA 2(I) CHAIN PRECURSOR.
   1.6e-10:124:37
   HOMO SAPIENS (HUMAN).
   P08123
HRIFA002805a
   ZINC FINGER PROTEIN 140.
   3.6e-23:43:74
   HOMO SAPIENS (HUMAN).
   P52738
HRIFA002891a
   "FIBULIN-1, ISOFORM C PRECURSOR (BASEMENT-MEMBRANE PROTEIN 90) (BM-90)."
   2.0e-41:239:39
   MUS MUSCULUS (MOUSE).
   Q08878
HRIFA002919a
   BEM46 PROTEIN (FRAGMENT).
   1.0e-12:171:32
   SCHIZOSACCHAROMYCES POMBE (FISSION YEAST).
   P54069
HRIFA002980a
   LOW-DENSITY LIPOPROTEIN RECEPTOR-RELATED PROTEIN 1 PRECURSOR (LRP) (ALPHA-2-MACROGLOBULIN RECEPTOR) (A2MR).
   8.7e-32:202:37
   GALLUS GALLUS (CHICKEN).
   P98157
HRIFA003055a
   PROLINE-RICH PROTEIN MP-2 PRECURSOR.
   3.4e-08:175:29
   MUS MUSCULUS (MOUSE).
   P05142
HRIFA003063a
   B-CELL LYMPHOMA 6 PROTEIN HOMOLOG.
   2.8e-15:123:34
   MUS MUSCULUS (MOUSE).
   P41183
HRIFA003093a
   PROLINE-RICH PROTEIN MP-2 PRECURSOR.
   1.3e-11:142:37
   MUS MUSCULUS (MOUSE).
   P05142
HRIFA003340a
   TRANS-ACTING TRANSCRIPTIONAL PROTEIN ICP0 (IMMEDIATE-EARLY PROTEIN IE110) (VMW110) (ALPHA-0 PROTEIN).
   2.3e-05:200:31
   HERPES SIMPLEX VIRUS (TYPE 1 / STRAIN 17).
   P08393
HRIFA003357a
   GLUCOSE REPRESSION MEDIATOR PROTEIN.
   0.0023:190:26
   SACCHAROMYCES CEREVISIAE (BAKER'S YEAST).
   P14922
HRIFA003402a
   COLLAGEN ALPHA 1(II) CHAIN (FRAGMENTS).
   3.6e-05:194:27
   BOS TAURUS (BOVINE).
   P02459
HRIFA003504a
   CADHERIN-RELATED TUMOR SUPPRESSOR PRECURSOR (FAT PROTEIN).
   1.4e-08:150:33
   DROSOPHILA MELANOGASTER (FRUIT FLY).
   P33450
HRIFA003592a
   CD9 ANTIGEN.
   0.0053:24:70
   BOS TAURUS (BOVINE).
   P30932
HRIFA003635a
   "MANNOSYL-OLIGOSACCHARIDE ALPHA-1,2-MANNOSIDASE ISOFORM 1 (EC 3.2.1.113) (MAN(9)-ALPHA-MANNOSIDASE)."
   5.3e-45:239:43
   DROSOPHILA MELANOGASTER (FRUIT FLY).
   P53624
HRIFA003640a
   PROCYCLIC FORM SPECIFIC POLYPEPTIDE A-BETA PRECURSOR (PROCYCLIN) (PARP A-BETA).
   0.00018:28:64
   TRYPANOSOMA BRUCEI BRUCEI.
   P09791
HRIFA003883a
   TRANSCRIPTIONAL REPRESSOR PROTEIN YY1 (YIN AND YANG 1) (YY-1) (DELTA TRANSCRIPTION FACTOR) (NF-E1) (UCR-MOTIF DNA-BINDING PROTEIN).
   1.0:57:35
   MUS MUSCULUS (MOUSE).
   Q00899
HRIFA003892a
   MULTIDRUG RESISTANCE PROTEIN 2 (MULTIDRUG-EFFLUX TRANSPORTER 2).
   6.5e-08:144:25
   BACILLUS SUBTILIS.
   P39843
HRIFA003946a
   PROLINE-RICH PROTEIN MP-2 PRECURSOR.
   1.4e-06:85:37
   MUS MUSCULUS (MOUSE).
   P05142
HRIFA004006a
   ZINC FINGER PROTEIN 140.
   6.2e-20:83:66
   HOMO SAPIENS (HUMAN).
   P52738
HRIFA004034a
   B-CELL LYMPHOMA 3-ENCODED PROTEIN (BCL-3 PROTEIN).
   1.4e-15:192:32
   HOMO SAPIENS (HUMAN).
   P20749
HRIFA004112a
   CADHERIN-RELATED TUMOR SUPPRESSOR PRECURSOR (FAT PROTEIN).
   7.2e-26:193:37
   DROSOPHILA MELANOGASTER (FRUIT FLY).
   P33450
HRIFA004162a
   ERYTHROCYTE BAND 7 INTEGRAL MEMBRANE PROTEIN (STOMATIN) (PROTEIN 7.2B).
   3.6e-10:117:29
   MUS MUSCULUS (MOUSE).
   P54116
HRIFA004401a
   LACTOSE OPERON REPRESSOR.
   1.1e-07:36:86
   ESCHERICHIA COLI.
   P03023
HRIFA004426a
   ATRIAL GLAND-SPECIFIC ANTIGEN PRECURSOR (AGSA).
   5.1e-11:85:41
   APLYSIA CALIFORNICA (CALIFORNIA SEA HARE).
   P15287
HRIFA004490a
   BETA-GALACTOSIDASE PRECURSOR (EC 3.2.1.23) (LACTASE) (ACID BETA-GALACTOSIDASE).
   5.3e-19:101:44
   MUS MUSCULUS (MOUSE).
   P23780
HRIFA004523a
   GLYCOSYLTRANSFERASE ALG2 (EC 2.4.1.-).
   2.6e-36:180:43
   SACCHAROMYCES CEREVISIAE (BAKER'S YEAST).
   P43636
HRIFA004663a
   T-CELL-SPECIFIC TRANSCRIPTION FACTOR 1 (TCF-1) (T-CELL FACTOR 1) (TRANSCRIPTION FACTOR-7).
   1.2e-40:112:75
   MUS MUSCULUS (MOUSE).
   Q00417
HRIFA004696a
   PROTEIN TRANSPORT PROTEIN SEC61 ALPHA SUBUNIT.
   1.1e-62:145:84
   CANIS FAMILIARIS (DOG).
   P38377
HRIFA004714a
   HYPOTHETICAL 36.7 KD PROTEIN AH6.2 IN CHROMOSOME II.
   2.3e-50:127:54
   CAENORHABDITIS ELEGANS.
   Q09201
HRIFA004745a
   MITOCHONDRIAL RNA SPLICING PROTEIN MSR4.
   5.0e-17:107:43
   SACCHAROMYCES CEREVISIAE (BAKER'S YEAST).
   P23500
HRIFA004780a
   EXTENSIN PRECURSOR (PROLINE-RICH GLYCOPROTEIN).
   7.2e-07:142:30
   ZEA MAYS (MAIZE).
   P14918
HRIFA004919a
   GLYCINE-RICH CELL WALL STRUCTURAL PROTEIN 1.8 PRECURSOR (GRP 1.8).
   1.5e-25:156:46
   PHASEOLUS VULGARIS (KIDNEY BEAN) (FRENCH BEAN).
   P10496
HRIFA005072a
   GLYCINE-RICH CELL WALL STRUCTURAL PROTEIN (CLONE W10-1) (FRAGMENT).
   8.3e-05:24:62
   LYCOPERSICON ESCULENTUM (TOMATO).
   Q01157
HRIFA005102a
   A-AGGLUTININ ATTACHMENT SUBUNIT PRECURSOR.
   2.5e-07:188:31
   SACCHAROMYCES CEREVISIAE (BAKER'S YEAST).
   P32323
HRIFA005184a
   CYTOCHROME B5.
   3.4e-11:117:29
   SACCHAROMYCES CEREVISIAE (BAKER'S YEAST).
   P40312
HRIFA005214a
   TRANS-ACTING TRANSCRIPTIONAL PROTEIN ICP0 (VMW118 PROTEIN).
   5.9e-05:141:33
   HERPES SIMPLEX VIRUS (TYPE 2 / STRAIN HG52).
   P28284
HRIFA005231a
   ORM1 PROTEIN.
   1.7e-18:137:35
   SACCHAROMYCES CEREVISIAE (BAKER'S YEAST).
   P53224
HRIFA005240a
   ZINC FINGER PROTEIN 85 (ZINC FINGER PROTEIN HPF4) (HTF1).
   6.3e-81:194:70
   HOMO SAPIENS (HUMAN).
   Q03923
HRIFA005255a
   HYPOTHETICAL 57.1 KD PROTEIN IN MAP2-TEL1 INTERGENIC REGION.
   1.5e-07:202:24
   SACCHAROMYCES CEREVISIAE (BAKER'S YEAST).
   P38176
HRIFA005271a
   MITOCHONDRIAL PHOSPHATE CARRIER PROTEIN PRECURSOR.
   1.2e-55:86:81
   HOMO SAPIENS (HUMAN).
   Q00325
HRIFA005296a
   INSULIN PROMOTER FACTOR 1 (IPF-1) (ISLET/DUODENUM HOMEOBOX-1) (IDX-1) (SOMATOSTATIN TRANSACTIVATING FACTOR-1) (STF-1) (PANCREAS/DUODENUM HOMEOBOX-1) (GLUCOSE SENSITIVE FACTOR) (GSF).
   0.82:90:34
   HOMO SAPIENS (HUMAN).
   P52945
HRIFA005300a
   SKIN SECRETORY PROTEIN XP2 PRECURSOR (APEG PROTEIN).
   1.6e-07:178:30
   XENOPUS LAEVIS (AFRICAN CLAWED FROG).
   P17437
HRIFA005369a
   EBNA-1 NUCLEAR PROTEIN.
   2.3e-07:101:39
   EPSTEIN-BARR VIRUS (STRAIN B95-8) (HUMAN HERPESVIRUS 4).
   P03211
HRIFA005372a
   CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).
   1.1e-14:97:38
   PETROMYZON MARINUS (SEA LAMPREY).
   P25210
HRIFA005392a
   SYNDECAN-2 PRECURSOR (FIBROGLYCAN) (HEPARAN SULFATE PROTEOGLYCAN CORE PROTEIN) (HSPG) (SYND2).
   1.3e-50:126:84
   HOMO SAPIENS (HUMAN).
   P34741
HRIFA005409a
   HIGH-AFFINITY CATIONIC AMINO ACID TRANSPORTER-1 (CAT-1) (CAT1) (SYSTEM Y+ BASIC AMINO ACID TRANSPORTER) (ECOTROPIC RETROVIRAL LEUKEMIA RECEPTOR HOMOLOG) (ERR) (ECOTROPIC RETROVIRUS RECEPTOR HOMOLOG).
   7.1e-66:197:64
   HOMO SAPIENS (HUMAN).
   P30825
HRIFA005420a
   INTERFERON-RELATED PROTEIN PC4 (TPA INDUCED SEQUENCE 7) (TIS7 PROTEIN).
   1.5e-33:221:41
   MUS MUSCULUS (MOUSE).
   P19182
HRIFA005438a
   SUCCINATE DEHYDROGENASE [UBIQUINONE] FLAVOPROTEIN SUBUNIT PRECURSOR (EC 1.3.5.1) (FP) (FLAVOPROTEIN SUBUNIT OF COMPLEX II).
   6.4e-71:175:68
   HOMO SAPIENS (HUMAN).
   P31040
HRIFA005462a
   CARBONIC ANHYDRASE VI (EC 4.2.1.1) (SALIVARY) (CARBONATE DEHYDRATASE VI).
   1.4e-19:137:37
   OVIS ARIES (SHEEP).
   P08060
HRIFA005500a
   EBNA-1 NUCLEAR PROTEIN.
   0.00042:54:50
   EPSTEIN-BARR VIRUS (STRAIN B95-8) (HUMAN HERPESVIRUS 4).
   P03211
HRIFA005540a
   CYCLIC-AMP-DEPENDENT TRANSCRIPTION FACTOR ATF-6 (FRAGMENT).
   0.12:47:29
   HOMO SAPIENS (HUMAN).
   P18850
HRIFA005644a
   VACUOLAR ATP SYNTHASE SUBUNIT AC45 PRECURSOR (EC 3.6.1.34) (V-ATPASE AC45 SUBUNIT).
   1.2e-102:233:87
   BOS TAURUS (BOVINE).
   P40682
HRIFA005702a
   CELL SURFACE GLYCOPROTEIN MUC18 PRECURSOR (MELANOMA-ASSOCIATED ANTIGEN MUC18) (MELANOMA-ASSOCIATED ANTIGEN A32) (S-ENDO 1 ENDOTHELIAL- ASSOCIATED ANTIGEN) (CD146 ANTIGEN) (MELANOMA ADHESION MOLECULE).
   8.7e-05:174:28
   HOMO SAPIENS (HUMAN).
   P43121
HRIFA005720a
   F-SPONDIN PRECURSOR.
   8.9e-12:155:31
   XENOPUS LAEVIS (AFRICAN CLAWED FROG).
   P35447
HRIFA005728a
   SPORULATION-SPECIFIC PROTEIN 1 (EC 2.7.1.-).
   1.7e-05:126:29
   SACCHAROMYCES CEREVISIAE (BAKER'S YEAST).
   P08458
HRIFA005732a
   PUTATIVE SERINE/THREONINE-PROTEIN KINASE PKWA (EC 2.7.1.-).
   4.4e-26:159:38
   THERMOMONOSPORA CURVATA.
   P49695
HRIFA005760a
   FUCOSYLGLYCOPROTEIN ALPHA-N-ACETYLGALACTOSAMINYLTRANSFERASE (EC 2.4.1.40) (HISTO-BLOOD GROUP A TRANSFERASE) (A TRANSFERASE) / FUCOSYLGLYCOPROTEIN 3-ALPHA-GALACTOSYLTRANSFERASE (EC 2.4.1.37) (HISTO-BLOOD GROUP B TRANSFERASE) (B TRANSFERASE) (NAGAT).
   3.8e-15:53:54
   HOMO SAPIENS (HUMAN).
   P16442
HRIFA005781a
   ESTRADIOL 17 BETA-DEHYDROGENASE 3 (EC 1.1.1.62) (17-BETA-HSD 3) (TESTICULAR 17-BETA-HYDROXYSTEROID DEHYDROGENASE).
   5.2e-47:228:47
   HOMO SAPIENS (HUMAN).
   P37058
HRIFA005944a
   PROCOLLAGEN ALPHA 1(II) CHAIN PRECURSOR [CONTAINS: CHONDROCALCIN].
   2.5e-06:142:35
   MUS MUSCULUS (MOUSE).
   P28481
HRIFA006183a
   ZINC FINGER PROTEIN 136.
   1.3e-42:129:62
   HOMO SAPIENS (HUMAN).
   P52737
HRIFA006250a
   HOMEOTIC GENE REGULATOR (BRAHMA PROTEIN).
   0.0038:75:37
   DROSOPHILA MELANOGASTER (FRUIT FLY).
   P25439
HRIFA006298a
   EBNA-1 NUCLEAR PROTEIN.
   1.4e-05:80:42
   EPSTEIN-BARR VIRUS (STRAIN B95-8) (HUMAN HERPESVIRUS 4).
   P03211
HRIFA006448a
   SKIN SECRETORY PROTEIN XP2 PRECURSOR (APEG PROTEIN).
   8.5e-05:183:28
   XENOPUS LAEVIS (AFRICAN CLAWED FROG).
   P17437
HRIFA006494a
   AXONIN-1 PRECURSOR (AXONAL GLYCOPROTEIN TAG-1) (TRANSIENT AXONAL GLYCOPROTEIN 1).
   1.2e-18:201:33
   HOMO SAPIENS (HUMAN).
   Q02246
HRIFA006510a
   CORNICHON PROTEIN.
   6.0e-53:144:66
   DROSOPHILA MELANOGASTER (FRUIT FLY).
   P49858
HRIFA006566a
   CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).
   6.6e-15:97:38
   PETROMYZON MARINUS (SEA LAMPREY).
   P25210
HRIFA006572a
   PROCOLLAGEN ALPHA 1(I) CHAIN PRECURSOR.
   7.2e-05:158:29
   MUS MUSCULUS (MOUSE).
   P11087
HRIFA006586a
   HYPOTHETICAL 53.3 KD PROTEIN IN HXT8-CAN1 INTERGENIC REGION.
   1.3e-13:219:26
   SACCHAROMYCES CEREVISIAE (BAKER'S YEAST).
   P39981
HRIFA006596a
   POTENTIAL CAAX PRENYL PROTEASE 1 (EC 3.4.24.-) (PRENYL PROTEIN- SPECIFIC ENDOPROTEASE 1) (PPSEP 1).
   7.2e-22:241:32
   SCHIZOSACCHAROMYCES POMBE (FISSION YEAST).
   Q10071
HRIFA006609a
   PANCREATIC HORMONE PRECURSOR (PANCREATIC POLYPEPTIDE) (PP).
   0.61:28:46
   "GALLUS GALLUS (CHICKEN), AND MELEAGRIS GALLOPAVO (COMMON TURKEY)."
   P01306
HRIFA006633a
   COLLAGEN ALPHA 1(XVI) CHAIN PRECURSOR.
   7.8e-07:170:34
   HOMO SAPIENS (HUMAN).
   Q07092
HRIFA006642a
   AMALGAM PROTEIN PRECURSOR.
   1.5e-09:185:28
   DROSOPHILA MELANOGASTER (FRUIT FLY).
   P15364
HRIFA006649a
   ZINC FINGER PROTEIN 85 (ZINC FINGER PROTEIN HPF4) (HTF1).
   1.7e-50:166:50
   HOMO SAPIENS (HUMAN).
   Q03923
HRIFA006667a
   ZINC FINGER PROTEIN 85 (ZINC FINGER PROTEIN HPF4) (HTF1).
   6.8e-45:180:43
   HOMO SAPIENS (HUMAN).
   Q03923
HRIFA006730a
   SYG1 PROTEIN.
   1.8e-14:164:35
   SACCHAROMYCES CEREVISIAE (BAKER'S YEAST).
   P40528
HRIFA006798a
   SKIN SECRETORY PROTEIN XP2 PRECURSOR (APEG PROTEIN).
   0.22:149:34
   XENOPUS LAEVIS (AFRICAN CLAWED FROG).
   P17437
HRIFA006926a
   SYNAPTOTAGMIN IV.
   3.6e-19:168:38
   RATTUS NORVEGICUS (RAT).
   P50232
HRIFA007013a
   MIC1 PROTEIN.
   1.4e-13:115:38
   SACCHAROMYCES CEREVISIAE (BAKER'S YEAST).
   P53258
HRIFA007032a
   CCAAT DISPLACEMENT PROTEIN (HOMEOBOX PROTEIN CLOX) (CLOX-1) (FRAGMENT).
   0.00013:92:35
   CANIS FAMILIARIS (DOG).
   P39881
HRIFA007068a
   EBNA-1 NUCLEAR PROTEIN.
   7.0e-10:145:33
   EPSTEIN-BARR VIRUS (STRAIN B95-8) (HUMAN HERPESVIRUS 4).
   P03211
HRIFA007152a
   TRANSCRIPTION FACTOR SOX-4.
   0.90:47:44
   HOMO SAPIENS (HUMAN).
   Q06945
HRIFA007219a
   THROMBOSPONDIN 3 PRECURSOR.
   1.3e-105:209:88
   HOMO SAPIENS (HUMAN).
   P49746
HRIFA007228a
   HYPOTHETICAL 53.3 KD PROTEIN IN HXT8-CAN1 INTERGENIC REGION.
   2.3e-11:174:24
   SACCHAROMYCES CEREVISIAE (BAKER'S YEAST).
   P39981
HRIFA007243a
   PROBABLE CALCIUM-TRANSPORTING ATPASE 6 (EC 3.6.1.38).
   3.0e-18:163:36
   SACCHAROMYCES CEREVISIAE (BAKER'S YEAST).
   P39986
HRIFA007244a
   EXTENSIN PRECURSOR (CELL WALL HYDROXYPROLINE-RICH GLYCOPROTEIN).
   4.2e-05:81:33
   NICOTIANA TABACUM (COMMON TOBACCO).
   P13983
HRIFA007256a
   DEATH-ASSOCIATED PROTEIN KINASE 1 (EC 2.7.1.-) (DAP KINASE 1).
   2.3e-77:186:75
   HOMO SAPIENS (HUMAN).
   P53355
HRIFA007262a
   PAIRED AMPHIPATHIC HELIX PROTEIN.
   1.3e-06:152:26
   SACCHAROMYCES CEREVISIAE (BAKER'S YEAST).
   P22579
HRIFA007352a
   5'-TG-3'INTERACTING FACTOR (HOMEOBOX PROTEIN TGIF).
   4.2e-36:146:57
   HOMO SAPIENS (HUMAN).
   Q15583
HRIFA007424a
   F-SPONDIN PRECURSOR.
   8.9e-34:84:89
   RATTUS NORVEGICUS (RAT).
   P35446
HRIFA007435a
   PROTEIN KINASE CEK1 (EC 2.7.1.-).
   1.0e-37:159:53
   SCHIZOSACCHAROMYCES POMBE (FISSION YEAST).
   P38938
HRIFA007463a
   HST1 PROTEIN (HOMOLOGOUS TO SIR2 PROTEIN 1).
   4.8e-32:85:48
   SACCHAROMYCES CEREVISIAE (BAKER'S YEAST).
   P53685
HRIFA007493a
   UBIQUITIN-CONJUGATING ENZYME E2-28.4 KD (EC 6.3.2.19) (UBIQUITIN- PROTEIN LIGASE) (UBIQUITIN CARRIER PROTEIN).
   1.2e-47:171:56
   SACCHAROMYCES CEREVISIAE (BAKER'S YEAST).
   P33296
HRIFA007512a
   EXTENSIN PRECURSOR (CELL WALL HYDROXYPROLINE-RICH GLYCOPROTEIN).
   8.0e-07:173:28
   NICOTIANA TABACUM (COMMON TOBACCO).
   P13983
HRIFA007532a
   "CALPAIN P94, LARGE [CATALYTIC] SUBUNIT (EC 3.4.22.17) (CALCIUM-ACTIVATED NEUTRAL PROTEINASE) (CANP) (P94 PROTEIN) (MUSCLE-SPECIFIC CALCIUM-ACTIVATED NEUTRAL PROTEASE 3 LARGE SUBUNIT)."
   1.8e-10:110:37
   HOMO SAPIENS (HUMAN).
   P20807
HRIFA007547a
   TRANS-ACTING TRANSCRIPTIONAL PROTEIN ICP0 (P135 PROTEIN) (IER 2.9/ER2.6).
   0.068:51:45
   BOVINE HERPESVIRUS TYPE 1 (STRAIN K22).
   P29836
HRIFA007565a
   COLLAGEN ALPHA 1(X) CHAIN PRECURSOR.
   5.1e-08:121:37
   HOMO SAPIENS (HUMAN).
   Q03692
HRIFA007571a
   ORM1 PROTEIN.
   5.8e-17:106:36
   SACCHAROMYCES CEREVISIAE (BAKER'S YEAST).
   P53224
HRIFA007659a
   HYPOTHETICAL 51.5 KD PROTEIN D2024.3 IN CHROMOSOME IV.
   2.5e-47:213:41
   CAENORHABDITIS ELEGANS.
   P49191
HRIFA007722a
   HYPOTHETICAL 24.5 KD PROTEIN IN SAP185-BCK1 INTERGENIC REGION.
   7.7e-13:146:32
   SACCHAROMYCES CEREVISIAE (BAKER'S YEAST).
   P40857
HRIFA007728a
   EXTENSIN PRECURSOR (CELL WALL HYDROXYPROLINE-RICH GLYCOPROTEIN).
   9.1e-05:124:31
   NICOTIANA TABACUM (COMMON TOBACCO).
   P13983
HRIFA007745a
   ACETYLCHOLINESTERASE PRECURSOR (EC 3.1.1.7) (ACHE).
   7.0e-15:109:36
   TORPEDO CALIFORNICA (PACIFIC ELECTRIC RAY).
   P04058
HRIFA007829a
   GLYCINE-RICH CELL WALL STRUCTURAL PROTEIN (CLONE W10-1) (FRAGMENT).
   0.00045:16:68
   LYCOPERSICON ESCULENTUM (TOMATO).
   Q01157
HRIFA007909a
   COLLAGEN ALPHA 1(I) CHAIN (FRAGMENTS).
   6.1e-06:173:34
   BOS TAURUS (BOVINE).
   P02453
HRIFA007985a
   T-CELL RECEPTOR BETA CHAIN PRECURSOR (ANA 11).
   0.00079:97:37
   ORYCTOLAGUS CUNICULUS (RABBIT).
   P06333
HRIFA008000a
   "DIHYDROPYRIDINE-SENSITIVE L-TYPE, CALCIUM CHANNEL ALPHA-2/DELTA SUBUNITS PRECURSOR."
   1.6e-37:165:42
   ORYCTOLAGUS CUNICULUS (RABBIT).
   P13806
HRIFA008174a
   COLLAGEN 1(X) CHAIN PRECURSOR.
   4.5e-05:215:28
   BOS TAURUS (BOVINE).
   P23206
HRIFA008186a
   ESTRADIOL 17 BETA-DEHYDROGENASE 3 (EC 1.1.1.62) (17-BETA-HSD 3) (TESTICULAR 17-BETA-HYDROXYSTEROID DEHYDROGENASE).
   2.1e-25:118:46
   HOMO SAPIENS (HUMAN).
   P37058
HRIFA008200a
   ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
   7.9e-17:139:36
   SACCHAROMYCES CEREVISIAE (BAKER'S YEAST).
   P32802
HRIFA008212a
   A-AGGLUTININ ATTACHMENT SUBUNIT PRECURSOR.
   0.035:135:28
   SACCHAROMYCES CEREVISIAE (BAKER'S YEAST).
   P32323
HRIFA008252a
   PROLINE-RICH PROTEIN MP-2 PRECURSOR.
   0.00015:128:32
   MUS MUSCULUS (MOUSE).
   P05142
HRIFA008284a
   NEURAL CELL ADHESION MOLECULE L1 PRECURSOR (N-CAM L1).
   3.9e-18:153:30
   HOMO SAPIENS (HUMAN).
   P32004
HRIFA008314a
   HYPOTHETICAL 149.7 KD PROTEIN IN IRE1-KSP1 INTERGENIC REGION.
   2.1e-18:99:47
   SACCHAROMYCES CEREVISIAE (BAKER'S YEAST).
   P38800
HRIFA008362a
   PHOSPHATIDYLCHOLINE-STEROL ACYLTRANSFERASE PRECURSOR (EC 2.3.1.43) (LECITHIN-CHOLESTEROL ACYLTRANSFERASE) (PHOSPHOLIPID-CHOLESTEROL ACYLTRANSFERASE) (FRAGMENT).
   9.1e-42:135:57
   GALLUS GALLUS (CHICKEN).
   P53760
HRIFA008426a
   HOMEODOMAIN PROTEIN AKR (AVIAN KNOTTED-RELATED PROTEIN).
   1.3e-08:104:45
   GALLUS GALLUS (CHICKEN).
   Q90655
HRIFA008459a
   CARBON CATABOLITE DEREPRESSING PROTEIN KINASE (EC 2.7.1.-).
   5.5e-15:96:40
   SACCHAROMYCES CEREVISIAE (BAKER'S YEAST).
   P06782
HRIFA008483a
   PROBABLE LONG-CHAIN FATTY ACID TRANSPORT PROTEIN.
   7.4e-26:154:41
   SACCHAROMYCES CEREVISIAE (BAKER'S YEAST).
   P38225
HRIFA008547a
   ZINC FINGER PROTEIN 136.
   7.2e-57:228:50
   HOMO SAPIENS (HUMAN).
   P52737
HRIFA008596a
   SKIN SECRETORY PROTEIN XP2 PRECURSOR (APEG PROTEIN).
   1.6e-05:97:35
   XENOPUS LAEVIS (AFRICAN CLAWED FROG).
   P17437
HRIFA008611a
   NPL1 PROTEIN (SEC63 PROTEIN).
   8.1e-15:113:38
   SACCHAROMYCES CEREVISIAE (BAKER'S YEAST).
   P14906
HRIFA008661a
   GALACTOSE-PROTON SYMPORT (GALACTOSE TRANSPORTER).
   2.7e-16:184:29
   ESCHERICHIA COLI.
   P37021
HRIFA008717a
   SERINE/THREONINE-PROTEIN KINASE NRK1 (EC 2.7.1.-) (N-RICH KINASE 1).
   6.9e-32:198:41
   SACCHAROMYCES CEREVISIAE (BAKER'S YEAST).
   P38692
HRIFA008784a
   HYPOTHETICAL 26.3 KD PROTEIN IN OYE2-GND1 INTERGENIC REGION.
   2.2e-16:93:47
   SACCHAROMYCES CEREVISIAE (BAKER'S YEAST).
   P38869
HRIFA008790a
   HYPOTHETICAL 15.7 KD PROTEIN IN NUP85-SSC1 INTERGENIC REGION.
   4.2e-08:121:32
   SACCHAROMYCES CEREVISIAE (BAKER'S YEAST).
   P47111
HRIFA008976a
   ACROSIN PRECURSOR (EC 3.4.21.10).
   0.31:20:70
   HOMO SAPIENS (HUMAN).
   P10323
HRIFA008981a
   ZINC FINGER PROTEIN 85 (ZINC FINGER PROTEIN HPF4) (HTF1).
   1.0e-84:126:74
   HOMO SAPIENS (HUMAN).
   Q03923
HRIFA008989a
   TRANS-ACTING TRANSCRIPTIONAL PROTEIN ICP0 (IMMEDIATE-EARLY PROTEIN IE110) (VMW110) (ALPHA-0 PROTEIN).
   1.2e-05:134:33
   HERPES SIMPLEX VIRUS (TYPE 1 / STRAIN 17).
   P08393
HRIFA009071 a
   CELLULAR TUMOR ANTIGEN P53 (PHOSPHOPROTEIN P53).
   0.14:104:31
   HOMO SAPIENS (HUMAN).
   P04637
HRIFA009101a
   ZINC FINGER PROTEIN 136.
   6.5e-47:126:67
   HOMO SAPIENS (HUMAN).
   P52737
HRIFA009123a
   SKIN SECRETORY PROTEIN XP2 PRECURSOR (APEG PROTEIN).
   0.010:127:35
   XENOPUS LAEVIS (AFRICAN CLAWED FROG).
   P17437
HRIFA009136a
   REGULATORY PROTEIN E2.
   0.032:100:37
   HUMAN PAPILLOMAVIRUS TYPE 25.
   P36787
HRIFA009171a
   BUTYROPHILIN PRECURSOR (BT).
   1.6e-15:168:31
   BOS TAURUS (BOVINE).
   P18892
HRIFA009220a
   HYPOTHETICAL 43.7 KD PROTEIN C24B11.08C IN CHROMOSOME I.
   2.2e-48:268:41
   SCHIZOSACCHAROMYCES POMBE (FISSION YEAST).
   Q09895
HRIFA009339a
   PROCOLLAGEN ALPHA 2(I) CHAIN PRECURSOR.
   0.63:57:35
   MUS MUSCULUS (MOUSE).
   Q01149
HRIFA009451a
   METALLOPROTEINASE INHIBITOR 1 PRECURSOR (TIMP-1) (ERYTHROID POTENTIATING ACTIVITY) (EPA) (TISSUE INHIBITOR OF METALLOPROTEINASES) (FIBROBLAST COLLAGENASE INHIBITOR) (COLLAGENASE INHIBITOR).
   1.7e-57:163:73
   HOMO SAPIENS (HUMAN).
   P01033
HRIFA009482a
   BETA-GALACTOSIDASE PRECURSOR (EC 3.2.1.23) (LACTASE) (ACID BETA-GALACTOSIDASE).
   7.7e-25:86:59
   MUS MUSCULUS (MOUSE).
   P23780
HRIFA009578a
   HYPOTHETICAL 24.5 KD PROTEIN IN SAP185-BCK1 INTERGENIC REGION.
   8.8e-10:199:26
   SACCHAROMYCES CEREVISIAE (BAKER'S YEAST).
   P40857
HRIFA009762a
   CCAAT DISPLACEMENT PROTEIN (CDP) (CDP2) (FRAGMENT).
   0.17:116:32
   RATTUS NORVEGICUS (RAT).
   P53565
HRIFA009783a
   HYPOTHETICAL 85.7 KD PROTEIN C13G6.03 IN CHROMOSOME I.
   6.2e-48:231:48
   SCHIZOSACCHAROMYCES POMBE (FISSION YEAST).
   Q09782
HRIFA009825a
   ANTER-SPECIFIC PROLINE-RICH PROTEIN APG PRECURSOR.
   4.0e-06:70:38
   ARABIDOPSIS THALIANA (MOUSE-EAR CRESS).
   P40602
HRIFA009852a
   "NEURAL CELL ADHESION MOLECULE 1, LARGE ISOFORM PRECURSOR (N-CAM 180) [CONTAINS: N-CAM 140]."
   4.0e-07:198:27
   XENOPUS LAEVIS (AFRICAN CLAWED FROG).
   P16170
HRIFA009881a
   EXTENSIN PRECURSOR (PROLINE-RICH GLYCOPROTEIN).
   1.5e-11:106:35
   SORGHUM VULGARE (SORGHUM).
   P24152
HRIFA009983a
   G-BOX BINDING FACTOR (GBF).
   3.8e-10:156:30
   DICTYOSTELIUM DISCOIDEUM (SLIME MOLD).
   P36417
HRIFA010005a
   "M PROTEIN, SEROTYPE 49 PRECURSOR."
   1.6e-05:183:27
   STREPTOCOCCUS PYOGENES.
   P16947
HRIFA010078a
   HYPOTHETICAL 57.5 KD PROTEIN IN VMA7-RPS25A INTERGENIC REGION.
   4.7e-05:194:31
   SACCHAROMYCES CEREVISIAE (BAKER'S YEAST).
   P53214
HRIFA010085a
   ZINC FINGER PROTEIN 85 (ZINC FINGER PROTEIN HPF4) (HTF1).
   2.9e-92:243:69
   HOMO SAPIENS (HUMAN).
   Q03923
HRIFA010090a
   N-ACETYLGLUCOSAMINE-6-SULFATASE PRECURSOR (EC 3.1.6.14) (G6S) (GLUCOSAMINE-6-SULFATASE).
   6.7e-16:78:51
   HOMO SAPIENS (HUMAN).
   P15586
HRIFA010130a
   DOLICHYL-PHOSPHATE-MANNOSE--PROTEIN MANNOSYLTRANSFERASE 4 (EC 2.4.1.109).
   5.6e-13:99:34
   SACCHAROMYCES CEREVISIAE (BAKER'S YEAST).
   P46971
HRIFA010152a
   "ADENYLATE CYCLASE, TYPE V (EC 4.6.1.1) (ATP PYROPHOSPHATE-LYASE) (CA(2+)-INHIBITABLE ADENYLYL CYCLASE)."
   2.3e-05:73:43
   CANIS FAMILIARIS (DOG).
   P30803
HRIFA010176a
   HEPATOCYTE NUCLEAR FACTOR 3-BETA (HNF-3B).
   0.066:105:31
   MUS MUSCULUS (MOUSE).
   P35583
HRIFA010301a
   ANTER-SPECIFIC PROLINE-RICH PROTEIN APG PRECURSOR.
   1.1e-09:120:34
   ARABIDOPSIS THALIANA (MOUSE-EAR CRESS).
   P40602
HRIFA010319a
   DOPAMINE-BETA-MONOOXYGENASE PRECURSOR (EC 1.14.17.1) (DOPAMINE BETA- HYDROXYLASE) (DBH).
   4.8e-23:185:32
   RATTUS NORVEGICUS (RAT).
   Q05754
HRIFA010361a
   SKIN SECRETORY PROTEIN XP2 PRECURSOR (APEG PROTEIN).
   2.6e-08:136:32
   XENOPUS LAEVIS (AFRICAN CLAWED FROG).
   P17437
HRIFA010394a
   HYPOTHETICAL 51.5 KD PROTEIN D2024.3 IN CHROMOSOME IV.
   3.3e-36:144:47
   CAENORHABDITIS ELEGANS.
   P49191
HRIFA010425a
   A-AGGLUTININ ATTACHMENT SUBUNIT PRECURSOR.
   1.9e-09:199:29
   SACCHAROMYCES CEREVISIAE (BAKER'S YEAST).
   P32323
HRIFA010460a
   TRANSCRIPTIONAL ACTIVATOR FE65.
   2.3e-27:101:54
   RATTUS NORVEGICUS (RAT).
   P46933
HRIFA010466a
   SKIN SECRETORY PROTEIN XP2 PRECURSOR (APEG PROTEIN).
   5.3e-07:123:34
   XENOPUS LAEVIS (AFRICAN CLAWED FROG).
   P17437
HRIFA010490a
   TRANS-ACTING TRANSCRIPTIONAL PROTEIN ICP0 (EARLY PROTEIN 0) (EP0).
   0.0031:118:30
   PSEUDORABIES VIRUS (STRAIN INDIANA-FUNKHAUSER / BECKER) (PRV).
   P29129
HRIFA010736a
   PROTEIN Q300.
   0.018:14:85
   MUS MUSCULUS (MOUSE).
   Q02722
HRIFA010790a
   RENAL SODIUM-DEPENDENT PHOSPHATE TRANSPORT PROTEIN 2 (SODIUM/PHOSPHATE COTRANSPORTER 2) (NA(+)/PI COTRANSPORTER 2) (RENAL SODIUM-PHOSPHATE TRANSPORT PROTEIN 2) (RENAL NA+-DEPENDENT PHOSPHATE COTRANSPORTER 2).
   1.6e-82:197:72
   HOMO SAPIENS (HUMAN).
   Q06495
HRIFA010799a
   PROCOLLAGEN ALPHA 1(I) CHAIN PRECURSOR.
   1.7e-05:220:30
   GALLUS GALLUS (CHICKEN).
   P02457
HRIFA010859a
   ALPHA-2C-1 ADRENERGIC RECEPTOR (ALPHA-2C-1 ADRENOCEPTOR) (SUBTYPE C4).
   0.063:134:33
   HOMO SAPIENS (HUMAN).
   P18825
HRIFA010891a
   HYPOTHETICAL 22.7 KD PROTEIN IN PAS1-MST1 INTERGENIC REGION.
   0.044:28:64
   SACCHAROMYCES CEREVISIAE (BAKER'S YEAST).
   P36015
HRIFA010975a
   TYROSINE-PROTEIN KINASE SYK (EC 2.7.1.112) (SPLEEN TYROSINE KINASE).
   8.5e-113:144:86
   HOMO SAPIENS (HUMAN).
   P43405
HRIFA010988a
   GASTRIN PRECURSOR.
   0.084:59:37
   HOMO SAPIENS (HUMAN).
   P01350
HRIFA011016a
   PROTEIN DISULFIDE ISOMERASE-RELATED PROTEIN PRECURSOR (ERP72) (CALCIUM-BINDING PROTEIN 2) (CABP2).
   3.1e-15:127:37
   RATTUS NORVEGICUS (RAT).
   P38659
HRIFA011105a
   SALIVARY GLUE PROTEIN SGS-7 PRECURSOR.
   0.97:41:43
   DROSOPHILA MELANOGASTER (FRUIT FLY).
   P02841
HRIFA011128a
   GLYCINE-RICH CELL WALL STRUCTURAL PROTEIN (CLONE W10-1) (FRAGMENT).
   0.0046:30:63
   LYCOPERSICON ESCULENTUM (TOMATO).
   Q01157
HRIFA011179a
   PROBABLE SERINE/THREONINE-PROTEIN KINASE YKL101W (EC 2.7.1.-).
   1.1e-20:127:42
   SACCHAROMYCES CEREVISIAE (BAKER'S YEAST).
   P34244
HRIFA011197a
   DIPEPTIDYL PEPTIDASE IV (EC 3.4.14.5) (DPP IV) (THYMOCYTE-ACTIVATING MOLECULE) (THAM).
   5.8e-26:169:40
   MUS MUSCULUS (MOUSE).
   P28843
HRIFA011449a
   GALACTOSYLTRANSFERASE ASSOCIATED PROTEIN KINASE P58/GTA (EC 2.7.1.-).
   1.9e-26:109:53
   MUS MUSCULUS (MOUSE).
   P24788
HRIFA011484a
   D(4) DOPAMINE RECEPTOR (D(2C) DOPAMINE RECEPTOR).
   0.00055:115:33
   HOMO SAPIENS (HUMAN).
   P21917
HRIFA011512a
   POSSIBLE GLOBAL TRANSCRIPTION ACTIVATOR SNF2L2 (SNF2-ALPHA).
   0.00024:139:25
   HOMO SAPIENS (HUMAN).
   P51531
HRIFA011580a
   VITELLOGENIN II PRECURSOR (MAJOR VITELLOGENIN) [CONTAINS: LIPOVITELLIN I (LVI) PHOSVITIN (PV) LIPOVITELLIN II (LVII) YGP40].
   4.0e-08:182:32
   GALLUS GALLUS (CHICKEN).
   P02845
HRIFA011659a
   VON WILLEBRAND FACTOR PRECURSOR.
   9.8e-17:210:25
   HOMO SAPIENS (HUMAN).
   P04275
HRIFA011820a
   ZINC FINGER PROTEIN 136.
   1.9e-10:42:73
   HOMO SAPIENS (HUMAN).
   P52737
HRIFA011926a
   TRANSCRIPTIONAL REGULATORY PROTEIN ALGP (ALGINATE REGULATORY PROTEIN ALGR3).
   1.0:149:22
   PSEUDOMONAS AERUGINOSA.
   P15276
HRIFA011947a
   ZINC FINGER PROTEIN 136.
   1.3e-80:180:72
   HOMO SAPIENS (HUMAN).
   P52737
HRIFA012069a
   A-AGGLUTININ ATTACHMENT SUBUNIT PRECURSOR.
   0.0027:205:28
   SACCHAROMYCES CEREVISIAE (BAKER'S YEAST).
   P32323
HRIFA012151a
   NEUROGENIC LOCUS NOTCH HOMOLOG PROTEIN 1 PRECURSOR.
   0.00028:72:37
   RATTUS NORVEGICUS (RAT).
   Q07008
HRIFA012167a
   HYPOTHETICAL SYMPORTER IN GLTS-SELC INTERGENIC REGION.
   6.4e-09:145:28
   ESCHERICHIA COLI.
   P31435
HRIFA012278a
   ZINC FINGER PROTEIN 140.
   3.1e-14:88:52
   HOMO SAPIENS (HUMAN).
   P52738
HRIFA012354a
   "SODIUM CHANNEL PROTEIN, BRAIN II ALPHA SUBUNIT."
   2.1e-05:120:32
   RATTUS NORVEGICUS (RAT).
   P04775
HRIFA012427a
   PROCOLLAGEN ALPHA 1(I) CHAIN PRECURSOR.
   6.3e-08:250:28
   MUS MUSCULUS (MOUSE).
   P11087
HRIFA012436a
   INTESTINAL MEMBRANE A4 PROTEIN (DIFFERENTIATION-DEPENDENT PROTEIN A4).
   4.7e-09:95:31
   HOMO SAPIENS (HUMAN).
   Q04941
HRIFA012515a
   SODIUM/GLUCOSE COTRANSPORTER 1 (NA(+)/GLUCOSE COTRANSPORTER 1) (HIGH AFFINITY SODIUM-GLUCOSE COTRANSPORTER).
   3.5e-06:181:27
   ORYCTOLAGUS CUNICULUS (RABBIT).
   P11170
HRIFA012584a
   MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).
   4.9e-14:136:29
   NEUROSPORA CRASSA.
   P23231
HRIFA012625a
   "HIGH AFFINITY IMMUNOGLOBULIN EPSILON RECEPTOR BETA-SUBUNIT (FCERI) (IGE FC RECEPTOR, BETA-SUBUNIT)."
   9.6e-12:103:40
   RATTUS NORVEGICUS (RAT).
   P13386
HRIFA012692a
   BLOOM'S SYNDROME PROTEIN.
   6.3e-26:203:34
   HOMO SAPIENS (HUMAN).
   P54132
HRIFA012702a
   EXTENSIN PRECURSOR (PROLINE-RICH GLYCOPROTEIN).
   1.9e-07:153:30
   ZEA MAYS (MAIZE).
   P14918
HRIFA012737a
   LEUCOCYTE ANTIGEN CD97 PRECURSOR.
   1.6e-09:170:24
   HOMO SAPIENS (HUMAN).
   P48960
HRIFA012795a
   VOLTAGE-GATED POTASSIUM CHANNEL PROTEIN KV2.1 (DRK1).
   3.0e-34:189:39
   RATTUS NORVEGICUS (RAT).
   P15387
HRIFA012885a
   HYPOTHETICAL 30.6 KD PROTEIN IN SCP160-SMC3 INTERGENIC REGION PRECURSOR.
   2.9e-21:159:40
   SACCHAROMYCES CEREVISIAE (BAKER'S YEAST).
   P47032
HRIFA012914a
   ENV POLYPROTEIN PRECURSOR (COAT POLYPROTEIN) [CONTAINS: OUTER MEMBRANE PROTEIN GP70 TRANSMEMBRANE PROTEIN P20E].
   3.4e-29:134:47
   BABOON ENDOGENOUS VIRUS (STRAIN M7).
   P10269
HRIFA012969a
   ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
   1.2e-30:228:32
   SACCHAROMYCES CEREVISIAE (BAKER'S YEAST).
   P32802
HRIFA012990a
   PROBABLE CALCIUM-TRANSPORTING ATPASE 6 (EC 3.6.1.38).
   7.4e-20:181:33
   SACCHAROMYCES CEREVISIAE (BAKER'S YEAST).
   P39986
HRIFA013092a
   OUTER MEMBRANE PROTEIN H.8 PRECURSOR.
   0.0039:51:39
   NEISSERIA GONORRHOEAE.
   P11910
HRIFA013103a
   N-ACETYLLACTOSAMINE SYNTHASE (EC 2.4.1.90) (N-ACETYLGLUCOSAMINE (BETA 1→4)GALACTOSYLTRANSFERASE) (EC 2.4.1.38) (LACTOSE SYNTHASE A PROTEIN (EC 2.4.1.22)) (GALACTOSYLTRANSFERASE) (GT).
   0.25:50:34
   MUS MUSCULUS (MOUSE).
   P15535
HRIFA013135a
   CELL SURFACE GLYCOPROTEIN 1 PRECURSOR (OUTER LAYER PROTEIN B) (SLAYER PROTEIN 1).
   1.6e-05:214:28
   CLOSTRIDIUM THERMOCELLUM.
   Q06852
HRIFA013235a
   PROCOLLAGEN ALPHA 1(III) CHAIN PRECURSOR.
   1.9e-05:113:40
   HOMO SAPIENS (HUMAN).
   P02461
HRIFA013254a
   COMPLEMENT C4 PRECURSOR [CONTAINS: C4A ANAPHYLATOXIN].
   3.8e-13:123:41
   MUS MUSCULUS (MOUSE).
   P01029
HRIFA013265a
   CATHEPSIN L PRECURSOR (EC 3.4.22.15) (MAJOR EXCRETED PROTEIN) (MEP).
   7.0e-107:225:86
   HOMO SAPIENS (HUMAN).
   P07711
HRIFA013276a
   5'-NUCLEOTIDASE PRECURSOR (EC 3.1.3.5) (ECTO-NUCLEOTIDASE) (5'-NT) (CD73 ANTIGEN).
   2.2e-117:270:85
   HOMO SAPIENS (HUMAN).
   P21589
HRIFA013279a
   CIRCUMSPOROZOITE PROTEIN PRECURSOR (CS).
   4.9e-05:127:37
   PLASMODIUM VIVAX.
   P08677
HRIFA013376a
   MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).
   8.0e-23:230:31
   NEUROSPORA CRASSA.
   P23231
HRIFA013477a
   OX-2 MEMBRANE GLYCOPROTEIN PRECURSOR (FRAGMENT).
   5.8e-87:197:87
   HOMO SAPIENS (HUMAN).
   P41217
HRIFA013586a
   ENDOZEPINE-RELATED PROTEIN PRECURSOR (MEMBRANE-ASSOCIATED DIAZEPAM BINDING INHIBITOR) (MA-DBI).
   3.8e-31:93:64
   BOS TAURUS (BOVINE).
   P07106
HRIFA013589a
   T-CELL SURFACE PROTEIN TACTILE PRECURSOR (CD96 ANTIGEN).
   5.0e-06:95:35
   HOMO SAPIENS (HUMAN).
   P40200
HRIFA013620a
   "HIGH AFFINITY IMMUNOGLOBULIN EPSILON RECEPTOR BETA-SUBUNIT (FCERI) (IGE FC RECEPTOR, BETA-SUBUNIT)."
   7.1e-08:95:37
   MUS MUSCULUS (MOUSE).
   P20490
HRIFA013726a
   SERINE/THREONINE-PROTEIN KINASE STE20 (EC 2.7.1.-).
   1.5e-33:99:50
   SACCHAROMYCES CEREVISIAE (BAKER'S YEAST).
   Q03497
HRIFA013744a
   ENDOZEPINE-RELATED PROTEIN PRECURSOR (MEMBRANE-ASSOCIATED DIAZEPAM BINDING INHIBITOR) (MA-DBI).
   7.5e-15:105:38
   BOS TAURUS (BOVINE).
   P07106
HRIFA013911a
   BIOTINIDASE PRECURSOR (EC 3.5.1.12).
   7.8e-37:104:46
   HOMO SAPIENS (HUMAN).
   P43251
HRIFA013919a
   MUCIN 2 PRECURSOR (INTESTINAL MUCIN 2).
   1.2e-10:170:32
   HOMO SAPIENS (HUMAN).
   Q02817
HRIFA013932a
   "SALIVARY PROLINE-RICH PROTEIN PRECURSOR (CLONES CP3, CP4 AND CP5) [CONTAINS: BASIC PEPTIDE IB-6" PEPTIDE P-H].
   2.6e-05:168:34
   HOMO SAPIENS (HUMAN).
   P04280
HRIFA013980a
   TRANSCRIPTION REGULATORY PROTEIN SNF5 (SWI/SNF COMPLEX COMPONENT SNF5) (TRANSCRIPTION FACTOR TYE4).
   0.00036:157:27
   SACCHAROMYCES CEREVISIAE (BAKER'S YEAST).
   P18480
HRIFA014006a
   T-CELL SURFACE GLYCOPROTEIN YE1/48 (T LYMPHOCYTE ANTIGEN A1) (LY49-A ANTIGEN).
   9.4e-16:185:28
   MUS MUSCULUS (MOUSE).
   P20937
HRIFA014024a
   TRANS-ACTING TRANSCRIPTIONAL PROTEIN ICP0 (P135 PROTEIN) (IER 2.9/ER2.6).
   0.0013:102:44
   BOVINE HERPESVIRUS TYPE 1 (STRAIN JURA).
   P29128
HRIFA014056a
   PROTEIN Q300.
   5.1e-05:24:70
   MUS MUSCULUS (MOUSE).
   Q02722
HRIFA014111a
   TOLL PROTEIN PRECURSOR.
   5.5e-08:203:27
   DROSOPHILA MELANOGASTER (FRUIT FLY).
   P08953
HRIFA014133a
   PROLINE-RICH PROTEIN MP-2 PRECURSOR.
   1.6e-06:143:33
   MUS MUSCULUS (MOUSE).
   P05142
HRIFA014185a
   LEUCOCYTE ANTIGEN CD97 PRECURSOR.
   6.0e-14:192:30
   HOMO SAPIENS (HUMAN).
   P48960
HRIFA014336a
   "GELSOLIN PRECURSOR, PLASMA (ACTIN-DEPOLYMERIZING FACTOR) (ADF) (BREVIN) (FRAGMENT)."
   2.8e-70:198:58
   SUS SCROFA (PIG).
   P20305
HRIFA014396a
   CREB-BINDING PROTEIN.
   2.6e-07:101:34
   MUS MUSCULUS (MOUSE).
   P45481
HRIFA014397a
   GENERAL NEGATIVE REGULATOR OF TRANSCRIPTION SUBUNIT 1.
   5.2e-05:147:30
   SACCHAROMYCES CEREVISIAE (BAKER'S YEAST).
   P25655
HRIFA014465a
   HYPOTHETICAL 59.1 KD PROTEIN ZK637.1 IN CHROMOSOME III.
   2.8e-11:166:30
   CAENORHABDITIS ELEGANS.
   P30638
HRIFA014500a
   HYPOTHETICAL 71.4 KD PROTEIN IN NMD3-ENO2 INTERGENIC REGION.
   1.0e-14:149:35
   SACCHAROMYCES CEREVISIAE (BAKER'S YEAST).
   P38862
HRIFA014561a
   PROBABLE G PROTEIN-COUPLED RECEPTOR GPR1.
   4.1e-70:156:89
   HOMO SAPIENS (HUMAN).
   P46091
HRIFA014568a
   AMINOPEPTIDASE N (EC 3.4.11.2) (MICROSOMAL AMINOPEPTIDASE).
   2.4e-40:196:44
   RATTUS NORVEGICUS (RAT).
   P15684
HRIFA014590a
   ATP SYNTHASE PROTEIN 8 (EC 3.6.1.34) (A6L).
   0.18:26:30
   GALLUS GALLUS (CHICKEN).
   P14093
HRIFA014598a
   SKIN SECRETORY PROTEIN XP2 PRECURSOR (APEG PROTEIN).
   4.9e-05:124:29
   XENOPUS LAEVIS (AFRICAN CLAWED FROG).
   P17437
HRIFA014620a
   ENL PROTEIN.
   0.58:170:30
   HOMO SAPIENS (HUMAN).
   Q03111
HRIFA014621a
   PREGNANCY-SPECIFIC BETA-1 GLYCOPROTEIN PRECURSOR.
   2.7e-50:150:74
   HOMO SAPIENS (HUMAN).
   P11462
HRIFA014688a
   INTEGRIN BETA-6 SUBUNIT PRECURSOR.
   6.9e-31:189:39
   HOMO SAPIENS (HUMAN).
   P18564
HRIFA014702a
   PROLINE-RICH PROTEIN MP-2 PRECURSOR.
   6.4e-05:89:40
   MUS MUSCULUS (MOUSE).
   P05142
HRIFA014819a
   MICROFIBRIL-ASSOCIATED GLYCOPROTEIN 4.
   7.8e-26:117:46
   HOMO SAPIENS (HUMAN).
   P55083
HRIFA014868a
   SALIVARY GLUE PROTEIN SGS-3 PRECURSOR.
   8.9e-08:195:29
   DROSOPHILA ERECTA (FRUIT FLY).
   P13730
HRIFA014951a
   PLASMINOGEN (EC 3.4.21.7) (FRAGMENT).
   4.1e-23:132:39
   EQUUS CABALLUS (HORSE).
   P80010
HRIFA014967a
   CHLORINE CHANNEL PROTEIN P64.
   2.0e-52:142:76
   BOS TAURUS (BOVINE).
   P35526
HRIFA015063a
   ZINC FINGER PROTEIN 136.
   6.6e-53:229:48
   HOMO SAPIENS (HUMAN).
   P52737
HRIFA015070a
   SERINE/THREONINE-PROTEIN KINASE NRK1 (EC 2.7.1.-) (N-RICH KINASE 1).
   9.3e-24:143:41
   SACCHAROMYCES CEREVISIAE (BAKER'S YEAST).
   P38692
HRIFA015122a
   REGULATORY PROTEIN E2.
   0.45:129:30
   HUMAN PAPILLOMAVIRUS TYPE 5.
   P06921
HRIFA015219a
   FIBRILLIN 1 PRECURSOR (MP340).
   9.9e-09:132:32
   BOS TAURUS (BOVINE).
   P98133
HRIFA015246a
   PREGNANCY-SPECIFIC BETA-1-GLYCOPROTEIN 4 PRECURSOR (PSBG-4).
   2.4e-33:184:46
   HOMO SAPIENS (HUMAN).
   Q00888
HRIFA015351a
   PROTEOGLYCAN LINK PROTEIN PRECURSOR (CARTILAGE LINK PROTEIN) (LP).
   0.0021:122:30
   RATTUS NORVEGICUS (RAT).
   P03994
HRIFA015423a
   B-CELL LYMPHOMA 3-ENCODED PROTEIN (BCL-3 PROTEIN).
   1.2e-11:148:35
   HOMO SAPIENS (HUMAN).
   P20749
HRIFA015453a
   RAC-FAMILY SERINE/THREONINE KINASE HOMOLOG (EC 2.7.1.-).
   6.8e-11:91:37
   DICTYOSTELIUM DISCOIDEUM (SLIME MOLD).
   P54644
HRIFA015486a
   BETA-2-GLYCOPROTEIN I PRECURSOR (APOLIPOPROTEIN H) (APO-H) (ACTIVATED PROTEIN C-BINDING PROTEIN) (APC INHIBITOR).
   2.0e-22:208:27
   MUS MUSCULUS (MOUSE).
   Q01339
HRIFA015506a
   COLORECTAL MUTANT CANCER PROTEIN (MCC PROTEIN).
   1.3e-12:73:50
   HOMO SAPIENS (HUMAN).
   P23508
HRIFA015536a
   CHLORINE CHANNEL PROTEIN P64.
   1.2e-49:115:79
   BOS TAURUS (BOVINE).
   P35526
HRIFA015547a
   BEM46 PROTEIN (FRAGMENT).
   1.4e-33:137:49
   SCHIZOSACCHAROMYCES POMBE (FISSION YEAST).
   P54069
HRIFA015568a
   HYPOTHETICAL 35.8 KD PROTEIN C12G12.12 IN CHROMOSOME I.
   2.4e-16:152:34
   SCHIZOSACCHAROMYCES POMBE (FISSION YEAST).
   Q09875
HRIFA015756a
   EBNA-2 NUCLEAR PROTEIN.
   2.9e-15:28:75
   EPSTEIN-BARR VIRUS (STRAIN B95-8) (HUMAN HERPESVIRUS 4).
   P12978
HRIFA015802a
   PROTEOGLYCAN LINK PROTEIN PRECURSOR (CARTILAGE LINK PROTEIN) (LP).
   0.0035:122:30
   RATTUS NORVEGICUS (RAT).
   P03994
HRIFA015811a
   GLYCOSYLTRANSFERASE ALG2 (EC 2.4.1.-).
   6.2e-39:171:43
   SACCHAROMYCES CEREVISIAE (BAKER'S YEAST).
   P43636
HRIFA015902a
   A-AGGLUTININ ATTACHMENT SUBUNIT PRECURSOR.
   0.0075:161:29
   SACCHAROMYCES CEREVISIAE (BAKER'S YEAST).
   P32323
HRIFA015947a
   ZINC FINGER Y-CHROMOSOMAL PROTEIN 1.
   0.035:98:28
   MUS MUSCULUS (MOUSE).
   P10925
HRIFA015995a
   PROCOLLAGEN ALPHA 1(III) CHAIN PRECURSOR.
   6.2e-08:221:37
   HOMO SAPIENS (HUMAN).
   P02461
HRIFA016070a
   "COMPLEMENT C1Q SUBCOMPONENT, A CHAIN PRECURSOR."
   1.0e-18:179:35
   HOMO SAPIENS (HUMAN).
   P02745
HRIFA016214a
   PROLINE-RICH PROTEIN MP-2 PRECURSOR.
   4.0e-05:96:42
   MUS MUSCULUS (MOUSE).
   P05142
HRIFA016240a
   HYPOTHETICAL 65.3 KD PROTEIN IN PRE3-SAG1 INTERGENIC REGION.
   8.5e-05:103:33
   SACCHAROMYCES CEREVISIAE (BAKER'S YEAST).
   P47082
HRIFA016255a
   EBNA-1 NUCLEAR PROTEIN.
   4.5e-09:219:33
   EPSTEIN-BARR VIRUS (STRAIN B95-8) (HUMAN HERPESVIRUS 4).
   P03211
HRIFA016290a
   COAGULATION FACTOR V PRECURSOR (ACTIVATED PROTEIN C COFACTOR).
   6.7e-21:182:41
   HOMO SAPIENS (HUMAN).
   P12259
HRIFA016430a
   ER LUMEN PROTEIN RETAINING RECEPTOR 1 (KDEL RECEPTOR 1).
   7.1e-50:120:86
   HOMO SAPIENS (HUMAN).
   P24390
HRIFA016599a
   MEIOTIC RECOMBINATION PROTEIN REC104.
   0.57:73:31
   SACCHAROMYCES CEREVISIAE (BAKER'S YEAST).
   P33323
HRIFA016639a
   "GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE)."
   8.0e-06:206:23
   SACCHAROMYCES CEREVISIAE (BAKER'S YEAST).
   P08640
HRIFA016654a
   HEME-REGULATED EUKARYOTIC INITIATION FACTOR EIF-2-ALPHA KINASE (EC 2.7.1.-) (HRI).
   1.1e-78:181:86
   ORYCTOLAGUS CUNICULUS (RABBIT).
   P33279
HRIFA016669a
   ANTER-SPECIFIC PROLINE-RICH PROTEIN APG PRECURSOR.
   1.4e-08:87:36
   ARABIDOPSIS THALIANA (MOUSE-EAR CRESS).
   P40602
HRIFA016758a
   GLYCOSYLTRANSFERASE ALG2 (EC 2.4.1.-).
   9.5e-17:158:40
   SACCHAROMYCES CEREVISIAE (BAKER'S YEAST).
   P43636
HRIFA016963a
   FMRFAMIDE-RELATED NEUROPEPTIDES PRECURSOR.
   6.2e-08:131:32
   LYMNAEA STAGNALIS (GREAT POND SNAIL).
   P42565
HRIFA017031a
   MYOSIN HEAVY CHAIN KINASE A (EC 2.7.1.129) (MHCK A).
   2.6e-11:152:34
   DICTYOSTELIUM DISCOIDEUM (SLIME MOLD).
   P42527
HRIFA017146a
   D(4) DOPAMINE RECEPTOR (D(2C) DOPAMINE RECEPTOR).
   0.0014:78:37
   HOMO SAPIENS (HUMAN).
   P21917
HRIFA017190a
   FLI-1 ONCOGENE (ERGB TRANSCRIPTION FACTOR).
   0.0026:89:30
   HOMO SAPIENS (HUMAN).
   Q01543
HRIFA017257a
   "GELSOLIN PRECURSOR, PLASMA (ACTIN-DEPOLYMERIZING FACTOR) (ADF) (BREVIN) (AGEL)."
   2.5e-79:261:57
   HOMO SAPIENS (HUMAN).
   P06396
HRIFA017295a
   "ALPHA-1,6-MANNOSYL-GLYCOPROTEIN BETA-1,2-N-ACETYLGLUCOSAMINYLTRANSFERASE (EC 2.4.1.143) (N-GLYCOSYL-OLIGOSACCHARIDE-GLYCOPROTEIN N-ACETYLGLUCOSAMINYLTRANSFERASE II) (BETA-1,2-N-ACETYLGLUCOSAMINYLTRANSFERASE II) (GNT-II) (GLCNAC-T II)."
   3.4e-20:66:78
   HOMO SAPIENS (HUMAN).
   Q10469
HRIFA017312a
   C4B-BINDING PROTEIN ALPHA CHAIN PRECURSOR (PROLINE-RICH PROTEIN) (PRP).
   2.7e-19:221:33
   HOMO SAPIENS (HUMAN).
   P04003
HRIFA017456a
   LAMININ ALPHA-1 CHAIN PRECURSOR (LAMININ A CHAIN).
   0.11:94:35
   MUS MUSCULUS (MOUSE).
   P19137
HRIFA017457a
   SYNAPTOTAGMIN II.
   7.2e-07:98:35
   MUS MUSCULUS (MOUSE).
   P46097
HRIFA017643a
   NOV PROTEIN HOMOLOG PRECURSOR (NOVH).
   2.2e-07:81:41
   HOMO SAPIENS (HUMAN).
   P48745
HRIFA017670a
   TRANS-GOLGI NETWORK INTEGRAL MEMBRANE PROTEIN TGN38 PRECURSOR.
   4.9e-06:172:27
   RATTUS NORVEGICUS (RAT).
   P19814
HRIFA017703a
   PUTATIVE SERINE/THREONINE-PROTEIN KINASE PKWA (EC 2.7.1.-).
   1.9e-16:129:34
   THERMOMONOSPORA CURVATA.
   P49695
HRIFA017791a
   MUCIN 2 PRECURSOR (INTESTINAL MUCIN 2).
   0.012:71:38
   HOMO SAPIENS (HUMAN).
   Q02817
HRIFA017801a
   PROLINE-RICH PROTEIN MP-2 PRECURSOR.
   4.5e-07:86:39
   MUS MUSCULUS (MOUSE).
   P05142
HRIFA017818a
   ATP SYNTHASE C CHAIN (EC 3.6.1.34) (LIPID-BINDING PROTEIN).
   1.0:32:40
   STREPTOMYCES LIVIDANS.
   P50014
HRIFA017836a
   "TETRACYCLINE RESISTANCE PROTEIN, CLASS H (TETA(H))."
   1.3e-08:113:31
   PASTEURELLA MULTOCIDA.
   P51564
HRIFA017855a
   ORM1 PROTEIN.
   1.7e-18:137:35
   SACCHAROMYCES CEREVISIAE (BAKER'S YEAST).
   P53224
HRIFA017921a
   TRANS-ACTING TRANSCRIPTIONAL PROTEIN ICP0 (IMMEDIATE-EARLY PROTEIN IE110) (VMW110) (ALPHA-0 PROTEIN).
   2.0e-09:182:35
   HERPES SIMPLEX VIRUS (TYPE 1 / STRAIN 17).
   P08393
HRIFA018092a
   "DIACYLGLYCEROL CHOLINEPHOSPHOTRANSFERASE (EC 2.7.8.2) (SN-1,2-DIACYLGLYCEROL CHOLINEPHOSPHOTRANSFERASE) (CHOPT)."
   2.1e-20:119:42
   SACCHAROMYCES CEREVISIAE (BAKER'S YEAST).
   P17898
HRIFA018131a
   ORM1 PROTEIN.
   2.6e-20:137:37
   SACCHAROMYCES CEREVISIAE (BAKER'S YEAST).
   P53224
HRIFA018134a
   SERINE/THREONINE-PROTEIN KINASE CTR1 (EC 2.7.1.37).
   1.1e-11:147:32
   ARABIDOPSIS THALIANA (MOUSE-EAR CRESS).
   Q05609
HRIFA018238a
   NEUROGENIC LOCUS NOTCH HOMOLOG PROTEIN 1 PRECURSOR (MOTCH PROTEIN).
   8.6e-06:74:44
   MUS MUSCULUS (MOUSE).
   Q01705
HRIFA018262a
   PUTATIVE SERINE/THREONINE-PROTEIN KINASE PKWA (EC 2.7.1.-).
   6.4e-10:71:38
   THERMOMONOSPORA CURVATA.
   P49695
HRIFA018287a
   HYPOTHETICAL 57.5 KD PROTEIN IN VMA7-RPS25A INTERGENIC REGION.
   1.5e-06:214:32
   SACCHAROMYCES CEREVISIAE (BAKER'S YEAST).
   P53214
HRIFA018447a
   SKIN SECRETORY PROTEIN XP2 PRECURSOR (APEG PROTEIN).
   0.00065:133:33
   XENOPUS LAEVIS (AFRICAN CLAWED FROG).
   P17437
HRIFA018580a
   COP-COATED VESICLE MEMBRANE PROTEIN P24 PRECURSOR (FRAGMENT).
   2.1e-18:109:41
   CRICETULUS GRISEUS (CHINESE HAMSTER).
   P49020
HRIFA018666a
   PROTEIN-TYROSINE PHOSPHATASE DLAR PRECURSOR (EC 3.1.3.48) (PROTEIN-TYROSINE-PHOSPHATE PHOSPHOHYDROLASE).
   1.7e-06:191:28
   DROSOPHILA MELANOGASTER (FRUIT FLY).
   P16621
HRIFA018688a
   PHLB PROTEIN PRECURSOR.
   1.9e-06:110:35
   SERRATIA LIQUEFACIENS.
   P18954
HRIFA018754a
   "GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE)."
   1.8e-06:195:27
   SACCHAROMYCES CEREVISIAE (BAKER'S YEAST).
   P08640
HRIFA018794a
   MSP1 PROTEIN HOMOLOG.
   3.2e-06:93:25
   CAENORHABDITIS ELEGANS.
   P54815
HRIFA018827a
   HYPOTHETICAL 59.1 KD PROTEIN ZK637.1 IN CHROMOSOME III.
   3.1e-17:180:28
   CAENORHABDITIS ELEGANS.
   P30638
HRIFA018870a
   HYPOTHETICAL 35.6 KD PROTEIN IN SPC1-ILV3 INTERGENIC REGION.
   4.7e-09:70:37
   SACCHAROMYCES CEREVISIAE (BAKER'S YEAST).
   P47088
HRIFA018904a
   MYOTONIN-PROTEIN KINASE (EC 2.7.1.-) (MYOTONIC DISTROPHY PROTEIN KINASE) (MDPK) (DM-KINASE) (DMK) (DMPK) (MT-PK).
   5.5e-12:142:32
   HOMO SAPIENS (HUMAN).
   Q09013
HRIFA018931a
   ZINC FINGER PROTEIN 140.
   2.9e-10:47:74
   HOMO SAPIENS (HUMAN).
   P52738
HRIFA018993a
   HYPOTHETICAL 21.5 KD PROTEIN IN SEC15-SAP4 INTERGENIC REGION.
   1.2e-13:117:34
   SACCHAROMYCES CEREVISIAE (BAKER'S YEAST).
   P53073
HRIFA019105a
   DORSAL-VENTRAL PATTERNING TOLLOID PROTEIN PRECURSOR (EC 3.4.24.-).
   7.5e-22:203:32
   DROSOPHILA MELANOGASTER (FRUIT FLY).
   P25723
HRIFA019136a
   "MYRISTOYLATED ALANINE-RICH C-KINASE SUBSTRATE (MARCKS) (PROTEIN KINASE C SUBSTRATE, 80 KD PROTEIN, LIGHT CHAIN) (PKCSL) (80K-L PROTEIN)."
   1.0e-25:74:81
   HOMO SAPIENS (HUMAN).
   P29966
HRIFA019175a
   PROTEIN KINASE WIS1 (EC 2.7.1.-).
   1.3e-14:84:39
   SCHIZOSACCHAROMYCES POMBE (FISSION YEAST).
   P33886
HRIFA019262a
   ZINC FINGER PROTEIN 85 (ZINC FINGER PROTEIN HPF4) (HTF1).
   2.5e-55:188:50
   HOMO SAPIENS (HUMAN).
   Q03923
HRIFA019412a
   CATHEPSIN E PRECURSOR (EC 3.4.23.34).
   1.4e-09:121:33
   CAVIA PORCELLUS (GUINEA PIG).
   P25796
HRIFA019437a
   REGULATORY PROTEIN E2.
   0.26:77:37
   HUMAN PAPILLOMAVIRUS TYPE 14.
   P36783
HRIFA019466a
   EBNA-1 NUCLEAR PROTEIN.
   2.7e-19:130:43
   EPSTEIN-BARR VIRUS (STRAIN B95-8) (HUMAN HERPESVIRUS 4).
   P03211
HRIFA019490a
   TRANSCRIPTION REGULATORY PROTEIN SNF5 (SWI/SNF COMPLEX COMPONENT SNF5) (TRANSCRIPTION FACTOR TYE4).
   1.1e-09:132:34
   SACCHAROMYCES CEREVISIAE (BAKER'S YEAST).
   P18480
HRIFA019498a
   VOLTAGE-GATED POTASSIUM CHANNEL PROTEIN SHAL (SHAL2).
   5.6e-05:87:36
   DROSOPHILA MELANOGASTER (FRUIT FLY).
   P17971
HRIFA019532a
   EBNA-1 NUCLEAR PROTEIN.
   1.8e-05:67:49
   EPSTEIN-BARR VIRUS (STRAIN B95-8) (HUMAN HERPESVIRUS 4).
   P03211
HRIFA019651a
   ACIDIC PHOSPHOPROTEIN PRECURSOR (50 KD ANTIGEN).
   6.1e-05:31:64
   PLASMODIUM CHABAUDI.
   Q02752
HRIFA019867a
   RENAL SODIUM-DEPENDENT PHOSPHATE TRANSPORT PROTEIN 2 (SODIUM/PHOSPHATE COTRANSPORTER 2) (NA(+)/PI COTRANSPORTER 2) (RENAL SODIUM-PHOSPHATE TRANSPORT PROTEIN 2) (RENAL NA+-DEPENDENT PHOSPHATE COTRANSPORTER 2).
   8.2e-34:103:71
   RATTUS NORVEGICUS (RAT).
   Q06496
HRIFA019869a
   SERINE/THREONINE-PROTEIN KINASE FUSED (EC 2.7.1.-).
   7.2e-29:83:49
   DROSOPHILA MELANOGASTER (FRUIT FLY).
   P23647
HRIFA019958a
   REPRESSOR PROTEIN CI (FRAGMENT).
   0.99:45:37
   BACTERIOPHAGE 434.
   P16117
HRIFA020144a
   SKIN SECRETORY PROTEIN XP2 PRECURSOR (APEG PROTEIN).
   2.8e-06:176:30
   XENOPUS LAEVIS (AFRICAN CLAWED FROG).
   P17437
HRIFA020184a
   NON-RECEPTOR TYROSINE KINASE SPORE LYSIS A (EC 2.7.1.112) (TYROSINE-PROTEIN KINASE 1).
   1.9e-10:102:37
   DICTYOSTELIUM DISCOIDEUM (SLIME MOLD).
   P18160
HRIFA020272a
   MUSCARINIC ACETYLCHOLINE RECEPTOR M3.
   5.5e-91:211:85
   HOMO SAPIENS (HUMAN).
   P20309
HRIFA020335a
   PUTATIVE SERINE/THREONINE-PROTEIN KINASE P78 (EC 2.7.1.-).
   5.0e-104:275:72
   HOMO SAPIENS (HUMAN).
   P27448
HRIFA020349a
   BRITTLE-1 PROTEIN PRECURSOR.
   6.0e-30:214:35
   ZEA MAYS (MAIZE).
   P29518
HRIFA020453a
   PROTEIN TRANSPORT PROTEIN SEC22 (PROTEIN SLY2).
   2.5e-08:132:28
   SACCHAROMYCES CEREVISIAE (BAKER'S YEAST).
   P22214
HRIFA020693a
   TUMOR SUPPRESSOR PROTEIN DCC PRECURSOR (COLORECTAL CANCER SUPPRESSOR).
   3.9e-09:96:35
   HOMO SAPIENS (HUMAN).
   P43146
HRIFA020707a
   PROCYCLIC FORM SPECIFIC POLYPEPTIDE B-ALPHA PRECURSOR (PROCYCLIN) (PARP B-ALPHA) (PSSA-1).
   3.4e-09:95:33
   TRYPANOSOMA BRUCEI BRUCEI.
   Q06084
HRIFA020748a
   EXTENSIN PRECURSOR (CELL WALL HYDROXYPROLINE-RICH GLYCOPROTEIN).
   3.2e-09:210:28
   NICOTIANA TABACUM (COMMON TOBACCO).
   P13983
HRIFA020862a
   MODIFIER 3 PROTEIN (M33).
   5.6e-26:76:61
   MUS MUSCULUS (MOUSE).
   P30658
HRIFA020883a
   PROTEIN Q300.
   0.00054:21:66
   MUS MUSCULUS (MOUSE).
   Q02722
HRIFA021007a
   TRANS-ACTING TRANSCRIPTIONAL PROTEIN ICP0 (IMMEDIATE-EARLY PROTEIN IE110) (VMW110) (ALPHA-0 PROTEIN).
   0.092:73:36
   HERPES SIMPLEX VIRUS (TYPE 1 / STRAIN 17).
   P08393
HRIFA021040a
   TRANSCRIPTION FACTOR GATA-4 (GATA BINDING FACTOR-4).
   0.98:63:39
   HOMO SAPIENS (HUMAN).
   P43694
HRIFA021061a
   SKIN SECRETORY PROTEIN XP2 PRECURSOR (APEG PROTEIN).
   2.8e-09:162:31
   XENOPUS LAEVIS (AFRICAN CLAWED FROG).
   P17437
HRIFA021213a
   OLIGOSACCHARYL TRANSFERASE STT3 SUBUNIT HOMOLOG.
   2.0e-38:96:72
   CAENORHABDITIS ELEGANS.
   P46975
HRIFA021224a
   RENAL TRANSCRIPTION FACTOR KID-1 (TRANSCRIPTION FACTOR 17).
   2.8e-06:55:52
   RATTUS NORVEGICUS (RAT).
   Q02975
HRIFA021398a
   COAGULATION FACTOR VII PRECURSOR (EC 3.4.21.21).
   2.5e-17:78:51
   ORYCTOLAGUS CUNICULUS (RABBIT).
   P98139
HRIFA021445a
   PRE-B-CELL LEUKEMIA TRANSCRIPTION FACTOR-1 (HOMEOBOX PROTEIN PBX1) (HOMEOBOX PROTEIN PRL).
   0.38:146:31
   HOMO SAPIENS (HUMAN).
   P40424
HRIFA021494a
   EBNA-1 NUCLEAR PROTEIN.
   6.8e-07:116:41
   EPSTEIN-BARR VIRUS (STRAIN B95-8) (HUMAN HERPESVIRUS 4).
   P03211
HRIFA021499a
   CARTILAGE MATRIX PROTEIN PRECURSOR (MATRILIN-1).
   7.1e-34:159:50
   GALLUS GALLUS (CHICKEN).
   P05099
HRIFA021543a
   ANTITHROMBIN-III PRECURSOR (ATIII) (FRAGMENT).
   0.0087:50:40
   GALLUS GALLUS (CHICKEN).
   Q03352
HRIFA021620a
   PLATELET FACTOR 4 (PF-4).
   0.019:65:27
   SUS SCROFA (PIG).
   P30034
HRIFA021637a
   CARTILAGE MATRIX PROTEIN PRECURSOR (MATRILIN-1).
   6.0e-37:147:53
   GALLUS GALLUS (CHICKEN).
   P05099
HRIFA021651a
   CARG-BINDING FACTOR-A (CBF-A).
   2.6e-11:170:30
   MUS MUSCULUS (MOUSE).
   Q99020
HRIFA021754a
   CARTILAGE MATRIX PROTEIN PRECURSOR (MATRILIN-1).
   1.2e-37:137:51
   GALLUS GALLUS (CHICKEN).
   P05099
HRIFA021781a
   DNA-REPAIR PROTEIN COMPLEMENTING XP-D CELLS (XERODERMA PIGMENTOSUM GROUP D COMPLEMENTING PROTEIN) (DNA EXCISION REPAIR PROTEIN ERCC-2).
   7.1e-19:199:31
   HOMO SAPIENS (HUMAN).
   P18074
HRIFA021787a
   PROTEIN Q300.
   0.051:13:84
   MUS MUSCULUS (MOUSE).
   Q02722
HRIFA021794a
   RAC-FAMILY SERINE/THREONINE KINASE HOMOLOG (EC 2.7.1.-).
   1.6e-07:90:32
   DICTYOSTELIUM DISCOIDEUM (SLIME MOLD).
   P54644
HRIFA021855a
   SKIN SECRETORY PROTEIN XP2 PRECURSOR (APEG PROTEIN).
   8.6e-06:163:30
   XENOPUS LAEVIS (AFRICAN CLAWED FROG).
   P17437
HRIFA021906a
   S-ANTIGEN PROTEIN PRECURSOR.
   2.1e-09:226:28
   PLASMODIUM FALCIPARUM (ISOLATE V1).
   P09593
HRIFA022055a
   BETA-LYTIC METALLOENDOPEPTIDASE PRECURSOR (EC 3.4.24.32) (BETA-LYTIC PROTEASE).
   0.63:118:31
   ACHROMOBACTER LYTICUS.
   P27458
HRIFA022065a
   BETA GALACTOSIDASE-RELATED PROTEIN PRECURSOR.
   9.7e-24:235:34
   HOMO SAPIENS (HUMAN).
   P16279
HRIFA022139a
   HYPOTHETICAL 85.7 KD PROTEIN C13G6.03 IN CHROMOSOME I.
   2.1e-57:232:52
   SCHIZOSACCHAROMYCES POMBE (FISSION YEAST).
   Q09782
HRIFA022156a
   "GLUTENIN, HIGH MOLECULAR WEIGHT SUBUNIT PW212 PRECURSOR."
   1.4e-07:133:35
   TRITICUM AESTIVUM (WHEAT).
   P08489
HRIFA022166a
   EXCISION REPAIR PROTEIN ERCC-6 (COCKAYNE SYNDROME PROTEIN CSB).
   3.5e-28:194:35
   HOMO SAPIENS (HUMAN).
   Q03468
HRIFA022177a
   PUTATIVE SERINE/THREONINE-PROTEIN KINASE PKWA (EC 2.7.1.-).
   2.2e-12:137:32
   THERMOMONOSPORA CURVATA.
   P49695
HRIFA022182a
   SERINE/THREONINE-PROTEIN KINASE MAK (EC 2.7.1.-) (MALE GERM CELL-ASSOCIATED KINASE).
   1.2e-47:121:79
   RATTUS NORVEGICUS (RAT).
   P20793
HRIFA022203a
   COLLAGEN ALPHA 1 (III) CHAIN.
   1.1e-05:211:33
   BOS TAURUS (BOVINE).
   P04258
HRIFA022227a
   POTENTIAL CAAX PRENYL PROTEASE 1 (EC 3.4.24.-) (PRENYL PROTEIN- SPECIFIC ENDOPROTEASE 1) (PPSEP 1).
   3.2e-31:229:36
   SCHIZOSACCHAROMYCES POMBE (FISSION YEAST).
   Q10071
HRIFA022234a
   CARBOXYPEPTIDASE KEX1 PRECURSOR (EC 3.4.16.6) (CARBOXYPEPTIDASE D).
   1.8e-08:110:30
   SACCHAROMYCES CEREVISIAE (BAKER'S YEAST).
   P09620
HRIFA022249a
   ZINC FINGER PROTEIN 133.
   1.1e-34:84:48
   HOMO SAPIENS (HUMAN).
   P52736
HRIFA022265a
   CALCIUM/CALMODULIN-DEPENDENT PROTEIN KINASE TYPE IV CATALYTIC CHAIN (EC 2.7.1.123) (CAM KINASE-GR) (CAMK IV) [CONTAINS: CALSPERMIN].
   5.1e-26:188:40
   RATTUS NORVEGICUS (RAT).
   P13234
HRIFA022328a
   SCO1 PROTEIN PRECURSOR.
   5.4e-25:84:45
   SACCHAROMYCES CEREVISIAE (BAKER'S YEAST).
   P23833
HRIFA022335a
   TRANS-ACTING TRANSCRIPTIONAL PROTEIN ICP0 (IMMEDIATE-EARLY PROTEIN IE110) (VMW110) (ALPHA-0 PROTEIN).
   0.21:121:29
   HERPES SIMPLEX VIRUS (TYPE 1 / STRAIN 17).
   P08393
HRIFA022348a
   AGAMOUS PROTEIN.
   1.0:40:42
   ARABIDOPSIS THALIANA (MOUSE-EAR CRESS).
   P17839
HRIFA022411a
   TRANS-ACTING TRANSCRIPTIONAL PROTEIN ICP0 (IMMEDIATE-EARLY PROTEIN IE110) (VMW110) (ALPHA-0 PROTEIN).
   0.00059:111:35
   HERPES SIMPLEX VIRUS (TYPE 1 / STRAIN 17).
   P08393
HRIFA022423a
   HYPOTHETICAL 24.5 KD PROTEIN IN SAP185-BCK1 INTERGENIC REGION.
   2.5e-15:106:42
   SACCHAROMYCES CEREVISIAE (BAKER'S YEAST).
   P40857
HRIFA022462a
   RETINOIC ACID RECEPTOR RXR-BETA.
   0.0010:124:33
   HOMO SAPIENS (HUMAN).
   P28702
HRIFA022493a
   ALPHA-2A ADRENERGIC RECEPTOR (ALPHA-2A ADRENOCEPTOR) (ALPHA-2AAR).
   0.0018:130:34
   MUS MUSCULUS (MOUSE).
   Q01338
HRIFA022528a
   EXTENSIN PRECURSOR (PROLINE-RICH GLYCOPROTEIN).
   3.2e-23:230:28
   ZEA MAYS (MAIZE).
   P14918
HRIFA022546a
   NINAC SHORT PROTEIN (EC 2.7.1.-).
   8.5e-42:209:43
   DROSOPHILA MELANOGASTER (FRUIT FLY).
   P10677
HRIFA022564a
   ZINC FINGER PROTEIN 140.
   7.9e-23:116:51
   HOMO SAPIENS (HUMAN).
   P52738
HRIFA022616a
   LOW-DENSITY LIPOPROTEIN RECEPTOR-RELATED PROTEIN 1 PRECURSOR (LRP) (ALPHA-2-MACROGLOBULIN RECEPTOR) (A2MR) (APOLIPOPROTEIN E RECEPTOR) (APOER) (CD91).
   7.4e-36:172:43
   HOMO SAPIENS (HUMAN).
   Q07954
HRIFA022671a
   PAIRED AMPHIPATHIC HELIX PROTEIN.
   2.0e-26:186:36
   SACCHAROMYCES CEREVISIAE (BAKER'S YEAST).
   P22579
HRIFA022691a
   FIBRINOGEN-LIKE PROTEIN A PRECURSOR (FREP-A).
   1.4e-44:229:41
   PARASTICHOPUS PARVIMENSIS (SEA CUCUMBER).
   P19477
HRIFA022702a
   PROCOLLAGEN ALPHA 1(I) CHAIN PRECURSOR.
   1.1e-08:146:38
   GALLUS GALLUS (CHICKEN).
   P02457
HRIFA022707a
   GC-RICH SEQUENCE DNA-BINDING FACTOR (GCF) (TRANSCRIPTION FACTOR 9) (TCF-9).
   7.0e-40:229:37
   HOMO SAPIENS (HUMAN).
   P16383
HRIFA022714a
   "AMELOGENIN, CLASS I PRECURSOR."
   0.62:96:31
   BOS TAURUS (BOVINE).
   P02817
HRIFA022728a
   ACROSIN PRECURSOR (EC 3.4.21.10) (53 KD FUCOSE-BINDING PROTEIN).
   1.7e-06:28:64
   SUS SCROFA (PIG).
   P08001
HRIFA022729a
   "ALPHA-1,6-MANNOSYL-GLYCOPROTEIN BETA-1,2-N-ACETYLGLUCOSAMINYLTRANSFERASE (EC 2.4.1.143) (N-GLYCOSYL-OLIGOSACCHARIDE-GLYCOPROTEIN N-ACETYLGLUCOSAMINYLTRANSFERASE II) (BETA-1,2-N-ACETYLGLUCOSAMINYLTRANSFERASE II) (GNT-II) (GLCNAC-T II)."
   7.7e-29:69:84
   HOMO SAPIENS (HUMAN).
   Q10469
HRIFA022737a
   TENASCIN PRECURSOR (TN) (HEXABRACHION) (CYTOTACTIN) (NEURONECTIN) (GMEM) (JI) (MIOTENDINOUS ANTIGEN) (GLIOMA-ASSOCIATED-EXTRACELLULAR MATRIX ANTIGEN) (GP 150-225).
   6.7e-19:170:37
   GALLUS GALLUS (CHICKEN).
   P10039
HRIFA022776a
   PROBABLE PROTEIN DISULFIDE ISOMERASE P5 PRECURSOR (EC 5.3.4.1).
   4.0e-20:199:31
   MEDICAGO SATIVA (ALFALFA).
   P38661
HRIFA022782a
   CIRCUMSPOROZOITE PROTEIN PRECURSOR (CS).
   3.7e-09:184:36
   PLASMODIUM CYNOMOLGI (STRAIN BEROK).
   P08672
HRIFA022865a
   COLLAGEN ALPHA 1(III) CHAIN.
   2.5e-09:169:33
   BOS TAURUS (BOVINE).
   P04258
HRIFA022875a
   BONE PROTEOGLYCAN II PRECURSOR (PG-S2) (DECORIN).
   9.1e-14:115:33
   BOS TAURUS (BOVINE).
   P21793
HRIFA022890a
   SKIN SECRETORY PROTEIN XP2 PRECURSOR (APEG PROTEIN).
   1.8e-10:237:30
   XENOPUS LAEVIS (AFRICAN CLAWED FROG).
   P17437
HRIFA022895a
   ZINC FINGER PROTEIN 85 (ZINC FINGER PROTEIN HPF4) (HTF1).
   2.4e-106:283:67
   HOMO SAPIENS (HUMAN).
   Q03923
HRIFA022985a
   PROCYCLIC FORM SPECIFIC POLYPEPTIDE B-ALPHA PRECURSOR (PROCYCLIN) (PARP B-ALPHA) (PSSA-1).
   3.0e-10:33:72
   TRYPANOSOMA BRUCEI BRUCEI.
   Q06084
HRIFA023007a
   MHC CLASS II REGULATORY FACTOR RFX1 (RFX) (ENHANCER FACTOR C) (EF-C).
   1.1e-27:66:54
   HOMO SAPIENS (HUMAN).
   P22670
HRIFA023048a
   COLLAGEN ALPHA 1(I) CHAIN (FRAGMENTS).
   2.2e-07:221:33
   RATTUS NORVEGICUS (RAT).
   P02454
HRIFA023069a
   BASEMENT MEMBRANE-SPECIFIC HEPARAN SULFATE PROTEOGLYCAN CORE PROTEIN PRECURSOR (HSPG) (PERLECAN) (PLC).
   3.4e-08:149:31
   HOMO SAPIENS (HUMAN).
   P98160
HRIFA023129a
   HISTIDINE-RICH GLYCOPROTEIN PRECURSOR.
   4.2e-06:37:51
   PLASMODIUM LOPHURAE.
   P04929
HRIFA023154a
   GLYCOPROTEIN X PRECURSOR.
   8.2e-05:140:27
   EQUINE HERPESVIRUS TYPE 1 (STRAIN AB4P) (EHV-1).
   P28968
HRIFA023212a
   A-AGGLUTININ ATTACHMENT SUBUNIT PRECURSOR.
   8.3e-10:249:32
   SACCHAROMYCES CEREVISIAE (BAKER'S YEAST).
   P32323
HRIFA023227a
   GALACTOSE-PROTON SYMPORT (GALACTOSE TRANSPORTER).
   9.2e-15:180:30
   ESCHERICHIA COLI.
   P37021
HRIFA023257a
   PROTEIN TRANSPORT PROTEIN SEC61 ALPHA SUBUNIT.
   2.4e-118:229:88
   RATTUS NORVEGICUS (RAT).
   P38378
HRIFA023304a
   PROBABLE CALCIUM-TRANSPORTING ATPASE 3 (EC 3.6.1.38) (ENDOPLASMIC RETICULUM CA2+-ATPASE).
   1.3e-23:222:29
   SACCHAROMYCES CEREVISIAE (BAKER'S YEAST).
   P39524
HRIFA023434a
   VOLTAGE-GATED POTASSIUM CHANNEL PROTEIN KV1.6 (RCK2) (KV2).
   0.00018:157:30
   RATTUS NORVEGICUS (RAT).
   P17659
HRIFA023464a
   GLYCINE-RICH CELL WALL STRUCTURAL PROTEIN 2 PRECURSOR.
   1.0e-11:75:46
   ORYZA SATIVA (RICE).
   P29834
HRIFA023489a
   HYPOTHETICAL 111.9 KD PROTEIN C22H10.03C IN CHROMOSOME I.
   4.4e-09:230:23
   SCHIZOSACCHAROMYCES POMBE (FISSION YEAST).
   Q10297
HRIFA023634a
   EBNA-1 NUCLEAR PROTEIN.
   1.8e-08:113:45
   EPSTEIN-BARR VIRUS (STRAIN B95-8) (HUMAN HERPESVIRUS 4).
   P03211
HRIFA023767a
   CYTOCHROME B5.
   1.1e-12:92:38
   SACCHAROMYCES CEREVISIAE (BAKER'S YEAST).
   P40312
HRIFA023894a
   PROLINE-RICH PROTEIN MP-2 PRECURSOR.
   3.6e-05:80:40
   MUS MUSCULUS (MOUSE).
   P05142
HRIFA023923a
   CYTOCHROME C OXIDASE POLYPEPTIDE I (EC 1.9.3.1).
   4.2e-76:128:85
   HOMO SAPIENS (HUMAN).
   P00395
HRIFA024088a
   NEURON-SPECIFIC X11 PROTEIN (FRAGMENT).
   1.1e-05:118:32
   MUS MUSCULUS (MOUSE).
   P98084
HRIFA024132a
   VOLTAGE-GATED POTASSIUM CHANNEL PROTEIN KV1.9.
   6.5e-40:136:61
   HOMO SAPIENS (HUMAN).
   P51787
HRIFA024185a
   ETS-DOMAIN TRANSCRIPTION FACTOR ERF.
   0.55:128:29
   HOMO SAPIENS (HUMAN).
   P50548
HRIFA024197a
   MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP (CARRIER) (TRANSLOCASE OF OUTER MEMBRANE TOM70).
   7.5e-09:93:34
   NEUROSPORA CRASSA.
   P23231
HRIFA024218a
   PROCOLLAGEN ALPHA 1(I) CHAIN PRECURSOR.
   6.7e-06:180:36
   HOMO SAPIENS (HUMAN).
   P02452
HRIFA024255a
   HYPOTHETICAL 116.3 KD PROTEIN C26F1.09 IN CHROMOSOME I.
   4.8e-23:172:33
   SCHIZOSACCHAROMYCES POMBE (FISSION YEAST).
   Q10496
HRIFA024305a
   CYCLIC-AMP-DEPENDENT TRANSCRIPTION FACTOR ATF-6 (FRAGMENT).
   0.047:47:29
   HOMO SAPIENS (HUMAN).
   P18850
HRIFA024392a
   TRANSMEMBRANE PROTEIN SEX PRECURSOR.
   6.7e-24:119:43
   HOMO SAPIENS (HUMAN).
   P51805
HRIFA024423a
   COP-COATED VESICLE MEMBRANE PROTEIN P24 PRECURSOR (FRAGMENT).
   2.1e-18:109:41
   CRICETULUS GRISEUS (CHINESE HAMSTER).
   P49020
HRIFA024473a
   COLLAGEN ALPHA 1(I) CHAIN (FRAGMENTS).
   3.3e-05:106:41
   BOS TAURUS (BOVINE).
   P02453
HRIFA024482a
   PISTIL-SPECIFIC EXTENSIN-LIKE PROTEIN PRECURSOR (PELP).
   1.2e-07:99:31
   NICOTIANA TABACUM (COMMON TOBACCO).
   Q03211
HRIFA024504a
   ESTRADIOL 17 BETA-DEHYDROGENASE 3 (EC 1.1.1.62) (17-BETA-HSD 3) (TESTICULAR 17-BETA-HYDROXYSTEROID DEHYDROGENASE).
   2.6e-43:205:49
   HOMO SAPIENS (HUMAN).
   P37058
HRIFA024543a
   GLYCOPROTEIN X PRECURSOR.
   1.5e-06:257:28
   EQUINE HERPESVIRUS TYPE 1 (STRAIN AB4P) (EHV-1).
   P28968
HRIFA024718a
   BETA-GALACTOSIDASE PRECURSOR (EC 3.2.1.23) (LACTASE) (ACID BETA-GALACTOSIDASE).
   5.3e-45:168:52
   MUS MUSCULUS (MOUSE).
   P23780
HRIFA024767a
   SODIUM CHANNEL PROTEIN (NA+ CHANNEL).
   7.4e-30:221:31
   ELECTROPHORUS ELECTRICUS (ELECTRIC EEL).
   P02719
HRIFA024884a
   HYPOTHETICAL 13.6 KD PROTEIN IN SPT4-ROM1 INTERGENIC REGION.
   0.0089:23:65
   SACCHAROMYCES CEREVISIAE (BAKER'S YEAST).
   P53245
HRIFA024893a
   REGULATORY PROTEIN E2.
   0.0021:167:31
   HUMAN PAPILLOMAVIRUS TYPE 8.
   P06422
HRIFA024937a
   GNS1 PROTEIN.
   1.0e-15:173:33
   SACCHAROMYCES CEREVISIAE (BAKER'S YEAST).
   P25358
HRIFA024978a
   MUCIN 2 PRECURSOR (INTESTINAL MUCIN 2).
   0.00019:150:32
   HOMO SAPIENS (HUMAN).
   Q02817
HRIFA024994a
   EXTENSIN PRECURSOR (CELL WALL HYDROXYPROLINE-RICH GLYCOPROTEIN).
   5.3e-22:145:46
   NICOTIANA TABACUM (COMMON TOBACCO).
   P13983
HRIFA025033a
   TRANS-ACTING TRANSCRIPTIONAL PROTEIN ICP0 (VMW118 PROTEIN).
   0.50:215:29
   HERPES SIMPLEX VIRUS (TYPE 2 / STRAIN HG52).
   P28284
HRIFA025046a
   PROBABLE CALCIUM-TRANSPORTING ATPASE 6 (EC 3.6.1.38).
   1.7e-41:104:48
   SACCHAROMYCES CEREVISIAE (BAKER'S YEAST).
   P39986
HRIFA025250a
   "PROTEIN KINASE C, BRAIN ISOZYME (EC 2.7.1.-) (PKC) (DPKC53E(BR))."
   7.4e-17:126:34
   DROSOPHILA MELANOGASTER (FRUIT FLY).
   P05130
HRIFA025261a
   MYOSIN I ALPHA (MMI-ALPHA).
   2.3e-64:141:84
   MUS MUSCULUS (MOUSE).
   P46735
HRIFA025290a
   EBNA-1 NUCLEAR PROTEIN.
   0.016:79:40
   EPSTEIN-BARR VIRUS (STRAIN B95-8) (HUMAN HERPESVIRUS 4).
   P03211
HRIFA025327a
   PROLINE-RICH PROTEIN MP-2 PRECURSOR.
   2.3e-06:104:37
   MUS MUSCULUS (MOUSE).
   P05142
HRIFA025353a
   GLYCINE-RICH CELL WALL STRUCTURAL PROTEIN 2 PRECURSOR.
   1.0e-11:75:46
   ORYZA SATIVA (RICE).
   P29834
HRIFA025479a
   PROTEASE DEGS PRECURSOR (EC 3.4.21.-).
   3.0e-05:112:33
   ESCHERICHIA COLI.
   P31137
HRIFA025488a
   PROCOLLAGEN ALPHA 1(III) CHAIN PRECURSOR (FRAGMENTS).
   9.5e-05:104:40
   MUS MUSCULUS (MOUSE).
   P08121
HRIFA025492a
   SERINE/THREONINE-SPECIFIC PROTEIN KINASE MINIBRAIN HOMOLOG (EC 2.7.1.-) (HP86) (DYRK).
   1.8e-53:159:69
   HOMO SAPIENS (HUMAN).
   Q13627
HRIFA025636a
   MITOCHONDRIAL RESPIRATORY CHAIN COMPLEXES ASSEMBLY PROTEIN RCA1 (EC 3.4.24.-) (TAT-BINDING HOMOLOG 12).
   4.7e-32:81:66
   SACCHAROMYCES CEREVISIAE (BAKER'S YEAST).
   P40341
HRIFA025695a
   PEREGRIN (BR140 PROTEIN).
   3.1e-40:227:43
   HOMO SAPIENS (HUMAN).
   P55201
HRIFA025703a
   CELL SURFACE ANTIGEN 114/A10 PRECURSOR.
   1.8e-08:71:42
   MUS MUSCULUS (MOUSE).
   P19467
HRIFA025706a
   GLYCOSYLTRANSFERASE ALG2 (EC 2.4.1.-).
   1.2e-28:111:44
   SACCHAROMYCES CEREVISIAE (BAKER'S YEAST).
   P43636
HRIFA025766a
   CYTOCHROME B5.
   4.2e-13:133:33
   ORYCTOLAGUS CUNICULUS (RABBIT).
   P00169
HRIFA025771a
   HYPOTHETICAL 22.2 KD PROTEIN IN NSR1-TIF4631 INTERGENIC REGION.
   6.7e-10:129:31
   SACCHAROMYCES CEREVISIAE (BAKER'S YEAST).
   P53288
HRIFA025778a
   A-AGGLUTININ ATTACHMENT SUBUNIT PRECURSOR.
   1.5e-05:212:30
   SACCHAROMYCES CEREVISIAE (BAKER'S YEAST).
   P32323
HRIFA025800a
   HYPOTHETICAL 37.4 KD PROTEIN IN IRR1-TIM44 INTERGENIC REGION.
   3.7e-18:165:33
   SACCHAROMYCES CEREVISIAE (BAKER'S YEAST).
   P40544
HRIFA025904a
   COMPLEMENT RECEPTOR TYPE 1 PRECURSOR (C3B/C4B RECEPTOR) (CD35 ANTIGEN).
   2.6e-05:211:28
   HOMO SAPIENS (HUMAN).
   P17927
HRIFA025907a
   INTERFERON GAMMA UP-REGULATED I-5111 PROTEIN PRECURSOR (IGUPI-5111).
   2.1e-38:176:38
   HOMO SAPIENS (HUMAN).
   Q06323
HRIFA025913a
   DOLICHYL-PHOSPHATE-MANNOSE--PROTEIN MANNOSYLTRANSFERASE 4 (EC 2.4.1.109).
   2.5e-32:185:37
   SACCHAROMYCES CEREVISIAE (BAKER'S YEAST).
   P46971
HRIFA025936a
   TRANSCRIPTIONAL ACTIVATOR FE65.
   3.4e-09:43:46
   RATTUS NORVEGICUS (RAT).
   P46933
HRIFA025966a
   SYNAPTOTAGMIN III.
   4.5e-05:93:33
   RATTUS NORVEGICUS (RAT).
   P40748
HRIFA025978a
   "GLUTENIN, HIGH MOLECULAR WEIGHT SUBUNIT DX5 PRECURSOR."
   3.5e-06:224:28
   TRITICUM AESTIVUM (WHEAT).
   P10388
HRIFA026089a
   BUTYROPHILIN PRECURSOR (BT).
   1.1e-12:146:29
   BOS TAURUS (BOVINE).
   P18892
HRIFA026121a
   FAS ANTIGEN LIGAND (APOPTOSIS ANTIGEN LIGAND) (APTL).
   9.7e-06:72:43
   HOMO SAPIENS (HUMAN).
   P48023
HRIFA026242a
   HYPOTHETICAL 73.0 KD PROTEIN IN CLA4-MID1 INTERGENIC REGION.
   7.4e-09:188:26
   SACCHAROMYCES CEREVISIAE (BAKER'S YEAST).
   P48566
HRIFA026265a
   DNA BINDING PROTEIN S1FA.
   0.67:43:37
   ARABIDOPSIS THALIANA (MOUSE-EAR CRESS).
   P42551
HRIFA026303a
   SALIVARY PROLINE-RICH PROTEIN PO PRECURSOR (ALLELE S).
   0.014:88:32
   HOMO SAPIENS (HUMAN).
   P10163
HRIFA026316a
   EBNA-2 NUCLEAR PROTEIN.
   1.5e-07:82:35
   EPSTEIN-BARR VIRUS (STRAIN B95-8) (HUMAN HERPESVIRUS 4).
   P12978
HRIFA026351a
   FLI-1 ONCOGENE (ERGB TRANSCRIPTION FACTOR).
   0.019:89:31
   HOMO SAPIENS (HUMAN).
   Q01543
HRIFA026364a
   PROBABLE G PROTEIN-COUPLED RECEPTOR EDG-1.
   8.3e-40:167:49
   RATTUS NORVEGICUS (RAT).
   P48303
HRIFA026382a
   T-CELL RECEPTOR BETA CHAIN PRECURSOR (ANA 11).
   6.2e-10:135:38
   ORYCTOLAGUS CUNICULUS (RABBIT).
   P06333
HRIFA026465a
   COLLAGEN ALPHA 1(IX) CHAIN PRECURSOR (FRAGMENTS).
   8.6e-07:158:35
   GALLUS GALLUS (CHICKEN).
   P12106
HRIFA026496a
   ZINC FINGER PROTEIN 140.
   5.9e-24:122:52
   HOMO SAPIENS (HUMAN).
   P52738
HRIFA026519a
   PROLINE-RICH PROTEIN MP-2 PRECURSOR.
   1.3e-08:130:36
   MUS MUSCULUS (MOUSE).
   P05142
HRIFA026564a
   GLYCOPROTEIN X PRECURSOR
   1.8e-10:225:25
   EQUINE HERPESVIRUS TYPE 1 (STRAIN AB4P) (EHV-1).
   P28968
HRIFA026576a
   "ADP,ATP CARRIER PROTEIN, HEART/SKELETAL MUSCLE ISOFORM T1 (ADP/ATP TRANSLOCASE 1) (ADENINE NUCLEOTIDE TRANSLOCATOR 1) (ANT 1)."
   1.7e-09:116:34
   HOMO SAPIENS (HUMAN).
   P12235
HRIFA026615a
   REGULATORY PROTEIN E2.
   0.0024:132:31
   HUMAN PAPILLOMAVIRUS TYPE 9.
   P36780
HRIFA026618a
   PROTEIN Q300.
   1.2e-05:27:66
   MUS MUSCULUS (MOUSE).
   Q02722
HRIFA026659a
   SERINE/THREONINE-PROTEIN KINASE SGK (EC 2.7.1.-) (SERUM/GLUCOCORTICOID-REGULATED KINASE).
   2.0e-10:81:45
   RATTUS NORVEGICUS (RAT).
   Q06226
HRIFA026764a
   MRC OX-45 SURFACE ANTIGEN PRECURSOR (BCM1 SURFACE ANTIGEN) (BLAST-1) (CD48).
   3.4e-05:162:25
   RATTUS NORVEGICUS (RAT).
   P10252
HRIFA026789a
   PUTATIVE GENERAL NEGATIVE REGULATOR OF TRANSCRIPTION C16C9.04C.
   8.1e-22:175:38
   SCHIZOSACCHAROMYCES POMBE (FISSION YEAST).
   Q09818
HRIFA026813a
   "PROTEIN KINASE C, MU TYPE (EC 2.7.1.-) (NPKC-MU)."
   7.1e-89:256:67
   HOMO SAPIENS (HUMAN).
   Q15139
HRIFA026860a
   MONOCARBOXYLATE TRANSPORTER 2 (MCT 2).
   2.6e-19:103:43
   MESOCRICETUS AURATUS (GOLDEN HAMSTER).
   P53988
HRIFA026923a
   CCAAT DISPLACEMENT PROTEIN (HOMEOBOX PROTEIN CLOX) (CLOX-1) (FRAGMENT).
   0.18:119:36
   CANIS FAMILIARIS (DOG).
   P39881
HRIFA027012a
   "MANNOSYL-OLIGOSACCHARIDE ALPHA-1,2-MANNOSIDASE (EC 3.2.1.113) (MAN(9)-ALPHA-MANNOSIDASE) (ALPHA-MANNOSIDASE 1A)."
   1.8e-44:234:41
   MUS MUSCULUS (MOUSE).
   P45700
HRIFA027045a
   HYPOTHETICAL PROTEIN HI0519.
   2.7e-27:181:38
   HAEMOPHILUS INFLUENZAE.
   P44742
HRIFA027125a
   ZINC FINGER PROTEIN 133.
   3.9e-33:70:61
   HOMO SAPIENS (HUMAN).
   P52736
HRIFA027173a
   TRANS-ACTING TRANSCRIPTIONAL PROTEIN ICP0 (VMW118 PROTEIN).
   0.15:137:27
   HERPES SIMPLEX VIRUS (TYPE 2 / STRAIN HG52).
   P28284
HRIFA027179a
   EXCISION REPAIR PROTEIN ERCC-6 (COCKAYNE SYNDROME PROTEIN CSB).
   3.6e-30:90:77
   HOMO SAPIENS (HUMAN).
   Q03468
HRIFA027187a
   B-CELL GROWTH FACTOR PRECURSOR (BCGF-12 KD).
   4.7e-11:44:61
   HOMO SAPIENS (HUMAN).
   P20931
HRIFA027327a
   COLLAGEN ALPHA 1(X) CHAIN PRECURSOR.
   3.8e-07:184:35
   HOMO SAPIENS (HUMAN).
   Q03692
HRIFA027329a
   SALIVARY GLUE PROTEIN SGS-3 PRECURSOR.
   9.1e-08:195:29
   DROSOPHILA ERECTA (FRUIT FLY).
   P13730
HRIFA027355a
   B-CELL GROWTH FACTOR PRECURSOR (BCGF-12 KD).
   1.9e-06:33:72
   HOMO SAPIENS (HUMAN).
   P20931
HRIFA027485a
   COLLAGEN ALPHA 1(XI) CHAIN PRECURSOR.
   0.00099:174:36
   HOMO SAPIENS (HUMAN).
   P12107
HRIFA027536a
   VITELLINE MEMBRANE PROTEIN VM26AB PRECURSOR (PROTEIN TU-4) (PROTEIN SV23).
   0.0042:104:35
   DROSOPHILA MELANOGASTER (FRUIT FLY).
   P13238
HRIFA027549a
   D(4) DOPAMINE RECEPTOR (D(2C) DOPAMINE RECEPTOR).
   0.00023:101:44
   HOMO SAPIENS (HUMAN).
   P21917
HRIFA027622a
   GUANOSINE-DIPHOSPHATASE (EC 3.6.1.42) (GDPASE).
   2.2e-23:146:45
   SACCHAROMYCES CEREVISIAE (BAKER'S YEAST).
   P32621
HRIFA027625a
   CALCIUM-TRANSPORTING ATPASE 1 (EC 3.6.1.38) (GOLGI CA2+-ATPASE).
   1.1e-57:220:54
   SACCHAROMYCES CEREVISIAE (BAKER'S YEAST).
   P13586
HRIFA027644a
   COLLAGEN ALPHA 1(I) CHAIN (FRAGMENTS).
   7.5e-05:72:40
   RATTUS NORVEGICUS (RAT).
   P02454
HRIFA027656a
   NON-RECEPTOR TYROSINE KINASE SPORE LYSIS A (EC 2.7.1.112) (TYROSINE-PROTEIN KINASE 1).
   1.6e-13:149:34
   DICTYOSTELIUM DISCOIDEUM (SLIME MOLD).
   P18160
HRIFA027673a
   CONNECTIVE TISSUE GROWTH FACTOR PRECURSOR.
   6.4e-06:47:57
   HOMO SAPIENS (HUMAN).
   P29279
HRIFA027681a
   SPORULATION-SPECIFIC PROTEIN 1 (EC 2.7.1.-).
   1.1e-13:158:31
   SACCHAROMYCES CEREVISIAE (BAKER'S YEAST).
   P08458
HRIFA027714a
   HYPOTHETICAL 146.8 KD PROTEIN C34E10.5 IN CHROMOSOME III.
   7.2e-06:146:30
   CAENORHABDITIS ELEGANS.
   P46580
HRIFA027722a
   SIGNAL RECOGNITION PARTICLE 68 KD PROTEIN (SRP68).
   2.7e-105:242:85
   CANIS FAMILIARIS (DOG).
   Q00004
HRIFA027860a
   SKIN SECRETORY PROTEIN XP2 PRECURSOR (APEG PROTEIN).
   8.3e-08:168:32
   XENOPUS LAEVIS (AFRICAN CLAWED FROG).
   P17437
HRIFA027867a
   STANNIOCALCIN PRECURSOR (STC) (CORPUSCLES OF STANNIUS PROTEIN) (CS) (HYPOCALCIN) (TELEOCALCIN).
   1.0:100:27
   ANGUILLA AUSTRALIS (AUSTRALIAN EEL).
   P18301
HRIFA027940a
   INHIBIN BETA C CHAIN PRECURSOR (ACTIVIN BETA-C CHAIN).
   8.7e-15:149:38
   HOMO SAPIENS (HUMAN).
   P55103
HRIFA028061a
   PUTATIVE SERINE/THREONINE-PROTEIN KINASE PKWA (EC 2.7.1.-).
   9.7e-07:157:26
   THERMOMONOSPORA CURVATA.
   P49695
HRIFA028157a
   HIGH-AFFINITY CATIONIC AMINO ACID TRANSPORTER-1 (CAT-1) (CAT1) (SYSTEM Y+ BASIC AMINO ACID TRANSPORTER) (ECOTROPIC RETROVIRAL LEUKEMIA RECEPTOR HOMOLOG) (ERR) (ECOTROPIC RETROVIRUS RECEPTOR HOMOLOG).
   2.8e-71:201:68
   HOMO SAPIENS (HUMAN).
   P30825
HRIFA028187a
   EBNA-1 NUCLEAR PROTEIN.
   1.5e-09:131:38
   EPSTEIN-BARR VIRUS (STRAIN B95-8) (HUMAN HERPESVIRUS 4).
   P03211
HRIFA028262a
   CELLULAR NUCLEIC ACID BINDING PROTEIN (CNBP).
   7.2e-09:99:33
   MUS MUSCULUS (MOUSE).
   P53996
HRIFA028371a
   PLASMINOGEN (EC 3.4.21.7) (FRAGMENT).
   1.0e-08:103:33
   RATTUS NORVEGICUS (RAT).
   Q01177
HRIFA028402a
   PUTATIVE SERINE/THREONINE-PROTEIN KINASE PKWA (EC 2.7.1.-).
   3.2e-33:204:39
   THERMOMONOSPORA CURVATA.
   P49695
HRIFA028440a
   COLLAGEN ALPHA 4(IV) CHAIN PRECURSOR.
   1.9e-07:192:36
   HOMO SAPIENS (HUMAN).
   P53420
HRIFA028468a
   CALCIUM/CALMODULIN-DEPENDENT PROTEIN KINASE TYPE IV CATALYTIC CHAIN (EC 2.7.1.123) (CAM KINASE-GR) (CAMK IV) [CONTAINS: CALSPERMIN].
   5.8e-32:178:44
   RATTUS NORVEGICUS (RAT).
   P13234
HRIFA028501a
   VOLTAGE-GATED POTASSIUM CHANNEL PROTEIN KV1.6 (RCK2) (KV2).
   6.3e-05:161:31
   RATTUS NORVEGICUS (RAT).
   P17659
HRIFA028511a
   ANKYRIN HOMOLOG PRECURSOR.
   3.0e-19:176:34
   CHROMATIUM VINOSUM.
   Q06527
HRIFA028576a
   ACROSIN PRECURSOR (EC 3.4.21.10).
   4.8e-08:78:46
   ORYCTOLAGUS CUNICULUS (RABBIT).
   P48038
HRIFA028614a
   HISTIDINE-RICH GLYCOPROTEIN PRECURSOR.
   1.0e-08:82:39
   PLASMODIUM LOPHURAE.
   P04929
HRIFA028651a
   BAND 3 ANION TRANSPORT PROTEIN.
   1.3e-18:156:32
   GALLUS GALLUS (CHICKEN).
   P15575
HRIFA028790a
   PROBABLE CALCIUM-TRANSPORTING ATPASE 6 (EC 3.6.1.38).
   5.0e-18:212:29
   SACCHAROMYCES CEREVISIAE (BAKER'S YEAST).
   P39986
HRIFA028804a
   CCAAT-BINDING FACTOR (CBF).
   0.98:232:23
   MUS MUSCULUS (MOUSE).
   P53569
HRIFA028867a
   REGULATORY PROTEIN E2.
   0.0057:124:31
   HUMAN PAPILLOMAVIRUS TYPE 25.
   P36787
HRIFA028911a
   HYPOTHETICAL 118.4 KD PROTEIN IN BAT2-DAL5 INTERGENIC REGION PRECURSOR.
   1.2e-09:206:33
   SACCHAROMYCES CEREVISIAE (BAKER'S YEAST).
   P47179
HRIFA028983a
   HOMEOTIC GENE REGULATOR (BRAHMA PROTEIN).
   0.0051:115:33
   DROSOPHILA MELANOGASTER (FRUIT FLY).
   P25439
HRIFA029002a
   FIBRINOGEN BETA CHAIN.
   3.2e-25:121:45
   BOS TAURUS (BOVINE).
   P02676
HRIFA029050a
   RETINAL-CADHERIN PRECURSOR (R-CADHERIN) (R-CAD).
   1.2e-10:134:32
   GALLUS GALLUS (CHICKEN).
   P24503
HRIFA029208a
   RENAL TRANSCRIPTION FACTOR KID-1 (TRANSCRIPTION FACTOR 17).
   1.4e-14:64:59
   RATTUS NORVEGICUS (RAT).
   Q02975
HRIFA029209a
   "ALPHA-MANNOSIDASE II (EC 3.2.1.114) (MANNOSYL-OLIGOSACCHARIDE 1,3-1,6-ALPHA-MANNOSIDASE) (MAN II) (GOLGI ALPHA-MANNOSIDASE II)."
   2.3e-12:114:37
   MUS MUSCULUS (MOUSE).
   P27046
HRIFA029256a
   GAP JUNCTION BETA-2 PROTEIN (CONNEXIN 26) (CX26).
   1.8e-35:89:75
   HOMO SAPIENS (HUMAN).
   P29033
HRIFA029263a
   SARCALUMENIN PRECURSOR.
   2.1e-16:161:31
   ORYCTOLAGUS CUNICULUS (RABBIT).
   P13666
HRIFA029278a
   "SALIVARY PROLINE-RICH PROTEIN PRECURSOR (CLONES CP3, CP4 AND CP5) [CONTAINS: BASIC PEPTIDE IB-6" PEPTIDE P-H].
   3.5e-10:204:32
   HOMO SAPIENS (HUMAN).
   P04280
HRIFA029285a
   GLYCOPROTEIN 25L PRECURSOR (GP25L).
   4.9e-58:197:55
   CANIS FAMILIARIS (DOG).
   P27869
HRIFA029317a
   HIGH AFFINITY SULPHATE TRANSPORTER 2.
   2.3e-25:83:50
   STYLOSANTHES HAMATA.
   P53392
HRIFA029327a
   MITOCHONDRIAL 2-OXOGLUTARATE/MALATE CARRIER PROTEIN (OGCP).
   9.1e-34:227:37
   BOS TAURUS (BOVINE).
   P22292
HRIFA029349a
   CUTICLE COLLAGEN 12 PRECURSOR.
   5.1e-09:190:33
   CAENORHABDITIS ELEGANS.
   P20630
HRIFA029393a
   PROBABLE G PROTEIN-COUPLED RECEPTOR APJ.
   9.7e-69:165:84
   HOMO SAPIENS (HUMAN).
   P35414
HRIFA029398a
   TRANS-ACTING TRANSCRIPTIONAL PROTEIN ICP0 (P135 PROTEIN) (IER 2.9/ER2.6).
   0.011:170:34
   BOVINE HERPESVIRUS TYPE 1 (STRAIN JURA).
   P29128
HRIFA029425a
   ALPHA CRYSTALLIN B CHAIN (ALPHA(B)-CRYSTALLIN).
   2.0e-08:99:32
   BOS TAURUS (BOVINE).
   P02510
HRIFA029434a
   "SALIVARY PROLINE-RICH PROTEIN PRECURSOR (CLONES CP3, CP4 AND CPS) [CONTAINS: BASIC PEPTIDE IB-6" PEPTIDE P-H].
   2.6e-05:232:32
   HOMO SAPIENS (HUMAN).
   P04280
HRIFA029440a
   SKIN SECRETORY PROTEIN XP2 PRECURSOR (APEG PROTEIN).
   0.00046:131:33
   XENOPUS LAEVIS (AFRICAN CLAWED FROG).
   P17437
HRIFA029460a
   SYNAPTOTAGMIN III.
   1.5e-08:102:35
   RATTUS NORVEGICUS (RAT).
   P40748
HRIFA029467a
   GLYCOPROTEIN X PRECURSOR.
   5.2e-07:182:31
   EQUINE HERPESVIRUS TYPE 1 (STRAIN AB4P) (EHV-1).
   P28968
HRIFA029508a
   PROPERDIN PRECURSOR.
   1.9e-06:218:32
   HOMO SAPIENS (HUMAN).
   P27918
HRIFA029511a
   POTASSIUM CHANNEL PROTEIN EAG.
   2.3e-66:139:61
   DROSOPHILA MELANOGASTER (FRUIT FLY).
   Q02280
HRIFA029602a
   SEX-DETERMINING REGION Y PROTEIN (TESTIS-DETERMINING FACTOR).
   1.0:37:37
   SUS SCROFA (PIG).
   P36393
HRIFA029649a
   TRANS-ACTING TRANSCRIPTIONAL PROTEIN ICP0 (VMW118 PROTEIN).
   0.30:99:34
   HERPES SIMPLEX VIRUS (TYPE 2 / STRAIN HG52).
   P28284
HRIFA029715a
   GROWTH ARREST AND DNA-DAMAGE-INDUCIBLE PROTEIN GADD153 (DNA-DAMAGE INDUCIBLE TRANSCRIPT 3) (DDIT3) (C/EBP-HOMOLOGOUS PROTEIN) (CHOP).
   0.54:95:30
   HOMO SAPIENS (HUMAN).
   P35638
HRIFA029730a
   HISTIDINE-RICH GLYCOPROTEIN PRECURSOR.
   3.8e-05:131:29
   PLASMODIUM LOPHURAE.
   P04929
HRIFA029792a
   PUTATIVE SERINE/THREONINE-PROTEIN KINASE PKWA (EC 2.7.1.-).
   9.0e-09:178:30
   THERMOMONOSPORA CURVATA.
   P49695
HRIFA029802a
   TRAM PROTEIN (TRANSLOCATING CHAIN-ASSOCIATING MEMBRANE PROTEIN).
   7.2e-73:204:69
   CANIS FAMILIARIS (DOG).
   Q01685
HRIFA029866a
   PROTEIN KINASE BYR2 (EC 2.7.1.-) (PROTEIN KINASE STE8) (MAPK KINASE KINASE) (MAPKKK).
   1.2e-27:144:45
   SCHIZOSACCHAROMYCES POMBE (FISSION YEAST).
   P28829
HRIFA029932a
   F-SPONDIN PRECURSOR.
   9.1e-24:191:37
   XENOPUS LAEVIS (AFRICAN CLAWED FROG).
   P35447
HRIFA030025a
   ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
   1.0e-11:138:31
   SACCHAROMYCES CEREVISIAE (BAKER'S YEAST).
   P32802
HRIFA030045a
   SARCALUMENIN PRECURSOR.
   2.4e-20:151:32
   ORYCTOLAGUS CUNICULUS (RABBIT).
   P13666
HRIFA030103a
   HYPOTHETICAL 57.5 KD PROTEIN IN VMA7-RPS25A INTERGENIC REGION.
   2.1e-05:215:29
   SACCHAROMYCES CEREVISIAE (BAKER'S YEAST).
   P53214
HRIFA030106a
   SCO-SPONDIN (FRAGMENT).
   0.53:60:36
   BOS TAURUS (BOVINE).
   P98167
HRIFA030147a
   PUTATIVE MITOCHONDRIAL CARRIER YGR096W.
   1.8e-10:93:34
   SACCHAROMYCES CEREVISIAE (BAKER'S YEAST).
   P53257
HRIFA030250a
   ENAMELIN (TUFTELIN).
   3.7e-108:250:86
   BOS TAURUS (BOVINE).
   P27628
HRIFA030264a
   SODIUM/GLUCOSE COTRANSPORTER 1 (NA(+)/GLUCOSE COTRANSPORTER 1) (HIGH AFFINITY SODIUM-GLUCOSE COTRANSPORTER).
   3.3e-09:119:27
   ORYCTOLAGUS CUNICULUS (RABBIT).
   P11170
HRIFA030342a
   ESTRADIOL 17 BETA-DEHYDROGENASE 3 (EC 1.1.1.62) (17-BETA-HSD 3) (TESTICULAR 17-BETA-HYDROXYSTEROID DEHYDROGENASE).
   1.5e-42:203:49
   HOMO SAPIENS (HUMAN).
   P37058
HRIFA030370a
   HYPOTHETICAL 56.2 KD PROTEIN CY31.25C.
   8.0e-12:88:48
   MYCOBACTERIUM TUBERCULOSIS.
   Q10555
HRIFA030371a
   "PROTEIN KINASE C, MU TYPE (EC 2.7.1.-) (NPKC-MU)."
   1.6e-68:228:59
   HOMO SAPIENS (HUMAN).
   Q15139
HRIFA030381a
   COLLAGEN 1(X) CHAIN PRECURSOR.
   3.0e-05:204:30
   GALLUS GALLUS (CHICKEN).
   P08125
HRIFA030385a
   COLLAGEN ALPHA 1(X) CHAIN PRECURSOR.
   0.029:162:31
   HOMO SAPIENS (HUMAN).
   Q03692
HRIFA030411a
   SERINE PALMITOYLTRANSFERASE 2 (EC 2.3.1.50) (LONG CHAIN BASE BIOSYNTHESIS PROTEIN 2) (SPT 2).
   1.2e-27:115:53
   SCHIZOSACCHAROMYCES POMBE (FISSION YEAST).
   Q09925
HRIFA030448a
   PUTATIVE SERINE/THREONINE-PROTEIN KINASE P78 (EC 2.7.1.-).
   2.5e-92:225:77
   HOMO SAPIENS (HUMAN).
   P27448
HRIFA030456a
   PROLINE-RICH PROTEIN MP-2 PRECURSOR.
   9.3e-08:127:35
   MUS MUSCULUS (MOUSE).
   P05142
HRIFA030461a
   CUTICLE COLLAGEN 12 PRECURSOR.
   0.046:140:31
   CAENORHABDITIS ELEGANS.
   P20630
HRIFA030472a
   NUC-1 NEGATIVE REGULATORY PROTEIN PREG.
   0.0030:98:31
   NEUROSPORA CRASSA.
   Q06712
HRIFA030509a
   "INTERFERON-INDUCED, DOUBLE-STRANDED RNA-ACTIVATED PROTEIN KINASE (EC 2.7.1.-) (INTERFERON-INDUCIBLE RNA-DEPENDENT PROTEIN KINASE) (P68 KINASE) (P1/EIF-2A PROTEIN KINASE)."
   2.5e-09:65:43
   HOMO SAPIENS (HUMAN).
   P19525
HRIFA030511a
   T-LYMPHOCYTE MATURATION-ASSOCIATED PROTEIN.
   0.00010:99:33
   HOMO SAPIENS (HUMAN).
   P21145
HRIFA030545a
   PROBABLE SERINE/THREONINE-PROTEIN KINASE YNL020C (EC 2.7.1.-).
   7.6e-21:165:35
   SACCHAROMYCES CEREVISIAE (BAKER'S YEAST).
   P53974
HRIFA030566a
   "GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE)."
   2.7e-07:221:30
   SACCHAROMYCES CEREVISIAE (BAKER'S YEAST).
   P08640
HRIFA030599a
   GLYCOPROTEIN X PRECURSOR.
   2.8e-05:236:27
   EQUINE HERPESVIRUS TYPE 1 (STRAIN AB4P) (EHV-1).
   P28968
HRIFA030629a
   PROTEIN DISULFIDE ISOMERASE PRECURSOR (PDI) (EC 5.3.4.1) (PROLYL 4-HYDROXYLASE BETA SUBUNIT) (CELLULAR THYROID HORMONE BINDING PROTEIN) (P55).
   3.5e-16:115:38
   BOS TAURUS (BOVINE).
   P05307
HRIFA030642a
   SULFATED SURFACE GLYCOPROTEIN 185 (SSG 185).
   2.5e-12:93:47
   VOLVOX CARTERI.
   P21997
HRIFA030662a
   NADH-UBIQUINONE OXIDOREDUCTASE CHAIN 1 (EC 1.6.5.3).
   9.1e-120:279:83
   HOMO SAPIENS (HUMAN).
   P03886
HRIFA030839a
   HYPOTHETICAL GENE 51 MEMBRANE PROTEIN.
   1.0:66:27
   ICTALURID HERPESVIRUS 1 (CHANNEL CATFISH VIRUS) (CCV).
   Q00135
HRIFA031091a
   PROTEIN Q300.
   0.0042:27:62
   MUS MUSCULUS (MOUSE).
   Q02722
HRIFA031126a
   P2Y PURINOCEPTOR 5 (P2Y5) (PURINERGIC RECEPTOR 5) (RB INTRON ENCODED G-PROTEIN COUPLED RECEPTOR).
   1.3e-06:70:34
   HOMO SAPIENS (HUMAN).
   P43657
HRIFA031249a
   ACIDIC PROLINE-RICH PROTEIN PRECURSOR (CLONE PRP33).
   5.9e-05:166:31
   RATTUS NORVEGICUS (RAT).
   P04474
HRIFA031336a
   CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).
   6.6e-15:97:38
   PETROMYZON MARINUS (SEA LAMPREY).
   P25210
HRIFA031395a
   COLD SHOCK PROTEIN CSPB (FRAGMENT).
   0.95:32:40
   BACILLUS GLOBISPORUS.
   P41018
HRIFA031397a
   REGULATORY PROTEIN E2.
   0.0077:145:35
   HUMAN PAPILLOMAVIRUS TYPE 47.
   P22420
HRIFA031438a
   GLUCOSE REPRESSION MEDIATOR PROTEIN.
   1.3e-06:176:26
   SACCHAROMYCES CEREVISIAE (BAKER'S YEAST).
   P14922
HRIFA031869a
   TRANSCRIPTION FACTOR HES-1 (HAIRY AND ENHANCER OF SPLIT 1) (HAIRY-LIKE) (RHL).
   1.7e-18:163:41
   RATTUS NORVEGICUS (RAT).
   Q04666
HRIFA031935a
   EXTENSIN PRECURSOR (PROLINE-RICH GLYCOPROTEIN).
   1.8e-06:192:32
   ZEA MAYS (MAIZE).
   P14918
HRIFA031986a
   SERINE/THREONINE-PROTEIN KINASE PAK-ALPHA (EC 2.7.1.-) (P68-PAK) (P21-ACTIVATED KINASE) (ALPHA-PAK) (PROTEIN KINASE MUK2).
   2.4e-49:222:47
   RATTUS NORVEGICUS (RAT).
   P35465
HRIFA032009a
   PROBABLE G PROTEIN-COUPLED RECEPTOR FROM T-CELLS PRECURSOR (GLUCOCORTICOID-INDUCED RECEPTOR).
   1.0e-17:118:36
   MUS MUSCULUS (MOUSE).
   P30731
HRIFA032011a
   MUSCARINIC ACETYLCHOLINE RECEPTOR M4.
   7.8e-35:184:32
   HOMO SAPIENS (HUMAN).
   P08173
HRIFA032070a
   MITOCHONDRIAL RNA SPLICING PROTEIN MSR4.
   2.1e-18:107:44
   SACCHAROMYCES CEREVISIAE (BAKER'S YEAST).
   P23500
HRIFA032073a
   SECRETOGRANIN III PRECURSOR (SGIII).
   9.7e-69:182:76
   MUS MUSCULUS (MOUSE).
   P47867
HRIFA032079a
   HYPOTHETICAL 49.3 KD PROTEIN C30D11.06C IN CHROMOSOME I.
   3.5e-12:96:39
   SCHIZOSACCHAROMYCES POMBE (FISSION YEAST).
   Q09906
HRIFA032097a
   GLYCOPROTEIN J.
   0.023:61:32
   HERPES SIMPLEX VIRUS (TYPE 1 / STRAIN 17).
   P06480
HRIFA032161a
   CCAAT/ENHANCER BINDING PROTEIN DELTA (C/EBP DELTA) (NUCLEAR FACTOR NF-IL6-BETA) (NF-IL6-BETA).
   0.22:56:42
   HOMO SAPIENS (HUMAN).
   P49716
HRIFA032186a
   D-BINDING PROTEIN (DBP) (ALBUMIN D BOX-BINDING PROTEIN) (TAXREB302).
   0.86:50:38
   HOMO SAPIENS (HUMAN).
   Q10586
HRIFA032224a
   HYPOTHETICAL 52.7 KD PROTEIN C38C10.2 IN CHROMOSOME III.
   2.6e-43:196:45
   CAENORHABDITIS ELEGANS.
   Q03567
HRIFA032257a
   GLUCOSE REPRESSION MEDIATOR PROTEIN.
   4.7e-07:204:25
   SACCHAROMYCES CEREVISIAE (BAKER'S YEAST).
   P14922
HRIFA032274a
   ZINC FINGER PROTEIN 38 (ZFP-38) (CTFIN51) (TRANSCRIPTION FACTOR RU49).
   7.8e-60:163:74
   MUS MUSCULUS (MOUSE).
   Q07231
HRIFA032275a
   CELL DIVISION CONTROL PROTEIN 28 (EC 2.7.1.-).
   7.2e-41:179:38
   SACCHAROMYCES CEREVISIAE (BAKER'S YEAST).
   P00546
HRIFA032360a
   HYPOTHETICAL 84.3 KD PROTEIN ZK945.10 IN CHROMOSOME II.
   3.0e-05:198:28
   CAENORHABDITIS ELEGANS.
   Q09625
HRIFA032389a
   EBNA-1 NUCLEAR PROTEIN.
   1.3e-05:86:39
   EPSTEIN-BARR VIRUS (STRAIN B95-8) (HUMAN HERPESVIRUS 4). P03211
HRIFA032433a
   GONADOTROPIN-RELEASING HORMONE RECEPTOR (GNRH-R).
   3.1e-14:54:53
   RATTUS NORVEGICUS (RAT).
   P30969
HRIFA032453a
   BUTYROPHILIN PRECURSOR (BT).
   5.9e-13:162:32
   BOS TAURUS (BOVINE).
   P18892
HRIFA032478a
   GLYCOPROTEIN X PRECURSOR.
   3.8e-06:253:28
   EQUINE HERPESVIRUS TYPE 1 (STRAIN AB4P) (EHV-1).
   P28968
HRIFA032506a
   COLLAGEN ALPHA 3(VI) CHAIN PRECURSOR.
   1.2e-06:226:34
   HOMO SAPIENS (HUMAN).
   P12111
HRIFA032511a
   COLLAGEN ALPHA 1(XVI) CHAIN PRECURSOR.
   8.7e-09:229:34
   HOMO SAPIENS (HUMAN).
   Q07092
HRIFA032530a
   SKIN SECRETORY PROTEIN XP2 PRECURSOR (APEG PROTEIN).
   9.0e-05:159:33
   XENOPUS LAEVIS (AFRICAN CLAWED FROG).
   P17437
HRIFA032587a
   SYNAPTOTAGMIN (P65).
   3.2e-08:72:52
   APLYSIA CALIFORNICA (CALIFORNIA SEA HARE).
   P41823
HRIFA032605a
   ANTIGEN PEPTIDE TRANSPORTER 1 (APT1) (PEPTIDE TRANSPORTER TAP1) (PEPTIDE TRANSPORTER PSF1) (PEPTIDE SUPPLY FACTOR 1) (PSF-1) (PEPTIDE TRANSPORTER INVOLVED IN ANTIGEN PROCESSING 1).
   8.4e-37:192:41
   HOMO SAPIENS (HUMAN).
   Q03518
HRIFA032642a
   PROLINE-RICH PROTEIN MP-2 PRECURSOR.
   5.0e-05:127:33
   MUS MUSCULUS (MOUSE).
   P05142
HRIFA032696a
   COLLAGEN ALPHA 1(II) CHAIN (FRAGMENTS).
   1.4e-13:200:38
   BOS TAURUS (BOVINE).
   P02459
HRIFA032730a
   K-GLYPICAN PRECURSOR.
   4.8e-67:180:68
   MUS MUSCULUS (MOUSE).
   P51655
HRIFA032820a
   GLUTAMIC ACID-RICH PROTEIN PRECURSOR.
   7.5e-05:192:23
   PLASMODIUM FALCIPARUM (ISOLATE FC27 / PAPUA NEW GUINEA).
   P13816

### Homology search result 2

Homology of representative sequences of the 5'-end cluster to the data in Swiss-Prot database

Representative sequence of the 5'-end cluster exhibiting relatively high homology (304 cluster: "exhibiting relatively high homology" means that the P value is 10⁻¹⁰ or less)
HRIFA000327a, HRIFA000432a, HRIFA000553a, HRIFA000564a, HRIFA000631a, HRIFA000683a, HRIFA000776a, HRIFA000814a, HRIFA001132a, HRIFA001138a, HRIFA001337a, HRIFA001341a, HRIFA001489a, HRIFA001712a, HRIFA001720a, HRIFA001942a, HRIFA001975a, HRIFA001984a, HRIFA002384a, HRIFA002503a, HRIFA002743a, HRIFA002766a, HRIFA002805a, HRIFA002891a, HRIFA002919a, HRIFA002980a, HRIFA003063a, HRIFA003093a, HRIFA003635a, HRIFA004006a, HRIFA004034a, HRIFA004112a, HRIFA004426a, HRIFA004490a, HRIFA004523a, HRIFA004663a, HRIFA004696a, HRIFA004714a, HRIFA004745a, HRIFA004919a, HRIFA005184a, HRIFA005231a, HRIFA005240a, HRIFA005271a, HRIFA005372a, HRIFA005392a, HRIFA005409a, HRIFA005420a, HRIFA005438a, HRIFA005462a, HRIFA005644a, HRIFA005720a, HRIFA005732a, HRIFA005760a, HRIFA005781a, HRIFA006183a, HRIFA006494a, HRIFA006510a, HRIFA006566a, HRIFA006586a, HRIFA006596a, HRIFA006649a, HRIFA006667a, HRIFA006730a, HRIFA006926a, HRIFA007013a, HRIFA007219a, HRIFA007228a, HRIFA007243a, HRIFA007352a, HRIFA007424a, HRIFA007435a, HRIFA007463a, HRIFA007493a, HRIFA007571a, HRIFA007659a, HRIFA007722a, HRIFA007745a, HRIFA008000a, HRIFA008200a, HRIFA008284a, HRIFA008314a, HRIFA008362a, HRIFA008459a, HRIFA008483a, HRIFA008547a, HRIFA008611a, HRIFA008661a, HRIFA008717a, HRIFA008784a, HRIFA008981a, HRIFA009101a, HRIFA009171a, HRIFA009220a, HRIFA009451a, HRIFA009482a, HRIFA009783a, HRIFA009881a, HRIFA010085a, HRIFA010090a,
HRIFA010130a, HRIFA010319a, HRIFA010394a, HRIFA010460a, HRIFA010790a, HRIFA010975a, HRIFA011016a, HRIFA011179a, HRIFA011197a, HRIFA011449a, HRIFA011659a, HRIFA011947a, HRIFA012278a, HRIFA012584a, HRIFA012625a, HRIFA012692a, HRIFA012795a, HRIFA012885a, HRIFA012914a, HRIFA012969a, HRIFA012990a, HRIFA013254a, HRIFA013265a, HRIFA013276a, HRIFA013376a, HRIFA013477a, HRIFA013586a, HRIFA013726a, HRIFA013744a, HRIFA013911a, HRIFA014006a, HRIFA014185a, HRIFA014336a, HRIFA014465a, HRIFA014500a, HRIFA014561a, HRIFA014568a, HRIFA014621a, HRIFA014688a, HRIFA014819a, HRIFA014951a, HRIFA014967a, HRIFA015063a, HRIFA015070a, HRIFA015246a, HRIFA015423a, HRIFA015453a, HRIFA015486a, HRIFA015506a, HRIFA015536a, HRIFA015547a, HRIFA015568a, HRIFA015756a, HRIFA015811a, HRIFA016070a, HRIFA016290a, HRIFA016430a, HRIFA016654a, HRIFA016758a, HRIFA017031a, HRIFA017257a, HRIFA017295a, HRIFA017312a, HRIFA017703a, HRIFA017855a, HRIFA018092a, HRIFA018131a, HRIFA018134a, HRIFA018580a, HRIFA018827a, HRIFA018904a, HRIFA018993a, HRIFA019105a, HRIFA019136a, HRIFA019175a, HRIFA019262a, HRIFA019466a, HRIFA019867a, HRIFA019869a, HRIFA020272a, HRIFA020335a, HRIFA020349a, HRIFA020862a, HRIFA021213a, HRIFA021398a, HRIFA021499a, HRIFA021637a, HRIFA021651a, HRIFA021754a, HRIFA021781a, HRIFA022065a, HRIFA022139a, HRIFA022166a, HRIFA022177a, HRIFA022182a, HRIFA022227a, HRIFA022249a, HRIFA022265a, HRIFA022328a, HRIFA022423a, HRIFA022528a, HRIFA022546a, HRIFA022564a, HRIFA022616a, HRIFA022671a, HRIFA022691a, HRIFA022707a, HRIFA022729a, HRIFA022737a, HRIFA022776a, HRIFA022875a, HRAFA022895a, HRIFA023007a, HRIFA023227a, HRIFA023257a, HRIFA023304a, HRIFA023464a, HRIFA023767a, HRIFA023923a, HRIFA024132a, HRIFA024255a, HRIFA024392a, HRIFA024423a, HRIFA024504a, HRIFA024718a, HRIFA024767a, HRIFA024937a, HRIFA024994a, HRIFA025046a, HRIFA025250a, HRIFA025261a, HRIFA025353a, HRIFA025492a, HRIFA025636a, HRIFA025695a, HRIFA025706a, HRIFA025766a, HRIFA025800a, HRIFA025907a, HRIFA025913a, HRIFA026089a, HRIFA026364a, HRIFA026496a, HRIFA026789a, HRIFA026813a, HRIFA026860a, HRIFA027012a, HRIFA027045a, HRIFA027125a, HRIFA027179a, HRIFA027187a, HRIFA027622a, HRIFA027625a, HRIFA027656a, HRIFA027681a, HRIFA027722a, HRIFA027940a, HRIFA028157a, HRIFA028402a, HRIFA028468a, HRIFA028511a, HRIFA028651a, HRIFA028790a, HRIFA029002a, HRIFA029208a, HRIFA029209a, HRIFA029256a, HRIFA029263a, HRIFA029285a, HRIFA029317a, HRIFA029327a, HRIFA029393a, HRIFA029511a, HRIFA029802a, HRIFA029866a, HRIFA029932a, HRIFA030025a, HRIFA030045a, HRIFA030250a, HRIFA030342a, HRIFA030370a, HRIFA030371a, HRIFA030411a, HRIFA030448a, HRIFA030545a, HRIFA030629a, HRIFA030642a, HRIFA030662a, HRIFA031336a, HRIFA031869a, HRIFA031986a, HRIFA032009a, HRIFA032011a, HRIFA032070a, HRIFA032073a, HRIFA032079a, HRIFA032224a, HRIFA032274a, HRIFA032275a, HRIFA032433a,
HRIFA032453a, HRIFA032605a, HRIFA032696a, HRIFA032730a,

### Homology search result 3

Representative sequence of the 5'-end cluster exhibiting relatively low homology (221 cluster: "exhibiting relatively low homology" means that the P value is higher than 10⁻¹⁰ and 10⁻⁴ or less)
HRIFA000016a, HRIFA000071a, HRIFA000116a, HRIFA000123a, HRIFA000264a, HRIFA000415a, HRIFA000446a, HRIFA000695a, HRIFA000845a, HRIFA001971a, HRIFA002063a, HRIFA002102a, HRIFA002284a, HRIFA002309a, HRIFA002694a, HRIFA002762a, HRIFA002787a, HRIFA003055a, HRIFA003340a, HRIFA003402a, HRIFA003504a, HRIFA003892a, HRIFA003946a, HRIFA004162a, HRIFA004401a, HRIFA004780a, HRIFA005072a, HRIFA005102a, HRIFA005214a, HRIFA005255a, HRIFA005300a, HRIFA005369a, HRIFA005702a, HRIFA005728a, HRIFA005944a, HRIFA006298a, HRIFA006448a, HRIFA006572a, HRIFA006633a, HRIFA006642a, HRIFA007068a, HRIFA007244a, HRIFA007262a, HRIFA007512a, HRIFA007532a, HRIFA007565a, HRIFA007728a, HRIFA007909a, HRIFA008174a, HRIFA008426a, HRIFA008596a, HRIFA008790a, HRIFA008989a, HRIFA009578a, HRIFA009825a, HRIFA009852a, HRIFA009983a, HRIFA010005a, HRIFA010078a, HRIFA010152a, HRIFA010301a, HRIFA010361a, HRIFA010425a, HRIFA010466a, HRIFA010799a, HRIFA011580a, HRIFA011820a, HRIFA012167a, HRIFA012354a, HRIFA012427a, HRIFA012436a, HRIFA012515a, HRIFA012702a, HRIFA012737a, HRIFA013135a, HRIFA013235a, HRIFA013279a, HRIFA013589a, HRIFA013620a, HRIFA013919a, HRIFA013932a, HRIFA014056a, HRIFA014111a, HRIFA014133a, HRIFA014396a, HRIFA014397a, HRIFA014598a, HRIFA014702a, HRIFA014868a, HRIFA015219a, HRIFA015995a, HRIFA016214a, HRIFA016240a, HRIFA016255a, HRIFA016639a, HRIFA016669a, HRIFA016963a, HRIFA017457a, HRIFA017643a, HRIFA017670a,
HRIFA017801a, HRIFA017836a, HRIFA017921a, HRIFA018238a, HRIFA018262a, HRIFA018287a, HRIFA018666a, HRIFA018688a, HRIFA018754a, HRIFA018794a, HRIFA018870a, HRIFA018931a, HRIFA019412a, HRIFA019490a, HRIFA019498a, HRIFA019532a, HRIFA019651a, HRIFA0201440, HRIFA020184a, HRIFA020453a, HRIFA020693a, HRIFA020707a, HRIFA020748a, HRIFA021061a, HRIFA021224a, HRIFA021494a, HRIFA021794a, HRIFA021855a, HRIFA021906a, HRIFA022156a, HRIFA022203a, HRIFA022234a, HRIFA022702a, HRIFA022728a, HRIFA022782a, HRIFA022865a, HRIFA022890a, HRIFA022985a, HRIFA023048a, HRIFA023069a, HRIFA023129a, HRIFA023154a, HRIFA023212a, HRIFA023489a, HRIFA023634a, HRIFA023894a, HRIFA024088a, HRIFA024197a, HRIFA024218a, HRIFA024473a, HRIFA024482a, HRIFA024543a, HRIFA025327a, HRIFA025479a, HRIFA025488a, HRIFA025703a, HRIFA025771a, HRIFA025778a, HRIFA025904a, HRIFA025966a, HRIFA025978a, HRIFA026121a, HRIFA026242a, HRIFA026316a, HRIFA026382a, HRIFA026465a, HRIFA026519a, HRIFA026564a, HRIFA026576a, HRIFA026618a, HRIFA026659a, HRIFA026764a, HRIFA027327a, HRIFA027329a, HRIFA027355a, HRIFA027644a, HRIFA027673a, HRIFA027714a, HRIFA027860a, HRIFA028061a, HRIFA028187a, HRIFA028262a, HRIFA028371a, HRIFA028440a, HRIFA028501a, HRIFA028576a, HRIFA028614a, HRIFA028911a, HRIFA029050a, HRIFA029278a, HRIFA029349a, HRIFA029425a, HRIFA029434a, HRIFA029460a, HRIFA029467a, HRIFA029508a, HRIFA029730a, HRIFA029792a, HRIFA030103a, HRIFA030147a,
HRIFA030264a, HRIFA030381a, HRIFA030456a, HRIFA030509a, HRIFA030511a, HRIFA030566a, HRIFA030599a, HRIFA031126a, HRIFA031249a, HRIFA031438a, HRIFA031935a, HRIFA032257a, HRIFA032360a, HRIFA032389a, HRIFA032478a, HRIFA032506a, HRIFA032511a, HRIFA032530a, HRIFA032587a, HRIFA032642a, HRIFA032820a,

### Homology search result 4

Representative sequence of the 5'-end cluster exhibiting low homology (115 cluster: "exhibiting low homology" means that the P value is higher than 10⁻⁴ and 1 or less)
HRIFA001099a, HRIFA001200a, HRIFA001413a, HRIFA001439a, HRIFA001558a, HRIFA001866a, HRIFA001972a, HRIFA002689a, HRIFA003357a, HRIFA003592a, HRIFA003640a, HRIFA003883a, HRIFA005296a, HRIFA005500a, HRIFA005540a, HRIFA006250a, HRIFA006609a, HRIFA006798a, HRIFA007032a, HRIFA007152a, HRIFA007547a, HRIFA007829a, HRIFA007985a, HRIFA008212a, HRIFA008252a, HRIFA008976a, HRIFA009071a, HRIFA009123a, HRIFA009136a, HRIFA009339a, HRIFA009762a, HRIFA010176a, HRIFA010490a, HRIFA010736a, HRIFA010859a, HRIFA010891a, HRIFA010988a, HRIFA011105a, HRIFA011128a, HRIFA011484a, HRIFA011512a, HRIFA011926a, HRIFA012069a, HRIFA012151a, HRIFA013092a, HRIFA013103a, HRIFA013980a, HRIFA014024a, HRIFA014590a, HRIFA014620a, HRIFA015122a, HRIFA015351a, HRIFA015802a, HRIFA015902a, HRIFA015947a, HRIFA016599a, HRIFA017146a, HRIFA017190a, HRIFA017456a, HRIFA017791a, HRIFA017818a, HRIFA018447a, HRIFA019437a, HRIFA019958a, HRIFA020883a, HRIFA021007a, HRIFA021040a, HRIFA021445a, HRIFA021543a, HRIFA021620a, HRIFA021787a, HRIFA022055a, HRIFA022335a, HRIFA022348a, HRIFA022411a, HRIFA022462a, HRIFA022493a, HRIFA022714a, HRIFA023434a, HRIFA024185a, HRIFA024305a, HRIFA024884a, HRIFA024893a, HRIFA024978a, HRIFA025033a, HRIFA025290a, HRIFA026265a, HRIFA026303a, HRIFA026351a, HRIFA026615a, HRIFA026923a, HRIFA027173a, HRIFA027485a, HRIFA027536a, HRIFA027549a, HRIFA027867a, HRIFA028804a, HRIFA028867a, HRIFA028983a, HRIFA029398a,
HRIFA029440a, HRIFA029602a, HRIFA029649a, HRIFA029715a, HRIFA030106a, HRIFA030385a, HRIFA030461a, HRIFA030472a, HRIFA030839a, HRIFA031091a, HRIFA031395a, HRIFA031397a, HRIFA032097a, HRIFA032161a, HRIFA032186a,

### Homology search result 5

The result of the homology search in the SwissProt using the clone sequences of the 5'-ends.
Indicated are from the top,
the name of the clone sequence,
definition of the top hit data,
the P-value: the length of the sequence used for comparison (nucleotide):similarity (%),
the organism of which the top hit data is obtained,
the Accession No. of the top hit data.

Data were not shown for the clones in which the P-value was higher than 1.
F-BNGH41000020
   NADH-UBIQUINONE OXIDOREDUCTASE CHAIN 1 (EC 1.6.5.3).
   1.2e-119:279:83
   HOMO SAPIENS (HUMAN).
   P03886
F-BNGH41000087
   PROPERDIN PRECURSOR.
   2.5e-06:218:32
   HOMO SAPIENS (HUMAN).
   P27918
F-BNGH41000091
   POTASSIUM CHANNEL PROTEIN EAG.
   3.1e-66:139:61
   DROSOPHILA MELANOGASTER (FRUIT FLY).
   Q02280
F-HEMBA1000006
   S-ANTIGEN PROTEIN PRECURSOR.
   3.0e-05:164:31
   PLASMODIUM FALCIPARUM (ISOLATE V1).
   P09593
F-HEMBA1000121
   HYPOTHETICAL 68.7 KD PROTEIN ZK757.1 IN CHROMOSOME III.
   8.2e-06:83:27
   CAENORHABDITIS ELEGANS.
   P34679
F-HEMBA1000128
   PATHOGENESIS-RELATED PROTEIN 1 PRECURSOR (PR-1).
   8.2e-08:89:34
   ARABIDOPSIS THALIANA (MOUSE-EAR CRESS).
   P33154
F-HEMBA1000275
   PROCOLLAGEN ALPHA 1(I) CHAIN PRECURSOR.
   1.9e-06:231:34
   GALLUS GALLUS (CHICKEN).
   P02457
F-HEMBA1000300
   !!!! ALU SUBFAMILY SX WARNING ENTRY !!!!
   1.4e-13:73:56
   HOMO SAPIENS (HUMAN).
   P39195
F-HEMBA1000349
   ATP-BINDING CASSETTE TRANSPORTER 1.
   2.6e-16:238:31
   MUS MUSCULUS (MOUSE).
   P41233
F-HEMBA1000443
   PROLINE-RICH PROTEIN MP-2 PRECURSOR.
   4.8e-06:120:35
   MUS MUSCULUS (MOUSE).
   P05142
F-HEMBA1000462
   PUTATIVE GENERAL NEGATIVE REGULATOR OF TRANSCRIPTION C16C9.04C.
   2.9e-21:86:52
   SCHIZOSACCHAROMYCES POMBE (FISSION YEAST).
   Q09818
F-HEMBA1000477
   HYPOTHETICAL 64.8 KD PROTEIN IN GDI1-COX15 INTERGENIC REGION.
   3.3e-09:138:34
   SACCHAROMYCES CEREVISIAE (BAKER'S YEAST).
   P40085
F-HEMBA1000590
   CARTILAGE MATRIX PROTEIN PRECURSOR (MATRILIN-1).
   2.2e-27:117:48
   GALLUS GALLUS (CHICKEN).
   P05099
F-HEMBA1000634
   TRAMS-ACTING TRANSCRIPTIONAL PROTEIN ICP0 (IMMEDIATE-EARLY PROTEIN IE110) (VMW110) (ALPHA-0 PROTEIN).
   0.00027:85:43
   HERPES SIMPLEX VIRUS (TYPE 1 / STRAIN 17).
   P08393
F-HEMBA1000671
   ZINC FINGER PROTEIN 140.
   1.1e-44:155:47
   HOMO SAPIENS (HUMAN).
   P52738
F-HEMBA1000713
   BLADDER CANCER 10 KD PROTEIN.
   1.5e-42:81:97
   HOMO SAPIENS (HUMAN).
   060629
F-HEMBA1000732
   FIBRILLIN 1 PRECURSOR.
   6.3e-18:77:46
   HOMO SAPIENS (HUMAN).
   P35555
F-HEMBA1000745
   SALIVARY PROLINE-RICH PROTEIN PRECURSOR (CLONES CP3, CP4 AND CP5) [CONTAINS: BASIC PEPTIDE IB-6; PEPTIDE P-H].
   5.2e-06:105:33
   HOMO SAPIENS (HUMAN).
   P04280
F-HEMBA1000835
   FIBRILLIN 2 PRECURSOR.
   2.1e-42:214:44
   HOMO SAPIENS (HUMAN).
   P35556
F-HEMBA1000875
   ZINC FINGER PROTEIN 133.
   5.8e-16:49:87
   HOMO SAPIENS (HUMAN).
   P52736
F-HEMBA1000907
   PROCOLLAGEN ALPHA 1(I) CHAIN PRECURSOR.
   2.2e-05:172:34
   MUS MUSCULUS (MOUSE).
   P11087
F-HEMBA1000940
   GAP JUNCTION CX43.4 PROTEIN (CONNEXIN 43.4) (CX43.4).
   1.4e-20:90:42
   BRACHYDANIO RERIO (ZEBRAFISH) (ZEBRA DANIO).
   Q92052
F-HEMBA1000962
   WATER-STRESS INDUCIBLE PROTEIN RAB21.
   0.089:122:25
   ORYZA SATIVA (RICE).
   P12253
F-HEMBA1001184
   SH3BGR PROTEIN (21-GLUTAMIC ACID-RICH PROTEIN) (21-GARP).
   4.9e-33:100:60
   HOMO SAPIENS (HUMAN).
   P55822
F-HEMBA1001221
   AGRIN PRECURSOR.
   1.7e-26:239:32
   GALLUS GALLUS (CHICKEN).
   P31696
F-HEMBA1001228
   CARTILAGE OLIGOMERIC MATRIX PROTEIN PRECURSOR (COMP).
   7.7e-114:147:83
   HOMO SAPIENS (HUMAN).
   P49747
F-HEMBA1001272
   SPLICEOSOME ASSOCIATED PROTEIN 49 (SAP 49) (SF3B53).
   5.8e-06:129:33
   HOMO SAPIENS (HUMAN).
   Q15427
F-HEMBA1001296
   TRANSCRIPTION FACTOR BF-2 (BRAIN FACTOR 2) (BF2) (CBF-2) (T-14-6).
   0.0019:115:36
   GALLUS GALLUS (CHICKEN).
   Q98937
F-HEMBA1001297
   50S RIBOSOMAL PROTEIN L37E (L35E).
   0.65:40:40
   HALOARCULA MARISMORTUI (HALOBACTERIUM MARISMORTUI).
   P32410
F-HEMBA1001390
   SPIDROIN 2 (DRAGLINE SILK FIBROIN 2) (FRAGMENT).
   0.00050:89:33
   NEPHILA CLAVIPES (ORB SPIDER).
   P46804
F-HEMBA1001563
   B-CELL GROWTH FACTOR PRECURSOR (BCGF-12 KD).
   0.00041:34:61
   HOMO SAPIENS (HUMAN).
   P20931
F-HEMBA1001621
   PROBABLE G PROTEIN-COUPLED RECEPTOR APJ.
   1.1e-64:105:72
   HOMO SAPIENS (HUMAN).
   P35414
F-HEMBA1001878
   VEGETATIBLE INCOMPATIBILITY PROTEIN HET-E-1.
   1.3e-24:170:35
   PODOSPORA ANSERINA.
   Q00808
F-HEMBA1001886
   ZINC FINGER PROTEIN 85 (ZINC FINGER PROTEIN HPF4) (HTF1).
   1.8e-94:273:64
   HOMO SAPIENS (HUMAN).
   Q03923
F-HEMBA1002048
   EARLY ANTIGEN PROTEIN D (EA-D).
   0.13:93:34
   EPSTEIN-BARR VIRUS (STRAIN B95-8) (HUMAN HERPESVIRUS 4).
   P03191
F-HEMBA1002131
   PROCOLLAGEN-LYSINE,2-OXOGLUTARATE 5-DIOXYGENASE 1 PRECURSOR (EC 1.14.11.4) (LYSYL HYDROXYLASE 1) (LH1).
   6.3e-12:140:30
   GALLUS GALLUS (CHICKEN).
   P24802
F-HEMBA1002163
   HYPOTHETICAL 40.7 KD PROTEIN IN DAK1-ORC1 INTERGENIC REGION.
   2.1e-10:204:27
   SACCHAROMYCES CEREVISIAE (BAKER'S YEAST).
   Q04651
F-HEMBA1002164
   BIOTIN CARBOXYL CARRIER PROTEIN OF ACETYL-COA CARBOXYLASE PRECURSOR (EC 6.4.1.2) (BCCP).
   0.022:62:32
   GLYCINE MAX (SOYBEAN).
   Q42783
F-HEMBA1002167
   ACETYLCHOLINESTERASE PRECURSOR (EC 3.1.1.7).
   5.2e-31:247:31
   BUNGARUS FASCIATUS (BANDED KRAIT).
   Q92035
F-HEMBA1002178
   PROCOLLAGEN-LYSINE,2-OXOGLUTARATE 5-DIOXYGENASE 1 PRECURSOR (EC 1.14.11.4) (LYSYL HYDROXYLASE 1) (LH1).
   1.5e-11:140:30
   GALLUS GALLUS (CHICKEN).
   P24802
F-HEMBA1002195
   VEGETATIBLE INCOMPATIBILITY PROTEIN HET-E-1.
   5.0e-07:52:36
   PODOSPORA ANSERINA.
   Q00808
F-HEMBA1002227
   MYRISTOYLATED ALANINE-RICH C-KINASE SUBSTRATE (MARCKS) (ACAMP-81).
   0.00063:21:100
   BOS TAURUS (BOVINE).
   P12624
F-HEMBA1002239
   !!!! ALU SUBFAMILY SC WARNING ENTRY !!!!
   1.5e-33:101:70
   HOMO SAPIENS (HUMAN).
   P39192
F-HEMBA1002316
   EXTENSIN PRECURSOR (PROLINE-RICH GLYCOPROTEIN).
   4.6e-08:186:32
   SORGHUM VULGARE (SORGHUM).
   P24152
F-HEMBA1002420
   WISKOTT-ALDRICH SYNDROME PROTEIN HOMOLOG (WASP).
   0.0078:19:68
   MUS MUSCULUS (MOUSE).
   P70315
F-HEMBA1002421
   SYNDECAN-2 PRECURSOR (FIBROGLYCAN) (HEPARAN SULFATE PROTEOGLYCAN CORE PROTEIN) (HSPG) (SYND2).
   1.1e-52:107:97
   HOMO SAPIENS (HUMAN).
   P34741
F-HEMBA1002524
   ACIDIC PROLINE-RICH PROTEIN PRECURSOR (CLONE PRP33).
   5.0e-05:104:34
   RATTUS NORVEGICUS (RAT).
   P04474
F-HEMBA1002551
   HYPOTHETICAL WD-REPEAT PROTEIN SLR0143.
   9.9e-09:128:29
   SYNECHOCYSTIS SP. (STRAIN PCC 6803).
   P74442
F-HEMBA1002767
   N-ACETYLLACTOSAMINE SYNTHASE (EC 2.4.1.90) (N-ACETYLGLUCOSAMINE (BETA 1→4)GALACTOSYLTRANSFERASE) (EC 2.4.1.38) (LACTOSE SYNTHASE A PROTEIN (EC 2.4.1.22)) (GALACTOSYLTRANSFERASE) (GT).
   8.0e-92:246:67
   MUS MUSCULUS (MOUSE).
   P15535
F-HEMBA1002985
   TRANSCRIPTION FACTOR GATA-6 (GATA BINDING FACTOR-6).
   0.060:49:34
   MUS MUSCULUS (MOUSE).
   Q61169
F-HEMBA1002992
   HOLOTRICIN 3 PRECURSOR.
   0.0035:64:37
   HOLOTRICHIA DIOMPHALIA.
   Q25055
F-HEMBA1003047
   BONE MORPHOGENETIC PROTEIN 1 PRECURSOR (EC 3.4.24.-) (BMP-1).
   1.5e-23:216:31
   HOMO SAPIENS (HUMAN).
   P13497
F-HEMBA1003072
   PROLINE-RICH PROTEIN MP-2 PRECURSOR.
   6.8e-09:129:41
   MUS MUSCULUS (MOUSE).
   P05142
F-HEMBA1003101
   PROCOLLAGEN ALPHA 2(I) CHAIN PRECURSOR.
   2.2e-10:124:37
   HOMO SAPIENS (HUMAN).
   P08123
F-HEMBA1003120
   ZINC FINGER PROTEIN 140.
   4.8e-23:43:74
   HOMO SAPIENS (HUMAN).
   P52738
F-HEMBA1003230
   FIBULIN-1, ISOFORM C PRECURSOR (BASEMENT-MEMBRANE PROTEIN 90) (BM-90).
   2.7e-41:239:39
   MUS MUSCULUS (MOUSE).
   Q08878
F-HEMBA1003294
   !!!! ALU SUBFAMILY SQ WARNING ENTRY !!!!
   7.0e-34:84:69
   HOMO SAPIENS (HUMAN).
   P39194
F-HEMBA1003315
   GLYCINE-RICH CELL WALL STRUCTURAL PROTEIN 1.8 PRECURSOR (GRP 1.8).
   0.00012:178:32
   PHASEOLUS VULGARIS (KIDNEY BEAN) (FRENCH BEAN).
   P10496
F-HEMBA1003392
   LOW-DENSITY LIPOPROTEIN RECEPTOR-RELATED PROTEIN 1 PRECURSOR (LRP) (ALPHA-2-MACROGLOBULIN RECEPTOR) (A2MR).
   1.1e-31:202:37
   GALLUS GALLUS (CHICKEN).
   P98157
F-HEMBA1003399
   MVP1 PROTEIN.
   5.6e-12:67:38
   SACCHAROMYCES CEREVISIAE (BAKER'S YEAST).
   P40959
F-HEMBA1003487
   PROLINE-RICH PROTEIN MP-2 PRECURSOR.
   4.5e-08:175:29
   MUS MUSCULUS (MOUSE).
   P05142
F-HEMBA1003497
   ZINC FINGER PROTEIN 151 (POLYOMAVIRUS LATE INITIATOR PROMOTER BINDING PROTEIN) (LP-1) (ZINC FINGER PROTEIN Z13).
   9.3e-18:171:33
   MUS MUSCULUS (MOUSE).
   Q60821
F-HEMBA1003530
   SALIVARY PROLINE-RICH PROTEIN II-1 (FRAGMENT).
   9.9e-12:122:35
   HOMO SAPIENS (HUMAN).
   P81489
F-HEMBA1003602
   PROLINE-RICH PROTEIN MP-3 (FRAGMENT).
   0.98:114:33
   MUS MUSCULUS (MOUSE).
   P05143
F-HEMBA1003732
   TRANSCRIPTIONAL REGULATORY PROTEIN ALGP (ALGINATE REGULATORY PROTEIN ALGR3).
   0.35:225:28
   PSEUDOMONAS AERUGINOSA.
   P15276
F-HEMBA1003945
   HYPOTHETICAL 43.7 KD PROTEIN C24B11.08C IN CHROMOSOME I.
   2.9e-48:268:41
   SCHIZOSACCHAROMYCES POMBE (FISSION YEAST).
   Q09895
F-HEMBA1004007
   THYROID TRANSCRIPTION FACTOR 1 (THYROID NUCLEAR FACTOR 1) (TTF-1) (FRAGMENT).
   0.90:60:30
   CAVIA PORCELLUS (GUINEA PIG).
   P97273
F-HEMBA1004067
   TRANS-ACTING TRANSCRIPTIONAL PROTEIN ICP0 (IMMEDIATE-EARLY PROTEIN IE110) (VMW110) (ALPHA-0 PROTEIN).
   3.0e-05:200:31
   HERPES SIMPLEX VIRUS (TYPE 1 / STRAIN 17).
   P08393
F-HEMBA1004085
   GLUCOSE REPRESSION MEDIATOR PROTEIN.
   0.0030:190:26
   SACCHAROMYCES CEREVISIAE (BAKER'S YEAST).
   P14922
F-HEMBA1004110
   EPIDERMAL GROWTH FACTOR RECEPTOR SUBSTRATE SUBSTRATE 15 (PROTEIN EPS15).
   1.2e-14:102:36
   MUS MUSCULUS (MOUSE).
   P42567
F-HEMBA1004250
   CADHERIN-RELATED TUMOR SUPPRESSOR PRECURSOR (FAT PROTEIN).
   1.8e-08:150:33
   DROSOPHILA MELANOGASTER (FRUIT FLY).
   P33450
F-HEMBA1004391
   TUMOR SUPPRESSOR PROTEIN DCC PRECURSOR.
   4.5e-09:96:35
   MUS MUSCULUS (MOUSE).
   P70211
F-HEMBA1004444
   GLYCOPROTEIN 25L PRECURSOR (GP25L).
   4.6e-41:148:52
   CANIS FAMILIARIS (DOG).
   P27869
F-HEMBA1004454
   CD9 ANTIGEN.
   0.0070:24:70
   BOS TAURUS (BOVINE).
   P30932
F-HEMBA1004505
   MANNOSYL-OLIGOSACCHARIDE ALPHA-1,2-MANNOSIDASE ISOFORM 1 (EC 3.2.1.113) (MAN(9)-ALPHA-MANNOSIDASE).
   7.0e-45:239:43
   DROSOPHILA MELANOGASTER (FRUIT FLY).
   P53624
F-HEMBA1004785
   MODIFIER 3 PROTEIN (M33).
   7.4e-26:76:61
   MUS MUSCULUS (MOUSE).
   P30658
F-HEMBA1004797
   PROTEIN Q300.
   0.00071:21:66
   MUS MUSCULUS (MOUSE).
   Q02722
F-HEMBA1004952
   EBNA-1 NUCLEAR PROTEIN.
   2.4e-05:67:49
   EPSTEIN-BARR VIRUS (STRAIN B95-8) (HUMAN HERPESVIRUS 4).
   P03211
F-HEMBA1004971
F-HEMBA1004982
   MULTIDRUG RESISTANCE PROTEIN 2 (MULTIDRUG-EFFLUX TRANSPORTER 2).
   8.6e-08:144:25
   BACILLUS SUBTILIS.
   P39843
F-HEMBA1005070
   HYPOTHETICAL PROTEIN KIAA0310.
   1.0e-38:140:68
   HOMO SAPIENS (HUMAN).
   O15027
F-HEMBA1005084
   NON-RECEPTOR TYROSINE KINASE SPORE LYSIS A (EC 2.7.1.112) (TYROSINE-PROTEIN KINASE 1).
   2.5e-10:102:37
   DICTYOSTELIUM DISCOIDEUM (SLIME MOLD).
   P18160
F-HEMBA1005145
   PROLINE-RICH PROTEIN MP-2 PRECURSOR.
   1.8e-06:85:37
   MUS MUSCULUS (MOUSE).
   P05142
F-HEMBA1005230
   ZINC FINGER PROTEIN 140.
   8.2e-20:83:66
   HOMO SAPIENS (HUMAN).
   P52738
F-HEMBA1005246
   TRANSCRIPTION REGULATORY PROTEIN SNF5 (SWI/SNF COMPLEX COMPONENT SNF5) (TRANSCRIPTION FACTOR TYE4).
   1.5e-09:132:34
   SACCHAROMYCES CEREVISIAE (BAKER'S YEAST).
   P18480
F-HEMBA1005267
   B-CELL LYMPHOMA 3-ENCODED PROTEIN (BCL-3 PROTEIN).
   1.9e-15:192:32
   HOMO SAPIENS (HUMAN).
   P20749
F-HEMBA1005337
   ACIDIC PHOSPHOPROTEIN PRECURSOR (50 KD ANTIGEN).
   8.0e-05:31:64
   PLASMODIUM CHABAUDI.
   Q02752
F-HEMBA1005430
   MALE SPECIFIC SPERM PROTEIN MST84DB.
   0.34:42:42
   DROSOPHILA MELANOGASTER (FRUIT FLY).
   Q01643
F-HEMBA1005449
   PROCYCLIC FORM SPECIFIC POLYPEPTIDE B-ALPHA PRECURSOR (PROCYCLIN) (PARP B-ALPHA) (PSSA-1).
   4.5e-09:95:33
   TRYPANOSOMA BRUCEI BRUCEI.
   Q06084
F-HEMBA1005489
   CHANNEL ASSOCIATED PROTEIN OF SYNAPSE-110 (CHAPSYN-110).
   7.2e-05:90:36
   HOMO SAPIENS (HUMAN).
   Q15700
F-HEMBA1005522
   COAGULATION FACTOR VII PRECURSOR (EC 3.4.21.21).
   3.3e-17:78:51
   ORYCTOLAGUS CUNICULUS (RABBIT).
   P98139
F-HEMBA1005545
   MUSCARINIC ACETYLCHOLINE RECEPTOR M3.
   7.2e-91:211:85
   HOMO SAPIENS (HUMAN).
   P20309
F-HEMBA1005698
   PROTEIN TRANSPORT PROTEIN SEC22 (PROTEIN SLY2).
   3.3e-08:132:28
   SACCHAROMYCES CEREVISIAE (BAKER'S YEAST).
   P22214
F-HEMBA1005913
   HYPOTHETICAL 5.8 KD PROTEIN.
   0.97:43:30
   CLOVER YELLOW MOSAIC VIRUS (CYMV).
   P16485
F-HEMBA1005929
   PUTATIVE SERINE/THREONINE-PROTEIN KINASE P78 (EC 2.7.1.-).
   6.6e-104:275:72
   HOMO SAPIENS (HUMAN).
   P27448
F-HEMBA1005945
   BRITTLE-1 PROTEIN PRECURSOR.
   7.8e-30:214:35
   ZEA MAYS (MAIZE).
   P29518
F-HEMBA1006016
   !!!! ALU SUBFAMILY J WARNING ENTRY !!!!
   1.9e-07:34:76
   HOMO SAPIENS (HUMAN).
   P39188
F-HEMBA1006171
   PROBABLE E5 PROTEIN.
   0.98:66:31
   HUMAN PAPILLOMAVIRUS TYPE 33.
   P06426
F-HEMBA1006276
   ZINC FINGER PROTEIN 90 (ZFP-90) (ZINC FINGER PROTEIN NK10).
   4.1e-07:56:57
   MUS MUSCULUS (MOUSE).
   Q61967
F-HEMBA1006299
   BASIC PROLINE-RICH PEPTIDE P-E (IB-9).
   0.11:38:28
   HOMO SAPIENS (HUMAN).
   P02811
F-HEMBA1006311
   ZINC FINGER PROTEIN 23 (ZINC FINGER PROTEIN KOX16) (FRAGMENT).
   0.91:22:45
   HOMO SAPIENS (HUMAN).
   P17027
F-HEMBA1006335
   PERIPHERAL MYELIN PROTEIN 22 (PMP-22) (GROWTH-ARREST-SPECIFIC PROTEIN 3) (GAS3).
   0.017:125:27
   MUS MUSCULUS (MOUSE).
   P16646
F-HEMBA1006357
   SECRETORY CARRIER-ASSOCIATED MEMBRANE PROTEIN 2.
   5.2e-40:136:52
   HOMO SAPIENS (HUMAN).
   O15127
F-HEMBA1006430
   OLIGOSACCHARYL TRANSFERASE STT3 SUBUNIT HOMOLOG.
   2.7e-38:96:72
   CAENORHABDITIS ELEGANS.
   P46975
F-HEMBA1006482
   SCO1 PROTEIN PRECURSOR.
   7.1e-25:84:45
   SACCHAROMYCES CEREVISIAE (BAKER'S YEAST).
   P23833
F-HEMBA1006517
   HYPOTHETICAL 93.4 KD PROTEIN IN STE3-GIN10 INTERGENIC REGION.
   0.48:145:30
   SACCHAROMYCES CEREVISIAE (BAKER'S YEAST).
   P34239
F-HEMBA1006544
   TRANSCRIPTION INITIATION FACTOR TFIID 135 KD SUBUNIT (TAFII-135) (TAFII135) (TAFII-130) (TAFII130).
   7.0e-11:210:37
   HOMO SAPIENS (HUMAN).
   000268
F-HEMBA1006572
   ANTITHROMBIN-III PRECURSOR (ATIII) (FRAGMENT).
   0.011:50:40
   GALLUS GALLUS (CHICKEN).
   Q03352
F-HEMBA1006658
   SERINE/THREONINE-PROTEIN KINASE MIG-15 (EC 2.7.1.-).
   4.6e-44:234:45
   CAENORHABDITIS ELEGANS.
   Q23356
F-HEMBA1006707
   CARTILAGE MATRIX PROTEIN PRECURSOR (MATRILIN-1).
   9.3e-34:159:50
   GALLUS GALLUS (CHICKEN).
   P05099
F-HEMBA1006724
   PLATELET FACTOR 4 (PF-4).
   0.025:65:27
   SUS SCROFA (PIG).
   P30034
F-HEMBA1006749
   CARTILAGE MATRIX PROTEIN PRECURSOR (MATRILIN-1).
   7.9e-37:147:53
   GALLUS GALLUS (CHICKEN).
   P05099
F-HEMBA1006770
   FLOWERING TIME CONTROL PROTEIN FCA.
   3.4e-27:139:39
   ARABIDOPSIS THALIANA (MOUSE-EAR CRESS).
   004425
F-HEMBA1006902
   CARTILAGE MATRIX PROTEIN PRECURSOR (MATRILIN-1).
   1.5e-37:137:51
   GALLUS GALLUS (CHICKEN).
   P05099
F-HEMBA1006912
   TRANS-ACTING TRANSCRIPTIONAL PROTEIN ICP0 (IMMEDIATE-EARLY PROTEIN IE110) (VMW110) (ALPHA-0 PROTEIN).
   0.27:121:29
   HERPES SIMPLEX VIRUS (TYPE 1 / STRAIN 17).
   P08393
F-HEMBA1006916
   SKIN SECRETORY PROTEIN XP2 PRECURSOR (APEG PROTEIN).
   1.1e-05:163:30
   XENOPUS LAEVIS (AFRICAN CLAWED FROG).
   P17437
F-HEMBA1006960
   SMALL PROLINE-RICH PROTEIN 2-1.
   1.0:34:35
   HOMO SAPIENS (HUMAN).
   P35326
F-HEMBA1007013
   S-ANTIGEN PROTEIN PRECURSOR.
   2.8e-09:226:28
   PLASMODIUM FALCIPARUM (ISOLATE V1).
   P09593
F-HEMBA1007057
   PROCYCLIC FORM SPECIFIC POLYPEPTIDE B-ALPHA PRECURSOR (PROCYCLIN) (PARP B-ALPHA) (PSSA-1).
   4.0e-10:33:72
   TRYPANOSOMA BRUCEI BRUCEI.
   Q06084
F-HEMBA1007063
   AGAMOUS PROTEIN.
   1.0:40:42
   ARABIDOPSIS THALIANA (MOUSE-EAR CRESS).
   P17839
F-HEMBA1007226
   PUTATIVE CUTICLE COLLAGEN C09G5.5.
   0.10:105:38
   CAENORHABDITIS ELEGANS.
   Q09456
F-HEMBA1007241
   HYPOTHETICAL 24.5 KD PROTEIN IN SAP185-BCK1 INTERGENIC REGION.
   3.3e-15:106:42
   SACCHAROMYCES CEREVISIAE (BAKER'S YEAST).
   P40857
F-HEMBA1007291
   RETINOIC ACID RECEPTOR RXR-BETA.
   0.0013:124:33
   HOMO SAPIENS (HUMAN).
   P28702
F-HEMBA1007332
   ALPHA-2A ADRENERGIC RECEPTOR (ALPHA-2A ADRENOCEPTOR) (ALPHA-2AAR).
   0.0024:130:34
   MUS MUSCULUS (MOUSE).
   Q01338
F-HEMBB1000106
   CELLULAR NUCLEIC ACID BINDING PROTEIN (CNBP).
   9.5e-09:99:33
   MUS MUSCULUS (MOUSE).
   P53996
F-HEMBB1000276
F-HEMBB1000309
F-HEMBB1000407
   TRANS-ACTING TRANSCRIPTIONAL PROTEIN ICP0 (VMW118 PROTEIN).
   0.38:99:34
   HERPES SIMPLEX VIRUS (TYPE 2 / STRAIN HG52).
   P28284
F-HEMBB1000447
   HISTIDINE-RICH GLYCOPROTEIN PRECURSOR.
   0.0076:80:31
   PLASMODIUM LOPHURAE.
   P04929
F-HEMBB1000542
   BETA-2 BUNGAROTOXIN B CHAIN PRECURSOR (BUNGAROTOXIN, B2 CHAIN).
   0.017:53:33
   BUNGARUS MULTICINCTUS (MANY-BANDED KRAIT).
   P00989
F-HEMBB1000567
   HISTIDINE-RICH GLYCOPROTEIN PRECURSOR.
   5.0e-05:131:29
   PLASMODIUM LOPHURAE.
   P04929
F-HEMBB1000642
   BASIC PROLINE-RICH PEPTIDE IB-1.
   0.0074:66:31
   HOMO SAPIENS (HUMAN).
   P04281
F-HEMBB1000668
   VEGETATIBLE INCOMPATIBILITY PROTEIN HET-E-1.
   7.3e-10:184:32
   PODOSPORA ANSERINA.
   Q00808
F-HEMBB1000679
   TRAM PROTEIN (TRANSLOCATING CHAIN-ASSOCIATING MEMBRANE PROTEIN).
   9.5e-73:204:69
   CANIS FAMILIARIS (DOG).
   Q01685
F-HEMBB1000881
   F-SPONDIN PRECURSOR.
   1.2e-23:191:37
   XENOPUS LAEVIS (AFRICAN CLAWED FROG).
   P35447
F-HEMBB1000905
   TRANSCRIPTIONAL REPRESSOR RCO-1.
   0.068:105:34
   NEUROSPORA CRASSA.
   P78706
F-HEMBB1001026
   ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
   1.3e-11:138:31
   SACCHAROMYCES CEREVISIAE (BAKER'S YEAST).
   P32802
F-HEMBB1001048
   SARCALUMENIN PRECURSOR.
   3.1e-20:151:32
   ORYCTOLAGUS CUNICULUS (RABBIT).
   P13666
F-HEMBB1001200
   HYPOTHETICAL GENE 51 MEMBRANE PROTEIN.
   1.0:66:27
   ICTALURID HERPESVIRUS 1 (CHANNEL CATFISH VIRUS) (CCV).
   Q00135
F-HEMBB1001407
   !!!! ALU SUBFAMILY SQ WARNING ENTRY !!!!
   2.0e-24:58:60
   HOMO SAPIENS (HUMAN).
   P39194
F-HEMBB1001530
   SLS1 PROTEIN PRECURSOR.
   0.0012:37:51
   YARROWIA LIPOLYTICA (CANDIDA LIPOLYTICA).
   Q99158
F-HEMBB1001547
   HYPOTHETICAL 71.7 KD PROTEIN F52H3.2 IN CHROMOSOME II.
   4.1e-49:200:55
   CAENORHABDITIS ELEGANS.
   Q20680
F-HEMBB1001573
   PROTEIN Q300.
   0.0055:27:62
   MUS MUSCULUS (MOUSE).
   Q02722
F-HEMBB1001847
   ACIDIC PROLINE-RICH PROTEIN PRECURSOR (CLONE PRP33).
   7.8e-05:166:31
   RATTUS NORVEGICUS (RAT).
   P04474
F-HEMBB1001959
   CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).
   8.8e-15:97:38
   PETROMYZON MARINUS (SEA LAMPREY).
   P25210
F-HEMBB1001978
   MICROCIN B17 PROCESSING PROTEIN MCBC.
   0.049:100:31
   ESCHERICHIA COLI.
   P23185
F-HEMBB1002039
   COLD SHOCK PROTEIN CSPB (FRAGMENT).
   0.98:32:40
   BACILLUS GLOBISPORUS.
   P41018
F-HEMBB1002041
   REGULATORY PROTEIN E2.
   0.010:145:35
   HUMAN PAPILLOMAVIRUS TYPE 47.
   P22420
F-HEMBB1002051
   FLI-1 ONCOGENE (ERGB TRANSCRIPTION FACTOR).
   0.0056:89:31
   HOMO SAPIENS (HUMAN).
   Q01543
F-HEMBB1002120
   UDP-N-ACETYLGLUCOSAMINE--PEPTIDE N-ACETYLGLUCOSAMINYLTRANSFERASE 110 KD SUBUNIT (EC 2.4.1.-) (O-GLCNAC TRANSFERASE P110 SUBUNIT).
   1.4e-08:154:30
   RATTUS NORVEGICUS (RAT).
   P56558
F-HEMBB1002162
   IMMEDIATE-EARLY PROTEIN IE180.
   0.86:130:31
   PSEUDORABIES VIRUS (STRAIN INDIANA-FUNKHAUSER / BECKER) (PRV).
   P11675
F-HEMBB1002228
   PTB-ASSOCIATED SPLICING FACTOR (PSF).
   0.00092:97:34
   HOMO SAPIENS (HUMAN).
   P23246
F-HEMBB1002245
   PROSTAGLANDIN F2-ALPHA RECEPTOR REGULATORY PROTEIN PRECURSOR (PROSTAGLANDIN F2-ALPHA RECEPTOR ASSOCIATED PROTEIN).
   2.5e-55:128:88
   RATTUS NORVEGICUS (RAT).
   Q62786
F-HEMBB1002302
   REGULATORY PROTEIN E2.
   0.042:100:37
   HUMAN PAPILLOMAVIRUS TYPE 25.
   P36787
F-HEMBB1002427
   FUCOSYLGLYCOPROTEIN ALPHA-N-ACETYLGALACTOSAMINYLTRANSFERASE (EC 2.4.1.40) (HISTO-BLOOD GROUP A TRANSFERASE) (A TRANSFERASE) / FUCOSYLGLYCOPROTEIN 3-ALPHA-GALACTOSYLTRANSFERASE (EC 2.4.1.37) (HISTO-BLOOD GROUP B TRANSFERASE) (B TRANSFERASE) (NAGAT).
   5.0e-15:53:54
   HOMO SAPIENS (HUMAN).
   P16442
F-HEMBB1002465
   ACYL-COA DEHYDROGENASE (EC 1.3.99.-).
   8.2e-35:162:50
   BACILLUS SUBTILIS.
   P45857
F-HEMBB1002661
   TRANSCRIPTION FACTOR HES-1 (C-HAIRY1).
   2.2e-18:159:40
   GALLUS GALLUS (CHICKEN).
   057337
F-HEMBB1002663
F-HEMBB1002693
   GAG POLYPROTEIN [CONTAINS: CORE PROTEIN P15; INNER COAT PROTEIN P12; CORE SHELL PROTEIN P30; NUCLEOPROTEIN P10].
   0.83:74:28
   DUPLAN MURINE LEUKEMIA VIRUS.
   P23090
F-MAMMA1000046
   !!!! ALU SUBFAMILY SB2 WARNING ENTRY !!!!
   2.3e-24:98:67
   HOMO SAPIENS (HUMAN).
   P39191
F-MAMMA1000102
   APOLIPOPROTEIN L PRECURSOR (APO-L).
   4.3e-22:213:34
   HOMO SAPIENS (HUMAN).
   O14791
F-MAMMA1000106
   PISTIL-SPECIFIC EXTENSIN-LIKE PROTEIN PRECURSOR (PELP).
   1.6e-07:99:31
   NICOTIANA TABACUM (COMMON TOBACCO).
   Q03211
F-MAMMA1000118
   HYPOTHETICAL 29.3 KD PROTEIN (ORF92).
   0.00059:155:30
   ORGYIA PSEUDOTSUGATA MULTICAPSID POLYHEDROSIS VIRUS (OPMNPV). O10341
F-MAMMA1000141
   !!!! ALU SUBFAMILY SX WARNING ENTRY !!!!
   0.00011:39:66
   HOMO SAPIENS (HUMAN).
   P39195
F-MAMMA1000204
   SYNAPTOTAGMIN III (SYTIII).
   5.9e-05:93:33
   MUS MUSCULUS (MOUSE).
   035681
F-MAMMA1000226
   GLUTENIN, HIGH MOLECULAR WEIGHT SUBUNIT DX5 PRECURSOR.
   4.6e-06:224:28
   TRITICUM AESTIVUM (WHEAT).
   P10388
F-MAMMA1000403
   COLLAGEN ALPHA 1(IX) CHAIN PRECURSOR (FRAGMENTS).
   1.1e-06:158:35
   GALLUS GALLUS (CHICKEN).
   P12106
F-MAMMA1000449
   SYNAPSINS IA AND IB (BRAIN PROTEIN 4.1).
   6.3e-05:137:32
   HOMO SAPIENS (HUMAN).
   P17600
F-MAMMA1000457
   NADH-CYTOCHROME B5 REDUCTASE (EC 1.6.2.2).
   7.6e-48:151:62
   BOS TAURUS (BOVINE).
   P07514
F-MAMMA1000473
   SPERM PROTAMINE P1.
   0.024:29:44
   DROMICIOPS AUSTRALIS (MONITO DEL MONTE) (DROMICIOPS GLIROIDES). P42132
F-MAMMA1000496
   HYPOTHETICAL 73.0 KD PROTEIN IN CLA4-MID1 INTERGENIC REGION.
   9.8e-09:188:26
   SACCHAROMYCES CEREVISIAE (BAKER'S YEAST).
   P48566
F-MAMMA1000528
   DNA BINDING PROTEIN S1FA.
   0.77:43:37
   ARABIDOPSIS THALIANA (MOUSE-EAR CRESS).
   P42551
F-MAMMA1000591
   SALIVARY PROLINE-RICH PROTEIN PO PRECURSOR (ALLELE S).
   0.018:88:32
   HOMO SAPIENS (HUMAN).
   P10163
F-MAMMA1000614
   HYPOTHETICAL 29.3 KD PROTEIN (ORF92).
   7.5e-08:148:36
   ORGYIA PSEUDOTSUGATA MULTICAPSID POLYHEDROSIS VIRUS (OPMNPV). O10341
F-MAMMA1000652
   !!!! ALU SUBFAMILY SB WARNING ENTRY !!!!
   5.3e-36:56:85
   HOMO SAPIENS (HUMAN).
   P39189
F-MAMMA1000681
   PROBABLE G PROTEIN-COUPLED RECEPTOR EDG-1.
   7.5e-41:167:51
   MUS MUSCULUS (MOUSE).
   008530
F-MAMMA1000706
   T-CELL RECEPTOR BETA CHAIN PRECURSOR (ANA 11).
   8.1e-10:135:38
   ORYCTOLAGUS CUNICULUS (RABBIT).
   P06333
F-MAMMA1000788
   HYPOTHETICAL 57.5 KD PROTEIN IN VMA7-RPS25A INTERGENIC REGION.
   2.0e-06:214:32
   SACCHAROMYCES CEREVISIAE (BAKERS YEAST).
   P53214
F-MAMMA1000810
   REGULATORY PROTEIN E2.
   0.0031:132:31
   HUMAN PAPILLOMAVIRUS TYPE 9.
   P36780
F-MAMMA1000814
   PROTEIN Q300.
   1.6e-05:27:66
   MUS MUSCULUS (MOUSE).
   Q02722
F-MAMMA1000881
   SERINE/THREONINE-PROTEIN KINASE SGK (EC 2.7.1.-) (SERUM/GLUCOCORTICOID-REGULATED KINASE).
   2.7e-10:81:45
   RATTUS NORVEGICUS (RAT).
   Q06226
F-MAMMA1000986
   INVOLUCRIN.
   0.95:125:24
   SUS SCROFA (PIG).
   P18175
F-MAMMA1000994
   CUTICLE COLLAGEN 2C (FRAGMENT).
   0.00062:97:34
   HAEMONCHUS CONTORTUS.
   P16252
F-MAMMA1001043
   MRC OX-45 SURFACE ANTIGEN PRECURSOR (BCM1 SURFACE ANTIGEN) (BLAST-1) (CD48).
   4.5e-05:162:25
   RATTUS NORVEGICUS (RAT).
   P10252
F-MAMMA1001066
   B-CELL GROWTH FACTOR PRECURSOR (BCGF-12 KD).
   2.6e-06:33:72
   HOMO SAPIENS (HUMAN).
   P20931
F-MAMMA1001094
   PUTATIVE GENERAL NEGATIVE REGULATOR OF TRANSCRIPTION C16C9.04C.
   1.1e-21:175:38
   SCHIZOSACCHAROMYCES POMBE (FISSION YEAST).
   Q09818
F-MAMMA1001141
   PTB-ASSOCIATED SPLICING FACTOR (PSF).
   0.13:196:27
   HOMO SAPIENS (HUMAN).
   P23246
F-MAMMA1001150
   PROTEIN KINASE C, MU TYPE (EC 2.7.1.-) (NPKC-MU).
   9.4e-89:256:67
   HOMO SAPIENS (HUMAN).
   Q15139
F-MAMMA1001237
   MONOCARBOXYLATE TRANSPORTER 2 (MCT 2).
   3.5e-19:103:43
   MESOCRICETUS AURATUS (GOLDEN HAMSTER).
   P53988
F-MAMMA1001284
   AUTOIMMUNE REGULATOR (APECED PROTEIN).
   0.027:178:30
   HOMO SAPIENS (HUMAN).
   043918
F-MAMMA1001310
   HYPOTHETICAL 57.3 KD TRP-ASP REPEATS CONTAINING PROTEIN IN POM152-REC114 INTERGENIC REGION.
   1.9e-14:151:31
   SACCHAROMYCES CEREVISIAE (BAKER'S YEAST).
   Q04225
F-MAMMA1001344
   MALE SPECIFIC SPERM PROTEIN MST84DC.
   0.16:35:42
   DROSOPHILA MELANOGASTER (FRUIT FLY).
   Q01644
F-MAMMA1001418
   HYPOTHETICAL PROTEIN HI0519.
   3.5e-27:181:38
   HAEMOPHILUS INFLUENZAE.
   P44742
F-MAMMA1001532
   ZINC FINGER PROTEIN 90 (ZFP-90) (ZINC FINGER PROTEIN NK10).
   1.1e-34:78:58
   MUS MUSCULUS (MOUSE).
   Q61967
F-MAMMA1001609
   TRANS-ACTING TRANSCRIPTIONAL PROTEIN ICP0 (VMW118 PROTEIN).
   0.20:137:27
   HERPES SIMPLEX VIRUS (TYPE 2 / STRAIN HG52).
   P28284
F-MAMMA1001615
   5E5 ANTIGEN.
   2.3e-07:205:34
   RATTUS NORVEGICUS (RAT).
   Q63003
F-MAMMA1001623
   EXCISION REPAIR PROTEIN ERCC-6 (COCKAYNE SYNDROME PROTEIN CSB).
   4.8e-30:90:77
   HOMO SAPIENS (HUMAN).
   Q03468
F-MAMMA1001634
   B-CELL GROWTH FACTOR PRECURSOR (BCGF-12 KD).
   6.1e-11:44:61
   HOMO SAPIENS (HUMAN).
   P20931
F-MAMMA1001893
   COLLAGEN ALPHA 1(XI) CHAIN PRECURSOR.
   0.0013:174:36
   HOMO SAPIENS (HUMAN).
   P12107
F-MAMMA1001901
   !!!! ALU SUBFAMILY SX WARNING ENTRY !!!!
   1.3e-21:65:66
   HOMO SAPIENS (HUMAN).
   P39195
F-MAMMA1001957
   VITELLINE MEMBRANE PROTEIN VM26AB PRECURSOR (PROTEIN TU-4) (PROTEIN SV23).
   0.0055:104:35
   DROSOPHILA MELANOGASTER (FRUIT FLY).
   P13238
F-MAMMA1001978
   D(4) DOPAMINE RECEPTOR (D(2C) DOPAMINE RECEPTOR).
   0.00030:101:44
   HOMO SAPIENS (HUMAN).
   P21917
F-MAMMA1002070
   PLASMINOGEN (EC 3.4.21.7) (FRAGMENT).
   1.4e-08:103:33
   RATTUS NORVEGICUS (RAT).
   Q01177
F-MAMMA1002080
   FMRFAMIDE-RELATED NEUROPEPTIDES PRECURSOR.
   8.2e-08:131:32
   LYMNAEA STAGNALIS (GREAT POND SNAIL).
   P42565
F-MAMMA1002087
   MALE SPECIFIC SPERM PROTEIN MST84DD.
   0.65:24:45
   DROSOPHILA MELANOGASTER (FRUIT FLY).
   Q01645
F-MAMMA1002091
   APYRASE PRECURSOR (EC 3.6.1.5) (ATP-DIPHOSPHATASE) (ADENOSINE DIPHOSPHATASE) (ADPASE) (ATP-DIPHOSPHOHYDROLASE).
   2.6e-24:155:43
   SOLANUM TUBEROSUM (POTATO).
   P80595
F-MAMMA1002095
   CALCIUM-TRANSPORTING ATPASE 1 (EC 3.6.1.38) (P-TYPE CALCIUM ATPASE).
   2.3e-58:213:56
   YARROWIA LIPOLYTICA (CANDIDA LIPOLYTICA).
   043108
F-MAMMA1002128
   COLLAGEN ALPHA 1(I) CHAIN (FRAGMENTS).
   9.9e-05:72:40
   RATTUS NORVEGICUS (RAT).
   P02454
F-MAMMA1002142
   NON-RECEPTOR TYROSINE KINASE SPORE LYSIS A (EC 2.7.1.112) (TYROSINE-PROTEIN KINASE 1).
   2.1e-13:149:34
   DICTYOSTELIUM DISCOIDEUM (SLIME MOLD).
   P18160
F-MAMMA1002165
   CONNECTIVE TISSUE GROWTH FACTOR PRECURSOR.
   8.4e-06:47:57
   HOMO SAPIENS (HUMAN).
   P29279
F-MAMMA1002205
   !!!! ALU SUBFAMILY J WARNING ENTRY !!!!
   5.9e-26:56:78
   HOMO SAPIENS (HUMAN).
   P39188
F-MAMMA1002224
   !!!! ALU SUBFAMILY SQ WARNING ENTRY !!!!
   3.7e-16:62:67
   HOMO SAPIENS (HUMAN).
   P39194
F-MAMMA1002234
   SIGNAL RECOGNITION PARTICLE 68 KD PROTEIN (SRP68).
   3.5e-105:242:85
   CANIS FAMILIARIS (DOG).
   Q00004
F-MAMMA1002586
   MANNOSYL-OLIGOSACCHARIDE ALPHA-1,2-MANNOSIDASE (EC 3.2.1.113) (MAN(9)-ALPHA-MANNOSIDASE) (ALPHA-MANNOSIDASE 1A).
   4.7e-24:203:35
   MUS MUSCULUS (MOUSE).
   P45700
F-MAMMA1002633
   !!!! ALU SUBFAMILY J WARNING ENTRY !!!!
   7.3e-27:49:73
   HOMO SAPIENS (HUMAN).
   P39188
F-MAMMA1003126
   SARCALUMENIN PRECURSOR.
   7.9e-30:156:35
   ORYCTOLAGUS CUNICULUS (RABBIT).
   P13666
F-NT2RM1000407
   LACTOSE OPERON REPRESSOR.
   1.4e-07:36:86
   ESCHERICHIA COLI.
   P03023
F-NT2RM1000462
   ATRIAL GLAND-SPECIFIC ANTIGEN PRECURSOR (AGSA).
   6.7e-11:85:41
   APLYSIA CALIFORNICA (CALIFORNIA SEA HARE).
   P15287
F-NT2RM1000542
   BETA-GALACTOSIDASE PRECURSOR (EC 3.2.1.23) (LACTASE) (ACID BETA-GALACTOSIDASE).
   3.5e-19:104:48
   FELIS SILVESTRIS CATUS (CAT).
   O19015
F-NT2RM1000580
   GLYCOSYLTRANSFERASE ALG2 (EC 2.4.1.-).
   3.4e-36:180:43
   SACCHAROMYCES CEREVISIAE (BAKER'S YEAST).
   P43636
F-NT2RM1000789
   T-CELL-SPECIFIC TRANSCRIPTION FACTOR 1 (TCF-1) (T-CELL FACTOR 1) (TRANSCRIPTION FACTOR-7).
   1.5e-40:112:75
   MUS MUSCULUS (MOUSE).
   Q00417
F-NT2RM1000855
   PROTEIN TRANSPORT PROTEIN SEC61 ALPHA SUBUNIT.
   2.5e-81:152:94
   CANIS FAMILIARIS (DOG).
   P38377
F-NT2RM1000858
   HYPOTHETICAL 36.7 KD PROTEIN AH6.2 IN CHROMOSOME II.
   3.1e-50:127:54
   CAENORHABDITIS ELEGANS.
   Q09201
F-NT2RM1000899
   MITOCHONDRIAL RNA SPLICING PROTEIN MSR4.
   6.6e-17:107:43
   SACCHAROMYCES CEREVISIAE (BAKER'S YEAST).
   P23500
F-NT2RM2000241
   DOUBLESEX PROTEIN, MALE-SPECIFIC.
   0.0021:64:32
   DROSOPHILA MELANOGASTER (FRUIT FLY).
   P23023
F-NT2RM2000306
   PUTATIVE GTP-BINDING PROTEIN W08E3.3.
   1.1e-69:198:69
   CAENORHABDITIS ELEGANS.
   P91917
F-NT2RM2000410
   BETA-LYTIC METALLOENDOPEPTIDASE PRECURSOR (EC 3.4.24.32) (BETA-LYTIC PROTEASE).
   0.73:118:31
   ACHROMOBACTER LYTICUS.
   P27458
F-NT2RM2000423
   BETA GALACTOSIDASE-RELATED PROTEIN PRECURSOR.
   1.3e-23:235:34
   HOMO SAPIENS (HUMAN).
   P16279
F-NT2RM2000497
   DNA-REPAIR PROTEIN COMPLEMENTING XP-D CELLS (XERODERMA PIGMENTOSUM GROUP D COMPLEMENTING PROTEIN) (DNA EXCISION REPAIR PROTEIN ERCC-2).
   9.4e-19:199:31
   CRICETULUS GRISEUS (CHINESE HAMSTER).
   Q60452
F-NT2RM2000514
   HYPOTHETICAL PROTEIN HI1558.
   7.7e-06:82:34
   HAEMOPHILUS INFLUENZAE.
   P45252
F-NT2RM2000565
   HYPOTHETICAL 85.7 KD PROTEIN C13G6.03 IN CHROMOSOME I.
   2.8e-57:232:52
   SCHIZOSACCHAROMYCES POMBE (FISSION YEAST).
   Q09782
F-NT2RM2000582
   PROTEIN Q300.
   0.066:13:84
   MUS MUSCULUS (MOUSE).
   Q02722
F-NT2RM2000589
   RAC-FAMILY SERINE/THREONINE KINASE HOMOLOG (EC 2.7.1.-).
   2.1e-07:90:32
   DICTYOSTELIUM DISCOIDEUM (SLIME MOLD).
   P54644
F-NT2RM2000622
   GLUTENIN, HIGH MOLECULAR WEIGHT SUBUNIT PW212 PRECURSOR.
   1.9e-07:133:35
   TRITICUM AESTIVUM (WHEAT).
   P08489
F-NT2RM2000632
   EXCISION REPAIR PROTEIN ERCC-6 (COCKAYNE SYNDROME PROTEIN CSB).
   4.6e-28:194:35
   HOMO SAPIENS (HUMAN).
   Q03468
F-NT2RM2000773
   MYC-ASSOCIATED ZINC FINGER PROTEIN (MAZI) (PUR-1) (ZF87).
   3.4e-24:156:47
   HOMO SAPIENS (HUMAN).
   P56270
F-NT2RM2001126
   NEURON-SPECIFIC X11 PROTEIN (FRAGMENT).
   1.5e-05:118:32
   MUS MUSCULUS (MOUSE).
   P98084
F-NT2RM2001558
   MAJOR FIBROUS SHEATH PROTEIN PRECURSOR (FSC1) (P82).
   1.9e-24:164:40
   MUS MUSCULUS (MOUSE).
   Q60662
F-NT2RM2001626
   HYPOTHETICAL 118.4 KD PROTEIN IN BAT2-DAL5 INTERGENIC REGION PRECURSOR.
   1.6e-09:206:33
   SACCHAROMYCES CEREVISIAE (BAKER'S YEAST).
   P47179
F-NT2RM2001643
   HYPOTHETICAL PROTEIN MJ1025.
   0.21:203:22
   METHANOCOCCUS JANNASCHII.
   Q58431
F-NT2RM2001738
   REGULATORY PROTEIN E2.
   0.0076:124:31
   HUMAN PAPILLOMAVIRUS TYPE 25.
   P36787
F-NT2RM2001767
   HOMEOTIC GENE REGULATOR (BRAHMA PROTEIN).
   0.0068:115:33
   DROSOPHILA MELANOGASTER (FRUIT FLY).
   P25439
F-NT2RM2001792
   FIBRINOGEN BETA CHAIN.
   4.3e-25:121:45
   BOS TAURUS (BOVINE).
   P02676
F-NT2RM2001818
   EXTENSIN PRECURSOR (PROLINE-RICH GLYCOPROTEIN).
   2.4e-06:192:32
   ZEA MAYS (MAIZE).
   P14918
F-NT2RM2001902
   SERINE/THREONINE-PROTEIN KINASE PAK-BETA (EC 2.7.1.-) (BETA-PAK) (P21-ACTIVATED KINASE 3) (P65-PAK).
   2.3e-52:250:45
   RATTUS NORVEGICUS (RAT).
   Q62829
F-NT2RM2001939
   PROBABLE G PROTEIN-COUPLEID RECEPTOR GPR19 (GPR-NGA).
   4.0e-97:204:92
   HOMO SAPIENS (HUMAN).
   Q15760
F-NT2RM2001941
   MUSCARINIC ACETYLCHOLINE RECEPTOR M4.
   1.0e-34:184:32
   HOMO SAPIENS (HUMAN).
   P08173
F-NT2RM4000100
   EBNA-1 NUCLEAR PROTEIN.
   1.7e-05:86:39
   EPSTEIN-BARR VIRUS (STRAIN B95-8) (HUMAN HERPESVIRUS 4).
   P03211
F-NT2RM4000115
   DNA-DIRECTED RNA POLYMERASE II LARGEST SUBUNIT (EC 2.7.7.6) (RNA POLYMERASE II SUBUNIT 1).
   9.5e-05:116:35
   SCHIZOSACCHAROMYCES POMBE (FISSION YEAST).
   P36594
F-NT2RM4000198
   BUTYROPHILIN PRECURSOR (BT).
   8.6e-14:162:33
   MUS MUSCULUS (MOUSE).
   Q62556
F-NT2RM4000284
   COLLAGEN ALPHA 2(VI) CHAIN (FRAGMENT).
   0.86:95:37
   HOMO SAPIENS (HUMAN).
   P12110
F-NT2RM4000295
   COLLAGEN ALPHA 1(XVI) CHAIN PRECURSOR.
   1.1e-08:229:34
   HOMO SAPIENS (HUMAN).
   Q07092
F-NT2RM4000326
   SH3 DOMAIN-BINDING PROTEIN 3BP-2.
   6.1e-05:187:31
   HOMO SAPIENS (HUMAN).
   P78314
F-NT2RM4000417
   SYNAPTOTAGMIN (P65).
   4.2e-08:72:52
   APLYSIA CALIFORNICA (CALIFORNIA SEA HARE).
   P41823
F-NT2RM4000444
   ANTIGEN PEPTIDE TRANSPORTER 1 (APT1) (PEPTIDE TRANSPORTER TAP1) (PEPTIDE TRANSPORTER PSF1) (PEPTIDE SUPPLY FACTOR 1) (PSF-1) (PEPTIDE TRANSPORTER INVOLVED IN ANTIGEN PROCESSING 1).
   1.1e-36:192:41
   HOMO SAPIENS (HUMAN).
   Q03518
F-NT2RM4000587
   COLLAGEN ALPHA 1(II) CHAIN (FRAGMENTS).
   1.8e-13:200:38
   BOS TAURUS (BOVINE).
   P02459
F-NT2RM4000593
F-NT2RM4000648
   K-GLYPICAN PRECURSOR.
   6.4e-67:180:68
   MUS MUSCULUS (MOUSE).
   P51655
F-NT2RM4000761
   CYTOCHROME C OXIDASE POLYPEPTIDE I (EC 1.9.3.1).
   2.3e-53:107:81
   RATTUS NORVEGICUS (RAT).
   P05503
F-NT2RM4000965
   PUTATIVE IMPORTIN BETA-4 SUBUNIT (KARYOPHERIN BETA-4 SUBUNIT).
   4.9e-14:188:34
   SCHIZOSACCHAROMYCES POMBE (FISSION YEAST).
   060100
F-NT2RM4000997
   HISTONE H1C (CLONE XLHW2).
   0.88:73:26
   XENOPUS LAEVIS (AFRICAN CLAWED FROG).
   P15866
F-NT2RM4001321
   HOMEOBOX PROTEIN HOX-A2.
   0.27:77:37
   GALLUS GALLUS (CHICKEN).
   Q08727
F-NT2RM4001325
   CHONDROITIN 6-SULFOTRANSFERASE (EC 2.8.2.17) (C6ST).
   3.8e-30:184:39
   GALLUS GALLUS (CHICKEN).
   Q92179
F-NT2RM4001377
   HYPOTHETICAL BHLF1 PROTEIN.
   5.9e-06:216:33
   EPSTEIN-BARR VIRUS (STRAIN B95-8) (HUMAN HERPESVIRUS 4).
   P03181
F-NT2RM4001735
   GNS 1 PROTEIN.
   0.0028:114:29
   SACCHAROMYCES CEREVISIAE (BAKER'S YEAST).
   P25358
F-NT2RM4001768
   PROBABLE OXIDOREDUCTASE (EC 1.-.-.-).
   8.6e-24:205:36
   STREPTOMYCES ANTIBIOTICUS.
   Q03326
F-NT2RM4001843
   BETA-GALACTOSIDASE PRECURSOR (EC 3.2.1.23) (LACTASE).
   4.6e-33:258:37
   XANTHOMONAS MANIHOTIS.
   P48982
F-NT2RM4002352
   BASEMENT MEMBRANE PROTEOGLYCAN PRECURSOR (PERLECAN HOMOLOG).
   1.0e-15:85:45
   CAENORHABDITIS ELEGANS.
   Q06561
F-NT2RP1000002
   GLYCINE-RICH CELL WALL STRUCTURAL PROTEIN (CLONE W10-1) (FRAGMENT).
   0.00011:24:62
   LYCOPERSICON ESCULENTUM (TOMATO).
   Q01157
F-NT2RP1000050
   A-AGGLUTININ ATTACHMENT SUBUNIT PRECURSOR.
   2.5e-07:198:33
   SACCHAROMYCES CEREVISIAE (BAKER'S YEAST).
   P32323
F-NT2RP1000181
   CYTOCHROME B5.
   4.4e-11:117:29
   SACCHAROMYCES CEREVISIAE (BAKER'S YEAST).
   P40312
F-NT2RP1000239
   TARNS-ACTING TRANSCRIPTIONAL PROTEIN ICP0 (VMW118 PROTEIN).
   7.8e-05:141:33
   HERPES SIMPLEX VIRUS (TYPE 2 / STRAIN HG52).
   P28284
F-NT2RP1000261
   ORM1 PROTEIN.
   2.2e-18:137:35
   SACCHAROMYCES CEREVISIAE (BAKER'S YEAST).
   P53224
F-NT2RP1000271
   ZINC FINGER PROTEIN 85 (ZINC FINGER PROTEIN HPF4) (HTF1).
   8.3e-81:194:70
   HOMO SAPIENS (HUMAN).
   Q03923
F-NT2RP1000300
   HYPOTHETICAL 57.1 KD PROTEIN IN MAP2-TEL1 INTERGENIC REGION.
   2.0e-07:202:24
   SACCHAROMYCES CEREVISIAE (BAKER'S YEAST).
   P38176
F-NT2RP1000325
   MITOCHONDRIAL PHOSPHATE CARRIER PROTEIN PRECURSOR.
   1.6e-55:86:81
   HOMO SAPIENS (HUMAN).
   Q00325
F-NT2RP1000448
   PROLINE-RICH PEPTIDE P-B.
   0.094:32:43
   HOMO SAPIENS (HUMAN).
   P02814
F-NT2RP1000465
   EBNA-1 NUCLEAR PROTEIN.
   3.1e-07:101:39
   EPSTEIN-BARR VIRUS (STRAIN B95-8) (HUMAN HERPESVIRUS 4).
   P03211
F-NT2RP1000468
   CCAAT-BINDING TRANSCRIPTION FACTOR SUBUNIT A (CBF-A) (NF-Y PROTEIN CHAIN B) (NF-YB) (CAAT-BOX DIVA BINDING PROTEIN SUBUNIT B).
   1.4e-14:97:38
   PETROMYZON MARINUS (SEA LAMPREY).
   P25210
F-NT2RP1000551
   INTERFERON-RELATED PROTEIN PC4 (TPA INDUCED SEQUENCE 7) (TIS7 PROTEIN).
   1.9e-33:221:41
   MUS MUSCULUS (MOUSE).
   P19182
F-NT2RP1000579
   SUCCINATE DEHYDROGENASE [UBIQUINONE] FLAVOPROTEIN SUBUNIT PRECURSOR (EC 1.3.5.1) (FP) (FLAVOPROTEIN SUBUNIT OF COMPLEX II).
   3.4e-68:247:62
   HOMO SAPIENS (HUMAN).
   P31040
F-NT2RP1000613
   CARBONIC ANHYDRASE VI (EC 4.2.1.1) (SALIVARY) (CARBONATE DEHYDRATASE VI).
   1.9e-19:137:37
   OVIS ARIES (SHEEP).
   P08060
F-NT2RP1000679
   EBNA-1 NUCLEAR PROTEIN.
   0.00055:54:50
   EPSTEIN-BARR VIRUS (STRAIN B95-8) (HUMAN HERPESVIRUS 4).
   P03211
F-NT2RP1000740
   TRANSCRIPTION INITIATION FACTOR TFIID 135 KD SUBUNIT (TAFII-135) (TAFII135) (TAFII-130) (TAFII130).
   0.071:71:45
   HOMO SAPIENS (HUMAN).
   000268
F-NT2RP1000903
   SPORE COAT PROTEIN SP96.
   0.016:124:26
   DICTYOSTELIUM DISCOIDEUM (SLIME MOLD).
   P14328
F-NT2RP1000981
   CELL SURFACE A33 ANTIGEN PRECURSOR.
   1.1e-08:196:28
   HOMO SAPIENS (HUMAN).
   Q99795
F-NT2RP1001004
   F-SPONDIN PRECURSOR.
   1.2e-11:155:31
   XENOPUS LAEVIS (AFRICAN CLAWED FROG).
   P35447
F-NT2RP1001020
   SPORULATION-SPECIFIC PROTEIN 1 (EC 2.7.1.-).
   2.2e-05:126:29
   SACCHAROMYCES CEREVISIAE (BAKER'S YEAST).
   P08458
F-NT2RP1001031
   PUTATIVE SERINE/THREONINE-PROTEIN KINASE PKWA (EC 2.7.1.-).
   5.8e-26:159:38
   THERMOMONOSPORA CURVATA.
   P49695
F-NT2RP1001563
   METALLOTHIONEIN-III (MT-III) (GROWTH INHIBITORY FACTOR) (GIF).
   0.00036:42:40
   BOS TAURUS (BOVINE).
   P37359
F-NT2RP2000092
   ZINC FINGER PROTEIN 136.
   2.9e-44:129:62
   HOMO SAPIENS (HUMAN).
   P52737
F-NT2RP2000178
   HOMEOTIC GENE REGULATOR (BRAHMA PROTEIN).
   0.0050:75:37
   DROSOPHILA MELANOGASTER (FRUIT FLY).
   P25439
F-NT2RP2000240
   PUTATIVE CUTICLE COLLAGEN C09G5.5.
   9.2e-08:137:34
   CAENORHABDITIS ELEGANS.
   Q09456
F-NT2RP2000394
   PROCYCLIC FORM SPECIFIC POLYPEPTIDE A-BETA PRECURSOR (PROCYCLIN) (PARP A-BETA).
   0.00019:28:64
   TRYPANOSOMA BRUCEI BRUCEI.
   P09791
F-NT2RP2000447
   GOLGIN-95.
   6.4e-25:55:67
   HOMO SAPIENS (HUMAN).
   Q08379
F-NT2RP2000479
   PROBABLE E5B PROTEIN.
   1.0:32:37
   HUMAN PAPILLOMAVIRUS TYPE 6B.
   P06461
F-NT2RP2000514
   AXONIN-1 PRECURSOR (AXONAL GLYCOPROTEIN TAG-1) (TRANSIENT AXONAL GLYCOPROTEIN 1).
   1.5e-18:201:33
   HOMO SAPIENS (HUMAN).
   Q02246
F-NT2RP2000533
   CORNICHON PROTEIN.
   5.6e-52:144:65
   DROSOPHILA MELANOGASTER (FRUIT FLY).
   P49858
F-NT2RP2000610
   CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).
   8.7e-15:97:38
   PETROMYZON MARINUS (SEA LAMPREY).
   P25210
F-NT2RP2000616
   PROCOLLAGEN ALPHA 1(I) CHAIN PRECURSOR.
   0.028:163:30
   MUS MUSCULUS (MOUSE).
   P11087
F-NT2RP2000649
   POTENTIAL CAAX PRENYL PROTEASE 1 (EC 3.4.24.-) (PRENYL PROTEIN- SPECIFIC ENDOPROTEASE 1) (PPSEP 1).
   9.5e-22:241:32
   SCHIZOSACCHAROMYCES POMBE (FISSION YEAST).
   Q10071
F-NT2RP2000663
   PANCREATIC HORMONE PRECURSOR (PANCREATIC POLYPEPTIDE) (PP).
   0.71:28:46
   GALLUS GALLUS (CHICKEN), AND MELEAGRIS GALLOPAVO (COMMON TURKEY).
   P01306
F-NT2RP2000694
   WISKOTT-ALDRICH SYNDROME PROTEIN HOMOLOG (WASP).
   8.8e-10:90:42
   MUS MUSCULUS (MOUSE).
   P70315
F-NT2RP2000712
   ZINC FINGER PROTEIN 85 (ZINC FINGER PROTEIN HPF4) (HTF1).
   2.3e-50:166:50
   HOMO SAPIENS (HUMAN).
   Q03923
F-NT2RP2000739
   ZINC FINGER PROTEIN 85 (ZINC FINGER PROTEIN HPF4) (HTF1).
   8.9e-45:180:43
   HOMO SAPIENS (HUMAN).
   Q03923
F-NT2RP2000818
   SYG1 PROTEIN.
   2.4e-14:164:35
   SACCHAROMYCES CEREVISIAE (BAKER'S YEAST).
   P40528
F-NT2RP2000903
   SKIN SECRETORY PROTEIN XP2 PRECURSOR (APEG PROTEIN).
   0.28:149:34
   XENOPUS LAEVIS (AFRICAN CLAWED FROG).
   P17437
F-NT2RP2001200
   MIC1 PROTEIN.
   1.8e-13:115:38
   SACCHAROMYCES CEREVISIAE (BAKER'S YEAST).
   P53258
F-NT2RP2001223
   CCAAT DISPLACEMENT PROTEIN (HOMEOBOX PROTEIN CLOX) (CLOX-1) (FRAGMENT).
   0.00017:92:35
   CANIS FAMILIARIS (DOG).
   P39881
F-NT2RP2001276
   NPDC-1 PROTEIN PRECURSOR.
   4.9e-35:96:71
   MUS MUSCULUS (MOUSE).
   Q64322
F-NT2RP2001388
   CECROPIN B PRECURSOR.
   0.98:31:51
   DROSOPHILA MELANOGASTER (FRUIT FLY).
   P14956
F-NT2RP2001469
   VEGETATIBLE INCOMPATIBILITY PROTEIN HET-E-1.
   6.0e-07:146:22
   PODOSPORA ANSERINA.
   Q00808
F-NT2RP2001480
   THROMBOSPONDIN 3 PRECURSOR.
   2.1e-100:209:88
   HOMO SAPIENS (HUMAN).
   P49746
F-NT2RP2001495
   HYPOTHETICAL 53.3 KD PROTEIN IN HXT8-CAN1 INTERGENIC REGION.
   3.1e-11:174:24
   SACCHAROMYCES CEREVISIAE (BAKER'S YEAST).
   P39981
F-NT2RP2001514
   PROBABLE CALCIUM-TRANSPORTING ATPASE 6 (EC 3.6.1.38).
   4.0e-18:163:36
   SACCHAROMYCES CEREVISIAE (BAKER'S YEAST).
   P39986
F-NT2RP2001529
   DEATH-ASSOCIATED PROTEIN KINASE 1 (EC 2.7.1.-) (DAP KINASE 1).
   4.4e-83:186:78
   HOMO SAPIENS (HUMAN).
   P53355
F-NT2RP2001538
   PAIRED AMPHIPATHIC HELIX PROTEIN.
   1.7e-06:152:26
   SACCHAROMYCES CEREVISIAE (BAKER'S YEAST).
   P22579
F-NT2RP2001562
   CLATHRIN LIGHT CHAIN B (BRAIN AND LYMPHOCYTE LCB).
   0.0093:124:27
   HOMO SAPIENS (HUMAN).
   P09497
F-NT2RP2001662
   5'-TG-3'INTERACTING FACTOR (HOMEOBOX PROTEIN TGIF).
   5.6e-36:146:57
   HOMO SAPIENS (HUMAN).
   Q15583
F-NT2RP2001755
   F-SPONDIN PRECURSOR.
   1.2e-33:84:89
   RATTUS NORVEGICUS (RAT).
   P35446
F-NT2RP2001769
   PROTEIN KINASE CEK1 (EC 2.7.1.-).
   1.3e-37:159:53
   SCHIZOSACCHAROMYCES POMBE (FISSION YEAST).
   P38938
F-NT2RP2001817
   HST1 PROTEIN (HOMOLOGOUS TO SIR2 PROTEIN 1).
   6.4e-32:85:48
   SACCHAROMYCES CEREVISIAE (BAKER'S YEAST).
   P53685
F-NT2RP2001878
   EXTENSIN PRECURSOR (CELL WALL HYDROXYPROLINE-RICH GLYCOPROTEIN).
   1.1e-06:173:28
   NICOTIANA TABACUM (COMMON TOBACCO).
   P13983
F-NT2RP2001903
   CALPAIN P94, LARGE [CATALYTIC] SUBUNIT (EC 3.4.22.17) (CALCIUM- ACTIVATED NEUTRAL PROTEINASE) (CANP) (P94 PROTEIN) (MUSCLE-SPECIFIC CALCIUM-ACTIVATED NEUTRAL PROTEASE 3 LARGE SUBUNIT).
   2.4e-10:110:37
   HOMO SAPIENS (HUMAN).
   P20807
F-NT2RP2001915
   TRANS-ACTING TRANSCRIPTIONAL PROTEIN ICP0 (VMW118 PROTEIN).
   0.0069:74:39
   HERPES SIMPLEX VIRUS (TYPE 2 / STRAIN HG52).
   P28284
F-NT2RP2001921
   TRANS-ACTING TRANSCRIPTIONAL PROTEIN ICP0 (P135 PROTEIN) (IER 2.9/ER2.6).
   0.016:51:45
   BOVINE HERPESVIRUS TYPE 1 (STRAIN K22).
   P29836
F-NT2RP2001948
   COLLAGEN ALPHA 1(X) CHAIN PRECURSOR.
   6.7e-08:121:37
   HOMO SAPIENS (HUMAN).
   Q03692
F-NT2RP2001956
   ORM1 PROTEIN.
   7.6e-17:106:36
   SACCHAROMYCES CEREVISIAE (BAKER'S YEAST).
   P53224
F-NT2RP2002015
   HOMEOBOX PROTEIN HOX-A2.
   0.12:77:37
   GALLUS GALLUS (CHICKEN).
   Q08727
F-NT2RP2002063
   HYPOTHETICAL 51.5 KD PROTEIN D2024.3 IN CHROMOSOME IV.
   3.2e-47:213:41
   CAENORHABDITIS ELEGANS.
   P49191
F-NT2RP2002188
   ACETYLCHOLINESTERASE PRECURSOR (EC 3.1.1.7) (ACHE).
   9.2e-15:109:36
   TORPEDO CALIFORNICA (PACIFIC ELECTRIC RAY).
   P04058
F-NT2RP2002232
   HYPOTHETICAL 85.7 KD PROTEIN C13G6.03 IN CHROMOSOME I.
   2.0e-12:92:50
   SCHIZOSACCHAROMYCES POMBE (FISSION YEAST).
   Q09782
F-NT2RP2002304
   GLYCINE-RICH CELL WALL STRUCTURAL PROTEIN (CLONE W10-1) (FRAGMENT).
   0.00059:16:68
   LYCOPERSICON ESCULENTUM (TOMATO).
   Q01157
F-NT2RP2002409
   COLLAGEN ALPHA 1(I) CHAIN (FRAGMENTS).
   0.00039:184:33
   BOS TAURUS (BOVINE).
   P02453
F-NT2RP2002510
   T-CELL RECEPTOR BETA CHAIN PRECURSOR (ANA 11).
   0.0010:97:37
   ORYCTOLAGUS CUNICULUS (RABBIT).
   P06333
F-NT2RP2002527
   CYTOCHROME B5.
   1.3e-11:77:38
   SACCHAROMYCES CEREVISIAE (BAKERS YEAST).
   P40312
F-NT2RP2002533
   DIHYDROPYRIDINE-SENSITIVE L-TYPE, CALCIUM CHANNEL ALPHA-2/DELTA SUBUNITS PRECURSOR.
   2.0e-37:165:42
   ORYCTOLAGUS CUNICULUS (RABBIT).
   P13806
F-NT2RP2002564
   EXTENSIN PRECURSOR (CELL WALL HYDROXYPROLINE-RICH GLYCOPROTEIN).
   4.7e-06:81:35
   NICOTIANA TABACUM (COMMON TOBACCO).
   P13983
F-NT2RP2002674
   SOLUBLE EPOXIDE HYDROLASE (SEH) (EC 3.3.2.3) (EPOXIDE HYDRATASE) (CYTOSOLIC EPOXIDE HYDROLASE) (CEH).
   2.4e-25:147:41
   HOMO SAPIENS (HUMAN).
   P34913
F-NT2RP2002721
   GLUCOSE 6-PHOSPHATE TRANSLOCASE.
   0.0073:88:26
   HOMO SAPIENS (HUMAN).
   043826
F-NT2RP2002824
   ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
   1.0e-16:139:36
   SACCHAROMYCES CEREVISIAE (BAKER'S YEAST).
   P32802
F-NT2RP2002942
   NEURAL CELL ADHESION MOLECULE L1 PRECURSOR (N-CAM L1).
   5.1e-18:153:30
   HOMO SAPIENS (HUMAN).
   P32004
F-NT2RP2002974
   HOMEOBOX PROTEIN SIX5 (DM LOCUS-ASSOCIATED HOMEODOMAIN PROTEIN HOMOLOG) (FRAGMENT).
   3.6e-80:187:84
   MUS MUSCULUS (MOUSE).
   P70178
F-NT2RP2002976
   HYPOTHETICAL 149.7 KD PROTEIN IN IRE1-KSP1 INTERGENIC REGION.
   2.8e-18:99:47
   SACCHAROMYCES CEREVISIAE (BAKER'S YEAST).
   P38800
F-NT2RP2003042
   PHOSPHATIDYLCHOLINE-STEROL ACYLTRANSFERASE PRECURSOR (EC 2.3.1.43) (LECITHIN-CHOLESTEROL ACYLTRANSFERASE) (PHOSPHOLIPID-CHOLESTEROL ACYLTRANSFERASE) (FRAGMENT).
   1.2e-41:135:57
   GALLUS GALLUS (CHICKEN).
   P53760
F-NT2RP2003138
   5'-TG-3'INTERACTING FACTOR (HOMEOBOX PROTEIN TGIF).
   3.3e-09:104:45
   MUS MUSCULUS (MOUSE).
   P70284
F-NT2RP2003179
   CARBON CATABOLITE DEREPRESSING PROTEIN KINASE (EC 2.7.1.-).
   7.2e-15:96:40
   SACCHAROMYCES CEREVISIAE (BAKER'S YEAST).
   P06782
F-NT2RP2003210
   LONG-CHAIN FATTY ACID TRANSPORT PROTEIN (FATP).
   6.2e-69:235:57
   MUS MUSCULUS (MOUSE).
   Q60714
F-NT2RP2003302
   ZINC FINGER PROTEIN 136.
   9.7e-52:140:52
   HOMO SAPIENS (HUMAN).
   P52737
F-NT2RP2003369
   SALIVARY PROLINE-RICH PROTEIN PO (ALLELE M) [CONTAINS: PEPTIDE P-D] (FRAGMENT).
   0.00020:87:32
   HOMO SAPIENS (HUMAN).
   P10161
F-NT2RP2003383
   LONG NEUROTOXIN 2 (TOXINS I AND V).
   0.86:38:39
   DENDROASPIS VIRIDIS (WESTERN GREEN MAMBA).
   P01395
F-NT2RP2003390
   NPL1 PROTEIN (SEC63 PROTEIN).
   1.1e-14:113:38
   SACCHAROMYCES CEREVISIAE (BAKER'S YEAST).
   P14906
F-NT2RP2003469
   MYO-INOSITOL TRANSPORTER 2.
   1.7e-09:148:28
   SACCHAROMYCES CEREVISIAE (BAKER'S YEAST).
   P30606
F-NT2RP2003545
   SERINE/THREONINE-PROTEIN KINASE NRK1 (EC 2.7.1.-) (N-RICH KINASE 1).
   9.2e-32:198:41
   SACCHAROMYCES CEREVISIAE (BAKER'S YEAST).
   P38692
F-NT2RP2003593
   PROLINE-RICH PROTEIN MP-2 PRECURSOR.
   0.00019:128:32
   MUS MUSCULUS (MOUSE).
   P05142
F-NT2RP2003599
   ATP-DEPENDENT BILE ACID PERMEASE.
   0.88:69:34
   SACCHAROMYCES CEREVISIAE (BAKER'S YEAST).
   P32386
F-NT2RP2003655
   HYPOTHETICAL 26.3 KD PROTEIN IN OYE2-GND1 INTERGENIC REGION.
   2.9e-16:93:47
   SACCHAROMYCES CEREVISIAE (BAKER'S YEAST).
   P38869
F-NT2RP2003664
   HYPOTHETICAL 15.7 KD PROTEIN IN NUP85-SSC1 INTERGENIC REGION.
   5.6e-08:121:32
   SACCHAROMYCES CEREVISIAE (BAKER'S YEAST).
   P47111
F-NT2RP2003931
   ACROSIN PRECURSOR (EC 3.4.21.10).
   0.38:20:70
   HOMO SAPIENS (HUMAN).
   P10323
F-NT2RP2003940
   ZINC FINGER PROTEIN 85 (ZINC FINGER PROTEIN HPF4) (HTF1).
   1.3e-84:126:74
   HOMO SAPIENS (HUMAN).
   Q03923
F-NT2RP2003950
   TRANS-ACTING TRANSCRIPTIONAL PROTEIN ICP0 (IMMEDIATE-EARLY PROTEIN IE110) (VMW110) (ALPHA-0 PROTEIN).
   1.5e-05:134:33
   HERPES SIMPLEX VIRUS (TYPE 1 / STRAIN 17).
   P08393
F-NT2RP2004069
   HYPOTHETICAL 26.4 KD PROTEIN EEED8.8 IN CHROMOSOME II.
   4.3e-13:68:54
   CAENORHABDITIS ELEGANS.
   Q09297
F-NT2RP2004108
   ZINC FINGER PROTEIN 136.
   8.6e-47:126:67
   HOMO SAPIENS (HUMAN).
   P52737
F-NT2RP2004141
   SKIN SECRETORY PROTEIN XP2 PRECURSOR (APEG PROTEIN).
   0.013:127:35
   XENOPUS LAEVIS (AFRICAN CLAWED FROG).
   P17437
F-NT2RP2004179
   GLYCEROPHOSPHORYL DIESTER PHOSPHODIESTERASE (EC 3.1.4.46) (GLYCEROPHOSPHODIESTER PHOSPHODIESTERASE).
   5.9e-10:110:36
   ESCHERICHIA COLI.
   P10908
F-NT2RP2004205
   MYELIN-OLIGODENDROCYTE GLYCOPROTEIN PRECURSOR.
   4.6e-10:99:34
   HOMO SAPIENS (HUMAN).
   Q16653
F-NT2RP2004447
   PROCOLLAGEN ALPHA 2(I) CHAIN PRECURSOR.
   0.86:48:37
   MUS MUSCULUS (MOUSE).
   Q01149
F-NT2RP2004495
   HYPOTHETICAL 53.3 KD PROTEIN IN HXT8-CAN1 INTERGENIC REGION.
   0.031:135:31
   SACCHAROMYCES CEREVISIAE (BAKER'S YEAST).
   P39981
F-NT2RP2004524
   HYPOTHETICAL 18.7 KD PROTEIN IN HMS1-ABF2 INTERGENIC REGION.
   0.042:96:23
   SACCHAROMYCES CEREVISIAE (BAKER'S YEAST).
   Q04767
F-NT2RP2004556
   SALIVARY PROLINE-RICH PROTEIN II-1 (FRAGMENT).
   0.0082:87:35
   HOMO SAPIENS (HUMAN).
   P81489
F-NT2RP2004606
   METALLOPROTEINASE INHIBITOR 1 PRECURSOR (TIMP-1) (ERYTHROID POTENTIATING ACTIVITY) (EPA) (TISSUE INHIBITOR OF METALLOPROTEINASES) (FIBROBLAST COLLAGENASE INHIBITOR) (COLLAGENASE INHIBITOR).
   2.2e-57:163:73
   HOMO SAPIENS (HUMAN).
   P01033
F-NT2RP2004648
   BETA-GALACTOSIDASE PRECURSOR (EC 3.2.1.23) (LACTASE) (ACID BETA-GALACTOSIDASE).
   3.2e-25:90:62
   FELIS SILVESTRIS CATUS (CAT).
   O19015
F-NT2RP2004670
   CALCIUM/CALMODULIN-DEPENDENT PROTEIN KINASE TYPE I (EC 2.7.1.123) (CAM KINASE I).
   6.6e-14:108:34
   RATTUS NORVEGICUS (RAT).
   Q63450
F-NT2RP2004794
   HYPOTHETICAL 24.5 KD PROTEIN IN SAP185-BCK1 INTERGENIC REGION.
   5.7e-11:140:31
   SACCHAROMYCES CEREVISIAE (BAKER'S YEAST).
   P40857
F-NT2RP2004837
F-NT2RP2004847
   ADULT ENHANCER FACTOR 1 (AEF-1).
   7.9e-09:81:37
   DROSOPHILA MELANOGASTER (FRUIT FLY).
   P39413
F-NT2RP2005027
   GLUCOSE TRANSPORTER TYPE 3, BRAIN.
   3.6e-64:130:96
   HOMO SAPIENS (HUMAN).
   P11169
F-NT2RP2005069
   CCAAT DISPLACEMENT PROTEIN (CDP) (CDP2) (FRAGMENT).
   0.22:116:32
   RATTUS NORVEGICUS (RAT).
   P53565
F-NT2RP2005163
   ANTER-SPECIFIC PROLINE-RICH PROTEIN APG PRECURSOR.
   5.3e-06:70:38
   ARABIDOPSIS THALIANA (MOUSE-EAR CRESS).
   P40602
F-NT2RP2005181
   HIGH-AFFINITY CATIONIC AMINO ACID TRANSPORTER-1 (CAT-1) (CAT1) (SYSTEM Y+ BASIC AMINO ACID TRANSPORTER) (ECOTROPIC RETROVIRAL LEUKEMIA RECEPTOR HOMOLOG) (ERR) (ECOTROPIC RETROVIRUS RECEPTOR HOMOLOG).
   4.2e-54:153:69
   HOMO SAPIENS (HUMAN).
   P30825
F-NT2RP2005247
   EXTENSIN PRECURSOR (PROLINE-RICH GLYCOPROTEIN).
   2.0e-11:106:35
   SORGHUM VULGARE (SORGHUM).
   P24152
F-NT2RP2005378
   A-AGGLUTININ ATTACHMENT SUBUNIT PRECURSOR.
   0.11:97:32
   SACCHAROMYCES CEREVISIAE (BAKER'S YEAST).
   P32323
F-NT2RP2005391
   G-BOX BINDING FACTOR (GBF).
   5.1e-10:156:30
   DICTYOSTELIUM DISCOIDEUM (SLIME MOLD).
   P36417
F-NT2RP2005425
   M PROTEIN, SEROTYPE 49 PRECURSOR.
   2.1e-05:183:27
   STREPTOCOCCUS PYOGENES.
   P16947
F-NT2RP2005463
   OVOMUCOID (FRAGMENT).
   1.0:21:52
   BAMBUSICOLA THORACICA (CHINESE BAMBOO-PARTRIDGE).
   P52259
F-NT2RP2005514
   MOBC PROTEIN.
   1.0:26:53
   THIOBACILLUS FERROOXIDANS.
   P22899
F-NT2RP2005535
   ZINC FINGER PROTEIN 85 (ZINC FINGER PROTEIN HPF4) (HTF1).
   3.8e-92:243:69
   HOMO SAPIENS (HUMAN).
   Q03923
F-NT2RP2005541
   N-ACETYLGLUCOSAMINE-6-SULFATASE PRECURSOR (EC 3.1.6.14) (G6S) (GLUCOSAMINE-6-SULFATASE).
   8.8e-16:78:51
   HOMO SAPIENS (HUMAN).
   P15586
F-NT2RP2005597
   DOLICHYL-PHOSPHATE-MANNOSE--PROTEIN MANNOSYLTRANSFERASE 4 (EC
   2.4.1.109).
   7.4e-13:99:34
   SACCHAROMYCES CEREVISIAE (BAKER'S YEAST).
   P46971
F-NT2RP2005632
   ADENYLATE CYCLASE, TYPE V (EC 4.6.1.1) (ATP PYROPHOSPHATE-LYASE) (CA(2+)-INHIBITABLE ADENYLYL CYCLASE).
   3.0e-05:73:43
   CANIS FAMILIARIS (DOG).
   P30803
F-NT2RP2005666
   HEPATOCYTE NUCLEAR FACTOR 3-BETA (HNF-3B).
   0.086:105:31
   MUS MUSCULUS (MOUSE).
   P35583
F-NT2RP2005774
   ZINC FINGER PROTEIN 136.
   7.8e-33:128:57
   HOMO SAPIENS (HUMAN).
   P52737
F-NT2RP2005878
   PUTATIVE STEROID DEHYDROGENASE KIK-I (EC 1.1.1.-).
   6.8e-23:96:48
   MUS MUSCULUS (MOUSE).
   070503
F-NT2RP2005883
   DOPAMINE-BETA-MONOOXYGENASE PRECURSOR (EC 1.14.17.1) (DOPAMINE BETA- HYDROXYLASE) (DBH).
   6.4e-23:185:32
   RATTUS NORVEGICUS (RAT).
   Q05754
F-NT2RP2005887
   DNA-DIRECTED RNA POLYMERASE SUBUNIT K (EC 2.7.7.6).
   1.0:40:30
   METHANOCOCCUS JANNASCHII.
   Q57650
F-NT2RP2005941
   SKIN SECRETORY PROTEIN XP2 PRECURSOR (APEG PROTEIN).
   3.5e-08:136:32
   XENOPUS LAEVIS (AFRICAN CLAWED FROG).
   P17437
F-NT2RP2005994
   HYPOTHETICAL 51.5 KD PROTEIN D2024.3 IN CHROMOSOME IV.
   4.4e-36:144:47
   CAENORHABDITIS ELEGANS.
   P49191
F-NT2RP2006004
   BONE PROTEOGLYCAN II PRECURSOR (PG-S2) (DECORIN) (PG40) (DERMATAN SULFATE PROTEOGLYCAN-II) (DSPG).
   0.030:28:50
   RATTUS NORVEGICUS (RAT).
   Q01129
F-NT2RP2006042
   HYPOTHETICAL PROTEIN KIAA0144.
   1.2e-22:228:39
   HOMO SAPIENS (HUMAN).
   Q14157
F-NT2RP2006092
   TRANSCRIPTIONAL ACTIVATOR FE65.
   3.1e-27:101:54
   RATTUS NORVEGICUS (RAT).
   P46933
F-NT2RP2006099
   SKIN SECRETORY PROTEIN XP2 PRECURSOR (APEG PROTEIN).
   7.0e-07:123:34
   XENOPUS LAEVIS (AFRICAN CLAWED FROG).
   P17437
F-NT2RP2006134
   TRANS-ACTING TRANSCRIPTIONAL PROTEIN ICP0 (EARLY PROTEIN 0) (EP0).
   0.0041:118:30
   PSEUDORABIES VIRUS (STRAIN INDIANA-FUNKHAUSER / BECKER) (PRV).
   P29129
F-NT2RP2006269
   DOLICHYL-PHOSPHATE-MANNOSE--PROTEIN MANNOSYLTRANSFERASE 4 (EC 2.4.1.109).
   6.3e-17:119:36
   SACCHAROMYCES CEREVISIAE (BAKER'S YEAST).
   P46971
F-NT2RP2006512
   GNS1 PROTEIN.
   8.6e-14:186:30
   SACCHAROMYCES CEREVISIAE (BAKER'S YEAST).
   P25358
F-NT2RP3000011
   PUTATIVE SERINE/THREONINE-PROTEIN KINASE PKWA (EC 2.7.1.-).
   2.9e-12:137:32
   THERMOMONOSPORA CURVATA.
   P49695
F-NT2RP3000022
   SERINE/THREONINE-PROTEIN KINASE MAK (EC 2.7.1.-) (MALE GERM CELL-ASSOCIATED KINASE).
   1.6e-47:121:79
   RATTUS NORVEGICUS (RAT).
   P20793
F-NT2RP3000059
   COLLAGEN ALPHA 1(III) CHAIN.
   1.5e-05:211:33
   BOS TAURUS (BOVINE).
   P04258
F-NT2RP3000063
   EXTENSIN PRECURSOR (PROLINE-RICH GLYCOPROTEIN).
   4.2e-23:230:28
   ZEA MAYS (MAIZE).
   P14918
F-NT2RP3000125
   CARBOXYPEPTIDASE KEX1 PRECURSOR (EC 3.4.16.6) (CARBOXYPEPTIDASE D).
   2.3e-08:110:30
   SACCHAROMYCES CEREVISIAE (BAKER'S YEAST).
   P09620
F-NT2RP3000148
   ZINC FINGER PROTEIN 133.
   1.4e-34:84:48
   HOMO SAPIENS (HUMAN).
   P52736
F-NT2RP3000169
   SMALL PROLINE-RICH PROTEIN 2-1.
   0.00092:14:57
   HOMO SAPIENS (HUMAN).
   P35326
F-NT2RP3000171
   24.1 KD PROTEIN IN VMA12-APN1 INTERGENIC REGION.
   4.6e-10:134:32
   SACCHAROMYCES CEREVISIAE (BAKER'S YEAST).
   P28707
F-NT2RP3000172
   CALCIUM/CALMODULIN-DEPENDENT PROTEIN KINASE TYPE I (EC 2.7.1.123) (CAM KINASE I).
   1.8e-33:161:42
   RATTUS NORVEGICUS (RAT).
   Q63450
F-NT2RP3000201
   SERINE/THREONINE-PROTEIN KINASE MIG-15 (EC 2.7.1.-).
   4.1e-79:254:64
   CAENORHABDITIS ELEGANS.
   Q23356
F-NT2RP3000232
   ZINC FINGER PROTEIN 184 (FRAGMENT).
   8.5e-23:119:45
   HOMO SAPIENS (HUMAN).
   Q99676
F-NT2RP3000304
   LOW-DENSITY LIPOPROTEIN RECEPTOR-RELATED PROTEIN 1 PRECURSOR (LRP) (ALPHA-2-MACROGLOBULIN RECEPTOR) (A2MR) (APOLIPOPROTEIN E RECEPTOR) (APOER) (CD91).
   9.8e-36:172:43
   HOMO SAPIENS (HUMAN).
   Q07954
F-NT2RP3000378
   PAIRED AMPHIPATHIC HELIX PROTEIN.
   2.7e-26:186:36
   SACCHAROMYCES CEREVISIAE (BAKER'S YEAST).
   P22579
F-NT2RP3000427
   5E5 ANTIGEN.
   0.086:204:31
   RATTUS NORVEGICUS (RAT).
   Q63003
F-NT2RP3000436
   PROBABLE PROTEIN DISULFIDE ISOMERASE P5 PRECURSOR (EC 5.3.4.1).
   1.3e-23:106:33
   CAENORHABDITIS ELEGANS.
   Q11067
F-NT2RP3000444
   TRANSCRIPTION INITIATION FACTOR TFIID 135 KD SUBUNIT (TAFII-135) (TAFII135) (TAFII-130) (TAFII130).
   0.00052:166:36
   HOMO SAPIENS (HUMAN).
   000268
F-NT2RP3000460
   PROTEIN TRANSPORT PROTEIN SEC61 ALPHA SUBUNIT.
   1.0e-98:194:100
   RATTUS NORVEGICUS (RAT).
   P38378
F-NT2RP3000481
   NONSENSE-MEDIATED MRNA DECAY PROTEIN 5.
   7.4e-19:217:29
   SACCHAROMYCES CEREVISIAE (BAKER'S YEAST).
   P46970
F-NT2RP3000616
   BONE PROTEOGLYCAN II PRECURSOR (PG-S2) (DECORIN).
   1.2e-13:115:33
   BOS TAURUS (BOVINE).
   P21793
F-NT2RP3000645
   SKIN SECRETORY PROTEIN XP2 PRECURSOR (APEG PROTEIN).
   2.3e-10:237:30
   XENOPUS LAEVIS (AFRICAN CLAWED FROG).
   P17437
F-NT2RP3000652
   ZINC FINGER PROTEIN 85 (ZINC FINGER PROTEIN HPF4) (HTF1).
   3.1e-106:283:67
   HOMO SAPIENS (HUMAN).
   Q03923
F-NT2RP3000676
   ADP,ATP CARRIER PROTEIN, HEART/SKELETAL MUSCLE ISOFORM T1 (ADP/ATP TRANSLOCASE 1) (ADENINE NUCLEOTIDE TRANSLOCATOR 1) (ANT 1).
   7.4e-07:102:32
   HOMO SAPIENS (HUMAN).
   P12235
F-NT2RP3000677
   MHC CLASS II REGULATORY FACTOR RFX1 (RFX) (ENHANCER FACTOR C) (EF-C).
   1.5e-27:66:54
   HOMO SAPIENS (HUMAN).
   P22670
F-NT2RP3000721
   HYPOTHETICAL 62.5 KD PROTEIN IN SEC53-ACT1 INTERGENIC REGION.
   1.6e-22:208:32
   SACCHAROMYCES CEREVISIAE (BAKER'S YEAST).
   P43560
F-NT2RP3000789
   ONCONEURAL VENTRAL ANTIGEN-1 (NOVA-1) (PARANEOPLASTIC RI ANTIGEN) (VENTRAL NEURON-SPECIFIC PROTEIN 1).
   1.0e-07:190:26
   HOMO SAPIENS (HUMAN).
   P51513
F-NT2RP3000818
   HYPOTHETICAL 67.5 KD PROTEIN IN APE1/LAP4-CWP1 INTERGENIC REGION.
   5.9e-05:100:32
   SACCHAROMYCES CEREVISIAE (BAKER'S YEAST).
   P34248
F-NT2RP3000820
   BIOTIN SYNTHASE (EC 2.8.1.6) (BIOTIN SYNTHETASE).
   0.92:97:26
   SYNECHOCYSTIS SP. (STRAIN PCC 6803).
   P73538
F-NT2RP3000838
   SKIN SECRETORY PROTEIN XP2 PRECURSOR (APEG PROTEIN).
   6.4e-07:231:31
   XENOPUS LAEVIS (AFRICAN CLAWED FROG).
   P17437
F-NT2RP3000871
   COLLAGEN ALPHA 1(I) CHAIN (FRAGMENTS).
   2.8e-07:221:33
   RATTUS NORVEGICUS (RAT).
   P02454
F-NT2RP3000907
   PROBABLE CALCIUM-TRANSPORTING ATPASE 6 (EC 3.6.1.38).
   2.2e-41:104:48
   SACCHAROMYCES CEREVISIAE (BAKER'S YEAST).
   P39986
F-NT2RP3000921
   BASEMENT MEMBRANE-SPECIFIC HEPARAN SULFATE PROTEOGLYCAN CORE PROTEIN PRECURSOR (HSPG) (PERLECAN) (PLC).
   4.5e-08:149:31
   HOMO SAPIENS (HUMAN).
   P98160
F-NT2RP3001012
   HISTIDINE-RICH GLYCOPROTEIN PRECURSOR.
   5.5e-06:37:51
   PLASMODIUM LOPHURAE.
   P04929
F-NT2RP3001044
   RNA POLYMERASE PRINCIPAL SIGMA FACTOR HRDA.
   0.10:61:31
   STREPTOMYCES COELICOLOR.
   P18182
F-NT2RP3001061
   GLYCOPROTEIN X PRECURSOR.
   0.00011:140:27
   EQUINE HERPESVIRUS TYPE 1 (STRAIN AB4P) (EHV-1).
   P28968
F-NT2RP3001159
   A-AGGLUTININ ATTACHMENT SUBUNIT PRECURSOR.
   1.1e-09:249:32
   SACCHAROMYCES CEREVISIAE (BAKER'S YEAST).
   P32323
F-NT2RP3001170
   POU DOMAIN PROTEIN 1 (DJPOU1).
   0.020:173:29
   DUGESIA JAPONICA (PLANARIAN).
   P31370
F-NT2RP3001195
   GALACTOSE-PROTON SYMPORT (GALACTOSE TRANSPORTER).
   1.2e-14:180:30
   ESCHERICHIA COLI.
   P37021
F-NT2RP3001240
   PROTEIN TRANSPORT PROTEIN SEC61 ALPHA SUBUNIT.
   3.1e-118:229:88
   RATTUS NORVEGICUS (RAT).
   P38378
F-NT2RP3001271
   EBNA-1 NUCLEAR PROTEIN.
   2.3e-08:113:45
   EPSTEIN-BARR VIRUS (STRAIN B95-8) (HUMAN HERPESVIRUS 4).
   P03211
F-NT2RP3001322
   PROBABLE CALCIUM-TRANSPORTING ATPASE 3 (EC 3.6.1.38) (ENDOPLASMIC RETICULUM CA2+-ATPASE).
   1.7e-23:222:29
   SACCHAROMYCES CEREVISIAE (BAKER'S YEAST).
   P39524
F-NT2RP3001388
   SYNAPTOTAGMIN IV.
   4.8e-19:168:38
   RATTUS NORVEGICUS (RAT).
   P50232
F-NT2RP3001542
   TUMOR NECROSIS FACTOR, ALPHA-INDUCED PROTEIN 1, ENDOTHELIAL (B12 PROTEIN).
   2.7e-12:132:37
   HOMO SAPIENS (HUMAN).
   Q13829
F-NT2RP3001560
   SYNAPSINS IA AND IB.
   0.59:104:35
   BOS TAURUS (BOVINE).
   P17599
F-NT2RP3001592
   GLYCINE-RICH CELL WALL STRUCTURAL PROTEIN 2 PRECURSOR.
   1.3e-11:75:46
   ORYZA SATIVA (RICE).
   P29834
F-NT2RP3001650
   CCAAT DISPLACEMENT PROTEIN (HOMEOBOX PROTEIN CLOX) (CLOX-1) (FRAGMENT).
   0.23:119:36
   CANIS FAMILIARIS (DOG).
   P39881
F-NT2RP3001685
   HYPOTHETICAL PROTEIN IN MMSB 3'REGION (ORF1) (FRAGMENT).
   2.2e-48:207:48
   PSEUDOMONAS AERUGINOSA.
   P28812
F-NT2RP3001738
   CYTOCHROME B5.
   9.5e-13:133:33
   ORYCTOLAGUS CUNICULUS (RABBIT).
   P00169
F-NT2RP3001754
   SYNAPSINS IA AND IB (BRAIN PROTEIN 4.1).
   7.9e-05:117:29
   HOMO SAPIENS (HUMAN).
   P17600
F-NT2RP3001858
   CUTICLE COLLAGEN 2.
   0.030:118:35
   CAENORHABDITIS ELEGANS.
   P17656
F-NT2RP3001976
   ZINC FINGER PROTEIN 140.
   7.8e-24:122:52
   HOMO SAPIENS (HUMAN).
   P52738
F-NT2RP3002015
   PROCOLLAGEN ALPHA 1(I) CHAIN PRECURSOR.
   0.018:224:30
   GALLUS GALLUS (CHICKEN).
   P02457
F-NT2RP3002160
   SKIN SECRETORY PROTEIN XP2 PRECURSOR (APEG PROTEIN).
   0.0058:206:29
   XENOPUS LAEVIS (AFRICAN CLAWED FROG).
   P17437
F-NT2RP3002281
   HETEROGENEOUS NUCLEAR RIBONUCLEOPROTEIN F (HNRNP F).
   1.3e-14:86:40
   HOMO SAPIENS (HUMAN).
   P52597
F-NT2RP3002286
   ETS-DOMAIN TRANSCRIPTION FACTOR ERF.
   0.65:128:29
   HOMO SAPIENS (HUMAN).
   P50548
F-NT2RP3002311
   BETA-GALACTOSIDASE PRECURSOR (EC 3.2.1.23) (LACTASE) (ACID BETA-GALACTOSIDASE).
   6.1e-46:172:54
   FELIS SILVESTRIS CATUS (CAT).
   O19015
F-NT2RP3002324
   HYPOTHETICAL 13.6 KD PROTEIN IN SPT4-ROM1 INTERGENIC REGION.
   0.012:23:65
   SACCHAROMYCES CEREVISIAE (BAKER'S YEAST).
   P53245
F-NT2RP3002342
   HYPOTHETICAL 53.3 KD PROTEIN IN HXT8-CAN1 INTERGENIC REGION.
   1.8e-13:219:26
   SACCHAROMYCES CEREVISIAE (BAKER'S YEAST).
   P39981
F-NT2RP3002353
   REGULATORY PROTEIN E2.
   0.0027:167:31
   HUMAN PAPILLOMAVIRUS TYPE 8.
   P06422
F-NT2RP3002409
   MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).
   9.9e-09:93:34
   NEUROSPORA CRASSA.
   P23231
F-NT2RP3002411
   PUTATIVE STEROID DEHYDROGENASE KIK-I (EC 1.1.1.-).
   5.6e-107:254:80
   MUS MUSCULUS (MOUSE).
   070503
F-NT2RP3002448
   TRANSCRIPTION INITIATION FACTOR TFIID 135 KD SUBUNIT (TAFII-135) (TAFII135) (TAFII-130) (TAFII130).
   1.5e-05:163:33
   HOMO SAPIENS (HUMAN).
   000268
F-NT2RP3002571
   HYPOTHETICAL 116.3 KD PROTEIN C26F1.09 IN CHROMOSOME I.
   6.4e-23:172:33
   SCHIZOSACCHAROMYCES POMBE (FISSION YEAST).
   Q10496
F-NT2RP3002664
   CYCLIC-AMP-DEPENDENT TRANSCRIPTION FACTOR ATF-6 (FRAGMENT).
   0.062:47:29
   HOMO SAPIENS (HUMAN).
   P18850
F-NT2RP3002721
   CITRATE SYNTHASE, MITOCHONDRIAL PRECURSOR (EC 4.1.3.7).
   6.2e-140:283:92
   SUS SCROFA (PIG).
   P00889
F-NT2RP3002737
   VOLTAGE-GATED POTASSIUM CHANNEL PROTEIN KV1.9.
   4.1e-40:136:61
   MUS MUSCULUS (MOUSE).
   P97414
F-NT2RP3002738
   SALIVARY PROLINE-RICH PROTEIN PO PRECURSOR (ALLELE S).
   0.029:195:28
   HOMO SAPIENS (HUMAN).
   P10163
F-NT2RP3002790
   PROLINE-RICH PROTEIN MP-2 PRECURSOR.
   1.7e-08:130:36
   MUS MUSCULUS (MOUSE).
   P05142
F-NT2RP3002836
   TRANSMEMBRANE PROTEIN SEX PRECURSOR.
   8.9e-24:119:43
   HOMO SAPIENS (HUMAN).
   P51805
F-NT2RP3002887
   SALIVARY PROLINE-RICH PROTEIN PRECURSOR (CLONE CP7) [CONTAINS: BASIC PEPTIDE P-F] (FRAGMENT).
   2.9e-11:198:34
   HOMO SAPIENS (HUMAN).
   P02812
F-NT2RP3002900
   COP-COATED VESICLE MEMBRANE PROTEIN P24 PRECURSOR (FRAGMENT).
   2.8e-18:109:41
   CRICETULUS GRISEUS (CHINESE HAMSTER).
   P49020
F-NT2RP3002958
   TRANS-GOLGI NETWORK INTEGRAL MEMBRANE PROTEIN TGN38 PRECURSOR.
   6.4e-06:172:27
   RATTUS NORVEGICUS (RAT).
   P19814
F-NT2RP3002983
   COLLAGEN ALPHA 1(I) CHAIN (FRAGMENTS).
   4.4e-05:106:41
   BOS TAURUS (BOVINE).
   P02453
F-NT2RP3003000
   SODIUM CHANNEL PROTEIN (NA+ CHANNEL).
   9.7e-30:221:31
   ELECTROPHORUS ELECTRICUS (ELECTRIC EEL).
   P02719
F-NT2RP3003076
   ENVELOPE GLYCOPROTEIN GP340 (MEMBRANE ANTIGEN) (MA) [CONTAINS: GLYCOPROTEIN GP220].
   0.00033:173:30
   EPSTEIN-BARR VIRUS (STRAIN B95-8) (HUMAN HERPESVIRUS 4).
   P03200
F-NT2RP3003354
   SECRETORY CARRIER-ASSOCIATED MEMBRANE PROTEIN 3.
   2.0e-54:204:51
   MUS MUSCULUS (MOUSE).
   035609
F-NT2RP3003448
   PROTEASE DEGS PRECURSOR (EC 3.4.21.-).
   4.0e-05:112:33
   ESCHERICHIA COLI.
   P31137
F-NT2RP3003469
   !!!! ALU SUBFAMILY SQ WARNING ENTRY !!!!
   1.2e-17:70:64
   HOMO SAPIENS (HUMAN).
   P39194
F-NT2RP3003473
   BACTENECIN 7 PRECURSOR (BAC7) (PR-59).
   0.0037:33:63
   BOS TAURUS (BOVINE).
   P19661
F-NT2RP3003527
   SERINE/THREONINE-SPECIFIC PROTEIN KINASE MINIBRAIN HOMOLOG (EC 2.7.1.-) (HP86) (DYRK).
   1.8e-53:159:69
   HOMO SAPIENS (HUMAN).
   Q13627
F-NT2RP3003532
   OX-2 MEMBRANE GLYCOPROTEIN PRECURSOR (FRAGMENT).
   2.3e-114:219:97
   HOMO SAPIENS (HUMAN).
   P41217
F-NT2RP3003535
   HYPOTHETICAL 7.2 KD PROTEIN IN RPS2 3 REGION (ORF57).
   0.98:36:30
   ASTASIA LONGA (EUGLENOPHYCEAN ALGA).
   P34774
F-NT2RP3003559
   MALE SPECIFIC SPERM PROTEIN MST84DB.
   0.047:29:48
   DROSOPHILA MELANOGASTER (FRUIT FLY).
   Q01643
F-NT2RP3003614
   TRYPSIN INHIBITOR II (BDTI-II).
   0.98:23:39
   BRYONIA DIOICA (RED BRYONY).
   P11968
F-NT2RP3003729
   HYPOTHETICAL 42.1 KD PROTEIN IN SNZ1-YPK2 INTERGENIC REGION.
   4.1e-11:204:30
   SACCHAROMYCES CEREVISIAE (BAKER'S YEAST).
   Q03151
F-NT2RP3003849
   PROTEIN KINASE C, BRAIN ISOZYME (EC 2.7.1.-) (PKC) (DPKC53E(BR)).
   9.7e-17:126:34
   DROSOPHILA MELANOGASTER (FRUIT FLY).
   P05130
F-NT2RP3003874
   MYOSIN I ALPHA (MMI-ALPHA).
   3.1e-64:141:84
   MUS MUSCULUS (MOUSE).
   P46735
F-NT2RP3003939
   CELL DIVISION PROTEIN FTSH HOMOLOG 4 (EC 3.4.24.-).
   7.1e-34:76:61
   SYNECHOCYSTIS SP. (STRAIN PCC 6803).
   P72991
F-NT2RP3003963
   HISTIDINE-RICH, METAL BINDING POLYPEPTIDE.
   0.95:31:38
   HELICOBACTER PYLORI (CAMPYLOBACTER PYLORI).
   Q48251
F-NT2RP3004000
   DNA-DIRECTED RNA POLYMERASE II LARGEST SUBUNIT (EC 2.7.7.6) (RPB1) (FRAGMENT).
   7.1e-07:187:29
   CRICETULUS GRISEUS (CHINESE HAMSTER).
   P11414
F-NT2RP3004025
   EBNA-1 NUCLEAR PROTEIN.
   0.022:79:40
   EPSTEIN-BARR VIRUS (STRAIN B95-8) (HUMAN HERPESVIRUS 4).
   P03211
F-NT2RP3004067
   COLLAGEN ALPHA 1(X) CHAIN PRECURSOR.
   5.0e-07:184:35
   HOMO SAPIENS (HUMAN).
   Q03692
F-NT2RP3004075
   GLYCINE-RICH CELL WALL STRUCTURAL PROTEIN PRECURSOR.
   2.9e-07:92:40
   HORDEUM VULGARE (BARLEY).
   P17816
F-NT2RP3004083
   ATP SYNTHASE PROTEIN 8 (EC 3.6.1.34) (A6L).
   0.013:24:45
   COTURNIX COTURNIX JAPONICA (JAPANESE QUAIL).
   P50682
F-NT2RP3004090
   SALIVARY GLUE PROTEIN SGS-3 PRECURSOR.
   1.2e-07:195:29
   DROSOPHILA ERECTA (FRUIT FLY).
   P13730
F-NT2RP3004119
   PEREGRIN (BR140 PROTEIN).
   4.1e-40:227:43
   HOMO SAPIENS (HUMAN).
   P55201
F-NT2RP3004130
   CELL SURFACE ANTIGEN 114/A10 PRECURSOR.
   2.4e-08:71:42
   MUS MUSCULUS (MOUSE).
   P19467
F-NT2RP3004133
   GLYCOSYLTRANSFERASE ALG2 (EC 2.4.1.-).
   1.5e-28:111:44
   SACCHAROMYCES CEREVISIAE (BAKER'S YEAST).
   P43636
F-NT2RP3004202
   PROLINE-RICH PROTEIN MP-2 PRECURSOR.
   3.0e-06:104:37
   MUS MUSCULUS (MOUSE).
   P05142
F-NT2RP3004294
   HYPOTHETICAL 22.2 KD PROTEIN IN NSR1-TIF4631 INTERGENIC REGION.
   8.8e-10:129:31
   SACCHAROMYCES CEREVISIAE (BAKER'S YEAST).
   P53288
F-NT2RP3004309
   A-AGGLUTININ ATTACHMENT SUBUNIT PRECURSOR.
   1.9e-05:212:30
   SACCHAROMYCES CEREVISIAE (BAKER'S YEAST).
   P32323
F-NT2RP3004321
   REGULATORY PROTEIN SIR2 HOMOLOG (LMSIR2RP).
   2.8e-09:81:40
   LEISHMANIA MAJOR.
   Q25337
F-NT2RP3004345
   GLYCINE-RICH CELL WALL STRUCTURAL PROTEIN 2 PRECURSOR.
   1.3e-11:75:46
   ORYZA SATIVA (RICE).
   P29834
F-NT2RP3004355
   HYDROGENASE EXPRESSION/FORMATION PROTEIN HUPV.
   0.81:154:26
   AZOTOBACTER CHROOCOCCUM MCD 1.
   Q43959
F-NT2RP3004374
   HOMEOBOX PROTEIN HOX-A2.
   0.28:77:37
   GALLUS GALLUS (CHICKEN).
   Q08727
F-NT2RP3004406
   HYPOTHETICAL 37.4 KD PROTEIN IN IRR1-TIM44 INTERGENIC REGION.
   4.9e-18:165:33
   SACCHAROMYCES CEREVISIAE (BAKER'S YEAST).
   P40544
F-NT2RP3004481
   BUTYROPHILIN PRECURSOR (BT).
   4.0e-13:152:31
   HOMO SAPIENS (HUMAN).
   Q13410
F-NT2RP3004552
   COMPLEMENT RECEPTOR TYPE 1 PRECURSOR (C3B/C4B RECEPTOR) (CD35 ANTIGEN).
   3.4e-05:211:28
   HOMO SAPIENS (HUMAN).
   P17927
F-NT2RP3004557
   INTERFERON GAMMA UP-REGULATED I-5111 PROTEIN PRECURSOR (IGUP I-5111).
   1.6e-23:129:35
   HOMO SAPIENS (HUMAN).
   Q06323
F-NT2RP3004625
   GLYCOPROTEIN X PRECURSOR.
   2.4e-10:225:25
   EQUINE HERPESVIRUS TYPE 1 (STRAIN AB4P) (EHV-1).
   P28968
F-NT2RP3004640
   ENAMELIN (TUFTELIN).
   2.6e-70:167:85
   BOS TAURUS (BOVINE).
   P27628
F-NT2RP3004647
   ADP,ATP CARRIER PROTEIN, HEART/SKELETAL MUSCLE ISOFORM T1 (ADP/ATP TRANSLOCASE 1) (ADENINE NUCLEOTIDE TRANSLOCATOR 1) (ANT 1).
   4.6e-10:116:34
   HOMO SAPIENS (HUMAN).
   P12235
F-NT2RP4000108
   NEUROFILAMENT TRIPLET L PROTEIN (68 KD NEUROFILAMENT PROTEIN) (NF-L) (NF68).
   3.4e-107:255:87
   RATTUS NORVEGICUS (RAT).
   P19527
F-NT2RP4000634
   MAPK/ERK KINASE KINASE 2 (EC 2.7.1.-) (MEK KINASE 2) (MEKK 2).
   7.9e-142:267:88
   MUS MUSCULUS (MOUSE).
   Q61083
F-NT2RP4000962
   SPORULATION-SPECIFIC PROTEIN 1 (EC 2.7.1.-).
   1.5e-13:158:31
   SACCHAROMYCES CEREVISIAE (BAKER'S YEAST).
   P08458
F-NT2RP4001001
F-NT2RP4001009
   POTENTIAL CAAX PRENYL PROTEASE 1 (EC 3.4.24.-) (PRENYL PROTEIN- SPECIFIC ENDOPROTEASE 1) (PPSEP 1).
   7.7e-24:235:31
   SCHIZOSACCHAROMYCES POMBE (FISSION YEAST).
   Q10071
F-NT2RP4001467
   5'-NUCLEOTIDASE PRECURSOR (EC 3.1.3.5) (ECTO-NUCLEOTIDASE) (5'-NT) (CD73 ANTIGEN).
   1.2e-120:237:97
   HOMO SAPIENS (HUMAN).
   P21589
F-NT2RP4001877
   GLYCINE-RICH RNA-BINDING PROTEIN.
   1.4e-08:89:34
   DAUCUS CAROTA (CARROT).
   Q03878
F-NT2RP4001879
   VERY HYPOTHETICAL 8.9 KD PROTEIN CY441.05 PRECURSOR.
   0.98:49:34
   MYCOBACTERIUM TUBERCULOSIS.
   P71934
F-NT2RP4002187
   PUTATIVE STEROID DEHYDROGENASE KIK-I (EC 1.1.1.-).
   4.5e-98:246:78
   MUS MUSCULUS (MOUSE).
   070503
F-NT2RP4002451
   CUTICLE COLLAGEN 2.
   0.85:92:35
   CAENORHABDITIS ELEGANS.
   P17656
F-NT2RP4002715
   HYPOTHETICAL 13.6 KD PROTEIN IN SPT4-ROM1 INTERGENIC REGION.
   0.47:31:48
   SACCHAROMYCES CEREVISIAE (BAKER'S YEAST).
   P53245
F-NT2RP4002750
   HIGH-AFFINITY CATIONIC AMINO ACID TRANSPORTER-1 (CAT-1) (CAT1) (SYSTEM Y+ BASIC AMINO ACID TRANSPORTER) (ECOTROPIC RETROVIRAL LEUKEMIA RECEPTOR HOMOLOG) (ERR) (ECOTROPIC RETROVIRUS RECEPTOR HOMOLOG).
   3.3e-63:185:67
   HOMO SAPIENS (HUMAN).
   P30825
F-OVARC1000003
   RENAL SODIUM-DEPENDENT PHOSPHATE TRANSPORT PROTEIN 2 (SODIUM/PHOSPHATE COTRANSPORTER 2) (NA(+)/PI COTRANSPORTER 2) (RENAL SODIUM-PHOSPHATE TRANSPORT PROTEIN 2) (RENAL NA+-DEPENDENT PHOSPHATE COTRANSPORTER 2).
   2.2e-82:197:72
   HOMO SAPIENS (HUMAN).
   Q06495
F-OVARC1000090
   HOMEOBOX PROTEIN HOX-B1 (GHOX-LAB).
   0.049:120:32
   GALLUS GALLUS (CHICKEN).
   P31259
F-OVARC1000105
   UBIQUITIN-CONJUGATING ENZYME E2-28.4 KD (EC 6.3.2.19) (UBIQUITIN- PROTEIN LIGASE) (UBIQUITIN CARRIER PROTEIN).
   8.6e-47:159:58
   SACCHAROMYCES CEREVISIAE (BAKER'S YEAST).
   P33296
F-OVARC1000137
   HYPOTHETICAL 22.7 KD PROTEIN IN PAS1-MST1 INTERGENIC REGION.
   0.058:28:64
   SACCHAROMYCES CEREVISIAE (BAKER'S YEAST).
   P36015
F-OVARC1000208
   !!!! ALU SUBFAMILY SX WARNING ENTRY !!!!
   2.2e-12:51:74
   HOMO SAPIENS (HUMAN).
   P39195
F-OVARC1000255
   TYROSINE-PROTEIN KINASE SYK (EC 2.7.1.112) (SPLEEN TYROSINE KINASE).
   1.1e-112:144:86
   HOMO SAPIENS (HUMAN).
   P43405
F-OVARC1000275
   GASTRIN PRECURSOR.
   0.11:59:37
   HOMO SAPIENS (HUMAN).
   P01350
F-OVARC1000298
   PROLINE-RICH PROTEIN MP-3 (FRAGMENT).
   0.014:74:39
   MUS MUSCULUS (MOUSE).
   P05143
F-OVARC1000307
   EXTENSIN PRECURSOR (CELL WALL HYDROXYPROLINE-RICH GLYCOPROTEIN).
   1.0:33:54
   NICOTIANA TABACUM (COMMON TOBACCO).
   P13983
F-OVARC1000313
   PROTEIN DISULFIDE ISOMERASE-RELATED PROTEIN PRECURSOR (ERP72) (CALCIUM-BINDING PROTEIN 2) (CABP2).
   4.0e-15:127:37
   RATTUS NORVEGICUS (RAT).
   P38659
F-OVARC1000331
   GMP REDUCTASE (EC 1.6.6.8) (GUANOSINE 5'-MONOPHOSPHATE OXIDOREDUCTASE).
   2.0e-24:64:84
   HOMO SAPIENS (HUMAN).
   P36959
F-OVARC1000410
   FIBRINOGEN-LIKE PROTEIN A PRECURSOR (FREP-A).
   1.9e-44:229:41
   PARASTICHOPUS PARVIMENSIS (SEA CUCUMBER).
   P19477
F-OVARC1000439
   SALIVARY GLUE PROTEIN SGS-7 PRECURSOR.
   0.99:41:43
   DROSOPHILA MELANOGASTER (FRUIT FLY).
   P02841
F-OVARC1000467
   GLYCINE-RICH CELL WALL STRUCTURAL PROTEIN (CLONE W10-1) (FRAGMENT).
   0.0061:30:63
   LYCOPERSICON ESCULENTUM (TOMATO).
   Q01157
F-OVARC1000529
   PROBABLE SERINE/THREONINE-PROTEIN KINASE YKL 101 W (EC 2.7.1.-).
   1.5e-20:127:42
   SACCHAROMYCES CEREVISIAE (BAKER'S YEAST).
   P34244
F-OVARC1000553
   DIPEPTIDYL PEPTIDASE IV (EC 3.4.14.5) (DPP IV) (THYMOCYTE-ACTIVATING MOLECULE) (THAM).
   7.6e-26:169:40
   MUS MUSCULUS (MOUSE).
   P28843
F-OVARC1000775
   METALLOTHIONEIN (MT).
   0.91:31:38
   CARASSIUS AURATUS (GOLDFISH).
   P52723
F-OVARC1000811
   COAGULATION FACTOR XII PRECURSOR (EC 3.4.21.38) (HAGEMAN FACTOR) (HAF).
   2.8e-11:69:43
   HOMO SAPIENS (HUMAN).
   P00748
F-OVARC1000853
   CUTICLE COLLAGEN 40.
   0.00013:130:33
   CAENORHABDITIS ELEGANS.
   P34804
F-OVARC1000873
   MALE SPECIFIC SPERM PROTEIN MST84DB.
   0.00015:53:33
   DROSOPHILA MELANOGASTER (FRUIT FLY).
   Q01643
F-OVARC1000916
   GALACTOSYLTRANSFERASE ASSOCIATED PROTEIN KINASE P58/GTA (EC 2.7.1.-).
   2.5e-26:109:53
   MUS MUSCULUS (MOUSE).
   P24788
F-OVARC1000956
   D(4) DOPAMINE RECEPTOR (D(2C) DOPAMINE RECEPTOR).
   0.00073:115:33
   HOMO SAPIENS (HUMAN).
   P21917
F-OVARC1000995
   POSSIBLE GLOBAL TRANSCRIPTION ACTIVATOR SNF2L2 (SNF2-ALPHA).
   0.00031:139:25
   HOMO SAPIENS (HUMAN).
   P51531
F-OVARC1001030
   5E5 ANTIGEN.
   1.9e-09:89:41
   RATTUS NORVEGICUS (RAT).
   Q63003
F-OVARC1001049
   PROCOLLAGEN ALPHA 1(I) CHAIN PRECURSOR.
   1.5e-08:146:38
   GALLUS GALLUS (CHICKEN).
   P02457
F-OVARC1001086
   VITELLOGENIN II PRECURSOR (MAJOR VITELLOGENIN) [CONTAINS: LIPOVITELLIN I (LVI); PHOSVITIN (PV); LIPOVITELLIN II (LVII); YGP40].
   5.3e-08:182:32
   GALLUS GALLUS (CHICKEN).
   P02845
F-OVARC1001132
   GC-RICH SEQUENCE DNA-BINDING FACTOR (GCF) (TRANSCRIPTION FACTOR 9) (TCF-9).
   9.2e-40:229:37
   HOMO SAPIENS (HUMAN).
   P16383
F-OVARC1001163
   HYPOTHETICAL 49.3 KD PROTEIN C30D 11.06C IN CHROMOSOME I.
   8.8e-05:38:44
   SCHIZOSACCHAROMYCES POMBE (FISSION YEAST).
   Q09906
F-OVARC1001222
   AMELOGENIN, CLASS I PRECURSOR.
   0.72:96:31
   BOS TAURUS (BOVINE).
   P02817
F-OVARC1001260
F-OVARC1001336
   RENAL SODIUM-DEPENDENT PHOSPHATE TRANSPORT PROTEIN 2 (SODIUM/PHOSPHATE COTRANSPORTER 2) (NA(+)/PI COTRANSPORTER 2) (RENAL SODIUM-PHOSPHATE TRANSPORT PROTEIN 2) (RENAL NA+-DEPENDENT PHOSPHATE COTRANSPORTER 2).
   1.1e-33:103:71
   RATTUS NORVEGICUS (RAT).
   Q06496
F-OVARC1001338
   SERINE/THREONINE-PROTEIN KINASE UNC-51 (EC 2.7.1.-).
   3.8e-30:89:46
   CAENORHABDITIS ELEGANS.
   Q23023
F-OVARC1001569
   ACROSIN PRECURSOR (EC 3.4.21.10) (53 KD FUCOSE-BINDING PROTEIN).
   2.2e-06:28:64
   SUS SCROFA (PIG).
   P08001
F-OVARC1001570
   CATHEPSIN E PRECURSOR (EC 3.4.23.34).
   1.8e-09:121:33
   CAVIA PORCELLUS (GUINEA PIG).
   P25796
F-OVARC1001596
   REGULATORY PROTEIN E2.
   0.33:77:37
   HUMAN PAPILLOMAVIRUS TYPE 14.
   P36783
F-OVARC1001607
   ALPHA-1,6-MANNOSYL-GLYCOPROTEIN BETA-1,2-N-ACETYLGLUCOSAMINYLTRANSFERASE (EC 2.4.1.143) (N-GLYCOSYL-OLIGOSACCHARIDE-GLYCOPROTEIN N-ACETYLGLUCOSAMINYLTRANSFERASE II) (BETA-1,2-N-ACETYLGLUCOSAMINYLTRANSFERASE II) (GNT-II) (GLCNAC-T II).
   1.0e-28:69:84
   HOMO SAPIENS (HUMAN).
   Q10469
F-OVARC1001725
F-OVARC1001727
F-OVARC1001807
   EARLY RESPONSE PROTEIN NAK1 (TR3 ORPHAN RECEPTOR).
   2.4e-51:153:75
   HOMO SAPIENS (HUMAN).
   P22736
F-OVARC1001833
   CIS-GOLGI MATRIX PROTEIN GM130.
   1.2e-55:169:75
   RATTUS NORVEGICUS (RAT).
   Q62839
F-OVARC1001952
   EBNA-1 NUCLEAR PROTEIN.
   3.5e-19:130:43
   EPSTEIN-BARR VIRUS (STRAIN B95-8) (HUMAN HERPESVIRUS 4).
   P03211
F-OVARC1001991
   HYPOTHETICAL PROTEIN KIAA0176 (FRAGMENT).
   3.7e-16:141:43
   HOMO SAPIENS (HUMAN).
   Q14681
F-OVARC1002058
   LAMININ ALPHA-5 CHAIN (FRAGMENT).
   2.8e-22:163:33
   MUS MUSCULUS (MOUSE).
   Q61001
F-OVARC1002178
   TRANS-ACTING TRANSCRIPTIONAL PROTEIN ICP0 (IMMEDIATE-EARLY PROTEIN IE110) (VMW110) (ALPHA-0 PROTEIN).
   0.12:73:36
   HERPES SIMPLEX VIRUS (TYPE 1 / STRAIN 17).
   P08393
F-PLACE1000033
   VON WILLEBRAND FACTOR PRECURSOR.
   1.7e-19:190:28
   CANIS FAMILIARIS (DOG).
   Q28295
F-PLACE1000231
   DNA-BINDING PROTEIN MNB1A.
   0.24:60:33
   ZEA MAYS (MAIZE).
   P38564
F-PLACE1000258
   ZINC FINGER PROTEIN 177.
   3.6e-19:55:61
   HOMO SAPIENS (HUMAN).
   Q13360
F-PLACE1000442
   ZINC FINGER PROTEIN 136.
   1.7e-80:180:72
   HOMO SAPIENS (HUMAN).
   P52737
F-PLACE1000560
   COLICIN E9 (EC 3.1.21.1).
   0.015:47:44
   ESCHERICHIA COLI.
   P09883
F-PLACE1000740
   NEUROGENIC LOCUS NOTCH HOMOLOG PROTEIN 4 PRECURSOR (TRANSFORMING PROTEIN INT-3).
   1.6e-05:75:36
   MUS MUSCULUS (MOUSE).
   P31695
F-PLACE1000907
   ZINC FINGER PROTEIN 141.
   2.8e-15:43:88
   HOMO SAPIENS (HUMAN).
   Q15928
F-PLACE1000912
   PROBABLE E4 PROTEIN (E1^E4).
   0.19:46:36
   HUMAN PAPILLOMAVIRUS TYPE 6B.
   P06459
F-PLACE1000914
   MALE SPECIFIC SPERM PROTEIN MST87F.
   0.054:27:44
   DROSOPHILA MELANOGASTER (FRUIT FLY).
   P08175
F-PLACE1000927
   HYPOTHETICAL PROTEIN HI0044.
   3.9e-07:139:30
   HAEMOPHILUS INFLUENZAE.
   P44477
F-PLACE1000986
F-PLACE1001016
   SODIUM CHANNEL PROTEIN, BRAIN II ALPHA SUBUNIT.
   2.7e-05:120:32
   RATTUS NORVEGICUS (RAT).
   P04775
F-PLACE1001100
F-PLACE1001114
   PROCOLLAGEN ALPHA 1(I) CHAIN PRECURSOR.
   2.5e-07:250:28
   MUS MUSCULUS (MOUSE).
   P11087
F-PLACE1001123
   INTESTINAL MEMBRANE A4 PROTEIN (DIFFERENTIATION-DEPENDENT PROTEIN A4).
   6.2e-09:95:31
   HOMO SAPIENS (HUMAN).
   Q04941
F-PLACE1001183
   NONHISTONE CHROMOSOMAL PROTEIN HMG-17.
   0.31:52:34
   GALLUS GALLUS (CHICKEN).
   P02314
F-PLACE1001229
   NADH-UBIQUINONE OXIDOREDUCTASE CHAIN 5 (EC 1.6.5.3) (FRAGMENT).
   1.0:38:47
   ARTEMIA SAUNA (BRINE SHRIMP).
   P19047
F-PLACE1001231
   SODIUM/GLUCOSE COTRANSPORTER 1 (NA(+)/GLUCOSE COTRANSPORTER 1) (HIGH AFFINITY SODIUM-GLUCOSE COTRANSPORTER).
   4.7e-06:181:27
   ORYCTOLAGUS CUNICULUS (RABBIT).
   P11170
F-PLACE1001340
   MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).
   6.5e-14:136:29
   NEUROSPORA CRASSA.
   P23231
F-PLACE1001401
   HIGH AFFINITY IMMUNOGLOBULIN EPSILON RECEPTOR BETA-SUBUNIT (FCERI) (IGE FC RECEPTOR, BETA-SUBUNIT).
   1.3e-11:103:40
   RATTUS NORVEGICUS (RAT).
   P13386
F-PLACE1001407
   INTEGUMENTARY MUCIN C.1 (FIM-C.1) (FRAGMENT).
   0.013:121:32
   XENOPUS LAEVIS (AFRICAN CLAWED FROG).
   Q05049
F-PLACE1001464
   5'-NUCLEOTIDASE PRECURSOR (EC 3.1.3.5) (ECTO-NUCLEOTIDASE) (5'-NT) (CD73 ANTIGEN).
   1.4e-119:246:89
   HOMO SAPIENS (HUMAN).
   P21589
F-PLACE1001500
   BLOOM'S SYNDROME PROTEIN.
   8.3e-26:203:34
   HOMO SAPIENS (HUMAN).
   P54132
F-PLACE1001516
   GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE).
   7.4e-07:204:30
   SACCHAROMYCES CEREVISIAE (BAKER'S YEAST).
   P08640
F-PLACE1001536
F-PLACE1001564
   LEUCOCYTE ANTIGEN CD97 PRECURSOR.
   2.1e-09:170:24
   HOMO SAPIENS (HUMAN).
   P48960
F-PLACE1001655
   VOLTAGE-GATED POTASSIUM CHANNEL PROTEIN KV2.1 (DRK1).
   4.0e-34:189:39
   RATTUS NORVEGICUS (RAT).
   P15387
F-PLACE1001788
   HYPOTHETICAL 36.7 KD PROTEIN C2F7.02C IN CHROMOSOME I.
   6.2e-21:75:58
   SCHIZOSACCHAROMYCES POMBE (FISSION YEAST).
   Q09695
F-PLACE1001795
   HYPOTHETICAL 30.6 KD PROTEIN IN SCP160-SMC3 INTERGENIC REGION PRECURSOR.
   3.8e-21:159:40
   SACCHAROMYCES CEREVISIAE (BAKER'S YEAST).
   P47032
F-PLACE1001836
   ENV POLYPROTEIN PRECURSOR (COAT POLYPROTEIN) [CONTAINS: OUTER MEMBRANE PROTEIN GP70; TRANSMEMBRANE PROTEIN P20E].
   4.5e-29:134:47
   BABOON ENDOGENOUS VIRUS (STRAIN M7).
   P10269
F-PLACE1001918
   ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).
   1.5e-30:228:32
   SACCHAROMYCES CEREVISIAE (BAKER'S YEAST).
   P32802
F-PLACE1001949
   PROBABLE CALCIUM-TRANSPORTING ATPASE 9 (EC 3.6.1.38).
   5. le-36:210:46
   SACCHAROMYCES CEREVISIAE (BAKER'S YEAST).
   Q12697
F-PLACE1002080
   HYPOTHETICAL PROTEIN KIAA0288 (HA6116).
   3.5e-26:207:45
   HOMO SAPIENS (HUMAN).
   P56524
F-PLACE1002095
   N-ACETYLLACTOSAMINE SYNTHASE (EC 2.4.1.90) (N-ACETYLGLUCOSAMINE (BETA 1→4)GALACTOSYLTRANSFERASE) (EC 2.4.1.38) (LACTOSE SYNTHASE A PROTEIN (EC 2.4.1.22)) (GALACTOSYLTRANSFERASE) (GT).
   0.32:50:34
   MUS MUSCULUS (MOUSE).
   P15535
F-PLACE1002153
   CELL SURFACE GLYCOPROTEIN 1 PRECURSOR (OUTER LAYER PROTEIN B) (SLAYER PROTEIN 1).
   0.00021:214:26
   CLOSTRIDIUM THERMOCELLUM.
   Q06852
F-PLACE1002329
   EPIDERMAL GROWTH FACTOR RECEPTOR KINASE SUBSTRATE EPS8.
   1.1e-35:179:44
   MUS MUSCULUS (MOUSE).
   Q08509
F-PLACE1002355
   COMPLEMENT C4 PRECURSOR [CONTAINS: C4A ANAPHYLATOXIN] (FRAGMENTS).
   1.0e-14:183:32
   BOS TAURUS (BOVINE).
   P01030
F-PLACE1002374
   CATHEPSIN L PRECURSOR (EC 3.4.22.15) (MAJOR EXCRETED PROTEIN) (MEP).
   9.2e-107:225:86
   HOMO SAPIENS (HUMAN).
   P07711
F-PLACE1002518
   HYPOTHETICAL 13.2 KD PROTEIN IN ORC2-TIP1 INTERGENIC REGION.
   6.1e-05:59:44
   SACCHAROMYCES CEREVISIAE (BAKER'S YEAST).
   P38239
F-PLACE1002547
   MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).
   1.0e-22:230:31
   NEUROSPORA CRASSA.
   P23231
F-PLACE1002726
   COLLAGEN ALPHA 1(I) CHAIN (FRAGMENT).
   0.61:25:48
   ORYCTOLAGUS CUNICULUS (RABBIT).
   P02456
F-PLACE1002905
   ENDOZEPINE-RELATED PROTEIN PRECURSOR (MEMBRANE-ASSOCIATED DIAZEPAM BINDING INHIBITOR) (MA-DBI).
   5.0e-31:93:64
   BOS TAURUS (BOVINE).
   P07106
F-PLACE1002911
   T-CELL SURFACE PROTEIN TACTILE PRECURSOR (CD96 ANTIGEN).
   6.6e-06:95:35
   HOMO SAPIENS (HUMAN).
   P40200
F-PLACE1002967
   HIGH AFFINITY IMMUNOGLOBULIN EPSILON RECEPTOR BETA-SUBUNIT (FCERI) (IGE FC RECEPTOR, BETA-SUBUNIT).
   9.4e-08:95:37
   MUS MUSCULUS (MOUSE).
   P20490
F-PLACE1003135
   SERINE/THREONINE-PROTEIN KINASE STE20 (EC 2.7.1.-).
   1.9e-33:99:50
   SACCHAROMYCES CEREVISIAE (BAKER'S YEAST).
   Q03497
F-PLACE1003163
   ENDOZEPINE-RELATED PROTEIN PRECURSOR (MEMBRANE-ASSOCIATED DIAZEPAM BINDING INHIBITOR) (MA-DBI).
   9.8e-15:105:38
   BOS TAURUS (BOVINE).
   P07106
F-PLACE1003407
   CLN5 PROTEIN.
   4.2e-109:217:89
   HOMO SAPIENS (HUMAN).
   075503
F-PLACE1003428
   BIOTINIDASE PRECURSOR (EC 3.5.1.12).
   1.0e-36:104:46
   HOMO SAPIENS (HUMAN).
   P43251
F-PLACE1003438
   HYPOTHETICAL 104.4 KD PROTEIN C17A5.16 IN CHROMOSOME I.
   1.1e-10:148:33
   SCHIZOSACCHAROMYCES POMBE (FISSION YEAST).
   013776
F-PLACE1003460
   2-HYDROXY-6-KETONONA-2,4-DIENEDIOIC ACID HYDROLASE (EC 3.7.1.-).
   0.00028:134:27
   ESCHERICHIA COLI.
   P77044
F-PLACE1003529
   TRANSCRIPTION REGULATORY PROTEIN SNF5 (SWI/SNF COMPLEX COMPONENT SNF5) (TRANSCRIPTION FACTOR TYE4).
   0.00047:157:27
   SACCHAROMYCES CEREVISIAE (BAKER'S YEAST).
   P18480
F-PLACE1003573
   T-CELL SURFACE GLYCOPROTEIN YE1/48 (T LYMPHOCYTE ANTIGEN A1) (LY49-A ANTIGEN).
   0.022:129:25
   MUS MUSCULUS (MOUSE).
   P20937
F-PLACE1003598
   TRANS-ACTING TRANSCRIPTIONAL PROTEIN ICP0 (P135 PROTEIN) (IER 2.9/ER2.6).
   0.0017:102:44
   BOVINE HERPESVIRUS TYPE 1 (STRAIN JURA).
   P29128
F-PLACE1003644
   PROTEIN Q300.
   6.7e-05:24:70
   MUS MUSCULUS (MOUSE).
   Q02722
F-PLACE1003737
   TOLL PROTEIN PRECURSOR.
   7.3e-08:203:27
   DROSOPHILA MELANOGASTER (FRUIT FLY).
   P08953
F-PLACE1003772
   SALIVARY PROLINE-RICH PROTEIN II-1 (FRAGMENT).
   3.7e-07:141:32
   HOMO SAPIENS (HUMAN).
   P81489
F-PLACE1003839
   PROLINE-RICH PROTEIN MP-3 (FRAGMENT).
   1.3e-09:201:31
   MUS MUSCULUS (MOUSE).
   P05143
F-PLACE1003845
   PUTATIVE UDP-GLUCOSE 4-EPIMERASE (EC 5.1.3.2) (GALACTOWALDENASE) (UDP-GALACTOSE 4-EPIMERASE).
   5.0e-13:103:33
   METHANOCOCCUS JANNASCHII.
   Q57664
F-PLACE1003852
   CELL SURFACE GLYCOPROTEIN EMR1 PRECURSOR (EMR1 HORMONE RECEPTOR).
   2.0e-18:189:29
   HOMO SAPIENS (HUMAN).
   Q14246
F-PLACE1004028
   HYPOTHETICAL 9.7 KD PROTEIN IN PURC-PURL INTERGENIC REGION.
   0.97:47:31
   BACILLUS SUBTILIS.
   P12049
F-PLACE1004078
   ADSEVERIN (SCINDERIN) (SC).
   5.3e-98:176:90
   BOS TAURUS (BOVINE).
   Q28046
F-PLACE1004166
   CREB-BINDING PROTEIN.
   9.6e-08:107:34
   HOMO SAPIENS (HUMAN).
   Q92793
F-PLACE1004168
   GENERAL NEGATIVE REGULATOR OF TRANSCRIPTION SUBUNIT 1.
   6.8e-05:147:30
   SACCHAROMYCES CEREVISIAE (BAKER'S YEAST).
   P25655
F-PLACE1004199
   !!!! ALU SUBFAMILY J WARNING ENTRY !!!!
   4.2e-05:65:52
   HOMO SAPIENS (HUMAN).
   P39188
F-PLACE1004279
   HYPOTHETICAL 59.1 KD PROTEIN ZK637.1 IN CHROMOSOME III.
   3.6e-11:166:30
   CAENORHABDITIS ELEGANS.
   P30638
F-PLACE1004282
   HISTONE H1C (CLONE XLHW2).
   0.74:73:26
   XENOPUS LAEVIS (AFRICAN CLAWED FROG).
   P15866
F-PLACE1004305
   RAS-RELATED PROTEIN RAC1.
   2.3e-23:161:39
   DROSOPHILA MELANOGASTER (FRUIT FLY).
   P40792
F-PLACE1004441
   PROBABLE G PROTEIN-COUPLED RECEPTOR GPR1.
   5.4e-70:156:89
   HOMO SAPIENS (HUMAN).
   P46091
F-PLACE1004450
   AMINOPEPTIDASE N (EC 3.4.11.2) (MICROSOMAL AMINOPEPTIDASE).
   3.1e-40:196:44
   RATTUS NORVEGICUS (RAT).
   P15684
F-PLACE1004482
   ATP SYNTHASE PROTEIN 8 (EC 3.6.1.34) (A6L).
   0.23:26:30
   GALLUS GALLUS (CHICKEN).
   P14093
F-PLACE1004492
   COLLAGEN ALPHA 1(I) CHAIN (FRAGMENTS).
   1.2e-05:150:34
   BOS TAURUS (BOVINE).
   P02453
F-PLACE1004519
   ENL PROTEIN.
   0.68:170:30
   HOMO SAPIENS (HUMAN).
   Q03111
F-PLACE1004520
   PREGNANCY-SPECIFIC BETA-1 GLYCOPROTEIN PRECURSOR.
   3.5e-50:150:74
   HOMO SAPIENS (HUMAN).
   P11462
F-PLACE1004630
   INTEGRIN BETA-6 SUBUNIT PRECURSOR.
   9.1e-31:189:39
   HOMO SAPIENS (HUMAN).
   P18564
F-PLACE1004637
   MALE SPECIFIC SPERM PROTEIN MST84DA.
   0.47:29:44
   DROSOPHILA MELANOGASTER (FRUIT FLY).
   Q01642
F-PLACE1004648
   PROLINE-RICH PROTEIN MP-2 PRECURSOR.
   8.4e-05:89:40
   MUS MUSCULUS (MOUSE).
   P05142
F-PLACE1004816
   MICROFIBRIL-ASSOCIATED GLYCOPROTEIN 4.
   1.0e-25:117:46
   HOMO SAPIENS (HUMAN).
   P55083
F-PLACE1004887
   SALIVARY GLUE PROTEIN SGS-3 PRECURSOR.
   8.4e-09:195:30
   DROSOPHILA ERECTA (FRUIT FLY).
   P13730
F-PLACE1005003
   PROSTASIN PRECURSOR (EC 3.4.21.-).
   1.2e-24:139:40
   HOMO SAPIENS (HUMAN).
   Q16651
F-PLACE1005005
   UBIQUITIN-CONJUGATING ENZYME E2 G2 (EC 6.3.2.19) (UBIQUITIN-PROTEIN LIGASE) (UBIQUITIN CARRIER PROTEIN).
   2.5e-28:51:84
   HOMO SAPIENS (HUMAN).
   P56554
F-PLACE1005031
   CHLORINE CHANNEL PROTEIN P64.
   2.7e-52:142:76
   BOS TAURUS (BOVINE).
   P35526
F-PLACE1005239
   SPLICING FACTOR, ARGININE/SERINE-RICH 6 (PRE-MRNA SPLICING FACTOR SRP55) (FRAGMENT).
   0.27:78:26
   ORYCTOLAGUS CUNICULUS (RABBIT).
   018776
F-PLACE1005250
   HYPOTHETICAL 9.0 KD PROTEIN IN ADH4 5'REGION.
   0.22:35:48
   SACCHAROMYCES CEREVISIAE (BAKER'S YEAST).
   P53056
F-PLACE1005383
   FIBRILLIN 1 PRECURSOR.
   6.7e-09:134:32
   MUS MUSCULUS (MOUSE).
   Q61554
F-PLACE1005410
   PROTEIN TRANSPORT PROTEIN SEC61 ALPHA SUBUNIT.
   9.5e-105:204:100
   RATTUS NORVEGICUS (RAT).
   P38378
F-PLACE1005426
   PREGNANCY-SPECIFIC BETA-1-GLYCOPROTEIN 4 PRECURSOR (PSBG-4).
   3.2e-33:184:46
   HOMO SAPIENS (HUMAN).
   Q00888
F-PLACE1005519
   SERINE/THREONINE-PROTEIN KINASE NRK1 (EC 2.7.1.-) (N-RICH KINASE 1).
   1.2e-23:143:41
   SACCHAROMYCES CEREVISIAE (BAKER'S YEAST).
   P38692
F-PLACE1005539
   ANTER-SPECIFIC PROLINE-RICH PROTEIN APG (PROTEIN CEX) (FRAGMENT).
   5.5e-05:94:37
   BRASSICA NAPUS (RAPE).
   P40603
F-PLACE1005544
   CELL SURFACE A33 ANTIGEN PRECURSOR.
   0.00015:132:31
   HOMO SAPIENS (HUMAN).
   Q99795
F-PLACE1005569
   PROTEOGLYCAN LINK PROTEIN PRECURSOR (CARTILAGE LINK PROTEIN) (LP).
   0.00092:122:31
   EQUUS CABALLUS (HORSE).
   Q28381
F-PLACE1005601
   TOXIN S4C8.
   0.34:32:37
   DENDROASPIS JAMESONI KAIMOSAE (EASTERN JAMESON'S MAMBA).
   P25683
F-PLACE1005660
   SALIVARY GLUE PROTEIN SGS-7 PRECURSOR.
   0.99:41:43
   DROSOPHILA MELANOGASTER (FRUIT FLY).
   P02841
F-PLACE1005669
   COLLAGEN ALPHA 1(VII) CHAIN PRECURSOR (LONG-CHAIN COLLAGEN) (LC COLLAGEN).
   0.0078:105:37
   HOMO SAPIENS (HUMAN).
   Q02388
F-PLACE1005682
   THYROID RECEPTOR INTERACTING PROTEIN 9 (TRIP9).
   2.7e-12:81:41
   HOMO SAPIENS (HUMAN).
   Q15653
F-PLACE1005725
   HYPOTHETICAL 55.1 KD PROTEIN B0416.5 IN CHROMOSOME X.
   7.5e-08:142:31
   CAENORHABDITIS ELEGANS.
   Q11073
F-PLACE1005736
   RAC-FAMILY SERINE/THREONINE KINASE HOMOLOG (EC 2.7.1.-).
   9.0e-11:91:37
   DICTYOSTELIUM DISCOIDEUM (SLIME MOLD).
   P54644
F-PLACE1005745
   ORM1 PROTEIN.
   2.2e-18:137:35
   SACCHAROMYCES CEREVISIAE (BAKER'S YEAST).
   P53224
F-PLACE1005768
   NEUROTOXINS I AND I'PRECURSOR (AAH I AND AAH I').
   0.63:13:69
   ANDROCTONUS AUSTRALIS HECTOR (SAHARA SCORPION). P01479
F-PLACE1005815
   COLORECTAL MUTANT CANCER PROTEIN (MCC PROTEIN).
   1.8e-12:73:50
   HOMO SAPIENS (HUMAN).
   P23508
F-PLACE1005878
   CHLORINE CHANNEL PROTEIN P64.
   1.6e-49:115:79
   BOS TAURUS (BOVINE).
   P35526
F-PLACE1005927
   HYPOTHETICAL 35.8 KD PROTEIN C12G12.12 IN CHROMOSOME I.
   3.2e-16:152:34
   SCHIZOSACCHAROMYCES POMBE (FISSION YEAST).
   Q09875
F-PLACE1006071
   LAMININ BETA-1 CHAIN PRECURSOR (LAMININ B1 CHAIN).
   4.1e-08:215:26
   MUS MUSCULUS (MOUSE).
   P02469
F-PLACE1006073
   SPIDROIN 2 (DRAGLINE SILK FIBROIN 2) (FRAGMENT).
   2.1e-05:137:34
   NEPHILA CLAVIPES (ORB SPIDER).
   P46804
F-PLACE1006079
   HOMEOBOX PROTEIN DLX-3.
   1.5e-58:144:83
   HOMO SAPIENS (HUMAN).
   060479
F-PLACE1006093
   SYNAPSINS IA AND IB (BRAIN PROTEIN 4.1).
   3.8e-05:72:40
   HOMO SAPIENS (HUMAN).
   P17600
F-PLACE1006208
   EBNA-2 NUCLEAR PROTEIN.
   3.8e-15:28:75
   EPSTEIN-BARR VIRUS (STRAIN B95-8) (HUMAN HERPESVIRUS 4). P12978
F-PLACE1006219
   UDP-GLUCOSE 4-EPIMERASE (EC 5.1.3.2) (GALACTOWALDENASE) (UDP-GALACTOSE 4-EPIMERASE) (FRAGMENT)
   2.0e-09:38:42
   KLEBSIELLA PNEUMONIAE.
   P45602
F-PLACE1006277
   CELL SURFACE A33 ANTIGEN PRECURSOR.
   1.2e-07:183:29
   HOMO SAPIENS (HUMAN).
   Q99795
F-PLACE1006290
   GLYCOSYLTRANSFERASE ALG2 (EC 2.4.1.-).
   8.2e-39:171:43
   SACCHAROMYCES CEREVISIAE (BAKER'S YEAST).
   P43636
F-PLACE1006443
   HYPOTHETICAL 60.0 KD PROTEIN IN IMP1-HLJ1 INTERGENIC REGION.
   0.0010:155:27
   SACCHAROMYCES CEREVISIAE (BAKER'S YEAST).
   Q03795
F-PLACE1006515
   ZINC FINGER Y-CHROMOSOMAL PROTEIN 1.
   0.046:98:28
   MUS MUSCULUS (MOUSE).
   P10925
F-PLACE1006716
   30 KD ADIPOCYTE COMPLEMENT-RELATED PROTEIN PRECURSOR (ACRP30) (ADIPOCYTE SPECIFIC PROTEIN ADIPOQ).
   3.6e-25:177:35
   MUS MUSCULUS (MOUSE).
   Q60994
F-PLACE1006786
   PROBABLE NONSPECIFIC LIPID-TRANSFER PROTEIN (LTP) (BASIC PROTEIN) (WBP) (FRAGMENT).
   1.0:19:42
   TRITICUM AESTIVUM (WHEAT).
   P26913
F-PLACE1006809
   SLS1 PROTEIN PRECURSOR.
   0.0011:37:51
   YARROWIA LIPOLYTICA (CANDIDA LIPOLYTICA).
   Q99158
F-PLACE1006959
   PROLINE-RICH PROTEIN MP-2 PRECURSOR.
   5.3e-05:96:41
   MUS MUSCULUS (MOUSE).
   P05142
F-PLACE1007028
   EBNA-1 NUCLEAR PROTEIN.
   5.9e-09:219:33
   EPSTEIN-BARR VIRUS (STRAIN B95-8) (HUMAN HERPESVIRUS 4).
   P03211
F-PLACE1007040
   SALIVARY PROLINE-RICH PROTEIN PRECURSOR (CLONE CP7) [CONTAINS: BASIC PEPTIDE P-F] (FRAGMENT).
   0.68:138:24
   HOMO SAPIENS (HUMAN).
   P02812
F-PLACE1007077
   SERINE/THREONINE-PROTEIN KINASE CLA4 (EC 2.7.1.-).
   0.73:177:25
   SACCHAROMYCES CEREVISIAE (BAKER'S YEAST).
   P48562
F-PLACE1007081
   COAGULATION FACTOR V PRECURSOR (ACTIVATED PROTEIN C COFACTOR).
   3.0e-20:182:39
   BOS TAURUS (BOVINE).
   Q28107
F-PLACE1007096
   HYPOTHETICAL SYMPORTER SLL1374.
   2.8e-14:162:30
   SYNECHOCYSTIS SP. (STRAIN PCC 6803).
   P74168
F-PLACE1007296
   ER LUMEN PROTEIN RETAINING RECEPTOR 1 (KDEL RECEPTOR 1).
   9.4e-50:120:86
   HOMO SAPIENS (HUMAN).
   P24390
F-PLACE1007591
   MEIOTIC RECOMBINATION PROTEIN REC104.
   0.68:73:31
   SACCHAROMYCES CEREVISIAE (BAKER'S YEAST).
   P33323
F-PLACE1007626
   PTB-ASSOCIATED SPLICING FACTOR (PSF).
   0.00083:97:34
   HOMO SAPIENS (HUMAN).
   P23246
F-PLACE1007702
   ANTER-SPECIFIC PROLINE-RICH PROTEIN APG PRECURSOR.
   1.9e-08:87:36
   ARABIDOPSIS THALIANA (MOUSE-EAR CRESS).
   P40602
F-PLACE1007845
   GLYCOSYLTRANSFERASE ALG2 (EC 2.4.1.-).
   1.3e-16:158:40
   SACCHAROMYCES CEREVISIAE (BAKER'S YEAST).
   P43636
F-PLACE1007881
   HYPOTHETICAL 69.4 KD PROTEIN F13H10.3 IN CHROMOSOME IV.
   1.2e-11:113:37
   CAENORHABDITIS ELEGANS.
   Q19425
F-PLACE1007971
   METALLOTHIONEIN 20-III ISOFORMS A AND B (MT-20-IIIA AND MT-20-IIIB).
   1.0:32:43
   MYTILUS EDULIS (BLUE MUSSEL).
   P80253
F-PLACE1008282
   HEME-REGULATED EUKARYOTIC INITIATION FACTOR EIF-2-ALPHA KINASE (EC 2.7.1.-) (HRI).
   8.1e-87:178:87
   ORYCTOLAGUS CUNICULUS (RABBIT).
   P33279
F-PLACE1008297
   MYOSIN HEAVY CHAIN KINASE B (EC 2.7.1.129) (MHCK B).
   3.6e-17:187:33
   DICTYOSTELIUM DISCOIDEUM (SLIME MOLD).
   P90648
F-PLACE1008359
   BEM46 PROTEIN (FRAGMENT).
   4.9e-07:103:33
   SCHIZOSACCHAROMYCES POMBE (FISSION YEAST).
   P54069
F-PLACE1008469
   D(4) DOPAMINE RECEPTOR (D(2C) DOPAMINE RECEPTOR).
   0.0018:78:37
   HOMO SAPIENS (HUMAN).
   P21917
F-PLACE1008549
   FLI-1 ONCOGENE (ERGB TRANSCRIPTION FACTOR).
   0.0034:89:30
   HOMO SAPIENS (HUMAN).
   Q01543
F-PLACE1008657
   ADSEVERIN (SCINDERIN) (SC).
   6.7e-127:257:91
   BOS TAURUS (BOVINE).
   Q28046
F-PLACE1008716
   ALPHA-1,6-MANNOSYL-GLYCOPROTEIN BETA-1,2-N-ACETYLGLUCOSAMINYLTRANSFERASE (EC 2.4.1.143) (N-GLYCOSYL-OLIGOSACCHARIDE-GLYCOPROTEIN N-ACETYLGLUCOSAMINYLTRANSFERASE II) (BETA-1,2-N-ACETYLGLUCOSAMINYLTRANSFERASE II) (GNT-II) (GLCNAC-T II).
   4.5e-20:66:78
   HOMO SAPIENS (HUMAN).
   Q10469
F-PLACE1008744
   C4B-BINDING PROTEIN ALPHA CHAIN PRECURSOR (PROLINE-RICH PROTEIN) (PRP).
   3.6e-19:221:33
   HOMO SAPIENS (HUMAN).
   P04003
F-PLACE1008984
   BIOTIN CARBOXYL CARRIER PROTEIN OF ACETYL-COA CARBOXYLASE PRECURSOR (EC 6.4.1.2) (BCCP).
   0.089:61:31
   GLYCINE MAX (SOYBEAN).
   Q42783
F-PLACE1008985
   SYNAPTOTAGMIN V.
   8.6e-09:123:35
   HOMO SAPIENS (HUMAN).
   000445
F-PLACE1009067
   HYPOTHETICAL 33.4 KD PROTEIN.
   4.3e-09:60:50
   HOMO SAPIENS (HUMAN).
   Q04323
F-PLACE1009196
   SPERM PROTAMINE P1 (CYSTEINE-RICH PROTAMINE).
   0.050:23:34
   GORILLA GORILLA GORILLA (LOWLAND GORILLA).
   P35303
F-PLACE1009279
   8.6 KD TRANSGLUTAMINASE SUBSTRATE.
   1.4e-07:62:35
   TACHYPLEUS TRIDENTATUS (JAPANESE HORSESHOE CRAB).
   P81281
F-PLACE1009527
   MUCIN 2 PRECURSOR (INTESTINAL MUCIN 2).
   0.037:71:38
   HOMO SAPIENS (HUMAN).
   Q02817
F-PLACE1009546
   PROLINE-RICH PROTEIN MP-2 PRECURSOR.
   5.9e-07:86:39
   MUS MUSCULUS (MOUSE).
   P05142
F-PLACE1009600
   TETRACYCLINE RESISTANCE PROTEIN, CLASS H (TETA(H)).
   1.7e-08:113:31
   PASTEURELLA MULTOCIDA.
   P51564
F-PLACE1009735
   TARNS-ACTING TRANSCRIPTIONAL PROTEIN ICP0 (IMMEDIATE-EARLY PROTEIN IE1 10) (VMW110) (ALPHA-0 PROTEIN).
   2.6e-09:182:35
   HERPES SIMPLEX VIRUS (TYPE 1 / STRAIN 17).
   P08393
F-PLACE1009982
   REGULATORY PROTEIN E2.
   0.99:94:28
   HUMAN PAPILLOMAVIRUS TYPE 8.
   P06422
F-PLACE1010011
   DIACYLGLYCEROL CHOLINEPHOSPHOTRANSFERASE (EC 2.7.8.2) (SN-1,2-DIACYLGLYCEROL CHOLINEPHOSPHOTRANSFERASE) (CHOPT).
   2.8e-20:119:42
   SACCHAROMYCES CEREVISIAE (BAKER'S YEAST).
   P17898
F-PLACE1010078
   ORM1 PROTEIN.
   3.4e-20:137:37
   SACCHAROMYCES CEREVISIAE (BAKER'S YEAST).
   P53224
F-PLACE1010081
   SERINE/THREONINE-PROTEIN KINASE CTR1 (EC 2.7.1.37).
   1.5e-11:147:32
   ARABIDOPSIS THALIANA (MOUSE-EAR CRESS).
   Q05609
F-PLACE1010251
   NEL-LIKE PROTEIN (FRAGMENT).
   1.8e-10:73:42
   HOMO SAPIENS (HUMAN).
   Q92832
F-PLACE1010445
   HYPOTHETICAL BHLF1 PROTEIN.
   0.0042:227:33
   EPSTEIN-BARR VIRUS (STRAIN B95-8) (HUMAN HERPESVIRUS 4).
   P03181
F-PLACE1010713
   PUTATIVE STEROID DEHYDROGENASE KIK-I (EC 1.1.1.-).
   1.5e-77:177:80
   MUS MUSCULUS (MOUSE).
   070503
F-PLACE1010784
   P2Y PURINOCEPTOR 5 (P2Y5) (PURINERGIC RECEPTOR 5) (6H1).
   1.7e-18:102:40
   GALLUS GALLUS (CHICKEN).
   P32250
F-PLACE1010827
   COP-COATED VESICLE MEMBRANE PROTEIN P24 PRECURSOR (FRAGMENT).
   2.8e-18:109:41
   CRICETULUS GRISEUS (CHINESE HAMSTER).
   P49020
F-PLACE1010968
   PROTEIN-TYROSINE PHOSPHATASE DLAR PRECURSOR (EC 3.1.3.48) (PROTEIN-TYROSINE-PHOSPHATE PHOSPHOHYDROLASE).
   2.3e-06:191:28
   DROSOPHILA MELANOGASTER (FRUIT FLY).
   P16621
F-PLACE1011045
   HYPOTHETICAL 71.4 KD PROTEIN IN NMD3-ENO2 INTERGENIC REGION.
   6.0e-14:153:34
   SACCHAROMYCES CEREVISIAE (BAKER'S YEAST).
   P38862
F-PLACE1011116
   GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE).
   2.3e-06:195:27
   SACCHAROMYCES CEREVISIAE (BAKER'S YEAST).
   P08640
F-PLACE1011181
   MSP1 PROTEIN HOMOLOG.
   4.3e-06:93:25
   CAENORHABDITIS ELEGANS.
   P54815
F-PLACE1011236
   HYPOTHETICAL 59.1 KD PROTEIN ZK637.1 IN CHROMOSOME III.
   4.1e-17:180:28
   CAENORHABDITIS ELEGANS.
   P30638
F-PLACE1011364
   HYPOTHETICAL 141.2 KD PROTEIN EEED8.9 IN CHROMOSOME II.
   2.1e-24:158:41
   CAENORHABDITIS ELEGANS.
   Q09298
F-PLACE1011407
   ZINC FINGER PROTEIN 140.
   3.8e-10:47:74
   HOMO SAPIENS (HUMAN).
   P52738
F-PLACE1011516
   HYPOTHETICAL 21.5 KD PROTEIN IN SEC15-SAP4 INTERGENIC REGION.
   1.6e-13:117:34
   SACCHAROMYCES CEREVISIAE (BAKER'S YEAST).
   P53073
F-PLACE1011708
   DORSAL-VENTRAL PATTERNING TOLLOID PROTEIN PRECURSOR (EC 3.4.24.-).
   9.9e-22:203:32
   DROSOPHILA MELANOGASTER (FRUIT FLY).
   P25723
F-PLACE1011824
   SERINE/THREONINE-PROTEIN KINASE PAK-BETA (EC 2.7.1.-) (BETA-PAK) (P21-ACTIVATED KINASE 3) (CDC42/RAC EFFECTOR KINASE PAK-B).
   1.6e-15:103:36
   MUS MUSCULUS (MOUSE).
   Q61036
F-PLACE1011978
   ZINC FINGER PROTEIN 85 (ZINC FINGER PROTEIN HPF4) (HTF1).
   3.3e-55:188:50
   HOMO SAPIENS (HUMAN).
   Q03923
F-PLACE2000118
   EXTENSIN PRECURSOR (CELL WALL HYDROXYPROLINE-RICH GLYCOPROTEIN).
   2.8e-23:169:43
   NICOTIANA TABACUM (COMMON TOBACCO).
   P13983
F-PLACE2000219
   MALE SPECIFIC SPERM PROTEIN MST84DA.
   0.11:29:41
   DROSOPHILA MELANOGASTER (FRUIT FLY).
   Q01642
F-PLACE3000181
   CADHERIN-RELATED TUMOR SUPPRESSOR PRECURSOR (FAT PROTEIN).
   9.5e-26:193:37
   DROSOPHILA MELANOGASTER (FRUIT FLY).
   P33450
F-PLACE3000213
   COMPLEMENT RECEPTOR TYPE 1 PRECURSOR (C3B/C4B RECEPTOR) (CD35 ANTIGEN).
   2.3e-23:191:34
   HOMO SAPIENS (HUMAN).
   P17927
F-PLACE4000354
   E-SELECTIN PRECURSOR (ENDOTHELIAL LEUKOCYTE ADHESION MOLECULE 1) (ELAM-1) (LEUKOCYTE-ENDOTHELIAL CELL ADHESION MOLECULE 2) (LECAM2) (CD62E).
   3.2e-25:150:30
   ORYCTOLAGUS CUNICULUS (RABBIT).
   P27113
F-PLACE4000455
   IG KAPPA CHAIN PRECURSOR V-III REGION (VG) (FRAGMENT).
   0.66:52:36
   HOMO SAPIENS (HUMAN).
   P04433
F-SKNMC1000004
   OPTOMOTOR-BLIND PROTEIN (LETHAL(1)OPTOMOTOR-BLIND) (L(1)OMB) (BIFID PROTEIN).
   0.079:88:30
   DROSOPHILA MELANOGASTER (FRUIT FLY).
   Q24432
F-SKNMC1000014
   SCO-SPONDIN (FRAGMENT).
   0.63:60:36
   BOS TAURUS (BOVINE).
   P98167
F-SKNMC1000082
   PUTATIVE MITOCHONDRIAL CARRIER YGR096W.
   2.4e-10:93:34
   SACCHAROMYCES CEREVISIAE (BAKER'S YEAST).
   P53257
F-THYRO1000036
   PROLINE-RICH PROTEIN MP-3 (FRAGMENT).
   0.72:69:36
   MUS MUSCULUS (MOUSE).
   P05143
F-THYRO1000061
   COLLAGEN ALPHA 1(XII) CHAIN (FRAGMENTS).
   0.0068:70:38
   BOS TAURUS (BOVINE).
   P25508
F-THYRO1000099
   SPIDROIN 2 (DRAGLINE SILK FIBROIN 2) (FRAGMENT).
   0.0063:207:28
   NEPHILA CLAVIPES (ORB SPIDER).
   P46804
F-THYRO1000196
   RETINAL-CADHERIN PRECURSOR (R-CADHERIN) (R-CAD).
   1.6e-10:134:32
   GALLUS GALLUS (CHICKEN).
   P24503
F-THYRO1000400
   ERYTHROCYTE BAND 7 INTEGRAL MEMBRANE PROTEIN (STOMATIN) (PROTEIN 7.2B).
   3.9e-28:163:38
   MUS MUSCULUS (MOUSE).
   P54116
F-THYRO1000580
   RENAL TRANSCRIPTION FACTOR KID-1 (TRANSCRIPTION FACTOR 17).
   3.3e-15:64:62
   MUS MUSCULUS (MOUSE).
   Q61751
F-THYRO1000584
   EPIDIDYMIS-SPECIFIC ALPHA-MANNOSIDASE (EC 3.2.1.24) (ALPHA-D-MANNOSIDE MANNOHYDROLASE) (135 KD PROTEIN).
   1.5e-89:197:72
   SUS SCROFA (PIG).
   Q28949
F-THYRO1000678
   GAP JUNCTION BETA-6 PROTEIN (CONNEXIN 30) (CX30).
   7.7e-39:89:87
   MUS MUSCULUS (MOUSE).
   P70689
F-THYRO1000776
   HIGH AFFINITY SULPHATE TRANSPORTER 2.
   3.0e-25:83:50
   STYLOSANTHES HAMATA.
   P53392
F-THYRO1000795
   MITOCHONDRIAL 2-OXOGLUTARATE/MALATE CARRIER PROTEIN (OGCP).
   1.2e-33:227:37
   BOS TAURUS (BOVINE).
   P22292
F-THYRO1000846
   CUTICLE COLLAGEN 12 PRECURSOR.
   6.7e-09:190:33
   CAENORHABDITIS ELEGANS.
   P20630
F-THYRO1000866
   HYPOTHETICAL 146.8 KD PROTEIN C34E10.5 IN CHROMOSOME III.
   0.12:85:31
   CAENORHABDITIS ELEGANS.
   P46580
F-THYRO1000956
   PROBABLE G PROTEIN-COUPLEI) RECEPTOR APJ.
   1.3e-68:165:84
   HOMO SAPIENS (HUMAN).
   P35414
F-THYRO1000964
   TARNS-ACTING TRANSCRIPTIONAL PROTEIN ICP0 (P135 PROTEIN) (IER 2.9/ER2.6).
   0.015:170:34
   BOVINE HERPESVIRUS TYPE 1 (STRAIN JURA).
   P29128
F-THYRO1000999
   CRYPTDIN-RELATED PROTEIN 4C-2 PRECURSOR (CRS4C).
   0.28:40:45
   MUS MUSCULUS (MOUSE).
   P50715
F-THYRO1001063
   SALIVARY PROLINE-RICH PROTEIN PRECURSOR (CLONES CP3, CP4 AND CP5) [CONTAINS: BASIC PEPTIDE IB-6; PEPTIDE P-H].
   3.5e-05:232:32
   HOMO SAPIENS (HUMAN).
   P04280
F-THYRO1001071
   SKIN SECRETORY PROTEIN XP2 PRECURSOR (APEG PROTEIN).
   0.00061:131:33
   XENOPUS LAEVIS (AFRICAN CLAWED FROG).
   P17437
F-THYRO1001102
   TRANSCRIPTION INITIATION FACTOR TFIID 135 KD SUBUNIT (TAFII-135) (TAFII135) (TAFII-130) (TAFII130).
   0.25:94:38
   HOMO SAPIENS (HUMAN).
   000268
F-THYRO1001113
   SYNAPTOTAGMIN III (SYTIII).
   2.0e-08:102:35
   MUS MUSCULUS (MOUSE).
   035681
F-THYRO1001128
   GLYCOPROTEIN X PRECURSOR.
   6.8e-07:182:31
   EQUINE HERPESVIRUS TYPE 1 (STRAIN AB4P) (EHV-1).
   P28968
F-THYRO1001205
   NEUROGRANIN (NG) (PROTEIN KINASE C SUBSTRATE 7.5 KD PROTEIN) (RC3).
   0.91:33:42
   RATTUS NORVEGICUS (RAT).
   Q04940
F-THYRO1001237
   HYPOTHETICAL PROTEIN IN NIFH2 3 REGION (FRAGMENT).
   4.0e-07:68:38
   METHANOCOCCUS THERMOLITHOTROPHICUS.
   P05410
F-THYRO1001242
   SYNAPSINS IA AND IB (BRAIN PROTEIN 4.1).
   1.0:104:35
   HOMO SAPIENS (HUMAN).
   P17600
F-THYRO1001266
   SODIUM/GLUCOSE COTRANSPORTER 1 (NA(+)/GLUCOSE COTRANSPORTER 1) (HIGH AFFINITY SODIUM-GLUCOSE COTRANSPORTER).
   4.3e-09:119:27
   ORYCTOLAGUS CUNICULUS (RABBIT).
   P11170
F-THYR01001327
   HYPOTHETICAL 23.7 KD PROTEIN IN CYR1-OST1 INTERGENIC REGION.
   1.7e-06:141:24
   SACCHAROMYCES CEREVISIAE (BAKER'S YEAST).
   P41544
F-THYRO1001456
   HYPOTHETICAL 56.2 KD PROTEIN CY31.25C.
   1.1e-11:88:48
   MYCOBACTERIUM TUBERCULOSIS.
   Q10555
F-THYRO1001457
   PROTEIN KINASE C, MU TYPE (EC 2.7.1.-) (NPKC-MU).
   2.1e-68:228:59
   HOMO SAPIENS (HUMAN).
   Q15139
F-THYRO1001471
   COLLAGEN 1(X) CHAIN PRECURSOR.
   3.9e-05:204:30
   GALLUS GALLUS (CHICKEN).
   P08125
F-THYRO1001478
   COLLAGEN ALPHA 1(X) CHAIN PRECURSOR.
   0.038:162:31
   HOMO SAPIENS (HUMAN).
   Q03692
F-THYRO1001495
   !!!! ALU SUBFAMILY SP WARNING ENTRY !!!!
   4.8e-19:50:82
   HOMO SAPIENS (HUMAN).
   P39193
F-THYRO1001523
   !!!! ALU SUBFAMILY SX WARNING ENTRY ! ! ! !
   5.0e-13:66:62
   HOMO SAPIENS (HUMAN).
   P39195
F-THYRO1001529
   SERINE PALMITOYLTRANSFERASE 2 (EC 2.3.1.50) (LONG CHAIN BASE BIOSYNTHESIS PROTEIN 2) (SPT 2).
   1.6e-27:115:53
   SCHIZOSACCHAROMYCES POMBE (FISSION YEAST).
   Q09925
F-THYRO1001593
   PUTATIVE SERINE/THREONINE-PROTEIN KINASE P78 (EC 2.7.1.-).
   3.3e-92:225:77
   HOMO SAPIENS (HUMAN).
   P27448
F-THYRO1001608
   PROLINE-RICH PROTEIN MP-2 PRECURSOR.
   1.2e-07:127:35
   MUS MUSCULUS (MOUSE).
   P05142
F-THYRO1001641
   NUC-1 NEGATIVE REGULATORY PROTEIN PREG.
   0.0039:98:31
   NEUROSPORA CRASSA.
   Q06712
F-THYRO1001700
   INTERFERON-INDUCED, DOUBLE-STRANDED RNA-ACTIVATED PROTEIN KINASE (EC 2.7.1.-) (INTERFERON-INDUCIBLE RNA-DEPENDENT PROTEIN KINASE) (P68 KINASE) (P1/EIF-2A PROTEIN KINASE).
   3.3e-09:65:43
   HOMO SAPIENS (HUMAN).
   P19525
F-THYRO1001702
   MYELOID UPREGULATED PROTEIN.
   7.8e-62:161:78
   MUS MUSCULUS (MOUSE).
   035682
F-THYRO1001725
   PROTEIN KINASE C SUBSTRATE 80 KD PROTEIN (FRAGMENTS).
   0.00061:82:41
   RATTUS NORVEGICUS (RAT).
   P20468
F-THYRO1001770
   PROBABLE SERINE/THREONINE-PROTEIN KINASE YNL020C (EC 2.7.1.-).
   1.0e-20:165:35
   SACCHAROMYCES CEREVISIAE (BAKER'S YEAST).
   P53974
F-THYRO1001803
   GLUCOAMYLASE S1/S2 PRECURSOR (EC 3.2.1.3) (GLUCAN 1,4-ALPHA-GLUCOSIDASE) (1,4-ALPHA-D-GLUCAN GLUCOHYDROLASE).
   3.6e-07:221:30
   SACCHAROMYCES CEREVISIAE (BAKER'S YEAST).
   P08640
F-Y79AA1000030
   TRANSCRIPTIONAL ACTIVATOR FE65.
   4.5e-09:43:46
   RATTUS NORVEGICUS (RAT).
   P46933
F-Y79AA1000127
   FAS ANTIGEN LIGAND (APOPTOSIS ANTIGEN LIGAND) (APTL).
   1.3e-05:72:43
   HOMO SAPIENS (HUMAN).
   P48023
F-Y79AA1000207
   STANNIOCALCIN PRECURSOR (STC) (CORPUSCLES OF STANNIUS PROTEIN) (CS) (HYPOCALCIN) (TELEOCALCIN).
   1.0:100:27
   ANGUILLA AUSTRALIS (AUSTRALIAN EEL).
   P18301
F-Y79AA1000226
   HYPOTHETICAL 52.8 KD PROTEIN T05E11.5 IN CHROMOSOME IV.
   2.6e-07:188:28
   CAENORHABDITIS ELEGANS.
   P49049
F-Y79AA1000270
   VACUOLAR ATP SYNTHASE SUBUNIT AC45 PRECURSOR (EC 3.6.1.34) (V-ATPASE AC45 SUBUNIT).
   1.6e-102:233:87
   BOS TAURUS (BOVINE).
   P40682
F-Y79AA1000426
   INHIBIN BETA C CHAIN PRECURSOR (ACTIVIN BETA-C CHAIN).
   1.1e-14:149:38
   HOMO SAPIENS (HUMAN).
   P55103
F-Y79AA1000521
   MALE SPECIFIC SPERM PROTEIN MST84DD.
   0.00079:60:36
   DROSOPHILA MELANOGASTER (FRUIT FLY).
   Q01645
F-Y79AA1000750
   EBNA-1 NUCLEAR PROTEIN.
   2.0e-09:131:38
   EPSTEIN-BARR VIRUS (STRAIN B95-8) (HUMAN HERPESVIRUS 4).
   P03211
F-Y79AA1000776
   CORNIFIN (SMALL PROLINE-RICH PROTEIN I) (SPR-I) (SMALL PROLINE-RICH SQUAMOUS CELL MARKER) (SPRP).
   0.080:44:40
   SUS SCROFA (PIG).
   P35323
F-Y79AA1000777
   PUTATIVE SERINE/THREONINE-PROTEIN KINASE PKWA (EC 2.7.1.-).
   4.2e-33:204:39
   THERMOMONOSPORA CURVATA.
   P49695
F-Y79AA1000876
   PROTEIN DISULFIDE ISOMERASE PRECURSOR (PDI) (EC 5.3.4.1) (PROLYL 4-HYDROXYLASE BETA SUBUNIT) (CELLULAR THYROID HORMONE BINDING PROTEIN) (P55).
   4.6e-16:115:38
   BOS TAURUS (BOVINE).
   P05307
F-Y79AA1000888
   TRNA PSEUDOURIDINE SYNTHASE A (EC 4.2.1.70) (PSEUDOURIDYLATE SYNTHASE I) (PSEUDOURIDINE SYNTHASE I) (URACIL HYDROLYASE).
   2.0e-09:159:35
   TREPONEMA PALLIDUM.
   083802
F-Y79AA1000959
   HOMEOBOX PROTEIN HOX-B3 (HOX-2.7) (MH-23).
   8.8e-08:72:38
   MUS MUSCULUS (MOUSE).
   P09026
F-Y79AA1000967
   CALCIUM/CALMODULIN-DEPENDENT PROTEIN KINASE TYPE I (EC 2.7.1.123) (CAM KINASE I).
   1.1e-37:202:42
   RATTUS NORVEGICUS (RAT).
   Q63450
F-Y79AA1001013
   SALIVARY PROLINE-RICH PROTEIN PO (ALLELE K) [CONTAINS : PEPTIDE P-D] (FRAGMENT).
   0.038:128:28
   HOMO SAPIENS (HUMAN).
   P10162
F-Y79AA1001056
   HYPOTHETICAL 7.1 KD PROTEIN IN TK-VS INTERGENIC REGION.
   0.41:42:30
   BACTERIOPHAGE T4.
   P13307
F-Y79AA1001062
   TUMOR NECROSIS FACTOR, ALPHA-INDUCED PROTEIN 1, ENDOTHELIAL (B12 PROTEIN).
   9.9e-13:132:38
   HOMO SAPIENS (HUMAN).
   Q13829
F-Y79AA1001090
   ANKYRIN HOMOLOG PRECURSOR.
   4.0e-19:176:34
   CHROMATIUM VINOSUM.
   Q06527
F-Y79AA1001212
   HYPOTHETICAL PROTEIN MJ0110.
   0.095:55:34
   METHANOCOCCUS JANNASCHII.
   Q57574
F-Y79AA1001264
   HYPOTHETICAL 39.9 KD PROTEIN T15H9.1 IN CHROMOSOME II PRECURSOR.
   3.3e-53:177:55
   CAENORHABDITIS ELEGANS.
   Q10005
F-Y79AA1001272
   ACROSIN PRECURSOR (EC 3.4.21.10).
   6.3e-08:78:46
   ORYCTOLAGUS CUNICULUS (RABBIT).
   P48038
F-Y79AA1001328
   DELTA-LIKE PROTEIN 1 PRECURSOR (DELTA1).
   1.3e-08:118:39
   RATTUS NORVEGICUS (RAT).
   P97677
F-Y79AA1001426
   BAND 3 ANION TRANSPORT PROTEIN.
   1.7e-18:156:32
   GALLUS GALLUS (CHICKEN).
   P15575
F-Y79AA1001427
   INDUCIBLE NITRATE REDUCTASE 2 (EC 1.6.6.1) (NR).
   1.1e-49:131:51
   GLYCINE MAX (SOYBEAN).
   P39870
F-Y79AA1001430
   RING CANAL PROTEIN (KELCH PROTEIN).
   2.5e-24:157:40
   DROSOPHILA MELANOGASTER (FRUIT FLY).
   Q04652
F-Y79AA1001523
   TRANSCRIPTION INTERMEDIARY FACTOR 1-BETA (NUCLEAR COREPRESSOR KAP-1) (KRAB-ASSOCIATED PROTEIN 1).
   6.2e-15:141:39
   HOMO SAPIENS (HUMAN).
   Q13263
F-Y79AA1001530
   TUBULIN BETA-5 CHAIN.
   8.0e-76:204:76
   HOMO SAPIENS (HUMAN).
   P04350
F-Y79AA1001592
   PTB-ASSOCIATED SPLICING FACTOR (PSF).
   0.42:104:33
   HOMO SAPIENS (HUMAN).
   P23246
F-Y79AA1001727
   AMALGAM PROTEIN PRECURSOR.
   1.9e-09:185:28
   DROSOPHILA MELANOGASTER (FRUIT FLY).
   P15364
F-Y79AA1001787
   PROBABLE CALCIUM-TRANSPORTING ATPASE 9 (EC 3.6.1.38).
   7.6e-43:210:45
   SACCHAROMYCES CEREVISIAE (BAKER'S YEAST).
   Q12697
F-Y79AA1001793
   CORNIFIN (SMALL PROLINE-RICH PROTEIN I) (SPR-I) (SMALL PROLINE-RICH SQUAMOUS CELL MARKER) (SPRP).
   0.077:44:40
   SUS SCROFA (PIG).
   P35323
F-Y79AA1001795
   HYPOTHETICAL BHLF1 PROTEIN.
   0.00014:210:31
   EPSTEIN-BARR VIRUS (STRAIN B95-8) (HUMAN HERPESVIRUS 4).
   P03181
F-Y79AA1001799
   MITOCHONDRIAL RNA SPLICING PROTEIN MSR4.
   2.8e-18:107:44
   SACCHAROMYCES CEREVISIAE (BAKER'S YEAST).
   P23500
F-Y79AA1001803
   SECRETOGRANIN III PRECURSOR (SGIII).
   1.3e-68:182:76
   MUS MUSCULUS (MOUSE).
   P47867
F-Y79AA1001863
   GLYCOPROTEIN J.
   0.030:61:32
   HERPES SIMPLEX VIRUS (TYPE 1 / STRAIN 17).
   P06480
F-Y79AA1002022
   WISKOTT-ALDRICH SYNDROME PROTEIN (WASP).
   9.8e-08:127:37
   HOMO SAPIENS (HUMAN).
   P42768
F-Y79AA1002058
   CCAAT/ENHANCER BINDING PROTEIN DELTA (C/EBP DELTA) (NUCLEAR FACTOR NF-IL6-BETA) (NF-IL6-BETA).
   0.28:56:42
   HOMO SAPIENS (HUMAN).
   P49716
F-Y79AA1002121
   D-BINDING PROTEIN (DBP) (ALBUMIN D BOX-BINDING PROTEIN).
   0.71:57:36
   MUS MUSCULUS (MOUSE).
   Q60925
F-Y79AA1002129
   TRANSCRIPTIONAL REGULATORY PROTEIN ALGP (ALGINATE REGULATORY PROTEIN ALGR3).
   0.98:158:24
   PSEUDOMONAS AERUGINOSA.
   P15276
F-Y79AA1002213
   HYPOTHETICAL 52.7 KD PROTEIN C38C10.2 IN CHROMOSOME III.
   4.7e-39:218:41
   CAENORHABDITIS ELEGANS.
   Q03567
F-Y79AA1002334
   HYPOTHETICAL PROTEIN MJ1345.
   1.8e-08:164:26
   METHANOCOCCUS JANNASCHII.
   Q58741
F-Y79AA1002373
   CORNIFIN (SMALL PROLINE-RICH PROTEIN I) (SPR-I) (SMALL PROLINE-RICH SQUAMOUS CELL MARKER) (SPRP).
   0.083:44:40
   SUS SCROFA (PIG).
   P35323
F-Y79AA1002376
   DYNEIN INTERMEDIATE CHAIN 2, CYTOSOLIC (DH IC-2).
   3.0e-91:214:83
   RATTUS NORVEGICUS (RAT).
   Q62871
F-Y79AA1002378
   ZINC FINGER PROTEIN 38 (ZFP-38) (CTFIN51) (TRANSCRIPTION FACTOR RU49).
   1.0e-59:163:74
   MUS MUSCULUS (MOUSE).
   Q07231
F-Y79AA1002381
   CELL DIVISION CONTROL PROTEIN 28 (EC 2.7.1.-).
   9.5e-41:179:38
   SACCHAROMYCES CEREVISIAE (BAKER'S YEAST).
   P00546

### Homology search result 6

The result of the homology search in the GenBank(http://www.ncbi.nlm.nih.gov/web/GenBank/) using the clone sequences of the 5'-ends. except EST and STS sequences
Indicated are from the top,
the name of the clone sequence,
definition of the top hit data,
the P-value: the length of the sequence used for comparison (nucleotide):similarity (%),
the Accession No. of the top hit data.

Data were not shown for the clones in which the P-value was higher than 1.
F-BNGH41000020
   H.sapiens mitochondrial DNA, complete genome.
   6.0e-188:913:97
   X93334
F-BNGH41000087
   Human DNA sequence from clone 1049G16 on chromosome 20q12-13.2 Contains gene similar to GLUCOSAMINE-6-SULFATASE, a nuclear receptor coactivator gene, ESTs, STSs, GSSs, complete sequence.
   7.1e-32:176:99
   AL034418
F-BNGH41000091
   Homo sapiens potassium channel h-eag.
   1.6e-79:687:76
   AJ001366
F-HEMBA1000006
   S.erythraea second and third ORF's of eryA gene, complete cds.
   0.95:243:64
   M63677
F-HEMBA1000121
   Human DNA sequence *** SEQUENCING IN PROGRESS *** from clone 796F18, WORKING DRAFT SEQUENCE.
   5.9e-70:450:89
   AL031291
F-HEMBA1000128
   Plasmodium falciparum DNA *** SEQUENCING IN PROGRESS *** from contig 3-14, complete sequence.
   1.0:274:59
   Z98549
F-HEMBA1000275
   Herpes simplex virus type 2 (strain HG52), complete genome.
   0.036:625:55
   Z86099
F-HEMBA1000300
   Homo sapiens chromosome 17, clone hRPK.178_C_3, complete sequence.
   1.4e-40:343:80
   AC005702
F-HEMBA1000349
   Homo sapiens chromosome 17, clone hRPK.235_I_10, complete sequence.
   7.5e-65:451:72
   AC005922
F-HEMBA1000443
   Human DNA sequence *** SEQUENCING IN PROGRESS *** from clone 222E13, WORKING DRAFT SEQUENCE.
   8.1e-77:216:95
   Z93241
F-HEMBA1000462
   Caenorhabditis elegans cosmid C49H3.
   3.7e-06:98:82
   U42436
F-HEMBA1000477
   Mus musculus BALB/c putative growth factor GDF7 (Gdf7) gene, partial cds.
   9.1e-05:190:65
   U08339
F-HEMBA1000590
   Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
   3.0e-102:209:99
   AL021578
F-HEMBA1000634
   *** SEQUENCING IN PROGRESS *** Homo sapiens chromosome 4, BAC clone C0539L10; HTGS phase 1, WORKING DRAFT SEQUENCE, 15 unordered pieces.
   2.0e-95:460:99
   AC004480
F-HEMBA1000671
   Homo sapiens chromosome 19, BAC CIT-B-393i15 (BC301323), complete sequence.
   1.5e-28:259:69
   AC006116
F-HEMBA1000713
   Homo sapiens 10kD protein (BC10) mRNA, complete cds.
   6.5e-126:442:97
   AF053470
F-HEMBA1000732
   Homo sapiens clone IMAGE Consortium 302831 latent transforming growth factor-beta binding protein 4 mRNA, partial cds.
   1.7e-45:258:94
   AF054502
F-HEMBA1000745
   Streptomyces coelicolor cosmid 3F9.
   3.5e-06:360:61
   AL023862
F-HEMBA1000835
   Homo sapiens fibrillin mRNA, complete cds.
   1.3e-07:151:69
   L13923
F-HEMBA1000875
   Human Krueppel-type zinc finger protein (ZNF169) gene, partial cds.
   2.6e-28:249:81
   U28322
F-HEMBA1000907
   Spermatozopsis similis mRNA for 95 kD basal apparatus-protein.
   3.4e-09:599:60
   AJ001438
F-HEMBA1000940
   Homo sapiens connexin46.6 (Cx46.6) gene, complete cds.
   1.7e-16:307:66
   AF014643
F-HEMBA1000962
   Homo sapiens Chromosome 11q12.2 PAC clone pDJ519o13 containing human gene for ferritin heavy chain (FTH), complete sequence.
   0.00040:497:59
   AC004228
F-HEMBA1001184
   Homo sapiens SH3 domain binding glutamic acid-rich-like protein (SH3BGRL) mRNA, complete cds.
   8.8e-23:404:67
   AF042081
F-HEMBA1001221
   Human transmembrane protein mRNA, complete cds.
   2.4e-42:858:63
   U19878
F-HEMBA1001228
   Human germline oligomeric matrix protein (COMP) mRNA, complete cds.
   1.9e-82:470:91
   L32137
F-HEMBA1001272
   Human Ig gamma-2 heavy chain switch region.
   0.032:549:60
   U39934
F-HEMBA1001296
   H.sapiens mRNA for PQ-rich protein.
   6.9e-07:73:98
   Z50194
F-HEMBA1001297
   Homo sapiens putative transcription factor CA150 mRNA, complete cds.
   9.3e-14:143:81
   AF017789
F-HEMBA1001390
   Mus musculus polymerase I-transcript release factor mRNA, complete cds.
   2.5e-56:464:81
   AF036249
F-HEMBA1001563
   Human DNA sequence from clone 71L16 on chromosome Xp11. Contains a probable Zinc Finger protein (pseudo)gene, an unknown putative gene, a pseudogene with high similarity to part of antigen KI-67, a putative Chondroitin 6-Sulfotransferase LIKE gene and a KIAA0267 LIKE putative Na(+)/H(+) exchanger protein gene. Contains a predicted CpG island, ESTs, STSs and GSSs and genomic markers DXS1003 and DXS1055, complete sequence.
   3.1e-06:210:68
   AL022165
F-HEMBA1001621
   Human G protein-coupled receptor APJ gene, complete cds.
   2.0e-98:516:95
   U03642
F-HEMBA1001878
   Homo sapiens U5 snRNP-specific 40 kDa protein mRNA, complete cds.
   1.0e-170:810:98
   AF090988
F-HEMBA1001886
   Human repressor transcriptional factor (ZNF85) mRNA, complete cds.
   3.3e-114:849:80
   U35376
F-HEMBA1002048
   HS_3058_B2_A11_MR CIT Approved Human Genomic Sperm Library D Homo sapiens genomic clone Plate=3058 Col=22 Row=B, genomic survey sequence.
   3.8e-11:244:66
   AQ103440
F-HEMBA1002131
   Homo sapiens mRNA for KIAA0584 protein, partial cds.
   3.5e-44:709:66
   AB011156
F-HEMBA1002163
   Homo sapiens chromosome X, clone 592, WORKING DRAFT SEQUENCE, 8 unordered pieces.
   2.3e-28:373:71
   AC002489
F-HEMBA1002164
   Human DNA sequence *** SEQUENCING IN PROGRESS *** from clone 167A19, WORKING DRAFT SEQUENCE.
   1.3e-127:493:99
   AL031427
F-HEMBA1002167
   Rattus norvegicus neuroligin I mRNA, complete cds.
   8. 1e-155:850:91
   U22952
F-HEMBA1002178
   Homo sapiens mRNA for KIAA0584 protein, partial cds.
   2.6e-46:794:65
   AB011156
F-HEMBA1002195
   Human lysosomal alpha-mannosidase (manB) gene, 5' flanking region and exon 1.
   7.7e-35:255:86
   U60885
F-HEMBA1002227
   Homo sapiens mRNA for 80K-L protein, complete cds.
   3.8e-137:382:95
   D10522
F-HEMBA1002239
   Homo sapiens Chromosome 16 BAC clone CIT987SK-A-485G10, complete sequence.
   4.5e-43:452:74
   AC003049
F-HEMBA1002316
   Homo sapiens DNA sequence from PAC 29C18 on chromosome 22.
   3.0e-22:609:67
   Z97192
F-HEMBA1002420
   Homo sapiens clone RG031N19, WORKING DRAFT SEQUENCE, 1 unordered pieces.
   4.2e-142:322:98
   AC005632
F-HEMBA1002421
   Human heparan sulfate proteoglycan (HSPG) core protein, 3' end.
   1.3e-165:778:98
   J04621
F-HEMBA1002524
   Homo sapiens clone UWGC:y55c025 from 6p21, complete sequence.
   1.3e-153:313:96
   AC004209
F-HEMBA1002551
   Human potential CENP-C binding target sequence, 0.7 kb clone, partial sequence 2.
   6.1e-16:108:97
   U57994
F-HEMBA1002767
   Homo sapiens chromosome 1p33-p34 beta-1,4-galactosyltransferase mRNA, complete cds.
   1.4e-168:798:98
   AF038660
F-HEMBA1002985
   HS_3165_A2_C08_MR CIT Approved Human Genomic Sperm Library D Homo sapiens genomic clone Plate=3165 Col=16 Row=E, genomic survey sequence.
   1.7e-08:127:76
   AQ142051
F-HEMBA1002992
   RPCI11-67B15.TJ RPCI11 Homo sapiens genomic clone R-67815, genomic survey sequence.
   2.7e-11:119:86
   AQ201833
F-HEMBA1003047
   Homo sapiens intrinsic factor-B12 receptor precursor, mRNA, complete cds.
   4.5e-187:873:99
   AF034611
F-HEMBA1003072
   Gallus gallus single-strand DNA-binding protein csdp mRNA, partial cds.
   4.1e-50:515:73
   U68380
F-HEMBA1003101
   Homo sapiens tyrosylprotein sulfotransferase-2 mRNA, complete cds.
   5.3e-139:671:98
   AF049891
F-HEMBA1003120
   Homo sapiens chromosome 19, BAC CIT-B-393i15 (BC301323), complete sequence.
   3.3e-44:213:73
   AC006116
F-HEMBA1003230
   Homo sapiens UP50 mRNA, complete cds.
   5.5e-183:856:98
   AF093118
F-HEMBA1003294
   Human DNA sequence *** SEQUENCING IN PROGRESS *** from clone 495010, WORKING DRAFT SEQUENCE.
   4.2e-38:558:69
   AL031121
F-HEMBA1003315
   Arabidopsis thaliana genomic DNA, chromosome 5, TAC clone: K9I9, complete sequence.
   1.2e-61:737:68
   AB013390
F-HEMBA1003392
   Homo sapiens LDL receptor-related protein 6 (LRP6) mRNA, complete cds.
   2.9e-183:851:99
   AF074264
F-HEMBA1003399
   Homo sapiens clone DJ0826E18, WORKING DRAFT SEQUENCE, 4 unordered pieces.
   8.7e-16:215:74
   AC005282
F-HEMBA1003487
   H.sapiens DNA sequence.
   0.0075:158:67
   Z22340
F-HEMBA1003497
   Human DNA sequence *** SEQUENCING IN PROGRESS *** from clone 27O5, WORKING DRAFT SEQUENCE.
   1.1e-109:538:98
   AL033529
F-HEMBA1003530
   S.scrofa mRNA for BM88 antigen.
   2.8e-47:644:69
   X82027
F-HEMBA1003602
   Human (lambda) DNA for immunoglobulin light chain.
   2.5e-94:551:91
   D86997
F-HEMBA1003732
   Homo sapiens clone DJ0935K16, complete sequence.
   6.1e-151:777:96
   AC006011
F-HEMBA1003945
   Homo sapiens clone 638 unknown mRNA, complete sequence.
   1.8e-76:310:93
   AF091085
F-HEMBA1004007
F-HEMBA1004067
   Human DNA sequence from clone 612B18 on chromosome 1q24-25.3 Contains exon from gene similar to 40S ribosomal protein, first coding exon of dynamin 2 (DYNII). ESTs, STS, GSS, CpG Island, complete sequence.
   8.7e-133:718:94
   AL031864
F-HEMBA1004085
   Homo sapiens, clone hRPK.2_A_1, complete sequence.
   2.7e-58:256:80
   AC006197
F-HEMBA1004110
   Homo sapiens intersectin short form mRNA, complete cds.
   3.8e-159:779:96
   AF064243
F-HEMBA1004250
   Homo sapiens chromosome 5, BAC clone 182a8 (LBNL H161), complete sequence.
   1.2e-183:863:99
   AC005752
F-HEMBA1004391
   H.sapiens gene for neural cell adhesion molecule L1.
   0.51:426:59
   Z29373
F-HEMBA1004444
   Homo sapiens clone DJ0971C03, WORKING DRAFT SEQUENCE, 18 unordered pieces.
   3.3e-147:463:93
   AC004938
F-HEMBA1004454
   Homo sapiens tetraspan NET-4 mRNA, complete cds.
   0.00036:230:62
   AF065389
F-HEMBA1004505
   D.melanogaster mRNA for alpha 1,2 mannosidase.
   5.5e-17:663:58
   X82640
F-HEMBA1004785
   Gallus gallus mRNA for chromobox protein (CHCB3), complete cds.
   6.6e-19:322:68
   AB005619
F-HEMBA1004797
   Haemonchus contortus GT microsatellite DNA sequence.
   3.0e-08:175:71
   U84474
F-HEMBA1004952
   Mus musculus recombinant quaking gene sequence.
   4.8e-15:398:65
   U44942
F-HEMBA1004971
F-HEMBA1004982
F-HEMBA1005070
   Human mRNA for KIAA0310 gene, complete cds.
   2.5e-65:370:93
   AB002308
F-HEMBA1005084
   Mouse transcriptional control element.
   0.0024:189:63
   M17284
F-HEMBA1005145
   Pseudorabies virus glycoprotein gp50 gene, complete cds.
   0.00022:395:60
   AF092447
F-HEMBA1005230
   Homo sapiens chromosome 19, BAC CIT-B-393i15 (BC301323), complete sequence.
   2.8e-102:302:94
   AC006116
F-HEMBA1005246
   Homo sapiens CAGH44 mRNA, partial cds.
   5.0e-29:429:66
   U80741
F-HEMBA1005267
   Homo sapiens chromosome 3, olfactory receptor pseudogene cluster 1, complete sequence, and myosin light chain kinase (MLCK) pseudogene, partial sequence.
   1.0e-43:320:87
   AF042089
F-HEMBA1005337
   Plasmodium falciparum MAL3P6, complete sequence.
   4.1e-08:84:89
   Z98551
F-HEMBA1005430
F-HEMBA1005449
   T.aestivum mRNA for a proline-rich protein.
   0.00097:385:61
   X52472
F-HEMBA1005489
   Homo sapiens Xq28 genomic DNA in the region of the ALD locus containing the genes for creatine transporter (SLC6A8), CDM, adrenoleukodystrophy (ALD), Na+-isocitrate dehydrogenase gamma subunit (IDH), and translocon-associated protein delta (TRAP) genes, complete cds, plexin related protein (PLEXR) and serine kinase (SK) genes, partial cds, Xq281u1 gene and cytochrome C (CCp) pseudogene.
   7.8e-16:405:62
   U52111
F-HEMBA1005522
   O.cuniculus rACNG mRNA for aorta CNG channel.
   5.9e-47:344:85
   X59668
F-HEMBA1005545
   Human m3 muscarinic acetylcholine receptor (CHRM3) gene, complete cds.
   5.1e-173:810:98
   U29589
F-HEMBA1005698
F-HEMBA1005913
   HS_2249_B1_E01_MF CIT Approved Human Genomic Sperm Library D Homo sapiens genomic clone Plate=2249 Col=1 Row=J, genomic survey sequence.
   0.17:215:61
   AQ072649
F-HEMBA1005929
   Homo sapiens chromosome 19, cosmid R31237, complete sequence.
   7.0e-107:285:93
   AC005581
F-HEMBA1005945
   Oryctolagus cuniculus peroxisomal Ca-dependent solute carrier mRNA, complete cds.
   1.8e-46:670:65
   AF004161
F-HEMBA1006016
   CIT-HSP-2334L16.TF CIT-HSP Homo Sapiens genomic clone 2334L16, genomic survey sequence.
   2.1e-13:246:69
   AQ038406
F-HEMBA1006171
F-HEMBA1006276
   Homo sapiens chromosome 19, CIT-HSP-444n24, complete sequence.
   1.4e-144:416:93
   AC005261
F-HEMBA1006299
F-HEMBA1006311
F-HEMBA1006335
   Human DNA sequence from clone 496H19 on chromosome 6q24 Contains ESTs, complete sequence.
   9.6e-61:370:91
   AL023582
F-HEMBA1006357
   Homo sapiens secretory carrier membrane protein (SCAMP2) mRNA, complete cds.
   2.3e-26:389:67
   AF005038
F-HEMBA1006430
   Caenorhabditis elegans cosmid T12A2.
   4.6e-23:283:72
   U13019
F-HEMBA1006482
   Homo sapiens h-sco1 (SCO1) mRNA, nuclear gene encoding mitochondrial protein, complete cds.
   1.9e-144:575:98
   AF026852
F-HEMBA1006517
F-HEMBA1006544
   Homo sapiens suppressor of white-apricot homolog 2 (SWAP2) gene, exons 12 and 13.
   2.3e-151:732:97
   AF042809
F-HEMBA1006572
   HS_3058_B2_A11_MR CIT Approved Human Genomic Sperm Library D Homo sapiens genomic clone Plate=3058 Col=22 Row=B, genomic survey sequence.
   1.9e-45:245:96
   AQ103440
F-HEMBA1006658
   Homo sapiens mRNA for KIAA0687 protein, partial cds.
   3.6e-127:646:95
   AB014587
F-HEMBA1006707
   Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
   1.7e-118:397:98
   AL021578
F-HEMBA1006724
   H.sapiens CpG island DNA genomic Mse1 fragment, clone 40c2, forward read cpg40c2.ft1k.
   1.4e-53:282:97
   Z55440
F-HEMBA1006749
   Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
   3.9e-116:457:98
   AL021578
F-HEMBA1006770
   Xenopus laevis elav-type ribonucleoprotein (etr-1) mRNA, complete cds.
   1.6e-53:280:81
   U16800
F-HEMBA1006902
   Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
   4.9e-122:462:98
   AL021578
F-HEMBA1006912
F-HEMBA1006916
   Homo sapiens Grb14 mRNA, complete cds.
   1.6e-118:651:92
   L76687
F-HEMBA1006960
   Human DNA sequence *** SEQUENCING IN PROGRESS *** from clone 110F11, WORKING DRAFT SEQUENCE.
   0.20:298:60
   AL033526
F-HEMBA1007013
   Rattus norvegicus repeat element associated with the Rasgrf1 gene.
   8.0e-07:531:59
   AF056927
F-HEMBA1007057
   Human DNA sequence from clone 522J7 on chromosome 22q13.3. Contains part of a 60S Ribosomal protein L5 pseudogene and a Peregrin (BR140) LIKE gene downstream of a putative CpG island. Contains ESTs, STSs and GSSs, complete sequence.
   0.27:277:64
   Z98885
F-HEMBA1007063
F-HEMBA1007226
   Human DNA sequence *** SEQUENCING IN PROGRESS *** from clone 310013, WORKING DRAFT SEQUENCE.
   0.00033:488:63
   AL031658
F-HEMBA1007241
   Caenorhabditis elegans cosmid T15B7.
   0.068:304:59
   AF022985
F-HEMBA1007291
   Homo sapiens chromosome 19, fosmid 37502, complete sequence.
   6.2e-123:587:98
   AC004755
F-HEMBA1007332
   Human HeLa mRNA isolated as a false positive in a two-hybrid-screen.
   1.3e-30:172:97
   U56430
F-HEMBB1000106
   Human DNA sequence from clone 1170D6 on chromosome Xq22.3-23. Contains a pseudogene similar to U-SNRNP_associated Cyclophilin (USA-CYP, EC 5.2.1.8), ESTs, an STS and a GSS, complete sequence.
   0.033:332:61
   AL030995
F-HEMBB1000276
   Dictyostelium discoideum gene encoding a novel glycoprotein.
   0.00070:440:60
   AJ005262
F-HEMBB1000309
   Homo sapiens zinc finger protein (MBLL) mRNA, complete cds.
   7.6e-34:180:100
   AF061261
F-HEMBB1000407
   Human Chromosome 11 pac pDJ393o15, WORKING DRAFT SEQUENCE, 8 unordered pieces. 0.16:228:64
   AC000384
F-HEMBB1000447
   Homo sapiens JWA protein mRNA, complete cds.
   1.4e-158:750:98
   AF070523
F-HEMBB1000542
   Human DNA sequence from PAC 509L4 on chromosome 6q22.1-6q22.33. Contains SSX3 like pseudogene, EST, STS.
   4.3e-141:874:89
   Z99496
F-HEMBB1000567
   Human DNA for insulin-like growth factor II (IGF-2); exon 7 and additional ORF.
   9.7e-122:572:99
   X07868
F-HEMBB1000642
F-HEMBB1000668
   Caenorhabditis elegans cosmid K06A5.
   0.00041:174:64
   AF039038
F-HEMBB1000679
   C.familiaris mRNA for TRAM-protein.
   6.1e-100:756:80
   X63678
F-HEMBB1000881
   Danio rerio mRNA for MINDIN2, complete cds.
   6.2e-40:581:66
   AB006085
F-HEMBB1000905
   Homo sapiens clone RG315H11, WORKING DRAFT SEQUENCE, 5 unordered pieces.
   4.9e-91:209:94
   AC005089
F-HEMBB1001026
   Human p76 mRNA, complete cds.
   1.9e-06:410:61
   U81006
F-HEMBB1001048
   Human Hpast (HPAST) mRNA, complete cds.
   6.8e-55:524:75
   AF001434
F-HEMBB1001200
   Plasmodium falciparum 3D7 chromosome 12 PFYAC293 genomic sequence, WORKING DRAFT SEQUENCE, 9 unordered pieces.
   4.4e-12:794:59
   AC004157
F-HEMBB1001407
   Homo sapiens chromosome 17, clone hRPC.971_F_3, WORKING DRAFT SEQUENCE, 1
   ordered pieces.
   2.7e-43:281:91
   AC004150
F-HEMBB1001530
   HS_2255_B1_F05_MR CIT Approved Human Genomic Sperm Library D Homo sapiens
   genomic clone Plate=2255 Col=9 Row=L, genomic survey sequence.
   2.1e-14:95:97
   AQ131814
F-HEMBB1001547
   S.cerevisiae chromosome VII reading frame ORF YGL236c.
   1.1e-19:550:61
   Z72758
F-HEMBB1001573
   Homo sapiens 12p13.3 PAC RPCI5-951N9 (Roswell Park Cancer Institute Human PAC library) complete sequence.
   2.7e-07:467:60
   AC004672
F-HEMBB1001847
   H.sapiens CpG island DNA genomic Mse1 fragment, clone 13d12, reverse read cpg13d12.rt1c.
   1.1e-14:94:100
   Z64565
F-HEMBB1001959
   Human DNA sequence *** SEQUENCING IN PROGRESS *** from clone 537K23, WORKING DRAFT SEQUENCE.
   1.2e-82:492:90
   AL034405
F-HEMBB1001978
   Homo sapiens chromosome 10 clone CIT987SK-1057L21 map 10q25, complete sequence.
   7.0e-23:239:76
   AC005386
F-HEMBB1002039
   Human DNA sequence from cosmid 315B17, between markers DXS366 and DXS87 on chromosome X contains ESTs.
   3.5e-49:605:71
   Z73967
F-HEMBB1002041
   R.norvegicus mRNA for leucocyte common antigen-related protein (3941 bp).
   3.5e-09:501:60
   X83546
F-HEMBB1002051
   Homo sapiens E74-like factor 5 (ELF5) mRNA, complete cds.
   3.1e-95:454:99
   AF049703
F-HEMBB1002120
F-HEMBB1002162
   Homo sapiens genethonin 1 mRNA, complete cds.
   7.0e-67:328:99
   AF062534
F-HEMBB1002228
   Homo sapiens unknown mRNA, complete cds.
   1.6e-39:208:98
   AF047439
F-HEMBB1002245
   Rattus norvegicus prostaglandin F2a receptor regulatory protein precursor, mRNA, complete cds.
   3.7e-68:424:87
   U26595
F-HEMBB1002302
   RPCI11-18E11.TVB RPCI-11 Homo sapiens genomic clone RPCI-11-18E11, genomic survey sequence.
   2.7e-15:101:98
   B88081
F-HEMBB1002427
   Homo sapiens chromosome 9q34, clone 70C11, complete sequence.
   2.9e-123:249:90
   AC002319
F-HEMBB1002465
   Mouse short chain acyl-CoA dehydrogenase mRNA, complete cds.
   7.9e-18:545:61
   L11163
F-HEMBB1002661
   Drosophila melanogaster; Chromosome 2R; Region 44A1-44A2; P1 clone DS07435, WORKING DRAFT SEQUENCE, 2 unordered pieces.
   1.9e-07:187:67
   AC005445
F-HEMBB1002663
F-HEMBB1002693
   Homo sapiens full length insert cDNA, clone ZD85G07.
   2.1e-20:136:93
   AF086462
F-MAMMA1000046
   CIT-HSP-2166017.TF CIT-HSP Homo sapiens genomic clone 2166O17, genomic survey sequence.
   2.0e-60:345:92
   B92334
F-MAMMA1000102
   Human DNA sequence from cosmid B33F2 on chromosome 22 Contains ESTs.
   3.0e-161:766:98
   Z79996
F-MAMMA1000106
   Rat gene for alpha 1B adrenergic receptor, promoter region and partial cds.
   0.0025:247:64
   D32045
F-MAMMA1000118
   Canis familiaris beta1 adrenergic receptor (dogbetal) gene, complete cds.
   6.1e-06:545:60
   U73207
F-MAMMA1000141
   Homo sapiens 12q24.2 PAC RPCI1-128M12 (Roswell Park Cancer Institute Human PAC library) complete sequence.
   1.5e-10:151:78
   AC004024
F-MAMMA1000204
   Homo Sapiens mRNA for LGMD2B protein.
   2. le-166:781:98
   AJ007670
F-MAMMA1000226
   *** SEQUENCING IN PROGRESS *** Homo sapiens chromosome 4, BAC clone B331O8; HTGS phase 1, WORKING DRAFT SEQUENCE, 10 unordered pieces.
   2.9e-35:188:100
   AC004064
F-MAMMA1000403
   Human vascular addressin MAdCAM-1 mRNA, complete cds.
   0.00043:538:59
   U82483
F-MAMMA1000449
   Human DNA sequence *** SEQUENCING IN PROGRESS *** from clone 317C6, WORKING DRAFT SEQUENCE.
   0.090:514:60
   Z97651
F-MAMMA1000457
   H.sapiens mRNA for NADH-cytochrome b5 reductase.
   5.5e-36:469:68
   Y09501
F-MAMMA1000473
   Caenorhabditis elegans cosmid B0491, complete sequence.
   0.0052:187:64
   Z49907
F-MAMMA1000496
   Homo sapiens PAC clone DJ130H16 from 22q12.1-qter, complete sequence.
   1.2e-81:318:92
   AC004997
F-MAMMA1000528
   P.falciparum complete gene map of plastid-like DNA (IR-B).
   0.016:343:58
   X95276
F-MAMMA1000591
   Mus musculus UDP-GalNAc:polypeptide N-acetylgalactosaminyltransferase-T3 mRNA, complete cds.
   1.2e-24:493:63
   U70538
F-MAMMA1000614
   *** SEQUENCING IN PROGRESS *** Homo sapiens chromosome 4, BAC clone C0190L06; HTGS phase 1, WORKING DRAFT SEQUENCE, 21 unordered pieces.
   7.5e-13:615:60
   AC004670
F-MAMMA1000652
   Homo sapiens Chromosome 16 BAC clone CIT987SK-A-116A10, complete sequence.
   1.6e-59:451:82
   AC004638
F-MAMMA1000681
   Homo sapiens mRNA for putative G-protein coupled receptor, EDG6.
   1.2e-32:636:65
   AJ000479
F-MAMMA1000706
   *** SEQUENCING IN PROGRESS *** Homo sapiens chromosome 4, BAC clone C0110D16; HTGS phase 1, WORKING DRAFT SEQUENCE, 7 unordered pieces.
   6.8e-06:428:62
   AC004578
F-MAMMA1000788
   HS_3080_A2_B03_MR CIT Approved Human Genomic Sperm Library D Homo sapiens genomic clone Plate=3080 Col=6 Row=C, genomic survey sequence.
   4.9e-35:204:94
   AQ128409
F-MAMMA1000810
   Homo sapiens DNA sequence from PAC 510L9 on chromosome 6p24.1-p25.3.
   5.8e-06:246:65
   AL022098
F-MAMMA1000814
   Anadara trapezia (alpha 3.15L) hemoglobin alpha-chain (HBA) gene, exons 2 and 3, intron 2, including hypervariable microsatellite polymorphic repeat regions.
   1.0e-12:176:75
   L25098
F-MAMMA1000881
   Rattus norvegicus serum and glucocorticoid-regulated kinase (sgk) mRNA, complete cds.
   2.8e-07:283:63
   L01624
F-MAMMA1000986
   Homo sapiens chromosome 16 BAC clone CIT987SK-334D11 complete sequence.
   1.8e-166:306:99
   AF001550
F-MAMMA1000994
   Mycobacterium tuberculosis H37Rv complete genome; segment 48/162.
   0.75:260:61
   AL021897
F-MAMMA1001043
   H.sapiens mRNA for latent transforming growth factor-beta binding protein (LTBP-2).
   0.038:376:60
   Z37976
F-MAMMA1001066
   Homo sapiens DNA from chromosome 19-cosmid f24590 containing CAPNS and POL2RI, genomic sequence.
   4.4e-15:162:72
   AD001527
F-MAMMA1001094
   Homo sapiens clone 243 unknown mRNA, complete sequence.
   6.2e-181:844:99
   AF091094
F-MAMMA1001141
   Cams familiaris beta1 adrenergic receptor (dogbeta1) gene, complete cds.
   1.3e-10:602:59
   U73207
F-MAMMA1001150
   M.musculus (Balb/c) mRNA for serine/threonine protein kinase.
   7.7e-57:447:67
   Z34524
F-MAMMA1001237
   Rattus norvegicus monocarboxylate transporter MCT3 mRNA, complete cds.
   1.5e-08:306:65
   AF059258
F-MAMMA1001284
   HS_3076_A1_F08_MF CIT Approved Human Genomic Sperm Library D Homo sapiens genomic clone Plate=3076 Col=15 Row=K, genomic survey sequence.
   5.2e-53:307:93
   AQ120674
F-MAMMA1001310
   1(2)09851 Drosophila melanogaster P lethal line Drosophila melanogaster genomic Sequence recovered from 3' end of P element, genomic survey sequence.
   0.00072:209:66
   AQ025672
F-MAMMA1001344
   Fugu rubripes neural cell adhesion molecule L1 homolog (L1-CAM) gene, complete cds; putative protein 1 (PUT1) gene, partial cds; mitosis-specific chromosome segregation protein SMC1 homolog (SMC1) gene, complete cds; and calcium channel alpha-1 subunit homolog (CCA1) and putative protein 2 (PUT2) genes, partial cds, complete sequence.
   5.2e-05:164:67
   AF026198
F-MAMMA1001418
   Human Na+/nucleoside cotransporter (hCNT1a) mRNA, complete cds.
   6.0e-35:622:63
   U62966
F-MAMMA1001532
   Homo sapiens PAC clone DJ0728D04, complete sequence.
   5.2e-46:538:74
   AC004865
F-MAMMA1001609
   Homo sapiens chromosome 10 clone CIT-HSP-1240G16 map 10q25.1, complete sequence. 0.00031:592:57
   AC005886
F-MAMMA1001615
   H.sapiens CpG island DNA genomic Mse1 fragment, clone 71h9, reverse read cpg71h9.rt1a.
   1.2e-25:146:99
   Z62710
F-MAMMA1001623
   Homo sapiens 12q24.2 BAC RPCI11-407A16 (Roswell Park Cancer Institute Human BAC Library) complete sequence.
   3.9e-69:471:85
   AC006065
F-MAMMA1001634
   Human DNA sequence from PAC 93C23 on chromosome X. Contains steroid 5-alpha-reductase pseudogene, ESTs and STS.
   2.2e-22:228:79
   AL008713
F-MAMMA1001893
   HS_3067_B2_H09_MF CIT Approved Human Genomic Sperm Library D Homo sapiens
   genomic clone Plate=3067 Col=18 Row=P, genomic survey sequence.
   2.5e-29:188:93
   AQ138065
F-MAMMA1001901
   Human DNA sequence from clone 354J5 on chromosome 6q21-22. Contains pseudogene similar
   to zinc finger protein (ZPR1), EST, STS, GSS, complete sequence.
   2.0e-23:287:71
   Z95118
F-MAMMA1001957
   Drosophila melanogaster, chromosome 2L, region 21C5-21D1, P1 clone DS07610, complete sequence.
   1.5e-14:192:66
   AC004573
F-MAMMA1001978
   Human immunoglobulin S(u) like sequence.
   0.60:150:66
   X15517
F-MAMMA1002070
   Human PAC clone DJ515N1 from 22q11.2-q22, complete sequence.
   3.9e-116:250:93
   AC002073
F-MAMMA1002080
   Mus musculus chromosome 11, clone mCIT.268_P_23, complete sequence.
   1.1e-59:493:78
   AC004807
F-MAMMA1002087
   HS-1047-B2-A09-MR.abi CIT Human Genomic Sperm Library C Homo sapiens genomic clone Plate=CT 830 Col=18 Row=B, genomic survey sequence.
   2.1e-31:174:98
   B38457
F-MAMMA1002091
   Homo sapiens CD39L2 (CD39L2) mRNA, complete cds.
   1.6e-156:743:98
   AF039916
F-MAMMA1002095
   Rat alternatively spliced mRNA.
   4.9e-126:691:91
   M93017
F-MAMMA1002128
   Mus musculus C2C12 unknown mRNA, partial cds.
   5.0e-41:353:77
   U31629
F-MAMMA1002142
F-MAMMA1002165
   Homo sapiens connective tissue growth factor related protein WISP-2 (WISP2) mRNA, complete cds.
   1.2e-34:219:90
   AF100780
F-MAMMA1002205
   Homo Sapiens Chromosome X clone bWXD691, complete sequence.
   8.1e-33:535:67
   AC004386
F-MAMMA1002224
   Human DNA sequence *** SEQUENCING IN PROGRESS *** from clone 229A8, WORKING DRAFT SEQUENCE.
   1.2e-31:274:82
   Z86090
F-MAMMA1002234
   Canine mRNA for 68kDA subunit of signal recognition particle (SRP68).
   9.8e-145:736:91
   X53744
F-MAMMA1002586
   Drosophila melanogaster cosmid clone 86E4.
   0.0071:306:58
   AL021086
F-MAMMA1002633
   Human DNA sequence *** SEQUENCING IN PROGRESS *** from clone 850H21, WORKING DRAFT SEQUENCE.
   3.9e-33:297:79
   AL031680
F-MAMMA1003126
   Human Hpast (HPAST) mRNA, complete cds.
   1.7e-82:801:74
   AF001434
F-NT2RM1000407
   Cloning vector pUC-GM-INT, complete sequence.
   9.4e-141:673:98
   AF025392
F-NT2RM1000462
   , complete sequence.
   1.5e-86:232:82
   AC005300
F-NT2RM1000542
   Mouse beta-galactosidase (BGAL) gene, complete cds.
   4.4e-17:468:62
   M57734
F-NT2RM1000580
   Caenorhabditis elegans cosmid F09E5.
   1.6e-08:352:61
   U37429
F-NT2RM1000789
   Homo sapiens mRNA for hTCF-4.
   1.1e-94:299:92
   Y11306
F-NT2RM1000855
   Canis familiaris sec61 homologue mRNA, complete cds.
   6.6e-110:671:87
   M96629
F-NT2RM1000858
   tricarboxylate carrier [rats, liver, mRNA Partial, 2986 nt].
   2.0e-65:716:70
   S70011
F-NT2RM1000899
   S.pombe chromosome I cosmid c8C9.
   0.0010:300:59
   Z99168
F-NT2RM2000241
   Homo sapiens chromosome 12p13.3 clone RPCI11-350L7, WORKING DRAFT SEQUENCE, 72 unordered pieces.
   0.99:201:65
   AC005844
F-NT2RM2000306
   Homo sapiens DNA sequence from PAC 257I20 on chromosome 22q13.1-13.2. Contains cytochrome P450 pseudogenes CYP2D7P, CYP2D8P, CYP2D6(D),TCF20, NADH ubiquinone oxidoreductase B14 subunit, ESTs, CA repeat, STS, GSS.
   1.1e-142:595:97
   AL021878
F-NT2RM2000410
   S.gregaria Abd-B gene.
   0.076:172:66
   X69161
F-NT2RM2000423
   Arthrobacter sp. beta-galactosidase gene, complete cds.
   4.2e-06:606:57
   U78028
F-NT2RM2000497
   Homo sapiens chromosome 17, clone hRPK.215_P_18, complete sequence.
   1.2e-55:285:81
   AC005969
F-NT2RM2000514
F-NT2RM2000565
   Caenorhabditis elegans cosmid F28C5, complete sequence.
   4.2e-18:539:62
   Z68315
F-NT2RM2000582
   P.zebra microsatellite locus DNA, 429bp.
   0.00015:160:69
   X99784
F-NT2RM2000589
   Bos taurus myosin X, complete cds.
   3.4e-139:817:88
   U55042
F-NT2RM2000622
   H.sapiens MFH-1 gene.
   0.0010:466:57
   Y08223
F-NT2RM2000632
   Homo sapiens mRNA for TBP-associated factor 170 (TAFII170).
   0.0052:331:59
   AJ001017
F-NT2RM2000773
   Oryctolagus cuniculus serum amyloid A-activating factor SAF-8 mRNA, partial cds.
   2.9e-91:496:93
   AF076786
F-NT2RM2001126
   Homo sapiens multi PDZ domain protein MUPP1 (MUPP1) mRNA, complete cds.
   1.6e-161:663:99
   AF093419
F-NT2RM2001558
   Homo sapiens protein kinase A binding protein AKAP110 mRNA, complete cds.
   1.2e-164:770:98
   AF093408
F-NT2RM2001626
   F.rubripes GSS sequence, clone 060E22bA4, genomic survey sequence.
   4.5e-46:606:68
   Z88651
F-NT2RM2001643
   HS-1053-B2-E09-MR.abi CIT Human Genomic Sperm Library C Homo sapiens genomic clone Plate=CT775 Col=18 Row=J, genomic survey sequence.
   2.5e-06:181:66
   B41504
F-NT2RM2001738
   S.capreolus ard2 gene and orf2, orf4 and orf5.
   0.41:273:63
   Y11036
F-NT2RM2001767
   Human DNA sequence *** SEQUENCING IN PROGRESS *** from clone 50O24, WORKING DRAFT SEQUENCE.
   8.0e-18:130:92
   AL034380
F-NT2RM2001792
   Homo sapiens mRNA for serum lectin P35, complete cds.
   2.5e-12:244:67
   D49353
F-NT2RM2001818
F-NT2RM2001902
   Drosophila melanogaster mRNA for p21 activated kinase related protein.
   7.2e-74:683:75
   AJ011578
F-NT2RM2001939
   Human G protein-coupled receptor GPR-NGA gene, complete cds.
   1.4e-140:702:96
   U55312
F-NT2RM2001941
   Human gene for muscarinic acetylcholine receptor HM1.
   6.3e-20:488:62
   X15263
F-NT2RM4000100
   Homo sapiens PAC clone DJ044L15 from Xq23, complete sequence.
   7.7e-25:162:74
   AC004827
F-NT2RM4000115
F-NT2RM4000198
F-NT2RM4000284
   Human IgG Fc receptor hFcRn mRNA, complete cds.
   7.3e-37:194:98
   U12255
F-NT2RM4000295
   Streptomyces chrysomallus actinomycin synthetase II (acmB) gene, complete cds.
   1.6e-05:642:59
   AF047717
F-NT2RM4000326
   Homo sapiens complete genomic sequence between D16S3070 and D16S3275, containing Familial Mediterranean Fever gene disease.
   1.0e-127:340:92
   AJ003147
F-NT2RM4000417
   Oncorhynchus kisutch microsatellite OKi20 DNA.
   0.44:144:66
   AF055444
F-NT2RM4000444
   S.salar mRNA for transport-associated protein Tap2A.
   1.7e-27:577:62
   Z83328
F-NT2RM4000587
   Homo sapines chromosome 19, cosmid R28058, complete sequence.
   7.7e-16:388:64
   AC005615
F-NT2RM4000593
F-NT2RM4000648
   M.musculus mRNA for K-glypican.
   1.4e-50:610:70
   X83577
F-NT2RM4000761
   Human mitochondrial DNA, fragment M1, encoding transfer RNAs, cytochrome oxidase I, and 2 URFs.
   4.8e-167:787:98
   M10546
F-NT2RM4000965
   S.scrofa mRNA for calcium release channel (CRC).
   0.044:356:60
   X62880
F-NT2RM4000997
F-NT2RM4001321
   HS-1053-B2-E09-MR.abi CIT Human Genomic Sperm Library C Homo sapiens genomic clone Plate=CT 775 Col=18 Row=J, genomic survey sequence.
   1.3e-06:181:67
   B41504
F-NT2RM4001325
   Homo sapiens mRNA for chondroitin 6-sulfotransferase, complete cds.
   6.6e-12:384:64
   AB012192
F-NT2RM4001377
   Homo sapiens mRNA for KIAA0638 protein, partial cds.
   9.7e-155:719:99
   AB014538
F-NT2RM4001735
   Human DNA sequence *** SEQUENCING IN PROGRESS *** from clone 483K16, WORKING DRAFT SEQUENCE.
   1.3e-162:679:96
   AL034374
F-NT2RM4001768
   Human HepG2 partial cDNA, clone hrnd3a07m5.
   2.7e-52:271:98
   D17020
F-NT2RM4001843
F-NT2RM4002352
   Homo sapiens hLRp105 mRNA for LDL receptor related protein 105, complete cds.
   1.4e-155:761:97
   AB009462
F-NT2RP1000002
   Mouse cAMP-dependent protein kinase beta subunit gene, exon 1.
   1.7e-06:252:65
   M21096
F-NT2RP1000050
   Human HepG2 partial cDNA, clone hmd3g02m5.
   7.1e-18:115:97
   D17047
F-NT2RP1000181
   Homo sapiens Chromosome 11q12.2 PAC clone pDJ519o13 containing human gene for ferritin heavy chain (FTH), complete sequence.
   4.2e-139:427:98
   AC004228
F-NT2RP1000239
   Homo sapiens clone DT1P1E11 mRNA, CAG repeat region.
   1.4e-90:524:91
   U92989
F-NT2RP1000261
   Homo sapiens hPMS1 gene, promoter region and exon 1.
   2.5e-14:132:85
   AB006462
F-NT2RP1000271
   Homo sapiens DNA-binding protein mRNA, complete cds.
   4.3e-139:678:97
   AF038951
F-NT2RP1000300
   Homo sapiens, complete sequence.
   0.012:146:69
   AC005854
F-NT2RP1000325
   H.sapiens gene for phosphate carrier.
   4.2e-110:438:98
   X77337
F-NT2RP1000448
   Streptomyces coelicolor cosmid 1A6.
   0.79:209:61
   AL023496
F-NT2RP1000465
   Mus musculus nuclear protein NIP45 mRNA, complete cds.
   2.2e-29:489:68
   U76759
F-NT2RP1000468
   Human DNA sequence *** SEQUENCING IN PROGRESS *** from clone 537K23, WORKING DRAFT SEQUENCE.
   1.6e-49:306:91
   AL034405
F-NT2RP1000551
   Homo sapiens putative interferon-related protein (SM15) mRNA, partial cds.
   7.5e-139:742:93
   U09585
F-NT2RP1000579
   Human succinate dehydrogenase flavoprotein subunit (SDH) mRNA, complete cds.
   3.6e-140:798:91
   L21936
F-NT2RP1000613
   Sequence 1 from patent US 5589579.
   8.1e-10:468:58
   I32995
F-NT2RP1000679
   Homo sapiens chromosome 17, clone hRPC.4_G_17, complete sequence.
   1.3e-112:448:89
   AC003688
F-NT2RP1000740
   H.sapiens CpG island DNA genomic Mse1 fragment, clone 34a2, reverse read cpg34a2.rt1a.
   9.3e-14:211:73
   Z60772
F-NT2RP1000903
   HS_2256_B1_E10_MF CIT Approved Human Genomic Sperm Library D Homo sapiens genomic clone Plate=2256 Col=19 Row=J, genomic survey sequence.
   9.0e-21:197:84
   AQ084622
F-NT2RP1000981
   F-NT2RP1001004
   Danio rerio mRNA for MINDIN2, cemplete cds.
   4.1e-22:472:63
   AB006085
F-NT2RP1001020
   Mus musculus clone OST66, genomic survey sequence.
   1.5e-47:352:81
   AF046696
F-NT2RP1001031
   CIT-HSP-2330P23.TR CIT-HSP Homo sapiens genomic clone 2330P23, genomic survey sequence.
   8.0e-26:145:99
   AQ035969
F-NT2RP1001563
   Homo sapiens clone DJ1125K23, WORKING DRAFT SEQUENCE, 21 unordered pieces. 0.096:405:59
   AC004971
F-NT2RP2000092
   Human zinc finger protein ZNF136.
   1.8e-54:652:70
   U09367
F-NT2RP2000178
   Streptomyces coelicolor cosmid 3F9.
   0.92:217:64
   AL023862
F-NT2RP2000240
   Homo sapiens chromosome 16 BAC clone CIT987SK-334D11 complete sequence.
   2.9e-96:534:90
   AF001550
F-NT2RP2000394
   Gallus gallus p52 pro-apototic protein mRNA, complete cds.
   2.9e-19:380:65
   AF029071
F-NT2RP2000447
   Homo sapiens clone DJ1129D05, complete sequence.
   1.3e-109:289:98
   AC005630
F-NT2RP2000479
   Human DNA sequence from PAC 130G2 on chromosome 6p22.2-22.3. Contains ribosomal protein L29 pseudogene, ESTs and STSs.
   0.0039:219:63
   AL008627
F-NT2RP2000514
   Homo sapiens roundabout 2 (robo2) mRNA, partial cds.
   3.7e-89:461:95
   AF040991
F-NT2RP2000533
   Mus musculus cornichon mRNA, complete cds.
   1.4e-113:677:89
   AF022811
F-NT2RP2000610
   Human DNA sequence *** SEQUENCING IN PROGRESS *** from clone 537K23, WORKING DRAFT SEQUENCE.
   4.3e-25:177:89
   AL034405
F-NT2RP2000616
   RPCI11-75J11.TK MCI11 Homo sapiens genomic clone R-75J11, genomic survey sequence.
   8.4e-34:135:91
   AQ268877
F-NT2RP2000649
   Homo sapiens CAAX prenyl protease (STE24) mRNA, complete cds.
   1.2e-165:802:97
   AF064867
F-NT2RP2000663
   Human DNA sequence from cosmid U61B11, between markers DXS366 and DXS87 on chromosome X contains ESTs.
   1.6e-106:365:97
   Z73913
F-NT2RP2000694
   Homo sapiens 5T4 oncofetal trophoblast glycoprotein gene.
   4.2e-112:561:96
   AJ012159
F-NT2RP2000712
   HS_3071_A2_D05_MR CIT Approved Human Genomic Sperm Library D Homo sapiens genomic clone Plate=3071 Col=10 Row=G, genomic survey sequence.
   7.6e-78:389:97
   AQ166085
F-NT2RP2000739
   Human mRNA for KIAA0326 gene, partial cds.
   6.4e-24:574:62
   AB002324
F-NT2RP2000818
   Drosophila melanogaster, chromosome 2R, region 38A5-38B4, BAC clone BACR48M05, complete sequence.
   0.00047:304:61
   AC005719
F-NT2RP2000903
   Homo sapiens 5T4 oncofetal trophoblast glycoprotein gene.
   2.6e-110:541:97
   AJ012159
F-NT2RP2001200
   Homo sapiens mRNA for KIAA0676 protein, partial cds.
   3.3e-1 10:540:96
   AB014576
F-NT2RP2001223
   HS-1054-B2-C02-MR.abi CIT Human Genomic Sperm Library C Homo sapiens genomic clone Plate=CT 776 Col=4 Row=F, genomic survey sequence.
   7.2e-10:128:77
   B41982
F-NT2RP2001276
   Mouse regulatory protein (npdc-1) mRNA, complete cds.
   1.2e-38:296:81
   L03814
F-NT2RP2001388
   RPCI11-30G23.TV RPCI-11 Homo sapiens genomic clone RPCI-11-30G23, genomic survey sequence.
   0.32:53:94
   B87787
F-NT2RP2001469
   M.musculus tex292 mRNA (5'region).
   4.2e-10:120:83
   X80434
F-NT2RP2001480
   Homo sapiens thrombospondin 3 (THBS3) gene, complete cds.
   9.0e-140:686:96
   L38969
F-NT2RP2001495
   Human transporter protein (g17) mRNA, complete cds.
   1.9e-35:581:64
   U49082
F-NT2RP2001514
   Drosophila melanogaster DNA sequence (P1 DS06882 (D310)), complete sequence.
   3.7e-22:475:62
   AC005115
F-NT2RP2001529
   Homo sapiens mRNA for ZIP-kinase, complete cds.
   4.6e-152:757:96
   AB007144
F-NT2RP2001538
   Sequence 11 from patent US 5624818.
   1.4e-88:528:88
   I41141
F-NT2RP2001562
   Homo sapiens GLE1 (GLE1) mRNA, complete cds.
   2.3e-117:572:97
   AF058922
F-NT2RP2001662
   Human DNA sequence *** SEQUENCING IN PROGRESS *** from clone 460J8, WORKING DRAFT SEQUENCE.
   6.1e-107:365:91
   AL031662
F-NT2RP2001755
   Sequence 9 from patent US 5750502.
   1.5e-53:518:75
   AR007441
F-NT2RP2001769
   A.sativa Aspk11 mRNA.
   4.7e-17:537:60
   X79992
F-NT2RP2001817
   Candida albicans SIR2 (SIR2) gene, complete cds.
   4.6e-10:285:61
   AF045774
F-NT2RP2001878
   Mus musculus repeat element upstream of the Rasgrf1/Cdc25Mm gene.
   5.0e-06:554:60
   AF021791
F-NT2RP2001903
   M.musculus mRNA for m-calpain.
   3.1e-06:337:60
   Y10139
F-NT2RP2001915
   Homo sapiens chromosome 17, clone hRPK.63_A_1, complete sequence.
   6.8e-28:488:65
   AC005670
F-NT2RP2001921
   Homo sapiens clone NH0332L11, complete sequence.
   1.1e-77:148:99
   AC005538
F-NT2RP2001948
   Sequence 2 from patent US 5541311.
   0.59:284:57
   I24091
F-NT2RP2001956
   Feline c-sis proto-oncogene, segment 4.
   0.99:101:69
   M25356
F-NT2RP2002015
   HS-1053-B2-E09-MR.abi CIT Human Genomic Sperm Library C Homo sapiens genomic clone Plate=CT 775 Col=18 Row=J, genomic survey sequence.
   3.0e-06:181:65
   B41504
F-NT2RP2002063
   Homo sapiens chromosome 4 clone B207D4 map 4q25, complete sequence.
   1.3e-108:418:94
   AC004050
F-NT2RP2002188
   Rattus norvegicus neuroligin 3 mRNA, complete cds.
   1.0e-125:700:90
   U41663
F-NT2RP2002232
F-NT2RP2002304
   Human FMR1 gene, 5' end.
   0.12:93:67
   L19476
F-NT2RP2002409
   Myxococcus xanthus response regulator FrzZ (frzZ) gene, partial cds; alanine dehydrogenase (aldA), putative ECF sigma factor RpoE1 (rpoE1), and response regulator homolog (frzS) genes, complete cds; and unknown genes.
   9.0e-10:553:59
   AF049107
F-NT2RP2002510
   Mus musculus (129SV) DNA, unmapped BAC 10817, complete sequence.
   4.2e-27:573:62
   AC004093
F-NT2RP2002527
   Homo sapiens Chromosome 11q12.2 PAC clone pDJ519o13 containing human gene for ferritin heavy chain (FTH), complete sequence.
   3.2e-110:439:99
   AC004228
F-NT2RP2002533
   Homo sapiens putative tumor suppressor gene 26 protein alpha 2 delta calcium channel subunit mRNA, complete cds.
   6.4e-141:726:95
   AF040709
F-NT2RP2002564
   Homo sapiens PAC clone DJ0979P20 from 7q33-q35, complete sequence.
   2.6e-112:403:98
   AC004941
F-NT2RP2002674
   HS_3122_B2_A02_MR CIT Approved Human Genomic Sperm Library D Homo sapiens genomic clone Plate=3122 Col=4 Row=B, genomic survey sequence.
   4.8e-13:86:100
   AQ182907
F-NT2RP2002721
F-NT2RP2002824
   Arabidopsis thaliana BAC T19D16 genomic sequence.
   1.3e-12:135:69
   U95973
F-NT2RP2002942
   Homo sapiens mRNA for KIAA0806 protein, complete cds.
   6.1e-145:758:94
   AB018349
F-NT2RP2002974
   Mus musculus mRNA for Six5, partial cds.
   8.0e-84:588:82
   D83146
F-NT2RP2002976
   H.sapiens gene for phospholipase C beta 3, exon 14.
   0.93:210:61
   Z37557
F-NT2RP2003042
   G.gallus mRNA for lecithin-cholesterol acyltransferase.
   9.1e-26:462:65
   X91011
F-NT2RP2003138
   Human DNA sequence *** SEQUENCING IN PROGRESS *** from clone 460J8, WORKING DRAFT SEQUENCE.
   3.9e-142:702:96
   AL031662
F-NT2RP2003179
   Homo sapiens mRNA for KIAA0537 protein, complete cds.
   3.3e-42:587:70
   AB011109
F-NT2RP2003210
   Mus musculus fatty acid transport protein 4 mRNA, partial cds.
   2.6e-112:726:85
   AF072759
F-NT2RP2003302
   Human zinc finger protein ZNF136.
   5.5e-63:691:69
   U09367
F-NT2RP2003369
   Homo sapiens chromosome 7q22 sequence, complete sequence.
   2.0e-49:249:95
   AF053356
F-NT2RP2003383
   Homo sapiens chromosome 1 atrophin-1 related protein (DRPLA) mRNA, complete cds.
   1.5e-159:817:95
   AF016005
F-NT2RP2003390
   Homo sapiens SEC63 (SEC63) mRNA, complete cds.
   7.0e-115:554:98
   AF100141
F-NT2RP2003469
   Genomic sequence from Human 9q34, complete sequence.
   5.6e-38:210:97
   AC001644
F-NT2RP2003545
   Homo sapiens STE20-like kinase 3 (mst-3) mRNA, complete cds.
   2.2e-48:579:71
   AF024636
F-NT2RP2003593
   Human DNA sequence *** SEQUENCING IN PROGRESS *** from clone 971N18, WORKING DRAFT SEQUENCE.
   1.8e-90:326:99
   AL021396
F-NT2RP2003599
F-NT2RP2003655
   M.musculus tex261 mRNA.
   5.3e-77:513:85
   X81058
F-NT2RP2003664
   Homo sapiens mRNA for leptin receptor gene-related protein.
   1.7e-132:630:98
   Y12670
F-NT2RP2003931
   Homo sapiens chromosome 19, overlapping cosmids R27918 and R33775, complete sequence.
   1.3e-114:411:97
   AC004447
F-NT2RP2003940
   Human ZNF43 mRNA.
   1.4e-97:693:82
   X59244
F-NT2RP2003950
   Sequence 1 from patent US 5648238.
   6.9e-13:143:79
   I55887
F-NT2RP2004069
F-NT2RP2004108
   Human zinc finger protein ZNF136.
   1.5e-67:548:78
   U09367
F-NT2RP2004141
   Rattus norvegicus leukocyte common antigen receptor (LAR) gene, trans-spliced alternative untranslated exon.
   8.0e-10:487:62
   U87960
F-NT2RP2004179
   Homo sapiens chromosome 11 from 11p15.5 region, complete sequence.
   0.56:600:57
   AF015416
F-NT2RP2004205
   Homo sapiens chromosome 16, cosmid clone RT81 (LANL), complete sequence.
   0.32:431:55
   AC005356
F-NT2RP2004447
   Homo sapiens Chromosome 11q13 BAC Clone 18h3, WORKING DRAFT SEQUENCE, 7 ordered pieces.
   2.0e-23:252:79
   AC000353
F-NT2RP2004495
   Human transporter protein (g17) mRNA, complete cds.
   3.6e-25:497:61
   U49082
F-NT2RP2004524
   Genomic sequence from Human 9q34, complete sequence.
   5.9e-60:203:98
   AC001644
F-NT2RP2004556
   HS_3022_A1_A11_T7 CIT Approved Human Genomic Sperm Library D Homo sapiens genomic clone Plate=3022 Col=21 Row=A, genomic survey sequence.
   1.3e-51:419:79
   AQ119143
F-NT2RP2004606
   cDNA encoding NIC(Natural Inhibitor of Collagenase).
   1.2e-113:617:92
   E00985
F-NT2RP2004648
   Felis catus lysosomal beta-galactosiclase (Bgal) mRNA, complete cds.
   1.5e-15:403:64
   AF006749
F-NT2RP2004670
   Rattus norvegicus vesicla-associate calmodulin-binding protein mRNA, complete cds.
   1.1e-73:493:85
   L22557
F-NT2RP2004794
   Mus musculus mRNA for B-IND1 protein.
   5.6e-12:109:86
   Z97207
F-NT2RP2004837
   Homo sapiens clone NH0001P09, WORKING DRAFT SEQUENCE, 1 unordered pieces.
   2.8e-39:352:78
   AC006030
F-NT2RP2004847
   CIT-HSP-2357D24.TR CIT-HSP Homo sapiens genomic clone 2357D24, genomic survey sequence.
   2.5e-35:196:96
   AQ074738
F-NT2RP2005027
   Human glucose transporter-like protein-III (GLUT3), complete cds.
   2.2e-145:713:96
   M20681
F-NT2RP2005069
   Rat vacuolar protein sorting homolog r-vps33b mRNA, complete cds.
   9.4e-51:200:90
   U35245
F-NT2RP2005163
   Mouse DNA fragment that hybridizes to HSV-1 SmaI A fragment.
   1.4e-08:231:67
   M11041
F-NT2RP2005181
   Mus musculus cationic amino acid trmsporter (CAT3) mRNA, complete cds.
   1.6e-96:575:85
   U70859
F-NT2RP2005247
   Mus musculus ret finger protein mRNA, complete cds.
   1.8e-13:310:66
   L46855
F-NT2RP2005378
   RPCI11-21D23.TP RPCI-11 Homo sapiens genomic clone RPCI-11-21D23, genomic survey sequence.
   3.0e-12:131:80
   B85846
F-NT2RP2005391
   S.muris mRNA for microneme antigen.
   2.5e-10:345:61
   Z26947
F-NT2RP2005425
   Homo sapiens mRNA for KIAA0803 protein, partial cds.
   1.0e-116:566:97
   AB018346
F-NT2RP2005463
F-NT2RP2005514
F-NT2RP2005535
   Homo sapiens DNA-binding protein mRNA, complete cds.
   2.3e-125:726:90
   AF038951
F-NT2RP2005541
   CIT-HSP-2386E2.TF.1 CIT-HSP Homo sapiens genomic clone 2386E2, genomic survey sequence.
   6.2e-20:152:88
   AQ240341
F-NT2RP2005597
   D.melanogaster mRNA for rotated abdomen protein.
   0.088:270:57
   X95956
F-NT2RP2005632
   Gallus gallus chicken brain factor-2 (CBF-2) mRNA, complete cds.
   2.0e-07:207:67
   U47276
F-NT2RP2005666
   Homo sapiens chromosome 11 clone CIT-HSP-1337H24, WORKING DRAFT SEQUENCE, 9 unordered pieces.
   1.0:328:57
   AC005849
F-NT2RP2005774
   Human zinc finger protein ZNF136.
   4.0e-44:451:74
   U09367
F-NT2RP2005878
   Mus musculus putative steroid dehydrogenase (KIK-I) mRNA, complete cds.
   5.1e-16:382:63
   AF064635
F-NT2RP2005883
   Human DNA sequence from clone 248E1 on chromosome 6q23.1-23.3 Contains DOPAMINE-BETA-MONOOXYGENASE PRECURSOR, EF-1-ALPHA-2 pseudogene EST GSS and CA repeat, complete sequence.
   1.5e-30:191:95
   AL023578
F-NT2RP2005887
   Homo sapiens clone NH0001P09, WORKING DRAFT SEQUENCE, 1 unordered pieces.
   1.8e-50:394:79
   AC006030
F-NT2RP2005941
   Human DNA sequence from cosmid CFAT5, chromosome region 11p13 contains PAX6 exons 1-4, EST and CpG Islands.
   9.5e-93:468:96
   Z95332
F-NT2RP2005994
   Homo sapiens chromosome 4 clone B207D4 map 4q25, complete sequence.
   1.6e-139:692:96
   AC004050
F-NT2RP2006004
   CIT-HSP-2366J21.TF CIT-HSP Homo sapiens genomic clone 2366J21, genomic survey sequence.
   6.6e-39:206:98
   AQ080257
F-NT2RP2006042
   Human mRNA for KIAA0144 gene, complete cds.
   1.7e-10:220:69
   D63478
F-NT2RP2006092
   Homo sapiens chromosome 5, BAC clone 319C17 (LBNL H159), complete sequence.
   3.6e-121:562:82
   AC005214
F-NT2RP2006099
   Human Chromosome 11 pac pDJ392a17, complete sequence.
   8.7e-76:383:92
   AC000385
F-NT2RP2006134
   Homo sapiens Chromosome 22q11.2 Cosmid Clone 91c In DGCR Region, complete sequence. 0.055:125:71
   AC000091
F-NT2RP2006269
   D.melanogaster mRNA for rotated abdomen protein.
   5.4e-05:357:58
   X95956
F-NT2RP2006512
   Sequence 1 from Patent EP 0285405.
   3.7e-102:659:85
   I05465
F-NT2RP3000011
   RPCI11-43E12.TJ RPCI11 Homo sapiens genomic clone R-43E12, genomic survey sequence.
   1.8e-10:113:84
   AQ195722
F-NT2RP3000022
   Human DNA sequence from clone 341E18 on chromosome 6p11.2-12.3. Contains a Serine/Threonine Protein Kinase gene (presumptive isolog of a Rat gene) and a novel alternatively spliced gene. Contains a putative CpG island, ESTs and GSSs, complete sequence.
   6.7e-116:284:99
   AL031178
F-NT2RP3000059
   R.norvegicus mRNA for leucocyte common antigen-related protein (3941 bp).
   0.0031:511:59
   X83546
F-NT2RP3000063
   Homo sapiens chromosome 19, fosmid 37502, complete sequence.
   0.20:544:57
   AC004755
F-NT2RP3000125
   HS_3025_A1_D11_MR CIT Approved Human Genomic Sperm Library D Homo sapiens genomic clone Plate=3025 Col=21 Row=G, genomic survey sequence.
   1.0e-21:161:88
   AQ101452
F-NT2RP3000148
   Human Chromosome 16 BAC clone CIT987SK-A-635H12, complete sequence.
   5.2e-40:257:77
   AC002310
F-NT2RP3000169
   Homo sapiens MRS1 mRNA, complete cds.
   3.4e-106:501:99
   AF093239
F-NT2RP3000171
   Mus musculus mRNA for B-IND1 protein.
   1.8e-97:571:89
   Z97207
F-NT2RP3000172
   Rattus norvegicus vesicla-associate calmodulin-binding protein mRNA, complete cds.
   2.0e-123:702:86
   L22557
F-NT2RP3000201
   Homo sapiens mRNA for KIAA0687 protein, partial cds.
   9.2e-170:792:98
   AB014587
F-NT2RP3000232
   HS_3238_B2_D04_MR CIT Approved Human Genomic Sperm Library D Homo sapiens genomic clone Plate=3238 Col=8 Row=H, genomic survey sequence.
   9.2e-24:174:88
   AQ219879
F-NT2RP3000304
   Homo sapiens LDL receptor-related protein 6 (LRP6) mRNA, complete cds.
   3.3e-171:797:98
   AF074264
F-NT2RP3000378
   Mus musculus (clone pVZmSin3A9) mSin3A9 mRNA, complete cds.
   5.8e-137:774:89
   L38621
F-NT2RP3000427
   Mouse cAMP-dependent protein kinase beta subunit gene, exon 1.
   1.5e-18:390:65
   M21096
F-NT2RP3000436
   cDNA encoding a human novel protein disulfide isomerase like enzyme,EP52.
   4.5e-05:353:59
   E13330
F-NT2RP3000444
   Human DNA sequence *** SEQUENCING IN PROGRESS *** from clone 1177I5, WORKING DRAFT SEQUENCE.
   9.7e-75:203:97
   AL022315
F-NT2RP3000460
   Canis familiaris sec61 homologue mRNA, complete cds.
   7.1e-131:643:88
   M96629
F-NT2RP3000481
   Homo sapiens RanBP7/importin 7 mRNA, complete cds.
   1.7e-162:770:98
   AF098799
F-NT2RP3000616
   Homo sapiens KIAA0405 mRNA, complete cds.
   4.7e-31:579:62
   AB007865
F-NT2RP3000645
   Human chromosome 12p13 sequence, complete sequence.
   5.9e-07:484:61
   U47924
F-NT2RP3000652
   Human ZNF43 mRNA.
   4.4e-131:853:84
   X59244
F-NT2RP3000676
   Homo sapiens mRNA for KIAA0446 protein, complete cds.
   2.7e-86:420:98
   AB007915
F-NT2RP3000677
   Human estrogen receptor-related protein (variant ER from breast cancer) mRNA, complete cds.
   2.9e-21:125:100
   M69296
F-NT2RP3000721
   HS_2221_A2_C01_MF CIT Approved Human Genomic Sperm Library D Homo sapiens genomic clone Plate=2221 Col=2 Row=E, genomic survey sequence.
   0.94:254:60
   AQ253443
F-NT2RP3000789
   Mus musculus coding region determinant binding protein mRNA, complete cds.
   5.4e-139:827:87
   AF061569
F-NT2RP3000818
   Human DNA sequence *** SEQUENCING IN PROGRESS *** from clone 94M16, WORKING DRAFT SEQUENCE.
   3.0e-28:218:86
   Z97201
F-NT2RP3000820
   Mus musculus WSB-1 mRNA, complete cds.
   1.1e-77:477:87
   AF033186
F-NT2RP3000838
   Homo sapiens mRNA for KIAA0638 protein, partial cds.
   2.6e-77:682:79
   AB014538
F-NT2RP3000871
   Homo sapiens retinoblastoma-interacting protein (RBBP8) mRNA, complete cds.
   5.8e-07:350:60
   AF043431
F-NT2RP3000907
   Drosophila melanogaster DNA sequence (P1 DS06882 (D310)), complete sequence.
   1.7e-13:330:62
   AC005115
F-NT2RP3000921
   cDNA GA3-43 encoding novel polypeptide which appear when differentiate from embryo-tumor cell P19 to nerve cell.
   6.8e-68:812:69
   E12950
F-NT2RP3001012
   cDNA encoding novel rat protein TIP120 which is formed of complex with TBP (TATA binding protein).
   2.4e-129:692:92
   E12829
F-NT2RP3001044
   Homo sapiens clone NH0001P09, WORKING DRAFT SEQUENCE, 1 unordered pieces.
   3.7e-60:393:79
   AC006030
F-NT2RP3001061
   F.rubripes GSS sequence, clone 154E17aC12, genomic survey sequence.
   1.8e-07:239:62
   AL018519
F-NT2RP3001159
   Homo sapiens chromosome 11, BAC CIT-HSP-311e8 (BC269730) containing the hFEN1 gene, complete sequence.
   4.4e-24:156:72
   AC004770
F-NT2RP3001170
   Homo sapiens mRNA for KIAA0784 protein, partial cds.
   2.3e-181:859:98
   AB018327
F-NT2RP3001195
   Genomic sequence from Human 9q34, complete sequence.
   3.8e-53:253:92
   AC001644
F-NT2RP3001240
   Canis familiaris sec61 homologue mRNA, complete cds.
   1.4e-133:740:87
   M96629
P-NT2RP3001271
   Homo sapiens chromosome 19, cosmid F20237, complete sequence.
   0.082:370:60
   AC005775
F-NT2RP3001322
   Homo sapiens mRNA for KIAA0566 protein, partial cds.
   1.9e-38:728:63
   AB011138
F-NT2RP3001388
   Rattus norvegicus synaptotagmin XI mRNA, complete cds.
   1.2e-103:701:83
   AF000423
F-NT2RP3001542
   Human Chromosome 11 Cosmid cSRL34e5, complete sequence.
   8.6e-17:293:65
   U73643
F-NT2RP3001560
   Mouse mRNA for thymic epithelial cell surface antigen, complete cds.
   7.8e-135:742:91
   D67067
F-NT2RP3001592
   Human Cdk-inhibitor p57KIP2 (KIP2) mRNA, complete cds.
   7.2e-12:188:71
   U22398
F-NT2RP3001650
   Homo sapiens PAC clone DJ0722F20 from 7q31.1-q31.3, complete sequence.
   1.9e-26:374:72
   AC005281
F-NT2RP3001685
   Homo sapiens 12q13.1 Cosmid C174F5 (Lawrence Livermore LL12NC01 or LL12NC02 human cosmid libraries) complete sequence.
   4.6e-73:284:98
   AC004550
F-NT2RP3001738
   Homo sapiens chromosome 11, BAC CIT-HSP-311e8 (BC269730) containing the hFEN1 gene, complete sequence.
   1.8e-21:186:67
   AC004770
F-NT2RP3001754
   Human DNA sequence *** SEQUENCING IN PROGRESS *** from clone 50024, WORKING DRAFT SEQUENCE.
   5.0e-21:131:96
   AL034380
F-NT2RP3001858
   Homo sapiens mRNA for KIAA0584 protein, partial cds.
   5.9e-39:770:63
   AB011156
F-NT2RP3001976
   M.domesticus (C57B1/6J) mRNA for zinc finger protein 30.
   2.0e-37:536:70
   Z30174
F-NT2RP3002015
   Homo sapiens huntingtin gene, partial exon.
   0.024:175:65
   L49359
F-NT2RP3002160
   Homo sapiens chromosome 9q34, clone 70C11, complete sequence.
   1.6e-95:249:91
   AC002319
F-NT2RP3002281
   Homo sapiens mRNA for KIAA0765 protein, partial cds.
   1.6e-149:713:98
   AB018308
F-NT2RP3002286
   Mus musculus EGF repeat transmembrane protein mRNA, complete cds.
   2.0e-136:756:92
   U57368
F-NT2RP3002311
   Mouse beta-galactosidase (BGAL) gene, complete cds.
   1.0e-29:624:63
   M57734
F-NT2RP3002324
   Human DNA sequence from cosmid RJ14 from a contig from the tip of the short arm of chromosome 16, spanning 2Mb of 16p13.3. Contains ESTs and CpG island.
   5.7e-122:655:93
   Z69890
F-NT2RP3002342
   Human transporter protein (g17) mRNA, complete cds.
   9.8e-36:565:65
   U49082
F-NT2RP3002353
   Streptomyces phaeochromogenes plasmid pJV1, complete sequence.
   0.15:466:60
   U23762
F-NT2RP3002409
   Homo sapiens mRNA for KIAA0719 protein, complete cds.
   2.0e-189:897:98
   AB018262
F-NT2RP3002411
   Mus musculus putative steroid dehydrogenase (KIK-I) mRNA, complete cds.
   7.8e-122:796:84
   AF064635
F-NT2RP3002448
   R.norvegicus mRNA for leucocyte common antigen-related protein (3941 bp).
   4.0e-11:403:64
   X83546
F-NT2RP3002571
   Bos taurus mRNA for lyncein.
   8.7e-114:652:90
   Y17923
F-NT2RP3002664
   H.sapiens CpG island DNA genomic Mse1 fragment, clone 34a2, reverse read cpg34a2.rt1a.
   6.1e-14:211:72
   Z60772
F-NT2RP3002721
   Homo sapiens citrate synthase mRNA, complete cds.
   7.5e-179:873:96
   AF047042
F-NT2RP3002737
   Homo sapiens mRNA for HNSPC, complete cds.
   1.4e-42:409:75
   D82346
F-NT2RP3002738
   Rattus norvegicus mRNA for Crk-associated substrate, p130, complete cds.
   8.9e-122:812:83
   D29766
F-NT2RP3002790
   Human Cdk-inhibitor p57KIP2 (KIP2) mRNA, complete cds.
   2.2e-15:626:62
   U22398
F-NT2RP3002836
   Homo sapiens mRNA for KIAA0463 protein, partial cds.
   6.8e-152:717:99
   AB007932
F-NT2RP3002887
   R.norvegicus mRNA for leucocyte common antigen-related protein (3941 bp).
   2.0e-05:491:59
   X83546
F-NT2RP3002900
   Cricetulus griseus COP-coated vesicle membrane protein CHOp24 mRNA, partial cds.
   7.3e-13:327:66
   U26264
F-NT2RP3002958
   Mus musculus IgK chain (6S) intron with insertion/deletion mutations.
   5.6e-22:403:66
   L12153
F-NT2RP3002983
   Homo sapiens genomic DNA, chromosome 21q11.1, segment 18/28, WORKING DRAFT SEQUENCE.
   1.2e-118:339:99
   AP000047
F-NT2RP3003000
   Homo sapiens T-type calcium channel alpha-1 subunit mRNA, complete cds.
   7.9e-88:555:88
   AF051946
F-NT2RP3003076
   Streptomyces coelicolor cosmid 2A11.
   0.15:505:59
   AL031184
F-NT2RP3003354
   Homo sapiens secretory carrier membrane protein (SCAMP2) mRNA, complete cds.
   1.2e-34:625:64
   AF005038
F-NT2RP3003448
   CIT-HSP-721P7.TV CIT-HSP Homo sapiens genomic clone 721P7, genomic survey sequence.
   1.2e-16:126:89
   B50017
F-NT2RP3003469
   Homo sapiens chromosome 19, cosmid F23990, complete sequence.
   2.0e-18:126:94
   AC005262
F-NT2RP3003473
   Homo sapiens chromosome 17, clone hRPK.1003_J_3, complete sequence.
   7. 1e-68:474:71
   AC005181
F-NT2RP3003527
   Homo sapiens mRNA for protein kinase Dyrk1B.
   1.4e-160:769:98
   Y17999
F-NT2RP3003532
   Mus musculus cell surface molecule OX-2 mRNA, complete cds.
   1.3e-96:712:80
   AF004023
F-NT2RP3003535
   Drosophila melanogaster (P1 DS02368 (D205)) DNA sequence, complete sequence. 0.027:155:65
   AC004313
F-NT2RP3003559
   H.sapiens CpG island DNA genomic Mse1 fragment, clone 171h5, reverse read cpg171h5.rt1a.
   3.9e-50:261:97
   Z59762
F-NT2RP3003614
   Mus musculus semaphorin VIa mRNA, complete cds.
   1.7e-131:811:86
   AF030430
F-NT2RP3003729
   Homo sapiens chromosome 10 clone LA10NC01_15_E_11 map 10q26.3, WORKING DRAFT SEQUENCE, 3 unordered pieces.
   1.4e-97:259:91
   AC006171
F-NT2RP3003849
F-NT2RP3003874
   M.musculus mRNA for myosin I heavy chain.
   2.9e-151:863:89
   X69987
F-NT2RP3003939
   T24C19TF TAMU Arabidopsis thaliana genomic clone T24C19, genomic survey sequence.
   1.4e-19:293:68
   B29025
F-NT2RP3003963
   CIT-HSP-2050C19.TF CIT-HSP Homo sapiens genomic clone 2050C19, genomic survey sequence.
   1.3e-16:111:95
   B80539
F-NT2RP3004000
   Homo sapiens klotho gene, exon 1.
   0.042:430:60
   AB009666
F-NT2RP3004025
   Human DNA sequence from Fosmid 49D8 on chromosome 22, complete sequence.
   0.062:197:65
   Z82186
F-NT2RP3004067
   Human mRNA for KIAA0375 gene, complete cds.
   1.7e-33:556:66
   AB002373
F-NT2RP3004075
   jd187 Trypanosome Shotgun M13 genomic Trypanosoma brucei brucei genomic clone 5H9, genomic survey sequence.
   1.5e-12:438:61
   B13419
F-NT2RP3004083
F-NT2RP3004090
   Dog alpha-L-iduronidase (IDUA) gene, exons 7-12.
   1.4e-06:469:60
   L01060
F-NT2RP30041 19
   Human mRNA for KIAA0215 gene, complete cds.
   1.3e-72:640:75
   D86969
F-NT2RP3004130
F-NT2RP3004133
   Pseudomonas aeruginosa phage phi CTX DNA, complete genome.
   0.0018:421:60
   Y13918
F-NT2RP3004202
F-NT2RP3004294
   Xenopus laevis ER1 mRNA, complete cds.
   5.0e-77:335:78
   AF015454
F-NT2RP3004309
   Homo sapiens chromosome 11, BAC CIT-HSP-311e8 (BC269730) containing the hFEN1 gene, complete sequence.
   9.6e-25:231:65
   AC004770
F-NT2RP3004321
   Homo sapiens chromosome 11 from 11p15.5 region, complete sequence.
   3.7e-80:279:95
   AF015416
F-NT2RP3004345
   Human Cdk-inhibitor p57KIP2 (KIP2) mRNA, complete cds.
   7.2e-12:188:71
   U22398
F-NT2RP3004355
   HS_3212_A1_C08_MR CIT Approved Human Genomic Sperm Library D Homo sapiens genomic clone Plate=3212 Col=15 Row=E, genomic survey sequence.
   0.061:266:65
   AQ176625
F-NT2RP3004374
   HS-1053-B2-E09-MR.abi CIT Human Genomic Sperm Library C Homo sapiens genomic clone Plate=CT 775 Col=18 Row=J, genornic survey sequence.
   1.3e-06:181:67
   B41504
F-NT2RP3004406
   CIT-HSP-2340N18.TF CIT-HSP Homo sapiens genomic clone 2340N18, genomic survey sequence.
   9.9e-74:359:99
   AQ058326
F-NT2RP3004481
   Mus musculus bassoon gene, exon 6 to 11.
   0.0060:528:59
   Y17038
F-NT2RP3004552
   Mouse mRNA for seizure-related gene product 6 type 2 precursor, complete cds.
   7.6e-40:731:64
   D64009
F-NT2RP3004557
   Human Ki nuclear autoantigen mRNA, complete cds.
   8.1e-120:626:94
   U11292
F-NT2RP3004625
   Homo sapiens I-1 receptor candidate protein mRNA, complete cds.
   9.8e-151:710:98
   AF082516
F-NT2RP3004640
   Bos taurus tuftelin mRNA, complete cds.
   8.2e-104:565:87
   AF105228
F-NT2RP3004647
   Homo sapiens mRNA for KIAA0446 protein, complete cds.
   2.1e-109:524:98
   AB007915
F-NT2RP4000108
   Human gene for neurofilament subunit NF-L.
   7.0e-158:862:93
   X05608
F-NT2RP4000634
   Sequence 11 from patent US 5753446.
   2.9e-155:828:92
   AR008281
F-NT2RP4000962
   Mus musculus clone OST66, genomic survey sequence.
   6.0e-48:352:81
   AF046696
F-NT2RP4001001
   Homo sapiens chromosome 5, Bac clone 58g14 (LBNL H76), complete sequence.
   4.8e-47:360:84
   AC005915
F-NT2RP4001009
   Homo sapiens CAAX prenyl protease (STE24) mRNA, complete cds.
   5.9e-175:828:98
   AF064867
F-NT2RP4001467
   Human placental cDNA coding for 5'nucleotidase (EC 3.1.3.5).
   3.3e-159:742:98
   X55740
F-NT2RP4001877
   1.7e-27:401:69
   AC005637
F-NT2RP4001879
F-NT2RP4002187
   Mus musculus putative steroid dehydrogenase (KIK-I) mRNA, complete cds.
   4.2e-115:777:83
   AF064635
F-NT2RP4002451
   Human DNA sequence *** SEQUENCING IN PROGRESS *** from clone 111B22, WORKING DRAFT SEQUENCE.
   6.1e-86:452:96
   Z98200
F-NT2RP4002715
   Homo sapiens clone NH0523H20, complete sequence.
   3.6e-59:410:77
   AC005041
F-NT2RP4002750
   Mus musculus cationic amino acid transporter (CAT3) mRNA, complete cds.
   3.4e-105:586:87
   U70859
F-OVARC1000003
   B.taurus mRNA for sodium dependent phosphate transporter.
   9.0e-125:823:83
   X81699
F-OVARC1000090
   RPCI11-25E14.TP RPCI-11 Homo sapiens genomic clone RPCI-11-25E14, genomic survey sequence.
   1.9e-06:151:74
   B86784
F-OVARC1000105
   S.cerevisiae UBC6 gene.
   4.6e-25:525:64
   X73234
F-OVARC1000137
   Human SNARE protein Ykt6 (YKT6) mRNA, complete cds.
   1.2e-33:184:98
   U95735
F-OVARC1000208
   Homo sapiens Chromosome 16 BAC clone CIT987SK-A-761H5, complete sequence.
   1.7e-79:362:91
   AC002544
F-OVARC1000255
   Porcine protein-tyrosine kinase (syk) mRNA, complete cds.
   4.9e-116:424:88
   M73237
F-OVARC1000275
   Homo sapiens chromosome 21q22.3, PAC clones 314N7, 225L15, BAC clone 7B7, complete sequence bases 1..333303.
   0.32:314:61
   AJ011930
F-OVARC1000298
   Homo sapiens clone RG031N19, WORKING DRAFT SEQUENCE, 1 unordered pieces.
   2.5e-121:306:98
   AC005632
F-OVARC1000307
   Bovine herpesvirus type 1 genes for UL[27,28,29,30,31].
   0.017:162:67
   X94677
F-OVARC1000313
   Homo sapiens mRNA for KIAA0573 protein, partial cds.
   1.7e-119:585:97
   AB011145
F-OVARC1000331
   Sequence 2 from patent US 5756332.
   1.9e-48:290:91
   AR009648
F-OVARC1000410
   Homo sapiens mRNA for angiopoietin-like factor.
   4.6e-26:538:62
   Y16132
F-OVARC1000439
F-OVARC1000467
   HS_3008_A2_D12_MR CIT Approved Human Genomic Sperm Library D Homo sapiens genomic clone Plate=3008 Col=24 Row=G, genomic survey sequence.
   2.0e-11:132:82
   AQ116995
F-OVARC1000529
   HS_3092_B2_C11_MF CIT Approved Human Genomic Sperm Library D Homo sapiens genomic clone Plate=3092 Col=22 Row=F, genomic survey sequence.
   8.2e-12:115:84
   AQ127947
F-OVARC1000553
   Homo sapiens chromosome 19, cosmid R26894, complete sequence.
   6.5e-92:221:96
   AC005594
F-OVARC1000775
   Human chromosome 3p21.1 gene sequence.
   6.9e-69:380:95
   L13435
F-OVARC1000811
   Homo sapiens chromosome 16, cosmid clone 432A1 (LANL), complete sequence.
   6.7e-77:500:86
   AC004235
F-OVARC1000853
   HS_3234_A1_F05_MR CIT Approved Human Genomic Sperm Library D Homo sapiens genomic clone Plate=3234 Col=9 Row=K, genomic survey sequence.
   4.6e-05:111:71
   AQ191345
F-OVARC1000873
   Human DNA sequence from clone 1049G16 on chromosome 20q12-13.2 Contains gene similar to GLUCOSAMINE-6-SULFATASE, a nuclear receptor coactivator gene, ESTs, STSs, GSSs, complete sequence.
   8.2e-42:234:96
   AL034418
F-OVARC1000916
   Sequence 3 from patent US 5674748.
   2.0e-55:422:84
   I68139
F-OVARC1000956
   Human DNA sequence from cosmid L241B9, Huntington's Disease Region, chromosome 4p16.3 contains polymorphic VNTR pYNZ32.
   1.2e-107:540:97
   Z69708
F-OVARC1000995
   H.sapiens genomic DNA (chromosome 3; clone NL1106D).
   4.3e-28:166:95
   X87478
F-OVARC1001030
   Human mRNA for KIAA0339 gene, complete cds.
   2.1e-10:334:64
   AB002337
F-OVARC1001049
   *** SEQUENCING IN PROGRESS *** Homo sapiens chromosome 4, BAC clone C0190L06; HTGS phase 1, WORKING DRAFT SEQUENCE, 21 unordered pieces.
   5.4e-12:420:62
   AC004670
F-OVARC1001086
   Homo sapiens cyclin T2a mRNA, complete cds.
   1.9e-164:761:99
   AF048731
F-OVARC1001132
   Homo sapiens genomic DNA, chromosome 21q11.1, segment 9/28, WORKING DRAFT SEQUENCE.
   1.5e-89:328:75
   AP000038
F-OVARC1001163
   Caenorhabditis elegans cosmid F40E10, complete sequence.
   3.8e-26:337:71
   Z69792
F-OVARC1001222
   CIT-HSP-2010I15.TR CIT-HSP Horno sapiens genomic clone 2010I15, genomic survey sequence.
   1.2e-08:171:70
   B57734
F-OVARC1001260
F-OVARC1001336
   B.taurus mRNA for sodium dependent phosphate transporter.
   5.4e-83:622:80
   X81699
F-OVARC1001338
   HS_2181_B2_E11_MF CIT Approved Human Genomic Sperm Library D Homo sapiens genomic clone Plate=2181 Col=22 Row=J, genomic survey sequence.
   2.3e-17:144:86
   AQ022764
F-OVARC1001569
   Mus musculus bone morphogenetic factor 11 (Bmp11) gene, exon 1.
   2.9e-06:241:63
   AF100904
F-OVARC1001570
   Homo sapiens *** SEQUENCING IN PROGRESS ***, WORKING DRAFT SEQUENCE.
   1.6e-10:235:64
   AJ011929
F-OVARC1001596
   Homo sapiens chromosome 17, clone hRPK.372_K_20, complete sequence.
   2.2e-45:498:73
   AC005951
F-OVARC1001607
   Human beta-1,2-N-acetylglucosaminyltransferase II (MGAT2) gene, complete cds.
   1.7e-38:323:80
   U15128
F-OVARC1001725
   Homo sapiens patched related protein TRC8 (TRC8) gene, exon 1 and partial cds.
   3.5e-172:821:98
   AF064800
F-OVARC1001727
   Human DNA sequence from clone 48A11 on chromosome 20p12 Contains EST, STS, GSS, complete sequence.
   2.2e-132:633:98
   AL031132
F-OVARC1001807
   Human TR3 orphan receptor mRNA, complete cds.
   7. le-90:566:87
   L13740
F-OVARC 1001833
   Rattus norvegicus cis-Golgi matrix protein GM130 mRNA, complete cds.
   5.2e-46:364:79
   U35022
F-OVARC1001952
   Homo sapiens FGFR-4 gene.
   1.7e-14:392:62
   Y13901
F-OVARC1001991
   Human Chromosome 11 Cosmid cSRL34e5, complete sequence.
   2.3e-06:298:64
   U73643
F-OVARC1002058
   , complete sequence.
   1.3e-108:617:92
   AC005500
F-OVARC1002178
   Herpes simplex virus type 2 (strain HG52), complete genome.
   0.43:234:63
   Z86099
F-PLACE1000033
   Mus musculus otogelin mRNA, complete cds.
   5.9e-18:579:59
   U96411
F-PLACE1000231
   Rattus norvegicus WAP four-disulfide core domain protein (ps20) mRNA, complete cds.
   1.1e-18:273:68
   AF037272
F-PLACE1000258
   Human KRAB zinc finger protein (ZNP177) mRNA, complete cds.
   1.2e-13:241:70
   U37263
F-PLACE1000442
   Human zinc finger protein ZNF136.
   2.3e-87:774:76
   U09367
F-PLACE1000560
   Homo sapiens chromosome 5, BAC clone 194j18 (LBNL H158), complete sequence.
   4.1e-107:318:96
   AC005368
F-PLACE1000740
   Rat notch 2 mRNA.
   1.1e-37:399:74
   M93661
F-PLACE1000907
   RPCI11-73M20.TJ RPCI11 Homo sapiens genomic clone R-73M20, genomic survey sequence.
   3.5e-21:147:92
   AQ269030
F-PLACE1000912
F-PLACE1000914
   Homo sapiens chromosome 17, clone HCIT145P4, WORKING DRAFT SEQUENCE, 8 unordered pieces.
   1.8e-74:206:93
   AC002093
F-PLACE1000927
   Cowpox virus strain GRI-90 DNA (49 kb fragment).
   6.8e-75:683:74
   Y15035
F-PLACE1000986
   RPCI11-75H23.TK RPCI11 Homo sapiens genomic clone R-75H23, genomic survey sequence.
   1.0:316:57
   AQ268409
F-PLACE1001016
   Human dihydropyridine-sensitive L-type calcium channel alpha-1 subunit (CACNL1A3) mRNA, complete cds.
   0.28:432:59
   L33798
F-PLACE1001100
   RPCI11-32N5.TK RPCI-11 Homo sapiens genomic clone RPCI-11-32N5, genomic survey sequence.
   0.48:145:64
   AQ047336
F-PLACE1001114
   Lysobacter enzymogenes beta-lactamase gene sequence.
   0.033:349:60
   M97392
F-PLACE1001123
   F.rubripes GSS sequence, clone 084A20aC12, genomic survey sequence.
   9.7e-05:138:64
   AL015804
F-PLACE1001183
   Homo sapiens BAC clone RG318C11 from 7p14-p15, complete sequence.
   0.15:576:59
   AC005091
F-PLACE1001229
   F.rubripes GSS sequence, clone 144D13aC10, genomic survey sequence.
   2.2e-21:271:70
   AL017986
F-PLACE1001231
   Rattus norvegicus sodium-dependent multi-vitamin transporter (SMVT) mRNA, complete cds.
   6.4e-102:677:84
   AF026554
F-PLACE1001340
   Homo sapiens mRNA for KIAA0719 protein, complete cds.
   1.3e-130:636:97
   AB018262
F-PLACE1001401
   CIT-HSP-2323H22.TR CIT-HSP Homo sapiens genomic clone 2323H22, genomic survey sequence.
   6.4e-13:165:76
   AQ028562
F-PLACE1001407
   Human DNA sequence from clone 496H19 on chromosome 6q24 Contains ESTs, complete sequence.
   2.4e-28:228:85
   AL023582
F-PLACE1001464
   Human placental cDNA coding for 5 nucleotidase (EC 3.1.3.5).
   5.0e-151:742:96
   X55740
F-PLACE1001500
   CIT-HSP-2368L16.TR CIT-HSP Homo sapiens genomic clone 2368L16, genomic survey sequence.
   1.1e-25:150:97
   AQ078655
F-PLACE1001516
   Homo sapiens Chromosome 16 BAC clone CIT987SK-A-575C2, complete sequence.
   1.2e-139:676:98
   AC002425
F-PLACE1001536
   Human Chromosome X clone bWXD173, WORKING DRAFT SEQUENCE, 2 ordered pieces.
   1.7e-142:513:97
   AC004387
F-PLACE1001564
   Human DNA sequence *** SEQUENCING IN PROGRESS *** from clone 287G14, WORKING DRAFT SEQUENCE.
   2.9e-104:373:89
   AL033377
F-PLACE1001655
   Homo sapiens Shab-related delayed-rectifier K+ channel alpha subunit (KCNS3) mRNA, complete cds.
   1.3e-123:585:98
   AF043472
F-PLACE1001788
   Homo sapiens mRNA for HYA22, complete cds.
   9.9e-21:234:75
   D88153
F-PLACE1001795
   Drosophila melanogaster; Chromosome 3L; Region 83F1-83F2; P1 clone DS07437, WORKING DRAFT SEQUENCE, 3 unordered pieces.
   1.4e-05:218:64
   AC005985
F-PLACE1001836
   Homo sapiens BAC clone GS155M11 from 7q21-q22, complete sequence.
   4.9e-79:577:82
   AC004022
F-PLACE1001918
   Arabidopsis thaliana BAC T19D16 genomic sequence.
   3.7e-24:417:63
   U95973
F-PLACE1001949
   S.cerevisiae chromosome XV reading frame ORF YOR291w.
   3.6e-16:255:70
   Z75199
F-PLACE1002080
   Homo sapiens antigen NY-CO-9 (NY-CO-9) mRNA, partial cds.
   7.5e-129:622:98
   AF039691
F-PLACE1002095
   Homo sapiens chromosome 5, P1 clone 1130f1 (LBNL H40), complete sequence.
   2.3e-48:551:71
   AC004219
F-PLACE1002153
   Homo sapiens TACC2 protein (TACC2) mRNA, partial cds.
   8.3e-161:764:98
   AF095791
F-PLACE1002329
   Sequence 12 from Patent WO 9000403.
   6.9e-05:380:63
   I09634
F-PLACE1002355
   Homo sapiens protease-activated receptor 4 mRNA, complete cds.
   2.8e-17:190:77
   AF055917
F-PLACE1002374
   Human mRNA for pro-cathepsin L(major excreted protein MEP).
   6.2e-162:716:94
   X12451
F-PLACE 1002518
   HS_3091_A1_F08_MF CIT Approved Human Genomic Sperm Library D Homo sapiens genomic clone Plate=3091 Col=15 Row=K, genomic survey sequence.
   3.2e-74:316:94
   AQ123005
F-PLACE1002547
   Homo sapiens mRNA for KIAA0719 protein, complete cds.
   2.6e-171:819:98
   AB018262
F-PLACE1002726
   CIT-HSP-2369G10.TR CIT-HSP Homo sapiens genomic clone 2369G10, genomic survey sequence.
   4.8e-18:135:88
   AQ075115
F-PLACE1002905
   Drosophila melanogaster DNA sequence (P1 DS00906 (D99)), complete sequence.
   3.7e-06:235:66
   AC004154
F-PLACE1002911
   Bovine herpesvirus 1 complete genome.
   0.93:264:63
   AJ004801
F-PLACE1002967
   Homo sapiens IgE receptor beta chain (HTm4) mRNA, complete cds.
   0.0041:302:60
   L35848
F-PLACE1003135
   Homo sapiens STE20-like kinase 3 (mst-3) mRNA, complete cds.
   4.7e-49:450:75
   AF024636
F-PLACE1003163
   Homo sapiens DBI-related protein mRNA, complete cds.
   4.7e-152:722:98
   AF069301
F-PLACE1003407
   Homo sapiens putative transmembrane protein (CLN5) mRNA, complete cds.
   6.3e-141:682:97
   AF068227
F-PLACE1003428
   Human DNA sequence from clone 55C23 on chromosome 6q22.3-23.3 contains vanin-like genes VNN1 and VNN2, ESTs, GSSs,, complete sequence.
   1.2e-116:286:100
   AL032821
F-PLACE1003438
   Pseudomonas alcaligenes outer membrane Xcp-secretion system gene cluster.
   0.13:468:60
   AF092918
F-PLACE1003460
   HS_3234_A1_F05_MR CIT Approved Human Genomic Sperm Library D Homo sapiens genomic clone Plate=3234 Col=9 Row=K, genomic survey sequence.
   5.8e-05:111:71
   AQ191345
F-PLACE1003529
   Homo sapiens clone DJ0981O07, complete sequence.
   5.8e-134:457:97
   AC006017
F-PLACE1003573
   Sequence 2 from patent US 5792648.
   0.93:186:62
   AR022348
F-PLACE1003598
   Mus musculus mismatch repair protein (MSH6) gene, exon 1.
   3.3e-07:311:63
   AF031085
F-PLACE1003644
   Homo sapiens Chromosome 11q23 PAC clone pDJ149k2 containing PLZF gene encoding kruppel-like zinc finger protein, complete sequence.
   1.8e-06:138:74
   AC001234
F-PLACE1003737
   Homo sapiens Xp22-83 BAC GSHB-324M7 (Genome Systems Human BAC Library) complete sequence.
   1.4e-165:791:98
   AC005859
F-PLACE1003772
   Human p300/CBP-associated factor (P/CAF) mRNA, complete cds.
   2.2e-07:448:61
   U57317
F-PLACE1003839
   Homo sapiens Chromosome 16 BAC clone CIT987SK-A-69G12, complete sequence.
   2.0e-106:525:97
   AC004131
F-PLACE1003845
   Caenorhabditis elegans cosmid D2096.
   9.8e-26:386:69
   U40800
F-PLACE1003852
   Homo sapiens mRNA for KIAA0758 protein, partial cds.
   7.4e-171:814:98
   AB018301
F-PLACE1004028
F-PLACE1004078
   Homo sapiens PAC clone DJ0722F20 from 7q31.1-q31.3, complete sequence.
   2.0e-116:274:98
   AC005281
F-PLACE1004166
   HS_3223_A1_H09_T7 CIT Approved Human Genomic Sperm Library D Homo sapiens genomic clone Plate=3223 Col=17 Row=O, genomic survey sequence.
   0.77:304:58
   AQ193346
F-PLACE1004168
F-PLACE1004199
   CIT-HSP-2328F14.TR CIT-HSP Homo sapiens genomic clone 2328F14, genomic survey sequence.
   9.4e-16:186:76
   AQ042262
F-PLACE1004279
   Homo sapiens putative renal organic anion transporter 1 (hROAT1) mRNA, complete cds.
   1.2e-18:456:62
   AF057039
F-PLACE1004282
F-PLACE1004305
   Homo sapiens mRNA for KIAA0740 protein, complete cds.
   2.7e-121:612:96
   AB018283
F-PLACE1004441
   Human G protein-coupled receptor (GPR1) gene, complete cds.
   2.4e-104:537:95
   U13666
F-PLACE1004450
   Pleuronectes americanus aminopeptidase N (ampN) mRNA, complete cds.
   3.1e-20:601:60
   AF012465
F-PLACE1004482
   HS_3032_B1_C03_MR CIT Approved Human Genomic Sperm Library D Homo sapiens genomic clone Plate=3032 Col=5 Row=F, genomic survey sequence.
   1.1e-86:423:98
   AQ129106
F-PLACE1004492
   Human DNA sequence from PAC 434P1 on chromosome 22. Contains inward rectifier potassium channel 4, (potassium channel, inwardly rectifying, subfamily J, member 4) (hippocampal inward rectifier) (HIR) (HRK1) (HIRK2) (KIR2.3), ESTs similar to lumen protein retaining receptor 2 (KDEL receptor 2), DEAD-box protein P72, ESTs, CpG islands.
   0.17:180:67
   Z97056
F-PLACE1004519
   Human DNA sequence from clone 24o18 on chromosome 6p21.31-22.2 Contains zinc finger protein pseudogene, VNO-type olfactory receptor pseudogene, nuclear envelope pore membrane protein, EST, STS, GSS, complete sequence.
   1.1e-75:432:84
   AL021808
F-PLACE1004520
   Human pregnancy-specific beta-1-glycoprotein mRNA PSG95, complete cds.
   4.1e-109:606:92
   M34715
F-PLACE1004630
   Homo sapiens mRNA for osteoblast specific cysteine-rich protein, complete cds.
   6.2e-138:749:92
   AB008375
F-PLACE1004637
   HS-1061-B1-E10-MF.abi CIT Human Genomic Sperm Library C Homo sapiens genomic clone Plate=CT 783 Col=19 Row=J, genomic survey sequence.
   0.013:92:75
   B45487
F-PLACE1004648
F-PLACE1004816
   Homo sapiens mRNA for Hakata antigen, complete cds.
   3.8e-98:590:90
   D88587
F-PLACE1004887
   Dog alpha-L-iduronidase (IDUA) gene, exons 7-12.
   1.2e-06:469:60
   L01060
F-PLACE1005003
   Human SNC19 mRNA sequence.
   4.8e-20:472:63
   U20428
F-PLACE1005005
   Homo sapiens chromosome 21q22.3, PAC clones 314N7, 225L15, BAC clone 7B7, complete sequence bases 1..333303.
   7.8e-143:650:97
   AJ011930
F-PLACE1005031
   Bovine chlorine channel protein (p64) mRNA, complete cds.
   7.1e-62:463:83
   L16547
F-PLACE1005239
   Homo sapiens mRNA for HIRIP3 protein, clone pH4-31.
   2.2e-14:115:85
   AJ223349
F-PLACE1005250
   Mus musculus maternal transcript Maid (Maid) mRNA, complete cds.
   3.3e-40:370:77
   U50734
F-PLACE1005383
   Homo sapiens UP50 mRNA, complete cds.
   2.7e-126:633:96
   AF093118
F-PLACE1005410
   Rattus rattus sec61 homologue mRNA, complete cds.
   1.9e-115:771:85
   M96630
F-PLACE1005426
   Homo sapiens chromosome 19, CIT-HSP BAC 490g23 (BC338531), complete sequence.
   7.2e-113:391:96
   AC005392
F-PLACE1005519
   Homo sapiens STE20-like kinase 3 (mst-3) mRNA, complete cds.
   1.0e-53:521:74
   AF024636
F-PLACE1005539
   c-erbB=proto-oncogene {exon 1, promoter} [chickens, Genomic, 700 nt].
   3.6e-05:434:62
   S66408
F-PLACE1005544
   Mus musculus junctional adhesion molecule (Jam) mRNA, complete cds.
   3.3e-56:575:74
   U89915
F-PLACE1005569
   Human DNA sequence *** SEQUENCING IN PROGRESS *** from clone 159A1, WORKING DRAFT SEQUENCE.
   1.1e-118:381:96
   AL034397
F-PLACE1005601
   Human PAC clone DJ515N1 from 22q11.2-q22, complete sequence.
   3.9e-143:697:98
   AC002073
F-PLACE1005660
F-PLACE1005669
   Fruit fly (D.melanogaster) Glued mRNA, complete cds.
   3.4e-14:275:66
   J02932
F-PLACE1005682
   Mus musculus Ankhzn mRNA, complete cds.
   0.75:347:57
   AB011370
F-PLACE1005725
   Homo sapiens huntingtin (HD) gene, exon 1.
   1.4e-06:425:62
   L27350
F-PLACE1005736
F-PLACE1005745
   HS_3039_B1_F12_MF CIT Approved Human Genomic Sperm Library D Homo sapiens genomic clone Plate=3039 Col=23 Row=L, genomic survey sequence.
   1.0:283:59
   AQ155068
F-PLACE1005768
   Human DNA sequence from Fosmid 24E5 on chromosome 22q11.2-qter contains parvalbumin, ESTs, STS.
   1.5e-141:719:96
   Z82185
F-PLACE1005815
   Sequence 1 from patent US 5571905.
   0.088:199:62
   I28535
F-PLACE1005878
   Bovine chlorine channel protein (p64) mRNA, complete cds.
   2.5e-54:394:84
   L16547
F-PLACE1005927
   HS_3138_B2_B03_MR CIT Approved Human Genomic Sperm Library D Homo sapiens genomic clone Plate=3138 Col=6 Row=D, genomic survey sequence.
   8.0e-32:162:95
   AQ183333
F-PLACE1006071
   1.6e-180:877:96
   AF028816
F-PLACE1006073
   Homo sapiens mRNA for glucuronyltransferase I, complete cds.
   1.7e-94:464:98
   AB009598
F-PLACE1006079
   Homo sapiens distal-less homeobox protein (DLX3) gene, complete cds.
   5.2e-107:423:96
   AF028233
F-PLACE1006093
   jd187 Trypanosome Shotgun M13 genomic Trypanosoma brucei brucei genomic clone 5H9, genomic survey sequence.
   0.00018:316:60
   B13419
F-PLACE1006208
   Bovine herpesvirus type 1 early-intermediate transcription control protein (BICP4) gene, complete cds.
   1.4e-12:421:64
   L14320
F-PLACE1006219
   Caenorhabditis elegans cosmid D2096.
   6.4e-25:386:69
   U40800
F-PLACE1006277
   Human DNA sequence *** SEQUENCING IN PROGRESS *** from clone 159A1, WORKING DRAFT SEQUENCE.
   7.2e-135:381:97
   AL034397
F-PLACE1006290
   Caenorhabditis elegans cosmid F09E5.
   1.4e-08:354:61
   U37429
F-PLACE1006443
   Homo sapiens chromosome 17, clone HCIT145P4, WORKING DRAFT SEQUENCE, 8 unordered pieces.
   2.9e-80:168:95
   AC002093
F-PLACE1006515
   Homo sapiens mRNA for KIAA0576 protein, partial cds.
   4.2e-140:655:99
   AB011148
F-PLACE1006716
   Human DNA sequence from PAC 151B14 on chromosome 22q12-qter contains somatostatin receptor subtype 3 (SSTR3), tRNA, ESTs, CpG island and STS.
   2.2e-51:621:70
   Z86000
F-PLACE1006786
   CITBI-E1-2502A9.TR CITBI-E1 Homo sapiens genomic clone 2502A9, genomic survey sequence.
   0.43:237:64
   AQ264473
F-PLACE1006809
   HS_2255_B1_F05_MR CIT Approved Human Genomic Sperm Library D Homo sapiens genomic clone Plate=2255 Col=9 Row=L, genomic survey sequence.
   2.1e-14:95:97
   AQ131814
F-PLACE1006959
   HS_3247_B1_E03_MR CIT Approved Human Genomic Sperm Library D Homo sapiens genomic clone Plate=3247 Col=5 Row=J, genomic survey sequence.
   1.1e-09:199:70
   AQ220414
F-PLACE1007028
   Homo sapiens Chromsome 11p15.5 PAC clone pDJ754h15 containing cdk-inhibitor p57/KIP2 (CDKN1C) gene, complete sequence.
   2.0e-24:658:62
   AC005950
F-PLACE1007040
   Mus musculus neuronal intermediate filament protein (alpha-internexin) gene, complete cds.
   8.8e-09:585:62
   L27220
F-PLACE1007077
F-PLACE1007081
   RPCI11-31D7.TJ RPCI-11 Homo sapiens genomic clone RPCI-11-31D7, genomic survey sequence.
   2.3e-42:228:97
   AQ016433
F-PLACE1007096
F-PLACE1007296
   Human mRNA for a presumptive KDEL receptor.
   1.3e-71:542:83
   X55885
F-PLACE1007591
   Homo sapiens full length insert cDNA clone YP44A02.
   1.1e-18:141:90
   AF085890
F-PLACE1007626
   Homo sapiens unknown mRNA, complete cds.
   7.8e-104:516:97
   AF047439
F-PLACE1007702
   Homo sapiens chromosome 17, clone 363G12, WORKING DRAFT SEQUENCE, 11 unordered pieces.
   7.5e-50:439:77
   AC002348
F-PLACE1007845
   Caenorhabditis elegans cosmid F09E5.
   4.4e-08:355:62
   U37429
F-PLACE1007881
   CITBI-E1-2517N6.TF CITBI-E1 Homo sapiens genomic clone 2517N6, genomic survey sequence.
   1.4e-14:104:95
   AQ279407
F-PLACE1007971
   HS_3237_B2_F09_MR CIT Approved Human Genomic Sperm Library D Homo sapiens genomic clone Plate=3237 Col=18 Row=L, genomic survey sequence.
   1.2e-12:169:76
   AQ206052
F-PLACE1008282
   Homo sapiens clone DJ0042M02, WORKING DRAFT SEQUENCE, 20 unordered pieces.
   4.5e-101:192:100
   AC005995
F-PLACE1008297
   Mycoplasma genitalium random genomic clone hg1, partial cds.
   0.099:193:60
   U02109
F-PLACE1008359
   Homo sapiens DNA for (CGG)n trinucleotide repeat region, isolate CL16-1 (Chr.16).
   0.53:185:65
   AJ001218
F-PLACE1008469
   Homo sapiens chromosome 17, clone HCIT145P4, WORKING DRAFT SEQUENCE, 8 unordered pieces.
   1.2e-93:213:98
   AC002093
F-PLACE1008549
   Homo sapiens E74-like factor 5 (ELF5) mRNA, complete cds.
   5.7e-144:693:98
   AF049703
F-PLACE1008657
   Bovine mRNA for adseverin, complete cds.
   5.6e-140:782:90
   D26549
F-PLACE1008716
   Human beta-1,2-N-acetylglucosaminyltransferase II (MGAT2) gene, complete cds.
   1.1e-133:648:97
   U15128
F-PLACE1008744
   Sequence 1 from patent US 5691147.
   8.4e-91:475:95
   I76197
F-PLACE1008984
   Human DNA sequence *** SEQUENCING IN PROGRESS *** from clone 167A19, WORKING DRAFT SEQUENCE.
   4.2e-103:493:99
   AL031427
F-PLACE1008985
   Mus musculus synaptotagmin VIII mRNA, partial cds.
   1.1e-23:289:72
   U20107
F-PLACE1009067
   H.sapiens CpG island DNA genomic Mse1 fragment, clone 52e12, forward read cpg52e12.ft1a.
   1.2e-28:164:96
   Z61442
F-PLACE1009196
F-PLACE1009279
   H.sapiens mRNA for serine protease.
   6.0e-10:327:64
   Y07921
F-PLACE1009527
   Human DNA-binding protein ABP/ZF mRNA, complete cds.
   2.0e-19:125:96
   U82613
F-PLACE1009546
   S.lividans mercury resistance operon.
   0.56:358:59
   X65467
F-PLACE1009600
   Mouse mRNA for tetracycline transporter-like protein, complete cds.
   2.1e-128:718:91
   D88315
F-PLACE1009735
   Homo sapiens clone NH0523H20, complete sequence.
   2.9e-128:613:99
   AC005041
F-PLACE1009982
F-PLACE1010011
   , complete sequence.
   2.1e-26:234:83
   AC005409
F-PLACE1010078
   Saccharomyces cerevisiae chromosome XII cosmid 8300.
   0.066:273:58
   U19028
F-PLACE1010081
   Homo sapiens serine/threonine kinase RICK (RICK) mRNA, complete cds.
   7.0e-150:733:97
   AF027706
F-PLACE1010251
   Sequence 1 from patent US 5665588.
   0.0012:309:62
   I64695
F-PLACE1010445
   Herpes simplex virus type 2 (strain HG52), complete genome.
   9.4e-07:511:58
   Z86099
F-PLACE1010713
   Mus musculus putative steroid dehydrogenase (KIK-I) mRNA, complete cds.
   2.1e-89:612:83
   AF064635
F-PLACE1010784
   Sequence 1 from patent US 5686597.
   2.5e-103:505:98
   I73723
F-PLACE1010827
   Cricetulus griseus COP-coated vesicle membrane protein CHOp24 mRNA, partial cds.
   7.3e-13:327:66
   U26264
F-PLACE1010968
   O.cuniculus mRNA for titin.
   0.44:165:64
   X64696
F-PLACE1011045
   Homo sapiens E1-like protein mRNA, complete cds.
   1.8e-127:595:99
   AF094516
F-PLACE1011116
   HS_2033_A2_E05_T7 CIT Approved Human Genomic Sperm Library D Homo sapiens genomic clone Plate=2033 Col=10 Row=I, genomic survey sequence.
   8.3e-29:192:92
   AQ229784
F-PLACE1011181
   H.sapiens CpG island DNA genomic Mse1 fragment, clone 99f2, reverse read cpg99f2.rt1a.
   4.8e-35:200:95
   Z64239
F-PLACE1011236
   Mus musculus mRNA for RST, complete cds.
   4.5e-54:717:66
   AB005451
F-PLACE1011364
   Homo sapiens protein kinase/endoribonulcease (IRE1) mRNA, complete cds.
   0.13:502:57
   AF059198
F-PLACE1011407
   M.domesticus (C57B1/6J) mRNA for zinc finger protein 30.
   7.2e-15:313:68
   Z30174
F-PLACE1011516
   Drosophila melanogaster; Chromosome 3L; Region 79F1-80A2; BAC clone BACR48E05, WORKING DRAFT SEQUENCE, 4 unordered pieces.
   1.8e-16:317:66
   AC005720
F-PLACE1011708
   Homo sapiens intrinsic factor-B12 receptor precursor, mRNA, complete cds.
   1.8e-143:722:96
   AF034611
F-PLACE1011824
   Human Ste20-like kinase (MST2) mRNA, complete cds.
   5.0e-100:561:92
   U26424
F-PLACE 10I 1978
   Figure 2. Nucleotide and translated protein sequences of HPF1, -2, and -9.
   9.6e-76:722:74
   M27877
F-PLACE2000118
   Homo sapiens DNA sequence from PAC 393P12 on chromosome Xp11.21. Contains a hypothetical protein KIAA0413 (KIAA0412, KIAA0065, KIAA0569) LIKE Zinc Finger protein pseudogene, a hypothetical Proline-rich protein KIAA0269 LIKE gene and a 60S Ribosomal Protein L7 LIKE pseudogene. Contains ESTs, an STS and a GSS (BAC end sequence), complete sequence.
   5.2e-112:568:95
   AL022578
F-PLACE2000219
   Homo sapiens Down Syndrome critical region, partial sequence.
   0.0059:144:71
   AF015262
F-PLACE3000181
   Sequence 102 from patent US 5643781.
   4.1e-127:745:90
   I51041
F-PLACE3000213
F-PLACE4000354
   HS_3071_A2_B06_MF CIT Approved Human Genomic Sperm Library D Homo sapiens genomic clone Plate=3071 Col=12 Row=C, genomic survey sequence.
   4.4e-12:335:64
   AQ137396
F-PLACE4000455
   Homo sapiens transcriptional enhancer factor (TEF1) DNA, complete CDS.
   9.5e-118:563:98
   M63896
F-SKNMC1000004
   Homo sapiens clone RG031N19, WORKING DRAFT SEQUENCE, 1 unordered pieces.
   2.9e-141:292:98
   AC005632
F-SKNMC1000014
   CIT-HSP-2359K11.TR CIT-HSP Homo sapiens genomic clone 2359K11, genomic survey sequence.
   0.89:136:67
   AQ075724
F-SKNMC1000082
   H.sapiens CpG island DNA genomic Msel fragment, clone 26g3, reverse read cpg26g3.rt1b.
   5.6e-06:60:98
   265216
F-THYRO1000036
F-THYRO1000061
   Homo sapiens chromosome 19, cosmid R28991, complete sequence.
   2.4e-105:425:94
   AC004623
F-THYRO1000099
   Rattus norvegicus leukocyte common antigen receptor (LAR) gene, trans-spliced alternative untranslated exon.
   0.35:609:57
   U87960
F-THYRO1000196
   Homo sapiens Ksp-cadherin (CDH16) mRNA, complete cds.
   5.1e-125:475:98
   AF016272
F-THYRO1000400
   Mycobacterium tuberculosis sequence from clone y423.
   1.0:264:59
   AD000014
F-THYRO1000580
   HS_3216_A1_H09_T7 CIT Approved Human Genomic Sperm Library D Homo sapiens genomic clone Plate=3216 Col=17 Row=O, genomic survey sequence.
   2.8e-25:157:96
   AQ184086
F-THYRO1000584
   Boar mRNA for 135kDa protein, complete cds.
   2.0e-104:787:80
   D28521
F-THYRO1000678
   M.musculus Cx30 gene.
   6.9e-41:285:85
   Z70023
F-THYRO1000776
   Drosophila melanogaster DNA sequence (P1 DS08948 (D168)), complete sequence.
   2.7e-10:389:59
   AC004288
F-THYRO1000795
   Rattus norvegicus mRNA for mitochondrial dicarboxylate carrier.
   1.2e-107:736:83
   AJ223355
F-THYRO1000846
   CITBI-E1-2505H6.TR CITBI-E1 Homo sapiens genomic clone 2505H6, genomic survey sequence.
   0.00025:351:61
   AQ260270
F-THYRO1000866
   Homo sapiens SKB1Hs mRNA, complete cds.
   3.3e-91:529:89
   AF015913
F-THYRO1000956
   Human G protein-coupled receptor APJ gene, complete cds.
   3.8e-148:724:97
   U03642
F-THYRO1000964
   Drosophila melanogaster Pelle associated protein Pellino (Pli) mRNA, complete cds.
   5.0e-37:714:64
   AF091624
F-THYRO1000999
   CITBI-E1-2508B3.TF CITBI-E1 Homo sapiens genomic clone 2508B3, genomic survey sequence.
   1.2e-06:280:62
   AQ261426
F-THYRO1001063
   H.sapiens (xs174) mRNA, 300bp.
   1.6e-41:298:85
   Z36825
F-THYRO1001071
   Human mRNA for KIAA0154 gene, partial cds.
   7.4e-16:197:73
   D63876
F-THYRO1001102
   Homo sapiens PAC clone DJ130H16 from 22q12.1-qter, complete sequence.
   3.5e-10:128:83
   AC004997
F-THYRO1001113
   Homo sapiens mRNA for LGMD2B protein.
   8.8e-52:684:68
   AJ007670
F-THYRO1001128
   Homo sapiens chromosome 9q34, clone 63G10, complete sequence.
   1.2e-141:227:97
   AC002096
F-THYRO1001205
F-THYRO1001237
   Mus musculus interleukin-2 (Il-2) gene, 5'end.
   0.77:78:74
   L07576
F-THYRO1001242
   Mouse mRNA for thymic epithelial cell surface antigen, complete cds.
   5.1e-127:721:90
   D67067
F-THYRO1001266
   Human sodium iodide symporter mRNA, complete cds.
   2.7e-41:806:62
   U66088
F-THYRO1001327
   Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
   3.1e-117:374:96
   AL021578
F-THYRO1001456
F-THYRO1001457
   M.musculus (Balb/c) mRNA for serine/threonine protein kinase.
   1.8e-57:491:69
   Z34524
F-THYRO1001471
   Sequence 52 from Patent WO9712992.
   0.00019:546:58
   A62364
F-THYRO1001478
F-THYRO1001495
   Homo sapiens clone DJ1163L11, complete sequence.
   4.4e-20:222:76
   AC005230
F-THYRO1001523
   Human DNA sequence *** SEQUENCING IN PROGRESS *** from clone 407F17, WORKING DRAFT SEQUENCE.
   8.8e-21:538:62
   Z83845
F-THYRO1001529
   M.musculus mRNA for serine palmitoyltransferase subunit B.
   5.8e-32:448:66
   X95642
F-THYRO1001593
   Homo sapiens chromosome 19, cosmid R31237, complete sequence.
   5.8e-91:213:98
   AC005581
F-THYRO1001608
   Homo sapiens T-cell receptor alpha delta locus from bases 1000498 to 1071650 (section 5 of 5) of the Complete Nucleotide Sequence.
   0.0028:335:65
   AE000662
F-THYRO1001641
   Leishmania major chromosome 3 clone L6290 strain Friedlin, WORKING DRAFT SEQUENCE, 2 ordered pieces.
   0.92:378:61
   AC005928
F-THYRO1001700
   HS_3220_A1_B08_MR CIT Approved Human Genomic Sperm Library D Homo sapiens genomic clone Plate=3220 Col=15 Row=C, genomic survey sequence.
   1.0e-49:265:96
   AQ184388
F-THYRO1001702
   Mus musculus mRNA for myeloid associated differentiation protein.
   1.4e-70:502:82
   AJ001616
F-THYRO1001725
   F.rubripes GSS sequence, clone 133B16aE1, genomic survey sequence.
   3.8e-06:249:65
   AL004967
F-THYRO1001770
   S.cerevisiae chromosome II reading frame ORF YBR059c.
   1.5e-07:320:62
   Z35928
F-THYRO1001803
   Homo sapiens chromosome 10 clone CRI-JC2019 map 10q22.1-10q22.2, WORKING DRAFT SEQUENCE, 1 ordered pieces.
   1.2e-38:234:94
   AC006108
F-Y79AA1000030
   Homo sapiens chromosome 5, BAC clone 319C17 (LBNL H159), complete sequence.
   9.9e-92:389:98
   AC005214
F-Y79AA1000127
   Homo sapiens genomic DNA, chromosome 21q11.1, segment 5/28, WORKING DRAFT SEQUENCE.
   9.2e-131:359:100
   AP000034
F-Y79AA1000207
   Homo sapiens chromosome 17, clone hRPK.271_K_11, complete sequence.
   2.2e-151:302:98
   AC005562
F-Y79AA1000226
   Drosophila melanogaster, chromosome 2L, region 21C5-21D1, P1 clone DS07610, complete sequence.
   1.1e-50:549:67
   AC004573
F-Y79AA1000270
   Bos taurus vacuolar H+ ATPase subunit Ac45 mRNA, complete cds.
   6.4e-111:771:83
   U10039
F-Y79AA1000426
   Mus musculus activin beta E subunit mRNA, complete cds.
   2.4e-87:703:76
   U96386
F-Y79AA1000521
   Homo sapiens LERK-6 (EPLG6) gene, exon 1.
   0.0092:148:68
   U92893
F-Y79AA1000750
   Homo sapiens Chromosome 16 BAC clone CIT987SK-A-761H5, complete sequence.
   9.0e-07:143:74
   AC002544
F-Y79AA1000776
F-Y79AA1000777
   Podospora anserina beta transducin-like protein (het-e1) gene, complete cds.
   6.6e-17:760:59
   L28125
F-Y79AA1000876
   Homo sapiens long form transcription factor C-MAF (c-maf) mRNA, complete cds.
   3.3e-10:323:66
   AF055377
F-Y79AA1000888
   Streptomyces coelicolor cosmid 8A6.
   3.1e-06:665:59
   AL031013
F-Y79AA1000959
   Homo sapiens Hsp70 binding protein HspBP1 mRNA, complete cds.
   1.6e-52:277:96
   AF093420
F-Y79AA1000967
   Rattus norvegicus vesicla-associate calmodulin-binding protein mRNA, complete cds.
   2.9e-131:752:86
   L22557
F-Y79AA1001013
F-Y79AA1001056
   Mus musculus maternal transcript Maid (Maid) mRNA, complete cds.
   1.2e-85:676:79
   U50734
F-Y79AA1001062
   Human Chromosome 11 Cosmid cSRL34e5, complete sequence.
   8.6e-17:293:65
   U73643
F-Y79AA1001090
   Homo sapiens chromosome 17, clone hCIT.175_E_5, complete sequence.
   1.9e-05:223:63
   AC004596
F-Y79AA1001212
   Homo sapiens SL15 protein mRNA, complete cds.
   4.7e-162:763:98
   AF038961
F-Y79AA1001264
   Drosophila melanogaster DNA sequence (P1s DS00764 (D273) and DS00501 (D274)), complete sequence.
   1.2e-32:599:63
   AC005269
F-Y79AA1001272
   Homo sapiens *** SEQUENCING IN PROGRESS *** from cosmid 5L5, WORKING DRAFT SEQUENCE.
   1.2e-11:356:67
   AJ009613
F-Y79AA1001328
   Rattus norvegicus Delta 3 mRNA, complete cds.
   2.1e-51:443:76
   AF084576
F-Y79AA1001426
   HS_3146_A1_A10_T7 CIT Approved Human Genomic Sperm Library D Homo sapiens genomic clone Plate=3146 Col=19 Row=A, genomic survey sequence.
   9.0e-23:106:91
   AQ141090
F-Y79AA1001427
   Bovine cytochrome b5 reductase mRNA, partial cds.
   1.4e-55:670:70
   M83104
F-Y79AA1001430
   Homo sapiens mRNA for KIAA0469 protein, complete cds.
   8.6e-123:577:99
   AB007938
F-Y79AA1001523
   Homo sapiens transcription intermediary factor 1 (TIF1) mRNA, complete cds.
   3.3e-91:496:93
   AF009353
F-Y79AA1001530
   Human DNA sequence from clone 1189B24 on chromosome Xq25-26.3. Contains NADH-Ubiquinone Oxidoreductase MLRQ subunit (EC 1.6.5.3, EC 1.6.99.3, CI-MLRQ), Tubulin Beta and Proto-oncogene Tyrosine-protein Kinase FER (EC 2.7.1.112, P94-FER, C-FER, TYK3) pseudogenes, and part of a novel gene similar to hypothetical proteins S. pombe C22F3.14C and C. elegans C16A3.8. Contains ESTs, an STS and GSSs, complete sequence.
   1.8e-126:764:89
   AL030996
F-Y79AA1001592
   HS_3219_A2_E12_MR CIT Approved Human Genomic Sperm Library D Homo sapiens genomic clone Plate=3219 Col=24 Row=I, genomic survey sequence.
   5.2e-36:234:89
   AQ180547
F-Y79AA1001727
F-Y79AA1001787
   S.pombe chromosome III cosmid *c*1672.
   8.8e-11:409:58
   AL031324
F-Y79AA1001793
   Fugu rubripes GSS sequence, clone 027L23aG3, genomic survey sequence.
   0.12:131:70
   AL025355
F-Y79AA1001795
   Human DNA sequence from clone 1033B10 on chromosome 6p21.2-21.31. Contains the BINGS gene, exons 11 to 15 of the BING4 gene, the gene for GalT3 (beta3-Galactosyltransferase), the RPS18 (40S ribosomal protein S18) gene, the SACM2L (suppressor of actin mutation 2, yeast, homolog) gene, a pseudogene similar to TAT-SF1, a Pseudogene similar to zinc finger genes, the RING1 gene, the gene for HKE6 (RING2), the gene for HKE4 (RINGS), the RXRB (Retinoid X receptor beta) gene, the COL11A2 (collagen, type XI, alpha 2) gene, the HLA-DPB2 pseudogene and part of the HLA-DPA3 pseudogene. Contains predicted CpG islands, ESTs, STSs, and GSSs, complete sequence.
   1.2e-140:672:98
   AL031228
F-Y79AA1001799
   S.pombe chromosome I cosmid c8C9.
   0.00031:300:60
   Z99168
F-Y79AA1001803
   Mus musculus secretogranin III (SgIII) mRNA, complete cds.
   4.6e-101:516:82
   U02982
F-Y79AA1001863
   Homo sapiens DNA, anonymous heat-stable fragment RP5-6A.
   5.2e-85:410:99
   AB012170
F-Y79AA1002022
   CIT-HSP-2053H1.TF CIT-HSP Homo sapiens genomic clone 2053H1, genomic survey sequence.
   4.3e-20:130:95
   B68526
F-Y79AA1002058
   Homo sapiens clone 24733 mRNA sequence.
   5.3e-153:740:98
   AF052149
F-Y79AA1002121
F-Y79AA1002129
   Bovine herpesvirus type 1 early-intermediate transcription control protein (BICP4) gene, complete cds.
   5.5e-12:565:61
   L14320
F-Y79AA1002213
   Rattus norvegicus brain specific Na+dependent inorganic phosphate cotransporter mRNA, complete cds.
   4.0e-12:434:60
   U07609
F-Y79AA1002334
   F.rubripes GSS sequence, clone 174E24aB10, genomic survey sequence.
   3.0e-10:171:72
   AL019366
F-Y79AA1002373
   Rattus norvegicus Smad8 mRNA, complete cds.
   0.96:420:61
   AF012347
F-Y79AA1002376
   Rattus norvegicus cytoplasmic dynein intermediate chain 2B mRNA, complete cds.
   1.1e-132:805:88
   U39045
F-Y79AA1002378
   Mus musculus mRNA for zinc finger protein, complete cds, clone:CTfin51.
   1.9e-64:521:78
   D10630
F-Y79AA1002381
   O.sativa mRNA for cdc2+/CDC28-related protein kinase.
   3.3e-21:431:60
   X58194

### Homology search result 7

The result of the homology search in the GenBank(http://www.ncbi.nlm.nih.gov/web/GenBank/) using the clone sequences of the 3'-ends. except EST and STS sequences.
Indicated are from the top,
the name of the clone sequence,
definition of the top hit data,
the P-value: the length of the sequence used for comparison (nucleotide):similarity (%),
the Accession No. of the top hit data.

Blank indicates that the 3'-end sequence corresponding to the 5'-end was not determined in the clone. Data were not shown for the clones in which the P-value was higher than 1.
R-HEMBA1000006
R-HEMBA1000121
   Human DNA sequence *** SEQUENCING IN PROGRESS *** from clone 796F18, WORKING DRAFT SEQUENCE.
   2.2e-43:355:80
   AL031291
R-HEMBA1000128
   Homo sapiens chromosome X, PAC 671D9, complete sequence.
   0.99:389:60
   AF031078
R-HEMBA1000275
   Homo sapiens DNA sequence from PAC 958B3 on chromosome Xp22.11-Xp22.22. Contains ESTs STS and CpG island.
   3.4e-10:212:66
   Z93023
R-HEMBA1000300
   {Alu RNA transcript, clone NE461} [human, embryonal carcinoma cells, NTera2D1 pluripotential cells, Other RNA, 282 nt].
   4.6e-42:246:89
   S42653
R-nnnnnnnnnnnn
   Homo sapiens chromosome 17, clone hRPK.235_I_10, complete sequence.
   1.0e-71:192:95
   AC005922
R-HEMBA1000462
   Homo sapiens clone 243 unknown mRNA, complete sequence.
   8.3e-90:313:94
   AF091094
R-HEMBA1000477
   Homo sapiens genomic DNA of 8p21.3-p22 anti-oncogene of hepatocellular colorectal and non-small cell lung cancer, segment 8/11.
   0.22:377:60
   AB020865
R-HEMBA1000590
   Homo sapiens mRNA for matrilin-4, partial.
   8.0e-101:547:93
   AJ007581
R-HEMBA1000634
   *** SEQUENCING IN PROGRESS *** Homo sapiens chromosome 4, BAC clone C0539L10; HTGS phase 1, WORKING DRAFT SEQUENCE, 15 unordered pieces.
   0.95:186:62
   AC004480
R-HEMBA1000671
   RPCI11-65E1.TJ RPCI11 Homo sapiens genomic clone R-65E1, genomic survey sequence.
   2.1e-09:165:73
   AQ237194
R-HEMBA1000713
   Homo sapiens 10kD protein (BC10) mRNA, complete cds.
   1.2e-117:575:97
   AF053470
R-HEMBA1000732
   Homo sapiens mRNA for latent transforming growth factor-beta binding protein-4.
   1.4e-108:581:93
   Y13622
R-nnnnnnnnnnnn
R-HEMBA1000875
   Homo sapiens chromosome 17, clone hRPK.1090_M_7, complete sequence.
   0.044:253:64
   AC005274
R-HEMBA1000940
   ***ALU WARNING: Human Alu-J subfamily consensus sequence.
   1.9e-33:222:82
   U14567
R-HEMBA1000962
R-HEMBA1001184
   Plasmodium falciparum 3D7 chromosome 12 PFYAC69 genomic sequence, WORKING DRAFT SEQUENCE, 4 unordered pieces.
   0.00044:466:58
   AC004688
R-HEMBA1001221
   Sequence 1 from patent US 5633147.
   7.1e-11:232:65
   I43819
R-HEMBA1001228
   Human germline oligomeric matrix protein (COMP) mRNA, complete cds.
   7.8e-89:358:96
   L32137
R-HEMBA1001272
   nbxb0003bDO1r CUGI Rice BAC Library Oryza sativa genomic clone nbxb0003G00r, genomic survey sequence.
   0.00014:201:64
   AQ050116
R-HEMBA1001296
   Homo sapiens PAC clone DJ1168D11 from 7p21-p22, complete sequence.
   0.13:440:58
   AC004614
R-HEMBA1001297
   Homo sapiens putative transcription factor CA150 mRNA, complete cds.
   5.0e-92:466:96
   AF017789
R-HEMBA1001390
   CIT-HSP-2314K10.TR CIT-HSP Homo sapiens genomic clone 2314K10, genomic survey sequence.
   3.4e-43:196:85
   AQ027191
R-HEMBA1001563
   H.sapiens villin gene, exon 1.
   2.1e-43:342:81
   X71058
R-HEMBA1001621
   Human G protein-coupled receptor APJ gene, complete cds.
   1.2e-41:288:87
   U03642
R-HEMBA1001878
   Homo sapiens U5 snRNP-specific 40 kDa protein mRNA, complete cds.
   2.0e-79:434:93
   AF090988
R-HEMBA1001886
   Human zinc finger protein (ZNF141) mRNA, complete cds.
   1.8e-59:530:80
   L15309
R-HEMBA1002048
   Homo sapiens chromosome 5, P1 clone 1307e8 (LBNL H60), complete sequence.
   0.36:322:61
   AC005355
R-HEMBA1002131
   Human DNA sequence from clone 301K23 on chromosome 1p35.1-36.21. Contains the 5' part of a novel gene similar to predicted yeast and worm genes. Contains ESTs and GSSs, complete sequence.
   0.22:233:61
   AL031730
R-HEMBA1002163
   Homo sapiens chromosome X, clone 592, WORKING DRAFT SEQUENCE, 8 unordered pieces.
   1.1e-16:275:69
   AC002489
R-HEMBA1002167
   Rattus norvegicus neuroligin I mRNA, complete cds.
   8.7e-23:193:84
   U22952
R-HEMBA1002178
R-HEMBA1002195
   Homo sapiens DHPS gene, exons 8 to 9.
   1.4e-19:114:100
   AJ001704
R-HEMBA1002227
   Homo sapiens mRNA for 80K-L protein, complete cds.
   6.1e-115:567:97
   D10522
R-HEMBA1002316
   Homo sapiens mRNA for putative GTP-binding protein.
   1.5e-18:161:85
   Y14391
R-HEMBA1002420
   Caenorhabditis elegans cosmid C27A7, complete sequence.
   0.88:214:62
   Z81041
R-HEMBA1002421
   Human heparan sulfate proteoglycan (HSPG) core protein, 3' end.
   6.0e-90:443:97
   J04621
R-HEMBA1002524
   Human MHC Class I region proline rich protein mRNA, complete cds.
   3.2e-110:551:96
   U63336
R-HEMBA1002551
   Mouse Bac 276o8, WORKING DRAFT SEQUENCE, 25 unordered pieces.
   7.0e-06:397:61
   AC003022
R-HEMBA1002767
   Homo sapiens clone NH0486I22, WORKING DRAFT SEQUENCE, 5 unordered pieces.
   4.2e-110:568:96
   AC005038
R-HEMBA1002985
   Homo sapiens chromosome 17, clone hRPK.15_K_2, complete sequence.
   3.4e-23:184:86
   AC005901
R-HEMBA1003047
   Homo sapiens intrinsic factor-B12 receptor precursor, mRNA, complete cds.
   5.0e-114:571:96
   AF034611
R-HEMBA1003072
   HS-1014-B1-F12-MR.abi CIT Human Genomic Sperm Library C Homo sapiens genomic clone Plate=CT 789 Col=23 Row=L, genomic survey sequence.
   1.5e-62:340:94
   B32084
R-HEMBA1003101
   Homo sapiens tyrosylprotein sulfotransferase-2 mRNA, complete cds.
   3.8e-116:575:97
   AF049891
R-HEMBA1003120
   HS_3220_A1_F04_T7 CIT Approved Human Genomic Sperm Library D Homo sapiens genomic clone Plate=3220 Col=7 Row=K, genomic survey sequence.
   3.6e-61:354:92
   AQ184345
R-HEMBA1003230
   Homo sapiens UP50 mRNA, complete cds.
   1.3e-42:258:93
   AF093118
R-HEMBA1003294
   HS_3220_A1_D03_T7 CIT Approved Human Genomic Sperm Library D Homo sapiens genomic clone Plate=3220 Col=5 Row=G, genomic survey sequence.
   0.0095:204:63
   AQ190655
R-HEMBA1003315
   Sus scrofa DNA for LH beta, exons 1, 2, 3, complete cds.
   6.6e-24:163:79
   D00579
R-HEMBA1003392
   Homo sapiens LDL receptor-related protein 6 (LRP6) mRNA, complete cds.
   2.6e-115:557:98
   AF074264
R-HEMBA1003399
   Homo sapiens clone DJ1125K23, WORKING DRAFT SEQUENCE, 21 unordered pieces.
   1.8e-63:166:100
   AC004971
R-HEMBA1003487
R-HEMBA1003497
   Human DNA sequence *** SEQUENCING IN PROGRESS *** from clone 27O5, WORKING DRAFT SEQUENCE.
   1.4e-119:592:97
   AL033529
R-HEMBA1003530
R-HEMBA1003602
   Human Chromosome 16 BAC clone CIT987SK-A-17E1, complete sequence.
   9.4e-79:468:91
   AC002041
R-HEMBA1003732
   Homo sapiens clone DJ0935K16, complete sequence.
   2.0e-118:586:98
   AC006011
R-HEMBA1003945
   Human calcineurin B mRNA, complete cds.
   8.9e-82:410:97
   M30773
R-HEMBA1004007
   Homo sapiens clone DJ0665P05, WORKING DRAFT SEQUENCE, 5 unordered pieces.
   6.7e-56:404:75
   AC004851
R-HEMBA1004085
   G.gallus microsatellite DNA (LEI0311 (= EC12A05)).
   0.66:144:65
   Z95196
R-nnnnnnnnnnnn
   Homo sapiens intersectin short form mRNA, complete cds.
   2.1e-115:569:97
   AF064243
R-HEMBA1004250
   Homo sapiens chromosome 5, BAC clone 182a8 (LBNL H161), complete sequence.
   3.8e-98:478:98
   AC005752
R-HEMBA1004391
   Plasmodium falciparum MAL3P8, complete sequence.
   0.29:126:65
   AL034560
R-HEMBA1004444
   Homo sapiens clone DJ0971C03, WORKING DRAFT SEQUENCE, 18 unordered pieces.
   8.4e-52:308:78
   AC004938
R-HEMBA1004454
   CIT-HSP-2337122.TF CIT-HSP Homo sapiens genomic clone 2337I22, genomic survey sequence.
   0.78:59:77
   AQ038475
R-HEMBA1004505
R-HEMBA1004785
R-HEMBA1004797
R-HEMBA1004952
   Mus musculus diabetic embryopathy (Dep-1) mRNA.
   3.4e-39:327:82
   AF032130
R-HEMBA1004971
   Homo sapiens BAC clone RG351J01 from 7q22-q31, complete sequence.
   0.00040:251:66
   AC005099
R-HEMBA1004982
   Strongyloides fulleborni 18S ribosomal RNA and 5.8S ribosomal RNA genes, partial sequence, and internal transcribed spacer 1, complete sequence.
   0.092:191:63
   U43581
R-HEMBA1005070
   Human mRNA for KIAA0310 gene, complete cds.
   1.2e-94:381:91
   AB002308
R-HEMBA1005084
R-HEMBA1005145
   Homo sapiens complete genomic sequence between D16S3070 and D16S3275, containing Familial Mediterranean Fever gene disease.
   5.7e-58:283:84
   AJ003147
R-HEMBA1005230
   CIT-HSP-2333N15.TR CIT-HSP Homo sapiens genomic clone 2333N15, genomic survey sequence.
   5.5e-31:363:73
   AQ040189
R-HEMBA1005246
   Homo sapiens full length insert cDNA clone YX52E07.
   1.6e-11:173:72
   AF086040
R-HEMBA1005267
   Sequence 1 from patent US 5618695.
   2.4e-73:536:81
   I40055
R-HEMBA1005337
   Caenorhabditis elegans cosmid K07D4.
   0.16:157:63
   AF077534
R-HEMBA1005430
R-HEMBA1005449
R-HEMBA1005489
   Anopheles rangeli NADH dehydrogenase subunit 2 gene, mitochondrial gene encoding mitochondrial product, partial cds.
   0.020:271:61
   U35272
R-HEMBA1005522
R-HEMBA1005545
   Human m3 muscarinic acetylcholine receptor (CHRM3) gene, complete cds.
   1.8e-115:579:96
   U29589
R-HEMBA1005698
   0.0065:223:65
   AG004952
R-HEMBA1005913
   Homo sapiens Xp22 BAC GSHB 526D21 (Genome Systems Human BAC library) complete sequence.
   3.7e-15:272:68
   AC003037
R-HEMBA1005929
   Homo sapiens chromosome 19, cosmid R31237, complete sequence.
   9.4e-55:502:76
   AC005581
R-HEMBA1005945
   Human DNA sequence *** SEQUENCING IN PROGRESS *** from clone 774G10, WORKING DRAFT SEQUENCE.
   0.45:245:62
   AL034410
R-HEMBA1006016
   Homo sapiens chromosome 17, clone hRPK.271_K_11, complete sequence.
   3.5e-25:415:66
   AC005562
R-HEMBA1006171
   Human DNA sequence from PAC 433M19 on chromosome Xq26.3-Xq27.1. Contains ESTs, STSs and polymorphic CA repeat.
   1.0:176:64
   Z95703
R-HEMBA1006276
   Homo sapiens chromosome 19, CIT-HSP-444n24, complete sequence.
   2.8e-118:592:96
   AC005261
R-HEMBA1006299
R-HEMBA1006311
R-HEMBA1006335
   Human DNA sequence from clone 496H19 on chromosome 6q24 Contains ESTs, complete sequence.
   6.1e-111:578:96
   AL023582
R-HEMBA1006357
   Homo sapiens chromosome Xp22-67-68, WORKING DRAFT SEQUENCE, 99 unordered pieces.
   4.8e-11:174:74
   AC004469
R-HEMBA1006430
   Homo sapiens, WORKING DRAFT SEQUENCE, 52 unordered pieces.
   8.7e-45:402:79
   AC004086
R-HEMBA1006482
   Homo sapiens h-sco1 (SCO1) mRNA, nuclear gene encoding mitochondrial protein, complete cds.
   1.7e-105:537:96
   AF026852
R-HEMBA1006517
   345A19.TV CIT978SKA1 Homo sapiens genomic clone A-345A19, genomic survey sequence.
   1.5e-44:176:88
   B15409
R-HEMBA1006544
   Homo sapiens PAC clone DJ130H16 from 22q12.1-qter, complete sequence.
   2.5e-66:310:83
   AC004997
R-HEMBA1006572
   Homo sapiens reduced folate carrier (RFC1) gene, exons 1a, 1c and 1b.
   0.028:255:64
   U92868
R-HEMBA1006658
   Homo sapiens mRNA for KIAA0687 protein, partial cds.
   7.3e-1 11:570:94
   AB014587
R-HEMBA1006707
   Homo sapiens mRNA for matrilin-4, partial.
   5.1e-78:389:97
   AJ007581
R-HEMBA1006724
   HS_2052_B1_C08_MR CIT Approved Human Genomic Sperm Library D Homo sapiens genomic clone Plate=2052 Col=15 Row=F, genomic survey sequence.
   2.6e-46:309:88
   AQ305998
R-HEMBA1006749
   Homo sapiens mRNA for matrilin-4, partial.
   3.2e-88:472:94
   AJ007581
R-HEMBA1006770
   Homo sapiens CAGH4 mRNA, partial cds.
   6.5e-25:145:82
   U80746
R-HEMBA1006902
   Homo sapiens mRNA for matrilin-4, partial.
   9.3e-112:540:98
   AJ007581
R-HEMBA1006912
   ***ALU WARNING: Human Alu-Sc subfamily consensus sequence.
   6.6e-48:279:92
   U14571
R-HEMBA1006916
   Homo sapiens Grb14 mRNA, complete cds.
   1.8e-114:346:99
   L76687
R-HEMBA1006960
   Homo sapiens chromosome 19, cosmid F16403, complete sequence.
   0.78:203:62
   AC005777
R-HEMBA1007013
   Human mRNA for DNA-binding protein TAXREB302, complete cds.
   6.3e-31:163:100
   D28468
R-HEMBA1007057
   CIT-HSP-517F5.TP CIT-HSP Homo sapiens genomic clone 517F5, genomic survey sequence.
   1.0:128:67
   B49904
R-HEMBA1007063
   Homo sapiens DNA sequence from PAC 168L15 on chromosome 6q26-27. Contains RSK3 gene, ribosomal protein S6 kinase, EST, GSS, STS. CpG island, complete sequence.
   5.0e-43:300:88
   AL022069
R-HEMBA1007241
   HIV-1 RNA V3 region (patient Y, sample Y1, clone 05).
   0.74:148:66
   Z47529
R-HEMBA1007291
   Homo sapiens chromosome 19, fosmid 37502, complete sequence.
   3.6e-36:300:80
   AC004755
R-HEMBA1007332
   Human HeLa mRNA isolated as a false positive in a two-hybrid-screen.
   7.3e-15:148:80
   U56430
R-HEMBB1000106
   Plasmodium falciparum (strain FCR3) variant-specific surface protein (var-2, var-3) genes, complete cds's.
   8.0e-05:313:60
   L40609
R-HEMBB1000276
   HS_3048_A2_C07_MR CIT Approved Human Genomic Sperm Library D Homo sapiens genomic clone Plate=3048 Col=14 Row=E, genomic survey sequence.
   0.91:234:58
   AQ099411
R-HEMBB1000309
R-HEMBB1000407
   Mus musculus clone OST5976, genomic survey sequence.
   6.4e-28:226:81
   AF046768
R-HEMBB1000447
   Homo sapiens JWA protein mRNA, complete cds.
   1.7e-107:533:97
   AF070523
R-HEMBB1000542
   Mus musculus bromodomain-containing protein BP75 mRNA, complete cds.
   4.4e-72:547:80
   AF084259
R-HEMBB1000567
   Human insulin-like growth factor (IGF-II) gene, exon 1 of 4.
   4.3e-60:368:88
   M13970
R-HEMBB1000642
   Human DNA sequence from PAC 46H23, BRCA2 gene region chromosome 13q12-13 contains Klotho, ESTs.
   2.9e-42:431:75
   Z84483
R-HEMBB1000668
   CITBI-E1-2508D15.TR CITBI-E1 Homo sapiens genomic clone 2508D15, genomic survey sequence.
   2.5e-40:249:91
   AQ261535
R-HEMBB1000679
   HS_3061_A1_C03_T7 CIT Approved Human Genomic Sperm Library D Homo sapiens genomic clone Plate=3061 Col=5 Row=E, genomic survey sequence.
   1.8e-48:257:96
   AQ127602
R-HEMBB1000881
   CIT-HSP-2350020.TR CIT-HSP Homo sapiens genomic clone 2350O20, genomic survey sequence.
   0.0072:248:61
   AQ062620
R-HEMBB1000905
   Homo sapiens clone RG315H11, WORKING DRAFT SEQUENCE, 5 unordered pieces.
   2.5e-104:547:94
   AC005089
R-HEMBB1001026
R-HEMBB1001048
R-HEMBB1001200
   P.falciparum complete gene map of plastid-like DNA (IR-A).
   1.5e-11:521:59
   X95275
R-HEMBB1001407
   Human DNA sequence *** SEQUENCING IN PROGRESS *** from clone 2705, WORKING DRAFT SEQUENCE.
   3.0e-29:308:77
   AL033529
R-HEMBB1001530
   Homo sapiens chromosome 19, cosmid R30538, complete sequence.
   0.040:373:63
   AC005943
R-HEMBB1001547
   Rickettsia prowazekii strain Madrid E, complete genome; segment 1/4.
   0.027:291:62
   AJ235270
R-HEMBB1001573
   CIT-HSP-2307C1.TR CIT-HSP Homo sapiens genomic clone 2307C1, genomic survey sequence.
   1.3e-13:90:98
   AQ020395
R-HEMBB1001847
   Homo sapiens chromosome 21q22.3 PAC 21L13, complete sequence.
   3.4e-27:147:80
   AF064864
R-HEMBB1001959
   Homo sapiens clone 24781 mRNA sequence.
   4.4e-103:504:97
   AF070640
R-HEMBB1001978
   CIT-HSP-2328G6.TF CIT-HSP Homo sapiens genomic clone 2328G6, genomic survey sequence.
   7.9e-29:220:86
   AQ040310
R-HEMBB1002039
   Homo sapiens BAC clone GS166A23 from 7p21, complete sequence.
   2.7e-37:550:68
   AC005014
R-HEMBB1002041
   Sequence 1 from patent US 5633147.
   2.7e-23:322:70
   I43819
R-HEMBB1002051
   Homo sapiens clone DJ0292L20, WORKING DRAFT SEQUENCE, 2 unordered pieces.
   9.2e-35:302:79
   AC004825
R-HEMBB1002120
   Homo sapiens Xp22 GS-524I1 (Genome Systems Human BAC library), complete sequence.
   6.0e-05:479:59
   AC003106
R-HEMBB1002162
   Human DNA sequence from clone 739H11 on chromosome 1p33-34.2 Contains KIAA0237 gene, EST, STS, GSS, complete sequence.
   3.7e-30:238:84
   AL031289
R-HEMBB1002228
   Homo sapiens BAC clone NH0436H22 from 2, complete sequence.
   6.6e-57:274:86
   AC005234
R-HEMBB1002245
   Sequence 25 from patent US 5747660.
   4.8e-30:361:73
   AR005295
R-HEMBB1002302
   Methanococcus jannaschii section 84 of 150 of the complete genome.
   0.00019:362:59
   U67542
R-HEMBB1002427
   Genomic sequence from Human 9q34, complete sequence.
   3.9e-105:533:96
   AC001643
R-HEMBB1002465
   Plasmodium falciparum chromosome 2, section 19 of 73 of the complete sequence.
   2.9e-05:335:62
   AE001382
R-HEMBB1002661
R-HEMBB1002663
   ***ALU WARNING: Human Alu-Sq subfamily consensus sequence.
   8.3e-43:268:89
   U14573
R-HEMBB1002693
   Human BAC clone RG126M09 from 7q21-q22, complete sequence.
   2.4e-24:220:76
   AC002067
R-MAMMA1000046
   Plasmodium falciparum 3D7 chromosome 12 PFYAC181 genomic sequence, WORKING DRAFT SEQUENCE, 8 unordered pieces.
   0.032:402:57
   AC005505
R-MAMMA1000102
   Human DNA sequence from cosmid B33F2 on chromosome 22 Contains ESTs.
   2.0e-84:428:96
   Z79996
R-MAMMA1000106
   Homo sapiens genomic DNA, chromosome 21q11.1, segment 2/28, WORKING DRAFT SEQUENCE.
   0.095:138:66
   AP000031
R-MAMMA1000118
R-MAMMA1000141
   Homo sapiens 12q24.2 PAC RPCI1-128M12 (Roswell Park Cancer Institute Human PAC library) complete sequence.
   9.0e-91:480:95
   AC004024
R-MAMMA1000204
   Homo sapiens mRNA for LGMD2B protein.
   1.5e-107:544:96
   AJ007670
R-MAMMA1000226
   H.sapiens VASP gene, exons 4 to 13.
   0.99:244:63
   X98534
R-MAMMA1000403
   CIT-HSP-2372A15.TF CIT-HSP Homo sapiens genomic clone 2372A15, genomic survey sequence.
   8.0e-38:187:81
   AQ112406
R-MAMMA1000449
   Homo sapiens BAC clone RG139P11 from 7q11-q21, complete sequence.
   1.2e-41:422:76
   AC004491
R-MAMMA1000457
   Homo sapiens clone 638 unknown mRNA, complete sequence.
   7.4e-116:570:97
   AF091084
R-MAMMA1000473
   Homo sapiens Chromosome 16 BAC clone CIT987SK-A-69G12, complete sequence.
   9.6e-09:136:77
   AC004131
R-MAMMA1000496
   Homo sapiens PAC clone DJ130H16 from 22q12.1-qter, complete sequence.
   2.6e-48:272:93
   AC004997
R-MAMMA1000528
   Human BAC clone RG114A06 from 7q31, complete sequence.
   1.8e-13:109:80
   AC002542
R-MAMMA1000591
   Human cosmid g1572c264, complete sequence.
   1.6e-22:329:71
   AC000359
R-MAMMA1000614
R-MAMMA1000652
   H.sapiens flow-sorted chromosome 6 HindIII fragment, SC6pA28A10.
   0.81:158:65
   Z84499
R-MAMMA1000681
   Homo sapiens full length insert cDNA clone YY85D04.
   1.0e-107:560:94
   AF088014
R-MAMMA1000706
   Homo sapiens cGMP phosphodiesterase A1 (PDE9A) mRNA, complete cds.
   1.1e-46:232:100
   AF067223
R-MAMMA1000788
   Bos taurus P14 (p14) mRNA, complete cds.
   3.8e-72:493:84
   AF037349
R-MAMMA1000810
   Human DNA from overlapping chromosome 19-specific cosmids R29515 and R28253, genomic sequence, complete sequence.
   5.0e-37:318:79
   AC003002
R-MAMMA1000814
   Homo sapiens BAC clone BK085E05 from 22q12.1-qter, complete sequence.
   7.7e-15:140:85
   AC003071
R-MAMMA1000881
   Human DNA sequence from clone 105D16 on chromosome Xp11.3-11.4 Contains pseudogene similar to laminin-binding protein, CA repeat, STS, complete sequence.
   8.8e-46:457:75
   AL031311
R-MAMMA1000986
   Homo sapiens chromosome 16 BAC clone CIT987SK-334D11 complete sequence.
   7.7e-44:343:82
   AF001550
R-MAMMA1000994
   Homo sapiens mRNA for ISLR, complete cds.
   3.6e-108:552:96
   AB003184
R-MAMMA1001043
R-MAMMA1001066
   Homo sapiens chromosome 17, clone hRPK.346_K_10, complete sequence.
   1.3e-42:302:82
   AC006120
R-MAMMA1001094
   Homo sapiens clone 243 unknown mRNA, complete sequence.
   5.4e-115:567:97
   AF091094
R-MAMMA1001141
   HS_3059_B1_H06_MR CIT Approved Human Genomic Sperm Library D Homo sapiens genomic clone Plate=3059 Col=11 Row=P, genomic survey sequence.
   1.3e-68:388:92
   AQ214896
R-MAMMA1001150
   H.sapiens mRNA for protein kinase C mu.
   5.4e-20:340:66
   X75756
R-MAMMA1001237
   Mouse DNA fragment that hybridizes to HSV- 1 SmaI A fragment.
   0.15:222:65
   M11041
R-MAMMA1001284
   Human DNA sequence *** SEQUENCING IN PROGRESS *** from clone 1185N5, WORKING DRAFT SEQUENCE.
   1.2e-33:344:76
   AL034423
R-MAMMA1001310
   Human Bruton agammaglobulinemia (BTK) gene, exons 10-12.
   1.8e-39:332:80
   L31565
R-MAMMA1001418
   Human proto-oncogene tyrosine-protein kinase (ABL) gene, exon 1a and exons 2-10, complete cds.
   4.4e-42:411:76
   U07563
R-MAMMA1001532
   Homo sapiens PAC clone DJ0728D04, complete sequence.
   2.3e-10:196:73
   AC004865
R-MAMMA1001609
   HS-1054-B2-H01-MF.abi CIT Human Genomic Sperm Library C Homo sapiens genomic clone Plate=CT 776 Col=2 Row=P, genomic survey sequence.
   1.6e-34:170:79
   B42016
R-MAMMA1001615
R-MAMMA1001623
   Homo sapiens 12q24.2 BAC RPCI11-407A16 (Roswell Park Cancer Institute Human BAC Library) complete sequence.
   8.8e-21:180:82
   AC006065
R-MAMMA1001634
   Homo sapiens chromosome 17, clone hRPK.85_B_7, complete sequence.
   2.6e-40:283:86
   AC005695
R-MAMMA1001893
   Homo sapiens clone DJ0782K24, WORKING DRAFT SEQUENCE, 16 unordered pieces.
   0.73:132:67
   AC006003
R-MAMMA1001901
   Homo sapiens DNA sequence from PAC 958B3 on chromosome Xp22.11-Xp22.22. Contains ESTs STS and CpG island.
   4.0e-43:288:77
   Z93023
R-MAMMA1001957
   Homo sapiens chromosome 16, P1 clone 96-4B (LANL), complete sequence.
   1.2e-41:298:86
   AC005212
R-MAMMA1001978
R-MAMMA1002070
   Homo sapiens clone DJ400N23, WORKING DRAFT SEQUENCE, 10 unordered pieces.
   2.1e-104:530:97
   AC005003
R-MAMMA1002080 rah=ras-related homolog [mice, HT4 neural cell line, mRNA, 993 nt].
   5.9e-47:449:76
   S72304
R-MAMMA1002087
   Human Cosmid g1572c037 from 7q31.3, complete sequence.
   1.7e-11:120:83
   AC000125
R-MAMMA1002095
   Rat alternatively spliced mRNA.
   5.3e-30:289:74
   M93018
R-MAMMA1002128
R-MAMMA1002142
R-MAMMA1002165
   Homo sapiens chromosome 10 clone CIT987SK-1109P11, complete sequence.
   1.1e-28:350:72
   AC005871
R-MAMMA1002205
   Human DNA sequence from PAC 368A4 on chromosome X. Contains ESTs, CELLULAR NUCLEIC ACID BINDING PROTEIN (CNBP) like gene and STSs.
   1.2e-42:282:75
   Z83843
R-MAMMA1002224
   Arabidopsis thaliana DNA chromosome 4, BAC clone F1C12 (ESSAII project).
   0.99:210:60
   AL022224
R-MAMMA1002234
   Canine mRNA for 68kDA subunit of signal recognition particle (SRP68).
   1.7e-61:310:81
   X53744
R-MAMMA1002586
   Streptomyces collinus coenzyme B12-dependent mutase (meaA) gene, complete cds.
   0.99:348:60
   AF008569
R-MAMMA1002633
   Homo sapiens, clone hRPK. 1_A_1, complete sequence.
   2.6e-13:381:64
   AC006196
R-MAMMA1003126
R-NT2RM4000100
   Plasmodium falciparum MAL3P2, complete sequence.
   0.00047:296:61
   AL034558
R-NT2RM4000115
   Plasmodium falciparum DNA *** SEQUENCING IN PROGRESS *** from MAL1P3, WORKING DRAFT SEQUENCE.
   0.079:270:64
   AL031746
R-NT2RM4000198
   Homo sapiens chromosome 10 clone LA10NC01_15_E_11 map 10q26.3, WORKING DRAFT SEQUENCE, 3 unordered pieces.
   7.7e-24:244:78
   AC006171
R-NT2RM4000284
   Human IgG Fc receptor hFcRn mRNA, complete cds.
   1.7e-93:440:100
   U12255
R-NT2RM4000295
   , complete sequence.
   0.89:351:58
   AC005663
R-NT2RM4000326
   Homo sapiens complete genomic sequence between D16S3070 and D16S3275, containing Familial Mediterranean Fever gene disease.
   2.3e-112:602:94
   AJ003147
R-NT2RM4000417
   Human DNA sequence from PAC 326L13 containing brain-4 mRNA ESTs and polymorphic CA repeat.
   0.78:229:62
   Z82170
R-NT2RM4000444
R-NT2RM4000587
R-NT2RM4000593
R-NT2RM4000648
   0.010:260:61
   AG005508
R-NT2RM4000761
   H.sapiens mitochondrial genome (consensus sequence).
   3.2e-95:476:97
   X62996
R-NT2RM4000965
R-NT2RM4000997
R-NT2RM4001321
   Human DNA sequence from clone 1177E19 on chromosome 1p36.12-36.31. Contains the 3'part of the DNA-binding Zinc finger protein RIZ gene, ESTs, an STS, GSSs and a CpG island, complete sequence.
   6.0e-19:282:73
   AL031277
R-NT2RM4001325
R-NT2RM4001377
   Homo sapiens mRNA for KIAA0638 protein, partial cds.
   2.9e-111:553:96
   AB014538
R-NT2RM4001735
   Homo sapiens clone 23904 mRNA sequence.
   4.6e-106:553:94
   AF052129
R-NT2RM4001768
   Human HepG2 3'region MboI cDNA, clone hmd3c03m3.
   4.1e-29:187:91
   D17194
R-NT2RM4001843
   Homo sapiens chromosome 17, clone hRPK.269_G_24, complete sequence.
   0.95:366:58
   AC005828
R-NT2RM4002352
   Homo sapiens hLRp105 mRNA for LDL receptor related protein 105, complete cds.
   5.5e-108:557:95
   AB009462
R-NT2RP2000092
   HS_3070_B1_B04_MR CIT Approved Human Genomic Sperm Library D Homo sapiens genomic clone Plate=3070 Col=7 Row=D, genomic survey sequence.
   1.1e-23:247:77
   AQ120714
R-NT2RP2000178
   E.amylovora lon gene.
   1.1e-15:422:62
   X77706
R-NT2RP2000240
   *** SEQUENCING IN PROGRESS *** Homo sapiens chromosome 4, BAC clone C0539L10; HTGS phase 1, WORKING DRAFT SEQUENCE, 15 unordered pieces.
   0.00010:260:62
   AC004480
R-NT2RP2000394
   HS_3211_B2_G06_T7 CIT Approved Human Genomic Sperm Library D Homo sapiens genomic clone Plate=3211 Col=12 Row=N, genomic survey sequence.
   1.1e-61:316:97
   AQ174850
R-NT2RP2000447
   Homo sapiens clone DJ1129D05, complete sequence.
   8.7e-67:357:94
   AC005630
R-NT2RP2000479
   Homo sapiens chromosome 17, clone 193h18, complete sequence.
   5.7e-51:551:73
   AC002546
R-NT2RP2000514
   P.falciparum parasite antigen reactive with the parasite inhibitory mouse monoclonal antibody (mMAb) 43E5, clone #366, partial cds.
   2.1e-08:192:68
   M21323
R-NT2RP2000533
   Mus musculus cornichon mRNA, complete cds.
   3.5e-59:243:82
   AF022811
R-NT2RP2000616
   Human DNA sequence from clone 694E4 on chromosome 22 Contains exon similar to phosphatidylserine decarboxylase, EST, GSS, complete sequence.
   0.0064:105:67
   AL031255
R-NT2RP2000649
   Homo sapiens mRNA for Hs Ste24p, complete cds.
   1.4e-65:326:98
   AB016068
R-NT2RP2000663
   Human DNA sequence from cosmid U61B11, between markers DXS366 and DXS87 on chromosome X contains ESTs.
   7.9e-110:555:96
   Z73913
R-NT2RP2000712
   Homo sapiens clone DJ0756H11, WORKING DRAFT SEQUENCE, 5 unordered pieces.
   9.8e-32:308:78
   AC006001
R-NT2RP2000739
   Bos taurus TATA box binding protein (TBP) gene, partial cds.
   0.19:128:68
   L47974
R-NT2RP2000818
   Caenorhabditis elegans cosmid C48D5, complete sequence.
   0.010:429:58
   Z36237
R-NT2RP2000903
   H.sapiens 5T4 gene for 5T4 Oncofetal antigen.
   4.0e-99:505:96
   Z29083
R-NT2RP2001200
   Homo sapiens mRNA for KIAA0676 protein, partial cds.
   2.0e-57:306:95
   AB014576
R-NT2RP2001223
R-NT2RP2001276
   Mouse regulatory protein (npdc-1) mRNA, complete cds.
   5.8e-14:353:65
   L03814
R-NT2RP2001388
   Homo sapiens clone DJ1125K23, WORKING DRAFT SEQUENCE, 21 unordered pieces.
   1.7e-31:291:77
   AC004971
R-NT2RP2001469
   M.musculus tex292 mRNA (3'region).
   3.7e-26:188:89
   X80433
R-NT2RP2001480
   Homo sapiens thrombospondin 3 (THBS3) gene, complete cds.
   6.6e-83:426:95
   L38969
R-NT2RP2001495
   transcript ch123.Rev [human, RF1,RF48 stomach cancer cell lines, mRNA, 252 nt].
   6.3e-43:238:96
   S77359
R-NT2RP2001514
   Homo sapiens cyclin K (CPR4) mRNA, complete cds.
   6.6e-06:57:100
   AF060515
R-NT2RP2001538
   Mus musculus transcriptional regulatory protein (mSin3) gene, complete cds.
   6.9e-12:179:75
   L36831
R-NT2RP2001562
   Human PAC clone DJ0800B09 from 7q11.23-q21, complete sequence.
   0.074:257:61
   AC004028
R-NT2RP2001662
   Homo sapiens clone 24615 mRNA sequence.
   3.2e-94:485:95
   AF055012
R-NT2RP2001755
   Homo sapiens mRNA for KIAA0762 protein, partial cds.
   1.3e-103:576:92
   AB018305
R-NT2RP2001769
   CIT-HSP-2376O23.TF CIT-HSP Homo sapiens genomic clone 2376023, genomic survey sequence.
   1.5e-74:381:96
   AQ111163
R-NT2RP2001817
   HS_2037_B2_A09_T7 CIT Approved Human Genomic Sperm Library D Homo sapiens genomic clone Plate=2037 Col=18 Row=B, genomic survey sequence.
   3.9e-60:430:84
   AQ243047
R-NT2RP2001878
   Human DNA sequence from PAC 296K21 on chromosome X contains cytokeratin exon, delta-aminolevulinate synthase (erythroid); 5-aminolevulinic acid synthase.(EC 2.3.1.37). 6-phosphofructo-2-kinase/fructose-2,6-bisphosphatase (EC 2.7.1.105, EC 3.1.3.46), ESTs and STS.
   0.018:148:67
   Z83821
R-NT2RP2001903
   Human Not1 linking clone from chromosome 1q32.
   0.99:160:63
   U36769
R-NT2RP2001915
R-NT2RP2001921
   Homo sapiens clone NH0332L11, complete sequence.
   6.5e-86:295:98
   AC005538
R-NT2RP2001948
   Homo sapiens chromosome 19, cosmid R33590, complete sequence.
   2.3e-79:440:91
   AC005620
R-NT2RP2001956
R-NT2RP2002015
   Human DNA sequence from clone 1177E19 on chromosome 1p36.12-36.31. Contains the 3'part of the DNA-binding Zinc finger protein RIZ gene, ESTs, an STS, GSSs and a CpG island, complete sequence.
   1.1e-16:254:72
   AL031277
R-NT2RP2002063
   Homo sapiens chromosome 4 clone B207D4 map 4q25, complete sequence.
   5.8e-105:550:95
   AC004050
R-NT2RP2002188
   Human DNA sequence *** SEQUENCING IN PROGRESS *** from clone 134019, WORKING DRAFT SEQUENCE.
   0.47:212:62
   AL034555
R-NT2RP2002232
R-nnnnnnnnnnnn
   Human mRNA for KIAA0383 gene, partial cds.
   2.5e-100:511:96
   AB002381
R-NT2RP2002409
   S.pombe chromosome I cosmid c17H9.
   1.0:241:63
   Z98597
R-NT2RP2002510
   Homo sapiens chromosome 19, cosmid F19847, complete sequence.
   1.6e-38:307:81
   AC005952
R-NT2RP2002527
   Homo sapiens chromosome 11, BAC CIT-HSP-311e8 (BC269730) containing the hFEN1 gene, complete sequence.
   1.5e-18:165:83
   AC004770
R-NT2RP2002533
   Homo sapiens alpha 2 delta calcium channel subunit isoform II mRNA, complete cds.
   9.7e-116:580:96
   AF042793
R-NT2RP2002564
   Homo sapiens clone DJ0800G07, complete sequence.
   3.8e-110:580:94
   AC004890
R-NT2RP2002674
   Plasmodium falciparum chromosome 2, section 11 of 73 of the complete sequence.
   1.0:244:60
   AE001374
R-NT2RP2002721
   Homo sapiens chromosome 17, clone HCIT217L10, complete sequence.
   1.2e-10:221:73
   AC003962
R-NT2RP2002824
   Human HepG2 3' region MboI cDNA, clone hmd4f06m3.
   3.0e-07:108:77
   D17237
R-NT2RP2002942
   Human DNA sequence from clone 88D7 on chromosome Xq25-26.3 Contains F9 (coagulation factor IX (plasma thromboplastic component, Christmas disease, haemophilia B)), dbl oncogene. EST, STS, GSS, complete sequence.
   2.0e-37:491:71
   AL033403
R-NT2RP2002974
   H.sapiens DMAHP gene.
   4.0e-118:585:97
   X84813
R-NT2RP2002976
   CIT-HSP-2348J20.TF CIT-HSP Homo sapiens genomic clone 2348J20, genomic survey sequence.
   8.4e-45:233:98
   AQ059444
R-NT2RP2003042
R-NT2RP2003179
R-NT2RP2003210
R-NT2RP2003302
   Human DNA sequence from 4PTEL, Huntington's Disease Region, chromosome 4p16.3.
   1.5e-24:255:78
   Z95704
R-NT2RP2003369
   Homo sapiens chromosome 7q22 sequence, complete sequence.
   3.1e-95:514:92
   AF053356
R-NT2RP2003383
   Homo sapiens mRNA for KIAA0458 protein, complete cds.
   3.9e-111:549:97
   AB007927
R-NT2RP2003390
   Human DNA sequence *** SEQUENCING IN PROGRESS *** from clone 191J18, WORKING DRAFT SEQUENCE.
   4.9e-102:413:99
   AL024507
R-NT2RP2003469
   Genomic sequence from Human 9q34, complete sequence.
   1.4e-35:376:74
   AC001644
R-NT2RP2003545
   Plasmodium falciparum DNA *** SEQUENCING IN PROGRESS *** from MAL4P1, WORKING DRAFT SEQUENCE.
   1.5e-09:503:61
   AL034557
R-NT2RP2003593
   Human DNA sequence *** SEQUENCING IN PROGRESS *** from clone 971N18, WORKING DRAFT SEQUENCE.
   7.8e-81:433:93
   AL021396
R-NT2RP2003599
   HS_3240_A1_C04_T7 CIT Approved Human Genomic Sperm Library D Homo sapiens genomic clone Plate=3240 Col=7 Row=E, genomic survey sequence.
   0.091:341:58
   AQ206348
R-NT2RP2003655
   Homo sapiens PAC clone DJ0015I23 from 22, complete sequence.
   2.0e-08:249:69
   AC004819
R-NT2RP2003664
   Homo sapiens mRNA for leptin receptor gene-related protein.
   1.7e-110:549:96
   Y12670
R-NT2RP2003931
   Human mRNA for KIAA0365 gene, partial cds.
   5.4e-112:571:96
   AB002363
R-NT2RP2003940
   Human Chromosome 11 pac pDJ1173a5, complete sequence.
   2.4e-20:353:70
   AC000378
R-NT2RP2003950
   Homo sapiens clone 24778 unknown mRNA.
   1.5e-96:494:95
   AF070572
R-NT2RP2004069
   Human DNA sequence from clone 618F1 on chromosome Xq25 Contains part of gene similar to DOC4, CA repeat, GSS, complete sequence.
   2.6e-50:539:75
   AL023878
R-NT2RP2004108
   RPCII1-91F9.TV RPCI11 Homo sapiens genomic clone R-91F9, genomic survey sequence. 0.00013:281:63
   AQ283338
R-NT2RP2004141
   *c*SRL-115f11-u cSRL flow sorted Chromosome 11 specific cosmid Homo sapiens genomic clone cSRL-115f11, genomic survey sequence.
   2.3e-05:239:64
   B00539
R-NT2RP2004179
   Genomic sequence from Human 9q34, complete sequence.
   0.43:130:68
   AC002322
R-NT2RP2004205
   Homo sapiens chromosome 7q22 sequence, complete sequence.
   1.4e-42:324:83
   AF053356
R-NT2RP2004447
   Homo sapiens Chromosome 11q13 BAC Clone 18h3, WORKING DRAFT SEQUENCE, 7 ordered pieces.
   5.5e-35:285:84
   AC000353
R-NT2RP2004495
   transcript ch123.Rev [human, RF1,RF48 stomach cancer cell lines, mRNA, 252 nt].
   3.4e-44:238:97
   S77359
R-NT2RP2004524
   Genomic sequence from Human 9q34, complete sequence.
   7.4e-113:572:96
   AC001644
R-NT2RP2004556
   CIT-HSP-2306F6.TF CIT-HSP Homo sapiens genomic clone 2306F6, genomic survey sequence.
   8.1e-99:514:95
   AQ019229
R-NT2RP2004606 cDNA encoding NIC(Natural Inhibitor of Collagenase).
   8.2e-116:576:96
   E00985
R-NT2RP2004648
   Homo sapiens chromosome 17, clone hRPK.269_G_24, complete sequence.
   0.98:369:57
   AC005828
R-NT2RP2004670
   Rattus norvegicus vesicla-associate calmodulin-binding protein mRNA, complete cds.
   4.5e-43:592:69
   L22557
R-NT2RP2004794
R-NT2RP2004837
   Human Chromosome 11 pac pDJ148f1, WORKING DRAFT SEQUENCE, 27 unordered pieces.
   1.2e-60:366:90
   AC001232
R-NT2RP2004847
   Homo sapiens full length insert cDNA clone YY87C09.
   1.0e-68:333:100
   AF086055
R-NT2RP2005027
   Human glucose transporter-like protein-III (GLUT3), complete cds.
   7.8e-103:508:97
   M20681
R-NT2RP2005069
   Rat vacuolar protein sorting homolog r-vps33b mRNA, complete cds.
   3.8e-42:463:73
   U35245
R-NT2RP2005163
   CIT-HSP-2348J20.TF CIT-HSP Homo sapiens genomic clone 2348J20, genomic survey sequence.
   7.4e-44:233:96
   AQ059444
R-NT2RP2005181
   Rattus norvegicus mRNA for cationic amino acid transporter 3, complete cds.
   7.6e-53:567:73
   AB000113
R-NT2RP2005247
   Homo sapiens Xp22 Cosmid U151G1 (from Lawrence Livermore X library) and PAC RPCI1-93D11 (from Roswell Park Cancer Center) complete sequence.
   5.8e-38:341:76
   AC002357
R-NT2RP2005378
   Homo sapiens full length insert cDNA clone YW25A12.
   0.13:152:66
   AF086029
R-NT2RP2005391
   HS_3056_A1_C03_MR CIT Approved Human Genomic Sperm Library D Homo sapiens genomic clone Plate=3056 Col=5 Row=E, genomic survey sequence.
   1.1e-14:140:84
   AQ134064
R-NT2RP2005425
   Homo sapiens mRNA for KIAA0803 protein, partial cds.
   8.2e-100:526:94
   AB018346
R-NT2RP2005463
R-NT2RP2005514
R-NT2RP2005535
   Homo sapiens DNA from chromosome 19, BAC 33152, complete sequence.
   1.9e-11:488:62
   AC003973
R-NT2RP2005541
   CIT-HSP-2034G23.TF CIT-HSP Homo sapiens genomic clone 2034G23, genomic survey sequence.
   2.7e-61:311:98
   B74709
R-NT2RP2005597
R-nnnnnnnnnnnn
   {FRA16A, folate-sensitive fragile site} [human, Genomic, 160 nt].
   0.92:104:65
   S70397
R-NT2RP2005666
R-NT2RP2005774
   Homo sapiens apoptosis-related mRNA, 3'UTR, partial sequence.
   2.2e-94:440:96
   AF035364
R-NT2RP2005878
   Homo sapiens chromosome 19, cosmid F17987, complete sequence.
   1.3e-32:340:76
   AC004790
R-NT2RP2005883
   Human DNA sequence from clone 248E1 on chromosome 6q23.1-23.3 Contains DOPAMINE-BETA-MONOOXYGENASE PRECURSOR, EF-1-ALPHA-2 pseudogene EST GSS and CA repeat, complete sequence.
   1.3e-117:581:97
   AL023578
R-NT2RP2005887
   Human Chromosome 11 pac pDJ148f1, WORKING DRAFT SEQUENCE, 27 unordered pieces
   2.5e-61:367:90
   AC001232
R-nnnnnnnnnnnn
   Human paired box gene (PAX6) homologue, complete cds.
   5.0e-115:578:96
   M93650
R-NT2RP2005994
   Homo sapiens chromosome 4 clone B207D4 map 4q25, complete sequence.
   2.4e-116:594:96
   AC004050
R-NT2RP2006004
   Rattus norvegicus Shal-related potassium channel Kv4.3 mRNA, complete cds.
   1.8e-45:264:93
   U42975
R-NT2RP2006042
   T31H24TF TAMU Arabidopsis thaliana genomic clone T31H24, genomic survey sequence.
   0.42:111:70
   B78148
R-NT2RP2006092
   Homo sapiens chromosome 5, BAC clone 319C17 (LBNL H159), complete sequence.
   1.7e-73:385:95
   AC005214
R-NT2RP2006099
   Homo sapiens PAC clone DJ0903G02, complete sequence.
   1.3e-27:335:74
   AC004924
R-NT2RP2006134
   Homo sapiens chromosome 4 clone B139M23 map 4q25, complete sequence.
   1.0:143:63
   AC004045
R-NT2RP2006269
   Phreatamoeba balamuthi UBI3 sequence, putative polyubiquitin gene.
   0.82:153:63
   AJ000657
R-NT2RP2006512
   Homo sapiens clone 23904 mRNA sequence.
   4.6e-106:531:96
   AF052129
R-NT2RP3000011
   HS_2196_A2_E08_MR CIT Approved Human Genomic Sperm Library D Homo sapiens genomic clone Plate=2196 Col=16 Row=I, genomic survey sequence.
   1.3e-36:292:83
   AQ210450
R-NT2RP3000022
   Plasmodium falciparum DNA *** SEQUENCING IN PROGRESS *** from contig 3-15, complete sequence.
   0.28:248:60
   Z98550
R-NT2RP3000059
   Homo sapiens chick ovalbumin upstream promoter transcription factor II (COUP-TFII) mRNA, partial cds.
   0.047:393:61
   M62760
R-NT2RP3000063
   HS_3190_B2_D10_MR CIT Approved Human Genomic Sperm Library D Homo sapiens genomic clone Plate=3190 Col=20 Row=H, genomic survey sequence.
   0.88:232:63
   AQ172428
R-nnnnnnnnnnnn
   RPCI11-35A1.TJ RPCI-11 Homo sapiens genomic clone RPCI-11-35A1, genomic survey sequence.
   3.8e-29:159:99
   AQ045699
R-NT2RP3000148
   Homo sapiens full length insert cDNA clone ZE03A07.
   2.8e-112:574:95
   AF086510
R-NT2RP3000169
   Homo sapiens MRS1 mRNA, complete cds.
   4.4e-110:551:96
   AF093239
R-NT2RP3000171
R-NT2RP3000172
   Rattus norvegicus vesicla-associate calmodulin-binding protein mRNA, complete cds.
   1.3e-40:554:70
   L22557
R-NT2RP3000201
   Homo sapiens BAC clone NH0353P23 from 2, complete sequence.
   6.4e-96:478:97
   AC005035
R-NT2RP3000232
   Plasmodium falciparum MAL3P2, complete sequence.
   0.93:262:61
   AL034558
R-NT2RP3000304
   Homo sapiens LDL receptor-related protein 6 (LRP6) mRNA, complete cds.
   2.4e-109:546:97
   AF074264
R-NT2RP3000378
   Mus musculus mSin3A (sin3A) mRNA, complete cds.
   3.0e-27:411:72
   U22394
R-NT2RP3000436
   Plasmodium falciparum chromosome 2, section 35 of 73 of the complete sequence.
   1.1e-06:440:57
   AE001398
R-NT2RP3000444
   Human DNA sequence *** SEQUENCING IN PROGRESS *** from clone 889J22, WORKING DRAFT SEQUENCE.
   5.9e-105:543:95
   AL031406
R-NT2RP3000460
   Canis familiaris sec61 homologue mRNA, complete cds.
   2.8e-12:292:68
   M96629
R-NT2RP3000481
   WORKING DRAFT SEQUENCE, 8 unordered pieces.
   0.99:160:65
   AC005992
R-NT2RP3000616
   Plasmodium falciparum 3D7 chromosome 12 PFYAC492 genomic sequence, WORKING DRAFT SEQUENCE, 5 unordered pieces.
   0.00087:412:57
   AC005308
R-NT2RP3000645
R-NT2RP3000652
   Homo sapiens DNA from chromosome 19, cosmid R32532, complete sequence.
   1.9e-44:539:74
   AC004004
R-NT2RP3000676
   Homo sapiens mRNA for KIAA0446 protein, complete cds.
   3.1e-103:542:94
   AB007915
R-NT2RP3000677
   Homo sapiens genomic DNA of 9q32 anti-oncogene of flat epitherium cancer, segment 4/10.
   0.067:235:61
   AB020872
R-NT2RP3000721
   CIT-HSP-2348J20.TF CIT-HSP Homo sapiens genomic clone 2348J20, genomic survey sequence.
   4.0e-45:233:98
   AQ059444
R-NT2RP3000789
R-NT2RP3000818
   Human DNA sequence *** SEQUENCING IN PROGRESS *** from clone 94M16, WORKING DRAFT SEQUENCE.
   5.7e-95:510:93
   Z97201
R-NT2RP3000820
   RPCI11-77B13.TJ RPCI11 Homo sapiens genomic clone R-77B13, genomic survey sequence.
   2.1e-50:266:96
   AQ283547
R-NT2RP3000838
   Homo sapiens mRNA for KIAA0638 protein, partial cds.
   4.6e-99:522:94
   AB014538
R-NT2RP3000871
   Homo sapiens clone DJ0703P08, WORKING DRAFT SEQUENCE, 23 unordered pieces.
   0.68:249:61
   AC005481
R-NT2RP3000907
   X.laevis oocyte repetitive sequence (XLOREP) mRNA.
   2.9e-30:386:69
   X65290
R-NT2RP3000921
   HS_2026_A1_B06_MR CIT Approved Human Genomic Sperm Library D Homo sapiens genomic clone Plate=2026 Col=11 Row=C, genomic survey sequence.
   2.2e-54:311:92
   AQ232644
R-NT2RP3001012
   Rattus norvegicus mRNA for TIP120, complete cds.
   9.2e-63:456:83
   D87671
R-NT2RP3001044
   Human Chromosome 11 pac pDJ148f1, WORKING DRAFT SEQUENCE, 27 unordered pieces.
   1.2e-60:366:90
   AC001232
R-NT2RP3001061
   Plasmodium falciparum 3D7 chromosome 12 PFYAC357 genomic sequence, WORKING DRAFT SEQUENCE, 7 unordered pieces.
   0.17:357:61
   AC005506
R-NT2RP3001159
   Homo sapiens Chromosome 11q12.2 PAC clone pDJ519o13 containing human gene for ferritin heavy chain (FTH), complete sequence.
   8.8e-111:561:96
   AC004228
R-NT2RP3001170
   Homo sapiens mRNA for KIAA0784 protein, partial cds.
   8.8e-117:561:98
   AB018327
R-NT2RP3001195
   Genomic sequence from Human 9q34, complete sequence.
   1.4e-35:376:74
   AC001644
R-NT2RP3001240
   Canis familiaris sec61 homologue mRNA, complete cds.
   2.8e-12:292:68
   M96629
R-NT2RP3001271
   Homo sapiens chromosome 19, BAC CIT-B-470f8 (BC330812), complete sequence.
   7.9e-17:260:71
   AC006115
R-NT2RP3001322
   Human DNA sequence from PAC 128N22 on chromosome Xq25-Xq26.3. contains STS.
   0.035:451:60
   Z97629
R-NT2RP3001542
   Plasmodium falciparum 3D7 chromosome 12 PFYAC812 genomic sequence, WORKING DRAFT SEQUENCE, 8 unordered pieces.
   4.1e-08:500:61
   AC004153
R-NT2RP3001560
   Mouse mRNA for thymic epithelial cell surface antigen, complete cds.
   1.0e-30:523:65
   D67067
R-NT2RP3001592
R-NT2RP3001685
   Human DNA sequence from clone 91J24 on chromosome 6q24 Contains part of utrophin Gene, part of cytochrome C oxidase gene, EST, CpG island, complete sequence.
   2.4e-30:147:85
   AL024474
R-NT2RP3001738
   Homo sapiens Chromosome 11q12.2 PAC clone pDJ519o13 containing human gene for ferritin heavy chain (FTH), complete sequence.
   9.2e-107:553:95
   AC004228
R-NT2RP3001754
   Human DNA sequence *** SEQUENCING IN PROGRESS *** from clone 50024, WORKING DRAFT SEQUENCE.
   2.0e-67:345:97
   AL034380
R-NT2RP3001858
R-NT2RP3001976
   Homo sapiens chromosome 9, clone hRPK.467_F_21, complete sequence.
   4.4e-14:302:62
   AC006239
R-NT2RP3002015
   Homo sapiens clone DJ0539M06, WORKING DRAFT SEQUENCE, 10 unordered pieces.
   6.2e-65:492:82
   AC004832
R-NT2RP3002160
   Genomic sequence from Human 9q34, complete sequence.
   2.1e-82:431:95
   AC001643
R-NT2RP3002281
   Homo sapiens mRNA for KIAA0765 protein, partial cds.
   1.1e-81:446:93
   AB018308
R-NT2RP3002286
   Mus musculus EGF repeat transmembrane protein mRNA, complete cds.
   1.0e-80:378:90
   U57368
R-NT2RP3002311
   Homo sapiens chromosome 17, clone hRPK.269_G_24, complete sequence.
   0.57:366:58
   AC005828
R-NT2RP3002324
   H.sapiens gene for nitric oxide synthase (promoter region).
   1.6e-30:337:72
   Z49251
R-NT2RP3002342
   transcript ch123.Rev [human, RF1,RF48 stomach cancer cell lines, mRNA, 252 nt].
   6.5e-45:238:98
   S77359
R-NT2RP3002353
   Human Chromosome 16 BAC clone CIT987SK-A-418G10, complete sequence. 0.00015:164:70
   AC002044
NNNNNNNNNNNNNN
   Homo sapiens mRNA for KIAA0788 protein, partial cds.
   4.5e-98:493:96
   AB018331
NNNNNNNNNNNNNN
R-NT2RP3002448
   S.cerevisiae DNA for ori 2.
   0.52:91:71
   X59535
R-NT2RP3002571
R-NT2RP3002664
   Homo sapiens full length insert cDNA clone ZC48G09.
   9.9e-103:522:96
   AF086209
R-NT2RP3002721
R-NT2RP3002737
R-NT2RP3002738
   Sequence 4 from patent US 5541109.
   2.9e-22:171:74
   I24014
R-NT2RP3002790
R-NT2RP3002836
   Bos taurus retina specific RGS protein (RET-RGS1) mRNA, complete cds.
   2.3e-34:384:75
   U89254
R-NT2RP3002887
   Mus musculus cathepsin S (CatS) gene, promoter region and exons 1 and 2.
   1.6e-05:435:62
   AF051726
R-NT2RP3002900
   Homo sapiens mRNA from chromosome 5q21-22, clone:A3-B.
   1.3e-116:569:97
   AB002451
R-NT2RP3002958
   Homo sapiens clone 23851 mRNA sequence.
   2.0e-117:575:98
   AF035313
R-NT2RP3002983
   Homo sapiens genomic DNA, chromosome 21q11.1, segment 17/28, WORKING DRAFT SEQUENCE.
   5.1e-59:295:99
   AP000046
R-NT2RP3003000
   Homo sapiens clone 24597 mRNA sequence.
   6. le-109:562:95
   AF070604
R-NT2RP3003076
R-NT2RP3003354
   Human protocadherin 42 mRNA, complete cds for abbreviated PC42.
   0.87:208:61
   L11370
R-NT2RP3003448
   High throughput sequencing of human chromosome 12, WORKING DRAFT SEQUENCE, 1 ordered pieces.
   1.3e-41:287:80
   AC005840
R-NT2RP3003469
   Human DNA sequence from clone 1177E19 on chromosome 1p36.12-36.31. Contains the 3'part of the DNA-binding Zinc finger protein RIZ gene, ESTs, an STS, GSSs and a CpG island, complete sequence.
   2.1e-18:223:77
   AL031277
R-NT2RP3003473
   Human DNA sequence *** SEQUENCING IN PROGRESS *** from clone 250D10, WORKING DRAFT SEQUENCE.
   1.5e-54:294:96
   Z99716
R-NT2RP3003527
   Homo sapiens mRNA for protein kinase Dyrk1B.
   5.1e-91:445:97
   Y17999
R-NT2RP3003532
   Mus musculus cell surface molecule OX-2 mRNA, complete cds.
   1.2e-30:529:67
   AF004023
R-nnnnnnnnnnnn
   Homo sapiens PAC clone DJ0531G15 from 7p21, complete sequence.
   0.13:294:61
   AC004739
R-NT2RP3003559
   CIT-HSP-2307F17.TR CIT-HSP Homo sapiens genomic clone 2307F17, genomic survey sequence.
   1.4e-15:342:68
   AQ016972
R-NT2RP3003614
   Homo sapiens chromosome 19, cosmid F21967, complete sequence.
   0.013:174:64
   AC005256
R-NT2RP3003729
R-NT2RP3003849
   Genomic sequence from Human 9q34, WORKING DRAFT SEQUENCE, 6 unordered pieces.
   8.9e-106:557:94
   AC002320
R-NT2RP3003874
   Homo sapiens incomplete cDNA for a mutated allele of a myosin class I, myh-1c.
   1.6e-55:302:94
   AJ001381
R-NT2RP3003963
   cSRL-66f9-u cSRL flow sorted Chromosome 11 specific cosmid Homo sapiens genomic clone cSRL-66f9, genomic survey sequence.
   0.028:78:76
   B05608
R-NT2RP3004000
   Tapa-1=integral membrane protein TAPA-1 [mice, B cell lymphoma line 38C13, Genomic, 861 nt, segment 7 of 7].
   0.87:212:62
   S45012
R-NT2RP3004025
   Homo sapiens chromosome 19, cosmid F17987, complete sequence.
   0.71:197:62
   AC004790
R-NT2RP3004075
R-NT2RP3004083
   Arabidopsis thaliana DNA chromosome 4, ESSA I contig fragment No. 5.
   0.27:375:59
   Z97340
R-NT2RP3004090
   CIT-HSP-2172H20.TR CIT-HSP Homo sapiens genomic clone 2172H20, genomic survey sequence.
   2.2e-40:243:91
   B99962
R-NT2RP3004119
   Homo sapiens PAC clone DJ1059M17 from 7q21-q31.1, complete sequence.
   8.3e-42:475:73
   AC004953
R-NT2RP3004130
R-NT2RP3004133
   CIT-HSP-2306G15.TR CIT-HSP Homo sapiens genomic clone 2306G15, genomic survey sequence.
   0.00037:194:64
   AQ022229
R-NT2RP3004202
   Homo sapiens BAC clone GS285F21 from 7q21-q22, complete sequence.
   0.65:209:62
   AC004012
R-NT2RP3004294
R-NT2RP3004309
   Homo sapiens Chromosome 11q12.2 PAC clone pDJ519o13 containing human gene for ferritin heavy chain (FTH), complete sequence.
   7.4e-99:500:96
   AC004228
R-NT2RP3004321
   Human chromosome 11 168h3 cosmid, complete sequence.
   1.7e-105:540:96
   U73637
R-NT2RP3004345
   Human BAC clone RG016J04 from 7q21, complete sequence.
   0.00033:348:61
   AC002064
R-NT2RP3004355
   Plasmodium falciparum 3D7 chromosome 12 PFYAC1122 genomic sequence, WORKING DRAFT SEQUENCE, 3 unordered pieces.
   0.0029:180:66
   AC004709
R-NT2RP3004374
   Human DNA sequence from clone 1177E19 on chromosome 1p36.12-36.31. Contains the 3' part of the DNA-binding Zinc finger protein RIZ gene, ESTs, an STS, GSSs and a CpG island, complete sequence.
   4.3e-18:223:77
   AL031277
R-NT2RP3004406
   Plasmodium falciparum DNA *** SEQUENCING IN PROGRESS *** from contig 4-70, complete sequence.
   1.0:201:62
   AL010239
R-NT2RP3004481
R-NT2RP3004552
   Human germline immunoglobulin lambda light chain pseudogene (VII.1).
   1.0:165:63
   X57825
R-NT2RP3004625
   Homo sapiens I-1 receptor candidate protein mRNA, complete cds.
   8.2e-49:352:84
   AF082516
R-NT2RP3004640
   Homo sapiens full length insert cDNA clone ZC45E05.
   1.2e-96:471:98
   AF086205
R-NT2RP3004647
   Homo sapiens mRNA for KIAA0446 protein, complete cds.
   1.5e-109:555:96
   AB007915
R-NT2RP4000108
   Mouse neurofilament protein (NF-L) gene, 3' flank.
   1.0e-09:344:63
   M55424
R-NT2RP4000634
   Homo sapiens Xp22 BAC GSHB 526D21 (Genome Systems Human BAC library) complete sequence.
   1.6e-16:267:71
   AC003037
R-NT2RP4000962
   Human DNA sequence from PAC 970D1 on chromosome 1q24. Contains ESTs, STSs and a BAC end-sequence (GSS).
   0.026:176:67
   AL021069
R-NT2RP4001001
   Drosophila melanogaster Oregon-R mitochondrial A+T region.
   0.00026:354:61
   U11584
R-NT2RP4001009
   Homo sapiens mRNA for Hs Ste24p, complete cds.
   1.6e-82:408:98
   AB016068
R-NT2RP4001467
   Human placental cDNA coding for 5'nucleotidase (EC 3.1.3.5).
   1.8e-111:545:97
   X55740
R-NT2RP4001877
   Yeast (S.cerevisiae) mitochondrial cob gene, intron 4.
   0.19:384:59
   J01469
R-NT2RP4001879
   Homo sapiens full length insert cDNA clone ZD76G10.
   4.4e-107:548:94
   AF086408
R-NT2RP4002187
   RPCI11-69F22.TK RPCI11 Homo sapiens genomic clone R-69F22, genomic survey sequence.
   7.1e-37:240:89
   AQ238297
R-NT2RP4002451
   Human DNA sequence *** SEQUENCING IN PROGRESS *** from clone 111B22, WORKING DRAFT SEQUENCE.
   5.8e-111:575:96
   Z98200
R-NT2RP4002715
   Human Chromosome 11 pac pDJ148f1, WORKING DRAFT SEQUENCE, 27 unordered pieces.
   2.5e-61:367:90
   AC001232
R-NT2RP4002750
   Rattus norvegicus mRNA for cationic amino acid transporter 3, complete cds.
   1.2e-52:527:74
   AB000113
R-OVARC1000003
   Homo sapiens clone DJ0856O24, WORKING DRAFT SEQUENCE, 4 unordered pieces.
   2.3e-10:140:77
   AC004909
R-OVARC1000090
   Homo sapiens genomic DNA, 237 kb segment from 6p21.3 region including HLA genes, WORKING DRAFT SEQUENCE.
   2.8e-59:323:78
   D84394
R-OVARC1000105
   H.sapiens gene for ribosomal protein L38.
   2.7e-12:83:100
   Z26876
R-OVARC1000137
R-OVARC1000208
   Homo sapiens PAC clone DJ0817I18 from 7p11.2p13, complete sequence.
   2.7e-52:464:79
   AC004901
R-OVARC1000255
   H.sapiens syk mRNA for protein-tyrosine kinase.
   1.9e-105:511:98
   Z29630
R-OVARC1000275
R-OVARC1000298
   Plasmodium falciparum carbamoyl phosphate synthetase II gene, complete cds.
   0.66:364:59
   L32150
R-OVARC1000307
R-OVARC1000313
   Homo sapiens mRNA for KIAA0573 protein, partial cds.
   1.6e-96:534:93
   AB011145
R-OVARC1000331
   Sequence 2 from patent US 5763589.
   8.1e-66:335:97
   AR012692
R-OVARC1000410
   Homo sapiens clone 23767 and 23782 mRNA sequences.
   1.0e-88:462:94
   AF007150
R-OVARC1000439
   E.coli fanG and fanH genes.
   0.99:424:58
   Y00531
R-OVARC1000467
   HS_3235_A2_A12_T7 CIT Approved Human Genomic Sperm Library D Homo sapiens genomic clone Plate=3235 Col=24 Row=A, genomic survey sequence.
   9.2e-09:125:76
   AQ206826
R-OVARC1000529
R-OVARC1000553
   Homo sapiens chromosome 19, cosmid R26894, complete sequence.
   2.0e-84:437:96
   AC005594
R-OVARC1000775
   CIT-HSP-2060P5.TF CIT-HSP Homo sapiens genomic clone 2060P5, genomic survey sequence. 0.29:223:63
   B70025
R-OVARC1000811
   Homo sapiens chromosome 16, cosmid clone 432A1 (LANL), complete sequence.
   2.8e-89:438:98
   AC004235
R-OVARC1000853
   RPCI11-16C11.TV RPCI-11 Homo sapiens genomic clone RPCI-11-16C11, genomic survey sequence.
   5.3e-53:317:92
   B76661
R-OVARC1000873
   Human DNA sequence from clone 1049G16 on chromosome 20q12-13.2 Contains gene similar to GLUCOSAMINE-6-SULFATASE, a nuclear receptor coactivator gene, ESTs, STSs, GSSs, complete sequence.
   1.2e-102:511:97
   AL034418
R-OVARC1000916
   H.sapiens PISSLRE mRNA.
   5.8e-111:588:94
   X78342
R-OVARC1000956
   Human DNA sequence from cosmid L241B9, Huntington's Disease Region, chromosome 4p16.3 contains polymorphic VNTR pYNZ32.
   2.7e-89:478:94
   Z69708
R-OVARC1000995
   Human DNA sequence from clone 885E17 on chromosome 1p33-34.1. Contains STSs and GSSs and genomic marker D1S1302, complete sequence.
   9.5e-46:325:84
   AL031294
R-OVARC1001030
   Homo sapiens neuroendocrine-specific protein C (NSP) mRNA, complete cds.
   1.2e-05:197:63
   L10335
R-OVARC1001049
R-OVARC1001086
   Homo sapiens cyclin T2a mRNA, complete cds.
   4.3e-105:569:94
   AF048731
R-OVARC1001132
   Homo sapiens genomic DNA, chromosome 21q11.1, segment 9/28, WORKING DRAFT SEQUENCE.
   2.2e- 111:569:96
   AP000038
R-OVARC1001163
   Sus scrofa MHC SLA class III steroid 21-hydroxylase (CYP21) gene, complete cds, ORF human-like gene, last 5 exons.
   0.010:349:60
   M83939
R-OVARC1001222
   Spiroplasma citri orfa and orff genes, partial cds, orfb, orfc, and orfe genes and Spiroplasma virus SpV1-derived ORF1 and ORF3 genes, complete cds, and SpV1-derived ORF14 gene, partial cds.
   0.58:184:60
   U28972
R-OVARC1001260
   Homo sapiens clone DJ0856O24, WORKING DRAFT SEQUENCE, 4 unordered pieces.
   1.1e-10:140:78
   AC004909
R-OVARC1001336
   Homo sapiens clone DJ0856O24, WORKING DRAFT SEQUENCE, 4 unordered pieces.
   2.3e-10:140:77
   AC004909
R-OVARC1001338
R-OVARC1001569
R-OVARC1001570
R-OVARC1001596
   Homo sapiens chromosome 17, clone hRPK.372_K_20, complete sequence.
   5.9e-47:361:83
   AC005951
R-OVARC1001607
   Human beta-1,2-N-acetylglucosaminyltransferase II (MGAT2) gene, complete cds.
   3.3e-112:559:96
   U15128
R-OVARC1001725
   Homo sapiens patched related protein TRC8 (TRC8) gene, exon 1 and partial cds.
   3.9e-56:318:95
   AF064800
R-OVARC1001727
   Human DNA sequence from clone 48A11 on chromosome 20p12 Contains EST, STS, GSS, complete sequence.
   6.1e-101:533:94
   AL031132
R-OVARC1001807
   Human TR3 orphan receptor mRNA, complete cds.
   2.8e-87:426:97
   L13740
R-OVARC1001833
   Mouse fork head related protein (HNF-3beta) mRNA, complete cds.
   1.1e-21:263:76
   L10409
R-OVARC1001991
   H.sapiens chromosome 22 CpG island DNA genomic Mse1 fragment, clone 301e3, reverse read 301e3.r.
   0.90:151:59
   Z79826
R-OVARC1002058
   Homo sapiens full length insert cDNA clone ZD58C02.
   1.9e-105:547:95
   AF088043
R-OVARC1002178
   Human DNA sequence from clone 267M20 on chromosome Xq22.2-22.3. Contains part of the DIAPH2 gene and a pseudogene, ESTs, STSs and GSSs, complete sequence.
   0.26:429:58
   AL031053
R-PLACE1000033
   Plasmodium falciparum 3D7 chromosome 12 PFYAC69 genomic sequence, WORKING DRAFT SEQUENCE, 4 unordered pieces.
   0.098:467:59
   AC004688
R-PLACE1000231
   Homo sapiens BAC clone RG060N22 from 7q21, complete sequence.
   0.91:141:64
   AC003083
R-PLACE1000258
   Human DNA sequence from clone 710L4 on chromosome Xq11.2-12 Contains part of a gene similar to myotubularin-related protein, EST, STS and GSS, complete sequence.
   3.8e-53:524:75
   AL034408
R-PLACE1000442
   Arabidopsis thaliana genomic DNA, chromosome 5, TAC clone: K22F20, complete sequence.
   3.0e-07:413:62
   AB016873
R-PLACE1000560
   Homo sapiens chromosome 5, BAC clone 194j18 (LBNL H158), complete sequence.
   6.3e-59:323:94
   AC005368
R-PLACE1000740
   H.sapiens PEX gene.
   0.0065:202:63
   Y10196
R-PLACE1000912
R-PLACE1000914
   Homo sapiens chromosome 17, clone HCIT145P4, WORKING DRAFT SEQUENCE, 8 unordered pieces.
   3.4e-68:452:86
   AC002093
R-PLACE1000927
   Cowpox virus strain GRI-90 DNA (49 kb fragment).
   1.8e-46:432:76
   Y15035
R-PLACE1000986
   HS_2037_A2_B06_MR CIT Approved Human Genomic Sperm Library D Homo sapiens genomic clone Plate=2037 Col=12 Row=C, genomic survey sequence.
   0.087:48:89
   AQ232754
R-PLACE1001016
   M.fascicularis gene for apolipoprotein A-IV.
   0.016:226:61
   X68361
R-PLACE1001100
   Human DNA sequence from PAC 426I6 on chromosome 1p34.1-1p35. Contains NIPP-1-like gene a nuclear inhibitor of protein phosphatase-1, ESTs, and a CA repeat.
   3.4e-37:320:80
   AL020997
R-PLACE1001114
   RPC111-5C23.TV RPCI-11 Homo sapiens genomic clone RPCI-11-5C23, genomic survey sequence.
   9.2e-44:173:85
   B49180
R-PLACE1001123
R-PLACE1001183
   Plasmodium falciparum MAL3P8, complete sequence.
   0.47:217:63
   AL034560
R-PLACE1001229
   Mitochondrion Culex torrentium A+T rich mitochondrial control region.
   3.3e-09:356:63
   U69573
R-PLACE1001231
   Rattus norvegicus sodium-dependent multi-vitamin transporter (SMVT) mRNA, complete cds.
   1.2e-09:186:72
   AF026554
R-PLACE1001340
   Homo sapiens mRNA for KIAA0719 protein, complete cds.
   2.0e-51:265:98
   AB018262
R-PLACE1001401
   Bactrocera dorsalis strain Tahiti mitochondrial D-loop region, complete sequence.
   0.0073:203:60
   AF033929
R-PLACE1001407
   Human DNA sequence from clone 496H19 on chromosome 6q24 Contains ESTs, complete sequence.
   5.8e-70:360:96
   AL023582
R-PLACE1001464
   Human placental cDNA coding for 5'nucleotidase (EC 3.1.3.5).
   3.1e-90:457:96
   X55740
R-PLACE1001500
   Homo sapiens clone DJ0917G04, WORKING DRAFT SEQUENCE, 35 unordered pieces.
   1.0:232:62
   AC004929
R-PLACE1001516
   Homo sapiens Chromosome 16 BAC clone CIT987SK-A-575C2, complete sequence.
   1.9e-26:168:88
   AC002425
R-PLACE1001536
   Human Chromosome X clone bWXD187, complete sequence.
   6.5e-61:310:98
   AC004383
R-PLACE1001564
   Human DNA sequence *** SEQUENCING IN PROGRESS *** from clone 287G14, WORKING DRAFT SEQUENCE.
   2.9e-100:504:97
   AL033377
R-PLACE1001655
   Homo sapiens Shab-related delayed-rectifier K+ channel alpha subunit (KCNS3) mRNA, complete cds.
   3.8e-1 17:578:97
   AF043472
R-PLACE1001788
   Sequence 9 from Patent WO9722695.
   1.9e-05:91:82
   A63556
R-PLACE1001795
R-PLACE1001836
   , complete sequence.
   4.1e-14:269:69
   AC005406
R-PLACE1001918
   Human HepG2 3' region MboI cDNA, clone hmd4f06m3.
   7.3e-25:151:95
   D17237
R-PLACE1001949
   Human DNA sequence from PAC 436M11 on chromosome Xp22.11-22.2. Contains the serine threonine protein phosphatase gene PPEF1, and the first coding exon of the RS1 gene for retinoschisis (X-linked, juvenile) 1 (XLRS1). Contains ESTs, an STS and GSSs, complete sequence.
   0.54:165:63
   Z94056
R-PLACE1002080
   Homo sapiens chromosome 17, clone hRPC.971_F_3, WORKING DRAFT SEQUENCE, 1 ordered pieces.
   3.7e-60:289:95
   AC004150
R-PLACE1002095
   Plasmodium falciparum DNA *** SEQUENCING IN PROGRESS *** from contig 3-52, complete sequence.
   0.00052:422:59
   AL008982
R-PLACE1002153
   Homo sapiens TACC2 protein (TACC2) mRNA, partial cds.
   4.6e-100:514:95
   AF095791
R-PLACE1002329
   Homo sapiens chromosome 19, cosmid R31855, complete sequence.
   1.3e-46:257:95
   AC005782
R-PLACE1002355
   HS_2057_B1_D01_T7 CIT Approved Human Genomic Sperm Library D Homo sapiens genomic clone Plate=2057 Col=1 Row=H, genomic survey sequence.
   0.089:132:65
   AQ245227
R-PLACE1002374
   Human mRNA for pro-cathepsin L (major excreted protein MEP).
   2.6e-101:501:97
   X12451
R-PLACE1002518
   HS_2176_A2_D04_MR CIT Approved Human Genomic Sperm Library D Homo sapiens genomic clone Plate=2176 Col=8 Row=G, genomic survey sequence.
   1.7e-43:221:100
   AQ014851
R-PLACE1002547
   Homo sapiens mRNA for KIAA0719 protein, complete cds.
   2.0e-53:276:97
   AB018262
R-PLACE1002726
   Human DNA-binding protein ABP/ZF mRNA, complete cds.
   1.1e-37:212:94
   U82613
R-PLACE1002905
   Human DNA sequence *** SEQUENCING IN PROGRESS *** from clone 215D11, WORKING DRAFT SEQUENCE.
   1.2e-42:302:86
   AL034417
R-PLACE1002911
R-PLACE1002967
   Homo sapiens chromosome 16, BAC clone 461A8, complete sequence.
   2.3e-39:310:82
   AC006111
R-PLACE1003135
   Plasmodium falciparum DNA *** SEQUENCING IN PROGRESS *** from MAL4P1, WORKING DRAFT SEQUENCE.
   2.2e-07:418:60
   AL034557
R-PLACE1003163
   Homo sapiens DBI-related protein mRNA, complete cds.
   4.7e-110:547:96
   AF069301
R-PLACE1003407
   Homo sapiens putative transmembrane protein (CLN5) mRNA, complete cds.
   1.7e-47:287:91
   AF068227
R-PLACE1003428
   Human DNA sequence from clone 55C23 on chromosome 6q22.3-23.3 contains vanin-like genes VNN1 and VNN2, ESTs, GSSs,, complete sequence.
   1.1e-75:268:98
   AL032821
R-PLACE1003438
R-PLACE1003460
   HS_3026_B1_A08_MR CIT Approved Human Genomic Sperm Library D Homo sapiens genomic clone Plate=3026 Col=15 Row=B, genomic survey sequence.
   0.30:100:69
   AQ093523
R-nnnnnnnnnnnn
   Homo sapiens clone DJ0981007, complete sequence.
   3.3e-46:135:98
   AC006017
R-PLACE1003573
   HS_3079_B2_A02_MR CIT Approved Human Genomic Sperm Library D Homo sapiens genomic clone Plate=3079 Col=4 Row=B, genomic survey sequence.
   1.1e-49:261:96
   AQ121751
R-PLACE1003598
R-PLACE1003644
   Caenorhabditis elegans cosmid F52H3, complete sequence.
   0.38:345:62
   Z66512
R-PLACE1003737
   Homo sapiens Xp22-83 BAC GSHB-324M7 (Genome Systems Human BAC Library) complete sequence.
   1.9e-77:406:96
   AC005859
R-PLACE1003772
   Human DNA sequence from PAC 426I6 on chromosome 1p34.1-1p35. Contains NIPP-1-like gene a nuclear inhibitor of protein phosphatase-1, ESTs, and a CA repeat.
   2.2e-29:454:70
   AL020997
R-PLACE1003839
   Homo sapiens Chromosome 16 BAC clone CIT987SK-A-69G12, complete sequence.
   3.0e-52:272:97
   AC004131
R-PLACE1003845
   HS_3219_A1_A10_MR CIT Approved Human Genomic Sperm Library D Homo sapiens genomic clone Plate=3219 Col=19 Row=A, genomic survey sequence.
   1.5e-13:231:70
   AQ181482
R-PLACE1003852
   Homo sapiens mRNA for KIAA0758 protein, partial cds.
   6.8e-86:439:96
   AB018301
R-PLACE1004028
R-PLACE1004078
   Homo sapiens 12p13.3 PAC RPCI5-940J5 (Roswell Park Cancer Institute Human PAC Library) complete sequence.
   5.0e-36:310:80
   AC006064
R-PLACE1004166
   Petromyzon marinus neurofilament subunit NF-180 mRNA, complete cds.
   0.95:224:62
   U19361
R-nnnnnnnnnnnn
   Fugu rubripes GSS sequence, clone 076D01bH10, genomic survey sequence.
   3.0e-08:107:77
   AL026605
R-PLACE1004199
   Human prostaglandin D2 synthase gene, exons 2 through 6 and complete cds.
   0.0028:157:67
   M98538
R-PLACE1004279
   Human DNA sequence from PAC 193B12 on chromosome 6p21.3-22.3. Contains histone H2A/d, H2B/d, H2A.i, H1.5, H3.F, H4.k, H3/j genes, histone pH2b.i and hypothetical protein A4 pseudogenes, histone H2A.1 and H2B.2 duplicate genes, Glycine (GGC) tRNA gene, olfactory receptor OL1 like gene, ESTs STSs and predicted CpG islands.
   0.00065:228:58
   Z98744
R-PLACE1004282
R-PLACE1004305
   Homo sapiens mRNA for KIAA0740 protein, complete cds.
   2.0e-77:377:99
   AB018283
R-PLACE1004441
   RPCI11-76P13.TV RPCI11 Homo sapiens genomic clone R-76P13, genomic survey sequence.
   1.8e-73:370:97
   AQ281810
R-PLACE1004450
   Arabidopsis thaliana genomic DNA, chromosome 5, TAC clone: K24G6, complete sequence.
   0.87:269:59
   AB012242
R-PLACE1004482
   Mus musculus hematopoietic lineage switch 2 (HLS2) mRNA, complete cds.
   5.2e-33:356:75
   AF009513
R-PLACE1004492
   Mus musculus mRNA for Doc2, partial cds.
   4.1e-28:268:77
   D50000
R-PLACE1004519
   Human DNA sequence from clone 24o18 on chromosome 6p21.31-22.2 Contains zinc finger protein pseudogene, VNO-type olfactory receptor pseudogene, nuclear envelope pore membrane protein, EST, STS, GSS, complete sequence.
   1.8e-14:330:67
   AL021808
R-PLACE1004520
   Human pregnancy specific beta-1-glycoprotein 1 (PSG1) gene.
   1.4e-73:397:93
   M93705
R-PLACE1004630
R-PLACE1004637
   Human Chromosome 11 Cosmid cSRL16b6, complete sequence.
   5.5e-108:625:91
   U73638
R-PLACE1004648
R-PLACE1004816
   Homo sapiens mRNA for Hakata antigen, complete cds.
   5.6e-103:586:90
   D88587
R-PLACE1004887
   CIT-HSP-2172H20.TR CIT-HSP Homo sapiens genomic clone 2172H20, genomic survey sequence.
   1.2e-31:177:97
   B99962
R-PLACE1005003
   Mus musculus clone OST13719, genomic survey sequence.
   0.0043:159:64
   AF046703
R-PLACE1005005
   Homo sapiens ubiquitin conjugating enzyme G2 (UBE2G2) mRNA, complete cds.
   2.1e-56:299:95
   AF032456
R-PLACE1005031
   Homo sapiens chromosome 17, clone hRPK.156_L_14, complete sequence.
   1.0:155:65
   AC005821
R-PLACE1005239
   Homo sapiens mRNA for HIRIP3 protein (clone pH4-31, pH4-17).
   4.4e-85:450:93
   AJ223351
R-PLACE1005250
   Mus musculus maternal transcript Maid (Maid) mRNA, complete cds.
   7.7e-19:232:73
   U50734
R-PLACE1005383
   Homo sapiens UP50 mRNA, complete cds.
   2.0e-77:471:88
   AF093118
R-PLACE1005410
   Canis familiaris sec61 homologue mRNA, complete cds.
   6.4e-12:132:82
   M96629
R-PLACE1005426
   Human pregnancy specific beta-1-glycoprotein 4 (PSG4) mRNA, complete cds.
   2.3e-109:574:94
   M94891
R-PLACE1005519
   Plasmodium falciparum DNA *** SEQUENCING IN PROGRESS *** from MAL4P1, WORKING DRAFT SEQUENCE.
   4.1e-08:426:61
   AL034557
R-PLACE1005539
R-PLACE1005544
   Homo sapiens chromosome 19, cosmid F20887, complete sequence.
   1.0e-17:202:73
   AC005578
R-PLACE1005569
   Human DNA sequence *** SEQUENCING IN PROGRESS *** from clone 159A1, WORKING DRAFT SEQUENCE.
   3.8e-89:470:95
   AL034397
R-PLACE 1005601
   Homo Sapiens angiotensin II receptor gene, complete cds.
   8.0e-52:301:90
   L48211
R-PLACE1005660
R-PLACE1005669
   Mitochondrion Culex torrentium A+T rich mitochondrial control region.
   9.5e-09:338:63
   U69573
R-PLACE1005682
   Caenorhabditis elegans cosmid M70.
   0.012:226:62
   AF047661
R-PLACE1005725
   Caenorhabditis elegans DNA *** SEQUENCING IN PROGRESS *** from clone Y48E1, WORKING DRAFT SEQUENCE.
   0.42:435:59
   Z92856
R-PLACE1005736
   Rattus norvegicus DD6A4-1 mRNA, partial sequence.
   9.0e-21:282:73
   AF034237
R-PLACE1005745
   RPCI11-88L20.TJ RPCI11 Homo sapiens genomic clone R-88L20, genomic survey sequence.
   2.4e-62:310:99
   AQ281511
R-PLACE1005768
   Human DNA sequence from Fosmid 24E5 on chromosome 22q11.2-qter contains parvalbumin, ESTs, STS.
   5.6e-94:511:93
   Z82185
R-PLACE1005815
   Human Chromosome 16 BAC clone CIT987SK-A-635H12, complete sequence.
   9.0e-55:586:73
   AC002310
R-PLACE1005878
R-PLACE1005927
R-PLACE1006071
   CIT-HSP-2308A18.TR CIT-HSP Homo sapiens genomic clone 2308A18, genomic survey sequence.
   1.6e-76:410:95
   AQ022149
R-PLACE1006073
   Homo sapiens mRNA for glucuronyltransferase I, complete cds.
   2.2e-97:513:93
   AB009598
R-PLACE1006079
   Homo sapiens distal-less homeobox protein (DLX3) gene, complete cds.
   5.4e-57:333:91
   AF028233
R-PLACE1006093
R-nnnnnnnnnnnn
   Caenorhabditis elegans mRNA for GAP-2-7, partial cds.
   1.9e-08:251:60
   AB011283
R-PLACE1006219
   HS_3219_A1_A10_MR CIT Approved Human Genomic Sperm Library D Homo sapiens genomic clone Plate=3219 Col=19 Row=A, genomic survey sequence.
   3.1e-12:228:69
   AQ181482
R-PLACE1006277
   Human DNA sequence *** SEQUENCING IN PROGRESS *** from clone 159A1, WORKING DRAFT SEQUENCE.
   7.8e-96:557:91
   AL034397
R-PLACE1006290
   Bacillus whitei clone pWH/Cugl satellite DNA.
   0.37:209:62
   U90159
R-PLACE1006443
   Homo sapiens chromosome 17, clone HCIT145P4, WORKING DRAFT SEQUENCE, 8 unordered pieces.
   8.9e-76:451:91
   AC002093
R-PLACE1006515
   Homo sapiens mRNA for KIAA0576 protein, partial cds.
   2.1e-76:413:94
   AB011148
R-PLACE1006716
   M.musculus gene encoding prostaglandin D synthase, putative.
   1.0:199:59
   Y10138
R-PLACE1006786
   HS_2037_A2_B06_MR CIT Approved Human Genomic Sperm Library D Homo sapiens genomic clone Plate=2037 Col=12 Row=C, genomic survey sequence.
   0.33:47:91
   AQ232754
R-PLACE1006809
   Homo sapiens Xq28 genomic DNA in the region of the L1CAM locus containing the genes for neural cell adhesion molecule L1 (L1CAM), arginine-vasopressin receptor (AVPR2), C1 p115 (C1), ARD1 N-acetyltransferase related protein (TE2), renin-binding protein (RbP), host cell factor 1 (HCF1), and interleukin-1 receptor-associated kinase (IRAK) genes, complete cds, and Xq28lu2 gene.
   0.67:241:59
   U52112
R-PLACE1006959
R-PLACE1007028
R-PLACE1007040
   Rabbit angiotensin-converting enzyrne (ACE-P) gene, 5' end.
   0.0037:208:65
   M58579
R-PLACE1007077
   CIT-HSP-2308A18.TR CIT-HSP Homo sapiens genomic clone 2308A18, genomic survey sequence.
   3.0e-76:411:94
   AQ022149
R-PLACE1007081
   RPCI11-31D7.TJ RPCI-11 Homo sapiens genomic clone RPCI-11-31D7, genomic survey sequence.
   1.9e-06:88:84
   AQ016433
R-PLACE1007096
   H.sapiens DMD gene microsatellite (147-200bp).
   1.0:142:59
   X77677
R-PLACE1007296
R-PLACE1007591
   Human DNA sequence from clone 113J7 on chromosome Xp11.22-11.4. Contains part of a putative Homeobox (pseudo?) gene, ESTs and an STS, complete sequence.
   1.6e-11:203:66
   AL023574
R-PLACE1007626
   Homo sapiens unknown mRNA, complete cds.
   4.9e-29:183:91
   AF047439
R-PLACE1007702
   Homo sapiens chromosome 17, clone 363G12, WORKING DRAFT SEQUENCE, 11 unordered pieces.
   2.3e-75:445:90
   AC002348
R-PLACE1007845
   CIT-HSP-2306G15.TR CIT-HSP Homo sapiens genomic clone 2306G15, genomic survey sequence.
   0.00045:194:64
   AQ022229
R-PLACE1007881
   CITBI-E1-2503C21.TF CITBI-E1 Homo sapiens genomic clone 2503C21, genomic survey sequence.
   0.43:104:69
   AQ263355
R-PLACE1007971
R-PLACE1008282
   Homo sapiens clone DJ0042M02, WORKING DRAFT SEQUENCE, 20 unordered pieces.
   7.7e-73:396:94
   AC005995
R-PLACE1008297
   N.frontalis enolase gene, promotor region.
   1.2e-08:457:57
   X81451
R-PLACE1008359
   Plasmodium falciparum MAL3P1, complete sequence.
   0.00044:443:56
   Z97348
R-PLACE1008469
   Homo sapiens chromosome 17, clone HCIT145P4, WORKING DRAFT SEQUENCE, 8 unordered pieces.
   4.4e-78:536:84
   AC002093
R-PLACE1008549
   Mus musculus E74-like factor 5 (Elf5) mRNA, complete cds.
   3.4e-30:256:75
   AF049702
R-PLACE1008657
   Homo sapiens BAC clone GS067A24 from 7q21.q21.2, complete sequence.
   1.9e-40:320:82
   AC005009
R-PLACE1008716
   Human beta-1,2-N-acetylglucosaminyltransferase II (MGAT2) gene, complete cds.
   8.2e-118:591:96
   U15128
R-PLACE1008744
   Sequence 1 from patent US 5691147.
   3.le-108:559:94
   I76197
R-PLACE1008984
   Human DNA sequence *** SEQUENCING IN PROGRESS *** from clone 167A19, WORKING DRAFT SEQUENCE.
   1.6e-102:503:96
   AL031427
R-PLACE1008985
   Mus musculus synaptotagmin VIII mRNA, partial cds.
   9.7e-29:255:77
   U20107
R-PLACE1009067
R-PLACE1009196
   Caenorhabditis elegans DNA *** SEQUENCING IN PROGRESS *** from clone Y48A6, WORKING DRAFT SEQUENCE.
   0.0094:206:65
   Z92854
R-PLACE1009279
   Homo sapiens serine protease (PRSS11) mRNA, partial cds.
   2.4e-26:553:62
   AF097709
R-PLACE1009527
   Human DNA-binding protein ABP/ZF mRNA, complete cds.
   7.9e-91:497:91
   U82613
R-PLACE1009546
   Human PAC clone DJ218B13 from Xq23, complete sequence.
   0.29:147:64
   AC002072
R-PLACE1009600
   Mouse mRNA for tetracycline transporter-like protein, complete cds.
   6.1e-81:466:90
   D88315
R-PLACE1009735
   Homo sapiens clone NH0523H20, complete sequence.
   2.0e-74:268:99
   AC005041
R-nnnnnnnnnnnn
   Homo sapiens DNA sequence from PAC 833B2 on chromosome Xq26.1-27.2. Contains an EST, complete sequence.
   1.9e-05:255:65
   AL023800
R-PLACE1010011
   , complete sequence.
   2.9e-77:174:100
   AC005409
R-PLACE1010078
   Human DNA sequence *** SEQUENCING IN PROGRESS *** from clone 209H1, WORKING DRAFT SEQUENCE.
   1.0:108:65
   Z84465
R-PLACE1010081
   Homo sapiens serine/threonine kinase RICK (RICK) mRNA, complete cds.
   9.2e-105:560:93
   AF027706
R-PLACE1010251
   Plasmodium falciparum MAL3P4, complete sequence.
   5.0e-07:468:58
   AL008970
R-PLACE1010445
   Pan troglodytes HS19.8-similar locus and Y Alu element, genomic survey sequence.
   1.2e-49:303:90
   AF077058
R-PLACE1010713
   RPCI11-69F22.TK RPCI11 Homo sapiens genomic clone R-69F22, genomic survey sequence.
   7.4e-11:114:80
   AQ238297
R-PLACE1010784
   Capra hircus strain Saanen, genomic survey sequence.
   7.4e-24:182:87
   AF083406
R-PLACE1010827
   nbxb0026K23f CUGI Rice BAC Library Oryza sativa genomic clone nbxb0026K23f, genomic survey sequence.
   1.0:252:61
   AQ271546
R-PLACE1010968
   Plasmodium falciparum 3D7 chromosome 12 PFYAC492 genomic sequence, WORKING DRAFT SEQUENCE, 5 unordered pieces.
   0.0038:295:57
   AC005308
R-PLACE1011045
   Homo sapiens E1-like protein mRNA, complete cds.
   1.6e-90:453:96
   AF094516
R-PLACE1011116
   Homo sapiens embryonic lung protein (HUEL) mRNA, complete cds.
   4.6e-72:385:94
   AF006621
R-PLACE1011236
   *** SEQUENCING IN PROGRESS *** Homo sapiens chromosome 4, BAC clone C0289H06; HTGS phase 1, WORKING DRAFT SEQUENCE, 4 unordered pieces.
   0.98:142:65
   AC004580
R-PLACE1011364
R-PLACE1011407
   Human DNA sequence from clone 127F18 on chromosome Xp11.4-21.3. Contains part of a novel gene with some similarity to parts of chicken Myosin Light Chain and various species' Interleukin-1 Receptor Type 1 (IL1-R-1). Contains GSSs, complete sequence.
   9.1e-27:293:74
   AL031575
R-PLACE1011516
   Fugu rubripes GSS sequence, clone 190N14aC12, genomic survey sequence.
   1.0:96:68
   AL030602
R-PLACE1011708
   Homo sapiens intrinsic factor-B12 receptor precursor, mRNA, complete cds.
   2.4e-91:521:91
   AF034611
R-PLACE1011824
   Figure 6. DNA sequence of three 3'apoB VNTR alleles.
   2.2e-06:264:65
   M23046
R-PLACE1011978
   Figure 2. Nucleotide and translated protein sequences of HPF1, -2, and -9.
   4.8e-50:553:69
   M27877
R-PLACE2000118
   Homo sapiens DNA sequence from PAC 393P12 on chromosome Xp11.21. Contains a hypothetical protein KIAA0413 (KIAA0412, KIAA0065, KIAA0569) LIKE Zinc Finger protein pseudogene, a hypothetical Proline-rich protein KIAA0269 LIKE gene and a 60S Ribosomal Protein L7 LIKE pseudogene. Contains ESTs, an STS and a GSS (BAC end sequence), complete sequence.
   3.9e-107:567:94
   AL022578
R-PLACE2000219
   Homo sapiens clone RG132J19, complete sequence.
   4.7e-39:317:82
   AC005163
R-PLACE3000181
   Human protocadherin 42 mRNA, 3' end of cds for alternative splicing PC42-8.
   3.9e-81:441:94
   L11369
R-PLACE3000213
   Sequence 1 from patent US 5691147.
   1.5e-109:559:95
   I76197
R-PLACE4000354
   Sequence 1 from patent US 5691147.
   2.7e-106:548:95
   I76197
R-PLACE4000455
   Arabidopsis thaliana genomic DNA chromosome 3, P1 clone: MEBS, complete sequence.
   9.3e-07:357:60
   AB019230
R-THYRO1000036
   Sequence 11 from patent US 5773248.
   4.0e-99:527:93
   AR014074
R-THYRO1000061
   Homo sapiens chromosome 19, cosmid R34382, complete sequence.
   7.3e-90:460:96
   AC005329
R-THYRO1000099
R-THYRO1000196
   Homo sapiens Ksp-cadherin (CDH16) mRNA, complete cds.
   1.1e-104:530:96
   AF016272
R-THYRO1000400
   Homo sapiens Chromosome 16 BAC clone CIT987SK-A-233A8, complete sequence.
   1.0:308:61
   AC004685
R-THYRO1000580
   Human Kox26 mRNA for zinc finger protein, partial.
   0.11:105:67
   X52357
R-THYRO1000584
   *** SEQUENCING IN PROGRESS *** Homo sapiens chromosome 4, BAC clone C0539L10; HTGS phase 1, WORKING DRAFT SEQUENCE, 15 unordered pieces.
   1.4e-14:241:68
   AC004480
R-THYRO1000678
   Belonogaster petiolata 16S ribosomal RNA gene, mitochondrial gene for mitochondrial rRNA, partial sequence.
   0.049:150:64
   AF066910
R-THYRO1000776
   CITBI-E1-2505N5.TF.1 CITBI-E1 Homo sapiens genomic clone 2505N5, genomic survey sequence.
   0.38:179:63
   AQ241670
R-THYRO1000795
R-THYRO1000846
   Homo sapiens hJAG2.del-E6 (JAG2) mRNA, alternatively spliced isoform of Jagged2, complete cds.
   3.6e-06:425:61
   AF029779
R-THYRO1000866
   Homo sapiens SKB1Hs mRNA, complete cds.
   4.0e-42:251:92
   AF015913
R-THYRO1000956
R-THYRO1000964
   Human Chromosome 11 Cosmid cSRL186g7, complete sequence.
   0.18:292:61
   U73627
R-THYRO1000999
   CIT-HSP-2288E24.TR CIT-HSP Homo sapiens genomic clone 2288E24, genomic survey sequence.
   3.6e-18:292:71
   AQ002356
R-THYRO1001063
   Homo sapiens chromosome 16 BAC clone CIT987SK-381E11 complete sequence.
   1.5e-27:292:76
   AF001552
R-THYRO1001071
   Human DNA sequence *** SEQUENCING IN PROGRESS *** from clone 37E16, WORKING DRAFT SEQUENCE.
   1.7e-105:513:98
   Z83844
R-THYRO1001102
   Homo sapiens clone DJ0539M06, WORKING DRAFT SEQUENCE, 10 unordered pieces.
   3.2e-62:429:86
   AC004832
R-THYRO1001113
   Caenorhabditis elegans cosmid C25F9, complete sequence.
   0.026:338:58
   Z81476
R-THYRO1001128
   Homo sapiens chromosome 9q34, clone 63G10, complete sequence.
   5.3e-12:132:79
   AC002096
R-THYRO1001205
   Homo sapiens clone DJ1173I20, WORKING DRAFT SEQUENCE, 5 unordered pieces.
   1.9e-60:251:85
   AC004987
R-THYRO1001237
R-THYRO1001242
   Mouse mRNA for thymic epithelial cell surface antigen, complete cds.
   1.5e-45:525:75
   D67067
R-THYRO1001266
   H.sapiens DNA containing a polymorphic (CA)n repeat (436bp).
   6.0e-05:258:67
   X65457
R-THYRO1001327
   Human DNA sequence from clone 453C12 on chromosome 20q12-13.12 Contains SDC4 (syndecan 4 (amphiglycan, ryudocan)) predicts a gene like the mouse transcription factor RBP-L, MATN4 (matrilin-4) STS, GSS, CpG island, complete sequence.
   2.8e-104:541:95
   AL021578
R-THYRO1001456
R-THYRO1001457
   H.sapiens mRNA for protein kinase C mu.
   2.9e-23:391:66
   X75756
R-THYRO1001471
   Homo sapiens Chromosome 16 BAC clone CIT987SK-A-952F10, complete sequence.
   0.39:271:61
   AC004787
R-THYRO1001478
R-THYRO1001495
   Homo sapiens clone DJ0813F11, WORKING DRAFT SEQUENCE, 5 unordered pieces.
   2.8e-88:446:88
   AC006006
R-THYRO1001523
   CIT-HSP-2333F9.TF CIT-HSP Homo sapiens genomic clone 2333F9, genomic survey sequence.
   1.4e-05:126:71
   AQ039390
R-THYR01001529
R-THYRO1001593
   Homo sapiens chromosome 19, cosmid R33632, complete sequence.
   3.7e-100:514:96
   AC005781
R-THYRO1001608
   Homo sapiens clone DJ0635O05, WORKING DRAFT SEQUENCE, 7 unordered pieces.
   2.3e-40:369:79
   AC004845
R-THYRO1001641
   Homo sapiens clone 24448 unknown mRNA, partial cds.
   3.4e-110:562:96
   AF070638
R-THYRO1001700
R-THYRO1001702
   Mus musculus mRNA for myeloid associated differentiation protein.
   1.1e-11:367:66
   AJ001616
R-THYRO1001725
   Homo sapiens, clone hRPK.1_A_1, complete sequence.
   9.1e-12:329:65
   AC006196
R-THYRO1001770
   Homo sapiens PAC clone DJ0755G17 from 7p21-p22, complete sequence.
   0.12:339:59
   AC004879
R-THYRO1001803
R-Y79AA1000030
   Homo sapiens chromosome 5, BAC clone 319C17 (LBNL H159), complete sequence.
   2.0e-98:515:95
   AC005214
R-Y79AA1000127
   Homo sapiens genomic DNA, chromosome 21q11.1, segment 5/28, WORKING DRAFT SEQUENCE.
   3.2e-115:551:99
   AP000034
R-Y79AA1000207
   Homo sapiens chromosome 17, clone hRPK.271_K_11, complete sequence.
   1.8e-38:282:85
   AC005562
R-Y79AA1000226
   Homo sapiens full length insert cDNA YN52F10.
   4.8e-09:104:85
   AF075033
R-Y79AA1000270
   Human mRNA for ORF, Xq terminal portion.
   1.0e-105:564:93
   D16469
R-Y79AA1000426
   Rattus norvegicus activin beta E mRNA, complete cds.
   6.1e-50:562:72
   AF089825
R-Y79AA1000521
   Rattus norvegicus steroid sulfatase (Sts) mRNA, complete cds.
   0.48:233:62
   U37138
R-Y79AA1000750
   Human DNA from cosmid f23280 from chromosome 19q13.2, genomic sequence.
   6.8e-07:320:60
   L47334
R-Y79AA1000776
R-Y79AA1000777
   Homo sapiens full length insert cDNA clone ZD93D10.
   2.9e-110:574:95
   AF088072
R-Y79AA1000876
   Mus musculus bone morphogenetic protein-6 (BMP-6) gene, exons 6 and 7 and complete cds.
   0.0096:105:71
   U73520
R-Y79AA1000959
   Homo sapiens Hsp70 binding protein HspBP1 mRNA, complete cds.
   1.0e-78:453:92
   AF093420
R-Y79AA1000967
   Rattus norvegicus vesicla-associate calmodulin-binding protein mRNA, complete cds.
   2.3e-43:263:84
   L22557
R-Y79AA1001013
R-Y79AA1001056
   Mus musculus maternal transcript Maid (Maid) mRNA, complete cds.
   1.5e-22:269:73
   U50734
R-Y79AA1001062
   D.teissieri mitochondrial DNA for tRNA-fmet, tRNA-Ile, tRNA-Gln & tRNA-Val.
   1.1e-07:494:57
   X54011
R-Y79AA1001090
   Homo sapiens Chromosome 16 BAC clone CIT987SK-A-279B10, complete sequence.
   1.2e-26:269:77
   AC002300
R-Y79AA1001212
   Homo sapiens SL15 protein mRNA, complete cds.
   5.7e-82:407:97
   AF038961
R-Y79AA1001264
   HS_2195_A2_A07_MR CIT Approved Human Genomic Sperm Library D Homo sapiens genomic clone Plate=2195 Col=14 Row=A, genomic survey sequence.
   3.4e-07:101:82
   AQ191092
R-Y79AA1001272
   Hansenula wingei mitochondrial DNA, complete sequence.
   2.1e-05:435:60
   D31785
R-Y79AA1001328
   Rattus norvegicus Delta 3 mRNA, complete cds.
   1.0e-29:356:72
   AF084576
R-Y79AA1001426
R-Y79AA1001430
   Homo sapiens mRNA for KIAA0469 protein, complete cds.
   6.2e-111:555:96
   AB007938
R-Y79AA1001523
   Homo sapiens DNA sequence from PAC 418A9 on chromosome 6q21. Contains the first (5') two exons of a CDK8 (Cell Division Protein Kinase 8) LIKE gene, a Neutral Calponin LIKE pseudogene, ESTs and STSs, complete sequence.
   3.7e-71:259:90
   Z84480
R-Y79AA1001530
   Human beta-tubulin gene (5-beta) with ten Alu family members.
   2.6e-56:301:96
   X00734
R-Y79AA1001592
   HS_2175_A2_B11_T7 CIT Approved Human Genomic Sperm Library D Homo sapiens genomic clone Plate=2175 Col=22 Row=C, genomic survey sequence.
   1.0:237:59
   AQ307634
R-Y79AA1001727
R-Y79AA1001787
   Homo sapiens mRNA for putative ATPase, partial.
   7.2e-80:405:97
   AJ009947
R-Y79AA1001795
   Human DNA sequence from clone 1033B10 on chromosome 6p21.2-21.31. Contains the BINGS gene, exons 11 to 15 of the BING4 gene, the gene for GalT3 (beta3-Galactosyltransferase), the RPS18 (40S ribosomal protein S18) gene, the SACM2L (suppressor of actin mutation 2, yeast, homolog) gene, a pseudogene similar to TAT-SF1, a Pseudogene similar to zinc finger genes, the RING1 gene, the gene for HKE6 (RING2), the gene for HKE4 (RING5), the RXRB (Retinoid X receptor beta) gene, the COL11A2 (collagen, type XI, alpha 2) gene, the HLA-DPB2 pseudogene and part of the HLA-DPA3 pseudogene. Contains predicted CpG islands, ESTs, STSs, and GSSs, complete sequence.
   4.2e-110:555:97
   AL031228
R-Y79AA1001799
R-Y79AA1001803
   Rattus norvegicus secretogranin III (SgIII) mRNA, complete cds.
   6.2e-60:499:77
   U02983
R-Y79AA1001863
   Human DNA sequence from PAC 365E2 on chromosome 6p22.3-24.1. Contains EST and STS.
   1.4e-45:261:75
   AL009177
R-Y79AA1002022
   H.sapiens mRNA for basement membrane heparan sulfate proteoglycan.
   1.0:311:61
   X62515
R-nnnnnnnnnnnn
   Plasmodium falciparum chromosome 2, section 18 of 73 of the complete sequence.
   1.0:208:62
   AE001381
R-nnnnnnnnnnnn
   Homo sapiens DNA, trinucleotide repeats region, clone CAG83.
   0.17:132:67
   AB018494
R-Y79AA1002213
   Human DNA sequence from PAC 340G1 on chromosome 6 contains STS.
   5.6e-46:490:73
   Z84719
R-Y79AA1002334
   Japanese Quail (C.coturnix) troponin T isoform mRNA, clone cC501.
   0.96:210:63
   M26599
R-Y79AA1002373
   Human BAC clone RG126M09 from 7q21-q22, complete sequence.
   9.7e-82:544:85
   AC002067
R-Y79AA1002376
   Human mitochondrial DNA, fragment M1, encoding transfer RNAs, cytochrome oxidase I, and 2 URFs.
   1.9e-111:546:97
   M10546
R-Y79AA1002378
   Mus musculus mRNA for zinc finger protein, complete cds, clone:CTfin51.
   1.5e-33:244:74
   D10630
R-Y79AA1002381
   Homo sapiens DNA sequence from PAC 179N16 on chromosome 6p21.1-21.33. Contains the SAPK4 (MAPK p38delta) gene, and the alternatively spliced SAPK2 gene coding for CSaids binding protein CSBP2 and a MAPK p38beta LIKE protein. Contains ESTs, STSs and two predicted CpG islands, complete sequence.
   0.0046:177:68
   Z95152

### Homology search result 8.

The result of the homology search in the Human Unigene(http://www.ncbi.nlm.nih.gov/UniGene) using the clone sequences of the 5'-ends.
Indicated are from the top,
the name of the clone sequence,
title of the top hit data,
the P-value: the length of the sequence used for comparison (nucleotide):similarity (%),
the Accession No. of the top hit data.

Data were not shown for the clones in which the P-value was higher than 1.
F-BNGH41000020
   ESTs
   6.6e-72:412:92
   Hs.153375:AI287812
F-BNGH41000087
   Homo sapiens mRNA for MIFR-1, complete cds
   0.027:499:57
   Hs.58269:AB010962
F-BNGH41000091
   Homo sapiens voltage-gated potassium channel eag (EAG) mRNA, complete cds
   5.2e-81:687:76
   Hs.158305:AJ001366
F-HEMBA1000006
   ESTs, Weakly similar to HYPOTHETICAL 51.2 KD PROTEIN IN LAG1-RPL14B INTERGENIC REGION [S.cerevisiae]
   2.0e-25:167:91
   Hs.9252:R53360
F-HEMBA1000121
   ESTs, Moderately similar to HYPOTHETICAL 68.7 KD PROTEIN ZK757.1 IN CHROMOSOME III [Caenorhabditis elegans]
   3.0e-34:180:98
   Hs.149509:N24022
F-HEMBA1000128
   EST
   0.00069:177:62
   Hs.158854:AI377837
F-HEMBA1000275
   Human modulator recognition factor I (MRF-1) mRNA, 3'end
   0.012:508:58
   Hs.920:M62324
F-HEMBA1000300
   Human mRNA for KIAA0355 gene, complete cds
   1.6e-46:402:78
   Hs.153014:AB002353
F-HEMBA1000349
   EST
   6.7e-08:65:95
   Hs.54372:N80032
F-HEMBA1000443
   ESTs
   6.1e-23:278:76
   Hs.69492:AA116026
F-HEMBA1000462
F-HEMBA1000477
   ESTs
   6.9e-78:414:94
   Hs.152861:AA287444
F-HEMBA1000590
   Homo sapiens mRNA for matrilin-4, partial
   7.3e-95:482:96
   Hs.129361:AJ007581
F-HEMBA1000634
   ESTs
   1.3e-38:246:86
   Hs.6145:W26640
F-HEMBA1000671
   Zinc finger protein 140 (clone pHZ-39)
   2.4e-53:469:68
   Hs.154205:U09368
F-HEMBA1000713
   Homo sapiens 10kD protein (BC10) mRNA, complete cds
   2.1e-127:442:97
   Hs.5300:AF053470
F-HEMBA1000732
   Homo sapiens latent transforming growth factor-beta binding protein 4S mRNA, complete cds
   1.0e-45:258:94
   Hs.85087:AF051344
F-HEMBA1000745
   Human cardiotrophin-1 (CTF1) mRNA, complete cds
   1.1e-07:316:61
   Hs.25537:U43030
F-HEMBA1000835
   ESTs
   4.2e-11:188:72
   Hs.116265:AI184988
F-HEMBA1000875
   Zinc finger protein 133 (clone pHZ-13)
   1.5e-27:169:93
   Hs.78434:U09366
F-HEMBA1000907
   Homo sapiens transcription factor forkhead-like 7 (FKHL7) gene, complete cds
   1.3e-06:545:60
   Hs.143551:AF048693
F-HEMBA1000940
   Homo sapiens connexin46.6 (Cx46.6) gene, complete cds
   4.1e-18:307:66
   Hs.100072:AF014643
F-HEMBA1000962
   Homo sapiens mRNA for MEGF8, partial cds
   0.0018:391:62
   Hs.158200:AB011541
F-HEMBA1001184
   Homo sapiens SH3 domain binding glutamic acid-rich-like protein (SH3BGRL) mRNA, complete cds
   2.7e-24:404:67
   Hs.14368:AF042081
F-HEMBA1001221
   Human transmembrane protein mRNA, complete cds
   7.7e-44:858:63
   Hs.78531:U19878
F-HEMBA1001228
   Human germline oligomeric matrix protein (COMP) mRNA, complete cds
   2.2e-85:463:93
   Hs.1584:AC003107
F-HEMBA1001272
   Antidiuretic hormone receptor
   0.064:616:57
   Hs.2524:L22206
F-HEMBA1001296
   Homo sapiens delta-catenin mRNA, complete cds
   0.031:410:59
   Hs.80220:U96136
F-HEMBA1001297
   Homo sapiens putative transcription factor CA150 mRNA, complete cds
   3.0e-15:143:81
   Hs.13063:AF017789
F-HEMBA1001390
   ESTs, Highly similar to polymerase I-transcript release factor [M.musculus]
   1.6e-49:297:91
   Hs.25581:AI246284
F-HEMBA1001563
   ESTs
   4.9e-12:160:74
   Hs.162813:AA524616
F-HEMBA1001621
   Human P2U nucleotide receptor mRNA, complete cds
   0.00098:314:61
   Hs.339:U07225
F-HEMBA1001878
   Homo sapiens U5 snRNP-specific 40 kDa protein mRNA, complete cds
   3.4e-172:810:98
   Hs.10290:AF090988
F-HEMBA1001886
   Human repressor transcriptional factor (ZNF85) mRNA, complete cds
   1.1e-115:849:80
   Hs.37138:U35376
F-HEMBA1002048
   Homo sapiens mRNA for APC 2 protein, complete cds
   0.96:266:62
   Hs.20912:AB012162
F-HEMBA1002131
   Homo sapiens mRNA for KIAA0584 protein, partial cds
   1.1e-45:709:66
   Hs.106794:AB011156
F-HEMBA1002163
   ASPARTYL-TRNA SYNTHETASE
   0.026:568:58
   Hs.80758:J05032
F-HEMBA1002164
   Pregnancy-associated plasma protein A
   0.0049:274:60
   Hs.158229:U28727
F-HEMBA1002167
F-HEMBA1002178
   Homo sapiens mRNA for KIAA0584 protein, partial cds
   8.3e-48:794:65
   Hs.106794:AB011156
F-HEMBA1002195
   EST
   2.0e-05:177:70
   Hs.145935:AI275921
F-HEMBA1002227
   Myristoylated alanine-rich C-kinase substrate
   1.2e-138:382:95
   Hs.75607:D10522
F-HEMBA1002239
   Homo sapiens mRNA, chromosome I specific transcript KIAA0488
   1.2e-47:570:71
   Hs.67619:AB007957
F-HEMBA1002316
   EST
   1.8e-28:246:79
   Hs.136950:AA825638
F-HEMBA1002420
   Homo sapiens GABA-B receptor mRNA, complete cds
   1.7e-05:303:63
   Hs.12307:AF056085
F-HEMBA1002421
   Syndecan 2 (heparan sulfate proteoglycan 1, cell surface-associated, fibroglycan)
   4.3e-167:778:98
   Hs.1501:J04621
F-HEMBA1002524
   Human MHC Class I region proline rich protein mRNA, complete cds
   8.5e-128:751:89
   Hs.41548:U63336
F-HEMBA1002551
   ESTs
   2.4e-25:207:84
   Hs.158172:N24325
F-HEMBA1002767
   Homo sapiens chromosome 1p33-p34 beta-1,4-galactosyltransferase mRNA, complete cds
   4.4e-170:798:98
   Hs.19154:AF038660
F-HEMBA1002985
   ESTs
   2.6e-09:124:76
   Hs.118620:T60326
F-HEMBA1002992
   ESTs
   2.4e-21:121:97
   Hs.143571:AI089396
F-HEMBA1003047
   Homo sapiens intrinsic factor-B12 receptor precursor, mRNA, complete cds
   1.5e-188:873:99
   Hs.148318:AF034611
F-HEMBA1003072
   ESTs
   1.2e-33:387:71
   Hs.59628:W91959
F-HEMBA1003101
   Homo sapiens tyrosylprotein sulfotransferase-2 mRNA, complete cds
   1.7e-140:671:98
   Hs.26350:AF049891
F-HEMBA1003120
   Zinc finger protein 91 (HPF7, HTF10)
   1.0e-24:143:76
   Hs.8597:L11672
F-HEMBA1003230
   Homo sapiens UP50 mRNA, complete cds
   1.8e-184:856:98
   Hs.11494:AF093118
F-HEMBA1003294
   Human antisecretory factor-1 mRNA, complete cds
   5.1e-45:324:83
   Hs.148495:AF050199
F-HEMBA1003315
   Homo sapiens mRNA for TIP49, complete cds
   4.2e-19:377:64
   Hs.155541:AF070735
F-HEMBA1003392
   Homo sapiens LDL receptor-related protein 6 (LRP6) mRNA, complete cds
   9.2e-185:851:99
   Hs.23672:AF074264
F-HEMBA1003399
   H.sapiens BDP1 mRNA for protein-tyrosine-phosphatase
   0.00042:297:61
   Hs.118929:X79568
F-HEMBA1003487
   Homo sapiens receptor for viral semaphorin protein (VESPR) mRNA, complete cds
   0.0011:237:63
   Hs.88145:AF030339
F-HEMBA1003497
   ESTs, Weakly similar to similar to zinc finger 5 protein from Gallus gallus, U51640 [H.sapiens]
   2.5e-09:303:63
   Hs.143723:H86048
F-HEMBA1003530
   Homo sapiens mRNA for ephrin-A2
   0.024:396:60
   Hs.158306:AJ007292
F-HEMBA1003602
   Homo sapiens DNA from chromosome 19, cosmid R29144
   0.0072:663:57
   Hs.155647:AC004221
F-HEMBA1003732
   ESTs
   1.0e-106:494:100
   Hs.157568:AI356515
F-HEMBA1003945
   Homo sapiens clone 638 unknown nRNA, complete sequence
   5.9e-78:310:93
   Hs.159515:AF091085
F-HEMBA1004007
   PROTO-ONCOGENE TYROSINE-PROTEIN KINASE SRC
   0.56:165:64
   Hs.115742:AF077754
F-HEMBA1004067
   Fatty acid synthase {3' region} [human, breast and HepG2 cells, mRNA Partial, 2237 nt]
   0.048:581:58
   Hs.83190:U29344
F-HEMBA1004085
   ESTs
   1.7e-15:92:98
   Hs.98138:AI183561
F-HEMBA1004110
   Homo sapiens intersectin short form mRNA, complete cds
   1.2e-159:779:96
   Hs.66392:AF064244
F-HEMBA1004250
   Human mRNA for KIAA0327 protein, complete cds
   2.1e-23:676:59
   Hs.149323:AB002325
F-HEMBA1004391
   NEURAL CELL ADHESION MOLECULE L1 PRECURSOR
   0.43:157:63
   Hs.1757:U52112
F-HEMBA1004444
   H.sapiens mRNA for gp25L2 protein
   1.5e-54:544:73
   Hs.159569:X90872
F-HEMBA1004454
   Homo sapiens tetraspan NET-4 mRNA, complete cds
   1.1e-05:230:62
   Hs.20709:AF065389
F-HEMBA1004505
   ESTs
   9.1e-61:345:93
   Hs.4814:AA631254
F-HEMBA1004785
   Homo sapiens Polycomb 2 homolog (hPc2) mRNA, complete cds
   3.7e-18:294:65
   Hs.123085:AF013956
F-HEMBA1004797
   ESTs
   3.9e-06:107:73
   Hs.42302:AI032142
F-HEMBA1004952
   Human cardiotrophin-1 (CTF1) mRNA, complete cds
   0.00021:175:68
   Hs.25537:U43030
F-HEMBA1004971
F-HEMBA1004982
   Human metabotropic glutamate receptor 8 mRNA, complete cds
   0.31:288:60
   Hs.86204:U92459
F-HEMBA1005070
   Human mRNA for KIAA0310 gene, complete cds
   7.9e-67:370:93
   Hs.5716:AB002308
F-HEMBA1005084
   Homo sapiens mRNA for KIAA0612 protein, partial cds
   0.00022:400:59
   Hs.112499:AB014512
F-HEMBA1005145
   Lymphocyte-activation gene 3
   3.4e-05:480:59
   Hs.74011:X51985
F-HEMBA1005230
   ESTs
   2.3e-103:481:99
   Hs.135112:AI090827
F-HEMBA1005246
   Homo sapiens ALR mRNA, complete cds
   2.0e-05:220:62
   Hs.153638:AF010403
F-HEMBA1005267
   ESTs
   5.6e-16:305:64
   Hs.125699:AA868017
F-HEMBA1005337
   EST
   2.1e-59:304:97
   Hs.48956:N64339
F-HEMBA1005430
   ESTs
   6.9e-19:333:65
   Hs.116567:AI332643
F-HEMBA1005449
   Human plectin (PLEC1) mRNA, complete cds
   0.026:576:56
   Hs.79706:U53204
F-HEMBA1005489
   Homo sapiens mRNA for KIAA0291 gene, partial cds
   0.14:551:59
   Hs.104717:AB006629
F-HEMBA1005522
   COAGULATION FACTOR VII PRECURSOR
   1.8e-12:298:64
   Hs.36989:M13232
F-HEMBA1005545
   MUSCARINIC ACETYLCHOLINE RECEPTOR M3
   2.6e-143:672:98
   Hs.7138:U29589
F-HEMBA1005698
   ESTs
   1.8e-124:611:97
   Hs.144441:AI338335
F-HEMBA1005913
F-HEMBA1005929
   H.sapiens mRNA for serine/threonine protein kinase EMK
   1.5e-86:847:72
   Hs.157199:X97630
F-HEMBA1005945
   ESTs, Weakly similar to F17E5.2 [C.elegans]
   4.2e-26:159:92
   Hs.126571:AI038963
F-HEMBA1006016
   ESTs
   1.3e-22:145:93
   Hs.33728:H97503
F-HEMBA1006171
F-HEMBA1006276
   Homo sapiens KIAA0412 mRNA, partial cds
   5.1e-19:371:65
   Hs.6200:AB007872
F-HEMBA1006299
F-HEMBA1006311
F-HEMBA1006335
   ESTs
   0.00021:327:62
   Hs.146044:AI089998
F-HEMBA1006357
   Homo sapiens secretory carrier mernbrane protein (SCAMP2) mRNA, complete cds
   7.4e-28:389:67
   Hs.10761:AF005038
F-HEMBA1006430
   ESTs
   9.7e-92:463:95
   Hs.143702:AI084062
F-HEMBA1006482
   Homo sapiens h-sco1 (SCO1) mRNA, nuclear gene encoding mitochondrial protein, complete cds
   6.2e-146:575:98
   Hs.14511:AF026852
F-HEMBA1006517
   ESTs
   3.6e-63:381:87
   Hs.11611:W21919
F-HEMBA1006544
   Homo sapiens suppressor of white apricot homolog 2 (SWAP2) mRNA, complete cds
   2.0e-50:503:76
   Hs.43543:AF042800
F-HEMBA1006572
   Calcium channel, voltage-dependent, P/Q type, alpha 1A subunit
   0.031:611:57
   Hs.96253:U79666
F-HEMBA1006658
   Homo sapiens mRNA for KIAA0687 protein, partial cds
   1.2e-128:646:95
   Hs.3628:AB014587
F-HEMBA1006707
   Homo sapiens mRNA for matrilin-4, partial
   1.7e-101:476:98
   Hs.129361:AJ007581
F-HEMBA1006724
   ESTs
   8.3e-86:450:95
   Hs.10056:AA210796
F-HEMBA1006749
   Homo sapiens mRNA for matrilin-4, partial
   6.1e-97:457:98
   Hs.129361:AJ007581
F-HEMBA1006770
   ESTs, Highly similar to BRAIN PROTEIN F41 [Mus musculus]
   1.6e-31:237:85
   Hs.31612:H41366
F-HEMBA1006902
   Homo sapiens mRNA for matrilin-4, partial
   9.4e-113:541:97
   Hs.129361:AJ007581
F-HEMBA1006912
   ESTs
   1.4e-94:460:97
   Hs.88672:AA279956
F-HEMBA1006916
   Homo sapiens Grb14 mRNA, complete cds
   5.2e-120:651:92
   Hs.83070:L76687
F-HEMBA1006960
   Human DNA binding protein FKHL15 (FKHL15) mRNA, complete cds
   0.011:628:57
   Hs.159234:U89995
F-HEMBA1007013
   ESTs
   2.6e-05:139:69
   Hs.113817:AA702497
F-HEMBA1007057
   Homo sapiens hLRp105 mRNA for LDL receptor related protein 105, complete cds
   7.5e-12:389:64
   Hs.143641:AB009462
F-HEMBA1007063
F-HEMBA1007226
   ESTs
   1.8e-35:202:94
   Hs.105140:N32669
F-HEMBA1007241
   ESTs, Weakly similar to No definition line found [C.elegans]
   4.1e-27:361:67
   Hs.114062:AI421699
F-HEMBA1007291
   ESTs
   0.96:114:69
   Hs.121411:AA770241
F-HEMBA1007332
   ESTs, Weakly similar to hTAFII100 [H.sapiens]
   2.5e-81:405:97
   Hs.3727:AA205887
F-HEMBB1000106
   ESTs
   2.2e-76:393:96
   Hs.151874:AI023405
F-HEMBB1000276
   EST
   0.81:239:63
   Hs.149811:AI286277
F-HEMBB1000309
   Homo sapiens zinc finger protein (MBLL) mRNA, complete cds
   2.4e-35:180:100
   Hs.44806:AF061261
F-HEMBB1000407
   Cyclin-dependent kinase inhibitor 1C (p57, Kip2)
   0.026:218:65
   Hs.106070:U22398
F-HEMBB1000447
   Homo sapiens JWA protein mRNA, complete cds
   4.6e-160:750:98
   Hs.92384:AF070523
F-HEMBB1000542
   ESTs, Weakly similar to C01H6.7 [C.elegans]
   6.8e-07:130:77
   Hs.18171:AA524327
F-HEMBB1000567
   ESTs
   8.8e-13:271:71
   Hs.19934:AA455673
F-HEMBB1000642
F-HEMBB1000668
   EST
   0.83:192:58
   Hs.126372:AA912193
F-HEMBB1000679
   H.sapiens mRNA for TRAMP protein
   4.1e-96:727:80
   Hs.4147:X63679
F-HEMBB1000881
   Homo sapiens chromosome 4p homeobox mRNA sequence
   2.2e-06:512:60
   Hs.104134:M99587
F-HEMBB1000905
   Homo sapiens mRNA for voltage gated potassium channel
   0.93:337:58
   Hs.4975:Y15065
F-HEMBB1001026
   Human p76 mRNA, complete cds
   6. le-08:410:61
   Hs.28757:U81006
F-HEMBB1001048
   Human Hpast (HPAST) mRNA, complete cds
   2.1e-56:524:75
   Hs.155119:AF001434
F-HEMBB 1001200
   EST
   0.10:300:61
   Hs.161647:AA133367
F-HEMBB1001407
   Homo sapiens PRKY exon 1 and joined CDS
   2.6e-40:271:81
   Hs.56336:Y15801
F-HEMBB1001530
   ESTs
   1.2e-98:477:98
   Hs.135208:AI093908
F-HEMBB1001547
F-HEMBB1001573
   EST
   2.2e-06:115:75
   Hs.138275:R43976
F-HEMBB1001847
   ESTs
   5.3e-79:389:98
   Hs.16141:W56079
F-HEMBB1001959
   Homo sapiens clone 24781 mRNA sequence
   1.0e-58:322:93
   Hs.108112:AF070640
F-HEMBB1001978
   EST
   4.7e-23:245:74
   Hs.136356:AA493225
F-HEMBB1002039
   EST
   2.3e-25:345:70
   Hs.128248:AA972858
F-HEMBB1002041
   Human plectin (PLEC1) mRNA, complete cds
   2.2e-08:477:60
   Hs.79706:U53204
F-HEMBB1002051
   Homo sapiens E74-like factor 5 (ELF5) mRNA, complete cds
   9.9e-97:454:99
   Hs.159267:AF049703
F-HEMBB1002120
   ESTs
   7.6e-10:68:100
   Hs.146335:AI262660
F-HEMBB1002162
   Homo sapiens genethonin 1 mRNA, complete cds
   2.2e-68:328:99
   Hs.109590:AF062534
F-HEMBB1002228
   Homo sapiens unknown mRNA, complete cds
   5.3e-41:208:98
   Hs.11441:AF047439
F-HEMBB1002245
   Human growth/differentiation factor 1 (GDF-1) mRNA, complete cds
   5.6e-05:299:63
   Hs.92614:M62302
F-HEMBB1002302
F-HEMBB1002427
   Homo sapiens GN6ST mRNA for long form of N-acetylglucosamine-6-O-sulfotransferase (GlcNAc6ST), complete cds
   0.84:108:68
   Hs.8786:AB014680
F-HEMBB1002465
   ESTs, Highly similar to ACYL-COA DEHYDROGENASE [Bacillus subtilis]
   3.2e-18:159:84
   Hs.14791:AA741056
F-HEMBB1002661
   ESTs
   0.023:424:55
   Hs.154029:AI380603
F-HEMBB1002663
F-HEMBB1002693
F-MAMMA1000046
   Human mRNA for tryptophan hydroxylase (EC 1.14.16.4)
   3.2e-43:454:74
   Hs.144563:AF057280
F-MAMMA1000102
   Homo sapiens mRNA for cathepsin V, complete cds
   0.70:222:65
   Hs.87417:AB001928
F-MAMMA1000106
   Homo sapiens mRNA for KIAA0754 protein, partial cds
   0.00076:331:61
   Hs.159183:AB018297
F-MAMMA1000118
   B94 PROTEIN
   1.5e-07:511:61
   Hs.75522:M92357
F-MAMMA1000141
   ESTs
   2.3e-18:268:73
   Hs.155334:AA827904
F-MAMMA1000204
   Homo sapiens dysferlin mRNA, complete cds
   2.5e-167:781:98
   Hs.143897:AF075575
F-MAMMA1000226
   Human involucrin mRNA
   0.0010:414:61
   Hs.157091:M13903
F-MAMMA1000403
   ESTs
   2.0e-24:163:90
   Hs.44281:AI342377
F-MAMMA1000449
   ESTs
   0.99:211:60
   Hs.143715:AI167929
F-MAMMA1000457
   NADH-CYTOCHROME B5 REDUCTASE
   7.7e-37:551:66
   Hs.75666:M28713
F-MAMMA1000473
F-MAMMA1000496
   Homo sapiens PAC clone DJ130H16 from 22q12.1-qter
   1.1e-107:543:96
   Hs.8003:AC004997
F-MAMMA1000528
   EST
   0.22:227:59
   Hs.161400:AI423879
F-MAMMA1000591
   H.sapiens mRNA for UDP-GalNAc:polypeptide N-acetylgalactosaminyl transferase
   3.3e-23:470:62
   Hs.55823:X92689
F-MAMMA1000614
   H.sapiens mRNA for CCAAT/enhancer binding protein alpha
   1.9e-06:492:61
   Hs.76171:Y11525
F-MAMMA1000652
   Homo sapiens mRNA, chromosome I specific transcript KIAA0487
   1.5e-61:449:75
   Hs.92381:AB007956
F-MAMMA1000681
   Homo sapiens mRNA for putative G-protein coupled receptor, EDG6
   4.0e-34:636:65
   Hs.159543:AJ000479
F-MAMMA1000706
   COAGULATION FACTOR VII PRECURSOR
   9.7e-16:378:65
   Hs.36989:M13232
F-MAMMA1000788
   ESTs, Weakly similar to M01E11.2 [C.elegans]
   3.4e-118:571:97
   Hs.78389:AI191127
F-MAMMA1000810
   EST
   0.065:211:61
   Hs.116798:AA633813
F-MAMMA1000814
   EST
   3.1e-08:224:66
   Hs.141620:N63316
F-MAMMA1000881
   Homo sapiens sgk gene
   3.5e-08:165:69
   Hs.159640:AJ000512
F-MAMMA1000986
   Homo sapiens clone 24796 mRNA sequence
   2.3e-115:320:99
   Hs.27191:AF070596
F-MAMMA1000994
   Human HOX4C mRNA for a homeobox protein
   0.050:178:64
   Hs.74061:X59372
F-MAMMA1001043
   Latent transforming growth factor beta binding protein 2
   0.0013:376:60
   Hs.83337:Z37976
F-MAMMA1001066
   ESTs
   1.1e-18:128:77
   Hs.114031:AA700958
F-MAMMA1001094
   Homo sapiens clone 243 unknown mRNA, complete sequence
   2.0e-182:844:99
   Hs.20423:AF091094
F-MAMMA1001141
   Homo sapiens achaete scute homologous protein (ASH1) mRNA, complete cds
   6.1e-07:492:58
   Hs.1619:L08424
F-MAMMA1001150
   Protein kinase C, mu
   8.3e-51:691:67
   Hs.2891:X75756
F-MAMMA1001237
   Homo sapiens monocarboxylate transporter (MCT3) mRNA, complete cds
   8.2e-08:386:60
   Hs.85838:U81800
F-MAMMA1001284
   ESTs
   1.1e-91:452:97
   Hs.114756:AI279440
F-MAMMA1001310
   Homo sapiens mRNA for KIAA0708 protein, partial cds
   0.014:512:57
   Hs.117177:AB014608
F-MAMMA1001344
   ESTs, Weakly similar to No definition line found [C.elegans]
   8.3e-80:406:96
   Hs.121619:AI188389
F-MAMMA1001418
   Human Na+/nucleoside cotransporter (hCNT1a) mRNA, complete cds
   1.9e-36:622:63
   Hs.97207:U62966
F-MAMMA1001532
   Human kruppel-related zinc finger protein (ZNF184) mRNA, partial cds
   2.1e-33:282:68
   Hs.158174:U66561
F-MAMMA1001609
   Insulin-like growth factor-binding protein 4
   0.00026:596:57
   Hs.1516:U20982
F-MAMMA1001615
   Homo sapiens DNA from chromosome 19, cosmid R29144
   1.1e-05:504:59
   Hs.155647:AC004221
F-MAMMA1001623
   Excision repair protein ERCC6
   1.2e-38:274:86
   Hs.99924:L04791
F-MAMMA1001634
   ESTs
   1.5e-26:176:90
   Hs.16187:AI139901
F-MAMMA1001893
   Cyclin-dependent kinase inhibitor 1C (p57, Kip2)
   0.00030:170:68
   Hs.106070:U22398
F-MAMMA1001901
   ESTs
   1.5e-36:201:76
   Hs.161660:AA167744
F-MAMMA1001957
   Prostaglandin I2 (prostacyclin) receptor (IP)
   0.041:277:61
   Hs.393:D38128
F-MAMMA1001978
   EST
   4.0e-43:359:81
   Hs.136494:AA587773
F-MAMMA1002070
   Human PAC clone DJ515N1 from 22q11.2-q22
   5.1e-135:652:97
   Hs.26670:AC002073
F-MAMMA1002080
   Calcium channel, voltage-dependent, L type, alpha 1C subunit
   0.0019:574:57
   Hs.89925:L04569
F-MAMMA1002087
   Human mRNA for KIAA0009 gene, complete cds
   0.71:228:63
   Hs.79972:D13634
F-MAMMA1002091
   Homo sapiens CD39L2 (CD39L2) mRNA, complete cds
   5.2e-158:743:98
   Hs.12330:AF039916
F-MAMMA1002095
   Homo sapiens mRNA for KIAA0703 protein, complete cds
   4.9e-55:657:68
   Hs.6168:AB014603
F-MAMMA1002128
   Human leucine zipper on the D14S46E locus mRNA, complete cds
   0.77:449:59
   Hs.89606:M95925
F-MAMMA1002142
F-MAMMA1002165
   Homo sapiens connective tissue growth factor-like protein precursor, mRNA, complete cds
   1.2e-35:182:98
   Hs.139340:AF083500
F-MAMMA1002205
   ESTs
   4.7e-32:385:71
   Hs.46158:AI160121
F-MAMMA1002224
   TRANSCRIPTION INITIATION FACTOR IIE, ALPHA SUBUNIT
   1.3e-34:248:85
   Hs.3006:X63468
F-MAMMA1002234
   ESTs
   1.1e-100:501:97
   Hs.158161:AA312511
F-MAMMA1002586
   Human mRNA for KIAA0183 gene, partial cds
   0.00041:388:61
   Hs.76666:D80005
F-MAMMA1002633
   Landsteiner-Wiener blood group glycoprotein
   1.1e-37:477:71
   Hs.108287:L27670
F-MAMMA1003126
   Human Hpast (HPAST) mRNA, complete cds
   4.1e-84:801:74
   Hs.155119:AF001434
F-NT2RM1000407
   ESTs
   4.1e-19:132:92
   Hs.133484:D80522
F-NT2RM1000462
F-NT2RM1000542
   Beta-galactosidase (GLB1)
   1.3e-17:436:61
   Hs.79222:M34423
F-NT2RM1000580
   ESTs, Weakly similar to similar to M. musculus MER5 and other AHPC/TSA proteins [C.elegans]
   6.2e-51:254:98
   Hs.132096:AA314601
F-NT2RM1000789
   Homo sapiens mRNA for hTCF-4
   3.5e-96:299:92
   Hs.154485:Y11306
F-NT2RM1000855
   Hydroxysteroid (11-beta) dehydrogenase 2
   0.021:178:67
   Hs.1376:U26726
F-NT2RM1000858
F-NT2RM1000899
   Homo sapiens BAC clone RG119C02 from 7p15
   0.037:222:63
   Hs.22900:AC004520
F-NT2RM2000241
   ESTs
   2.9e-31:166:97
   Hs.156175:AI334328
F-NT2RM2000306
F-NT2RM2000410
   ESTs
   3.2e-12:81:97
   Hs.72116:AA151564
F-NT2RM2000423
   Beta-galactosidase (GLB1)
   0.074:163:63
   Hs.79222:M34423
F-NT2RM2000497
   ESTs, Weakly similar to CHL1 protein [H.sapiens]
   3.7e-21:121:97
   Hs.97515:AA435715
F-NT2RM2000514
F-NT2RM2000565
F-NT2RM2000582
   EST
   1.7e-42:218:98
   Hs.160262:AI146610
F-NT2RM2000589
F-NT2RM2000622
   Androgen receptor (dihydrotestosterone receptor; testicular feminization; spinal and bulbar muscular atrophy; Kennedy disease)
   0.00018:409:62
   Hs.99915:M23263
F-NT2RM2000632
   Homo sapiens TBP-associated factor 172 (TAF-172) mRNA, complete cds
   0.00017:331:59
   Hs.14244:AF038362
F-NT2RM2000773
   Human zinc finger protein (MAZ) rnRNA
   7.2e-47:274:91
   Hs.7647:M94046
F-NT2RM2001126
   Homo sapiens multi PDZ domain protein MUPP1 (MUPP1) mRNA, complete cds
   5.1e-163:663:99
   Hs.21301:AF093419
F-NT2RM2001558
   Homo sapiens protein kinase A binding protein AKAP110 mRNA, complete cds
   3.9e-166:770:98
   Hs.98397:AF093408
F-NT2RM2001626
   Human mRNA for KIAA0231 gene, partial cds
   2.8e-40:562:67
   Hs.7938:D86984
F-NT2RM2001643
   ESTs
   7.9e-112:548:97
   Hs.12610:W56112
F-NT2RM2001738
   FACTOR VIII INTRON 22 PROTEIN
   0.32:452:59
   Hs.83363:M34677
F-NT2RM2001767
   Homo sapiens mRNA for B120, complete cds
   5.0e-24:131:100
   Hs.123090:AB001895
F-NT2RM2001792
   Homo sapiens mRNA for serum lectin P35, complete cds
   8.2e-14:244:67
   Hs.54517:D63160
F-NT2RM2001818
   EST
   0.051:152:61
   Hs.157619:AI357718
F-NT2RM2001902
   Human p21-activated protein kinase (Pak1) gene, complete cds
   4.4e-39:568:66
   Hs.62402:U24152
F-NT2RM2001939
   Human G protein-coupled receptor GPR-NGA gene, complete cds
   4.2e-141:664:98
   Hs.92458:U55312
F-NT2RM2001941
   Dopamine receptor D4
   1.3e-14:547:61
   Hs.99922:L12398
F-NT2RM4000100
   Human involucrin mRNA
   1.1e-09:487:62
   Hs.157091:M13903
F-NT2RM4000115
F-NT2RM4000198
   ESTs
   9.3e-101:496:98
   Hs.128676:AA464413
F-NT2RM4000284
   Human IgG Fc receptor hFcRn mRNA, complete cds
   2.4e-38: 194:98
   Hs.110804:U12255
F-NT2RM4000295
   Homo sapiens SOX22 protein (SOX22) mRNA, complete cds
   1.7e-06:479:60
   Hs.43627:U35612
F-NT2RM4000326
   Phosphorylase kinase, gamma 2 (testis)
   0.95:204:63
   Hs.87452:M31606
F-NT2RM4000417
   H.sapiens Syt V gene (genomic and cDNA sequence)
   0.97:143:67
   Hs.23179:X96783
F-NT2RM4000444
   Eukaryotic translation elongation factor 1 delta (guanine nucleotide exchange protein)
   0.45:194:64
   Hs.90319:Z21507
F-NT2RM4000587
   Human proto-oncogene (FRATI) gene, complete cds
   3.8e-05:495:60
   Hs.143005:U58975
F-NT2RM4000593
F-NT2RM4000648
   Homo sapiens glypican-4 (GPC4) mRNA, complete cds
   1.0e-50:610:70
   Hs.58367:AF030186
F-NT2RM4000761
   EST
   0.89:53:79
   Hs.161967:AA494423
F-NT2RM4000965
   H.sapiens mRNA for PHAPI2b protein
   0.18:148:68
   Hs.84264:U70439
F-NT2RM4000997
F-NT2RM4001321
   ESTs
   1.8e-94:467:97
   Hs.12610:W56112
F-NT2RM4001325
   Homo sapiens mRNA for chondroitin 6-sulfotransferase, complete cds
   2.1e-13:384:64
   Hs.158304:AB012192
F-NT2RM4001377
   Homo sapiens mRNA for KIAA0638 protein, partial cds
   3.1e-156:719:99
   Hs.77864:AB014538
F-NT2RM4001735
F-NT2RM4001768
   ESTs
   0.00012:123:68
   Hs.128045:AA970231
F-NT2RM4001843
F-NT2RM4002352
   Homo sapiens hLRp105 mRNA for LDL receptor related protein 105, complete cds
   4.4e-157:761:97
   Hs.143641:AB009462
F-NT2RP1000002
   EST
   0.00023:170:68
   Hs.135504:AI091717
F-NT2RP1000050
   Histidine-rich calcium binding protein
   0.0047:257:61
   Hs.1480:M60052
F-NT2RP1000181
   Homo sapiens chromosome 11, BAC CIT-HSP-311e8 (BC269730) containing the hFEN1 gene
   6.9e-99:510:94
   Hs.132898:AC004770
F-NT2RP1000239
   ESTs
   1.7e-34:240:67
   Hs.33020:N31946
F-NT2RP1000261
   ESTs, Weakly similar to HYPOTHETICAL 25.2 KD PROTEIN IN ACB1-KSS1 INTERGENIC REGION [S.cerevisiae]
   9.1e-92:484:94
   Hs.7870:AI078137
F-NT2RP1000271
   Homo sapiens DNA-binding protein mRNA, complete cds
   1.4e-140:678:97
   Hs.137582:AF038951
F-NT2RP1000300
   Human endosome-associated protein (EEA1) mRNA, complete cds
   1.0:205:61
   Hs.2864:L40157
F-NT2RP1000325
   Phosphate carrier, mitochondrial
   7.7e-84:444:93
   Hs.78713:X60036
F-NT2RP1000448
   ESTs
   9.5e-73:405:93
   Hs.24054:N46499
F-NT2RP1000465
   ESTs
   8.5e-10:81:87
   Hs.18619:AI202769
F-NT2RP1000468
   Homo sapiens clone 24781 mRNA sequence
   2.1e-20:133:92
   Hs.108112:AF070640
F-NT2RP1000551
   Homo sapiens putative interferon-related protein (SM15) mRNA, partial cds
   2.4e-140:742:93
   Hs.75402:U09585
F-NT2RP1000579
   SUCCINATE DEHYDROGENASE
   1.1e-141:798:91
   Hs.469:L21936
F-NT2RP1000613
   Homo sapiens carbonic anhydrase precursor (CA 12) mRNA, complete cds
   5.5e-11:468:58
   Hs.5338:AF037335
F-NT2RP1000679
   ESTs
   0.79:127:65
   Hs.146093:AA100242
F-NT2RP1000740
   Homo sapiens Trio isoform mRNA, complete cds
   0.24:160:66
   Hs.150625:AF091395
F-NT2RP1000903
F-NT2RP1000981
F-NT2RP1001004
   Human mRNA for Doc2 beta, complete cds
   0.00072:520:57
   Hs.54402:D70830
F-NT2RP1001020
   ESTs, Weakly similar to SPORULATION-SPECIFIC PROTEIN 1 [Saccharomyces cerevisiae]
   2.1e-73:392:94
   Hs.4789:AI418298
F-NT2RP1001031
   Miller-Dieker syndrome chromosome region
   4.5e-07:383:61
   Hs.77318:L13385
F-NT2RP1001563
   Human eukaryotic translation initiation factor (eIF3) mRNA, complete cds
   0.086:398:59
   Hs.57783:U78525
F-NT2RP2000092
   Zinc finger protein 136 (clone pHZ-20)
   5.5e-56:652:70
   Hs.69740:U09367
F-NT2RP2000178
   Homo sapiens chromosome 5, BAC clone 203o13 (LBNL H155), complete sequence
   0.14:231:62
   Hs.159402:AC005609
F-NT2RP2000240
   Homo sapiens KIAA0415 mRNA, complete cds
   3.0e-61:554:76
   Hs.7289:AB007875
F-NT2RP2000394
   ESTs
   0.0063:210:63
   Hs.134272:AI220363
F-NT2RP2000447
   Human (clone SY11) golgin-95 mRNA, complete cds
   3.8e-22:498:65
   Hs.24049:L06147
F-NT2RP2000479
   ESTs
   1.3e-46:298:90
   Hs.15641:W63676
F-NT2RP2000514
   Homo sapiens roundabout 1 (robo1) mRNA, complete cds
   1.2e-37:543:67
   Hs.36702:AF040990
F-NT2RP2000533
   ESTs, Highly similar to HYPOTHETICAL 16.3 KD PROTEIN IN DUR1,2-NGR1 INTERGENIC REGION [Saccharomyces cerevisiae]
   5.4e-132:647:96
   Hs.18120:AA913148
F-NT2RP2000610
   Homo sapiens antigen NY-CO-16 mRNA, complete cds
   0.00027:182:66
   Hs.132206:AF039694
F-NT2RP2000616
   ESTs
   0.44:235:60
   Hs.31714:AA514389
F-NT2RP2000649
   Homo sapiens mRNA for Hs Ste24p, complete cds
   6.2e-167:802:97
   Hs.25846:AB016068
F-NT2RP2000663
   Homo sapiens mRNA for KIAA0512 protein, complete cds
   4.8e-15:305:64
   Hs.48924:AB011084
F-NT2RP2000694
   H.sapiens 5T4 gene for 5T4 Oncofetal antigen
   1.0e-113:558:96
   Hs.82128:AJ012159
F-NT2RP2000712
   ESTs, Weakly similar to ZINC FINGER PROTEIN 91 [H.sapiens]
   1.5e-83:442:93
   Hs.154226:AA468767
F-NT2RP2000739
   Human mRNA for KIAA0326 gene, partial cds
   2.1e-25:574:62
   Hs.6833:AB002324
F-NT2RP2000818
F-NT2RP2000903
   H.sapiens 5T4 gene for 5T4 Oncofetal antigen
   3.5e-112:539:97
   Hs.82128:AJ012159
F-NT2RP2001200
   Homo sapiens mRNA for KIAA0676 protein, partial cds
   1.1e-111:540:96
   Hs.115763:AB014576
F-NT2RP2001223
   ESTs
   5.9e-91:461:95
   Hs.103733:AA436929
F-NT2RP2001276
   Homo sapiens mRNA for KIAA0634 protein, partial cds
   2.4e-11:382:62
   Hs.30898:AB014534
F-NT2RP2001388
F-NT2RP2001469
   ESTs
   7.3e-39:213:95
   Hs.151001:AA564706
F-NT2RP2001480
   Homo sapiens thrombospondin 3 (THBS3) gene, complete cds
   2.9e-141:686:96
   Hs.82165:L38969
F-NT2RP2001495
   Human transporter protein (g17) mRNA, complete cds
   6.0e-37:581:64
   Hs.76460:U49082
F-NT2RP2001514
F-NT2RP2001529
   Homo sapiens mRNA for ZIP-kinase, complete cds
   1.5e-153:757:96
   Hs.25619:AB007144
F-NT2RP2001538
   ESTs, Highly similar to co-repressor protein [M.musculus]
   4.4e-63:329:94
   Hs.22583:AA188168
F-NT2RP2001562
   Homo sapiens GLE1 (GLE1) mRNA, complete cds
   7.5e-119:572:97
   Hs.81449:AF058922
F-NT2RP2001662
   H.sapiens mRNA for TGIF protein
   2.6e-29:448:67
   Hs.90077:X89750
F-NT2RP2001755
   ESTs, Highly similar to F-SPONDIN PRECURSOR [Rattus norvegicus]
   1.0e-47:275:92
   Hs.153657:H37929
F-NT2RP2001769
   Human protein kinase C-L (PRKCL) mRNA, complete cds
   1.9e-09:399:59
   Hs.89616:M55284
F-NT2RP2001817
   EST
   0.97:133:63
   Hs.145274:AI249468
F-NT2RP2001878
   Human DNA binding protein FKHL15 (FKHL15) mRNA, complete cds
   3.6e-05:491:60
   Hs.159234:U89995
F-NT2RP2001903
   Human mRNA for apolipoprotein E receptor 2, complete cds
   0.0023:270:60
   Hs.54481:D86407
F-NT2RP2001915
   Homo sapiens Pig3 (PIG3) mRNA complete cds
   3.2e-05:493:60
   Hs.50649:AF010309
F-NT2RP2001921
F-NT2RP2001948
   ESTs
   0.55:213:61
   Hs.147805:AI221717
F-NT2RP2001956
   ESTs, Weakly similar to HYPOTHETICAL 25.2 KD PROTEIN IN ACB1-KSS1 INTERGENIC REGION [S.cerevisiae]
   8.1e-45:510:70
   Hs.13144:T67556
F-NT2RP2002015
   ESTs
   4.3e-20:127:92
   Hs.12610:W56112
F-NT2RP2002063
   ESTs
   1.0e-08:73:91
   Hs.19814:T81721
F-NT2RP2002188
F-NT2RP2002232
   EST
   0.82:99:67
   Hs.148596:AI202232
F-NT2RP2002304
   Human monocytic leukaemia zinc finger protein (MOZ) mRNA, complete cds
   0.031:107:71
   Hs.82210:U47742
F-NT2RP2002409
   Homo sapiens lymphocyte secreted C-type lectin precursor, mRNA, complete cds
   0.00063:302:65
   Hs.105927:AF020044
F-NT2RP2002510
   ABO blood group (transferase A, alpha 1-3-N-acetylgalactosaminyltransferase; transferase B, alpha 1-3-galactosyltransferase)
   4.4e-09:298:64
   Hs.144023:U15197
F-NT2RP2002527
   Homo sapiens chromosome 11, BAC CIT-HSP-311e8 (BC269730) containing the hFEN1 gene
   5.2e-65:327:96
   Hs.132898:AC004770
F-NT2RP2002533
   Homo sapiens putative tumor suppressor gene 26 protein alpha 2 delta calcium channel subunit mRNA, complete cds
   2.1e-142:726:95
   Hs.127436:AF040709
F-NT2RP2002564
   Homo sapiens mRNA for repressor protein, partial cds
   3.5e-55:594:74
   Hs.58167:D30612
F-NT2RP2002674
   Epoxide hydrolase 2, cytoplasmic
   2.5e-07:332:62
   Hs.113:L05779
F-NT2RP2002721
F-NT2RP2002824
   ESTs, Weakly similar to ZK858.6 [C.elegans]
   5.2e-28:190:90
   Hs.120416:AA057428
F-NT2RP2002942
   Homo sapiens mRNA for KIAA0806 protein, complete cds
   2.0e-146:758:94
   Hs.24279:AB018349
F-NT2RP2002974
   ESTs
   4.9e-51:475:77
   Hs.137840:AI123378
F-NT2RP2002976
   ESTs, Weakly similar to No definition line found [C.elegans]
   7.8e-50:315:89
   Hs.159604:AI380827
F-NT2RP2003042
   Lecithin-cholesterol acyltransferase
   2.4e-25:454:65
   Hs.112125:M12625
F-NT2RP2003138
   H.sapiens mRNA for TGIF protein
   2.0e-05:121:75
   Hs.90077:X89750
F-NT2RP2003179
   Homo sapiens mRNA for KIAA0537 protein, complete cds
   1.0e-43:587:70
   Hs.12836:AB011109
F-NT2RP2003210
F-NT2RP2003302
   Zinc finger protein 136 (clone pHZ-20)
   1.8e-64:691:69
   Hs.69740:U09367
F-NT2RP2003369
   Homo sapiens chromosome 7q22 sequence
   5.1e-109:539:96
   Hs.125742:AF053356
F-NT2RP2003383
   Homo sapiens mRNA for KIAA0458 protein, complete cds
   1.6e-159:801:95
   Hs.7414:AB007927
F-NT2RP2003390
   Homo sapiens SEC63 (SEC63) mRNA, complete cds
   2.2e-116:554:98
   Hs.31575:AF100141
F-NT2RP2003469
   ESTs
   0.26:127:69
   Hs.62649:AA115328
F-NT2RP2003545
   ESTs, Highly similar to SERINE/THREONINE-PROTEIN KINASE PAK [Rattus norvegicus]
   4.2e-111:550:96
   Hs.85768:W16504
F-NT2RP2003593
   EST
   8.7e-43:213:99
   Hs.130657:AI005473
F-NT2RP2003599
   ESTs
   7.8e-14:84:98
   Hs.107171:H53973
F-NT2RP2003655
F-NT2RP2003664
   Homo sapiens mRNA for leptin receptor gene-related protein
   5.4e-134:630:98
   Hs.23581:Y12670
F-NT2RP2003931
   Human mRNA for KIAA0365 gene, partial cds
   4.3e-14:101:92
   Hs.84123:AB002363
F-NT2RP2003940
   Zinc finger protein 43 (HTF6)
   4.6e-99:693:82
   Hs.74107:X59244
F-NT2RP2003950
   Cell division cycle 25A
   0.00041:419:59
   Hs.1634:M81933
F-NT2RP2004069
   ESTs, Weakly similar to HYPOTHETICAL 26.4 KD PROTEIN EEED8.8 IN CHROMOSOME II [C.elegans]
   1.3e-75:390:94
   Hs.13322:AA151730
F-NT2RP2004108
   Zinc finger protein 136 (clone pHZ-20)
   4.9e-69:548:78
   Hs.69740:U09367
F-NT2RP2004141
   TRICHOHYALIN
   4.8e-11:435:63
   Hs.82276:L09190
F-NT2RP2004179
   ESTs
   0.0054:180:66
   Hs.134917:AI092952
F-NT2RP2004205
   Homo sapiens nuclear dual-specificity phosphatase (SBF1) mRNA, partial cds
   0.27:474:56
   Hs.112049:U93181
F-NT2RP2004447
   Homo sapiens LDL receptor member LR3 mRNA, complete cds
   0.016:456:57
   Hs.6347:AF077820
F-NT2RP2004495
   Human transporter protein (g17) mRNA, complete cds
   1.2e-26:497:61
   Hs.76460:U49082
F-NT2RP2004524
   Human bone morphogenetic protein-3b
   0.0016:259:64
   Hs.2171:D49493
F-NT2RP2004556
   ESTs
   1.1e-34:181:97
   Hs.27160:AA421991
F-NT2RP2004606
   Tissue inhibitor of metalloproteinase 1 (erythroid potentiating activity, collagenase inhibitor)
   5.7e-107:587:92
   Hs.148726:X03124
F-NT2RP2004648
   TUBULIN ALPHA-4 CHAIN
   0.59:186:61
   Hs.75318:X06956
F-NT2RP2004670
   Human mRNA for KIAA0369 gene, complete cds
   0.097:309:61
   Hs.21355:AB002367
F-NT2RP2004794
   ESTs
   1.3e-60:310:96
   Hs.84926:N50073
F-NT2RP2004837
F-NT2RP2004847
   Zinc finger protein 42 (myeloid-specific retinoic acid-responsive)
   1.4e-05:396:60
   Hs.78247:M58297
F-NT2RP2005027
   GLUCOSE TRANSPORTER TYPE 3, BRAIN
   7.2e-147:713:96
   Hs.7594:M20681
F-NT2RP2005069
   Human mRNA for KIAA0355 gene, complete cds
   0.14:303:61
   Hs.153014:AB002353
F-NT2RP2005163
   ESTs, Weakly similar to No definition line found [C.elegans]
   1.4e-23:334:70
   Hs.159604:AI380827
F-NT2RP2005181
   Ecotropic retroviral receptor
   8.3e-45:501:70
   Hs.2928:X57303
F-NT2RP2005247
   Oxysterol binding protein
   4.2e-08:356:62
   Hs.143065:M86917
F-NT2RP2005378
   ESTs
   1.7e-100:485:97
   Hs.151572:AA588083
F-NT2RP2005391
   EST
   1.0:264:62
   Hs.148259:AA905706
F-NT2RP2005425
   Homo sapiens mRNA for KIAA0803 protein, partial cds
   3.3e-118:566:97
   Hs.58103:AB018346
F-NT2RP2005463
F-NT2RP2005514
   ESTs
   3.6e-18:193:77
   Hs.153344:R26293
F-NT2RP2005535
   Homo sapiens DNA-binding protein mRNA, complete cds
   7.5e-127:726:90
   Hs.137582:AF038951
F-NT2RP2005541
   Glucosamine (N-acetyl)-6-sulfatase (Sanfilippo disease IIID)
   1.2e-06:225:64
   Hs.2703:Z12173
F-NT2RP2005597
F-NT2RP2005632
   ESTs
   5.6e-67:344:96
   Hs.112011:AA987961
F-NT2RP2005666
   ESTs
   5.8e-71:453:87
   Hs.122698:AI042484
F-NT2RP2005774
   Zinc finger protein 136 (clone pHZ-20)
   1.3e-45:451:74
   Hs.69740:U09367
F-NT2RP2005878
   ESTs, Highly similar to ESTRADIOL 17 BETA-DEHYDROGENASE 3 [Homo sapiens]
   5.9e-10:67:100
   Hs.104523:AA584520
F-NT2RP2005883
F-NT2RP2005887
F-NT2RP2005941
   Human novel homeobox mRNA for a DNA binding protein
   6.2e-11:464:61
   Hs.37035:U07664
F-NT2RP2005994
F-NT2RP2006004
   Homo sapiens KIAA0405 mRNA, complete cds
   1.2e-13:273:63
   Hs.48998:AB007865
F-NT2RP2006042
   Human mRNA for KIAA0144 gene, complete cds
   5.6e-12:220:69
   Hs.8127:D63478
F-NT2RP2006092
   Human FE65-like protein (hFE65L) mRNA, partial cds
   2.6e-23:353:65
   Hs.24957:U62325
F-NT2RP2006099
   EST
   2.5e-28:180:90
   Hs.160878:AI361890
F-NT2RP2006134
   Neogenin (chicken) homolog 1
   0.035:219:60
   Hs.90408:U61262
F-NT2RP2006269
   Homo sapiens mRNA for matrilin-3
   1.0:147:65
   Hs.119534:AJ224741
F-NT2RP2006512
   ESTs
   1.6e-09:70:95
   Hs.118981:AA282396
F-NT2RP3000011
F-NT2RP3000022
   EST
   0.016:293:60
   Hs.127706:AA961478
F-NT2RP3000059
   Human SH3 domain-containing proline-rich kinase (sprk) mRNA, complete cds
   0.0041:608:59
   Hs.89449:L32976
F-NT2RP3000063
   Excision repair protein ERCC6
   1.0:264:59
   Hs.99924:L04791
F-NT2RP3000125
   Human mRNA for KIAA0314 gene, partial cds
   6.9e-08:379:59
   Hs.155045:AB002312
F-NT2RP3000148
   Human Chromosome 16 BAC clone CIT987SK-A-635H12
   4.5e-40:349:73
   Hs.108604:AC002310
F-NT2RP3000169
   Homo sapiens MRS1 mRNA, complete cds
   1.1e-107:501:99
   Hs.30985:AF093239
F-NT2RP3000171
   Homo sapiens methionine synthase reductase (MTRR) mRNA, complete cds
   1.0:279:64
   Hs.153792:AF025794
F-NT2RP3000172
   Homo sapiens mRNA for ZIP-kinase, complete cds
   7.4e-09:463:59
   Hs.25619:AB007144
F-NT2RP3000201
   Homo sapiens mRNA for KIAA0687 protein, partial cds
   3.0e-171:792:98
   Hs.3628:AB014587
F-NT2RP3000232
   ESTs, Weakly similar to ZINC FINGER PROTEIN 91 [H.sapiens]
   8.6e-24:304:70
   Hs.112094:AA447558
F-NT2RP3000304
   Homo sapiens LDL receptor-related protein 6 (LRP6) mRNA, complete cds
   1.1e-172:797:98
   Hs.23672:AF074264
F-NT2RP3000378
   Homo sapiens mRNA for KIAA0700 protein, partial cds
   4.3e-45:585:66
   Hs.13999:AB014600
F-NT2RP3000427
   Protein kinase, cAMP-dependent, catalytic, beta
   1.2e-15:97:98
   Hs.87773:M34181
F-NT2RP3000436
   Human protein disulfide isomerase-related protein P5 mRNA, partial cds
   4.1e-06:353:59
   Hs.85200:D49489
F-NT2RP3000444
   Homo sapiens mRNA for KIAA0445 protein, complete cds
   1.2e-08:542:60
   Hs.154139:AB007914
F-NT2RP3000460
   ESTs, Highly similar to PROTEIN TRANSPORT PROTEIN SEC61 ALPHA SUBUNIT [Canis familiaris]
   1.3e-17:181:75
   Hs.131840:AI016073
F-NT2RP3000481
   Homo sapiens RanBP7/importin 7 mRNA, complete cds
   5.4e-164:770:98
   Hs.5151:AF098799
F-NT2RP3000616
   Homo sapiens KIAA0405 mRNA, complete cds
   1.5e-32:579:62
   Hs.48998:AB007865
F-NT2RP3000645
   Human KH type splicing regulatory protein KSRP mRNA, complete cds
   4.6e-06:245:64
   Hs.91142:U94832
F-NT2RP3000652
   Homo sapiens DNA from chromosome 19, BAC 33152
   2.6e-135:853:84
   Hs.55452:AC003973
F-NT2RP3000676
   Homo sapiens mRNA for KIAA0446 protein, complete cds
   8.8e-88:420:98
   Hs.158286:AB007915
F-NT2RP3000677
   ESTs
   3.9e-09:67:97
   Hs.98819:AA778727
F-NT2RP3000721
   ESTs, Weakly similar to No definition line found [C.elegans]
   1.2e-57:395:86
   Hs.159604:AI380827
F-NT2RP3000789
   Homo sapiens putative RNA binding protein KOC (koc) mRNA, complete cds
   4.8e-75:833:69
   Hs.79440:U97188
F-NT2RP3000818
   Homo sapiens chromosome 19, fosmid 39554
   5.9e-08:313:63
   Hs.129906:AC004410
F-NT2RP3000820
   ESTs, Moderately similar to WSB-1 [M.musculus]
   8.8e-127:613:97
   Hs.24630:AI365246
F-NT2RP3000838
   Homo sapiens mRNA for KIAA0638 protein, partial cds
   8.3e-79:682:79
   Hs.77864:AB014538
F-NT2RP3000871
   Homo sapiens retinoblastoma-interacting protein (RBBP8) mRNA, complete cds
   1.9e-08:350:60
   Hs.29287:U72066
F-NT2RP3000907
   Human Ini1 mRNA, complete cds
   0.91:345:59
   Hs.155626:U04847
F-NT2RP3000921
   Homo sapiens mRNA for KIAA0806 protein, complete cds
   2.0e-65:798:68
   Hs.24279:AB018349
F-NT2RP3001012
   Homo sapiens mRNA for KIAA0667 protein, partial cds
   1.3e-21:383:64
   Hs.154740:AB014567
F-NT2RP3001044
F-NT2RP3001061
   KERATIN, TYPE II CYTOSKELETAL 7
   3.4e-05:256:62
   Hs.23881:M99063
F-NT2RP3001159
   Homo sapiens chromosome 11, BAC CIT-HSP-311e8 (BC269730) containing the hFEN1 gene
   1.8e-81:527:70
   Hs.132874:AC004770
F-NT2RP3001170
   Homo sapiens mRNA for KIAA0784 protein, partial cds
   7.3e-183:859:98
   Hs.3657:AB018327
F-NT2RP3001 195
   ESTs
   3.5e-08:282:62
   Hs.135168:AI394026
F-NT2RP3001240
   ESTs, Highly similar to PROTEIN TRANSPORT PROTEIN SEC61 ALPHA SUBUNIT [Canis familiaris]
   2.8e-64:344:95
   Hs.14038:R06800
F-NT2RP3001271
   Centromere protein B (80kD)
   7.6e-08:288:64
   Hs.85004:X05299
F-NT2RP3001322
   ESTs, Weakly similar to W09D10.2 [C.elegans]
   1.2e-86:422:98
   Hs.26107:R60661
F-NT2RP3001388
F-NT2RP3001542
F-NT2RP3001560
   Protein phosphatase 2C alpha [human, teratocarcinoma, mRNA, 2346 nt]
   0.016:190:63
   Hs.57764:S87759
F-NT2RP3001592
   Cyclin-dependent kinase inhibitor 1C (p57, Kip2)
   2.3e-13:188:71
   Hs.106070:U22398
F-NT2RP3001650
   Homo sapiens KIAA0415 mRNA, complete cds
   1.6e-17:394:66
   Hs.7289:AB007875
F-NT2RP3001685
F-NT2RP3001738
   Homo sapiens chromosome 11, BAC CIT-HSP-311e8 (BC269730) containing the hFEN1 gene
   1.9e-54:776:65
   Hs.132898:AC004770
F-NT2RP3001754
   Homo sapiens mRNA for B120, complete cds
   2.4e-18:106:100
   Hs.123090:AB001895
F-NT2RP3001858
   Homo sapiens mRNA for KIAA0584 protein, partial cds
   1.9e-40:770:63
   Hs.106794:AB011156
F-NT2RP3001976
   Zinc finger protein 140 (clone pHZ-39)
   7.3e-33:493:68
   Hs.154205:U09368
F-NT2RP3002015
   Homo sapiens OPA-containing protein mRNA, complete cds
   0.018:329:62
   Hs.85313:AF071309
F-NT2RP3002160
   Homo sapiens protein phosphatase with EF-hands-2 long form (PPEF-2) mRNA, complete cds
   0.53:182:64
   Hs.113259:AF023456
F-NT2RP3002281
   Homo sapiens mRNA for KIAA0765 protein, partial cds
   5.2e-151:713:98
   Hs.62318:AB018308
F-NT2RP3002286
   ESTs
   0.034:48:95
   Hs.124692:AA777421
F-NT2RP3002311
   Beta-galactosidase (GEB1)
   2.3e-28:633:61
   Hs.79222:M34423
F-NT2RP3002324
   ESTs
   2.5e-28:296:75
   Hs.22822:H06408
F-NT2RP3002342
   Human transporter protein (g17) mRNA, complete cds
   3.2e-37:565:65
   Hs.76460:U49082
F-NT2RP3002353
   Homo sapiens mRNA for KIAA0790 protein, partial cds
   0.0055:271:60
   Hs.12002:AB018333
F-NT2RP3002409
   Homo sapiens mRNA for KIAA0719 protein, complete cds
   6.4e-191:897:98
   Hs.21198:AB018262
F-NT2RP3002411
   Hydroxysteroid (17-beta) dehydrogenase 3
   2.9e-28:604:62
   Hs.477:U05659
F-NT2RP3002448
   Human mRNA for KIAA0233 gene, complete cds
   1.6e-08:721:57
   Hs.79077:D87071
F-NT2RP3002571
   Homo sapiens mRNA for KIAA0603 protein, complete cds
   9.7e-67:707:71
   Hs.16909:AB011175
F-NT2RP3002664
   Homo sapiens Trio isoform mRNA, complete cds
   0.26:160:66
   Hs.150625:AF091395
F-NT2RP3002721
   Homo sapiens citrate synthase mRNA, complete cds
   2.4e-180:873:96
   Hs.132991:AF047042
F-NT2RP3002737
   Homo sapiens mRNA for voltage gated potassium channel
   7.1e-43:409:75
   Hs.4975:Y15065
F-NT2RP3002738
   Human BMK1 alpha kinase mRNA, complete cds
   0.0070:722:57
   Hs.3080:U29725
F-NT2RP3002790
   Cyclin-dependent kinase inhibitor 1C (p57, Kip2)
   7.2e-17:626:62
   Hs.106070:U22398
F-NT2RP3002836
   Homo sapiens mRNA for KIAA0463 protein, partial cds
   2.2e-153:717:99
   Hs.77738:AB007932
F-NT2RP3002887
   Human plectin (PLEC1) mRNA, complete cds
   2.5e-06:605:59
   Hs.79706:U53204
F-NT2RP3002900
   H.sapiens mRNA for transmembrane protein rnp24
   3.1e-09:346:64
   Hs.75914:X92098
F-NT2RP3002958
   ESTs
   8.3e-117:765:86
   Hs.107119:AI198794
F-NT2RP3002983
   ESTs
   1.4e-07:270:67
   Hs.160271:AI149075
F-NT2RP3003000
   Homo sapiens T-type calcium channel alpha-1 subunit mRNA, complete cds
   2.5e-89:555:88
   Hs.122359:AF051946
F-NT2RP3003076
   Homo sapiens mRNA for APC 2 protein, complete cds
   0.00016:522:60
   Hs.20912:AB012162
F-NT2RP3003354
   Homo sapiens secretory carrier membrane protein (SCAMP2) mRNA, complete cds
   4.0e-36:625:64
   Hs.10761:AF005038
F-NT2RP3003448
   Arginine vasopressin receptor 1B
   0.77:149:69
   Hs.1372:L37112
F-NT2RP3003469
   ESTs
   1.4e-42:239:93
   Hs.12610:W56112
F-NT2RP3003473
   ESTs, Highly similar to transcription factor ARF6 chain B [M.musculus]
   8.7e-46:281:89
   Hs.136172:W28257
F-NT2RP3003527
   Homo sapiens mRNA for protein kinase Dyrk1B
   4.6e-162:769:98
   Hs.130988:Y17999
F-NT2RP3003532
   OX-2 MEMBRANE GLYCOPROTEIN PRECURSOR
   1.5e-146:682:98
   Hs.79015:M17229
F-NT2RP3003535
   EST
   6.7e-10:330:60
   Hs.133239:AI052508
F-NT2RP3003559
   Breakpoint cluster region protein BCR
   1.0:143:66
   Hs.2557:Y00661
F-NT2RP3003614
   ESTs
   3.7e-50:327:88
   Hs.148873:T33582
F-NT2RP3003729
   ESTs, Weakly similar to unknown [S.cerevisiae]
   1.9e-96:449:99
   Hs.100843:W28953
F-NT2RP3003849
   ESTs, Weakly similar to rhophilin [M.musculus]
   1.7e-32:197:92
   Hs.118457:AA019161
F-NT2RP3003874
   Homo sapiens incomplete cDNA for a myosin class I, myh-1c
   8.5e-84:494:90
   Hs.109805:AJ001381
F-NT2RP3003939
   Peroxisomal biogenesis factor 6
   1.5e-05:236:62
   Hs.30729:D83703
F-NT2RP3003963
F-NT2RP3004000
   Homo sapiens mRNA for APC 2 protein, complete cds
   4.8e-06:669:59
   Hs.20912:AB012162
F-NT2RP3004025
   ESTs
   0.0015:68:86
   Hs.154835:AI289188
F-NT2RP3004067
   ESTs, Weakly similar to HYPOTHETICAL 51.2 KD PROTEIN IN LAG1-RPL14B INTERGENIC REGION [S.cerevisiae]
   2.1e-76:416:94
   Hs.9252:R53360
F-NT2RP3004075
   ESTs
   1.1e-54:298:94
   Hs.124051:T15786
F-NT2RP3004083
F-NT2RP3004090
   Homo sapiens Achaete-Scute homologue 2 (ASCL2) gene, complete cds
   2.4e-06:486:62
   Hs.135639:U77629
F-NT2RP3004119
   Human mRNA for KIAA0215 gene, complete cds
   4.1e-74:640:75
   Hs.82292:D86969
F-NT2RP3004130
F-NT2RP3004133
   ESTs, Weakly similar to similar to M. musculus MER5 and other AHPC/TSA proteins [C.elegans]
   4.6e-52:259:98
   Hs.132096:AA314601
F-NT2RP3004202
   ALPHA-2C-1 ADRENERGIC RECEPTOR
   1.0:229:62
   Hs.123022:J03853
F-NT2RP3004294
   Homo sapiens mRNA for KIAA0741 protein, complete cds
   2.4e-05:404:59
   Hs.3615:AB018284
F-NT2RP3004309
   Homo sapiens chromosome 11, BAC CIT-HSP-311e8 (BC269730) containing the hFEN1 gene
   3.4e-71:756:71
   Hs.132874:AC004770
F-NT2RP3004321
   Homo sapiens (clone MG2-5-12) mucin (MG2) mRNA, complete polyA site
   0.015:263:60
   Hs.103944:L13283
F-NT2RP3004345
   Cyclin-dependent kinase inhibitor 1C (p57, Kip2)
   2.3e-13:188:71
   Hs.106070:U22398
F-NT2RP3004355
   EST
   0.25:130:59
   Hs.149436:AI274484
F-NT2RP3004374
   ESTs
   1.4e-95:480:96
   Hs.12610:W56112
F-NT2RP3004406
   Human breast cancer, estrogen regulated LIV-1 protein (LIV-1) mRNA, partial cds
   3.4e-45:505:70
   Hs.79136:U41060
F-NT2RP3004481
   Homo sapiens mRNA for KIAA0476 protein, complete cds
   0.00065:594:58
   Hs.6684:AB007945
F-NT2RP3004552
   Biglycan
   0.92:347:57
   Hs.821:J04599
F-NT2RP3004557
   Human Ki nuclear autoantigen mRNA, complete cds
   2.6e-121:626:94
   Hs.152978:U11292
F-NT2RP3004625
   Homo sapiens I-1 receptor candidate protein mRNA, complete cds
   3.1e-152:710:98
   Hs.26285:AF082516
F-NT2RP3004640
   ESTs, Moderately similar to unknown [H.sapiens]
   0.76:195:64
   Hs.6487:T65302
F-NT2RP3004647
   Homo sapiens mRNA for KIAA0446 protein, complete cds
   6.6e-111:524:98
   Hs.158286:AB007915
F-NT2RP4000108
   NEUROFILAMENT TRIPLET L PROTEIN
   5.3e-159:862:93
   Hs.159540:X05608
F-NT2RP4000634
   Human MEK kinase 3 mRNA, complete cds
   2.3e-54:370:71
   Hs.86201:U78876
F-NT2RP4000962
   ESTs, Weakly similar to SPORULATION-SPECIFIC PROTEIN 1 [Saccharomyces cerevisiae]
   2.3e-95:479:96
   Hs.4789:AI418298
F-NT2RP4001001
   EST
   0.98:93:64
   Hs.147598:AI217868
F-NT2RP4001009
   Homo sapiens mRNA for Hs Ste24p, complete cds
   3.1e-176:828:98
   Hs.25846:AB016068
F-NT2RP4001467
   5' nucleotidase (CD73)
   1.1e-160:742:98
   Hs.153952:X55740
F-NT2RP4001877
   ESTs, Weakly similar to siah binding protein 1 [H.sapiens]
   3.3e-103:495:98
   Hs.65648:AA600816
F-NT2RP4001879
   EST
   0.78:171:61
   Hs.112790:AA609949
F-NT2RP4002187
   Hydroxysteroid (17-beta) dehydrogenase 3
   9.9e-27:534:63
   Hs.477:U05659
F-NT2RP4002451
   ESTs
   1.5e-11:106:86
   Hs.163724:AA017689
F-NT2RP4002715
   EST
   4.2e-07:64:93
   Hs.160901:AI366910
F-NT2RP4002750
   Ecotropic retroviral receptor
   6.6e-51:581:68
   Hs.2928:X57303
F-OVARC1000003
   Solute carrier family 17 (sodium phosphate), member 2
   6.9e-65:587:73
   Hs.936:L13258
F-OVARC1000090
   ESTs
   4.8e-07:214:65
   Hs.87456:AA434484
F-OVARC1000105
   Human novel homeobox mRNA for a DNA binding protein
   0.00095:204:64
   Hs.37035:U07664
F-OVARC1000137
   Human SNARE protein Ykt6 (YKT6) mRNA, complete cds
   4.0e-35:184:98
   Hs.31531:U95735
F-OVARC1000208
   Human calcium-dependent group X phospholipase A2 mRNA, complete cds
   1.5e-61:365:90
   Hs.136004:U95301
F-OVARC1000255
   Spleen tyrosine kinase
   2.2e-88:615:84
   Hs.74101:L28824
F-OVARC1000275
   ESTs, Weakly similar to Rap2 interacting protein 8 [M.musculus]
   4.7e-85:424:97
   Hs.55165:AA573499
F-OVARC1000298
   Homo sapiens GABA-B receptor mRNA, complete cds
   0.00021:285:61
   Hs.12307:AF056085
F-OVARC1000307
   ESTs
   0.00016:226:63
   Hs.162935:AI393970
F-OVARC1000313
   Homo sapiens mRNA for KIAA0573 protein, partial cds
   5.5e-121:585:97
   Hs.154023:AB011145
F-OVARC1000331
   Glucose-6-phosphate dehydrogenase
   5.3e-18:213:71
   Hs.1435:M24470
F-OVARC1000410
   Homo sapiens mRNA for angiopoietin-like factor
   1.5e-27:538:62
   Hs.146559:Y16132
F-OVARC1000439
F-OVARC1000467
   ESTs
   2.5e-26:173:90
   Hs.105040:AA292817
F-OVARC1000529
   Homo sapiens mRNA for C8FW phosphoprotein
   1.1e-12:391:59
   Hs.143513:AJ000480
F-OVARC1000553
   Homo sapiens chromosome 19, cosmid R26894
   9.0e-111:425:99
   Hs.157732:AC005594
F-OVARC1000775
   Human chromosome 3p21.1 gene sequence
   2.2e-70:380:95
   Hs.82837:L13435
F-OVARC1000811
   HEPATOCYTE GROWTH FACTOR ACTIVATOR PRECURSOR
   1.2e-06:446:61
   Hs.104:D14012
F-OVARC1000853
   ESTs
   7.9e-09:268:63
   Hs.92700:W37903
F-OVARC1000873
   Homo sapiens mRNA for MIFR-1, complete cds
   0.038:343:60
   Hs.58269:AB010962
F-OVARC1000916
   H.sapiens PISSLRE mRNA
   1.3e-56:435:82
   Hs.77313:X78342
F-OVARC1000956
   Human TBP-associated factor (hTAFII130) mRNA, partial cds
   7.7e-05:511:59
   Hs.24644:U75308
F-OVARC1000995
   ESTs
   2.4e-39:205:98
   Hs.163662:AA514348
F-OVARC1001030
   EST
   1.1e-44:232:96
   Hs.135504:AI091717
F-OVARC1001049
   ESTs
   6.1e-78:373:98
   Hs.135022:AI417283
F-OVARC1001086
   Homo sapiens cyclin T2a mRNA, complete cds
   6.0e-166:761:99
   Hs.155478:AF048731
F-OVARC1001132
   ESTs, Weakly similar to GC-RICH SEQUENCE DNA-BINDING FACTOR [Homo sapiens]
   7.9e-121:610:96
   Hs.26461:AI341685
F-OVARC1001163
   ESTs
   5.9e-39:215:94
   Hs.126067:AI344351
F-OVARC1001222
   ESTs
   2.7e-93:467:95
   Hs.10267:W27845
F-OVARC1001260
   Pregnancy-zone protein
   1.0:251:58
   Hs.74094:X54380
F-OVARC1001336
   Solute carrier family 17 (sodium phosphate), member 2
   1.2e-31:304:74
   Hs.936:L13258
F-OVARC1001338
   Homo sapiens cam kinase I mRNA, complete cds
   3.7e-17:570:60
   Hs.118414:L41816
F-OVARC1001569
   Human novel homeobox mRNA for a DNA binding protein
   0.038:178:63
   Hs.37035:U07664
F-OVARC1001570
   ESTs
   4.5e-10:75:93
   Hs.120928:AA703165
F-OVARC1001596
   Matrix metalloproteinase 2 (gelatinase A, 72kD gelatinase, 72kD type IV collagenase)
   0.0092:287:63
   Hs.111301:M55593
F-OVARC1001607
   Human beta-1,2-N-acetylglucosaminyltransferase II (MGAT2) gene, complete cds
   5.5e-41:323:80
   Hs.154844:U15128
F-OVARC 1001725
F-OVARC1001727
   EST
   3.2e-05:237:61
   Hs.119508:AA485732
F-OVARC1001807
   Hormone receptor (growth factor-inducible nuclear protein N10)
   3.4e-91:564:88
   Hs.1119:D49728
F-OVARC1001833
   ESTs
   1.2e-94:444:97
   Hs.155256:AA707750
F-OVARC1001952
   Myristoylated alanine-rich C-kinase substrate
   2.9e-10:364:64
   Hs.75607:D10522
F-OVARC1001991
   Human mRNA for KIAA0176 gene, partial cds
   0.0019:224:62
   Hs.4935:D79998
F-OVARC1002058
   Human mRNA for KIAA0149 gene, complete cds
   5.0e-48:674:67
   Hs.57735:D86864
F-OVARC1002178
   Homo sapiens zinc-finger protein of the cerebellum 3 (ZIC3) mRNA, complete cds
   0.010:310:61
   Hs.111227:AF028706
F-PLACE1000033
F-PLACE1000231
   Guanine nucleotide binding protein (G protein), alpha 11 (Gq class)
   0.00021:235:63
   Hs.1686:M69013
F-PLACE1000258
   KRAB zinc finger protein {alternative products}
   1.2e-14:241:70
   Hs.22556:U37251
F-PLACE1000442
   Zinc finger protein 136 (clone pHZ-20)
   7.3e-89:774:76
   Hs.69740:U09367
F-PLACE1000560
   ESTs
   1.5e-36:200:96
   Hs.86541:AA214554
F-PLACE1000740
   Homo sapiens secreted apoptosis related protein 3 (SARP3) mRNA, complete cds
   6.5e-05:283:62
   Hs.113285:AF017988
F-PLACE1000907
   ESTs, Moderately similar to zinc finger protein [H.sapiens]
   8.1e-38:237:89
   Hs.139115:AA325104
F-PLACE1000912
   ESTs
   4.6e-61:331:95
   Hs.17558:AA155762
F-PLACE1000914
   Homo sapiens PB39 mRNA, complete cds
   3.1e-45:500:69
   Hs.18910:AF045584
F-PLACE1000927
   ESTs, Weakly similar to N-methyl-D-aspartate receptor-associated protein [D.melanogaster]
   1.4e-123:655:94
   Hs.8661:AI189791
F-PLACE1000986
   ESTs
   1.2e-105:494:99
   Hs.19207:AA039595
F-PLACE1001016
   Calcium channel, voltage-dependent, L type, alpha 1S subunit
   0.011:432:59
   Hs.1294:L33798
F-PLACE1001100
   Human clone 23839 mRNA sequence
   0.38:342:60
   Hs.78362:U79249
F-PLACE1001114
   Human mRNA for KIAA0303 gene, partial cds
   0.085:339:59
   Hs.54985:AB002301
F-PLACE1041123
   ESTs
   5.0e-14:505:61
   Hs.99272:AI147740
F-PLACE1001183
   ESTs, Weakly similar to gene pp21 protein [H.sapiens]
   0.66:361:58
   Hs.15984:A,I085974
F-PLACE1001229
   ESTs, Weakly similar to D9481.15 gene product [S.cerevisiae]
   9.3e-110:561:96
   Hs.125155:W52093
F-PLACE1001231
   ESTs, Weakly similar to sodium iodide symporter [H.sapiens]
   1.0e-17:120:91
   Hs.5167:AA053914
F-PLACE1001340
   Homo sapiens mRNA for KIAA0719 protein, complete cds
   4.1e-132:636:97
   Hs.21198:AB018262
F-PLACE1001401
   ESTs, Weakly similar to IgE receptor beta subunit [H.sapiens]
   3.1e-100:516:95
   Hs.43900:AA418443
F-PLACE 1001407
   H.sapiens mRNA for B-HLH DNA binding protein
   0.00015:244:66
   Hs.66744:X99268
F-PLACE1001464
   5' nucleotidase (CD73)
   1.6e-152:742:96
   Hs.153952:X55740
F-PLACE1001500
   Bloom syndrome
   5.7e-05:450:58
   Hs.36820:U39817
F-PLACE1001516
   Homo sapiens Rigui (RIGUI) mRNA, complete cds
   2.3e-07:663:58
   Hs.8114:AF022991
F-PLACE1001536
   ESTs
   4.6e-60:318:97
   Hs.13026:H04491
F-PLACE1001564
   H.sapiens mRNA for HE6 Tm7 receptor
   8.8e-41:499:70
   Hs.155681:X81892
F-PLACE1001655
   Homo sapiens Shab-related delayed-rectifier K+ channel alpha subunit (KCNS3) mRNA, complete cds
   4.3e-125:585:98
   Hs.47584:AF043472
F-PLACE1001788
   Homo sapiens mRNA for HYA22, complete cds
   3.2e-22:234:75
   Hs.147189:D88153
F-PLACE1001795
F-PLACE1001836
   ESTs, Moderately similar to !!!! ALU SUBFAMILY SQ WARNING ENTRY !!!! [H.sapiens]
   1.1e-18:162:80
   Hs.157223:AA309318
F-PLACE1001918
   Human p76 mRNA, complete cds
   1.3e-22:693:60
   Hs.28757:U81006
F-PLACE1001949
   ESTs
   0.97:243:63
   Hs.151143:AA576926
F-PLACE1002080
   Homo sapiens mRNA for KIAA0600 protein, partial cds
   2.4e-130:622:98
   Hs.9028:AF039691
F-PLACE1002095
F-PLACE1002153
   Homo sapiens TACC2 protein (TACC2) mRNA, partial cds
   2.7e-162:764:98
   Hs.90415:AF095791
F-PLACE1002329
   ESTs
   1.5e-107:556:95
   Hs.28907:AI343292
F-PLACE1002355
   Homo sapiens protease-activated receptor 4 mRNA, complete cds
   9.0e-19:190:77
   Hs.137574:AF055917
F-PLACE1002374
   Cathepsin L
   2.0e-163:716:94
   Hs.78056:X12451
F-PLACE1002518
   Homo sapiens multiple membrane spanning receptor TRC8 (TRC8) mRNA, complete cds
   7.0e-19:396:64
   Hs.28285:AF064801
F-PLACE1002547
   Homo sapiens mRNA for KIAA0719 protein, complete cds
   8.3e-173:819:98
   Hs.21198:AB018262
F-PLACE1002726
   Human mRNA for KIAA0362 gene, partial cds
   1.0:310:59
   Hs.25515:AB002360
F-PLACE1002905
   ESTs
   2.4e-74:415:92
   Hs.110298:AA621807
F-PLACE1002911
   ESTs, Weakly similar to Y53C12A.3 [C.elegans]
   0.030:279:58
   Hs.107747:AI357868
F-PLACE1002967
   ESTs
   3.3e-120:574:98
   Hs.11090:W37646
F-PLACE1003135
   Homo sapiens STE20-like kinase 3 (mst-3) mRNA, complete cds
   1.5e-50:450:75
   Hs.72292:AF024636
F-PLACE1003163
   Homo sapiens DBI-related protein mRNA, complete cds
   1.5e-153:722:98
   Hs.15250:AF069301
F-PLACE1003407
   Homo sapiens putative transmembrane protein (CLN5) mRNA, complete cds
   2.0e-142:682:97
   Hs.30213:AF068227
F-PLACE1003428
   Biotinidase
   8.2e-06:265:62
   Hs.78885:AF018631
F-PLACE1003438
   ESTs
   0.018:470:60
   Hs.119482:AI361002
F-PLACE1003460
   ESTs
   0.019:211:60
   Hs.92700:W37903
F-PLACE1003529
   130 KD LEUCINE-RICH PROTEIN
   0.53:208:63
   Hs.87157:M92439
F-PLACE1003573
F-PLACE1003598
   Calcium channel, voltage-dependent, P/Q type, alpha 1A subunit
   0.00064:302:64
   Hs.96253:U79666
F-PLACE1003644
   ESTs
   1.3e-06:265:63
   Hs.163564:R43678
F-PLACE1003737
F-PLACE1003772
   Human p300/CBP-associated factor (P/CAF) mRNA, complete cds
   7.0e-09:448:61
   Hs.155302:U57317
F-PLACE1003839
   Homo sapiens Chromosome 16 BAC clone CIT987SK-A-69G12
   7.7e-109:521:97
   Hs.154050:AC004131
F-PLACE1003845
   ESTs, Moderately similar to similar to thymidine diphosphoglucose 4,6-dehydratase [C.elegans]
   1.2e-92:432:100
   Hs.153778:AI246000
F-PLACE1003852
   Homo sapiens mRNA for KIAA0758 protein, partial cds
   2.4e-172:814:98
   Hs.22039:AB018301
F-PLACE1004028
F-PLACE1004078
   GELSOLIN PRECURSOR, PLASMA
   3.1e-49:616:67
   Hs.80562:X04412
F-PLACE1004166
   ESTs
   7.6e-79:415:94
   Hs.163741:AA551077
F-PLACE1004168
F-PLACE1004199
   EST
   6.8e-15:147:80
   Hs.128205:AA972308
F-PLACE1004279
   Homo sapiens putative renal organic anion transporter 1 (hROAT1) mRNA, complete cds
   3.9e-20:456:62
   Hs.23965:AF057039
F-PLACE1004282
F-PLACE1004305
   Homo sapiens mRNA for KIAA0740 protein, complete cds
   8.7e-123:612:96
   Hs.15099:AB018283
F-PLACE1004441
   Human G protein-coupled receptor (GPR1) gene, complete cds
   8.6e-99:501:96
   Hs.159248:U13666
F-PLACE1004450
   AMINOPEPTIDASE N
   1.1e-09:587:57
   Hs.1239:M22324
F-PLACE1004482
F-PLACE1004492
   ESTs
   2.1e-25:134:100
   Hs.154475:AI199037
F-PLACE1004519
   ESTs
   1.0e-110:518:99
   Hs.128505:AA306435
F-PLACE1004520
   Pregnancy-specific beta 1-glycoprotein 7
   1.3e-110:606:92
   Hs.119662:M34715
F-PLACE1004630
   Homo sapiens mRNA for osteoblast specific cysteine-rich protein, complete cds
   2.0e-139:749:92
   Hs.82582:AB008375
F-PLACE1004637
F-PLACE1004648
F-PLACE1004816
   Homo sapiens mRNA for Hakata antigen, complete cds
   1.2e-99:590:90
   Hs.9225:D88587
F-PLACE1004887
   Homo sapiens Achaete-Scute homologue 2 (ASCL2) gene, complete cds
   5.1e-06:486:62
   Hs.135639:U77629
F-PLACE1005003
   Human SNC19 mRNA sequence
   1.5e-21:472:63
   Hs.56937:U20428
F-PLACE1005005
   Homo sapiens ubiquitin conjugating enzyme G2 (UBE2G2) mRNA, complete cds
   4.7e-42:245:93
   Hs.151614:AF032456
F-PLACE1005031
   ESTs, Highly similar to CHLORINE CHANNEL PROTEIN P64 [Bos taurus]
   2.9e-43:538:70
   Hs.118991:AA675919
F-PLACE1005239
   ESTs
   2.4e-42:209:100
   Hs.154475:AI199037
F-PLACE1005250
F-PLACE1005383
   Homo sapiens UP50 mRNA, complete cds
   8.5e-128:633:96
   Hs.11494:AF093118
F-PLACE1005410
   ESTs, Highly similar to PROTEIN TRANSPORT PROTEIN SEC61 ALPHA SUBUNIT [Canis familiaris]
   1.3e-17:181:75
   Hs.131840:AI016073
F-PLACE1005426
   Pregnancy-specific beta-1 glycoprotein 4
   2.3e-109:596:93
   Hs.108936:X17097
F-PLACE1005519
   Homo sapiens STE20-like kinase 3 (mst-3) mRNA, complete cds
   3.3e-55:521:74
   Hs.72292:AF024636
F-PLACE1005539
   HETEROGENOUS NUCLEAR RIBONUCLEOPROTEIN U
   5.8e-05:277:63
   Hs.103804:AF068846
F-PLACE1005544
F-PLACE1005569
   EST
   0.38:60:75
   Hs.137086:AA912486
F-PLACE1005601
   Homo Sapiens angiotensin II receptor gene, complete cds
   0.016:72:84
   Hs.20954:AI054441
F-PLACE1005660
F-PLACE1005669
   Homo sapiens N-methyl-D-aspartate receptor 2D subunit precursor (NMDAR2D) mRNA, complete cds
   3.5e-08:461:60
   Hs.113286:U77783
F-PLACE1005682
F-PLACE1005725
   Huntingtin (Huntington disease)
   1.1e-06:401:61
   Hs.79391:L12392
F-PLACE1005736
   ESTs
   3.6e-63:343:94
   Hs.17757:AA875839
F-PLACE1005745
   ESTs, Weakly similar to HYPOTHETICAL 25.2 KD PROTEIN IN ACB1-KSS1 INTERGENIC REGION [S.cerevisiae]
   6.9e-66:351:94
   Hs.7870:AI078137
F-PLACE1005768
   ESTs
   7.9e-60:318:95
   Hs.143856:AI186351
F-PLACE1005815
   Mutated in colorectal cancers
   0.0029:199:62
   Hs.1345:M62397
F-PLACE1005878
   ESTs, Highly similar to CHLORINE CHANNEL PROTEIN P64 [Bos taurus]
   5.0e-38:464:70
   Hs.118991:AA675919
F-PLACE1005927
   INSULIN-LIKE GROWTH FACTOR BINDING PROTEIN COMPLEX ACID LABILE CHAIN PRECURSOR
   0.010:511:59
   Hs.839:M86826
F-PLACE1006071
   EST
   0.68:224:59
   Hs.161788:AA371859
F-PLACE1006073
   Homo sapiens mRNA for glucuronyltransferase I, complete cds
   5.5e-96:464:98
   Hs.26492:AB009598
F-PLACE1006079
   Homo sapiens BAC clone RG300E22 from 7q21-q31.1
   1.5e-18:402:65
   Hs.99348:AC004774
F-PLACE1006093
   Homo sapiens mRNA for protein phosphatase 1 (PPP1R6)
   0.0022:306:59
   Hs.106471:Y18206
F-PLACE1006208
   HOMEOBOX/POU DOMAIN PROTEIN RDC-1
   0.022:425:57
   Hs.74095:L20433
F-PLACE1006219
   ESTs, Moderately similar to similar to thymidine diphosphoglucose 4,6-dehydratase [C.elegans]
   1.7e-61:294:100
   Hs.153778:AI246000
F-PLACE1006277
   EST
   0.42:60:75
   Hs.137086:AA912486
F-PLACE1006290
   ESTs, Weakly similar to similar to M. musculus MERS and other AHPC/TSA proteins [C.elegans]
   1.3e-51:260:98
   Hs.132096:AA314601
F-PLACE1006443
   Homo sapiens PB39 mRNA, complete cds
   1.2e-53:553:70
   Hs.18910:AF045584
F-PLACE1006515
   Homo sapiens mRNA for KIAA0576 protein, partial cds
   1.3e-141:655:99
   Hs.14687:AB011148
F-PLACE1006716
   EST
   7.2e-12:148:75
   Hs.162969:AA677315
F-PLACE1006786
   ESTs
   0.0050:125:72
   Hs.109156:AA193501
F-PLACE1006809
   ESTs
   4.5e-99:477:98
   Hs.135208:AI093908
F-PLACE1006959
   ESTs
   7.4e-72:381:93
   Hs.4963:W29030
F-PLACE1007028
   Homo sapiens p17-Beckwith-Wiedemann region 1 C (BWR1C) mRNA, complete cds
   1.8e-18:364:65
   Hs.154036:AF035444
F-PLACE1007040
   H.sapiens NF-H gene, exon 1 (and joined CDS)
   1.4e-09:501:61
   Hs.75735:X15306
F-PLACE1007077
   ESTs, Moderately similar to testis-specific TCP20 [H.sapiens]
   0.88:192:62
   Hs.85818:AI216525
F-PLACE1007081
   Human plectin (PLEC1) mRNA, complete cds
   0.079:403:60
   Hs.79706:U53204
F-PLACE1007096
   YY1 transcription factor
   0.64:173:64
   Hs.97496:M77698
F-PLACE1007296
   ER LUMEN PROTEIN RETAINING RECEPTOR 1
   4.2e-73:542:83
   Hs.78040:X55885
F-PLACE1007591
   EST
   0.026:136:64
   Hs.130897:AI014389
F-PLACE1007626
   Homo sapiens unknown mRNA, complete cds
   2.6e-105:516:97
   Hs.11441:AF047439
F-PLACE1007702
   Homo sapiens mRNA for UTF1, complete cds
   0.033:297:62
   Hs.158307:AB011076
F-PLACE1007845
   ESTs
   4.8e-22:158:89
   Hs.23445:AA489015
F-PLACE1007881
F-PLACE1007971
   ESTs, Weakly similar to K07F5.14 [C.elegans]
   1.1e-128:599:99
   Hs.157918:AA313781
F-PLACE1008282
   ESTs, Highly similar to HEME-EGULATED EUKARYOTIC INITIATION FACTOR EIF-2-ALPHA KINASE [Oryctolagus cuniculus]
   2.4e-65:353:94
   Hs.130830:W27380
F-PLACE1008297
F-PLACE1008359
   Human arginine-rich protein (ARP) gene, complete cds
   0.020:197:64
   Hs.75412:M83751
F-PLACE1008469
   Homo sapiens PB39 mRNA, complete: cds
   5.3e-20:620:60
   Hs.18910:AF045584
F-PLACE1008549
   Homo sapiens E74-like factor 5 (ELF5) mRNA, complete cds
   1.8e-145:693:98
   Hs.159267:AF049703
F-PLACE1008657
   VILLIN
   2.3e-10:356:61
   Hs.3046:X12901
F-PLACE1008716
   Human beta-1,2-N-acetylglucosaminyltransferase II (MGAT2) gene, complete cds
   1.5e-31:191:92
   Hs.154844:U15128
F-PLACE1008744
F-PLACE1008984
   Pregnancy-associated plasma protein A
   0.0085:268:60
   Hs.158229:U28727
F-PLACE1008985
   Signal transducer and activator of transcription 5A
   0.0047:249:64
   Hs.14203:U43185
F-PLACE1009067
   Human density enhanced phosphatase-1 mRNA, complete cds
   2.0e-06:453:60
   Hs.1177:U10886
F-PLACE1009196
F-PLACE1009279
   Homo sapiens mRNA for serin protease with IGF-binding motif, complete cds
   1.9e-11:327:64
   Hs.75111:D87258
F-PLACE1009527
   Human DNA-binding protein ABP/ZF mRNA, complete cds
   6.8e-21:125:96
   Hs.86185:U82613
F-PLACE1009546
   TRANSCRIPTION FACTOR RELB
   0.051:248:61
   Hs.858:M83221
F-PLACE1009600
   ESTs
   1.0:124:64
   Hs.52794:W51887
F-PLACE1009735
   ESTs
   0.022:387:61
   Hs.132253:AI027207
F-PLACE1009982
F-PLACE1010011
   Homo sapiens transcription factor forkhead-like 7 (FKHL7) gene, complete cds
   1.3e-09:330:66
   Hs.143551:AF048693
F-PLACE1010078
   ESTs, Weakly similar to HYPOTHETICAL 25.2 KD PROTEIN IN ACB1-KSS1 INTERGENIC REGION [S.cerevisiae]
   8.3e-47:474:72
   Hs.13144:T67556
F-PLACE1010081
   Homo sapiens serine/threonine kinase RICK (RICK) mRNA, complete cds
   2.2e-151:733:97
   Hs.103755:AF027706
F-PLACE1010251
   Homo sapiens Na+/H+ exchanger regulatory factor 2 (NHERF-2) mRNA, complete cds
   0.0037:405:60
   Hs.101813:AB016243
F-PLACE1010445
   ESTs
   1.7e-45:235:97
   Hs.144501:N39767
F-PLACE1010713
   Hydroxysteroid (17-beta) dehydrogenase 3
   2.8e-20:447:62
   Hs.477:U05659
F-PLACE1010784
   Human protease-activated receptor 3 (PAR3) mRNA, complete cds
   0.56:199:59
   Hs.159196:U92971
F-PLACE 1010827
   H.sapiens mRNA for transmembrane protein rnp24
   2.9e-09:346:64
   Hs.75914:X92098
F-PLACE1010968
   ESTs
   0.00062:52:98
   Hs.119408:T87544
F-PLACE1011045
   Homo sapiens E1-like protein mRNA, complete cds
   6.0e-129:595:99
   Hs.28190:AF094516
F-PLACE1011116
F-PLACE1011181
   ESTs
   1.0:301:58
   Hs.80285:AI092519
F-PLACE1011236
   Homo sapiens putative renal organic anion transporter 1 (hROAT1) mRNA, complete cds
   1.1e-41:776:62
   Hs.23965:AF057039
F-PLACE1011364
   ESTs, Weakly similar to HYPOTHETICAL 141.2 KD PROTEIN EEED8.9 IN CHROMOSOME II [C.elegans]
   3.7e-53:276:96
   Hs.106499:W28299
F-PLACE1011407
   ESTs, Moderately similar to ZINC FINGER PROTEIN 140 [H.sapiens]
   3.2e-15:228:70
   Hs.152174:AI199619
F-PLACE1011516
   ESTs, Weakly similar to HYPOTHETICAL 21.5 KD PROTEIN IN SEC15-SAP4 INTERGENIC REGION [S.cerevisiae]
   1.7e-85:444:95
   Hs.110978:AA843431
F-PLACE1011708
   Homo sapiens intrinsic factor-B12 receptor precursor, mRNA, complete cds
   5.9e-145:722:96
   Hs.148318:AF034611
F-PLACE1011824
   Human Ste20-like kinase (MST2) mRNA, complete cds
   1.6e-101:561:92
   Hs.92317:U26424
F-PLACE1011978
   Homo sapiens DNA from chromosome 19, BAC 33152
   3.8e-67:733:72
   Hs.55452:AC003973
F-PLACE2000118
   Homo sapiens DNA sequence from PAC 393P12 on chromosome Xp11.21. Contains a hypothetical protein KIAA0413 (KIAA0412, KIAA0065, KIAA0569) LIKE Zinc Finger protein pseudogene, a hypothetical Proline-rich protein KIAA0269 LIKE gene and a 60S Ribosomal Protein L7 LIKE pseudogene. Contains ESTs, an STS and a GSS (BAC end sequence)
   7.8e-115:568:95
   Hs.120856:AL022578
F-PLACE2000219
   EST
   8.7e-11:137:75
   Hs.98191:AA417044
F-PLACE3000181
   Human protocadherin 42 mRNA, complete cds for abbreviated PC42
   1.5e-128:745:90
   Hs.79769:L11370
F-PLACE3000213
   EST
   1.0:219:63
   Hs.98452:AA426058
F-PLACE4000354
   ESTs
   1.4e-13:190:71
   Hs.138841:R94879
F-PLACE4000455
F-SKNMC1000004
   Homo sapiens GABA-B receptor mRNA, complete cds
   0.00039:275:62
   Hs.12307:AF056085
F-SKNMC1000014
   ESTs
   3.3e-38:196:98
   Hs.113307:H16716
F-SKNMC1000082
   Complement component 4A
   0.98:324:63
   Hs.76682:K02403
F-THYRO1000036
   Homo sapiens mRNA for putative ATPase, partial
   0.98:199:60
   Hs.91471:AJ006268
F-THYRO1000061
   Human kinase Myt1 (Myt1) mRNA, complete cds
   1.0:210:62
   Hs.77783:AF014118
F-THYRO1000099
   ESTs
   2.5e-119:605:96
   Hs.11782:W07369
F-THYRO1000196
   Homo sapiens Ksp-cadherin (CDH16) mRNA, complete cds
   1.6e-126:475:98
   Hs.115418:AF016272
F-THYRO1000400
   Human R kappa B mRNA, complete cds
   0.64:223:63
   Hs.95262:U08191
F-THYRO1000580
   ESTs, Weakly similar to ZINC FINGER PROTEIN 7 [H.sapiens]
   5.4e-27:248:76
   Hs.25465:AA528105
F-THYRO1000584
   Alpha mannosidase II isozyme
   2.2e-06:528:60
   Hs.155961:L28821
F-THYRO1000678
   Gap junction protein, beta 2, 26kD (connexin 26)
   1.3e-33:266:80
   Hs.81795:M86849
F-THYRO1000776
   Human involucrin mRNA
   0.0025:497:59
   Hs.157091:M13903
F-THYRO1000795
   MITOCHONDRIAL 2-OXOGLUTARATE/MALATE CARRIER PROTEIN
   4.1e-19:532:62
   Hs.3816:AF070548
F-THYRO1000846
   Homo sapiens centrosomal Nek2-associated protein 1 (C-NAP1) mRNA, complete cds
   0.029:387:60
   Hs.27910:AF049105
F-THYRO1000866
   Homo sapiens SKB1Hs mRNA, complete cds
   1.1e-92:529:89
   Hs.12912:AF015913
F-THYRO1000956
   Homo sapiens mRNA for G-protein coupled receptor
   1.8e-15:474:64
   Hs.155235:Y13583
F-THYRO1000964
   Human OB binding protein-2 (OB-BP2) mRNA, complete cds
   0.22:303:61
   Hs.117005:U71383
F-THYRO1000999
   EST
   2.0e-05:198:63
   Hs.146520:AI130948
F-THYRO1001063
   Human mRNA for cerebroside sulfotransferase, complete cds
   0.51:448:60
   Hs.17958:D88667
F-THYRO1001071
   ESTs
   2.1e-29:237:83
   Hs.155582:AI125241
F-THYRO1001102
   ESTs, Weakly similar to growth arrest inducible gene product [H.sapiens]
   4.7e-32:208:88
   Hs.7854:W21970
F-THYRO1001113
   Homo sapiens dysferlin mRNA, complete cds
   3.2e-53:684:68
   Hs.143897:AF075575
F-THYRO1001128
   ESTs
   2.1e-120:589:97
   Hs.62595:AA306052
F-THYRO1001205
F-THYRO1001237
   ESTs
   0.66:326:60
   Hs.148352:U80757
F-THYRO1001242
   Protein phosphatase 2C alpha [human, teratocarcinoma, mRNA, 2346 nt]
   0.017:188:63
   Hs.57764:S87759
F-THYRO1001266
   Human sodium iodide symporter mRNA, complete cds
   8.6e-43:806:62
   Hs.103983:U66088
F-THYRO1001327
   ESTs
   2.8e-50:264:96
   Hs.154667:AI343524
F-THYRO1001456
   EST
   0.90:84:72
   Hs.130049:AA902650
F-THYRO1001457
   Protein kinase C, mu
   6.0e-53:705:67
   Hs.2891:X75756
F-THYRO1001471
   ESTs
   8.0e-52:278:94
   Hs.7604:W31115
F-THYRO1001478
   Human mRNA for KIAA0150 gene, partial cds
   0.79:150:66
   Hs.98508:D63484
F-THYRO1001495
   Homo sapiens KIAA0415 mRNA, complete cds
   9.5e-75:550:82
   Hs.7289:AB007875
F-THYRO1001523
   ESTs
   7.2e-19:142:86
   Hs.140588:H60533
F-THYRO1001529
   ESTs
   5.7e-24:141:95
   Hs.114172:AA703201
F-THYRO1001593
   H.sapiens mRNA for serine/threonine protein kinase EMK
   1.4e-70:643:74
   Hs.157199:X97630
F-THYRO1001608
   Human mRNA for KIAA0227 gene, partial cds
   2.6e-07:533:59
   Hs.79170:D86980
F-THYRO1001641
   ESTs
   0.87:269:59
   Hs.14599:AA522511
F-THYRO1001700
   Homo sapiens c-Jun N-terminal kinase kinase 2 (JNKK2) mRNA, complete cds
   3.3e-05:441:59
   Hs.110299:AF013589
F-THYRO1001702
   Human plectin (PLEC1) mRNA, complete cds
   0.00017:346:62
   Hs.79706:U53204
F-THYRO1001725
   Homo sapiens mRNA for procollagen I-N proteinase
   1.3e-06:275:64
   Hs.120330:AJ003125
F-THYRO1001770
   Homo sapiens mRNA for HsGAK, complete cds
   0.046:265:58
   Hs.153227:D88435
F-THYRO1001803
   EST
   0.0085:201:63
   Hs.158782:AI376601
F-Y79AA1000030
   ESTs
   0.00051:276:60
   Hs.111999:AA465020
F-Y79AA1000127
   ESTs
   1.3e-85:430:96
   Hs.49932:W58552
F-Y79AA1000207
   ESTs
   4.5e-81:407:96
   Hs.125308:AI376737
F-Y79AA1000226
   ESTs, Highly similar to HYPOTHETICAL 52.8 KD PROTEIN T05E11.5 IN CHROMOSOME IV [Caenorhabditis elegans]
   0.00081:76:84
   Hs.11221:AI192291
F-Y79AA1000270
   Human mRNA for ORF, Xq terminal portion
   9.9e-97:590:88
   Hs.6551:D16469
F-Y79AA1000426
   CELL SURFACE GLYCOPROTEIN MUC18 PRECURSOR
   0.045:507:59
   Hs.82914:X68264
F-Y79AA1000521
   Homo sapiens mRNA for putative G-protein coupled receptor, EDG6
   0.0029:489:58
   Hs.159543:AJ000479
F-Y79AA1000750
   ESTs
   9.9e-12:252:65
   Hs.52885:H29851
F-Y79AA1000776
   ESTs
   1.4e-50:340:87
   Hs.144198:AI017555
F-Y79AA1000777
   Homo sapiens mRNA for putative transcription factor, partial
   3.9e-10:501:61
   Hs.26782:AJ009770
F-Y79AA1000876
   Homo sapiens short form transcription factor C-MAF (c-maf) mRNA, complete cds
   1.3e-11:323:66
   Hs.30250:AF055376
F-Y79AA1000888
   Homo sapiens mRNA for KIAA0469 protein, complete cds
   1.5e-05:641:58
   Hs.7764:AB007938
F-Y79AA1000959
   Homo sapiens Hsp70 binding protein HspBP1 mRNA, complete cds
   5.3e-54:277:96
   Hs.53066:AF093420
F-Y79AA1000967
   Human mRNA for KIAA0369 gene, complete cds
   8.1e-10:517:61
   Hs.21355:AB002367
F-Y79AA1001013
   ESTs
   2.4e-44:259:93
   Hs.109468:W52074
F-Y79AA1001056
   ESTs, Moderately similar to maternal transcript Maid [M.musculus]
   4.7e-07:90:87
   Hs.36794:AI038407
F-Y79AA1001062
   ESTs, Weakly similar to tumor necrosis factor-alpha-induced protein B12 [H.sapiens]
   1.6e-60:320:96
   Hs.13982:W27344
F-Y79AA1001090
   H.sapiens DAP-kinase mRNA
   2.3e-06:465:59
   Hs.153924:X76104
F-Y79AA1001212
   Homo sapiens SL15 protein mRNA, complete cds
   1.5e-163:763:98
   Hs.6710:AF038961
F-Y79AA1001264
   Homo sapiens mRNA for MSJ-1, complete cds
   5.3e-15:367:64
   Hs.3845:AB014888
F-Y79AA1001272
   Human plectin (PLEC1) mRNA, complete cds
   6.3e-05:325:63
   Hs.79706:U53204
F-Y79AA1001328
   Homo sapiens Delta mRNA, complete cds
   1.8e-07:271:61
   Hs.144631:AF003522
F-Y79AA1001426
   Aldehyde dehydrogenase 7
   0.94:485:56
   Hs.83155:U10868
F-Y79AA1001427
   NADH-CYTOCHROME B5 REDUCTASE
   1.7e-56:649:69
   Hs.75666:M28713
F-Y79AA1001430
   Homo sapiens mRNA for KIAA0469 protein, complete cds
   2.8e-124:577:99
   Hs.7764:AB007938
F-Y79AA1001523
   Homo sapiens transcription intermediary factor 1 (TIF1) mRNA, complete cds
   1.1e-92:496:93
   Hs.128763:AF009353
F-Y79AA1001530
   Human beta-tubulin gene (5-beta) with ten Alu family members
   1.0e-131:669:95
   Hs.108014:X00734
F-Y79AA1001592
   ESTs
   1.2e-88:212:97
   Hs.131180:AA594251
F-Y79AA1001727
F-Y79AA1001787
   Human mRNA for KIAA0315 gene, partial cds
   0.48:248:63
   Hs.3989:AB002313
F-Y79AA1001793
   ESTs
   1.4e-67:192:98
   Hs.118559:AA887084
F-Y79AA1001795
   Homo sapiens mRNA for GalT4 protein
   5.3e-89:431:98
   Hs.21495:AL031228
F-Y79AA1001799
   NUCLEAR FACTOR RIP140
   0.54:182:62
   Hs.155017:X84373
F-Y79AA1001803
   ESTs, Highly similar to MELANOMA-ASSOCIATED ANTIGEN XP [Homo sapiens]
   0.72:169:63
   Hs.94011:AA627644
F-Y79AA1001863
   EST
   1.0:114:63
   Hs.152260:AA489703
F-Y79AA1002022
   B94 PROTEIN
   5.7e-13:469:65
   Hs.75522:M92357
F-Y79AA1002058
   Homo sapiens clone 24733 mRNA sequence
   1.7e-154:740:98
   Hs.21970:AF052149
F-Y79AA1002121
   EST
   0.14:104:66
   Hs.100070:M91493
F-Y79AA1002129
   ESTs
   5.1e-90:431:98
   Hs.40719:AI183452
F-Y79AA1002213
F-Y79AA1002334
   ESTs
   5.0e-20:187:80
   Hs.111900:AA397579
F-Y79AA1002373
   ESTs
   4.5e-37:192:98
   Hs.118559:AA887084
F-Y79AA1002376
   Homo sapiens cytoplasmic dynein intermediate chain 1 mRNA, complete cds
   1.2e-36:657:64
   Hs.65248:AF063228
F-Y79AA1002378
   Homo sapiens KIAA0426 mRNA, complete cds
   4.9e-38:424:72
   Hs.97476:AB007886
F-Y79AA1002381
   CELL DIVISION PROTEIN KINASE 3
   8.4e-17:580:61
   Hs.100009:X66357

### Homology search result 9

The result of the homology search in the Human Unigene(http://www.ncbi.nlm.nih.gov/UniGene) using the clone sequences of the 3'-ends.
Indicated are from the top,
the name of the clone sequence,
title of the top hit data,
the P-value: the length of the sequence used for comparison (nucleotide):similarity (%),
the Accession No. of the top hit data.

Blank indicates that the 3'-end sequence corresponding to the 5'-end was not determined in the clone.

Data were not shown for the clones in which the P-value was higher than 1.
R-HEMBA1000006
   ESTs
   1.0:85:71
   Hs.130699:AA621478
R-HEMBA1000121
   ESTs
   1.3e-111:545:97
   Hs.153432:AA098922
R-HEMBA1000128
   ESTs, Weakly similar to HYPOTHETICAL 30.6 KD PROTEIN IN SCP160-MRPL8 INTERGENIC REGION PRECURSOR [S.cerevisiae]
   3.0e-98:532:93
   Hs.7745:H92988
R-HEMBA1000275
   ESTs
   6.5e-11:81:81
   Hs.163492:AI334460
R-HEMBA1000300
   Homo sapiens mRNA for putative lipoic acid synthetase, partial
   1.2e-39:309:81
   Hs.53531:AJ224162
R-nnnnnnnnnnnn
   ESTs
   4.9e-95:455:98
   Hs.154009:AI284184
R-HEMBA1000462
   Homo sapiens clone 243 unknown mRNA, complete sequence
   3.6e-91:313:94
   Hs.20423:AF091094
R-HEMBA1000477
   ESTs
   4.7e-111:541:97
   Hs.84526:AI341541
R-HEMBA1000590
   Homo sapiens mRNA for matrilin-4, partial
   2.6e-102:547:93
   Hs.129361:AJ007581
R-HEMBA1000634
   ESTs
   0.85:189:62
   Hs.131268:AA909162
R-HEMBA1000671
   ESTs
   6.5e-84:432:96
   Hs.31991:T78668
R-HEMBA1000713
   Homo sapiens 10kD protein (BC10) mRNA, complete cds
   4.0e-119:575:97
   Hs.5300:AF053470
R-HEMBA1000732
   EST
   3.9e-81:435:92
   Hs.146718:AI146722
R-nnnnnnnnnnnn
R-HEMBA1000875
   EST
   0.023:207:62
   Hs.148275:AA907849
R-HEMBA1000940
   Human macrophage-derived chemokine precursor (MDC) mRNA, complete cds
   7.4e-31:211:81
   Hs.97203:U83171
R-HEMBA1000962
   ESTs
   1.1e-104:515:97
   Hs.8978:W63573
R-HEMBA1001 184
   EST
   7.1e-07:382:62
   Hs.124559:AA847550
R-HEMBA1001221
   ESTs, Weakly similar to transmembrane protein [H.sapiens]
   1.2e-95:487:95
   Hs.22791:AI056665
R-HEMBA1001228
   Human germline oligomeric matrix protein (COMP) mRNA, complete cds
   4.0e-42:170:92
   Hs.1584:AC003107
R-HEMBA1001272
   ESTs
   5.7e-71:514:84
   Hs.26966:N74056
R-HEMBA1001296
   EST
   1.7e-93:494:95
   Hs.102465:N27272
R-HEMBA1001297
   Homo sapiens putative transcription factor CA150 mRNA, complete cds
   1.5e-93:466:96
   Hs.13063:AF017789
R-HEMBA1001390
   ESTs
   1.6e-42:181:89
   Hs.139190:N55515
R-HEMBA1001563
   Homo sapiens DEC-205 mRNA, complete cds
   8.4e-42:311:83
   Hs.153563:AF011333
R-HEMBA1001621
   ESTs, Highly similar to PROBABLE G PROTEIN-COUPLED RECEPTOR APJ [Homo sapiens]
   4.2e-56:386:86
   Hs.9305:W84893
R-HEMBA1001878
   Homo sapiens U5 snRNP-specific 40 kDa protein mRNA, complete cds
   1.1e-80:433:93
   Hs.10290:AF090988
R-HEMBA1001886
   Zinc finger protein 141 (clone pHZ-44)
   5.9e-61:530:80
   Hs.159596:L15309
R-HEMBA1002048
   ESTs
   0.95:127:63
   Hs.98690:AA431162
R-HEMBA1002131
R-HEMBA1002163
   ESTs, Weakly similar to K09E9.2 [C.elegans]
   5.9e-36:225:90
   Hs.26813:AI339473
R-HEMBA1002167
   ESTs
   1.5e-35:325:80
   Hs.124171:N98933
R-HEMBA1002178
   MICROSOMAL DIPEPTIDASE PRECURSOR
   0.99:243:61
   Hs.109:J05257
R-HEMBA1002195
   Deoxyhypusine synthase
   1.9e-19:109:100
   Hs.79064:U79262
R-HEMBA1002227
   Myristoylated alanine-rich C-kinase substrate
   2.0e-116:567:97
   Hs.75607:D10522
R-HEMBA1002316
   Homo sapiens mRNA for putative GTP-binding protein
   8.2e-20:160:85
   Hs.101033:Y14391
R-HEMBA1002420
   ESTs, Weakly similar to T03G11.6 gene product [C.elegans]
   2.7e-78:402:97
   Hs.108354:W19984
R-HEMBA1002421
   Syndecan 2 (heparan sulfate proteoglycan 1, cell surface-associated, fibroglycan)
   1.9e-91:443:97
   Hs.1501:J04621
R-HEMBA1002524
   Human MHC Class I region proline rich protein mRNA, complete cds
   1.0e-111:551:96
   Hs.41548:U63336
R-HEMBA1002551
   ESTs
   3.4e-107:553:96
   Hs.92071:W80592
R-HEMBA1002767
   Homo sapiens chromosome 1p33-p34 beta-1,4-galactosyltransferase mRNA, complete cds
   5.5e-108:568:95
   Hs.19154:AF038660
R-HEMBA1002985
   ESTs
   4.4e-39:211:96
   Hs.126894:AA932538
R-HEMBA1003047
   Homo sapiens intrinsic factor-B12 receptor precursor, mRNA, complete cds
   1.6e-115:571:96
   Hs.148318:AF034611
R-HEMBA1003072
   EST
   0.044:220:61
   Hs.136349:AA490873
R-HEMBA1003101
   Homo sapiens tyrosylprotein sulfotransferase-2 mRNA, complete cds
   1.2e-117:575:97
   Hs.26350:AF049891
R-HEMBA1003120
   Zinc finger protein 10 (KOX 1)
   5.8e-41:412:73
   Hs.2479:X78933
R-HEMBA1003230
   Homo sapiens UP50 mRNA, complete cds
   4.2e-44:258:93
   Hs.11494:AF093118
R-HEMBA1003294
   ESTs
   4.3e-84:410:98
   Hs.113517:AA418756
R-HEMBA1003315
   ESTs, Weakly similar to TIP49 [R.norvegicus]
   7.3e-73:476:87
   Hs.6455:AA515838
R-HEMBA1003392
   Homo sapiens LDL receptor-related protein 6 (LRP6) mRNA, complete cds
   8.3e-117:557:98
   Hs.23672:AF074264
R-HEMBA1003399
   ESTs, Highly similar to MVP1 PROTEIN [Saccharomyces cerevisiae]
   8.0e-94:526:92
   Hs.12169:N38744
R-HEMBA1003487
   ESTs
   4.5e-84:417:96
   Hs.21835:AA458524
R-HEMBA1003497
   ESTs
   1.4e-72:346:99
   Hs.129837:AA778570
R-HEMBA1003530
   ESTs
   8.5e-82:459:91
   Hs.22140:R41751
R-HEMBA1003602
   ESTs
   1.0e-101:592:90
   Hs.124342:AA829829
R-HEMBA1003732
   ESTs
   2.1e-111:530:99
   Hs.101660:AA481200
R-HEMBA1003945
   Calcineurin B
   2.9e-83:410:97
   Hs.1335:M30773
R-HEMBA1004007
   Homo sapiens PYRIN (MEFV) mRNA, complete cds
   3.8e-57:382:77
   Hs.113283:AF018080
R-HEMBA1004085
   ESTs
   3.0e-59:396:87
   Hs.102480:AA520980
R-nnnnnnnnnnnn
   Homo sapiens intersectin short form mRNA, complete cds
   2.0e-116:569:97
   Hs.66392:AF064244
R-HEMBA1004250
   ESTs
   1.6e-97:469:97
   Hs.125529:AA883986
R-HEMBA1004391
   EST
   0.085:113:63
   Hs.157582:AI356856
R-HEMBA1004444
   ESTs
   2.3e-88:430:98
   Hs.141680:N98441
R-HEMBA1004454
   ESTs
   1.7e-71:338:100
   Hs.103913:AA740543
R-HEMBA1004505
   ESTs
   2.2e-63:329:95
   Hs.4814:AA631254
R-HEMBA1004785
   EST
   1.0:77:67
   Hs.144066:AA905236
R-HEMBA1004797
   ESTs
   4.1e-11:71:100
   Hs.27206:AA626782
R-HEMBA1004952
   ESTs
   6.0e-93:435:99
   Hs.115120:AA935633
R-HEMBA1004971
   ESTs
   0.98:152:58
   Hs.112621:AA608964
R-HEMBA1004982
   ESTs
   2.3e-109:516:98
   Hs.14877:AA749081
R-HEMBA1005070
   Human mRNA for KIAA0310 gene, complete cds
   4.0e-96:381:91
   Hs.5716:AB002308
R-HEMBA1005084
   ESTs
   1.0:75:80
   Hs.62119:AA043299
R-HEMBA1005145
   Homo sapiens LIM protein mRNA, complete cds
   1.6e-58:278:84
   Hs.154103:AF061258
R-HEMBA1005230
   ESTs
   3.7e-65:336:95
   Hs.124946:AI026708
R-HEMBA1005246
R-HEMBA1005267
   Human protein immuno-reactive with anti-PTH polyclonal antibodies mRNA, partial cds
   7.8e-75:536:81
   Hs.44566:U28831
R-HEMBA1005337
   EST
   8.7e-58:291:97
   Hs.48956:N64339
R-HEMBA1005430
   ESTs
   7.6e-83:388:100
   Hs.28968:AA524690
R-HEMBA1005449
   ESTs
   5.0e-47:317:86
   Hs.23650:H21144
R-HEMBA1005489
   ESTs
   1.8e-96:504:94
   Hs.8028:AA053817
R-HEMBA1005522
   EST
   1.0:98:64
   Hs.157385:AI364194
R-HEMBA1005545
   MUSCARINIC ACETYLCHOLINE RECEPTOR M3
   6.3e-117:579:96
   Hs.7138:U29589
R-HEMBA1005698
   ESTs
   1.6e-113:562:96
   Hs.12942:AI042353
R-HEMBA1005913
   ESTs
   2.8e-109:564:94
   Hs.28827:AI125541
R-HEMBA1005929
   Human macrophage-derived chemokine precursor (MDC) mRNA, complete cds
   9.6e-63:497:77
   Hs.97203:U83171
R-HEMBA1005945
   ESTs
   1.1e-74:412:92
   Hs.32246:AA464020
R-HEMBA1006016
   ESTs
   1.4e-18:162:82
   Hs.149448:AI082465
R-HEMBA1006171
   EST
   0.049:94:69
   Hs.159919:AA961766
R-HEMBA1006276
   ESTs
   6.3e-22:257:75
   Hs.138847:N64493
R-HEMBA1006299
   ESTs, Weakly similar to R06B9.b [C.elegans]
   3.9e-107:596:91
   Hs.30432:W28988
R-HEMBA1006311
   Homo sapiens SALL1 gene, partial
   0.99:273:60
   Hs.123094:X98833
R-HEMBA1006335
   ESTs
   2.5e-72:447:89
   Hs.23579:W38893
R-HEMBA1006357
   ESTs
   6.3e-15:187:74
   Hs.161714:AA229078
R-HEMBA1006430
   Homo sapiens tumor necrosis factor superfamily member LIGHT mRNA, complete cds
   2.9e-47:303:88
   Hs.129708:AF064090
R-HEMBA1006482
   Homo sapiens h-sco1 (SCO1) mRNA, nuclear gene encoding mitochondrial protein, complete cds
   5.5e-107:537:96
   Hs.14511:AF026852
R-HEMBA1006517
   ESTs, Weakly similar to !!!! ALU SUBFAMILY J WARNING ENTRY !!!! [H.sapiens]
   2.7e-43:173:86
   Hs.141505:N30650
R-HEMBA1006544
   Homo sapiens mRNA for small GTP-binding protein, complete cds
   5.8e-60:329:80
   Hs.115325:D84488
R-HEMBA1006572
   ESTs
   7.2e-94:450:99
   Hs.123933:AA758566
R-HEMBA1006658
   Homo sapiens mRNA for KIAA0687 protein, partial cds
   2.3e-112:570:94
   Hs.3628:AB014587
R-HEMBA1006707
   Homo sapiens mRNA for matrilin-4, partial
   1.7e-79:389:97
   Hs.129361:AJ007581
R-HEMBA1006724
R-HEMBA1006749
   Homo sapiens mRNA for matrilin-4, partial
   1.0e-89:472:94
   Hs.129361:AJ007581
R-HEMBA1006770
   ESTs, Moderately similar to CAGH4 [H.sapiens]
   2.0e-112:554:96
   Hs.41641:AA428519
R-HEMBA1006902
   Homo sapiens mRNA for matrilin-4, partial
   3.0e-113:540:98
   Hs.129361:AJ007581
R-HEMBA1006912
   H.sapiens mRNA for phosphoinositide 3-kinase
   5.9e-45:297:86
   Hs.101238:Y11312
R-HEMBA1006916
   Homo sapiens Grb14 mRNA, complete cds
   5.8e-116:346:99
   Hs.83070:L76687
R-HEMBA1006960
   ESTs
   1.7e-110:519:99
   Hs.22015:AI359551
R-HEMBA1007013
   ESTs
   0.53:280:59
   Hs.143532:AI087321
R-HEMBA1007057
R-HEMBA1007063
   EST
   3.2e-41:310:83
   Hs.163333:AA879053
R-HEMBA1007241
   ESTs
   1.8e-103:492:98
   Hs.127478:AI188768
R-HEMBA1007291
   Human mRNA for KIAA0266 gene, complete cds
   8.7e-46:283:89
   Hs.78878:D87455
R-HEMBA1007332
   ESTs, Weakly similar to hTAFII100 [H.sapiens]
   2.8e-17:161:80
   Hs.3727:AA205887
R-HEMBB1000106
   ESTs
   1.3e-100:491:97
   Hs.27774:AA576731
R-HEMBB1000276
R-HEMBB1000309
   EST
   1.0:150:64
   Hs.125409:AA879096
R-HEMBB1000407
   ESTs, Weakly similar to C47D12.2 [C.elegansJ
   4.1e-110:535:97
   Hs.14328:AA503393
R-HEMBB1000447
   Homo sapiens JWA protein mRNA, complete cds
   5.6e-109:533:97
   Hs.92384:AF070523
R-HEMBB1000542
   ESTs, Weakly similar to C01H6.7 [C.elegans]
   1.6e-88:497:91
   Hs.18171:AA524327
R-HEMBB1000567
   Insulin-like growth factor 2 (somatomedin A)
   8.9e-61:369:88
   Hs.155487:J03242
R-HEMBB1000642
   ESTs
   2.2e-44:308:84
   Hs.141318:N71080
R-HEMBB1000668
   ESTs, Weakly similar to hTAFII100 [H.sapiens]
   2.5e-102:520:95
   Hs.3830:AA167691
R-HEMBB1000679
   ESTs
   6.7e-36:188:97
   Hs.154218:AA169554
R-HEMBB1000881
   ESTs
   8.4e-105:519:96
   Hs.110967:AA570505
R-HEMBB1000905
   ESTs
   1.1e-94:454:98
   Hs.52515:AA464314
R-HEMBB1001026
   ESTs
   0.22:93:69
   Hs.119510:AA630235
R-HEMBB1001048
   EST
   0.42:127:66
   Hs.147466:AI215091
R-HEMBB1001200
   ESTs
   3.7e-07:330:62
   Hs.10109:AI148628
R-HEMBB1001407
   MHC class II transactivator
   3.8e-35:414:71
   Hs.3076:U18259
R-HEMBB1001530
   ESTs
   2.4e-95:455:98
   Hs.8956:AI146421
R-HEMBB1001547
   ESTs
   1.0e-111:533:98
   Hs.33979:AI074147
R-HEMBB1001573
   ESTs, Moderately similar to LL5 protein [R.norvegicus]
   1.7e-06:64:95
   Hs.131327:AI148746
R-HEMBB1001847
   ESTs
   1.4e-54:280:96
   Hs.109755:AA180809
R-HEMBB1001959
   Homo sapiens clone 24781 mRNA sequence
   1.5e-104:504:97
   Hs.108112:AF070640
R-HEMBB1001978
   Homo sapiens mRNA for TRAFS, complete cds
   7.0e-28:220:84
   Hs.29736:AB000509
R-HEMBB1002039
   ESTs, Weakly similar to ! ! ! ! ALU SUBFAMILY J WARNING ENTRY ! ! ! ! [H.sapiens]
   1.5e-34:423:72
   Hs.154912:N63897
R-HEMBB1002041
   ESTs, Weakly similar to transmembrane protein [H.sapiens]
   7.0e-122:575:98
   Hs.22791:AI056665
R-HEMBB1002051
   ESTs, Weakly similar to HYPOTHETICAL 92.1 KD PROTEIN ZK1098.3 IN CHROMOSOME III [Caenorhabditis elegans]
   4.2e-43:298:84
   Hs.141429:AA631915
R-HEMBB1002120
   ESTs
   1.4e-91:438:99
   Hs.145014:AI218562
R-HEMBB1002162
   ESTs
   1.0e-34:238:86
   Hs.164036:AA845659
R-HEMBB1002228
   Homo sapiens neuronal thread protein AD7c-NTP mRNA, complete cds
   1.7e-59:583:77
   Hs.129735:AF010144
R-HEMBB1002245
   ESTs
   9.1e-66:383:91
   Hs.8989:R71365
R-HEMBB1002302
   ESTs
   3.6e-54:329:89
   Hs.37706:AA005120
R-HEMBB1002427
   ESTs
   4.2e-83:400:98
   Hs.130783:AI263114
R-HEMBB1002465
   EST
   9.9e-38:231:90
   Hs.133443:AI061405
R-HEMBB1002661
   ESTs
   2.5e-101:472:99
   Hs.26878:AI421289
R-HEMBB1002663
   Small inducible cytokine A5 (RANTES)
   7.1e-43:268:88
   Hs.155464:AF088219
R-HEMBB1002693
   ESTs
   4.6e-84:435:96
   Hs.155522:AA829725
R-MAMMA1000046
   EST
   3.9e-06:196:65
   Hs.136664:AA707467
R-MAMMA1000102
   Human G protein-coupled receptor (STRL22) mRNA, complete cds
   1.1e-31:237:83
   Hs.46468:U45984
R-MAMMA1000106
   ESTs
   1.3e-65:333:95
   Hs.130749:AI284219
R-MAMMA1000118
   ESTs
   7.3e-95:465:97
   Hs.119286:AA126730
R-MAMMA1000141
   ESTs
   4.2e-94:515:93
   Hs.8116:H23508
R-MAMMA1000204
   Homo sapiens dysferlin mRNA, complete cds
   2.3e-108:542:96
   Hs.143897:AF075575
R-MAMMA1000226
   ESTs
   2.1e-112:535:98
   Hs.105761:AA903862
R-MAMMA1000403
   ESTs
   1.5e-36:162:83
   Hs.152413:AA780515
R-MAMMA1000449
   EST
   1.5e-40:347:78
   Hs.163333:AA879053
R-MAMMA1000457
   Homo sapiens clone 638 unknown mRNA, complete sequence
   2.6e-117:570:97
   Hs.5825:AF091084
R-MAMMA1000473
   ESTs
   1.3e-62:308:99
   Hs.53565:W02102
R-MAMMA1000496
   Phosphodiesterase 4C, cAMP-specific (dunce (Drosophila)-homolog phosphodiesterase E1)
   0.051:125:68
   Hs.189:AC005759
R-MAMMA1000528
   ESTs
   2.4e-12:216:71
   Hs.134105:AI078038
R-MAMMA1000591
   ESTs
   5.0e-104:509:98
   Hs.151678:AA032243
R-MAMMA1000614
   Homo sapiens mRNA for KIAA0665 protein, complete cds
   0.57:251:62
   Hs.119004:AB014565
R-MAMMA1000652
   ESTs
   0.93:49:87
   Hs.13248:R54144
R-MAMMA1000681
   ESTs
   1.3e-87:434:97
   Hs.46668:N47089
R-MAMMA1000706
   Homo sapiens cGMP phosphodiesterase A1 (PDE9A) mRNA, complete cds
   3.7e-48:232:100
   Hs.18953:AF067223
R-MAMMA1000788
   ESTs
   3.7e-108:559:94
   Hs.38969:AA130220
R-MAMMA1000810
   ESTs
   1.2e-45:347:80
   Hs.146811:AA410788
R-MAMMA1000814
   ESTs
   1.1e-18:288:70
   Hs.140608:N53448
R-MAMMA1000881
   ESTs
   1.9e-107:557:96
   Hs.141602:N63562
R-MAMMA1000986
   ESTs
   3.8e-46:342:83
   Hs.132722:AA618531
R-MAMMA1000994
   Homo sapiens mRNA for ISLR, complete cds
   1.2e-109:552:96
   Hs.102171:AB003184
R-MAMMA1001043
   ESTs
   2.3e-88:445:96
   Hs.20450:AI094818
R-MAMMA1001066
   Homo sapiens KIAA0414 mRNA, partial cds
   1.5e-43:282:81
   Hs.127649:AB007874
R-MAMMA1001094
   Homo sapiens clone 243 unknown mRNA, complete sequence
   3.0e-116:566:97
   Hs.20423:AF091094
R-MAMMA1001141
   ESTs
   1.2e-104:496:98
   Hs.29669:AI285856
R-MAMMA1001150
   ESTs, Highly similar to MYOSIN LIGHT CHAIN KINASE [Dictyostelium discoideum]
   1.9e-59:284:100
   Hs.9915:AI300083
R-MAMMA1001237
   ESTs
   0.45:206:62
   Hs.121366:AA758653
R-MAMMA1001284
   ESTs
   6.3e-40:279:85
   Hs.109765:AI096738
R-MAMMA1001310
   ESTs, Moderately similar to !!!! ALU SUBFAMTLY J WARNING ENTRY !!!! [H.sapiens]
   5.1e-98:498:96
   Hs.27264:AA159597
R-MAMMA1001418
   Human mRNA for platelet-activating factor acetylhydrolase 2, complete cds
   1.2e-41:302:85
   Hs.86188:D87845
R-MAMMA1001532
   ESTs
   3.9e-22:331:71
   Hs.141840:AA028117
R-MAMMA1001609
   Small inducible cytokine A5 (RANTES)
   1.5e-31:277:78
   Hs.155464:AF088219
R-MAMMA1001615
   ESTs
   1.1e-72:376:95
   Hs.135569:AA923461
R-MAMMA1001623
   ESTs
   7.9e-106:505:98
   Hs.22908:AI224910
R-MAMMA1001634
   Homo sapiens PYRIN (MEFV) mRNA, complete cds
   1.9e-44:428:76
   Hs.113283:AF018080
R-MAMMA1001893
   ESTs
   8.0e-67:367:92
   Hs.19210:W26097
R-MAMMA1001901
   Homo sapiens mRNA, chromosome 1 specific transcript KIAA0492
   4.7e-35:342:69
   Hs.127338:AB007961
R-MAMMA1001957
   Cytochrome P450, subfamily I (aromatic compound-inducible), polypeptide 2
   5.5e-47:383:79
   Hs.1361:M55053
R-MAMMA1001978
   ESTs
   6.6e-108:560:95
   Hs.8859:AA191552
R-MAMMA1002070
R-MAMMA1002080
   ESTs, Weakly similar to GTP-BINDING PROTEIN YPTM1 [Zea mays]
   9.8e-105:542:94
   Hs.10092:AI189282
R-MAMMA1002087
   ESTs
   4.0e-19:153:84
   Hs.136678:AA730474
R-MAMMA1002095
   ESTs
   6.8e-34:196:93
   Hs.48119:AA454227
R-MAMMA1002128
   ESTs, Highly similar to ABC1 PROTEIN PRECURSOR [Saccharomyces cerevisiae]
   9.0e-96:503:94
   Hs.39088:AA194773
R-MAMMA1002142
   ESTs
   5.6e-21:145:90
   Hs.62119:AA043299
R-MAMMA1002165
   ESTs
   1.6e-35:351:76
   Hs.140413:N47721
R-MAMMA1002205
   Homo sapiens DNA fragmentation factor 40 kDa subunit (DFF40) mRNA, complete cds
   6.4e-42:217:79
   Hs.133089:AF064019
R-MAMMA1002224
   ESTs
   0.50:170:64
   Hs.144140:H04293
R-MAMMA1002234
R-MAMMA1002586
   ESTs
   5.0e-105:529:96
   Hs.4814:AA631254
R-MAMMA1002633
   ESTs
   7.3e-97:470:98
   Hs.38039:AI360128
R-MAMMA1003126
   ESTs
   6.1e-114:567:97
   Hs.20733:AI417917
R-NT2RM4000100
   ESTs
   3.6e-71:343:99
   Hs.92186:AI080282
R-NT2RM4000115
   ESTs
   1.5e-86:405:100
   Hs.129151:AA988192
R-NT2RM4000198
   ESTs
   8.4e-83:462:93
   Hs.96772:AI369496
R-NT2RM4000284
   Human IgG Fc receptor hFcRn mRNA, complete cds
   5.4e-95:440:100
   Hs.110804:U12255
R-NT2RM4000295
   ESTs
   1.1e-112:544:97
   Hs.21452:AA581881
R-NT2RM4000326
   EST
   4.0e-59:301:96
   Hs.86264:AA206496
R-NT2RM4000417
   ESTs
   2.0e-88:489:93
   Hs.29098:AA521439
R-NT2RM4000444
   ESTs
   6.4e-90:497:92
   Hs.6129:U66676
R-NT2RM4000587
   ESTs
   1.0e-97:473:98
   Hs.24947:AA039350
R-NT2RM4000593
   ESTs
   9.8e-109:554:95
   Hs.7579:AA775865
R-NT2RM4000648
   ESTs, Moderately similar to GLYPICAN-1 PRECURSOR [Homo sapiens]
   7.6e-39:262:85
   Hs.118407:AA001322
R-NT2RM4000761
   ESTs
   6.4e-86:433:95
   Hs.153428:AI246519
R-NT2RM4000965
   ESTs
   2.8e-102:523:96
   Hs.61790:AA421156
R-NT2RM4000997
R-NT2RM4001321
   ESTs
   2.4e-66:315:100
   Hs.75425:AA149434
R-NT2RM4001325
   ESTs
   0.99:104:62
   Hs.116257:AA628680
R-NT2RM4001377
   Homo sapiens mRNA for KIAA0638 protein, partial cds
   9.3e-113:553:96
   Hs.77864:AB014538
R-NT2RM4001735
   Homo sapiens clone 23904 mRNA sequence
   1.5e-107:553:94
   Hs.67364:AF052129
R-NT2RM4001768
   EST
   1.6e-14:82:85
   Hs.140922:R51520
R-NT2RM4001843
   ESTs
   2.1e-123:579:98
   Hs.3741:AI057614
R-NT2RM4002352
   Homo sapiens hLRp105 mRNA for LI)L receptor related protein 105, complete cds
   1.8e-109:557:95
   Hs.143641:AB009462
R-NT2RP2000092
   ESTs
   3.3e-08:286:65
   Hs.79881:AA401302
R-NT2RP2000178
   ESTs, Weakly similar to MITOCHONDRIAL LON PROTEASE HOMOLOG PRECURSOR [H.sapiens]
   2.3e-95:462:98
   Hs.47305:AA195153
R-NT2RP2000240
   ESTs
   1.3e-55:272:98
   Hs.125522:AI299693
R-NT2RP2000394
   ESTs
   2.4e-107:528:96
   Hs.28555:W55892
R-NT2RP2000447
   ESTs, Moderately similar to dynamin, internal form 2, short C-terminal form [H.sapiens]
   1.6e-67:357:94
   Hs.128788:AA424076
R-NT2RP2000479
   ESTs
   2.6e-48:312:86
   Hs.146811:AA410788
R-NT2RP2000514
   EST
   3.2e-63:348:93
   Hs.44542:N33966
R-NT2RP2000533
   ESTs
   0.017:307:57
   Hs.97873:AA402799
R-NT2RP2000616
   ESTs
   1.0e-91:475:95
   Hs.50344:AI300539
R-NT2RP2000649
   Homo sapiens mRNA for Hs Ste24p, complete cds
   1.4e-66:322:98
   Hs.25846:AB016068
R-NT2RP2000663
   ESTs
   8.2e-59:311:96
   Hs.9728:T98746
R-NT2RP2000712
   EST
   1.7e-27:239:76
   Hs.161561:W60681
R-NT2RP2000739
   ESTs, Weakly similar to zinc finger protein [H.sapiens]
   6.3e-86:462:93
   Hs.13323:AA897542
R-NT2RP2000818
   ESTs
   7.3e-99:485:97
   Hs.100525:AI310204
R-NT2RP2000903
   H.sapiens 5T4 gene for 5T4 Oncofetal antigen
   1.2e-100:505:96
   Hs.82128:AJ012159
R-NT2RP2001200
   Homo sapiens mRNA for KIAA0676 protein, partial cds
   6.6e-59:306:95
   Hs.115763:AB014576
R-NT2RP2001223
   ESTs
   1.2e-94:475:95
   Hs.27556:AA115361
R-NT2RP2001276
   ESTs, Moderately similar to regulatory protein [M.musculus]
   4.7e-65:354:92
   Hs.105547:AI361036
R-NT2RP2001388
   ESTs
   5.5e-83:459:93
   Hs.15713:AA485755
R-NT2RP2001469
   ESTs, Weakly similar to teg292 protein [M.musculus]
   2.0e-30:233:83
   Hs.68791:AA527270
R-NT2RP2001480
   Homo sapiens thrombospondin 3 (THBS3) gene, complete cds
   2.1e-84:426:95
   Hs.82165:L38969
R-NT2RP2001495
   ESTs, Weakly similar to transporter protein [H.sapiens]
   1.7e-14:130:84
   Hs.18272:N78499
R-NT2RP2001514
   ESTs, Weakly similar to PROBABLE CATION-TRANSPORTING ATPASE YEL031W [Saccharomyces cerevisiae]
   3.3e-45:242:95
   Hs.9275:AA973284
R-NT2RP2001538
   EST
   1.4e-05:111:73
   Hs.137268:T39311
R-NT2RP2001562
   EST
   0.50:35:91
   Hs.140505:AA804211
R-NT2RP2001662
   Homo sapiens clone 24615 mRNA sequence
   1.0e-95:485:95
   Hs.94785:AF055012
R-NT2RP2001755
   Homo sapiens mRNA for KIAA0762 protein, partial cds
   4.2e-105:576:92
   Hs.5378:AB018305
R-NT2RP2001769
   ESTs
   4.2e-102:548:93
   Hs.14014:AA745592
R-NT2RP2001817
   ESTs
   6.0e-97:472:97
   Hs.31176:AI037953
R-NT2RP2001878
   ESTs
   3.3e-94:475:95
   Hs.144655:AI279798
R-NT2RP2001903
   ESTs
   1.7e-88:461:95
   Hs.112218:AI038601
R-NT2RP2001915
   ESTs
   7.8e-96:480:96
   Hs.100890:AA779892
R-NT2RP2001921
   ESTs
   2.5e-88:466:94
   Hs.104859:AA779101
R-NT2RP2001948
   ESTs
   1.9e-81:439:91
   Hs.105463:AA583017
R-NT2RP2001956
   ESTs
   8.7e-85:477:91
   Hs.12101:AA677423
R-NT2RP2002015
   ESTs
   3.5e-85:431:95
   Hs.75425:AA149434
R-NT2RP2002063
   EST
   0.0083:199:62
   Hs.48699:N63049
R-NT2RP2002188
   ESTs
   1.5e-108:559:94
   Hs.47320:AA057440
R-NT2RP2002232
   ESTs
   2.5e-113:576:95
   Hs.7099:AI089774
R-nnnnnnnnnnnn
   Human mRNA for KIAA0383 gene, partial cds
   8.0e-102:511:96
   Hs.27590:AB002381
R-NT2RP2002409
   ESTs
   3.2e-84:432:95
   Hs.128443:AI281991
R-NT2RP2002510
   ESTs
   1.3e-42:303:82
   Hs.146811:AA410788
R-NT2RP2002527
   Thromboxane A2 receptor
   2.9e-23:164:88
   Hs.89887:D38081
R-NT2RP2002533
   Homo sapiens putative tumor suppressor gene 26 protein alpha 2 delta calcium channel subunit mRNA, complete cds
   4.0e-117:580:96
   Hs.127436:AF040709
R-NT2RP2002564
   Human zinc-finger protein C2H2-150 mRNA, complete cds
   4.0e-111:569:94
   Hs.108139:U38864
R-NT2RP2002674
   ESTs, Weakly similar to putative p150 [H.sapiens]
   0.010:293:60
   Hs.140964:AI214400
R-NT2RP2002721
   ESTs
   5.6e-10:165:69
   Hs.108745:H95644
R-NT2RP2002824
   EST
   0.0055:209:58
   Hs.136259:AA347883
R-NT2RP2002942
   ESTs
   9.2e-82:422:96
   Hs.140952:R59211
R-NT2RP2002974
   ESTs
   5.6e-99:507:96
   Hs.43314:AA160543
R-NT2RP2002976
   ESTs
   2.9e-78:397:91
   Hs.83575:N28730
R-NT2RP2003042
   ESTs
   2.7e-107:526:97
   Hs.6770:AA972732
R-NT2RP2003179
   ESTs
   2.9e-59:335:92
   Hs.87019:AA760977
R-NT2RP2003210
   ESTs
   2.1e-80:419:94
   Hs.25354:N28667
R-NT2RP2003302
   ESTs, Moderately similar to ZINC FINGER PROTEIN 7 [Homo sapiens]
   2.1e-92:443:98
   Hs.112508:AA599140
R-NT2RP2003369
   ESTs
   9.7e-84:462:92
   Hs.155116:C16874
R-NT2RP2003383
   Homo sapiens mRNA for KIAA0458 protein, complete cds
   1.3e-112:549:97
   Hs.7414:AB007927
R-NT2RP2003390
   Homo sapiens SEC63 (SEC63) mRNA, complete cds
   4.9e-56:286:96
   Hs.31575:AF100141
R-NT2RP2003469
   Human mRNA for KIAA0355 gene, complete cds
   6.6e-40:302:83
   Hs.153014:AB002353
R-NT2RP2003545
   ESTs
   8.0e-121:572:98
   Hs.23643:AI299952
R-NT2RP2003593
   EST
   1.0:124:62
   Hs.59890:AA001879
R-NT2RP2003599
   EST
   5.2e-06:319:60
   Hs.147887:AI223203
R-NT2RP2003655
   ESTs
   9.3e-107:519:97
   Hs.5831:AA176450
R-NT2RP2003664
   Homo sapiens mRNA for leptin receptor gene-related protein
   5.3e-112:549:96
   Hs.23581:Y12670
R-NT2RP2003931
   Human mRNA for KIAA0365 gene, partial cds
   1.7e-113:571:96
   Hs.84123:AB002363
R-NT2RP2003940
   EST
   3.0e-71:385:93
   Hs.162657:AA603590
R-NT2RP2003950
   Homo sapiens clone 24778 unknown mRNA
   5.0e-98:494:95
   Hs.25306:AF070572
R-NT2RP2004069
   Human kpni repeat mrna (cdna clone pcd-kpni-8), 3' end
   6.3e-54:556:74
   Hs.103948:K00627
R-NT2RP2004108
   ESTs, Moderately similar to ZINC FINGER PROTEIN ZFP-36 [Homo sapiens]
   6.9e-92:442:98
   Hs.14831:AI261191
R-NT2RP2004141
   ESTs
   8.3e-29:171:93
   Hs.25700:AI338437
R-NT2RP2004179
   ESTs
   3.1e-71:461:88
   Hs.6748:R68509
R-NT2RP2004205
   ESTs
   2.6e-44:397:78
   Hs.95115:AA206594
R-NT2RP2004447
   ESTs
   4.0e-101:494:97
   Hs.51655:AA523276
R-NT2RP2004495
   ESTs, Weakly similar to transporter protein [H.sapiens]
   6.1e-71:417:92
   Hs.18272:N78499
R-NT2RP2004524
   ESTs
   1.8e-93:482:95
   Hs.119285:AI225008
R-NT2RP2004556
   Homo sapiens mRNA for KIAA0459 protein, partial cds
   8.8e-48:353:82
   Hs.28169:AB007928
R-NT2RP2004606
   Tissue inhibitor of metalloproteinase 1 (erythroid potentiating activity, collagenase inhibitor)
   3.5e-116:576:96
   Hs.148726:X03124
R-NT2RP2004648
   ESTs
   5.9e-114:600:93
   Hs.3741:AI057614
R-NT2RP2004670
   ESTs
   1.7e-92:488:94
   Hs.6262:T89093
R-NT2RP2004794
   EST
   0.44:205:57
   Hs.147759:AI220726
R-NT2RP2004837
   ESTs
   6.9e-111:576:94
   Hs.12305:AA166889
R-NT2RP2004847
   ESTs
   8.3e-94:445:99
   Hs.53996:AI268861
R-NT2RP2005027
   GLUCOSE TRANSPORTER TYPE 3, BRAIN
   2.5e-104:508:97
   Hs.7594:M20681
R-NT2RP2005069
   ESTs, Highly similar to vacuolar protein sorting homolog r-vps33b [R.norvegicus]
   4.7e-111:541:97
   Hs.26510:AA700425
R-NT2RP2005163
   ESTs
   6.8e-64:327:89
   Hs.83575:N28730
R-NT2RP2005181
   ESTs, Moderately similar to HIGH-AFFINITY CATIONIC AMINO ACID TRANSPORTER-1 [H.sapiens]
   1.6e-106:527:97
   Hs.86362:AA205485
R-NT2RP2005247
   MHC class II transactivator
   7.9e-35:465:69
   Hs.3076:U18259
R-NT2RP2005378
   ESTs
   3.4e-110:566:94
   Hs.23060:N64748
R-NT2RP2005391
   ESTs
   5.5e-82:463:92
   Hs.118793:AA192438
R-NT2RP2005425
   Homo sapiens mRNA for KIAA0803 protein, partial cds
   2.6e-101:526:94
   Hs.58103:AB018346
R-NT2RP2005463
   ESTs, Weakly similar to weakly similar to S. cervisiae PTM1 precursor [C.elegans]
   7.6e-111:554:97
   Hs.16492:N95400
R-NT2RP2005514
   ESTs
   1.8e-97:490:95
   Hs.109677:AA447864
R-NT2RP2005535
   EST
   5.1e-40:399:73
   Hs.127142:AA937570
R-NT2RP2005541
   ESTs
   5.2e-114:573:96
   Hs.70823:AI378619
R-NT2RP2005597
   ESTs, Weakly similar to rotated abdomen protein [D.melanogaster]
   3.7e-109:543:96
   Hs.99654:AA670164
R-nnnnnnnnnnnn
   ESTs
   1.1 e-100:501:97
   Hs.112011:AA987961
R-NT2RP2005666
   ESTs
   2.7e-106:560:94
   Hs.42814:AA205754
R-NT2RP2005774
   Homo sapiens apoptosis-related mRNA, 3'UTR, partial sequence
   7.0e-96:440:96
   Hs.139345:AF035364
R-NT2RP2005878
   ESTs
   2.8e-89:479:93
   Hs.142305:R42591
R-NT2RP2005883
   ESTs
   1.0e-85:431:96
   Hs.6909:AA928115
R-NT2RP2005887
   ESTs
   5.5e-109:566:94
   Hs.12305:AA166889
R-nnnnnnnnnnnn
   Paired box homeotic gene 6 (aniridia, keratitis)
   1.6e-116:578:96
   Hs.89506:M93650
R-NT2RP2005994
   EST
   0.0061:129:68
   Hs.160756:AI310589
R-NT2RP2006004
   ESTs, Weakly similar to KIAA0405 [H.sapiens]
   4.7e-45:303:86
   Hs.14146:W92235
R-NT2RP2006042
   EST
   0.64:84:71
   Hs.133275:AI053487
R-NT2RP2006092
   ESTs, Weakly similar to HYPOTHETICAL PROTEIN KIAA0168 [H.sapiens]
   1.1e-75:384:95
   Hs.32822:AI194045
R-NT2RP2006099
   ESTs
   6.9e-35:224:82
   Hs.139446:AA461080
R-NT2RP2006134
   EST
   1.3e-95:445:100
   Hs.162033:AA514590
R-NT2RP2006269
   Human mRNA for KIAA0315 gene, partial cds
   0.96:343:60
   Hs.3989:AB002313
R-NT2RP2006512
   Homo sapiens clone 23904 mRNA sequence
   1.5e-107:531:96
   Hs.67364:AF052129
R-NT2RP3000011
   ESTs
   7.3e-92:508:91
   Hs.112041:W26001
R-NT2RP3000022
   EST
   0.78:175:63
   Hs.135650:AA902912
R-NT2RP3000059
   ESTs
   6.2e-99:475:98
   Hs.123136:AA631067
R-NT2RP3000063
   ESTs
   9.7e-105:554:94
   Hs.7542:AA121663
R-nnnnnnnnnnnn
   Human mRNA for KIAA0314 gene, partial cds
   5.0e-17:307:65
   Hs.155045:AB002312
R-NT2RP3000148
   ESTs
   6.4e-101:527:94
   Hs.58461:W80378
R-NT2RP3000169
   Homo sapiens MRS1 mRNA, complete cds
   1.4e-111:551:96
   Hs.30985:AF093239
R-NT2RP3000171
   EST
   0.45:205:57
   Hs.147759:AI220726
R-NT2RP3000172
   ESTs
   2.0e-89:494:93
   Hs.6262:T89093
R-NT2RP3000201
   Human mRNA for KIAA0355 gene, complete cds
   1.1e-40:305:83
   Hs.153014:AB002353
R-NT2RP3000232
   ESTs, Weakly similar to kruppel-related zinc finger protein [H.sapiens]
   5.7e-65:386:90
   Hs.4841:AI279875
R-NT2RP3000304
   Homo sapiens LDL receptor-related protein 6 (LRP6) mRNA, complete cds
   1.1e-109:541:97
   Hs.23672:AF074264
R-NT2RP3000378
   EST
   2.0e-05:112:74
   Hs.137268:T39311
R-NT2RP3000436
   EST
   1.2e-08:347:62
   Hs.158830:AI377454
R-NT2RP3000444
   ESTs
   3.3e-70:314:99
   Hs.57973:AI263207
R-NT2RP3000460
   EST
   1.9e-50:294:92
   Hs.7260:T23737
R-NT2RP3000481
   PROBABLE G PROTEIN-COUPLED RECEPTOR GPR6
   1.0:183:59
   Hs.46332:U18549
R-NT2RP3000616
   ESTs
   3.0e-71:309:93
   Hs.41296:N71923
R-NT2RP3000645
   ESTs
   1.5e-101:550:92
   Hs.21910:AA020743
R-NT2RP3000652
   ESTs
   6.6e-88:411:100
   Hs.43134:AA766138
R-NT2RP3000676
   Homo sapiens mRNA for KIAA0446 protein, complete cds
   1.0e-104:542:94
   Hs.158286:AB007915
R-NT2RP3000677
   ESTs
   0.33:307:59
   Hs.133022:AI374739
R-NT2RP3000721
   ESTs
   1.6e-75:390:90
   Hs.83575:N28730
R-NT2RP3000789
   ESTs
   1.5e-71:340:99
   Hs.37776:H93038
R-NT2RP3000818
   ESTs
   1.9e-52:330:88
   H:s.111052:H80504
R-NT2RP3000820
   EST
   9.1e-05:82:74
   Hs.124352:AA830406
R-NT2RP3000838
   Homo sapiens mRNA for KIAA0638 protein, partial cds
   1.5e-100:522:94
   Hs.77864:AB014538
R-NT2RP3000871
   ESTs
   3.9e-76:424:91
   Hs.121642:AA772262
R-NT2RP3000907
   ESTs, Weakly similar to PROBABLE CATION-TRANSPORTING ATPASE YEL031W [Saccharomyces cerevisiae]
   4.5e-95:493:94
   Hs.9275:AA973284
R-NT2RP3000921
   ESTs
   4.1e-52:283:94
   Hs.49714:AA442453
R-NT2RP3001012
   Homo sapiens mRNA for CMP-sialic acid transporter, complete cds
   0.60:250:61
   Hs.82921:D87969
R-NT2RP3001044
   ESTs
   3.5e-106:547:95
   Hs.12305:AA166889
R-NT2RP3001061
   ESTs
   1.3e-96:453:99
   Hs.4916:AI149707
R-NT2RP3001159
   ESTs, Weakly similar to T13F2.1 [C.elegans]
   3.8e-47:302:90
   Hs.6281:AA523081
R-NT2RP3001170
   Homo sapiens mRNA for KIAA0784 protein, partial cds
   2.8e-118:561:98
   Hs.3657:AB018327
R-NT2RP3001195
   ESTs
   1.5e-40:461:72
   Hs.152438:AI334078
R-NT2RP3001240
   EST
   1.9e-50:294:92
   Hs.7260:T23737
R-NT2RP3001271
   ESTs
   1.1e-77:432:92
   Hs.12211:AA908631
R-NT2RP3001322
   ESTs
   0.25:331:60
   Hs.44330:N32264
R-NT2RP3001542
   EST
   0.0032:432:58
   Hs.148107:AA693476
R-NT2RP3001560
   EST
   3.5e-50:281:93
   Hs.101727:H16171
R-NT2RP3001592
   ESTs
   3.2e-65:344:93
   Hs.28964:AA715101
R-NT2RP3001685
   EST
   3.0e-37:165:81
   Hs.160895:AI365871
R-NT2RP3001738
   ESTs, Weakly similar to T13F2.1 [C.elegans]
   3.8e-47:302:90
   Hs.6281 :AA523081
R-NT2RP3001754
   EST
   0.00043:104:69
   Hs.148331:AA911426
R-NT2RP3001858
   ESTs
   7.6e-93:502:93
   Hs.153524:AA533582
R-NT2RP3001976
   ESTs
   5.0e-104:516:96
   Hs.35461:AA777644
R-NT2RP3002015
R-NT2RP3002160
   ESTs
   1.4e-34:178:99
   Hs.130783:AI263114
R-NT2RP3002281
   Homo sapiens mRNA for KIAA0765 protein, partial cds
   3.5e-83:446:93
   Hs.62318:AB018308
R-NT2RP3002286
   ESTs
   2.1e-103:513:97
   Hs.58570:AA521423
R-NT2RP3002311
   ESTs
   1.4e-108:538:96
   Hs.3741:AI057614
R-NT2RP3002324
   ESTs
   3.7e-102:483:99
   Hs.99668:AA829521
R-NT2RP3002342
   ESTs, Weakly similar to transporter protein [H.sapiens]
   2.0e-60:339:95
   Hs.18272:N78499
R-NT2RP3002353
   ESTs
   6.8e-98:484:97
   Hs.9732:AA527784
NNNNNNNNNNNNNN
   Homo sapiens mRNA for KIAA0788 protein, partial cds
   2.7e-29:214:85
   Hs.2397:Z70200
NNNNNNNNNNNNNN
   ESTs
   3.0e-72:389:94
   Hs.32246:AA464020
R-NT2RP3002448
   ESTs, Weakly similar to Y48E1B.t [C.elegans]
   1.0e-19:131:75
   Hs.8715:H58021
R-NT2RP3002571
   ESTs
   1.1e-78:407:95
   Hs.27356:AA740928
R-NT2RP3002664
   ESTs
   1.2e-56:341:90
   Hs.23308:AA115020
R-NT2RP3002721
   EST
   2.8e-41:302:82
   Hs.124936:AA825548
R-NT2RP3002737
   EST
   1.7e-51:267:97
   Hs.161348:AI422470
R-NT2RP3002738
   ESTs, Weakly similar to enhancer of filmentation 1 [H.sapiens]
   1.7e-106:530:96
   Hs.4894:AI191323
R-NT2RP3002790
R-NT2RP3002836
   ESTs
   4.6e-49:282:92
   Hs.107979:AA146994
R-NT2RP3002887
   ESTs
   6.3e-98:516:94
   Hs.11900:AA535065
R-NT2RP3002900
   ESTs
   2.0e-29:155:99
   Hs.153329:AA112325
R-NT2RP3002958
   Homo sapiens clone 23851 mRNA sequence
   6.6e-119:575:98
   Hs.10065:AF035313
R-NT2RP3002983
   ESTs
   1.1e-61:374:90
   Hs.17834:AA128246
R-NT2RP3003000
   Homo sapiens T-type calcium channel alpha-1 subunit mRNA, complete cds
   4. le-65:358:94
   Hs.122359:AF051946
R-NT2RP3003076
   ESTs
   2.6e-95:507:93
   Hs.21910:AA020743
R-NT2RP3003354
   ESTs, Moderately similar to !!!! ALU SUBFAMILY SQ WARNING ENTRY !!!! [H.sapiens]
   2.1e-78:385:96
   Hs.92177:AI207792
R-NT2RP3003448
   ESTs
   6.7e-105:521:96
   Hs.106833:AA470128
R-NT2RP3003469
   ESTs
   1.1e-91:461:96
   Hs.75425: AA149434
R-NT2RP3003473
R-NT2RP3003527
   Homo sapiens mRNA for protein kinase Dyrk1B
   1.6e-92:445:97
   Hs.130988:Y17999
R-NT2RP3003532
   ESTs
   0.022:193:63
   Hs.122593:Z99400
R-nnnnnnnnnnnn
   EST
   0.036:279:59
   Hs.158745:AI375513
R-NT2RP3003559
   ESTs
   9.8e-106:513:97
   Hs.44970:AI061464
R-NT2RP3003614
   Homo sapiens mRNA, chromosome 1 specific transcript KIAA0510
   0.00016:113:69
   Hs.92660:AB007979
R-NT2RP3003729
   ESTs
   1.2e-43:289:86
   Hs.106401:R50967
R-NT2RP3003849
   ESTs
   5.4e-91:435:98
   Hs.144840:AI221746
R-NT2RP3003874
   ESTs
   0.21:323:59
   Hs.42919:AA805764
R-NT2RP3003963
   ESTs
   1.7e-90:438:97
   Hs.105894:AA564110
R-NT2RP3004000
   ESTs
   2.9e-101:559:91
   Hs.21910:AA020743
R-NT2RP3004025
   ESTs
   2.3e-108:517:98
   Hs.15356:AA911109
R-NT2RP3004075
   ESTs
   7.4e-84:453:93
   Hs.22412:AA523036
R-NT2RP3004083
   ESTs, Weakly similar to R06B9.b [C.elegans]
   4.2e-84:474:91
   Hs.30432:W28988
R-NT2RP3004090
   ESTs
   1.0:207:61
   Hs.92832:AA631027
R-NT2RP3004119
   EST
   1.8e-50:248:99
   Hs.162023:AA506128
R-NT2RP3004130
   ESTs
   1.1e-103:520:96
   Hs.10491:W28968
R-NT2RP3004133
   ESTs
   4.7e-104:545:93
   Hs.15727:H98190
R-NT2RP3004202
   ESTs
   1.1e-98:471:98
   Hs.61884:AI335985
R-NT2RP3004294
   ESTs, Weakly similar to R06B9.b [C.elegans]
   2.4e-96:500:94
   Hs.30432:W28988
R-NT2RP3004309
   ESTs, Weakly similar to T13F2.1 [C.elegans]
   3.5e-48:308:90
   Hs.6281:AA523081
R-NT2RP3004321
   ESTs
   2.6e-99:494:97
   Hs.19306:N53491
R-NT2RP3004345
   ESTs
   5.4e-95:444:99
   Hs.107149:AI379497
R-NT2RP3004355
   ESTs
   3.9e-99:490:97
   Hs.43410:N23651
R-NT2RP3004374
   ESTs
   1.2e-90:462:95
   Hs.75425:AA149434
R-NT2RP3004406
   ESTs
   1.9e-100:502:96
   Hs.24936:AA479402
R-NT2RP3004481
   ESTs
   1.6e-53:370:87
   Hs.11953:AA194120
R-NT2RP3004552
   ESTs, Weakly similar to gene SEZ-6 [M.musculus]
   7.8e-92:488:93
   Hs.6314:AA522619
R-NT2RP3004625
   Homo sapiens I-1 receptor candidate protein mRNA, complete cds
   2.6e-50:352:84
   Hs.26285:AF082516
R-NT2RP3004640
   ESTs
   1.1e-105:551:94
   Hs.83348:AA527170
R-NT2RP3004647
   Homo sapiens mRNA for KIAA0446 protein, complete cds
   4.9e-111:555:96
   Hs.158286:AB007915
R-NT2RP4000108
   ESTs
   2.9e-94:479:96
   Hs.6625:AA115182
R-NT2RP4000634
   ESTs
   3.0e-120:572:98
   Hs.28827:AI125541
R-NT2RP4000962
   ESTs, Weakly similar to SPORULATION-SPECIFIC PROTEIN 1 [Saccharomyces cerevisiae]
   6.0e-17:98:98
   Hs.4789:AI418298
R-NT2RP4001001
   ESTs
   3.1e-117:567:97
   Hs.4931:AA523860
R-NT2RP4001009
   Homo sapiens mRNA for Hs Ste24p, complete cds
   1.6e-83:404:98
   Hs.25846:AB016068
R-NT2RP4001467
   5' nucleotidase (CD73)
   5.9e-113:545:97
   Hs.153952:X55740
R-NT2RP4001877
   ESTs, Highly similar to GONADOTROPIN-RELEASING HORMONE RECEPTOR [Rattus norvegicus]
   2.2e-67:375:93
   Hs.16389:AA206356
R-NT2RP4001879
R-NT2RP4002187
   EST
   0.010:117:70
   Hs.160416:AI394161
R-NT2RP4002451
   EST
   1.3e-62:386:87
   Hs.57082:H25761
R-NT2RP4002715
   ESTs
   6.9e-111:552:96
   Hs.12305:AA166889
R-NT2RP4002750
   ESTs, Moderately similar to HIGH-AFFINITY CATIONIC AMINO ACID TRANSPORTER-1 [H.sapiens]
   7.0e-109:532:97
   Hs.86362:AA205485
R-OVARC1000003
   ESTs
   1.3e-74:391:95
   Hs.105039:AA477819
R-OVARC1000090
   Homo sapiens neuronal thread protein AD7c-NTP mRNA, complete cds
   9.9e-44:471:75
   Hs.129735:AF010144
R-OVARC1000105
   60S RIBOSOMAL PROTEIN L38
   8.8e-14:83:100
   Hs.2017:Z26876
R-OVARC1000137
   ESTs
   3.0e-84:387:95
   Hs.22028:AA167715
R-OVARC1000208
   Human mRNA for KIAA0392 gene, partial cds
   2.8e-51:313:89
   Hs.40100:AB002390
R-OVARC1000255
   Spleen tyrosine kinase
   2.8e-106:510:98
   Hs.74101:L28824
R-OVARC1000275
   ESTs, Highly similar to PROBABLE PHOSPHATIDYLINOSITOL-4-PHOSPHATE 5-KINASE FAB [Saccharomyces cerevisiae]
   6.9e-105:556:94
   Hs.5748:AA608559
R-OVARC1000298
   ESTs, Weakly similar to T03G11.6 gene product [C.elegans]
   2.4e-56:338:90
   Hs.108354:W19984
R-OVARC1000307
   ESTs
   2.4e-101:563:93
   Hs.24479:N25972
R-OVARC1000313
   Homo sapiens mRNA for KIAA0573 protein, partial cds
   5.0e-98:534:93
   Hs.154023:AB011145
R-OVARC1000331
   Homo sapiens chromosome 9, P1 clone 11659
   1.0e-55:281:97
   Hs.3439:AC004472
R-OVARC1000410
   Homo sapiens clone 23767 and 23782 mRNA sequences
   3.3e-90:462:94
   Hs.8025:AF007150
R-OVARC1000439
   ESTs, Highly similar to HYPOTHETICAL 64.3 KD GTP-BINDING PROTEIN C02F5.3 IN CHROMOSOME III [Caenorhabditis elegans]
   1.6e-99:510:95
   Hs.7471:AI143226
R-OVARC1000467
R-OVARC1000529
   ESTs
   5.7e-93:461:96
   Hs.21396:AA114834
R-OVARC1000553
   ESTs
   4.3e-51:351:87
   Hs.42979:W31096
R-OVARC1000775
R-OVARC1000811
   ESTs
   1.3e-82:441:95
   Hs.73452:AA581386
R-OVARC1000853
   ESTs, Weakly similar to !!!! ALU SUBFAMILY J WARNING ENTRY ! ! ! ! [H.sapiens]
   3.1e-95:492:95
   Hs.6853:AA401703
R-OVARC1000873
   ESTs
   2.4e-46:281:91
   Hs.43857:R91358
R-OVARC1000916
   H.sapiens PISSLRE mRNA
   1.9e-112:588:94
   Hs.77313:X78342
R-OVARC1000956
   Homo sapiens mRNA for MDC3, complete cds
   0.18:259:62
   Hs.7164:AB009672
R-OVARC1000995
   EST
   6.6e-43:343:81
   Hs.149580:AI281881
R-OVARC1001030
   ESTs, Weakly similar to neuroendocrine-specific protein C [H.sapiens]
   1.5e-21:116:100
   Hs.65450:AA055913
R-OVARC1001049
   ESTs
   1.2e-70:369:95
   Hs.42949:N21131
R-OVARC1001086
   Homo sapiens cyclin T2a mRNA, complete cds
   1.3e-106:569:94
   Hs.155478:AF048731
R-OVARC1001132
   INITIATION FACTOR IF-2, MITOCHONDRIAL PRECURSOR
   0.16:170:64
   Hs.149894:L34600
R-OVARC1001163
   ESTs
   1.9e-39:219:94
   Hs.126067:AI344351
R-OVARC1001222
   ESTs
   0.62:177:63
   Hs.141162:H66213
R-OVARC1001260
   ESTs
   2.1e-79:425:94
   Hs.105039:AA477819
R-OVARC1001336
   ESTs
   9.2e-75:439:91
   Hs.105039:AA477819
R-OVARC1001338
   ESTs
   2.3e-19:139:92
   Hs.7978:W05059
R-OVARC1001569
   ESTs
   2.4e-83:412:97
   Hs.21396:AA114834
R-OVARC1001570
   ESTs
   2.6e-49:280:94
   Hs.3854:R12478
R-OVARC1001596
   EST
   8.2e-15:93:97
   Hs.136918:AA811543
R-OVARC1001607
   ESTs
   0.019:413:56
   Hs.24684:AA587245
R-OVARC1001725
   ESTs
   1.4e-96:504:95
   Hs.23754:N29716
R-OVARC1001727
R-OVARC1001807
   Hormone receptor (growth factor-inducible nuclear protein N10)
   8.5e-78:425:94
   Hs.1119:D49728
R-OVARC1001833
   ESTs
   1.0e-63:325:96
   Hs.126912:AA469087
R-OVARC1001991
   ESTs
   1.3e-92:467:95
   Hs.26506:AI348000
R-OVARC1002058
   ESTs
   2.5e-89:512:91
   Hs.58093:W63576
R-OVARC1002178
   ESTs
   3.3e-99:487:96
   Hs.136527:AI419398
R-PLACE1000033
   ESTs
   0.012:202:59
   Hs.157400:AI370528
R-PLACE1000231
   ESTs
   2.9e-56:285:96
   Hs.36688:AA603479
R-PLACE1000258
   EST
   3.4e-50:353:83
   Hs.146794:AI149478
R-PLACE1000442
   ESTs, Moderately similar to ZINC FINGER PROTEIN ZFP-36 [Homo sapiens]
   5.5e-91:437:98
   Hs.14831:AI261191
R-PLACE1000560
   ESTs
   3.7e-60:317:94
   Hs.65713:AI269328
R-PLACE1000740
   ESTs
   4.2e-67:362:94
   Hs.163434:T79849
R-PLACE1000912
   ESTs
   3.4e-57:329:92
   Hs.121907:R66773
R-PLACE1000914
   ESTs
   2.6e-71:419:89
   Hs.90809:AA010979
R-PLACE1000927
   ESTs, Weakly similar to N-methyl-D-aspartate receptor-associated protein [D.melanogaster]
   7.8e-111:545:97
   Hs.8661:AI189791
R-PLACE1000986
   ESTs
   1.5e-91:431:99
   Hs.42458:AA452296
R-PLACE1001016
   ESTs
   3.4e-45:231:97
   Hs.121013:AA324765
R-PLACE1001100
   Homo sapiens nephrin (NPHS1) mRNA, complete cds
   3.5e-43:321:83
   Hs.128834:AF035835
R-PLACE1001114
   Human clone 23732 mRNA, partial cds
   1.6e-42:305:83
   Hs.81281:U79258
R-PLACE1001123
   ESTs, Highly similar to UBIQUITIN-CONJUGATING ENZYME E2-28.4 KD [Saccharomyces
   cerevisiae]
   1.2e-51:310:90
   Hs.7773:AA127629
R-PLACE1001183
   Human mRNA for KIAA0308 gene, partial cds
   0.88:182:65
   Hs.10351:AB002306
R-PLACE1001229
   ESTs
   5.2e-90:471:95
   Hs.18271:N92774
R-PLACE1001231
R-PLACE1001340
   Homo sapiens mRNA for KIAA0719 protein, complete cds
   6.6e-53:265:98
   Hs.21198:AB018262
R-PLACE1001401
   ESTs
   1.9e-72:362:96
   Hs.20161:AA056410
R-PLACE1001407
   ESTs
   2.1e-36:249:85
   Hs.23579:W38893
R-PLACE1001464
   5' nucleotidase (CD73)
   1.0e-91:457:96
   Hs.153952:X55740
R-PLACE1001500
   ESTs, Weakly similar to DNA helicase Q1 [H.sapiens]
   2.0e-19:150:87
   Hs.154199:AA155882
R-PLACE1001516
   EST
   1.9e-11:109:82
   Hs.137486:AA425225
R-PLACE1001536
   Human BRCA2 region, mRNA sequence CG016
   0.28:146:63
   Hs.112434:U50529
R-PLACE1001564
   ESTs
   6.3e-14:109:88
   Hs.26519:AA442703
R-PLACE1001655
   Homo sapiens Shab-related delayed-rectifier K+ channel alpha subunit (KCNS3) mRNA, complete cds
   1.2e-118:578:97
   Hs.47584:AF043472
R-PLACE1001788
   ESTs
   8.4e-38:205:95
   Hs.23800:AA524095
R-PLACE1001795
   ESTs, Weakly similar to HYPOTHETICAL 30.6 KD PROTEIN IN SCP160-MRPL8 INTERGENIC REGION PRECURSOR [S.cerevisiae]
   2.5e-77:392:96
   Hs.7745:H92988
R-PLACE1001836
   ESTs
   1.5e-49:296:90
   Hs.17691:H60366
R-PLACE1001918
   ESTs, Weakly similar to multispanning membrane protein [H.sapiens]
   2.0e-42:304:85
   Hs.110439:N93209
R-PLACE1001949
R-PLACE1002080
   Small inducible cytokine A5 (RANTES)
   8.5e-41:296:82
   Hs. 155464:AF088219
R-PLACE1002095
   ESTs
   8.5e-25:227:81
   Hs.110488:AA034235
R-PLACE1002153
   Homo sapiens TACC2 protein (TACC2) mRNA, partial cds
   1.5e-101:514:95
   Hs.90415:AF095791
R-PLACE1002329
   ESTs
   8.7e-48:257:94
   Hs.126062:AA411593
R-PLACE1002355
   ESTs
   7.7e-71:362:95
   Hs.120866:AI076780
R-PLACE1002374
   Cathepsin L
   8.4e-103:501:97
   Hs.78056:X12451
R-PLACE1002518
   ESTs
   6.9e-97:471:97
   Hs.104893:AA576941
R-PLACE1002547
   Homo sapiens mRNA for KIAA0719 protein, complete cds
   6.5e-55:276:97
   Hs.21198:AB018262
R-PLACE1002726
   Human DNA-binding protein ABP/ZF mRNA, complete cds
   3.8e-39:212:94
   Hs.86185:U82613
R-PLACE1002905
   Homo sapiens mRNA for KIAA0563 protein, complete cds
   2.9e-41:330:81
   Hs.15731:AB011135
R-PLACE1002911
R-PLACE1002967
   ESTs
   1.0e-43:384:77
   Hs.132722:AA618531
R-PLACE1003135
   ESTs
   8.2e-94:462:97
   Hs.23643:AI299952
R-PLACE1003163
   Homo sapiens DBI-related protein mRNA, complete cds
   3.5e-110:541:96
   Hs.15250:AF069301
R-PLACE1003407
   Homo sapiens putative transmembrane protein (CLN5) mRNA, complete cds
   5.5e-49:287:91
   Hs.30213:AF068227
R-PLACE1003428
   ESTs, Moderately similar to BIOTINIDASE PRECURSOR [Homo sapiens]
   6.8e-83:406:97
   Hs.17586:AA461448
R-PLACE1003438
   ESTs
   2.9e-83:463:92
   Hs.11067:H30385
R-PLACE1003460
   ESTs
   7.0e-27:187:87
   Hs.18763:H56292
R-nnnnnnnnnnnn
   ESTs
   1.7e-52:265:97
   Hs.114049:AI091839
R-PLACE1003573
   Human mRNA for KIAA0160 gene, partial cds
   0.13:102:69
   Hs.79880:D63881
R-PLACE1003598
   ESTs
   8.0e-39:210:95
   Hs.26286:AA040823
R-PLACE1003644
   EST
   0.47:84:73
   Hs.105856:AA551478
R-PLACE1003737
   ESTs
   1.1e-77:366:100
   Hs.62699:AA707766
R-PLACE1003772
   Human mRNA for KIAA0355 gene, complete cds
   6.1e-27:551:65
   Hs.153014:AB002353
R-PLACE1003839
   ESTs
   0.019:244:59
   Hs.137825:AA778400
R-PLACE1003845
   EST
   5.3e-79:416:93
   Hs.150153:AI300555
R-PLACE1003852
   Homo sapiens mRNA for KIAA0758 protein, partial cds
   2.2e-87:439:96
   Hs.22039:AB018301
R-PLACE1004028
   Sialyltransferase 4A (beta-galactosidase alpha-2,3-sialytransferase)
   0.73:128:71
   Hs.60617:L13972
R-PLACE1004078
   ESTs
   1.7e-69:353:96
   Hs.142075:AA654529
R-PLACE1004166
   ESTs
   1.7e-64:362:92
   Hs.10177:AA191619
R-nnnnnnnnnnnn
   EST
   0.98:59:71
   Hs.132255:AI027216
R-PLACE1004199
   ESTs
   1.3e-55:279:97
   Hs.147585:AI217699
R-PLACE1004279
   ESTs
   3.7e-68:373:93
   Hs.145531:H87181
R-PLACE1004282
R-PLACE1004305
   Homo sapiens mRNA for KIAA0740 protein, complete cds
   6.4e-79:377:99
   Hs.15099:AB018283
R-PLACE1004441
   ESTs
   1.8e-46:244:95
   Hs.107082:R63714
R-PLACE1004450
R-PLACE1004482
   ESTs
   1.2e-92:491:93
   Hs.17840:AI269915
R-PLACE1004492
   ESTs
   6.1e-54:278:95
   Hs.55862:A1341676
R-PLACE1004519
   ESTs
   3.1e-25:133:100
   Hs.47378:AI193598
R-PLACE1004520
   Pregnancy-specific beta-1 glycoprotein 4
   2.8e-66:390:89
   Hs.108936:X17097
R-PLACE1004630
   ESTs
   7.3e-58:338:92
   Hs.155506:AI281549
R-PLACE1004637
   ESTs
   1.1e-37:309:82
   Hs.20102:AA150165
R-PLACE1004648
   ESTs
   2.3e-67:340:96
   Hs.69321:AA633240
R-PLACE1004816
   Homo sapiens mRNA for Hakata antigen, complete cds
   1.8e-104:586:90
   Hs.9225:D88587
R-PLACE1004887
   ESTs, Weakly similar to GOLIATH PROTEIN [D.melanogaster]
   2.6e-30:222:86
   Hs.18557:AA203416
R-PLACE1005003
   ESTs
   0.99:123:68
   Hs.146244:AI276718
R-PLACE1005005
   Homo sapiens ubiquitin conjugating enzyme G2 (UBE2G2) mRNA, complete cds
   6.8e-58:299:95
   Hs.151614:AF032456
R-PLACE1005031
   ESTs
   4.7e-57:325:92
   Hs.31196:H13265
R-PLACE1005239
   Homo sapiens mRNA for HIRIP3 protein, clone pH4-17
   1.4e-86:450:93
   Hs.26484:AJ223351
R-PLACE1005250
   ESTs, Moderately similar to maternal transcript Maid [M.musculus]
   1.7e-106:521:97
   Hs.36794:AI038407
R-PLACE1005383
   Homo sapiens UP50 mRNA, complete cds
   6.3e-79:471:88
   Hs.11494:AF093118
R-PLACE1005410
   EST
   2.3e-49:296:90
   Hs.7260:T23737
R-PLACE1005426
   Pregnancy-specific beta-1 glycoprotein 4
   8.0e-109:576:93
   Hs.108936:X17097
R-PLACE1005519
   ESTs
   5.4e-108:569:93
   Hs.23643:AI299952
R-PLACE1005539
   ESTs, Weakly similar to p20 protein [R.norvegicus]
   4.5e-05:107:77
   Hs.56874:W61026
R-PLACE1005544
   ESTs
   4.2e-57:280:98
   Hs.155391:AA451633
R-PLACE1005569
   ESTs
   2.7e-90:470:94
   Hs.8904:AI129815
R-PLACE1005601
R-PLACE1005660
   ESTs, Highly similar to HYPOTHETICAL 64.3 KD GTP-BINDING PROTEIN C02F5.3 IN CHROMOSOME III [Caenorhabditis elegans]
   1.4e-91:483:93
   Hs.7471:AI143226
R-PLACE1005669
   ESTs
   1.7e-84:438:95
   Hs.18271:N92774
R-PLACE1005682
   ESTs
   6.3e-80:482:88
   Hs.128679:AI160081
R-PLACE1005725
   ESTs
   1.5e-98:519:93
   Hs.11360:AI147467
R-PLACE1005736
   ESTs
   3.1e-110:561:95
   Hs.24111:AI346026
R-PLACE1005745
   ESTs
   2.4e-96:473:97
   Hs.126935:AA603217
R-PLACE1005768
   Cytochrome P450, subfamily I (aromatic compound-inducible), polypeptide 2
   4.0e-46:387:77
   Hs.1361:M55053
R-PLACE1005815
   Homo sapiens PYRIN (MEFV) mRNA, complete cds
   7.1e-56:324:79
   Hs.113283:AF018080
R-PLACE1005878
   ESTs
   3.1e-75:388:94
   Hs.153483:AA569128
R-PLACE1005927
   ESTs
   4.3e-64:403:87
   Hs.126899:N50907
R-PLACE1006071
   ESTs
   5.3e-96:510:93
   Hs.24398:AI262946
R-PLACE1006073
   Homo sapiens mRNA for glucuronyltransferase I, complete cds
   3.0e-97:504:93
   Hs.26492:AB009598
R-PLACE1006079
   ESTs
   3.1e-79:453:90
   Hs.134194:AI142137
R-PLACE1006093
   ESTs
   1.3e-78:378:98
   Hs.129327:AI201040
R-nnnnnnnnnnnn
R-PLACE1006219
   EST
   1.6e-75:412:92
   Hs.150153:AI300555
R-PLACE1006277
   ESTs
   2.8e-92:493:93
   Hs.8904:AI129815
R-PLACE1006290
   ESTs
   2.8e-92:433:99
   Hs.23445:AA489015
R-PLACE1006443
   ESTs
   2.5e-73:419:91
   Hs.90809:AA010979
R-PLACE1006515
   Homo sapiens mRNA for KIAA0576 protein, partial cds
   6.9e-78:413:94
   Hs.14687:AB011148
R-PLACE1006716
   ESTs
   4.8e-44:262:88
   Hs.8503:AI393886
R-PLACE1006786
   ESTs
   6.3e-89:431:98
   Hs.42458:AA452296
R-PLACE1006809
   ESTs
   1.6e-68:377:92
   Hs.8956:AI146421
R-PLACE1006959
   EST
   0.00065:211:63
   Hs.136605:AA665784
R-PLACE1007028
   ESTs
   7.4e-92:475:94
   Hs.110222:AA532444
R-PLACE1007040
   ESTs
   5.1e-103:509:97
   Hs.71190:AA524036
R-PLACE1007077
   ESTs
   1.0e-98:529:93
   Hs.24398:AI262946
R-PLACE1007081
   Human growth factor independence-1 (Gfi-1) mRNA, complete cds
   0.57:238:61
   Hs.73172:U67369
R-PLACE1007096
   ESTs
   1.2e-88:466:94
   Hs.8268:N70144
R-PLACE1007296
   EST
   4.3e-53:338:86
   Hs.147274:AI206582
R-PLACE1007591
   EST
   4.6e-76:384:97
   Hs.94445:N90719
R-PLACE1007626
   Homo sapiens unknown mRNA, complete cds
   5.0e-30:179:91
   Hs.11441:AF047439
R-PLACE1007702
   ESTs
   1.0e-52:341:87
   Hs.103382:AA026923
R-PLACE1007845
   ESTs
   2.2e-102:541:93
   Hs.15727:H98190
R-PLACE1007881
   ESTs
   4.1e-75:398:93
   Hs.55560:AI142804
R-PLACE1007971
   ESTs
   2.8e-43:304:85
   Hs.82933:AA058963
R-PLACE1008282
   ESTs, Highly similar to HEME-REGULATED EUKARYOTIC INITIATION FACTOR EIF-2-ALPHA KINASE [Oryctolagus cuniculus]
   2.2e-74:393:94
   Hs.77613:AI367385
R-PLACE1008297
   ESTs
   6.5e-101:506:96
   Hs.44274:AA523749
R-PLACE1008359
   ESTs
   1.8e-94:469:96
   Hs.160551:AI281417
R-PLACE1008469
   ESTs
   7.0e-74:421:90
   Hs.90809:AA010979
R-PLACE1008549
   ESTs
   2.0e-81:474:90
   Hs.11713:T65960
R-PLACE1008657
   ESTs
   9.5e-89:512:89
   Hs.142075:AA654529
R-PLACE1008716
   Human beta-1,2-N-acetylglucosaminyltransferase II (MGAT2) gene, complete cds
   5.6e-100:504:95
   Hs.154844:U15128
R-PLACE1008744
   ESTs, Highly similar to COMPLEMENT RECEPTOR TYPE 2 PRECURSOR [Homo sapiens]
   2.3e-107:528:96
   Hs.8963:AI379350
R-PLACE1008984
   ESTs
   2.0e-76:464:89
   Hs.40094:D12041
R-PLACE1008985
   EST, Highly similar to SYNAPTOTAGMIN B [Discopyge ommata]
   2.2e-59:343:90
   Hs.161031:H72014
R-PLACE1009067
   ESTs
   7.7e-90:503:92
   Hs.55067:AA037664
R-PLACE1009196
   EST
   0.011:243:60
   Hs.149839:AI287601
R-PLACE1009279
   Homo sapiens mRNA for serin protease with IGF-binding motif, complete cds
   5.4e-27:553:62
   Hs.75111:D87258
R-PLACE1009527
   Human DNA-binding protein ABP/ZF mRNA, complete cds
   2.7e-92:497:91
   Hs.86185:U82613
R-PLACE1009546
   ESTs
   5.9e-80:461:90
   Hs.134292:AA603031
R-PLACE1009600
   ESTs
   5.5e-98:509:93
   Hs.21015:AA428288
R-PLACE1009735
   ESTs
   1.1e-85:462:93
   Hs.48563:AA526595
R-nnnnnnnnnnnn
   ESTs
   6.8e-82:499:87
   Hs.43498:AA570507
R-PLACE1010011
   ESTs, Moderately similar to synaptonemal complex protein [M.musculus]
   2.7e-15:171:78
   Hs.31655:AI075991
R-PLACE1010078
   ESTs
   1.2e-48:267:92
   Hs.12101:AA677423
R-PLACE1010081
   Homo sapiens serine/threonine kinase RICK (RICK) mRNA, complete cds
   3.0e-106:560:93
   Hs.103755:AF027706
R-PLACE1010251
   ESTs
   0.00049:248:60
   Hs.154164:AI246893
R-PLACE1010445
   ESTs
   1.5e-90:496:92
   Hs.163999:AA778110
R-PLACE1010713
   Interleukin 1 receptor antagonist
   4.1e-07:307:59
   Hs.81134:U65590
R-PLACE1010784
   ESTs, Weakly similar to putative G-protein-coupled receptor [H.sapiens]
   1.5e-21:206:78
   Hs.29202:R71586
R-PLACE1010827
R-PLACE 1010968
   ESTs
   2.6e-75:385:95
   Hs.109884:AA766018
R-PLACE1011045
   Homo sapiens E1-like protein mRNA, complete cds
   5.3e-92:453:96
   Hs.28190:AF094516
R-PLACE1011116
   Homo sapiens embryonic lung protein (HUEL) mRNA, complete cds
   1.5e-73:385:94
   Hs.44053:AF006621
R-PLACE1011236
R-PLACE1011364
   ESTs
   2.3e-47:289:89
   Hs.6163:W26652
R-PLACE1011407
   ESTs
   1.1e-09:191:64
   Hs.118620:T60326
R-PLACE1011516
   ESTs, Weakly similar to HYPOTHETICAL 21.5 KD PROTEIN IN SEC15-SAP4 INTERGENIC REGION [S.cerevisiae]
   6.3e-75:441:88
   Hs.110978:AA843431
R-PLACE1011708
   Homo sapiens intrinsic factor-B12 receptor precursor, mRNA, complete cds
   7.7e-93:521:91
   Hs.148318:AF034611
R-PLACE1011824
   ESTs
   0.013:199:62
   Hs.44343:AA532514
R-PLACE1011978
   EST
   4.0e-97:462:98
   Hs.116391:AA644085
R-PLACE2000118
   ESTs
   1.2e-83:468:92
   Hs.110578:AA115763
R-PLACE2000219
   Homo sapiens KIAA0414 mRNA, partial cds
   2.0e-44:344:81
   Hs.127649:AB007874
R-PLACE3000181
   Human protocadherin 42 mRNA, 3' end of cds for alternative splicing PC42-8
   1.3e-82:441:94
   Hs.115642:L11369
R-PLACE3000213
   ESTs, Highly similar to COMPLEMENT RECEPTOR TYPE 2 PRECURSOR [Homo sapiens]
   2.3e-114:557:97
   Hs.8963:AI379350
R-PLACE4000354
   ESTs, Highly similar to COMPLEMENT RECEPTOR TYPE 2 PRECURSOR [Homo sapiens]
   3.4e-105:518:97
   Hs.8963:AI379350
R-PLACE4000455
   ESTs
   9.0e-57:289:96
   Hs.42458:AA452296
R-THYRO1000036
   Collagen, type IX, alpha 3
   1.3e-100:527:93
   Hs.53563:L41162
R-THYRO1000061
   ESTs
   1.8e-87:460:94
   Hs.124869:H98977
R-THYRO1000099
   ESTs
   1.2e-34:193:94
   Hs.149488:AI243816
R-THYRO1000196
   Homo sapiens Ksp-cadherin (CDH16) mRNA, complete cds
   3.7e-106:530:96
   Hs.115418:AF016272
R-THYRO1000400
   Human HU-K4 mRNA, complete cds
   0.99:227:60
   Hs.74573:U60644
R-THYRO1000580
   Homo sapiens mRNA for KIAA0628 protein, complete cds
   0.21:126:67
   Hs.43133:AB014528
R-THYRO1000584
   ESTs, Weakly similar to golgi alpha-mannosidaseII [H.sapiens]
   3.0e-106:529:96
   Hs.12183:AA888145
R-THYRO1000678
   EST
   2.9e-62:304:99
   Hs.48956:N64339
R-THYRO1000776
   ESTs
   1.3e-102:533:94
   Hs.4866:AA582196
R-THYRO1000795
   ESTs
   3.3e-98:529:92
   Hs.55263:AI344338
R-THYRO1000846
   ESTs
   1.6e-105:522:96
   Hs.135106:AI335251
R-THYRO1000866
   Homo sapiens SKB1Hs mRNA, complete cds
   1.3e-43:251:92
   Hs.12912:AF015913
R-THYRO1000956
   ESTs, Highly similar to PROBABLE G PROTEIN-COUPLED RECEPTOR APJ [Homo sapiens]
   5.2e-106:548:94
   Hs.9305:W84893
R-THYRO1000964
R-THYRO1000999
   ESTs
   1.9e-18:150:84
   Hs.111583:AA463590
R-THYRO1001063
   ESTs
   1.5e-95:464:97
   Hs.142684:AA902402
R-THYRO1001071
   ESTs
   2.5e-104:496:98
   Hs.6071:AA868544
R-THYRO1001102
R-THYRO1001113
   ESTs, Weakly similar to FER-1 [C.elegans]
   7.1e-90:446:97
   Hs.8076:AA115644
R-THYRO1001128
   ESTs
   1.9e-16:270:68
   Hs.140194:N35720
R-THYRO1001205
   Small inducible cytokine A5 (RANTES)
   1.9e-58:400:84
   Hs.155464:AF088219
R-THYRO1001237
   ESTs
   1.5e-104:532:96
   Hs.6603:AA772122
R-THYRO1001242
   EST
   1.7e-50:281:93
   Hs.101727:H16171
R-THYRO1001266
   Homo sapiens mRNA for KIAA0650 protein, partial cds
   0.00037:403:60
   Hs.8118:AB014550
R-THYRO1001327
   ESTs
   1.2e-96:530:93
   Hs.28786:AA034412
R-THYRO1001456
   ESTs, Weakly similar to Similar to phytoene desaturase [C.elegans]
   3.3e-43:257:92
   Hs.97031:AA773647
R-THYRO1001457
   ESTs, Highly similar to MYOSIN LIGHT CHAIN KINASE [Dictyostelium discoideum]
   4.8e-59:284:99
   Hs.9915:AI300083
R-THYRO1001471
   ESTs
   1.1e-67:378:93
   Hs.52113:R40587
R-THYRO1001478
R-THYRO1001495
   H.sapiens mRNA for Zinc-finger protein (ZNFpT17)
   1.6e-63:434:84
   Hs.32954:X65233
R-THYRO1001523
   ESTs
   5.8e-75:388:96
   Hs.6527:R21517
R-THYRO1001529
   ESTs
   1.1e-25:184:87
   Hs.18441:AA005104
R-THYRO1001593
   ESTs
   4.7e-34:182:98
   Hs.8312:AA813022
R-THYRO1001608
   ESTs
   2.8e-107:547:95
   Hs.23765:AA524283
R-THYRO1001641
   Homo sapiens clone 24448 unknown mRNA, partial cds
   1.1e-111:562:96
   Hs.4973:AF070638
R-THYRO1001700
   ESTs
   1.3e-78:407:95
   Hs.86987:N99896
R-THYRO1001702
   ESTs
   4.3e-98:566:92
   Hs.119447:AA524436
R-THYRO1001725
   ESTs
   1.3e-84:424:96
   Hs.38039:AI360128
R-THYRO1001770
   ESTs
   1.0e-62:325:97
   Hs.20137:R08273
R-THYRO1001803
   ESTs
   6.8e-90:456:96
   Hs.134438:R42585
R-Y79AA1000030
   ESTs, Weakly similar to HYPOTHETICAL PROTEIN KIAA0168 [H.sapiens]
   2.4e-98:515:94
   Hs.32822:AI194045
R-Y79AA1000127
   ESTs, Weakly similar to ! ! ! ! ALU SUBFAMILY J WARNING ENTRY !!!! [H.sapiens]
   1.1e-57:307:95
   Hs.83513:W05849
R-Y79AA1000207
   EST
   1.0e-97:411:96
   Hs.141431:N21286
R-Y79AA1000226
   ESTs, Highly similar to HYPOTHETICAL 52.8 KD PROTEIN T05E11.5 IN CHROMOSOME IV [Caenorhabditis elegans]
   7.2e-102:545:94
   Hs.11221:AI192291
R-Y79AA1000270
   Human mRNA for ORF, Xq terminal portion
   3.3e-107:564:93
   Hs.6551:D16469
R-Y79AA1000426
   H.sapiens mRNA for activin beta-C chain
   2.5e-10:217:66
   Hs.83267:X82540
R-Y79AA1000521
   Homo sapiens N-methyl-D-aspartate receptor 2D subunit precursor (NMDAR2D) mRNA, complete cds
   0.73:257:59
   Hs.113286:U77783
R-Y79AA1000750
   ESTs
   4.3e-75:391:95
   Hs.157192:W84862
R-Y79AA1000776
   ESTs
   3.5e-56:303:95
   Hs.118559:AA887084
R-Y79AA1000777
   ESTs, Weakly similar to LIS-1 protein [H.sapiens]
   9.5e-98:515:95
   Hs.59461:W93217
R-Y79AA1000876
   EST
   2.7e-23:173:84
   Hs.135872:AI037885
R-Y79AA1000959
   Homo sapiens Hsp70 binding protein HspBP1 mRNA, complete cds
   3.4e-80:453:92
   Hs.53066:AF093420
R-Y79AA1000967
   ESTs
   7.3e-86:461:93
   Hs.6262:T89093
R-Y79AA1001013
   ESTs
   1.4e-115:566:97
   Hs.108408:N31922
R-Y79AA1001056
   ESTs, Moderately similar to maternal transcript Maid [M.musculus]
   8.7e-111:557:95
   Hs.36794:AI038407
R-Y79AA1001062
   ESTs
   0.0021:365:59
   Hs.106129:AA292171
R-Y79AA1001090
   ESTs
   5.0e-52:255:99
   Hs.106214:AI123831
R-Y79AA1001212
   Homo sapiens SL15 protein mRNA, complete cds
   1.8e-83:407:97
   Hs.6710:AF038961
R-Y79AA1001264
   ESTs, Highly similar to DNAJ PROTEIN HOMOLOG 2 [Homo sapiens]
   2.8e-111:552:96
   Hs.62489:AI057091
R-Y79AA1001272
   Zinc finger protein, X-linked
   0.019:317:59
   Hs.2074:X59739
R-Y79AA1001328
   ESTs
   3.6e-67:385:92
   Hs.127792:AI421472
R-Y79AA1001426
   ESTs
   2.0e-13:92:93
   Hs.105607:AA478379
R-Y79AA1001430
   Homo sapiens mRNA for KIAA0469 protein, complete cds
   2.0e-112:555:96
   Hs.7764:AB007938
R-Y79AA1001523
   EST
   1.7e-07:120:73
   Hs.130984:AI015430
R-Y79AA1001530
   Homo sapiens RRM RNA binding protein Gry-rbp (GRY-RBP) mRNA, complete cds
   0.030:169:63
   Hs.155489:AF037448
R-Y79AA1001592
   ESTs
   5.0e-60:340:91
   Hs.87019:AA760977
R-Y79AA1001727
   ESTs
   6.1e-101:547:93
   Hs.7404:W29012
R-Y79AA1001787
   ESTs
   8.8e-84:449:95
   Hs.128866:AA977749
R-Y79AA1001795
   Homo sapiens mRNA for GalT4 protein
   9.9e-110:541:97
   Hs.21495:AL031228
R-Y79AA1001799
   ESTs, Highly similar to MITOCHONDRIAL RNA SPLICING PROTEIN MSR4 [Saccharomyces cerevisiae]
   1.6e-94:567:90
   Hs.34401:AA447775
R-Y79AA1001803
   ESTs, Highly similar to SECRETOGRANIN III PRECURSOR [Mus musculus]
   1.2e-86:509:90
   Hs.22215:AI371482
R-Y79AA1001863
   ESTs
   1.4e-23:268:73
   Hs.131613:AI190576
R-Y79AA1002022
   ESTs
   8.9e-97:462:98
   Hs.6140:D52151
R-nnnnnnnnnnnn
R-nnnnnnnnnnnn
   Homo sapiens DNA recombination and repair protein (MRE11B) mRNA, complete cds
   0.00075:456:59
   Hs.153855:AF022778
R-Y79AA1002213
   Human mRNA for KIAA0392 gene, partial cds
   6.2e-45:304:85
   Hs.40100:AB002390
R-Y79AA1002334
   ESTs
   7.7e-91:495:92
   Hs.90804:W28091
R-Y79AA1002373
   Human kpni repeat mrna (cdna clone pcd-kpni-8), 3' end
   5.2e-98:545:91
   Hs.103948:K00627
R-Y79AA1002376
   ESTs
   2.0e-91:455:97
   Hs.153375:AI287812
R-Y79AA1002378
   ESTs, Highly similar to ZINC FINGER PROTEIN ZFP-35 [Mus musculus]
   9.4e-15:131:83
   Hs.20082:W89121
R-Y79AA1002381
   ESTs, Highly similar to CELL DIVISION CONTROL PROTEIN 2 HOMOLOG [Plasmodium falciparum (isolate k1/ thailand)]
   1.5e-104:531:95
   Hs.26322:AA156858

### Homology search result 10

Data obtained by the homology search for full length nucleotide sequences and deduced amino acid sequences. In the result of the search shown below, both units, aa and bp, are used as length units for the sequences to be compared. Each data includes Clone name, Definition in matching data, P value, Length of sequence to be compared, Homology, and Accession number (No.) of matching data. These items are shown in this order, separated by a double-slash mark, //.
C-HEMBA1000006//Homo sapiens mRNA; cDNA DKFZp564G1762 (from clone DKFZp564G1762).//0//1230bp//92%//AB026894
C-nnnnnnnnnnnn//GAMETOGENESIS EXPRESSED PROTEIN GEG-154.//2.30E-71//344aa//50%//P50636
C-HEMBA1000121//HYPOTHETICAL 68.7 KD PROTEIN ZK757.1 IN CHROMOSOME III.//4.80E-05//83aa//27%//P34679
C-HEMBA1000128//PATHOGENESIS-RELATED PROTEIN 1 PRECURSOR (PR-1).//3.20E-07//89aa//34%//P33154
C-HEMBA1000275
C-HEMBA1000300
C-HEMBA1000349//ATP-BINDING CASSETTE TRANSPORTER 1.//5.30E-65//352aa//39%//P41233
C-HEMBA1000443//Homo sapiens CGI-96 protein mRNA, complete cds.//4.70E-129//686bp//91%//AF151854
C-HEMBA1000590//Homo sapiens mRNA for matrilin-4, partial.//2.00E-273//1254bp//99%//AJ007581
C-HEMBA1000634//Homo sapiens T-cell activation protein (PGR1) gene, complete cds.//0//994bp//99%//AF116272
C-HEMBA1000713//Homo sapiens 10kD protein (BC10) mRNA, complete cds.//0//1254bp//99%//AF053470
C-HEMBA1000745//COLLAGEN ALPHA 1(I) CHAIN (FRAGMENTS).//2.00E-07//445aa//27%//P02454
C-HEMBA1000907
C-HEMBA1000940//GAP JUNCTION ALPHA-3 PROTEIN (CONNEXIN 44) (CX44).//2.90E-39//362aa//31%//P41987
C-HEMBA1000962
C-HEMBA1001221//AGRIN PRECURSOR.//2.50E-25//294aa//29%//P31696
C-HEMBA1001228//Human germline oligomeric matrix protein (COMP) mRNA, complete cds.//7.80E-286//1105bp//94%//L32137
C-HEMBA1001297
C-HEMBA1001390//Mus musculus polymerase I-transcript release factor mRNA, complete cds.//2.50E-57//464bp//82%//AF036249
C-HEMBA1001563
C-HEMBA1001621//PROBABLE G PROTEIN-COUPLED RECEPTOR APJ.//3.50E-123//259aa//89%//P35414
C-nnnnnnnnnnnn//ZINC FINGER PROTEIN 84 (ZINC FINGER PROTEIN HPF2).//2.40E-85//293aa//50%//P51523
C-HEMBA1001878//Homo sapiens U5 snRNP-specific 40 kDa protein mRNA, complete cds.//0//1488bp//99%//AF090988
C-HEMBA1002131//PROCOLLAGEN-LYSINE,2-OXOGLUTARATE 5-DIOXYGENASE 1//4.10E-10//140aa//30%//P24802
C-HEMBA1002163//HYPOTHETICAL 40.7 KD PROTEIN IN DAK1-ORC1 INTERGENIC REGION.//9.40E-28//309aa//30%//Q04651
C-HEMBA1002164
C-HEMBA1002167//Rattus norvegicus neuroligin I mRNA, complete cds.//1.30E-305//1643bp//91%//U22952
C-HEMBA1002178//PROCOLLAGEN-LYSINE,2-OXOGLUTARATE 5-DIOXYGENASE 1 PRECURSOR (EC 1.14.11.4) (LYSYL HYDROXYLASE 1) (LH1).//3.70E-10//140aa//30%//P24802
C-nnnnnnnnnnnn//Human glycyl-tRNA synthetase mRNA, complete cds.//0//2380bp//99%//U09587
C-HEMBA1002195//VEGETATIBLE INCOMPATIBILITY PROTEIN HET-E-1.//8.80E-23//221aa//31%//Q00808
C-HEMBA1002227//Homo sapiens mRNA for 80K-L protein, complete cds.//0//1324bp//98%//D10522
C-HEMBA1002239
C-HEMBA1002316
C-HEMBA1002420
C-HEMBA1002421//Human heparan sulfate proteoglycan (HSPG) core protein, 3' end.//0//2097bp//99%//J04621
C-HEMBA1002524//Human MHC Class I region proline rich protein mRNA, complete cds.//0//1763bp//95%//U63336
C-HEMBA1002551//PUTATIVE SERINE/THREONINE-PROTEIN KINASE PKWA (EC 2.7.1.-).//9.80E-08//110aa//37%//P49695
C-HEMBA1002767//Homo sapiens chromosome 1p33-p34 beta-1,4-galactosyltransferase mRNA, complete cds.//0//1497bp//99%//AF038660
C-HEMBA1002992//UBIQUITIN-LIKE PROTEIN DSK2.//2.00E-21//216aa//35%//P48510
C-HEMBA1003047//Homo sapiens intrinsic factor-B12 receptor precursor, mRNA, complete cds.//0//1768bp//99%//AF034611
C-HEMBA1003072//Gallus gallus single-strand DNA-binding protein csdp mRNA, partial cds.//3.30E-93//927bp//73%//U68380
C-HEMBA1003101//Homo sapiens tyrosylprotein sulfotransferase-2 mRNA, complete cds.//0//1854bp//99%//AF049891
C-HEMBA1003230//Homo sapiens fibulin-5.//5.60E-308//1398bp//99%//AJ133490
C-HEMBA1003294
C-HEMBA1003315//Mus musculus mRNA for DNA helicase, complete cds.//6.30E-250//1426bp//88%//AB013912
C-HEMBA1003392//Homo sapiens LDL receptor-related protein 6 (LRP6) mRNA, complete cds.//0//1721bp//100%//AF074264
C-HEMBA1003399//MVP1 PROTEIN.//2.30E-15//279aa//23%//P40959
C-HEMBA1003487
C-HEMBA1003530//S.scrofa mRNA for BM88 antigen.//1.20E-60//900bp//66%//X82027
C-HEMBA1003602//Homo sapiens CGI-67 protein mRNA, complete cds.//3.50E-70//732bp//66%//AF151825
C-HEMBA1003732//SFT2 PROTEIN.//1.50E-06//162aa//30%//P38166
C-HEMBA1003945//Homo sapiens hypothetical 43.2 Kd protein mRNA, complete cds.//8.90E-287//757bp//97%//AF077030
C-HEMBA1004110//Homo sapiens intersectin short form mRNA, complete cds.//0//2033bp//99%//AF064243
C-HEMBA1004250//CADHERIN-RELATED TUMOR SUPPRESSOR PRECURSOR (FAT PROTEIN).//6.40E-51//277aa//35%//P33450
C-HEMBA1004391//TUMOR SUPPRESSOR PROTEIN DCC PRECURSOR.//5.60E-20//194aa//26%//P70211
C-HEMBA1004444//GLYCOPROTEIN 25L PRECURSOR (GP25L).//4.60E-41//148aa//52%//P27869
C-HEMBA1004454
C-HEMBA1004505//MANNOSYL-OLIGOSACCHARIDE ALPHA-1,2-MANNOSIDASE ISOFORM 1 (EC 3.2.1.113) (MAN(9)-ALPHA-MANNOSIDASE).//2.70E-45//239aa//43%//P53624
C-HEMBA1004797
C-HEMBA1004982//TETRACYCLINE RESISTANCE PROTEIN, CLASS E (TETA(E)).//6.30E-10//149aa//26%//Q07282
C-HEMBA1005070//SKIN SECRETORY PROTEIN XP2 PRECURSOR (APEG PROTEIN).//1.10E-05//187aa//29%//P17437
C-HEMBA1005084//NON-RECEPTOR TYROSINE KINASE SPORE LYSIS A (EC 2.7.1.112) (TYROSINE- PROTEIN KINASE 1).//1.20E-07//102aa//37%//P18160
C-HEMBA1005145
C-HEMBA1005430
C-HEMBA1005449//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//5.40E-10//224aa//24%//P13983
C-HEMBA1005489
C-HEMBA1005522//COAGULATION FACTOR VII PRECURSOR (EC 3.4.21.21).//7.70E-15//78aa//51%//P98139
C-HEMBA1005545//MUSCARINIC ACETYLCHOLINE RECEPTOR M3.//0//2121aa//100%//U29589
C-HEMBA1005698//Homo sapiens vesicle trafficking protein (SEC22C) mRNA, complete cds.//6.60E-163//753bp//99%//AF039568
C-HEMBA1005913
C-HEMBA1005929//H.sapiens mRNA for serine/threonine protein kinase EMK.//6.50E-92//1092bp//69%//X97630
C-HEMBA1005945//Oryctolagus cuniculus peroxisomal Ca-dependent solute carrier mRNA, complete Gds.//1.90E-44//666bp//65%//AF004161
C-HEMBA1006016
C-HEMBA1006171
C-HEMBA1006299
C-HEMBA1006311
C-HEMBA1006335
C-HEMBA1006430//Human putative transmembrane protein precursor (B5) mRNA, complete cds.//2.40E-70//1108bp//65%//L38961
C-HEMBA1006482//Homo sapiens h-scol (SCOI) mRNA, nuclear gene encoding mitochondrial protein, complete cds.//0//1101bp//98%//AF026852
C-HEMBA1006572//ODD-SKIPPED PROTEIN.//2.60E-39//85aa//83%//P23803
C-HEMBA1006707//Homo sapiens mRNA for matrilin-4, partial.//0//2003bp//99%//AJ007581
C-HEMBA1006724
C-HEMBA1006902//Homo sapiens mRNA for matrilin-4, partial.//4.80E-275//1799bp//85%//AJ007581
C-HEMBA1006916//Homo sapiens Grb14 mRNA, complete cds.//3.00E-277//1010bp//95%//L76687
C-HEMBA1006960
C-HEMBA1007013
C-HEMBA1007057
C-HEMBA1007241
C-HEMBA1007291
C-HEMBA1007332
C-HEMBB1000276
C-HEMBB1000447//Homo sapiens JWA protein mRNA, complete cds.//0//2059bp//99%//AF070523
C-HEMBB1000642
C-HEMBB1000668//Homo sapiens mRNA for KIAA0893 protein, complete cds.//0//2375bp//99%//AB020700
C-HEMBB1000679//C.familiaris mRNA for TRAM-protein.//4.10E-210//1149bp//80%//X63678
C-HEMBB1000881//Danio rerio mRNA for MINDIN2, complete cds.//1.70E-67//948bp//66%//AB006085
C-HEMBB1000905//TRANSCRIPTIONAL REPRESSOR RCO-1.//1.00E-11//311aa//27%//P78706
C-HEMBB1001026//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//5.30E-11//142aa//30%//P32802
C-HEMBB1001048//Human Hpast (HPAST) mRNA, complete cds.//6.50E-39//448bp//75%//AC000159
C-HEMBB1001200
C-HEMBB1001407
C-HEMBB1001530//SLS1 PROTEIN PRECURSOR.//9.80E-10//273aa//27%//Q99158
C-HEMBB1001573
C-nnnnnnnnnnnn//Homo sapiens orphan G protein-coupled receptor (GPR34) gene, complete cds.//1.50E-251//1146bp//99%//AF118670
C-HEMBB1001847//NEUROGENIC PROTEIN BIG BRAIN.//4.70E-06//258aa//24%//P23645
C-HEMBB1001978
C-HEMBB1002162//Homo sapiens genethonin 1 mRNA, complete cds.//8.30E-67//328bp//99%//AP062354
C-HEMBB1002228
C-HEMBB1002245//PROSTAGLANDIN F2-ALPHA RECEPTOR REGULATORY PROTEIN PRECURSOR (PROSTAGLANDIN F2-ALPHA RECEPTOR ASSOCIATED PROTEIN).//0//879aa//89%//Q62786
C-HEMBB 1002427//FUCOSYLGLYCOPROTEIN ALPHA-N-ACETYLGALACTOSAMINYLTRANSFERASE (EC 2.4.1.40) (HISTO-BLOOD GROUP A TRANSFERASE) (A TRANSFERASE) / FUCOSYLGLYCOPROTEIN 3-ALPHA-GALACTOSYLTRANSFERASE (EC 2.4.1.37) (HISTO-BLOOD GROUP B TRANSFERASE) (B TRANSFERASE) (NAGAT).//1.80E-70//221aa//50%//P16442
C-HEMBB1002465//ACYL-COA DEHYDROGENASE (EC 1.3.99.-).//2.30E-53//249aa//48%//P45857
C-HEMBB1002663
C-HEMBB1002693
C-MAMMA1000046
C-MAMMA1000118
C-nnnnnnnnnnnn//Homo sapiens SNC73 protein (SNC73) mRNA, complete cds.//1.50E-312//1594bp//93%//AF067420
C-MAMMA1000449
C-MAMMA1000457//Human NADH-cytochrome b5 reductase mRNA, 3' end.//9.50E-79//829bp//71%//M16462
C-MAMMA1000591//POLYPEPTIDE N-ACETYLGALACTOSAMINYLTRANSFERASE (EC 2.4.1.41) (PROTEIN- UDP ACETYLGALACTOSAMINYLTRANSFERASE) (UDP-GALNAC:POLYPEPTIDE, N- ACETYLGALACTOSAMINYLTRANSFERASE) (GALNAC-T1).//1.20E-115//515aa//49%//Q07537
C-MAMMA1000681//PROBABLE G PROTEIN-COUPLED RECEPTOR EDG-1.//9.40E-82//311aa//52%//O08530
C-MAMMA1001043//MRC OX-45 SURFACE ANTIGEN PRECURSOR (BCM1 SURFACE ANTIGEN)(BLAST-1)(CD48).//2.90E-12//239aa//28%//P10252
C-MAMMA1001893
C-NT2RM2000241
C-NT2RM2000306//PUTATIVE GTP-BINDING PROTEIN W08E3.3.//4.50E-130//362aa//68%//P91917
C-NT2RM2000410
C-NT2RM2000423//BETA-GALACTOSIDASE PRECURSOR (EC 3.2.1.23) (LACTASE).//1.80E-38//308aa//35%//P48982
C-NT2RM2000497//CHL1 PROTEIN.//9.90E-24//296aa//29%//P22516
C-NT2RM2000514//Homo sapiens mRNA for KIAA0875 protein, partial cds.//0//2381bp//99%//AB020682
C-NT2RM2000622
C-NT2RM2001126//Homo sapiens mRNA for multi PDZ domain protein.//0//1600bp//99%//AJ0001319
C-NT2RM2001902//Homo sapiens mRNA for PAK4 protein.//5.40E-216//988bp//99%//AJ011855
C-NT2RM2001939//Human G protein-coupled receptor GPR-NGA gene, complete cds.//0//1559bp//98%//U55312
C-NT2RM2001941//MUSCARINIC ACETYLCHOLINE RECEPTOR M1.//7.40E-38//193aa//34%//P08482
C-NT2RM4000198
C-NT2RM4000284//Human IgG Fc receptor hFcRn mRNA, complete cds.//1.30E-257//603bp//96%//U12255
C-NT2RM4000295
C-NT2RM4000326//ZINC FINGER PROTEIN 84 (ZINC FINGER PROTEIN HPF2).//9.00E-100//434aa//43%//P51523
C-NT2RM4000444//ANTIGEN PEPTIDE TRANSPORTER 1 (APT1).//1.70E-112//493aa//44%//P36370
C-NT2RM4000587
C-NT2RM4000648//K-GLYPICAN PRECURSOR.//4.00E-193//531aa//66%//P51655
C-NT2RM4000997//MONO- AND DIACYLGLYCEROL LIPASE PRECURSOR (EC 3.1.1.-) (MDGL).//1.80E-10//189aa//30%//P25234
C-NT2RM4001321
C-NT2RM4001325//CHONDROITIN 6-SULFOTRANSFERASE (EC 2.8.2.17) (C6ST).//2.90E-48//343aa//34%//Q92179
C-NT2RM4001735
C-NT2RM4002352//Homo sapiens hLRp105 mRNA for LDL receptor related protein 105, complete cds.//0//2184bp//99%//AB009462
C-NT2RP1000002
C-NT2RP1000050
C-NT2RP1000181//Homo sapiens delta-6 fatty acid desaturase mRNA, complete cds.//3.30E-121//1394bp//69%//AF126799
C-NT2RP1000261//ORM1 PROTEIN.//2.40E-17//137aa//35%//P53224
C-NT2RP1000300//Human transporter protein (g17) mRNA, complete cds.//3.80E-26//758bp//62%//U49082
C-NT2RP1000325//H.sapiens gene for phosphate carner.//0//439bp//98%//X77337
C-NT2RP1000448
C-NT2RP1000551//Homo sapiens putative interferon-related protein (SM15) mRNA, partial cds.//0//1761bp//99%//U09585
C-NT2RP1000579//Human succinate dehydrogenase flavoprotein subunit (SDH) mRNA, complete cds.//0//1951bp//94%//L21936
C-NT2RP1000613//CARBONIC ANHYDRASE VI (EC 4.2.1.1) (SALIVARY) (CARBONATE DEHYDRATASE VI).//3.40E-52//304aa//40%//P08060
C-NT2RP1000903
C-NT2RP1000981//CELL SURFACE A33 ANTIGEN PRECURSOR.//3.60E-14//286aa//27%//Q99795
C-NT2RP1001004//F-SPONDIN PRECURSOR.//9.20E-43//322aa//35%//P35446
C-NT2RP1001020//SERINE/THREONINE-PROTEIN KINASE PAK-BETA (EC 2.7.1.-) (BETA-PAK) (P21-ACTIVATED KINASE 3) (CDC42/RAC EFFECTOR KINASE PAK-B).//9.70E-22//227aa//31%//Q61036
C-NT2RP1001563//TESTIS-SPECIFIC PROTEIN TPX-1 PRECURSOR (AUTOANTIGEN 1) (25 KD ACROSOMAL AUTOANTIGEN) (AA1).//9.70E-19//201aa//31%//Q60477
C-NT2RP2000394//Gallus gallus p52 pro-apototic protein mRNA, complete cds.//1.60E-90//956bp//70%//AF029071
C-NT2RP2000479
C-NT2RP2000514//Homo sapiens roundabout 2 (robo2) mRNA, partial cds.//3.00E-185//855bp//99%//AF040991
C-NT2RP2000533//Homo sapiens cornichon protein mRNA, complete cds.//1.30E-290//1324bp//99%//AF070654
C-NT2RP2000649//Homo sapiens mRNA for Hs Ste24p, complete cds.//0//2847bp//99%//AB016068
C-NT2RP2000663
C-NT2RP2000694//Homo sapiens 5T4 oncofetal trophoblast glycoprotein gene.//0//2278bp//99%//AJ012159
C-NT2RP2000903//Homo sapiens 5T4 oncofetal trophoblast glycoprotein gene.//0//2276bp//100%//AJ012159
C-NT2RP2001480//Homo sapiens thrombospondin 3 (THBS3) gene, complete cds.//0//2547bp//99%//L38969
C-NT2RP2001495//Human transporter protein (g17) mRNA, complete cds.//2.20E-65//641bp//65%//U49082
C-NT2RP2001514//PROBABLE CALCIUM-TRANSPORTING ATPASE 6 (EC 3.6.1.38).//1.20E-133//429aa//41%//P39986
C-NT2RP2001529//Homo sapiens mRNA for ZIP-kinase, complete cds.//0//2079bp//99%//AB007144
C-NT2RP2001769//SERINE/THREONINE-PROTEIN KINASE ORB6 (EC 2.7.1.-).//9.10E-47//185aa//44%//O13310
C-NT2RP2001878
C-NT2RP2001903//CALPAIN, LARGE [CATALYTIC] SUBUNIT (EC 3.4.22.17) (CALCIUM- ACTIVATED NEUTRAL PROTEINASE) (CANP) (MU/M-TYPE).//3.80E-58//475aa//34%//P00789
C-NT2RP2001915
C-NT2RP2001956//ORM1 PROTEIN.//3.90E-19//137aa//37%//P53224
C-NT2RP2002063//GNS1 PROTEIN.//3.60E-18//231aa//33%//P25358
C-NT2RP2002188//Rattus norvegicus neuroligin 3 mRNA, complete cds.//2.50E-226//1284bp//89%//U41663
C-NT2RP2002232//HYPOTHETICAL 85.7 KD PROTEIN C13G6.03 IN CHROMOSOME I.//1.90E-93//420aa//43%//Q09782
C-NT2RP2002304//Human mRNA for KIAA0383 gene, partial cds.//0//1640bp//99%//AB002381
C-NT2RP2002409
C-NT2RP2002510
C-NT2RP2002527//CYTOCHROME B5.//1.30E-11//92aa//38%//P40312
C-NT2RP2002533//Homo sapiens alpha 2 delta calcium channel subunit isoform I mRNA, complete cds.//0//2365bp//99%//AF042792
C-NT2RP2002564//Human zinc-finger protein C2H2-150 mRNA, complete cds.//0//2237bp//99%//U38864
C-NT2RP2002674//SOLUBLE EPOXIDE HYDROLASE (SEH) (EC 3.3.2.3) (EPOXIDE HYDRATASE) (CYTOSOLIC EPOXIDE HYDROLASE) (CEH).//5.50E-38//201aa//39%//P34913
C-NT2RP2002721//REGULATORY PROTEIN UHPC.//1.60E-23//153aa//30%//P27669
C-NT2RP2002824//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//3.50E-63//404aa//33%//P32802
C-NT2RP2002942//Homo sapiens mRNA for KIAA0806 protein, complete cds.//0//2090bp//99%//AB018349
C-NT2RP2002974//HOMEOBOX PROTEIN SIX5 (DM LOCUS-ASSOCIATED HOMEODOMAIN PROTEIN HOMOLOG) (FRAGMENT).//8.20E-241//555aa//84%//P70178
C-NT2RP2002976
C-NT2RP2003042//PHOSPHATIDYLCHOLINE-STEROL ACYLTRANSFERASE PRECURSOR (EC 2.3.1.43) (LECITHIN-CHOLESTEROL ACYLTRANSFERASE) (PHOSPHOLIPID-CHOLESTEROL ACYLTRANSFERASE) (FRAGMENT).//2.10E-109//385aa//52%//P53760
C-NT2RP2003179//PUTATIVE SERINE/THREONINE-PROTEIN KINASE EMK (EC 2.7.1.-).//2.60E-67//256aa//49%//Q05512
C-NT2RP2003210//Homo sapiens fatty acid transport protein (FATP) mRNA, complete cds.//9.80E-272//1265bp//98%//AF055899
C-NT2RP2003369//RAS SUPPRESSOR PROTEIN 1 (RSU-1) (RSP-1 PROTEIN) (RSP-1).//5.90E-20//204aa//34%//Q15404
C-NT2RP2003383//Homo sapiens mRNA for KIAA0458 protein, complete cds.//0//2565bp//99%//AB007927
C-NT2RP2003469//GALACTOSE-PROTON SYMPORT (GALACTOSE TRANSPORTER).//1.10E-45//324aa//29%//P37021
C-NT2RP2003545//Homo sapiens STE20-like kinase 3 (mst-3) mRNA, complete cds.//5.40E-48//578bp//71%//AF024636
C-NT2RP2003593
C-NT2RP2003599
C-NT2RP2003655//HYPOTHETICAL 26.3 KD PROTEIN IN OYE2-GND1 INTERGENIC//4.80E-15//93aa//47%//P38869
C-NT2RP2003931
C-NT2RP2004141
C-NT2RP2004179
C-NT2RP2004205//BUTYROPHILIN PRECURSOR (BT).//1.60E-21//276aa//32%//Q62556
C-NT2RP2004447
C-NT2RP2004495//Human transporter protein (g17) mRNA, complete cds.//9.80E-64//642bp//64%//U49082
C-NT2RP2004524
C-NT2RP2004556
C-NT2RP2004606//Human fibroblast collagenase inhibitor mRNA, complete cds.//2.10E-166//768bp//99%//M12670
C-NT2RP2004648//Mouse beta-galactosidase (BGAL) gene, complete cds.//1.20E-33//1136bp//59%//M57734
C-NT2RP2004670//Rattus norvegicus vesicla-associate calmodulin-binding protein mRNA, complete cds.//0//1250bp//86%//L22557
C-NT2RP2004794//HYPOTHETICAL 24.5 KD PROTEIN IN SAP185-BCK1 INTERGENIC REGION.//2.70E-09//203aa//26%//P40857
C-NT2RP2004837
C-NT2RP2004847//ZINC FINGER PROTEIN 135.//8.00E-35//193aa//40%//P52742
C-nnnnnnnnnnnn//Homo sapiens SCG10-like-protein (SCLIP) mRNA, complete cds.//2.90E-170//813bp//98%//AF069709
C-NT2RP2005027
C-NT2RP2005163
C-NT2RP2005181//Mus musculus cationic amino acid transporter (CAT3) mRNA, complete cds.//5.30E-315//2126bp//81%//U70859
C-NT2RP2005247//ZINC-FINGER PROTEIN RFP (RET FINGER PROTEIN).//5.00E-53//296aa//37%//Q62158
C-NT2RP2005425//Homo sapiens mRNA for AKAP450 protein.//0//4341bp//99%//AJ131693
C-NT2RP2005463//PROTEIN PTM1 PRECURSOR.//7.40E-15//284aa//28%//P32857
C-NT2RP2005514
C-NT2RP2005541//N-ACETYLGLUCOSAMINE-6-SULFATASE PRECURSOR (EC 3.1.6.14) (G6S) (GLUCOSAMINE-6-SULFATASE).//4.70E-24//78aa//51%//P15586
C-NT2RP2005632
C-NT2RP2005878//PUTATIVE STEROID DEHYDROGENASE KIK-I (EC 1.1.1.-).//3.60E-55//238aa//50%//O57314
C-NT2RP2005883//DOPAMINE-BETA-MONOOXYGENASE PRECURSOR (EC 1.14.17.1) (DOPAMINE BETA- HYDROXYLASE) (DBH).//6.70E-72//512aa//34%//P15101
C-NT2RP2005887
C-NT2RP2005941//Human paired box gene (PAX6) homologue, complete cds.//1.40E-308//1396bp//99%//M93650
C-NT2RP2005994//HYPOTHETICAL 51.5 KD PROTEIN D2024.3 IN CHROMOSOME IV.//3.50E-35//144aa//47%//P49191
C-NT2RP2006042//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//9.40E-15//501aa//25%//P08640
C-NT2RP2006269//DOLICHYL-PHOSPHATE-MANNOSE--PROTEIN MANNOSYLTRANSFERASE 2 (E,C 2.4.1.109).//2.30E-78//679aa//32%//P31382
C-NT2RP2006512//GNS 1 PROTEIN.//2.00E-21//290aa//29%//P25358
C-NT2RP3000059//ANKYRIN, BRAIN VARIANT 2 (ANKYRIN B) (ANKYRIN, NONERYTHROID) (FRAGMENT).//3.70E-12//133aa//32%//Q01485
C-NT2RP3000063//NEUROFILAMENT TRIPLET H PROTEIN (200 KD NEUROFILAMENT PROTEIN) (NF-H).//5.00E-29//596aa//30%//P19246
C-NT2RP3000125//RETINOBLASTOMA BINDING PROTEIN 2 (RBBP-2).//6.30E-08//70aa//41%//P29375
C-NT2RP3000169//Homo sapiens MRS1 mRNA, complete cds.//0//1519bp//97%//AF093239
C-NT2RP3000172//CALCIUM/CALMODULIN-DEPENDENT PROTEIN KINASE TYPE I (EC 2.7.1.123) (CAM KINASE I).//1.30E-80//359aa//44%//Q14012
C-NT2RP3000201//Homo sapiens mRNA for KIAA0687 protein, partial cds.//2.00E-305//1224bp//99%//AB014587
C-NT2RP3000436//PROBABLE PROTEIN DISULFIDE ISOMERASE P5 PRECURSOR (EC 5.3.4.1).//6.20E-27//90aa//42%//P38660
C-NT2RP3000460//Canis familiaris sec61 homologue mRNA, complete cds.//1.80E-198//643bp//89%//M96629
C-NT2RP3000616//FIBROMODULIN PRECURSOR (FM) (COLLAGEN-BINDING 59 KD PROTEIN).//5.20E-26//227aa//36%//Q06828
C-NT2RP3000721//HYPOTHETICAL 149.7 KD PROTEIN IN IRE1-KSP1 INTERGENIC REGION.//1.10E-22//171aa//36%//P38800
C-NT2RP3000820//PUTATIVE SERINE/THREONINE-PROTEIN KINASE PKWA (EC 2.7.1.-).//1.90E-30//269aa//33%//P49695
C-NT2RP3000871
C-NT2RP3000907//PROBABLE CALCIUM-TRANSPORTING ATPASE 6 (EC 3.6.1.38).//2.20E-134//296aa//42%//P39986
C-NT2RP3001012//Homo sapiens mRNA for KIAA0829 protein, partial cds.//0//2906bp//98%//AB020636
C-NT2RP3001044
C-NT2RP3001061//Homo sapiens mRNA for KIAA0853 protein, partial cds.//0//3591bp//99%//AB020660
C-NT2RP3001170//Homo sapiens mRNA; cDNA DKFZp586K2120 (from clone DKFZp586K2120).//0//2421bp//99%//AL080163
C-NT2RP3001195//GALACTOSE-PROTON SYMPORT (GALACTOSE TRANSPORTER).//4.70E-48//339aa//29%//P37021
C-NT2RP3001240//Canis familiaris sec61 homologue mRNA, complete cds.//1.20E-301//1141bp//89%//M96629
C-NT2RP3001322//PROBABLE CALCIUM-TRANSPORTING ATPASE 3 (EC 3.6.1.38) (ENDOPLASMIC RETICULUM CA2+-ATPASE).//1.70E-21//220aa//30%//P39524
C-NT2RP3001388//SYNAPTOTAGMIN IV.//2.00E-118//430aa//54%//P50232
C-nnnnnnnnnnnn//Human mRNA for KIAA0315 gene, partial cds.//0//2971bp//99%//AB002313
C-nnnnnnnnnnnn//Homo sapiens mRNA for KIAA0017 protein, complete cds.//0//3243bp//99%//D87686
C-NT2RP3001560//Homo sapiens cleft lip and palate transmembrane protein 1 (CLPTM1) mRNA, complete cds.//0//2468bp//99%//AF037339
C-NT2RP3001592//N2,N2-DIMETHYLGUANOSINE TRNA METHYLTRANSFERASE PRECURSOR (EC 2.1.1.32).//1.30E-18//279aa//27%//P15565
C-NT2RP3001738//CYTOCHROME B5.//1.30E-11//133aa//33%//P00169
C-NT2RP3001754
C-NT2RP3001858
C-NT2RP3002160//Canis familiaris forssman synthetase mRNA, complete cds.//5.00E-152//789bp//84%//U66140
C-NT2RP3002311//BETA-GALACTOSIDASE PRECURSOR (EC 3.2.1.23) (LACTASE).//9.80E-103//547aa//43%//P48982
C-NT2RP3002342//Human transporter protein (g17) mRNA, complete cds.//1.70E-65//641bp//65%//U49082
C-NT2RP3002448
C-NT2RP3002721//Porcine citrate synthase mRNA, complete cds.//9.10E-281//1454bp//93%//M21197
C-NT2RP3002738//Homo sapiens enhancer of filamentation (HEF1) mRNA, complete cds.//2.20E-47//763bp//65%//L43821
C-NT2RP3002790
C-NT2RP3002836//Homo sapiens mRNA for KIAA0463 protein, partial cds.//0//1617bp//99%//AB007932
C-NT2RP3002958//TRANS-GOLGI NETWORK INTEGRAL MEMBRANE PROTEIN TGN38 PRECURSOR.//8.00E-08//197aa//26%//P19814
C-NT2RP3003000//Homo sapiens T-type calcium channel alpha-1 subunit mRNA, complete cds.//0//3160bp//96%//AF051946
C-NT2RP3003076
C-NT2RP3003354//SECRETORY CARRIER-ASSOCIATED MEMBRANE PROTEIN 3.//5.10E-55//208aa//51%//O35609
C-NT2RP3003469
C-NT2RP3003527//Homo sapiens mRNA for protein kinase Dyrk1B.//0//2483bp//99%//Y17999
C-NT2RP3003535//UDP-N-ACETYLGLUCOSAMINE--PEPTIDE N-ACETYLGLUCOSAMINYLTRANSFERASE 110 KD SUBUNIT (EC 2.4.1.-) (O-GLCNAC TRANSFERASE P110 SUBUNIT).//8.80E-18//368aa//25%//P56558
C-NT2RP3003559
C-NT2RP3003614
C-NT2RP3003729
C-NT2RP3003849//PROTEIN KINASE C, BRAIN ISOZYME (EC 2.7.1.-) (PKC) (DPKC53E(BR)). PKC1.//1.20E-13//126aa//34%//P05130
C-NT2RP3003874//Homo sapiens incomplete cDNA for a mutated allele of a myosin class I, myh-1c://0//2160bp//98%//AJ001381
C-NT2RP3003963
C-NT2RP3004000
C-NT2RP3004075
C-NT2RP3004083
C-NT2RP3004090//GOLIATH PROTEIN (G1 PROTEIN).//9.00E-33//179aa//47%//Q06003
C-NT2RP3004130//CELL SURFACE ANTIGEN 114/A10 PRECURSOR.//8.10E-06//71aa//42%//P19467
C-NT2RP3004133//GLYCOSYLTRANSFERASE ALG2 (EC 2.4.1.-).//2.50E-48//198aa//37%//P43636
C-NT2RP3004202
C-NT2RP3004309//Homo sapiens mRNA; cDNA DKFZp586C201 (from clone DKFZp586C201).//0//2584bp//99%//AL050118
C-NT2RP3004321
C-NT2RP3004355
C-NT2RP3004374
C-NT2RP3004406//HYPOTHETICAL 37.4 KD PROTEIN IN IRR1-TIM44 INTERGENIC REGION.//3.20E-15//165aa//33%//P40544
C-NT2RP3004552//COMPLEMENT RECEPTOR TYPE 1 PRECURSOR (C3B/C4B RECEPTOR) (CD35 ANTIGEN).//8.50E-24//263aa//33%//P17927
C-NT2RP3004557//Human Ki nuclear autoantigen mRNA, complete cds.//0//2181bp//96%//U11292
C-NT2RP3004625//Homo sapiens mRNA for KIAA0975 protein, partial cds.//0//1339bp//99%//AB023192
C-NT2RP3004640//Bos taurus tuftelin mRNA, complete cds.//0//1204bp//88%//AF105228
C-NT2RP3004647//PUTATIVE MITOCHONDRIAL CARRIER YMR166C.//2.00E-15//220aa//27%//Q03829
C-NT2RP4000108//Human gene for neurofilament subunit NF-L.//0//1998bp//99%//AF176680
C-NT2RP4000962//SPORULATION-SPECIFIC PROTEIN 1 (EC 2.7.1.-).//2.60E-18//225aa//32%//P08458
C-NT2RP4001009//Homo sapiens mRNA for farnesylated-proteins converting enzyme 1.//0//2965bp//99%//Y13834
C-NT2RP4001467//Human placental cDNA coding for 5'nucleotidase (EC 3.1.3.5).//0//2140bp//99%//X55740
C-OVARC1000090
C-OVARC1000105//UBIQUITIN-CONJUGATING ENZYME E2-28.4 KD (EC 6.3.2.19) (UBIQUITIN- PROTEIN LIGASE) (UBIQUITIN CARRIER PROTEIN).//4.20E-47//171aa//56%//P33296
C-OVARC1000137
C-OVARC1000208
C-OVARC1000255//H.sapiens syk mRNA for protein-tyrosine kinase.//0//1525bp//97%//Z29630
C-OVARC1000275//DESMOPLAKIN I AND II (DPI AND DPII) (FRAGMENT).//9.90E-16//352aa//23%//P15924
C-OVARC1000298
C-OVARC1000410//Homo sapiens angiopoietin Y1 mRNA, complete cds.//2.10E-63//744bp//69%//AF107253
C-OVARC1000439//HYPOTHETICAL 64.3 KD GTP-BINDING PROTEIN C02F5.3 IN CHROMOSOME III.//1.40E-33//143aa//53%//P34280
C-OVARC1000467
C-OVARC1000529//PROBABLE SERINE/THREONINE-PROTEIN KINASE YKL101W (EC 2.7.1.-).//1.40E-23//165aa//39%//P34244
C-OVARC1000775
C-nnnnnnnnnnnn//ZINC FINGER PROTEIN 157.//1.00E-35//130aa//46%//P51786
C-OVARC1000811//PLASMINOGEN (EC 3.4.21.7) (FRAGMENT).//6.40E-13//115aa//34%//Q01177
C-OVARC1000853
C-OVARC1000916//H.sapiens PISSLRE mRNA.//7.30E-280//1117bp//95%//X78342
C-OVARC1000956//SKIN SECRETORY PROTEIN XP2 PRECURSOR (APEG PROTEIN).//2.20E-09//250aa//28%//P17437
C-OVARC1001030//Homo Sapiens mRNA for KIAA0886 protein, complete cds.//0//907bp//99%//AB020693
C-OVARC1001049//TRANSCRIPTION FACTOR HES- (C-HAIRY1).//7.50E-14//96aa//36%//O57337
C-OVARC1001086//Homo Sapiens cyclin T2a mRNA, complete cds.//0//1593bp//98%//AF048731
C-OVARC10011321/GC-RICH SEQUENCE DNA-BINDING FACTOR (GCF) (TRANSCRIPTION FACTOR 9)(TCF-9).//2.30E-44//268aa//36%//P16383
C-OVARC1001163//HYPOTHETICAL 49.3 KD PROTEIN C30D11.06C IN CHROMOSOME I.//2.30E-20//152aa//30%//Q09906
C-OVARC1001222
C-OVARC1001338//AUTOPHAGY SERINE/THREONINE-PROTEIN KINASE APG1 (EC 2.7.1.-).//8.80E-30//125aa//40%//P53104
C-OVARC1001569//PROBABLE SERINE/THREONINE-PROTEIN KINASE YKL101W (EC 2.7.1.-).//1.50E-22//164aa//39%//P34244
C-OVARC1001596//Homo sapiens Arf-like 2 binding protein BART1 mRNA, complete cds.//0//1766bp//99%//AF126062
C-OVARC1001725
C-OVARC1001727
C-OVARC1001807//Human TR3 orphan receptor mRNA, complete cds.//1.10E-243//1145bp//98%//L13740
C-OVARC1001991//VOLTAGE-GATED POTASSIUM CHANNEL PROTEIN KV3.3 (KSHIIID).//8.30E-06//114aa/35%//Q01956
C-OVARC1002058//Human 18S rRNA gene, complete.//1.50E-164//921bp//91%//M10098
C-OVARC1002178
C-PLACE1000033//VON WILLEBRAND FACTOR PRECURSOR.//3.80E-17//190aa//28%//Q28295
C-PLACE1000231//Rattus norvegicus WAP four-disulfide core domain protein (ps20) mRNA, complete cds.//2.70E-101//947bp//74%//AF037272
C-PLACE1000258//ZINC FINGER PROTEIN 91 (ZINC FINGER PROTEIN HTF10) (HPF7).//1.70E-55//431aa//35%//Q05481
C-PLACE1000442//ZINC FINGER PROTEIN ZFP-36 (FRAGMENT).//1.00E-88//213aa//67%//P16415
C-PLACE1000560
C-PLACE1000740//Mus musculus (Notch2) mRNA, complete cds.//5.60E-122//893bp//81%//M93661
C-PLACE1000912
C-PLACE1000914//Homo sapiens PB39 mRNA, complete cds.//7.50E-88//500bp//69%//AF045584
C-PLACE1000927//HYPOTHETICAL 20.9 KD PROTEIN B0563.3 IN CHROMOSOME X.//6.30E-21//123aa//37%//Q11079
C-PLACE1001016//SODIUM CHANNEL PROTEIN PARA (PARALYTIC
PROTEIN).//6.80E-12//133aa//28%//P35500
C-nnnnnnnnnnnn//Homo sapiens T245 protein (T245) mRNA, complete cds.//0//1801bp//99%//AF043906
C-PLACE1001100
C-PLACE1001114//HYPOTHETICAL BHLF1 PROTEIN.//9.20E-06//389aa//31%//P03181
C-PLACE1001123
C-PLACE1001183
C-PLACE1001229
C-PLACE1001231//Rattus norvegicus sodium-dependent multi-vitamin transporter (SMVT) mRNA, complete cds.//2.20E-137//918bp//80%//AF026554
C-nnnnnnnnnnnn//Human pregnancy-specific beta 1-glycoprotein 4 (PSG4) clone FL-64 mRNA, complete cds.//7.60E-293//1631bp//90%//U18469
C-PLACE1001340//Homo sapiens mRNA for KIAA0719 protein, complete cds.//0//2868bp//99%//AB018262
C-PLACE1001401//HIGH AFFINITY IMMUNOGLOBULIN EPSILON RECEPTOR BETA-SUBUNIT (FCERI) (IGE FC RECEPTOR, BETA-SUBUNIT).//3.70E-18//148aa//39%//P13386
C-PLACE1001407
C-PLACE1001464//Human placental cDNA coding for 5'nucleotidase (EC 3.1.3.5).//0//2756bp//99%//X55740
C-PLACE1001500//Homo sapiens RecQ5 mRNA for DNA helicase, complete cds.//2.30E-271//1230bp//99%//AB006533
C-PLACE1001516//240 KD PROTEIN OF ROD PHOTORECEPTOR CNG-CHANNEL [CONTAINS: GLUTAMIC ACID-RICH PROTEIN (GARP); CYCLIC-NUCLEOTIDE-GATED CATION CHANNEL 4 (CNG CHANNEL 4) (CNG-4) (CYCLIC NUCLEOTIDE-GATED CATION CHANNEL MODULATORY SUBUNIT)].//2.30E-08//274aa//28%//Q28181
C-PLACE1001536
C-PLACE1001564//H.sapiens mRNA for HE6 Tm7 receptor.//5.10E-36//499bp//70%//X81892
C-PLACE1001655//Homo sapiens Shab-related delayed-rectifier K+ channel alpha subunit (KCNS3) mRNA, complete cds.//0//1708bp//99%//AF043472
C-nnnnnnnnnnnn//Homo sapiens calumein (Calu) mRNA, complete cds.//0//1776bp//99%//AF013759
C-PLACE1001788
C-PLACE1001795//HYPOTHETICAL 30.6 KD PROTEIN IN SCP160-SMC3 INTERGENIC REGION PRECURSOR.//3.40E-20//159aa//40%//P47032
C-PLACE1001836//ENV POLYPROTEIN PRECURSOR (COAT POLYPROTEIN) [CONTAINS: OUTER MEMBRANE PROTEIN GP70; TRANSMEMBRANE PROTEIN P2OE].//5.00E-27//134aa//47%//P10269
C-PLACE1001918//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//2.30E-53//339aa//33%//P32802
C-PLACE1001949//PROBABLE CALCIUM-TRANSPORTING ATPASE 9 (EC 3.6.1.38).//3.00E-75//315aa//44%//Q12697
C-PLACE1002080//Homo sapiens antigen NY-CO-9 (NY-CO-9) mRNA, partial cds.//0//1588bp//99%//AF039691
C-PLACE1002095
C-PLACE1002153//Homo sapiens TACC2 protein (TACC2) mRNA, partial cds.//0//1202bp//99%//AF095791
C-PLACE1002329//EPIDERMAL GROWTH FACTOR RECEPTOR KINASE SUBSTRATE EPS8.//6.50E-105//213aa//45%//Q08509
C-PLACE1002355//COMPLEMENT C4 PRECURSOR [CONTAINS: C4A ANAPHYLATOXIN].//4.20E-12//131aa//40%//P01029
C-PLACE1002374//Human mRNA for pro-cathepsin L (major excreted protein MEP).//1.30E-313//1363bp//97%//X12451
C-PLACE1002518
C-PLACE1002547//Homo sapiens mRNA for KIAA0719 protein, complete cds.//0//2985bp//99%//AB018262
C-PLACE1002726//Oryctolagus cuniculus mRNA for epithelial calcium channel (ECaC).//2.80E-202//926bp//82%//AJ133128
C-PLACE1002905//ENDOZEPINE-RELATED PROTEIN PRECURSOR (MEMBRANE-ASSOCIATED DIAZEPAM BINDING INHIBITOR) (MA-DBI).//2.40E-37//188aa//40%//P07106
C-PLACE1002911//POLIOVIRUS RECEPTOR HOMOLOG PRECURSOR.//4.50E-39//345aa//32%//P32507
C-PLACE1002967//HIGH AFFINITY IMMUNOGLOBULIN EPSILON RECEPTOR BETA-SUBUNIT (FCERI) (IGE FC RECEPTOR, BETA-SUBUNIT).//4.60E-08//156aa//30%//Q01362
C-PLACE1003135//SPORULATION-SPECIFIC PROTEIN 1 (EC 2.7.1.-).//1.30E-47//210aa//49%//P08458
C-PLACE1003163//Homo sapiens DBI-related protein mRNA, complete cds.//1.00E-294//1344bp//99%//AF069301
C-PLACE1003428//Homo sapiens mRNA for VNN1 protein.//1.80E-142//676bp//72%//AJ132099
C-PLACE1003438
C-PLACE1003460
C-PLACE1003529//DNA-DIRECTED RNA POLYMERASE II LARGEST SUBUNIT (EC 2.7.7.6) (RPB1) (FRAGMENT).//1.30E-09//281aa//22%//P11414
C-PLACE1003573//T-CELL SURFACE GLYCOPROTEIN YE1/48 (T LYMPHOCYTE ANTIGEN A1) (LY49-A ANTIGEN).//3.70E-16//226aa//26%//P20937
C-PLACE1003598//TRP-ASP REPEATS CONTAINING PROTEIN RBA-1.//1.80E-07//161aa//27%//P90917
C-PLACE1003644
C-PLACE1003737//TOLL PROTEIN PRECURSOR.//5.40E-07//203aa//27%//P08953
C-PLACE1003772//EXTENSIN PRECURSOR (CELL WALL HYDROXYPROLINE-RICH GLYCOPROTEIN).//2.40E-12//124aa//38%//P 13983
C-PLACE1003839//Homo sapiens putative G protein-coupled receptor (RAIG1) mRNA, complete cds.//8.10E-18//771bp//58%//AF095448
C-PLACE1003845//PUTATIVE UDP-GLUCOSE 4-EPIMERASE (EC 5.1.3.2) (GALACTOWALDENASE) (UDP- GALACTOSE 4-EPIMERASE).//3.40E-37//302aa//30%//Q57664
C-PLACE1003852//Homo sapiens mRNA for KIAA0758 protein, partial cds.//0//1667bp//99%//AB018301
C-PLACE1004028
C-PLACE1004166//CREB-BINDING PROTEIN.//1.80E-12//147aa//35%//P45481
C-PLACE1004168//Homo sapiens mRNA for KIAA1007 protein, partial cds.//0//2637bp//99%//AB023224
C-PLACE1004199
C-PLACE1004279//HYPOTHETICAL 59.1 KD PROTEIN ZK637.1 IN CHROMOSOME III.//1.40E-08//166aa//30%//P30638
C-PLACE1004282//MONO- AND DIACYLGLYCEROL LIPASE PRECURSOR (EC 3.1.1.-) (MDGL).//2.10E-11//189aa//30%//P25234
C-PLACE1004305//RAS-RELATED PROTEIN RAC1.//9.60E-29//197aa//41%//P40792
C-PLACE1004441//Human G protein-coupled receptor (GPR1) gene, complete cds.//0//1880bp//98%//AC007383
C-PLACE1004450//AMINOPEPTIDASE N (EC 3.4.11.2) (MICROSOMAL AMINOPEPTIDASE) (LEUKEMIA ANTIGEN CD13).//1.30E-91//562aa//35%//P15541
C-PLACE 1004482//Rattus norvegicus hematopoietic lineage switch 2 related protein (Hls2-rp) mRNA, complete cds.//1.90E-246//1643bp//83%//AF097723
C-PLACE1004519
C-PLACE1004520//Human pregnancy-specific beta-glycoprotein d mRNA, complete cds.//9.10E-279//882bp//88%//M20881
C-PLACE1004630
C-PLACE1004637
C-PLACE1004648//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//1.40E-18//395aa//25%//P08640
C-nnnnnnnnnnnn//Homo sapiens tyrosylprotein sulfotransferase-2 mRNA, complete cds.//0//1825bp//99%//AF049891
C-PLACE1004816//Homo sapiens mRNA for Hakata antigen, complete cds.//1.00E-166//856bp//94%//D88587
C-PLACE1004887//GOLIATH PROTEIN (G1 PROTEIN).//4.80E-33//179aa//47%//Q06003
C-PLACE1005003//PROSTASIN PRECURSOR (EC 3.4.21.-).//2.20E-52//269aa//41%//Q16651
C-PLACE1005005//Homo sapiens ubiquitin conjugating enzyme G2 (UBE2G2) mRNA, complete cds.//4.10E-261//1209bp//98%//AF032456
C-PLACE1005031//CHLORINE CHANNEL PROTEIN P64.//8.00E-92//205aa//87%//P35526
C-PLACE1005239//Homo sapiens mRNA for HIRIP3 protein (clone pH4-31, pH4-17).//1.80E-235//1010bp//84%//AJ223351
C-PLACE1005250//Homo sapiens D-type cyclin-interacting protein 1 (DIP1) mRNA, complete cds.//0//1046bp//96%//AF082569
C-PLACE1005383//Homo sapiens UP50 mRNA, complete cds.//0//2019bp//99%//AF093118
C-PLACE1005410//Canis familiaris sec61 homologue mRNA, complete cds.//2.40E-204//673bp//89%//M96629
C-PLACE1005426//Human pregnancy-specific beta 1-glycoprotein 4 (PSG4) clone FL-64 mRNA, complete cds.//0//1629bp//95%//U18469
C-PLACE1005519//Homo sapiens STE20-like kinase 3 (mst-3) mRNA, complete cds.//4.60E-108//1070bp//73%//AF024636
C-PLACE1005544//Mus musculus junctional adhesion molecule (Jam) mRNA, complete cds.//2.00E-159//1237bp//76%//U89915
C-PLACE1005660//HYPOTHETICAL 64.3 KD GTP-BINDING PROTEIN C02F5.3 IN CHROMOSOME III.//1.90E-33//143aa//53%//P34280
C-PLACE1005669
C-PLACE1005682//B-CELL LYMPHOMA 3-ENCODED PROTEIN (BCL-3 PROTEIN).//4.90E-09//183aa//33%//P20749
C-PLACE1005725//HYPOTHETICAL 55.1 KD PROTEIN B0416.5 IN CHROMOSOME X.//7.60E-17//295aa//27%//Q11073
C-PLACE1005736//Human mRNA for BAS-GRIP protein.//0//2378bp//99%//E16311
C-PLACE1005768
C-PLACE1005878//Bovine chlorine channel protein (p64) mRNA, complete cds.//5.90E-137//889bp//85%//L16547
C-PLACE1006093
C-PLACE1006208//Homo sapiens nGAP mRNA, complete cds.//3.30E-151//694bp//100%//AP047711
C-PLACE1006219//PUTATIVE UDP-GLUCOSE 4-EPIMERASE (EC 5.1.3.2) (GALACTOWALDENASE) (UDP- GALACTOSE 4-EPIMERASE).//3.50E-37//302aa//30%//Q57664
C-PLACE1006277//Homo sapiens mRNA for Z39Ig protein.//0//1768bp//99%//AJ132502
C-PLACE1006290//GLYCOSYLTRANSFERASE ALG2 (EC 2.4.1.-).//8.50E-75//301aa//39%//P43636
C-PLACE1006443//Homo sapiens PB39 mRNA, complete cds.//4.30E-98//553bp//70%//AF045584
C-PLACE1006515//Homo sapiens mRNA for KIAA0576 protein, partial cds.//0//2846bp//99%//AB011148
C-PLACE1006716//30 KD ADIPOCYTE COMPLEMENT-RELATED PROTEIN PRECURSOR (ACRP30) (ADIPOCYTE SPECIFIC PROTEIN ADIPOQ).//4.60E-25//181aa//35%//Q60994
C-PLACE1006809//SLS1 PROTEIN PRECURSOR.//9.10E-10//273aa//27%//P08124
C-PLACE1006959
C-PLACE1007028
C-PLACE1007040
C-PLACE1007096//PUTATIVE SUGAR TRANSPORT PROTEIN GETA.//2.70E-17//174aa//27%//O34368
C-nnnnnnnnnnnn//Homo sapiens mRNA for putative glucosyltransferase, partial cds.//0//1373bp//99%//AJ224875
C-PLACE1007296//Human mRNA for a presumptive KDEL receptor.//1.10E-185//1038bp//91%//X55885
C-PLACE1007591
C-PLACE1007626//Homo sapiens unknown mRNA, complete cds.//3.00E-246//1122bp//99%//AF047439
C-PLACE1007702//Mus musculus TRA1 mRNA, complete cds.//7.50E-41//662bp//64%//D78335
C-PLACE1007845//GLYCOSYLTRANSFERASE ALG2 (EC 2.4.1.-).//4.80E-14//158aa//40%//P43636
C-PLACE1007881//HYPOTHETICAL 69.4 KD PROTEIN F13H10.3 IN CHROMOSOME IV.//3.10E-99//504aa//42%//Q19425
C-PLACE1008297//MYOSIN HEAVY CHAIN KINASE B (EC 2.7.1.129) (MHCK B).//1.30E-14//187aa//33%//P90648
C-nnnnnnnnnnnn//Homo sapiens mRNA for putative glucosyltransferase, partial cds.//0//1616bp//99%//AJ224875
C-PLACE1008469
C-PLACE1008549//Homo sapiens Ets transcription factor ESE-2b mRNA, complete cds.//0//2274bp//99%//AF115403
C-PLACE1008657//Bovine mRNA for adseverin, complete cds.//7.80E-227//1246bp//90%//D26549
C-PLACE1008716//Human beta-1,2-N-acetylglucosaminyltransferase II (MGAT2) gene, complete cds.//0//1888bp//99%//U15128
C-PLACE1008984
C-PLACE1008985//Mus musculus synaptotagmin VIII mRNA, partial cds.//3.80E-140//650bp//81%//U20107
C-PLACE1009067
C-PLACE1009196
C-PLACE1009279//cDNA encoding novel physiologically active protein which have serine protease activity.//6.60E-86//1414bp//64%//E12965
C-PLACE1009527//Oryctolagus cuniculus mRNA for epithelial calcium channel (ECaC).//1.20E-87//585bp//83%//AJ133128
C-PLACE1009546
C-PLACE1009600//Mouse mRNA for tetracycline transporter-like protein, complete cds.//1.10E-264//924bp//88%//D88315
C-PLACE1009735
C-PLACE1009982//SALIVARY GLUE PROTEIN SGS-3 PRECURSOR.//5.20E-08//166aa//28%//P02840
C-PLACE1010078//ORM1 PROTEIN.//3.70E-19//137aa//37%//P53224
C-PLACE1010081//Homo sapiens serine/threonine kinase RICK (RICK) mRNA, complete cds.//0//2033bp//99%//AF027706
C-PLACE1010251//FIBRILLIN 2 PRECURSOR.//1.70E-31//201aa//35%//Q61555
C-PLACE1010784//Homo sapiens orphan G protein-coupled receptor (GPR34) gene, complete cds.//2.30E-252//1146bp//99%//AF008670
C-PLACE1010827//COP-COATED VESICLE MEMBRANE PROTEIN P24 PRECURSOR (FRAGMENT).//1.90E-19//163aa//34%//P49020
C-PLACE1010968//PROTEIN-TYROSINE PHOSPHATASE DLAR PRECURSOR (EC 3.1.3.48) (PROTEIN- TYROSINE-PHOSPHATE PHOSPHOHYDROLASE).//3.40E-30//690aa//26%//P16621
C-PLACE1011045//Homo sapiens E1-like protein mRNA, complete cds.//0//2376bp//99%//AF094516
C-PLACE1011116//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//3.30E-09//234aa//27%//P08640
C-PLACE1011236//Mus musculus mRNA for RST, complete cds.//1.70E-90//1398bp//65%//AB005451
C-PLACE1011407//ZINC FINGER PROTEIN 184 (FRAGMENT).//1.80E-133//342aa//59%//Q99676
C-PLACE1011516//HYPOTHETICAL 21.5 KD PROTEIN IN SEC15-SAP4 INTERGENIC REGION.//1.30E-13//139aa//34%//P53073
C-PLACE1011708//Homo sapiens intrinsic factor-B12 receptor precursor, mRNA, complete cds.//0//1840bp//98%//AF034611
C-PLACE1011824//Human Ste20-like kinase (MST2) mRNA, complete cds.//6.40E-202//561bp//92%//U26424
C-PLACE1011978//ZINC FINGER PROTEIN 91 (ZINC FINGER PROTEIN HTF10) (HPF7).//1.10E-194//547aa//57%//Q05481
C-PLACE2000118//WISKOTT-ALDRICH SYNDROME PROTEIN HOMOLOG (WASP).//3.20E-27//205aa//43%//P70315
C-PLACE2000219
C-SKNMC1000004
C-THYRO1000036//Homo sapiens collagen alpha 3 type IX (COL9A3) mRNA, complete cds.//1.20E-258//1376bp//93%//L41162
C-THYRO1000061
C-THYRO1000099
C-THYRO1000196//Homo sapiens Ksp-cadherin (CDH16) mRNA, complete cds.//0//1632bp//91%//AF016272
C-THYRO1000400//Homo sapiens mRNA full length insert cDNA clone EUROIMAGE 25795.//0//1893bp//99%//AL109665
C-THYRO1000580//ZINC FINGER PROTEIN 184 (FRAGMENT).//9.90E-114//279aa//59%//Q99676
C-THYRO1000584//Homo sapiens mRNA for KIAA0935 protein, partial cds.//0//1338bp//99%//AB023152
C-THYRO1000678//Homo sapiens Cx30 gene.//0//1741bp//97%//AJ005585
C-THYRO1000795//C.elegans mRNA for Oxoglutarate/malate carrier protein.//8.80E-42//821bp//63%//X76114
C-THYRO1000846
C-THYRO1000866//SHK1 KINASE-BINDING PROTEIN 1.//4.40E-91//449aa//44%//P78963
C-THYRO1000956//Human G protein-coupled receptor APJ gene, complete cds.//0//1583bp//99%//U03642
C-THYRO1000999
C-THYRO1001063
C-THYRO1001071//GAMMA-ADAPTIN (GOLGI ADAPTOR HA1/AP1 ADAPTIN GAMMA SUBUNIT) (CLATHRIN ASSEMBLY PROTEIN COMPLEX 1 GAMMA LARGE CHAIN).//8.20E-14//157aa//33%//P22892
C-THYRO1001102
C-THYRO1001113//Homo sapiens mRNA; cDNA DKFZp564E1616 (from clone DKFZp564E1616).//0//1361bp//99%//AL096713
C-THYRO1001 128
C-THYRO1001205
C-THYRO1001237//PHYTOENE DEHYDROGENASE (EC 1.3.-.-) (PHYTOENE DESATURASE) (ALBINO-1 PROTEIN).//3.10E-13//346aa//22%//P21334
C-THYRO1001266//Human sodium iodide symporter mRNA, complete cds.//7.20E-81//1466bp//62%//U66088
C-THYRO1001327
C-THYRO1001456//HYPOTHETICAL 56.2 KD PROTEIN CY31.25C.//9.40E-32//355aa//31%//Q10555
C-THYRO1001457//H.sapiens mRNA for protein kinase C mu.//2.30E-218//1183bp//73%//X75756
C-THYRO1001471
C-THYRO1001478//CYTOCHROME B-245 HEAVY CHAIN (P22 PHAGOCYTE B-CYTOCHROME) (NEUTROPHIL CYTOCHROME B, 91 KD POLYPEPTIDE) (CGD91-PHOX) (GP91-PHOX) (CYTOCHROME B(558) BETA CHAIN) (SUPEROXIDE-GENERATING NADPH OXIDASE HEAVY CHAIN SUBUNIT).//8.90E-50//296aa//35%//P04839
C-THYRO1001495
C-THYRO1001523//Homo sapiens mRNA for TM7XN1 protein.//0//3663bp//99%//AJ011001
C-THYRO1001529//SERINE PALMITOYLTRANSFERASE 2 (EC 2.3.1.50) (LONG CHAIN BASE BIOSYNTHESIS PROTEIN 2) (SPT 2).//5.50E-25//115aa//53%//Q09925
C-THYRO1001700//SERINE/THREONINE PROTEIN KINASE RIP (EC 2.7.1.-) (CELL DEATH PROTEIN RIP) (RECEPTOR INTERACTING PROTEIN).//9.70E-33//268aa//37%//Q60855
C-THYRO1001702//Mus musculus mRNA for myeloid associated differentiation protein.//1.50E-128//1204bp//73%//AJ001616
C-THYRO1001725
C-THYRO1001803
C-Y79AA1000127
C-Y79AA1000207
C-Y79AA1000226
C-Y79AA1000270//Bos taurus vacuolar H+ ATPase subunit Ac45 mRNA, complete cds.//1.00E-271//1490bp//83%//U10039
C-Y79AA1000426//Mus musculus activin beta E subunit mRNA, complete cds.//7.70E-200//1533bp//78%//U96386
C-Y79AA1000521
C-Y79AA1000776
C-Y79AA1000777//VEGETATIBLE INCOMPATIBILITY PROTEIN HET-E-1.//8.10E-48//283aa//38%//Q00808
C-nnnnnnnnnnnn//Homo sapiens intersectin long form mRNA, complete cds.//0//1519bp//99%//AF064244
C-Y79AA1000876//PROTEIN DISULFIDE ISOMERASE-RELATED PROTEIN PRECURSOR (ERP72).//1.60E-44//210aa//38%//P13667
C-Y79AA1000959//Homo sapiens Hsp70 binding protein HspBP1 mRNA, complete cds.//4.80E-283//1405bp//95%//AF093420
C-Y79AA1000967//CALCIUM/CALMODULIN-DEPENDENT PROTEIN KINASE TYPE I (EC 2.7.1.123) (CAM KINASE I).//1.00E-77//359aa//44%//Q14012
C-Y79AA1001013
C-Y79AA1001056
C-Y79AA1001062//TUMOR NECROSIS FACTOR, ALPHA-INDUCED PROTEIN 1, ENDOTHELIAL (B12 PROTEIN).//8.90E-12//132aa//38%//Q13829
C-Y79AA1001090//NUCLEAR FACTOR NF-KAPPA-B P105 SUBUNIT (DNA-BINDING FACTOR KBF1) (EBP- 1) (NF-KAPPA-B1 P84 /NF-KAPPA-B1 P98) [CONTAINS: NUCLEAR FACTOR NF- KAPPA-B P50 SUBUNIT] (FRAGMENT).//4.50E-09//144aa//31 %//Q63369
C-Y79AA1001264//HYPOTHETICAL 39.9 KD PROTEIN T15H9.1 IN CHROMOSOME II PRECURSOR.//5.10E-106//351aa//58%//Q10005
C-Y79AA1001272
C-Y79AA1001328//Mus musculus mRNA for Dll3 protein, complete cds.//1.90E-263//1988bp//79%//ABO13440
C-Y79AA1001430//Homo sapiens mRNA for KIAA0469 protein, complete cds.//0//2943bp//99%//AB007938
C-Y79AA1002022//POLIOVIRUS RECEPTOR HOMOLOG PECURSOR.//2.20E-06//140aa//26%//P32507
C-BNGH41000020//NADH-UBIQUINONE OXIDOREDUCTASE CHAIN 2 (EC 1.6.5.3).//9.80E-159//347aa//90%//P03891
C-BNGH41000091//POTASSIUM CHANNEL PROTEIN EAG.//1.20E-249//625aa//65%//Q02280
C-HEMBA1000462//Human potential transcriptional repressor NOT4Hp (NOT4H) mRNA, complete cds.//0//935bp//99%//U71267
C-HEMBA1000477//HYPOTHETICAL 64.8 KD PROTEIN IN GDI1-COX15 INTERGENIC REGION.//1.40E-38//344aa//34%//P40085
C-HEMBA1000671//ZINC FINGER PROTEIN 184 (FRAGMENT).//3.90E-104//388aa//46%//Q99676
C-HEMBA1000732//Homo sapiens mRNA for latent transforming growth factor-beta binding protein-4.//0//2153bp//94%//Y13622
C-HEMBA1000835
C-HEMBA1000875
C-HEMBA1001184//SH3BGR PROTEIN (21-GLUTAMIC ACID-RICH PROTEIN) (21-GARP).//2.50E-32//100aa//60%//P55822
C-HEMBA1001272//Homo sapiens mRNA for KIAA1171 protein, partial cds.//0//1490bp//99%//AB032997
C-HEMBA1001296
C-HEMBA1002048//ODD-SKIPPED PROTEIN.//1.60E-55//122aa//75%//P23803
C-HEMBA1002985
C-HEMBA1003120//ZINC FINGER PROTEIN 184 (FRAGMENT).//1.00E-193//547aa//54%//Q99676
C-HEMBA1003497//ZINC FINGER PROTEIN 151 (POLYOMAVIRUS LATE INITIATOR PROMOTER BINDING PROTEIN) (LP-1) (ZINC FINGER PROTEIN Z13).//4.00E-28//358aa//29%//Q60821
C-HEMBA1004007
C-HEMBA1004085
C-HEMBA1004785//MODIFIER 3 PROTEIN (M33).//1.40E-27//221aa//33%//P30658
C-HEMBA1004952
C-HEMBA1004971
C-HEMBA1005230//ZINC FINGER PROTEIN 140.//2.00E-17//83aa//66%//P52738
C-HEMBA1005246
C-HEMBA1005267//ANKYRIN, BRAIN VARIANT 1 (ANKYRIN B) (ANKYRIN, NONERYTHROID).//8.40E-14//187aa//33%//Q01484
C-HEMBA1006276//ZINC FINGER PROTEIN 90 (ZFP-90) (ZINC FINGER PROTEIN NK10).//1.70E-06//56aa//57%//Q61967
C-HEMBA1006357//SECRETORY CARRIER-ASSOCIATED MEMBRANE PROTEIN 2.//3.70E-39//136aa//52%//O15127
C-HEMBA1006517
C-HEMBA1006544
C-HEMBA1006749//Homo sapiens mRNA for matrilin-4, partial.//1.40E-275//1942bp//83%//AJ007581
C-HEMBA1006770//FLOWERING TIME CONTROL PROTEIN FCA.//1.20E-33//352aa//34%//O04425
C-HEMBA1006912
C-HEMBA1007063
C-HEMBB1000106//CELLULAR NUCLEIC ACID BINDING PROTEIN (CNBP).//1.60E-10//139aa//30%//P53996
C-HEMBB1000407
C-HEMBB1000542//Mus musculus bromodomain-containing protein BP75 mRNA, complete cds.//2.60E-232//1452bp//85%//AF084259
C-HEMBB1001547//Homo sapiens CGI-02 protein mRNA, complete cds.//0//2311bp//99%//AF132937
C-HEMBB1001959//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//7.30E-14//97aa//38%//P25210
C-HEMBB1002039
C-HEMBB1002041//Homo sapiens transmembrane protein TENB2 (TENB2) mRNA, complete cds.//0//1746bp//99%//AF179274
C-HEMBB1002051//Homo sapiens Ets transcription factor ESE-2b mRNA, complete cds.//1.30E-95//454bp//99%//AF115403
C-HEMBB1002120//UDP-N-ACETYLGLUCOSAMINE-PEPTIDE N-ACETYLGLUCOSAMINYLTRANSFERASE 110 KD SUBUNIT (EC 2.4.1.-) (O-GLCNAC TRANSFERASE P110 SUBUNIT).//4.90E-22//337aa//27%//P56558
C-HEMBB1002302
C-HEMBB1002661//Homo sapiens cardiovascular helix-loop-helix factor 2 (CHF2) mRNA, complete cds.//0//2174bp//99%//AF179274
C-MAMMA1000106
C-MAMMA1000141
C-MAMMA1000204//Homo sapiens dysferlin mRNA, complete cds.//0//2028bp//99%//AF075575
C-MAMMA1000226
C-MAMMA1000403//Homo sapiens CDK4-binding protein p34SEI1 (SEI1) mRNA, complete cds.//1.20E-255//1165bp//99%//AF117959
C-MAMMA1000473//HYPOTHETICAL 35.6 KD PROTEIN IN SPC1-ILV3 INTERGENIC REGION.//5.10E-45//299aa//34%//P47088
C-MAMMA1000496//MIC1 PROTEIN.//3.00E-25//202aa//33%//P53258
C-MAMMA1000528
C-MAMMA1000614//Homo sapiens pseudouridine synthase 1 (PUS1) mRNA, partial cds.//2.10E-302//1370bp//99%//AF116238
C-MAMMA1000652
C-MAMMA1000706
C-MAMMA1000788//Bos taurus P14 (p14) mRNA, complete cds.//3.90E-85//502bp//89%//AF037349
C-MAMMA1000810
C-MAMMA1000814
C-MAMMA1000881//Homo sapiens protein kinase (SGK3) mRNA, complete cds.//0//1292bp//100%//AF169035
C-MAMMA1000986
C-MAMMA1000994//Homo sapiens ISLR(immunoglobulin superfamily containing leucine-rich repeat) mRNA, complete cds, alternatively spliced transcript ISLR-2.//0//2211bp//99%//AB024536
C-MAMMA1001141
C-MAMMA1001150//PROTEIN KINASE C, MU TYPE (EC 2.7.1.-) (NPKC-MU).//7.50E-304//587aa//68%//Q15139
C-MAMMA1001237//MONOCARBOXYLATE TRANSPORTER 2 (MCT 2).//7.70E-64//196aa//41%//P53988
C-MAMMA1001284
C-MAMMA1001310//HYPOTHETICAL 57.3 KD TRP-ASP REPEATS CONTAINING PROTEIN IN POM152- REC114 INTERGENIC REGION.//1.50E-67//441aa//37%//Q04225
C-MAMMA1001344
C-MAMMA1001418//HYPOTHETICAL PROTEIN HI0519.//6.90E-27//181aa//38%//P44742
C-MAMMA1001532//ZINC FINGER PROTEIN 84 (ZINC FINGER PROTEIN HPF2).//5.60E-126//319aa//56%//P51523
C-MAMMA1001615//NEUROGENIC DIFFERENTIATION FACTOR 1.//3.80E-11//90aa//42%//Q13562
C-MAMMA1001623//Homo sapiens mRNA; cDNA DKFZp434J1027 (from clone DKFZp434J1027); partial cds.//1.30E-269//1222bp//99%//AL133084
C-MAMMA1001634
C-MAMMA1001957
C-MAMMA1001978//Cimex lectularius apyrase (APY) mRNA, complete cds.//6.70E-19//988bp//56%//AF085499
C-MAMMA1002070//PLASMINOGEN (EC 3.4.21.7) (FRAGMENT).//1.10E-07//103aa//33%//Q01177
C-MAMMA1002080//RAS-RELATED PROTEIN RAB-13.//1.80E-29//208aa//37%//P51153
C-MAMMA1002087
C-MAMMA1002095//CALCIUM-TRANSPORTING ATPASE 1 (EC 3.6.1.38) (P-TYPE CALCIUM ATPASE).//3.70E-222//867aa//52%//O43108
C-MAMMA1002128//ABC1 PROTEIN HOMOLOG PRECURSOR.//2.50E-97//464aa//45%//Q92338
C-MAMMA1002142//NON-RECEPTOR TYROSINE KINASE SPORE LYSIS A (EC 2.7.1.112) (TYROSINE- PROTEIN KINASE 1).//9.80E-17//146aa//35%//P18160
C-MAMMA1002165//Homo sapiens connective tissue growth factor related protein WISP-2 (WISP2) mRNA, complete cds.//4.80E-60//382bp//89%//AF100780
C-MAMMA1002205//Homo sapiens mRNA; cDNA DKFZp586C091 (from clone DKFZp586C091).//2.00E-81//308bp//81%//AL050119
C-MAMMA1002234//SIGNAL RECOGNITION PARTICLE 68 KD PROTEIN (SRP68).//0//627aa//96%//Q00004
C-MAMMA1002586//Homo sapiens alpha 1,2-mannosidase mRNA, complete cds.//0//2228bp//99%//AF148509
C-MAMMA1002633
C-MAMMA1003126//Human Hpast (HPAST) mRNA, complete cds.//3.70E-162//1355bp//75%//AF001434
C-NT2RM1000580//GLYCOSYLTRANSFERASE ALG2 (EC 2.4.1.-).//8.50E-75//301aa//39%//P43636
C-NT2RM1000858//tricarboxylate carrier [rats, liver, mRNA Partial, 2986 nt].//2.10E-98//1035bp//70%//S70011
C-NT2RM2000565//HYPOTHETICAL 85.7 KD PROTEIN C13G6.03 IN CHROMOSOME I.//9.40E-94//394aa//43%//Q09782
C-NT2RM2000582//Homo sapiens mRNA for KIAA1053 protein, partial cds.//0//2666bp//99%//AB028976
C-NT2RM2000589//Bos taurus myosin X, complete cds.//0//4376bp//84%//U55042
C-NT2RM2000632//EXCISION REPAIR PROTEIN ERCC-6 (COCKAYNE SYNDROME PROTEIN CSB).//6.40E-62//183aa//47%//Q03468
C-NT2RM2000773//Homo sapiens KNSL4 and MAZ genes for kinesin-like DNA binding protein and Myc-associated zinc finger protein, complete cds.//3.00E-289//1092bp//99%//AB017335
C-NT2RM2001558//Homo sapiens protein kinase A binding protein AKAP110 mRNA, complete cds.//0//2398bp//99%//AF093408
C-NT2RM2001626//FLIGHTLESS-1 PROTEIN HOMOLOG.//4.30E-19//362aa//26%//P34268
C-NT2RM2001643
C-NT2RM2001738//SOF1 PROTEIN.//3.00E-110//325aa//47%//P33750
C-NT2RM2001792//Homo sapiens angiopoietin-related protein-2 mRNA,complete cds.//7.10E-149//995bp//86%//AF125175
C-NT2RM2001818//SIALIDASE (EC 3.2.1.18) (NEURAMINIDASE) (NA) (MAJOR SURFACE ANTIGEN).//4.30E-11//488aa//26%//P23253
C-NT2RM4000100//Homo sapiens mRNA for KIAA0989 protein, partial cds.//0//2678bp//99%//AB023206
C-NT2RM4000115//HYPOTHETICAL 68.8 KD PROTEIN B0464.6 IN CHROMOSOME III.//1.20E-16//204aa//30%//Q03564
C-NT2RM4000417//SYNAPTOTAGMIN II.//2.70E-23//293aa//30%//P46097
C-NT2RM4000593//HYPOTHETICAL 111.9 KD PROTEIN C22H10.03C IN CHROMOSOME I.//3.90E-27//576aa//24%//Q10297
C-NT2RM4000761//H.sapiens mitochondrial genome (consensus sequence).//0//1931bp//99%//X62996
C-NT2RM4000965//PUTATIVE IMPORTIN BETA-4 SUBUNIT (KARYOPHERIN BETA-4 SUBUNIT).//9.00E-44//520aa//29%//O60100
C-NT2RM4001377//Homo sapiens mRNA for KIAA0638 protein, partial cds.//0//1346bp//99%//AB014538
C-NT2RM4001768//Homo sapiens CGI-82 protein mRNA, complete cds.//0//1925bp//99%//AF151840
C-NT2RM4001843//BETA-GALACTOSIDASE PRECURSOR (EC 3.2.1.23) (LACTASE).//6.20E-33//263aa//38%//P48982
C-NT2RP1000239
C-NT2RP1000465//UBIQUITIN-LIKE PROTEIN SMT3.//5.10E-07//81aa//33%//P55857
C-NT2RP1000468//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//2.00E-13//97aa//38%//P25210
C-NT2RP1000679
C-NT2RP1000740//Homo sapiens mRNA; cDNA DKFZp586F1918 (from clone DKFZp586F1918).//4.60E-97//456bp//99%//AL050091
C-NT2RP1001031//VEGETATIBLE INCOMPATIBILITY PROTEIN HET-E-1.//3.40E-42//285aa//35%//Q00808
C-NT2RP2000178//MITOCHONDRIAL LON PROTEASE HOMOLOG 1 PRECURSOR (EC 3.4.21.-).//2.40E-192//778aa//48%//P93647
C-NT2RP2000240
C-NT2RP2000447//GOLGIN-95.//2.80E-33//99aa//66%//Q08379
C-NT2RP2000610//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//1.50E-13//97aa//38%//P25210
C-NT2RP2000616//EXTENSIN PRECURSOR (CELL WALL HYDROXYPROLINE-RICH GLYCOPROTEIN).//4.10E-12//323aa//30%//P13983
C-NT2RP2000712//ZINC FINGER PROTEIN 135.//3.70E-87//296aa//53%//P52742
C-NT2RP2000739//ZINC FINGER PROTEIN 83 (ZNNC FNNGER PROTENN HPF1).//7.50E-73//387aa//37%//P51522
C-NT2RP2000818//Homo sapiens xenotropic and polytropic murine leukemia virus receptor (X3) mRNA, complete cds.//0//2724bp//99%//AF089744
C-NT2RP2001200//Homo sapiens mRNA for KIAA0676 protein, partial cds.//0//1539bp//100%//AB014576
C-NT2RP2001223//MYOTUBULARIN-RELATED PROTEIN 3 (FRAGMENT).//3.30E-05//76aa//39%//Q13615
C-NT2RP2001276//NPDC-1 PROTEIN PRECURSOR.//3.00E-133//331aa//77%//Q64322
C-NT2RP2001388//TRNA-SPUCING ENDONUCLEASE SUBUNIT SEN2 (EC 3.1.27.9) (TRNA-INTRON ENDONUCLEASE).//5.90E-13//157aa//33%//P16658
C-NT2RP2001469//VEGETATIBLE INCOMPATIBILITY PROTEIN HET-E-1.//1.30E-14//242aa//24%//Q00808
C-NT2RP2001562//Homo Sapiens GLE1 (GLE1) mRNA, complete cds.//0//1899bp//98%//AF058922
C-NT2RP2001662//HOMEODOMAIN PROTEIN AKR (AVIAN KNOTTED-RELATED PROTEIN).//1.80E-49//94aa//81%//Q90655
C-NT2RP2001755//Rattus norvegicus f-spondin mRNA, complete cds.//0//2974bp//86%//M88469
C-NT2RP2001817//Homo sapiens sirtuin type 1 (SIRT1) mRNA, complete cds.//0//3092bp//99%//AF083106
C-NT2RP2001948//HST2 PROTEIN (HOMOLOGOUS TO SIR2 PROTEIN 2).//1.40E-08//191aa//27%//P53686
C-NT2RP2002015
C-NT2RP2003390//Homo sapiens mRNA for SEC63 protein.//0//2629bp//99%//AJ011779
C-NT2RP2003664//Homo sapiens mRNA for leptin receptor gene-related protein.//1.90E-237//1081bp//99%//Y12670
C-NT2RP2003940//ZINC FINGER PROTEIN 43 (ZINC PROTEIN HTF6).//7.00E-111//401aa//43%//P28160
C-NT2RP2004069//HYPOTHETICAL 26.4 KD PROTEIN EEED8.8 IN CHROMOSOME II.//3.00E-45//188aa//52%//Q09297
C-NT2RP2004108//ZINC FINGER PROTEIN ZFP-36 (FRAGMENT).//3.30E-171//474aa//62%//P16415
C-nnnnnnnnnnnn//Homo sapiens calumein (Calu) mRNA, complete cds.//0//2415bp//99%//AF013759
C-NT2RP2005069//Rat vacuolar protein sorting homolog r-vps33b mRNA, complete cds.//0//1792bp//87%//U35245
C-NT2RP2005378//GLYCINE-RICH CELL WALL STRUCTURAL PROTEIN 1.8 PRECURSOR (GRP 1.8).//6.30E-28//183aa//47%//P10496
C-NT2RP2005391//Homo sapiens partial mRNA for putative p621 protein which interacts with transcription factor Sp1.//0//1544bp//99%//AJ242978
C-NT2RP2005597//Homo sapiens protein O-mannosyl-transferase 1 (POMT1) mRNA, complete cds.//0//1821bp//97%//AF095136
C-NT2RP2005666
C-NT2RP2006004//FIBROMODULIN PRECURSOR (FM) (COLLAGEN-BINDING 59 KD PROTEIN).//3.00E-26//227aa//36%//Q06828
C-NT2RP2006092//Homo sapiens mRNA for Fe65L2, complete cds.//0//1156bp//99%//AB018247
C-NT2RP2006134
C-NT2RP3000011//VEGETATIBLE INCOMPATIBILITY PROTEIN HET-E-1.//4.00E-14//320aa//24%//Q00808
C-NT2RP3000022//Homo sapiens mRNA for KIAA0936 protein, complete cds.//0//2881bp//99%//AB023153
C-NT2RP3000171//Mus musculus partial mRNA for B-IND1 protein (B-ind1 gene).//4.40E-99//571bp//89%//Z97207
C-NT2RP3000304//Homo sapiens LDL receptor-related protein 6 (LRP6) mRNA, complete cds.//0//2895bp//99%//AF074264
C-NT2RP3000378//PAIRED AMPHIPATHIC HELIX PROTEIN.//4.20E-39//186aa//36%//P22579
C-NT2RP3000444
C-NT2RP3000645
C-NT2RP3000676//PUTATIVE MITOCHONDRIAL CARRIER YMR166C.//2.10E-15//220aa//27%//Q03829
C-NT2RP3000677//DNA BINDING PROTEIN RFX2.//3.60E-56//233aa//41%//P48378
C-NT2RP3000789//Homo sapiens IGF-II mRNA-binding protein 1 (IMP-1) mRNA, complete cds.//0//1458bp//100%//AF117106
C-NT2RP3000818
C-NT2RP3000838//TRICHOHYALIN.//9.80E-11//491aa//26%//Q07283
C-NT2RP3000921//TUMOR SUPPRESSOR PROTEIN DCC PRECURSOR (COLORECTAL CANCER SUPPRESSOR).//4.00E-21//316aa//29%//P43146
C-NT2RP3001159//Homo sapiens mRNA; cDNA DKFZp586C201 (from clone DKFZp586C201).//0//2558bp//99%//AL050118
C-NT2RP3001271//NON-GREEN PLASTID TRIOSE PHOSPHATE TRANSLOCATOR PRECURSOR (CTPT).//2.60E-09//334aa//22%//P52178
C-NT2RP3001542//TUMOR NECROSIS FACTOR, ALPHA-INDUCED PROTEIN 1, ENDOTHELIAL (B12 PROTEIN).//4.70E-11//132aa//37%//Q13829
C-NT2RP3001685//PRPE PROTEIN.//1.00E-68//382aa//41%//P77495
C-NT2RP3001976//ZINC FINGER PROTEIN 84 (ZINC FINGER PROTEIN HPF2).//8.90E-143//379aa//55%//P51523
C-NT2RP3002015//Homo sapiens CGI-71 protein mRNA, complete cds.//0//1991bp//99%//AF151829
C-NT2RP3002281//Homo sapiens mRNA for KIAA0765 protein, partial cds.//0//2286bp//99%//AB018308
C-NT2RP3002286//Homo sapiens candidate tumor suppressor protein DICE 1 mRNA, complete cds.//0//2719bp//99%//AF097645
C-NT2RP3002324
C-NT2RP3002353
C-NT2RP3002571//Homo sapiens mRNA for KIAA1108 protein, partial cds.//4.40E-273//1311bp//97%//AB029031
C-NT2RP3002664
C-NT2RP3002737//Homo sapiens voltage-gated potassium channel KCNQ4 (KCNQ4) mRNA, complete cds.//0//1552bp//99%//AF105202
C-NT2RP3002887
C-NT2RP3002900//Homo sapiens CGI-109 protein mRNA, complete cds.//8.70E-298//1321bp//92%//AF151867
C-NT2RP3002983
C-NT2RP3003473//Homo sapiens CGI-55 protein mRNA, complete cds.//5.50E-275//1309bp//98%//AF151813
C-NT2RP3003532//OX-2 MEMBRANE GLYCOPROTEIN PRECURSOR (FRAGMENT).//4.40E-139//263aa//99%//P41217
C-NT2RP3004025
C-NT2RP3004067//Homo sapiens mRNA for NESCA, complete cds.//0//1962bp//99%//AB026894
C-NT2RP3004119//PEREGRIN (BE140 PROTEIN).//7.30E-39//227aa//43%//P55201
C-NT2RP3004294//Xenopus laevis ER1 mRNA, complete cds.//1.20E-71//335bp//79%//AF015454
C-NT2RP3004345//N2,N2-DIMETHYLGUANOSINE TRNA METHYLTRANSFERASE PRECURSOR (EC 2.1.1.32).//3.90E-18//279aa//27%//P15565
C-NT2RP4000634//Homo sapiens mitogen-activated protein kinase kinase kinase MEKK2 mRNA, complete cds.//0//1501bp//98%//AF111105
C-NT2RP4001001//Homo sapiens clone 24856 mRNA sequence, complete cds.//3.90E-301//1374bp//99%//AF131856
C-NT2RP4001877//Homo sapiens ribonucleoprotein RBM8 (RBM8) mRNA, complete cds.//0//2770bp//99%//AF127761
C-NT2RP4001879
C-NT2RP4002187//Homo sapiens steroid dehydrogenase homolog mRNA, complete cds.//0//2373bp//99%//AF078850
C-NT2RP4002451
C-NT2RP4002750//Mus musculus cationic amino acid transporter (CAT3) mRNA, complete cds.//1.00E-310//2084bp//81%//U70859
C-OVARC1000003//Homo sapiens sodium dependent phosphate transporter isoform NaPi-3b mRNA, complete cds.//4.30E-220//1158bp//94%//AF111856
C-OVARC1000313//Homo sapiens mRNA for KIAA0573 protein, partial cds.//0//1833bp//99%//AB011145
C-OVARC1000331//GMP REDUCTASE (EC 1.6.6.8) (GUANOSINE 5'-MONOPHOSPHATE OXIDOREDUCTASE).//9.40E-44//106aa//59%//P36959
C-OVARC1000553//DIPEPTIDYL PEPTIDASE IV (EC 3.4.14.5) (DPP IV) (THYMOCYTE-ACTIVATING MOLECULE) (THAM).//1.30E-23//169aa//40%//P28843
C-OVARC1000873//Homo sapiens mRNA for KIAA1247 protein, partial cds.//0//2178bp//99%//AB033073
C-OVARC1000995
C-OVARC1001260
C-OVARC1001336//Homo sapiens sodium dependent phosphate transporter isoform NaPi-3b mRNA, complete cds.//0//1435bp//99%//AF111856
C-OVARC1001570//Homo sapiens beta-site APP cleaving enzyme (BACE) mRNA, complete cds.//0//1792bp//100%//AF190725
C-OVARC1001607//Human beta-1,2-N-acetylglucosaminyltransferase II (MGAT2) gene, complete cds.//0//1836bp//96%//U15128
C-OVARC1001833//CIS-GOLGI MATRIX PROTEIN GM130.//6.60E-136//363aa//76%//Q62839
C-OVARC1001952//TRICHOHYALIN.//3.30E-16//487aa//27%//Q07283
C-PLACE1000986//Homo sapiens mRNA; cDNA DKFZp586C1817 (from clone DKFZp586C1817).//0//2055bp//99%//AL117450
C-PLACE1003407//Homo sapiens putative transmembrane protein (CLN5) mRNA, complete cds.//0//1965bp//99%//AF068227
C-PLACE1004078//Bovine mRNA for adseverin, complete cds.//0//2218bp//89%//D26549
C-PLACE1004492//VERPROLIN.//3.30E-07//149aa//29%//P37370
C-PLACE1005539//ACTIN POLYMERIZATION INHIBITOR (HEAT SHOCK 25 KD PROTEIN) (25-KD IAP).//3.10E-08//84aa//34%//Q00649
C-PLACE1005569//Homo sapiens mRNA for Z39Ig protein.//0//1768bp//99%//AJ132502
C-PLACE1005601
C-PLACE1005745//ORM1 PROTEIN.//2.40E-17//137aa//35%//P53224
C-PLACE1005815//COLORECTAL MUTANT CANCER PROTEIN (MCC PROTEIN).//1.50E-26//274aa//26%//P23508
C-PLACE1005927//HYPOTHETICAL 35.8 KD PROTEIN C12G12.12 IN CHROMOSOME I.//1.60E-38//333aa//33%//Q09875
C-PLACE1006071//LAMININ BETA-1 CHAIN PRECURSOR (LAMININ B1 CHAIN).//6.00E-08//215aa//26%//P02469
C-PLACE1006073//Homo sapiens mRNA for glucuronyltransferase I, complete cds.//4.10E-316//1020bp//96%//AB009598
C-PLACE1006079//Homo sapiens distal-less homeobox protein (DLX3) gene, complete cds.//0//1379bp//97%//AF028233
C-PLACE1006786
C-PLACE1007077//Homo sapiens mRNA for KIAA0977 protein, complete cds.//0//2578bp//99%//AB023194
C-PLACE1007971
C-PLACE1008282//HEME-REGULATED EUKARYOTIC INITIATION FACTOR EIF-2-ALPHA KINASE (EC 2.7.1.-) (HRI).//7.10E-274//627aa//82%//P33279
C-PLACE1008359//BEM46 PROTEIN (FRAGMENT).//1.70E-50//289aa//42%//P54069
C-PLACE1008744//Homo sapiens mRNA full length insert cDNA clone EUROIMAGE 248114.//0//1757bp//99%//AL079279
C-PLACE1010445
C-PLACE1010713//Homo sapiens steroid dehydrogenase homolog mRNA, complete cds.//0//2374bp//99%//AF078850
C-nnnnnnnnnnnn//Homo sapiens angiopoietin-2 mRNA, complete cds.//0//2227bp//99%//AF004327
C-PLACE1011181//MSP1 PROTEIN HOMOLOG.//9.40E-82//353aa//47%//P54815
C-PLACE1011364//MYOTONIN-PROTEIN KINASE (EC 2.7.1.-) (MYOTONIC DISTROPHY PROTEIN KINASE) (MDPK) (DM-KINASE) (DMK) (DMPK) (MT-PK).//2.20E-09//153aa//32%//Q09013
C-PLACE3000181//Human protocadherin 42 mRNA, complete cds for abbreviated PC42.//0//2719bp//95%//L11370
C-SKNMC1000014
C-SKNMC1000082//BRITTLE-1 PROTEIN PRECURSOR.//3.70E-31//307aa//30%//P29518
C-THYRO1000964
C-THYRO1001242//Homo sapiens cleft lip and palate transmembrane protein 1 (CLPTM1) mRNA, complete cds.//0//2468bp//99%//AF037339
C-THYRO1001608
C-THYRO1001641//Homo sapiens CGI-57 protein mRNA, complete cds.//0//1668bp//99%//AF151815
C-THYRO1001770//PROBABLE SERINE/THREONINE-PROTEIN KINASE YNL020C (EC 2.7.1.-).//6.30E-20/1169aa//35%//P53974
C-Y79AA1000030//Homo sapiens mRNA for Fe65L2, complete cds.//0//1828bp//100%//AB018247
C-Y79AA1001212//Homo sapiens SL15 protein mRNA, complete cds.//6.30E-306//1388bp//99%//AF038961
C-Y79AA1001426//ANION EXCHANGE PROTEIN 3 (CARDIAC/BRAIN BAND 3-LIKE PROTEIN) (CAE3/BAE3).//6.20E-66//609aa//31%//P48751
C-Y79AA1001427//Homo sapiens cytochrome b5 reductase 1 (B5R.1) mRNA, complete cds.//0//1588bp//99%//AF169481
C-Y79AA1001523//Homo sapiens transcriptional intermediary factor 1 alpha mRNA, complete cds.//0//2263bp//99%//AF119042
C-Y79AA1001530//Human beta-tubulin gene (5-beta) with ten Alu family members.//0//1920bp//98%//X00734
C-Y79AA1001592
C-Y79AA1001727//CELL SURFACE A33 ANTIGEN PRECURSOR.//1.10E-13//286aa//27%//Q99795
C-Y79AA1001787//PROBABLE CALCIUM-TRANSPORTING ATPASE 9 (EC 3.6.1.38).//1.70E-133//544aa//37%//Q12697
C-Y79AA1001793//Mus musculus mRNA for GSG1, complete cds.//3.70E-126//532bp//78%//D87325
C-Y79AA1001795//Homo sapiens mRNA for GalT4 protein.//2.30E-250//1137bp//99%//Y15061
C-Y79AA1001799//MITOCHONDRIAL RNA SPLICING PROTEIN MSR4.//3.40E-54//182aa//39%//P23500
C-Y79AA1001803//Homo sapiens secretogranin III mRNA, complete cds.//0//1871bp//99%//AF078851
C-Y79AA1001863
C-Y79AA1002058//Mus musculus Gng31g mRNA, complete cds.//4.10E-167//1145bp//83%//AF069954
C-Y79AA1002121//HISTONE H1.//4.90E-12//114aa//35%//P35060
C-Y79AA1002213//HYPOTHETICAL 52.7 KD PROTEIN C38C10.2 IN CHROMOSOME III.//1.20E-98//262aa//41%//Q03567
C-Y79AA1002373//Mus musculus mRNA for GSG1, complete cds.//7.20E-147//680bp//79%//D87325
C-Y79AA1002376//Rattus norvegicus cytoplasmic dynein intermediate chain 2B mRNA, complete cds.//1.50E-304//1667bp//90%//U39045
C-Y79AA1002378//Homo sapiens zinc finger protein NY-REN-21 antigen mRNA, partial cds.//0//963bp//99%//AF155100
C-Y79AA1002381//Homo sapiens cell cycle related kinase mRNA, complete cds.//0//1791bp//98%//AF035013
C-BNGH41000087//Homo sapiens mRNA for KIAA1247 protein, partial cds.//0//2294bp//99%//AB033073
C-HEMBA1001886
C-HEMBA1004067//Homo sapiens mRNA for KIAA0859 protein, complete cds.//8.30E-309//623bp//99%//AB020666
C-HEMBA1007226//Homo sapiens RPA-binding trans-activator (RBT1) mRNA, complete cds.//7.30E-273//1242bp//99%//AF192529
C-HEMBB1000309
C-HEMBB1000567
C-MAMMA1000102//APOLIPOPROTEIN L PRECURSOR (APO-L).//1.40E-21//221aa//35%//O14791
C-MAMMA1001066
C-MAMMA1001094//Human potential transcriptional repressor NOT4Hp (NOT4H) mRNA, complete cds.//0//1394bp//93%//U71267
C-MAMMA1001609
C-MAMMA1001901
C-MAMMA1002091//Homo sapiens CD39L2 (CD39L2) mRNA, complete cds.//0//2564bp//99%//AF039916
C-NT2RM1000462
C-NT2RM1000542//BETA-GALACTOSIDASE PRECURSOR (EC 3.2.1.23) (LACTASE).//1.40E-103//566aa//43%//P48982
C-NT2RM1000789//Homo sapiens mRNA for hTCF-4.//2.80E-221//757bp//99%//Y11306
C-NT2RM1000855//Homo sapiens sec61 homolog mRNA, complete cds.//0//3541bp//99%//AF084458
C-NT2RM1000899//MITOCHONDRIAL RNA SPLICING PROTEIN MSR4.//1.10E-54//182aa//39%//P23500
C-NT2RP2000092//ZINC FINGER PROTEIN 136.//1.90E-117//419aa//54%//P52737
C-NT2RP2001538//Homo sapiens mRNA; cDNA DKFZp434K2235 (from clone DKFZp434K2235).//0//2139bp//99%//AL117513
C-NT2RP2001921
C-NT2RP2003138//5'-TG-3'INTERACTING FACTOR (HOMEOBOX PROTEIN TGIF).//2.10E-08//104aa//46%//P70284
C-NT2RP2003302//Homo sapiens GIOT-4 mRNA for gonadotropin inducible transcription repressor-4, complete cds.//0//2891bp//99%//AB021644
C-NT2RP2003950//Homo sapiens clone 24778 unknown mRNA.//0//1545bp//99%//AF070572
C-NT2RP2005535//ZINC FINGER PROTEIN 85 (ZINC FINGER PROTEIN HPF4) (HTF1).//1.90E-172//489aa//62%//Q03923
C-NT2RP2005774//Homo sapiens GIOT-4 mRNA for gonadotropin inducible transcription repressor-4, complete cds.//6.90E-224//1461bp//72%//AB021644
C-NT2RP3000148//ZINC FINGER PROTEIN 84 (ZINC FINGER PROTEIN HPF2).//4.10E-106//350aa//47%//P51523
C-NT2RP3000232//ZINC FINGER PROTEIN 184 (FRAGMENT).//1.40E-130//693aa//41%//Q99676
C-NT2RP3000427
C-NT2RP3000652//ZINC FINGER PROTEIN 43 (ZINC PROTEIN HTF6).//1.90E-153//441aa//62%//P28160
C-NT2RP3001650//BONE MORPHOGENETIC PROTEIN 1 PRECURSOR (EC 3.4.24.-) (BMP-1).//2.20E-22//107aa//42%//P98063
C-NT2RP3002409//Homo sapiens mRNA for KIAA0719 protein, complete cds.//0//2404bp//99%//AB018262
C-NT2RP3002411//Homo sapiens steroid dehydrogenase homolog mRNA, complete cds.//0//2374bp//99%//AF078850
C-NT2RP3003448
C-NT2RP4002715
C-OVARC1000307//HYPOTHETICAL 29.7 KD PROTEIN CY339.02.//3.00E-19//194aa//35%//Q50658
C-PLACE1000907//ZINC FINGER PROTEIN 83 (ZINC FINGER PROTEIN HPF1).//1.30E-204//396aa//85%//P51522
C-PLACE1007081
C-PLACE1010011//Homo sapiens AAPT1-like protein mRNA, partial cds.//1.70E-237//1092bp//99%//AF047431
C-PLACE3000213//COMPLEMENT RECEPTOR TYPE 2 PRECURSOR (CR2) (COMPLEMENT C3D RECEPTOR).//4.60E-68//317aa//34%//P19070
C-PLACE4000354//COMPLEMENT RECEPTOR TYPE 1 PRECURSOR (C3B/C4B RECEPTOR) (CD35 ANTIGEN).//1.00E-129//482aa//29%//P17927
C-PLACE4000455
C-THYRO1000776//PROBABLE SULFATE PERMEASE SPBC3H7.02.//1.30E-68//442aa//36%//O74377
C-THYRO1001593//PUTATIVE SERINE/THREONINE-PROTEIN KINASE P78 (EC 2.7.1.-).//3.10E-203//550aa//62%//P27448
C-Y79AA1000750
C-Y79AA1000888//TRNA PSEUDOURIDINE SYNTHASE A (EC 4.2.1.70) (PSEUDOURIDYLATE SYNTHASE I) (PSEUDOURIDINE SYNTHASE I) (URACIL HYDROLYASE).//1.50E-21//267aa//32%//P70973
C-Y79AA1002129
C-Y79AA1002334//GLUCOSE REPRESSION MEDIATOR PROTEIN.//1.70E-10//333aa//23%//P14922
C-BNGH41000020//NADH-UBIQUINONE OXIDOREDUCTASE CHAIN 2 (EC 1.6.5.3).//9.80E-159//347aa//90%//P03891
C-BNGH41000087//N-ACETYLGLUCOSAMINE-6-SULFATASE PRECURSOR (EC 3.1.6.14) (G6S) (GLUCOSAMINE-6-SULFATASE).//1.20E-17//83aa//40%//P50426
C-BNGH41000091//POTASSIUM CHANNEL PROTEIN EAG.//1.20E-249//625aa//65%//Q02280
C-HEMBA1000006//Homo sapiens mRNA for NESCA, complete cds.//0//1230bp//92%//AB026894
C-HEMBA1000121//HYPOTHETICAL 68.7 KD PROTEIN ZK757.1 IN CHROMOSOME III.//4.80E-05//83aa//27%//P34679
C-HEMBA1000128//PATHOGENESIS-RELATED PROTEIN 1 PRECURSOR (PR-1).//3.20E-07//89aa//34%//P33154
C-HEMBA1000275
C-HEMBA1000300
C-HEMBA1000349//ATP-BINDING CASSETTE TRANSPORTER 1.//5.30E-65//352aa//39%//P41233
C-HEMBA1000443//Homo sapiens CGI-96 protein mRNA, complete cds.//4.70E-129//686bp//91%//AF151854
C-HEMBA1000462//Human potential transcriptional repressor NOT4Hp (NOT4H) mRNA, complete cds.//0//935bp//99%//U71267
C-HEMBA1000477//HYPOTHETICAL 64.8 KD PROTEIN IN GDI1-COX15 INTERGENIC REGION.//1.40E-38//344aa//34%//P40085
C-HEMBA1000590//Homo sapiens mRNA for matrilin-4, partial.//2.00E-273//1254bp//99%//AJ007581
C-HEMBA1000634//Homo sapiens T-cell activation protein (PGR1) gene, complete cds.//0//994bp//99%//AF116272
C-HEMBA1000671//ZINC FINGER. PROTEIN 184 (FRAGMENT).//3.90E-104//388aa//46%//Q99676
C-HEMBA1000713//Homo sapiens 10kD protein (BC10) mRNA, complete cds.//0//1254bp//99%//AF053470
C-HEMBA1000732//Homo sapiens mRNA for latent transforming growth factor-beta binding protein-4.//0//2153bp//94%//Y13622
C-HEMBA1000745//COLLAGEN ALPHA 1(I) CHAIN (FRAGMENTS).//2.00E-07//445aa//27%//P02454
C-HEMBA1000835//FIBRILLIN 2 PRECURSOR.//1.30E-42//214aa//45%//P35556
C-HEMBA1000875
C-HEMBA1000907
C-HEMBA1000940//GAP JUNCTION ALPHA-3 PROTEIN (CONNEXIN 44) (CX44).//2.90E-39//362aa//31%//P41987
C-HEMBA1000962
C-HEMBA1001184//SH3BGR PROTEIN (21-GLUTAMIC ACID-RICH PROTEIN) (21-GARP).//2.50E-32//100aa//60%//P55822
C-HEMBA1001221//AGRIN PRECURSOR.//2.50E-25//294aa//29%//P31696
C-HEMBA1001228//Human germline oligomeric matrix protein (COMP) mRNA, complete cds.//7.80E-286//1105bp//94%//L32137
C-HEMBA1001272//Homo sapiens mRNA for KIAA1171 protein, partial cds.//0//1490bp//99%//AB032997
C-HEMBA1001296
C-HEMBA1001297//Homo sapiens putative transcription factor CA150 mRNA, complete cds.//4.60E-276//1081bp//99%//AF017789
C-HEMBA1001390//Mus musculus polymerase I-transcript release factor mRNA, complete cds.//2.50E-57//464bp//82%//AF036249
C-HEMBA1001563
C-HEMBA1001621//PROBABLE G PROTEIN-COUPLED RECEPTOR APJ.//3.50E-123//259aa//89%//P35414
C-HEMBA1001878//Homo sapiens U5 snRNP-specific 40 kDa protein mRNA, complete cds.//0//1488bp//99%//AF090988
C-HEMBA1001886//ZINC FINGER PROTEIN 85 (ZINC FINGER PROTEIN HPF4) (HTF1).//1.40E-148//421aa//60%//Q03923
C-HEMBA1002048//ODD-SKIPPED PROTEIN.//1.60E-55//122aa//75%//P23803
C-HEMBA1002131//PROCOLLAGEN-LYSINE,2-OXOGLUTARATE 5-DIOXYGENASE 1//4.10E-10//140aa//30%//P24802
C-HEMBA1002163//HYPOTHETICAL 40.7 KD PROTEIN IN DAK1-ORC1 INTERGENIC REGION.//9.40E-28//309aa//30%//Q04651
C-HEMBA1002164
C-HEMBA1002167//Rattus norvegicus neuroligin I mRNA, complete cds.//1.30E-305//1643bp//91%//U22952
C-HEMBA1002178//PROCOLLAGEN-LYSINE,2-OXOGLUTARATE 5-DIOXYGENASE 1 PRECURSOR (EC 1.14.11.4) (LYSYL HYDROXYLASE 1) (LH1).//3.70E-10//140aa//30%//P24802
C-HEMBA1002195//VEGETATIBLE INCOMPATIBILITY PROTEIN HET-E-1.//8.80E-23//221aa//31%//Q00808
C-HEMBA1002227//Homo sapiens mRNA for 80K-L protein, complete cds.//0//1324bp//98%//D10522
C-HEMBA1002239
C-HEMBA1002316//GTP-BINDING PROTEIN HFLX.//5.80E-12//196aa//29%//P25519
C-HEMBA1002420
C-HEMBA1002421//Human heparan sulfate proteoglycan (HSPG) core protein, 3' end.//0//2097bp//99%//J04621
C-HEMBA1002524//Human MHC Class I region proline rich protein mRNA, complete cds.//0//1763bp//95%//U63336
C-HEMBA1002551//PUTATIVE SERINE/THREONINE-PROTEIN KINASE PKWA (EC 2.7.1.-).//9.80E-08//110aa//37%//P49695
C-HEMBA1002767//Homo sapiens chromosome 1p33-p34 beta-1,4-galactosyltransferase mRNA, complete cds.//0//1497bp//99%//AF038660
C-HEMBA1002985
C-HEMBA1002992//UBIQUITIN-LIKE PROTEIN DSK2.//2.00E-21//216aa//35%//P48510
C-HEMBA1003047//Homo sapiens intrinsic factor-B12 receptor precursor, mRNA, complete cds.//0//1768bp//99%//AF034611
C-HEMBA1003072//Gallus gallus single-strand DNA-binding protein csdp mRNA, partial cds.//3.30E-93//927bp//73%//U68380
C-HEMBA1003101//Homo sapiens tyrosylprotein sulfotransferase-2 mRNA, complete cds.//0//1854bp//99%//AF049891
C-HEMBA1003120//ZINC FINGER PROTEIN 184 (FRAGMENT).//1.00E-193//547aa//54%//Q99676
C-HEMBA1003230//Homo sapiens fibulin-5.//5.60E-308//1398bp//99%//AJ133490
C-HEMBA1003294
C-HEMBA1003315//Mus musculus mRNA for DNA helicase, complete cds.//6.30E-250//1426bp//88%//AB013912
C-HEMBA1003392//Homo sapiens LDL receptor-related protein 6 (LRP6) mRNA, complete cds.//0//1721bp//100%//AF074264
C-HEMBA1003399//MVP1 PROTEIN.//2.30E-15//279aa//23%//P40959
C-HEMBA1003487
C-HEMBA1003497//ZINC FINGER PROTEIN 151 (POLYOMAVIRUS LATE INITIATOR PROMOTER BINDING PROTEIN) (LP-1) (ZINC FINGER PROTEIN Z13).//4.00E-28//358aa//29%//Q60821
C-HEMBA1003530//S.scrofa mRNA for BM88 antigen.//1.20E-60//900bp//66%//X82027
C-HEMBA1003602//HYPOTHETICAL 29.7 KD PROTEIN CY339.02.//2.80E-21//200aa//33%//Q50658
C-HEMBA1003732//SFT2 PROTEIN.//1.50E-06//162aa//30%//P38166
C-HEMBA1003945//Homo sapiens hypothetical 43.2 Kd protein mRNA, complete cds.//8.90E-287//757bp//97%//AF077030
C-HEMBA1004007
C-HEMBA1004067//Homo sapiens mRNA for KIAA0859 protein, complete cds.//0.00E+00//623bp//99%//AB020666
C-HEMBA1004085
C-HEMBA1004110//Homo sapiens intersectin short form mRNA, complete cds.//0//2033bp//99%//AF064243
C-HEMBA1004250//CADHERIN-RELATED TUMOR SUPPRESSOR PRECURSOR (FAT PROTEIN).//6.40E-51//277aa//35%//P33450
C-HEMBA1004391//TUMOR SUPPRESSOR PROTEIN DCC PRECURSOR.//5.60E-20//194aa//26%//F70211
C-HEMBA1004444//GLYCOPROTEIN 25L PRECURSOR (GP25L).//4.60E-41//148aa//52%//P27869
C-HEMBA1004454
C-HEMBA1004505//MANNOSYL-OLIGOSACCHARIDE ALPHA-1,2-MANNOSIDASE ISOFORM 1 (EC 3.2.1.113) (MAN(9)-ALPHA-MANNOSIDASE).//2.70E-45//239aa//43%//P53624
C-HEMBA1004785//MODIFIER 3 PROTEIN (M33).//1.40E-27//221aa//33%//P30658
C-HEMBA1004797
C-HEMBA1004952
C-HEMBA1004971
C-HEMBA1004982//TETRACYCLINE RESISTANCE PROTEIN, CLASS E (TETA(E)).//6.30E-10//149aa//26%//Q07282
C-HEMBA1005070//SKIN SECRETORY PROTEIN XP2 PRECURSOR (APEG PROTEIN).//1.10E-05//187aa//29%//P17437
C-HEMBA1005084//NON-RECEPTOR TYROSINE KINASE SPORE LYSIS A (EC 2.7.1.112) (TYROSINE- PROTEIN KINASE 1).//1.20E-07//102aa//37%//P18160
C-HEMBA1005145
C-HEMBA1005230//ZINC FINGER PROTEIN 140.//2.00E-17//83aa//66%//P52738
C-HEMBA1005246//HEPATOCYTE NUCLEAR FACTOR 3 FORKHEAD HOMOLOG 4 (HFH-4).//2.10E-15//230aa//28%//Q92949
C-HEMBA1005267//ANKYRIN, BRAIN VARIANT 1 (ANKYRIN B) (ANKYRIN, NONERYTHROID).//8.40E-14//187aa//33%//Q01484
C-HEMBA1005430
C-HEMBA1005449//EXTENSIN PRECURSOR (CELL WALL HYDROXYPROLINE-RICH GLYCOPROTEIN).//5.40E-10//224aa//24%//P13983
C-HEMBA1005489//Mus musculus semaphorin cytoplasmic domain-associated protein 3A (Semcap3) mRNA, complete cds.//8.40E-255//924bp//80%//AF127084
C-HEMBA1005522//COAGULATION FACTOR VII PRECURSOR (EC 3.4.21.21).//7.70E-15//78aa//51%//P98139
C-HEMBA1005545//MUSCARINIC ACETYLCHOLINE RECEPTOR M3.//0//590aa//100%//P20309
C-HEMBA1005698//Homo sapiens vesicle trafficking protein (SEC22C) mRNA, complete cds.//6.60E-163//753bp//99%//AF039568
C-HEMBA1005913
C-HEMBA1005929//H.sapiens mRNA for serine/threonine protein kinase EMK.//6.50E-92//1092bp//69%//X97630
C-HEMBA1005945//BRITTLE-1 PROTEIN PRECURSOR.//1.70E-29//220aa//35%//P29518
C-HEMBA1006016
C-HEMBA1006171
C-HEMBA1006276//ZINC FINGER PROTEIN 90 (ZFP-90) (ZINC FINGER PROTEIN NK10).//1.70E-06//56aa//57%//Q61967
C-HEMBA1006299
C-HEMBA1006311
C-HEMBA1006335
C-HEMBA1006357//SECRETORY CARRIER-ASSOCIATED MEMBRANE PROTEIN 2.//3.70E-39//136aa//52%//O15127
C-HEMBA1006430//Human putative transmembrane protein precursor (B5) mRNA, complete cds.//2.40E-70//1108bp//65%//L38961
C-HEMBA1006482//Homo sapiens h-sco1 (SCO1) mRNA, nuclear gene encoding mitochondrial protein, complete cds.//0//1101bp//98%//AF026852
C-HEMBA1006517
C-HEMBA1006544
C-HEMBA1006572//ODD-SKIPPED PROTEIN.//2.60E-39//85aa//83%//P23803
C-HEMBA1006658//Homo sapiens mRNA for NIK, partial cds.//0//1500bp//98%//AB013385
C-HEMBA1006707//Homo sapiens mRNA for matrilin-4, partial.//0//2003bp//99%//AJ007581
C-HEMBA1006724
C-HEMBA1006749//Homo sapiens mRNA for matrilin-4, partial.//1.40E-275//1942bp//83%//AJ007581
C-HEMBA1006770//FLOWERING TIME CONTROL PROTEIN FCA.//1.20E-33//352aa//34%//O04425
C-HEMBA1006902//Homo sapiens mRNA for matrilin-4, partial.//4.80E-275//1799bp//85%//AJ007581
C-HEMBA1006912
C-HEMBA1006916//Homo sapiens Grb14 mRNA, complete cds.//3.00E-277//1010bp//95%//L76687
C-HEMBA1006960
C-HEMBA1007013//Mus musculus sphingosine kinase (SPHK1b) mRNA, complete cds.//1.10E-14//412bp//63%//AF068749
C-HEMBA1007057
C-HEMBA1007063
C-HEMBA1007226//Homo sapiens RPA-binding trans-activator (RBT1) mRNA, complete cds.//7.30E-273//1242bp//99%//AF192529
C-HEMBA1007241//HYPOTHETICAL 24.5 KD PROTEIN IN SAP185-BCK1 INTERGENIC REGION.//2.70E-14//106aa//42%//P40857
C-HEMBA1007291
C-HEMBA1007332//Homo sapiens mRNA for unr-interacting protein.//6.40E-83//266bp//98%//AJ010025
C-HEMBB1000106//CELLULAR NUCLEIC ACID BINDING PROTEIN (CNBP).//1.60E-10//139aa//30%//P53996
C-HEMBB1000276
C-HEMBB1000309
C-HEMBB1000407
C-HEMBB1000447//Homo sapiens JWA protein mRNA, complete cds.//0//2059bp//99%//AF070523
C-HEMBB1000542//Mus musculus bromodomain-containing protein BP75 mRNA, complete cds.//2.60E-232//1452bp//85%//AF084259
C-HEMBB1000567
C-HEMBB1000642
C-HEMBB1000668//Homo sapiens mRNA for KIAA0893 protein, complete cds.//0//2375bp//99%//AB020700
C-HEMBB1000679//C.familiaris mRNA for TRAM-protein.//4.10E-210//1149bp//80%//X63678
C-HEMBB1000881//Danio rerio mRNA for MINDIN2, complete cds.//1.70E-67//948bp//66%//AB006085
C-HEMBB1000905/ITRANSCRIPTIONAL REPRESSOR RCO-1.//1.00E-11//311aa//27%//P78706
C-HEMBB1001026//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//5.30E-11//142aa//30%//P32802
C-HEMBB1001048//SARCALUMENIN PRECURSOR.//6.50E-18//154aa//33%//P13666
C-HEMBB1001200
C-HEMBB1001407
C-HEMBB1001530//SLS1 PROTEIN PRECURSOR.//9.80E-10//273aa//27%//Q99158
C-HEMBB1001547//Homo sapiens CGI-02 protein mRNA, complete cds.//0//2311bp//99%//AF132937
C-HEMBB1001573
C-HEMBB1001847//NEUROGENIC PROTEIN BIG BRAIN.//4.70E-06//258aa//24%//P23645
C-HEMBB1001959//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//7.30E-14//97aa//38%//P25210
C-HEMBB1001978
C-HEMBB1002039
C-HEMBB1002041//Homo sapiens transmembrane protein TENB2 (TENB2) mRNA, complete cds.//0//1746bp//99%//AF179274
C-HEMBB1002051//Homo sapiens Ets transcription factor ESE-2b mRNA, complete cds.//1.30E-95//454bp//99%//AF115403
C-HEMBB1002120//UDP-N-ACETYLGLUCOSAMINE--PEPTIDE N-ACETYLGLUCOSAMINYLTRANSFERASE 110 KD SUBUNIT (EC 2.4.1.-) (O-GLCNAC TRANSFERASE P110 SUBUNIT).//4.90E-22//337aa//27%//P56558
C-HEMBB1002162//Homo sapiens genethonin 1 mRNA, complete cds.//8.30E-67//328bp//99%//AF062534
C-HEMBB1002228
C-HEMBB 1002245//PROSTAGLANDIN F2-ALPHA RECEPTOR REGULATORY PROTEIN PRECURSOR (PROSTAGLANDIN F2-ALPHA RECEPTOR ASSOCIATED PROTEIN).//0//879aa//89%//Q62786
C-HEMBB1002302
C-HEMBB1002427//FUCOSYLGLYCOPROTEIN ALPHA-N-ACETYLGALACTOSAMINYLTRANSFERASE (EC 2.4.1.40) (HISTO-BLOOD GROUP A TRANSFERASE) (A TRANSFERASE) / FUCOSYLGLYCOPROTEIN 3-ALPHA-GALACTOSYLTRANSFERASE (EC 2.4.1.37) (HISTO-BLOOD GROUP B TRANSFERASE) (B TRANSFERASE) (NAGAT).//1.80E-70//221aa//50%//P16442
C-HEMBB1002465//ACYL-COA DEHYDROGENASE (EC 1.3.99.-).//2.30E-53//249aa//48%//P45857
C-HEMBB1002661//Homo sapiens cardiovascular helix-loop-helix factor 2 (CHF2) mRNA, complete cds.//0//2174bp//99%//AF176422
C-HEMBB1002663
C-HEMBB1002693
C-MAMMA1000046
C-MAMMA1000102//APOLIPOPROTEIN L PRECURSOR (APO-L).//1.40E-21//221aa//35%//O14791
C-MAMMA1000106
C-MAMMA1000118
C-MAMMA1000141
C-MAMMA1000204//Homo sapiens dysferlin mRNA, complete cds.//0//2028bp//99%//AF075575
C-MAMMA1000226
C-MAMMA1000403//Homo sapiens CDK4-binding protein p34SEI1 (SEI1) mRNA, complete cds.//1.20E-255//1165bp//99%//AF117959
C-MAMMA1000449
C-MAMMA1000457//Human NADH-cytochrome b5 reductase mRNA, 3' end.//9.50E-79//829bp//71%//M16462
C-MAMMA1000473//HYPOTHETICAL 35.6 KD PROTEIN IN SPC1-ILV3 INTERGENIC REGION.//5.10E-45//299aa//34%//P47088
C-MAMMA1000496//MIC1 PROTEIN.//3.00E-25//202aa//33%//P53258
C-MAMMA1000528
C-MAMMA1000591//POLYPEPTIDE N-ACETYLGALACTOSAMINYLTRANSFERASE (EC 2.4.1.41) (PROTEIN- UDP ACETYLGALACTOSAMINYLTRANSFERASE) (UDP-GALNAC:POLYPEPTIDE, N- ACETYLGALACTOSAMINYLTRANSFERASE) (GALNAC-T1).//1.20E-115//515aa//49%//Q07537
C-MAMMA10006141/Homo sapiens pseudouridine synthase I (PUS1) mRNA, partial cds.//2.10E-302//1370bp//99%//AF116238
C-MAMMA1000652
C-MAMMA1000681//PROBABLE G PROTEIN-COUPLED RECEPTOR EDG-1.//9.40E-82//311aa//52%//O08530
C-MAMMA1000706
C-MAMMA1000788//Bos taurus P14 (p14) mRNA, complete cds.//3.90E-85//502bp//89%//AF037349
C-MAMMA1000810
C-MAMMA1000814
C-MAMMA1000881//Homo sapiens protein kinase (SGK3) mRNA, complete cds.//0//1292bp//100%//AF169035
C-MAMMA1000986
C-MAMMA1000994//Homo sapiens ISLR(immunoglobulin superfamily containing leucine-rich repeat) mRNA, complete cds, alternatively spliced transcript ISLR-2.//0//2211bp//99%//AB024536
C-MAMMA1001043//MRC OX-45 SURFACE ANTIGEN PRECURSOR (BCM1 SURFACE ANTIGEN) (BLAST-1) (CD48).//2.90E-12//239aa//28%//P10252
C-MAMMA1001066
C-MAMMA1001094//Human potential transcriptional repressor NOT4Hp (NOT4H) mRNA, complete cds.//0//1394bp//93%//U71267
C-MAMMA1001141
C-MAMMA1001150//PROTEIN KINASE C, MU TYPE (EC 2.7.1.-) (NPKC-MU).//7.50E-304//587aa//68%//Q15139
C-MAMMA1001237//MONOCARBOXYLATE TRANSPORTER 2 (MCT 2).//7.70E-64//196aa//41%//P53988
C-MAMMA1001284
C-MAMMA1001310//HYPOTHETICAL 57.3 KD TRP-ASP REPEATS CONTAINING PROTEIN IN POM152- REC114 INTERGENIC REGION.//1.50E-67//441aa//37%//Q04225
C-MAMMA1001344
C-MAMMA1001418//HYPOTHETICAL PROTEIN HI0519.//6.90E-27//181aa//38%//P44742
C-MAMMA1001532//ZINC FINGER PROTEIN 84 (ZINC FINGER PROTEIN HPF2).//5.60E-126//319aa//56%//P51523
C-MAMMA1001609//MYOSIN II HEAVY CHAIN, NON MUSCLE.//1.50E-26//204aa//38%//P05659
C-MAMMA1001615//NEUROGENIC DIFFERENTIATION FACTOR 1.//3.80E-11//90aa//42%//Q13562
C-MAMMA1001623
C-MAMMA1001634
C-MAMMA1001893
C-MAMMA1001901
C-MAMMA1001957
C-MAMMA1001978//Cimex lectularius apyrase (APY) mRNA, complete cds.//6.70E-19//988bp//56%//AF085499
C-MAMMA1002070//PLASMINOGEN (EC 3.4.21.7) (FRAGMENT).//1.10E-07//103aa//33%//Q01177
C-MAMMA1002080//RAS-RELATED PROTEIN RAB-13.//1.80E-29//208aa//37%//P51153
C-MAMMA1002087
C-MAMMA1002091//Homo sapiens CD39L2 (CD39L2) mRNA, complete cds.//0//2564bp//99%//AF039916
C-MAMMA1002095//CALCIUM-TRANSPORTING ATPASE 1 (EC 3.6.1.38) (P-TYPE CALCIUM ATPASE).//3.70E-222//867aa//52%//O43108
C-MAMMA1002128//ABC1 PROTEIN HOMOLOG PRECURSOR.//2.50E-97//464aa//45%//Q92338
C-MAMMA1002142//NON-RECEPTOR TYROSINE KINASE SPORE LYSIS A (EC 2.7.1.112) (TYROSINE- PROTEIN KINASE 1).//9.80E-17//146aa//35%//P18160
C-MAMMA1002165//Homo sapiens connective tissue growth factor related protein WISP-2 (WISP2) mRNA, complete cds.//4.80E-60//382bp//89%//AF100780
C-MAMMA1002205//Homo sapiens mRNA; cDNA DKFZp586C091 (from clone DKFZp586C091).//2.00E-81//308bp//81%//AL050119
C-MAMMA1002224
C-MAMMA1002234//SIGNAL RECOGNITION PARTICLE 68 KD PROTEIN (SRP68).//0//627aa//96%//Q00004
C-MAMMA1002586//Homo sapiens alpha 1,2-mannosidase mRNA, complete cds.//0//2228bp//99%//AF148509
C-MAMMA1002633
C-MAMMA1003126//SARCALUMENIN PRECURSOR.//1.10E-51//388aa//32%//P13666
C-NT2RM1000462//ATRIAL GLAND-SPECIFIC ANTIGEN PRECURSOR (AGSA).//8.60E-14//104aa//40%//P15287
C-NT2RM1000542//BETA-GALACTOSIDASE PRECURSOR (EC 3.2.1.23) (LACTASE).//1.40E-103//566aa//43%//P48982
C-NT2RM1000580//GLYCOSYLTRANSFERASE ALG2 (EC 2.4.1.-).//8.50E-75//301aa//39%//P43636
C-NT2RM1000789//Homo sapiens mRNA for hTCF-4.//2.80E-221//757bp//99%//Y11306
C-NT2RM1000855//Homo sapiens sec61 homolog mRNA, complete cds.//0//3541bp//99%//AF084458
C-NT2RM1000858//tricarboxylate carrier [rats, liver, mRNA Partial, 2986 nt].//2.10E-98//1035bp//70%//S70011
C-NT2RM1000899//MITOCHONDRIAL RNA SPLICING PROTEIN MSR4.//1.10E-54//182aa//39%//P23500
C-NT2RM2000241
C-NT2RM2000306//PUTATIVE GTP-BINDING PROTEIN W08E3.3.//4.50E-130//362aa//68%//P91917
C-NT2RM2000410
C-NT2RM2000423//BETA-GALACTOSIDASE PRECURSOR (EC 3.2.1.23) (LACTASE).//1.80E-38//308aa//35%//P48982
C-NT2RM2000497//CHL1 PROTEIN.//9.90E-24//296aa//29%//P22516
C-NT2RM2000514//Homo sapiens F-box protein Fbx21 (FBX21) mRNA, complete cds.//4.40E-304//1374bp//99%//AF174601
C-NT2RM2000565//HYPOTHETICAL 85.7 KD PROTEIN C13G6.03 IN CHROMOSOME I.//4.40E-304//394aa//43%//Q09782
C-NT2RM2000582//Homo sapiens mRNA for KIAA1053 protein, partial cds.//0//2666bp//99%//AB028976
C-NT2RM2000589//Bos taurus myosin X, complete cds.//0//4376bp//84%//U55042
C-NT2RM2000622//Mus musculus F-box protein FBL10 mRNA, partial cds.//3.00E-203//915bp//91%//AF176524
C-NT2RM2000632//EXCISION REPAIR PROTEIN ERCC-6 (COCKAYNE SYNDROME PROTEIN CSB).//6.40E-62//183aa//47%//Q03468
C-NT2RM2000773//Homo sapiens KNSL4 and MAZ genes for kinesin-like DNA binding protein and Myc-associated zinc finger protein, complete cds.//3.00E-289//1092bp//99%//AB017335
C-NT2RM2001126//Homo sapiens mRNA for multi PDZ domain protein.//0//1600bp//99%//AJ001319
C-NT2RM2001558//Homo sapiens protein kinase A binding protein AKAP110 mRNA, complete cds.//0//2398bp//99%//AF093408
C-NT2RM2001626//FLIGHTLESS-I PROTEIN HOMOLOG.//4.30E-19//362aa//26%//P34268
C-NT2RM2001643
C-NT2RM2001738//SOF1 PROTEIN.//3.00E-110//325aa//47%//P33750
C-NT2RM2001792//Homo sapiens angiopoietin-related protein-2 mRNA,complete cds.//7.10E-149//995bp//86%//AF125175
C-NT2RM2001818//SIALIDASE (EC 3.2.1.18) (NEURAMINIDASE) (NA) (MAJOR SURFACE ANTIGEN).//4.30E-11//488aa//26%//P23253
C-NT2RM2001902//Homo sapiens mRNA for PAK4 protein.//5.40E-216//988bp//99%//AJ011855
C-NT2RM2001939//Human G protein-coupled receptor GPR-NGA gene, complete cds.//0//1559bp//98%//U55312
C-NT2RM2001941//MUSCARINIC ACETYLCHOLINE RECEPTOR M1.//7.40E-38//193aa//34%//P08482
C-NT2RM4000100//Homo sapiens Leman coiled-coil protein (LCCP) mRNA, complete cds.//0//2678bp//99%//AF175966
C-NT2RM4000115
C-NT2RM4000198//BUTYROPHILIN PRECURSOR (BT).//5.10E-12//162aa//33%//Q13410
C-NT2RM4000284//Human IgG Fc receptor hFcRn mRNA, complete cds.//1.30E-257//603bp//96%//U12255
C-NT2RM4000295
C-NT2RM4000326//ZINC FINGER PROTEIN 84 (ZINC FINGER PROTEIN HPF2).//9.00E-100//434aa//43%//P51523
C-NT2RM4000417//SYNAPTOTAGMIN II.//2.70E-23//293aa//30%//P46097
C-NT2RM4000444//ANTIGEN PEPTIDE TRANSPORTER 1 (APT1).//1.70E-112//493aa//44%//P36370
C-NT2RM4000587
C-NT2RM4000593//HYPOTHETICAL 111.9 KD PROTEIN C22H10.03C IN CHROMOSOME I.//3.90E-27//576aa//24%//Q10297
C-NT2RM4000648//K-GLYPICAN PRECURSOR.//4.00E-193//531aa//66%//P51655
C-NT2RM4000761//CYTOCHROME C OXIDASE POLYPEPTIDE I (EC 1.9.3.1).//2.50E-245//306aa//91%//P00395
C-NT2RM4000965//PUTATIVE IMPORTIN BETA-4 SUBUNIT (KARYOPHERIN BETA-4 SUBUNIT).//9.00E-44//520aa//29%//O60100
C-NT2RM4000997//MONO- AND DIACYLGLYCEROL LIPASE PRECURSOR (EC 3.1.1.-) (MDGL).//1.80E-10//189aa//30%//P25234
C-NT2RM4001321
C-NT2RM4001325//CHONDROITIN 6-SULFOTRANSFERASE (EC 2.8.2.17) (C6ST).//2.90E-48//343aa//34%//Q92179
C-NT2RM4001377//R.norvegicus LL5 mRNA.//8.50E-236//990bp//87%//X74226
C-NT2RM4001735
C-NT2RM4001768//Homo sapiens CGI-82 protein mRNA, complete cds.//0//1925bp//99%//AF151840
C-NT2RM4001843//BETA-GALACTOSIDASE PRECURSOR (EC 3.2.1.23) (LACTASE).//6.20E-33//263aa//38%//P48982
C-NT2RM4002352//Homo sapiens hLRp105 mRNA for LDL receptor related protein 105, complete cds.//0//2184bp//99%//AB009462
C-NT2RP1000002
C-NT2RP1000050
C-NT2RP1000181//Homo sapiens delta-6 fatty acid desaturase mRNA, complete cds.//3.30E-121//1394bp//69%//AF126799
C-NT2RP1000239
C-NT2RP1000261//ORM1 PROTEIN.//2.40E-17//137aa//35%//P53224
C-NT2RP1000271//ZINC FINGER PROTEIN 85 (ZINC FINGER PROTEIN HPF4) (HTF1).//4.70E-199//547aa//66%//Q03923
C-NT2RP1000300//Human transporter protein (g17) mRNA, complete cds.//3.80E-26//758bp//62%//U49082
C-NT2RP1000325//H.sapiens gene for phosphate carrier.//0//439bp//98%//X77337
C-NT2RP1000448
C-NT2RP1000465//UBIQUITIN-LIKE PROTEIN SMT3.//5.10E-07//81aa//33%//P55857
C-NT2RP1000468//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//2.00E-13//97aa//38%//P25210
C-NT2RP1000551//Homo sapiens putative interferon-related protein (SM15) mRNA, partial cds.//0//1761bp//99%//U09585
C-NT2RP1000579//Human succinate dehydrogenase flavoprotein subunit (SDH) mRNA, complete cds.//0//1951bp//94%//L21936
C-NT2RP1000613//CARBONIC ANHYDRASE VI (EC 4.2.1.1) (SALIVARY) (CARBONATE DEHYDRATASE VI).//3.40E-52//304aa//40%//P08060
C-NT2RP1000679
C-NT2RP1000740
C-NT2RP1000903
C-NT2RP1000981//CELL SURFACE A33 ANTIGEN PRECURSOR.//3.60E-14//286aa//27%//Q99795
C-NT2RP1001004//F-SPONDIN PRECURSOR.//9.20E-43//322aa//35%//P35446
C-NT2RP1001020//SERINE/THREONINE-PROTEIN KINASE PAK-BETA (EC 2.7.1.-) (BETA-PAK) (P21-ACTIVATED KINASE 3) (CDC42/RAC EFFECTOR KINASE PAK-B).//9.70E-22//227aa//31%//Q61036
C-NT2RP1001031//VEGETATIBLE INCOMPATIBILITY PROTEIN HET-E-1.//3.40E-42//285aa//35%//Q00808
C-NT2RP1001563//TESTIS-SPECIFIC PROTEIN TPX-1 PRECURSOR (AUTOANTIGEN 1) (25 KD ACROSOMAL AUTOANTIGEN) (AA1).//9.70E-19//201aa//31%//Q60477
C-NT2RP2000092//ZINC FINGER PROTEIN 136.//1.90E-117//419aa//54%//P52737
C-NT2RP2000178//MITOCHONDRIAL ION PROTEASE HOMOLOG 1 PRECURSOR (EC 3.4.21.-).//2.40E-192//778aa//48%//P93647
C-NT2RP2000240
C-NT2RP2000394//Gallus gallus p52 pro-apototic protein mRNA, complete cds.//1.60E-90//956bp//70%//AF029071
C-NT2RP2000447//GOLGIN-95.//2.80E-33//99aa//66%//Q08379
C-NT2RP2000479
C-NT2RP2000514//Homo sapiens roundabout 2 (robo2) mRNA, partial cds.//3.00E-185//855bp//99%//AF040991
C-NT2RP2000533//Homo sapiens cornichon protein mRNA, complete cds.//1.30E-290//1324bp//99%//AF070654
C-NT2RP2000610//CCAAT-BINDING TRANSCRIPTION FACTOR SUBUNIT A (CBF-A) (NF-Y PROTEIN CHAIN B) (NF-YB) (CAAT-BOX DNA BINDING PROTEIN SUBUNIT B).//1.50E-13//97aa//38%//P25210
C-NT2RP2000616//EXTENSIN PRECURSOR (CELL WALL HYDROXYPROLINE-RICH GLYCOPROTEIN).//4.10E-12//323aa//30%//P13983
C-NT2RP2000649//Homo sapiens mRNA for Hs Ste24p, complete cds.//0//2847bp//99%//AB016068
C-NT2RP2000663
C-NT2RP2000694//Homo sapiens 5T4 oncofetal trophoblast glycoprotein gene.//0//2278bp//99%//AJ012159
C-NT2RP2000712//ZINC FINGER PROTEIN 135.//3.70E-87//296aa//53%//P52742
C-NT2RP2000739//ZINC FINGER PROTEIN 83 (ZINC FINGER PROTEIN HPF1).//7.50E-73//387aa//37%//P51522
C-NT2RP2000818//Homo sapiens xenotropic and polytropic murine leukemia virus receptor (X3) mRNA, complete cds.//0//2724bp//99%//AF089744
C-NT2RP2000903//Homo sapiens 5T4 oncofetal trophoblast glycoprotein gene.//0//2276bp//100%//AJ012159
C-NT2RP2001200//Homo sapiens mRNA for KIAA0676 protein, partial cds.//0//1539bp//100%//AB014576
C-NT2RP2001223//MYOTUBULARIN-RELATED PROTEIN 3 (FRAGMENT).//3.30E-05//76aa//39%//Q13615
C-NT2RP2001276//NPDC-1 PROTEIN PRECURSOR.//3.00E-133//331aa//77%//Q64322
C-NT2RP2001388//TRNA-SPLICING ENDONUCLEASE SUBUNIT SEN2 (EC 3.1.27.9) (TRNA-INTRON ENDONUCLEASE).//5.90E-13//157aa//33%//P16658
C-NT2RP2001469//VEGETATIBLE INCOMPATIBILITY PROTEIN HET-E-1.//1.30E-14//242aa//24%//Q00808
C-NT2RP2001480//Homo sapiens thrombospondin 3 (THBS3) gene, complete cds.//0//2547bp//99%//L38969
C-NT2RP2001495//Human transporter protein (g17) mRNA, complete cds.//2.20E-65//641bp//65%//U49082
C-NT2RP2001514//PROBABLE CALCIUM-TRANSPORTING ATPASE 6 (EC 3.6.1.38).//1.20E-133//429aa//41%//P39986
C-NT2RP2001529//Homo sapiens mRNA for ZIP-kinase, complete cds.//0//2079bp//99%//AB007144
C-NT2RP2001538//Mus musculus mSin3A (sin3A) mRNA, complete cds.//7.60E-272//1480bp//84%//U22394
C-NT2RP2001562//Homo sapiens GLE1 (GLE1) mRNA, complete cds.//0//1899bp//98%//AF058922
C-NT2RP2001662//HOMEODOMAIN PROTEIN AKR (AVIAN KNOTTED-RELATED PROTEIN).//1.80E-49//94aa//81%//Q90655
C-NT2RP2001755//Rattus norvegicus f-spondin mRNA, complete cds.//0//2974bp//86%//M88469
C-NT2RP2001769//SERINE/THREONINE-PROTEIN KINASE ORB6 (EC 2.7.1.-).//9.10E-47//185aa//44%//O13310
C-NT2RP2001817//Homo sapiens sirtuin type 1 (SIRT1) mRNA, complete cds.//0//3092bp//99%//AF083106
C-NT2RP2001878
C-NT2RP2001903//CALPAIN, LARGE [CATALYTIC] SUBUNIT (EC 3.4.22.17) (CALCIUM- ACTIVATED NEUTRAL PROTEINASE) (CANP) (MU/M-TYPE).//3.80E-58//475aa//34%//P00789
C-NT2RP2001915
C-NT2RP2001921
C-NT2RP2001948//HST2 PROTEIN (HOMOLOGOUS TO SIR2 PROTEIN 2).//1.40E-08//191aa//27%//P53686
C-NT2RP2001956//ORM1 PROTEIN.//3.90E-19//137aa//37%//P53224
C-NT2RP2002015
C-NT2RP2002063//GNS1 PROTEIN.//3.60E-18//231aa//33%//P25358
C-NT2RP2002188//Rattus norvegicus neuroligin 3 mRNA, complete cds.//2.50E-226//1284bp//89%//U41663
C-NT2RP2002232//HYPOTHETICAL 85.7 KD PROTEIN C13G6.03 IN CHROMOSOME I.//1.90E-93//420aa//43%//Q09782
C-NT2RP2002304//Homo sapiens histone acetyltransferase MORF mRNA, complete cds.//0//2737bp//99%//AF113514
C-NT2RP2002409
C-NT2RP2002510
C-NT2RP2002527//CYTOCHROME B5.//1.30E-11//92aa//38%//P40312
C-NT2RP2002533//Homo sapiens alpha 2 delta calcium channel subunit isoform I mRNA, complete cds.//0//2365bp//99%//AF042792
C-NT2RP2002564//Human zinc-finger protein C2H2-150 mRNA, complete cds.//0//2237bp//99%//U38864
C-NT2RP2002674//SOLUBLE EPOXIDE HYDROLASE (SEH) (EC 3.3.2.3) (EPOXIDE HYDRATASE) (CYTOSOLIC EPOXIDE HYDROLASE) (CEH).//5.50E-38//201aa//39%//P34913
C-NT2RP2002721//REGULATORY PROTEIN UHPC.//1.60E-23//153aa//30%//P27669
C-NT2RP2002824//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//3.50E-63//404aa//33%//P32802
C-NT2RP2002942//Homo sapiens mRNA for KIAA0806 protein, complete cds.//0//2090bp//99%//AB018349
C-NT2RP2002974//HOMEOBOX PROTEIN SIXS (DM LOCUS-ASSOCIATED HOMEODOMAIN PROTEIN HOMOLOG) (FRAGMENT).//8.20E-241//555aa//84%//P70178
C-NT2RP2002976//HYPOTHETICAL 149.7 KD PROTEIN IN IRE1-KSP1 INTERGENIC REGION.//1.30E-20//99aa//47%//P38800
C-NT2RP2003042//PHOSPHATIDYLCHOLINE-STEROL ACYLTRANSFERASE PRECURSOR (EC 2.3.1.43) (LECITHIN-CHOLESTEROL ACYLTRANSFERASE) (PHOSPHOLIPID-CHOLESTEROL ACYLTRANSFERASE) (FRAGMENT).//2.10E-109//385aa//52%//P53760
C-NT2RP2003138//5'-TG-3' INTERACTING FACTOR (HOMEOBOX PROTEIN TGIF).//2.10E-08//104aa//46%//P70284
C-NT2RP2003179//PUTATIVE SERINE/THREONINE-PROTEIN KINASE EMK (EC 2.7.1.-).//2.60E-67//256aa//49%//Q05512
C-NT2RP2003210//Homo sapiens fatty acid transport protein (FATP) mRNA, complete cds.//9.80E-272//1265bp//98%//AF055899
C-NT2RP2003302//Homo sapiens GIOT-4 mRNA for gonadotropin inducible transcription repressor-4, complete cds.//0//2891bp//99%//AB021644
C-NT2RP2003369//RAS SUPPRESSOR PROTEIN 1 (RSU-1) (RSP-1 PROTEIN) (RSP-1 ).//5.90E-20//204aa//34%//Q15404
C-NT2RP2003383//Homo sapiens mRNA for KIAA0458 protein, complete cds.//0//2565bp//99%//AB007927
C-NT2RP2003390//Homo sapiens mRNA for SEC63 protein.//0//2629bp//99%//AJ011779
C-NT2RP2003469//GALACTOSE-PROTON SYMPORT (GALACTOSE TRANSPORTER).//1.10E-45//324aa//29%//P37021
C-NT2RP2003545//Homo sapiens STE20-like kinase 3 (mst-3) mRNA, complete cds.//5.40E-48//578bp//71%//AF024636
C-NT2RP2003593
C-NT2RP2003599
C-NT2RP2003655//HYPOTHETICAL 26.3 KD PROTEIN IN OYE2-GND1 INTERGENIC//4.80E-15//93aa//47%//P38869
C-NT2RP2003664//Homo sapiens mRNA for leptin receptor gene-related protein.//1.90E-237//1081bp//99%//Y12670
C-NT2RP2003931
C-NT2RP2003940//ZINC FINGER PROTEIN 43 (ZINC PROTEIN HTF6).//7.00E-111//401aa//43%//P28160
C-NT2RP2003950//Homo sapiens clone 24778 unknown mRNA.//0//1545bp//99%//AF070572
C-NT2RP2004069
C-NT2RP2004108//ZINC FINGER PROTEIN ZFP-36 (FRAGMENT).//3.30E-171//474aa//62%//P16415
C-NT2RP2004141
C-NT2RP2004179
C-NT2RP2004205//BUTYROPHILIN PRECURSOR (BT).//1.60E-21//276aa//32%//Q62556
C-NT2RP2004447
C-NT2RP2004495//Human transporter protein (g17) mRNA, complete cds.//9.80E-64//642bp//64%//L149082
C-NT2RP2004524
C-NT2RP2004556
C-NT2RP2004606//Human fibroblast collagenase inhibitor mRNA, complete cds.//2.10E-166//768bp//99%//M12670
C-NT2RP2004648//Mouse beta-galactosidase (BGAL) gene, complete cds.//1.20E-33//1136bp//59%//M57734
C-NT2RP2004670//Rattus norvegicus vesicla-associate calmodulin-binding protein mRNA, complete cds.//0//1250bp//86%//L22557
C-NT2RP2004794//HYPOTHETICAL 24.5 KD PROTEIN IN SAP185-BCK1 INTERGENIC REGION.//2.70E-09//203aa//26%//P40857
C-NT2RP2004837
C-NT2RP2004847//ZINC FINGER PROTEIN 135.//8.00E-35//193aa//40%//P52742
C-NT2RP2005027//GLUCOSE TRANSPORTER TYPE 3, BRAIN.//6.20E-67//130aa//100%//P11169
C-NT2RP2005069//Rat vacuolar protein sorting homolog r-vps33b mRNA, complete cds.//0//1792bp//87%//U35245
C-NT2RP2005163
C-NT2RP2005181//Mus musculus cationic amino acid transporter (CAT3) mRNA, complete cds.//5.30E-315//2126bp//81%//U70859
C-NT2RP2005247//ZINC-FINGER PROTEIN RFP (RET FINGER PROTEIN).//5.00E-53//296aa//37%//Q62158
C-NT2RP2005378//GLYCINE-RICH CELL WALL STRUCTURAL PROTEIN 1.8 PRECURSOR (GRP 1.8).//6.30E-28//183aa//47%//P10496
C-NT2RP2005391//Homo sapiens partial mRNA for putative p621 protein which interacts with transcription factor Sp1.//0//1544bp//99%//AJ242978
C-NT2RP2005425//Homo sapiens mRNA for AKAP450 protein.//0//4341bp//99%//AJ131693
C-NT2RP2005463//PROTEIN PTM1 PRECURSOR.//7.40E-15//284aa//28%//P32857
C-NT2RP2005514
C-NT2RP2005535//ZINC FINGER PROTEIN 85 (ZINC FINGER PROTEIN HPF4) (HTF1).//1.90E-172//489aa//62%//Q03923
C-NT2RP2005541//N-ACETYLGLUCOSAMINE-6-SULFATASE PRECURSOR (EC 3.1.6.14) (G6S) (GLUCOSAMINE-6-SULFATASE).//4.70E-24//78aa//51%//P15586
C-NT2RP2005597//Homo sapiens protein O-mannosyl-transferase 1 (POMT1) mRNA, complete cds.//0//1821bp//97%//AF095136
C-NT2RP2005632
C-NT2RP2005666
C-NT2RP2005774//Homo sapiens GIOT-4 mRNA for gonadotropin inducible transcription repressor-4, complete cds.//6.90E-224//1461bp//72%//AB021644
C-NT2RP2005878//PUTATIVE STEROID DEHYDROGENASE KIK-I (EC 1.1.1.-).//3.60E-55//238aa//50%//O57314
C-NT2RP2005883//DOPAMINE-BETA-MONOOXYGENASE PRECURSOR (EC 1.14.17.1) (DOPAMINE BETA- HYDROXYLASE) (DBH).//6.70E-72//512aa//34%//P15101
C-NT2RP2005887
C-NT2RP2005941//Human paired box gene (PAX6) homologue, complete cds.//1.40E-308//1396bp//99%//M93650
C-NT2RP2005994//HYPOTHETICAL 51.5 KD PROTEIN D2024.3 IN CHROMOSOME IV.//3.50E-35//144aa//47%//P49191
C-NT2RP2006004//FIBROMODULIN PRECURSOR (FM) (COLLAGEN-BINDING 59 KD PROTEIN).//3.00E-26//227aa//36%//Q06828
C-NT2RP2006042//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//9.40E-15//501aa//25%//P08640
C-NT2RP2006092//Homo sapiens mRNA for Fe65L2, complete cds.//0//1156bp//99%//AB018247
C-NT2RP2006099
C-NT2RP2006134
C-NT2RP2006269//DOLICHYL-PHOSPHATE-MANNOSE--PROTEIN MANNOSYLTRANSFERASE 2 (EC 2.4.1.109).//2.30E-78//679aa//32%//P31382
C-NT2RP2006512//GNS1 PROTEIN.//2.00E-21//290aa//29%//P25358
C-NT2RP2006580//Homo sapiens transitional epithelia response protein (TERE1) mRNA, complete cds.//0//1483bp//99%//AF117064
C-NT2RP3000011//VEGETATIBLE INCOMPATIBILITY PROTEIN HET-E-1.//4.00E-14//320aa//24%//Q00808
C-NT2RP3000022//Rat heart mRNA serine/threonine protein kinase, complete cds.//4.80E-203//1496bp//78%//D26178
C-NT2RP3000059//ANKYRIN, BRAIN VARIANT 2 (ANKYRIN B) (ANKYRIN, NONERYTHROID) (FRAGMENT).//3.70E-12//133aa//32%//Q01485
C-NT2RP3000063//NEUROFILAMENT TRIPLET H PROTEIN (200 KD NEUROFILAMENT PROTEIN) (NF-H).//5.00E-29//596aa//30%//P19246
C-NT2RP3000125//RETINOBLASTOMA BINDING PROTEIN 2 (RBBP-2).//6.30E-08//70aa//41%//P29375
C-NT2RP3000148//ZINC FINGER PROTEIN 84 (ZINC FINGER PROTEIN HPF2).//4.10E-106//350aa//47%//P51523
C-NT2RP3000169//Homo sapiens MRS1 mRNA, complete cds.//0//1519bp//97%//AF093239
C-NT2RP3000171//Mus musculus partial mRNA for B-IND1 protein (B-ind1 gene).//4.40E-99//571bp//89%//Z97207
C-NT2RP3000172//CALCIUM/CALMODULIN-DEPENDENT PROTEIN KINASE TYPE I (EC 2.7.1.123) (CAM KINASE I).//1.30E-80//359aa//44%//Q14012
C-NT2RP3000201//Homo sapiens HPK/GCK-like kinase HGK mRNA, complete cds.//1.30E-270//1231bp//99%//AF096300
C-NT2RP3000232//ZINC FINGER PROTEIN 184 (FRAGMENT).//1.40E-130//693aa//41%//Q99676
C-NT2RP3000304//Homo sapiens LDL receptor-related protein 6 (LRP6) mRNA, complete cds.//0//2895bp//99%//AF074264
C-NT2RP3000378//PAIRED AMPHIPATHIC HELIX PROTEIN.//4.20E-39//186aa//36%//P22579
C-NT2RP3000427
C-NT2RP3000436//PROBABLE PROTEIN DISULFIDE ISOMERASE P5 PRECURSOR (EC 5.3.4.1).//6.20E-27//90aa//42%//P38660
C-NT2RP3000444
C-NT2RP3000460//Canis familiaris sec61 homologue mRNA, complete cds.//1.80E-198//643bp//89%//M96629
C-NT2RP3000481//Homo sapiens RanBP7/importin 7 mRNA, complete cds.//0//2623bp//100%//AF098799
C-NT2RP3000616//FIBROMODULIN PRECURSOR (FM) (COLLAGEN-BINDING 59 KD PROTEIN).//5.20E-26//227aa//36%//Q06828
C-NT2RP3000645
C-NT2RP3000652//ZINC FINGER PROTEIN 43 (ZINC PROTEIN HTF6).//1.90E-153//441aa//62%//P28160
C-NT2RP3000676//PUTATIVE MITOCHONDRIAL CARRIER YMR166C.//2.10E-15//220aa//27%//Q03829
C-NT2RP3000677//DNA BINDING PROTEIN RFX2.//3.60E-56//233aa//41%//P48378
C-NT2RP3000721//HYPOTHETICAL 149.7 KD PROTEIN IN IRE1-KSP1 INTERGENIC REGION.//1.10E-22//171aa//36%//P38800
C-NT2RP3000789//Homo sapiens IGF-II mRNA-binding protein 1 (IMP-1) mRNA, complete cds.//0//1458bp//100%//AF117106
C-NT2RP3000818
C-NT2RP3000820//PUTATIVE SERINE/THREONINE-PROTEIN KINASE PKWA (EC 2.7.1.-).//1.90E-30//269aa//33%//P49695
C-NT2RP3000838//TRICHOHYALIN.//9.80E-11//491aa//26%//Q07283
C-NT2RP3000871
C-NT2RP3000907//PROBABLE CALCIUM-TRANSPORTING ATPASE 6 (EC 3.6.1.38).//2.20E-134//296aa//42%//P39986
C-NT2RP3000921//TUMOR SUPPRESSOR PROTEIN DCC PRECURSOR (COLORECTAL CANCER SUPPRESSOR).//4.00E-21//316aa//29%//P43146
C-NT2RP3001012//Homo sapiens mRNA for KIAA0829 protein, partial cds.//0//2906bp//98%//AB020636
C-NT2RP3001044
C-NT2RP3001061//Homo sapiens mRNA for KIAA0853 protein, partial cds.//0//3591bp//99%//AB020660
C-NT2RP3001159//Homo sapiens mRNA; cDNA DKFZp586C201 (from clone DKFZp586C201).//0//2558bp//99%//AL050118
C-NT2RP3001170//Mus musculus activity-dependent neuroprotective protein (Adnp) mRNA, complete cds.//4.80E-240//850bp//88%//AF068198
C-NT2RP3001195//GALACTOSE-PROTON SYMPORT (GALACTOSE TRANSPORTER).//4.70E-48//339aa//29%//P37021
C-NT2RP3001240//Canis familiaris sec61 homologue mRNA, complete cds.//1.20E-301//1141bp//89%//M96629
C-NT2RP3001271//NON-GREEN PLASTID TRIOSE PHOSPHATE TRANSLOCATOR PRECURSOR (CTPT).//2.60E-09//34aa//22%//P52178
C-NT2RP3001322//PROBABLE CALCIUM-TRANSPORTING ATPASE 3 (EC 3.6.1.38) (ENDOPLASMIC RETICULUM CA2+-ATPASE).//1.70E-21//220aa//30%//P39524
C-NT2RP3001388//SYNAPTOTAGMIN IV.//2.00E-118//430aa//54%//P50232
C-NT2RP3001542//TUMOR NECROSIS FACTOR, ALPHA-INDUCED PROTEIN 1, ENDOTHELIAL (B12 PROTEIN).//4.70E-11//132aa//37%//Q13829
C-NT2RP3001560//Homo sapiens cleft lip and palate transmembrane protein 1 (CLPTM1) mRNA, complete cds.//0//2468bp//99%//AF037339
C-NT2RP3001592//N2,N2-DIMETHYLGUANOSINE TRNA METHYLTRANSFERASE PRECURSOR (EC 2.1.1.32).//1.30E-18//279aa//27%//P15565
C-NT2RP3001650//BONE MORPHOGENETIC PROTEIN 1 PRECURSOR (EC 3.4.24.-) (BMP-1).//2.20E-22//107aa//42%//P98063
C-NT2RP3001685//PRPE PROTEIN.//1.00E-68//382aa//41%//P77495
C-NT2RP3001738//CYTOCHROME B5.//1.30E-11//133aa//33%//P00169
C-NT2RP3001754
C-NT2RP3001858
C-NT2RP3001976//ZINC FINGER PROTEIN 84 (ZINC FINGER PROTEIN HPF2).//8.90E-143//379aa//55%//P51523
C-NT2RP3002015//Homo sapiens CGI-71 protein mRNA, complete cds.//0//1991bp//99%//AF151829
C-NT2RP3002160//HISTO-BLOOD GROUP ABO SYSTEM TRANSFERASE (NAGAT) [INCLUDES: GLYCOPROTEIN-FUCOSYLGALACTOSIDE ALPHA-N-ACETYLGALACTOSAMINYLTRANSFERASE (EC 2.4.1.40) (FUCOSYLGLYCOPROTEIN ALPHA-N-ACETYLGALACTOSAMINYLTRANSFERASE) (HISTO-BLOOD GROUP A TRANSFERASE) (A TRANSFERASE); GLYCOPROTEIN-FUCOSYLGALACTOSIDE ALPHA- GALACTOSYLTRANSFERASE (EC 2.4.1.37) (FUCOSYLGLYCOPROTEIN 3-ALPHA- GALACTOSYLTRANSFERASE) (HISTO-BLOOD GROUP B TRANSFERASE) (B TRANSFERASE)].//3.50E-72//231aa//49%//P16442
C-NT2RP3002281//Homo sapiens mRNA for KIAA0765 protein, partial cds.//0//2286bp//99%//AB018308
C-NT2RP3002286//Homo sapiens candidate tumor suppressor protein DICE1 mRNA, complete cds.//0//2719bp//99%//AF097645
C-NT2RP3002311//BETA-GALACTOSIDASE PRECURSOR (EC 3.2.1.23) (LACTASE).//9.80E-103//547aa//43%//P48982
C-NT2RP3002324
C-NT2RP3002342//Human transporter protein (g17) mRNA, complete cds.//1.70E-65//641bp//65%//U49082
C-NT2RP3002353
C-NT2RP3002409//Homo sapiens mRNA for KIAA0719 protein, complete cds.//0//2404bp//99%//AB018262
C-NT2RP3002411//Homo sapiens steroid dehydrogenase homolog mRNA, complete cds.//0//2374bp//99%//AF078850
C-NT2RP3002448
C-NT2RP3002571//Bos taurus mRNA for lyncein.//7.30E-169//1115bp//84%//Y17923
C-NT2RP3002664
C-NT2RP3002721//CITRATE SYNTHASE, MITOCHONDRIAL PRECURSOR (EC 4.1.3.7).//5.80E-249//466aa//98%//O75390
C-NT2RP3002737//Homo sapiens voltage-gated potassium channel KCNQ4 (KCNQ4) mRNA, complete cds.//0//1552bp//99%//AF105202
C-NT2RP3002738//Homo sapiens enhancer of filamentation (HEF1) mRNA, complete cds.//2.20E-47//763bp//65%//L43821
C-NT2RP3002790
C-NT2RP3002836//Homo sapiens mRNA for KIAA0463 protein, partial cds.//0//1617bp//99%//AB007932
C-NT2RP3002887
C-NT2RP3002900//Homo sapiens CGI-109 protein mRNA, complete cds.//8.70E-298//1321bp//92%//AF151867
C-NT2RP3002958//TRANS-GOLGI NETWORK INTEGRAL MEMBRANE PROTEIN TGN38 PRECURSOR.//8.00E-08//197aa//26%//P19814
C-NT2RP3002983
C-NT2RP3003000//Homo sapiens T-type calcium channel alpha-1 subunit mRNA, complete cds.//0//3160bp//96%//AF051946
C-NT2RP3003076
C-NT2RP3003354//SECRETORY CARRIER-ASSOCIATED MEMBRANE PROTEIN 3.//5.10E-55//208aa//51%//O35609
C-NT2RP3003448
C-NT2RP3003469
C-NT2RP3003473//Homo sapiens CGI-55 protein mRNA, complete cds.//5.50E-275//1309bp//98%//AF151813
C-NT2RP3003527//Homo sapiens mRNA for protein kinase Dyrk1B.//0//2483bp//99%//Y17999
C-NT2RP3003532//OX-2 MEMBRANE GLYCOPROTEIN PRECURSOR (FRAGMENT).//4.40E-139//263aa//99%//P41217
C-NT2RP3003535//UDP-N-ACETYLGLUCOSAMINE--PEPTIDE N-ACETYLGLUCOSAMINYLTRANSFERASE 110 KD SUBUNIT (EC 2.4.1.-) (O-GLCNAC TRANSFERASE P110 SUBUNIT).//8.80E-18//368aa//25%//P56558
C-NT2RP3003559
C-NT2RP3003614
C-NT2RP3003729//HYPOTHETICAL 42.1 KD PROTEIN IN SNZ1-YPK2 INTERGENIC REGION.//5.80E-17//204aa//30%//Q03151
C-NT2RP3003849//PROTEIN KINASE C, BRAIN ISOZYME (EC 2.7.1.-) (PKC) (DPKC53E(BR)). PKC1.//1.20E-13//126aa//34%//P05130
C-NT2RP3003874//Homo sapiens incomplete cDNA for a mutated allele of a myosin class I, myh-1c.//0//2160bp//98%//AJ001381
C-NT2RP3003963
C-NT2RP3004000
C-NT2RP3004025
C-NT2RP3004067//Homo sapiens mRNA for NESCA, complete cds.//0//1962bp//99%//AB026894
C-NT2RP3004075
C-NT2RP3004083
C-NT2RP3004090//GOLIATH PROTEIN (G1 PROTEIN).//9.00E-33//179aa//47%//Q06003
C-NT2RP3004119//PEREGRNN (BR140 PROTEIN).//7.30E-39//227aa//43%//P55201
C-NT2RP3004130//CELL SURFACE ANTIGEN 114/A10 PRECURSOR.//8.10E-06//71aa//42%//P19467
C-NT2RP3004133//GLYCOSYLTRANSFERASE ALG2 (EC 2.4.1.-).//2.50E-48//198aa//37%//P43636
C-NT2RP3004202
C-NT2RP3004294//Xenopus laevis ER1 mRNA, complete cds.//1.20E-71//335bp//79%//AF015454
C-NT2RP3004309//Homo sapiens mRNA; cDNA DKFZp586C201 (from clone DKFZp586C201).//0//2584bp//99%//AL050118
C-NT2RP3004321
C-NT2RP3004345//N2,N2-DIMETHYLGUANOSINE TRNA METHYLTRANSFERASE PRECURSOR (EC 2.1.1.32).//3.90E-18//279aa//27%//P15565
C-NT2RP3004355
C-NT2RP3004374
C-NT2RP3004406//HYPOTHETICAL 37.4 KD PROTEIN IN IRR1-TIM44 INTERGENIC REGION.//3.20E-15//165aa//33%//P40544
C-NT2RP3004481//BUTYROPHILIN PRECURSOR (BT).//8.50E-22//276aa//32%//Q62556
C-NT2RP3004552//COMPLEMENT RECEPTOR TYPE 1 PRECURSOR (C3B/C4B RECEPTOR) (CD35 ANTIGEN).//8.50E-24//263aa//33%//P17927
C-NT2RP3004557//Human Ki nuclear autoantigen mRNA, complete cds.//0//2181bp//96%//U11292
C-NT2RP3004625//Homo sapiens I-1 receptor candidate protein mRNA, complete cds.//0//1339bp//99%//AF082516
C-NT2RP3004640//Bos taurus tuftelin mRNA, complete cds.//0//1204bp//88%//AF105228
C-NT2RP3004647//PUTATIVE MITOCHONDRIAL CARRIER YMR166C.//2.00E-15//220aa//27%//Q03829
C-NT2RP4000108//Human gene for neurofilament subunit NF-L.//0//1998bp//99%//X05608
C-NT2RP4000634//Homo sapiens mitogen-activated protein kinase kinase kinase MEKK2 mRNA, complete cds.//0//1501bp//98%//AF111105
C-NT2RP4000962//SPORULATION-SPECIFIC PROTEIN 1 (EC 2.7.1.-).//2.60E-18//225aa//32%//P08458
C-NT2RP4001001//Homo sapiens clone 24856 mRNA sequence, complete cds.//3.90E-301//1374bp//99%//AF131856
C-NT2RP4001009//Homo sapiens mRNA for farnesylated-proteins converting enzyme 1.//0//2965bp//99%//Y13834
C-NT2RP4001467//Human placental cDNA coding for 5'nucleotidase (EC 3.1.3.5).//0//2140bp//99%//X55740
C-NT2RP4001877//Homo sapiens ribonucleoprotein RBM8 (RBM8) mRNA, complete cds.//0//2770bp//99%//AF127761
C-NT2RP4001879
C-NT2RP4002187//Homo sapiens steroid dehydrogenase homolog mRNA, complete cds.//0//2373bp//99%//AF078850
C-NT2RP4002451
C-NT2RP4002715
C-NT2RP4002750//Mus musculus cationic amino acid transporter (CAT3) mRNA, complete cds.//1.00E-310//2084bp//81%//U70859
C-OVARC1000003//Homo sapiens sodium dependent phosphate transporter isoform NaPi-3b mRNA, complete cds.//4.30E-220//1158bp//94%//AF111856
C-OVARC1000090
C-OVARC1000105//UBIQUITIN-CONJUGATING ENZYME E2-28.4 KD (EC 6.3.2.19) (UBIQUITIN- PROTEIN LIGASE) (UBIQUITIN CARRIER PROTEIN).//4.20E-47//171aa//56%//P33296
C-OVARC1000137
C-OVARC1000208//Human calcium-dependent group X phospholipase A2 mRNA, complete cds//1.50E-61//365bp//90%//U95301
C-OVARC1000255//H.sapiens syk mRNA for protein-tyrosine kinase.//0//1525bp//97%//Z29630
C-OVARC1000275//DESMOPLAKIN I AND II (DPI AND DPII) (FRAGMENT).//9.90E-16//352aa//23%//P15924
C-OVARC1000298
C-OVARC1000307//HYPOTHETICAL 29.7 KD PROTEIN CY339.02.//3.00E-19//194aa//35%//Q50658
C-OVARC1000313//PROTEIN DISULFIDE ISOMERASE PRECURSOR (PDI) (EC 5.3.4.1).//3.00E-24//353aa//27%//Q12730
C-OVARC1000331//GMP REDUCTASE (EC 1.6.6.8) (GUANOSINE 5'-MONOPHOSPHATE OXIDOREDUCTASE).//9.40E-44//106aa//59%//P36959
C-OVARC1000410//Homo sapiens angiopoietin Y1 mRNA, complete cds.//2.10E-63//744bp//69%//AF107253
C-OVARC1000439//HYPOTHETICAL 64.3 KD GTP-BINDING PROTEIN C02F5.3 IN CHROMOSOME III.//1.40E-33//143aa//53%//P34280
C-OVARC1000467
C-OVARC1000529//PROBABLE SERINE/THREONINE-PROTEIN KINASE YKL101W (EC 2.7.1.-).//1.40E-23//165aa//39%//P34244
C-OVARC1000553//DIPEPTIDYL PEPTIDASE IV (EC 3.4.14.5) (DPP IV) (THYMOCYTE-ACTIVATING MOLECULE) (THAM).//1.30E-23//169aa//40%//P28843
C-OVARC1000775
C-OVARC1000811//PLASMINOGEN (EC 3.4.21.7) (FRAGMENT).//6.40E-13//115aa//34%//Q01177
C-OVARC1000853
C-OVARC1000873//N-ACETYLGLUCOSAMINE-6-SULFATASE PRECURSOR (EC 3.1.6.14) (G6S) (GLUCOSAMINE-6-SULFATASE).//1.00E-09//83aa//40%//P50426
C-OVARC1000916//H.sapiens PISSLRE mRNA.//7.30E-280//1117bp//95%//X78342
C-OVARC1000956//SKIN SECRETORY PROTEIN XP2 PRECURSOR (APEG
PROTEIN).//2.20E-09//250aa//28%//P17437
C-OVARC1000995
C-OVARC1001030//Homo sapiens mRNA for KIAA0886 protein, complete cds.//0//907bp//99%//AB020693
C-OVARC1001049//TRANSCRIPTION FACTOR HES-1 (C-HAIRY1).//7.50E-14//96aa//36%//O57337
C-OVARC1001086//Homo sapiens cyclin T2a mRNA, complete cds.//0//1593bp//98%//AF048731
C-OVARC1001132//GC-RICH SEQUENCE DNA-BINDING FACTOR (GCF) (TRANSCRIPTION FACTOR 9)(TCF-9).//2.30E-44//268aa//36%//P16383
C-OVARC1001163//HYPOTHETICAL 49.3 KD PROTEIN C30D11.06C IN CHROMOSOME I.//2.30E-20//152aa//30%//Q09906
C-OVARC1001222
C-OVARC1001260
C-OVARC1001336//Homo sapiens sodium dependent phosphate transporter isoform NaPi-3b mRNA, complete cds.//0//1435bp//99%//AF111856
C-OVARC1001338//AUTOPHAGY SERINE/THREONINE-PROTEIN KINASE APG1 (EC 2.7.1 .-).//8.80E-30//125aa//40%//P53104
C-OVARC1001569//PROBABLE SERINE/THREONINE-PROTEIN KINASE YKL101W (EC 2.7.1.-).//1.50E-22//164aa//39%//P34244
C-OVARC1001570//Homo sapiens beta-site APP cleaving enzyme (BACE) mRNA, complete cds.//0//1792bp//100%//AF190725
C-OVARC1001596//Homo sapiens Arf-like 2 binding protein BART1 mRNA, complete cds.//0//1766bp//99%//AF126062
C-OVARC1001607//Human beta-1,2-N-acetylglucosaminyltransferase II (MGAT2) gene, complete cds.//0//1836bp//96%//U15128
C-OVARC1001725//Homo sapiens patched related protein TRC8 (TRC8) gene, exon 1 and partial cds.//0//1624bp//99%//AF064800
C-OVARC1001727
C-OVARC1001807//Human TR3 orphan receptor mRNA, complete cds.//1.10E-243//1145bp//98%//L13740
C-OVARC1001833//CIS-GOLGI MATRIX PROTEIN GM130.//6.60E-136//363aa//76%//Q62839
C-OVARC1001952//TRICHOHYALIN.//3.30E-16//487aa//27%//Q07283
C-OVARC1001991//VOLTAGE-GATED POTASSIUM CHANNEL PROTEIN KV3.3 (KSHIIID).//8.30E-06//114aa//35%//Q01956
C-OVARC1002058//Human 18S rRNA gene, complete.//1.50E-164//921bp//91%//M10098
C-OVARC1002178
C-PLACE1000033//VON WILLEBRAND FACTOR PRECURSOR.//3.80E-17//190aa//28%//Q28295
C-PLACE1000231//Rattus norvegicus WAP four-disulfide core domain protein (ps20) mRNA, complete cds.//2.70E-101//947bp//74%//AF037272
C-PLACE1000258//ZINC FINGER. PROTEIN 91 (ZINC FINGER PROTEIN HTF10) (HPF7).//1.70E-55//431aa//35%//Q05481
C-PLACE1000442//ZINC FINGER PROTEIN ZFP-36 (FRAGMENT).//1.00E-88//213aa//67%//P16415
C-PLACE1000560
C-PLACE1000740//Mus musculus (Notch2) mRNA, complete cds.//5.60E-122//893bp//81%//M93661
C-PLACE1000907//ZINC FINGER PROTEIN 83 (ZINC FINGER PROTEIN HPF1).//1.30E-204//396aa//85%//P51522
C-PLACE1000912
C-PLACE1000914//Homo sapiens PB39 mRNA, complete cds.//7.50E-88//500bp//69%//AF045584
C-PLACE1000927//HYPOTHETICAL 20.9 KD PROTEIN B0563.3 IN CHROMOSOME X.//6.30E-21//123aa//37%//Q11079
C-PLACE1000986//Homo sapiens mRNA; cDNA DKFZp586C1817 (from clone DKFZp586C1817).//0//2055bp//99%//AL117450
C-PLACE1001016//SODIUM CHANNEL PROTEIN PARA (PARALYTIC PROTEIN).//6.80E-12//133aa//28%//P35500
C-PLACE1001100//Homo sapiens nephrin (NPHS1) mRNA, complete cds//3.10E-46//323bp//84%//AF035835
C-PLACE1001114//HYPOTHETICAL BHLF1 PROTEIN.//9.20E-06//389aa//31%//P03181
C-PLACE1001123//INTESTINAL MEMBRANE A4 PROTEIN (DIFFERENTIATION-DEPENDENT PROTEIN A4).//5.00E-08//95aa//31%//Q04941
C-PLACE1001183
C-PLACE1001229
C-PLACE1001231//Rattus norvegicus sodium-dependent multi-vitamin transporter (SMVT) mRNA, complete cds.//2.20E-137//918bp//80%//AF026554
C-PLACE1001340//MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).//1.20E-23//231aa//31%//P23231
C-PLACE1001401//HIGH AFFINITY IMMUNOGLOBULIN EPSILON RECEPTOR BETA-SUBUNIT (FCERI) (IGE FC RECEPTOR, BETA-SUBUNIT).//3.70E-18//148aa//39%//P13386
C-PLACE1001407
C-PLACE1001464//Human placental cDNA coding for 5'nucleotidase (EC 3.1.3.5).//0//2756bp//99%//X55740
C-PLACE1001500//Homo sapiens RecQ5 mRNA for DNA helicase, complete cds.//2.30E-271//1230bp//99%//AB006533
C-PLACE1001516//240 KD PROTEIN OF ROD PHOTORECEPTOR CNG-CHANNEL [CONTAINS: GLUTAMIC ACID-RICH PROTEIN (GARP); CYCLIC-NUCLEOTIDE-GATED CATION CHANNEL 4 (CNG CHANNEL 4) (CNG-4) (CYCLIC NUCLEOTIDE-GATED CATION CHANNEL MODULATORY SUBUNIT)].//2.30E-08//274aa//28%//Q28181
C-PLACE1001536
C-PLACE1001564//H.sapiens mRNA for HE6 Tm7 receptor.//5.10E-36//499bp//70%//X81892
C-PLACE1001655//Homo sapiens Shab-related delayed-rectifier K+ channel alpha subunit (KCNS3) mRNA, complete cds.//0//1708bp//99%//AF043472
C-PLACE1001788
C-PLACE1001795//HYPOTHETICAL 30.6 KD PROTEIN IN SCP160-SMC3 INTERGENIC REGION PRECURSOR.//3.40E-20//159aa//40%//P47032
C-PLACE1001836//ENV POLYPROTEIN PRECURSOR (COAT POLYPROTEIN) [CONTAINS: OUTER MEMBRANE PROTEIN GP70; TRANSMEMBRANE PROTEIN P20E].//5.00E-27//134aa//47%//P10269
C-PLACE1001918//ENDOSOMAL P24A PROTEIN PRECURSOR (70 KD ENDOMEMBRANE PROTEIN) (PHEROMONE ALPHA-FACTOR TRANSPORTER) (ACIDIC 24 KD LATE ENDOCYTIC INTERMEDIATE COMPONENT).//2.30E-53//339aa//33%//P32802
C-PLACE1001949//PROBABLE CALCIUM-TRANSPORTING ATPASE 9 (EC 3.6.1.38).//3.00E-75//315aa//44%//Q12697
C-PLACE1002080//Homo sapiens antigen NY-CO-9 (NY-CO-9) mRNA, partial cds.//0//1588bp//99%//AF039691
C-PLACE1002095
C-PLACE1002153//Homo sapiens TACC2 protein (TACC2) mRNA, partial cds.//0//1202bp//99%//AF095791
C-PLACE1002329//EPIDERMAL GROWTH FACTOR RECEPTOR KINASE SUBSTRATE EPS8.//6.50E-105//213aa//45%//Q08509
C-PLACE1002355//COMPLEMENT C4 PRECURSOR [CONTAINS: C4A ANAPHYLATOXIN].//4.20E-12//131aa//40%//P01029
C-PLACE1002374//Human mRNA for pro-cathepsin L (major excreted protein MEP).//1.30E-313//1363bp//97%//X12451
C-PLACE1002518//Homo sapiens multiple membrane spanning receptor TRC8 (TRC8) mRNA, complete cds.//2.50E-14//396bp//64%//AF064801
C-PLACE1002547//MITOCHONDRIAL PRECURSOR PROTEINS IMPORT RECEPTOR (72 KD MITOCHONDRIAL OUTER MEMBRANE PROTEIN) (MITOCHONDRIAL IMPORT RECEPTOR FOR THE ADP/ATP CARRIER) (TRANSLOCASE OF OUTER MEMBRANE TOM70).//2.30E-28//277aa//31%//P23231
C-PLACE1002726//Oryctolagus cuniculus mRNA for epithelial calcium channel (ECaC).//2.80E-202//926bp//82%//AJ133128
C-PLACE1002905//ENDOZEPINE-RELATED PROTEIN PRECURSOR (MEMBRANE-ASSOCIATED DIAZEPAM BINDING INHIBITOR) (MA-DBI).//2.40E-37//188aa//40%//P07106
C-PLACE1002911//POLIOVIRUS RECEPTOR HOMOLOG PRECURSOR.//4.50E-39//345aa//32%//P32507
C-PLACE1002967//HIGH AFFINITY IMMUNOGLOBULIN EPSILON RECEPTOR BETA-SUBUNIT (FCERI) (IGE FC RECEPTOR, BETA-SUBUNIT).//4.60E-08//156aa//30%//Q01362
C-PLACE1003135//SPORULATION-SPECIFIC PROTEIN 1 (EC 2.7.1.-).//1.30E-47//210aa//49%//P08458
C-PLACE1003163//Homo sapiens DBI-related protein mRNA, complete cds.//1.00E-294//1344bp//99%//AF069301
C-PLACE1003407//Homo sapiens putative transmembrane protein (CLN5) mRNA, complete cds.//0//1965bp//99%//AF068227
C-PLACE1003428//Homo sapiens mRNA for VNN1 protein.//1.80E-142//676bp//72%//AJ132099
C-PLACE1003438
C-PLACE1003460
C-PLACE1003529//DNA-DIRECTED RNA POLYMERASE II LARGEST SUBUNIT (EC 2.7.7.6) (RPB1) (FRAGMENT).//1.30E-09//281aa//22%//P11414
C-PLACE1003573//T-CELL SURFACE GLYCOPROTEIN YE1/48 (T LYMPHOCYTE ANTIGEN A1) (LY49-A ANTIGEN).//3.70E-16//226aa//26%//P20937
C-PLACE1003598//TRP-ASP REPEATS CONTAINING PROTEIN RBA-1.//1.80E-07//161aa//27%//P90917
C-PLACE1003644
C-PLACE1003737//TOLL PROTEIN PRECURSOR.//5.40E-07//203aa//27%//P08953
C-PLACE1003772//EXTENSIN PRECURSOR (CELL WALL HYDROXYPROLINE-RICH GLYCOPROTEIN).//2.40E-12//124aa//38%//P13983
C-PLACE1003839//Homo sapiens putative G protein-coupled receptor (RAIG1) mRNA, complete cds.//8.10E-18//771bp//58%//AF095448
C-PLACE1003845//PUTATIVE UDP-GLUCOSE 4-EPIMERASE (EC 5.1.3.2) (GALACTOWALDENASE) (UDP- GALACTOSE 4-EPIMERASE).//3.40E-37//302aa//30%//Q57664
C-PLACE1003852//Homo sapiens mRNA for KIAA0758 protein, partial cds.//0//1667bp//99%//AB018301
C-PLACE1004028
C-PLACE1004078//Bovine mRNA for adseverin, complete cds.//0//2218bp//89%//D26549
C-PLACE1004166//CREB-BINDING PROTEIN.//1.80E-12//147aa//35%//P45481
C-PLACE1004168//GENERAL NEGATIVE REGULATOR OF TRANSCRIPTION SUBUNIT 1.//9.10E-62//485aa//32%//P25655
C-PLACE1004199
C-PLACE1004279//HYPOTHETICAL 59.1 KD PROTEIN ZK637.1 IN CHROMOSOME III.//1.40E-08//166aa//30%//P30638
C-PLACE1004282//MONO- AND DIACYLGLYCEROL LIPASE PRECURSOR (EC 3.1.1.-) (MDGL).//2.10E-11//189aa//30%//P25234
C-PLACE1004305//RAS-RELATED PROTEIN RAC1.//9.60E-29//197aa//41%//P40792
C-PLACE1004441//Human G protein-coupled receptor (GPR1) gene, complete cds.//0//1880bp//98%//U13666
C-PLACE1004450//AMINOPEPTIDASE N (EC 3.4.11.2) (MICROSOMAL AMINOPEPTIDASE) (LEUKEMIA ANTIGEN CD13).//1.30E-91//562aa//35%//P15541
C-PLACE1004482//Rattus norvegicus hematopoietic lineage switch 2 related protein (Hls2-rp) mRNA, complete cds.//1.90E-246//1643bp//83%//AF097723
C-PLACE1004492//VERPROLIN//3.30E-07//149aa//29%//P37370
C-PLACE1004519
C-PLACE1004520//Human pregnancy-specific beta-glycoprotein d mRNA, complete cds.//9.10E-279//882bp//88%//M20881
C-PLACE1004630//Homo sapiens ten integrin EGF-like repeat domains protein precursor (ITGBL1) mRNA, complete cds.//1.00E-138//643bp//99%//AF072752
C-PLACE1004637
C-PLACE1004648//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//1.40E-18//395aa//25%//P08640
C-PLACE1004816//Homo sapiens mRNA for Hakata antigen, complete cds.//1.00E-166//856bp//94%//D88587
C-PLACE1004887//GOLIATH PROTEIN (G1 PROTENN).//4.80E-33//179aa//47%//Q06003
C-PLACE1005003//PROSTASIN PRECURSOR (EC 3.4.21.-).//2.20E-52//269aa//41%//Q16651
C-PLACE1005005//Homo sapiens ubiquitin conjugating enzyme G2 (UBE2G2) mRNA, complete cds.//4.10E-261//1209bp//98%//AF032456
C-PLACE1005031//CHLORINE CHANNEL PROTEIN P64.//8.00E-92//205aa//87%//P35526
C-PLACE1005239//Homo sapiens mRNA for HIRIP3 protein (clone pH4-31, PH4-17).//1.80E-235//1010bp//84%//AJ2233511
C-PLACE1005250//Homo sapiens D-type cyclin-interacting protein 1 (DIP1) mRNA, complete cds.//0//1046bp//96%//AF082569
C-PLACE1005383//Homo sapiens UP50 mRNA, complete cds.//0//2019bp//99%//AF093118
C-PLACE1005410//Canis familiaris sec61 homologue mRNA, complete cds.//2.40E-204//673bp//89%//M96629
C-PLACE1005426//Human pregnancy-specific beta 1-glycoprotein 4 (PSG4) clone FL-64 mRNA, complete cds.//0//1629bp//95%//U18469
C-PLACE1005519//Homo sapiens STE20-like kinase 3 (mst-3) mRNA, complete cds.//4.60E-108//1070bp//73%//AF024636
C-PLACE1005539//ACTIN POLYMERIZATION INHIBITOR (HEAT SHOCK 25 KD PROTEIN) (25-KD IAP).//3.10E-08//84aa//34%//Q00649
C-PLACE1005544//Mus musculus junctional adhesion molecule (Jam) mRNA, complete cds.//2.00E-159//1237bp//76%//U89915
C-PLACE1005569//Homo sapiens mRNA for Z39Ig protein.//0//1768bp//99%//AJ132502
C-PLACE1005601
C-PLACE1005660//HYPOTHETICAL 64.3 KD GTP-BINDING PROTEIN C02F5.3 IN CHROMOSOME III.//1.90E-33//143aa//53%//P34280
C-PLACE1005669
C-PLACE1005682//B-CELL LYMPHOMA 3-ENCODED PROTEIN (BCL-3 PROTEIN).//4.90E-09//183aa//33%//P20749
C-PLACE1005725//HYPOTHETICAL 55.1 KD PROTEIN B0416.5 IN CHROMOSOME X.//7.60E-17//295aa//27%//Q11073
C-PLACE1005736//Human mRNA for BAS-GRIP protein.//0//2378bp//99%//E16311 C-PLACE1005745//ORM1 PROTEIN.//2.40E-17//137aa//35%//P53224
C-PLACE1005768
C-PLACE1005815//COLORECTAL MUTANT CANCER PROTEIN (MCC PROTEIN).//1.50E-26//274aa//26%//P23508
C-PLACE1005878//Bovine chlorine channel protein (p64) mRNA, complete cds.//5.90E-137//889bp//85%//L16547
C-PLACE1005927//HYPOTHETICAL 35.8 KD PROTEIN C12G12.12 IN CHROMOSOME I.//1.60E-38//333aa//33%//Q09875
C-PLACE1006071//LAMININ BETA-1 CHAIN PRECURSOR (LAMININ B1 CHAIN).//6.00E-08//215aa//26%//P02469
C-PLACE1006073//Homo sapiens mRNA for glucuronyltransferase I, complete cds.//4.10E-316//1020bp//96%//AB009598
C-PLACE1006079//Homo sapiens distal-less homeobox protein (DLX3) gene, complete cds.//0//1379bp//97%//AF028233
C-PLACE1006093
C-PLACE1006208//Homo sapiens nGAP mRNA, complete cds.//3.30E-151//694bp//100%//AF047711
C-PLACE1006219//PUTATIVE UDP-GLUCOSE 4-EPIMERASE (EC 5.1.3.2) (GALACTOWALDENASE) (UDP- GALACTOSE 4-EPIMERASE).//3.50E-37//302aa//30%//Q57664
C-PLACE1006277//Homo sapiens mRNA for Z39Ig protein.//0//1768bp//99%//AJ132502
C-PLACE1006290//GLYCOSYLTRANSFERASE ALG2 (EC 2.4.1.-).//8.50E-75//301aa//39%//P43636
C-PLACE1006443//Homo sapiens PB39 mRNA, complete cds.//4.30E-98//553bp//70%//AF045584
C-PLACE1006515//Homo sapiens mRNA for KIAA0576 protein, partial cds.//0//2846bp//99%//AB011148
C-PLACE1006716//30 KD ADIPOCYTE COMPLEMENT-RELATED PROTEIN PRECURSOR (ACRP30) (ADIPOCYTE SPECIFIC PROTEIN ADIPOQ).//4.60E-25//181aa//35%//Q60994
C-PLACE1006786
C-PLACE1006809//SLS1 PROTEIN PRECURSOR.//9.10E-10//273aa//27%//P08124
C-PLACE1006959
C-PLACE1007028//Homo sapiens TDAG51/Ip1 homologue 1 (TIH1) mRNA, complete cds.//1.40E-307//1423bp//99%//AF151100
C-PLACE1007040
C-PLACE1007077//Homo sapiens mRNA for KIAA0977 protein, complete cds.//0//2578bp//99%//AB023194
C-PLACE1007081//COAGULATION FACTOR V PRECURSOR (ACTIVATED PROTEIN C COFACTOR).//5.00E-20//247aa//34%//Q28107
C-PLACE1007096//PUTATIVE SUGAR TRANSPORT PROTEIN GUTA.//2.70E-17//174aa//27%//O34368
C-PLACE1007296//Human mRNA for a presumptive KDEL receptor.//1.10E-185//1038bp//91%//X55885
C-PLACE1007591
C-PLACE1007626//Homo sapiens unknown mRNA, complete cds.//3.00E-246//1122bp//99%//AF047439
C-PLACE1007702//Mus musculus TRA1 mRNA, complete cds.//7.50E-41//662bp//64%//D78335
C-PLACE1007845//GLYCOSYLTRANSFERASE ALG2 (EC 2.4.1.-).//4.80E-14//158aa//40%//P43636
C-PLACE1007881//HYPOTHETICAL 69.4 KD PROTEIN F13H10.3 IN CHROMOSOME IV.//3.10E-99//504aa//42%//Q19425
C-PLACE1007971
C-PLACE1008282//HEME-REGULATED EUKARYOTIC INITIATION FACTOR EIF-2-ALPHA KINASE (EC 2.7.1.-) (HRI).//7.10E-274//627aa//82%//P33279
C-PLACE1008297//MYOSIN HEAVY CHAIN KINASE B (EC 2.7.1.129) (MHCK B).//1.30E-14//187aa//33%//P90648
C-PLACE1008359//BEM46 PROTEIN (FRAGMENT).//1.70E-50//289aa//42%//P54069
C-PLACE1008469
C-PLACE1008549//Homo sapiens Ets transcription factor ESE-2b mRNA, complete cds.//0//2274bp//99%//AF115403
C-PLACE1008657//Bovine mRNA for adseverin, complete cds.//7.80E-227//1246bp//90%//D26549
C-PLACE1008716//Human beta-1,2-N-acetylglucosaminyltransferase II (MGAT2) gene, complete cds.//0//1888bp//99%//U15128
C-PLACE1008744//P-SELECTIN PRECURSOR (GRANULE MEMBRANE PROTEIN 140) (GMP-140) (PADGEM) (CD62P) (LEUKOCYTE-ENDOTHELIAL CELL ADHESION MOLECULE 3) (LECAM3).//4.80E-32//338aa//30%//Q01102
C-PLACE1008984
C-PLACE1008985//Mus musculus synaptotagmin VIII mRNA, partial cds.//3.80E-140//650bp//81%//U20107
C-PLACE1009067
C-PLACE1009196
C-PLACE1009279//cDNA encoding novel physiologically active protein which have serine protease activity.//6.60E-86//1414bp//64%//E12965
C-PLACE1009527//Oryctolagus cuniculus mRNA for epithelial calcium channel (ECaC).//1.20E-87//585bp//83%//AJ133128
C-PLACE1009546
C-PLACE1009600//Mouse mRNA for tetracycline transporter-like protein, complete cds.//1.10E-264//924bp//88%//D88315
C-PLACE1009735
C-PLACE1009982//SALIVARY GILUE PROTEIN SGS-3 PRECURSOR.//5.20E-08//166aa//28%//P02840
C-PLACE1010011//Homo sapiens AAPT1-like protein mRNA, partial cds.//1.70E-237//1092bp//99%//AF047431
C-PLACE1010078//ORM1 PROTEIN.//3.70E-19//137aa//37%//P53224
C-PLACE1010081//Homo sapiens serine/threonine kinase RICK (RICK) mRNA, complete cds.//0//2033bp//99%//AF027706
C-PLACE1010251//FIBRILLIN 2 PRECURSOR.//1.70E-31//201aa//35%//Q61555
C-PLACE1010445
C-PLACE1010713//Homo sapiens steroid dehydrogenase homolog mRNA, complete cds.//0//2374bp//99%//AF078850
C-PLACE1010784//Homo sapiens orphan G protein-coupled receptor (GPR34) gene, complete cds.//2.30E-252//1146bp//99%//AF039686
C-PLACE1010827//COP-COATED VESICLE MEMBRANE PROTEIN P24 PRECURSOR (FRAGMENT).//1.90E-19//163aa//34%//P49020
C-PLACE1010968//PROTEIN-TYROSINE PHOSPHATASE DLAR PRECURSOR (EC 3.1.3.48) (PROTEIN- TYROSINE-PHOSPHATE PHOSPHOHYDROLASE).//3.40E-30//690aa//26%//P16621
C-PLACE1011045//Homo sapiens E1-like protein mRNA, complete cds.//0//2376bp//99%//AF094516
C-PLACE1011116//GLUCOAMYLASE S1/S2 PRECURSOR (EC 3.2.1.3) (GLUCAN 1,4-ALPHA- GLUCOSIDASE) (1,4-ALPHA-D-GLUCAN GLUCOHYDROLASE).//3.30E-09//234aa//27%//P08640
C-PLACE1011181//MSP1 PROTEIN HOMOLOG.//9.40E-82//353aa//47%//P54815
C-PLACE1011236//Mus musculus mRNA for RST, complete cds.//1.70E-90//1398bp//65%//AB005451
C-PLACE1011364//MYOTONIN-PROTEIN KINASE (EC 2.7.1.-) (MYOTONIC DISTROPHY PROTEIN KINASE) (MDPK) (DM-KINASE) (DMK) (DMPK) (MT-PK).//2.20E-09//153aa//32%//Q09013
C-PLACE1011407//ZINC FINGER PROTEIN 184 (FRAGMENT).//1.80E-133//342aa//59%//Q99676
C-PLACE1011516//HYPOTHETICAL 21.5 KD PROTEIN IN SEC15-SAP4 INTERGENIC REGION.//1.30E-13//139aa//34%//P53073
C-PLACE1011708//Homo sapiens intrinsic factor-B12 receptor precursor, mRNA, complete cds.//0//1840bp//98%//AF034611
C-PLACE1011824//Human Ste20-like kinase (MST2) mRNA, complete cds.//6.40E-202//561bp//92%//U26424
C-PLACE1011978//ZINC FINGER PROTEIN 91 (ZINC FINGER PROTEIN HTF10) (HPF7).//1.10E-194//547aa//57%//Q05481
C-PLACE2000118//WISKOTT-ALDRICH SYNDROME PROTEIN HOMOLOG (WASP).//3.20E-27//205aa//43%//P70315
C-PLACE2000219
C-PLACE3000181//Human protocadherin 42 mRNA, complete cds for abbreviated PC42.//0//2719bp//95%//L11370
C-PLACE3000213//COMPLEMENT RECEPTOR TYPE 2 PRECURSOR (CR2) (COMPLEMENT C3D RECEPTOR).//4.60E-68//317aa//34%//P19070
C-PLACE4000354//COMPLEMENT RECEPTOR TYPE 1 PRECURSOR (C3B/C4B RECEPTOR) (CD35 ANTIGEN).//1.00E-129//482aa//29%//P17927
C-PLACE4000455
C-SKNMC1000004
C-SKNMC1000014
C-SKNMC1000082//BRITTLE-1 PROTEIN PRECURSOR.//3.70E-31//307aa/130%//P29518
C-THYRO1000036//Homo sapiens collagen alpha 3 type IX (COL9A3) mRNA, complete cds.//1.20E-258//1376bp//93%//L41162
C-THYRO1000061//Mus musculus mRNA for UBE-1c1, UBE-1c2, UBE-1c3, complete cds.//1.40E-117//1126bp//74%//AB030505
C-THYRO1000099
C-THYRO1000196//Homo sapiens Ksp-cadherin (CDH16) mRNA, complete cds.//0//1632bp//91%//AF016272
C-THYRO1000400//Homo sapiens mRNA full length insert cDNA clone EUROIMAGE 25795.//0//1893bp//99%//AL109665
C-THYRO1000580//ZINC FINGER PROTEIN 184 (FRAGMENT).//9.90E-114//279aa//59%//Q99676
C-THYRO1000584//EPIDIDYMIS-SPECIFIC ALPHA-MANNOSIDASE (EC 3.2.1.24) (ALPHA-D-MANNOSIDE MANNOHYDROLASE) (135 KD PROTEIN).//5.40E-127//335aa//71%//Q28949
C-THYRO1000678//Homo sapiens Cx30 gene.//0//1741bp//97%//AJ005585
C-THYRO1000776//PROBABLE SULFATE PERMEASE SPBC3H7.02.//1.30E-68//442aa//36%//O74377
C-THYRO1000795//C.elegans mRNA for Oxoglutarate/malate carrier protein.//8.80E-42//821bp//63%//X76114
C-THYRO1000846
C-THYRO1000866//SHK1 KINASE-BINDING PROTEIN 1.//4.40E-91//449aa//44%//P78963
C-THYRO1000956//Human G protein-coupled receptor APJ gene, complete cds.//0//1583bp//99%//U03642
C-THYRO1000964//Drosophila melanogaster Pelle associated protein Pellino (Pli) mRNA, complete cds.//1.10E-34//759bp//63%//AF091624
C-THYRO1000999
C-THYRO1001063
C-THYRO1001071//GAMMA-ADAPTIN (GOLGI ADAPTOR HA1/AP1 ADAPTIN GAMMA SUBUNIT) (CLATHRIN ASSEMBLY PROTEIN COMPLEX 1 GAMMA LARGE CHAIN).//8.20E-14//157aa//33%//P22892
C-THYRO1001102
C-THYRO1001113//Homo sapiens mRNA; cDNA DKFZp564E1616 (from clone DKFZp564E1616).//0//1361bp//99%//AL096713
C-THYRO1001128//Homo sapiens mRNA for hypothetical protein (C9orf9 gene).//6.40E-155//648bp//99%//AJ011375
C-THYRO1001205
C-THYRO1001237//PHYTOENE DEHYDROGENASE (EC 1.3.-.-) (PHYTOENE DESATURASE) (ALBINO-1 PROTEIN).//3.10E-13//346aa//22%//P21334
C-THYRO1001242//Homo sapiens cleft lip and palate transmembrane protein 1 (CLPTM1) mRNA, complete cds.//0//2468bp//99%//AF037339
C-THYRO1001266//Human sodium iodide symporter mRNA, complete cds.//7.20E-81//1466bp//62%//U66088
C-THYRO1001327
C-THYRO1001456//HYPOTHETICAL 56.2 KD PROTEIN CY31.25C.//9.40E-32//355aa//31%//Q10555
C-THYRO1001457//H.sapiens mRNA for protein kinase C mu.//2.30E-218//1183bp//73%//X75756
C-THYRO1001471
C-THYRO1001478//CYTOCHROME B-245 HEAVY CHAIN (P22 PHAGOCYTE B-CYTOCHROME) (NEUTROPHIL CYTOCHROME B, 91 KD POLYPEPTIDE) (CGD91-PHOX) (GP91-PHOX) (CYTOCHROME B(558) BETA CHAIN) (SUPEROXIDE-GENERATING NADPH OXIDASE HEAVY CHAIN SUBUNIT).//8.90E-50//296aa//35%//P04839
C-THYRO1001495
C-THYRO1001523//Homo sapiens mRNA for TM7XN1 protein.//0//3663bp//99%//AJ011001
C-THYRO1001529//SERINE PALMITOYLTRANSFERASE 2 (EC 2.3.1.50) (LONG CHAIN BASE BIOSYNTHESIS PROTEIN 2) (SPT 2).//5.50E-25//115aa//53%//Q09925
C-THYRO1001593//PUTATIVE SERINE/THREONINE-PROTEIN KINASE P78 (EC 2.7.1.-).//3.10E-203//550aa//62%//P27448
C-THYRO1001608
C-THYRO1001641//Homo sapiens CGI-57 protein mRNA, complete cds.//0//1668bp//99%//AF151815
C-THYRO1001700//SERINE/THREONINE PROTEIN KINASE RIP (EC 2.7.1.-) (CELL DEATH PROTEIN RIP) (RECEPTOR INTERACTING PROTEIN).//9.70E-33//268aa//37%//Q60855
C-THYRO1001702//Mus musculus mRNA for myeloid associated differentiation protein.//1.50E-128//1204bp//73%//AJ001616
C-THYRO1001725
C-THYRO1001770//PROBABLE SERINE/THREONINE-PROTEIN KINASE YNL020C (EC 2.7.1.-).//6.30E-20//169aa//35%//P53974
C-THYRO1001803
C-Y79AA1000030//Homo sapiens mRNA for Fe65L2, complete cds.//0//1828bp//100%//AB018247
C-Y79AA1000127
C-Y79AA1000207
C-Y79AA1000226
C-Y79AA1000270//Bos taurus vacuolar H+ ATPase subunit Ac45 mRNA, complete cds.//1.00E-271//1490bp//83%//U10039
C-Y79AA1000426//Mus musculus activin beta E subunit mRNA, complete cds.//7.70E-200//1533bp//78%//U96386
C-Y79AA1000521
C-Y79AA1000750
C-Y79AA1000776//Mus musculus mRNA for GSG1, complete cds.//2.40E-161//820bp//85%//D87325
C-Y79AA1000777//VEGETATIBLE INCOMPATIBILITY PROTEIN HET-E-1.//8.10E-48//283aa//38%//Q00808
C-Y79AA1000876//PROTEIN DISULHDE ISOMERASE-RELATED PROTEIN PRECURSOR (ERP72).//1.60E-44//210aa//38%//P13667
C-Y79AA1000888//TRNA PSEUDOURIDINE SYNTHASE A (EC 4.2.1.70) (PSEUDOURIDYLATE SYNTHASE I) (PSEUDOURIDINE SYNTHASE I) (URACIL HYDROLYASE).//1.50E-21//267aa//32%//P70973
C-Y79AA1000959//Homo sapiens Hsp70 binding protein HspBP1 mRNA, complete cds.//4.80E-283//1405bp//95%//AF093420
C-Y79AA1000967//CALCIUM/CALMODULIN-DEPENDENT PROTEIN KINASE TYPE I (EC 2.7.1.123) (CAM KINASE I).//1.00E-77//359aa//44%//Q14012
C-Y79AA1001013
C-Y79AA1001056//Homo sapiens MAID protein mRNA, complete cds.//0//1475bp//99%//AF113535
C-Y79AA1001062//TUMOR NECROSIS FACTOR, ALPHA-INDUCED PROTEIN 1, ENDOTHELIAL (B12 PROTEIN).//8.90E-12//132aa//38%//Q13829
C-Y79AA1001090//NUCLEAR FACTOR NF-KAPPA-B P105 SUBUNIT (DNA-BINDING FACTOR KBF1) (EBP- 1) (NF-KAPPA-B1 P84 /NF-KAPPA-B1 P98) [CONTAINS: NUCLEAR FACTOR NF- KAPPA-B P50 SUBUNIT] (FRAGMENT).//4.50E-09//144aa//31%//Q63369
C-Y79AA1001212//Homo sapiens SL15 protein mRNA, complete cds.//6.30E-306//1388bp//99%//AF038961
C-Y79AA1001264//HYPOTHETICAL 39.9 KD PROTEIN T15H9.1 IN CHROMOSOME II PRECURSOR.//5.10E-106//351aa//58%//Q10005
C-Y79AA1001272//Homo sapiens retinoic acid repressible protein (RARG-1) mRNA, complete cds.//1.50E-183//867bp//98%//AF172066
C-Y79AA1001328//Mus musculus mRNA for Dll3 protein, complete cds.//1.90E-263//1988bp//79%//AB013440
C-Y79AA1001426//ANION EXCHANGE PROTEIN 3 (CARDIAC/BRAIN BAND 3-LIKE PROTEIN) (CAE3/BAE3).//6.20E-66//609aa//31%//P48751
C-Y79AA1001427//Homo sapiens cytochrome b5 reductase 1 (B5R.1) mRNA, complete cds.//0//1588bp//99%//AF169481
C-Y79AA1001430//Homo sapiens mRNA for KIAA0469 protein, complete cds.//0//2943bp//99%//AB007938
C-Y79AA1001523//Homo sapiens transcriptional intermediary factor 1 alpha mRNA, complete cds.//0//2263bp//99%//AF119042
C-Y79AA1001530//Human beta-tubulin gene (5-beta) with ten Alu family members.//0//1920bp//98%//X00734
C-Y79AA1001592
C-Y79AA1001727//CELL SURFACE A33 ANTIGEN PRECURSOR.//1.10E-13//286aa//27%//Q99795
C-Y79AA1001787//PROBABLE CALCIUM-TRANSPORTING ATPASE 9 (EC 3.6.1.38).//1.70E-133//544aa//37%//Q12697
C-Y79AA1001793//Mus musculus mRNA for GSG1, complete cds.//3.70E-126//532bp//78%//D87325
C-Y79AA1001795//Homo sapiens mRNA for GalT4 protein.//2.30E-250//1137bp//99%//Y15061
C-Y79AA1001799//MITOCHONDRIAL RNA SPLICING PROTEIN MSR4.//3.40E-54//182aa//39%//P23500
C-Y79AA1001803//Homo sapiens secretogranin III mRNA, complete cds.//0//1871bp//99%//AF078851
C-Y79AA1001863
C-Y79AA1002022//POLIOVIRUS RECEPTOR HOMOLOG PRECURSOR.//2.20E-06//140aa//26%//P32507
C-Y79AA1002058//Mus musculus Gng3lg mRNA, complete cds.//4.10E-167//1145bp//83%//AF069954
C-Y79AA1002121//HISTONE H1.//4.90E-12//114aa//35%//P35060
C-Y79AA1002129
C-Y79AA1002213//HYPOTHETICAL 52.7 KD PROTEIN C38C10.2 IN CHROMOSOME III.//1.20E-98//262aa//41%//Q03567
C-Y79AA1002334//GLUCOSE REPRESSION MEDIATOR PROTEIN.//1.70E-10//333aa//23%//P14922
C-Y79AA1002373//Mus musculus mRNA for GSG1, complete cds.//7.20E-147//680bp//79%//D87325
C-Y79AA1002376//Rattus norvegicus cytoplasmic dynein intermediate chain 2B mRNA, complete cds.//1.50E-304//1667bp//90%//U39045
C-Y79AA1002378//Homo sapiens zinc finger protein NY-REN-21 antigen mRNA, partial cds.//0//963bp//99%//AF155100
C-Y79AA1002381//Homo sapiens cell cycle related kinase mRNA, complete cds.//0//1791bp//98%//AF035013

## Claims

1. Use of an oligonucleotide as a primer for synthesizing the polynucleotide comprising the nucleotide sequence set forth in any one of SEQ ID NOs: 1-829 and 2545, or the complementary strand thereof, wherein said oligonucleotide is complementary to said polynucleotide or the complementary strand thereof and comprises at least 15 nucleotides.

2. A primer set for synthesizing polynucleotides, the primer set comprising an oligo-dT primer and an oligonucleotide complementary to the complementary strand of the polynucleotide comprising the nucleotide sequence set forth in any one of SEQ ID NOs: 1-829 and 2545, wherein said oligonucleotide comprises at least 15 nucleotides.

3. A primer set for synthesizing polynucleotides, the primer set comprising a combination of an oligonucleotide comprising a nucleotide sequence complementary to the complementary strand of the polynucleotide comprising a 5'-end nucleotide sequence and an oligonucleotide comprising a nucleotide sequence complementary to the polynucleotide comprising a 3'-end nucleotide sequence, wherein said oligonucleotides comprise at least 15 nucleotides and wherein said combination of 5'-end nucleotide sequence.3'-end nucleotide sequence is selected from the group consisting of:

SEQ ID NO:4 and SEQ ID NO:830
SEQ ID NO:5 and SEQ ID NO:831
SEQ ID NO:6 and SEQ ID NO:832
SEQ ID NO:7 and SEQ ID NO:833
SEQ ID NO:8 and SEQ ID NO:834
SEQ ID NO:9 and SEQ ID NO:835
SEQ ID NO:11 and SEQ ID NO:836
SEQ ID NO:12 and SEQ ID NO:837
SEQ ID NO:13 and SEQ ID NO:838
SEQ ID NO:14 and SEQ ID NO:839
SEQ ID NO:15 and SEQ ID NO:840
SEQ ID NO:16 and SEQ ID NO:841
SEQ ID NO:17 and SEQ ID NO:842
SEQ ID NO:18 and SEQ ID NO:843
SEQ ID NO:20 and SEQ ID NO:844
SEQ ID NO:22 and SEQ ID NO:845
SEQ ID NO:23 and SEQ ID NO:846
SEQ ID NO:24 and SEQ ID NO:847
SEQ ID NO:25 and SEQ ID NO:848
SEQ ID NO:26 and SEQ ID NO:849
SEQ ID NO:27 and SEQ ID NO:850
SEQ ID NO:28 and SEQ ID NO:851
SEQ ID NO:29 and SEQ ID NO:852
SEQ ID NO:30 and SEQ ID NO:853
SEQ ID NO:31 and SEQ ID NO:854
SEQ ID NO:32 and SEQ ID NO:855
SEQ ID NO:33 and SEQ ID NO:856
SEQ ID NO:34 and SEQ ID NO:857
SEQ ID NO:35 and SEQ ID NO:858
SEQ ID NO:36 and SEQ ID NO:859
SEQ ID NO:37 and SEQ ID NO:860
SEQ ID NO:39 and SEQ ID NO:861
SEQ ID NO:40 and SEQ ID NO:862
SEQ ID NO:41 and SEQ ID NO:863
SEQ ID NO:42 and SEQ ID NO:864
SEQ ID NO:44 and SEQ ID NO:865
SEQ ID NO:45 and SEQ ID NO:866
SEQ ID NO:46 and SEQ ID NO:867
SEQ ID NO:47 and SEQ ID NO:868

SEQ ID NO:48 and SEQ ID NO:869
SEQ ID NO:49 and SEQ ID NO:870
SEQ ID NO:50 and SEQ ID NO:871
SEQ ID NO:52 and SEQ ID NO:872
SEQ ID NO:53 and SEQ ID NO:873
SEQ ID NO:54 and SEQ ID NO:874
SEQ ID NO:55 and SEQ ID NO:875
SEQ ID NO:56 and SEQ ID NO:876
SEQ ID NO:57 and SEQ ID NO:877
SEQ ID NO:58 and SEQ ID NO:878
SEQ ID NO:59 and SEQ ID NO:879
SEQ ID NO:60 and SEQ ID NO:880
SEQ ID NO:61 and SEQ ID NO:881
SEQ ID NO:62 and SEQ ID NO:882
SEQ ID NO:63 and SEQ ID NO:883
SEQ ID NO:64 and SEQ ID NO:884
SEQ ID NO:65 and SEQ ID NO:885
SEQ ID NO:66 and SEQ ID NO:886
SEQ ID NO:67 and SEQ ID NO:887
SEQ ID NO:69 and SEQ ID NO:888
SEQ ID NO:70 and SEQ ID NO:889
SEQ ID NO:71 and SEQ ID NO:890
SEQ ID NO:72 and SEQ ID NO:891
SEQ ID NO:73 and SEQ ID NO:892
SEQ ID NO:74 and SEQ ID NO:893
SEQ ID NO:75 and SEQ ID NO:894
SEQ ID NO:76 and SEQ ID NO:895
SEQ ID NO:77 and SEQ ID NO:896
SEQ ID NO:78 and SEQ ID NO:897
SEQ ID NO:79 and SEQ ID NO:898
SEQ ID NO:80 and SEQ ID NO:899
SEQ ID NO:81 and SEQ ID NO:900
SEQ ID NO:82 and SEQ ID NO:901
SEQ ID NO:83 and SEQ ID NO:902
SEQ ID NO:84 and SEQ ID NO:903
SEQ ID NO:85 and SEQ ID NO:904
SEQ ID NO:86 and SEQ ID NO:905
SEQ ID NO:87 and SEQ ID NO:906
SEQ ID NO:88 and SEQ ID NO:907
SEQ ID NO:89 and SEQ ID NO:908
SEQ ID NO:90 and SEQ ID NO:909
SEQ ID NO:91 and SEQ ID NO:910
SEQ ID NO:92 and SEQ ID NO:911
SEQ ID NO:93 and SEQ ID NO:912
SEQ ID NO:94 and SEQ ID NO:913
SEQ ID NO:95 and SEQ ID NO:914
SEQ ID NO:96 and SEQ ID NO:915
SEQ ID NO:97 and SEQ ID NO:916
SEQ ID NO:98 and SEQ ID NO:917
SEQ ID NO:99 and SEQ ID NO:918
SEQ ID NO:100 and SEQ ID NO:919
SEQ ID NO:101 and SEQ ID NO:920

SEQ ID NO:102 and SEQ ID NO:921
SEQ ID NO:103 and SEQ ID NO:922
SEQ ID NO:104 and SEQ ID NO:923
SEQ ID NO:105 and SEQ ID NO:924
SEQ ID NO:106 and SEQ ID NO:925
SEQ ID NO:107 and SEQ ID NO:926
SEQ ID NO:108 and SEQ ID NO:927
SEQ ID NO:109 and SEQ ID NO:928
SEQ ID NO:110 and SEQ ID NO:929
SEQ ID NO:111 and SEQ ID NO:930
SEQ ID NO:112 and SEQ ID NO:931
SEQ ID NO:113 and SEQ ID NO:932
SEQ ID NO:114 and SEQ ID NO:933
SEQ ID NO:115 and SEQ ID NO:934
SEQ ID NO:116 and SEQ ID NO:935
SEQ ID NO:117 and SEQ ID NO:936
SEQ ID NO:118 and SEQ ID NO:937
SEQ ID NO:119 and SEQ ID NO:938
SEQ ID NO:120 and SEQ ID NO:939
SEQ ID NO:122 and SEQ ID NO:940
SEQ ID NO:123 and SEQ ID NO:941
SEQ ID NO:124 and SEQ ID NO:942
SEQ ID NO:125 and SEQ ID NO:943
SEQ ID NO:126 and SEQ ID NO:944
SEQ ID NO:127 and SEQ ID NO:945
SEQ ID NO:128 and SEQ ID NO:946
SEQ ID NO:129 and SEQ ID NO:947
SEQ ID NO:130 and SEQ ID NO:948
SEQ ID NO:131 and SEQ ID NO:949
SEQ ID NO:132 and SEQ ID NO:950
SEQ ID NO:133 and SEQ ID NO:951
SEQ ID NO:134 and SEQ ID NO:952
SEQ ID NO:135 and SEQ ID NO:953
SEQ ID NO:136 and SEQ ID NO:954
SEQ ID NO:137 and SEQ ID NO:955
SEQ ID NO:138 and SEQ ID NO:956
SEQ ID NO:139 and SEQ ID NO:957
SEQ ID NO:140 and SEQ ID NO:958
SEQ ID NO:141 and SEQ ID NO:959
SEQ ID NO:142 and SEQ ID NO:960
SEQ ID NO:143 and SEQ ID NO:961
SEQ ID NO:144 and SEQ ID NO:962
SEQ ID NO:145 and SEQ ID NO:963
SEQ ID NO:146 and SEQ ID NO:964
SEQ ID NO:147 and SEQ ID NO:965
SEQ ID NO:148 and SEQ ID NO:966
SEQ ID NO:149 and SEQ ID NO:967
SEQ ID NO:150 and SEQ ID NO:968
SEQ ID NO:151 and SEQ ID NO:969
SEQ ID NO:152 and SEQ ID NO:970
SEQ ID NO:153 and SEQ ID NO:971
SEQ ID NO:154 and SEQ ID NO:972

SEQ ID NO:155 and SEQ ID NO:973
SEQ ID NO:156 and SEQ ID NO:974
SEQ ID NO:157 and SEQ ID NO:975
SEQ ID NO:158 and SEQ ID NO:976
SEQ ID NO:159 and SEQ ID NO:977
SEQ ID NO:160 and SEQ ID NO:978
SEQ ID NO:161 and SEQ ID NO:979
SEQ ID NO:162 and SEQ ID NO:980
SEQ ID NO:163 and SEQ ID NO:981
SEQ ID NO:164 and SEQ ID NO:982
SEQ ID NO:165 and SEQ ID NO:983
SEQ ID NO:166 and SEQ ID NO:984
SEQ ID NO:167 and SEQ ID NO:985
SEQ ID NO:168 and SEQ ID NO:986
SEQ ID NO:169 and SEQ ID NO:987
SEQ ID NO:170 and SEQ ID NO:988
SEQ ID NO:171 and SEQ ID NO:989
SEQ ID NO:172 and SEQ ID NO:990
SEQ ID NO:173 and SEQ ID NO:991
SEQ ID NO:174 and SEQ ID NO:992
SEQ ID NO:175 and SEQ ID NO:993
SEQ ID NO:176 and SEQ ID NO:994
SEQ ID NO:177 and SEQ ID NO:995
SEQ ID NO:178 and SEQ ID NO:996
SEQ ID NO:179 and SEQ ID NO:997
SEQ ID NO:180 and SEQ ID NO:998
SEQ ID NO:181 and SEQ ID NO:999
SEQ ID NO:182 and SEQ ID NO:1000
SEQ ID NO:183 and SEQ ID NO:1001
SEQ ID NO:184 and SEQ ID NO:1002
SEQ ID NO:185 and SEQ ID NO:1003
SEQ ID NO:186 and SEQ ID NO:1004
SEQ ID NO:187 and SEQ ID NO:1005
SEQ ID NO:188 and SEQ ID NO:1006
SEQ ID NO:189 and SEQ ID NO:1007
SEQ ID NO:190 and SEQ ID NO:1008
SEQ ID NO:191 and SEQ ID NO:1009
SEQ ID NO:193 and SEQ ID NO:1010
SEQ ID NO:194 and SEQ ID NO:1011
SEQ ID NO:195 and SEQ ID NO:1012
SEQ ID NO:196 and SEQ ID NO:1013
SEQ ID NO:197 and SEQ ID NO:1014
SEQ ID NO:198 and SEQ ID NO:1015
SEQ ID NO:199 and SEQ ID NO:1016
SEQ ID NO:200 and SEQ ID NO:1017
SEQ ID NO:201 and SEQ ID NO:1018
SEQ ID NO:202 and SEQ ID NO:1019
SEQ ID NO:203 and SEQ ID NO:1020
SEQ ID NO:204 and SEQ ID NO:1021
SEQ ID NO:205 and SEQ ID NO:1022
SEQ ID NO:207 and SEQ ID NO:1023
SEQ ID NO:208 and SEQ ID NO:1024

SEQ ID NO:209 and SEQ ID NO:1025
SEQ ID NO:210 and SEQ ID NO:1026
SEQ ID NO:211 and SEQ ID NO:1027
SEQ ID NO:212 and SEQ ID NO:1028
SEQ ID NO:213 and SEQ ID NO:1029
SEQ ID NO:214 and SEQ ID NO:1030
SEQ ID NO:215 and SEQ ID NO:1031
SEQ ID NO:216 and SEQ ID NO:1032
SEQ ID NO:248 and SEQ ID NO:1033
SEQ ID NO:249 and SEQ ID NO:1034
SEQ ID NO:250 and SEQ ID NO:1035
SEQ ID NO:251 and SEQ ID NO:1036
SEQ ID NO:252 and SEQ ID NO:1037
SEQ ID NO:253 and SEQ ID NO:1038
SEQ ID NO:254 and SEQ ID NO:1039
SEQ ID NO:255 and SEQ ID NO:1040
SEQ ID NO:256 and SEQ ID NO:1041
SEQ ID NO:257 and SEQ ID NO:1042
SEQ ID NO:258 and SEQ ID NO:1043
SEQ ID NO:259 and SEQ ID NO:1044
SEQ ID NO:260 and SEQ ID NO:1045
SEQ ID NO:261 and SEQ ID NO:1046
SEQ ID NO:262 and SEQ ID NO:1047
SEQ ID NO:263 and SEQ ID NO:1048
SEQ ID NO:264 and SEQ ID NO:1049
SEQ ID NO:265 and SEQ ID NO:1050
SEQ ID NO:266 and SEQ ID NO:1051
SEQ ID NO:267 and SEQ ID NO:1052
SEQ ID NO:268 and SEQ ID NO:1053
SEQ ID NO:291 and SEQ ID NO:1054
SEQ ID NO:292 and SEQ ID NO:1055
SEQ ID NO:293 and SEQ ID NO:1056
SEQ ID NO:294 and SEQ ID NO:1057
SEQ ID NO:295 and SEQ ID NO:1058
SEQ ID NO:296 and SEQ ID NO:1059
SEQ ID NO:297 and SEQ ID NO:1060
SEQ ID NO:298 and SEQ ID NO:1061
SEQ ID NO:300 and SEQ ID NO:1062
SEQ ID NO:301 and SEQ ID NO:1063
SEQ ID NO:302 and SEQ ID NO:1064
SEQ ID NO:304 and SEQ ID NO:1065
SEQ ID NO:305 and SEQ ID NO:1066
SEQ ID NO:306 and SEQ ID NO:1067
SEQ ID NO:307 and SEQ ID NO:1068
SEQ ID NO:308 and SEQ ID NO:1069
SEQ ID NO:309 and SEQ ID NO:1070
SEQ ID NO:310 and SEQ ID NO:1071
SEQ ID NO:311 and SEQ ID NO:1072
SEQ ID NO:312 and SEQ ID NO:1073
SEQ ID NO:313 and SEQ ID NO:1074
SEQ ID NO:314 and SEQ ID NO:1075
SEQ ID NO:315 and SEQ ID NO:1076

SEQ ID NO:317 and SEQ ID NO:1077
SEQ ID NO:318 and SEQ ID NO:1078
SEQ ID NO:319 and SEQ ID NO:1079
SEQ ID NO:320 and SEQ ID NO:1080
SEQ ID NO:321 and SEQ ID NO:1081
SEQ ID NO:322 and SEQ ID NO:1082
SEQ ID NO:323 and SEQ ID NO:1083
SEQ ID NO:324 and SEQ ID NO:1084
SEQ ID NO:325 and SEQ ID NO:1085
SEQ ID NO:326 and SEQ ID NO:1086
SEQ ID NO:327 and SEQ ID NO:1087
SEQ ID NO:328 and SEQ ID NO:1088
SEQ ID NO:329 and SEQ ID NO:1089
SEQ ID NO:330 and SEQ ID NO:1090
SEQ ID NO:331 and SEQ ID NO:1091
SEQ ID NO:332 and SEQ ID NO:1092
SEQ ID NO:333 and SEQ ID NO:1093
SEQ ID NO:334 and SEQ ID NO:1094
SEQ ID NO:335 and SEQ ID NO:1095
SEQ ID NO:336 and SEQ ID NO:1096
SEQ ID NO:337 and SEQ ID NO:1097
SEQ ID NO:338 and SEQ ID NO:1098
SEQ ID NO:339 and SEQ ID NO:1099
SEQ ID NO:340 and SEQ ID NO:1100
SEQ ID NO:341 and SEQ ID NO:1101
SEQ ID NO:342 and SEQ ID NO:1102
SEQ ID NO:343 and SEQ ID NO:1103
SEQ ID NO:344 and SEQ ID NO:1104
SEQ ID NO:345 and SEQ ID NO:1105
SEQ ID NO:347 and SEQ ID NO:1106
SEQ ID NO:348 and SEQ ID NO:1107
SEQ ID NO:349 and SEQ ID NO:1108
SEQ ID NO:350 and SEQ ID NO:1109
SEQ ID NO:351 and SEQ ID NO:1110
SEQ ID NO:352 and SEQ ID NO:1111
SEQ ID NO:353 and SEQ ID NO:1112
SEQ ID NO:354 and SEQ ID NO:1113
SEQ ID NO:355 and SEQ ID NO:1114
SEQ ID NO:356 and SEQ ID NO:1115
SEQ ID NO:357 and SEQ ID NO:1116
SEQ ID NO:358 and SEQ ID NO:1117
SEQ ID NO:359 and SEQ ID NO:1118
SEQ ID NO:360 and SEQ ID NO:1119
SEQ ID NO:361 and SEQ ID NO:1120
SEQ ID NO:362 and SEQ ID NO:1121
SEQ ID NO:363 and SEQ ID NO:1122
SEQ ID NO:364 and SEQ ID NO:1123
SEQ ID NO:365 and SEQ ID NO:1124
SEQ ID NO:366 and SEQ ID NO:1125
SEQ ID NO:367 and SEQ ID NO:1126
SEQ ID NO:368 and SEQ ID NO:1127
SEQ ID NO:369 and SEQ ID NO:1128

SEQ ID NO:370 and SEQ ID NO:1129
SEQ ID NO:371 and SEQ ID NO:1130
SEQ ID NO:372 and SEQ ID NO:1131
SEQ ID NO:373 and SEQ ID NO:1132
SEQ ID NO:374 and SEQ ID NO:1133
SEQ ID NO:375 and SEQ ID NO:1134
SEQ ID NO:376 and SEQ ID NO:1135
SEQ ID NO:377 and SEQ ID NO:1136
SEQ ID NO:378 and SEQ ID NO:1137
SEQ ID NO:379 and SEQ ID NO:1138
SEQ ID NO:380 and SEQ ID NO:1139
SEQ ID NO:381 and SEQ ID NO:1140
SEQ ID NO:382 and SEQ ID NO:1141
SEQ ID NO:383 and SEQ ID NO:1142
SEQ ID NO:384 and SEQ ID NO:1143
SEQ ID NO:385 and SEQ ID NO:1144
SEQ ID NO:386 and SEQ ID NO:1145
SEQ ID NO:387 and SEQ ID NO:1146
SEQ ID NO:388 and SEQ ID NO:1147
SEQ ID NO:389 and SEQ ID NO:1148
SEQ ID NO:390 and SEQ ID NO:1149
SEQ ID NO:391 and SEQ ID NO:1150
SEQ ID NO:392 and SEQ ID NO:1151
SEQ ID NO:393 and SEQ ID NO:1152
SEQ ID NO:394 and SEQ ID NO:1153
SEQ ID NO:395 and SEQ ID NO:1154
SEQ ID NO:396 and SEQ ID NO:1155
SEQ ID NO:397 and SEQ ID NO:1156
SEQ ID NO:398 and SEQ ID NO:1157
SEQ ID NO:399 and SEQ ID NO:1158
SEQ ID NO:400 and SEQ ID NO:1159
SEQ ID NO:401 and SEQ ID NO:1160
SEQ ID NO:402 and SEQ ID NO:1161
SEQ ID NO:403 and SEQ ID NO:1162
SEQ ID NO:404 and SEQ ID NO:1163
SEQ ID NO:405 and SEQ ID NO:1164
SEQ ID NO:406 and SEQ ID NO:1165
SEQ ID NO:407 and SEQ ID NO:1166
SEQ ID NO:408 and SEQ ID NO:1167
SEQ ID NO:409 and SEQ ID NO:1168
SEQ ID NO:410 and SEQ ID NO:1169
SEQ ID NO:411 and SEQ ID NO:1170
SEQ ID NO:412 and SEQ ID NO:1171
SEQ ID NO:413 and SEQ ID NO:1172
SEQ ID NO:414 and SEQ ID NO:1173
SEQ ID NO:415 and SEQ ID NO:1174
SEQ ID NO:416 and SEQ ID NO:1175
SEQ ID NO:417 and SEQ ID NO:1176
SEQ ID NO:419 and SEQ ID NO:1177
SEQ ID NO:420 and SEQ ID NO:1178
SEQ ID NO:421 and SEQ ID NO:1179
SEQ ID NO:422 and SEQ ID NO:1180

SEQ ID NO:423 and SEQ ID NO:1181
SEQ ID NO:424 and SEQ ID NO:1182
SEQ ID NO:425 and SEQ ID NO:1183
SEQ ID NO:426 and SEQ ID NO:1184
SEQ ID NO:427 and SEQ ID NO:1185
SEQ ID NO:428 and SEQ ID NO:1186
SEQ ID NO:429 and SEQ ID NO:1187
SEQ ID NO:430 and SEQ ID NO:1188
SEQ ID NO:431 and SEQ ID NO:1189
SEQ ID NO:432 and SEQ ID NO:1190
SEQ ID NO:433 and SEQ ID NO:1191
SEQ ID NO:434 and SEQ ID NO:1192
SEQ ID NO:435 and SEQ ID NO:1193
SEQ ID NO:436 and SEQ ID NO:1194
SEQ ID NO:437 and SEQ ID NO:1195
SEQ ID NO:438 and SEQ ID NO:1196
SEQ ID NO:439 and SEQ ID NO:1197
SEQ ID NO:440 and SEQ ID NO:1198
SEQ ID NO:441 and SEQ ID NO:1199
SEQ ID NO:442 and SEQ ID NO:1200
SEQ ID NO:443 and SEQ ID NO:1201
SEQ ID NO:444 and SEQ ID NO:1202
SEQ ID NO:446 and SEQ ID NO:1203
SEQ ID NO:447 and SEQ ID NO:1204
SEQ ID NO:448 and SEQ ID NO:1205
SEQ ID NO:450 and SEQ ID NO:1206
SEQ ID NO:451 and SEQ ID NO:1207
SEQ ID NO:452 and SEQ ID NO:1208
SEQ ID NO:453 and SEQ ID NO:1209
SEQ ID NO:454 and SEQ ID NO:1210
SEQ ID NO:455 and SEQ ID NO:1211
SEQ ID NO:456 and SEQ ID NO:1212
SEQ ID NO:457 and SEQ ID NO:1213
SEQ ID NO:458 and SEQ ID NO:1214
SEQ ID NO:459 and SEQ ID NO:1215
SEQ ID NO:460 and SEQ ID NO:1216
SEQ ID NO:461 and SEQ ID NO:1217
SEQ ID NO:462 and SEQ ID NO:1218
SEQ ID NO:463 and SEQ ID NO:1219
SEQ ID NO:464 and SEQ ID NO:1220
SEQ ID NO:465 and SEQ ID NO:1221
SEQ ID NO:466 and SEQ ID NO:1222
SEQ ID NO:467 and SEQ ID NO:1223
SEQ ID NO:468 and SEQ ID NO:1224
SEQ ID NO:469 and SEQ ID NO:1225
SEQ ID NO:470 and SEQ ID NO:1226
SEQ ID NO:471 and SEQ ID NO:1227
SEQ ID NO:472 and SEQ ID NO:1228
SEQ ID NO:473 and SEQ ID NO:1229
SEQ ID NO:474 and SEQ ID NO:1230
SEQ ID NO:475 and SEQ ID NO:1231
SEQ ID NO:476 and SEQ ID NO:1232

SEQ ID NO:477 and SEQ ID NO:1233
SEQ ID NO:478 and SEQ ID NO:1234
SEQ ID NO:479 and SEQ ID NO:1235
SEQ ID NO:480 and SEQ ID NO:1236
SEQ ID NO:481 and SEQ ID NO:1237
SEQ ID NO:482 and SEQ ID NO:1238
SEQ ID NO:483 and SEQ ID NO:1239
SEQ ID NO:484 and SEQ ID NO:1240
SEQ ID NO:485 and SEQ ID NO:1241
SEQ ID NO:486 and SEQ ID NO:1242
SEQ ID NO:487 and SEQ ID NO:1243
SEQ ID NO:488 and SEQ ID NO:1244
SEQ ID NO:489 and SEQ ID NO:1245
SEQ ID NO:490 and SEQ ID NO:1246
SEQ ID NO:492 and SEQ ID NO:1247
SEQ ID NO:493 and SEQ ID NO:1248
SEQ ID NO:494 and SEQ ID NO:1249
SEQ ID NO:496 and SEQ ID NO:1250
SEQ ID NO:497 and SEQ ID NO:1251
SEQ ID NO:498 and SEQ ID NO:1252
SEQ ID NO:499 and SEQ ID NO:1253
SEQ ID NO:500 and SEQ ID NO:1254
SEQ ID NO:501 and SEQ ID NO:1255
SEQ ID NO:502 and SEQ ID NO:1256
SEQ ID NO:503 and SEQ ID NO:1257
SEQ ID NO:504 and SEQ ID NO:1258
SEQ ID NO:505 and SEQ ID NO:1259
SEQ ID NO:506 and SEQ ID NO:1260
SEQ ID NO:507 and SEQ ID NO:1261
SEQ ID NO:508 and SEQ ID NO:1262
SEQ ID NO:509 and SEQ ID NO:1263
SEQ ID NO:510 and SEQ ID NO:1264
SEQ ID NO:511 and SEQ ID NO:1265
SEQ ID NO:513 and SEQ ID NO:1266
SEQ ID NO:514 and SEQ ID NO:1267
SEQ ID NO:515 and SEQ ID NO:1268
SEQ ID NO:516 and SEQ ID NO:1269
SEQ ID NO:517 and SEQ ID NO:1270
SEQ ID NO:518 and SEQ ID NO:1271
SEQ ID NO:519 and SEQ ID NO:1272
SEQ ID NO:520 and SEQ ID NO:1273
SEQ ID NO:521 and SEQ ID NO:1274
SEQ ID NO:522 and SEQ ID NO:1275
SEQ ID NO:523 and SEQ ID NO:1276
SEQ ID NO:524 and SEQ ID NO:1277
SEQ ID NO:525 and SEQ ID NO:1278
SEQ ID NO:526 and SEQ ID NO:1279
SEQ ID NO:527 and SEQ ID NO:1280
SEQ ID NO:528 and SEQ ID NO:1281
SEQ ID NO:529 and SEQ ID NO:1282
SEQ ID NO:530 and SEQ ID NO:1283
SEQ ID NO:531 and SEQ ID NO:1284

SEQ ID NO:532 and SEQ ID NO:1285
SEQ ID NO:533 and SEQ ID NO:1286
SEQ ID NO:534 and SEQ ID NO:1287
SEQ ID NO:535 and SEQ ID NO:1288
SEQ ID NO:536 and SEQ ID NO:1289
SEQ ID NO:537 and SEQ ID NO:1290
SEQ ID NO:538 and SEQ ID NO:1291
SEQ ID NO:539 and SEQ ID NO:1292
SEQ ID NO:540 and SEQ ID NO:1293
SEQ ID NO:541 and SEQ ID NO:1294
SEQ ID NO:542 and SEQ ID NO:1295
SEQ ID NO:543 and SEQ ID NO:1296
SEQ ID NO:544 and SEQ ID NO:1297
SEQ ID NO:545 and SEQ ID NO:1298
SEQ ID NO:546 and SEQ ID NO:1299
SEQ ID NO:547 and SEQ ID NO:1300
SEQ ID NO:548 and SEQ ID NO:1301
SEQ ID NO:549 and SEQ ID NO:1302
SEQ ID NO:550 and SEQ ID NO:1303
SEQ ID NO:551 and SEQ ID NO:1304
SEQ ID NO:552 and SEQ ID NO:1305
SEQ ID NO:553 and SEQ ID NO:1306
SEQ ID NO:554 and SEQ ID NO:1307
SEQ ID NO:555 and SEQ ID NO:1308
SEQ ID NO:556 and SEQ ID NO:1309
SEQ ID NO:557 and SEQ ID NO:1310
SEQ ID NO:558 and SEQ ID NO:1311
SEQ ID NO:559 and SEQ ID NO:1312
SEQ ID NO:560 and SEQ ID NO:1313
SEQ ID NO:561 and SEQ ID NO:1314
SEQ ID NO:562 and SEQ ID NO:1315
SEQ ID NO:563 and SEQ ID NO:1316
SEQ ID NO:564 and SEQ ID NO:1317
SEQ ID NO:565 and SEQ ID NO:1318
SEQ ID NO:566 and SEQ ID NO:1319
SEQ ID NO:567 and SEQ ID NO:1320
SEQ ID NO:569 and SEQ ID NO:1321
SEQ ID NO:570 and SEQ ID NO:1322
SEQ ID NO:571 and SEQ ID NO:1323
SEQ ID NO:572 and SEQ ID NO:1324
SEQ ID NO:573 and SEQ ID NO:1325
SEQ ID NO:574 and SEQ ID NO:1326
SEQ ID NO:575 and SEQ ID NO:1327
SEQ ID NO:576 and SEQ ID NO:1328
SEQ ID NO:577 and SEQ ID NO:1329
SEQ ID NO:579 and SEQ ID NO:1330
SEQ ID NO:580 and SEQ ID NO:1331
SEQ ID NO:581 and SEQ ID NO:1332
SEQ ID NO:582 and SEQ ID NO:1333
SEQ ID NO:583 and SEQ ID NO:1334
SEQ ID NO:584 and SEQ ID NO:1335
SEQ ID NO:585 and SEQ ID NO:1336

SEQ ID NO:586 and SEQ ID NO:1337
SEQ ID NO:587 and SEQ ID NO:1338
SEQ ID NO:588 and SEQ ID NO:1339
SEQ ID NO:589 and SEQ ID NO:1340
SEQ ID NO:590 and SEQ ID NO:1341
SEQ ID NO:591 and SEQ ID NO:1342
SEQ ID NO:592 and SEQ ID NO:1343
SEQ ID NO:593 and SEQ ID NO:1344
SEQ ID NO:594 and SEQ ID NO:1345
SEQ ID NO:595 and SEQ ID NO:1346
SEQ ID NO:596 and SEQ ID NO:1347
SEQ ID NO:597 and SEQ ID NO:1348
SEQ ID NO:598 and SEQ ID NO:1349
SEQ ID NO:599 and SEQ ID NO:1350
SEQ ID NO:600 and SEQ ID NO:1351
SEQ ID NO:601 and SEQ ID NO:1352
SEQ ID NO:602 and SEQ ID NO:1353
SEQ ID NO:603 and SEQ ID NO:1354
SEQ ID NO:604 and SEQ ID NO:1355
SEQ ID NO:605 and SEQ ID NO:1356
SEQ ID NO:606 and SEQ ID NO:1357
SEQ ID NO:607 and SEQ ID NO:1358
SEQ ID NO:608 and SEQ ID NO:1359
SEQ ID NO:609 and SEQ ID NO:1360
SEQ ID NO:610 and SEQ ID NO:1361
SEQ ID NO:611 and SEQ ID NO:1362
SEQ ID NO:612 and SEQ ID NO:1363
SEQ ID NO:613 and SEQ ID NO:1364
SEQ ID NO:614 and SEQ ID NO:1365
SEQ ID NO:615 and SEQ ID NO:1366
SEQ ID NO:616 and SEQ ID NO:1367
SEQ ID NO:617 and SEQ ID NO:1368
SEQ ID NO:618 and SEQ ID NO:1369
SEQ ID NO:619 and SEQ ID NO:1370
SEQ ID NO:620 and SEQ ID NO:1371
SEQ ID NO:621 and SEQ ID NO:1372
SEQ ID NO:622 and SEQ ID NO:1373
SEQ ID NO:623 and SEQ ID NO:1374
SEQ ID NO:624 and SEQ ID NO:1375
SEQ ID NO:625 and SEQ ID NO:1376
SEQ ID NO:626 and SEQ ID NO:1377
SEQ ID NO:627 and SEQ ID NO:1378
SEQ ID NO:628 and SEQ ID NO:1379
SEQ ID NO:629 and SEQ ID NO:1380
SEQ ID NO:630 and SEQ ID NO:1381
SEQ ID NO:631 and SEQ ID NO:1382
SEQ ID NO:632 and SEQ ID NO:1383
SEQ ID NO:633 and SEQ ID NO:1384
SEQ ID NO:634 and SEQ ID NO:1385
SEQ ID NO:635 and SEQ ID NO:1386
SEQ ID NO:636 and SEQ ID NO:1387
SEQ ID NO:637 and SEQ ID NO:1388

SEQ ID NO:638 and SEQ ID NO:1389
SEQ ID NO:639 and SEQ ID NO:1390
SEQ ID NO:640 and SEQ ID NO:1391
SEQ ID NO:641 and SEQ ID NO:1392
SEQ ID NO:642 and SEQ ID NO:1393
SEQ ID NO:643 and SEQ ID NO:1394
SEQ ID NO:644 and SEQ ID NO:1395
SEQ ID NO:645 and SEQ ID NO:1396
SEQ ID NO:646 and SEQ ID NO:1397
SEQ ID NO:647 and SEQ ID NO:1398
SEQ ID NO:648 and SEQ ID NO:1399
SEQ ID NO:649 and SEQ ID NO:1400
SEQ ID NO:650 and SEQ ID NO:1401
SEQ ID NO:651 and SEQ ID NO:1402
SEQ ID NO:652 and SEQ ID NO:1403
SEQ ID NO:653 and SEQ ID NO:1404
SEQ ID NO:654 and SEQ ID NO:1405
SEQ ID NO:655 and SEQ ID NO:1406
SEQ ID NO:656 and SEQ ID NO:1407
SEQ ID NO:657 and SEQ ID NO:1408
SEQ ID NO:658 and SEQ ID NO:1409
SEQ ID NO:659 and SEQ ID NO:1410
SEQ ID NO:660 and SEQ ID NO:1411
SEQ ID NO:661 and SEQ ID NO:1412
SEQ ID NO:662 and SEQ ID NO:1413
SEQ ID NO:663 and SEQ ID NO:1414
SEQ ID NO:664 and SEQ ID NO:1415
SEQ ID NO:665 and SEQ ID NO:1416
SEQ ID NO:666 and SEQ ID NO:1417
SEQ ID NO:667 and SEQ ID NO:1418
SEQ ID NO:668 and SEQ ID NO:1419
SEQ ID NO:669 and SEQ ID NO:1420
SEQ ID NO:670 and SEQ ID NO:1421
SEQ ID NO:671 and SEQ ID NO:1422
SEQ ID NO:672 and SEQ ID NO:1423
SEQ ID NO:673 and SEQ ID NO:1424
SEQ ID NO:674 and SEQ ID NO:1425
SEQ ID NO:675 and SEQ ID NO:1426
SEQ ID NO:676 and SEQ ID NO:1427
SEQ ID NO:677 and SEQ ID NO:1428
SEQ ID NO:678 and SEQ ID NO:1429
SEQ ID NO:679 and SEQ ID NO:1430
SEQ ID NO:680 and SEQ ID NO:1431
SEQ ID NO:681 and SEQ ID NO:1432
SEQ ID NO:682 and SEQ ID NO:1433
SEQ ID NO:683 and SEQ ID NO:1434
SEQ ID NO:684 and SEQ ID NO:1435
SEQ ID NO:685 and SEQ ID NO:1436
SEQ ID NO:686 and SEQ ID NO:1437
SEQ ID NO:687 and SEQ ID NO:1438
SEQ ID NO:688 and SEQ ID NO:1439
SEQ ID NO:689 and SEQ ID NO:1440

SEQ ID NO:690 and SEQ ID NO:1441
SEQ ID NO:691 and SEQ ID NO:1442
SEQ ID NO:692 and SEQ ID NO:1443
SEQ ID NO:693 and SEQ ID NO:1444
SEQ ID NO:694 and SEQ ID NO:1445
SEQ ID NO:695 and SEQ ID NO:1446
SEQ ID NO:696 and SEQ ID NO:1447
SEQ ID NO:697 and SEQ ID NO:1448
SEQ ID NO:698 and SEQ ID NO:1449
SEQ ID NO:699 and SEQ ID NO:1450
SEQ ID NO:700 and SEQ ID NO:1451
SEQ ID NO:701 and SEQ ID NO:1452
SEQ ID NO:702 and SEQ ID NO:1453
SEQ ID NO:703 and SEQ ID NO:1454
SEQ ID NO:704 and SEQ ID NO:1455
SEQ ID NO:705 and SEQ ID NO:1456
SEQ ID NO:706 and SEQ ID NO:1457
SEQ ID NO:707 and SEQ ID NO:1458
SEQ ID NO:708 and SEQ ID NO:1459
SEQ ID NO:709 and SEQ ID NO:1460
SEQ ID NO:710 and SEQ ID NO:1461
SEQ ID NO:711 and SEQ ID NO:1462
SEQ ID NO:712 and SEQ ID NO:1463
SEQ ID NO:713 and SEQ ID NO:1464
SEQ ID NO:714 and SEQ ID NO:1465
SEQ ID NO:715 and SEQ ID NO:1466
SEQ ID NO:716 and SEQ ID NO:1467
SEQ ID NO:717 and SEQ ID NO:1468
SEQ ID NO:718 and SEQ ID NO:1469
SEQ ID NO:719 and SEQ ID NO:1470
SEQ ID NO:720 and SEQ ID NO:1471
SEQ ID NO:721 and SEQ ID NO:1472
SEQ ID NO:722 and SEQ ID NO:1473
SEQ ID NO:723 and SEQ ID NO:1474
SEQ ID NO:724 and SEQ ID NO:1475
SEQ ID NO:725 and SEQ ID NO:1476
SEQ ID NO:726 and SEQ ID NO:1477
SEQ ID NO:727 and SEQ ID NO:1478
SEQ ID NO:729 and SEQ ID NO:1479
SEQ ID NO:730 and SEQ ID NO:1480
SEQ ID NO:731 and SEQ ID NO:1481
SEQ ID NO:732 and SEQ ID NO:1482
SEQ ID NO:733 and SEQ ID NO:1483
SEQ ID NO:734 and SEQ ID NO:1484
SEQ ID NO:735 and SEQ ID NO:1485
SEQ ID NO:736 and SEQ ID NO:1486
SEQ ID NO:737 and SEQ ID NO:1487
SEQ ID NO:738 and SEQ ID NO:1488
SEQ ID NO:739 and SEQ ID NO:1489
SEQ ID NO:740 and SEQ ID NO:1490
SEQ ID NO:741 and SEQ ID NO:1491
SEQ ID NO:745 and SEQ ID NO:1492

SEQ ID NO:746 and SEQ ID NO:1493
SEQ ID NO:747 and SEQ ID NO:1494
SEQ ID NO:748 and SEQ ID NO:1495
SEQ ID NO:749 and SEQ ID NO:1496
SEQ ID NO:750 and SEQ ID NO:1497
SEQ ID NO:751 and SEQ ID NO:1498
SEQ ID NO:752 and SEQ ID NO:1499
SEQ ID NO:753 and SEQ ID NO:1500
SEQ ID NO:754 and SEQ ID NO:1501
SEQ ID NO:755 and SEQ ID NO:1502
SEQ ID NO:756 and SEQ ID NO:1503
SEQ ID NO:757 and SEQ ID NO:1504
SEQ ID NO:758 and SEQ ID NO:1505
SEQ ID NO:759 and SEQ ID NO:1506
SEQ ID NO:760 and SEQ ID NO:1507
SEQ ID NO:761 and SEQ ID NO:1508
SEQ ID NO:762 and SEQ ID NO:1509
SEQ ID NO:763 and SEQ ID NO:1510
SEQ ID NO:764 and SEQ ID NO:1511
SEQ ID NO:765 and SEQ ID NO:1512
SEQ ID NO:766 and SEQ ID NO:1513
SEQ ID NO:767 and SEQ ID NO:1514
SEQ ID NO:768 and SEQ ID NO:1515
SEQ ID NO:769 and SEQ ID NO:1516
SEQ ID NO:770 and SEQ ID NO:1517
SEQ ID NO:771 and SEQ ID NO:1518
SEQ ID NO:772 and SEQ ID NO:1519
SEQ ID NO:773 and SEQ ID NO:1520
SEQ ID NO:774 and SEQ ID NO:1521
SEQ ID NO:775 and SEQ ID NO:1522
SEQ ID NO:776 and SEQ ID NO:1523
SEQ ID NO:777 and SEQ ID NO:1524
SEQ ID NO:778 and SEQ ID NO:1525
SEQ ID NO:779 and SEQ ID NO:1526
SEQ ID NO:780 and SEQ ID NO:1527
SEQ ID NO:781 and SEQ ID NO:1528
SEQ ID NO:782 and SEQ ID NO:1529
SEQ ID NO:783 and SEQ ID NO:1530
SEQ ID NO:784 and SEQ ID NO:1531
SEQ ID NO:785 and SEQ ID NO:1532
SEQ ID NO:786 and SEQ ID NO:1533
SEQ ID NO:787 and SEQ ID NO:1534
SEQ ID NO:788 and SEQ ID NO:1535
SEQ ID NO:789 and SEQ ID NO:1536
SEQ ID NO:790 and SEQ ID NO:1537
SEQ ID NO:791 and SEQ ID NO:1538
SEQ ID NO:792 and SEQ ID NO:1539
SEQ ID NO:793 and SEQ ID NO:1540
SEQ ID NO:794 and SEQ ID NO:1541
SEQ ID NO:795 and SEQ ID NO:1542
SEQ ID NO:797 and SEQ ID NO:1543
SEQ ID NO:798 and SEQ ID NO:1544

SEQ ID NO:799 and SEQ ID NO:1545
SEQ ID NO:800 and SEQ ID NO:1546
SEQ ID NO:801 and SEQ ID NO:1547
SEQ ID NO:802 and SEQ ID NO:1548
SEQ ID NO:803 and SEQ ID NO:1549
SEQ ID NO:804 and SEQ ID NO:1550
SEQ ID NO:805 and SEQ ID NO:1551
SEQ ID NO:806 and SEQ ID NO:1552
SEQ ID NO:807 and SEQ ID NO:1553
SEQ ID NO:809 and SEQ ID NO:1554
SEQ ID NO:810 and SEQ ID NO:1555
SEQ ID NO:811 and SEQ ID NO:1556
SEQ ID NO:812 and SEQ ID NO:1557
SEQ ID NO:813 and SEQ ID NO:1558
SEQ ID NO:814 and SEQ ID NO:1559
SEQ ID NO:816 and SEQ ID NO:1560
SEQ ID NO:817 and SEQ ID NO:1561
SEQ ID NO:818 and SEQ ID NO:1562
SEQ ID NO:819 and SEQ ID NO:1563
SEQ ID NO:820 and SEQ ID NO:1564
SEQ ID NO:822 and SEQ ID NO:1565
SEQ ID NO:823 and SEQ ID NO:1566
SEQ ID NO:824 and SEQ ID NO:1567
SEQ ID NO:825 and SEQ ID NO:1568
SEQ ID NO:826 and SEQ ID NO:1569
SEQ ID NO:827 and SEQ ID NO:1570
SEQ ID NO:828 and SEQ ID NO:1571
SEQ ID NO:829 and SEQ ID NO:1572, and
SEQ ID NO:2545 and SEQ ID NO:2546

4. A polynucleotide which can be synthesized with the primer set of claim 2 or 3.

5. A polynucleotide comprising a coding region in the polynucleotide of claim 4.

6. A substantially pure protein encoded by polynucleotide of claim 4.

7. A partial peptide of the protein of claim 6.

8. An isolated polynucleotide selected from the group consisting of
(a) a polynucleotide comprising a coding region of the nucleotide sequence set forth in any one of the following SEQ ID NOs: 
SEQ ID NO:2547, SEQ ID NO:nnnn , SEQ ID NO:2551, SEQ ID NO:2553, SEQ ID NO:2555,
SEQ ID NO:2557, SEQ ID NO:2558, SEQ ID NO:2560, SEQ ID NO:2562, SEQ ID NO:2564,
SEQ ID NO:2566, SEQ ID NO:2568, SEQ ID NO:2570, SEQ ID NO:2572, SEQ ID NO:2574,
SEQ ID NO:2576, SEQ ID NO:2578, SEQ ID NO:2580, SEQ ID NO:2581, SEQ ID NO:2583,
SEQ ID NO:2584, SEQ ID NO:nnnn , SEQ ID NO:2588, SEQ ID NO:2590, SEQ ID NO:2592,
SEQ ID NO:2594, SEQ ID NO:2596, SEQ ID NO:2598, SEQ ID NO:nnnn , SEQ ID NO:2602,
SEQ ID NO:2604, SEQ ID NO:2606, SEQ ID NO:2607, SEQ ID NO:2609, SEQ ID NO:2611,
SEQ ID NO:2613, SEQ ID NO:2615, SEQ ID NO:2617, SEQ ID NO:2619, SEQ ID NO:2621,
SEQ ID NO:2623, SEQ ID NO:2625, SEQ ID NO:2627, SEQ ID NO:2629, SEQ ID NO:2630,
SEQ ID NO:2632, SEQ ID NO:2634, SEQ ID NO:2636, SEQ ID NO:2638, SEQ ID NO:2640,
SEQ ID NO:2642, SEQ ID NO:2644, SEQ ID NO:2646, SEQ ID NO:2648, SEQ ID NO:2650,
SEQ ID NO:2652, SEQ ID NO:2654, SEQ ID NO:2656, SEQ ID NO:2658, SEQ ID NO:2660,
SEQ ID NO:2662, SEQ ID NO:2664, SEQ ID NO:2666, SEQ ID NO:2668, SEQ ID NO:2670,
SEQ ID NO:2672, SEQ ID NO:2674, SEQ ID NO:2676, SEQ ID NO:2678, SEQ ID NO:2680,
SEQ ID NO:2681, SEQ ID NO:2683, SEQ ID NO:2685, SEQ ID NO:2686, SEQ ID NO:2688,
SEQ ID NO:2690, SEQ ID NO:2692, SEQ ID NO:2694, SEQ ID NO:2696, SEQ ID NO:2698,
SEQ ID NO:2700, SEQ ID NO:2702, SEQ ID NO:2704, SEQ ID NO:2706, SEQ ID NO:2708,
 
SEQ ID NO:2710, SEQ ID NO:2712, SEQ ID NO:2714, SEQ ID NO:2715, SEQ ID NO:2717,
SEQ ID NO:2718, SEQ ID NO:2719, SEQ ID NO:2721, SEQ ID NO:2722, SEQ ID NO:2724,
SEQ ID NO:2726, SEQ ID NO:2728, SEQ ID NO:2730, SEQ ID NO:2732, SEQ ID NO:2734
SEQ ID NO:2735, SEQ ID NO:2737, SEQ ID NO:2739, SEQ ID NO:nnnn , SEQ ID NO:2743,
SEQ ID NO:2745, SEQ ID NO:2747, SEQ ID NO:2749, SEQ ID NO:2750, SEQ ID NO:2752,
SEQ ID NO:2754, SEQ ID NO:2756, SEQ ID NO:2758, SEQ ID NO:2760, SEQ ID NO:2761,
SEQ ID NO:nnnn , SEQ ID NO:2765, SEQ ID NO:2766, SEQ ID NO:2768, SEQ ID NO:2770,
SEQ ID NO:2772, SEQ ID NO:2774, SEQ ID NO:2776, SEQ ID NO:2778, SEQ ID NO:2780,
SEQ ID NO:2782, SEQ ID NO:2784, SEQ ID NO:2786, SEQ ID NO:2788, SEQ ID NO:2790,
SEQ ID NO:2792, SEQ ID NO:2794, SEQ ID NO:2796, SEQ ID NO:2798, SEQ ID NO:2800,
SEQ ID NO:2802, SEQ ID NO:2804, SEQ ID NO:2806, SEQ ID NO:2808, SEQ ID NO:2810,
SEQ ID NO:2812, SEQ ID NO:2814, SEQ ID NO:2816, SEQ ID NO:2818, SEQ ID NO:2820,
SEQ ID NO:2822, SEQ ID NO:2824, SEQ ID NO:2826, SEQ ID NO:2828, SEQ ID NO:2830,
SEQ ID NO:2832, SEQ ID NO:2834, SEQ ID NO:2836, SEQ ID NO:2838, SEQ ID NO:2840,
SEQ ID NO:2842, SEQ ID NO:2844, SEQ ID NO:2846, SEQ ID NO:2848, SEQ ID NO:2850,
SEQ ID NO:2852, SEQ ID NO:2854, SEQ ID NO:2856, SEQ ID NO:2858, SEQ ID NO:2860,
SEQ ID NO:2862, SEQ ID NO:2864, SEQ ID NO:2866, SEQ ID NO:2868, SEQ ID NO:2870,
SEQ ID NO:2872, SEQ ID NO:2874, SEQ ID NO:2876, SEQ ID NO:2878, SEQ ID NO:2880,
SEQ ID NO:2882, SEQ ID NO:2884, SEQ ID NO:2886, SEQ ID NO:2888, SEQ ID NO:2890,
SEQ ID NO:2892, SEQ ID NO:2894, SEQ ID NO:2896, SEQ ID NO:2898, SEQ ID NO:2900,
SEQ ID NO:2902, SEQ ID NO:2904, SEQ ID NO:2906, SEQ ID NO:2908, SEQ ID NO:2910,
SEQ ID NO:2912, SEQ ID NO:2914, SEQ ID NO:2916, SEQ ID NO:2918, SEQ ID NO:2920,
SEQ ID NO:2922, SEQ ID NO:2924, SEQ ID NO:2926, SEQ ID NO:2928, SEQ ID NO:2930
SEQ ID NO:2932, SEQ ID NO:2934, SEQ ID NO:2936, SEQ ID NO:2938, SEQ ID NO:2940,
SEQ ID NO:2942, SEQ ID NO:2944, SEQ ID NO:2946, SEQ ID NO:2948, SEQ ID NO:2950,
SEQ ID NO:2952, SEQ ID NO:2954, SEQ ID NO:2956, SEQ ID NO:2958, SEQ ID NO:2960,
SEQ ID NO:2962, SEQ ID NO:nnnn , SEQ ID NO:2966, SEQ ID NO:2967, SEQ ID NO:2969,
SEQ ID NO:2971, SEQ ID NO:2973, SEQ ID NO:2975, SEQ ID NO:2977, SEQ ID NO:2979,
SEQ ID NO:2981, SEQ ID NO:2983, SEQ ID NO:2985, SEQ ID NO:2987, SEQ ID NO:2989,
SEQ ID NO:2991, SEQ ID NO:2993, SEQ ID NO:2995, SEQ ID NO:2997, SEQ ID NO:2999,
SEQ ID NO:3001, SEQ ID NO:3003, SEQ ID NO:3005, SEQ ID NO:3007, SEQ ID NO:3009,
SEQ ID NO:3011, SEQ ID NO:3013, SEQ ID NO:3015, SEQ ID NO:3017, SEQ ID NO:3019,
SEQ ID NO:3021, SEQ ID NO:3023, SEQ ID NO:3025, SEQ ID NO:3027, SEQ ID NO:3029,
SEQ ID NO:3031, SEQ ID NO:3033, SEQ ID NO:3035, SEQ ID NO:3037, SEQ ID NO:3039,
SEQ ID NO:nnnn , SEQ ID NO:nnnn , SEQ ID NO:3045, SEQ ID NO:3047, SEQ ID NO:3049,
SEQ ID NO:3051, SEQ ID NO:3053, SEQ ID NO:3055, SEQ ID NO:3057, SEQ ID NO:3059,
SEQ ID NO:3061, SEQ ID NO:3063, SEQ ID NO:3065, SEQ ID NO:3067, SEQ ID NO:3069,
SEQ ID NO:3071, SEQ ID NO:3073, SEQ ID NO:3075, SEQ ID NO:3077, SEQ ID NO:3079,
SEQ ID NO:3081, SEQ ID NO:3083, SEQ ID NO:3085, SEQ ID NO:3087, SEQ ID NO:3089,
SEQ ID NO:3091, SEQ ID NO:3093, SEQ ID NO:3095, SEQ ID NO:3097, SEQ ID NO:3099,
SEQ ID NO:3101, SEQ ID NO:3103, SEQ ID NO:3105, SEQ ID NO:3107, SEQ ID NO:3109,
SEQ ID NO:3111, SEQ ID NO:3113, SEQ ID NO:3115, SEQ ID NO:3117, SEQ ID NO:3119,
SEQ ID NO:3121, SEQ ID NO:3123, SEQ ID NO:3125, SEQ ID NO:3127, SEQ ID NO:3129
SEQ ID NO:3131, SEQ ID NO:3133, SEQ ID NO:3135, SEQ ID NO:3137, SEQ ID NO:3139,
SEQ ID NO:3140, SEQ ID NO:3142, SEQ ID NO:3143, SEQ ID NO:3144, SEQ ID NO:3146,
SEQ ID NO:3148, SEQ ID NO:3150, SEQ ID NO:3152, SEQ ID NO:3154, SEQ ID NO:3156,
SEQ ID NO:3158, SEQ ID NO:nnnn , SEQ ID NO:3162, SEQ ID NO:3164, SEQ ID NO:3166,
SEQ ID NO:3168, SEQ ID NO:3170, SEQ ID NO:3172, SEQ ID NO:3174, SEQ ID NO:3176,
SEQ ID NO:3178, SEQ ID NO:3180, SEQ ID NO:3181, SEQ ID NO:3183, SEQ ID NO:3185,
SEQ ID NO:3187, SEQ ID NO:3188, SEQ ID NO:3189, SEQ ID NO:3191, SEQ ID NO:3193,
SEQ ID NO:3195, SEQ ID NO:3196, SEQ ID NO:3198, SEQ ID NO:3200, SEQ ID NO:3202,
SEQ ID NO:3204, SEQ ID NO:3206, SEQ ID NO:3208, SEQ ID NO:3210, SEQ ID NO:3212,
 
SEQ ID NO:3214, SEQ ID NO:nnnn , SEQ ID NO:3218, SEQ ID NO:3219, SEQ ID NO:3221,
SEQ ID NO:3222, SEQ ID NO:3224, SEQ ID NO:3226, SEQ ID NO:nnnn , SEQ ID NO:3230,
SEQ ID NO:3232, SEQ ID NO:3234, SEQ ID NO:3236, SEQ ID NO:3238, SEQ ID NO:3240,
SEQ ID NO:3242, SEQ ID NO:3244, SEQ ID NO:3246, SEQ ID NO:nnnn , SEQ ID NO:3250,
SEQ ID NO:3252, SEQ ID NO:3254, SEQ ID NO:3256, SEQ ID NO:3258, SEQ ID NO:3260,
SEQ ID NO:3262, SEQ ID NO:3264, SEQ ID NO:3266, SEQ ID NO:3268, SEQ ID NO:3270,
SEQ ID NO:3272, SEQ ID NO:3274, SEQ ID NO:3276, SEQ ID NO:3278, SEQ ID NO:3280,
SEQ ID NO:3282, SEQ ID NO:3284, SEQ ID NO:3286, SEQ ID NO:3288, SEQ ID NO:3290,
SEQ ID NO:3292, SEQ ID NO:3294, SEQ ID NO:3296, SEQ ID NO:3298, SEQ ID NO:3300,
SEQ ID NO:3301, SEQ ID NO:3303, SEQ ID NO:3305, SEQ ID NO:3307, SEQ ID NO:3309,
SEQ ID NO:3311, SEQ ID NO:3312, SEQ ID NO:3314, SEQ ID NO:3316, SEQ ID NO:3318
SEQ ID NO:3320, SEQ ID NO:3322, SEQ ID NO:3324, SEQ ID NO:3326, SEQ ID NO:3328,
SEQ ID NO:3330, SEQ ID NO:3332, SEQ ID NO:3334, SEQ ID NO:3336, SEQ ID NO:3338,
SEQ ID NO:nnnn , SEQ ID NO:3342, SEQ ID NO:3344, SEQ ID NO:3346, SEQ ID NO:3348,
SEQ ID NO:3350, SEQ ID NO:3352, SEQ ID NO:3354, SEQ ID NO:3356, SEQ ID NO:3358,
SEQ ID NO:3360, SEQ ID NO:3362, SEQ ID NO:3364, SEQ ID NO:3366, SEQ ID NO:3368,
SEQ ID NO:3370, SEQ ID NO:3372, SEQ ID NO:3374, SEQ ID NO:3376, SEQ ID NO:3378,
SEQ ID NO:3380, SEQ ID NO:3382, SEQ ID NO:3384, SEQ ID NO:3386, SEQ ID NO:3388,
SEQ ID NO:3390, SEQ ID NO:3392, SEQ ID NO:3394, SEQ ID NO:3396, SEQ ID NO:3398,
SEQ ID NO:3400, SEQ ID NO:3402, SEQ ID NO:3404, SEQ ID NO:nnnn , SEQ ID NO:3408,
SEQ ID NO:3410, SEQ ID NO:3411, SEQ ID NO:3413, SEQ ID NO:3415, SEQ ID NO:3417,
SEQ ID NO:3419, SEQ ID NO:nnnn , SEQ ID NO:3423, SEQ ID NO:3425, SEQ ID NO:3427,
SEQ ID NO:3429, SEQ ID NO:3431, SEQ ID NO:3433, SEQ ID NO:3435, SEQ ID NO:3437,
SEQ ID NO:3439, SEQ ID NO:3441, SEQ ID NO:3443, SEQ ID NO:3445, SEQ ID NO:3447,
SEQ ID NO:3449, SEQ ID NO:3451, SEQ ID NO:3453, SEQ ID NO:3455, SEQ ID NO:3457,
SEQ ID NO:3459, SEQ ID NO:3461, SEQ ID NO:3463, SEQ ID NO:3465, SEQ ID NO:3467,
SEQ ID NO:3469, SEQ ID NO:3471, SEQ ID NO:3473, SEQ ID NO:3475, SEQ ID NO:3477,
SEQ ID NO:3479, SEQ ID NO:3481, SEQ ID NO:3482, SEQ ID NO:3484, SEQ ID NO:3486,
SEQ ID NO:3488, SEQ ID NO:3490, SEQ ID NO:3492, SEQ ID NO:3494, SEQ ID NO:3496,
SEQ ID NO:3498, SEQ ID NO:3500, SEQ ID NO:3502, SEQ ID NO:3504, SEQ ID NO:3506,
SEQ ID NO:3508, SEQ ID NO:3509, SEQ ID NO:3511, SEQ ID NO:3513, SEQ ID NO:3515
SEQ ID NO:3517, SEQ ID NO:3519, SEQ ID NO:3521, SEQ ID NO:3523, SEQ ID NO:3525,
SEQ ID NO:3527, SEQ ID NO:3529, SEQ ID NO:3531, SEQ ID NO:3533, SEQ ID NO:3535,
SEQ ID NO:3537, SEQ ID NO:3539, SEQ ID NO:3541, SEQ ID NO:3543, SEQ ID NO:3545,
SEQ ID NO:3547, SEQ ID NO:3549, SEQ ID NO:3551, SEQ ID NO:3553, SEQ ID NO:3555,
SEQ ID NO:3557, SEQ ID NO:3559, SEQ ID NO:3561, SEQ ID NO:3563, SEQ ID NO:nnnn ,
SEQ ID NO:3567, SEQ ID NO:3569, SEQ ID NO:3571, SEQ ID NO:3573, SEQ ID NO:3575,
SEQ ID NO:3577, SEQ ID NO:3579, SEQ ID NO:3581, SEQ ID NO:3583, SEQ ID NO:3585,
SEQ ID NO:3587, SEQ ID NO:3589 , SEQ ID NO:3595, SEQ ID NO:3597, SEQ ID NO:3599,
SEQ ID NO:3601, SEQ ID NO:3603, SEQ ID NO:3605, SEQ ID NO:3607, SEQ ID NO:3609,
SEQ ID NO:3610, SEQ ID NO:3611, SEQ ID NO:3612, SEQ ID NO:3613, SEQ ID NO:3615,
SEQ ID NO:3617, SEQ ID NO:3619, SEQ ID NO:3621, SEQ ID NO:3622, SEQ ID NO:3624,
SEQ ID NO:3626, SEQ ID NO:3627, SEQ ID NO:3628, SEQ ID NO:3629, SEQ ID NO:3631,
SEQ ID NO:3633, SEQ ID NO:3634, SEQ ID NO:3636, SEQ ID NO:3638, SEQ ID NO:3640,
SEQ ID NO:3642, SEQ ID NO:3644, SEQ ID NO:3645, SEQ ID NO:3647, SEQ ID NO:3649,
SEQ ID NO:3651, SEQ ID NO:3653, SEQ ID NO:3655, SEQ ID NO:3657, SEQ ID NO:3658,
SEQ ID NO:3660, SEQ ID NO:3662, SEQ ID NO:3664, SEQ ID NO:3666, SEQ ID NO:3668,
SEQ ID NO:3670, SEQ ID NO:3672, SEQ ID NO:3674, SEQ ID NO:3676, SEQ ID NO:3678,
SEQ ID NO:3680, SEQ ID NO:3682, SEQ ID NO:3683, SEQ ID NO:3685, SEQ ID NO:3686,
SEQ ID NO:3688, SEQ ID NO:3690, SEQ ID NO:3692, SEQ ID NO:3694, SEQ ID NO:3696,
SEQ ID NO:3698, SEQ ID NO:3700, SEQ ID NO:3702, SEQ ID NO:3704, SEQ ID NO:3706,
SEQ ID NO:3708, SEQ ID NO:3710, SEQ ID NO:3712, SEQ ID NO:3714, SEQ ID NO:3716,
 
SEQ ID NO:3718, SEQ ID NO:3720, SEQ ID NO:3722, SEQ ID NO:3724, SEQ ID NO:3726,
SEQ ID NO:3728, SEQ ID NO:3730, SEQ ID NO:3732, SEQ ID NO:3734, SEQ ID NO:3736,
SEQ ID NO:3738, SEQ ID NO:3740, SEQ ID NO:3741, SEQ ID NO:3743, SEQ ID NO:3745,
SEQ ID NO:3747, SEQ ID NO:3749, SEQ ID NO:3751, SEQ ID NO:3753, SEQ ID NO:3755,
SEQ ID NO:3756, SEQ ID NO:3758, SEQ ID NO:3760, SEQ ID NO:3761, SEQ ID NO:3763,
SEQ ID NO:3765, SEQ ID NO:3767, SEQ ID NO:3769, SEQ ID NO:3771, SEQ ID NO:3773,
SEQ ID NO:3775, SEQ ID NO:3777
SEQ ID NO:3779, SEQ ID NO:3781, SEQ ID NO:3783, SEQ ID NO:3785, SEQ ID NO:3787,
SEQ ID NO:3789, SEQ ID NO:3791, SEQ ID NO:3793, SEQ ID NO:3795, SEQ ID NO:3797,
SEQ ID NO:3799, SEQ ID NO:3801, SEQ ID NO:3803, SEQ ID NO:3805, SEQ ID NO:3806,
SEQ ID NO:3808, SEQ ID NO:3810, SEQ ID NO:3812, SEQ ID NO:3814, SEQ ID NO:3816,
SEQ ID NO:3818, SEQ ID NO:3820, SEQ ID NO:3822, SEQ ID NO:3824, SEQ ID NO:3826,
SEQ ID NO:3828, SEQ ID NO:3830, SEQ ID NO:3832, SEQ ID NO:3834, SEQ ID NO:3836,
SEQ ID NO:3838, SEQ ID NO:3840, SEQ ID NO:3842, SEQ ID NO:3844, SEQ ID NO:3846,
SEQ ID NO:3848, SEQ ID NO:3852, SEQ ID NO:3854, SEQ ID NO:3856, SEQ ID NO:3858,
SEQ ID NO:3860, SEQ ID NO:3862, SEQ ID NO:3864, SEQ ID NO:3866, SEQ ID NO:3868,
SEQ ID NO:3870, SEQ ID NO:3872, SEQ ID NO:3874, SEQ ID NO:3876, SEQ ID NO:3878,
SEQ ID NO:3880, SEQ ID NO:3882, SEQ ID NO:3884, SEQ ID NO:3886, SEQ ID NO:3888,
SEQ ID NO:3890, SEQ ID NO:3892, SEQ ID NO:3894, SEQ ID NO:3896, SEQ ID NO:3898,
SEQ ID NO:3900, SEQ ID NO:3902, SEQ ID NO:3904, SEQ ID NO:3906, SEQ ID NO:3908,
SEQ ID NO:3910, SEQ ID NO:3912, SEQ ID NO:3914, SEQ ID NO:3916, SEQ ID NO:3918,
SEQ ID NO:3920, SEQ ID NO:3922, SEQ ID NO:3924, SEQ ID NO:3926, SEQ ID NO:3928,
SEQ ID NO:3930, SEQ ID NO:3932, SEQ ID NO:3934, SEQ ID NO:3936, SEQ ID NO:3938,
SEQ ID NO:3940, SEQ ID NO:3942, SEQ ID NO:3944, SEQ ID NO:3946, SEQ ID NO:3948,
SEQ ID NO:3950, SEQ ID NO:3952, SEQ ID NO:3954, SEQ ID NO:3956, SEQ ID NO:3958,
SEQ ID NO:3960, SEQ ID NO:3962, SEQ ID NO:3964, SEQ ID NO:3965, SEQ ID NO:3966,
SEQ ID NO:3968, SEQ ID NO:3970, SEQ ID NO:3972, SEQ ID NO:3974, SEQ ID NO:3976
SEQ ID NO:3977, SEQ ID NO:3979, SEQ ID NO:3981, SEQ ID NO:3983, SEQ ID NO:3985,
SEQ ID NO:3987, SEQ ID NO:3988, SEQ ID NO:3990, SEQ ID NO:3992, SEQ ID NO:3994,
SEQ ID NO:3996, SEQ ID NO:3998, SEQ ID NO:4000, SEQ ID NO:4001, SEQ ID NO:4003,
SEQ ID NO:4004, SEQ ID NO:4006, SEQ ID NO:4008, SEQ ID NO:4010, SEQ ID NO:4012,
SEQ ID NO:4016, SEQ ID NO:4018, SEQ ID NO:4020, SEQ ID NO:4022, SEQ ID NO:4024,
SEQ ID NO:4026, SEQ ID NO:4028, SEQ ID NO:4030, SEQ ID NO:4032, SEQ ID NO:4034,
SEQ ID NO:4036, SEQ ID NO:4038, SEQ ID NO:4040, SEQ ID NO:4042, SEQ ID NO:4044,
SEQ ID NO:4046, SEQ ID NO:4048, SEQ ID NO:4050, SEQ ID NO:4052, SEQ ID NO:4054,
SEQ ID NO:4056, SEQ ID NO:4058, SEQ ID NO:4060, SEQ ID NO:4062, SEQ ID NO:4064,
SEQ ID NO:4066, SEQ ID NO:4068, SEQ ID NO:4070, SEQ ID NO:4072, SEQ ID NO:4074,
SEQ ID NO:4076
SEQ ID NO:4078, SEQ ID NO:4080, SEQ ID NO:4082, SEQ ID NO:4084, SEQ ID NO:4086,
SEQ ID NO:4088, SEQ ID NO:4090, SEQ ID NO:4092, SEQ ID NO:4094, SEQ ID NO:4096,
SEQ ID NO:4098, SEQ ID NO:4099, SEQ ID NO:4101, SEQ ID NO:4103, SEQ ID NO:4105,
SEQ ID NO:4107, SEQ ID NO:4109, SEQ ID NO:4111, SEQ ID NO:4113, SEQ ID NO:4115,
SEQ ID NO:4117, SEQ ID NO:4119, SEQ ID NO:4121, SEQ ID NO:4123, SEQ ID NO:4125,
SEQ ID NO:4127, SEQ ID NO:4129, SEQ ID NO:4131, SEQ ID NO:4132, SEQ ID NO:4134,
SEQ ID NO:4136, SEQ ID NO:4137, SEQ ID NO:4139, SEQ ID NO:4140, SEQ ID NO:4142,
SEQ ID NO:4144, SEQ ID NO:4146, SEQ ID NO:4148, SEQ ID NO:4150, SEQ ID NO:4152,
SEQ ID NO:4154, SEQ ID NO:4155, SEQ ID NO:4157, SEQ ID NO:4159, SEQ ID NO:4161,
SEQ ID NO:4163, SEQ ID NO:4165
SEQ ID NO:4167, SEQ ID NO:4168, SEQ ID NO:4170, SEQ ID NO:4172, SEQ ID NO:4174,
SEQ ID NO:4176, and SEQ ID NO:4178
(b) a polynucleotide comprising a nucleotide sequence encoding a protein comprising the amino acid sequence set forth in any one of the following SEQ ID NOs: 
SEQ ID NO:2548, SEQ ID NO:nnnn , SEQ ID NO:2552, SEQ ID NO:2554, SEQ ID NO:2556,
SEQ ID NO:2559, SEQ ID NO:2561, SEQ ID NO:2563, SEQ ID NO:2565, SEQ ID NO:2567,
SEQ ID NO:2569, SEQ ID NO:2571, SEQ ID NO:2573, SEQ ID NO:2575, SEQ ID NO:2577,
SEQ ID NO:2579, SEQ ID NO:2582, SEQ ID NO:2585, SEQ ID NO:nnnn , SEQ ID NO:2589,
SEQ ID NO:2591, SEQ ID NO:2593, SEQ ID NO:2595, SEQ ID NO:2597, SEQ ID NO:2599,
SEQ ID NO:nnnn , SEQ ID NO:2603, SEQ ID NO:2605, SEQ ID NO:2608, SEQ ID NO:2610,
SEQ ID NO:2612, SEQ ID NO:2614, SEQ ID NO:2616, SEQ ID NO:2618, SEQ ID NO:2620,
SEQ ID NO:2622, SEQ ID NO:2624, SEQ ID NO:2626, SEQ ID NO:2628, SEQ ID NO:2631,
SEQ ID NO:2633, SEQ ID NO:2635, SEQ ID NO:2637, SEQ ID NO:2639, SEQ ID NO:2641,
SEQ ID NO:2643, SEQ ID NO:2645, SEQ ID NO:2647, SEQ ID NO:2649, SEQ ID NO:2651,
SEQ ID NO:2653, SEQ ID NO:2655, SEQ ID NO:2657, SEQ ID NO:2659, SEQ ID NO:2661,
SEQ ID NO:2663, SEQ ID NO:2665, SEQ ID NO:2667, SEQ ID NO:2669, SEQ ID NO:2671,
SEQ ID NO:2673, SEQ ID NO:2675, SEQ ID NO:2677, SEQ ID NO:2679, SEQ ID NO:2682,
SEQ ID NO:2684, SEQ ID NO:2687, SEQ ID NO:2689, SEQ ID NO:2691, SEQ ID NO:2693,
SEQ ID NO:2695, SEQ ID NO:2697, SEQ ID NO:2699, SEQ ID NO:2701, SEQ ID NO:2703,
SEQ ID NO:2705, SEQ ID NO:2707, SEQ ID NO:2709, SEQ ID NO:2711, SEQ ID NO:2713,
SEQ ID NO:2716, SEQ ID NO:2720, SEQ ID NO:2723, SEQ ID NO:2725, SEQ ID NO:2727,
SEQ ID NO:2729, SEQ ID NO:2731, SEQ ID NO:2733, SEQ ID NO:2736, SEQ ID NO:2738,
SEQ ID NO:2740, SEQ ID NO:nnnn , SEQ ID NO:2744, SEQ ID NO:2746, SEQ ID NO:2748,
SEQ ID NO:2751, SEQ ID NO:2753, SEQ ID NO:2755, SEQ ID NO:2757, SEQ ID NO:2759
SEQ ID NO:2762, SEQ ID NO:nnnn , SEQ ID NO:2767, SEQ ID NO:2769, SEQ ID NO:2771,
SEQ ID NO:2773, SEQ ID NO:2775, SEQ ID NO:2777, SEQ ID NO:2779, SEQ ID NO:2781,
SEQ ID NO:2783, SEQ ID NO:2785, SEQ ID NO:2787, SEQ ID NO:2789, SEQ ID NO:2791,
SEQ ID NO:2793, SEQ ID NO:2795, SEQ ID NO:2797, SEQ ID NO:2799, SEQ ID NO:2801,
SEQ ID NO:2803, SEQ ID NO:2805, SEQ ID NO:2807, SEQ ID NO:2809, SEQ ID NO:2811,
SEQ ID NO:2813, SEQ ID NO:2815, SEQ ID NO:2817, SEQ ID NO:2819, SEQ ID NO:2821,
SEQ ID NO:2823, SEQ ID NO:2825, SEQ ID NO:2827, SEQ ID NO:2829, SEQ ID NO:2831,
SEQ ID NO:2833, SEQ ID NO:2835, SEQ ID NO:2837, SEQ ID NO:2839, SEQ ID NO:2841,
SEQ ID NO:2843, SEQ ID NO:2845, SEQ ID NO:2847, SEQ ID NO:2849, SEQ ID NO:2851,
SEQ ID NO:2853, SEQ ID NO:2855, SEQ ID NO:2857, SEQ ID NO:2859, SEQ ID NO:2861,
SEQ ID NO:2863, SEQ ID NO:2865, SEQ ID NO:2867, SEQ ID NO:2869, SEQ ID NO:2871,
SEQ ID NO:2873, SEQ ID NO:2875, SEQ ID NO:2877, SEQ ID NO:2879, SEQ ID NO:2881,
SEQ ID NO:2883, SEQ ID NO:2885, SEQ ID NO:2887, SEQ ID NO:2889, SEQ ID NO:2891,
SEQ ID NO:2893, SEQ ID NO:2895, SEQ ID NO:2897, SEQ ID NO:2899, SEQ ID NO:2901,
SEQ ID NO:2903, SEQ ID NO:2905, SEQ ID NO:2907, SEQ ID NO:2909, SEQ ID NO:2911,
SEQ ID NO:2913, SEQ ID NO:2915, SEQ ID NO:2917, SEQ ID NO:2919, SEQ ID NO:2921,
SEQ ID NO:2923, SEQ ID NO:2925, SEQ ID NO:2927, SEQ ID NO:2929, SEQ ID NO:2931,
SEQ ID NO:2933, SEQ ID NO:2935, SEQ ID NO:2937, SEQ ID NO:2939, SEQ ID NO:2941,
SEQ ID NO:2943, SEQ ID NO:2945, SEQ ID NO:2947, SEQ ID NO:2949, SEQ ID NO:2951,
SEQ ID NO:2953, SEQ ID NO:2955, SEQ ID NO:2957, SEQ ID NO:2959, SEQ ID NO:2961
SEQ ID NO:2963, SEQ ID NO:nnnn , SEQ ID NO:2968, SEQ ID NO:2970, SEQ ID NO:2972,
SEQ ID NO:2974, SEQ ID NO:2976, SEQ ID NO:2978, SEQ ID NO:2980, SEQ ID NO:2982,
SEQ ID NO:2984, SEQ ID NO:2986, SEQ ID NO:2988, SEQ ID NO:2990, SEQ ID NO:2992,
SEQ ID NO:2994, SEQ ID NO:2996, SEQ ID NO:2998, SEQ ID NO:3000, SEQ ID NO:3002,
SEQ ID NO:3004, SEQ ID NO:3006, SEQ ID NO:3008, SEQ ID NO:3010, SEQ ID NO:3012,
SEQ ID NO:3014, SEQ ID NO:3016, SEQ ID NO:3018, SEQ ID NO:3020, SEQ ID NO:3022,
SEQ ID NO:3024, SEQ ID NO:3026, SEQ ID NO:3028, SEQ ID NO:3030, SEQ ID NO:3032,
SEQ ID NO:3034, SEQ ID NO:3036, SEQ ID NO:3038, SEQ ID NO:3040, SEQ ID NO:nnnn ,
SEQ ID NO:nnnn , SEQ ID NO:3046, SEQ ID NO:3048, SEQ ID NO:3050, SEQ ID NO:3052,
SEQ ID NO:3054, SEQ ID NO:3056, SEQ ID NO:3058, SEQ ID NO:3060, SEQ ID NO:3062,
SEQ ID NO:3064, SEQ ID NO:3066, SEQ ID NO:3068, SEQ ID NO:3070, SEQ ID NO:3072,
SEQ ID NO:3074, SEQ ID NO:3076, SEQ ID NO:3078, SEQ ID NO:3080, SEQ ID NO:3082,
 
SEQ ID NO:3084, SEQ ID NO:3086, SEQ ID NO:3088, SEQ ID NO:3090, SEQ ID NO:3092,
SEQ ID NO:3094, SEQ ID NO:3096, SEQ ID NO:3098, SEQ ID NO:3100, SEQ ID NO:3102,
SEQ ID NO:3104, SEQ ID NO:3106, SEQ ID NO:3108, SEQ ID NO:3110, SEQ ID NO:3112,
SEQ ID NO:3114, SEQ ID NO:3116, SEQ ID NO:3118, SEQ ID NO:3120, SEQ ID NO:3122,
SEQ ID NO:3124, SEQ ID NO:3126, SEQ ID NO:3128, SEQ ID NO:3130, SEQ ID NO:3132,
SEQ ID NO:3134, SEQ ID NO:3136, SEQ ID NO:3138, SEQ ID NO:3141, SEQ ID NO:3145,
SEQ ID NO:3147, SEQ ID NO:3149, SEQ ID NO:3151, SEQ ID NO:3153, SEQ ID NO:3155,
SEQ ID NO:3157, SEQ ID NO:3159, SEQ ID NO:nnnn , SEQ ID NO:3163, SEQ ID NO:3165
SEQ ID NO:3167, SEQ ID NO:3169, SEQ ID NO:3171, SEQ ID NO:3173, SEQ ID NO:3175,
SEQ ID NO:3177, SEQ ID NO:3179, SEQ ID NO:3182, SEQ ID NO:3184, SEQ ID NO:3186,
SEQ ID NO:3190, SEQ ID NO:3192, SEQ ID NO:3194, SEQ ID NO:3197, SEQ ID NO:3199,
SEQ ID NO:3201, SEQ ID NO:3203, SEQ ID NO:3205, SEQ ID NO:3207, SEQ ID NO:3209,
SEQ ID NO:3211, SEQ ID NO:3213, SEQ ID NO:3215, SEQ ID NO:nnnn , SEQ ID NO:3220,
SEQ ID NO:3223, SEQ ID NO:3225, SEQ ID NO:3227, SEQ ID NO:nnnn , SEQ ID NO:3231,
SEQ ID NO:3233, SEQ ID NO:3235, SEQ ID NO:3237, SEQ ID NO:3239, SEQ ID NO:3241,
SEQ ID NO:3243, SEQ ID NO:3245, SEQ ID NO:3247, SEQ ID NO:nnnn , SEQ ID NO:3251,
SEQ ID NO:3253, SEQ ID NO:3255, SEQ ID NO:3257, SEQ ID NO:3259, SEQ ID NO:3261,
SEQ ID NO:3263, SEQ ID NO:3265, SEQ ID NO:3267, SEQ ID NO:3269, SEQ ID NO:3271,
SEQ ID NO:3273, SEQ ID NO:3275, SEQ ID NO:3277, SEQ ID NO:3279, SEQ ID NO:3281,
SEQ ID NO:3283, SEQ ID NO:3285, SEQ ID NO:3287, SEQ ID NO:3289, SEQ ID NO:3291,
SEQ ID NO:3293, SEQ ID NO:3295, SEQ ID NO:3297, SEQ ID NO:3299, SEQ ID NO:3302,
SEQ ID NO:3304, SEQ ID NO:3306, SEQ ID NO:3308, SEQ ID NO:3310, SEQ ID NO:3313,
SEQ ID NO:3315, SEQ ID NO:3317, SEQ ID NO:3319, SEQ ID NO:3321, SEQ ID NO:3323,
SEQ ID NO:3325, SEQ ID NO:3327, SEQ ID NO:3329, SEQ ID NO:3331, SEQ ID NO:3333,
SEQ ID NO:3335, SEQ ID NO:3337, SEQ ID NO:3339, SEQ ID NO:nnnn , SEQ ID NO:3343,
SEQ ID NO:3345, SEQ ID NO:3347, SEQ ID NO:3349, SEQ ID NO:3351, SEQ ID NO:3353,
SEQ ID NO:3355, SEQ ID NO:3357, SEQ ID NO:3359, SEQ ID NO:3361, SEQ ID NO:3363,
SEQ ID NO:3365, SEQ ID NO:3367, SEQ ID NO:3369, SEQ ID NO:3371, SEQ ID NO:3373
SEQ ID NO:3375, SEQ ID NO:3377, SEQ ID NO:3379, SEQ ID NO:3381, SEQ ID NO:3383,
SEQ ID NO:3385, SEQ ID NO:3387, SEQ ID NO:3389, SEQ ID NO:3391, SEQ ID NO:3393,
SEQ ID NO:3395, SEQ ID NO:3397, SEQ ID NO:3399, SEQ ID NO:3401, SEQ ID NO:3403,
SEQ ID NO:3405, SEQ ID NO:nnnn , SEQ ID NO:3409, SEQ ID NO:3412, SEQ ID NO:3414,
SEQ ID NO:3416, SEQ ID NO:3418, SEQ ID NO:3420, SEQ ID NO:nnnn , SEQ ID NO:3424,
SEQ ID NO:3426, SEQ ID NO:3428, SEQ ID NO:3430, SEQ ID NO:3432, SEQ ID NO:3434,
SEQ ID NO:3436, SEQ ID NO:3438, SEQ ID NO:3440, SEQ ID NO:3442, SEQ ID NO:3444,
SEQ ID NO:3446, SEQ ID NO:3448, SEQ ID NO:3450, SEQ ID NO:3452, SEQ ID NO:3454,
SEQ ID NO:3456, SEQ ID NO:3458, SEQ ID NO:3460, SEQ ID NO:3462, SEQ ID NO:3464,
SEQ ID NO:3466, SEQ ID NO:3468, SEQ ID NO:3470, SEQ ID NO:3472, SEQ ID NO:3474,
SEQ ID NO:3476, SEQ ID NO:3478, SEQ ID NO:3480, SEQ ID NO:3483, SEQ ID NO:3485,
SEQ ID NO:3487, SEQ ID NO:3489, SEQ ID NO:3491, SEQ ID NO:3493, SEQ ID NO:3495,
SEQ ID NO:3497, SEQ ID NO:3499, SEQ ID NO:3501, SEQ ID NO:3503, SEQ ID NO:3505,
SEQ ID NO:3507, SEQ ID NO:3510, SEQ ID NO:3512, SEQ ID NO:3514, SEQ ID NO:3516,
SEQ ID NO:3518, SEQ ID NO:3520, SEQ ID NO:3522, SEQ ID NO:3524, SEQ ID NO:3526,
SEQ ID NO:3528, SEQ ID NO:3530, SEQ ID NO:3532, SEQ ID NO:3534, SEQ ID NO:3536,
SEQ ID NO:3538, SEQ ID NO:3540, SEQ ID NO:3542, SEQ ID NO:3544, SEQ ID NO:3546,
SEQ ID NO:3548, SEQ ID NO:3550, SEQ ID NO:3552, SEQ ID NO:3554, SEQ ID NO:3556,
SEQ ID NO:3558, SEQ ID NO:3560, SEQ ID NO:3562, SEQ ID NO:3564, SEQ ID NO:nnnn ,
SEQ ID NO:3568, SEQ ID NO:3570, SEQ ID NO:3572, SEQ ID NO:3574, SEQ ID NO:3576
SEQ ID NO:3578, SEQ ID NO:3580, SEQ ID NO:3582, SEQ ID NO:3584, SEQ ID NO:3586,
SEQ ID NO:3588, SEQ ID NO:3590
SEQ ID NO:3596, SEQ ID NO:3598, SEQ ID NO:3600, SEQ ID NO:3602, SEQ ID NO:3604,
SEQ ID NO:3606, SEQ ID NO:3608, SEQ ID NO:3614, SEQ ID NO:3616, SEQ ID NO:3618,
 
SEQ ID NO:3620, SEQ ID NO:3623, SEQ ID NO:3625, SEQ ID NO:3630, SEQ ID NO:3632,
SEQ ID NO:3635, SEQ ID NO:3637, SEQ ID NO:3639, SEQ ID NO:3641, SEQ ID NO:3643,
SEQ ID NO:3646, SEQ ID NO:3648, SEQ ID NO:3650, SEQ ID NO:3652, SEQ ID NO:3654,
SEQ ID NO:3656, SEQ ID NO:3659, SEQ ID NO:3661, SEQ ID NO:3663, SEQ ID NO:3665,
SEQ ID NO:3667, SEQ ID NO:3669, SEQ ID NO:3671, SEQ ID NO:3673, SEQ ID NO:3675,
SEQ ID NO:3677, SEQ ID NO:3679, SEQ ID NO:3681, SEQ ID NO:3684, SEQ ID NO:3687,
SEQ ID NO:3689, SEQ ID NO:3691, SEQ ID NO:3693, SEQ ID NO:3695, SEQ ID NO:3697,
SEQ ID NO:3699, SEQ ID NO:3701, SEQ ID NO:3703, SEQ ID NO:3705, SEQ ID NO:3707,
SEQ ID NO:3709, SEQ ID NO:3711, SEQ ID NO:3713, SEQ ID NO:3715, SEQ ID NO:3717,
SEQ ID NO:3719, SEQ ID NO:3721, SEQ ID NO:3723, SEQ ID NO:3725, SEQ ID NO:3727,
SEQ ID NO:3729, SEQ ID NO:3731, SEQ ID NO:3733, SEQ ID NO:3735, SEQ ID NO:3737,
SEQ ID NO:3739, SEQ ID NO:3742, SEQ ID NO:3744, SEQ ID NO:3746, SEQ ID NO:3748,
SEQ ID NO:3750, SEQ ID NO:3752, SEQ ID NO:3754, SEQ ID NO:3757, SEQ ID NO:3759,
SEQ ID NO:3762, SEQ ID NO:3764, SEQ ID NO:3766, SEQ ID NO:3768, SEQ ID NO:3770,
SEQ ID NO:3772, SEQ ID NO:3774, SEQ ID NO:3776, SEQ ID NO:3778, SEQ ID NO:3780,
SEQ ID NO:3782, SEQ ID NO:3784, SEQ ID NO:3786, SEQ ID NO:3788, SEQ ID NO:3790,
SEQ ID NO:3792, SEQ ID NO:3794, SEQ ID NO:3796, SEQ ID NO:3798, SEQ ID NO:3800,
SEQ ID NO:3802, SEQ ID NO:3804, SEQ ID NO:3807, SEQ ID NO:3809, SEQ ID NO:3811
SEQ ID NO:3813, SEQ ID NO:3815, SEQ ID NO:3817, SEQ ID NO:3819, SEQ ID NO:3821,
SEQ ID NO:3823, SEQ ID NO:3825, SEQ ID NO:3827, SEQ ID NO:3829, SEQ ID NO:3831,
SEQ ID NO:3833, SEQ ID NO:3835, SEQ ID NO:3837, SEQ ID NO:3839, SEQ ID NO:3841,
SEQ ID NO:3843, SEQ ID NO:3845, SEQ ID NO:3847, SEQ ID NO:3849, SEQ ID NO:3853,
SEQ ID NO:3855, SEQ ID NO:3857, SEQ ID NO:3859, SEQ ID NO:3861, SEQ ID NO:3863,
SEQ ID NO:3865, SEQ ID NO:3867, SEQ ID NO:3869, SEQ ID NO:3871, SEQ ID NO:3873,
SEQ ID NO:3875, SEQ ID NO:3877, SEQ ID NO:3879, SEQ ID NO:3881, SEQ ID NO:3883,
SEQ ID NO:3885, SEQ ID NO:3887, SEQ ID NO:3889, SEQ ID NO:3891, SEQ ID NO:3893,
SEQ ID NO:3895, SEQ ID NO:3897, SEQ ID NO:3899, SEQ ID NO:3901, SEQ ID NO:3903,
SEQ ID NO:3905, SEQ ID NO:3907, SEQ ID NO:3909, SEQ ID NO:3911, SEQ ID NO:3913,
SEQ ID NO:3915, SEQ ID NO:3917, SEQ ID NO:3919, SEQ ID NO:3921, SEQ ID NO:3923,
SEQ ID NO:3925, SEQ ID NO:3927, SEQ ID NO:3929, SEQ ID NO:3931, SEQ ID NO:3933,
SEQ ID NO:3935, SEQ ID NO:3937, SEQ ID NO:3939, SEQ ID NO:3941, SEQ ID NO:3943,
SEQ ID NO:3945, SEQ ID NO:3947, SEQ ID NO:3949, SEQ ID NO:3951, SEQ ID NO:3953,
SEQ ID NO:3955, SEQ ID NO:3957, SEQ ID NO:3959, SEQ ID NO:3961, SEQ ID NO:3963,
SEQ ID NO:3967, SEQ ID NO:3969, SEQ ID NO:3971, SEQ ID NO:3973, SEQ ID NO:3975,
SEQ ID NO:3978, SEQ ID NO:3980, SEQ ID NO:3982, SEQ ID NO:3984, SEQ ID NO:3986,
SEQ ID NO:3989, SEQ ID NO:3991, SEQ ID NO:3993, SEQ ID NO:3995, SEQ ID NO:3997,
SEQ ID NO:3999, SEQ ID NO:4002, SEQ ID NO:4005, SEQ ID NO:4007, SEQ ID NO:4009,
SEQ ID NO:4011, SEQ ID NO:4013, SEQ ID NO:4017, SEQ ID NO:4019, SEQ ID NO:4021
SEQ ID NO:4023, SEQ ID NO:4025, SEQ ID NO:4027, SEQ ID NO:4029, SEQ ID NO:4031,
SEQ ID NO:4033, SEQ ID NO:4035, SEQ ID NO:4037, SEQ ID NO:4039, SEQ ID NO:4041,
SEQ ID NO:4043, SEQ ID NO:4045, SEQ ID NO:4047, SEQ ID NO:4049, SEQ ID NO:4051,
SEQ ID NO:4053, SEQ ID NO:4055, SEQ ID NO:4057, SEQ ID NO:4059, SEQ ID NO:4061,
SEQ ID NO:4063, SEQ ID NO:4065, SEQ ID NO:4067, SEQ ID NO:4069, SEQ ID NO:4071,
SEQ ID NO:4073, SEQ ID NO:4075, SEQ ID NO:4077
SEQ ID NO:4079, SEQ ID NO:4081, SEQ ID NO:4083, SEQ ID NO:4085, SEQ ID NO:4087,
SEQ ID NO:4089, SEQ ID NO:4091, SEQ ID NO:4093, SEQ ID NO:4095, SEQ ID NO:4097,
SEQ ID NO:4100, SEQ ID NO:4102, SEQ ID NO:4104, SEQ ID NO:4106, SEQ ID NO:4108,
SEQ ID NO:4110, SEQ ID NO:4112, SEQ ID NO:4114, SEQ ID NO:4116, SEQ ID NO:4118,
SEQ ID NO:4120, SEQ ID NO:4122, SEQ ID NO:4124, SEQ ID NO:4126, SEQ ID NO:4128,
SEQ ID NO:4130, SEQ ID NO:4133, SEQ ID NO:4135, SEQ ID NO:4138, SEQ ID NO:4141,
SEQ ID NO:4143, SEQ ID NO:4145, SEQ ID NO:4147, SEQ ID NO:4149, SEQ ID NO:4151,
SEQ ID NO:4153, SEQ ID NO:4156, SEQ ID NO:4158, SEQ ID NO:4160, SEQ ID NO:4162,
 
SEQ ID NO:4164, SEQ ID NO:4166
SEQ ID NO:4169, SEQ ID NO:4171, SEQ ID NO:4173, SEQ ID NO:4175, SEQ ID NO:4177,
and SEQ ID NO:4179
(c) a polynucleotide comprising a nucleotide sequence encoding a protein comprising an amino acid sequence selected from the amino acid sequences of (b), in which one or more amino acids are substituted, deleted, inserted, and/or added, wherein said protein is functionally equivalent to the protein comprising said amino acid sequence selected from the amino acid sequences of (b);
(d) a polynucleotide that hybridizes with a polynucleotide comprising a nucleotide sequence selected from the nucleotide sequences of (a), and that comprises a nucleotide sequence encoding a protein functionally equivalent to the protein encoded by the nucleotide sequence selected from the nucleotide sequences of (a);
(e) a polynucleotide comprising a nucleotide sequence encoding a partial amino acid sequence of a protein encoded by the polynucleotide of (a) to (d);
(f) a polynucleotide comprising a nucleotide sequence with at least 70% identity to the nucleotide sequence of (a).

9. A substantially pure protein encoded by the polynucleotide of claim 8.

10. An antibody against the protein or peptide of any one of claims 6, 7, and 9.

11. A vector comprising the polynucleotide of claim 5 or 8.

12. A transformant carrying the polynucleotide of claim 5 or 8, or the vector of claim 11.

13. A transformant expressively carrying the polynucleotide of claim 5 or 8, or the vector of claim 11.

14. A method for producing the protein or peptide of any one of claims 6, 7, and 9, comprising culturing the transformant of claim 13 and recovering the expression product.

15. An oligonucleotide comprising the nucleotide sequence of claim 8 (a) or the nucleotide sequence complementary to the complementary strand thereof, wherein said oligonucleotide comprises 15 nucleotides or more.

16. Use of the oligonucleotide of claim 15 as a primer for synthesizing a polynucleotide.

17. Use of the oligonucleotide of claim 15 as a probe for detecting a gene.

18. An antisense polynucleotide against the polynucleotide of claim 8, or the portion thereof.

19. A method for synthesizing a polynucleotide, the method comprising:
a) synthesizing a complementary strand using a cDNA library as a template, and using the primer set of claim 2 or 3, or the primer of claim 16; and
b) recovering the synthesized product.

20. The method of claim 19, wherein the cDNA library is obtainable by oligo-capping method.

21. The method of claim 19, wherein the complementary strand is obtainable by PCR.

22. A method for detecting the polynucleotide of claim 8, the method comprising:
a) incubating a target polynucleotide with the oligonucleotide of claim 15 under the conditions where hybridization occurs, and
b) detecting the hybridization of the target polynucleotide with the oligonucleotide of claim 15.

23. A database of polynucleotides and/or proteins, the database comprising information on at least one sequence selected from the nucleotide sequences of claim 8 (a) and/or the amino acid sequences of claim 8 (b), or a medium on which the database is stored.
